(19) Europäisches Patentamt
European Patent Office
Office européen des brevets

(11) EP 1 672 070 A2

(12) # EUROPEAN PATENT APPLICATION

(43) Date of publication:
**21.06.2006 Bulletin 2006/25**

(21) Application number: **05025102.4**

(22) Date of filing: **01.12.2000**

(51) Int Cl.:
*C12N 15/12* (2006.01) *C07K 14/47* (2006.01)
*C07K 14/705* (2006.01) *G01N 33/53* (2006.01)
*C12N 15/62* (2006.01) *C07K 16/18* (2006.01)

(84) Designated Contracting States:
**AT BE CH CY DE DK ES FI FR GB GR IE IT LI LU MC NL PT SE TR**
Designated Extension States:
**AL LT LV MK RO SI**

(30) Priority:
**01.12.1999 WOPCT/US99/28301**
**01.12.1999 WOPCT/US99/28634**
**02.12.1999 WOPCT/US99/28551**
**02.12.1999 WOPCT/US99/28564**
**02.12.1999 WOPCT/US99/28565**
**09.12.1999 US 170262 P**
**16.12.1999 WOPCT/US99/30095**
**20.12.1999 WOPCT/US99/30911**
**20.12.1999 WOPCT/US99/30999**
**30.12.1999 WOPCT/US99/31243**
**30.12.1999 WOPCT/US99/31274**
**05.01.2000 WOPCT/US00/00219**
**06.01.2000 WOPCT/US00/00277**
**06.01.2000 WOPCT/US00/00376**
**11.02.2000 WOPCT/US00/03565**
**18.02.2000 WOPCT/US00/04341**
**18.02.2000 WOPCT/US00/04342**
**22.02.2000 WOPCT/US00/04414**
**24.02.2000 WOPCT/US00/04914**
**24.02.2000 US 5004**
**01.03.2000 WOPCT/US00/05601**
**02.03.2000 WOPCT/US00/05841**
**03.03.2000 US 187202 P**
**10.03.2000 WOPCT/US00/06319**
**15.03.2000 WOPCT/US00/06884**
**20.03.2000 WOPCT/US00/07377**
**21.03.2000 WOPCT/US00/07532**
**30.03.2000 WOPCT/US00/08439**
**17.05.2000 WOPCT/US00/13705**
**22.05.2000 WOPCT/US00/14042**
**30.05.2000 WOPCT/US00/14941**
**02.06.2000 WOPCT/US00/15264**
**05.06.2000 US 209832 P**
**28.07.2000 WOPCT/US00/20710**
**11.08.2000 WOPCT/US00/22031**
**23.08.2000 WOPCT/US00/23522**
**24.08.2000 WOPCT/US00/23328**
**08.11.2000 WOPCT/US00/30952**
**10.11.2000 WOPCT/US00/30873**

(62) Document number(s) of the earlier application(s) in accordance with Art. 76 EPC:
**00983846.7 / 1 250 426**

(71) Applicant: **GENENTECH, INC.**
**South San Francisco, CA 94080-4990 (US)**

(72) Inventors:
- **Baker, Kevin, P.** Darnestown MD 20878 (US)
- **Beresini, Maureen** Moss Beach CA 94038 (US)
- **Deforge, Laura** Pacifica CA 94044 (US)
- **Desnoyers, Luc** San Francisco CA 94133 (US)
- **Filvaroff, Ellen** San Francisco CA 94121 (US)
- **Gao, Wei-Qiang** Foster City CA 94404 (US)
- **Gerritsen, Mary, E.** San Mateo CA 94402 (US)
- **Goddard, Audrey** San Francisco CA 94131 (US)
- **Godowsky, Paul, J.** Burlingame CA 94010 (US)
- **Gurney, Austin, L.** Belmont CA 94002 (US)
- **Sherwood, Steven** Los Altos CA 94024 (US)
- **Smith, Victoria** Burlingame CA 94010 (US)
- **Stewart, Timothy, A.** San Francisco CA 94114 (US)

EP 1 672 070 A2

- **Tumas, Daniel**
  **Orinda**
  **CA 94563 (US)**
- **Wantanbe, Colin, K.**
  **Moraga**
  **CA 94556 (US)**
- **Wood, William, I.**
  **Cupertino, CA 95014 (US)**

(74) Representative: **Denison, Christopher Marcus et al**
**Mewburn Ellis LLP**
**York House**
**23 Kingsway**
**London WC2B 6HP (GB)**


Remarks:
- This application was filed on 17 - 11 - 2005 as a divisional application to the application mentioned under INID code 62.
- The sequence listing, which is published as annex to the application documents, was filed after the date of filing. The applicant has declared that it does not include matter which goes beyond the content of the application as filed.


(54) **Secreted and transmembrane polypeptides and nucleic acids encoding the same**

(57) The present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

Annex to the application documents - subsequently filed sequences listing

Sequence Listing

<110> Genentech, Inc.

<120> SECRETED AND TRANSMEMBRANE POLYPEPTIDES AND NUCLEIC ACIDS ENCODING THE SAME

<130> CMD/FP6262729

<140> EP05025102.4
<141> 2005-11-17

<150> EP00983846.7
<151> 2000-12-01

<150> PCT/US00/32678
<151> 2000-12-01

<150> PCT/US99/28301
<151> 1999-12-01

<150> PCT/US99/28634
<151> 1999-12-01

<150> PCT/US99/28551
<151> 1999-12-02

<150> PCT/US99/58564
<151> 1999-12-02

<150> PCT/US99/28565
<151> 1999-12-02

<150> 60/170,262
<151> 1999-12-09

<150> PCT/US99/30095
<151> 1999-12-16

<150> PCT/US99/30911
<151> 1999-12-20

<150> PCT/US99/30999
<151> 1999-12-20

<150> PCT/US99/31243
<151> 1999-12-30

<150> PCT/US99/31274
<151> 1999-12-30

<150> PCT/US00/00219
<151> 2000-01-05

<150> PCT/US00/00277
<151> 2000-01-06

<150> PCT/US00/00376
<151> 2000-01-06

<150> PCT/US00/03565
<151> 2000-02-11

<150> PCT/US00/04341

<151> 2000-02-18

<150> PCT/US00/04342
<151> 2000-02-18

<150> PCT/US00/04414
<151> 2000-02-22

<150> PCT/US00/04914
<151> 2000-02-24

<150> PCT/US00/05004
<151> 2000-02-24

<150> PCT/US00/05601
<151> 2000-03-01

<150> PCT/US00/05841
<151> 2000-03-02

<150> 60/187,202
<151> 2000-03-03

<150> PCT/US00/06319
<151> 2000-03-10

<150> PCT/US00/06884
<151> 2000-03-15

<150> PCT/US00/07377
<151> 2000-03-20

<150> PCT/US00/07532
<151> 2000-03-21

<150> PCT/US00/08439
<151> 2000-03-30

<150> PCT/US00/13705
<151> 2000-05-17

<150> PCT/US00/14042
<151> 2000-05-22

<150> PCT/US00/14941
<151> 2000-05-30

<150> PCT/US00/15264
<151> 2000-06-02

<150> 60/209,832
<151> 2000-06-05

<150> PCT/US00/20710
<151> 2000-07-28

<150> PCT/US00/22031
<151> 2000-08-11

<150> PCT/US00/23522
<151> 2000-08-23

<150> PCT/US00/23328
<151> 2000-08-24

<150> 60/000,000
<151> 2000-09-15

<150> PCT/US00/30952
<151> 2000-11-08

<150> PCT/US00/30873
<151> 2000-11-10

<160> 550

<210> 1
<211> 1264
<212> DNA
<213> Homo Sapien

<400> 1
gttactcggt ggtggcggag tctacggaag ccgttttcgc ttcacttttc 50

ctggctgtag agcgctttcc ccctggcggg tgagagtgca gagacgaagg 100

tgcgagatga gcactatgtt cgcggacact ctcctcatcg tttttatctc 150

tgtgtgcacg gctctgctcg cagagggcat aacctgggtc ctggtttaca 200

ggacagacaa gtacaagaga ctgaaggcag aagtggaaaa acagagtaaa 250

aaattggaaa agaagaagga aacaataaca gagtcagctg gtcgacaaca 300

gaaaaagaaa atagagagac aagaagagaa actgaagaat aacaacagag 350

atctatcaat ggttcgaatg aaatccatgt ttgctattgg cttttgtttt 400

actgccctaa tgggaatgtt caattccata tttgatggta gagtggtggc 450

aaagcttcct tttacccctc tttcttacat ccaaggactg tctcatcgaa 500

atctgctggg agatgacacc acagactgtt ccttcatttt cctgtatatt 550

ctctgtacta tgtcgattcg acagaacatt cagaagattc tcggccttgc 600

cccttcacga gccgccacca agcaggcagg tggatttctt ggcccaccac 650

ctccttctgg gaagttctct tgaactcaag aactctttat tttctatcat 700

tctttctaga cacacacaca tcagactggc aactgttttg tagcaagagc 750

cataggtagc cttactactt gggcctcttt ctagttttga attatttcta 800

agccttttgg gtatgattag agtgaaaatg gcagccagca aacttgatag 850

tgcttttggt cctagatgat ttttatcaaa taagtggatt gattagttaa 900

gttcaggtaa tgtttatgta atgaaaaaca aatagcatcc ttcttgtttc 950

atttacataa gtattttctg tgggaccgac tctcaaggca ctgtgtatgc 1000

cctgcaagtt ggctgtctat gagcatttag agatttagaa gaaaaattta 1050

gtttgtttaa cccttgtaac tgtttgtttt gttgttgttt ttttttcaag 1100

ccaaatacat gacataagat caataaagag gccaaatttt tagctgtttt 1150

atgtacaagg agagatctgt ttcattttgt tttgccgtat ttctagatat 1200

aagttttagc atgggccagg aaggactaaa ataaaagttt ttaaggtaca 1250

aaaaaaaaaa aaaa 1264

<210> 2
<211> 188
<212> PRT
<213> Homo Sapien

<400> 2
Met Ser Thr Met Phe Ala Asp Thr Leu Leu Ile Val Phe Ile Ser
1 5 10 15

Val Cys Thr Ala Leu Leu Ala Glu Gly Ile Thr Trp Val Leu Val
20 25 30

Tyr Arg Thr Asp Lys Tyr Lys Arg Leu Lys Ala Glu Val Glu Lys
35 40 45

Gln Ser Lys Lys Leu Glu Lys Lys Lys Glu Thr Ile Thr Glu Ser
50 55 60

Ala Gly Arg Gln Gln Lys Lys Lys Ile Glu Arg Gln Glu Glu Lys
65 70 75

Leu Lys Asn Asn Asn Arg Asp Leu Ser Met Val Arg Met Lys Ser
80 85 90

Met Phe Ala Ile Gly Phe Cys Phe Thr Ala Leu Met Gly Met Phe
95 100 105

Asn Ser Ile Phe Asp Gly Arg Val Val Ala Lys Leu Pro Phe Thr
110 115 120

Pro Leu Ser Tyr Ile Gln Gly Leu Ser His Arg Asn Leu Leu Gly
125 130 135

Asp Asp Thr Thr Asp Cys Ser Phe Ile Phe Leu Tyr Ile Leu Cys
140 145 150

Thr Met Ser Ile Arg Gln Asn Ile Gln Lys Ile Leu Gly Leu Ala
155 160 165

Pro Ser Arg Ala Ala Thr Lys Gln Ala Gly Gly Phe Leu Gly Pro
170 175 180

Pro Pro Pro Ser Gly Lys Phe Ser
185

<210> 3
<211> 1395
<212> DNA
<213> Homo Sapien

<400> 3
agccgggggc gggtttgaag acgcgtcgtt gggttttgga ggccgtgaaa 50

cagccgtttg agtttggctg cgggtggaga acgtttgtca ggggcccggc 100

caagaaggag gcccgcctgt tacgatggtg tccatgagtt tcaagcggaa 150

ccgcagtgac cggttctaca gcacccggtg ctgcggctgt tgccatgtcc 200

```
gcaccgggac gatcatcctg gggacctggt acatggtagt aaacctattg 250
atggcaattt tgctgactgt ggaagtgact catccaaact ccatgccagc 300
tgtcaacatt cagtatgaag tcatcggtaa ttactattcg tctgagagaa 350
tggctgataa tgcctgtgtt ctttttgccg tctctgttct tatgtttata 400
atcagttcaa tgctggttta tggagcaatt tcttatcaag tgggttggct 450
gattccattc ttctgttacc gactttttga cttcgtcctc agttgcctgg 500
ttgctattag ttctctcacc tatttgccaa gaatcaaaga atatctggat 550
caactacctg attttcccta caaagatgac ctcctggcct tggactccag 600
ctgcctcctg ttcattgttc ttgtgttctt tgccttattc atcattttta 650
aggcttatct aattaactgt gtttggaact gctataaata catcaacaac 700
cgaaacgtgc cggagattgc tgtgtaccct gcctttgaaa gcacctcctc 750
agtacgtttt gccaacctat gaaatggccg tgaaaatgcc tgaaaaagaa 800
ccaccacctc cttacttacc tgcctgaaga aattctgcct ttgacaataa 850
atcctatacc agctttttgt ttgtttatgt tacagaatgc tgcaattcag 900
ggctcttcaa acttgtttga tataaaatat gttgtctttt gtttaagcat 950
ttattttcaa acactaagga gctttttgac atctgttaaa cgtctttttg 1000
ttttttgtt aagtctttta cattttaata gtttttgaag acaatctagg 1050
ttaagcaaga gcaaagtgcc attgtttgcc tttaattggg gggtgggaag 1100
ggaaagaggg tacttgccac atagtttcct ttttaactgc actttcttta 1150
tataatcgtt tgcattttgt tacttgctac cctgagtact ttcaggaaga 1200
ctgacttaaa tattcggggt gagtaagtag ttgggtataa gatctgaact 1250
tttcatctgc agaggcaaga aaaatatttg acattgtgac ttgactgtgg 1300
aagatgatgg ttgcatgttt ctagtttgta tatgtttcca tctttgtgat 1350
aagatgattt aataaatctc tttaaatact aaaaaaaaaa aaaaa 1395
```

<210> 4
<211> 215
<212> PRT
<213> Homo Sapien

<400> 4

```
Met Val Ser Met Ser Phe Lys Arg Asn Arg Ser Asp Arg Phe Tyr
 1               5                  10                  15

Ser Thr Arg Cys Cys Gly Cys Cys His Val Arg Thr Gly Thr Ile
                20                  25                  30

Ile Leu Gly Thr Trp Tyr Met Val Val Asn Leu Leu Met Ala Ile
                35                  40                  45
```

```
Leu Leu Thr Val Glu Val Thr His Pro Asn Ser Met Pro Ala Val
                50                  55                  60

Asn Ile Gln Tyr Glu Val Ile Gly Asn Tyr Tyr Ser Ser Glu Arg
                65                  70                  75

Met Ala Asp Asn Ala Cys Val Leu Phe Ala Val Ser Val Leu Met
                80                  85                  90

Phe Ile Ile Ser Ser Met Leu Val Tyr Gly Ala Ile Ser Tyr Gln
                95                  100                 105

Val Gly Trp Leu Ile Pro Phe Phe Cys Tyr Arg Leu Phe Asp Phe
                110                 115                 120

Val Leu Ser Cys Leu Val Ala Ile Ser Ser Leu Thr Tyr Leu Pro
                125                 130                 135

Arg Ile Lys Glu Tyr Leu Asp Gln Leu Pro Asp Phe Pro Tyr Lys
                140                 145                 150

Asp Asp Leu Leu Ala Leu Asp Ser Ser Cys Leu Leu Phe Ile Val
                155                 160                 165

Leu Val Phe Phe Ala Leu Phe Ile Ile Phe Lys Ala Tyr Leu Ile
                170                 175                 180

Asn Cys Val Trp Asn Cys Tyr Lys Tyr Ile Asn Asn Arg Asn Val
                185                 190                 195

Pro Glu Ile Ala Val Tyr Pro Ala Phe Glu Ser Thr Ser Ser Val
                200                 205                 210

Arg Phe Ala Asn Leu
                215
```

<210> 5
<211> 1760
<212> DNA
<213> Homo Sapien

<400> 5

```
cccgctggcc cgtcagtgct ctccccgtcg tttgccctct ccagttcccc 50

cagtgcctgc cctacgcacc ccgatggcgg agctgcggcc tagcggcgcc 100

cccggcccca ccgcgccccc ggcccctggc ccgactgccc ccccggcctt 150

cgcttcgctc tttcccccgg gactgcacgc catctacgga gagtgccgcc 200

gcctttaccc tgaccagccg aacccgctcc aggttaccgc tatcgtcaag 250

tactggttgg gtggcccaga cccccttggac tatgttagca tgtacaggaa 300

tgtggggagc ccttctgcta acatccccga gcactggcac tacatcagct 350

tcggcctgag tgatctctat ggtgacaaca gagtccatga gtttacagga 400

acagatggac ctagtggttt tggctttgag ttgacctttc gtctgaagag 450

agaaactggg gagtctgccc caccaacatg gcccgcagag ttaatgcagg 500

gcttggcacg atacgtgttc cagtcagaga acaccttctg cagtggggac 550
```

catgtgtcct ggcacagccc tttggataac agtgagtcaa gaattcagca 600

catgctgctg acagaggacc cacagatgca gcccgtgcag acaccctttg 650

gggtagttac cttcctccag atcgttggtg tctgcactga agagctacac 700

tcagcccagc agtggaacgg gcagggcatc ctggagctgc tgcggacagt 750

gcctattgct ggcggcccct ggctgataac tgacatgcgg aggggagaga 800

ccatatttga gatcgatcca cacctgcaag agagagttga caaaggcatc 850

gagacagatg gctccaacct gagtggtgtc agtgccaagt gtgcctggga 900

tgacctgagc cggccccccg aggatgacga ggacagccgg agcatctgca 950

tcggcacaca gccccggcga ctctctggca aagacacaga gcagatccgg 1000

gagaccctga ggagaggact cgagatcaac agcaaacctg tccttccacc 1050

aatcaaccct cagcggcaga atggcctcgc ccacgaccgg gccccgagcc 1100

gcaaagacag cctggaaagt gacagctcca cggccatcat tccccatgag 1150

ctgattcgca cgcggcagct tgagagcgta catctgaaat tcaaccagga 1200

gtccggagcc ctcattcctc tctgcctaag ggcaggctc ctgcatggac 1250

ggcactttac atataaaagt atcacaggtg acatggccat cacgtttgtc 1300

tccacgggag tggaaggcgc ctttgccact gaggagcatc cttacgcggc 1350

tcatggaccc tggttacaac tctgaaccta tcctcggagc tctgccctcc 1400

cgtcctggaa cgtctttctg ccctgaggag agggtagtca gcatctccaa 1450

ttttcagcag ctcaagaacc ttggccccca caggacttcg cagatgtcac 1500

attgcccctc agtcccctga atgcccttcg gacccaaccc caattcccca 1550

agcccctgac cccctagctg ccggggttcc cactcccagt gccacaaccc 1600

cctcacctcc cctggcagcc cctcagcgag cctgaggccc agcacccgct 1650

ggctccccag cacatggtcc cctcccatgg gctgttgccc agggaaccgg 1700

ggcgcggtgg gaacgagctg ctggcctcgg catgtttcaa taaagttgct 1750

gtgctgggag 1760

<210> 6
<211> 433
<212> PRT
<213> Homo Sapien

<400> 6

```
Met Ala Glu Leu Arg Pro Ser Gly Ala Pro Gly Pro Thr Ala Pro
  1               5                  10                  15

Pro Ala Pro Gly Pro Thr Ala Pro Pro Ala Phe Ala Ser Leu Phe
                 20                  25                  30

Pro Pro Gly Leu His Ala Ile Tyr Gly Glu Cys Arg Arg Leu Tyr
                 35                  40                  45
```

```
Pro Asp Gln Pro Asn Pro Leu Gln Val Thr Ala Ile Val Lys Tyr
                50                  55                  60

Trp Leu Gly Gly Pro Asp Pro Leu Asp Tyr Val Ser Met Tyr Arg
                65                  70                  75

Asn Val Gly Ser Pro Ser Ala Asn Ile Pro Glu His Trp His Tyr
                80                  85                  90

Ile Ser Phe Gly Leu Ser Asp Leu Tyr Gly Asp Asn Arg Val His
                95                 100                 105

Glu Phe Thr Gly Thr Asp Gly Pro Ser Gly Phe Gly Phe Glu Leu
               110                 115                 120

Thr Phe Arg Leu Lys Arg Glu Thr Gly Glu Ser Ala Pro Pro Thr
               125                 130                 135

Trp Pro Ala Glu Leu Met Gln Gly Leu Ala Arg Tyr Val Phe Gln
               140                 145                 150

Ser Glu Asn Thr Phe Cys Ser Gly Asp His Val Ser Trp His Ser
               155                 160                 165

Pro Leu Asp Asn Ser Glu Ser Arg Ile Gln His Met Leu Leu Thr
               170                 175                 180

Glu Asp Pro Gln Met Gln Pro Val Gln Thr Pro Phe Gly Val Val
               185                 190                 195

Thr Phe Leu Gln Ile Val Gly Val Cys Thr Glu Glu Leu His Ser
               200                 205                 210

Ala Gln Gln Trp Asn Gly Gln Gly Ile Leu Glu Leu Leu Arg Thr
               215                 220                 225

Val Pro Ile Ala Gly Gly Pro Trp Leu Ile Thr Asp Met Arg Arg
               230                 235                 240

Gly Glu Thr Ile Phe Glu Ile Asp Pro His Leu Gln Glu Arg Val
               245                 250                 255

Asp Lys Gly Ile Glu Thr Asp Gly Ser Asn Leu Ser Gly Val Ser
               260                 265                 270

Ala Lys Cys Ala Trp Asp Asp Leu Ser Arg Pro Pro Glu Asp Asp
               275                 280                 285

Glu Asp Ser Arg Ser Ile Cys Ile Gly Thr Gln Pro Arg Arg Leu
               290                 295                 300

Ser Gly Lys Asp Thr Glu Gln Ile Arg Glu Thr Leu Arg Arg Gly
               305                 310                 315

Leu Glu Ile Asn Ser Lys Pro Val Leu Pro Pro Ile Asn Pro Gln
               320                 325                 330

Arg Gln Asn Gly Leu Ala His Asp Arg Ala Pro Ser Arg Lys Asp
               335                 340                 345

Ser Leu Glu Ser Asp Ser Ser Thr Ala Ile Ile Pro His Glu Leu
               350                 355                 360
```

```
Ile Arg Thr Arg Gln Leu Glu Ser Val His Leu Lys Phe Asn Gln
                365                 370                 375

Glu Ser Gly Ala Leu Ile Pro Leu Cys Leu Arg Gly Arg Leu Leu
                380                 385                 390

His Gly Arg His Phe Thr Tyr Lys Ser Ile Thr Gly Asp Met Ala
                395                 400                 405

Ile Thr Phe Val Ser Thr Gly Val Glu Gly Ala Phe Ala Thr Glu
                410                 415                 420

Glu His Pro Tyr Ala Ala His Gly Pro Trp Leu Gln Leu
                425                 430
```

<210> 7
<211> 1730
<212> DNA
<213> Homo Sapien

<400> 7

```
cgcgaatgaa gtttgcattt tcctctgttc ttgagcccag cttcttctcg 50
tctcccaccc cagcttcccg gcattggaag aagggaccgt cctcttcctt 100
gtcttggcca cccaaatcct ggtatcgaaa gggttgaacg gaccggaagt 150
gtgcagcagc gacgggtccc cagctaatcg acgccggaag tagcaattac 200
tagacaagca ttccgccgcc ggcttcgcta tggcggcaat tcccccagat 250
tcctggcagc cacccaacgt ttacttggag accagcatgg gaatcattgt 300
gctggagctg tactggaagc atgctccaaa gacctgtaag aactttgctg 350
agttggctcg tcgaggttac tacaatggca caaaattcca cagaattatc 400
aaagacttca tgatccaagg aggtgaccca acagggacag gtcgaggtgg 450
tgcatctatc tatggcaaac aatttgaaga tgaacttcat ccagacttga 500
aattcacggg ggctggaatt ctcgcaatgg ccaatgcggg gccagatacc 550
aatggcagcc agttctttgt gaccctcgcc cccacccagt ggcttgacgg 600
caaacacacc atttttggcc gagtgtgtca gggcatagga atggtgaatc 650
gcgtgggaat ggtagaaaca aactcccagg accgccctgt ggacgacgtg 700
aagatcatta aggcataccc ttctgggtag acttgctacc ctcttgagca 750
gctcttctga gatggcccca gtgaaccagc ttctagatga catagaatga 800
catgtaatgc taaatttcat tttggctttg caagtcatga agcttaggag 850
gcctggcatc ttgggtgagt tagagatgga agtacatttt aataggatgc 900
ttcttttctc ttcccccagt gcctaggttg ccagagcatt tgcacaaatg 950
cccctgttta tcaataggtg actacttact acacatgaac cataatgctg 1000
cttcttgtgc atgtctgctc tgatatacgt cgaacaatgt agcagccact 1050
gtcatttctc agtggttttg cctaaccaaa cttcttccta aggagattta 1100
```

tattctggcc tacacagcag tccttgatgg ctgacagcca cagaattcca 1150

aaccaagtag tgtctgtcag ccctcttaac tctgtgcacg ccctatttca 1200

gtcttttaca tttgttcttc tagggaatgt atgcatctct atatatattt 1250

tccctctcaa aaccagaaca tcaacagtgc tgtttctgac acttcagaca 1300

tcccacgcaa agccacattg aatttttgcc aaatgaaaaa cacatccaac 1350

aatcaagttt ctaagaaggt gtcaagtggg gaataataat aatgtataat 1400

aatcaagaaa ttagtttatt aaaaggaagc agaagcattg accatttttt 1450

cccagagaag aggagaaatc tgtagtgagc aaaggacaga ccatgaatcc 1500

tccttgagaa gtagtactct cagaaaggag aagcgccact caagttcttt 1550

taacccaaga ctttagagaa attaggtcca agatttttat atgttcagtt 1600

gtttatgtat aaaaataact ttctggattt tgtggggagg agcaggagag 1650

gaaggaagtt aatacctatg taatacatag aaacttccac aataaaatgc 1700

cattgatggt taaaaaaaaa aaaaaaaaaa 1730

<210> 8
<211> 166
<212> PRT
<213> Homo Sapien

<400> 8
Met Ala Ala Ile Pro Pro Asp Ser Trp Gln Pro Pro Asn Val Tyr
1 5 10 15

Leu Glu Thr Ser Met Gly Ile Ile Val Leu Glu Leu Tyr Trp Lys
20 25 30

His Ala Pro Lys Thr Cys Lys Asn Phe Ala Glu Leu Ala Arg Arg
35 40 45

Gly Tyr Tyr Asn Gly Thr Lys Phe His Arg Ile Ile Lys Asp Phe
50 55 60

Met Ile Gln Gly Gly Asp Pro Thr Gly Thr Gly Arg Gly Gly Ala
65 70 75

Ser Ile Tyr Gly Lys Gln Phe Glu Asp Glu Leu His Pro Asp Leu
80 85 90

Lys Phe Thr Gly Ala Gly Ile Leu Ala Met Ala Asn Ala Gly Pro
95 100 105

Asp Thr Asn Gly Ser Gln Phe Phe Val Thr Leu Ala Pro Thr Gln
110 115 120

Trp Leu Asp Gly Lys His Thr Ile Phe Gly Arg Val Cys Gln Gly
125 130 135

Ile Gly Met Val Asn Arg Val Gly Met Val Glu Thr Asn Ser Gln
140 145 150

Asp Arg Pro Val Asp Asp Val Lys Ile Ile Lys Ala Tyr Pro Ser

155 160 165

Gly

<210> 9
<211> 2276
<212> DNA
<213> Homo Sapien

<400> 9
cggacgcgtg ggcgcgcgcg agcgcagcgg tgggaggcgg cgaccagccg 50

gttgaggccc caggcttggc ctcaccacaa tgtggcacga ggctcggaag 100

catgagcgga agcttcgagg catgatggtc gactacaaga agagggcgga 150

gcggagacgg gagtattatg aaaagatcaa gaaggaccca gcccagttcc 200

tgcaggtaca tggccgagct tgcaaggtgc acctggattc tgcagtcgcc 250

ctggccgctg agagccctgt taatatgatg ccctggcagg gggacaccaa 300

caacatgatt gaccgattcg atgtccgtgc ccacctggac cacatccccg 350

actacacccc ccctctgctc accaccatct ccccagaaca ggagtcggac 400

gaacggaagt gtaactacga gcgctacaga ggcctggtgc agaacgactt 450

tgccggcatc tcagaggagc agtgcctgta ccagatctac attgatgagt 500

tgtacggagg cctccagaga cccagcgaag atgagaagaa gaagctggca 550

gagaagaagg cttccatcgg ttatacctac gaggacagca cggtggccga 600

ggtagagaag gcggcagaaa agccagagga ggaggagtca gcggccgagg 650

aggagagcaa ctcggacgaa gatgaggtca tccccgacat cgacgtggag 700

gtggacgtgg atgaattgaa ccaggagcag gtggcagatc tcaacaaaca 750

ggccacgact tatggcatgg ccgacggtga cttcgtcagg atgctccgga 800

aagacaagga ggaggcagag gccatcaagc atgccaaggc tcttgaggag 850

gagaaggcca tgtactcggg acgccgctct cgacgccagc ggagagagtt 900

tcgggagaag cggctgaggg gtcgcaagat cagcccaccc agctatgccc 950

gccgagacag ccccacctat gacccctata agcggtcacc ctcggagtcc 1000

agctcagagt cccgctcccg ctcccgctcc ccgaccccgg gccgcgagga 1050

gaagatcacg ttcatcacca gttttggggg cagcgatgag gaggcagccg 1100

cagccgctgc tgccgcagca gcatcaggag tcaccacagg gaagcccccc 1150

gcacctcccc agcctggcgg ccccgccccg ggacgtaatg ccagcgcccg 1200

ccgccgctcc tcctcctcct cctcctcctc ttctgcctcg aggacctcca 1250

gctcccgctc cagctctcgc tccagctccc gctctcgccg tggtgggggc 1300

tactaccgtt ccggccgcca cgcccgctcc cggtcccgct cctggtcccg 1350

ctcccgctcc cgctcccggc gctattcccg gtcccgtagc cgtggccggc 1400

ggcactcagg tgggggctcc cgagacggac accggtactc ccgctcgccc 1450

gcccggcgtg gtggttacgg gccccggcgc agaagcagga gccgctccca 1500

ctcaggggac cgctacaggc ggggcggccg gggcctcagg caccacagca 1550

gtagccgcag ccgcagcagc tggtccctca gcccgtcccg cagtcgcagc 1600

ctgactcgca gccgcagcca tagccccagc cccagccaga gccgcagccg 1650

cagccgcagc cgcagccaga gcccctcgcc atcacccgca agagagaagc 1700

tgaccaggcc ggccgcgtcc cctgctgtgg gcgagaagct gaaaaagacc 1750

gaacctgccg ctggtaaaga gacaggagct gccaaagtca cccaagctga 1800

cgcctcagga gaagctgaaa ctgaggatgc agaaggcgct gaacaggcag 1850

ttcaaggcgg ataagaaggc ggcacaagaa aagatgatcc agcaggagca 1900

tgagcggcag gagcgggaag acgagcttcg agccatggcc cgcaagatcc 1950

gcatgaagga gcgggaacgc cgagagaagg agagagaaga gtgggaacgc 2000

cagtacagcc ggcagagccg ctcaccctcc ccccgataca gtcgagaata 2050

cagctcttct cgaaggcgct caaggtcccg atcccgaagc ccccattacc 2100

gacattaggc agaagagtgg ggggtgggga ggacaagggg gtgggtaagg 2150

ggctcaagct gtgatgctgc tggttttatc tctagtgaaa taaagtcaaa 2200

agttatttaa ttcccgtcaa aaaaaaaaaa aaaaaaaaaa aaaaaaaaaa 2250

aaaaaaaaaa aaaaaaaaaa aaaaaa 2276

<210> 10
<211> 594
<212> PRT
<213> Homo Sapien

<400> 10
Met Trp His Glu Ala Arg Lys His Glu Arg Lys Leu Arg Gly Met
1               5                   10                  15

Met Val Asp Tyr Lys Lys Arg Ala Glu Arg Arg Arg Glu Tyr Tyr
                20                  25                  30

Glu Lys Ile Lys Lys Asp Pro Ala Gln Phe Leu Gln Val His Gly
                35                  40                  45

Arg Ala Cys Lys Val His Leu Asp Ser Ala Val Ala Leu Ala Ala
                50                  55                  60

Glu Ser Pro Val Asn Met Met Pro Trp Gln Gly Asp Thr Asn Asn
                65                  70                  75

Met Ile Asp Arg Phe Asp Val Arg Ala His Leu Asp His Ile Pro
                80                  85                  90

Asp Tyr Thr Pro Pro Leu Leu Thr Thr Ile Ser Pro Glu Gln Glu

95 100 105

Ser Asp Glu Arg Lys Cys Asn Tyr Glu Arg Tyr Arg Gly Leu Val
110 115 120

Gln Asn Asp Phe Ala Gly Ile Ser Glu Glu Gln Cys Leu Tyr Gln
125 130 135

Ile Tyr Ile Asp Glu Leu Tyr Gly Gly Leu Gln Arg Pro Ser Glu
140 145 150

Asp Glu Lys Lys Lys Leu Ala Glu Lys Lys Ala Ser Ile Gly Tyr
155 160 165

Thr Tyr Glu Asp Ser Thr Val Ala Glu Val Glu Lys Ala Ala Glu
170 175 180

Lys Pro Glu Glu Glu Glu Ser Ala Ala Glu Glu Glu Ser Asn Ser
185 190 195

Asp Glu Asp Glu Val Ile Pro Asp Ile Asp Val Glu Val Asp Val
200 205 210

Asp Glu Leu Asn Gln Glu Gln Val Ala Asp Leu Asn Lys Gln Ala
215 220 225

Thr Thr Tyr Gly Met Ala Asp Gly Asp Phe Val Arg Met Leu Arg
230 235 240

Lys Asp Lys Glu Glu Ala Glu Ala Ile Lys His Ala Lys Ala Leu
245 250 255

Glu Glu Glu Lys Ala Met Tyr Ser Gly Arg Arg Ser Arg Arg Gln
260 265 270

Arg Arg Glu Phe Arg Glu Lys Arg Leu Arg Gly Arg Lys Ile Ser
275 280 285

Pro Pro Ser Tyr Ala Arg Arg Asp Ser Pro Thr Tyr Asp Pro Tyr
290 295 300

Lys Arg Ser Pro Ser Glu Ser Ser Ser Glu Ser Arg Ser Arg Ser
305 310 315

Arg Ser Pro Thr Pro Gly Arg Glu Glu Lys Ile Thr Phe Ile Thr
320 325 330

Ser Phe Gly Gly Ser Asp Glu Glu Ala Ala Ala Ala Ala Ala Ala
335 340 345

Ala Ala Ala Ser Gly Val Thr Thr Gly Lys Pro Pro Ala Pro Pro
350 355 360

Gln Pro Gly Gly Pro Ala Pro Gly Arg Asn Ala Ser Ala Arg Arg
365 370 375

Arg Ser Ser Ser Ser Ser Ser Ser Ser Ser Ala Ser Arg Thr Ser
380 385 390

Ser Ser Arg Ser Ser Ser Arg Ser Ser Ser Arg Ser Arg Arg Gly
395 400 405

Gly Gly Tyr Tyr Arg Ser Gly Arg His Ala Arg Ser Arg Ser Arg
410 415 420

```
Ser Trp Ser Arg Ser Arg Ser Arg Ser Arg Arg Tyr Ser Arg Ser
                425                 430                 435

Arg Ser Arg Gly Arg Arg His Ser Gly Gly Gly Ser Arg Asp Gly
                440                 445                 450

His Arg Tyr Ser Arg Ser Pro Ala Arg Arg Gly Gly Tyr Gly Pro
                455                 460                 465

Arg Arg Arg Ser Arg Ser Arg Ser His Ser Gly Asp Arg Tyr Arg
                470                 475                 480

Arg Gly Gly Arg Gly Leu Arg His His Ser Ser Ser Arg Ser Arg
                485                 490                 495

Ser Ser Trp Ser Leu Ser Pro Ser Arg Ser Arg Ser Leu Thr Arg
                500                 505                 510

Ser Arg Ser His Ser Pro Ser Pro Ser Gln Ser Arg Ser Arg Ser
                515                 520                 525

Arg Ser Arg Ser Gln Ser Pro Ser Pro Ser Pro Ala Arg Glu Lys
                530                 535                 540

Leu Thr Arg Pro Ala Ala Ser Pro Ala Val Gly Glu Lys Leu Lys
                545                 550                 555

Lys Thr Glu Pro Ala Ala Gly Lys Glu Thr Gly Ala Ala Lys Val
                560                 565                 570

Thr Gln Ala Asp Ala Ser Gly Glu Ala Glu Thr Glu Asp Ala Glu
                575                 580                 585

Gly Ala Glu Gln Ala Val Gln Gly Gly
                590
```

<210> 11
<211> 1547
<212> DNA
<213> Homo Sapien

<400> 11

```
ggtaggcgcg cccagacctg agacgggttg ggactgggct gcgtcacgcg 50

cgggctctaa gcgcccgggg ccccgcccag tggccggcac agccaatcgc 100

agcgcgggaa ggcggtgggg gcggggaagg ccgcctggaa acttaaatcc 150

cgaggcgggc gaacctgcac cagaccgcgg acgtctgtaa tctcagaggc 200

ttgtttgctg agggtgcctg cgcagctgcg acggctgctg gttttgaaac 250

atgaatcttt cgctcgtcct ggctgccttt tgcttgggaa tagcctccgc 300

tgttccaaaa tttgaccaaa atttggatac aaagtggtac cagtggaagg 350

caacacacag aagattatat ggcgcgaatg aagaaggatg gaggagagca 400

gtgtgggaaa agaatatgaa aatgattgaa ctgcacaatg gggaatacag 450

ccaagggaaa catggcttca caatggccat gaatgctttt ggtgacatga 500

ccaatgaaga attcaggcag atgatgggtt gctttcgaaa ccagaaattc 550
```

aggaagggga aagtgttccg tgagcctctg tttcttgatc ttcccaaatc 600

tgtggattgg agaaagaaag gctacgtgac gccagtgaag aatcagaaac 650

agtgtggttc ttgttgggct tttagtgcga ctggtgctct tgaaggacag 700

atgttccgga aaactgggaa acttgtctca ctgagcgagc agaatctggt 750

ggactgttcg cgtcctcaag gcaatcaggg ctgcaatggt ggcttcatgg 800

ctagggcctt ccagtatgtc aaggagaacg gaggcctgga ctctgaggaa 850

tcctatccat atgtagcagt ggatgaaatc tgtaagtaca gacctgagaa 900

ttctgttgct aatgacactg gcttcacagt ggtcgcacct ggaaaggaga 950

aggccctgat gaaagcagtc gcaactgtgg ggcccatctc cgttgctatg 1000

gatgcaggcc attcgtcctt ccagttctac aaatcaggca tttattttga 1050

accagactgc agcagcaaaa acctggatca tggtgttctg gtggttggct 1100

acggctttga aggagcaaat tcgaataaca gcaagtattg gctcgtcaaa 1150

aacagctggg gtccagaatg gggctcgaat ggctatgtaa aaatagccaa 1200

agacaagaac aaccactgtg gaatcgccac agcagccagc taccccaatg 1250

tgtgagctga tggatggtga ggaggaagga cttaaggaca gcatgtctgg 1300

ggaaatttta tcttgaaact gaccaaacgc ttattgtgta agataaacca 1350

gttgaatcat ggaggatcca agttgagatt ttaattctgt gacattttta 1400

caagggtaaa atgttaccac tactttaatt attgttatac acagctttat 1450

gatatcaaag actcattgct taattctaag acttttgaat ttcatttttt 1500

taaaaagatg tacaaaacag tttgaaataa attttaattc gtatata 1547

<210> 12
<211> 334
<212> PRT
<213> Homo Sapien

<400> 12

```
Met Asn Leu Ser Leu Val Leu Ala Ala Phe Cys Leu Gly Ile Ala
  1               5                  10                  15

Ser Ala Val Pro Lys Phe Asp Gln Asn Leu Asp Thr Lys Trp Tyr
                 20                  25                  30

Gln Trp Lys Ala Thr His Arg Arg Leu Tyr Gly Ala Asn Glu Glu
                 35                  40                  45

Gly Trp Arg Arg Ala Val Trp Glu Lys Asn Met Lys Met Ile Glu
                 50                  55                  60

Leu His Asn Gly Glu Tyr Ser Gln Gly Lys His Gly Phe Thr Met
                 65                  70                  75

Ala Met Asn Ala Phe Gly Asp Met Thr Asn Glu Glu Phe Arg Gln
                 80                  85                  90
```

Met Met Gly Cys Phe Arg Asn Gln Lys Phe Arg Lys Gly Lys Val
95 100 105

Phe Arg Glu Pro Leu Phe Leu Asp Leu Pro Lys Ser Val Asp Trp
110 115 120

Arg Lys Lys Gly Tyr Val Thr Pro Val Lys Asn Gln Lys Gln Cys
125 130 135

Gly Ser Cys Trp Ala Phe Ser Ala Thr Gly Ala Leu Glu Gly Gln
140 145 150

Met Phe Arg Lys Thr Gly Lys Leu Val Ser Leu Ser Glu Gln Asn
155 160 165

Leu Val Asp Cys Ser Arg Pro Gln Gly Asn Gln Gly Cys Asn Gly
170 175 180

Gly Phe Met Ala Arg Ala Phe Gln Tyr Val Lys Glu Asn Gly Gly
185 190 195

Leu Asp Ser Glu Glu Ser Tyr Pro Tyr Val Ala Val Asp Glu Ile
200 205 210

Cys Lys Tyr Arg Pro Glu Asn Ser Val Ala Asn Asp Thr Gly Phe
215 220 225

Thr Val Val Ala Pro Gly Lys Glu Lys Ala Leu Met Lys Ala Val
230 235 240

Ala Thr Val Gly Pro Ile Ser Val Ala Met Asp Ala Gly His Ser
245 250 255

Ser Phe Gln Phe Tyr Lys Ser Gly Ile Tyr Phe Glu Pro Asp Cys
260 265 270

Ser Ser Lys Asn Leu Asp His Gly Val Leu Val Val Gly Tyr Gly
275 280 285

Phe Glu Gly Ala Asn Ser Asn Asn Ser Lys Tyr Trp Leu Val Lys
290 295 300

Asn Ser Trp Gly Pro Glu Trp Gly Ser Asn Gly Tyr Val Lys Ile
305 310 315

Ala Lys Asp Lys Asn Asn His Cys Gly Ile Ala Thr Ala Ala Ser
320 325 330

Tyr Pro Asn Val

<210> 13
<211> 2762
<212> DNA
<213> Homo Sapien

<400> 13
ggcggcgtca tgtgatccgc ttccctgctc ctttaagcgt ccacaggcgg 50

cggagcggcc acaatcacag ctccgggcat tgggggaacc cgagccggct 100

gcgccggggg aatccgtgcg ggcgccttcc gtcccggtcc catcctcgcc 150

```
gcgctccagc acctctgaag ttttgcagcg cccagaaagg aggcgaggaa 200
ggagggagtg tgtgagagga gggagcaaaa agctcaccct aaaacattta 250
tttcaaggag aaaagaaaaa ggggggcgc aaaaatggct ggggcaatta 300
tagaaaacat gagcaccaag aagctgtgca ttgttggtgg gattctgctc 350
gtgttccaaa tcatcgcctt tctggtggga ggcttgattg ctccagggcc 400
cacaacggca gtgtcctaca tgtcggtgaa atgtgtggat gcccgtaaga 450
accatcacaa gacaaaatgg ttcgtgcctt ggggacccaa tcattgtgac 500
aagatccgag acattgaaga ggcaattcca agggaaattg aagccaatga 550
catcgtgttt tctgttcaca ttcccctccc ccacatggag atgagtcctt 600
ggttccaatt catgctgttt atcctgcagc tggacattgc cttcaagcta 650
aacaaccaaa tcagagaaaa tgcagaagtc tccatggacg tttccctggc 700
ttaccgtgat gacgcatttg ctgagtggac tgaaatggcc catgaaagag 750
taccacggaa actcaaatgc accttcacat ctcccaagac tccagagcat 800
gagggccgtt actatgaatg tgatgtcctt cctttcatgg aaattgggtc 850
tgtggcccat aagttttacc ttttaaacat ccggctgcct gtgaatgaga 900
agaagaaaat caatgtggga attggggaga taaaggatat ccggttggtg 950
gggatccacc aaaatggagg cttcaccaag gtgtggtttg ccatgaagac 1000
cttccttacg cccagcatct tcatcattat ggtgtggtat tggaggagga 1050
tcaccatgat gtcccgaccc ccagtgcttc tggaaaaagt catctttgcc 1100
cttgggattt ccatgacctt tatcaatatc ccagtggaat ggttttccat 1150
cgggtttgac tggacctgga tgctgctgtt tggtgacatc cgacagggca 1200
tcttctatgc gatgcttctg tccttctgga tcatcttctg tggcgagcac 1250
atgatggatc agcacgagcg gaaccacatc gcagggtatt ggaagcaagt 1300
cggacccatt gccgttggct ccttctgcct cttcatattt gacatgtgtg 1350
agagaggggt acaactcacg aatcccttct acagtatctg gactacagac 1400
attggaacag agctggccat ggccttcatc atcgtggctg gaatctgcct 1450
ctgcctctac ttcctgtttc tatgcttcat ggtatttcag gtgtttcgga 1500
acatcagtgg gaagcagtcc agcctgccag ctatgagcaa agtccggcgg 1550
ctacactatg aggggctaat tttaggttc aagttcctca tgcttatcac 1600
cttggcctgc gctgccatga ctgtcatctt cttcatcgtt agtcaggtaa 1650
cggaaggcca ttggaaatgg ggcggcgtca cagtccaagt gaacagtgcc 1700
tttttcacag gcatctatgg gatgtggaat ctgtatgtct ttgctctgat 1750
```

gttcttgtat gcaccatccc ataaaaacta tggagaagac cagtccaatg 1800

gcgatctggg tgtccatagt ggggaagaac tccagctcac caccactatc 1850

acccatgtgg acggacccac tgagatctac aagttgaccc gcaaggaggc 1900

ccaggagtag gaggctgcag cgcccggctg ggacggtctc tccatacccc 1950

agcccctcta actagagtgg ggagcatgcc agagagagct caatgtacaa 2000

atgaatgcct catggctctt agctgtggtt tcttggacca gcggcatgga 2050

catttgtcag tttgccttct gacggtagct tttggaggaa gattcctgca 2100

gccactaatg cattgtgtat gataacaaaa actctggtat gacacatttt 2150

ctgtgatcat tgttaattag tgacatagta acatctgtag cagctggtta 2200

gtaaacctca tgtgggggtg gggtgggggt gtattccttg ggggatggtt 2250

tgggccgaat ggggagtgga atatttgaca tttttcctgt tttaaattct 2300

aggatagatt ttaacatcct ttgcggtccc agtccaaggt aggctggtgt 2350

catagtcttc tcactcctaa tccatgacca ctgttttttt cctatttata 2400

tcaccaggta gcctactgag ttaatattta agttgtcaat agataagtgt 2450

ccctgttttg tggcataata taactgaatt tcatgagaag atttattcca 2500

ccaggggtat ttcagctttg aaaccaaatc tgtgtatcta atactaacca 2550

atctgttgga tgtggatttt aaaaaatgtt tgctaaacta cccaagtaag 2600

atttactgta ttaaatggcc ttcgggtctg aaaagctttt ttaacctctt 2650

gcttaaaatg cgttttattt tgataagata cttcaaatag cctccaaaag 2700

tgtagatcca atcacttaaa taaacctgta tgtatatgca aaaaaaaaaa 2750

aaaaaaaaaa aa 2762

<210> 14
<211> 541
<212> PRT
<213> Homo Sapien

<400> 14

Met Ala Gly Ala Ile Ile Glu Asn Met Ser Thr Lys Lys Leu Cys
1               5                   10                  15

Ile Val Gly Gly Ile Leu Leu Val Phe Gln Ile Ile Ala Phe Leu
                20                  25                  30

Val Gly Gly Leu Ile Ala Pro Gly Pro Thr Thr Ala Val Ser Tyr
                35                  40                  45

Met Ser Val Lys Cys Val Asp Ala Arg Lys Asn His His Lys Thr
                50                  55                  60

Lys Trp Phe Val Pro Trp Gly Pro Asn His Cys Asp Lys Ile Arg
                65                  70                  75

Asp Ile Glu Glu Ala Ile Pro Arg Glu Ile Glu Ala Asn Asp Ile

80 85 90

Val Phe Ser Val His Ile Pro Leu Pro His Met Glu Met Ser Pro
95 100 105

Trp Phe Gln Phe Met Leu Phe Ile Leu Gln Leu Asp Ile Ala Phe
110 115 120

Lys Leu Asn Asn Gln Ile Arg Glu Asn Ala Glu Val Ser Met Asp
125 130 135

Val Ser Leu Ala Tyr Arg Asp Asp Ala Phe Ala Glu Trp Thr Glu
140 145 150

Met Ala His Glu Arg Val Pro Arg Lys Leu Lys Cys Thr Phe Thr
155 160 165

Ser Pro Lys Thr Pro Glu His Glu Gly Arg Tyr Tyr Glu Cys Asp
170 175 180

Val Leu Pro Phe Met Glu Ile Gly Ser Val Ala His Lys Phe Tyr
185 190 195

Leu Leu Asn Ile Arg Leu Pro Val Asn Glu Lys Lys Lys Ile Asn
200 205 210

Val Gly Ile Gly Glu Ile Lys Asp Ile Arg Leu Val Gly Ile His
215 220 225

Gln Asn Gly Gly Phe Thr Lys Val Trp Phe Ala Met Lys Thr Phe
230 235 240

Leu Thr Pro Ser Ile Phe Ile Ile Met Val Trp Tyr Trp Arg Arg
245 250 255

Ile Thr Met Met Ser Arg Pro Pro Val Leu Leu Glu Lys Val Ile
260 265 270

Phe Ala Leu Gly Ile Ser Met Thr Phe Ile Asn Ile Pro Val Glu
275 280 285

Trp Phe Ser Ile Gly Phe Asp Trp Thr Trp Met Leu Leu Phe Gly
290 295 300

Asp Ile Arg Gln Gly Ile Phe Tyr Ala Met Leu Leu Ser Phe Trp
305 310 315

Ile Ile Phe Cys Gly Glu His Met Met Asp Gln His Glu Arg Asn
320 325 330

His Ile Ala Gly Tyr Trp Lys Gln Val Gly Pro Ile Ala Val Gly
335 340 345

Ser Phe Cys Leu Phe Ile Phe Asp Met Cys Glu Arg Gly Val Gln
350 355 360

Leu Thr Asn Pro Phe Tyr Ser Ile Trp Thr Thr Asp Ile Gly Thr
365 370 375

Glu Leu Ala Met Ala Phe Ile Ile Val Ala Gly Ile Cys Leu Cys
380 385 390

Leu Tyr Phe Leu Phe Leu Cys Phe Met Val Phe Gln Val Phe Arg
395 400 405

```
Asn Ile Ser Gly Lys Gln Ser Ser Leu Pro Ala Met Ser Lys Val
                410                 415                 420

Arg Arg Leu His Tyr Glu Gly Leu Ile Phe Arg Phe Lys Phe Leu
                425                 430                 435

Met Leu Ile Thr Leu Ala Cys Ala Ala Met Thr Val Ile Phe Phe
                440                 445                 450

Ile Val Ser Gln Val Thr Glu Gly His Trp Lys Trp Gly Gly Val
                455                 460                 465

Thr Val Gln Val Asn Ser Ala Phe Phe Thr Gly Ile Tyr Gly Met
                470                 475                 480

Trp Asn Leu Tyr Val Phe Ala Leu Met Phe Leu Tyr Ala Pro Ser
                485                 490                 495

His Lys Asn Tyr Gly Glu Asp Gln Ser Asn Gly Asp Leu Gly Val
                500                 505                 510

His Ser Gly Glu Glu Leu Gln Leu Thr Thr Thr Ile Thr His Val
                515                 520                 525

Asp Gly Pro Thr Glu Ile Tyr Lys Leu Thr Arg Lys Glu Ala Gln
                530                 535                 540

Glu
```

<210> 15
<211> 2956
<212> DNA
<213> Homo Sapien

<400> 15

```
gtgaggggaa cagctgatcc gtctgttggg aggacagata tctcaaggcc 50
aggatggaag aatcaccact aagccgggca ccatcccgtg gtggagtcaa 100
ctttctcaat gtagcccgga cctacatccc caacaccaag gtggaatgtc 150
actacaccct tcccccaggc accatgccca gtgccagtga ctggattggc 200
atcttcaagg tggaggctgc ctgtgttcgg gattaccaca catttgtgtg 250
gtcttccgtg cctgaaagta caactgatgg ttcccccatt cacaccagtg 300
tccagttcca agccagctac ctgcccaaac caggagctca gctctaccag 350
ttccgatatg tgaaccgcca gggccaggtg tgtgggcaga gcccccccttt 400
ccagttccga gagccaaggc ccatggatga actggtgacc ctggaggagg 450
ctgatggggg ctctgacatc ctgctggttg tccccaaggc aactgtgtta 500
cagaaccagc tcgatgagag ccagcaagaa cggaatgacc tgatgcagct 550
gaagctacag ctggagggac aggtgacaga gctgaggagc cgagtgcagg 600
agctcgagag ggctctggca actgccaggc aggagcacac ggagctgatg 650
gaacagtaca aggggatttc ccggtcccat ggggagatca cagaagagag 700
```

ggacatcctg agccggcaac agggagacca tgtggcacgc atcctggagc 750
tagaggatga catccagacc atcagtgaga aagtgctgac gaaggaagtg 800
gagctggaca ggcttagaga cacagtgaag gccctgactc gggaacaaga 850
gaagctcctt gggcaactga aagaagtaca agcagacaag gagcaaagtg 900
aggctgagct ccaagtggca caacaggaga accatcactt aaatttggac 950
ctgaaggagg cgaagagctg gcaagaggag cagagtgctc aggctcagcg 1000
actgaaagac aaggtggccc agatgaagga caccctaggc caggcccagc 1050
agcgggtggc cgagctggag cccttgaagg agcagcttcg aggggcccag 1100
gagcttgcag cctcaagcca gcagaaagcc acccttcttg ggaggagtt 1150
ggccagtgca gcagcagcca gggaccgcac catagccgaa ctacaccgca 1200
gccgcctgga agtggctgaa gttaacggca ggctggctga gctcggtttg 1250
cacttgaagg aagaaaaatg ccaatggagc aaggagcggg cagggctgct 1300
gcagagtgtg gaggcagaga aggacaagat cctgaagctg agtgcagaga 1350
tacttcgatt ggagaaggca gttcaggagg agaggaccca aaaccaagtg 1400
ttcaagactg agctggcccg ggagaaggat tctagcctgg tacagttgtc 1450
agaaagtaag cgggagctga cagagctgcg gtcagccctg cgtgtgctcc 1500
agaaggaaaa ggagcagtta caggaggaga aacaggaatt gctagagtac 1550
atgagaaagc tagaggcccg cctggagaag gtggcagatg agaagtggaa 1600
tgaggatgcc accacagagg atgaggaggc cgctgtgggg ctgagctgcc 1650
cggcagctct gacagactca gaggacgagt ccccagaaga catgaggctc 1700
ccaccctatg gcctttgtga gcgtggagac ccaggctcct ctcctgctgg 1750
gcctcgagag gcttctcccc ttgttgtcat cagccagccg gctcccattt 1800
ctcctcacct ctctgggcca gctgaggaca gtagctctga ctcggaggct 1850
gaagatgaga agtcagtcct gatggcagct gtgcagagtg gggtgagga 1900
ggccaactta ctgcttcctg aactgggcag tgccttctat gacatggcca 1950
gtggctttac agtgggtacc ctgtcagaaa ccagcactgg gggccctgcc 2000
acccccacat ggaaggagtg tcctatctgt aaggagcgct ttcctgctga 2050
gagtgacaag gatgccctgg aggaccacat ggatggacac ttctttttca 2100
gcacccagga ccccttcacc tttgagtgat cttactccct cgtacatgca 2150
caaatacaca ctcatgcaca cacacactca cacacatgca tacacttagg 2200
tttcatgccc attttctatc acactgggct ccatgatatt ctgttcccta 2250
agaactgctt ctgtgtgccc tgttttcatc ccaagatttc tcacttcatc 2300

ctctcctacc tggctctttt gtcccaggga ggggtcctgt tcggaagcag 2350

tggctgaatt tatcccctga aagtggtttt ggaggaaccg ggatggagga 2400

ggccttcccc tgtgggaata gaatcgtcca ctcctagccc tggttgcttc 2450

tgatacacag ccactgcaca cacacactca cactcacact cccttgtctg 2500

atgccccaaa gccaattcct ggggcaccct accctctctt atttggagtt 2550

tccgttggtt tacctgagtt ttctctgggg tctgcacaga ggcagcagca 2600

tggacatcat ggcctctcag gtccctttg gttctcagtt tcattggttc 2650

ctctttctgt tcccccattg acttctgtgc cccaccctag ccttttccat 2700

aaccttaggt attcagtttg gaggggtttt ttgtattttt gaggattcct 2750

gtattctgta tcctctcctc gcatctcctc acatggaaag aaataatgta 2800

tttgtgcctt ctgtgaggaa tggggggaac aagtggtccc aggtatcccc 2850

atttccaagg ccccctccc tctccaggtc cccccacagc aataaaagct 2900

tccccctgat atccatccct ttgtagtttg aacaaatata tttatatgat 2950

atgtaa 2956

<210> 16
<211> 691
<212> PRT
<213> Homo Sapien

<400> 16

```
Met Glu Glu Ser Pro Leu Ser Arg Ala Pro Ser Arg Gly Gly Val
1               5                   10                  15

Asn Phe Leu Asn Val Ala Arg Thr Tyr Ile Pro Asn Thr Lys Val
                20                  25                  30

Glu Cys His Tyr Thr Leu Pro Pro Gly Thr Met Pro Ser Ala Ser
                35                  40                  45

Asp Trp Ile Gly Ile Phe Lys Val Glu Ala Ala Cys Val Arg Asp
                50                  55                  60

Tyr His Thr Phe Val Trp Ser Ser Val Pro Glu Ser Thr Thr Asp
                65                  70                  75

Gly Ser Pro Ile His Thr Ser Val Gln Phe Gln Ala Ser Tyr Leu
                80                  85                  90

Pro Lys Pro Gly Ala Gln Leu Tyr Gln Phe Arg Tyr Val Asn Arg
                95                  100                 105

Gln Gly Gln Val Cys Gly Gln Ser Pro Pro Phe Gln Phe Arg Glu
                110                 115                 120

Pro Arg Pro Met Asp Glu Leu Val Thr Leu Glu Glu Ala Asp Gly
                125                 130                 135

Gly Ser Asp Ile Leu Leu Val Val Pro Lys Ala Thr Val Leu Gln
                140                 145                 150
```

```
Asn Gln Leu Asp Glu Ser Gln Gln Glu Arg Asn Asp Leu Met Gln
                155                 160                 165

Leu Lys Leu Gln Leu Glu Gly Gln Val Thr Glu Leu Arg Ser Arg
                170                 175                 180

Val Gln Glu Leu Glu Arg Ala Leu Ala Thr Ala Arg Gln Glu His
                185                 190                 195

Thr Glu Leu Met Glu Gln Tyr Lys Gly Ile Ser Arg Ser His Gly
                200                 205                 210

Glu Ile Thr Glu Glu Arg Asp Ile Leu Ser Arg Gln Gln Gly Asp
                215                 220                 225

His Val Ala Arg Ile Leu Glu Leu Glu Asp Asp Ile Gln Thr Ile
                230                 235                 240

Ser Glu Lys Val Leu Thr Lys Glu Val Glu Leu Asp Arg Leu Arg
                245                 250                 255

Asp Thr Val Lys Ala Leu Thr Arg Glu Gln Glu Lys Leu Leu Gly
                260                 265                 270

Gln Leu Lys Glu Val Gln Ala Asp Lys Glu Gln Ser Glu Ala Glu
                275                 280                 285

Leu Gln Val Ala Gln Gln Glu Asn His His Leu Asn Leu Asp Leu
                290                 295                 300

Lys Glu Ala Lys Ser Trp Gln Glu Glu Gln Ser Ala Gln Ala Gln
                305                 310                 315

Arg Leu Lys Asp Lys Val Ala Gln Met Lys Asp Thr Leu Gly Gln
                320                 325                 330

Ala Gln Gln Arg Val Ala Glu Leu Glu Pro Leu Lys Glu Gln Leu
                335                 340                 345

Arg Gly Ala Gln Glu Leu Ala Ala Ser Ser Gln Gln Lys Ala Thr
                350                 355                 360

Leu Leu Gly Glu Glu Leu Ala Ser Ala Ala Ala Ala Arg Asp Arg
                365                 370                 375

Thr Ile Ala Glu Leu His Arg Ser Arg Leu Glu Val Ala Glu Val
                380                 385                 390

Asn Gly Arg Leu Ala Glu Leu Gly Leu His Leu Lys Glu Glu Lys
                395                 400                 405

Cys Gln Trp Ser Lys Glu Arg Ala Gly Leu Leu Gln Ser Val Glu
                410                 415                 420

Ala Glu Lys Asp Lys Ile Leu Lys Leu Ser Ala Glu Ile Leu Arg
                425                 430                 435

Leu Glu Lys Ala Val Gln Glu Glu Arg Thr Gln Asn Gln Val Phe
                440                 445                 450

Lys Thr Glu Leu Ala Arg Glu Lys Asp Ser Ser Leu Val Gln Leu
                455                 460                 465
```

```
Ser Glu Ser Lys Arg Glu Leu Thr Glu Leu Arg Ser Ala Leu Arg
                470                 475                 480

Val Leu Gln Lys Glu Lys Glu Gln Leu Gln Glu Glu Lys Gln Glu
                485                 490                 495

Leu Leu Glu Tyr Met Arg Lys Leu Glu Ala Arg Leu Glu Lys Val
                500                 505                 510

Ala Asp Glu Lys Trp Asn Glu Asp Ala Thr Thr Glu Asp Glu Glu
                515                 520                 525

Ala Ala Val Gly Leu Ser Cys Pro Ala Ala Leu Thr Asp Ser Glu
                530                 535                 540

Asp Glu Ser Pro Glu Asp Met Arg Leu Pro Pro Tyr Gly Leu Cys
                545                 550                 555

Glu Arg Gly Asp Pro Gly Ser Ser Pro Ala Gly Pro Arg Glu Ala
                560                 565                 570

Ser Pro Leu Val Val Ile Ser Gln Pro Ala Pro Ile Ser Pro His
                575                 580                 585

Leu Ser Gly Pro Ala Glu Asp Ser Ser Ser Asp Ser Glu Ala Glu
                590                 595                 600

Asp Glu Lys Ser Val Leu Met Ala Ala Val Gln Ser Gly Gly Glu
                605                 610                 615

Glu Ala Asn Leu Leu Leu Pro Glu Leu Gly Ser Ala Phe Tyr Asp
                620                 625                 630

Met Ala Ser Gly Phe Thr Val Gly Thr Leu Ser Glu Thr Ser Thr
                635                 640                 645

Gly Gly Pro Ala Thr Pro Thr Trp Lys Glu Cys Pro Ile Cys Lys
                650                 655                 660

Glu Arg Phe Pro Ala Glu Ser Asp Lys Asp Ala Leu Glu Asp His
                665                 670                 675

Met Asp Gly His Phe Phe Phe Ser Thr Gln Asp Pro Phe Thr Phe
                680                 685                 690

Glu
```

<210> 17
<211> 1528
<212> DNA
<213> Homo Sapien

<400> 17

```
gcaagttggg aattttagac tgtcactgca catggacctc tgggaagacg 50

tctggcgaga gctaggccca ctggccctac agacggatct tgctggctca 100

cctgtccctg tggaggttcc cctgggaagg caagatgccc aacaacagca 150

ctgctctgtc attggccaat gttacctaca tcaccatgga aattttcatt 200

ggactctgcg ccatagtggg caacgtgctg gtcatctgcg tggtcaagct 250
```

gaaccccagc ctgcagacca ccaccttcta tttcattgtc tctctagccc 300

tggctgacat tgctgttggg gtgctggtca tgcctttggc cattgttgtc 350

agcctgggca tcacaatcca cttctacagc tgccttttta tgacttgcct 400

actgcttatc tttacccacg cctccatcat gtccttgctg gccatcgctg 450

tggaccgata cttgcgggtc aagcttaccg tcagattcag aattcctggg 500

ctccctgggt gcattctatc attccagttg aaagtttgct tccttccagt 550

catgtggctc ttcattctac tctccttggc tctcatttca gatgccatgg 600

tcatggatga aaaggtcaag agaagctttg tgctggacac ggcttctgcc 650

atctgcaact acaatgccca ctacaagaat caccccaaat actggtgccg 700

aggctatttc cgtgactact gcaacatcat cgccttctcc cctaacagca 750

ccaatcatgt ggccctgagg gacacaggga accagctcat tgtcactatg 800

tcctgcctga ccaaagagga cacgggctgg tactggtgtg gcatccagcg 850

ggactttgcc agggatgaca tggattttac agagctgatt gtaactgacg 900

acaaaggaac cctggccaat gacttttggt ctgggaaaga cctatcaggc 950

aacaaaacca gaagctgcaa ggctcccaaa gttgtccgca aggctgaccg 1000

ctccaggacg tccattctca tcatttgcat actgatcacg ggtttgggaa 1050

tcatctctgt aatcagtcat ttgaccaaaa ggaggagaag tcaaaggaat 1100

agaagggtag gcaacacttt gaagcccttc tcgcgtgtcc tgactccaaa 1150

ggaaatggct cctactgaac agatgtgact gaagattttt ttaatttagt 1200

tcataaagtg atgctacaac agaataatca ccatgacaac tggcccacac 1250

ctcagagact gattctgatc tcccaggaat tctgaaggac cctctatcct 1300

tgacaacaat catttgcagc caggtagcaa cggcggtagt cagaggagct 1350

atgatagacc acacccaagc aaggctgccc tcaaataaca tctcaagatc 1400

ttagttctta tgcattccat cagtcagaag tgaagaagag gtggagaatc 1450

tggattgggg accaggaaat cacttgtatt ttgttagcca ataaattcct 1500

agccagtgtt gaatgaaaaa aaaaaaaa 1528

<210> 18
<211> 347
<212> PRT
<213> Homo Sapien

<400> 18

```
Met Pro Asn Asn Ser Thr Ala Leu Ser Leu Ala Asn Val Thr Tyr
  1               5                  10                  15

Ile Thr Met Glu Ile Phe Ile Gly Leu Cys Ala Ile Val Gly Asn
                 20                  25                  30
```

```
Val Leu Val Ile Cys Val Val Lys Leu Asn Pro Ser Leu Gln Thr
                35                  40                  45

Thr Thr Phe Tyr Phe Ile Val Ser Leu Ala Leu Ala Asp Ile Ala
                50                  55                  60

Val Gly Val Leu Val Met Pro Leu Ala Ile Val Val Ser Leu Gly
                65                  70                  75

Ile Thr Ile His Phe Tyr Ser Cys Leu Phe Met Thr Cys Leu Leu
                80                  85                  90

Leu Ile Phe Thr His Ala Ser Ile Met Ser Leu Leu Ala Ile Ala
                95                  100                 105

Val Asp Arg Tyr Leu Arg Val Lys Leu Thr Val Arg Phe Arg Ile
                110                 115                 120

Pro Gly Leu Pro Gly Cys Ile Leu Ser Phe Gln Leu Lys Val Cys
                125                 130                 135

Phe Leu Pro Val Met Trp Leu Phe Ile Leu Leu Ser Leu Ala Leu
                140                 145                 150

Ile Ser Asp Ala Met Val Met Asp Glu Lys Val Lys Arg Ser Phe
                155                 160                 165

Val Leu Asp Thr Ala Ser Ala Ile Cys Asn Tyr Asn Ala His Tyr
                170                 175                 180

Lys Asn His Pro Lys Tyr Trp Cys Arg Gly Tyr Phe Arg Asp Tyr
                185                 190                 195

Cys Asn Ile Ile Ala Phe Ser Pro Asn Ser Thr Asn His Val Ala
                200                 205                 210

Leu Arg Asp Thr Gly Asn Gln Leu Ile Val Thr Met Ser Cys Leu
                215                 220                 225

Thr Lys Glu Asp Thr Gly Trp Tyr Trp Cys Gly Ile Gln Arg Asp
                230                 235                 240

Phe Ala Arg Asp Asp Met Asp Phe Thr Glu Leu Ile Val Thr Asp
                245                 250                 255

Asp Lys Gly Thr Leu Ala Asn Asp Phe Trp Ser Gly Lys Asp Leu
                260                 265                 270

Ser Gly Asn Lys Thr Arg Ser Cys Lys Ala Pro Lys Val Val Arg
                275                 280                 285

Lys Ala Asp Arg Ser Arg Thr Ser Ile Leu Ile Ile Cys Ile Leu
                290                 295                 300

Ile Thr Gly Leu Gly Ile Ile Ser Val Ile Ser His Leu Thr Lys
                305                 310                 315

Arg Arg Arg Ser Gln Arg Asn Arg Arg Val Gly Asn Thr Leu Lys
                320                 325                 330

Pro Phe Ser Arg Val Leu Thr Pro Lys Glu Met Ala Pro Thr Glu
                335                 340                 345

Gln Met
```

<210> 19
<211> 3906
<212> DNA
<213> Homo Sapien

<400> 19
ctcgggcgcg cacaggcagc tcggtttgcc ctgcgattga gctgcgggtc 50

gcggccggcg ccggcctctc caatggcaaa tgtgtgtggc tggaggcgag 100

cgcgaggctt tcggcaaagg cagtcgagtg tttgcagacc ggggcgagtc 150

ctgtgaaagc agataaaaga aaacatttat taacgtgtca ttacgagggg 200

agcgcccggc cggggctgtc gcactccccg cggaacattt ggctccctcc 250

agctccgaga gaggagaaga agaaagcgga aaagaggcag attcacgtcg 300

tttccagcca agtggacctg atcgatggcc ctcctgaatt tatcacgata 350

tttgatttat tagcgatgcc ccctggtttg tgtgttacgc acacacacgt 400

gcacacaagg ctctggctcg cttccctccc tcgtttccag ctcctgggcg 450

aatcccacat ctgtttcaac tctccgccga gggcgagcag gagcgagagt 500

gtgtcgaatc tgcgagtgaa gagggacgag ggaaaagaaa caaagccaca 550

gacgcaactt gagactcccg catcccaaaa gaagcaccag atcagcaaaa 600

aaagaagatg ggcccccga gcctcgtgct gtgcttgctg tccgcaactg 650

tgttctccct gctgggtgga agctcggcct tcctgtcgca ccaccgcctg 700

aaaggcaggt ttcagaggga ccgcaggaac atccgcccca acatcatcct 750

ggtgctgacg gacgaccagg atgtggagct gggttccatg caggtgatga 800

acaagacccg gcgcatcatg gagcagggcg gggcgcactt catcaacgcc 850

ttcgtgacca cacccatgtg ctgcccctca cgctcctcca tcctcactgg 900

caagtacgtc cacaaccaca acacctacac caacaatgag aactgctcct 950

cgccctcctg gcaggcacag cacgagagcc gcacctttgc cgtgtacctc 1000

aatagcactg gctaccggac agctttcttc gggaagtatc ttaatgaata 1050

caacggctcc tacgtgccac ccggctggaa ggagtgggtc ggactcctta 1100

aaaactcccg cttttataac tacacgctgt gtcggaacgg ggtgaaagag 1150

aagcacggct ccgactactc caaggattac ctcacagacc tcatcaccaa 1200

tgacagcgtg agcttcttcc gcacgtccaa gaagatgtac ccgcacaggc 1250

cagtcctcat ggtcatcagc catgcagccc cccacggccc tgaggattca 1300

gccccacaat attcacgcct cttcccaaac gcatctcagc acatcacgcc 1350

gagctacaac tacgcgccca acccggacaa acactggatc atgcgctaca 1400

cggggcccat gaagcccatc cacatggaat tcaccaacat gctccagcgg 1450
aagcgcttgc agaccctcat gtcggtggac gactccatgg agacgattta 1500
caacatgctg gttgagacgg gcgagctgga caacacgtac atcgtataca 1550
ccgccgacca cggttaccac atcggccagt ttggcctggt gaaagggaaa 1600
tccatgccat atgagtttga catcagggtc ccgttctacg tgaggggccc 1650
caacgtggaa gccggctgtc tgaatcccca catcgtcctc aacattgacc 1700
tggcccccac catcctggac attgcaggcc tggacatacc tgcggatatg 1750
gacgggaaat ccatcctcaa gctgctggac acggagcggc cggtgaatcg 1800
gtttcacttg aaaaagaaga tgagggtctg gcgggactcc ttcttggtgg 1850
agagaggcaa gctgctacac aagagagaca atgacaaggt ggacgcccag 1900
gaggagaact ttctgcccaa gtaccagcgt gtgaaggacc tgtgtcagcg 1950
tgctgagtac cagacggcgt gtgagcagct gggacagaag tggcagtgtg 2000
tggaggacgc cacggggaag ctgaagctgc ataagtgcaa gggccccatg 2050
cggctgggcg gcagcagagc cctctccaac ctcgtgccca agtactacgg 2100
gcagggcagc gaggcctgca cctgtgacag cggggactac aagctcagcc 2150
tggccggacg ccggaaaaaa ctcttcaaga agaagtacaa ggccagctat 2200
gtccgcagtc gctccatccg ctcagtggcc atcgaggtgg acggcagggt 2250
gtaccacgta ggcctgggtg atgccgccca gccccgaaac ctcaccaagc 2300
ggcactggcc aggggcccct gaggaccaag atgacaagga tggtggggac 2350
ttcagtggca ctggaggcct tcccgactac tcagccgcca accccattaa 2400
agtgacacat cggtgctaca tcctagagaa cgacacagtc cagtgtgacc 2450
tggacctgta caagtccctg caggcctgga aagaccacaa gctgcacatc 2500
gaccacgaga ttgaaaccct gcagaacaaa attaagaacc tgagggaagt 2550
ccgaggtcac ctgaagaaaa agcggccaga agaatgtgac tgtcacaaaa 2600
tcagctacca cacccagcac aaaggccgcc tcaagcacag aggctccagt 2650
ctgcatcctt tcaggaaggg cctgcaagag aaggacaagg tgtggctgtt 2700
gcgggagcag aagcgcaaga agaaactccg caagctgctc aagcgcctgc 2750
agaacaacga cacgtgcagc atgccaggcc tcacgtgctt cacccacgac 2800
aaccagcact ggcagacggc gcctttctgg acactggggc ctttctgtgc 2850
ctgcaccagc gccaacaata acacgtactg gtgcatgagg accatcaatg 2900
agactcacaa tttcctcttc tgtgaatttg caactggctt cctagagtac 2950
tttgatctca acacagaccc ctaccagctg atgaatgcag tgaacacact 3000

ggacagggat gtcctcaacc agctacacgt acagctcatg gagctgagga 3050

gctgcaaggg ttacaagcag tgtaaccccc ggactcgaaa catggacctg 3100

gatggaggaa gctatgagca atacaggcag tttcagcgtc gaaagtggcc 3150

agaaatgaag agaccttctt ccaaatcact gggacaactg tgggaaggct 3200

gggaaggtta agaaacaaca gaggtggacc tccaaaaaca tagaggcatc 3250

acctgactgc acaggcaatg aaaaaccatg tgggtgattt ccagcagacc 3300

tgtgctattg gccaggaggc ctgagaaagc aagcacgcac tctcagtcaa 3350

catgacagat tctggaggat aaccagcagg agcagagata acttcaggaa 3400

gtccattttt gcccctgctt ttgctttgga ttatacctca ccagctgcac 3450

aaaatgcatt ttttcgtatc aaaaagtcac cactaaccct cccccagaag 3500

ctcacaaagg aaaacggaga gagcgagcga gagagatttc cttggaaatt 3550

tctcccaagg gcgaaagtca ttggaatttt taaatcatag gggaaaagca 3600

gtcctgttct aaatcctctt attcttttgg tttgtcacaa agaaggaact 3650

aagaagcagg acagaggcaa cgtggagagg ctgaaaacag tgcagagacg 3700

tttgacaatg agtcagtagc acaaaagaga tgacatttac ctagcactat 3750

aaaccctggt tgcctctgaa gaaactgcct tcattgtata tatgtgacta 3800

tttacatgta atcaacatgg gaacttttag ggaacctaa taagaaatcc 3850

caattttcag gagtggtggt gtcaataaac gctctgtggc cagtgtaaaa 3900

gaaaaa 3906

<210> 20
<211> 867
<212> PRT
<213> Homo Sapien

<400> 20

```
Met Gly Pro Pro Ser Leu Val Leu Cys Leu Leu Ser Ala Thr Val
1               5                   10                  15

Phe Ser Leu Leu Gly Gly Ser Ser Ala Phe Leu Ser His His Arg
                20                  25                  30

Leu Lys Gly Arg Phe Gln Arg Asp Arg Arg Asn Ile Arg Pro Asn
                35                  40                  45

Ile Ile Leu Val Leu Thr Asp Asp Gln Asp Val Glu Leu Gly Ser
                50                  55                  60

Met Gln Val Met Asn Lys Thr Arg Arg Ile Met Glu Gln Gly Gly
                65                  70                  75

Ala His Phe Ile Asn Ala Phe Val Thr Thr Pro Met Cys Cys Pro
                80                  85                  90

Ser Arg Ser Ser Ile Leu Thr Gly Lys Tyr Val His Asn His Asn
                95                  100                 105
```

Thr Tyr Thr Asn Asn Glu Asn Cys Ser Ser Pro Ser Trp Gln Ala
110 115 120

Gln His Glu Ser Arg Thr Phe Ala Val Tyr Leu Asn Ser Thr Gly
125 130 135

Tyr Arg Thr Ala Phe Phe Gly Lys Tyr Leu Asn Glu Tyr Asn Gly
140 145 150

Ser Tyr Val Pro Pro Gly Trp Lys Glu Trp Val Gly Leu Leu Lys
155 160 165

Asn Ser Arg Phe Tyr Asn Tyr Thr Leu Cys Arg Asn Gly Val Lys
170 175 180

Glu Lys His Gly Ser Asp Tyr Ser Lys Asp Tyr Leu Thr Asp Leu
185 190 195

Ile Thr Asn Asp Ser Val Ser Phe Phe Arg Thr Ser Lys Lys Met
200 205 210

Tyr Pro His Arg Pro Val Leu Met Val Ile Ser His Ala Ala Pro
215 220 225

His Gly Pro Glu Asp Ser Ala Pro Gln Tyr Ser Arg Leu Phe Pro
230 235 240

Asn Ala Ser Gln His Ile Thr Pro Ser Tyr Asn Tyr Ala Pro Asn
245 250 255

Pro Asp Lys His Trp Ile Met Arg Tyr Thr Gly Pro Met Lys Pro
260 265 270

Ile His Met Glu Phe Thr Asn Met Leu Gln Arg Lys Arg Leu Gln
275 280 285

Thr Leu Met Ser Val Asp Asp Ser Met Glu Thr Ile Tyr Asn Met
290 295 300

Leu Val Glu Thr Gly Glu Leu Asp Asn Thr Tyr Ile Val Tyr Thr
305 310 315

Ala Asp His Gly Tyr His Ile Gly Gln Phe Gly Leu Val Lys Gly
320 325 330

Lys Ser Met Pro Tyr Glu Phe Asp Ile Arg Val Pro Phe Tyr Val
335 340 345

Arg Gly Pro Asn Val Glu Ala Gly Cys Leu Asn Pro His Ile Val
350 355 360

Leu Asn Ile Asp Leu Ala Pro Thr Ile Leu Asp Ile Ala Gly Leu
365 370 375

Asp Ile Pro Ala Asp Met Asp Gly Lys Ser Ile Leu Lys Leu Leu
380 385 390

Asp Thr Glu Arg Pro Val Asn Arg Phe His Leu Lys Lys Lys Met
395 400 405

Arg Val Trp Arg Asp Ser Phe Leu Val Glu Arg Gly Lys Leu Leu
410 415 420

His Lys Arg Asp Asn Asp Lys Val Asp Ala Gln Glu Glu Asn Phe
425 430 435

Leu Pro Lys Tyr Gln Arg Val Lys Asp Leu Cys Gln Arg Ala Glu
440 445 450

Tyr Gln Thr Ala Cys Glu Gln Leu Gly Gln Lys Trp Gln Cys Val
455 460 465

Glu Asp Ala Thr Gly Lys Leu Lys Leu His Lys Cys Lys Gly Pro
470 475 480

Met Arg Leu Gly Gly Ser Arg Ala Leu Ser Asn Leu Val Pro Lys
485 490 495

Tyr Tyr Gly Gln Gly Ser Glu Ala Cys Thr Cys Asp Ser Gly Asp
500 505 510

Tyr Lys Leu Ser Leu Ala Gly Arg Arg Lys Lys Leu Phe Lys Lys
515 520 525

Lys Tyr Lys Ala Ser Tyr Val Arg Ser Arg Ser Ile Arg Ser Val
530 535 540

Ala Ile Glu Val Asp Gly Arg Val Tyr His Val Gly Leu Gly Asp
545 550 555

Ala Ala Gln Pro Arg Asn Leu Thr Lys Arg His Trp Pro Gly Ala
560 565 570

Pro Glu Asp Gln Asp Asp Lys Asp Gly Gly Asp Phe Ser Gly Thr
575 580 585

Gly Gly Leu Pro Asp Tyr Ser Ala Ala Asn Pro Ile Lys Val Thr
590 595 600

His Arg Cys Tyr Ile Leu Glu Asn Asp Thr Val Gln Cys Asp Leu
605 610 615

Asp Leu Tyr Lys Ser Leu Gln Ala Trp Lys Asp His Lys Leu His
620 625 630

Ile Asp His Glu Ile Glu Thr Leu Gln Asn Lys Ile Lys Asn Leu
635 640 645

Arg Glu Val Arg Gly His Leu Lys Lys Lys Arg Pro Glu Glu Cys
650 655 660

Asp Cys His Lys Ile Ser Tyr His Thr Gln His Lys Gly Arg Leu
665 670 675

Lys His Arg Gly Ser Ser Leu His Pro Phe Arg Lys Gly Leu Gln
680 685 690

Glu Lys Asp Lys Val Trp Leu Leu Arg Glu Gln Lys Arg Lys Lys
695 700 705

Lys Leu Arg Lys Leu Leu Lys Arg Leu Gln Asn Asn Asp Thr Cys
710 715 720

Ser Met Pro Gly Leu Thr Cys Phe Thr His Asp Asn Gln His Trp
725 730 735

Gln Thr Ala Pro Phe Trp Thr Leu Gly Pro Phe Cys Ala Cys Thr

740 745 750

Ser Ala Asn Asn Asn Thr Tyr Trp Cys Met Arg Thr Ile Asn Glu
755 760 765

Thr His Asn Phe Leu Phe Cys Glu Phe Ala Thr Gly Phe Leu Glu
770 775 780

Tyr Phe Asp Leu Asn Thr Asp Pro Tyr Gln Leu Met Asn Ala Val
785 790 795

Asn Thr Leu Asp Arg Asp Val Leu Asn Gln Leu His Val Gln Leu
800 805 810

Met Glu Leu Arg Ser Cys Lys Gly Tyr Lys Gln Cys Asn Pro Arg
815 820 825

Thr Arg Asn Met Asp Leu Asp Gly Gly Ser Tyr Glu Gln Tyr Arg
830 835 840

Gln Phe Gln Arg Arg Lys Trp Pro Glu Met Lys Arg Pro Ser Ser
845 850 855

Lys Ser Leu Gly Gln Leu Trp Glu Gly Trp Glu Gly
860 865

<210> 21
<211> 1041
<212> DNA
<213> Homo Sapien

<400> 21
gggcgcgcga gagctgctag ggcggtttct ctgcctcggg cctgttgggc 50

agggccggct aaggtgcgcg tgctcgctgg ttctaaccct tctgttgggc 100

gtttctgctg agaggcggga ggcgctgaga gtctgtgcgg aggtccgtgg 150

acagactgct ttgctcgttg ttgctcttcg gaggcggcga tccccgaagg 200

cgagctgaaa tacggctgca ggctacaatt tgcagccgac gattatggaa 250

gacggaagcg ggagaggtgg cccaccctca tggagcgctt gtgctcggat 300

ggcttcgcat ttccccaata ccccattaaa ccgtatcatc tgaagaggat 350

ccacagagct gtcttacatg gtaatctaga gaaactgaag taccttctgc 400

tcacgtatta tgacgccaat aagagagaca ggaaggaaag gaccgcccta 450

catttggcct gtgccactgg ccaaccggaa atggtacatc tcctggtgtc 500

cagaagatgt gagcttaacc tctgcgaccg tgaagacagg acacctctga 550

tcaaggctgt acaactgagg caggaggctt gtgcaactct tctgctgcaa 600

aatggcgcca atccaaatat tacggatttc tttggaagga ctgctctgca 650

ctacgctgtg tataatgaag atacatccat gatagaaaaa cttctttcac 700

atggtacaaa tattgaagaa tgcagcaagg tataggtcaa ccaatgttat 750

tttcaaacta tctgaaatga atttatttta acattgacac atgtaagggt 800

caatttttca tatttggaag ctcaaacatt ccttgaatga aaatattttg 850

aaatgcctta actgtctaag attttacttt aaatattgga acttttaaag 900

aagcattata gggaacagcc ttttttcatg cacttatggt aaataactat 950

aaaaacaaat gaattacaat aaatttataa ttcatgacaa ctgaatttgg 1000

gaaaggtaat agttaagtgt ttttccacta aattactttt t 1041

<210> 22
<211> 151
<212> PRT
<213> Homo Sapien

<400> 22
Met Glu Arg Leu Cys Ser Asp Gly Phe Ala Phe Pro Gln Tyr Pro
1 5 10 15

Ile Lys Pro Tyr His Leu Lys Arg Ile His Arg Ala Val Leu His
20 25 30

Gly Asn Leu Glu Lys Leu Lys Tyr Leu Leu Leu Thr Tyr Tyr Asp
35 40 45

Ala Asn Lys Arg Asp Arg Lys Glu Arg Thr Ala Leu His Leu Ala
50 55 60

Cys Ala Thr Gly Gln Pro Glu Met Val His Leu Leu Val Ser Arg
65 70 75

Arg Cys Glu Leu Asn Leu Cys Asp Arg Glu Asp Arg Thr Pro Leu
80 85 90

Ile Lys Ala Val Gln Leu Arg Gln Glu Ala Cys Ala Thr Leu Leu
95 100 105

Leu Gln Asn Gly Ala Asn Pro Asn Ile Thr Asp Phe Phe Gly Arg
110 115 120

Thr Ala Leu His Tyr Ala Val Tyr Asn Glu Asp Thr Ser Met Ile
125 130 135

Glu Lys Leu Leu Ser His Gly Thr Asn Ile Glu Glu Cys Ser Lys
140 145 150

Val

<210> 23
<211> 1121
<212> DNA
<213> Homo Sapien

<400> 23
gaggcagaaa ggcagaaagg agaaaattca ggataactct cctgaggggt 50

gagccaagcc ctgccatgta gtgcacgcag gacatcaaca aacacagata 100

acaggaaatg atccattccc tgtggtcact tattctaaag gccccaacct 150

tcaaagttca agtagtgata tggatgactc cacagaaagg gagcagtcac 200

gccttacttc ttgccttaag aaaagagaag aaatgaaact gaaggagtgt 250

gtttccatcc tcccacggaa ggaaagcccc tctgtccgat cctccaaaga 300

cggaaagctg ctggctgcaa ccttgctgct ggcactgctg tcttgctgcc 350

tcacggtggt gtctttctac caggtggccg ccctgcaagg ggacctggcc 400

agcctccggg cagagctgca gggccaccac gcggagaagc tgccagcagg 450

agcaggagcc cccaaggccg gcctggagga agctccagct gtcaccgcgg 500

gactgaaaat ctttgaacca ccagctccag gagaaggcaa ctccagtcag 550

aacagcagaa ataagcgtgc cgttcagggt ccagaagaaa cagtcactca 600

agactgcttg caactgattg cagacagtga aacaccaact atacaaaaag 650

gatcttacac atttgttcca tggcttctca gctttaaaag gggaagtgcc 700

ctagaagaaa aagagaataa aatattggtc aaagaaactg gttacttttt 750

tatatatggt caggttttat atactgataa gacctacgcc atgggacatc 800

taattcagag gaagaaggtc catgtctttg gggatgaatt gagtctggtg 850

actttgtttc gatgtattca aaatatgcct gaaacactac ccaataattc 900

ctgctattca gctggcattg caaaactgga agaaggagat gaactccaac 950

ttgcaatacc aagagaaaat gcacaaatat cactggatgg agatgtcaca 1000

ttttttggtg cattgaaact gctgtgacct acttacacca tgtctgtagc 1050

tattttcctc cctttctctg tacctctaag aagaaagaat ctaactgaaa 1100

ataccaaaaa aaaaaaaaaa a 1121

<210> 24
<211> 285
<212> PRT
<213> Homo Sapien

<400> 24
Met Asp Asp Ser Thr Glu Arg Glu Gln Ser Arg Leu Thr Ser Cys
1 5 10 15

Leu Lys Lys Arg Glu Glu Met Lys Leu Lys Glu Cys Val Ser Ile
20 25 30

Leu Pro Arg Lys Glu Ser Pro Ser Val Arg Ser Ser Lys Asp Gly
35 40 45

Lys Leu Leu Ala Ala Thr Leu Leu Leu Ala Leu Leu Ser Cys Cys
50 55 60

Leu Thr Val Val Ser Phe Tyr Gln Val Ala Ala Leu Gln Gly Asp
65 70 75

Leu Ala Ser Leu Arg Ala Glu Leu Gln Gly His His Ala Glu Lys
80 85 90

Leu Pro Ala Gly Ala Gly Ala Pro Lys Ala Gly Leu Glu Glu Ala
95 100 105

```
Pro Ala Val Thr Ala Gly Leu Lys Ile Phe Glu Pro Pro Ala Pro
                110                 115                 120

Gly Glu Gly Asn Ser Ser Gln Asn Ser Arg Asn Lys Arg Ala Val
                125                 130                 135

Gln Gly Pro Glu Glu Thr Val Thr Gln Asp Cys Leu Gln Leu Ile
                140                 145                 150

Ala Asp Ser Glu Thr Pro Thr Ile Gln Lys Gly Ser Tyr Thr Phe
                155                 160                 165

Val Pro Trp Leu Leu Ser Phe Lys Arg Gly Ser Ala Leu Glu Glu
                170                 175                 180

Lys Glu Asn Lys Ile Leu Val Lys Glu Thr Gly Tyr Phe Phe Ile
                185                 190                 195

Tyr Gly Gln Val Leu Tyr Thr Asp Lys Thr Tyr Ala Met Gly His
                200                 205                 210

Leu Ile Gln Arg Lys Lys Val His Val Phe Gly Asp Glu Leu Ser
                215                 220                 225

Leu Val Thr Leu Phe Arg Cys Ile Gln Asn Met Pro Glu Thr Leu
                230                 235                 240

Pro Asn Asn Ser Cys Tyr Ser Ala Gly Ile Ala Lys Leu Glu Glu
                245                 250                 255

Gly Asp Glu Leu Gln Leu Ala Ile Pro Arg Glu Asn Ala Gln Ile
                260                 265                 270

Ser Leu Asp Gly Asp Val Thr Phe Phe Gly Ala Leu Lys Leu Leu
                275                 280                 285
```

<210> 25
<211> 2698
<212> DNA
<213> Homo Sapien

<400> 25

```
ctgcttggat acctccagtc cccaaactgt gttccaggag ttttcttggc 50

cgaagctgcc cgatgtttga gccttttctt cccagagaag aagatggact 100

gaaagctgcc agttggggac tttttgtgat cacggcgttg cagcgtttta 150

aaggaggtga tggggcttgc gctggcttgt cttcccaccc aagtgaagag 200

ttgatgttca ctggttatgc ttagacaatg tgcagtttgt gttaatttaa 250

aattttgggt gggatagggg cataggcttg tgaagggcag tccggatccg 300

gaggaactcg tctttgtccc tggtaggaga gacacccccca gtctatcctc 350

gatgccgtca gccttggcca tcttcacttg ccgcccgaac tcgcacccgt 400

ttcaggagcg tcatgtctac ctggacgagc ccatcaaaat cggccgctca 450

gtggcccgct gtcgaccagc gcagaataat gccacttttg attgcaaagt 500

gctatcaagg aaccacgctc tcgtctggtt tgatcacaag acgggcaagt 550
```

tttatcttca agacactaaa agtagtaatg gtacttttat aaatagccag 600
agattgagtc gaggctctga agaaagtcca ccatgtgaaa ttctttccgg 650
tgacattatc cagtttggag tagacgtgac agagaataca cggaaagtta 700
cccatgggtg tattgtttcc acaataaaac tttttctacc agatggtatg 750
gaagcccggc tccgctcaga tgtcatccat gcaccattac caagtcctgt 800
tgacaaagtt gctgctaaca ctccaagtat gtactctcag gaactattcc 850
agctttctca gtatctacag gaggccttac atcgggaaca aatgttggaa 900
cagaagttag ccacgcttca gcggctacta gccatcaccc aagaggcttc 950
agataccagt tggcaggctt taatagatga agatagactc ttatcacggt 1000
tagaagttat gggaaaccaa ttacaggcat gctccaaaaa tcaaacagaa 1050
gatagtttac gaaaggaact tatagcatta caagaggata aacataacta 1100
tgagacaaca gccaaagagt ccctgaggcg ggttcttcag gagaaaattg 1150
aagtggttag aaaactttca gaagttgagc gaagtctgag taatactgaa 1200
gatgaatgta cccatctgaa agaaatgaat gaaaggactc aggaagaatt 1250
aagagaatta gccaacaaat ataatggagc agttaatgag attaaagatt 1300
tatctgataa attaaaggta gcagagggaa aacaagagga aatccaacag 1350
aagggacagg ctgagaaaaa agaattacaa cataaaatag atgaaatgga 1400
agaaaaagaa caggagctcc aggcaaaaat agaagctttg caagctgata 1450
atgatttcac caatgaaagg ctaacagctt tacaagtacg gttagaacat 1500
cttcaggaga aaactcttaa agaatgcagc agcttggctg atcgtcgaag 1550
ggcatctaac caaagcggta gaagaaacaa agctttcaaa aggtttgttt 1600
tctgtttttc tatgtttttt gacagttctt ttggataatg aaggttagtg 1650
tatattttca aggttatagt attttaacca tcagtttact tcttatagct 1700
cacaaaatag caagccagta acagtatcag ataatatata aaataatcag 1750
acttctgttt taagaagggt atcgtaactg gaatgtgtct ttttaagtgg 1800
atgtatattt atggtttttt gaatgttagt acttgatata ggtttcttta 1850
ggtattaaag atttgttgca atctctgtca ttcccagcat taatttcagc 1900
tttgatctca aattttaatc aaacacaatg taagtcgttt gtgatacaac 1950
ttaagtgaaa catgcttgca cttctatttt gggggttaca gtacctttaa 2000
aatctcttat gatgtttaat atttccttaa tttttggcat ctcagtttga 2050
tttaaacaaa attaatgact tttgtgaatg tagaatcttc ttatatttta 2100
tgagtagtcc agtaattgcc caaagtagtt tattgtgtta attctgttac 2150

agttgtcaga gaagaaaagt gagttttaaa gcaccatatt gtcaagtcac 2200

ttttatacat agggaaatta ggcaaataaa tttggtggca tgtgtttatc 2250

atagtagaac tttcattaga ctataccagt ataaaattta aaactagatt 2300

cacagtcctt ttggccaatt aaaacattga gttacaaaag tttgagatac 2350

ttaattttag tacattctat tttattaaag taactggatt catttgactt 2400

ttttaaccat gtaagaggat ggtgttattt caaatatctc gtggtttcca 2450

ttctgaattt tgtgcacggc agatgccata tttggggaaa aaatgcatag 2500

aatatgcatc attaatattg ttttggcaaa caggcattga gtttcagaac 2550

agtgaactat ttttagtaca tatggcaatt tttttcacct tattaaagtg 2600

agatgagaac agaccttaaa atagctttta cctcaccatc caaataccta 2650

ttcagattag ttggttgaat agccagcact ttgaagtaga gccttagg 2698

<210> 26
<211> 296
<212> PRT
<213> Homo Sapien

<400> 26
Met Glu Ala Arg Leu Arg Ser Asp Val Ile His Ala Pro Leu Pro
1               5                   10                  15

Ser Pro Val Asp Lys Val Ala Ala Asn Thr Pro Ser Met Tyr Ser
                20                  25                  30

Gln Glu Leu Phe Gln Leu Ser Gln Tyr Leu Gln Glu Ala Leu His
                35                  40                  45

Arg Glu Gln Met Leu Glu Gln Lys Leu Ala Thr Leu Gln Arg Leu
                50                  55                  60

Leu Ala Ile Thr Gln Glu Ala Ser Asp Thr Ser Trp Gln Ala Leu
                65                  70                  75

Ile Asp Glu Asp Arg Leu Leu Ser Arg Leu Glu Val Met Gly Asn
                80                  85                  90

Gln Leu Gln Ala Cys Ser Lys Asn Gln Thr Glu Asp Ser Leu Arg
                95                  100                 105

Lys Glu Leu Ile Ala Leu Gln Glu Asp Lys His Asn Tyr Glu Thr
                110                 115                 120

Thr Ala Lys Glu Ser Leu Arg Arg Val Leu Gln Glu Lys Ile Glu
                125                 130                 135

Val Val Arg Lys Leu Ser Glu Val Glu Arg Ser Leu Ser Asn Thr
                140                 145                 150

Glu Asp Glu Cys Thr His Leu Lys Glu Met Asn Glu Arg Thr Gln
                155                 160                 165

Glu Glu Leu Arg Glu Leu Ala Asn Lys Tyr Asn Gly Ala Val Asn
                170                 175                 180

Glu Ile Lys Asp Leu Ser Asp Lys Leu Lys Val Ala Glu Gly Lys
185 190 195

Gln Glu Glu Ile Gln Gln Lys Gly Gln Ala Glu Lys Lys Glu Leu
200 205 210

Gln His Lys Ile Asp Glu Met Glu Glu Lys Glu Gln Glu Leu Gln
215 220 225

Ala Lys Ile Glu Ala Leu Gln Ala Asp Asn Asp Phe Thr Asn Glu
230 235 240

Arg Leu Thr Ala Leu Gln Val Arg Leu Glu His Leu Gln Glu Lys
245 250 255

Thr Leu Lys Glu Cys Ser Ser Leu Ala Asp Arg Arg Arg Ala Ser
260 265 270

Asn Gln Ser Gly Arg Arg Asn Lys Ala Phe Lys Arg Phe Val Phe
275 280 285

Cys Phe Ser Met Phe Phe Asp Ser Ser Phe Gly
290 295

<210> 27
<211> 2700
<212> DNA
<213> Homo Sapien

<400> 27
gaacctggcg ccgccggaac tgatcgcggc ctagtcccga cgcgtgtgtg 50

ctagtgagcc ggagccggcg acggcggcag tggcggcccg gcctgcagga 100

gcccgacggg gtctctgcca tgggggagtg acgcgcctgc acccgctgtt 150

ccgcggcagc ggcgagacat gaggagaccc cgcgacaggg gcagcggcgg 200

cggctcgtga gccccgggat ggaggagaaa tacggcgggg acgtgctggc 250

cggcccgggc ggcggcggcg gccttgggcc ggtggacgta cccagcgctc 300

gattaacaaa atatattgtg ttactatgtt tcactaaatt tttgaaggct 350

gtgggacttt tcgaatcata tgatctccta aaagctgttc acattgttca 400

gttcattttt atattaaaac ttgggactgc attttttatg gttttgtttc 450

aaaagccatt ttcttctggg aaaactatta ccaaacacca gtggatcaaa 500

atatttaaac atgcagttgc tgggtgtatt atttcactct tgtggttttt 550

tggcctcact ctttgtggac cactaaggac tttgctgcta tttgagcaca 600

gtgatattgt tgtcatttca ctactcagtg ttttgttcac cagttctgga 650

ggaggaccag caaagacaag gggagctgct tttttcatta ttgctgtgat 700

ctgtttattg ctttttgaca atgatgatct catggctaaa atggctgaac 750

accctgaagg acatcatgac agtgctctaa ctcatatgct ttacacagcc 800

attgccttct taggtgtggc agatcacaag ggtggagtat tattgctagt 850

```
actggctttg tgttgtaaag ttggttttca tacagcttcc agaaagctct 900
ctgtcgacgt tggtggagct aaacgtcttc aagctttatc tcatcttgtt 950
tctgtgcttc tcttgtgccc atgggtcatt gttctttctg tgacaactga 1000
gagtaaagtg gagtcttggt tttctctcat tatgcctttt gcaacggtta 1050
tctttttttgt catgatcctg gatttctacg tggattccat ttgttcagtc 1100
aaaatggaag tttccaaatg tgctcgttat ggatcctttc ccatttttat 1150
tagtgctctc ctttttggaa atttttggac acatccaata acagaccagc 1200
ttcgggctat gaacaaagca gcacaccagg agagcactga acacgtcctg 1250
tctggaggag tggtagtgag tgctatattc ttcattttgt ctgccaatat 1300
cttatcatct ccctctaaga gaggacaaaa aggtaccctt attggatatt 1350
ctcctgaagg aacacctctt tataacttca tgggtgatgc ttttcagcat 1400
agctctcaat cgatccctag gtttattaag gaatcactaa aacaaattct 1450
tgaggagagt gactctaggc agatctttta cttcttgtgc ttgaatctgc 1500
tttttacctt tgtggaatta ttctatggcg tgctgaccaa tagtctgggc 1550
ctgatctcgg atggattcca catgcttttt gactgctctg ctttagtcat 1600
gggacttttt gctgccctga tgagtaggtg gaaagccact cggattttct 1650
cctatgggta cggccgaata gaaattctgt ctggatttat taatggactt 1700
tttctaatag taatagcgtt ttttgtgttt atggagtcag tggctagatt 1750
gattgatcct ccagaattag acactcacat gttaacacca gtctcagttg 1800
gagggctgat agtaaacctt attggtatct gtgcctttag ccatgcccat 1850
agccatgccc atggagcttc tcaaggaagc tgtcactcat ctgatcacag 1900
ccattcacac catatgcatg gacacagtga ccatgggcat ggtcacagcc 1950
acggatctgc gggtggaggc atgaatgcta acatgagggg tgtatttcta 2000
catgttttgg cagatacact tggcagcatt ggtgtgatcg tatccacagt 2050
tcttatagag cagtttggat ggttcatcgc tgacccactc tgttctcttt 2100
ctactgctat attaatattt ctcagtgttg ttccactgat taaagatgcc 2150
tgccaggttc tactcctgag attgccacca gaatatgaaa aagaactaca 2200
tattgcttta gaaaagatac agaaaattga aggattaata tcataccgag 2250
accctcattt ttggcgtcat tctgctagta ttgtggcagg aacaattcat 2300
atacaggtga catctgatgt gctagaacaa agaatagtac agcaggttac 2350
aggaatactt aaagatgctg gagtaaacaa tttaacaatt caagtggaaa 2400
aggaggcata ctttcaacat atgtctggcc taagtactgg atttcatgat 2450
```

gttctggcta tgacaaaaca aatggaatcc atgaaatact gcaaagatgg 2500

tacttacatc atgtgagata actcaagaat tacccctgga gaataaacaa 2550

tgaagattaa atgactcagt atttgtaata ttgccagaag gataaaaatt 2600

acacattaac tgtacagaaa cagagttccc tactactgga tcaaggaatc 2650

tttcttgaag gaaatttaaa tacagaatga aacattaatg gtaaaaaaaa 2700

<210> 28
<211> 765
<212> PRT
<213> Homo Sapien

<400> 28
Met Glu Glu Lys Tyr Gly Gly Asp Val Leu Ala Gly Pro Gly Gly
1 5 10 15

Gly Gly Gly Leu Gly Pro Val Asp Val Pro Ser Ala Arg Leu Thr
20 25 30

Lys Tyr Ile Val Leu Leu Cys Phe Thr Lys Phe Leu Lys Ala Val
35 40 45

Gly Leu Phe Glu Ser Tyr Asp Leu Leu Lys Ala Val His Ile Val
50 55 60

Gln Phe Ile Phe Ile Leu Lys Leu Gly Thr Ala Phe Phe Met Val
65 70 75

Leu Phe Gln Lys Pro Phe Ser Ser Gly Lys Thr Ile Thr Lys His
80 85 90

Gln Trp Ile Lys Ile Phe Lys His Ala Val Ala Gly Cys Ile Ile
95 100 105

Ser Leu Leu Trp Phe Phe Gly Leu Thr Leu Cys Gly Pro Leu Arg
110 115 120

Thr Leu Leu Leu Phe Glu His Ser Asp Ile Val Val Ile Ser Leu
125 130 135

Leu Ser Val Leu Phe Thr Ser Ser Gly Gly Gly Pro Ala Lys Thr
140 145 150

Arg Gly Ala Ala Phe Phe Ile Ile Ala Val Ile Cys Leu Leu Leu
155 160 165

Phe Asp Asn Asp Asp Leu Met Ala Lys Met Ala Glu His Pro Glu
170 175 180

Gly His His Asp Ser Ala Leu Thr His Met Leu Tyr Thr Ala Ile
185 190 195

Ala Phe Leu Gly Val Ala Asp His Lys Gly Gly Val Leu Leu Leu
200 205 210

Val Leu Ala Leu Cys Cys Lys Val Gly Phe His Thr Ala Ser Arg
215 220 225

Lys Leu Ser Val Asp Val Gly Gly Ala Lys Arg Leu Gln Ala Leu
230 235 240

```
Ser His Leu Val Ser Val Leu Leu Leu Cys Pro Trp Val Ile Val
                245                 250                 255

Leu Ser Val Thr Thr Glu Ser Lys Val Glu Ser Trp Phe Ser Leu
                260                 265                 270

Ile Met Pro Phe Ala Thr Val Ile Phe Phe Val Met Ile Leu Asp
                275                 280                 285

Phe Tyr Val Asp Ser Ile Cys Ser Val Lys Met Glu Val Ser Lys
                290                 295                 300

Cys Ala Arg Tyr Gly Ser Phe Pro Ile Phe Ile Ser Ala Leu Leu
                305                 310                 315

Phe Gly Asn Phe Trp Thr His Pro Ile Thr Asp Gln Leu Arg Ala
                320                 325                 330

Met Asn Lys Ala Ala His Gln Glu Ser Thr Glu His Val Leu Ser
                335                 340                 345

Gly Gly Val Val Val Ser Ala Ile Phe Phe Ile Leu Ser Ala Asn
                350                 355                 360

Ile Leu Ser Ser Pro Ser Lys Arg Gly Gln Lys Gly Thr Leu Ile
                365                 370                 375

Gly Tyr Ser Pro Glu Gly Thr Pro Leu Tyr Asn Phe Met Gly Asp
                380                 385                 390

Ala Phe Gln His Ser Ser Gln Ser Ile Pro Arg Phe Ile Lys Glu
                395                 400                 405

Ser Leu Lys Gln Ile Leu Glu Glu Ser Asp Ser Arg Gln Ile Phe
                410                 415                 420

Tyr Phe Leu Cys Leu Asn Leu Leu Phe Thr Phe Val Glu Leu Phe
                425                 430                 435

Tyr Gly Val Leu Thr Asn Ser Leu Gly Leu Ile Ser Asp Gly Phe
                440                 445                 450

His Met Leu Phe Asp Cys Ser Ala Leu Val Met Gly Leu Phe Ala
                455                 460                 465

Ala Leu Met Ser Arg Trp Lys Ala Thr Arg Ile Phe Ser Tyr Gly
                470                 475                 480

Tyr Gly Arg Ile Glu Ile Leu Ser Gly Phe Ile Asn Gly Leu Phe
                485                 490                 495

Leu Ile Val Ile Ala Phe Phe Val Phe Met Glu Ser Val Ala Arg
                500                 505                 510

Leu Ile Asp Pro Pro Glu Leu Asp Thr His Met Leu Thr Pro Val
                515                 520                 525

Ser Val Gly Gly Leu Ile Val Asn Leu Ile Gly Ile Cys Ala Phe
                530                 535                 540

Ser His Ala His Ser His Ala His Gly Ala Ser Gln Gly Ser Cys
                545                 550                 555

His Ser Ser Asp His Ser His Ser His His Met His Gly His Ser
```

560 565 570

Asp His Gly His Gly His Ser His Gly Ser Ala Gly Gly Gly Met
575 580 585

Asn Ala Asn Met Arg Gly Val Phe Leu His Val Leu Ala Asp Thr
590 595 600

Leu Gly Ser Ile Gly Val Ile Val Ser Thr Val Leu Ile Glu Gln
605 610 615

Phe Gly Trp Phe Ile Ala Asp Pro Leu Cys Ser Leu Ser Thr Ala
620 625 630

Ile Leu Ile Phe Leu Ser Val Val Pro Leu Ile Lys Asp Ala Cys
635 640 645

Gln Val Leu Leu Leu Arg Leu Pro Pro Glu Tyr Glu Lys Glu Leu
650 655 660

His Ile Ala Leu Glu Lys Ile Gln Lys Ile Glu Gly Leu Ile Ser
665 670 675

Tyr Arg Asp Pro His Phe Trp Arg His Ser Ala Ser Ile Val Ala
680 685 690

Gly Thr Ile His Ile Gln Val Thr Ser Asp Val Leu Glu Gln Arg
695 700 705

Ile Val Gln Gln Val Thr Gly Ile Leu Lys Asp Ala Gly Val Asn
710 715 720

Asn Leu Thr Ile Gln Val Glu Lys Glu Ala Tyr Phe Gln His Met
725 730 735

Ser Gly Leu Ser Thr Gly Phe His Asp Val Leu Ala Met Thr Lys
740 745 750

Gln Met Glu Ser Met Lys Tyr Cys Lys Asp Gly Thr Tyr Ile Met
755 760 765

<210> 29
<211> 1701
<212> DNA
<213> Homo Sapien

<400> 29
ggcacgaggg caggatatta gaaatggcta ctccccagtc aattttcatc 50

tttgcaatct gcattttaat gataacagaa ttaattctgg cctcaaaaag 100

ctactatgat atcttaggtg tgccaaaatc ggcatcagag cgccaaatca 150

agaaggcctt tcacaagttg gccatgaagt accaccctga caaaaataag 200

agcccggatg ctgaagcaaa attcagagag attgcagaag catatgaaac 250

actctcagat gctaatagac gaaaagagta tgatacactt ggacacagtg 300

cttttactag tggtaaagga caaagaggta gtggaagttc ttttgagcag 350

tcatttaact tcaattttga tgacttattt aaagactttg gctttttttgg 400

tcaaaaccaa aacactggat ccaagaagcg ttttgaaaat catttccaga 450

cacgccagga tggtggttcc agtagacaaa ggcatcattt ccaagaattt 500

tctttttggag gtggattatt tgatgacatg tttgaagata tggagaaaat 550

gttttctttt agtggttttg actctaccaa tcagcataca gtacagactg 600

aaaatagatt tcatggatct agcaagcact gcaggactgt cactcaacga 650

agaggaaata tggttactac atacactgac tgttcaggac agtagttctt 700

attctattct cactaaatcc aactggttga ctcttcctca ttatctttga 750

tgctaaacaa ttttctgtga actattttga caagtgcatg atttcacttt 800

aaacaatttg atatagctat taaatatatt taagggtttt ttttttgac 850

aaattcaaca ttcaacgagt agacaaaatg ctaattattt ccctgattag 900

gaaagtttct ttaaaaaaca cgtaattttg cctagtgctt tttctctacc 950

tgcccttggg ctcactaata tcaccagtat tattaccaag aaaatattga 1000

gtttacctga ttaaacttta aaagttaatt gtagatttaa attgtgtgaa 1050

cctaatgatt tttgcagtga aacctttact aattcaaagt tgcatgttct 1100

atgacatctg tgacttgcgt tgcagagtgt acatgaaact gtataattga 1150

gtcattcagt aaaggagaac agtatcttgg ttaattgcta ctgaaaggtt 1200

gagaaaggaa tggtttgata tttaccacag cgctgtgcct ttctacagta 1250

gaactggggt aaaggaaatg gttttattgc ccatagtcat ttaggctgga 1300

aaaaagttga aaacttaacg aaatattgcc aagagattgt tatgtgtttg 1350

gttccagcct aaaaatgatt ttgtagtgtt gaaatcatag ctacttacat 1400

agctttttca tatttctttc ttagttgttg gcactcttag gtcttagtat 1450

ggatttatgt gtttgtgtgt gtgtagttta tcctctctct catctttatc 1500

tagagattga ctgatacctc attctgtttg taaaaccagc cagtaatttc 1550

tgtgcaacct tactatgtgc aatattttta aatcctgaga aatgtgtgct 1600

tttgttttcg gatagactta tttctttagt tctgcacttt tccacattat 1650

actccatatg agtattaatc ctatggatac atattaaaac aagtgtctca 1700

t 1701

<210> 30
<211> 223
<212> PRT
<213> Homo Sapien

<400> 30
Met Ala Thr Pro Gln Ser Ile Phe Ile Phe Ala Ile Cys Ile Leu
1 5 10 15

Met Ile Thr Glu Leu Ile Leu Ala Ser Lys Ser Tyr Tyr Asp Ile
20 25 30

Leu Gly Val Pro Lys Ser Ala Ser Glu Arg Gln Ile Lys Lys Ala
35 40 45

Phe His Lys Leu Ala Met Lys Tyr His Pro Asp Lys Asn Lys Ser
50 55 60

Pro Asp Ala Glu Ala Lys Phe Arg Glu Ile Ala Glu Ala Tyr Glu
65 70 75

Thr Leu Ser Asp Ala Asn Arg Arg Lys Glu Tyr Asp Thr Leu Gly
80 85 90

His Ser Ala Phe Thr Ser Gly Lys Gly Gln Arg Gly Ser Gly Ser
95 100 105

Ser Phe Glu Gln Ser Phe Asn Phe Asn Phe Asp Asp Leu Phe Lys
110 115 120

Asp Phe Gly Phe Phe Gly Gln Asn Gln Asn Thr Gly Ser Lys Lys
125 130 135

Arg Phe Glu Asn His Phe Gln Thr Arg Gln Asp Gly Gly Ser Ser
140 145 150

Arg Gln Arg His His Phe Gln Glu Phe Ser Phe Gly Gly Gly Leu
155 160 165

Phe Asp Asp Met Phe Glu Asp Met Glu Lys Met Phe Ser Phe Ser
170 175 180

Gly Phe Asp Ser Thr Asn Gln His Thr Val Gln Thr Glu Asn Arg
185 190 195

Phe His Gly Ser Ser Lys His Cys Arg Thr Val Thr Gln Arg Arg
200 205 210

Gly Asn Met Val Thr Thr Tyr Thr Asp Cys Ser Gly Gln
215 220

<210> 31
<211> 2056
<212> DNA
<213> Homo Sapien

<400> 31
aaagttacat tttctctgga actctcctag gccactccct gctgatgcaa 50

catctgggtt tgggcagaaa ggagggtgct tcggagcccg ccctttctga 100

gcttcctggg ccggctctag aacaattcag gcttcgctgc gactcagacc 150

tcagctccaa catatgcatt ctgaagaaag atggctgaga tggacagaat 200

gctttatttt ggaaagaaac aatgttctag gtcaaactga gtctaccaaa 250

tgcagacttt cacaatggtt ctagaagaaa tctggacaag tcttttcatg 300

tggtttttct acgcattgat tccatgtttg ctcacagatg aagtggccat 350

tctgcctgcc cctcagaacc tctctgtact ctcaaccaac atgaagcatc 400

tcttgatgtg gagcccagtg atcgcgcctg gagaaacagt gtactattct 450

gtcgaatacc agggggagta cgagagcctg tacacgagcc acatctggat 500
ccccagcagc tggtgctcac tcactgaagg tcctgagtgt gatgtcactg 550
atgacatcac ggccactgtg ccatacaacc ttcgtgtcag ggccacattg 600
ggctcacaga cctcagcctg gagcatcctg aagcatccct ttaatagaaa 650
ctcaaccatc cttacccgac ctgggatgga gatcaccaaa gatggcttcc 700
acctggttat tgagctggag gacctggggc cccagtttga gttccttgtg 750
gcctactgga ggagggagcc tggtgccgag gaacatgtca aaatggtgag 800
gagtgggggt attccagtgc acctagaaac catggagcca ggggctgcat 850
actgtgtgaa ggcccagaca ttcgtgaagg ccattgggag gtacagcgcc 900
ttcagccaga cagaatgtgt ggaggtgcaa ggagaggcca ttcccctggt 950
actggccctg tttgcctttg ttggcttcat gctgatcctt gtggtcgtgc 1000
cactgttcgt ctggaaaatg ggccggctgc tccagtactc ctgttgcccc 1050
gtggtggtcc tcccagacac cttgaaaata accaattcac cccagaagtt 1100
aatcagctgc agaagggagg aggtggatgc ctgtgccacg gctgtgatgt 1150
ctcctgagga actcctcagg gcctggatct cataggtttg cggaagggcc 1200
caggtgaagc cgagaacctg gtctgcatga catggaaacc atgaggggac 1250
aagttgtgtt tctgttttcc gccacggaca agggatgaga gaagtaggaa 1300
gagcctgttg tctacaagtc tagaagcaac catcagaggc agggtggttt 1350
gtctaacaga acactgactg aggcttaggg gatgtgacct ctagactggg 1400
ggctgccact tgctggctga gcaaccctgg gaaaagtgac ttcatccctt 1450
cggtcctaag ttttctcatc tgtaatgggg gaattaccta cacacctgct 1500
aaacacacac acacagagtc tctctctata tatacacacg tacacataaa 1550
tacacccagc acttgcaagg ctagagggaa actggtgaca ctctacagtc 1600
tgactgattc agtgtttctg gagagcagga cataaatgta tgatgagaat 1650
gatcaaggac tctacacact gggtggcttg gagagcccac tttcccagaa 1700
taatccttga gagaaaagga atcatgggag caatggtgtt gagttcactt 1750
caagcccaat gccggtgcag aggggaatgg cttagcgagc tctacagtag 1800
gtgacctgga ggaaggtcac agccacactg aaaatgggat gtgcatgaac 1850
acggaggatc catgaactac tgtaaagtgt tgacagtgtg tgcacactgc 1900
agacagcagg tgaaatgtat gtgtgcaatg cgacgagaat gcagaagtca 1950
gtaacatgtg catgtttgtt gtgctccttt tttctgttgg taaagtacag 2000
aattcagcaa ataaaaaggg ccaccctggc caaaagcggt aaaaaaaaaa 2050

aaaaaa 2056

<210> 32
<211> 311
<212> PRT
<213> Homo Sapien

<400> 32

```
Met Gln Thr Phe Thr Met Val Leu Glu Glu Ile Trp Thr Ser Leu
  1               5                  10                  15

Phe Met Trp Phe Phe Tyr Ala Leu Ile Pro Cys Leu Leu Thr Asp
                 20                  25                  30

Glu Val Ala Ile Leu Pro Ala Pro Gln Asn Leu Ser Val Leu Ser
                 35                  40                  45

Thr Asn Met Lys His Leu Leu Met Trp Ser Pro Val Ile Ala Pro
                 50                  55                  60

Gly Glu Thr Val Tyr Tyr Ser Val Glu Tyr Gln Gly Glu Tyr Glu
                 65                  70                  75

Ser Leu Tyr Thr Ser His Ile Trp Ile Pro Ser Ser Trp Cys Ser
                 80                  85                  90

Leu Thr Glu Gly Pro Glu Cys Asp Val Thr Asp Asp Ile Thr Ala
                 95                 100                 105

Thr Val Pro Tyr Asn Leu Arg Val Arg Ala Thr Leu Gly Ser Gln
                110                 115                 120

Thr Ser Ala Trp Ser Ile Leu Lys His Pro Phe Asn Arg Asn Ser
                125                 130                 135

Thr Ile Leu Thr Arg Pro Gly Met Glu Ile Thr Lys Asp Gly Phe
                140                 145                 150

His Leu Val Ile Glu Leu Glu Asp Leu Gly Pro Gln Phe Glu Phe
                155                 160                 165

Leu Val Ala Tyr Trp Arg Arg Glu Pro Gly Ala Glu Glu His Val
                170                 175                 180

Lys Met Val Arg Ser Gly Gly Ile Pro Val His Leu Glu Thr Met
                185                 190                 195

Glu Pro Gly Ala Ala Tyr Cys Val Lys Ala Gln Thr Phe Val Lys
                200                 205                 210

Ala Ile Gly Arg Tyr Ser Ala Phe Ser Gln Thr Glu Cys Val Glu
                215                 220                 225

Val Gln Gly Glu Ala Ile Pro Leu Val Leu Ala Leu Phe Ala Phe
                230                 235                 240

Val Gly Phe Met Leu Ile Leu Val Val Val Pro Leu Phe Val Trp
                245                 250                 255

Lys Met Gly Arg Leu Leu Gln Tyr Ser Cys Cys Pro Val Val Val
                260                 265                 270

Leu Pro Asp Thr Leu Lys Ile Thr Asn Ser Pro Gln Lys Leu Ile
                275                 280                 285
```

```
Ser Cys Arg Arg Glu Glu Val Asp Ala Cys Ala Thr Ala Val Met
                290                 295                 300

Ser Pro Glu Glu Leu Leu Arg Ala Trp Ile Ser
                305                 310
```

<210> 33
<211> 2531
<212> DNA
<213> Homo Sapien

<400> 33

```
gagacacgcg agcggggaga cctccaaggc agcgaggcat cggacatgtg 50

tcagcacatc tggggcgcac atccgtcgag cccgagggga gatttgccgg 100

aacaattcaa actgcgatat tgatcttggg ggtgactgtc cctggccggc 150

tgtcgggtgg gagtgcgagt gtgcactcgc tcggaagtgt gtgcgagtgt 200

gtatgtgtgt gtgccgtgtc gggctccccc cttccccccg ttttcccgtc 250

gagtgatgca cttggaatga gaatcagagg atggaaatag tctgggaggt 300

gctttttctt cttcaagcca atttcatcgt ctgcatatca gctcaacaga 350

attcaccaaa aatccatgaa ggctggtggg catacaagga ggtggtccag 400

ggaagctttg ttccagttcc ttctttctgg ggattggtga actcagcttg 450

gaatctttgc tctgtgggga aacggcagtc gccagtcaac atagagacca 500

gtcacatgat cttcgacccc tttctgacac ctcttcgcat caacacgggg 550

ggcaggaagg tcagtgggac catgtacaac actggaagac acgtatccct 600

tcgcctggac aaggagcact tggtcaacat atctggaggg cccatgacat 650

acagccaccg gctggaggag atccgactac actttgggag tgaggacagc 700

caagggtcgg agcacctcct caatggacag gccttctctg gggaggtgca 750

gctcatccac tataaccatg agctatatac gaatgtcaca gaagctgcaa 800

agagtccaaa tggattggtg gtagtttcta tatttataaa agtttctgat 850

tcatcaaacc catttcttaa tcgaatgctc aacagagata ctatcacaag 900

aataacatat aaaaatgatg catatttact acaggggctt aatatagagg 950

aactatatcc agagacctct agtttcatca cttacgatgg gtcgatgact 1000

atcccaccct gctatgagac agcaagttgg atcataatga acaaacctgt 1050

ctatataacc aggatgcaga tgcattcctt gcgcctgctc agccagaacc 1100

agccatctca gatctttctg agcatgagtg acaacttcag gcctgtccag 1150

ccactcaaca accgctgcat ccgcaccaat atcaacttca gtttacaggg 1200

gaaggactgt ccaaacaacc gagcccagaa gcttcagtat agagtaaatg 1250

aatggctcct caagtaggga acaaagccaa gaagaatccc acctcagtga 1300
```

aatgctacaa ctgtgaattg acgtaaccta gaatgtcccc cttcttgctt 1350
ctctctcctt ctttccccca agcctcattc attcttggga ttggcccttt 1400
cttcatgaaa agtgtctgcg aaaccatggc agaggaatac atctctcaca 1450
catactcaca aacacacaca caagcacttg cacatacata caaacacatg 1500
caaacatacc tacacacaca cactctctta caacctccat catgggaagt 1550
caagtttcag aaacaaaagt ctcattcata agaggtctta gaagaaaata 1600
accagttaac ctgatttcaa ttttgatacc gttttcctga actaataaat 1650
ctacccaatg agacttttca gcctttgtac atacaaaatt cttccaaaag 1700
agagaggaga aaatacagct ctgatggcat caaacggact ttgcatcaag 1750
taatttcaga tagtgtccta ggatcctttg agggtgctgg tagcaggtga 1800
gcaggacaaa gttgaccaag gacacttatt tctagattat gattcttctg 1850
tttactcaac aatttacaaa gaaaaaaagg acagacattg aagagctaca 1900
cattgtatat atatcaccac agactataag gaaatggaat tatttccctc 1950
tttgtcacat atctgtagta ggatttgcca agatcagaaa tgatccattt 2000
gctgtttctt gttttccaaa ggtcatacat tgtgtttggt tattgttacc 2050
agctcaataa atgtgtttaa cgagttaatt tcatttttct ggctttggtc 2100
tgttctcctt ccttacaggc taagccctgg ctccatgcaa ctgcattctt 2150
tgatttcact tgttccttca tctacatgtt ttgttcattt gcagccagtt 2200
tttactgagt ttgtggcaat caggaatgca tttgctaagc aagtatgact 2250
ttaattccac tccatggctc aatcattcac atgaggtgag cttcagcctg 2300
agatagcagg cgacagactt cttgcgtttc aaaactgcca tgcccccctg 2350
tgatgctccc gtgaaggaat gcactttgcc ttgtaagttc ctgggaaagg 2400
ggtatgtttt ctctccaggt gcagccagat ctcacaaagt acaaaacgaa 2450
tgcctttctt ttcttgttta taatggtcac tcactgtgtt tggttactgt 2500
caagaaatca ataaatgtgt ttaacaagtt a 2531

<210> 34
<211> 328
<212> PRT
<213> Homo Sapien

<400> 34
Met Glu Ile Val Trp Glu Val Leu Phe Leu Leu Gln Ala Asn Phe
1 5 10 15

Ile Val Cys Ile Ser Ala Gln Gln Asn Ser Pro Lys Ile His Glu
20 25 30

Gly Trp Trp Ala Tyr Lys Glu Val Val Gln Gly Ser Phe Val Pro

35 40 45

Val Pro Ser Phe Trp Gly Leu Val Asn Ser Ala Trp Asn Leu Cys
50 55 60

Ser Val Gly Lys Arg Gln Ser Pro Val Asn Ile Glu Thr Ser His
65 70 75

Met Ile Phe Asp Pro Phe Leu Thr Pro Leu Arg Ile Asn Thr Gly
80 85 90

Gly Arg Lys Val Ser Gly Thr Met Tyr Asn Thr Gly Arg His Val
95 100 105

Ser Leu Arg Leu Asp Lys Glu His Leu Val Asn Ile Ser Gly Gly
110 115 120

Pro Met Thr Tyr Ser His Arg Leu Glu Glu Ile Arg Leu His Phe
125 130 135

Gly Ser Glu Asp Ser Gln Gly Ser Glu His Leu Leu Asn Gly Gln
140 145 150

Ala Phe Ser Gly Glu Val Gln Leu Ile His Tyr Asn His Glu Leu
155 160 165

Tyr Thr Asn Val Thr Glu Ala Ala Lys Ser Pro Asn Gly Leu Val
170 175 180

Val Val Ser Ile Phe Ile Lys Val Ser Asp Ser Ser Asn Pro Phe
185 190 195

Leu Asn Arg Met Leu Asn Arg Asp Thr Ile Thr Arg Ile Thr Tyr
200 205 210

Lys Asn Asp Ala Tyr Leu Leu Gln Gly Leu Asn Ile Glu Glu Leu
215 220 225

Tyr Pro Glu Thr Ser Ser Phe Ile Thr Tyr Asp Gly Ser Met Thr
230 235 240

Ile Pro Pro Cys Tyr Glu Thr Ala Ser Trp Ile Ile Met Asn Lys
245 250 255

Pro Val Tyr Ile Thr Arg Met Gln Met His Ser Leu Arg Leu Leu
260 265 270

Ser Gln Asn Gln Pro Ser Gln Ile Phe Leu Ser Met Ser Asp Asn
275 280 285

Phe Arg Pro Val Gln Pro Leu Asn Asn Arg Cys Ile Arg Thr Asn
290 295 300

Ile Asn Phe Ser Leu Gln Gly Lys Asp Cys Pro Asn Asn Arg Ala
305 310 315

Gln Lys Leu Gln Tyr Arg Val Asn Glu Trp Leu Leu Lys
320 325

<210> 35
<211> 3371
<212> DNA
<213> Homo Sapien

<400> 35

gtcggaaccc cctcaggcca ccctcgggag tcctggggtc cagaggggtg 50

tccctgtacc ccttgcacac aggaccctca ctctgcaggg ataagccagc 100

tgcgcctgca gcctagggtg ccaaggaggc tgctgattgt ggcccacagc 150

ctcatctgaa cgccaggaga ccaggatacc gaggcaccgg atcccctctc 200

tgtgccctgg ggagccccag tgctgcccag tcaccccagg gctgaggtct 250

gcgtccctag tggtgcaagg cctggtagga ccacggggca gggaatgtga 300

gcgccatccg agctcacggt gtcctgagtc gcggcttcgt gactttggca 350

ggggcctccg gaccagtgac cccagtcaaa cccagagggt cttgggcggc 400

agcgacgaag gaggtattca ggctccaggc caggtggggc cggacgcccc 450

cagccatcca ccatggtggt ggcacacccc accgccactg ccaccaccac 500

gcccactgcc actgtcacgg ccaccgttgt gatgaccacg gccaccatgg 550

acctgcggga ctggctgttc ctctgctacg ggctcatcgc cttcctgacg 600

gaggtcatcg acagcaccac ctgcccctcg gtgtgccgct gcgacaacgg 650

cttcatctac tgcaacgacc ggggactcac atccatcccc gcagatatcc 700

ctgatgacgc caccaccctc tacctgcaga acaaccagat caacaacgcc 750

ggcatccccc aggacctcaa gaccaaggtc aacgtgcagg tcatctacct 800

atacgagaat gacctggatg agttccccat caacctgccc cgctccctcc 850

gggagctgca cctgcaggac aacaatgtgc gcaccattgc cagggactcg 900

ctggcccgca tcccgctgct ggagaagctg cacctggatg acaactccgt 950

gtccaccgtc agcattgagg aggacgcctt cgccgacagc aaacagctca 1000

agctgctctt cctgagccgg aaccacctga gcagcatccc ctcggggctg 1050

ccgcacacgc tggaggagct gcggctggat gacaaccgca tctccaccat 1100

cccgctgcat gccttcaagg gcctcaacag cctgcggcgc ctggtgctgg 1150

acggtaacct gctggccaac cagcgcatcg ccgacgacac cttcagccgc 1200

ctacagaacc tcacagagct ctcgctggtg cgcaattcgc tggccgcgcc 1250

acccctcaac ctgcccagcg cccacctgca gaagctctac ctgcaggaca 1300

atgccatcag ccacatcccc tacaacacgc tggccaagat gcgtgagctg 1350

gagcggctgg acctgtccaa caacaacctg accacgctgc cccgcggcct 1400

gttcgacgac ctggggaacc tggcccagct gctgctcagg aacaaccctt 1450

ggttttgtgg ctgcaacctc atgtggctgc gggactgggt gaaggcacgg 1500

gcggccgtgg tcaacgtgcg gggcctcatg tgccagggcc ctgagaaggt 1550

ccggggcatg gccatcaagg acattaccag cgagatggac gagtgttttg 1600

agacggggcc gcagggcggc gtggccaatg cggctgccaa gaccacggcc 1650
agcaaccacg cctctgccac cacgccccag ggttccctgt ttaccctcaa 1700
ggccaaaagg ccagggctgc gcctccccga ctccaacatt gactacccca 1750
tggccacggg tgatggcgcc aagaccctgg ccatccacgt gaaggccctg 1800
acggcagact ccatccgcat cacgtggaag gccacgctcc ccgcctcctc 1850
tttccggctc agttggctgc gcctgggcca cagcccagcc gtgggctcca 1900
tcacggagac cttggtgcag gggacaaga cagagtacct gctgacagcc 1950
ctggagccca agtccaccta catcatctgc atggtcacca tggagaccag 2000
caatgcctat gtagctgatg agacacccgt gtgtgccaag gcagagacag 2050
ccgacagcta tggccctacc accacactca accaggagca gaacgctggc 2100
cccatggcga gcctgcccct ggcgggcatc atcggcgggg cagtggctct 2150
ggtcttcctc ttcctggtcc tgggggccat ctgctggtac gtgcaccagg 2200
ctggcgagct gctgacccgg gagagggcct acaaccgggg cagcaggaaa 2250
aaggatgact atatggagtc agggaccaag aaggataact ccatcctgga 2300
aatccgcggc cctgggctgc agatgctgcc catcaacccg taccgcgcca 2350
aagaggagta cgtggtccac actatcttcc cctccaacgg cagcagcctc 2400
tgcaaggcca cacacaccat tggctacggc accacgcggg gctaccggga 2450
cggcggcatc cccgacatag actactccta cacatgatgc ccgcccaccc 2500
gggctgcccc gcctcagccc cagctgccct ggcgtggcca tgtggctttg 2550
cccagcctgc tgcaatccaa gagagcaagg aagagaaatt ccatgggtga 2600
ctttcctccg cagaaagcaa agtttgggga gggctgacga ttttgtagaa 2650
cacaacagtg acaatttttt ttaaaagaat agaaggcagg agggggaatt 2700
cgacattgtt gaagacataa tttataccaa gttatgccag ttggggaggg 2750
aaggactaaa aataatattg caggcagggc tgggttgggt tttttttttt 2800
ccccccctgaa ctggaaggat actacctgta caacatctgt ggacacctca 2850
tgctctgttc aaggccatca caaaggaacc gccagggaga agcagccggc 2900
tctcaaagct cccacgcagc tctcccgcca ctggccactc gctggcgacc 2950
cgatggaagg ttttcaggct cctcacaaag gagagaggga agaaaagatc 3000
ttttgccctg gagatatggt cctgaaatct ctcccctggc ttattccata 3050
ccatttccct tgcagatttg cagaaacatg gcatctttca ctgcattctt 3100
tgaacaatca tgtagtcgat taaaaaaaaa aacaaacttt tttttcctag 3150
gctgaagccc tcttcagttc catgcaccac gctccgtaga agccccggcg 3200

gaagccgtag ctttccctgc cacctggagg tgcatctgtc tgcctgtcta 3250

tccctgtcgc ggtgtctcta agtacagatg ggtagataga gccacatgca 3300

cggtccttac cgttcttctt gggtcagttc ttaccatttc ctgaacaata 3350

gaattgtgaa agtgttaaaa a 3371

<210> 36
<211> 674
<212> PRT
<213> Homo Sapien

<400> 36
Met Val Val Ala His Pro Thr Ala Thr Ala Thr Thr Thr Pro Thr
1 5 10 15

Ala Thr Val Thr Ala Thr Val Val Met Thr Thr Ala Thr Met Asp
20 25 30

Leu Arg Asp Trp Leu Phe Leu Cys Tyr Gly Leu Ile Ala Phe Leu
35 40 45

Thr Glu Val Ile Asp Ser Thr Thr Cys Pro Ser Val Cys Arg Cys
50 55 60

Asp Asn Gly Phe Ile Tyr Cys Asn Asp Arg Gly Leu Thr Ser Ile
65 70 75

Pro Ala Asp Ile Pro Asp Asp Ala Thr Thr Leu Tyr Leu Gln Asn
80 85 90

Asn Gln Ile Asn Asn Ala Gly Ile Pro Gln Asp Leu Lys Thr Lys
95 100 105

Val Asn Val Gln Val Ile Tyr Leu Tyr Glu Asn Asp Leu Asp Glu
110 115 120

Phe Pro Ile Asn Leu Pro Arg Ser Leu Arg Glu Leu His Leu Gln
125 130 135

Asp Asn Asn Val Arg Thr Ile Ala Arg Asp Ser Leu Ala Arg Ile
140 145 150

Pro Leu Leu Glu Lys Leu His Leu Asp Asp Asn Ser Val Ser Thr
155 160 165

Val Ser Ile Glu Glu Asp Ala Phe Ala Asp Ser Lys Gln Leu Lys
170 175 180

Leu Leu Phe Leu Ser Arg Asn His Leu Ser Ser Ile Pro Ser Gly
185 190 195

Leu Pro His Thr Leu Glu Glu Leu Arg Leu Asp Asp Asn Arg Ile
200 205 210

Ser Thr Ile Pro Leu His Ala Phe Lys Gly Leu Asn Ser Leu Arg
215 220 225

Arg Leu Val Leu Asp Gly Asn Leu Leu Ala Asn Gln Arg Ile Ala
230 235 240

Asp Asp Thr Phe Ser Arg Leu Gln Asn Leu Thr Glu Leu Ser Leu

```
                245                 250                 255

Val Arg Asn Ser Leu Ala Ala Pro Pro Leu Asn Leu Pro Ser Ala
                260                 265                 270

His Leu Gln Lys Leu Tyr Leu Gln Asp Asn Ala Ile Ser His Ile
                275                 280                 285

Pro Tyr Asn Thr Leu Ala Lys Met Arg Glu Leu Glu Arg Leu Asp
                290                 295                 300

Leu Ser Asn Asn Asn Leu Thr Thr Leu Pro Arg Gly Leu Phe Asp
                305                 310                 315

Asp Leu Gly Asn Leu Ala Gln Leu Leu Leu Arg Asn Asn Pro Trp
                320                 325                 330

Phe Cys Gly Cys Asn Leu Met Trp Leu Arg Asp Trp Val Lys Ala
                335                 340                 345

Arg Ala Ala Val Val Asn Val Arg Gly Leu Met Cys Gln Gly Pro
                350                 355                 360

Glu Lys Val Arg Gly Met Ala Ile Lys Asp Ile Thr Ser Glu Met
                365                 370                 375

Asp Glu Cys Phe Glu Thr Gly Pro Gln Gly Gly Val Ala Asn Ala
                380                 385                 390

Ala Ala Lys Thr Thr Ala Ser Asn His Ala Ser Ala Thr Thr Pro
                395                 400                 405

Gln Gly Ser Leu Phe Thr Leu Lys Ala Lys Arg Pro Gly Leu Arg
                410                 415                 420

Leu Pro Asp Ser Asn Ile Asp Tyr Pro Met Ala Thr Gly Asp Gly
                425                 430                 435

Ala Lys Thr Leu Ala Ile His Val Lys Ala Leu Thr Ala Asp Ser
                440                 445                 450

Ile Arg Ile Thr Trp Lys Ala Thr Leu Pro Ala Ser Ser Phe Arg
                455                 460                 465

Leu Ser Trp Leu Arg Leu Gly His Ser Pro Ala Val Gly Ser Ile
                470                 475                 480

Thr Glu Thr Leu Val Gln Gly Asp Lys Thr Glu Tyr Leu Leu Thr
                485                 490                 495

Ala Leu Glu Pro Lys Ser Thr Tyr Ile Ile Cys Met Val Thr Met
                500                 505                 510

Glu Thr Ser Asn Ala Tyr Val Ala Asp Glu Thr Pro Val Cys Ala
                515                 520                 525

Lys Ala Glu Thr Ala Asp Ser Tyr Gly Pro Thr Thr Thr Leu Asn
                530                 535                 540

Gln Glu Gln Asn Ala Gly Pro Met Ala Ser Leu Pro Leu Ala Gly
                545                 550                 555

Ile Ile Gly Gly Ala Val Ala Leu Val Phe Leu Phe Leu Val Leu
                560                 565                 570
```

```
Gly Ala Ile Cys Trp Tyr Val His Gln Ala Gly Glu Leu Leu Thr
                575                 580                 585

Arg Glu Arg Ala Tyr Asn Arg Gly Ser Arg Lys Lys Asp Asp Tyr
                590                 595                 600

Met Glu Ser Gly Thr Lys Lys Asp Asn Ser Ile Leu Glu Ile Arg
                605                 610                 615

Gly Pro Gly Leu Gln Met Leu Pro Ile Asn Pro Tyr Arg Ala Lys
                620                 625                 630

Glu Glu Tyr Val Val His Thr Ile Phe Pro Ser Asn Gly Ser Ser
                635                 640                 645

Leu Cys Lys Ala Thr His Thr Ile Gly Tyr Gly Thr Thr Arg Gly
                650                 655                 660

Tyr Arg Asp Gly Gly Ile Pro Asp Ile Asp Tyr Ser Tyr Thr
                665                 670

<210> 37
<211> 3501
<212> DNA
<213> Homo Sapien

<220>
<221> unsure
<222> 2762, 2778
<223> unknown base

<400> 37
ggtgactgaa gcgagcctgg cctcttgcat cctccgcctg tgtacctccc 50

tccccttttt ttccgccttc tgccagcaga agcagcagcc gcagcacctg 100

agccgctact gccgctcact caggacaacg ctatggctga gcctgggcac 150

agccaccatc tctccgccag agtcaggaga agaactgaga ggcgcatacc 200

ccggctgtgg cggctgctgc tctgggctgg gaccgccttc caggtgaccc 250

agggaacggg accggagctt catgcctgca aagagtctga gtaccactat 300

gagtacacgg cgtgtgacag cacgggttcc aggtggaggg tcgccgtgcc 350

gcataccccg ggcctgtgca ccagcctgtc tgaccccgtc aagggcaccg 400

agtgctcctt ctcctgcaac gccggggagt ttctggatat gaaggaccag 450

tcatgtaagc catgcgctga gggccgctac tccctcggca caggcattcg 500

gtttgatgag tgggatgagc tgccccatgg ctttgccagc ctctcagcca 550

acatggagct ggatgacagt gctgctgagt ccaccgggaa ctgtacttcg 600

tccaagtggg ttccccgggg cgactacatc gcctccaaca cggacgaatg 650

cacagccaca ctgatgtacg ccgtcaacct gaagcaatct ggcaccgtta 700

acttcgaata ctactatcca gactccagca tcatctttga gtttttcgtt 750

cagaatgacc agtgccagcc caatgcagat gactccaggt ggatgaagac 800
```

cacagagaaa ggatgggaat tccacagtgt ggagctaaat cgaggcaata 850
atgtcctcta ttggagaacc acagccttct cagtatggac caaagtaccc 900
aagcctgtgc tggtgagaaa cattgccata acaggggtgg cctacacttc 950
agaatgcttc ccctgcaaac ctggcacgta tgcagacaag cagggctcct 1000
ctttctgcaa actttgccca gccaactctt attcaaataa aggagaaact 1050
tcttgccacc agtgtgaccc tgacaaatac tcagagaaag gatcttcttc 1100
ctgtaacgtg cgcccagctt gcacagacaa agattatttc tacacacaca 1150
cggcctgcga tgccaacgga gagacacaac tcatgtacaa atgggccaag 1200
ccgaaaatct gtagcgagga ccttgagggg gcagtgaagc tgcctgcctc 1250
tggtgtgaag acccactgcc caccctgcaa cccaggcttc ttcaaaacca 1300
acaacagcac ctgccagccc tgcccatatg gttcctactc caatggctca 1350
gactgtaccc gctgccctgc agggactgaa cctgctgtgg gatttgaata 1400
caaatggtgg aacacgctgc ccacaaacat ggaaacgacc gttctcagtg 1450
ggatcaactt cgagtacaag ggcatgacag gctgggaggt ggctggtgat 1500
cacatttaca cagctgctgg agcctcagac aatgacttca tgattctcac 1550
tctggttgtg ccaggattta gacctccgca gtcggtgatg gcagacacag 1600
agaataaaga ggtggccaga atcacatttg tctttgagac cctctgttct 1650
gtgaactgtg agctctactt catggtgggt gtgaattcta ggaccaacac 1700
tcctgtggag acgtggaaag gttccaaagg caaacagtcc tatacctaca 1750
tcattgagga gaacactacc acgagcttca cctgggcctt ccagaggacc 1800
acttttcatg aggcaagcag gaagtacacc aatgacgttg ccaagatcta 1850
ctccatcaat gtcaccaatg ttatgaatgg cgtggcctcc tactgccgtc 1900
cctgtgccct agaagcctct gatgtgggct cctcctgcac ctcttgtcct 1950
gctggttact atattgaccg agattcagga acctgccact cctgccccc 2000
taacacaatt ctgaaagccc accagcctta tggtgtccag gcctgtgtgc 2050
cctgtggtcc agggaccaag aacaacaaga tccactctct gtgctacaat 2100
gattgcacct tctcacgcaa cactccaacc aggactttca actacaactt 2150
ctccgctttg gcaaacaccg tcactcttgc tggagggcca agcttcactt 2200
ccaaagggtt gaaatacttc catcacttta ccctcagtct ctgtggaaac 2250
cagggtagga aaatgtctgt gtgcaccgac aatgtcactg acctccggat 2300
tcctgagggt gagtcagggt tctccaaatc tatcacagcc tacgtctgcc 2350
aggcagtcat catccccca gaggtgacag gctacaaggc cggggtttcc 2400

```
tcacagcctg tcagccttgc tgatcgactt attggggtga caacagatat 2450
gactctggat ggaatcacct ccccagctga acttttccac ctggagtcct 2500
tgggaatacc ggacgtgatc ttcttttata ggtccaatga tgtgacccag 2550
tcctgcagtt ctgggagatc aaccaccatc cgcgtcaggt gcagtccaca 2600
gaaaactgtc cctggaagtt tgctgctgcc aggaacgtgc tcagatggga 2650
cctgtgatgg ctgcaacttc cacttcctgt gggagagcgc ggctgcttgc 2700
ccgctctgct cagtggctga ctaccatgct atcgtcagca gctgtgtggc 2750
tgggatccag angactactt acgtgtgncg agaacccaag ctatgctctg 2800
gtggcatttc tctgcctgag cagagagtca ccatctgcaa aaccatagat 2850
ttctggctga aagtgggcat ctctgcaggc acctgtactg ccatcctgct 2900
caccgtcttg acctgctact tttggaaaaa gaatcaaaaa ctagagtaca 2950
agtactccaa gctggtgatg aatgctactc tcaaggactg tgacctgcca 3000
gcagctgaca gctgcgccat catggaaggc gaggatgtag aggacgacct 3050
catctttacc agcaagaagt cactttttgg gaagatcaaa tcatttacct 3100
ccaagaggac tcctgatgga tttgactcag tgccgctgaa gacatcctca 3150
ggaggcccag acatggacct gtgagaggca ctgcctgcct cacctgcctc 3200
ctcaccttgc atagcacctt tgcaagcctg cggcgatttg ggtgccagca 3250
tcctgcaaca cccactgctg gaaatctctt cattgtggcc ttatcagatg 3300
tttgaatttc agatcttttt ttatagagta cccaaaccct cctttctgct 3350
tgcctcaaac ctgccaaata tacccacatt ttttttaaa aaaaaaaaaa 3400
aaaaaaaaaa aaaaaaaaaa aaaaaaaaaa aaaaaaaaaa aaaaaaaaaa 3450
aaaaaaaaaa aaaaaaaaaa aaaaaaaaaa aaaaaaaaaa aaaaaaaaaa 3500
a 3501
```

<210> 38
<211> 1013
<212> PRT
<213> Homo Sapien

<220>
<221> unsure
<222> 877, 882
<223> unknown amino acid

<400> 38

```
Met Ala Glu Pro Gly His Ser His His Leu Ser Ala Arg Val Arg
 1               5                  10                  15

Arg Arg Thr Glu Arg Arg Ile Pro Arg Leu Trp Arg Leu Leu Leu
                20                  25                  30
```

```
Trp Ala Gly Thr Ala Phe Gln Val Thr Gln Gly Thr Gly Pro Glu
                35                  40                  45

Leu His Ala Cys Lys Glu Ser Glu Tyr His Tyr Glu Tyr Thr Ala
                50                  55                  60

Cys Asp Ser Thr Gly Ser Arg Trp Arg Val Ala Val Pro His Thr
                65                  70                  75

Pro Gly Leu Cys Thr Ser Leu Ser Asp Pro Val Lys Gly Thr Glu
                80                  85                  90

Cys Ser Phe Ser Cys Asn Ala Gly Glu Phe Leu Asp Met Lys Asp
                95                  100                 105

Gln Ser Cys Lys Pro Cys Ala Glu Gly Arg Tyr Ser Leu Gly Thr
                110                 115                 120

Gly Ile Arg Phe Asp Glu Trp Asp Glu Leu Pro His Gly Phe Ala
                125                 130                 135

Ser Leu Ser Ala Asn Met Glu Leu Asp Asp Ser Ala Ala Glu Ser
                140                 145                 150

Thr Gly Asn Cys Thr Ser Ser Lys Trp Val Pro Arg Gly Asp Tyr
                155                 160                 165

Ile Ala Ser Asn Thr Asp Glu Cys Thr Ala Thr Leu Met Tyr Ala
                170                 175                 180

Val Asn Leu Lys Gln Ser Gly Thr Val Asn Phe Glu Tyr Tyr Tyr
                185                 190                 195

Pro Asp Ser Ser Ile Ile Phe Glu Phe Phe Val Gln Asn Asp Gln
                200                 205                 210

Cys Gln Pro Asn Ala Asp Asp Ser Arg Trp Met Lys Thr Thr Glu
                215                 220                 225

Lys Gly Trp Glu Phe His Ser Val Glu Leu Asn Arg Gly Asn Asn
                230                 235                 240

Val Leu Tyr Trp Arg Thr Thr Ala Phe Ser Val Trp Thr Lys Val
                245                 250                 255

Pro Lys Pro Val Leu Val Arg Asn Ile Ala Ile Thr Gly Val Ala
                260                 265                 270

Tyr Thr Ser Glu Cys Phe Pro Cys Lys Pro Gly Thr Tyr Ala Asp
                275                 280                 285

Lys Gln Gly Ser Ser Phe Cys Lys Leu Cys Pro Ala Asn Ser Tyr
                290                 295                 300

Ser Asn Lys Gly Glu Thr Ser Cys His Gln Cys Asp Pro Asp Lys
                305                 310                 315

Tyr Ser Glu Lys Gly Ser Ser Ser Cys Asn Val Arg Pro Ala Cys
                320                 325                 330

Thr Asp Lys Asp Tyr Phe Tyr Thr His Thr Ala Cys Asp Ala Asn
                335                 340                 345

Gly Glu Thr Gln Leu Met Tyr Lys Trp Ala Lys Pro Lys Ile Cys
                                            --
```

350 355 360

Ser Glu Asp Leu Glu Gly Ala Val Lys Leu Pro Ala Ser Gly Val
365 370 375

Lys Thr His Cys Pro Pro Cys Asn Pro Gly Phe Phe Lys Thr Asn
380 385 390

Asn Ser Thr Cys Gln Pro Cys Pro Tyr Gly Ser Tyr Ser Asn Gly
395 400 405

Ser Asp Cys Thr Arg Cys Pro Ala Gly Thr Glu Pro Ala Val Gly
410 415 420

Phe Glu Tyr Lys Trp Trp Asn Thr Leu Pro Thr Asn Met Glu Thr
425 430 435

Thr Val Leu Ser Gly Ile Asn Phe Glu Tyr Lys Gly Met Thr Gly
440 445 450

Trp Glu Val Ala Gly Asp His Ile Tyr Thr Ala Ala Gly Ala Ser
455 460 465

Asp Asn Asp Phe Met Ile Leu Thr Leu Val Val Pro Gly Phe Arg
470 475 480

Pro Pro Gln Ser Val Met Ala Asp Thr Glu Asn Lys Glu Val Ala
485 490 495

Arg Ile Thr Phe Val Phe Glu Thr Leu Cys Ser Val Asn Cys Glu
500 505 510

Leu Tyr Phe Met Val Gly Val Asn Ser Arg Thr Asn Thr Pro Val
515 520 525

Glu Thr Trp Lys Gly Ser Lys Gly Lys Gln Ser Tyr Thr Tyr Ile
530 535 540

Ile Glu Glu Asn Thr Thr Thr Ser Phe Thr Trp Ala Phe Gln Arg
545 550 555

Thr Thr Phe His Glu Ala Ser Arg Lys Tyr Thr Asn Asp Val Ala
560 565 570

Lys Ile Tyr Ser Ile Asn Val Thr Asn Val Met Asn Gly Val Ala
575 580 585

Ser Tyr Cys Arg Pro Cys Ala Leu Glu Ala Ser Asp Val Gly Ser
590 595 600

Ser Cys Thr Ser Cys Pro Ala Gly Tyr Tyr Ile Asp Arg Asp Ser
605 610 615

Gly Thr Cys His Ser Cys Pro Pro Asn Thr Ile Leu Lys Ala His
620 625 630

Gln Pro Tyr Gly Val Gln Ala Cys Val Pro Cys Gly Pro Gly Thr
635 640 645

Lys Asn Asn Lys Ile His Ser Leu Cys Tyr Asn Asp Cys Thr Phe
650 655 660

Ser Arg Asn Thr Pro Thr Arg Thr Phe Asn Tyr Asn Phe Ser Ala
665 670 675

```
Leu Ala Asn Thr Val Thr Leu Ala Gly Gly Pro Ser Phe Thr Ser
                680                 685                 690

Lys Gly Leu Lys Tyr Phe His His Phe Thr Leu Ser Leu Cys Gly
                695                 700                 705

Asn Gln Gly Arg Lys Met Ser Val Cys Thr Asp Asn Val Thr Asp
                710                 715                 720

Leu Arg Ile Pro Glu Gly Glu Ser Gly Phe Ser Lys Ser Ile Thr
                725                 730                 735

Ala Tyr Val Cys Gln Ala Val Ile Ile Pro Pro Glu Val Thr Gly
                740                 745                 750

Tyr Lys Ala Gly Val Ser Ser Gln Pro Val Ser Leu Ala Asp Arg
                755                 760                 765

Leu Ile Gly Val Thr Thr Asp Met Thr Leu Asp Gly Ile Thr Ser
                770                 775                 780

Pro Ala Glu Leu Phe His Leu Glu Ser Leu Gly Ile Pro Asp Val
                785                 790                 795

Ile Phe Phe Tyr Arg Ser Asn Asp Val Thr Gln Ser Cys Ser Ser
                800                 805                 810

Gly Arg Ser Thr Thr Ile Arg Val Arg Cys Ser Pro Gln Lys Thr
                815                 820                 825

Val Pro Gly Ser Leu Leu Leu Pro Gly Thr Cys Ser Asp Gly Thr
                830                 835                 840

Cys Asp Gly Cys Asn Phe His Phe Leu Trp Glu Ser Ala Ala Ala
                845                 850                 855

Cys Pro Leu Cys Ser Val Ala Asp Tyr His Ala Ile Val Ser Ser
                860                 865                 870

Cys Val Ala Gly Ile Gln Xaa Thr Thr Tyr Val Xaa Arg Glu Pro
                875                 880                 885

Lys Leu Cys Ser Gly Gly Ile Ser Leu Pro Glu Gln Arg Val Thr
                890                 895                 900

Ile Cys Lys Thr Ile Asp Phe Trp Leu Lys Val Gly Ile Ser Ala
                905                 910                 915

Gly Thr Cys Thr Ala Ile Leu Leu Thr Val Leu Thr Cys Tyr Phe
                920                 925                 930

Trp Lys Lys Asn Gln Lys Leu Glu Tyr Lys Tyr Ser Lys Leu Val
                935                 940                 945

Met Asn Ala Thr Leu Lys Asp Cys Asp Leu Pro Ala Ala Asp Ser
                950                 955                 960

Cys Ala Ile Met Glu Gly Glu Asp Val Glu Asp Asp Leu Ile Phe
                965                 970                 975

Thr Ser Lys Lys Ser Leu Phe Gly Lys Ile Lys Ser Phe Thr Ser
                980                 985                 990
```

```
Lys Arg Thr Pro Asp Gly Phe Asp Ser Val Pro Leu Lys Thr Ser
                995                 1000                1005

Ser Gly Gly Pro Asp Met Asp Leu
                1010
```

<210> 39
<211> 2998
<212> DNA
<213> Homo Sapien

<400> 39

```
gggaaggggt tctgggctgc cgcaggcaca caggccagag cttcgtggat 50
acctgcaggg cccaaaggtc cctccctgtt ttgaagagtg agtgatggct 100
atgaggtagc ggccaggctg atcacccctg cgttggctgg aggcagaatt 150
ctgtaaatcc tcgccaagtc tttctccagg ccactggtta gctcatctca 200
gcctcctctg ggagcatcaa caccaacatg gcacagggga ctgcagtggt 250
gtgctttgga cctgtgtacc cacccaaggc taaaggcaga gccaggtgac 300
tttgcggggg tctcttctct aggattatct gtacttcccc tctgtcctct 350
tttactacgg gagatcgagc tagctataac ccaccttctt tcatgagaac 400
cacactaaat tgcaaaaatt atcccagtgc tggaggaggg cagcaggttg 450
agattatgtt ggcaggaaga atgttggcat tgattggcac gcaggggacg 500
agagctgctt tgtgctttaa aggagccaag ttacaccctg tttaaccctg 550
ccttcaaagg gacgactctg taagattctc tgctacttat tcaagttgac 600
acgatgccct tcacactcca cctgaggtcc cgccttccct ctgccataag 650
gagtttgatt ctacaaaaga aaccaaacat cagaaataca tccagcatgg 700
ctggagagct ccgaccagcc agcctggtgg tcctgcccag gtcccttgct 750
ccagcttttg aaagattctg ccaggtcaac actggtcctc taccccctgct 800
gggccagagt gagccagaaa agtggatgct gccccctcaa ggtgctatct 850
cagagaccag gatgggccat ccccagttct ggaaatacga gttcggtgcc 900
tgcaccggta gcctggcttc gctggagcag tactcggagc agctgaagga 950
catggtggcc ttcttcctgg gctgcagctt ctccctggag gaggccttgg 1000
agaaagcggg gctccccaga agagacccag caggtcacag ccaggcgggt 1050
gcatacaaga caacagtgcc ttgtgttacc catgctggct tctgctgccc 1100
tctggtggtc acgatgaggc ccattcccaa ggacaagctg gaagggctgg 1150
tgcgggcctg ctgctccctc ggaggtgagc aggggcaacc tgttcacatg 1200
ggcgacccag aactgttggg aatcaaagag ctttccaaac ctgcctacgg 1250
ggatgccatg gtgtgtcccc caggggaggt tccagtgttc tggccttctc 1300
```

cgctgaccag tctcggagct gtcagcagct gtgagacccc actggctttt 1350
gccagcatcc caggctgcac agttatgact gacctgaagg atgcaaaggc 1400
tccacctggt tgtctcaccc cagagagaat tccagaggtc catcacattt 1450
cccaagatcc tctgcactac agcatcgcgt cagtctctgc ttctcagaag 1500
atcagagaac tagagtctat gatcggcata gacccaggga accgggggat 1550
tgggcacctg ctctgtaaag atgagctgct gaaggcctct ctctcgctgt 1600
cccatgcccg ctcagtgctc atcaccactg ggttccccac acatttcaat 1650
catgagcctc cagaagagac agatggccca ccaggagctg ttgctctggt 1700
tgccttcctg caggccttgg agaaggaggt cgccataatc gttgaccaga 1750
gagcctggaa cttgcaccag aagattgttg aagatgctgt tgagcaaggt 1800
gttctgaaga cgcagatccc gatattaact taccaaggtg gatcagtgga 1850
agctgctcag gcattcctgt gcaaaaatgg ggacccgcag acacctagat 1900
ttgaccacct ggtggccata gagcgtgccg gaagagctgc tgatggcaat 1950
tactacaatg caaggaagat gaacatcaag cacttggttg accccattga 2000
cgatcttttt cttgctgcga agaagattcc tggaatctca tcaactggag 2050
tcggtgatgg aggcaacgag cttgggatgg gtaaagtcaa ggaggctgtg 2100
aggaggcaca tacggcacgg ggatgtcatc gcctgcgacg tggaggctga 2150
ctttgccgtc attgctggtg tttctaactg gggaggctat gccctggcct 2200
gcgcactcta catcctgtac tcatgtgctg tccacagtca gtacctgagg 2250
aaagcagtcg gaccctccag ggcacctgga gatcaggcct ggactcaggc 2300
cctcccgtcg gtcattaagg aagaaaaaat gctgggcatc ttggtgcagc 2350
acaaagtccg gagtggcgtc tcgggcatcg tgggcatgga ggtggatggg 2400
ctgcccttcc acaacaccca cgccgagatg atccagaagc tggtggacgt 2450
caccacggca caggtgtaac cgtccatgtt ccgtgtgagc agagtcccta 2500
ccaacgggca ggtctgcatc cggggagaat gcagctgctt ctggcgacaa 2550
tcctgctagt aaacactggt cttcggtgag caacgaacac tcgcctggcc 2600
tgggaaactg catgcccact ttctgggagg ggttagtgca ggtgccgtgg 2650
acaaaggaca acatttctct ggggcttttt aacttttatt cctaagactc 2700
taaaggcgtt gatttcaacc ctccttcact ctggcttctt caggcaaccc 2750
acgtggtctc ctatgagaat cttctcgaca gttacttatg gggacacttg 2800
tgaacaatta actgccaggg cagagcatga gaacaaacat tcccaggcca 2850
tgtaggatag gatactccag actccagtca tcctccccca tccatggttt 2900

ctgttactca tggtttcagt tactcatagc caactgcaga ccgaaaatac 2950

taaatgaaaa atttcagaaa taaacaactc ttaagtttta aaaaaaaa 2998

<210> 40
<211> 621
<212> PRT
<213> Homo Sapien

<400> 40

```
Met Pro Phe Thr Leu His Leu Arg Ser Arg Leu Pro Ser Ala Ile
1               5                   10                  15

Arg Ser Leu Ile Leu Gln Lys Lys Pro Asn Ile Arg Asn Thr Ser
                20                  25                  30

Ser Met Ala Gly Glu Leu Arg Pro Ala Ser Leu Val Val Leu Pro
                35                  40                  45

Arg Ser Leu Ala Pro Ala Phe Glu Arg Phe Cys Gln Val Asn Thr
                50                  55                  60

Gly Pro Leu Pro Leu Leu Gly Gln Ser Glu Pro Glu Lys Trp Met
                65                  70                  75

Leu Pro Pro Gln Gly Ala Ile Ser Glu Thr Arg Met Gly His Pro
                80                  85                  90

Gln Phe Trp Lys Tyr Glu Phe Gly Ala Cys Thr Gly Ser Leu Ala
                95                  100                 105

Ser Leu Glu Gln Tyr Ser Glu Gln Leu Lys Asp Met Val Ala Phe
                110                 115                 120

Phe Leu Gly Cys Ser Phe Ser Leu Glu Glu Ala Leu Glu Lys Ala
                125                 130                 135

Gly Leu Pro Arg Arg Asp Pro Ala Gly His Ser Gln Ala Gly Ala
                140                 145                 150

Tyr Lys Thr Thr Val Pro Cys Val Thr His Ala Gly Phe Cys Cys
                155                 160                 165

Pro Leu Val Val Thr Met Arg Pro Ile Pro Lys Asp Lys Leu Glu
                170                 175                 180

Gly Leu Val Arg Ala Cys Cys Ser Leu Gly Gly Glu Gln Gly Gln
                185                 190                 195

Pro Val His Met Gly Asp Pro Glu Leu Leu Gly Ile Lys Glu Leu
                200                 205                 210

Ser Lys Pro Ala Tyr Gly Asp Ala Met Val Cys Pro Pro Gly Glu
                215                 220                 225

Val Pro Val Phe Trp Pro Ser Pro Leu Thr Ser Leu Gly Ala Val
                230                 235                 240

Ser Ser Cys Glu Thr Pro Leu Ala Phe Ala Ser Ile Pro Gly Cys
                245                 250                 255

Thr Val Met Thr Asp Leu Lys Asp Ala Lys Ala Pro Pro Gly Cys
                260                 265                 270
```

```
Leu Thr Pro Glu Arg Ile Pro Glu Val His His Ile Ser Gln Asp
                275                 280                 285

Pro Leu His Tyr Ser Ile Ala Ser Val Ser Ala Ser Gln Lys Ile
                290                 295                 300

Arg Glu Leu Glu Ser Met Ile Gly Ile Asp Pro Gly Asn Arg Gly
                305                 310                 315

Ile Gly His Leu Leu Cys Lys Asp Glu Leu Leu Lys Ala Ser Leu
                320                 325                 330

Ser Leu Ser His Ala Arg Ser Val Leu Ile Thr Thr Gly Phe Pro
                335                 340                 345

Thr His Phe Asn His Glu Pro Pro Glu Glu Thr Asp Gly Pro Pro
                350                 355                 360

Gly Ala Val Ala Leu Val Ala Phe Leu Gln Ala Leu Glu Lys Glu
                365                 370                 375

Val Ala Ile Ile Val Asp Gln Arg Ala Trp Asn Leu His Gln Lys
                380                 385                 390

Ile Val Glu Asp Ala Val Glu Gln Gly Val Leu Lys Thr Gln Ile
                395                 400                 405

Pro Ile Leu Thr Tyr Gln Gly Gly Ser Val Glu Ala Ala Gln Ala
                410                 415                 420

Phe Leu Cys Lys Asn Gly Asp Pro Gln Thr Pro Arg Phe Asp His
                425                 430                 435

Leu Val Ala Ile Glu Arg Ala Gly Arg Ala Ala Asp Gly Asn Tyr
                440                 445                 450

Tyr Asn Ala Arg Lys Met Asn Ile Lys His Leu Val Asp Pro Ile
                455                 460                 465

Asp Asp Leu Phe Leu Ala Ala Lys Lys Ile Pro Gly Ile Ser Ser
                470                 475                 480

Thr Gly Val Gly Asp Gly Gly Asn Glu Leu Gly Met Gly Lys Val
                485                 490                 495

Lys Glu Ala Val Arg Arg His Ile Arg His Gly Asp Val Ile Ala
                500                 505                 510

Cys Asp Val Glu Ala Asp Phe Ala Val Ile Ala Gly Val Ser Asn
                515                 520                 525

Trp Gly Gly Tyr Ala Leu Ala Cys Ala Leu Tyr Ile Leu Tyr Ser
                530                 535                 540

Cys Ala Val His Ser Gln Tyr Leu Arg Lys Ala Val Gly Pro Ser
                545                 550                 555

Arg Ala Pro Gly Asp Gln Ala Trp Thr Gln Ala Leu Pro Ser Val
                560                 565                 570

Ile Lys Glu Glu Lys Met Leu Gly Ile Leu Val Gln His Lys Val
                575                 580                 585

Arg Ser Gly Val Ser Gly Ile Val Gly Met Glu Val Asp Gly Leu
```

```
                590                 595                 600

Pro Phe His Asn Thr His Ala Glu Met Ile Gln Lys Leu Val Asp
                605                 610                 615

Val Thr Thr Ala Gln Val
                620
```

<210> 41
<211> 889
<212> DNA
<213> Homo Sapien

<400> 41

```
ctttcctgtt ttatccgcag ccctttctt ctttgagtta gtaaagattt 50

attctgtaac ctgacactca tctggccctt tgcagtttgc cagccatatt 100

cccatgtgat ttcccactgg atccaggccc ccatccggct ggcaggaggg 150

ggctctgacg tacaggttgg aaatcagaag tctgtgagag cgcgggagtg 200

catggcagct ctgggtccca gacctggccc gacccctctg cttcacctcc 250

agctctgctg ctcctctact cttgggtcga gatccctttg gagccacagc 300

gaggaaccct gtggtcctca ggcaggtgta ccttgagtca gccaggagcc 350

ctcttttcct gtgtcaaagc ctgccctcgg gctctgctca cctctggtga 400

ccctccaaga tgcccctgcc ctcagtttcc cctcatgatc tggcctctgc 450

cccctctct agccacagcc tctagtacac tttagcaata ccaccagact 500

agttagagtt ccccactcac caagcaagac atgcagtttc atgcctctgt 550

gccttcgctc atgctgtttc ttccgactgg aatgccttcc cctgctcctc 600

ctgccttgtc tgcctggcaa gttcatctct cacgatcccc tcaaaggccc 650

cctcctccag gaaggcaacc cctgtgcccc tcccctccag gctacctctg 700

cactttgtca atgcttctct tgtggcactt atcacactgt attttacttg 750

tttacatgtt tgtctcccct tctagactgt gaatccttaa gggcatggac 800

tgtatcttat gcatctctgt atttctgcgc ctagcacggt gcctagcaca 850

cagtaggcgc tcaataaatg ttgaatgaat gaatgattt 889
```

<210> 42
<211> 83
<212> PRT
<213> Homo Sapien

<400> 42

```
Met Gln Phe His Ala Ser Val Pro Ser Leu Met Leu Phe Leu Pro
 1               5                  10                  15

Thr Gly Met Pro Ser Pro Ala Pro Pro Ala Leu Ser Ala Trp Gln
                20                  25                  30

Val His Leu Ser Arg Ser Pro Gln Arg Pro Pro Pro Pro Gly Arg
                35                  40                  45
```

```
Gln Pro Leu Cys Pro Ser Pro Pro Gly Tyr Leu Cys Thr Leu Ser
                50                  55                  60

Met Leu Leu Leu Trp His Leu Ser His Cys Ile Leu Leu Val Tyr
                65                  70                  75

Met Phe Val Ser Pro Ser Arg Leu
                80

<210> 43
<211> 1356
<212> DNA
<213> Homo Sapien

<400> 43
gtttccaaca aggatgatat gaagacttcc ctgaagaaag ttgtgaaggg 50

acctcctacg agatgatgat gcagtgtgtg tcccgcatgt tggcccaccc 100

cctgcatgtc atctcaatgc gctgcatggt ccagtttgtg ggacgggagg 150

ccaagtacag tggtgtgctg agctccattg ggaagatttt caaagaggaa 200

gggctgctgg gattcttcgt tggattaatc cctcacctcc tgggcgatgt 250

ggttttcttg tggggctgta acctgctggc ccacttcatc aatgcctacc 300

tggtggatga cagcttcagc caggccctgg ccatccggag ctataccaag 350

ttcgtgatgg ggattgcagt gagcatgctg acctacccct tcctgctagt 400

tggcgacctc atggctgtga acaactgcgg gctgcaagct gggctccccc 450

cttactcccc agtgttcaaa tcctggattc actgctggaa gtacctgagt 500

gtgcagggcc agctcttccg aggctccagc ctgcttttcc gccgggtgtc 550

atcaggatca tgctttgccc tggagtaacc tgaatcatct aaaaaacacg 600

gtctcaacct ggccactgtg ggtgaggcct gaccaccttg ggacacctgc 650

aagacgactc caacccaaca acaaccagat gtgctccagc ccagccgggc 700

ttcagttcca tatttgccat gtgtctgtcc agatgtgggg ttgagcgggg 750

gtggggctgc acccagtgga ttgggtcacc cggcagacct agggaaggtg 800

aggcgaggtg gggagttggc agaatcccca tacctcgcag atttgctgag 850

tctgtcttgt gcagagggcc agagaatggc ttatgggggc ccaggttgga 900

tggggaaagg ctaatggggt cagaccccac cccgtctacc cctccagtca 950

gcccagcgcc catcctgcag ctcagctggg agcatcattc tcctgctttg 1000

tacatagggt gtggtcccct ggcacgtggc caccatcatg tctaggccta 1050

tgctaggagg caaatggcca ggctctgcct gtgtttttct caacactact 1100

tttctgatat gagggcagca cctgcctctg aatgggaaat catgcaacta 1150

ctcagaatgt gtcctcctca tctaatgctc atctgtttaa tggtgatgcc 1200
```

tcgcgtacag gatctggtta cctgtgcagt tgtgaatacc cagaggttgg 1250

gcagatcagt gtctctagtc ctacccagtt ttaaagttca tggtaagatt 1300

tgacctcatc tcccgcaaat aaatgtattg gtgatttgga aaaaaaaaaa 1350

aaaaaa 1356

<210> 44
<211> 171
<212> PRT
<213> Homo Sapien

<400> 44
Met Met Met Gln Cys Val Ser Arg Met Leu Ala His Pro Leu His
1 5 10 15

Val Ile Ser Met Arg Cys Met Val Gln Phe Val Gly Arg Glu Ala
20 25 30

Lys Tyr Ser Gly Val Leu Ser Ser Ile Gly Lys Ile Phe Lys Glu
35 40 45

Glu Gly Leu Leu Gly Phe Phe Val Gly Leu Ile Pro His Leu Leu
50 55 60

Gly Asp Val Val Phe Leu Trp Gly Cys Asn Leu Leu Ala His Phe
65 70 75

Ile Asn Ala Tyr Leu Val Asp Asp Ser Phe Ser Gln Ala Leu Ala
80 85 90

Ile Arg Ser Tyr Thr Lys Phe Val Met Gly Ile Ala Val Ser Met
95 100 105

Leu Thr Tyr Pro Phe Leu Leu Val Gly Asp Leu Met Ala Val Asn
110 115 120

Asn Cys Gly Leu Gln Ala Gly Leu Pro Pro Tyr Ser Pro Val Phe
125 130 135

Lys Ser Trp Ile His Cys Trp Lys Tyr Leu Ser Val Gln Gly Gln
140 145 150

Leu Phe Arg Gly Ser Ser Leu Leu Phe Arg Arg Val Ser Ser Gly
155 160 165

Ser Cys Phe Ala Leu Glu
170

<210> 45
<211> 2237
<212> DNA
<213> Homo Sapien

<400> 45
gctcactctt tgggtccaca ctgcctttat gagctgtaac actcactggg 50

aatgtctgca gcttcactcc tgaagccagc gagaccacga acccaccagg 100

aggaacaaac aactccagac gcgcagcctt aagagctgta acactcaccg 150

cgaaggtctg cagcttcact cctgagccag ccagaccacg aacccaccag 200

aaggaagaaa ctccaaacac atccgaacat cagaaggagc aaactcgtga 250
cacgccacct ttaagaaccg tgacactcaa cgctagggtc cgcggcttca 300
ttcttgaagt cagtgagacc aagaacccac caattccgga cacggcaaag 350
taacatccta gacatggctt tagagatcca catgtcagac cccatgtgcc 400
tcatcgagaa ctttaatgag cagctgaagg ttaatcagga agctttggag 450
atcctgtctg ccattacgca acctgtagtt gtggtagcga ttgtgggcct 500
ctatcgcact ggcaaatcct acctgatgaa caagctggct gggaagaaca 550
agggcttctc tgttgcatct acggtgcagt ctcacaccaa gggaatttgg 600
atatggtgtg tgcctcatcc caactggcca aatcacacat tagttctgct 650
tgacaccgag ggcctgggag atgtagagaa ggctgacaac aagaatgata 700
tccagatctt tgcactggca ctcttactga gcagcacctt tgtgtacaat 750
actgtgaaca aaattgatca gggtgctatc gacctactgc acaatgtgac 800
agaactgaca gatctgctca aggcaagaaa ctcacctgac cttgacaggg 850
ttgaagatcc tgctgactct gcgagcttct tcccagactt agtgtggact 900
ctgagagatt tctgcttagg cctggaaata gatgggcaac ttgtcacacc 950
agatgaatac ctggagaatt ccctaaggcc aaagcaaggt agtgatcaaa 1000
gagttcaaaa tttcaatttg ccccgtctgt gtatacagaa gttctttcca 1050
aaaaagaaat gctttatctt tgacttacct gctcaccaaa aaaagcttgc 1100
ccaacttgaa acactgcctg atgatgagct agagcctgaa tttgtgcaac 1150
aagtgacaga attctgttcc tacatcttta gccattctat gaccaagact 1200
cttccaggtg gcatcatggt caatggatct cgtctaaaga acctggtgct 1250
gacctatgtc aatgccatca gcagtgggga tctgccttgc atagagaatg 1300
cagtcctggc cttggctcag agagagaact cagctgcagt gcaaaaggcc 1350
attgcccact atgaccagca aatgggccag aaagtgcagc tgcccatgga 1400
aaccctccag gagctgctgg acctgcacag gaccagtgag agggaggcca 1450
ttgaagtctt catgaaaaac tctttcaagg atgtagacca aagtttccag 1500
aaagaattgg agactctact agatgcaaaa cagaatgaca tttgtaaacg 1550
gaacctggaa gcatcctcgg attattgctc ggctttactt aaggatattt 1600
ttggtcctct agaagaagca gtgaagcagg gaatttattc taagccagga 1650
ggccataatc tcttcattca gaaaacagaa gaactgaagg caaagtacta 1700
tcgggagcct cggaaaggaa tacaggctga agaagttctg cagaaatatt 1750
taaagtccaa ggagtctgtg agtcatgcaa tattacagac tgaccaggct 1800

ctcacagaga cggaaaaaaa gaagaaagag gcacaagtga aagcagaagc 1850

tgaaaaggct gaagcgcaaa ggttggcggc gattcaaagg cagaacgagc 1900

aaatgatgca ggagagggag agactccatc aggaacaagt gagacaaatg 1950

gagatagcca aacaaaattg gctggcagag caacagaaaa tgcaggaaca 2000

acagatgcag gaacaggctg cacagctcag cacaacattc caagctcaaa 2050

atagaagcct tctcagtgag ctccagcacg cccagagggc tgttaataac 2100

gatgatccat gtgttttact ctaaagtgct aaatatggga gtttcctttt 2150

tttactcttt gtcactgatg acacaacaga aaagaaactg tagaccttgg 2200

gacaatcaac atttaaataa actttataat tattaaa 2237

<210> 46
<211> 586
<212> PRT
<213> Homo Sapien

<400> 46
Met Ala Leu Glu Ile His Met Ser Asp Pro Met Cys Leu Ile Glu
1 5 10 15

Asn Phe Asn Glu Gln Leu Lys Val Asn Gln Glu Ala Leu Glu Ile
20 25 30

Leu Ser Ala Ile Thr Gln Pro Val Val Val Val Ala Ile Val Gly
35 40 45

Leu Tyr Arg Thr Gly Lys Ser Tyr Leu Met Asn Lys Leu Ala Gly
50 55 60

Lys Asn Lys Gly Phe Ser Val Ala Ser Thr Val Gln Ser His Thr
65 70 75

Lys Gly Ile Trp Ile Trp Cys Val Pro His Pro Asn Trp Pro Asn
80 85 90

His Thr Leu Val Leu Leu Asp Thr Glu Gly Leu Gly Asp Val Glu
95 100 105

Lys Ala Asp Asn Lys Asn Asp Ile Gln Ile Phe Ala Leu Ala Leu
110 115 120

Leu Leu Ser Ser Thr Phe Val Tyr Asn Thr Val Asn Lys Ile Asp
125 130 135

Gln Gly Ala Ile Asp Leu Leu His Asn Val Thr Glu Leu Thr Asp
140 145 150

Leu Leu Lys Ala Arg Asn Ser Pro Asp Leu Asp Arg Val Glu Asp
155 160 165

Pro Ala Asp Ser Ala Ser Phe Phe Pro Asp Leu Val Trp Thr Leu
170 175 180

Arg Asp Phe Cys Leu Gly Leu Glu Ile Asp Gly Gln Leu Val Thr
185 190 195

Pro Asp Glu Tyr Leu Glu Asn Ser Leu Arg Pro Lys Gln Gly Ser

```
                200                 205                 210

Asp Gln Arg Val Gln Asn Phe Asn Leu Pro Arg Leu Cys Ile Gln
                215                 220                 225

Lys Phe Phe Pro Lys Lys Lys Cys Phe Ile Phe Asp Leu Pro Ala
                230                 235                 240

His Gln Lys Lys Leu Ala Gln Leu Glu Thr Leu Pro Asp Asp Glu
                245                 250                 255

Leu Glu Pro Glu Phe Val Gln Gln Val Thr Glu Phe Cys Ser Tyr
                260                 265                 270

Ile Phe Ser His Ser Met Thr Lys Thr Leu Pro Gly Gly Ile Met
                275                 280                 285

Val Asn Gly Ser Arg Leu Lys Asn Leu Val Leu Thr Tyr Val Asn
                290                 295                 300

Ala Ile Ser Ser Gly Asp Leu Pro Cys Ile Glu Asn Ala Val Leu
                305                 310                 315

Ala Leu Ala Gln Arg Glu Asn Ser Ala Ala Val Gln Lys Ala Ile
                320                 325                 330

Ala His Tyr Asp Gln Gln Met Gly Gln Lys Val Gln Leu Pro Met
                335                 340                 345

Glu Thr Leu Gln Glu Leu Leu Asp Leu His Arg Thr Ser Glu Arg
                350                 355                 360

Glu Ala Ile Glu Val Phe Met Lys Asn Ser Phe Lys Asp Val Asp
                365                 370                 375

Gln Ser Phe Gln Lys Glu Leu Glu Thr Leu Leu Asp Ala Lys Gln
                380                 385                 390

Asn Asp Ile Cys Lys Arg Asn Leu Glu Ala Ser Ser Asp Tyr Cys
                395                 400                 405

Ser Ala Leu Leu Lys Asp Ile Phe Gly Pro Leu Glu Glu Ala Val
                410                 415                 420

Lys Gln Gly Ile Tyr Ser Lys Pro Gly Gly His Asn Leu Phe Ile
                425                 430                 435

Gln Lys Thr Glu Glu Leu Lys Ala Lys Tyr Tyr Arg Glu Pro Arg
                440                 445                 450

Lys Gly Ile Gln Ala Glu Glu Val Leu Gln Lys Tyr Leu Lys Ser
                455                 460                 465

Lys Glu Ser Val Ser His Ala Ile Leu Gln Thr Asp Gln Ala Leu
                470                 475                 480

Thr Glu Thr Glu Lys Lys Lys Lys Glu Ala Gln Val Lys Ala Glu
                485                 490                 495

Ala Glu Lys Ala Glu Ala Gln Arg Leu Ala Ala Ile Gln Arg Gln
                500                 505                 510

Asn Glu Gln Met Met Gln Glu Arg Glu Arg Leu His Gln Glu Gln
                515                 520                 525
```

```
Val Arg Gln Met Glu Ile Ala Lys Gln Asn Trp Leu Ala Glu Gln
                530                 535                 540

Gln Lys Met Gln Glu Gln Gln Met Gln Glu Gln Ala Ala Gln Leu
                545                 550                 555

Ser Thr Thr Phe Gln Ala Gln Asn Arg Ser Leu Leu Ser Glu Leu
                560                 565                 570

Gln His Ala Gln Arg Ala Val Asn Asn Asp Asp Pro Cys Val Leu
                575                 580                 585

Leu


<210> 47
<211> 1808
<212> DNA
<213> Homo Sapien

<400> 47
cactcattca ttccaaaggg tctctcaagg caatggtaat gtgcaaggag 50

gtgataccta aatgaatgac caaaagaaca tgcttctgct tttgtgtgtc 100

tcctacattt tagacatttg tttgtttctc ttggtagcct ttaaattcct 150

tgaagcccag gaccatgtct cacttacctt tgtgtttcca ctaactagtc 200

tacctcctgg aattggcaga tactcagtga aagcctgtga aataagtgat 250

gtctatttct agcatattat tctgagattt aatgatagat ttagtgattg 300

aatgagattt ccattttcaa atacagcaaa agcataacta ttttcattca 350

ttcatattca ttcaacttca ttctcaaaat taggtcctga gttaactaat 400

aattaccttt gaaatgtgtg ggttatttga ggcaatcagg tggtgacatt 450

gagctctcag ccagagtttg tttctggaat tgattcagtt ccattgcatt 500

gatttttgtt ctcagaagcc aaggtttccc atgaaaaatc attcccactt 550

gaattgggct gtgattcttg ctgcgtttaa gtaaaggaag cctcttggtt 600

ctagttctgc aaacttacac actgaactgg gacaagtttt tgtttagagt 650

aatggctggg aaaagaggaa cctttcattt tattcagaag tcaaaaacaa 700

aggcctccca gccacctgga gatgttttgt tgcagacacc agcctggctc 750

tgtctttatg cctaacaatt gagcatccag tcttctttgt gctgggacca 800

ttgctcagct ctgcaagggg aaaagaggga gaaagccaga gctgccaggc 850

ttcttgcact ggggccgggg gagggttcct gggaagcagg tgctctctgg 900

cttcttggta cgtgaggctc tcggagctgc ctctcctctg accctcaggt 950

cctcaccgag tttgctccag gagtatattg aaaacatacc cagtgctctc 1000

tcaagcaccc actgcttaga gggcccagat ttcttttcct tctttccctt 1050
```

gcagagctgg agactgcatc gggcatctgg tgtttaaact aaacaggaaa 1100

actgactaaa ggtccacagt gctcattgtg tagactagct gccctccgat 1150

gggtgctctg attatcagtg gttccagtgc agggcctgtc actaaacagg 1200

cctcacttcc tccttggggg ctttcccatg ggaggtgtgg ctttttactc 1250

tacatggaaa tgactctctg cagccacaga acacagtcat tttctgaatt 1300

atcccagtct ctcatgcgcc ctggattcct ccagatgcct tatatctctt 1350

gtgcaaagtt gtctaaaatt tggttcccag cttccaagcc ttgccttttg 1400

gccttcctgg aagtattttt gttgatgagt cgtctgtcat tattctctaa 1450

aatgatttgc tttttgtttc tttcattcct atttccaccc cacatataca 1500

cacatgcttc ttaacttagg ggattacatg ccaataaatc tattgttgaa 1550

aatgcactaa tactatcgca aagacgaaaa ttcacaggct gaaccgttgt 1600

aagtccatat gctcctcaac ttacatgtgt gatggagtta tgcccaaata 1650

agtccatcgt caagttgaaa aatcaaaatc aagccatctt aggttgagga 1700

ccatttgttt gtacctccaa agatgtcata tctttaaaca tactccctag 1750

cttttctttt tactttttat tttgaagtaa ttatagaatc acagaaagtt 1800

gcaaaaaa 1808

<210> 48
<211> 121
<212> PRT
<213> Homo Sapien

<400> 48
Met Gly Ala Leu Ile Ile Ser Gly Ser Ser Ala Gly Pro Val Thr
1               5                   10                  15

Lys Gln Ala Ser Leu Pro Pro Trp Gly Leu Ser His Gly Arg Cys
                20                  25                  30

Gly Phe Leu Leu Tyr Met Glu Met Thr Leu Cys Ser His Arg Thr
                35                  40                  45

Gln Ser Phe Ser Glu Leu Ser Gln Ser Leu Met Arg Pro Gly Phe
                50                  55                  60

Leu Gln Met Pro Tyr Ile Ser Cys Ala Lys Leu Ser Lys Ile Trp
                65                  70                  75

Phe Pro Ala Ser Lys Pro Cys Leu Leu Ala Phe Leu Glu Val Phe
                80                  85                  90

Leu Leu Met Ser Arg Leu Ser Leu Phe Ser Lys Met Ile Cys Phe
                95                  100                 105

Leu Phe Leu Ser Phe Leu Phe Pro Pro His Ile Tyr Thr His Ala
                110                 115                 120

Ser

<210> 49
<211> 3719
<212> DNA
<213> Homo Sapien

<400> 49
ggcttctaca gtccacaaca cccaccagcc ccaggcccag cagaatgagc 50

ccagtgagtg ccggggctcc cagtttggct gttgctatga caacgtggcc 100

actgcagccg gtcctcttgg ggaaggctgt gtgggccagc ccagccatgc 150

ctaccccgtg cggtgcctgc tgcccagtgc ccatggctct tgtgcagact 200

gggctgcccg ctggtacttc gttgcctctg tgggccaatg taaccgcttc 250

tggtatggcg gctgccatgg caatgccaat aactttgcct cggagcaaga 300

gtgcatgagc agctgccagg gatctctcca tgggccccgt cgtccccagc 350

ctggggcttc tggaaggagc acccacacgg atggtggcgg cagcagtcct 400

gcaggcgagc aggaacccag ccagcacagg acaggggccg cggtgcagag 450

aaagccctgg ccttctggtg gtctctggcg gcaagaccaa cagcctgggc 500

caggggaggc cccccacacc caggcctttg gagaatggcc atgggggcag 550

gagcttgggt ccagggcccc tggactgggt ggagatgccg gatcaccagc 600

gccacccttc cacagctcct cctacagatc tcacttccca cctctccagg 650

attagcttgg caggtgtgga gccctcgttg gtgcaggcag ccctggggca 700

gttggtgcgg ctctcctgct cagacgacac tgccccggaa tcccaggctg 750

cctggcagaa agatggccag cccatctcct ctgacaggca caggctgcag 800

ttcgacggat ccctgatcat ccacccccctg caggcagagg acgcgggcac 850

ctacagctgt ggcagcaccc ggccaggccg cgactcccag aagatccaac 900

tccgcattat agggggtgac atggccgtgc tgtctgaggc tgagctgagc 950

cgcttccctc agcccaggga cccagctcag gactttggcc aagcgggggc 1000

tgctgggccc ctgggggcca tcccctcttc acacccacag cctgcaaaca 1050

ggctgcgttt ggaccagaac cagccccggg tggtggatgc cagtccaggc 1100

cagcggatcc ggatgacctg ccgtgccgaa ggcttcccgc ccccagccat 1150

cgagtggcag agagatgggc agcctgtctc ttctcccaga caccagctgc 1200

agcctgatgg ctccctggtc attagccgag tggctgtaga agatggcggc 1250

ttctacacct gtgtcgcttt caatgggcag gaccgagacc agcgatgggt 1300

ccagctcaga gttctggggg agctgacaat ctcaggactg ccccctactg 1350

tgacagtgcc agagggtgat acggccaggc tattgtgtgt ggtagcagga 1400

gaaagtgtga acatcaggtg gtccaggaac gggctacctg tgcaggctga 1450

tggccaccgt gtccaccagt ccccagatgg cacgctgctc atttacaact 1500
tgcgggccag ggatgagggc tcctacatgt gcagtgccta ccaggggagc 1550
caggcagtca gccgcagcac cgaggtgaag gtggtctcac cagcacccac 1600
cgcccagccc agggaccctg gcagggactg cgtcgaccag ccagagctgg 1650
ccaactgtga tttgatcctg caggcccagc tttgtggcaa tgagtattac 1700
tccagcttct gctgtgccag ctgttcacgt ttccagcctc acgctcagcc 1750
catctggcag tagggatgaa ggctagttcc agccccagtc caaaatagtt 1800
catagggcta gggagaaagg aagatggact cttggcttcc tctctctggc 1850
tggcaaaggg agttatcttc tggaatacat tagctctttc aaaaacccac 1900
ccagtgttta gcctcaacgg cagccagtta ccagcttctc tctgtagcct 1950
tcagcagtgt ttgcatctct gacataacca caggctgctg ttttcaagaa 2000
gagcaatctg tttggataag aaaaaccttt actttacagc ttccctttat 2050
aatttgttac acaggaatag ttaaatgcat ttgtttgttt gtttttgag 2100
acggagtttc actcttgttg cccaggctgg agggcaatgg cgcgatctca 2150
gctcactgca acctccgtct cctgggttct tgattctcct gtgtcagcct 2200
tctgagtagc tgggattaca gatgcctatc accatgcctg ggtaattttt 2250
gtatttttag ttgagatggg gtttcgccat gttggccagg ctggtctcga 2300
acttctgacc tcagatgatc tgcccgcctc agcctcccaa agtgctggga 2350
ttacaggcat gagccaccac gcccagccat caatgcattt tttttatttt 2400
ttttttgaga cagagtttcg cacttcttgc ccaggctgga gtacaatggt 2450
gcgatcttgg ctcactgcaa cctccacctc ctgggttcaa gcgcttctcc 2500
agcctcagcc tcctgagtag ctgggattac aggtatgtgc caccatgcct 2550
ggctaatttt gtatttttgg tggagacggg gtttctccat gttggtcaga 2600
ctggtcttga actcccgacc tcaggtaatc cgcccgcctc cgcctcccaa 2650
aatgctggga ttagaggtgt gagccactgt gcccagccca tcaatgtgtt 2700
ttaaagctag ctgtcagggt tccacttaat ttaaagctgg gcagggagat 2750
gtgtaatgat ttcaaagtta acacctgttt gttttctaaa gggcatgcca 2800
agtcctgctg tatcagggaa gtattctgtg ctaaaatcag cgatggttca 2850
ttgctctagt ctctctcacc cttctaggca gtgcatcagt cagctctaaa 2900
tctggtgcag agggttaaca gcataaccct tgttggcaaa atggaataga 2950
tgttaagacc tcaaataggg atttgggatg aaacagctgc agttagcact 3000
gttatctgag catgaaagaa ctggaaacgc tccttacgtc gagatgttgg 3050

accttgaagc cctcctgagg ccaacatgca aatctggctg tgacggttca 3100

tctgacacct gtgtaaagct gaccagcctg ctctgtacag tgacaatgag 3150

gagcccctct cttccttaag taggaatctg tgaagcaaaa tgtttgctgc 3200

caaagacaaa tcagactgtc agtcattaaa aacagcatta gcaggatgag 3250

gatagcaatg gggaagggtt gtgggcaatg cagtaacagg gaaatggctt 3300

cagaaatggt ttgagttgga agacaacatt cttcatctct caggacttct 3350

aattccttga tgctaaaaga agaggcatgg attctatgag cttccaagtc 3400

cctttccact ttaaccttct acaaatcttt cagaggactg cctagtagca 3450

aaggttattc ctggacacag gaaagacggg cattacaggg accaaagctc 3500

tgaaaggtga cttttattac caacacactg gctggaaaag ggacaaacca 3550

catcacgggt gagtgatact tctcagtctt ctctactcat tcaacaaagg 3600

aaatgtgggc tggggcagag gtcttttttc atttaatact ggaaaaatat 3650

tgaagagcat ccatgttcac ttatggctgg ttttgctata gaaattggaa 3700

aataaaggcc actttttttg 3719

<210> 50
<211> 477
<212> PRT
<213> Homo Sapien

<400> 50
Met Gly Pro Val Val Pro Ser Leu Gly Leu Leu Glu Gly Ala Pro
1 5 10 15

Thr Arg Met Val Ala Ala Ala Val Leu Gln Ala Ser Arg Asn Pro
20 25 30

Ala Ser Thr Gly Gln Gly Pro Arg Cys Arg Glu Ser Pro Gly Leu
35 40 45

Leu Val Val Ser Gly Gly Lys Thr Asn Ser Leu Gly Gln Gly Arg
50 55 60

Pro Pro Thr Pro Arg Pro Leu Glu Asn Gly His Gly Gly Arg Ser
65 70 75

Leu Gly Pro Gly Pro Leu Asp Trp Val Glu Met Pro Asp His Gln
80 85 90

Arg His Pro Ser Thr Ala Pro Pro Thr Asp Leu Thr Ser His Leu
95 100 105

Ser Arg Ile Ser Leu Ala Gly Val Glu Pro Ser Leu Val Gln Ala
110 115 120

Ala Leu Gly Gln Leu Val Arg Leu Ser Cys Ser Asp Asp Thr Ala
125 130 135

Pro Glu Ser Gln Ala Ala Trp Gln Lys Asp Gly Gln Pro Ile Ser
140 145 150

```
Ser Asp Arg His Arg Leu Gln Phe Asp Gly Ser Leu Ile Ile His
                155                 160                 165

Pro Leu Gln Ala Glu Asp Ala Gly Thr Tyr Ser Cys Gly Ser Thr
                170                 175                 180

Arg Pro Gly Arg Asp Ser Gln Lys Ile Gln Leu Arg Ile Ile Gly
                185                 190                 195

Gly Asp Met Ala Val Leu Ser Glu Ala Glu Leu Ser Arg Phe Pro
                200                 205                 210

Gln Pro Arg Asp Pro Ala Gln Asp Phe Gly Gln Ala Gly Ala Ala
                215                 220                 225

Gly Pro Leu Gly Ala Ile Pro Ser Ser His Pro Gln Pro Ala Asn
                230                 235                 240

Arg Leu Arg Leu Asp Gln Asn Gln Pro Arg Val Val Asp Ala Ser
                245                 250                 255

Pro Gly Gln Arg Ile Arg Met Thr Cys Arg Ala Glu Gly Phe Pro
                260                 265                 270

Pro Pro Ala Ile Glu Trp Gln Arg Asp Gly Gln Pro Val Ser Ser
                275                 280                 285

Pro Arg His Gln Leu Gln Pro Asp Gly Ser Leu Val Ile Ser Arg
                290                 295                 300

Val Ala Val Glu Asp Gly Gly Phe Tyr Thr Cys Val Ala Phe Asn
                305                 310                 315

Gly Gln Asp Arg Asp Gln Arg Trp Val Gln Leu Arg Val Leu Gly
                320                 325                 330

Glu Leu Thr Ile Ser Gly Leu Pro Pro Thr Val Thr Val Pro Glu
                335                 340                 345

Gly Asp Thr Ala Arg Leu Leu Cys Val Val Ala Gly Glu Ser Val
                350                 355                 360

Asn Ile Arg Trp Ser Arg Asn Gly Leu Pro Val Gln Ala Asp Gly
                365                 370                 375

His Arg Val His Gln Ser Pro Asp Gly Thr Leu Leu Ile Tyr Asn
                380                 385                 390

Leu Arg Ala Arg Asp Glu Gly Ser Tyr Met Cys Ser Ala Tyr Gln
                395                 400                 405

Gly Ser Gln Ala Val Ser Arg Ser Thr Glu Val Lys Val Val Ser
                410                 415                 420

Pro Ala Pro Thr Ala Gln Pro Arg Asp Pro Gly Arg Asp Cys Val
                425                 430                 435

Asp Gln Pro Glu Leu Ala Asn Cys Asp Leu Ile Leu Gln Ala Gln
                440                 445                 450

Leu Cys Gly Asn Glu Tyr Tyr Ser Ser Phe Cys Cys Ala Ser Cys
                455                 460                 465
```

```
Ser Arg Phe Gln Pro His Ala Gln Pro Ile Trp Gln
                470                 475

<210> 51
<211> 2014
<212> DNA
<213> Homo Sapien

<400> 51
caggcagaag cgaacaaaga cccagcaaga gaaggcagag gctaagaccc 50

atcccgtatc tgctctcctg aaataattct ggagtcatgc ctgaaatgcc 100

agaggacatg gagcaggagg aagttaacat ccctaatagg agggttctgg 150

ttactggtgc cactgggctt cttggcagag ctgtacacaa agaatttcag 200

cagaataatt ggcatgcagt tggctgtggt ttcagaagag caagaccaaa 250

atttgaacag gttaatctgt tggattctaa tgcagttcat cacatcattc 300

atgattttca gccccatgtt atagtacatt gtgcagcaga gagaagacca 350

gatgttgtag aaaatcagcc agatgctgcc tctcaactta atgtggatgc 400

ttctgggaat ttagcaaagg aagcagctgc tgttggagca tttctcatct 450

acattagctc agattatgta tttgatggaa caaatccacc ttacagagag 500

gaagacatac cagctcccct aaatttgtat ggcaaaacaa aattagatgg 550

agaaaaggct gtcctggaga acaatctagg agctgctgtt ttgaggattc 600

ctattctgta tggggaagtt gaaaagctcg aagaaagtgc tgtgactgtt 650

atgtttgata aagtgcagtt cagcaacaag tcagcaaaca tggatcactg 700

gcagcagagg ttccccacac atgtcaaaga tgtggccact gtgtgccggc 750

agctagcaga gaagagaatg ctggatccat caattaaggg aacctttcac 800

tggtctggca atgaacagat gactaagtat gaaatggcat gtgcaattgc 850

agatgccttc aacctcccca gcagtcactt aagacctatt actgacagcc 900

ctgtcctagg agcacaacgt ccgagaaatg ctcagcttga ctgctccaaa 950

ttggagacct tgggcattgg ccaacgaaca ccatttcgaa ttggaatcaa 1000

agaatcactt tggcctttcc tcattgacaa gagatggaga caaacggtct 1050

ttcattagtt tatttgtgtt gggttctttt ttttttaaa tgaaaagtat 1100

agtatgtggc actttttaaa gaacaaagga aatagttttg tatgagtact 1150

ttaattgtga ctcttaggat ctttcaggta aatgatgctc ttgcactagt 1200

gaaattgtct aaagaaacta aagggcagtc atgccctgtt tgcagtaatt 1250

tttcttttta tcattttgtt tgtcctggct aaacttggag tttgagtata 1300

gtaaattatg atccttaaat atttgagagt caggatgaag cagatctgct 1350

gtagactttt cagatgaaat tgttcattct cgtaacctcc atattttcag 1400
```

gatttttgaa gctgttgacc ttttcatgtt gattatttta aattgtgtga 1450

aatagtataa aaatcattgg tgttcattat ttgctttgcc tgagctcaga 1500

tcaaaatgtt tgaagaaagg aactttattt ttgcaagtta cgtacagttt 1550

ttatgcttga gatatttcaa catgttatgt atattggaac ttctacagct 1600

tgatgcctcc tgcttttata gcagtttatg gggagcactt gaaagagcgt 1650

gtgtacatgt atttttttc taggcaaaca ttgaatgcaa acgtgtattt 1700

ttttaatata aatatataac tgtccttttc atcccatgtt gccgctaagt 1750

gatatttcat atgtgtggtt atactcataa taatgggcct tgtaagtctt 1800

ttcaccattc atgaataata ataaatatgt actgctggca tgtaatgctt 1850

agttttcttg tatttacttc ttttttaaa tgtaaggacc aaacttctaa 1900

actaattgtt cttttgttgc tttaattttt aaaaattaca ttcttctgat 1950

gtaacatgtg atacatacaa aagaatatag tttaatatgt attgaaataa 2000

aacacaataa aatt 2014

<210> 52
<211> 323
<212> PRT
<213> Homo Sapien

<400> 52
Met Pro Glu Met Pro Glu Asp Met Glu Gln Glu Glu Val Asn Ile
1 5 10 15

Pro Asn Arg Arg Val Leu Val Thr Gly Ala Thr Gly Leu Leu Gly
20 25 30

Arg Ala Val His Lys Glu Phe Gln Gln Asn Asn Trp His Ala Val
35 40 45

Gly Cys Gly Phe Arg Arg Ala Arg Pro Lys Phe Glu Gln Val Asn
50 55 60

Leu Leu Asp Ser Asn Ala Val His His Ile Ile His Asp Phe Gln
65 70 75

Pro His Val Ile Val His Cys Ala Ala Glu Arg Arg Pro Asp Val
80 85 90

Val Glu Asn Gln Pro Asp Ala Ala Ser Gln Leu Asn Val Asp Ala
95 100 105

Ser Gly Asn Leu Ala Lys Glu Ala Ala Ala Val Gly Ala Phe Leu
110 115 120

Ile Tyr Ile Ser Ser Asp Tyr Val Phe Asp Gly Thr Asn Pro Pro
125 130 135

Tyr Arg Glu Glu Asp Ile Pro Ala Pro Leu Asn Leu Tyr Gly Lys
140 145 150

Thr Lys Leu Asp Gly Glu Lys Ala Val Leu Glu Asn Asn Leu Gly

155 160 165

Ala Ala Val Leu Arg Ile Pro Ile Leu Tyr Gly Glu Val Glu Lys
170 175 180

Leu Glu Glu Ser Ala Val Thr Val Met Phe Asp Lys Val Gln Phe
185 190 195

Ser Asn Lys Ser Ala Asn Met Asp His Trp Gln Gln Arg Phe Pro
200 205 210

Thr His Val Lys Asp Val Ala Thr Val Cys Arg Gln Leu Ala Glu
215 220 225

Lys Arg Met Leu Asp Pro Ser Ile Lys Gly Thr Phe His Trp Ser
230 235 240

Gly Asn Glu Gln Met Thr Lys Tyr Glu Met Ala Cys Ala Ile Ala
245 250 255

Asp Ala Phe Asn Leu Pro Ser Ser His Leu Arg Pro Ile Thr Asp
260 265 270

Ser Pro Val Leu Gly Ala Gln Arg Pro Arg Asn Ala Gln Leu Asp
275 280 285

Cys Ser Lys Leu Glu Thr Leu Gly Ile Gly Gln Arg Thr Pro Phe
290 295 300

Arg Ile Gly Ile Lys Glu Ser Leu Trp Pro Phe Leu Ile Asp Lys
305 310 315

Arg Trp Arg Gln Thr Val Phe His
320

<210> 53
<211> 4372
<212> DNA
<213> Homo Sapien

<400> 53
tgggctccct ccagcactgc tgttgcctgc tgcctaagat gggtgacact 50

tgggcccagc ttccctggcc tgggccaccc cacccagcaa tgctgctgat 100

ctccctcctc ttggcagccg ggttgatgca ctcggatgcc ggcaccagct 150

gccccgtcct ttgcacatgc cgtaaccagg tggtggattg tagcagccag 200

cggctattct ccgtgccccc agacctgcca atggacaccc gaaacctcag 250

cctggcccac aaccgcatca cagcagtgcc gcctggctac ctcacatgct 300

acatggagct ccaggtgctg gatttgcaca acaactcctt aatggagctg 350

ccccggggcc tcttcctcca tgccaagcgc ttggcacact tggacctgag 400

ctacaacaat ttcagccatg tgccagccga catgttccag gaggcccatg 450

ggctagtcca catcgacctg agccacaacc cctggctgcg gagggtgcat 500

ccccaggcct ttcagggcct catgcagctc cgagacctgg acctcagtta 550

tgggggcctg gccttcctca gcctggaggc tcttgagggc ctaccggggc 600

tggtgaccct gcagatcggt ggcaatccct gggtgtgtgg ctgcaccatg 650
gaacccctgc tgaagtggct gcgaaaccgg atccagcgct gtacagcaga 700
ttctcagctg gctgagtgcc ggggccctcc tgaagtcgag ggcgccccgc 750
tcttctcact cactgaggag agcttcaagg cctgccacct gaccctgacc 800
ctggatgatt acctattcat tgcgttcgtg ggcttcgtgg tctccattgc 850
ttctgtggcc accaacttcc tcctgggcat cactgccaac tgctgccacc 900
gctggagcaa ggccagtgaa gaggaagaga tctgacatgc ctgcctctca 950
tccctccatg ctgctgaccg ccacagctgc tggccaccag acgccctccc 1000
tgattgctca ctctggttcc atggtgacct ggctgcctca gtcatggttc 1050
aagcaaggtg gggacactca ttttgtatga gcatctgctt tgggccaggc 1100
ggcacgctag gaattgggaa catcagatga actgactcag tccctgccct 1150
caaggcactt ccctctggtc aaggagagag atccaaaaac tattcccttt 1200
aagactatat gtcaggactc tgagcacgtc attatggagg cccagaggag 1250
gagccatcat ctgtatctag caatgtccat gagaattata agattagagt 1300
gatttgtgaa ctgggtcatc aggaaatatc tactttgtca ggtaggcaaa 1350
gaagggtgtc tgcacatggc agaggccaga atatgcatag tgtgctgtgt 1400
tgagaagagt gaacagttcc tggtcactta cttgtataga gggggtgtgg 1450
cacagaactc aaacctaccc ctcacctcct gacaccaaaa ctgtcagctc 1500
tcagcaatgc cagccactgc ctacagggag taagaacacc tctatgacag 1550
cccctggcct ccttccacca gcagctacca ggtgagacca cctcccagtg 1600
actgccccca tatgaccaaa tgtcaccagt tggtgaggtc ccaggcagca 1650
ggctgaggat ggacactttc aatgcccttg ctcctgcctc tcactcaagt 1700
tttgcttcag aagagagagg caggaggccc agcaactggg gcagcaagag 1750
tcctggcacc ttgggatcct aatcatgtga ctgttcttgc cacagtgctc 1800
atgccacagg gtctcaccag gaaagtgcac tgtgggccac agacccacag 1850
cctggcagca cccagagcta aaaggggaca aaggcagcac agttatgacc 1900
atatgaggct ttgcattttc ttctaagcaa cttacccacg ttaagcatga 1950
gggtgagaga gctattaaat actaagccct tgccagtgtc aggtactttg 2000
aaaagctctc tgcacaaacc attccctttg acacacacac acacaaatct 2050
tttgaggtga acgctgttgt tcccatttta cggatgaggc aactaaggct 2100
cagagaggtt aaagtcacat gccactatga gcaagataaa gtctgtgctc 2150
tttctactgc cccatccaag ttggggaaca tcaccattcc ctctagagtt 2200

atataaattc aaattcaact agagctgaca aagttcctca taaggtccag 2250
gcactcctct gggcactttt atatctattg actcacttct ttcaattctc 2300
acagcaacac tgcctggtgg tttttattat ccccatttga cagatgaatt 2350
aatcgtagag agttgagtga cttacccaag gttgtctgga taagccctag 2400
aaggaaggcg gtaggcagct ccattcaggg aaactgcatc taatcagtca 2450
gtcaaaaatc aagtaacttt acgagcaaag cacaattatc atcatcgtgg 2500
tcttcttcat cagtttcgtc agcagcatca ttatcttccc tctatttgtt 2550
cagcaccgga tagttcatga gtatttttgc atcattctcc ttgacttttc 2600
acatccctgt gcaggaggta aatcaaacat cagtaatcct gttttacaga 2650
tggggaaaaa agtctcaagg ttggatatga cttgctatgt ggcaaggttg 2700
gggctcaacc ctaacacagt tctctttcca gtgctttctc aagtgcttgg 2750
ggaagagaat gcctcagaag gctgggtagt ggggccctgg aattcagcat 2800
ccatgaatgt gctagtggat aagctaaata gaaggcagcc aaacccatct 2850
gctgtacaga ttgaactatg ctcacggtag ggcaaattgc aggctctgaa 2900
acagagacta cacaggtaac acctgaatag gagactcctg ctttacaatg 2950
tgtagataaa acatcagcaa tggtggccat ggtggcagtc atgtgaaaag 3000
taagatcttt gggaatcaag aaaggaagct gtgttaacca ctcctgctca 3050
agccctgctg cgtgtgttgc aagagatact aagagagcaa gaaagctata 3100
ggtgagaacc tctgcagttt aggagaagaa catcaaggca cagtccaaca 3150
tgctgataag tctggccagg aggagaatta aaacaggggc tttccacacc 3200
tcccttgccc caagctccag cggtattcta tcagcccatc ctcctggaaa 3250
gcctgaaagg aatgaaggag gctaataagt catcttccag gaaggcatcc 3300
ctcactcgtg cttccctgag ctagtcaacc aaaagagtct tcagaaactt 3350
tgctagacct gaagtacttg aacctgtgtc ccctgaatct ttcttacaac 3400
atctgggaca aatccctggt cctgtgacat ccgaagcaga actgtgccct 3450
gctctctcct tctgtgatga ccaaggatgg tgaactcaag ttgttctcta 3500
caagccaggc cagcaaccta aatacttgga gaggaacttt tagaaactat 3550
aatcctgaca aaatagaaaa gtttcccata ggggcatacc ataatactat 3600
aataacctcc caggaactat tgtttgccaa aatgtagtta atatatttta 3650
agatatatgc ttttttgcat aggactagaa ccagaaaaga caccaaatgc 3700
ccccttgaca tcaatgtcct ttctagtggg acaatttggt ctccattaat 3750
gccaaacctt tctgaacagg atacatggct tttaaaggac agatgtttct 3800

cctgctgcta gaagttcctc agtttactag agcacaatga ggaaagtatt 3850

caacctccct actgccaagg aattccctgc ttctccccca ccgccatcat 3900

cttgtccaag ctatcagaag caaccttcta gagataatct aacaatcctg 3950

attagaattg ctcccatatc cctggtgacc acaggcttca ttcaaattgt 4000

ccaaactggt taacatgtat gtgatggggt atctctgcat ctgtatgtct 4050

gtctgcgagg ttccttgtat attggctgtc cgctgacttg ggacagatct 4100

ctctagaact tgggttcagt tctctgacat agtccactca gccataggct 4150

gagtggctaa atatgcataa ataagcatgc ctaaataggc atatataggt 4200

tggtgcaaaa gtaattgcgg tttttgccat taaaaatgat ggcaaaaatc 4250

ccaattactt ttgcgtcaat ctaatattac attgcttgat agattaagat 4300

ggaatcccac caggtttagg gtaggactgg atgctcaaaa aaaaaaaaaa 4350

aaaaaaaaaa aaaaaaaaaa aa 4372

<210> 54
<211> 281
<212> PRT
<213> Homo Sapien

<400> 54

```
Met Leu Leu Ile Ser Leu Leu Leu Ala Ala Gly Leu Met His Ser
 1               5                  10                  15

Asp Ala Gly Thr Ser Cys Pro Val Leu Cys Thr Cys Arg Asn Gln
                20                  25                  30

Val Val Asp Cys Ser Ser Gln Arg Leu Phe Ser Val Pro Pro Asp
                35                  40                  45

Leu Pro Met Asp Thr Arg Asn Leu Ser Leu Ala His Asn Arg Ile
                50                  55                  60

Thr Ala Val Pro Pro Gly Tyr Leu Thr Cys Tyr Met Glu Leu Gln
                65                  70                  75

Val Leu Asp Leu His Asn Asn Ser Leu Met Glu Leu Pro Arg Gly
                80                  85                  90

Leu Phe Leu His Ala Lys Arg Leu Ala His Leu Asp Leu Ser Tyr
                95                 100                 105

Asn Asn Phe Ser His Val Pro Ala Asp Met Phe Gln Glu Ala His
               110                 115                 120

Gly Leu Val His Ile Asp Leu Ser His Asn Pro Trp Leu Arg Arg
               125                 130                 135

Val His Pro Gln Ala Phe Gln Gly Leu Met Gln Leu Arg Asp Leu
               140                 145                 150

Asp Leu Ser Tyr Gly Gly Leu Ala Phe Leu Ser Leu Glu Ala Leu
               155                 160                 165
```

```
Glu Gly Leu Pro Gly Leu Val Thr Leu Gln Ile Gly Gly Asn Pro
                170                 175                 180

Trp Val Cys Gly Cys Thr Met Glu Pro Leu Leu Lys Trp Leu Arg
                185                 190                 195

Asn Arg Ile Gln Arg Cys Thr Ala Asp Ser Gln Leu Ala Glu Cys
                200                 205                 210

Arg Gly Pro Pro Glu Val Glu Gly Ala Pro Leu Phe Ser Leu Thr
                215                 220                 225

Glu Glu Ser Phe Lys Ala Cys His Leu Thr Leu Thr Leu Asp Asp
                230                 235                 240

Tyr Leu Phe Ile Ala Phe Val Gly Phe Val Val Ser Ile Ala Ser
                245                 250                 255

Val Ala Thr Asn Phe Leu Leu Gly Ile Thr Ala Asn Cys Cys His
                260                 265                 270

Arg Trp Ser Lys Ala Ser Glu Glu Glu Glu Ile
                275                 280
```

<210> 55
<211> 2737
<212> DNA
<213> Homo Sapien

<400> 55

```
ggctgcgccc aggccggcgg gcccagcagc tgcgaaccgc cggcgcacca 50

cctgtttccg cgcccgggga cttccccggc ggggctcaga agtgtggggt 100

cggtcgcttg gcttcccctg gcgtcagcga cccagggtaa cctcctccac 150

tgctgcgtgc cgtgcaggcc tgcctgtgtg agagccacgt gtgccgcgct 200

ctgggcacag ccttggaaag tcaggaccgc gacggcagca gagcagaaac 250

cttacagaaa catgaagccc tcaaccatct gctactcagt tattcggggc 300

tgacggcggc ttctagaaca tccaggtgtt ctgcagatgc gagaactcat 350

cctgtagtca ccagatggag tcccaaacag ccaagcagat gtaaggcctg 400

tgctgtggct ctgaggccct gaatacagaa gggtcacttt cttagtggcc 450

aaagagcagt tgttgacatt gatgtctaat tattgaacac gaccagtcat 500

tttactgagc tgcagtgagg aaacactgac catagaagat caagccaaat 550

gagggattgc aaatttcctg attcttttga attaggattc cagatggggg 600

cctcatttct acagccccca acattcctat agccgttatc actgccatca 650

ccactgccac cagcatcttc ttgcagattc cacccctgct ccccagagac 700

ttcctgcttt gaaagtgagc agaaaggaag ctctcagaaa aatctctagt 750

ggtggctgcc gtcgctccag acaatcggaa tcctgccttc accaccatgg 800

gctggctttt tctaaaggtt ttgttggcgg gagtgagttt ctcaggattt 850
```

ctttatcctc ttgtggattt ttgcatcagt gggaaaacaa gaggacagaa 900
gccaaacttt gtgattattt tggccgatga catggggtgg ggtgacctgg 950
gagcaaactg ggcagaaaca aaggacactg ccaaccttga taagatggct 1000
tcggagggaa tgaggtttgt ggatttccat gcagctgcct ccacctgctc 1050
accctcccgg gcttccttgc tcaccggccg gcttggcctt cgcaatggag 1100
tcacacgcaa ctttgcagtc acttctgtgg gaggccttcc gctcaacgag 1150
accaccttgg cagaggtgct gcagcaggcg ggttacgtca ctgggataat 1200
aggcaaatgg catcttggac accacggctc ttatcacccc aacttccgtg 1250
gttttgatta ctactttgga atcccatata gccatgatat gggctgtact 1300
gatactccag gctacaacca ccctccttgt ccagcgtgtc cacagggtga 1350
tggaccatca aggaaccttc aaagagactg ttacactgac gtggccctcc 1400
ctctttatga aaacctcaac attgtggagc agccggtgaa cttgagcagc 1450
cttgcccaga agtatgctga gaaagcaacc cagttcatcc agcgtgcaag 1500
caccagcggg aggcccttcc tgctctatgt ggctctggcc cacatgcacg 1550
tgcccttacc tgtgactcag ctaccagcag cgccacgggg cagaagcctg 1600
tatggtgcag ggctctggga gatggacagt ctggtgggcc agatcaagga 1650
caaagttgac cacacagtga aggaaaacac attcctctgg tttacaggag 1700
acaatggccc gtgggctcag aagtgtgagc tagcgggcag tgtgggtccc 1750
ttcactggat tttggcaaac tcgtcaaggg ggaagtccag ccaagcagac 1800
gacctgggaa ggagggcacc gggtcccagc actggcttac tggcctggca 1850
gagttccagt taatgtcacc agcactgcct tgttaagcgt gctggacatt 1900
tttccaactg tggtagccct ggcccaggcc agcttacctc aaggacggcg 1950
ctttgatggt gtggacgtct ccgaggtgct ctttggccgg tcacagcctg 2000
ggcacagggt gctgttccac cccaacagcg gggcagctgg agagtttgga 2050
gccctgcaga ctgtccgcct ggagcgttac aaggccttct acattaccgg 2100
tggagccagg gcgtgtgatg ggagcatggt gcctgagctg cagcataagt 2150
ttcctctgat tttcaacctg gaagacgata ccgcagaagc tgtgccccta 2200
gaaagaggtg gtgcggagta ccaggctgtg ctgcccgagg tcagaaaggt 2250
tcttgcagac gtcctccaag acattgccaa cgacaacatc tccagcgcag 2300
attacactca ggacccttca gtaactccct gctgtaatcc ctaccaaatt 2350
gcctgccgct gtcaagccgc ataacagacc aatttttatt ccacgaggag 2400
gagtacctgg aaattaggca agtttgcttc caaatttcat ttttaccctc 2450

tttacaaaca cacgctttag tttagtcttg gagtttagtt ttggagttag 2500

ccttgcatat cccttctgta tcctgtcccc cctccacgcc gacccgagag 2550

cagctgagct gcgctggctc tgggcaggga gtgtgcctta atgggaagca 2600

cacgggcttt ggagtcaggc acaggtgcca gctccagctt ttgaacttgg 2650

gcaattgttt aacctaacct gcaagttgat tttgagggtt aaataaaggc 2700

atacatgaaa atgcctggca actttaaaaa aaaaaaa 2737

<210> 56
<211> 525
<212> PRT
<213> Homo Sapien

<400> 56
Met Gly Trp Leu Phe Leu Lys Val Leu Leu Ala Gly Val Ser Phe
1 5 10 15

Ser Gly Phe Leu Tyr Pro Leu Val Asp Phe Cys Ile Ser Gly Lys
20 25 30

Thr Arg Gly Gln Lys Pro Asn Phe Val Ile Ile Leu Ala Asp Asp
35 40 45

Met Gly Trp Gly Asp Leu Gly Ala Asn Trp Ala Glu Thr Lys Asp
50 55 60

Thr Ala Asn Leu Asp Lys Met Ala Ser Glu Gly Met Arg Phe Val
65 70 75

Asp Phe His Ala Ala Ala Ser Thr Cys Ser Pro Ser Arg Ala Ser
80 85 90

Leu Leu Thr Gly Arg Leu Gly Leu Arg Asn Gly Val Thr Arg Asn
95 100 105

Phe Ala Val Thr Ser Val Gly Gly Leu Pro Leu Asn Glu Thr Thr
110 115 120

Leu Ala Glu Val Leu Gln Gln Ala Gly Tyr Val Thr Gly Ile Ile
125 130 135

Gly Lys Trp His Leu Gly His His Gly Ser Tyr His Pro Asn Phe
140 145 150

Arg Gly Phe Asp Tyr Tyr Phe Gly Ile Pro Tyr Ser His Asp Met
155 160 165

Gly Cys Thr Asp Thr Pro Gly Tyr Asn His Pro Pro Cys Pro Ala
170 175 180

Cys Pro Gln Gly Asp Gly Pro Ser Arg Asn Leu Gln Arg Asp Cys
185 190 195

Tyr Thr Asp Val Ala Leu Pro Leu Tyr Glu Asn Leu Asn Ile Val
200 205 210

Glu Gln Pro Val Asn Leu Ser Ser Leu Ala Gln Lys Tyr Ala Glu
215 220 225

Lys Ala Thr Gln Phe Ile Gln Arg Ala Ser Thr Ser Gly Arg Pro

```
                230                 235                 240

Phe Leu Leu Tyr Val Ala Leu Ala His Met His Val Pro Leu Pro
                245                 250                 255

Val Thr Gln Leu Pro Ala Ala Pro Arg Gly Arg Ser Leu Tyr Gly
                260                 265                 270

Ala Gly Leu Trp Glu Met Asp Ser Leu Val Gly Gln Ile Lys Asp
                275                 280                 285

Lys Val Asp His Thr Val Lys Glu Asn Thr Phe Leu Trp Phe Thr
                290                 295                 300

Gly Asp Asn Gly Pro Trp Ala Gln Lys Cys Glu Leu Ala Gly Ser
                305                 310                 315

Val Gly Pro Phe Thr Gly Phe Trp Gln Thr Arg Gln Gly Gly Ser
                320                 325                 330

Pro Ala Lys Gln Thr Thr Trp Glu Gly Gly His Arg Val Pro Ala
                335                 340                 345

Leu Ala Tyr Trp Pro Gly Arg Val Pro Val Asn Val Thr Ser Thr
                350                 355                 360

Ala Leu Leu Ser Val Leu Asp Ile Phe Pro Thr Val Val Ala Leu
                365                 370                 375

Ala Gln Ala Ser Leu Pro Gln Gly Arg Arg Phe Asp Gly Val Asp
                380                 385                 390

Val Ser Glu Val Leu Phe Gly Arg Ser Gln Pro Gly His Arg Val
                395                 400                 405

Leu Phe His Pro Asn Ser Gly Ala Ala Gly Glu Phe Gly Ala Leu
                410                 415                 420

Gln Thr Val Arg Leu Glu Arg Tyr Lys Ala Phe Tyr Ile Thr Gly
                425                 430                 435

Gly Ala Arg Ala Cys Asp Gly Ser Met Val Pro Glu Leu Gln His
                440                 445                 450

Lys Phe Pro Leu Ile Phe Asn Leu Glu Asp Asp Thr Ala Glu Ala
                455                 460                 465

Val Pro Leu Glu Arg Gly Gly Ala Glu Tyr Gln Ala Val Leu Pro
                470                 475                 480

Glu Val Arg Lys Val Leu Ala Asp Val Leu Gln Asp Ile Ala Asn
                485                 490                 495

Asp Asn Ile Ser Ser Ala Asp Tyr Thr Gln Asp Pro Ser Val Thr
                500                 505                 510

Pro Cys Cys Asn Pro Tyr Gln Ile Ala Cys Arg Cys Gln Ala Ala
                515                 520                 525
```

<210> 57
<211> 2443
<212> DNA
<213> Homo Sapien

<400> 57
tggacaagac acctccagga gcccagctca cagccaccgg taccttcttc 50
caggacaagc tgggggcctc catgggcgcc tgagggccag gcgccagggc 100
cgtgggcacg agtatggtga gacaccagcc cctgcagtac tacgagccac 150
agctgtgcct ctcctgcctc acgggcatct acggctgccg ttggaagcgc 200
taccagcgct cccatgatga taccacaccg ggcacagcgc cattcctgca 250
tgtgggggct gtggcagcag tcaccatgct ctcctggatc gtggcaggac 300
agttcgcccg tgcagagcgg acctcctccc aggtgaccat tctctgtacc 350
ttcttcaccg tggtgtttgc cctctacctg gcccctctca ccatctcctc 400
tccctgcatc atggagaaga aagacctcgg ccccaagcct gctctcattg 450
gccaccgcgg ggcccccatg ctggctccag agcacacgct catgtccttc 500
cggaaggccc tcgagcagaa gctgtacggg ctccaggctg acattaccat 550
cagcctggac ggcgtgccct tcctcatgca tgacaccacc ctgcggcgca 600
ccaccaacgt ggaggaggag ttcccggagc tggcccgcag gcctgcctcc 650
atgcttaact ggaccaccct gcagagactc aacgctggcc agtggttcct 700
gaagactgac cccttctgga cagccagctc cctgtcaccc tccgaccaca 750
gagaggccca gaaccagtcc atctgcagcc tggcagagct cctggagctg 800
gccaagggca atgccacact gctgctcaac ctgcgtgacc cgccccggga 850
gcacccctac cgcagcagtt ttatcaacgt gactctggag gccgtgctgc 900
actccggctt cccccagcac caggtcatgt ggctgcctag caggcagagg 950
cccctggtgc ggaaggtggc tcccggcttc caacagacat caggctccaa 1000
ggaggcagtc gccagcctgc ggagaggcca catccagcgg ctgaacctgc 1050
gctacactca ggtgtcccgc caggagctca gggactacgc gtcctggaac 1100
ctgagtgtga acctctacac agtcaacgca ccgtggctct tctccctgct 1150
gtggtgtgcg ggggtcccat ccgtcacctc tgacaactcc cacaccctgt 1200
cccaggtgcc ttccccctc tggatcatgc ccccggacga gtactgtctc 1250
atgtgggtca ctgccgacct ggtctccttc accctcatcg tgggcatctt 1300
cgtgctccag aagtggcgcc tgggtggcat acggagctac aaccctgagc 1350
agatcatgct gagtgctgcg gtgcgccgga ccagccggga cgtcagcatc 1400
atgaaggaga agcttatttt ctcagagatc agcgatggtg tagaggtctc 1450
cgatgtgctc tccgtatgtt cagacaacag ttatgacaca tatgccaaca 1500
gcaccgccac ccctgtgggc ccccgagggg gtggcagcca caccaagacc 1550
ctcatagagc ggagtgggcg ttagctgaag acatgtctgt cccacctgta 1600

cctgacacag aagctgggga gcctaggaga gctggtggaa gtgtgtctga 1650
actcggagtg ctctgggagc gggctccaca gcctccttgt gggctccagc 1700
cccttgtcag ccgcagcctc tcttgagggg gactccctgt ctcctgaggc 1750
ccagctgggc caggactcca tcctttcaga tgcccctgca ggcctggggc 1800
tccttctggg aagtatgggg cctagggctt ggtccccctc ttctgaggcc 1850
ctctcctgta tcccgacctg gaagctttga tgggtcatgg gccatgccat 1900
accccctgtg gcaatggagt gtgtggatgc tcacctgtgc catctgtcct 1950
cctgtctgtg ccaggaggca cctgagttct ctgctgttat cctgccccaa 2000
gggcctgggc cgagcctcta cctgaagcaa ctctgctctt cctgtcagtc 2050
tcaaagcaca aggaggttca gcccaggagg aagccagctg caatgtggag 2100
acacgtcctc ctccccaacc cacctcatgc caccgccaac cccctgcccc 2150
aggagcgggc ctgagccacg tcccctagga gcagctggag atggccaaaa 2200
gagtgagctc aggactactg gatcccatgc ccaggtgtcc agcagacctc 2250
aaggcagaag ggtcacctaa cccaggagtc cacagactga tgtgacctca 2300
ggttcccaca tcagtggcca cagggcaggg cccacctggt agaagtgttc 2350
tggatatggc cagggtgggt gtgtggctaa gtgggcctga acagagggaa 2400
cctagggccc ttggccaatg tgattaaagc tgccatcttg aaa 2443

<210> 58
<211> 486
<212> PRT
<213> Homo Sapien

<400> 58
Met Val Arg His Gln Pro Leu Gln Tyr Tyr Glu Pro Gln Leu Cys
1 5 10 15

Leu Ser Cys Leu Thr Gly Ile Tyr Gly Cys Arg Trp Lys Arg Tyr
20 25 30

Gln Arg Ser His Asp Asp Thr Thr Pro Gly Thr Ala Pro Phe Leu
35 40 45

His Val Gly Ala Val Ala Ala Val Thr Met Leu Ser Trp Ile Val
50 55 60

Ala Gly Gln Phe Ala Arg Ala Glu Arg Thr Ser Ser Gln Val Thr
65 70 75

Ile Leu Cys Thr Phe Phe Thr Val Val Phe Ala Leu Tyr Leu Ala
80 85 90

Pro Leu Thr Ile Ser Ser Pro Cys Ile Met Glu Lys Lys Asp Leu
95 100 105

Gly Pro Lys Pro Ala Leu Ile Gly His Arg Gly Ala Pro Met Leu
110 115 120

Ala Pro Glu His Thr Leu Met Ser Phe Arg Lys Ala Leu Glu Gln
125 130 135

Lys Leu Tyr Gly Leu Gln Ala Asp Ile Thr Ile Ser Leu Asp Gly
140 145 150

Val Pro Phe Leu Met His Asp Thr Thr Leu Arg Arg Thr Thr Asn
155 160 165

Val Glu Glu Glu Phe Pro Glu Leu Ala Arg Arg Pro Ala Ser Met
170 175 180

Leu Asn Trp Thr Thr Leu Gln Arg Leu Asn Ala Gly Gln Trp Phe
185 190 195

Leu Lys Thr Asp Pro Phe Trp Thr Ala Ser Ser Leu Ser Pro Ser
200 205 210

Asp His Arg Glu Ala Gln Asn Gln Ser Ile Cys Ser Leu Ala Glu
215 220 225

Leu Leu Glu Leu Ala Lys Gly Asn Ala Thr Leu Leu Leu Asn Leu
230 235 240

Arg Asp Pro Pro Arg Glu His Pro Tyr Arg Ser Ser Phe Ile Asn
245 250 255

Val Thr Leu Glu Ala Val Leu His Ser Gly Phe Pro Gln His Gln
260 265 270

Val Met Trp Leu Pro Ser Arg Gln Arg Pro Leu Val Arg Lys Val
275 280 285

Ala Pro Gly Phe Gln Gln Thr Ser Gly Ser Lys Glu Ala Val Ala
290 295 300

Ser Leu Arg Arg Gly His Ile Gln Arg Leu Asn Leu Arg Tyr Thr
305 310 315

Gln Val Ser Arg Gln Glu Leu Arg Asp Tyr Ala Ser Trp Asn Leu
320 325 330

Ser Val Asn Leu Tyr Thr Val Asn Ala Pro Trp Leu Phe Ser Leu
335 340 345

Leu Trp Cys Ala Gly Val Pro Ser Val Thr Ser Asp Asn Ser His
350 355 360

Thr Leu Ser Gln Val Pro Ser Pro Leu Trp Ile Met Pro Pro Asp
365 370 375

Glu Tyr Cys Leu Met Trp Val Thr Ala Asp Leu Val Ser Phe Thr
380 385 390

Leu Ile Val Gly Ile Phe Val Leu Gln Lys Trp Arg Leu Gly Gly
395 400 405

Ile Arg Ser Tyr Asn Pro Glu Gln Ile Met Leu Ser Ala Ala Val
410 415 420

Arg Arg Thr Ser Arg Asp Val Ser Ile Met Lys Glu Lys Leu Ile
425 430 435

Phe Ser Glu Ile Ser Asp Gly Val Glu Val Ser Asp Val Leu Ser
440 445 450

Val Cys Ser Asp Asn Ser Tyr Asp Thr Tyr Ala Asn Ser Thr Ala
455 460 465

Thr Pro Val Gly Pro Arg Gly Gly Gly Ser His Thr Lys Thr Leu
470 475 480

Ile Glu Arg Ser Gly Arg
485

<210> 59
<211> 2550
<212> DNA
<213> Homo Sapien

<400> 59
cctgagcaaa cacagcagcc cgagtgttcc caaggccaaa atgctgagaa 50

cgtccactcc taatctgtgt ggtggtctgc attgccgggc cccctggctc 100

tcttctggca ttctctgcct ctgcctcata ttcttgttag gccaggtggg 150

cttgctgcag ggacaccccc agtgcctgga ttacgggccc cctttccagc 200

cccctctgca ccttgagttt tgctctgact atgagtcctt cggctgctgt 250

gatcagcaca aggaccgccg catcgctgcc cggtactggg acatcatgga 300

atattttgat ctgaagagac atgagctgtg tggagattac attaaagaca 350

tcctttgcca ggagtgctcg ccctacgcag cccacctcta cgacgccgaa 400

aacacccaga cgcctctccg gaatctcccg ggcctctgct ctgattactg 450

ctctgccttc cattctaact gtcactcagc catttccctg ctgaccaatg 500

accgcggcct ccaggagtct catggaaggg acggtacccg cttctgccac 550

ctcctggacc ttcctgacaa ggactattgc ttccctaatg tcctgaggaa 600

cgactatctc aaccgccacc tgggcatggt ggcccaagat cctcagggct 650

gcctgcagct ctgcctgagc gaggtggcca acgggctgag gaaccccgtc 700

tccatggtcc atgctgggga cggcacccat cgcttctttg ttgccgagca 750

ggtaggagtg gtgtgggtct acctccctga tgggagtcgc ctggagcaac 800

ccttcctgga cctcaagaac atcgtgttga ccaccccatg gatcggggat 850

gagagaggct tcttggggtt ggcttttcac cccaaattcc gccacaatcg 900

caagttctat atttattatt cgtgcctgga caagaagaag gtagaaaaga 950

tccgaattag tgagatgaag gtttctcggg ctgatcctaa caaagctgac 1000

ctgaaatcag agagggtcat cttggagatt gaagaaccag cctcaaacca 1050

taatggcgga caacttcttt ttggcctgga tggctatatg tacatattca 1100

ctggggacgg gggacaggct ggagatccct ttggcctgtt tggaaatgct 1150

cagaacaaaa gttccctgct gggaaaagtt ttaaggatcg atgtgaacag 1200
ggcaggctca catggcaagc ggtaccgagt cccctcggac aatccatttg 1250
tttctgagcc aggggcccac cccgccatct atgcctatgg gatcaggaac 1300
atgtggcgtt gtgctgtgga ccgaggggac cccatcacgc gccagggccg 1350
aggccggata ttctgtgggg acgtgggcca gaacaggttt gaagaggttg 1400
acctcatttt gaaaggtgga aactatggct ggagagcaaa ggaagggttt 1450
gcatgttatg acaaaaaact ttgtcacaat gcctctttgg atgatgttct 1500
gccaatctat gcttatggcc atgcagtggg gaagtcagtc actggaggtt 1550
atgtctatcg tggttgtgaa tccccaaatc tcaatggcct gtatatcttt 1600
ggagacttca tgagtggtcg acttatggct ttgcaggaag atagaaaaaa 1650
caagaaatgg aagaagcagg atctttgcct gggcagcacc acgtcctgtg 1700
ccttcccagg gctgatcagc acccatagca agttcatcat ctcctttgct 1750
gaagatgaag caggggagct gtatttcctg gcgacctctt acccaagtgc 1800
ctatgcacca cgtggatcta tttacaagtt tgttgacccc tcaaggcgag 1850
cacccccagg caagtgcaaa tacaagccag tgcccgtgag aaccaagagt 1900
aagcggatcc cgttcagacc actcgccaag acagtcttgg acttgctaaa 1950
ggaacaatca gagaaagctg ctagaaaatc ttccagtgca accttagctt 2000
ctggcccagc ccagggtttg tctgagaaag gctcctccaa gaagctggct 2050
tctcctacaa gcagcaagaa tacattgcga gggcctggta caaagaagaa 2100
agccagagtg gggccccacg tccgccaggg caagaggagg aagagcctga 2150
aaagccacag tggcaggatg aggccatcag cagagcagaa gcgagctggc 2200
agaagtctcc cttgacctat tggtcaaggt ggccgacagg gtgacgtgag 2250
agaggagagc cacctcatca aatgaaagtc actgctgaat aaagacctta 2300
gaagtctggg aagccagggt agaggtgggg cagggcggtt ttcctctccc 2350
tgggaaatct tgctgtctac tgaataaata aatgcacctt ctctgtatgc 2400
agtgcttctg tgggagacca tatcccagat tgctggtgca cctgggttat 2450
ggtaagcact agtccatgag cctgcttgga atcacactgg atgtctccgt 2500
tttgtcttgt aaatgcctac aacctgaggt aataaatcaa catttgctca 2550

<210> 60
<211> 724
<212> PRT
<213> Homo Sapien

<400> 60
Met Leu Arg Thr Ser Thr Pro Asn Leu Cys Gly Gly Leu His Cys
1 5 10 15

```
Arg Ala Pro Trp Leu Ser Ser Gly Ile Leu Cys Leu Cys Leu Ile
                20                  25                  30

Phe Leu Leu Gly Gln Val Gly Leu Leu Gln Gly His Pro Gln Cys
                35                  40                  45

Leu Asp Tyr Gly Pro Pro Phe Gln Pro Pro Leu His Leu Glu Phe
                50                  55                  60

Cys Ser Asp Tyr Glu Ser Phe Gly Cys Cys Asp Gln His Lys Asp
                65                  70                  75

Arg Arg Ile Ala Ala Arg Tyr Trp Asp Ile Met Glu Tyr Phe Asp
                80                  85                  90

Leu Lys Arg His Glu Leu Cys Gly Asp Tyr Ile Lys Asp Ile Leu
                95                 100                 105

Cys Gln Glu Cys Ser Pro Tyr Ala Ala His Leu Tyr Asp Ala Glu
               110                 115                 120

Asn Thr Gln Thr Pro Leu Arg Asn Leu Pro Gly Leu Cys Ser Asp
               125                 130                 135

Tyr Cys Ser Ala Phe His Ser Asn Cys His Ser Ala Ile Ser Leu
               140                 145                 150

Leu Thr Asn Asp Arg Gly Leu Gln Glu Ser His Gly Arg Asp Gly
               155                 160                 165

Thr Arg Phe Cys His Leu Leu Asp Leu Pro Asp Lys Asp Tyr Cys
               170                 175                 180

Phe Pro Asn Val Leu Arg Asn Asp Tyr Leu Asn Arg His Leu Gly
               185                 190                 195

Met Val Ala Gln Asp Pro Gln Gly Cys Leu Gln Leu Cys Leu Ser
               200                 205                 210

Glu Val Ala Asn Gly Leu Arg Asn Pro Val Ser Met Val His Ala
               215                 220                 225

Gly Asp Gly Thr His Arg Phe Phe Val Ala Glu Gln Val Gly Val
               230                 235                 240

Val Trp Val Tyr Leu Pro Asp Gly Ser Arg Leu Glu Gln Pro Phe
               245                 250                 255

Leu Asp Leu Lys Asn Ile Val Leu Thr Thr Pro Trp Ile Gly Asp
               260                 265                 270

Glu Arg Gly Phe Leu Gly Leu Ala Phe His Pro Lys Phe Arg His
               275                 280                 285

Asn Arg Lys Phe Tyr Ile Tyr Tyr Ser Cys Leu Asp Lys Lys Lys
               290                 295                 300

Val Glu Lys Ile Arg Ile Ser Glu Met Lys Val Ser Arg Ala Asp
               305                 310                 315

Pro Asn Lys Ala Asp Leu Lys Ser Glu Arg Val Ile Leu Glu Ile
               320                 325                 330
```

Glu Glu Pro Ala Ser Asn His Asn Gly Gly Gln Leu Leu Phe Gly
335 340 345

Leu Asp Gly Tyr Met Tyr Ile Phe Thr Gly Asp Gly Gly Gln Ala
350 355 360

Gly Asp Pro Phe Gly Leu Phe Gly Asn Ala Gln Asn Lys Ser Ser
365 370 375

Leu Leu Gly Lys Val Leu Arg Ile Asp Val Asn Arg Ala Gly Ser
380 385 390

His Gly Lys Arg Tyr Arg Val Pro Ser Asp Asn Pro Phe Val Ser
395 400 405

Glu Pro Gly Ala His Pro Ala Ile Tyr Ala Tyr Gly Ile Arg Asn
410 415 420

Met Trp Arg Cys Ala Val Asp Arg Gly Asp Pro Ile Thr Arg Gln
425 430 435

Gly Arg Gly Arg Ile Phe Cys Gly Asp Val Gly Gln Asn Arg Phe
440 445 450

Glu Glu Val Asp Leu Ile Leu Lys Gly Gly Asn Tyr Gly Trp Arg
455 460 465

Ala Lys Glu Gly Phe Ala Cys Tyr Asp Lys Lys Leu Cys His Asn
470 475 480

Ala Ser Leu Asp Asp Val Leu Pro Ile Tyr Ala Tyr Gly His Ala
485 490 495

Val Gly Lys Ser Val Thr Gly Gly Tyr Val Tyr Arg Gly Cys Glu
500 505 510

Ser Pro Asn Leu Asn Gly Leu Tyr Ile Phe Gly Asp Phe Met Ser
515 520 525

Gly Arg Leu Met Ala Leu Gln Glu Asp Arg Lys Asn Lys Lys Trp
530 535 540

Lys Lys Gln Asp Leu Cys Leu Gly Ser Thr Thr Ser Cys Ala Phe
545 550 555

Pro Gly Leu Ile Ser Thr His Ser Lys Phe Ile Ile Ser Phe Ala
560 565 570

Glu Asp Glu Ala Gly Glu Leu Tyr Phe Leu Ala Thr Ser Tyr Pro
575 580 585

Ser Ala Tyr Ala Pro Arg Gly Ser Ile Tyr Lys Phe Val Asp Pro
590 595 600

Ser Arg Arg Ala Pro Pro Gly Lys Cys Lys Tyr Lys Pro Val Pro
605 610 615

Val Arg Thr Lys Ser Lys Arg Ile Pro Phe Arg Pro Leu Ala Lys
620 625 630

Thr Val Leu Asp Leu Leu Lys Glu Gln Ser Glu Lys Ala Ala Arg
635 640 645

Lys Ser Ser Ser Ala Thr Leu Ala Ser Gly Pro Ala Gln Gly Leu

650 655 660

Ser Glu Lys Gly Ser Ser Lys Lys Leu Ala Ser Pro Thr Ser Ser
665 670 675

Lys Asn Thr Leu Arg Gly Pro Gly Thr Lys Lys Lys Ala Arg Val
680 685 690

Gly Pro His Val Arg Gln Gly Lys Arg Arg Lys Ser Leu Lys Ser
695 700 705

His Ser Gly Arg Met Arg Pro Ser Ala Glu Gln Lys Arg Ala Gly
710 715 720

Arg Ser Leu Pro

<210> 61
<211> 2119
<212> DNA
<213> Homo Sapien

<400> 61
ctccattaaa ccaccaccag ctccccaagc caccccttca gccatgaagt 50

tcctgctcct ggtcttggca gccctcggat tcctgaccca ggtgatccca 100

gccagtgcag gtgggtcaaa atgtgtgagt aacaccccag gatactgcag 150

gacatgttgc cactgggggg agacagcatt gttcatgtgc aacgcttcca 200

gaaaatgctg catcagctac tccttcctgc cgaagcctga cctaccacag 250

ctcatcggta accactggca atcaaggaga agaaacacac aaaggaaaga 300

caagaagcaa caaacgaccg taacatcata ataaccactg ctatcgcctc 350

caccaactca gagaaatatc atttccacag ttccaattcc tcctacattg 400

ctgagtacta gccaaggctc ctctttatgg ggcagatatc tatagccaac 450

cccaaaactt ctgtcttcta tcattctgtc attcatctag taactaattt 500

ggagtttgta tctatcttac gagaacaatc atcatgcaga ttcgtccaca 550

ggggatctgt cagtttgggt cctccaaatg aaaaatgtca agacagaatt 600

ggacatgcaa aagattgact gggagaacac acctctgatg gacaaaggtg 650

agacagagca gccacaggca gggagagcct tcagactgca acgctggcct 700

gatacgtgtc aaaggagaga gggatagagg aggattgaat agaaggagac 750

taagactgca gctctaagaa agtctcagcc aaacagatgg ggaggcccaa 800

agcaaggctt gcccctcaga ggagctcacg cagggcagga atagccaggt 850

tctcatatcc caggggttca gacttggctg agaacagccc ctggagaaca 900

tggggtgact gctaccatag gtctggaagt atgaggctgt ccaccaacta 950

tccccttgaa gcaagttctc ttgaaaggaa atctaaacag tgcacccccca 1000

tggctgccac ggagtataag gagggagaga aaggagctga aagtctaggt 1050

ttggccagct aggtagactg acttgtgagg tatttattta ttcatttgag 1100

taacaaagca gacagaatac atagccacca ttggtagtac accccaaaag 1150

caaggatggc atgatgctgg tgactcaaac gtgcctactc atggtgtcaa 1200

attggcataa tcctcttggg aagctgtgtg gaaataagca cagagaagca 1250

gaactctaat tgcttaatcc actaaacatt acttctggga attggctcat 1300

cataaattat ccaagagaaa gcacaaagtt atgggcacaa aggttttcca 1350

tataatatta tttaaaatgc tgagaaaatg aaaaaatcta aatggtgaaa 1400

tatatactaa tgccatctat aaatacaaac aaatagaatg tttatagaat 1450

aatggaacat aataacatta ttcaaaattg catttatgct atagttgtca 1500

aaattgtctc cttatatgat acaaaactca tgaaaattat gacttttttg 1550

tttggttgga aagcagaatt atgcataaat ttcctcttac agttcgatgc 1600

ccattagttt tatataacat ttatttgaca cgtactgact tctatctgag 1650

aagaacaaac caaaacactc aggcctaaat aattaaaaac ggtcctaaaa 1700

actagcaaac cagataagaa aagatgttaa tgcccattcc ctaacttatg 1750

tcttagacca aaattaattc tagatggttt taaaatgaca gtgtaaaagt 1800

aaagtattaa aagattgtgt ggtcaaatat tcaatttaag agcaaggaaa 1850

ttcttataaa tataacaata gaggcagaac tcatgtaaga ataaattgat 1900

taggtggtat taaatattaa gttcttatgt atgtcaaaag atatcatttt 1950

gaaattcatc catcttattg ggtattgcag gagttcattc ctttttgttt 2000

ataaatactc ttccgtcata tgaatagtat tcatttgtat actggtttgt 2050

tgatggacat ttgggttgtt cccagtttat ggctattaca aataaagctt 2100

ctatgaacat ttatgtaca 2119

<210> 62
<211> 95
<212> PRT
<213> Homo Sapien

<400> 62
Met Lys Phe Leu Leu Leu Val Leu Ala Ala Leu Gly Phe Leu Thr
1 5 10 15

Gln Val Ile Pro Ala Ser Ala Gly Gly Ser Lys Cys Val Ser Asn
20 25 30

Thr Pro Gly Tyr Cys Arg Thr Cys Cys His Trp Gly Glu Thr Ala
35 40 45

Leu Phe Met Cys Asn Ala Ser Arg Lys Cys Cys Ile Ser Tyr Ser
50 55 60

Phe Leu Pro Lys Pro Asp Leu Pro Gln Leu Ile Gly Asn His Trp

65 70 75

Gln Ser Arg Arg Arg Asn Thr Gln Arg Lys Asp Lys Lys Gln Gln
80 85 90

Thr Thr Val Thr Ser
95

<210> 63
<211> 2623
<212> DNA
<213> Homo Sapien

<400> 63
gcggagcgcc tgggagagga gaaggagccg acctgccgag atggaggcga 50

ccggcacctg ggcgctgctg ctggcgctgg cgctgctcct gctgctgacg 100

ctggcgctgt ccgggaccag ggcccgaggc cacctgcccc ccgggcccac 150

gccgctacca ctgctgggaa acctcctgca gctacggccc ggggcgctgt 200

attcagggct catgcggctg agtaagaagt acggaccggt gttcaccatc 250

tacctgggac cctggcggcc tgtggtggtc ctggttgggc aggaggctgt 300

gcgggaggcc ctgggaggtc aggctgagga gttcagcggc cggggaaccg 350

tagcgatgct ggaagggact tttgatggcc atggggtttt cttctccaac 400

ggggagcggt ggaggcagct gaggaagttt accatgcttg ctctgcggga 450

cctgggcatg ggaagcgag aaggcgagga gctgatccag gcggaggccc 500

ggtgtctggt ggagacattc caggggacag aaggacgccc attcgatccc 550

tccctgctgc tggcccaggc cacctccaac gtagtctgct ccctcctctt 600

tggcctccgc ttctcctatg aggataagga gttccaggcc gtggtccggg 650

cagctggtgg taccctgctg ggagtcagct cccagggggg tcagacctac 700

gagatgttct cctggttcct gcggcccctg ccaggccccc acaagcagct 750

cctccaccac gtcagcacct tggctgcctt cacagtccgg caggtgcagc 800

agcaccaggg gaacctggat gcttcgggcc ccgcacgtga ccttgtcgat 850

gccttcctgc tgaagatggc acaggaggaa caaaacccag gcacagaatt 900

caccaacaag aacatgctga tgacagtcat ttatttgctg tttgctggga 950

cgatgacggt cagcaccacg gtcggctata ccctcctgct cctgatgaaa 1000

taccctcatg tccaaaagtg ggtacgtgag gagctgaatc gggagctggg 1050

ggctggccag gcaccaagcc taggggaccg tacccgcctc ccttacaccg 1100

acgcggttct gcatgaggcg cagcggctgc tggcgctggt gcccatggga 1150

ataccccgca ccctcatgcg gaccacccgc ttccgagggt acaccctgcc 1200

ccagggcacg gaggtcttcc ccctccttgg ctccatcctg catgacccca 1250

acatcttcaa gcacccagaa gagttcaacc cagaccgttt cctggatgca 1300
gatggacggt tcaggaagca tgaggcgttc ctgcccttct ccttagggaa 1350
gcgtgtctgc cttggagagg gcctggcaaa agcggagctc ttcctcttct 1400
tcaccaccat cctacaagcc ttctccctgg agagcccgtg cccgccggac 1450
accctgagcc tcaagcccac cgtcagtggc cttttcaaca ttcccccagc 1500
cttccagctg caagtccgtc ccactgacct tcactccacc acgcagacca 1550
gatgaaggaa ggcaacttgg aagtggtggg tgcccaggac ggtgcctcca 1600
gcctcaacag tgggcatgga cagggttaat gtctccagag tgtacactgc 1650
aggcagccac atttacacgc ctgcagttgt tttccggagt ctgtcccacg 1700
gcccacacgc tcacttgact catgctgcta agatgcacaa ccgcacaccc 1750
atacacaact acaagggcca caaagcaact gctgggttag ctttccacag 1800
acataaatat agtccatctg caatcacaag cacatagcca ggtaacccac 1850
caactcccct ggatctgcag cccacacgtg ggagtctggc tgtcaccttc 1900
acaagccaca gaaacggcca cacatgttca cagctcacac gccctctcca 1950
ttcatcgaac ttctcagtgt ccctgtccct ggtgcctggc acagggaaca 2000
gcatgccccc tccggggtca tgccacccag agactgtcgc tgtctatggc 2050
cccaactcat gctccctctc ttggctacac cactctccca gcctgtgacc 2100
accgatgtcc acacaccccc aaccacttgt ccacacagct acccacgtac 2150
aacatcgtcc tggctcccca gagtatcttc ccactgagac acgccgcccc 2200
cacagaggca cagtccccag ccacctctgc aactgcagcc ctcagtcacc 2250
cctttttaag caccctgatt ctaccaaatg caaacacatc tgggtctgcg 2300
attatgcaca gagactttgg acatacgagg accctcagac cggaggaaca 2350
cctgcccaac cccaacacgt gcttatgtaa ccacgtggaa agcggcccct 2400
gctgcccctc cacacacaca tacacactca ctgatctaca gcccctgttc 2450
ggcgtcagag tccccactag acccagtgga aggggttaga gaccaagtag 2500
gggccagttt ccaattcacc ctgtcaggga gtgagccgga tctgacgttc 2550
cttgtgactt aagggtccgg cttgggaatt aaagtttgtt tctggccttt 2600
agcctaaaaa aaaaaaaaaa aaa 2623

<210> 64
<211> 504
<212> PRT
<213> Homo Sapien

<400> 64
Met Glu Ala Thr Gly Thr Trp Ala Leu Leu Leu Ala Leu Ala Leu
1               5                   10                  15

Leu Leu Leu Leu Thr Leu Ala Leu Ser Gly Thr Arg Ala Arg Gly
20 25 30

His Leu Pro Pro Gly Pro Thr Pro Leu Pro Leu Leu Gly Asn Leu
35 40 45

Leu Gln Leu Arg Pro Gly Ala Leu Tyr Ser Gly Leu Met Arg Leu
50 55 60

Ser Lys Lys Tyr Gly Pro Val Phe Thr Ile Tyr Leu Gly Pro Trp
65 70 75

Arg Pro Val Val Val Leu Val Gly Gln Glu Ala Val Arg Glu Ala
80 85 90

Leu Gly Gly Gln Ala Glu Glu Phe Ser Gly Arg Gly Thr Val Ala
95 100 105

Met Leu Glu Gly Thr Phe Asp Gly His Gly Val Phe Phe Ser Asn
110 115 120

Gly Glu Arg Trp Arg Gln Leu Arg Lys Phe Thr Met Leu Ala Leu
125 130 135

Arg Asp Leu Gly Met Gly Lys Arg Glu Gly Glu Glu Leu Ile Gln
140 145 150

Ala Glu Ala Arg Cys Leu Val Glu Thr Phe Gln Gly Thr Glu Gly
155 160 165

Arg Pro Phe Asp Pro Ser Leu Leu Leu Ala Gln Ala Thr Ser Asn
170 175 180

Val Val Cys Ser Leu Leu Phe Gly Leu Arg Phe Ser Tyr Glu Asp
185 190 195

Lys Glu Phe Gln Ala Val Val Arg Ala Ala Gly Gly Thr Leu Leu
200 205 210

Gly Val Ser Ser Gln Gly Gly Gln Thr Tyr Glu Met Phe Ser Trp
215 220 225

Phe Leu Arg Pro Leu Pro Gly Pro His Lys Gln Leu Leu His His
230 235 240

Val Ser Thr Leu Ala Ala Phe Thr Val Arg Gln Val Gln Gln His
245 250 255

Gln Gly Asn Leu Asp Ala Ser Gly Pro Ala Arg Asp Leu Val Asp
260 265 270

Ala Phe Leu Leu Lys Met Ala Gln Glu Glu Gln Asn Pro Gly Thr
275 280 285

Glu Phe Thr Asn Lys Asn Met Leu Met Thr Val Ile Tyr Leu Leu
290 295 300

Phe Ala Gly Thr Met Thr Val Ser Thr Thr Val Gly Tyr Thr Leu
305 310 315

Leu Leu Leu Met Lys Tyr Pro His Val Gln Lys Trp Val Arg Glu
320 325 330

```
Glu Leu Asn Arg Glu Leu Gly Ala Gly Gln Ala Pro Ser Leu Gly
                335                 340                 345

Asp Arg Thr Arg Leu Pro Tyr Thr Asp Ala Val Leu His Glu Ala
                350                 355                 360

Gln Arg Leu Leu Ala Leu Val Pro Met Gly Ile Pro Arg Thr Leu
                365                 370                 375

Met Arg Thr Thr Arg Phe Arg Gly Tyr Thr Leu Pro Gln Gly Thr
                380                 385                 390

Glu Val Phe Pro Leu Leu Gly Ser Ile Leu His Asp Pro Asn Ile
                395                 400                 405

Phe Lys His Pro Glu Glu Phe Asn Pro Asp Arg Phe Leu Asp Ala
                410                 415                 420

Asp Gly Arg Phe Arg Lys His Glu Ala Phe Leu Pro Phe Ser Leu
                425                 430                 435

Gly Lys Arg Val Cys Leu Gly Glu Gly Leu Ala Lys Ala Glu Leu
                440                 445                 450

Phe Leu Phe Phe Thr Thr Ile Leu Gln Ala Phe Ser Leu Glu Ser
                455                 460                 465

Pro Cys Pro Pro Asp Thr Leu Ser Leu Lys Pro Thr Val Ser Gly
                470                 475                 480

Leu Phe Asn Ile Pro Pro Ala Phe Gln Leu Gln Val Arg Pro Thr
                485                 490                 495

Asp Leu His Ser Thr Thr Gln Thr Arg
                500

<210> 65
<211> 1606
<212> DNA
<213> Homo Sapien

<400> 65
cggacgcgtg gggccgtatg cgcggctctg tggagtgcac ctggggttgg 50

gggcactgtg cccccagccc cctgctcctt tggactctac ttctgtttgc 100

agccccattt ggcctgctgg gggagaagac ccgccaggtg tctctggagg 150

tcatccctaa ctggctgggc cccctgcaga acctgcttca tatacgggca 200

gtgggcacca attccacact gcactatgtg tggagcagcc tggggcctct 250

ggcagtggta atggtggcca ccaacacccc ccacagcacc ctgagcatca 300

actggagcct cctgctatcc cctgagcccg atgggggcct gatggtgctc 350

cctaaggaca gcattcagtt ttcttctgcc cttgttttta ccaggctgct 400

tgagtttgac agcaccaacg tgtccgatac ggcagcaaag cctttgggaa 450

gaccatatcc tccatactcc ttggccgatt tctcttggaa caacatcact 500

gattcattgg atcctgccac cctgagtgcc acatttcaag gccaccccat 550
```

gaacgaccct accaggactt ttgccaatgg cagcctggcc ttcagggtcc 600
aggccttttc caggtccagc cgaccagccc aaccccctcg cctcctgcac 650
acagcagaca cctgtcagct agaggtggcc ctgattggag cctctccccg 700
gggaaaccgt tccctgtttg ggctggaggt agccacattg ggccagggcc 750
ctgactgccc ctcaatgcag gagcagcact ccatcgacga tgaatatgca 800
ccggccgtct tccagttgga ccagctactg tggggctccc tcccatcagg 850
ctttgcacag tggcgaccag tggcttactc ccagaagccg gggggccgag 900
aatcagccct gccctgccaa gcttcccctc ttcatcctgc cttagcatac 950
tctcttcccc agtcacccat tgtccgagcc ttctttgggt cccagaataa 1000
cttctgtgcc ttcaatctga cgttcggggc ttccacaggc cctggctatt 1050
gggaccaaca ctacctcagc tggtcgatgc tcctgggtgt gggcttccct 1100
ccagtggacg gcttgtcccc actagtcctg ggcatcatgg cagtggccct 1150
gggtgcccca gggctcatgc tgctaggggg cggcttggtt ctgctgctgc 1200
accacaagaa gtactcagag taccagtcca taaattaagg cccgctctct 1250
ggagggaagg acattactga acctgtcttg ctgtgcctcg aaactctgga 1300
ggttggagca tcaagttcca gccggcccct tcactccccc atcttgcttt 1350
tctgtggaac ctcagaggcc agcctcgact tcctggagac ccccaggtgg 1400
ggcttccttc atactttgtt gggggacttt ggaggcgggc aggggacagg 1450
gctattgata aggtcccctt ggtgttgcct tcttgcatct ccacacattt 1500
cccttggatg ggacttgcag gcctaaatga gaggcattct gactggttgg 1550
ctgccctgga aggcaagaaa atagatttat ttttttcac agggaaaaaa 1600
aaaaaa 1606

<210> 66
<211> 406
<212> PRT
<213> Homo Sapien

<400> 66
Met Arg Gly Ser Val Glu Cys Thr Trp Gly Trp Gly His Cys Ala
1 5 10 15

Pro Ser Pro Leu Leu Leu Trp Thr Leu Leu Leu Phe Ala Ala Pro
20 25 30

Phe Gly Leu Leu Gly Glu Lys Thr Arg Gln Val Ser Leu Glu Val
35 40 45

Ile Pro Asn Trp Leu Gly Pro Leu Gln Asn Leu Leu His Ile Arg
50 55 60

Ala Val Gly Thr Asn Ser Thr Leu His Tyr Val Trp Ser Ser Leu
65 70 75

Gly Pro Leu Ala Val Val Met Val Ala Thr Asn Thr Pro His Ser
80 85 90

Thr Leu Ser Ile Asn Trp Ser Leu Leu Leu Ser Pro Glu Pro Asp
95 100 105

Gly Gly Leu Met Val Leu Pro Lys Asp Ser Ile Gln Phe Ser Ser
110 115 120

Ala Leu Val Phe Thr Arg Leu Leu Glu Phe Asp Ser Thr Asn Val
125 130 135

Ser Asp Thr Ala Ala Lys Pro Leu Gly Arg Pro Tyr Pro Pro Tyr
140 145 150

Ser Leu Ala Asp Phe Ser Trp Asn Asn Ile Thr Asp Ser Leu Asp
155 160 165

Pro Ala Thr Leu Ser Ala Thr Phe Gln Gly His Pro Met Asn Asp
170 175 180

Pro Thr Arg Thr Phe Ala Asn Gly Ser Leu Ala Phe Arg Val Gln
185 190 195

Ala Phe Ser Arg Ser Ser Arg Pro Ala Gln Pro Pro Arg Leu Leu
200 205 210

His Thr Ala Asp Thr Cys Gln Leu Glu Val Ala Leu Ile Gly Ala
215 220 225

Ser Pro Arg Gly Asn Arg Ser Leu Phe Gly Leu Glu Val Ala Thr
230 235 240

Leu Gly Gln Gly Pro Asp Cys Pro Ser Met Gln Glu Gln His Ser
245 250 255

Ile Asp Asp Glu Tyr Ala Pro Ala Val Phe Gln Leu Asp Gln Leu
260 265 270

Leu Trp Gly Ser Leu Pro Ser Gly Phe Ala Gln Trp Arg Pro Val
275 280 285

Ala Tyr Ser Gln Lys Pro Gly Gly Arg Glu Ser Ala Leu Pro Cys
290 295 300

Gln Ala Ser Pro Leu His Pro Ala Leu Ala Tyr Ser Leu Pro Gln
305 310 315

Ser Pro Ile Val Arg Ala Phe Phe Gly Ser Gln Asn Asn Phe Cys
320 325 330

Ala Phe Asn Leu Thr Phe Gly Ala Ser Thr Gly Pro Gly Tyr Trp
335 340 345

Asp Gln His Tyr Leu Ser Trp Ser Met Leu Leu Gly Val Gly Phe
350 355 360

Pro Pro Val Asp Gly Leu Ser Pro Leu Val Leu Gly Ile Met Ala
365 370 375

Val Ala Leu Gly Ala Pro Gly Leu Met Leu Leu Gly Gly Gly Leu
380 385 390

Val Leu Leu Leu His His Lys Lys Tyr Ser Glu Tyr Gln Ser Ile
395 400 405

Asn

<210> 67
<211> 4185
<212> DNA
<213> Homo Sapien

<400> 67
cgggacaggc gcgtgaggcc acaacacatg cgtgtatctt gcttgggcta 50

tcttccctgc tctgccacgc cgggtctgga gaaggggttt cagccccagg 100

acatttactg agagtcggcg aatattggga gccgcgatgt tccccccttcg 150

ggccctgtgg ttggtctggg cgcttctagg agtggccgga tcatgcccgg 200

agccgtgcgc ctgcgtggac aagtacgctc accagttcgc ggactgcgct 250

tacaaagagt tgcgtgaggt gccggaagga ctgcctgcca acgtgacgac 300

gcttagtctg tccgcgaaca agatcactgt gctgcggcgc ggggccttcg 350

ccgacgtcac acaggtcacg tcgctgtggc tggcgcacaa tgaggtgcgc 400

accgtggagc caggcgcact ggccgtgctg agtcagctca agaacctcga 450

tctgagccac aacttcatat ccagctttcc gtggagcgac ctgcgcaacc 500

tgagcgcgct gcagctgctc aaaatgaacc acaaccgcct gggctctctg 550

ccccgggacg cactcggtgc gctacccgac ctgcgttccc tgcgcatcaa 600

caacaaccgg ctgcgtacgc tggcgcctgg caccttcgac gcgcttagcg 650

cgctgtcaca cttgcaactc tatcacaatc ccttccactg cggctgcggc 700

cttgtgtggc tgcaggcctg ggccgcgagc acccgggtgt ccttacccga 750

gcccgactcc attgcttgtg cctcgcctcc cgcgctgcag ggggtgccgg 800

tgtaccgcct gcccgccctg ccctgtgcac cgcccagcgt gcatctgagt 850

gccgagccac cgcttgaagc acccggcacc ccactgcgcg caggactggc 900

gttcgtgtta cactgcatcg ccgacggcca ccctacgcct cgcctgcaat 950

ggcaacttca gatccccggt ggcaccgtag tcttagagcc accggttctg 1000

agcggggagg acgacggggt tggggcggag gaaggagagg gagaaggaga 1050

tggggatttg ctgacgcaga cccaagccca aacgccgact ccagcacccg 1100

cttggccggc gcccccagcc acaccgcgct tcctggccct cgcaaatggc 1150

tccctgttgg tgcccctcct gagtgccaag gaggcgggcg tctacacttg 1200

ccgtgcacac aatgagctgg gcgccaactc tacgtcaata cgcgtggcgg 1250

tggcagcaac cgggccccca aaacacgcgc ctggcgccgg gggagaaccc 1300

gacggacagg ccccgacctc tgagcgcaag tccacagcca agggccgggg 1350
caacagcgtc ctgccttcca aacccgaggg caaaatcaaa ggccaaggcc 1400
tggccaaggt cagcattctc ggggagaccg agacggagcc ggaggaggac 1450
acaagtgagg gagaggaggc cgaagaccag atcctcgcgg acccggcgga 1500
ggagcagcgc tgtggcaacg gggacccctc tcggtacgtt tctaaccacg 1550
cgttcaacca gagcgcagag ctcaagccgc acgtcttcga gctgggcgtc 1600
atcgcgctgg atgtggcgga gcgcgaggcg cgggtgcagc tgactccgct 1650
ggctgcgcgc tggggccctg ggcccggcgg ggctggcgga gccccgcgac 1700
ccgggcggcg acccctgcgc ctactctatc tgtgtccagc ggggggcggc 1750
gcggcagtgc agtggtcccg cgtagaggaa ggcgtcaacg cctactggtt 1800
ccgcggcctg cggccgggta ccaactactc cgtgtgcctg gcgctggcgg 1850
gcgaagcctg ccacgtgcaa gtggtgtttt ccaccaagaa ggagctccca 1900
tcgctgctgg tcatagtggc agtgagcgta ttcctcctgg tgctggccac 1950
agtgcccctt ctgggcgccg cctgctgcca tctgctggct aaacacccgg 2000
gcaagcccta ccgtctgatc ctgcggcctc aggcccctga ccctatggag 2050
aagcgcatcg ccgcagactt cgacccgcgt gcttcgtacc tcgagtccga 2100
gaaaagctac ccggcaggcg gcgaggcggg cggcgaggag ccagaggacg 2150
tgcaggggga gggccttgat gaagacgcgg agcagggaga cccaagtggg 2200
gacctgcaga gagaggagag cctggcggcc tgctcactgg tggagtccca 2250
gtccaaggcc aaccaagagg agttcgaggc gggctctgag tacagcgatc 2300
ggctgcccct gggcgccgag gcggtcaaca tcgcccagga gattaatggc 2350
aactacaggc agacggcagg ctgaacctcc gcccgtccgg cccgcccatt 2400
cccgacctcc acctagggtg cctgggagca gcagtctagg gctggcagga 2450
cttatgtccc ccgtccccaa ccttcaccta ctcctccccc ttactactcc 2500
ccaaccttga ctaccaggga cttctattag ggagtgggcc gatttcacca 2550
gtccctgcta cccacggctg ccattctccc tgcgggctga atccccttcc 2600
ccgccaagca cagtgtttat cttaccccat gcaagactcc acccgcagac 2650
ggtgggcgat atctatgtcc ctccattccc gtcgcgatta tctgcgaaat 2700
ccaccccgca gcccgcccca ccgtgggctc tggagccaga ggaaacgagc 2750
gaagactttg gaaacctcgc ggtaacgcgg tggtttcggg ggccagccaa 2800
ggccagtgga gtgctgtggg gtcccacctc gacccctcct cctccctttc 2850
tttctttcct ttttttttat tttttaattt tatttattta tttatttatt 2900

ttttgacgga gtcttggtct gtcgccaggc tggagtgcag tggcgcgatc 2950

tcggctcact gcatcttccg cctcccgggt tcaagcgatt ctcctgcctc 3000

agcctgccta gtagctggga ctacaggcgc gcgccaccac gaccagctaa 3050

tttcttctat ttttagtaga gacggggttt caccatgttg gccaggatgg 3100

tctggatctc ttgacctcag gtgatccatc tgcctcggcc tctcaaagtg 3150

ctgggattac aggcgtgagg caccgcgccc ggcccctcct ccctttcaat 3200

ccctactccc agaagccggg attcgtggca acccctagtt tttagttcca 3250

aagcctcctg ccggcaggga accaaatcct tctgtcctcc cacccccacc 3300

ccacttctgg ccagttggag tccagcccgg tgcctggggc gcctttcagc 3350

tccgcgctca gattttcctg ttttcgttgt tttcaaagac agcgacattt 3400

cgggtctggt gctaacaccc ccttcccagc ctctgggaaa atcgagtgtg 3450

tgtgtcgggg ggtagggagg gaatgcgttt tctgtcgtct ctctcctaac 3500

ttaaagcgcc gcaggaccgc gcgccccttg gcggctgagc ctgtggactt 3550

ggtcgcgggc caatttcgtt gtccgtgtgt tgggctttcc ggaggtctgt 3600

gcgcccaaca gcgccgctcc cgcggctcca cccgacccag accctagctg 3650

gaaagcgccg gaggcggagg aagctgactg tggcctcccg ggccgcggct 3700

ctctggaggg ctcgcgccct agttcgcaca aagcctgctc gtgactgtgc 3750

gactgtgcga cgggatccgg atggagccga gcccctccgt cctcgcgtct 3800

cggtcctcgc gtcgccccgc cccacccgcc cctgcttcgg cgggaatcgt 3850

gtttgcccgg cgtgtagtcc ctgacaagcg tgccctgtag gagaaaagtc 3900

tgtgtcctgt gaagtgtgac cgtgtagtgt agggggggcgg gcgggggggc 3950

ggatgggcgg ggagggaggg aaggggaggg gcgcggcgcg gcgactcggg 4000

gcggggttct tttttccatt ttgaaagaaa gcgtcggggt tggggtgggg 4050

ggagtttcag tcctcgggat cagccctctc cgcgaagcgc agcacaagcg 4100

cgggcctggg acggagtagc cccccggagc ccgtgccctt ttctaaacgc 4150

gtctgtatgc agtcaataaa acaatcgatt tgaaa 4185

<210> 68
<211> 745
<212> PRT
<213> Homo Sapien

<400> 68
Met Phe Pro Leu Arg Ala Leu Trp Leu Val Trp Ala Leu Leu Gly
1 5 10 15

Val Ala Gly Ser Cys Pro Glu Pro Cys Ala Cys Val Asp Lys Tyr
20 25 30

```
Ala His Gln Phe Ala Asp Cys Ala Tyr Lys Glu Leu Arg Glu Val
                35                  40                  45

Pro Glu Gly Leu Pro Ala Asn Val Thr Thr Leu Ser Leu Ser Ala
                50                  55                  60

Asn Lys Ile Thr Val Leu Arg Arg Gly Ala Phe Ala Asp Val Thr
                65                  70                  75

Gln Val Thr Ser Leu Trp Leu Ala His Asn Glu Val Arg Thr Val
                80                  85                  90

Glu Pro Gly Ala Leu Ala Val Leu Ser Gln Leu Lys Asn Leu Asp
                95                  100                 105

Leu Ser His Asn Phe Ile Ser Ser Phe Pro Trp Ser Asp Leu Arg
                110                 115                 120

Asn Leu Ser Ala Leu Gln Leu Leu Lys Met Asn His Asn Arg Leu
                125                 130                 135

Gly Ser Leu Pro Arg Asp Ala Leu Gly Ala Leu Pro Asp Leu Arg
                140                 145                 150

Ser Leu Arg Ile Asn Asn Asn Arg Leu Arg Thr Leu Ala Pro Gly
                155                 160                 165

Thr Phe Asp Ala Leu Ser Ala Leu Ser His Leu Gln Leu Tyr His
                170                 175                 180

Asn Pro Phe His Cys Gly Cys Gly Leu Val Trp Leu Gln Ala Trp
                185                 190                 195

Ala Ala Ser Thr Arg Val Ser Leu Pro Glu Pro Asp Ser Ile Ala
                200                 205                 210

Cys Ala Ser Pro Pro Ala Leu Gln Gly Val Pro Val Tyr Arg Leu
                215                 220                 225

Pro Ala Leu Pro Cys Ala Pro Pro Ser Val His Leu Ser Ala Glu
                230                 235                 240

Pro Pro Leu Glu Ala Pro Gly Thr Pro Leu Arg Ala Gly Leu Ala
                245                 250                 255

Phe Val Leu His Cys Ile Ala Asp Gly His Pro Thr Pro Arg Leu
                260                 265                 270

Gln Trp Gln Leu Gln Ile Pro Gly Gly Thr Val Val Leu Glu Pro
                275                 280                 285

Pro Val Leu Ser Gly Glu Asp Asp Gly Val Gly Ala Glu Glu Gly
                290                 295                 300

Glu Gly Glu Gly Asp Gly Asp Leu Leu Thr Gln Thr Gln Ala Gln
                305                 310                 315

Thr Pro Thr Pro Ala Pro Ala Trp Pro Ala Pro Pro Ala Thr Pro
                320                 325                 330

Arg Phe Leu Ala Leu Ala Asn Gly Ser Leu Leu Val Pro Leu Leu
                335                 340                 345

Ser Ala Lys Glu Ala Gly Val Tyr Thr Cys Arg Ala His Asn Glu
```

```
                350                 355                 360

Leu Gly Ala Asn Ser Thr Ser Ile Arg Val Ala Val Ala Ala Thr
                365                 370                 375

Gly Pro Pro Lys His Ala Pro Gly Ala Gly Gly Glu Pro Asp Gly
                380                 385                 390

Gln Ala Pro Thr Ser Glu Arg Lys Ser Thr Ala Lys Gly Arg Gly
                395                 400                 405

Asn Ser Val Leu Pro Ser Lys Pro Glu Gly Lys Ile Lys Gly Gln
                410                 415                 420

Gly Leu Ala Lys Val Ser Ile Leu Gly Glu Thr Glu Thr Glu Pro
                425                 430                 435

Glu Glu Asp Thr Ser Glu Gly Glu Glu Ala Glu Asp Gln Ile Leu
                440                 445                 450

Ala Asp Pro Ala Glu Glu Gln Arg Cys Gly Asn Gly Asp Pro Ser
                455                 460                 465

Arg Tyr Val Ser Asn His Ala Phe Asn Gln Ser Ala Glu Leu Lys
                470                 475                 480

Pro His Val Phe Glu Leu Gly Val Ile Ala Leu Asp Val Ala Glu
                485                 490                 495

Arg Glu Ala Arg Val Gln Leu Thr Pro Leu Ala Ala Arg Trp Gly
                500                 505                 510

Pro Gly Pro Gly Gly Ala Gly Gly Ala Pro Arg Pro Gly Arg Arg
                515                 520                 525

Pro Leu Arg Leu Leu Tyr Leu Cys Pro Ala Gly Gly Gly Ala Ala
                530                 535                 540

Val Gln Trp Ser Arg Val Glu Glu Gly Val Asn Ala Tyr Trp Phe
                545                 550                 555

Arg Gly Leu Arg Pro Gly Thr Asn Tyr Ser Val Cys Leu Ala Leu
                560                 565                 570

Ala Gly Glu Ala Cys His Val Gln Val Val Phe Ser Thr Lys Lys
                575                 580                 585

Glu Leu Pro Ser Leu Leu Val Ile Val Ala Val Ser Val Phe Leu
                590                 595                 600

Leu Val Leu Ala Thr Val Pro Leu Leu Gly Ala Ala Cys Cys His
                605                 610                 615

Leu Leu Ala Lys His Pro Gly Lys Pro Tyr Arg Leu Ile Leu Arg
                620                 625                 630

Pro Gln Ala Pro Asp Pro Met Glu Lys Arg Ile Ala Ala Asp Phe
                635                 640                 645

Asp Pro Arg Ala Ser Tyr Leu Glu Ser Glu Lys Ser Tyr Pro Ala
                650                 655                 660

Gly Gly Glu Ala Gly Gly Glu Glu Pro Glu Asp Val Gln Gly Glu
                665                 670                 675
```

```
Gly Leu Asp Glu Asp Ala Glu Gln Gly Asp Pro Ser Gly Asp Leu
                680                 685                 690

Gln Arg Glu Glu Ser Leu Ala Ala Cys Ser Leu Val Glu Ser Gln
                695                 700                 705

Ser Lys Ala Asn Gln Glu Glu Phe Glu Ala Gly Ser Glu Tyr Ser
                710                 715                 720

Asp Arg Leu Pro Leu Gly Ala Glu Ala Val Asn Ile Ala Gln Glu
                725                 730                 735

Ile Asn Gly Asn Tyr Arg Gln Thr Ala Gly
                740                 745
```

```
<210> 69
<211> 2916
<212> DNA
<213> Homo Sapien

<400> 69
ggcggcggga gcagcgaagg gggcggcagg gatcctccag gctgccggct 50

gggaaggcgt gggcgacccg gtgtgtggcg cgcccagagc cccgcgtttc 100

agccctaggg aaggaagcca gttgagggaa gttctccatg aatgtacgtc 150

acaatgatga tgaccgacca aatccctctg gaactgccac cattgctgaa 200

cggagaggta gccatgatgc cccacttggt gaatggagat gcagctcagc 250

atgttattct cgttcaagtt aatccaggtg agactttcac aataagagca 300

gaggatggaa cacttcagtg cattcaagga cctgctgaag ttcccatgat 350

gtcacccaat ggatccattc ctcccattca tgtgcctcca ggttatatct 400

cacaggtgat tgaagatagt actggagtcc gccgggtggt ggtcacaccc 450

cagtctcctg agtgttatcc cccaagctac ccctcagcca tgtctccaac 500

ccatcatctc cctccctatc tgactcacca tccacatttt attcataact 550

cacacacggc ttactaccca cctgttaccg gacctggaga tatgccgcct 600

cagtttttc cccagcatca tcttccccac acaatatatg gtgagcaaga 650

aattatacca ttttatggaa tgtcaagcta catcacccga gaagaccagt 700

acagcaagcc tccgcacaaa aaactgaaag accgccagat cgatcgccag 750

aaccgcctca acagccctcc ttcttctatc tacaaaagca gctgcacaac 800

agtatacaat ggctatggga agggccatag tggtggaagt ggcggaggcg 850

gcagcggtag tggtcccgga attaagaaaa cagagcgacg agcaagaagc 900

agcccaaagt cgaatgattc agacttgcaa gaatatgagt tggaagtaaa 950

gagggtgcaa gacattcttt cgggaataga gaaaccacag gtttctaata 1000

ttcaggcaag agcagttgtg ttgtcctggg ctccccctgt tggactttcc 1050
```

tgtggacccc acagtggtct ttccttcccc tacagttacg aggtggcctt 1100
atcagacaaa ggacgagatg gaaaatacaa gataatttac agtggagaag 1150
aattagaatg taacctgaaa gatcttagac cagcaacaga ttatcatgtg 1200
agggtgtatg ccatgtacaa ttccgtaaag ggatcctgct ccgagcctgt 1250
tagcttcacc acccacagct gtgcacccga gtgtcctttc ccccctaagc 1300
tggcacatag gagcaaaagt tcactaaccc tgcagtggaa ggcaccaatt 1350
gacaacggtt caaaaatcac caactacctt ttagagtggg atgagggaaa 1400
aagaaatagt ggtttcagac agtgcttctt cgggagccag aagcactgca 1450
agttgacaaa gctttgtccg gcaatggggt acacattcag gctggccgct 1500
cgaaacgaca ttggcaccag tggttatagc caagaggtgg tgtgctacac 1550
attaggaaat atccctcaga tgccttctgc accaaggctg gttcgagctg 1600
gcatcacatg ggtcacgttg cagtggagta agccagaagg ctgttcaccc 1650
gaggaagtga tcacctacac cttggaaatt caggaggatg aaaatgataa 1700
ccttttccac ccaaaataca ctggagagga tttaacctgt actgtgaaaa 1750
atctcaaaag aagcacacag tataaattca ggctgactgc ttctaatacg 1800
gaaggaaaaa gctgtccaag cgaagttctt gtttgtacga cgagtcctga 1850
caggcctgga cctcctacca gaccgcttgt caaaggccca gttacatctc 1900
atggctttag tgtcaaatgg gatcccccta aggacaatgg tggttcagaa 1950
atcctcaagt acttgctaga gattactgat ggaaattctg aaggtgaagt 2000
ttttggcaat tgttttattc aaatccaata gcaagctctg ttttctaata 2050
tagtaaatgt ctttatagta atagtgagta atcattaatt ctaaagatag 2100
aattattatt acaataaaca aactttagtc acatattggc agtttttcta 2150
tttcaaacac agcaccagag atcagagtct acttgaaact tacatttgtg 2200
ttatttaaca atttttctgt atctttttca ttggtgtttt gttttgttta 2250
tcttttgttt ttgtttcttt ggtttggttt gtttttgttt tgtttttga 2300
gatacgatct ctgtcacaca ggctggaggg cagtggcaca gacatggccc 2350
attgcagtct cagactcctg ggcttaagtg actcttctgc cacagaagat 2400
gaggaagaat acatttttca tagtgatggg gtctcactat gttatctagg 2450
ctggtctcaa actcctggcc tcaagcaacc ctccaccttg gcctcccaaa 2500
gtgctgggac tatagacatg aatcaccaca ctcagcttcc atgtcttttt 2550
atgaactagg gttcctaatt aatcagataa atttggtatt ttcatctcct 2600
aacttgccat atgttttctg gaaattctta taagcagccg agagtggtgg 2650

ctcacgctgt agtcccagca ctttgggagg ctgaggtggg tggtcaggag 2700

atcaagacca tcctggccaa catggtgaaa ccccgtctct actaaaaata 2750

caaaaattag ctgggtgtgg tggcaggcac ctgtagtccc agctacttgg 2800

gaggctgagg cagaagaatt gcttgaaccc agcaggcgga ggttgcagtg 2850

agctgagatt gcaccactgc actccagcct ggtgacagag tgagactctg 2900

tctcaaaaaa aaaaaa 2916

<210> 70
<211> 625
<212> PRT
<213> Homo Sapien

<400> 70
```
Met Met Met Thr Asp Gln Ile Pro Leu Glu Leu Pro Pro Leu Leu
  1               5                  10                  15

Asn Gly Glu Val Ala Met Met Pro His Leu Val Asn Gly Asp Ala
                 20                  25                  30

Ala Gln His Val Ile Leu Val Gln Val Asn Pro Gly Glu Thr Phe
                 35                  40                  45

Thr Ile Arg Ala Glu Asp Gly Thr Leu Gln Cys Ile Gln Gly Pro
                 50                  55                  60

Ala Glu Val Pro Met Met Ser Pro Asn Gly Ser Ile Pro Pro Ile
                 65                  70                  75

His Val Pro Pro Gly Tyr Ile Ser Gln Val Ile Glu Asp Ser Thr
                 80                  85                  90

Gly Val Arg Arg Val Val Val Thr Pro Gln Ser Pro Glu Cys Tyr
                 95                 100                 105

Pro Pro Ser Tyr Pro Ser Ala Met Ser Pro Thr His His Leu Pro
                110                 115                 120

Pro Tyr Leu Thr His His Pro His Phe Ile His Asn Ser His Thr
                125                 130                 135

Ala Tyr Tyr Pro Pro Val Thr Gly Pro Gly Asp Met Pro Pro Gln
                140                 145                 150

Phe Phe Pro Gln His His Leu Pro His Thr Ile Tyr Gly Glu Gln
                155                 160                 165

Glu Ile Ile Pro Phe Tyr Gly Met Ser Ser Tyr Ile Thr Arg Glu
                170                 175                 180

Asp Gln Tyr Ser Lys Pro Pro His Lys Lys Leu Lys Asp Arg Gln
                185                 190                 195

Ile Asp Arg Gln Asn Arg Leu Asn Ser Pro Pro Ser Ser Ile Tyr
                200                 205                 210

Lys Ser Ser Cys Thr Thr Val Tyr Asn Gly Tyr Gly Lys Gly His
                215                 220                 225

Ser Gly Gly Ser Gly Gly Gly Gly Ser Gly Ser Gly Pro Gly Ile
```

```
                230                 235                 240

Lys Lys Thr Glu Arg Arg Ala Arg Ser Ser Pro Lys Ser Asn Asp
                245                 250                 255

Ser Asp Leu Gln Glu Tyr Glu Leu Glu Val Lys Arg Val Gln Asp
                260                 265                 270

Ile Leu Ser Gly Ile Glu Lys Pro Gln Val Ser Asn Ile Gln Ala
                275                 280                 285

Arg Ala Val Val Leu Ser Trp Ala Pro Pro Val Gly Leu Ser Cys
                290                 295                 300

Gly Pro His Ser Gly Leu Ser Phe Pro Tyr Ser Tyr Glu Val Ala
                305                 310                 315

Leu Ser Asp Lys Gly Arg Asp Gly Lys Tyr Lys Ile Ile Tyr Ser
                320                 325                 330

Gly Glu Glu Leu Glu Cys Asn Leu Lys Asp Leu Arg Pro Ala Thr
                335                 340                 345

Asp Tyr His Val Arg Val Tyr Ala Met Tyr Asn Ser Val Lys Gly
                350                 355                 360

Ser Cys Ser Glu Pro Val Ser Phe Thr Thr His Ser Cys Ala Pro
                365                 370                 375

Glu Cys Pro Phe Pro Pro Lys Leu Ala His Arg Ser Lys Ser Ser
                380                 385                 390

Leu Thr Leu Gln Trp Lys Ala Pro Ile Asp Asn Gly Ser Lys Ile
                395                 400                 405

Thr Asn Tyr Leu Leu Glu Trp Asp Glu Gly Lys Arg Asn Ser Gly
                410                 415                 420

Phe Arg Gln Cys Phe Phe Gly Ser Gln Lys His Cys Lys Leu Thr
                425                 430                 435

Lys Leu Cys Pro Ala Met Gly Tyr Thr Phe Arg Leu Ala Ala Arg
                440                 445                 450

Asn Asp Ile Gly Thr Ser Gly Tyr Ser Gln Glu Val Val Cys Tyr
                455                 460                 465

Thr Leu Gly Asn Ile Pro Gln Met Pro Ser Ala Pro Arg Leu Val
                470                 475                 480

Arg Ala Gly Ile Thr Trp Val Thr Leu Gln Trp Ser Lys Pro Glu
                485                 490                 495

Gly Cys Ser Pro Glu Glu Val Ile Thr Tyr Thr Leu Glu Ile Gln
                500                 505                 510

Glu Asp Glu Asn Asp Asn Leu Phe His Pro Lys Tyr Thr Gly Glu
                515                 520                 525

Asp Leu Thr Cys Thr Val Lys Asn Leu Lys Arg Ser Thr Gln Tyr
                530                 535                 540

Lys Phe Arg Leu Thr Ala Ser Asn Thr Glu Gly Lys Ser Cys Pro
                545                 550                 555
```

```
Ser Glu Val Leu Val Cys Thr Thr Ser Pro Asp Arg Pro Gly Pro
                560                 565                 570

Pro Thr Arg Pro Leu Val Lys Gly Pro Val Thr Ser His Gly Phe
                575                 580                 585

Ser Val Lys Trp Asp Pro Pro Lys Asp Asn Gly Gly Ser Glu Ile
                590                 595                 600

Leu Lys Tyr Leu Leu Glu Ile Thr Asp Gly Asn Ser Glu Gly Glu
                605                 610                 615

Val Phe Gly Asn Cys Phe Ile Gln Ile Gln
                620                 625
```

<210> 71
<211> 3732
<212> DNA
<213> Homo Sapien

<400> 71

```
aagtcattca gtggatgtga tcttggctca caggggacga tgtcaagctc 50

ttcctggctc cttctcagcc ttgttgctgt aactgctgct cagtccacca 100

ttgaggaaca ggccaagaca tttttggaca agtttaacca cgaagccgaa 150

gacctgttct atcaaagttc acttgcttct tggaattata acaccaatat 200

tactgaagag aatgtccaaa acatgaataa tgctggggac aaatggtctg 250

cctttttaaa ggaacagtcc acacttgccc aaatgtatcc actacaagaa 300

attcagaatc tcacagtcaa gcttcagctg caggctcttc agcaaaatgg 350

gtcttcagtg ctctcagaag acaagagcaa acggttgaac acaattctaa 400

atacaatgag caccatctac agtactggaa aagtttgtaa cccagataat 450

ccacaagaat gcttattact tgaaccaggt ttgaatgaaa taatggcaaa 500

cagtttagac tacaatgaga ggctctgggc ttgggaaagc tggagatctg 550

aggtcggcaa gcagctgagg ccattatatg aagagtatgt ggtcttgaaa 600

aatgagatgg caagagcaaa tcattatgag gactatgggg attattggag 650

aggagactat gaagtaaatg gggtagatgg ctatgactac agccgcggcc 700

agttgattga agatgtggaa catacctttg aagagattaa accattatat 750

gaacatcttc atgcctatgt gagggcaaag ttgatgaatg cctatccttc 800

ctatatcagt ccaattggat gcctccctgc tcatttgctt ggtgatatgt 850

ggggtagatt ttggacaaat ctgtactctt tgacagttcc ctttggacag 900

aaaccaaaca tagatgttac tgatgcaatg gtggaccagg cctgggatgc 950

acagagaata ttcaaggagg ccgagaagtt ctttgtatct gttggtcttc 1000

ctaatatgac tcaaggattc tgggaaaatt ccatgctaac ggacccagga 1050
```

aatgttcaga aagcagtctg ccatcccaca gcttgggacc tggggaaggg 1100
cgacttcagg atccttatgt gcacaaaggt gacaatggac gacttcctga 1150
cagctcatca tgagatgggg catatccagt atgatatggc atatgctgca 1200
caacctttc tgctaagaaa tggagctaat gaaggattcc atgaagctgt 1250
tggggaaatc atgtcacttt ctgcagccac acctaagcat ttaaaatcca 1300
ttggtcttct gtcacccgat tttcaagaag acaatgaaac agaaataaac 1350
ttcctgctca aacaagcact cacgattgtt gggactctgc catttactta 1400
catgttagag aagtggaggt ggatggtctt taaaggggaa attcccaaag 1450
accagtggat gaaaaagtgg tgggagatga agcgagagat agttggggtg 1500
gtggaacctg tgccccatga tgaaacatac tgtgaccccg catctctgtt 1550
ccatgtttct gatgattact cattcattcg atattacaca aggacccttt 1600
accaattcca gtttcaagaa gcactttgtc aagcagctaa acatgaaggc 1650
cctctgcaca aatgtgacat ctcaaactct acagaagctg gacagaaact 1700
gttgtaagaa atacctcaaa atgttgaacc tctcctagta ttcagtatta 1750
ctcatttcca tgcctaggtt tgtatttgat ttctttgttc taaaaagaaa 1800
attttatggc ctcaaaatgt cctcatttac aaaccaaaca tttaatttgt 1850
ggtcagacag gaacctagac catacaacaa ttgggtgggc cacctctttt 1900
ctccctatca taactacagc cctctcttcc tggtaattgg aaggaaagag 1950
cggtttaggg tggaatatat ctgttaatat gcattctttt cttatctgcc 2000
agaagcaaat ttagccaagt caaagagaag aaaccataga tcatagatgt 2050
aaatatatgt acatctggaa cccctcaaaa ggccctgaac cccctttttt 2100
tgtgtagcaa tatgctgagg cttggaaaat cagaaccctg gaccctagca 2150
ttggaaaatg ttgtaggagc aagaacatga atgtaaggcc actgctcaac 2200
tactttgagc ccttatttac ctggctgaaa gaccagaaca agaattcttt 2250
tgtgggatgg agtaccgact ggagtccata tgcagaccca aagcatcaaa 2300
gtgaggataa gcctaaaatc agctcttgga gataaagcat atgaatggaa 2350
cgacaatgaa atgtacctgt tccgatcatc tgttgcatat gctatgaggc 2400
agtacttttt aaaagtaaaa aatcagatga ttctttttgg ggaggaggat 2450
gtgcgagtgg ctaatttgaa accaagaatc tcctttaatt tctttgtcac 2500
tgcacctaaa aatgtgtctg atatcattcc tagaactgaa gttgaaaagg 2550
ccatcaggat gtcccggagc cgtatcaatg atgctttccg tctgaatgac 2600
aacagcctag agtttctggg gatacagcca acacttggac ctcctaacca 2650

```
gccccctgtt tccatatggc tgattgtttt tggagttgtg atgggagtga 2700
tagtggttgg cattgtcatc ctgatcttca ctgggatcag agatcggaag 2750
aagaaaaata aagcaagaag tggagaaaat ccttatgcct ccatcgatat 2800
tagcaaagga gaaaataatc caggattcca aaacactgat gatgttcaga 2850
cctcctttta gaaaaatcta tgtttttcct cttgaggtga ttttgttgta 2900
tgtaaatgtt aatttcatgg tatagaaaat ataagatgat aaagatatca 2950
ttaaatgtca aaactatgac tctgttcaga aaaaaaattg tccaaagaca 3000
acatggccaa ggagagagca tcttcattga cattgctttc agtatttatt 3050
tctgtctctg gatttgactt ctgttctgtt tcttaataag gattttgtat 3100
tagagtatat tagggaaagt gtgtatttgg tctcacaggc tgttcaggga 3150
taatctaaat gtaaatgtct gttgaatttc tgaagttgaa aacaaggata 3200
tatcattgga gcaagtgttg gatcttgtat ggaatatgga tggatcactt 3250
gtaaggacag tgcctgggaa ctggtgtagc tgcaaggatt gagaatggca 3300
tgcattagct cactttcatt taatccattg tcaaggatga catgctttct 3350
tcacagtaac tcagttcaag tactatggtg atttgcctac agtgatgttt 3400
ggaatcgatc atgctttctt caaggtgaca ggtctaaaga gagaagaatc 3450
cagggaacag gtagaggaca ttgcttttc acttccaagg tgcttgatca 3500
acatctccct gacaacacaa aactagagcc aggggcctcc gtgaactccc 3550
cagagcatgc ctgatagaaa ctcatttcta ctgttctcta actgtggagt 3600
gaatggaaat tccaactgta tgttcaccct ctgaagtggg tacccagtct 3650
cttaaatctt ttgtatttgc tcacagtgtt tgagcagtgc tgagcacaaa 3700
gcagacactc aataaatgct agatttacaa aa 3732
```

<210> 72
<211> 555
<212> PRT
<213> Homo Sapien

<400> 72

```
Met Ser Ser Ser Ser Trp Leu Leu Leu Ser Leu Val Ala Val Thr
 1               5                  10                  15

Ala Ala Gln Ser Thr Ile Glu Glu Gln Ala Lys Thr Phe Leu Asp
                20                  25                  30

Lys Phe Asn His Glu Ala Glu Asp Leu Phe Tyr Gln Ser Ser Leu
                35                  40                  45

Ala Ser Trp Asn Tyr Asn Thr Asn Ile Thr Glu Glu Asn Val Gln
                50                  55                  60

Asn Met Asn Asn Ala Gly Asp Lys Trp Ser Ala Phe Leu Lys Glu
                65                  70                  75
```

```
Gln Ser Thr Leu Ala Gln Met Tyr Pro Leu Gln Glu Ile Gln Asn
                 80                  85                  90

Leu Thr Val Lys Leu Gln Leu Gln Ala Leu Gln Gln Asn Gly Ser
                 95                 100                 105

Ser Val Leu Ser Glu Asp Lys Ser Lys Arg Leu Asn Thr Ile Leu
                110                 115                 120

Asn Thr Met Ser Thr Ile Tyr Ser Thr Gly Lys Val Cys Asn Pro
                125                 130                 135

Asp Asn Pro Gln Glu Cys Leu Leu Leu Glu Pro Gly Leu Asn Glu
                140                 145                 150

Ile Met Ala Asn Ser Leu Asp Tyr Asn Glu Arg Leu Trp Ala Trp
                155                 160                 165

Glu Ser Trp Arg Ser Glu Val Gly Lys Gln Leu Arg Pro Leu Tyr
                170                 175                 180

Glu Glu Tyr Val Val Leu Lys Asn Glu Met Ala Arg Ala Asn His
                185                 190                 195

Tyr Glu Asp Tyr Gly Asp Tyr Trp Arg Gly Asp Tyr Glu Val Asn
                200                 205                 210

Gly Val Asp Gly Tyr Asp Tyr Ser Arg Gly Gln Leu Ile Glu Asp
                215                 220                 225

Val Glu His Thr Phe Glu Glu Ile Lys Pro Leu Tyr Glu His Leu
                230                 235                 240

His Ala Tyr Val Arg Ala Lys Leu Met Asn Ala Tyr Pro Ser Tyr
                245                 250                 255

Ile Ser Pro Ile Gly Cys Leu Pro Ala His Leu Leu Gly Asp Met
                260                 265                 270

Trp Gly Arg Phe Trp Thr Asn Leu Tyr Ser Leu Thr Val Pro Phe
                275                 280                 285

Gly Gln Lys Pro Asn Ile Asp Val Thr Asp Ala Met Val Asp Gln
                290                 295                 300

Ala Trp Asp Ala Gln Arg Ile Phe Lys Glu Ala Glu Lys Phe Phe
                305                 310                 315

Val Ser Val Gly Leu Pro Asn Met Thr Gln Gly Phe Trp Glu Asn
                320                 325                 330

Ser Met Leu Thr Asp Pro Gly Asn Val Gln Lys Ala Val Cys His
                335                 340                 345

Pro Thr Ala Trp Asp Leu Gly Lys Gly Asp Phe Arg Ile Leu Met
                350                 355                 360

Cys Thr Lys Val Thr Met Asp Asp Phe Leu Thr Ala His His Glu
                365                 370                 375

Met Gly His Ile Gln Tyr Asp Met Ala Tyr Ala Ala Gln Pro Phe
                380                 385                 390
```

Leu Leu Arg Asn Gly Ala Asn Glu Gly Phe His Glu Ala Val Gly
395 400 405

Glu Ile Met Ser Leu Ser Ala Ala Thr Pro Lys His Leu Lys Ser
410 415 420

Ile Gly Leu Leu Ser Pro Asp Phe Gln Glu Asp Asn Glu Thr Glu
425 430 435

Ile Asn Phe Leu Leu Lys Gln Ala Leu Thr Ile Val Gly Thr Leu
440 445 450

Pro Phe Thr Tyr Met Leu Glu Lys Trp Arg Trp Met Val Phe Lys
455 460 465

Gly Glu Ile Pro Lys Asp Gln Trp Met Lys Lys Trp Trp Glu Met
470 475 480

Lys Arg Glu Ile Val Gly Val Val Glu Pro Val Pro His Asp Glu
485 490 495

Thr Tyr Cys Asp Pro Ala Ser Leu Phe His Val Ser Asp Asp Tyr
500 505 510

Ser Phe Ile Arg Tyr Tyr Thr Arg Thr Leu Tyr Gln Phe Gln Phe
515 520 525

Gln Glu Ala Leu Cys Gln Ala Ala Lys His Glu Gly Pro Leu His
530 535 540

Lys Cys Asp Ile Ser Asn Ser Thr Glu Ala Gly Gln Lys Leu Leu
545 550 555

<210> 73
<211> 2120
<212> DNA
<213> Homo Sapien

<400> 73
cccacgcgtc cgagcggggt ggacaagtgg cgtgtgtgct gcgaccccga 50

gggaagatga acgggacgcg gaactggtgt accctggtgg acgtgcaccc 100

agaggaccag gcggcggcgg gcaggaagac ctatgccatg gtgtccagcc 150

actcagctgg tcattctctg gcttcagaac tggtggagtc ccatgatgga 200

catgaggaga tcattaaggt gtacttgaag gggaggtctg gagacaagat 250

gattcacgag aagaatatta accagctgaa gagtgaggtc cagtacatcc 300

aggaggccag gaactgccta cagaagctcc gggaggatat aagtagcaag 350

cttgacagga acctaggaga ttctctccat cgacaggaga tacaggtggt 400

gctagaaaag ccaaatggct ttagtcagag tcccacagcc ctgtacagca 450

gcccacctga ggtggacacc tgtataaatg aggatgttga gagcttgagg 500

aagacggtgc aggacttgct ggccaagctt caggaggcca agcggcaaca 550

ccagtcagac tgtgtggctt ttgaggtcac actcagccgg taccagaggg 600

aagcagaaca aagtaatgtg gcccttcaga gagaggagga cagatgtcca 650

gagtgattgg agaatgtcct gggggaatga agttccttcc acaaacacag 700
ctcagttctt agcaacaaac tgtttgtttt tctacttgct ccatctgcag 750
cctacgctgc cctggcctcc tgcagacaga tagtggggtt acctggcaag 800
gcctggtgag agccagtgaa cctaagcttt gactgggtgg ccttgtcttt 850
ctggggagga gggaatgtac attcagggag tagccttttg cggaaaaatt 900
ctctagggct acagacagtc atgtgtgact tctctctgct gtgaaaactc 950
ccagagtctc tttagggatt ttccctaagg tgtaccacca ggcacacctc 1000
agtcttcttg acccagagcc tgaaaactgt tttcactggg ttccaccagt 1050
cccagcaaaa tcctctttgt atttattttg ctaagttatt ggtggttttg 1100
cttacatctc atgattgata taataccaaa gttctatagc cttctcttgc 1150
agtatttgga tttgcttgaa accgggaaaa ctgttcccat taggcttgtt 1200
aatgtcagag tgacactatt atgaatcttt ctctcccttt cctctgcctg 1250
tttcttctct ctttctcctt caaacttgct ctgcagctaa ggaaggtgag 1300
tctactttcc ctgaggcttt ggggtcagag tatatgttgt ttggagaaag 1350
agggcaatca ggactcttct gggacccaga tgagttcttc actagccctt 1400
ctgaacccct tgctccataa ttggtctttt atcctggctc tgaatgaccc 1450
tgcaggtcat catggttttc tttttttatt gttttttttt ttttctgaga 1500
cagagtctca ctctgtcacc caggctggag tgcagtggcg cgatctcagc 1550
tcactgcaac ctctgcctcc cggatttaag cgattcttct gcctcagcct 1600
cccgagtagc tgggactaca ggtgtgccac cacgcctggc tgatttttgt 1650
atttttagta gagatggggt ttcaccatac tggctaggct ggtctcgaat 1700
tcctgacctc aggtgatcca cccacctcgg cttcccaaag tgctaggatt 1750
ataggcttga gctactgcgc ccggcccatg gtgtttttct ttagggctct 1800
tcctacagcc ttgagaagta gataggcatc agagtatggt actataggaa 1850
tcagaaaaat tcaaaacaaa tgtggattaa gtgtttaggc tctatgtggc 1900
tcacgcagcc agaatcctta agtctgtgtg tttctgtgtc tcaagactgg 1950
gctcacattc tggctttgtc cataacaatg ctctgggatt tcagggagtt 2000
ccctcatttg taaaatgagg gggtcagagc aggtgatatc catgtttctt 2050
ccctttctga tattgttgtc tgtggcatat tctttgtatg gcgaatttaa 2100
taaattatat taatgtgtca 2120

<210> 74
<211> 199
<212> PRT

<213> Homo Sapien

<400> 74
Met Asn Gly Thr Arg Asn Trp Cys Thr Leu Val Asp Val His Pro
1 5 10 15

Glu Asp Gln Ala Ala Ala Gly Arg Lys Thr Tyr Ala Met Val Ser
20 25 30

Ser His Ser Ala Gly His Ser Leu Ala Ser Glu Leu Val Glu Ser
35 40 45

His Asp Gly His Glu Glu Ile Ile Lys Val Tyr Leu Lys Gly Arg
50 55 60

Ser Gly Asp Lys Met Ile His Glu Lys Asn Ile Asn Gln Leu Lys
65 70 75

Ser Glu Val Gln Tyr Ile Gln Glu Ala Arg Asn Cys Leu Gln Lys
80 85 90

Leu Arg Glu Asp Ile Ser Ser Lys Leu Asp Arg Asn Leu Gly Asp
95 100 105

Ser Leu His Arg Gln Glu Ile Gln Val Val Leu Glu Lys Pro Asn
110 115 120

Gly Phe Ser Gln Ser Pro Thr Ala Leu Tyr Ser Ser Pro Pro Glu
125 130 135

Val Asp Thr Cys Ile Asn Glu Asp Val Glu Ser Leu Arg Lys Thr
140 145 150

Val Gln Asp Leu Leu Ala Lys Leu Gln Glu Ala Lys Arg Gln His
155 160 165

Gln Ser Asp Cys Val Ala Phe Glu Val Thr Leu Ser Arg Tyr Gln
170 175 180

Arg Glu Ala Glu Gln Ser Asn Val Ala Leu Gln Arg Glu Glu Asp
185 190 195

Arg Cys Pro Glu

<210> 75
<211> 3192
<212> DNA
<213> Homo Sapien

<220>
<221> unsure
<222> 1428, 1431
<223> unknown base

<400> 75
gcttgcacac atggctccgg aggctccggt tgcccatccg agcccctgcc 50

aggctctaac gttcccaact gacaacacca gtaactaaat ataggagcag 100

atggtgggga cgggctgtcg cagcggctcc tttgcagagg tctccggact 150

gcagataagg ctcaggccct tttgtgagaa gcagaccagc ctgggggctg 200

gcggcaggac acctgtgtct gcatgctgaa gaagatgggt gaggccgtgg 250
ccagagtagc aaggaaggtc aacgagacgg tggagagcgg ctctgacact 300
ctggacctgg ccgagtgcaa gctggtctcc tttcccattg gcatctacaa 350
ggtcctgcgg aatgtctctg gccagatcca cctcatcacc ctggctaaca 400
acgagcttaa gtccctcacc agcaagttca tgaccacatt cagtcagctc 450
cgagagctcc acctggaggg gaacttccta caccgcctcc ccagcgaggt 500
cagtgccctg cagcacctca aggccattga cctgtcccgg aaccagttcc 550
aggacttccc tgagcagctt accgccctgc cggcgctgga gaccatcaac 600
ctggaggaga acgagatcgt agatgtgccc gtggagaagc tggccgccat 650
gccagccttg cgcagcatca acctccgctt caacccactc aacgccgagg 700
tgcgcgtgat cgccccgccg ctcatcaagt ttgacatgct catgtctccg 750
gaaggcgcaa gagccccct accttaggcc accctcctca tgcccaccca 800
gcaagggaca gaggccacag gcctggaacc ctggaaggga gggaggccca 850
tgggaggcca agcctggggg ctgggggcgg gtgggccgag cagcacgtgg 900
tgggtggggt gcagctggtc tggatagata gcttacagca gtagtgggct 950
ctggaatgcc caagggaaga ggcaaggtgg ggcctgcagc ctggactcgg 1000
cactcacagc tgctgtgcaa actcaggcag atctcctgcc ctctctgagc 1050
cttgtcactt gaaaaaaaca ggaccctttc cctcctttgg gctccctgga 1100
ggtttttaag cagtacgtgc ctccaagtta cctccagatc agcaggcaca 1150
ggtgggcatt gccaggtatt ttctgagccc ctgcgggttt gaggccttgt 1200
ttttagtgct gagagccagt tgctgccctg agaagagaag acaacctcca 1250
tctatttatt gcttcctgag aactgacctg gatgcggccc tctgcagggc 1300
ccagtcttca gtcctgtggt ccctggactg gtgggaacct gaactaggag 1350
tcctgggaga gctgtggtgg gaatatgggc tggcactgct gcagggcaag 1400
aacattcatg taggagcccg aggaccanca ngctgggaat ggggagcaag 1450
tcacgtcagc tctgtcattc cccacagtta acaaattggc ggggtgggaa 1500
gtcctgagtg ctccgtccct ctagcatcac tcctgagctg cgggagaggt 1550
ggcccagaga acagcagagt cagttacacc tgcagctctt gtctaaagtg 1600
attagatggc caccctcacc actgtccagt ccagcagcag cctggctgcc 1650
ttgtcatggc ctcctggggg cagaaggcga tgtggaccac gggatttgta 1700
gccagccagc tcccaggcca acgcccaaag ccctgatgac ctggttcttc 1750
tgaggccctc aacctggcat cttagggtat ggtcaggcaa cagggtgacc 1800

agctgtcctg gtttcccagg acatggaact ttcaatgcta aaactgggac 1850

attacccagc aagtggggat ggttggtccc ctaccaggag agggcctggg 1900

gctcttgctt cccgagaacg cctgtggctt gaagaacctt gactgcttgg 1950

tcctcaggta tctacctccc accttctcct catctgtgga gcaagccaac 2000

tcagtgcccc agaccccacc tgatctgcat ctttgtttgc tccagagaca 2050

cctgaggccc cagagcttga ggcaaagcca ggccgtccaa atcctgtgtg 2100

ccgtggacga gtggccactt tactactcct aaggctaaga tgttgagagc 2150

tcagaccact gctcagagca gtaatccctg ctcagaatgc tcccagttcc 2200

ctcgtccctg cccaggtctc ttgtctcttg ggaaggaact gataggtcgg 2250

gccattgttg ggccatcact gagcgctcag tatctcaaga gactctgttc 2300

attctgctcg tatcccaagg cctggttggt caaactctgg gcaaagggtt 2350

ttcaggatga ggaggtcaag acaggatgtc cagagctacc gagttcatct 2400

gtgggtgttg gggcaagtg ggggctgaag tcctgtgcag gctgcgctgg 2450

ccccacctgc cttgtgccct ggagtggggt ttctccttgt tgaagaagag 2500

gcatccttct ctgatgtgca caaacacaat gtatgaccag agccttgcaa 2550

ctcaaagtgt ggtctgtgga ccagcagcgg cagtgacacc tgggagcttg 2600

ttaggaatgc agagtctagg cctcacccta tacctcccga ctcagaccct 2650

gcattttagc aagaccccca gctgattcct ataagcactt tagagtttga 2700

gaagcaagga cctaggctgg ggatgtcctc cgagcagagg gtgaagtttc 2750

tctcagttct ctccctgcca cttccaggga tctgagcctg tgttcagcct 2800

cctccctaac ccaccctggg agacacttgg cctgttagat tgttccagag 2850

tctgcatggc actcctgaag aagggagtgt gacctgcagt caccaggaga 2900

tgagggttag gtgtgcccag ccctccagac ccggcctttc tggttaaccc 2950

ctgcatgcca agctgcctgc tgccccaggt cctcacctca ggcctttgaa 3000

ggggcagctt ctggaagttg ttttctcctc tgcttggaga gtttgccctt 3050

gtctgtcttg gaaagtgtgg gcagccacag atgcccccaa atcagagctc 3100

acagtgagtg agcccctaag cttcagtctg caataaagaa tgcattggtt 3150

tcaaaaaaaa aaaaaaaaaa aaaaaaaaaa aaaaaaaaaa aa 3192

<210> 76
<211> 184
<212> PRT
<213> Homo Sapien

<400> 76
Met Leu Lys Lys Met Gly Glu Ala Val Ala Arg Val Ala Arg Lys
1 5 10 15

```
Val Asn Glu Thr Val Glu Ser Gly Ser Asp Thr Leu Asp Leu Ala
                20                  25                  30

Glu Cys Lys Leu Val Ser Phe Pro Ile Gly Ile Tyr Lys Val Leu
                35                  40                  45

Arg Asn Val Ser Gly Gln Ile His Leu Ile Thr Leu Ala Asn Asn
                50                  55                  60

Glu Leu Lys Ser Leu Thr Ser Lys Phe Met Thr Thr Phe Ser Gln
                65                  70                  75

Leu Arg Glu Leu His Leu Glu Gly Asn Phe Leu His Arg Leu Pro
                80                  85                  90

Ser Glu Val Ser Ala Leu Gln His Leu Lys Ala Ile Asp Leu Ser
                95                  100                 105

Arg Asn Gln Phe Gln Asp Phe Pro Glu Gln Leu Thr Ala Leu Pro
                110                 115                 120

Ala Leu Glu Thr Ile Asn Leu Glu Glu Asn Glu Ile Val Asp Val
                125                 130                 135

Pro Val Glu Lys Leu Ala Ala Met Pro Ala Leu Arg Ser Ile Asn
                140                 145                 150

Leu Arg Phe Asn Pro Leu Asn Ala Glu Val Arg Val Ile Ala Pro
                155                 160                 165

Pro Leu Ile Lys Phe Asp Met Leu Met Ser Pro Glu Gly Ala Arg
                170                 175                 180

Ala Pro Leu Pro
```

<210> 77
<211> 3567
<212> DNA
<213> Homo Sapien

<400> 77

```
caccaacaag caatcgttca tgagaaagcc gtgcacccgc tgcagttggg 50

ccatgtggtc cgcatcgtat tccactaggt ccccattgta caccaagtac 100

tgtcccggcg tctccagcag atgcctgcag ccttccacct tctcaagcag 150

ggtggtgtga gtgcgctgct ttccttcctc gcctggaccg gagccgtcgc 200

gggaggcacc cccgggggtg gagaaaaagc cggcctggcc tcggaggtgg 250

tctcggcccc ccgccccacc gactccctcc tcccctccag aggcggcggc 300

ggctccggcg gcagcagcgg caggcagcaa cgtaagcggg atgctctcca 350

ggctgctttt ctgctcggtc agcaaatggc tgagctggta catctcgctc 400

tccaggtagg agatctcgcg ggccgtctct atgaactgcc ggtagttctg 450

gtagacgttg cgcttcaggt tctgcgccgt ctcctccgcc agcgcctgga 500

tgcgctgccg gtgctcctgg aggtcccggt ccccatccga ctgctgcgag 550
```

agctgcttca cgtacagccg cgcctcaaaa ccccctgact ccagctgccg 600
acgcaggcgg ctcgccccac tgtccgacat cgccatcgcc atttctctcc 650
gggtctcacg cactcactgt cactatcggc gccgcagccg ccgcggctgt 700
ctagacccac ccaaggccaa ccgagctcct gggctgagga agcaggaatg 750
ggaacgagac gagtacgcct gcgccgggtc tgagcgtcag acactgcgcc 800
tgcgcaagtg ggccgagcgc agacattgcg cctgcgcagc aatgccatcg 850
gttaaagcgc atgcgcaaga tgagctattg cggaagtgag gggagggaga 900
ggccgagaga aatttcggta ctgcgcatga accgagcgtg acgttgaggt 950
ttgaaataac cggcaaagag taaaggctga aactagcttc ctgaaagctt 1000
cgtagggccc gagccctgtg agcccaggtt ctgcgcccac taggaggtgt 1050
catgctgact gcttttttta aagccctaga atccttggct tcggcgtttg 1100
gggtaagctc cgttctcgtt ctcaagcgcg tttccgcgaa ctctcgcggg 1150
attgacgggc cgtctcgaga gccggcatct cctaggagct agtcctggtc 1200
ctcggctagg cggcttgggg tcgcggcgta actggggagc cagcctgacg 1250
ccggcggacc ccgcctgtga tcctggcaac gatggatgat gacttgatgt 1300
tggcactgcg gcttcaggag gagtggaact tgcaggaggc ggagcgcgat 1350
catgcccagg agtccctgtc gctagtggac gcgtcgtggg agttggtgga 1400
ccccacaccg gacttgcagg cactgtttgt tcagtttaac gaccaattct 1450
tctggggcca gctggaggcc gtcgaggtga agtggagcgt gcgaatgacc 1500
ctgtgtgctg ggatatgcag ctatgaaggg aagggtggaa tgtgttccat 1550
ccgtctcagc gaacccctttt tgaagttgag gccaagaaag gatcttgtag 1600
agaccctcct gcatgaaatg atacatgcct atttatttgt cactaataac 1650
gacaaagacc gagaagggca tggtccagaa ttttgtaaac atatgcatcg 1700
catcaacagc ctgactggag ccaatataac ggtataccat acttttcacg 1750
atgaggtgga tgagtatcgg cgacactggt ggcgctgcaa tgggccgtgc 1800
cagcacaggc caccgtatta cggctatgtc aaacgagcta ctaacaggga 1850
accctctgct catgactatt ggtgggctga gcaccagaaa acctgtggag 1900
gcacttacat aaaaatcaag gaaccagaga attactcaaa aaaaggcaaa 1950
ggaaaggcaa aactaggaaa ggaaccagta ttggccgcag agaataaagg 2000
taccttcgtg tatattcttc tgatttttat gtgaccatag ctatgatgta 2050
aagacaatac tgtccttcag agaactggta ttaagataaa cttaaggatc 2100
gtttctggtg tagaagtctt caagtgtaga cttaaggaaa aaatcccact 2150

gtccatgaaa tgatggtagg aaaacagact ttgctctgta cagaagtaag 2200

taaaagtagg aatagtttcc atggatattt ttatttttat taactttttt 2250

cagtttcttt ttattcaaag aaacaaaatt caatctctga taatatttga 2300

ggtaaagttc ctttccctat cttgactcac tgagttatta ggaaacagaa 2350

ggcaaaaaga ttgtcaaaat aaaaacaata attcaagtaa caatgcccgg 2400

aatatacgtc ctaactacac cccttcctat cagctggatt ctatccaagt 2450

gactctattg atgtatgtat gttcattcaa agaatgggaa aaggatatga 2500

catatatttg ccagtacttc atcttcaaga tttacccttt tcctgtgaag 2550

ttcagagtta ctgaagatgc ttcttccctt gggaagttgt tgacccaaga 2600

acataggtta tatttcccaa atctttaatt attgagtgaa agagctatag 2650

atgaattgat atggaaagac cgtatcttca ttttcgtgag tagaaggaaa 2700

gataagaatg aggcagcaga ttttccctcc tggaattaca cataaaggac 2750

actaagcaat tttcaaggta aatgttgcct tgttgttggt ctttggcatg 2800

ataagattct ttatttaaat atgagagaat ttttttttat cctttatatt 2850

ctctcaatat cagaactcct gaattctgaa gattgccctc ctcccattaa 2900

taggattgta tggatgtaag atggaataaa atactagttc ttcattttga 2950

gaaaactgta cattagttta atgtttgtta ctgtatttct tttgagttga 3000

ggcacttaca taacaatctt ctttgctttt ttggcagata aacccaacag 3050

aggtgaggcc cagctagtaa tccctttttag tgggaaagga tatgttctag 3100

gagaaacaag caatttacct tcacctggga aactgatcac ttcacatgcc 3150

attaataaaa cccaagatct tttaaatcaa aaccattcag caaatgctgt 3200

aagacctaat tctaaaatca aggtgaaatt tgaacagaat ggttcaagta 3250

aaaattctca tctggtctcc cctgctgtta gtaacagtca ccaaaatgtt 3300

ctaagcaact actttcctag agtatcattt gccaaccaaa aggctttcag 3350

aggtgtgaat ggatctccaa ggataagtgt aacagttggc aacatcccta 3400

aaaactcagt ctcttctagt tctcagagaa gggtttcatc ttctaagata 3450

tccctaagaa attcttcaaa agtaacggaa tcagcatctg tgatgccatc 3500

ccaggatgtg agtgggtctg aagatacatt cccaaataaa cgacctaggc 3550

tagaagataa aaaaaaa 3567

<210> 78
<211> 250
<212> PRT
<213> Homo Sapien

<400> 78
Met Asp Asp Asp Leu Met Leu Ala Leu Arg Leu Gln Glu Glu Trp
1               5                   10                  15

Asn Leu Gln Glu Ala Glu Arg Asp His Ala Gln Glu Ser Leu Ser
                20                  25                  30

Leu Val Asp Ala Ser Trp Glu Leu Val Asp Pro Thr Pro Asp Leu
                35                  40                  45

Gln Ala Leu Phe Val Gln Phe Asn Asp Gln Phe Phe Trp Gly Gln
                50                  55                  60

Leu Glu Ala Val Glu Val Lys Trp Ser Val Arg Met Thr Leu Cys
                65                  70                  75

Ala Gly Ile Cys Ser Tyr Glu Gly Lys Gly Gly Met Cys Ser Ile
                80                  85                  90

Arg Leu Ser Glu Pro Leu Leu Lys Leu Arg Pro Arg Lys Asp Leu
                95                  100                 105

Val Glu Thr Leu Leu His Glu Met Ile His Ala Tyr Leu Phe Val
                110                 115                 120

Thr Asn Asn Asp Lys Asp Arg Glu Gly His Gly Pro Glu Phe Cys
                125                 130                 135

Lys His Met His Arg Ile Asn Ser Leu Thr Gly Ala Asn Ile Thr
                140                 145                 150

Val Tyr His Thr Phe His Asp Glu Val Asp Glu Tyr Arg Arg His
                155                 160                 165

Trp Trp Arg Cys Asn Gly Pro Cys Gln His Arg Pro Pro Tyr Tyr
                170                 175                 180

Gly Tyr Val Lys Arg Ala Thr Asn Arg Glu Pro Ser Ala His Asp
                185                 190                 195

Tyr Trp Trp Ala Glu His Gln Lys Thr Cys Gly Gly Thr Tyr Ile
                200                 205                 210

Lys Ile Lys Glu Pro Glu Asn Tyr Ser Lys Lys Gly Lys Gly Lys
                215                 220                 225

Ala Lys Leu Gly Lys Glu Pro Val Leu Ala Ala Glu Asn Lys Gly
                230                 235                 240

Thr Phe Val Tyr Ile Leu Leu Ile Phe Met
                245                 250

<210> 79
<211> 2714
<212> DNA
<213> Homo Sapien

<400> 79
cggacgcgtg ggtggcaacc aggagaagcc aaacttggtc ccccggctcg 50

cggagtgcct gcgagcggtg ctcatggcgc tctatgaggt cttctctcac 100

ccggtcgagc gcagttaccg cgcggggctc tgctccaaag ccgcgctgtt 150

cctgctgctg gccgctgcgc tcacgtacat cccgccgctg ctggtggcct 200
tccggagcca cgggttttgg ctgaagcgga gcagctacga ggagcagccg 250
accgtgcgct tccaacacca ggtgctgctc gtggccctgc tcggacccga 300
aagcgacggg ttcctcgcct ggagcacgtt ccccgccttc aaccggctgc 350
aaggggatcg cctgcgcgtc ccgctcgttt cgactagaga agaagacagg 400
aaccaggatg ggaagacgga catgttacat tttaagctgg agcttcccct 450
gcagtccacg gagcacgttc tcggtgtgca gctcatcctg actttctcct 500
atcgattaca caggatggcg accctcgtga tgcagagcat ggcgtttctc 550
cagtcctcct ttcctgtccc gggatcccag ttatacgtga acggagacct 600
gaggctgcag cagaagcagc cgctgagctg tggtggccta gatgcccgat 650
acaacatatc cgtgatcaac gggaccagcc cctttgccta tgactacgac 700
ctcacccata ttgttgctgc ctaccaggag aggaacgtta ccaccgtcct 750
gaatgatccc aaccccatct ggctggtggg cagggccgca gatgctccat 800
ttgtgattaa tgctatcatc cgataccctg tggaagtcat ttcttatcag 850
ccaggattct gggagatggt aaagttcgcc tgggtacagt atgtcagcat 900
cctgcttatc ttcctctggg tgtttgaaag aatcaagatc ttcgtgtttc 950
agaatcaggt ggtgaccacc attcctgtga cagtgacgcc ccggggagac 1000
ttgtgtaagg agcacttatc ctagaaaggc catttctgaa gactcagcag 1050
gaccgtggct gcctcattgt catcttctgg gaacatctta ggaccttttg 1100
aaagagccca gcggacacct gcgggcttgt gtgcttttcc ctcagagaca 1150
acggttcttt ccggttttgc tctacacagt tccgtatctt cagagctcct 1200
gcagaattgt cagggactag tttgtggaaa ggtctgagag ttcctggagg 1250
ctataattag ctttttgggt tttccttctt tgccttagcg ttgaatttca 1300
ggagaaaatt gcagtcagtt cagacatctt ggaaagagtc ccatctctgg 1350
tcaagcagag acttttcctc tgttgaactg aggaacacac tgtgcatttc 1400
ttccttctgt tgtgagccac tcttactctt ttcagggctc tcttgtgaca 1450
aacatgccaa tcactagcac tttgcacccc tgggcttctc catttcccat 1500
tcacagcttt gatttccaga gctgaggcct ttaactggag acctggaggg 1550
gcagggccca agggcaaggg ccgcattagc acaggcaatc agggagggcc 1600
gctgaaggac acttggaccg tccacctgcc ccagcccaac agtcagtcat 1650
ctgtcatcag ctcagctgag cagccctgga tctttgccgt actgtgactg 1700
ggctctttgc cctatttttc cctctgtctg tgcccctgga tggcaggctg 1750

aagtcagagg ggctgtttca ttctcagccc cctcagcagc actgggggaa 1800

gaaagcattg tcacaacagg ttctttctgg ccctcaccca acagcctggg 1850

cacttggccc tcctcctcct tgacagccct ccccccttcct gcaaaggaca 1900

ggggcgacag gggttggtgt tgggattggc tcccgctgcc tgacaaccac 1950

aagtttattt ggaaggctag cgggaagccc agcggctggc gtttcccttg 2000

actaaggaac agggtgccca tcagagtggg gcgggcagct ttgggaagga 2050

cacaagaagc agtaagagtg taaagaggat gctggcctgg gcaggccagt 2100

ccagcctggc cactagcaga ataccaagca gtccagtgga ttaccctcgt 2150

ggctaagcaa gtgtctgcag gagcagagat ggctggaagg ggcctctgca 2200

cacggaagat ggcttgttca gcccattcac ctcctgagga tgtgggcagt 2250

ctcctccaag aacacatgga gctgcttcct gatcccaagc aggtcattgc 2300

cactggaagg acatggcccc ggtgatccat gcttcatgcc cacccagaaa 2350

cacacccctc agtgtgtgcc tcagtttact ttggagatca gttgtcgttt 2400

ttagtgctcc tttaggctta ctaaaacagt tttggaaaca aagctatttt 2450

gaagtattca agcagaggaa ttccctaaca ctgacccccct tgtctttttt 2500

taatattcag gctgttttat atgcctaaat ttttttctta agatctaaac 2550

gaaaaatagt ttcttgttta aattcacata aggcaatgag atatggaaag 2600

atgacaagat acgtataaac attggtttgc atcttattaa attattctaa 2650

tgcaaatctt gtataaagaa cccatgatgt tttgtaactt tctaattaaa 2700

atgttcaaaa tgag 2714

<210> 80
<211> 316
<212> PRT
<213> Homo Sapien

<400> 80
Met Ala Leu Tyr Glu Val Phe Ser His Pro Val Glu Arg Ser Tyr
1               5                   10                  15

Arg Ala Gly Leu Cys Ser Lys Ala Ala Leu Phe Leu Leu Leu Ala
                20                  25                  30

Ala Ala Leu Thr Tyr Ile Pro Pro Leu Leu Val Ala Phe Arg Ser
                35                  40                  45

His Gly Phe Trp Leu Lys Arg Ser Ser Tyr Glu Glu Gln Pro Thr
                50                  55                  60

Val Arg Phe Gln His Gln Val Leu Leu Val Ala Leu Leu Gly Pro
                65                  70                  75

Glu Ser Asp Gly Phe Leu Ala Trp Ser Thr Phe Pro Ala Phe Asn
                80                  85                  90

```
Arg Leu Gln Gly Asp Arg Leu Arg Val Pro Leu Val Ser Thr Arg
                95                  100                 105

Glu Glu Asp Arg Asn Gln Asp Gly Lys Thr Asp Met Leu His Phe
                110                 115                 120

Lys Leu Glu Leu Pro Leu Gln Ser Thr Glu His Val Leu Gly Val
                125                 130                 135

Gln Leu Ile Leu Thr Phe Ser Tyr Arg Leu His Arg Met Ala Thr
                140                 145                 150

Leu Val Met Gln Ser Met Ala Phe Leu Gln Ser Ser Phe Pro Val
                155                 160                 165

Pro Gly Ser Gln Leu Tyr Val Asn Gly Asp Leu Arg Leu Gln Gln
                170                 175                 180

Lys Gln Pro Leu Ser Cys Gly Gly Leu Asp Ala Arg Tyr Asn Ile
                185                 190                 195

Ser Val Ile Asn Gly Thr Ser Pro Phe Ala Tyr Asp Tyr Asp Leu
                200                 205                 210

Thr His Ile Val Ala Ala Tyr Gln Glu Arg Asn Val Thr Thr Val
                215                 220                 225

Leu Asn Asp Pro Asn Pro Ile Trp Leu Val Gly Arg Ala Ala Asp
                230                 235                 240

Ala Pro Phe Val Ile Asn Ala Ile Ile Arg Tyr Pro Val Glu Val
                245                 250                 255

Ile Ser Tyr Gln Pro Gly Phe Trp Glu Met Val Lys Phe Ala Trp
                260                 265                 270

Val Gln Tyr Val Ser Ile Leu Leu Ile Phe Leu Trp Val Phe Glu
                275                 280                 285

Arg Ile Lys Ile Phe Val Phe Gln Asn Gln Val Val Thr Thr Ile
                290                 295                 300

Pro Val Thr Val Thr Pro Arg Gly Asp Leu Cys Lys Glu His Leu
                305                 310                 315

Ser
```

<210> 81
<211> 3233
<212> DNA
<213> Homo Sapien

<400> 81

```
gccgggagct tccctgatgg tgccgccgcc tccgagccgg ggaggagctg 50

ccaggggcca gctgggcagg agcctgggtc cgctgctgct gctcctggcg 100

ttgggacaca cgtggaccta cagagaggag ccggaggacg gcgacagaga 150

aatctgctca gagagcaaaa tcgcgacgac taaatacccg tgtctgaagt 200

cttcaggcga gctcaccaca tgctacagga aaaagtgctg caaaggatat 250
```

aaatttgttc ttggacaatg catcccagaa gattacgacg tttgtgccga 300
ggctccctgt gaacagcagt gcacggacaa ctttggccga gtgctgtgta 350
cttgttatcc gggataccga tatgaccggg agagacaccg gaagcgggag 400
aagccatact gtctggatat tgatgagtgt gccagcagca atgggacgct 450
gtgtgcccac atctgcatca ataccttggg cagctaccgc tgcgagtgcc 500
gggaaggcta catccgggaa gatgatggga agacatgtac caggggagac 550
aaatatccca atgacactgg ccatgagaag tctgagaaca tggtgaaagc 600
cggaacttgc tgtgccacat gcaaggagtt ctaccagatg aagcagaccg 650
tgctgcagct gaagcaaaag attgctctgc tccccaacaa tgcagctgac 700
ctgggcaagt atatcactgg tgacaaggtg ctggcctcaa acacctacct 750
tccaggacct cctggcctgc ctgggggcca gggccctccc ggctcaccag 800
gaccaaaggg aagcccaggc ttccccggta tgccaggccc tcctgggcag 850
cccggcccac ggggctcaat gggacccatg ggaccatctc ctgatctgtc 900
ccacattaag caaggccgga ggggccctgt gggtccacca ggggcaccag 950
gaagagatgg ttctaagggg gagagaggag cgcctgggcc cagagggtct 1000
ccaggacccc ctggttcttt cgacttcctg ctacttatgc tggctgacat 1050
ccgcaatgac atcactgagc tgcaggaaaa ggtgttcggg caccggactc 1100
actcttcagc agaggagttc cctttacctc aggaatttcc cagctaccca 1150
gaagccatgg acctgggctc tggagatgac catccaagaa gaactgagac 1200
aagagacttg agagccccca gagacttcta cccatagcac atcccaacac 1250
cgtcacgcca aaggaagaga aagatcaact cacctgcagt taaaccatct 1300
aaagagaaga aagaccactg gagacctaga aaacatacat ttttctcttc 1350
tcttctcctg acgtctctcc actcctcttc ttccaaatac gatgctattt 1400
tcagagtccc ctcctaggcc tgcagacatg agggagtgaa tgattgattt 1450
acctgcttct cactaagagt ccattggggt ggtttgcatt gtaacttttc 1500
ttttacatcc tatttttcca ggaactttgg atttaagtac tctcacagtg 1550
tcttaaatca taaattcttg aagttaaatt tggcagagta tcaaaagggg 1600
gaaaatgaca aagtgagctc taagaaaatg tgaggctact tctaagatgt 1650
gtgttcacaa tagaccataa ctcctctagt atcaaaattg gggctcttca 1700
gttaaaaagg ggtggggagg acaaacgtgt cgatgtgctt tggtggagaa 1750
ttttttcctt gtgcttctag tagactttaa atattgtatc cctttgtcaa 1800
accttgtttc ccaaattcaa ttaaagagag gagagaattg aatggcgttt 1850

agagaagata gaaaagaatc acagtcatat atttactgtt atatagattg 1900

ccacattcta aaattcaaat acggtgctta aggtttcatg ccatgcttat 1950

ctgtaagtat cctatttagg gaagaagatt aaactctctt ttcaaaaaaa 2000

caaagtgaaa tgcctggatt cacattaaaa caatgggctc tcgtttgcta 2050

taatatttta aagctgttta atcaacagtg gagtctgctc tataaatata 2100

gattatttgt tcaataaact ggctgagctt agagagaggt gcagaattcc 2150

tggttctgag caggtgccca gaaggtacca ttaggtgcca tgatccaggc 2200

tgaaccaata tacagtgggg ctgaagtctg caaggaggtt gctggcttgg 2250

gctgacctca ctaatgccat cagcagcggt aggtaaattt tttctccttg 2300

ggtattacaa gtttttgtct ggagccaacc aagcttgcca ccaacatatt 2350

gagagtaata cactattgaa agttatcttg gatggggaga aaaaaaaata 2400

gtggttttcc ttgtttgcaa aaacttcctt cctattctca tttttttcta 2450

attttcttta atttagtcca agttccagtt cttttaggcc ttctctttga 2500

tttattttcc cctgcatgtg agaagcagtt cagaaaaagg tctatatctc 2550

cacctcctag tgagttagag tgttttctca gagcacctct gggtggcaaa 2600

gggaagcatg ttcctgccaa ggtttgctgt ggattcagaa gcaccaggag 2650

caagagacca gaaggatgat ctgctccttt gtaacgttgt tgagggccct 2700

cttgtttcca atgagcagct tataggttac tcacagtcca ctttctcact 2750

ggacacacaa agtggctctt tatctacctt tgcgggagat tttcactctc 2800

ctgcaaatga tcgttctcac actcatatta gctcatgttg gaatttccca 2850

tcctgccatg tcctttccca tttctttttg gctttttttgc ctccaccttt 2900

tagcccacat catttaactc cactactgtg aaagcttgct taaagaaaat 2950

ccctcttggc cgggtgtggt agcccacgcc tctaatccca gcactttggg 3000

aggctgaggc ggggagatca caaggtcagg agatcgagac cagcctgacc 3050

aacatggtga aaccctgtct ctactaaaaa tacaaaaatt agctgggcgt 3100

gttggcacac acctgtaatc ccagctactc aggaggctga ggcaggagaa 3150

ttactttaac ctgcgggggg agcctagatt gcgctactgc actccagcct 3200

aggcaacaga gggagactct gtctcattaa aaa 3233

<210> 82
<211> 406
<212> PRT
<213> Homo Sapien

<400> 82
Met Val Pro Pro Pro Pro Ser Arg Gly Gly Ala Ala Arg Gly Gln
1               5                   10                  15

```
Leu Gly Arg Ser Leu Gly Pro Leu Leu Leu Leu Leu Ala Leu Gly
                20                  25                  30

His Thr Trp Thr Tyr Arg Glu Glu Pro Glu Asp Gly Asp Arg Glu
                35                  40                  45

Ile Cys Ser Glu Ser Lys Ile Ala Thr Thr Lys Tyr Pro Cys Leu
                50                  55                  60

Lys Ser Ser Gly Glu Leu Thr Thr Cys Tyr Arg Lys Lys Cys Cys
                65                  70                  75

Lys Gly Tyr Lys Phe Val Leu Gly Gln Cys Ile Pro Glu Asp Tyr
                80                  85                  90

Asp Val Cys Ala Glu Ala Pro Cys Glu Gln Gln Cys Thr Asp Asn
                95                  100                 105

Phe Gly Arg Val Leu Cys Thr Cys Tyr Pro Gly Tyr Arg Tyr Asp
                110                 115                 120

Arg Glu Arg His Arg Lys Arg Glu Lys Pro Tyr Cys Leu Asp Ile
                125                 130                 135

Asp Glu Cys Ala Ser Ser Asn Gly Thr Leu Cys Ala His Ile Cys
                140                 145                 150

Ile Asn Thr Leu Gly Ser Tyr Arg Cys Glu Cys Arg Glu Gly Tyr
                155                 160                 165

Ile Arg Glu Asp Asp Gly Lys Thr Cys Thr Arg Gly Asp Lys Tyr
                170                 175                 180

Pro Asn Asp Thr Gly His Glu Lys Ser Glu Asn Met Val Lys Ala
                185                 190                 195

Gly Thr Cys Cys Ala Thr Cys Lys Glu Phe Tyr Gln Met Lys Gln
                200                 205                 210

Thr Val Leu Gln Leu Lys Gln Lys Ile Ala Leu Leu Pro Asn Asn
                215                 220                 225

Ala Ala Asp Leu Gly Lys Tyr Ile Thr Gly Asp Lys Val Leu Ala
                230                 235                 240

Ser Asn Thr Tyr Leu Pro Gly Pro Pro Gly Leu Pro Gly Gly Gln
                245                 250                 255

Gly Pro Pro Gly Ser Pro Gly Pro Lys Gly Ser Pro Gly Phe Pro
                260                 265                 270

Gly Met Pro Gly Pro Pro Gly Gln Pro Gly Pro Arg Gly Ser Met
                275                 280                 285

Gly Pro Met Gly Pro Ser Pro Asp Leu Ser His Ile Lys Gln Gly
                290                 295                 300

Arg Arg Gly Pro Val Gly Pro Pro Gly Ala Pro Gly Arg Asp Gly
                305                 310                 315

Ser Lys Gly Glu Arg Gly Ala Pro Gly Pro Arg Gly Ser Pro Gly
                320                 325                 330
```

```
Pro Pro Gly Ser Phe Asp Phe Leu Leu Leu Met Leu Ala Asp Ile
                335                 340                 345

Arg Asn Asp Ile Thr Glu Leu Gln Glu Lys Val Phe Gly His Arg
                350                 355                 360

Thr His Ser Ser Ala Glu Glu Phe Pro Leu Pro Gln Glu Phe Pro
                365                 370                 375

Ser Tyr Pro Glu Ala Met Asp Leu Gly Ser Gly Asp Asp His Pro
                380                 385                 390

Arg Arg Thr Glu Thr Arg Asp Leu Arg Ala Pro Arg Asp Phe Tyr
                395                 400                 405

Pro


<210> 83
<211> 443
<212> DNA
<213> Homo Sapien

<400> 83
atctgagtga gctaactgac acaatgaaac tgtcaggcat gtttctgctc 50

ctctctctgg ctcttttctg ctttttaaca ggtgtcttca gtcagggagg 100

acaggttgac tgtggtgagt tccaggaccc caaggtctac tgcactcggg 150

aatctaaccc acactgtggc tctgatggcc agacatatgg caataaatgt 200

gccttctgta aggccatagt gaaaagtggt ggaaagatta gcctaaagca 250

tcctggaaaa tgctgagtta aagccaatgt ttcttggtga cttgccagct 300

tttgcagcct tcttttctca cttctgctta tacttttgct ggtggattcc 350

tttaattcat aaagacatac ctactctgcc tgggtcttga ggagttcaat 400

gtatgtctat ttctcttgat tcacttgtca ataaagtaca ttc 443

<210> 84
<211> 80
<212> PRT
<213> Homo Sapien

<400> 84
Met Lys Leu Ser Gly Met Phe Leu Leu Leu Ser Leu Ala Leu Phe
  1               5                  10                  15

Cys Phe Leu Thr Gly Val Phe Ser Gln Gly Gly Gln Val Asp Cys
                 20                  25                  30

Gly Glu Phe Gln Asp Pro Lys Val Tyr Cys Thr Arg Glu Ser Asn
                 35                  40                  45

Pro His Cys Gly Ser Asp Gly Gln Thr Tyr Gly Asn Lys Cys Ala
                 50                  55                  60

Phe Cys Lys Ala Ile Val Lys Ser Gly Gly Lys Ile Ser Leu Lys
                 65                  70                  75

His Pro Gly Lys Cys
```

80

<210> 85
<211> 2750
<212> DNA
<213> Homo Sapien

<400> 85
ggagcagaca cacagacccg ggccggaggc ccctcttcta gccctgcggg 50

aaccggacag ttccccaact ggggactctg gaaccacagc tcctaaatca 100

tcaaattctc aagctttttt tttccctctc ttcgtcccag ccatcccagt 150

cttcttcttc tttttttttt ttttaactta ttgttttttt cgctcctgtc 200

attatgaaag tggtcacgcc attcaatatt aagacttgga gggaattggg 250

gaaagaaaag aaagaatcta aaagaagaga agcgaccggt gcttttaagg 300

gtgtctaatt ttcaaaagag acgtctggga gtattttgct ctgggcgttt 350

ggagcaactt cgcggacagc ggagctcgcc cagcatggat gttccaggtt 400

cacaggcgcc tttcttctga gaacgaccct ggccttgaac gtcagagccg 450

gggacgaagg cccccggagg ctgctgcgag ctccgcgcgt tccttcgcgc 500

ccttccgcgc cgctcgcgcc ggcgccggcc tccacccccg cgcgccgcct 550

cccaccagtc ccgatgcagg cgcccggccg gggccactc gggctgcggc 600

tgatgatgcc cgggcgccgg ggggcgctgc gcgagcctgg cggctgcgga 650

tcctgcctgg gggtggcgct ggccctgctg ttgctgctac tgcccgcctg 700

ctgccccgtg cgggcgcaga acgacacgga gcccatcgtg ctggagggca 750

agtgcctggt ggtgtgcgac tccagcccgt cggcggacgg cgccgtcacc 800

tcctccctag gcatctccgt gcgctccggc agcgccaagg tggccttctc 850

cgccacgcgg agcaccaacc acgagccgtc cgagatgagc aaccgcacca 900

tgaccatcta tttcgaccag gtattagtaa atattggcaa ccactttgat 950

cttgcttcca gtatatttgt agcaccgaga aaagggattt atagcttcag 1000

cttccacgtg gtcaaagtgt ataacagaca aaccatccag gtcagtttaa 1050

tgcagaatgg ctacccagtg atctcggcct ttgcaggaga ccaggatgtc 1100

accagagaag ctgctagcaa tggcgtgctg ctgctcatgg aaagggaaga 1150

caaagtgcat ctcaaacttg agagaggcaa cctcatgggg ggctggaaat 1200

actccacatt ctcgggcttc ttggtgtttc ctctataaac acagagcccc 1250

ctagatggtg ggggaatggc aaactggacc caggactccg ccctttaaaa 1300

caccctgaac ttactggaat tggacacctt gtttccaacc tccgtcagac 1350

tgttgcagta gaagaatgat ttcctttgaa acctccagta cttttgtttt 1400

tgttttttgg aatactgaca attcctcggg aacctggcct ctaattagtt 1450

ttagatgaca aggtcttaag gagaaatgaa attatcgatt tgagcaattt 1500

gtacctgtga ttgtaaagtc aatatcggat tttattgttg ggaccatgga 1550

cctcttttgt ttgtatgttg tattgtcgtc ccaacggaag gagagctcct 1600

gactccagga tgggctgcag gttgcagtca gggcttgaag taggagccca 1650

gcaaagaacc acctgctgga cagtccttga catgtgttct gtgtgtgtct 1700

gtatagcctt aagaaaaaga atggcttcac tttcattctg tattcttccc 1750

cccaccatgt ggctgggagg acttgggagg gggatgggga cattgggaac 1800

ctgtcaagaa gtgctttatc cagagaagca aattttgcac gattggactg 1850

caatttttgt tttgtattgt ttgtgttttt tcttgaaaag ctttactttt 1900

ctttccacac tcagctctcc ctcctcaacc ccacttttat ttttcttgct 1950

ggggttgagg agagaaaata tagaattcct ggataagacc aaacaaaaca 2000

aaacattaaa atacctgtat gttttgtttt agacgagacc aaactaaaca 2050

aaaagtatct gtttatcaaa gtaaaagtaa cacaatggac aattctgctt 2100

attctctcaa agagattcta agatgcacct ttagaactat taatagcaac 2150

ctgcattttt ttttaattta tacttcagaa tcctttaaga acctggtgtt 2200

cctgagtggt cctgaatcat ataagttggt aatggaagct gtaatgacca 2250

agtcccctaa acatactatg tctttgccac gtgtgctgtg acttctctgt 2300

gggtgattta atttatttgg atccacctct gagtgagcgc acagtgatca 2350

ggtgcttcaa agccaacaga ccagctcctc ttcctccgga tcctcttttg 2400

atctgcccag gaaagggatg cattgacact ctcctgcatg cacctggcga 2450

gaagccacct gaaagtcact gtggttaaag atattggtgg aggtacccca 2500

ggagcactgt tacaaatcct tcttgttttg gcatctcgta caacattatt 2550

aagacacagc tgagagttga tgggtgtgta atgcatatgc caaggaaatg 2600

tcactaatcc caaagcaatc aaaaaggaga cctcaaacca gatgttaatt 2650

tgttctttgt gtaacaatgt aaccaaaata ttgatgataa aagtcataat 2700

ttaagattca gaataaatgg gtttgatgtc tggcaaaaaa aaaaaaaaaa 2750

<210> 86
<211> 224
<212> PRT
<213> Homo Sapien

<400> 86
Met Gln Ala Pro Gly Arg Gly Pro Leu Gly Leu Arg Leu Met Met
1 5 10 15

Pro Gly Arg Arg Gly Ala Leu Arg Glu Pro Gly Gly Cys Gly Ser

20 25 30

Cys Leu Gly Val Ala Leu Ala Leu Leu Leu Leu Leu Leu Pro Ala
35 40 45

Cys Cys Pro Val Arg Ala Gln Asn Asp Thr Glu Pro Ile Val Leu
50 55 60

Glu Gly Lys Cys Leu Val Val Cys Asp Ser Ser Pro Ser Ala Asp
65 70 75

Gly Ala Val Thr Ser Ser Leu Gly Ile Ser Val Arg Ser Gly Ser
80 85 90

Ala Lys Val Ala Phe Ser Ala Thr Arg Ser Thr Asn His Glu Pro
95 100 105

Ser Glu Met Ser Asn Arg Thr Met Thr Ile Tyr Phe Asp Gln Val
110 115 120

Leu Val Asn Ile Gly Asn His Phe Asp Leu Ala Ser Ser Ile Phe
125 130 135

Val Ala Pro Arg Lys Gly Ile Tyr Ser Phe Ser Phe His Val Val
140 145 150

Lys Val Tyr Asn Arg Gln Thr Ile Gln Val Ser Leu Met Gln Asn
155 160 165

Gly Tyr Pro Val Ile Ser Ala Phe Ala Gly Asp Gln Asp Val Thr
170 175 180

Arg Glu Ala Ala Ser Asn Gly Val Leu Leu Leu Met Glu Arg Glu
185 190 195

Asp Lys Val His Leu Lys Leu Glu Arg Gly Asn Leu Met Gly Gly
200 205 210

Trp Lys Tyr Ser Thr Phe Ser Gly Phe Leu Val Phe Pro Leu
215 220

<210> 87
<211> 2159
<212> DNA
<213> Homo Sapien

<400> 87
agggcccgcg ggtggagaga gcgacgcccg aggggatggc ggcagcgtcc 50

cggagcgcct ctggctgggc gctactgctg ctggtggcac tttggcagca 100

gcgcgcggcc ggctccggcg tcttccagct gcagctgcag gagttcatca 150

acgagcgcgg cgtactggcc agtgggcggc cttgcgagcc cggctgccgg 200

actttcttcc gcgtctgcct taagcacttc caggcggtcg tctcgcccgg 250

accctgcacc ttcgggaccg tctccacgcc ggtattgggc accaactcct 300

tcgctgtccg ggacgacagt agcggcgggg ggcgcaaccc tctccaactg 350

cccttcaatt tcacctggcc gggtaccttc tcgctcatca tcgaagcttg 400

gcacgcgcca ggagacgacc tgcggccaga ggccttgcca ccagatgcac 450

tcatcagcaa gatcgccatc cagggctccc tagctgtggg tcagaactgg 500
ttattggatg agcaaaccag caccctcaca aggctgcgct actcttaccg 550
ggtcatctgc agtgacaact actatggaga caactgctcc cgcctgtgca 600
agaagcgcaa tgaccacttc ggccactatg tgtgccagcc agatggcaac 650
ttgtcctgcc tgcccggttg gactggggaa tattgccaac agcctatctg 700
tctttcgggc tgtcatgaac agaatggcta ctgcagcaag ccagcagagt 750
gcctctgccg cccaggctgg cagggccggc tgtgtaacga atgcatcccc 800
cacaatggct gtcgccacgg cacctgcagc actccctggc aatgtacttg 850
tgatgagggc tggggaggcc tgttttgtga ccaagatctc aactactgca 900
cccaccactc cccatgcaag aatggggcaa cgtgctccaa cagtgggcag 950
cgaagctaca cctgcacctg tcgcccaggc tacactggtg tggactgtga 1000
gctggagctc agcgagtgtg acagcaaccc ctgtcgcaat ggaggcagct 1050
gtaaggacca ggaggatggc taccactgcc tgtgtcctcc gggctactat 1100
ggcctgcact gtgaacacag caccttgagc tgcgccgact ccccctgctt 1150
caatgggggc tcctgccggg agcgcaacca gggggccaac tatgcttgtg 1200
aatgtccccc caacttcacc ggctccaact gcgagaagaa agtggacagg 1250
tgcaccagca acccctgtgc caacggggga cagtgcctga accgaggtcc 1300
aagccgcatg tgccgctgcc gtcctggatt cacgggcacc tactgtgaac 1350
tccacgtcag cgactgtgcc cgtaaccctt gcgcccacgg tggcacttgc 1400
catgacctgg agaatgggct catgtgcacc tgccctgccg gcttctctgg 1450
ccgacgctgt gaggtgcgga catccatcga tgcctgtgcc tcgagtccct 1500
gcttcaacag ggccacctgc tacaccgacc tctccacaga cacctttgtg 1550
tgcaactgcc cttatggctt tgtgggcagc cgctgcgagt tccccgtggg 1600
cttgccgccc agcttcccct gggtggccgt ctcgctgggt gtggggctgg 1650
cagtgctgct ggtactgctg ggcatggtgg cagtggctgt gcggcagctg 1700
cggcttcgac ggccggacga cggcagcagg gaagccatga acaacttgtc 1750
ggacttccag aaggacaacc tgattcctgc cgcccagctt aaaaacacaa 1800
accagaagaa ggagctggaa gtggactgtg gcctggacaa gtccaactgt 1850
ggcaaacagc aaaaccacac attggactat aatctggccc cagggcccct 1900
ggggcggggg accatgccag gaaagtttcc ccacagtgac aagagcttag 1950
gagagaaggc gccactgcgg ttacacagtg aaaagccaga gtgtcggata 2000
tcagcgatat gctcccccag ggactccatg taccagtctg tgtgtttgat 2050

atcagaggag aggaatgaat gtgtcattgc cacggaggta taaggcagga 2100

gcctacctgg acatccctgc tcagccccgc ggctggacct tccttctgca 2150

ttgtttaca 2159

<210> 88
<211> 685
<212> PRT
<213> Homo Sapien

<400> 88
Met Ala Ala Ala Ser Arg Ser Ala Ser Gly Trp Ala Leu Leu Leu
1 5 10 15

Leu Val Ala Leu Trp Gln Gln Arg Ala Ala Gly Ser Gly Val Phe
20 25 30

Gln Leu Gln Leu Gln Glu Phe Ile Asn Glu Arg Gly Val Leu Ala
35 40 45

Ser Gly Arg Pro Cys Glu Pro Gly Cys Arg Thr Phe Phe Arg Val
50 55 60

Cys Leu Lys His Phe Gln Ala Val Val Ser Pro Gly Pro Cys Thr
65 70 75

Phe Gly Thr Val Ser Thr Pro Val Leu Gly Thr Asn Ser Phe Ala
80 85 90

Val Arg Asp Asp Ser Ser Gly Gly Gly Arg Asn Pro Leu Gln Leu
95 100 105

Pro Phe Asn Phe Thr Trp Pro Gly Thr Phe Ser Leu Ile Ile Glu
110 115 120

Ala Trp His Ala Pro Gly Asp Asp Leu Arg Pro Glu Ala Leu Pro
125 130 135

Pro Asp Ala Leu Ile Ser Lys Ile Ala Ile Gln Gly Ser Leu Ala
140 145 150

Val Gly Gln Asn Trp Leu Leu Asp Glu Gln Thr Ser Thr Leu Thr
155 160 165

Arg Leu Arg Tyr Ser Tyr Arg Val Ile Cys Ser Asp Asn Tyr Tyr
170 175 180

Gly Asp Asn Cys Ser Arg Leu Cys Lys Lys Arg Asn Asp His Phe
185 190 195

Gly His Tyr Val Cys Gln Pro Asp Gly Asn Leu Ser Cys Leu Pro
200 205 210

Gly Trp Thr Gly Glu Tyr Cys Gln Gln Pro Ile Cys Leu Ser Gly
215 220 225

Cys His Glu Gln Asn Gly Tyr Cys Ser Lys Pro Ala Glu Cys Leu
230 235 240

Cys Arg Pro Gly Trp Gln Gly Arg Leu Cys Asn Glu Cys Ile Pro
245 250 255

His Asn Gly Cys Arg His Gly Thr Cys Ser Thr Pro Trp Gln Cys
260 265 270

Thr Cys Asp Glu Gly Trp Gly Gly Leu Phe Cys Asp Gln Asp Leu
275 280 285

Asn Tyr Cys Thr His His Ser Pro Cys Lys Asn Gly Ala Thr Cys
290 295 300

Ser Asn Ser Gly Gln Arg Ser Tyr Thr Cys Thr Cys Arg Pro Gly
305 310 315

Tyr Thr Gly Val Asp Cys Glu Leu Glu Leu Ser Glu Cys Asp Ser
320 325 330

Asn Pro Cys Arg Asn Gly Gly Ser Cys Lys Asp Gln Glu Asp Gly
335 340 345

Tyr His Cys Leu Cys Pro Pro Gly Tyr Tyr Gly Leu His Cys Glu
350 355 360

His Ser Thr Leu Ser Cys Ala Asp Ser Pro Cys Phe Asn Gly Gly
365 370 375

Ser Cys Arg Glu Arg Asn Gln Gly Ala Asn Tyr Ala Cys Glu Cys
380 385 390

Pro Pro Asn Phe Thr Gly Ser Asn Cys Glu Lys Lys Val Asp Arg
395 400 405

Cys Thr Ser Asn Pro Cys Ala Asn Gly Gly Gln Cys Leu Asn Arg
410 415 420

Gly Pro Ser Arg Met Cys Arg Cys Arg Pro Gly Phe Thr Gly Thr
425 430 435

Tyr Cys Glu Leu His Val Ser Asp Cys Ala Arg Asn Pro Cys Ala
440 445 450

His Gly Gly Thr Cys His Asp Leu Glu Asn Gly Leu Met Cys Thr
455 460 465

Cys Pro Ala Gly Phe Ser Gly Arg Arg Cys Glu Val Arg Thr Ser
470 475 480

Ile Asp Ala Cys Ala Ser Ser Pro Cys Phe Asn Arg Ala Thr Cys
485 490 495

Tyr Thr Asp Leu Ser Thr Asp Thr Phe Val Cys Asn Cys Pro Tyr
500 505 510

Gly Phe Val Gly Ser Arg Cys Glu Phe Pro Val Gly Leu Pro Pro
515 520 525

Ser Phe Pro Trp Val Ala Val Ser Leu Gly Val Gly Leu Ala Val
530 535 540

Leu Leu Val Leu Leu Gly Met Val Ala Val Ala Val Arg Gln Leu
545 550 555

Arg Leu Arg Arg Pro Asp Asp Gly Ser Arg Glu Ala Met Asn Asn
560 565 570

Leu Ser Asp Phe Gln Lys Asp Asn Leu Ile Pro Ala Ala Gln Leu

```
                575                 580                 585

Lys Asn Thr Asn Gln Lys Lys Glu Leu Glu Val Asp Cys Gly Leu
                590                 595                 600

Asp Lys Ser Asn Cys Gly Lys Gln Gln Asn His Thr Leu Asp Tyr
                605                 610                 615

Asn Leu Ala Pro Gly Pro Leu Gly Arg Gly Thr Met Pro Gly Lys
                620                 625                 630

Phe Pro His Ser Asp Lys Ser Leu Gly Glu Lys Ala Pro Leu Arg
                635                 640                 645

Leu His Ser Glu Lys Pro Glu Cys Arg Ile Ser Ala Ile Cys Ser
                650                 655                 660

Pro Arg Asp Ser Met Tyr Gln Ser Val Cys Leu Ile Ser Glu Glu
                665                 670                 675

Arg Asn Glu Cys Val Ile Ala Thr Glu Val
                680                 685

<210> 89
<211> 1893
<212> DNA
<213> Homo Sapien

<400> 89
gtctccgcgt cacaggaact tcagcaccca cagggcggac agcgctcccc 50

tctacctgga gacttgactc ccgcgcgccc caaccctgct tatcccttga 100

ccgtcgagtg tcagagatcc tgcagccgcc cagtcccggc ccctctcccg 150

ccccacaccc accctcctgg ctcttcctgt ttttactcct ccttttcatt 200

cataacaaaa gctacagctc caggagccca gcgccgggct gtgacccaag 250

ccgagcgtgg aagaatgggg ttcctcggga ccggcacttg gattctggtg 300

ttagtgctcc cgattcaagc tttccccaaa cctggaggaa gccaagacaa 350

atctctacat aatagagaat taagtgcaga aagacctttg aatgaacaga 400

ttgctgaagc agaagaagac aagattaaaa aaacatatcc tccagaaaac 450

aagccaggtc agagcaacta ttcttttgtt gataacttga acctgctaaa 500

ggcaataaca gaaaaggaaa aaattgagaa agaaaagacaa tctataagaa 550

gctccccact tgataataag ttgaatgtgg aagatgttga ttcaaccaag 600

aatcgaaaac tgatcgatga ttatgactct actaagagtg gattggatca 650

taaatttcaa gatgatccag atggtcttca tcaactagac gggactcctt 700

taaccgctga agacattgtc cataaaatcg ctgccaggat ttatgaagaa 750

aatgacagag ccgtgtttga caagattgtt tctaaactac ttaatctcgg 800

ccttatcaca gaaagccaag cacatacact ggaagatgaa gtagcagagg 850

ttttacaaaa attaatctca aaggaagcca acaattatga ggaggatccc 900
```

aataagccca caagctggac tgagaatcag gctggaaaaa taccagagaa 950
agtgactcca atggcagcaa ttcaagatgg tcttgctaag ggagaaaacg 1000
atgaaacagt atctaacaca ttaaccttga caaatggctt ggaaaggaga 1050
actaaaacct acagtgaaga caactttgag gaactccaat atttcccaaa 1100
tttctatgcg ctactgaaaa gtattgattc agaaaaagaa gcaaaagaga 1150
aagaaacact gattactatc atgaaaacac tgattgactt tgtgaagatg 1200
atggtgaaat atggaacaat atctccagaa gaaggtgttt cctaccttga 1250
aaacttggat gaaatgattg ctcttcagac caaaaacaag ctagaaaaaa 1300
atgctactga caatataagc aagcttttcc cagcaccatc agagaagagt 1350
catgaagaaa cagacagtac caaggaagaa gcagctaaga tggaaaagga 1400
atatggaagc ttgaaggatt ccacaaaaga tgataactcc aacccaggag 1450
gaaagacaga tgaacccaaa ggaaaaacag aagcctattt ggaagccatc 1500
agaaaaaata ttgaatggtt gaagaaacat gacaaaaagg gaaataaaga 1550
agattatgac ctttcaaaga tgagagactt catcaataaa caagctgatg 1600
cttatgtgga gaaaggcatc cttgacaagg aagaagccga ggccatcaag 1650
cgcatttata gcagcctgta aaaatggcaa aagatccagg agtctttcaa 1700
ctgtttcaga aaacataata tagcttaaaa cacttctaat tctgtgatta 1750
aaatttttg acccaagggt tattagaaag tgctgaattt acagtagtta 1800
accttttaca agtggttaaa acatagcttt cttcccgtaa aaactatctg 1850
aaagtaaagt tgtatgtaag ctgaaaaaaa aaaaaaaaaa aaa 1893

<210> 90
<211> 468
<212> PRT
<213> Homo Sapien

<400> 90

```
Met Gly Phe Leu Gly Thr Gly Thr Trp Ile Leu Val Leu Val Leu
 1               5                  10                  15

Pro Ile Gln Ala Phe Pro Lys Pro Gly Gly Ser Gln Asp Lys Ser
                20                  25                  30

Leu His Asn Arg Glu Leu Ser Ala Glu Arg Pro Leu Asn Glu Gln
                35                  40                  45

Ile Ala Glu Ala Glu Glu Asp Lys Ile Lys Lys Thr Tyr Pro Pro
                50                  55                  60

Glu Asn Lys Pro Gly Gln Ser Asn Tyr Ser Phe Val Asp Asn Leu
                65                  70                  75

Asn Leu Leu Lys Ala Ile Thr Glu Lys Glu Lys Ile Glu Lys Glu
                80                  85                  90
```

```
Arg Gln Ser Ile Arg Ser Ser Pro Leu Asp Asn Lys Leu Asn Val
                95                  100                 105

Glu Asp Val Asp Ser Thr Lys Asn Arg Lys Leu Ile Asp Asp Tyr
                110                 115                 120

Asp Ser Thr Lys Ser Gly Leu Asp His Lys Phe Gln Asp Asp Pro
                125                 130                 135

Asp Gly Leu His Gln Leu Asp Gly Thr Pro Leu Thr Ala Glu Asp
                140                 145                 150

Ile Val His Lys Ile Ala Ala Arg Ile Tyr Glu Glu Asn Asp Arg
                155                 160                 165

Ala Val Phe Asp Lys Ile Val Ser Lys Leu Leu Asn Leu Gly Leu
                170                 175                 180

Ile Thr Glu Ser Gln Ala His Thr Leu Glu Asp Glu Val Ala Glu
                185                 190                 195

Val Leu Gln Lys Leu Ile Ser Lys Glu Ala Asn Asn Tyr Glu Glu
                200                 205                 210

Asp Pro Asn Lys Pro Thr Ser Trp Thr Glu Asn Gln Ala Gly Lys
                215                 220                 225

Ile Pro Glu Lys Val Thr Pro Met Ala Ala Ile Gln Asp Gly Leu
                230                 235                 240

Ala Lys Gly Glu Asn Asp Glu Thr Val Ser Asn Thr Leu Thr Leu
                245                 250                 255

Thr Asn Gly Leu Glu Arg Arg Thr Lys Thr Tyr Ser Glu Asp Asn
                260                 265                 270

Phe Glu Glu Leu Gln Tyr Phe Pro Asn Phe Tyr Ala Leu Leu Lys
                275                 280                 285

Ser Ile Asp Ser Glu Lys Glu Ala Lys Glu Lys Glu Thr Leu Ile
                290                 295                 300

Thr Ile Met Lys Thr Leu Ile Asp Phe Val Lys Met Met Val Lys
                305                 310                 315

Tyr Gly Thr Ile Ser Pro Glu Glu Gly Val Ser Tyr Leu Glu Asn
                320                 325                 330

Leu Asp Glu Met Ile Ala Leu Gln Thr Lys Asn Lys Leu Glu Lys
                335                 340                 345

Asn Ala Thr Asp Asn Ile Ser Lys Leu Phe Pro Ala Pro Ser Glu
                350                 355                 360

Lys Ser His Glu Glu Thr Asp Ser Thr Lys Glu Glu Ala Ala Lys
                365                 370                 375

Met Glu Lys Glu Tyr Gly Ser Leu Lys Asp Ser Thr Lys Asp Asp
                380                 385                 390

Asn Ser Asn Pro Gly Gly Lys Thr Asp Glu Pro Lys Gly Lys Thr
                395                 400                 405
```

```
Glu Ala Tyr Leu Glu Ala Ile Arg Lys Asn Ile Glu Trp Leu Lys
                410                 415                 420

Lys His Asp Lys Lys Gly Asn Lys Glu Asp Tyr Asp Leu Ser Lys
                425                 430                 435

Met Arg Asp Phe Ile Asn Lys Gln Ala Asp Ala Tyr Val Glu Lys
                440                 445                 450

Gly Ile Leu Asp Lys Glu Glu Ala Glu Ala Ile Lys Arg Ile Tyr
                455                 460                 465

Ser Ser Leu
```

<210> 91
<211> 1240
<212> DNA
<213> Homo Sapien

<400> 91

```
tgcatcagtg cccaggcaag cccaggagtt gacatttctc tgcccagcca 50
tgggcctcac cctgctcttg ctgctgctcc tgggactaga aggtcagggc 100
atagttggca gcctccctga ggtgctgcag gcacccgtgg gaagctccat 150
tctggtgcag tgccactaca ggctccagga tgtcaaagct cagaaggtgt 200
ggtgccggtt cttgccggag gggtgccagc ccctggtgtc ctcagctgtg 250
gatcgcagag ctccagcggg caggcgtacg tttctcacag acctgggtgg 300
gggcctgctg caggtggaaa tggttaccct gcaggaagag gatgctggcg 350
agtatggctg catggtggat ggggccaggg ggccccagat tttgcacaga 400
gtctctctga acatactgcc cccagaggaa gaagaagaga cccataagat 450
tggcagtctg gctgagaacg cattctcaga ccctgcaggc agtgccaacc 500
ctttggaacc cagccaggat gagaagagca tccccttgat ctggggtgct 550
gtgctcctgg taggtctgct ggtggcagcg gtggtgctgt ttgctgtgat 600
ggccaagagg aaacaagaat ccctcctcag tggtccacca cgtcagtgac 650
tctggaccgg ctgctgaatt gcctttggat gtaccacaca ttaggcttga 700
ctcaccacct tcatttgaca ataccaccta caccagccta cctcttgatt 750
ccccatcagg aaaaccttca ctcccagctc catcctcatt gccccctcta 800
cctcctaagg tcctggtctg ctccaagcct gtgacatatg ccacagtaat 850
cttcccggga gggaacaagg gtggagggac ctcgtgtggg ccagcccaga 900
atccacctaa caatcagact ccatccagct aagctgctca tcacacttta 950
aactcatgag gaccatccct aggggttctg tgcatccatc cagccagctc 1000
atgccctagg atccttagga tatctgagca accagggact ttaagatcta 1050
atccaatgtc ctaactttac tagggaaagt gacgctcaga catgactgag 1100
```

atgtcttggg gaagacctcc ctgcacccaa ctcccccact ggttcttcta 1150

ccattacaca ctgggctaaa taaaccctaa taatgatgtg caaaaaaaaa 1200

aaaaaaaaaa aaaaaaaaaa aaaaaaaaaa aaaaaaaaaa 1240

<210> 92
<211> 199
<212> PRT
<213> Homo Sapien

<400> 92
Met Gly Leu Thr Leu Leu Leu Leu Leu Leu Leu Gly Leu Glu Gly
1 5 10 15

Gln Gly Ile Val Gly Ser Leu Pro Glu Val Leu Gln Ala Pro Val
20 25 30

Gly Ser Ser Ile Leu Val Gln Cys His Tyr Arg Leu Gln Asp Val
35 40 45

Lys Ala Gln Lys Val Trp Cys Arg Phe Leu Pro Glu Gly Cys Gln
50 55 60

Pro Leu Val Ser Ser Ala Val Asp Arg Arg Ala Pro Ala Gly Arg
65 70 75

Arg Thr Phe Leu Thr Asp Leu Gly Gly Gly Leu Leu Gln Val Glu
80 85 90

Met Val Thr Leu Gln Glu Glu Asp Ala Gly Glu Tyr Gly Cys Met
95 100 105

Val Asp Gly Ala Arg Gly Pro Gln Ile Leu His Arg Val Ser Leu
110 115 120

Asn Ile Leu Pro Pro Glu Glu Glu Glu Glu Thr His Lys Ile Gly
125 130 135

Ser Leu Ala Glu Asn Ala Phe Ser Asp Pro Ala Gly Ser Ala Asn
140 145 150

Pro Leu Glu Pro Ser Gln Asp Glu Lys Ser Ile Pro Leu Ile Trp
155 160 165

Gly Ala Val Leu Leu Val Gly Leu Leu Val Ala Ala Val Val Leu
170 175 180

Phe Ala Val Met Ala Lys Arg Lys Gln Glu Ser Leu Leu Ser Gly
185 190 195

Pro Pro Arg Gln

<210> 93
<211> 2285
<212> DNA
<213> Homo Sapien

<400> 93
ggcggcgttg ccgggctctc cggaaggaga cgtggcggcg gttgggccgg 50

tgatacccgg gcgctttata gtcccgccgc ctcctcctcc acctcctcct 100

cctcctcctc tcctcctggg gcagaggagg ttgtggcggt ggctggagaa 150
agcggcggcg gaggatggag gaaggaggcg gcggcgtacg gagtctggtc 200
ccgggcgggc cggtgttact ggtcctctgc ggcctcctgg aggcgtccgg 250
cggcggccga gcccttcctc aactcagcga tgacatccct ttccgagtca 300
actggcccgg caccgagttc tctctgccca caactggagt tttatataaa 350
gaagataatt atgtcatcat gacaactgca cataaagaaa aatataaatg 400
catacttccc cttgtgacaa gtggggatga ggaagaagaa aaggattata 450
aaggccctaa tccaagagag cttttggagc cactatttaa acaaagcagt 500
tgttcctaca gaattgagtc ttattggact tacgaagtat gtcatggaaa 550
acacattcgg cagtaccatg aagagaaaga aactggtcag aaaataaata 600
ttcacgagta ctaccttggg aatatgttgg ccaagaacct tctatttgaa 650
aaagaacgag aagcagaaga aaaggaaaaa tcaaatgaga ttcccactaa 700
aaatatcgaa ggtcagatga caccatacta tcctgtggga atgggaaatg 750
gtacaccttg tagtttgaaa cagaaccggc ccagatcaag tactgtgatg 800
tacatatgtc atcctgaatc taagcatgaa attctttcag tagctgaagt 850
tacaacttgt gaatatgaag ttgtcatttt gacaccactc ttgtgcagtc 900
atcctaaata taggttcaga gcatctcctg tgaatgacat attttgtcaa 950
tcactgccag gatctccatt taagcccctc accctgaggc agctggagca 1000
gcaggaagaa atactaaggg tgccttttag gagaaataaa gagggtgtcg 1050
gttggtggaa atatgaattc tgctatggca aacatgtaca tcaataccat 1100
gaggacaagg atagtgggaa aacctctgtg gttgtcggga catggaacca 1150
agaagagcat attgaatggg ctaagaagaa tactgctaga gcttatcatc 1200
ttcaagacga tggtacccag acagtcagga tggtgtcaca tttttatgga 1250
aatggagata tttgtgatat aactgacaaa ccaagacagg tgactgtaaa 1300
actaaagtgc aaagaatcag attcacctca tgctgttact gtatatatgc 1350
tagagcctca ctcctgtcaa tatattcttg ggttgaatc tccagtgatc 1400
tgtaaaatct tagatacagc agatgaaaat ggacttcttt ctctccccaa 1450
ctaaaggata ttaaagttag gggaaagaaa agatcattga aagtcatgat 1500
aatttctgtc ccactgtgtc tcattataga gttctcagcc attggacctc 1550
ttctaaagga tggtataaaa tgactctcaa ccactttgtg aatacatatg 1600
tgtatataag aggttattga taaacttctg aggcagacat ttgtctcgct 1650
ttttttcatt tttgttgtgt cttataaact gactgttttt ctttgcttgg 1700

atactgtgat tccaaaataa atctcatcca agcaagttag agtccagcct 1750

aatcaaatgt cataattgtt gtacctattg aaagttttta aataatagat 1800

ttattatgta aattatagta tatgtaagta gctaatgaag taaagatcat 1850

gaagaaagaa attgataggt gtaaatgaga gaccatgtaa aatatgtaaa 1900

ttctagtacc tgaaatcctt tcaacagatt tttatatagc aactgctctc 1950

tgcaagtagt taaactagaa actgggcaca tggtagaggc tcacatggga 2000

gttgtcctca cccttgttaa tctcaagaaa ctcttattta taataggttg 2050

cttctctctc agaactttta tctattactt ttttcttctt atgagtatgt 2100

ttactctcag agtatctatc tgatgtagac agttggtgat gcttctgaga 2150

ctcagaatgg tttactctaa caaaacactg tgctgtctat cccttgtact 2200

tgcctactgt aatatggatt tcacttctga acagtttaca gcacaatatt 2250

tattttaaag tgaataaaat gtccacaagc aaaaa 2285

<210> 94
<211> 429
<212> PRT
<213> Homo Sapien

<400> 94

```
Met Glu Glu Gly Gly Gly Gly Val Arg Ser Leu Val Pro Gly Gly
  1               5                  10                  15

Pro Val Leu Leu Val Leu Cys Gly Leu Leu Glu Ala Ser Gly Gly
                 20                  25                  30

Gly Arg Ala Leu Pro Gln Leu Ser Asp Asp Ile Pro Phe Arg Val
                 35                  40                  45

Asn Trp Pro Gly Thr Glu Phe Ser Leu Pro Thr Thr Gly Val Leu
                 50                  55                  60

Tyr Lys Glu Asp Asn Tyr Val Ile Met Thr Thr Ala His Lys Glu
                 65                  70                  75

Lys Tyr Lys Cys Ile Leu Pro Leu Val Thr Ser Gly Asp Glu Glu
                 80                  85                  90

Glu Glu Lys Asp Tyr Lys Gly Pro Asn Pro Arg Glu Leu Leu Glu
                 95                 100                 105

Pro Leu Phe Lys Gln Ser Ser Cys Ser Tyr Arg Ile Glu Ser Tyr
                110                 115                 120

Trp Thr Tyr Glu Val Cys His Gly Lys His Ile Arg Gln Tyr His
                125                 130                 135

Glu Glu Lys Glu Thr Gly Gln Lys Ile Asn Ile His Glu Tyr Tyr
                140                 145                 150

Leu Gly Asn Met Leu Ala Lys Asn Leu Leu Phe Glu Lys Glu Arg
                155                 160                 165
```

Glu Ala Glu Glu Lys Glu Lys Ser Asn Glu Ile Pro Thr Lys Asn
170 175 180

Ile Glu Gly Gln Met Thr Pro Tyr Tyr Pro Val Gly Met Gly Asn
185 190 195

Gly Thr Pro Cys Ser Leu Lys Gln Asn Arg Pro Arg Ser Ser Thr
200 205 210

Val Met Tyr Ile Cys His Pro Glu Ser Lys His Glu Ile Leu Ser
215 220 225

Val Ala Glu Val Thr Thr Cys Glu Tyr Glu Val Val Ile Leu Thr
230 235 240

Pro Leu Leu Cys Ser His Pro Lys Tyr Arg Phe Arg Ala Ser Pro
245 250 255

Val Asn Asp Ile Phe Cys Gln Ser Leu Pro Gly Ser Pro Phe Lys
260 265 270

Pro Leu Thr Leu Arg Gln Leu Glu Gln Gln Glu Glu Ile Leu Arg
275 280 285

Val Pro Phe Arg Arg Asn Lys Glu Gly Val Gly Trp Trp Lys Tyr
290 295 300

Glu Phe Cys Tyr Gly Lys His Val His Gln Tyr His Glu Asp Lys
305 310 315

Asp Ser Gly Lys Thr Ser Val Val Val Gly Thr Trp Asn Gln Glu
320 325 330

Glu His Ile Glu Trp Ala Lys Lys Asn Thr Ala Arg Ala Tyr His
335 340 345

Leu Gln Asp Asp Gly Thr Gln Thr Val Arg Met Val Ser His Phe
350 355 360

Tyr Gly Asn Gly Asp Ile Cys Asp Ile Thr Asp Lys Pro Arg Gln
365 370 375

Val Thr Val Lys Leu Lys Cys Lys Glu Ser Asp Ser Pro His Ala
380 385 390

Val Thr Val Tyr Met Leu Glu Pro His Ser Cys Gln Tyr Ile Leu
395 400 405

Gly Val Glu Ser Pro Val Ile Cys Lys Ile Leu Asp Thr Ala Asp
410 415 420

Glu Asn Gly Leu Leu Ser Leu Pro Asn
425

<210> 95
<211> 2542
<212> DNA
<213> Homo Sapien

<400> 95
ttccgtttct gggaggagtg aggggcaacg ggtcggagaa aaaggaaaaa 50

agaagggctc agcgcctccc cgccgggccg tggacagagg ggcacagttt 100

cggcaggcgg gtgaggtcgc tgagggcccg ccggagatgt tttccttgtc 150
gagcacggtg caaccccagg ttacagttcc tctgagtcat ctcatcaatg 200
ccttccatac accaaaaaac acttctgttt ctctcagtgg agtgtcagtt 250
tctcaaaacc agcatcgaga tgtagttcct gagcatgagg ctcccagcag 300
tgagccttca cttaacttaa gggaccttgg attatctgaa ctaaaaattg 350
gacagattga tcagctggta gaaaatctac ttcctggatt ttgtaaaggc 400
aaaaacattt cttcccattg gcatacatcc catgtctctg cacaatcctt 450
ctttgaaaat aaatatggta acttagatat atttagtaca ttacgttcct 500
cttgcttgta tcgacatcat tcaagagctc ttcaaagcat ttgttcagat 550
cttcagtact ggccagtttt catacagtct cggggtttta aaactttgaa 600
atcaaggaca cgacgtctcc agtctacctc cgagagatta gctgaaacac 650
agaatatagc gccatcattc gtgaaggggt ttcttttgcg ggacagagga 700
tcagatgttg agagtttgga caaactcatg aaaaccaaaa atatacctga 750
agctcaccaa gatgcattta aaactggttt tgcggaaggt tttctgaaag 800
ctcaagcact cacacaaaaa accaatgatt ccctaaggcg aacccgtctg 850
attctcttcg ttctgctgct attcggcatt tatggacttc taaaaaaccc 900
atttttatct gtccgcttcc ggacaacaac agggcttgat tctgcagtag 950
atcctgtcca gatgaaaaat gtcacctttg aacatgttaa aggggtggag 1000
gaagctaaac aagaattaca ggaagttgtt gaattcttga aaaatccaca 1050
aaaatttact attcttggag gtaaacttcc aaaaggaatt cttttagttg 1100
gacccccagg gactggaaag acacttcttg cccgagctgt ggcgggagaa 1150
gctgatgttc ctttttatta tgcttctgga tccgaatttg atgagatgtt 1200
tgtgggtgtg ggagccagcc gtatcagaaa tctttttagg gaagcaaagg 1250
cgaatgctcc ttgtgttata tttattgatg aattagattc tgttggtggg 1300
aagagaattg aatctccaat gcatccatat tcaaggcaga ccataaatca 1350
acttcttgct gaaatggatg gttttaaacc caatgaagga gttatcataa 1400
taggagccac aaacttccca gaggcattag ataatgcctt aatacgtcct 1450
ggtcgttttg acatgcaagt tacagttcca aggccagatg taaaaggtcg 1500
aacagaaatt ttgaaatggt atctcaataa aataaagttt gatcaatccg 1550
ttgatccaga aattatagct cgaggtactg ttggcttttc cggagcagag 1600
ttggagaatc ttgtgaacca ggctgcatta aaagcagctg ttgatggaaa 1650
agaaatggtt accatgaagg agctggagtt ttccaaagac aaaattctaa 1700

tggggcctga aagaagaagt gtggaaattg ataacaaaaa caaaaccatc 1750

acagcatatc atgaatctgg tcatgccatt attgcatatt acacaaaaga 1800

tgcaatgcct atcaacaaag ctacaatcat gccacggggg ccaacacttg 1850

gacatgtgtc cctgttacct gagaatgaca gatggaatga aactagagcc 1900

cagctgcttg cacaaatgga tgttagtatg ggaggaagag tggcagagga 1950

gcttatattt ggaaccgacc atattacaac aggtgcttcc agtgattttg 2000

ataatgccac taaaatagca aagcggatgg ttaccaaatt tggaatgagt 2050

gaaaagcttg gagttatgac ctacagtgat acagggaaac taagtccaga 2100

aacccaatct gccatcgaac aagaaataag aatccttcta agggactcat 2150

atgaacgagc aaaacatatc ttgaaaactc atgcaaagga gcataagaat 2200

ctcgcagaag ctttattgac ctatgagact ttggatgcca aagagattca 2250

aattgttctt gaggggaaaa agttggaagt gagatgataa ctctcttgat 2300

atggatgctt gctggtttta ttgcaagaat ataagtagca ttgcagtagt 2350

ctacttttac aacgctttcc cctcattctt gatgtggtgt aattgaaggg 2400

tgtgaaatgc tttgtcaatc atttgtcaca tttatccagt ttgggttatt 2450

ctcattatga cacctattgc aaattagcat cccatggcaa atatattttg 2500

aaaaaataaa gaactatcag gattgaaaac aaaaaaaaaa aa 2542

<210> 96
<211> 716
<212> PRT
<213> Homo Sapien

<400> 96

```
Met Phe Ser Leu Ser Ser Thr Val Gln Pro Gln Val Thr Val Pro
 1               5                  10                  15

Leu Ser His Leu Ile Asn Ala Phe His Thr Pro Lys Asn Thr Ser
                20                  25                  30

Val Ser Leu Ser Gly Val Ser Val Ser Gln Asn Gln His Arg Asp
                35                  40                  45

Val Val Pro Glu His Glu Ala Pro Ser Ser Glu Pro Ser Leu Asn
                50                  55                  60

Leu Arg Asp Leu Gly Leu Ser Glu Leu Lys Ile Gly Gln Ile Asp
                65                  70                  75

Gln Leu Val Glu Asn Leu Leu Pro Gly Phe Cys Lys Gly Lys Asn
                80                  85                  90

Ile Ser Ser His Trp His Thr Ser His Val Ser Ala Gln Ser Phe
                95                 100                 105

Phe Glu Asn Lys Tyr Gly Asn Leu Asp Ile Phe Ser Thr Leu Arg
               110                 115                 120
```

```
Ser Ser Cys Leu Tyr Arg His His Ser Arg Ala Leu Gln Ser Ile
                125                 130                 135

Cys Ser Asp Leu Gln Tyr Trp Pro Val Phe Ile Gln Ser Arg Gly
                140                 145                 150

Phe Lys Thr Leu Lys Ser Arg Thr Arg Arg Leu Gln Ser Thr Ser
                155                 160                 165

Glu Arg Leu Ala Glu Thr Gln Asn Ile Ala Pro Ser Phe Val Lys
                170                 175                 180

Gly Phe Leu Leu Arg Asp Arg Gly Ser Asp Val Glu Ser Leu Asp
                185                 190                 195

Lys Leu Met Lys Thr Lys Asn Ile Pro Glu Ala His Gln Asp Ala
                200                 205                 210

Phe Lys Thr Gly Phe Ala Glu Gly Phe Leu Lys Ala Gln Ala Leu
                215                 220                 225

Thr Gln Lys Thr Asn Asp Ser Leu Arg Arg Thr Arg Leu Ile Leu
                230                 235                 240

Phe Val Leu Leu Leu Phe Gly Ile Tyr Gly Leu Leu Lys Asn Pro
                245                 250                 255

Phe Leu Ser Val Arg Phe Arg Thr Thr Thr Gly Leu Asp Ser Ala
                260                 265                 270

Val Asp Pro Val Gln Met Lys Asn Val Thr Phe Glu His Val Lys
                275                 280                 285

Gly Val Glu Glu Ala Lys Gln Glu Leu Gln Glu Val Val Glu Phe
                290                 295                 300

Leu Lys Asn Pro Gln Lys Phe Thr Ile Leu Gly Gly Lys Leu Pro
                305                 310                 315

Lys Gly Ile Leu Leu Val Gly Pro Pro Gly Thr Gly Lys Thr Leu
                320                 325                 330

Leu Ala Arg Ala Val Ala Gly Glu Ala Asp Val Pro Phe Tyr Tyr
                335                 340                 345

Ala Ser Gly Ser Glu Phe Asp Glu Met Phe Val Gly Val Gly Ala
                350                 355                 360

Ser Arg Ile Arg Asn Leu Phe Arg Glu Ala Lys Ala Asn Ala Pro
                365                 370                 375

Cys Val Ile Phe Ile Asp Glu Leu Asp Ser Val Gly Gly Lys Arg
                380                 385                 390

Ile Glu Ser Pro Met His Pro Tyr Ser Arg Gln Thr Ile Asn Gln
                395                 400                 405

Leu Leu Ala Glu Met Asp Gly Phe Lys Pro Asn Glu Gly Val Ile
                410                 415                 420

Ile Ile Gly Ala Thr Asn Phe Pro Glu Ala Leu Asp Asn Ala Leu
                425                 430                 435

Ile Arg Pro Gly Arg Phe Asp Met Gln Val Thr Val Pro Arg Pro
```

```
                440                 445                 450

Asp Val Lys Gly Arg Thr Glu Ile Leu Lys Trp Tyr Leu Asn Lys
                455                 460                 465

Ile Lys Phe Asp Gln Ser Val Asp Pro Glu Ile Ile Ala Arg Gly
                470                 475                 480

Thr Val Gly Phe Ser Gly Ala Glu Leu Glu Asn Leu Val Asn Gln
                485                 490                 495

Ala Ala Leu Lys Ala Ala Val Asp Gly Lys Glu Met Val Thr Met
                500                 505                 510

Lys Glu Leu Glu Phe Ser Lys Asp Lys Ile Leu Met Gly Pro Glu
                515                 520                 525

Arg Arg Ser Val Glu Ile Asp Asn Lys Asn Lys Thr Ile Thr Ala
                530                 535                 540

Tyr His Glu Ser Gly His Ala Ile Ile Ala Tyr Tyr Thr Lys Asp
                545                 550                 555

Ala Met Pro Ile Asn Lys Ala Thr Ile Met Pro Arg Gly Pro Thr
                560                 565                 570

Leu Gly His Val Ser Leu Leu Pro Glu Asn Asp Arg Trp Asn Glu
                575                 580                 585

Thr Arg Ala Gln Leu Leu Ala Gln Met Asp Val Ser Met Gly Gly
                590                 595                 600

Arg Val Ala Glu Glu Leu Ile Phe Gly Thr Asp His Ile Thr Thr
                605                 610                 615

Gly Ala Ser Ser Asp Phe Asp Asn Ala Thr Lys Ile Ala Lys Arg
                620                 625                 630

Met Val Thr Lys Phe Gly Met Ser Glu Lys Leu Gly Val Met Thr
                635                 640                 645

Tyr Ser Asp Thr Gly Lys Leu Ser Pro Glu Thr Gln Ser Ala Ile
                650                 655                 660

Glu Gln Glu Ile Arg Ile Leu Leu Arg Asp Ser Tyr Glu Arg Ala
                665                 670                 675

Lys His Ile Leu Lys Thr His Ala Lys Glu His Lys Asn Leu Ala
                680                 685                 690

Glu Ala Leu Leu Thr Tyr Glu Thr Leu Asp Ala Lys Glu Ile Gln
                695                 700                 705

Ile Val Leu Glu Gly Lys Lys Leu Glu Val Arg
                710                 715
```

<210> 97
<211> 1571
<212> DNA
<213> Homo Sapien

<400> 97
gatggcgcag ccacagcttc tgtgagattc gatttctccc cagttcccct 50

gtgggtctga ggggaccaga agggtgagct acgttggctt tctggaaggg 100
gaggctatat gcgtcaattc cccaaaacaa gttttgacat ttcccctgaa 150
atgtcattct ctatctattc actgcaagtg cctgctgttc caggccttac 200
ctgctgggca ctaacggcgg agccaggatg gggacagaat aaaggagcca 250
cgacctgtgc caccaactcg cactcagact ctgaactcag acctgaaatc 300
ttctcttcac gggaggcttg gcagtttttc ttactcctgt ggtctccaga 350
tttcaggcct aagatgaaag cctctagtct tgccttcagc cttctctctg 400
ctgcgtttta tctcctatgg actccttcca ctggactgaa gacactcaat 450
ttgggaagct gtgtgatcgc cacaaacctt caggaaatac gaaatggatt 500
ttctgagata cggggcagtg tgcaagccaa agatggaaac attgacatca 550
gaatcttaag gaggactgag tctttgcaag acacaaagcc tgcgaatcga 600
tgctgcctcc tgcgccattt gctaagactc tatctggaca gggtatttaa 650
aaactaccag acccctgacc attatactct ccggaagatc agcagcctcg 700
ccaattcctt tcttaccatc aagaaggacc tccggctctc tcatgcccac 750
atgacatgcc attgtgggga ggaagcaatg aagaaataca gccagattct 800
gagtcacttt gaaaagctgg aacctcaggc agcagttgtg aaggctttgg 850
gggaactaga cattcttctg caatggatgg aggagacaga ataggaggaa 900
agtgatgctg ctgctaagaa tattcgaggt caagagctcc agtcttcaat 950
acctgcagag gaggcatgac cccaaaccac catctcttta ctgtactagt 1000
cttgtgctgg tcacagtgta tcttatttat gcattacttg cttccttgca 1050
tgattgtctt tatgcatccc caatcttaat tgagaccata cttgtataag 1100
atttttgtaa tatctttctg ctattggata tatttattag ttaatatatt 1150
tatttatttt ttgctattta atgtatttat ttttttactt ggacatgaaa 1200
ctttaaaaaa attcacagat tatatttata acctgactag agcaggtgat 1250
gtatttttat acagtaaaaa aaaaaaacct tgtaaattct agaagagtgg 1300
ctaggggggt tattcatttg tattcaacta aggacatatt tactcatgct 1350
gatgctctgt gagatatttg aaattgaacc aatgactact taggatgggt 1400
tgtggaataa gttttgatgt ggaattgcac atctacctta caattactga 1450
ccatccccag tagactcccc agtcccataa ttgtgtatct tccagccagg 1500
aatcctacac ggccagcatg tatttctaca aataaagttt tctttgcata 1550
ccaaaaaaaa aaaaaaaaaa a 1571

<210> 98
<211> 176

<212> PRT
<213> Homo Sapien

<400> 98
Met Lys Ala Ser Ser Leu Ala Phe Ser Leu Leu Ser Ala Ala Phe
1 5 10 15

Tyr Leu Leu Trp Thr Pro Ser Thr Gly Leu Lys Thr Leu Asn Leu
20 25 30

Gly Ser Cys Val Ile Ala Thr Asn Leu Gln Glu Ile Arg Asn Gly
35 40 45

Phe Ser Glu Ile Arg Gly Ser Val Gln Ala Lys Asp Gly Asn Ile
50 55 60

Asp Ile Arg Ile Leu Arg Arg Thr Glu Ser Leu Gln Asp Thr Lys
65 70 75

Pro Ala Asn Arg Cys Cys Leu Leu Arg His Leu Leu Arg Leu Tyr
80 85 90

Leu Asp Arg Val Phe Lys Asn Tyr Gln Thr Pro Asp His Tyr Thr
95 100 105

Leu Arg Lys Ile Ser Ser Leu Ala Asn Ser Phe Leu Thr Ile Lys
110 115 120

Lys Asp Leu Arg Leu Cys His Ala His Met Thr Cys His Cys Gly
125 130 135

Glu Glu Ala Met Lys Lys Tyr Ser Gln Ile Leu Ser His Phe Glu
140 145 150

Lys Leu Glu Pro Gln Ala Ala Val Val Lys Ala Leu Gly Glu Leu
155 160 165

Asp Ile Leu Leu Gln Trp Met Glu Glu Thr Glu
170 175

<210> 99
<211> 1904
<212> DNA
<213> Homo Sapien

<400> 99
gcgccggctc cgcgcctcgc gcccagtccg cgggccgcgc cgccgctccc 50

gccgctcccg ccgctcccgc agccgccccg ccgcccgccc ggagccccgc 100

gtccctaggc ctggctcccg cctgcccgag acccgcccag cctgccccgc 150

tcagccgcca gagaagatgc ggctgctccc ggaatggttc ctcttgctct 200

ttggcccgtg gctccttagg aaggccgtca gtgcccagat accagagtcc 250

ggaaggccgc agtacctggg gctgcgcccc gccgcggccg gagcgggtgc 300

ccccggccag cagctcccag agccaaggtc ttcggacggc ctaggcgtgg 350

gccgcgcctg gagctgggcc tggccgacca accacacggg ggcgctggcc 400

cgggcagggg cagccggggc gttgcccgcg cagcgcacca agaggaagcc 450

gtccatcaag gcggcgcgcg ccaaaaagat cttcggctgg ggggacttct 500
actttcgggt gcataccctc aagttttcgc tgctggtgac cggcaagatc 550
gtggaccatg tgaacggtac cttcagtgtg tatttccgcc acaactcgtc 600
cagcctgggc aacctcagtg tcagcatcgt gccgccctcc aagcgtgtcg 650
agttcggagg agtctggctg cccgggcctg tcccccaccc tctgcagtct 700
acgctcgccc tggagggggt gcttcctggg ctggggcccc cgctggggat 750
ggcagcagca gcggcggggc cggggcttgg gggctccctc gggggcgcac 800
tggcggggcc gcttgggggc gcgttgggag tgcctggggc caaagagtca 850
cgcgctttca attgccacgt ggagtatgag aagacaaacc gcgcgcgcaa 900
gcaccgaccg tgcctgtacg acccgtcgca ggtgtgtttc accgagcaca 950
cgcagagcca ggccgcctgg ctctgtgcca agcccttcaa agtcatctgt 1000
atcttcgtct ctttcctcag ctttgactac aaactggtgc agaaggtgtg 1050
cccagactat aacttccaga gtgagcaccc ctacttcgga tagcgcccct 1100
ccccagccag tcctgagcct cccgccaaat cccagcctca ctaggtggga 1150
ccccccttccc agtgttctgc cgctcctgtg gccatgtcgc ccactccttc 1200
cactctgggg gcggaggga atggcttctc gggaccctca gctagcgtgg 1250
gtgccctttt ccttatgcgg agtgcccgca aggctggggt agccccctcc 1300
agtacacccc aaagtgaaag ggataagagt gcagccccag aataggcggg 1350
gcttggaggc ggtcccaatg tcccctgggt ccacagtggg tcccctttttc 1400
acccttggcg ctaggctgcg cactcccttt ccccgcagct ttaataactc 1450
ctggcctggc accctcaccc caccctgact ttcccatccc ccagcgcttg 1500
tcctgcttca ccataccccg cctaagactg taaaggccta aaaacctcgg 1550
cctgtcctcc caccattctg cctgccatat gcctgtcccc ttttcctcca 1600
aaccctatta gggtaccgga agcagaaccc ctgggctgag gccctggccc 1650
tgcccccggc ccctgcccct gcccgccccc ctccagtcca ggcagtcgag 1700
ctccacctgc cctctcctgc tgcttcctct cggtgatatt ttttctacgc 1750
caaaacagac gggaaaggga acaaaataaa gtgaaatccc aaaaaaaaaa 1800
aaaaaaaaaa aaaaaaaaaa aaaaaaaaaa aaaaaaaaaa aaaaaaaaaa 1850
aaaaaaaaaa aaaaaaaaaa aaaaaaaaaa aaaaaaaaaa aaaaaaaaaa 1900
aaaa 1904

<210> 100
<211> 308
<212> PRT
<213> Homo Sapien

<400> 100

```
Met Arg Leu Leu Pro Glu Trp Phe Leu Leu Leu Phe Gly Pro Trp
1               5                   10                  15

Leu Leu Arg Lys Ala Val Ser Ala Gln Ile Pro Glu Ser Gly Arg
                20                  25                  30

Pro Gln Tyr Leu Gly Leu Arg Pro Ala Ala Ala Gly Ala Gly Ala
                35                  40                  45

Pro Gly Gln Gln Leu Pro Glu Pro Arg Ser Ser Asp Gly Leu Gly
                50                  55                  60

Val Gly Arg Ala Trp Ser Trp Ala Trp Pro Thr Asn His Thr Gly
                65                  70                  75

Ala Leu Ala Arg Ala Gly Ala Ala Gly Ala Leu Pro Ala Gln Arg
                80                  85                  90

Thr Lys Arg Lys Pro Ser Ile Lys Ala Ala Arg Ala Lys Lys Ile
                95                  100                 105

Phe Gly Trp Gly Asp Phe Tyr Phe Arg Val His Thr Leu Lys Phe
                110                 115                 120

Ser Leu Leu Val Thr Gly Lys Ile Val Asp His Val Asn Gly Thr
                125                 130                 135

Phe Ser Val Tyr Phe Arg His Asn Ser Ser Ser Leu Gly Asn Leu
                140                 145                 150

Ser Val Ser Ile Val Pro Pro Ser Lys Arg Val Glu Phe Gly Gly
                155                 160                 165

Val Trp Leu Pro Gly Pro Val Pro His Pro Leu Gln Ser Thr Leu
                170                 175                 180

Ala Leu Glu Gly Val Leu Pro Gly Leu Gly Pro Pro Leu Gly Met
                185                 190                 195

Ala Ala Ala Ala Ala Gly Pro Gly Leu Gly Gly Ser Leu Gly Gly
                200                 205                 210

Ala Leu Ala Gly Pro Leu Gly Gly Ala Leu Gly Val Pro Gly Ala
                215                 220                 225

Lys Glu Ser Arg Ala Phe Asn Cys His Val Glu Tyr Glu Lys Thr
                230                 235                 240

Asn Arg Ala Arg Lys His Arg Pro Cys Leu Tyr Asp Pro Ser Gln
                245                 250                 255

Val Cys Phe Thr Glu His Thr Gln Ser Gln Ala Ala Trp Leu Cys
                260                 265                 270

Ala Lys Pro Phe Lys Val Ile Cys Ile Phe Val Ser Phe Leu Ser
                275                 280                 285

Phe Asp Tyr Lys Leu Val Gln Lys Val Cys Pro Asp Tyr Asn Phe
                290                 295                 300

Gln Ser Glu His Pro Tyr Phe Gly
                305
```

<210> 101
<211> 2031
<212> DNA
<213> Homo Sapien

<220>
<221> unsure
<222> 2020
<223> unknown base

<400> 101
aatgccccat gcgcacccca cagctcgcgc tcctgcaagt gttctttctg 50

gtgttccccg atggcgtccg gcctcagccc tcttcctccc catcaggggc 100

agtgcccacg tctttggagc tgcagcgagg gacggatggc ggaaccctcc 150

agtccccttc agaggcgact gcaactcgcc cggccgtgcc tggactccct 200

acagtggtcc ctactctcgt gactccctcg gcccctggga ataggactgt 250

ggacctcttc ccagtcttac cgatctgtgt ctgtgacttg actcctggag 300

cctgcgatat aaattgctgc tgcgacaggg actgctatct tctccatccg 350

aggacagttt tctccttctg ccttccaggc agcgtaaggt cttcaagctg 400

ggtttgtgta gacaactctg ttatcttcag gagtaattcc ccgtttcctt 450

caagagtttt catggattct aatggaatca ggcagttttg tgtccatgtg 500

aacaactcaa acttaaacta tttccagaag cttcaaaagg tcaatgcaac 550

caacttccag gccctggctg cagagtttgg aggcgaatca ttcacttcaa 600

cattccaaac tcaatcacca ccatcttttt acagggctgg ggaccccatt 650

cttacttact tccccaagtg gtctgtaata agcttgctga gacaacctgc 700

aggagttgga gctgggggac tctgtgctga aagcaatcct gcaggtttcc 750

tagagagtaa aagtacaact tgcactcgtt ttttcaagaa cctggctagt 800

agctgtacct tggattcagc cctcaatgct gcctcttact ataacttcac 850

agtcttaaag gttccaagaa gcatgactga tccacagaat atggagttcc 900

aggttcctgt aatacttacc tcacaggcta atgctcctct gttggctgga 950

aacacttgtc agaatgtagt ttctcaggtc acctatgaga tagagaccaa 1000

tgggactttt ggaatccaga aagtttctgt cagtttggga caaaccaacc 1050

tgactgttga gccaggcgct tccttacagc aacacttcat ccttcgcttc 1100

agggcttttc aacagagcac agctgcttct ctcaccagtc ctagaagtgg 1150

gaatcctggc tatatagttg ggaagccact cttggctctg actgatgata 1200

taagttactc aatgaccctc ttacagagcc agggtaatgg aagttgctct 1250

gttaaaagac atgaagtgca gtttggagtg aatgcaatat ctggatgcaa 1300

gctcaggttg aagaaggcag actgcagcca cttgcagcag gagatttatc 1350

agactcttca tggaaggccc agaccagagt atgttgccat ctttggtaat 1400

gctgacccag cccagaaagg agggtggacc aggatcctca acaggcactg 1450

cagcatttca gctataaact gtacttcctg ctgtctcata ccagtttccc 1500

tggagatcca ggtattgtgg gcatatgtag gtctcctgtc caacccgcaa 1550

gctcatgtat caggagttcg attcctatac cagtgccagt ctatacagga 1600

ttctcagcaa gttacagaag tatctttgac aactcttgtg aactttgtgg 1650

acattaccca gaagccacag cctccaaggg gccaacccaa aatggactgg 1700

aaatggccat tcgacttctt tcccttcaaa gtggcattca gcagaggagt 1750

attctctcaa aaatgctcag tctctcccat ccttatcctg tgcctcttac 1800

tacttggagt tctcaaccta gagactatgt gaagaaaaga aaataatcag 1850

atttcagttt tccctatgag aaactctgag gcagccactt atcttggcta 1900

aatagaacct cacctgctca tgaccagaga gcatttagga taatagatga 1950

cctaactgaa ggaatccttg tatatgaaag gagttatttt agaaaagcaa 2000

taaaaatatt ttattcatcn taaaaaaaaa a 2031

<210> 102
<211> 607
<212> PRT
<213> Homo Sapien

<400> 102

```
Met Arg Thr Pro Gln Leu Ala Leu Leu Gln Val Phe Phe Leu Val
  1               5                  10                  15

Phe Pro Asp Gly Val Arg Pro Gln Pro Ser Ser Ser Pro Ser Gly
                 20                  25                  30

Ala Val Pro Thr Ser Leu Glu Leu Gln Arg Gly Thr Asp Gly Gly
                 35                  40                  45

Thr Leu Gln Ser Pro Ser Glu Ala Thr Ala Thr Arg Pro Ala Val
                 50                  55                  60

Pro Gly Leu Pro Thr Val Val Pro Thr Leu Val Thr Pro Ser Ala
                 65                  70                  75

Pro Gly Asn Arg Thr Val Asp Leu Phe Pro Val Leu Pro Ile Cys
                 80                  85                  90

Val Cys Asp Leu Thr Pro Gly Ala Cys Asp Ile Asn Cys Cys Cys
                 95                 100                 105

Asp Arg Asp Cys Tyr Leu Leu His Pro Arg Thr Val Phe Ser Phe
                110                 115                 120

Cys Leu Pro Gly Ser Val Arg Ser Ser Ser Trp Val Cys Val Asp
                125                 130                 135

Asn Ser Val Ile Phe Arg Ser Asn Ser Pro Phe Pro Ser Arg Val
```

```
                140                 145                 150

Phe Met Asp Ser Asn Gly Ile Arg Gln Phe Cys Val His Val Asn
                155                 160                 165

Asn Ser Asn Leu Asn Tyr Phe Gln Lys Leu Gln Lys Val Asn Ala
                170                 175                 180

Thr Asn Phe Gln Ala Leu Ala Ala Glu Phe Gly Gly Glu Ser Phe
                185                 190                 195

Thr Ser Thr Phe Gln Thr Gln Ser Pro Pro Ser Phe Tyr Arg Ala
                200                 205                 210

Gly Asp Pro Ile Leu Thr Tyr Phe Pro Lys Trp Ser Val Ile Ser
                215                 220                 225

Leu Leu Arg Gln Pro Ala Gly Val Gly Ala Gly Gly Leu Cys Ala
                230                 235                 240

Glu Ser Asn Pro Ala Gly Phe Leu Glu Ser Lys Ser Thr Thr Cys
                245                 250                 255

Thr Arg Phe Phe Lys Asn Leu Ala Ser Ser Cys Thr Leu Asp Ser
                260                 265                 270

Ala Leu Asn Ala Ala Ser Tyr Tyr Asn Phe Thr Val Leu Lys Val
                275                 280                 285

Pro Arg Ser Met Thr Asp Pro Gln Asn Met Glu Phe Gln Val Pro
                290                 295                 300

Val Ile Leu Thr Ser Gln Ala Asn Ala Pro Leu Leu Ala Gly Asn
                305                 310                 315

Thr Cys Gln Asn Val Val Ser Gln Val Thr Tyr Glu Ile Glu Thr
                320                 325                 330

Asn Gly Thr Phe Gly Ile Gln Lys Val Ser Val Ser Leu Gly Gln
                335                 340                 345

Thr Asn Leu Thr Val Glu Pro Gly Ala Ser Leu Gln Gln His Phe
                350                 355                 360

Ile Leu Arg Phe Arg Ala Phe Gln Gln Ser Thr Ala Ala Ser Leu
                365                 370                 375

Thr Ser Pro Arg Ser Gly Asn Pro Gly Tyr Ile Val Gly Lys Pro
                380                 385                 390

Leu Leu Ala Leu Thr Asp Asp Ile Ser Tyr Ser Met Thr Leu Leu
                395                 400                 405

Gln Ser Gln Gly Asn Gly Ser Cys Ser Val Lys Arg His Glu Val
                410                 415                 420

Gln Phe Gly Val Asn Ala Ile Ser Gly Cys Lys Leu Arg Leu Lys
                425                 430                 435

Lys Ala Asp Cys Ser His Leu Gln Gln Glu Ile Tyr Gln Thr Leu
                440                 445                 450

His Gly Arg Pro Arg Pro Glu Tyr Val Ala Ile Phe Gly Asn Ala
                455                 460                 465
```

Asp Pro Ala Gln Lys Gly Gly Trp Thr Arg Ile Leu Asn Arg His
470 475 480

Cys Ser Ile Ser Ala Ile Asn Cys Thr Ser Cys Cys Leu Ile Pro
485 490 495

Val Ser Leu Glu Ile Gln Val Leu Trp Ala Tyr Val Gly Leu Leu
500 505 510

Ser Asn Pro Gln Ala His Val Ser Gly Val Arg Phe Leu Tyr Gln
515 520 525

Cys Gln Ser Ile Gln Asp Ser Gln Gln Val Thr Glu Val Ser Leu
530 535 540

Thr Thr Leu Val Asn Phe Val Asp Ile Thr Gln Lys Pro Gln Pro
545 550 555

Pro Arg Gly Gln Pro Lys Met Asp Trp Lys Trp Pro Phe Asp Phe
560 565 570

Phe Pro Phe Lys Val Ala Phe Ser Arg Gly Val Phe Ser Gln Lys
575 580 585

Cys Ser Val Ser Pro Ile Leu Ile Leu Cys Leu Leu Leu Leu Gly
590 595 600

Val Leu Asn Leu Glu Thr Met
605

<210> 103
<211> 2099
<212> DNA
<213> Homo Sapien

<400> 103
cctaattctc aaggtgatgc tatttaggaa gtcataactc atgtgagtgg 50

agccatgtgg gattaagaag tgataggaga gcttgctgtc tgtctctgct 100

ctccactgtg tgaggataca acaggaagac agccatctgg tgaggaagag 150

agggccctcg ccagataccg gacctgctga caccttgatc ttggacttcc 200

catcttccag gaaggcctga cctcagttgt tccagggtaa agaatttggg 250

cagtgcccac acccacgctg ttggataaca tttcttcacc ataccagtga 300

gggtgaatgt gtacacgccc agcttcctgc ctgttactct ccacagtatg 350

cgaagaatat ccctgacttc tagccctgtg cgccttcttt tgtttctgct 400

gttgctacta atagccttgg agatcatggt tggtggtcac tctctttgct 450

tcaacttcac tataaaatca ttgtccagac ctggacagcc ctggtgtgaa 500

gcgcaggtct tcttgaataa aaatcttttc cttcagtaca acagtgacaa 550

caacatggtc aaacctctgg gcctcctggg gaagaaggta tatgccacca 600

gcacttgggg agaattgacc caaacgctgg gagaagtggg gcgagacctc 650

aggatgctcc tttgtgacat caaaccccag ataaagacca gtgatccttc 700

cactctgcaa gtcgagatgt tttgtcaacg tgaagcagaa cggtgcactg 750
gtgcatcctg gcagttcgcc accaatggag agaaatccct cctctttgac 800
gcaatgaaca tgacctggac agtaattaat catgaagcca gtaagatcaa 850
ggagacatgg aagaaagaca gagggctgga aaagtatttc aggaagctct 900
caaagggaga ctgcgatcac tggctcaggg aattcttagg gcactgggag 950
gcaatgccag aaccgacagg cagaagatcc acctagaggt gataccacgg 1000
cggcgcagag ttgttcacct gtggtcctcg atcgctgaca gccttggctc 1050
ccactgctgt gtgttccctg agtcaagtgg aggcggagcc tgcaatgagc 1100
ggagatcgcg cctctgcatt ccagtcttgg caacagagca agactccgtc 1150
tcaaaaaaaa aaaatttttt ttcagtacat attttttaaa agatagggct 1200
gggcacagca gctcacatct ataatcccaa cactttggga ggcctaggca 1250
ggaggatcac ttgagcccag gaatctgaag ctgcagtgag cctttgctcg 1300
tgagattgtg gacctatgat cctaccacca gcccacctgg ttctaacacc 1350
ccctcctcta tgtgtgagag ggagagaaga aaagtgaggg agaaaagaga 1400
gataagcaaa gaacagagag gaaaaatgga aaataagagg aaattggggg 1450
aattaaacag aggggagggc atggatcccc gggagttaga agagtagcag 1500
cttgtggatt actacgcagt ggaggaagaa gagttgttgg aaattatttg 1550
agaggtagta taatcatttg tgaggcagtt ttctgcattc accatttctc 1600
acagactaag ttactcataa gcaaacgtgc aattcacatt acactgaaat 1650
tcttccctaa tacatcattt gcattggaat aaagtacggt tttcaaacaa 1700
cctgatatag cagaactgac tgtataaatt atgtgagcac agtgcaagta 1750
attctttgtt tgtttgtttg ttttttgag acagagtctc actctatctc 1800
ccaggctgga gtgtagtggt gcgatcccgg ctcactgcaa cctcgatctc 1850
ccaggctcaa gcgattcccc tgcctcagcc tcctgagtag ctgggattac 1900
aggcatgagc caccacgccc ggctaatttt tgtattttta gtagagacgg 1950
ggtttcaccc tgttggccag gctggtctcg aactacggac ctcaggtgat 2000
ctgccccccct cagcctctca aagtgctggg attatagcat gagccactga 2050
gcccagacac aagtagttct ttctgataaa cactttaaca ctgaatgca 2099

<210> 104
<211> 212
<212> PRT
<213> Homo Sapien

<400> 104
Met Arg Arg Ile Ser Leu Thr Ser Ser Pro Val Arg Leu Leu Leu

```
1               5                   10                  15

Phe Leu Leu Leu Leu Leu Ile Ala Leu Glu Ile Met Val Gly Gly
                20                  25                  30

His Ser Leu Cys Phe Asn Phe Thr Ile Lys Ser Leu Ser Arg Pro
                35                  40                  45

Gly Gln Pro Trp Cys Glu Ala Gln Val Phe Leu Asn Lys Asn Leu
                50                  55                  60

Phe Leu Gln Tyr Asn Ser Asp Asn Asn Met Val Lys Pro Leu Gly
                65                  70                  75

Leu Leu Gly Lys Lys Val Tyr Ala Thr Ser Thr Trp Gly Glu Leu
                80                  85                  90

Thr Gln Thr Leu Gly Glu Val Gly Arg Asp Leu Arg Met Leu Leu
                95                  100                 105

Cys Asp Ile Lys Pro Gln Ile Lys Thr Ser Asp Pro Ser Thr Leu
                110                 115                 120

Gln Val Glu Met Phe Cys Gln Arg Glu Ala Glu Arg Cys Thr Gly
                125                 130                 135

Ala Ser Trp Gln Phe Ala Thr Asn Gly Glu Lys Ser Leu Leu Phe
                140                 145                 150

Asp Ala Met Asn Met Thr Trp Thr Val Ile Asn His Glu Ala Ser
                155                 160                 165

Lys Ile Lys Glu Thr Trp Lys Lys Asp Arg Gly Leu Glu Lys Tyr
                170                 175                 180

Phe Arg Lys Leu Ser Lys Gly Asp Cys Asp His Trp Leu Arg Glu
                185                 190                 195

Phe Leu Gly His Trp Glu Ala Met Pro Glu Pro Thr Gly Arg Arg
                200                 205                 210

Ser Thr
```

<210> 105
<211> 1975
<212> DNA
<213> Homo Sapien

<400> 105

```
ttttccgagt gaccttcttg atgctggctg tttctctcac cgttcccctg 50

cttggagcca tgatgctgct ggaatctcct atagatccac agcctctcag 100

cttcaaagaa cccccgctct tgcttggtgt tctgcatcca aatacgaagc 150

tgcgacaggc agaaaggctg tttgaaaatc aacttgttgg accggagtcc 200

atagcacata ttggggatgt gatgtttact gggacagcag atggccgggt 250

cgtaaaactt gaaaatggtg aaatagagac cattgcccgg tttggttcgg 300

gcccttgcaa aacccgagat gatgagcctg tgtgtgggag acccctgggt 350
```

atccgtgcag ggcccaatgg gactctcttt gtggccgatg catacaaggg 400
actatttgaa gtaaatccct ggaaacgtga agtgaaactg ctgctgtcct 450
ccgagacacc cattgagggg aagaacatgt cctttgtgaa tgatcttaca 500
gtcactcagg atgggaggaa gatttatttc accgattcta gcagcaaatg 550
gcaaagacga gactacctgc ttctggtgat ggagggcaca gatgacgggc 600
gcctgctgga gtatgatact gtgaccaggg aagtaaaagt tttattggac 650
cagctgcggt tcccgaatgg agtccagctg tctcctgcag aagactttgt 700
cctggtggca gaaacaacca tggccaggat acgaagagtc tacgtttctg 750
gcctgatgaa gggcggggct gatctgtttg tggagaacat gcctggattt 800
ccagacaaca tccggcccag cagctctggg ggtactgggg tgggcatgtc 850
gaccatccgc cctaaccctg ggttttccat gctggatttc ttatctgaga 900
gaccctggat taaaaggatg atttttaagc tctttagtca agagacggtg 950
atgaagtttg tgccgcggta cagcctcgtc ctagaactca gcgacagcgg 1000
tgccttccgg agaagcctgc atgatcccga tgggctggtg gccacctaca 1050
tcagcgaggt gcacgaacac gatgggcacc tgtacctggg ctctttcagg 1100
tccccttcc tctgcagact cagcctccag gctgtttagc cctcccagat 1150
agctgcccct gccacgcagg ccaggagtct tcacactcag gcaccaggcc 1200
tggtccagga ggagctgtgg acacagtcgt ggttcaagtg tccacatgca 1250
cctgttagtc cctgagaggt ggtgggaatg gctgcttcat tcctcgagga 1300
tgcccgggcc ccacctgggc ttgtctttct gtttagaggg aagtgtaaca 1350
tatctgccat gaggaacata aattcatgta aagccatttt ctcttaaaca 1400
aaacaaaact ttctaagtac aatcattctc taggatttgg gaagctcctt 1450
gcacttggaa cagggctcag gtgggtggag cagtaaggca ctacccagag 1500
agcttgctgc tgcggccctg tcctgcggcc tcaaagttct tctttactat 1550
atataacgtg cggtcatacc tttcttcgtt gtggtgggga tggaagagca 1600
gagggagcat ggcccagggg tgttgaggcc agcggtgaga gccgtgttag 1650
ccaagacatg gaactgtgtt ctcaagggtt atgtggggcg tgggctctcc 1700
atagtgtgta tgaaaagctt gttgactcta gcggctcaga gaggactttg 1750
ctgggtttct ttctgtgaat atctccgtgc tgaccatgct ggaattggat 1800
gattctgcaa ttcgggacct actgcagggg tccgtttagt aacgtcttgt 1850
ctgtgatctt tgttcttgac ctctagaccc caagatgtga acagtgcacg 1900
tgttaatgtc atctttgctc atgtgttata agccccaagt tgctgtatat 1950

tttcacaagt atgtctacac actgg 1975

<210> 106
<211> 372
<212> PRT
<213> Homo Sapien

<400> 106
Met Leu Ala Val Ser Leu Thr Val Pro Leu Leu Gly Ala Met Met
1 5 10 15

Leu Leu Glu Ser Pro Ile Asp Pro Gln Pro Leu Ser Phe Lys Glu
20 25 30

Pro Pro Leu Leu Leu Gly Val Leu His Pro Asn Thr Lys Leu Arg
35 40 45

Gln Ala Glu Arg Leu Phe Glu Asn Gln Leu Val Gly Pro Glu Ser
50 55 60

Ile Ala His Ile Gly Asp Val Met Phe Thr Gly Thr Ala Asp Gly
65 70 75

Arg Val Val Lys Leu Glu Asn Gly Glu Ile Glu Thr Ile Ala Arg
80 85 90

Phe Gly Ser Gly Pro Cys Lys Thr Arg Asp Asp Glu Pro Val Cys
95 100 105

Gly Arg Pro Leu Gly Ile Arg Ala Gly Pro Asn Gly Thr Leu Phe
110 115 120

Val Ala Asp Ala Tyr Lys Gly Leu Phe Glu Val Asn Pro Trp Lys
125 130 135

Arg Glu Val Lys Leu Leu Leu Ser Ser Glu Thr Pro Ile Glu Gly
140 145 150

Lys Asn Met Ser Phe Val Asn Asp Leu Thr Val Thr Gln Asp Gly
155 160 165

Arg Lys Ile Tyr Phe Thr Asp Ser Ser Ser Lys Trp Gln Arg Arg
170 175 180

Asp Tyr Leu Leu Leu Val Met Glu Gly Thr Asp Asp Gly Arg Leu
185 190 195

Leu Glu Tyr Asp Thr Val Thr Arg Glu Val Lys Val Leu Leu Asp
200 205 210

Gln Leu Arg Phe Pro Asn Gly Val Gln Leu Ser Pro Ala Glu Asp
215 220 225

Phe Val Leu Val Ala Glu Thr Thr Met Ala Arg Ile Arg Arg Val
230 235 240

Tyr Val Ser Gly Leu Met Lys Gly Gly Ala Asp Leu Phe Val Glu
245 250 255

Asn Met Pro Gly Phe Pro Asp Asn Ile Arg Pro Ser Ser Ser Gly
260 265 270

Gly Tyr Trp Val Gly Met Ser Thr Ile Arg Pro Asn Pro Gly Phe
275 280 285

Ser Met Leu Asp Phe Leu Ser Glu Arg Pro Trp Ile Lys Arg Met
290 295 300

Ile Phe Lys Leu Phe Ser Gln Glu Thr Val Met Lys Phe Val Pro
305 310 315

Arg Tyr Ser Leu Val Leu Glu Leu Ser Asp Ser Gly Ala Phe Arg
320 325 330

Arg Ser Leu His Asp Pro Asp Gly Leu Val Ala Thr Tyr Ile Ser
335 340 345

Glu Val His Glu His Asp Gly His Leu Tyr Leu Gly Ser Phe Arg
350 355 360

Ser Pro Phe Leu Cys Arg Leu Ser Leu Gln Ala Val
365 370

<210> 107
<211> 2343
<212> DNA
<213> Homo Sapien

<400> 107
aacgaagcgt gcgcgctttg gtaaccggct agaaatcccg cacgcgcgcc 50

tgcctcctct ccccaggcct gagctgcccc tcccactgcc tttccttctt 100

cccgcgagtc agaagcttcg cgagggccca gagaggcggt ggggtgggcg 150

accctacgcc agctccgggc gggagaaagc ccaccctctc ccgcgcccca 200

ggaaaccgcc ggcgttcggc gctgcgcaga gccatggaat tctcctggct 250

ggagacgcgc tgggcgcggc ccttttacct ggcgttcgtg ttctgcctgg 300

ccctggggct gctgcaggcc attaagctgt acctgcggag gcagcggctg 350

ctgcgggacc tgcgcccctt cccagcgccc cccacccact ggttccttgg 400

gcaccagaag tttattcagg atgataacat ggagaagctt gaggaaatta 450

ttgaaaaata ccctcgtgcc ttccctttct ggattgggcc ctttcaggca 500

tttttctgta tctatgaccc agactatgca aagacacttc tgagcagaac 550

agatcccaag tcccagtacc tgcagaaatt ctcacctcca cttcttggaa 600

aaggactagc ggctctagac ggacccaagt ggttccagca tcgtcgccta 650

ctaactcctg gattccattt taacatcctg aaagcataca ttgaggtgat 700

ggctcattct gtgaaaatga tgctggataa gtgggagaag atttgcagca 750

ctcaggacac aagcgtggag gtctatgagc acatcaactc gatgtctctg 800

gatataatca tgaaatgcgc tttcagcaag gagaccaact gccagacaaa 850

cagcacccat gatccttatg caaaagccat atttgaactc agcaaaatca 900

tatttcaccg cttgtacagt ttgttgtatc acagtgacat aattttcaaa 950

ctcagccctc agggctaccg cttccagaag ttaagccgag tgttgaatca 1000

gtacacagat acaataatcc aggaaagaaa gaaatccctc caggctgggg 1050

taaagcagga taacactccg aagaggaagt accaggattt tctggatatt 1100

gtcctttctg ccaaggatga aagtggtagc agcttctcag atattgatgt 1150

acactctgaa gtgagcacat tcctgttggc aggacatgac accttggcag 1200

caagcatctc ctggatcctt tactgcctgg ctctgaaccc tgagcatcaa 1250

gagagatgcc gggaggaggt caggggcatc ctgggggatg ggtcttctat 1300

cacttgggac cagctgggtg agatgtcgta caccacaatg tgcatcaagg 1350

agacgtgccg attgattcct gcagtcccgt ccatttccag agatctcagc 1400

aagccactta ccttcccaga tggatgcaca ttgcctgcag ggatcaccgt 1450

ggttcttagt atttggggtc ttcaccacaa ccctgctgtc tggaaaaacc 1500

caaaggtctt tgaccccttg aggttctctc aggagaattc tgatcagaga 1550

cacccctatg cctacttacc attctcagct ggatcaagga actgcattgg 1600

gcaggagttt gccatgattg agttaaaggt aaccattgcc ttgattctgc 1650

tccacttcag agtgactcca gaccccacca ggcctcttac tttccccaac 1700

cattttatcc tcaagcccaa gaatgggatg tatttgcacc tgaagaaact 1750

ctctgaatgt tagatctcag ggtacaatga ttaaacgtac tttgtttttc 1800

gaagttaaat ttacagctaa tgatccaagc agatagaaag ggatcaatgt 1850

atggtgggag gattggaggt tggtgggata ggggtctctg tgaagagatc 1900

caaaatcatt tctaggtaca cagtgtgtca gctagatctg tttctatata 1950

actttgggag attttcagat cttttctgtt aaactttcac tactattaat 2000

gctgtataca ccaatagact ttcatatatt ttctgttgtt tttaaaatag 2050

ttttcagaat tatgcaagta ataagtgcat gtatgctcac tgtcaaaaat 2100

tcccaacact agaaaatcat gtagaataaa aattttaaat ctcacttcac 2150

ttagccgaca ttccatgccc tgaccaatcc tactgctttt cctaaaaaca 2200

gaataatttg gtgtgcattc tttcagactt tttcctatac attttatatg 2250

tagaaatgta gcaatgtatt tgtatagatg tgatcattcc tatattgtta 2300

ttgatttttt tcacttaata aaaattcacc ttattcctta aaa 2343

<210> 108
<211> 509
<212> PRT
<213> Homo Sapien

<400> 108
Met Glu Phe Ser Trp Leu Glu Thr Arg Trp Ala Arg Pro Phe Tyr
1               5                   10                  15

Leu Ala Phe Val Phe Cys Leu Ala Leu Gly Leu Leu Gln Ala Ile
20 25 30

Lys Leu Tyr Leu Arg Arg Gln Arg Leu Leu Arg Asp Leu Arg Pro
35 40 45

Phe Pro Ala Pro Pro Thr His Trp Phe Leu Gly His Gln Lys Phe
50 55 60

Ile Gln Asp Asp Asn Met Glu Lys Leu Glu Glu Ile Ile Glu Lys
65 70 75

Tyr Pro Arg Ala Phe Pro Phe Trp Ile Gly Pro Phe Gln Ala Phe
80 85 90

Phe Cys Ile Tyr Asp Pro Asp Tyr Ala Lys Thr Leu Leu Ser Arg
95 100 105

Thr Asp Pro Lys Ser Gln Tyr Leu Gln Lys Phe Ser Pro Pro Leu
110 115 120

Leu Gly Lys Gly Leu Ala Ala Leu Asp Gly Pro Lys Trp Phe Gln
125 130 135

His Arg Arg Leu Leu Thr Pro Gly Phe His Phe Asn Ile Leu Lys
140 145 150

Ala Tyr Ile Glu Val Met Ala His Ser Val Lys Met Met Leu Asp
155 160 165

Lys Trp Glu Lys Ile Cys Ser Thr Gln Asp Thr Ser Val Glu Val
170 175 180

Tyr Glu His Ile Asn Ser Met Ser Leu Asp Ile Ile Met Lys Cys
185 190 195

Ala Phe Ser Lys Glu Thr Asn Cys Gln Thr Asn Ser Thr His Asp
200 205 210

Pro Tyr Ala Lys Ala Ile Phe Glu Leu Ser Lys Ile Ile Phe His
215 220 225

Arg Leu Tyr Ser Leu Leu Tyr His Ser Asp Ile Ile Phe Lys Leu
230 235 240

Ser Pro Gln Gly Tyr Arg Phe Gln Lys Leu Ser Arg Val Leu Asn
245 250 255

Gln Tyr Thr Asp Thr Ile Ile Gln Glu Arg Lys Lys Ser Leu Gln
260 265 270

Ala Gly Val Lys Gln Asp Asn Thr Pro Lys Arg Lys Tyr Gln Asp
275 280 285

Phe Leu Asp Ile Val Leu Ser Ala Lys Asp Glu Ser Gly Ser Ser
290 295 300

Phe Ser Asp Ile Asp Val His Ser Glu Val Ser Thr Phe Leu Leu
305 310 315

Ala Gly His Asp Thr Leu Ala Ala Ser Ile Ser Trp Ile Leu Tyr
320 325 330

Cys Leu Ala Leu Asn Pro Glu His Gln Glu Arg Cys Arg Glu Glu

335 340 345

Val Arg Gly Ile Leu Gly Asp Gly Ser Ser Ile Thr Trp Asp Gln
350 355 360

Leu Gly Glu Met Ser Tyr Thr Thr Met Cys Ile Lys Glu Thr Cys
365 370 375

Arg Leu Ile Pro Ala Val Pro Ser Ile Ser Arg Asp Leu Ser Lys
380 385 390

Pro Leu Thr Phe Pro Asp Gly Cys Thr Leu Pro Ala Gly Ile Thr
395 400 405

Val Val Leu Ser Ile Trp Gly Leu His His Asn Pro Ala Val Trp
410 415 420

Lys Asn Pro Lys Val Phe Asp Pro Leu Arg Phe Ser Gln Glu Asn
425 430 435

Ser Asp Gln Arg His Pro Tyr Ala Tyr Leu Pro Phe Ser Ala Gly
440 445 450

Ser Arg Asn Cys Ile Gly Gln Glu Phe Ala Met Ile Glu Leu Lys
455 460 465

Val Thr Ile Ala Leu Ile Leu Leu His Phe Arg Val Thr Pro Asp
470 475 480

Pro Thr Arg Pro Leu Thr Phe Pro Asn His Phe Ile Leu Lys Pro
485 490 495

Lys Asn Gly Met Tyr Leu His Leu Lys Lys Leu Ser Glu Cys
500 505

<210> 109
<211> 1113
<212> DNA
<213> Homo Sapien

<400> 109
ggcgttccgg gcctcaactt tggcgtcgtg agattcttgt gaggcgtctg 50

cctggaagcc ggcagcaatt ttgcttcttt aaagagaaaa agaaggctag 100

ggactcagat tcctggattc tgagatccag accagctcct cccagacctc 150

tccagaagaa gccatgggaa cccctcgtat ccagcatttg ctgatcctcc 200

tggtcctagg agcctccctc ctgacctcgg gcctagagct gtattgtcaa 250

aagggtctgt ccatgactgt ggaagcagat ccagccaata tgtttaactg 300

gaccacagag gaagtggaga cttgtgacaa aggggcactt tgccaggaaa 350

ccatactaat aattaaagca gggactgaga cagccatttt ggccacgaag 400

ggctgcatcc cggaagggga ggaggccata acaattgtcc agcactcttc 450

acctcccggc ctgatcgtga cctcctacag taactactgt gaggattcct 500

tctgtaatga caaagacagc ctgtctcagt tttgggagtt cagtgagacc 550

acagcttcca ctgtgtcaac aaccctccat tgtccaacct gtgtggcttt 600

ggggacctgt ttcagtgctc cttctcttcc ctgtcccaat ggtacaactc 650

gatgctatca aggaaaactt gagatcactg gaggtggcat tgagtcgtct 700

gtggaggtca aaggctgtac agccatgatt ggctgcaggc tgatgtctgg 750

aatcttagca gtaggaccca tgtttgtgag ggaagcgtgc ccacatcagc 800

tgctcactca acctcgaaag actgaaaatg gggccacctg tcttcccatt 850

cctgtttggg ggttacagct actgctgcca ttgctgctgc catcatttat 900

tcacttttcc taagaaggca cttctgggcc tgggtctgag gacatctttt 950

ttgactggga gccttcttac tgttgaggtt caacaagctg aggagtagat 1000

gggaatttga gggagaatac agagatacta tgaacgtatt tgacattttt 1050

aatacaattt ctgctataat ttttgtatgc agtaggcgtt actaataaac 1100

atttctgctg tga 1113

<210> 110
<211> 249
<212> PRT
<213> Homo Sapien

<400> 110
Met Gly Thr Pro Arg Ile Gln His Leu Leu Ile Leu Leu Val Leu
1               5                   10                  15

Gly Ala Ser Leu Leu Thr Ser Gly Leu Glu Leu Tyr Cys Gln Lys
                20                  25                  30

Gly Leu Ser Met Thr Val Glu Ala Asp Pro Ala Asn Met Phe Asn
                35                  40                  45

Trp Thr Thr Glu Glu Val Glu Thr Cys Asp Lys Gly Ala Leu Cys
                50                  55                  60

Gln Glu Thr Ile Leu Ile Ile Lys Ala Gly Thr Glu Thr Ala Ile
                65                  70                  75

Leu Ala Thr Lys Gly Cys Ile Pro Glu Gly Glu Glu Ala Ile Thr
                80                  85                  90

Ile Val Gln His Ser Ser Pro Pro Gly Leu Ile Val Thr Ser Tyr
                95                  100                 105

Ser Asn Tyr Cys Glu Asp Ser Phe Cys Asn Asp Lys Asp Ser Leu
                110                 115                 120

Ser Gln Phe Trp Glu Phe Ser Glu Thr Thr Ala Ser Thr Val Ser
                125                 130                 135

Thr Thr Leu His Cys Pro Thr Cys Val Ala Leu Gly Thr Cys Phe
                140                 145                 150

Ser Ala Pro Ser Leu Pro Cys Pro Asn Gly Thr Thr Arg Cys Tyr
                155                 160                 165

Gln Gly Lys Leu Glu Ile Thr Gly Gly Gly Ile Glu Ser Ser Val
                170                 175                 180

Glu Val Lys Gly Cys Thr Ala Met Ile Gly Cys Arg Leu Met Ser
185 190 195

Gly Ile Leu Ala Val Gly Pro Met Phe Val Arg Glu Ala Cys Pro
200 205 210

His Gln Leu Leu Thr Gln Pro Arg Lys Thr Glu Asn Gly Ala Thr
215 220 225

Cys Leu Pro Ile Pro Val Trp Gly Leu Gln Leu Leu Leu Pro Leu
230 235 240

Leu Leu Pro Ser Phe Ile His Phe Ser
245

<210> 111
<211> 3162
<212> DNA
<213> Homo Sapien

<400> 111
cgagaagagg acagaggaga ctgagcaaag gggggtgggc tccaggcgac 50

ccctagccca attctgcccc tccatcccaa ggggcagaga aattgtcttt 100

ctttgctgac tcctacgagg aaaaaaaaaa aaaaaaaaaa aaccatttaa 150

agggaaagat aaacggagac ggaggaaagg tggcagccag attacttaga 200

gaggcacaga ggagagagat cggggtgagt cgccatgggg actcccaggg 250

cccagcaccc gccgcctccc cagctgctgt tcctaattct gctgagctgt 300

ccctggatcc agggtctgcc cctgaaggag gaggagatat tgccagagcc 350

tggaagtgag acccccacgg tggcctctga ggccctggct gaactgcttc 400

atggggccct gctgaggagg ggcccagaga tgggctacct gccaggatct 450

gatccggacc ccacgctagc cacccctccg gccggccaga ctctcgcagt 500

gccctccctg ccacgggcca ctgagccggg gacagggcct ctgacaacag 550

ccgtcacccc taacggggtc agggggggcag gccccactgc gccagaactg 600

ctgaccccgc ccccaggaac cacagcccca cccccaccca gccctgcctc 650

cccagggcct cccccttgggc ctgagggagg agaggaggag acgacgacca 700

ccatcatcac cacgacaact gttaccacta cggtgaccag cccagttctg 750

tgtaataaca acatctccga gggcgaaggg tatgtggagt ctccagatct 800

ggggagcccc gtcagccgca ccctggggct cctggactgc acttacagca 850

tccatgtcta ccctggctac ggcattgaga tccaggtgca gacgctgaac 900

ctgtcacagg aagaggagct cctggtgctg gctggtgggg gatccccagg 950

cctggccccc cgactcctgg ccaactcatc catgcttgga gaaggacaag 1000

tccttcggag cccaaccaac cggctgcttc tgcacttcca gagcccacgg 1050

gtcccaaggg gcggtggctt caggatccac tatcaggcct acctcctgag 1100
ctgtggcttc cctccccggc cggcccatgg ggacgtgagt gtgacggacc 1150
tgcaccctgg gggcactgcc acctttcact gtgattcggg ctaccagctg 1200
cagggagagg agaccctcat ctgcctcaat ggcacccggc catcctggaa 1250
cggtgaaacc cccagctgca tggcatcctg tggtggcacc atccacaatg 1300
ccaccctggg ccgcatcgtg tccccagagc ctgggggagc cgtagggccc 1350
aacctcacct gccgttgggt cattgaagca gctgaggggc gccggctgca 1400
cctgcacttt gaaagggtct cgctggatga ggacaatgac cggctgatgg 1450
tgcgctcagg gggcagcccc ctatccccccg tgatctatga ttcggacatg 1500
gacgatgtcc ccgagcgggg tctcatcagt gacgcccagt ccctctacgt 1550
ggagctgctg tcagagacac ctgccaatcc cctgctgtta agccttcgat 1600
ttgaagcctt tgaggaggat cgctgcttcg ccccttcct ggcacatgga 1650
aatgtcacta ccacggaccc tgagtatcgc ccaggggcac tggcaacctt 1700
ctcgtgcctc ccaggatatg ccctggagcc ccctgggccc cccaatgcca 1750
tcgaatgtgt ggatcccaca gaaccccact ggaacgacac agagccggcc 1800
tgcaaagcca tgtgtggagg ggagctgtcg gaaccagctg gcgtggtcct 1850
ctctcccgac tggccccaga gctatagccc gggccaagac tgcgtgtggg 1900
gcgtgcacgt ccaggaagag aagcgcatct tgctccaagt tgagatattg 1950
aatgtgcggg aaggggacat gctgacgctg ttcgacgggg acggtcccag 2000
cgcccgagtc ttggcccagc tgcggggacc tcagccgcgc cgccgccttc 2050
tctcctctgg gcccgacctc acactgcagt ttcaggcacc gcccgggccc 2100
ccaaatccag gcctgggcca gggcttcgta ttgcacttca aagaggtccc 2150
gaggaacgac acgtgccccg agctgccacc tccggagtgg ggctggagaa 2200
cggcatccca cggggacctg atccggggca cggtgctcac ctaccagtgc 2250
gagcctggct acgagctgct aggctccgac attctcactt gccagtggga 2300
cctgtcttgg agcgccgcgc cgcccgcctg ccaaaagatc atgacttgtg 2350
ctgaccctgg cgagattgcc aacgggcacc gcaccgcctc ggacgccggc 2400
ttccccgttg gctcccacgt ccagtaccgc tgcctgccag ggtacagcct 2450
cgagggggca gccatgctca cctgctacag ccgggacaca ggcacaccca 2500
agtggagcga tagggtcccc aaatgcgcct tgaagtacga gccgtgcctg 2550
aacccggggg ttcccgagaa tggctaccag acgctgtaca agcaccacta 2600
ccaggcgggc gagtctctgc gcttcttctg ctatgagggc tttgagctta 2650

tcggcgaggt caccatcacc tgtgtgcccg gccacccctc ccagtggacc 2700

agccagcccc cactctgcaa agtgacccag accacagatc catcacggca 2750

gctggaaggg gggaacctgg ccctggccat cctgctgcct ctaggcttgg 2800

tcattgtcct cggcagtggc gtttacatct actacaccaa gcttcaggga 2850

aagtcccttt tcggcttctc gggctcccac tcctacagcc ccatcaccgt 2900

ggagtcggac ttcagcaacc cgctgtatga agctggggat acgcgggagt 2950

atgaagtttc catctgaacc ccaagactac agctgcagga cccaggacgc 3000

ccctcccctc ctcattcggg cagagggaaa tacgggaccc ggtctctgcc 3050

tcctggctgc cctcctccct ggctgtgtaa atagtctccc tatcccacga 3100

gggggctttg atggccctgg agatcctaca gtaaataaac cagcatcctg 3150

ccgcccaaaa aa 3162

<210> 112
<211> 910
<212> PRT
<213> Homo Sapien

<400> 112

Met Gly Thr Pro Arg Ala Gln His Pro Pro Pro Pro Gln Leu Leu
1 5 10 15

Phe Leu Ile Leu Leu Ser Cys Pro Trp Ile Gln Gly Leu Pro Leu
20 25 30

Lys Glu Glu Glu Ile Leu Pro Glu Pro Gly Ser Glu Thr Pro Thr
35 40 45

Val Ala Ser Glu Ala Leu Ala Glu Leu Leu His Gly Ala Leu Leu
50 55 60

Arg Arg Gly Pro Glu Met Gly Tyr Leu Pro Gly Ser Asp Pro Asp
65 70 75

Pro Thr Leu Ala Thr Pro Pro Ala Gly Gln Thr Leu Ala Val Pro
80 85 90

Ser Leu Pro Arg Ala Thr Glu Pro Gly Thr Gly Pro Leu Thr Thr
95 100 105

Ala Val Thr Pro Asn Gly Val Arg Gly Ala Gly Pro Thr Ala Pro
110 115 120

Glu Leu Leu Thr Pro Pro Pro Gly Thr Thr Ala Pro Pro Pro Pro
125 130 135

Ser Pro Ala Ser Pro Gly Pro Pro Leu Gly Pro Glu Gly Gly Glu
140 145 150

Glu Glu Thr Thr Thr Thr Ile Ile Thr Thr Thr Thr Val Thr Thr
155 160 165

Thr Val Thr Ser Pro Val Leu Cys Asn Asn Asn Ile Ser Glu Gly
170 175 180

Glu Gly Tyr Val Glu Ser Pro Asp Leu Gly Ser Pro Val Ser Arg
185 190 195

Thr Leu Gly Leu Leu Asp Cys Thr Tyr Ser Ile His Val Tyr Pro
200 205 210

Gly Tyr Gly Ile Glu Ile Gln Val Gln Thr Leu Asn Leu Ser Gln
215 220 225

Glu Glu Glu Leu Leu Val Leu Ala Gly Gly Gly Ser Pro Gly Leu
230 235 240

Ala Pro Arg Leu Leu Ala Asn Ser Ser Met Leu Gly Glu Gly Gln
245 250 255

Val Leu Arg Ser Pro Thr Asn Arg Leu Leu Leu His Phe Gln Ser
260 265 270

Pro Arg Val Pro Arg Gly Gly Gly Phe Arg Ile His Tyr Gln Ala
275 280 285

Tyr Leu Leu Ser Cys Gly Phe Pro Pro Arg Pro Ala His Gly Asp
290 295 300

Val Ser Val Thr Asp Leu His Pro Gly Gly Thr Ala Thr Phe His
305 310 315

Cys Asp Ser Gly Tyr Gln Leu Gln Gly Glu Glu Thr Leu Ile Cys
320 325 330

Leu Asn Gly Thr Arg Pro Ser Trp Asn Gly Glu Thr Pro Ser Cys
335 340 345

Met Ala Ser Cys Gly Gly Thr Ile His Asn Ala Thr Leu Gly Arg
350 355 360

Ile Val Ser Pro Glu Pro Gly Gly Ala Val Gly Pro Asn Leu Thr
365 370 375

Cys Arg Trp Val Ile Glu Ala Ala Glu Gly Arg Arg Leu His Leu
380 385 390

His Phe Glu Arg Val Ser Leu Asp Glu Asp Asn Asp Arg Leu Met
395 400 405

Val Arg Ser Gly Gly Ser Pro Leu Ser Pro Val Ile Tyr Asp Ser
410 415 420

Asp Met Asp Asp Val Pro Glu Arg Gly Leu Ile Ser Asp Ala Gln
425 430 435

Ser Leu Tyr Val Glu Leu Leu Ser Glu Thr Pro Ala Asn Pro Leu
440 445 450

Leu Leu Ser Leu Arg Phe Glu Ala Phe Glu Glu Asp Arg Cys Phe
455 460 465

Ala Pro Phe Leu Ala His Gly Asn Val Thr Thr Thr Asp Pro Glu
470 475 480

Tyr Arg Pro Gly Ala Leu Ala Thr Phe Ser Cys Leu Pro Gly Tyr
485 490 495

Ala Leu Glu Pro Pro Gly Pro Pro Asn Ala Ile Glu Cys Val Asp

500 505 510

Pro Thr Glu Pro His Trp Asn Asp Thr Glu Pro Ala Cys Lys Ala
515 520 525

Met Cys Gly Gly Glu Leu Ser Glu Pro Ala Gly Val Val Leu Ser
530 535 540

Pro Asp Trp Pro Gln Ser Tyr Ser Pro Gly Gln Asp Cys Val Trp
545 550 555

Gly Val His Val Gln Glu Glu Lys Arg Ile Leu Leu Gln Val Glu
560 565 570

Ile Leu Asn Val Arg Glu Gly Asp Met Leu Thr Leu Phe Asp Gly
575 580 585

Asp Gly Pro Ser Ala Arg Val Leu Ala Gln Leu Arg Gly Pro Gln
590 595 600

Pro Arg Arg Arg Leu Leu Ser Ser Gly Pro Asp Leu Thr Leu Gln
605 610 615

Phe Gln Ala Pro Pro Gly Pro Pro Asn Pro Gly Leu Gly Gln Gly
620 625 630

Phe Val Leu His Phe Lys Glu Val Pro Arg Asn Asp Thr Cys Pro
635 640 645

Glu Leu Pro Pro Pro Glu Trp Gly Trp Arg Thr Ala Ser His Gly
650 655 660

Asp Leu Ile Arg Gly Thr Val Leu Thr Tyr Gln Cys Glu Pro Gly
665 670 675

Tyr Glu Leu Leu Gly Ser Asp Ile Leu Thr Cys Gln Trp Asp Leu
680 685 690

Ser Trp Ser Ala Ala Pro Pro Ala Cys Gln Lys Ile Met Thr Cys
695 700 705

Ala Asp Pro Gly Glu Ile Ala Asn Gly His Arg Thr Ala Ser Asp
710 715 720

Ala Gly Phe Pro Val Gly Ser His Val Gln Tyr Arg Cys Leu Pro
725 730 735

Gly Tyr Ser Leu Glu Gly Ala Ala Met Leu Thr Cys Tyr Ser Arg
740 745 750

Asp Thr Gly Thr Pro Lys Trp Ser Asp Arg Val Pro Lys Cys Ala
755 760 765

Leu Lys Tyr Glu Pro Cys Leu Asn Pro Gly Val Pro Glu Asn Gly
770 775 780

Tyr Gln Thr Leu Tyr Lys His His Tyr Gln Ala Gly Glu Ser Leu
785 790 795

Arg Phe Phe Cys Tyr Glu Gly Phe Glu Leu Ile Gly Glu Val Thr
800 805 810

Ile Thr Cys Val Pro Gly His Pro Ser Gln Trp Thr Ser Gln Pro
815 820 825

```
Pro Leu Cys Lys Val Thr Gln Thr Thr Asp Pro Ser Arg Gln Leu
                830                 835                 840

Glu Gly Gly Asn Leu Ala Leu Ala Ile Leu Leu Pro Leu Gly Leu
                845                 850                 855

Val Ile Val Leu Gly Ser Gly Val Tyr Ile Tyr Tyr Thr Lys Leu
                860                 865                 870

Gln Gly Lys Ser Leu Phe Gly Phe Ser Gly Ser His Ser Tyr Ser
                875                 880                 885

Pro Ile Thr Val Glu Ser Asp Phe Ser Asn Pro Leu Tyr Glu Ala
                890                 895                 900

Gly Asp Thr Arg Glu Tyr Glu Val Ser Ile
                905                 910
```

<210> 113
<211> 3323
<212> DNA
<213> Homo Sapien

<400> 113

```
gccgcgggcg gagctgcctg ccggtcccgc gccgcgcgtc cgcactcctc 50

ggccctcggg cggtcgatgg gacggggcgc cgcggagcag gaggcggcgc 100

ccgtcggggt gctcgggccg cgcgggagcc cactgtgggg ctcgggcatg 150

gcgggccgca ggacctgagc tctcctcagg ggagcgggga ggcagctgct 200

ggccggcgat ggggacggag tggggccgtc gccgccgcgc cgagccgtga 250

gcgccgagcc accgccgccg ctacctcagc ccttcgcgaa gcgccgggca 300

gctcgggaac atggccctgg agcggctctg ctcggtcctc aaagtgttgt 350

taataacagt actggtagtg gaagggattg ccgtggccca aaaaacccaa 400

gatggacaaa atattggaat caagcatatt cctgcaaccc agtgtggcat 450

ttgggttcga accagcaatg gaggtcattt tgcttcgcca aattatcctg 500

actcatatcc accaaacaag gagtgtatct acattttgga agctgctcca 550

cgtcaaagaa tagagttgac ctttgatgaa cattattata tagaaccatc 600

atttgagtgt cggtttgatc acttggaagt tcgagatggg ccatttggtt 650

tctctcctct tatagatcgt tactgtggcg tgaaaagccc tccattaatt 700

agatcaacag ggagattcat gtggattaag tttagttctg atgaagagct 750

tgaaggactg ggatttcgag caaaatattc atttattcca gatccagact 800

ttacttacct aggaggtatt ttaaatccca ttccagattg tcagttcgag 850

ctctcgggag ctgatggaat agtgcgctct agtcaggtag aacaagagga 900

gaaaacaaaa ccaggccaag ccgttgattg catctggacc attaaagcca 950

ctccaaaagc taagatttat ttgaggttcc tagattatca aatggagcac 1000
```

tcaaatgaat gcaagagaaa cttcgttgca gtctatgatg gaagcagttc 1050
tattgaaaac ctgaaggcca agttttgcag cactgtggcc aatgatgtaa 1100
tgcttaaaac aggaattgga gtgattcgaa tgtgggcaga tgaaggtagt 1150
cggcttagca ggtttcgaat gctctttact tcctttgtgg agcctccctg 1200
cacaagcagc actttctttt gccatagcaa catgtgcatc aataattctt 1250
tagtctgtaa tggtgtccaa aattgtgcat acccttggga tgaaaatcat 1300
tgtaaagaaa agaaaaaagc aggagtattt gaacaaatca ctaagactca 1350
tggaacaatt attggcatta cttcagggat tgtcttggtc cttctcatta 1400
tttctatttt agtacaagtg aaacagcctc gaaaaaaggt catggcttgc 1450
aaaaccgctt ttaataaaac cgggttccaa gaagtgtttg atcctcctca 1500
ttatgaactg ttttcactaa gggacaaaga gatttctgca gacctggcag 1550
acttgtcgga agaattggac aactaccaga agatgcggcg ctcctccacc 1600
gcctcccgct gcatccacga ccaccactgt gggtcgcagg cctccagcgt 1650
caaacaaagc aggaccaacc tcagttccat ggaacttcct ttccgaaatg 1700
actttgcaca accacagcca atgaaaacat ttaatagcac cttcaagaaa 1750
agtagttaca ctttcaaaca gggacatgag tgccctgagc aggccctgga 1800
agaccgagta atggaggaga ttccctgtga aatttatgtc agggggcgag 1850
aagattctgc acaagcatcc atatccattg acttctaatc ttctgctaat 1900
ggtgatgtga attcttaggg tgtgtacgta cgcagcctcc agggcaccat 1950
actgtttcca gcagccaacc cttttctccc atcacaacta cgaagacctt 2000
gatttaccgt taacctattg tatggtgatg tttttattct ctcaggcagt 2050
ctatatatgt taaaccaatc aaggaactta ctctattcag tggaaacaat 2100
aatcatctct attgcttggt gtcatttata ggaagcactg ccagttaaag 2150
agcattagaa gaggtggttg gatggagcca ggctcaggct gcctcttcgt 2200
tttagcaaca agaagactgc tcttgactga taacagctct gtcaatattt 2250
tgatgccaca ataaacttga ttttttttta cattcctttt atttttcctt 2300
tctctaaatt taatttgttt tataagccta tcgttttacc atttcatttt 2350
cttacataag tacaagtggt taatgtacca catacttcag tataggcatt 2400
tgttcttgag tgtgtcaaaa tacagctagt tactgtgcca attaagaccc 2450
agttgtattt cacccatctg tttcttcttg gctaatctct gtacttctgc 2500
cttttaatta ctgggccctt attccttatt ttctgtgaga aataatagat 2550
gatatgattt attacctttc aattatattt ttctcagtta tactagaaaa 2600

tttcataatc ctgggatata tgtaccattg tcagctatga ctaaaaattt 2650

gaaaaagata aaaatttcta gcaagccttt gaagtttacc aagtatagtc 2700

acattcagtg acagcccatt cattccagta aagaatcatt tcattcactt 2750

tgggagaggc ctataattac atttatttgc aatgtttctc ttcgctagat 2800

tgttacatag ctcccattct gttggttttg cttacagcat atggtaacca 2850

aggttagatg ccagttaaaa ttccttagaa attggatgag ccttgagatt 2900

gcttcttaac tgggacatga catttttcta gctcttatca agaataacaa 2950

cttccacttt tttttaaact gcacttttga cttttttttat ggtataaaaa 3000

caataattta taaacataaa agctcattgt gtttttttaga cttttgatat 3050

tatttgatac tgtacaaact ttattaaatc aagatgaaag acctacagga 3100

cagattcctt tcagtgttca catcagtggc tttgtatgca aatatgctgt 3150

gttggacctg gacgctataa cttattgtaa agaccttgga aatgtggaca 3200

taagctcttt ctttcctttt gttactgtat ttagtttgtg ataaattttt 3250

cactgtgtga tatttatgct ctaaatcact acacaaatcc catattaaaa 3300

tatacattgt acctgaaaaa aaa 3323

<210> 114
<211> 525
<212> PRT
<213> Homo Sapien

<400> 114
Met Ala Leu Glu Arg Leu Cys Ser Val Leu Lys Val Leu Leu Ile
1 5 10 15

Thr Val Leu Val Val Glu Gly Ile Ala Val Ala Gln Lys Thr Gln
20 25 30

Asp Gly Gln Asn Ile Gly Ile Lys His Ile Pro Ala Thr Gln Cys
35 40 45

Gly Ile Trp Val Arg Thr Ser Asn Gly Gly His Phe Ala Ser Pro
50 55 60

Asn Tyr Pro Asp Ser Tyr Pro Pro Asn Lys Glu Cys Ile Tyr Ile
65 70 75

Leu Glu Ala Ala Pro Arg Gln Arg Ile Glu Leu Thr Phe Asp Glu
80 85 90

His Tyr Tyr Ile Glu Pro Ser Phe Glu Cys Arg Phe Asp His Leu
95 100 105

Glu Val Arg Asp Gly Pro Phe Gly Phe Ser Pro Leu Ile Asp Arg
110 115 120

Tyr Cys Gly Val Lys Ser Pro Pro Leu Ile Arg Ser Thr Gly Arg
125 130 135

Phe Met Trp Ile Lys Phe Ser Ser Asp Glu Glu Leu Glu Gly Leu
140 145 150

Gly Phe Arg Ala Lys Tyr Ser Phe Ile Pro Asp Pro Asp Phe Thr
155 160 165

Tyr Leu Gly Gly Ile Leu Asn Pro Ile Pro Asp Cys Gln Phe Glu
170 175 180

Leu Ser Gly Ala Asp Gly Ile Val Arg Ser Ser Gln Val Glu Gln
185 190 195

Glu Glu Lys Thr Lys Pro Gly Gln Ala Val Asp Cys Ile Trp Thr
200 205 210

Ile Lys Ala Thr Pro Lys Ala Lys Ile Tyr Leu Arg Phe Leu Asp
215 220 225

Tyr Gln Met Glu His Ser Asn Glu Cys Lys Arg Asn Phe Val Ala
230 235 240

Val Tyr Asp Gly Ser Ser Ser Ile Glu Asn Leu Lys Ala Lys Phe
245 250 255

Cys Ser Thr Val Ala Asn Asp Val Met Leu Lys Thr Gly Ile Gly
260 265 270

Val Ile Arg Met Trp Ala Asp Glu Gly Ser Arg Leu Ser Arg Phe
275 280 285

Arg Met Leu Phe Thr Ser Phe Val Glu Pro Pro Cys Thr Ser Ser
290 295 300

Thr Phe Phe Cys His Ser Asn Met Cys Ile Asn Asn Ser Leu Val
305 310 315

Cys Asn Gly Val Gln Asn Cys Ala Tyr Pro Trp Asp Glu Asn His
320 325 330

Cys Lys Glu Lys Lys Lys Ala Gly Val Phe Glu Gln Ile Thr Lys
335 340 345

Thr His Gly Thr Ile Ile Gly Ile Thr Ser Gly Ile Val Leu Val
350 355 360

Leu Leu Ile Ile Ser Ile Leu Val Gln Val Lys Gln Pro Arg Lys
365 370 375

Lys Val Met Ala Cys Lys Thr Ala Phe Asn Lys Thr Gly Phe Gln
380 385 390

Glu Val Phe Asp Pro Pro His Tyr Glu Leu Phe Ser Leu Arg Asp
395 400 405

Lys Glu Ile Ser Ala Asp Leu Ala Asp Leu Ser Glu Glu Leu Asp
410 415 420

Asn Tyr Gln Lys Met Arg Arg Ser Ser Thr Ala Ser Arg Cys Ile
425 430 435

His Asp His His Cys Gly Ser Gln Ala Ser Ser Val Lys Gln Ser
440 445 450

Arg Thr Asn Leu Ser Ser Met Glu Leu Pro Phe Arg Asn Asp Phe

455 460 465

Ala Gln Pro Gln Pro Met Lys Thr Phe Asn Ser Thr Phe Lys Lys
470 475 480

Ser Ser Tyr Thr Phe Lys Gln Gly His Glu Cys Pro Glu Gln Ala
485 490 495

Leu Glu Asp Arg Val Met Glu Glu Ile Pro Cys Glu Ile Tyr Val
500 505 510

Arg Gly Arg Glu Asp Ser Ala Gln Ala Ser Ile Ser Ile Asp Phe
515 520 525

<210> 115
<211> 2314
<212> DNA
<213> Homo Sapien

<400> 115
ggtctctgtc cttggctgtg gctcctgcgc tctggctgag ccatgttcct 50

tctcctcgcc ctcctcactg agcttggaag actgcaagcc cacgaaggtt 100

ctgaaggaat atttctgcat gtcacagttc cacggaagat taagtcaaat 150

gacagtgaag tttcagagag gaagatgatt tacatcatta caattgatgg 200

acaaccttac actctacatc tcggaaaaca atcattctta ccccagaact 250

ttttggttta tacatataat gaaactggat ctttgcattc tgtgtctcca 300

tattttatga tgcattgcca ttaccaagga tatgctgccg aatttccaaa 350

ttcatttgtg acactcagta tatgttctgg tctcagggga tttctccagt 400

ttgaaaatat cagttatgga attgaaccag tagaatcttc agcaagattt 450

gagcatataa tttatcaaat gaaaaataat gatccaaatg tatccatttt 500

agcagtaaat tacagtcata tttggcagaa agaccagccc tacaaagttc 550

ctttaaactc acagataaaa aatctttcaa aactattacc ccaatatctg 600

gaaatataca ttatagtgga aaaagctttg atgtttaccc agttcaaatt 650

gactgttata ctgtcttcct tggaattgtg gtcaaatgaa aaccagattt 700

ccaccagtgg ggatgctgat gatatattac aaagattttt ggcatggaaa 750

cgggactatc tcatcctacg gccccatgac atagcatact tacttgttta 800

caggaaacat cctaaatatg tgggagcaac atttcctggc accgtatgca 850

ataaaagcta tgatgcaggt attgctatgt atccagatgc aataggtttg 900

gagggatttt cggttattat agctcaactg cttggcctta atgtaggatt 950

aacatatgat gacatcactc agtgtttctg tctgagagct acatgcatca 1000

tgaatcatga agcagtgagt gccagtggta gaaagatttt tagcaactgc 1050

agcatgcacg actatagata ttttgtttca aaatttgaga ctaaatgcct 1100

tcagaagctt tcaaatttgc aaccattaca tcaaaatcaa ccagtgtgtg 1150
gtaatgggat tttggaatcc aatgaagaat gtgactgtgg taataaaaat 1200
gaatgtcaat ttaagaagtg ctgtgattat aacacatgta aactgaaggg 1250
ctcagtaaaa tgtggttctg gaccatgttg tacatcaaag tgtgagttgt 1300
caatagcagg cactccatgt agaaagagta ttgatccaga gtgtgatttt 1350
acagagtact gcaatggaac ctctagtaat tgtgttcctg acacttatgc 1400
actgaatggc cgtttgtgca agttgggaac tgcctattgc tataacggac 1450
aatgtcaaac tactgataac cagtgtgcca agatatttgg aaaaggtgct 1500
caaggtgctc catttgcctg ttttaaagaa gttaattctc tgcatgaaag 1550
atctgaaaac tgtggtttta aaaattcaca accattacct tgtgaacgga 1600
aggatgttct ctgtggaaaa ttagcttgtg ttcagccaca taaaaatgct 1650
aataaaagtg acgctcaatc tacagtttat tcatatattc aagaccatgt 1700
atgtgtatct atagccactg gttcctccat gagatcagat ggaacagaca 1750
atgcctatgt ggctgatggc accatgtgtg gtccagaaat gtactgtgta 1800
aataaaacct gcagaaaagt tcatttaatg ggatataact gtaatgccac 1850
cacaaaatgc aaagggaaag ggatatgtaa taattttggt aattgtcaat 1900
gcttccctgg acatagacct ccagattgta aattccagtt tggttcccca 1950
gggggtagta ttgatgatgg aaattttcag aaatctggtg acttttatac 2000
tgaaaaaggc tacaatacac actggaacaa ctggtttatt ctgagtttct 2050
gcatttttct gccgtttttc atagttttca ccactgtgat ctttaaaaga 2100
aatgaaataa gtaaatcatg taacagagag aatgcagagt ataatcgtaa 2150
ttcatccgtt gtatcagaaa gcgatgacgt gggacattaa tattgcacag 2200
aacttccata gcaaataacc taaaggaacg aatgtgcttt atttataacc 2250
ttacgttatc cccaatgcat tgtaaatgtc aaacttttgg aaaataaagc 2300
ctgcgtgccc tccc 2314

<210> 116
<211> 715
<212> PRT
<213> Homo Sapien

<400> 116
Met Phe Leu Leu Leu Ala Leu Leu Thr Glu Leu Gly Arg Leu Gln
1               5                   10                  15

Ala His Glu Gly Ser Glu Gly Ile Phe Leu His Val Thr Val Pro
                20                  25                  30

Arg Lys Ile Lys Ser Asn Asp Ser Glu Val Ser Glu Arg Lys Met
                35                  40                  45

Ile Tyr Ile Ile Thr Ile Asp Gly Gln Pro Tyr Thr Leu His Leu
50 55 60

Gly Lys Gln Ser Phe Leu Pro Gln Asn Phe Leu Val Tyr Thr Tyr
65 70 75

Asn Glu Thr Gly Ser Leu His Ser Val Ser Pro Tyr Phe Met Met
80 85 90

His Cys His Tyr Gln Gly Tyr Ala Ala Glu Phe Pro Asn Ser Phe
95 100 105

Val Thr Leu Ser Ile Cys Ser Gly Leu Arg Gly Phe Leu Gln Phe
110 115 120

Glu Asn Ile Ser Tyr Gly Ile Glu Pro Val Glu Ser Ser Ala Arg
125 130 135

Phe Glu His Ile Ile Tyr Gln Met Lys Asn Asn Asp Pro Asn Val
140 145 150

Ser Ile Leu Ala Val Asn Tyr Ser His Ile Trp Gln Lys Asp Gln
155 160 165

Pro Tyr Lys Val Pro Leu Asn Ser Gln Ile Lys Asn Leu Ser Lys
170 175 180

Leu Leu Pro Gln Tyr Leu Glu Ile Tyr Ile Ile Val Glu Lys Ala
185 190 195

Leu Met Phe Thr Gln Phe Lys Leu Thr Val Ile Leu Ser Ser Leu
200 205 210

Glu Leu Trp Ser Asn Glu Asn Gln Ile Ser Thr Ser Gly Asp Ala
215 220 225

Asp Asp Ile Leu Gln Arg Phe Leu Ala Trp Lys Arg Asp Tyr Leu
230 235 240

Ile Leu Arg Pro His Asp Ile Ala Tyr Leu Leu Val Tyr Arg Lys
245 250 255

His Pro Lys Tyr Val Gly Ala Thr Phe Pro Gly Thr Val Cys Asn
260 265 270

Lys Ser Tyr Asp Ala Gly Ile Ala Met Tyr Pro Asp Ala Ile Gly
275 280 285

Leu Glu Gly Phe Ser Val Ile Ile Ala Gln Leu Leu Gly Leu Asn
290 295 300

Val Gly Leu Thr Tyr Asp Asp Ile Thr Gln Cys Phe Cys Leu Arg
305 310 315

Ala Thr Cys Ile Met Asn His Glu Ala Val Ser Ala Ser Gly Arg
320 325 330

Lys Ile Phe Ser Asn Cys Ser Met His Asp Tyr Arg Tyr Phe Val
335 340 345

Ser Lys Phe Glu Thr Lys Cys Leu Gln Lys Leu Ser Asn Leu Gln
350 355 360

```
Pro Leu His Gln Asn Gln Pro Val Cys Gly Asn Gly Ile Leu Glu
                365                 370                 375

Ser Asn Glu Glu Cys Asp Cys Gly Asn Lys Asn Glu Cys Gln Phe
                380                 385                 390

Lys Lys Cys Cys Asp Tyr Asn Thr Cys Lys Leu Lys Gly Ser Val
                395                 400                 405

Lys Cys Gly Ser Gly Pro Cys Cys Thr Ser Lys Cys Glu Leu Ser
                410                 415                 420

Ile Ala Gly Thr Pro Cys Arg Lys Ser Ile Asp Pro Glu Cys Asp
                425                 430                 435

Phe Thr Glu Tyr Cys Asn Gly Thr Ser Ser Asn Cys Val Pro Asp
                440                 445                 450

Thr Tyr Ala Leu Asn Gly Arg Leu Cys Lys Leu Gly Thr Ala Tyr
                455                 460                 465

Cys Tyr Asn Gly Gln Cys Gln Thr Thr Asp Asn Gln Cys Ala Lys
                470                 475                 480

Ile Phe Gly Lys Gly Ala Gln Gly Ala Pro Phe Ala Cys Phe Lys
                485                 490                 495

Glu Val Asn Ser Leu His Glu Arg Ser Glu Asn Cys Gly Phe Lys
                500                 505                 510

Asn Ser Gln Pro Leu Pro Cys Glu Arg Lys Asp Val Leu Cys Gly
                515                 520                 525

Lys Leu Ala Cys Val Gln Pro His Lys Asn Ala Asn Lys Ser Asp
                530                 535                 540

Ala Gln Ser Thr Val Tyr Ser Tyr Ile Gln Asp His Val Cys Val
                545                 550                 555

Ser Ile Ala Thr Gly Ser Ser Met Arg Ser Asp Gly Thr Asp Asn
                560                 565                 570

Ala Tyr Val Ala Asp Gly Thr Met Cys Gly Pro Glu Met Tyr Cys
                575                 580                 585

Val Asn Lys Thr Cys Arg Lys Val His Leu Met Gly Tyr Asn Cys
                590                 595                 600

Asn Ala Thr Thr Lys Cys Lys Gly Lys Gly Ile Cys Asn Asn Phe
                605                 610                 615

Gly Asn Cys Gln Cys Phe Pro Gly His Arg Pro Pro Asp Cys Lys
                620                 625                 630

Phe Gln Phe Gly Ser Pro Gly Gly Ser Ile Asp Asp Gly Asn Phe
                635                 640                 645

Gln Lys Ser Gly Asp Phe Tyr Thr Glu Lys Gly Tyr Asn Thr His
                650                 655                 660

Trp Asn Asn Trp Phe Ile Leu Ser Phe Cys Ile Phe Leu Pro Phe
                665                 670                 675

Phe Ile Val Phe Thr Thr Val Ile Phe Lys Arg Asn Glu Ile Ser
```

```
                680                 685                 690

Lys Ser Cys Asn Arg Glu Asn Ala Glu Tyr Asn Arg Asn Ser Ser
                695                 700                 705

Val Val Ser Glu Ser Asp Asp Val Gly His
                710                 715
```

<210> 117
<211> 1422
<212> DNA
<213> Homo Sapien

<400> 117

```
cccacgcgtc cgcggacgcg tggggctcag tgggcgtcgc gcgaaggcta 50

agggagtgtg gcgggcggct ccgggagcca acatgcctcg gtatgcgcag 100

ctggtcatgg gccccgcggg cagcgggaag agcacctact gtgccaccat 150

ggtccagcac tgtgaagccc tcaaccggtc tgtccaagtt gtaaacctgg 200

atccagcagc agaacacttc aactactccg tgatggctga catccgggaa 250

ctgatcgagg tggatgatgt aatggaggat gattctctgc gattcggtcc 300

caacggagga ttggtatttt gcatggagta ctttgccaat aattttgact 350

ggctggagaa ctgtcttggc catgtagagg acgactatat cctttttgat 400

tgtccaggtc agattgagtt gtacactcac ctgcctgtga tgaaacatct 450

ggtccagcag ctcgagcagt gggagttccg agtctgtgga gtttttcttg 500

ttgattctca gttcatggtg gagtcattca agtttatttc tggcatcttg 550

gcagccctga gtgccatgat ctctctagaa attccgcaag tcaacatcat 600

gacaaaaatg gatctgctga gtaaaaaagc aaaaaaggaa attgagaaat 650

ttttagatcc agacatgtat tctttattag aagattctac aagtgactta 700

agaagcaaaa aattcaagaa actgactaaa gctatatgtg gactgattga 750

tgactacagc atggttcgat ttttacctta cgatcagtca gatgaagaaa 800

gcatgaacat tgtattgcag catattgatt ttgccattca atatggagaa 850

gacctagaat ttaaagaacc aaaggaacgt gaagatgagt cttcctctat 900

gtttgacgaa tattttcaag aatgccagga tgaatgaaga gtttactaaa 950

agtaaccatc taaagagctt gtggccaaac cagcagaaca ttcttctctt 1000

caaaggatgc aatagtagaa agctacttat tttaatgaaa aaaagtaaaa 1050

cttcgttctt tatcagcctc atgcctgaat caaattttta attattctga 1100

aactgctgct gtttaaagtg gaatctttta gtattataac agcatcactt 1150

tagattttgt aagtcaaaat tgaaatgaat gcacatagat ttatatataa 1200

attagcacct gagctaaggt taaggccggt ctaaacttat tttcactttt 1250
```

tgtattattt ttgagatgca ggaattactg taacaaaata tgtatgtccg 1300

aagggaaaaa gctgcaagga tatatataag accactgctt atctgtatct 1350

tcccattttc ctatattgaa aatgtatatt atttatataa cttaaaaagt 1400

aaaaataact atgttttgag at 1422

<210> 118
<211> 284
<212> PRT
<213> Homo Sapien

<400> 118
Met Pro Arg Tyr Ala Gln Leu Val Met Gly Pro Ala Gly Ser Gly
1 5 10 15

Lys Ser Thr Tyr Cys Ala Thr Met Val Gln His Cys Glu Ala Leu
20 25 30

Asn Arg Ser Val Gln Val Val Asn Leu Asp Pro Ala Ala Glu His
35 40 45

Phe Asn Tyr Ser Val Met Ala Asp Ile Arg Glu Leu Ile Glu Val
50 55 60

Asp Asp Val Met Glu Asp Asp Ser Leu Arg Phe Gly Pro Asn Gly
65 70 75

Gly Leu Val Phe Cys Met Glu Tyr Phe Ala Asn Asn Phe Asp Trp
80 85 90

Leu Glu Asn Cys Leu Gly His Val Glu Asp Asp Tyr Ile Leu Phe
95 100 105

Asp Cys Pro Gly Gln Ile Glu Leu Tyr Thr His Leu Pro Val Met
110 115 120

Lys His Leu Val Gln Gln Leu Glu Gln Trp Glu Phe Arg Val Cys
125 130 135

Gly Val Phe Leu Val Asp Ser Gln Phe Met Val Glu Ser Phe Lys
140 145 150

Phe Ile Ser Gly Ile Leu Ala Ala Leu Ser Ala Met Ile Ser Leu
155 160 165

Glu Ile Pro Gln Val Asn Ile Met Thr Lys Met Asp Leu Leu Ser
170 175 180

Lys Lys Ala Lys Lys Glu Ile Glu Lys Phe Leu Asp Pro Asp Met
185 190 195

Tyr Ser Leu Leu Glu Asp Ser Thr Ser Asp Leu Arg Ser Lys Lys
200 205 210

Phe Lys Lys Leu Thr Lys Ala Ile Cys Gly Leu Ile Asp Asp Tyr
215 220 225

Ser Met Val Arg Phe Leu Pro Tyr Asp Gln Ser Asp Glu Glu Ser
230 235 240

Met Asn Ile Val Leu Gln His Ile Asp Phe Ala Ile Gln Tyr Gly
245 250 255

Glu Asp Leu Glu Phe Lys Glu Pro Lys Glu Arg Glu Asp Glu Ser
260 265 270

Ser Ser Met Phe Asp Glu Tyr Phe Gln Glu Cys Gln Asp Glu
275 280

<210> 119
<211> 2868
<212> DNA
<213> Homo Sapien

<400> 119
gggcgctggg agacaccgga cgcccgctcg gctgcgctgc ggctcaggcc 50

cccgctcggg cccgacccgc tcggtcaccg ccggctcggg cgcgcacctg 100

ccggctgcgg ccccagggcc atgcggaggc ccacgaggag gccggcggcc 150

acgcgcatcc cgtagcccag gtggcccagg tctgcaccgc ggcggcctcg 200

gcgccatgga gcccccgtat tcgctgacgg cgcactacga tgagttccaa 250

gaggtcaagt acgtgagccg ctgcggcgcg gggggcgcgc gcggggcctc 300

cctgcccccg ggcttcccgt tgggcgctgc gcgcagcgtc accggggccc 350

ggtccgggct gccgcgctgg aaccggcgcg aggtgtgcct gctgtcgggg 400

ctggtgttcg ccgccggcct ctgcgccatt ctggcggcta tgctggccct 450

caagtacctg ggcccggtcg cggccggcgg cggcgcctgt cccgagggct 500

gccctgagcg caaggccttc gcgcgcgccg ctcgcttcct ggccgccaac 550

ctggacgcca gcatcgaccc atgccaggac ttctactcgt tcgcctgcgg 600

cggttggctg cggcgccacg ccatccccga cgacaagctc acctatggca 650

ccatcgcggc catcggcgag caaaacgagg agcgcctacg gcgcctgctg 700

gcgcggcccg gggtgggcc tggcggcgcg gcccagcgca aggtgcgcgc 750

cttcttccgc tcgtgcctcg acatgcgcga gatcgagcga ctgggcccgc 800

gacccatgct agaggtcatc gaggactgcg gggctggga cctgggcggc 850

gcggaggagc gtccgggggt cgcggcgcga tgggacctca accggctgct 900

gtacaaggcg cagggcgtgt acagcgccgc cgcgctcttc tcgctcacgg 950

tcagcctgga cgacaggaac tcctcgcgct acgtcatccg cattgaccag 1000

gatgggctca ccctgccaga gaggaccctg tacctcgctc aggatgagga 1050

cagtgagaag atcctggcag catacagggt gttcatggag cgagtgctca 1100

gcctcctggg tgcagacgct gtggaacaga aggcccaaga gatcctgcaa 1150

gtggagcagc agctggccaa catcactgtg tcagagtatg acgacctacg 1200

gcgagatgtc agctccatgt acaacaaggt gacgctgggg cagctgcaga 1250

agatcacccc ccacttgcgg tggaagtggc tgctagacca gatcttccag 1300

gaggacttct cagaggaaga ggaggtggtg ctgctggcga cagactacat 1350
gcagcaggtg tcgcagctca tccgctccac accccaccgg gtcctgcaca 1400
actacctggt gtggcgcgtg gtggtggtcc tgagtgaaca cctgtccccg 1450
ccattccgtg aggcactgca cgagctggca caggagatgg agggcagcga 1500
caagccacag gagctggccc gggtctgctt gggccaggcc aatcgccact 1550
ttggcatggc gcttggcgcc ctctttgtac atgagcactt ctcagccgcc 1600
agcaaagcca aggtgcagca gctagtggaa gacatcaagt acatcctggg 1650
ccagcgcctg gaggagctgg actggatgga cgccgagacc agggctgctg 1700
ctcgggccaa gctccagtac atgatggtga tggtcggcta cccggacttc 1750
ctgctgaaac ccgatgctgt ggacaaggag tatgagtttg aggtccatga 1800
gaagacctac ttcaagaaca tcttgaacag catccccttc agcatccagc 1850
tctcagttaa gaagattcgg caggaggtgg acaagtccac gtggctgctc 1900
cccccacagg cgctcaatgc ctactatcta cccaacaaga accagatggt 1950
gttccccgcg ggcatcctgc agcccaccct gtacgaccct gacttcccac 2000
agtctctcaa ctacgggggc atcggcacca tcattggaca tgagctgacc 2050
cacggctacg acgactgggg gggccagtat gaccgctcag ggaacctgct 2100
gcactggtgg acggaggcct cctacagccg cttcctgcga aaggctgagt 2150
gcatcgtccg tctctatgac aacttcactg tctacaacca gcgggtgaac 2200
gggaaacaca cgcttgggga gaacatcgca gatatgggcg tcctcaagct 2250
ggcctaccac gcctatcaga agtgggtgcg ggagcacggc ccagagcacc 2300
cacttccccg gctcaagtac acacatgacc agctcttctt cattgccttt 2350
gcccagaact ggtgcatcaa gcggcggtcg cagtccatct acctgcaggt 2400
gctgactgac aagcatgccc ctgagcacta cagggtgctg ggcagtgtgt 2450
cccagtttga ggagtttggc cgggctttcc actgtcccaa ggactcaccc 2500
atgaaccctg cccacaagtg ttccgtgtgg tgagcctggc tgcccgcctg 2550
cacgccccca ctgcccccgc acgaatcacc tcctgctggc taccggggca 2600
ggcatgcacc cggtgccagc cccgctctgg gcaccacctg ccttccagcc 2650
cctccaggac ccggtccccc tgctgcccct cacttcagga ggggcctgga 2700
gcagggtgag gctggacttt ggggggctgt gagggaaata tactggggtc 2750
cccagattct gctctaaggg ggccagaccc tctgccaggc tggattgtac 2800
gggccccacc ttcgctgtgt tcttgctgca aagtctggtc aataaatcac 2850
tgcactgtta aaaaaaaa 2868

<210> 120
<211> 775
<212> PRT
<213> Homo Sapien

<400> 120
```
Met Glu Pro Pro Tyr Ser Leu Thr Ala His Tyr Asp Glu Phe Gln
  1               5                  10                  15

Glu Val Lys Tyr Val Ser Arg Cys Gly Ala Gly Gly Ala Arg Gly
                 20                  25                  30

Ala Ser Leu Pro Pro Gly Phe Pro Leu Gly Ala Ala Arg Ser Val
                 35                  40                  45

Thr Gly Ala Arg Ser Gly Leu Pro Arg Trp Asn Arg Arg Glu Val
                 50                  55                  60

Cys Leu Leu Ser Gly Leu Val Phe Ala Ala Gly Leu Cys Ala Ile
                 65                  70                  75

Leu Ala Ala Met Leu Ala Leu Lys Tyr Leu Gly Pro Val Ala Ala
                 80                  85                  90

Gly Gly Gly Ala Cys Pro Glu Gly Cys Pro Glu Arg Lys Ala Phe
                 95                 100                 105

Ala Arg Ala Ala Arg Phe Leu Ala Ala Asn Leu Asp Ala Ser Ile
                110                 115                 120

Asp Pro Cys Gln Asp Phe Tyr Ser Phe Ala Cys Gly Gly Trp Leu
                125                 130                 135

Arg Arg His Ala Ile Pro Asp Asp Lys Leu Thr Tyr Gly Thr Ile
                140                 145                 150

Ala Ala Ile Gly Glu Gln Asn Glu Glu Arg Leu Arg Arg Leu Leu
                155                 160                 165

Ala Arg Pro Gly Gly Gly Pro Gly Gly Ala Ala Gln Arg Lys Val
                170                 175                 180

Arg Ala Phe Phe Arg Ser Cys Leu Asp Met Arg Glu Ile Glu Arg
                185                 190                 195

Leu Gly Pro Arg Pro Met Leu Glu Val Ile Glu Asp Cys Gly Gly
                200                 205                 210

Trp Asp Leu Gly Gly Ala Glu Glu Arg Pro Gly Val Ala Ala Arg
                215                 220                 225

Trp Asp Leu Asn Arg Leu Leu Tyr Lys Ala Gln Gly Val Tyr Ser
                230                 235                 240

Ala Ala Ala Leu Phe Ser Leu Thr Val Ser Leu Asp Asp Arg Asn
                245                 250                 255

Ser Ser Arg Tyr Val Ile Arg Ile Asp Gln Asp Gly Leu Thr Leu
                260                 265                 270

Pro Glu Arg Thr Leu Tyr Leu Ala Gln Asp Glu Asp Ser Glu Lys
                275                 280                 285
```

```
Ile Leu Ala Ala Tyr Arg Val Phe Met Glu Arg Val Leu Ser Leu
                290                 295                 300

Leu Gly Ala Asp Ala Val Glu Gln Lys Ala Gln Glu Ile Leu Gln
                305                 310                 315

Val Glu Gln Gln Leu Ala Asn Ile Thr Val Ser Glu Tyr Asp Asp
                320                 325                 330

Leu Arg Arg Asp Val Ser Ser Met Tyr Asn Lys Val Thr Leu Gly
                335                 340                 345

Gln Leu Gln Lys Ile Thr Pro His Leu Arg Trp Lys Trp Leu Leu
                350                 355                 360

Asp Gln Ile Phe Gln Glu Asp Phe Ser Glu Glu Glu Glu Val Val
                365                 370                 375

Leu Leu Ala Thr Asp Tyr Met Gln Gln Val Ser Gln Leu Ile Arg
                380                 385                 390

Ser Thr Pro His Arg Val Leu His Asn Tyr Leu Val Trp Arg Val
                395                 400                 405

Val Val Val Leu Ser Glu His Leu Ser Pro Pro Phe Arg Glu Ala
                410                 415                 420

Leu His Glu Leu Ala Gln Glu Met Glu Gly Ser Asp Lys Pro Gln
                425                 430                 435

Glu Leu Ala Arg Val Cys Leu Gly Gln Ala Asn Arg His Phe Gly
                440                 445                 450

Met Ala Leu Gly Ala Leu Phe Val His Glu His Phe Ser Ala Ala
                455                 460                 465

Ser Lys Ala Lys Val Gln Gln Leu Val Glu Asp Ile Lys Tyr Ile
                470                 475                 480

Leu Gly Gln Arg Leu Glu Glu Leu Asp Trp Met Asp Ala Glu Thr
                485                 490                 495

Arg Ala Ala Ala Arg Ala Lys Leu Gln Tyr Met Met Val Met Val
                500                 505                 510

Gly Tyr Pro Asp Phe Leu Leu Lys Pro Asp Ala Val Asp Lys Glu
                515                 520                 525

Tyr Glu Phe Glu Val His Glu Lys Thr Tyr Phe Lys Asn Ile Leu
                530                 535                 540

Asn Ser Ile Pro Phe Ser Ile Gln Leu Ser Val Lys Lys Ile Arg
                545                 550                 555

Gln Glu Val Asp Lys Ser Thr Trp Leu Leu Pro Pro Gln Ala Leu
                560                 565                 570

Asn Ala Tyr Tyr Leu Pro Asn Lys Asn Gln Met Val Phe Pro Ala
                575                 580                 585

Gly Ile Leu Gln Pro Thr Leu Tyr Asp Pro Asp Phe Pro Gln Ser
                590                 595                 600

Leu Asn Tyr Gly Gly Ile Gly Thr Ile Ile Gly His Glu Leu Thr
```

```
                605                 610                 615
His Gly Tyr Asp Asp Trp Gly Gly Gln Tyr Asp Arg Ser Gly Asn
                620                 625                 630
Leu Leu His Trp Trp Thr Glu Ala Ser Tyr Ser Arg Phe Leu Arg
                635                 640                 645
Lys Ala Glu Cys Ile Val Arg Leu Tyr Asp Asn Phe Thr Val Tyr
                650                 655                 660
Asn Gln Arg Val Asn Gly Lys His Thr Leu Gly Glu Asn Ile Ala
                665                 670                 675
Asp Met Gly Val Leu Lys Leu Ala Tyr His Ala Tyr Gln Lys Trp
                680                 685                 690
Val Arg Glu His Gly Pro Glu His Pro Leu Pro Arg Leu Lys Tyr
                695                 700                 705
Thr His Asp Gln Leu Phe Phe Ile Ala Phe Ala Gln Asn Trp Cys
                710                 715                 720
Ile Lys Arg Arg Ser Gln Ser Ile Tyr Leu Gln Val Leu Thr Asp
                725                 730                 735
Lys His Ala Pro Glu His Tyr Arg Val Leu Gly Ser Val Ser Gln
                740                 745                 750
Phe Glu Glu Phe Gly Arg Ala Phe His Cys Pro Lys Asp Ser Pro
                755                 760                 765
Met Asn Pro Ala His Lys Cys Ser Val Trp
                770                 775
```

<210> 121
<211> 1806
<212> DNA
<213> Homo Sapien

<400> 121
cggactgccc ggaccgcgcg atggagtcga ccggcagcgt cggggaggcc 50

ccgggcggac cccgggtgct ggtggtgggc ggcggcatcg cggggctggg 100

cgcggcgcag aggctctgcg gccactccgc cttcccgcac ctgcgggtcc 150

tggaggccac ggcccgcgcc gggggccgca tccgctcgga gcgctgcttc 200

ggtggcgtgg tggaggtggg cgcgcactgg atccatgggc cctcccgggg 250

taaccccgtc ttccagctgg ctgctgagta cgggctgctg gggggagaagg 300

agctgtccca ggagaaccag ctggtggaga ccgggggtca cgtgggcctg 350

ccctccgtga gctacgccag ctccggggcc agcgtgagcc tccagctggt 400

ggcggagatg gcgactctgt tctacggcct gatagaccag acccgggagt 450

tcctgcacgc tgcagagacc ccggtgccca gcgtcgggga gtacctcaag 500

aaggagattg gccagcacgt ggccggctgg acagaggatg aggagaccag 550

gaagctgaag ctggccgtcc tgaactcctt cttcaacctg gaatgctgtg 600

tgagcggcac ccacagcatg gacctggtgg ccctggcacc ctttggggag 650

tataccgtgc tgccggggct ggactgcacc ttttctaagg gctatcaagg 700

actcacaaac tgcatgatgg ccgccctgcc ggaggacact gtagtttttg 750

agaagcctgt gaagaccatc cactggaacg ggtccttcca ggaggcagcc 800

tttcccgggg agacctttcc agtgtcggta gagtgtgagg atggagaccg 850

gttcccggcg caccatgtca tcgtcaccgt gcccttaggt tttcttaggg 900

aacatttgga caccttcttt gaccctcccc tgccggctga gaaggcagaa 950

gcaatcagga agataggctt tgggaccaac aacaaaatct tcctggagtt 1000

tgaggagccc ttctgggagc cagactgcca gctgatccag ctggtgtggg 1050

aggacacgtc gcccctggag gatgctgccc ctgagctaca ggacgcctgg 1100

ttccggaagc tcattggctt tgtggtcctg cctgcctttg cgtctgtcca 1150

cgttctctgt gggttcattg ccggacttga gtctgagttc atggagactc 1200

tgtcggatga agaagtactt ctgtgtctca cccaagtgct ccggagagtg 1250

acaggaaacc cacggctccc cgcgcccaag agcgtcctgc ggtctcgctg 1300

gcacagcgcc ccgtacacta gggggtccta cagctacgtg gccgtgggca 1350

gtactggggg cgacctggac ctgctggctc agcccctccc tgcagacggc 1400

gccggcgccc agctccagat cctgtttgcg gggaagccca cacatcgcac 1450

gttttactcc acgacgcacg ggctctgct gtcgggatgg agggaggccg 1500

accgcctcct cagtctgtgg gccccgcagg tgcagcagcc caggccgagg 1550

ctctagctgg gcccagccta ctctgttcca cccgtgtcgg gggtaggctg 1600

ggaccgtcat ttcttctgac agatttcagt ctggcttgaa atttggggat 1650

gttaatgagg gtcctctggt ttttggtaac cagggccacc ttctcagttc 1700

ttgtgtctgt tattggagtc tggccagggt tgacttgagc tgagacacca 1750

gatgctcacg gagatgctgg acacataaag caagttacag ccacaaaaaa 1800

aaaaaa 1806

<210> 122
<211> 511
<212> PRT
<213> Homo Sapien

<400> 122

Met Glu Ser Thr Gly Ser Val Gly Glu Ala Pro Gly Gly Pro Arg
1 5 10 15

Val Leu Val Val Gly Gly Gly Ile Ala Gly Leu Gly Ala Ala Gln
20 25 30

Arg Leu Cys Gly His Ser Ala Phe Pro His Leu Arg Val Leu Glu

35 40 45

Ala Thr Ala Arg Ala Gly Gly Arg Ile Arg Ser Glu Arg Cys Phe
50 55 60

Gly Gly Val Val Glu Val Gly Ala His Trp Ile His Gly Pro Ser
65 70 75

Arg Gly Asn Pro Val Phe Gln Leu Ala Ala Glu Tyr Gly Leu Leu
80 85 90

Gly Glu Lys Glu Leu Ser Gln Glu Asn Gln Leu Val Glu Thr Gly
95 100 105

Gly His Val Gly Leu Pro Ser Val Ser Tyr Ala Ser Ser Gly Ala
110 115 120

Ser Val Ser Leu Gln Leu Val Ala Glu Met Ala Thr Leu Phe Tyr
125 130 135

Gly Leu Ile Asp Gln Thr Arg Glu Phe Leu His Ala Ala Glu Thr
140 145 150

Pro Val Pro Ser Val Gly Glu Tyr Leu Lys Lys Glu Ile Gly Gln
155 160 165

His Val Ala Gly Trp Thr Glu Asp Glu Glu Thr Arg Lys Leu Lys
170 175 180

Leu Ala Val Leu Asn Ser Phe Phe Asn Leu Glu Cys Cys Val Ser
185 190 195

Gly Thr His Ser Met Asp Leu Val Ala Leu Ala Pro Phe Gly Glu
200 205 210

Tyr Thr Val Leu Pro Gly Leu Asp Cys Thr Phe Ser Lys Gly Tyr
215 220 225

Gln Gly Leu Thr Asn Cys Met Met Ala Ala Leu Pro Glu Asp Thr
230 235 240

Val Val Phe Glu Lys Pro Val Lys Thr Ile His Trp Asn Gly Ser
245 250 255

Phe Gln Glu Ala Ala Phe Pro Gly Glu Thr Phe Pro Val Ser Val
260 265 270

Glu Cys Glu Asp Gly Asp Arg Phe Pro Ala His His Val Ile Val
275 280 285

Thr Val Pro Leu Gly Phe Leu Arg Glu His Leu Asp Thr Phe Phe
290 295 300

Asp Pro Pro Leu Pro Ala Glu Lys Ala Glu Ala Ile Arg Lys Ile
305 310 315

Gly Phe Gly Thr Asn Asn Lys Ile Phe Leu Glu Phe Glu Glu Pro
320 325 330

Phe Trp Glu Pro Asp Cys Gln Leu Ile Gln Leu Val Trp Glu Asp
335 340 345

Thr Ser Pro Leu Glu Asp Ala Ala Pro Glu Leu Gln Asp Ala Trp
350 355 360

```
Phe Arg Lys Leu Ile Gly Phe Val Val Leu Pro Ala Phe Ala Ser
                365                 370                 375

Val His Val Leu Cys Gly Phe Ile Ala Gly Leu Glu Ser Glu Phe
                380                 385                 390

Met Glu Thr Leu Ser Asp Glu Glu Val Leu Leu Cys Leu Thr Gln
                395                 400                 405

Val Leu Arg Arg Val Thr Gly Asn Pro Arg Leu Pro Ala Pro Lys
                410                 415                 420

Ser Val Leu Arg Ser Arg Trp His Ser Ala Pro Tyr Thr Arg Gly
                425                 430                 435

Ser Tyr Ser Tyr Val Ala Val Gly Ser Thr Gly Gly Asp Leu Asp
                440                 445                 450

Leu Leu Ala Gln Pro Leu Pro Ala Asp Gly Ala Gly Ala Gln Leu
                455                 460                 465

Gln Ile Leu Phe Ala Gly Glu Ala Thr His Arg Thr Phe Tyr Ser
                470                 475                 480

Thr Thr His Gly Ala Leu Leu Ser Gly Trp Arg Glu Ala Asp Arg
                485                 490                 495

Leu Leu Ser Leu Trp Ala Pro Gln Val Gln Gln Pro Arg Pro Arg
                500                 505                 510

Leu


<210> 123
<211> 3479
<212> DNA
<213> Homo Sapien

<400> 123
cggacgcgtg ggggaagatg gataaataat tctgtcacac gtgccctggc 50

ctctggagct cagctgccag tccacgtcta gggaatctta gcatctggga 100

ccaagacact ttacagcaat catcaccctt tgcagaggag gtgagctcac 150

caggactcat ctgccatttc agaccttttg ctgctacctg ccaggtggcc 200

cccactgctg acgagagatg gtggatctct cagtctcccc ggactccttg 250

aagccagtat cgctgaccag cagtcttgtc ttcctcatgc acctcctcct 300

ccttcagcct ggggagccga gctcagaggt caaggtgcta ggccctgagt 350

atcccatcct ggccctcgtc ggggaggagg tggagttccc gtgccaccta 400

tggccacagc tggatgccca gcaaatggag atccgctggt tccggagtca 450

gaccttcaat gtggtacacc tgtaccagga gcagcaggag ctccctggca 500

ggcagatgcc ggcgttccgg aacaggacca agttggtcaa ggacgacatc 550

gcctatggca gcgtggtcct gcagcttcac agcatcatcc cctctgacaa 600
```

gggcacatat ggctgccgct tccactccga caacttctct ggcgaagctc 650
tctgggaact ggaggtagca gggctgggct cagaccctca cctctccctt 700
gagggcttca aggaaggagg cattcagctg aggctcagat ccagtggctg 750
gtaccccaag cctaaggttc agtggagaga ccaccaggga cagtgcctgc 800
ctccagagtt tgaagccatc gtctgggatg cccaggacct gttcagtctg 850
gaaacatctg tggttgtccg agcgggagcc ctcagcaatg tgtccgtctc 900
catccagaat ctcctcttga gccagaagaa agagttggtg gtccagatag 950
cagacgtgtt cgtacccgga gcctctgcgt ggaagagcgc gttcgtcgcg 1000
accctgccgc tgctgttggt cctcgcggcg ctggcgctgg gcgtcctccg 1050
gaagcagcgg agaagccgag aaaagctgag gaagcaggcg gagaagagac 1100
aagagaaact cactgcagag ctggaaaagc ttcagacaga gcttgactgg 1150
agacgggctg aaggccaggc tgagtggaga gcagcccaaa aatatgcagt 1200
ggatgtgacg ctggacccgg cctcggcgca ccccagcctg gaggtgtcgg 1250
aggatggcaa gagcgtgtct tcccgcgggg cgccgccagg cccggcgcct 1300
ggccacccgc agcggttctc ggagcagacg tgcgcgctga gcctggagcg 1350
gttctccgcc ggccgccact actgggaggt gcacgtgggc cgccgcagcc 1400
gctggttcct gggcgcctgc ctggccgcgg tgccgcgcgc ggggcctgcg 1450
cgcctgagcc ctgcggccgg ctactgggtg ctggggctgt ggaacggctg 1500
cgagtacttc gtcctggccc cgcaccgcgt cgcgctcacc ctgcgcgtgc 1550
ccccgcggcg cctgggcgtc ttcctggact acgaggccgg agagctgtcc 1600
ttcttcaacg tgtccgacgg ctcccacatc ttcaccttcc acgacacctt 1650
ctcgggcgcg ctctgtgcgt acttcaggcc cagggcccac gacggcggcg 1700
aacatccgga tcccctgacc atctgcccgc tgccggttag agggacgggc 1750
gtccccgaag agaacgacag tgacacctgg ctacagccct atgagcccgc 1800
ggaccccgcc ctggactggt ggtgaggcgc cctcgtggcc gcgggactgg 1850
ccccgggggg ccccctggat cccaggccag cgctttgctc tcctgctccg 1900
tctgaaggga gcaggtgcac cagccaaaat gtcagcgagg gggacaaaga 1950
gagggacctt tgcctacgta gatgtgtatg tgtagtgcga ttttcttcaa 2000
ggaaaggaga caagtccaaa gctcgtttgt ggattgtggg actgagcgaa 2050
ggagtacaaa tatatccacg tcgctcagag ctggggtgct cacggtgggc 2100
ggtgggcaag aagccagcat ggaagaaaga agggagaaaa ctttggtgac 2150
tgccttagag ggatcagtta atttgtatag ttttatattt tttgtatatg 2200

tttgctagct ctaaaaaggt cgagatgcaa taacacttcg taagcaacga 2250

gttcacctaa gtaaggctca gatcctagtt ttaaaaacca tttcccatta 2300

aaatgaagtt ggaggaacag ctgcttctga gccggggcaa aaatttcaag 2350

gtgagcctgg agcattgtgt gtggtgaagt aaaataaagg ctcaaaacgt 2400

gacggcaacc cggcaaaagg gtagggagcc aggccgaagg ggcctcactg 2450

accaattgtg ggacaatttg aacatcagga tgaataatga caggagagat 2500

tataacacac tgaataaaaa cataatccat gagttcatgc tgatactcaa 2550

atttcttttt aaaaaggaga aacaggaagg tttcttttgg aggtgaaatc 2600

taattattgg tgagagtctt ggagaacagg ctgtttccag tctcaaagca 2650

gtaaccttat acactactta taagtttgaa aggggaaagg ttacctttac 2700

aatggagaca tctaccagat catccaagtg attaaattta acatcatcaa 2750

tgatgggacc aaggacatta ttagtttgac aactggggaa agaagtgttc 2800

ttcacccccct acccccaaga cattctctct gtcggccagg ctggagtgca 2850

gcctcaacct cctgggccca agtgatcctc ccacctcagc acacaacacc 2900

atgcccaatt ttaagtgcgt tatagagacg ggggtctcac tttgttaccc 2950

aggctggtct caaactcctg cgctcaagca atcctcccac ctgggcctcc 3000

caaaatgctg ggtgtacagg catgagccgc tgtgcctggc ttcattttca 3050

gagtgagaca tttgtactgt ggctatgtag gagaacattc ttgttcttag 3100

caaacatact gaagttttta gatattaatt accacagtgt ctgccactga 3150

atttccagtg actaagtgga aaaatataaa acatatgaat ataaagaaag 3200

aaagagacaa gtcaaatgta gtaaaatgac aacacttggt gactctaggt 3250

gactggtcga cagatgttca ttgtactatc aatgtggctt tgctgtgggt 3300

ttgaaatttt gcaaactaag agttgggtgg cggggagaag gatacaccaa 3350

aaaactaagt gattatcttt ggatgggaaa atgtttggta attgcattct 3400

taaaatgtct tctttgtatt ttttaatgtt caataatgta tatgtatcag 3450

ttctgtaata aaggggaaaa cacttttca 3479

<210> 124
<211> 535
<212> PRT
<213> Homo Sapien

<400> 124
Met Val Asp Leu Ser Val Ser Pro Asp Ser Leu Lys Pro Val Ser
1               5                   10                  15

Leu Thr Ser Ser Leu Val Phe Leu Met His Leu Leu Leu Leu Gln
                20                  25                  30

```
Pro Gly Glu Pro Ser Ser Glu Val Lys Val Leu Gly Pro Glu Tyr
                35                  40                  45

Pro Ile Leu Ala Leu Val Gly Glu Glu Val Glu Phe Pro Cys His
                50                  55                  60

Leu Trp Pro Gln Leu Asp Ala Gln Gln Met Glu Ile Arg Trp Phe
                65                  70                  75

Arg Ser Gln Thr Phe Asn Val Val His Leu Tyr Gln Glu Gln Gln
                80                  85                  90

Glu Leu Pro Gly Arg Gln Met Pro Ala Phe Arg Asn Arg Thr Lys
                95                 100                 105

Leu Val Lys Asp Asp Ile Ala Tyr Gly Ser Val Val Leu Gln Leu
               110                 115                 120

His Ser Ile Ile Pro Ser Asp Lys Gly Thr Tyr Gly Cys Arg Phe
               125                 130                 135

His Ser Asp Asn Phe Ser Gly Glu Ala Leu Trp Glu Leu Glu Val
               140                 145                 150

Ala Gly Leu Gly Ser Asp Pro His Leu Ser Leu Glu Gly Phe Lys
               155                 160                 165

Glu Gly Gly Ile Gln Leu Arg Leu Arg Ser Ser Gly Trp Tyr Pro
               170                 175                 180

Lys Pro Lys Val Gln Trp Arg Asp His Gln Gly Gln Cys Leu Pro
               185                 190                 195

Pro Glu Phe Glu Ala Ile Val Trp Asp Ala Gln Asp Leu Phe Ser
               200                 205                 210

Leu Glu Thr Ser Val Val Val Arg Ala Gly Ala Leu Ser Asn Val
               215                 220                 225

Ser Val Ser Ile Gln Asn Leu Leu Leu Ser Gln Lys Lys Glu Leu
               230                 235                 240

Val Val Gln Ile Ala Asp Val Phe Val Pro Gly Ala Ser Ala Trp
               245                 250                 255

Lys Ser Ala Phe Val Ala Thr Leu Pro Leu Leu Leu Val Leu Ala
               260                 265                 270

Ala Leu Ala Leu Gly Val Leu Arg Lys Gln Arg Arg Ser Arg Glu
               275                 280                 285

Lys Leu Arg Lys Gln Ala Glu Lys Arg Gln Glu Lys Leu Thr Ala
               290                 295                 300

Glu Leu Glu Lys Leu Gln Thr Glu Leu Asp Trp Arg Arg Ala Glu
               305                 310                 315

Gly Gln Ala Glu Trp Arg Ala Ala Gln Lys Tyr Ala Val Asp Val
               320                 325                 330

Thr Leu Asp Pro Ala Ser Ala His Pro Ser Leu Glu Val Ser Glu
               335                 340                 345

Asp Gly Lys Ser Val Ser Ser Arg Gly Ala Pro Pro Gly Pro Ala
```

350 355 360

Pro Gly His Pro Gln Arg Phe Ser Glu Gln Thr Cys Ala Leu Ser
365 370 375

Leu Glu Arg Phe Ser Ala Gly Arg His Tyr Trp Glu Val His Val
380 385 390

Gly Arg Arg Ser Arg Trp Phe Leu Gly Ala Cys Leu Ala Ala Val
395 400 405

Pro Arg Ala Gly Pro Ala Arg Leu Ser Pro Ala Ala Gly Tyr Trp
410 415 420

Val Leu Gly Leu Trp Asn Gly Cys Glu Tyr Phe Val Leu Ala Pro
425 430 435

His Arg Val Ala Leu Thr Leu Arg Val Pro Pro Arg Arg Leu Gly
440 445 450

Val Phe Leu Asp Tyr Glu Ala Gly Glu Leu Ser Phe Phe Asn Val
455 460 465

Ser Asp Gly Ser His Ile Phe Thr Phe His Asp Thr Phe Ser Gly
470 475 480

Ala Leu Cys Ala Tyr Phe Arg Pro Arg Ala His Asp Gly Gly Glu
485 490 495

His Pro Asp Pro Leu Thr Ile Cys Pro Leu Pro Val Arg Gly Thr
500 505 510

Gly Val Pro Glu Glu Asn Asp Ser Asp Thr Trp Leu Gln Pro Tyr
515 520 525

Glu Pro Ala Asp Pro Ala Leu Asp Trp Trp
530 535

<210> 125
<211> 4374
<212> DNA
<213> Homo Sapien

<400> 125
tatagtccca gctactcatg gggctgatgc aggttgaggc aggaggttca 50

tgagcccagg aggttggagc tgtaatgagc taggattctg cctctgcact 100

cctagctgga tgacagagca agaccctgtc tcaaaaaaga aaaaaaaaaa 150

aaaaagaatg catgaaccag acatgacagt tcctggcctc aaagatcttc 200

caaaggaaat gatttttttt taaccaccaa tgctgcagga aaaagcaaca 250

tatttaagtt atccaataac acctatccaa taattgtaaa tcattatcat 300

gacatggtag agttgtttat atttcttttc cttttaggtg aaacaccatt 350

caaagtcgta gtcaaatctc tttcacctaa agagttggtc cggatacatg 400

tccctaaacc tttggacagg aatgatggaa catttttgat gagatatagg 450

atgtatgaaa ctgtcgatga aggcctgaag atagaggtcc tttatggtga 500

tgaacatgtg gctcagtctc cctatatttt gaaaggacca gtgtaccatg 550
agtactgtga gtgtccggaa gatcctcagg cctggcagaa gactctttct 600
tgtccaacca aggaaccaca gattgcaaaa gattttgctt cctttcccag 650
catcaatctc cagcaaatgc taaaagaagt ccccaaaagg tttggggatg 700
agagaggtgc cattgttcat tacacgattc tcaataacca tgtttaccgg 750
agatctttag ggaaatacac agacttcaag atgttctctg atgagatttt 800
gttatcattg acaagaaagg tccttctccc agatttagaa ttttatgtta 850
atcttggaga ttggcccttg gagcatcgaa aagtcaatgg aacccctagc 900
cccataccta tcatttcatg gtgtggctct ctggattcaa gagatgttgt 950
ccttccaacg tatgacatca cccactccat gcttgaagcc atgcggggtg 1000
ttacaaatga tctcctctct attcagggaa atacagggcc ttcctggatc 1050
aataaaacag agagagcttt cttcagaggt agagacagcc gagaggagag 1100
gctccagttg gtacagctgt ccaaagaaaa tcctcagcta ctagatgcag 1150
gaattacagg atatttcttt ttccaagaga aagaaaagga gcttggaaaa 1200
gccaagttga tgggtttctt tgatttcttt aagtacaagt atcaagtaaa 1250
tgtggatggg accgtggctg cttacagata tccatatctc atgctgggcg 1300
acagtctggt tttaaagcag gactcgccat attatgaaca tttctacatg 1350
gcactagaac cttggaagca ttatgttcca attaaaagaa atctgagtga 1400
tttattagag aaagttaaat gggctaagga aaatgatgaa gaagccaaga 1450
agattgcaaa agaaggacag ttgatggcta gggacctact acagccacac 1500
aggctttact gctactatta ccaagtactg cagaaatatg ccgagcgcca 1550
gtccagcaaa cccgaagtac gtgatggaat ggaacttgtt cctcagccag 1600
aagatagcac agccatctgc cagtgccaca ggaaaaagcc ttcaagagaa 1650
gaactttgag tcagcccaga atcacactcc tgtgtatccc ggctacactt 1700
taaggaaaga ttgaatctaa gctgtgaagg acagtataga agactgcacc 1750
aagtggacta gttctcccgg tggctttata tatgtagatg gatatagcag 1800
tactggttga gtatccctca tctgaaatgc ttaggaccag gagtgtttca 1850
ggcttcagat tttttaagat ttgggaatat ttgcatgtac ataatgaggt 1900
atcttgggga tgagatccaa gtctaaacac aaaattcatt tatattttat 1950
atataccttg ttcacatacc ctgaaggtaa ttttatataa tatttttaat 2000
aatttgtgca tgaaacaaag tttgtataca ttgaactgtc agaaagcaaa 2050
ggtgtcacta tcttagcgac ccaagtggtg gtgtcagcac tcaaaaagtt 2100

ttggattttg gggtatttca gattttagat ttttgtatga ggaatgttca 2150
acctgtattt gaacaagcat taccaaatat cattgaatat taatatcttt 2200
tgcgtaaaaa ctgctattat cagcatcata gtttctctaa aaagaaaact 2250
tggggatcat agccgataga gagacttgct aaaatataaa tcagcctctg 2300
caaaactgtt tacatattta ttggtttaca tattttattg gtttatttct 2350
atcccctgtt cactttttct cttccacttc caattatgaa gagaaaatat 2400
ttgttcaggg ttgtcccccc gccccccgtc actgcataat ttctcctctt 2450
acaagctgct tttggctttc attaataaca gcttcctttt agaaggtctg 2500
ataaggatat ttaaggaaga agagaatgac tctgttatta aaggtggcat 2550
ggagactgtg gagggaatat tttttaaagc actactcata tcctttaaac 2600
taaattttgc caaagcccga gacaacatta aggagaaatt gtaccttaag 2650
ttagtaattc caaatctatc tgagttgtat acccatcaaa gacaatacag 2700
ttattaacat agatgaaggt atgctatagg catcattcat tatctctata 2750
ttgaataggt gaaagataac tgtagtcagg tgaaaggcat tcatcatttt 2800
taagctgaaa aggggatcct tgaaaacact gaaaacctct acaacaatct 2850
tcaggaagcc tgctatcttg ggattcacta ataataggcc aagaacaaag 2900
gcaagcatcc attcctcact ccaccacttt tctatttcag tgggtgtcat 2950
tgctacgatg aagactttgg aaatttcctt tctcttttag gacagggtca 3000
ggatttagga ctcatagcct gaaagctcat tacatactcc ttgtaaccat 3050
cagtccaagg ttcagttcac taaagtgcat gttctaaaac aagagctatc 3100
ctcattccaa attttaaaat atgtactctg gccggttgca gtggctcacg 3150
cctgtaatcc cagcactttg gcaggccgag atgggcggat cttttgaggt 3200
caggagtttg agaccagcct ggccaacatg gtgaaacccc gtctctacta 3250
aaaatacaaa aattagccag gcatggtggc atttgcctgt aatcccagct 3300
actcgggagg ctgaggcagg agaatcactt gaacctggga ggcagaggtt 3350
gcagtgagct gagattacac cactgcactc cagcctgggt gacagagtga 3400
gactccatct caaaaactga aaataaaaat aaaaatatgt attctcctaa 3450
ctgaaatatt tacttaatct ggaaaacaat gtaactattt ttaaagtggt 3500
tacatctatt cttgctgaag aacaataaac agaatttttt gactaagcat 3550
aaccaaattt cagaacagtc taatcaatgc caagtatcca aggcaaactc 3600
taatacccat ccattgtgca aaaccacaag cacgcaagta ttaaataaga 3650
gcaagctgtc ctgagcccat acctaatgaa tttgtgtctt aaatattgta 3700

cattgtgttt gaggcttgtc aaaactggga ttatggcaag aaaggttgcc 3750

taactcatac ctttctgcct caaattccag gtgctaaagg ctaatggcat 3800

tttaaacatc ttacattttt aaaaatttat attgcctctg ccaaacaggc 3850

ctaatagtta aaagcaagtt gagacaaacc aggcagattc agtgtgtgga 3900

acaggaagga tgtgctttaa aaaaaggtgg aatccctcaa aaaattctat 3950

agggagacag cagccttaat ctacataatt cttcatctcg ccaattcagc 4000

cgcagccttt aaagagttag tgttaatggc tttctggttt gaaaacaaaa 4050

atgcatctat gtggttgaaa gtttgggagg agattcacca atatctgagg 4100

agaagatgga gtgaagggaa ttcttacttt ttgctttata cctttctata 4150

atatttagat ttttttttac tgtaagtatg gatcaaattg caaaataaag 4200

aaaaatgcca accttagaaa agacaataaa tgcacaaaag atataaacag 4250

gaacagcaaa tatttatatt ttttccattt tgctcttttt aaatctatgt 4300

ttagaacttt atatcttggg acttatgtat atatatacct tttaaataaa 4350

ataaattttc taaataaaaa gttg 4374

<210> 126
<211> 451
<212> PRT
<213> Homo Sapien

<400> 126
Met Val Glu Leu Phe Ile Phe Leu Phe Leu Leu Gly Glu Thr Pro
1 5 10 15

Phe Lys Val Val Val Lys Ser Leu Ser Pro Lys Glu Leu Val Arg
20 25 30

Ile His Val Pro Lys Pro Leu Asp Arg Asn Asp Gly Thr Phe Leu
35 40 45

Met Arg Tyr Arg Met Tyr Glu Thr Val Asp Glu Gly Leu Lys Ile
50 55 60

Glu Val Leu Tyr Gly Asp Glu His Val Ala Gln Ser Pro Tyr Ile
65 70 75

Leu Lys Gly Pro Val Tyr His Glu Tyr Cys Glu Cys Pro Glu Asp
80 85 90

Pro Gln Ala Trp Gln Lys Thr Leu Ser Cys Pro Thr Lys Glu Pro
95 100 105

Gln Ile Ala Lys Asp Phe Ala Ser Phe Pro Ser Ile Asn Leu Gln
110 115 120

Gln Met Leu Lys Glu Val Pro Lys Arg Phe Gly Asp Glu Arg Gly
125 130 135

Ala Ile Val His Tyr Thr Ile Leu Asn Asn His Val Tyr Arg Arg
140 145 150

Ser Leu Gly Lys Tyr Thr Asp Phe Lys Met Phe Ser Asp Glu Ile
155 160 165

Leu Leu Ser Leu Thr Arg Lys Val Leu Leu Pro Asp Leu Glu Phe
170 175 180

Tyr Val Asn Leu Gly Asp Trp Pro Leu Glu His Arg Lys Val Asn
185 190 195

Gly Thr Pro Ser Pro Ile Pro Ile Ile Ser Trp Cys Gly Ser Leu
200 205 210

Asp Ser Arg Asp Val Val Leu Pro Thr Tyr Asp Ile Thr His Ser
215 220 225

Met Leu Glu Ala Met Arg Gly Val Thr Asn Asp Leu Leu Ser Ile
230 235 240

Gln Gly Asn Thr Gly Pro Ser Trp Ile Asn Lys Thr Glu Arg Ala
245 250 255

Phe Phe Arg Gly Arg Asp Ser Arg Glu Glu Arg Leu Gln Leu Val
260 265 270

Gln Leu Ser Lys Glu Asn Pro Gln Leu Leu Asp Ala Gly Ile Thr
275 280 285

Gly Tyr Phe Phe Phe Gln Glu Lys Glu Lys Glu Leu Gly Lys Ala
290 295 300

Lys Leu Met Gly Phe Phe Asp Phe Phe Lys Tyr Lys Tyr Gln Val
305 310 315

Asn Val Asp Gly Thr Val Ala Ala Tyr Arg Tyr Pro Tyr Leu Met
320 325 330

Leu Gly Asp Ser Leu Val Leu Lys Gln Asp Ser Pro Tyr Tyr Glu
335 340 345

His Phe Tyr Met Ala Leu Glu Pro Trp Lys His Tyr Val Pro Ile
350 355 360

Lys Arg Asn Leu Ser Asp Leu Leu Glu Lys Val Lys Trp Ala Lys
365 370 375

Glu Asn Asp Glu Glu Ala Lys Lys Ile Ala Lys Glu Gly Gln Leu
380 385 390

Met Ala Arg Asp Leu Leu Gln Pro His Arg Leu Tyr Cys Tyr Tyr
395 400 405

Tyr Gln Val Leu Gln Lys Tyr Ala Glu Arg Gln Ser Ser Lys Pro
410 415 420

Glu Val Arg Asp Gly Met Glu Leu Val Pro Gln Pro Glu Asp Ser
425 430 435

Thr Ala Ile Cys Gln Cys His Arg Lys Lys Pro Ser Arg Glu Glu
440 445 450

Leu

<210> 127

<211> 2163
<212> DNA
<213> Homo Sapien

<400> 127
agccgtcgga gggagccgga gcgcttctcc cgagttggtg atagattggt 50

ggtcatccaa catgcagaaa tgaatgagca gtgaaaagca gcagagccga 100

tgggtcatga ggatgtaagt gcgtttgaag gcttccacac cctctactcc 150

aggaatcatg aataaactgg aggataagca ggaccagatg ataccatgaa 200

gagaagttta caggccctct attgccaact gttaagtttc ctgctgatct 250

tggcactgac cgaagcgctg gcatttgcca tccaggaacc atctcccagg 300

gaatctcttc aggtcctccc ttcaggcact cccccgggaa ccatggtgac 350

agcaccccac agctctacca gacatacttc tgtggtgatg ctgaccccca 400

atcccgatgg accccccctca caggctgcag ctcccatggc aacactgaca 450

ccccgtgcag aggggcaccc tcctacgcac accatctcca ccatcgctgc 500

gacagtaacc gccccctatt ctgaaagctc cctgtccaca gggcccgctc 550

cagcagccat ggcaaccaca tcctccaagc cagagggccg ccctcgaggg 600

caggctgccc ccaccatcct gctgacaaag ccaccggggg ccaccagccg 650

ccccaccaca gcgccccccc gcactaccac acgcaggccc cccaggcccc 700

caggctcttc ccgaaaaggg gctggtaatt catcacgccc tgtcccgcct 750

gcacctggtg gccactccag gagtaaagaa ggacagcgag gacgaaatcc 800

aagctccaca cctctggggc agaagcggcc cctggggaaa atctttcaga 850

tctacaaggg caacttcaca gggtctgtgg aaccagagcc ctctaccctc 900

acccccagga ccccactctg ggctactcc tcttcaccac agccccagac 950

agtggctgcg accacagtgc ccagcaatac ctcatgggca cccaccacca 1000

cctccctggg gcctgcaaag gacaagccag gccttcgcag agcagcccag 1050

gggggtggtt ctaccttcac cagccaagga gggacaccag atgccacagc 1100

agcctcaggt gcccctgtca gtccacaagc tgccccagtg ccttctcagc 1150

gcccccacca cggtgaccca caggatggcc ccagccatag tgactcttgg 1200

cttactgtta cccctggcac cagcagacct ctgtctacca gctctggggt 1250

cttcacggct gccacggggc ccaccccagc tgccttcgat accagtgtct 1300

cagccccttc ccaggggatt cctcagggag catccacaac ccccacaagct 1350

ccaacccatc cctccagggt ctcagaaagc actatttctg gagccaagga 1400

ggagactgtg gccaccctca ccatgaccga ccgggtgccc agtcctctct 1450

ccacagtggt atccacagcc acaggcaatt tcctcaaccg cctggtcccc 1500

gccgggacct ggaagcctgg gacagcaggg aacatctccc atgtggccga 1550

gggggacaaa ccgcagcaca gagccaccat ctgcctgagc aagatggata 1600

tcgcctgggt gatcctggcc atcagcgtgc ccatctcctc ctgctctgtc 1650

ctgctgacgg tgtgctgcat gaagaggaag aagaagaccg ccaacccgga 1700

gaacaacctg agctactgga acaacaccat caccatggac tacttcaaca 1750

ggcatgctgt ggagctgccc agggagatcc agtcccttga aacctctgag 1800

gaccagctct cagagccccg ctccccagcc aatggcgact atagagacac 1850

tgggatggtc cttgttaacc ccttctgtca agaaacactg tttgtgggaa 1900

acgatcaagt atctgagatc taactacagc aggcatcact ttgccattcc 1950

gtatttttcg tctctaaatt ataaatatac aaatatatat attataaata 2000

taaccttgtg taaccctgac ttaatgagaa acattttcag cttttttttcc 2050

tatgaattgt caacatcttt tttacaagtg tggtttaaaa aaaaaaaaac 2100

tttacagaat gatctgtggc tttataaaat aaaggtattt ctaagcaaaa 2150

aaaaaaaaaa aaa 2163

<210> 128
<211> 575
<212> PRT
<213> Homo Sapien

<400> 128
Met Lys Arg Ser Leu Gln Ala Leu Tyr Cys Gln Leu Leu Ser Phe
1 5 10 15

Leu Leu Ile Leu Ala Leu Thr Glu Ala Leu Ala Phe Ala Ile Gln
20 25 30

Glu Pro Ser Pro Arg Glu Ser Leu Gln Val Leu Pro Ser Gly Thr
35 40 45

Pro Pro Gly Thr Met Val Thr Ala Pro His Ser Ser Thr Arg His
50 55 60

Thr Ser Val Val Met Leu Thr Pro Asn Pro Asp Gly Pro Pro Ser
65 70 75

Gln Ala Ala Ala Pro Met Ala Thr Leu Thr Pro Arg Ala Glu Gly
80 85 90

His Pro Pro Thr His Thr Ile Ser Thr Ile Ala Ala Thr Val Thr
95 100 105

Ala Pro Tyr Ser Glu Ser Ser Leu Ser Thr Gly Pro Ala Pro Ala
110 115 120

Ala Met Ala Thr Thr Ser Ser Lys Pro Glu Gly Arg Pro Arg Gly
125 130 135

Gln Ala Ala Pro Thr Ile Leu Leu Thr Lys Pro Pro Gly Ala Thr
140 145 150

```
Ser Arg Pro Thr Thr Ala Pro Pro Arg Thr Thr Thr Arg Arg Pro
                155                 160                 165

Pro Arg Pro Pro Gly Ser Ser Arg Lys Gly Ala Gly Asn Ser Ser
                170                 175                 180

Arg Pro Val Pro Pro Ala Pro Gly Gly His Ser Arg Ser Lys Glu
                185                 190                 195

Gly Gln Arg Gly Arg Asn Pro Ser Ser Thr Pro Leu Gly Gln Lys
                200                 205                 210

Arg Pro Leu Gly Lys Ile Phe Gln Ile Tyr Lys Gly Asn Phe Thr
                215                 220                 225

Gly Ser Val Glu Pro Glu Pro Ser Thr Leu Thr Pro Arg Thr Pro
                230                 235                 240

Leu Trp Gly Tyr Ser Ser Ser Pro Gln Pro Gln Thr Val Ala Ala
                245                 250                 255

Thr Thr Val Pro Ser Asn Thr Ser Trp Ala Pro Thr Thr Thr Ser
                260                 265                 270

Leu Gly Pro Ala Lys Asp Lys Pro Gly Leu Arg Arg Ala Ala Gln
                275                 280                 285

Gly Gly Gly Ser Thr Phe Thr Ser Gln Gly Gly Thr Pro Asp Ala
                290                 295                 300

Thr Ala Ala Ser Gly Ala Pro Val Ser Pro Gln Ala Ala Pro Val
                305                 310                 315

Pro Ser Gln Arg Pro His His Gly Asp Pro Gln Asp Gly Pro Ser
                320                 325                 330

His Ser Asp Ser Trp Leu Thr Val Thr Pro Gly Thr Ser Arg Pro
                335                 340                 345

Leu Ser Thr Ser Ser Gly Val Phe Thr Ala Ala Thr Gly Pro Thr
                350                 355                 360

Pro Ala Ala Phe Asp Thr Ser Val Ser Ala Pro Ser Gln Gly Ile
                365                 370                 375

Pro Gln Gly Ala Ser Thr Thr Pro Gln Ala Pro Thr His Pro Ser
                380                 385                 390

Arg Val Ser Glu Ser Thr Ile Ser Gly Ala Lys Glu Glu Thr Val
                395                 400                 405

Ala Thr Leu Thr Met Thr Asp Arg Val Pro Ser Pro Leu Ser Thr
                410                 415                 420

Val Val Ser Thr Ala Thr Gly Asn Phe Leu Asn Arg Leu Val Pro
                425                 430                 435

Ala Gly Thr Trp Lys Pro Gly Thr Ala Gly Asn Ile Ser His Val
                440                 445                 450

Ala Glu Gly Asp Lys Pro Gln His Arg Ala Thr Ile Cys Leu Ser
                455                 460                 465
```

Lys Met Asp Ile Ala Trp Val Ile Leu Ala Ile Ser Val Pro Ile
470 475 480

Ser Ser Cys Ser Val Leu Leu Thr Val Cys Cys Met Lys Arg Lys
485 490 495

Lys Lys Thr Ala Asn Pro Glu Asn Asn Leu Ser Tyr Trp Asn Asn
500 505 510

Thr Ile Thr Met Asp Tyr Phe Asn Arg His Ala Val Glu Leu Pro
515 520 525

Arg Glu Ile Gln Ser Leu Glu Thr Ser Glu Asp Gln Leu Ser Glu
530 535 540

Pro Arg Ser Pro Ala Asn Gly Asp Tyr Arg Asp Thr Gly Met Val
545 550 555

Leu Val Asn Pro Phe Cys Gln Glu Thr Leu Phe Val Gly Asn Asp
560 565 570

Gln Val Ser Glu Ile
575

<210> 129
<211> 2334
<212> DNA
<213> Homo Sapien

<400> 129
aggcgaggcg cggcgccgct gcacacacgc acacggagct atggggtgcc 50

atgttgccac cagctgccac gtggcctggc ttttggtgct gatctctgga 100

tgctggggcc aggtgaaccg gctgcccttc ttcaccaacc acttctttga 150

tacatacctg ctgatcagcg aggacacgcc tgtgggttct tctgtgaccc 200

agttgctggc ccaagacatg gacaatgacc ccctggtgtt tggcgtgtct 250

ggggaggagg cctctcgctt ctttgcagtg gagcctgaca ctggcgtggt 300

gtggctccgg cagccactgg acagagagac caagtcagag ttcaccgtgg 350

agttctctgt cagcgaccac caggggtga tcacacggaa ggtgaacatc 400

caggtcgggg atgtgaatga caacgcgccc acatttcaca atcagcccta 450

cagcgtccgc atccctgaga atacaccagt ggggacgccc atcttcatcg 500

tgaatgccac agaccccgac ttgggggcag gggcagcgt cctctactcc 550

ttccagcccc cctcccaatt cttcgccatt gacagcgccc gcggtatcgt 600

cacagtgatc cgggagctgg actacgagac cacacaggcc taccagctca 650

cggtcaacgc cacagatcaa gacaagacca ggcctctgtc caccctggcc 700

aacttggcca tcatcatcac agatgtccag gacatggacc ccatcttcat 750

caacctgcct tacagcacca acatctacga gcattctcct ccgggcacga 800

cggtgcgcat catcaccgcc atagaccagg ataaaggacg tccccggggc 850

attggctaca ccatcgtttc agggaatacc aacagcatct ttgccctgga 900
ctacatcagc ggagtgctga ccttgaatgg cctgctggac cgggagaacc 950
ccctgtacag ccatggcttc atcctgactg tgaagggcac ggagctgaac 1000
gatgaccgca ccccatctga cgctacagtc accacgacct tcaatatcct 1050
ggttattgac atcaatgaca atgccccgga gttcaacagc tccgagtaca 1100
gcgtggccat cactgagctg gcacaggtcg gctttgccct tccactcttc 1150
atccaggtgg tggacaagga tgagaatttg ggcctgaaca gcatgtttga 1200
ggtgtacttg gtggggaaca actcccacca cttcatcatc tccccgacct 1250
ccgtccaggg gaaggcggac attcgtattc gggtggccat cccactggac 1300
tacgagaccg tggaccgcta cgactttgat ctctttgcca atgagagtgt 1350
gcctgaccat gtgggctatg ccaaggtgaa gatcactctc atcaatgaaa 1400
atgacaaccg gcccatcttc agccagccac tgtacaacat cagcctgtac 1450
gagaacgtca ccgtggggac ctctgtgctg acagtcctgg tgagtccccg 1500
cttcactgca gggccactga gctctccagg gccgactgtg gtgaggcacc 1550
cagagggatt ttgtccaagg gacctcagca atcagggaag gaggcacccc 1600
caaatccctg agctgtgttt gttggtgtat taaataaagt ttttggactc 1650
ttcaggaagg ggctcccttg acctaggttg caatatggaa aaggagccaa 1700
cctgaggggt gacgagactg agctgaggac actggttttc tgcctttccc 1750
tgagagagac tcagtgaggg tgggctggga gccctggaag ccccctcaaa 1800
tgggtgggaa ggtgccagcc atccttgaga agggcaaccc tctccatgtg 1850
agcacaggca ccagagaggg gcaggcgcct ggagggtacc ggggcacccc 1900
cagctgccca tggctggact tgccctttga caaggggccc tcccagtgtc 1950
atttgtatct gtcagtactc ttggttgcaa gggacagaaa cccttaagta 2000
gttcaagcaa aaaaggattg gctcatgtaa ctcaaaagta taagtgattt 2050
caggccgggc tcggtggctc acgcctgtca tccaacacct tgagaaagcc 2100
gaggtgggcg gatcacttga ggtcgggagt ttgagaccag cctggccaac 2150
atggcaaaac cccgtctcta ctaaaaatac aaaaattagc cgggtgtggt 2200
ggcacacgcc tgtagtccca gctactaggg aggctgaggc aggagaatcg 2250
cttgaaccca ggaggcggag gttgcagtga gccgagattg tgtcactgcc 2300
ctccagcctg ggcgacagag ccagattctg tctc 2334

<210> 130
<211> 530
<212> PRT
<213> Homo Sapien

<400> 130

```
Met Gly Cys His Val Ala Thr Ser Cys His Val Ala Trp Leu Leu
1               5                   10                  15

Val Leu Ile Ser Gly Cys Trp Gly Gln Val Asn Arg Leu Pro Phe
                20                  25                  30

Phe Thr Asn His Phe Phe Asp Thr Tyr Leu Leu Ile Ser Glu Asp
                35                  40                  45

Thr Pro Val Gly Ser Ser Val Thr Gln Leu Leu Ala Gln Asp Met
                50                  55                  60

Asp Asn Asp Pro Leu Val Phe Gly Val Ser Gly Glu Glu Ala Ser
                65                  70                  75

Arg Phe Phe Ala Val Glu Pro Asp Thr Gly Val Val Trp Leu Arg
                80                  85                  90

Gln Pro Leu Asp Arg Glu Thr Lys Ser Glu Phe Thr Val Glu Phe
                95                  100                 105

Ser Val Ser Asp His Gln Gly Val Ile Thr Arg Lys Val Asn Ile
                110                 115                 120

Gln Val Gly Asp Val Asn Asp Asn Ala Pro Thr Phe His Asn Gln
                125                 130                 135

Pro Tyr Ser Val Arg Ile Pro Glu Asn Thr Pro Val Gly Thr Pro
                140                 145                 150

Ile Phe Ile Val Asn Ala Thr Asp Pro Asp Leu Gly Ala Gly Gly
                155                 160                 165

Ser Val Leu Tyr Ser Phe Gln Pro Pro Ser Gln Phe Phe Ala Ile
                170                 175                 180

Asp Ser Ala Arg Gly Ile Val Thr Val Ile Arg Glu Leu Asp Tyr
                185                 190                 195

Glu Thr Thr Gln Ala Tyr Gln Leu Thr Val Asn Ala Thr Asp Gln
                200                 205                 210

Asp Lys Thr Arg Pro Leu Ser Thr Leu Ala Asn Leu Ala Ile Ile
                215                 220                 225

Ile Thr Asp Val Gln Asp Met Asp Pro Ile Phe Ile Asn Leu Pro
                230                 235                 240

Tyr Ser Thr Asn Ile Tyr Glu His Ser Pro Pro Gly Thr Thr Val
                245                 250                 255

Arg Ile Ile Thr Ala Ile Asp Gln Asp Lys Gly Arg Pro Arg Gly
                260                 265                 270

Ile Gly Tyr Thr Ile Val Ser Gly Asn Thr Asn Ser Ile Phe Ala
                275                 280                 285

Leu Asp Tyr Ile Ser Gly Val Leu Thr Leu Asn Gly Leu Leu Asp
                290                 295                 300

Arg Glu Asn Pro Leu Tyr Ser His Gly Phe Ile Leu Thr Val Lys
                305                 310                 315
```

Gly Thr Glu Leu Asn Asp Asp Arg Thr Pro Ser Asp Ala Thr Val
320 325 330

Thr Thr Thr Phe Asn Ile Leu Val Ile Asp Ile Asn Asp Asn Ala
335 340 345

Pro Glu Phe Asn Ser Ser Glu Tyr Ser Val Ala Ile Thr Glu Leu
350 355 360

Ala Gln Val Gly Phe Ala Leu Pro Leu Phe Ile Gln Val Val Asp
365 370 375

Lys Asp Glu Asn Leu Gly Leu Asn Ser Met Phe Glu Val Tyr Leu
380 385 390

Val Gly Asn Asn Ser His His Phe Ile Ile Ser Pro Thr Ser Val
395 400 405

Gln Gly Lys Ala Asp Ile Arg Ile Arg Val Ala Ile Pro Leu Asp
410 415 420

Tyr Glu Thr Val Asp Arg Tyr Asp Phe Asp Leu Phe Ala Asn Glu
425 430 435

Ser Val Pro Asp His Val Gly Tyr Ala Lys Val Lys Ile Thr Leu
440 445 450

Ile Asn Glu Asn Asp Asn Arg Pro Ile Phe Ser Gln Pro Leu Tyr
455 460 465

Asn Ile Ser Leu Tyr Glu Asn Val Thr Val Gly Thr Ser Val Leu
470 475 480

Thr Val Leu Val Ser Pro Arg Phe Thr Ala Gly Pro Leu Ser Ser
485 490 495

Pro Gly Pro Thr Val Val Arg His Pro Glu Gly Phe Cys Pro Arg
500 505 510

Asp Leu Ser Asn Gln Gly Arg Arg His Pro Gln Ile Pro Glu Leu
515 520 525

Cys Leu Leu Val Tyr
530

<210> 131
<211> 1840
<212> DNA
<213> Homo Sapien

<400> 131
gtgggccgcc cctgctgctg ccgtccatgc tgatgtttgc ggtgatcgtg 50

gcctccagcg ggctgctgct catgatcgag cggggcatcc tggccgagat 100

gaagcccctg cccctgcacc cgcccggccg cgagggcaca gcctggcgcg 150

ggaaagcccc caagcctggg ggcctgtccc tcagggctgg ggacgcggac 200

ttgcaagtgc ggcaggacgt ccggaacagg accctgcggg cggtgtgcgg 250

acagccaggc atgccccggg acccctggga cttgccggtg gggcagcggc 300

gcaccctgct gcgccacatc ctcgtaagtg accgttaccg cttcctctac 350

tgctacgtcc ccaaggtggc ctgctctaac tggaagcggg tgatgaaggt 400

gctggcaggc gtcctggaca gcgtggacgt ccgcctcaag atggaccacc 450

gcagtgacct ggtgttcctg gccgacctgc ggcctgagga gattcgctac 500

cgcctgcagc actactttaa gttcctgttt gtgcgggagc ccttggaacg 550

cctcctctct gcctaccgca acaagtttgg cgagatccga gagtaccagc 600

aacgctatgg ggctgagata gtgaggcggt acagggctgg agcggggccc 650

agccctgcag gcgacgatgt cacattcccc gagttcctga gatacctggt 700

ggatgaggac cctgagcgca tgaatgagca ttggatgccc gtgtaccacc 750

tgtgccagcc ttgtgccgtg cactatgact ttgtgggctc ctatgagagg 800

ctggaggctg atgcaaatca ggtgctggag tgggtacggg caccacctca 850

cgtccgattt ccagctcgcc aggcctggta ccggccagcc agccccgaaa 900

gcctgcatta ccacttgtgc agtgcccccc gggccctgct gcaggatgtg 950

ctgcctaagt atatcctgga cttctccctc tttgcctacc cactgcctaa 1000

tgtcaccaag gaggcgtgtc agcagtgacc atgggtgtgg ggccagcagc 1050

tggtggggac tggtttcaac gccagctttc tgtgcttctg cctgtcattc 1100

ggagaaactc tggctctggg gcttggggct tctcaggatc ctggatggca 1150

gagactgccc tcagaagttc cttgtccagg gtgggcaccc acagtgactc 1200

agaggacagg gctaggcagg agacctgctg ctcctcattg gggggatctc 1250

ttgggggca gacaccagtt tgccaatgaa gcaacacatc tgatctaaag 1300

actggctcca gaccccgggc tgccaggatt atgcagtcca cttggtctac 1350

cttaatttaa cctgtggcca aactcagaga tggtaccagc caggggcaag 1400

catgaccaga gccagggacc ctgtggctct gatcccccat ttatccaccc 1450

catgtgcctc aggactagag tgagcaatca taccttataa atgacttttg 1500

tgcctttctg ctccagtctc aaaatttcct acacctgcca gttctttaca 1550

tttttccaag gaaaggaaaa cggaagcagg gttcttgcct ggtagctcca 1600

ggacccagct ctgcaggcac ccaaagaccc tctgtgccca gcctcttcct 1650

tgagttctcg gaacctcctc cctaattctc ccttccttcc ccacaaggcc 1700

tttgaggttg tgactgtggc tggtatatct ggctgccatt tttctgatgc 1750

atttatttaa aatttgtact ttttgataga acccttgtaa gggctttgtt 1800

ttcctaatag ctgacttttt aataaagcag ttttatatat 1840

<210> 132
<211> 333

<212> PRT
<213> Homo Sapien

<400> 132
Met Leu Met Phe Ala Val Ile Val Ala Ser Ser Gly Leu Leu Leu
1 5 10 15

Met Ile Glu Arg Gly Ile Leu Ala Glu Met Lys Pro Leu Pro Leu
20 25 30

His Pro Pro Gly Arg Glu Gly Thr Ala Trp Arg Gly Lys Ala Pro
35 40 45

Lys Pro Gly Gly Leu Ser Leu Arg Ala Gly Asp Ala Asp Leu Gln
50 55 60

Val Arg Gln Asp Val Arg Asn Arg Thr Leu Arg Ala Val Cys Gly
65 70 75

Gln Pro Gly Met Pro Arg Asp Pro Trp Asp Leu Pro Val Gly Gln
80 85 90

Arg Arg Thr Leu Leu Arg His Ile Leu Val Ser Asp Arg Tyr Arg
95 100 105

Phe Leu Tyr Cys Tyr Val Pro Lys Val Ala Cys Ser Asn Trp Lys
110 115 120

Arg Val Met Lys Val Leu Ala Gly Val Leu Asp Ser Val Asp Val
125 130 135

Arg Leu Lys Met Asp His Arg Ser Asp Leu Val Phe Leu Ala Asp
140 145 150

Leu Arg Pro Glu Glu Ile Arg Tyr Arg Leu Gln His Tyr Phe Lys
155 160 165

Phe Leu Phe Val Arg Glu Pro Leu Glu Arg Leu Leu Ser Ala Tyr
170 175 180

Arg Asn Lys Phe Gly Glu Ile Arg Glu Tyr Gln Gln Arg Tyr Gly
185 190 195

Ala Glu Ile Val Arg Arg Tyr Arg Ala Gly Ala Gly Pro Ser Pro
200 205 210

Ala Gly Asp Asp Val Thr Phe Pro Glu Phe Leu Arg Tyr Leu Val
215 220 225

Asp Glu Asp Pro Glu Arg Met Asn Glu His Trp Met Pro Val Tyr
230 235 240

His Leu Cys Gln Pro Cys Ala Val His Tyr Asp Phe Val Gly Ser
245 250 255

Tyr Glu Arg Leu Glu Ala Asp Ala Asn Gln Val Leu Glu Trp Val
260 265 270

Arg Ala Pro Pro His Val Arg Phe Pro Ala Arg Gln Ala Trp Tyr
275 280 285

Arg Pro Ala Ser Pro Glu Ser Leu His Tyr His Leu Cys Ser Ala
290 295 300

Pro Arg Ala Leu Leu Gln Asp Val Leu Pro Lys Tyr Ile Leu Asp
305 310 315

Phe Ser Leu Phe Ala Tyr Pro Leu Pro Asn Val Thr Lys Glu Ala
320 325 330

Cys Gln Gln

<210> 133
<211> 1636
<212> DNA
<213> Homo Sapien

<400> 133
cggcagttct ggcccctgca gctggaggta ccctgagttc tgagggtcgt 50

agtgctgttt ctggtattct catcgcggtc acctctaccg gtgtggacaa 100

gtaaagtttg aatcagcttc tccatggcct gggcaccagt tcccggctga 150

gccattttcc ttttggctaa aagtccccgc ccagaggcca attcgtcgcg 200

gcggcggtgg agatcgcagg tcgctcaggc ttgcagatgg gtcaagggtt 250

gtggagagtg gtcagaaacc agcagctgca acaagaaggc tacagtgagc 300

aaggctacct caccagagag cagagcagga gaatggatgc gagcaacatt 350

tctaacacca atcatcgtaa acaagtccaa ggaggcattg acatatatca 400

tcttttgaag gcaaggaaat cgaaagaaca ggaaggattc attaatttgg 450

aaatgttgcc tcctgagcta agctttacca tcttgtccta cctgaatgca 500

actgaccttt gcttggcttc atgtgtttgg caggaccttg cgaatgatga 550

acttctctgg caagggttgt gcaaatccac ttggggtcac tgttccatat 600

acaataagaa cccaccttta ggattttctt ttagaaaatt gtatatgcag 650

ctggatgaag gcagcctcac ctttaatgcc aacccagatg agggagtgaa 700

ctactttatg tccaagggta tcctggatga ttcgccaaag gaaatagcaa 750

agtttatctt ctgtacaaga acactaaatt ggaaaaaact gagaatctat 800

cttgatgaaa ggagagatgt cttggatgac cttgtaacat tgcataattt 850

tagaaatcag ttcttgccaa atgcactgag agaatttttt cgtcatatcc 900

atgcccctga agagcgtgga gagtatcttg aaactcttat aacaaagttc 950

tcacatagat tctgtgcttg caaccctgat ttaatgcgag aacttggcct 1000

tagtcctgat gctgtctatg tactgtgcta ctctttgatt ctactttcca 1050

ttgacctcac tagccctcat gtgaagaata aaatgtcaaa aagggaattt 1100

attcgaaata cccgtcgcgc tgctcaaaat attagtgaag attttgtagg 1150

gcatctttat gacaatatct accttattgg ccatgtggct gcataaaaag 1200

cacaattgct aggacttcag tttttacttc agactaaagc tacccaagga 1250

cttagcagat atgggggtta catcagtgct ggtcattgta gcctgagtat 1300

acaatcaagc ttcagtgtgc aaccttttt tcttttgcca ttttctattt 1350

tagtaatttc cttggggaac taaataattt tgcagaattt ttcctaattt 1400

tgtttatcac gttttgcaca aagcagagcc actgtctaac acagctgtta 1450

acgaatgata aactgacatt atactctaaa agatggtgta tttgtgcatt 1500

agatttgcct gaaaaacttt atccatttcc attctttata caaataccat 1550

gtaatgtgta catatttaac taaagagatt tatagtcata attattttat 1600

tgtaaagatt ttaactaaag tttttccttt tctctc 1636

<210> 134
<211> 319
<212> PRT
<213> Homo Sapien

<400> 134
Met Gly Gln Gly Leu Trp Arg Val Val Arg Asn Gln Gln Leu Gln
1 5 10 15

Gln Glu Gly Tyr Ser Glu Gln Gly Tyr Leu Thr Arg Glu Gln Ser
20 25 30

Arg Arg Met Asp Ala Ser Asn Ile Ser Asn Thr Asn His Arg Lys
35 40 45

Gln Val Gln Gly Gly Ile Asp Ile Tyr His Leu Leu Lys Ala Arg
50 55 60

Lys Ser Lys Glu Gln Glu Gly Phe Ile Asn Leu Glu Met Leu Pro
65 70 75

Pro Glu Leu Ser Phe Thr Ile Leu Ser Tyr Leu Asn Ala Thr Asp
80 85 90

Leu Cys Leu Ala Ser Cys Val Trp Gln Asp Leu Ala Asn Asp Glu
95 100 105

Leu Leu Trp Gln Gly Leu Cys Lys Ser Thr Trp Gly His Cys Ser
110 115 120

Ile Tyr Asn Lys Asn Pro Pro Leu Gly Phe Ser Phe Arg Lys Leu
125 130 135

Tyr Met Gln Leu Asp Glu Gly Ser Leu Thr Phe Asn Ala Asn Pro
140 145 150

Asp Glu Gly Val Asn Tyr Phe Met Ser Lys Gly Ile Leu Asp Asp
155 160 165

Ser Pro Lys Glu Ile Ala Lys Phe Ile Phe Cys Thr Arg Thr Leu
170 175 180

Asn Trp Lys Lys Leu Arg Ile Tyr Leu Asp Glu Arg Arg Asp Val
185 190 195

Leu Asp Asp Leu Val Thr Leu His Asn Phe Arg Asn Gln Phe Leu
200 205 210

Pro Asn Ala Leu Arg Glu Phe Phe Arg His Ile His Ala Pro Glu
215 220 225

Glu Arg Gly Glu Tyr Leu Glu Thr Leu Ile Thr Lys Phe Ser His
230 235 240

Arg Phe Cys Ala Cys Asn Pro Asp Leu Met Arg Glu Leu Gly Leu
245 250 255

Ser Pro Asp Ala Val Tyr Val Leu Cys Tyr Ser Leu Ile Leu Leu
260 265 270

Ser Ile Asp Leu Thr Ser Pro His Val Lys Asn Lys Met Ser Lys
275 280 285

Arg Glu Phe Ile Arg Asn Thr Arg Arg Ala Ala Gln Asn Ile Ser
290 295 300

Glu Asp Phe Val Gly His Leu Tyr Asp Asn Ile Tyr Leu Ile Gly
305 310 315

His Val Ala Ala

<210> 135
<211> 1675
<212> DNA
<213> Homo Sapien

<400> 135
ggcacgaggg agcctccgtt agggggtggg aaaggacttt gccataggtc 50

gctgaggcca ccatctgctc tcttactggc caagggcgta aaaagatagt 100

cttcccatta gctagagagc aaaccccaga aagcctattg gctgcgccgt 150

ccgcgggcct tggtccgctt tgaaggcggg ctgcggctgc gagaggaggg 200

cgggcgggag gctagctgtt gtcgtggttg ctcggaggca cgtgtgcagt 250

cccggaagcg gcgaggggaa actgctccgc gcgcgccgcg ggaggaggaa 300

ccgcccggtc ctttagggtc cgggcccggc cgggccatgg attcaatgcc 350

tgagcccgcg tcccgctgtc ttctgcttct tcccttgctg ctgctgctgc 400

tgctgctgct gccggccccg gagctgggcc cgagccaggc cggagctgag 450

gagaacgact gggttcgcct gcccagcaaa tgcgaagtgt gtaaatatgt 500

tgctgtggag ctgaagtcag cctttgagga aaccggcaag accaaggagg 550

tgattggcac gggctatggc atcctggacc agaaggcctc tggagtcaaa 600

tacaccaagt cggacttgcg gttaatcgaa gtcactgaga ccatttgcaa 650

gaggctcctg gattatagcc tgcacaagga gaggaccggc agcaatcgat 700

ttgccaaggg catgtcagag acctttgaga cattacacaa cctggtacac 750

aaaggggtca aggtggtgat ggacatcccc tatgagctgt ggaacgagac 800

ttctgcagag gtggctgacc tcaagaagca gtgtgatgtg ctggtggaag 850

agtttgagga ggtgatcgag gactggtaca ggaaccacca ggaggaagac 900

ctgactgaat tcctctgcgc caaccacgtg ctgaagggaa aagacaccag 950

ttgcctggca gagcagtggt ccggcaagaa gggagacaca gctgccctgg 1000

gagggaagaa gtccaagaag aagagcagca gggccaaggc agcaggcggc 1050

aggagtagca gcagcaaaca aaggaaggag ctgggtggcc ttgagggaga 1100

ccccagcccc gaggaggatg agggcatcca gaaggcatcc cctctcacac 1150

acagcccccc tgatgagctc tgagcccacc cagcatcctc tgtcctgaga 1200

cccctgattt tgaagctgag gagtcagggg catggctctg gcaggccggg 1250

atggccccgc agccttcagc ccctccttgc cttggctgtg ccctcttctg 1300

ccaaggaaag acacaagccc caggaagaac tcagagccgt catgggtagc 1350

ccacgccgtc ctttcccctc cccaagtgtt tctctcctga cccagggttc 1400

aggcaggcct tgtggtttca ggactgcaag gactccagtg tgaactcagg 1450

aggggcaggt gtcagaactg ggcaccagga ctggagcccc ctccggagac 1500

caaactcacc atccctcagt cctccccaac agggtactag gactgcagcc 1550

ccctgtagct cctctctgct taccccctcct gtggacacct tgcactctgc 1600

ctggcccttc ccagagccca aagagtaaaa atgttctggt tctgatttct 1650

gaaaaaaaaa aaaaaaaaaa ttcct 1675

<210> 136
<211> 278
<212> PRT
<213> Homo Sapien

<400> 136

```
Met Asp Ser Met Pro Glu Pro Ala Ser Arg Cys Leu Leu Leu Leu
  1               5                  10                  15

Pro Leu Leu Leu Leu Leu Leu Leu Leu Leu Pro Ala Pro Glu Leu
                 20                  25                  30

Gly Pro Ser Gln Ala Gly Ala Glu Glu Asn Asp Trp Val Arg Leu
                 35                  40                  45

Pro Ser Lys Cys Glu Val Cys Lys Tyr Val Ala Val Glu Leu Lys
                 50                  55                  60

Ser Ala Phe Glu Glu Thr Gly Lys Thr Lys Glu Val Ile Gly Thr
                 65                  70                  75

Gly Tyr Gly Ile Leu Asp Gln Lys Ala Ser Gly Val Lys Tyr Thr
                 80                  85                  90

Lys Ser Asp Leu Arg Leu Ile Glu Val Thr Glu Thr Ile Cys Lys
                 95                 100                 105

Arg Leu Leu Asp Tyr Ser Leu His Lys Glu Arg Thr Gly Ser Asn
                110                 115                 120
```

Arg Phe Ala Lys Gly Met Ser Glu Thr Phe Glu Thr Leu His Asn
125 130 135

Leu Val His Lys Gly Val Lys Val Val Met Asp Ile Pro Tyr Glu
140 145 150

Leu Trp Asn Glu Thr Ser Ala Glu Val Ala Asp Leu Lys Lys Gln
155 160 165

Cys Asp Val Leu Val Glu Glu Phe Glu Glu Val Ile Glu Asp Trp
170 175 180

Tyr Arg Asn His Gln Glu Glu Asp Leu Thr Glu Phe Leu Cys Ala
185 190 195

Asn His Val Leu Lys Gly Lys Asp Thr Ser Cys Leu Ala Glu Gln
200 205 210

Trp Ser Gly Lys Lys Gly Asp Thr Ala Ala Leu Gly Gly Lys Lys
215 220 225

Ser Lys Lys Lys Ser Ser Arg Ala Lys Ala Ala Gly Gly Arg Ser
230 235 240

Ser Ser Ser Lys Gln Arg Lys Glu Leu Gly Gly Leu Glu Gly Asp
245 250 255

Pro Ser Pro Glu Glu Asp Glu Gly Ile Gln Lys Ala Ser Pro Leu
260 265 270

Thr His Ser Pro Pro Asp Glu Leu
275

<210> 137
<211> 2207
<212> DNA
<213> Homo Sapien

<220>
<221> unsure
<222> 2153, 2160
<223> unknown base

<400> 137
cacgcctccc gctgccagcc cggcaccggg atcttaatca gtcactatga 50

aaactcatta gctccacagc aatgagtcct ccactgctga agcttggcgc 100

tgtgcttagt accatggcaa tgatctcaaa ctggatgtcc caaactctcc 150

catccttggt gggactgaac accacgaggc tgtcgactcc ggatacctta 200

actcagatta gtcctaaaga agggtggcag gtgtacagct cagctcagga 250

tcctgatggg cggtgcattt gcacagttgt tgctccagaa caaaacctgt 300

gttcccggga tgccaaaagc aggcaacttc gccaactact ggaaaaggtt 350

cagaacatgt cccagtctat tgaagtctta aacttgagaa ctcagagaga 400

tttccaatat gttttaaaaa tggaaaccca aatgaaaggg ctgaaggcaa 450

aatttcggca gattgaagat gatcgaaaga cacttatgac caagcatttt 500

```
caggagttga aagagaaaat ggacgagctc ctgcctttga tccccgtgct 550
ggaacagtac aaaacagatg ctaagttaat cacccagttc aaggaggaaa 600
taaggaatct gtctgctgtc ctcactggta ttcaggagga aattggtgcc 650
tatgactacg aggaactaca ccaaagagtg ctgagcttgg aaacaagact 700
tcgtgactgc atgaaaaagc taacatgtgg caaactgatg aaaatcacag 750
gcccagttac agtcaagaca tctggaaccc gatttggtgc ttggatgaca 800
gaccctttag catctgagaa aaacaacaga gtctggtaca tggacagtta 850
tactaacaat aaaattgttc gtgaatacaa atcaattgca gactttgtca 900
gtggggctga atcaaggaca tacaaccttc ctttcaagtg ggcaggaact 950
aaccatgttg tctacaatgg ctcactctat tttaacaagt atcagagtaa 1000
tatcatcatc aaatacagct ttgatatggg gagagtgctt gcccaacgaa 1050
gcctggagta tgctggtttt cataatgttt acccctacac atggggtgga 1100
ttctctgaca tcgacctaat ggctgatgaa atcgggctgt gggctgtgta 1150
tgcaactaac cagaatgcag gcaatattgt catcagccaa cttaaccaag 1200
ataccttgga ggtgatgaag agctggagca ctggctaccc caagagaagt 1250
gcaggggaat ctttcatgat ctgtgggaca ctgtatgtca ccaactccca 1300
cttaactgga gccaaggtgt attattccta ttccaccaaa acctccacat 1350
atgagtacac agacattccc ttccataacc aatactttca catatccatg 1400
cttgactaca atgcaagaga tcgagctctc tatgcctgga acaatggcca 1450
ccaggtgctg ttcaatgtca ccctttttcca tatcatcaag acagaggatg 1500
acacataggc aaatgtgaca tgttttcatt gatttaaaca gtgtgatttg 1550
tgataaactc tataagaccc cttccgtttt tttcttcact attatttttc 1600
atcatttctc caaagcaaag catttttatt gtaaagttgg tgtttcaaaa 1650
acatagctga gcttgtctaa cttaccatgt tggaaacaca tcttaacttc 1700
taaatttaca aggcctatca tgtccttgtc atgaaaagca ctaaaaaaaa 1750
aaaagagttt aagtggctaa agtcatagtt ttgcaagaga ttaatgatct 1800
gccttatatt agagtcagag actaatggtg gcttaaatgc acgaatgtct 1850
ttttttttaa aactgtcatt ttttactgtc ttttgctcca tctcaggaaa 1900
tattttggta ggaattagga gaacaaaaag cacttttatc ccatttattt 1950
ctttaaaaaa tgtaaggatt tcatttatat tgaaaaataa tattaatcat 2000
tttgctgtta acacaattct ctgatgcggt gctgtacagt catttttaaa 2050
tctcttgcta acattttatt ggcagtatgt atttctacca ttgtaaccac 2100
```

cattgtgcta ttgtatctct tcacttctgt gaaagtaata ttttttataa 2150

aanacactgn aattttaaaa aaaaaaaaaa aaaacaaaaa aaaaaaaaaa 2200

aaaaaaa 2207

<210> 138
<211> 478
<212> PRT
<213> Homo Sapien

<400> 138

```
Met Ser Pro Pro Leu Leu Lys Leu Gly Ala Val Leu Ser Thr Met
  1               5                  10                  15

Ala Met Ile Ser Asn Trp Met Ser Gln Thr Leu Pro Ser Leu Val
                 20                  25                  30

Gly Leu Asn Thr Thr Arg Leu Ser Thr Pro Asp Thr Leu Thr Gln
                 35                  40                  45

Ile Ser Pro Lys Glu Gly Trp Gln Val Tyr Ser Ser Ala Gln Asp
                 50                  55                  60

Pro Asp Gly Arg Cys Ile Cys Thr Val Val Ala Pro Glu Gln Asn
                 65                  70                  75

Leu Cys Ser Arg Asp Ala Lys Ser Arg Gln Leu Arg Gln Leu Leu
                 80                  85                  90

Glu Lys Val Gln Asn Met Ser Gln Ser Ile Glu Val Leu Asn Leu
                 95                 100                 105

Arg Thr Gln Arg Asp Phe Gln Tyr Val Leu Lys Met Glu Thr Gln
                110                 115                 120

Met Lys Gly Leu Lys Ala Lys Phe Arg Gln Ile Glu Asp Asp Arg
                125                 130                 135

Lys Thr Leu Met Thr Lys His Phe Gln Glu Leu Lys Glu Lys Met
                140                 145                 150

Asp Glu Leu Leu Pro Leu Ile Pro Val Leu Glu Gln Tyr Lys Thr
                155                 160                 165

Asp Ala Lys Leu Ile Thr Gln Phe Lys Glu Glu Ile Arg Asn Leu
                170                 175                 180

Ser Ala Val Leu Thr Gly Ile Gln Glu Glu Ile Gly Ala Tyr Asp
                185                 190                 195

Tyr Glu Glu Leu His Gln Arg Val Leu Ser Leu Glu Thr Arg Leu
                200                 205                 210

Arg Asp Cys Met Lys Lys Leu Thr Cys Gly Lys Leu Met Lys Ile
                215                 220                 225

Thr Gly Pro Val Thr Val Lys Thr Ser Gly Thr Arg Phe Gly Ala
                230                 235                 240

Trp Met Thr Asp Pro Leu Ala Ser Glu Lys Asn Asn Arg Val Trp
                245                 250                 255
```

```
Tyr Met Asp Ser Tyr Thr Asn Asn Lys Ile Val Arg Glu Tyr Lys
                260                 265                 270

Ser Ile Ala Asp Phe Val Ser Gly Ala Glu Ser Arg Thr Tyr Asn
                275                 280                 285

Leu Pro Phe Lys Trp Ala Gly Thr Asn His Val Val Tyr Asn Gly
                290                 295                 300

Ser Leu Tyr Phe Asn Lys Tyr Gln Ser Asn Ile Ile Ile Lys Tyr
                305                 310                 315

Ser Phe Asp Met Gly Arg Val Leu Ala Gln Arg Ser Leu Glu Tyr
                320                 325                 330

Ala Gly Phe His Asn Val Tyr Pro Tyr Thr Trp Gly Gly Phe Ser
                335                 340                 345

Asp Ile Asp Leu Met Ala Asp Glu Ile Gly Leu Trp Ala Val Tyr
                350                 355                 360

Ala Thr Asn Gln Asn Ala Gly Asn Ile Val Ile Ser Gln Leu Asn
                365                 370                 375

Gln Asp Thr Leu Glu Val Met Lys Ser Trp Ser Thr Gly Tyr Pro
                380                 385                 390

Lys Arg Ser Ala Gly Glu Ser Phe Met Ile Cys Gly Thr Leu Tyr
                395                 400                 405

Val Thr Asn Ser His Leu Thr Gly Ala Lys Val Tyr Tyr Ser Tyr
                410                 415                 420

Ser Thr Lys Thr Ser Thr Tyr Glu Tyr Thr Asp Ile Pro Phe His
                425                 430                 435

Asn Gln Tyr Phe His Ile Ser Met Leu Asp Tyr Asn Ala Arg Asp
                440                 445                 450

Arg Ala Leu Tyr Ala Trp Asn Asn Gly His Gln Val Leu Phe Asn
                455                 460                 465

Val Thr Leu Phe His Ile Ile Lys Thr Glu Asp Asp Thr
                470                 475
```

<210> 139
<211> 1971
<212> DNA
<213> Homo Sapien

<400> 139

```
gaagcagtgc agagaggaga gcggagcgga gctgccgctg agcaaaggcc 50

ttcaccatgg ccgagtcccc cggctgctgc tccgtctggg cccgctgcct 100

ccactgcctg tatagctgcc actggaggaa atgccccaga gagaggatgc 150

aaaccagcaa gtgcgactgt atctggtttg gcctgctctt cctcaccttc 200

ctcctttccc tgagctggct gtacatcggg ctcgtccttc tcaatgacct 250

gcacaacttc aatgaattcc tcttccgccg ctggggacac tggatggact 300

ggtccctggc attcctgctg gtcatctctc tactggtcac atatgcatcc 350
```

ttgctattgg tcctggccct gctcctgcgg ctttgtagac agcccctgca 400
tctgcacagc ctccacaagg tgctgctgct cctcattatg ctgcttgtgg 450
cggctggcct tgtgggactg gacatccaat ggcagcagga gtggcatagc 500
ttgcgtgtgt cactgcaggc cacagcccca ttccttcata ttggagcagc 550
cgctggaatt gccctcctgg cctggcctgt ggctgatacc ttctaccgta 600
tccaccgaag aggtcccaag attctgctac tgctcctatt ttttggagtt 650
gtcctggtca tctacttggc ccccctatgc atctcctcac cctgcatcat 700
ggaacccaga gacttaccac ccaagcctgg gctggtggga caccgagggg 750
cccccatgct ggctcccgag aacaccctga tgtccttgcg gaagacagct 800
gaatgcggag ctactgtgtt tgagactgat gtgatggtca gctccgatgg 850
ggtccccttc ctcatgcatg atgagcacct cagcaggacc acgaatgtag 900
cctctgtatt cccaacccga atcacagccc acagcagtga cttctcctgg 950
actgaactga agagactcaa tgctggatcc tggttcctag agaggcgacc 1000
cttctggggg gccaaaccgc tggcaggccc tgatcagaaa gaggctgaga 1050
gtcagacggt accagcatta gaagagctat tggaggaagc tgcagccctc 1100
aacctttcca tcatgttcga cttgcgccga cccccacaga accacacata 1150
ctatgacact tttgtgatcc agacattgga gactgtgctg aatgcaaggg 1200
tgccccaagc catggtcttt tggctaccag atgaagatcg ggctaatgtc 1250
caacgacggg cacctggaat gcgccagata tatggacgtc agggaggcaa 1300
cagaacggag aggccccagt ttcttaacct cccctatcaa gatctgccac 1350
tattggatat caaggcattg cataaggata atgtctcggt gaacctattt 1400
gtagtgaaca agccctggct cttctctctg ctttggtgtg caggggtgga 1450
ttcggtcacc accaacgact gccagctgct gcagcagatg cgttacccta 1500
tctggcttat taccccctcaa acctacctaa tcatatgggt cattaccaat 1550
tgtgtttcca ccatgctgct tttgtggacc ttcctcctcc aaaggagatt 1600
tgttaagaag agagggaaaa ctggcttaga aacagcagtg ctgctgacaa 1650
ggatcaacaa tttcatgatg gagtgaatgc cctgccctgc ttccccaccc 1700
aagccagtct acattgccca aacagcaagg gttggagagt ggcttaagtg 1750
gaatgcttca ggggtggtgg gttgcaagtg gggggagctt tgccaacagg 1800
aggttttgaa ccatgagggc cctctgccca ggtgatgggc attccctaag 1850
ctgctatgga atctgctccc tttggggttt tgacctgaga tgtttgggaa 1900
gagagtgagt aatgagaagt ttctcctcaa atgaaactag aacagaggaa 1950

gtaaaaggga gattgctcgg a 1971

<210> 140
<211> 539
<212> PRT
<213> Homo Sapien

<400> 140
Met Ala Glu Ser Pro Gly Cys Cys Ser Val Trp Ala Arg Cys Leu
1 5 10 15

His Cys Leu Tyr Ser Cys His Trp Arg Lys Cys Pro Arg Glu Arg
20 25 30

Met Gln Thr Ser Lys Cys Asp Cys Ile Trp Phe Gly Leu Leu Phe
35 40 45

Leu Thr Phe Leu Leu Ser Leu Ser Trp Leu Tyr Ile Gly Leu Val
50 55 60

Leu Leu Asn Asp Leu His Asn Phe Asn Glu Phe Leu Phe Arg Arg
65 70 75

Trp Gly His Trp Met Asp Trp Ser Leu Ala Phe Leu Leu Val Ile
80 85 90

Ser Leu Leu Val Thr Tyr Ala Ser Leu Leu Leu Val Leu Ala Leu
95 100 105

Leu Leu Arg Leu Cys Arg Gln Pro Leu His Leu His Ser Leu His
110 115 120

Lys Val Leu Leu Leu Leu Ile Met Leu Leu Val Ala Ala Gly Leu
125 130 135

Val Gly Leu Asp Ile Gln Trp Gln Gln Glu Trp His Ser Leu Arg
140 145 150

Val Ser Leu Gln Ala Thr Ala Pro Phe Leu His Ile Gly Ala Ala
155 160 165

Ala Gly Ile Ala Leu Leu Ala Trp Pro Val Ala Asp Thr Phe Tyr
170 175 180

Arg Ile His Arg Arg Gly Pro Lys Ile Leu Leu Leu Leu Leu Phe
185 190 195

Phe Gly Val Val Leu Val Ile Tyr Leu Ala Pro Leu Cys Ile Ser
200 205 210

Ser Pro Cys Ile Met Glu Pro Arg Asp Leu Pro Pro Lys Pro Gly
215 220 225

Leu Val Gly His Arg Gly Ala Pro Met Leu Ala Pro Glu Asn Thr
230 235 240

Leu Met Ser Leu Arg Lys Thr Ala Glu Cys Gly Ala Thr Val Phe
245 250 255

Glu Thr Asp Val Met Val Ser Ser Asp Gly Val Pro Phe Leu Met
260 265 270

His Asp Glu His Leu Ser Arg Thr Thr Asn Val Ala Ser Val Phe

275 280 285

Pro Thr Arg Ile Thr Ala His Ser Ser Asp Phe Ser Trp Thr Glu
290 295 300

Leu Lys Arg Leu Asn Ala Gly Ser Trp Phe Leu Glu Arg Arg Pro
305 310 315

Phe Trp Gly Ala Lys Pro Leu Ala Gly Pro Asp Gln Lys Glu Ala
320 325 330

Glu Ser Gln Thr Val Pro Ala Leu Glu Glu Leu Leu Glu Glu Ala
335 340 345

Ala Ala Leu Asn Leu Ser Ile Met Phe Asp Leu Arg Arg Pro Pro
350 355 360

Gln Asn His Thr Tyr Tyr Asp Thr Phe Val Ile Gln Thr Leu Glu
365 370 375

Thr Val Leu Asn Ala Arg Val Pro Gln Ala Met Val Phe Trp Leu
380 385 390

Pro Asp Glu Asp Arg Ala Asn Val Gln Arg Arg Ala Pro Gly Met
395 400 405

Arg Gln Ile Tyr Gly Arg Gln Gly Gly Asn Arg Thr Glu Arg Pro
410 415 420

Gln Phe Leu Asn Leu Pro Tyr Gln Asp Leu Pro Leu Leu Asp Ile
425 430 435

Lys Ala Leu His Lys Asp Asn Val Ser Val Asn Leu Phe Val Val
440 445 450

Asn Lys Pro Trp Leu Phe Ser Leu Leu Trp Cys Ala Gly Val Asp
455 460 465

Ser Val Thr Thr Asn Asp Cys Gln Leu Leu Gln Gln Met Arg Tyr
470 475 480

Pro Ile Trp Leu Ile Thr Pro Gln Thr Tyr Leu Ile Ile Trp Val
485 490 495

Ile Thr Asn Cys Val Ser Thr Met Leu Leu Leu Trp Thr Phe Leu
500 505 510

Leu Gln Arg Arg Phe Val Lys Lys Arg Gly Lys Thr Gly Leu Glu
515 520 525

Thr Ala Val Leu Leu Thr Arg Ile Asn Asn Phe Met Met Glu
530 535

<210> 141
<211> 3671
<212> DNA
<213> Homo Sapien

<400> 141
gccgccggcc cgggctggag ccgagcgcag cagccaccgc cgccgccgcg 50

ccagaagttt gggttgaacc ggagctgccg ggaggaaact tttttctttt 100

ttccccctcc ctcccgggag gaggaggagg aggaggaggg gaagctgccg 150

ccggcgccaa ggctcgtggg ctcggggtcg gcgcggcccg cagaaggggc 200
gggggcctcg ccccgcgagg ggaggcgcgc cccgggggcc ccgagagggg 250
cggtgaggac cgcgggctgc tggtgcggcg gcggcggcgc gtgtgccccg 300
cgcaggggag ggcgcccgcc ccgctcccgg cccggctgcg aggaggaggc 350
ggcggcggcg caggaggatg tacttggtgg cgggggacag gggttggcc 400
ggctgcgggc acctcctggt ctcgctgctg ggctgctgc tgctgctggc 450
gcgctccggc acccgggcgc tggtctgcct gccctgtgac gagtccaagt 500
gcgaggagcc caggaactgc ccggggagca tcgtgcaggg cgtctgcggc 550
tgctgctaca cgtgcgccag ccagaggaac gagagctgcg gcggcacctt 600
cgggatttac ggaacctgcg accgggggct gcgttgtgtc atccgccccc 650
cgctcaatgg cgactccctc accgagtacg aagcgggcgt ttgcgaagat 700
gagaactgga ctgatgacca actgcttggt tttaaaccat gcaatgaaaa 750
ccttattgct ggctgcaata taatcaatgg gaaatgtgaa tgtaacacca 800
ttcgaacctg cagcaatccc tttgagtttc caagtcagga tatgtgcctt 850
tcagctttaa agagaattga agaagagaag ccagattgct ccaaggcccg 900
ctgtgaagtc cagttctctc cacgttgtcc tgaagattct gttctgatcg 950
agggttatgc tcctcctggg gagtgctgtc ccttacccag ccgctgcgtg 1000
tgcaaccccg caggctgtct gcgcaaagtc tgccagccgg gaaacctgaa 1050
catactagtg tcaaaagcct cagggaagcc gggagagtgc tgtgacctct 1100
atgagtgcaa accagttttc ggcgtggact gcaggactgt ggaatgccct 1150
cctgttcagc agaccgcgtg tcccccggac agctatgaaa ctcaagtcag 1200
actaactgca gatggttgct gtactttgcc aacaagatgc gagtgtctct 1250
ctggcttatg tggtttcccc gtgtgtgagg tgggatccac tccccgcata 1300
gtctctcgtg gcgatgggac acctggaaag tgctgtgatg tctttgaatg 1350
tgttaatgat acaaagccag cctgcgtatt taacaatgtg gaatattatg 1400
atggagacat gtttcgaatg gacaactgtc ggttctgtcg atgccaaggg 1450
ggcgttgcca tctgcttcac tgcccagtgt ggtgagataa actgcgagag 1500
gtactacgtg cccgaaggag agtgctgccc agtgtgtgaa gatccagtgt 1550
atccttttaa taatcccgct ggctgctatg ccaatggcct gatccttgcc 1600
cacggagacc ggtggcggga agacgactgc acattctgcc agtgcgtcaa 1650
cggtgaacgc cactgcgttg cgaccgtctg cggacagacc tgcacaaacc 1700
ctgtgaaagt gcctggggag tgttgccctg tgtgcgaaga accaaccatc 1750

atcacagttg atccacctgc atgtggggag ttatcaaact gcactctgac 1800
agggaaggac tgcattaatg gtttcaaacg cgatcacaat ggttgtcgga 1850
cctgtcagtg cataaacacc gaggaactat gttcagaacg taaacaaggc 1900
tgcaccttga actgtccctt cggtttcctt actgatgccc aaaactgtga 1950
gatctgtgag tgccgcccaa ggcccaagaa gtgcagaccc ataatctgtg 2000
acaagtattg tccacttgga ttgctgaaga ataagcacgg ctgtgacatc 2050
tgtcgctgta agaaatgtcc agagctctca tgcagtaaga tctgcccctt 2100
gggtttccag caggacagtc acggctgtct tatctgcaag tgcagagagg 2150
cctctgcttc agctgggcca cccatcctgt cgggcacttg tctcaccgtg 2200
gatggtcatc atcataaaaa tgaggagagc tggcacgatg ggtgccggga 2250
atgctactgt ctcaatggac gggaaatgtg tgccctgatc acctgcccgg 2300
tgcctgcctg tggcaacccc accattcacc ctggacagtg ctgcccatca 2350
tgtgcagatg actttgtggt gcagaagcca gagctcagta ctccctccat 2400
ttgccacgcc cctggaggag aatactttgt ggaaggagaa acgtggaaca 2450
ttgactcctg tactcagtgc acctgccaca gcggacgggt gctgtgtgag 2500
acagaggtgt gcccaccgct gctctgccag aacccctcac gcacccagga 2550
ttcctgctgc ccacagtgta cagatcaacc ttttcggcct tccttgtccc 2600
gcaataacag cgtacctaat tactgcaaaa atgatgaagg ggatatattc 2650
ctggcagctg agtcctggaa gcctgacgtt tgtaccagct gcatctgcat 2700
tgatagcgta attagctgtt tctctgagtc ctgcccttct gtatcctgtg 2750
aaagacctgt cttgagaaaa ggccagtgtt gtccctactg catagaagac 2800
acaattccaa agaaggtggt gtgccacttc agtgggaagg cctatgccga 2850
cgaggagcgg tgggaccttg acagctgcac ccactgctac tgcctgcagg 2900
gccagaccct ctgctcgacc gtcagctgcc cccctctgcc ctgtgttgag 2950
cccatcaacg tggaaggaag ttgctgccca atgtgtccag aaatgtatgt 3000
cccagaacca accaatatac ccattgagaa gacaaaccat cgaggagagg 3050
ttgacctgga ggttcccctg tggcccacgc ctagtgaaaa tgatatcgtc 3100
catctcccta gagatatggg tcacctccag gtagattaca gagataacag 3150
gctgcaccca agtgaagatt cttcactgga ctccattgcc tcagttgtgg 3200
ttcccataat tatatgcctc tctattataa tagcattcct attcatcaat 3250
cagaagaaac agtggatacc actgctttgc tggtatcgaa caccaactaa 3300
gccttcttcc ttaaataatc agctagtatc tgtggactgc aagaaaggaa 3350

ccagagtcca ggtggacagt tcccagagaa tgctaagaat tgcagaacca 3400

gatgcaagat tcagtggctt ctacagcatg caaaaacaga accatctaca 3450

ggcagacaat ttctaccaaa cagtgtgaag aaaggcaact aggatgaggt 3500

ttcaaaagac ggaagacgac taaatctgct ctaaaaagta aactagaatt 3550

tgtgcacttg cttagtggat tgtattggat tgtgacttga tgtacagcgc 3600

taagacctta ctgggatggg ctctgtctac agcaatgtgc agaacaagca 3650

ttcccacttt tcctcaaaaa a 3671

<210> 142
<211> 1036
<212> PRT
<213> Homo Sapien

<400> 142
Met Tyr Leu Val Ala Gly Asp Arg Gly Leu Ala Gly Cys Gly His
1 5 10 15

Leu Leu Val Ser Leu Leu Gly Leu Leu Leu Leu Leu Ala Arg Ser
20 25 30

Gly Thr Arg Ala Leu Val Cys Leu Pro Cys Asp Glu Ser Lys Cys
35 40 45

Glu Glu Pro Arg Asn Cys Pro Gly Ser Ile Val Gln Gly Val Cys
50 55 60

Gly Cys Cys Tyr Thr Cys Ala Ser Gln Arg Asn Glu Ser Cys Gly
65 70 75

Gly Thr Phe Gly Ile Tyr Gly Thr Cys Asp Arg Gly Leu Arg Cys
80 85 90

Val Ile Arg Pro Pro Leu Asn Gly Asp Ser Leu Thr Glu Tyr Glu
95 100 105

Ala Gly Val Cys Glu Asp Glu Asn Trp Thr Asp Asp Gln Leu Leu
110 115 120

Gly Phe Lys Pro Cys Asn Glu Asn Leu Ile Ala Gly Cys Asn Ile
125 130 135

Ile Asn Gly Lys Cys Glu Cys Asn Thr Ile Arg Thr Cys Ser Asn
140 145 150

Pro Phe Glu Phe Pro Ser Gln Asp Met Cys Leu Ser Ala Leu Lys
155 160 165

Arg Ile Glu Glu Glu Lys Pro Asp Cys Ser Lys Ala Arg Cys Glu
170 175 180

Val Gln Phe Ser Pro Arg Cys Pro Glu Asp Ser Val Leu Ile Glu
185 190 195

Gly Tyr Ala Pro Pro Gly Glu Cys Cys Pro Leu Pro Ser Arg Cys
200 205 210

Val Cys Asn Pro Ala Gly Cys Leu Arg Lys Val Cys Gln Pro Gly

215 220 225

Asn Leu Asn Ile Leu Val Ser Lys Ala Ser Gly Lys Pro Gly Glu
230 235 240

Cys Cys Asp Leu Tyr Glu Cys Lys Pro Val Phe Gly Val Asp Cys
245 250 255

Arg Thr Val Glu Cys Pro Pro Val Gln Gln Thr Ala Cys Pro Pro
260 265 270

Asp Ser Tyr Glu Thr Gln Val Arg Leu Thr Ala Asp Gly Cys Cys
275 280 285

Thr Leu Pro Thr Arg Cys Glu Cys Leu Ser Gly Leu Cys Gly Phe
290 295 300

Pro Val Cys Glu Val Gly Ser Thr Pro Arg Ile Val Ser Arg Gly
305 310 315

Asp Gly Thr Pro Gly Lys Cys Cys Asp Val Phe Glu Cys Val Asn
320 325 330

Asp Thr Lys Pro Ala Cys Val Phe Asn Asn Val Glu Tyr Tyr Asp
335 340 345

Gly Asp Met Phe Arg Met Asp Asn Cys Arg Phe Cys Arg Cys Gln
350 355 360

Gly Gly Val Ala Ile Cys Phe Thr Ala Gln Cys Gly Glu Ile Asn
365 370 375

Cys Glu Arg Tyr Tyr Val Pro Glu Gly Glu Cys Cys Pro Val Cys
380 385 390

Glu Asp Pro Val Tyr Pro Phe Asn Asn Pro Ala Gly Cys Tyr Ala
395 400 405

Asn Gly Leu Ile Leu Ala His Gly Asp Arg Trp Arg Glu Asp Asp
410 415 420

Cys Thr Phe Cys Gln Cys Val Asn Gly Glu Arg His Cys Val Ala
425 430 435

Thr Val Cys Gly Gln Thr Cys Thr Asn Pro Val Lys Val Pro Gly
440 445 450

Glu Cys Cys Pro Val Cys Glu Glu Pro Thr Ile Ile Thr Val Asp
455 460 465

Pro Pro Ala Cys Gly Glu Leu Ser Asn Cys Thr Leu Thr Gly Lys
470 475 480

Asp Cys Ile Asn Gly Phe Lys Arg Asp His Asn Gly Cys Arg Thr
485 490 495

Cys Gln Cys Ile Asn Thr Glu Glu Leu Cys Ser Glu Arg Lys Gln
500 505 510

Gly Cys Thr Leu Asn Cys Pro Phe Gly Phe Leu Thr Asp Ala Gln
515 520 525

Asn Cys Glu Ile Cys Glu Cys Arg Pro Arg Pro Lys Lys Cys Arg
530 535 540

Pro Ile Ile Cys Asp Lys Tyr Cys Pro Leu Gly Leu Leu Lys Asn
545 550 555

Lys His Gly Cys Asp Ile Cys Arg Cys Lys Lys Cys Pro Glu Leu
560 565 570

Ser Cys Ser Lys Ile Cys Pro Leu Gly Phe Gln Gln Asp Ser His
575 580 585

Gly Cys Leu Ile Cys Lys Cys Arg Glu Ala Ser Ala Ser Ala Gly
590 595 600

Pro Pro Ile Leu Ser Gly Thr Cys Leu Thr Val Asp Gly His His
605 610 615

His Lys Asn Glu Glu Ser Trp His Asp Gly Cys Arg Glu Cys Tyr
620 625 630

Cys Leu Asn Gly Arg Glu Met Cys Ala Leu Ile Thr Cys Pro Val
635 640 645

Pro Ala Cys Gly Asn Pro Thr Ile His Pro Gly Gln Cys Cys Pro
650 655 660

Ser Cys Ala Asp Asp Phe Val Val Gln Lys Pro Glu Leu Ser Thr
665 670 675

Pro Ser Ile Cys His Ala Pro Gly Gly Glu Tyr Phe Val Glu Gly
680 685 690

Glu Thr Trp Asn Ile Asp Ser Cys Thr Gln Cys Thr Cys His Ser
695 700 705

Gly Arg Val Leu Cys Glu Thr Glu Val Cys Pro Pro Leu Leu Cys
710 715 720

Gln Asn Pro Ser Arg Thr Gln Asp Ser Cys Cys Pro Gln Cys Thr
725 730 735

Asp Gln Pro Phe Arg Pro Ser Leu Ser Arg Asn Asn Ser Val Pro
740 745 750

Asn Tyr Cys Lys Asn Asp Glu Gly Asp Ile Phe Leu Ala Ala Glu
755 760 765

Ser Trp Lys Pro Asp Val Cys Thr Ser Cys Ile Cys Ile Asp Ser
770 775 780

Val Ile Ser Cys Phe Ser Glu Ser Cys Pro Ser Val Ser Cys Glu
785 790 795

Arg Pro Val Leu Arg Lys Gly Gln Cys Cys Pro Tyr Cys Ile Glu
800 805 810

Asp Thr Ile Pro Lys Lys Val Val Cys His Phe Ser Gly Lys Ala
815 820 825

Tyr Ala Asp Glu Glu Arg Trp Asp Leu Asp Ser Cys Thr His Cys
830 835 840

Tyr Cys Leu Gln Gly Gln Thr Leu Cys Ser Thr Val Ser Cys Pro
845 850 855

Pro Leu Pro Cys Val Glu Pro Ile Asn Val Glu Gly Ser Cys Cys
860 865 870

Pro Met Cys Pro Glu Met Tyr Val Pro Glu Pro Thr Asn Ile Pro
875 880 885

Ile Glu Lys Thr Asn His Arg Gly Glu Val Asp Leu Glu Val Pro
890 895 900

Leu Trp Pro Thr Pro Ser Glu Asn Asp Ile Val His Leu Pro Arg
905 910 915

Asp Met Gly His Leu Gln Val Asp Tyr Arg Asp Asn Arg Leu His
920 925 930

Pro Ser Glu Asp Ser Ser Leu Asp Ser Ile Ala Ser Val Val Val
935 940 945

Pro Ile Ile Ile Cys Leu Ser Ile Ile Ile Ala Phe Leu Phe Ile
950 955 960

Asn Gln Lys Lys Gln Trp Ile Pro Leu Leu Cys Trp Tyr Arg Thr
965 970 975

Pro Thr Lys Pro Ser Ser Leu Asn Asn Gln Leu Val Ser Val Asp
980 985 990

Cys Lys Lys Gly Thr Arg Val Gln Val Asp Ser Ser Gln Arg Met
995 1000 1005

Leu Arg Ile Ala Glu Pro Asp Ala Arg Phe Ser Gly Phe Tyr Ser
1010 1015 1020

Met Gln Lys Gln Asn His Leu Gln Ala Asp Asn Phe Tyr Gln Thr
1025 1030 1035

Val

<210> 143
<211> 1985
<212> DNA
<213> Homo Sapien

<400> 143
gacgtctggc cggctcccgg cgaagggcag cggaggagcg gcccagagcg 50

cgcagctagg gcactggcga aaccccggga cagtccctct ccgtgcgggg 100

gcggcgcaga gcagtcccat ccccggggtc ccgggcgcgg ctgactgccg 150

gctggttccc tgcgcgcagt agctccccga gccgggctgc accggaggcg 200

gcgagatggt cgcgcgcgtc ggcctcctgc tgcgcgccct gcagctgcta 250

ctgtggggcc acctggacgc ccagcccgcg gagcgcggag gccaggagct 300

gcgcaaggag gcggaggcat tcctagagaa gtacggatac ctcaatgaac 350

aggtccccaa agctcccacc tccactcgat tcagcgatgc catcagagcg 400

tttcagtggg tgtcccagct acctgtcagc ggcgtgttgg accgcgccac 450

cctgcgccag atgactcgtc cccgctgcgg ggttacagat accaacagtt 500

atgcggcctg ggctgagagg atcagtgact tgtttgctag acaccggacc 550

aaaatgaggc gtaagaaacg ctttgcaaag caaggtaaca aatggtacaa 600

gcagcacctc tcctaccgcc tggtgaactg gcctgagcat ctgccggagc 650

cggcagttcg ggcgccgtg cgcgccgcct tccagttgtg gagcaacgtc 700

tcagcgctgg agttctggga ggccccagcc acaggccccg ctgacatccg 750

gctcaccttc ttccaagggg accacaacga tgggctgggc aatgcctttg 800

atggcccagg gggcgccctg gcgcacgcct tcctgccccg ccgcggcgaa 850

gcgcacttcg accaagatga gcgctggtcc ctgagccgcc gccgcgggcg 900

caacctgttc gtggtgctgg cgcacgagat cggtcacacg cttggcctca 950

cccactcgcc cgcgccgcgc gcgctcatgg cgccctacta caagaggctg 1000

ggccgcgacg cgctgctcag ctgggacgac gtgctggccg tgcagagcct 1050

gtatgggaag cccctagggg gctcagtggc cgtccagctc ccaggaaagc 1100

tgttcactga ctttgagacc tgggactcct acagccccca aggaaggcgc 1150

cctgaaacgc agggccctaa atactgccac tcttccttcg atgccatcac 1200

tgtagacagg caacagcaac tgtacatttt taaagggagc catttctggg 1250

aggtggcagc tgatggcaac gtctcagagc cccgtccact gcaggaaaga 1300

tgggtcgggc tgccccccaa cattgaggct gcggcagtgt cattgaatga 1350

tggagatttc tacttcttca aggggggtcg atgctggagg ttccggggcc 1400

ccaagccagt gtggggtctc ccacagctgt gccgggcagg gggcctgccc 1450

cgccatcctg acgccgccct cttcttccct cctctgcgcc gcctcatcct 1500

cttcaagggt gcccgctact acgtgctggc ccgagggga ctgcaagtgg 1550

agccctacta cccccgaagt ctgcaggact ggggaggcat ccctgaggag 1600

gtcagcggcg ccctgccgag gcccgatggc tccatcatct tcttccgaga 1650

tgaccgctac tggcgcctcg accaggccaa actgcaggca accacctcgg 1700

gccgctgggc caccgagctg ccctggatgg gctgctggca tgccaactcg 1750

gggagcgccc tgttctgaag gcacctcctc acctcagaaa ctggtggtgc 1800

tctcagggca aaatcatgtt ccccaccccc ggggcagaac ccctcttaga 1850

agcctctgag tccctctgca gaagaccggg cagcaaagcc tccatctgga 1900

agtctgtctg cctttgttcc ttggaaaaaa aaaaaaaaaa aaaaaaaaaa 1950

aaaaaaaaaa aaaaaaaaaa aaaaaaaaaa aaaaa 1985

<210> 144
<211> 520
<212> PRT

<213> Homo Sapien

<400> 144
```
Met Val Ala Arg Val Gly Leu Leu Leu Arg Ala Leu Gln Leu Leu
  1               5                  10                  15

Leu Trp Gly His Leu Asp Ala Gln Pro Ala Glu Arg Gly Gly Gln
                 20                  25                  30

Glu Leu Arg Lys Glu Ala Glu Ala Phe Leu Glu Lys Tyr Gly Tyr
                 35                  40                  45

Leu Asn Glu Gln Val Pro Lys Ala Pro Thr Ser Thr Arg Phe Ser
                 50                  55                  60

Asp Ala Ile Arg Ala Phe Gln Trp Val Ser Gln Leu Pro Val Ser
                 65                  70                  75

Gly Val Leu Asp Arg Ala Thr Leu Arg Gln Met Thr Arg Pro Arg
                 80                  85                  90

Cys Gly Val Thr Asp Thr Asn Ser Tyr Ala Ala Trp Ala Glu Arg
                 95                 100                 105

Ile Ser Asp Leu Phe Ala Arg His Arg Thr Lys Met Arg Arg Lys
                110                 115                 120

Lys Arg Phe Ala Lys Gln Gly Asn Lys Trp Tyr Lys Gln His Leu
                125                 130                 135

Ser Tyr Arg Leu Val Asn Trp Pro Glu His Leu Pro Glu Pro Ala
                140                 145                 150

Val Arg Gly Ala Val Arg Ala Ala Phe Gln Leu Trp Ser Asn Val
                155                 160                 165

Ser Ala Leu Glu Phe Trp Glu Ala Pro Ala Thr Gly Pro Ala Asp
                170                 175                 180

Ile Arg Leu Thr Phe Phe Gln Gly Asp His Asn Asp Gly Leu Gly
                185                 190                 195

Asn Ala Phe Asp Gly Pro Gly Gly Ala Leu Ala His Ala Phe Leu
                200                 205                 210

Pro Arg Arg Gly Glu Ala His Phe Asp Gln Asp Glu Arg Trp Ser
                215                 220                 225

Leu Ser Arg Arg Arg Gly Arg Asn Leu Phe Val Val Leu Ala His
                230                 235                 240

Glu Ile Gly His Thr Leu Gly Leu Thr His Ser Pro Ala Pro Arg
                245                 250                 255

Ala Leu Met Ala Pro Tyr Tyr Lys Arg Leu Gly Arg Asp Ala Leu
                260                 265                 270

Leu Ser Trp Asp Asp Val Leu Ala Val Gln Ser Leu Tyr Gly Lys
                275                 280                 285

Pro Leu Gly Gly Ser Val Ala Val Gln Leu Pro Gly Lys Leu Phe
                290                 295                 300

Thr Asp Phe Glu Thr Trp Asp Ser Tyr Ser Pro Gln Gly Arg Arg
```

305 310 315

Pro Glu Thr Gln Gly Pro Lys Tyr Cys His Ser Ser Phe Asp Ala
320 325 330

Ile Thr Val Asp Arg Gln Gln Gln Leu Tyr Ile Phe Lys Gly Ser
335 340 345

His Phe Trp Glu Val Ala Ala Asp Gly Asn Val Ser Glu Pro Arg
350 355 360

Pro Leu Gln Glu Arg Trp Val Gly Leu Pro Pro Asn Ile Glu Ala
365 370 375

Ala Ala Val Ser Leu Asn Asp Gly Asp Phe Tyr Phe Phe Lys Gly
380 385 390

Gly Arg Cys Trp Arg Phe Arg Gly Pro Lys Pro Val Trp Gly Leu
395 400 405

Pro Gln Leu Cys Arg Ala Gly Gly Leu Pro Arg His Pro Asp Ala
410 415 420

Ala Leu Phe Phe Pro Pro Leu Arg Arg Leu Ile Leu Phe Lys Gly
425 430 435

Ala Arg Tyr Tyr Val Leu Ala Arg Gly Gly Leu Gln Val Glu Pro
440 445 450

Tyr Tyr Pro Arg Ser Leu Gln Asp Trp Gly Gly Ile Pro Glu Glu
455 460 465

Val Ser Gly Ala Leu Pro Arg Pro Asp Gly Ser Ile Ile Phe Phe
470 475 480

Arg Asp Asp Arg Tyr Trp Arg Leu Asp Gln Ala Lys Leu Gln Ala
485 490 495

Thr Thr Ser Gly Arg Trp Ala Thr Glu Leu Pro Trp Met Gly Cys
500 505 510

Trp His Ala Asn Ser Gly Ser Ala Leu Phe
515 520

<210> 145
<211> 3884
<212> DNA
<213> Homo Sapien

<400> 145
gccggctagg gcgccggagc cgcacgcagc cgcggggctc cgagaggcgc 50

gcactggggc tgggactgcg cggcgccgcc gctgcgagcg ccactgagcg 100

gtcgcgcaac ttcggaggca cagcgccgga gccaggcgag cgctcagaga 150

cccggagcca gaggggcgcg ccggagcctc gttcgagagc cggcgccagg 200

cacccaccgc gctccgagtg ccaggcggcc ctccgcgcag cgtggcttcc 250

gctgccccca cggaaggcac gggctggcgc tgccgggcgc cggggaggac 300

ggcgaggagg aggcggcggc ggcggagacg gcggcggcga gactggggcc 350

agggagacag ccctggggga gaggcgcccg aaccaggccg cgggagcatg 400
ggggcccgga gcggagctcg gggcgcgctg ctgctggcac tgctgctctg 450
ctgggacccg aggctgagcc aagcaggcac tgattctggc agcgaggtgc 500
tccctgactc cttcccgtca gcgccagcag agccgctgcc ctacttcctg 550
caggagccac aggacgccta cattgtgaag aacaagcctg tggagctccg 600
ctgccgcgcc ttccccgcca cacagatcta cttcaagtgc aacggcgagt 650
gggtcagcca gaacgaccac gtcacacagg aaggcctgga tgaggccacc 700
ggcctgcggg tgcgcgaggt gcagatcgag gtgtcgcggc agcaggtgga 750
ggagctcttt gggctggagg attactggtg ccagtgcgtg gcctggagct 800
ccgcaggcac caccaagagt cgccgagcct acgtccgcat cgcctacctg 850
cgcaagaact tcgatcagga gcctctgggc aaggaggtgc ccctggacca 900
tgaggttctc ctgcagtgcc gcccgccgga gggggtgcct gtggccgagg 950
tggaatggct caagaatgag gatgtcatcg accccaccca ggacaccaac 1000
ttcctgctca ccatcgacca caacctcatc atccgccagg cccgcctgtc 1050
ggacactgcc aactatacct gcgtggccaa gaacatcgtg gccaaacgcc 1100
ggagcaccac tgccaccgtc atcgtctacg tgaatggcgg ctggtccagc 1150
tgggcagagt ggtcaccctg ctccaaccgc tgtggccgag gctggcagaa 1200
gcgcacccgg acctgcacca accccgctcc actcaacgga ggggccttct 1250
gcgagggcca ggcattccag aagaccgcct gcaccaccat ctgcccagtc 1300
gatggggcgt ggacggagtg gagcaagtgg tcagcctgca gcactgagtg 1350
tgcccactgg cgtagccgcg agtgcatggc gcccccaccc cagaacggag 1400
gccgtgactg cagcgggacg ctgctcgact ctaagaactg cacagatggg 1450
ctgtgcatgc aaaataagaa aactctaagc gaccccaaca gccacctgct 1500
ggaggcctca ggggatgcgg cgctgtatgc ggggctcgtg gtggccatct 1550
tcgtggtcgt ggcaatcctc atggcggtgg gggtggtggt gtaccgccgc 1600
aactgccgtg acttcgacac agacatcact gactcatctg ctgccctgac 1650
tggtggtttc caccccgtca actttaagac ggcaaggccc agcaacccgc 1700
agctcctaca cccctctgtg cctcctgacc tgacagccag cgccggcatc 1750
taccgcggac ccgtgtatgc cctgcaggac tccaccgaca aaatccccat 1800
gaccaactct cctctgctgg acccccttacc cagccttaag gtcaaggtct 1850
acagctccag caccacgggc tctgggccag gcctggcaga tggggctgac 1900
ctgctggggg tcttgccgcc tggcacatac cctagcgatt tcgcccggga 1950

cacccacttc ctgcacctgc gcagcgccag cctcggttcc cagcagctct 2000
tgggcctgcc ccgagaccca gggagcagcg tcagcggcac ctttggctgc 2050
ctgggtggga ggctcagcat ccccggcaca ggggtcagct tgctggtgcc 2100
caatggagcc attccccagg gcaagttcta cgagatgtat ctactcatca 2150
acaaggcaga aagtaccctc ccgctttcag aagggaccca gacagtattg 2200
agcccctcgg tgacctgtgg acccacaggc ctcctgctgt gccgccccgt 2250
catcctcacc atgccccact gtgccgaagt cagtgcccgt gactggatct 2300
ttcagctcaa gacccaggcc caccagggcc actgggagga ggtggtgacc 2350
ctggatgagg agaccctgaa cacaccctgc tactgccagc tggagcccag 2400
ggcctgtcac atcctgctgg accagctggg cacctacgtg ttcacgggcg 2450
agtcctattc ccgctcagca gtcaagcggc tccagctggc cgtcttcgcc 2500
cccgccctct gcacctccct ggagtacagc ctccgggtct actgcctgga 2550
ggacacgcct gtagcactga aggaggtgct ggagctggag cggactctgg 2600
gcggatactt ggtggaggag ccgaaaccgc taatgttcaa ggacagttac 2650
cacaacctgc gcctctccct ccatgacctc ccccatgccc attggaggag 2700
caagctgctg gccaaatacc aggagatccc cttctatcac atttggagtg 2750
gcagccagaa ggccctccac tgcactttca ccctggagag gcacagcttg 2800
gcctccacag agctcacctg caagatctgc gtgcggcaag tggaagggga 2850
gggccagata ttccagctgc ataccactct ggcagagaca cctgctggct 2900
ccctggacac tctctgctct gcccctggca gcactgtcac cacccagctg 2950
ggaccttatg ccttcaagat cccactgtcc atccgccaga agatatgcaa 3000
cagcctagat gcccccaact cacggggcaa tgactggcgg atgttagcac 3050
agaagctctc tatggaccgg tacctgaatt actttgccac caaagcgagc 3100
cccacgggtg tgatcctgga cctctgggaa gctctgcagc aggacgatgg 3150
ggacctcaac agcctggcga gtgccttgga ggagatgggc aagagtgaga 3200
tgctggtggc tgtggccacc gacggggact gctgagcctc ctgggacagc 3250
gggctggcag ggactggcag gaggcaggtg cagggaggcc tggggcagcc 3300
tcctgatggg gatgtttggc ctctgcttcc tcccagttca cagccagagt 3350
tgcctctcct cctcctcttc cccaaccccc agaccatgac cagccttaga 3400
aaatccatgt actctgttgt tagagggccc agagttcctt ctccaccccc 3450
gctctctctc tcttggcctg agatctctgt gcaggaacca agatggggct 3500
gaagcctctg gaggcagttg gttgggggcg ggcaggcagg aggccctccc 3550

tccaccccccc caccctcagc ccggcaactt ctgggttccg tgggttttag 3600

ttccgttctt cgttttcttc ctccgttatt gatttctcct ttctccctaa 3650

gccccccttct gcttccacgc ccttttcctc tttgaagagt caagtacaat 3700

tcagacaaac tgctttctcc tgtccaaaag caaaaaggca aaggaaagaa 3750

agaaagcttc agaccgctag taaggctcaa agaagaagaa aaacaccaaa 3800

accacaaggg aaaagaaaaa cccagtttct taggaaacgc aaacgattta 3850

ttatccagat tatttggata agtccttttt aaaa 3884

<210> 146
<211> 945
<212> PRT
<213> Homo Sapien

<400> 146
```
Met Gly Ala Arg Ser Gly Ala Arg Gly Ala Leu Leu Leu Ala Leu
 1               5                  10                  15

Leu Leu Cys Trp Asp Pro Arg Leu Ser Gln Ala Gly Thr Asp Ser
                20                  25                  30

Gly Ser Glu Val Leu Pro Asp Ser Phe Pro Ser Ala Pro Ala Glu
                35                  40                  45

Pro Leu Pro Tyr Phe Leu Gln Glu Pro Gln Asp Ala Tyr Ile Val
                50                  55                  60

Lys Asn Lys Pro Val Glu Leu Arg Cys Arg Ala Phe Pro Ala Thr
                65                  70                  75

Gln Ile Tyr Phe Lys Cys Asn Gly Glu Trp Val Ser Gln Asn Asp
                80                  85                  90

His Val Thr Gln Glu Gly Leu Asp Glu Ala Thr Gly Leu Arg Val
                95                 100                 105

Arg Glu Val Gln Ile Glu Val Ser Arg Gln Gln Val Glu Glu Leu
               110                 115                 120

Phe Gly Leu Glu Asp Tyr Trp Cys Gln Cys Val Ala Trp Ser Ser
               125                 130                 135

Ala Gly Thr Thr Lys Ser Arg Arg Ala Tyr Val Arg Ile Ala Tyr
               140                 145                 150

Leu Arg Lys Asn Phe Asp Gln Glu Pro Leu Gly Lys Glu Val Pro
               155                 160                 165

Leu Asp His Glu Val Leu Leu Gln Cys Arg Pro Pro Glu Gly Val
               170                 175                 180

Pro Val Ala Glu Val Glu Trp Leu Lys Asn Glu Asp Val Ile Asp
               185                 190                 195

Pro Thr Gln Asp Thr Asn Phe Leu Leu Thr Ile Asp His Asn Leu
               200                 205                 210

Ile Ile Arg Gln Ala Arg Leu Ser Asp Thr Ala Asn Tyr Thr Cys
               215                 220                 225
```

```
Val Ala Lys Asn Ile Val Ala Lys Arg Arg Ser Thr Thr Ala Thr
                230                 235                 240

Val Ile Val Tyr Val Asn Gly Gly Trp Ser Ser Trp Ala Glu Trp
                245                 250                 255

Ser Pro Cys Ser Asn Arg Cys Gly Arg Gly Trp Gln Lys Arg Thr
                260                 265                 270

Arg Thr Cys Thr Asn Pro Ala Pro Leu Asn Gly Gly Ala Phe Cys
                275                 280                 285

Glu Gly Gln Ala Phe Gln Lys Thr Ala Cys Thr Thr Ile Cys Pro
                290                 295                 300

Val Asp Gly Ala Trp Thr Glu Trp Ser Lys Trp Ser Ala Cys Ser
                305                 310                 315

Thr Glu Cys Ala His Trp Arg Ser Arg Glu Cys Met Ala Pro Pro
                320                 325                 330

Pro Gln Asn Gly Gly Arg Asp Cys Ser Gly Thr Leu Leu Asp Ser
                335                 340                 345

Lys Asn Cys Thr Asp Gly Leu Cys Met Gln Asn Lys Lys Thr Leu
                350                 355                 360

Ser Asp Pro Asn Ser His Leu Leu Glu Ala Ser Gly Asp Ala Ala
                365                 370                 375

Leu Tyr Ala Gly Leu Val Val Ala Ile Phe Val Val Val Ala Ile
                380                 385                 390

Leu Met Ala Val Gly Val Val Val Tyr Arg Arg Asn Cys Arg Asp
                395                 400                 405

Phe Asp Thr Asp Ile Thr Asp Ser Ser Ala Ala Leu Thr Gly Gly
                410                 415                 420

Phe His Pro Val Asn Phe Lys Thr Ala Arg Pro Ser Asn Pro Gln
                425                 430                 435

Leu Leu His Pro Ser Val Pro Pro Asp Leu Thr Ala Ser Ala Gly
                440                 445                 450

Ile Tyr Arg Gly Pro Val Tyr Ala Leu Gln Asp Ser Thr Asp Lys
                455                 460                 465

Ile Pro Met Thr Asn Ser Pro Leu Leu Asp Pro Leu Pro Ser Leu
                470                 475                 480

Lys Val Lys Val Tyr Ser Ser Ser Thr Thr Gly Ser Gly Pro Gly
                485                 490                 495

Leu Ala Asp Gly Ala Asp Leu Leu Gly Val Leu Pro Pro Gly Thr
                500                 505                 510

Tyr Pro Ser Asp Phe Ala Arg Asp Thr His Phe Leu His Leu Arg
                515                 520                 525

Ser Ala Ser Leu Gly Ser Gln Gln Leu Leu Gly Leu Pro Arg Asp
                530                 535                 540
```

```
Pro Gly Ser Ser Val Ser Gly Thr Phe Gly Cys Leu Gly Gly Arg
                545                 550                 555

Leu Ser Ile Pro Gly Thr Gly Val Ser Leu Leu Val Pro Asn Gly
                560                 565                 570

Ala Ile Pro Gln Gly Lys Phe Tyr Glu Met Tyr Leu Leu Ile Asn
                575                 580                 585

Lys Ala Glu Ser Thr Leu Pro Leu Ser Glu Gly Thr Gln Thr Val
                590                 595                 600

Leu Ser Pro Ser Val Thr Cys Gly Pro Thr Gly Leu Leu Leu Cys
                605                 610                 615

Arg Pro Val Ile Leu Thr Met Pro His Cys Ala Glu Val Ser Ala
                620                 625                 630

Arg Asp Trp Ile Phe Gln Leu Lys Thr Gln Ala His Gln Gly His
                635                 640                 645

Trp Glu Glu Val Val Thr Leu Asp Glu Glu Thr Leu Asn Thr Pro
                650                 655                 660

Cys Tyr Cys Gln Leu Glu Pro Arg Ala Cys His Ile Leu Leu Asp
                665                 670                 675

Gln Leu Gly Thr Tyr Val Phe Thr Gly Glu Ser Tyr Ser Arg Ser
                680                 685                 690

Ala Val Lys Arg Leu Gln Leu Ala Val Phe Ala Pro Ala Leu Cys
                695                 700                 705

Thr Ser Leu Glu Tyr Ser Leu Arg Val Tyr Cys Leu Glu Asp Thr
                710                 715                 720

Pro Val Ala Leu Lys Glu Val Leu Glu Leu Glu Arg Thr Leu Gly
                725                 730                 735

Gly Tyr Leu Val Glu Glu Pro Lys Pro Leu Met Phe Lys Asp Ser
                740                 745                 750

Tyr His Asn Leu Arg Leu Ser Leu His Asp Leu Pro His Ala His
                755                 760                 765

Trp Arg Ser Lys Leu Leu Ala Lys Tyr Gln Glu Ile Pro Phe Tyr
                770                 775                 780

His Ile Trp Ser Gly Ser Gln Lys Ala Leu His Cys Thr Phe Thr
                785                 790                 795

Leu Glu Arg His Ser Leu Ala Ser Thr Glu Leu Thr Cys Lys Ile
                800                 805                 810

Cys Val Arg Gln Val Glu Gly Glu Gly Gln Ile Phe Gln Leu His
                815                 820                 825

Thr Thr Leu Ala Glu Thr Pro Ala Gly Ser Leu Asp Thr Leu Cys
                830                 835                 840

Ser Ala Pro Gly Ser Thr Val Thr Thr Gln Leu Gly Pro Tyr Ala
                845                 850                 855

Phe Lys Ile Pro Leu Ser Ile Arg Gln Lys Ile Cys Asn Ser Leu
```

860 865 870

Asp Ala Pro Asn Ser Arg Gly Asn Asp Trp Arg Met Leu Ala Gln
875 880 885

Lys Leu Ser Met Asp Arg Tyr Leu Asn Tyr Phe Ala Thr Lys Ala
890 895 900

Ser Pro Thr Gly Val Ile Leu Asp Leu Trp Glu Ala Leu Gln Gln
905 910 915

Asp Asp Gly Asp Leu Asn Ser Leu Ala Ser Ala Leu Glu Glu Met
920 925 930

Gly Lys Ser Glu Met Leu Val Ala Val Ala Thr Asp Gly Asp Cys
935 940 945

<210> 147
<211> 3734
<212> DNA
<213> Homo Sapien

<400> 147
gagagggaca gaggctggag aaggatgtat ggcctgccct gggcttgtct 50

gttccctcct gagcctgagc cccttacctt cctgacccca tgaagcacac 100

actggctctg ctggctcccc tgctgggcct gggcctgggg ctggccctga 150

gtcagctggc tgcaggggcc acagactgca agttccttgg cccggcagag 200

cacctgacat tcaccccagc agccagggcc cggtggctgg cccctcgagt 250

tcgtgcgcca ggactcctgg actccctcta tggcaccgtg cgccgcttcc 300

tctcggtggt gcagctcaat cctttccctt cagagttggt aaaggcccta 350

ctgaatgagc tggcctccgt gaaggtgaat gaggtggtgc ggtacgaggc 400

gggctacgtg gtatgcgctg tgatcgcggg cctctacctg ctgctggtgc 450

ccactgccgg gctttgcttc tgctgctgcc gctgccaccg gcgctgcggg 500

ggacgagtga agacagagca caaggcgctg gcctgtgagc gcgcggccct 550

catggtcttc ctgctgctga ccaccctctt gctgctgatt ggtgtggtct 600

gtgcctttgt caccaaccag cgcacgcatg aacagatggg ccccagcatc 650

gaggccatgc ctgagaccct gctcagcctc tggggcctgg tctctgatgt 700

cccccaagag ctgcaggccg tggcacagca attctccctg ccccaggagc 750

aagtctcaga ggagctggat ggtgttggtg tgagcattgg gagcgcgatc 800

cacactcagc tcaggagctc cgtgtacccc ttgctggcgg ccgtgggcag 850

tttgggccag gtcctgcagg tctccgtgca ccacctgcaa accttgaatg 900

ctacagtggt agagctgcag gccgggcagc aggacctgga gccagccatc 950

cgggaacacc gggaccgcct ccttgagctg ctgcaggagg ccaggtgcca 1000

gggagattgt gcaggggccc tgagctgggc ccgcaccctg gagctgggtg 1050

ctgacttcag ccaggtgccc tctgtggacc atgtcctgca ccagctaaaa 1100
ggtgtccccg aggccaactt ctccagcatg gtccaggagg agaacagcac 1150
cttcaacgcc cttccagccc tggctgccat gcagacatcc agcgtggtgc 1200
aagagctgaa gaaggcagtg gcccagcagc cggaaggggt gaggacactg 1250
gctgaagggt tcccgggctt ggaggcagct tcccgctggg cccaggcact 1300
gcaggaggtg gaggagagca gccgccccta cctgcaggag gtgcagagat 1350
acgagaccta caggtggatc gtgggctgcg tgctgtgctc cgtggtccta 1400
ttcgtggtgc tctgcaacct gctgggcctc aatctgggca tctggggcct 1450
gtctgccagg gacgacccca gccacccaga agccaagggc gaggctggag 1500
cccgcttcct catggcaggt gtgggcctca gcttcctctt tgctgcaccc 1550
ctcatcctcc tggtgttcgc caccttcctg gtgggtggca acgtgcagac 1600
gctggtgtgc cggagctggg agaacggcga gctctttgag tttgcagaca 1650
ccccagggaa cctgcccccg tccatgaacc tgtcgcaact tcttggcctg 1700
aggaagaaca tcagcatcca ccaagcctat cagcagtgca aggaaggggc 1750
agcgctctgg acagtcctgc agctcaacga ctcctacgac ctggaggagc 1800
acctggatat caaccagtat accaacaagc tacggcagga gttgcagagc 1850
ctgaaagtag acacacagag cctggacctg ctgagctcag ccgcccgccg 1900
ggacctggag gccctgcaga gcagtgggct tcagcgcatc cactaccccg 1950
acttcctcgt tcagatccag aggcccgtgg tgaagaccag catggagcag 2000
ctggcccagg agctgcaagg actggcccag gcccaagaca attctgtgct 2050
ggggcagcgg ctgcaggagg aggcccaagg actcagaaac cttcaccagg 2100
agaaggtcgt cccccagcag agccttgtgg caaagctcaa cctcagcgtc 2150
agggccctgg agtcctctgc cccgaatctc cagctggaga cctcagatgt 2200
cctagccaat gtcacctacc tgaaaggaga gctgcctgcc tgggcagcca 2250
ggatcctgag gaatgtgagt gagtgtttcc tggcccggga gatgggctac 2300
ttctcccagt acgtggcctg ggtgagagag gaggtgactc agcgcattgc 2350
cacctgccag cccctctccg gagccctgga caacagccgt gtgatcctgt 2400
gtgacatgat ggctgacccc tggaatgcct tctggttctg cctggcatgg 2450
tgcaccttct tcctgatccc cagcatcatc tttgccgtca agacctccaa 2500
atacttccgt cctatccgga aacgcctcag ctccaccagc tctgaggaga 2550
ctcagctctt ccacatcccc cgggttacct ccctgaagct gtagggcctt 2600
gtggggtgag gtgaccctga ggctgcctgt cctccccttt gatttagcct 2650

gggccacagg acttcggtag ctcttgcccc agagcccagg ctggcatcca 2700

ggcctggact gtccccagtt ccggcttacc tggccccacc ttgcctgctc 2750

ctttccaccc ctttctgctc acgaccccca tcattcacgc tcagaatcac 2800

atgggacttc tgtgcagctg cagagccagc aagtccctac aggtgtcacc 2850

cgttaccccc atgctggtgg catcctcaca ggaagagcct gttctccacc 2900

tgctggagcc tggaccctgg ggtgggacag aggcctcgtc caaccccact 2950

ccccttcccg tgtgtcttcc ccctgccaag cctccccctg ccaagcctcc 3000

ccctgcccct ctctgagccc ctcgcccccc acaccgtcct catctggcct 3050

ccccccctggc ccccacttcc ctcttatgcc cttcctggcc ctttgcttcc 3100

tcccttagtc ccctcttcac catatctcca ctgctacctt gctggcccca 3150

gagaccaccc tgcccaacca aaccactcag gtaacgccac taatcaggca 3200

ggggccacca tggcctaggt ctgggctggc tgcaggccct gcctcatggc 3250

ctctgagccc tccactgccc cagggccttg ggccctctgc agatctcatc 3300

caggatttat tgttgtccag tggggtgagg gaggcctgtc tgaaggccga 3350

gcctccctgc ctgcacccaa gttagaaatg ggggtaccag cacttagctt 3400

ctctctgagt gctggctccc aaggaaggga cctgggacct gggccacagt 3450

gggggcttgc ccttacctct tcagaaggaa gcatcttcca cagcccccac 3500

ccaactttct taggagtgat ctggtggcca gaacaggatt ttgcacggcc 3550

ccttttatcc tgcgcatgtg gcctagggtc atccccagcc catccctgtg 3600

tcagccctga gtgctggaca ctgcgttcca gaaatgagga agaggagaga 3650

gaagagatgg acagacctca gatccattaa agtgttctca cttcaaaaaa 3700

aaaaaaaaaa aaaaaaaaaa aaaaaaaaaa aaaa 3734

<210> 148
<211> 834
<212> PRT
<213> Homo Sapien

<400> 148

```
Met Lys His Thr Leu Ala Leu Leu Ala Pro Leu Leu Gly Leu Gly
  1               5                  10                  15

Leu Gly Leu Ala Leu Ser Gln Leu Ala Ala Gly Ala Thr Asp Cys
                 20                  25                  30

Lys Phe Leu Gly Pro Ala Glu His Leu Thr Phe Thr Pro Ala Ala
                 35                  40                  45

Arg Ala Arg Trp Leu Ala Pro Arg Val Arg Ala Pro Gly Leu Leu
                 50                  55                  60

Asp Ser Leu Tyr Gly Thr Val Arg Arg Phe Leu Ser Val Val Gln
```

```
                65                  70                  75
Leu Asn Pro Phe Pro Ser Glu Leu Val Lys Ala Leu Leu Asn Glu
                80                  85                  90
Leu Ala Ser Val Lys Val Asn Glu Val Val Arg Tyr Glu Ala Gly
                95                  100                 105
Tyr Val Val Cys Ala Val Ile Ala Gly Leu Tyr Leu Leu Leu Val
                110                 115                 120
Pro Thr Ala Gly Leu Cys Phe Cys Cys Cys Arg Cys His Arg Arg
                125                 130                 135
Cys Gly Gly Arg Val Lys Thr Glu His Lys Ala Leu Ala Cys Glu
                140                 145                 150
Arg Ala Ala Leu Met Val Phe Leu Leu Leu Thr Thr Leu Leu Leu
                155                 160                 165
Leu Ile Gly Val Val Cys Ala Phe Val Thr Asn Gln Arg Thr His
                170                 175                 180
Glu Gln Met Gly Pro Ser Ile Glu Ala Met Pro Glu Thr Leu Leu
                185                 190                 195
Ser Leu Trp Gly Leu Val Ser Asp Val Pro Gln Glu Leu Gln Ala
                200                 205                 210
Val Ala Gln Gln Phe Ser Leu Pro Gln Glu Gln Val Ser Glu Glu
                215                 220                 225
Leu Asp Gly Val Gly Val Ser Ile Gly Ser Ala Ile His Thr Gln
                230                 235                 240
Leu Arg Ser Ser Val Tyr Pro Leu Leu Ala Ala Val Gly Ser Leu
                245                 250                 255
Gly Gln Val Leu Gln Val Ser Val His His Leu Gln Thr Leu Asn
                260                 265                 270
Ala Thr Val Val Glu Leu Gln Ala Gly Gln Gln Asp Leu Glu Pro
                275                 280                 285
Ala Ile Arg Glu His Arg Asp Arg Leu Leu Glu Leu Leu Gln Glu
                290                 295                 300
Ala Arg Cys Gln Gly Asp Cys Ala Gly Ala Leu Ser Trp Ala Arg
                305                 310                 315
Thr Leu Glu Leu Gly Ala Asp Phe Ser Gln Val Pro Ser Val Asp
                320                 325                 330
His Val Leu His Gln Leu Lys Gly Val Pro Glu Ala Asn Phe Ser
                335                 340                 345
Ser Met Val Gln Glu Glu Asn Ser Thr Phe Asn Ala Leu Pro Ala
                350                 355                 360
Leu Ala Ala Met Gln Thr Ser Ser Val Val Gln Glu Leu Lys Lys
                365                 370                 375
Ala Val Ala Gln Gln Pro Glu Gly Val Arg Thr Leu Ala Glu Gly
                380                 385                 390
```

```
Phe Pro Gly Leu Glu Ala Ala Ser Arg Trp Ala Gln Ala Leu Gln
                395                 400                 405

Glu Val Glu Glu Ser Ser Arg Pro Tyr Leu Gln Glu Val Gln Arg
                410                 415                 420

Tyr Glu Thr Tyr Arg Trp Ile Val Gly Cys Val Leu Cys Ser Val
                425                 430                 435

Val Leu Phe Val Val Leu Cys Asn Leu Leu Gly Leu Asn Leu Gly
                440                 445                 450

Ile Trp Gly Leu Ser Ala Arg Asp Asp Pro Ser His Pro Glu Ala
                455                 460                 465

Lys Gly Glu Ala Gly Ala Arg Thr Leu Met Ala Gly Val Gly Leu
                470                 475                 480

Ser Phe Leu Phe Ala Ala Pro Leu Ile Leu Leu Val Phe Ala Thr
                485                 490                 495

Phe Leu Val Gly Gly Asn Val Gln Thr Leu Val Cys Arg Ser Trp
                500                 505                 510

Glu Asn Gly Glu Leu Phe Glu Phe Ala Asp Thr Pro Gly Asn Leu
                515                 520                 525

Pro Pro Ser Met Asn Leu Ser Gln Leu Leu Gly Leu Arg Lys Asn
                530                 535                 540

Ile Ser Ile His Gln Ala Tyr Gln Gln Cys Lys Glu Gly Ala Ala
                545                 550                 555

Leu Trp Thr Val Leu Gln Leu Asn Asp Ser Tyr Asp Leu Glu Glu
                560                 565                 570

His Leu Asp Ile Asn Gln Tyr Thr Asn Lys Leu Arg Gln Glu Leu
                575                 580                 585

Gln Ser Leu Lys Val Asp Thr Gln Ser Leu Asp Leu Leu Ser Ser
                590                 595                 600

Ala Ala Arg Arg Asp Leu Glu Ala Leu Gln Ser Ser Gly Leu Gln
                605                 610                 615

Arg Ile His Tyr Pro Asp Phe Leu Val Gln Ile Gln Arg Pro Val
                620                 625                 630

Val Lys Thr Ser Met Glu Gln Leu Ala Gln Glu Leu Gln Gly Leu
                635                 640                 645

Ala Gln Ala Gln Asp Asn Ser Val Leu Gly Gln Arg Leu Gln Glu
                650                 655                 660

Glu Ala Gln Gly Leu Arg Asn Leu His Gln Glu Lys Val Val Pro
                665                 670                 675

Gln Gln Ser Leu Val Ala Lys Leu Asn Leu Ser Val Arg Ala Leu
                680                 685                 690

Glu Ser Ser Ala Pro Asn Leu Gln Leu Glu Thr Ser Asp Val Leu
                695                 700                 705
```

```
Ala Asn Val Thr Tyr Leu Lys Gly Glu Leu Pro Ala Trp Ala Ala
                710                 715                 720

Arg Ile Leu Arg Asn Val Ser Glu Cys Phe Leu Ala Arg Glu Met
                725                 730                 735

Gly Tyr Phe Ser Gln Tyr Val Ala Trp Val Arg Glu Glu Val Thr
                740                 745                 750

Gln Arg Ile Ala Thr Cys Gln Pro Leu Ser Gly Ala Leu Asp Asn
                755                 760                 765

Ser Arg Val Ile Leu Cys Asp Met Met Ala Asp Pro Trp Asn Ala
                770                 775                 780

Phe Trp Phe Cys Leu Ala Trp Cys Thr Phe Phe Leu Ile Pro Ser
                785                 790                 795

Ile Ile Phe Ala Val Lys Thr Ser Lys Tyr Phe Arg Pro Ile Arg
                800                 805                 810

Lys Arg Leu Ser Ser Thr Ser Ser Glu Glu Thr Gln Leu Phe His
                815                 820                 825

Ile Pro Arg Val Thr Ser Leu Lys Leu
                830
```

<210> 149
<211> 804
<212> DNA
<213> Homo Sapien

<400> 149

```
cacagctccc ttcccaggac gtgaaaatct gccttctcac catgaggctt 50

ctagtccttt ccagcctgct ctgtatcctg cttctctgct tctccatctt 100

ctccacagaa gggaagaggc gtcctgccaa ggcctggtca ggcaggagaa 150

ccaggctctg ctgccaccga gtccctagcc ccaactcaac aaacctgaaa 200

ggacatcatg tgaggctctg taaaccatgc aagcttgagc cagagccccg 250

cctttgggtg gtgcctgggg cactcccaca ggtgtagcac tcccaaagca 300

agactccaga cagcggagaa cctcatgcct ggcacctgag gtacccagca 350

gcctcctgtc tcccctttca gccttcacag cagtgagctg caatgttgga 400

gggcttcatc tcgggctgca aggaccctgg gaaagttcca gaactccacg 450

tccttgtctc aattgtgcca tcaactttca gagctatcat gagccaacct 500

caccccacag ggcctcagtc gccaccatgt gggcctctcc agtgcaaacc 550

accgagcatt ccaccatgac cggtcacagc tacaaatcca gagaccatca 600

atcctgctag agtgcagggt ggcaagcacc caagggtggc tgaccaagac 650

tgcagagtct cctccatctt caggtccatt cagcctcctg gcatttaact 700

accagcatcc agtggtcccc aaggaatccc ttcctagcct cctgacatga 750

gtctgctgga aagagcatcc aaacaaacaa gtaataaata aataaataaa 800
```

ctca 804

<210> 150
<211> 81
<212> PRT
<213> Homo Sapien

<400> 150
Met Arg Leu Leu Val Leu Ser Ser Leu Leu Cys Ile Leu Leu Leu
1 5 10 15

Cys Phe Ser Ile Phe Ser Thr Glu Gly Lys Arg Arg Pro Ala Lys
20 25 30

Ala Trp Ser Gly Arg Arg Thr Arg Leu Cys Cys His Arg Val Pro
35 40 45

Ser Pro Asn Ser Thr Asn Leu Lys Gly His His Val Arg Leu Cys
50 55 60

Lys Pro Cys Lys Leu Glu Pro Glu Pro Arg Leu Trp Val Val Pro
65 70 75

Gly Ala Leu Pro Gln Val
80

<210> 151
<211> 2164
<212> DNA
<213> Homo Sapien

<400> 151
caccggaggg cacgcagctg acggagctgc gctgcgttcg cctcgtttgc 50

ctcgcgccct ccactggagc tgttcgcgcc tcccggctcc caccgcagcc 100

cacccggcag aggagtcgct accagcgccc agtgcgctct gtcagtccgc 150

aaactccttg ccgcccgccc cgggctgggc accaaatacc aggctaccat 200

ggtctacaag actctcttcg ctctttgcat cttaactgca ggatggaggg 250

tacagagtct gcctacatca gctcctttgt ctgtttctct tccgacaaac 300

attgtaccac cgaccaccat ctggactagc tctccacaaa acactgatgc 350

agacactgcc tccccatcca acggcactca caacaactcg gtgctcccag 400

ttacagcatc agccccaaca tctctgcttc ctaagaacat ttccatagag 450

tccagagaag aggagatcac cagcccaggt tcgaattggg aaggcacaaa 500

cacagacccc tcaccttctg ggttctcgtc aacaagcggt ggagtccact 550

taacaaccac gttggaggaa cacagctcgg gcactcctga agcaggcgtg 600

gcagctacac tgtcgcagtc cgctgctgag cctcccacac tcatctcccc 650

tcaagctcca gcctcatcac cctcatccct atcaacctca ccacctgagg 700

tcttttctgc ctccgttact accaaccata gctccactgt gaccagcacc 750

caacccactg gagctccaac tgcaccagag tccccgacag aggagtccag 800

ctctgaccac acacccactt cacatgccac agctgagcca gtgccccagg 850

agaaaacacc cccaacaact gtgtcaggca aagtgatgtg tgagctcata 900

gacatggaga ccaccaccac ctttcccagg gtgatcatgc aggaagtaga 950

acatgcatta agttcaggca gcatcgccgc cattaccgtg acagtcattg 1000

ccgtggtgct gctggtgttt ggagttgcag cctacctaaa aatcaggcat 1050

tcctcctatg gaagactttt ggacgaccat gactacgggt cctggggaaa 1100

ctacaacaac cctctgtacg atgactccta acaatggaat atggcctggg 1150

atgaggatta actgttcttt atttataagt gcttatccag tagaattaat 1200

aagtacctga tgcgcattga acgacaatct taagccctgt tttgttggta 1250

tggttgtttt tgttttcctc cctctcctct ggctgctaca acttcccctt 1300

tctggtacaa gaagaaccat tctttaaagg tgagtggagg ctgatttgca 1350

gctgaagtgg gccagccttg caccagccag gccagaccac catggtgaag 1400

gcttctttcc ccactgcagg acccactttg agaaggatcg aggaggagga 1450

tttgggttgt tttgttaggg gttactttca ggggaacatt tcatttgtgt 1500

tatttcttaa acttctattt aggaaattac attaagtatt aatgagggga 1550

aaggaaatga gctctacgag gatttcacct tgcatgggag agagcagggt 1600

tttctcagat tcctttttaa tctctattta tctggttgtt tctgacagga 1650

tgctgcctgc ttggctctac gagctggaaa gcagcttctt agctgcctaa 1700

ttaatgaaag atgaaaatag gaagtgccct ggagggggcc agcaggtcac 1750

ggggcagaat ctctcaggtt gctgtgggat ctcagtgtgc ccctacctgt 1800

tctcccctcc aggccacctg tctctgtaaa ggatgtctgc tctgttcaaa 1850

aggcagctgg gatcccagcc cacaagtgat cagcagagtt gcatttccaa 1900

agaaaaaggc tatgagatga gctgagttat agagagaaag ggagaggcat 1950

gtacggtgtg gggaagtgga agagaagctg gcgggggaga aggaggctaa 2000

cctgcactga gtacttcatt aggacaagtg agaatcagct attgataatg 2050

gccagagata tccacagctt ggaggagccc agagactgtt tgctttatac 2100

ccacacagca actggtccac tgctttactg tctgttggat aatggctgta 2150

aaatgtttaa aaac 2164

<210> 152
<211> 310
<212> PRT
<213> Homo Sapien

<400> 152
Met Val Tyr Lys Thr Leu Phe Ala Leu Cys Ile Leu Thr Ala Gly

```
1               5                   10                  15
Trp Arg Val Gln Ser Leu Pro Thr Ser Ala Pro Leu Ser Val Ser
                20                  25                  30
Leu Pro Thr Asn Ile Val Pro Pro Thr Thr Ile Trp Thr Ser Ser
                35                  40                  45
Pro Gln Asn Thr Asp Ala Asp Thr Ala Ser Pro Ser Asn Gly Thr
                50                  55                  60
His Asn Asn Ser Val Leu Pro Val Thr Ala Ser Ala Pro Thr Ser
                65                  70                  75
Leu Leu Pro Lys Asn Ile Ser Ile Glu Ser Arg Glu Glu Glu Ile
                80                  85                  90
Thr Ser Pro Gly Ser Asn Trp Glu Gly Thr Asn Thr Asp Pro Ser
                95                  100                 105
Pro Ser Gly Phe Ser Ser Thr Ser Gly Gly Val His Leu Thr Thr
                110                 115                 120
Thr Leu Glu Glu His Ser Ser Gly Thr Pro Glu Ala Gly Val Ala
                125                 130                 135
Ala Thr Leu Ser Gln Ser Ala Ala Glu Pro Pro Thr Leu Ile Ser
                140                 145                 150
Pro Gln Ala Pro Ala Ser Ser Pro Ser Ser Leu Ser Thr Ser Pro
                155                 160                 165
Pro Glu Val Phe Ser Ala Ser Val Thr Thr Asn His Ser Ser Thr
                170                 175                 180
Val Thr Ser Thr Gln Pro Thr Gly Ala Pro Thr Ala Pro Glu Ser
                185                 190                 195
Pro Thr Glu Glu Ser Ser Ser Asp His Thr Pro Thr Ser His Ala
                200                 205                 210
Thr Ala Glu Pro Val Pro Gln Glu Lys Thr Pro Pro Thr Thr Val
                215                 220                 225
Ser Gly Lys Val Met Cys Glu Leu Ile Asp Met Glu Thr Thr Thr
                230                 235                 240
Thr Phe Pro Arg Val Ile Met Gln Glu Val Glu His Ala Leu Ser
                245                 250                 255
Ser Gly Ser Ile Ala Ala Ile Thr Val Thr Val Ile Ala Val Val
                260                 265                 270
Leu Leu Val Phe Gly Val Ala Ala Tyr Leu Lys Ile Arg His Ser
                275                 280                 285
Ser Tyr Gly Arg Leu Leu Asp Asp His Asp Tyr Gly Ser Trp Gly
                290                 295                 300
Asn Tyr Asn Asn Pro Leu Tyr Asp Asp Ser
                305                 310
```

<210> 153
<211> 755

<212> DNA
<213> Homo Sapien

<220>
<221> unsure
<222> 67
<223> unknown base

<400> 153
acgtcactgt cttgaagcag cagtagcctg ggaagtgagg caggaggaat 50

tgagaggcag gaagggngct ggagacacag ctgagcctgg aaatgagagt 100

gggcatcgcc gtggtcatca tgactcctct gcggcgtggt caccatgttg 150

gttcactgtg ttgggctctt attgacgggt ctcctgctag gcctgacctt 200

gggtgccgga gccctgctgg cttctgagcc tatctaccaa ccaccttcag 250

cctgggtgcc agctgggggg ctggtggggc tggcgctgct gggagccctg 300

ctcacacttc ggtggccacg tccattcaca gttctgggca caaccctgct 350

gggttctgca gtgcttgtgg cctgtgttga ctacttcctg gaggggctgg 400

cactggggag ttggctgggc caacgcctgc agacacttcc agccttgcct 450

tctctctgct gatatagctg ggtcttactg gggatctggc cagccttggg 500

ggcccttgga gccctggccc agtggaagct cgtgcctgag gaacatggag 550

gccacgctaa tgggtctgtt cctggtttcc cagatgcata aaggaagaca 600

tatccctccc ctgggcagca aggctacaat gggagggagg gagaacatgg 650

gagcatgtga ataaaatggc attaaatact gaaaaaaaaa aaaaaaaaaa 700

aaaaaaaaaa aaaaaaaaaa aaaaaaaaaa aaaaaaaaaa aaaaaaaaaa 750

aaaaa 755

<210> 154
<211> 105
<212> PRT
<213> Homo Sapien

<400> 154
Met Leu Val His Cys Val Gly Leu Leu Leu Thr Gly Leu Leu Leu
1 5 10 15

Gly Leu Thr Leu Gly Ala Gly Ala Leu Leu Ala Ser Glu Pro Ile
20 25 30

Tyr Gln Pro Pro Ser Ala Trp Val Pro Ala Gly Gly Leu Val Gly
35 40 45

Leu Ala Leu Leu Gly Ala Leu Leu Thr Leu Arg Trp Pro Arg Pro
50 55 60

Phe Thr Val Leu Gly Thr Thr Leu Leu Gly Ser Ala Val Leu Val
65 70 75

Ala Cys Val Asp Tyr Phe Leu Glu Gly Leu Ala Leu Gly Ser Trp
80 85 90

Leu Gly Gln Arg Leu Gln Thr Leu Pro Ala Leu Pro Ser Leu Cys
95 100 105

<210> 155
<211> 1825
<212> DNA
<213> Homo Sapien

<400> 155
tgcaattaaa ggagtcgggt ctctaactgt tgatctgttt ttttcccttc 50

tgagcaatgg agcttaccat ctttatcctg agactggcca tttacatcct 100

gacatttccc ttgtacctgc tgaactttct gggcttgtgg agctggatat 150

gcaaaaaatg gttcccctac ttcttggtga ggttcactgt gatatacaac 200

gaacagatgg caagcaagaa gcgggagctc ttcagtaacc tgcaggagtt 250

tgcgggcccc tccgggaaac tctccctgct ggaagtgggc tgtggcacgg 300

gggccaactt caagttctac ccacctgggt gcagggtgac ctgtattgac 350

cccaacccca actttgagaa gtttttgatc aagagcattg cagagaaccg 400

acacctgcag tttgagcgct ttgtggtagc tgccggggag aacatgcacc 450

aggtggctga tggctctgtg gatgtggtgg tctgcaccct ggtgctgtgc 500

tctgtgaaga accaggagcg gattctccgc gaggtgtgca gagtgctgag 550

accgggaggg gctttctatt tcatggagca tgtggcagct gagtgttcga 600

cttggaatta cttctggcaa caagtcctgg atcctgcctg gcaccttctg 650

tttgatgggt gcaacctgac cagagagagc tggaaggccc tggagcgggc 700

cagcttctct aagctgaagc tgcagcacat ccaggcccca ctgtcctggg 750

agttggtgcg ccctcatatc tatggatatg ctgtgaaata gtgtgagctg 800

gcagttaaga gctgaatggc tcaaagaatt taaagcttca gttttacatt 850

taaaatgcta agtgggagaa gagaaacctt ttttttgggg ggcggttttt 900

ttggtttgtt gttggttttt tttttttttt tggcaggaga atctcttgaa 950

cccagaaggc gaaggttgca gtgaaccgag atcatgccat tgtactctag 1000

cctgggtgac aagagcaaga ctccgtctca aaaaaaaaaa aaaaaaaaaa 1050

aagaagtaga gacagggaga cggggtctca ctgtgttgcc taggccggtc 1100

ttgaactcct gggctcaagt gattctccca ccttgacctc ctaaattgtt 1150

gggattacag gtgtgagaca gtgcacctgg ccgaaatagc tcaagtttct 1200

gaaaaacaaa tctgaatcta tttgttattc ttagcgtcac tggtctggct 1250

ttcagaatta acatacaagg ttgccacacc tagttctgcc cagctttatg 1300

tcttttattc cagtattcca ccaaagtttg ttttcctgca ttccagttct 1350

caagtcttaa gataaagatt gtacttgaca gtttagtata tccataaaac 1400

tatttgaggt ggttaaggtt cttgggttca ttttccttaa tactttgctg 1450

aatattgtag attgtaggca atgaaaaagt ctactaaatt aggaaaacct 1500

tgaataatta ggtatcctag gtaagagccc ctaaacatca agcaatctgt 1550

gagtctgtaa agaaataaat attttttgga ttattcttat ctaattccac 1600

ccctgttgga agatgatttc tttgttcttt gcaactatgg aagctgtgaa 1650

aatcatcaca agtgcctctg aaagcgagtg ttaggttggt tagagggttt 1700

aatattttct gcaatggttt gtaggaattt taataaatgt agtatatttt 1750

ctgagatgat tttgtaaaag tactatttta aatatcaaat caaccaataa 1800

attcacattt gtgttaggaa caaaa 1825

<210> 156
<211> 244
<212> PRT
<213> Homo Sapien

<400> 156
Met Glu Leu Thr Ile Phe Ile Leu Arg Leu Ala Ile Tyr Ile Leu
1 5 10 15

Thr Phe Pro Leu Tyr Leu Leu Asn Phe Leu Gly Leu Trp Ser Trp
20 25 30

Ile Cys Lys Lys Trp Phe Pro Tyr Phe Leu Val Arg Phe Thr Val
35 40 45

Ile Tyr Asn Glu Gln Met Ala Ser Lys Lys Arg Glu Leu Phe Ser
50 55 60

Asn Leu Gln Glu Phe Ala Gly Pro Ser Gly Lys Leu Ser Leu Leu
65 70 75

Glu Val Gly Cys Gly Thr Gly Ala Asn Phe Lys Phe Tyr Pro Pro
80 85 90

Gly Cys Arg Val Thr Cys Ile Asp Pro Asn Pro Asn Phe Glu Lys
95 100 105

Phe Leu Ile Lys Ser Ile Ala Glu Asn Arg His Leu Gln Phe Glu
110 115 120

Arg Phe Val Val Ala Ala Gly Glu Asn Met His Gln Val Ala Asp
125 130 135

Gly Ser Val Asp Val Val Val Cys Thr Leu Val Leu Cys Ser Val
140 145 150

Lys Asn Gln Glu Arg Ile Leu Arg Glu Val Cys Arg Val Leu Arg
155 160 165

Pro Gly Gly Ala Phe Tyr Phe Met Glu His Val Ala Ala Glu Cys
170 175 180

Ser Thr Trp Asn Tyr Phe Trp Gln Gln Val Leu Asp Pro Ala Trp
185 190 195

His Leu Leu Phe Asp Gly Cys Asn Leu Thr Arg Glu Ser Trp Lys
200 205 210

Ala Leu Glu Arg Ala Ser Phe Ser Lys Leu Lys Leu Gln His Ile
215 220 225

Gln Ala Pro Leu Ser Trp Glu Leu Val Arg Pro His Ile Tyr Gly
230 235 240

Tyr Ala Val Lys

<210> 157
<211> 1328
<212> DNA
<213> Homo Sapien

<400> 157
ccgctgagat gtacgaactt ccggttctcc gggcagctgc cactgctgta 50

gcttctgcca cctgccacga ccgggcctct ccctggcgtt tggtcacctc 100

tgcttcattc tccaccgcgc ctatggtccc tcttggagcc agcgtggcgg 150

gcctggcggc tcccgggtgg tgagagagcg gtccgggaac gatgaaggcc 200

tcgcagtgct gctgctgtct cagccacctc ttggcttccg tcctcctcct 250

gctgttgctg cctgaactaa gcgggcccct ggcagtcctg ctgcaggcag 300

ccgaggccgc gccaggtctt gggcctcctg accctagacc acggacatta 350

ccgccgctgc caccgggccc taccccctgcc cagcagccgg gccgtggtct 400

ggctgaagct gcggggccgc ggggctccga gggaggcaat ggcagcaacc 450

ctgtggccgg gcttgagacg gacgatcacg gagggaaggc cggggaaggc 500

tcggtgggtg gcggccttgc tgtgagcccc aaccctggcg acaagcccat 550

gacccagcgg gccctgaccg tgttgatggt ggtgagcggc gcggtgctgg 600

tgtacttcgt ggtcaggacg gtcaggatga gaagaagaaa ccgaaagact 650

aggagatatg gagttttgga cactaacata gaaaatatgg aattgacacc 700

tttagaacag gatgatgagg atgatgacaa cacgttgttt gatgccaatc 750

atcctcgaag ataagaatgt gccttttgat gaaagaactt tatctttcta 800

caatgaagag tggaatttct atgtttaagg aataagaagc cactatatca 850

atgttggggg ggtatttaag ttacatatat tttaacaacc tttaatttgc 900

tgttgcaata aataccgtat ccttttatta tatctttata tgtatagaag 950

tactctatta atgggctcag agatgttggg gataaagtat actgtaataa 1000

tttatctgtt tgaaaattac tataaaacgg tgttttctgg tcggtttttg 1050

tttcctgctt accatatgat tgtaaattgt tttatgtatt aatcagttaa 1100

tgctaattat ttttgctgat gtcatatgtt aaagagctat aaattccaac 1150

aaccaactgg tgtgtaaaaa taatttaaaa tttcctttac tgaaaggtat 1200

ttcccatttt tgtggggaaa agaagccaaa tttattactt tgtgttgggg 1250

tttttaaaat attaagaaat gtctaagtta ttgtttgcaa aacaataaat 1300

atgattttaa attctcttaa aaaaaaaa 1328

<210> 158
<211> 190
<212> PRT
<213> Homo Sapien

<400> 158
Met Lys Ala Ser Gln Cys Cys Cys Cys Leu Ser His Leu Leu Ala
1 5 10 15

Ser Val Leu Leu Leu Leu Leu Leu Pro Glu Leu Ser Gly Pro Leu
20 25 30

Ala Val Leu Leu Gln Ala Ala Glu Ala Ala Pro Gly Leu Gly Pro
35 40 45

Pro Asp Pro Arg Pro Arg Thr Leu Pro Pro Leu Pro Pro Gly Pro
50 55 60

Thr Pro Ala Gln Gln Pro Gly Arg Gly Leu Ala Glu Ala Ala Gly
65 70 75

Pro Arg Gly Ser Glu Gly Gly Asn Gly Ser Asn Pro Val Ala Gly
80 85 90

Leu Glu Thr Asp Asp His Gly Gly Lys Ala Gly Glu Gly Ser Val
95 100 105

Gly Gly Gly Leu Ala Val Ser Pro Asn Pro Gly Asp Lys Pro Met
110 115 120

Thr Gln Arg Ala Leu Thr Val Leu Met Val Val Ser Gly Ala Val
125 130 135

Leu Val Tyr Phe Val Val Arg Thr Val Arg Met Arg Arg Arg Asn
140 145 150

Arg Lys Thr Arg Arg Tyr Gly Val Leu Asp Thr Asn Ile Glu Asn
155 160 165

Met Glu Leu Thr Pro Leu Glu Gln Asp Asp Glu Asp Asp Asp Asn
170 175 180

Thr Leu Phe Asp Ala Asn His Pro Arg Arg
185 190

<210> 159
<211> 2167
<212> DNA
<213> Homo Sapien

<400> 159
gctgcaggcg gcgacggcta caccatgggc cggctgctgc gggccgcccg 50

gctgccgccg ctgctttcgc cgctgctgct tctgctggtt gggggagcgt 100

tcctgggtgc ctgtgtggct gggtctgatg agcctggccc agagggcctc 150
acctccacct ccctgctaga cctcctgctg cccactggct tggagccact 200
ggactcagag gagcctagtg agaccatggg cctgggagct gggctgggag 250
cctctggctc aggcttcccc agcgaagaga atgaagagtc tcggattctg 300
cagccaccac agtacttctg ggaagaggag gaagagctga atgactcaag 350
tctggacctg ggacccactg cagattatgt tttcctgac ttaactgaga 400
aggcaggttc cattgaagac actagccagg ctcaagagct gccaaacctc 450
ccctctccct tgcccaagat gaatctggtt gagcctccct ggcatatgcc 500
tcccagagag gaggaagaag aggaagagga agaggaggag agggagaagg 550
aagaggtaga gaaacaagag gaggaggaag aggaggagct gctccctgtg 600
aatggatccc aagaagaagc caagcctcag gtccgtgact tttctctcac 650
cagcagcagc cagaccccag gggccaccaa aagcaggcat gaagactccg 700
gggaccaggc ctcatcaggt gtggaggtgg agagcagcat ggggcccagc 750
ttgctgctgc cttcagtcac cccaactaca gtgactccgg gggaccagga 800
ctccaccagc caagaggcag aggccacagt gctgccagct gcagggcttg 850
gggtagagtt cgaggctcct caggaagcaa gcgaggaagc cactgcagga 900
gcagctggtt tgtctggcca gcacgaggag gtgccggcct tgccttcatt 950
ccctcaaacc acagctccca gtggggccga gcacccagat gaagatcccc 1000
ttggctctag aacctcagcc tcttccccac tggcccctgg agacatggaa 1050
ctgacacctt cctctgctac cttgggacaa gaagatctca accagcagct 1100
cctagaaggg caggcagctg aagctcaatc caggataccc tgggattcta 1150
cgcaggtgat ctgcaaggac tggagcaatc tggctgggaa aaactacatc 1200
attctgaaca tgacagagaa catagactgt gaggtgttcc ggcagcaccg 1250
ggggccacag ctcctggccc tggtggaaga ggtgctgccc cgccatggca 1300
gtggccacca tggggcctgg cacatctctc tgagcaagcc cagcgagaag 1350
gagcagcacc ttctcatgac actggtgggc gagcaggggg tggtgcccac 1400
tcaagatgtc ctttccatgc tgggtgacat ccgcaggagc ctggaggaga 1450
ttggcatcca gaactattcc acaaccagca gctgccaggc gcgggccagc 1500
caggtgcgca gcgactacgg cacgctcttc gtggtgctgg tggtcattgg 1550
ggccatctgc atcatcatca ttgcgcttgg cctgctctac aactgctggc 1600
agcgccggct gcccaagctc aagcacgtgt cgcacggcga ggagctgcgc 1650
ttcgtggaga acggctgcca cgacaacccc acgctggacg tggccagcga 1700

cagccagtcg gagatgcagg agaagcaccc cagcctgaac ggcggcgggg 1750

ccctcaacgg cccggggagc tggggggcgc tcatgggggg caagcgggac 1800

cccgaggact cggacgtgtt cgaggaggac acgcacctgt gagcgcagcc 1850

gaggcgcagg ccgagtgggc cgccaggacc aagcgaggtg gaccccgaaa 1900

cggacggccc ggagcccgca ccagccccgc gcctacccgg gccgcccccg 1950

cggcctggcc ctcggcgcgg gctccttccc gcttcccccg acttcacacg 2000

gcggcttcgg accaactccc tcactcccgc ccgaggggca ggcctcaaag 2050

cccgccttgg ccccgctttc ccgcccctga accccggccc cgcgggcggc 2100

gggcggcgct tcctgcgccc cgggactcaa ttaaacccgc ccggagacca 2150

cgccgggccc agcaaaa 2167

<210> 160
<211> 605
<212> PRT
<213> Homo Sapien

<400> 160
Met Gly Arg Leu Leu Arg Ala Ala Arg Leu Pro Pro Leu Leu Ser
1 5 10 15

Pro Leu Leu Leu Leu Leu Val Gly Gly Ala Phe Leu Gly Ala Cys
20 25 30

Val Ala Gly Ser Asp Glu Pro Gly Pro Glu Gly Leu Thr Ser Thr
35 40 45

Ser Leu Leu Asp Leu Leu Leu Pro Thr Gly Leu Glu Pro Leu Asp
50 55 60

Ser Glu Glu Pro Ser Glu Thr Met Gly Leu Gly Ala Gly Leu Gly
65 70 75

Ala Ser Gly Ser Gly Phe Pro Ser Glu Glu Asn Glu Glu Ser Arg
80 85 90

Ile Leu Gln Pro Pro Gln Tyr Phe Trp Glu Glu Glu Glu Glu Leu
95 100 105

Asn Asp Ser Ser Leu Asp Leu Gly Pro Thr Ala Asp Tyr Val Phe
110 115 120

Pro Asp Leu Thr Glu Lys Ala Gly Ser Ile Glu Asp Thr Ser Gln
125 130 135

Ala Gln Glu Leu Pro Asn Leu Pro Ser Pro Leu Pro Lys Met Asn
140 145 150

Leu Val Glu Pro Pro Trp His Met Pro Pro Arg Glu Glu Glu Glu
155 160 165

Glu Glu Glu Glu Glu Glu Glu Arg Glu Lys Glu Glu Val Glu Lys
170 175 180

Gln Glu Glu Glu Glu Glu Glu Glu Leu Leu Pro Val Asn Gly Ser
185 190 195

```
Gln Glu Glu Ala Lys Pro Gln Val Arg Asp Phe Ser Leu Thr Ser
                200                 205                 210

Ser Ser Gln Thr Pro Gly Ala Thr Lys Ser Arg His Glu Asp Ser
                215                 220                 225

Gly Asp Gln Ala Ser Ser Gly Val Glu Val Glu Ser Ser Met Gly
                230                 235                 240

Pro Ser Leu Leu Leu Pro Ser Val Thr Pro Thr Thr Val Thr Pro
                245                 250                 255

Gly Asp Gln Asp Ser Thr Ser Gln Glu Ala Glu Ala Thr Val Leu
                260                 265                 270

Pro Ala Ala Gly Leu Gly Val Glu Phe Glu Ala Pro Gln Glu Ala
                275                 280                 285

Ser Glu Glu Ala Thr Ala Gly Ala Ala Gly Leu Ser Gly Gln His
                290                 295                 300

Glu Glu Val Pro Ala Leu Pro Ser Phe Pro Gln Thr Thr Ala Pro
                305                 310                 315

Ser Gly Ala Glu His Pro Asp Glu Asp Pro Leu Gly Ser Arg Thr
                320                 325                 330

Ser Ala Ser Ser Pro Leu Ala Pro Gly Asp Met Glu Leu Thr Pro
                335                 340                 345

Ser Ser Ala Thr Leu Gly Gln Glu Asp Leu Asn Gln Gln Leu Leu
                350                 355                 360

Glu Gly Gln Ala Ala Glu Ala Gln Ser Arg Ile Pro Trp Asp Ser
                365                 370                 375

Thr Gln Val Ile Cys Lys Asp Trp Ser Asn Leu Ala Gly Lys Asn
                380                 385                 390

Tyr Ile Ile Leu Asn Met Thr Glu Asn Ile Asp Cys Glu Val Phe
                395                 400                 405

Arg Gln His Arg Gly Pro Gln Leu Leu Ala Leu Val Glu Glu Val
                410                 415                 420

Leu Pro Arg His Gly Ser Gly His His Gly Ala Trp His Ile Ser
                425                 430                 435

Leu Ser Lys Pro Ser Glu Lys Glu Gln His Leu Leu Met Thr Leu
                440                 445                 450

Val Gly Glu Gln Gly Val Val Pro Thr Gln Asp Val Leu Ser Met
                455                 460                 465

Leu Gly Asp Ile Arg Arg Ser Leu Glu Glu Ile Gly Ile Gln Asn
                470                 475                 480

Tyr Ser Thr Thr Ser Ser Cys Gln Ala Arg Ala Ser Gln Val Arg
                485                 490                 495

Ser Asp Tyr Gly Thr Leu Phe Val Val Leu Val Val Ile Gly Ala
                500                 505                 510
```

```
Ile Cys Ile Ile Ile Ile Ala Leu Gly Leu Leu Tyr Asn Cys Trp
                515                 520                 525

Gln Arg Arg Leu Pro Lys Leu Lys His Val Ser His Gly Glu Glu
                530                 535                 540

Leu Arg Phe Val Glu Asn Gly Cys His Asp Asn Pro Thr Leu Asp
                545                 550                 555

Val Ala Ser Asp Ser Gln Ser Glu Met Gln Glu Lys His Pro Ser
                560                 565                 570

Leu Asn Gly Gly Gly Ala Leu Asn Gly Pro Gly Ser Trp Gly Ala
                575                 580                 585

Leu Met Gly Gly Lys Arg Asp Pro Glu Asp Ser Asp Val Phe Glu
                590                 595                 600

Glu Asp Thr His Leu
                605
```

<210> 161
<211> 1376
<212> DNA
<213> Homo Sapien

<400> 161

```
ccagggcgga gcgcagctgc gccgggcttg ggcgcctggg gccgccgctc 50

cccaccgtcg ttttccccac cgaggccgag gcgtcccgga gtcatggccg 100

gcctgaactg cggggtctct atcgcactgc taggggttct gctgctgggt 150

gcggcgcgcc tgccgcgcgg ggcagaagct tttgagattg ctctgccacg 200

agaaagcaac attacagttc tcataaagct ggggaccccg actctgctgg 250

caaaaccctg ttacatcgtc atttctaaaa gacatataac catgttgtcc 300

atcaagtctg gagaaagaat agtctttacc tttagctgcc agagtcctga 350

gaatcacttt gtcatagaga tccagaaaaa tattgactgt atgtcaggcc 400

catgtccttt tggggaggtt cagcttcagc cctcgacatc gttgttgcct 450

accctcaaca gaactttcat ctgggatgtc aaagctcata agagcatcgg 500

tttagagctg cagttttcca tccctcgcct gaggcagatc ggtccgggtg 550

agagctgccc agacggagtc actcactcca tcagcggccg aatcgatgcc 600

accgtggtca ggatcggaac cttctgcagc aatggcactg tgtcccggat 650

caagatgcaa gaaggagtga aaatggcctt acacctccca tggttccacc 700

ccagaaatgt ctccggcttc agcattgcaa accgctcatc tataaaacgt 750

ctgtgcatca tcgagtctgt gtttgagggt gaaggctcag caaccctgat 800

gtctgccaac tacccagaag gcttccctga ggatgagctc atgacgtggc 850

agtttgtcgt tcctgcacac ctgcgggcca gcgtctcctt cctcaacttc 900

aacctctcca actgtgagag gaaggaggag cgggttgaat actacatccc 950
```

gggctccacc accaaccccg aggtgttcaa gctggaggac aagcagcctg 1000

ggaacatggc ggggaacttc aacctctctc tgcaaggctg tgaccaagat 1050

gcccaaagtc cagggatcct ccggctgcag ttccaagttt tggtccaaca 1100

tccacaaaat gaaagcagtg agtgagcccc actttccttt ttcttcctcc 1150

tccagcacct tcgttgtttc ctgggtagtc tgcctgggtg aggctccctt 1200

cctgtttctc atctgtggct tctgaaacac ttagactctg gacccagcaa 1250

gagtttcagg aagtgggttg ctaggcagtt agacaggctt gttggtgaac 1300

acccggtatg tagttccatt tcagcacaat aaaaagaaat cttgcattca 1350

agatgctaaa ttgtttttaa cgaaaa 1376

<210> 162
<211> 343
<212> PRT
<213> Homo Sapien

<400> 162
Met Ala Gly Leu Asn Cys Gly Val Ser Ile Ala Leu Leu Gly Val
1 5 10 15

Leu Leu Leu Gly Ala Ala Arg Leu Pro Arg Gly Ala Glu Ala Phe
20 25 30

Glu Ile Ala Leu Pro Arg Glu Ser Asn Ile Thr Val Leu Ile Lys
35 40 45

Leu Gly Thr Pro Thr Leu Leu Ala Lys Pro Cys Tyr Ile Val Ile
50 55 60

Ser Lys Arg His Ile Thr Met Leu Ser Ile Lys Ser Gly Glu Arg
65 70 75

Ile Val Phe Thr Phe Ser Cys Gln Ser Pro Glu Asn His Phe Val
80 85 90

Ile Glu Ile Gln Lys Asn Ile Asp Cys Met Ser Gly Pro Cys Pro
95 100 105

Phe Gly Glu Val Gln Leu Gln Pro Ser Thr Ser Leu Leu Pro Thr
110 115 120

Leu Asn Arg Thr Phe Ile Trp Asp Val Lys Ala His Lys Ser Ile
125 130 135

Gly Leu Glu Leu Gln Phe Ser Ile Pro Arg Leu Arg Gln Ile Gly
140 145 150

Pro Gly Glu Ser Cys Pro Asp Gly Val Thr His Ser Ile Ser Gly
155 160 165

Arg Ile Asp Ala Thr Val Val Arg Ile Gly Thr Phe Cys Ser Asn
170 175 180

Gly Thr Val Ser Arg Ile Lys Met Gln Glu Gly Val Lys Met Ala
185 190 195

```
Leu His Leu Pro Trp Phe His Pro Arg Asn Val Ser Gly Phe Ser
                200                 205                 210

Ile Ala Asn Arg Ser Ser Ile Lys Arg Leu Cys Ile Ile Glu Ser
                215                 220                 225

Val Phe Glu Gly Glu Gly Ser Ala Thr Leu Met Ser Ala Asn Tyr
                230                 235                 240

Pro Glu Gly Phe Pro Glu Asp Glu Leu Met Thr Trp Gln Phe Val
                245                 250                 255

Val Pro Ala His Leu Arg Ala Ser Val Ser Phe Leu Asn Phe Asn
                260                 265                 270

Leu Ser Asn Cys Glu Arg Lys Glu Glu Arg Val Glu Tyr Tyr Ile
                275                 280                 285

Pro Gly Ser Thr Thr Asn Pro Glu Val Phe Lys Leu Glu Asp Lys
                290                 295                 300

Gln Pro Gly Asn Met Ala Gly Asn Phe Asn Leu Ser Leu Gln Gly
                305                 310                 315

Cys Asp Gln Asp Ala Gln Ser Pro Gly Ile Leu Arg Leu Gln Phe
                320                 325                 330

Gln Val Leu Val Gln His Pro Gln Asn Glu Ser Ser Glu
                335                 340
```

<210> 163
<211> 1968
<212> DNA
<213> Homo Sapien

<400> 163

```
caaccacaca cctggggaat tgctggcctg acttctgacc cctgactcct 50

catacccttc ctccagagca tgacatttga ccaccaactg aaacctgacc 100

tctgacccca gaccactggc ccttccccg ccctgtggtg acttcataaa 150

ggttactagc ttctcccctg gccttgagac ccacacgatg gccctgctgg 200

ctctggccag tgccgtcccc tctgccctgc tggccctggc tgtcttcagg 250

gtgcccgcct gggcctgtct cctctgcttc acaacctact ctgagcgcct 300

ccgcatctgc cagatgtttg ttgggatgcg gagccccaag cttgaagagt 350

gtgaggaggc cttcacggcc gccttccagg gcctctctga caccgaaatc 400

agtgaggaga ccatccacac ttcatcagtg tcctggggaa ggtgcagagg 450

gagggcagga gaggcccaga gggtcaggct gagggacaga cagagagaaa 500

cagtcagagg agaaaggctc aaagaccatg agaacaacag agacttaggg 550

acagagagac acagacaggg gaagacagca gggcaaagac tcagagaggg 600

gaggatggag agtcagagag gggaagatgg agactcagag agaggggagg 650

atggagactc agagagagag gaagatggag actcagaggg aaagatggag 700
```

actcaggagt atggagagtc agagagggga ggatggacac tcaggggagg 750
atggagagtc aggaggatgg agactcatag aaaggggagg atggagagtc 800
aggagaggtt ggagactgga gagggaatag agacccagaa aggggaggat 850
ggagactcag agggtggaag atggagactc aaagaggatg gaaacccaga 900
gagaggagga cagagatgag gcagagacta ggggaagcag gatagcgact 950
ggtcgggggc agagactcag ggaggataga gactcacaga gaggtgagga 1000
tagagacttg ggagggactc aggaagcata gcgactgtgg ggcaaagagt 1050
cagagagggg aggatacaga cttgggaggg cagagactca gaaacagaat 1100
gttcgcatta gggacatggt gttgcgggga gctgcctccc ccagcccctg 1150
ctccctccct caccgccaga ctatgatgag agaagccacc tgcatgacac 1200
cttcacccag atgacccatg ccctgcagga gctggctgct gcccagggat 1250
cctttgaggt tgccttccct gatgctgcag agaaaatgaa gaaggtcatt 1300
acacagctta aagaagccca ggcttgcatc cctccctgcg gtctccagga 1350
gttcgcccgg cgtttcctct gcagcgggtg ctactctagg gtctgcgacc 1400
tcccgctgga ctgcccagtt caggatgtga cagtgactcg ggcgaccag 1450
gctatgtttt cttgcatcgt aaacttccag ctgccaaagg aggagatcac 1500
ctattcctgg aagttcgcag gaggaggtct ccggactcag gacttgtcct 1550
atttccgaga tatgccgcgg gccgaaggat acctggcgcg gatccggccg 1600
gctcagctca cgcaccgcgg gacgttctcc tgcgtgatca agcaagacca 1650
gcgcccccctg gcccggctct acttctttct taacgtcctc ggggccctcg 1700
catcagcgag tgcgacagtg ttggcgtggt gagttctggg gactccggag 1750
ccccagcatc tagctccccg ctgtctcaga tcccaccgag aagtctgggt 1800
tcccagcaac ctccaaccca ggaggatgtt ctttcgatgg tactgcagtg 1850
gcaactaaca aaggtatctt tcctccttcc ctatcctatt tccatcctga 1900
aaataaagaa tatatttcaa ctctaaaaaa aaaaaaaaaa aaaaaaaaaa 1950
aaaaaaaaaa aaaaaaaa 1968

<210> 164
<211> 243
<212> PRT
<213> Homo Sapien

<400> 164
Met Ala Leu Leu Ala Leu Ala Ser Ala Val Pro Ser Ala Leu Leu
1 5 10 15

Ala Leu Ala Val Phe Arg Val Pro Ala Trp Ala Cys Leu Leu Cys
20 25 30

Phe Thr Thr Tyr Ser Glu Arg Leu Arg Ile Cys Gln Met Phe Val
35 40 45

Gly Met Arg Ser Pro Lys Leu Glu Glu Cys Glu Glu Ala Phe Thr
50 55 60

Ala Ala Phe Gln Gly Leu Ser Asp Thr Glu Ile Ser Glu Glu Thr
65 70 75

Ile His Thr Ser Ser Val Ser Trp Gly Arg Cys Arg Gly Arg Ala
80 85 90

Gly Glu Ala Gln Arg Val Arg Leu Arg Asp Arg Gln Arg Glu Thr
95 100 105

Val Arg Gly Glu Arg Leu Lys Asp His Glu Asn Asn Arg Asp Leu
110 115 120

Gly Thr Glu Arg His Arg Gln Gly Lys Thr Ala Gly Gln Arg Leu
125 130 135

Arg Glu Gly Arg Met Glu Ser Gln Arg Gly Glu Asp Gly Asp Ser
140 145 150

Glu Arg Gly Glu Asp Gly Asp Ser Glu Arg Glu Glu Asp Gly Asp
155 160 165

Ser Glu Gly Lys Met Glu Thr Gln Glu Tyr Gly Glu Ser Glu Arg
170 175 180

Gly Gly Trp Thr Leu Arg Gly Gly Trp Arg Val Arg Arg Met Glu
185 190 195

Thr His Arg Lys Gly Arg Met Glu Ser Gln Glu Arg Leu Glu Thr
200 205 210

Gly Glu Gly Ile Glu Thr Gln Lys Gly Glu Asp Gly Asp Ser Glu
215 220 225

Gly Gly Arg Trp Arg Leu Lys Glu Asp Gly Asn Pro Glu Arg Gly
230 235 240

Gly Gln Arg

<210> 165
<211> 1941
<212> DNA
<213> Homo Sapien

<400> 165
cagaatcgca gattgccagc ccttttcccg acccctacgg aaagacgagt 50

ccaggggccg tcctggcgag gtcaaaacat ttagtctggt cttttcagcg 100

tggaccctgc cagcagccag gccatggagc tctctgatgt caccctcatt 150

gagggtgtgg gtaatgaggt gatggtggtg gcaggtgtgg tggtgctgat 200

tctagccttg gtcctagctt ggctctctac ctacgtagca gacagcggta 250

gcaaccagct cctgggcgct attgtgtcag caggcgacac atccgtcctc 300

cacctggggc atgtggacca cctggtggca ggccaaggca accccgagcc 350

aactgaactc ccccatccat cagagggtaa tgatgagaag gctgaagagg 400
cgggtgaagg tcggggagac tccactgggg aggctggagc tgggggtggt 450
gttgagccca gccttgagca tctccttgac atccaaggcc tgcccaaaag 500
acaagcaggt gcaggcagca gcagtccaga ggccccctg agatctgagg 550
atagcacctg cctccctccc agccctggcc tcatcactgt gcggctcaaa 600
ttcctcaatg ataccgagga gctggctgtg gctaggccag aggataccgt 650
gggtgccctg aagagcaaat acttccctgg acaagaaagc cagatgaaac 700
tgatctacca gggccgcctg ctacaagacc cagcccgcac actgcgttct 750
ctgaacatta ccgacaactg tgtgattcac tgccaccgct cacccccagg 800
gtcagctgtt ccaggcccct cagcctcctt ggccccctcg gccactgagc 850
cacccagcct tggtgtcaat gtgggcagcc tcatggtgcc tgtctttgtg 900
gtgctgttgg gtgtggtctg gtacttccga atcaattacc gccaattctt 950
cacagcacct gccactgtct ccctggtggg agtcaccgtc ttcttcagct 1000
tcctagtatt tgggatgtat ggacgataag gacataggaa gaaaatgaaa 1050
ggcatggtct ttctccttta tggcctcccc acttttcctg gccagagctg 1100
ggcccaaggg ccggggaggg aggggtggaa aggatgtgat ggaaatctcc 1150
tccataggac acaggaggca agtatgcggc ctccccttct catccacagg 1200
agtacagatg tccctcccgt gcgagcacaa ctcaggtaga aatgaggatg 1250
tcatcttcct tcacttttag ggtcctctga aggagttcaa agctgctggc 1300
caagctcagt ggggagcctg ggctctgaga ttccctccca cctgtggttc 1350
tgactcttcc cagtgtcctg catgtctgcc cccagcaccc agggctgcct 1400
gcaagggcag ctcagcatgg ccccagcaca actccgtagg gagcctggag 1450
tatccttcca tttctcagcc aaatactcat cttttgagac tgaaatcaca 1500
ctggcgggaa tgaagattgt gccagccttc tcttatgggc acctagccgc 1550
cttcaccttc ttcctctacc ccttagcagg aatagggtgt cctcccttct 1600
ttcaaagcac tttgcttgca ttttatttta ttttttaag agtccttcat 1650
agagctcagt caggaagggg atggggcacc aagccaagcc cccagcattg 1700
ggagcggcca ggccacagct gctgctcccg tagtcctcag gctgtaagca 1750
agagacagca ctggcccttg gccagcgtcc taccctgccc aactccaagg 1800
actgggtatg gatcgctggg ccctaggctc ttgcttctgg ggctattgga 1850
gggtcagtgt ctgtgactga ataaagttcc attttgtgga aaaaaaaaaa 1900
aaaaaaaaaa aaaaaaaaaa aaaaaaaaaa aaaaaaaaaa a 1941

<210> 166
<211> 301
<212> PRT
<213> Homo Sapien

<400> 166

```
Met Glu Leu Ser Asp Val Thr Leu Ile Glu Gly Val Gly Asn Glu
1               5                   10                  15

Val Met Val Val Ala Gly Val Val Val Leu Ile Leu Ala Leu Val
                20                  25                  30

Leu Ala Trp Leu Ser Thr Tyr Val Ala Asp Ser Gly Ser Asn Gln
                35                  40                  45

Leu Leu Gly Ala Ile Val Ser Ala Gly Asp Thr Ser Val Leu His
                50                  55                  60

Leu Gly His Val Asp His Leu Val Ala Gly Gln Gly Asn Pro Glu
                65                  70                  75

Pro Thr Glu Leu Pro His Pro Ser Glu Gly Asn Asp Glu Lys Ala
                80                  85                  90

Glu Glu Ala Gly Glu Gly Arg Gly Asp Ser Thr Gly Glu Ala Gly
                95                  100                 105

Ala Gly Gly Gly Val Glu Pro Ser Leu Glu His Leu Leu Asp Ile
                110                 115                 120

Gln Gly Leu Pro Lys Arg Gln Ala Gly Ala Gly Ser Ser Ser Pro
                125                 130                 135

Glu Ala Pro Leu Arg Ser Glu Asp Ser Thr Cys Leu Pro Pro Ser
                140                 145                 150

Pro Gly Leu Ile Thr Val Arg Leu Lys Phe Leu Asn Asp Thr Glu
                155                 160                 165

Glu Leu Ala Val Ala Arg Pro Glu Asp Thr Val Gly Ala Leu Lys
                170                 175                 180

Ser Lys Tyr Phe Pro Gly Gln Glu Ser Gln Met Lys Leu Ile Tyr
                185                 190                 195

Gln Gly Arg Leu Leu Gln Asp Pro Ala Arg Thr Leu Arg Ser Leu
                200                 205                 210

Asn Ile Thr Asp Asn Cys Val Ile His Cys His Arg Ser Pro Pro
                215                 220                 225

Gly Ser Ala Val Pro Gly Pro Ser Ala Ser Leu Ala Pro Ser Ala
                230                 235                 240

Thr Glu Pro Pro Ser Leu Gly Val Asn Val Gly Ser Leu Met Val
                245                 250                 255

Pro Val Phe Val Val Leu Leu Gly Val Val Trp Tyr Phe Arg Ile
                260                 265                 270

Asn Tyr Arg Gln Phe Phe Thr Ala Pro Ala Thr Val Ser Leu Val
                275                 280                 285
```

Gly Val Thr Val Phe Phe Ser Phe Leu Val Phe Gly Met Tyr Gly
290 295 300

Arg

<210> 167
<211> 3323
<212> DNA
<213> Homo Sapien

<400> 167
ggcggctgtg tgtcgccgga gccgaagcgc gcaggcccgt cccggtggcc 50

ggggagcggg cgggtggggg cgccatgtgg ttcatgtacc tgctgagctg 100

gctgtcgctc ttcatccagg tggccttcat cacgctggct gtcgcggctg 150

gactctatta cctggcagaa ctgatagaag aatacacagt ggccaccagc 200

aggatcataa aatacatgat ctggttctcc accgctgtac tgattggcct 250

ctacgtcttt gagcgcttcc ccaccagcat gattggagtg ggcctattca 300

ccaacctcgt ctactttggc ctcctccaga ccttcccctt catcatgctg 350

acctcgccta acttcatcct gtcgtgtgga ctagtggtgg tgaatcatta 400

cctagcattt cagtttttg cagaagaata ttatcccttc tcagaggtcc 450

tggcctattt cactttctgc ctgtggataa ttccgtttgc gtttttgtg 500

tcactttcgg ccggggagaa cgtcctgccc tctaccatgc agccaggaga 550

tgatgtcgtc tccaattatt tcaccaaagg caagcggggc aaacgcttag 600

ggatcctggt tgtcttctcc ttcatcaaag aggccattct acccagtcgt 650

cagaagatat actgaccccc atgcaggcag gatgtggggg gcaagatcag 700

gagagtcagg cccctgggcc tctatgccag gtggggacca gaagtcggga 750

aggcacctac cacctgccct ggctttcttc ccctcaactc tggagcccca 800

tccccaccct ccttgggggg ctcagcttgg ctcagatctg atgcttcaag 850

aggctgtaac ctcagagggc accaaggagg gtggcagagc ctgcttagcc 900

aggaggccga ggtccctcag tcctcccctg tcccttccaa ggtgggtcag 950

gaggttctgg ccccgctggg gcaggcaggg cagggtctgt gaagcttaag 1000

agcagatggt gacaagttct ctgggcaggt ggccatgggg aggggccatg 1050

gcttggcatg tccaacagaa atagttttg ctgttgaacg gtgatttctg 1100

tccaagtgca gatttccgtt tgaataaagc ttcgcttcta ggtggcactg 1150

tttgccttaa taccctgaca gttcatcttc ctttcttcct gctaaccttc 1200

tgctctggac tggactcact tttctgctcc agggactcct tttctgggtt 1250

tgggtcttgc ccttcccaag ggactgttct tgtggccctt aatgggaagg 1300

gggcaggggt gaggagctga gcctgctcaa ggagtgggaa gtggggctat 1350
aggcagcctc tctgatgcac tctcttccat ctctttcccc aaggctccgt 1400
gactgtcaaa ctgggagtag gagaggggac aatttaggac tgggctagat 1450
tttcagaaga acatctacaa tatcctattt ataaatcttc ctctgggaaa 1500
aggagtggtt tctggctgaa tactatctta ggctcaagga gaaacaaaat 1550
aaaaattagc ttccaggcag cctgttttta aagaaatggg actaatggga 1600
gaagctgttt gtcactctaa gagcatccaa gccctggccc gtctgtgcac 1650
tcttggctcc tggggagata tatctgcctt ctaagaaggc aggccaggtc 1700
ttgggcacag acctgcattt gttgaccttg cactccaact atagtgcctt 1750
gcaagtgctc aacagtacat attggaatga agtccctatg agagccattt 1800
ctggccatgt tctatacctc aaagtgaggc tggcaggtac agagatgaac 1850
tgtacacatg tgatacattt aagccactgg aaaaacccct gtgcttgaaa 1900
atatttcctc tatatcatgc ctggagttcc atcatagccc ttcatttcct 1950
tggctttagc atttaccttc tcttaagaat accagctttc ccctttccct 2000
gagaggaaga gcacatgttg gtctcctctt agtgtgaacg agattgccag 2050
gcccttttct cctatgcaca ccaggataga caaggcaggg gatactggca 2100
gcctgcatca tcctcccatt gggctgacag ctggccctac tttcctccct 2150
ctgctgcttg gtccctcacc ttgatgatgt ggcttcgccc cctccactct 2200
actgccagtg ttctcccagg ggttgctaaa tccagcagac ccctttcctg 2250
tcttactaga tctgggcagc atttgacatg gctgatcacc ccttgcttct 2300
tggatggcac ttccctggca cctctgtggc tagttgtcct acctccctgg 2350
ctgttccttt caggcttccg tgcaggcttc tccacttgcc catgcacagt 2400
agggtctttc agggttctgc tgtgggctcc ctagggaagc ccatccatct 2450
ggatggtttc aaggatggtg aggaatttag agttgacctc cagccccaac 2500
atccttcctg atcacctgaa ccacagtttt gctgccctct aggtgcacag 2550
acaattcagg tccatggccc agatggtact tgctgtcttc tgcaaacctg 2600
ccccttctgg gtacttccct tgaccccgag atcactcagg agccagacag 2650
gaaacttatt ctattcctgt tttctctttc tgcccaccac atccaatctc 2700
tcaaaacggt caggtctacc ttaacatctc ttgatttgag ccactcccac 2750
tgtcatcagc tttcacctgg attatcgtga cagcctccta ctgcttctct 2800
atcatgtggc cagagctatc ttcctaaaat gcattgcata gttgatcaag 2850
tcactctctg gcctaaaacc ttccttggct ccctgctgcc ctcaggataa 2900

agtctggacc cctcagcatg gcttgtgaga ctcatggtgt ccttgtccct 2950

gctcacctct ctggtctcat cacttgcctt cttgcattct gggtcccagc 3000

ctcctgtatc cagagatgca gtggctctcc attgccactc tgattcctcc 3050

tttcttttgg tcacagagaa agggtacttt ctctgtcaaa tctcaactta 3100

gacttgactt cctccaagga gctttggcta tactctctcc tcccgacccc 3150

caccctggca tactacacag atcactctgg gctcacttgc ctgcctaatg 3200

gtcatctccc cagtagactg taagctcctt gagggcaagg attgtgttgg 3250

aatttttgta ttaacagtgc ctggcttggt gcctggcacc tagaaagcac 3300

tcaataaatg tttgtttaat gaa 3323

<210> 168
<211> 196
<212> PRT
<213> Homo Sapien

<400> 168
Met Trp Phe Met Tyr Leu Leu Ser Trp Leu Ser Leu Phe Ile Gln
1               5                   10                  15

Val Ala Phe Ile Thr Leu Ala Val Ala Ala Gly Leu Tyr Tyr Leu
20                  25                  30

Ala Glu Leu Ile Glu Glu Tyr Thr Val Ala Thr Ser Arg Ile Ile
35                  40                  45

Lys Tyr Met Ile Trp Phe Ser Thr Ala Val Leu Ile Gly Leu Tyr
50                  55                  60

Val Phe Glu Arg Phe Pro Thr Ser Met Ile Gly Val Gly Leu Phe
65                  70                  75

Thr Asn Leu Val Tyr Phe Gly Leu Leu Gln Thr Phe Pro Phe Ile
80                  85                  90

Met Leu Thr Ser Pro Asn Phe Ile Leu Ser Cys Gly Leu Val Val
95                  100                 105

Val Asn His Tyr Leu Ala Phe Gln Phe Phe Ala Glu Glu Tyr Tyr
110                 115                 120

Pro Phe Ser Glu Val Leu Ala Tyr Phe Thr Phe Cys Leu Trp Ile
125                 130                 135

Ile Pro Phe Ala Phe Phe Val Ser Leu Ser Ala Gly Glu Asn Val
140                 145                 150

Leu Pro Ser Thr Met Gln Pro Gly Asp Asp Val Val Ser Asn Tyr
155                 160                 165

Phe Thr Lys Gly Lys Arg Gly Lys Arg Leu Gly Ile Leu Val Val
170                 175                 180

Phe Ser Phe Ile Lys Glu Ala Ile Leu Pro Ser Arg Gln Lys Ile
185                 190                 195

Tyr

<210> 169
<211> 1664
<212> DNA
<213> Homo Sapien

<400> 169
caaagcccta ccctcaccat tcaccaggtc ctgtgggaag agcagcgtgg 50

aggtgggctg aggttagaag gtgcagagcg tggaagaaga ttgtgagctg 100

agtattggac atctgttctt gaatagtccc tgggcctgcc ataggaaagg 150

aagttctcca gggttacagt tcttatccgc gtgaatacac atggctctgt 200

tacgaaaaat taatcaggtg ctgctgttcc ttctgatcgt gaccctctgt 250

gtgattctgt ataagaaagt tcataagggg actgtgccca agaatgacgc 300

agatgatgaa tccgagactc ctgaagaact ggaagaagag attcctgtgg 350

tgatttgtgc tgcagcaggg aggatgggtg ccactatggc tgccatcaat 400

agcatctaca gcaacactga cgccaacatc ttgttctatg tagtgggact 450

ccggaatact ctgactcgaa tacgaaaatg gattgaacat tccaaactga 500

gagaaataaa ctttaaaatc gtggaattca acccgatggt cctcaaaggg 550

aagatcagac cagactcatc gaggcctgaa ttgctccagc ctctgaactt 600

tgttcgattt tatctccctc tacttatcca ccaacacgag aaagtcatct 650

atttggacga tgatgtaatt gtacaaggtg atatccaaga actgtatgac 700

accaccttgg ccctgggcca cgcggcggct ttctcagatg actgcgattt 750

gccctctgct caggacataa acagactcgt gggacttcag aacacatata 800

tgggctatct ggactaccgg aagaaggcca tcaaggacct tggcatcagc 850

cccagcacct gctctttcaa tcctggtgtg attgttgcca acatgacaga 900

atggaagcac cagcgcatca ccaagcaatt ggagaaatgg atgcaaaaga 950

atgtggagga aaacctctat agcagctccc tgggaggagg ggtggccacc 1000

tccccaatgc tgattgtgtt tcatgggaaa tattccacaa ttaacccccct 1050

gtggcacata aggcacctgg gctggaatcc agatgccaga tattcggagc 1100

attttctgca ggaagctaaa ttactccact ggaatggaag acataaacct 1150

tgggacttcc ctagtgttca caacgactta tgggaaagct ggtttgttcc 1200

tgaccctgca gggatattta aactcaatca ccatagctga tataactcta 1250

cccttaaaat attccctgta tagaaatgtg gaattgtccc tttgtagcca 1300

actataacat tgttctttat gaatattacc tttgatacat atgatccaca 1350

atataaaaac caaaaactac tgtgtgcaaa ttataccttg gaccatatag 1400

gcattgatta acttctttaa gtacatgtga taactatgga aatcaagatt 1450

atgtgactga aaaacataaa ggaagagacc catctagata acagcaatca 1500

acctgcttaa ttctgaatga caattatatc cacaaatttt taaaacttct 1550

acatgtattt ttcacatgaa gatctcctta acaggttgcc aaccttttct 1600

tttataaaac tattacattt aaaatatgga cgtctgaaaa ataaaatatt 1650

catcattttt aaaa 1664

<210> 170
<211> 349
<212> PRT
<213> Homo Sapien

<400> 170

Met Ala Leu Leu Arg Lys Ile Asn Gln Val Leu Leu Phe Leu Leu
1 5 10 15

Ile Val Thr Leu Cys Val Ile Leu Tyr Lys Lys Val His Lys Gly
20 25 30

Thr Val Pro Lys Asn Asp Ala Asp Asp Glu Ser Glu Thr Pro Glu
35 40 45

Glu Leu Glu Glu Glu Ile Pro Val Val Ile Cys Ala Ala Ala Gly
50 55 60

Arg Met Gly Ala Thr Met Ala Ala Ile Asn Ser Ile Tyr Ser Asn
65 70 75

Thr Asp Ala Asn Ile Leu Phe Tyr Val Val Gly Leu Arg Asn Thr
80 85 90

Leu Thr Arg Ile Arg Lys Trp Ile Glu His Ser Lys Leu Arg Glu
95 100 105

Ile Asn Phe Lys Ile Val Glu Phe Asn Pro Met Val Leu Lys Gly
110 115 120

Lys Ile Arg Pro Asp Ser Ser Arg Pro Glu Leu Leu Gln Pro Leu
125 130 135

Asn Phe Val Arg Phe Tyr Leu Pro Leu Leu Ile His Gln His Glu
140 145 150

Lys Val Ile Tyr Leu Asp Asp Asp Val Ile Val Gln Gly Asp Ile
155 160 165

Gln Glu Leu Tyr Asp Thr Thr Leu Ala Leu Gly His Ala Ala Ala
170 175 180

Phe Ser Asp Asp Cys Asp Leu Pro Ser Ala Gln Asp Ile Asn Arg
185 190 195

Leu Val Gly Leu Gln Asn Thr Tyr Met Gly Tyr Leu Asp Tyr Arg
200 205 210

Lys Lys Ala Ile Lys Asp Leu Gly Ile Ser Pro Ser Thr Cys Ser
215 220 225

Phe Asn Pro Gly Val Ile Val Ala Asn Met Thr Glu Trp Lys His

230 235 240

Gln Arg Ile Thr Lys Gln Leu Glu Lys Trp Met Gln Lys Asn Val
245 250 255

Glu Glu Asn Leu Tyr Ser Ser Ser Leu Gly Gly Gly Val Ala Thr
260 265 270

Ser Pro Met Leu Ile Val Phe His Gly Lys Tyr Ser Thr Ile Asn
275 280 285

Pro Leu Trp His Ile Arg His Leu Gly Trp Asn Pro Asp Ala Arg
290 295 300

Tyr Ser Glu His Phe Leu Gln Glu Ala Lys Leu Leu His Trp Asn
305 310 315

Gly Arg His Lys Pro Trp Asp Phe Pro Ser Val His Asn Asp Leu
320 325 330

Trp Glu Ser Trp Phe Val Pro Asp Pro Ala Gly Ile Phe Lys Leu
335 340 345

Asn His His Ser

<210> 171
<211> 756
<212> DNA
<213> Homo Sapien

<400> 171
gccagaggct gcagctggag cccagagccc aagatggagc cccagctggg 50

gcctgaggct gccgccctcc gccctggctg gctggccctg ctgctgtggg 100

tctcagccct gagctgttct ttctccttgc cagcttcttc cctttcttct 150

ctggtgcccc aagtcagaac cagctacaat tttggaagga ctttcctcgg 200

tcttgataaa tgcaatgcct gcatcgggac atctatttgc aagaagttct 250

ttaaagaaga aataagatct gacaactggc tggcttccca ccttggactg 300

cctcccgatt ccttgctttc ttatcctgca aattactcag atgattccaa 350

aatctggcgc cctgtggaga tctttagact ggtcagcaaa tatcaaaacg 400

agatctcaga caggagaatc tgtgcctctg catcagcccc aaagacctgc 450

agcattgagc gtgtcctgcg gaaaacagag aggttccaga aatggctgca 500

ggccaagcgc ctcacgccgg acctggtgca ggactgtcac cagggccaga 550

gagaactaaa gttcctgtgt atgctgagat aacaccagtg aaaaagcctg 600

gcatggagcc cagcactgag aacttccaga aagtgttagc cttctcccaa 650

ctgtgttata ccaaccacat tttcaaatag taatcattaa agaggcttct 700

gcatcaaaaa aaaaaaaaaa aaaaaaaaaa aaaaaaaaaa aaaaaaaaaa 750

aaaaaa 756

<210> 172
<211> 182
<212> PRT
<213> Homo Sapien

<400> 172
Met Glu Pro Gln Leu Gly Pro Glu Ala Ala Ala Leu Arg Pro Gly
1 5 10 15

Trp Leu Ala Leu Leu Leu Trp Val Ser Ala Leu Ser Cys Ser Phe
20 25 30

Ser Leu Pro Ala Ser Ser Leu Ser Ser Leu Val Pro Gln Val Arg
35 40 45

Thr Ser Tyr Asn Phe Gly Arg Thr Phe Leu Gly Leu Asp Lys Cys
50 55 60

Asn Ala Cys Ile Gly Thr Ser Ile Cys Lys Lys Phe Phe Lys Glu
65 70 75

Glu Ile Arg Ser Asp Asn Trp Leu Ala Ser His Leu Gly Leu Pro
80 85 90

Pro Asp Ser Leu Leu Ser Tyr Pro Ala Asn Tyr Ser Asp Asp Ser
95 100 105

Lys Ile Trp Arg Pro Val Glu Ile Phe Arg Leu Val Ser Lys Tyr
110 115 120

Gln Asn Glu Ile Ser Asp Arg Arg Ile Cys Ala Ser Ala Ser Ala
125 130 135

Pro Lys Thr Cys Ser Ile Glu Arg Val Leu Arg Lys Thr Glu Arg
140 145 150

Phe Gln Lys Trp Leu Gln Ala Lys Arg Leu Thr Pro Asp Leu Val
155 160 165

Gln Asp Cys His Gln Gly Gln Arg Glu Leu Lys Phe Leu Cys Met
170 175 180

Leu Arg

<210> 173
<211> 1210
<212> DNA
<213> Homo Sapien

<400> 173
gctggactgc tcgctggccg gcagcgcacc gttttgaagg tcctagccca 50

cctgggctgg ctcacgcgca cgactagccg ctcccataca gcacgcccgg 100

actctgtcgt cgcttaaggc cactcctatt ctacggctga cccctggtgg 150

tcacgtggat ctgttcgcca cgcaagtctg ggtccttcgg cgattgaccg 200

gggtccttgc tgttcgggag cctctcctaa gctgcctgtt cgcgcgagag 250

tttggagggg cgggtttggg gtcggtgtct gattggggct cgcaccgcag 300

cacgctggag tcccgcttag gtaccagtta gcgtcagggg agctgggtca 350

ggcggtcgcc gggacacccc gtgtgtggca ggcggcgaag cgctctggag 400

aatcccggac agccctgctc cctgcagcca ggtgtagttt cgggagccac 450

tggggccaaa gtgagagtcc agcggtcttc cagcgcttgg gccacggcgg 500

cggccctggg agcagaggtg gagcgacccc attacgctaa agatgaaagg 550

ctggggttgg ctggccctgc ttctgggggc cctgctggga accgcctggg 600

ctcggaggag ccaggatctc cactgtggag catgcagggc tctggtggat 650

gaactagaat gggaaattgc ccaggtggac cccaagaaga ccattcagat 700

gggatctttc cggatcaatc cagatggcag ccagtcagtg gtggaggtgc 750

cttatgcccg ctcagaggcc cacctcacag agctgctgga ggagatatgt 800

gaccggatga aggagtatgg ggaacagatt gatccttcca cccatcgcaa 850

gaactacgta cgtgtagtgg gccggaatgg agaatccagt gaactggacc 900

tacaaggcat ccgaatcgac tcagatatta gcggcaccct caagtttgcg 950

tgtgagagca ttgtggagga atacgaggat gaactcattg aattcttttc 1000

ccgagaggct gacaatgtta aagacaaact ttgcagtaag cgaacagatc 1050

tttgtgacca tgccctgcac atatcgcatg atgagctatg aaccactgga 1100

gcagcccaca ctggcttgat ggatcacccc caggaggga aaatggtggc 1150

aatgcctttt atatattatg tttttactga aattaactga aaaaatatga 1200

aaccaaaagt 1210

<210> 174
<211> 182
<212> PRT
<213> Homo Sapien

<400> 174
Met Lys Gly Trp Gly Trp Leu Ala Leu Leu Leu Gly Ala Leu Leu
1 5 10 15

Gly Thr Ala Trp Ala Arg Arg Ser Gln Asp Leu His Cys Gly Ala
20 25 30

Cys Arg Ala Leu Val Asp Glu Leu Glu Trp Glu Ile Ala Gln Val
35 40 45

Asp Pro Lys Lys Thr Ile Gln Met Gly Ser Phe Arg Ile Asn Pro
50 55 60

Asp Gly Ser Gln Ser Val Val Glu Val Pro Tyr Ala Arg Ser Glu
65 70 75

Ala His Leu Thr Glu Leu Leu Glu Glu Ile Cys Asp Arg Met Lys
80 85 90

Glu Tyr Gly Glu Gln Ile Asp Pro Ser Thr His Arg Lys Asn Tyr
95 100 105

```
Val Arg Val Val Gly Arg Asn Gly Glu Ser Ser Glu Leu Asp Leu
                110                 115                 120

Gln Gly Ile Arg Ile Asp Ser Asp Ile Ser Gly Thr Leu Lys Phe
                125                 130                 135

Ala Cys Glu Ser Ile Val Glu Glu Tyr Glu Asp Glu Leu Ile Glu
                140                 145                 150

Phe Phe Ser Arg Glu Ala Asp Asn Val Lys Asp Lys Leu Cys Ser
                155                 160                 165

Lys Arg Thr Asp Leu Cys Asp His Ala Leu His Ile Ser His Asp
                170                 175                 180

Glu Leu
```

```
<210> 175
<211> 2027
<212> DNA
<213> Homo Sapien

<400> 175
cgcagcgcgg cagtcctgat ggcccggcat gggttaccgc tgctgcccct 50

gctgtcgctc ctggtcggcg cgtggctcaa gctaggaaat ggacaggcta 100

ctagcatggt ccaactgcag ggtgggagat tcctgatggg aacaaattct 150

ccagacagca gagatggtga agggcctgtg cgggaggcga cagtgaaacc 200

ctttgccatc gacatatttc ctgtcaccaa caaagatttc agggattttg 250

tcagggagaa aaagtatcgg acagaagctg agatgtttgg atggagcttt 300

gtctttgagg actttgtctc tgatgagctg agaaacaaag ccacccagcc 350

aatgaagtct gtactctggt ggcttccagt ggaaaaggca ttttggaggc 400

agcctgcagg tcctggctct ggcatccgag agagactgga gcacccagtg 450

ttacacgtga gctggaatga cgcccgtgcc tactgtgctt ggcggggaaa 500

acgactgccc acggaggaag agtgggagtt tgccgcccga gggggcttga 550

agggtcaagt ttacccatgg gggaactggt tccagccaaa ccgcaccaac 600

ctgtggcagg gaaagttccc caagggagac aaagctgagg atggcttcca 650

tggagtctcc ccagtgaatg ctttccccgc ccagaacaac tacgggctct 700

atgacctcct ggggaacgtg tgggagtgga cagcatcacc gtaccaggct 750

gctgagcagg acatgcgcgt cctccggggg gcatcctgga tcgacacagc 800

tgatggctct gccaatcacc gggcccgggt caccaccagg atgggcaaca 850

ctccagattc agcctcagac aacctcggtt tccgctgtgc tgcagacgca 900

ggccggccgc caggggagct gtaagcagcc gggtggtgac aaggagaaaa 950

gccttctagg gtcactgtca ttccctggcc atgttgcaaa cagcgcaatt 1000
```

ccaagctcga gagcttcagc ctcaggaaag aacttcccct tccctgtctc 1050

ccatccctct gtggcaggcg cctctcacca gggcaggaga ggactcagcc 1100

tcctgtgttt tggagaaggg gcccaatgtg tgttgacgat ggctgggggc 1150

caggtgtttc tgttagaggc caagtattat tgacacagga ttgcaaacac 1200

acaaacagtt ggaacagagc actctgaaag gccatttttt aagcatttta 1250

aaatctattc tctccccctt tctccctgga tgattcagga agctgacatt 1300

gtttcctcaa ggcagaattt tcctggttct gttttctcag ccagttgctg 1350

tggaaggaga atgctttctt tgtggcctca tctgtggttt cgtgtccctc 1400

tgaaggaaac tagtttccac tgtgtaacag gcagacatgt aactatttaa 1450

agcacagttc agtcctaaaa gggtctggga gaaccagatg atgtactagg 1500

tgaagcattg cattgtggga atcacaaagc aaatagtact ccagaaagac 1550

aaatatcaga agcttcctat tctttttttt tttttttttt tttttttgag 1600

acagggtctt tctctgttgc ccaggctaga gtgcactggt gatcacggct 1650

cactctagcc ttgaattcct gggcccaagc aattctccca cctcagcctc 1700

ctgagtagct gggactacaa gtgtgcacca ccatgcctgg ctaatttttt 1750

gaatttttgt agtgatggga tctcgctctg ttgcccaggg tggtctcgaa 1800

ctcctggcct caagcgatcc tcccacctcg acctcccaaa gtgctgggat 1850

tacaggtgtg agccacctcg cctgggcccc cttctccata tgcctccaaa 1900

aacatgtccc tggagagtag cctgctccca cactgtcact ggatgtcatg 1950

gggccaataa aatctcctgc aattgtgtat ctcaaaaaaa aaaaaaaaaa 2000

aaaaaaaaaa aaaaaaaaaa aaaaaaa 2027

<210> 176
<211> 301
<212> PRT
<213> Homo Sapien

<400> 176
Met Ala Arg His Gly Leu Pro Leu Leu Pro Leu Leu Ser Leu Leu
1 5 10 15

Val Gly Ala Trp Leu Lys Leu Gly Asn Gly Gln Ala Thr Ser Met
20 25 30

Val Gln Leu Gln Gly Gly Arg Phe Leu Met Gly Thr Asn Ser Pro
35 40 45

Asp Ser Arg Asp Gly Glu Gly Pro Val Arg Glu Ala Thr Val Lys
50 55 60

Pro Phe Ala Ile Asp Ile Phe Pro Val Thr Asn Lys Asp Phe Arg
65 70 75

Asp Phe Val Arg Glu Lys Lys Tyr Arg Thr Glu Ala Glu Met Phe

80 85 90

Gly Trp Ser Phe Val Phe Glu Asp Phe Val Ser Asp Glu Leu Arg
95 100 105

Asn Lys Ala Thr Gln Pro Met Lys Ser Val Leu Trp Trp Leu Pro
110 115 120

Val Glu Lys Ala Phe Trp Arg Gln Pro Ala Gly Pro Gly Ser Gly
125 130 135

Ile Arg Glu Arg Leu Glu His Pro Val Leu His Val Ser Trp Asn
140 145 150

Asp Ala Arg Ala Tyr Cys Ala Trp Arg Gly Lys Arg Leu Pro Thr
155 160 165

Glu Glu Glu Trp Glu Phe Ala Ala Arg Gly Gly Leu Lys Gly Gln
170 175 180

Val Tyr Pro Trp Gly Asn Trp Phe Gln Pro Asn Arg Thr Asn Leu
185 190 195

Trp Gln Gly Lys Phe Pro Lys Gly Asp Lys Ala Glu Asp Gly Phe
200 205 210

His Gly Val Ser Pro Val Asn Ala Phe Pro Ala Gln Asn Asn Tyr
215 220 225

Gly Leu Tyr Asp Leu Leu Gly Asn Val Trp Glu Trp Thr Ala Ser
230 235 240

Pro Tyr Gln Ala Ala Glu Gln Asp Met Arg Val Leu Arg Gly Ala
245 250 255

Ser Trp Ile Asp Thr Ala Asp Gly Ser Ala Asn His Arg Ala Arg
260 265 270

Val Thr Thr Arg Met Gly Asn Thr Pro Asp Ser Ala Ser Asp Asn
275 280 285

Leu Gly Phe Arg Cys Ala Ala Asp Ala Gly Arg Pro Pro Gly Glu
290 295 300

Leu

<210> 177
<211> 959
<212> DNA
<213> Homo Sapien

<400> 177
gccttctcgc gcctgaccat gcacccctgc atcttcctgc tgggccacag 50

gcgagcgctt tatttctgga gctgagggct aaaacttttt tgacttttct 100

tctcctcaac atctgaatca tgccatgtgc ccagaggagc tggcttgcaa 150

acctttccgt ggtggctcag ctccttaact ttggggcgct ttgctatggg 200

agacagcctc agccaggccc ggttcgcttc ccggacagga ggcaagagca 250

ttttatcaag ggcctgccag aataccacgt ggtgggtcca gtccgagtag 300

atgccagtgg gcattttttg tcatatggct tgcactatcc catcacgagc 350

agcaggagga agagagattt ggatggctca gaggactggg tgtactacag 400

aatttctcac gaggagaagg acctgttttt taacttgacg gtcaatcaag 450

gatttctttc caatagctac atcatggaga agagatatgg gaacctctcc 500

catgttaaga tgatggcttc ctctgccccc ctctgccatc tcagtggcac 550

ggttctacag cagggcacca gagttgggac ggcagccctc agtgcctgcc 600

atggactgac tggatttttc caactaccac atggagactt tttcattgaa 650

cccgtgaaga agcatccact ggttgaggga gggtaccacc cgcacatcgt 700

ttacaggagg cagaaagttc cagaaaccaa ggagccaacc tgtggattaa 750

agggtattgt gactcacatg tcctcctggg ttgaagaatc tgttttgttc 800

ttttggtagt tttattaaaa catgacctat tcttactcaa gtctcttatc 850

tcctctgtat tctttttttt ttaatatctt catgacattc aaatctcttc 900

tgtattctct tgccagaaag tgtacattct ttttgcttgt ataaaccctt 950

tcacttgtc 959

<210> 178
<211> 229
<212> PRT
<213> Homo Sapien

<400> 178
Met Pro Cys Ala Gln Arg Ser Trp Leu Ala Asn Leu Ser Val Val
1 5 10 15

Ala Gln Leu Leu Asn Phe Gly Ala Leu Cys Tyr Gly Arg Gln Pro
20 25 30

Gln Pro Gly Pro Val Arg Phe Pro Asp Arg Arg Gln Glu His Phe
35 40 45

Ile Lys Gly Leu Pro Glu Tyr His Val Val Gly Pro Val Arg Val
50 55 60

Asp Ala Ser Gly His Phe Leu Ser Tyr Gly Leu His Tyr Pro Ile
65 70 75

Thr Ser Ser Arg Arg Lys Arg Asp Leu Asp Gly Ser Glu Asp Trp
80 85 90

Val Tyr Tyr Arg Ile Ser His Glu Glu Lys Asp Leu Phe Phe Asn
95 100 105

Leu Thr Val Asn Gln Gly Phe Leu Ser Asn Ser Tyr Ile Met Glu
110 115 120

Lys Arg Tyr Gly Asn Leu Ser His Val Lys Met Met Ala Ser Ser
125 130 135

Ala Pro Leu Cys His Leu Ser Gly Thr Val Leu Gln Gln Gly Thr
140 145 150

```
Arg Val Gly Thr Ala Ala Leu Ser Ala Cys His Gly Leu Thr Gly
                155                 160                 165

Phe Phe Gln Leu Pro His Gly Asp Phe Phe Ile Glu Pro Val Lys
                170                 175                 180

Lys His Pro Leu Val Glu Gly Gly Tyr His Pro His Ile Val Tyr
                185                 190                 195

Arg Arg Gln Lys Val Pro Glu Thr Lys Glu Pro Thr Cys Gly Leu
                200                 205                 210

Lys Gly Ile Val Thr His Met Ser Ser Trp Val Glu Glu Ser Val
                215                 220                 225

Leu Phe Phe Trp


<210> 179
<211> 2134
<212> DNA
<213> Homo Sapien

<220>
<221> unsure
<222> 2108
<223> unknown base

<400> 179
cagatttaaa aagaaaacct ttactgaatc agctgagtgt taataatacg 50

aatttccttt tcttgccaat tctgatctga acagaaaatc caagaacagg 100

gatatgtgtg gattacagtt ttctctgcct tgcctacgac tgtttctggt 150

tgttacctgt tatcttttat tattactcca caaagaaata cttggatgtt 200

cgtctgtttg tcagctctgc actgggagac aaattaactg ccgtaactta 250

ggcctttcga gtattcctaa gaattttcct gaaagtacag tttttctgta 300

tctgactggg aataatatat cttatataaa tgaaagtgaa ttaacaggac 350

ttcattctct tgtagcattg tatttggata attctaacat tctgtatgta 400

tatccaaaag cctttgttca attgaggcat ctatattttc tatttctaaa 450

taataatttc atcaaacgct tagatcctgg aatatttaag ggacttttaa 500

atcttcgtaa tttatattta cagtataatc aggtatcttt tgttccgaga 550

ggagtattta atgatctagt ttcagttcag tacttaaatc tacaaaggaa 600

tcgcctcact gtccttggga gtggtacctt tgttggtatg gttgctcttc 650

ggatacttga tttatcaaac aataacattt tgaggatatc agaatcaggc 700

tttcaacatc ttgaaaacct tgcttgtttg tatttaggaa gtaataattt 750

aacaaaagta ccatcaaatg cctttgaagt acttaaaagt cttagaagac 800

tttctttgtc tcataatcct attgaagcaa tacagccctt tgcatttaaa 850
```

ggacttgcca atctggaata cctcctcctg aaaaattcaa gaattaggaa 900

tgttactagg gatgggttta gtggaattaa taatcttaaa catttgatct 950

taagtcataa tgatttagag aatttaaatt ctgacacatt cagtttgtta 1000

aagaatttaa tttaccttaa gttagataga aacagaataa ttagcattga 1050

taatgataca tttgaaaata tgggagcatc tttgaagatc cttaatctgt 1100

catttaataa tcttacagcc ttgcatccaa gggtccttaa gccgttgtct 1150

tcattgattc atcttcaggc aaattctaat ccttgggaat gtaactgcaa 1200

acttttgggc cttcgagact ggctagcatc ttcagccatt actctaaaca 1250

tctattgtca gaatccccca tccatgcgtg gcagagcatt acgttatatt 1300

aacattacaa attgtgttac atcttcaata aatgtatcca gagcttgggc 1350

tgttgtaaaa tctcctcata ttcatcacaa gactactgcg ctaatgatgg 1400

cctggcataa agtaaccaca aatggcagtc ctctggaaaa tactgagact 1450

gagaacatta ctttctggga acgaattcct acttcacctg ctggtagatt 1500

ttttcaagag aatgcctttg gtaatccatt agagactaca gcagtgttac 1550

ctgtgcaaat acaacttact acttctgtta ccttgaactt ggaaaaaaac 1600

agtgctctac cgaatgatgc tgcttcaatg tcagggaaaa catctctaat 1650

ttgtacacaa gaagttgaga agttgaatga ggcttttgac attttgctag 1700

ctttttttcat cttagcttgt gttttaatca tttttttgat ctacaaagtt 1750

gttcagttta aacaaaaact aaaggcatca gaaaactcaa gggaaaatag 1800

acttgaatac tacagctttt atcagtcagc aaggtataat gtaactgcct 1850

caatttgtaa cacttcccca aattctctag aaagtcctgg cttggagcag 1900

attcgacttc ataaacaaat tgttcctgaa aatgaggcac aggtcattct 1950

ttttgaacat tctgctttat aactcaacta aatattgtct ataagaaact 2000

tcagtgccat ggacatgatt taaactgaaa cctccttata taattatata 2050

ctttagttgg aaatataatg aattatatga ggttagcatt attaaaatat 2100

gtttttntt aaaaaaaaaa aaaaaaaaaa aaaa 2134

<210> 180
<211> 622
<212> PRT
<213> Homo Sapien

<400> 180
Met Cys Gly Leu Gln Phe Ser Leu Pro Cys Leu Arg Leu Phe Leu
1 5 10 15

Val Val Thr Cys Tyr Leu Leu Leu Leu Leu His Lys Glu Ile Leu
20 25 30

Gly Cys Ser Ser Val Cys Gln Leu Cys Thr Gly Arg Gln Ile Asn
35 40 45

Cys Arg Asn Leu Gly Leu Ser Ser Ile Pro Lys Asn Phe Pro Glu
50 55 60

Ser Thr Val Phe Leu Tyr Leu Thr Gly Asn Asn Ile Ser Tyr Ile
65 70 75

Asn Glu Ser Glu Leu Thr Gly Leu His Ser Leu Val Ala Leu Tyr
80 85 90

Leu Asp Asn Ser Asn Ile Leu Tyr Val Tyr Pro Lys Ala Phe Val
95 100 105

Gln Leu Arg His Leu Tyr Phe Leu Phe Leu Asn Asn Asn Phe Ile
110 115 120

Lys Arg Leu Asp Pro Gly Ile Phe Lys Gly Leu Leu Asn Leu Arg
125 130 135

Asn Leu Tyr Leu Gln Tyr Asn Gln Val Ser Phe Val Pro Arg Gly
140 145 150

Val Phe Asn Asp Leu Val Ser Val Gln Tyr Leu Asn Leu Gln Arg
155 160 165

Asn Arg Leu Thr Val Leu Gly Ser Gly Thr Phe Val Gly Met Val
170 175 180

Ala Leu Arg Ile Leu Asp Leu Ser Asn Asn Asn Ile Leu Arg Ile
185 190 195

Ser Glu Ser Gly Phe Gln His Leu Glu Asn Leu Ala Cys Leu Tyr
200 205 210

Leu Gly Ser Asn Asn Leu Thr Lys Val Pro Ser Asn Ala Phe Glu
215 220 225

Val Leu Lys Ser Leu Arg Arg Leu Ser Leu Ser His Asn Pro Ile
230 235 240

Glu Ala Ile Gln Pro Phe Ala Phe Lys Gly Leu Ala Asn Leu Glu
245 250 255

Tyr Leu Leu Leu Lys Asn Ser Arg Ile Arg Asn Val Thr Arg Asp
260 265 270

Gly Phe Ser Gly Ile Asn Asn Leu Lys His Leu Ile Leu Ser His
275 280 285

Asn Asp Leu Glu Asn Leu Asn Ser Asp Thr Phe Ser Leu Leu Lys
290 295 300

Asn Leu Ile Tyr Leu Lys Leu Asp Arg Asn Arg Ile Ile Ser Ile
305 310 315

Asp Asn Asp Thr Phe Glu Asn Met Gly Ala Ser Leu Lys Ile Leu
320 325 330

Asn Leu Ser Phe Asn Asn Leu Thr Ala Leu His Pro Arg Val Leu
335 340 345

Lys Pro Leu Ser Ser Leu Ile His Leu Gln Ala Asn Ser Asn Pro

```
                350                 355                 360

Trp Glu Cys Asn Cys Lys Leu Leu Gly Leu Arg Asp Trp Leu Ala
                365                 370                 375

Ser Ser Ala Ile Thr Leu Asn Ile Tyr Cys Gln Asn Pro Pro Ser
                380                 385                 390

Met Arg Gly Arg Ala Leu Arg Tyr Ile Asn Ile Thr Asn Cys Val
                395                 400                 405

Thr Ser Ser Ile Asn Val Ser Arg Ala Trp Ala Val Val Lys Ser
                410                 415                 420

Pro His Ile His His Lys Thr Thr Ala Leu Met Met Ala Trp His
                425                 430                 435

Lys Val Thr Thr Asn Gly Ser Pro Leu Glu Asn Thr Glu Thr Glu
                440                 445                 450

Asn Ile Thr Phe Trp Glu Arg Ile Pro Thr Ser Pro Ala Gly Arg
                455                 460                 465

Phe Phe Gln Glu Asn Ala Phe Gly Asn Pro Leu Glu Thr Thr Ala
                470                 475                 480

Val Leu Pro Val Gln Ile Gln Leu Thr Thr Ser Val Thr Leu Asn
                485                 490                 495

Leu Glu Lys Asn Ser Ala Leu Pro Asn Asp Ala Ala Ser Met Ser
                500                 505                 510

Gly Lys Thr Ser Leu Ile Cys Thr Gln Glu Val Glu Lys Leu Asn
                515                 520                 525

Glu Ala Phe Asp Ile Leu Leu Ala Phe Phe Ile Leu Ala Cys Val
                530                 535                 540

Leu Ile Ile Phe Leu Ile Tyr Lys Val Val Gln Phe Lys Gln Lys
                545                 550                 555

Leu Lys Ala Ser Glu Asn Ser Arg Glu Asn Arg Leu Glu Tyr Tyr
                560                 565                 570

Ser Phe Tyr Gln Ser Ala Arg Tyr Asn Val Thr Ala Ser Ile Cys
                575                 580                 585

Asn Thr Ser Pro Asn Ser Leu Glu Ser Pro Gly Leu Glu Gln Ile
                590                 595                 600

Arg Leu His Lys Gln Ile Val Pro Glu Asn Glu Ala Gln Val Ile
                605                 610                 615

Leu Phe Glu His Ser Ala Leu
                620
```

```
<210> 181
<211> 1624
<212> DNA
<213> Homo Sapien

<220>
<221> unsure
<222> 1560-1561
```

<223> unknown base

<400> 181
ggcctggcgc ggcgctccgg taaggcgtgt gtgcggcagg gcggggacag 50
aaccgtcctc tcgggctctg ggcgtgtccg agaccgcgct ccccgccgaa 100
atcaagctcc gagtcatccg tgtggggcat tcgtcccccc tggcacagtt 150
ggcctctttc cagaagcccg ttttgtttgt tttacgtcta aattcgcgtc 200
ggttcttatt tctctccctg gcaaggtctg aagacgggta ggagaataac 250
ctgtgtcagc gtgttatgat gccgtcccgt accaacctgg ctactggaat 300
ccccagtagt aaagtgaaat attcaaggct ctccagcaca gacgatggct 350
acattgacct tcagtttaag aaaacccctc ctaagatccc ttataaggcc 400
atcgcacttg ccactgtgct gtttttgatt ggcgcctttc tcattattat 450
aggctccctc ctgctgtcag gctacatcag caaagggggg gcagaccggg 500
ccgttccagt gctgatcatt ggcattctgg tgttcctacc cggattttac 550
cacctgcgca tcgcttacta tgcatccaaa ggctaccgtg gttactccta 600
tgatgacatt ccagactttg atgactagca cccaccccat agctgaggag 650
gagtcacagt ggaactgtcc cagctttaag atatctagca gaaactatag 700
ctgaggacta aggaattctg cagcttgcag atgtttaaga aaataatggc 750
cagatttttt gggtccttcc caaagatgtt aagtgaacct acagttagct 800
aattaggaca agctctattt ttcatccctg ggccctgaca agtttttcca 850
caggaatatg tatcatggaa gaatagaggt tattctgtaa tggaaaagtg 900
ttgcctgcca ccaccctctg tagagctgag catttctttt aaatagtctt 950
cattgccaat ttgttcttgt agcaaatgga acaatgtggt atggctaatt 1000
tcttattatt aagtagttta ttttaaaaat atctgagtat attatcctgt 1050
acacttatcc ctaccttcat gttccagtgg aagaccttag taaaatcaaa 1100
gatcagtgag ttcatctgta atattttttt tacttgcttt cttactgaca 1150
gcaaccagga attttttat cctgcagagc aagttttcaa aatgtaaata 1200
cttcctctgt ttaacagtcc ttggaccatt ctgatccagt tcaccagtag 1250
gttggacagc atataatttg catcattttg tcccttgtaa atcaagatgt 1300
tctgcagatt attcctttaa cggccggact tttggctgtt tcctaatgaa 1350
acatgtagtg gttattattt agagtttata gccgtattgc tagcaccttg 1400
tagtatgtca tcattctgct catgattcca aggatcagcc tggatgccta 1450
gaggactaga tcaccttagt ttgattctat tttttagctt gcaaaaagtg 1500
acttatattc caaagaaatt aaaatgttga aatccaaatc ctagaaataa 1550

aatgagtttn nttccaaaaa aaaaaaaaaa aaaaaaaaaa aaaaaaaaaa 1600

aaaaaaaaaa aaaaaaaaaa aaaa 1624

<210> 182
<211> 120
<212> PRT
<213> Homo Sapien

<400> 182
Met Met Pro Ser Arg Thr Asn Leu Ala Thr Gly Ile Pro Ser Ser
1 5 10 15

Lys Val Lys Tyr Ser Arg Leu Ser Ser Thr Asp Asp Gly Tyr Ile
20 25 30

Asp Leu Gln Phe Lys Lys Thr Pro Pro Lys Ile Pro Tyr Lys Ala
35 40 45

Ile Ala Leu Ala Thr Val Leu Phe Leu Ile Gly Ala Phe Leu Ile
50 55 60

Ile Ile Gly Ser Leu Leu Leu Ser Gly Tyr Ile Ser Lys Gly Gly
65 70 75

Ala Asp Arg Ala Val Pro Val Leu Ile Ile Gly Ile Leu Val Phe
80 85 90

Leu Pro Gly Phe Tyr His Leu Arg Ile Ala Tyr Tyr Ala Ser Lys
95 100 105

Gly Tyr Arg Gly Tyr Ser Tyr Asp Asp Ile Pro Asp Phe Asp Asp
110 115 120

<210> 183
<211> 2823
<212> DNA
<213> Homo Sapien

<400> 183
ctaaaaaata caaaaattag ctgggcgtgg tgtcatgtac ctgtaatccc 50

agctactcaa gaggctgagg caggagaatc gcttgaaccc aggaggcaga 100

ggttgcagtg agccaagatt aagtcactgc actccagcct gggtgacaga 150

gcaagactct gtatcaaaat aaataaataa agtacaactc tggatgggca 200

tggtggctta tgtctgtaat cccagcactt tgggaacttg aggcgggtag 250

attgcttgag tccgggagtt tgagaccagt ctgggtaata tggtaaccct 300

gtctaccaaa aatacaggta ttagccagtc tcataactcg gtctcaaaat 350

aaataaatac atacatacat agatgaaaat ttaaaaaata aagtccaact 400

cagcggtttt cagcatattt acagagttgt acaatcttca ccactatcta 450

atttcagaac attttcatca cccccaaaag aaacctaacc cattgactat 500

ctctccattt cctccctctc cctagcctct ggcaaccact aatctctttt 550

ttgtctctat agatttgcct attttggaca gttcatatac aaggaatcat 600

accacatgta gccttttgtg tccggcttct ttgattaata gaatgttttc 650
aaggctcatc tatgctgtag cctgtatcag cacttcattc ctttctatgg 700
ctgaataata gtccactgta gggatgtgcc atgtttttcc actagctgat 750
ggacatttgg gttgtttcca ccttctggct attataaata ttgctgctat 800
aaatattcac ttacaagttt ttgtgtggac atatgttttt atttcttctg 850
gtatatcctt cggagtggaa ctgctggatc aggtggtaac tctaggtcta 900
acctggcagt taaacagaat cctatgcatg ctgtagtcca tgagttgaaa 950
taaacacttg acccatagta agtgccagat catcttcatt tcacagcaac 1000
cagtaatttc acagatgagg aaatgaaggc tcccagaggt gaactggctt 1050
ttcccatttg agcagttcca agtcagacag ttaaaaagtg gcaggacctg 1100
gaagagaagc tagttctttc accctggcat tcagggctgc ctcctgggct 1150
acggggctgg catttagaat agagctaagg tctgctgcca aggcaggtgc 1200
cccagtctgc ctcctctgtg tccttattcc actttctctg cagccctcca 1250
ggggacccct ctctcagcca ccctctctct ggtgatgtca cagtgctgcc 1300
ggaagatcaa agatacggtg cagaaactgg cttcggacca taaggacatt 1350
cacagcagtg tatcccgagt gggcaaagcc attgacagga acttcgactc 1400
tgagatctgt ggtgttgtgt cagatgcggt gtgggacgcg cgggaacagc 1450
agcagcagat cctgcagatg gccatcgtgg aacacctgta tcagcagggc 1500
atgctcagcg tggccgagga gctgtgccag gaatcaacgc tgaatgtgga 1550
cttggatttc aagcagcctt tcctagagtt gaatcgaatc ctggaagccc 1600
tgcacgaaca agacctgggt cctgcgttgg aatgggccgt ctcccacagg 1650
cagcgcctgc tggaactcaa cagctccctg gagttcaagc tgcaccgact 1700
gcacttcatc cgcctcttgg caggaggccc cgcgaagcag ctggaggccc 1750
tcagctatgc tcggcacttc cagccctttg ctcggctgca ccagcgggag 1800
atccaggtga tgatgggcag cctggtgtac ctgcggctgg gcttggagaa 1850
gtcaccctac tgccacctgc tggacagcag ccactgggca gagatctgtg 1900
agacctttac ccgggacgcc tgttccctgc tggggctttc tgtggagtcc 1950
ccccttagcg tcagctttgc ctctggctgt gtggcgctgc ctgtgttgat 2000
gaacatcaag gctgtgattg agcagcggca gtgcactggg gtctggaatc 2050
acaaggacga gttaccgatt gagattgaac taggcatgaa gtgctggtac 2100
gctcatctgt ggccatgtta tctcccgaga tgcactcaat aagctcatta 2150
atggaggaaa cactccgtgt tcgcttgccc catcctccgc cagcagacgt 2200

cagattccaa ccctcccatc aagctgaagt gtccctactg tcccatggag 2250

cagaacccgg cagatgggaa acgcatcata ttctgattcc tacctggaag 2300

gaattttgtt gaaaggggtt ttcacctgtg agccttggtc tgtctcggta 2350

gggtggtcaa cttcagtgga ctgtggttgg tttcagagcg cctggctgag 2400

gagttccact gaggggagca ctggagcagc cctttggcag aggctgagga 2450

gggagatgga ccagcccacg cctggcacct ggctccatgg cataaggaaa 2500

gggagatgct ggcctctgtg ctcctgctgt cttttcctgt ttctgtttgc 2550

gtttgactta gtagcaaccg acagagtggc aagggatttg gtcttcagca 2600

gtagacatcc ttccacccct gccctcagcc aagtctcttg ctgccatgcc 2650

aatgctatgt ccacccttgc ccctcggccc aagagtgtcc agcggtggcc 2700

cacctcttcc tcccactaca gcctcaacag tatgtaccat ctcccactgt 2750

aaatagtccc agttagaacg gaatgccgtt gttttataac tttgaacaaa 2800

tgtatttact gcccttctca aaa 2823

<210> 184
<211> 331
<212> PRT
<213> Homo Sapien

<400> 184

```
Gln Cys Cys Arg Lys Ile Lys Asp Thr Val Gln Lys Leu Ala Ser
1               5                   10                  15

Asp His Lys Asp Ile His Ser Ser Val Ser Arg Val Gly Lys Ala
                20                  25                  30

Ile Asp Arg Asn Phe Asp Ser Glu Ile Cys Gly Val Val Ser Asp
                35                  40                  45

Ala Val Trp Asp Ala Arg Glu Gln Gln Gln Gln Ile Leu Gln Met
                50                  55                  60

Ala Ile Val Glu His Leu Tyr Gln Gln Gly Met Leu Ser Val Ala
                65                  70                  75

Glu Glu Leu Cys Gln Glu Ser Thr Leu Asn Val Asp Leu Asp Phe
                80                  85                  90

Lys Gln Pro Phe Leu Glu Leu Asn Arg Ile Leu Glu Ala Leu His
                95                  100                 105

Glu Gln Asp Leu Gly Pro Ala Leu Glu Trp Ala Val Ser His Arg
                110                 115                 120

Gln Arg Leu Leu Glu Leu Asn Ser Ser Leu Glu Phe Lys Leu His
                125                 130                 135

Arg Leu His Phe Ile Arg Leu Leu Ala Gly Gly Pro Ala Lys Gln
                140                 145                 150

Leu Glu Ala Leu Ser Tyr Ala Arg His Phe Gln Pro Phe Ala Arg
```

155 160 165

Leu His Gln Arg Glu Ile Gln Val Met Met Gly Ser Leu Val Tyr
170 175 180

Leu Arg Leu Gly Leu Glu Lys Ser Pro Tyr Cys His Leu Leu Asp
185 190 195

Ser Ser His Trp Ala Glu Ile Cys Glu Thr Phe Thr Arg Asp Ala
200 205 210

Cys Ser Leu Leu Gly Leu Ser Val Glu Ser Pro Leu Ser Val Ser
215 220 225

Phe Ala Ser Gly Cys Val Ala Leu Pro Val Leu Met Asn Ile Lys
230 235 240

Ala Val Ile Glu Gln Arg Gln Cys Thr Gly Val Trp Asn His Lys
245 250 255

Asp Glu Leu Pro Ile Glu Ile Glu Leu Gly Met Lys Cys Trp Tyr
260 265 270

His Ser Val Phe Ala Cys Pro Ile Leu Arg Gln Gln Thr Ser Asp
275 280 285

Ser Asn Pro Pro Ile Lys Leu Ile Cys Gly His Val Ile Ser Arg
290 295 300

Asp Ala Leu Asn Lys Leu Ile Asn Gly Gly Lys Leu Lys Cys Pro
305 310 315

Tyr Cys Pro Met Glu Gln Asn Pro Ala Asp Gly Lys Arg Ile Ile
320 325 330

Phe

<210> 185
<211> 1162
<212> DNA
<213> Homo Sapien

<400> 185
gagcgacgct gtctctagtc gctgatccca aatgcaccgg ctcatctttg 50

tctacactct aatctgcgca aacttttgca gctgtcggga cacttctgca 100

accccgcaga gcgcatccat caaagctttg cgcaacgcca acctcaggcg 150

agatgacttg taccgaagag atgagaccat ccaggtgaaa ggaaacggct 200

acgtgcagag tcctagattc ccgaacagct accccaggaa cctgctcctg 250

acatggcggc ttcactctca ggagaataca cggatacagc tagtgtttga 300

caatcagttt ggattagagg aagcagaaaa tgatatctgt aggtatgatt 350

ttgtggaagt tgaagatata tccgaaacca gtaccattat tagaggacga 400

tggtgtggac acaaggaagt tcctccaagg ataaaatcaa gaacgaacca 450

aattaaaatc acattcaagt ccgatgacta ctttgtggct aaacctggat 500

tcaagattta ttattctttg ctggaagatt tccaacccgc agcagcttca 550

gagaccaact gggaatctgt cacaagctct atttcagggg tatcctataa 600

ctctccatca gtaacggatc ccactctgat tgcggatgct ctggacaaaa 650

aaattgcaga atttgataca gtggaagatc tgctcaagta cttcaatcca 700

gagtcatggc aagaagatct tgagaatatg tatctggaca cccctcggta 750

tcgaggcagg tcataccatg accggaagtc aaaagttgac ctggataggc 800

tcaatgatga tgccaagcgt tacagttgca ctcccaggaa ttactcggtc 850

aatataagag aagagctgaa gttggccaat gtggtcttct ttccacgttg 900

cctcctcgtg cagcgctgtg gaggaaattg tggctgtgga actgtcaact 950

ggaggtcctg cacatgcaat tcagggaaaa ccgtgaaaaa gtatcatgag 1000

gtattacagt ttgagcctgg ccacatcaag aggaggggta gagctaagac 1050

catggctcta gttgacatcc agttggatca ccatgaacga tgcgattgta 1100

tctgcagctc aagaccacct cgataagaga atgtgcacat ccttacatta 1150

agcctgagag aa 1162

<210> 186
<211> 364
<212> PRT
<213> Homo Sapien

<400> 186

Met His Arg Leu Ile Phe Val Tyr Thr Leu Ile Cys Ala Asn Phe
1 5 10 15

Cys Ser Cys Arg Asp Thr Ser Ala Thr Pro Gln Ser Ala Ser Ile
20 25 30

Lys Ala Leu Arg Asn Ala Asn Leu Arg Arg Asp Asp Leu Tyr Arg
35 40 45

Arg Asp Glu Thr Ile Gln Val Lys Gly Asn Gly Tyr Val Gln Ser
50 55 60

Pro Arg Phe Pro Asn Ser Tyr Pro Arg Asn Leu Leu Leu Thr Trp
65 70 75

Arg Leu His Ser Gln Glu Asn Thr Arg Ile Gln Leu Val Phe Asp
80 85 90

Asn Gln Phe Gly Leu Glu Glu Ala Glu Asn Asp Ile Cys Arg Tyr
95 100 105

Asp Phe Val Glu Val Glu Asp Ile Ser Glu Thr Ser Thr Ile Ile
110 115 120

Arg Gly Arg Trp Cys Gly His Lys Glu Val Pro Pro Arg Ile Lys
125 130 135

Ser Arg Thr Asn Gln Ile Lys Ile Thr Phe Lys Ser Asp Asp Tyr
140 145 150

Phe Val Ala Lys Pro Gly Phe Lys Ile Tyr Tyr Ser Leu Leu Glu
155 160 165

Asp Phe Gln Pro Ala Ala Ala Ser Glu Thr Asn Trp Glu Ser Val
170 175 180

Thr Ser Ser Ile Ser Gly Val Ser Tyr Asn Ser Pro Ser Val Thr
185 190 195

Asp Pro Thr Leu Ile Ala Asp Ala Leu Asp Lys Lys Ile Ala Glu
200 205 210

Phe Asp Thr Val Glu Asp Leu Leu Lys Tyr Phe Asn Pro Glu Ser
215 220 225

Trp Gln Glu Asp Leu Glu Asn Met Tyr Leu Asp Thr Pro Arg Tyr
230 235 240

Arg Gly Arg Ser Tyr His Asp Arg Lys Ser Lys Val Asp Leu Asp
245 250 255

Arg Leu Asn Asp Asp Ala Lys Arg Tyr Ser Cys Thr Pro Arg Asn
260 265 270

Tyr Ser Val Asn Ile Arg Glu Glu Leu Lys Leu Ala Asn Val Val
275 280 285

Phe Phe Pro Arg Cys Leu Leu Val Gln Arg Cys Gly Gly Asn Cys
290 295 300

Gly Cys Gly Thr Val Asn Trp Arg Ser Cys Thr Cys Asn Ser Gly
305 310 315

Lys Thr Val Lys Lys Tyr His Glu Val Leu Gln Phe Glu Pro Gly
320 325 330

His Ile Lys Arg Arg Gly Arg Ala Lys Thr Met Ala Leu Val Asp
335 340 345

Ile Gln Leu Asp His His Glu Arg Cys Asp Cys Ile Cys Ser Ser
350 355 360

Arg Pro Pro Arg

<210> 187
<211> 1750
<212> DNA
<213> Homo Sapien

<400> 187
catgccgctg ccgccgctgc tgctgttgct cctggcggcg ccttggggac 50

gggcagttcc ctgtgtctct ggtggtttgc ctaaacctgc aaacatcacc 100

ttcttatcca tcaacatgaa gaatgtccta caatggactc caccagaggg 150

tcttcaagga gttaaagtta cttacactgt gcagtatttc atatatgggc 200

aaaagaaatg gctgaataaa tcagaatgca gaaatatcaa tagaacctac 250

tgtgatcttt ctgctgaaac ttctgactac gaacaccagt attatgccaa 300

agttaaggcc atttggggaa caaagtgttc caaatgggct gaaagtggac 350

ggttctatcc ttttttagaa acacaaattg gcccaccaga ggtggcactg 400

actacagatg agaagtccat ttctgttgtc ctgacagctc cagagaagtg 450

gaagagaaat ccagaagacc ttcctgtttc catgcaacaa atatactcca 500

atctgaagta taacgtgtct gtgttgaata ctaaatcaaa cagaacgtgg 550

tcccagtgtg tgaccaacca cacgctggtg ctcacctggc tggagccgaa 600

cactctttac tgcgtacacg tggagtcctt cgtcccaggg ccccctcgcc 650

gtgctcagcc ttctgagaag cagtgtgcca ggactttgaa agatcaatca 700

tcagagttca aggctaaaat catcttctgg tatgttttgc ccatatctat 750

taccgtgttt cttttttctg tgatgggcta ttccatctac cgatatatcc 800

acgttggcaa agagaaacac ccagcaaatt tgattttgat ttatggaaat 850

gaatttgaca aaagattctt tgtgcctgct gaaaaaatcg tgattaactt 900

tatcaccctc aatatctcgg atgattctaa aatttctcat caggatatga 950

gtttactggg aaaaagcagt gatgtatcca gccttaatga tcctcagccc 1000

agcgggaacc tgaggccccc tcaggaggaa gaggaggtga aacatttagg 1050

gtatgcttcg catttgatgg aaattttttg tgactctgaa gaaaacacgg 1100

aaggtacttc tctcacccag caagagtccc tcagcagaac aataccccccg 1150

gataaaacag tcattgaata tgaatatgat gtcagaacca ctgacatttg 1200

tgcggggcct gaagagcagg agctcagttt gcaggaggag gtgtccacac 1250

aaggaacatt attggagtcg caggcagcgt tggcagtctt gggcccgcaa 1300

acgttacagt actcatacac ccctcagctc caagacttag accccctggc 1350

gcaggagcac acagactcgg aggaggggcc ggaggaagag ccatcgacga 1400

ccctggtcga ctgggatccc caaactggca ggctgtgtat tccttcgctg 1450

tccagcttcg accaggattc agagggctgc gagccttctg agggggatgg 1500

gctcggagag gagggtcttc tatctagact ctatgaggag ccggctccag 1550

acaggccacc aggagaaaat gaaacctatc tcatgcaatt catggaggaa 1600

tgggggttat atgtgcagat ggaaaactga tgccaacact tccttttgcc 1650

ttttgtttcc tgtgcaaaca agtgagtcac ccctttgatc ccagccataa 1700

agtacctggg atgaaagaag ttttttccag tttgtcagtg tctgtgagaa 1750

<210> 188
<211> 542
<212> PRT
<213> Homo Sapien

<400> 188
Met Pro Leu Pro Pro Leu Leu Leu Leu Leu Leu Ala Ala Pro Trp

```
1               5                   10                  15
Gly Arg Ala Val Pro Cys Val Ser Gly Gly Leu Pro Lys Pro Ala
                20                  25                  30
Asn Ile Thr Phe Leu Ser Ile Asn Met Lys Asn Val Leu Gln Trp
                35                  40                  45
Thr Pro Pro Glu Gly Leu Gln Gly Val Lys Val Thr Tyr Thr Val
                50                  55                  60
Gln Tyr Phe Ile Tyr Gly Gln Lys Lys Trp Leu Asn Lys Ser Glu
                65                  70                  75
Cys Arg Asn Ile Asn Arg Thr Tyr Cys Asp Leu Ser Ala Glu Thr
                80                  85                  90
Ser Asp Tyr Glu His Gln Tyr Tyr Ala Lys Val Lys Ala Ile Trp
                95                  100                 105
Gly Thr Lys Cys Ser Lys Trp Ala Glu Ser Gly Arg Phe Tyr Pro
                110                 115                 120
Phe Leu Glu Thr Gln Ile Gly Pro Pro Glu Val Ala Leu Thr Thr
                125                 130                 135
Asp Glu Lys Ser Ile Ser Val Val Leu Thr Ala Pro Glu Lys Trp
                140                 145                 150
Lys Arg Asn Pro Glu Asp Leu Pro Val Ser Met Gln Gln Ile Tyr
                155                 160                 165
Ser Asn Leu Lys Tyr Asn Val Ser Val Leu Asn Thr Lys Ser Asn
                170                 175                 180
Arg Thr Trp Ser Gln Cys Val Thr Asn His Thr Leu Val Leu Thr
                185                 190                 195
Trp Leu Glu Pro Asn Thr Leu Tyr Cys Val His Val Glu Ser Phe
                200                 205                 210
Val Pro Gly Pro Pro Arg Arg Ala Gln Pro Ser Glu Lys Gln Cys
                215                 220                 225
Ala Arg Thr Leu Lys Asp Gln Ser Ser Glu Phe Lys Ala Lys Ile
                230                 235                 240
Ile Phe Trp Tyr Val Leu Pro Ile Ser Ile Thr Val Phe Leu Phe
                245                 250                 255
Ser Val Met Gly Tyr Ser Ile Tyr Arg Tyr Ile His Val Gly Lys
                260                 265                 270
Glu Lys His Pro Ala Asn Leu Ile Leu Ile Tyr Gly Asn Glu Phe
                275                 280                 285
Asp Lys Arg Phe Phe Val Pro Ala Glu Lys Ile Val Ile Asn Phe
                290                 295                 300
Ile Thr Leu Asn Ile Ser Asp Asp Ser Lys Ile Ser His Gln Asp
                305                 310                 315
Met Ser Leu Leu Gly Lys Ser Ser Asp Val Ser Ser Leu Asn Asp
                320                 325                 330
```

Pro Gln Pro Ser Gly Asn Leu Arg Pro Pro Gln Glu Glu Glu Glu
335 340 345

Val Lys His Leu Gly Tyr Ala Ser His Leu Met Glu Ile Phe Cys
350 355 360

Asp Ser Glu Glu Asn Thr Glu Gly Thr Ser Leu Thr Gln Gln Glu
365 370 375

Ser Leu Ser Arg Thr Ile Pro Pro Asp Lys Thr Val Ile Glu Tyr
380 385 390

Glu Tyr Asp Val Arg Thr Thr Asp Ile Cys Ala Gly Pro Glu Glu
395 400 405

Gln Glu Leu Ser Leu Gln Glu Glu Val Ser Thr Gln Gly Thr Leu
410 415 420

Leu Glu Ser Gln Ala Ala Leu Ala Val Leu Gly Pro Gln Thr Leu
425 430 435

Gln Tyr Ser Tyr Thr Pro Gln Leu Gln Asp Leu Asp Pro Leu Ala
440 445 450

Gln Glu His Thr Asp Ser Glu Glu Gly Pro Glu Glu Glu Pro Ser
455 460 465

Thr Thr Leu Val Asp Trp Asp Pro Gln Thr Gly Arg Leu Cys Ile
470 475 480

Pro Ser Leu Ser Ser Phe Asp Gln Asp Ser Glu Gly Cys Glu Pro
485 490 495

Ser Glu Gly Asp Gly Leu Gly Glu Glu Gly Leu Leu Ser Arg Leu
500 505 510

Tyr Glu Glu Pro Ala Pro Asp Arg Pro Pro Gly Glu Asn Glu Thr
515 520 525

Tyr Leu Met Gln Phe Met Glu Glu Trp Gly Leu Tyr Val Gln Met
530 535 540

Glu Asn

<210> 189
<211> 2150
<212> DNA
<213> Homo Sapien

<400> 189
atgtgctgct ggccgctgct cctgctgtgg gggctgctcc ccgggacggc 50

ggcgggggc tcgggccgaa cctatccgca ccggaccctc ctggactcgg 100

agggcaagta ctggctgggc tggagccagc ggggcagcca gatcgccttc 150

cgcctccagg tgcgcactgc aggctacgtg ggcttcggct tctcgcccac 200

cggggccatg gcgtccgccg acatcgtcgt gggcggggtg gcccacgggc 250

ggccctacct ccaggattat tttacaaatg caaatagaga gttgaaaaaa 300

gatgctcagc aagattacca tctagaatat gccatggaaa atagcacaca 350
cacaataatt gaatttacca gagagctgca tacatgtgac ataaatgaca 400
agagtataac ggatagcact gtgagagtga tctgggccta ccaccatgaa 450
gatgcaggag aagctggtcc caagtaccat gactccaata ggggcaccaa 500
gagtttgcgg ttattgaatc ctgagaaaac tagtgtgcta tctacagcct 550
taccatactt tgatctggta aatcaggacg tccccatccc aaacaaagat 600
acaacatatt ggtgccaaat gtttaagatt cctgtgttcc aagaaaagca 650
tcatgtaata aaggttgagc cagtgataca gagaggccat gagagtctgg 700
tgcaccacat cctgctctat cagtgcagca acaactttaa cgacagcgtt 750
ctggagtccg gccacgagtg ctatcacccc aacatgcccg atgcattcct 800
cacctgtgaa actgtgattt ttgcctgggc tattggtgga gagggctttt 850
cttatccacc tcatgttgga ttatcccttg gcactccatt agatccgcat 900
tatgtgctcc tagaagtcca ttatgataat cccacttatg aggaaggctt 950
aatagataat tctggactga ggttatttta cacaatggat ataaggaaat 1000
atgatgctgg ggtgattgag gctggcctct gggtgagcct cttccatacc 1050
atccctccag ggatgcctga gttccagtct gagggtcact gcactttgga 1100
gtgcctggaa gaggctctgg aagccgaaaa gccaagtgga attcatgtgt 1150
ttgctgttct tctccatgct cacctggctg gcagaggcat caggctgcgt 1200
cattttcgaa aagggaagga aatgaaatta cttgcctatg atgatgattt 1250
tgacttcaat ttccaggagt ttcagtatct aaaggaagaa caaacaatct 1300
taccaggaga taacctaatt actgagtgtc gctacaacac gaaagataga 1350
gctgagatga cttggggagg actaagcacc aggagtgaaa tgtgtctctc 1400
ataccttctt tattacccaa gaattaatct tactcgatgt gcaagtattc 1450
cagacattat ggaacaactt cagttcattg gggttaagga gatctacaga 1500
ccagtcacga cctggccttt cattatcaaa agtcccaagc aatataaaaa 1550
cctttctttc atggatgcta tgaataagtt taaatggact aaaaaggaag 1600
gtctctcctt caacaagctg gtcctcagcc tgccagtgaa tgtgagatgt 1650
tccaagacag acaatgctga gtggtcgatt caaggaatga cagcattacc 1700
tccagatata gaaagaccct ataaagcaga acctttggtg tgtggcacgt 1750
cttcttcctc ttccctgcac agagatttct ccatcaactt gcttgtttgc 1800
cttctgctac tcagctgcac gctgagcacc aagagcttgt gatcaaaatt 1850
ctgttggact tgacaatgtt ttctatgatc tgaacctgtc atttgaagta 1900

```
caggttaaag actgtgtcca ctttgggcat gaagagtgtg gagacttttc 1950

ttccccattt tccctccctc ctttttcctt tccatgttac atgagagaca 2000

tcaatcaggt tctcttctct ttcttagaaa tacctgatgt tatatataca 2050

tggtcaataa aataaaactg gcctgactta agataaccat tttaaaaaat 2100

tgggctgtca tgtgggaata aaagaattct ttctttccta aaaaaaaaaa 2150

<210> 190
<211> 613
<212> PRT
<213> Homo Sapien

<400> 190
Met Cys Cys Trp Pro Leu Leu Leu Leu Trp Gly Leu Leu Pro Gly
 1               5                  10                  15

Thr Ala Ala Gly Gly Ser Gly Arg Thr Tyr Pro His Arg Thr Leu
                20                  25                  30

Leu Asp Ser Glu Gly Lys Tyr Trp Leu Gly Trp Ser Gln Arg Gly
                35                  40                  45

Ser Gln Ile Ala Phe Arg Leu Gln Val Arg Thr Ala Gly Tyr Val
                50                  55                  60

Gly Phe Gly Phe Ser Pro Thr Gly Ala Met Ala Ser Ala Asp Ile
                65                  70                  75

Val Val Gly Gly Val Ala His Gly Arg Pro Tyr Leu Gln Asp Tyr
                80                  85                  90

Phe Thr Asn Ala Asn Arg Glu Leu Lys Lys Asp Ala Gln Gln Asp
                95                 100                 105

Tyr His Leu Glu Tyr Ala Met Glu Asn Ser Thr His Thr Ile Ile
               110                 115                 120

Glu Phe Thr Arg Glu Leu His Thr Cys Asp Ile Asn Asp Lys Ser
               125                 130                 135

Ile Thr Asp Ser Thr Val Arg Val Ile Trp Ala Tyr His His Glu
               140                 145                 150

Asp Ala Gly Glu Ala Gly Pro Lys Tyr His Asp Ser Asn Arg Gly
               155                 160                 165

Thr Lys Ser Leu Arg Leu Leu Asn Pro Glu Lys Thr Ser Val Leu
               170                 175                 180

Ser Thr Ala Leu Pro Tyr Phe Asp Leu Val Asn Gln Asp Val Pro
               185                 190                 195

Ile Pro Asn Lys Asp Thr Thr Tyr Trp Cys Gln Met Phe Lys Ile
               200                 205                 210

Pro Val Phe Gln Glu Lys His His Val Ile Lys Val Glu Pro Val
               215                 220                 225

Ile Gln Arg Gly His Glu Ser Leu Val His His Ile Leu Leu Tyr
               230                 235                 240
```

```
Gln Cys Ser Asn Asn Phe Asn Asp Ser Val Leu Glu Ser Gly His
                245                 250                 255

Glu Cys Tyr His Pro Asn Met Pro Asp Ala Phe Leu Thr Cys Glu
                260                 265                 270

Thr Val Ile Phe Ala Trp Ala Ile Gly Gly Glu Gly Phe Ser Tyr
                275                 280                 285

Pro Pro His Val Gly Leu Ser Leu Gly Thr Pro Leu Asp Pro His
                290                 295                 300

Tyr Val Leu Leu Glu Val His Tyr Asp Asn Pro Thr Tyr Glu Glu
                305                 310                 315

Gly Leu Ile Asp Asn Ser Gly Leu Arg Leu Phe Tyr Thr Met Asp
                320                 325                 330

Ile Arg Lys Tyr Asp Ala Gly Val Ile Glu Ala Gly Leu Trp Val
                335                 340                 345

Ser Leu Phe His Thr Ile Pro Pro Gly Met Pro Glu Phe Gln Ser
                350                 355                 360

Glu Gly His Cys Thr Leu Glu Cys Leu Glu Glu Ala Leu Glu Ala
                365                 370                 375

Glu Lys Pro Ser Gly Ile His Val Phe Ala Val Leu Leu His Ala
                380                 385                 390

His Leu Ala Gly Arg Gly Ile Arg Leu Arg His Phe Arg Lys Gly
                395                 400                 405

Lys Glu Met Lys Leu Leu Ala Tyr Asp Asp Asp Phe Asp Phe Asn
                410                 415                 420

Phe Gln Glu Phe Gln Tyr Leu Lys Glu Glu Gln Thr Ile Leu Pro
                425                 430                 435

Gly Asp Asn Leu Ile Thr Glu Cys Arg Tyr Asn Thr Lys Asp Arg
                440                 445                 450

Ala Glu Met Thr Trp Gly Gly Leu Ser Thr Arg Ser Glu Met Cys
                455                 460                 465

Leu Ser Tyr Leu Leu Tyr Tyr Pro Arg Ile Asn Leu Thr Arg Cys
                470                 475                 480

Ala Ser Ile Pro Asp Ile Met Glu Gln Leu Gln Phe Ile Gly Val
                485                 490                 495

Lys Glu Ile Tyr Arg Pro Val Thr Thr Trp Pro Phe Ile Ile Lys
                500                 505                 510

Ser Pro Lys Gln Tyr Lys Asn Leu Ser Phe Met Asp Ala Met Asn
                515                 520                 525

Lys Phe Lys Trp Thr Lys Lys Glu Gly Leu Ser Phe Asn Lys Leu
                530                 535                 540

Val Leu Ser Leu Pro Val Asn Val Arg Cys Ser Lys Thr Asp Asn
                545                 550                 555

Ala Glu Trp Ser Ile Gln Gly Met Thr Ala Leu Pro Pro Asp Ile
```

```
                560                 565                 570

Glu Arg Pro Tyr Lys Ala Glu Pro Leu Val Cys Gly Thr Ser Ser
                575                 580                 585

Ser Ser Ser Leu His Arg Asp Phe Ser Ile Asn Leu Leu Val Cys
                590                 595                 600

Leu Leu Leu Leu Ser Cys Thr Leu Ser Thr Lys Ser Leu
                605                 610
```

<210> 191
<211> 1647
<212> DNA
<213> Homo Sapien

<400> 191

```
gcttcagctg aagaaagaga ggaatgaagc gccttctgct tctgtttttg 50

ttctttataa cattttcttc tgcatttccc ttagtccgga tgacggaaaa 100

tgaagaaaat atgcaactgg ctcaggcata tctcaaccag ttctactctc 150

ttgaaataga agggaatcat cttgttcaaa gcaagaatag gagtctcata 200

gatgacaaaa ttcgggaaat gcaagcattt tttggattga cagtgactgg 250

aaaactggac tcaaacaccc ttgagatcat gaagacaccc aggtgtgggg 300

tgcctgatgt gggccagtat ggctacaccc tccctgggtg gagaaaatac 350

aacctcacct acagaataat aaactatact ccggatatgg cacgagctgc 400

tgtggatgag gctatccaag aaggtttaga agtgtggagc aaagtcactc 450

cactaaaatt caccaagatt tcaaagggga ttgcagacat catgattgcc 500

tttaggactc gagtccatgg tcggtgtcct cgctattttg atggtccctt 550

gggagtgctt ggccatgcct ttcctcctgg tccgggtctg ggtggtgaca 600

ctcattttga tgaggatgaa aactggacca aggatggagc aggattcaac 650

ttgtttcttg tggctgctca tgaatttggt catgcactgg ggctctctca 700

ctccaatgat caaacagcct tgatgttccc aaattatgtc tccctggatc 750

ccagaaaata cccactttct caggatgata tcaatggaat ccagtccatc 800

tatggaggtc tgcctaaggt acctgctaag ccaaaggaac ccactatacc 850

ccatgcctgt gaccctgact tgacttttga cgctatcaca actttccgca 900

gagaagtaat gttctttaaa ggcaggcacc tatggaggat ctattatgat 950

atcacggatg ttgagtttga attaattgct tcattctggc catctctgcc 1000

agctgatctg caagctgcat acgagaaccc cagagataag attctggttt 1050

ttaaagatga aaacttctgg atgatcagag gatatgctgt cttgccagat 1100

tatcccaaat ccatccatac attaggtttt ccaggacgtg tgaagaaaat 1150

agatgcagcc gtctgtgata agaccacaag aaaaacctac ttctttgtgg 1200
```

gcatttggtg ctggaggttt gatgaaatga cccaaaccat ggacaaagga 1250

ttcccgcaga gagtggtaaa acactttcct ggaatcagta tccgtgttga 1300

tgctgctttc cagtacaaag gattcttctt tttcagccgt ggatcaaagc 1350

aatttgaata caacattaag acaaagaata ttacccgaat catgagaact 1400

aatacttggt ttcaatgcaa agaaccaaag aactcctcat ttggttttga 1450

tatcaacaag gaaaaagcac attcaggagg cataaagata ttgtatcata 1500

agagtttaag cttgtttatt tttggtattg ttcatttgct gaaaaacact 1550

tctatttatc aataaattca tagacctaaa ataaacctca acaggtcttt 1600

taatataaat tctgcttcaa aatagaataa aaccattctt taacaac 1647

<210> 192
<211> 513
<212> PRT
<213> Homo Sapien

<400> 192
```
Met Lys Arg Leu Leu Leu Leu Phe Leu Phe Phe Ile Thr Phe Ser
1               5                   10                  15

Ser Ala Phe Pro Leu Val Arg Met Thr Glu Asn Glu Glu Asn Met
                20                  25                  30

Gln Leu Ala Gln Ala Tyr Leu Asn Gln Phe Tyr Ser Leu Glu Ile
                35                  40                  45

Glu Gly Asn His Leu Val Gln Ser Lys Asn Arg Ser Leu Ile Asp
                50                  55                  60

Asp Lys Ile Arg Glu Met Gln Ala Phe Phe Gly Leu Thr Val Thr
                65                  70                  75

Gly Lys Leu Asp Ser Asn Thr Leu Glu Ile Met Lys Thr Pro Arg
                80                  85                  90

Cys Gly Val Pro Asp Val Gly Gln Tyr Gly Tyr Thr Leu Pro Gly
                95                  100                 105

Trp Arg Lys Tyr Asn Leu Thr Tyr Arg Ile Ile Asn Tyr Thr Pro
                110                 115                 120

Asp Met Ala Arg Ala Ala Val Asp Glu Ala Ile Gln Glu Gly Leu
                125                 130                 135

Glu Val Trp Ser Lys Val Thr Pro Leu Lys Phe Thr Lys Ile Ser
                140                 145                 150

Lys Gly Ile Ala Asp Ile Met Ile Ala Phe Arg Thr Arg Val His
                155                 160                 165

Gly Arg Cys Pro Arg Tyr Phe Asp Gly Pro Leu Gly Val Leu Gly
                170                 175                 180

His Ala Phe Pro Pro Gly Pro Gly Leu Gly Gly Asp Thr His Phe
                185                 190                 195
```

```
Asp Glu Asp Glu Asn Trp Thr Lys Asp Gly Ala Gly Phe Asn Leu
                200                 205                 210

Phe Leu Val Ala Ala His Glu Phe Gly His Ala Leu Gly Leu Ser
                215                 220                 225

His Ser Asn Asp Gln Thr Ala Leu Met Phe Pro Asn Tyr Val Ser
                230                 235                 240

Leu Asp Pro Arg Lys Tyr Pro Leu Ser Gln Asp Asp Ile Asn Gly
                245                 250                 255

Ile Gln Ser Ile Tyr Gly Gly Leu Pro Lys Val Pro Ala Lys Pro
                260                 265                 270

Lys Glu Pro Thr Ile Pro His Ala Cys Asp Pro Asp Leu Thr Phe
                275                 280                 285

Asp Ala Ile Thr Thr Phe Arg Arg Glu Val Met Phe Phe Lys Gly
                290                 295                 300

Arg His Leu Trp Arg Ile Tyr Tyr Asp Ile Thr Asp Val Glu Phe
                305                 310                 315

Glu Leu Ile Ala Ser Phe Trp Pro Ser Leu Pro Ala Asp Leu Gln
                320                 325                 330

Ala Ala Tyr Glu Asn Pro Arg Asp Lys Ile Leu Val Phe Lys Asp
                335                 340                 345

Glu Asn Phe Trp Met Ile Arg Gly Tyr Ala Val Leu Pro Asp Tyr
                350                 355                 360

Pro Lys Ser Ile His Thr Leu Gly Phe Pro Gly Arg Val Lys Lys
                365                 370                 375

Ile Asp Ala Ala Val Cys Asp Lys Thr Thr Arg Lys Thr Tyr Phe
                380                 385                 390

Phe Val Gly Ile Trp Cys Trp Arg Phe Asp Glu Met Thr Gln Thr
                395                 400                 405

Met Asp Lys Gly Phe Pro Gln Arg Val Val Lys His Phe Pro Gly
                410                 415                 420

Ile Ser Ile Arg Val Asp Ala Ala Phe Gln Tyr Lys Gly Phe Phe
                425                 430                 435

Phe Phe Ser Arg Gly Ser Lys Gln Phe Glu Tyr Asn Ile Lys Thr
                440                 445                 450

Lys Asn Ile Thr Arg Ile Met Arg Thr Asn Thr Trp Phe Gln Cys
                455                 460                 465

Lys Glu Pro Lys Asn Ser Ser Phe Gly Phe Asp Ile Asn Lys Glu
                470                 475                 480

Lys Ala His Ser Gly Gly Ile Lys Ile Leu Tyr His Lys Ser Leu
                485                 490                 495

Ser Leu Phe Ile Phe Gly Ile Val His Leu Leu Lys Asn Thr Ser
                500                 505                 510

Ile Tyr Gln
```

<210> 193
<211> 702
<212> DNA
<213> Homo Sapien

<400> 193
cacaatcagg tcccattcta tagatgggga aactgaggct tgaggtcaca 50

taggcgtcgt tcaaggctgg tatacctgca ccctctccca tgtgaacaac 100

atggttctgg gtaatggggg ctgtcatcca gtctcctccc tgcccctgct 150

ggtgcacttc ctgcctctgc tggtgcactt tctgccccta ctggtatatt 200

tgctgcctct gctggggcgc ttcctgcctc ggctggtgta tctcctgccc 250

ctgctggtgc actttctgcc cccgctgatg cacttcctgc ctctgctggt 300

gcacttcctg gctctgctgg cacacttcct gcctctgctg gtgcacttcc 350

tggctctgct ggcgcacttt cctgcccctg ctggtgtatt tcctgcccct 400

gctggtgtac ttccttcccc tgctggtgca cttcctgcct ctgctggcgc 450

acttcttgcc tctccaggcc ctacctagcc tctccctctt atatatggaa 500

gtcttcccag ttcactgaca ctggtaacag ggactctgct cttggtgttg 550

ctgtctgccc tggggatggg catctgtgtc ttcctttact actgctggct 600

caggacccag agctttgaag catgtccaga tgcaggtccg ggcaccagag 650

tctaaggagc ccctacaccc accaggattt tccaataaag agatgttcac 700

ca 702

<210> 194
<211> 125
<212> PRT
<213> Homo Sapien

<400> 194
Met Val Leu Gly Asn Gly Gly Cys His Pro Val Ser Ser Leu Pro
1               5                   10                  15

Leu Leu Val His Phe Leu Pro Leu Leu Val His Phe Leu Pro Leu
                20                  25                  30

Leu Val Tyr Leu Leu Pro Leu Leu Gly Arg Phe Leu Pro Arg Leu
                35                  40                  45

Val Tyr Leu Leu Pro Leu Leu Val His Phe Leu Pro Pro Leu Met
                50                  55                  60

His Phe Leu Pro Leu Leu Val His Phe Leu Ala Leu Leu Ala His
                65                  70                  75

Phe Leu Pro Leu Leu Val His Phe Leu Ala Leu Leu Ala His Phe
                80                  85                  90

Pro Ala Pro Ala Gly Val Phe Pro Ala Pro Ala Gly Val Leu Pro
                95                  100                 105

Ser Pro Ala Gly Ala Leu Pro Ala Ser Ala Gly Ala Leu Leu Ala
110 115 120

Ser Pro Gly Pro Thr
125

<210> 195
<211> 2475
<212> DNA
<213> Homo Sapien

<400> 195
ggcaaggcgg cggcggcggc ggcggcagcc gcggtggcgg cgtggggaac 50

atctcggcag ccaccgcgct tctcccgctg gagcgggcgt ccagcttggc 100

tgccctcggt ccttccctgc cacgtttcgg gtcgccctgc accccccacc 150

caggctcgct tctcttcgaa gcgggaaggg cgccttgcag gatcctgccg 200

cccctccaac cggatcctgg gtctagagct ccccagagcg aggcgctcgc 250

caggactcct gccccgccaa ccctgaccgc cggggggtgc ccccgggacg 300

tagcgccgcg gagaggaagc ggcaaagggg accatgcggc gcctgactcg 350

tcggctggtt ctgccagtct tcggggtgct ctggatcacg gtgctgctgt 400

tcttctgggt aaccaagagg aagttggagg tgccgacggg acctgaagtg 450

cagaccccta agccttcgga cgctgactgg gacgacctgt gggaccagtt 500

tgatgagcgg cggtatctga atgccaaaaa gtggcgcgtt ggtgacgacc 550

cctataagct gtatgctttc aaccagcggg agagtgagcg gatctccagc 600

aatcgggcca tcccggacac tcgccatctg agatgcacac tgctggtgta 650

ttgcacggac cttccaccca ctagcatcat catcaccttc cacaacgagg 700

cccgctccac gctgctcagg accatccgca gtgtattaaa ccgcacccct 750

acgcatctga tccgggaaat catattagtg gatgacttca gcaatgaccc 800

tgatgactgt aaacagctca tcaagttgcc caaggtgaaa tgcttgcgca 850

ataatgaacg gcaaggtctg gtccggtccc ggattcgggg cgctgacatc 900

gcccagggca ccactctgac tttcctcgac agccactgtg aggtgaacag 950

ggactggctc cagcctctgt tgcacagggt caaagaggac tacacgcggg 1000

tggtgtgccc tgtgatcgat atcattaacc tggacacctt cacctacatc 1050

gagtctgcct cggagctcag agggggtttt gactggagcc tccacttcca 1100

gtgggagcag ctctccccag agcagaaggc tcggcgcctg gaccccacgg 1150

agcccatcag gactcctatc atagctggag ggctcttcgt gatcgacaaa 1200

gcttggtttg attacctggg gaaatatgat atggacatgg acatctgggg 1250

tggggagaac tttgaaatct ccttccgagt gtggatgtgc gggggcagcc 1300

tagagatcgt cccctgcagc cgagtggggc acgtcttccg gaagaagcac 1350

ccctacgttt tccctgatgg aaatgccaac acgtatataa agaacaccaa 1400

gcggacagct gaagtgtgga tggatgaata caagcaatac tattacgctg 1450

cccggccatt cgccctggag aggcccttcg ggaatgttga gagcagattg 1500

gacctgagga agaatctgcg ctgccagagc ttcaagtggt acctggagaa 1550

tatctaccct gaactcagca tccccaagga gtcctccatc cagaagggca 1600

atatccgaca gagacagaag tgcctggaat ctcaaaggca gaacaaccaa 1650

gaaaccccaa acctaaagtt gagcccctgt gccaaggtca aaggcgaaga 1700

tgcaaagtcc caggtatggg ccttcacata cacccagcag atcctccagg 1750

aggagctgtg cctgtcagtc atcaccttgt tccctggcgc ccagtggtt 1800

cttgtccttt gcaagaatgg agatgaccga cagcaatgga ccaaaactgg 1850

ttcccacatc gagcacatag catcccacct ctgcctcgat acagatatgt 1900

tcggtgatgg caccgagaac ggcaaggaaa tcgtcgtcaa cccatgtgag 1950

tcctcactca tgagccagca ctgggacatg gtgagctctt gaggacccct 2000

gccagaagca gcaagggcca tggggtggtg cttccctgga ccagaacaga 2050

ctggaaactg ggcagcaagc agcctgcaac cacctcagac atcctggact 2100

gggaggtgga ggcagagccc cccaggacag gagcaactgt ctcagggagg 2150

acagaggaaa acatcacaag ccaatgggct caaagacaaa tcccacatgt 2200

tctcaaggcc gttaagttcc agtcctggcc agtcattccc tgattggtat 2250

ctggagacag aaacctaatg ggaagtgttt attgttcctt ttcctacaaa 2300

ggaagcagtc tctggaggcc agaaagaaaa gccttctttt tcactaggcc 2350

aggactacat tgagagatga agaatggagg ttgtttccaa aagaaataaa 2400

gagaaactta gaagttgtct ctggaaaaaa aaaaaaaaaa aaaaaaaaaa 2450

aaaaaaaaaa aaaaaaaaaa aaaaa 2475

<210> 196
<211> 552
<212> PRT
<213> Homo Sapien

<400> 196

Met Arg Arg Leu Thr Arg Arg Leu Val Leu Pro Val Phe Gly Val
1 5 10 15

Leu Trp Ile Thr Val Leu Leu Phe Phe Trp Val Thr Lys Arg Lys
20 25 30

Leu Glu Val Pro Thr Gly Pro Glu Val Gln Thr Pro Lys Pro Ser
35 40 45

```
Asp Ala Asp Trp Asp Asp Leu Trp Asp Gln Phe Asp Glu Arg Arg
                50                  55                  60

Tyr Leu Asn Ala Lys Lys Trp Arg Val Gly Asp Asp Pro Tyr Lys
                65                  70                  75

Leu Tyr Ala Phe Asn Gln Arg Glu Ser Glu Arg Ile Ser Ser Asn
                80                  85                  90

Arg Ala Ile Pro Asp Thr Arg His Leu Arg Cys Thr Leu Leu Val
                95                  100                 105

Tyr Cys Thr Asp Leu Pro Pro Thr Ser Ile Ile Ile Thr Phe His
                110                 115                 120

Asn Glu Ala Arg Ser Thr Leu Leu Arg Thr Ile Arg Ser Val Leu
                125                 130                 135

Asn Arg Thr Pro Thr His Leu Ile Arg Glu Ile Ile Leu Val Asp
                140                 145                 150

Asp Phe Ser Asn Asp Pro Asp Asp Cys Lys Gln Leu Ile Lys Leu
                155                 160                 165

Pro Lys Val Lys Cys Leu Arg Asn Asn Glu Arg Gln Gly Leu Val
                170                 175                 180

Arg Ser Arg Ile Arg Gly Ala Asp Ile Ala Gln Gly Thr Thr Leu
                185                 190                 195

Thr Phe Leu Asp Ser His Cys Glu Val Asn Arg Asp Trp Leu Gln
                200                 205                 210

Pro Leu Leu His Arg Val Lys Glu Asp Tyr Thr Arg Val Val Cys
                215                 220                 225

Pro Val Ile Asp Ile Ile Asn Leu Asp Thr Phe Thr Tyr Ile Glu
                230                 235                 240

Ser Ala Ser Glu Leu Arg Gly Gly Phe Asp Trp Ser Leu His Phe
                245                 250                 255

Gln Trp Glu Gln Leu Ser Pro Glu Gln Lys Ala Arg Arg Leu Asp
                260                 265                 270

Pro Thr Glu Pro Ile Arg Thr Pro Ile Ile Ala Gly Gly Leu Phe
                275                 280                 285

Val Ile Asp Lys Ala Trp Phe Asp Tyr Leu Gly Lys Tyr Asp Met
                290                 295                 300

Asp Met Asp Ile Trp Gly Gly Glu Asn Phe Glu Ile Ser Phe Arg
                305                 310                 315

Val Trp Met Cys Gly Gly Ser Leu Glu Ile Val Pro Cys Ser Arg
                320                 325                 330

Val Gly His Val Phe Arg Lys Lys His Pro Tyr Val Phe Pro Asp
                335                 340                 345

Gly Asn Ala Asn Thr Tyr Ile Lys Asn Thr Lys Arg Thr Ala Glu
                350                 355                 360

Val Trp Met Asp Glu Tyr Lys Gln Tyr Tyr Tyr Ala Ala Arg Pro
```

```
                365                 370                 375
Phe Ala Leu Glu Arg Pro Phe Gly Asn Val Glu Ser Arg Leu Asp
                380                 385                 390
Leu Arg Lys Asn Leu Arg Cys Gln Ser Phe Lys Trp Tyr Leu Glu
                395                 400                 405
Asn Ile Tyr Pro Glu Leu Ser Ile Pro Lys Glu Ser Ser Ile Gln
                410                 415                 420
Lys Gly Asn Ile Arg Gln Arg Gln Lys Cys Leu Glu Ser Gln Arg
                425                 430                 435
Gln Asn Asn Gln Glu Thr Pro Asn Leu Lys Leu Ser Pro Cys Ala
                440                 445                 450
Lys Val Lys Gly Glu Asp Ala Lys Ser Gln Val Trp Ala Phe Thr
                455                 460                 465
Tyr Thr Gln Gln Ile Leu Gln Glu Glu Leu Cys Leu Ser Val Ile
                470                 475                 480
Thr Leu Phe Pro Gly Ala Pro Val Val Leu Val Leu Cys Lys Asn
                485                 490                 495
Gly Asp Asp Arg Gln Gln Trp Thr Lys Thr Gly Ser His Ile Glu
                500                 505                 510
His Ile Ala Ser His Leu Cys Leu Asp Thr Asp Met Phe Gly Asp
                515                 520                 525
Gly Thr Glu Asn Gly Lys Glu Ile Val Val Asn Pro Cys Glu Ser
                530                 535                 540
Ser Leu Met Ser Gln His Trp Asp Met Val Ser Ser
                545                 550
```

<210> 197
<211> 4060
<212> DNA
<213> Homo Sapien

<400> 197
gcagctcacc cttcgcagcc gcgatggggg aagacgacgc cgcgcttcgg 50

gctggcagca gggggctctc cgacccgtgg gcagactcag tgggagtgcg 100

accccgcacc acggagcgcc acatcgccgt acacaagcgg cttgtgctgg 150

ccttcgctgt gtccctcgtg gcattgctcg cggtcacaat gctcgctgtg 200

ctgctcagcc tgcgcttcga cgagtgcggg gcgagtgcca cgccaggcgc 250

cgacggtggc ccctcaggct ttccggagcg cggcggcaac gggagcctcc 300

ctggatcggc ccggcgcaac caccacgcag gcggggactc ctggcagccc 350

gaggcgggtg gggtggccag tccggggacc acgtcggccc agccgccgtc 400

ggaggaggag cgggagccgt gggagccgtg gacgcagctg cgcctgtcgg 450

gccacctgaa gccgctgcac tacaatctga tgctcaccgc cttcatggag 500

aacttcacct tctccgggga ggtcaacgtg gagatcgcgt gccggaacgc 550
cacccgctac gtagtgctgc acgcttcccg agtggcggtg gagaaagtgc 600
agctggccga ggaccgggcg ttcggggctg tccctgtagc cggtttttttc 650
ctctacccgc aaacccaggt cttagtggtg gtgctgaata ggacactgga 700
cgcgcagagg aattacaatc tgaagattat ctacaacgcg ctcatcgaga 750
atgagctcct gggcttcttc cgcagctcct atgtgctcca cggggagaga 800
agattccttg gtgttactca gttttcgcct acacatgcca gaaaggcatt 850
tccttgtttt gatgagccaa tctacaaggc tactttcaaa atcagcatca 900
agcatcaagc aacctattta tctttatcta atatgccagt ggaaacttcc 950
gtgtttgagg aagatggatg ggttacggat cacttttcac agacccctct 1000
catgtccaca tattatttag cctgggcaat ttgcaacttc acatacagag 1050
aaactaccac caagagtggg gttgtagtac gattatatgc aagacctgat 1100
gctatcagaa gaggatccgg ggactatgct ctccatataa caaagagatt 1150
aatagaattt tatgaagact actttaaagt gccctattcc ttgccaaaac 1200
tagatctttt agctgtgcct aagcatccgt atgctgctat ggagaactgg 1250
ggactaagta tttttgtgga acaaagaata ctgctggatc ccagtgtttc 1300
atctatttct tatttgctgg atgtcaccat ggtcattgtt catgagatat 1350
gtcaccagtg gtttggtgac cttgtgacgc ctgtgtggtg ggaagacgtg 1400
tggctgaagg aagggtttgc tcactacttt gaatttgttg gtacagacta 1450
cctctatcct ggctggaaca tggaaaagca gaggtttctg accgatgttc 1500
tgcatgaagt gatgctgctg gacggtttgg ccagttccca tccagtatca 1550
caggaagtgc tgcaggcaac agatattgac agggtgtttg actggatcgc 1600
atataaaaag ggtgctgctt taataagaat gctggctaat tttatgggcc 1650
attcagtttt ccagaggggt ttgcaagatt atttaaccat tcataagtat 1700
ggtaatgcag ccagaaatga tctctggaat acattatcgg aggctttaaa 1750
aagaaatggg aaatatgtaa atatacaaga agtaatggat cagtggacac 1800
tccagatggg ttatcctgtt atcaccatct tgggaaacac aacagcagaa 1850
aatagaataa taattaccca acagcatttt atctatgata tcagtgctaa 1900
aactaaagca cttaaacttc agaataacag ttacctgtgg cagattccat 1950
taactattgt ggtaggaaat agaagccatg tgtcttcaga agcaattatt 2000
tgggtgtcta acaaatcaga gcaccacaga ataacttatt tggacaaagg 2050
aagctggctg ctggggaaca tcaatcaaac tggctatttt agagtcaact 2100

atgacctaag gaactggaga ttattaattg atcaattaat ccggaatcat 2150
gaggttcttt ctgtcagtaa ccgagcgggc ttgatcgatg atgccttcag 2200
cctagccagg gctggctatt tgcctcagaa tattcctctg gagattatca 2250
gatacctgtc tgaggagaag gattttcttc cttggcatgc tgccagccga 2300
gctctttatc ctctagataa attactggac cgcatggaaa actacaacat 2350
tttcaatgaa tatattttaa agcaagttgc aacaacatat atcaagcttg 2400
ggtggccgaa aaataatttt aatggatctc ttgttcaagc atcctaccaa 2450
catgaagaac tacgtagaga agttataatg ctggcctgca gttttggcaa 2500
caagcactgt caccaacagg catcaacact tatttcagat tggatttcca 2550
gcaacaggaa cagaatacca ctaaatgtta gagacatcgt atactgtaca 2600
ggagtgtcac tactggatga ggatgtctgg gaattcatat ggatgaaatt 2650
ccattccacc acagcagttt ctgagaagaa aatattattg gaagccttaa 2700
cttgcagtga tgacaggaat ttattaaaca ggcttctaaa tctgtcactg 2750
aattctgagg tggtgctgga tcaagatgca attgatgtca taatccatgt 2800
agctcgaaat ccacatggtc gagaccttgc ctggaagttt ttcagggata 2850
aatggaagat attaaatacc aggtatggag aagcattgtt tatgtattcc 2900
aaactcatca gtggtgtcac agaatttctt aatactgaag gtgaactcaa 2950
agagctcaag aacttcatga aaaactatga tggggtagct gctgcttctt 3000
tctcacgagc tgtggaaact gtcgaagcca atgtgcgctg gaaaatgctt 3050
taccaagacg agcttttcca atggttagga aaagctctaa gacactaata 3100
tatgtatctt ataaacaaac aattcaactc agaagtttat gagaagacac 3150
gctttttgtg gaatgaggaa aatgtactac ctagaaaatg gccagatttt 3200
cagtgttaac gtgtgggagg aatttttttt tttagttttt attttttggt 3250
tttgggggat atttttattt tgtttcattc attctgttct gtttctctac 3300
tgggtgttcc tctctaaaga aactcttgca agtgaaacta gccatgattg 3350
cttcagctgt acattccttg ctgtacagga ccaaatatga tagtgatgca 3400
tgttgatgtt acagtcaatt tggaaaaaca tattcagaat atctgtgcat 3450
ggatatattg tcctgcctgt gttccagcat gcttatttca aacgtccagt 3500
gttgtgtgtg aatatgtgtt acacctagga tgggcattat gcaaaagcac 3550
aaagattata tatgacaatc agtattgcaa tgaaagaaaa actaaaaaca 3600
gaaatgatat tctcaatttt gggcaatgtg agaggtaaaa tagcccttga 3650
catgatgaac atcacttatt tcagcacttg gattgtctgg caatgattac 3700

tgtgttgcta actcattttc tttgagttaa agctgtgtat acattttaaa 3750

aggcatatag atagtgtatg catatgtata tgtacatagg gaagccccat 3800

atgtatatag tatgttgtac actgcacatg tacaaagaat gtcttcagat 3850

caaagaaaat ttatctcttt ttataaactt aaggacagtt gcaaaaggct 3900

tcaaggaatt tatctcaaca ttattctttc tatgtcctaa ctaaatttct 3950

caactgttat gaatttttca tctacttctt gaacagtggt ctattctgct 4000

acatgaagat gaatacaaac aaaatttttg tataaactcc caaaaaaaaa 4050

aaaaaaaaaa 4060

<210> 198
<211> 1024
<212> PRT
<213> Homo Sapien

<400> 198
Met Gly Glu Asp Asp Ala Ala Leu Arg Ala Gly Ser Arg Gly Leu
1 5 10 15

Ser Asp Pro Trp Ala Asp Ser Val Gly Val Arg Pro Arg Thr Thr
20 25 30

Glu Arg His Ile Ala Val His Lys Arg Leu Val Leu Ala Phe Ala
35 40 45

Val Ser Leu Val Ala Leu Leu Ala Val Thr Met Leu Ala Val Leu
50 55 60

Leu Ser Leu Arg Phe Asp Glu Cys Gly Ala Ser Ala Thr Pro Gly
65 70 75

Ala Asp Gly Gly Pro Ser Gly Phe Pro Glu Arg Gly Gly Asn Gly
80 85 90

Ser Leu Pro Gly Ser Ala Arg Arg Asn His His Ala Gly Gly Asp
95 100 105

Ser Trp Gln Pro Glu Ala Gly Gly Val Ala Ser Pro Gly Thr Thr
110 115 120

Ser Ala Gln Pro Pro Ser Glu Glu Glu Arg Glu Pro Trp Glu Pro
125 130 135

Trp Thr Gln Leu Arg Leu Ser Gly His Leu Lys Pro Leu His Tyr
140 145 150

Asn Leu Met Leu Thr Ala Phe Met Glu Asn Phe Thr Phe Ser Gly
155 160 165

Glu Val Asn Val Glu Ile Ala Cys Arg Asn Ala Thr Arg Tyr Val
170 175 180

Val Leu His Ala Ser Arg Val Ala Val Glu Lys Val Gln Leu Ala
185 190 195

Glu Asp Arg Ala Phe Gly Ala Val Pro Val Ala Gly Phe Phe Leu
200 205 210

```
Tyr Pro Gln Thr Gln Val Leu Val Val Val Leu Asn Arg Thr Leu
                215                 220                 225

Asp Ala Gln Arg Asn Tyr Asn Leu Lys Ile Ile Tyr Asn Ala Leu
                230                 235                 240

Ile Glu Asn Glu Leu Leu Gly Phe Phe Arg Ser Ser Tyr Val Leu
                245                 250                 255

His Gly Glu Arg Arg Phe Leu Gly Val Thr Gln Phe Ser Pro Thr
                260                 265                 270

His Ala Arg Lys Ala Phe Pro Cys Phe Asp Glu Pro Ile Tyr Lys
                275                 280                 285

Ala Thr Phe Lys Ile Ser Ile Lys His Gln Ala Thr Tyr Leu Ser
                290                 295                 300

Leu Ser Asn Met Pro Val Glu Thr Ser Val Phe Glu Glu Asp Gly
                305                 310                 315

Trp Val Thr Asp His Phe Ser Gln Thr Pro Leu Met Ser Thr Tyr
                320                 325                 330

Tyr Leu Ala Trp Ala Ile Cys Asn Phe Thr Tyr Arg Glu Thr Thr
                335                 340                 345

Thr Lys Ser Gly Val Val Val Arg Leu Tyr Ala Arg Pro Asp Ala
                350                 355                 360

Ile Arg Arg Gly Ser Gly Asp Tyr Ala Leu His Ile Thr Lys Arg
                365                 370                 375

Leu Ile Glu Phe Tyr Glu Asp Tyr Phe Lys Val Pro Tyr Ser Leu
                380                 385                 390

Pro Lys Leu Asp Leu Leu Ala Val Pro Lys His Pro Tyr Ala Ala
                395                 400                 405

Met Glu Asn Trp Gly Leu Ser Ile Phe Val Glu Gln Arg Ile Leu
                410                 415                 420

Leu Asp Pro Ser Val Ser Ser Ile Ser Tyr Leu Leu Asp Val Thr
                425                 430                 435

Met Val Ile Val His Glu Ile Cys His Gln Trp Phe Gly Asp Leu
                440                 445                 450

Val Thr Pro Val Trp Trp Glu Asp Val Trp Leu Lys Glu Gly Phe
                455                 460                 465

Ala His Tyr Phe Glu Phe Val Gly Thr Asp Tyr Leu Tyr Pro Gly
                470                 475                 480

Trp Asn Met Glu Lys Gln Arg Phe Leu Thr Asp Val Leu His Glu
                485                 490                 495

Val Met Leu Leu Asp Gly Leu Ala Ser Ser His Pro Val Ser Gln
                500                 505                 510

Glu Val Leu Gln Ala Thr Asp Ile Asp Arg Val Phe Asp Trp Ile
                515                 520                 525

Ala Tyr Lys Lys Gly Ala Ala Leu Ile Arg Met Leu Ala Asn Phe
```

530 535 540

Met Gly His Ser Val Phe Gln Arg Gly Leu Gln Asp Tyr Leu Thr
545 550 555

Ile His Lys Tyr Gly Asn Ala Ala Arg Asn Asp Leu Trp Asn Thr
560 565 570

Leu Ser Glu Ala Leu Lys Arg Asn Gly Lys Tyr Val Asn Ile Gln
575 580 585

Glu Val Met Asp Gln Trp Thr Leu Gln Met Gly Tyr Pro Val Ile
590 595 600

Thr Ile Leu Gly Asn Thr Thr Ala Glu Asn Arg Ile Ile Ile Thr
605 610 615

Gln Gln His Phe Ile Tyr Asp Ile Ser Ala Lys Thr Lys Ala Leu
620 625 630

Lys Leu Gln Asn Asn Ser Tyr Leu Trp Gln Ile Pro Leu Thr Ile
635 640 645

Val Val Gly Asn Arg Ser His Val Ser Ser Glu Ala Ile Ile Trp
650 655 660

Val Ser Asn Lys Ser Glu His His Arg Ile Thr Tyr Leu Asp Lys
665 670 675

Gly Ser Trp Leu Leu Gly Asn Ile Asn Gln Thr Gly Tyr Phe Arg
680 685 690

Val Asn Tyr Asp Leu Arg Asn Trp Arg Leu Leu Ile Asp Gln Leu
695 700 705

Ile Arg Asn His Glu Val Leu Ser Val Ser Asn Arg Ala Gly Leu
710 715 720

Ile Asp Asp Ala Phe Ser Leu Ala Arg Ala Gly Tyr Leu Pro Gln
725 730 735

Asn Ile Pro Leu Glu Ile Ile Arg Tyr Leu Ser Glu Glu Lys Asp
740 745 750

Phe Leu Pro Trp His Ala Ala Ser Arg Ala Leu Tyr Pro Leu Asp
755 760 765

Lys Leu Leu Asp Arg Met Glu Asn Tyr Asn Ile Phe Asn Glu Tyr
770 775 780

Ile Leu Lys Gln Val Ala Thr Thr Tyr Ile Lys Leu Gly Trp Pro
785 790 795

Lys Asn Asn Phe Asn Gly Ser Leu Val Gln Ala Ser Tyr Gln His
800 805 810

Glu Glu Leu Arg Arg Glu Val Ile Met Leu Ala Cys Ser Phe Gly
815 820 825

Asn Lys His Cys His Gln Gln Ala Ser Thr Leu Ile Ser Asp Trp
830 835 840

Ile Ser Ser Asn Arg Asn Arg Ile Pro Leu Asn Val Arg Asp Ile
845 850 855

```
Val Tyr Cys Thr Gly Val Ser Leu Leu Asp Glu Asp Val Trp Glu
                860                 865                 870

Phe Ile Trp Met Lys Phe His Ser Thr Thr Ala Val Ser Glu Lys
                875                 880                 885

Lys Ile Leu Leu Glu Ala Leu Thr Cys Ser Asp Asp Arg Asn Leu
                890                 895                 900

Leu Asn Arg Leu Leu Asn Leu Ser Leu Asn Ser Glu Val Val Leu
                905                 910                 915

Asp Gln Asp Ala Ile Asp Val Ile Ile His Val Ala Arg Asn Pro
                920                 925                 930

His Gly Arg Asp Leu Ala Trp Lys Phe Phe Arg Asp Lys Trp Lys
                935                 940                 945

Ile Leu Asn Thr Arg Tyr Gly Glu Ala Leu Phe Met Tyr Ser Lys
                950                 955                 960

Leu Ile Ser Gly Val Thr Glu Phe Leu Asn Thr Glu Gly Glu Leu
                965                 970                 975

Lys Glu Leu Lys Asn Phe Met Lys Asn Tyr Asp Gly Val Ala Ala
                980                 985                 990

Ala Ser Phe Ser Arg Ala Val Glu Thr Val Glu Ala Asn Val Arg
                995                 1000                1005

Trp Lys Met Leu Tyr Gln Asp Glu Leu Phe Gln Trp Leu Gly Lys
                1010                1015                1020

Ala Leu Arg His
```

<210> 199
<211> 3461
<212> DNA
<213> Homo Sapien

<400> 199

```
gcgcccggcg cagctcggcc agagcgaccg cggggctgag cgcgcgtccg 50

cccagggggc tccggaagct gccccggccc gcggcctcct ccctcgctcc 100

cgcttcccct ttctcgctca ccgccgccct ccttccccag ctccctcgcc 150

gtccgcccgc cccacagcca gcggctccgc gccccctgca gccacgatgc 200

ccgcggcccg gccgcccgcc gcgggactcc gcgggatctc gctgttcctc 250

gctctgctcc tggggagccc ggcggcagcg ctggagcgag atgctcttcc 300

cgagggagat gctagccctt tgggtcctta cctcctgccc tcaggagccc 350

cggagagagg cagtcctggc aaagagcacc ctgaagagag agtggtaaca 400

gcgccccccca gttcctcaca gtcggcggaa gtgctgggcg agctggtgct 450

ggatgggacc gcaccctctg cacatcacga catcccagcc ctgtcaccgc 500

tgcttccaga ggaggcccgc cccaagcacg ccttgccccc caagaagaaa 550
```

ctgccttcgc tcaagcaggt gaactctgcc aggaagcagc tgaggcccaa 600
ggccacctcc gcagccactg tccaaagggc agggtcccag ccagcgtccc 650
agggcctaga tctcctctcc tcctccacgg agaagcctgg cccaccgggg 700
gacccggacc ccatcgtggc ctccgaggag gcatcagaag tgcccctttg 750
gctggatcga aaggagagtg cggtccctac aacacccgca cccctgcaaa 800
tctccccctt cacttcgcag ccctatgtgg cccacacact cccccagagg 850
ccagaacccg gggagcctgg gcctgacatg gcccaggagg ccccccagga 900
ggacaccagc cccatggccc tgatggacaa aggtgagaat gagctgactg 950
ggtcagcctc agaggagagc caggagacca ctacctccac cattatcacc 1000
accacggtca tcaccaccga gcaagcacca gctctctgca gtgtgagctt 1050
ctccaatcct gaggggtaca ttgactccag cgactaccca ctgctgcccc 1100
tcaacaactt tctggagtgc acatacaacg tgacagtcta cactggctat 1150
ggggtggagc tccaggtgaa gagtgtgaac ctgtccgatg gggaactgct 1200
ctccatccgc ggggtggacg gccctaccct gaccgtcctg gccaaccaga 1250
cactcctggt ggaggggcag gtaatccgaa gccccaccaa caccatctcc 1300
gtctacttcc ggaccttcca ggacgacggc cttgggacct tccagcttca 1350
ctaccaggcc ttcatgctga gctgcaactt tccccgccgg cctgactctg 1400
gggatgtcac ggtgatggac ctgcactcag gtggggtggc ccactttcac 1450
tgccacctgg gctatgagct ccagggcgct aagatgctga catgcatcaa 1500
tgcctccaag ccgcactgga gcagccagga gcccatctgc tcagctcctt 1550
gtggaggggc agtgcacaat gccaccatcg gccgcgtcct ctccccaagt 1600
taccctgaaa acacaaatgg gagccaattc tgcatctgga cgattgaagc 1650
tccagagggc cagaagctgc acctgcactt tgagaggctg ttgctgcatg 1700
acaaggacag gatgacggtt cacagcgggc agaccaacaa gtcagctctt 1750
ctctacgact cccttcaaac cgagagtgtc ccttttgagg gcctgctgag 1800
cgaaggcaac accatccgca tcgagttcac gtccgaccag gcccgggcgg 1850
cctccacctt caacatccga tttgaagcgt ttgagaaagg ccactgctat 1900
gagccctaca tccagaatgg gaacttcact acatccgacc cgacctataa 1950
cattgggact atagtggagt tcacctgcga ccccggccac tccctggagc 2000
agggcccggc catcatcgaa tgcatcaatg tgcgggaccc atactggaat 2050
gacacagagc ccctgtgcag agccatgtgt ggtggggagc tctctgctgt 2100
ggctggggtg gtattgtccc caaactggcc cgagccctac gtggaaggtg 2150

aagattgtat ctggaagatc cacgtgggag aagagaaacg gatcttctta 2200

gatatccagt tcctgaatct gagcaacagt gacatcttga ccatctacga 2250

tggcgacgag gtcatgcccc acatcttggg gcagtacctt gggaacagtg 2300

gcccccagaa actgtactcc tccacgccag acttaaccat ccagttccat 2350

tcggaccctg ctggcctcat ctttggaaag ggccagggat ttatcatgaa 2400

ctacatagag gtatcaagga atgactcctg ctcggattta cccgagatcc 2450

agaatggctg gaaaaccact tctcacacgg agttggtgcg gggagccaga 2500

atcacctacc agtgtgaccc cggctatgac atcgtgggga gtgacaccct 2550

cacctgccag tgggacctca gctggagcag cgacccccca ttttgtgaga 2600

aaattatgta ctgcaccgac cccggagagg tggatcactc gacccgctta 2650

atttcggatc ctgtgctgct ggtggggacc accatccaat acacctgcaa 2700

ccccggtttt gtgcttgaag ggagttctct tctgacctgc tacagccgtg 2750

aaacagggac tcccatctgg acgtctcgcc tgccccactg cgtttcggag 2800

gagtccctgg catgtgacaa cccagggctg cctgaaaatg gataccaaat 2850

cctgtacaag cgactctacc tgccaggaga gtccctcacc ttcatgtgct 2900

acgaaggctt tgagctcatg ggtgaagtga ccatccgctg catcctggga 2950

cagccatccc actggaacgg gcccctgccc gtgtgtaaag ttaatcaaga 3000

cagttttgaa catgctttag aagcagaagc ggcagcagag acgtcgctgg 3050

aaggggggaa catggccctg gctatcttca tcccggtcct catcatctcc 3100

ttactgctgg gaggagccta catttacatc acaagatgtc gctactattc 3150

caacctccgc ctgcctctga tgtactccca cccctacagc cagatcaccg 3200

tggaaaccga gtttgacaac cccatttacg agacagggga aaccagagag 3250

tatgaggttt ctatctaaag agagctacac ttgagaaggg gacttgtgaa 3300

ctcaaccaca atctcctcga gacattcatc cagagaccat gtggcacttg 3350

attgaaaccc cagaatgtcg actgtctttt gtttagactc tttatcaaag 3400

gtttactgtt ttcttccctg tatttattat atttaaaagt gaaaaaaaaa 3450

aaaaaaaaaa a 3461

<210> 200
<211> 1023
<212> PRT
<213> Homo Sapien

<400> 200

Met Pro Ala Ala Arg Pro Pro Ala Ala Gly Leu Arg Gly Ile Ser
1 5 10 15

```
Leu Phe Leu Ala Leu Leu Leu Gly Ser Pro Ala Ala Ala Leu Glu
                20                  25                  30

Arg Asp Ala Leu Pro Glu Gly Asp Ala Ser Pro Leu Gly Pro Tyr
                35                  40                  45

Leu Leu Pro Ser Gly Ala Pro Glu Arg Gly Ser Pro Gly Lys Glu
                50                  55                  60

His Pro Glu Glu Arg Val Val Thr Ala Pro Pro Ser Ser Ser Gln
                65                  70                  75

Ser Ala Glu Val Leu Gly Glu Leu Val Leu Asp Gly Thr Ala Pro
                80                  85                  90

Ser Ala His His Asp Ile Pro Ala Leu Ser Pro Leu Leu Pro Glu
                95                 100                 105

Glu Ala Arg Pro Lys His Ala Leu Pro Pro Lys Lys Lys Leu Pro
               110                 115                 120

Ser Leu Lys Gln Val Asn Ser Ala Arg Lys Gln Leu Arg Pro Lys
               125                 130                 135

Ala Thr Ser Ala Ala Thr Val Gln Arg Ala Gly Ser Gln Pro Ala
               140                 145                 150

Ser Gln Gly Leu Asp Leu Leu Ser Ser Ser Thr Glu Lys Pro Gly
               155                 160                 165

Pro Pro Gly Asp Pro Asp Pro Ile Val Ala Ser Glu Glu Ala Ser
               170                 175                 180

Glu Val Pro Leu Trp Leu Asp Arg Lys Glu Ser Ala Val Pro Thr
               185                 190                 195

Thr Pro Ala Pro Leu Gln Ile Ser Pro Phe Thr Ser Gln Pro Tyr
               200                 205                 210

Val Ala His Thr Leu Pro Gln Arg Pro Glu Pro Gly Glu Pro Gly
               215                 220                 225

Pro Asp Met Ala Gln Glu Ala Pro Gln Glu Asp Thr Ser Pro Met
               230                 235                 240

Ala Leu Met Asp Lys Gly Glu Asn Glu Leu Thr Gly Ser Ala Ser
               245                 250                 255

Glu Glu Ser Gln Glu Thr Thr Thr Ser Thr Ile Ile Thr Thr Thr
               260                 265                 270

Val Ile Thr Thr Glu Gln Ala Pro Ala Leu Cys Ser Val Ser Phe
               275                 280                 285

Ser Asn Pro Glu Gly Tyr Ile Asp Ser Ser Asp Tyr Pro Leu Leu
               290                 295                 300

Pro Leu Asn Asn Phe Leu Glu Cys Thr Tyr Asn Val Thr Val Tyr
               305                 310                 315

Thr Gly Tyr Gly Val Glu Leu Gln Val Lys Ser Val Asn Leu Ser
               320                 325                 330

Asp Gly Glu Leu Leu Ser Ile Arg Gly Val Asp Gly Pro Thr Leu
```

```
                335                 340                 345

Thr Val Leu Ala Asn Gln Thr Leu Leu Val Glu Gly Gln Val Ile
                350                 355                 360

Arg Ser Pro Thr Asn Thr Ile Ser Val Tyr Phe Arg Thr Phe Gln
                365                 370                 375

Asp Asp Gly Leu Gly Thr Phe Gln Leu His Tyr Gln Ala Phe Met
                380                 385                 390

Leu Ser Cys Asn Phe Pro Arg Arg Pro Asp Ser Gly Asp Val Thr
                395                 400                 405

Val Met Asp Leu His Ser Gly Gly Val Ala His Phe His Cys His
                410                 415                 420

Leu Gly Tyr Glu Leu Gln Gly Ala Lys Met Leu Thr Cys Ile Asn
                425                 430                 435

Ala Ser Lys Pro His Trp Ser Ser Gln Glu Pro Ile Cys Ser Ala
                440                 445                 450

Pro Cys Gly Gly Ala Val His Asn Ala Thr Ile Gly Arg Val Leu
                455                 460                 465

Ser Pro Ser Tyr Pro Glu Asn Thr Asn Gly Ser Gln Phe Cys Ile
                470                 475                 480

Trp Thr Ile Glu Ala Pro Glu Gly Gln Lys Leu His Leu His Phe
                485                 490                 495

Glu Arg Leu Leu Leu His Asp Lys Asp Arg Met Thr Val His Ser
                500                 505                 510

Gly Gln Thr Asn Lys Ser Ala Leu Leu Tyr Asp Ser Leu Gln Thr
                515                 520                 525

Glu Ser Val Pro Phe Glu Gly Leu Leu Ser Glu Gly Asn Thr Ile
                530                 535                 540

Arg Ile Glu Phe Thr Ser Asp Gln Ala Arg Ala Ala Ser Thr Phe
                545                 550                 555

Asn Ile Arg Phe Glu Ala Phe Glu Lys Gly His Cys Tyr Glu Pro
                560                 565                 570

Tyr Ile Gln Asn Gly Asn Phe Thr Thr Ser Asp Pro Thr Tyr Asn
                575                 580                 585

Ile Gly Thr Ile Val Glu Phe Thr Cys Asp Pro Gly His Ser Leu
                590                 595                 600

Glu Gln Gly Pro Ala Ile Ile Glu Cys Ile Asn Val Arg Asp Pro
                605                 610                 615

Tyr Trp Asn Asp Thr Glu Pro Leu Cys Arg Ala Met Cys Gly Gly
                620                 625                 630

Glu Leu Ser Ala Val Ala Gly Val Val Leu Ser Pro Asn Trp Pro
                635                 640                 645

Glu Pro Tyr Val Glu Gly Glu Asp Cys Ile Trp Lys Ile His Val
                650                 655                 660
```

Gly Glu Glu Lys Arg Ile Phe Leu Asp Ile Gln Phe Leu Asn Leu
665 670 675

Ser Asn Ser Asp Ile Leu Thr Ile Tyr Asp Gly Asp Glu Val Met
680 685 690

Pro His Ile Leu Gly Gln Tyr Leu Gly Asn Ser Gly Pro Gln Lys
695 700 705

Leu Tyr Ser Ser Thr Pro Asp Leu Thr Ile Gln Phe His Ser Asp
710 715 720

Pro Ala Gly Leu Ile Phe Gly Lys Gly Gln Gly Phe Ile Met Asn
725 730 735

Tyr Ile Glu Val Ser Arg Asn Asp Ser Cys Ser Asp Leu Pro Glu
740 745 750

Ile Gln Asn Gly Trp Lys Thr Thr Ser His Thr Glu Leu Val Arg
755 760 765

Gly Ala Arg Ile Thr Tyr Gln Cys Asp Pro Gly Tyr Asp Ile Val
770 775 780

Gly Ser Asp Thr Leu Thr Cys Gln Trp Asp Leu Ser Trp Ser Ser
785 790 795

Asp Pro Pro Phe Cys Glu Lys Ile Met Tyr Cys Thr Asp Pro Gly
800 805 810

Glu Val Asp His Ser Thr Arg Leu Ile Ser Asp Pro Val Leu Leu
815 820 825

Val Gly Thr Thr Ile Gln Tyr Thr Cys Asn Pro Gly Phe Val Leu
830 835 840

Glu Gly Ser Ser Leu Leu Thr Cys Tyr Ser Arg Glu Thr Gly Thr
845 850 855

Pro Ile Trp Thr Ser Arg Leu Pro His Cys Val Ser Glu Glu Ser
860 865 870

Leu Ala Cys Asp Asn Pro Gly Leu Pro Glu Asn Gly Tyr Gln Ile
875 880 885

Leu Tyr Lys Arg Leu Tyr Leu Pro Gly Glu Ser Leu Thr Phe Met
890 895 900

Cys Tyr Glu Gly Phe Glu Leu Met Gly Glu Val Thr Ile Arg Cys
905 910 915

Ile Leu Gly Gln Pro Ser His Trp Asn Gly Pro Leu Pro Val Cys
920 925 930

Lys Val Asn Gln Asp Ser Phe Glu His Ala Leu Glu Ala Glu Ala
935 940 945

Ala Ala Glu Thr Ser Leu Glu Gly Gly Asn Met Ala Leu Ala Ile
950 955 960

Phe Ile Pro Val Leu Ile Ile Ser Leu Leu Leu Gly Gly Ala Tyr
965 970 975

```
Ile Tyr Ile Thr Arg Cys Arg Tyr Tyr Ser Asn Leu Arg Leu Pro
                980                 985                 990

Leu Met Tyr Ser His Pro Tyr Ser Gln Ile Thr Val Glu Thr Glu
                995                 1000                1005

Phe Asp Asn Pro Ile Tyr Glu Thr Gly Glu Thr Arg Glu Tyr Glu
               1010                1015                1020

Val Ser Ile


<210> 201
<211> 2806
<212> DNA
<213> Homo Sapien

<220>
<221> unsure
<222> 2157
<223> unknown base

<400> 201
gatggctacg gcagggggtg gctctggggc tgacccggga agtcggggtc 50

tccttcgcct tctgtctttc tgcgtcctac tagcaggttt gtgcagggga 100

aactcagtgg agaggaagat atatatcccc ttaaataaaa cagctccctg 150

tgttcgcctg ctcaacgcca ctcatcagat tggctgccag tcttcaatta 200

gtggagacac aggggttatc cacgtagtag agaaagagga ggacctacag 250

tgggtattga ctgatggccc caacccccct tacatggttc tgctggagag 300

caagcatttt accagggatt taatggagaa gctgaaaggg agaaccagcc 350

gaattgctgg tcttgcagtg tccttgacca agcccagtcc tgcctcaggc 400

ttctctccta gtgtacagtg cccaaatgat gggtttggtg tttactccaa 450

ttcctatggg ccagagtttg ctcactgcag agaaatacag tggaattcgc 500

tgggcaatgg tttggcttat gaagacttta gtttccccat ctttcttctt 550

gaagatgaaa atgaaaccaa agtcatcaag cagtgctatc aagatcacaa 600

cctgagtcag aatggctcag caccaacctt cccactatgt gccatgcagc 650

tcttttcaca catgcatgct gtcatcagca ctgccacctg catgcggcgc 700

agctccatcc aaagcacctt cagcatcaac ccagaaatcg tctgtgaccc 750

cctgtctgat tacaatgtgt ggagcatgct aaagcctata aatacaactg 800

ggacattaaa gcctgacgac agggttgtgg ttgctgccac ccggctggat 850

agtcgttcct ttttctggaa tgtggcccca ggggctgaaa gcgcagtggc 900

ttcctttgtc acccagctgg ctgctgctga agctttgcaa aaggcacctg 950

atgtgaccac cctgccccgc aatgtcatgt ttgtcttctt tcaaggggaa 1000

acttttgact acattggcag ctcgaggatg gtctacgata tggagaaggg 1050
```

caagtttccc gtgcagttag agaatgttga ctcatttgtg gagctgggac 1100
aggtggcctt aagaacttca ttagagcttt ggatgcacac agatcctgtt 1150
tctcagaaaa atgagtctgt acggaaccag gtggaggatc tcctggccac 1200
attggagaag agtggtgctg gtgtccctgc tgtcatcctc aggaggccaa 1250
atcagtccca gcctctccca ccatcttccc tgcagcgatt tcttcgagct 1300
cgaaacatct ctggcgttgt tctggctgac cactctggtg ccttccataa 1350
caaatattac cagagtattt acgacactgc tgagaacatt aatgtgagct 1400
atcccgaatg gctgagccct gaagaggacc tgaactttgt aacagacact 1450
gccaaggccc tggcagatgt ggccacggtg ctgggacgtg ctctgtatga 1500
gcttgcagga ggaaccaact tcagcgacac agttcaggct gatccccaaa 1550
cggttacccg cctgctctat gggttcctga ttaaagccaa caactcatgg 1600
ttccagtcta tcctcaggca ggacctaagg tcctacttgg gtgacgggcc 1650
tcttcaacat tacatcgctg tctccagccc caccaacacc acttatgttg 1700
tacagtatgc cttggcaaat ttgactggca cagtggtcaa cctcacccga 1750
gagcagtgcc aggatccaag taaagtccca agtgaaaaca aggatctgta 1800
tgagtactca tgggtccagg gccctttgca ttctaatgag acggaccgac 1850
tcccccggtg tgtgcgttct actgcacgat tagccagggc cttgtctcct 1900
gcctttgaac tgagtcagtg gagctctact gaatactcta catggactga 1950
gagccgctgg aaagatatcc gtgcccggat atttctcatc gccagcaaag 2000
agcttgagtt gatcaccctg acagtgggct tcggcatcct catcttctcc 2050
ctcatcgtca cctactgcat caatgccaaa gctgatgtcc ttttcattgc 2100
tccccgggag ccaggagctg tgtcatactg aggaggaccc cagcttttct 2150
tgccagntca gcagttcact tcctagagca tctgtcccac tgggacacaa 2200
ccactaattt gtcactggaa cctccctggg cctgtctcag attgggatta 2250
acataaaaga gtggaactat ccaaaagaga cagggagaaa taaataaatt 2300
gcctcccttc ctccgctccc ctttcccatc accccttccc catttcctct 2350
tccttctcta ctcatgccag attttgggat tacaaataga agcttcttgc 2400
tcctgtttaa ctccctagtt acccacccta atttgccctt caggaccctt 2450
ctactttttc cttcctgccc tgtacctctc tctgctcctc acccccaccc 2500
ctgtacccag ccaccttcct gactgggaag gacataaaag gtttaatgtc 2550
agggtcaaac tacattgagc ccctgaggac aggggcatct ctgggctgag 2600
cctactgtct ccttcccact gtcctttctc caggccctca gatggcacat 2650

tagggtgggc gtgctgcggg tgggtatccc acctccagcc cacagtgctc 2700

agttgtactt tttattaagc tgtaatatct atttttgttt ttgtcttttt 2750

cctttattct ttttgtaaat atatatataa tgagtttcat taaaatagat 2800

tatccc 2806

<210> 202
<211> 709
<212> PRT
<213> Homo Sapien

<400> 202
Met Ala Thr Ala Gly Gly Gly Ser Gly Ala Asp Pro Gly Ser Arg
1 5 10 15

Gly Leu Leu Arg Leu Leu Ser Phe Cys Val Leu Leu Ala Gly Leu
20 25 30

Cys Arg Gly Asn Ser Val Glu Arg Lys Ile Tyr Ile Pro Leu Asn
35 40 45

Lys Thr Ala Pro Cys Val Arg Leu Leu Asn Ala Thr His Gln Ile
50 55 60

Gly Cys Gln Ser Ser Ile Ser Gly Asp Thr Gly Val Ile His Val
65 70 75

Val Glu Lys Glu Glu Asp Leu Gln Trp Val Leu Thr Asp Gly Pro
80 85 90

Asn Pro Pro Tyr Met Val Leu Leu Glu Ser Lys His Phe Thr Arg
95 100 105

Asp Leu Met Glu Lys Leu Lys Gly Arg Thr Ser Arg Ile Ala Gly
110 115 120

Leu Ala Val Ser Leu Thr Lys Pro Ser Pro Ala Ser Gly Phe Ser
125 130 135

Pro Ser Val Gln Cys Pro Asn Asp Gly Phe Gly Val Tyr Ser Asn
140 145 150

Ser Tyr Gly Pro Glu Phe Ala His Cys Arg Glu Ile Gln Trp Asn
155 160 165

Ser Leu Gly Asn Gly Leu Ala Tyr Glu Asp Phe Ser Phe Pro Ile
170 175 180

Phe Leu Leu Glu Asp Glu Asn Glu Thr Lys Val Ile Lys Gln Cys
185 190 195

Tyr Gln Asp His Asn Leu Ser Gln Asn Gly Ser Ala Pro Thr Phe
200 205 210

Pro Leu Cys Ala Met Gln Leu Phe Ser His Met His Ala Val Ile
215 220 225

Ser Thr Ala Thr Cys Met Arg Arg Ser Ser Ile Gln Ser Thr Phe
230 235 240

Ser Ile Asn Pro Glu Ile Val Cys Asp Pro Leu Ser Asp Tyr Asn

245 250 255

Val Trp Ser Met Leu Lys Pro Ile Asn Thr Thr Gly Thr Leu Lys
260 265 270

Pro Asp Asp Arg Val Val Val Ala Ala Thr Arg Leu Asp Ser Arg
275 280 285

Ser Phe Phe Trp Asn Val Ala Pro Gly Ala Glu Ser Ala Val Ala
290 295 300

Ser Phe Val Thr Gln Leu Ala Ala Ala Glu Ala Leu Gln Lys Ala
305 310 315

Pro Asp Val Thr Thr Leu Pro Arg Asn Val Met Phe Val Phe Phe
320 325 330

Gln Gly Glu Thr Phe Asp Tyr Ile Gly Ser Ser Arg Met Val Tyr
335 340 345

Asp Met Glu Lys Gly Lys Phe Pro Val Gln Leu Glu Asn Val Asp
350 355 360

Ser Phe Val Glu Leu Gly Gln Val Ala Leu Arg Thr Ser Leu Glu
365 370 375

Leu Trp Met His Thr Asp Pro Val Ser Gln Lys Asn Glu Ser Val
380 385 390

Arg Asn Gln Val Glu Asp Leu Leu Ala Thr Leu Glu Lys Ser Gly
395 400 405

Ala Gly Val Pro Ala Val Ile Leu Arg Arg Pro Asn Gln Ser Gln
410 415 420

Pro Leu Pro Pro Ser Ser Leu Gln Arg Phe Leu Arg Ala Arg Asn
425 430 435

Ile Ser Gly Val Val Leu Ala Asp His Ser Gly Ala Phe His Asn
440 445 450

Lys Tyr Tyr Gln Ser Ile Tyr Asp Thr Ala Glu Asn Ile Asn Val
455 460 465

Ser Tyr Pro Glu Trp Leu Ser Pro Glu Glu Asp Leu Asn Phe Val
470 475 480

Thr Asp Thr Ala Lys Ala Leu Ala Asp Val Ala Thr Val Leu Gly
485 490 495

Arg Ala Leu Tyr Glu Leu Ala Gly Gly Thr Asn Phe Ser Asp Thr
500 505 510

Val Gln Ala Asp Pro Gln Thr Val Thr Arg Leu Leu Tyr Gly Phe
515 520 525

Leu Ile Lys Ala Asn Asn Ser Trp Phe Gln Ser Ile Leu Arg Gln
530 535 540

Asp Leu Arg Ser Tyr Leu Gly Asp Gly Pro Leu Gln His Tyr Ile
545 550 555

Ala Val Ser Ser Pro Thr Asn Thr Thr Tyr Val Val Gln Tyr Ala
560 565 570

Leu Ala Asn Leu Thr Gly Thr Val Val Asn Leu Thr Arg Glu Gln
575 580 585

Cys Gln Asp Pro Ser Lys Val Pro Ser Glu Asn Lys Asp Leu Tyr
590 595 600

Glu Tyr Ser Trp Val Gln Gly Pro Leu His Ser Asn Glu Thr Asp
605 610 615

Arg Leu Pro Arg Cys Val Arg Ser Thr Ala Arg Leu Ala Arg Ala
620 625 630

Leu Ser Pro Ala Phe Glu Leu Ser Gln Trp Ser Ser Thr Glu Tyr
635 640 645

Ser Thr Trp Thr Glu Ser Arg Trp Lys Asp Ile Arg Ala Arg Ile
650 655 660

Phe Leu Ile Ala Ser Lys Glu Leu Glu Leu Ile Thr Leu Thr Val
665 670 675

Gly Phe Gly Ile Leu Ile Phe Ser Leu Ile Val Thr Tyr Cys Ile
680 685 690

Asn Ala Lys Ala Asp Val Leu Phe Ile Ala Pro Arg Glu Pro Gly
695 700 705

Ala Val Ser Tyr

<210> 203
<211> 2695
<212> DNA
<213> Homo Sapien

<400> 203
gctagaccga gccctgggag gctacgggct cccccggaaa ccctgccagg 50

ggagccgggt tttgagctca ggcgcctcta gcggcggccc ccagaaatct 100

gactcgcgag gccagagttg cagggactga atagcaaact gaggctgagt 150

agggaacaga ccatgaggtc agtgcagatc ttcctctccc aatgccgttt 200

gctccttcta ctagttccga caatgctcct taagtctctt ggcgaagatg 250

taatttttca ccctgaaggg gagtttgact cgtatgaagt caccattcct 300

gagaagctga gcttccgggg agaggtgcag ggtgtggtca gtcccgtgtc 350

ctacctactg cagttaaaag gcaagaagca cgtcctccat ttgtggccca 400

agagacttct gttgccccga catctgcgcg ttttctcctt cacagaacat 450

ggggaactgc tggaggatca tccttacata ccaaaggact gcaactacat 500

gggctccgtg aaagagtctc tggactctaa agctactata agcacatgca 550

tggggggtct ccgaggtgta tttaacattg atgccaaaca ttaccaaatt 600

gagcccctca aggcctctcc cagttttgaa catgtcgtct atctcctgaa 650

gaaagagcag tttgggaatc aggtttgtgg cttaagtgat gatgaaatag 700

aatggcagat ggccccttat gagaataagg cgaggctaag ggactttcct 750
ggatcctata aacacccaaa gtacttggaa ttgatcctac tctttgatca 800
aagtaggtat aggtttgtga acaacaatct ttctcaagtc atacatgatg 850
ccattctttt gactgggatt atggacacct actttcaaga tgttcgtatg 900
aggatacact taaaggctct tgaagtatgg acagatttta acaaaatacg 950
cgttggatat ccagagttag ctgaagtttt aggcagattt gtaatatata 1000
aaaaaagtgt attaaatgct cgcctgtcat cagattgggc acatttatat 1050
cttcaaagaa aatataatga tgctcttgca tggtcgtttg gaaaagtgtg 1100
ttctctagaa tatgctggat cagtgagtac tttactagat acaaatatcc 1150
ttgcccctgc tacctggtct gctcatgagc tgggtcatgc tgtaggaatg 1200
tcacatgatg aacaatactg ccaatgtagg ggtaggctta attgcatcat 1250
gggctcagga cgcactgggt ttagcaattg cagttatatc tcttttttta 1300
aacatatctc ttcgggagca acatgtctaa ataatatccc aggactaggt 1350
tatgtgctta agagatgtgg aaacaaaatt gtggaggaca atgaggaatg 1400
tgactgtggt tccacagagg agtgtcagaa agatcggtgt tgccaatcaa 1450
attgtaagtt gcaaccaggt gccaactgta gcattggact ttgctgtcat 1500
gattgtcggt ttcgtccatc tggatacgtg tgtaggcagg aaggaaatga 1550
atgtgacctt gcagagtact gcgacgggaa ttcaagttcc tgcccaaatg 1600
acgtttataa gcaggatgga acccccttgca agtatgaagg ccgttgtttc 1650
aggaaggggt gcagatccag atatatgcag tgccaaagca tttttggacc 1700
tgatgccatg gaggctccta gtgagtgcta tgatgcagtt aacttaatag 1750
gtgatcaatt tggtaactgt gagattacag gaattcgaaa ttttaaaaag 1800
tgtgaaagtg caaattcaat atgtggcagg ctacagtgta taaatgttga 1850
aaccatccct gatttgccag agcatacgac tataatttct actcatttac 1900
aggcagaaaa tctcatgtgc tggggcacag gctatcatct atccatgaaa 1950
cccatgggaa tacctgacct aggtatgata aatgatggca cctcctgtgg 2000
agaaggccgg gtatgtttta aaaaaaattg cgtcaatagc tcagtcctgc 2050
agtttgactg tttgcctgag aaatgcaata cccggggtgt ttgcaacaac 2100
agaaaaaact gccactgcat gtatgggtgg gcacctccat tctgtgagga 2150
agtggggtat ggaggaagca ttgacagtgg gcctccagga ctgctcagag 2200
gggcgattcc ctcgtcaatt tgggttgtgt ccatcataat gtttcgcctt 2250
attttattaa tcctttcagt ggtttttgtg tttttccggc aagtgatagg 2300

aaaccactta aaacccaaac aggaaaaaat gccactatcc aaagcaaaaa 2350

ctgaacagga agaatctaaa acaaaaactg tacaggaaga atctaaaaca 2400

aaaactggac aggaagaatc tgaagcaaaa actggacagg aagaatctaa 2450

agcaaaaact ggacaggaag aatctaaagc aaacattgaa agtaaacgac 2500

ccaaagcaaa gagtgtcaag aaacaaaaaa agtaaccggg caatccatac 2550

tcattcagta acacaggctc atttatttaa ccagctaatc atttatccaa 2600

aggctttcca ttcttctccc aatatttttt tactttaatt tttcccacaa 2650

gttttgatca gcaaataaac agcattcttg ttttggaaac aaaaa 2695

<210> 204
<211> 790
<212> PRT
<213> Homo Sapien

<400> 204
Met Arg Ser Val Gln Ile Phe Leu Ser Gln Cys Arg Leu Leu Leu
1 5 10 15

Leu Leu Val Pro Thr Met Leu Leu Lys Ser Leu Gly Glu Asp Val
20 25 30

Ile Phe His Pro Glu Gly Glu Phe Asp Ser Tyr Glu Val Thr Ile
35 40 45

Pro Glu Lys Leu Ser Phe Arg Gly Glu Val Gln Gly Val Val Ser
50 55 60

Pro Val Ser Tyr Leu Leu Gln Leu Lys Gly Lys Lys His Val Leu
65 70 75

His Leu Trp Pro Lys Arg Leu Leu Leu Pro Arg His Leu Arg Val
80 85 90

Phe Ser Phe Thr Glu His Gly Glu Leu Leu Glu Asp His Pro Tyr
95 100 105

Ile Pro Lys Asp Cys Asn Tyr Met Gly Ser Val Lys Glu Ser Leu
110 115 120

Asp Ser Lys Ala Thr Ile Ser Thr Cys Met Gly Gly Leu Arg Gly
125 130 135

Val Phe Asn Ile Asp Ala Lys His Tyr Gln Ile Glu Pro Leu Lys
140 145 150

Ala Ser Pro Ser Phe Glu His Val Val Tyr Leu Leu Lys Lys Glu
155 160 165

Gln Phe Gly Asn Gln Val Cys Gly Leu Ser Asp Asp Glu Ile Glu
170 175 180

Trp Gln Met Ala Pro Tyr Glu Asn Lys Ala Arg Leu Arg Asp Phe
185 190 195

Pro Gly Ser Tyr Lys His Pro Lys Tyr Leu Glu Leu Ile Leu Leu
200 205 210

```
Phe Asp Gln Ser Arg Tyr Arg Phe Val Asn Asn Asn Leu Ser Gln
                215                 220                 225

Val Ile His Asp Ala Ile Leu Leu Thr Gly Ile Met Asp Thr Tyr
                230                 235                 240

Phe Gln Asp Val Arg Met Arg Ile His Leu Lys Ala Leu Glu Val
                245                 250                 255

Trp Thr Asp Phe Asn Lys Ile Arg Val Gly Tyr Pro Glu Leu Ala
                260                 265                 270

Glu Val Leu Gly Arg Phe Val Ile Tyr Lys Lys Ser Val Leu Asn
                275                 280                 285

Ala Arg Leu Ser Ser Asp Trp Ala His Leu Tyr Leu Gln Arg Lys
                290                 295                 300

Tyr Asn Asp Ala Leu Ala Trp Ser Phe Gly Lys Val Cys Ser Leu
                305                 310                 315

Glu Tyr Ala Gly Ser Val Ser Thr Leu Leu Asp Thr Asn Ile Leu
                320                 325                 330

Ala Pro Ala Thr Trp Ser Ala His Glu Leu Gly His Ala Val Gly
                335                 340                 345

Met Ser His Asp Glu Gln Tyr Cys Gln Cys Arg Gly Arg Leu Asn
                350                 355                 360

Cys Ile Met Gly Ser Gly Arg Thr Gly Phe Ser Asn Cys Ser Tyr
                365                 370                 375

Ile Ser Phe Phe Lys His Ile Ser Ser Gly Ala Thr Cys Leu Asn
                380                 385                 390

Asn Ile Pro Gly Leu Gly Tyr Val Leu Lys Arg Cys Gly Asn Lys
                395                 400                 405

Ile Val Glu Asp Asn Glu Glu Cys Asp Cys Gly Ser Thr Glu Glu
                410                 415                 420

Cys Gln Lys Asp Arg Cys Cys Gln Ser Asn Cys Lys Leu Gln Pro
                425                 430                 435

Gly Ala Asn Cys Ser Ile Gly Leu Cys Cys His Asp Cys Arg Phe
                440                 445                 450

Arg Pro Ser Gly Tyr Val Cys Arg Gln Glu Gly Asn Glu Cys Asp
                455                 460                 465

Leu Ala Glu Tyr Cys Asp Gly Asn Ser Ser Ser Cys Pro Asn Asp
                470                 475                 480

Val Tyr Lys Gln Asp Gly Thr Pro Cys Lys Tyr Glu Gly Arg Cys
                485                 490                 495

Phe Arg Lys Gly Cys Arg Ser Arg Tyr Met Gln Cys Gln Ser Ile
                500                 505                 510

Phe Gly Pro Asp Ala Met Glu Ala Pro Ser Glu Cys Tyr Asp Ala
                515                 520                 525
```

```
Val Asn Leu Ile Gly Asp Gln Phe Gly Asn Cys Glu Ile Thr Gly
                530                 535                 540

Ile Arg Asn Phe Lys Lys Cys Glu Ser Ala Asn Ser Ile Cys Gly
                545                 550                 555

Arg Leu Gln Cys Ile Asn Val Glu Thr Ile Pro Asp Leu Pro Glu
                560                 565                 570

His Thr Thr Ile Ile Ser Thr His Leu Gln Ala Glu Asn Leu Met
                575                 580                 585

Cys Trp Gly Thr Gly Tyr His Leu Ser Met Lys Pro Met Gly Ile
                590                 595                 600

Pro Asp Leu Gly Met Ile Asn Asp Gly Thr Ser Cys Gly Glu Gly
                605                 610                 615

Arg Val Cys Phe Lys Lys Asn Cys Val Asn Ser Ser Val Leu Gln
                620                 625                 630

Phe Asp Cys Leu Pro Glu Lys Cys Asn Thr Arg Gly Val Cys Asn
                635                 640                 645

Asn Arg Lys Asn Cys His Cys Met Tyr Gly Trp Ala Pro Pro Phe
                650                 655                 660

Cys Glu Glu Val Gly Tyr Gly Gly Ser Ile Asp Ser Gly Pro Pro
                665                 670                 675

Gly Leu Leu Arg Gly Ala Ile Pro Ser Ser Ile Trp Val Val Ser
                680                 685                 690

Ile Ile Met Phe Arg Leu Ile Leu Leu Ile Leu Ser Val Val Phe
                695                 700                 705

Val Phe Phe Arg Gln Val Ile Gly Asn His Leu Lys Pro Lys Gln
                710                 715                 720

Glu Lys Met Pro Leu Ser Lys Ala Lys Thr Glu Gln Glu Glu Ser
                725                 730                 735

Lys Thr Lys Thr Val Gln Glu Glu Ser Lys Thr Lys Thr Gly Gln
                740                 745                 750

Glu Glu Ser Glu Ala Lys Thr Gly Gln Glu Glu Ser Lys Ala Lys
                755                 760                 765

Thr Gly Gln Glu Glu Ser Lys Ala Asn Ile Glu Ser Lys Arg Pro
                770                 775                 780

Lys Ala Lys Ser Val Lys Lys Gln Lys Lys
                785                 790
```

<210> 205
<211> 2782
<212> DNA
<213> Homo Sapien

<400> 205

```
cggacgcgtg ggcggacgcg tgggcggacg cgtgggggaa ggttgaatgg 50

ggtagaaggc ctgttgtgga gggaaaccac ccatcctcct gcctcccacc 100
```

accaccatca tcctggctgg acggagaggg tgacgggggc tgggaagggg 150
cagctcatgt tcaggtttcc aggaggggct acctgttgac tgtctttgca 200
ggaagaagaa aacacctgag tgaccagatg tcccagctcc aggtgccttg 250
ccagatggcc agaaccacac ctcttgaaga gtgacagtgc tgtggagcat 300
ggtttctgca cacctggaat gactggaacc ccaaagactc aagaaggagc 350
taaagatctt gaagtagaca tgaataaaac agaaggctgt ggaccacctg 400
tcgagatgga gaagtccttc tgaggctatc caaacacgga ccaggccatg 450
agaccccgat gaccatccct gaatttttc gagagtcagt caaccgattt 500
ggaacttatc cagccctccc atccaagaat ggcaaaaagt gggaaattct 550
gaatttcaac cagtactatg aggcttgtcg gaaggctgca aaatccttga 600
tcaagctggg tttggagcgt ttccacggag ttggtatcct ggggtttaac 650
tctgcagagt ggtttatcac tgctgttggt gccatcctag ccgggggtct 700
ttgtgttggt atttatgcca ccaactctgc cgaggcttgt caatatgtca 750
tcactcatgc caaagtgaac atcttgctgg ttgagaatga tcaacagtta 800
cagaaaatcc tttcgattcc acagagcagc ctagagcccc taaaagcgat 850
catccagtac agactgccaa tgaagaagaa caacaacttg tactcttggg 900
atgatttcat ggaacttggc agaagtatcc ctgacaccca actggagcag 950
gtcatcgaga gccagaaggc gaatcaatgc gcagtgctca tctacacttc 1000
agggaccaca ggcataccca agggagtgat gctcagtcat gacaacatca 1050
cgtggattgc aggagcagtg acaaaggact ttaaactgac agacaagcat 1100
gagacggtgg ttagctacct cccactcagc catattgcag cacagatgat 1150
ggacatctgg gtacccataa agattggggc gctcacatac tttgctcaag 1200
cagatgctct caagggcacc ttggtaagta ctctaaagga ggtaaaacct 1250
actgtcttca ttggagtgcc tcaaatttgg gagaagatac atgagatggt 1300
gaagaaaaat agtgccaagt ccatgggctt gaagaagaag gcattcgtgt 1350
gggcaagaaa cattggcttc aaggtcaact caaaaaagat gttggggaaa 1400
tataatactc ccgtgagcta ccgcatggct aagactctcg tgttcagcaa 1450
agtcaagaca tcccttggct tggatcactg tcactctttt atcagtggga 1500
ctgcgcccct caaccaagag actgccgagt tctttctaag cttggacata 1550
cctataggcg agttgtatgg gttgagtgag agctcgggac cccacacgat 1600
atccaaccag aataactaca ggcttctaag ctgtggcaag atcttgactg 1650
ggtgtaagaa tatgctgttc cagcagaaca aggatggcat tggggagatc 1700

tgcctctggg gtaggcacat cttcatgggc tatctggaaa gtgagactga 1750

aactacagag gccatcgatg atgaaggctg gctacactct ggggatctgg 1800

gccagctgga cggtctgggt ttcctctatg tcaccggcca catcaaagaa 1850

atccttatca ctgctggtgg tgaaaatgtg ccccccattc ctgttgagac 1900

cttggttaag aagaagatcc ccatcatcag taacgccatg ttagtaggag 1950

ataaactgaa gtttctgagc atgttgctga cgctgaagtg tgagatgaat 2000

cagatgagcg gagaacctct ggacaagctg aacttcgagg ccatcaactt 2050

ctgtcggggt ctgggcagcc aggcatccac cgtgactgag attgtgaagc 2100

agcaagaccc cctggtctac aaggccatcc agcaaggcat caatgctgtg 2150

aaccaggaag ccatgaacaa tgcacagagg attgaaaagt gggtcatctt 2200

ggagaaggac ttttccatct atggtggaga gctaggtcca atgatgaaac 2250

ttaagagaca ttttgtagcc cagaaataca aaaaacaaat tgatcacatg 2300

taccactgac tgctttgatg gagctgctct cagctgttct gatgccttca 2350

gcaggaagac ctcattgcaa taagtgaaat gctgctctag gtagaagctc 2400

tccctgctgt ttttaagaag ccacattcct cattggtcag tttcttgatt 2450

gttcgtctgt tggagaggtg ctccctagaa gaacctgcca tacgtttcaa 2500

agcaataaaa tcactgtata tctttctaag gaccttcaag tcatgactcc 2550

agggaagcct attgggaagt ctactaaaaa ctgcctgatt tacaagaaag 2600

acctgaactt gtgggctccc atttgatttt tttctcctca ggggactcag 2650

acattagaaa gaaaaagcct cacagatttg aagaactgga cccccaaatc 2700

aactcacctg cctggaagca actgggaaac ccttccaata agtcctgata 2750

ataaagcact tcagggtccc aaaaaaaaaa aa 2782

<210> 206
<211> 616
<212> PRT
<213> Homo Sapien

<400> 206

| | | | | | | | | | | | | | | |
|---|---|---|---|---|---|---|---|---|---|---|---|---|---|---|
| Met | Thr | Ile | Pro | Glu | Phe | Phe | Arg | Glu | Ser | Val | Asn | Arg | Phe | Gly |
| 1 | | | | 5 | | | | | 10 | | | | | 15 |
| Thr | Tyr | Pro | Ala | Leu | Pro | Ser | Lys | Asn | Gly | Lys | Lys | Trp | Glu | Ile |
| | | | | 20 | | | | | 25 | | | | | 30 |
| Leu | Asn | Phe | Asn | Gln | Tyr | Tyr | Glu | Ala | Cys | Arg | Lys | Ala | Ala | Lys |
| | | | | 35 | | | | | 40 | | | | | 45 |
| Ser | Leu | Ile | Lys | Leu | Gly | Leu | Glu | Arg | Phe | His | Gly | Val | Gly | Ile |
| | | | | 50 | | | | | 55 | | | | | 60 |
| Leu | Gly | Phe | Asn | Ser | Ala | Glu | Trp | Phe | Ile | Thr | Ala | Val | Gly | Ala |
| | | | | 65 | | | | | 70 | | | | | 75 |

Ile Leu Ala Gly Gly Leu Cys Val Gly Ile Tyr Ala Thr Asn Ser
80 85 90

Ala Glu Ala Cys Gln Tyr Val Ile Thr His Ala Lys Val Asn Ile
95 100 105

Leu Leu Val Glu Asn Asp Gln Gln Leu Gln Lys Ile Leu Ser Ile
110 115 120

Pro Gln Ser Ser Leu Glu Pro Leu Lys Ala Ile Ile Gln Tyr Arg
125 130 135

Leu Pro Met Lys Lys Asn Asn Asn Leu Tyr Ser Trp Asp Asp Phe
140 145 150

Met Glu Leu Gly Arg Ser Ile Pro Asp Thr Gln Leu Glu Gln Val
155 160 165

Ile Glu Ser Gln Lys Ala Asn Gln Cys Ala Val Leu Ile Tyr Thr
170 175 180

Ser Gly Thr Thr Gly Ile Pro Lys Gly Val Met Leu Ser His Asp
185 190 195

Asn Ile Thr Trp Ile Ala Gly Ala Val Thr Lys Asp Phe Lys Leu
200 205 210

Thr Asp Lys His Glu Thr Val Val Ser Tyr Leu Pro Leu Ser His
215 220 225

Ile Ala Ala Gln Met Met Asp Ile Trp Val Pro Ile Lys Ile Gly
230 235 240

Ala Leu Thr Tyr Phe Ala Gln Ala Asp Ala Leu Lys Gly Thr Leu
245 250 255

Val Ser Thr Leu Lys Glu Val Lys Pro Thr Val Phe Ile Gly Val
260 265 270

Pro Gln Ile Trp Glu Lys Ile His Glu Met Val Lys Lys Asn Ser
275 280 285

Ala Lys Ser Met Gly Leu Lys Lys Lys Ala Phe Val Trp Ala Arg
290 295 300

Asn Ile Gly Phe Lys Val Asn Ser Lys Lys Met Leu Gly Lys Tyr
305 310 315

Asn Thr Pro Val Ser Tyr Arg Met Ala Lys Thr Leu Val Phe Ser
320 325 330

Lys Val Lys Thr Ser Leu Gly Leu Asp His Cys His Ser Phe Ile
335 340 345

Ser Gly Thr Ala Pro Leu Asn Gln Glu Thr Ala Glu Phe Phe Leu
350 355 360

Ser Leu Asp Ile Pro Ile Gly Glu Leu Tyr Gly Leu Ser Glu Ser
365 370 375

Ser Gly Pro His Thr Ile Ser Asn Gln Asn Asn Tyr Arg Leu Leu
380 385 390

Ser Cys Gly Lys Ile Leu Thr Gly Cys Lys Asn Met Leu Phe Gln
395 400 405

Gln Asn Lys Asp Gly Ile Gly Glu Ile Cys Leu Trp Gly Arg His
410 415 420

Ile Phe Met Gly Tyr Leu Glu Ser Glu Thr Glu Thr Thr Glu Ala
425 430 435

Ile Asp Asp Glu Gly Trp Leu His Ser Gly Asp Leu Gly Gln Leu
440 445 450

Asp Gly Leu Gly Phe Leu Tyr Val Thr Gly His Ile Lys Glu Ile
455 460 465

Leu Ile Thr Ala Gly Gly Glu Asn Val Pro Pro Ile Pro Val Glu
470 475 480

Thr Leu Val Lys Lys Lys Ile Pro Ile Ile Ser Asn Ala Met Leu
485 490 495

Val Gly Asp Lys Leu Lys Phe Leu Ser Met Leu Leu Thr Leu Lys
500 505 510

Cys Glu Met Asn Gln Met Ser Gly Glu Pro Leu Asp Lys Leu Asn
515 520 525

Phe Glu Ala Ile Asn Phe Cys Arg Gly Leu Gly Ser Gln Ala Ser
530 535 540

Thr Val Thr Glu Ile Val Lys Gln Gln Asp Pro Leu Val Tyr Lys
545 550 555

Ala Ile Gln Gln Gly Ile Asn Ala Val Asn Gln Glu Ala Met Asn
560 565 570

Asn Ala Gln Arg Ile Glu Lys Trp Val Ile Leu Glu Lys Asp Phe
575 580 585

Ser Ile Tyr Gly Gly Glu Leu Gly Pro Met Met Lys Leu Lys Arg
590 595 600

His Phe Val Ala Gln Lys Tyr Lys Lys Gln Ile Asp His Met Tyr
605 610 615

His

<210> 207
<211> 2845
<212> DNA
<213> Homo Sapien

<400> 207
cccacgcgtc cgcccacgcg tccgcggacg cgtggggcca gatcgcggcc 50

ggcgccagcg ccaccgtccg gtccacccgc cagcccgcac agccgcgccg 100

ccgccgagcg tttcgtgagc ggcgctccga ggatcaggaa tggggcttcg 150

ggcgctgggc gcgctccgaa cccggcgcac gtaagagcct gggagcgccc 200

gagccgcccg gctgcccgga gccccatcgc ctaggaccgg gagatgctgg 250

aaatgcaacc gcctgttccc cgaggagccg ctgcccccgg gaccccctgg 300
cactgtgcgc accctggtca gcagcccccg gagaagacgg cgcccccaac 350
gcccgacccg cgtggccgtg gcagcgccac gcgagccctc taggcgaccg 400
cagggccaca gcagctcagc cgccggtgcc ccctcggaaa ccatgacccc 450
cggcgcgggc ccatggagcc atggcctata gggtcctggg ccgcgcgggg 500
ccacctcagc cgcggagggc gcgcaggctg ctcttcgcct tcacgctctc 550
gctctcctgc acttacctgt gttacagctt cctgtgctgc tgcgacgacc 600
tgggtcggag ccgcctcctc ggcgcgcctc gctgcctccg cggccccagc 650
gcgggcggcc agaaacttct ccagaagtcc cgcccctgtg atccctccgg 700
gccgacgccc agcgagccca gcgctcccag cgcgcccgcc gccgccgtgc 750
ccgcccctcg cctctccggt tccaaccact ccggctcacc caagctgggt 800
accaagcggt tgccccaagc cctcattgtg ggcgtgaaga aggggggcac 850
ccgggccgtg ctggagttta tccgagtaca cccggacgtg cgggccttgg 900
gcacggaacc ccacttcttt gacaggaact acggccgcgg gctggattgg 950
tacaggagcc tgatgcccag gaccctcgag agccagatca cgctggagaa 1000
gacgcccagc tactttgtca ctcaagaggc tcctcgacgc atcttcaaca 1050
tgtcccgaga caccaagctg atcgtggttg tgcggaaccc tgtgacccgt 1100
gccatctctg attacacgca gacactctcc aagaagcccg acatcccgac 1150
ctttgagggc ctctccttcc gcaaccgcac cctgggcctg gtggacgtgt 1200
catggaacgc catccgcatc ggcatgtacg tgctgcacct ggagagctgg 1250
ctgcagtact tcccgctagc tcagattcac ttcgtcagtg gcgagcgact 1300
catcactgac ccggccggcg agatggggcg agtccaggac ttcctgggca 1350
ttaagagatt catcacggac aagcacttct atttcaacaa gaccaaagga 1400
ttcccttgct tgaaaaaaac agaatcgagc ctcctgcctc gatgcttggg 1450
caaatcaaaa gggagaactc atgtacagat tgatcctgaa gtgatagacc 1500
agctccgaga attttataga ccgtataata tcaaatttta tgaaaccgtt 1550
gggcaggact tcaggtggga ataagcccac gaaaggaaag ggctctcaag 1600
ggctcttctg ctcatctctt ccgtgagatt tgctcccaga ccctctgatc 1650
tccctccaac aaaccctggc tccagccccc tttcccaact tgagttgcat 1700
catcttggaa ccaggaagcc cagctaaagc caagagacca gagagtccct 1750
gccactagtt ttcatcagtc tgttcaagca aagttgatct gctcctggca 1800
cgtccagtaa attccagaat cattctcctt tctgcccata aagggccttg 1850

gagaattgct ttaagaagag tgaatgttcc aatgatgata gatattataa 1900

gcgatgatgg ttctgttgct atgaacacag cagtcggtcc ctgtcattgt 1950

ccacccagga gtggccttgt taattccaag tggcatgtat cttccctctg 2000

agcttcattt cttcaagatg ctctgggtgg tgggatggga gaccatcctc 2050

agccctcctc agaccttatc aattcattga gagattgcaa agctgaaagc 2100

acctccggcc actcctggga gacagaccct ttggtgatga aataaaccag 2150

tgacttcaga gcctatggtc tcaactgtgc ttgaaaaaca ctgtctctga 2200

aaacaacttt gtgattctcc ctgctccctg tggacaaaag cacataattc 2250

tgctgttacg ggtactttgc tcatacgagc tttcatgttc agcatgcaat 2300

ggaatcatgc ttgtccatgt gaaataaata tggctctctc gtgtccttaa 2350

tgctgggctt ttctctgtaa gctggttctg cagcacaatt cattaattaa 2400

acttctccca gtgcaagaag gcagctggtg ctgggggtgg tctgggggt 2450

cagggaggag ggcaaggact acatggggca gaggcaaggc ggtggtggag 2500

atgaggaaag aagttcttct tggcagaagc tggggcagaa agatcacatg 2550

agatctgtgg ggacaccctc tatctgaaac ataagtctgt gttcattctc 2600

tgcttagaaa ttttagatct gaagtgctac actgaaggtc cgaaggttga 2650

tggggcatca gatatctttt tggttggcca gcatgatatt ttgaaataac 2700

tgtcaacagt tagaaactgg gagcattcat atgtaaaaaa tatggatttt 2750

cagcttcttc ttaaaaaaaa aaaaaaaaaa aaaaaaaaaa aaaaaaaaaa 2800

aaaaaaaaaa aaaaaaaaaa aaaaaaaaaa aaaaaaaaaa aaaaa 2845

<210> 208
<211> 367
<212> PRT
<213> Homo Sapien

<400> 208
Met Ala Tyr Arg Val Leu Gly Arg Ala Gly Pro Pro Gln Pro Arg
1 5 10 15

Arg Ala Arg Arg Leu Leu Phe Ala Phe Thr Leu Ser Leu Ser Cys
20 25 30

Thr Tyr Leu Cys Tyr Ser Phe Leu Cys Cys Cys Asp Asp Leu Gly
35 40 45

Arg Ser Arg Leu Leu Gly Ala Pro Arg Cys Leu Arg Gly Pro Ser
50 55 60

Ala Gly Gly Gln Lys Leu Leu Gln Lys Ser Arg Pro Cys Asp Pro
65 70 75

Ser Gly Pro Thr Pro Ser Glu Pro Ser Ala Pro Ser Ala Pro Ala
80 85 90

```
Ala Ala Val Pro Ala Pro Arg Leu Ser Gly Ser Asn His Ser Gly
                95                  100                 105

Ser Pro Lys Leu Gly Thr Lys Arg Leu Pro Gln Ala Leu Ile Val
                110                 115                 120

Gly Val Lys Lys Gly Gly Thr Arg Ala Val Leu Glu Phe Ile Arg
                125                 130                 135

Val His Pro Asp Val Arg Ala Leu Gly Thr Glu Pro His Phe Phe
                140                 145                 150

Asp Arg Asn Tyr Gly Arg Gly Leu Asp Trp Tyr Arg Ser Leu Met
                155                 160                 165

Pro Arg Thr Leu Glu Ser Gln Ile Thr Leu Glu Lys Thr Pro Ser
                170                 175                 180

Tyr Phe Val Thr Gln Glu Ala Pro Arg Arg Ile Phe Asn Met Ser
                185                 190                 195

Arg Asp Thr Lys Leu Ile Val Val Val Arg Asn Pro Val Thr Arg
                200                 205                 210

Ala Ile Ser Asp Tyr Thr Gln Thr Leu Ser Lys Lys Pro Asp Ile
                215                 220                 225

Pro Thr Phe Glu Gly Leu Ser Phe Arg Asn Arg Thr Leu Gly Leu
                230                 235                 240

Val Asp Val Ser Trp Asn Ala Ile Arg Ile Gly Met Tyr Val Leu
                245                 250                 255

His Leu Glu Ser Trp Leu Gln Tyr Phe Pro Leu Ala Gln Ile His
                260                 265                 270

Phe Val Ser Gly Glu Arg Leu Ile Thr Asp Pro Ala Gly Glu Met
                275                 280                 285

Gly Arg Val Gln Asp Phe Leu Gly Ile Lys Arg Phe Ile Thr Asp
                290                 295                 300

Lys His Phe Tyr Phe Asn Lys Thr Lys Gly Phe Pro Cys Leu Lys
                305                 310                 315

Lys Thr Glu Ser Ser Leu Leu Pro Arg Cys Leu Gly Lys Ser Lys
                320                 325                 330

Gly Arg Thr His Val Gln Ile Asp Pro Glu Val Ile Asp Gln Leu
                335                 340                 345

Arg Glu Phe Tyr Arg Pro Tyr Asn Ile Lys Phe Tyr Glu Thr Val
                350                 355                 360

Gly Gln Asp Phe Arg Trp Glu
                365
```

<210> 209
<211> 2915
<212> DNA
<213> Homo Sapien

<400> 209
ctttccttat ctgtgtgtac tcttatctca ctgttctatt ttttctcctc 50

atttatatta actctttctt accttttttt ctgaacttct aggccttctc 100
tttccagaac tggtggaaga caaatgaaac ggccaagatg gtaagaaaca 150
agccgcattt ctccttgggg agactgataa tttaaaaggt ttgttgtgtc 200
agaaacattc ccagcttcat caccaaccct ttccttccac ctctgcccac 250
tggagaccac ttacatcccg aagcggacgc ggcagctgaa gtcaggaaac 300
catgcatcac attagcagga gccaactgca gactttaaac tccgttcaac 350
atgtggatgc ggcagagaaa tgacctgtcc agacaagccg gggcagctca 400
taaactggtt catctgctcc ctgtgcgtcc cgcgggtgcg taagctctgg 450
agcagccggc gtccaaggac ccggagaaac cttctgctgg gcactgcgtg 500
tgccatctac ttgggcttcc tggtgagcca ggtggggagg gcctctctcc 550
agcatggaca ggcggctgag aaggggccac atcgcagccg cgacaccgcc 600
gagccatcct tccctgagat acccctggat ggtaccctgg cccctccaga 650
gtcccagggc aatgggtcca ctctgcagcc caatgtggtg tacattaccc 700
tacgctccaa gcgcagcaag ccggccaata tccgtggcac cgtgaagccc 750
aagcgcagga aaaagcatgc agtggcatcg gctgccccag ggcaggaggc 800
tttggtcgga ccatcccttc agccgcagga agcggcaagg gaagctgatg 850
ctgtagcacc tgggtacgct cagggagcaa acctggttaa gattggagag 900
cgaccctgga ggttggtgcg gggtccggga gtgcgagccg ggggcccaga 950
cttcctgcag cccagctcca gggagagcaa cattaggatc tacagcgaga 1000
gcgccccctc ctggctgagc aaagatgaca tccgaagaat gcgactcttg 1050
gcggacagcg cagtggcagg gctccggcct gtgtcctcta ggagcggagc 1100
ccgtttgctg gtgctggagg ggggcgcacc tggcgctgtg ctccgctgtg 1150
gccctagccc ctgtgggctt ctcaagcagc ccttggacat gagtgaggtg 1200
tttgccttcc acctagacag gatcctgggg ctcaacagga ccctgccgtc 1250
tgtgagcagg aaagcagagt tcatccaaga tggccgccca tgccccatca 1300
ttctttggga tgcatcttta tcttcagcaa gtaatgacac ccattcttct 1350
gttaagctca cctggggaac ttatcagcag ttgctgaaac agaaatgctg 1400
gcagaatggc cgagtaccca agcctgaatc aggttgtact gaaatacatc 1450
atcatgagtg gtccaagatg gcactctttg atttttttgtt acagatttat 1500
aatcgcttag atacaaattg ctgtggattc agacctcgca aggaagatgc 1550
ctgtgtacag aatggattga ggccaaaatg tgatgaccaa ggttctgcgg 1600
ctctagcaca cattatccag cgaaagcatg acccaaggca tttggttttt 1650

atagacaaca agggtttctt tgacaggagt gaagataact taaacttcaa 1700

attgttagaa ggcatcaaag agtttccagc ttctgcagtt tctgttttga 1750

agagccagca cttacggcag aaacttcttc agtctctgtt tcttgataaa 1800

gtgtattggg aaagtcaagg aggtagacaa ggaattgaaa agcttatcga 1850

tgtaatagaa cacagagcca aaattcttat cacctatatc aatgcacacg 1900

gggtcaaagt attacctatg aatgaatgac aaaagaatct tctggctagg 1950

gtgttagata tatttatgca tttttggttt tgtttttaaa tcaagcacat 2000

caacctcaag cccgtttagc aatgaggcag tgtagatgaa tacgtaaaat 2050

aaatgacttt aaccaagtag ctataaaggg acttagcact gtatgcatac 2100

ttaaaaaggt tttgaaaaac aaactacttg agaaatattt gtttatattt 2150

ttctctaaca tcatgctatg tgtcagtctg aacatctgac aacagaaatt 2200

tcagttatta ttctagctaa gttttgaaaa catttgtcat gctgtttaat 2250

agaaaactgc aaaccagaga tactgactcc attaataaac catattttgt 2300

gccgttttga ctgttctgac caaatactaa tgggaacaat tcttgacgtt 2350

tttctgttgc tgattgttaa catagagcag tctctacact accctgaggc 2400

aactctacat tggaacactg aggcttacag cctgcaagag catcagagct 2450

gaccatacat ttaaacagaa atgctggttt atttgcaaaa tcaccagtat 2500

attttctatt gtgtctataa aaaatcagtc atttaagtac aagaatcata 2550

ttttccattc ctttttagaa atttattttg ttgtccctat ggaaatcatt 2600

cacatctgac aatttatatg ttaaagagtt ttactctctc tattttggtc 2650

caatttgtat ctagtggctg agaaattaaa taattctaaa gtatgaagtt 2700

acctatctga aaatgtactt acagagtatc attttaaaat ggatgtctct 2750

ttaaaaattt tgttactttt accaacaatg taatataatt tatgtatatt 2800

ttattaataa tagtgaattc cttaaaattt gttctatgta cttatattta 2850

atttgattta atggttactg cccagatatt gagaaatggt tcaaatattg 2900

agtgtgtttc aataa 2915

<210> 210
<211> 519
<212> PRT
<213> Homo Sapien

<400> 210

```
Met Thr Cys Pro Asp Lys Pro Gly Gln Leu Ile Asn Trp Phe Ile
  1               5                  10                  15

Cys Ser Leu Cys Val Pro Arg Val Arg Lys Leu Trp Ser Ser Arg
                 20                  25                  30
```

Arg Pro Arg Thr Arg Arg Asn Leu Leu Leu Gly Thr Ala Cys Ala
35 40 45

Ile Tyr Leu Gly Phe Leu Val Ser Gln Val Gly Arg Ala Ser Leu
50 55 60

Gln His Gly Gln Ala Ala Glu Lys Gly Pro His Arg Ser Arg Asp
65 70 75

Thr Ala Glu Pro Ser Phe Pro Glu Ile Pro Leu Asp Gly Thr Leu
80 85 90

Ala Pro Pro Glu Ser Gln Gly Asn Gly Ser Thr Leu Gln Pro Asn
95 100 105

Val Val Tyr Ile Thr Leu Arg Ser Lys Arg Ser Lys Pro Ala Asn
110 115 120

Ile Arg Gly Thr Val Lys Pro Lys Arg Arg Lys Lys His Ala Val
125 130 135

Ala Ser Ala Ala Pro Gly Gln Glu Ala Leu Val Gly Pro Ser Leu
140 145 150

Gln Pro Gln Glu Ala Ala Arg Glu Ala Asp Ala Val Ala Pro Gly
155 160 165

Tyr Ala Gln Gly Ala Asn Leu Val Lys Ile Gly Glu Arg Pro Trp
170 175 180

Arg Leu Val Arg Gly Pro Gly Val Arg Ala Gly Gly Pro Asp Phe
185 190 195

Leu Gln Pro Ser Ser Arg Glu Ser Asn Ile Arg Ile Tyr Ser Glu
200 205 210

Ser Ala Pro Ser Trp Leu Ser Lys Asp Asp Ile Arg Arg Met Arg
215 220 225

Leu Leu Ala Asp Ser Ala Val Ala Gly Leu Arg Pro Val Ser Ser
230 235 240

Arg Ser Gly Ala Arg Leu Leu Val Leu Glu Gly Gly Ala Pro Gly
245 250 255

Ala Val Leu Arg Cys Gly Pro Ser Pro Cys Gly Leu Leu Lys Gln
260 265 270

Pro Leu Asp Met Ser Glu Val Phe Ala Phe His Leu Asp Arg Ile
275 280 285

Leu Gly Leu Asn Arg Thr Leu Pro Ser Val Ser Arg Lys Ala Glu
290 295 300

Phe Ile Gln Asp Gly Arg Pro Cys Pro Ile Ile Leu Trp Asp Ala
305 310 315

Ser Leu Ser Ser Ala Ser Asn Asp Thr His Ser Ser Val Lys Leu
320 325 330

Thr Trp Gly Thr Tyr Gln Gln Leu Leu Lys Gln Lys Cys Trp Gln
335 340 345

Asn Gly Arg Val Pro Lys Pro Glu Ser Gly Cys Thr Glu Ile His
350 355 360

His His Glu Trp Ser Lys Met Ala Leu Phe Asp Phe Leu Leu Gln
365 370 375

Ile Tyr Asn Arg Leu Asp Thr Asn Cys Cys Gly Phe Arg Pro Arg
380 385 390

Lys Glu Asp Ala Cys Val Gln Asn Gly Leu Arg Pro Lys Cys Asp
395 400 405

Asp Gln Gly Ser Ala Ala Leu Ala His Ile Ile Gln Arg Lys His
410 415 420

Asp Pro Arg His Leu Val Phe Ile Asp Asn Lys Gly Phe Phe Asp
425 430 435

Arg Ser Glu Asp Asn Leu Asn Phe Lys Leu Leu Glu Gly Ile Lys
440 445 450

Glu Phe Pro Ala Ser Ala Val Ser Val Leu Lys Ser Gln His Leu
455 460 465

Arg Gln Lys Leu Leu Gln Ser Leu Phe Leu Asp Lys Val Tyr Trp
470 475 480

Glu Ser Gln Gly Gly Arg Gln Gly Ile Glu Lys Leu Ile Asp Val
485 490 495

Ile Glu His Arg Ala Lys Ile Leu Ile Thr Tyr Ile Asn Ala His
500 505 510

Gly Val Lys Val Leu Pro Met Asn Glu
515

<210> 211
<211> 3266
<212> DNA
<213> Homo Sapien

<400> 211
gtggggtggt gagcgcagcg ccgaggatga ggaggtgcaa cagcggctcc 50

gggccgccgc cgtcgctgct gctgctgctg ctgtggctgc tcgcggttcc 100

cggcgctaac gcggccccgc ggtcggcgct ctattcgcct tccgacccgc 150

tgacgctgct gcaggcggac acggtgcgcg gcgcggtgct gggctcccgc 200

agcgcctggg ccgtggagtt cttcgcctcc tggtgcggcc actgcatcgc 250

cttcgccccg acgtggaagg cgctggccga agacgtcaaa gcctggaggc 300

cggccctgta tctcgccgcc ctggactgtg ctgaggagac caacagtgca 350

gtctgcagag acttcaacat ccctggcttc ccgactgtga ggttcttcaa 400

ggcctttacc aagaacggct cgggagcagt atttccagtg gctggtgctg 450

acgtgcagac gctgcgggag aggctcattg acgccctgga gtcccatcat 500

gacacgtggc ccccagcctg tcccccactg gagcctgcca agctggagga 550

gattgatgga ttctttgcga gaaataacga agagtacctg gctctgatct 600

ttgaaaaggg aggctcctac ctgggtagag aggtggctct ggacctgtcc 650

cagcacaaag gcgtggcggt gcgcagggtg ctgaacacag aggccaatgt 700

ggtgagaaag tttggtgtca ccgacttccc ctcttgctac ctgctgttcc 750

ggaatggctc tgtctcccga gtccccgtgc tcatggaatc caggtccttc 800

tataccgctt acctgcagag actctctggg ctcaccaggg aggctgccca 850

gaccacagtt gcaccaacca ctgctaacaa gatagctccc actgtttgga 900

aattggcaga tcgctccaag atctacatgg ctgacctgga atctgcactg 950

cactacatcc tgcggataga agtgggcagg ttcccggtcc tggaagggca 1000

gcgcctggtg gccctgaaaa agtttgtggc agtgctggcc aagtatttcc 1050

ctggccggcc cttagtccag aacttcctgc actccgtgaa tgaatggctc 1100

aagaggcaga agagaaataa aattccctac agtttcttta aaactgccct 1150

ggacgacagg aaagagggtg ccgttcttgc caagaaggtg aactggattg 1200

gctgccaggg gagtgagccg catttccggg gctttccctg ctccctgtgg 1250

gtcctcttcc acttcttgac tgtgcaggca gctcggcaaa atgtagacca 1300

ctcacaggaa gcagccaagg ccaaggaggt cctcccagcc atccgaggct 1350

acgtgcacta cttcttcggc tgccgagact gcgctagcca cttcgagcag 1400

atggctgctg cctccatgca ccgggtgggg agtcccaacg ccgctgtcct 1450

ctggctctgg tctagccaca acagggtcaa tgctcgcctt gcaggtgccc 1500

ccagcgagga cccccagttc cccaaggtgc agtggccacc ccgtgaactt 1550

tgttctgcct gccacaatga acgcctggat gtgcccgtgt gggacgtgga 1600

agccaccctc aacttcctca aggcccactt ctccccaagc aacatcatcc 1650

tggacttccc tgcagctggg tcagctgccc ggagggatgt gcagaatgtg 1700

gcagccgccc cagagctggc gatgggagcc ctggagctgg aaagccggaa 1750

ttcaactctg gaccctggga agcctgagat gatgaagtcc cccacaaaca 1800

ccaccccaca tgtgccggct gagggacctg aggcaagtcg acccccgaag 1850

ctgcaccctg gcctcagagc tgcaccaggc caggagcctc ctgagcacat 1900

ggcagagctt cagaggaatg agcaggagca gccgcttggg cagtggcact 1950

tgagcaagcg agacacaggg gctgcattgc tggctgagtc cagggctgag 2000

aagaaccgcc tctggggccc tttggaggtc aggcgcgtgg gccgcagctc 2050

caagcagctg gtcgacatcc ctgagggcca gctggaggcc cgagctggac 2100

ggggccgagg ccagtggctg caggtgctgg gagggggctt ctcttacctg 2150

gacatcagcc tctgtgtggg gctctattcc ctgtccttca tgggcctgct 2200
ggccatgtac acctacttcc aggccaagat aagggccctg aagggccatg 2250
ctggccaccc tgcagcctga accacctggg gaggaggcgg gagagggagc 2300
tgccatctct aggcacctca agccccctga ccccattccc tcccctccca 2350
ccccttgctc cttgtctggc ctagaagtgt gggaaattca ggaaaacgag 2400
ttgctccagt gaagcttctt ggggttgcta ggacagagag ctcctttgac 2450
acaaaagaca ggagcagggt ccaggttccc ctgctgtgca gggagggcag 2500
ccccgggcag tgggcatagg gcagctcagt ccctggcctc ttagcaccac 2550
attcctgttt ttcagcttat ttgaagtcct gcctcattct cactggagcc 2600
tcagtctctc ctgcttggtc ttggccctca actggggcaa gtgaagccag 2650
aggagggtcc cccagctggg tgggctggaa tggaactcct cactagctgc 2700
tggggctccg cccaccctgc tcccttccgg acaatgaaga agcctttgca 2750
ccctgggagg aaggaccacc ccgggccctc tatgcctggc cagcctccag 2800
ctcctcagac ctcctgggtg gggtttggct tcagggtggg gtttggaagc 2850
ttctggaagt cgtgctggtc tcccaggtga ggcaagccat ggttgctggg 2900
ctgtagggtg agtggcttgc ttggtgggac ctgacgagtt ggtggcatgg 2950
gaaggatgtg ggtctctagt gccttgccct ggcttagctg caggagaaga 3000
tggctgcttt cacttccccc cattgagctc tgctccctct gagcctggtc 3050
ttttgtcctt ttttattttg gtctccaaga tgaatgctca tctttggagg 3100
gtgccaggta gaagctaggg aggggagtgt cttctctctc caggtttcac 3150
cttccagtgt gcagaagtta gaagggtctg gcgggggcag tgccttacac 3200
atgcttgatt cccacgctac cccctgcctt gggaggtgtg tggaataaat 3250
tatttttgtt aaggca 3266

<210> 212
<211> 747
<212> PRT
<213> Homo Sapien

<400> 212
Met Arg Arg Cys Asn Ser Gly Ser Gly Pro Pro Pro Ser Leu Leu
1 5 10 15

Leu Leu Leu Leu Trp Leu Leu Ala Val Pro Gly Ala Asn Ala Ala
20 25 30

Pro Arg Ser Ala Leu Tyr Ser Pro Ser Asp Pro Leu Thr Leu Leu
35 40 45

Gln Ala Asp Thr Val Arg Gly Ala Val Leu Gly Ser Arg Ser Ala
50 55 60

Trp Ala Val Glu Phe Phe Ala Ser Trp Cys Gly His Cys Ile Ala
65 70 75

Phe Ala Pro Thr Trp Lys Ala Leu Ala Glu Asp Val Lys Ala Trp
80 85 90

Arg Pro Ala Leu Tyr Leu Ala Ala Leu Asp Cys Ala Glu Glu Thr
95 100 105

Asn Ser Ala Val Cys Arg Asp Phe Asn Ile Pro Gly Phe Pro Thr
110 115 120

Val Arg Phe Phe Lys Ala Phe Thr Lys Asn Gly Ser Gly Ala Val
125 130 135

Phe Pro Val Ala Gly Ala Asp Val Gln Thr Leu Arg Glu Arg Leu
140 145 150

Ile Asp Ala Leu Glu Ser His His Asp Thr Trp Pro Pro Ala Cys
155 160 165

Pro Pro Leu Glu Pro Ala Lys Leu Glu Glu Ile Asp Gly Phe Phe
170 175 180

Ala Arg Asn Asn Glu Glu Tyr Leu Ala Leu Ile Phe Glu Lys Gly
185 190 195

Gly Ser Tyr Leu Gly Arg Glu Val Ala Leu Asp Leu Ser Gln His
200 205 210

Lys Gly Val Ala Val Arg Arg Val Leu Asn Thr Glu Ala Asn Val
215 220 225

Val Arg Lys Phe Gly Val Thr Asp Phe Pro Ser Cys Tyr Leu Leu
230 235 240

Phe Arg Asn Gly Ser Val Ser Arg Val Pro Val Leu Met Glu Ser
245 250 255

Arg Ser Phe Tyr Thr Ala Tyr Leu Gln Arg Leu Ser Gly Leu Thr
260 265 270

Arg Glu Ala Ala Gln Thr Thr Val Ala Pro Thr Thr Ala Asn Lys
275 280 285

Ile Ala Pro Thr Val Trp Lys Leu Ala Asp Arg Ser Lys Ile Tyr
290 295 300

Met Ala Asp Leu Glu Ser Ala Leu His Tyr Ile Leu Arg Ile Glu
305 310 315

Val Gly Arg Phe Pro Val Leu Glu Gly Gln Arg Leu Val Ala Leu
320 325 330

Lys Lys Phe Val Ala Val Leu Ala Lys Tyr Phe Pro Gly Arg Pro
335 340 345

Leu Val Gln Asn Phe Leu His Ser Val Asn Glu Trp Leu Lys Arg
350 355 360

Gln Lys Arg Asn Lys Ile Pro Tyr Ser Phe Phe Lys Thr Ala Leu
365 370 375

Asp Asp Arg Lys Glu Gly Ala Val Leu Ala Lys Lys Val Asn Trp

```
                380                 385                 390

Ile Gly Cys Gln Gly Ser Glu Pro His Phe Arg Gly Phe Pro Cys
                395                 400                 405

Ser Leu Trp Val Leu Phe His Phe Leu Thr Val Gln Ala Ala Arg
                410                 415                 420

Gln Asn Val Asp His Ser Gln Glu Ala Ala Lys Ala Lys Glu Val
                425                 430                 435

Leu Pro Ala Ile Arg Gly Tyr Val His Tyr Phe Phe Gly Cys Arg
                440                 445                 450

Asp Cys Ala Ser His Phe Glu Gln Met Ala Ala Ala Ser Met His
                455                 460                 465

Arg Val Gly Ser Pro Asn Ala Ala Val Leu Trp Leu Trp Ser Ser
                470                 475                 480

His Asn Arg Val Asn Ala Arg Leu Ala Gly Ala Pro Ser Glu Asp
                485                 490                 495

Pro Gln Phe Pro Lys Val Gln Trp Pro Pro Arg Glu Leu Cys Ser
                500                 505                 510

Ala Cys His Asn Glu Arg Leu Asp Val Pro Val Trp Asp Val Glu
                515                 520                 525

Ala Thr Leu Asn Phe Leu Lys Ala His Phe Ser Pro Ser Asn Ile
                530                 535                 540

Ile Leu Asp Phe Pro Ala Ala Gly Ser Ala Ala Arg Arg Asp Val
                545                 550                 555

Gln Asn Val Ala Ala Ala Pro Glu Leu Ala Met Gly Ala Leu Glu
                560                 565                 570

Leu Glu Ser Arg Asn Ser Thr Leu Asp Pro Gly Lys Pro Glu Met
                575                 580                 585

Met Lys Ser Pro Thr Asn Thr Thr Pro His Val Pro Ala Glu Gly
                590                 595                 600

Pro Glu Ala Ser Arg Pro Pro Lys Leu His Pro Gly Leu Arg Ala
                605                 610                 615

Ala Pro Gly Gln Glu Pro Pro Glu His Met Ala Glu Leu Gln Arg
                620                 625                 630

Asn Glu Gln Glu Gln Pro Leu Gly Gln Trp His Leu Ser Lys Arg
                635                 640                 645

Asp Thr Gly Ala Ala Leu Leu Ala Glu Ser Arg Ala Glu Lys Asn
                650                 655                 660

Arg Leu Trp Gly Pro Leu Glu Val Arg Arg Val Gly Arg Ser Ser
                665                 670                 675

Lys Gln Leu Val Asp Ile Pro Glu Gly Gln Leu Glu Ala Arg Ala
                680                 685                 690

Gly Arg Gly Arg Gly Gln Trp Leu Gln Val Leu Gly Gly Gly Phe
                695                 700                 705
```

Ser Tyr Leu Asp Ile Ser Leu Cys Val Gly Leu Tyr Ser Leu Ser
710 715 720

Phe Met Gly Leu Leu Ala Met Tyr Thr Tyr Phe Gln Ala Lys Ile
725 730 735

Arg Ala Leu Lys Gly His Ala Gly His Pro Ala Ala
740 745

<210> 213
<211> 1955
<212> DNA
<213> Homo Sapien

<400> 213
gcacgaggcc gacttccaga ccatctacaa ctgcacggcc tggaacagct 50

tcggctccga cactgagatc atccggctca aggagcaagg ttcggaaatg 100

aagtcgggag ccgggctgga agcagagtct gtgccgatgg ccgtcatcat 150

tggggtggcc gtaggagctg gtgtggcctt cctcgtcctt atggcaacca 200

tcgtggcgtt ctgctgtgcc cgttcccaga gaaatctcaa aggtgttgtg 250

tcagccaaaa atgatatccg agtggaaatt gtccacaagg aaccagcctc 300

tggtcgggag ggtgaggagc actccaccat caagcagctg atgatggacc 350

ggggtgaatt ccagcaagac tcagtcctga aacagctgga ggtcctcaaa 400

gaagaggaga aagagtttca gaacctgaag gaccccacca atggctacta 450

cagcgtcaac accttcaaag agcaccactc aaccccgacc atctccctct 500

ccagctgcca gcccgacctg cgtcctgcgg gtaagcagcg tgtgcccaca 550

ggcatgtcct tcaccaacat ctacagcacc ctgagcggcc agggccgcct 600

ctacgactac gggcagcggt ttgtgctggg catgggcagc tcgtccatcg 650

agctttgtga gcgggagttc cagagaggct ccctcagcga cagcagctcc 700

ttcctggaca cgcagtgtga cagcagcgtc agcagcagcg gcaagcagga 750

tggctatgtg cagttcgaca aggccagcaa ggcttctgct tcctcctccc 800

accactccca gtcctcgtcc cagaactctg accccagtcg acccctgcag 850

cggcggatgc agactcacgt ctaaggatca cacaccgcgg gtggggacgg 900

gccagggaag aggtcagggc acgttctggt tgtccaggga cgaggggtac 950

tttgcagagg acaccagaat tggccacttc caggacagcc tcccagcgcc 1000

tctgccactg ccttccttcg aagctctgat caagcacaaa tctgggtccc 1050

caggtgctgt gtgccagagg tgggcgggtg gggagacaga cagaggctgc 1100

ggctgagtgc gctgtgctta gtgctggaca cccgtgtccc cggccctttc 1150

ctggaggccc ctctaccacc tgctctgccc acaggcacaa gtggcagcta 1200

taactctgct ttcatgaaac tgcggtccac tctctggtct ctctgtgggc 1250

tctacccctc actgaccaca agctctacct acccctgtgc ctgtgctccc 1300

atacagccct ggggagaagg ggatgacgtc ttcccagcac tgagctgccc 1350

cagaaacccc ggctccccac tgctgctcat agcccatacc ctggaggctg 1400

acaagccaga aatggccttg gctaaaggag cctctctctc accaggctgg 1450

ccgggagccc acccccaatt tgtttggtgt tttgtgtcca tactcttgca 1500

gttctgtcct tggacttgat gccgctgaac tctgcggtgg gaccggtccc 1550

gtcagagcct ggtgtactgg ggggagggag ggaggaggga gcctgtgctg 1600

acggagcacc tcgccgggtg tgcccctcct gggctgtgtg accccagcct 1650

ccccacccac ctcctgcttt gtgtactcct cccctccccc tcagcacaat 1700

cggagttcat ataagaagtg cgggagcttc tctggtcagg gttctctgaa 1750

cacttatgga gagagtgctt cctgggaagt gtggcgtttg aaggggctgg 1800

agggcaggtc tttaagatgg cgagactgcc cttctcagct gataaacaca 1850

agaacggcga tcctgtcttc agtaaggctc cacgagaaga gaggaagtat 1900

atctacacct caaccctcct agtcaccacc tgaaataaat gttagggaaa 1950

aaaaa 1955

<210> 214
<211> 245
<212> PRT
<213> Homo Sapien

<400> 214

```
Met Ala Val Ile Ile Gly Val Ala Val Gly Ala Gly Val Ala Phe
1               5                   10                  15

Leu Val Leu Met Ala Thr Ile Val Ala Phe Cys Cys Ala Arg Ser
                20                  25                  30

Gln Arg Asn Leu Lys Gly Val Val Ser Ala Lys Asn Asp Ile Arg
                35                  40                  45

Val Glu Ile Val His Lys Glu Pro Ala Ser Gly Arg Glu Gly Glu
                50                  55                  60

Glu His Ser Thr Ile Lys Gln Leu Met Met Asp Arg Gly Glu Phe
                65                  70                  75

Gln Gln Asp Ser Val Leu Lys Gln Leu Glu Val Leu Lys Glu Glu
                80                  85                  90

Glu Lys Glu Phe Gln Asn Leu Lys Asp Pro Thr Asn Gly Tyr Tyr
                95                  100                 105

Ser Val Asn Thr Phe Lys Glu His His Ser Thr Pro Thr Ile Ser
                110                 115                 120

Leu Ser Ser Cys Gln Pro Asp Leu Arg Pro Ala Gly Lys Gln Arg
                125                 130                 135
```

Val Pro Thr Gly Met Ser Phe Thr Asn Ile Tyr Ser Thr Leu Ser
140 145 150

Gly Gln Gly Arg Leu Tyr Asp Tyr Gly Gln Arg Phe Val Leu Gly
155 160 165

Met Gly Ser Ser Ser Ile Glu Leu Cys Glu Arg Glu Phe Gln Arg
170 175 180

Gly Ser Leu Ser Asp Ser Ser Ser Phe Leu Asp Thr Gln Cys Asp
185 190 195

Ser Ser Val Ser Ser Ser Gly Lys Gln Asp Gly Tyr Val Gln Phe
200 205 210

Asp Lys Ala Ser Lys Ala Ser Ala Ser Ser Ser His His Ser Gln
215 220 225

Ser Ser Ser Gln Asn Ser Asp Pro Ser Arg Pro Leu Gln Arg Arg
230 235 240

Met Gln Thr His Val
245

<210> 215
<211> 1567
<212> DNA
<213> Homo Sapien

<400> 215
cagccttcct cccccagcct gagtgactac tctattcctt ggtccctgct 50

attgtcgggg acgattgcat gggctacgcc aggaaagtag gctgggtgac 100

cgcaggcctg gtgattgggg ctggcgcctg ctattgcatt tatagactga 150

ctaggggaag aaaacagaac aaggaaaaaa tggctgaggg tggatctggg 200

gatgtggatg atgctgggga ctgttctggg gccaggtata atgactggtc 250

tgatgatgat gatgacagca atgagagcaa gagtatagta tggtacccac 300

cttgggctcg gattgggact gaagctggaa ccagagctag ggccagggca 350

agggccaggg ctacccgggc acgtcgggct gtccagaaac gggcttcccc 400

caattcagat gataccgttt tgtcccctca agagctacaa aaggttcttt 450

gcttggttga gatgtctgaa aagccttata ttcttgaagc agctttaatt 500

gctctgggta acaatgctgc ttatgcattt aacagagata ttattcgtga 550

tctgggtggt ctcccaattg tcgcaaagat tctcaatact cgggatccca 600

tagttaagga aaaggcttta attgtcctga ataacttgag tgtgaatgct 650

gaaaatcagc gcaggcttaa agtatacatg aatcaagtgt gtgatgacac 700

aatcacttct cgcttgaact catctgtgca gcttgctgga ctgagattgc 750

ttacaaatat gactgttact aatgagtatc agcacatgct tgctaattcc 800

atttctgact tttttcgttt attttcagcg ggaaatgaag aaaccaaact 850

tcaggttctg aaactccttt tgaatttggc tgaaaatcca gccatgacta 900

gggaactgct cagggcccaa gtaccatctt cactgggctc cctctttaat 950

aagaaggaga acaaagaagt tattcttaaa cttctggtca tatttgagaa 1000

cataaatgat aatttcaaat gggaagaaaa tgaacctact cagaatcaat 1050

tcggtgaagg ttcacttttt ttctttttaa aagaatttca agtgtgtgct 1100

gataaggttc tgggaataga aagtcaccat gatttttttgg tgaaagtaaa 1150

agttggaaaa ttcatggcca aacttgctga acatatgttc ccaaagagcc 1200

aggaataaca ccttgatttt gtaatttaga agcaacacac attgtaaact 1250

attcattttc tccaccttgt ttatatggta aaggaatcct ttcagctgcc 1300

agttttgaat aatgaatatc atattgtatc atcaatgctg atatttaact 1350

gagttggtct ttaggtttaa gatggataaa tgaatatcac tacttgttct 1400

gaaaacatgt ttgttgcttt ttatctcgct gcctagattg aaatattttg 1450

ctatttcttc tgcataagtg acagtgaacc aattcatcat gagtaagctc 1500

ccttctgtca ttttcattga tttaatttgt gtatcatcaa taaaattgta 1550

tgttaatgct ggaaaga 1567

<210> 216
<211> 379
<212> PRT
<213> Homo Sapien

<400> 216

```
Met Gly Tyr Ala Arg Lys Val Gly Trp Val Thr Ala Gly Leu Val
 1               5                  10                  15

Ile Gly Ala Gly Ala Cys Tyr Cys Ile Tyr Arg Leu Thr Arg Gly
                20                  25                  30

Arg Lys Gln Asn Lys Glu Lys Met Ala Glu Gly Gly Ser Gly Asp
                35                  40                  45

Val Asp Asp Ala Gly Asp Cys Ser Gly Ala Arg Tyr Asn Asp Trp
                50                  55                  60

Ser Asp Asp Asp Asp Asp Ser Asn Glu Ser Lys Ser Ile Val Trp
                65                  70                  75

Tyr Pro Pro Trp Ala Arg Ile Gly Thr Glu Ala Gly Thr Arg Ala
                80                  85                  90

Arg Ala Arg Ala Arg Ala Arg Ala Thr Arg Ala Arg Arg Ala Val
                95                 100                 105

Gln Lys Arg Ala Ser Pro Asn Ser Asp Asp Thr Val Leu Ser Pro
               110                 115                 120

Gln Glu Leu Gln Lys Val Leu Cys Leu Val Glu Met Ser Glu Lys
               125                 130                 135
```

Pro Tyr Ile Leu Glu Ala Ala Leu Ile Ala Leu Gly Asn Asn Ala
140 145 150

Ala Tyr Ala Phe Asn Arg Asp Ile Ile Arg Asp Leu Gly Gly Leu
155 160 165

Pro Ile Val Ala Lys Ile Leu Asn Thr Arg Asp Pro Ile Val Lys
170 175 180

Glu Lys Ala Leu Ile Val Leu Asn Asn Leu Ser Val Asn Ala Glu
185 190 195

Asn Gln Arg Arg Leu Lys Val Tyr Met Asn Gln Val Cys Asp Asp
200 205 210

Thr Ile Thr Ser Arg Leu Asn Ser Ser Val Gln Leu Ala Gly Leu
215 220 225

Arg Leu Leu Thr Asn Met Thr Val Thr Asn Glu Tyr Gln His Met
230 235 240

Leu Ala Asn Ser Ile Ser Asp Phe Phe Arg Leu Phe Ser Ala Gly
245 250 255

Asn Glu Glu Thr Lys Leu Gln Val Leu Lys Leu Leu Leu Asn Leu
260 265 270

Ala Glu Asn Pro Ala Met Thr Arg Glu Leu Leu Arg Ala Gln Val
275 280 285

Pro Ser Ser Leu Gly Ser Leu Phe Asn Lys Lys Glu Asn Lys Glu
290 295 300

Val Ile Leu Lys Leu Leu Val Ile Phe Glu Asn Ile Asn Asp Asn
305 310 315

Phe Lys Trp Glu Glu Asn Glu Pro Thr Gln Asn Gln Phe Gly Glu
320 325 330

Gly Ser Leu Phe Phe Phe Leu Lys Glu Phe Gln Val Cys Ala Asp
335 340 345

Lys Val Leu Gly Ile Glu Ser His His Asp Phe Leu Val Lys Val
350 355 360

Lys Val Gly Lys Phe Met Ala Lys Leu Ala Glu His Met Phe Pro
365 370 375

Lys Ser Gln Glu

<210> 217
<211> 1633
<212> DNA
<213> Homo Sapien

<400> 217
gagacacaaa ggcaggcggg atgcgggagc aggcaaaggg aaagcgaaag 50

ccgcgcgccc ggccggtgac tgggtgaagg cgccgcgcag ctttcccgac 100

gccggctgta cccggacctc ctggtcgagc ctggcgcgcc gcagccatgg 150

ccatcgctca actggccacg gagtacgtgt tctcggattt cttgctgaag 200

gagcccacgg agcccaagtt caaggggctg cgactggagc tggctgtgga 250

caagatggtc acgtgcattg cggtggggct gcccctgctg ctcatctcgc 300

tggccttcgc gcaggagatc tcgattggta cacagataag ctgtttctct 350

ccaagttctt tctcctggcg tcaggctgcc tttgtggatt catattgctg 400

ggcggctgtt cagcagaaga actcactgca gagcgagtct ggaaacctcc 450

cactgtggct gcataagttt ttcccctaca tcctgctgct ctttgcgatc 500

ctcctgtacc tgcccccgct gttctggcgt ttcgcagctg ctcctcatat 550

ttgctcagac ttgaagttta tcatggaaga acttgacaaa gtttacaacc 600

gtgcaattaa ggctgcaaag agtgcgcgtg accttgacat gagagatgga 650

gcctgctcag ttccaggtgt taccgagaac ttagggcaaa gtttgtggga 700

ggtatctgaa agccacttca agtacccaat tgtggagcag tacttgaaga 750

caaagaaaaa ttctaataat ttaatcatca agtacattag ctgccgcctg 800

ctgacactca tcattatact gttagcgtgt atctacctgg gctattactt 850

cagcctctcc tcactctcag acgagtttgt gtgcagcatc aaatcaggga 900

tcctgagaaa cgacagcacc gtgcccgatc agtttcagtg caaactcatt 950

gccgtgggca tcttccagtt gctcagtgtc attaaccttg tggtttatgt 1000

cctgctggct cccgtggttg tctacacgct gtttgttcca ttccgacaga 1050

agacagatgt tctcaaagtg tacgaaatcc tccccacttt tgatgttctg 1100

catttcaaat ctgaagggta caacgatttg agcctctaca atctcttctt 1150

ggaggaaaat ataagtgagg tcaagtcata caagtgtctt aaggtactgg 1200

agaatattaa gagcagtggt caggggatcg acccaatgct actcctgaca 1250

aaccttggca tgatcaagat ggatgttgtt gatggcaaaa ctcccatgtc 1300

tgcagagatg agagaggagc aggggaacca gacggcagag ctccaaggta 1350

tgaacataga cagtgaaact aaagcaaata atggagagaa gaatgcccga 1400

cagagacttc tggattcttc ttgctgatga tttttttcc ttgagctgta 1450

aatctgtgac ttctgcgaca tgggatttaa tttggctaaa gcacccctgt 1500

tggtttcaca gctggtttgc aataaatggt tcttggtgga aaaaaaaaaa 1550

aaaaaaaaaa aaaaaaaaaa aaaaaaaaaa aaaaaaaaaa aaaaaaaaaa 1600

aaaaaaaaaa aaaaaaaaaa aaaaaaaaaa aaa 1633

<210> 218
<211> 426
<212> PRT
<213> Homo Sapien

<400> 218

```
Met Ala Ile Ala Gln Leu Ala Thr Glu Tyr Val Phe Ser Asp Phe
1               5                   10                  15

Leu Leu Lys Glu Pro Thr Glu Pro Lys Phe Lys Gly Leu Arg Leu
                20                  25                  30

Glu Leu Ala Val Asp Lys Met Val Thr Cys Ile Ala Val Gly Leu
                35                  40                  45

Pro Leu Leu Leu Ile Ser Leu Ala Phe Ala Gln Glu Ile Ser Ile
                50                  55                  60

Gly Thr Gln Ile Ser Cys Phe Ser Pro Ser Ser Phe Ser Trp Arg
                65                  70                  75

Gln Ala Ala Phe Val Asp Ser Tyr Cys Trp Ala Ala Val Gln Gln
                80                  85                  90

Lys Asn Ser Leu Gln Ser Glu Ser Gly Asn Leu Pro Leu Trp Leu
                95                  100                 105

His Lys Phe Phe Pro Tyr Ile Leu Leu Leu Phe Ala Ile Leu Leu
                110                 115                 120

Tyr Leu Pro Pro Leu Phe Trp Arg Phe Ala Ala Ala Pro His Ile
                125                 130                 135

Cys Ser Asp Leu Lys Phe Ile Met Glu Glu Leu Asp Lys Val Tyr
                140                 145                 150

Asn Arg Ala Ile Lys Ala Ala Lys Ser Ala Arg Asp Leu Asp Met
                155                 160                 165

Arg Asp Gly Ala Cys Ser Val Pro Gly Val Thr Glu Asn Leu Gly
                170                 175                 180

Gln Ser Leu Trp Glu Val Ser Glu Ser His Phe Lys Tyr Pro Ile
                185                 190                 195

Val Glu Gln Tyr Leu Lys Thr Lys Lys Asn Ser Asn Asn Leu Ile
                200                 205                 210

Ile Lys Tyr Ile Ser Cys Arg Leu Leu Thr Leu Ile Ile Ile Leu
                215                 220                 225

Leu Ala Cys Ile Tyr Leu Gly Tyr Tyr Phe Ser Leu Ser Ser Leu
                230                 235                 240

Ser Asp Glu Phe Val Cys Ser Ile Lys Ser Gly Ile Leu Arg Asn
                245                 250                 255

Asp Ser Thr Val Pro Asp Gln Phe Gln Cys Lys Leu Ile Ala Val
                260                 265                 270

Gly Ile Phe Gln Leu Leu Ser Val Ile Asn Leu Val Val Tyr Val
                275                 280                 285

Leu Leu Ala Pro Val Val Val Tyr Thr Leu Phe Val Pro Phe Arg
                290                 295                 300

Gln Lys Thr Asp Val Leu Lys Val Tyr Glu Ile Leu Pro Thr Phe
                305                 310                 315
```

Asp Val Leu His Phe Lys Ser Glu Gly Tyr Asn Asp Leu Ser Leu
320 325 330

Tyr Asn Leu Phe Leu Glu Glu Asn Ile Ser Glu Val Lys Ser Tyr
335 340 345

Lys Cys Leu Lys Val Leu Glu Asn Ile Lys Ser Ser Gly Gln Gly
350 355 360

Ile Asp Pro Met Leu Leu Leu Thr Asn Leu Gly Met Ile Lys Met
365 370 375

Asp Val Val Asp Gly Lys Thr Pro Met Ser Ala Glu Met Arg Glu
380 385 390

Glu Gln Gly Asn Gln Thr Ala Glu Leu Gln Gly Met Asn Ile Asp
395 400 405

Ser Glu Thr Lys Ala Asn Asn Gly Glu Lys Asn Ala Arg Gln Arg
410 415 420

Leu Leu Asp Ser Ser Cys
425

<210> 219
<211> 1076
<212> PRT
<213> Homo Sapien

<400> 219
Cys Thr Gly Thr Gly Ala Gly Thr Gly Ala Cys Ala Cys Ala Cys
1 5 10 15

Gly Cys Thr Gly Ala Gly Thr Gly Gly Gly Gly Thr Gly Ala Ala
20 25 30

Gly Gly Gly Ala Ala Ala Thr Gly Cys Thr Gly Gly Thr Gly Ala
35 40 45

Ala Thr Thr Thr Cys Ala Thr Thr Thr Thr Gly Ala Gly Gly Thr
50 55 60

Gly Thr Gly Gly Gly Thr Thr Gly Cys Thr Gly Thr Thr Ala Gly
65 70 75

Thr Cys Ala Cys Thr Cys Thr Gly Thr Cys Thr Cys Thr Thr Gly
80 85 90

Cys Cys Ala Thr Thr Gly Cys Cys Ala Ala Gly Cys Ala Cys Ala
95 100 105

Ala Gly Cys Ala Ala Thr Cys Thr Thr Cys Cys Thr Thr Cys Ala
110 115 120

Cys Cys Ala Ala Ala Ala Gly Thr Thr Gly Thr Thr Ala Cys Cys
125 130 135

Cys Ala Ala Gly Gly Gly Gly Ala Ala Cys Ala Thr Thr Gly Thr
140 145 150

Cys Cys Cys Ala Ala Gly Cys Thr Gly Thr Thr Gly Ala Cys Gly
155 160 165

Cys Thr Cys Thr Cys Thr Ala Thr Ala Thr Cys Ala Ala Ala Gly

170 175 180

Cys Ala Gly Cys Ala Thr Gly Gly Cys Thr Cys Ala Ala Ala Gly
185 190 195

Cys Ala Ala Cys Gly Ala Thr Thr Cys Cys Ala Gly Ala Ala Gly
200 205 210

Ala Cys Cys Gly Cys Ala Thr Ala Ala Ala Ala Ala Ala Thr Ala
215 220 225

Thr Ala Cys Gly Ala Thr Thr Ala Thr Thr Ala Ala Ala Ala Ala
230 235 240

Ala Gly Ala Ala Ala Ala Cys Ala Ala Ala Ala Ala Ala Gly Cys
245 250 255

Ala Gly Thr Thr Thr Ala Thr Gly Ala Ala Ala Ala Ala Cys Thr
260 265 270

Gly Thr Cys Ala Ala Thr Thr Thr Cys Ala Ala Gly Ala Ala Cys
275 280 285

Ala Gly Cys Thr Thr Cys Thr Gly Thr Cys Cys Thr Thr Cys Thr
290 295 300

Thr Cys Ala Thr Gly Gly Ala Ala Gly Ala Cys Gly Thr Thr Thr
305 310 315

Thr Thr Gly Gly Thr Cys Ala Ala Cys Thr Gly Cys Ala Ala Thr
320 325 330

Thr Gly Cys Ala Ala Gly Gly Cys Thr Gly Cys Ala Ala Gly Ala
335 340 345

Ala Ala Ala Thr Ala Cys Gly Cys Thr Thr Thr Gly Thr Gly Gly
350 355 360

Ala Gly Gly Ala Cys Thr Thr Thr Cys Ala Thr Ala Gly Cys Cys
365 370 375

Thr Thr Ala Gly Gly Cys Ala Gly Ala Ala Ala Thr Thr Gly Ala
380 385 390

Gly Cys Cys Ala Cys Thr Gly Thr Ala Thr Thr Thr Cys Cys Thr
395 400 405

Gly Thr Gly Cys Thr Thr Cys Ala Thr Cys Ala Gly Cys Thr Ala
410 415 420

Gly Ala Gly Ala Gly Ala Thr Gly Ala Ala Ala Thr Cys Cys Ala
425 430 435

Thr Thr Ala Cys Cys Ala Gly Gly Ala Thr Gly Ala Ala Ala Ala
440 445 450

Gly Ala Ala Thr Ala Thr Thr Thr Thr Ala Thr Ala Gly Gly Ala
455 460 465

Thr Thr Gly Gly Ala Ala Ala Cys Ala Ala Ala Gly Gly Ala Ala
470 475 480

Thr Cys Thr Ala Cys Ala Ala Ala Gly Cys Cys Ala Thr Cys Ala
485 490 495

```
Gly Thr Gly Ala Ala Cys Thr Gly Gly Ala Thr Ala Thr Thr Cys
                500                 505                 510

Thr Thr Cys Thr Thr Thr Cys Cys Thr Gly Gly Ala Thr Thr Ala
                515                 520                 525

Ala Ala Ala Ala Ala Thr Thr Ala Thr Thr Gly Gly Ala Ala Ala
                530                 535                 540

Gly Cys Ala Gly Thr Cys Ala Gly Thr Ala Ala Ala Cys Cys Ala
                545                 550                 555

Ala Ala Gly Cys Cys Ala Ala Gly Thr Ala Cys Ala Thr Thr Gly
                560                 565                 570

Ala Thr Thr Thr Thr Ala Cys Ala Gly Thr Thr Ala Thr Thr Thr
                575                 580                 585

Thr Gly Ala Ala Ala Thr Ala Cys Ala Ala Thr Ala Ala Gly Ala
                590                 595                 600

Ala Cys Thr Gly Cys Thr Ala Gly Ala Ala Ala Thr Ala Thr Gly
                605                 610                 615

Thr Thr Thr Ala Thr Ala Ala Cys Ala Gly Thr Cys Thr Ala Thr
                620                 625                 630

Thr Thr Cys Thr Thr Thr Thr Ala Ala Ala Ala Ala Cys Thr Thr
                635                 640                 645

Thr Thr Thr Ala Ala Cys Ala Thr Ala Ala Thr Ala Cys Thr Gly
                650                 655                 660

Ala Cys Gly Gly Cys Ala Thr Gly Thr Thr Ala Gly Gly Thr Gly
                665                 670                 675

Ala Thr Thr Cys Ala Gly Ala Ala Thr Ala Gly Ala Cys Ala Ala
                680                 685                 690

Gly Ala Ala Gly Gly Ala Thr Thr Thr Ala Gly Thr Ala Ala Ala
                695                 700                 705

Thr Thr Ala Ala Cys Gly Thr Thr Thr Thr Gly Gly Ala Thr Ala
                710                 715                 720

Thr Ala Ala Gly Thr Thr Gly Thr Cys Ala Cys Thr Ala Ala Thr
                725                 730                 735

Thr Thr Gly Cys Ala Cys Ala Thr Thr Thr Thr Cys Thr Gly Thr
                740                 745                 750

Gly Thr Thr Thr Thr Cys Ala Ala Ala Thr Ala Ala Thr Gly Thr
                755                 760                 765

Thr Thr Cys Cys Ala Thr Thr Cys Thr Gly Ala Ala Cys Ala Thr
                770                 775                 780

Gly Thr Thr Thr Thr Gly Thr Cys Ala Thr Thr Cys Ala Cys Ala
                785                 790                 795

Ala Gly Thr Ala Cys Ala Thr Thr Gly Thr Gly Thr Cys Ala Ala
                800                 805                 810
```

Cys Thr Thr Ala Ala Thr Thr Thr Ala Ala Ala Gly Thr Ala Thr
815 820 825

Gly Thr Ala Ala Cys Cys Thr Gly Ala Ala Thr Thr Ala Ala Cys
830 835 840

Thr Cys Gly Thr Gly Thr Ala Ala Thr Ala Thr Thr Thr Gly Thr
845 850 855

Gly Thr Gly Thr Gly Gly Ala Gly Thr Gly Gly Gly Ala Thr Gly
860 865 870

Thr Gly Gly Gly Gly Gly Gly Thr Gly Gly Ala Gly Gly Gly Gly
875 880 885

Gly Ala Ala Thr Gly Ala Cys Ala Gly Ala Thr Thr Thr Cys Thr
890 895 900

Gly Gly Ala Ala Thr Gly Cys Ala Ala Thr Gly Thr Ala Ala Thr
905 910 915

Gly Thr Thr Ala Cys Thr Gly Ala Gly Ala Cys Thr Thr Ala Ala
920 925 930

Ala Thr Ala Gly Ala Thr Gly Thr Thr Ala Thr Gly Thr Ala Thr
935 940 945

Ala Thr Gly Ala Thr Thr Gly Thr Cys Thr Gly Thr Thr Thr Ala
950 955 960

Ala Gly Thr Gly Thr Thr Thr Gly Ala Ala Ala Ala Thr Thr Gly
965 970 975

Thr Thr Ala Ala Thr Thr Ala Thr Gly Cys Cys Cys Ala Gly Thr
980 985 990

Gly Thr Gly Ala Ala Cys Thr Thr Ala Gly Thr Ala Cys Thr Thr
995 1000 1005

Ala Ala Cys Ala Cys Ala Thr Thr Thr Thr Gly Ala Thr Thr Thr
1010 1015 1020

Thr Ala Ala Thr Thr Ala Ala Ala Thr Ala Ala Ala Thr Thr Gly
1025 1030 1035

Gly Gly Thr Thr Thr Cys Cys Thr Thr Cys Thr Cys Ala Ala Ala
1040 1045 1050

Ala Ala Ala Ala Ala Ala Ala Ala Ala Ala Ala Ala Ala Ala Ala
1055 1060 1065

Ala Ala Ala Ala Ala Ala Ala Ala Ala Ala Ala
1070 1075

<210> 220
<211> 171
<212> PRT
<213> Homo Sapien

<400> 220
Met Leu Val Asn Phe Ile Leu Arg Cys Gly Leu Leu Leu Val Thr
1 5 10 15

Leu Ser Leu Ala Ile Ala Lys His Lys Gln Ser Ser Phe Thr Lys

20 25 30

Ser Cys Tyr Pro Arg Gly Thr Leu Ser Gln Ala Val Asp Ala Leu
35 40 45

Tyr Ile Lys Ala Ala Trp Leu Lys Ala Thr Ile Pro Glu Asp Arg
50 55 60

Ile Lys Asn Ile Arg Leu Leu Lys Lys Lys Thr Lys Lys Gln Phe
65 70 75

Met Lys Asn Cys Gln Phe Gln Glu Gln Leu Leu Ser Phe Phe Met
80 85 90

Glu Asp Val Phe Gly Gln Leu Gln Leu Gln Gly Cys Lys Lys Ile
95 100 105

Arg Phe Val Glu Asp Phe His Ser Leu Arg Gln Lys Leu Ser His
110 115 120

Cys Ile Ser Cys Ala Ser Ser Ala Arg Glu Met Lys Ser Ile Thr
125 130 135

Arg Met Lys Arg Ile Phe Tyr Arg Ile Gly Asn Lys Gly Ile Tyr
140 145 150

Lys Ala Ile Ser Glu Leu Asp Ile Leu Leu Ser Trp Ile Lys Lys
155 160 165

Leu Leu Glu Ser Ser Gln
170

<210> 221
<211> 1129
<212> DNA
<213> Homo Sapien

<400> 221
gaccacggcc ctgcgcccca gccaggcctg aggacatgag gcggccggcg 50

gcggtgccgc tcctgctgct gctgtgtttt gggtctcaga gggccaaggc 100

agcaacagcc tgtggtcgcc ccaggatgct gaaccgaatg gtgggcgggc 150

aggacacgca ggagggcgag tggccctggc aagtcagcat ccagcgcaac 200

ggaagccact tctgcggggg cagcctcatc gcggagcagt gggtcctgac 250

ggctgcgcac tgcttccgca acacctctga gacgtccctg taccaggtcc 300

tgctgggggc aaggcagcta gtgcagccgg gaccacacgc tatgtatgcc 350

cgggtgaggc aggtggagag caacccccctg taccagggca cggcctccag 400

cgctgacgtg gccctggtgg agctggaggc accagtgccc ttcaccaatt 450

acatcctccc cgtgtgcctg cctgacccct cggtgatctt tgagacgggc 500

atgaactgct gggtcactgg ctggggcagc cccagtgagg aagacctcct 550

gcccgaaccg cggatcctgc agaaactcgc tgtgcccatc atcgacacac 600

ccaagtgcaa cctgctctac agcaaagaca ccgagtttgg ctaccaaccc 650

aaaaccatca agaatgacat gctgtgcgcc ggcttcgagg agggcaagaa 700

ggatgcctgc aagggcgact cgggcggccc cctggtgtgc ctcgtgggtc 750

agtcgtggct gcaggcgggg gtgatcagct ggggtgaggg ctgtgcccgc 800

cagaaccgcc caggtgtcta catccgtgtc accgcccacc acaactggat 850

ccatcggatc atccccaaac tgcagttcca gccagcgagg ttgggcggcc 900

agaagtgaga cccccggggc caggagcccc ttgagcagag ctctgcaccc 950

agcctgcccg cccacaccat cctgctggtc ctcccagcgc tgctgttgca 1000

cctgtgagcc ccaccagact catttgtaaa tagcgctcct tcctcccctc 1050

tcaaataccc ttattttatt tatgtttctc ccaataaaaa cccagcctgt 1100

gtgccagctg aaaaaaaaaa aaaaaaaaa 1129

<210> 222
<211> 290
<212> PRT
<213> Homo Sapien

<400> 222
Met Arg Arg Pro Ala Ala Val Pro Leu Leu Leu Leu Leu Cys Phe
1 5 10 15

Gly Ser Gln Arg Ala Lys Ala Ala Thr Ala Cys Gly Arg Pro Arg
20 25 30

Met Leu Asn Arg Met Val Gly Gly Gln Asp Thr Gln Glu Gly Glu
35 40 45

Trp Pro Trp Gln Val Ser Ile Gln Arg Asn Gly Ser His Phe Cys
50 55 60

Gly Gly Ser Leu Ile Ala Glu Gln Trp Val Leu Thr Ala Ala His
65 70 75

Cys Phe Arg Asn Thr Ser Glu Thr Ser Leu Tyr Gln Val Leu Leu
80 85 90

Gly Ala Arg Gln Leu Val Gln Pro Gly Pro His Ala Met Tyr Ala
95 100 105

Arg Val Arg Gln Val Glu Ser Asn Pro Leu Tyr Gln Gly Thr Ala
110 115 120

Ser Ser Ala Asp Val Ala Leu Val Glu Leu Glu Ala Pro Val Pro
125 130 135

Phe Thr Asn Tyr Ile Leu Pro Val Cys Leu Pro Asp Pro Ser Val
140 145 150

Ile Phe Glu Thr Gly Met Asn Cys Trp Val Thr Gly Trp Gly Ser
155 160 165

Pro Ser Glu Glu Asp Leu Leu Pro Glu Pro Arg Ile Leu Gln Lys
170 175 180

Leu Ala Val Pro Ile Ile Asp Thr Pro Lys Cys Asn Leu Leu Tyr
185 190 195

Ser Lys Asp Thr Glu Phe Gly Tyr Gln Pro Lys Thr Ile Lys Asn
200 205 210

Asp Met Leu Cys Ala Gly Phe Glu Glu Gly Lys Lys Asp Ala Cys
215 220 225

Lys Gly Asp Ser Gly Gly Pro Leu Val Cys Leu Val Gly Gln Ser
230 235 240

Trp Leu Gln Ala Gly Val Ile Ser Trp Gly Glu Gly Cys Ala Arg
245 250 255

Gln Asn Arg Pro Gly Val Tyr Ile Arg Val Thr Ala His His Asn
260 265 270

Trp Ile His Arg Ile Ile Pro Lys Leu Gln Phe Gln Pro Ala Arg
275 280 285

Leu Gly Gly Gln Lys
290

<210> 223
<211> 1661
<212> DNA
<213> Homo Sapien

<400> 223
caagatgtgg acagctcttg tgctcatttg gattttctcc ttgtccttat 50

ctgaaagcca tgcggcatcc aacgatccac gcaactttgt ccctaacaaa 100

atgtggaagg gattagtcaa gaggaatgca tctgtggaaa cagttgataa 150

taaaacgtct gaggatgtaa ccatggcagc agcttctcct gtcacattga 200

ccaaagggac ttcggcagcc cacctcaact ctatggaagt cacaacagag 250

gacacaagca ggacagatgt gagtgaacca gcaacttcag gagttgcagc 300

tgatggtgtg acctccattg ctcccacggc tgtggcctcc agtacgactg 350

cggcctccat tacgactgcg gcctccagta tgactgtggc ctccagtgct 400

cccacgactg cagcctccag tacaactgtg gcctccattg ctcccacgac 450

tgcagcctcc agtatgactg cggcctccag cactcccatg acacttgcac 500

tccccgcgcc cacgtccact tccacagggc ggaccccgtc cactaccgcc 550

actgggcatc catctctcag cacagccctc gcacaagtgc caaagagcag 600

cgcgttgcca agaacagcaa ccctggccac attggccaca cgtgctcaga 650

ctgtagcgac cacagcaaac acaagcagcc ccatgagcac tcgtccaagt 700

ccttccaagc acatgcccag tgacaccgcg gcaagccctg taccccctat 750

gcgtccccaa gcacaaggtc ccattagcca ggtgtcagtg gaccagcctg 800

tggttaacac aacaaataaa tccacaccca tgccctcaaa cacaacccca 850

gagcccgccc ccaccccac agtggtgacc accaccaagg cacaagccag 900

ggagccaact gccagcccag tgccagtacc tcacaccagc ccaatccctg 950

agatggaggc catgtccccc acgacacagc caagccccat gccatatacc 1000

cagagggccg ctgggccagg cacatcccag gcaccggagc aggtagagac 1050

tgaagccaca ccaggtactg attccactgg gccaacaccc aggagctcag 1100

ggggcactaa gatgccagcc acggactcgt gccagcccag cacccaaggc 1150

cagtacatgg tggtcaccac tgagcccctc acccaggccg tggtagacaa 1200

aactctcctt ctggtggtgc tgttactcgg ggtgaccctt ttcatcacag 1250

tcttggtttt gtttgccctg caggcctatg agagctacaa gaagaaggac 1300

tacacccagg tggactactt aatcaacggg atgtatgcgg actcagaaat 1350

gtgagggggg cgggggcctg gcgggaggcc tggccccttc ctcgtccttt 1400

ccttttgcct ttgagaccaa accaagtgct tccaaattct tttggtgcaa 1450

ttgaggagat atgccagatg cttaaacaca tttaattgct gtcagattaa 1500

ttccatgatc actaaagagt tgctgctttt ttcatattta tttttgtaaa 1550

tgattctgtg cccaggagca gctgggggtt ccacctcagg gtggggcggg 1600

caggaccccg tctccccagg tgtcggagcc tgacctgaat taaagtactg 1650

actgctcgcc a 1661

<210> 224
<211> 449
<212> PRT
<213> Homo Sapien

<400> 224
Met Trp Thr Ala Leu Val Leu Ile Trp Ile Phe Ser Leu Ser Leu
1               5                   10                  15

Ser Glu Ser His Ala Ala Ser Asn Asp Pro Arg Asn Phe Val Pro
                20                  25                  30

Asn Lys Met Trp Lys Gly Leu Val Lys Arg Asn Ala Ser Val Glu
                35                  40                  45

Thr Val Asp Asn Lys Thr Ser Glu Asp Val Thr Met Ala Ala Ala
                50                  55                  60

Ser Pro Val Thr Leu Thr Lys Gly Thr Ser Ala Ala His Leu Asn
                65                  70                  75

Ser Met Glu Val Thr Thr Glu Asp Thr Ser Arg Thr Asp Val Ser
                80                  85                  90

Glu Pro Ala Thr Ser Gly Val Ala Ala Asp Gly Val Thr Ser Ile
                95                  100                 105

Ala Pro Thr Ala Val Ala Ser Ser Thr Thr Ala Ala Ser Ile Thr
                110                 115                 120

Thr Ala Ala Ser Ser Met Thr Val Ala Ser Ser Ala Pro Thr Thr
                125                 130                 135

Ala Ala Ser Ser Thr Thr Val Ala Ser Ile Ala Pro Thr Thr Ala
140 145 150

Ala Ser Ser Met Thr Ala Ala Ser Ser Thr Pro Met Thr Leu Ala
155 160 165

Leu Pro Ala Pro Thr Ser Thr Ser Thr Gly Arg Thr Pro Ser Thr
170 175 180

Thr Ala Thr Gly His Pro Ser Leu Ser Thr Ala Leu Ala Gln Val
185 190 195

Pro Lys Ser Ser Ala Leu Pro Arg Thr Ala Thr Leu Ala Thr Leu
200 205 210

Ala Thr Arg Ala Gln Thr Val Ala Thr Thr Ala Asn Thr Ser Ser
215 220 225

Pro Met Ser Thr Arg Pro Ser Pro Ser Lys His Met Pro Ser Asp
230 235 240

Thr Ala Ala Ser Pro Val Pro Pro Met Arg Pro Gln Ala Gln Gly
245 250 255

Pro Ile Ser Gln Val Ser Val Asp Gln Pro Val Val Asn Thr Thr
260 265 270

Asn Lys Ser Thr Pro Met Pro Ser Asn Thr Thr Pro Glu Pro Ala
275 280 285

Pro Thr Pro Thr Val Val Thr Thr Thr Lys Ala Gln Ala Arg Glu
290 295 300

Pro Thr Ala Ser Pro Val Pro Val Pro His Thr Ser Pro Ile Pro
305 310 315

Glu Met Glu Ala Met Ser Pro Thr Thr Gln Pro Ser Pro Met Pro
320 325 330

Tyr Thr Gln Arg Ala Ala Gly Pro Gly Thr Ser Gln Ala Pro Glu
335 340 345

Gln Val Glu Thr Glu Ala Thr Pro Gly Thr Asp Ser Thr Gly Pro
350 355 360

Thr Pro Arg Ser Ser Gly Gly Thr Lys Met Pro Ala Thr Asp Ser
365 370 375

Cys Gln Pro Ser Thr Gln Gly Gln Tyr Met Val Val Thr Thr Glu
380 385 390

Pro Leu Thr Gln Ala Val Val Asp Lys Thr Leu Leu Leu Val Val
395 400 405

Leu Leu Leu Gly Val Thr Leu Phe Ile Thr Val Leu Val Leu Phe
410 415 420

Ala Leu Gln Ala Tyr Glu Ser Tyr Lys Lys Lys Asp Tyr Thr Gln
425 430 435

Val Asp Tyr Leu Ile Asn Gly Met Tyr Ala Asp Ser Glu Met
440 445

<210> 225
<211> 1971
<212> DNA
<213> Homo Sapien

<400> 225
ggaaggcgct caaggtgcgc ggcccggggc gcgctactgg gggcgccctc 50

cgcggtgggc agcgcgccag ggatcggcct gggcagccgc ggggcgcgcg 100

aaggctgcgc tttccctacg gcccccctcg cttcctccgg cacggcggca 150

acggagattt cctctcgggg aaactacgcg gatcctttc ggggatcctc 200

gccccgcccc agttctccgc cccctcccct ttgctggggc gcctgggctg 250

gcccgcgcag gggaggaggc tctggcagcc tgggcaggga ggcggcgggg 300

ggccgcggag ccgctggcca tcgattctcc ccgccatgtg acgccgtcct 350

tagccctgcg acccccagcg cgtcccgggc ctgcgcctcc gccccgccgc 400

gcagcgcacg atgcttctgc cgggacgcgc acgccaaccg ccgacgcccc 450

agcccgtgca gcatcccggc ctccgccggc aggtagagcc gccggggcag 500

ctcctgcgcc tcttctactg cactgtcctg gtctgctcca aagagatctc 550

agcgctcacc gacttctctg gttacctaac caaactcctg caaaaccaca 600

ccacctatgc ctgtgatggg gactatttga atctacagtg ccctcggcat 650

tctacgataa gtgtccaatc ggcattttat gggcaagatt accaaatgtg 700

tagttcccag aagcctgcct cccagaggga agacagctta acctgtgtgg 750

cagccaccac cttccagaag gtgctggacg aatgccagaa ccagcgggcc 800

tgccacctcc tggtcaatag ccgtgttttt ggacctgacc tttgtccagg 850

aagcagtaaa tacctcctgg tctcctttaa atgccaacct aatgaattaa 900

aaaacaaaac cgtgtgtgaa gaccaggagc tgaaactgca ctgccatgaa 950

tccaagttcc tcaacatcta ctctgcgacc tacggcagga ggacccagga 1000

aagggacatc tgctcctcca aggcagagcg gctcccccct ttcgattgct 1050

tgtcttactc agctttgcaa gtcctatccc gaaggtgcta tgggaagcag 1100

agatgcaaaa tcatcgtcaa caatcaccat tttggaagcc cctgtttgcc 1150

aggcgtgaaa aaatacctca ctgtgaccta cgcatgtgtt cccaagaaca 1200

tactcacagc gattgatcca gccattgcta atctaaaacc ttctttgaag 1250

cagaaagatg gtgaatatgg tataaacttc gacccaagcg gatcgaaggt 1300

tctgaggaaa gatggaattc ttgttagcaa ctctctggca gcctttgctt 1350

acattagagc ccacccagag agagctgccc tgctgttcgt gtccagtgtc 1400

tgcatcggcc tggccctcac actgtgcgcc ctggtcatca gagagtcctg 1450

tgccaaggac ttccgcgact tgcagctggg gagggagcag ctggtgccag 1500

gaagtgacaa ggtcgaggag gacagcgagg atgaagaaga ggaggaggac 1550

ccctctgagt ctgatttccc aggggaactg tcggggttct gtaggacttc 1600

atatcctata tacagttcca tagaagctgc agagctcgca gaaaggattg 1650

agcgcaggga gcaaatcatt caggaaatat ggatgaacag tggtttggac 1700

acctcgctcc caagaaacat gggccagttc tactgaaaac cacatgcatc 1750

ttgatgcgat cgcactttct gaagaaggaa ggatcccaaa tgcccctcca 1800

gttctggttc acctgtacct tctatgaagg agaattcgtc atgtcattca 1850

acactcgtga ggccaggaag ctattaaagg gatgtttcaa gctgtttcta 1900

gcacattcca aaataaatga ggagggagga aaaaaaaaaa aaaaaaaaaa 1950

aaaaaaaaaa aaaaaaaaaa a 1971

<210> 226
<211> 441
<212> PRT
<213> Homo Sapien

<400> 226
Met Leu Leu Pro Gly Arg Ala Arg Gln Pro Pro Thr Pro Gln Pro
1 5 10 15

Val Gln His Pro Gly Leu Arg Arg Gln Val Glu Pro Pro Gly Gln
20 25 30

Leu Leu Arg Leu Phe Tyr Cys Thr Val Leu Val Cys Ser Lys Glu
35 40 45

Ile Ser Ala Leu Thr Asp Phe Ser Gly Tyr Leu Thr Lys Leu Leu
50 55 60

Gln Asn His Thr Thr Tyr Ala Cys Asp Gly Asp Tyr Leu Asn Leu
65 70 75

Gln Cys Pro Arg His Ser Thr Ile Ser Val Gln Ser Ala Phe Tyr
80 85 90

Gly Gln Asp Tyr Gln Met Cys Ser Ser Gln Lys Pro Ala Ser Gln
95 100 105

Arg Glu Asp Ser Leu Thr Cys Val Ala Ala Thr Thr Phe Gln Lys
110 115 120

Val Leu Asp Glu Cys Gln Asn Gln Arg Ala Cys His Leu Leu Val
125 130 135

Asn Ser Arg Val Phe Gly Pro Asp Leu Cys Pro Gly Ser Ser Lys
140 145 150

Tyr Leu Leu Val Ser Phe Lys Cys Gln Pro Asn Glu Leu Lys Asn
155 160 165

Lys Thr Val Cys Glu Asp Gln Glu Leu Lys Leu His Cys His Glu
170 175 180

Ser Lys Phe Leu Asn Ile Tyr Ser Ala Thr Tyr Gly Arg Arg Thr
185 190 195

Gln Glu Arg Asp Ile Cys Ser Ser Lys Ala Glu Arg Leu Pro Pro
200 205 210

Phe Asp Cys Leu Ser Tyr Ser Ala Leu Gln Val Leu Ser Arg Arg
215 220 225

Cys Tyr Gly Lys Gln Arg Cys Lys Ile Ile Val Asn Asn His His
230 235 240

Phe Gly Ser Pro Cys Leu Pro Gly Val Lys Lys Tyr Leu Thr Val
245 250 255

Thr Tyr Ala Cys Val Pro Lys Asn Ile Leu Thr Ala Ile Asp Pro
260 265 270

Ala Ile Ala Asn Leu Lys Pro Ser Leu Lys Gln Lys Asp Gly Glu
275 280 285

Tyr Gly Ile Asn Phe Asp Pro Ser Gly Ser Lys Val Leu Arg Lys
290 295 300

Asp Gly Ile Leu Val Ser Asn Ser Leu Ala Ala Phe Ala Tyr Ile
305 310 315

Arg Ala His Pro Glu Arg Ala Ala Leu Leu Phe Val Ser Ser Val
320 325 330

Cys Ile Gly Leu Ala Leu Thr Leu Cys Ala Leu Val Ile Arg Glu
335 340 345

Ser Cys Ala Lys Asp Phe Arg Asp Leu Gln Leu Gly Arg Glu Gln
350 355 360

Leu Val Pro Gly Ser Asp Lys Val Glu Glu Asp Ser Glu Asp Glu
365 370 375

Glu Glu Glu Glu Asp Pro Ser Glu Ser Asp Phe Pro Gly Glu Leu
380 385 390

Ser Gly Phe Cys Arg Thr Ser Tyr Pro Ile Tyr Ser Ser Ile Glu
395 400 405

Ala Ala Glu Leu Ala Glu Arg Ile Glu Arg Arg Glu Gln Ile Ile
410 415 420

Gln Glu Ile Trp Met Asn Ser Gly Leu Asp Thr Ser Leu Pro Arg
425 430 435

Asn Met Gly Gln Phe Tyr
440

<210> 227
<211> 840
<212> DNA
<213> Homo Sapien

<400> 227
ggcacgaggt ggaagggctt ttacaaacag attgctggcc ccacccccca 50

gaatttctca tcaggagtgg gcaagaccaa tcatttgcat ttctgacaag 100

ttcccaggag ctgcagctgc tggccctgga accacacttt gagaaccact 150

gctttagacc aaacaccaaa ggaagatgca gccaccctcc tttacatgtc 200

acaacgctca gggtccatga gtacctcagg ctgtccagct gagctccacc 250

tgcagcagcc gagattcccg actcgctcca ccattggggg ctaggagtga 300

agcgtgtcac catggtcagc tcatggccag ccaggaaagc ctctctgctg 350

tgcgtctgtg cagttcttgt tcttccctgg aggactcttg gatcgcctgt 400

gatcttggcc aggagaccag gtgcctgggt cccttcctgg aaggggacaa 450

gttacacacc ccagccccat tttcccacca acttctacat gccttgggag 500

aaccttctac atgttggctg ccccttccc ctatttcagc agtgcccagt 550

cctgcttata aacctgaggc ctgctcccca taccttccct gtgcaagtgc 600

cagccgttat tccaggcagc ccaatgttgt tgaggccaga tggattcctg 650

gaagcagctg gcccatggat gtgagtcatc acagtattct agaaacagag 700

aagaggtctt aacctaatgc gcatagagaa attgttctca ttgtaaacat 750

acccctgtcc ttagctgatc taggtggaag cccagcttca tgtgctaggg 800

ggcatgataa tgataataaa ggaattgtat ctaggactaa 840

<210> 228
<211> 120
<212> PRT
<213> Homo Sapien

<400> 228
Met Val Ser Ser Trp Pro Ala Arg Lys Ala Ser Leu Leu Cys Val
1 5 10 15

Cys Ala Val Leu Val Leu Pro Trp Arg Thr Leu Gly Ser Pro Val
20 25 30

Ile Leu Ala Arg Arg Pro Gly Ala Trp Val Pro Ser Trp Lys Gly
35 40 45

Thr Ser Tyr Thr Pro Gln Pro His Phe Pro Thr Asn Phe Tyr Met
50 55 60

Pro Trp Glu Asn Leu Leu His Val Gly Cys Pro Leu Pro Leu Phe
65 70 75

Gln Gln Cys Pro Val Leu Leu Ile Asn Leu Arg Pro Ala Pro His
80 85 90

Thr Phe Pro Val Gln Val Pro Ala Val Ile Pro Gly Ser Pro Met
95 100 105

Leu Leu Arg Pro Asp Gly Phe Leu Glu Ala Ala Gly Pro Trp Met
110 115 120

<210> 229
<211> 2837
<212> DNA
<213> Homo Sapien

<400> 229
gggaagggat gcaaggaagc cctccggcgc tgcgctccga ggcgggagac 50
agcgtcccgc tgaaaatgtg tgtctgacat gcaagctcag tggggcagag 100
acccgtggat tgctgtgccc tgccctccgg acctggatca tgaaggtgtt 150
gggaagaagc ttcttctggg tgctgtttcc cgtccttccc tgggcggtgc 200
aggctgtgga gcacgaggag gtggcgcagc gtgtgatcaa actgcaccgc 250
gggcgagggg tggctgccat gcagagccgg cagtgggtcc gggacagctg 300
caggaagctc tcagggcttc tccgccagaa gaatgcagtt ctgaacaaac 350
tgaaaactgc aattggagca gtggagaaag acgtgggcct gtcggatgaa 400
gagaaactgt ttcaggtgca cacgtttgaa attttccaga aagagctgaa 450
tgaaagtgaa aattccgttt tccaagctgt ctacggactg cagagagccc 500
tgcaggggga ttacaaagat gtcgtgaaca tgaaggagag cagccggcag 550
cgcctggagg ccctgagaga ggctgcaata aaggaagaaa cagaatatat 600
ggaacttctg gcagcagaaa aacatcaagt tgaagccctt aaaaatatgc 650
aacatcaaaa ccaaagttta tccatgcttg acgagattct tgaagatgta 700
agaaaggcag cggatcgtct ggaggaagag atagaggaac atgcttttga 750
cgacaataaa tcagtcaagg gggtcaattt tgaggcagtt ctgagggtgg 800
aggaagaaga ggccaattct aagcaaaata taacaaaacg agaagtggag 850
gatgacttgg gtcttagcat gctgattgac tcccagaaca accagtatat 900
tttgaccaag cccagagatt caaccatccc acgtgcagat caccacttta 950
taaaggacat tgttaccata ggaatgctgt ccttgccttg tggctggcta 1000
tgtacagcca taggattgcc tacaatgttt ggttatatta tttgtggtgt 1050
acttctggga ccttcaggac taaatagtat taagtctatt gtgcaagtgg 1100
agacattagg agaatttggg gtgtttttta ctctttttct tgttggctta 1150
gaattttctc cagaaaagct aagaaaggtg tggaagattt ccttacaagg 1200
gccgtgttac atgacactgt taatgattgc atttggcttg ctgtgggggc 1250
atctcttgcg gatcaaaccc acgcagagcg tcttcatttc cacgtgtctg 1300
tccttgtcaa gcacacccct cgtgtccagg ttcctcatgg gcagtgctcg 1350
gggtgacaaa gaaggcgaca ttgactacag caccgtgctc ctcggcatgc 1400
tggtgacgca ggacgtgcag ctcgggctct tcatggccgt catgccgact 1450
ctcatacagg cgggcgccag tgcatcttct agcattgtcg tggaagttct 1500
ccgaatcctg gttttgattg gtcagattct tttttcacta gcggcggttt 1550

ttcttttatg tcttgttata aagaagtatc tcattggacc ctattatcgg 1600

aagctgcaca tggaaagcaa ggggaacaaa gaaatcctga tcttgggaat 1650

atctgccttt atcttcttaa tgttaacggt cacggagctg ctggacgtct 1700

ccatggagct gggctgtttc ctggctggag cgctcgtctc ctctcagggc 1750

cccgtggtca ccgaggagat cgccacctcc atcgaaccca tccgcgactt 1800

cctggccatc gttttcttcg cctccatagg gctccacgtg ttccccacgt 1850

ttgtggcgta cgagctcacg gtgctggtgt tcctcacctt gtcagtggtg 1900

gtgatgaagt ttctcctggc ggcgctggtc ctgtctctca ttctgccgag 1950

gagcagccag tacatcaagt ggatcgtctc tgcggggctt gcccaggtca 2000

gcgagttttc ctttgtcctg gggagccggg cgcgaagagc gggcgtcatc 2050

tctcgggagg tgtacctcct tatactgagt gtgaccacgc tcagcctctt 2100

gctcgccccg gtgctgtgga gagctgcaat cacgaggtgt gtgcccagac 2150

cggagagacg gtccagcctc tgatggctcg gagatgatgg accgtggaag 2200

ggaagcgtct gtggggagtg agcgcttaga tggccagcag ctgctccttc 2250

tgggaagctc gcaccttggc aacagaacag ccctctagca gagcgtcagt 2300

gcagtcgtgt tatcccggct tttacagaat attcttgtcc tattttagaa 2350

ttttccggag tagtttattt gcagtctgtt gattatgtgc agtagacccg 2400

ggacactgcg ttttaccgat caccttgaat gtggtgcctg gatgtgcctt 2450

ttttttttt ccctgaaatt attattaatt ttctattgtg agttcatcag 2500

ttcatagttt ttttagtaaa gaagcaaaat taaaaggctt ttaaaaatgt 2550

acaacttcag aattataatc tgttagtcaa atatttgtta ttaaacattt 2600

ctgtaatatg aagttgtaat cctggccgtg agcttggaag cttacttttg 2650

attcttaaag cctatgtttt ctaaaatgag acaaatacgg atgtctattt 2700

gccttttatt gtaactttta aatgaaataa tttcatgtca atttctatta 2750

gatatatcac ttaaaatatt tggttttaaa tcacaagaat atgtattctt 2800

taataaagat aatttatgat catggtaaaa aaaaaaa 2837

<210> 230
<211> 677
<212> PRT
<213> Homo Sapien

<400> 230

```
Met Lys Val Leu Gly Arg Ser Phe Phe Trp Val Leu Phe Pro Val
  1               5                  10                  15

Leu Pro Trp Ala Val Gln Ala Val Glu His Glu Glu Val Ala Gln
                 20                  25                  30
```

```
Arg Val Ile Lys Leu His Arg Gly Arg Gly Val Ala Ala Met Gln
                35                  40                  45

Ser Arg Gln Trp Val Arg Asp Ser Cys Arg Lys Leu Ser Gly Leu
                50                  55                  60

Leu Arg Gln Lys Asn Ala Val Leu Asn Lys Leu Lys Thr Ala Ile
                65                  70                  75

Gly Ala Val Glu Lys Asp Val Gly Leu Ser Asp Glu Glu Lys Leu
                80                  85                  90

Phe Gln Val His Thr Phe Glu Ile Phe Gln Lys Glu Leu Asn Glu
                95                 100                 105

Ser Glu Asn Ser Val Phe Gln Ala Val Tyr Gly Leu Gln Arg Ala
               110                 115                 120

Leu Gln Gly Asp Tyr Lys Asp Val Val Asn Met Lys Glu Ser Ser
               125                 130                 135

Arg Gln Arg Leu Glu Ala Leu Arg Glu Ala Ala Ile Lys Glu Glu
               140                 145                 150

Thr Glu Tyr Met Glu Leu Leu Ala Ala Glu Lys His Gln Val Glu
               155                 160                 165

Ala Leu Lys Asn Met Gln His Gln Asn Gln Ser Leu Ser Met Leu
               170                 175                 180

Asp Glu Ile Leu Glu Asp Val Arg Lys Ala Ala Asp Arg Leu Glu
               185                 190                 195

Glu Glu Ile Glu Glu His Ala Phe Asp Asp Asn Lys Ser Val Lys
               200                 205                 210

Gly Val Asn Phe Glu Ala Val Leu Arg Val Glu Glu Glu Glu Ala
               215                 220                 225

Asn Ser Lys Gln Asn Ile Thr Lys Arg Glu Val Glu Asp Asp Leu
               230                 235                 240

Gly Leu Ser Met Leu Ile Asp Ser Gln Asn Asn Gln Tyr Ile Leu
               245                 250                 255

Thr Lys Pro Arg Asp Ser Thr Ile Pro Arg Ala Asp His His Phe
               260                 265                 270

Ile Lys Asp Ile Val Thr Ile Gly Met Leu Ser Leu Pro Cys Gly
               275                 280                 285

Trp Leu Cys Thr Ala Ile Gly Leu Pro Thr Met Phe Gly Tyr Ile
               290                 295                 300

Ile Cys Gly Val Leu Leu Gly Pro Ser Gly Leu Asn Ser Ile Lys
               305                 310                 315

Ser Ile Val Gln Val Glu Thr Leu Gly Glu Phe Gly Val Phe Phe
               320                 325                 330

Thr Leu Phe Leu Val Gly Leu Glu Phe Ser Pro Glu Lys Leu Arg
               335                 340                 345

Lys Val Trp Lys Ile Ser Leu Gln Gly Pro Cys Tyr Met Thr Leu
```

350 355 360

Leu Met Ile Ala Phe Gly Leu Leu Trp Gly His Leu Leu Arg Ile
365 370 375

Lys Pro Thr Gln Ser Val Phe Ile Ser Thr Cys Leu Ser Leu Ser
380 385 390

Ser Thr Pro Leu Val Ser Arg Phe Leu Met Gly Ser Ala Arg Gly
395 400 405

Asp Lys Glu Gly Asp Ile Asp Tyr Ser Thr Val Leu Leu Gly Met
410 415 420

Leu Val Thr Gln Asp Val Gln Leu Gly Leu Phe Met Ala Val Met
425 430 435

Pro Thr Leu Ile Gln Ala Gly Ala Ser Ala Ser Ser Ser Ile Val
440 445 450

Val Glu Val Leu Arg Ile Leu Val Leu Ile Gly Gln Ile Leu Phe
455 460 465

Ser Leu Ala Ala Val Phe Leu Leu Cys Leu Val Ile Lys Lys Tyr
470 475 480

Leu Ile Gly Pro Tyr Tyr Arg Lys Leu His Met Glu Ser Lys Gly
485 490 495

Asn Lys Glu Ile Leu Ile Leu Gly Ile Ser Ala Phe Ile Phe Leu
500 505 510

Met Leu Thr Val Thr Glu Leu Leu Asp Val Ser Met Glu Leu Gly
515 520 525

Cys Phe Leu Ala Gly Ala Leu Val Ser Ser Gln Gly Pro Val Val
530 535 540

Thr Glu Glu Ile Ala Thr Ser Ile Glu Pro Ile Arg Asp Phe Leu
545 550 555

Ala Ile Val Phe Phe Ala Ser Ile Gly Leu His Val Phe Pro Thr
560 565 570

Phe Val Ala Tyr Glu Leu Thr Val Leu Val Phe Leu Thr Leu Ser
575 580 585

Val Val Val Met Lys Phe Leu Leu Ala Ala Leu Val Leu Ser Leu
590 595 600

Ile Leu Pro Arg Ser Ser Gln Tyr Ile Lys Trp Ile Val Ser Ala
605 610 615

Gly Leu Ala Gln Val Ser Glu Phe Ser Phe Val Leu Gly Ser Arg
620 625 630

Ala Arg Arg Ala Gly Val Ile Ser Arg Glu Val Tyr Leu Leu Ile
635 640 645

Leu Ser Val Thr Thr Leu Ser Leu Leu Leu Ala Pro Val Leu Trp
650 655 660

Arg Ala Ala Ile Thr Arg Cys Val Pro Arg Pro Glu Arg Arg Ser
665 670 675

Ser Leu

<210> 231
<211> 1058
<212> DNA
<213> Homo Sapien

<400> 231
gagaaaaaca acaggaagca gcttacaaac tcggtgaaca actgagggaa 50

ccaaaccaga gacgcgctga acagagagaa tcaggctcaa agcaagtgga 100

agtgggcaga gattccacca ggactggtgc aaggcgcaga gccagccaga 150

tttgagaaga aggcaaaaag atgctgggga gcagagctgt aatgctgctg 200

ttgctgctgc cctggacagc tcagggcaga gctgtgcctg gggcagcag 250

ccctgcctgg actcagtgcc agcagctttc acagaagctc tgcacactgg 300

cctggagtgc acatccacta gtgggacaca tggatctaag agaagaggga 350

gatgaagaga ctacaaatga tgttccccat atccagtgtg gagatggctg 400

tgacccccaa ggactcaggg acaacagtca gttctgcttg caaaggatcc 450

accagggtct gatttttat gagaagctgc taggatcgga tattttcaca 500

ggggagcctt ctctgctccc tgatagccct gtgggccagc ttcatgcctc 550

cctactgggc ctcagccaac tcctgcagcc tgagggtcac cactgggaga 600

ctcagcagat tccaagcctc agtcccagcc agccatggca gcgtctcctt 650

ctccgcttca aaatccttcg cagcctccag gcctttgtgg ctgtagccgc 700

ccgggtcttt gcccatggag cagcaaccct gagtccctaa aggcagcagc 750

tcaaggatgg cactcagatc tccatggccc agcaaggcca agataaatct 800

accaccccag gcacctgtga gccaacaggt taattagtcc attaatttta 850

gtgggacctg catatgttga aaattaccaa tactgactga catgtgatgc 900

tgacctatga taaggttgag tatttattag atgggaaggg aaatttgggg 950

attatttatc ctcctgggga cagtttgggg aggattattt attgtattta 1000

tattgaatta tgtacttttt tcaataaagt cttatttttg tggctaaaaa 1050

aaaaaaaa 1058

<210> 232
<211> 189
<212> PRT
<213> Homo Sapien

<400> 232
Met Leu Gly Ser Arg Ala Val Met Leu Leu Leu Leu Leu Pro Trp
1 5 10 15

Thr Ala Gln Gly Arg Ala Val Pro Gly Gly Ser Ser Pro Ala Trp

20 25 30

Thr Gln Cys Gln Gln Leu Ser Gln Lys Leu Cys Thr Leu Ala Trp
35 40 45

Ser Ala His Pro Leu Val Gly His Met Asp Leu Arg Glu Glu Gly
50 55 60

Asp Glu Glu Thr Thr Asn Asp Val Pro His Ile Gln Cys Gly Asp
65 70 75

Gly Cys Asp Pro Gln Gly Leu Arg Asp Asn Ser Gln Phe Cys Leu
80 85 90

Gln Arg Ile His Gln Gly Leu Ile Phe Tyr Glu Lys Leu Leu Gly
95 100 105

Ser Asp Ile Phe Thr Gly Glu Pro Ser Leu Leu Pro Asp Ser Pro
110 115 120

Val Gly Gln Leu His Ala Ser Leu Leu Gly Leu Ser Gln Leu Leu
125 130 135

Gln Pro Glu Gly His His Trp Glu Thr Gln Gln Ile Pro Ser Leu
140 145 150

Ser Pro Ser Gln Pro Trp Gln Arg Leu Leu Leu Arg Phe Lys Ile
155 160 165

Leu Arg Ser Leu Gln Ala Phe Val Ala Val Ala Ala Arg Val Phe
170 175 180

Ala His Gly Ala Ala Thr Leu Ser Pro
185

<210> 233
<211> 4333
<212> DNA
<213> Homo Sapien

<400> 233
cccacgcgtc cggccctgta accaagatac tgactgaaca tggctggcgg 50

actcaggctg gggtctgcag tgcagcatta atgggccgct gacatgaata 100

tggagtagtt ttctctagca aagagtaatg tgggccatgg agtcaggcca 150

cctcctctgg gctctgctgt tcatgcagtc cttgtggcct caactgactg 200

atggagccac tcgagtctac tacctgggca tccgggatgt gcagtggaac 250

tatgctccca agggaagaaa tgtcatcacg aaccagcctc tggacagtga 300

catagtggct tccagcttct taaagtctga caagaaccgg atagggggaa 350

cctacaagaa gaccatctat aaagaataca aggatgactc atacacagat 400

gaagtggccc agcctgcctg gttgggcttc ctggggccag tgttgcaggc 450

tgaagtgggg gatgtcattc ttattcacct gaagaatttt gccactcgtc 500

cctataccat ccaccctcat ggtgtcttct acgagaagga ctctgaaggt 550

tccctatacc cagatggctc ctctgggcca ctgaaagctg atgactctgt 600

tcccccgggg ggcagccata tctacaactg gaccattcca gaaggccatg 650
cacccaccga tgctgaccca gcgtgcctca cctggatcta ccattctcat 700
gtagatgctc cacgagacat tgcaactggc ctaattgggc ctctcatcac 750
ctgtaaaaga ggagccctgg atgggaactc ccctcctcaa cgccaggatg 800
tagaccatga tttcttcctc ctcttcagtg tggtagatga gaacctcagc 850
tggcatctca atgagaacat tgccacttac tgctcagatc ctgcttcagt 900
ggacaaagaa gatgagacat ttcaggagag caataggatg catgcaatca 950
atggctttgt ttttgggaat ttacctgagc tgaacatgtg tgcacagaaa 1000
cgtgtggcct ggcacttgtt tggcatgggc aatgaaattg atgtccacac 1050
agcatttttc catggacaga tgctgactac ccgtggacac cacactgatg 1100
tggctaacat ctttccagcc acctttgtga ctgctgagat ggtgccctgg 1150
gaacctggta cctggttaat tagctgccaa gtgaacagtc actttcgaga 1200
tggcatgcag gcactctaca aggtcaagtc ttgctccatg gcccctcctg 1250
tggacctgct cacaggcaaa gttcgacagt acttcattga ggcccatgag 1300
attcaatggg actatggccc gatggggcat gatgggagta ctgggaagaa 1350
tttgagagag ccaggcagta tctcagataa gtttttccag aagagctcca 1400
gccgaattgg gggcacttac tggaaagtgc gatatgaagc ctttcaagat 1450
gagacattcc aagagaagat gcatttggag gaagataggc atcttggaat 1500
cctggggcca gtgatccggg ctgaggtggg tgacaccatt caggtggtct 1550
tctacaaccg tgcctcccag ccattcagca tgcagcccca tggggtcttt 1600
tatgagaaag actatgaagg cactgtgtac aatgatggct catcttaccc 1650
tggcttggtt gccaagccct ttgagaaagt aacataccgc tggacagtcc 1700
cccctcatgc cggtcccact gctcaggatc ctgcttgtct cacttggatg 1750
tacttctctg ctgcagatcc cataagagac acaaattctg gcctggtggg 1800
cccgctgctg gtgtgcaggg ctggtgcctt gggtgcagat ggcaagcaga 1850
aaggggtgga taaagaattc tttcttctct tcactgtgtt ggatgagaac 1900
aagagctggt acagcaatgc caatcaagca gctgctatgt tggatttccg 1950
actgctttca gaggatattg agggcttcca agactccaat cggatgcatg 2000
ccattaatgg gtttctgttc tctaacctgc ccaggctgga catgtgcaag 2050
ggtgacacag tggcctggca cctgctcggc ctgggcacag agactgatgt 2100
gcatggagtc atgttccagg gcaacactgt gcagcttcag ggcatgagga 2150
agggtgcagc tatgctcttt cctcatacct ttgtcatggc catcatgcag 2200

cctgacaacc ttgggacatt tgagatttat tgccaggcag gcagccatcg 2250
agaagcaggg atgagggcaa tctataatgt ctcccagtgt cctggccacc 2300
aagccacccc tcgccaacgc taccaagctg caagaatcta ctatatcatg 2350
gcagaagaag tagagtggga ctattgccct gaccggagct gggaacggga 2400
atggcacaac cagtctgaga aggacagtta tggttacatt ttcctgagca 2450
acaaggatgg gctcctgggt tccagataca agaaagctgt attcagggaa 2500
tacactgatg gtacattcag gatccctcgg ccaaggactg gaccagaaga 2550
acacttggga atcttgggtc cacttatcaa aggtgaagtt ggtgatatcc 2600
tgactgtggt attcaagaat aatgccagcc gcccctactc tgtgcatgct 2650
catggagtgc tagaatctac tactgtctgg ccactggctg ctgagcctgg 2700
tgaggtggtc acttatcagt ggaacatccc agagaggtct ggccctgggc 2750
ccaatgactc tgcttgtgtt tcctggatct attattctgc agtggatccc 2800
atcaaggaca tgtatagtgg cctggtgggg cccttggcta tctgccaaaa 2850
gggcatcctg gagccccatg gaggacggag tgacatggat cgggaatttg 2900
cattgttgtt cttgattttt gatgaaaata agtcttggta tttggaggaa 2950
aatgtggcaa cccatgggtc ccaggatcca ggcagtatta acctacagga 3000
tgaaactttc ttggagagca ataaaatgca tgcaatcaat gggaaactct 3050
atgccaacct taggggtctt accatgtacc aaggagaacg agtggcctgg 3100
tacatgctgg ccatgggcca agatgtggat ctacacacca tccactttca 3150
tgcagagagc ttcctctatc ggaatggcga gaactaccgg gcagatgtgg 3200
tggatctgtt cccagggact tttgaggttg tggagatggt ggccagcaac 3250
cctgggacat ggctgatgca ctgccatgtg actgaccatg tccatgctgg 3300
catggagacc ctcttcactg ttttttctcg aacagaacac ttaagccctc 3350
tcaccgtcat caccaaagag actgaaaaag tgccccccag agacattgaa 3400
gaaggcaatg tgaagatgct gggcatgcag atccccataa agaatgttga 3450
gatgctggcc tctgttttgg ttgccattag tgtcaccctt ctgctcgttg 3500
ttctggctct tggtggagtg gtttggtacc aacatcgaca gagaaagcta 3550
cgacgcaata ggaggtccat cctggatgac agcttcaagc ttctgtcttt 3600
caaacagtaa catctggagc ctggagatat cctcaggaag cacatctgta 3650
gtgcactccc agcaggccat ggactagtca ctaaccccac actcaaaggg 3700
gcatgggtgg tggagaagca gaaggagcaa tcaagcttat ctggatattt 3750
ctttctttat ttattttaca tggaaataat atgatttcac tttttcttta 3800

gtttctttgc tctacgtggg cacctggcac taagggagta ccttattatc 3850

ctacatcgca aatttcaaca gctacattat atttccttct gacacttgga 3900

aggtattgaa atttctagaa atgtatcctt ctcacaaagt agagaccaag 3950

agaaaaactc attgattggg tttctacttc tttcaaggac tcaggaaatt 4000

tcactttgaa ctgaggccaa gtgagctgtt aagataaccc acacttaaac 4050

taaaggctaa gaatataggc ttgatgggaa attgaaggta ggctgagtat 4100

tgggaatcca aattgaattt tgattctcct tggcagtgaa ctactttgaa 4150

gaagtggtca atgggttgtt gctgccatga gcatgtacaa cctctggagc 4200

tagaagctcc tcaggaaagc cagttctcca agttcttaac ctgtggcact 4250

gaaaggaatg ttgagttacc tcttcatgtt ttagacagca aaccctatcc 4300

attaaagtac ttgttagacc aaaaaaaaaa aaa 4333

<210> 234
<211> 1160
<212> PRT
<213> Homo Sapien

<400> 234
Met Trp Ala Met Glu Ser Gly His Leu Leu Trp Ala Leu Leu Phe
1 5 10 15

Met Gln Ser Leu Trp Pro Gln Leu Thr Asp Gly Ala Thr Arg Val
20 25 30

Tyr Tyr Leu Gly Ile Arg Asp Val Gln Trp Asn Tyr Ala Pro Lys
35 40 45

Gly Arg Asn Val Ile Thr Asn Gln Pro Leu Asp Ser Asp Ile Val
50 55 60

Ala Ser Ser Phe Leu Lys Ser Asp Lys Asn Arg Ile Gly Gly Thr
65 70 75

Tyr Lys Lys Thr Ile Tyr Lys Glu Tyr Lys Asp Asp Ser Tyr Thr
80 85 90

Asp Glu Val Ala Gln Pro Ala Trp Leu Gly Phe Leu Gly Pro Val
95 100 105

Leu Gln Ala Glu Val Gly Asp Val Ile Leu Ile His Leu Lys Asn
110 115 120

Phe Ala Thr Arg Pro Tyr Thr Ile His Pro His Gly Val Phe Tyr
125 130 135

Glu Lys Asp Ser Glu Gly Ser Leu Tyr Pro Asp Gly Ser Ser Gly
140 145 150

Pro Leu Lys Ala Asp Asp Ser Val Pro Pro Gly Gly Ser His Ile
155 160 165

Tyr Asn Trp Thr Ile Pro Glu Gly His Ala Pro Thr Asp Ala Asp
170 175 180

Pro Ala Cys Leu Thr Trp Ile Tyr His Ser His Val Asp Ala Pro
185 190 195

Arg Asp Ile Ala Thr Gly Leu Ile Gly Pro Leu Ile Thr Cys Lys
200 205 210

Arg Gly Ala Leu Asp Gly Asn Ser Pro Pro Gln Arg Gln Asp Val
215 220 225

Asp His Asp Phe Phe Leu Leu Phe Ser Val Val Asp Glu Asn Leu
230 235 240

Ser Trp His Leu Asn Glu Asn Ile Ala Thr Tyr Cys Ser Asp Pro
245 250 255

Ala Ser Val Asp Lys Glu Asp Glu Thr Phe Gln Glu Ser Asn Arg
260 265 270

Met His Ala Ile Asn Gly Phe Val Phe Gly Asn Leu Pro Glu Leu
275 280 285

Asn Met Cys Ala Gln Lys Arg Val Ala Trp His Leu Phe Gly Met
290 295 300

Gly Asn Glu Ile Asp Val His Thr Ala Phe Phe His Gly Gln Met
305 310 315

Leu Thr Thr Arg Gly His His Thr Asp Val Ala Asn Ile Phe Pro
320 325 330

Ala Thr Phe Val Thr Ala Glu Met Val Pro Trp Glu Pro Gly Thr
335 340 345

Trp Leu Ile Ser Cys Gln Val Asn Ser His Phe Arg Asp Gly Met
350 355 360

Gln Ala Leu Tyr Lys Val Lys Ser Cys Ser Met Ala Pro Pro Val
365 370 375

Asp Leu Leu Thr Gly Lys Val Arg Gln Tyr Phe Ile Glu Ala His
380 385 390

Glu Ile Gln Trp Asp Tyr Gly Pro Met Gly His Asp Gly Ser Thr
395 400 405

Gly Lys Asn Leu Arg Glu Pro Gly Ser Ile Ser Asp Lys Phe Phe
410 415 420

Gln Lys Ser Ser Ser Arg Ile Gly Gly Thr Tyr Trp Lys Val Arg
425 430 435

Tyr Glu Ala Phe Gln Asp Glu Thr Phe Gln Glu Lys Met His Leu
440 445 450

Glu Glu Asp Arg His Leu Gly Ile Leu Gly Pro Val Ile Arg Ala
455 460 465

Glu Val Gly Asp Thr Ile Gln Val Val Phe Tyr Asn Arg Ala Ser
470 475 480

Gln Pro Phe Ser Met Gln Pro His Gly Val Phe Tyr Glu Lys Asp
485 490 495

```
Tyr Glu Gly Thr Val Tyr Asn Asp Gly Ser Ser Tyr Pro Gly Leu
                500                 505                 510

Val Ala Lys Pro Phe Glu Lys Val Thr Tyr Arg Trp Thr Val Pro
                515                 520                 525

Pro His Ala Gly Pro Thr Ala Gln Asp Pro Ala Cys Leu Thr Trp
                530                 535                 540

Met Tyr Phe Ser Ala Ala Asp Pro Ile Arg Asp Thr Asn Ser Gly
                545                 550                 555

Leu Val Gly Pro Leu Leu Val Cys Arg Ala Gly Ala Leu Gly Ala
                560                 565                 570

Asp Gly Lys Gln Lys Gly Val Asp Lys Glu Phe Phe Leu Leu Phe
                575                 580                 585

Thr Val Leu Asp Glu Asn Lys Ser Trp Tyr Ser Asn Ala Asn Gln
                590                 595                 600

Ala Ala Ala Met Leu Asp Phe Arg Leu Leu Ser Glu Asp Ile Glu
                605                 610                 615

Gly Phe Gln Asp Ser Asn Arg Met His Ala Ile Asn Gly Phe Leu
                620                 625                 630

Phe Ser Asn Leu Pro Arg Leu Asp Met Cys Lys Gly Asp Thr Val
                635                 640                 645

Ala Trp His Leu Leu Gly Leu Gly Thr Glu Thr Asp Val His Gly
                650                 655                 660

Val Met Phe Gln Gly Asn Thr Val Gln Leu Gln Gly Met Arg Lys
                665                 670                 675

Gly Ala Ala Met Leu Phe Pro His Thr Phe Val Met Ala Ile Met
                680                 685                 690

Gln Pro Asp Asn Leu Gly Thr Phe Glu Ile Tyr Cys Gln Ala Gly
                695                 700                 705

Ser His Arg Glu Ala Gly Met Arg Ala Ile Tyr Asn Val Ser Gln
                710                 715                 720

Cys Pro Gly His Gln Ala Thr Pro Arg Gln Arg Tyr Gln Ala Ala
                725                 730                 735

Arg Ile Tyr Tyr Ile Met Ala Glu Glu Val Glu Trp Asp Tyr Cys
                740                 745                 750

Pro Asp Arg Ser Trp Glu Arg Glu Trp His Asn Gln Ser Glu Lys
                755                 760                 765

Asp Ser Tyr Gly Tyr Ile Phe Leu Ser Asn Lys Asp Gly Leu Leu
                770                 775                 780

Gly Ser Arg Tyr Lys Lys Ala Val Phe Arg Glu Tyr Thr Asp Gly
                785                 790                 795

Thr Phe Arg Ile Pro Arg Pro Arg Thr Gly Pro Glu Glu His Leu
                800                 805                 810

Gly Ile Leu Gly Pro Leu Ile Lys Gly Glu Val Gly Asp Ile Leu
```

815 820 825

Thr Val Val Phe Lys Asn Asn Ala Ser Arg Pro Tyr Ser Val His
830 835 840

Ala His Gly Val Leu Glu Ser Thr Thr Val Trp Pro Leu Ala Ala
845 850 855

Glu Pro Gly Glu Val Val Thr Tyr Gln Trp Asn Ile Pro Glu Arg
860 865 870

Ser Gly Pro Gly Pro Asn Asp Ser Ala Cys Val Ser Trp Ile Tyr
875 880 885

Tyr Ser Ala Val Asp Pro Ile Lys Asp Met Tyr Ser Gly Leu Val
890 895 900

Gly Pro Leu Ala Ile Cys Gln Lys Gly Ile Leu Glu Pro His Gly
905 910 915

Gly Arg Ser Asp Met Asp Arg Glu Phe Ala Leu Leu Phe Leu Ile
920 925 930

Phe Asp Glu Asn Lys Ser Trp Tyr Leu Glu Glu Asn Val Ala Thr
935 940 945

His Gly Ser Gln Asp Pro Gly Ser Ile Asn Leu Gln Asp Glu Thr
950 955 960

Phe Leu Glu Ser Asn Lys Met His Ala Ile Asn Gly Lys Leu Tyr
965 970 975

Ala Asn Leu Arg Gly Leu Thr Met Tyr Gln Gly Glu Arg Val Ala
980 985 990

Trp Tyr Met Leu Ala Met Gly Gln Asp Val Asp Leu His Thr Ile
995 1000 1005

His Phe His Ala Glu Ser Phe Leu Tyr Arg Asn Gly Glu Asn Tyr
1010 1015 1020

Arg Ala Asp Val Val Asp Leu Phe Pro Gly Thr Phe Glu Val Val
1025 1030 1035

Glu Met Val Ala Ser Asn Pro Gly Thr Trp Leu Met His Cys His
1040 1045 1050

Val Thr Asp His Val His Ala Gly Met Glu Thr Leu Phe Thr Val
1055 1060 1065

Phe Ser Arg Thr Glu His Leu Ser Pro Leu Thr Val Ile Thr Lys
1070 1075 1080

Glu Thr Glu Lys Val Pro Pro Arg Asp Ile Glu Glu Gly Asn Val
1085 1090 1095

Lys Met Leu Gly Met Gln Ile Pro Ile Lys Asn Val Glu Met Leu
1100 1105 1110

Ala Ser Val Leu Val Ala Ile Ser Val Thr Leu Leu Leu Val Val
1115 1120 1125

Leu Ala Leu Gly Gly Val Val Trp Tyr Gln His Arg Gln Arg Lys
1130 1135 1140

Leu Arg Arg Asn Arg Arg Ser Ile Leu Asp Asp Ser Phe Lys Leu
1145 1150 1155

Leu Ser Phe Lys Gln
1160

<210> 235
<211> 3442
<212> DNA
<213> Homo Sapien

<400> 235
ggaaagagtg ctggtactac aaccaggaag tgacagataa tgtgctttaa 50

actacattag aaaagcttct catagcaaaa ctgagagatt gaagcagtga 100

ttatttttac atagttgtca ttaaatattt ggagctctgc tgtgcataga 150

gatggcaaca tacttagaat acacagcttt ctgggccaga aattgatctt 200

ctgacttttg agccttatct gattactgct tggttcatct ttattttgtt 250

aaactactct gtaggctgaa agggagagac tctccttggt ttgcagagcc 300

tgactagaca ggaattctgg caactgctcc agcagaacta tggcactgag 350

ctaggtttaa atgctgagga gatggaaaac ttgtcactgt cgattgagga 400

tgtgcagcca agaagtccag gaagaagcag cttggatgac tctggggaga 450

gagatgaaaa attatccaag tcaatcagtt ttaccagtga atcaattagt 500

cgggtttcag aaacagagtc attcgatgga aattcatcaa aaggaggatt 550

aggcaaagag gagtcccaaa atgagaaaca gaccaaaaag agtctcttac 600

caactttgga aaagaagtta actagagtgc catcaaagtc actggacttg 650

aataaaaatg aatatctttc tctggacaaa agcagcactt cagattctgt 700

tgatgaagaa aatgttcctg agaaagatct tcatggaaga ctttttatca 750

accgtatttt tcatatcagt gctgacagaa tgtttgaatt gctctttacc 800

agttcacgct ttatgcagaa atttgccagt tctagaaata taatagatgt 850

agtatctacc ccttggactg cagaacttgg aggtgatcag ctgagaacga 900

tgacctacac tatagtcctt aatagtccac ttactggaaa atgcactgct 950

gccactgaaa agcagacact gtataaagaa agtcgggaag cacgatttta 1000

tttggtagat tcagaagtac tgacacatga tgtcccctac catgattact 1050

tctataccgt gaacagatac tgtatcatcc gatcttcaaa acagaaatgc 1100

aggctaagag tttccacaga tttgaaatac agaaaacagc catggggcct 1150

tgtcaaatct ttaattgaaa agaattcctg gagttctttg gaggactatt 1200

tcaaacagct tgaatcagat ttgttaattg aagaatctgt attaaatcag 1250

gccattgaag accctggaaa acttactggc ctacgaagga gaaggcgaac 1300

cttcaaccga acagcagaaa cagttcctaa actttcctct cagcattcct 1350
ctggagatgt gggcttaggt gccaaagggg atattacagg aaagaaaaag 1400
gaaatggaaa actataacgt cactcttatt gtggtaatga gtatttttgt 1450
gttgttatta gttttgttga atgtgacact gtttctgaag ctgtcaaaga 1500
tagaacatgc tgctcagtcc ttttaccgtc tccgcctcca agaagagaaa 1550
tctttaaatt tagcctctga tatggtgtca agagcagaaa ctattcagaa 1600
gaataaagat caggcccatc gtttaaaggg agtgctccga gactccatag 1650
tgatgcttga acagctgaag agctcactca ttatgcttca gaaaacgttt 1700
gatctactaa ataagaataa gactggcatg gctgttgaaa gctagtgatc 1750
tgaaggacta aaaccgcaga gatacttgga acttaaagaa aatacctgga 1800
agaaaaccag acgaatgaag gattttggca tagaacattt ctatgttttt 1850
tcattattga gatttctaat atgaacattt ctttcagtaa catttatttg 1900
ataattagtt tctgctggcc ttaataatcc atcctttcac ttcttataga 1950
tatttttaag ctgtgaattt cttcagtgaa ccatgaaata tattatagaa 2000
ctgaatttct ctgatacaaa aagaaaatga cacaccctga attgagtggt 2050
atggtctcat ttctacagtg aagtctgatg ctttgttagc acagaatccg 2100
tacatgtcca ataggtcgct tttgtaactg agataagacc aagaggataa 2150
acaggacaat ataagaagaa acctctatgt cattactgat tttaaaggtt 2200
ctgttttcag gcatataaca tttccaggtt tgtgtactgt aaagattata 2250
atgtcttcat ttatttagca tgcaaattta atagtcaaac tttttgaatc 2300
tgcatgttga tgatgattat cagaaagggt cttctgccat gctgtatctt 2350
tatgaaagaa atagttgttt tttcttaagg taactatcag aggtgggatt 2400
atcttgcctc ctcacttaga ataccaacag tcaaaaggaa gaaccatcct 2450
ctgagtttta aaaaccagaa ggttatgtta aaatctgggc atttagtgac 2500
agatcaaatg catacttgaa ctaagattgg cttcagctta gcagtctttc 2550
atggtggaag tgacacatct ggttgaaaat aatttgtgta ttttcagtaa 2600
ccatgtatgg cttccttctt tatgtatgtg tgtgacttgt tttaattggt 2650
aagttataag ccagacatag attttagctc tttaataaaa acttcagggg 2700
cacgtatgtc ccagtacaag tgtactgact atcaagtttt aactcagatg 2750
caagctttgg ctctttcata aaaagttttt atgcatatgt gtctccatac 2800
aagtggctca ttaaaataag aactttgtaa actgacttaa aatcagatat 2850
tttttcaaga gttagggaaa gttgaagtgt tttactgttt tgtctcttga 2900

gccctttctc tggggaaaaa atacatatcc atctatctat ctatatataa 2950

actgtgtata cattcttact gtttgaacaa ctattgcctt taattaaatg 3000

tttcattttt ctccagagtc cccaaagcca catggcatta ttatagtcat 3050

ttttgagatg cctgtagaga atgaaagtat tgactccgtt agagggaaaa 3100

tgggtttctc tgggtgaatt ccaacgaagc atacctaggg gtaacagtga 3150

acctacctgg gtttgttttg ttttggtaag gatttatgta gtgtctggct 3200

gtaagcaaga atgagtggat tataaacttg aagatttctc tgttaaagtc 3250

acaaaaatga tcgacaaaca atatttttgt gatgtttatt taaacgttgt 3300

attttataac atacttcaag gaagagtatc gaagtaagtt gctttataaa 3350

ttaagactaa attcgtatgg atgcagaatt caattaataa aatttgagcc 3400

tgttacgtaa attgaatatt aataaaattg aaaatttcaa aa 3442

<210> 236
<211> 457
<212> PRT
<213> Homo Sapien

<400> 236
Met Glu Asn Leu Ser Leu Ser Ile Glu Asp Val Gln Pro Arg Ser
1               5                   10                  15

Pro Gly Arg Ser Ser Leu Asp Asp Ser Gly Glu Arg Asp Glu Lys
                20                  25                  30

Leu Ser Lys Ser Ile Ser Phe Thr Ser Glu Ser Ile Ser Arg Val
                35                  40                  45

Ser Glu Thr Glu Ser Phe Asp Gly Asn Ser Ser Lys Gly Gly Leu
                50                  55                  60

Gly Lys Glu Glu Ser Gln Asn Glu Lys Gln Thr Lys Lys Ser Leu
                65                  70                  75

Leu Pro Thr Leu Glu Lys Lys Leu Thr Arg Val Pro Ser Lys Ser
                80                  85                  90

Leu Asp Leu Asn Lys Asn Glu Tyr Leu Ser Leu Asp Lys Ser Ser
                95                  100                 105

Thr Ser Asp Ser Val Asp Glu Glu Asn Val Pro Glu Lys Asp Leu
                110                 115                 120

His Gly Arg Leu Phe Ile Asn Arg Ile Phe His Ile Ser Ala Asp
                125                 130                 135

Arg Met Phe Glu Leu Leu Phe Thr Ser Ser Arg Phe Met Gln Lys
                140                 145                 150

Phe Ala Ser Ser Arg Asn Ile Ile Asp Val Val Ser Thr Pro Trp
                155                 160                 165

Thr Ala Glu Leu Gly Gly Asp Gln Leu Arg Thr Met Thr Tyr Thr
                170                 175                 180

Ile Val Leu Asn Ser Pro Leu Thr Gly Lys Cys Thr Ala Ala Thr
185 190 195

Glu Lys Gln Thr Leu Tyr Lys Glu Ser Arg Glu Ala Arg Phe Tyr
200 205 210

Leu Val Asp Ser Glu Val Leu Thr His Asp Val Pro Tyr His Asp
215 220 225

Tyr Phe Tyr Thr Val Asn Arg Tyr Cys Ile Ile Arg Ser Ser Lys
230 235 240

Gln Lys Cys Arg Leu Arg Val Ser Thr Asp Leu Lys Tyr Arg Lys
245 250 255

Gln Pro Trp Gly Leu Val Lys Ser Leu Ile Glu Lys Asn Ser Trp
260 265 270

Ser Ser Leu Glu Asp Tyr Phe Lys Gln Leu Glu Ser Asp Leu Leu
275 280 285

Ile Glu Glu Ser Val Leu Asn Gln Ala Ile Glu Asp Pro Gly Lys
290 295 300

Leu Thr Gly Leu Arg Arg Arg Arg Arg Thr Phe Asn Arg Thr Ala
305 310 315

Glu Thr Val Pro Lys Leu Ser Ser Gln His Ser Ser Gly Asp Val
320 325 330

Gly Leu Gly Ala Lys Gly Asp Ile Thr Gly Lys Lys Lys Glu Met
335 340 345

Glu Asn Tyr Asn Val Thr Leu Ile Val Val Met Ser Ile Phe Val
350 355 360

Leu Leu Leu Val Leu Leu Asn Val Thr Leu Phe Leu Lys Leu Ser
365 370 375

Lys Ile Glu His Ala Ala Gln Ser Phe Tyr Arg Leu Arg Leu Gln
380 385 390

Glu Glu Lys Ser Leu Asn Leu Ala Ser Asp Met Val Ser Arg Ala
395 400 405

Glu Thr Ile Gln Lys Asn Lys Asp Gln Ala His Arg Leu Lys Gly
410 415 420

Val Leu Arg Asp Ser Ile Val Met Leu Glu Gln Leu Lys Ser Ser
425 430 435

Leu Ile Met Leu Gln Lys Thr Phe Asp Leu Leu Asn Lys Asn Lys
440 445 450

Thr Gly Met Ala Val Glu Ser
455

<210> 237
<211> 762
<212> DNA
<213> Homo Sapien

<400> 237

caggggctgg agggcagggg aggggatgat gtcattcctg ctcggcgcaa 50

tcctgaccct gctctgggcg cccacggctc aggctgaggt tctgctgcag 100

cctgacttca atgctgaaaa gttctcaggc ctctggtacg tggtctccat 150

ggcatctgac tgcagggtct tcctgggcaa gaaggaccac ctgtccatgt 200

ccaccagggc catcaggccc acagaggagg gcggcctcca cgtccacatg 250

gagttcccgg gggcggacgg ctgtaaccag gtggatgccg agtacctgaa 300

ggtgggctcc gagggacact tcagagtccc ggccttgggc tacctggacg 350

tgcgcatcgt ggacacagac tacagctcct tcgccgtcct ttacatctac 400

aaggagctgg agggggccct cagcaccatg gtgcagctct acagccggac 450

ccaggatgtg agtccccagg ctctgaagtc cttccaggac ttctacccga 500

ccctggggct ccccaaggac atgatggtca tgctgcccca gtcagatgca 550

tgcaaccctg agagcaagga ggcgccctga cacctccgga gccccacccc 600

cgcccttccc aggtggagcc aaagcagcag gcgcctttgc ccctggagtc 650

aagacccaca gccctcgggg accacctgga gtctctccat cctccacccc 700

ccgcctgtgg gatgccttgt gggacgtctc tttctattca ataaacagat 750

gctgcagcct ca 762

<210> 238
<211> 184
<212> PRT
<213> Homo Sapien

<400> 238
Met Met Ser Phe Leu Leu Gly Ala Ile Leu Thr Leu Leu Trp Ala
1 5 10 15

Pro Thr Ala Gln Ala Glu Val Leu Leu Gln Pro Asp Phe Asn Ala
20 25 30

Glu Lys Phe Ser Gly Leu Trp Tyr Val Val Ser Met Ala Ser Asp
35 40 45

Cys Arg Val Phe Leu Gly Lys Lys Asp His Leu Ser Met Ser Thr
50 55 60

Arg Ala Ile Arg Pro Thr Glu Glu Gly Gly Leu His Val His Met
65 70 75

Glu Phe Pro Gly Ala Asp Gly Cys Asn Gln Val Asp Ala Glu Tyr
80 85 90

Leu Lys Val Gly Ser Glu Gly His Phe Arg Val Pro Ala Leu Gly
95 100 105

Tyr Leu Asp Val Arg Ile Val Asp Thr Asp Tyr Ser Ser Phe Ala
110 115 120

Val Leu Tyr Ile Tyr Lys Glu Leu Glu Gly Ala Leu Ser Thr Met
125 130 135

Val Gln Leu Tyr Ser Arg Thr Gln Asp Val Ser Pro Gln Ala Leu
140 145 150

Lys Ser Phe Gln Asp Phe Tyr Pro Thr Leu Gly Leu Pro Lys Asp
155 160 165

Met Met Val Met Leu Pro Gln Ser Asp Ala Cys Asn Pro Glu Ser
170 175 180

Lys Glu Ala Pro

<210> 239
<211> 1656
<212> DNA
<213> Homo Sapien

<400> 239
ggcgcgctgg tccaggtgag cgggcgcgtc cccgcgacgg cgctgcctgc 50

ccgaggcggt tcacgtaaag acagcgagat cctgagggcc agccgggaag 100

gaggcgtgga tatggagctg gctgctgcca agtccggggc ccgcgccgct 150

gcctagcgcg tcctggggac tctgtgggga cgcgccccgc gccgcggctc 200

ggggacccgt agagcccggc gctgcgcgca tggccctgct ctcgcgcccc 250

gcgctcaccc tcctgctcct cctcatggcc gctgttgtca ggtgccagga 300

gcaggcccag accaccgact ggagagccac cctgaagacc atccggaacg 350

gcgttcataa gatagacacg tacctgaacg ccgccttgga cctcctggga 400

ggcgaggacg gtctctgcca gtataaatgc agtgacggat ctaagccttt 450

cccacgttat ggttataaac cctccccacc gaatggatgt ggctctccac 500

tgtttggtgt tcatcttaac attggtatcc cttccctgac aaagtgttgc 550

aaccaacacg acaggtgcta tgagacctgt ggcaaaagca agaatgactg 600

tgatgaagaa ttccagtatt gcctctccaa gatctgccga gatgtacaga 650

aaacactagg actaactcag catgttcagg catgtgaaac aacagtggag 700

ctcttgtttg acagtgttat acatttaggt tgtaaaccat atctggacag 750

ccaacgagcc gcatgcaggt gtcattatga agaaaaaact gatctttaaa 800

ggagatgccg acagctagtg acagatgaag atggaagaac ataacctttg 850

acaaataact aatgttttta caacataaaa ctgtcttatt tttgtgaaag 900

gattattttg agaccttaaa ataatttata tcttgatgtt aaaacctcaa 950

agcaaaaaaa gtgagggaga tagtgagggg agggcacgct tgtcttctca 1000

ggtatcttcc ccagcattgc tcccttactt agtatgccaa atgtcttgac 1050

caatatcaaa aacaagtgct tgtttagcgg agaattttga aaagaggaat 1100

atataactca attttcacaa ccacatttac caaaaaaaga gatcaaatat 1150

aaaattcatc ataatgtctg ttcaacatta tcttatttgg aaaatgggga 1200

aattatcact tacaagtatt tgtttactat gaaattttaa atacacattt 1250

atgcctagaa ggaacggact tttttttct attttaatta cacataatat 1300

gtaattaaag tacaacataa tatgttgttt ctctgtagcc cgttgagcat 1350

atgagtaagt cacatttcta ttaggactac ttacaaggac aaggtttcca 1400

tttttccagt tgtaaaattg gaaccatcag ctgataacct cgtagggagc 1450

aaccccagga tagctaagtg ttatgtaata tgcctagaag gtgatgtgaa 1500

tgcgattcag aagcatagcc actcccattt tatgagctac tcacatgaca 1550

aatgtcatct tttgctataa cctttgccaa gttagagaaa agatggattt 1600

aatgagataa atgaaaagat atttaaccta aaaaaaaaaa aaaaaaaaaa 1650

aaaaaa 1656

<210> 240
<211> 189
<212> PRT
<213> Homo Sapien

<400> 240
Met Ala Leu Leu Ser Arg Pro Ala Leu Thr Leu Leu Leu Leu Leu
1 5 10 15

Met Ala Ala Val Val Arg Cys Gln Glu Gln Ala Gln Thr Thr Asp
20 25 30

Trp Arg Ala Thr Leu Lys Thr Ile Arg Asn Gly Val His Lys Ile
35 40 45

Asp Thr Tyr Leu Asn Ala Ala Leu Asp Leu Leu Gly Gly Glu Asp
50 55 60

Gly Leu Cys Gln Tyr Lys Cys Ser Asp Gly Ser Lys Pro Phe Pro
65 70 75

Arg Tyr Gly Tyr Lys Pro Ser Pro Pro Asn Gly Cys Gly Ser Pro
80 85 90

Leu Phe Gly Val His Leu Asn Ile Gly Ile Pro Ser Leu Thr Lys
95 100 105

Cys Cys Asn Gln His Asp Arg Cys Tyr Glu Thr Cys Gly Lys Ser
110 115 120

Lys Asn Asp Cys Asp Glu Glu Phe Gln Tyr Cys Leu Ser Lys Ile
125 130 135

Cys Arg Asp Val Gln Lys Thr Leu Gly Leu Thr Gln His Val Gln
140 145 150

Ala Cys Glu Thr Thr Val Glu Leu Leu Phe Asp Ser Val Ile His
155 160 165

Leu Gly Cys Lys Pro Tyr Leu Asp Ser Gln Arg Ala Ala Cys Arg
170 175 180

Cys His Tyr Glu Glu Lys Thr Asp Leu
185

<210> 241
<211> 1319
<212> DNA
<213> Homo Sapien

<400> 241
gattccgagc gcctccactg ctggtccgtt ggccagatca actcgccgcg 50

tgggccggcc gttccctgag agtctgagcg ctcgccgcac ccccttccga 100

gcttctattg gccgtagcag acgtccgtct gccgctatct ccgccccaat 150

acggaagcgg cctagtcctc cggctccgac agctgggtgt ccaggccatg 200

gggcagccct gggcggctgg gagcacggac ggggcgcccg cgcagctgcc 250

tctcgtgctc accgcgctgt gggccgcggc cgtgggcctg gagctggctt 300

acgtgctggt gctcggtccc gggccgccgc cgctgggacc cctggcccgg 350

gccttgcagc tggcgctggc cgccttccag ctgctcaacc tgctgggcaa 400

cgtggggctc ttcctgcgct cggatcccag catccgtggc gtgatgctgg 450

ccggccgcgg tctgggccag ggctgggctt actgctacca atgccaaagc 500

caggtgccgc cacgcagcgg acactgctct gcctgccgcg tctgcatcct 550

gcgtcgggac caccactgcc gcctgctggg ccgctgcgtg ggcttcggca 600

actaccggcc cttcctgtgc ctgctgcttc atgccgccgg cgtcctgctc 650

cacgtctctg tgctgctggg ccctgcactg tcggccctgc tgcgagccca 700

cacgcccctc cacatggctg ccctcctcct gcttccctgg ctcatgttgc 750

tcacaggcag agtgtctctg gcacagtttg ccttggcctt cgtgacggac 800

acgtgcgtgg cgggtgcgct gctgtgcggg gctgggctgc tcttccatgg 850

gatgctgctg ctgcggggcc agaccacatg ggagtgggct cggggccagc 900

actcctatga cctgggtccc tgccacaacc tgcaggcagc cctggggccc 950

cgctgggccc tcgtctggct ctggcccttc ctggcctccc cattgcctgg 1000

ggatgggatc accttccaga ccacagcaga tgtgggacac acagcctcct 1050

gactccagga agagccagag ctgtgcaggg aggaaggggt gagagggggg 1100

cccccacacc tagactcagt aaggaagtcg ggttggacct taacatctgc 1150

attggacaac tccacccctt ccttggcctt gcccctgccc gcctacactc 1200

ctacgtgtcc agggcttggg ccgtgactta ggcagaggag tgcagaggag 1250

ggtctggcag ggctgctca ggccgcctag ctgccccttt gccaggttaa 1300

taaagcactg acttgttaa 1319

<210> 242
<211> 284
<212> PRT
<213> Homo Sapien

<400> 242
```
Met Gly Gln Pro Trp Ala Ala Gly Ser Thr Asp Gly Ala Pro Ala
1               5                   10                  15

Gln Leu Pro Leu Val Leu Thr Ala Leu Trp Ala Ala Ala Val Gly
                20                  25                  30

Leu Glu Leu Ala Tyr Val Leu Val Leu Gly Pro Gly Pro Pro Pro
                35                  40                  45

Leu Gly Pro Leu Ala Arg Ala Leu Gln Leu Ala Leu Ala Ala Phe
                50                  55                  60

Gln Leu Leu Asn Leu Leu Gly Asn Val Gly Leu Phe Leu Arg Ser
                65                  70                  75

Asp Pro Ser Ile Arg Gly Val Met Leu Ala Gly Arg Gly Leu Gly
                80                  85                  90

Gln Gly Trp Ala Tyr Cys Tyr Gln Cys Gln Ser Gln Val Pro Pro
                95                  100                 105

Arg Ser Gly His Cys Ser Ala Cys Arg Val Cys Ile Leu Arg Arg
                110                 115                 120

Asp His His Cys Arg Leu Leu Gly Arg Cys Val Gly Phe Gly Asn
                125                 130                 135

Tyr Arg Pro Phe Leu Cys Leu Leu Leu His Ala Ala Gly Val Leu
                140                 145                 150

Leu His Val Ser Val Leu Leu Gly Pro Ala Leu Ser Ala Leu Leu
                155                 160                 165

Arg Ala His Thr Pro Leu His Met Ala Ala Leu Leu Leu Leu Pro
                170                 175                 180

Trp Leu Met Leu Leu Thr Gly Arg Val Ser Leu Ala Gln Phe Ala
                185                 190                 195

Leu Ala Phe Val Thr Asp Thr Cys Val Ala Gly Ala Leu Leu Cys
                200                 205                 210

Gly Ala Gly Leu Leu Phe His Gly Met Leu Leu Leu Arg Gly Gln
                215                 220                 225

Thr Thr Trp Glu Trp Ala Arg Gly Gln His Ser Tyr Asp Leu Gly
                230                 235                 240

Pro Cys His Asn Leu Gln Ala Ala Leu Gly Pro Arg Trp Ala Leu
                245                 250                 255

Val Trp Leu Trp Pro Phe Leu Ala Ser Pro Leu Pro Gly Asp Gly
                260                 265                 270

Ile Thr Phe Gln Thr Thr Ala Asp Val Gly His Thr Ala Ser
                275                 280
```

<210> 243

<211> 1837
<212> DNA
<213> Homo Sapien

<400> 243
cttgtctttg tgtcggttgt gattttccta atctctgatt ttccttttct 50

ctcggacgct ctccctcttc ggacccattt tctcccgtgc ttcatgccct 100

gatagcctgg ccccttcccg gcttccttcg ctaccgggga cgcctctagt 150

ttttctgaat ttctggctgg ctccaccctc cgcgttcatc ttcctcaaga 200

gttcgcccct ctgggggctc ctctgtgtaa tcgtcgcctt ctctgggtat 250

ttctgtgaac tccgtctcac accatcccgc catcttctct gccttggccc 300

cttttctctg tacagccagc tctgtgtcct tttcttctcc ccctctaaaa 350

tcgactcctc ttctccctga gagccccacc tttgtgcccc actcctcatt 400

ttcctacgcc tccctctctc tgctggtcct ctctctccct gcaaggttcc 450

attccatcaa tttgtttgtc ttttgtaggg gtggcatccc ctctgactac 500

tgctccatcc tttttttttt tttttttttt ttttttgctt gaggatttca 550

cttcaatctt ttctggttgc gtctccactt gtactcagct tgttaggtcc 600

aggtccagtt gttctgcatc tgaggctggc gtgtgctgtc ttctctgatt 650

ggcctaatct ccctcacccc cgtgagatct gttgtcagcc ttcgtttctc 700

tttcctgtgt cccagctttt ctgcgggtct tggcaccttt cttggccaca 750

gatttctggg ttacagagca tgtgtgtctg aggcattgca ggcagaaaag 800

ggtggccgac gtgacctcta gctggactgc tgggcagggg agctgtccta 850

gataaaattg gaaagaaaca gtgacccaga gacaggtgga caaagaattc 900

ggggactgat gggaactgag cttgggatcc agactgaaac tgattccaga 950

ctgacctcta gcacccagga cccagacaca gggccatggg accccagcat 1000

ttgagacttg tgcagctgtt ctgccttcta ggggccatcc ccactctgcc 1050

tcgggctgga gctcttttgt gctatgaagc aacagcctca agattcagag 1100

ctgttgcttt ccataactgg aagtggcttc tgatgaggaa catggtgtgt 1150

aagctgcaag agggctgcga ggagacgcta gtgttcattg agacagggac 1200

tgcaagggga gttgtgggct ttaaaggctg cagctcgtct tcgtcttacc 1250

ctgcgcaaat ctcctacctt gtttccccac ccggagtgtc cattgcctcc 1300

tacagtcgcg tctgccggtc ttatctctgc aacaacctca ccaatttgga 1350

gcctttttgtg aaactcaagg ccagcactcc taagtctatc acatctgcgt 1400

cctgtagctg cccgacctgt gtgggcgagc acatgaagga ttgcctccca 1450

aattttgtca ccactaattc ttgcccccttg gctgcttcta cgtgttacag 1500

ttccacctta aaatttcagg cagggtttct caataccacc ttcctcctca 1550

tggggtgtgc tcgtgaacat aaccagcttt tagcagattt tcatcatatt 1600

gggagcatca aagtgactga ggtcctcaac atcttagaga agtctcagat 1650

tgttggtgca gcatcctcca ggcaagatcc tgcttggggt gtcgtcttag 1700

gcctcctgtt tgccttcagg gactgaccat ctagctgcac ccgacaagca 1750

cccagactct ttcacataac aaataaaata gcagagttcc cttaaaaaaa 1800

aaaaaaaaaa aaaaaaaaaa aaaaaaaaaa aaaaaaa 1837

<210> 244
<211> 246
<212> PRT
<213> Homo Sapien

<400> 244
Met Gly Pro Gln His Leu Arg Leu Val Gln Leu Phe Cys Leu Leu
1 5 10 15

Gly Ala Ile Pro Thr Leu Pro Arg Ala Gly Ala Leu Leu Cys Tyr
20 25 30

Glu Ala Thr Ala Ser Arg Phe Arg Ala Val Ala Phe His Asn Trp
35 40 45

Lys Trp Leu Leu Met Arg Asn Met Val Cys Lys Leu Gln Glu Gly
50 55 60

Cys Glu Glu Thr Leu Val Phe Ile Glu Thr Gly Thr Ala Arg Gly
65 70 75

Val Val Gly Phe Lys Gly Cys Ser Ser Ser Ser Ser Tyr Pro Ala
80 85 90

Gln Ile Ser Tyr Leu Val Ser Pro Pro Gly Val Ser Ile Ala Ser
95 100 105

Tyr Ser Arg Val Cys Arg Ser Tyr Leu Cys Asn Asn Leu Thr Asn
110 115 120

Leu Glu Pro Phe Val Lys Leu Lys Ala Ser Thr Pro Lys Ser Ile
125 130 135

Thr Ser Ala Ser Cys Ser Cys Pro Thr Cys Val Gly Glu His Met
140 145 150

Lys Asp Cys Leu Pro Asn Phe Val Thr Thr Asn Ser Cys Pro Leu
155 160 165

Ala Ala Ser Thr Cys Tyr Ser Ser Thr Leu Lys Phe Gln Ala Gly
170 175 180

Phe Leu Asn Thr Thr Phe Leu Leu Met Gly Cys Ala Arg Glu His
185 190 195

Asn Gln Leu Leu Ala Asp Phe His His Ile Gly Ser Ile Lys Val
200 205 210

Thr Glu Val Leu Asn Ile Leu Glu Lys Ser Gln Ile Val Gly Ala

```
                215                 220                 225
Ala Ser Ser Arg Gln Asp Pro Ala Trp Gly Val Val Leu Gly Leu
                230                 235                 240

Leu Phe Ala Phe Arg Asp
                245
```

<210> 245
<211> 2594
<212> DNA
<213> Homo Sapien

<400> 245

```
gtggagttgg gtggtgtcgg gagcctctcc ctgaggggca ccgcgtcttc 50
aggagctggg cctccagtgc ggcgcgatgt caggcgcggt gacagctctg 100
tgagtccgag gccgcggccg tggcgctggg cggctgcggg gcctgaccgg 150
tccgctcatg gtgccgccac gacgccatcg cggggcagga aggccagggg 200
tgctgagttc ttcacctcct tttagactga gatctgccaa gttttccggc 250
attgctcttg aggatctcag aagggctctt aagacaagac tgcaaatggt 300
gtgtgtattt gtcatgaacc gaatgaattc ccagaacagt ggtttcactc 350
agcgcaggcg aatggctctt gggattgtta ttcttctgct tgttgatgtg 400
atatgggttg cttcctctga acttacttcg tatgttttta cccagtacaa 450
caaaccattc ttcagcacct ttgcaaaaac atctatgttt gttttgtacc 500
ttttgggctt tattatttgg aagccatgga gacaacagtg tacaagagga 550
cttcgcggaa agcatgctgc tttttttgca gatgctgaag gttactttgc 600
tgcttgcaca acagatacaa ctatgaatag ttctttgagt gaacctctgt 650
atgtgcctgt gaaattccat gatcttccaa gtgaaaaacc tgagagcaca 700
aacattgata ctgaaaaaac ccccaaaaag tctcgtgtga ggttcagtaa 750
tatcatggag attcgacagc ttccgtcaag tcatgcattg gaagcaaagt 800
tgtctcgcat gtcatatcct gtgaaagaac aagaatccat actgaaaact 850
gtggggaaac ttactgcaac tcaagtagcg aaaattagct tttttttttg 900
ctttgtgtgg tttttggcaa atttgtcata tcaagaagca ctttcagaca 950
cacaagttgc tatagttaat attttatctt caacttccgg actttttacc 1000
ttaatccttg ctgcagtatt tccaagtaac agtggagata gatttaccct 1050
ttctaaacta ttagctgtaa ttttaagcat tggaggcgtt gtactggtaa 1100
acctggcagg gtctgaaaaa cctgctggaa gagacacagt aggttccatt 1150
tggtctcttg ctggagccat gctctatgct gtctatattg ttatgattaa 1200
gagaaaagta gatagagaag acaagttgga tattccaatg ttctttggtt 1250
```

ttgtaggttt gtttaatctg ctgctcttat ggccaggttt ctttttactt 1300

cattatactg gatttgagga cttcgagttt cccaataaag tagtattaat 1350

gtgcattatc attaatggcc ttattggaac agtactctca gagttcctgt 1400

ggttgtgggg ctgctttctt acctcatcat tgataggcac acttgcacta 1450

agccttacaa tacctctgtc cataatagct gacatgtgta tgcaaaaggt 1500

gcagttttct tggttatttt ttgcaggagc tatccctgta ttttttttcat 1550

tttttattgt aactctccta tgccattata ataattggga tcctgtgatg 1600

gtgggaatca gaagaatatt tgcttttata tgcagaaaac atcgaattca 1650

gagagttcca gaagacagcg aacagtgtga gagtctcatt tctatgcaca 1700

gtgtttctca ggaggatgga gctagttagc tgtctgttgt ctgtagccca 1750

gcttgataat ggaactatac agcgaagaga caatctctgg caagtttttg 1800

tagaaaaaat gtttcagtgc ctagtctgaa aaataacagt ttgagttctt 1850

tgaaactcta aaatatattt ttctcatacc tgttttcttc attttcataa 1900

tgaagcactt tgctatgtag ctgtgtacat atcactacag ttataggaag 1950

tttcagtcta cagtccatcc aaaggaccaa cctgccttac acatctcaag 2000

gaattcagct gttgaaatca tttgaactaa tcaaggaata aatcctaatg 2050

ttctgggact ttattttcac atgttaaatg ctggaatata ttatgaaaat 2100

gttttcaaga aatcacttaa gtgttcatag accagtattt ctgacaggta 2150

aaatgctaaa ataagctacc tgtaataagt gtggattata tttttgggtt 2200

ttgtagaata ttgcaaatta accacacaaa aaatgtttaa tttatgcaac 2250

aagcatgttt gtgcaaattt catgggactt taaaaagaat aagtatttga 2300

gaaaatatct ggttcactta cactacattt actgtattat tcttttatag 2350

cattaggtgc cttgtatttt aaatctgtga caaaccatgg caaattttta 2400

aaggggaagt attattataa aatgaagaaa tatgtatttc taaaggctat 2450

attgctgtaa acttaattga taaagctctg tttaatttag agttttgaag 2500

aaatagtctc ccttcaatta agaaattttc ataatggaat gatttaaatt 2550

gaagtgacaa agagtattat taaaatacaa tgtttataaa aaaa 2594

<210> 246
<211> 523
<212> PRT
<213> Homo Sapien

<400> 246
Met Val Pro Pro Arg Arg His Arg Gly Ala Gly Arg Pro Gly Val
1               5                   10                  15

Leu Ser Ser Ser Pro Pro Phe Arg Leu Arg Ser Ala Lys Phe Ser

```
                20                  25                  30
Gly Ile Ala Leu Glu Asp Leu Arg Arg Ala Leu Lys Thr Arg Leu
                35                  40                  45
Gln Met Val Cys Val Phe Val Met Asn Arg Met Asn Ser Gln Asn
                50                  55                  60
Ser Gly Phe Thr Gln Arg Arg Arg Met Ala Leu Gly Ile Val Ile
                65                  70                  75
Leu Leu Leu Val Asp Val Ile Trp Val Ala Ser Ser Glu Leu Thr
                80                  85                  90
Ser Tyr Val Phe Thr Gln Tyr Asn Lys Pro Phe Phe Ser Thr Phe
                95                  100                 105
Ala Lys Thr Ser Met Phe Val Leu Tyr Leu Leu Gly Phe Ile Ile
                110                 115                 120
Trp Lys Pro Trp Arg Gln Gln Cys Thr Arg Gly Leu Arg Gly Lys
                125                 130                 135
His Ala Ala Phe Phe Ala Asp Ala Glu Gly Tyr Phe Ala Ala Cys
                140                 145                 150
Thr Thr Asp Thr Thr Met Asn Ser Ser Leu Ser Glu Pro Leu Tyr
                155                 160                 165
Val Pro Val Lys Phe His Asp Leu Pro Ser Glu Lys Pro Glu Ser
                170                 175                 180
Thr Asn Ile Asp Thr Glu Lys Thr Pro Lys Lys Ser Arg Val Arg
                185                 190                 195
Phe Ser Asn Ile Met Glu Ile Arg Gln Leu Pro Ser Ser His Ala
                200                 205                 210
Leu Glu Ala Lys Leu Ser Arg Met Ser Tyr Pro Val Lys Glu Gln
                215                 220                 225
Glu Ser Ile Leu Lys Thr Val Gly Lys Leu Thr Ala Thr Gln Val
                230                 235                 240
Ala Lys Ile Ser Phe Phe Phe Cys Phe Val Trp Phe Leu Ala Asn
                245                 250                 255
Leu Ser Tyr Gln Glu Ala Leu Ser Asp Thr Gln Val Ala Ile Val
                260                 265                 270
Asn Ile Leu Ser Ser Thr Ser Gly Leu Phe Thr Leu Ile Leu Ala
                275                 280                 285
Ala Val Phe Pro Ser Asn Ser Gly Asp Arg Phe Thr Leu Ser Lys
                290                 295                 300
Leu Leu Ala Val Ile Leu Ser Ile Gly Gly Val Val Leu Val Asn
                305                 310                 315
Leu Ala Gly Ser Glu Lys Pro Ala Gly Arg Asp Thr Val Gly Ser
                320                 325                 330
Ile Trp Ser Leu Ala Gly Ala Met Leu Tyr Ala Val Tyr Ile Val
                335                 340                 345
```

```
Met Ile Lys Arg Lys Val Asp Arg Glu Asp Lys Leu Asp Ile Pro
                350                 355                 360

Met Phe Phe Gly Phe Val Gly Leu Phe Asn Leu Leu Leu Leu Trp
                365                 370                 375

Pro Gly Phe Phe Leu Leu His Tyr Thr Gly Phe Glu Asp Phe Glu
                380                 385                 390

Phe Pro Asn Lys Val Val Leu Met Cys Ile Ile Ile Asn Gly Leu
                395                 400                 405

Ile Gly Thr Val Leu Ser Glu Phe Leu Trp Leu Trp Gly Cys Phe
                410                 415                 420

Leu Thr Ser Ser Leu Ile Gly Thr Leu Ala Leu Ser Leu Thr Ile
                425                 430                 435

Pro Leu Ser Ile Ile Ala Asp Met Cys Met Gln Lys Val Gln Phe
                440                 445                 450

Ser Trp Leu Phe Phe Ala Gly Ala Ile Pro Val Phe Phe Ser Phe
                455                 460                 465

Phe Ile Val Thr Leu Leu Cys His Tyr Asn Asn Trp Asp Pro Val
                470                 475                 480

Met Val Gly Ile Arg Arg Ile Phe Ala Phe Ile Cys Arg Lys His
                485                 490                 495

Arg Ile Gln Arg Val Pro Glu Asp Ser Glu Gln Cys Glu Ser Leu
                500                 505                 510

Ile Ser Met His Ser Val Ser Gln Glu Asp Gly Ala Ser
                515                 520
```

<210> 247
<211> 1123
<212> DNA
<213> Homo Sapien

<400> 247

```
cgtctgtaga gatatcatga acttcaactt agctttggta ctttcttccc 50

tgaagacaga gggcagaact ctgagttcca gaaccatttt caactgtatt 100

ggggaccaat cacttgactc tattcttgtc tctctgacag atgacgctac 150

actctcctct gaataatgga caccatttct aaaactgaat cctgctacta 200

aaataattca gatgatatat ttttccaatt ctacaatctt gctttgtttt 250

atttagttgt tttctctctc tcttcccagt tttccagaga ctggagctaa 300

actgggcttt caacatcatc atgaagttta tcctcctctg ggccctcttg 350

aatctgactg ttgctttggc ctttaatcca gattacacag tcagctccac 400

tccccttac ttggtctatt tgaaatctga ctacttgccc tgcgctggag 450

tcctgatcca cccgctttgg gtgatcacag ctgcacactg caatttacca 500

aagcttcggg tgatattggg ggttacaatc ccagcagact ctaatgaaaa 550
```

gcatctgcaa gtgattggct atgagaagat gattcatcat ccacacttct 600

cagtcacttc tattgatcat gacatcatgc taatcaagct gaaaacagag 650

gctgaactca atgactatgt gaaattagcc aacctgccct accaaactat 700

ctctgaaaat accatgtgct ctgtctctac ctggagctac aatgtgtgtg 750

atatctacaa agagcccgat tcactgcaaa ctgtgaacat ctctgtaatc 800

tccaagcctc agtgtcgcga tgcctataaa acctacaaca tcacggaaaa 850

tatgctgtgt gtgggcattg tgccaggaag gaggcagccc tgcaaggaag 900

tttctgctgc cccggcaatc tgcaatggga tgcttcaagg aatcctgtct 950

tttgcggatg gatgtgtttt gagagccgat gttggcatct atgccaaaat 1000

tttttactat ataccctgga ttgaaaatgt aatccaaaat aactgagctg 1050

tggcagttgt ggaccatatg acacagcttg tccccatcgt tcacctttag 1100

aattaaatat aaattaactc ctc 1123

<210> 248
<211> 241
<212> PRT
<213> Homo Sapien

<400> 248

```
Met Lys Phe Ile Leu Leu Trp Ala Leu Leu Asn Leu Thr Val Ala
1               5                   10                  15

Leu Ala Phe Asn Pro Asp Tyr Thr Val Ser Ser Thr Pro Pro Tyr
                20                  25                  30

Leu Val Tyr Leu Lys Ser Asp Tyr Leu Pro Cys Ala Gly Val Leu
                35                  40                  45

Ile His Pro Leu Trp Val Ile Thr Ala Ala His Cys Asn Leu Pro
                50                  55                  60

Lys Leu Arg Val Ile Leu Gly Val Thr Ile Pro Ala Asp Ser Asn
                65                  70                  75

Glu Lys His Leu Gln Val Ile Gly Tyr Glu Lys Met Ile His His
                80                  85                  90

Pro His Phe Ser Val Thr Ser Ile Asp His Asp Ile Met Leu Ile
                95                  100                 105

Lys Leu Lys Thr Glu Ala Glu Leu Asn Asp Tyr Val Lys Leu Ala
                110                 115                 120

Asn Leu Pro Tyr Gln Thr Ile Ser Glu Asn Thr Met Cys Ser Val
                125                 130                 135

Ser Thr Trp Ser Tyr Asn Val Cys Asp Ile Tyr Lys Glu Pro Asp
                140                 145                 150

Ser Leu Gln Thr Val Asn Ile Ser Val Ile Ser Lys Pro Gln Cys
                155                 160                 165
```

Arg Asp Ala Tyr Lys Thr Tyr Asn Ile Thr Glu Asn Met Leu Cys
170 175 180

Val Gly Ile Val Pro Gly Arg Arg Gln Pro Cys Lys Glu Val Ser
185 190 195

Ala Ala Pro Ala Ile Cys Asn Gly Met Leu Gln Gly Ile Leu Ser
200 205 210

Phe Ala Asp Gly Cys Val Leu Arg Ala Asp Val Gly Ile Tyr Ala
215 220 225

Lys Ile Phe Tyr Tyr Ile Pro Trp Ile Glu Asn Val Ile Gln Asn
230 235 240

Asn

<210> 249
<211> 526
<212> DNA
<213> Homo Sapien

<400> 249
gcgaggcggc cgctgtcttc tgctgcggct tccgcgacca caagtactgc 50

tgcgacgacc cgcacagctt cttcccctac gagcacagct acatgtggtg 100

gctcagcatt ggcgctctca taggcctgtc cgtagcagca gtggttcttc 150

tcgccttcat tgttaccgcc tgtgtgctct gctacctgtt catcagctct 200

aagccccaca caaagttgga cctgggcttg agcttacaga cagcaggccc 250

tgaggaggtt tctcctgact gccaaggtgt gaacacaggc atggcggcag 300

aagtgccaaa agtgagccct ctccagcaga gttactcctg cttgaacccg 350

cagctggaga gcaatgaggg gcaggctgtg aactccaaac gcctcctcca 400

tcattgcttc atggccacag tgaccaccag tgacattcca ggcagccctg 450

aggaagcctc tgtacccaac cctgacctat gtggaccagt cccataaaca 500

ttcaataaat gtctccatac catcaa 526

<210> 250
<211> 134
<212> PRT
<213> Homo Sapien

<400> 250
Met Trp Trp Leu Ser Ile Gly Ala Leu Ile Gly Leu Ser Val Ala
1 5 10 15

Ala Val Val Leu Leu Ala Phe Ile Val Thr Ala Cys Val Leu Cys
20 25 30

Tyr Leu Phe Ile Ser Ser Lys Pro His Thr Lys Leu Asp Leu Gly
35 40 45

Leu Ser Leu Gln Thr Ala Gly Pro Glu Glu Val Ser Pro Asp Cys
50 55 60

```
Gln Gly Val Asn Thr Gly Met Ala Ala Glu Val Pro Lys Val Ser
                65                  70                  75

Pro Leu Gln Gln Ser Tyr Ser Cys Leu Asn Pro Gln Leu Glu Ser
                80                  85                  90

Asn Glu Gly Gln Ala Val Asn Ser Lys Arg Leu Leu His His Cys
                95                  100                 105

Phe Met Ala Thr Val Thr Thr Ser Asp Ile Pro Gly Ser Pro Glu
                110                 115                 120

Glu Ala Ser Val Pro Asn Pro Asp Leu Cys Gly Pro Val Pro
                125                 130
```

<210> 251
<211> 1714
<212> DNA
<213> Homo Sapien

<400> 251

```
gtggtttgga ttgagccggg cccggccggg gcgccgagtc ggagggggtg 50

gcagtgagcg gcggcagagg ctacggggct cggtttggct gactggggag 100

tcggcaggcg gcaggaacca tgcgaggcca gcggagcctg ctgctgggcc 150

cggcccgcct ctgcctccgc ctccttctgc tgctgggtta caggcgccgc 200

tgtccacctc tactccgggg tctagtacag cgctggcgct acggcaaggt 250

ctgcctgcgc tccctgctct acaactcctt tgggggcagt gacaccgctg 300

ttgatgctgc ctttgagcct gtctactggc tggtagacaa cgtgatccgc 350

tggtttggag tggtgttcgt ggtcctggtg atcgtgctga caggctccat 400

tgtagctatc gcctacctgt gtgtcctgcc tctcatcctc cgaacctact 450

cagtgccacg actctgctgg catttcttct atagccactg gaatctgatc 500

ctgattgtct tccactacta ccaggccatc accactccgc ctgggtaccc 550

accccagggc aggaatgata tcgccaccgt ctccatctgt aagaagtgca 600

tttaccccaa gccagcccga acacaccact gcagcatctg caacaggtgt 650

gtgctgaaga tggatcacca ctgcccctgg ctaaacaatt gtgtgggcca 700

ctataaccat cggtacttct tctctttctg ctttttcatg actctgggct 750

gtgtctactg cagctatgga agttgggacc ttttccggga ggcttatgct 800

gccattgaga cttatcacca gaccccacca cccaccttct cctttcgaga 850

aaggatgact cacaagagtc ttgtctacct ctggttcctg tgcagttctg 900

tggcacttgc cctgggtgcc ctaactgtat ggcatgctgt tctcatcagt 950

cgaggtgaga ctagcatcga aaggcacatc aacaagaagg agagacgtcg 1000

gctacaggcc aagggcagag tatttaggaa tccttacaac tacggctgct 1050

tggacaactg gaaggtattc ctgggtgtgg atacaggaag gcactggctt 1100
```

actcgggtgc tcttaccttc tagtcacttg ccccatggga atggaatgag 1150

ctgggagccc cctccctggg tgactgctca ctcagcctct gtgatggcag 1200

tgtgagctgg actgtgtcag ccacgactcg agcactcatt ctgctcccta 1250

tgttatttca agggcctcca agggcagctt ttctcagaat ccttgatcaa 1300

aaagagccag tgggcctgcc ttagggtacc atgcaggaca attcaaggac 1350

cagccttttt accactgcag aagaaagaca caatgtggag aaatcttagg 1400

actgacatcc ctttactcag gcaaacagaa gttccaaccc cagactaggg 1450

gtcaggcagc tagctaccta ccttgcccag tgctgacccg gacctcctcc 1500

aggatacagc actggagttg gccaccacct cttctacttg ctgtctgaaa 1550

aaacacctga ctagtacagc tgagatcttg gcttctcaac agggcaaaga 1600

taccaggcct gctgctgagg tcactgccac ttctcacatg ctgcttaagg 1650

gagcacaaat aaaggtattc gatttttaaa aaaaaaaaaa aaaaaaaaaa 1700

aaaaaaaaaa aaaa 1714

<210> 252
<211> 361
<212> PRT
<213> Homo Sapien

<400> 252

```
Met Arg Gly Gln Arg Ser Leu Leu Leu Gly Pro Ala Arg Leu Cys
 1               5                  10                  15

Leu Arg Leu Leu Leu Leu Leu Gly Tyr Arg Arg Arg Cys Pro Pro
                20                  25                  30

Leu Leu Arg Gly Leu Val Gln Arg Trp Arg Tyr Gly Lys Val Cys
                35                  40                  45

Leu Arg Ser Leu Leu Tyr Asn Ser Phe Gly Gly Ser Asp Thr Ala
                50                  55                  60

Val Asp Ala Ala Phe Glu Pro Val Tyr Trp Leu Val Asp Asn Val
                65                  70                  75

Ile Arg Trp Phe Gly Val Val Phe Val Val Leu Val Ile Val Leu
                80                  85                  90

Thr Gly Ser Ile Val Ala Ile Ala Tyr Leu Cys Val Leu Pro Leu
                95                 100                 105

Ile Leu Arg Thr Tyr Ser Val Pro Arg Leu Cys Trp His Phe Phe
               110                 115                 120

Tyr Ser His Trp Asn Leu Ile Leu Ile Val Phe His Tyr Tyr Gln
               125                 130                 135

Ala Ile Thr Thr Pro Pro Gly Tyr Pro Pro Gln Gly Arg Asn Asp
               140                 145                 150

Ile Ala Thr Val Ser Ile Cys Lys Lys Cys Ile Tyr Pro Lys Pro
```

155 160 165

Ala Arg Thr His His Cys Ser Ile Cys Asn Arg Cys Val Leu Lys
170 175 180

Met Asp His His Cys Pro Trp Leu Asn Asn Cys Val Gly His Tyr
185 190 195

Asn His Arg Tyr Phe Phe Ser Phe Cys Phe Phe Met Thr Leu Gly
200 205 210

Cys Val Tyr Cys Ser Tyr Gly Ser Trp Asp Leu Phe Arg Glu Ala
215 220 225

Tyr Ala Ala Ile Glu Thr Tyr His Gln Thr Pro Pro Pro Thr Phe
230 235 240

Ser Phe Arg Glu Arg Met Thr His Lys Ser Leu Val Tyr Leu Trp
245 250 255

Phe Leu Cys Ser Ser Val Ala Leu Ala Leu Gly Ala Leu Thr Val
260 265 270

Trp His Ala Val Leu Ile Ser Arg Gly Glu Thr Ser Ile Glu Arg
275 280 285

His Ile Asn Lys Lys Glu Arg Arg Arg Leu Gln Ala Lys Gly Arg
290 295 300

Val Phe Arg Asn Pro Tyr Asn Tyr Gly Cys Leu Asp Asn Trp Lys
305 310 315

Val Phe Leu Gly Val Asp Thr Gly Arg His Trp Leu Thr Arg Val
320 325 330

Leu Leu Pro Ser Ser His Leu Pro His Gly Asn Gly Met Ser Trp
335 340 345

Glu Pro Pro Pro Trp Val Thr Ala His Ser Ala Ser Val Met Ala
350 355 360

Val

<210> 253
<211> 2016
<212> DNA
<213> Homo Sapien

<400> 253
gatcaagcgc cttcctttcc cttcctctcc ctacttggcc tttgccctaa 50

gccaagacct ggccatcagc ctggctgcag gggcctgcag agccagctgc 100

actttttcag gtatggggga gggccaggca ccatgaagcc agtgtgggtc 150

gccacccttc tgtggatgct actgctggtg cccaggctgg gggccgcccg 200

gaaggggtcc ccagaagagg cctccttcta ctatggaacc ttccctcttg 250

gcttctcctg gggcgtgggc agttctgcct accagacgga gggcgcctgg 300

gaccaggacg ggaaagggcc tagcatctgg gacgtcttca cacacagtgg 350

gaaggggaaa gtgcttggga atgagacggc agatgtagcc tgtgacggct 400
actacaaggt ccaggaggac atcattctgc tgagggaact gcacgtcaac 450
cactaccgat tctccctgtc ttggccccgg ctcctgccca caggcatccg 500
agccgagcag gtgaacaaga agggaatcga attctacagt gatcttatcg 550
atgcccttct gagcagcaac atcactccca tcgtgacctt gcaccactgg 600
gatctgccac agctgctcca ggtcaaatac ggtgggtggc agaatgtgag 650
catggccaac tacttcagag actacgccaa cctgtgcttt gaggcctttg 700
gggaccgtgt gaagcactgg atcacgttca gtgatcctcg ggcaatggca 750
gaaaaaggct atgagacggg ccaccatgcg ccgggcctga agctccgcgg 800
caccggcctg tacaaggcag cacaccacat cattaaggcc cacgccaaaa 850
cctggcattc ttataacacc acgtggcgca gcaagcagca aggtctggtg 900
ggaatttcac tgaactgtga ctggggggaa cctgtggaca ttagtaaccc 950
caaggaccta gaggctgccg agagatacct acagttctgt ctgggctggt 1000
ttgccaaccc catttatgcc ggtgactacc cccaagtcat gaaggactac 1050
attggaagaa agagtgcaga gcaaggcctg gagatgtcga ggttaccggt 1100
gttctcactc caggagaaga gctacattaa aggcacatcc gatttcttgg 1150
gattaggtca ttttactact cggtacatca cggaaaggaa ctacccctcc 1200
cgccaggggc ccagctacca gaacgatcgt gacttgatag agctggttga 1250
cccaaactgg ccagatctgg ggtctaaatg gctatattct gtgccatggg 1300
gatttaggag gctccttaac tttgctcaga ctcaatacgg tgatcctccc 1350
atatatgtga tggaaaatgg agcatctcaa aaattccact gtactcaatt 1400
atgtgatgag tggagaattc aataccttaa aggatacata aatgaaatgc 1450
taaaagctat aaaagatggt gctaatataa aggggtatac ttcctggtct 1500
ctgttggata agtttgaatg ggagaaagga tactcagata gatatggatt 1550
ctactatgtt gaatttaacg acagaaataa gcctcgctat ccaaaggctt 1600
cagttcaata ttacaagaag attatcattg ccaatgggtt tcccaatcca 1650
agagaggtgg aaagttggta cctcaaagct ttggaaactt gctctatcaa 1700
caatcagatg cttgctgcag agcctttgct aagtcacatg caaatggtta 1750
cggagatcgt ggtacccact gtctgctccc tctgtgtcct catcactgct 1800
gttctactaa tgctcctcct gaggaggcag agctgagaca ggattatcaa 1850
ttttggagct tcataagaga atcttcagga tcttcctccc ttttctgctt 1900
tgagggtttc catacattgc tgttttcagg ttctacaata attacctttt 1950

tttctctttc tctttttggc ttgtgctggg atttaagaat tagaaaataa 2000

aaataagcag aaatta 2016

<210> 254
<211> 567
<212> PRT
<213> Homo Sapien

<400> 254
Met Lys Pro Val Trp Val Ala Thr Leu Leu Trp Met Leu Leu Leu
1               5                   10                  15

Val Pro Arg Leu Gly Ala Ala Arg Lys Gly Ser Pro Glu Glu Ala
            20                  25                  30

Ser Phe Tyr Tyr Gly Thr Phe Pro Leu Gly Phe Ser Trp Gly Val
        35                  40                  45

Gly Ser Ser Ala Tyr Gln Thr Glu Gly Ala Trp Asp Gln Asp Gly
    50                  55                  60

Lys Gly Pro Ser Ile Trp Asp Val Phe Thr His Ser Gly Lys Gly
65                  70                  75

Lys Val Leu Gly Asn Glu Thr Ala Asp Val Ala Cys Asp Gly Tyr
80                  85                  90

Tyr Lys Val Gln Glu Asp Ile Ile Leu Leu Arg Glu Leu His Val
95                  100                 105

Asn His Tyr Arg Phe Ser Leu Ser Trp Pro Arg Leu Leu Pro Thr
110                 115                 120

Gly Ile Arg Ala Glu Gln Val Asn Lys Lys Gly Ile Glu Phe Tyr
125                 130                 135

Ser Asp Leu Ile Asp Ala Leu Leu Ser Ser Asn Ile Thr Pro Ile
140                 145                 150

Val Thr Leu His His Trp Asp Leu Pro Gln Leu Leu Gln Val Lys
155                 160                 165

Tyr Gly Gly Trp Gln Asn Val Ser Met Ala Asn Tyr Phe Arg Asp
170                 175                 180

Tyr Ala Asn Leu Cys Phe Glu Ala Phe Gly Asp Arg Val Lys His
185                 190                 195

Trp Ile Thr Phe Ser Asp Pro Arg Ala Met Ala Glu Lys Gly Tyr
200                 205                 210

Glu Thr Gly His His Ala Pro Gly Leu Lys Leu Arg Gly Thr Gly
215                 220                 225

Leu Tyr Lys Ala Ala His His Ile Ile Lys Ala His Ala Lys Thr
230                 235                 240

Trp His Ser Tyr Asn Thr Thr Trp Arg Ser Lys Gln Gln Gly Leu
245                 250                 255

Val Gly Ile Ser Leu Asn Cys Asp Trp Gly Glu Pro Val Asp Ile
260                 265                 270

```
Ser Asn Pro Lys Asp Leu Glu Ala Ala Glu Arg Tyr Leu Gln Phe
                275                 280                 285

Cys Leu Gly Trp Phe Ala Asn Pro Ile Tyr Ala Gly Asp Tyr Pro
                290                 295                 300

Gln Val Met Lys Asp Tyr Ile Gly Arg Lys Ser Ala Glu Gln Gly
                305                 310                 315

Leu Glu Met Ser Arg Leu Pro Val Phe Ser Leu Gln Glu Lys Ser
                320                 325                 330

Tyr Ile Lys Gly Thr Ser Asp Phe Leu Gly Leu Gly His Phe Thr
                335                 340                 345

Thr Arg Tyr Ile Thr Glu Arg Asn Tyr Pro Ser Arg Gln Gly Pro
                350                 355                 360

Ser Tyr Gln Asn Asp Arg Asp Leu Ile Glu Leu Val Asp Pro Asn
                365                 370                 375

Trp Pro Asp Leu Gly Ser Lys Trp Leu Tyr Ser Val Pro Trp Gly
                380                 385                 390

Phe Arg Arg Leu Leu Asn Phe Ala Gln Thr Gln Tyr Gly Asp Pro
                395                 400                 405

Pro Ile Tyr Val Met Glu Asn Gly Ala Ser Gln Lys Phe His Cys
                410                 415                 420

Thr Gln Leu Cys Asp Glu Trp Arg Ile Gln Tyr Leu Lys Gly Tyr
                425                 430                 435

Ile Asn Glu Met Leu Lys Ala Ile Lys Asp Gly Ala Asn Ile Lys
                440                 445                 450

Gly Tyr Thr Ser Trp Ser Leu Leu Asp Lys Phe Glu Trp Glu Lys
                455                 460                 465

Gly Tyr Ser Asp Arg Tyr Gly Phe Tyr Tyr Val Glu Phe Asn Asp
                470                 475                 480

Arg Asn Lys Pro Arg Tyr Pro Lys Ala Ser Val Gln Tyr Tyr Lys
                485                 490                 495

Lys Ile Ile Ile Ala Asn Gly Phe Pro Asn Pro Arg Glu Val Glu
                500                 505                 510

Ser Trp Tyr Leu Lys Ala Leu Glu Thr Cys Ser Ile Asn Asn Gln
                515                 520                 525

Met Leu Ala Ala Glu Pro Leu Leu Ser His Met Gln Met Val Thr
                530                 535                 540

Glu Ile Val Val Pro Thr Val Cys Ser Leu Cys Val Leu Ile Thr
                545                 550                 555

Ala Val Leu Leu Met Leu Leu Leu Arg Arg Gln Ser
                560                 565
```

<210> 255
<211> 1432
<212> DNA
<213> Homo Sapien

<400> 255
cgcgaagatg cgaaaggtgg ttttgatcac cggggctagc agtggcattg 50
gcctggccct ctgcaagcgg ctgctggcgg aagatgatga gcttcatctg 100
tgtttggcgt gcaggaacat gagcaaggca gaagctgtct gtgctgctct 150
gctggcctct caccccactg ctgaggtcac cattgtccag gtggatgtca 200
gcaacctgca gtcggtcttc cgggcctcca aggaacttaa gcaaaggttt 250
cagagattag actgtatata tctaaatgct gggatcatgc ctaatccaca 300
actaaatatc aaagcacttt tctttggcct cttttcaaga aaagtgattc 350
atatgttctc cacagctgaa ggcctgctga cccagggtga taagatcact 400
gctgatggac ttcaggaggt gtttgagacc aatgtctttg gccattttat 450
cctgattcgg gaactggagc ctctcctctg tcacagtgac aatccatctc 500
agctcatctg gacatcatct cgcagtgcaa ggaaatctaa tttcagcctc 550
gaggacttcc agcacagcaa aggcaaggaa ccctacagct cttccaaata 600
tgccactgac cttttgagtg tggctttgaa caggaacttc aaccagcagg 650
gtctctattc caatgtggcc tgtccaggta cagcattgac caatttgaca 700
tatggaattc tgcctccgtt tatatggacg ctgttgatgc cggcaatatt 750
gctacttcgc ttttttgcaa atgcattcac tttgacacca tataatggaa 800
cagaagctct ggtatggctt ttccaccaaa agcctgaatc tctcaatcct 850
ctgatcaaat atctgagtgc caccactggc tttggaagaa attatattat 900
gacccagaag atggacctag atgaagacac tgctgaaaaa ttttatcaaa 950
agttactgga actggaaaag cacattaggg tcactattca aaaaacagat 1000
aatcaggcca ggctcagtgg ctcatgccta taattccagc actttgggag 1050
gccaaggcag aaggatcact tgagaccagg agttcaagac cagcctgaga 1100
aacatagtga gcccttgtct ctacaaaaag aaataaaaat aatagctggg 1150
tgtggtggca tgcgcatgta gtcccagcta ctcagaagga tgaggtggga 1200
ggatctcttg aggctgggag gcagaggttg cagtgagctg agattgtgcc 1250
actgcactcc agcctgggtg acagcgagac cctgtctcaa aatatgtata 1300
tatttaatat atatataaaa ccagagctga caatgacact ctggaacatt 1350
gcataccttc tgtacattct ggggtacatg gatttctact gagttggata 1400
atatgcattt gtaataaact atgaactatg aa 1432

<210> 256
<211> 341
<212> PRT
<213> Homo Sapien

<400> 256

Met Arg Lys Val Val Leu Ile Thr Gly Ala Ser Ser Gly Ile Gly
1 5 10 15

Leu Ala Leu Cys Lys Arg Leu Leu Ala Glu Asp Asp Glu Leu His
20 25 30

Leu Cys Leu Ala Cys Arg Asn Met Ser Lys Ala Glu Ala Val Cys
35 40 45

Ala Ala Leu Leu Ala Ser His Pro Thr Ala Glu Val Thr Ile Val
50 55 60

Gln Val Asp Val Ser Asn Leu Gln Ser Val Phe Arg Ala Ser Lys
65 70 75

Glu Leu Lys Gln Arg Phe Gln Arg Leu Asp Cys Ile Tyr Leu Asn
80 85 90

Ala Gly Ile Met Pro Asn Pro Gln Leu Asn Ile Lys Ala Leu Phe
95 100 105

Phe Gly Leu Phe Ser Arg Lys Val Ile His Met Phe Ser Thr Ala
110 115 120

Glu Gly Leu Leu Thr Gln Gly Asp Lys Ile Thr Ala Asp Gly Leu
125 130 135

Gln Glu Val Phe Glu Thr Asn Val Phe Gly His Phe Ile Leu Ile
140 145 150

Arg Glu Leu Glu Pro Leu Leu Cys His Ser Asp Asn Pro Ser Gln
155 160 165

Leu Ile Trp Thr Ser Ser Arg Ser Ala Arg Lys Ser Asn Phe Ser
170 175 180

Leu Glu Asp Phe Gln His Ser Lys Gly Lys Glu Pro Tyr Ser Ser
185 190 195

Ser Lys Tyr Ala Thr Asp Leu Leu Ser Val Ala Leu Asn Arg Asn
200 205 210

Phe Asn Gln Gln Gly Leu Tyr Ser Asn Val Ala Cys Pro Gly Thr
215 220 225

Ala Leu Thr Asn Leu Thr Tyr Gly Ile Leu Pro Pro Phe Ile Trp
230 235 240

Thr Leu Leu Met Pro Ala Ile Leu Leu Leu Arg Phe Phe Ala Asn
245 250 255

Ala Phe Thr Leu Thr Pro Tyr Asn Gly Thr Glu Ala Leu Val Trp
260 265 270

Leu Phe His Gln Lys Pro Glu Ser Leu Asn Pro Leu Ile Lys Tyr
275 280 285

Leu Ser Ala Thr Thr Gly Phe Gly Arg Asn Tyr Ile Met Thr Gln
290 295 300

Lys Met Asp Leu Asp Glu Asp Thr Ala Glu Lys Phe Tyr Gln Lys
305 310 315

```
Leu Leu Glu Leu Glu Lys His Ile Arg Val Thr Ile Gln Lys Thr
                320                 325                 330

Asp Asn Gln Ala Arg Leu Ser Gly Ser Cys Leu
                335                 340
```

<210> 257
<211> 1606
<212> DNA
<213> Homo Sapien

<400> 257

```
cggacgcgtg gggccgtatg cgcggctctg tggagtgcac ctggggttgg 50

gggcactgtg cccccagccc cctgctcctt tggactctac ttctgtttgc 100

agccccattt ggcctgctgg gggagaagac ccgccaggtg tctctggagg 150

tcatccctaa ctggctgggc cccctgcaga acctgcttca tatacgggca 200

gtgggcacca attccacact gcactatgtg tggagcagcc tggggcctct 250

ggcagtggta atggtggcca ccaacacccc ccacagcacc ctgagcatca 300

actggagcct cctgctatcc cctgagcccg atgggggcct gatggtgctc 350

cctaaggaca gcattcagtt ttcttctgcc cttgttttta ccaggctgct 400

tgagtttgac agcaccaacg tgtccgatac ggcagcaaag cctttgggaa 450

gaccatatcc tccatactcc ttggccgatt tctcttggaa caacatcact 500

gattcattgg atcctgccac cctgagtgcc acatttcaag gccaccccat 550

gaacgaccct accaggactt ttgccaatgg cagcctggcc ttcagggtcc 600

aggccttttc caggtccagc cgaccagccc aaccccctcg cctcctgcac 650

acagcagaca cctgtcagct agaggtggcc ctgattggag cctctccccg 700

gggaaaccgt tccctgtttg ggctggaggt agccacattg ggccagggcc 750

ctgactgccc ctcaatgcag gagcagcact ccatcgacga tgaatatgca 800

ccggccgtct tccagttgga ccagctactg tggggctccc tcccatcagg 850

ctttgcacag tggcgaccag tggcttactc ccagaagccg gggggccgag 900

aatcagccct gccctgccaa gcttcccctc ttcatcctgc cttagcatac 950

tctcttcccc agtcacccat tgtccgagcc ttctttgggt cccagaataa 1000

cttctgtgcc ttcaatctga cgttcggggc ttccacaggc cctggctatt 1050

gggaccaaca ctacctcagc tggtcgatgc tcctgggtgt gggcttccct 1100

ccagtggacg gcttgtcccc actagtcctg ggcatcatgg cagtggccct 1150

gggtgcccca gggctcatgc tgctaggggg cggcttggtt ctgctgctgc 1200

accacaagaa gtactcagag taccagtcca taaattaagg cccgctctct 1250

ggagggaagg acattactga acctgtcttg ctgtgcctcg aaactctgga 1300
```

ggttggagca tcaagttcca gccggcccct tcactccccc atcttgcttt 1350

tctgtggaac ctcagaggcc agcctcgact tcctggagac ccccaggtgg 1400

ggcttccttc atactttgtt gggggacttt ggaggcgggc aggggacagg 1450

gctattgata aggtcccctt ggtgttgcct tcttgcatct ccacacattt 1500

cccttggatg ggacttgcag gcctaaatga gaggcattct gactggttgg 1550

ctgccctgga aggcaagaaa atagatttat ttttttcac agggaaaaaa 1600

aaaaaa 1606

<210> 258
<211> 406
<212> PRT
<213> Homo Sapien

<400> 258

```
Met Arg Gly Ser Val Glu Cys Thr Trp Gly Trp Gly His Cys Ala
  1               5                  10                  15

Pro Ser Pro Leu Leu Leu Trp Thr Leu Leu Leu Phe Ala Ala Pro
                 20                  25                  30

Phe Gly Leu Leu Gly Glu Lys Thr Arg Gln Val Ser Leu Glu Val
                 35                  40                  45

Ile Pro Asn Trp Leu Gly Pro Leu Gln Asn Leu Leu His Ile Arg
                 50                  55                  60

Ala Val Gly Thr Asn Ser Thr Leu His Tyr Val Trp Ser Ser Leu
                 65                  70                  75

Gly Pro Leu Ala Val Val Met Val Ala Thr Asn Thr Pro His Ser
                 80                  85                  90

Thr Leu Ser Ile Asn Trp Ser Leu Leu Leu Ser Pro Glu Pro Asp
                 95                 100                 105

Gly Gly Leu Met Val Leu Pro Lys Asp Ser Ile Gln Phe Ser Ser
                110                 115                 120

Ala Leu Val Phe Thr Arg Leu Leu Glu Phe Asp Ser Thr Asn Val
                125                 130                 135

Ser Asp Thr Ala Ala Lys Pro Leu Gly Arg Pro Tyr Pro Pro Tyr
                140                 145                 150

Ser Leu Ala Asp Phe Ser Trp Asn Asn Ile Thr Asp Ser Leu Asp
                155                 160                 165

Pro Ala Thr Leu Ser Ala Thr Phe Gln Gly His Pro Met Asn Asp
                170                 175                 180

Pro Thr Arg Thr Phe Ala Asn Gly Ser Leu Ala Phe Arg Val Gln
                185                 190                 195

Ala Phe Ser Arg Ser Ser Arg Pro Ala Gln Pro Pro Arg Leu Leu
                200                 205                 210

His Thr Ala Asp Thr Cys Gln Leu Glu Val Ala Leu Ile Gly Ala
```

215 220 225

Ser Pro Arg Gly Asn Arg Ser Leu Phe Gly Leu Glu Val Ala Thr
230 235 240

Leu Gly Gln Gly Pro Asp Cys Pro Ser Met Gln Glu Gln His Ser
245 250 255

Ile Asp Asp Glu Tyr Ala Pro Ala Val Phe Gln Leu Asp Gln Leu
260 265 270

Leu Trp Gly Ser Leu Pro Ser Gly Phe Ala Gln Trp Arg Pro Val
275 280 285

Ala Tyr Ser Gln Lys Pro Gly Gly Arg Glu Ser Ala Leu Pro Cys
290 295 300

Gln Ala Ser Pro Leu His Pro Ala Leu Ala Tyr Ser Leu Pro Gln
305 310 315

Ser Pro Ile Val Arg Ala Phe Phe Gly Ser Gln Asn Asn Phe Cys
320 325 330

Ala Phe Asn Leu Thr Phe Gly Ala Ser Thr Gly Pro Gly Tyr Trp
335 340 345

Asp Gln His Tyr Leu Ser Trp Ser Met Leu Leu Gly Val Gly Phe
350 355 360

Pro Pro Val Asp Gly Leu Ser Pro Leu Val Leu Gly Ile Met Ala
365 370 375

Val Ala Leu Gly Ala Pro Gly Leu Met Leu Leu Gly Gly Gly Leu
380 385 390

Val Leu Leu Leu His His Lys Lys Tyr Ser Glu Tyr Gln Ser Ile
395 400 405

Asn

<210> 259
<211> 2024
<212> DNA
<213> Homo Sapien

<400> 259
caggcgggcc cccgcgcggc agggccctgg acccgcgcgg ctcccgggga 50

tggtgagcaa ggcgctgctg cgcctcgtgt ctgccgtcaa ccgcaggagg 100

atgaagctgc tgctgggcat cgccttgctg gcctacgtcg cctctgtttg 150

gggcaacttc gttaatatga ggtctatcca ggaaaatggt gaactaaaaa 200

ttgaaagcaa gattgaagag atggttgaac cactaagaga gaaaatcaga 250

gatttagaaa aaagctttac ccagaaatac ccaccagtaa agtttttatc 300

agaaaaggat cggaaaagaa ttttgataac aggaggcgca gggttcgtgg 350

gctcccatct aactgacaaa ctcatgatgg acggccacga ggtgaccgtg 400

gtggacaatt tcttcacggg caggaagaga aacgtggagc actggatcgg 450

acatgagaac ttcgagttga ttaaccacga cgtggtggag cccctctaca 500
tcgaggttga ccagatatac catctggcat ctccagcctc ccctccaaac 550
tacatgtata atcctatcaa gacattaaag accaatacga ttgggacatt 600
aaacatgttg gggctggcaa aacgagtcgg tgcccgtctg ctcctggcct 650
ccacatcgga ggtgtatgga gatcctgaag tccaccctca aagtgaggat 700
tactggggcc acgtgaatcc aataggacct cgggcctgct acgatgaagg 750
caaacgtgtt gcagagacca tgtgctatgc ctacatgaag caggaaggcg 800
tggaagtgcg agtggccaga atcttcaaca cctttgggcc acgcatgcac 850
atgaacgatg ggcgagtagt cagcaacttc atcctgcagg cgctccaggg 900
ggagccactc acggtatacg gatccgggtc tcagacaagg gcgttccagt 950
acgtcagcga tctagtgaat ggcctcgtgg ctctcatgaa cagcaacgtc 1000
agcagcccgg tcaacctggg gaacccagaa gaacacacaa tcctagaatt 1050
tgctcagtta attaaaaacc ttgttggtag cggaagtgaa attcagtttc 1100
tctccgaagc ccaggatgac ccacagaaaa gaaaaccaga catcaaaaaa 1150
gcaaagctga tgctggggtg ggagcccgtg gtcccgctgg aggaaggttt 1200
aaacaaagca attcactact tccgtaaaga actcgagtac caggcaaata 1250
atcagtacat ccccaaacca aagcctgcca gaataaagaa aggacggact 1300
cgccacagct gaactcctca cttttaggac acaagactac cattgtacac 1350
ttgatgggat gtatttttgg cttttttttg ttgtcgttta aagaaagact 1400
ttaacaggtg tcatgaagaa caaactggaa tttcattctg aagcttgctt 1450
taatgaaatg gatgtgccta aaagctcccc tcaaaaaact gcagattttg 1500
ccttgcactt tttgaatctc tctttttatg taaaatagcg tagatgcatc 1550
tctgcgtatt ttcaagtttt tttatcttgc tgtgagagca tatgttgtga 1600
ctgtcgttga cagttttatt tactggtttc tttgtgaagc tgaaaaggaa 1650
cattaagcgg gacaaaaaat gccgatttta tttataaaag tgggtactta 1700
ataaatgagt cgttatacta tgcataaaga aaaatcctag cagtattgtc 1750
aggtggtggt gcgccggcat tgattttagg gcagataaaa gaattctgtg 1800
tgagagcttt atgtttctct tttaattcag agtttttcca aggtctactt 1850
ttgagttgca aacttgactt tgaaatattc ctgttggtca tgatcaagga 1900
tatttgaaat cactactgtg ttttgctgcg tatctggggc gggggcaggt 1950
tggggggcac aaagttaaca tattcttggt taaccatggt taaatatgct 2000
attttaataa aatattgaaa ctca 2024

<210> 260
<211> 420
<212> PRT
<213> Homo Sapien

<400> 260

```
Met Val Ser Lys Ala Leu Leu Arg Leu Val Ser Ala Val Asn Arg
  1               5                  10                  15

Arg Arg Met Lys Leu Leu Leu Gly Ile Ala Leu Leu Ala Tyr Val
                 20                  25                  30

Ala Ser Val Trp Gly Asn Phe Val Asn Met Arg Ser Ile Gln Glu
                 35                  40                  45

Asn Gly Glu Leu Lys Ile Glu Ser Lys Ile Glu Glu Met Val Glu
                 50                  55                  60

Pro Leu Arg Glu Lys Ile Arg Asp Leu Glu Lys Ser Phe Thr Gln
                 65                  70                  75

Lys Tyr Pro Pro Val Lys Phe Leu Ser Glu Lys Asp Arg Lys Arg
                 80                  85                  90

Ile Leu Ile Thr Gly Gly Ala Gly Phe Val Gly Ser His Leu Thr
                 95                 100                 105

Asp Lys Leu Met Met Asp Gly His Glu Val Thr Val Val Asp Asn
                110                 115                 120

Phe Phe Thr Gly Arg Lys Arg Asn Val Glu His Trp Ile Gly His
                125                 130                 135

Glu Asn Phe Glu Leu Ile Asn His Asp Val Val Glu Pro Leu Tyr
                140                 145                 150

Ile Glu Val Asp Gln Ile Tyr His Leu Ala Ser Pro Ala Ser Pro
                155                 160                 165

Pro Asn Tyr Met Tyr Asn Pro Ile Lys Thr Leu Lys Thr Asn Thr
                170                 175                 180

Ile Gly Thr Leu Asn Met Leu Gly Leu Ala Lys Arg Val Gly Ala
                185                 190                 195

Arg Leu Leu Leu Ala Ser Thr Ser Glu Val Tyr Gly Asp Pro Glu
                200                 205                 210

Val His Pro Gln Ser Glu Asp Tyr Trp Gly His Val Asn Pro Ile
                215                 220                 225

Gly Pro Arg Ala Cys Tyr Asp Glu Gly Lys Arg Val Ala Glu Thr
                230                 235                 240

Met Cys Tyr Ala Tyr Met Lys Gln Glu Gly Val Glu Val Arg Val
                245                 250                 255

Ala Arg Ile Phe Asn Thr Phe Gly Pro Arg Met His Met Asn Asp
                260                 265                 270

Gly Arg Val Val Ser Asn Phe Ile Leu Gln Ala Leu Gln Gly Glu
                275                 280                 285
```

```
Pro Leu Thr Val Tyr Gly Ser Gly Ser Gln Thr Arg Ala Phe Gln
                290                 295                 300

Tyr Val Ser Asp Leu Val Asn Gly Leu Val Ala Leu Met Asn Ser
                305                 310                 315

Asn Val Ser Ser Pro Val Asn Leu Gly Asn Pro Glu Glu His Thr
                320                 325                 330

Ile Leu Glu Phe Ala Gln Leu Ile Lys Asn Leu Val Gly Ser Gly
                335                 340                 345

Ser Glu Ile Gln Phe Leu Ser Glu Ala Gln Asp Asp Pro Gln Lys
                350                 355                 360

Arg Lys Pro Asp Ile Lys Lys Ala Lys Leu Met Leu Gly Trp Glu
                365                 370                 375

Pro Val Val Pro Leu Glu Glu Gly Leu Asn Lys Ala Ile His Tyr
                380                 385                 390

Phe Arg Lys Glu Leu Glu Tyr Gln Ala Asn Asn Gln Tyr Ile Pro
                395                 400                 405

Lys Pro Lys Pro Ala Arg Ile Lys Lys Gly Arg Thr Arg His Ser
                410                 415                 420
```

<210> 261
<211> 882
<212> DNA
<213> Homo Sapien

<400> 261

```
gcgtggtgcg ggggcgtggg gaaatcgggt tgccccagcc gttactggtc 50
cgcgcagtca gggcatcctc cgcatcctcc acatccttcc atggctctga 100
agaataaatt cagttgttta tggatcttgg gtctgtgttt ggtagccact 150
acatcttcca aaatcccatc catcactgac ccacacttta tagacaactg 200
catagaagcc cacaacgaat ggcgtggcaa agtcaacccct cccgcggccg 250
acatgaaata catgatttgg gataaaggtt tagcaaagat ggctaaagca 300
tgggcaaacc agtgcaaatt tgaacataat gactgtttgg ataaatcata 350
taaatgctat gcagcttttg aatatgttgg agaaaatatc tggttaggtg 400
gaataaagtc attcacacca agacatgcca ttacggcttg gtataatgaa 450
acccaatttt atgattttga tagtctatca tgctccagag tctgtggcca 500
ttatacacag ttagtttggg ccaattcatt ttatgtcggt tgtgcagttg 550
caatgtgtcc taaccttggg ggagcttcaa ctgcaatatt tgtatgcaac 600
tacggacctg caggaaattt tgcaaatatg cctccttacg caagaggaga 650
atcttgctct ctctgctcaa aagaagagaa atgtgtaaag aacctctgca 700
ggactccaca acttattata cctaaccaaa atccatttct gaagccaacg 750
gggagagcac ctcagcagac agcctttaat ccattcagct taggttttct 800
```

tcttctgaga atcttttaat gtcatttata tacaaaagaa attctcaaat 850

gttaaaataa aggaatagtt tattgcttaa ta 882

<210> 262
<211> 242
<212> PRT
<213> Homo Sapien

<400> 262

```
Met Ala Leu Lys Asn Lys Phe Ser Cys Leu Trp Ile Leu Gly Leu
  1               5                  10                  15

Cys Leu Val Ala Thr Thr Ser Ser Lys Ile Pro Ser Ile Thr Asp
                 20                  25                  30

Pro His Phe Ile Asp Asn Cys Ile Glu Ala His Asn Glu Trp Arg
                 35                  40                  45

Gly Lys Val Asn Pro Pro Ala Ala Asp Met Lys Tyr Met Ile Trp
                 50                  55                  60

Asp Lys Gly Leu Ala Lys Met Ala Lys Ala Trp Ala Asn Gln Cys
                 65                  70                  75

Lys Phe Glu His Asn Asp Cys Leu Asp Lys Ser Tyr Lys Cys Tyr
                 80                  85                  90

Ala Ala Phe Glu Tyr Val Gly Glu Asn Ile Trp Leu Gly Gly Ile
                 95                 100                 105

Lys Ser Phe Thr Pro Arg His Ala Ile Thr Ala Trp Tyr Asn Glu
                110                 115                 120

Thr Gln Phe Tyr Asp Phe Asp Ser Leu Ser Cys Ser Arg Val Cys
                125                 130                 135

Gly His Tyr Thr Gln Leu Val Trp Ala Asn Ser Phe Tyr Val Gly
                140                 145                 150

Cys Ala Val Ala Met Cys Pro Asn Leu Gly Gly Ala Ser Thr Ala
                155                 160                 165

Ile Phe Val Cys Asn Tyr Gly Pro Ala Gly Asn Phe Ala Asn Met
                170                 175                 180

Pro Pro Tyr Ala Arg Gly Glu Ser Cys Ser Leu Cys Ser Lys Glu
                185                 190                 195

Glu Lys Cys Val Lys Asn Leu Cys Arg Thr Pro Gln Leu Ile Ile
                200                 205                 210

Pro Asn Gln Asn Pro Phe Leu Lys Pro Thr Gly Arg Ala Pro Gln
                215                 220                 225

Gln Thr Ala Phe Asn Pro Phe Ser Leu Gly Phe Leu Leu Leu Arg
                230                 235                 240

Ile Phe
```

<210> 263
<211> 1687

<212> DNA
<213> Homo Sapien

<220>
<221> unsure
<222> 1447, 1489, 1593
<223> unknown base

<400> 263
cgccctccga cccgccccgc ggcgcattgt gggatctgtc ggcttgtcag 50

gtggtggagg aaaaggcgct ccgtcatggg gatccagacg agccccgtcc 100

tgctggcctc cctgggggtg gggctggtca ctctgctcgg cctggctgtg 150

ggctcctact tggttcggag gtcccgccgg cctcaggtca ctctcctgga 200

ccccaatgaa aagtacctgc tacgactgct agacaagacg actgtgagcc 250

acaacaccaa gaggttccgc tttgccctgc ccaccgccca ccacactctg 300

gggctgcctg tgggcaaaca tatctacctc tccacccgaa ttgatggcag 350

cctggtcatc aggccataca ctcctgtcac cagtgatgag gatcaaggct 400

atgtggatct tgtcatcaag gtctacctga agggtgtgca ccccaaattt 450

cctgagggag ggaagatgtc tcagtacctg gatagcctga aggttgggga 500

tgtggtggag tttcgggggc caagcgggtt gctcacttac actggaaaag 550

ggcattttaa cattcagccc aacaagaaat ctccaccaga accccgagtg 600

gcgaagaaac tgggaatgat tgccggcggg acaggaatca ccccaatgct 650

acagctgatc cgggccatcc tgaaagtccc tgaagatcca acccagtgct 700

ttctgctttt tgccaaccag acagaaaagg atatcatctt gcgggaggac 750

ttagaggaac tgcaggcccg ctatcccaat cgctttaagc tctggttcac 800

tctggatcat cccccaaaag attgggccta cagcaagggc tttgtgactg 850

ccgacatgat ccgggaacac ctgcccgctc caggggatga tgtgctggta 900

ctgctttgtg ggccaccccc aatggtgcag ctggcctgcc atcccaactt 950

ggacaaactg ggctactcac aaaagatgcg attcacctac tgagcatcct 1000

ccagcttccc tggtgctgtt cgctgcagtt gttccccatc agtactcaag 1050

cactataagc cttagattcc tttcctcaga gtttcaggtt ttttcagtta 1100

catctagagc tgaaatctgg atagtacctg caggaacaat attcctgtag 1150

ccatggaaga gggcaaggct cagtcactcc ttggatggcc tcctaaatct 1200

ccccgtggca acaggtccag gagaggccca tggagcagtc tcttccatgg 1250

agtaagaagg aagggagcat gtacgcttgg tccaagattg gctagttcct 1300

tgatagcatc ttactctcac cttctttgtg tctgtgatga aaggaacagt 1350

ctgtgcaatg ggttttactt aaacttcact gttcaaccta tgagcaaatc 1400

tgtatgtgtg agtataagtt gagcatagca tacttccaga ggtggtntta 1450

tggagatggc aagaaaggag gaaatgattt cttcagatnt caaaggagtc 1500

tgaaatatca tatttctgtg tgtgtctctc tcagcccctg cccaggctag 1550

agggaaacag ctactgataa tcgaaaactg ctgtttgtgg cangaacccc 1600

tggctgtgca aataaatggg gctgaggccc ctgtgtgata ttgaaaaaaa 1650

aaaaaaaaaa aaaaaaaaaa aaaaaaaaaa aaaaaga 1687

<210> 264
<211> 305
<212> PRT
<213> Homo Sapien

<400> 264
Met Gly Ile Gln Thr Ser Pro Val Leu Leu Ala Ser Leu Gly Val
1 5 10 15

Gly Leu Val Thr Leu Leu Gly Leu Ala Val Gly Ser Tyr Leu Val
20 25 30

Arg Arg Ser Arg Arg Pro Gln Val Thr Leu Leu Asp Pro Asn Glu
35 40 45

Lys Tyr Leu Leu Arg Leu Leu Asp Lys Thr Thr Val Ser His Asn
50 55 60

Thr Lys Arg Phe Arg Phe Ala Leu Pro Thr Ala His His Thr Leu
65 70 75

Gly Leu Pro Val Gly Lys His Ile Tyr Leu Ser Thr Arg Ile Asp
80 85 90

Gly Ser Leu Val Ile Arg Pro Tyr Thr Pro Val Thr Ser Asp Glu
95 100 105

Asp Gln Gly Tyr Val Asp Leu Val Ile Lys Val Tyr Leu Lys Gly
110 115 120

Val His Pro Lys Phe Pro Glu Gly Gly Lys Met Ser Gln Tyr Leu
125 130 135

Asp Ser Leu Lys Val Gly Asp Val Val Glu Phe Arg Gly Pro Ser
140 145 150

Gly Leu Leu Thr Tyr Thr Gly Lys Gly His Phe Asn Ile Gln Pro
155 160 165

Asn Lys Lys Ser Pro Pro Glu Pro Arg Val Ala Lys Lys Leu Gly
170 175 180

Met Ile Ala Gly Gly Thr Gly Ile Thr Pro Met Leu Gln Leu Ile
185 190 195

Arg Ala Ile Leu Lys Val Pro Glu Asp Pro Thr Gln Cys Phe Leu
200 205 210

Leu Phe Ala Asn Gln Thr Glu Lys Asp Ile Ile Leu Arg Glu Asp
215 220 225

```
Leu Glu Glu Leu Gln Ala Arg Tyr Pro Asn Arg Phe Lys Leu Trp
                230                 235                 240

Phe Thr Leu Asp His Pro Pro Lys Asp Trp Ala Tyr Ser Lys Gly
                245                 250                 255

Phe Val Thr Ala Asp Met Ile Arg Glu His Leu Pro Ala Pro Gly
                260                 265                 270

Asp Asp Val Leu Val Leu Leu Cys Gly Pro Pro Pro Met Val Gln
                275                 280                 285

Leu Ala Cys His Pro Asn Leu Asp Lys Leu Gly Tyr Ser Gln Lys
                290                 295                 300

Met Arg Phe Thr Tyr
                305
```

<210> 265
<211> 996
<212> DNA
<213> Homo Sapien

<400> 265

```
cccgtgccaa gagtgacgta agtaccgcct atagagtcta taggcccact 50
tggcttcgtt agaacgcggc tacaattaat acataacctt atgtatcata 100
cacatacgat ttaggtgaca ctatagaata acatccactt tgcctttctc 150
tccacaggtg tccactccca ggtccaactg cacctcggtt ctatcgataa 200
tctcagcacc agccactcag agcagggcac gatgttgggg gcccgcctca 250
ggctctgggt ctgtgccttg tgcagcgtct gcagcatgag cgtcctcaga 300
gcctatccca atgcctcccc actgctcggc tccagctggg gtggcctgat 350
ccacctgtac acagccacag ccaggaacag ctaccacctg cagatccaca 400
agaatggcca tgtggatggc gcaccccatc agaccatcta cagtgccctg 450
atgatcagat cagaggatgc tggctttgtg gtgattacag gtgtgatgag 500
cagaagatac ctctgcatgg atttcagagg caacattttt ggatcacact 550
atttcgaccc ggagaactgc aggttccaac accagacgct ggaaaacggg 600
tacgacgtct accactctcc tcagtatcac ttcctggtca gtctgggccg 650
ggcgaagaga gccttcctgc caggcatgaa cccacccccg tactcccagt 700
tcctgtcccg gaggaacgag atcccccctaa ttcacttcaa cacccccata 750
ccacggcggc acacccggag cgccgaggac gactcggagc gggacccccct 800
gaacgtgctg aagccccggg cccggatgac cccggccccg gcctcctgtt 850
cacaggagct cccgagcgcc gaggacaaca gcccgatggc cagtgaccca 900
ttaggggtgg tcaggggcgg tcgagtgaac acgcacgctg gggggaacggg 950
cccggaaggc tgccgcccct tcgccaagtt catctagggt cgctgg 996
```

-

<210> 266
<211> 251
<212> PRT
<213> Homo Sapien

<400> 266
Met Leu Gly Ala Arg Leu Arg Leu Trp Val Cys Ala Leu Cys Ser
1 5 10 15

Val Cys Ser Met Ser Val Leu Arg Ala Tyr Pro Asn Ala Ser Pro
20 25 30

Leu Leu Gly Ser Ser Trp Gly Gly Leu Ile His Leu Tyr Thr Ala
35 40 45

Thr Ala Arg Asn Ser Tyr His Leu Gln Ile His Lys Asn Gly His
50 55 60

Val Asp Gly Ala Pro His Gln Thr Ile Tyr Ser Ala Leu Met Ile
65 70 75

Arg Ser Glu Asp Ala Gly Phe Val Val Ile Thr Gly Val Met Ser
80 85 90

Arg Arg Tyr Leu Cys Met Asp Phe Arg Gly Asn Ile Phe Gly Ser
95 100 105

His Tyr Phe Asp Pro Glu Asn Cys Arg Phe Gln His Gln Thr Leu
110 115 120

Glu Asn Gly Tyr Asp Val Tyr His Ser Pro Gln Tyr His Phe Leu
125 130 135

Val Ser Leu Gly Arg Ala Lys Arg Ala Phe Leu Pro Gly Met Asn
140 145 150

Pro Pro Pro Tyr Ser Gln Phe Leu Ser Arg Arg Asn Glu Ile Pro
155 160 165

Leu Ile His Phe Asn Thr Pro Ile Pro Arg Arg His Thr Arg Ser
170 175 180

Ala Glu Asp Asp Ser Glu Arg Asp Pro Leu Asn Val Leu Lys Pro
185 190 195

Arg Ala Arg Met Thr Pro Ala Pro Ala Ser Cys Ser Gln Glu Leu
200 205 210

Pro Ser Ala Glu Asp Asn Ser Pro Met Ala Ser Asp Pro Leu Gly
215 220 225

Val Val Arg Gly Gly Arg Val Asn Thr His Ala Gly Gly Thr Gly
230 235 240

Pro Glu Gly Cys Arg Pro Phe Ala Lys Phe Ile
245 250

<210> 267
<211> 2290
<212> DNA
<213> Homo Sapien

<400> 267
ggctgagggg aggcccggag cctttctggg gcctggggga tcctcttgca 50

ctggtgggtg gagagaagcg cctgcagcca accagggtca ggctgtgctc 100
acagtttcct ctggcggcat gtaaaggctc cacaaaggag ttgggagttc 150
aaatgaggct gctgcggacg gcctgaggat ggaccccaag ccctggacct 200
gccgagcgtg gcactgaggc agcggctgac gctactgtga gggaaagaag 250
gttgtgagca gccccgcagg acccctggcc agccctggcc ccagcctctg 300
ccggagccct ctgtggaggc agagccagtg gagcccagtg aggcagggct 350
gcttggcagc caccggcctg caactcagga acccctccag aggccatgga 400
caggctgccc cgctgacggc cagggtgaag catgtgagga gccgccccgg 450
agccaagcag gagggaagag gctttcatag attctattca caaagaataa 500
ccaccatttt gcaaggacca tgaggccact gtgcgtgaca tgctggtggc 550
tcggactgct ggctgccatg ggagctgttg caggccagga ggacggtttt 600
gagggcactg aggagggctc gccaagagag ttcatttacc taaacaggta 650
caagcgggcg ggcgagtccc aggacaagtg cacctacacc ttcattgtgc 700
cccagcagcg ggtcacgggt gccatctgcg tcaactccaa ggagcctgag 750
gtgcttctgg agaaccgagt gcataagcag gagctagagc tgctcaacaa 800
tgagctgctc aagcagaagc ggcagatcga gacgctgcag cagctggtgg 850
aggtggacgg cggcattgtg agcgaggtga agctgctgcg caaggagagc 900
cgcaacatga actcgcgggt cacgcagctc tacatgcagc tcctgcacga 950
gatcatccgc aagcgggaca acgcgttgga gctctcccag ctggagaaca 1000
ggatcctgaa ccagacagcc gacatgctgc agctggccag caagtacaag 1050
gacctggagc acaagtacca gcacctggcc acactggccc acaaccaatc 1100
agagatcatc gcgcagcttg aggagcactg ccagagggtg ccctcggcca 1150
ggcccgtccc ccagccaccc cccgctgccc cgccccgggt ctaccaacca 1200
cccacctaca accgcatcat caaccagatc tctaccaacg agatccagag 1250
tgaccagaac ctgaaggtgc tgccaccccc tctgcccact atgcccactc 1300
tcaccagcct cccatcttcc accgacaagc cgtcgggccc atggagagac 1350
tgcctgcagg ccctggagga tggccacgac accagctcca tctacctggt 1400
gaagccggag aacaccaacc gcctcatgca ggtgtggtgc gaccagagac 1450
acgaccccgg gggctggacc gtcatccaga gacgcctgga tggctctgtt 1500
aacttcttca ggaactggga gacgtacaag caagggtttg ggaacattga 1550
cggcgaatac tggctgggcc tggagaacat ttactggctg acgaaccaag 1600
gcaactacaa actcctggtg accatggagg actggtccgg ccgcaaagtc 1650

tttgcagaat acgccagttt ccgcctggaa cctgagagcg agtattataa 1700

gctgcggctg gggcgctacc atggcaatgc gggtgactcc tttacatggc 1750

acaacggcaa gcagttcacc accctggaca gagatcatga tgtctacaca 1800

ggaaactgtg cccactacca gaagggaggc tggtggtata acgcctgtgc 1850

ccactccaac ctcaacgggg tctggtaccg cgggggccat taccggagcc 1900

gctaccagga cggagtctac tgggctgagt tccgaggagg ctcttactca 1950

ctcaagaaag tggtgatgat gatccgaccg aaccccaaca ccttccacta 2000

agccagctcc ccctcctgac ctctcgtggc cattgccagg agcccaccct 2050

ggtcacgctg gccacagcac aaagaacaac tcctcaccag ttcatcctga 2100

ggctgggagg accgggatgc tggattctgt tttccgaagt cactgcagcg 2150

gatgatggaa ctgaatcgat acggtgtttt ctgtccctcc tactttcctt 2200

cacaccagac agcccctcat gtctccagga caggacagga ctacagacaa 2250

ctctttcttt aaataaatta agtctctaca ataaaaaaaa 2290

<210> 268
<211> 493
<212> PRT
<213> Homo Sapien

<400> 268

Met Arg Pro Leu Cys Val Thr Cys Trp Trp Leu Gly Leu Leu Ala
1 5 10 15

Ala Met Gly Ala Val Ala Gly Gln Glu Asp Gly Phe Glu Gly Thr
20 25 30

Glu Glu Gly Ser Pro Arg Glu Phe Ile Tyr Leu Asn Arg Tyr Lys
35 40 45

Arg Ala Gly Glu Ser Gln Asp Lys Cys Thr Tyr Thr Phe Ile Val
50 55 60

Pro Gln Gln Arg Val Thr Gly Ala Ile Cys Val Asn Ser Lys Glu
65 70 75

Pro Glu Val Leu Leu Glu Asn Arg Val His Lys Gln Glu Leu Glu
80 85 90

Leu Leu Asn Asn Glu Leu Leu Lys Gln Lys Arg Gln Ile Glu Thr
95 100 105

Leu Gln Gln Leu Val Glu Val Asp Gly Gly Ile Val Ser Glu Val
110 115 120

Lys Leu Leu Arg Lys Glu Ser Arg Asn Met Asn Ser Arg Val Thr
125 130 135

Gln Leu Tyr Met Gln Leu Leu His Glu Ile Ile Arg Lys Arg Asp
140 145 150

Asn Ala Leu Glu Leu Ser Gln Leu Glu Asn Arg Ile Leu Asn Gln

```
                155                 160                 165
Thr Ala Asp Met Leu Gln Leu Ala Ser Lys Tyr Lys Asp Leu Glu
                170                 175                 180
His Lys Tyr Gln His Leu Ala Thr Leu Ala His Asn Gln Ser Glu
                185                 190                 195
Ile Ile Ala Gln Leu Glu Glu His Cys Gln Arg Val Pro Ser Ala
                200                 205                 210
Arg Pro Val Pro Gln Pro Pro Pro Ala Ala Pro Pro Arg Val Tyr
                215                 220                 225
Gln Pro Pro Thr Tyr Asn Arg Ile Ile Asn Gln Ile Ser Thr Asn
                230                 235                 240
Glu Ile Gln Ser Asp Gln Asn Leu Lys Val Leu Pro Pro Pro Leu
                245                 250                 255
Pro Thr Met Pro Thr Leu Thr Ser Leu Pro Ser Ser Thr Asp Lys
                260                 265                 270
Pro Ser Gly Pro Trp Arg Asp Cys Leu Gln Ala Leu Glu Asp Gly
                275                 280                 285
His Asp Thr Ser Ser Ile Tyr Leu Val Lys Pro Glu Asn Thr Asn
                290                 295                 300
Arg Leu Met Gln Val Trp Cys Asp Gln Arg His Asp Pro Gly Gly
                305                 310                 315
Trp Thr Val Ile Gln Arg Arg Leu Asp Gly Ser Val Asn Phe Phe
                320                 325                 330
Arg Asn Trp Glu Thr Tyr Lys Gln Gly Phe Gly Asn Ile Asp Gly
                335                 340                 345
Glu Tyr Trp Leu Gly Leu Glu Asn Ile Tyr Trp Leu Thr Asn Gln
                350                 355                 360
Gly Asn Tyr Lys Leu Leu Val Thr Met Glu Asp Trp Ser Gly Arg
                365                 370                 375
Lys Val Phe Ala Glu Tyr Ala Ser Phe Arg Leu Glu Pro Glu Ser
                380                 385                 390
Glu Tyr Tyr Lys Leu Arg Leu Gly Arg Tyr His Gly Asn Ala Gly
                395                 400                 405
Asp Ser Phe Thr Trp His Asn Gly Lys Gln Phe Thr Thr Leu Asp
                410                 415                 420
Arg Asp His Asp Val Tyr Thr Gly Asn Cys Ala His Tyr Gln Lys
                425                 430                 435
Gly Gly Trp Trp Tyr Asn Ala Cys Ala His Ser Asn Leu Asn Gly
                440                 445                 450
Val Trp Tyr Arg Gly Gly His Tyr Arg Ser Arg Tyr Gln Asp Gly
                455                 460                 465
Val Tyr Trp Ala Glu Phe Arg Gly Gly Ser Tyr Ser Leu Lys Lys
                470                 475                 480
```

```
Val Val Met Met Ile Arg Pro Asn Pro Asn Thr Phe His
                485                 490
```

<210> 269
<211> 1869
<212> DNA
<213> Homo Sapien

<400> 269

```
gccgagctga gcggatcctc acatgactgt gatccgattc tttccagcgg 50

cttctgcaac caagcgggtc ttacccccgg tcctccgcgt ctccagtcct 100

cgcacctgga accccaacgt ccccgagagt ccccgaatcc ccgctcccag 150

gctacctaag aggatgagcg gtgctccgac ggccggggca gccctgatgc 200

tctgcgccgc caccgccgtg ctactgagcg ctcagggcgg acccgtgcag 250

tccaagtcgc cgcgctttgc gtcctgggac gagatgaatg tcctggcgca 300

cggactcctg cagctcggcc aggggctgcg cgaacacgcg gagcgcaccc 350

gcagtcagct gagcgcgctg gagcggcgcc tgagcgcgtg cgggtccgcc 400

tgtcagggaa ccgaggggtc caccgacctc ccgttagccc ctgagagccg 450

ggtggaccct gaggtccttc acagcctgca gacacaactc aaggctcaga 500

acagcaggat ccagcaactc ttccacaagg tggcccagca gcagcggcac 550

ctggagaagc agcacctgcg aattcagcat ctgcaaagcc agtttggcct 600

cctggaccac aagcacctag accatgaggt ggccaagcct gcccgaagaa 650

agaggctgcc cgagatggcc cagccagttg acccggctca caatgtcagc 700

cgcctgcacc ggctgcccag ggattgccag gagctgttcc aggttgggga 750

gaggcagagt ggactatttg aaatccagcc tcaggggtct ccgccatttt 800

tggtgaactg caagatgacc tcagatggag gctggacagt aattcagagg 850

cgccacgatg gctcagtgga cttcaaccgg ccctgggaag cctacaaggc 900

ggggtttggg gatccccacg gcgagttctg gctgggtctg gagaaggtgc 950

atagcatcac gggggaccgc aacagccgcc tggccgtgca gctgcgggac 1000

tgggatggca acgccgagtt gctgcagttc tccgtgcacc tgggtggcga 1050

ggacacggcc tatagcctgc agctcactgc acccgtggcc ggccagctgg 1100

gcgccaccac cgtcccaccc agcggcctct ccgtaccctt ctccacttgg 1150

gaccaggatc acgacctccg cagggacaag aactgcgcca agagcctctc 1200

tggaggctgg tggtttggca cctgcagcca ttccaacctc aacggccagt 1250

acttccgctc catcccacag cagcggcaga agcttaagaa gggaatcttc 1300

tggaagacct ggcggggccg ctactacccg ctgcaggcca ccaccatgtt 1350
```

gatccagccc atggcagcag aggcagcctc ctagcgtcct ggctgggcct 1400

ggtcccaggc ccacgaaaga cggtgactct tggctctgcc cgaggatgtg 1450

gccgttccct gcctgggcag gggctccaag gaggggccat ctggaaactt 1500

gtggacagag aagaagacca cgactggaga agcccccttt ctgagtgcag 1550

gggggctgca tgcgttgcct cctgagatcg aggctgcagg atatgctcag 1600

actctagagg cgtggaccaa ggggcatgga gcttcactcc ttgctggcca 1650

gggagttggg gactcagagg gaccacttgg ggccagccag actggcctca 1700

atggcggact cagtcacatt gactgacggg gaccagggct tgtgtgggtc 1750

gagagcgccc tcatggtgct ggtgctgttg tgtgtaggtc ccctggggac 1800

acaagcaggc gccaatggta tctgggcgga gctcacagag ttcttggaat 1850

aaaagcaacc tcagaacac 1869

<210> 270
<211> 453
<212> PRT
<213> Homo Sapien

<400> 270

```
Met Thr Val Ile Arg Phe Phe Pro Ala Ala Ser Ala Thr Lys Arg
  1               5                  10                  15

Val Leu Pro Pro Val Leu Arg Val Ser Ser Pro Arg Thr Trp Asn
                 20                  25                  30

Pro Asn Val Pro Glu Ser Pro Arg Ile Pro Ala Pro Arg Leu Pro
                 35                  40                  45

Lys Arg Met Ser Gly Ala Pro Thr Ala Gly Ala Ala Leu Met Leu
                 50                  55                  60

Cys Ala Ala Thr Ala Val Leu Leu Ser Ala Gln Gly Gly Pro Val
                 65                  70                  75

Gln Ser Lys Ser Pro Arg Phe Ala Ser Trp Asp Glu Met Asn Val
                 80                  85                  90

Leu Ala His Gly Leu Leu Gln Leu Gly Gln Gly Leu Arg Glu His
                 95                 100                 105

Ala Glu Arg Thr Arg Ser Gln Leu Ser Ala Leu Glu Arg Arg Leu
                110                 115                 120

Ser Ala Cys Gly Ser Ala Cys Gln Gly Thr Glu Gly Ser Thr Asp
                125                 130                 135

Leu Pro Leu Ala Pro Glu Ser Arg Val Asp Pro Glu Val Leu His
                140                 145                 150

Ser Leu Gln Thr Gln Leu Lys Ala Gln Asn Ser Arg Ile Gln Gln
                155                 160                 165

Leu Phe His Lys Val Ala Gln Gln Gln Arg His Leu Glu Lys Gln
                170                 175                 180
```

His Leu Arg Ile Gln His Leu Gln Ser Gln Phe Gly Leu Leu Asp
185 190 195

His Lys His Leu Asp His Glu Val Ala Lys Pro Ala Arg Arg Lys
200 205 210

Arg Leu Pro Glu Met Ala Gln Pro Val Asp Pro Ala His Asn Val
215 220 225

Ser Arg Leu His Arg Leu Pro Arg Asp Cys Gln Glu Leu Phe Gln
230 235 240

Val Gly Glu Arg Gln Ser Gly Leu Phe Glu Ile Gln Pro Gln Gly
245 250 255

Ser Pro Pro Phe Leu Val Asn Cys Lys Met Thr Ser Asp Gly Gly
260 265 270

Trp Thr Val Ile Gln Arg Arg His Asp Gly Ser Val Asp Phe Asn
275 280 285

Arg Pro Trp Glu Ala Tyr Lys Ala Gly Phe Gly Asp Pro His Gly
290 295 300

Glu Phe Trp Leu Gly Leu Glu Lys Val His Ser Ile Thr Gly Asp
305 310 315

Arg Asn Ser Arg Leu Ala Val Gln Leu Arg Asp Trp Asp Gly Asn
320 325 330

Ala Glu Leu Leu Gln Phe Ser Val His Leu Gly Gly Glu Asp Thr
335 340 345

Ala Tyr Ser Leu Gln Leu Thr Ala Pro Val Ala Gly Gln Leu Gly
350 355 360

Ala Thr Thr Val Pro Pro Ser Gly Leu Ser Val Pro Phe Ser Thr
365 370 375

Trp Asp Gln Asp His Asp Leu Arg Arg Asp Lys Asn Cys Ala Lys
380 385 390

Ser Leu Ser Gly Gly Trp Trp Phe Gly Thr Cys Ser His Ser Asn
395 400 405

Leu Asn Gly Gln Tyr Phe Arg Ser Ile Pro Gln Gln Arg Gln Lys
410 415 420

Leu Lys Lys Gly Ile Phe Trp Lys Thr Trp Arg Gly Arg Tyr Tyr
425 430 435

Pro Leu Gln Ala Thr Thr Met Leu Ile Gln Pro Met Ala Ala Glu
440 445 450

Ala Ala Ser

<210> 271
<211> 1174
<212> DNA
<213> Homo Sapien

<400> 271
cggacgcgtg ggggaaaccc ttccgagaaa acagcaacaa gctgagctgc 50

tgtgacagag gggaacaaga tggcggcgcc gaaggggagc ctctgggtga 100

ggacccaact ggggctcccg ccgctgctgc tgctgaccat ggccttggcc 150

ggaggttcgg ggaccgcttc ggctgaagca tttgactcgg tcttgggtga 200

tacggcgtct tgccaccggg cctgtcagtt gacctacccc ttgcacacct 250

accctaagga agaggagttg tacgcatgtc agagaggttg caggctgttt 300

tcaatttgtc agtttgtgga tgatggaatt gacttaaatc gaactaaatt 350

ggaatgtgaa tctgcatgta cagaagcata ttcccaatct gatgagcaat 400

atgcttgcca tcttggttgc cagaatcagc tgccattcgc tgaactgaga 450

caagaacaac ttatgtccct gatgccaaaa atgcacctac tctttcctct 500

aactctggtg aggtcattct ggagtgacat gatggactcc gcacagagct 550

tcataacctc ttcatggact ttttatcttc aagccgatga cggaaaaata 600

gttatattcc agtctaagcc agaaatccag tacgcaccac atttggagca 650

ggagcctaca aatttgagag aatcatctct aagcaaaatg tcctatctgc 700

aaatgagaaa ttcacaagcg cacaggaatt ttcttgaaga tggagaaagt 750

gatggctttt taagatgcct ctctcttaac tctgggtgga ttttaactac 800

aactcttgtc ctctcggtga tggtattgct ttggatttgt tgtgcaactg 850

ttgctacagc tgtggagcag tatgttccct ctgagaagct gagtatctat 900

ggtgacttgg agtttatgaa tgaacaaaag ctaaacagat atccagcttc 950

ttctcttgtg gttgttagat ctaaaactga agatcatgaa gaagcagggc 1000

ctctacctac aaaagtgaat cttgctcatt ctgaaattta agcatttttc 1050

ttttaaaaga caagtgtaat agacatctaa aattccactc ctcatagagc 1100

ttttaaaatg gtttcattgg atataggcct taagaaatca ctataaaatg 1150

caaataaagt tactcaaatc tgtg 1174

<210> 272
<211> 323
<212> PRT
<213> Homo Sapien

<400> 272

```
Met Ala Ala Pro Lys Gly Ser Leu Trp Val Arg Thr Gln Leu Gly
1               5                   10                  15

Leu Pro Pro Leu Leu Leu Leu Thr Met Ala Leu Ala Gly Gly Ser
                20                  25                  30

Gly Thr Ala Ser Ala Glu Ala Phe Asp Ser Val Leu Gly Asp Thr
                35                  40                  45

Ala Ser Cys His Arg Ala Cys Gln Leu Thr Tyr Pro Leu His Thr
                50                  55                  60
```

Tyr Pro Lys Glu Glu Glu Leu Tyr Ala Cys Gln Arg Gly Cys Arg
65 70 75

Leu Phe Ser Ile Cys Gln Phe Val Asp Asp Gly Ile Asp Leu Asn
80 85 90

Arg Thr Lys Leu Glu Cys Glu Ser Ala Cys Thr Glu Ala Tyr Ser
95 100 105

Gln Ser Asp Glu Gln Tyr Ala Cys His Leu Gly Cys Gln Asn Gln
110 115 120

Leu Pro Phe Ala Glu Leu Arg Gln Glu Gln Leu Met Ser Leu Met
125 130 135

Pro Lys Met His Leu Leu Phe Pro Leu Thr Leu Val Arg Ser Phe
140 145 150

Trp Ser Asp Met Met Asp Ser Ala Gln Ser Phe Ile Thr Ser Ser
155 160 165

Trp Thr Phe Tyr Leu Gln Ala Asp Asp Gly Lys Ile Val Ile Phe
170 175 180

Gln Ser Lys Pro Glu Ile Gln Tyr Ala Pro His Leu Glu Gln Glu
185 190 195

Pro Thr Asn Leu Arg Glu Ser Ser Leu Ser Lys Met Ser Tyr Leu
200 205 210

Gln Met Arg Asn Ser Gln Ala His Arg Asn Phe Leu Glu Asp Gly
215 220 225

Glu Ser Asp Gly Phe Leu Arg Cys Leu Ser Leu Asn Ser Gly Trp
230 235 240

Ile Leu Thr Thr Thr Leu Val Leu Ser Val Met Val Leu Leu Trp
245 250 255

Ile Cys Cys Ala Thr Val Ala Thr Ala Val Glu Gln Tyr Val Pro
260 265 270

Ser Glu Lys Leu Ser Ile Tyr Gly Asp Leu Glu Phe Met Asn Glu
275 280 285

Gln Lys Leu Asn Arg Tyr Pro Ala Ser Ser Leu Val Val Val Arg
290 295 300

Ser Lys Thr Glu Asp His Glu Glu Ala Gly Pro Leu Pro Thr Lys
305 310 315

Val Asn Leu Ala His Ser Glu Ile
320

<210> 273
<211> 1200
<212> DNA
<213> Homo Sapien

<400> 273
cccacgcgtc cgaacctctc cagcgatggg agccgcccgc ctgctgccca 50

acctcactct gtgcttacag ctgctgattc tctgctgtca aactcagtac 100

gtgagggacc agggcgccat gaccgaccag ctgagcaggc ggcagatccg 150

cgagtaccaa ctctacagca ggaccagtgg caagcacgtg caggtcaccg 200

ggcgtcgcat ctccgccacc gccgaggacg gcaacaagtt tgccaagctc 250

atagtggaga cggacacgtt tggcagccgg gttcgcatca aaggggctga 300

gagtgagaag tacatctgta tgaacaagag gggcaagctc atcgggaagc 350

ccagcgggaa gagcaaagac tgcgtgttca cggagatcgt gctggagaac 400

aactatacgg ccttccagaa cgcccggcac gagggctggt tcatggcctt 450

cacgcggcag gggcggcccc gccaggcttc ccgcagccgc cagaaccagc 500

gcgaggccca cttcatcaag cgcctctacc aaggccagct gcccttcccc 550

aaccacgccg agaagcagaa gcagttcgag tttgtgggct ccgcccccac 600

ccgccggacc aagcgcacac ggcggcccca gcccctcacg tagtctggga 650

ggcagggggc agcagcccct gggccgcctc cccacccctt tcccttctta 700

atccaaggac tgggctgggg tggcgggagg ggagccagat ccccgaggga 750

ggaccctgag ggccgcgaag catccgagcc cccagctggg aaggggcagg 800

ccggtgcccc aggggcggct ggcacagtgc cccccttcccg gacgggtggc 850

aggccctgga gaggaactga gtgtcaccct gatctcaggc caccagcctc 900

tgccggcctc ccagccgggc tcctgaagcc cgctgaaagg tcagcgactg 950

aaggccttgc agacaaccgt ctggaggtgg ctgtcctcaa aatctgcttc 1000

tcggatctcc ctcagtctgc ccccagcccc caaactcctc ctggctagac 1050

tgtaggaagg gacttttgtt tgtttgtttg tttcaggaaa aaagaaaggg 1100

agagagagga aaatagaggg ttgtccactc ctcacattcc acgacccagg 1150

cctgcacccc acccccaact cccagccccg gaataaaacc attttcctgc 1200

<210> 274
<211> 205
<212> PRT
<213> Homo Sapien

<400> 274
Met Gly Ala Ala Arg Leu Leu Pro Asn Leu Thr Leu Cys Leu Gln
1 5 10 15

Leu Leu Ile Leu Cys Cys Gln Thr Gln Tyr Val Arg Asp Gln Gly
20 25 30

Ala Met Thr Asp Gln Leu Ser Arg Arg Gln Ile Arg Glu Tyr Gln
35 40 45

Leu Tyr Ser Arg Thr Ser Gly Lys His Val Gln Val Thr Gly Arg
50 55 60

Arg Ile Ser Ala Thr Ala Glu Asp Gly Asn Lys Phe Ala Lys Leu

```
                65                  70                  75

Ile Val Glu Thr Asp Thr Phe Gly Ser Arg Val Arg Ile Lys Gly
                80                  85                  90

Ala Glu Ser Glu Lys Tyr Ile Cys Met Asn Lys Arg Gly Lys Leu
                95                  100                 105

Ile Gly Lys Pro Ser Gly Lys Ser Lys Asp Cys Val Phe Thr Glu
                110                 115                 120

Ile Val Leu Glu Asn Asn Tyr Thr Ala Phe Gln Asn Ala Arg His
                125                 130                 135

Glu Gly Trp Phe Met Ala Phe Thr Arg Gln Gly Arg Pro Arg Gln
                140                 145                 150

Ala Ser Arg Ser Arg Gln Asn Gln Arg Glu Ala His Phe Ile Lys
                155                 160                 165

Arg Leu Tyr Gln Gly Gln Leu Pro Phe Pro Asn His Ala Glu Lys
                170                 175                 180

Gln Lys Gln Phe Glu Phe Val Gly Ser Ala Pro Thr Arg Arg Thr
                185                 190                 195

Lys Arg Thr Arg Arg Pro Gln Pro Leu Thr
                200                 205
```

<210> 275
<211> 715
<212> DNA
<213> Homo Sapien

<400> 275

```
tatttaccat atcagattca cattcagtcc tcagcaaaat gaagggctcc 50

attttcactc tgtttttatt ctctgtccta tttgccatct cagaagtgcg 100

gagcaaggag tctgtgagac tctgtgggct agaatacata cggacagtca 150

tctatatctg tgctagctcc aggtggagaa ggcatctgga ggggatccct 200

caagctcagc aagctgagac aggaaactcc ttccagctcc cacataaacg 250

tgagttttct gaggaaaatc cagcgcaaaa ccttccgaag gtggatgcct 300

caggggaaga ccgtctttgg ggtggacaga tgcccactga agagctttgg 350

aagtcaaaga agcattcagt gatgtcaaga caagatttac aaactttgtg 400

ttgcactgat ggctgttcca tgactgattt gagtgctctt tgctaagaca 450

agagcaaata cccaatgggt ggcagagctt tatcacatgt ttaattacag 500

tgttttactg cctggtagaa cactaatatt gtgttattaa aatgatggct 550

tttgggtagg caaaacttct tttctaaaag gtatagctga gcggttgaaa 600

ccacagtgat ctctattttc tccctttgcc aaggttaatg aactgttctt 650

ttcaaattct actaatgctt tgaaatttca aatgctgcgc aaaattgcaa 700

taaaaatgct ataaa 715
```

<210> 276
<211> 135
<212> PRT
<213> Homo Sapien

<400> 276

```
Met Lys Gly Ser Ile Phe Thr Leu Phe Leu Phe Ser Val Leu Phe
1               5                   10                  15

Ala Ile Ser Glu Val Arg Ser Lys Glu Ser Val Arg Leu Cys Gly
                20                  25                  30

Leu Glu Tyr Ile Arg Thr Val Ile Tyr Ile Cys Ala Ser Ser Arg
                35                  40                  45

Trp Arg Arg His Leu Glu Gly Ile Pro Gln Ala Gln Gln Ala Glu
                50                  55                  60

Thr Gly Asn Ser Phe Gln Leu Pro His Lys Arg Glu Phe Ser Glu
                65                  70                  75

Glu Asn Pro Ala Gln Asn Leu Pro Lys Val Asp Ala Ser Gly Glu
                80                  85                  90

Asp Arg Leu Trp Gly Gly Gln Met Pro Thr Glu Glu Leu Trp Lys
                95                  100                 105

Ser Lys Lys His Ser Val Met Ser Arg Gln Asp Leu Gln Thr Leu
                110                 115                 120

Cys Cys Thr Asp Gly Cys Ser Met Thr Asp Leu Ser Ala Leu Cys
                125                 130                 135
```

<210> 277
<211> 3355
<212> DNA
<213> Homo Sapien

<400> 277

```
gcagctggtt actgcatttc tccatgtggc agacagagca aagccacaac 50

gctttctctg ctggattaaa gacggcccac agaccagaac ttccactata 100

ctacttaaaa ttacataggt ggcttgtcaa attcaattga ttagtattgt 150

aaaaggaaaa agaagttcct tcttacagct tggattcaac ggtccaaaac 200

aaaaatgcag ctgccattaa agtctcagat gaacaaactt ctacactgat 250

ttttaaaatc aagaataagg gcagcaagtt tctggattca ctgaatcaac 300

agacacaaaa agctggcaat atagcaacta tgaagagaaa agctactaat 350

aaaattaacc caacgcatag aagacttttt tttctcttct aaaaacaact 400

aagtaaagac ttaaatttaa acacatcatt ttacaacctc atttcaaaat 450

gaagactttt acctggaccc taggtgtgct attcttccta ctagtggaca 500

ctggacattg cagaggtgga caattcaaaa ttaaaaaaat aaaccagaga 550

agataccctc gtgccacaga tggtaaagag gaagcaaaga aatgtgcata 600
```

cacattcctg gtacctgaac aaagaataac agggccaatc tgtgtcaaca 650
ccaaggggca agatgcaagt accattaaag acatgatcac caggatggac 700
cttgaaaacc tgaaggatgt gctctccagg cagaagcggg agatagatgt 750
tctgcaactg gtggtggatg tagatggaaa cattgtgaat gaggtaaagc 800
tgctgagaaa ggaaagccgt aacatgaact ctcgtgttac tcaactctat 850
atgcaattat tacatgagat tatccgtaag agggataatt cacttgaact 900
ttcccaactg gaaaacaaaa tcctcaatgt caccacagaa atgttgaaga 950
tggcaacaag atacagggaa ctagaggtga aatacgcttc cttgactgat 1000
cttgtcaata accaatctgt gatgatcact ttgttggaag aacagtgctt 1050
gaggatattt tcccgacaag acacccatgt gtctccccca cttgtccagg 1100
tggtgccaca acatattcct aacagccaac agtatactcc tggtctgctg 1150
ggaggtaacg agattcagag ggatccaggt tatcccagag atttaatgcc 1200
accacctgat ctggcaactt ctcccaccaa aagccctttc aagataccac 1250
cggtaacttt catcaatgaa ggaccattca aagactgtca gcaagcaaaa 1300
gaagctgggc attcggtcag tgggatttat atgattaaac ctgaaaacag 1350
caatggacca atgcagttat ggtgtgaaaa cagtttggac cctggggggtt 1400
ggactgttat tcagaaaaga acagacggct ctgtcaactt cttcagaaat 1450
tgggaaaatt ataagaaagg gtttggaaac attgacggag aatactggct 1500
tggactggaa aatatctata tgcttagcaa tcaagataat tacaagttat 1550
tgattgaatt agaagactgg agtgataaaa aagtctatgc agaatacagc 1600
agctttcgtc tggaacctga aagtgaattc tatagactgc gcctgggaac 1650
ttaccaggga aatgcagggg attctatgat gtggcataat ggtaaacaat 1700
tcaccacact ggacagagat aaagatatgt atgcaggaaa ctgcgcccac 1750
tttcataaag gaggctggtg gtacaatgcc tgtgcacatt ctaacctaaa 1800
tggagtatgg tacagaggag gccattacag aagcaagcac caagatggaa 1850
ttttctgggc cgaatacaga ggcgggtcat actccttaag agcagttcag 1900
atgatgatca agcctattga ctgaagagag acactcgcca atttaaatga 1950
cacagaactt tgtacttttc agctcttaaa aatgtaaatg ttacatgtat 2000
attacttggc acaatttatt tctacacaga aagtttttaa aatgaatttt 2050
accgtaacta taaaagggaa cctataaatg tagtttcatc tgtcgtcaat 2100
tactgcagaa aattatgtgt atccacaacc tagttatttt aaaaattatg 2150
ttgactaaat acaaagtttg ttttctaaaa tgtaaatatt tgccacaatg 2200

```
taaagcaaat cttagctata ttttaaatca taaataacat gttcaagata 2250
cttaacaatt tatttaaaat ctaagattgc tctaacgtct agtgaaaaaa 2300
atatttttta aatttcagcc aaataatgca ttttatttta taaaaataca 2350
gacagaaaat tagggagaaa cttctagttt tgccaataga aaatgttctt 2400
ccattgaata aaagttattt caaattgaat ttgtgccttt cacacgtaat 2450
gattaaatct gaattcttaa taatatatcc tatgctgatt ttcccaaaac 2500
atgacccata gtattaaata catatcattt ttaaaaataa aaaaaaaccc 2550
aaaaataatg catgcataat ttaaatggtc aatttataaa gacaaatcta 2600
tgaatgaatt tttcagtgtt atcttcatat gatatgctga acaccaaaat 2650
ctccagaaat gcattttatg tagttctaaa atcagcaaaa tattggtatt 2700
acaaaaatgc agaatattta gtgtgctaca gatctgaatt atagttctaa 2750
tttattatta cttttttctt aatttactga tcttactact acaaagaaaa 2800
aaaaacccaa cccatctgca attcaaatca gaaagtttgg acagctttac 2850
aagtattagt gcatgctcag aacaggtggg actaaaacaa actcaaggaa 2900
ctgttggctg ttttcccgat actgagaatt caacagctcc agagcagaag 2950
ccacaggggc atagcttagt ccaaactgct aatttcattt tacagtgtat 3000
gtaacgctta gtctcacagt gtctttaact catctttgca atcaacaact 3050
ttactagtga ctttctggaa caatttcctt tcaggaatac atattcactg 3100
cttagaggtg accttgcctt aatatatttg tgaagttaaa attttaaaga 3150
tagctcatga aacttttgct taagcaaaaa gaaaacctcg aattgaaatg 3200
tgtgaggcaa actatgcatg ggaatagctt aatgtgaaga taatcatttg 3250
gacaactcaa atccatcaac atgaccaatg tttttcatct gccacatctc 3300
aaaataaaac ttctggtgaa acaaattaaa caaaatatcc aaacctcaaa 3350
aaaaa 3355
```

<210> 278
<211> 491
<212> PRT
<213> Homo Sapien

<400> 278

```
Met Lys Thr Phe Thr Trp Thr Leu Gly Val Leu Phe Phe Leu Leu
  1               5                  10                  15

Val Asp Thr Gly His Cys Arg Gly Gly Gln Phe Lys Ile Lys Lys
               20                  25                  30

Ile Asn Gln Arg Arg Tyr Pro Arg Ala Thr Asp Gly Lys Glu Glu
               35                  40                  45

Ala Lys Lys Cys Ala Tyr Thr Phe Leu Val Pro Glu Gln Arg Ile
```

50 55 60

Thr Gly Pro Ile Cys Val Asn Thr Lys Gly Gln Asp Ala Ser Thr
65 70 75

Ile Lys Asp Met Ile Thr Arg Met Asp Leu Glu Asn Leu Lys Asp
80 85 90

Val Leu Ser Arg Gln Lys Arg Glu Ile Asp Val Leu Gln Leu Val
95 100 105

Val Asp Val Asp Gly Asn Ile Val Asn Glu Val Lys Leu Leu Arg
110 115 120

Lys Glu Ser Arg Asn Met Asn Ser Arg Val Thr Gln Leu Tyr Met
125 130 135

Gln Leu Leu His Glu Ile Ile Arg Lys Arg Asp Asn Ser Leu Glu
140 145 150

Leu Ser Gln Leu Glu Asn Lys Ile Leu Asn Val Thr Thr Glu Met
155 160 165

Leu Lys Met Ala Thr Arg Tyr Arg Glu Leu Glu Val Lys Tyr Ala
170 175 180

Ser Leu Thr Asp Leu Val Asn Asn Gln Ser Val Met Ile Thr Leu
185 190 195

Leu Glu Glu Gln Cys Leu Arg Ile Phe Ser Arg Gln Asp Thr His
200 205 210

Val Ser Pro Pro Leu Val Gln Val Val Pro Gln His Ile Pro Asn
215 220 225

Ser Gln Gln Tyr Thr Pro Gly Leu Leu Gly Gly Asn Glu Ile Gln
230 235 240

Arg Asp Pro Gly Tyr Pro Arg Asp Leu Met Pro Pro Pro Asp Leu
245 250 255

Ala Thr Ser Pro Thr Lys Ser Pro Phe Lys Ile Pro Pro Val Thr
260 265 270

Phe Ile Asn Glu Gly Pro Phe Lys Asp Cys Gln Gln Ala Lys Glu
275 280 285

Ala Gly His Ser Val Ser Gly Ile Tyr Met Ile Lys Pro Glu Asn
290 295 300

Ser Asn Gly Pro Met Gln Leu Trp Cys Glu Asn Ser Leu Asp Pro
305 310 315

Gly Gly Trp Thr Val Ile Gln Lys Arg Thr Asp Gly Ser Val Asn
320 325 330

Phe Phe Arg Asn Trp Glu Asn Tyr Lys Lys Gly Phe Gly Asn Ile
335 340 345

Asp Gly Glu Tyr Trp Leu Gly Leu Glu Asn Ile Tyr Met Leu Ser
350 355 360

Asn Gln Asp Asn Tyr Lys Leu Leu Ile Glu Leu Glu Asp Trp Ser
365 370 375

```
Asp Lys Lys Val Tyr Ala Glu Tyr Ser Ser Phe Arg Leu Glu Pro
                380                 385                 390

Glu Ser Glu Phe Tyr Arg Leu Arg Leu Gly Thr Tyr Gln Gly Asn
                395                 400                 405

Ala Gly Asp Ser Met Met Trp His Asn Gly Lys Gln Phe Thr Thr
                410                 415                 420

Leu Asp Arg Asp Lys Asp Met Tyr Ala Gly Asn Cys Ala His Phe
                425                 430                 435

His Lys Gly Gly Trp Trp Tyr Asn Ala Cys Ala His Ser Asn Leu
                440                 445                 450

Asn Gly Val Trp Tyr Arg Gly Gly His Tyr Arg Ser Lys His Gln
                455                 460                 465

Asp Gly Ile Phe Trp Ala Glu Tyr Arg Gly Gly Ser Tyr Ser Leu
                470                 475                 480

Arg Ala Val Gln Met Met Ile Lys Pro Ile Asp
                485                 490
```

<210> 279
<211> 1231
<212> DNA
<213> Homo Sapien

<400> 279

```
cccacgcgtc cgcgcagtcg cgcagttctg cctccgcctg ccagtctcgc 50

ccgcgatccc ggcccggggc tgtggcgtcg actccgaccc aggcagccag 100

cagcccgcgc gggagccgga ccgccgccgg aggagctcgg acggcatgct 150

gagcccccctc ctttgctgaa gcccgagtgc ggagaagccc gggcaaacgc 200

aggctaagga gaccaaagcg gcgaagtcgc gagacagcgg acaagcagcg 250

gaggagaagg aggaggaggc gaacccagag aggggcagca aaagaagcgg 300

tggtggtggg cgtcgtggcc atggcggcgg ctatcgccag ctcgctcatc 350

cgtcagaaga ggcaagcccg cgagcgcgag aaatccaacg cctgcaagtg 400

tgtcagcagc cccagcaaag gcaagaccag ctgcgacaaa aacaagttaa 450

atgtcttttc ccgggtcaaa ctcttcggct ccaagaagag gcgcagaaga 500

agaccagagc ctcagcttaa gggtatagtt accaagctat acagccgaca 550

aggctaccac ttgcagctgc aggcggatgg aaccattgat ggcaccaaag 600

atgaggacag cacttacact ctgtttaacc tcatccctgt gggtctgcga 650

gtggtggcta tccaaggagt tcaaaccaag ctgtacttgg caatgaacag 700

tgagggatac ttgtacacct cggaactttt cacacctgag tgcaaattca 750

aagaatcagt gtttgaaaat tattatgtga catattcatc aatgatatac 800

cgtcagcagc agtcaggccg agggtggtat ctgggtctga acaaagaagg 850
```

agagatcatg aaaggcaacc atgtgaagaa gaacaagcct gcagctcatt 900

ttctgcctaa accactgaaa gtggccatgt acaaggagcc atcactgcac 950

gatctcacgg agttctcccg atctggaagc gggaccccaa ccaagagcag 1000

aagtgtctct ggcgtgctga acggaggcaa atccatgagc cacaatgaat 1050

caacgtagcc agtgagggca aaagaagggc tctgtaacag aaccttacct 1100

ccaggtgctg ttgaattctt ctagcagtcc ttcacccaaa agttcaaatt 1150

tgtcagtgac atttaccaaa caaacaggca gagttcacta ttctatctgc 1200

cattagacct tcttatcatc catactaaag c 1231

<210> 280
<211> 245
<212> PRT
<213> Homo Sapien

<400> 280

```
Met Ala Ala Ala Ile Ala Ser Ser Leu Ile Arg Gln Lys Arg Gln
  1               5                  10                  15

Ala Arg Glu Arg Glu Lys Ser Asn Ala Cys Lys Cys Val Ser Ser
                 20                  25                  30

Pro Ser Lys Gly Lys Thr Ser Cys Asp Lys Asn Lys Leu Asn Val
                 35                  40                  45

Phe Ser Arg Val Lys Leu Phe Gly Ser Lys Lys Arg Arg Arg Arg
                 50                  55                  60

Arg Pro Glu Pro Gln Leu Lys Gly Ile Val Thr Lys Leu Tyr Ser
                 65                  70                  75

Arg Gln Gly Tyr His Leu Gln Leu Gln Ala Asp Gly Thr Ile Asp
                 80                  85                  90

Gly Thr Lys Asp Glu Asp Ser Thr Tyr Thr Leu Phe Asn Leu Ile
                 95                 100                 105

Pro Val Gly Leu Arg Val Val Ala Ile Gln Gly Val Gln Thr Lys
                110                 115                 120

Leu Tyr Leu Ala Met Asn Ser Glu Gly Tyr Leu Tyr Thr Ser Glu
                125                 130                 135

Leu Phe Thr Pro Glu Cys Lys Phe Lys Glu Ser Val Phe Glu Asn
                140                 145                 150

Tyr Tyr Val Thr Tyr Ser Ser Met Ile Tyr Arg Gln Gln Gln Ser
                155                 160                 165

Gly Arg Gly Trp Tyr Leu Gly Leu Asn Lys Glu Gly Glu Ile Met
                170                 175                 180

Lys Gly Asn His Val Lys Lys Asn Lys Pro Ala Ala His Phe Leu
                185                 190                 195

Pro Lys Pro Leu Lys Val Ala Met Tyr Lys Glu Pro Ser Leu His
                200                 205                 210
```

```
Asp Leu Thr Glu Phe Ser Arg Ser Gly Ser Gly Thr Pro Thr Lys
                215                 220                 225

Ser Arg Ser Val Ser Gly Val Leu Asn Gly Gly Lys Ser Met Ser
                230                 235                 240

His Asn Glu Ser Thr
                245
```

<210> 281
<211> 1471
<212> DNA
<213> Homo Sapien

<400> 281

```
ccaggatgga gctggggcct gtatagccat attattgttc tatgctacta 50
gacatggggg ggacttggtg aaaaaggtat tatccagcca gagggtctgg 100
gagccctgtc ttactgaacc tgggcaacct ggatattctg agacatattt 150
tggggggatt tcagtgaaaa aagtggggga tcccctccat ttagagtgta 200
gcaaaggaaa aaacaccaag gttgggttcc ttcctgacat tggcagtgcc 250
ccagtagggg tgggatgagc gaatattccc aaagctaaag tcccacaccc 300
tgtagattac aagagtggat ttggcaggag tgtgccccaa aatacagtgg 350
aaaggtgcct gaagatattt aaaccacgtc ttggaaattt agtgggtctt 400
ggctttggga taggtgaagt gaggacagac actggagagg agggaaaggg 450
gacgttttca ataggaggca aaactcgagg gtgggatcca ctgaggagta 500
cataggctgc tggatctggt ggagccagca ctgggcccac gggtggtaac 550
tggctgctgt ggaggggggt acgtgagggg ggggtctggg gcttatcctc 600
aggtcctgtg ggtggggcag cgagtcgggg cctgagcgtc aagagcatgc 650
cctagtgagc gggctcctct gggggagccc agcgcgctcc gggcgcctgc 700
cggtttgggg gtgtctcctc ccggggcgct atggcggcgc tggccagtag 750
cctgatccgg cagaagcggg aggtccgcga gcccgggggc agccggccgg 800
tgtcggcgca gcggcgcgtg tgtccccgcg gcaccaagtc cctttgccag 850
aagcagctcc tcatcctgct gtccaaggtg cgactgtgcg gggggcggcc 900
cgcgcggccg gaccgcggcc cggagcctca gctcaaaggc atcgtcacca 950
aactgttctg ccgccagggt ttctacctcc aggcgaatcc cgacggaagc 1000
atccagggca ccccagagga taccagctcc ttcacccact tcaacctgat 1050
ccctgtgggc ctccgtgtgg tcaccatcca gagcgccaag ctgggtcact 1100
acatggccat gaatgctgag ggactgctct acagttcgcc gcatttcaca 1150
gctgagtgtc gctttaagga gtgtgtcttt gagaattact acgtcctgta 1200
```

cgcctctgct ctctaccgcc agcgtcgttc tggccgggcc tggtacctcg 1250

gcctggacaa ggagggccag gtcatgaagg gaaaccgagt taagaagacc 1300

aaggcagctg cccactttct gcccaagctc ctggaggtgg ccatgtacca 1350

ggagccttct ctccacagtg tccccgaggc ctccccttcc agtccccctg 1400

ccccctgaaa tgtagtccct ggactggagg ttccctgcac tcccagtgag 1450

ccagccacca ccacaacctg t 1471

<210> 282
<211> 225
<212> PRT
<213> Homo Sapien

<400> 282
Met Ala Ala Leu Ala Ser Ser Leu Ile Arg Gln Lys Arg Glu Val
1 5 10 15

Arg Glu Pro Gly Gly Ser Arg Pro Val Ser Ala Gln Arg Arg Val
20 25 30

Cys Pro Arg Gly Thr Lys Ser Leu Cys Gln Lys Gln Leu Leu Ile
35 40 45

Leu Leu Ser Lys Val Arg Leu Cys Gly Gly Arg Pro Ala Arg Pro
50 55 60

Asp Arg Gly Pro Glu Pro Gln Leu Lys Gly Ile Val Thr Lys Leu
65 70 75

Phe Cys Arg Gln Gly Phe Tyr Leu Gln Ala Asn Pro Asp Gly Ser
80 85 90

Ile Gln Gly Thr Pro Glu Asp Thr Ser Ser Phe Thr His Phe Asn
95 100 105

Leu Ile Pro Val Gly Leu Arg Val Val Thr Ile Gln Ser Ala Lys
110 115 120

Leu Gly His Tyr Met Ala Met Asn Ala Glu Gly Leu Leu Tyr Ser
125 130 135

Ser Pro His Phe Thr Ala Glu Cys Arg Phe Lys Glu Cys Val Phe
140 145 150

Glu Asn Tyr Tyr Val Leu Tyr Ala Ser Ala Leu Tyr Arg Gln Arg
155 160 165

Arg Ser Gly Arg Ala Trp Tyr Leu Gly Leu Asp Lys Glu Gly Gln
170 175 180

Val Met Lys Gly Asn Arg Val Lys Lys Thr Lys Ala Ala Ala His
185 190 195

Phe Leu Pro Lys Leu Leu Glu Val Ala Met Tyr Gln Glu Pro Ser
200 205 210

Leu His Ser Val Pro Glu Ala Ser Pro Ser Ser Pro Pro Ala Pro
215 220 225

<210> 283

<211> 744
<212> DNA
<213> Homo Sapien

<400> 283
atggccgcgg ccatcgctag cggcttgatc cgccagaagc ggcaggcgcg 50

ggagcagcac tgggaccggc cgtctgccag caggaggcgg agcagcccca 100

gcaagaaccg cgggctctgc aacggcaacc tggtggatat cttctccaaa 150

gtgcgcatct tcggcctcaa gaagcgcagg ttgcggcgcc aagatcccca 200

gctcaagggt atagtgacca ggttatattg caggcaaggc tactacttgc 250

aaatgcaccc cgatggagct ctcgatggaa ccaaggatga cagcactaat 300

tctacactct tcaacctcat accagtggga ctacgtgttg ttgccatcca 350

gggagtgaaa acagggttgt atatagccat gaatggagaa ggttacctct 400

acccatcaga actttttacc cctgaatgca agtttaaaga atctgttttt 450

gaaaattatt atgtaatcta ctcatccatg ttgtacagac aacaggaatc 500

tggtagagcc tggtttttgg gattaaataa ggaagggcaa gctatgaaag 550

ggaacagagt aaagaaaacc aaaccagcag ctcattttct acccaagcca 600

ttggaagttg ccatgtaccg agaaccatct ttgcatgatg ttggggaaac 650

ggtcccgaag cctggggtga cgccaagtaa aagcacaagt gcgtctgcaa 700

taatgaatgg aggcaaacca gtcaacaaga gtaagacaac atag 744

<210> 284
<211> 247
<212> PRT
<213> Homo Sapien

<400> 284
Met Ala Ala Ala Ile Ala Ser Gly Leu Ile Arg Gln Lys Arg Gln
1 5 10 15

Ala Arg Glu Gln His Trp Asp Arg Pro Ser Ala Ser Arg Arg Arg
20 25 30

Ser Ser Pro Ser Lys Asn Arg Gly Leu Cys Asn Gly Asn Leu Val
35 40 45

Asp Ile Phe Ser Lys Val Arg Ile Phe Gly Leu Lys Lys Arg Arg
50 55 60

Leu Arg Arg Gln Asp Pro Gln Leu Lys Gly Ile Val Thr Arg Leu
65 70 75

Tyr Cys Arg Gln Gly Tyr Tyr Leu Gln Met His Pro Asp Gly Ala
80 85 90

Leu Asp Gly Thr Lys Asp Asp Ser Thr Asn Ser Thr Leu Phe Asn
95 100 105

Leu Ile Pro Val Gly Leu Arg Val Val Ala Ile Gln Gly Val Lys
110 115 120

```
Thr Gly Leu Tyr Ile Ala Met Asn Gly Glu Gly Tyr Leu Tyr Pro
                125                 130                 135

Ser Glu Leu Phe Thr Pro Glu Cys Lys Phe Lys Glu Ser Val Phe
                140                 145                 150

Glu Asn Tyr Tyr Val Ile Tyr Ser Ser Met Leu Tyr Arg Gln Gln
                155                 160                 165

Glu Ser Gly Arg Ala Trp Phe Leu Gly Leu Asn Lys Glu Gly Gln
                170                 175                 180

Ala Met Lys Gly Asn Arg Val Lys Lys Thr Lys Pro Ala Ala His
                185                 190                 195

Phe Leu Pro Lys Pro Leu Glu Val Ala Met Tyr Arg Glu Pro Ser
                200                 205                 210

Leu His Asp Val Gly Glu Thr Val Pro Lys Pro Gly Val Thr Pro
                215                 220                 225

Ser Lys Ser Thr Ser Ala Ser Ala Ile Met Asn Gly Gly Lys Pro
                230                 235                 240

Val Asn Lys Ser Lys Thr Thr
                245
```

<210> 285
<211> 2849
<212> DNA
<213> Homo Sapien

<220>
<221> unsure
<222> 2715
<223> unknown base

<400> 285
```
cggacgcgtg ggcggacgcg tgggcggacg cgtgggcgga cgcgtgggct 50

ggttcaggtc caggttttgc tttgatcctt ttcaaaaact ggagacacag 100

aagagggctc taggaaaaag ttttggatgg gattatgtgg aaactaccct 150

gcgattctct gctgccagag caggctcggc gcttccaccc cagtgcagcc 200

ttcccctggc ggtggtgaaa gagactcggg agtcgctgct tccaaagtgc 250

ccgccgtgag tgagctctca ccccagtcag ccaaatgagc ctcttcgggc 300

ttctcctgct gacatctgcc ctggccggcc agagacaggg gactcaggcg 350

gaatccaacc tgagtagtaa attccagttt tccagcaaca aggaacagaa 400

cggagtacaa gatcctcagc atgagagaat tattactgtg tctactaatg 450

gaagtattca cagcccaagg tttcctcata cttatccaag aaatacggtc 500

ttggtatgga gattagtagc agtagaggaa aatgtatgga tacaacttac 550

gtttgatgaa agatttgggc ttgaagaccc agaagatgac atatgcaagt 600

atgattttgt agaagttgag gaacccagtg atggaactat attagggcgc 650
```

tggtgtggtt ctggtactgt accaggaaaa cagatttcta aaggaaatca 700
aattaggata agatttgtat ctgatgaata ttttccttct gaaccagggt 750
tctgcatcca ctacaacatt gtcatgccac aattcacaga agctgtgagt 800
ccttcagtgc taccccccttc agctttgcca ctggacctgc ttaataatgc 850
tataactgcc tttagtacct tggaagacct tattcgatat cttgaaccag 900
agagatggca gttggactta gaagatctat ataggccaac ttggcaactt 950
cttggcaagg cttttgtttt tggaagaaaa tccagagtgg tggatctgaa 1000
ccttctaaca gaggaggtaa gattatacag ctgcacacct cgtaacttct 1050
cagtgtccat aagggaagaa ctaaagagaa ccgataccat tttctggcca 1100
ggttgtctcc tggttaaacg ctgtggtggg aactgtgcct gttgtctcca 1150
caattgcaat gaatgtcaat gtgtcccaag caaagttact aaaaaatacc 1200
acgaggtcct tcagttgaga ccaaagaccg gtgtcagggg attgcacaaa 1250
tcactcaccg acgtggccct ggagcaccat gaggagtgtg actgtgtgtg 1300
cagagggagc acaggaggat agccgcatca ccaccagcag ctcttgccca 1350
gagctgtgca gtgcagtggc tgattctatt agagaacgta tgcgttatct 1400
ccatccttaa tctcagttgt ttgcttcaag gacctttcat cttcaggatt 1450
tacagtgcat tctgaaagag gagacatcaa acagaattag gagttgtgca 1500
acagctcttt tgagaggagg cctaaaggac aggagaaaag gtcttcaatc 1550
gtggaaagaa aattaaatgt tgtattaaat agatcaccag ctagtttcag 1600
agttaccatg tacgtattcc actagctggg ttctgtattt cagttctttc 1650
gatacggctt agggtaatgt cagtacagga aaaaaactgt gcaagtgagc 1700
acctgattcc gttgccttgc ttaactctaa agctccatgt cctgggccta 1750
aaatcgtata aaatctggat tttttttttt ttttttgctc atattcacat 1800
atgtaaacca gaacattcta tgtactacaa acctggtttt taaaaaggaa 1850
ctatgttgct atgaattaaa cttgtgtcat gctgatagga cagactggat 1900
ttttcatatt tcttattaaa atttctgcca tttagaagaa gagaactaca 1950
ttcatggttt ggaagagata aacctgaaaa gaagagtggc cttatcttca 2000
ctttatcgat aagtcagttt atttgtttca ttgtgtacat ttttatattc 2050
tccttttgac attataactg ttggcttttc taatcttgtt aaatatatct 2100
atttttacca aaggtattta atattctttt ttatgacaac ttagatcaac 2150
tatttttagc ttggtaaatt tttctaaaca caattgttat agccagagga 2200
acaaagatga tataaaatat tgttgctctg acaaaaatac atgtatttca 2250

ttctcgtatg gtgctagagt tagattaatc tgcattttaa aaaactgaat 2300

tggaatagaa ttggtaagtt gcaaagactt tttgaaaata attaaattat 2350

catatcttcc attcctgtta ttggagatga aaataaaaag caacttatga 2400

aagtagacat tcagatccag ccattactaa cctattcctt ttttggggaa 2450

atctgagcct agctcagaaa aacataaagc accttgaaaa agacttggca 2500

gcttcctgat aaagcgtgct gtgctgtgca gtaggaacac atcctattta 2550

ttgtgatgtt gtggttttat tatcttaaac tctgttccat acacttgtat 2600

aaatacatgg atatttttat gtacagaagt atgtctctta accagttcac 2650

ttattgtact ctggcaattt aaaagaaaat cagtaaaata ttttgcttgt 2700

aaaatgctta atatngtgcc taggttatgt ggtgactatt tgaatcaaaa 2750

atgtattgaa tcatcaaata aaagaatgtg gctattttgg ggagaaaatt 2800

aaaaaaaaaa aaaaaaaaaa aggtttaggg ataacagggt aatgcggcc 2849

<210> 286
<211> 345
<212> PRT
<213> Homo Sapien

<400> 286

```
Met Ser Leu Phe Gly Leu Leu Leu Leu Thr Ser Ala Leu Ala Gly
  1               5                  10                  15

Gln Arg Gln Gly Thr Gln Ala Glu Ser Asn Leu Ser Ser Lys Phe
                 20                  25                  30

Gln Phe Ser Ser Asn Lys Glu Gln Asn Gly Val Gln Asp Pro Gln
                 35                  40                  45

His Glu Arg Ile Ile Thr Val Ser Thr Asn Gly Ser Ile His Ser
                 50                  55                  60

Pro Arg Phe Pro His Thr Tyr Pro Arg Asn Thr Val Leu Val Trp
                 65                  70                  75

Arg Leu Val Ala Val Glu Glu Asn Val Trp Ile Gln Leu Thr Phe
                 80                  85                  90

Asp Glu Arg Phe Gly Leu Glu Asp Pro Glu Asp Asp Ile Cys Lys
                 95                 100                 105

Tyr Asp Phe Val Glu Val Glu Glu Pro Ser Asp Gly Thr Ile Leu
                110                 115                 120

Gly Arg Trp Cys Gly Ser Gly Thr Val Pro Gly Lys Gln Ile Ser
                125                 130                 135

Lys Gly Asn Gln Ile Arg Ile Arg Phe Val Ser Asp Glu Tyr Phe
                140                 145                 150

Pro Ser Glu Pro Gly Phe Cys Ile His Tyr Asn Ile Val Met Pro
                155                 160                 165
```

```
Gln Phe Thr Glu Ala Val Ser Pro Ser Val Leu Pro Pro Ser Ala
                170                 175                 180

Leu Pro Leu Asp Leu Leu Asn Asn Ala Ile Thr Ala Phe Ser Thr
                185                 190                 195

Leu Glu Asp Leu Ile Arg Tyr Leu Glu Pro Glu Arg Trp Gln Leu
                200                 205                 210

Asp Leu Glu Asp Leu Tyr Arg Pro Thr Trp Gln Leu Leu Gly Lys
                215                 220                 225

Ala Phe Val Phe Gly Arg Lys Ser Arg Val Val Asp Leu Asn Leu
                230                 235                 240

Leu Thr Glu Glu Val Arg Leu Tyr Ser Cys Thr Pro Arg Asn Phe
                245                 250                 255

Ser Val Ser Ile Arg Glu Glu Leu Lys Arg Thr Asp Thr Ile Phe
                260                 265                 270

Trp Pro Gly Cys Leu Leu Val Lys Arg Cys Gly Gly Asn Cys Ala
                275                 280                 285

Cys Cys Leu His Asn Cys Asn Glu Cys Gln Cys Val Pro Ser Lys
                290                 295                 300

Val Thr Lys Lys Tyr His Glu Val Leu Gln Leu Arg Pro Lys Thr
                305                 310                 315

Gly Val Arg Gly Leu His Lys Ser Leu Thr Asp Val Ala Leu Glu
                320                 325                 330

His His Glu Glu Cys Asp Cys Val Cys Arg Gly Ser Thr Gly Gly
                335                 340                 345
```

<210> 287
<211> 1496
<212> DNA
<213> Homo Sapien

<400> 287

```
cagcgctgac tgcgccgcgg agaaagccag tgggaaccca gacccatagg 50
agacccgcgt ccccgctcgg cctggccagg ccccgcgcta tggagttcct 100
ctgggcccct ctcttgggtc tgtgctgcag tctggccgct gctgatcgcc 150
acaccgtctt ctggaacagt tcaaatccca agttccggaa tgaggactac 200
accatacatg tgcagctgaa tgactacgtg gacatcatct gtccgcacta 250
tgaagatcac tctgtggcag acgctgccat ggagcagtac atactgtacc 300
tggtggagca tgaggagtac cagctgtgcc agccccagtc caaggaccaa 350
gtccgctggc agtgcaaccg gcccagtgcc aagcatggcc cggagaagct 400
gtctgagaag ttccagcgct tcacaccttt caccctgggc aaggagttca 450
aagaaggaca cagctactac tacatctcca aacccatcca ccagcatgaa 500
gaccgctgct tgaggttgaa ggtgactgtc agtggcaaaa tcactcacag 550
```

tcctcaggcc catgacaatc cacaggagaa gagacttgca gcagatgacc 600

cagaggtgcg ggttctacat agcatcggtc acagtgctgc cccacgcctc 650

ttcccacttg cctggactgt gctgctcctt ccacttctgc tgctgcaaac 700

cccgtgaagg tgtgtgccac acctggcctt aaagagggac aggctgaaga 750

gagggacagg cactccaaac ctgtcttggg gccactttca gagcccccag 800

ccctgggaac cactcccacc acaggcataa gctatcacct agcagcctca 850

aaacgggtca atattaaggt tttcaaccgg aaggaggcca accagcccga 900

cagtgccatc cccaccttca cctcggaggg atggagaaag aagtggagac 950

agtcctttcc caccattcct gcctttaagc caaagaaaca agctgtgcag 1000

gcatggtccc ttaaggcaca gtgggagctg agctggaagg ggccacgtgg 1050

atgggcaaag cttgtcaaag atgccccctt caggagagag ccaggatgcc 1100

cagatgaact gactgaagga aaagcaagaa acagtttctt gcttggaagc 1150

caggtacagg agaggcagca tgcttgggct gacccagcat ctcccagcaa 1200

gacctcatct gtggagctgc cacagagaag tttgtagcca ggtactgcat 1250

tctctcccat cctggggcag cactccccag agctgtgcca gcaggggggc 1300

tgtgccaacc tgttcttaga gtgtagctgt aagggcagtg cccatgtgta 1350

cattctgcct agagtgtagc ctaaagggca gggcccacgt gtatagtatc 1400

tgtatataag ttgctgtgtg tctgtcctga tttctacaac tggagttttt 1450

ttatacaatg ttctttgtct caaaataaag caatgtgttt tttcgg 1496

<210> 288
<211> 204
<212> PRT
<213> Homo Sapien

<400> 288
Met Glu Phe Leu Trp Ala Pro Leu Leu Gly Leu Cys Cys Ser Leu
1               5                   10                  15

Ala Ala Ala Asp Arg His Thr Val Phe Trp Asn Ser Ser Asn Pro
                20                  25                  30

Lys Phe Arg Asn Glu Asp Tyr Thr Ile His Val Gln Leu Asn Asp
                35                  40                  45

Tyr Val Asp Ile Ile Cys Pro His Tyr Glu Asp His Ser Ala Asp
                50                  55                  60

Ala Ala Met Glu Gln Tyr Ile Leu Tyr Leu Val Glu His Glu Glu
                65                  70                  75

Tyr Gln Leu Cys Gln Pro Gln Ser Lys Asp Gln Val Arg Trp Gln
                80                  85                  90

Cys Asn Arg Pro Ser Ala Lys His Gly Pro Glu Lys Leu Ser Glu
                95                  100                 105

```
Lys Phe Gln Arg Phe Thr Pro Phe Thr Leu Gly Lys Glu Phe Lys
                110                 115                 120

Glu Gly His Ser Tyr Tyr Tyr Ile Ser Lys Pro Ile His Gln His
                125                 130                 135

Glu Asp Arg Cys Leu Arg Leu Lys Val Thr Val Ser Gly Lys Ile
                140                 145                 150

Thr His Ser Pro Gln Ala His Asp Asn Pro Gln Glu Lys Arg Leu
                155                 160                 165

Ala Ala Asp Asp Pro Glu Val Arg Val Leu His Ser Ile Gly His
                170                 175                 180

Ser Ala Ala Pro Arg Leu Phe Pro Leu Ala Trp Thr Val Leu Leu
                185                 190                 195

Leu Pro Leu Leu Leu Leu Gln Thr Pro
                200
```

<210> 289
<211> 1838
<212> DNA
<213> Homo Sapien

<400> 289

```
cggacgcgtg ggcggacgcg tgggcggccc acggcgcccg cgggctgggg 50

cggtcgcttc ttccttctcc gtggcctacg agggtcccca gcctgggtaa 100

agatggcccc atggccccg aagggcctag tcccagctgt gctctggggc 150

ctcagcctct tcctcaacct cccaggacct atctggctcc agccctctcc 200

acctccccag tcttctcccc cgcctcagcc ccatccgtgt catacctgcc 250

ggggactggt tgacagcttt aacaagggcc tggagagaac catccgggac 300

aactttggag gtggaaacac tgcctgggag gaagagaatt tgtccaaata 350

caaagacagt gagacccgcc tggtagaggt gctggagggt gtgtgcagca 400

agtcagactt cgagtgccac cgcctgctgg agctgagtga ggagctggtg 450

gagagctggt ggtttcacaa gcagcaggag gccccggacc tcttccagtg 500

gctgtgctca gattccctga agctctgctg ccccgcaggc accttcgggc 550

cctcctgcct tccctgtcct gggggaacag agaggccctg cggtggctac 600

gggcagtgtg aaggagaagg gacacgaggg ggcagcgggc actgtgactg 650

ccaagccggc tacgggggtg aggcctgtgg ccagtgtggc cttggctact 700

ttgaggcaga acgcaacgcc agccatctgg tatgttcggc ttgttttggc 750

ccctgtgccc gatgctcagg acctgaggaa tcaaactgtt tgcaatgcaa 800

gaagggctgg gccctgcatc acctcaagtg tgtagacatt gatgagtgtg 850

gcacagaggg agccaactgt ggagctgacc aattctgcgt gaacactgag 900
```

ggctcctatg agtgccgaga ctgtgccaag gcctgcctag gctgcatggg 950

ggcagggcca ggtcgctgta agaagtgtag ccctggctat cagcaggtgg 1000

gctccaagtg tctcgatgtg gatgagtgtg agacagaggt gtgtccggga 1050

gagaacaagc agtgtgaaaa caccgagggc ggttatcgct gcatctgtgc 1100

cgagggctac aagcagatgg aaggcatctg tgtgaaggag cagatcccag 1150

agtcagcagg cttcttctca gagatgacag aagacgagtt ggtggtgctg 1200

cagcagatgt tctttggcat catcatctgt gcactggcca cgctggctgc 1250

taagggcgac ttggtgttca ccgccatctt cattggggct gtggcggcca 1300

tgactggcta ctggttgtca gagcgcagtg accgtgtgct ggagggcttc 1350

atcaagggca gataatcgcg gccaccacct gtaggacctc ctcccaccca 1400

cgctgccccc agagcttggg ctgccctcct gctggacact caggacagct 1450

tggtttattt ttgagagtgg ggtaagcacc cctacctgcc ttacagagca 1500

gcccaggtac ccaggcccgg gcagacaagg cccctggggt aaaaagtagc 1550

cctgaaggtg gataccatga gctcttcacc tggcggggac tggcaggctt 1600

cacaatgtgt gaatttcaaa agtttttcct taatggtggc tgctagagct 1650

ttggcccctg cttaggatta ggtggtcctc acaggggtgg ggccatcaca 1700

gctccctcct gccagctgca tgctgccagt tcctgttctg tgttcaccac 1750

atccccacac cccattgcca cttatttatt catctcagga aataaagaaa 1800

ggtcttggaa agttaaaaaa aaaaaaaaaa aaaaaaaa 1838

<210> 290
<211> 420
<212> PRT
<213> Homo Sapien

<400> 290
Met Ala Pro Trp Pro Pro Lys Gly Leu Val Pro Ala Val Leu Trp
1 5 10 15

Gly Leu Ser Leu Phe Leu Asn Leu Pro Gly Pro Ile Trp Leu Gln
20 25 30

Pro Ser Pro Pro Pro Gln Ser Ser Pro Pro Pro Gln Pro His Pro
35 40 45

Cys His Thr Cys Arg Gly Leu Val Asp Ser Phe Asn Lys Gly Leu
50 55 60

Glu Arg Thr Ile Arg Asp Asn Phe Gly Gly Gly Asn Thr Ala Trp
65 70 75

Glu Glu Glu Asn Leu Ser Lys Tyr Lys Asp Ser Glu Thr Arg Leu
80 85 90

Val Glu Val Leu Glu Gly Val Cys Ser Lys Ser Asp Phe Glu Cys
95 100 105

```
His Arg Leu Leu Glu Leu Ser Glu Glu Leu Val Glu Ser Trp Trp
                110                 115                 120

Phe His Lys Gln Gln Glu Ala Pro Asp Leu Phe Gln Trp Leu Cys
                125                 130                 135

Ser Asp Ser Leu Lys Leu Cys Cys Pro Ala Gly Thr Phe Gly Pro
                140                 145                 150

Ser Cys Leu Pro Cys Pro Gly Gly Thr Glu Arg Pro Cys Gly Gly
                155                 160                 165

Tyr Gly Gln Cys Glu Gly Glu Gly Thr Arg Gly Gly Ser Gly His
                170                 175                 180

Cys Asp Cys Gln Ala Gly Tyr Gly Gly Glu Ala Cys Gly Gln Cys
                185                 190                 195

Gly Leu Gly Tyr Phe Glu Ala Glu Arg Asn Ala Ser His Leu Val
                200                 205                 210

Cys Ser Ala Cys Phe Gly Pro Cys Ala Arg Cys Ser Gly Pro Glu
                215                 220                 225

Glu Ser Asn Cys Leu Gln Cys Lys Lys Gly Trp Ala Leu His His
                230                 235                 240

Leu Lys Cys Val Asp Ile Asp Glu Cys Gly Thr Glu Gly Ala Asn
                245                 250                 255

Cys Gly Ala Asp Gln Phe Cys Val Asn Thr Glu Gly Ser Tyr Glu
                260                 265                 270

Cys Arg Asp Cys Ala Lys Ala Cys Leu Gly Cys Met Gly Ala Gly
                275                 280                 285

Pro Gly Arg Cys Lys Lys Cys Ser Pro Gly Tyr Gln Gln Val Gly
                290                 295                 300

Ser Lys Cys Leu Asp Val Asp Glu Cys Glu Thr Glu Val Cys Pro
                305                 310                 315

Gly Glu Asn Lys Gln Cys Glu Asn Thr Glu Gly Gly Tyr Arg Cys
                320                 325                 330

Ile Cys Ala Glu Gly Tyr Lys Gln Met Glu Gly Ile Cys Val Lys
                335                 340                 345

Glu Gln Ile Pro Glu Ser Ala Gly Phe Phe Ser Glu Met Thr Glu
                350                 355                 360

Asp Glu Leu Val Val Leu Gln Gln Met Phe Phe Gly Ile Ile Ile
                365                 370                 375

Cys Ala Leu Ala Thr Leu Ala Ala Lys Gly Asp Leu Val Phe Thr
                380                 385                 390

Ala Ile Phe Ile Gly Ala Val Ala Ala Met Thr Gly Tyr Trp Leu
                395                 400                 405

Ser Glu Arg Ser Asp Arg Val Leu Glu Gly Phe Ile Lys Gly Arg
                410                 415                 420
```

<210> 291
<211> 2447
<212> DNA
<213> Homo Sapien

<400> 291
caggtccaac tgcacctcgg ttctatcgat tgaattcccc ggggatcctc 50

tagagatccc tcgacctcga cccacgcgtc cgaacacagg tccttgttgc 100

tgcagagaag cagttgtttt gctggaagga gggagtgcgc gggctgcccc 150

gggctcctcc ctgccgcctc ctctcagtgg atggttccag gcaccctgtc 200

tggggcaggg agggcacagg cctgcacatc gaaggtgggg tgggaccagg 250

ctgcccctcg ccccagcatc caagtcctcc cttgggcgcc cgtggccctg 300

cagactctca gggctaaggt cctctgttgc tttttggttc caccttagaa 350

gaggctccgc ttgactaaga gtagcttgaa ggaggcacca tgcaggagct 400

gcatctgctc tggtgggcgc ttctcctggg cctggctcag gcctgccctg 450

agccctgcga ctgtggggaa aagtatggct tccagatcgc cgactgtgcc 500

taccgcgacc tagaatccgt gccgcctggc ttcccggcca atgtgactac 550

actgagcctg tcagccaacc ggctgccagg cttgccggag ggtgccttca 600

gggaggtgcc cctgctgcag tcgctgtggc tggcacacaa tgagatccgc 650

acggtggccg ccggagccct ggcctctctg agccatctca agagcctgga 700

cctcagccac aatctcatct ctgactttgc ctggagcgac ctgcacaacc 750

tcagtgccct ccaattgctc aagatggaca gcaacgagct gaccttcatc 800

ccccgcgacg ccttccgcag cctccgtgct ctgcgctcgc tgcaactcaa 850

ccacaaccgc ttgcacacat tggccgaggg caccttcacc ccgctcaccg 900

cgctgtccca cctgcagatc aacgagaacc ccttcgactg cacctgcggc 950

atcgtgtggc tcaagacatg ggccctgacc acggccgtgt ccatcccgga 1000

gcaggacaac atcgcctgca cctcacccca tgtgctcaag ggtacaccgc 1050

tgagccgcct gccgccactg ccatgctcgg cgccctcagt gcagctcagc 1100

taccaaccca gccaggatgg tgccgagctg cggcctggtt ttgtgctggc 1150

actgcactgt gatgtggacg ggcagccggc ccctcagctt cactggcaca 1200

tccagatacc cagtggcatt gtggagatca ccagccccaa cgtgggcact 1250

gatgggcgtg ccctgcctgg cacccctgtg gccagctccc agccgcgctt 1300

ccaggccttt gccaatggca gcctgcttat ccccgacttt ggcaagctgg 1350

aggaaggcac ctacagctgc ctggccacca atgagctggg cagtgctgag 1400

agctcagtgg acgtggcact ggccacgccc ggtgagggtg gtgaggacac 1450

actggggcgc aggttccatg gcaaagcggt tgagggaaag ggctgctata 1500

cggttgacaa cgaggtgcag ccatcagggc cggaggacaa tgtggtcatc 1550

atctacctca gccgtgctgg gaaccctgag gctgcagtcg cagaaggggt 1600

ccctgggcag ctgcccccag gcctgctcct gctgggccaa agcctcctcc 1650

tcttcttctt cctcacctcc ttctagcccc acccagggct tccctaactc 1700

ctccccttgc ccctaccaat gccccttttaa gtgctgcagg ggtctggggt 1750

tggcaactcc tgaggcctgc atgggtgact tcacattttc ctacctctcc 1800

ttctaatctc ttctagagca cctgctatcc ccaacttcta gacctgctcc 1850

aaactagtga ctaggataga atttgatccc ctaactcact gtctgcggtg 1900

ctcattgctg ctaacagcat tgcctgtgct ctcctctcag gggcagcatg 1950

ctaacggggc gacgtcctaa tccaactggg agaagcctca gtggtggaat 2000

tccaggcact gtgactgtca agctggcaag ggccaggatt gggggaatgg 2050

agctggggct tagctgggag gtggtctgaa gcagacaggg aatgggagag 2100

gaggatggga agtagacagt ggctggtatg gctctgaggc tccctggggc 2150

ctgctcaagc tcctcctgct ccttgctgtt ttctgatgat ttgggggctt 2200

gggagtccct ttgtcctcat ctgagactga aatgtgggga tccaggatgg 2250

ccttccttcc tcttaccctt cctccctcag cctgcaacct ctatcctgga 2300

acctgtcctc cctttctccc caactatgca tctgttgtct gctcctctgc 2350

aaaggccagc cagcttggga gcagcagaga aataaacagc atttctgatg 2400

ccaaaaaaaa aaaaaaaaaa gggcggccgc gactctagag tcgacct 2447

<210> 292
<211> 428
<212> PRT
<213> Homo Sapien

<400> 292
Met Gln Glu Leu His Leu Leu Trp Trp Ala Leu Leu Leu Gly Leu
1               5                   10                  15

Ala Gln Ala Cys Pro Glu Pro Cys Asp Cys Gly Glu Lys Tyr Gly
                20                  25                  30

Phe Gln Ile Ala Asp Cys Ala Tyr Arg Asp Leu Glu Ser Val Pro
                35                  40                  45

Pro Gly Phe Pro Ala Asn Val Thr Thr Leu Ser Leu Ser Ala Asn
                50                  55                  60

Arg Leu Pro Gly Leu Pro Glu Gly Ala Phe Arg Glu Val Pro Leu
                65                  70                  75

Leu Gln Ser Leu Trp Leu Ala His Asn Glu Ile Arg Thr Val Ala
                80                  85                  90

Ala Gly Ala Leu Ala Ser Leu Ser His Leu Lys Ser Leu Asp Leu
95 100 105

Ser His Asn Leu Ile Ser Asp Phe Ala Trp Ser Asp Leu His Asn
110 115 120

Leu Ser Ala Leu Gln Leu Leu Lys Met Asp Ser Asn Glu Leu Thr
125 130 135

Phe Ile Pro Arg Asp Ala Phe Arg Ser Leu Arg Ala Leu Arg Ser
140 145 150

Leu Gln Leu Asn His Asn Arg Leu His Thr Leu Ala Glu Gly Thr
155 160 165

Phe Thr Pro Leu Thr Ala Leu Ser His Leu Gln Ile Asn Glu Asn
170 175 180

Pro Phe Asp Cys Thr Cys Gly Ile Val Trp Leu Lys Thr Trp Ala
185 190 195

Leu Thr Thr Ala Val Ser Ile Pro Glu Gln Asp Asn Ile Ala Cys
200 205 210

Thr Ser Pro His Val Leu Lys Gly Thr Pro Leu Ser Arg Leu Pro
215 220 225

Pro Leu Pro Cys Ser Ala Pro Ser Val Gln Leu Ser Tyr Gln Pro
230 235 240

Ser Gln Asp Gly Ala Glu Leu Arg Pro Gly Phe Val Leu Ala Leu
245 250 255

His Cys Asp Val Asp Gly Gln Pro Ala Pro Gln Leu His Trp His
260 265 270

Ile Gln Ile Pro Ser Gly Ile Val Glu Ile Thr Ser Pro Asn Val
275 280 285

Gly Thr Asp Gly Arg Ala Leu Pro Gly Thr Pro Val Ala Ser Ser
290 295 300

Gln Pro Arg Phe Gln Ala Phe Ala Asn Gly Ser Leu Leu Ile Pro
305 310 315

Asp Phe Gly Lys Leu Glu Glu Gly Thr Tyr Ser Cys Leu Ala Thr
320 325 330

Asn Glu Leu Gly Ser Ala Glu Ser Ser Val Asp Val Ala Leu Ala
335 340 345

Thr Pro Gly Glu Gly Gly Glu Asp Thr Leu Gly Arg Arg Phe His
350 355 360

Gly Lys Ala Val Glu Gly Lys Gly Cys Tyr Thr Val Asp Asn Glu
365 370 375

Val Gln Pro Ser Gly Pro Glu Asp Asn Val Val Ile Ile Tyr Leu
380 385 390

Ser Arg Ala Gly Asn Pro Glu Ala Ala Val Ala Glu Gly Val Pro
395 400 405

Gly Gln Leu Pro Pro Gly Leu Leu Leu Leu Gly Gln Ser Leu Leu

410 415 420

Leu Phe Phe Phe Leu Thr Ser Phe
425

<210> 293
<211> 3449
<212> DNA
<213> Homo Sapien

<400> 293
acttggagca agcggcggcg gcggagacag aggcagaggc agaagctggg 50

gctccgtcct cgcctcccac gagcgatccc cgaggagagc cgcggccctc 100

ggcgaggcga agaggccgac gaggaagacc cgggtggctg cgcccctgcc 150

tcgcttccca ggcgccggcg gctgcagcct tgcccctctt gctcgccttg 200

aaaatggaaa agatgctcgc aggctgcttt ctgctgatcc tcggacagat 250

cgtcctcctc cctgccgagg ccagggagcg gtcacgtggg aggtccatct 300

ctaggggcag acacgctcgg acccacccgc agacggccct tctggagagt 350

tcctgtgaga acaagcgggc agacctggtt ttcatcattg acagctctcg 400

cagtgtcaac acccatgact atgcaaaggt caaggagttc atcgtggaca 450

tcttgcaatt cttggacatt ggtcctgatg tcacccgagt gggcctgctc 500

caatatggca gcactgtcaa gaatgagttc tccctcaaga ccttcaagag 550

gaagtccgag gtggagcgtg ctgtcaagag gatgcggcat ctgtccacgg 600

gcaccatgac tgggctggcc atccagtatg ccctgaacat cgcattctca 650

gaagcagagg gggcccggcc cctgagggag aatgtgccac gggtcataat 700

gatcgtgaca gatgggagac ctcaggactc cgtggccgag gtggctgcta 750

aggcacggga cacgggcatc ctaatctttg ccattggtgt gggccaggta 800

gacttcaaca ccttgaagtc cattgggagt gagccccatg aggaccatgt 850

cttccttgtg gccaatttca gccagattga gacgctgacc tccgtgttcc 900

agaagaagtt gtgcacggcc cacatgtgca gcaccctgga gcataactgt 950

gcccacttct gcatcaacat ccctggctca tacgtctgca ggtgcaaaca 1000

aggctacatt ctcaactcgg atcagacgac ttgcagaatc caggatctgt 1050

gtgccatgga ggaccacaac tgtgagcagc tctgtgtgaa tgtgccgggc 1100

tccttcgtct gccagtgcta cagtggctac gccctggctg aggatgggaa 1150

gaggtgtgtg gctgtggact actgtgcctc agaaaaccac ggatgtgaac 1200

atgagtgtgt aaatgctgat ggctcctacc tttgccagtg ccatgaagga 1250

tttgctctta acccagatga aaaaacgtgc acaaggatca actactgtgc 1300

actgaacaaa ccgggctgtg agcatgagtg cgtcaacatg gaggagagct 1350

actactgccg ctgccaccgt ggctacactc tggaccccaa tggcaaaacc 1400
tgcagccgag tggaccactg tgcacagcag gaccatggct gtgagcagct 1450
gtgtctgaac acggaggatt ccttcgtctg ccagtgctca gaaggcttcc 1500
tcatcaacga ggacctcaag acctgctccc gggtggatta ctgcctgctg 1550
agtgaccatg gttgtgaata ctcctgtgtc aacatggaca gatcctttgc 1600
ctgtcagtgt cctgagggac acgtgctccg cagcgatggg aagacgtgtg 1650
caaaattgga ctcttgtgct ctgggggacc acggttgtga acattcgtgt 1700
gtaagcagtg aagattcgtt tgtgtgccag tgctttgaag gttatatact 1750
ccgtgaagat ggaaaaacct gcagaaggaa agatgtctgc caagctatag 1800
accatggctg tgaacacatt tgtgtgaaca gtgacgactc atacacgtgc 1850
gagtgcttgg agggattccg gctcgctgag gatgggaaac gctgccgaag 1900
gaaggatgtc tgcaaatcaa cccaccatgg ctgcgaacac atttgtgtta 1950
ataatgggaa ttcctacatc tgcaaatgct cagagggatt tgttctagct 2000
gaggacggaa gacggtgcaa gaaatgcact gaaggcccaa ttgacctggt 2050
ctttgtgatc gatggatcca agagtcttgg agaagagaat tttgaggtcg 2100
tgaagcagtt tgtcactgga attatagatt ccttgacaat ttcccccaaa 2150
gccgctcgag tggggctgct ccagtattcc acacaggtcc acacagagtt 2200
cactctgaga aacttcaact cagccaaaga catgaaaaaa gccgtggccc 2250
acatgaaata catgggaaag ggctctatga ctgggctggc cctgaaacac 2300
atgtttgaga gaagttttac ccaaggagaa ggggccaggc ccctttccac 2350
aagggtgccc agagcagcca ttgtgttcac cgacggacgg gctcaggatg 2400
acgtctccga gtgggccagt aaagccaagg ccaatggtat cactatgtat 2450
gctgttgggg taggaaaagc cattgaggag gaactacaag agattgcctc 2500
tgagcccaca aacaagcatc tcttctatgc cgaagacttc agcacaatgg 2550
atgagataag tgaaaaactc aagaaaggca tctgtgaagc tctagaagac 2600
tccgatggaa gacaggactc tccagcaggg gaactgccaa aaacggtcca 2650
acagccaaca gaatctgagc cagtcaccat aaatatccaa gacctacttt 2700
cctgttctaa ttttgcagtg caacacagat atctgtttga agaagacaat 2750
cttttacggt ctacacaaaa gctttcccat tcaacaaaac cttcaggaag 2800
ccctttggaa gaaaaacacg atcaatgcaa atgtgaaaac cttataatgt 2850
tccagaacct tgcaaacgaa gaagtaagaa aattaacaca gcgcttagaa 2900
gaaatgacac agagaatgga agccctggaa aatcgcctga gatacagatg 2950

```
aagattagaa atcgcgacac atttgtagtc attgtatcac ggattacaat 3000
gaacgcagtg cagagcccca aagctcaggc tattgttaaa tcaataatgt 3050
tgtgaagtaa aacaatcagt actgagaaac ctggtttgcc acagaacaaa 3100
gacaagaagt atacactaac ttgtataaat ttatctagga aaaaaatcct 3150
tcagaattct aagatgaatt taccaggtga gaatgaataa gctatgcaag 3200
gtattttgta atatactgtg gacacaactt gcttctgcct catcctgcct 3250
tagtgtgcaa tctcatttga ctatacgata aagtttgcac agtcttactt 3300
ctgtagaaca ctggccatag gaaatgctgt ttttttgtac tggactttac 3350
cttgatatat gtatatggat gtatgcataa aatcatagga catatgtact 3400
tgtggaacaa gttggatttt ttatacaata ttaaaattca ccacttcag 3449
```

```
<210> 294
<211> 915
<212> PRT
<213> Homo Sapien

<400> 294
Met Glu Lys Met Leu Ala Gly Cys Phe Leu Leu Ile Leu Gly Gln
1               5                   10                  15

Ile Val Leu Leu Pro Ala Glu Ala Arg Glu Arg Ser Arg Gly Arg
                20                  25                  30

Ser Ile Ser Arg Gly Arg His Ala Arg Thr His Pro Gln Thr Ala
                35                  40                  45

Leu Leu Glu Ser Ser Cys Glu Asn Lys Arg Ala Asp Leu Val Phe
                50                  55                  60

Ile Ile Asp Ser Ser Arg Ser Val Asn Thr His Asp Tyr Ala Lys
                65                  70                  75

Val Lys Glu Phe Ile Val Asp Ile Leu Gln Phe Leu Asp Ile Gly
                80                  85                  90

Pro Asp Val Thr Arg Val Gly Leu Leu Gln Tyr Gly Ser Thr Val
                95                  100                 105

Lys Asn Glu Phe Ser Leu Lys Thr Phe Lys Arg Lys Ser Glu Val
                110                 115                 120

Glu Arg Ala Val Lys Arg Met Arg His Leu Ser Thr Gly Thr Met
                125                 130                 135

Thr Gly Leu Ala Ile Gln Tyr Ala Leu Asn Ile Ala Phe Ser Glu
                140                 145                 150

Ala Glu Gly Ala Arg Pro Leu Arg Glu Asn Val Pro Arg Val Ile
                155                 160                 165

Met Ile Val Thr Asp Gly Arg Pro Gln Asp Ser Val Ala Glu Val
                170                 175                 180

Ala Ala Lys Ala Arg Asp Thr Gly Ile Leu Ile Phe Ala Ile Gly
```

```
                185                 190                 195

Val Gly Gln Val Asp Phe Asn Thr Leu Lys Ser Ile Gly Ser Glu
                200                 205                 210

Pro His Glu Asp His Val Phe Leu Val Ala Asn Phe Ser Gln Ile
                215                 220                 225

Glu Thr Leu Thr Ser Val Phe Gln Lys Lys Leu Cys Thr Ala His
                230                 235                 240

Met Cys Ser Thr Leu Glu His Asn Cys Ala His Phe Cys Ile Asn
                245                 250                 255

Ile Pro Gly Ser Tyr Val Cys Arg Cys Lys Gln Gly Tyr Ile Leu
                260                 265                 270

Asn Ser Asp Gln Thr Thr Cys Arg Ile Gln Asp Leu Cys Ala Met
                275                 280                 285

Glu Asp His Asn Cys Glu Gln Leu Cys Val Asn Val Pro Gly Ser
                290                 295                 300

Phe Val Cys Gln Cys Tyr Ser Gly Tyr Ala Leu Ala Glu Asp Gly
                305                 310                 315

Lys Arg Cys Val Ala Val Asp Tyr Cys Ala Ser Glu Asn His Gly
                320                 325                 330

Cys Glu His Glu Cys Val Asn Ala Asp Gly Ser Tyr Leu Cys Gln
                335                 340                 345

Cys His Glu Gly Phe Ala Leu Asn Pro Asp Glu Lys Thr Cys Thr
                350                 355                 360

Arg Ile Asn Tyr Cys Ala Leu Asn Lys Pro Gly Cys Glu His Glu
                365                 370                 375

Cys Val Asn Met Glu Glu Ser Tyr Tyr Cys Arg Cys His Arg Gly
                380                 385                 390

Tyr Thr Leu Asp Pro Asn Gly Lys Thr Cys Ser Arg Val Asp His
                395                 400                 405

Cys Ala Gln Gln Asp His Gly Cys Glu Gln Leu Cys Leu Asn Thr
                410                 415                 420

Glu Asp Ser Phe Val Cys Gln Cys Ser Glu Gly Phe Leu Ile Asn
                425                 430                 435

Glu Asp Leu Lys Thr Cys Ser Arg Val Asp Tyr Cys Leu Leu Ser
                440                 445                 450

Asp His Gly Cys Glu Tyr Ser Cys Val Asn Met Asp Arg Ser Phe
                455                 460                 465

Ala Cys Gln Cys Pro Glu Gly His Val Leu Arg Ser Asp Gly Lys
                470                 475                 480

Thr Cys Ala Lys Leu Asp Ser Cys Ala Leu Gly Asp His Gly Cys
                485                 490                 495

Glu His Ser Cys Val Ser Ser Glu Asp Ser Phe Val Cys Gln Cys
                500                 505                 510
```

```
Phe Glu Gly Tyr Ile Leu Arg Glu Asp Gly Lys Thr Cys Arg Arg
                515                 520                 525

Lys Asp Val Cys Gln Ala Ile Asp His Gly Cys Glu His Ile Cys
                530                 535                 540

Val Asn Ser Asp Asp Ser Tyr Thr Cys Glu Cys Leu Glu Gly Phe
                545                 550                 555

Arg Leu Ala Glu Asp Gly Lys Arg Cys Arg Arg Lys Asp Val Cys
                560                 565                 570

Lys Ser Thr His His Gly Cys Glu His Ile Cys Val Asn Asn Gly
                575                 580                 585

Asn Ser Tyr Ile Cys Lys Cys Ser Glu Gly Phe Val Leu Ala Glu
                590                 595                 600

Asp Gly Arg Arg Cys Lys Lys Cys Thr Glu Gly Pro Ile Asp Leu
                605                 610                 615

Val Phe Val Ile Asp Gly Ser Lys Ser Leu Gly Glu Glu Asn Phe
                620                 625                 630

Glu Val Val Lys Gln Phe Val Thr Gly Ile Ile Asp Ser Leu Thr
                635                 640                 645

Ile Ser Pro Lys Ala Ala Arg Val Gly Leu Leu Gln Tyr Ser Thr
                650                 655                 660

Gln Val His Thr Glu Phe Thr Leu Arg Asn Phe Asn Ser Ala Lys
                665                 670                 675

Asp Met Lys Lys Ala Val Ala His Met Lys Tyr Met Gly Lys Gly
                680                 685                 690

Ser Met Thr Gly Leu Ala Leu Lys His Met Phe Glu Arg Ser Phe
                695                 700                 705

Thr Gln Gly Glu Gly Ala Arg Pro Leu Ser Thr Arg Val Pro Arg
                710                 715                 720

Ala Ala Ile Val Phe Thr Asp Gly Arg Ala Gln Asp Asp Val Ser
                725                 730                 735

Glu Trp Ala Ser Lys Ala Lys Ala Asn Gly Ile Thr Met Tyr Ala
                740                 745                 750

Val Gly Val Gly Lys Ala Ile Glu Glu Glu Leu Gln Glu Ile Ala
                755                 760                 765

Ser Glu Pro Thr Asn Lys His Leu Phe Tyr Ala Glu Asp Phe Ser
                770                 775                 780

Thr Met Asp Glu Ile Ser Glu Lys Leu Lys Lys Gly Ile Cys Glu
                785                 790                 795

Ala Leu Glu Asp Ser Asp Gly Arg Gln Asp Ser Pro Ala Gly Glu
                800                 805                 810

Leu Pro Lys Thr Val Gln Gln Pro Thr Glu Ser Glu Pro Val Thr
                815                 820                 825
```

Ile Asn Ile Gln Asp Leu Leu Ser Cys Ser Asn Phe Ala Val Gln
830 835 840

His Arg Tyr Leu Phe Glu Glu Asp Asn Leu Leu Arg Ser Thr Gln
845 850 855

Lys Leu Ser His Ser Thr Lys Pro Ser Gly Ser Pro Leu Glu Glu
860 865 870

Lys His Asp Gln Cys Lys Cys Glu Asn Leu Ile Met Phe Gln Asn
875 880 885

Leu Ala Asn Glu Glu Val Arg Lys Leu Thr Gln Arg Leu Glu Glu
890 895 900

Met Thr Gln Arg Met Glu Ala Leu Glu Asn Arg Leu Arg Tyr Arg
905 910 915

<210> 295
<211> 1364
<212> DNA
<213> Homo Sapien

<400> 295
ggccggagca gcacggccgc aggacctgga gctccggctg cgtcttcccg 50

cagcgctacc cgccatgcgc ctgccgcgcc gggccgcgct ggggctcctg 100

ccgcttctgc tgctgctgcc gcccgcgccg gaggccgcca agaagccgac 150

gccctgccac cggtgccggg ggctggtgga caagtttaac caggggatgg 200

tggacaccgc aaagaagaac tttggcggcg ggaacacggc ttgggaggaa 250

aagacgctgt ccaagtacga gtccagcgag attcgcctgc tggagatcct 300

ggaggggctg tgcgagagca gcgacttcga atgcaatcag atgctagagg 350

cgcaggagga gcacctggag gcctggtggc tgcagctgaa gagcgaatat 400

cctgacttat tcgagtggtt ttgtgtgaag acactgaaag tgtgctgctc 450

tccaggaacc tacggtcccg actgtctcgc atgccagggc ggatcccaga 500

ggccctgcag cgggaatggc cactgcagcg gagatgggag cagacagggc 550

gacgggtcct gccggtgcca catggggtac cagggcccgc tgtgcactga 600

ctgcatggac ggctacttca gctcgctccg gaacgagacc cacagcatct 650

gcacagcctg tgacgagtcc tgcaagacgt gctcgggcct gaccaacaga 700

gactgcggcg agtgtgaagt gggctgggtg ctggacgagg gcgcctgtgt 750

ggatgtggac gagtgtgcgg ccgagccgcc tccctgcagc gctgcgcagt 800

tctgtaagaa cgccaacggc tcctacacgt gcgaagagtg tgactccagc 850

tgtgtgggct gcacagggga aggcccagga aactgtaaag agtgtatctc 900

tggctacgcg agggagcacg gacagtgtgc agatgtggac gagtgctcac 950

tagcagaaaa aacctgtgtg aggaaaaacg aaaactgcta caatactcca 1000

gggagctacg tctgtgtgtg tcctgacggc ttcgaagaaa cggaagatgc 1050

ctgtgtgccg ccggcagagg ctgaagccac agaaggagaa agcccgacac 1100

agctgccctc ccgcgaagac ctgtaatgtg ccggacttac cctttaaatt 1150

attcagaagg atgtcccgtg gaaaatgtgg ccctgaggat gccgtctcct 1200

gcagtggaca gcggcgggga gaggctgcct gctctctaac ggttgattct 1250

catttgtccc ttaaacagct gcatttcttg gttgttctta aacagacttg 1300

tatattttga tacagttctt tgtaataaaa ttgaccattg taggtaatca 1350

ggaggaaaaa aaaa 1364

<210> 296
<211> 353
<212> PRT
<213> Homo Sapien

<400> 296
Met Arg Leu Pro Arg Arg Ala Ala Leu Gly Leu Leu Pro Leu Leu
1 5 10 15

Leu Leu Leu Pro Pro Ala Pro Glu Ala Ala Lys Lys Pro Thr Pro
20 25 30

Cys His Arg Cys Arg Gly Leu Val Asp Lys Phe Asn Gln Gly Met
35 40 45

Val Asp Thr Ala Lys Lys Asn Phe Gly Gly Gly Asn Thr Ala Trp
50 55 60

Glu Glu Lys Thr Leu Ser Lys Tyr Glu Ser Ser Glu Ile Arg Leu
65 70 75

Leu Glu Ile Leu Glu Gly Leu Cys Glu Ser Ser Asp Phe Glu Cys
80 85 90

Asn Gln Met Leu Glu Ala Gln Glu Glu His Leu Glu Ala Trp Trp
95 100 105

Leu Gln Leu Lys Ser Glu Tyr Pro Asp Leu Phe Glu Trp Phe Cys
110 115 120

Val Lys Thr Leu Lys Val Cys Cys Ser Pro Gly Thr Tyr Gly Pro
125 130 135

Asp Cys Leu Ala Cys Gln Gly Gly Ser Gln Arg Pro Cys Ser Gly
140 145 150

Asn Gly His Cys Ser Gly Asp Gly Ser Arg Gln Gly Asp Gly Ser
155 160 165

Cys Arg Cys His Met Gly Tyr Gln Gly Pro Leu Cys Thr Asp Cys
170 175 180

Met Asp Gly Tyr Phe Ser Ser Leu Arg Asn Glu Thr His Ser Ile
185 190 195

Cys Thr Ala Cys Asp Glu Ser Cys Lys Thr Cys Ser Gly Leu Thr
200 205 210

```
Asn Arg Asp Cys Gly Glu Cys Glu Val Gly Trp Val Leu Asp Glu
                215                 220                 225

Gly Ala Cys Val Asp Val Asp Glu Cys Ala Ala Glu Pro Pro Pro
                230                 235                 240

Cys Ser Ala Ala Gln Phe Cys Lys Asn Ala Asn Gly Ser Tyr Thr
                245                 250                 255

Cys Glu Glu Cys Asp Ser Ser Cys Val Gly Cys Thr Gly Glu Gly
                260                 265                 270

Pro Gly Asn Cys Lys Glu Cys Ile Ser Gly Tyr Ala Arg Glu His
                275                 280                 285

Gly Gln Cys Ala Asp Val Asp Glu Cys Ser Leu Ala Glu Lys Thr
                290                 295                 300

Cys Val Arg Lys Asn Glu Asn Cys Tyr Asn Thr Pro Gly Ser Tyr
                305                 310                 315

Val Cys Val Cys Pro Asp Gly Phe Glu Glu Thr Glu Asp Ala Cys
                320                 325                 330

Val Pro Pro Ala Glu Ala Glu Ala Thr Glu Gly Glu Ser Pro Thr
                335                 340                 345

Gln Leu Pro Ser Arg Glu Asp Leu
                350
```

<210> 297
<211> 2639
<212> DNA
<213> Homo Sapien

<400> 297

```
gacatcggag gtgggctagc actgaaactg cttttcaaga cgaggaagag 50

gaggagaaag agaaagaaga ggaagatgtt gggcaacatt tatttaacat 100

gctccacagc ccggaccctg gcatcatgct gctattcctg caaatactga 150

agaagcatgg gatttaaata ttttacttct aaataaatga attactcaat 200

ctcctatgac catctataca tactccacct tcaaaaagta catcaatatt 250

atatcattaa ggaaatagta accttctctt ctccaatatg catgacattt 300

ttggacaatg caattgtggc actggcactt atttcagtga agaaaaactt 350

tgtggttcta tggcattcat catttgacaa atgcaagcat cttccttatc 400

aatcagctcc tattgaactt actagcactg actgtggaat ccttaagggc 450

ccattacatt tctgaagaag aaagctaaga tgaaggacat gccactccga 500

attcatgtgc tacttggcct agctatcact acactagtac aagctgtaga 550

taaaaaagtg gattgtccac ggttatgtac gtgtgaaatc aggccttggt 600

ttacacccag atccatttat atggaagcat ctacagtgga ttgtaatgat 650

ttaggtcttt taactttccc agccagattg ccagctaaca cacagattct 700
```

tctcctacag actaacaata ttgcaaaaat tgaatactcc acagactttc 750

cagtaaacct tactggcctg gatttatctc aaaacaattt atcttcagtc 800

accaatatta atgtaaaaaa gatgcctcag ctcctttctg tgtacctaga 850

ggaaaacaaa cttactgaac tgcctgaaaa atgtctgtcc gaactgagca 900

acttacaaga actctatatt aatcacaact tgctttctac aatttcacct 950

ggagccttta ttggcctaca taatcttctt cgacttcatc tcaattcaaa 1000

tagattgcag atgatcaaca gtaagtggtt tgatgctctt ccaaatctag 1050

agattctgat gattggggaa aatccaatta tcagaatcaa agacatgaac 1100

tttaagcctc ttatcaatct tcgcagcctg gttatagctg gtataaacct 1150

cacagaaata ccagataacg ccttggttgg actggaaaac ttagaaagca 1200

tctcttttta cgataacagg cttattaaag taccccatgt tgctcttcaa 1250

aaagttgtaa atctcaaatt tttggatcta aataaaaatc ctattaatag 1300

aatacgaagg ggtgatttta gcaatatgct acacttaaaa gagttgggga 1350

taaataatat gcctgagctg atttccatcg atagtcttgc tgtggataac 1400

ctgccagatt taagaaaaat agaagctact aacaacccta gattgtctta 1450

cattcacccc aatgcatttt tcagactccc caagctggaa tcactcatgc 1500

tgaacagcaa tgctctcagt gccctgtacc atggtaccat tgagtctctg 1550

ccaaacctca aggaaatcag catacacagt aaccccatca ggtgtgactg 1600

tgtcatccgt tggatgaaca tgaacaaaac caacattcga ttcatggagc 1650

cagattcact gttttgcgtg gacccacctg aattccaagg tcagaatgtt 1700

cggcaagtgc atttcaggga catgatggaa atttgtctcc ctcttatagc 1750

tcctgagagc tttccttcta atctaaatgt agaagctggg agctatgttt 1800

cctttcactg tagagctact gcagaaccac agcctgaaat ctactggata 1850

acaccttctg gtcaaaaact cttgcctaat accctgacag acaagttcta 1900

tgtccattct gagggaacac tagatataaa tggcgtaact cccaaagaag 1950

ggggtttata tacttgtata gcaactaacc tagttggcgc tgacttgaag 2000

tctgttatga tcaaagtgga tggatctttt ccacaagata acaatggctc 2050

tttgaatatt aaaataagag atattcaggc caattcagtt ttggtgtcct 2100

ggaaagcaag ttctaaaatt ctcaaatcta gtgttaaatg gacagccttt 2150

gtcaagactg aaaattctca tgctgcgcaa agtgctcgaa taccatctga 2200

tgtcaaggta tataatctta ctcatctgaa tccatcaact gagtataaaa 2250

tttgtattga tattcccacc atctatcaga aaaacagaaa aaaatgtgta 2300

aatgtcacca ccaaaggttt gcaccctgat caaaaagagt atgaaaagaa 2350

taataccaca acacttatgg cctgtcttgg aggccttctg gggattattg 2400

gtgtgatatg tcttatcagc tgcctctctc cagaaatgaa ctgtgatggt 2450

ggacacagct atgtgaggaa ttacttacag aaaccaacct ttgcattagg 2500

tgagctttat cctcctctga taaatctctg ggaagcagga aaagaaaaaa 2550

gtacatcact gaaagtaaaa gcaactgtta taggtttacc aacaaatatg 2600

tcctaaaaac caccaaggaa acctactcca aaaatgaac 2639

<210> 298
<211> 708
<212> PRT
<213> Homo Sapien

<400> 298

```
Met Lys Asp Met Pro Leu Arg Ile His Val Leu Leu Gly Leu Ala
 1               5                  10                  15

Ile Thr Thr Leu Val Gln Ala Val Asp Lys Lys Val Asp Cys Pro
                20                  25                  30

Arg Leu Cys Thr Cys Glu Ile Arg Pro Trp Phe Thr Pro Arg Ser
                35                  40                  45

Ile Tyr Met Glu Ala Ser Thr Val Asp Cys Asn Asp Leu Gly Leu
                50                  55                  60

Leu Thr Phe Pro Ala Arg Leu Pro Ala Asn Thr Gln Ile Leu Leu
                65                  70                  75

Leu Gln Thr Asn Asn Ile Ala Lys Ile Glu Tyr Ser Thr Asp Phe
                80                  85                  90

Pro Val Asn Leu Thr Gly Leu Asp Leu Ser Gln Asn Asn Leu Ser
                95                  100                 105

Ser Val Thr Asn Ile Asn Val Lys Lys Met Pro Gln Leu Leu Ser
                110                 115                 120

Val Tyr Leu Glu Glu Asn Lys Leu Thr Glu Leu Pro Glu Lys Cys
                125                 130                 135

Leu Ser Glu Leu Ser Asn Leu Gln Glu Leu Tyr Ile Asn His Asn
                140                 145                 150

Leu Leu Ser Thr Ile Ser Pro Gly Ala Phe Ile Gly Leu His Asn
                155                 160                 165

Leu Leu Arg Leu His Leu Asn Ser Asn Arg Leu Gln Met Ile Asn
                170                 175                 180

Ser Lys Trp Phe Asp Ala Leu Pro Asn Leu Glu Ile Leu Met Ile
                185                 190                 195

Gly Glu Asn Pro Ile Ile Arg Ile Lys Asp Met Asn Phe Lys Pro
                200                 205                 210

Leu Ile Asn Leu Arg Ser Leu Val Ile Ala Gly Ile Asn Leu Thr
                215                 220                 225
```

Glu Ile Pro Asp Asn Ala Leu Val Gly Leu Glu Asn Leu Glu Ser
230 235 240

Ile Ser Phe Tyr Asp Asn Arg Leu Ile Lys Val Pro His Val Ala
245 250 255

Leu Gln Lys Val Val Asn Leu Lys Phe Leu Asp Leu Asn Lys Asn
260 265 270

Pro Ile Asn Arg Ile Arg Arg Gly Asp Phe Ser Asn Met Leu His
275 280 285

Leu Lys Glu Leu Gly Ile Asn Asn Met Pro Glu Leu Ile Ser Ile
290 295 300

Asp Ser Leu Ala Val Asp Asn Leu Pro Asp Leu Arg Lys Ile Glu
305 310 315

Ala Thr Asn Asn Pro Arg Leu Ser Tyr Ile His Pro Asn Ala Phe
320 325 330

Phe Arg Leu Pro Lys Leu Glu Ser Leu Met Leu Asn Ser Asn Ala
335 340 345

Leu Ser Ala Leu Tyr His Gly Thr Ile Glu Ser Leu Pro Asn Leu
350 355 360

Lys Glu Ile Ser Ile His Ser Asn Pro Ile Arg Cys Asp Cys Val
365 370 375

Ile Arg Trp Met Asn Met Asn Lys Thr Asn Ile Arg Phe Met Glu
380 385 390

Pro Asp Ser Leu Phe Cys Val Asp Pro Pro Glu Phe Gln Gly Gln
395 400 405

Asn Val Arg Gln Val His Phe Arg Asp Met Met Glu Ile Cys Leu
410 415 420

Pro Leu Ile Ala Pro Glu Ser Phe Pro Ser Asn Leu Asn Val Glu
425 430 435

Ala Gly Ser Tyr Val Ser Phe His Cys Arg Ala Thr Ala Glu Pro
440 445 450

Gln Pro Glu Ile Tyr Trp Ile Thr Pro Ser Gly Gln Lys Leu Leu
455 460 465

Pro Asn Thr Leu Thr Asp Lys Phe Tyr Val His Ser Glu Gly Thr
470 475 480

Leu Asp Ile Asn Gly Val Thr Pro Lys Glu Gly Gly Leu Tyr Thr
485 490 495

Cys Ile Ala Thr Asn Leu Val Gly Ala Asp Leu Lys Ser Val Met
500 505 510

Ile Lys Val Asp Gly Ser Phe Pro Gln Asp Asn Asn Gly Ser Leu
515 520 525

Asn Ile Lys Ile Arg Asp Ile Gln Ala Asn Ser Val Leu Val Ser
530 535 540

Trp Lys Ala Ser Ser Lys Ile Leu Lys Ser Ser Val Lys Trp Thr
545 550 555

Ala Phe Val Lys Thr Glu Asn Ser His Ala Ala Gln Ser Ala Arg
560 565 570

Ile Pro Ser Asp Val Lys Val Tyr Asn Leu Thr His Leu Asn Pro
575 580 585

Ser Thr Glu Tyr Lys Ile Cys Ile Asp Ile Pro Thr Ile Tyr Gln
590 595 600

Lys Asn Arg Lys Lys Cys Val Asn Val Thr Thr Lys Gly Leu His
605 610 615

Pro Asp Gln Lys Glu Tyr Glu Lys Asn Asn Thr Thr Thr Leu Met
620 625 630

Ala Cys Leu Gly Gly Leu Leu Gly Ile Ile Gly Val Ile Cys Leu
635 640 645

Ile Ser Cys Leu Ser Pro Glu Met Asn Cys Asp Gly Gly His Ser
650 655 660

Tyr Val Arg Asn Tyr Leu Gln Lys Pro Thr Phe Ala Leu Gly Glu
665 670 675

Leu Tyr Pro Pro Leu Ile Asn Leu Trp Glu Ala Gly Lys Glu Lys
680 685 690

Ser Thr Ser Leu Lys Val Lys Ala Thr Val Ile Gly Leu Pro Thr
695 700 705

Asn Met Ser

<210> 299
<211> 1102
<212> DNA
<213> Homo Sapien

<400> 299
gctgtgggaa cctctccacg cgcacgaact cagccaacga tttctgatag 50

atttttggga gtttgaccag agatgcaagg ggtgaaggag cgcttcctac 100

cgttagggaa ctctggggac agagcgcccc ggccgcctga tggccgaggc 150

agggtgcgac ccaggaccca ggacggcgtc gggaaccata ccatggcccg 200

gatccccaag accctaaagt tcgtcgtcgt catcgtcgcg gtcctgctgc 250

cagtcctagc ttactctgcc accactgccc ggcaggagga agttccccag 300

cagacagtgg ccccacagca acagaggcac agcttcaagg gggaggagtg 350

tccagcagga tctcatagat cagaacatac tggagcctgt aacccgtgca 400

cagagggtgt ggattacacc aacgcttcca acaatgaacc ttcttgcttc 450

ccatgtacag tttgtaaatc agatcaaaaa cataaaagtt cctgcaccat 500

gaccagagac acagtgtgtc agtgtaaaga aggcaccttc cggaatgaaa 550

actccccaga gatgtgccgg aagtgtagca ggtgccctag tggggaagtc 600

caagtcagta attgtacgtc ctgggatgat atccagtgtg ttgaagaatt 650

tggtgccaat gccactgtgg aaaccccagc tgctgaagag acaatgaaca 700

ccagcccggg gactcctgcc ccagctgctg aagagacaat gaacaccagc 750

ccagggactc ctgccccagc tgctgaagag acaatgacca ccagcccggg 800

gactcctgcc ccagctgctg aagagacaat gaccaccagc ccggggactc 850

ctgccccagc tgctgaagag acaatgacca ccagcccggg gactcctgcc 900

tcttctcatt acctctcatg caccatcgta gggatcatag ttctaattgt 950

gcttctgatt gtgtttgttt gaaagacttc actgtggaag aaattccttc 1000

cttacctgaa aggttcaggt aggcgctggc tgagggcggg gggcgctgga 1050

cactctctgc cctgcctccc tctgctgtgt tcccacagac agaaacgcct 1100

gc 1102

<210> 300
<211> 259
<212> PRT
<213> Homo Sapien

<400> 300
Met Ala Arg Ile Pro Lys Thr Leu Lys Phe Val Val Val Ile Val
1 5 10 15

Ala Val Leu Leu Pro Val Leu Ala Tyr Ser Ala Thr Thr Ala Arg
20 25 30

Gln Glu Glu Val Pro Gln Gln Thr Val Ala Pro Gln Gln Gln Arg
35 40 45

His Ser Phe Lys Gly Glu Glu Cys Pro Ala Gly Ser His Arg Ser
50 55 60

Glu His Thr Gly Ala Cys Asn Pro Cys Thr Glu Gly Val Asp Tyr
65 70 75

Thr Asn Ala Ser Asn Asn Glu Pro Ser Cys Phe Pro Cys Thr Val
80 85 90

Cys Lys Ser Asp Gln Lys His Lys Ser Ser Cys Thr Met Thr Arg
95 100 105

Asp Thr Val Cys Gln Cys Lys Glu Gly Thr Phe Arg Asn Glu Asn
110 115 120

Ser Pro Glu Met Cys Arg Lys Cys Ser Arg Cys Pro Ser Gly Glu
125 130 135

Val Gln Val Ser Asn Cys Thr Ser Trp Asp Asp Ile Gln Cys Val
140 145 150

Glu Glu Phe Gly Ala Asn Ala Thr Val Glu Thr Pro Ala Ala Glu
155 160 165

Glu Thr Met Asn Thr Ser Pro Gly Thr Pro Ala Pro Ala Ala Glu

170 175 180

Glu Thr Met Asn Thr Ser Pro Gly Thr Pro Ala Pro Ala Ala Glu
185 190 195

Glu Thr Met Thr Thr Ser Pro Gly Thr Pro Ala Pro Ala Ala Glu
200 205 210

Glu Thr Met Thr Thr Ser Pro Gly Thr Pro Ala Pro Ala Ala Glu
215 220 225

Glu Thr Met Thr Thr Ser Pro Gly Thr Pro Ala Ser Ser His Tyr
230 235 240

Leu Ser Cys Thr Ile Val Gly Ile Ile Val Leu Ile Val Leu Leu
245 250 255

Ile Val Phe Val

<210> 301
<211> 1576
<212> DNA
<213> Homo Sapien

<400> 301
cacaagcatc ttaatttgaa tccacaaagt ttcatgtaat gaaaagaaat 50

acataatttt aattcaaccc gagtgttttc caagaagatt gtatttgctt 100

aaattgctac agtaattcaa gagacagccc tgtctggaca cagagttact 150

gtggattttt aagagactca gttaaagaat ttaggaattt ctgattcatt 200

taaaggattt acaaattcat caacccctga aaactaaagc aaattgaaca 250

ggaaaaaaaa aaagaagatg ggttttttaa gtccaatata tgttattttc 300

ttcttttttg gagtcaaagt acattgccaa tatgaaactt atcagtggga 350

tgaagactat gaccaagagc cagatgatga ttaccaaaca ggattcccat 400

ttcgtcaaaa tgtagactac ggagttcctt ttcatcagta tactttaggc 450

tgtgtcagtg aatgcttctg tccaactaac tttccatcat caatgtactg 500

tgataatcgc aaactcaaga ctatcccaaa tattccgatg cacattcagc 550

aactctacct tcagttcaat gaaattgagg ctgtgactgc aaattcattc 600

atcaatgcaa ctcatcttaa agaaattaac ctcagccaca acaaaattaa 650

atctcaaaag attgattatg gtgtgtttgc taagcttcca aatctactac 700

aacttcatct agagcataat aatttagaag aatttccatt tcctcttcct 750

aaatctctgg aaagactcct tcttggttac aatgaaatct ccaaactgca 800

gacaaatgct atggatgggc tagtaaactt gaccatgctt gatctctgtt 850

ataattatct tcatgattct ctgctaaaag acaaaatctt tgccaaaatg 900

gaaaaactaa tgcagctcaa cctctgcagt aacagattag aatcaatgcc 950

tcctggtttg ccttcttcac ttatgtatct gtctttagaa aataattcaa 1000

tttcttctat acccgaaaaa tacttcgaca aacttccaaa acttcatact 1050

ctaagaatgt cacacaacaa actacaagac atcccatata atatttttaa 1100

tcttcccaac attgtagaac tcagtgttgg acacaacaaa ttgaagcaag 1150

cattctatat tccaagaaat ttggaacacc tatacctaca aaataatgaa 1200

atagaaaaga tgaatcttac agtgatgtgt ccttctattg acccactaca 1250

ttaccaccat ttaacataca ttcgtgtgga ccaaaataaa ctaaaagaac 1300

caataagctc atacatcttc ttctgcttcc ctcatataca cactatttat 1350

tatggtgaac aacgaagcac taatggtcaa acaatacaac taaagacaca 1400

agttttcagg agatttccag atgatgatga tgaaagtgaa gatcacgatg 1450

atcctgacaa tgctcatgag agcccagaac aagaaggagc agaagggcac 1500

tttgaccttc attattatga aaatcaagaa tagcaagaaa ctatataggt 1550

atacacttac gacttcacaa aaccta 1576

<210> 302
<211> 421
<212> PRT
<213> Homo Sapien

<400> 302
Met Gly Phe Leu Ser Pro Ile Tyr Val Ile Phe Phe Phe Phe Gly
1 5 10 15

Val Lys Val His Cys Gln Tyr Glu Thr Tyr Gln Trp Asp Glu Asp
20 25 30

Tyr Asp Gln Glu Pro Asp Asp Asp Tyr Gln Thr Gly Phe Pro Phe
35 40 45

Arg Gln Asn Val Asp Tyr Gly Val Pro Phe His Gln Tyr Thr Leu
50 55 60

Gly Cys Val Ser Glu Cys Phe Cys Pro Thr Asn Phe Pro Ser Ser
65 70 75

Met Tyr Cys Asp Asn Arg Lys Leu Lys Thr Ile Pro Asn Ile Pro
80 85 90

Met His Ile Gln Gln Leu Tyr Leu Gln Phe Asn Glu Ile Glu Ala
95 100 105

Val Thr Ala Asn Ser Phe Ile Asn Ala Thr His Leu Lys Glu Ile
110 115 120

Asn Leu Ser His Asn Lys Ile Lys Ser Gln Lys Ile Asp Tyr Gly
125 130 135

Val Phe Ala Lys Leu Pro Asn Leu Leu Gln Leu His Leu Glu His
140 145 150

Asn Asn Leu Glu Glu Phe Pro Phe Pro Leu Pro Lys Ser Leu Glu
155 160 165

Arg Leu Leu Leu Gly Tyr Asn Glu Ile Ser Lys Leu Gln Thr Asn
170 175 180

Ala Met Asp Gly Leu Val Asn Leu Thr Met Leu Asp Leu Cys Tyr
185 190 195

Asn Tyr Leu His Asp Ser Leu Leu Lys Asp Lys Ile Phe Ala Lys
200 205 210

Met Glu Lys Leu Met Gln Leu Asn Leu Cys Ser Asn Arg Leu Glu
215 220 225

Ser Met Pro Pro Gly Leu Pro Ser Ser Leu Met Tyr Leu Ser Leu
230 235 240

Glu Asn Asn Ser Ile Ser Ser Ile Pro Glu Lys Tyr Phe Asp Lys
245 250 255

Leu Pro Lys Leu His Thr Leu Arg Met Ser His Asn Lys Leu Gln
260 265 270

Asp Ile Pro Tyr Asn Ile Phe Asn Leu Pro Asn Ile Val Glu Leu
275 280 285

Ser Val Gly His Asn Lys Leu Lys Gln Ala Phe Tyr Ile Pro Arg
290 295 300

Asn Leu Glu His Leu Tyr Leu Gln Asn Asn Glu Ile Glu Lys Met
305 310 315

Asn Leu Thr Val Met Cys Pro Ser Ile Asp Pro Leu His Tyr His
320 325 330

His Leu Thr Tyr Ile Arg Val Asp Gln Asn Lys Leu Lys Glu Pro
335 340 345

Ile Ser Ser Tyr Ile Phe Phe Cys Phe Pro His Ile His Thr Ile
350 355 360

Tyr Tyr Gly Glu Gln Arg Ser Thr Asn Gly Gln Thr Ile Gln Leu
365 370 375

Lys Thr Gln Val Phe Arg Arg Phe Pro Asp Asp Asp Asp Glu Ser
380 385 390

Glu Asp His Asp Asp Pro Asp Asn Ala His Glu Ser Pro Glu Gln
395 400 405

Glu Gly Ala Glu Gly His Phe Asp Leu His Tyr Tyr Glu Asn Gln
410 415 420

Glu

<210> 303
<211> 1305
<212> DNA
<213> Homo Sapien

<400> 303
gcccgggact ggcgcaaggt gcccaagcaa ggaaagaaat aatgaagaga 50

cacatgtgtt agctgcagcc ttttgaaaca cgcaagaagg aaatcaatag 100

tgtggacagg gctggaacct ttaccacgct tgttggagta gatgaggaat 150

gggctcgtga ttatgctgac attccagcat gaatctggta gacctgtggt 200

taacccgttc cctctccatg tgtctcctcc tacaaagttt tgttcttatg 250

atactgtgct ttcattctgc cagtatgtgt cccaagggct gtctttgttc 300

ttcctctggg ggtttaaatg tcacctgtag caatgcaaat ctcaaggaaa 350

tacctagaga tcttcctcct gaaacagtct tactgtatct ggactccaat 400

cagatcacat ctattcccaa tgaaattttt aaggacctcc atcaactgag 450

agttctcaac ctgtccaaaa atggcattga gtttatcgat gagcatgcct 500

tcaaaggagt agctgaaacc ttgcagactc tggacttgtc cgacaatcgg 550

attcaaagtg tgcacaaaaa tgccttcaat aacctgaagg ccagggccag 600

aattgccaac aacccctggc actgcgactg tactctacag caagttctga 650

ggagcatggc gtccaatcat gagacagccc acaacgtgat ctgtaaaacg 700

tccgtgttgg atgaacatgc tggcagacca ttcctcaatg ctgccaacga 750

cgctgacctt tgtaacctcc ctaaaaaaac taccgattat gccatgctgg 800

tcaccatgtt tggctggttc actatggtga tctcatatgt ggtatattat 850

gtgaggcaaa atcaggagga tgcccggaga cacctcgaat acttgaaatc 900

cctgccaagc aggcagaaga aagcagatga acctgatgat attagcactg 950

tggtatagtg tccaaactga ctgtcattga gaaagaaaga aagtagtttg 1000

cgattgcagt agaaataagt ggtttacttc tcccatccat tgtaaacatt 1050

tgaaactttg tatttcagtt ttttttgaat tatgccactg ctgaactttt 1100

aacaaacact acaacataaa taatttgagt ttaggtgatc caccccttaa 1150

ttgtaccccc gatggtatat ttctgagtaa gctactatct gaacattagt 1200

tagatccatc tcactattta ataatgaaat ttattttttt aatttaaaag 1250

caaataaaag cttaactttg aaccatggga aaaaaaaaaa aaaaaaaaaa 1300

aaaca 1305

<210> 304
<211> 259
<212> PRT
<213> Homo Sapien

<400> 304

```
Met Asn Leu Val Asp Leu Trp Leu Thr Arg Ser Leu Ser Met Cys
  1               5                  10                  15

Leu Leu Leu Gln Ser Phe Val Leu Met Ile Leu Cys Phe His Ser
                 20                  25                  30

Ala Ser Met Cys Pro Lys Gly Cys Leu Cys Ser Ser Ser Gly Gly
```

```
                35                  40                  45

Leu Asn Val Thr Cys Ser Asn Ala Asn Leu Lys Glu Ile Pro Arg
                50                  55                  60

Asp Leu Pro Pro Glu Thr Val Leu Leu Tyr Leu Asp Ser Asn Gln
                65                  70                  75

Ile Thr Ser Ile Pro Asn Glu Ile Phe Lys Asp Leu His Gln Leu
                80                  85                  90

Arg Val Leu Asn Leu Ser Lys Asn Gly Ile Glu Phe Ile Asp Glu
                95                  100                 105

His Ala Phe Lys Gly Val Ala Glu Thr Leu Gln Thr Leu Asp Leu
                110                 115                 120

Ser Asp Asn Arg Ile Gln Ser Val His Lys Asn Ala Phe Asn Asn
                125                 130                 135

Leu Lys Ala Arg Ala Arg Ile Ala Asn Asn Pro Trp His Cys Asp
                140                 145                 150

Cys Thr Leu Gln Gln Val Leu Arg Ser Met Ala Ser Asn His Glu
                155                 160                 165

Thr Ala His Asn Val Ile Cys Lys Thr Ser Val Leu Asp Glu His
                170                 175                 180

Ala Gly Arg Pro Phe Leu Asn Ala Ala Asn Asp Ala Asp Leu Cys
                185                 190                 195

Asn Leu Pro Lys Lys Thr Thr Asp Tyr Ala Met Leu Val Thr Met
                200                 205                 210

Phe Gly Trp Phe Thr Met Val Ile Ser Tyr Val Val Tyr Tyr Val
                215                 220                 225

Arg Gln Asn Gln Glu Asp Ala Arg Arg His Leu Glu Tyr Leu Lys
                230                 235                 240

Ser Leu Pro Ser Arg Gln Lys Lys Ala Asp Glu Pro Asp Asp Ile
                245                 250                 255

Ser Thr Val Val
```

```
<210> 305
<211> 2822
<212> DNA
<213> Homo Sapien

<400> 305
cgccaccact gcggccaccg ccaatgaaac gcctcccgct cctagtggtt 50

ttttccactt tgttgaattg ttcctatact caaaattgca ccaagacacc 100

ttgtctccca aatgcaaaat gtgaaatacg caatggaatt gaagcctgct 150

attgcaacat gggattttca ggaaatggtg tcacaatttg tgaagatgat 200

aatgaatgtg gaaatttaac tcagtcctgt ggcgaaaatg ctaattgcac 250

taacacagaa ggaagttatt attgtatgtg tgtacctggc ttcagatcca 300
```

gcagtaacca agacaggttt atcactaatg atggaaccgt ctgtatagaa 350
aatgtgaatg caaactgcca tttagataat gtctgtatag ctgcaaatat 400
taataaaact ttaacaaaaa tcagatccat aaaagaacct gtggctttgc 450
tacaagaagt ctatagaaat tctgtgacag atctttcacc aacagatata 500
attacatata tagaaatatt agctgaatca tcttcattac taggttacaa 550
gaacaacact atctcagcca aggacaccct ttctaactca actcttactg 600
aatttgtaaa aaccgtgaat aattttgttc aaagggatac atttgtagtt 650
tgggacaagt tatctgtgaa tcataggaga acacatctta caaaactcat 700
gcacactgtt gaacaagcta ctttaaggat atcccagagc ttccaaaaga 750
ccacagagtt tgatacaaat tcaacggata tagctctcaa agttttcttt 800
tttgattcat ataacatgaa acatattcat cctcatatga atatggatgg 850
agactacata aatatatttc caaagagaaa agctgcatat gattcaaatg 900
gcaatgttgc agttgcattt ttatattata agagtattgg tcctttgctt 950
tcatcatctg acaacttctt attgaaacct caaaattatg ataattctga 1000
agaggaggaa agagtcatat cttcagtaat ttcagtctca atgagctcaa 1050
acccacccac attatatgaa cttgaaaaaa taacatttac attaagtcat 1100
cgaaaggtca cagataggta taggagtcta tgtgcatttt ggaattactc 1150
acctgatacc atgaatggca gctggtcttc agagggctgt gagctgacat 1200
actcaaatga gacccacacc tcatgccgct gtaatcacct gacacatttt 1250
gcaattttga tgtcctctgg tccttccatt ggtattaaag attataatat 1300
tcttacaagg atcactcaac taggaataat tatttcactg atttgtcttg 1350
ccatatgcat tttaccttc tggttcttca gtgaaattca aagcaccagg 1400
acaacaattc acaaaaatct ttgctgtagc ctatttcttg ctgaacttgt 1450
ttttcttgtt gggatcaata caaatactaa taagctcttc tgttcaatca 1500
ttgccggact gctacactac ttctttttag ctgcttttgc atggatgtgc 1550
attgaaggca tacatctcta tctcattgtt gtgggtgtca tctacaacaa 1600
gggatttttg cacaagaatt tttatatctt tggctatcta agcccagccg 1650
tggtagttgg attttcggca gcactaggat acagatatta tggcacaacc 1700
aaagtatgtt ggcttagcac cgaaaacaac tttatttgga gttttatagg 1750
accagcatgc ctaatcattc ttgttaatct cttggctttt ggagtcatca 1800
tatacaaagt ttttcgtcac actgcagggt tgaaaccaga agttagttgc 1850
tttgagaaca taaggtcttg tgcaagagga gccctcgctc ttctgttcct 1900

tctcggcacc acctggatct ttggggttct ccatgttgtg cacgcatcag 1950

tggttacagc ttacctcttc acagtcagca atgctttcca ggggatgttc 2000

attttttat tcctgtgtgt tttatctaga aagattcaag aagaatatta 2050

cagattgttc aaaaatgtcc cctgttgttt tggatgttta aggtaaacat 2100

agagaatggt ggataattac aactgcacaa aaataaaaat tccaagctgt 2150

ggatgaccaa tgtataaaaa tgactcatca aattatccaa ttattaacta 2200

ctagacaaaa agtattttaa atcagttttt ctgtttatgc tataggaact 2250

gtagataata aggtaaaatt atgtatcata tagatatact atgtttttct 2300

atgtgaaata gttctgtcaa aaatagtatt gcagatattt ggaaagtaat 2350

tggtttctca ggagtgatat cactgcaccc aaggaaagat ttctttcta 2400

acacgagaag tatatgaatg tcctgaagga aaccactggc ttgatatttc 2450

tgtgactcgt gttgcctttg aaactagtcc cctaccacct cggtaatgag 2500

ctccattaca gaaagtggaa cataagagaa tgaaggggca gaatatcaaa 2550

cagtgaaaag ggaatgataa gatgtatttt gaatgaactg ttttttctgt 2600

agactagctg agaaattgtt gacataaaat aaagaattga agaaacacat 2650

tttaccattt tgtgaattgt tctgaactta aatgtccact aaaacaactt 2700

agacttctgt ttgctaaatc tgtttctttt tctaatattc taaaaaaaaa 2750

aaaaaggttt acctccacaa attgaaaaaa aaaaaaaaaa aaaaaaaaaa 2800

aaaaaaaaaa aaaaaaaaaa aa 2822

<210> 306
<211> 690
<212> PRT
<213> Homo Sapien

<400> 306
Met Lys Arg Leu Pro Leu Leu Val Val Phe Ser Thr Leu Leu Asn
1 5 10 15

Cys Ser Tyr Thr Gln Asn Cys Thr Lys Thr Pro Cys Leu Pro Asn
20 25 30

Ala Lys Cys Glu Ile Arg Asn Gly Ile Glu Ala Cys Tyr Cys Asn
35 40 45

Met Gly Phe Ser Gly Asn Gly Val Thr Ile Cys Glu Asp Asp Asn
50 55 60

Glu Cys Gly Asn Leu Thr Gln Ser Cys Gly Glu Asn Ala Asn Cys
65 70 75

Thr Asn Thr Glu Gly Ser Tyr Tyr Cys Met Cys Val Pro Gly Phe
80 85 90

Arg Ser Ser Ser Asn Gln Asp Arg Phe Ile Thr Asn Asp Gly Thr

95 100 105

Val Cys Ile Glu Asn Val Asn Ala Asn Cys His Leu Asp Asn Val
110 115 120

Cys Ile Ala Ala Asn Ile Asn Lys Thr Leu Thr Lys Ile Arg Ser
125 130 135

Ile Lys Glu Pro Val Ala Leu Leu Gln Glu Val Tyr Arg Asn Ser
140 145 150

Val Thr Asp Leu Ser Pro Thr Asp Ile Ile Thr Tyr Ile Glu Ile
155 160 165

Leu Ala Glu Ser Ser Ser Leu Leu Gly Tyr Lys Asn Asn Thr Ile
170 175 180

Ser Ala Lys Asp Thr Leu Ser Asn Ser Thr Leu Thr Glu Phe Val
185 190 195

Lys Thr Val Asn Asn Phe Val Gln Arg Asp Thr Phe Val Val Trp
200 205 210

Asp Lys Leu Ser Val Asn His Arg Arg Thr His Leu Thr Lys Leu
215 220 225

Met His Thr Val Glu Gln Ala Thr Leu Arg Ile Ser Gln Ser Phe
230 235 240

Gln Lys Thr Thr Glu Phe Asp Thr Asn Ser Thr Asp Ile Ala Leu
245 250 255

Lys Val Phe Phe Phe Asp Ser Tyr Asn Met Lys His Ile His Pro
260 265 270

His Met Asn Met Asp Gly Asp Tyr Ile Asn Ile Phe Pro Lys Arg
275 280 285

Lys Ala Ala Tyr Asp Ser Asn Gly Asn Val Ala Val Ala Phe Leu
290 295 300

Tyr Tyr Lys Ser Ile Gly Pro Leu Leu Ser Ser Ser Asp Asn Phe
305 310 315

Leu Leu Lys Pro Gln Asn Tyr Asp Asn Ser Glu Glu Glu Glu Arg
320 325 330

Val Ile Ser Ser Val Ile Ser Val Ser Met Ser Ser Asn Pro Pro
335 340 345

Thr Leu Tyr Glu Leu Glu Lys Ile Thr Phe Thr Leu Ser His Arg
350 355 360

Lys Val Thr Asp Arg Tyr Arg Ser Leu Cys Ala Phe Trp Asn Tyr
365 370 375

Ser Pro Asp Thr Met Asn Gly Ser Trp Ser Ser Glu Gly Cys Glu
380 385 390

Leu Thr Tyr Ser Asn Glu Thr His Thr Ser Cys Arg Cys Asn His
395 400 405

Leu Thr His Phe Ala Ile Leu Met Ser Ser Gly Pro Ser Ile Gly
410 415 420

Ile Lys Asp Tyr Asn Ile Leu Thr Arg Ile Thr Gln Leu Gly Ile
425 430 435

Ile Ile Ser Leu Ile Cys Leu Ala Ile Cys Ile Phe Thr Phe Trp
440 445 450

Phe Phe Ser Glu Ile Gln Ser Thr Arg Thr Thr Ile His Lys Asn
455 460 465

Leu Cys Cys Ser Leu Phe Leu Ala Glu Leu Val Phe Leu Val Gly
470 475 480

Ile Asn Thr Asn Thr Asn Lys Leu Phe Cys Ser Ile Ile Ala Gly
485 490 495

Leu Leu His Tyr Phe Phe Leu Ala Ala Phe Ala Trp Met Cys Ile
500 505 510

Glu Gly Ile His Leu Tyr Leu Ile Val Val Gly Val Ile Tyr Asn
515 520 525

Lys Gly Phe Leu His Lys Asn Phe Tyr Ile Phe Gly Tyr Leu Ser
530 535 540

Pro Ala Val Val Val Gly Phe Ser Ala Ala Leu Gly Tyr Arg Tyr
545 550 555

Tyr Gly Thr Thr Lys Val Cys Trp Leu Ser Thr Glu Asn Asn Phe
560 565 570

Ile Trp Ser Phe Ile Gly Pro Ala Cys Leu Ile Ile Leu Val Asn
575 580 585

Leu Leu Ala Phe Gly Val Ile Ile Tyr Lys Val Phe Arg His Thr
590 595 600

Ala Gly Leu Lys Pro Glu Val Ser Cys Phe Glu Asn Ile Arg Ser
605 610 615

Cys Ala Arg Gly Ala Leu Ala Leu Leu Phe Leu Leu Gly Thr Thr
620 625 630

Trp Ile Phe Gly Val Leu His Val Val His Ala Ser Val Val Thr
635 640 645

Ala Tyr Leu Phe Thr Val Ser Asn Ala Phe Gln Gly Met Phe Ile
650 655 660

Phe Leu Phe Leu Cys Val Leu Ser Arg Lys Ile Gln Glu Glu Tyr
665 670 675

Tyr Arg Leu Phe Lys Asn Val Pro Cys Cys Phe Gly Cys Leu Arg
680 685 690

<210> 307
<211> 2033
<212> DNA
<213> Homo Sapien

<400> 307
ccaggccggg aggcgacgcg cccagccgtc taaacgggaa cagccctggc 50

tgagggagct gcagcgcagc agagtatctg acggcgccag gttgcgtagg 100

tgcggcacga ggagttttcc cggcagcgag gaggtcctga gcagcatggc 150
ccggaggagc gccttccctg ccgccgcgct ctggctctgg agcatcctcc 200
tgtgcctgct ggcactgcgg gcggaggccg ggccgccgca ggaggagagc 250
ctgtacctat ggatcgatgc tcaccaggca agagtactca taggatttga 300
agaagatatc ctgattgttt cagaggggaa aatggcacct tttacacatg 350
atttcagaaa agcgcaacag agaatgccag ctattcctgt caatatccat 400
tccatgaatt ttacctggca agctgcaggg caggcagaat acttctatga 450
attcctgtcc ttgcgctccc tggataaagg catcatggca gatccaaccg 500
tcaatgtccc tctgctggga acagtgcctc acaaggcatc agttgttcaa 550
gttggtttcc catgtcttgg aaaacaggat ggggtggcag catttgaagt 600
ggatgtgatt gttatgaatt ctgaaggcaa caccattctc caaacacctc 650
aaaatgctat cttctttaaa acatgtcaac aagctgagtg cccaggcggg 700
tgccgaaatg gaggcttttg taatgaaaga cgcatctgcg agtgtcctga 750
tgggttccac ggacctcact gtgagaaagc cctttgtacc ccacgatgta 800
tgaatggtgg actttgtgtg actcctggtt tctgcatctg cccacctgga 850
ttctatggag tgaactgtga caaagcaaac tgctcaacca cctgctttaa 900
tggagggacc tgtttctacc ctggaaaatg tatttgccct ccaggactag 950
agggagagca gtgtgaaatc agcaaatgcc cacaaccctg tcgaaatgga 1000
ggtaaatgca ttggtaaaag caaatgtaag tgttccaaag gttaccaggg 1050
agacctctgt tcaaagcctg tctgcgagcc tggctgtggt gcacatggaa 1100
cctgccatga acccaacaaa tgccaatgtc aagaaggttg gcatggaaga 1150
cactgcaata aaaggtacga agccagcctc atacatgccc tgaggccagc 1200
aggcgcccag ctcaggcagc acacgccttc acttaaaaag gccgaggagc 1250
ggcgggatcc acctgaatcc aattacatct ggtgaactcc gacatctgaa 1300
acgttttaag ttacaccaag ttcatagcct ttgttaacct ttcatgtgtt 1350
gaatgttcaa ataatgttca ttacacttaa gaatactggc ctgaatttta 1400
ttagcttcat tataaatcac tgagctgata tttactcttc cttttaagtt 1450
ttctaagtac gtctgtagca tgatggtata gattttcttg tttcagtgct 1500
ttgggacaga ttttatatta tgtcaattga tcaggttaaa attttcagtg 1550
tgtagttggc agatattttc aaaattacaa tgcatttatg gtgtctgggg 1600
gcaggggaac atcagaaagg ttaaattggg caaaaatgcg taagtcacaa 1650
gaatttggat ggtgcagtta atgttgaagt tacagcattt cagattttat 1700

tgtcagatat ttagatgttt gttacatttt taaaaattgc tcttaatttt 1750

taaactctca atacaatata ttttgacctt accattattc cagagattca 1800

gtattaaaaa aaaaaaaatt acactgtggt agtggcattt aaacaatata 1850

atatattcta aacacaatga aatagggaat ataatgtatg aactttttgc 1900

attggcttga agcaatataa tatattgtaa acaaaacaca gctcttacct 1950

aataaacatt ttatactgtt tgtatgtata aaataaaggt gctgctttag 2000

tttttggaa aaaaaaaaaa aaaaaaaaaa aaa 2033

<210> 308
<211> 379
<212> PRT
<213> Homo Sapien

<400> 308

```
Met Ala Arg Arg Ser Ala Phe Pro Ala Ala Ala Leu Trp Leu Trp
 1               5                  10                  15

Ser Ile Leu Leu Cys Leu Leu Ala Leu Arg Ala Glu Ala Gly Pro
                20                  25                  30

Pro Gln Glu Glu Ser Leu Tyr Leu Trp Ile Asp Ala His Gln Ala
                35                  40                  45

Arg Val Leu Ile Gly Phe Glu Glu Asp Ile Leu Ile Val Ser Glu
                50                  55                  60

Gly Lys Met Ala Pro Phe Thr His Asp Phe Arg Lys Ala Gln Gln
                65                  70                  75

Arg Met Pro Ala Ile Pro Val Asn Ile His Ser Met Asn Phe Thr
                80                  85                  90

Trp Gln Ala Ala Gly Gln Ala Glu Tyr Phe Tyr Glu Phe Leu Ser
                95                 100                 105

Leu Arg Ser Leu Asp Lys Gly Ile Met Ala Asp Pro Thr Val Asn
               110                 115                 120

Val Pro Leu Leu Gly Thr Val Pro His Lys Ala Ser Val Val Gln
               125                 130                 135

Val Gly Phe Pro Cys Leu Gly Lys Gln Asp Gly Val Ala Ala Phe
               140                 145                 150

Glu Val Asp Val Ile Val Met Asn Ser Glu Gly Asn Thr Ile Leu
               155                 160                 165

Gln Thr Pro Gln Asn Ala Ile Phe Phe Lys Thr Cys Gln Gln Ala
               170                 175                 180

Glu Cys Pro Gly Gly Cys Arg Asn Gly Gly Phe Cys Asn Glu Arg
               185                 190                 195

Arg Ile Cys Glu Cys Pro Asp Gly Phe His Gly Pro His Cys Glu
               200                 205                 210

Lys Ala Leu Cys Thr Pro Arg Cys Met Asn Gly Gly Leu Cys Val
```

215 220 225

Thr Pro Gly Phe Cys Ile Cys Pro Pro Gly Phe Tyr Gly Val Asn
230 235 240

Cys Asp Lys Ala Asn Cys Ser Thr Thr Cys Phe Asn Gly Gly Thr
245 250 255

Cys Phe Tyr Pro Gly Lys Cys Ile Cys Pro Pro Gly Leu Glu Gly
260 265 270

Glu Gln Cys Glu Ile Ser Lys Cys Pro Gln Pro Cys Arg Asn Gly
275 280 285

Gly Lys Cys Ile Gly Lys Ser Lys Cys Lys Cys Ser Lys Gly Tyr
290 295 300

Gln Gly Asp Leu Cys Ser Lys Pro Val Cys Glu Pro Gly Cys Gly
305 310 315

Ala His Gly Thr Cys His Glu Pro Asn Lys Cys Gln Cys Gln Glu
320 325 330

Gly Trp His Gly Arg His Cys Asn Lys Arg Tyr Glu Ala Ser Leu
335 340 345

Ile His Ala Leu Arg Pro Ala Gly Ala Gln Leu Arg Gln His Thr
350 355 360

Pro Ser Leu Lys Lys Ala Glu Glu Arg Arg Asp Pro Pro Glu Ser
365 370 375

Asn Tyr Ile Trp

<210> 309
<211> 1843
<212> DNA
<213> Homo Sapien

<220>
<221> unsure
<222> 1837
<223> unknown base

<400> 309
cccacgcgtc cggtctcgct cgctcgcgca gcggcggcag cagaggtcgc 50

gcacagatgc gggttagact ggcgggggga ggaggcggag gagggaagga 100

agctgcatgc atgagaccca cagactcttg caagctggat gccctctgtg 150

gatgaaagat gtatcatgga atgaacccga gcaatggaga tggatttcta 200

gagcagcagc agcagcagca gcaacctcag tccccccaga gactcttggc 250

cgtgatcctg tggtttcagc tggcgctgtg cttcggccct gcacagctca 300

cgggcgggtt cgatgacctt caagtgtgtg ctgaccccgg cattcccgag 350

aatggcttca ggacccccag cggaggggtt ttctttgaag gctctgtagc 400

ccgatttcac tgccaagacg gattcaagct gaagggcgct acaaagagac 450

tgtgtttgaa gcattttaat ggaaccctag gctggatccc aagtgataat 500
tccatctgtg tgcaagaaga ttgccgtatc cctcaaatcg aagatgctga 550
gattcataac aagacatata gacatggaga gaagctaatc atcacttgtc 600
atgaaggatt caagatccgg taccccgacc tacacaatat ggtttcatta 650
tgtcgcgatg atggaacgtg gaataatctg cccatctgtc aaggctgcct 700
gagacctcta gcctcttcta atggctatgt aaacatctct gagctccaga 750
cctccttccc ggtggggact gtgatctcct atcgctgctt tcccggattt 800
aaacttgatg ggtctgcgta tcttgagtgc ttacaaaacc ttatctggtc 850
gtccagccca ccccggtgcc ttgctctgga agcccaagtc tgtccactac 900
ctccaatggt gagtcacgga gatttcgtct gccacccgcg gccttgtgag 950
cgctacaacc acggaactgt ggtggagttt tactgcgatc ctggctacag 1000
cctcaccagc gactacaagt acatcacctg ccagtatgga gagtggtttc 1050
cttcttatca agtctactgc atcaaatcag agcaaacgtg gcccagcacc 1100
catgagaccc tcctgaccac gtggaagatt gtggcgttca cggcaaccag 1150
tgtgctgctg gtgctgctgc tcgtcatcct ggccaggatg ttccagacca 1200
agttcaaggc ccactttccc cccagggggc ctccccggag ttccagcagt 1250
gaccctgact ttgtggtggt agacggcgtg cccgtcatgc tcccgtccta 1300
tgacgaagct gtgagtggcg gcttgagtgc cttaggcccc gggtacatgg 1350
cctctgtggg ccagggctgc cccttacccg tggacgacca gagcccccca 1400
gcataccccg gctcagggga cacggacaca ggcccagggg agtcagaaac 1450
ctgtgacagc gtctcaggct cttctgagct gctccaaagt ctgtattcac 1500
ctcccaggtg ccaagagagc acccaccctg cttcggacaa ccctgacata 1550
attgccagca cggcagagga ggtggcatcc accagcccag gcatccatca 1600
tgcccactgg gtgttgttcc taagaaactg attgattaaa aaatttccca 1650
aagtgtcctg aagtgtctct tcaaatacat gttgatctgt ggagttgatt 1700
cctttccttc tcttggtttt agacaaatgt aaacaaagct ctgatcctta 1750
aaattgctat gctgatagag tggtgagggc tggaagcttg atcaagtcct 1800
gtttcttctt gacacagact gattaaaaat taaaagnaaa aaa 1843

<210> 310
<211> 490
<212> PRT
<213> Homo Sapien

<400> 310
Met Tyr His Gly Met Asn Pro Ser Asn Gly Asp Gly Phe Leu Glu
1 5 10 15

```
Gln Gln Gln Gln Gln Gln Gln Pro Gln Ser Pro Gln Arg Leu Leu
                20                  25                  30

Ala Val Ile Leu Trp Phe Gln Leu Ala Leu Cys Phe Gly Pro Ala
                35                  40                  45

Gln Leu Thr Gly Gly Phe Asp Asp Leu Gln Val Cys Ala Asp Pro
                50                  55                  60

Gly Ile Pro Glu Asn Gly Phe Arg Thr Pro Ser Gly Gly Val Phe
                65                  70                  75

Phe Glu Gly Ser Val Ala Arg Phe His Cys Gln Asp Gly Phe Lys
                80                  85                  90

Leu Lys Gly Ala Thr Lys Arg Leu Cys Leu Lys His Phe Asn Gly
                95                  100                 105

Thr Leu Gly Trp Ile Pro Ser Asp Asn Ser Ile Cys Val Gln Glu
                110                 115                 120

Asp Cys Arg Ile Pro Gln Ile Glu Asp Ala Glu Ile His Asn Lys
                125                 130                 135

Thr Tyr Arg His Gly Glu Lys Leu Ile Ile Thr Cys His Glu Gly
                140                 145                 150

Phe Lys Ile Arg Tyr Pro Asp Leu His Asn Met Val Ser Leu Cys
                155                 160                 165

Arg Asp Asp Gly Thr Trp Asn Asn Leu Pro Ile Cys Gln Gly Cys
                170                 175                 180

Leu Arg Pro Leu Ala Ser Ser Asn Gly Tyr Val Asn Ile Ser Glu
                185                 190                 195

Leu Gln Thr Ser Phe Pro Val Gly Thr Val Ile Ser Tyr Arg Cys
                200                 205                 210

Phe Pro Gly Phe Lys Leu Asp Gly Ser Ala Tyr Leu Glu Cys Leu
                215                 220                 225

Gln Asn Leu Ile Trp Ser Ser Ser Pro Pro Arg Cys Leu Ala Leu
                230                 235                 240

Glu Ala Gln Val Cys Pro Leu Pro Pro Met Val Ser His Gly Asp
                245                 250                 255

Phe Val Cys His Pro Arg Pro Cys Glu Arg Tyr Asn His Gly Thr
                260                 265                 270

Val Val Glu Phe Tyr Cys Asp Pro Gly Tyr Ser Leu Thr Ser Asp
                275                 280                 285

Tyr Lys Tyr Ile Thr Cys Gln Tyr Gly Glu Trp Phe Pro Ser Tyr
                290                 295                 300

Gln Val Tyr Cys Ile Lys Ser Glu Gln Thr Trp Pro Ser Thr His
                305                 310                 315

Glu Thr Leu Leu Thr Thr Trp Lys Ile Val Ala Phe Thr Ala Thr
                320                 325                 330
```

Ser Val Leu Leu Val Leu Leu Leu Val Ile Leu Ala Arg Met Phe
335 340 345

Gln Thr Lys Phe Lys Ala His Phe Pro Pro Arg Gly Pro Pro Arg
350 355 360

Ser Ser Ser Ser Asp Pro Asp Phe Val Val Val Asp Gly Val Pro
365 370 375

Val Met Leu Pro Ser Tyr Asp Glu Ala Val Ser Gly Gly Leu Ser
380 385 390

Ala Leu Gly Pro Gly Tyr Met Ala Ser Val Gly Gln Gly Cys Pro
395 400 405

Leu Pro Val Asp Asp Gln Ser Pro Pro Ala Tyr Pro Gly Ser Gly
410 415 420

Asp Thr Asp Thr Gly Pro Gly Glu Ser Glu Thr Cys Asp Ser Val
425 430 435

Ser Gly Ser Ser Glu Leu Leu Gln Ser Leu Tyr Ser Pro Pro Arg
440 445 450

Cys Gln Glu Ser Thr His Pro Ala Ser Asp Asn Pro Asp Ile Ile
455 460 465

Ala Ser Thr Ala Glu Glu Val Ala Ser Thr Ser Pro Gly Ile His
470 475 480

His Ala His Trp Val Leu Phe Leu Arg Asn
485 490

<210> 311
<211> 1210
<212> DNA
<213> Homo Sapien

<400> 311
cagcgcgtgg ccggcgccgc tgtggggaca gcatgagcgg cggttggatg 50

gcgcaggttg gagcgtggcg aacaggggct ctgggcctgg cgctgctgct 100

gctgctcggc ctcggactag gcctggaggc cgccgcgagc ccgctttcca 150

ccccgacctc tgcccaggcc gcaggcccca gctcaggctc gtgcccaccc 200

accaagttcc agtgccgcac cagtggctta tgcgtgcccc tcacctggcg 250

ctgcgacagg gacttggact gcagcgatgg cagcgatgag gaggagtgca 300

ggattgagcc atgtacccag aaagggcaat gcccaccgcc ccctggcctc 350

ccctgcccct gcaccggcgt cagtgactgc tctgggggaa ctgacaagaa 400

actgcgcaac tgcagccgcc tggcctgcct agcaggcgag ctccgttgca 450

cgctgagcga tgactgcatt ccactcacgt ggcgctgcga cggccaccca 500

gactgtcccg actccagcga cgagctcggc tgtggaacca atgagatcct 550

cccggaaggg gatgccacaa ccatggggcc ccctgtgacc ctggagagtg 600

tcacctctct caggaatgcc acaaccatgg ggccccctgt gaccctggag 650

agtgtcccct ctgtcgggaa tgccacatcc tcctctgccg gagaccagtc 700

tggaagccca actgcctatg ggttattgc agctgctgcg gtgctcagtg 750

caagcctggt caccgccacc ctcctccttt tgtcctggct ccgagcccag 800

gagcgcctcc gcccactggg gttactggtg gccatgaagg agtccctgct 850

gctgtcagaa cagaagacct cgctgccctg aggacaagca cttgccacca 900

ccgtcactca gccctgggcg tagccggaca ggaggagagc agtgatgcgg 950

atgggtaccc gggcacacca gccctcagag acctgagttc ttctggccac 1000

gtggaacctc gaacccgagc tcctgcagaa gtggccctgg agattgaggg 1050

tccctggaca ctccctatgg agatccgggg agctaggatg gggaacctgc 1100

cacagccaga actgaggggc tggccccagg cagctcccag ggggtagaac 1150

ggccctgtgc ttaagacact ccctgctgcc ccgtctgagg gtggcgatta 1200

aagttgcttc 1210

<210> 312
<211> 282
<212> PRT
<213> Homo Sapien

<400> 312
Met Ser Gly Gly Trp Met Ala Gln Val Gly Ala Trp Arg Thr Gly
1 5 10 15

Ala Leu Gly Leu Ala Leu Leu Leu Leu Leu Gly Leu Gly Leu Gly
20 25 30

Leu Glu Ala Ala Ala Ser Pro Leu Ser Thr Pro Thr Ser Ala Gln
35 40 45

Ala Ala Gly Pro Ser Ser Gly Ser Cys Pro Pro Thr Lys Phe Gln
50 55 60

Cys Arg Thr Ser Gly Leu Cys Val Pro Leu Thr Trp Arg Cys Asp
65 70 75

Arg Asp Leu Asp Cys Ser Asp Gly Ser Asp Glu Glu Glu Cys Arg
80 85 90

Ile Glu Pro Cys Thr Gln Lys Gly Gln Cys Pro Pro Pro Pro Gly
95 100 105

Leu Pro Cys Pro Cys Thr Gly Val Ser Asp Cys Ser Gly Gly Thr
110 115 120

Asp Lys Lys Leu Arg Asn Cys Ser Arg Leu Ala Cys Leu Ala Gly
125 130 135

Glu Leu Arg Cys Thr Leu Ser Asp Asp Cys Ile Pro Leu Thr Trp
140 145 150

Arg Cys Asp Gly His Pro Asp Cys Pro Asp Ser Ser Asp Glu Leu
155 160 165

Gly Cys Gly Thr Asn Glu Ile Leu Pro Glu Gly Asp Ala Thr Thr
170 175 180

Met Gly Pro Pro Val Thr Leu Glu Ser Val Thr Ser Leu Arg Asn
185 190 195

Ala Thr Thr Met Gly Pro Pro Val Thr Leu Glu Ser Val Pro Ser
200 205 210

Val Gly Asn Ala Thr Ser Ser Ser Ala Gly Asp Gln Ser Gly Ser
215 220 225

Pro Thr Ala Tyr Gly Val Ile Ala Ala Ala Ala Val Leu Ser Ala
230 235 240

Ser Leu Val Thr Ala Thr Leu Leu Leu Leu Ser Trp Leu Arg Ala
245 250 255

Gln Glu Arg Leu Arg Pro Leu Gly Leu Leu Val Ala Met Lys Glu
260 265 270

Ser Leu Leu Leu Ser Glu Gln Lys Thr Ser Leu Pro
275 280

<210> 313
<211> 2197
<212> DNA
<213> Homo Sapien

<400> 313
cggacgcgtg ggcgtccggc ggtcgcagag ccaggaggcg gaggcgcgcg 50

ggccagcctg ggccccagcc cacaccttca ccagggccca ggagccacca 100

tgtggcgatg tccactgggg ctactgctgt tgctgccgct ggctggccac 150

ttggctctgg gtgcccagca gggtcgtggg cgccgggagc tagcaccggg 200

tctgcacctg cggggcatcc gggacgcggg aggccggtac tgccaggagc 250

aggacctgtg ctgccgcggc cgtgccgacg actgtgccct gccctacctg 300

ggcgccatct gttactgtga cctcttctgc aaccgcacgg tctccgactg 350

ctgccctgac ttctgggact tctgcctcgg cgtgccaccc ccttttcccc 400

cgatccaagg atgtatgcat ggaggtcgta tctatccagt cttgggaacg 450

tactgggaca actgtaaccg ttgcacctgc caggagaaca ggcagtggca 500

tggtggatcc agacatgatc aaagccatca accagggcaa ctatggctgg 550

caggctggga accacagcgc cttctggggc atgaccctgg atgagggcat 600

tcgctaccgc ctgggcacca tccgcccatc ttcctcggtc atgaacatgc 650

atgaaattta tacagtgctg aacccagggg aggtgcttcc cacagccttc 700

gaggcctctg agaagtggcc caacctgatt catgagcctc ttgaccaagg 750

caactgtgca ggctcctggg ccttctccac agcagctgtg gcatccgatc 800

gtgtctcaat ccattctctg ggacacatga cgcctgtcct gtcgccccag 850

aacctgctgt cttgtgacac ccaccagcag cagggctgcc gcggtgggcg 900

tctcgatggt gcctggtggt tcctgcgtcg ccgaggggtg gtgtctgacc 950

actgctaccc cttctcgggc cgtgaacgag acgaggctgg ccctgcgccc 1000

ccctgtatga tgcacagccg agccatgggt cggggcaagc gccaggccac 1050

tgcccactgc cccaacagct atgttaataa caatgacatc taccaggtca 1100

ctcctgtcta ccgcctcggc tccaacgaca aggagatcat gaaggagctg 1150

atggagaatg gccctgtcca agccctcatg gaggtgcatg aggacttctt 1200

cctatacaag ggaggcatct acagccacac gccagtgagc cttgggaggc 1250

cagagagata ccgccggcat gggacccact cagtcaagat cacaggatgg 1300

ggagaggaga cgctgccaga tggaaggacg ctcaaatact ggactgcggc 1350

caactcctgg ggcccagcct ggggcgagag gggccacttc cgcatcgtgc 1400

gcggcgtcaa tgagtgcgac atcgagagct tcgtgctggg cgtctggggc 1450

cgcgtgggca tggaggacat gggtcatcac tgaggctgcg ggcaccacgc 1500

ggggtccggc ctgggatcca ggctaagggc cggcggaaga ggccccaatg 1550

gggcggtgac cccagcctcg cccgacagag cccggggcgc aggcgggcgc 1600

cagggcgcta atcccggcgc gggttccgct gacgcagcgc cccgcctggg 1650

agccgcgggc aggcgagact ggcggagccc ccagacctcc cagtggggac 1700

ggggcagggc ctggcctggg aagagcacag ctgcagatcc caggcctctg 1750

gcgcccccac tcaagactac caaagccagg acacctcaag tctccagccc 1800

caatacccca ccccaatccc gtattctttt tttttttttt ttagacaggg 1850

tcttgctccg ttgcccaggt tggagtgcag tggcccatca gggctcactg 1900

taacctccga ctcctgggtt caagtgaccc tcccacctca gcctctcaag 1950

tagctgggac tacaggtgca ccaccacacc tggctaattt ttgtattttt 2000

tgtaaagagg ggggtctcac tgtgttgccc aggctggttt cgaactcctg 2050

ggctcaagcg gtccacctgc ctccgcctcc caaagtgctg ggattgcagg 2100

catgagccac tgcacccagc cctgtattct tattcttcag atatttattt 2150

ttcttttcac tgttttaaaa taaaaccaaa gtattgataa aaaaaaa 2197

<210> 314
<211> 164
<212> PRT
<213> Homo Sapien

<400> 314

```
Met Trp Arg Cys Pro Leu Gly Leu Leu Leu Leu Leu Pro Leu Ala
  1               5                  10                  15

Gly His Leu Ala Leu Gly Ala Gln Gln Gly Arg Gly Arg Arg Glu
```

20 25 30

Leu Ala Pro Gly Leu His Leu Arg Gly Ile Arg Asp Ala Gly Gly
35 40 45

Arg Tyr Cys Gln Glu Gln Asp Leu Cys Cys Arg Gly Arg Ala Asp
50 55 60

Asp Cys Ala Leu Pro Tyr Leu Gly Ala Ile Cys Tyr Cys Asp Leu
65 70 75

Phe Cys Asn Arg Thr Val Ser Asp Cys Cys Pro Asp Phe Trp Asp
80 85 90

Phe Cys Leu Gly Val Pro Pro Pro Phe Pro Pro Ile Gln Gly Cys
95 100 105

Met His Gly Gly Arg Ile Tyr Pro Val Leu Gly Thr Tyr Trp Asp
110 115 120

Asn Cys Asn Arg Cys Thr Cys Gln Glu Asn Arg Gln Trp His Gly
125 130 135

Gly Ser Arg His Asp Gln Ser His Gln Pro Gly Gln Leu Trp Leu
140 145 150

Ala Gly Trp Glu Pro Gln Arg Leu Leu Gly His Asp Pro Gly
155 160

<210> 315
<211> 1024
<212> DNA
<213> Homo Sapien

<400> 315
cggacgcgtg ggcccctggt gggcccagca agatggatct actgtggatc 50

ctgccctccc tgtggcttct cctgcttggg gggcctgcct gcctgaagac 100

ccaggaacac cccagctgcc caggacccag ggaactggaa gccagcaaag 150

ttgtcctcct gcccagttgt cccggagctc caggaagtcc tggggagaag 200

ggagccccag gtcctcaagg gccacctgga ccaccaggca agatgggccc 250

caagggtgag ccaggcccca gaaactgccg ggagctgttg agccagggcg 300

ccaccttgag cggctggtac catctgtgcc tacctgaggg cagggccctc 350

ccagtctttt gtgacatgga caccgagggg ggcggctggc tggtgtttca 400

gaggcgccag gatggttctg tggatttctt ccgctcttgg tcctcctaca 450

gagcaggttt tgggaaccaa gagtctgaat tctggctggg aaatgagaat 500

ttgcaccagc ttactctcca gggtaactgg gagctgcggg tagagctgga 550

agactttaat ggtaaccgta ctttcgccca ctatgcgacc ttccgcctcc 600

tcggtgaggt agaccactac cagctggcac tgggcaagtt ctcagagggc 650

actgcagggg attccctgag cctccacagt gggaggccct ttaccaccta 700

tgacgctgac cacgattcaa gcaacagcaa ctgtgcagtg attgtccacg 750

gtgcctggtg gtatgcatcc tgttaccgat caaatctcaa tggtcgctat 800

gcagtgtctg aggctgccgc ccacaaatat ggcattgact gggcctcagg 850

ccgtggtgtg ggccacccct accgcagggt tcggatgatg cttcgatagg 900

gcactctggc agccagtgcc cttatctctc ctgtacagct tccggatcgt 950

cagccacctt gcctttgcca accacctctg cttgcctgtc cacatttaaa 1000

aataaaatca ttttagccct ttca 1024

<210> 316
<211> 288
<212> PRT
<213> Homo Sapien

<400> 316
Met Asp Leu Leu Trp Ile Leu Pro Ser Leu Trp Leu Leu Leu Leu
1 5 10 15

Gly Gly Pro Ala Cys Leu Lys Thr Gln Glu His Pro Ser Cys Pro
20 25 30

Gly Pro Arg Glu Leu Glu Ala Ser Lys Val Val Leu Leu Pro Ser
35 40 45

Cys Pro Gly Ala Pro Gly Ser Pro Gly Glu Lys Gly Ala Pro Gly
50 55 60

Pro Gln Gly Pro Pro Gly Pro Pro Gly Lys Met Gly Pro Lys Gly
65 70 75

Glu Pro Gly Pro Arg Asn Cys Arg Glu Leu Leu Ser Gln Gly Ala
80 85 90

Thr Leu Ser Gly Trp Tyr His Leu Cys Leu Pro Glu Gly Arg Ala
95 100 105

Leu Pro Val Phe Cys Asp Met Asp Thr Glu Gly Gly Gly Trp Leu
110 115 120

Val Phe Gln Arg Arg Gln Asp Gly Ser Val Asp Phe Phe Arg Ser
125 130 135

Trp Ser Ser Tyr Arg Ala Gly Phe Gly Asn Gln Glu Ser Glu Phe
140 145 150

Trp Leu Gly Asn Glu Asn Leu His Gln Leu Thr Leu Gln Gly Asn
155 160 165

Trp Glu Leu Arg Val Glu Leu Glu Asp Phe Asn Gly Asn Arg Thr
170 175 180

Phe Ala His Tyr Ala Thr Phe Arg Leu Leu Gly Glu Val Asp His
185 190 195

Tyr Gln Leu Ala Leu Gly Lys Phe Ser Glu Gly Thr Ala Gly Asp
200 205 210

Ser Leu Ser Leu His Ser Gly Arg Pro Phe Thr Thr Tyr Asp Ala
215 220 225

Asp His Asp Ser Ser Asn Ser Asn Cys Ala Val Ile Val His Gly
230 235 240

Ala Trp Trp Tyr Ala Ser Cys Tyr Arg Ser Asn Leu Asn Gly Arg
245 250 255

Tyr Ala Val Ser Glu Ala Ala Ala His Lys Tyr Gly Ile Asp Trp
260 265 270

Ala Ser Gly Arg Gly Val Gly His Pro Tyr Arg Arg Val Arg Met
275 280 285

Met Leu Arg

<210> 317
<211> 1875
<212> DNA
<213> Homo Sapien

<400> 317
cccaagccag ccgagccgcc agagccgcgg gccgcggggg tgtcgcgggc 50

ccaaccccag gatgctcccc tgcgcctcct gcctacccgg gtctctactg 100

ctctgggcgc tgctactgtt gctcttggga tcagcttctc ctcaggattc 150

tgaagagccc gacagctaca cggaatgcac agatggctat gagtgggacc 200

cagacagcca gcactgccgg gatgtcaacg agtgtctgac catccctgag 250

gcctgcaagg gggaaatgaa gtgcatcaac cactacgggg gctacttgtg 300

cctgccccgc tccgctgccg tcatcaacga cctacatggc gagggacccc 350

cgccaccagt gcctcccgct caacacccca acccctgccc accaggctat 400

gagcccgacg atcaggacag ctgtgtggat gtggacgagt gtgcccaggc 450

cctgcacgac tgtcgcccca gccaggactg ccataacttg cctggctcct 500

atcagtgcac ctgccctgat ggttaccgca agatcgggcc cgagtgtgtg 550

gacatagacg agtgccgcta ccgctactgc cagcaccgct gcgtgaacct 600

gcctggctcc ttccgctgcc agtgcgagcc gggcttccag ctggggccta 650

acaaccgctc ctgtgttgat gtgaacgagt gtgacatggg ggccccatgc 700

gagcagcgct gcttcaactc ctatgggacc ttcctgtgtc gctgccacca 750

gggctatgag ctgcatcggg atggcttctc ctgcagtgat attgatgagt 800

gtagctactc cagctacctc tgtcagtacc gctgcgtcaa cgagccaggc 850

cgtttctcct gccactgccc acagggttac cagctgctgg ccacacgcct 900

ctgccaagac attgatgagt gtgagtctgg tgcgcaccag tgctccgagg 950

cccaaacctg tgtcaacttc catgggggct accgctgcgt ggacaccaac 1000

cgctgcgtgg agccctacat ccaggtctct gagaaccgct gtctctgccc 1050

ggcctccaac cctctatgtc gagagcagcc ttcatccatt gtgcaccgct 1100

acatgaccat cacctcggag cggagcgtgc ccgctgacgt gttccagatc 1150

caggcgacct ccgtctaccc cggtgcctac aatgcctttc agatccgtgc 1200

tggaaactcg caggggggact tttacattag gcaaatcaac aacgtcagcg 1250

ccatgctggt cctcgcccgg ccggtgacgg gcccccggga gtacgtgctg 1300

gacctggaga tggtcaccat gaattccctc atgagctacc gggccagctc 1350

tgtactgagg ctcaccgtct ttgtaggggc ctacaccttc tgaggagcag 1400

gagggagcca ccctccctgc agctacccta gctgaggagc ctgttgtgag 1450

gggcagaatg agaaaggcaa taaagggaga aagaaagtcc tggtggctga 1500

ggtgggcggg tcacactgca ggaagcctca ggctggggca gggtggcact 1550

tgggggggca ggccaagttc acctaaatgg gggtctctat atgttcaggc 1600

ccaggggccc ccattgacag gagctgggag ctctgcacca cgagcttcag 1650

tcaccccgag aggagaggag gtaacgagga gggcggactc caggccccgg 1700

cccagagatt tggacttggc tggcttgcag gggtcctaag aaactccact 1750

ctggacagcg ccaggaggcc ctgggttcca ttcctaactc tgcctcaaac 1800

tgtacatttg gataagccct agtagttccc tgggcctgtt tttctataaa 1850

acgaggcaac tggaaaaaaa aaaaa 1875

<210> 318
<211> 443
<212> PRT
<213> Homo Sapien

<400> 318
Met Leu Pro Cys Ala Ser Cys Leu Pro Gly Ser Leu Leu Leu Trp
1 5 10 15

Ala Leu Leu Leu Leu Leu Leu Gly Ser Ala Ser Pro Gln Asp Ser
20 25 30

Glu Glu Pro Asp Ser Tyr Thr Glu Cys Thr Asp Gly Tyr Glu Trp
35 40 45

Asp Pro Asp Ser Gln His Cys Arg Asp Val Asn Glu Cys Leu Thr
50 55 60

Ile Pro Glu Ala Cys Lys Gly Glu Met Lys Cys Ile Asn His Tyr
65 70 75

Gly Gly Tyr Leu Cys Leu Pro Arg Ser Ala Ala Val Ile Asn Asp
80 85 90

Leu His Gly Glu Gly Pro Pro Pro Pro Val Pro Pro Ala Gln His
95 100 105

Pro Asn Pro Cys Pro Pro Gly Tyr Glu Pro Asp Asp Gln Asp Ser
110 115 120

Cys Val Asp Val Asp Glu Cys Ala Gln Ala Leu His Asp Cys Arg

125 130 135

Pro Ser Gln Asp Cys His Asn Leu Pro Gly Ser Tyr Gln Cys Thr
140 145 150

Cys Pro Asp Gly Tyr Arg Lys Ile Gly Pro Glu Cys Val Asp Ile
155 160 165

Asp Glu Cys Arg Tyr Arg Tyr Cys Gln His Arg Cys Val Asn Leu
170 175 180

Pro Gly Ser Phe Arg Cys Gln Cys Glu Pro Gly Phe Gln Leu Gly
185 190 195

Pro Asn Asn Arg Ser Cys Val Asp Val Asn Glu Cys Asp Met Gly
200 205 210

Ala Pro Cys Glu Gln Arg Cys Phe Asn Ser Tyr Gly Thr Phe Leu
215 220 225

Cys Arg Cys His Gln Gly Tyr Glu Leu His Arg Asp Gly Phe Ser
230 235 240

Cys Ser Asp Ile Asp Glu Cys Ser Tyr Ser Ser Tyr Leu Cys Gln
245 250 255

Tyr Arg Cys Val Asn Glu Pro Gly Arg Phe Ser Cys His Cys Pro
260 265 270

Gln Gly Tyr Gln Leu Leu Ala Thr Arg Leu Cys Gln Asp Ile Asp
275 280 285

Glu Cys Glu Ser Gly Ala His Gln Cys Ser Glu Ala Gln Thr Cys
290 295 300

Val Asn Phe His Gly Gly Tyr Arg Cys Val Asp Thr Asn Arg Cys
305 310 315

Val Glu Pro Tyr Ile Gln Val Ser Glu Asn Arg Cys Leu Cys Pro
320 325 330

Ala Ser Asn Pro Leu Cys Arg Glu Gln Pro Ser Ser Ile Val His
335 340 345

Arg Tyr Met Thr Ile Thr Ser Glu Arg Ser Val Pro Ala Asp Val
350 355 360

Phe Gln Ile Gln Ala Thr Ser Val Tyr Pro Gly Ala Tyr Asn Ala
365 370 375

Phe Gln Ile Arg Ala Gly Asn Ser Gln Gly Asp Phe Tyr Ile Arg
380 385 390

Gln Ile Asn Asn Val Ser Ala Met Leu Val Leu Ala Arg Pro Val
395 400 405

Thr Gly Pro Arg Glu Tyr Val Leu Asp Leu Glu Met Val Thr Met
410 415 420

Asn Ser Leu Met Ser Tyr Arg Ala Ser Ser Val Leu Arg Leu Thr
425 430 435

Val Phe Val Gly Ala Tyr Thr Phe
440

<210> 319
<211> 1266
<212> DNA
<213> Homo Sapien

<400> 319
gctggggaca tgagaggcac accgaagacc cacctcctgg ccttctccct 50

cctctgcctc ctctcaaagg tgcgtaccca gctgtgcccg acaccatgta 100

cctgcccctg gccacctccc cgatgcccgc tgggagtacc cctggtgctg 150

gatggctgtg gctgctgccg ggtatgtgca cggcggctgg gggagccctg 200

cgaccaactc cacgtctgcg acgccagcca gggcctggtc tgccagcccg 250

gggcaggacc cggtggccgg ggggccctgt gcctcttggc agaggacgac 300

agcagctgtg aggtgaacgg ccgcctgtat cgggaagggg agaccttcca 350

gccccactgc agcatccgct gccgctgcga ggacggcggc ttcacctgcg 400

tgccgctgtg cagcgaggat gtgcggctgc ccagctggga ctgcccccac 450

cccaggaggg tcgaggtcct gggcaagtgc tgccctgagt gggtgtgcgg 500

ccaaggaggg ggactgggga cccagcccct tccagcccaa ggaccccagt 550

tttctggcct tgtctcttcc ctgccccctg gtgtccccctg cccagaatgg 600

agcacggcct ggggaccctg ctcgaccacc tgtgggctgg gcatggccac 650

ccgggtgtcc aaccagaacc gcttctgccg actggagacc cagcgccgcc 700

tgtgcctgtc caggccctgc ccaccctcca ggggtcgcag tccacaaaac 750

agtgccttct agagccgggc tgggaatggg gacacggtgt ccaccatccc 800

cagctggtgg ccctgtgcct gggccctggg ctgatggaag atggtccgtg 850

cccaggccct tggctgcagg caacacttta gcttgggtcc accatgcaga 900

acaccaatat taacacgctg cctggtctgt ctggatcccg aggtatggca 950

gaggtgcaag acctagtccc ctttcctcta actcactgcc taggaggctg 1000

gccaaggtgt ccagggtcct ctagcccact ccctgcctac acacacagcc 1050

tatatcaaac atgcacacgg gcgagctttc tctccgactt cccctgggca 1100

agagatggga caagcagtcc cttaatattg aggctgcagc aggtgctggg 1150

ctggactggc catttttctg gggtaggat gaagagaagg cacacagaga 1200

ttctggatct cctgctgcct tttctggagt ttgtaaaatt gttcctgaat 1250

acaagcctat gcgtga 1266

<210> 320
<211> 250
<212> PRT
<213> Homo Sapien

<400> 320

```
Met Arg Gly Thr Pro Lys Thr His Leu Leu Ala Phe Ser Leu Leu
  1               5                  10                  15

Cys Leu Leu Ser Lys Val Arg Thr Gln Leu Cys Pro Thr Pro Cys
                 20                  25                  30

Thr Cys Pro Trp Pro Pro Pro Arg Cys Pro Leu Gly Val Pro Leu
                 35                  40                  45

Val Leu Asp Gly Cys Gly Cys Cys Arg Val Cys Ala Arg Arg Leu
                 50                  55                  60

Gly Glu Pro Cys Asp Gln Leu His Val Cys Asp Ala Ser Gln Gly
                 65                  70                  75

Leu Val Cys Gln Pro Gly Ala Gly Pro Gly Gly Arg Gly Ala Leu
                 80                  85                  90

Cys Leu Leu Ala Glu Asp Asp Ser Ser Cys Glu Val Asn Gly Arg
                 95                 100                 105

Leu Tyr Arg Glu Gly Glu Thr Phe Gln Pro His Cys Ser Ile Arg
                110                 115                 120

Cys Arg Cys Glu Asp Gly Gly Phe Thr Cys Val Pro Leu Cys Ser
                125                 130                 135

Glu Asp Val Arg Leu Pro Ser Trp Asp Cys Pro His Pro Arg Arg
                140                 145                 150

Val Glu Val Leu Gly Lys Cys Cys Pro Glu Trp Val Cys Gly Gln
                155                 160                 165

Gly Gly Gly Leu Gly Thr Gln Pro Leu Pro Ala Gln Gly Pro Gln
                170                 175                 180

Phe Ser Gly Leu Val Ser Ser Leu Pro Pro Gly Val Pro Cys Pro
                185                 190                 195

Glu Trp Ser Thr Ala Trp Gly Pro Cys Ser Thr Thr Cys Gly Leu
                200                 205                 210

Gly Met Ala Thr Arg Val Ser Asn Gln Asn Arg Phe Cys Arg Leu
                215                 220                 225

Glu Thr Gln Arg Arg Leu Cys Leu Ser Arg Pro Cys Pro Pro Ser
                230                 235                 240

Arg Gly Arg Ser Pro Gln Asn Ser Ala Phe
                245                 250
```

<210> 321
<211> 783
<212> DNA
<213> Homo Sapien

<400> 321

```
agaacctcag aaatgtgagt tatttgggaa tggctgtttg taaatgtcct 50

tacgtaagcc aagaggaggt cttgacttgg ggtcccaggg gtaccgcaga 100

tcccagggac tggagcagca ctagcaagct ctggaggatg agccaggagt 150
```

ctggaattga ggctgagcca aagaccccag ggccgtctca gtctcataaa 200

aggggatcag gcaggaggag tttgggagaa acctgagaag ggcctgattt 250

gcagcatcat gatgggcctc tccttggcct ctgctgtgct cctggcctcc 300

ctcctgagtc tccaccttgg aactgccaca cgtgggagtg acatatccaa 350

gacctgctgc ttccaataca gccacaagcc ccttccctgg acctgggtgc 400

gaagctatga attcaccagt aacagctgct cccagcgggc tgtgatattc 450

actaccaaaa gaggcaagaa agtctgtacc catccaagga aaaaatgggt 500

gcaaaaatac atttctttac tgaaaactcc gaaacaattg tgactcagct 550

gaattttcat ccgaggacgc ttggaccccg ctcttggctc tgcagccctc 600

tggggagcct gcggaatctt ttctgaaggc tacatggacc cgctggggag 650

gagagggtgt ttcctcccag agttacttta ataaaggttg ttcatagagt 700

tgaaaaaaaa aaaaaaaaaa aaaaaaaaaa aaaaaaaaaa aaaaaaaaaa 750

aaaaaaaaaa aaaaaaaaaa aaaaaaaaaa aaa 783

<210> 322
<211> 94
<212> PRT
<213> Homo Sapien

<400> 322
Met Met Gly Leu Ser Leu Ala Ser Ala Val Leu Leu Ala Ser Leu
1               5                   10                  15

Leu Ser Leu His Leu Gly Thr Ala Thr Arg Gly Ser Asp Ile Ser
                20                  25                  30

Lys Thr Cys Cys Phe Gln Tyr Ser His Lys Pro Leu Pro Trp Thr
                35                  40                  45

Trp Val Arg Ser Tyr Glu Phe Thr Ser Asn Ser Cys Ser Gln Arg
                50                  55                  60

Ala Val Ile Phe Thr Thr Lys Arg Gly Lys Lys Val Cys Thr His
                65                  70                  75

Pro Arg Lys Lys Trp Val Gln Lys Tyr Ile Ser Leu Leu Lys Thr
                80                  85                  90

Pro Lys Gln Leu

<210> 323
<211> 2290
<212> DNA
<213> Homo Sapien

<400> 323
accgagccga gcggaccgaa ggcgcgcccg agatgcaggt gagcaagagg 50

atgctggcgg ggggcgtgag gagcatgccc agccccctcc tggcctgctg 100

gcagcccatc ctcctgctgg tgctgggctc agtgctgtca ggctcggcca 150

cgggctgccc gccccgctgc gagtgctccg cccaggaccg cgctgtgctg 200
tgccaccgca agtgctttgt ggcagtcccc gagggcatcc ccaccgagac 250
gcgcctgctg gacctaggca agaaccgcat caaaacgctc aaccaggacg 300
agttcgccag cttcccgcac ctggaggagc tggagctcaa cgagaacatc 350
gtgagcgccg tggagcccgg cgccttcaac aacctcttca acctccggac 400
gctgggtctc cgcagcaacc gcctgaagct catcccgcta ggcgtcttca 450
ctggcctcag caacctgacc aagcaggaca tcagcgagaa caagatcgtt 500
atcctactgg actacatgtt tcaggacctg tacaacctca agtcactgga 550
ggttggcgac aatgacctcg tctacatctc tcaccgcgcc ttcagcggcc 600
tcaacagcct ggagcagctg acgctggaga aatgcaacct gacctccatc 650
cccaccgagg cgctgtccca cctgcacggc ctcatcgtcc tgaggctccg 700
gcacctcaac atcaatgcca tccgggacta ctccttcaag aggctgtacc 750
gactcaaggt cttggagatc tcccactggc cctacttgga caccatgaca 800
cccaactgcc tctacggcct caacctgacg tccctgtcca tcacacactg 850
caatctgacc gctgtgccct acctggccgt ccgccaccta gtctatctcc 900
gcttcctcaa cctctcctac aaccccatca gcaccattga gggctccatg 950
ttgcatgagc tgctccggct gcaggagatc cagctggtgg gcgggcagct 1000
ggccgtggtg gagccctatg ccttccgcgg cctcaactac ctgcgcgtgc 1050
tcaatgtctc tggcaaccag ctgaccacac tggaggaatc agtcttccac 1100
tcggtgggca acctggagac actcatcctg gactccaacc cgctggcctg 1150
cgactgtcgg ctcctgtggg tgttccggcg ccgctggcgg ctcaacttca 1200
accggcagca gcccacgtgc gccacgcccg agtttgtcca gggcaaggag 1250
ttcaaggact tccctgatgt gctactgccc aactacttca cctgccgccg 1300
cgcccgcatc cgggaccgca aggcccagca ggtgtttgtg gacgagggcc 1350
acacggtgca gtttgtgtgc cgggccgatg gcgacccgcc gcccgccatc 1400
ctctggctct caccccgaaa gcacctggtc tcagccaaga gcaatgggcg 1450
gctcacagtc ttccctgatg gcacgctgga ggtgcgctac gcccaggtac 1500
aggacaacgg cacgtacctg tgcatcgcgg ccaacgcggg cggcaacgac 1550
tccatgcccg cccacctgca tgtgcgcagc tactcgcccg actggcccca 1600
tcagcccaac aagaccttcg ctttcatctc caaccagccg ggcgagggag 1650
aggccaacag cacccgcgcc actgtgcctt tccccttcga catcaagacc 1700
ctcatcatcg ccaccaccat gggcttcatc tctttcctgg gcgtcgtcct 1750

cttctgcctg gtgctgctgt ttctctggag ccggggcaag ggcaacacaa 1800

agcacaacat cgagatcgag tatgtgcccc gaaagtcgga cgcaggcatc 1850

agctccgccg acgcgccccg caagttcaac atgaagatga tatgaggccg 1900

gggcgggggg cagggacccc cgggcggccg ggcaggggaa ggggcctggt 1950

cgccacctgc tcactctcca gtccttccca cctcctccct acccttctac 2000

acacgttctc tttctccctc ccgcctccgt cccctgctgc cccccgccag 2050

ccctcaccac ctgccctcct tctaccagga cctcagaagc ccagacctgg 2100

ggaccccacc tacacagggg cattgacaga ctggagttga aagccgacga 2150

accgacacgc ggcagagtca ataattcaat aaaaaagtta cgaactttct 2200

ctgtaacttg ggtttcaata attatggatt tttatgaaaa cttgaaataa 2250

taaaaagaga aaaaaactaa aaaaaaaaaa aaaaaaaaaa 2290

<210> 324
<211> 620
<212> PRT
<213> Homo Sapien

<400> 324
Met Gln Val Ser Lys Arg Met Leu Ala Gly Gly Val Arg Ser Met
1               5                   10                  15

Pro Ser Pro Leu Leu Ala Cys Trp Gln Pro Ile Leu Leu Leu Val
                20                  25                  30

Leu Gly Ser Val Leu Ser Gly Ser Ala Thr Gly Cys Pro Pro Arg
                35                  40                  45

Cys Glu Cys Ser Ala Gln Asp Arg Ala Val Leu Cys His Arg Lys
                50                  55                  60

Cys Phe Val Ala Val Pro Glu Gly Ile Pro Thr Glu Thr Arg Leu
                65                  70                  75

Leu Asp Leu Gly Lys Asn Arg Ile Lys Thr Leu Asn Gln Asp Glu
                80                  85                  90

Phe Ala Ser Phe Pro His Leu Glu Glu Leu Glu Leu Asn Glu Asn
                95                  100                 105

Ile Val Ser Ala Val Glu Pro Gly Ala Phe Asn Asn Leu Phe Asn
                110                 115                 120

Leu Arg Thr Leu Gly Leu Arg Ser Asn Arg Leu Lys Leu Ile Pro
                125                 130                 135

Leu Gly Val Phe Thr Gly Leu Ser Asn Leu Thr Lys Gln Asp Ile
                140                 145                 150

Ser Glu Asn Lys Ile Val Ile Leu Leu Asp Tyr Met Phe Gln Asp
                155                 160                 165

Leu Tyr Asn Leu Lys Ser Leu Glu Val Gly Asp Asn Asp Leu Val
                170                 175                 180

Tyr Ile Ser His Arg Ala Phe Ser Gly Leu Asn Ser Leu Glu Gln
185 190 195

Leu Thr Leu Glu Lys Cys Asn Leu Thr Ser Ile Pro Thr Glu Ala
200 205 210

Leu Ser His Leu His Gly Leu Ile Val Leu Arg Leu Arg His Leu
215 220 225

Asn Ile Asn Ala Ile Arg Asp Tyr Ser Phe Lys Arg Leu Tyr Arg
230 235 240

Leu Lys Val Leu Glu Ile Ser His Trp Pro Tyr Leu Asp Thr Met
245 250 255

Thr Pro Asn Cys Leu Tyr Gly Leu Asn Leu Thr Ser Leu Ser Ile
260 265 270

Thr His Cys Asn Leu Thr Ala Val Pro Tyr Leu Ala Val Arg His
275 280 285

Leu Val Tyr Leu Arg Phe Leu Asn Leu Ser Tyr Asn Pro Ile Ser
290 295 300

Thr Ile Glu Gly Ser Met Leu His Glu Leu Leu Arg Leu Gln Glu
305 310 315

Ile Gln Leu Val Gly Gly Gln Leu Ala Val Val Glu Pro Tyr Ala
320 325 330

Phe Arg Gly Leu Asn Tyr Leu Arg Val Leu Asn Val Ser Gly Asn
335 340 345

Gln Leu Thr Thr Leu Glu Glu Ser Val Phe His Ser Val Gly Asn
350 355 360

Leu Glu Thr Leu Ile Leu Asp Ser Asn Pro Leu Ala Cys Asp Cys
365 370 375

Arg Leu Leu Trp Val Phe Arg Arg Arg Trp Arg Leu Asn Phe Asn
380 385 390

Arg Gln Gln Pro Thr Cys Ala Thr Pro Glu Phe Val Gln Gly Lys
395 400 405

Glu Phe Lys Asp Phe Pro Asp Val Leu Leu Pro Asn Tyr Phe Thr
410 415 420

Cys Arg Arg Ala Arg Ile Arg Asp Arg Lys Ala Gln Gln Val Phe
425 430 435

Val Asp Glu Gly His Thr Val Gln Phe Val Cys Arg Ala Asp Gly
440 445 450

Asp Pro Pro Pro Ala Ile Leu Trp Leu Ser Pro Arg Lys His Leu
455 460 465

Val Ser Ala Lys Ser Asn Gly Arg Leu Thr Val Phe Pro Asp Gly
470 475 480

Thr Leu Glu Val Arg Tyr Ala Gln Val Gln Asp Asn Gly Thr Tyr
485 490 495

```
Leu Cys Ile Ala Ala Asn Ala Gly Gly Asn Asp Ser Met Pro Ala
                500                 505                 510

His Leu His Val Arg Ser Tyr Ser Pro Asp Trp Pro His Gln Pro
                515                 520                 525

Asn Lys Thr Phe Ala Phe Ile Ser Asn Gln Pro Gly Glu Gly Glu
                530                 535                 540

Ala Asn Ser Thr Arg Ala Thr Val Pro Phe Pro Phe Asp Ile Lys
                545                 550                 555

Thr Leu Ile Ile Ala Thr Thr Met Gly Phe Ile Ser Phe Leu Gly
                560                 565                 570

Val Val Leu Phe Cys Leu Val Leu Leu Phe Leu Trp Ser Arg Gly
                575                 580                 585

Lys Gly Asn Thr Lys His Asn Ile Glu Ile Glu Tyr Val Pro Arg
                590                 595                 600

Lys Ser Asp Ala Gly Ile Ser Ser Ala Asp Ala Pro Arg Lys Phe
                605                 610                 615

Asn Met Lys Met Ile
                620
```

<210> 325
<211> 1670
<212> DNA
<213> Homo Sapien

<400> 325

```
cccacgcgtc cgcccacgcg tccgagggac aagagagaag agagactgaa 50

acagggagaa gaggcaggag aggaggaggt ggggagagca cgaagctgga 100

ggccgacact gagggagggc gggaggaggt gaagaaggag agaggggaga 150

agaggcagga gctggaaagg agagagggag gaggaggagg agatgcggga 200

tggagacctg gagttaggtg gcttgggaga gcttaatgaa aagagaacgg 250

agaggaggtg tgggttagga accaagaggt agccctgtgg gcagcagaag 300

gctgagagga gtaggaagat caggagctag agggagactg gagggttccg 350

ggaaaagagc agaggaaaga ggaaagacac agagagacgg gagagagaag 400

aagagtgggt ttgaagggcg gatctcagtc cctggctgct ttggcatttg 450

gggaactggg actccctgtg gggaggagag gaaagctgga agtcctggag 500

ggacagggtc ccagaaggag gggacagagg agctgagaga gggggcaggg 550

gcgttgggca ggggtccctc ggaggcctcc tggggatggg ggctgcagct 600

cgtctgagcg cccctcgagc gctggtactc tgggctgcac tgggggcagc 650

agctcacatc ggaccagcac ctgaccccga ggactggtgg agctacaagg 700

ataatctcca gggaaacttc gtgccagggc ctcctttctg gggcctggtg 750

aatgcagcgt ggagtctgtg tgctgtgggg aagcggcaga gccccgtgga 800
```

tgtggagctg aagagggttc tttatgaccc ctttctgccc ccattaaggc 850

tcagcactgg aggagagaag ctccggggaa ccttgtacaa caccggccga 900

catgtctcct tcctgcctgc accccgacct gtggtcaatg tgtctggagg 950

tcccctcctt tacagccacc gactcagtga actgcggctg ctgtttggag 1000

ctcgcgacgg agccggctcg gaacatcaga tcaaccacca gggcttctct 1050

gctgaggtgc agctcattca cttcaaccag gaactctacg ggaatttcag 1100

cgctgcctcc cgcggcccca atggcctggc cattctcagc ctctttgtca 1150

acgttgccag tacctctaac ccattcctca gtcgcctcct taaccgcgac 1200

accatcactc gcatctccta caagaatgat gcctactttc ttcaagacct 1250

gagcctggag ctcctgttcc ctgaatcctt cggcttcatc acctatcagg 1300

gctctctcag caccccgccc tgctccgaga ctgtcacctg gatcctcatt 1350

gaccgggccc tcaatatcac ctcccttcag atgcactccc tgagactcct 1400

gagccagaat cctccatctc agatcttcca gagcctcagc ggtaacagcc 1450

ggcccctgca gcccttggcc cacagggcac tgaggggcaa cagggacccc 1500

cggcaccccg agaggcgctg ccgaggcccc aactaccgcc tgcatgtgga 1550

tggtgtcccc catggtcgct gagactcccc ttcgaggatt gcacccgccc 1600

gtcctaagcc tccccacaag gcgaggggag ttacccctaa aacaaagcta 1650

ttaaagggac agaatactta 1670

<210> 326
<211> 328
<212> PRT
<213> Homo Sapien

<400> 326

```
Met Gly Ala Ala Ala Arg Leu Ser Ala Pro Arg Ala Leu Val Leu
 1               5                  10                  15

Trp Ala Ala Leu Gly Ala Ala Ala His Ile Gly Pro Ala Pro Asp
                20                  25                  30

Pro Glu Asp Trp Trp Ser Tyr Lys Asp Asn Leu Gln Gly Asn Phe
                35                  40                  45

Val Pro Gly Pro Pro Phe Trp Gly Leu Val Asn Ala Ala Trp Ser
                50                  55                  60

Leu Cys Ala Val Gly Lys Arg Gln Ser Pro Val Asp Val Glu Leu
                65                  70                  75

Lys Arg Val Leu Tyr Asp Pro Phe Leu Pro Pro Leu Arg Leu Ser
                80                  85                  90

Thr Gly Gly Glu Lys Leu Arg Gly Thr Leu Tyr Asn Thr Gly Arg
                95                 100                 105
```

His Val Ser Phe Leu Pro Ala Pro Arg Pro Val Val Asn Val Ser
110 115 120

Gly Gly Pro Leu Leu Tyr Ser His Arg Leu Ser Glu Leu Arg Leu
125 130 135

Leu Phe Gly Ala Arg Asp Gly Ala Gly Ser Glu His Gln Ile Asn
140 145 150

His Gln Gly Phe Ser Ala Glu Val Gln Leu Ile His Phe Asn Gln
155 160 165

Glu Leu Tyr Gly Asn Phe Ser Ala Ala Ser Arg Gly Pro Asn Gly
170 175 180

Leu Ala Ile Leu Ser Leu Phe Val Asn Val Ala Ser Thr Ser Asn
185 190 195

Pro Phe Leu Ser Arg Leu Leu Asn Arg Asp Thr Ile Thr Arg Ile
200 205 210

Ser Tyr Lys Asn Asp Ala Tyr Phe Leu Gln Asp Leu Ser Leu Glu
215 220 225

Leu Leu Phe Pro Glu Ser Phe Gly Phe Ile Thr Tyr Gln Gly Ser
230 235 240

Leu Ser Thr Pro Pro Cys Ser Glu Thr Val Thr Trp Ile Leu Ile
245 250 255

Asp Arg Ala Leu Asn Ile Thr Ser Leu Gln Met His Ser Leu Arg
260 265 270

Leu Leu Ser Gln Asn Pro Pro Ser Gln Ile Phe Gln Ser Leu Ser
275 280 285

Gly Asn Ser Arg Pro Leu Gln Pro Leu Ala His Arg Ala Leu Arg
290 295 300

Gly Asn Arg Asp Pro Arg His Pro Glu Arg Arg Cys Arg Gly Pro
305 310 315

Asn Tyr Arg Leu His Val Asp Gly Val Pro His Gly Arg
320 325

<210> 327
<211> 2454
<212> DNA
<213> Homo Sapien

<400> 327
ggactaatct gtgggagcag tttattccag tatcacccag ggtgcagcca 50

caccaggact gtgttgaagg gtgttttttt tcttttaaat gtaatacctc 100

ctcatctttt cttcttacac agtgtctgag aacatttaca ttatagataa 150

gtagtacatg gtggataact tctactttta ggaggactac tctcttctga 200

cagtcctaga ctggtcttct acactaagac accatgaagg agtatgtgct 250

cctattattc ctggctttgt gctctgccaa acccttcttt agcccttcac 300

acatcgcact gaagaatatg atgctgaagg atatggaaga cacagatgat 350

gatgatgatg atgatgatga tgatgatgat gatgaggaca actctctttt 400

tccaacaaga gagccaagaa gccatttttt tccatttgat ctgtttccaa 450

tgtgtccatt tggatgtcag tgctattcac gagttgtaca ttgctcagat 500

ttaggtttga cctcagtccc aaccaacatt ccatttgata ctcgaatgct 550

tgatcttcaa aacaataaaa ttaaggaaat caaagaaaat gattttaaag 600

gactcacttc actttatggt ctgatcctga acaacaacaa gctaacgaag 650

attcacccaa aagcctttct aaccacaaag aagttgcgaa ggctgtatct 700

gtcccacaat caactaagtg aaataccact taatcttccc aaatcattag 750

cagaactcag aattcatgaa aataaagtta agaaaataca aaaggacaca 800

ttcaaaggaa tgaatgcttt acacgttttg gaaatgagtg caaaccctct 850

tgataataat gggatagagc caggggcatt tgaaggggtg acggtgttcc 900

atatcagaat tgcagaagca aaactgacct cagttcctaa aggcttacca 950

ccaactttat tggagcttca cttagattat aataaaattt caacagtgga 1000

acttgaggat tttaaacgat acaaagaact acaaaggctg ggcctaggaa 1050

acaacaaaat cacagatatc gaaaatggga gtcttgctaa cataccacgt 1100

gtgagagaaa tacatttgga aaacaataaa ctaaaaaaaa tcccttcagg 1150

attaccagag ttgaaatacc tccagataat cttccttcat tctaattcaa 1200

ttgcaagagt gggagtaaat gacttctgtc caacagtgcc aaagatgaag 1250

aaatctttat acagtgcaat aagtttattc aacaacccgg tgaaatactg 1300

ggaaatgcaa cctgcaacat ttcgttgtgt tttgagcaga atgagtgttc 1350

agcttgggaa ctttggaatg taataattag taattggtaa tgtccattta 1400

atataagatt caaaaatccc tacatttgga atacttgaac tctattaata 1450

atggtagtat tatatataca agcaaatatc tattctcaag tggtaagtcc 1500

actgacttat tttatgacaa gaaatttcaa cggaattttg ccaaactatt 1550

gatacataag gggttgagag aaacaagcat ctattgcagt ttcctttttg 1600

cgtacaaatg atcttacata aatctcatgc ttgaccattc ctttcttcat 1650

aacaaaaaag taagatattc ggtatttaac actttgttat caagcacatt 1700

ttaaaaagaa ctgtactgta aatggaatgc ttgacttagc aaaatttgtg 1750

ctctttcatt tgctgttaga aaaacagaat taacaaagac agtaatgtga 1800

agagtgcatt acactattct tattctttag taacttgggt agtactgtaa 1850

tatttttaat catcttaaag tatgatttga tataatctta ttgaaattac 1900

cttatcatgt cttagagccc gtctttatgt ttaaaactaa tttcttaaaa 1950

taaagccttc agtaaatgtt cattaccaac ttgataaatg ctactcataa 2000

gagctggttt ggggctatag catatgcttt ttttttttta attattacct 2050

gatttaaaaa tctctgtaaa aacgtgtagt gtttcataaa atctgtaact 2100

cgcattttaa tgatccgcta ttataagctt ttaatagcat gaaaattgtt 2150

aggctatata acattgccac ttcaactcta aggaatattt ttgagatatc 2200

cctttggaag accttgcttg gaagagcctg gacactaaca attctacacc 2250

aaattgtctc ttcaaatacg tatggactgg ataactctga gaaacacatc 2300

tagtataact gaataagcag agcatcaaat taaacagaca gaaaccgaaa 2350

gctctatata aatgctcaga gttctttatg tatttcttat tggcattcaa 2400

catatgtaaa atcagaaaac agggaaattt tcattaaaaa tattggtttg 2450

aaat 2454

<210> 328
<211> 379
<212> PRT
<213> Homo Sapien

<400> 328
Met Lys Glu Tyr Val Leu Leu Leu Phe Leu Ala Leu Cys Ser Ala
1 5 10 15

Lys Pro Phe Phe Ser Pro Ser His Ile Ala Leu Lys Asn Met Met
20 25 30

Leu Lys Asp Met Glu Asp Thr Asp Asp Asp Asp Asp Asp Asp Asp
35 40 45

Asp Asp Asp Asp Asp Glu Asp Asn Ser Leu Phe Pro Thr Arg Glu
50 55 60

Pro Arg Ser His Phe Phe Pro Phe Asp Leu Phe Pro Met Cys Pro
65 70 75

Phe Gly Cys Gln Cys Tyr Ser Arg Val Val His Cys Ser Asp Leu
80 85 90

Gly Leu Thr Ser Val Pro Thr Asn Ile Pro Phe Asp Thr Arg Met
95 100 105

Leu Asp Leu Gln Asn Asn Lys Ile Lys Glu Ile Lys Glu Asn Asp
110 115 120

Phe Lys Gly Leu Thr Ser Leu Tyr Gly Leu Ile Leu Asn Asn Asn
125 130 135

Lys Leu Thr Lys Ile His Pro Lys Ala Phe Leu Thr Thr Lys Lys
140 145 150

Leu Arg Arg Leu Tyr Leu Ser His Asn Gln Leu Ser Glu Ile Pro
155 160 165

Leu Asn Leu Pro Lys Ser Leu Ala Glu Leu Arg Ile His Glu Asn
170 175 180

Lys Val Lys Lys Ile Gln Lys Asp Thr Phe Lys Gly Met Asn Ala
185 190 195

Leu His Val Leu Glu Met Ser Ala Asn Pro Leu Asp Asn Asn Gly
200 205 210

Ile Glu Pro Gly Ala Phe Glu Gly Val Thr Val Phe His Ile Arg
215 220 225

Ile Ala Glu Ala Lys Leu Thr Ser Val Pro Lys Gly Leu Pro Pro
230 235 240

Thr Leu Leu Glu Leu His Leu Asp Tyr Asn Lys Ile Ser Thr Val
245 250 255

Glu Leu Glu Asp Phe Lys Arg Tyr Lys Glu Leu Gln Arg Leu Gly
260 265 270

Leu Gly Asn Asn Lys Ile Thr Asp Ile Glu Asn Gly Ser Leu Ala
275 280 285

Asn Ile Pro Arg Val Arg Glu Ile His Leu Glu Asn Asn Lys Leu
290 295 300

Lys Lys Ile Pro Ser Gly Leu Pro Glu Leu Lys Tyr Leu Gln Ile
305 310 315

Ile Phe Leu His Ser Asn Ser Ile Ala Arg Val Gly Val Asn Asp
320 325 330

Phe Cys Pro Thr Val Pro Lys Met Lys Lys Ser Leu Tyr Ser Ala
335 340 345

Ile Ser Leu Phe Asn Asn Pro Val Lys Tyr Trp Glu Met Gln Pro
350 355 360

Ala Thr Phe Arg Cys Val Leu Ser Arg Met Ser Val Gln Leu Gly
365 370 375

Asn Phe Gly Met

<210> 329
<211> 1514
<212> DNA
<213> Homo Sapien

<400> 329
ggggtctccc tcagggccgg gaggcacagc ggtccctgct tgctgaaggg 50

ctggatgtac gcatccgcag gttcccgcgg acttgggggc gcccgctgag 100

ccccggcgcc cgcagaagac ttgtgtttgc ctcctgcagc ctcaacccgg 150

agggcagcga gggcctacca ccatgatcac tggtgtgttc agcatgcgct 200

tgtggacccc agtgggcgtc ctgacctcgc tggcgtactg cctgcaccag 250

cggcgggtgg ccctggccga gctgcaggag gccgatggcc agtgtccggt 300

cgaccgcagc ctgctgaagt tgaaaatggt gcaggtcgtg tttcgacacg 350

gggctcggag tcctctcaag ccgctcccgc tggaggagca ggtagagtgg 400

aacccccagc tattagaggt cccaccccaa actcagtttg attacacagt 450

caccaatcta gctggtggtc cgaaaccata ttctccttac gactctcaat 500

accatgagac caccctgaag gggggcatgt ttgctgggca gctgaccaag 550

gtgggcatgc agcaaatgtt tgccttggga gagagactga ggaagaacta 600

tgtggaagac attccctttc tttcaccaac cttcaaccca caggaggtct 650

ttattcgttc cactaacatt tttcggaatc tggagtccac ccgttgtttg 700

ctggctgggc ttttccagtg tcagaaagaa ggacccatca tcatccacac 750

tgatgaagca gattcagaag tcttgtatcc caactaccaa agctgctgga 800

gcctgaggca gagaaccaga ggccggaggc agactgcctc tttacagcca 850

ggaatctcag aggatttgaa aaaggtgaag gacaggatgg gcattgacag 900

tagtgataaa gtggacttct tcatcctcct ggacaacgtg gctgccgagc 950

aggcacacaa cctcccaagc tgccccatgc tgaagagatt tgcacggatg 1000

atcgaacaga gagctgtgga cacatccttg tacatactgc ccaaggaaga 1050

cagggaaagt cttcagatgg cagtaggccc attcctccac atcctagaga 1100

gcaacctgct gaaagccatg gactctgcca ctgcccccga caagatcaga 1150

aagctgtatc tctatgcggc tcatgatgtg accttcatac cgctcttaat 1200

gaccctgggg atttttgacc acaaatggcc accgtttgct gttgacctga 1250

ccatggaact ttaccagcac ctggaatcta aggagtggtt tgtgcagctc 1300

tattaccacg ggaaggagca ggtgccgaga ggttgccctg atgggctctg 1350

cccgctggac atgttcttga atgccatgtc agtttatacc ttaagcccag 1400

aaaaatacca tgcactctgc tctcaaactc aggtgatgga agttggaaat 1450

gaagagtaac tgatttataa aagcaggatg tgttgatttt aaaataaagt 1500

gcctttatac aatg 1514

<210> 330
<211> 428
<212> PRT
<213> Homo Sapien

<400> 330
Met Ile Thr Gly Val Phe Ser Met Arg Leu Trp Thr Pro Val Gly
1 5 10 15

Val Leu Thr Ser Leu Ala Tyr Cys Leu His Gln Arg Arg Val Ala
20 25 30

Leu Ala Glu Leu Gln Glu Ala Asp Gly Gln Cys Pro Val Asp Arg
35 40 45

Ser Leu Leu Lys Leu Lys Met Val Gln Val Val Phe Arg His Gly
50 55 60

```
Ala Arg Ser Pro Leu Lys Pro Leu Pro Leu Glu Glu Gln Val Glu
                65                  70                  75

Trp Asn Pro Gln Leu Leu Glu Val Pro Pro Gln Thr Gln Phe Asp
                80                  85                  90

Tyr Thr Val Thr Asn Leu Ala Gly Gly Pro Lys Pro Tyr Ser Pro
                95                  100                 105

Tyr Asp Ser Gln Tyr His Glu Thr Thr Leu Lys Gly Gly Met Phe
                110                 115                 120

Ala Gly Gln Leu Thr Lys Val Gly Met Gln Gln Met Phe Ala Leu
                125                 130                 135

Gly Glu Arg Leu Arg Lys Asn Tyr Val Glu Asp Ile Pro Phe Leu
                140                 145                 150

Ser Pro Thr Phe Asn Pro Gln Glu Val Phe Ile Arg Ser Thr Asn
                155                 160                 165

Ile Phe Arg Asn Leu Glu Ser Thr Arg Cys Leu Leu Ala Gly Leu
                170                 175                 180

Phe Gln Cys Gln Lys Glu Gly Pro Ile Ile Ile His Thr Asp Glu
                185                 190                 195

Ala Asp Ser Glu Val Leu Tyr Pro Asn Tyr Gln Ser Cys Trp Ser
                200                 205                 210

Leu Arg Gln Arg Thr Arg Gly Arg Arg Gln Thr Ala Ser Leu Gln
                215                 220                 225

Pro Gly Ile Ser Glu Asp Leu Lys Lys Val Lys Asp Arg Met Gly
                230                 235                 240

Ile Asp Ser Ser Asp Lys Val Asp Phe Phe Ile Leu Leu Asp Asn
                245                 250                 255

Val Ala Ala Glu Gln Ala His Asn Leu Pro Ser Cys Pro Met Leu
                260                 265                 270

Lys Arg Phe Ala Arg Met Ile Glu Gln Arg Ala Val Asp Thr Ser
                275                 280                 285

Leu Tyr Ile Leu Pro Lys Glu Asp Arg Glu Ser Leu Gln Met Ala
                290                 295                 300

Val Gly Pro Phe Leu His Ile Leu Glu Ser Asn Leu Leu Lys Ala
                305                 310                 315

Met Asp Ser Ala Thr Ala Pro Asp Lys Ile Arg Lys Leu Tyr Leu
                320                 325                 330

Tyr Ala Ala His Asp Val Thr Phe Ile Pro Leu Leu Met Thr Leu
                335                 340                 345

Gly Ile Phe Asp His Lys Trp Pro Pro Phe Ala Val Asp Leu Thr
                350                 355                 360

Met Glu Leu Tyr Gln His Leu Glu Ser Lys Glu Trp Phe Val Gln
                365                 370                 375
```

```
Leu Tyr Tyr His Gly Lys Glu Gln Val Pro Arg Gly Cys Pro Asp
                380                 385                 390

Gly Leu Cys Pro Leu Asp Met Phe Leu Asn Ala Met Ser Val Tyr
                395                 400                 405

Thr Leu Ser Pro Glu Lys Tyr His Ala Leu Cys Ser Gln Thr Gln
                410                 415                 420

Val Met Glu Val Gly Asn Glu Glu
                425
```

<210> 331
<211> 2477
<212> DNA
<213> Homo Sapien

<400> 331

```
cgagggcttt tccggctccg gaatggcaca tgtgggaatc ccagtcttgt 50

tggctacaac atttttccct ttcctaacaa gttctaacag ctgttctaac 100

agctagtgat caggggttct tcttgctgga gaagaaaggg ctgagggcag 150

agcagggcac tctcactcag ggtgaccagc tccttgcctc tctgtggata 200

acagagcatg agaaagtgaa gagatgcagc ggagtgaggt gatggaagtc 250

taaaatagga aggaattttg tgtgcaatat cagactctgg gagcagttga 300

cctggagagc ctgggggagg gcctgcctaa caagctttca aaaaacagga 350

gcgacttcca ctgggctggg ataagacgtg ccggtaggat agggaagact 400

gggtttagtc ctaatatcaa attgactggc tgggtgaact tcaacagcct 450

tttaacctct ctgggagatg aaaacgatgg cttaaggggc cagaaataga 500

gatgctttgt aaaataaaat tttaaaaaaa gcaagtattt tatagcataa 550

aggctagaga ccaaaataga taacaggatt ccctgaacat tcctaagagg 600

gagaaagtat gttaaaaata gaaaaaccaa aatgcagaag gaggagactc 650

acagagctaa accaggatgg ggaccctggg tcaggccagc ctctttgctc 700

ctcccggaaa ttatttttgg tctgaccact ctgccttgtg ttttgcagaa 750

tcatgtgagg gccaaccggg gaaggtggag cagatgagca cacacaggag 800

ccgtctcctc accgccgccc ctctcagcat ggaacagagg cagccctggc 850

cccgggccct ggaggtggac agccgctctg tggtcctgct ctcagtggtc 900

tgggtgctgc tggccccccc agcagccggc atgcctcagt tcagcacctt 950

ccactctgag aatcgtgact ggaccttcaa ccacttgacc gtccaccaag 1000

ggacgggggc cgtctatgtg ggggccatca accgggtcta taagctgaca 1050

ggcaacctga ccatccaggt ggctcataag acagggccag aagaggacaa 1100

caagtctcgt tacccgcccc tcatcgtgca gccctgcagc gaagtgctca 1150
```

ccctcaccaa caatgtcaac aagctgctca tcattgacta ctctgagaac 1200
cgcctgctgg cctgtgggag cctctaccag gggtctgca agctgctgcg 1250
gctggatgac ctcttcatcc tggtggagcc atcccacaag aaggagcact 1300
acctgtccag tgtcaacaag acgggcacca tgtacggggt gattgtgcgc 1350
tctgagggtg aggatggcaa gctcttcatc ggcacggctg tggatgggaa 1400
gcaggattac ttcccgaccc tgtccagccg gaagctgccc cgagaccctg 1450
agtcctcagc catgctcgac tatgagctac acagcgattt tgtctcctct 1500
ctcatcaaga tcccttcaga caccctggcc ctggtctccc actttgacat 1550
cttctacatc tacggctttg ctagtggggg ctttgtctac tttctcactg 1600
tccagcccga gacccctgag ggtgtggcca tcaactccgc tggagacctc 1650
ttctacacct cacgcatcgt gcggctctgc aaggatgacc ccaagttcca 1700
ctcatacgtg tccctgccct tcggctgcac ccgggccggg gtggaatacc 1750
gcctcctgca ggctgcttac ctggccaagc ctggggactc actggcccag 1800
gccttcaata tcaccagcca ggacgatgta ctctttgcca tcttctccaa 1850
agggcagaag cagtatcacc acccgcccga tgactctgcc ctgtgtgcct 1900
tccctatccg ggccatcaac ttgcagatca aggagcgcct gcagtcctgc 1950
taccagggcg agggcaacct ggagctcaac tggctgctgg ggaaggacgt 2000
ccagtgcacg aaggcgcctg tccccatcga tgataacttc tgtggactgg 2050
acatcaacca gcccctggga ggctcaactc cagtggaggg cctgaccctg 2100
tacaccacca gcagggaccg catgacctct gtggcctcct acgtttacaa 2150
cggctacagc gtggtttttg tggggactaa gagtggcaag ctgaaaaagg 2200
taagagtcta tgagttcaga tgctccaatg ccattcacct cctcagcaaa 2250
gagtccctct tggaaggtag ctattggtgg agatttaact ataggcaact 2300
ttattttctt ggggaacaaa ggtgaaatgg ggaggtaaga aggggttaat 2350
tttgtgactt agcttctagc tacttcctcc agccatcagt cattgggtat 2400
gtaaggaatg caagcgtatt tcaatatttc ccaaacttta agaaaaaact 2450
ttaagaaggt acatctgcaa aagcaaa 2477

<210> 332
<211> 552
<212> PRT
<213> Homo Sapien

<400> 332
Met Gly Thr Leu Gly Gln Ala Ser Leu Phe Ala Pro Pro Gly Asn
1 5 10 15

Tyr Phe Trp Ser Asp His Ser Ala Leu Cys Phe Ala Glu Ser Cys

20 25 30

Glu Gly Gln Pro Gly Lys Val Glu Gln Met Ser Thr His Arg Ser
35 40 45

Arg Leu Leu Thr Ala Ala Pro Leu Ser Met Glu Gln Arg Gln Pro
50 55 60

Trp Pro Arg Ala Leu Glu Val Asp Ser Arg Ser Val Val Leu Leu
65 70 75

Ser Val Val Trp Val Leu Leu Ala Pro Pro Ala Ala Gly Met Pro
80 85 90

Gln Phe Ser Thr Phe His Ser Glu Asn Arg Asp Trp Thr Phe Asn
95 100 105

His Leu Thr Val His Gln Gly Thr Gly Ala Val Tyr Val Gly Ala
110 115 120

Ile Asn Arg Val Tyr Lys Leu Thr Gly Asn Leu Thr Ile Gln Val
125 130 135

Ala His Lys Thr Gly Pro Glu Glu Asp Asn Lys Ser Arg Tyr Pro
140 145 150

Pro Leu Ile Val Gln Pro Cys Ser Glu Val Leu Thr Leu Thr Asn
155 160 165

Asn Val Asn Lys Leu Leu Ile Ile Asp Tyr Ser Glu Asn Arg Leu
170 175 180

Leu Ala Cys Gly Ser Leu Tyr Gln Gly Val Cys Lys Leu Leu Arg
185 190 195

Leu Asp Asp Leu Phe Ile Leu Val Glu Pro Ser His Lys Lys Glu
200 205 210

His Tyr Leu Ser Ser Val Asn Lys Thr Gly Thr Met Tyr Gly Val
215 220 225

Ile Val Arg Ser Glu Gly Glu Asp Gly Lys Leu Phe Ile Gly Thr
230 235 240

Ala Val Asp Gly Lys Gln Asp Tyr Phe Pro Thr Leu Ser Ser Arg
245 250 255

Lys Leu Pro Arg Asp Pro Glu Ser Ser Ala Met Leu Asp Tyr Glu
260 265 270

Leu His Ser Asp Phe Val Ser Ser Leu Ile Lys Ile Pro Ser Asp
275 280 285

Thr Leu Ala Leu Val Ser His Phe Asp Ile Phe Tyr Ile Tyr Gly
290 295 300

Phe Ala Ser Gly Gly Phe Val Tyr Phe Leu Thr Val Gln Pro Glu
305 310 315

Thr Pro Glu Gly Val Ala Ile Asn Ser Ala Gly Asp Leu Phe Tyr
320 325 330

Thr Ser Arg Ile Val Arg Leu Cys Lys Asp Asp Pro Lys Phe His
335 340 345

Ser Tyr Val Ser Leu Pro Phe Gly Cys Thr Arg Ala Gly Val Glu
350 355 360

Tyr Arg Leu Leu Gln Ala Ala Tyr Leu Ala Lys Pro Gly Asp Ser
365 370 375

Leu Ala Gln Ala Phe Asn Ile Thr Ser Gln Asp Asp Val Leu Phe
380 385 390

Ala Ile Phe Ser Lys Gly Gln Lys Gln Tyr His His Pro Pro Asp
395 400 405

Asp Ser Ala Leu Cys Ala Phe Pro Ile Arg Ala Ile Asn Leu Gln
410 415 420

Ile Lys Glu Arg Leu Gln Ser Cys Tyr Gln Gly Glu Gly Asn Leu
425 430 435

Glu Leu Asn Trp Leu Leu Gly Lys Asp Val Gln Cys Thr Lys Ala
440 445 450

Pro Val Pro Ile Asp Asp Asn Phe Cys Gly Leu Asp Ile Asn Gln
455 460 465

Pro Leu Gly Gly Ser Thr Pro Val Glu Gly Leu Thr Leu Tyr Thr
470 475 480

Thr Ser Arg Asp Arg Met Thr Ser Val Ala Ser Tyr Val Tyr Asn
485 490 495

Gly Tyr Ser Val Val Phe Val Gly Thr Lys Ser Gly Lys Leu Lys
500 505 510

Lys Val Arg Val Tyr Glu Phe Arg Cys Ser Asn Ala Ile His Leu
515 520 525

Leu Ser Lys Glu Ser Leu Leu Glu Gly Ser Tyr Trp Trp Arg Phe
530 535 540

Asn Tyr Arg Gln Leu Tyr Phe Leu Gly Glu Gln Arg
545 550

<210> 333
<211> 1520
<212> DNA
<213> Homo Sapien

<400> 333
gctgagtctg ctgctcctgc tgctgctgct ccagcctgta acctgtgcct 50

acaccacgcc aggccccccc agagccctca ccacgctggg cgcccccaga 100

gcccacacca tgccgggcac ctacgctccc tcgaccacac tcagtagtcc 150

cagcacccag ggcctgcaag agcaggcacg ggccctgatg cgggacttcc 200

cgctcgtgga cggccacaac gacctgcccc tggtcctaag gcaggtttac 250

cagaaagggc tacaggatgt taacctgcgc aatttcagct acggccagac 300

cagcctggac aggcttagag atggcctcgt gggcgcccag ttctggtcag 350

cctatgtgcc atgccagacc caggaccggg atgccctgcg cctcaccctg 400

gagcagattg acctcatacg ccgcatgtgt gcctcctatt ctgagctgga 450

gcttgtgacc tcggctaaag ctctgaacga cactcagaaa ttggcctgcc 500

tcatcggtgt agagggtggc cactcgctgg acaatagcct ctccatctta 550

cgtaccttct acatgctggg agtgcgctac ctgacgctca cccacacctg 600

caacacaccc tgggcagaga gctccgctaa gggcgtccac tccttctaca 650

acaacatcag cgggctgact gactttggtg agaaggtggt ggcagaaatg 700

aaccgcctgg gcatgatggt agacttatcc catgtctcag atgctgtggc 750

acggcgggcc ctggaagtgt cacaggcacc tgtgatcttc tcccactcgg 800

ctgcccgggg tgtgtgcaac agtgctcgga atgttcctga tgacatcctg 850

cagcttctga agaagaacgg tggcgtcgtg atggtgtctt tgtccatggg 900

agtaatacag tgcaacccat cagccaatgt gtccactgtg gcagatcact 950

tcgaccacat caaggctgtc attggatcca agttcatcgg gattggtgga 1000

gattatgatg gggccggcaa attccctcag gggctggaag acgtgtccac 1050

atacccggtc ctgatagagg agttgctgag tcgtggctgg agtgaggaag 1100

agcttcaggg tgtccttcgt ggaaacctgc tgcgggtctt cagacaagtg 1150

gaaaaggtac aggaagaaaa caaatggcaa agccccttgg aggacaagtt 1200

cccggatgag cagctgagca gttcctgcca ctccgacctc tcacgtctgc 1250

gtcagagaca gagtctgact tcaggccagg aactcactga gattcccata 1300

cactggacag ccaagttacc agccaagtgg tcagtctcag agtcctcccc 1350

ccacatggcc ccagtccttg cagttgtggc caccttccca gtccttattc 1400

tgtggctctg atgacccagt tagtcctgcc agatgtcact gtagcaagcc 1450

acagacaccc cacaaagttc ccctgttgtg caggcacaaa tatttcctga 1500

aataaatgtt ttggacatag 1520

<210> 334
<211> 433
<212> PRT
<213> Homo Sapien

<400> 334

```
Met Pro Gly Thr Tyr Ala Pro Ser Thr Thr Leu Ser Ser Pro Ser
  1               5                  10                  15

Thr Gln Gly Leu Gln Glu Gln Ala Arg Ala Leu Met Arg Asp Phe
                 20                  25                  30

Pro Leu Val Asp Gly His Asn Asp Leu Pro Leu Val Leu Arg Gln
                 35                  40                  45

Val Tyr Gln Lys Gly Leu Gln Asp Val Asn Leu Arg Asn Phe Ser
                 50                  55                  60
```

```
Tyr Gly Gln Thr Ser Leu Asp Arg Leu Arg Asp Gly Leu Val Gly
                65                  70                  75

Ala Gln Phe Trp Ser Ala Tyr Val Pro Cys Gln Thr Gln Asp Arg
                80                  85                  90

Asp Ala Leu Arg Leu Thr Leu Glu Gln Ile Asp Leu Ile Arg Arg
                95                  100                 105

Met Cys Ala Ser Tyr Ser Glu Leu Glu Leu Val Thr Ser Ala Lys
                110                 115                 120

Ala Leu Asn Asp Thr Gln Lys Leu Ala Cys Leu Ile Gly Val Glu
                125                 130                 135

Gly Gly His Ser Leu Asp Asn Ser Leu Ser Ile Leu Arg Thr Phe
                140                 145                 150

Tyr Met Leu Gly Val Arg Tyr Leu Thr Leu Thr His Thr Cys Asn
                155                 160                 165

Thr Pro Trp Ala Glu Ser Ser Ala Lys Gly Val His Ser Phe Tyr
                170                 175                 180

Asn Asn Ile Ser Gly Leu Thr Asp Phe Gly Glu Lys Val Val Ala
                185                 190                 195

Glu Met Asn Arg Leu Gly Met Met Val Asp Leu Ser His Val Ser
                200                 205                 210

Asp Ala Val Ala Arg Arg Ala Leu Glu Val Ser Gln Ala Pro Val
                215                 220                 225

Ile Phe Ser His Ser Ala Ala Arg Gly Val Cys Asn Ser Ala Arg
                230                 235                 240

Asn Val Pro Asp Asp Ile Leu Gln Leu Leu Lys Lys Asn Gly Gly
                245                 250                 255

Val Val Met Val Ser Leu Ser Met Gly Val Ile Gln Cys Asn Pro
                260                 265                 270

Ser Ala Asn Val Ser Thr Val Ala Asp His Phe Asp His Ile Lys
                275                 280                 285

Ala Val Ile Gly Ser Lys Phe Ile Gly Ile Gly Gly Asp Tyr Asp
                290                 295                 300

Gly Ala Gly Lys Phe Pro Gln Gly Leu Glu Asp Val Ser Thr Tyr
                305                 310                 315

Pro Val Leu Ile Glu Glu Leu Leu Ser Arg Gly Trp Ser Glu Glu
                320                 325                 330

Glu Leu Gln Gly Val Leu Arg Gly Asn Leu Leu Arg Val Phe Arg
                335                 340                 345

Gln Val Glu Lys Val Gln Glu Glu Asn Lys Trp Gln Ser Pro Leu
                350                 355                 360

Glu Asp Lys Phe Pro Asp Glu Gln Leu Ser Ser Ser Cys His Ser
                365                 370                 375
```

```
Asp Leu Ser Arg Leu Arg Gln Arg Gln Ser Leu Thr Ser Gly Gln
                380                 385                 390

Glu Leu Thr Glu Ile Pro Ile His Trp Thr Ala Lys Leu Pro Ala
                395                 400                 405

Lys Trp Ser Val Ser Glu Ser Ser Pro His Met Ala Pro Val Leu
                410                 415                 420

Ala Val Val Ala Thr Phe Pro Val Leu Ile Leu Trp Leu
                425                 430
```

<210> 335
<211> 1295
<212> DNA
<213> Homo Sapien

<400> 335

```
cccagaagtt caagggcccc cggcctcctg cgctcctgcc gccgggaccc 50

tcgacctcct cagagcagcc ggctgccgcc ccgggaagat ggcgaggagg 100

agccgccacc gcctcctcct gctgctgctg cgctacctgg tggtcgccct 150

gggctatcat aaggcctatg ggttttctgc cccaaaagac caacaagtag 200

tcacagcagt agagtaccaa gaggctattt tagcctgcaa aaccccaaag 250

aagactgttt cctccagatt agagtggaag aaactgggtc ggagtgtctc 300

ctttgtctac tatcaacaga ctcttcaagg tgattttaaa aatcgagctg 350

agatgataga tttcaatatc cggatcaaaa atgtgacaag aagtgatgcg 400

gggaaatatc gttgtgaagt tagtgcccca tctgagcaag gccaaaacct 450

ggaagaggat acagtcactc tggaagtatt agtggctcca gcagttccat 500

catgtgaagt accctcttct gctctgagtg gaactgtggt agagctacga 550

tgtcaagaca aagaagggaa tccagctcct gaatacacat ggtttaagga 600

tggcatccgt ttgctagaaa atcccagact tggctcccaa agcaccaaca 650

gctcatacac aatgaataca aaaactggaa ctctgcaatt taatactgtt 700

tccaaactgg acactggaga atattcctgt gaagcccgca attctgttgg 750

atatcgcagg tgtcctggga aacgaatgca agtagatgat ctcaacataa 800

gtggcatcat agcagccgta gtagttgtgg ccttagtgat ttccgtttgt 850

ggccttggtg tatgctatgc tcagaggaaa ggctactttt caaaagaaac 900

ctccttccag aagagtaatt cttcatctaa agccacgaca atgagtgaaa 950

atgtgcagtg gctcacgcct gtaatcccag cactttggaa ggccgcggcg 1000

ggcggatcac gaggtcagga gttctagacc agtctggcca atatggtgaa 1050

accccatctc tactaaaata caaaaattag ctgggcatgg tggcatgtgc 1100

ctgcagttcc agctgcttgg gagacaggag aatcacttga acccgggagg 1150
```

cggaggttgc agtgagctga gatcacgcca ctgcagtcca gcctgggtaa 1200

cagagcaaga ttccatctca aaaaataaaa taaataaata aataaatact 1250

ggtttttacc tgtagaattc ttacaataaa tatagcttga tattc 1295

<210> 336
<211> 312
<212> PRT
<213> Homo Sapien

<400> 336

```
Met Ala Arg Arg Ser Arg His Arg Leu Leu Leu Leu Leu Leu Arg
  1               5                  10                  15

Tyr Leu Val Val Ala Leu Gly Tyr His Lys Ala Tyr Gly Phe Ser
                 20                  25                  30

Ala Pro Lys Asp Gln Gln Val Val Thr Ala Val Glu Tyr Gln Glu
                 35                  40                  45

Ala Ile Leu Ala Cys Lys Thr Pro Lys Lys Thr Val Ser Ser Arg
                 50                  55                  60

Leu Glu Trp Lys Lys Leu Gly Arg Ser Val Ser Phe Val Tyr Tyr
                 65                  70                  75

Gln Gln Thr Leu Gln Gly Asp Phe Lys Asn Arg Ala Glu Met Ile
                 80                  85                  90

Asp Phe Asn Ile Arg Ile Lys Asn Val Thr Arg Ser Asp Ala Gly
                 95                 100                 105

Lys Tyr Arg Cys Glu Val Ser Ala Pro Ser Glu Gln Gly Gln Asn
                110                 115                 120

Leu Glu Glu Asp Thr Val Thr Leu Glu Val Leu Val Ala Pro Ala
                125                 130                 135

Val Pro Ser Cys Glu Val Pro Ser Ser Ala Leu Ser Gly Thr Val
                140                 145                 150

Val Glu Leu Arg Cys Gln Asp Lys Glu Gly Asn Pro Ala Pro Glu
                155                 160                 165

Tyr Thr Trp Phe Lys Asp Gly Ile Arg Leu Leu Glu Asn Pro Arg
                170                 175                 180

Leu Gly Ser Gln Ser Thr Asn Ser Ser Tyr Thr Met Asn Thr Lys
                185                 190                 195

Thr Gly Thr Leu Gln Phe Asn Thr Val Ser Lys Leu Asp Thr Gly
                200                 205                 210

Glu Tyr Ser Cys Glu Ala Arg Asn Ser Val Gly Tyr Arg Arg Cys
                215                 220                 225

Pro Gly Lys Arg Met Gln Val Asp Asp Leu Asn Ile Ser Gly Ile
                230                 235                 240

Ile Ala Ala Val Val Val Val Ala Leu Val Ile Ser Val Cys Gly
                245                 250                 255

Leu Gly Val Cys Tyr Ala Gln Arg Lys Gly Tyr Phe Ser Lys Glu
```

260 265 270

Thr Ser Phe Gln Lys Ser Asn Ser Ser Ser Lys Ala Thr Thr Met
275 280 285

Ser Glu Asn Val Gln Trp Leu Thr Pro Val Ile Pro Ala Leu Trp
290 295 300

Lys Ala Ala Ala Gly Gly Ser Arg Gly Gln Glu Phe
305 310

<210> 337
<211> 1813
<212> DNA
<213> Homo Sapien

<400> 337
ggagccgccc tgggtgtcag cggctcggct cccgcgcacg ctccggccgt 50

cgcgcagcct cggcacctgc aggtccgtgc gtcccgcggc tggcgcccct 100

gactccgtcc cggccaggga gggccatgat ttccctcccg gggcccctgg 150

tgaccaactt gctgcggttt ttgttcctgg ggctgagtgc cctcgcgccc 200

ccctcgcggg cccagctgca actgcacttg cccgccaacc ggttgcaggc 250

ggtggaggga ggggaagtgg tgcttccagc gtggtacacc ttgcacgggg 300

aggtgtcttc atcccagcca tgggaggtgc cctttgtgat gtggttcttc 350

aaacagaaag aaaaggagga tcaggtgttg tcctacatca atggggtcac 400

aacaagcaaa cctggagtat ccttggtcta ctccatgccc tcccggaacc 450

tgtccctgcg gctggagggt ctccaggaga aagactctgg cccctacagc 500

tgctccgtga atgtgcaaga caaacaaggc aaatctaggg gccacagcat 550

caaaacctta gaactcaatg tactggttcc tccagctcct ccatcctgcc 600

gtctccaggg tgtgccccat gtgggggcaa acgtgaccct gagctgccag 650

tctccaagga gtaagcccgc tgtccaatac cagtgggatc ggcagcttcc 700

atccttccag actttctttg caccagcatt agatgtcatc cgtgggtctt 750

taagcctcac caacctttcg tcttccatgg ctggagtcta tgtctgcaag 800

gcccacaatg aggtgggcac tgcccaatgt aatgtgacgc tggaagtgag 850

cacagggcct ggagctgcag tggttgctgg agctgttgtg ggtaccctgg 900

ttggactggg gttgctggct gggctggtcc tcttgtacca ccgccggggc 950

aaggccctgg aggagccagc caatgatatc aaggaggatg ccattgctcc 1000

ccggaccctg ccctggccca agagctcaga cacaatctcc aagaatggga 1050

ccctttcctc tgtcacctcc gcacgagccc tccggccacc ccatggccct 1100

cccaggcctg gtgcattgac cccacgccc agtctctcca gccaggccct 1150

gccctcacca agactgccca cgacagatgg ggcccaccct caaccaatat 1200

cccccatccc tggtggggtt tcttcctctg gcttgagccg catgggtgct 1250

gtgcctgtga tggtgcctgc ccagagtcaa gctggctctc tggtatgatg 1300

accccaccac tcattggcta aaggatttgg ggtctctcct tcctataagg 1350

gtcacctcta gcacagaggc ctgagtcatg ggaaagagtc acactcctga 1400

cccttagtac tctgcccccca cctctcttta ctgtgggaaa accatctcag 1450

taagacctaa gtgtccagga gacagaagga gaagaggaag tggatctgga 1500

attgggagga gcctccaccc acccctgact cctccttatg aagccagctg 1550

ctgaaattag ctactcacca agagtgaggg gcagagactt ccagtcactg 1600

agtctcccag gccccttga tctgtacccc acccctatct aacaccaccc 1650

ttggctccca ctccagctcc ctgtattgat ataacctgtc aggctggctt 1700

ggttaggttt tactggggca gaggataggg aatctcttat taaaactaac 1750

atgaaatatg tgttgttttc atttgcaaat ttaaataaag atacataatg 1800

tttgtatgaa aaa 1813

<210> 338
<211> 390
<212> PRT
<213> Homo Sapien

<400> 338
Met Ile Ser Leu Pro Gly Pro Leu Val Thr Asn Leu Leu Arg Phe
1               5                   10                  15

Leu Phe Leu Gly Leu Ser Ala Leu Ala Pro Pro Ser Arg Ala Gln
                20                  25                  30

Leu Gln Leu His Leu Pro Ala Asn Arg Leu Gln Ala Val Glu Gly
                35                  40                  45

Gly Glu Val Val Leu Pro Ala Trp Tyr Thr Leu His Gly Glu Val
                50                  55                  60

Ser Ser Ser Gln Pro Trp Glu Val Pro Phe Val Met Trp Phe Phe
                65                  70                  75

Lys Gln Lys Glu Lys Glu Asp Gln Val Leu Ser Tyr Ile Asn Gly
                80                  85                  90

Val Thr Thr Ser Lys Pro Gly Val Ser Leu Val Tyr Ser Met Pro
                95                  100                 105

Ser Arg Asn Leu Ser Leu Arg Leu Glu Gly Leu Gln Glu Lys Asp
                110                 115                 120

Ser Gly Pro Tyr Ser Cys Ser Val Asn Val Gln Asp Lys Gln Gly
                125                 130                 135

Lys Ser Arg Gly His Ser Ile Lys Thr Leu Glu Leu Asn Val Leu
                140                 145                 150

Val Pro Pro Ala Pro Pro Ser Cys Arg Leu Gln Gly Val Pro His

155 160 165

Val Gly Ala Asn Val Thr Leu Ser Cys Gln Ser Pro Arg Ser Lys
170 175 180

Pro Ala Val Gln Tyr Gln Trp Asp Arg Gln Leu Pro Ser Phe Gln
185 190 195

Thr Phe Phe Ala Pro Ala Leu Asp Val Ile Arg Gly Ser Leu Ser
200 205 210

Leu Thr Asn Leu Ser Ser Ser Met Ala Gly Val Tyr Val Cys Lys
215 220 225

Ala His Asn Glu Val Gly Thr Ala Gln Cys Asn Val Thr Leu Glu
230 235 240

Val Ser Thr Gly Pro Gly Ala Ala Val Val Ala Gly Ala Val Val
245 250 255

Gly Thr Leu Val Gly Leu Gly Leu Leu Ala Gly Leu Val Leu Leu
260 265 270

Tyr His Arg Arg Gly Lys Ala Leu Glu Glu Pro Ala Asn Asp Ile
275 280 285

Lys Glu Asp Ala Ile Ala Pro Arg Thr Leu Pro Trp Pro Lys Ser
290 295 300

Ser Asp Thr Ile Ser Lys Asn Gly Thr Leu Ser Ser Val Thr Ser
305 310 315

Ala Arg Ala Leu Arg Pro Pro His Gly Pro Pro Arg Pro Gly Ala
320 325 330

Leu Thr Pro Thr Pro Ser Leu Ser Ser Gln Ala Leu Pro Ser Pro
335 340 345

Arg Leu Pro Thr Thr Asp Gly Ala His Pro Gln Pro Ile Ser Pro
350 355 360

Ile Pro Gly Gly Val Ser Ser Ser Gly Leu Ser Arg Met Gly Ala
365 370 375

Val Pro Val Met Val Pro Ala Gln Ser Gln Ala Gly Ser Leu Val
380 385 390

<210> 339
<211> 3552
<212> DNA
<213> Homo Sapien

<400> 339
gcgagaacct ttgcacgcgc acaaactacg gggacgattt ctgattgatt 50

tttggcgctt tcgatccacc ctcctccctt ctcatgggac tttggggaca 100

aagcgtcccg accgcctcga gcgctcgagc agggcgctat ccaggagcca 150

ggacagcgtc gggaaccaga ccatggctcc tggaccccaa gatccttaag 200

ttcgtcgtct tcatcgtcgc ggttctgctg ccggtccggg ttgactctgc 250

caccatcccc cggcaggacg aagttcccca gcagacagtg gccccacagc 300

aacagaggcg cagcctcaag gaggaggagt gtccagcagg atctcataga 350
tcagaatata ctggagcctg taacccgtgc acagagggtg tggattacac 400
cattgcttcc aacaatttgc cttcttgcct gctatgtaca gtttgtaaat 450
caggtcaaac aaataaaagt tcctgtacca cgaccagaga caccgtgtgt 500
cagtgtgaaa aaggaagctt ccaggataaa aactcccctg agatgtgccg 550
gacgtgtaga acagggtgtc ccagagggat ggtcaaggtc agtaattgta 600
cgccccggag tgacatcaag tgcaaaaatg aatcagctgc cagttccact 650
gggaaaaccc cagcagcgga ggagacagtg accaccatcc tggggatgct 700
tgcctctccc tatcactacc ttatcatcat agtggtttta gtcatcattt 750
tagctgtggt tgtggttggc ttttcatgtc ggaagaaatt catttcttac 800
ctcaaaggca tctgctcagg tggtggagga ggtcccgaac gtgtgcacag 850
agtccttttc cggcggcgtt catgtccttc acgagttcct ggggcggagg 900
acaatgcccg caacgagacc ctgagtaaca gatacttgca gcccacccag 950
gtctctgagc aggaaatcca aggtcaggag ctggcagagc taacaggtgt 1000
gactgtagag tcgccagagg agccacagcg tctgctggaa caggcagaag 1050
ctgaagggtg tcagaggagg aggctgctgg ttccagtgaa tgacgctgac 1100
tccgctgaca tcagcacctt gctggatgcc tcggcaacac tggaagaagg 1150
acatgcaaag gaaacaattc aggaccaact ggtgggctcc gaaaagctct 1200
tttatgaaga agatgaggca ggctctgcta cgtcctgcct gtgaaagaat 1250
ctcttcagga aaccagagct tccctcattt accttttctc ctacaaaggg 1300
aagcagcctg gaagaaacag tccagtactt gacccatgcc ccaacaaact 1350
ctactatcca atatggggca gcttaccaat ggtcctagaa ctttgttaac 1400
gcacttggag taatttttat gaaatactgc gtgtgataag caaacgggag 1450
aaatttatat cagattcttg gctgcatagt tatacgattg tgtattaagg 1500
gtcgttttag gccacatgcg gtggctcatg cctgtaatcc cagcactttg 1550
ataggctgag gcaggtggat tgcttgagct cgggagtttg agaccagcct 1600
catcaacaca gtgaaactcc atctcaattt aaaaagaaaa aaagtggttt 1650
taggatgtca ttctttgcag ttcttcatca tgagacaagt ctttttttct 1700
gcttcttata ttgcaagctc catctctact ggtgtgtgca tttaatgaca 1750
tctaactaca gatgccgcac agccacaatg ctttgcctta tagtttttta 1800
actttagaac gggattatct tgttattacc tgtattttca gtttcggata 1850
tttttgactt aatgatgaga ttatcaagac gtagccctat gctaagtcat 1900

gagcatatgg acttacgagg gttcgactta gagttttgag ctttaagata 1950
ggattattgg ggcttacccc caccttaatt agagaaacat ttatattgct 2000
tactactgta ggctgtacat ctcttttccg atttttgtat aatgatgtaa 2050
acatggaaaa actttaggaa atgcacttat taggctgttt acatgggttg 2100
cctggataca aatcagcagt caaaaatgac taaaaatata actagtgacg 2150
gagggagaaa tcctccctct gtgggaggca cttactgcat tccagttctc 2200
cctcctgcgc cctgagactg gaccagggtt tgatggctgg cagcttctca 2250
aggggcagct tgtcttactt gttaatttta gaggtatata gccatattta 2300
tttataaata aatatttatt tatttattta taagtagatg tttacatatg 2350
cccaggattt tgaagagcct ggtatctttg ggaagccatg tgtctggttt 2400
gtcgtgctgg gacagtcatg ggactgcatc ttccgacttg tccacagcag 2450
atgaggacag tgagaattaa gttagatccg agactgcgaa gagcttctct 2500
ttcaagcgcc attacagttg aacgttagtg aatcttgagc ctcatttggg 2550
ctcagggcag agcaggtgtt tatctgcccc ggcatctgcc atggcatcaa 2600
gagggaagag tggacggtgc ttgggaatgg tgtgaaatgg ttgccgactc 2650
aggcatggat gggcccctct cgcttctggt ggtctgtgaa ctgagtccct 2700
gggatgcctt ttagggcaga gattcctgag ctgcgtttta gggtacagat 2750
tccctgtttg aggagcttgg cccctctgta agcatctgac tcatctcaga 2800
gatatcaatt cttaaacact gtgacaacgg gatctaaaat ggctgacaca 2850
tttgtccttg tgtcacgttc cattatttta tttaaaaacc tcagtaatcg 2900
ttttagcttc tttccagcaa actcttctcc acagtagccc agtcgtggta 2950
ggataaatta cggatatagt cattctaggg gtttcagtct tttccatctc 3000
aaggcattgt gtgttttgtt ccgggactgg tttggctggg acaaagttag 3050
aactgcctga agttcgcaca ttcagattgt tgtgtccatg gagttttagg 3100
aggggatggc ctttccggtc ttcgcacttc catcctctcc cacttccatc 3150
tggcgtccca caccttgtcc cctgcacttc tggatgacac agggtgctgc 3200
tgcctcctag tctttgcctt tgctgggcct tctgtgcagg agacttggtc 3250
tcaaagctca gagagagcca gtccggtccc agctcctttg tcccttcctc 3300
agaggccttc cttgaagatg catctagact accagcctta tcagtgttta 3350
agcttattcc tttaacataa gcttcctgac aacatgaaat tgttggggtt 3400
ttttggcgtt ggttgatttg tttaggtttt gctttatacc cgggccaaat 3450
agcacataac acctggttat atatgaaata ctcatatgtt tatgaccaaa 3500

ataaatatga aacctcatrt taaaaaaaaa aaaaaaaaaa aaaaaaaaaa 3550

aa 3552

<210> 340
<211> 386
<212> PRT
<213> Homo Sapien

<400> 340
Met Gly Leu Trp Gly Gln Ser Val Pro Thr Ala Ser Ser Ala Arg
1 5 10 15

Ala Gly Arg Tyr Pro Gly Ala Arg Thr Ala Ser Gly Thr Arg Pro
20 25 30

Trp Leu Leu Asp Pro Lys Ile Leu Lys Phe Val Val Phe Ile Val
35 40 45

Ala Val Leu Leu Pro Val Arg Val Asp Ser Ala Thr Ile Pro Arg
50 55 60

Gln Asp Glu Val Pro Gln Gln Thr Val Ala Pro Gln Gln Gln Arg
65 70 75

Arg Ser Leu Lys Glu Glu Glu Cys Pro Ala Gly Ser His Arg Ser
80 85 90

Glu Tyr Thr Gly Ala Cys Asn Pro Cys Thr Glu Gly Val Asp Tyr
95 100 105

Thr Ile Ala Ser Asn Asn Leu Pro Ser Cys Leu Leu Cys Thr Val
110 115 120

Cys Lys Ser Gly Gln Thr Asn Lys Ser Ser Cys Thr Thr Thr Arg
125 130 135

Asp Thr Val Cys Gln Cys Glu Lys Gly Ser Phe Gln Asp Lys Asn
140 145 150

Ser Pro Glu Met Cys Arg Thr Cys Arg Thr Gly Cys Pro Arg Gly
155 160 165

Met Val Lys Val Ser Asn Cys Thr Pro Arg Ser Asp Ile Lys Cys
170 175 180

Lys Asn Glu Ser Ala Ala Ser Ser Thr Gly Lys Thr Pro Ala Ala
185 190 195

Glu Glu Thr Val Thr Thr Ile Leu Gly Met Leu Ala Ser Pro Tyr
200 205 210

His Tyr Leu Ile Ile Ile Val Val Leu Val Ile Ile Leu Ala Val
215 220 225

Val Val Val Gly Phe Ser Cys Arg Lys Lys Phe Ile Ser Tyr Leu
230 235 240

Lys Gly Ile Cys Ser Gly Gly Gly Gly Gly Pro Glu Arg Val His
245 250 255

Arg Val Leu Phe Arg Arg Arg Ser Cys Pro Ser Arg Val Pro Gly
260 265 270

Ala Glu Asp Asn Ala Arg Asn Glu Thr Leu Ser Asn Arg Tyr Leu
275 280 285

Gln Pro Thr Gln Val Ser Glu Gln Glu Ile Gln Gly Gln Glu Leu
290 295 300

Ala Glu Leu Thr Gly Val Thr Val Glu Ser Pro Glu Glu Pro Gln
305 310 315

Arg Leu Leu Glu Gln Ala Glu Ala Glu Gly Cys Gln Arg Arg Arg
320 325 330

Leu Leu Val Pro Val Asn Asp Ala Asp Ser Ala Asp Ile Ser Thr
335 340 345

Leu Leu Asp Ala Ser Ala Thr Leu Glu Glu Gly His Ala Lys Glu
350 355 360

Thr Ile Gln Asp Gln Leu Val Gly Ser Glu Lys Leu Phe Tyr Glu
365 370 375

Glu Asp Glu Ala Gly Ser Ala Thr Ser Cys Leu
380 385

<210> 341
<211> 1252
<212> DNA
<213> Homo Sapien

<400> 341
gcctctgaat tgttgggcag tctggcagtg gagctctccc cggtctgaca 50

gccactccag aggccatgct tcgtttcttg ccagatttgg ctttcagctt 100

cctgttaatt ctggctttgg gccaggcagt ccaatttcaa gaatatgtct 150

ttctccaatt tctgggctta gataaggcgc cttcacccca gaagttccaa 200

cctgtgcctt atatcttgaa gaaaattttc caggatcgcg aggcagcagc 250

gaccactggg gtctcccgag acttatgcta cgtaaaggag ctgggcgtcc 300

gcgggaatgt acttcgcttt ctcccagacc aaggtttctt tctttaccca 350

aagaaaattt cccaagcttc ctcctgcctg cagaagctcc tctactttaa 400

cctgtctgcc atcaaagaaa gggaacagtt gacattggcc cagctgggcc 450

tggacttggg gcccaattct tactataacc tgggaccaga gctggaactg 500

gctctgttcc tggttcagga gcctcatgtg tggggccaga ccacccctaa 550

gccaggtaaa atgtttgtgt tgcggtcagt cccatggcca caaggtgctg 600

ttcacttcaa cctgctggat gtagctaagg attggaatga caaccccccgg 650

aaaaatttcg ggttattcct ggagatactg gtcaaagaag atagagactc 700

aggggtgaat tttcagcctg aagacacctg tgccagacta agatgctccc 750

ttcatgcttc cctgctggtg gtgactctca accctgatca gtgccaccct 800

tctcggaaaa ggagagcagc catccctgtc cccaagcttt cttgtaagaa 850

cctctgccac cgtcaccagc tattcattaa cttccgggac ctgggttggc 900

acaagtggat cattgccccc aaggggttca tggcaaatta ctgccatgga 950

gagtgtccct tctcactgac catctctctc aacagctcca attatgcttt 1000

catgcaagcc ctgatgcatg ccgttgaccc agagatcccc caggctgtgt 1050

gtatccccac caagctgtct cccatttcca tgctctacca ggacaataat 1100

gacaatgtca ttctacgaca ttatgaagac atggtagtcg atgaatgtgg 1150

gtgtgggtag gatgtcagaa atgggaatag aaggagtgtt cttagggtaa 1200

atcttttaat aaaactacct atctggttta tgaccactta gatcgaaatg 1250

tc 1252

<210> 342
<211> 364
<212> PRT
<213> Homo Sapien

<400> 342
Met Leu Arg Phe Leu Pro Asp Leu Ala Phe Ser Phe Leu Leu Ile
1 5 10 15

Leu Ala Leu Gly Gln Ala Val Gln Phe Gln Glu Tyr Val Phe Leu
20 25 30

Gln Phe Leu Gly Leu Asp Lys Ala Pro Ser Pro Gln Lys Phe Gln
35 40 45

Pro Val Pro Tyr Ile Leu Lys Lys Ile Phe Gln Asp Arg Glu Ala
50 55 60

Ala Ala Thr Thr Gly Val Ser Arg Asp Leu Cys Tyr Val Lys Glu
65 70 75

Leu Gly Val Arg Gly Asn Val Leu Arg Phe Leu Pro Asp Gln Gly
80 85 90

Phe Phe Leu Tyr Pro Lys Lys Ile Ser Gln Ala Ser Ser Cys Leu
95 100 105

Gln Lys Leu Leu Tyr Phe Asn Leu Ser Ala Ile Lys Glu Arg Glu
110 115 120

Gln Leu Thr Leu Ala Gln Leu Gly Leu Asp Leu Gly Pro Asn Ser
125 130 135

Tyr Tyr Asn Leu Gly Pro Glu Leu Glu Leu Ala Leu Phe Leu Val
140 145 150

Gln Glu Pro His Val Trp Gly Gln Thr Thr Pro Lys Pro Gly Lys
155 160 165

Met Phe Val Leu Arg Ser Val Pro Trp Pro Gln Gly Ala Val His
170 175 180

Phe Asn Leu Leu Asp Val Ala Lys Asp Trp Asn Asp Asn Pro Arg
185 190 195

Lys Asn Phe Gly Leu Phe Leu Glu Ile Leu Val Lys Glu Asp Arg
200 205 210

Asp Ser Gly Val Asn Phe Gln Pro Glu Asp Thr Cys Ala Arg Leu
215 220 225

Arg Cys Ser Leu His Ala Ser Leu Leu Val Val Thr Leu Asn Pro
230 235 240

Asp Gln Cys His Pro Ser Arg Lys Arg Arg Ala Ala Ile Pro Val
245 250 255

Pro Lys Leu Ser Cys Lys Asn Leu Cys His Arg His Gln Leu Phe
260 265 270

Ile Asn Phe Arg Asp Leu Gly Trp His Lys Trp Ile Ile Ala Pro
275 280 285

Lys Gly Phe Met Ala Asn Tyr Cys His Gly Glu Cys Pro Phe Ser
290 295 300

Leu Thr Ile Ser Leu Asn Ser Ser Asn Tyr Ala Phe Met Gln Ala
305 310 315

Leu Met His Ala Val Asp Pro Glu Ile Pro Gln Ala Val Cys Ile
320 325 330

Pro Thr Lys Leu Ser Pro Ile Ser Met Leu Tyr Gln Asp Asn Asn
335 340 345

Asp Asn Val Ile Leu Arg His Tyr Glu Asp Met Val Val Asp Glu
350 355 360

Cys Gly Cys Gly

<210> 343
<211> 2917
<212> DNA
<213> Homo Sapien

<400> 343
cccacgcgtc cggccttctc tctggacttt gcatttccat tccttttcat 50

tgacaaactg actttttta tttctttttt tccatctctg ggccagcttg 100

ggatcctagg ccgccctggg aagacatttg tgttttacac acataaggat 150

ctgtgtttgg ggtttcttct tcctcccctg acattggcat tgcttagtgg 200

ttgtgtgggg agggagacca cgtgggctca gtgcttgctt gcacttatct 250

gcctaggtac atcgaagtct tttgacctcc atacagtgat tatgcctgtc 300

atcgctggtg gtatcctggc ggccttgctc ctgctgatag ttgtcgtgct 350

ctgtctttac ttcaaaatac acaacgcgct aaaagctgca aaggaacctg 400

aagctgtggc tgtaaaaaat cacaacccag acaaggtgtg gtgggccaag 450

aacagccagg ccaaaaccat tgccacggag tcttgtcctg ccctgcagtg 500

ctgtgaagga tatagaatgt gtgccagttt tgattccctg ccaccttgct 550

gttgcgacat aaatgagggc ctctgagtta ggaaaggctc ccttctcaaa 600
gcagagccct gaagacttca atgatgtcaa tgaggccacc tgtttgtgat 650
gtgcaggcac agaagaaagg cacagctccc catcagtttc atggaaaata 700
actcagtgcc tgctgggaac cagctgctgg agatccctac agagagcttc 750
cactgggggc aacccttcca ggaaggagtt ggggagagag aaccctcact 800
gtggggaatg ctgataaacc agtcacacag ctgctctatt ctcacacaaa 850
tctacccctt gcgtggctgg aactgacgtt tccctggagg tgtccagaaa 900
gctgatgtaa cacagagcct ataaaagctg tcggtcctta aggctgccca 950
gcgccttgcc aaaatggagc ttgtaagaag gctcatgcca ttgaccctct 1000
taattctctc ctgtttggcg gagctgacaa tggcggaggc tgaaggcaat 1050
gcaagctgca cagtcagtct agggggtgcc aatatggcag agacccacaa 1100
agccatgatc ctgcaactca atcccagtga gaactgcacc tggacaatag 1150
aaagaccaga aaacaaaagc atcagaatta tcttttccta tgtccagctt 1200
gatccagatg gaagctgtga aagtgaaaac attaaagtct ttgacggaac 1250
ctccagcaat gggcctctgc tagggcaagt ctgcagtaaa aacgactatg 1300
ttcctgtatt tgaatcatca tccagtacat tgacgtttca aatagttact 1350
gactcagcaa gaattcaaag aactgtcttt gtcttctact acttcttctc 1400
tcctaacatc tctattccaa actgtggcgg ttacctggat accttggaag 1450
gatccttcac cagccccaat tacccaaagc cgcatcctga gctggcttat 1500
tgtgtgtggc acatacaagt ggagaaagat tacaagataa aactaaactt 1550
caaagagatt ttcctagaaa tagacaaaca gtgcaaattt gattttcttg 1600
ccatctatga tggcccctcc accaactctg gcctgattgg acaagtctgt 1650
ggccgtgtga ctcccacctt cgaatcgtca tcaaactctc tgactgtcgt 1700
gttgtctaca gattatgcca attcttaccg gggattttct gcttcctaca 1750
cctcaattta tgcagaaaac atcaacacta catctttaac ttgctcttct 1800
gacaggatga gagttattat aagcaaatcc tacctagagg cttttaactc 1850
taatgggaat aacttgcaac taaaagaccc aacttgcaga ccaaaattat 1900
caaatgttgt ggaattttct gtccctctta atggatgtgg tacaatcaga 1950
aaggtagaag atcagtcaat tacttacacc aatataatca ccttttctgc 2000
atcctcaact tctgaagtga tcacccgtca gaaacaactc cagattattg 2050
tgaagtgtga aatgggacat aattctacag tggagataat atacataaca 2100
gaagatgatg taatacaaag tcaaaatgca ctgggcaaat ataacaccag 2150

catggctctt tttgaatcca attcatttga aaagactata cttgaatcac 2200

catattatgt ggatttgaac caaactcttt ttgttcaagt tagtctgcac 2250

acctcagatc caaatttggt ggtgtttctt gatacctgta gagcctctcc 2300

cacctctgac tttgcatctc caacctacga cctaatcaag agtggatgta 2350

gtcgagatga aacttgtaag gtgtatccct tatttggaca ctatgggaga 2400

ttccagttta atgcctttaa attcttgaga agtatgagct ctgtgtatct 2450

gcagtgtaaa gttttgatat gtgatagcag tgaccaccag tctcgctgca 2500

atcaaggttg tgtctccaga agcaaacgag acatttcttc atataaatgg 2550

aaaacagatt ccatcatagg acccattcgt ctgaaaaggg atcgaagtgc 2600

aagtggcaat tcaggatttc agcatgaaac acatgcggaa gaaactccaa 2650

accagccttt caacagtgtg catctgtttt ccttcatggt tctagctctg 2700

aatgtggtga ctgtagcgac aatcacagtg aggcattttg taaatcaacg 2750

ggcagactac aaataccaga agctgcagaa ctattaacta acaggtccaa 2800

ccctaagtga gacatgtttc tccaggatgc caaaggaaat gctacctcgt 2850

ggctacacat attatgaata aatgaggaag ggcctgaaag tgacacacag 2900

gcctgcatgt aaaaaaa 2917

<210> 344
<211> 607
<212> PRT
<213> Homo Sapien

<400> 344

```
Met Glu Leu Val Arg Arg Leu Met Pro Leu Thr Leu Leu Ile Leu
 1               5                  10                  15

Ser Cys Leu Ala Glu Leu Thr Met Ala Glu Ala Glu Gly Asn Ala
                20                  25                  30

Ser Cys Thr Val Ser Leu Gly Gly Ala Asn Met Ala Glu Thr His
                35                  40                  45

Lys Ala Met Ile Leu Gln Leu Asn Pro Ser Glu Asn Cys Thr Trp
                50                  55                  60

Thr Ile Glu Arg Pro Glu Asn Lys Ser Ile Arg Ile Ile Phe Ser
                65                  70                  75

Tyr Val Gln Leu Asp Pro Asp Gly Ser Cys Glu Ser Glu Asn Ile
                80                  85                  90

Lys Val Phe Asp Gly Thr Ser Ser Asn Gly Pro Leu Leu Gly Gln
                95                 100                 105

Val Cys Ser Lys Asn Asp Tyr Val Pro Val Phe Glu Ser Ser Ser
               110                 115                 120

Ser Thr Leu Thr Phe Gln Ile Val Thr Asp Ser Ala Arg Ile Gln
               125                 130                 135
```

```
Arg Thr Val Phe Val Phe Tyr Tyr Phe Phe Ser Pro Asn Ile Ser
                140                 145                 150

Ile Pro Asn Cys Gly Gly Tyr Leu Asp Thr Leu Glu Gly Ser Phe
                155                 160                 165

Thr Ser Pro Asn Tyr Pro Lys Pro His Pro Glu Leu Ala Tyr Cys
                170                 175                 180

Val Trp His Ile Gln Val Glu Lys Asp Tyr Lys Ile Lys Leu Asn
                185                 190                 195

Phe Lys Glu Ile Phe Leu Glu Ile Asp Lys Gln Cys Lys Phe Asp
                200                 205                 210

Phe Leu Ala Ile Tyr Asp Gly Pro Ser Thr Asn Ser Gly Leu Ile
                215                 220                 225

Gly Gln Val Cys Gly Arg Val Thr Pro Thr Phe Glu Ser Ser Ser
                230                 235                 240

Asn Ser Leu Thr Val Val Leu Ser Thr Asp Tyr Ala Asn Ser Tyr
                245                 250                 255

Arg Gly Phe Ser Ala Ser Tyr Thr Ser Ile Tyr Ala Glu Asn Ile
                260                 265                 270

Asn Thr Thr Ser Leu Thr Cys Ser Ser Asp Arg Met Arg Val Ile
                275                 280                 285

Ile Ser Lys Ser Tyr Leu Glu Ala Phe Asn Ser Asn Gly Asn Asn
                290                 295                 300

Leu Gln Leu Lys Asp Pro Thr Cys Arg Pro Lys Leu Ser Asn Val
                305                 310                 315

Val Glu Phe Ser Val Pro Leu Asn Gly Cys Gly Thr Ile Arg Lys
                320                 325                 330

Val Glu Asp Gln Ser Ile Thr Tyr Thr Asn Ile Ile Thr Phe Ser
                335                 340                 345

Ala Ser Ser Thr Ser Glu Val Ile Thr Arg Gln Lys Gln Leu Gln
                350                 355                 360

Ile Ile Val Lys Cys Glu Met Gly His Asn Ser Thr Val Glu Ile
                365                 370                 375

Ile Tyr Ile Thr Glu Asp Asp Val Ile Gln Ser Gln Asn Ala Leu
                380                 385                 390

Gly Lys Tyr Asn Thr Ser Met Ala Leu Phe Glu Ser Asn Ser Phe
                395                 400                 405

Glu Lys Thr Ile Leu Glu Ser Pro Tyr Tyr Val Asp Leu Asn Gln
                410                 415                 420

Thr Leu Phe Val Gln Val Ser Leu His Thr Ser Asp Pro Asn Leu
                425                 430                 435

Val Val Phe Leu Asp Thr Cys Arg Ala Ser Pro Thr Ser Asp Phe
                440                 445                 450
```

```
Ala Ser Pro Thr Tyr Asp Leu Ile Lys Ser Gly Cys Ser Arg Asp
                455                 460                 465

Glu Thr Cys Lys Val Tyr Pro Leu Phe Gly His Tyr Gly Arg Phe
                470                 475                 480

Gln Phe Asn Ala Phe Lys Phe Leu Arg Ser Met Ser Ser Val Tyr
                485                 490                 495

Leu Gln Cys Lys Val Leu Ile Cys Asp Ser Ser Asp His Gln Ser
                500                 505                 510

Arg Cys Asn Gln Gly Cys Val Ser Arg Ser Lys Arg Asp Ile Ser
                515                 520                 525

Ser Tyr Lys Trp Lys Thr Asp Ser Ile Ile Gly Pro Ile Arg Leu
                530                 535                 540

Lys Arg Asp Arg Ser Ala Ser Gly Asn Ser Gly Phe Gln His Glu
                545                 550                 555

Thr His Ala Glu Glu Thr Pro Asn Gln Pro Phe Asn Ser Val His
                560                 565                 570

Leu Phe Ser Phe Met Val Leu Ala Leu Asn Val Val Thr Val Ala
                575                 580                 585

Thr Ile Thr Val Arg His Phe Val Asn Gln Arg Ala Asp Tyr Lys
                590                 595                 600

Tyr Gln Lys Leu Gln Asn Tyr
                605
```

<210> 345
<211> 2933
<212> DNA
<213> Homo Sapien

<400> 345

```
tgggggcccc ccaggctcgc gcgtggagcg aagcagcatg ggcagtcggt 50

gcgcgctggc cctggcggtg ctctcggcct tgctgtgtca ggtctggagc 100

tctggggtgt tcgaactgaa gctgcaggag ttcgtcaaca agaaggggct 150

gctggggaac cgcaattgct gccgcggggg cgcggggcca ccgccgtgcg 200

cctgccggac cttcttccgc gtgtgcctca agcactacca ggccagcgtg 250

tcccccgagc cgccctgcac ctacggcagc gccgtcaccc ccgtgctggg 300

cgtcgactcc ttcagtctgc ccgacggcgg gggcgccgac tccgcgttca 350

gcaaccccat ccgcttcccc ttcggcttca cctggccggg caccttctct 400

ctgattattg aagctctcca cacagattct cctgatgacc tcgcaacaga 450

aaacccagaa agactcatca gccgcctggc cacccagagg cacctgacgg 500

tgggcgagga gtggtcccag gacctgcaca gcagcggccg cacggacctc 550

aagtactcct accgcttcgt gtgtgacgaa cactactacg gagagggctg 600

ctccgttttc tgccgtcccc gggacgatgc cttcggccac ttcacctgtg 650
```

gggagcgtgg ggagaaagtg tgcaaccctg gctggaaagg gccctactgc 700
acagagccga tctgcctgcc tggatgtgat gagcagcatg gattttgtga 750
caaaccaggg gaatgcaagt gcagagtggg ctggcagggc cggtactgtg 800
acgagtgtat ccgctatcca ggctgtctcc atggcacctg ccagcagccc 850
tggcagtgca actgccagga aggctggggg ggccttttct gcaaccagga 900
cctgaactac tgcacacacc ataagccctg caagaatgga gccacctgca 950
ccaacacggg ccaggggagc tacacttgct cttgccggcc tgggtacaca 1000
ggtgccacct gcgagctggg gattgacgag tgtgacccca gcccttgtaa 1050
gaacggaggg agctgcacgg atctcgagaa cagctactcc tgtacctgcc 1100
cacccggctt ctacggcaaa atctgtgaat tgagtgccat gacctgtgcg 1150
gacggccctt gctttaacgg gggtcggtgc tcagacagcc ccgatggagg 1200
gtacagctgc cgctgccccg tgggctactc cggcttcaac tgtgagaaga 1250
aaattgacta ctgcagctct tcaccctgtt ctaatggtgc caagtgtgtg 1300
gacctcggtg atgcctacct gtgccgctgc caggccggct tctcggggag 1350
gcactgtgac gacaacgtgg acgactgcgc ctcctccccg tgcgccaacg 1400
ggggcacctg ccgggatggc gtgaacgact tctcctgcac ctgcccgcct 1450
ggctacacgg gcaggaactg cagtgccccc gtcagcaggt gcgagcacgc 1500
accctgccac aatggggcca cctgccacga gaggggccac cgctatgtgt 1550
gcgagtgtgc ccgaggctac gggggtccca actgccagtt cctgctcccc 1600
gagctgcccc cgggcccagc ggtggtggac ctcactgaga agctagaggg 1650
ccagggcggg ccattcccct gggtggccgt gtgcgccggg gtcatccttg 1700
tcctcatgct gctgctgggc tgtgccgctg tggtggtctg cgtccggctg 1750
aggctgcaga agcaccggcc cccagccgac ccctgccggg gggagacgga 1800
gaccatgaac aacctggcca actgccagcg tgagaaggac atctcagtca 1850
gcatcatcgg ggccacgcag atcaagaaca ccaacaagaa ggcggacttc 1900
cacggggacc acagcgccga caagaatggc ttcaaggccc gctacccagc 1950
ggtggactat aacctcgtgc aggacctcaa gggtgacgac accgccgtca 2000
gggacgcgca cagcaagcgt gacaccaagt gccagcccca gggctcctca 2050
ggggaggaga aggggacccc gaccacactc aggggtggag aagcatctga 2100
aagaaaaagg ccggactcgg gctgttcaac ttcaaaagac accaagtacc 2150
agtcggtgta cgtcatatcc gaggagaagg atgagtgcgt catagcaact 2200
gaggtgtaaa atggaagtga gatggcaaga ctcccgtttc tcttaaaata 2250

agtaaaattc caaggatata tgccccaacg aatgctgctg aagaggaggg 2300

aggcctcgtg gactgctgct gagaaaccga gttcagaccg agcaggttct 2350

cctcctgagg tcctcgacgc ctgccgacag cctgtcgcgg cccggccgcc 2400

tgcggcactg ccttccgtga cgtcgccgtt gcactatgga cagttgctct 2450

taagagaata tatatttaaa tgggtgaact gaattacgca taagaagcat 2500

gcactgcctg agtgtatatt ttggattctt atgagccagt cttttcttga 2550

attagaaaca caaacactgc ctttattgtc ctttttgata cgaagatgtg 2600

ctttttctag atggaaaaga tgtgtgttat tttttggatt tgtaaaaata 2650

tttttcatga tatctgtaaa gcttgagtat tttgtgatgt tcgtttttta 2700

taatttaaat tttggtaaat atgtacaaag gcacttcggg tctatgtgac 2750

tatatttttt tgtatataaa tgtatttatg gaatattgtg caaatgttat 2800

ttgagttttt tactgttttg ttaatgaaga aattcctttt taaaatattt 2850

ttccaaaata aattttatga atgacaaaaa aaaaaaaaaa aaaaaaaaaa 2900

aaaaaaaaaa aaaaaaaaaa aaaaaaaaaa aaa 2933

<210> 346
<211> 723
<212> PRT
<213> Homo Sapien

<400> 346

```
Met Gly Ser Arg Cys Ala Leu Ala Leu Ala Val Leu Ser Ala Leu
  1               5                  10                  15

Leu Cys Gln Val Trp Ser Ser Gly Val Phe Glu Leu Lys Leu Gln
                 20                  25                  30

Glu Phe Val Asn Lys Lys Gly Leu Leu Gly Asn Arg Asn Cys Cys
                 35                  40                  45

Arg Gly Gly Ala Gly Pro Pro Pro Cys Ala Cys Arg Thr Phe Phe
                 50                  55                  60

Arg Val Cys Leu Lys His Tyr Gln Ala Ser Val Ser Pro Glu Pro
                 65                  70                  75

Pro Cys Thr Tyr Gly Ser Ala Val Thr Pro Val Leu Gly Val Asp
                 80                  85                  90

Ser Phe Ser Leu Pro Asp Gly Gly Gly Ala Asp Ser Ala Phe Ser
                 95                 100                 105

Asn Pro Ile Arg Phe Pro Phe Gly Phe Thr Trp Pro Gly Thr Phe
                110                 115                 120

Ser Leu Ile Ile Glu Ala Leu His Thr Asp Ser Pro Asp Asp Leu
                125                 130                 135

Ala Thr Glu Asn Pro Glu Arg Leu Ile Ser Arg Leu Ala Thr Gln
                140                 145                 150
```

```
Arg His Leu Thr Val Gly Glu Glu Trp Ser Gln Asp Leu His Ser
                155                 160                 165

Ser Gly Arg Thr Asp Leu Lys Tyr Ser Tyr Arg Phe Val Cys Asp
                170                 175                 180

Glu His Tyr Tyr Gly Glu Gly Cys Ser Val Phe Cys Arg Pro Arg
                185                 190                 195

Asp Asp Ala Phe Gly His Phe Thr Cys Gly Glu Arg Gly Glu Lys
                200                 205                 210

Val Cys Asn Pro Gly Trp Lys Gly Pro Tyr Cys Thr Glu Pro Ile
                215                 220                 225

Cys Leu Pro Gly Cys Asp Glu Gln His Gly Phe Cys Asp Lys Pro
                230                 235                 240

Gly Glu Cys Lys Cys Arg Val Gly Trp Gln Gly Arg Tyr Cys Asp
                245                 250                 255

Glu Cys Ile Arg Tyr Pro Gly Cys Leu His Gly Thr Cys Gln Gln
                260                 265                 270

Pro Trp Gln Cys Asn Cys Gln Glu Gly Trp Gly Gly Leu Phe Cys
                275                 280                 285

Asn Gln Asp Leu Asn Tyr Cys Thr His His Lys Pro Cys Lys Asn
                290                 295                 300

Gly Ala Thr Cys Thr Asn Thr Gly Gln Gly Ser Tyr Thr Cys Ser
                305                 310                 315

Cys Arg Pro Gly Tyr Thr Gly Ala Thr Cys Glu Leu Gly Ile Asp
                320                 325                 330

Glu Cys Asp Pro Ser Pro Cys Lys Asn Gly Gly Ser Cys Thr Asp
                335                 340                 345

Leu Glu Asn Ser Tyr Ser Cys Thr Cys Pro Pro Gly Phe Tyr Gly
                350                 355                 360

Lys Ile Cys Glu Leu Ser Ala Met Thr Cys Ala Asp Gly Pro Cys
                365                 370                 375

Phe Asn Gly Gly Arg Cys Ser Asp Ser Pro Asp Gly Gly Tyr Ser
                380                 385                 390

Cys Arg Cys Pro Val Gly Tyr Ser Gly Phe Asn Cys Glu Lys Lys
                395                 400                 405

Ile Asp Tyr Cys Ser Ser Ser Pro Cys Ser Asn Gly Ala Lys Cys
                410                 415                 420

Val Asp Leu Gly Asp Ala Tyr Leu Cys Arg Cys Gln Ala Gly Phe
                425                 430                 435

Ser Gly Arg His Cys Asp Asp Asn Val Asp Asp Cys Ala Ser Ser
                440                 445                 450

Pro Cys Ala Asn Gly Gly Thr Cys Arg Asp Gly Val Asn Asp Phe
                455                 460                 465
```

Ser Cys Thr Cys Pro Pro Gly Tyr Thr Gly Arg Asn Cys Ser Ala
470 475 480

Pro Val Ser Arg Cys Glu His Ala Pro Cys His Asn Gly Ala Thr
485 490 495

Cys His Glu Arg Gly His Arg Tyr Val Cys Glu Cys Ala Arg Gly
500 505 510

Tyr Gly Gly Pro Asn Cys Gln Phe Leu Leu Pro Glu Leu Pro Pro
515 520 525

Gly Pro Ala Val Val Asp Leu Thr Glu Lys Leu Glu Gly Gln Gly
530 535 540

Gly Pro Phe Pro Trp Val Ala Val Cys Ala Gly Val Ile Leu Val
545 550 555

Leu Met Leu Leu Leu Gly Cys Ala Ala Val Val Val Cys Val Arg
560 565 570

Leu Arg Leu Gln Lys His Arg Pro Pro Ala Asp Pro Cys Arg Gly
575 580 585

Glu Thr Glu Thr Met Asn Asn Leu Ala Asn Cys Gln Arg Glu Lys
590 595 600

Asp Ile Ser Val Ser Ile Ile Gly Ala Thr Gln Ile Lys Asn Thr
605 610 615

Asn Lys Lys Ala Asp Phe His Gly Asp His Ser Ala Asp Lys Asn
620 625 630

Gly Phe Lys Ala Arg Tyr Pro Ala Val Asp Tyr Asn Leu Val Gln
635 640 645

Asp Leu Lys Gly Asp Asp Thr Ala Val Arg Asp Ala His Ser Lys
650 655 660

Arg Asp Thr Lys Cys Gln Pro Gln Gly Ser Ser Gly Glu Glu Lys
665 670 675

Gly Thr Pro Thr Thr Leu Arg Gly Gly Glu Ala Ser Glu Arg Lys
680 685 690

Arg Pro Asp Ser Gly Cys Ser Thr Ser Lys Asp Thr Lys Tyr Gln
695 700 705

Ser Val Tyr Val Ile Ser Glu Glu Lys Asp Glu Cys Val Ile Ala
710 715 720

Thr Glu Val

<210> 347
<211> 1685
<212> DNA
<213> Homo Sapien

<400> 347
cccacgcgtc cgcacctcgg ccccgggctc cgaagcggct cgggggcgcc 50

ctttcggtca acatcgtagt ccaccccctc cccatcccca gcccccgggg 100

```
attcaggctc gccagcgccc agccagggag ccggccggga agcgcgatgg 150
gggccccagc cgcctcgctc ctgctcctgc tcctgctgtt cgcctgctgc 200
tgggcgcccg gcggggccaa cctctcccag gacgacagcc agccctggac 250
atctgatgaa acagtggtgg ctggtggcac cgtggtgctc aagtgccaag 300
tgaaagatca cgaggactca tccctgcaat ggtctaaccc tgctcagcag 350
actctctact ttggggagaa gagagccctt cgagataatc gaattcagct 400
ggttacctct acgccccacg agctcagcat cagcatcagc aatgtggccc 450
tggcagacga gggcgagtac acctgctcaa tcttcactat gcctgtgcga 500
actgccaagt ccctcgtcac tgtgctagga attccacaga agcccatcat 550
cactggttat aaatcttcat tacgggaaaa agacacagcc accctaaact 600
gtcagtcttc tgggagcaag cctgcagccc ggctcacctg gagaaagggt 650
gaccaagaac tccacggaga accaacccgc atacaggaag atcccaatgg 700
taaaaccttc actgtcagca gctcggtgac attccaggtt acccgggagg 750
atgatggggc gagcatcgtg tgctctgtga accatgaatc tctaaaggga 800
gctgacagat ccacctctca acgcattgaa gttttataca caccaactgc 850
gatgattagg ccagaccctc cccatcctcg tgagggccag aagctgttgc 900
tacactgtga gggtcgcggc aatccagtcc cccagcagta cctatgggag 950
aaggagggca gtgtgccacc cctgaagatg acccaggaga gtgccctgat 1000
cttccctttc ctcaacaaga gtgacagtgg cacctacggc tgcacagcca 1050
ccagcaacat gggcagctac aaggcctact acaccctcaa tgttaatgac 1100
cccagtccgg tgccctcctc ctccagcacc taccacgcca tcatcggtgg 1150
gatcgtggct ttcattgtct tcctgctgct catcatgctc atcttccttg 1200
gccactactt gatccggcac aaaggaacct acctgacaca tgaggcaaaa 1250
ggctccgacg atgctccaga cgcggacacg gccatcatca atgcagaagg 1300
cgggcagtca ggaggggacg acaagaagga atatttcatc tagaggcgcc 1350
tgcccacttc ctgcgccccc caggggccct gtggggactg ctggggccgt 1400
caccaacccg gacttgtaca gagcaaccgc agggccgccc ctcccgcttg 1450
ctccccagcc cacccacccc cctgtacaga atgtctgctt tgggtgcggt 1500
tttgtactcg gtttggaatg gggagggagg agggcggggg gaggggaggg 1550
ttgccctcag ccctttccgt ggcttctctg catttgggtt attattattt 1600
ttgtaacaat cccaaatcaa atctgtctcc aggctggaga ggcaggagcc 1650
ctggggtgag aaaagcaaaa aacaaacaaa aaaca 1685
```

<210> 348
<211> 398
<212> PRT
<213> Homo Sapien

<400> 348

```
Met Gly Ala Pro Ala Ala Ser Leu Leu Leu Leu Leu Leu Leu Phe
 1               5                  10                  15

Ala Cys Cys Trp Ala Pro Gly Gly Ala Asn Leu Ser Gln Asp Asp
                20                  25                  30

Ser Gln Pro Trp Thr Ser Asp Glu Thr Val Val Ala Gly Gly Thr
                35                  40                  45

Val Val Leu Lys Cys Gln Val Lys Asp His Glu Asp Ser Ser Leu
                50                  55                  60

Gln Trp Ser Asn Pro Ala Gln Gln Thr Leu Tyr Phe Gly Glu Lys
                65                  70                  75

Arg Ala Leu Arg Asp Asn Arg Ile Gln Leu Val Thr Ser Thr Pro
                80                  85                  90

His Glu Leu Ser Ile Ser Ile Ser Asn Val Ala Leu Ala Asp Glu
                95                  100                 105

Gly Glu Tyr Thr Cys Ser Ile Phe Thr Met Pro Val Arg Thr Ala
                110                 115                 120

Lys Ser Leu Val Thr Val Leu Gly Ile Pro Gln Lys Pro Ile Ile
                125                 130                 135

Thr Gly Tyr Lys Ser Ser Leu Arg Glu Lys Asp Thr Ala Thr Leu
                140                 145                 150

Asn Cys Gln Ser Ser Gly Ser Lys Pro Ala Ala Arg Leu Thr Trp
                155                 160                 165

Arg Lys Gly Asp Gln Glu Leu His Gly Glu Pro Thr Arg Ile Gln
                170                 175                 180

Glu Asp Pro Asn Gly Lys Thr Phe Thr Val Ser Ser Ser Val Thr
                185                 190                 195

Phe Gln Val Thr Arg Glu Asp Asp Gly Ala Ser Ile Val Cys Ser
                200                 205                 210

Val Asn His Glu Ser Leu Lys Gly Ala Asp Arg Ser Thr Ser Gln
                215                 220                 225

Arg Ile Glu Val Leu Tyr Thr Pro Thr Ala Met Ile Arg Pro Asp
                230                 235                 240

Pro Pro His Pro Arg Glu Gly Gln Lys Leu Leu Leu His Cys Glu
                245                 250                 255

Gly Arg Gly Asn Pro Val Pro Gln Gln Tyr Leu Trp Glu Lys Glu
                260                 265                 270

Gly Ser Val Pro Pro Leu Lys Met Thr Gln Glu Ser Ala Leu Ile
                275                 280                 285

Phe Pro Phe Leu Asn Lys Ser Asp Ser Gly Thr Tyr Gly Cys Thr
```

```
                290                 295                 300

Ala Thr Ser Asn Met Gly Ser Tyr Lys Ala Tyr Tyr Thr Leu Asn
                305                 310                 315

Val Asn Asp Pro Ser Pro Val Pro Ser Ser Ser Ser Thr Tyr His
                320                 325                 330

Ala Ile Ile Gly Gly Ile Val Ala Phe Ile Val Phe Leu Leu Leu
                335                 340                 345

Ile Met Leu Ile Phe Leu Gly His Tyr Leu Ile Arg His Lys Gly
                350                 355                 360

Thr Tyr Leu Thr His Glu Ala Lys Gly Ser Asp Asp Ala Pro Asp
                365                 370                 375

Ala Asp Thr Ala Ile Ile Asn Ala Glu Gly Gly Gln Ser Gly Gly
                380                 385                 390

Asp Asp Lys Lys Glu Tyr Phe Ile
                395

<210> 349
<211> 2479
<212> DNA
<213> Homo Sapien

<400> 349
acttgccatc acctgttgcc agtgtggaaa aattctccct gttgaatttt 50

ttgcacatgg aggacagcag caaagagggc aacacaggct gataagacca 100

gagacagcag ggagattatt ttaccatacg ccctcaggac gttccctcta 150

gctggagttc tggacttcaa cagaacccca tccagtcatt ttgattttgc 200

tgtttatttt ttttttcttt ttctttttcc caccacattg tattttattt 250

ccgtacttca gaaatgggcc tacagaccac aaagtggccc agccatgggg 300

ctttttttcct gaagtcttgg cttatcattt ccctggggct ctactcacag 350

gtgtccaaac tcctggcctg ccctagtgtg tgccgctgcg acaggaactt 400

tgtctactgt aatgagcgaa gcttgacctc agtgcctctt gggatcccgg 450

agggcgtaac cgtactctac ctccacaaca accaaattaa taatgctgga 500

tttcctgcag aactgcacaa tgtacagtcg gtgcacacgg tctacctgta 550

tggcaaccaa ctggacgaat tccccatgaa ccttcccaag aatgtcagag 600

ttctccattt gcaggaaaac aatattcaga ccatttcacg ggctgctctt 650

gcccagctct tgaagcttga agagctgcac ctggatgaca actccatatc 700

cacagtgggg gtggaagacg gggccttccg ggaggctatt agcctcaaat 750

tgttgttttt gtctaagaat cacctgagca gtgtgcctgt tgggcttcct 800

gtggacttgc aagagctgag agtggatgaa aatcgaattg ctgtcatatc 850

cgacatggcc ttccagaatc tcacgagctt ggagcgtctt attgtggacg 900
```

ggaacctcct gaccaacaag ggtatcgccg agggcacctt cagccatctc 950
accaagctca aggaattttc aattgtacgt aattcgctgt cccaccctcc 1000
tcccgatctc ccaggtacgc atctgatcag gctctatttg caggacaacc 1050
agataaacca cattcctttg acagccttct caaatctgcg taagctggaa 1100
cggctggata tatccaacaa ccaactgcgg atgctgactc aaggggtttt 1150
tgataatctc tccaacctga agcagctcac tgctcggaat aacccttggt 1200
tttgtgactg cagtattaaa tgggtcacag aatggctcaa atatatccct 1250
tcatctctca acgtgcgggg tttcatgtgc caaggtcctg aacaagtccg 1300
ggggatggcc gtcagggaat taaatatgaa tcttttgtcc tgtcccacca 1350
cgacccccgg cctgcctctc ttcaccccag ccccaagtac agcttctccg 1400
accactcagc ctcccaccct ctctattcca aaccctagca gaagctacac 1450
gcctccaact cctaccacat cgaaacttcc cacgattcct gactgggatg 1500
gcagagaaag agtgacccca cctatttctg aacggatcca gctctctatc 1550
cattttgtga atgatacttc cattcaagtc agctggctct ctctcttcac 1600
cgtgatggca tacaaactca catgggtgaa aatgggccac agtttagtag 1650
ggggcatcgt tcaggagcgc atagtcagcg gtgagaagca acacctgagc 1700
ctggttaact tagagccccg atccacctat cggatttgtt tagtgccact 1750
ggatgctttt aactaccgcg cggtagaaga caccatttgt tcagaggcca 1800
ccacccatgc ctcctatctg aacaacggca gcaacacagc gtccagccat 1850
gagcagacga cgtcccacag catgggctcc ccctttctgc tggcgggctt 1900
gatcgggggc gcggtgatat ttgtgctggt ggtcttgctc agcgtctttt 1950
gctggcatat gcacaaaaag gggcgctaca cctcccagaa gtggaaatac 2000
aaccggggcc ggcggaaaga tgattattgc gaggcaggca ccaagaagga 2050
caactccatc ctggagatga cagaaaccag ttttcagatc gtctccttaa 2100
ataacgatca actccttaaa ggagatttca gactgcagcc catttacacc 2150
ccaaatgggg gcattaatta cacagactgc catatcccca acaacatgcg 2200
atactgcaac agcagcgtgc cagacctgga gcactgccat acgtgacagc 2250
cagaggccca gcgttatcaa ggcggacaat tagactcttg agaacacact 2300
cgtgtgtgca cataaagaca cgcagattac atttgataaa tgttacacag 2350
atgcatttgt gcatttgaat actctgtaat ttatacggtg tactatataa 2400
tgggatttaa aaaaagtgct atcttttcta tttcaagtta attacaaaca 2450
gttttgtaac tctttgcttt ttaaatctt 2479

```
<210> 350
<211> 660
<212> PRT
<213> Homo Sapien

<400> 350
Met Gly Leu Gln Thr Thr Lys Trp Pro Ser His Gly Ala Phe Phe
1               5                   10                  15

Leu Lys Ser Trp Leu Ile Ile Ser Leu Gly Leu Tyr Ser Gln Val
                20                  25                  30

Ser Lys Leu Leu Ala Cys Pro Ser Val Cys Arg Cys Asp Arg Asn
                35                  40                  45

Phe Val Tyr Cys Asn Glu Arg Ser Leu Thr Ser Val Pro Leu Gly
                50                  55                  60

Ile Pro Glu Gly Val Thr Val Leu Tyr Leu His Asn Asn Gln Ile
                65                  70                  75

Asn Asn Ala Gly Phe Pro Ala Glu Leu His Asn Val Gln Ser Val
                80                  85                  90

His Thr Val Tyr Leu Tyr Gly Asn Gln Leu Asp Glu Phe Pro Met
                95                  100                 105

Asn Leu Pro Lys Asn Val Arg Val Leu His Leu Gln Glu Asn Asn
                110                 115                 120

Ile Gln Thr Ile Ser Arg Ala Ala Leu Ala Gln Leu Leu Lys Leu
                125                 130                 135

Glu Glu Leu His Leu Asp Asp Asn Ser Ile Ser Thr Val Gly Val
                140                 145                 150

Glu Asp Gly Ala Phe Arg Glu Ala Ile Ser Leu Lys Leu Leu Phe
                155                 160                 165

Leu Ser Lys Asn His Leu Ser Ser Val Pro Val Gly Leu Pro Val
                170                 175                 180

Asp Leu Gln Glu Leu Arg Val Asp Glu Asn Arg Ile Ala Val Ile
                185                 190                 195

Ser Asp Met Ala Phe Gln Asn Leu Thr Ser Leu Glu Arg Leu Ile
                200                 205                 210

Val Asp Gly Asn Leu Leu Thr Asn Lys Gly Ile Ala Glu Gly Thr
                215                 220                 225

Phe Ser His Leu Thr Lys Leu Lys Glu Phe Ser Ile Val Arg Asn
                230                 235                 240

Ser Leu Ser His Pro Pro Pro Asp Leu Pro Gly Thr His Leu Ile
                245                 250                 255

Arg Leu Tyr Leu Gln Asp Asn Gln Ile Asn His Ile Pro Leu Thr
                260                 265                 270

Ala Phe Ser Asn Leu Arg Lys Leu Glu Arg Leu Asp Ile Ser Asn
                275                 280                 285
```

Asn Gln Leu Arg Met Leu Thr Gln Gly Val Phe Asp Asn Leu Ser
290 295 300

Asn Leu Lys Gln Leu Thr Ala Arg Asn Asn Pro Trp Phe Cys Asp
305 310 315

Cys Ser Ile Lys Trp Val Thr Glu Trp Leu Lys Tyr Ile Pro Ser
320 325 330

Ser Leu Asn Val Arg Gly Phe Met Cys Gln Gly Pro Glu Gln Val
335 340 345

Arg Gly Met Ala Val Arg Glu Leu Asn Met Asn Leu Leu Ser Cys
350 355 360

Pro Thr Thr Thr Pro Gly Leu Pro Leu Phe Thr Pro Ala Pro Ser
365 370 375

Thr Ala Ser Pro Thr Thr Gln Pro Pro Thr Leu Ser Ile Pro Asn
380 385 390

Pro Ser Arg Ser Tyr Thr Pro Pro Thr Pro Thr Thr Ser Lys Leu
395 400 405

Pro Thr Ile Pro Asp Trp Asp Gly Arg Glu Arg Val Thr Pro Pro
410 415 420

Ile Ser Glu Arg Ile Gln Leu Ser Ile His Phe Val Asn Asp Thr
425 430 435

Ser Ile Gln Val Ser Trp Leu Ser Leu Phe Thr Val Met Ala Tyr
440 445 450

Lys Leu Thr Trp Val Lys Met Gly His Ser Leu Val Gly Gly Ile
455 460 465

Val Gln Glu Arg Ile Val Ser Gly Glu Lys Gln His Leu Ser Leu
470 475 480

Val Asn Leu Glu Pro Arg Ser Thr Tyr Arg Ile Cys Leu Val Pro
485 490 495

Leu Asp Ala Phe Asn Tyr Arg Ala Val Glu Asp Thr Ile Cys Ser
500 505 510

Glu Ala Thr Thr His Ala Ser Tyr Leu Asn Asn Gly Ser Asn Thr
515 520 525

Ala Ser Ser His Glu Gln Thr Thr Ser His Ser Met Gly Ser Pro
530 535 540

Phe Leu Leu Ala Gly Leu Ile Gly Gly Ala Val Ile Phe Val Leu
545 550 555

Val Val Leu Leu Ser Val Phe Cys Trp His Met His Lys Lys Gly
560 565 570

Arg Tyr Thr Ser Gln Lys Trp Lys Tyr Asn Arg Gly Arg Arg Lys
575 580 585

Asp Asp Tyr Cys Glu Ala Gly Thr Lys Lys Asp Asn Ser Ile Leu
590 595 600

Glu Met Thr Glu Thr Ser Phe Gln Ile Val Ser Leu Asn Asn Asp

605 610 615

Gln Leu Leu Lys Gly Asp Phe Arg Leu Gln Pro Ile Tyr Thr Pro
620 625 630

Asn Gly Gly Ile Asn Tyr Thr Asp Cys His Ile Pro Asn Asn Met
635 640 645

Arg Tyr Cys Asn Ser Ser Val Pro Asp Leu Glu His Cys His Thr
650 655 660

<210> 351
<211> 4053
<212> DNA
<213> Homo Sapien

<400> 351
agccgacgct gctcaagctg caactctgtt gcagttggca gttcttttcg 50

gtttccctcc tgctgtttgg gggcatgaaa gggcttcgcc gccgggagta 100

aaagaaggaa ttgaccgggc agcgcgaggg aggagcgcgc acgcgaccgc 150

gagggcgggc gtgcaccctc ggctggaagt ttgtgccggg ccccgagcgc 200

gcgccggctg ggagcttcgg gtagagacct aggccgctgg accgcgatga 250

gcgcgccgag cctccgtgcg cgcgccgcgg ggttggggct gctgctgtgc 300

gcggtgctgg ggcgcgctgg ccggtccgac agcggcggtc gcggggaact 350

cgggcagccc tctggggtag ccgccgagcg cccatgcccc actacctgcc 400

gctgcctcgg ggacctgctg gactgcagtc gtaagcggct agcgcgtctt 450

cccgagccac tcccgtcctg ggtcgctcgg ctggacttaa gtcacaacag 500

attatctttc atcaaggcaa gttccatgag ccaccttcaa agccttcgag 550

aagtgaaact gaacaacaat gaattggaga ccattccaaa tctgggacca 600

gtctcggcaa atattacact tctctccttg gctggaaaca ggattgttga 650

aatactccct gaacatctga aagagtttca gtcccttgaa actttggacc 700

ttagcagcaa caatatttca gagctccaaa ctgcatttcc agccctacag 750

ctcaaatatc tgtatctcaa cagcaaccga gtcacatcaa tggaacctgg 800

gtattttgac aatttggcca acacactcct tgtgttaaag ctgaacagga 850

accgaatctc agctatccca cccaagatgt ttaaactgcc ccaactgcaa 900

catctcgaat tgaaccgaaa caagattaaa aatgtagatg gactgacatt 950

ccaaggcctt ggtgctctga agtctctgaa aatgcaaaga aatggagtaa 1000

cgaaacttat ggatggagct tttgggggc tgagcaacat ggaaattttg 1050

cagctggacc ataacaacct aacagagatt accaaaggct ggctttacgg 1100

cttgctgatg ctgcaggaac ttcatctcag ccaaaatgcc atcaacagga 1150

tcagccctga tgcctgggag ttctgccaga agctcagtga gctggaccta 1200

actttcaatc acttatcaag gttagatgat tcaagcttcc ttggcctaag 1250
cttactaaat acactgcaca ttgggaacaa cagagtcagc tacattgctg 1300
attgtgcctt ccgggggctt tccagtttaa agactttgga tctgaagaac 1350
aatgaaattt cctggactat tgaagacatg aatggtgctt tctctgggct 1400
tgacaaactg aggcgactga tactccaagg aaatcggatc cgttctatta 1450
ctaaaaaagc cttcactggt ttggatgcat tggagcatct agacctgagt 1500
gacaacgcaa tcatgtcttt acaaggcaat gcattttcac aaatgaagaa 1550
actgcaacaa ttgcatttaa atacatcaag ccttttgtgc gattgccagc 1600
taaaatggct cccacagtgg gtggcggaaa acaactttca gagctttgta 1650
aatgccagtt gtgcccatcc tcagctgcta aaaggaagaa gcatttttgc 1700
tgttagccca gatggctttg tgtgtgatga ttttcccaaa ccccagatca 1750
cggttcagcc agaaacacag tcggcaataa aaggttccaa tttgagtttc 1800
atctgctcag ctgccagcag cagtgattcc ccaatgactt ttgcttggaa 1850
aaaagacaat gaactactgc atgatgctga aatggaaaat tatgcacacc 1900
tccgggccca aggtggcgag gtgatggagt ataccaccat ccttcggctg 1950
cgcgaggtgg aatttgccag tgaggggaaa tatcagtgtg tcatctccaa 2000
tcactttggt tcatcctact ctgtcaaagc caagcttaca gtaaatatgc 2050
ttccctcatt caccaagacc cccatggatc tcaccatccg agctggggcc 2100
atggcacgct tggagtgtgc tgctgtgggg cacccagccc cccagatagc 2150
ctggcagaag gatgggggca cagacttccc agctgcacgg gagagacgca 2200
tgcatgtgat gcccgaggat gacgtgttct ttatcgtgga tgtgaagata 2250
gaggacattg ggtatacag ctgcacagct cagaacagtg caggaagtat 2300
ttcagcaaat gcaactctga ctgtcctaga aacaccatca tttttgcggc 2350
cactgttgga ccgaactgta accaagggag aaacagccgt cctacagtgc 2400
attgctggag gaagccctcc ccctaaactg aactggacca aagatgatag 2450
cccattggtg gtaaccgaga ggcacttttt tgcagcaggc aatcagcttc 2500
tgattattgt ggactcagat gtcagtgatg ctgggaaata cacatgtgag 2550
atgtctaaca cccttggcac tgagagagga aacgtgcgcc tcagtgtgat 2600
ccccactcca acctgcgact cccctcagat gacagcccca tcgttagacg 2650
atgacggatg ggccactgtg ggtgtcgtga tcatagccgt ggtttgctgt 2700
gtggtgggca cgtcactcgt gtgggtggtc atcatatacc acacaaggcg 2750
gaggaatgaa gattgcagca ttaccaacac agatgagacc aacttgccag 2800

cagatattcc tagttatttg tcatctcagg gaacgttagc tgacaggcag 2850

gatgggtacg tgtcttcaga aagtggaagc caccaccagt ttgtcacatc 2900

ttcaggtgct ggatttttct taccacaaca tgacagtagt gggacctgcc 2950

atattgacaa tagcagtgaa gctgatgtgg aagctgccac agatctgttc 3000

ctttgtccgt ttttgggatc cacaggccct atgtatttga agggaaatgt 3050

gtatggctca gatccttttg aaacatatca tacaggttgc agtcctgacc 3100

caagaacagt tttaatggac cactatgagc ccagttacat aaagaaaaag 3150

gagtgctacc catgttctca tccttcagaa gaatcctgcg aacggagctt 3200

cagtaatata tcgtggcctt cacatgtgag gaagctactt aacactagtt 3250

actctcacaa tgaaggacct ggaatgaaaa atctgtgtct aaacaagtcc 3300

tctttagatt ttagtgcaaa tccagagcca gcgtcggttg cctcgagtaa 3350

ttctttcatg ggtacctttg gaaaagctct caggagacct cacctagatg 3400

cctattcaag ctttggacag ccatcagatt gtcagccaag agccttttat 3450

ttgaaagctc attcttcccc agacttggac tctgggtcag aggaagatgg 3500

gaaagaaagg acagattttc aggaagaaaa tcacatttgt acctttaaac 3550

agactttaga aaactacagg actccaaatt ttcagtctta tgacttggac 3600

acatagactg aatgagacca aaggaaaagc ttaacatact acctcaagtg 3650

aacttttatt taaaagagag agaatcttat gtttttaaa tggagttatg 3700

aattttaaaa ggataaaaat gctttattta tacagatgaa ccaaaattac 3750

aaaaagttat gaaaattttt atactgggaa tgatgctcat ataagaatac 3800

ctttttaaac tattttttaa ctttgtttta tgcaaaaaag tatcttacgt 3850

aaattaatga tataaatcat gattatttta tgtattttta taatgccaga 3900

tttcttttta tggaaaatga gttactaaag cattttaaat aatacctgcc 3950

ttgtaccatt ttttaaatag aagttacttc attatatttt gcacattata 4000

tttaataaaa tgtgtcaatt tgaaaaaaaa aaaaaaaaaa aaaaaaaaaa 4050

aaa 4053

<210> 352
<211> 1119
<212> PRT
<213> Homo Sapien

<400> 352
Met Ser Ala Pro Ser Leu Arg Ala Arg Ala Ala Gly Leu Gly Leu
1 5 10 15

Leu Leu Cys Ala Val Leu Gly Arg Ala Gly Arg Ser Asp Ser Gly
20 25 30

```
Gly Arg Gly Glu Leu Gly Gln Pro Ser Gly Val Ala Ala Glu Arg
                35                  40                  45

Pro Cys Pro Thr Thr Cys Arg Cys Leu Gly Asp Leu Leu Asp Cys
                50                  55                  60

Ser Arg Lys Arg Leu Ala Arg Leu Pro Glu Pro Leu Pro Ser Trp
                65                  70                  75

Val Ala Arg Leu Asp Leu Ser His Asn Arg Leu Ser Phe Ile Lys
                80                  85                  90

Ala Ser Ser Met Ser His Leu Gln Ser Leu Arg Glu Val Lys Leu
                95                 100                 105

Asn Asn Asn Glu Leu Glu Thr Ile Pro Asn Leu Gly Pro Val Ser
               110                 115                 120

Ala Asn Ile Thr Leu Leu Ser Leu Ala Gly Asn Arg Ile Val Glu
               125                 130                 135

Ile Leu Pro Glu His Leu Lys Glu Phe Gln Ser Leu Glu Thr Leu
               140                 145                 150

Asp Leu Ser Ser Asn Asn Ile Ser Glu Leu Gln Thr Ala Phe Pro
               155                 160                 165

Ala Leu Gln Leu Lys Tyr Leu Tyr Leu Asn Ser Asn Arg Val Thr
               170                 175                 180

Ser Met Glu Pro Gly Tyr Phe Asp Asn Leu Ala Asn Thr Leu Leu
               185                 190                 195

Val Leu Lys Leu Asn Arg Asn Arg Ile Ser Ala Ile Pro Pro Lys
               200                 205                 210

Met Phe Lys Leu Pro Gln Leu Gln His Leu Glu Leu Asn Arg Asn
               215                 220                 225

Lys Ile Lys Asn Val Asp Gly Leu Thr Phe Gln Gly Leu Gly Ala
               230                 235                 240

Leu Lys Ser Leu Lys Met Gln Arg Asn Gly Val Thr Lys Leu Met
               245                 250                 255

Asp Gly Ala Phe Trp Gly Leu Ser Asn Met Glu Ile Leu Gln Leu
               260                 265                 270

Asp His Asn Asn Leu Thr Glu Ile Thr Lys Gly Trp Leu Tyr Gly
               275                 280                 285

Leu Leu Met Leu Gln Glu Leu His Leu Ser Gln Asn Ala Ile Asn
               290                 295                 300

Arg Ile Ser Pro Asp Ala Trp Glu Phe Cys Gln Lys Leu Ser Glu
               305                 310                 315

Leu Asp Leu Thr Phe Asn His Leu Ser Arg Leu Asp Asp Ser Ser
               320                 325                 330

Phe Leu Gly Leu Ser Leu Leu Asn Thr Leu His Ile Gly Asn Asn
               335                 340                 345
```

Arg Val Ser Tyr Ile Ala Asp Cys Ala Phe Arg Gly Leu Ser Ser
350 355 360

Leu Lys Thr Leu Asp Leu Lys Asn Asn Glu Ile Ser Trp Thr Ile
365 370 375

Glu Asp Met Asn Gly Ala Phe Ser Gly Leu Asp Lys Leu Arg Arg
380 385 390

Leu Ile Leu Gln Gly Asn Arg Ile Arg Ser Ile Thr Lys Lys Ala
395 400 405

Phe Thr Gly Leu Asp Ala Leu Glu His Leu Asp Leu Ser Asp Asn
410 415 420

Ala Ile Met Ser Leu Gln Gly Asn Ala Phe Ser Gln Met Lys Lys
425 430 435

Leu Gln Gln Leu His Leu Asn Thr Ser Ser Leu Leu Cys Asp Cys
440 445 450

Gln Leu Lys Trp Leu Pro Gln Trp Val Ala Glu Asn Asn Phe Gln
455 460 465

Ser Phe Val Asn Ala Ser Cys Ala His Pro Gln Leu Leu Lys Gly
470 475 480

Arg Ser Ile Phe Ala Val Ser Pro Asp Gly Phe Val Cys Asp Asp
485 490 495

Phe Pro Lys Pro Gln Ile Thr Val Gln Pro Glu Thr Gln Ser Ala
500 505 510

Ile Lys Gly Ser Asn Leu Ser Phe Ile Cys Ser Ala Ala Ser Ser
515 520 525

Ser Asp Ser Pro Met Thr Phe Ala Trp Lys Lys Asp Asn Glu Leu
530 535 540

Leu His Asp Ala Glu Met Glu Asn Tyr Ala His Leu Arg Ala Gln
545 550 555

Gly Gly Glu Val Met Glu Tyr Thr Thr Ile Leu Arg Leu Arg Glu
560 565 570

Val Glu Phe Ala Ser Glu Gly Lys Tyr Gln Cys Val Ile Ser Asn
575 580 585

His Phe Gly Ser Ser Tyr Ser Val Lys Ala Lys Leu Thr Val Asn
590 595 600

Met Leu Pro Ser Phe Thr Lys Thr Pro Met Asp Leu Thr Ile Arg
605 610 615

Ala Gly Ala Met Ala Arg Leu Glu Cys Ala Ala Val Gly His Pro
620 625 630

Ala Pro Gln Ile Ala Trp Gln Lys Asp Gly Gly Thr Asp Phe Pro
635 640 645

Ala Ala Arg Glu Arg Arg Met His Val Met Pro Glu Asp Asp Val
650 655 660

Phe Phe Ile Val Asp Val Lys Ile Glu Asp Ile Gly Val Tyr Ser

```
                665                 670                 675

Cys Thr Ala Gln Asn Ser Ala Gly Ser Ile Ser Ala Asn Ala Thr
                680                 685                 690

Leu Thr Val Leu Glu Thr Pro Ser Phe Leu Arg Pro Leu Leu Asp
                695                 700                 705

Arg Thr Val Thr Lys Gly Glu Thr Ala Val Leu Gln Cys Ile Ala
                710                 715                 720

Gly Gly Ser Pro Pro Pro Lys Leu Asn Trp Thr Lys Asp Asp Ser
                725                 730                 735

Pro Leu Val Val Thr Glu Arg His Phe Phe Ala Ala Gly Asn Gln
                740                 745                 750

Leu Leu Ile Ile Val Asp Ser Asp Val Ser Asp Ala Gly Lys Tyr
                755                 760                 765

Thr Cys Glu Met Ser Asn Thr Leu Gly Thr Glu Arg Gly Asn Val
                770                 775                 780

Arg Leu Ser Val Ile Pro Thr Pro Thr Cys Asp Ser Pro Gln Met
                785                 790                 795

Thr Ala Pro Ser Leu Asp Asp Asp Gly Trp Ala Thr Val Gly Val
                800                 805                 810

Val Ile Ile Ala Val Val Cys Cys Val Val Gly Thr Ser Leu Val
                815                 820                 825

Trp Val Val Ile Ile Tyr His Thr Arg Arg Arg Asn Glu Asp Cys
                830                 835                 840

Ser Ile Thr Asn Thr Asp Glu Thr Asn Leu Pro Ala Asp Ile Pro
                845                 850                 855

Ser Tyr Leu Ser Ser Gln Gly Thr Leu Ala Asp Arg Gln Asp Gly
                860                 865                 870

Tyr Val Ser Ser Glu Ser Gly Ser His His Gln Phe Val Thr Ser
                875                 880                 885

Ser Gly Ala Gly Phe Phe Leu Pro Gln His Asp Ser Ser Gly Thr
                890                 895                 900

Cys His Ile Asp Asn Ser Ser Glu Ala Asp Val Glu Ala Ala Thr
                905                 910                 915

Asp Leu Phe Leu Cys Pro Phe Leu Gly Ser Thr Gly Pro Met Tyr
                920                 925                 930

Leu Lys Gly Asn Val Tyr Gly Ser Asp Pro Phe Glu Thr Tyr His
                935                 940                 945

Thr Gly Cys Ser Pro Asp Pro Arg Thr Val Leu Met Asp His Tyr
                950                 955                 960

Glu Pro Ser Tyr Ile Lys Lys Lys Glu Cys Tyr Pro Cys Ser His
                965                 970                 975

Pro Ser Glu Glu Ser Cys Glu Arg Ser Phe Ser Asn Ile Ser Trp
                980                 985                 990
```

Pro Ser His Val Arg Lys Leu Leu Asn Thr Ser Tyr Ser His Asn
995 1000 1005

Glu Gly Pro Gly Met Lys Asn Leu Cys Leu Asn Lys Ser Ser Leu
1010 1015 1020

Asp Phe Ser Ala Asn Pro Glu Pro Ala Ser Val Ala Ser Ser Asn
1025 1030 1035

Ser Phe Met Gly Thr Phe Gly Lys Ala Leu Arg Arg Pro His Leu
1040 1045 1050

Asp Ala Tyr Ser Ser Phe Gly Gln Pro Ser Asp Cys Gln Pro Arg
1055 1060 1065

Ala Phe Tyr Leu Lys Ala His Ser Ser Pro Asp Leu Asp Ser Gly
1070 1075 1080

Ser Glu Glu Asp Gly Lys Glu Arg Thr Asp Phe Gln Glu Glu Asn
1085 1090 1095

His Ile Cys Thr Phe Lys Gln Thr Leu Glu Asn Tyr Arg Thr Pro
1100 1105 1110

Asn Phe Gln Ser Tyr Asp Leu Asp Thr
1115

<210> 353
<211> 2755
<212> DNA
<213> Homo Sapien

<400> 353
ggggggttagg gaggaaggaa tccaccccca cccccccaaa cccttttctt 50

ctcctttcct ggcttcggac attggagcac taaatgaact tgaattgtgt 100

ctgtggcgag caggatggtc gctgttactt tgtgatgaga tcggggatga 150

attgctcgct ttaaaaatgc tgctttggat tctgttgctg gagacgtctc 200

tttgttttgc cgctggaaac gttacagggg acgtttgcaa agagaagatc 250

tgttcctgca atgagataga aggggaccta cacgtagact gtgaaaaaaa 300

gggcttcaca agtctgcagc gtttcactgc cccgacttcc cagttttacc 350

atttatttct gcatggcaat tccctcactc gacttttccc taatgagttc 400

gctaactttt ataatgcggt tagtttgcac atggaaaaca atggcttgca 450

tgaaatcgtt ccgggggctt ttctggggct gcagctggtg aaaaggctgc 500

acatcaacaa caacaagatc aagtcttttc gaaagcagac ttttctgggg 550

ctggacgatc tggaatatct ccaggctgat tttaatttat tacgagatat 600

agacccgggg gccttccagg acttgaacaa gctggaggtg ctcattttaa 650

atgacaatct catcagcacc ctacctgcca acgtgttcca gtatgtgccc 700

atcacccacc tcgacctccg gggtaacagg ctgaaaacgc tgccctatga 750

ggaggtcttg gagcaaatcc ctggtattgc ggagatcctg ctagaggata 800
acccttggga ctgcacctgt gatctgctct ccctgaaaga atggctggaa 850
aacattccca agaatgccct gatcggccga gtggtctgcg aagcccccac 900
cagactgcag ggtaaagacc tcaatgaaac caccgaacag gacttgtgtc 950
ctttgaaaaa ccgagtggat tctagtctcc cggcgccccc tgcccaagaa 1000
gagacctttg ctcctggacc cctgccaact cctttcaaga caaatgggca 1050
agaggatcat gccacaccag ggtctgctcc aaacggaggt acaaagatcc 1100
caggcaactg gcagatcaaa atcagaccca cagcagcgat agcgacgggt 1150
agctccagga acaaaccctt agctaacagt ttaccctgcc ctgggggctg 1200
cagctgcgac cacatcccag ggtcgggttt aaagatgaac tgcaacaaca 1250
ggaacgtgag cagcttggct gatttgaagc ccaagctctc taacgtgcag 1300
gagcttttcc tacgagataa caagatccac agcatccgaa aatcgcactt 1350
tgtggattac aagaacctca ttctgttgga tctgggcaac aataacatcg 1400
ctactgtaga gaacaacact ttcaagaacc ttttggacct caggtggcta 1450
tacatggata gcaattacct ggacacgctg tcccgggaga aattcgcggg 1500
gctgcaaaac ctagagtacc tgaacgtgga gtacaacgct atccagctca 1550
tcctcccggg cactttcaat gccatgccca aactgaggat cctcattctc 1600
aacaacaacc tgctgaggtc cctgcctgtg gacgtgttcg ctggggtctc 1650
gctctctaaa ctcagcctgc acaacaatta cttcatgtac ctcccggtgg 1700
caggggtgct ggaccagtta acctccatca tccagataga cctccacgga 1750
aacccctggg agtgctcctg cacaattgtg cctttcaagc agtgggcaga 1800
acgcttgggt tccgaagtgc tgatgagcga cctcaagtgt gagacgccgg 1850
tgaacttctt tagaaaggat ttcatgctcc tctccaatga cgagatctgc 1900
cctcagctgt acgctaggat ctcgcccacg ttaacttcgc acagtaaaaa 1950
cagcactggg ttggcggaga ccgggacgca ctccaactcc tacctagaca 2000
ccagcagggt gtccatctcg gtgttggtcc cgggactgct gctggtgttt 2050
gtcacctccg ccttcaccgt ggtgggcatg ctcgtgttta tcctgaggaa 2100
ccgaaagcgg tccaagagac gagatgccaa ctcctccgcg tccgagatta 2150
attccctaca gacagtctgt gactcttcct actggcacaa tgggccttac 2200
aacgcagatg gggcccacag agtgtatgac tgtggctctc actcgctctc 2250
agactaagac cccaacccca ataggggagg gcagagggaa ggcgatacat 2300
ccttccccac cgcaggcacc ccgggggctg gaggggcgtg tacccaaatc 2350

cccgcgccat cagcctggat gggcataagt agataaataa ctgtgagctc 2400

gcacaaccga aagggcctga ccccttactt agctccctcc ttgaaacaaa 2450

gagcagactg tggagagctg ggagagcgca gccagctcgc tctttgctga 2500

gagccccttt tgacagaaag cccagcacga ccctgctgga agaactgaca 2550

gtgccctcgc cctcggcccc ggggcctgtg gggttggatg ccgcggttct 2600

atacatatat acatatatcc acatctatat agagagatag atatctattt 2650

ttcccctgtg gattagcccc gtgatggctc cctgttggct acgcagggat 2700

gggcagttgc acgaaggcat gaatgtattg taaataagta actttgactt 2750

ctgac 2755

<210> 354
<211> 696
<212> PRT
<213> Homo Sapien

<400> 354
Met Leu Leu Trp Ile Leu Leu Leu Glu Thr Ser Leu Cys Phe Ala
1               5                   10                  15

Ala Gly Asn Val Thr Gly Asp Val Cys Lys Glu Lys Ile Cys Ser
                20                  25                  30

Cys Asn Glu Ile Glu Gly Asp Leu His Val Asp Cys Glu Lys Lys
                35                  40                  45

Gly Phe Thr Ser Leu Gln Arg Phe Thr Ala Pro Thr Ser Gln Phe
                50                  55                  60

Tyr His Leu Phe Leu His Gly Asn Ser Leu Thr Arg Leu Phe Pro
                65                  70                  75

Asn Glu Phe Ala Asn Phe Tyr Asn Ala Val Ser Leu His Met Glu
                80                  85                  90

Asn Asn Gly Leu His Glu Ile Val Pro Gly Ala Phe Leu Gly Leu
                95                  100                 105

Gln Leu Val Lys Arg Leu His Ile Asn Asn Asn Lys Ile Lys Ser
                110                 115                 120

Phe Arg Lys Gln Thr Phe Leu Gly Leu Asp Asp Leu Glu Tyr Leu
                125                 130                 135

Gln Ala Asp Phe Asn Leu Leu Arg Asp Ile Asp Pro Gly Ala Phe
                140                 145                 150

Gln Asp Leu Asn Lys Leu Glu Val Leu Ile Leu Asn Asp Asn Leu
                155                 160                 165

Ile Ser Thr Leu Pro Ala Asn Val Phe Gln Tyr Val Pro Ile Thr
                170                 175                 180

His Leu Asp Leu Arg Gly Asn Arg Leu Lys Thr Leu Pro Tyr Glu
                185                 190                 195

Glu Val Leu Glu Gln Ile Pro Gly Ile Ala Glu Ile Leu Leu Glu

200 205 210

Asp Asn Pro Trp Asp Cys Thr Cys Asp Leu Leu Ser Leu Lys Glu
215 220 225

Trp Leu Glu Asn Ile Pro Lys Asn Ala Leu Ile Gly Arg Val Val
230 235 240

Cys Glu Ala Pro Thr Arg Leu Gln Gly Lys Asp Leu Asn Glu Thr
245 250 255

Thr Glu Gln Asp Leu Cys Pro Leu Lys Asn Arg Val Asp Ser Ser
260 265 270

Leu Pro Ala Pro Pro Ala Gln Glu Glu Thr Phe Ala Pro Gly Pro
275 280 285

Leu Pro Thr Pro Phe Lys Thr Asn Gly Gln Glu Asp His Ala Thr
290 295 300

Pro Gly Ser Ala Pro Asn Gly Gly Thr Lys Ile Pro Gly Asn Trp
305 310 315

Gln Ile Lys Ile Arg Pro Thr Ala Ala Ile Ala Thr Gly Ser Ser
320 325 330

Arg Asn Lys Pro Leu Ala Asn Ser Leu Pro Cys Pro Gly Gly Cys
335 340 345

Ser Cys Asp His Ile Pro Gly Ser Gly Leu Lys Met Asn Cys Asn
350 355 360

Asn Arg Asn Val Ser Ser Leu Ala Asp Leu Lys Pro Lys Leu Ser
365 370 375

Asn Val Gln Glu Leu Phe Leu Arg Asp Asn Lys Ile His Ser Ile
380 385 390

Arg Lys Ser His Phe Val Asp Tyr Lys Asn Leu Ile Leu Leu Asp
395 400 405

Leu Gly Asn Asn Asn Ile Ala Thr Val Glu Asn Asn Thr Phe Lys
410 415 420

Asn Leu Leu Asp Leu Arg Trp Leu Tyr Met Asp Ser Asn Tyr Leu
425 430 435

Asp Thr Leu Ser Arg Glu Lys Phe Ala Gly Leu Gln Asn Leu Glu
440 445 450

Tyr Leu Asn Val Glu Tyr Asn Ala Ile Gln Leu Ile Leu Pro Gly
455 460 465

Thr Phe Asn Ala Met Pro Lys Leu Arg Ile Leu Ile Leu Asn Asn
470 475 480

Asn Leu Leu Arg Ser Leu Pro Val Asp Val Phe Ala Gly Val Ser
485 490 495

Leu Ser Lys Leu Ser Leu His Asn Asn Tyr Phe Met Tyr Leu Pro
500 505 510

Val Ala Gly Val Leu Asp Gln Leu Thr Ser Ile Ile Gln Ile Asp
515 520 525

```
Leu His Gly Asn Pro Trp Glu Cys Ser Cys Thr Ile Val Pro Phe
                530                 535                 540

Lys Gln Trp Ala Glu Arg Leu Gly Ser Glu Val Leu Met Ser Asp
                545                 550                 555

Leu Lys Cys Glu Thr Pro Val Asn Phe Phe Arg Lys Asp Phe Met
                560                 565                 570

Leu Leu Ser Asn Asp Glu Ile Cys Pro Gln Leu Tyr Ala Arg Ile
                575                 580                 585

Ser Pro Thr Leu Thr Ser His Ser Lys Asn Ser Thr Gly Leu Ala
                590                 595                 600

Glu Thr Gly Thr His Ser Asn Ser Tyr Leu Asp Thr Ser Arg Val
                605                 610                 615

Ser Ile Ser Val Leu Val Pro Gly Leu Leu Leu Val Phe Val Thr
                620                 625                 630

Ser Ala Phe Thr Val Val Gly Met Leu Val Phe Ile Leu Arg Asn
                635                 640                 645

Arg Lys Arg Ser Lys Arg Arg Asp Ala Asn Ser Ser Ala Ser Glu
                650                 655                 660

Ile Asn Ser Leu Gln Thr Val Cys Asp Ser Ser Tyr Trp His Asn
                665                 670                 675

Gly Pro Tyr Asn Ala Asp Gly Ala His Arg Val Tyr Asp Cys Gly
                680                 685                 690

Ser His Ser Leu Ser Asp
                695
```

<210> 355
<211> 2226
<212> DNA
<213> Homo Sapien

<400> 355

```
agtcgactgc gtcccctgta cccggcgcca gctgtgttcc tgaccccaga 50

ataactcagg gctgcaccgg gcctggcagc gctccgcaca catttcctgt 100

cgcggcctaa gggaaactgt tggccgctgg gcccgcgggg ggattcttgg 150

cagttggggg gtccgtcggg agcgagggcg gaggggaagg gagggggaac 200

cgggttgggg aagccagctg tagagggcgg tgaccgcgct ccagacacag 250

ctctgcgtcc tcgagcggga cagatccaag ttgggagcag ctctgcgtgc 300

ggggcctcag agaatgaggc cggcgttcgc cctgtgcctc ctctggcagg 350

cgctctggcc cgggccgggc ggcggcgaac accccactgc cgaccgtgct 400

ggctgctcgg cctcgggggc ctgctacagc ctgcaccacg ctaccatgaa 450

gcggcaggcg gccgaggagg cctgcatcct gcgaggtggg gcgctcagca 500

ccgtgcgtgc gggcgccgag ctgcgcgctg tgctcgcgct cctgcgggca 550
```

ggcccagggc ccggaggggg ctccaaagac ctgctgttct gggtcgcact 600

ggagcgcagg cgttcccact gcaccctgga gaacgagcct ttgcggggtt 650

tctcctggct gtcctccgac cccggcggtc tcgaaagcga cacgctgcag 700

tgggtggagg agccccaacg ctcctgcacc gcgcggagat gcgcggtact 750

ccaggccacc ggtggggtcg agcccgcagg ctggaaggag atgcgatgcc 800

acctgcgcgc caacggctac ctgtgcaagt accagtttga ggtcttgtgt 850

cctgcgccgc gccccggggc cgcctctaac ttgagctatc gcgcgccctt 900

ccagctgcac agcgccgctc tggacttcag tccacctggg accgaggtga 950

gtgcgctctg ccggggacag ctcccgatct cagttacttg catcgcggac 1000

gaaatcggcg ctcgctggga caaactctcg ggcgatgtgt tgtgtccctg 1050

ccccgggagg tacctccgtg ctggcaaatg cgcagagctc cctaactgcc 1100

tagacgactt gggaggcttt gcctgcgaat gtgctacggg cttcgagctg 1150

gggaaggacg gccgctcttg tgtgaccagt ggggaaggac agccgaccct 1200

tggggggacc ggggtgccca ccaggcgccc gccggccact gcaaccagcc 1250

ccgtgccgca gagaacatgg ccaatcaggg tcgacgagaa gctgggagag 1300

acaccacttg tccctgaaca agacaattca gtaacatcta ttcctgagat 1350

tcctcgatgg ggatcacaga gcacgatgtc tacccttcaa atgtcccttc 1400

aagccgagtc aaaggccact atcaccccat cagggagcgt gatttccaag 1450

tttaattcta cgacttcctc tgccactcct caggctttcg actcctcctc 1500

tgccgtggtc ttcatatttg tgagcacagc agtagtagtg ttggtgatct 1550

tgaccatgac agtactgggg cttgtcaagc tctgctttca cgaaagcccc 1600

tcttcccagc caaggaagga gtctatgggc ccgccgggcc tggagagtga 1650

tcctgagccc gctgctttgg gctccagttc tgcacattgc acaaacaatg 1700

gggtgaaagt cggggactgt gatctgcggg acagagcaga gggtgccttg 1750

ctggcggagt cccctcttgg ctctagtgat gcatagggaa acaggggaca 1800

tgggcactcc tgtgaacagt tttcactttt tgatgaaacg gggaaccaag 1850

aggaacttac ttgtgtaact gacaatttct gcagaaatcc cccttcctct 1900

aaattccctt tactccactg aggagctaaa tcagaactgc acactccttc 1950

cctgatgata gaggaagtgg aagtgccttt aggatggtga tactggggga 2000

ccgggtagtg ctggggagag atattttctt atgtttattc ggagaatttg 2050

gagaagtgat tgaacttttc aagacattgg aaacaaatag aacacaatat 2100

aatttacatt aaaaaataat ttctaccaaa atggaaagga aatgttctat 2150

gttgttcagg ctaggagtat attggttcga aatcccaggg aaaaaaataa 2200

aaataaaaaa ttaaaggatt gttgat 2226

<210> 356
<211> 490
<212> PRT
<213> Homo Sapien

<400> 356
Met Arg Pro Ala Phe Ala Leu Cys Leu Leu Trp Gln Ala Leu Trp
1 5 10 15

Pro Gly Pro Gly Gly Gly Glu His Pro Thr Ala Asp Arg Ala Gly
20 25 30

Cys Ser Ala Ser Gly Ala Cys Tyr Ser Leu His His Ala Thr Met
35 40 45

Lys Arg Gln Ala Ala Glu Glu Ala Cys Ile Leu Arg Gly Gly Ala
50 55 60

Leu Ser Thr Val Arg Ala Gly Ala Glu Leu Arg Ala Val Leu Ala
65 70 75

Leu Leu Arg Ala Gly Pro Gly Pro Gly Gly Gly Ser Lys Asp Leu
80 85 90

Leu Phe Trp Val Ala Leu Glu Arg Arg Arg Ser His Cys Thr Leu
95 100 105

Glu Asn Glu Pro Leu Arg Gly Phe Ser Trp Leu Ser Ser Asp Pro
110 115 120

Gly Gly Leu Glu Ser Asp Thr Leu Gln Trp Val Glu Glu Pro Gln
125 130 135

Arg Ser Cys Thr Ala Arg Arg Cys Ala Val Leu Gln Ala Thr Gly
140 145 150

Gly Val Glu Pro Ala Gly Trp Lys Glu Met Arg Cys His Leu Arg
155 160 165

Ala Asn Gly Tyr Leu Cys Lys Tyr Gln Phe Glu Val Leu Cys Pro
170 175 180

Ala Pro Arg Pro Gly Ala Ala Ser Asn Leu Ser Tyr Arg Ala Pro
185 190 195

Phe Gln Leu His Ser Ala Ala Leu Asp Phe Ser Pro Pro Gly Thr
200 205 210

Glu Val Ser Ala Leu Cys Arg Gly Gln Leu Pro Ile Ser Val Thr
215 220 225

Cys Ile Ala Asp Glu Ile Gly Ala Arg Trp Asp Lys Leu Ser Gly
230 235 240

Asp Val Leu Cys Pro Cys Pro Gly Arg Tyr Leu Arg Ala Gly Lys
245 250 255

Cys Ala Glu Leu Pro Asn Cys Leu Asp Asp Leu Gly Gly Phe Ala
260 265 270

Cys Glu Cys Ala Thr Gly Phe Glu Leu Gly Lys Asp Gly Arg Ser
275 280 285

Cys Val Thr Ser Gly Glu Gly Gln Pro Thr Leu Gly Gly Thr Gly
290 295 300

Val Pro Thr Arg Arg Pro Pro Ala Thr Ala Thr Ser Pro Val Pro
305 310 315

Gln Arg Thr Trp Pro Ile Arg Val Asp Glu Lys Leu Gly Glu Thr
320 325 330

Pro Leu Val Pro Glu Gln Asp Asn Ser Val Thr Ser Ile Pro Glu
335 340 345

Ile Pro Arg Trp Gly Ser Gln Ser Thr Met Ser Thr Leu Gln Met
350 355 360

Ser Leu Gln Ala Glu Ser Lys Ala Thr Ile Thr Pro Ser Gly Ser
365 370 375

Val Ile Ser Lys Phe Asn Ser Thr Thr Ser Ser Ala Thr Pro Gln
380 385 390

Ala Phe Asp Ser Ser Ser Ala Val Val Phe Ile Phe Val Ser Thr
395 400 405

Ala Val Val Val Leu Val Ile Leu Thr Met Thr Val Leu Gly Leu
410 415 420

Val Lys Leu Cys Phe His Glu Ser Pro Ser Ser Gln Pro Arg Lys
425 430 435

Glu Ser Met Gly Pro Pro Gly Leu Glu Ser Asp Pro Glu Pro Ala
440 445 450

Ala Leu Gly Ser Ser Ser Ala His Cys Thr Asn Asn Gly Val Lys
455 460 465

Val Gly Asp Cys Asp Leu Arg Asp Arg Ala Glu Gly Ala Leu Leu
470 475 480

Ala Glu Ser Pro Leu Gly Ser Ser Asp Ala
485 490

<210> 357
<211> 3283
<212> DNA
<213> Homo Sapien

<400> 357
cccatctcaa gctgatcttg gcacctctca tgctctgctc tcttcaacca 50

gacctctaca ttccattttg gaagaagact aaaaatggtg tttccaatgt 100

ggacactgaa gagacaaatt cttatccttt ttaacataat cctaatttcc 150

aaactccttg gggctagatg gtttcctaaa actctgccct gtgatgtcac 200

tctggatgtt ccaaagaacc atgtgatcgt ggactgcaca gacaagcatt 250

tgacagaaat tcctggaggt attcccacga acaccacgaa cctcaccctc 300

accattaacc acataccaga catctcccca gcgtcctttc acagactgga 350
ccatctggta gagatcgatt tcagatgcaa ctgtgtacct attccactgg 400
ggtcaaaaaa caacatgtgc atcaagaggc tgcagattaa acccagaagc 450
tttagtggac tcacttattt aaaatccctt tacctggatg gaaaccagct 500
actagagata ccgcagggcc tcccgcctag cttacagctt ctcagccttg 550
aggccaacaa catcttttcc atcagaaaag agaatctaac agaactggcc 600
aacatagaaa tactctacct gggccaaaac tgttattatc gaaatccttg 650
ttatgtttca tattcaatag agaaagatgc cttcctaaac ttgacaaagt 700
taaaagtgct ctccctgaaa gataacaatg tcacagccgt ccctactgtt 750
ttgccatcta ctttaacaga actatatctc tacaacaaca tgattgcaaa 800
aatccaagaa gatgatttta ataacctcaa ccaattacaa attcttgacc 850
taagtggaaa ttgccctcgt tgttataatg ccccatttcc ttgtgcgccg 900
tgtaaaaata attctcccct acagatccct gtaaatgctt ttgatgcgct 950
gacagaatta aaagttttac gtctacacag taactctctt cagcatgtgc 1000
ccccaagatg gtttaagaac atcaacaaac tccaggaact ggatctgtcc 1050
caaaacttct tggccaaaga aattggggat gctaaatttc tgcattttct 1100
ccccagcctc atccaattgg atctgtcttt caattttgaa cttcaggtct 1150
atcgtgcatc tatgaatcta tcacaagcat tttcttcact gaaaagcctg 1200
aaaattctgc ggatcagagg atatgtcttt aaagagttga aaagctttaa 1250
cctctcgcca ttacataatc ttcaaaatct tgaagttctt gatcttggca 1300
ctaactttat aaaaattgct aacctcagca tgtttaaaca atttaaaaga 1350
ctgaaagtca tagatctttc agtgaataaa atatcacctt caggagattc 1400
aagtgaagtt ggcttctgct caaatgccag aacttctgta gaaagttatg 1450
aaccccaggt cctggaacaa ttacattatt tcagatatga taagtatgca 1500
aggagttgca gattcaaaaa caaagaggct tctttcatgt ctgttaatga 1550
aagctgctac aagtatgggc agaccttgga tctaagtaaa aatagtatat 1600
tttttgtcaa gtcctctgat tttcagcatc tttctttcct caaatgcctg 1650
aatctgtcag gaaatctcat tagccaaact cttaatggca gtgaattcca 1700
acctttagca gagctgagat atttggactt ctccaacaac cggcttgatt 1750
tactccattc aacagcattt gaagagcttc acaaactgga agttctggat 1800
ataagcagta atagccatta ttttcaatca gaaggaatta ctcatatgct 1850
aaactttacc aagaacctaa aggttctgca gaaactgatg atgaacgaca 1900

atgacatctc ttcctccacc agcaggacca tggagagtga gtctcttaga 1950
actctggaat tcagaggaaa tcacttagat gttttatgga gagaaggtga 2000
taacagatac ttacaattat tcaagaatct gctaaaatta gaggaattag 2050
acatctctaa aaattcccta agtttcttgc cttctggagt ttttgatggt 2100
atgcctccaa atctaaagaa tctctctttg gccaaaaatg ggctcaaatc 2150
tttcagttgg aagaaactcc agtgtctaaa gaacctggaa actttggacc 2200
tcagccacaa ccaactgacc actgtccctg agagattatc caactgttcc 2250
agaagcctca agaatctgat tcttaagaat aatcaaatca ggagtctgac 2300
gaagtatttt ctacaagatg ccttccagtt gcgatatctg gatctcagct 2350
caaataaaat ccagatgatc caaaagacca gcttcccaga aaatgtcctc 2400
aacaatctga agatgttgct tttgcatcat aatcggtttc tgtgcacctg 2450
tgatgctgtg tggtttgtct ggtgggttaa ccatacggag gtgactattc 2500
cttacctggc cacagatgtg acttgtgtgg ggccaggagc acacaagggc 2550
caaagtgtga tctccctgga tctgtacacc tgtgagttag atctgactaa 2600
cctgattctg ttctcacttt ccatatctgt atctctcttt ctcatggtga 2650
tgatgacagc aagtcacctc tatttctggg atgtgtggta tatttaccat 2700
ttctgtaagg ccaagataaa ggggtatcag cgtctaatat caccagactg 2750
ttgctatgat gcttttattg tgtatgacac taaagaccca gctgtgaccg 2800
agtgggtttt ggctgagctg gtggccaaac tggaagaccc aagagagaaa 2850
cattttaatt tatgtctcga ggaaagggac tggttaccag ggcagccagt 2900
tctggaaaac ctttcccaga gcatacagct tagcaaaaag acagtgtttg 2950
tgatgacaga caagtatgca aagactgaaa attttaagat agcattttac 3000
ttgtcccatc agaggctcat ggatgaaaaa gttgatgtga ttatcttgat 3050
atttcttgag aagccctttc agaagtccaa gttcctccag ctccggaaaa 3100
ggctctgtgg gagttctgtc cttgagtggc caacaaaccc gcaagctcac 3150
ccatacttct ggcagtgtct aaagaacgcc ctggccacag acaatcatgt 3200
ggcctatagt caggtgttca aggaaacggt ctagcccttc tttgcaaaac 3250
acaactgcct agtttaccaa ggagaggcct ggc 3283

<210> 358
<211> 1049
<212> PRT
<213> Homo Sapien

<400> 358
Met Val Phe Pro Met Trp Thr Leu Lys Arg Gln Ile Leu Ile Leu
1               5                   10                  15

```
Phe Asn Ile Ile Leu Ile Ser Lys Leu Leu Gly Ala Arg Trp Phe
                20                  25                  30

Pro Lys Thr Leu Pro Cys Asp Val Thr Leu Asp Val Pro Lys Asn
                35                  40                  45

His Val Ile Val Asp Cys Thr Asp Lys His Leu Thr Glu Ile Pro
                50                  55                  60

Gly Gly Ile Pro Thr Asn Thr Thr Asn Leu Thr Leu Thr Ile Asn
                65                  70                  75

His Ile Pro Asp Ile Ser Pro Ala Ser Phe His Arg Leu Asp His
                80                  85                  90

Leu Val Glu Ile Asp Phe Arg Cys Asn Cys Val Pro Ile Pro Leu
                95                  100                 105

Gly Ser Lys Asn Asn Met Cys Ile Lys Arg Leu Gln Ile Lys Pro
                110                 115                 120

Arg Ser Phe Ser Gly Leu Thr Tyr Leu Lys Ser Leu Tyr Leu Asp
                125                 130                 135

Gly Asn Gln Leu Leu Glu Ile Pro Gln Gly Leu Pro Pro Ser Leu
                140                 145                 150

Gln Leu Leu Ser Leu Glu Ala Asn Asn Ile Phe Ser Ile Arg Lys
                155                 160                 165

Glu Asn Leu Thr Glu Leu Ala Asn Ile Glu Ile Leu Tyr Leu Gly
                170                 175                 180

Gln Asn Cys Tyr Tyr Arg Asn Pro Cys Tyr Val Ser Tyr Ser Ile
                185                 190                 195

Glu Lys Asp Ala Phe Leu Asn Leu Thr Lys Leu Lys Val Leu Ser
                200                 205                 210

Leu Lys Asp Asn Asn Val Thr Ala Val Pro Thr Val Leu Pro Ser
                215                 220                 225

Thr Leu Thr Glu Leu Tyr Leu Tyr Asn Asn Met Ile Ala Lys Ile
                230                 235                 240

Gln Glu Asp Asp Phe Asn Asn Leu Asn Gln Leu Gln Ile Leu Asp
                245                 250                 255

Leu Ser Gly Asn Cys Pro Arg Cys Tyr Asn Ala Pro Phe Pro Cys
                260                 265                 270

Ala Pro Cys Lys Asn Asn Ser Pro Leu Gln Ile Pro Val Asn Ala
                275                 280                 285

Phe Asp Ala Leu Thr Glu Leu Lys Val Leu Arg Leu His Ser Asn
                290                 295                 300

Ser Leu Gln His Val Pro Pro Arg Trp Phe Lys Asn Ile Asn Lys
                305                 310                 315

Leu Gln Glu Leu Asp Leu Ser Gln Asn Phe Leu Ala Lys Glu Ile
                320                 325                 330
```

Gly Asp Ala Lys Phe Leu His Phe Leu Pro Ser Leu Ile Gln Leu
335 340 345

Asp Leu Ser Phe Asn Phe Glu Leu Gln Val Tyr Arg Ala Ser Met
350 355 360

Asn Leu Ser Gln Ala Phe Ser Ser Leu Lys Ser Leu Lys Ile Leu
365 370 375

Arg Ile Arg Gly Tyr Val Phe Lys Glu Leu Lys Ser Phe Asn Leu
380 385 390

Ser Pro Leu His Asn Leu Gln Asn Leu Glu Val Leu Asp Leu Gly
395 400 405

Thr Asn Phe Ile Lys Ile Ala Asn Leu Ser Met Phe Lys Gln Phe
410 415 420

Lys Arg Leu Lys Val Ile Asp Leu Ser Val Asn Lys Ile Ser Pro
425 430 435

Ser Gly Asp Ser Ser Glu Val Gly Phe Cys Ser Asn Ala Arg Thr
440 445 450

Ser Val Glu Ser Tyr Glu Pro Gln Val Leu Glu Gln Leu His Tyr
455 460 465

Phe Arg Tyr Asp Lys Tyr Ala Arg Ser Cys Arg Phe Lys Asn Lys
470 475 480

Glu Ala Ser Phe Met Ser Val Asn Glu Ser Cys Tyr Lys Tyr Gly
485 490 495

Gln Thr Leu Asp Leu Ser Lys Asn Ser Ile Phe Phe Val Lys Ser
500 505 510

Ser Asp Phe Gln His Leu Ser Phe Leu Lys Cys Leu Asn Leu Ser
515 520 525

Gly Asn Leu Ile Ser Gln Thr Leu Asn Gly Ser Glu Phe Gln Pro
530 535 540

Leu Ala Glu Leu Arg Tyr Leu Asp Phe Ser Asn Asn Arg Leu Asp
545 550 555

Leu Leu His Ser Thr Ala Phe Glu Glu Leu His Lys Leu Glu Val
560 565 570

Leu Asp Ile Ser Ser Asn Ser His Tyr Phe Gln Ser Glu Gly Ile
575 580 585

Thr His Met Leu Asn Phe Thr Lys Asn Leu Lys Val Leu Gln Lys
590 595 600

Leu Met Met Asn Asp Asn Asp Ile Ser Ser Ser Thr Ser Arg Thr
605 610 615

Met Glu Ser Glu Ser Leu Arg Thr Leu Glu Phe Arg Gly Asn His
620 625 630

Leu Asp Val Leu Trp Arg Glu Gly Asp Asn Arg Tyr Leu Gln Leu
635 640 645

Phe Lys Asn Leu Leu Lys Leu Glu Glu Leu Asp Ile Ser Lys Asn

650 655 660

Ser Leu Ser Phe Leu Pro Ser Gly Val Phe Asp Gly Met Pro Pro
665 670 675

Asn Leu Lys Asn Leu Ser Leu Ala Lys Asn Gly Leu Lys Ser Phe
680 685 690

Ser Trp Lys Lys Leu Gln Cys Leu Lys Asn Leu Glu Thr Leu Asp
695 700 705

Leu Ser His Asn Gln Leu Thr Thr Val Pro Glu Arg Leu Ser Asn
710 715 720

Cys Ser Arg Ser Leu Lys Asn Leu Ile Leu Lys Asn Asn Gln Ile
725 730 735

Arg Ser Leu Thr Lys Tyr Phe Leu Gln Asp Ala Phe Gln Leu Arg
740 745 750

Tyr Leu Asp Leu Ser Ser Asn Lys Ile Gln Met Ile Gln Lys Thr
755 760 765

Ser Phe Pro Glu Asn Val Leu Asn Asn Leu Lys Met Leu Leu Leu
770 775 780

His His Asn Arg Phe Leu Cys Thr Cys Asp Ala Val Trp Phe Val
785 790 795

Trp Trp Val Asn His Thr Glu Val Thr Ile Pro Tyr Leu Ala Thr
800 805 810

Asp Val Thr Cys Val Gly Pro Gly Ala His Lys Gly Gln Ser Val
815 820 825

Ile Ser Leu Asp Leu Tyr Thr Cys Glu Leu Asp Leu Thr Asn Leu
830 835 840

Ile Leu Phe Ser Leu Ser Ile Ser Val Ser Leu Phe Leu Met Val
845 850 855

Met Met Thr Ala Ser His Leu Tyr Phe Trp Asp Val Trp Tyr Ile
860 865 870

Tyr His Phe Cys Lys Ala Lys Ile Lys Gly Tyr Gln Arg Leu Ile
875 880 885

Ser Pro Asp Cys Cys Tyr Asp Ala Phe Ile Val Tyr Asp Thr Lys
890 895 900

Asp Pro Ala Val Thr Glu Trp Val Leu Ala Glu Leu Val Ala Lys
905 910 915

Leu Glu Asp Pro Arg Glu Lys His Phe Asn Leu Cys Leu Glu Glu
920 925 930

Arg Asp Trp Leu Pro Gly Gln Pro Val Leu Glu Asn Leu Ser Gln
935 940 945

Ser Ile Gln Leu Ser Lys Lys Thr Val Phe Val Met Thr Asp Lys
950 955 960

Tyr Ala Lys Thr Glu Asn Phe Lys Ile Ala Phe Tyr Leu Ser His
965 970 975

Gln Arg Leu Met Asp Glu Lys Val Asp Val Ile Ile Leu Ile Phe
980 985 990

Leu Glu Lys Pro Phe Gln Lys Ser Lys Phe Leu Gln Leu Arg Lys
995 1000 1005

Arg Leu Cys Gly Ser Ser Val Leu Glu Trp Pro Thr Asn Pro Gln
1010 1015 1020

Ala His Pro Tyr Phe Trp Gln Cys Leu Lys Asn Ala Leu Ala Thr
1025 1030 1035

Asp Asn His Val Ala Tyr Ser Gln Val Phe Lys Glu Thr Val
1040 1045

<210> 359
<211> 1875
<212> DNA
<213> Homo Sapien

<400> 359
gacggctggc caccatgcac ggctcctgca gtttcctgat gcttctgctg 50

ccgctactgc tactgctggt ggccaccaca ggccccgttg gagccctcac 100

agatgaggag aaacgtttga tggtggagct gcacaacctc taccgggccc 150

aggtatcccc gacggcctca gacatgctgc acatgagatg ggacgaggag 200

ctggccgcct tcgccaaggc ctacgcacgg cagtgcgtgt ggggccacaa 250

caaggagcgc gggcgccgcg gcgagaatct gttcgccatc acagacgagg 300

gcatggacgt gccgctggcc atggaggagt ggcaccacga gcgtgagcac 350

tacaacctca gcgccgccac ctgcagccca ggccagatgt gcggccacta 400

cacgcaggtg gtatgggcca agacagagag gatcggctgt ggttcccact 450

tctgtgagaa gctccagggt gttgaggaga ccaacatcga attactggtg 500

tgcaactatg agcctccggg gaacgtgaag gggaaacggc cctaccagga 550

ggggactccg tgctcccaat gtccctctgg ctaccactgc aagaactccc 600

tctgtgaacc catcggaagc ccggaagatg ctcaggattt gccttacctg 650

gtaactgagg ccccatcctt ccgggcgact gaagcatcag actctaggaa 700

aatgggtact ccttcttccc tagcaacggg gattccggct ttcttggtaa 750

cagaggtctc aggctccctg gcaaccaagg ctctgcctgc tgtggaaacc 800

caggccccaa cttccttagc aacgaaagac ccgccctcca tggcaacaga 850

ggctccacct tgcgtaacaa ctgaggtccc ttccattttg gcagctcaca 900

gcctgccctc cttggatgag gagccagtta ccttccccaa atcgacccat 950

gttcctatcc caaaatcagc agacaaagtg acagacaaaa caaaagtgcc 1000

ctctaggagc ccagagaact ctctggaccc caagatgtcc ctgacagggg 1050

caagggaact cctaccccat gcccaggagg aggctgaggc tgaggctgag 1100

ttgcctcctt ccagtgaggt cttggcctca gtttttccag cccaggacaa 1150

gccaggtgag ctgcaggcca cactggacca cacggggcac acctcctcca 1200

agtccctgcc caatttcccc aatacctctg ccaccgctaa tgccacgggt 1250

gggcgtgccc tggctctgca gtcgtccttg ccaggtgcag agggccctga 1300

caagcctagc gttgtgtcag ggctgaactc gggccctggt catgtgtggg 1350

gccctctcct gggactactg ctcctgcctc ctctggtgtt ggctggaatc 1400

ttctgaatgg gataccactc aaagggtgaa gaggtcagct gtcctcctgt 1450

catcttcccc accctgtccc cagcccctaa acaagatact tcttggttaa 1500

ggccctccgg aagggaaagg ctacggggca tgtgcctcat cacaccatcc 1550

atcctggagg cacaaggcct ggctggctgc gagctcagga ggccgcctga 1600

ggactgcaca ccgggcccac acctctcctg cccctccctc ctgagtcctg 1650

ggggtgggag gatttgaggg agctcactgc ctacctggcc tggggctgtc 1700

tgcccacaca gcatgtgcgc tctccctgag tgcctgtgta gctggggatg 1750

gggattccta ggggcagatg aaggacaagc cccactggag tggggttctt 1800

tgagtggggg aggcagggac gagggaagga aagtaactcc tgactctcca 1850

ataaaaacct gtccaacctg tgaaa 1875

<210> 360
<211> 463
<212> PRT
<213> Homo Sapien

<400> 360

Met His Gly Ser Cys Ser Phe Leu Met Leu Leu Leu Pro Leu Leu
1 5 10 15

Leu Leu Leu Val Ala Thr Thr Gly Pro Val Gly Ala Leu Thr Asp
20 25 30

Glu Glu Lys Arg Leu Met Val Glu Leu His Asn Leu Tyr Arg Ala
35 40 45

Gln Val Ser Pro Thr Ala Ser Asp Met Leu His Met Arg Trp Asp
50 55 60

Glu Glu Leu Ala Ala Phe Ala Lys Ala Tyr Ala Arg Gln Cys Val
65 70 75

Trp Gly His Asn Lys Glu Arg Gly Arg Arg Gly Glu Asn Leu Phe
80 85 90

Ala Ile Thr Asp Glu Gly Met Asp Val Pro Leu Ala Met Glu Glu
95 100 105

Trp His His Glu Arg Glu His Tyr Asn Leu Ser Ala Ala Thr Cys
110 115 120

```
Ser Pro Gly Gln Met Cys Gly His Tyr Thr Gln Val Val Trp Ala
                125                 130                 135

Lys Thr Glu Arg Ile Gly Cys Gly Ser His Phe Cys Glu Lys Leu
                140                 145                 150

Gln Gly Val Glu Glu Thr Asn Ile Glu Leu Leu Val Cys Asn Tyr
                155                 160                 165

Glu Pro Pro Gly Asn Val Lys Gly Lys Arg Pro Tyr Gln Glu Gly
                170                 175                 180

Thr Pro Cys Ser Gln Cys Pro Ser Gly Tyr His Cys Lys Asn Ser
                185                 190                 195

Leu Cys Glu Pro Ile Gly Ser Pro Glu Asp Ala Gln Asp Leu Pro
                200                 205                 210

Tyr Leu Val Thr Glu Ala Pro Ser Phe Arg Ala Thr Glu Ala Ser
                215                 220                 225

Asp Ser Arg Lys Met Gly Thr Pro Ser Ser Leu Ala Thr Gly Ile
                230                 235                 240

Pro Ala Phe Leu Val Thr Glu Val Ser Gly Ser Leu Ala Thr Lys
                245                 250                 255

Ala Leu Pro Ala Val Glu Thr Gln Ala Pro Thr Ser Leu Ala Thr
                260                 265                 270

Lys Asp Pro Pro Ser Met Ala Thr Glu Ala Pro Pro Cys Val Thr
                275                 280                 285

Thr Glu Val Pro Ser Ile Leu Ala Ala His Ser Leu Pro Ser Leu
                290                 295                 300

Asp Glu Glu Pro Val Thr Phe Pro Lys Ser Thr His Val Pro Ile
                305                 310                 315

Pro Lys Ser Ala Asp Lys Val Thr Asp Lys Thr Lys Val Pro Ser
                320                 325                 330

Arg Ser Pro Glu Asn Ser Leu Asp Pro Lys Met Ser Leu Thr Gly
                335                 340                 345

Ala Arg Glu Leu Leu Pro His Ala Gln Glu Glu Ala Glu Ala Glu
                350                 355                 360

Ala Glu Leu Pro Pro Ser Ser Glu Val Leu Ala Ser Val Phe Pro
                365                 370                 375

Ala Gln Asp Lys Pro Gly Glu Leu Gln Ala Thr Leu Asp His Thr
                380                 385                 390

Gly His Thr Ser Ser Lys Ser Leu Pro Asn Phe Pro Asn Thr Ser
                395                 400                 405

Ala Thr Ala Asn Ala Thr Gly Gly Arg Ala Leu Ala Leu Gln Ser
                410                 415                 420

Ser Leu Pro Gly Ala Glu Gly Pro Asp Lys Pro Ser Val Val Ser
                425                 430                 435

Gly Leu Asn Ser Gly Pro Gly His Val Trp Gly Pro Leu Leu Gly
```

440 445 450

Leu Leu Leu Leu Pro Pro Leu Val Leu Ala Gly Ile Phe
455 460

<210> 361
<211> 1377
<212> DNA
<213> Homo Sapien

<400> 361
gactagttct cttggagtct gggaggagga aagcggagcc ggcagggagc 50

gaaccaggac tggggtgacg gcagggcagg gggcgcctgg ccggggagaa 100

gcgcgggggc tggagcacca ccaactggag ggtccggagt agcgagcgcc 150

ccgaaggagg ccatcgggga gccgggaggg gggactgcga gaggaccccg 200

gcgtccgggc tcccggtgcc agcgctatga ggccactcct cgtcctgctg 250

ctcctgggcc tggcggccgg ctcgccccca ctggacgaca acaagatccc 300

cagcctctgc ccggggcacc ccggccttcc aggcacgccg ggccaccatg 350

gcagccaggg cttgccgggc cgcgatggcc gcgacggccg cgacggcgcg 400

cccggggctc cgggagagaa aggcgagggc gggaggccgg gactgccggg 450

acctcgaggg gaccccgggc cgcgaggaga ggcgggaccc gcggggccca 500

ccgggcctgc cggggagtgc tcggtgcctc cgcgatccgc cttcagcgcc 550

aagcgctccg agagccgggt gcctccgccg tctgacgcac ccttgccctt 600

cgaccgcgtg ctggtgaacg agcagggaca ttacgacgcc gtcaccggca 650

agttcacctg ccaggtgcct ggggtctact acttcgccgt ccatgccacc 700

gtctaccggg ccagcctgca gtttgatctg gtgaagaatg gcgaatccat 750

tgcctctttc ttccagtttt tcggggggtg gcccaagcca gcctcgctct 800

cggggggggc catggtgagg ctggagcctg aggaccaagt gtgggtgcag 850

gtgggtgtgg gtgactacat tggcatctat gccagcatca agacagacag 900

caccttctcc ggatttctgg tgtactccga ctggcacagc tccccagtct 950

ttgcttagtg cccactgcaa agtgagctca tgctctcact cctagaagga 1000

gggtgtgagg ctgacaacca ggtcatccag gagggctggc ccccctggaa 1050

tattgtgaat gactagggag gtggggtaga gcactctccg tcctgctgct 1100

ggcaaggaat gggaacagtg gctgtctgcg atcaggtctg gcagcatggg 1150

gcagtggctg gatttctgcc caagaccaga ggagtgtgct gtgctggcaa 1200

gtgtaagtcc cccagttgct ctggtccagg agcccacggt ggggtgctct 1250

cttcctggtc ctctgcttct ctggatcctc cccacccccct cctgctcctg 1300

gggccggccc ttttctcaga gatcactcaa taaacctaag aaccctcata 1350

aaaaaaaaaa aaaaaaaaaa aaaaaaa 1377

<210> 362
<211> 243
<212> PRT
<213> Homo Sapien

<400> 362
```
Met Arg Pro Leu Leu Val Leu Leu Leu Leu Gly Leu Ala Ala Gly
  1               5                  10                  15

Ser Pro Pro Leu Asp Asp Asn Lys Ile Pro Ser Leu Cys Pro Gly
                 20                  25                  30

His Pro Gly Leu Pro Gly Thr Pro Gly His His Gly Ser Gln Gly
                 35                  40                  45

Leu Pro Gly Arg Asp Gly Arg Asp Gly Arg Asp Gly Ala Pro Gly
                 50                  55                  60

Ala Pro Gly Glu Lys Gly Glu Gly Gly Arg Pro Gly Leu Pro Gly
                 65                  70                  75

Pro Arg Gly Asp Pro Gly Pro Arg Gly Glu Ala Gly Pro Ala Gly
                 80                  85                  90

Pro Thr Gly Pro Ala Gly Glu Cys Ser Val Pro Pro Arg Ser Ala
                 95                 100                 105

Phe Ser Ala Lys Arg Ser Glu Ser Arg Val Pro Pro Pro Ser Asp
                110                 115                 120

Ala Pro Leu Pro Phe Asp Arg Val Leu Val Asn Glu Gln Gly His
                125                 130                 135

Tyr Asp Ala Val Thr Gly Lys Phe Thr Cys Gln Val Pro Gly Val
                140                 145                 150

Tyr Tyr Phe Ala Val His Ala Thr Val Tyr Arg Ala Ser Leu Gln
                155                 160                 165

Phe Asp Leu Val Lys Asn Gly Glu Ser Ile Ala Ser Phe Phe Gln
                170                 175                 180

Phe Phe Gly Gly Trp Pro Lys Pro Ala Ser Leu Ser Gly Gly Ala
                185                 190                 195

Met Val Arg Leu Glu Pro Glu Asp Gln Val Trp Val Gln Val Gly
                200                 205                 210

Val Gly Asp Tyr Ile Gly Ile Tyr Ala Ser Ile Lys Thr Asp Ser
                215                 220                 225

Thr Phe Ser Gly Phe Leu Val Tyr Ser Asp Trp His Ser Ser Pro
                230                 235                 240

Val Phe Ala
```

<210> 363
<211> 1503
<212> DNA
<213> Homo Sapien

<400> 363
ggagagcgga gcgaagctgg ataacagggg accgatgatg tggcgaccat 50

cagttctgct gcttctgttg ctactgaggc acggggccca ggggaagcca 100

tccccagacg caggccctca tggccagggg agggtgcacc aggcggcccc 150

cctgagcgac gctccccatg atgacgccca cgggaacttc cagtacgacc 200

atgaggcttt cctgggacgg gaagtggcca aggaattcga ccaactcacc 250

ccagaggaaa gccaggcccg tctggggcgg atcgtggacc gcatggaccg 300

cgcgggggac ggcgacggct gggtgtcgct ggccgagctt cgcgcgtgga 350

tcgcgcacac gcagcagcgg cacatacggg actcggtgag cgcggcctgg 400

gacacgtacg acacggaccg cgacgggcgt gtgggttggg aggagctgcg 450

caacgccacc tatggccact acgcgcccgg tgaagaattt catgacgtgg 500

aggatgcaga gacctacaaa aagatgctgg ctcgggacga gcggcgtttc 550

cgggtggccg accaggatgg ggactcgatg gccactcgag aggagctgac 600

agccttcctg caccccgagg agttccctca catgcgggac atcgtgattg 650

ctgaaaccct ggaggacctg gacagaaaca aagatggcta tgtccaggtg 700

gaggagtaca tcgcggatct gtactcagcc gagcctgggg aggaggagcc 750

ggcgtgggtg cagacggaga ggcagcagtt ccgggacttc cgggatctga 800

acaaggatgg gcacctggat gggagtgagg tgggccactg ggtgctgccc 850

cctgcccagg accagcccct ggtggaagcc aaccacctgc tgcacgagag 900

cgacacggac aaggatgggc ggctgagcaa agcggaaatc ctgggtaatt 950

ggaacatgtt tgtgggcagt caggccacca actatggcga ggacctgacc 1000

cggcaccacg atgagctgtg agcaccgcgc acctgccaca gcctcagagg 1050

cccgcacaat gaccggagga ggggccgctg tggtctggcc ccctccctgt 1100

ccaggccccg caggaggcag atgcagtccc aggcatcctc ctgcccctgg 1150

gctctcaggg acccccctggg tcggcttctg tccctgtcac acccccaacc 1200

ccagggaggg gctgtcatag tcccagagga taagcaatac ctatttctga 1250

ctgagtctcc cagcccagac ccagggaccc ttggccccaa gctcagctct 1300

aagaaccgcc ccaacccctc cagctccaaa tctgagcctc caccacatag 1350

actgaaactc ccctggcccc agccctctcc tgcctggcct ggcctgggac 1400

acctcctctc tgccaggagg caataaaagc cagcgccggg accttgaaaa 1450

aaaaaaaaaa aaaaaaaaaa aaaaaaaaaa aaaaaaaaaa aaaaaaaaaa 1500

aaa 1503

<210> 364
<211> 328
<212> PRT
<213> Homo Sapien

<400> 364
Met Met Trp Arg Pro Ser Val Leu Leu Leu Leu Leu Leu Leu Arg
1               5                   10                  15

His Gly Ala Gln Gly Lys Pro Ser Pro Asp Ala Gly Pro His Gly
                20                  25                  30

Gln Gly Arg Val His Gln Ala Ala Pro Leu Ser Asp Ala Pro His
                35                  40                  45

Asp Asp Ala His Gly Asn Phe Gln Tyr Asp His Glu Ala Phe Leu
                50                  55                  60

Gly Arg Glu Val Ala Lys Glu Phe Asp Gln Leu Thr Pro Glu Glu
                65                  70                  75

Ser Gln Ala Arg Leu Gly Arg Ile Val Asp Arg Met Asp Arg Ala
                80                  85                  90

Gly Asp Gly Asp Gly Trp Val Ser Leu Ala Glu Leu Arg Ala Trp
                95                  100                 105

Ile Ala His Thr Gln Gln Arg His Ile Arg Asp Ser Val Ser Ala
                110                 115                 120

Ala Trp Asp Thr Tyr Asp Thr Asp Arg Asp Gly Arg Val Gly Trp
                125                 130                 135

Glu Glu Leu Arg Asn Ala Thr Tyr Gly His Tyr Ala Pro Gly Glu
                140                 145                 150

Glu Phe His Asp Val Glu Asp Ala Glu Thr Tyr Lys Lys Met Leu
                155                 160                 165

Ala Arg Asp Glu Arg Arg Phe Arg Val Ala Asp Gln Asp Gly Asp
                170                 175                 180

Ser Met Ala Thr Arg Glu Glu Leu Thr Ala Phe Leu His Pro Glu
                185                 190                 195

Glu Phe Pro His Met Arg Asp Ile Val Ile Ala Glu Thr Leu Glu
                200                 205                 210

Asp Leu Asp Arg Asn Lys Asp Gly Tyr Val Gln Val Glu Glu Tyr
                215                 220                 225

Ile Ala Asp Leu Tyr Ser Ala Glu Pro Gly Glu Glu Glu Pro Ala
                230                 235                 240

Trp Val Gln Thr Glu Arg Gln Gln Phe Arg Asp Phe Arg Asp Leu
                245                 250                 255

Asn Lys Asp Gly His Leu Asp Gly Ser Glu Val Gly His Trp Val
                260                 265                 270

Leu Pro Pro Ala Gln Asp Gln Pro Leu Val Glu Ala Asn His Leu
                275                 280                 285

Leu His Glu Ser Asp Thr Asp Lys Asp Gly Arg Leu Ser Lys Ala

290 295 300

Glu Ile Leu Gly Asn Trp Asn Met Phe Val Gly Ser Gln Ala Thr
305 310 315

Asn Tyr Gly Glu Asp Leu Thr Arg His His Asp Glu Leu
320 325

<210> 365
<211> 1857
<212> DNA
<213> Homo Sapien

<400> 365
gtctgttccc aggagtcctt cggcggctgt tgtgtcagtg gcctgatcgc 50

gatggggaca aaggcgcaag tcgagaggaa actgttgtgc ctcttcatat 100

tggcgatcct gttgtgctcc ctggcattgg gcagtgttac agtgcactct 150

tctgaacctg aagtcagaat tcctgagaat aatcctgtga agttgtcctg 200

tgcctactcg ggcttttctt ctccccgtgt ggagtggaag tttgaccaag 250

gagacaccac cagactcgtt tgctataata acaagatcac agcttcctat 300

gaggaccggg tgaccttctt gccaactggt atcaccttca agtccgtgac 350

acgggaagac actgggacat acacttgtat ggtctctgag gaaggcggca 400

acagctatgg ggaggtcaag gtcaagctca tcgtgcttgt gcctccatcc 450

aagcctacag ttaacatccc ctcctctgcc accattggga accgggcagt 500

gctgacatgc tcagaacaag atggttcccc accttctgaa tacacctggt 550

tcaaagatgg gatagtgatg cctacgaatc ccaaaagcac ccgtgccttc 600

agcaactctt cctatgtcct gaatcccaca acaggagagc tggtctttga 650

tcccctgtca gcctctgata ctggagaata cagctgtgag gcacggaatg 700

ggtatgggac acccatgact tcaaatgctg tgcgcatgga agctgtggag 750

cggaatgtgg gggtcatcgt ggcagccgtc cttgtaaccc tgattctcct 800

gggaatcttg gtttttggca tctggtttgc ctatagccga ggccactttg 850

acagaacaaa gaaagggact tcgagtaaga aggtgattta cagccagcct 900

agtgcccgaa gtgaaggaga attcaaacag acctcgtcat tcctggtgtg 950

agcctggtcg gctcaccgcc tatcatctgc atttgcctta ctcaggtgct 1000

accggactct ggcccctgat gtctgtagtt tcacaggatg ccttatttgt 1050

cttctacacc ccacagggcc ccctacttct tcggatgtgt ttttaataat 1100

gtcagctatg tgccccatcc tccttcatgc cctccctccc tttcctacca 1150

ctgctgagtg gcctggaact tgtttaaagt gtttattccc catttctttg 1200

agggatcagg aaggaatcct gggtatgcca ttgacttccc ttctaagtag 1250

acagcaaaaa tggcgggggt cgcaggaatc tgcactcaac tgcccacctg 1300

gctggcaggg atctttgaat aggtatcttg agcttggttc tgggctcttt 1350

ccttgtgtac tgacgaccag ggccagctgt tctagagcgg gaattagagg 1400

ctagagcggc tgaaatggtt gtttggtgat gacactgggg tccttccatc 1450

tctggggccc actctcttct gtcttcccat gggaagtgcc actgggatcc 1500

ctctgccctg tcctcctgaa tacaagctga ctgacattga ctgtgtctgt 1550

ggaaaatggg agctcttgtt gtggagagca tagtaaattt tcagagaact 1600

tgaagccaaa aggatttaaa accgctgctc taaagaaaag aaaactggag 1650

gctgggcgca gtggctcacg cctgtaatcc cagaggctga ggcaggcgga 1700

tcacctgagg tcgggagttc gggatcagcc tgaccaacat ggagaaaccc 1750

tactggaaat acaaagttag ccaggcatgg tggtgcatgc ctgtagtccc 1800

agctgctcag gagcctggca acaagagcaa aactccagct caaaaaaaaa 1850

aaaaaaa 1857

<210> 366
<211> 299
<212> PRT
<213> Homo Sapien

<400> 366
Met Gly Thr Lys Ala Gln Val Glu Arg Lys Leu Leu Cys Leu Phe
1               5                   10                  15

Ile Leu Ala Ile Leu Leu Cys Ser Leu Ala Leu Gly Ser Val Thr
20                  25                  30

Val His Ser Ser Glu Pro Glu Val Arg Ile Pro Glu Asn Asn Pro
35                  40                  45

Val Lys Leu Ser Cys Ala Tyr Ser Gly Phe Ser Ser Pro Arg Val
50                  55                  60

Glu Trp Lys Phe Asp Gln Gly Asp Thr Thr Arg Leu Val Cys Tyr
65                  70                  75

Asn Asn Lys Ile Thr Ala Ser Tyr Glu Asp Arg Val Thr Phe Leu
80                  85                  90

Pro Thr Gly Ile Thr Phe Lys Ser Val Thr Arg Glu Asp Thr Gly
95                  100                 105

Thr Tyr Thr Cys Met Val Ser Glu Glu Gly Gly Asn Ser Tyr Gly
110                 115                 120

Glu Val Lys Val Lys Leu Ile Val Leu Val Pro Pro Ser Lys Pro
125                 130                 135

Thr Val Asn Ile Pro Ser Ser Ala Thr Ile Gly Asn Arg Ala Val
140                 145                 150

Leu Thr Cys Ser Glu Gln Asp Gly Ser Pro Pro Ser Glu Tyr Thr
155                 160                 165

```
Trp Phe Lys Asp Gly Ile Val Met Pro Thr Asn Pro Lys Ser Thr
                170                 175                 180

Arg Ala Phe Ser Asn Ser Ser Tyr Val Leu Asn Pro Thr Thr Gly
                185                 190                 195

Glu Leu Val Phe Asp Pro Leu Ser Ala Ser Asp Thr Gly Glu Tyr
                200                 205                 210

Ser Cys Glu Ala Arg Asn Gly Tyr Gly Thr Pro Met Thr Ser Asn
                215                 220                 225

Ala Val Arg Met Glu Ala Val Glu Arg Asn Val Gly Val Ile Val
                230                 235                 240

Ala Ala Val Leu Val Thr Leu Ile Leu Leu Gly Ile Leu Val Phe
                245                 250                 255

Gly Ile Trp Phe Ala Tyr Ser Arg Gly His Phe Asp Arg Thr Lys
                260                 265                 270

Lys Gly Thr Ser Ser Lys Lys Val Ile Tyr Ser Gln Pro Ser Ala
                275                 280                 285

Arg Ser Glu Gly Glu Phe Lys Gln Thr Ser Ser Phe Leu Val
                290                 295
```

<210> 367
<211> 2906
<212> DNA
<213> Homo Sapien

<400> 367

```
ggggagagga attgaccatg taaaaggaga cttttttttt tggtggtggt 50

ggctgttggg tgccttgcaa aaatgaagga tgcaggacgc agctttctcc 100

tggaaccgaa cgcaatggat aaactgattg tgcaagagag aaggaagaac 150

gaagcttttt cttgtgagcc ctggatctta acacaaatgt gtatatgtgc 200

acacagggag cattcaagaa tgaaataaac cagagttaga cccgcggggg 250

ttggtgtgtt ctgacataaa taaataatct taaagcagct gttcccctcc 300

ccacccccaa aaaaaaggat gattggaaat gaagaaccga ggattcacaa 350

agaaaaaagt atgttcattt ttctctataa aggagaaagt gagccaagga 400

gatatttttg gaatgaaaag tttggggctt ttttagtaaa gtaaagaact 450

ggtgtggtgg tgttttcctt tctttttgaa tttcccacaa gaggagagga 500

aattaataat acatctgcaa agaaatttca gagaagaaaa gttgaccgcg 550

gcagattgag gcattgattg gggagagaa accagcagag cacagttgga 600

tttgtgccta tgttgactaa aattgacgga taattgcagt tggatttttc 650

ttcatcaacc tccttttttt taaattttta ttccttttgg tatcaagatc 700

atgcgttttc tcttgttctt aaccacctgg atttccatct ggatgttgct 750
```

gtgatcagtc tgaaatacaa ctgtttgaat tccagaagga ccaacaccag 800
ataaattatg aatgttgaac aagatgacct tacatccaca gcagataatg 850
ataggtccta ggtttaacag ggccctattt gaccccctgc ttgtggtgct 900
gctggctctt caacttcttg tggtggctgg tctggtgcgg gctcagacct 950
gcccttctgt gtgctcctgc agcaaccagt tcagcaaggt gatttgtgtt 1000
cggaaaaacc tgcgtgaggt tccggatggc atctccacca acacacggct 1050
gctgaacctc catgagaacc aaatccagat catcaaagtg aacagcttca 1100
agcacttgag gcacttggaa atcctacagt tgagtaggaa ccatatcaga 1150
accattgaaa ttggggcttt caatggtctg gcgaacctca acactctgga 1200
actctttgac aatcgtctta ctaccatccc gaatggagct tttgtatact 1250
tgtctaaact gaaggagctc tggttgcgaa acaaccccat tgaaagcatc 1300
ccttcttatg cttttaacag aattccttct ttgcgccgac tagacttagg 1350
ggaattgaaa agactttcat acatctcaga aggtgccttt gaaggtctgt 1400
ccaacttgag gtatttgaac cttgccatgt gcaaccttcg ggaaatccct 1450
aacctcacac cgctcataaa actagatgag ctggatcttt ctgggaatca 1500
tttatctgcc atcaggcctg gctctttcca gggtttgatg caccttcaaa 1550
aactgtggat gatacagtcc cagattcaag tgattgaacg gaatgccttt 1600
gacaaccttc agtcactagt ggagatcaac ctggcacaca ataatctaac 1650
attactgcct catgacctct tcactccctt gcatcatcta gagcggatac 1700
atttacatca caacccttgg aactgtaact gtgacatact gtggctcagc 1750
tggtggataa aagacatggc cccctcgaac acagcttgtt gtgcccggtg 1800
taacactcct cccaatctaa aggggaggta cattggagag ctcgaccaga 1850
attacttcac atgctatgct ccggtgattg tggagccccc tgcagacctc 1900
aatgtcactg aaggcatggc agctgagctg aaatgtcggg cctccacatc 1950
cctgacatct gtatcttgga ttactccaaa tggaacagtc atgacacatg 2000
gggcgtacaa agtgcggata gctgtgctca gtgatggtac gttaaatttc 2050
acaaatgtaa ctgtgcaaga tacaggcatg tacacatgta tggtgagtaa 2100
ttccgttggg aatactactg cttcagccac cctgaatgtt actgcagcaa 2150
ccactactcc tttctcttac ttttcaaccg tcacagtaga gactatggaa 2200
ccgtctcagg atgaggcacg gaccacagat aacaatgtgg gtcccactcc 2250
agtggtcgac tgggagacca ccaatgtgac cacctctctc acaccacaga 2300
gcacaaggtc gacagagaaa accttcacca tcccagtgac tgatataaac 2350

agtgggatcc caggaattga tgaggtcatg aagactacca aaatcatcat 2400

tgggtgtttt gtggccatca cactcatggc tgcagtgatg ctggtcattt 2450

tctacaagat gaggaagcag caccatcggc aaaaccatca cgccccaaca 2500

aggactgttg aaattattaa tgtggatgat gagattacgg gagacacacc 2550

catggaaagc cacctgccca tgcctgctat cgagcatgag cacctaaatc 2600

actataactc atacaaatct cccttcaacc acacaacaac agttaacaca 2650

ataaattcaa tacacagttc agtgcatgaa ccgttattga tccgaatgaa 2700

ctctaaagac aatgtacaag agactcaaat ctaaaacatt tacagagtta 2750

caaaaaacaa acaatcaaaa aaaaagacag tttattaaaa atgacacaaa 2800

tgactgggct aaatctactg tttcaaaaaa gtgtctttac aaaaaaacaa 2850

aaaagaaaag aaatttattt attaaaaatt ctattgtgat ctaaagcaga 2900

caaaaa 2906

<210> 368
<211> 640
<212> PRT
<213> Homo Sapien

<400> 368

```
Met Leu Asn Lys Met Thr Leu His Pro Gln Gln Ile Met Ile Gly
  1               5                  10                  15

Pro Arg Phe Asn Arg Ala Leu Phe Asp Pro Leu Leu Val Val Leu
                 20                  25                  30

Leu Ala Leu Gln Leu Leu Val Val Ala Gly Leu Val Arg Ala Gln
                 35                  40                  45

Thr Cys Pro Ser Val Cys Ser Cys Ser Asn Gln Phe Ser Lys Val
                 50                  55                  60

Ile Cys Val Arg Lys Asn Leu Arg Glu Val Pro Asp Gly Ile Ser
                 65                  70                  75

Thr Asn Thr Arg Leu Leu Asn Leu His Glu Asn Gln Ile Gln Ile
                 80                  85                  90

Ile Lys Val Asn Ser Phe Lys His Leu Arg His Leu Glu Ile Leu
                 95                 100                 105

Gln Leu Ser Arg Asn His Ile Arg Thr Ile Glu Ile Gly Ala Phe
                110                 115                 120

Asn Gly Leu Ala Asn Leu Asn Thr Leu Glu Leu Phe Asp Asn Arg
                125                 130                 135

Leu Thr Thr Ile Pro Asn Gly Ala Phe Val Tyr Leu Ser Lys Leu
                140                 145                 150

Lys Glu Leu Trp Leu Arg Asn Asn Pro Ile Glu Ser Ile Pro Ser
                155                 160                 165

Tyr Ala Phe Asn Arg Ile Pro Ser Leu Arg Arg Leu Asp Leu Gly
```

```
                170                 175                 180

Glu Leu Lys Arg Leu Ser Tyr Ile Ser Glu Gly Ala Phe Glu Gly
                185                 190                 195

Leu Ser Asn Leu Arg Tyr Leu Asn Leu Ala Met Cys Asn Leu Arg
                200                 205                 210

Glu Ile Pro Asn Leu Thr Pro Leu Ile Lys Leu Asp Glu Leu Asp
                215                 220                 225

Leu Ser Gly Asn His Leu Ser Ala Ile Arg Pro Gly Ser Phe Gln
                230                 235                 240

Gly Leu Met His Leu Gln Lys Leu Trp Met Ile Gln Ser Gln Ile
                245                 250                 255

Gln Val Ile Glu Arg Asn Ala Phe Asp Asn Leu Gln Ser Leu Val
                260                 265                 270

Glu Ile Asn Leu Ala His Asn Asn Leu Thr Leu Leu Pro His Asp
                275                 280                 285

Leu Phe Thr Pro Leu His His Leu Glu Arg Ile His Leu His His
                290                 295                 300

Asn Pro Trp Asn Cys Asn Cys Asp Ile Leu Trp Leu Ser Trp Trp
                305                 310                 315

Ile Lys Asp Met Ala Pro Ser Asn Thr Ala Cys Cys Ala Arg Cys
                320                 325                 330

Asn Thr Pro Pro Asn Leu Lys Gly Arg Tyr Ile Gly Glu Leu Asp
                335                 340                 345

Gln Asn Tyr Phe Thr Cys Tyr Ala Pro Val Ile Val Glu Pro Pro
                350                 355                 360

Ala Asp Leu Asn Val Thr Glu Gly Met Ala Ala Glu Leu Lys Cys
                365                 370                 375

Arg Ala Ser Thr Ser Leu Thr Ser Val Ser Trp Ile Thr Pro Asn
                380                 385                 390

Gly Thr Val Met Thr His Gly Ala Tyr Lys Val Arg Ile Ala Val
                395                 400                 405

Leu Ser Asp Gly Thr Leu Asn Phe Thr Asn Val Thr Val Gln Asp
                410                 415                 420

Thr Gly Met Tyr Thr Cys Met Val Ser Asn Ser Val Gly Asn Thr
                425                 430                 435

Thr Ala Ser Ala Thr Leu Asn Val Thr Ala Ala Thr Thr Thr Pro
                440                 445                 450

Phe Ser Tyr Phe Ser Thr Val Thr Val Glu Thr Met Glu Pro Ser
                455                 460                 465

Gln Asp Glu Ala Arg Thr Thr Asp Asn Asn Val Gly Pro Thr Pro
                470                 475                 480

Val Val Asp Trp Glu Thr Thr Asn Val Thr Thr Ser Leu Thr Pro
                485                 490                 495
```

Gln Ser Thr Arg Ser Thr Glu Lys Thr Phe Thr Ile Pro Val Thr
500 505 510

Asp Ile Asn Ser Gly Ile Pro Gly Ile Asp Glu Val Met Lys Thr
515 520 525

Thr Lys Ile Ile Ile Gly Cys Phe Val Ala Ile Thr Leu Met Ala
530 535 540

Ala Val Met Leu Val Ile Phe Tyr Lys Met Arg Lys Gln His His
545 550 555

Arg Gln Asn His His Ala Pro Thr Arg Thr Val Glu Ile Ile Asn
560 565 570

Val Asp Asp Glu Ile Thr Gly Asp Thr Pro Met Glu Ser His Leu
575 580 585

Pro Met Pro Ala Ile Glu His Glu His Leu Asn His Tyr Asn Ser
590 595 600

Tyr Lys Ser Pro Phe Asn His Thr Thr Thr Val Asn Thr Ile Asn
605 610 615

Ser Ile His Ser Ser Val His Glu Pro Leu Leu Ile Arg Met Asn
620 625 630

Ser Lys Asp Asn Val Gln Glu Thr Gln Ile
635 640

<210> 369
<211> 3296
<212> DNA
<213> Homo Sapien

<400> 369
caaaacttgc gtcgcggaga gcgcccagct tgacttgaat ggaaggagcc 50

cgagcccgcg gagcgcagct gagactgggg gagcgcgttc ggcctgtggg 100

gcgccgctcg gcgccggggc gcagcaggga aggggaagct gtggtctgcc 150

ctgctccacg aggcgccact ggtgtgaacc gggagagccc ctgggtggtc 200

ccgtccccta tccctccttt atatagaaac cttccacact gggaaggcag 250

cggcgaggca ggagggctca tggtgagcaa ggaggccggc tgatctgcag 300

gcgcacagca ttccgagttt acagattttt acagatacca aatggaaggc 350

gaggaggcag aacagcctgc ctggttccat cagccctggc gcccaggcgc 400

atctgactcg gcaccccctg caggcaccat ggcccagagc cgggtgctgc 450

tgctcctgct gctgctgccg ccacagctgc acctgggacc tgtgcttgcc 500

gtgagggccc caggatttgg ccgaagtggc ggccacagcc tgagccccga 550

agagaacgaa tttgcggagg aggagccggt gctggtactg agccctgagg 600

agcccgggcc tggcccagcc gcggtcagct gcccccgaga ctgtgcctgt 650

tcccaggagg gcgtcgtgga ctgtggcggt attgacctgc gtgagttccc 700

gggggacctg cctgagcaca ccaaccacct atctctgcag aacaaccagc 750
tggaaaagat ctaccctgag gagctctccc ggctgcaccg gctggagaca 800
ctgaacctgc aaaacaaccg cctgacttcc cgagggctcc cagagaaggc 850
gtttgagcat ctgaccaacc tcaattacct gtacttggcc aataacaagc 900
tgaccttggc accccgcttc ctgccaaacg ccctgatcag tgtggacttt 950
gctgccaact atctcaccaa gatctatggg ctcacctttg gccagaagcc 1000
aaacttgagg tctgtgtacc tgcacaacaa caagctggca gacgccgggc 1050
tgccggacaa catgttcaac ggctccagca acgtcgaggt cctcatcctg 1100
tccagcaact tcctgcgcca cgtgcccaag cacctgccgc ctgccctgta 1150
caagctgcac ctcaagaaca acaagctgga gaagatcccc ccgggggcct 1200
tcagcgagct gagcagcctg cgcgagctat acctgcagaa caactacctg 1250
actgacgagg gcctggacaa cgagaccttc tggaagctct ccagcctgga 1300
gtacctggat ctgtccagca acaacctgtc tcgggtccca gctgggctgc 1350
cgcgcagcct ggtgctgctg cacttggaga agaacgccat ccggagcgtg 1400
gacgcgaatg tgctgacccc catccgcagc ctggagtacc tgctgctgca 1450
cagcaaccag ctgcgggagc agggcatcca cccactggcc ttccagggcc 1500
tcaagcggtt gcacacggtg cacctgtaca acaacgcgct ggagcgcgtg 1550
cccagtggcc tgcctcgccg cgtgcgcacc ctcatgatcc tgcacaacca 1600
gatcacaggc attggccgcg aagactttgc caccacctac ttcctggagg 1650
agctcaacct cagctacaac cgcatcacca gcccacaggt gcaccgcgac 1700
gccttccgca agctgcgcct gctgcgctcg ctggacctgt cgggcaaccg 1750
gctgcacacg ctgccacctg ggctgcctcg aaatgtccat gtgctgaagg 1800
tcaagcgcaa tgagctggct gccttggcac gaggggcgct ggcgggcatg 1850
gctcagctgc gtgagctgta cctcaccagc aaccgactgc gcagccgagc 1900
cctgggcccc cgtgcctggg tggacctcgc ccatctgcag ctgctggaca 1950
tcgccgggaa tcagctcaca gagatccccg aggggctccc cgagtcactt 2000
gagtacctgt acctgcagaa caacaagatt agtgcggtgc ccgccaatgc 2050
cttcgactcc acgcccaacc tcaaggggat ctttctcagg tttaacaagc 2100
tggctgtggg ctccgtggtg gacagtgcct tccggaggct gaagcacctg 2150
caggtcttgg acattgaagg caacttagag tttggtgaca tttccaagga 2200
ccgtggccgc ttggggaagg aaaaggagga ggaggaagag gaggaggagg 2250
aggaagagga aacaagatag tgacaaggtg atgcagatgt gacctaggat 2300

gatggaccgc cggactcttt tctgcagcac acgcctgtgt gctgtgagcc 2350
ccccactctg ccgtgctcac acagacacac ccagctgcac acatgaggca 2400
tcccacatga cacgggctga cacagtctca tatccccacc ccttcccacg 2450
gcgtgtccca cggccagaca catgcacaca catcacaccc tcaaacaccc 2500
agctcagcca cacacaacta ccctccaaac caccacagtc tctgtcacac 2550
ccccactacc gctgccacgc cctctgaatc atgcagggaa gggtctgccc 2600
ctgccctggc acacacaggc acccattccc tccccctgct gacatgtgta 2650
tgcgtatgca tacacaccac acacacacac atgcacaagt catgtgcgaa 2700
cagccctcca aagcctatgc cacagacagc tcttgcccca gccagaatca 2750
gccatagcag ctcgccgtct gccctgtcca tctgtccgtc cgttccctgg 2800
agaagacaca agggtatcca tgctctgtgg ccaggtgcct gccaccctct 2850
ggaactcaca aaagctggct tttattcctt tcccatccta tggggacagg 2900
agccttcagg actgctggcc tggcctggcc caccctgctc ctccaggtgc 2950
tgggcagtca ctctgctaag agtccctccc tgccacgccc tggcaggaca 3000
caggcacttt tccaatgggc aagcccagtg gaggcaggat gggagagccc 3050
cctgggtgct gctggggcct tggggcagga gtgaagcaga ggtgatgggg 3100
ctgggctgag ccagggagga aggacccagc tgcacctagg agacaccttt 3150
gttcttcagg cctgtggggg aagttccggg tgcctttatt ttttattctt 3200
ttctaaggaa aaaaatgata aaaatctcaa agctgatttt tcttgttata 3250
gaaaaactaa tataaaagca ttatccctat ccctgcaaaa aaaaaa 3296

<210> 370
<211> 642
<212> PRT
<213> Homo Sapien

<400> 370
Met Glu Gly Glu Glu Ala Glu Gln Pro Ala Trp Phe His Gln Pro
1               5                   10                  15

Trp Arg Pro Gly Ala Ser Asp Ser Ala Pro Pro Ala Gly Thr Met
                20                  25                  30

Ala Gln Ser Arg Val Leu Leu Leu Leu Leu Leu Leu Pro Pro Gln
                35                  40                  45

Leu His Leu Gly Pro Val Leu Ala Val Arg Ala Pro Gly Phe Gly
                50                  55                  60

Arg Ser Gly Gly His Ser Leu Ser Pro Glu Glu Asn Glu Phe Ala
                65                  70                  75

Glu Glu Glu Pro Val Leu Val Leu Ser Pro Glu Glu Pro Gly Pro
                80                  85                  90

Gly Pro Ala Ala Val Ser Cys Pro Arg Asp Cys Ala Cys Ser Gln
95 100 105

Glu Gly Val Val Asp Cys Gly Gly Ile Asp Leu Arg Glu Phe Pro
110 115 120

Gly Asp Leu Pro Glu His Thr Asn His Leu Ser Leu Gln Asn Asn
125 130 135

Gln Leu Glu Lys Ile Tyr Pro Glu Glu Leu Ser Arg Leu His Arg
140 145 150

Leu Glu Thr Leu Asn Leu Gln Asn Asn Arg Leu Thr Ser Arg Gly
155 160 165

Leu Pro Glu Lys Ala Phe Glu His Leu Thr Asn Leu Asn Tyr Leu
170 175 180

Tyr Leu Ala Asn Asn Lys Leu Thr Leu Ala Pro Arg Phe Leu Pro
185 190 195

Asn Ala Leu Ile Ser Val Asp Phe Ala Ala Asn Tyr Leu Thr Lys
200 205 210

Ile Tyr Gly Leu Thr Phe Gly Gln Lys Pro Asn Leu Arg Ser Val
215 220 225

Tyr Leu His Asn Asn Lys Leu Ala Asp Ala Gly Leu Pro Asp Asn
230 235 240

Met Phe Asn Gly Ser Ser Asn Val Glu Val Leu Ile Leu Ser Ser
245 250 255

Asn Phe Leu Arg His Val Pro Lys His Leu Pro Pro Ala Leu Tyr
260 265 270

Lys Leu His Leu Lys Asn Asn Lys Leu Glu Lys Ile Pro Pro Gly
275 280 285

Ala Phe Ser Glu Leu Ser Ser Leu Arg Glu Leu Tyr Leu Gln Asn
290 295 300

Asn Tyr Leu Thr Asp Glu Gly Leu Asp Asn Glu Thr Phe Trp Lys
305 310 315

Leu Ser Ser Leu Glu Tyr Leu Asp Leu Ser Ser Asn Asn Leu Ser
320 325 330

Arg Val Pro Ala Gly Leu Pro Arg Ser Leu Val Leu Leu His Leu
335 340 345

Glu Lys Asn Ala Ile Arg Ser Val Asp Ala Asn Val Leu Thr Pro
350 355 360

Ile Arg Ser Leu Glu Tyr Leu Leu Leu His Ser Asn Gln Leu Arg
365 370 375

Glu Gln Gly Ile His Pro Leu Ala Phe Gln Gly Leu Lys Arg Leu
380 385 390

His Thr Val His Leu Tyr Asn Asn Ala Leu Glu Arg Val Pro Ser
395 400 405

Gly Leu Pro Arg Arg Val Arg Thr Leu Met Ile Leu His Asn Gln
410 415 420

Ile Thr Gly Ile Gly Arg Glu Asp Phe Ala Thr Thr Tyr Phe Leu
425 430 435

Glu Glu Leu Asn Leu Ser Tyr Asn Arg Ile Thr Ser Pro Gln Val
440 445 450

His Arg Asp Ala Phe Arg Lys Leu Arg Leu Leu Arg Ser Leu Asp
455 460 465

Leu Ser Gly Asn Arg Leu His Thr Leu Pro Pro Gly Leu Pro Arg
470 475 480

Asn Val His Val Leu Lys Val Lys Arg Asn Glu Leu Ala Ala Leu
485 490 495

Ala Arg Gly Ala Leu Ala Gly Met Ala Gln Leu Arg Glu Leu Tyr
500 505 510

Leu Thr Ser Asn Arg Leu Arg Ser Arg Ala Leu Gly Pro Arg Ala
515 520 525

Trp Val Asp Leu Ala His Leu Gln Leu Leu Asp Ile Ala Gly Asn
530 535 540

Gln Leu Thr Glu Ile Pro Glu Gly Leu Pro Glu Ser Leu Glu Tyr
545 550 555

Leu Tyr Leu Gln Asn Asn Lys Ile Ser Ala Val Pro Ala Asn Ala
560 565 570

Phe Asp Ser Thr Pro Asn Leu Lys Gly Ile Phe Leu Arg Phe Asn
575 580 585

Lys Leu Ala Val Gly Ser Val Val Asp Ser Ala Phe Arg Arg Leu
590 595 600

Lys His Leu Gln Val Leu Asp Ile Glu Gly Asn Leu Glu Phe Gly
605 610 615

Asp Ile Ser Lys Asp Arg Gly Arg Leu Gly Lys Glu Lys Glu Glu
620 625 630

Glu Glu Glu Glu Glu Glu Glu Glu Glu Glu Thr Arg
635 640

<210> 371
<211> 2849
<212> DNA
<213> Homo Sapien

<400> 371
cactttctcc ctctcttcct ttactttcga gaaaccgcgc ttccgcttct 50

ggtcgcagag acctcggaga ccgcgccggg gagacggagg tgctgtgggt 100

gggggggacc tgtggctgct cgtaccgccc cccaccctcc tcttctgcac 150

tgccgtcctc cggaagacct ttcccctgc tctgtttcct tcaccgagtc 200

tgtgcatcgc cccggacctg gccgggagga ggcttggccg gcgggagatg 250

ctctaggggc ggcgcgggag gagcggccgg cgggacggag ggcccggcag 300
gaagatgggc tcccgtggac agggactctt gctggcgtac tgcctgctcc 350
ttgcctttgc ctctggcctg gtcctgagtc gtgtgcccca tgtccagggg 400
gaacagcagg agtgggaggg gactgaggag ctgccgtcgc ctccggacca 450
tgccgagagg gctgaagaac aacatgaaaa atacaggccc agtcaggacc 500
aggggctccc tgcttcccgg tgcttgcgct gctgtgaccc cggtacctcc 550
atgtacccgg cgaccgccgt gccccagatc aacatcacta tcttgaaagg 600
ggagaagggt gaccgcggag atcgaggcct ccaagggaaa tatggcaaaa 650
caggctcagc aggggccagg ggccacactg gacccaaagg gcagaagggc 700
tccatggggg cccctgggga gcggtgcaag agccactacg ccgccttttc 750
ggtgggccgg aagaagccca tgcacagcaa ccactactac cagacggtga 800
tcttcgacac ggagttcgtg aacctctacg accacttcaa catgttcacc 850
ggcaagttct actgctacgt gcccggcctc tacttcttca gcctcaacgt 900
gcacacctgg aaccagaagg agacctacct gcacatcatg aagaacgagg 950
aggaggtggt gatcttgttc gcgcaggtgg gcgaccgcag catcatgcaa 1000
agccagagcc tgatgctgga gctgcgagag caggaccagg tgtgggtacg 1050
cctctacaag ggcgaacgtg agaacgccat cttcagcgag gagctggaca 1100
cctacatcac cttcagtggc tacctggtca agcacgccac cgagccctag 1150
ctggccggcc acctcctttc ctctcgccac cttccacccc tgcgctgtgc 1200
tgaccccacc gcctcttccc cgatccctgg actccgactc cctggctttg 1250
gcattcagtg agacgccctg cacacacaga aagccaaagc gatcggtgct 1300
cccagatccc gcagcctctg gagagagctg acggcagatg aaatcaccag 1350
ggcggggcac ccgcgagaac cctctgggac cttccgcggc cctctctgca 1400
cacatcctca agtgaccccg cacggcgaga cgcgggtggc ggcagggcgt 1450
cccagggtgc ggcaccgcgg ctccagtcct tggaaataat taggcaaatt 1500
ctaaaggtct caaaaggagc aaagtaaacc gtggaggaca aagaaaaggg 1550
ttgttatttt tgtctttcca gccagcctgc tggctcccaa gagagaggcc 1600
ttttcagttg agactctgct taagagaaga tccaaagtta aagctctggg 1650
gtcaggggag gggccggggg caggaaacta cctctggctt aattctttta 1700
agccacgtag gaactttctt gagggatagg tggaccctga catccctgtg 1750
gccttgccca agggctctgc tggtctttct gagtcacagc tgcgaggtga 1800
tgggggctgg ggccccaggc gtcagcctcc cagagggaca gctgagcccc 1850

ctgccttggc tccaggttgg tagaagcagc cgaagggctc ctgacagtgg 1900

ccagggaccc ctgggtcccc caggcctgca gatgtttcta tgaggggcag 1950

agctccttgg tacatccatg tgtggctctg ctccacccct gtgccacccc 2000

agagccctgg ggggtggtct ccatgcctgc caccctggca tcggctttct 2050

gtgccgcctc ccacacaaat cagccccaga aggccccggg gccttggctt 2100

ctgtttttta taaaacacct caagcagcac tgcagtctcc catctcctcg 2150

tgggctaagc atcaccgctt ccacgtgtgt tgtgttggtt ggcagcaagg 2200

ctgatccaga ccccttctgc ccccactgcc ctcatccagg cctctgacca 2250

gtagcctgag aggggctttt tctaggcttc agagcagggg agagctggaa 2300

ggggctagaa agctcccgct tgtctgtttc tcaggctcct gtgagcctca 2350

gtcctgagac cagagtcaag aggaagtaca cgtcccaatc acccgtgtca 2400

ggattcactc tcaggagctg ggtggcagga gaggcaatag cccctgtggc 2450

aattgcagga ccagctggag cagggttgcg gtgtctccac ggtgctctcg 2500

ccctgcccat ggccacccca gactctgatc tccaggaacc ccatagcccc 2550

tctccacctc accccatgtt gatgcccagg gtcactcttg ctacccgctg 2600

ggcccccaaa cccccgctgc ctctcttcct tccccccatc ccccacctgg 2650

ttttgactaa tcctgcttcc ctctctgggc ctggctgccg ggatctgggg 2700

tccctaagtc cctctcttta aagaacttct gcgggtcaga ctctgaagcc 2750

gagttgctgt gggcgtgccc ggaagcagag cgccacactc gctgcttaag 2800

ctcccccagc tctttccaga aaacattaaa ctcagaattg tgttttcaa 2849

<210> 372
<211> 281
<212> PRT
<213> Homo Sapien

<400> 372

```
Met Gly Ser Arg Gly Gln Gly Leu Leu Leu Ala Tyr Cys Leu Leu
  1               5                  10                  15

Leu Ala Phe Ala Ser Gly Leu Val Leu Ser Arg Val Pro His Val
                 20                  25                  30

Gln Gly Glu Gln Gln Glu Trp Glu Gly Thr Glu Glu Leu Pro Ser
                 35                  40                  45

Pro Pro Asp His Ala Glu Arg Ala Glu Glu Gln His Glu Lys Tyr
                 50                  55                  60

Arg Pro Ser Gln Asp Gln Gly Leu Pro Ala Ser Arg Cys Leu Arg
                 65                  70                  75

Cys Cys Asp Pro Gly Thr Ser Met Tyr Pro Ala Thr Ala Val Pro
                 80                  85                  90
```

```
Gln Ile Asn Ile Thr Ile Leu Lys Gly Glu Lys Gly Asp Arg Gly
                95                  100                 105

Asp Arg Gly Leu Gln Gly Lys Tyr Gly Lys Thr Gly Ser Ala Gly
                110                 115                 120

Ala Arg Gly His Thr Gly Pro Lys Gly Gln Lys Gly Ser Met Gly
                125                 130                 135

Ala Pro Gly Glu Arg Cys Lys Ser His Tyr Ala Ala Phe Ser Val
                140                 145                 150

Gly Arg Lys Lys Pro Met His Ser Asn His Tyr Tyr Gln Thr Val
                155                 160                 165

Ile Phe Asp Thr Glu Phe Val Asn Leu Tyr Asp His Phe Asn Met
                170                 175                 180

Phe Thr Gly Lys Phe Tyr Cys Tyr Val Pro Gly Leu Tyr Phe Phe
                185                 190                 195

Ser Leu Asn Val His Thr Trp Asn Gln Lys Glu Thr Tyr Leu His
                200                 205                 210

Ile Met Lys Asn Glu Glu Glu Val Val Ile Leu Phe Ala Gln Val
                215                 220                 225

Gly Asp Arg Ser Ile Met Gln Ser Gln Ser Leu Met Leu Glu Leu
                230                 235                 240

Arg Glu Gln Asp Gln Val Trp Val Arg Leu Tyr Lys Gly Glu Arg
                245                 250                 255

Glu Asn Ala Ile Phe Ser Glu Glu Leu Asp Thr Tyr Ile Thr Phe
                260                 265                 270

Ser Gly Tyr Leu Val Lys His Ala Thr Glu Pro
                275                 280
```

<210> 373
<211> 1572
<212> DNA
<213> Homo Sapien

<400> 373

```
cggagtggtg cgccaacgtg agaggaaacc cgtgcgcggc tgcgctttcc 50

tgtccccaag ccgttctaga cgcgggaaaa atgctttctg aaagcagctc 100

ctttttgaag ggtgtgatgc ttggaagcat tttctgtgct ttgatcacta 150

tgctaggaca cattaggatt ggtcatggaa atagaatgca ccaccatgag 200

catcatcacc tacaagctcc taacaaagaa gatatcttga aaatttcaga 250

ggatgagcgc atggagctca gtaagagctt tcgagtatac tgtattatcc 300

ttgtaaaacc caaagatgtg agtctttggg ctgcagtaaa ggagacttgg 350

accaaacact gtgacaaagc agagttcttc agttctgaaa atgttaaagt 400

gtttgagtca attaatatgg acacaaatga catgtggtta atgatgagaa 450

aagcttacaa atacgccttt gataagtata gagaccaata caactggttc 500
```

```
ttccttgcac gccccactac gtttgctatc attgaaaacc taaagtattt 550
tttgttaaaa aaggatccat cacagccttt ctatctaggc cacactataa 600
aatctggaga ccttgaatat gtgggtatgg aaggaggaat tgtcttaagt 650
gtagaatcaa tgaaaagact taacagcctt ctcaatatcc cagaaaagtg 700
tcctgaacag ggagggatga tttggaagat atctgaagat aaacagctag 750
cagtttgcct gaaatatgct ggagtatttg cagaaaatgc agaagatgct 800
gatggaaaag atgtatttaa taccaaatct gttgggcttt ctattaaaga 850
ggcaatgact tatcacccca accaggtagt agaaggctgt tgttcagata 900
tggctgttac ttttaatgga ctgactccaa atcagatgca tgtgatgatg 950
tatggggtat accgccttag ggcatttggg catattttca atgatgcatt 1000
ggttttctta cctccaaatg gttctgacaa tgactgagaa gtggtagaaa 1050
agcgtgaata tgatctttgt ataggacgtg tgttgtcatt atttgtagta 1100
gtaactacat atccaataca gctgtatgtt tctttttctt ttctaatttg 1150
gtggcactgg tataaccaca cattaaagtc agtagtacat ttttaaatga 1200
gggtggtttt tttctttaaa acacatgaac attgtaaatg tgttggaaag 1250
aagtgtttta agaataataa ttttgcaaat aaactattaa taaatattat 1300
atgtgataaa ttctaaatta tgaacattag aaatctgtgg ggcacatatt 1350
tttgctgatt ggttaaaaaa ttttaacagg tctttagcgt tctaagatat 1400
gcaaatgata tctctagttg tgaatttgtg attaaagtaa aacttttagc 1450
tgtgtgttcc ctttacttct aatactgatt tatgttctaa gcctccccaa 1500
gttccaatgg atttgccttc tcaaaatgta caactaagca actaaagaaa 1550
attaaagtga aagttgaaaa at 1572
```

<210> 374
<211> 318
<212> PRT
<213> Homo Sapien

<400> 374

```
Met Leu Ser Glu Ser Ser Ser Phe Leu Lys Gly Val Met Leu Gly
1               5                   10                  15

Ser Ile Phe Cys Ala Leu Ile Thr Met Leu Gly His Ile Arg Ile
                20                  25                  30

Gly His Gly Asn Arg Met His His His Glu His His His Leu Gln
                35                  40                  45

Ala Pro Asn Lys Glu Asp Ile Leu Lys Ile Ser Glu Asp Glu Arg
                50                  55                  60

Met Glu Leu Ser Lys Ser Phe Arg Val Tyr Cys Ile Ile Leu Val
```

65 70 75

Lys Pro Lys Asp Val Ser Leu Trp Ala Ala Val Lys Glu Thr Trp
80 85 90

Thr Lys His Cys Asp Lys Ala Glu Phe Phe Ser Ser Glu Asn Val
95 100 105

Lys Val Phe Glu Ser Ile Asn Met Asp Thr Asn Asp Met Trp Leu
110 115 120

Met Met Arg Lys Ala Tyr Lys Tyr Ala Phe Asp Lys Tyr Arg Asp
125 130 135

Gln Tyr Asn Trp Phe Phe Leu Ala Arg Pro Thr Thr Phe Ala Ile
140 145 150

Ile Glu Asn Leu Lys Tyr Phe Leu Leu Lys Lys Asp Pro Ser Gln
155 160 165

Pro Phe Tyr Leu Gly His Thr Ile Lys Ser Gly Asp Leu Glu Tyr
170 175 180

Val Gly Met Glu Gly Gly Ile Val Leu Ser Val Glu Ser Met Lys
185 190 195

Arg Leu Asn Ser Leu Leu Asn Ile Pro Glu Lys Cys Pro Glu Gln
200 205 210

Gly Gly Met Ile Trp Lys Ile Ser Glu Asp Lys Gln Leu Ala Val
215 220 225

Cys Leu Lys Tyr Ala Gly Val Phe Ala Glu Asn Ala Glu Asp Ala
230 235 240

Asp Gly Lys Asp Val Phe Asn Thr Lys Ser Val Gly Leu Ser Ile
245 250 255

Lys Glu Ala Met Thr Tyr His Pro Asn Gln Val Val Glu Gly Cys
260 265 270

Cys Ser Asp Met Ala Val Thr Phe Asn Gly Leu Thr Pro Asn Gln
275 280 285

Met His Val Met Met Tyr Gly Val Tyr Arg Leu Arg Ala Phe Gly
290 295 300

His Ile Phe Asn Asp Ala Leu Val Phe Leu Pro Pro Asn Gly Ser
305 310 315

Asp Asn Asp

<210> 375
<211> 1679
<212> DNA
<213> Homo Sapien

<400> 375
gttgtgtcct tcagcaaaac agtggattta aatctccttg cacaagcttg 50

agagcaacac aatctatcag gaaagaaaga aagaaaaaaa ccgaacctga 100

caaaaaagaa gaaaaagaag aagaaaaaaa atcatgaaaa ccatccagcc 150

aaaaatgcac aattctatct cttgggcaat cttcacgggg ctggctgctc 200
tgtgtctctt ccaaggagtg cccgtgcgca gcggagatgc caccttcccc 250
aaagctatgg acaacgtgac ggtccggcag gggagagcg ccaccctcag 300
gtgcactatt gacaaccggg tcacccgggt ggcctggcta aaccgcagca 350
ccatcctcta tgctgggaat gacaagtggt gcctggatcc tcgcgtggtc 400
cttctgagca acacccaaac gcagtacagc atcgagatcc agaacgtgga 450
tgtgtatgac gagggccctt acacctgctc ggtgcagaca gacaaccacc 500
caaagacctc tagggtccac ctcattgtgc aagtatctcc caaaattgta 550
gagatttctt cagatatctc cattaatgaa gggaacaata ttagcctcac 600
ctgcatagca actggtagac cagagcctac ggttacttgg agacacatct 650
ctcccaaagc ggttggcttt gtgagtgaag acgaatactt ggaaattcag 700
ggcatcaccc gggagcagtc aggggactac gagtgcagtg cctccaatga 750
cgtggccgcg cccgtggtac ggagagtaaa ggtcaccgtg aactatccac 800
catacatttc agaagccaag ggtacaggtg tccccgtggg acaaaagggg 850
acactgcagt gtgaagcctc agcagtcccc tcagcagaat tccagtggta 900
caaggatgac aaaagactga ttgaaggaaa gaaaggggtg aaagtggaaa 950
acagaccttt cctctcaaaa ctcatcttct tcaatgtctc tgaacatgac 1000
tatgggaact acacttgcgt ggcctccaac aagctgggcc acaccaatgc 1050
cagcatcatg ctatttggtc caggcgccgt cagcgaggtg agcaacggca 1100
cgtcgaggag ggcaggctgc gtctggctgc tgcctcttct ggtcttgcac 1150
ctgcttctca aattttgatg tgagtgccac ttccccaccc gggaaaggct 1200
gccgccacca ccaccaccaa cacaacagca atggcaacac cgacagcaac 1250
caatcagata tatacaaatg aaattagaag aaacacagcc tcatgggaca 1300
gaaatttgag ggaggggaac aaagaatact ttggggggaa aagagtttta 1350
aaaaagaaat tgaaaattgc cttgcagata tttaggtaca atggagtttt 1400
cttttcccaa acgggaagaa cacagcacac ccggcttgga cccactgcaa 1450
gctgcatcgt gcaacctctt tggtgccagt gtgggcaagg gctcagcctc 1500
tctgcccaca gagtgccccc acgtggaaca ttctggagct ggccatccca 1550
aattcaatca gtccatagag acgaacagaa tgagaccttc cggcccaagc 1600
gtggcgctgc gggcactttg gtagactgtg ccaccacggc gtgtgttgtg 1650
aaacgtgaaa taaaaagagc aaaaaaaaa 1679

<210> 376

<211> 344
<212> PRT
<213> Homo Sapien

<400> 376
```
Met Lys Thr Ile Gln Pro Lys Met His Asn Ser Ile Ser Trp Ala
  1               5                  10                  15

Ile Phe Thr Gly Leu Ala Ala Leu Cys Leu Phe Gln Gly Val Pro
                 20                  25                  30

Val Arg Ser Gly Asp Ala Thr Phe Pro Lys Ala Met Asp Asn Val
                 35                  40                  45

Thr Val Arg Gln Gly Glu Ser Ala Thr Leu Arg Cys Thr Ile Asp
                 50                  55                  60

Asn Arg Val Thr Arg Val Ala Trp Leu Asn Arg Ser Thr Ile Leu
                 65                  70                  75

Tyr Ala Gly Asn Asp Lys Trp Cys Leu Asp Pro Arg Val Val Leu
                 80                  85                  90

Leu Ser Asn Thr Gln Thr Gln Tyr Ser Ile Glu Ile Gln Asn Val
                 95                 100                 105

Asp Val Tyr Asp Glu Gly Pro Tyr Thr Cys Ser Val Gln Thr Asp
                110                 115                 120

Asn His Pro Lys Thr Ser Arg Val His Leu Ile Val Gln Val Ser
                125                 130                 135

Pro Lys Ile Val Glu Ile Ser Ser Asp Ile Ser Ile Asn Glu Gly
                140                 145                 150

Asn Asn Ile Ser Leu Thr Cys Ile Ala Thr Gly Arg Pro Glu Pro
                155                 160                 165

Thr Val Thr Trp Arg His Ile Ser Pro Lys Ala Val Gly Phe Val
                170                 175                 180

Ser Glu Asp Glu Tyr Leu Glu Ile Gln Gly Ile Thr Arg Glu Gln
                185                 190                 195

Ser Gly Asp Tyr Glu Cys Ser Ala Ser Asn Asp Val Ala Ala Pro
                200                 205                 210

Val Val Arg Arg Val Lys Val Thr Val Asn Tyr Pro Pro Tyr Ile
                215                 220                 225

Ser Glu Ala Lys Gly Thr Gly Val Pro Val Gly Gln Lys Gly Thr
                230                 235                 240

Leu Gln Cys Glu Ala Ser Ala Val Pro Ser Ala Glu Phe Gln Trp
                245                 250                 255

Tyr Lys Asp Asp Lys Arg Leu Ile Glu Gly Lys Lys Gly Val Lys
                260                 265                 270

Val Glu Asn Arg Pro Phe Leu Ser Lys Leu Ile Phe Phe Asn Val
                275                 280                 285

Ser Glu His Asp Tyr Gly Asn Tyr Thr Cys Val Ala Ser Asn Lys
                290                 295                 300
```

Leu Gly His Thr Asn Ala Ser Ile Met Leu Phe Gly Pro Gly Ala
305 310 315

Val Ser Glu Val Ser Asn Gly Thr Ser Arg Arg Ala Gly Cys Val
320 325 330

Trp Leu Leu Pro Leu Leu Val Leu His Leu Leu Leu Lys Phe
335 340

<210> 377
<211> 2110
<212> DNA
<213> Homo Sapien

<400> 377
cttctttgaa aaggattatc acctgatcag gttctctctg catttgcccc 50

tttagattgt gaaatgtggc tcaaggtctt cacaactttc ctttcctttg 100

caacaggtgc ttgctcgggg ctgaaggtga cagtgccatc acacactgtc 150

catggcgtca gaggtcaggc cctctaccta cccgtccact atggcttcca 200

cactccagca tcagacatcc agatcatatg gctatttgag agaccccaca 250

caatgcccaa atacttactg ggctctgtga ataagtctgt ggttcctgac 300

ttggaatacc aacacaagtt caccatgatg ccacccaatg catctctgct 350

tatcaaccca ctgcagttcc ctgatgaagg caattacatc gtgaaggtca 400

acattcaggg aaatggaact ctatctgcca gtcagaagat acaagtcacg 450

gttgatgatc ctgtcacaaa gccagtggtg cagattcatc ctccctctgg 500

ggctgtggag tatgtgggga acatgaccct gacatgccat gtggaagggg 550

gcactcggct agcttaccaa tggctaaaaa atgggagacc tgtccacacc 600

agctccacct actccttttc tccccaaaac aatacccttc atattgctcc 650

agtaaccaag gaagacattg ggaattacag ctgcctggtg aggaaccctg 700

tcagtgaaat ggaaagtgat atcattatgc ccatcatata ttatggacct 750

tatggacttc aagtgaattc tgataaaggg ctaaaagtag gggaagtgtt 800

tactgttgac cttggagagg ccatcctatt tgattgttct gctgattctc 850

atccccccaa cacctactcc tggattagga ggactgacaa tactacatat 900

atcattaagc atgggcctcg cttagaagtt gcatctgaga aagtagccca 950

gaagacaatg gactatgtgt gctgtgctta caacaacata accggcaggc 1000

aagatgaaac tcatttcaca gttatcatca cttccgtagg actggagaag 1050

cttgcacaga aaggaaaatc attgtcacct ttagcaagta taactggaat 1100

atcactattt ttgattatat ccatgtgtct tctcttccta tggaaaaaat 1150

atcaacccta caaagttata aaacagaaac tagaaggcag gccagaaaca 1200

gaatacagga aagctcaaac attttcaggc catgaagatg ctctggatga 1250

cttcggaata tatgaatttg ttgcttttcc agatgtttct ggtgtttcca 1300

ggattccaag caggtctgtt ccagcctctg attgtgtatc ggggcaagat 1350

ttgcacagta cagtgtatga agttattcag cacatccctg cccagcagca 1400

agaccatcca gagtgaactt tcatgggcta aacagtacat tcgagtgaaa 1450

ttctgaagaa acattttaag gaaaaacagt ggaaaagtat attaatctgg 1500

aatcagtgaa gaaaccagga ccaacacctc ttactcatta ttcctttaca 1550

tgcagaatag aggcatttat gcaaattgaa ctgcaggttt ttcagcatat 1600

acacaatgtc ttgtgcaaca gaaaaacatg ttggggaaat attcctcagt 1650

ggagagtcgt tctcatgctg acggggagaa cgaaagtgac aggggtttcc 1700

tcataagttt tgtatgaaat atctctacaa acctcaatta gttctactct 1750

acactttcac tatcatcaac actgagacta tcctgtctca cctacaaatg 1800

tggaaacttt acattgttcg atttttcagc agactttgtt ttattaaatt 1850

tttattagtg ttaagaatgc taaatttatg tttcaatttt atttccaaat 1900

ttctatcttg ttatttgtac aacaaagtaa taaggatggt tgtcacaaaa 1950

acaaaactat gccttctctt ttttttcaat caccagtagt atttttgaga 2000

agacttgtga acacttaagg aaatgactat taaagtctta tttttatttt 2050

tttcaaggaa agatggattc aaataaatta ttctgttttt gcttttaaaa 2100

aaaaaaaaaa 2110

<210> 378
<211> 450
<212> PRT
<213> Homo Sapien

<400> 378

```
Met Trp Leu Lys Val Phe Thr Thr Phe Leu Ser Phe Ala Thr Gly
  1               5                  10                  15

Ala Cys Ser Gly Leu Lys Val Thr Val Pro Ser His Thr Val His
                 20                  25                  30

Gly Val Arg Gly Gln Ala Leu Tyr Leu Pro Val His Tyr Gly Phe
                 35                  40                  45

His Thr Pro Ala Ser Asp Ile Gln Ile Ile Trp Leu Phe Glu Arg
                 50                  55                  60

Pro His Thr Met Pro Lys Tyr Leu Leu Gly Ser Val Asn Lys Ser
                 65                  70                  75

Val Val Pro Asp Leu Glu Tyr Gln His Lys Phe Thr Met Met Pro
                 80                  85                  90

Pro Asn Ala Ser Leu Leu Ile Asn Pro Leu Gln Phe Pro Asp Glu
                 95                 100                 105
```

Gly Asn Tyr Ile Val Lys Val Asn Ile Gln Gly Asn Gly Thr Leu
110 115 120

Ser Ala Ser Gln Lys Ile Gln Val Thr Val Asp Asp Pro Val Thr
125 130 135

Lys Pro Val Val Gln Ile His Pro Pro Ser Gly Ala Val Glu Tyr
140 145 150

Val Gly Asn Met Thr Leu Thr Cys His Val Glu Gly Gly Thr Arg
155 160 165

Leu Ala Tyr Gln Trp Leu Lys Asn Gly Arg Pro Val His Thr Ser
170 175 180

Ser Thr Tyr Ser Phe Ser Pro Gln Asn Asn Thr Leu His Ile Ala
185 190 195

Pro Val Thr Lys Glu Asp Ile Gly Asn Tyr Ser Cys Leu Val Arg
200 205 210

Asn Pro Val Ser Glu Met Glu Ser Asp Ile Ile Met Pro Ile Ile
215 220 225

Tyr Tyr Gly Pro Tyr Gly Leu Gln Val Asn Ser Asp Lys Gly Leu
230 235 240

Lys Val Gly Glu Val Phe Thr Val Asp Leu Gly Glu Ala Ile Leu
245 250 255

Phe Asp Cys Ser Ala Asp Ser His Pro Pro Asn Thr Tyr Ser Trp
260 265 270

Ile Arg Arg Thr Asp Asn Thr Thr Tyr Ile Ile Lys His Gly Pro
275 280 285

Arg Leu Glu Val Ala Ser Glu Lys Val Ala Gln Lys Thr Met Asp
290 295 300

Tyr Val Cys Cys Ala Tyr Asn Asn Ile Thr Gly Arg Gln Asp Glu
305 310 315

Thr His Phe Thr Val Ile Ile Thr Ser Val Gly Leu Glu Lys Leu
320 325 330

Ala Gln Lys Gly Lys Ser Leu Ser Pro Leu Ala Ser Ile Thr Gly
335 340 345

Ile Ser Leu Phe Leu Ile Ile Ser Met Cys Leu Leu Phe Leu Trp
350 355 360

Lys Lys Tyr Gln Pro Tyr Lys Val Ile Lys Gln Lys Leu Glu Gly
365 370 375

Arg Pro Glu Thr Glu Tyr Arg Lys Ala Gln Thr Phe Ser Gly His
380 385 390

Glu Asp Ala Leu Asp Asp Phe Gly Ile Tyr Glu Phe Val Ala Phe
395 400 405

Pro Asp Val Ser Gly Val Ser Arg Ile Pro Ser Arg Ser Val Pro
410 415 420

Ala Ser Asp Cys Val Ser Gly Gln Asp Leu His Ser Thr Val Tyr
425 430 435

Glu Val Ile Gln His Ile Pro Ala Gln Gln Gln Asp His Pro Glu
440 445 450

<210> 379
<211> 823
<212> DNA
<213> Homo Sapien

<400> 379
atagtagaag aatgtctctg aaattactgg atgagtttca gtcatacttt 50

cacatgggca caatttcaca ttcaagctcc ttatcctagg ctaattttat 100

attatgttaa atcacttgtt tttgttctca cggcttcctg cctgctatag 150

gcataattac gaggaagcag aacttctcca gaagcaagcg cacatgcgtt 200

ccaaaataag agcaaattcg ctctaaacac aggaaaagac ctgaagcttt 250

aattaagggg ttacatccaa ccccagagcg cttttgtggg cactgattgc 300

tccagcttct gcgtcactgc gcgagggaag agggaagagg atccaggcgt 350

tagacatgta tagacacaaa aacagctgga gattgggctt aaaataccca 400

ccaagctcca aagaagagac ccaagtcccc aaaacattga tttcagggct 450

gccaggaagg aagagcagca gcagggtggg agagaagctc cagtcagccc 500

acaagatgcc attgtccccc ggcctcctgc tgctgctgct ctccggggcc 550

acggccaccg ctgccctgcc cctggagggt ggccccaccg gccgagacag 600

cgagcatatg caggaagcgg caggaataag gaaaagcagc ctcctgactt 650

tcctcgcttg gtggtttgag tggacctccc aggccagtgc cgggcccctc 700

ataggagagg aagctcggga ggtggccagg cggcaggaag gcgcaccccc 750

ccagcaatcc gcgcgccggg acagaatgcc ctgcaggaac ttcttctgga 800

agaccttctc ctcctgcaaa tag 823

<210> 380
<211> 155
<212> PRT
<213> Homo Sapien

<400> 380
Met Tyr Arg His Lys Asn Ser Trp Arg Leu Gly Leu Lys Tyr Pro
1 5 10 15

Pro Ser Ser Lys Glu Glu Thr Gln Val Pro Lys Thr Leu Ile Ser
20 25 30

Gly Leu Pro Gly Arg Lys Ser Ser Ser Arg Val Gly Glu Lys Leu
35 40 45

Gln Ser Ala His Lys Met Pro Leu Ser Pro Gly Leu Leu Leu Leu
50 55 60

```
Leu Leu Ser Gly Ala Thr Ala Thr Ala Ala Leu Pro Leu Glu Gly
                65                  70                  75

Gly Pro Thr Gly Arg Asp Ser Glu His Met Gln Glu Ala Ala Gly
                80                  85                  90

Ile Arg Lys Ser Ser Leu Leu Thr Phe Leu Ala Trp Trp Phe Glu
                95                  100                 105

Trp Thr Ser Gln Ala Ser Ala Gly Pro Leu Ile Gly Glu Glu Ala
                110                 115                 120

Arg Glu Val Ala Arg Arg Gln Glu Gly Ala Pro Pro Gln Gln Ser
                125                 130                 135

Ala Arg Arg Asp Arg Met Pro Cys Arg Asn Phe Phe Trp Lys Thr
                140                 145                 150

Phe Ser Ser Cys Lys
                155
```

<210> 381
<211> 2236
<212> DNA
<213> Homo Sapien

<400> 381

```
ggcgccggtg caccgggcgg gctgagcgcc tcctgcggcc cggcctgcgc 50
gccccggccc gccgcgccgc ccacgcccca accccggccc gcgcccccta 100
gcccccgccc gggcccgcgc ccgcgcccgc gcccaggtga gcgctccgcc 150
cgccgcgagg ccccgccccg gcccgccccc gccccgcccc ggccggcggg 200
ggaaccgggc ggattcctcg cgcgtcaaac cacctgatcc cataaaacat 250
tcatcctccc ggcggcccgc gctgcgagcg ccccgccagt ccgcgccgcc 300
gccgccctcg ccctgtgcgc cctgcgcgcc ctgcgcaccc gcggcccgag 350
cccagccaga gccgggcgga gcggagcgcg ccgagcctcg tcccgcggcc 400
gggccggggc cgggccgtag cggcggcgcc tggatgcgga cccggccgcg 450
gggagacggg cgcccgcccc gaaacgactt tcagtccccg acgcgccccg 500
cccaacccct acgatgaaga gggcgtccgc tggagggagc cggctgctgg 550
catgggtgct gtggctgcag gcctggcagg tggcagcccc atgcccaggt 600
gcctgcgtat gctacaatga gcccaaggtg acgacaagct gcccccagca 650
gggcctgcag gctgtgcccg tgggcatccc tgctgccagc cagcgcatct 700
tcctgcacgg caaccgcatc tcgcatgtgc cagctgccag cttccgtgcc 750
tgccgcaacc tcaccatcct gtggctgcac tcgaatgtgc tggcccgaat 800
tgatgcggct gccttcactg gcctggccct cctggagcag ctggacctca 850
gcgataatgc acagctccgg tctgtggacc ctgccacatt ccacggcctg 900
ggccgcctac acacgctgca cctggaccgc tgcggcctgc aggagctggg 950
```

cccggggctg ttccgcggcc tggctgccct gcagtacctc tacctgcagg 1000

acaacgcgct gcaggcactg cctgatgaca ccttccgcga cctgggcaac 1050

ctcacacacc tcttcctgca cggcaaccgc atctccagcg tgcccgagcg 1100

cgccttccgt gggctgcaca gcctcgaccg tctcctactg caccagaacc 1150

gcgtggccca tgtgcacccg catgccttcc gtgaccttgg ccgcctcatg 1200

acactctatc tgtttgccaa caatctatca gcgctgccca ctgaggccct 1250

ggcccccctg cgtgccctgc agtacctgag gctcaacgac aacccctggg 1300

tgtgtgactg ccgggcacgc ccactctggg cctggctgca gaagttccgc 1350

ggctcctcct ccgaggtgcc ctgcagcctc ccgcaacgcc tggctggccg 1400

tgacctcaaa cgcctagctg ccaatgacct gcagggctgc gctgtggcca 1450

ccggccctta ccatcccatc tggaccggca gggccaccga tgaggagccg 1500

ctggggcttc ccaagtgctg ccagccagat gccgctgaca aggcctcagt 1550

actggagcct ggaagaccag cttcggcagg caatgcgctg aagggacgcg 1600

tgccgcccgg tgacagcccg ccgggcaacg gctctggccc acggcacatc 1650

aatgactcac cctttgggac tctgcctggc tctgctgagc ccccgctcac 1700

tgcagtgcgg cccgagggct ccgagccacc agggttcccc acctcgggcc 1750

ctcgccggag gccaggctgt tcacgcaaga accgcacccg cagccactgc 1800

cgtctgggcc aggcaggcag cgggggtggc gggactggtg actcagaagg 1850

ctcaggtgcc ctacccagcc tcacctgcag cctcaccccc ctgggcctgg 1900

cgctggtgct gtggacagtg cttgggccct gctgaccccc agcggacaca 1950

agagcgtgct cagcagccag gtgtgtgtac atacggggtc tctctccacg 2000

ccgccaagcc agccgggcgg ccgacccgtg ggcaggccca ggccaggtcc 2050

tccctgatgg acgcctgccg cccgccaccc ccatctccac cccatcatgt 2100

ttacagggtt cggcggcagc gtttgttcca gaacgccgcc tcccacccag 2150

atcgcggtat atagagatat gcattttatt ttacttgtgt aaaaatatcg 2200

gacgacgtgg aataaagagc tcttttctta aaaaaa 2236

<210> 382
<211> 473
<212> PRT
<213> Homo Sapien

<400> 382
Met Lys Arg Ala Ser Ala Gly Gly Ser Arg Leu Leu Ala Trp Val
1               5                   10                  15

Leu Trp Leu Gln Ala Trp Gln Val Ala Ala Pro Cys Pro Gly Ala
            20                  25                  30

```
Cys Val Cys Tyr Asn Glu Pro Lys Val Thr Thr Ser Cys Pro Gln
                35                  40                  45

Gln Gly Leu Gln Ala Val Pro Val Gly Ile Pro Ala Ala Ser Gln
                50                  55                  60

Arg Ile Phe Leu His Gly Asn Arg Ile Ser His Val Pro Ala Ala
                65                  70                  75

Ser Phe Arg Ala Cys Arg Asn Leu Thr Ile Leu Trp Leu His Ser
                80                  85                  90

Asn Val Leu Ala Arg Ile Asp Ala Ala Ala Phe Thr Gly Leu Ala
                95                  100                 105

Leu Leu Glu Gln Leu Asp Leu Ser Asp Asn Ala Gln Leu Arg Ser
                110                 115                 120

Val Asp Pro Ala Thr Phe His Gly Leu Gly Arg Leu His Thr Leu
                125                 130                 135

His Leu Asp Arg Cys Gly Leu Gln Glu Leu Gly Pro Gly Leu Phe
                140                 145                 150

Arg Gly Leu Ala Ala Leu Gln Tyr Leu Tyr Leu Gln Asp Asn Ala
                155                 160                 165

Leu Gln Ala Leu Pro Asp Asp Thr Phe Arg Asp Leu Gly Asn Leu
                170                 175                 180

Thr His Leu Phe Leu His Gly Asn Arg Ile Ser Ser Val Pro Glu
                185                 190                 195

Arg Ala Phe Arg Gly Leu His Ser Leu Asp Arg Leu Leu Leu His
                200                 205                 210

Gln Asn Arg Val Ala His Val His Pro His Ala Phe Arg Asp Leu
                215                 220                 225

Gly Arg Leu Met Thr Leu Tyr Leu Phe Ala Asn Asn Leu Ser Ala
                230                 235                 240

Leu Pro Thr Glu Ala Leu Ala Pro Leu Arg Ala Leu Gln Tyr Leu
                245                 250                 255

Arg Leu Asn Asp Asn Pro Trp Val Cys Asp Cys Arg Ala Arg Pro
                260                 265                 270

Leu Trp Ala Trp Leu Gln Lys Phe Arg Gly Ser Ser Ser Glu Val
                275                 280                 285

Pro Cys Ser Leu Pro Gln Arg Leu Ala Gly Arg Asp Leu Lys Arg
                290                 295                 300

Leu Ala Ala Asn Asp Leu Gln Gly Cys Ala Val Ala Thr Gly Pro
                305                 310                 315

Tyr His Pro Ile Trp Thr Gly Arg Ala Thr Asp Glu Glu Pro Leu
                320                 325                 330

Gly Leu Pro Lys Cys Cys Gln Pro Asp Ala Ala Asp Lys Ala Ser
                335                 340                 345
```

```
Val Leu Glu Pro Gly Arg Pro Ala Ser Ala Gly Asn Ala Leu Lys
                350                 355                 360

Gly Arg Val Pro Pro Gly Asp Ser Pro Pro Gly Asn Gly Ser Gly
                365                 370                 375

Pro Arg His Ile Asn Asp Ser Pro Phe Gly Thr Leu Pro Gly Ser
                380                 385                 390

Ala Glu Pro Pro Leu Thr Ala Val Arg Pro Glu Gly Ser Glu Pro
                395                 400                 405

Pro Gly Phe Pro Thr Ser Gly Pro Arg Arg Arg Pro Gly Cys Ser
                410                 415                 420

Arg Lys Asn Arg Thr Arg Ser His Cys Arg Leu Gly Gln Ala Gly
                425                 430                 435

Ser Gly Gly Gly Gly Thr Gly Asp Ser Glu Gly Ser Gly Ala Leu
                440                 445                 450

Pro Ser Leu Thr Cys Ser Leu Thr Pro Leu Gly Leu Ala Leu Val
                455                 460                 465

Leu Trp Thr Val Leu Gly Pro Cys
                470
```

<210> 383
<211> 2336
<212> DNA
<213> Homo Sapien

<220>
<221> unsure
<222> 1620, 1673
<223> unknown base

<400> 383

```
ttcgtgaccc ttgagaaaag agttggtggt aaatgtgcca cgtcttctaa 50

gaagggggag tcctgaactt gtctgaagcc cttgtccgta agccttgaac 100

tacgttctta aatctatgaa gtcgagggac ctttcgctgc ttttgtaggg 150

acttctttcc ttgcttcagc aacatgaggc ttttcttgtg gaacgcggtc 200

ttgactctgt tcgtcacttc tttgattggg gctttgatcc ctgaaccaga 250

agtgaaaatt gaagttctcc agaagccatt catctgccat cgcaagacca 300

aaggagggga tttgatgttg gtccactatg aaggctactt agaaaaggac 350

ggctccttat ttcactccac tcacaaacat aacaatggtc agcccatttg 400

gtttaccctg ggcatcctgg aggctctcaa aggttgggac cagggcttga 450

aaggaatgtg tgtaggagag aagagaaagc tcatcattcc tcctgctctg 500

ggctatggaa aagaaggaaa aggtaaaatt cccccagaaa gtacactgat 550

atttaatatt gatctcctgg agattcgaaa tggaccaaga tcccatgaat 600

cattccaaga aatggatctt aatgatgact ggaaactctc taaagatgag 650
```

gttaaagcat atttaaagaa ggagtttgaa aaacatggtg cggtggtgaa 700
tgaaagtcat catgatgctt tggtggagga tatttttgat aaagaagatg 750
aagacaaaga tgggtttata tctgccagag aatttacata taaacacgat 800
gagttataga gatacatcta cccttttaat atagcactca tctttcaaga 850
gagggcagtc atctttaaag aacattttat ttttatacaa tgttctttct 900
tgctttgttt tttattttta tatatttttt ctgactccta tttaaagaac 950
cccttaggtt tctaagtacc catttctttc tgataagtta ttgggaagaa 1000
aaagctaatt ggtctttgaa tagaagactt ctggacaatt tttcactttc 1050
acagatatga agctttgttt tactttctca cttataaatt taaaatgttg 1100
caactgggaa tataccacga catgagacca ggttatagca caaattagca 1150
ccctatattt ctgcttccct ctattttctc caagttagag gtcaacattt 1200
gaaaagcctt ttgcaatagc ccaaggcttg ctattttcat gttataatga 1250
aatagtttat gtgtaactgg ctctgagtct ctgcttgagg accagaggaa 1300
aatggttgtt ggacctgact tgttaatggc tactgcttta ctaaggagat 1350
gtgcaatgct gaagttagaa acaaggttaa tagccaggca tggtggctca 1400
tgcctgtaat cccagcactt tgggaggctg aggcgggcgg atcacctgag 1450
gttgggagtt cgagaccagc ctgaccaaca cggagaaacc ctatctctac 1500
taaaaataca aagtagcccg gcgtggtgat gcgtgcctgt aatcccagct 1550
acccaggaag gctgaggcgg cagaatcact tgaacccgag gccgaggttg 1600
cggtaagccg agatcacctn cagcctggac actctgtctc gaaaaaagaa 1650
aagaacacgg ttaataccat atnaatatgt atgcattgag acatgctacc 1700
taggacttaa gctgatgaag cttggctcct agtgattggt ggcctattat 1750
gataaatagg acaaatcatt tatgtgtgag tttctttgta ataaaatgta 1800
tcaatatgtt atagatgagg tagaaagtta tatttatatt caatatttac 1850
ttcttaaggc tagcggaata tccttcctgg ttctttaatg ggtagtctat 1900
agtatattat actacaataa cattgtatca taagataaag tagtaaacca 1950
gtctacattt tcccatttct gtctcatcaa aaactgaagt tagctgggtg 2000
tggtggctca tgcctgtaat cccagcactt tgggggccaa ggagggtgga 2050
tcacttgaga tcaggagttc aagaccagcc tggccaacat ggtgaaacct 2100
tgtctctact aaaaatacaa aaattagcca ggcgtggtgg tgcacacctg 2150
tagtcccagc tactcgggag gctgagacag gagatttgct tgaacccggg 2200
aggcggaggt tgcagtgagc caagattgtg ccactgcact ccagcctggg 2250

tgacagagca agactccatc tcaaaaaaaa aaaaaagaag cagacctaca 2300

gcagctacta ttgaataaat acctatcctg gatttt 2336

<210> 384
<211> 211
<212> PRT
<213> Homo Sapien

<400> 384
Met Arg Leu Phe Leu Trp Asn Ala Val Leu Thr Leu Phe Val Thr
1 5 10 15

Ser Leu Ile Gly Ala Leu Ile Pro Glu Pro Glu Val Lys Ile Glu
20 25 30

Val Leu Gln Lys Pro Phe Ile Cys His Arg Lys Thr Lys Gly Gly
35 40 45

Asp Leu Met Leu Val His Tyr Glu Gly Tyr Leu Glu Lys Asp Gly
50 55 60

Ser Leu Phe His Ser Thr His Lys His Asn Asn Gly Gln Pro Ile
65 70 75

Trp Phe Thr Leu Gly Ile Leu Glu Ala Leu Lys Gly Trp Asp Gln
80 85 90

Gly Leu Lys Gly Met Cys Val Gly Glu Lys Arg Lys Leu Ile Ile
95 100 105

Pro Pro Ala Leu Gly Tyr Gly Lys Glu Gly Lys Gly Lys Ile Pro
110 115 120

Pro Glu Ser Thr Leu Ile Phe Asn Ile Asp Leu Leu Glu Ile Arg
125 130 135

Asn Gly Pro Arg Ser His Glu Ser Phe Gln Glu Met Asp Leu Asn
140 145 150

Asp Asp Trp Lys Leu Ser Lys Asp Glu Val Lys Ala Tyr Leu Lys
155 160 165

Lys Glu Phe Glu Lys His Gly Ala Val Val Asn Glu Ser His His
170 175 180

Asp Ala Leu Val Glu Asp Ile Phe Asp Lys Glu Asp Glu Asp Lys
185 190 195

Asp Gly Phe Ile Ser Ala Arg Glu Phe Thr Tyr Lys His Asp Glu
200 205 210

Leu

<210> 385
<211> 2749
<212> DNA
<213> Homo Sapien

<220>
<221> unsure
<222> 1869, 1887
<223> unknown base

<400> 385

```
ctcccacggt gtccagcgcc cagaatgcgg cttctggtcc tgctatgggg 50
ttgcctgctg ctcccaggtt atgaagccct ggagggccca gaggaaatca 100
gcgggttcga aggggacact gtgtccctgc agtgcaccta cagggaagag 150
ctgagggacc accggaagta ctggtgcagg aagggtggga tcctcttctc 200
tcgctgctct ggcaccatct atgcagaaga agaaggccag gagacaatga 250
agggcagggt gtccatccgt gacagccgcc aggagctctc gctcattgtg 300
accctgtgga acctcaccct gcaagacgct ggggagtact ggtgtggggt 350
cgaaaaacgg ggccccgatg agtctttact gatctctctg ttcgtctttc 400
caggaccctg ctgtcctccc tccccttctc ccaccttcca gcctctggct 450
acaacacgcc tgcagcccaa ggcaaaagct cagcaaaccc agcccccagg 500
attgacttct cctgggctct acccggcagc caccacagcc aagcagggga 550
agacaggggc tgaggcccct ccattgccag ggacttccca gtacgggcac 600
gaaaggactt ctcagtacac aggaacctct cctcacccag cgacctctcc 650
tcctgcaggg agctcccgcc cccccatgca gctggactcc acctcagcag 700
aggacaccag tccagctctc agcagtggca gctctaagcc cagggtgtcc 750
atcccgatgg tccgcatact ggccccagtc ctggtgctgc tgagccttct 800
gtcagccgca ggcctgatcg ccttctgcag ccacctgctc ctgtggagaa 850
aggaagctca acaggccacg gagacacaga ggaacgagaa gttctggctc 900
tcacgcttga ctgcggagga aaaggaagcc ccttcccagg cccctgaggg 950
ggacgtgatc tcgatgcctc ccctccacac atctgaggag gagctgggct 1000
tctcgaagtt tgtctcagcg tagggcagga ggccctcctg gccaggccag 1050
cagtgaagca gtatggctgg ctggatcagc accgattccc gaaagctttc 1100
cacctcagcc tcagagtcca gctgcccgga ctccagggct ctccccaccc 1150
tccccaggct ctcctcttgc atgttccagc ctgacctaga agcgtttgtc 1200
agccctggag cccagagcgg tggccttgct cttccggctg gagactggga 1250
catccctgat aggttcacat ccctgggcag agtaccaggc tgctgaccct 1300
cagcagggcc agacaaggct cagtggatct ggtctgagtt tcaatctgcc 1350
aggaactcct gggcctcatg cccagtgtcg gaccctgcct tcctcccact 1400
ccagacccca ccttgtcttc cctccctggc gtcctcagac ttagtcccac 1450
ggtctcctgc atcagctggt gatgaagagg agcatgctgg ggtgagactg 1500
ggattctggc ttctctttga accacctgca tccagccctt caggaagcct 1550
```

gtgaaaaacg tgattcctgg ccccaccaag acccaccaaa accatctctg 1600

ggcttggtgc aggactctga attctaacaa tgcccagtga ctgtcgcact 1650

tgagtttgag ggccagtggg cctgatgaac gctcacaccc cttcagctta 1700

gagtctgcat ttgggctgtg acgtctccac ctgccccaat agatctgctc 1750

tgtctgcgac accagatcca cgtggggact cccctgaggc ctgctaagtc 1800

caggccttgg tcaggtcagg tgcacattgc aggataagcc caggaccggc 1850

acagaagtgg ttgcctttnc catttgccct ccctggncca tgccttcttg 1900

cctttggaaa aaatgatgaa gaaaaccttg gctccttcct tgtctggaaa 1950

gggttacttg cctatgggtt ctggtggcta gagagaaaag tagaaaacca 2000

gagtgcacgt aggtgtctaa cacagaggag agtaggaaca gggcggatac 2050

ctgaaggtga ctccgagtcc agccccctgg agaaggggtc gggggtggtg 2100

gtaaagtagc acaactacta tttttttct ttttccatta ttattgtttt 2150

ttaagacaga atctcgtgct gctgcccagg ctggagtgca gtggcacgat 2200

ctgcaaactc cgcctcctgg gttcaagtga ttcttctgcc tcagcctccc 2250

gagtagctgg gattacaggc acgcaccacc acacctggct aatttttgta 2300

cttttagtag agatggggtt tcaccatgtt ggccaggctg gtcttgaact 2350

cctgacctca aatgagcctc ctgcttcagt ctcccaaatt gccgggatta 2400

caggcatgag ccactgtgtc tggccctatt tcctttaaaa agtgaaatta 2450

agagttgttc agtatgcaaa acttggaaag atggaggaga aaaagaaaag 2500

gaagaaaaaa atgtcaccca tagtctcacc agagactatc attatttcgt 2550

tttgttgtac ttccttccac tcttttcttc ttcacataat ttgccggtgt 2600

tctttttaca gagcaattat cttgtatata caactttgta tcctgccttt 2650

tccaccttat cgttccatca ctttattcca gcacttctct gtgttttaca 2700

gacctttttta taaataaaat gttcatcagc tgcataaaaa aaaaaaaaa 2749

<210> 386
<211> 332
<212> PRT
<213> Homo Sapien

<400> 386
Met Arg Leu Leu Val Leu Leu Trp Gly Cys Leu Leu Leu Pro Gly
1               5                   10                  15

Tyr Glu Ala Leu Glu Gly Pro Glu Glu Ile Ser Gly Phe Glu Gly
            20                  25                  30

Asp Thr Val Ser Leu Gln Cys Thr Tyr Arg Glu Glu Leu Arg Asp
            35                  40                  45

His Arg Lys Tyr Trp Cys Arg Lys Gly Gly Ile Leu Phe Ser Arg

```
                50                  55                  60

Cys Ser Gly Thr Ile Tyr Ala Glu Glu Glu Gly Gln Glu Thr Met
                65                  70                  75

Lys Gly Arg Val Ser Ile Arg Asp Ser Arg Gln Glu Leu Ser Leu
                80                  85                  90

Ile Val Thr Leu Trp Asn Leu Thr Leu Gln Asp Ala Gly Glu Tyr
                95                  100                 105

Trp Cys Gly Val Glu Lys Arg Gly Pro Asp Glu Ser Leu Leu Ile
                110                 115                 120

Ser Leu Phe Val Phe Pro Gly Pro Cys Cys Pro Pro Ser Pro Ser
                125                 130                 135

Pro Thr Phe Gln Pro Leu Ala Thr Thr Arg Leu Gln Pro Lys Ala
                140                 145                 150

Lys Ala Gln Gln Thr Gln Pro Pro Gly Leu Thr Ser Pro Gly Leu
                155                 160                 165

Tyr Pro Ala Ala Thr Thr Ala Lys Gln Gly Lys Thr Gly Ala Glu
                170                 175                 180

Ala Pro Pro Leu Pro Gly Thr Ser Gln Tyr Gly His Glu Arg Thr
                185                 190                 195

Ser Gln Tyr Thr Gly Thr Ser Pro His Pro Ala Thr Ser Pro Pro
                200                 205                 210

Ala Gly Ser Ser Arg Pro Pro Met Gln Leu Asp Ser Thr Ser Ala
                215                 220                 225

Glu Asp Thr Ser Pro Ala Leu Ser Ser Gly Ser Ser Lys Pro Arg
                230                 235                 240

Val Ser Ile Pro Met Val Arg Ile Leu Ala Pro Val Leu Val Leu
                245                 250                 255

Leu Ser Leu Leu Ser Ala Ala Gly Leu Ile Ala Phe Cys Ser His
                260                 265                 270

Leu Leu Leu Trp Arg Lys Glu Ala Gln Gln Ala Thr Glu Thr Gln
                275                 280                 285

Arg Asn Glu Lys Phe Trp Leu Ser Arg Leu Thr Ala Glu Glu Lys
                290                 295                 300

Glu Ala Pro Ser Gln Ala Pro Glu Gly Asp Val Ile Ser Met Pro
                305                 310                 315

Pro Leu His Thr Ser Glu Glu Glu Leu Gly Phe Ser Lys Phe Val
                320                 325                 330

Ser Ala
```

<210> 387
<211> 2458
<212> DNA
<213> Homo Sapien

<400> 387
gcgccgggag cccatctgcc cccaggggca cggggcgcgg ggccggctcc 50
cgcccggcac atggctgcag ccacctcgcg cgcaccccga ggcgccgcgc 100
ccagctcgcc cgaggtccgt cggaggcgcc cggccgcccc ggagccaagc 150
agcaactgag cggggaagcg cccgcgtccg gggatcggga tgtccctcct 200
ccttctcctc ttgctagttt cctactatgt tggaaccttg gggactcaca 250
ctgagatcaa gagagtggca gaggaaaagg tcactttgcc ctgccaccat 300
caactggggc ttccagaaaa agacactctg gatattgaat ggctgctcac 350
cgataatgaa gggaaccaaa aagtggtgat cacttactcc agtcgtcatg 400
tctacaataa cttgactgag gaacagaagg gccgagtggc ctttgcttcc 450
aatttcctgg caggagatgc ctccttgcag attgaacctc tgaagcccag 500
tgatgagggc cggtacacct gtaaggttaa gaattcaggg cgctacgtgt 550
ggagccatgt catcttaaaa gtcttagtga gaccatccaa gcccaagtgt 600
gagttggaag gagagctgac agaaggaagt gacctgactt tgcagtgtga 650
gtcatcctct ggcacagagc ccattgtgta ttactggcag cgaatccgag 700
agaaagaggg agaggatgaa cgtctgcctc ccaaatctag gattgactac 750
aaccaccctg gacgagttct gctgcagaat cttaccatgt cctactctgg 800
actgtaccag tgcacagcag gcaacgaagc tgggaaggaa agctgtgtgg 850
tgcgagtaac tgtacagtat gtacaaagca tcggcatggt tgcaggagca 900
gtgacaggca tagtggctgg agccctgctg attttcctct tggtgtggct 950
gctaatccga aggaaagaca aagaaagata tgaggaagaa gagagaccta 1000
atgaaattcg agaagatgct gaagctccaa aagcccgtct tgtgaaaccc 1050
agctcctctt cctcaggctc tcggagctca cgctctggtt cttcctccac 1100
tcgctccaca gcaaatagtg cctcacgcag ccagcggaca ctgtcaactg 1150
acgcagcacc ccagccaggg ctggccaccc aggcatacag cctagtgggg 1200
ccagaggtga gaggttctga accaaagaaa gtccaccatg ctaatctgac 1250
caaagcagaa accacaccca gcatgatccc cagccagagc agagccttcc 1300
aaacggtctg aattacaatg gacttgactc ccacgctttc ctaggagtca 1350
gggtctttgg actcttctcg tcattggagc tcaagtcacc agccacacaa 1400
ccagatgaga ggtcatctaa gtagcagtga gcattgcacg gaacagattc 1450
agatgagcat tttccttata caataccaaa caagcaaaag gatgtaagct 1500
gattcatctg taaaaaggca tcttattgtg cctttagacc agagtaaggg 1550
aaagcaggag tccaaatcta tttgttgacc aggacctgtg gtgagaaggt 1600

tggggaaagg tgaggtgaat atacctaaaa cttttaatgt gggatatttt 1650

gtatcagtgc tttgattcac aattttcaag aggaaatggg atgctgtttg 1700

taaattttct atgcatttct gcaaacttat tggattatta gttattcaga 1750

cagtcaagca gaacccacag ccttattaca cctgtctaca ccatgtactg 1800

agctaaccac ttctaagaaa ctccaaaaaa ggaaacatgt gtcttctatt 1850

ctgacttaac ttcatttgtc ataaggtttg gatattaatt tcaaggggag 1900

ttgaaatagt gggagatgga gaagagtgaa tgagtttctc ccactctata 1950

ctaatctcac tatttgtatt gagcccaaaa taactatgaa aggagacaaa 2000

aatttgtgac aaaggattgt gaagagcttt ccatcttcat gatgttatga 2050

ggattgttga caaacattag aaatatataa tggagcaatt gtggatttcc 2100

cctcaaatca gatgcctcta aggactttcc tgctagatat ttctggaagg 2150

agaaaataca acatgtcatt tatcaacgtc cttagaaaga attcttctag 2200

agaaaaaggg atctaggaat gctgaaagat tacccaacat accattatag 2250

tctcttcttt ctgagaaaat gtgaaaccag aattgcaaga ctgggtggac 2300

tagaaaggga gattagatca gttttctctt aatatgtcaa ggaaggtagc 2350

cgggcatggt gccaggcacc tgtaggaaaa tccagcaggt ggaggttgca 2400

gtgagccgag attatgccat tgcactccag cctgggtgac agagcgggac 2450

tccgtctc 2458

<210> 388
<211> 373
<212> PRT
<213> Homo Sapien

<400> 388
Met Ser Leu Leu Leu Leu Leu Leu Leu Val Ser Tyr Tyr Val Gly
1 5 10 15

Thr Leu Gly Thr His Thr Glu Ile Lys Arg Val Ala Glu Glu Lys
20 25 30

Val Thr Leu Pro Cys His His Gln Leu Gly Leu Pro Glu Lys Asp
35 40 45

Thr Leu Asp Ile Glu Trp Leu Leu Thr Asp Asn Glu Gly Asn Gln
50 55 60

Lys Val Val Ile Thr Tyr Ser Ser Arg His Val Tyr Asn Asn Leu
65 70 75

Thr Glu Glu Gln Lys Gly Arg Val Ala Phe Ala Ser Asn Phe Leu
80 85 90

Ala Gly Asp Ala Ser Leu Gln Ile Glu Pro Leu Lys Pro Ser Asp
95 100 105

Glu Gly Arg Tyr Thr Cys Lys Val Lys Asn Ser Gly Arg Tyr Val
110 115 120

Trp Ser His Val Ile Leu Lys Val Leu Val Arg Pro Ser Lys Pro
125 130 135

Lys Cys Glu Leu Glu Gly Glu Leu Thr Glu Gly Ser Asp Leu Thr
140 145 150

Leu Gln Cys Glu Ser Ser Ser Gly Thr Glu Pro Ile Val Tyr Tyr
155 160 165

Trp Gln Arg Ile Arg Glu Lys Glu Gly Glu Asp Glu Arg Leu Pro
170 175 180

Pro Lys Ser Arg Ile Asp Tyr Asn His Pro Gly Arg Val Leu Leu
185 190 195

Gln Asn Leu Thr Met Ser Tyr Ser Gly Leu Tyr Gln Cys Thr Ala
200 205 210

Gly Asn Glu Ala Gly Lys Glu Ser Cys Val Val Arg Val Thr Val
215 220 225

Gln Tyr Val Gln Ser Ile Gly Met Val Ala Gly Ala Val Thr Gly
230 235 240

Ile Val Ala Gly Ala Leu Leu Ile Phe Leu Leu Val Trp Leu Leu
245 250 255

Ile Arg Arg Lys Asp Lys Glu Arg Tyr Glu Glu Glu Glu Arg Pro
260 265 270

Asn Glu Ile Arg Glu Asp Ala Glu Ala Pro Lys Ala Arg Leu Val
275 280 285

Lys Pro Ser Ser Ser Ser Ser Gly Ser Arg Ser Ser Arg Ser Gly
290 295 300

Ser Ser Ser Thr Arg Ser Thr Ala Asn Ser Ala Ser Arg Ser Gln
305 310 315

Arg Thr Leu Ser Thr Asp Ala Ala Pro Gln Pro Gly Leu Ala Thr
320 325 330

Gln Ala Tyr Ser Leu Val Gly Pro Glu Val Arg Gly Ser Glu Pro
335 340 345

Lys Lys Val His His Ala Asn Leu Thr Lys Ala Glu Thr Thr Pro
350 355 360

Ser Met Ile Pro Ser Gln Ser Arg Ala Phe Gln Thr Val
365 370

<210> 389
<211> 963
<212> DNA
<213> Homo Sapien

<400> 389
gcggcacctg gaagatgcgc ccattggctg gtggcctgct caaggtggtg 50

ttcgtggtct tcgcctcctt gtgtgcctgg tattcggggt acctgctcgc 100

agagctcatt ccagatgcac ccctgtccag tgctgcctat agcatccgca 150

gcatcgggga gaggcctgtc ctcaaagctc cagtccccaa aaggcaaaaa 200

tgtgaccact ggactccctg cccatctgac acctatgcct acaggttact 250

cagcggaggt ggcagaagca agtacgccaa aatctgcttt gaggataacc 300

tacttatggg agaacagctg ggaaatgttg ccagaggaat aaacattgcc 350

attgtcaact atgtaactgg gaatgtgaca gcaacacgat gttttgatat 400

gtatgaaggc gataactctg gaccgatgac aaagtttatt cagagtgctg 450

ctccaaaatc cctgctcttc atggtgacct atgacgacgg aagcacaaga 500

ctgaataacg atgccaagaa tgccatagaa gcacttggaa gtaaagaaat 550

caggaacatg aaattcaggt ctagctgggt atttattgca gcaaaaggct 600

tggaactccc ttccgaaatt cagagagaaa agatcaacca ctctgatgct 650

aagaacaaca gatattctgg ctggcctgca gagatccaga tagaaggctg 700

catacccaaa gaacgaagct gacactgcag ggtcctgagt aaatgtgttc 750

tgtataaaca aatgcagctg gaatcgctca agaatcttat ttttctaaat 800

ccaacagccc atatttgatg agtattttgg gtttgttgta aaccaatgaa 850

catttgctag ttgtatcaaa tcttggtacg cagtattttt ataccagtat 900

tttatgtagt gaagatgtca attagcagga aactaaaatg aatggaaatt 950

cttaaaaaaa aaa 963

<210> 390
<211> 235
<212> PRT
<213> Homo Sapien

<400> 390

```
Met Arg Pro Leu Ala Gly Gly Leu Leu Lys Val Val Phe Val Val
  1               5                  10                  15

Phe Ala Ser Leu Cys Ala Trp Tyr Ser Gly Tyr Leu Leu Ala Glu
                 20                  25                  30

Leu Ile Pro Asp Ala Pro Leu Ser Ser Ala Ala Tyr Ser Ile Arg
                 35                  40                  45

Ser Ile Gly Glu Arg Pro Val Leu Lys Ala Pro Val Pro Lys Arg
                 50                  55                  60

Gln Lys Cys Asp His Trp Thr Pro Cys Pro Ser Asp Thr Tyr Ala
                 65                  70                  75

Tyr Arg Leu Leu Ser Gly Gly Gly Arg Ser Lys Tyr Ala Lys Ile
                 80                  85                  90

Cys Phe Glu Asp Asn Leu Leu Met Gly Glu Gln Leu Gly Asn Val
                 95                 100                 105

Ala Arg Gly Ile Asn Ile Ala Ile Val Asn Tyr Val Thr Gly Asn
```

110 115 120

Val Thr Ala Thr Arg Cys Phe Asp Met Tyr Glu Gly Asp Asn Ser
125 130 135

Gly Pro Met Thr Lys Phe Ile Gln Ser Ala Ala Pro Lys Ser Leu
140 145 150

Leu Phe Met Val Thr Tyr Asp Asp Gly Ser Thr Arg Leu Asn Asn
155 160 165

Asp Ala Lys Asn Ala Ile Glu Ala Leu Gly Ser Lys Glu Ile Arg
170 175 180

Asn Met Lys Phe Arg Ser Ser Trp Val Phe Ile Ala Ala Lys Gly
185 190 195

Leu Glu Leu Pro Ser Glu Ile Gln Arg Glu Lys Ile Asn His Ser
200 205 210

Asp Ala Lys Asn Asn Arg Tyr Ser Gly Trp Pro Ala Glu Ile Gln
215 220 225

Ile Glu Gly Cys Ile Pro Lys Glu Arg Ser
230 235

<210> 391
<211> 3772
<212> DNA
<213> Homo Sapien

<400> 391
gggggctttc ttgggcttgg ctgcttggaa cacctgcctc caaggaccgg 50

cctcggaggg gtcgccggga aagggaggga agaaggaagg gcggggccgg 100

ccccccctgcg cccgccccgc gcctctgcgc gcccctgtcc gccccggccc 150

agcccagccc agccccgcgg gccggtcaca cgcgcagcca gccggccgcc 200

tcccgcgccc aagcgcgccg ctctgctgtg ccctgcgccc ttgccccgcg 250

ccagcttctg cgcccgcagc ccgcccggcg cccccggtga ccgtgaccct 300

gccctgggcg cggggcggag caggcatgtc ccgcccgggg accgctaccc 350

cagcgctggc cctggtgctc ctggcagtga ccctggccgg ggtcggagcc 400

cagggcgcag ccctcgagga ccctgattat tacgggcagg agatctggag 450

ccgggagccc tactacgcgc gcccggagcc cgagctcgag accttctctc 500

cgccgctgcc tgcggggccc ggggaggagt gggagcggcg cccgcaggag 550

cccaggccgc ccaagagggc caccaagccc aagaaagctc ccaagaggga 600

gaagtcggct ccggagccgc ctccaccagg taaacacagc aacaaaaaag 650

ttatgagaac caagagctct gagaaggctg ccaacgatga tcacagtgtc 700

cgtgtggccc gtgaagatgt cagagagagt tgcccacctc ttggtctgga 750

aaccttaaaa atcacagact tccagctcca tgcctccacg gtgaagcgct 800

atggcctggg ggcacatcga gggagactca acatccaggc gggcattaat 850
gaaaatgatt tttatgacgg agcgtggtgc gcgggaagaa atgacctcca 900
gcagtggatt gaagtggatg ctcggcgcct gaccagattc actggtgtca 950
tcactcaagg gaggaactcc ctctggctga gtgactgggt gacatcctat 1000
aaggtcatgg tgagcaatga cagccacacg tgggtcactg ttaagaatgg 1050
atctggagac atgatatttg agggaaacag tgagaaggag atccctgttc 1100
tcaatgagct acccgtcccc atggtggccc gctacatccg cataaaccct 1150
cagtcctggt ttgataatgg gagcatctgc atgagaatgg agatcctggg 1200
ctgcccactg ccagatccta ataattatta tcaccgccgg aacgagatga 1250
ccaccactga tgacctggat tttaagcacc acaattataa ggaaatgcgc 1300
cagttgatga aagttgtgaa tgaaatgtgt cccaatatca ccagaattta 1350
caacattgga aaaagccacc agggcctgaa gctgtatgct gtggagatct 1400
cagatcaccc tggggagcat gaagtcggtg agcccgagtt ccactacatc 1450
gcgggggccc acggcaatga ggtgctgggc cgggagctgc tgctgctgct 1500
ggtgcagttc gtgtgtcagg agtacttggc ccggaatgcg cgcatcgtcc 1550
acctggtgga ggagacgcgg attcacgtcc tcccctccct caaccccgat 1600
ggctacgaga aggcctacga agggggctcg gagctgggag gctggtccct 1650
gggacgctgg acccacgatg gaattgacat caacaacaac tttcctgatt 1700
taaacacgct gctctgggag gcagaggatc gacagaatgt ccccaggaaa 1750
gttcccaatc actatattgc aatccctgag tggtttctgt cggaaaatgc 1800
cacggtggct gccgagacca gagcagtcat agcctggatg gaaaaaatcc 1850
cttttgtgct gggcggcaac ctgcagggcg gcgagctggt ggtggcgtat 1900
ccctacgacc tggtgcggtc cccctggaag acgcaggaac acacccccac 1950
ccccgatgac cacgtgttcc gctggctggc ctactcctat gcctccacac 2000
accgcctcat gacagacgcc cggaggaggg tgtgccacac ggaggacttc 2050
cagaaggagg agggcactgt caatggggcc tcctggcaca ccgtcgctgg 2100
aagtctgaac gatttcagct accttcatac aaactgcttc gaactgtcca 2150
tctacgtggg ctgtgataaa tacccacatg agagccagct gcccgaggag 2200
tgggagaata accgggaatc tctgatcgtg ttcatggagc aggttcatcg 2250
tggcattaaa ggcttggtga gagattcaca tggaaaagga atcccaaacg 2300
ccattatctc cgtagaaggc attaaccatg acatccgaac agccaacgat 2350
ggggattact ggcgcctcct gaaccctgga gagtatgtgg tcacagcaaa 2400

ggccgaaggt ttcactgcat ccaccaagaa ctgtatggtt ggctatgaca 2450

tgggggccac aaggtgtgac ttcacactta gcaaaaccaa catggccagg 2500

atccgagaga tcatggagaa gtttgggaag cagcccgtca gcctgccagc 2550

caggcggctg aagctgcggg ggcggaagag acgacagcgt gggtgaccct 2600

cctgggccct tgagactcgt ctgggaccca tgcaaattaa accaacctgg 2650

tagtagctcc atagtggact cactcactgt tgtttcctct gtaattcaag 2700

aagtgcctgg aagagagggt gcattgtgag gcaggtccca aaagggaagg 2750

ctggaggctg aggctgtttt cttttctttg ttcccattta tccaaataac 2800

ttggacagag cagcagagaa aagctgatgg gagtgagaga actcagcaag 2850

ccaacctggg aatcagagag agaaggagaa ggaggggagc ctgtccgttc 2900

agagcctctg gctgcataga aaaggattct ggtgcttccc ctgtttgcgt 2950

ggcagcaagg gttccacgtg catttgcaat ttgcacagct aaaattgcag 3000

catttcccca gctgggctgt cccaaatgtt accatttgag atgctcccag 3050

gcgtcctaag agaatccacc ctctctggcc ctgggacatt gcaagctgct 3100

acaaataaat tctgtgttct tttgacaata gcgtcattgc caagtgcaca 3150

tcagtgagcc tcttgaatct gtttagtctc ctttttcaac aaaggagtgt 3200

gttcagaaaa ggagagagag gctgagatca ttcaggagtt tgttgggcag 3250

caagcatgga gcttcttgca caaattctgg gtccataaac aacccccaaa 3300

gtccctgctg atccagtagc cctggaggtt ccccaggtag ggagagccag 3350

aggtgccagc cttcctgaag ggccagaaaa tttagcctgg atctcctctt 3400

ttacctgcta ggactggaaa gagccagaag tggggtggcc tgaagccctc 3450

tctctgcttg aggtattgcc cctgtgtgga attgagtgct catgggttgg 3500

cctcatatca gcctgggagt tatttttgat atgtagaatg ccagatcttc 3550

cagattaggc taaatgtaat gaaaacctct taggattatc tgtggagcat 3600

cagtttggga agaattattg aattatcttg caagaaaaaa gtatgtctca 3650

ctttttgtta atgttgctgc ctcattgacc tgggaaaaat gaaaaaaaaa 3700

aataaagcaa atggtaagac ccttaaaaaa aaaaaaaaaa aaaaaaaaaa 3750

aaaaaaaaaa aaaaaaaaaa aa 3772

<210> 392
<211> 756
<212> PRT
<213> Homo Sapien

<400> 392
Met Ser Arg Pro Gly Thr Ala Thr Pro Ala Leu Ala Leu Val Leu
1 5 10 15

Leu Ala Val Thr Leu Ala Gly Val Gly Ala Gln Gly Ala Ala Leu
20 25 30

Glu Asp Pro Asp Tyr Tyr Gly Gln Glu Ile Trp Ser Arg Glu Pro
35 40 45

Tyr Tyr Ala Arg Pro Glu Pro Glu Leu Glu Thr Phe Ser Pro Pro
50 55 60

Leu Pro Ala Gly Pro Gly Glu Glu Trp Glu Arg Arg Pro Gln Glu
65 70 75

Pro Arg Pro Pro Lys Arg Ala Thr Lys Pro Lys Lys Ala Pro Lys
80 85 90

Arg Glu Lys Ser Ala Pro Glu Pro Pro Pro Pro Gly Lys His Ser
95 100 105

Asn Lys Lys Val Met Arg Thr Lys Ser Ser Glu Lys Ala Ala Asn
110 115 120

Asp Asp His Ser Val Arg Val Ala Arg Glu Asp Val Arg Glu Ser
125 130 135

Cys Pro Pro Leu Gly Leu Glu Thr Leu Lys Ile Thr Asp Phe Gln
140 145 150

Leu His Ala Ser Thr Val Lys Arg Tyr Gly Leu Gly Ala His Arg
155 160 165

Gly Arg Leu Asn Ile Gln Ala Gly Ile Asn Glu Asn Asp Phe Tyr
170 175 180

Asp Gly Ala Trp Cys Ala Gly Arg Asn Asp Leu Gln Gln Trp Ile
185 190 195

Glu Val Asp Ala Arg Arg Leu Thr Arg Phe Thr Gly Val Ile Thr
200 205 210

Gln Gly Arg Asn Ser Leu Trp Leu Ser Asp Trp Val Thr Ser Tyr
215 220 225

Lys Val Met Val Ser Asn Asp Ser His Thr Trp Val Thr Val Lys
230 235 240

Asn Gly Ser Gly Asp Met Ile Phe Glu Gly Asn Ser Glu Lys Glu
245 250 255

Ile Pro Val Leu Asn Glu Leu Pro Val Pro Met Val Ala Arg Tyr
260 265 270

Ile Arg Ile Asn Pro Gln Ser Trp Phe Asp Asn Gly Ser Ile Cys
275 280 285

Met Arg Met Glu Ile Leu Gly Cys Pro Leu Pro Asp Pro Asn Asn
290 295 300

Tyr Tyr His Arg Arg Asn Glu Met Thr Thr Thr Asp Asp Leu Asp
305 310 315

Phe Lys His His Asn Tyr Lys Glu Met Arg Gln Leu Met Lys Val
320 325 330

Val Asn Glu Met Cys Pro Asn Ile Thr Arg Ile Tyr Asn Ile Gly
335 340 345

Lys Ser His Gln Gly Leu Lys Leu Tyr Ala Val Glu Ile Ser Asp
350 355 360

His Pro Gly Glu His Glu Val Gly Glu Pro Glu Phe His Tyr Ile
365 370 375

Ala Gly Ala His Gly Asn Glu Val Leu Gly Arg Glu Leu Leu Leu
380 385 390

Leu Leu Val Gln Phe Val Cys Gln Glu Tyr Leu Ala Arg Asn Ala
395 400 405

Arg Ile Val His Leu Val Glu Glu Thr Arg Ile His Val Leu Pro
410 415 420

Ser Leu Asn Pro Asp Gly Tyr Glu Lys Ala Tyr Glu Gly Gly Ser
425 430 435

Glu Leu Gly Gly Trp Ser Leu Gly Arg Trp Thr His Asp Gly Ile
440 445 450

Asp Ile Asn Asn Asn Phe Pro Asp Leu Asn Thr Leu Leu Trp Glu
455 460 465

Ala Glu Asp Arg Gln Asn Val Pro Arg Lys Val Pro Asn His Tyr
470 475 480

Ile Ala Ile Pro Glu Trp Phe Leu Ser Glu Asn Ala Thr Val Ala
485 490 495

Ala Glu Thr Arg Ala Val Ile Ala Trp Met Glu Lys Ile Pro Phe
500 505 510

Val Leu Gly Gly Asn Leu Gln Gly Gly Glu Leu Val Val Ala Tyr
515 520 525

Pro Tyr Asp Leu Val Arg Ser Pro Trp Lys Thr Gln Glu His Thr
530 535 540

Pro Thr Pro Asp Asp His Val Phe Arg Trp Leu Ala Tyr Ser Tyr
545 550 555

Ala Ser Thr His Arg Leu Met Thr Asp Ala Arg Arg Arg Val Cys
560 565 570

His Thr Glu Asp Phe Gln Lys Glu Glu Gly Thr Val Asn Gly Ala
575 580 585

Ser Trp His Thr Val Ala Gly Ser Leu Asn Asp Phe Ser Tyr Leu
590 595 600

His Thr Asn Cys Phe Glu Leu Ser Ile Tyr Val Gly Cys Asp Lys
605 610 615

Tyr Pro His Glu Ser Gln Leu Pro Glu Glu Trp Glu Asn Asn Arg
620 625 630

Glu Ser Leu Ile Val Phe Met Glu Gln Val His Arg Gly Ile Lys
635 640 645

Gly Leu Val Arg Asp Ser His Gly Lys Gly Ile Pro Asn Ala Ile

650 655 660

Ile Ser Val Glu Gly Ile Asn His Asp Ile Arg Thr Ala Asn Asp
665 670 675

Gly Asp Tyr Trp Arg Leu Leu Asn Pro Gly Glu Tyr Val Val Thr
680 685 690

Ala Lys Ala Glu Gly Phe Thr Ala Ser Thr Lys Asn Cys Met Val
695 700 705

Gly Tyr Asp Met Gly Ala Thr Arg Cys Asp Phe Thr Leu Ser Lys
710 715 720

Thr Asn Met Ala Arg Ile Arg Glu Ile Met Glu Lys Phe Gly Lys
725 730 735

Gln Pro Val Ser Leu Pro Ala Arg Arg Leu Lys Leu Arg Gly Arg
740 745 750

Lys Arg Arg Gln Arg Gly
755

<210> 393
<211> 4313
<212> DNA
<213> Homo Sapien

<400> 393
gtcccacatc ctgctcaact gggtcaggtc cctcttagac cagctcttgt 50

ccatcatttg ctgaagtgga ccaactagtt ccccagtagg gggtctcccc 100

tggcaattct tgatcggcgt ttggacatct cagatcgctt ccaatgaaga 150

tggccttgcc ttggggtcct gcttgtttca taatcatcta actatgggac 200

aaggttgtgc cggcagctct gggggaagga gcacggggct gatcaagcca 250

tccaggaaac actggaggac ttgtccagcc ttgaaagaac tctagtggtt 300

tctgaatcta gcccacttgg cggtaagcat gatgcaactt ctgcaacttc 350

tgctggggct tttggggcca ggtggctact tatttctttt aggggattgt 400

caggaggtga ccactctcac ggtgaaatac caagtgtcag aggaagtgcc 450

atctggtaca gtgatcggga agctgtccca ggaactgggc cgggaggaga 500

ggcggaggca agctggggcc gccttccagg tgttgcagct gcctcaggcg 550

ctccccattc aggtggactc tgaggaaggc ttgctcagca caggcaggcg 600

gctggatcga gagcagctgt gccgacagtg ggatccctgc ctggtttcct 650

ttgatgtgct tgccacaggg gatttggctc tgatccatgt ggagatccaa 700

gtgctggaca tcaatgacca ccagccacgg tttcccaaag gcgagcagga 750

gctggaaatc tctgagagcg cctctctgcg aacccggatc cccctggaca 800

gagctcttga cccagacaca ggccctaaca ccctgcacac ctacactctg 850

tctcccagtg agcactttgc cttggatgtc attgtgggcc ctgatgagac 900

caaacatgca gaactcatag tggtgaagga gctggacagg gaaatccatt 950
catttttga tctggtgtta actgcctatg acaatgggaa cccccccaag 1000
tcaggtacca gcttggtcaa ggtcaacgtc ttggactcca atgacaatag 1050
ccctgcgttt gctgagagtt cactggcact ggaaatccaa gaagatgctg 1100
cacctggtac gcttctcata aaactgaccg ccacagaccc tgaccaaggc 1150
cccaatgggg aggtggagtt cttcctcagt aagcacatgc ctccagaggt 1200
gctggacacc ttcagtattg atgccaagac aggccaggtc attctgcgtc 1250
gacctctaga ctatgaaaag aaccctgcct acgaggtgga tgttcaggca 1300
agggacctgg gtcccaatcc tatcccagcc cattgcaaag ttctcatcaa 1350
ggttctggat gtcaatgaca acatcccaag catccacgtc acatgggcct 1400
cccagccatc actggtgtca gaagctcttc ccaaggacag ttttattgct 1450
cttgtcatgg cagatgactt ggattcagga cacaatggtt tggtccactg 1500
ctggctgagc caagagctgg gccacttcag gctgaaaaga actaatggca 1550
acacatacat gttgctaacc aatgccacac tggacagaga gcagtggccc 1600
aaatataccc tcactctgtt agcccaagac caaggactcc agcccttatc 1650
agccaagaaa cagctcagca ttcagatcag tgacatcaac gacaatgcac 1700
ctgtgtttga gaaaagcagg tatgaagtct ccacgcggga aaacaactta 1750
ccctctcttc acctcattac catcaaggct catgatgcag acttgggcat 1800
taatggaaaa gtctcatacc gcatccagga ctccccagtt gctcacttag 1850
tagctattga ctccaacaca ggagaggtca ctgctcagag gtcactgaac 1900
tatgaagaga tggccggctt tgagttccag gtgatcgcag aggacagcgg 1950
gcaacccatg cttgcatcca gtgtctctgt gtgggtcagc ctcttggatg 2000
ccaatgataa tgccccagag gtggtccagc ctgtgctcag cgatggaaaa 2050
gccagcctct ccgtgcttgt gaatgcctcc acaggccacc tgctggtgcc 2100
catcgagact cccaatggct tgggcccagc gggcactgac acacctccac 2150
tggccactca cagctcccgg ccattccttt tgacaaccat tgtggcaaga 2200
gatgcagact cgggggcaaa tggagagccc ctctacagca tccgcaatgg 2250
aaatgaagcc cacctcttca tcctcaaccc tcatacgggg cagctgttcg 2300
tcaatgtcac caatgccagc agcctcattg ggagtgagtg ggagctggag 2350
atagtagtag aggaccaggg aagccccccc ttacagaccc gagccctgtt 2400
gagggtcatg tttgtcacca gtgtggacca cctgagggac tcagcccgca 2450
agcctggggc cttgagcatg tcgatgctga cggtgatctg cctggctgta 2500

ctgttgggca tcttcgggtt gatcctggct ttgttcatgt ccatctgccg 2550
gacagaaaag aaggacaaca gggcctacaa ctgtcgggag gccgagtcca 2600
cctaccgcca gcagcccaag aggccccaga aacacattca gaaggcagac 2650
atccacctcg tgcctgtgct caggggtcag gcaggtgagc cttgtgaagt 2700
cgggcagtcc cacaaagatg tggacaagga ggcgatgatg gaagcaggct 2750
gggacccctg cctgcaggcc cccttccacc tcaccccgac cctgtacagg 2800
acgctgcgta atcaaggcaa ccagggagca ccggcggaga gccgagaggt 2850
gctgcaagac acggtcaacc tccttttcaa ccatcccagg cagaggaatg 2900
cctcccggga gaacctgaac cttcccgagc cccagcctgc cacaggccag 2950
ccacgttcca ggcctctgaa ggttgcaggc agccccacag ggaggctggc 3000
tggagaccag ggcagtgagg aagccccaca gaggccacca gcctcctctg 3050
caaccctgag acggcagcga catctcaatg gcaaagtgtc ccctgagaaa 3100
gaatcagggc cccgtcagat cctgcggagc ctggtccggc tgtctgtggc 3150
tgccttcgcc gagcggaacc ccgtggagga gctcactgtg gattctcctc 3200
ctgttcagca aatctcccag ctgctgtcct tgctgcatca gggccaattc 3250
cagcccaaac caaaccaccg aggaaataag tacttggcca agccaggagg 3300
cagcaggagt gcaatcccag acacagatgg cccaagtgca agggctggag 3350
gccagacaga cccagaacag gaggaagggc ctttggatcc tgaagaggac 3400
ctctctgtga agcaactgct agaagaagag ctgtcaagtc tgctggaccc 3450
cagcacaggt ctggccctgg accggctgag cgcccctgac ccggcctgga 3500
tggcgagact ctctttgccc ctcaccacca actaccgtga caatgtgatc 3550
tccccggatg ctgcagccac ggaggagccg aggaccttcc agacgttcgg 3600
caaggcagag gcaccagagc tgagcccaac aggcacgagg ctggccagca 3650
cctttgtctc ggagatgagc tcactgctgg agatgctgct ggaacagcgc 3700
tccagcatgc ccgtggaggc cgcctccgag gcgctgcggc ggctctcggt 3750
ctgcgggagg accctcagtt tagacttggc caccagtgca gcctcaggca 3800
tgaaagtgca aggggaccca ggtggaaaga cggggactga gggcaagagc 3850
agaggcagca gcagcagcag caggtgcctg tgaacatacc tcagacgcct 3900
ctggatccaa gaaccagggg cctgaggatc tgtggacaag agctggtttc 3950
taaaatcttg taactcacta gctagcggcg gcctgagaac tttagggtga 4000
ctgatgctac ccccacagag gaggcaagag ccccaggact aacagctgac 4050
tgaccaaagc agccccttgt aagcagctct gagtcttttg gaggacaggg 4100

acggtttgtg gctgagataa gtgtttcctg gcaaaacata tgtggagcac 4150

aaagggtcag tcctctggca gaacagatgc cacggagtat cacaggcagg 4200

aaagggtggc cttcttgggt agcaggagtc agggggctgt accctggggg 4250

tgccaggaaa tgctctctga cctatcaata aaggaaaagc agtaaaaaaa 4300

aaaaaaaaaa aaa 4313

<210> 394
<211> 1184
<212> PRT
<213> Homo Sapien

<400> 394
Met Met Gln Leu Leu Gln Leu Leu Leu Gly Leu Leu Gly Pro Gly
1               5                   10                  15

Gly Tyr Leu Phe Leu Leu Gly Asp Cys Gln Glu Val Thr Thr Leu
                20                  25                  30

Thr Val Lys Tyr Gln Val Ser Glu Glu Val Pro Ser Gly Thr Val
                35                  40                  45

Ile Gly Lys Leu Ser Gln Glu Leu Gly Arg Glu Glu Arg Arg Arg
                50                  55                  60

Gln Ala Gly Ala Ala Phe Gln Val Leu Gln Leu Pro Gln Ala Leu
                65                  70                  75

Pro Ile Gln Val Asp Ser Glu Glu Gly Leu Leu Ser Thr Gly Arg
                80                  85                  90

Arg Leu Asp Arg Glu Gln Leu Cys Arg Gln Trp Asp Pro Cys Leu
                95                  100                 105

Val Ser Phe Asp Val Leu Ala Thr Gly Asp Leu Ala Leu Ile His
                110                 115                 120

Val Glu Ile Gln Val Leu Asp Ile Asn Asp His Gln Pro Arg Phe
                125                 130                 135

Pro Lys Gly Glu Gln Glu Leu Glu Ile Ser Glu Ser Ala Ser Leu
                140                 145                 150

Arg Thr Arg Ile Pro Leu Asp Arg Ala Leu Asp Pro Asp Thr Gly
                155                 160                 165

Pro Asn Thr Leu His Thr Tyr Thr Leu Ser Pro Ser Glu His Phe
                170                 175                 180

Ala Leu Asp Val Ile Val Gly Pro Asp Glu Thr Lys His Ala Glu
                185                 190                 195

Leu Ile Val Val Lys Glu Leu Asp Arg Glu Ile His Ser Phe Phe
                200                 205                 210

Asp Leu Val Leu Thr Ala Tyr Asp Asn Gly Asn Pro Pro Lys Ser
                215                 220                 225

Gly Thr Ser Leu Val Lys Val Asn Val Leu Asp Ser Asn Asp Asn
                230                 235                 240

Ser Pro Ala Phe Ala Glu Ser Ser Leu Ala Leu Glu Ile Gln Glu
245 250 255

Asp Ala Ala Pro Gly Thr Leu Leu Ile Lys Leu Thr Ala Thr Asp
260 265 270

Pro Asp Gln Gly Pro Asn Gly Glu Val Glu Phe Phe Leu Ser Lys
275 280 285

His Met Pro Pro Glu Val Leu Asp Thr Phe Ser Ile Asp Ala Lys
290 295 300

Thr Gly Gln Val Ile Leu Arg Arg Pro Leu Asp Tyr Glu Lys Asn
305 310 315

Pro Ala Tyr Glu Val Asp Val Gln Ala Arg Asp Leu Gly Pro Asn
320 325 330

Pro Ile Pro Ala His Cys Lys Val Leu Ile Lys Val Leu Asp Val
335 340 345

Asn Asp Asn Ile Pro Ser Ile His Val Thr Trp Ala Ser Gln Pro
350 355 360

Ser Leu Val Ser Glu Ala Leu Pro Lys Asp Ser Phe Ile Ala Leu
365 370 375

Val Met Ala Asp Asp Leu Asp Ser Gly His Asn Gly Leu Val His
380 385 390

Cys Trp Leu Ser Gln Glu Leu Gly His Phe Arg Leu Lys Arg Thr
395 400 405

Asn Gly Asn Thr Tyr Met Leu Leu Thr Asn Ala Thr Leu Asp Arg
410 415 420

Glu Gln Trp Pro Lys Tyr Thr Leu Thr Leu Leu Ala Gln Asp Gln
425 430 435

Gly Leu Gln Pro Leu Ser Ala Lys Lys Gln Leu Ser Ile Gln Ile
440 445 450

Ser Asp Ile Asn Asp Asn Ala Pro Val Phe Glu Lys Ser Arg Tyr
455 460 465

Glu Val Ser Thr Arg Glu Asn Asn Leu Pro Ser Leu His Leu Ile
470 475 480

Thr Ile Lys Ala His Asp Ala Asp Leu Gly Ile Asn Gly Lys Val
485 490 495

Ser Tyr Arg Ile Gln Asp Ser Pro Val Ala His Leu Val Ala Ile
500 505 510

Asp Ser Asn Thr Gly Glu Val Thr Ala Gln Arg Ser Leu Asn Tyr
515 520 525

Glu Glu Met Ala Gly Phe Glu Phe Gln Val Ile Ala Glu Asp Ser
530 535 540

Gly Gln Pro Met Leu Ala Ser Ser Val Ser Val Trp Val Ser Leu
545 550 555

```
Leu Asp Ala Asn Asp Asn Ala Pro Glu Val Val Gln Pro Val Leu
                560                 565                 570

Ser Asp Gly Lys Ala Ser Leu Ser Val Leu Val Asn Ala Ser Thr
                575                 580                 585

Gly His Leu Leu Val Pro Ile Glu Thr Pro Asn Gly Leu Gly Pro
                590                 595                 600

Ala Gly Thr Asp Thr Pro Pro Leu Ala Thr His Ser Ser Arg Pro
                605                 610                 615

Phe Leu Leu Thr Thr Ile Val Ala Arg Asp Ala Asp Ser Gly Ala
                620                 625                 630

Asn Gly Glu Pro Leu Tyr Ser Ile Arg Asn Gly Asn Glu Ala His
                635                 640                 645

Leu Phe Ile Leu Asn Pro His Thr Gly Gln Leu Phe Val Asn Val
                650                 655                 660

Thr Asn Ala Ser Ser Leu Ile Gly Ser Glu Trp Glu Leu Glu Ile
                665                 670                 675

Val Val Glu Asp Gln Gly Ser Pro Pro Leu Gln Thr Arg Ala Leu
                680                 685                 690

Leu Arg Val Met Phe Val Thr Ser Val Asp His Leu Arg Asp Ser
                695                 700                 705

Ala Arg Lys Pro Gly Ala Leu Ser Met Ser Met Leu Thr Val Ile
                710                 715                 720

Cys Leu Ala Val Leu Leu Gly Ile Phe Gly Leu Ile Leu Ala Leu
                725                 730                 735

Phe Met Ser Ile Cys Arg Thr Glu Lys Lys Asp Asn Arg Ala Tyr
                740                 745                 750

Asn Cys Arg Glu Ala Glu Ser Thr Tyr Arg Gln Gln Pro Lys Arg
                755                 760                 765

Pro Gln Lys His Ile Gln Lys Ala Asp Ile His Leu Val Pro Val
                770                 775                 780

Leu Arg Gly Gln Ala Gly Glu Pro Cys Glu Val Gly Gln Ser His
                785                 790                 795

Lys Asp Val Asp Lys Glu Ala Met Met Glu Ala Gly Trp Asp Pro
                800                 805                 810

Cys Leu Gln Ala Pro Phe His Leu Thr Pro Thr Leu Tyr Arg Thr
                815                 820                 825

Leu Arg Asn Gln Gly Asn Gln Gly Ala Pro Ala Glu Ser Arg Glu
                830                 835                 840

Val Leu Gln Asp Thr Val Asn Leu Leu Phe Asn His Pro Arg Gln
                845                 850                 855

Arg Asn Ala Ser Arg Glu Asn Leu Asn Leu Pro Glu Pro Gln Pro
                860                 865                 870

Ala Thr Gly Gln Pro Arg Ser Arg Pro Leu Lys Val Ala Gly Ser
```

875 880 885

Pro Thr Gly Arg Leu Ala Gly Asp Gln Gly Ser Glu Glu Ala Pro
890 895 900

Gln Arg Pro Pro Ala Ser Ser Ala Thr Leu Arg Arg Gln Arg His
905 910 915

Leu Asn Gly Lys Val Ser Pro Glu Lys Glu Ser Gly Pro Arg Gln
920 925 930

Ile Leu Arg Ser Leu Val Arg Leu Ser Val Ala Ala Phe Ala Glu
935 940 945

Arg Asn Pro Val Glu Glu Leu Thr Val Asp Ser Pro Pro Val Gln
950 955 960

Gln Ile Ser Gln Leu Leu Ser Leu Leu His Gln Gly Gln Phe Gln
965 970 975

Pro Lys Pro Asn His Arg Gly Asn Lys Tyr Leu Ala Lys Pro Gly
980 985 990

Gly Ser Arg Ser Ala Ile Pro Asp Thr Asp Gly Pro Ser Ala Arg
995 1000 1005

Ala Gly Gly Gln Thr Asp Pro Glu Gln Glu Glu Gly Pro Leu Asp
1010 1015 1020

Pro Glu Glu Asp Leu Ser Val Lys Gln Leu Leu Glu Glu Glu Leu
1025 1030 1035

Ser Ser Leu Leu Asp Pro Ser Thr Gly Leu Ala Leu Asp Arg Leu
1040 1045 1050

Ser Ala Pro Asp Pro Ala Trp Met Ala Arg Leu Ser Leu Pro Leu
1055 1060 1065

Thr Thr Asn Tyr Arg Asp Asn Val Ile Ser Pro Asp Ala Ala Ala
1070 1075 1080

Thr Glu Glu Pro Arg Thr Phe Gln Thr Phe Gly Lys Ala Glu Ala
1085 1090 1095

Pro Glu Leu Ser Pro Thr Gly Thr Arg Leu Ala Ser Thr Phe Val
1100 1105 1110

Ser Glu Met Ser Ser Leu Leu Glu Met Leu Leu Glu Gln Arg Ser
1115 1120 1125

Ser Met Pro Val Glu Ala Ala Ser Glu Ala Leu Arg Arg Leu Ser
1130 1135 1140

Val Cys Gly Arg Thr Leu Ser Leu Asp Leu Ala Thr Ser Ala Ala
1145 1150 1155

Ser Gly Met Lys Val Gln Gly Asp Pro Gly Gly Lys Thr Gly Thr
1160 1165 1170

Glu Gly Lys Ser Arg Gly Ser Ser Ser Ser Ser Arg Cys Leu
1175 1180

<210> 395
<211> 999

<212> DNA
<213> Homo Sapien

<400> 395
cccaggctct agtgcaggag gagaaggagg aggagcagga ggtggagatt 50

cccagttaaa aggctccaga atcgtgtacc aggcagagaa ctgaagtact 100

ggggcctcct ccactgggtc cgaatcagta ggtgaccccg cccctggatt 150

ctggaagacc tcaccatggg acgcccccga cctcgtgcgg ccaagacgtg 200

gatgttcctg ctcttgctgg ggggagcctg ggcaggacac tccagggcac 250

aggaggacaa ggtgctgggg ggtcatgagt gccaacccca ttcgcagcct 300

tggcaggcgg ccttgttcca gggccagcaa ctactctgtg gcggtgtcct 350

tgtaggtggc aactgggtcc ttacagctgc ccactgtaaa aaaccgaaat 400

acacagtacg cctgggagac cacagcctac agaataaaga tggcccagag 450

caagaaatac ctgtggttca gtccatccca cacccctgct acaacagcag 500

cgatgtggag gaccacaacc atgatctgat gcttcttcaa ctgcgtgacc 550

aggcatccct ggggtccaaa gtgaagccca tcagcctggc agatcattgc 600

acccagcctg gccagaagtg caccgtctca ggctggggca ctgtcaccag 650

tccccgagag aattttcctg acactctcaa ctgtgcagaa gtaaaaatct 700

ttccccagaa gaagtgtgag gatgcttacc cggggcagat cacagatggc 750

atggtctgtg caggcagcag caaaggggct gacacgtgcc agggcgattc 800

tggaggcccc ctggtgtgtg atggtgcact ccagggcatc acatcctggg 850

gctcagaccc ctgtgggagg tccgacaaac ctggcgtcta taccaacatc 900

tgccgctacc tggactggat caagaagatc ataggcagca agggctgatt 950

ctaggataag cactagatct cccttaataa actcacaact ctctggttc 999

<210> 396
<211> 260
<212> PRT
<213> Homo Sapien

<400> 396
Met Gly Arg Pro Arg Pro Arg Ala Ala Lys Thr Trp Met Phe Leu
1 5 10 15

Leu Leu Leu Gly Gly Ala Trp Ala Gly His Ser Arg Ala Gln Glu
20 25 30

Asp Lys Val Leu Gly Gly His Glu Cys Gln Pro His Ser Gln Pro
35 40 45

Trp Gln Ala Ala Leu Phe Gln Gly Gln Gln Leu Leu Cys Gly Gly
50 55 60

Val Leu Val Gly Gly Asn Trp Val Leu Thr Ala Ala His Cys Lys
65 70 75

Lys Pro Lys Tyr Thr Val Arg Leu Gly Asp His Ser Leu Gln Asn
80 85 90

Lys Asp Gly Pro Glu Gln Glu Ile Pro Val Val Gln Ser Ile Pro
95 100 105

His Pro Cys Tyr Asn Ser Ser Asp Val Glu Asp His Asn His Asp
110 115 120

Leu Met Leu Leu Gln Leu Arg Asp Gln Ala Ser Leu Gly Ser Lys
125 130 135

Val Lys Pro Ile Ser Leu Ala Asp His Cys Thr Gln Pro Gly Gln
140 145 150

Lys Cys Thr Val Ser Gly Trp Gly Thr Val Thr Ser Pro Arg Glu
155 160 165

Asn Phe Pro Asp Thr Leu Asn Cys Ala Glu Val Lys Ile Phe Pro
170 175 180

Gln Lys Lys Cys Glu Asp Ala Tyr Pro Gly Gln Ile Thr Asp Gly
185 190 195

Met Val Cys Ala Gly Ser Ser Lys Gly Ala Asp Thr Cys Gln Gly
200 205 210

Asp Ser Gly Gly Pro Leu Val Cys Asp Gly Ala Leu Gln Gly Ile
215 220 225

Thr Ser Trp Gly Ser Asp Pro Cys Gly Arg Ser Asp Lys Pro Gly
230 235 240

Val Tyr Thr Asn Ile Cys Arg Tyr Leu Asp Trp Ile Lys Lys Ile
245 250 255

Ile Gly Ser Lys Gly
260

<210> 397
<211> 1312
<212> DNA
<213> Homo Sapien

<400> 397
ggcggctgct gagctgcctt gaggtgcagt gttggggatc cagagccatg 50

tcggacctgc tactactggg cctgattggg ggcctgactc tcttactgct 100

gctgacgctg ctggcctttg ccgggtactc agggctactg gctggggtgg 150

aagtgagtgc tgggtcaccc cccatccgca acgtcactgt ggcctacaag 200

ttccacatgg ggctctatgg tgagactggg cggcttttca ctgagagctg 250

cagcatctct cccaagctcc gctccatcgc tgtctactat gacaaccccc 300

acatggtgcc ccctgataag tgccgatgtg ccgtgggcag catcctgagt 350

gaaggtgagg aatcgccctc ccctgagctc atcgacctct accagaaatt 400

tggcttcaag gtgttctcct tcccggcacc cagccatgtg gtgacagcca 450

ccttccccta caccaccatt ctgtccatct ggctggctac ccgccgtgtc 500

catcctgcct tggacaccta catcaaggag cggaagctgt gtgcctatcc 550

tcggctggag atctaccagg aagaccagat ccatttcatg tgcccactgg 600

cacggcaggg agacttctat gtgcctgaga tgaaggagac agagtggaaa 650

tggcgggggc ttgtggaggc cattgacacc caggtggatg gcacaggagc 700

tgacacaatg agtgacacga gttctgtaag cttggaagtg agccctggca 750

gccgggagac ttcagctgcc acactgtcac ctggggcgag cagccgtggc 800

tgggatgacg gtgacacccg cagcgagcac agctacagcg agtcaggtgc 850

cagcggctcc tcttttgagg agctggactt ggagggcgag gggcccttag 900

gggagtcacg gctggaccct gggactgagc ccctggggac taccaagtgg 950

ctctgggagc ccactgcccc tgagaagggc aaggagtaac ccatggcctg 1000

caccctcctg cagtgcagtt gctgaggaac tgagcagact ctccagcaga 1050

ctctccagcc ctcttcctcc ttcctctggg ggaggagggg ttcctgaggg 1100

acctgacttc ccctgctcca ggcctcttgc taagccttct cctcactgcc 1150

ctttaggctc ccagggccag aggagccagg gactattttc tgcaccagcc 1200

cccagggctg ccgcccctgt tgtgtctttt tttcagactc acagtggagc 1250

ttccaggacc cagaataaag ccaatgattt acttgtttca cctggaaaaa 1300

aaaaaaaaaa aa 1312

<210> 398
<211> 313
<212> PRT
<213> Homo Sapien

<400> 398
Met Ser Asp Leu Leu Leu Leu Gly Leu Ile Gly Gly Leu Thr Leu
1 5 10 15

Leu Leu Leu Leu Thr Leu Leu Ala Phe Ala Gly Tyr Ser Gly Leu
20 25 30

Leu Ala Gly Val Glu Val Ser Ala Gly Ser Pro Pro Ile Arg Asn
35 40 45

Val Thr Val Ala Tyr Lys Phe His Met Gly Leu Tyr Gly Glu Thr
50 55 60

Gly Arg Leu Phe Thr Glu Ser Cys Ser Ile Ser Pro Lys Leu Arg
65 70 75

Ser Ile Ala Val Tyr Tyr Asp Asn Pro His Met Val Pro Pro Asp
80 85 90

Lys Cys Arg Cys Ala Val Gly Ser Ile Leu Ser Glu Gly Glu Glu
95 100 105

Ser Pro Ser Pro Glu Leu Ile Asp Leu Tyr Gln Lys Phe Gly Phe

110 115 120

Lys Val Phe Ser Phe Pro Ala Pro Ser His Val Val Thr Ala Thr
125 130 135

Phe Pro Tyr Thr Thr Ile Leu Ser Ile Trp Leu Ala Thr Arg Arg
140 145 150

Val His Pro Ala Leu Asp Thr Tyr Ile Lys Glu Arg Lys Leu Cys
155 160 165

Ala Tyr Pro Arg Leu Glu Ile Tyr Gln Glu Asp Gln Ile His Phe
170 175 180

Met Cys Pro Leu Ala Arg Gln Gly Asp Phe Tyr Val Pro Glu Met
185 190 195

Lys Glu Thr Glu Trp Lys Trp Arg Gly Leu Val Glu Ala Ile Asp
200 205 210

Thr Gln Val Asp Gly Thr Gly Ala Asp Thr Met Ser Asp Thr Ser
215 220 225

Ser Val Ser Leu Glu Val Ser Pro Gly Ser Arg Glu Thr Ser Ala
230 235 240

Ala Thr Leu Ser Pro Gly Ala Ser Ser Arg Gly Trp Asp Asp Gly
245 250 255

Asp Thr Arg Ser Glu His Ser Tyr Ser Glu Ser Gly Ala Ser Gly
260 265 270

Ser Ser Phe Glu Glu Leu Asp Leu Glu Gly Glu Gly Pro Leu Gly
275 280 285

Glu Ser Arg Leu Asp Pro Gly Thr Glu Pro Leu Gly Thr Thr Lys
290 295 300

Trp Leu Trp Glu Pro Thr Ala Pro Glu Lys Gly Lys Glu
305 310

<210> 399
<211> 1510
<212> DNA
<213> Homo Sapien

<400> 399
ggacgagggc agatctcgtt ctggggcaag ccgttgacac tcgctccctg 50

ccaccgcccg ggctccgtgc cgccaagttt tcattttcca ccttctctgc 100

ctccagtccc ccagcccctg gccgagagaa gggtcttacc ggccgggatt 150

gctggaaaca ccaagaggtg gtttttgttt tttaaaactt ctgtttcttg 200

ggagggggtg tggcggggca ggatgagcaa ctccgttcct ctgctctgtt 250

tctggagcct ctgctattgc tttgctgcgg ggagccccgt accttttggt 300

ccagagggac ggctggaaga taagctccac aaacccaaag ctacacagac 350

tgaggtcaaa ccatctgtga ggtttaacct ccgcacctcc aaggacccag 400

agcatgaagg atgctacctc tccgtcggcc acagccagcc cttagaagac 450

tgcagtttca acatgacagc taaaaccttt ttcatcattc acggatggac 500

gatgagcggt atctttgaaa actggctgca caaactcgtg tcagccctgc 550

acacaagaga gaaagacgcc aatgtagttg tggttgactg gctccccctg 600

gcccaccagc tttacacgga tgcggtcaat aataccaggg tggtgggaca 650

cagcattgcc aggatgctcg actggctgca ggagaaggac gatttttctc 700

tcgggaatgt ccacttgatc ggctacagcc tcggagcgca cgtggccggg 750

tatgcaggca acttcgtgaa aggaacggtg ggccgaatca caggtttgga 800

tcctgccggg cccatgtttg aaggggccga catccacaag aggctctctc 850

cggacgatgc agattttgtg gatgtcctcc acacctacac gcgttccttc 900

ggcttgagca ttggtattca gatgcctgtg ggccacattg acatctaccc 950

caatgggggt gacttccagc caggctgtgg actcaacgat gtcttgggat 1000

caattgcata tggaacaatc acagaggtgg taaaatgtga gcatgagcga 1050

gccgtccacc tctttgttga ctctctggtg aatcaggaca agccgagttt 1100

tgccttccag tgcactgact ccaatcgctt caaaaagggg atctgtctga 1150

gctgccgcaa gaaccgttgt aatagcattg gctacaatgc caagaaaatg 1200

aggaacaaga ggaacagcaa aatgtaccta aaaacccggg caggcatgcc 1250

tttcagaggt aaccttcagt ccctggagtg tccctgagga aggcccttaa 1300

tacctccttc ttaataccat gctgcagagc agggcacatc ctagcccagg 1350

agaagtggcc agcacaatcc aatcaaatcg ttgcaaatca gattacactg 1400

tgcatgtcct aggaaaggga atctttacaa aataaacagt gtggacccct 1450

aataaaaaaa aaaaaaaaaa aaaaaaaaaa aaaaaaaaaa aaaaaaaaaa 1500

aaaaaaaaaa 1510

<210> 400
<211> 354
<212> PRT
<213> Homo Sapien

<400> 400

```
Met Ser Asn Ser Val Pro Leu Leu Cys Phe Trp Ser Leu Cys Tyr
  1               5                  10                  15

Cys Phe Ala Ala Gly Ser Pro Val Pro Phe Gly Pro Glu Gly Arg
                 20                  25                  30

Leu Glu Asp Lys Leu His Lys Pro Lys Ala Thr Gln Thr Glu Val
                 35                  40                  45

Lys Pro Ser Val Arg Phe Asn Leu Arg Thr Ser Lys Asp Pro Glu
                 50                  55                  60

His Glu Gly Cys Tyr Leu Ser Val Gly His Ser Gln Pro Leu Glu
```

```
                65                  70                  75

Asp Cys Ser Phe Asn Met Thr Ala Lys Thr Phe Phe Ile Ile His
                80                  85                  90

Gly Trp Thr Met Ser Gly Ile Phe Glu Asn Trp Leu His Lys Leu
                95                  100                 105

Val Ser Ala Leu His Thr Arg Glu Lys Asp Ala Asn Val Val Val
                110                 115                 120

Val Asp Trp Leu Pro Leu Ala His Gln Leu Tyr Thr Asp Ala Val
                125                 130                 135

Asn Asn Thr Arg Val Val Gly His Ser Ile Ala Arg Met Leu Asp
                140                 145                 150

Trp Leu Gln Glu Lys Asp Asp Phe Ser Leu Gly Asn Val His Leu
                155                 160                 165

Ile Gly Tyr Ser Leu Gly Ala His Val Ala Gly Tyr Ala Gly Asn
                170                 175                 180

Phe Val Lys Gly Thr Val Gly Arg Ile Thr Gly Leu Asp Pro Ala
                185                 190                 195

Gly Pro Met Phe Glu Gly Ala Asp Ile His Lys Arg Leu Ser Pro
                200                 205                 210

Asp Asp Ala Asp Phe Val Asp Val Leu His Thr Tyr Thr Arg Ser
                215                 220                 225

Phe Gly Leu Ser Ile Gly Ile Gln Met Pro Val Gly His Ile Asp
                230                 235                 240

Ile Tyr Pro Asn Gly Gly Asp Phe Gln Pro Gly Cys Gly Leu Asn
                245                 250                 255

Asp Val Leu Gly Ser Ile Ala Tyr Gly Thr Ile Thr Glu Val Val
                260                 265                 270

Lys Cys Glu His Glu Arg Ala Val His Leu Phe Val Asp Ser Leu
                275                 280                 285

Val Asn Gln Asp Lys Pro Ser Phe Ala Phe Gln Cys Thr Asp Ser
                290                 295                 300

Asn Arg Phe Lys Lys Gly Ile Cys Leu Ser Cys Arg Lys Asn Arg
                305                 310                 315

Cys Asn Ser Ile Gly Tyr Asn Ala Lys Lys Met Arg Asn Lys Arg
                320                 325                 330

Asn Ser Lys Met Tyr Leu Lys Thr Arg Ala Gly Met Pro Phe Arg
                335                 340                 345

Gly Asn Leu Gln Ser Leu Glu Cys Pro
                350
```

<210> 401
<211> 584
<212> DNA
<213> Homo Sapien

<400> 401
cttcccagcc ctgtgcccca aagcacctgg agcatatagc cttgcagaac 50

ttctacttgc ctgcctccct gcctctggcc atggcctgcc ggtgcctcag 100

cttccttctg atggggacct tcctgtcagt ttcccagaca gtcctggccc 150

agctggatgc actgctggtc ttcccaggcc aagtggctca actctcctgc 200

acgctcagcc cccagcacgt caccatcagg gactacggtg tgtcctggta 250

ccagcagcgg gcaggcagtg cccctcgata tctcctctac taccgctcgg 300

aggaggatca ccaccggcct gctgacatcc ccgatcgatt ctcggcagcc 350

aaggatgagg cccacaatgc ctgtgtcctc accattagtc ccgtgcagcc 400

tgaagacgac gcggattact actgctctgt tggctacggc tttagtccct 450

aggggtgggg tgtgagatgg gtgcctcccc tctgcctccc atttctgccc 500

ctgaccttgg gtccctttta aactttctct gagccttgct tcccctctgt 550

aaaatgggtt aataatattc aacatgtcaa caac 584

<210> 402
<211> 123
<212> PRT
<213> Homo Sapien

<400> 402
Met Ala Cys Arg Cys Leu Ser Phe Leu Leu Met Gly Thr Phe Leu
1 5 10 15

Ser Val Ser Gln Thr Val Leu Ala Gln Leu Asp Ala Leu Leu Val
20 25 30

Phe Pro Gly Gln Val Ala Gln Leu Ser Cys Thr Leu Ser Pro Gln
35 40 45

His Val Thr Ile Arg Asp Tyr Gly Val Ser Trp Tyr Gln Gln Arg
50 55 60

Ala Gly Ser Ala Pro Arg Tyr Leu Leu Tyr Tyr Arg Ser Glu Glu
65 70 75

Asp His His Arg Pro Ala Asp Ile Pro Asp Arg Phe Ser Ala Ala
80 85 90

Lys Asp Glu Ala His Asn Ala Cys Val Leu Thr Ile Ser Pro Val
95 100 105

Gln Pro Glu Asp Asp Ala Asp Tyr Tyr Cys Ser Val Gly Tyr Gly
110 115 120

Phe Ser Pro

<210> 403
<211> 1964
<212> DNA
<213> Homo Sapien

<400> 403

cgcgccgggc gcagggagct gagtggacgg ctcgagacgg cggcgcgtgc 50
agcagctcca gaaagcagcg agttggcaga gcagggctgc atttccagca 100
ggagctgcga gcacagtgct ggctcacaac aagatgctca aggtgtcagc 150
cgtactgtgt gtgtgtgcag ccgcttggtg cagtcagtct ctcgcagctg 200
ccgcggcggt ggctgcagcc gggggcggt cggacggcgg taattttctg 250
gatgataaac aatggctcac cacaatctct cagtatgaca aggaagtcgg 300
acagtggaac aaattccgag acgaagtaga ggatgattat ttccgcactt 350
ggagtccagg aaaacccttc gatcaggctt tagatccagc taaggatcca 400
tgcttaaaga tgaaatgtag tcgccataaa gtatgcattg ctcaagattc 450
tcagactgca gtctgcatta gtcaccggag gcttacacac aggatgaaag 500
aagcaggagt agaccatagg cagtggaggg gtcccatatt atccacctgc 550
aagcagtgcc cagtggtcta tcccagccct gtttgtggtt cagatggtca 600
tacctactct tttcagtgca aactagaata tcaggcatgt gtcttaggaa 650
aacagatctc agtcaaatgt gaaggacatt gcccatgtcc ttcagataag 700
cccaccagta caagcagaaa tgttaagaga gcatgcagtg acctggagtt 750
cagggaagtg gcaaacagat tgcgggactg gttcaaggcc cttcatgaaa 800
gtggaagtca aaacaagaag acaaaaacat tgctgaggcc tgagagaagc 850
agattcgata ccagcatctt gccaatttgc aaggactcac ttggctggat 900
gtttaacaga cttgatacaa actatgacct gctattggac cagtcagagc 950
tcagaagcat ttaccttgat aagaatgaac agtgtaccaa ggcattcttc 1000
aattcttgtg acacatacaa ggacagttta atatctaata atgagtggtg 1050
ctactgcttc cagagacagc aagacccacc ttgccagact gagctcagca 1100
atattcagaa gcggcaaggg gtaaagaagc tcctaggaca gtatatcccc 1150
ctgtgtgatg aagatggtta ctacaagcca acacaatgtc atggcagtgt 1200
tggacagtgc tggtgtgttg acagatatgg aaatgaagtc atgggatcca 1250
gaataaatgg tgttgcagat tgtgctatag attttgagat ctccggagat 1300
tttgctagtg gcgattttca tgaatggact gatgatgagg atgatgaaga 1350
cgatattatg aatgatgaag atgaaattga agatgatgat gaagatgaag 1400
gggatgatga tgatggtggt gatgaccatg atgtatacat ttgattgatg 1450
acagttgaaa tcaataaatt ctacatttct aatatttaca aaaatgatag 1500
cctatttaaa attatcttct tccccaataa caaaatgatt ctaaacctca 1550
catatatttt gtataattat ttgaaaaatt gcagctaaag ttatagaact 1600

ttatgtttaa ataagaatca tttgctttga gtttttatat tccttacaca 1650

aaaagaaaat acatatgcag tctagtcaga caaaataaag ttttgaagtg 1700

ctactataat aaatttttca cgagaacaaa ctttgtaaat cttccataag 1750

caaaatgaca gctagtgctt gggatcgtac atgttaattt tttgaaagat 1800

aattctaagt gaaatttaaa ataaataaat ttttaatgac ctgggtctta 1850

aggatttagg aaaaatatgc atgctttaat tgcatttcca aagtagcatc 1900

ttgctagacc tagatgagtc aggataacag agagatacca catgactcca 1950

aaaaaaaaaa aaaa 1964

<210> 404
<211> 436
<212> PRT
<213> Homo Sapien

<400> 404

```
Met Leu Lys Val Ser Ala Val Leu Cys Val Cys Ala Ala Ala Trp
  1               5                  10                  15

Cys Ser Gln Ser Leu Ala Ala Ala Ala Ala Val Ala Ala Ala Gly
                 20                  25                  30

Gly Arg Ser Asp Gly Gly Asn Phe Leu Asp Asp Lys Gln Trp Leu
                 35                  40                  45

Thr Thr Ile Ser Gln Tyr Asp Lys Glu Val Gly Gln Trp Asn Lys
                 50                  55                  60

Phe Arg Asp Glu Val Glu Asp Asp Tyr Phe Arg Thr Trp Ser Pro
                 65                  70                  75

Gly Lys Pro Phe Asp Gln Ala Leu Asp Pro Ala Lys Asp Pro Cys
                 80                  85                  90

Leu Lys Met Lys Cys Ser Arg His Lys Val Cys Ile Ala Gln Asp
                 95                 100                 105

Ser Gln Thr Ala Val Cys Ile Ser His Arg Arg Leu Thr His Arg
                110                 115                 120

Met Lys Glu Ala Gly Val Asp His Arg Gln Trp Arg Gly Pro Ile
                125                 130                 135

Leu Ser Thr Cys Lys Gln Cys Pro Val Val Tyr Pro Ser Pro Val
                140                 145                 150

Cys Gly Ser Asp Gly His Thr Tyr Ser Phe Gln Cys Lys Leu Glu
                155                 160                 165

Tyr Gln Ala Cys Val Leu Gly Lys Gln Ile Ser Val Lys Cys Glu
                170                 175                 180

Gly His Cys Pro Cys Pro Ser Asp Lys Pro Thr Ser Thr Ser Arg
                185                 190                 195

Asn Val Lys Arg Ala Cys Ser Asp Leu Glu Phe Arg Glu Val Ala
                200                 205                 210
```

```
Asn Arg Leu Arg Asp Trp Phe Lys Ala Leu His Glu Ser Gly Ser
                215                 220                 225

Gln Asn Lys Lys Thr Lys Thr Leu Leu Arg Pro Glu Arg Ser Arg
                230                 235                 240

Phe Asp Thr Ser Ile Leu Pro Ile Cys Lys Asp Ser Leu Gly Trp
                245                 250                 255

Met Phe Asn Arg Leu Asp Thr Asn Tyr Asp Leu Leu Leu Asp Gln
                260                 265                 270

Ser Glu Leu Arg Ser Ile Tyr Leu Asp Lys Asn Glu Gln Cys Thr
                275                 280                 285

Lys Ala Phe Phe Asn Ser Cys Asp Thr Tyr Lys Asp Ser Leu Ile
                290                 295                 300

Ser Asn Asn Glu Trp Cys Tyr Cys Phe Gln Arg Gln Gln Asp Pro
                305                 310                 315

Pro Cys Gln Thr Glu Leu Ser Asn Ile Gln Lys Arg Gln Gly Val
                320                 325                 330

Lys Lys Leu Leu Gly Gln Tyr Ile Pro Leu Cys Asp Glu Asp Gly
                335                 340                 345

Tyr Tyr Lys Pro Thr Gln Cys His Gly Ser Val Gly Gln Cys Trp
                350                 355                 360

Cys Val Asp Arg Tyr Gly Asn Glu Val Met Gly Ser Arg Ile Asn
                365                 370                 375

Gly Val Ala Asp Cys Ala Ile Asp Phe Glu Ile Ser Gly Asp Phe
                380                 385                 390

Ala Ser Gly Asp Phe His Glu Trp Thr Asp Asp Glu Asp Asp Glu
                395                 400                 405

Asp Asp Ile Met Asn Asp Glu Asp Glu Ile Glu Asp Asp Asp Glu
                410                 415                 420

Asp Glu Gly Asp Asp Asp Asp Gly Gly Asp Asp His Asp Val Tyr
                425                 430                 435

Ile
```

<210> 405
<211> 3819
<212> DNA
<213> Homo Sapien

<400> 405

```
ggaaggggag gagcaggcca cacaggcaca ggccggtgag ggacctgccc 50

agacctggag ggtctcgctc tgtcacacag gctggagtgc agtggtgtga 100

tcttggctca tcgtaacctc cacctcccgg gttcaagtga ttctcatgcc 150

tcagcctccc gagtagctgg gattacaggt ggtgacttcc aagagtgact 200

ccgtcggagg aaaatgactc cccagtcgct gctgcagacg acactgttcc 250
```

tgctgagtct gctcttcctg gtccaaggtg cccacggcag gggccacagg 300
gaagactttc gcttctgcag ccagcggaac cagacacaca ggagcagcct 350
ccactacaaa cccacaccag acctgcgcat ctccatcgag aactccgaag 400
aggccctcac agtccatgcc cctttccctg cagcccaccc tgcttcccga 450
tccttccctg accccagggg cctctaccac ttctgcctct actggaaccg 500
acatgctggg agattacatc ttctctatgg caagcgtgac ttcttgctga 550
gtgacaaagc ctctagcctc ctctgcttcc agcaccagga ggagagcctg 600
gctcagggcc ccccgctgtt agccacttct gtcacctcct ggtggagccc 650
tcagaacatc agcctgccca gtgccgccag cttcaccttc tccttccaca 700
gtcctcccca cacggccgct cacaatgcct cggtggacat gtgcgagctc 750
aaaagggacc tccagctgct cagccagttc ctgaagcatc cccagaaggc 800
ctcaaggagg ccctcggctg cccccgccag ccagcagttg cagagcctgg 850
agtcgaaact gacctctgtg agattcatgg gggacatggt gtccttcgag 900
gaggaccgga tcaacgccac ggtgtggaag ctccagccca cagccggcct 950
ccaggacctg cacatccact cccggcagga ggaggagcag agcgagatca 1000
tggagtactc ggtgctgctg cctcgaacac tcttccagag gacgaaaggc 1050
cggagcgggg aggctgagaa gagactcctc ctggtggact tcagcagcca 1100
agccctgttc caggacaaga attccagcca agtcctgggt gagaaggtct 1150
tggggattgt ggtacagaac accaaagtag ccaacctcac ggagcccgtg 1200
gtgctcactt tccagcacca gctacagccg aagaatgtga ctctgcaatg 1250
tgtgttctgg gttgaagacc ccacattgag cagcccgggg cattggagca 1300
gtgctgggtg tgagaccgtc aggagagaaa cccaaacatc ctgcttctgc 1350
aaccacttga cctactttgc agtgctgatg gtctcctcgg tggaggtgga 1400
cgccgtgcac aagcactacc tgagcctcct ctcctacgtg ggctgtgtcg 1450
tctctgccct ggcctgcctt gtcaccattg ccgcctacct ctgctccagg 1500
gtgcccctgc cgtgcaggag gaaacctcgg gactacacca tcaaggtgca 1550
catgaacctg ctgctggccg tcttcctgct ggacacgagc ttcctgctca 1600
gcgagccggt ggccctgaca ggctctgagg ctggctgccg agccagtgcc 1650
atcttcctgc acttctccct gctcacctgc ctttcctgga tgggcctcga 1700
ggggtacaac ctctaccgac tcgtggtgga ggtctttggc acctatgtcc 1750
ctggctacct actcaagctg agcgccatgg gctggggctt ccccatcttt 1800
ctggtgacgc tggtggccct ggtggatgtg gacaactatg gccccatcat 1850

cttggctgtg cataggactc cagagggcgt catctaccct tccatgtgct 1900
ggatccggga ctccctggtc agctacatca ccaacctggg cctcttcagc 1950
ctggtgtttc tgttcaacat ggccatgcta gccaccatgg tggtgcagat 2000
cctgcggctg cgcccccaca cccaaaagtg gtcacatgtg ctgacactgc 2050
tgggcctcag cctggtcctt ggcctgccct gggccttgat cttcttctcc 2100
tttgcttctg gcaccttcca gcttgtcgtc ctctaccttt tcagcatcat 2150
cacctccttc caaggcttcc tcatcttcat ctggtactgg tccatgcggc 2200
tgcaggcccg gggtggcccc tcccctctga agagcaactc agacagcgcc 2250
aggctcccca tcagctcggg cagcacctcg tccagccgca tctaggcctc 2300
cagcccacct gcccatgtga tgaagcagag atgcggcctc gtcgcacact 2350
gcctgtggcc cccgagccag gcccagcccc aggccagtca gccgcagact 2400
ttggaaagcc caacgaccat ggagagatgg gccgttgcca tggtggacgg 2450
actcccgggc tgggcttttg aattggcctt ggggactact cggctctcac 2500
tcagctccca cgggactcag aagtgcgccg ccatgctgcc tagggtactg 2550
tccccacatc tgtcccaacc cagctggagg cctggtctct ccttacaacc 2600
cctgggccca gccctcattg ctgggggcca ggccttggat cttgagggtc 2650
tggcacatcc ttaatcctgt gcccctgcct gggacagaaa tgtggctcca 2700
gttgctctgt ctctcgtggt caccctgagg gcactctgca tcctctgtca 2750
ttttaacctc aggtggcacc cagggcgaat ggggcccagg gcagaccttc 2800
agggccagag ccctggcgga ggagaggccc tttgccagga gcacagcagc 2850
agctcgccta cctctgagcc caggccccct ccctccctca gcccccccagt 2900
cctccctcca tcttccctgg ggttctcctc ctctcccagg gcctccttgc 2950
tccttcgttc acagctgggg gtccccgatt ccaatgctgt tttttgggga 3000
gtggtttcca ggagctgcct ggtgtctgct gtaaatgttt gtctactgca 3050
caagcctcgg cctgcccctg agccaggctc ggtaccgatg cgtgggctgg 3100
gctaggtccc tctgtccatc tgggcctttg tatgagctgc attgcccttg 3150
ctcaccctga ccaagcacac gcctcagagg ggccctcagc ctctcctgaa 3200
gccctcttgt ggcaagaact gtggaccatg ccagtcccgt ctggtttcca 3250
tcccaccact ccaaggactg agactgacct cctctggtga cactggccta 3300
gagcctgaca ctctcctaag aggttctctc caagccccca aatagctcca 3350
ggcgccctcg gccgcccatc atggttaatt ctgtccaaca aacacacacg 3400
ggtagattgc tggcctgttg taggtggtag ggacacagat gaccgacctg 3450

gtcactcctc ctgccaacat tcagtctggt atgtgaggcg tgcgtgaagc 3500

aagaactcct ggagctacag ggacagggag ccatcattcc tgcctgggaa 3550

tcctggaaga cttcctgcag gagtcagcgt tcaatcttga ccttgaagat 3600

gggaaggatg ttctttttac gtaccaattc ttttgtcttt tgatattaaa 3650

aagaagtaca tgttcattgt agagaatttg gaaactgtag aagagaatca 3700

agaagaaaaa taaaaatcag ctgttgtaat cgcctagcaa aaaaaaaaaa 3750

aaaaaaaaaa aaaaaaaaaa aaaaaaaaaa aaaaaaaaaa aaaaaaaaaa 3800

aaaaaaaaaa aaaaaaaaa 3819

<210> 406
<211> 693
<212> PRT
<213> Homo Sapien

<400> 406
Met Thr Pro Gln Ser Leu Leu Gln Thr Thr Leu Phe Leu Leu Ser
1 5 10 15

Leu Leu Phe Leu Val Gln Gly Ala His Gly Arg Gly His Arg Glu
20 25 30

Asp Phe Arg Phe Cys Ser Gln Arg Asn Gln Thr His Arg Ser Ser
35 40 45

Leu His Tyr Lys Pro Thr Pro Asp Leu Arg Ile Ser Ile Glu Asn
50 55 60

Ser Glu Glu Ala Leu Thr Val His Ala Pro Phe Pro Ala Ala His
65 70 75

Pro Ala Ser Arg Ser Phe Pro Asp Pro Arg Gly Leu Tyr His Phe
80 85 90

Cys Leu Tyr Trp Asn Arg His Ala Gly Arg Leu His Leu Leu Tyr
95 100 105

Gly Lys Arg Asp Phe Leu Leu Ser Asp Lys Ala Ser Ser Leu Leu
110 115 120

Cys Phe Gln His Gln Glu Glu Ser Leu Ala Gln Gly Pro Pro Leu
125 130 135

Leu Ala Thr Ser Val Thr Ser Trp Trp Ser Pro Gln Asn Ile Ser
140 145 150

Leu Pro Ser Ala Ala Ser Phe Thr Phe Ser Phe His Ser Pro Pro
155 160 165

His Thr Ala Ala His Asn Ala Ser Val Asp Met Cys Glu Leu Lys
170 175 180

Arg Asp Leu Gln Leu Leu Ser Gln Phe Leu Lys His Pro Gln Lys
185 190 195

Ala Ser Arg Arg Pro Ser Ala Ala Pro Ala Ser Gln Gln Leu Gln
200 205 210

Ser Leu Glu Ser Lys Leu Thr Ser Val Arg Phe Met Gly Asp Met
215 220 225

Val Ser Phe Glu Glu Asp Arg Ile Asn Ala Thr Val Trp Lys Leu
230 235 240

Gln Pro Thr Ala Gly Leu Gln Asp Leu His Ile His Ser Arg Gln
245 250 255

Glu Glu Glu Gln Ser Glu Ile Met Glu Tyr Ser Val Leu Leu Pro
260 265 270

Arg Thr Leu Phe Gln Arg Thr Lys Gly Arg Ser Gly Glu Ala Glu
275 280 285

Lys Arg Leu Leu Leu Val Asp Phe Ser Ser Gln Ala Leu Phe Gln
290 295 300

Asp Lys Asn Ser Ser Gln Val Leu Gly Glu Lys Val Leu Gly Ile
305 310 315

Val Val Gln Asn Thr Lys Val Ala Asn Leu Thr Glu Pro Val Val
320 325 330

Leu Thr Phe Gln His Gln Leu Gln Pro Lys Asn Val Thr Leu Gln
335 340 345

Cys Val Phe Trp Val Glu Asp Pro Thr Leu Ser Ser Pro Gly His
350 355 360

Trp Ser Ser Ala Gly Cys Glu Thr Val Arg Arg Glu Thr Gln Thr
365 370 375

Ser Cys Phe Cys Asn His Leu Thr Tyr Phe Ala Val Leu Met Val
380 385 390

Ser Ser Val Glu Val Asp Ala Val His Lys His Tyr Leu Ser Leu
395 400 405

Leu Ser Tyr Val Gly Cys Val Val Ser Ala Leu Ala Cys Leu Val
410 415 420

Thr Ile Ala Ala Tyr Leu Cys Ser Arg Val Pro Leu Pro Cys Arg
425 430 435

Arg Lys Pro Arg Asp Tyr Thr Ile Lys Val His Met Asn Leu Leu
440 445 450

Leu Ala Val Phe Leu Leu Asp Thr Ser Phe Leu Leu Ser Glu Pro
455 460 465

Val Ala Leu Thr Gly Ser Glu Ala Gly Cys Arg Ala Ser Ala Ile
470 475 480

Phe Leu His Phe Ser Leu Leu Thr Cys Leu Ser Trp Met Gly Leu
485 490 495

Glu Gly Tyr Asn Leu Tyr Arg Leu Val Val Glu Val Phe Gly Thr
500 505 510

Tyr Val Pro Gly Tyr Leu Leu Lys Leu Ser Ala Met Gly Trp Gly
515 520 525

Phe Pro Ile Phe Leu Val Thr Leu Val Ala Leu Val Asp Val Asp

530 535 540

Asn Tyr Gly Pro Ile Ile Leu Ala Val His Arg Thr Pro Glu Gly
545 550 555

Val Ile Tyr Pro Ser Met Cys Trp Ile Arg Asp Ser Leu Val Ser
560 565 570

Tyr Ile Thr Asn Leu Gly Leu Phe Ser Leu Val Phe Leu Phe Asn
575 580 585

Met Ala Met Leu Ala Thr Met Val Val Gln Ile Leu Arg Leu Arg
590 595 600

Pro His Thr Gln Lys Trp Ser His Val Leu Thr Leu Leu Gly Leu
605 610 615

Ser Leu Val Leu Gly Leu Pro Trp Ala Leu Ile Phe Phe Ser Phe
620 625 630

Ala Ser Gly Thr Phe Gln Leu Val Val Leu Tyr Leu Phe Ser Ile
635 640 645

Ile Thr Ser Phe Gln Gly Phe Leu Ile Phe Ile Trp Tyr Trp Ser
650 655 660

Met Arg Leu Gln Ala Arg Gly Gly Pro Ser Pro Leu Lys Ser Asn
665 670 675

Ser Asp Ser Ala Arg Leu Pro Ile Ser Ser Gly Ser Thr Ser Ser
680 685 690

Ser Arg Ile

<210> 407
<211> 950
<212> DNA
<213> Homo Sapien

<400> 407
ttgtgactaa aagctggcct agcaggccag ggagtgcagc tgcaggcgtg 50

ggggtggcag gagccgcaga gccagagcag acagccgaga aacaggtgga 100

cagtgtgaaa gaaccagtgg tctcgctctg ttgcccaggc tagagtgtac 150

tggcgtgatc atagctcact gcagcctcag actcctggac ttgagaaatc 200

ctcctgcctt agcctcctgc atatctggga ctccaggggt gcactcaagc 250

cctgtttctt ctccttctgt gagtggacca cggaggctgg tgagctgcct 300

gtcatcccaa agctcagctc tgagccagag tggtggtggc tccacctctg 350

ccgccggcat agaagccagg agcagggctc tcagaaggcg gtggtgccca 400

gctgggatca tgttgttggc cctggtctgt ctgctcagct gcctgctacc 450

ctccagtgag gccaagctct acggtcgttg tgaactggcc agagtgctac 500

atgacttcgg gctggacgga taccggggat acagcctggc tgactgggtc 550

tgccttgctt atttcacaag cggtttcaac gcagctgctt tggactacga 600

ggctgatggg agcaccaaca acgggatctt ccagatcaac agccggaggt 650

ggtgcagcaa cctcaccccg aacgtcccca acgtgtgccg gatgtactgc 700

tcagatttgt tgaatcctaa tctcaaggat accgttatct gtgccatgaa 750

gataacccaa gagcctcagg gtctgggtta ctgggaggcc tggaggcatc 800

actgccaggg aaaagacctc actgaatggg tggatggctg tgacttctag 850

gatggacgga accatgcaca gcaggctggg aaatgtggtt tggttcctga 900

cctaggcttg ggaagacaag ccagcgaata aaggatggtt gaacgtgaaa 950

<210> 408
<211> 146
<212> PRT
<213> Homo Sapien

<400> 408
Met Leu Leu Ala Leu Val Cys Leu Leu Ser Cys Leu Leu Pro Ser
1 5 10 15

Ser Glu Ala Lys Leu Tyr Gly Arg Cys Glu Leu Ala Arg Val Leu
20 25 30

His Asp Phe Gly Leu Asp Gly Tyr Arg Gly Tyr Ser Leu Ala Asp
35 40 45

Trp Val Cys Leu Ala Tyr Phe Thr Ser Gly Phe Asn Ala Ala Ala
50 55 60

Leu Asp Tyr Glu Ala Asp Gly Ser Thr Asn Asn Gly Ile Phe Gln
65 70 75

Ile Asn Ser Arg Arg Trp Cys Ser Asn Leu Thr Pro Asn Val Pro
80 85 90

Asn Val Cys Arg Met Tyr Cys Ser Asp Leu Leu Asn Pro Asn Leu
95 100 105

Lys Asp Thr Val Ile Cys Ala Met Lys Ile Thr Gln Glu Pro Gln
110 115 120

Gly Leu Gly Tyr Trp Glu Ala Trp Arg His His Cys Gln Gly Lys
125 130 135

Asp Leu Thr Glu Trp Val Asp Gly Cys Asp Phe
140 145

<210> 409
<211> 3617
<212> DNA
<213> Homo Sapien

<400> 409
cagactccag atttccctgt caaccacgag gagtccagag aggaaacgcg 50

gagcggagac aacagtacct gacgcctctt tcagcccggg atcgccccag 100

cagggatggg cgacaagatc tggctgccct tccccgtgct ccttctggcc 150

gctctgcctc cggtgctgct gcctggggcg gccggcttca caccttccct 200

cgatagcgac ttcaccttta cccttcccgc cggccagaag gagtgcttct 250
accagcccat gcccctgaag gcctcgctgg agatcgagta ccaagtttta 300
gatggagcag gattagatat tgatttccat cttgcctctc cagaaggcaa 350
aaccttagtt tttgaacaaa gaaaatcaga tggagttcac actgtagaga 400
ctgaagttgg tgattacatg ttctgctttg acaatacatt cagcaccatt 450
tctgagaagg tgattttctt tgaattaatc ctggataata tgggagaaca 500
ggcacaagaa caagaagatt ggaagaaata tattactggc acagatatat 550
tggatatgaa actggaagac atcctggaat ccatcaacag catcaagtcc 600
agactaagca aaagtgggca catacaaatt ctgcttagag catttgaagc 650
tcgtgatcga aacatacaag aaagcaactt tgatagagtc aatttctggt 700
ctatggttaa tttagtggtc atggtggtgg tgtcagccat tcaagtttat 750
atgctgaaga gtctgtttga agataagagg aaaagtagaa cttaaaactc 800
caaactagag tacgtaacat tgaaaaatga ggcataaaaa tgcaataaac 850
tgttacagtc aagaccatta atggtcttct ccaaaatatt ttgagatata 900
aaagtaggaa acaggtataa ttttaatgtg aaaattaagt cttcactttc 950
tgtgcaagta atcctgctga tccagttgta cttaagtgtg taacaggaat 1000
attttgcaga atataggttt aactgaatga agccatatta ataactgcat 1050
tttcctaact ttgaaaaatt ttgcaaatgt cttaggtgat ttaaataaat 1100
gagtattggg cctaattgca acaccagtct gtttttaaca ggttctatta 1150
cccagaactt ttttgtaaat gcggcagtta caaattaact gtggaagttt 1200
tcagttttaa gttataaatc acctgagaat tacctaatga tggattgaat 1250
aaatctttag actacaaaag cccaactttt ctctatttac atatgcatct 1300
ctcctataat gtaaatagaa taatagcttt gaaatacaat taggtttttg 1350
agatttttat aaccaaatac atttcagtgt aacatattag cagaaagcat 1400
tagtctttgt actttgctta cattcccaaa agctgacatt ttcacgattc 1450
ttaaaaacac aaagttacac ttactaaaat taggacatgt tttctctttg 1500
aaatgaagaa tatagtttaa aagcttcctc ctccataggg acacattttc 1550
tctaaccctt aactaaagtg taggatttta aaattaaatg tgaggtaaaa 1600
taagtttatt tttaatagta tctgtcaagt taatatctgt caacagttaa 1650
taatcatgtt atgttaattt taacatgatt gctgacttgg ataattcatt 1700
attaccagca gttatgaagg aaatattgct aaaatgatct gggcctacca 1750
taaataaata tctccttttc tgagctctaa gaattatcag aaaacaggaa 1800

agaatttaga aaaacttgag aaaacctaat ccaaaataaa attcacttaa 1850
gtagaactat aaataaatat ctagaatctg actggctcat catgacatcc 1900
tactcataac ataaatcaaa ggagatgatt aatttccagt tagctggaag 1950
aaactttggc tgtaggtttt tattttctac aagaattctg gtttgaatta 2000
tttttgtaag caggtacatt ttataaaatg taagccctac tgtaaggttt 2050
agcactgggt gtacatattt attaaaaatt tttattataa caacttttat 2100
taaaatggcc tttctgaaca ctttatttat tgatgttgaa gtaaggatta 2150
gaaacataga ctcccaagtt ttaaacacct aaatgtgaat aacccatata 2200
tacaacaaag tttctgccat ctagcttttt gaagtctatg gggtcttac 2250
tcaagtacta gtaatttaac ttcatcatga atgaactata atttttaagt 2300
tatgcccatt tataacgttg tttatgacta cattgtgagt tagaaacaaa 2350
cttaaaattt ggggtataga acccctcaac aggttagtaa tgctggaatt 2400
cttgatgagc aataatgata accagagagt gatttcattt acactcatag 2450
tagtataaaa agagatacat ttccctctta ggcccctggg agaagagcag 2500
cttagatttc cctactggca aggtttttaa aaatgaggta aatgccgtat 2550
atgatcaatt accttaattg gccaagaaaa tgcttcaggt gtctaggggt 2600
atcctctgca acacttgcag aacaaaggtc aataagatcc ttgcctatga 2650
ataccccctcc cttttgcgct gttaaatttg caatgagaag caaatttaca 2700
gtaccataac taataaagca gggtacagat ataaactact gcatcttttc 2750
tataaaactg tgattaagaa ttctacctct cctgtatggc tgttactgta 2800
ctgtactctc tgactcctta cctaacaatg aatttgttac ataatcttct 2850
acatgtatga tttgtgccac tgatcttaaa cctatgattc agtaacttct 2900
taccatataa aaacgataat tgctttattt ggaaaagaat ttaggaatac 2950
taaggacaat tatttttata gacaaagtaa aaagacagat atttaagagg 3000
cataaccaaa aaagcaaaac ttgtaaacag agtaaaaatc tttaatattt 3050
ctaaagacat actgtttatc tgcttcatat gcttttttta atttcactat 3100
tccatttcta aattaaagtt atgctaaatt gagtaagctg tttatcactt 3150
aacagctcat tttgtctttt tcaatataca aattttaaaa atactacaat 3200
atttaactaa ggcccaaccg atttccataa tgtagcagtt accgtgttca 3250
cctcacacta aggcctagag tttgctctga tatgcatttg gatgattaat 3300
gttatgctgt tctttcatgt gaatgtcaag acatggaggg tgtttgtaat 3350
tttatggtaa aattaatcct tcttacacat aatggtgtct taaaattgac 3400

aaaaaatgag cacttacaat tgtatgtctc ctcaaatgaa gattctttat 3450

gtgaaatttt aaaagacatt gattccgcat gtaaggattt ttcatctgaa 3500

gtacaataat gcacaatcag tgttgctcaa actgctttat acttataaac 3550

agccatctta aataagcaac gtattgtgag tactgatatg tatataataa 3600

aaattatcaa aggaaaa 3617

<210> 410
<211> 229
<212> PRT
<213> Homo Sapien

<400> 410

```
Met Gly Asp Lys Ile Trp Leu Pro Phe Pro Val Leu Leu Leu Ala
 1               5                  10                  15

Ala Leu Pro Pro Val Leu Leu Pro Gly Ala Ala Gly Phe Thr Pro
                20                  25                  30

Ser Leu Asp Ser Asp Phe Thr Phe Thr Leu Pro Ala Gly Gln Lys
                35                  40                  45

Glu Cys Phe Tyr Gln Pro Met Pro Leu Lys Ala Ser Leu Glu Ile
                50                  55                  60

Glu Tyr Gln Val Leu Asp Gly Ala Gly Leu Asp Ile Asp Phe His
                65                  70                  75

Leu Ala Ser Pro Glu Gly Lys Thr Leu Val Phe Glu Gln Arg Lys
                80                  85                  90

Ser Asp Gly Val His Thr Val Glu Thr Glu Val Gly Asp Tyr Met
                95                 100                 105

Phe Cys Phe Asp Asn Thr Phe Ser Thr Ile Ser Glu Lys Val Ile
               110                 115                 120

Phe Phe Glu Leu Ile Leu Asp Asn Met Gly Glu Gln Ala Gln Glu
               125                 130                 135

Gln Glu Asp Trp Lys Lys Tyr Ile Thr Gly Thr Asp Ile Leu Asp
               140                 145                 150

Met Lys Leu Glu Asp Ile Leu Glu Ser Ile Asn Ser Ile Lys Ser
               155                 160                 165

Arg Leu Ser Lys Ser Gly His Ile Gln Ile Leu Leu Arg Ala Phe
               170                 175                 180

Glu Ala Arg Asp Arg Asn Ile Gln Glu Ser Asn Phe Asp Arg Val
               185                 190                 195

Asn Phe Trp Ser Met Val Asn Leu Val Val Met Val Val Val Ser
               200                 205                 210

Ala Ile Gln Val Tyr Met Leu Lys Ser Leu Phe Glu Asp Lys Arg
               215                 220                 225

Lys Ser Arg Thr
```

<210> 411
<211> 4420
<212> DNA
<213> Homo Sapien

<400> 411
cccagctgag gagccctgct caagacacgg tcactggatc tgagaaactt 50
cccaggggac cgcattccag agtcagtgac tctgtgaagc acccacatct 100
acctcttgcc acgttcccac gggcttgggg gaaagatggt ggggaccaag 150
gcctgggtgt tctccttcct ggtcctggaa gtcacatctg tgttggggag 200
acagacgatg ctcacccagt cagtaagaag agtccagcct gggaagaaga 250
accccagcat ctttgccaag cctgccgaca ccctggagag ccctggtgag 300
tggacaacat ggttcaacat cgactaccca ggcgggaagg gcgactatga 350
gcggctggac gccattcgct tctactatgg ggaccgtgta tgtgcccgtc 400
ccctgcggct agaggctcgg accactgact ggacacctgc gggcagcact 450
ggccaggtgg tccatggtag tccccgtgag ggtttctggt gcctcaacag 500
ggagcagcgg cctggccaga actgctctaa ttacaccgta cgcttcctct 550
gcccaccagg atccctgcgc cgagacacag agcgcatctg gagcccatgg 600
tctccctgga gcaagtgctc agctgcctgt ggtcagactg gggtccagac 650
tcgcacacgc atttgcttgg cagagatggt gtcgctgtgc agtgaggcca 700
gcgaagaggg tcagcactgc atgggccagg actgtacagc ctgtgacctg 750
acctgcccaa tgggccaggt gaatgctgac tgtgatgcct gcatgtgcca 800
ggacttcatg cttcatgggg ctgtctccct tcccggaggt gccccagcct 850
caggggctgc tatctacctc ctgaccaaga cgccgaagct gctgacccag 900
acagacagtg atgggagatt ccgaatccct ggcttgtgcc ctgatggcaa 950
aagcatcctg aagatcacaa aggtcaagtt tgcccccatt gtactcacaa 1000
tgcccaagac tagcctgaag gcagccacca tcaaggcaga gtttgtgagg 1050
gcagagactc catacatggt gatgaaccct gagacaaaag cacggagagc 1100
tgggcagagc gtgtctctgt gctgtaaggc cacagggaag cccaggccag 1150
acaagtattt ttggtatcat aatgacacat tgctggatcc ttccctctac 1200
aagcatgaga gcaagctggt gctgaggaaa ctgcagcagc accaggctgg 1250
ggagtacttt tgcaaggccc agagtgatgc tggggctgtg aagtccaagg 1300
ttgcccagct gattgtcaca gcatctgatg agactccttg caacccagtt 1350
cctgagagct atcttatccg gctgccccat gattgctttc agaatgccac 1400
caactccttc tactatgacg tgggacgctg ccctgttaag acttgtgcag 1450

ggcagcagga taatgggatc aggtgccgtg atgctgtgca gaactgctgt 1500
ggcatctcca agacagagga aagggagatc cagtgcagtg gctacacgct 1550
acccaccaag gtggccaagg agtgcagctg ccagcggtgt acggaaactc 1600
ggagcatcgt gcggggccgt gtcagtgctg ctgacaatgg ggagcccatg 1650
cgctttggcc atgtgtacat ggggaacagc cgtgtaagca tgactggcta 1700
caagggcact ttcaccctcc atgtccccca ggacactgag aggctggtgc 1750
tcacatttgt ggacaggctg cagaagtttg tcaacaccac caaagtgcta 1800
cctttcaaca agaaggggag tgccgtgttc catgaaatca agatgcttcg 1850
tcggaaagag cccatcactt tggaagccat ggagaccaac atcatccccc 1900
tgggggaagt ggttggtgaa gaccccatgg ctgaactgga gattccatcc 1950
aggagtttct acaggcagaa tggggagccc tacataggaa aagtgaaggc 2000
cagtgtgacc ttcctggatc cccggaatat ttccacagcc acagctgccc 2050
agactgacct gaacttcatc aatgacgaag gagacacttt ccccccttcgg 2100
acgtatggca tgttctctgt ggacttcaga gatgaggtca cctcagagcc 2150
acttaatgct ggcaaagtga aggtccacct tgactcgacc caggtcaaga 2200
tgccagagca catatccaca gtgaaactct ggtcactcaa tccagacaca 2250
gggctgtggg aggaggaagg tgatttcaaa tttgaaaatc aaaggaggaa 2300
caaaagagaa gacagaacct tcctggtggg caacctggag attcgtgaga 2350
ggaggctctt taacctggat gttcctgaaa gcaggcggtg ctttgttaag 2400
gtgagggcct accggagtga gaggttcttg cctagtgagc agatccaggg 2450
ggttgtgatc tccgtgatta acctggagcc tagaactggc ttcttgtcca 2500
accctagggc ctggggccgc tttgacagtg tcatcacagg ccccaacggg 2550
gcctgtgtgc ctgccttctg tgatgaccag tcccctgatg cctactctgc 2600
ctatgtcttg gcaagcctgg ctggggagga actgcaagca gtggagtctt 2650
ctcctaaatt caacccaaat gcaattggcg tccctcagcc ctatctcaac 2700
aagctcaact accgtcggac ggaccatgag gatccacggg ttaaaaagac 2750
agctttccag attagcatgg ccaagccaag gcccaactca gctgaggaga 2800
gcaatgggcc catctatgcc tttgagaacc tccgggcatg tgaagaggca 2850
ccacccagtg cagcccactt ccggttctac cagattgagg gggatcgata 2900
tgactacaac acagtcccct tcaacgaaga tgaccctatg agctggactg 2950
aagactatct ggcatggtgg ccaaagccga tggaattcag ggcctgctat 3000
atcaaggtga agattgtggg gccactggaa gtgaatgtgc gatcccgcaa 3050

catgggggc actcatcggc ggacagtggg gaagctgtat ggaatccgag 3100

atgtgaggag cactcgggac agggaccagc ccaatgtctc agctgcctgt 3150

ctggagttca agtgcagtgg gatgctctat gatcaggacc gtgtggaccg 3200

caccctggtg aaggtcatcc cccagggcag ctgccgtcga gccagtgtga 3250

accccatgct gcatgagtac ctggtcaacc acttgccact tgcagtcaac 3300

aacgacacca gtgagtacac catgctggca cccttggacc cactgggcca 3350

caactatggc atctacactg tcactgacca ggaccctcgc acggccaagg 3400

agatcgcgct cggccggtgc tttgatggca catccgatgg ctcctccaga 3450

atcatgaaga gcaatgtggg agtagccctc accttcaact gtgtagagag 3500

gcaagtaggc cgccagagtg ccttccagta cctccaaagc accccagccc 3550

agtcccctgc tgcaggcact gtccaaggaa gagtgccctc gaggaggcag 3600

cagcgagcga gcaggggtgg ccagcgccag ggtggagtgg tggcctctct 3650

gagatttcct agagttgctc aacagcccct gatcaactaa gttttgtggt 3700

acttcaccct cttctgccct catttcatgt gacagccatt gtgagactga 3750

tgcacaaact gtcacttggt taatttaagc acttctgttt tcgtgaattt 3800

gcttgtttgt ttcttcatgc ctttacttac tttgtcccat gctactgatt 3850

ggcacgtggc cccccacaatg gcacaataaa gccccttt gt gaaactgttc 3900

tttaaatgaa acacaagaaa ttggccactg gtaaaactct gcagcttcaa 3950

ctgtacttca tttaatgcca ttaatgcaaa tatacttcct cttctttttg 4000

catggttttg cccacctctg caatagtgat aatctgatgc tgaagatcaa 4050

ataaccaata taaagcatat ttcttggcct tgctccacag gacataggca 4100

agccttgatc atagttcata catataaatg gtggtgaaat aaagaaataa 4150

aacacaatac ttttacttga aatgtaaata acttatttat ttctttgcta 4200

aatttggaat tctagtgcac attcaaagtt aagctattaa atatagggtg 4250

atcatagttc ctctaccaag tctggaaaga acatctcctg gtatccacaa 4300

ttacaccagg ttgctaactg tatttgtaca tttccctttg cattcgcttt 4350

tgttcttgct agaaacccag tgtagcccag ggcagatgtc aataaatgca 4400

tactctgtat ttcgaaaaaa 4420

<210> 412
<211> 1184
<212> PRT
<213> Homo Sapien

<400> 412
Met Val Gly Thr Lys Ala Trp Val Phe Ser Phe Leu Val Leu Glu

```
1               5                   10                  15
Val Thr Ser Val Leu Gly Arg Gln Thr Met Leu Thr Gln Ser Val
                20                  25                  30
Arg Arg Val Gln Pro Gly Lys Lys Asn Pro Ser Ile Phe Ala Lys
                35                  40                  45
Pro Ala Asp Thr Leu Glu Ser Pro Gly Glu Trp Thr Thr Trp Phe
                50                  55                  60
Asn Ile Asp Tyr Pro Gly Gly Lys Gly Asp Tyr Glu Arg Leu Asp
                65                  70                  75
Ala Ile Arg Phe Tyr Tyr Gly Asp Arg Val Cys Ala Arg Pro Leu
                80                  85                  90
Arg Leu Glu Ala Arg Thr Thr Asp Trp Thr Pro Ala Gly Ser Thr
                95                  100                 105
Gly Gln Val Val His Gly Ser Pro Arg Glu Gly Phe Trp Cys Leu
                110                 115                 120
Asn Arg Glu Gln Arg Pro Gly Gln Asn Cys Ser Asn Tyr Thr Val
                125                 130                 135
Arg Phe Leu Cys Pro Pro Gly Ser Leu Arg Arg Asp Thr Glu Arg
                140                 145                 150
Ile Trp Ser Pro Trp Ser Pro Trp Ser Lys Cys Ser Ala Ala Cys
                155                 160                 165
Gly Gln Thr Gly Val Gln Thr Arg Thr Arg Ile Cys Leu Ala Glu
                170                 175                 180
Met Val Ser Leu Cys Ser Glu Ala Ser Glu Glu Gly Gln His Cys
                185                 190                 195
Met Gly Gln Asp Cys Thr Ala Cys Asp Leu Thr Cys Pro Met Gly
                200                 205                 210
Gln Val Asn Ala Asp Cys Asp Ala Cys Met Cys Gln Asp Phe Met
                215                 220                 225
Leu His Gly Ala Val Ser Leu Pro Gly Gly Ala Pro Ala Ser Gly
                230                 235                 240
Ala Ala Ile Tyr Leu Leu Thr Lys Thr Pro Lys Leu Leu Thr Gln
                245                 250                 255
Thr Asp Ser Asp Gly Arg Phe Arg Ile Pro Gly Leu Cys Pro Asp
                260                 265                 270
Gly Lys Ser Ile Leu Lys Ile Thr Lys Val Lys Phe Ala Pro Ile
                275                 280                 285
Val Leu Thr Met Pro Lys Thr Ser Leu Lys Ala Ala Thr Ile Lys
                290                 295                 300
Ala Glu Phe Val Arg Ala Glu Thr Pro Tyr Met Val Met Asn Pro
                305                 310                 315
Glu Thr Lys Ala Arg Arg Ala Gly Gln Ser Val Ser Leu Cys Cys
                320                 325                 330
```

```
Lys Ala Thr Gly Lys Pro Arg Pro Asp Lys Tyr Phe Trp Tyr His
                335                 340                 345

Asn Asp Thr Leu Leu Asp Pro Ser Leu Tyr Lys His Glu Ser Lys
                350                 355                 360

Leu Val Leu Arg Lys Leu Gln Gln His Gln Ala Gly Glu Tyr Phe
                365                 370                 375

Cys Lys Ala Gln Ser Asp Ala Gly Ala Val Lys Ser Lys Val Ala
                380                 385                 390

Gln Leu Ile Val Thr Ala Ser Asp Glu Thr Pro Cys Asn Pro Val
                395                 400                 405

Pro Glu Ser Tyr Leu Ile Arg Leu Pro His Asp Cys Phe Gln Asn
                410                 415                 420

Ala Thr Asn Ser Phe Tyr Tyr Asp Val Gly Arg Cys Pro Val Lys
                425                 430                 435

Thr Cys Ala Gly Gln Gln Asp Asn Gly Ile Arg Cys Arg Asp Ala
                440                 445                 450

Val Gln Asn Cys Cys Gly Ile Ser Lys Thr Glu Glu Arg Glu Ile
                455                 460                 465

Gln Cys Ser Gly Tyr Thr Leu Pro Thr Lys Val Ala Lys Glu Cys
                470                 475                 480

Ser Cys Gln Arg Cys Thr Glu Thr Arg Ser Ile Val Arg Gly Arg
                485                 490                 495

Val Ser Ala Ala Asp Asn Gly Glu Pro Met Arg Phe Gly His Val
                500                 505                 510

Tyr Met Gly Asn Ser Arg Val Ser Met Thr Gly Tyr Lys Gly Thr
                515                 520                 525

Phe Thr Leu His Val Pro Gln Asp Thr Glu Arg Leu Val Leu Thr
                530                 535                 540

Phe Val Asp Arg Leu Gln Lys Phe Val Asn Thr Thr Lys Val Leu
                545                 550                 555

Pro Phe Asn Lys Lys Gly Ser Ala Val Phe His Glu Ile Lys Met
                560                 565                 570

Leu Arg Arg Lys Glu Pro Ile Thr Leu Glu Ala Met Glu Thr Asn
                575                 580                 585

Ile Ile Pro Leu Gly Glu Val Val Gly Glu Asp Pro Met Ala Glu
                590                 595                 600

Leu Glu Ile Pro Ser Arg Ser Phe Tyr Arg Gln Asn Gly Glu Pro
                605                 610                 615

Tyr Ile Gly Lys Val Lys Ala Ser Val Thr Phe Leu Asp Pro Arg
                620                 625                 630

Asn Ile Ser Thr Ala Thr Ala Ala Gln Thr Asp Leu Asn Phe Ile
                635                 640                 645
```

Asn Asp Glu Gly Asp Thr Phe Pro Leu Arg Thr Tyr Gly Met Phe
650 655 660

Ser Val Asp Phe Arg Asp Glu Val Thr Ser Glu Pro Leu Asn Ala
665 670 675

Gly Lys Val Lys Val His Leu Asp Ser Thr Gln Val Lys Met Pro
680 685 690

Glu His Ile Ser Thr Val Lys Leu Trp Ser Leu Asn Pro Asp Thr
695 700 705

Gly Leu Trp Glu Glu Glu Gly Asp Phe Lys Phe Glu Asn Gln Arg
710 715 720

Arg Asn Lys Arg Glu Asp Arg Thr Phe Leu Val Gly Asn Leu Glu
725 730 735

Ile Arg Glu Arg Arg Leu Phe Asn Leu Asp Val Pro Glu Ser Arg
740 745 750

Arg Cys Phe Val Lys Val Arg Ala Tyr Arg Ser Glu Arg Phe Leu
755 760 765

Pro Ser Glu Gln Ile Gln Gly Val Val Ile Ser Val Ile Asn Leu
770 775 780

Glu Pro Arg Thr Gly Phe Leu Ser Asn Pro Arg Ala Trp Gly Arg
785 790 795

Phe Asp Ser Val Ile Thr Gly Pro Asn Gly Ala Cys Val Pro Ala
800 805 810

Phe Cys Asp Asp Gln Ser Pro Asp Ala Tyr Ser Ala Tyr Val Leu
815 820 825

Ala Ser Leu Ala Gly Glu Glu Leu Gln Ala Val Glu Ser Ser Pro
830 835 840

Lys Phe Asn Pro Asn Ala Ile Gly Val Pro Gln Pro Tyr Leu Asn
845 850 855

Lys Leu Asn Tyr Arg Arg Thr Asp His Glu Asp Pro Arg Val Lys
860 865 870

Lys Thr Ala Phe Gln Ile Ser Met Ala Lys Pro Arg Pro Asn Ser
875 880 885

Ala Glu Glu Ser Asn Gly Pro Ile Tyr Ala Phe Glu Asn Leu Arg
890 895 900

Ala Cys Glu Glu Ala Pro Pro Ser Ala Ala His Phe Arg Phe Tyr
905 910 915

Gln Ile Glu Gly Asp Arg Tyr Asp Tyr Asn Thr Val Pro Phe Asn
920 925 930

Glu Asp Asp Pro Met Ser Trp Thr Glu Asp Tyr Leu Ala Trp Trp
935 940 945

Pro Lys Pro Met Glu Phe Arg Ala Cys Tyr Ile Lys Val Lys Ile
950 955 960

Val Gly Pro Leu Glu Val Asn Val Arg Ser Arg Asn Met Gly Gly

965 970 975

Thr His Arg Arg Thr Val Gly Lys Leu Tyr Gly Ile Arg Asp Val
980 985 990

Arg Ser Thr Arg Asp Arg Asp Gln Pro Asn Val Ser Ala Ala Cys
995 1000 1005

Leu Glu Phe Lys Cys Ser Gly Met Leu Tyr Asp Gln Asp Arg Val
1010 1015 1020

Asp Arg Thr Leu Val Lys Val Ile Pro Gln Gly Ser Cys Arg Arg
1025 1030 1035

Ala Ser Val Asn Pro Met Leu His Glu Tyr Leu Val Asn His Leu
1040 1045 1050

Pro Leu Ala Val Asn Asn Asp Thr Ser Glu Tyr Thr Met Leu Ala
1055 1060 1065

Pro Leu Asp Pro Leu Gly His Asn Tyr Gly Ile Tyr Thr Val Thr
1070 1075 1080

Asp Gln Asp Pro Arg Thr Ala Lys Glu Ile Ala Leu Gly Arg Cys
1085 1090 1095

Phe Asp Gly Thr Ser Asp Gly Ser Ser Arg Ile Met Lys Ser Asn
1100 1105 1110

Val Gly Val Ala Leu Thr Phe Asn Cys Val Glu Arg Gln Val Gly
1115 1120 1125

Arg Gln Ser Ala Phe Gln Tyr Leu Gln Ser Thr Pro Ala Gln Ser
1130 1135 1140

Pro Ala Ala Gly Thr Val Gln Gly Arg Val Pro Ser Arg Arg Gln
1145 1150 1155

Gln Arg Ala Ser Arg Gly Gly Gln Arg Gln Gly Gly Val Val Ala
1160 1165 1170

Ser Leu Arg Phe Pro Arg Val Ala Gln Gln Pro Leu Ile Asn
1175 1180

<210> 413
<211> 595
<212> DNA
<213> Homo Sapien

<400> 413
gccacgttgt cttctttcct tcaccaccac ccaggagctc agagatctaa 50

gctgctttcc atcttttctc ccagccccag gacactgact ctgtacagga 100

tggggccgtc ctcttgcctc cttctcatcc taatccccct tctccagctg 150

atcaacccgg ggagtactca gtgttcctta gactccgtta tggataagaa 200

gatcaaggat gttctcaaca gtctagagta cagtccctct cctataagca 250

agaagctctc gtgtgctagt gtcaaaagcc aaggcagacc gtcctcctgc 300

cctgctggga tggctgtcac tggctgtgct tgtggctatg gctgtggttc 350

gtgggatgtt cagctggaaa ccacctgcca ctgccagtgc agtgtggtgg 400

actggaccac tgcccgctgc tgccacctga cctgacaggg aggaggctga 450

gaactcagtt ttgtgaccat gacagtaatg aaaccagggt cccaaccaag 500

aaatctaact caaacgtccc acttcatttg ttccattcct gattcttggg 550

taataaagac aaactttgta cctcaaaaaa aaaaaaaaaa aaaaa 595

<210> 414
<211> 111
<212> PRT
<213> Homo Sapien

<400> 414
Met Gly Pro Ser Ser Cys Leu Leu Leu Ile Leu Ile Pro Leu Leu
1 5 10 15

Gln Leu Ile Asn Pro Gly Ser Thr Gln Cys Ser Leu Asp Ser Val
20 25 30

Met Asp Lys Lys Ile Lys Asp Val Leu Asn Ser Leu Glu Tyr Ser
35 40 45

Pro Ser Pro Ile Ser Lys Lys Leu Ser Cys Ala Ser Val Lys Ser
50 55 60

Gln Gly Arg Pro Ser Ser Cys Pro Ala Gly Met Ala Val Thr Gly
65 70 75

Cys Ala Cys Gly Tyr Gly Cys Gly Ser Trp Asp Val Gln Leu Glu
80 85 90

Thr Thr Cys His Cys Gln Cys Ser Val Val Asp Trp Thr Thr Ala
95 100 105

Arg Cys Cys His Leu Thr
110

<210> 415
<211> 1621
<212> DNA
<213> Homo Sapien

<400> 415
cagaagaggg ggctagctag ctgtctctgc ggaccaggga gaccccccgcg 50

cccccccggt gtgaggcggc ctcacagggc cgggtgggct ggcgagccga 100

cgcggcggcg gaggaggctg tgaggagtgt gtggaacagg acccgggaca 150

gaggaaccat ggctccgcag aacctgagca ccttttgcct gttgctgcta 200

tacctcatcg gggcggtgat tgccggacga gatttctata agatcttggg 250

ggtgcctcga agtgcctcta taaaggatat taaaaaggcc tataggaaac 300

tagccctgca gcttcatccc gaccggaacc ctgatgatcc acaagcccag 350

gagaaattcc aggatctggg tgctgcttat gaggttctgt cagatagtga 400

gaaacggaaa cagtacgata cttatggtga agaaggatta aaagatggtc 450

atcagagctc ccatggagac attttttcac acttctttgg ggattttggt 500

ttcatgtttg gaggaacccc tcgtcagcaa gacagaaata ttccaagagg 550

aagtgatatt attgtagatc tagaagtcac tttggaagaa gtatatgcag 600

gaaattttgt ggaagtagtt agaaacaaac ctgtggcaag gcaggctcct 650

ggcaaacgga agtgcaattg tcggcaagag atgcggacca cccagctggg 700

ccctgggcgc ttccaaatga cccaggaggt ggtctgcgac gaatgcccta 750

atgtcaaact agtgaatgaa gaacgaacgc tggaagtaga aatagagcct 800

ggggtgagag acggcatgga gtaccccttt attggagaag gtgagcctca 850

cgtggatggg gagcctggag atttacggtt ccgaatcaaa gttgtcaagc 900

acccaatatt tgaaaggaga ggagatgatt tgtacacaaa tgtgacaatc 950

tcattagttg agtcactggt tggctttgag atggatatta ctcacttgga 1000

tggtcacaag gtacatattt cccgggataa gatcaccagg ccaggagcga 1050

agctatggaa gaaaggggaa gggctcccca actttgacaa caacaatatc 1100

aagggctctt tgataatcac ttttgatgtg gattttccaa aagaacagtt 1150

aacagaggaa gcgagagaag gtatcaaaca gctactgaaa caagggtcag 1200

tgcagaaggt atacaatgga ctgcaaggat attgagagtg aataaaattg 1250

gactttgttt aaaataagtg aataagcgat atttattatc tgcaaggttt 1300

ttttgtgtgt gtttttgttt ttattttcaa tatgcaagtt aggcttaatt 1350

tttttatcta atgatcatca tgaaatgaat aagagggctt aagaatttgt 1400

ccatttgcat tcggaaaaga atgaccagca aaaggtttac taatacctct 1450

ccctttgggg atttaatgtc tggtgctgcc gcctgagttt caagaattaa 1500

agctgcaaga ggactccagg agcaaaagaa acacaatata gagggttgga 1550

gttgttagca atttcattca aaatgccaac tggagaagtc tgtttttaaa 1600

tacattttgt tgttattttt a 1621

<210> 416
<211> 358
<212> PRT
<213> Homo Sapien

<400> 416
Met Ala Pro Gln Asn Leu Ser Thr Phe Cys Leu Leu Leu Leu Tyr
1               5                   10                  15

Leu Ile Gly Ala Val Ile Ala Gly Arg Asp Phe Tyr Lys Ile Leu
                20                  25                  30

Gly Val Pro Arg Ser Ala Ser Ile Lys Asp Ile Lys Lys Ala Tyr
                35                  40                  45

Arg Lys Leu Ala Leu Gln Leu His Pro Asp Arg Asn Pro Asp Asp

50 55 60

Pro Gln Ala Gln Glu Lys Phe Gln Asp Leu Gly Ala Ala Tyr Glu
65 70 75

Val Leu Ser Asp Ser Glu Lys Arg Lys Gln Tyr Asp Thr Tyr Gly
80 85 90

Glu Glu Gly Leu Lys Asp Gly His Gln Ser Ser His Gly Asp Ile
95 100 105

Phe Ser His Phe Phe Gly Asp Phe Gly Phe Met Phe Gly Gly Thr
110 115 120

Pro Arg Gln Gln Asp Arg Asn Ile Pro Arg Gly Ser Asp Ile Ile
125 130 135

Val Asp Leu Glu Val Thr Leu Glu Glu Val Tyr Ala Gly Asn Phe
140 145 150

Val Glu Val Val Arg Asn Lys Pro Val Ala Arg Gln Ala Pro Gly
155 160 165

Lys Arg Lys Cys Asn Cys Arg Gln Glu Met Arg Thr Thr Gln Leu
170 175 180

Gly Pro Gly Arg Phe Gln Met Thr Gln Glu Val Val Cys Asp Glu
185 190 195

Cys Pro Asn Val Lys Leu Val Asn Glu Glu Arg Thr Leu Glu Val
200 205 210

Glu Ile Glu Pro Gly Val Arg Asp Gly Met Glu Tyr Pro Phe Ile
215 220 225

Gly Glu Gly Glu Pro His Val Asp Gly Glu Pro Gly Asp Leu Arg
230 235 240

Phe Arg Ile Lys Val Val Lys His Pro Ile Phe Glu Arg Arg Gly
245 250 255

Asp Asp Leu Tyr Thr Asn Val Thr Ile Ser Leu Val Glu Ser Leu
260 265 270

Val Gly Phe Glu Met Asp Ile Thr His Leu Asp Gly His Lys Val
275 280 285

His Ile Ser Arg Asp Lys Ile Thr Arg Pro Gly Ala Lys Leu Trp
290 295 300

Lys Lys Gly Glu Gly Leu Pro Asn Phe Asp Asn Asn Asn Ile Lys
305 310 315

Gly Ser Leu Ile Ile Thr Phe Asp Val Asp Phe Pro Lys Glu Gln
320 325 330

Leu Thr Glu Glu Ala Arg Glu Gly Ile Lys Gln Leu Leu Lys Gln
335 340 345

Gly Ser Val Gln Lys Val Tyr Asn Gly Leu Gln Gly Tyr
350 355

<210> 417
<211> 1547

<212> DNA
<213> Homo Sapien

<400> 417
cggcggcggc tgcgggcgcg aggtgagggg cgcgaggtga ggggcgcgag 50

gttcccagca ggatgccccg gctctgcagg aagctgaagt gagaggcccg 100

gagagggccc agcccgcccg gggcaggatg accaaggccc ggctgttccg 150

gctgtggctg gtgctggggt cggtgttcat gatcctgctg atcatcgtgt 200

actgggacag cgcaggcgcc gcgcacttct acttgcacac gtccttctct 250

aggccgcaca cggggccgcc gctgcccacg cccgggccgg acagggacag 300

ggagctcacg gccgactccg atgtcgacga gtttctggac aagtttctca 350

gtgctggcgt gaagcagagc gaccttccca gaaaggagac ggagcagccg 400

cctgcgccgg ggagcatgga ggagagcgtg agaggctacg actggtcccc 450

gcgcgacgcc cggcgcagcc cagaccaggg ccggcagcag gcggagcgga 500

ggagcgtgct gcggggcttc tgcgccaact ccagcctggc cttccccacc 550

aaggagcgcg cattcgacga catccccaac tcggagctga gccacctgat 600

cgtggacgac cggcacgggg ccatctactg ctacgtgccc aaggtggcct 650

gcaccaactg gaagcgcgtg atgatcgtgc tgagcggaag cctgctgcac 700

cgcggtgcgc cctaccgcga cccgctgcgc atcccgcgcg agcacgtgca 750

caacgccagc gcgcacctga ccttcaacaa gttctggcgc cgctacggga 800

agctctcccg ccacctcatg aaggtcaagc tcaagaagta caccaagttc 850

ctcttcgtgc gcgacccctt cgtgcgcctg atctccgcct tccgcagcaa 900

gttcgagctg gagaacgagg agttctaccg caagttcgcc gtgcccatgc 950

tgcggctgta cgccaaccac accagcctgc ccgcctcggc gcgcgaggcc 1000

ttccgcgctg gcctcaaggt gtccttcgcc aacttcatcc agtacctgct 1050

ggacccgcac acggagaagc tggcgccctt caacgagcac tggcggcagg 1100

tgtaccgcct ctgccacccg tgccagatcg actacgactt cgtggggaag 1150

ctggagactc tggacgagga cgccgcgcag ctgctgcagc tactccaggt 1200

ggaccggcag ctccgcttcc ccccgagcta ccggaacagg accgccagca 1250

gctgggagga ggactggttc gccaagatcc ccctggcctg gaggcagcag 1300

ctgtataaac tctacgaggc cgactttgtt ctcttcggct accccaagcc 1350

cgaaaacctc ctccgagact gaaagctttc gcgttgcttt ttctcgcgtg 1400

cctggaacct gacgcacgcg cactccagtt tttttatgac ctacgatttt 1450

gcaatctggg cttcttgttc actccactgc ctctatccat tgagtactgt 1500

atcgatattg ttttttaaga ttaatatatt tcaggtattt aatacga 1547

<210> 418
<211> 414
<212> PRT
<213> Homo Sapien

<400> 418
Met Thr Lys Ala Arg Leu Phe Arg Leu Trp Leu Val Leu Gly Ser
1 5 10 15

Val Phe Met Ile Leu Leu Ile Ile Val Tyr Trp Asp Ser Ala Gly
20 25 30

Ala Ala His Phe Tyr Leu His Thr Ser Phe Ser Arg Pro His Thr
35 40 45

Gly Pro Pro Leu Pro Thr Pro Gly Pro Asp Arg Asp Arg Glu Leu
50 55 60

Thr Ala Asp Ser Asp Val Asp Glu Phe Leu Asp Lys Phe Leu Ser
65 70 75

Ala Gly Val Lys Gln Ser Asp Leu Pro Arg Lys Glu Thr Glu Gln
80 85 90

Pro Pro Ala Pro Gly Ser Met Glu Glu Ser Val Arg Gly Tyr Asp
95 100 105

Trp Ser Pro Arg Asp Ala Arg Arg Ser Pro Asp Gln Gly Arg Gln
110 115 120

Gln Ala Glu Arg Arg Ser Val Leu Arg Gly Phe Cys Ala Asn Ser
125 130 135

Ser Leu Ala Phe Pro Thr Lys Glu Arg Ala Phe Asp Asp Ile Pro
140 145 150

Asn Ser Glu Leu Ser His Leu Ile Val Asp Asp Arg His Gly Ala
155 160 165

Ile Tyr Cys Tyr Val Pro Lys Val Ala Cys Thr Asn Trp Lys Arg
170 175 180

Val Met Ile Val Leu Ser Gly Ser Leu Leu His Arg Gly Ala Pro
185 190 195

Tyr Arg Asp Pro Leu Arg Ile Pro Arg Glu His Val His Asn Ala
200 205 210

Ser Ala His Leu Thr Phe Asn Lys Phe Trp Arg Arg Tyr Gly Lys
215 220 225

Leu Ser Arg His Leu Met Lys Val Lys Leu Lys Lys Tyr Thr Lys
230 235 240

Phe Leu Phe Val Arg Asp Pro Phe Val Arg Leu Ile Ser Ala Phe
245 250 255

Arg Ser Lys Phe Glu Leu Glu Asn Glu Glu Phe Tyr Arg Lys Phe
260 265 270

Ala Val Pro Met Leu Arg Leu Tyr Ala Asn His Thr Ser Leu Pro
275 280 285

```
Ala Ser Ala Arg Glu Ala Phe Arg Ala Gly Leu Lys Val Ser Phe
                290                 295                 300

Ala Asn Phe Ile Gln Tyr Leu Leu Asp Pro His Thr Glu Lys Leu
                305                 310                 315

Ala Pro Phe Asn Glu His Trp Arg Gln Val Tyr Arg Leu Cys His
                320                 325                 330

Pro Cys Gln Ile Asp Tyr Asp Phe Val Gly Lys Leu Glu Thr Leu
                335                 340                 345

Asp Glu Asp Ala Ala Gln Leu Leu Gln Leu Leu Gln Val Asp Arg
                350                 355                 360

Gln Leu Arg Phe Pro Pro Ser Tyr Arg Asn Arg Thr Ala Ser Ser
                365                 370                 375

Trp Glu Glu Asp Trp Phe Ala Lys Ile Pro Leu Ala Trp Arg Gln
                380                 385                 390

Gln Leu Tyr Lys Leu Tyr Glu Ala Asp Phe Val Leu Phe Gly Tyr
                395                 400                 405

Pro Lys Pro Glu Asn Leu Leu Arg Asp
                410
```

<210> 419
<211> 1781
<212> DNA
<213> Homo Sapien

<400> 419

```
ggcacgaggc tgaacccagc cggctccatc tcagcttctg gtttctaagt 50

ccatgtgcca aaggctgcca ggaaggagac gccttcctga gtcctggatc 100

tttcttcctt ctggaaatct ttgactgtgg gtagttattt atttctgaat 150

aagagcgtcc acgcatcatg gacctcgcgg gactgctgaa gtctcagttc 200

ctgtgccacc tggtcttctg ctacgtcttt attgcctcag ggctaatcat 250

caacaccatt cagctcttca ctctcctcct ctggcccatt aacaagcagc 300

tcttccggaa gatcaactgc agactgtcct attgcatctc aagccagctg 350

gtgatgctgc tggagtggtg gtcgggcacg gaatgcacca tcttcacgga 400

cccgcgcgcc tacctcaagt atgggaagga aaatgccatc gtggttctca 450

accacaagtt tgaaattgac tttctgtgtg gctggagcct gtccgaacgc 500

tttgggctgt taggggggctc caaggtcctg gccaagaaag agctggccta 550

tgtcccaatt atcggctgga tgtggtactt caccgagatg gtcttctgtt 600

cgcgcaagtg ggagcaggat cgcaagacgg ttgccaccag tttgcagcac 650

ctccgggact accccgagaa gtattttttc ctgattcact gtgagggcac 700

acggttcacg gagaagaagc atgagatcag catgcaggtg gcccgggcca 750
```

aggggctgcc tcgcctcaag catcacctgt tgccacgaac caagggcttc 800

gccatcaccg tgaggagctt gagaaatgta gtttcagctg tatatgactg 850

tacactcaat ttcagaaata atgaaaatcc aacactgctg ggagtcctaa 900

acggaaagaa ataccatgca gatttgtatg ttaggaggat cccactggaa 950

gacatccctg aagacgatga cgagtgctcg gcctggctgc acaagctcta 1000

ccaggagaag gatgcctttc aggaggagta ctacaggacg ggcaccttcc 1050

cagagacgcc catggtgccc ccccggcggc cctggaccct cgtgaactgg 1100

ctgttttggg cctcgctggt gctctaccct ttcttccagt tcctggtcag 1150

catgatcagg agcgggtctt ccctgacgct ggccagcttc atcctcgtct 1200

tctttgtggc ctccgtggga gttcgatgga tgattggtgt gacggaaatt 1250

gacaagggct ctgcctacgg caactctgac agcaagcaga aactgaatga 1300

ctgactcagg gaggtgtcac catccgaagg gaaccttggg gaactggtgg 1350

cctctgcata tcctccttag tgggacacgg tgacaaaggc tgggtgagcc 1400

cctgctgggc acggcggaag tcacgacctc tccagccagg gagtctggtc 1450

tcaaggccgg atggggagga agatgttttg taatcttttt ttccccatgt 1500

gctttagtgg gctttggttt tctttttgtg cgagtgtgtg tgagaatggc 1550

tgtgtggtga gtgtgaactt tgttctgtga tcatagaaag ggtattttag 1600

gctgcagggg agggcagggc tggggaccga aggggacaag ttcccctttc 1650

atcctttggt gctgagtttt ctgtaaccct tggttgccag agataaagtg 1700

aaaagtgctt taggtgagat gactaaatta tgcctccaag aaaaaaaaat 1750

taaagtgctt ttctgggtca aaaaaaaaaa a 1781

<210> 420
<211> 378
<212> PRT
<213> Homo Sapien

<400> 420

```
Met Asp Leu Ala Gly Leu Leu Lys Ser Gln Phe Leu Cys His Leu
  1               5                  10                  15

Val Phe Cys Tyr Val Phe Ile Ala Ser Gly Leu Ile Ile Asn Thr
                 20                  25                  30

Ile Gln Leu Phe Thr Leu Leu Leu Trp Pro Ile Asn Lys Gln Leu
                 35                  40                  45

Phe Arg Lys Ile Asn Cys Arg Leu Ser Tyr Cys Ile Ser Ser Gln
                 50                  55                  60

Leu Val Met Leu Leu Glu Trp Trp Ser Gly Thr Glu Cys Thr Ile
                 65                  70                  75

Phe Thr Asp Pro Arg Ala Tyr Leu Lys Tyr Gly Lys Glu Asn Ala
```

80 85 90

Ile Val Val Leu Asn His Lys Phe Glu Ile Asp Phe Leu Cys Gly
95 100 105

Trp Ser Leu Ser Glu Arg Phe Gly Leu Leu Gly Gly Ser Lys Val
110 115 120

Leu Ala Lys Lys Glu Leu Ala Tyr Val Pro Ile Ile Gly Trp Met
125 130 135

Trp Tyr Phe Thr Glu Met Val Phe Cys Ser Arg Lys Trp Glu Gln
140 145 150

Asp Arg Lys Thr Val Ala Thr Ser Leu Gln His Leu Arg Asp Tyr
155 160 165

Pro Glu Lys Tyr Phe Phe Leu Ile His Cys Glu Gly Thr Arg Phe
170 175 180

Thr Glu Lys Lys His Glu Ile Ser Met Gln Val Ala Arg Ala Lys
185 190 195

Gly Leu Pro Arg Leu Lys His His Leu Leu Pro Arg Thr Lys Gly
200 205 210

Phe Ala Ile Thr Val Arg Ser Leu Arg Asn Val Val Ser Ala Val
215 220 225

Tyr Asp Cys Thr Leu Asn Phe Arg Asn Asn Glu Asn Pro Thr Leu
230 235 240

Leu Gly Val Leu Asn Gly Lys Lys Tyr His Ala Asp Leu Tyr Val
245 250 255

Arg Arg Ile Pro Leu Glu Asp Ile Pro Glu Asp Asp Asp Glu Cys
260 265 270

Ser Ala Trp Leu His Lys Leu Tyr Gln Glu Lys Asp Ala Phe Gln
275 280 285

Glu Glu Tyr Tyr Arg Thr Gly Thr Phe Pro Glu Thr Pro Met Val
290 295 300

Pro Pro Arg Arg Pro Trp Thr Leu Val Asn Trp Leu Phe Trp Ala
305 310 315

Ser Leu Val Leu Tyr Pro Phe Phe Gln Phe Leu Val Ser Met Ile
320 325 330

Arg Ser Gly Ser Ser Leu Thr Leu Ala Ser Phe Ile Leu Val Phe
335 340 345

Phe Val Ala Ser Val Gly Val Arg Trp Met Ile Gly Val Thr Glu
350 355 360

Ile Asp Lys Gly Ser Ala Tyr Gly Asn Ser Asp Ser Lys Gln Lys
365 370 375

Leu Asn Asp

<210> 421
<211> 1355

<212> DNA
<213> Homo Sapien

<400> 421
cggacgcgtg ggcggacgcg tgggcggacg cgtgggcgga cgcgtgggct 50

gggtgcctgc atcgccatgg acaccaccag gtacagcaag tggggcggca 100

gctccgagga ggtccccgga gggccctggg gacgctgggt gcactggagc 150

aggagacccc tcttcttggc cctggctgtc ctggtcacca cagtcctttg 200

ggctgtgatt ctgagtatcc tattgtccaa ggcctccacg gagcgcgcgg 250

cgctgcttga cggccacgac ctgctgagga caaacgcctc gaagcagacg 300

gcggcgctgg gtgccctgaa ggaggaggtc ggagactgcc acagctgctg 350

ctcggggacg caggcgcagc tgcagaccac gcgcgcggag cttggggagg 400

cgcaggcgaa gctgatggag caggagagcg ccctgcggga actgcgtgag 450

cgcgtgaccc agggcttggc tgaagccggc aggggccgtg aggacgtccg 500

cactgagctg ttccgggcgc tggaggccgt gaggctccag aacaactcct 550

gcgagccgtg ccccacgtcg tggctgtcct tcgagggctc ctgctacttt 600

ttctctgtgc caaagacgac gtgggcggcg gcgcaggatc actgcgcaga 650

tgccagcgcg cacctggtga tcgttggggg cctggatgag cagggcttcc 700

tcactcggaa cacgcgtggc cgtggttact ggctgggcct gagggctgtg 750

cgccatctgg gcaaggttca gggctaccag tgggtggacg gagtctctct 800

cagcttcagc cactggaacc agggagagcc caatgacgct tgggggcgcg 850

agaactgtgt catgatgctg cacacggggc tgtggaacga cgcaccgtgt 900

gacagcgaga aggacggctg gatctgtgag aaaaggcaca actgctgacc 950

ccgcccagtg ccctggagcc gcgcccattg cagcatgtcg tatcctgggg 1000

gctgctcacc tccctggctc ctggagctga ttgccaaaga gtttttttct 1050

tcctcatcca ccgctgctga gtctcagaaa cacttggccc aacatagccc 1100

tgtccagccc agtgcctggg ctctgggacc tccatgccga cctcatccta 1150

actccactca cgcagaccca acctaacctc cactagctcc aaaatccctg 1200

ctcctgcgtc cccgtgatat gcctccactt ctctccctaa ccaaggttag 1250

gtgactgagg actggagctg tttggttttc tcgcattttc caccaaactg 1300

gaagctgttt ttgcagcctg aggaagcatc aataaatatt tgagaaatga 1350

aaaaa 1355

<210> 422
<211> 293
<212> PRT
<213> Homo Sapien

<400> 422

```
Met Asp Thr Thr Arg Tyr Ser Lys Trp Gly Gly Ser Ser Glu Glu
  1               5                  10                  15

Val Pro Gly Gly Pro Trp Gly Arg Trp Val His Trp Ser Arg Arg
                 20                  25                  30

Pro Leu Phe Leu Ala Leu Ala Val Leu Val Thr Thr Val Leu Trp
                 35                  40                  45

Ala Val Ile Leu Ser Ile Leu Leu Ser Lys Ala Ser Thr Glu Arg
                 50                  55                  60

Ala Ala Leu Leu Asp Gly His Asp Leu Leu Arg Thr Asn Ala Ser
                 65                  70                  75

Lys Gln Thr Ala Ala Leu Gly Ala Leu Lys Glu Glu Val Gly Asp
                 80                  85                  90

Cys His Ser Cys Cys Ser Gly Thr Gln Ala Gln Leu Gln Thr Thr
                 95                 100                 105

Arg Ala Glu Leu Gly Glu Ala Gln Ala Lys Leu Met Glu Gln Glu
                110                 115                 120

Ser Ala Leu Arg Glu Leu Arg Glu Arg Val Thr Gln Gly Leu Ala
                125                 130                 135

Glu Ala Gly Arg Gly Arg Glu Asp Val Arg Thr Glu Leu Phe Arg
                140                 145                 150

Ala Leu Glu Ala Val Arg Leu Gln Asn Asn Ser Cys Glu Pro Cys
                155                 160                 165

Pro Thr Ser Trp Leu Ser Phe Glu Gly Ser Cys Tyr Phe Phe Ser
                170                 175                 180

Val Pro Lys Thr Thr Trp Ala Ala Ala Gln Asp His Cys Ala Asp
                185                 190                 195

Ala Ser Ala His Leu Val Ile Val Gly Gly Leu Asp Glu Gln Gly
                200                 205                 210

Phe Leu Thr Arg Asn Thr Arg Gly Arg Gly Tyr Trp Leu Gly Leu
                215                 220                 225

Arg Ala Val Arg His Leu Gly Lys Val Gln Gly Tyr Gln Trp Val
                230                 235                 240

Asp Gly Val Ser Leu Ser Phe Ser His Trp Asn Gln Gly Glu Pro
                245                 250                 255

Asn Asp Ala Trp Gly Arg Glu Asn Cys Val Met Met Leu His Thr
                260                 265                 270

Gly Leu Trp Asn Asp Ala Pro Cys Asp Ser Glu Lys Asp Gly Trp
                275                 280                 285

Ile Cys Glu Lys Arg His Asn Cys
                290
```

<210> 423
<211> 2368

<212> DNA
<213> Homo Sapien

<400> 423
gcgccgccag gcgtaggcgg ggtggccctt gcgtctcccg cttccttgaa 50

aaacccggcg ggcgagcgag gctgcgggcc ggccgctgcc cttccccaca 100

ctccccgccg agaagcctcg ctcggcgccc aacatggcgg gtgggcgctg 150

cggcccgcag ctaacggcgc tcctggccgc ctggatcgcg gctgtggcgg 200

cgacggcagg ccccgaggag gccgcgctgc cgccggagca gagccgggtc 250

cagcccatga ccgcctccaa ctggacgctg gtgatggagg gcgagtggat 300

gctgaaattt tacgccccat ggtgtccatc ctgccagcag actgattcag 350

aatgggaggc ttttgcaaag aatggtgaaa tacttcagat cagtgtgggg 400

aaggtagatg tcattcaaga accaggtttg agtggccgct tctttgtcac 450

cactctccca gcatttttc atgcaaagga tgggatattc cgccgttatc 500

gtggcccagg aatcttcgaa gacctgcaga attatatctt agagaagaaa 550

tggcaatcag tcgagcctct gactggctgg aaatccccag cttctctaac 600

gatgtctgga atggctggtc tttttagcat ctctggcaag atatggcatc 650

ttcacaacta tttcacagtg actcttggaa ttcctgcttg gtgttcttat 700

gtgtttttcg tcatagccac cttggttttt ggcctttta tgggtctggt 750

cttggtggta atatcagaat gtttctatgt gccacttcca aggcatttat 800

ctgagcgttc tgagcagaat cggagatcag aggaggctca tagagctgaa 850

cagttgcagg atgcggagga ggaaaaagat gattcaaatg aagaagaaaa 900

caaagacagc cttgtagatg atgaagaaga gaaagaagat cttggcgatg 950

aggatgaagc agaggaagaa gaggaggagg acaacttggc tgctggtgtg 1000

gatgaggaga gaagtgaggc caatgatcag gggcccccag gagaggacgg 1050

tgtgacccgg gaggaagtag agcctgagga ggctgaagaa ggcatctctg 1100

agcaaccctg cccagctgac acagaggtgg tggaagactc cttgaggcag 1150

cgtaaaagtc agcatgctga caagggactg tagatttaat gatgcgtttt 1200

caagaataca caccaaaaca atatgtcagc ttccctttgg cctgcagttt 1250

gtaccaaatc cttaattttt cctgaatgag caagcttctc ttaaaagatg 1300

ctctctagtc atttggtctc atggcagtaa gcctcatgta tactaaggag 1350

agtcttccag gtgtgacaat caggatatag aaaaacaaac gtagtgttgg 1400

gatctgtttg gagactggga tgggaacaag ttcatttact taggggtcag 1450

agagtctcga ccagaggagg ccattcccag tcctaatcag caccttccag 1500

agacaaggct gcaggccctg tgaaatgaaa gccaagcagg agccttggct 1550

cctgagcatc cccaaagtgt aacgtagaag ccttgcatcc ttttcttgtg 1600

taaagtattt atttttgtca aattgcagga aacatcaggc accacagtgc 1650

atgaaaaatc tttcacagct agaaattgaa agggccttgg gtatagagag 1700

cagctcagaa gtcatcccag ccctctgaat ctcctgtgct atgttttatt 1750

tcttaccttt aatttttcca gcatttccac catgggcatt caggctctcc 1800

acactcttca ctattatctc ttggtcagag gactccaata acagccaggt 1850

ttacatgaac tgtgtttgtt cattctgacc taaggggttt agataatcag 1900

taaccataac ccctgaagct gtgactgcca aacatctcaa atgaaatgtt 1950

gtggccatca gagactcaaa aggaagtaag gattttacaa gacagattaa 2000

aaaaaaattg ttttgtccaa aatatagttg ttgttgattt ttttttaagt 2050

tttctaagca atatttttca agccagaagt cctctaagtc ttgccagtac 2100

aaggtagtct tgtgaagaaa agttgaatac tgttttgttt tcatctcaag 2150

gggttccctg ggtcttgaac tactttaata ataactaaaa aaccacttct 2200

gattttcctt cagtgatgtg cttttggtga aagaattaat gaactccagt 2250

acctgaaagt gaaagatttg attttgtttc catcttctgt aatcttccaa 2300

agaattatat ctttgtaaat ctctcaatac tcaatctact gtaagtaccc 2350

agggaggcta atttcttt 2368

<210> 424
<211> 349
<212> PRT
<213> Homo Sapien

<400> 424
Met Ala Gly Gly Arg Cys Gly Pro Gln Leu Thr Ala Leu Leu Ala
1 5 10 15

Ala Trp Ile Ala Ala Val Ala Ala Thr Ala Gly Pro Glu Glu Ala
20 25 30

Ala Leu Pro Pro Glu Gln Ser Arg Val Gln Pro Met Thr Ala Ser
35 40 45

Asn Trp Thr Leu Val Met Glu Gly Glu Trp Met Leu Lys Phe Tyr
50 55 60

Ala Pro Trp Cys Pro Ser Cys Gln Gln Thr Asp Ser Glu Trp Glu
65 70 75

Ala Phe Ala Lys Asn Gly Glu Ile Leu Gln Ile Ser Val Gly Lys
80 85 90

Val Asp Val Ile Gln Glu Pro Gly Leu Ser Gly Arg Phe Phe Val
95 100 105

Thr Thr Leu Pro Ala Phe Phe His Ala Lys Asp Gly Ile Phe Arg

110 115 120

Arg Tyr Arg Gly Pro Gly Ile Phe Glu Asp Leu Gln Asn Tyr Ile
125 130 135

Leu Glu Lys Lys Trp Gln Ser Val Glu Pro Leu Thr Gly Trp Lys
140 145 150

Ser Pro Ala Ser Leu Thr Met Ser Gly Met Ala Gly Leu Phe Ser
155 160 165

Ile Ser Gly Lys Ile Trp His Leu His Asn Tyr Phe Thr Val Thr
170 175 180

Leu Gly Ile Pro Ala Trp Cys Ser Tyr Val Phe Phe Val Ile Ala
185 190 195

Thr Leu Val Phe Gly Leu Phe Met Gly Leu Val Leu Val Val Ile
200 205 210

Ser Glu Cys Phe Tyr Val Pro Leu Pro Arg His Leu Ser Glu Arg
215 220 225

Ser Glu Gln Asn Arg Arg Ser Glu Glu Ala His Arg Ala Glu Gln
230 235 240

Leu Gln Asp Ala Glu Glu Glu Lys Asp Asp Ser Asn Glu Glu Glu
245 250 255

Asn Lys Asp Ser Leu Val Asp Asp Glu Glu Glu Lys Glu Asp Leu
260 265 270

Gly Asp Glu Asp Glu Ala Glu Glu Glu Glu Glu Glu Asp Asn Leu
275 280 285

Ala Ala Gly Val Asp Glu Glu Arg Ser Glu Ala Asn Asp Gln Gly
290 295 300

Pro Pro Gly Glu Asp Gly Val Thr Arg Glu Glu Val Glu Pro Glu
305 310 315

Glu Ala Glu Glu Gly Ile Ser Glu Gln Pro Cys Pro Ala Asp Thr
320 325 330

Glu Val Val Glu Asp Ser Leu Arg Gln Arg Lys Ser Gln His Ala
335 340 345

Asp Lys Gly Leu

<210> 425
<211> 4040
<212> DNA
<213> Homo Sapien

<400> 425
gaggaaccta ccggtaccgg ccgcgcgctg gtagtcgccg gtgtggctgc 50

acctcaccaa tcccgtgcgc cgcggctggg ccgtcggaga gtgcgtgtgc 100

ttctctcctg cacgcggtgc ttgggctcgg ccaggcgggg tccgccgcca 150

gggtttgagg atgggggagt agctacagga agcgaccccg cgatggcaag 200

gtatattttt gtggaatgaa aaggaagtat tagaaatgag ctgaagacca 250
ttcacagatt aatatttttg gggacagatt tgtgatgctt gattcaccct 300
tgaagtaatg tagacagaag ttctcaaatt tgcatattac atcaactgga 350
accagcagtg aatcttaatg ttcacttaaa tcagaacttg cataagaaag 400
agaatgggag tctggttaaa taaagatgac tatatcagag acttgaaaag 450
gatcattctc tgttttctga tagtgtatat ggccatttta gtgggcacag 500
atcaggattt ttacagttta cttggagtgt ccaaaactgc aagcagtaga 550
gaaataagac aagctttcaa gaaattggca ttgaagttac atcctgataa 600
aaacccgaat aacccaaatg cacatggcga ttttttaaaa ataaatagag 650
catatgaagt actcaaagat gaagatctac ggaaaaagta tgacaaatat 700
ggagaaaagg gacttgagga taatcaaggt ggccagtatg aaagctggaa 750
ctattatcgt tatgattttg gtatttatga tgatgatcct gaaatcataa 800
cattggaaag aagagaattt gatgctgctg ttaattctgg agaactgtgg 850
tttgtaaatt tttactcccc aggctgttca cactgccatg atttagctcc 900
cacatggaga gactttgcta aagaagtgga tgggttactt cgaattggag 950
ctgttaactg tggtgatgat agaatgcttt gccgaatgaa aggagtcaac 1000
agctatccca gtctcttcat tttcggtct ggaatggccc cagtgaaata 1050
tcatggagac agatcaaagg agagtttagt gagttttgca atgcagcatg 1100
ttagaagtac agtgacagaa ctttggacag gaaattttgt caactccata 1150
caaactgctt ttgctgctgg tattggctgg ctgatcactt tttgttcaaa 1200
aggaggagat tgtttgactt cacagacacg actcaggctt agtggcatgt 1250
tgtttctcaa ctcattggat gctaaagaaa tatatttgga agtaatacat 1300
aatcttccag attttgaact actttcggca aacacactag aggatcgttt 1350
ggctcatcat cggtggctgt tattttttca ttttggaaaa aatgaaaatt 1400
caaatgatcc tgagctgaaa aaactaaaaa ctctacttaa aaatgatcat 1450
attcaagttg gcaggtttga ctgttcctct gcaccagaca tctgtagtaa 1500
tctgtatgtt tttcagccgt ctctagcagt atttaaagga caaggaacca 1550
aagaatatga aattcatcat ggaaagaaga ttctatatga tatacttgcc 1600
tttgccaaag aaagtgtgaa ttctcatgtt accacgcttg gacctcaaaa 1650
ttttcctgcc aatgacaaag aaccatggct tgttgatttc tttgccccct 1700
ggtgtccacc atgtcgagct ttactaccag agttacgaag agcatcaaat 1750
cttctttatg gtcagcttaa gtttggtaca ctagattgta cagttcatga 1800

gggactctgt aacatgtata acattcaggc ttatccaaca acagtggtat 1850
tcaaccagtc caacattcat gagtatgaag gacatcactc tgctgaacaa 1900
atcttggagt tcatagagga tcttatgaat ccttcagtgg tctcccttac 1950
acccaccacc ttcaacgaac tagttacaca aagaaaacac aacgaagtct 2000
ggatggttga tttctattct ccgtggtgtc atccttgcca agtcttaatg 2050
ccagaatgga aaagaatggc ccggacatta actggactga tcaacgtggg 2100
cagtatagat tgccaacagt atcattcttt ttgtgcccag gaaaacgttc 2150
aaagataccc tgagataaga ttttttcccc caaaatcaaa taaagcttat 2200
cagtatcaca gttacaatgg ttggaatagg gatgcttatt ccctgagaat 2250
ctggggtcta ggatttttac ctcaagtatc cacagatcta acacctcaga 2300
ctttcagtga aaaagttcta caagggaaaa atcattgggt gattgatttc 2350
tatgctcctt ggtgtggacc ttgccagaat tttgctccag aatttgagct 2400
cttggctagg atgattaaag gaaaagtgaa agctggaaaa gtagactgtc 2450
aggcttatgc tcagacatgc cagaaagctg ggatcagggc ctatccaact 2500
gttaagtttt atttctacga aagagcaaag agaaattttc aagaagagca 2550
gataaatacc agagatgcaa aagcaatcgc tgccttaata agtgaaaaat 2600
tggaaactct ccgaaatcaa ggcaagagga ataaggatga actttgataa 2650
tgttgaagat gaagaaaaag tttaaaagaa attctgacag atgacatcag 2700
aagacaccta tttagaatgt tacatttatg atgggaatga atgaacatta 2750
tcttagactt gcagttgtac tgccagaatt atctacagca ctggtgtaaa 2800
agaagggtct gcaaactttt tctgtaaagg gccggtttat aaatatttta 2850
gactttgcag gctataatat atggttcaca catgagaaca agaatagagt 2900
catcatgtat tctttgttat ttgcttttaa caacctttaa aaaatattaa 2950
aacgattctt agctcagagc catacaaaag taggctggat tcagtccatg 3000
gaccatagat tgctgtcccc ctcgacggac ttataatgtt tcaggtggct 3050
ggcttgaaca tgagtctgct gtgctatcta cataaatgtc taagttgtat 3100
aaagtccact ttcccttcac gtttttggc tgacctgaaa agaggtaact 3150
tagtttttgg tcacttgttc tcctaaaaat gctatcccta accatatatt 3200
tatatttcgt tttaaaaaca cccatgatgt ggcacagtaa acaaaccctg 3250
ttatgctgta ttattatgag gagattcttc attgttttct ttccttctca 3300
aaggttgaaa aaatgctttt aatttttcac agccgagaaa cagtgcagca 3350
gtatatgtgc acacagtaag tacacaaatt tgagcaacag taagtgcaca 3400

aattctgtag tttgctgtat catccaggaa aacctgaggg aaaaaaatta 3450

tagcaattaa ctgggcattg tagagtatcc taaatatgtt atcaagtatt 3500

tagagttcta tattttaaag atatatgtgt tcatgtattt tctgaaattg 3550

ctttcataga aattttccca ctgatagttg atttttgagg catctaatat 3600

ttacatattt gccttctgaa ctttgttttg acctgtatcc tttatttaca 3650

ttgggttttt ctttcatagt tttggttttt cactcctgtc cagtctattt 3700

attattcaaa taggaaaaat tactttacag gttgttttac tgtagcttat 3750

aatgatactg tagttattcc agttactagt ttactgtcag agggctgcct 3800

ttttcagata aatattgaca taataactga agttattttt ataagaaaat 3850

caagtatata aatctaggaa agggatcttc tagtttctgt gttgtttaga 3900

ctcaaagaat cacaaatttg tcagtaacat gtagttgttt agttataatt 3950

cagagtgtac agaatggtaa aaattccaat cagtcaaaag aggtcaatga 4000

attaaaaggc ttgcaacttt ttcaaaaaaa aaaaaaaaaa 4040

<210> 426
<211> 747
<212> PRT
<213> Homo Sapien

<400> 426
Met Gly Val Trp Leu Asn Lys Asp Asp Tyr Ile Arg Asp Leu Lys
1 5 10 15

Arg Ile Ile Leu Cys Phe Leu Ile Val Tyr Met Ala Ile Leu Val
20 25 30

Gly Thr Asp Gln Asp Phe Tyr Ser Leu Leu Gly Val Ser Lys Thr
35 40 45

Ala Ser Ser Arg Glu Ile Arg Gln Ala Phe Lys Lys Leu Ala Leu
50 55 60

Lys Leu His Pro Asp Lys Asn Pro Asn Asn Pro Asn Ala His Gly
65 70 75

Asp Phe Leu Lys Ile Asn Arg Ala Tyr Glu Val Leu Lys Asp Glu
80 85 90

Asp Leu Arg Lys Lys Tyr Asp Lys Tyr Gly Glu Lys Gly Leu Glu
95 100 105

Asp Asn Gln Gly Gly Gln Tyr Glu Ser Trp Asn Tyr Tyr Arg Tyr
110 115 120

Asp Phe Gly Ile Tyr Asp Asp Asp Pro Glu Ile Ile Thr Leu Glu
125 130 135

Arg Arg Glu Phe Asp Ala Ala Val Asn Ser Gly Glu Leu Trp Phe
140 145 150

Val Asn Phe Tyr Ser Pro Gly Cys Ser His Cys His Asp Leu Ala
155 160 165

```
Pro Thr Trp Arg Asp Phe Ala Lys Glu Val Asp Gly Leu Leu Arg
                170                 175                 180

Ile Gly Ala Val Asn Cys Gly Asp Asp Arg Met Leu Cys Arg Met
                185                 190                 195

Lys Gly Val Asn Ser Tyr Pro Ser Leu Phe Ile Phe Arg Ser Gly
                200                 205                 210

Met Ala Pro Val Lys Tyr His Gly Asp Arg Ser Lys Glu Ser Leu
                215                 220                 225

Val Ser Phe Ala Met Gln His Val Arg Ser Thr Val Thr Glu Leu
                230                 235                 240

Trp Thr Gly Asn Phe Val Asn Ser Ile Gln Thr Ala Phe Ala Ala
                245                 250                 255

Gly Ile Gly Trp Leu Ile Thr Phe Cys Ser Lys Gly Gly Asp Cys
                260                 265                 270

Leu Thr Ser Gln Thr Arg Leu Arg Leu Ser Gly Met Leu Phe Leu
                275                 280                 285

Asn Ser Leu Asp Ala Lys Glu Ile Tyr Leu Glu Val Ile His Asn
                290                 295                 300

Leu Pro Asp Phe Glu Leu Leu Ser Ala Asn Thr Leu Glu Asp Arg
                305                 310                 315

Leu Ala His His Arg Trp Leu Leu Phe Phe His Phe Gly Lys Asn
                320                 325                 330

Glu Asn Ser Asn Asp Pro Glu Leu Lys Lys Leu Lys Thr Leu Leu
                335                 340                 345

Lys Asn Asp His Ile Gln Val Gly Arg Phe Asp Cys Ser Ser Ala
                350                 355                 360

Pro Asp Ile Cys Ser Asn Leu Tyr Val Phe Gln Pro Ser Leu Ala
                365                 370                 375

Val Phe Lys Gly Gln Gly Thr Lys Glu Tyr Glu Ile His His Gly
                380                 385                 390

Lys Lys Ile Leu Tyr Asp Ile Leu Ala Phe Ala Lys Glu Ser Val
                395                 400                 405

Asn Ser His Val Thr Thr Leu Gly Pro Gln Asn Phe Pro Ala Asn
                410                 415                 420

Asp Lys Glu Pro Trp Leu Val Asp Phe Phe Ala Pro Trp Cys Pro
                425                 430                 435

Pro Cys Arg Ala Leu Leu Pro Glu Leu Arg Arg Ala Ser Asn Leu
                440                 445                 450

Leu Tyr Gly Gln Leu Lys Phe Gly Thr Leu Asp Cys Thr Val His
                455                 460                 465

Glu Gly Leu Cys Asn Met Tyr Asn Ile Gln Ala Tyr Pro Thr Thr
                470                 475                 480
```

```
Val Val Phe Asn Gln Ser Asn Ile His Glu Tyr Glu Gly His His
                485                 490                 495

Ser Ala Glu Gln Ile Leu Glu Phe Ile Glu Asp Leu Met Asn Pro
                500                 505                 510

Ser Val Val Ser Leu Thr Pro Thr Thr Phe Asn Glu Leu Val Thr
                515                 520                 525

Gln Arg Lys His Asn Glu Val Trp Met Val Asp Phe Tyr Ser Pro
                530                 535                 540

Trp Cys His Pro Cys Gln Val Leu Met Pro Glu Trp Lys Arg Met
                545                 550                 555

Ala Arg Thr Leu Thr Gly Leu Ile Asn Val Gly Ser Ile Asp Cys
                560                 565                 570

Gln Gln Tyr His Ser Phe Cys Ala Gln Glu Asn Val Gln Arg Tyr
                575                 580                 585

Pro Glu Ile Arg Phe Phe Pro Pro Lys Ser Asn Lys Ala Tyr Gln
                590                 595                 600

Tyr His Ser Tyr Asn Gly Trp Asn Arg Asp Ala Tyr Ser Leu Arg
                605                 610                 615

Ile Trp Gly Leu Gly Phe Leu Pro Gln Val Ser Thr Asp Leu Thr
                620                 625                 630

Pro Gln Thr Phe Ser Glu Lys Val Leu Gln Gly Lys Asn His Trp
                635                 640                 645

Val Ile Asp Phe Tyr Ala Pro Trp Cys Gly Pro Cys Gln Asn Phe
                650                 655                 660

Ala Pro Glu Phe Glu Leu Leu Ala Arg Met Ile Lys Gly Lys Val
                665                 670                 675

Lys Ala Gly Lys Val Asp Cys Gln Ala Tyr Ala Gln Thr Cys Gln
                680                 685                 690

Lys Ala Gly Ile Arg Ala Tyr Pro Thr Val Lys Phe Tyr Phe Tyr
                695                 700                 705

Glu Arg Ala Lys Arg Asn Phe Gln Glu Glu Gln Ile Asn Thr Arg
                710                 715                 720

Asp Ala Lys Ala Ile Ala Ala Leu Ile Ser Glu Lys Leu Glu Thr
                725                 730                 735

Leu Arg Asn Gln Gly Lys Arg Asn Lys Asp Glu Leu
                740                 745
```

<210> 427
<211> 1518
<212> DNA
<213> Homo Sapien

<400> 427
ctgcagtcag gactctggga ccgcaggggg ctcccggacc ctgactctgc 50

agccgaaccg gcacggtttc gtggggaccc aggcttgcaa agtgacggtc 100

atttctctt tctttctccc tcttgagtcc ttctgagatg atggctctgg 150

gcgcagcggg agctacccgg gtctttgtcg cgatggtagc ggcggctctc 200

ggcggccacc ctctgctggg agtgagcgcc accttgaact cggttctcaa 250

ttccaacgct atcaagaacc tgcccccacc gctgggcggc gctgcggggc 300

acccaggctc tgcagtcagc gccgcgccgg gaatcctgta cccgggcggg 350

aataagtacc agaccattga caactaccag ccgtacccgt gcgcagagga 400

cgaggagtgc ggcactgatg agtactgcgc tagtcccacc cgcggagggg 450

acgcaggcgt gcaaatctgt ctcgcctgca ggaagcgccg aaaacgctgc 500

atgcgtcacg ctatgtgctg ccccgggaat tactgcaaaa atggaatatg 550

tgtgtcttct gatcaaaatc atttccgagg agaaattgag gaaaccatca 600

ctgaaagctt tggtaatgat catagcacct tggatgggta ttccagaaga 650

accaccttgt cttcaaaaat gtatcacacc aaaggacaag aaggttctgt 700

ttgtctccgg tcatcagact gtgcctcagg attgtgttgt gctagacact 750

tctggtccaa gatctgtaaa cctgtcctga aagaaggtca agtgtgtacc 800

aagcatagga gaaaaggctc tcatggacta gaaatattcc agcgttgtta 850

ctgtggagaa ggtctgtctt gccggataca gaaagatcac catcaagcca 900

gtaattcttc taggcttcac acttgtcaga gacactaaac cagctatcca 950

aatgcagtga actcctttta tataatagat gctatgaaaa ccttttatga 1000

ccttcatcaa ctcaatccta aggatataca agttctgtgg tttcagttaa 1050

gcattccaat aacaccttcc aaaaacctgg agtgtaagag ctttgtttct 1100

ttatggaact cccctgtgat tgcagtaaat tactgtattg taaattctca 1150

gtgtggcact tacctgtaaa tgcaatgaaa cttttaatta tttttctaaa 1200

ggtgctgcac tgcctatttt tcctcttgtt atgtaaattt ttgtacacat 1250

tgattgttat cttgactgac aaatattcta tattgaactg aagtaaatca 1300

tttcagctta tagttcttaa aagcataacc ctttacccca tttaattcta 1350

gagtctagaa cgcaaggatc tcttggaatg acaaatgata ggtacctaaa 1400

atgtaacatg aaaatactag cttattttct gaaatgtact atcttaatgc 1450

ttaaattata tttcccttta ggctgtgata gtttttgaaa taaaatttaa 1500

catttaaaaa aaaaaaaa 1518

<210> 428
<211> 266
<212> PRT
<213> Homo Sapien

<400> 428

```
Met Met Ala Leu Gly Ala Ala Gly Ala Thr Arg Val Phe Val Ala
1               5                   10                  15

Met Val Ala Ala Ala Leu Gly Gly His Pro Leu Leu Gly Val Ser
                20                  25                  30

Ala Thr Leu Asn Ser Val Leu Asn Ser Asn Ala Ile Lys Asn Leu
                35                  40                  45

Pro Pro Pro Leu Gly Gly Ala Ala Gly His Pro Gly Ser Ala Val
                50                  55                  60

Ser Ala Ala Pro Gly Ile Leu Tyr Pro Gly Gly Asn Lys Tyr Gln
                65                  70                  75

Thr Ile Asp Asn Tyr Gln Pro Tyr Pro Cys Ala Glu Asp Glu Glu
                80                  85                  90

Cys Gly Thr Asp Glu Tyr Cys Ala Ser Pro Thr Arg Gly Gly Asp
                95                  100                 105

Ala Gly Val Gln Ile Cys Leu Ala Cys Arg Lys Arg Arg Lys Arg
                110                 115                 120

Cys Met Arg His Ala Met Cys Cys Pro Gly Asn Tyr Cys Lys Asn
                125                 130                 135

Gly Ile Cys Val Ser Ser Asp Gln Asn His Phe Arg Gly Glu Ile
                140                 145                 150

Glu Glu Thr Ile Thr Glu Ser Phe Gly Asn Asp His Ser Thr Leu
                155                 160                 165

Asp Gly Tyr Ser Arg Arg Thr Thr Leu Ser Ser Lys Met Tyr His
                170                 175                 180

Thr Lys Gly Gln Glu Gly Ser Val Cys Leu Arg Ser Ser Asp Cys
                185                 190                 195

Ala Ser Gly Leu Cys Cys Ala Arg His Phe Trp Ser Lys Ile Cys
                200                 205                 210

Lys Pro Val Leu Lys Glu Gly Gln Val Cys Thr Lys His Arg Arg
                215                 220                 225

Lys Gly Ser His Gly Leu Glu Ile Phe Gln Arg Cys Tyr Cys Gly
                230                 235                 240

Glu Gly Leu Ser Cys Arg Ile Gln Lys Asp His His Gln Ala Ser
                245                 250                 255

Asn Ser Ser Arg Leu His Thr Cys Gln Arg His
                260                 265
```

<210> 429
<211> 1523
<212> DNA
<213> Homo Sapien

<400> 429
gagaggacga ggtgccgctg cctggagaat cctccgctgc cgtcggctcc 50

cggagcccag ccctttccta acccaaccca acctagccca gtcccagccg 100

ccagcgcctg tccctgtcac ggaccccagc gttaccatgc atcctgccgt 150
cttcctatcc ttacccgacc tcagatgctc ccttctgctc ctggtaactt 200
gggtttttac tcctgtaaca actgaaataa caagtcttgc tacagagaat 250
atagatgaaa ttttaaacaa tgctgatgtt gctttagtaa atttttatgc 300
tgactggtgt cgtttcagtc agatgttgca tccaattttt gaggaagctt 350
ccgatgtcat taaggaagaa tttccaaatg aaaatcaagt agtgtttgcc 400
agagttgatt gtgatcagca ctctgacata gcccagagat acaggataag 450
caaataccca accctcaaat tgtttcgtaa tgggatgatg atgaagagag 500
aatacagggg tcagcgatca gtgaaagcat tggcagatta catcaggcaa 550
caaaaaagtg accccattca agaaattcgg gacttagcag aaatcaccac 600
tcttgatcgc agcaaaagaa atatcattgg atattttgag caaaaggact 650
cggacaacta tagagttttt gaacgagtag cgaatatttt gcatgatgac 700
tgtgcctttc tttctgcatt tggggatgtt tcaaaaccgg aaagatatag 750
tggcgacaac ataatctaca aaccaccagg gcattctgct ccggatatgg 800
tgtacttggg agctatgaca aattttgatg tgacttacaa ttggattcaa 850
gataaatgtg ttcctcttgt ccgagaaata acatttgaaa atggagagga 900
attgacagaa gaaggactgc cttttctcat actctttcac atgaaagaag 950
atacagaaag tttagaaata ttccagaatg aagtagctcg gcaattaata 1000
agtgaaaaag gtacaataaa ctttttacat gccgattgtg acaaatttag 1050
acatcctctt ctgcacatac agaaaactcc agcagattgt cctgtaatcg 1100
ctattgacag ctttaggcat atgtatgtgt ttggagactt caaagatgta 1150
ttaattcctg gaaaactcaa gcaattcgta tttgacttac attctggaaa 1200
actgcacaga gaattccatc atggacctga cccaactgat acagccccag 1250
gagagcaagc ccaagatgta gcaagcagtc cacctgagag ctccttccag 1300
aaactagcac ccagtgaata taggtatact ctattgaggg atcgagatga 1350
gctttaaaaa cttgaaaaac agtttgtaag cctttcaaca gcagcatcaa 1400
cctacgtggt ggaaatagta aacctatatt ttcataattc tatgtgtatt 1450
tttattttga ataaacagaa agaaatttaa aaaaaaaaaa aaaaaaaaaa 1500
aaaaaaaaaa aaaaaaaaaa aaa 1523

<210> 430
<211> 406
<212> PRT
<213> Homo Sapien

<400> 430

Met His Pro Ala Val Phe Leu Ser Leu Pro Asp Leu Arg Cys Ser
1 5 10 15

Leu Leu Leu Leu Val Thr Trp Val Phe Thr Pro Val Thr Thr Glu
20 25 30

Ile Thr Ser Leu Ala Thr Glu Asn Ile Asp Glu Ile Leu Asn Asn
35 40 45

Ala Asp Val Ala Leu Val Asn Phe Tyr Ala Asp Trp Cys Arg Phe
50 55 60

Ser Gln Met Leu His Pro Ile Phe Glu Glu Ala Ser Asp Val Ile
65 70 75

Lys Glu Glu Phe Pro Asn Glu Asn Gln Val Val Phe Ala Arg Val
80 85 90

Asp Cys Asp Gln His Ser Asp Ile Ala Gln Arg Tyr Arg Ile Ser
95 100 105

Lys Tyr Pro Thr Leu Lys Leu Phe Arg Asn Gly Met Met Met Lys
110 115 120

Arg Glu Tyr Arg Gly Gln Arg Ser Val Lys Ala Leu Ala Asp Tyr
125 130 135

Ile Arg Gln Gln Lys Ser Asp Pro Ile Gln Glu Ile Arg Asp Leu
140 145 150

Ala Glu Ile Thr Thr Leu Asp Arg Ser Lys Arg Asn Ile Ile Gly
155 160 165

Tyr Phe Glu Gln Lys Asp Ser Asp Asn Tyr Arg Val Phe Glu Arg
170 175 180

Val Ala Asn Ile Leu His Asp Asp Cys Ala Phe Leu Ser Ala Phe
185 190 195

Gly Asp Val Ser Lys Pro Glu Arg Tyr Ser Gly Asp Asn Ile Ile
200 205 210

Tyr Lys Pro Pro Gly His Ser Ala Pro Asp Met Val Tyr Leu Gly
215 220 225

Ala Met Thr Asn Phe Asp Val Thr Tyr Asn Trp Ile Gln Asp Lys
230 235 240

Cys Val Pro Leu Val Arg Glu Ile Thr Phe Glu Asn Gly Glu Glu
245 250 255

Leu Thr Glu Glu Gly Leu Pro Phe Leu Ile Leu Phe His Met Lys
260 265 270

Glu Asp Thr Glu Ser Leu Glu Ile Phe Gln Asn Glu Val Ala Arg
275 280 285

Gln Leu Ile Ser Glu Lys Gly Thr Ile Asn Phe Leu His Ala Asp
290 295 300

Cys Asp Lys Phe Arg His Pro Leu Leu His Ile Gln Lys Thr Pro
305 310 315

Ala Asp Cys Pro Val Ile Ala Ile Asp Ser Phe Arg His Met Tyr

320 325 330

Val Phe Gly Asp Phe Lys Asp Val Leu Ile Pro Gly Lys Leu Lys
335 340 345

Gln Phe Val Phe Asp Leu His Ser Gly Lys Leu His Arg Glu Phe
350 355 360

His His Gly Pro Asp Pro Thr Asp Thr Ala Pro Gly Glu Gln Ala
365 370 375

Gln Asp Val Ala Ser Ser Pro Pro Glu Ser Ser Phe Gln Lys Leu
380 385 390

Ala Pro Ser Glu Tyr Arg Tyr Thr Leu Leu Arg Asp Arg Asp Glu
395 400 405

Leu

<210> 431
<211> 1575
<212> DNA
<213> Homo Sapien

<400> 431
gagcaggacg gagccatgga ccccgccagg aaagcaggtg cccaggccat 50

gatctggact gcaggctggc tgctgctgct gctgcttcgc ggaggagcgc 100

aggccctgga gtgctacagc tgcgtgcaga aagcagatga cggatgctcc 150

ccgaacaaga tgaagacagt gaagtgcgcg ccgggcgtgg acgtctgcac 200

cgaggccgtg gggcggtgg agaccatcca cggacaattc tcgctggcag 250

tgcggggttg cggttcggga ctccccggca agaatgaccg cggcctggat 300

cttcacgggc ttctggcgtt catccagctg cagcaatgcg ctcaggatcg 350

ctgcaacgcc aagctcaacc tcacctcgcg ggcgctcgac ccggcaggta 400

atgagagtgc atacccgccc aacggcgtgg agtgctacag ctgtgtgggc 450

ctgagccggg aggcgtgcca gggtacatcg ccgccggtcg tgagctgcta 500

caacgccagc gatcatgtct acaagggctg cttcgacggc aacgtcacct 550

tgacggcagc taatgtgact gtgtccttgc ctgtccgggg ctgtgtccag 600

gatgaattct gcactcggga tggagtaaca ggcccagggt tcacgctcag 650

tggctcctgt tgccaggggt cccgctgtaa ctctgacctc cgcaacaaga 700

cctacttctc ccctcgaatc ccaccccttg tccggctgcc ccctccagag 750

cccacgactg tggcctcaac cacatctgtc accacttcta cctcggcccc 800

agtgagaccc acatccacca ccaaacccat gccagcgcca accagtcaga 850

ctccgagaca gggagtagaa cacgaggcct cccgggatga ggagcccagg 900

ttgactggag gcgccgctgg ccaccaggac cgcagcaatt cagggcagta 950

tcctgcaaaa gggggggcccc agcagcccca taataaaggc tgtgtggctc 1000
ccacagctgg attggcagcc cttctgttgg ccgtggctgc tggtgtccta 1050
ctgtgagctt ctccacctgg aaatttccct ctcacctact tctctggccc 1100
tgggtacccc tcttctcatc acttcctgtt cccaccactg gactgggctg 1150
gcccagcccc tgtttttcca acattcccca gtatccccag cttctgctgc 1200
gctggtttgc ggctttggga aataaaatac cgttgtatat attctgccag 1250
gggtgttcta gctttttgag gacagctcct gtatccttct catccttgtc 1300
tctccgcttg tcctcttgtg atgttaggac agagtgagag aagtcagctg 1350
tcacggggaa ggtgagagag aggatgctaa gcttcctact cactttctcc 1400
tagccagcct ggactttgga gcgtggggtg ggtgggacaa tggctcccca 1450
ctctaagcac tgcctcccct actccccgca tctttgggga atcggttccc 1500
catatgtctt ccttactaga ctgtgagctc ctcgaggggg ggcccggtac 1550
ccaattcgcc ctatagtgag tcgta 1575

<210> 432
<211> 346
<212> PRT
<213> Homo Sapien

<400> 432

```
Met Asp Pro Ala Arg Lys Ala Gly Ala Gln Ala Met Ile Trp Thr
  1               5                  10                  15

Ala Gly Trp Leu Leu Leu Leu Leu Leu Arg Gly Gly Ala Gln Ala
                 20                  25                  30

Leu Glu Cys Tyr Ser Cys Val Gln Lys Ala Asp Asp Gly Cys Ser
                 35                  40                  45

Pro Asn Lys Met Lys Thr Val Lys Cys Ala Pro Gly Val Asp Val
                 50                  55                  60

Cys Thr Glu Ala Val Gly Ala Val Glu Thr Ile His Gly Gln Phe
                 65                  70                  75

Ser Leu Ala Val Arg Gly Cys Gly Ser Gly Leu Pro Gly Lys Asn
                 80                  85                  90

Asp Arg Gly Leu Asp Leu His Gly Leu Leu Ala Phe Ile Gln Leu
                 95                 100                 105

Gln Gln Cys Ala Gln Asp Arg Cys Asn Ala Lys Leu Asn Leu Thr
                110                 115                 120

Ser Arg Ala Leu Asp Pro Ala Gly Asn Glu Ser Ala Tyr Pro Pro
                125                 130                 135

Asn Gly Val Glu Cys Tyr Ser Cys Val Gly Leu Ser Arg Glu Ala
                140                 145                 150

Cys Gln Gly Thr Ser Pro Pro Val Val Ser Cys Tyr Asn Ala Ser
                155                 160                 165
```

```
Asp His Val Tyr Lys Gly Cys Phe Asp Gly Asn Val Thr Leu Thr
                170                 175                 180

Ala Ala Asn Val Thr Val Ser Leu Pro Val Arg Gly Cys Val Gln
                185                 190                 195

Asp Glu Phe Cys Thr Arg Asp Gly Val Thr Gly Pro Gly Phe Thr
                200                 205                 210

Leu Ser Gly Ser Cys Cys Gln Gly Ser Arg Cys Asn Ser Asp Leu
                215                 220                 225

Arg Asn Lys Thr Tyr Phe Ser Pro Arg Ile Pro Pro Leu Val Arg
                230                 235                 240

Leu Pro Pro Pro Glu Pro Thr Thr Val Ala Ser Thr Thr Ser Val
                245                 250                 255

Thr Thr Ser Thr Ser Ala Pro Val Arg Pro Thr Ser Thr Thr Lys
                260                 265                 270

Pro Met Pro Ala Pro Thr Ser Gln Thr Pro Arg Gln Gly Val Glu
                275                 280                 285

His Glu Ala Ser Arg Asp Glu Glu Pro Arg Leu Thr Gly Gly Ala
                290                 295                 300

Ala Gly His Gln Asp Arg Ser Asn Ser Gly Gln Tyr Pro Ala Lys
                305                 310                 315

Gly Gly Pro Gln Gln Pro His Asn Lys Gly Cys Val Ala Pro Thr
                320                 325                 330

Ala Gly Leu Ala Ala Leu Leu Leu Ala Val Ala Ala Gly Val Leu
                335                 340                 345

Leu
```

<210> 433
<211> 1657
<212> DNA
<213> Homo Sapien

<400> 433

```
cgggactcgg cgggtcctcc tgggagtctc ggaggggacc ggctgtgcag 50

acgccatgga gttggtgctg gtcttcctct gcagcctgct ggcccccatg 100

gtcctggcca gtgcagctga aaaggagaag gaaatggacc cttttcatta 150

tgattaccag accctgagga ttgggggact ggtgttcgct gtggtcctct 200

tctcggttgg gatcctcctt atcctaagtc gcaggtgcaa gtgcagtttc 250

aatcagaagc cccgggcccc aggagatgag gaagcccagg tggagaacct 300

catcaccgcc aatgcaacag agccccagaa gcagagaact gaagtgcagc 350

catcaggtgg aagcctctgg aacctgaggc ggctgcttga acctttggat 400

gcaaatgtcg atgcttaaga aaaccggcca cttcagcaac agccctttcc 450
```

ccaggagaag ccaagaactt gtgtgtcccc caccctatcc cctctaacac 500

cattcctcca cctgatgatg caactaacac ttgcctcccc actgcagcct 550

gcggtcctgc ccacctcccg tgatgtgtgt gtgtgtgtgt gtgtgtgact 600

gtgtgtgttt gctaactgtg gtctttgtgg ctacttgttt gtggatggta 650

ttgtgtttgt tagtgaactg tggactcgct ttcccaggca ggggctgagc 700

cacatggcca tctgctcctc cctgcccccg tggccctcca tcaccttctg 750

ctcctaggag gctgcttgtt gcccgagacc agccccctcc cctgatttag 800

ggatgcgtag ggtaagagca cgggcagtgg tcttcagtcg tcttgggacc 850

tgggaaggtt tgcagcactt tgtcatcatt cttcatggac tcctttcact 900

cctttaacaa aaaccttgct tccttatccc acctgatccc agtctgaagg 950

tctcttagca actggagata caaagcaagg agctggtgag cccagcgttg 1000

acgtcaggca ggctatgccc ttccgtggtt aatttcttcc caggggcttc 1050

cacgaggagt ccccatctgc cccgcccctt cacagagcgc ccggggattc 1100

caggcccagg gcttctactc tgcccctggg gaatgtgtcc cctgcatatc 1150

ttctcagcaa taactccatg ggctctggga ccctacccct tccaaccttc 1200

cctgcttctg agacttcaat ctacagccca gctcatccag atgcagacta 1250

cagtccctgc aattgggtct ctggcaggca atagttgaag gactcctgtt 1300

ccgttggggc cagcacaccg ggatggatgg agggagagca gaggcctttg 1350

cttctctgcc tacgtcccct tagatgggca gcagaggcaa ctcccgcatc 1400

ctttgctctg cctgtcggtg gtcagagcgg tgagcgaggt gggttggaga 1450

ctcagcaggc tccgtgcagc ccttgggaac agtgagaggt tgaaggtcat 1500

aacgagagtg ggaactcaac ccagatcccg cccctcctgt cctctgtgtt 1550

cccgcggaaa ccaaccaaac cgtgcgctgt gacccattgc tgttctctgt 1600

atcgtgatct atcctcaaca acaacagaaa aaaggaataa aatatccttt 1650

gtttcct 1657

<210> 434
<211> 120
<212> PRT
<213> Homo Sapien

<400> 434

```
Met Glu Leu Val Leu Val Phe Leu Cys Ser Leu Leu Ala Pro Met
  1               5                  10                  15

Val Leu Ala Ser Ala Ala Glu Lys Glu Lys Glu Met Asp Pro Phe
                 20                  25                  30

His Tyr Asp Tyr Gln Thr Leu Arg Ile Gly Gly Leu Val Phe Ala
                 35                  40                  45
```

Val Val Leu Phe Ser Val Gly Ile Leu Leu Ile Leu Ser Arg Arg
50 55 60

Cys Lys Cys Ser Phe Asn Gln Lys Pro Arg Ala Pro Gly Asp Glu
65 70 75

Glu Ala Gln Val Glu Asn Leu Ile Thr Ala Asn Ala Thr Glu Pro
80 85 90

Gln Lys Gln Arg Thr Glu Val Gln Pro Ser Gly Gly Ser Leu Trp
95 100 105

Asn Leu Arg Arg Leu Leu Glu Pro Leu Asp Ala Asn Val Asp Ala
110 115 120

<210> 435
<211> 1297
<212> DNA
<213> Homo Sapien

<400> 435
ggtccttaat ggcagcagcc gccgctacca agatccttct gtgcctcccg 50

cttctgctcc tgctgtccgg ctggtcccgg gctgggcgag ccgaccctca 100

ctctctttgc tatgacatca ccgtcatccc taagttcaga cctggaccac 150

ggtggtgtgc ggttcaaggc caggtggatg aaaagacttt tcttcactat 200

gactgtggca acaagacagt cacacctgtc agtcccctgg ggaagaaact 250

aaatgtcaca acggcctgga aagcacagaa cccagtactg agagaggtgg 300

tggacatact tacagagcaa ctgcgtgaca ttcagctgga gaattacaca 350

cccaaggaac ccctcaccct gcaggcaagg atgtcttgtg agcagaaagc 400

tgaaggacac agcagtggat cttggcagtt cagtttcgat gggcagatct 450

tcctcctctt tgactcagag aagagaatgt ggacaacggt tcatcctgga 500

gccagaaaga tgaaagaaaa gtgggagaat gacaaggttg tggccatgtc 550

cttccattac ttctcaatgg gagactgtat aggatggctt gaggacttct 600

tgatgggcat ggacagcacc ctggagccaa gtgcaggagc accactcgcc 650

atgtcctcag gcacaaccca actcagggcc acagccacca ccctcatcct 700

ttgctgcctc ctcatcatcc tcccctgctt catcctccct ggcatctgag 750

gagagtcctt tagagtgaca ggttaaagct gataccaaaa ggctcctgtg 800

agcacggtct tgatcaaact cgcccttctg tctggccagc tgcccacgac 850

ctacggtgta tgtccagtgg cctccagcag atcatgatga catcatggac 900

ccaatagctc attcactgcc ttgattcctt ttgccaacaa ttttaccagc 950

agttatacct aacatattat gcaattttct cttggtgcta cctgatggaa 1000

ttcctgcact taaagttctg gctgactaaa caagatatat cattttcttt 1050

cttctctttt tgtttggaaa atcaagtact tctttgaatg atgatctctt 1100

tcttgcaaat gatattgtca gtaaaataat cacgttagac ttcagacctc 1150

tggggattct ttccgtgtcc tgaaagagaa tttttaaatt atttaataag 1200

aaaaaattta tattaatgat tgtttccttt agtaatttat tgttctgtac 1250

tgatatttaa ataaagagtt ctatttccca aaaaaaaaaa aaaaaaa 1297

<210> 436
<211> 246
<212> PRT
<213> Homo Sapien

<400> 436
Met Ala Ala Ala Ala Ala Thr Lys Ile Leu Leu Cys Leu Pro Leu
1 5 10 15

Leu Leu Leu Leu Ser Gly Trp Ser Arg Ala Gly Arg Ala Asp Pro
20 25 30

His Ser Leu Cys Tyr Asp Ile Thr Val Ile Pro Lys Phe Arg Pro
35 40 45

Gly Pro Arg Trp Cys Ala Val Gln Gly Gln Val Asp Glu Lys Thr
50 55 60

Phe Leu His Tyr Asp Cys Gly Asn Lys Thr Val Thr Pro Val Ser
65 70 75

Pro Leu Gly Lys Lys Leu Asn Val Thr Thr Ala Trp Lys Ala Gln
80 85 90

Asn Pro Val Leu Arg Glu Val Val Asp Ile Leu Thr Glu Gln Leu
95 100 105

Arg Asp Ile Gln Leu Glu Asn Tyr Thr Pro Lys Glu Pro Leu Thr
110 115 120

Leu Gln Ala Arg Met Ser Cys Glu Gln Lys Ala Glu Gly His Ser
125 130 135

Ser Gly Ser Trp Gln Phe Ser Phe Asp Gly Gln Ile Phe Leu Leu
140 145 150

Phe Asp Ser Glu Lys Arg Met Trp Thr Thr Val His Pro Gly Ala
155 160 165

Arg Lys Met Lys Glu Lys Trp Glu Asn Asp Lys Val Val Ala Met
170 175 180

Ser Phe His Tyr Phe Ser Met Gly Asp Cys Ile Gly Trp Leu Glu
185 190 195

Asp Phe Leu Met Gly Met Asp Ser Thr Leu Glu Pro Ser Ala Gly
200 205 210

Ala Pro Leu Ala Met Ser Ser Gly Thr Thr Gln Leu Arg Ala Thr
215 220 225

Ala Thr Thr Leu Ile Leu Cys Cys Leu Leu Ile Ile Leu Pro Cys
230 235 240

Phe Ile Leu Pro Gly Ile
245

<210> 437
<211> 2185
<212> DNA
<213> Homo Sapien

<400> 437
gttctccttt ccgagccaaa atcccaggcg atggtgaatt atgaacgtgc 50

cacaccatga agctcttgtg gcaggtaact gtgcaccacc acacctggaa 100

tgccatcctg ctcccgttcg tctacctcac ggcgcaagtg tggattctgt 150

gtgcagccat cgctgctgcc gcctcagccg ggccccagaa ctgcccctcc 200

gtttgctcgt gcagtaacca gttcagcaag gtggtgtgca cgcgccgggg 250

cctctccgag gtcccgcagg gtattccctc gaacacccgg tacctcaacc 300

tcatggagaa caacatccag atgatccagg ccgacacctt ccgccacctc 350

caccacctgg aggtcctgca gttgggcagg aactccatcc ggcagattga 400

ggtgggggcc ttcaacggcc tggccagcct caacaccctg gagctgttcg 450

acaactggct gacagtcatc cctagcgggg cctttgaata cctgtccaag 500

ctgcgggagc tctggcttcg caacaacccc atcgaaagca tcccctctta 550

cgccttcaac cgggtgccct ccctcatgcg cctggacttg ggggagctca 600

agaagctgga gtatatctct gagggagctt ttgaggggct gttcaacctc 650

aagtatctga acttgggcat gtgcaacatt aaagacatgc ccaatctcac 700

ccccctggtg gggctggagg agctggagat gtcagggaac cacttccctg 750

agatcaggcc tggctccttc catggcctga gctccctcaa gaagctctgg 800

gtcatgaact cacaggtcag cctgattgag cggaatgctt ttgacgggct 850

ggcttcactt gtggaactca acttggccca caataacctc tcttctttgc 900

cccatgacct ctttaccccg ctgaggtacc tggtggagtt gcatctacac 950

cacaaccctt ggaactgtga ttgtgacatt ctgtggctag cctggtggct 1000

tcgagagtat atacccacca attccacctg ctgtggccgc tgtcatgctc 1050

ccatgcacat gcgaggccgc tacctcgtgg aggtggacca ggcctccttc 1100

cagtgctctg ccccttcat catggacgca cctcgagacc tcaacatttc 1150

tgagggtcgg atggcagaac ttaagtgtcg gactccccct atgtcctccg 1200

tgaagtggtt gctgcccaat gggacagtgc tcagccacgc ctcccgccac 1250

ccaaggatct ctgtcctcaa cgacggcacc ttgaactttt cccacgtgct 1300

gctttcagac actggggtgt acacatgcat ggtgaccaat gttgcaggca 1350

actccaacgc ctcggcctac ctcaatgtga gcacggctga gcttaacacc 1400

tccaactaca gcttcttcac cacagtaaca gtggagacca cggagatctc 1450

gcctgaggac acaacgcgaa agtacaagcc tgttcctacc acgtccactg 1500

gttaccagcc ggcatatacc acctctacca cggtgctcat tcagactacc 1550

cgtgtgccca agcaggtggc agtacccgcg acagacacca ctgacaagat 1600

gcagaccagc ctggatgaag tcatgaagac caccaagatc atcattggct 1650

gctttgtggc agtgactctg ctagctgccg ccatgttgat tgtcttctat 1700

aaacttcgta agcggcacca gcagcggagt acagtcacag ccgcccggac 1750

tgttgagata atccaggtgg acgaagacat cccagcagca acatccgcag 1800

cagcaacagc agctccgtcc ggtgtatcag gtgagggggc agtagtgctg 1850

cccacaattc atgaccatat taactacaac acctacaaac cagcacatgg 1900

ggcccactgg acagaaaaca gcctggggaa ctctctgcac cccacagtca 1950

ccactatctc tgaaccttat ataattcaga cccataccaa ggacaaggta 2000

caggaaactc aaatatgact cccctccccc aaaaaactta taaaatgcaa 2050

tagaatgcac acaaagacag caacttttgt acagagtggg gagagacttt 2100

ttcttgtata tgcttatata ttaagtctat gggctggtta aaaaaaacag 2150

attatattaa aatttaaaga caaaaagtca aaaca 2185

<210> 438
<211> 653
<212> PRT
<213> Homo Sapien

<400> 438
Met Lys Leu Leu Trp Gln Val Thr Val His His His Thr Trp Asn
1 5 10 15

Ala Ile Leu Leu Pro Phe Val Tyr Leu Thr Ala Gln Val Trp Ile
20 25 30

Leu Cys Ala Ala Ile Ala Ala Ala Ala Ser Ala Gly Pro Gln Asn
35 40 45

Cys Pro Ser Val Cys Ser Cys Ser Asn Gln Phe Ser Lys Val Val
50 55 60

Cys Thr Arg Arg Gly Leu Ser Glu Val Pro Gln Gly Ile Pro Ser
65 70 75

Asn Thr Arg Tyr Leu Asn Leu Met Glu Asn Asn Ile Gln Met Ile
80 85 90

Gln Ala Asp Thr Phe Arg His Leu His His Leu Glu Val Leu Gln
95 100 105

Leu Gly Arg Asn Ser Ile Arg Gln Ile Glu Val Gly Ala Phe Asn
110 115 120

Gly Leu Ala Ser Leu Asn Thr Leu Glu Leu Phe Asp Asn Trp Leu

```
                125                 130                 135

Thr Val Ile Pro Ser Gly Ala Phe Glu Tyr Leu Ser Lys Leu Arg
                140                 145                 150

Glu Leu Trp Leu Arg Asn Asn Pro Ile Glu Ser Ile Pro Ser Tyr
                155                 160                 165

Ala Phe Asn Arg Val Pro Ser Leu Met Arg Leu Asp Leu Gly Glu
                170                 175                 180

Leu Lys Lys Leu Glu Tyr Ile Ser Glu Gly Ala Phe Glu Gly Leu
                185                 190                 195

Phe Asn Leu Lys Tyr Leu Asn Leu Gly Met Cys Asn Ile Lys Asp
                200                 205                 210

Met Pro Asn Leu Thr Pro Leu Val Gly Leu Glu Glu Leu Glu Met
                215                 220                 225

Ser Gly Asn His Phe Pro Glu Ile Arg Pro Gly Ser Phe His Gly
                230                 235                 240

Leu Ser Ser Leu Lys Lys Leu Trp Val Met Asn Ser Gln Val Ser
                245                 250                 255

Leu Ile Glu Arg Asn Ala Phe Asp Gly Leu Ala Ser Leu Val Glu
                260                 265                 270

Leu Asn Leu Ala His Asn Asn Leu Ser Ser Leu Pro His Asp Leu
                275                 280                 285

Phe Thr Pro Leu Arg Tyr Leu Val Glu Leu His Leu His His Asn
                290                 295                 300

Pro Trp Asn Cys Asp Cys Asp Ile Leu Trp Leu Ala Trp Trp Leu
                305                 310                 315

Arg Glu Tyr Ile Pro Thr Asn Ser Thr Cys Cys Gly Arg Cys His
                320                 325                 330

Ala Pro Met His Met Arg Gly Arg Tyr Leu Val Glu Val Asp Gln
                335                 340                 345

Ala Ser Phe Gln Cys Ser Ala Pro Phe Ile Met Asp Ala Pro Arg
                350                 355                 360

Asp Leu Asn Ile Ser Glu Gly Arg Met Ala Glu Leu Lys Cys Arg
                365                 370                 375

Thr Pro Pro Met Ser Ser Val Lys Trp Leu Leu Pro Asn Gly Thr
                380                 385                 390

Val Leu Ser His Ala Ser Arg His Pro Arg Ile Ser Val Leu Asn
                395                 400                 405

Asp Gly Thr Leu Asn Phe Ser His Val Leu Leu Ser Asp Thr Gly
                410                 415                 420

Val Tyr Thr Cys Met Val Thr Asn Val Ala Gly Asn Ser Asn Ala
                425                 430                 435

Ser Ala Tyr Leu Asn Val Ser Thr Ala Glu Leu Asn Thr Ser Asn
                440                 445                 450
```

Tyr Ser Phe Phe Thr Thr Val Thr Val Glu Thr Thr Glu Ile Ser
455 460 465

Pro Glu Asp Thr Thr Arg Lys Tyr Lys Pro Val Pro Thr Thr Ser
470 475 480

Thr Gly Tyr Gln Pro Ala Tyr Thr Thr Ser Thr Thr Val Leu Ile
485 490 495

Gln Thr Thr Arg Val Pro Lys Gln Val Ala Val Pro Ala Thr Asp
500 505 510

Thr Thr Asp Lys Met Gln Thr Ser Leu Asp Glu Val Met Lys Thr
515 520 525

Thr Lys Ile Ile Ile Gly Cys Phe Val Ala Val Thr Leu Leu Ala
530 535 540

Ala Ala Met Leu Ile Val Phe Tyr Lys Leu Arg Lys Arg His Gln
545 550 555

Gln Arg Ser Thr Val Thr Ala Ala Arg Thr Val Glu Ile Ile Gln
560 565 570

Val Asp Glu Asp Ile Pro Ala Ala Thr Ser Ala Ala Ala Thr Ala
575 580 585

Ala Pro Ser Gly Val Ser Gly Glu Gly Ala Val Val Leu Pro Thr
590 595 600

Ile His Asp His Ile Asn Tyr Asn Thr Tyr Lys Pro Ala His Gly
605 610 615

Ala His Trp Thr Glu Asn Ser Leu Gly Asn Ser Leu His Pro Thr
620 625 630

Val Thr Thr Ile Ser Glu Pro Tyr Ile Ile Gln Thr His Thr Lys
635 640 645

Asp Lys Val Gln Glu Thr Gln Ile
650

<210> 439
<211> 434
<212> DNA
<213> Homo Sapien

<400> 439
gtcgaatcca aatcactcat tgtgaaagct gagctcacag ccgaataagc 50

caccatgagg ctgtcagtgt gtctcctgat ggtctcgctg gccctttgct 100

gctaccaggc ccatgctctt gtctgcccag ctgttgcttc tgagatcaca 150

gtcttcttat tcttaagtga cgctgcggta aacctccaag ttgccaaact 200

taatccacct ccagaagctc ttgcagccaa gttggaagtg aagcactgca 250

ccgatcagat atcttttaag aaacgactct cattgaaaaa gtcctggtgg 300

aaatagtgaa aaaatgtggt gtgtgacatg taaaaatgct caacctggtt 350

tccaaagtct ttcaacgaca ccctgatctt cactaaaaat tgtaaaggtt 400

tcaacacgtt gctttaataa atcacttgcc ctgc 434

<210> 440
<211> 83
<212> PRT
<213> Homo Sapien

<400> 440
Met Arg Leu Ser Val Cys Leu Leu Met Val Ser Leu Ala Leu Cys
1 5 10 15

Cys Tyr Gln Ala His Ala Leu Val Cys Pro Ala Val Ala Ser Glu
20 25 30

Ile Thr Val Phe Leu Phe Leu Ser Asp Ala Ala Val Asn Leu Gln
35 40 45

Val Ala Lys Leu Asn Pro Pro Pro Glu Ala Leu Ala Ala Lys Leu
50 55 60

Glu Val Lys His Cys Thr Asp Gln Ile Ser Phe Lys Lys Arg Leu
65 70 75

Ser Leu Lys Lys Ser Trp Trp Lys
80

<210> 441
<211> 654
<212> DNA
<213> Homo Sapien

<400> 441
gaacattttt agttcccaag gaatgtacat cagccccacg gaagctaggc 50

cacctctggg atggggttgc tggtttaaaa caaacgccag tcatcctata 100

taaggacctg acagccacca ggcaccacct ccgccaggaa ctgcaggccc 150

acctgtctgc aacccagctg aggccatgcc ctccccaggg accgtctgca 200

gcctcctgct cctcggcatg ctctggctgg acttggccat ggcaggctcc 250

agcttcctga gccctgaaca ccagagagtc cagcagagaa aggagtcgaa 300

gaagccacca gccaagctgc agccccgagc tctagcaggc tggctccgcc 350

cggaagatgg aggtcaagca gaaggggcag aggatgaact ggaagtccgg 400

ttcaacgccc cctttgatgt tggaatcaag ctgtcagggg ttcagtacca 450

gcagcacagc caggccctgg ggaagtttct tcaggacatc ctctgggaag 500

aggccaaaga ggccccagcc gacaagtgat cgcccacaag ccttactcac 550

ctctctctaa gtttagaagc gctcatctgg cttttcgctt gcttctgcag 600

caactcccac gactgttgta caagctcagg aggcgaataa atgttcaaac 650

tgta 654

<210> 442
<211> 117
<212> PRT

<213> Homo Sapien

<400> 442

```
Met Pro Ser Pro Gly Thr Val Cys Ser Leu Leu Leu Leu Gly Met
  1               5                  10                  15

Leu Trp Leu Asp Leu Ala Met Ala Gly Ser Ser Phe Leu Ser Pro
                 20                  25                  30

Glu His Gln Arg Val Gln Gln Arg Lys Glu Ser Lys Lys Pro Pro
                 35                  40                  45

Ala Lys Leu Gln Pro Arg Ala Leu Ala Gly Trp Leu Arg Pro Glu
                 50                  55                  60

Asp Gly Gly Gln Ala Glu Gly Ala Glu Asp Glu Leu Glu Val Arg
                 65                  70                  75

Phe Asn Ala Pro Phe Asp Val Gly Ile Lys Leu Ser Gly Val Gln
                 80                  85                  90

Tyr Gln Gln His Ser Gln Ala Leu Gly Lys Phe Leu Gln Asp Ile
                 95                 100                 105

Leu Trp Glu Glu Ala Lys Glu Ala Pro Ala Asp Lys
                110                 115
```

<210> 443
<211> 1332
<212> DNA
<213> Homo Sapien

<400> 443

```
cggccacagc tggcatgctc tgcctgatcg ccatcctgct gtatgtcctc 50
gtccagtacc tcgtgaaccc cggggtgctc cgcacggacc ccagatgtca 100
agaatatgaa cacgtggctg ctgttcctcc ccctgttccc ggtgcaggtg 150
cagaccctga tagtcgtgat catcgggatg ctcgtgctcc tgctggactt 200
tcttggcttg gtgcacctgg gccagctgct catcttccac atctacctga 250
gtatgtcccc caccctaagc ccccgatccc cccaaggctg ggtggtcaga 300
gctgctcatc ttacacctct acttgagtat gtccctaacc ctgagccccc 350
cacgcctggg gccagagtct ttgtcccccg tgtgcgcatg tgttcagggt 400
cagcctctcc cagaagtgag atcatggaca aaaagggcaa atcacaggaa 450
gaaattaaat ccatgaggac ccagcaggcc cagcaagaag ctgaactcac 500
gccgagacct gcaggagtgg tgccaggtgc ttgaagtaac aagtttaaaa 550
tgttcagaga caatggaatg gaatctatta ggcaagaaca ggacattatg 600
aaataaggac aggtggactt ccaaaaacac aagtagaaat tctaacaatg 650
aaatatatta caggcaggtc acccactaac caaacaactg aagcgagagc 700
tgtggtcttg cttggtctca cagtgggcac agcggtaggc ggtcagtcat 750
gttgctgaac gacggagggt aaactcccca gccccaagaa aacctgtgtt 800
```

ggaagtaaca acaacctccc tgctcctggc accagccgtt ttggtcatgg 850

tgggccagct gcaaagcgtc ttccattctc tgggcagtgg tggccccgag 900

gctgtggcct ctcagggggt ttctgtggac acgggcagca gagtgtgtcc 950

aggccagccc ccaagaatgc cctgctcctg acagcttggc caacccctgg 1000

tcagggcaga gggagttggg tgggtcaggc tctgggctca cctccatctc 1050

cagagcatcc cctgcctgca gttgtggcaa gaacgcccag ctcagaatga 1100

acacacccca ccaagagcct ccttgttcat aaccacaggt taccctacaa 1150

accactgtcc ccacacaacc ctggggatgt tttaaaacac acacctctaa 1200

cgcatatctt acagtcactg ttgtcttgcc tgagggttga atttttttta 1250

atgaaagtgc aatgaaaatc actggattaa atcctacgga cacagagctg 1300

aaaaaaaaaa aaaaaaaaaa aaaaaaaaaa aa 1332

<210> 444
<211> 142
<212> PRT
<213> Homo Sapien

<400> 444
```
Met Asn Thr Trp Leu Leu Phe Leu Pro Leu Phe Pro Val Gln Val
1               5                   10                  15

Gln Thr Leu Ile Val Val Ile Ile Gly Met Leu Val Leu Leu Leu
                20                  25                  30

Asp Phe Leu Gly Leu Val His Leu Gly Gln Leu Leu Ile Phe His
                35                  40                  45

Ile Tyr Leu Ser Met Ser Pro Thr Leu Ser Pro Arg Ser Pro Gln
                50                  55                  60

Gly Trp Val Val Arg Ala Ala His Leu Thr Pro Leu Leu Glu Tyr
                65                  70                  75

Val Pro Asn Pro Glu Pro Pro Thr Pro Gly Ala Arg Val Phe Val
                80                  85                  90

Pro Arg Val Arg Met Cys Ser Gly Ser Ala Ser Pro Arg Ser Glu
                95                  100                 105

Ile Met Asp Lys Lys Gly Lys Ser Gln Glu Glu Ile Lys Ser Met
                110                 115                 120

Arg Thr Gln Gln Ala Gln Gln Glu Ala Glu Leu Thr Pro Arg Pro
                125                 130                 135

Ala Gly Val Val Pro Gly Ala
                140
```

<210> 445
<211> 687
<212> DNA
<213> Homo Sapien

-

<400> 445
aggcgggcag cagctgcagg ctgaccttgc agcttggcgg aatggactgg 50

cctcacaacc tgctgtttct tcttaccatt tccatcttcc tggggctggg 100

ccagcccagg agccccaaaa gcaagaggaa ggggcaaggg cggcctgggc 150

ccctggcccc tggccctcac caggtgccac tggacctggt gtcacggatg 200

aaaccgtatg cccgcatgga ggagtatgag aggaacatcg aggagatggt 250

ggcccagctg aggaacagct cagagctggc ccagagaaag tgtgaggtca 300

acttgcagct gtggatgtcc aacaagagga gcctgtctcc ctggggctac 350

agcatcaacc acgaccccag ccgtatcccc gtggacctgc cggaggcacg 400

gtgcctgtgt ctgggctgtg tgaacccctt caccatgcag gaggaccgca 450

gcatggtgag cgtgccggtg ttcagccagg ttcctgtgcg ccgccgcctc 500

tgcccgccac cgccccgcac agggccttgc cgccagcgcg cagtcatgga 550

gaccatcgct gtgggctgca cctgcatctt ctgaatcacc tggcccagaa 600

gccaggccag cagcccgaga ccatcctcct tgcacctttg tgccaagaaa 650

ggcctatgaa aagtaaacac tgacttttga aagcaag 687

<210> 446
<211> 180
<212> PRT
<213> Homo Sapien

<400> 446
Met Asp Trp Pro His Asn Leu Leu Phe Leu Leu Thr Ile Ser Ile
1               5                   10                  15

Phe Leu Gly Leu Gly Gln Pro Arg Ser Pro Lys Ser Lys Arg Lys
                20                  25                  30

Gly Gln Gly Arg Pro Gly Pro Leu Ala Pro Gly Pro His Gln Val
                35                  40                  45

Pro Leu Asp Leu Val Ser Arg Met Lys Pro Tyr Ala Arg Met Glu
                50                  55                  60

Glu Tyr Glu Arg Asn Ile Glu Glu Met Val Ala Gln Leu Arg Asn
                65                  70                  75

Ser Ser Glu Leu Ala Gln Arg Lys Cys Glu Val Asn Leu Gln Leu
                80                  85                  90

Trp Met Ser Asn Lys Arg Ser Leu Ser Pro Trp Gly Tyr Ser Ile
                95                  100                 105

Asn His Asp Pro Ser Arg Ile Pro Val Asp Leu Pro Glu Ala Arg
                110                 115                 120

Cys Leu Cys Leu Gly Cys Val Asn Pro Phe Thr Met Gln Glu Asp
                125                 130                 135

Arg Ser Met Val Ser Val Pro Val Phe Ser Gln Val Pro Val Arg
                140                 145                 150

Arg Arg Leu Cys Pro Pro Pro Pro Arg Thr Gly Pro Cys Arg Gln
155 160 165

Arg Ala Val Met Glu Thr Ile Ala Val Gly Cys Thr Cys Ile Phe
170 175 180

<210> 447
<211> 1484
<212> DNA
<213> Homo Sapien

<400> 447
ggagtgcaga tggcatcctt cggttcttcc agacaagctg caagacgctg 50

accatggcca agatggagct ctcgaaggcc ttctctggcc agcggacact 100

cctatctgcc atcctcagca tgctatcact cagcttctcc acaacatccc 150

tgctcagcaa ctactggttt gtgggcacac agaaggtgcc caagcccctg 200

tgcgagaaag gtctggcagc caagtgcttt gacatgccag tgtccctgga 250

tggagatacc aacacatcca cccaggaggt ggtacaatac aactgggaga 300

ctggggatga ccggttctcc ttccggagct tccggagtgg catgtggcta 350

tcctgtgagg aaactgtgga agaaccaggg gagaggtgcc gaagtttcat 400

tgaacttaca ccaccagcca agagaggtga gaaaggacta ctggaatttg 450

ccacgttgca aggcccatgt caccccactc tccgatttgg agggaagcgg 500

ttgatggaga aggcttccct cccctcccct cccttggggc tttgtggcaa 550

aaatcctatg gttatccctg ggaacgcaga tcacctacat cggacttcaa 600

ttcatcagct tcctcctgct actaacagac ttgctactca ctgggaaccc 650

tgcctgtggg ctcaaactga gcgcctttgc tgctgtttcc tctgtcctgt 700

caggtctcct ggggatggtg gcccacatga tgtattcaca agtcttccaa 750

gcgactgtca acttgggtcc agaagactgg agaccacatg tttggaatta 800

tggctgggcc ttctacatgg cctggctctc cttcacctgc tgcatggcgt 850

cggctgtcac caccttcaac acgtacacca ggatggtgct ggagttcaag 900

tgcaagcata gtaagagctt caaggaaaac ccgaactgcc taccacatca 950

ccatcagtgt ttccctcggc ggctgtcaag tgcagccccc accgtgggtc 1000

ctttgaccag ctaccaccag tatcataatc agcccatcca ctctgtctct 1050

gagggagtcg acttctactc cgagctgcgg aacaagggat ttcaaagagg 1100

ggccagccag gagctgaaag aagcagttag gtcatctgta gaggaagagc 1150

agtgttagga gttaagcggg tttggggagt aggcttgagc cctaccttac 1200

acgtctgctg attatcaaca tgtgcttaag ccaacatccg tctcttgagc 1250

atggttttta gaggctacga ataaggctat gaataagggt tatctttaag 1300

tcctaaggga ttcctgggtg ccactgctct cttttcctct acagctccat 1350

cttgtttcac ccaccccaca tctcacacat ccagaattcc cttctttact 1400

gatagtttct gtgccaggtt ctgggctaaa ccatggagat aaaaagaaga 1450

gtaaaataca cttcccgacc ttaaggatct gaaa 1484

<210> 448
<211> 285
<212> PRT
<213> Homo Sapien

<400> 448
Met Ala Lys Met Glu Leu Ser Lys Ala Phe Ser Gly Gln Arg Thr
1 5 10 15

Leu Leu Ser Ala Ile Leu Ser Met Leu Ser Leu Ser Phe Ser Thr
20 25 30

Thr Ser Leu Leu Ser Asn Tyr Trp Phe Val Gly Thr Gln Lys Val
35 40 45

Pro Lys Pro Leu Cys Glu Lys Gly Leu Ala Ala Lys Cys Phe Asp
50 55 60

Met Pro Val Ser Leu Asp Gly Asp Thr Asn Thr Ser Thr Gln Glu
65 70 75

Val Val Gln Tyr Asn Trp Glu Thr Gly Asp Asp Arg Phe Ser Phe
80 85 90

Arg Ser Phe Arg Ser Gly Met Trp Leu Ser Cys Glu Glu Thr Val
95 100 105

Glu Glu Pro Gly Glu Arg Cys Arg Ser Phe Ile Glu Leu Thr Pro
110 115 120

Pro Ala Lys Arg Gly Glu Lys Gly Leu Leu Glu Phe Ala Thr Leu
125 130 135

Gln Gly Pro Cys His Pro Thr Leu Arg Phe Gly Gly Lys Arg Leu
140 145 150

Met Glu Lys Ala Ser Leu Pro Ser Pro Pro Leu Gly Leu Cys Gly
155 160 165

Lys Asn Pro Met Val Ile Pro Gly Asn Ala Asp His Leu His Arg
170 175 180

Thr Ser Ile His Gln Leu Pro Pro Ala Thr Asn Arg Leu Ala Thr
185 190 195

His Trp Glu Pro Cys Leu Trp Ala Gln Thr Glu Arg Leu Cys Cys
200 205 210

Cys Phe Leu Cys Pro Val Arg Ser Pro Gly Asp Gly Gly Pro His
215 220 225

Asp Val Phe Thr Ser Leu Pro Ser Asp Cys Gln Leu Gly Ser Arg
230 235 240

Arg Leu Glu Thr Thr Cys Leu Glu Leu Trp Leu Gly Leu Leu His

245 250 255

Gly Leu Ala Leu Leu His Leu Leu His Gly Val Gly Cys His His
260 265 270

Leu Gln His Val His Gln Asp Gly Ala Gly Val Gln Val Gln Ala
275 280 285

<210> 449
<211> 4104
<212> DNA
<213> Homo Sapien

<400> 449
cccacgcgtc cgcccacgcg tccgcccacg cgtccgccca cgcgtccgcc 50

cacgcgtccg cccacgcgtc cgcccacgcg tccggtgcaa gctcgcgccg 100

cacactgcct ggtggaggga aggagcccgg gcgcctctcg ccgctccccg 150

cgccgccgtc cgcacctccc caccgcccgc cgcccgccgc ccgccgcccg 200

caaagcatga gtgagcccgc tctctgcagc tgcccggggc gcgaatggca 250

ggctgtttcc gcggagtaaa aggtggcgcc ggtcagtggt cgtttccaat 300

gacggacatt aaccagactg tcagatcctg ggagtcgcg agcccgagt 350

ttggagtttt ttccccccac aacgtcacag tccgaactgc agagggaaag 400

gaaggcggca ggaaggcgaa gctcgggctc cggcacgtag ttgggaaact 450

tgcgggtcct agaagtcgcc tccccgcctt gccggccgcc cttgcagccc 500

cgagccgagc agcaaagtga gacattgtgc gcctgccaga tccgccggcc 550

gcggaccggg gctgcctcgg aaacacagag gggtcttctc tcgccctgca 600

tataattagc ctgcacacaa agggagcagc tgaatggagg ttgtcactct 650

ctggaaaagg atttctgacc gagcgcttcc aatggacatt ctccagtctc 700

tctggaaaga ttctcgctaa tggatttcct gctgctcggt ctctgtctat 750

actggctgct gaggaggccc tcgggggtgg tcttgtgtct gctgggggcc 800

tgctttcaga tgctgcccgc cgcccccagc gggtgcccgc agctgtgccg 850

gtgcgagggg cggctgctgt actgcgaggc gctcaacctc accgaggcgc 900

cccacaacct gtccggcctg ctgggcttgt ccctgcgcta caacagcctc 950

tcggagctgc gcgccggcca gttcacgggg ttaatgcagc tcacgtggct 1000

ctatctggat cacaatcaca tctgctccgt gcagggggac gcctttcaga 1050

aactgcgccg agttaaggaa ctcacgctga gttccaacca gatcacccaa 1100

ctgcccaaca ccaccttccg gcccatgccc aacctgcgca gcgtggacct 1150

ctcgtacaac aagctgcagg cgctcgcgcc cgacctcttc cacgggctgc 1200

ggaagctcac cacgctgcat atgcgggcca acgccatcca gtttgtgccc 1250

gtgcgcatct tccaggactg ccgcagcctc aagtttctcg acatcggata 1300
caatcagctc aagagtctgg cgcgcaactc tttcgccggc ttgtttaagc 1350
tcaccgagct gcacctcgag cacaacgact tggtcaaggt gaacttcgcc 1400
cacttcccgc gcctcatctc cctgcactcg ctctgcctgc ggaggaacaa 1450
ggtggccatt gtggtcagct cgctggactg ggtttggaac ctggagaaaa 1500
tggacttgtc gggcaacgag atcgagtaca tggagcccca tgtgttcgag 1550
accgtgccgc acctgcagtc cctgcagctg gactccaacc gcctcaccta 1600
catcgagccc cggatcctca actcttggaa gtccctgaca agcatcaccc 1650
tggccgggaa cctgtgggat tgcgggcgca acgtgtgtgc cctagcctcg 1700
tggctcagca acttccaggg gcgctacgat ggcaacttgc agtgcgccag 1750
cccggagtac gcacagggcg aggacgtcct ggacgccgtg tacgccttcc 1800
acctgtgcga ggatggggcc gagcccacca gcggccacct gctctcggcc 1850
gtcaccaacc gcagtgatct ggggcccccct gccagctcgg ccaccacgct 1900
cgcggacggc ggggaggggc agcacgacgg cacattcgag cctgccaccg 1950
tggctcttcc aggcggcgag cacgccgaga acgccgtgca gatccacaag 2000
gtggtcacgg gcaccatggc cctcatcttc tccttcctca tcgtggtcct 2050
ggtgctctac gtgtcctgga agtgtttccc agccagcctc aggcagctca 2100
gacagtgctt tgtcacgcag cgcaggaagc aaaagcagaa acagaccatg 2150
catcagatgg ctgccatgtc tgcccaggaa tactacgttg attacaaacc 2200
gaaccacatt gagggagccc tggtgatcat caacgagtat ggctcgtgta 2250
cctgccacca gcagcccgcg agggaatgcg aggtgtgatt gtcccagtgg 2300
ctctcaaccc atgcgctacc aaatacgcct gggcagccgg gacgggccgg 2350
cgggcaccag gctggggtct ccttgtctgt gctctgatat gctccttgac 2400
tgaaacttta aggggatctc tcccagagac ttgacatttt agctttattg 2450
tgtcttaaaa acaaaagcga attaaaacac aacaaaaaac cccaccccac 2500
aaccttcagg acagtctatc ttaaatttca tatgagaact ccttcctccc 2550
tttgaagatc tgtccatatt caggaatctg agagtgtaaa aaaggtggcc 2600
ataagacaga gagagaataa tcgtgctttg ttttatgcta ctcctcccac 2650
cctgcccatg attaaacatc atgtatgtag aagatcttaa gtccatacgc 2700
atttcatgaa gaaccattgg aaagaggaat ctgcaatctg ggagcttaag 2750
agcaaatgat gaccatagaa agctatgttc ttactttgtg tgtgtgtctg 2800
tatgtttctg cgttgtgtgt ctttgtaggc aagcaaacgt tgtctacaca 2850

aacgggaatt tagctcacat catttcatgc ccctgtgcct ctagctctgg 2900

agattggtgg ggggaggtgg ggggaaacgg caggaataag ggaaagtggt 2950

agttttaact aaggttttgt aacacttgaa atcttttctt tctcaaatta 3000

attatcttta agcttcaaga aacttgctct gacccctcta agcaaactac 3050

taagcattta aaagagaatc taatttttaa aggtgtagca cctttttttt 3100

tattcttccc acagagggtg ctaatctcat tatgctgtgc tatctgaaaa 3150

gaacttaagg ccacaattca cgtctcgtcc tgggcattgt gatggattga 3200

ccctccattt gcagtacctt cccagctgat taaagttcag cagtggtatt 3250

gaggtttttc gaatatttat atagaaaaaa agtcttttca catgacaaat 3300

gacactctca caccagtctt agccctagta gtttttttagg ttggaccaga 3350

ggaagcaggt taaatgagac ctgtcctctg ctgcactcag aaaaaatagg 3400

cagtccctga tgctcagatc ttagccttga tattaatagt tgagaccacc 3450

tacccacaat gcagcctata ctcccaagac tacaaagtta ccatcgcaaa 3500

ggaaaggtta ttccagtaaa aggaaatagt tttctcaacc atttaaaaat 3550

attcttctga actcatcaaa gtagaagagc ccccaacctt ttctctctgc 3600

cttcaagaag gcagacattt ggtatgattt agcatcaaca acacatttat 3650

gagtatatgt aagtaatcag aggggcaaat gccacttgtt attcctccca 3700

agttttccaa gcaagtacac acagatctct ggtaggatta ggggccactt 3750

gtgtttccgg cttattttag tcgacttgtc agcaagtttg atgcctagtc 3800

tatctgacat ggcccagtag aacagggcat tgatggatca catgagatgg 3850

tagaaggaac atcatcacat acccctctca cagagaaaat tatcaaagaa 3900

ccagaaatta tatctgtttt ggagcaagag tgtcataatg tttcagggta 3950

gtcaaaataa acataaatta tctcctctag atgagtggcg atgttggctg 4000

atttgggtct gccattgaca gaatgtcaaa taaaaaggaa ttagctagaa 4050

tatgaccatt aaatgtgctt ctgaaatata ttttgagata ggtttagaat 4100

gtca 4104

<210> 450
<211> 522
<212> PRT
<213> Homo Sapien

<400> 450
Met Asp Phe Leu Leu Leu Gly Leu Cys Leu Tyr Trp Leu Leu Arg
1 5 10 15

Arg Pro Ser Gly Val Val Leu Cys Leu Leu Gly Ala Cys Phe Gln
20 25 30

Met Leu Pro Ala Ala Pro Ser Gly Cys Pro Gln Leu Cys Arg Cys
35 40 45

Glu Gly Arg Leu Leu Tyr Cys Glu Ala Leu Asn Leu Thr Glu Ala
50 55 60

Pro His Asn Leu Ser Gly Leu Leu Gly Leu Ser Leu Arg Tyr Asn
65 70 75

Ser Leu Ser Glu Leu Arg Ala Gly Gln Phe Thr Gly Leu Met Gln
80 85 90

Leu Thr Trp Leu Tyr Leu Asp His Asn His Ile Cys Ser Val Gln
95 100 105

Gly Asp Ala Phe Gln Lys Leu Arg Arg Val Lys Glu Leu Thr Leu
110 115 120

Ser Ser Asn Gln Ile Thr Gln Leu Pro Asn Thr Thr Phe Arg Pro
125 130 135

Met Pro Asn Leu Arg Ser Val Asp Leu Ser Tyr Asn Lys Leu Gln
140 145 150

Ala Leu Ala Pro Asp Leu Phe His Gly Leu Arg Lys Leu Thr Thr
155 160 165

Leu His Met Arg Ala Asn Ala Ile Gln Phe Val Pro Val Arg Ile
170 175 180

Phe Gln Asp Cys Arg Ser Leu Lys Phe Leu Asp Ile Gly Tyr Asn
185 190 195

Gln Leu Lys Ser Leu Ala Arg Asn Ser Phe Ala Gly Leu Phe Lys
200 205 210

Leu Thr Glu Leu His Leu Glu His Asn Asp Leu Val Lys Val Asn
215 220 225

Phe Ala His Phe Pro Arg Leu Ile Ser Leu His Ser Leu Cys Leu
230 235 240

Arg Arg Asn Lys Val Ala Ile Val Val Ser Ser Leu Asp Trp Val
245 250 255

Trp Asn Leu Glu Lys Met Asp Leu Ser Gly Asn Glu Ile Glu Tyr
260 265 270

Met Glu Pro His Val Phe Glu Thr Val Pro His Leu Gln Ser Leu
275 280 285

Gln Leu Asp Ser Asn Arg Leu Thr Tyr Ile Glu Pro Arg Ile Leu
290 295 300

Asn Ser Trp Lys Ser Leu Thr Ser Ile Thr Leu Ala Gly Asn Leu
305 310 315

Trp Asp Cys Gly Arg Asn Val Cys Ala Leu Ala Ser Trp Leu Ser
320 325 330

Asn Phe Gln Gly Arg Tyr Asp Gly Asn Leu Gln Cys Ala Ser Pro
335 340 345

Glu Tyr Ala Gln Gly Glu Asp Val Leu Asp Ala Val Tyr Ala Phe

```
                350                 355                 360
His Leu Cys Glu Asp Gly Ala Glu Pro Thr Ser Gly His Leu Leu
                365                 370                 375
Ser Ala Val Thr Asn Arg Ser Asp Leu Gly Pro Pro Ala Ser Ser
                380                 385                 390
Ala Thr Thr Leu Ala Asp Gly Gly Glu Gly Gln His Asp Gly Thr
                395                 400                 405
Phe Glu Pro Ala Thr Val Ala Leu Pro Gly Gly Glu His Ala Glu
                410                 415                 420
Asn Ala Val Gln Ile His Lys Val Val Thr Gly Thr Met Ala Leu
                425                 430                 435
Ile Phe Ser Phe Leu Ile Val Val Leu Val Leu Tyr Val Ser Trp
                440                 445                 450
Lys Cys Phe Pro Ala Ser Leu Arg Gln Leu Arg Gln Cys Phe Val
                455                 460                 465
Thr Gln Arg Arg Lys Gln Lys Gln Lys Gln Thr Met His Gln Met
                470                 475                 480
Ala Ala Met Ser Ala Gln Glu Tyr Tyr Val Asp Tyr Lys Pro Asn
                485                 490                 495
His Ile Glu Gly Ala Leu Val Ile Ile Asn Glu Tyr Gly Ser Cys
                500                 505                 510
Thr Cys His Gln Gln Pro Ala Arg Glu Cys Glu Val
                515                 520
```

<210> 451
<211> 2623
<212> DNA
<213> Homo Sapien

<400> 451

```
ttgagcgcag gtgagctcct gcgcgttccg ggggcgttcc tccagtcacc 50
ctcccgccgt tacccgcggc gcgcccgagg gagtctcctc cagaccctcc 100
ctcccgttgc tccaaactaa tacggactga acggatcgct gcgagggtgg 150
gagagaaaat tagggggaga aaggacagag agagcaacta ccatccatag 200
ccagatagat tatcttacac tgaactgatc aagtactttg aaaatgactt 250
cgaaatttat cttggtgtcc ttcatacttg ctgcactgag tctttcaacc 300
acctttttctc tccaactaga ccagcaaaag gttctactag tttcttttga 350
tggattccgt tgggattact tatataaagt tccaacgccc cattttcatt 400
atattatgaa atatggtgtt cacgtgaagc aagttactaa tgtttttatt 450
acaaaaacct accctaacca ttatactttg gtaactggcc tctttgcaga 500
gaatcatggg attgttgcaa atgatatgtt tgatcctatt cggaacaaat 550
ctttctcctt ggatcacatg aatatttatg attccaagtt ttgggaagaa 600
```

gcgacaccaa tatggatcac aaaccagagg gcaggacata ctagtggtgc 650
agccatgtgg cccggaacag atgtaaaaat acataagcgc tttcctactc 700
attacatgcc ttacaatgag tcagtttcat ttgaagatag agttgccaaa 750
attgttgaat ggtttacgtc aaaagagccc ataaatcttg gtcttctcta 800
ttgggaagac cctgatgaca tgggccacca tttgggacct gacagtccgc 850
tcatggggcc tgtcatttca gatattgaca agaagttagg atatctcata 900
caaatgctga aaaaggcaaa gttgtggaac actctgaacc taatcatcac 950
aagtgatcat ggaatgacgc agtgctctga ggaaaggtta atagaacttg 1000
accagtacct ggataaagac cactataccc tgattgatca atctccagta 1050
gcagccatct tgccaaaaga aggtaaattt gatgaagtct atgaagcact 1100
aactcacgct catcctaatc ttactgttta caaaaaagaa gacgttccag 1150
aaaggtggca ttacaaatac aacagtcgaa ttcaaccaat catagcagtg 1200
gctgatgaag ggtggcacat tttacagaat aagtcagatg actttctgtt 1250
aggcaaccac ggttacgata atgcgttagc agatatgcat ccaatatttt 1300
tagcccatgg tcctgccttc agaaagaatt tctcaaaaga agccatgaac 1350
tccacagatt tgtacccact actatgccac ctcctcaata tcactgccat 1400
gccacacaat ggatcattct ggaatgtcca ggatctgctc aattcagcaa 1450
tgccaagggt ggtcccttat acacagagta ctatactcct ccctggtagt 1500
gttaaaccag cagaatatga ccaagagggg tcataccctt atttcatagg 1550
ggtctctctt ggcagcatta tagtgattgt atttttgta attttcatta 1600
agcatttaat tcacagtcaa atacctgcct tacaagatat gcatgctgaa 1650
atagctcaac cattattaca agcctaatgt tactttgaag tggatttgca 1700
tattgaagtg gagattccat aattatgtca gtgtttaaag gtttcaaatt 1750
ctgggaaacc agttccaaac atctgcagaa accattaagc agttacatat 1800
ttaggtatac acacacacac acacacacac atacacacac acggaccaaa 1850
atacttacac ctgcaaagga ataaagatgt gagagtatgt ctccattgtt 1900
cactgtagca tagggataga taagatcctg ctttatttgg acttggcgca 1950
gataatgtat atatttagca actttgcact atgtaaagta ccttatatat 2000
tgcactttaa atttctctcc tgatgggtac tttaatttga aatgcacttt 2050
atggacagtt atgtcttata acttgattga aaatgacaac tttttgcacc 2100
catgtcacag aatacttgtt acgcattgtt caaactgaag gaaatttcta 2150
ataatcccga ataatgaaca tagaaatcta tctccataaa ttgagagaag 2200

aagaaggtga taagtgttga aaattaaatg tgataacctt tgaaccttga 2250

attttggaga tgtattccca acagcagaat gcaactgtgg gcatttcttg 2300

tcttatttct ttccagagaa cgtggttttc atttattttt ccctcaaaag 2350

agagtcaaat actgacagat tcgttctaaa tatattgttt ctgtcataaa 2400

attattgtga tttcctgatg agtcatatta ctgtgatttt cataataatg 2450

aagacaccat gaatatactt ttcttctata tagttcagca atggcctgaa 2500

tagaagcaac caggcaccat ctcagcaatg ttttctcttg tttgtaatta 2550

tttgctcctt tgaaaattaa atcactatta attacattaa aaatcaaatt 2600

ggataaaaaa aaaaaaaaaa aaa 2623

<210> 452
<211> 477
<212> PRT
<213> Homo Sapien

<400> 452
Met Thr Ser Lys Phe Ile Leu Val Ser Phe Ile Leu Ala Ala Leu
1 5 10 15

Ser Leu Ser Thr Thr Phe Ser Leu Gln Leu Asp Gln Gln Lys Val
20 25 30

Leu Leu Val Ser Phe Asp Gly Phe Arg Trp Asp Tyr Leu Tyr Lys
35 40 45

Val Pro Thr Pro His Phe His Tyr Ile Met Lys Tyr Gly Val His
50 55 60

Val Lys Gln Val Thr Asn Val Phe Ile Thr Lys Thr Tyr Pro Asn
65 70 75

His Tyr Thr Leu Val Thr Gly Leu Phe Ala Glu Asn His Gly Ile
80 85 90

Val Ala Asn Asp Met Phe Asp Pro Ile Arg Asn Lys Ser Phe Ser
95 100 105

Leu Asp His Met Asn Ile Tyr Asp Ser Lys Phe Trp Glu Glu Ala
110 115 120

Thr Pro Ile Trp Ile Thr Asn Gln Arg Ala Gly His Thr Ser Gly
125 130 135

Ala Ala Met Trp Pro Gly Thr Asp Val Lys Ile His Lys Arg Phe
140 145 150

Pro Thr His Tyr Met Pro Tyr Asn Glu Ser Val Ser Phe Glu Asp
155 160 165

Arg Val Ala Lys Ile Val Glu Trp Phe Thr Ser Lys Glu Pro Ile
170 175 180

Asn Leu Gly Leu Leu Tyr Trp Glu Asp Pro Asp Asp Met Gly His
185 190 195

His Leu Gly Pro Asp Ser Pro Leu Met Gly Pro Val Ile Ser Asp
200 205 210

Ile Asp Lys Lys Leu Gly Tyr Leu Ile Gln Met Leu Lys Lys Ala
215 220 225

Lys Leu Trp Asn Thr Leu Asn Leu Ile Ile Thr Ser Asp His Gly
230 235 240

Met Thr Gln Cys Ser Glu Glu Arg Leu Ile Glu Leu Asp Gln Tyr
245 250 255

Leu Asp Lys Asp His Tyr Thr Leu Ile Asp Gln Ser Pro Val Ala
260 265 270

Ala Ile Leu Pro Lys Glu Gly Lys Phe Asp Glu Val Tyr Glu Ala
275 280 285

Leu Thr His Ala His Pro Asn Leu Thr Val Tyr Lys Lys Glu Asp
290 295 300

Val Pro Glu Arg Trp His Tyr Lys Tyr Asn Ser Arg Ile Gln Pro
305 310 315

Ile Ile Ala Val Ala Asp Glu Gly Trp His Ile Leu Gln Asn Lys
320 325 330

Ser Asp Asp Phe Leu Leu Gly Asn His Gly Tyr Asp Asn Ala Leu
335 340 345

Ala Asp Met His Pro Ile Phe Leu Ala His Gly Pro Ala Phe Arg
350 355 360

Lys Asn Phe Ser Lys Glu Ala Met Asn Ser Thr Asp Leu Tyr Pro
365 370 375

Leu Leu Cys His Leu Leu Asn Ile Thr Ala Met Pro His Asn Gly
380 385 390

Ser Phe Trp Asn Val Gln Asp Leu Leu Asn Ser Ala Met Pro Arg
395 400 405

Val Val Pro Tyr Thr Gln Ser Thr Ile Leu Leu Pro Gly Ser Val
410 415 420

Lys Pro Ala Glu Tyr Asp Gln Glu Gly Ser Tyr Pro Tyr Phe Ile
425 430 435

Gly Val Ser Leu Gly Ser Ile Ile Val Ile Val Phe Phe Val Ile
440 445 450

Phe Ile Lys His Leu Ile His Ser Gln Ile Pro Ala Leu Gln Asp
455 460 465

Met His Ala Glu Ile Ala Gln Pro Leu Leu Gln Ala
470 475

<210> 453
<211> 1674
<212> DNA
<213> Homo Sapien

<400> 453
ggccgcctgg aattgtggga gttgtgtctg ccactcggct gccggaggcc 50

gaaggtccgt gactatggct ccccagagcc tgccttcatc taggatggct 100
cctctgggca tgctgcttgg gctgctgatg gccgcctgct tcaccttctg 150
cctcagtcat cagaacctga aggagtttgc cctgaccaac ccagagaaga 200
gcagcaccaa agaaacggag agaaaagaaa ccaaagccga ggaggagctg 250
gatgccgaag tcctggaggt gttccacccg acgcatgagt ggcaggccct 300
tcagccaggg caggctgtcc ctgcaggatc ccacgtacgg ctgaatcttc 350
agactgggga aagagaggca aaactccaat atgaggacaa gttccgaaat 400
aatttgaaag gcaaaaggct ggatatcaac accaacacct acacatctca 450
ggatctcaag agtgcactgg caaaattcaa ggagggggca gagatggaga 500
gttcaaagga agacaaggca aggcaggctg aggtaaagcg gctcttccgc 550
cccattgagg aactgaagaa agactttgat gagctgaatg ttgtcattga 600
gactgacatg cagatcatgg tacggctgat caacaagttc aatagttcca 650
gctccagttt ggaagagaag attgctgcgc tctttgatct tgaatattat 700
gtccatcaga tggacaatgc gcaggacctg ctttcctttg gtggtcttca 750
agtggtgatc aatgggctga acagcacaga gcccctcgtg aaggagtatg 800
ctgcgtttgt gctgggcgct gccttttcca gcaaccccaa ggtccaggtg 850
gaggccatcg aaggggagc cctgcagaag ctgctggtca tcctggccac 900
ggagcagccg ctcactgcaa agaagaaggt cctgtttgca ctgtgctccc 950
tgctgcgcca cttcccctat gcccagcggc agttcctgaa gctcgggggg 1000
ctgcaggtcc tgaggaccct ggtgcaggag aagggcacgg aggtgctcgc 1050
cgtgcgcgtg gtcacactgc tctacgacct ggtcacggag aagatgttcg 1100
ccgaggagga ggctgagctg acccaggaga tgtccccaga gaagctgcag 1150
cagtatcgcc aggtacacct cctgccaggc ctgtgggaac agggctggtg 1200
cgagatcacg gcccacctcc tggcgctgcc cgagcatgat gcccgtgaga 1250
aggtgctgca gacactgggc gtcctcctga ccacctgccg ggaccgctac 1300
cgtcaggacc cccagctcgg caggacactg gccagcctgc aggctgagta 1350
ccaggtgctg gccagcctgg agctgcagga tggtgaggac gagggctact 1400
tccaggagct gctgggctct gtcaacagct tgctgaagga gctgagatga 1450
ggccccacac caggactgga ctgggatgcc gctagtgagg ctgaggggtg 1500
ccagcgtggg tgggcttctc aggcaggagg acatcttggc agtgctggct 1550
tggccattaa atggaaacct gaaggccaaa aaaaaaaaaa aaaaaaaaaa 1600
aaaaaaaaaa aaaaaaaaaa aaaaaaaaaa aaaaaaaaaa aaaaaaaaaa 1650

aaaaaaaaaa aaaaaaaaaa aaaa 1674

<210> 454
<211> 461
<212> PRT
<213> Homo Sapien

<400> 454
Met Ala Pro Gln Ser Leu Pro Ser Ser Arg Met Ala Pro Leu Gly
1 5 10 15

Met Leu Leu Gly Leu Leu Met Ala Ala Cys Phe Thr Phe Cys Leu
20 25 30

Ser His Gln Asn Leu Lys Glu Phe Ala Leu Thr Asn Pro Glu Lys
35 40 45

Ser Ser Thr Lys Glu Thr Glu Arg Lys Glu Thr Lys Ala Glu Glu
50 55 60

Glu Leu Asp Ala Glu Val Leu Glu Val Phe His Pro Thr His Glu
65 70 75

Trp Gln Ala Leu Gln Pro Gly Gln Ala Val Pro Ala Gly Ser His
80 85 90

Val Arg Leu Asn Leu Gln Thr Gly Glu Arg Glu Ala Lys Leu Gln
95 100 105

Tyr Glu Asp Lys Phe Arg Asn Asn Leu Lys Gly Lys Arg Leu Asp
110 115 120

Ile Asn Thr Asn Thr Tyr Thr Ser Gln Asp Leu Lys Ser Ala Leu
125 130 135

Ala Lys Phe Lys Glu Gly Ala Glu Met Glu Ser Ser Lys Glu Asp
140 145 150

Lys Ala Arg Gln Ala Glu Val Lys Arg Leu Phe Arg Pro Ile Glu
155 160 165

Glu Leu Lys Lys Asp Phe Asp Glu Leu Asn Val Val Ile Glu Thr
170 175 180

Asp Met Gln Ile Met Val Arg Leu Ile Asn Lys Phe Asn Ser Ser
185 190 195

Ser Ser Ser Leu Glu Glu Lys Ile Ala Ala Leu Phe Asp Leu Glu
200 205 210

Tyr Tyr Val His Gln Met Asp Asn Ala Gln Asp Leu Leu Ser Phe
215 220 225

Gly Gly Leu Gln Val Val Ile Asn Gly Leu Asn Ser Thr Glu Pro
230 235 240

Leu Val Lys Glu Tyr Ala Ala Phe Val Leu Gly Ala Ala Phe Ser
245 250 255

Ser Asn Pro Lys Val Gln Val Glu Ala Ile Glu Gly Gly Ala Leu
260 265 270

Gln Lys Leu Leu Val Ile Leu Ala Thr Glu Gln Pro Leu Thr Ala

275 280 285

Lys Lys Lys Val Leu Phe Ala Leu Cys Ser Leu Leu Arg His Phe
290 295 300

Pro Tyr Ala Gln Arg Gln Phe Leu Lys Leu Gly Gly Leu Gln Val
305 310 315

Leu Arg Thr Leu Val Gln Glu Lys Gly Thr Glu Val Leu Ala Val
320 325 330

Arg Val Val Thr Leu Leu Tyr Asp Leu Val Thr Glu Lys Met Phe
335 340 345

Ala Glu Glu Glu Ala Glu Leu Thr Gln Glu Met Ser Pro Glu Lys
350 355 360

Leu Gln Gln Tyr Arg Gln Val His Leu Leu Pro Gly Leu Trp Glu
365 370 375

Gln Gly Trp Cys Glu Ile Thr Ala His Leu Leu Ala Leu Pro Glu
380 385 390

His Asp Ala Arg Glu Lys Val Leu Gln Thr Leu Gly Val Leu Leu
395 400 405

Thr Thr Cys Arg Asp Arg Tyr Arg Gln Asp Pro Gln Leu Gly Arg
410 415 420

Thr Leu Ala Ser Leu Gln Ala Glu Tyr Gln Val Leu Ala Ser Leu
425 430 435

Glu Leu Gln Asp Gly Glu Asp Glu Gly Tyr Phe Gln Glu Leu Leu
440 445 450

Gly Ser Val Asn Ser Leu Leu Lys Glu Leu Arg
455 460

<210> 455
<211> 1570
<212> DNA
<213> Homo Sapien

<400> 455
gccccaggga gcagtgggtg gttataactc aggcccggtg cccagagccc 50

aggaggaggc agtggccagg aaggcacagg cctgagaagt ctgcggctga 100

gctgggagca aatcccccac cccctacctg ggggacaggg caagtgagac 150

ctggtgaggg tggctcagca ggcagggaag gagaggtgtc tgtgcgtcct 200

gcacccacat ctttctctgt cccctccttg ccctgtctgg aggctgctag 250

actcctatct tctgaattct atagtgcctg ggtctcagcg cagtgccgat 300

ggtggcccgt ccttgtggtt cctctctacc tggggaaata aggtgcagcg 350

gccatggcta cagcaagacc cccctggatg tgggtgctct gtgctctgat 400

cacagccttg cttctggggg tcacagagca tgttctcgcc aacaatgatg 450

tttcctgtga ccacccctct aacaccgtgc cctctgggag caaccaggac 500

ctgggagctg gggccgggga agacgcccgg tcggatgaca gcagcagccg 550

catcatcaat ggatccgact gcgatatgca cacccagccg tggcaggccg 600

cgctgttgct aaggcccaac cagctctact gcggggcggt gttggtgcat 650

ccacagtggc tgctcacggc cgcccactgc aggaagaaag ttttcagagt 700

ccgtctcggc cactactccc tgtcaccagt ttatgaatct gggcagcaga 750

tgttccaggg ggtcaaatcc atcccccacc ctggctactc ccaccctggc 800

cactctaacg acctcatgct catcaaactg aacagaagaa ttcgtcccac 850

taaagatgtc agacccatca acgtctcctc tcattgtccc tctgctggga 900

caaagtgctt ggtgtctggc tgggggacaa ccaagagccc ccaagtgcac 950

ttccctaagg tcctccagtg cttgaatatc agcgtgctaa gtcagaaaag 1000

gtgcgaggat gcttacccga gacagataga tgacaccatg ttctgcgccg 1050

gtgacaaagc aggtagagac tcctgccagg gtgattctgg ggggcctgtg 1100

gtctgcaatg gctccctgca gggactcgtg tcctggggag attacccttg 1150

tgcccggccc aacagaccgg gtgtctacac gaacctctgc aagttcacca 1200

agtggatcca ggaaaccatc caggccaact cctgagtcat cccaggactc 1250

agcacaccgg catccccacc tgctgcaggg acagccctga cactcctttc 1300

agaccctcat tccttcccag agatgttgag aatgttcatc tctccagccc 1350

ctgaccccat gtctcctgga ctcagggtct gcttcccccca cattgggctg 1400

accgtgtctc tctagttgaa ccctgggaac aatttccaaa actgtccagg 1450

gcgggggttg cgtctcaatc tccctggggc actttcatcc tcaagctcag 1500

ggcccatccc ttctctgcag ctctgaccca aatttagtcc cagaaataaa 1550

ctgagaagtg gaaaaaaaaa 1570

<210> 456
<211> 293
<212> PRT
<213> Homo Sapien

<400> 456

| | | | | | | | | | | | | | | |
|---|---|---|---|---|---|---|---|---|---|---|---|---|---|---|
| Met | Ala | Thr | Ala | Arg | Pro | Pro | Trp | Met | Trp | Val | Leu | Cys | Ala | Leu |
| 1 | | | | 5 | | | | | 10 | | | | | 15 |
| Ile | Thr | Ala | Leu | Leu | Leu | Gly | Val | Thr | Glu | His | Val | Leu | Ala | Asn |
| | | | | 20 | | | | | 25 | | | | | 30 |
| Asn | Asp | Val | Ser | Cys | Asp | His | Pro | Ser | Asn | Thr | Val | Pro | Ser | Gly |
| | | | | 35 | | | | | 40 | | | | | 45 |
| Ser | Asn | Gln | Asp | Leu | Gly | Ala | Gly | Ala | Gly | Glu | Asp | Ala | Arg | Ser |
| | | | | 50 | | | | | 55 | | | | | 60 |
| Asp | Asp | Ser | Ser | Ser | Arg | Ile | Ile | Asn | Gly | Ser | Asp | Cys | Asp | Met |
| | | | | 65 | | | | | 70 | | | | | 75 |

```
His Thr Gln Pro Trp Gln Ala Ala Leu Leu Leu Arg Pro Asn Gln
                80                  85                  90

Leu Tyr Cys Gly Ala Val Leu Val His Pro Gln Trp Leu Leu Thr
                95                  100                 105

Ala Ala His Cys Arg Lys Lys Val Phe Arg Val Arg Leu Gly His
                110                 115                 120

Tyr Ser Leu Ser Pro Val Tyr Glu Ser Gly Gln Gln Met Phe Gln
                125                 130                 135

Gly Val Lys Ser Ile Pro His Pro Gly Tyr Ser His Pro Gly His
                140                 145                 150

Ser Asn Asp Leu Met Leu Ile Lys Leu Asn Arg Arg Ile Arg Pro
                155                 160                 165

Thr Lys Asp Val Arg Pro Ile Asn Val Ser Ser His Cys Pro Ser
                170                 175                 180

Ala Gly Thr Lys Cys Leu Val Ser Gly Trp Gly Thr Thr Lys Ser
                185                 190                 195

Pro Gln Val His Phe Pro Lys Val Leu Gln Cys Leu Asn Ile Ser
                200                 205                 210

Val Leu Ser Gln Lys Arg Cys Glu Asp Ala Tyr Pro Arg Gln Ile
                215                 220                 225

Asp Asp Thr Met Phe Cys Ala Gly Asp Lys Ala Gly Arg Asp Ser
                230                 235                 240

Cys Gln Gly Asp Ser Gly Gly Pro Val Val Cys Asn Gly Ser Leu
                245                 250                 255

Gln Gly Leu Val Ser Trp Gly Asp Tyr Pro Cys Ala Arg Pro Asn
                260                 265                 270

Arg Pro Gly Val Tyr Thr Asn Leu Cys Lys Phe Thr Lys Trp Ile
                275                 280                 285

Gln Glu Thr Ile Gln Ala Asn Ser
                290
```

```
<210> 457
<211> 1841
<212> DNA
<213> Homo Sapien

<400> 457
gcagtcagag acttcccctg cccctcgctg ggaaagaaca ttaggaatgc 50

cttttagtgc cttgcttcct gaactagctc acagtagccc ggcggcccag 100

ggcaatccga ccacatttca ctctcaccgc tgtaggaatc cagatgcagg 150

ccaagtacag cagcacgagg gacatgctgg atgatgatgg ggacaccacc 200

atgagcctgc attctcaagc ctctgccaca actcggcatc cagagccccg 250

gcgcacagag cacagggctc cctcttcaac gtggcgacca gtggccctga 300
```

ccctgctgac tttgtgcttg gtgctgctga tagggctggc agccctgggg 350
cttttgtttt ttcagtacta ccagctctcc aatactggtc aagacaccat 400
ttctcaaatg gaagaaagat taggaaatac gtcccaagag ttgcaatctc 450
ttcaagtcca gaatataaag cttgcaggaa gtctgcagca tgtggctgaa 500
aaactctgtc gtgagctgta taacaaagct ggagcacaca ggtgcagccc 550
ttgtacagaa caatggaaat ggcatggaga caattgctac cagttctata 600
aagacagcaa aagttgggag gactgtaaat atttctgcct tagtgaaaac 650
tctaccatgc tgaagataaa caaacaagaa gacctggaat ttgccgcgtc 700
tcagagctac tctgagtttt tctactctta ttggacaggg cttttgcgcc 750
ctgacagtgg caaggcctgg ctgtggatgg atggaacccc tttcacttct 800
gaactgttcc atattataat agatgtcacc agcccaagaa gcagagactg 850
tgtggccatc ctcaatggga tgatcttctc aaaggactgc aaagaattga 900
agcgttgtgt ctgtgagaga agggcaggaa tggtgaagcc agagagcctc 950
catgtccccc ctgaaacatt aggcgaaggt gactgattcg ccctctgcaa 1000
ctacaaatag cagagtgagc caggcggtgc caaagcaagg gctagttgag 1050
acattgggaa atggaacata atcaggaaag actatctctc tgactagtac 1100
aaaatgggtt ctcgtgtttc ctgttcagga tcaccagcat ttctgagctt 1150
gggtttatgc acgtatttaa cagtcacaag aagtcttatt tacatgccac 1200
caaccaacct cagaaaccca taatgtcatc tgccttcttg gcttagagat 1250
aacttttagc tctctttctt ctcaatgtct aatatcacct ccctgttttc 1300
atgtcttcct tacacttggt ggaataagaa actttttgaa gtagaggaaa 1350
tacattgagg taacatcctt ttctctgaca gtcaagtagt ccatcagaaa 1400
ttggcagtca cttcccagat tgtaccagca aatacacaag gaattctttt 1450
tgtttgtttc agttcatact agtcccttcc caatccatca gtaaagaccc 1500
catctgcctt gtccatgccg tttcccaaca gggatgtcac ttgatatgag 1550
aatctcaaat ctcaatgcct tataagcatt ccttcctgtg tccattaaga 1600
ctctgataat tgtctcccct ccataggaat ttctcccagg aaagaaatat 1650
atccccatct ccgtttcata tcagaactac cgtccccgat attcccttca 1700
gagagattaa agaccagaaa aaagtgagcc tcttcatctg cacctgtaat 1750
agtttcagtt cctattttct tccattgacc catatttata cctttcaggt 1800
actgaagatt taataataat aaatgtaaat actgtgaaaa a 1841

<210> 458
<211> 280

```
<212> PRT
<213> Homo Sapien

<400> 458
Met Gln Ala Lys Tyr Ser Ser Thr Arg Asp Met Leu Asp Asp Asp
1               5                   10                  15

Gly Asp Thr Thr Met Ser Leu His Ser Gln Ala Ser Ala Thr Thr
                20                  25                  30

Arg His Pro Glu Pro Arg Arg Thr Glu His Arg Ala Pro Ser Ser
                35                  40                  45

Thr Trp Arg Pro Val Ala Leu Thr Leu Leu Thr Leu Cys Leu Val
                50                  55                  60

Leu Leu Ile Gly Leu Ala Ala Leu Gly Leu Leu Phe Phe Gln Tyr
                65                  70                  75

Tyr Gln Leu Ser Asn Thr Gly Gln Asp Thr Ile Ser Gln Met Glu
                80                  85                  90

Glu Arg Leu Gly Asn Thr Ser Gln Glu Leu Gln Ser Leu Gln Val
                95                  100                 105

Gln Asn Ile Lys Leu Ala Gly Ser Leu Gln His Val Ala Glu Lys
                110                 115                 120

Leu Cys Arg Glu Leu Tyr Asn Lys Ala Gly Ala His Arg Cys Ser
                125                 130                 135

Pro Cys Thr Glu Gln Trp Lys Trp His Gly Asp Asn Cys Tyr Gln
                140                 145                 150

Phe Tyr Lys Asp Ser Lys Ser Trp Glu Asp Cys Lys Tyr Phe Cys
                155                 160                 165

Leu Ser Glu Asn Ser Thr Met Leu Lys Ile Asn Lys Gln Glu Asp
                170                 175                 180

Leu Glu Phe Ala Ala Ser Gln Ser Tyr Ser Glu Phe Phe Tyr Ser
                185                 190                 195

Tyr Trp Thr Gly Leu Leu Arg Pro Asp Ser Gly Lys Ala Trp Leu
                200                 205                 210

Trp Met Asp Gly Thr Pro Phe Thr Ser Glu Leu Phe His Ile Ile
                215                 220                 225

Ile Asp Val Thr Ser Pro Arg Ser Arg Asp Cys Val Ala Ile Leu
                230                 235                 240

Asn Gly Met Ile Phe Ser Lys Asp Cys Lys Glu Leu Lys Arg Cys
                245                 250                 255

Val Cys Glu Arg Arg Ala Gly Met Val Lys Pro Glu Ser Leu His
                260                 265                 270

Val Pro Pro Glu Thr Leu Gly Glu Gly Asp
                275                 280

<210> 459
<211> 1337
<212> DNA
```

<213> Homo Sapien

<400> 459
gttgatggca aacttcctca aaggaggggc agagcctgcg cagggcagga 50

gcagctggcc cactggcggc ccgcaacact ccgtctcacc ctctgggccc 100

actgcatcta gaggagggcc gtctgtgagg ccactacccc tccagcaact 150

gggaggtggg actgtcagaa gctggcccag ggtggtggtc agctgggtca 200

gggacctacg gcacctgctg gaccacctcg ccttctccat cgaagcaggg 250

aagtgggagc ctcgagccct cgggtggaag ctgaccccaa gccacccttc 300

acctggacag gatgagagtg tcaggtgtgc ttcgcctcct ggccctcatc 350

tttgccatag tcacgacatg gatgtttatt cgaagctaca tgagcttcag 400

catgaaaacc atccgtctgc cacgctggct ggcagcctcg cccaccaagg 450

agatccaggt taaaaagtac aagtgtggcc tcatcaagcc ctgcccagcc 500

aactactttg cgtttaaaat ctgcagtggg gccgccaacg tcgtgggccc 550

tactatgtgc tttgaagacc gcatgatcat gagtcctgtg aaaaacaatg 600

tgggcagagg cctaaacatc gccctggtga atggaaccac gggagctgtg 650

ctgggacaga aggcatttga catgtactct ggagatgtta tgcacctagt 700

gaaattcctt aaagaaattc cgggggtgc actggtgctg gtggcctcct 750

acgacgatcc agggaccaaa atgaacgatg aaagcaggaa actcttctct 800

gacttgggga gttcctacgc aaaacaactg ggcttccggg acagctgggt 850

cttcatagga gccaaagacc tcaggggtaa aagccccttt gagcagttct 900

taaagaacag cccagacaca aacaaatacg agggatggcc agagctgctg 950

gagatggagg gctgcatgcc cccgaagcca ttttagggtg gctgtggctc 1000

ttcctcagcc aggggcctga agaagctcct gcctgactta ggagtcagag 1050

cccggcaggg gctgaggagg aggagcaggg ggtgctgcgt ggaaggtgct 1100

gcaggtcctt gcacgctgtg tcgcgcctct cctcctcgga aacagaaccc 1150

tcccacagca catcctaccc ggaagaccag cctcagaggg tccttctgga 1200

accagctgtc tgtggagaga atggggtgct ttcgtcaggg actgctgacg 1250

gctggtcctg aggaaggaca aactgcccag acttgagccc aattaaattt 1300

tatttttgct ggttttgaaa aaaaaaaaaa aaaaaaa 1337

<210> 460
<211> 224
<212> PRT
<213> Homo Sapien

<400> 460
Met Arg Val Ser Gly Val Leu Arg Leu Leu Ala Leu Ile Phe Ala

```
1               5                   10                  15

Ile Val Thr Thr Trp Met Phe Ile Arg Ser Tyr Met Ser Phe Ser
                20                  25                  30

Met Lys Thr Ile Arg Leu Pro Arg Trp Leu Ala Ala Ser Pro Thr
                35                  40                  45

Lys Glu Ile Gln Val Lys Lys Tyr Lys Cys Gly Leu Ile Lys Pro
                50                  55                  60

Cys Pro Ala Asn Tyr Phe Ala Phe Lys Ile Cys Ser Gly Ala Ala
                65                  70                  75

Asn Val Val Gly Pro Thr Met Cys Phe Glu Asp Arg Met Ile Met
                80                  85                  90

Ser Pro Val Lys Asn Asn Val Gly Arg Gly Leu Asn Ile Ala Leu
                95                  100                 105

Val Asn Gly Thr Thr Gly Ala Val Leu Gly Gln Lys Ala Phe Asp
                110                 115                 120

Met Tyr Ser Gly Asp Val Met His Leu Val Lys Phe Leu Lys Glu
                125                 130                 135

Ile Pro Gly Gly Ala Leu Val Leu Val Ala Ser Tyr Asp Asp Pro
                140                 145                 150

Gly Thr Lys Met Asn Asp Glu Ser Arg Lys Leu Phe Ser Asp Leu
                155                 160                 165

Gly Ser Ser Tyr Ala Lys Gln Leu Gly Phe Arg Asp Ser Trp Val
                170                 175                 180

Phe Ile Gly Ala Lys Asp Leu Arg Gly Lys Ser Pro Phe Glu Gln
                185                 190                 195

Phe Leu Lys Asn Ser Pro Asp Thr Asn Lys Tyr Glu Gly Trp Pro
                200                 205                 210

Glu Leu Leu Glu Met Glu Gly Cys Met Pro Pro Lys Pro Phe
                215                 220
```

<210> 461
<211> 2528
<212> DNA
<213> Homo Sapien

<400> 461

```
aaactcagca cttgccggag tggctcattg ttaagacaaa gggtgtgcac 50

ttcctggcca ggaaacctga gcggtgagac tcccagctgc ctacatcaag 100

gccccaggac atgcagaacc ttcctctaga acccgaccca ccaccatgag 150

gtcctgcctg tggagatgca ggcacctgag ccaaggcgtc cagtggtcct 200

tgcttctggc tgtcctggtc ttctttctct tcgccttgcc ctcttttatt 250

aaggagcctc aaacaaagcc ttccaggcat caacgcacag agaacattaa 300

agaaaggtct ctacagtccc tggcaaagcc taagtcccag gcacccacaa 350
```

gggcgaggag gacaaccatc tatgcagagc cagcgccaga gaacaatgcc 400
ctcaacacac aaacccagcc caaggcccac accaccggag acagaggaaa 450
ggaggccaac caggcaccgc cggaggagca ggacaaggtg ccccacacag 500
cacagagggc agcatggaag agcccagaaa aagagaaaac catggtgaac 550
acactgtcac ccagagggca agatgcaggg atggcctctg gcaggacaga 600
ggcacaatca tggaagagcc aggacacaaa gacgacccaa ggaaatgggg 650
gccagaccag gaagctgacg gcctccagga cggtgtcaga gaagcaccag 700
ggcaaagcgg caaccacagc caagacgctc attcccaaaa gtcagcacag 750
aatgctggct cccacaggag cagtgtcaac aaggacgaga cagaaaggag 800
tgaccacagc agtcatccca cctaaggaga agaaacctca ggccaccccca 850
ccccctgccc ctttccagag ccccacgacg cagagaaacc aaagactgaa 900
ggccgccaac ttcaaatctg agcctcggtg ggattttgag gaaaaataca 950
gcttcgaaat aggaggcctt cagacgactt gccctgactc tgtgaagatc 1000
aaagcctcca agtcgctgtg gctccagaaa ctctttctgc ccaacctcac 1050
tctcttcctg gactccagac acttcaacca gagtgagtgg gaccgcctgg 1100
aacactttgc accacccttt ggcttcatgg agctcaacta ctccttggtg 1150
cagaaggtcg tgacacgctt ccctccagtg ccccagcagc agctgctcct 1200
ggccagcctc cccgctggga gcctccggtg catcacctgt gccgtggtgg 1250
gcaacggggg catcctgaac aactcccaca tgggccagga gatagacagt 1300
cacgactacg tgttccgatt gagcggagct ctcattaaag gctacgaaca 1350
ggatgtgggg actcggacat ccttctacgg ctttaccgcc ttctccctga 1400
cccagtcact ccttatattg ggcaatcggg gtttcaagaa cgtgcctctt 1450
gggaaggacg tccgctactt gcacttcctg gaaggcaccc gggactatga 1500
gtggctggaa gcactgctta tgaatcagac ggtgatgtca aaaaaccttt 1550
tctggttcag gcacagaccc caggaagctt ttcgggaagc cctgcacatg 1600
gacaggtacc tgttgctgca cccagacttt ctccgataca tgaagaacag 1650
gtttctgagg tctaagaccc tggatggtgc ccactggagg atataccgcc 1700
ccaccactgg ggccctcctg ctgctcactg cccttcagct ctgtgaccag 1750
gtgagtgctt atggcttcat cactgagggc catgagcgct tttctgatca 1800
ctactatgat acatcatgga agcggctgat cttttacata aaccatgact 1850
tcaagctgga gagagaagtc tggaagcggc tacacgatga agggataatc 1900
cggctgtacc agcgtcctgg tcccggaact gccaaagcca agaactgacc 1950

ggggccaggg ctgccatggt ctccttgcct gctccaaggc acaggataca 2000

gtgggaatct tgagactctt tggccatttc ccatggctca gactaagctc 2050

caagcccttc aggagttcca agggaacact tgaaccatgg acaagactct 2100

ctcaagatgg caaatggcta attgaggttc tgaagttctt cagtacattg 2150

ctgtaggtcc tgaggccagg gatttttaat taaatggggt gatgggtggc 2200

caataccaca attcctgctg aaaaacactc ttccagtcca aaagcttctt 2250

gatacagaaa aaagagcctg gatttacaga aacatataga tctggtttga 2300

attccagatc gagtttacag ttgtgaaatc ttgaaggtat tacttaactt 2350

cactacagat tgtctagaag acctttctag gagttatctg attctagaag 2400

ggtctatact tgtccttgtc tttaagctat ttgacaactc tacgtgttgt 2450

agaaaactga taataataca aatgattgtt gtccatggaa aggcaaataa 2500

attttctaca gtgaaaaaaa aaaaaaaa 2528

<210> 462
<211> 600
<212> PRT
<213> Homo Sapien

<400> 462
Met Arg Ser Cys Leu Trp Arg Cys Arg His Leu Ser Gln Gly Val
1               5                   10                  15

Gln Trp Ser Leu Leu Leu Ala Val Leu Val Phe Phe Leu Phe Ala
                20                  25                  30

Leu Pro Ser Phe Ile Lys Glu Pro Gln Thr Lys Pro Ser Arg His
                35                  40                  45

Gln Arg Thr Glu Asn Ile Lys Glu Arg Ser Leu Gln Ser Leu Ala
                50                  55                  60

Lys Pro Lys Ser Gln Ala Pro Thr Arg Ala Arg Arg Thr Thr Ile
                65                  70                  75

Tyr Ala Glu Pro Ala Pro Glu Asn Asn Ala Leu Asn Thr Gln Thr
                80                  85                  90

Gln Pro Lys Ala His Thr Thr Gly Asp Arg Gly Lys Glu Ala Asn
                95                  100                 105

Gln Ala Pro Pro Glu Glu Gln Asp Lys Val Pro His Thr Ala Gln
                110                 115                 120

Arg Ala Ala Trp Lys Ser Pro Glu Lys Glu Lys Thr Met Val Asn
                125                 130                 135

Thr Leu Ser Pro Arg Gly Gln Asp Ala Gly Met Ala Ser Gly Arg
                140                 145                 150

Thr Glu Ala Gln Ser Trp Lys Ser Gln Asp Thr Lys Thr Thr Gln
                155                 160                 165

Gly Asn Gly Gly Gln Thr Arg Lys Leu Thr Ala Ser Arg Thr Val

170 175 180

Ser Glu Lys His Gln Gly Lys Ala Ala Thr Thr Ala Lys Thr Leu
185 190 195

Ile Pro Lys Ser Gln His Arg Met Leu Ala Pro Thr Gly Ala Val
200 205 210

Ser Thr Arg Thr Arg Gln Lys Gly Val Thr Thr Ala Val Ile Pro
215 220 225

Pro Lys Glu Lys Lys Pro Gln Ala Thr Pro Pro Pro Ala Pro Phe
230 235 240

Gln Ser Pro Thr Thr Gln Arg Asn Gln Arg Leu Lys Ala Ala Asn
245 250 255

Phe Lys Ser Glu Pro Arg Trp Asp Phe Glu Glu Lys Tyr Ser Phe
260 265 270

Glu Ile Gly Gly Leu Gln Thr Thr Cys Pro Asp Ser Val Lys Ile
275 280 285

Lys Ala Ser Lys Ser Leu Trp Leu Gln Lys Leu Phe Leu Pro Asn
290 295 300

Leu Thr Leu Phe Leu Asp Ser Arg His Phe Asn Gln Ser Glu Trp
305 310 315

Asp Arg Leu Glu His Phe Ala Pro Pro Phe Gly Phe Met Glu Leu
320 325 330

Asn Tyr Ser Leu Val Gln Lys Val Val Thr Arg Phe Pro Pro Val
335 340 345

Pro Gln Gln Gln Leu Leu Leu Ala Ser Leu Pro Ala Gly Ser Leu
350 355 360

Arg Cys Ile Thr Cys Ala Val Val Gly Asn Gly Gly Ile Leu Asn
365 370 375

Asn Ser His Met Gly Gln Glu Ile Asp Ser His Asp Tyr Val Phe
380 385 390

Arg Leu Ser Gly Ala Leu Ile Lys Gly Tyr Glu Gln Asp Val Gly
395 400 405

Thr Arg Thr Ser Phe Tyr Gly Phe Thr Ala Phe Ser Leu Thr Gln
410 415 420

Ser Leu Leu Ile Leu Gly Asn Arg Gly Phe Lys Asn Val Pro Leu
425 430 435

Gly Lys Asp Val Arg Tyr Leu His Phe Leu Glu Gly Thr Arg Asp
440 445 450

Tyr Glu Trp Leu Glu Ala Leu Leu Met Asn Gln Thr Val Met Ser
455 460 465

Lys Asn Leu Phe Trp Phe Arg His Arg Pro Gln Glu Ala Phe Arg
470 475 480

Glu Ala Leu His Met Asp Arg Tyr Leu Leu Leu His Pro Asp Phe
485 490 495

```
Leu Arg Tyr Met Lys Asn Arg Phe Leu Arg Ser Lys Thr Leu Asp
                500                 505                 510

Gly Ala His Trp Arg Ile Tyr Arg Pro Thr Thr Gly Ala Leu Leu
                515                 520                 525

Leu Leu Thr Ala Leu Gln Leu Cys Asp Gln Val Ser Ala Tyr Gly
                530                 535                 540

Phe Ile Thr Glu Gly His Glu Arg Phe Ser Asp His Tyr Tyr Asp
                545                 550                 555

Thr Ser Trp Lys Arg Leu Ile Phe Tyr Ile Asn His Asp Phe Lys
                560                 565                 570

Leu Glu Arg Glu Val Trp Lys Arg Leu His Asp Glu Gly Ile Ile
                575                 580                 585

Arg Leu Tyr Gln Arg Pro Gly Pro Gly Thr Ala Lys Ala Lys Asn
                590                 595                 600
```

<210> 463
<211> 3226
<212> DNA
<213> Homo Sapien

<400> 463

```
gggggagcta ggccggcggc agtggtggtg gcggcggcgc aagggtgagg 50

gcggccccag aaccccaggt aggtagagca agaagatggt gtttctgccc 100

ctcaaatggt cccttgcaac catgtcattt ctactttcct cactgttggc 150

tctcttaact gtgtccactc cttcatggtg tcagagcact gaagcatctc 200

caaaacgtag tgatgggaca ccatttcctt ggaataaaat acgacttcct 250

gagtacgtca tcccagttca ttatgatctc ttgatccatg caaaccttac 300

cacgctgacc ttctggggaa ccacgaaagt agaaatcaca gccagtcagc 350

ccaccagcac catcatcctg catagtcacc acctgcagat atctagggcc 400

accctcagga agggagctgg agagaggcta tcggaagaac ccctgcaggt 450

cctggaacac ccccctcagg agcaaattgc actgctggct cccgagcccc 500

tccttgtcgg gctcccgtac acagttgtca ttcactatgc tggcaatctt 550

tcggagactt tccacggatt ttacaaaagc acctacagaa ccaaggaagg 600

ggaactgagg atactagcat caacacaatt tgaacccact gcagctagaa 650

tggcctttcc ctgctttgat gaacctgcct tcaaagcaag tttctcaatc 700

aaaattagaa gagagccaag gcacctagcc atctccaata tgccattggt 750

gaaatctgtg actgttgctg aaggactcat agaagaccat tttgatgtca 800

ctgtgaagat gagcacctat ctggtggcct tcatcatttc agattttgag 850

tctgtcagca agataaccaa gagtggagtc aaggtttctg tttatgctgt 900
```

gccagacaag ataaatcaag cagattatgc actggatgct gcggtgactc 950
ttctagaatt ttatgaggat tatttcagca taccgtatcc cctacccaaa 1000
caagatcttg ctgctattcc cgactttcag tctggtgcta tggaaaactg 1050
gggactgaca acatatagag aatctgctct gttgtttgat gcagaaaagt 1100
cttctgcatc aagtaagctt ggcatcacag tgactgtggc ccatgaactg 1150
gcccaccagt ggtttgggaa cctggtcact atggaatggt ggaatgatct 1200
ttggctaaat gaaggatttg ccaaatttat ggagtttgtg tctgtcagtg 1250
tgacccatcc tgaactgaaa gttggagatt atttctttgg caaatgtttt 1300
gacgcaatgg aggtagatgc tttaaattcc tcacaccctg tgtctacacc 1350
tgtggaaaat cctgctcaga tccgggagat gtttgatgat gtttcttatg 1400
ataagggagc ttgtattctg aatatgctaa gggagtatct tagcgctgac 1450
gcatttaaaa gtggtattgt acagtatctc cagaagcata gctataaaaa 1500
tacaaaaaac gaggacctgt gggatagtat ggcaagtatt tgccctacag 1550
atggtgtaaa agggatggat ggcttttgct ctagaagtca acattcatct 1600
tcatcctcac attggcatca ggaaggggtg gatgtgaaaa ccatgatgaa 1650
cacttggaca ctgcagaggg gttttcccct aataaccatc acagtgaggg 1700
ggaggaatgt acacatgaag caagagcact acatgaaggg ctctgacggc 1750
gccccggaca ctgggtacct gtggcatgtt ccattgacat tcatcaccag 1800
caaatccaac atggtccatc gatttttgct aaaaacaaaa acagatgtgc 1850
tcatcctccc agaagaggtg gaatggatca aatttaatgt gggcatgaat 1900
ggctattaca ttgtgcatta cgaggatgat ggatgggact ctttgactgg 1950
ccttttaaaa ggaacacaca cagcagtcag cagtaatgat cgggcaagtc 2000
tcattaacaa tgcatttcag ctcgtcagca ttgggaagct gtccattgaa 2050
aaggccttgg atttatccct gtacttgaaa catgaaactg aaattatgcc 2100
cgtgtttcaa ggtttgaatg agctgattcc tatgtataag ttaatggaga 2150
aaagagatat gaatgaagtg gaaactcaat tcaaggcctt cctcatcagg 2200
ctgctaaggg acctcattga taagcagaca tggacagacg agggctcagt 2250
ctcagagcaa atgctgcgga gtgaactact actcctcgcc tgtgtgcaca 2300
actatcagcc gtgcgtacag agggcagaag gctatttcag aaagtggaag 2350
gaatccaatg gaaacttgag cctgcctgtc gacgtgacct tggcagtgtt 2400
tgctgtgggg gcccagagca cagaaggctg ggattttctt tatagtaaat 2450
atcagttttc tttgtccagt actgagaaaa gccaaattga atttgccctc 2500

tgcagaaccc aaaataagga aaagcttcaa tggctactag atgaaagctt 2550

taagggagat aaaataaaaa ctcaggagtt tccacaaatt cttacactca 2600

ttggcaggaa cccagtagga tacccactgg cctggcaatt tctgaggaaa 2650

aactggaaca aacttgtaca aaagtttgaa cttggctcat cttccatagc 2700

ccacatggta atgggtacaa caaatcaatt ctccacaaga acacggcttg 2750

aagaggtaaa aggattcttc agctctttga aagaaaatgg ttctcagctc 2800

cgttgtgtcc aacagacaat tgaaaccatt gaagaaaaca tcggttggat 2850

ggataagaat tttgataaaa tcagagtgtg gctgcaaagt gaaaagcttg 2900

aacgtatgta aaaattcctc ccttgcccgg ttcctgttat ctctaatcac 2950

caacattttg ttgagtgtat tttcaaacta gagatggctg ttttggctcc 3000

aactggagat actttttttcc cttcaactca ttttttgact atccctgtga 3050

aaagaatagc tgttagtttt tcatgaatgg gctttttcat gaatgggcta 3100

tcgctaccat gtgttttgtt catcacaggt gttgccctgc aacgtaaacc 3150

caagtgttgg gttccctgcc acagaagaat aaagtacctt attcttctca 3200

aaaaaaaaaa aaaaaaaaaa aaaaaa 3226

<210> 464
<211> 941
<212> PRT
<213> Homo Sapien

<400> 464
Met Val Phe Leu Pro Leu Lys Trp Ser Leu Ala Thr Met Ser Phe
1               5                   10                  15

Leu Leu Ser Ser Leu Leu Ala Leu Leu Thr Val Ser Thr Pro Ser
                20                  25                  30

Trp Cys Gln Ser Thr Glu Ala Ser Pro Lys Arg Ser Asp Gly Thr
                35                  40                  45

Pro Phe Pro Trp Asn Lys Ile Arg Leu Pro Glu Tyr Val Ile Pro
                50                  55                  60

Val His Tyr Asp Leu Leu Ile His Ala Asn Leu Thr Thr Leu Thr
                65                  70                  75

Phe Trp Gly Thr Thr Lys Val Glu Ile Thr Ala Ser Gln Pro Thr
                80                  85                  90

Ser Thr Ile Ile Leu His Ser His His Leu Gln Ile Ser Arg Ala
                95                  100                 105

Thr Leu Arg Lys Gly Ala Gly Glu Arg Leu Ser Glu Glu Pro Leu
                110                 115                 120

Gln Val Leu Glu His Pro Pro Gln Glu Gln Ile Ala Leu Leu Ala
                125                 130                 135

Pro Glu Pro Leu Leu Val Gly Leu Pro Tyr Thr Val Val Ile His

```
                140                 145                 150

Tyr Ala Gly Asn Leu Ser Glu Thr Phe His Gly Phe Tyr Lys Ser
                155                 160                 165

Thr Tyr Arg Thr Lys Glu Gly Glu Leu Arg Ile Leu Ala Ser Thr
                170                 175                 180

Gln Phe Glu Pro Thr Ala Ala Arg Met Ala Phe Pro Cys Phe Asp
                185                 190                 195

Glu Pro Ala Phe Lys Ala Ser Phe Ser Ile Lys Ile Arg Arg Glu
                200                 205                 210

Pro Arg His Leu Ala Ile Ser Asn Met Pro Leu Val Lys Ser Val
                215                 220                 225

Thr Val Ala Glu Gly Leu Ile Glu Asp His Phe Asp Val Thr Val
                230                 235                 240

Lys Met Ser Thr Tyr Leu Val Ala Phe Ile Ile Ser Asp Phe Glu
                245                 250                 255

Ser Val Ser Lys Ile Thr Lys Ser Gly Val Lys Val Ser Val Tyr
                260                 265                 270

Ala Val Pro Asp Lys Ile Asn Gln Ala Asp Tyr Ala Leu Asp Ala
                275                 280                 285

Ala Val Thr Leu Leu Glu Phe Tyr Glu Asp Tyr Phe Ser Ile Pro
                290                 295                 300

Tyr Pro Leu Pro Lys Gln Asp Leu Ala Ala Ile Pro Asp Phe Gln
                305                 310                 315

Ser Gly Ala Met Glu Asn Trp Gly Leu Thr Thr Tyr Arg Glu Ser
                320                 325                 330

Ala Leu Leu Phe Asp Ala Glu Lys Ser Ser Ala Ser Ser Lys Leu
                335                 340                 345

Gly Ile Thr Val Thr Val Ala His Glu Leu Ala His Gln Trp Phe
                350                 355                 360

Gly Asn Leu Val Thr Met Glu Trp Trp Asn Asp Leu Trp Leu Asn
                365                 370                 375

Glu Gly Phe Ala Lys Phe Met Glu Phe Val Ser Val Ser Val Thr
                380                 385                 390

His Pro Glu Leu Lys Val Gly Asp Tyr Phe Phe Gly Lys Cys Phe
                395                 400                 405

Asp Ala Met Glu Val Asp Ala Leu Asn Ser Ser His Pro Val Ser
                410                 415                 420

Thr Pro Val Glu Asn Pro Ala Gln Ile Arg Glu Met Phe Asp Asp
                425                 430                 435

Val Ser Tyr Asp Lys Gly Ala Cys Ile Leu Asn Met Leu Arg Glu
                440                 445                 450

Tyr Leu Ser Ala Asp Ala Phe Lys Ser Gly Ile Val Gln Tyr Leu
                455                 460                 465
```

Gln Lys His Ser Tyr Lys Asn Thr Lys Asn Glu Asp Leu Trp Asp
470 475 480

Ser Met Ala Ser Ile Cys Pro Thr Asp Gly Val Lys Gly Met Asp
485 490 495

Gly Phe Cys Ser Arg Ser Gln His Ser Ser Ser Ser Ser His Trp
500 505 510

His Gln Glu Gly Val Asp Val Lys Thr Met Met Asn Thr Trp Thr
515 520 525

Leu Gln Arg Gly Phe Pro Leu Ile Thr Ile Thr Val Arg Gly Arg
530 535 540

Asn Val His Met Lys Gln Glu His Tyr Met Lys Gly Ser Asp Gly
545 550 555

Ala Pro Asp Thr Gly Tyr Leu Trp His Val Pro Leu Thr Phe Ile
560 565 570

Thr Ser Lys Ser Asn Met Val His Arg Phe Leu Leu Lys Thr Lys
575 580 585

Thr Asp Val Leu Ile Leu Pro Glu Glu Val Glu Trp Ile Lys Phe
590 595 600

Asn Val Gly Met Asn Gly Tyr Tyr Ile Val His Tyr Glu Asp Asp
605 610 615

Gly Trp Asp Ser Leu Thr Gly Leu Leu Lys Gly Thr His Thr Ala
620 625 630

Val Ser Ser Asn Asp Arg Ala Ser Leu Ile Asn Asn Ala Phe Gln
635 640 645

Leu Val Ser Ile Gly Lys Leu Ser Ile Glu Lys Ala Leu Asp Leu
650 655 660

Ser Leu Tyr Leu Lys His Glu Thr Glu Ile Met Pro Val Phe Gln
665 670 675

Gly Leu Asn Glu Leu Ile Pro Met Tyr Lys Leu Met Glu Lys Arg
680 685 690

Asp Met Asn Glu Val Glu Thr Gln Phe Lys Ala Phe Leu Ile Arg
695 700 705

Leu Leu Arg Asp Leu Ile Asp Lys Gln Thr Trp Thr Asp Glu Gly
710 715 720

Ser Val Ser Glu Gln Met Leu Arg Ser Glu Leu Leu Leu Leu Ala
725 730 735

Cys Val His Asn Tyr Gln Pro Cys Val Gln Arg Ala Glu Gly Tyr
740 745 750

Phe Arg Lys Trp Lys Glu Ser Asn Gly Asn Leu Ser Leu Pro Val
755 760 765

Asp Val Thr Leu Ala Val Phe Ala Val Gly Ala Gln Ser Thr Glu
770 775 780

```
Gly Trp Asp Phe Leu Tyr Ser Lys Tyr Gln Phe Ser Leu Ser Ser
                785                 790                 795

Thr Glu Lys Ser Gln Ile Glu Phe Ala Leu Cys Arg Thr Gln Asn
                800                 805                 810

Lys Glu Lys Leu Gln Trp Leu Leu Asp Glu Ser Phe Lys Gly Asp
                815                 820                 825

Lys Ile Lys Thr Gln Glu Phe Pro Gln Ile Leu Thr Leu Ile Gly
                830                 835                 840

Arg Asn Pro Val Gly Tyr Pro Leu Ala Trp Gln Phe Leu Arg Lys
                845                 850                 855

Asn Trp Asn Lys Leu Val Gln Lys Phe Glu Leu Gly Ser Ser Ser
                860                 865                 870

Ile Ala His Met Val Met Gly Thr Thr Asn Gln Phe Ser Thr Arg
                875                 880                 885

Thr Arg Leu Glu Glu Val Lys Gly Phe Phe Ser Ser Leu Lys Glu
                890                 895                 900

Asn Gly Ser Gln Leu Arg Cys Val Gln Gln Thr Ile Glu Thr Ile
                905                 910                 915

Glu Glu Asn Ile Gly Trp Met Asp Lys Asn Phe Asp Lys Ile Arg
                920                 925                 930

Val Trp Leu Gln Ser Glu Lys Leu Glu Arg Met
                935                 940
```

<210> 465
<211> 1587
<212> DNA
<213> Homo Sapien

<400> 465

```
cagccacaga cgggtcatga gcgcggtatt actgctggcc ctcctggggt 50

tcatcctccc actgccagga gtgcaggcgc tgctctgcca gtttgggaca 100

gttcagcatg tgtggaaggt gtccgaccta ccccggcaat ggacccctaa 150

gaacaccagc tgcgacagcg gcttggggtg ccaggacacg ttgatgctca 200

ttgagagcgg accccaagtg agcctggtgc tctccaaggg ctgcacggag 250

gccaaggacc aggagccccg cgtcactgag caccggatgg gccccggcct 300

ctccctgatc tcctacacct tcgtgtgccg ccaggaggac ttctgcaaca 350

acctcgttaa ctccctcccg ctttgggccc cacagccccc agcagaccca 400

ggatccttga ggtgcccagt ctgcttgtct atggaaggct gtctggaggg 450

gacaacagaa gagatctgcc ccaaggggac cacacactgt tatgatggcc 500

tcctcaggct caggggagga ggcatcttct ccaatctgag agtccaggga 550

tgcatgcccc agccaggttg caacctgctc aatgggacac aggaaattgg 600

gcccgtgggt atgactgaga actgcaatag gaaagatttt ctgacctgtc 650
```

atcgggggac caccattatg acacacggaa acttggctca agaacccact 700

gattggacca catcgaatac cgagatgtgc gaggtggggc aggtgtgtca 750

ggagacgctg ctgctcatag atgtaggact cacatcaacc ctggtgggga 800

caaaaggctg cagcactgtt ggggctcaaa attcccagaa gaccaccatc 850

cactcagccc ctcctggggt gcttgtggcc tcctataccc acttctgctc 900

ctcggacctg tgcaatagtg ccagcagcag cagcgttctg ctgaactccc 950

tccctcctca agctgcccct gtcccaggag accggcagtg tcctacctgt 1000

gtgcagcccc ttggaacctg ttcaagtggc tccccccgaa tgacctgccc 1050

caggggcgcc actcattgtt atgatgggta cattcatctc tcaggaggtg 1100

ggctgtccac caaaatgagc attcagggct gcgtggccca accttccagc 1150

ttcttgttga accacaccag acaaatcggg atcttctctg cgcgtgagaa 1200

gcgtgatgtg cagcctcctg cctctcagca tgagggaggt ggggctgagg 1250

gcctggagtc tctcacttgg ggggtggggc tggcactggc cccagcgctg 1300

tggtggggag tggtttgccc ttcctgctaa ctctattacc cccacgattc 1350

ttcaccgctg ctgaccaccc acactcaacc tccctctgac ctcataacct 1400

aatggccttg gacaccagat tctttcccat tctgtccatg aatcatcttc 1450

cccacacaca atcattcata tctactcacc taacagcaac actggggaga 1500

gcctggagca tccggacttg ccctatggga gaggggacgc tggaggagtg 1550

gctgcatgta tctgataata cagaccctgt cctttca 1587

<210> 466
<211> 437
<212> PRT
<213> Homo Sapien

<400> 466
Met Ser Ala Val Leu Leu Leu Ala Leu Leu Gly Phe Ile Leu Pro
1 5 10 15

Leu Pro Gly Val Gln Ala Leu Leu Cys Gln Phe Gly Thr Val Gln
20 25 30

His Val Trp Lys Val Ser Asp Leu Pro Arg Gln Trp Thr Pro Lys
35 40 45

Asn Thr Ser Cys Asp Ser Gly Leu Gly Cys Gln Asp Thr Leu Met
50 55 60

Leu Ile Glu Ser Gly Pro Gln Val Ser Leu Val Leu Ser Lys Gly
65 70 75

Cys Thr Glu Ala Lys Asp Gln Glu Pro Arg Val Thr Glu His Arg
80 85 90

Met Gly Pro Gly Leu Ser Leu Ile Ser Tyr Thr Phe Val Cys Arg

```
                95                  100                 105

Gln Glu Asp Phe Cys Asn Asn Leu Val Asn Ser Leu Pro Leu Trp
                110                 115                 120

Ala Pro Gln Pro Pro Ala Asp Pro Gly Ser Leu Arg Cys Pro Val
                125                 130                 135

Cys Leu Ser Met Glu Gly Cys Leu Glu Gly Thr Thr Glu Glu Ile
                140                 145                 150

Cys Pro Lys Gly Thr Thr His Cys Tyr Asp Gly Leu Leu Arg Leu
                155                 160                 165

Arg Gly Gly Gly Ile Phe Ser Asn Leu Arg Val Gln Gly Cys Met
                170                 175                 180

Pro Gln Pro Gly Cys Asn Leu Leu Asn Gly Thr Gln Glu Ile Gly
                185                 190                 195

Pro Val Gly Met Thr Glu Asn Cys Asn Arg Lys Asp Phe Leu Thr
                200                 205                 210

Cys His Arg Gly Thr Thr Ile Met Thr His Gly Asn Leu Ala Gln
                215                 220                 225

Glu Pro Thr Asp Trp Thr Thr Ser Asn Thr Glu Met Cys Glu Val
                230                 235                 240

Gly Gln Val Cys Gln Glu Thr Leu Leu Leu Ile Asp Val Gly Leu
                245                 250                 255

Thr Ser Thr Leu Val Gly Thr Lys Gly Cys Ser Thr Val Gly Ala
                260                 265                 270

Gln Asn Ser Gln Lys Thr Thr Ile His Ser Ala Pro Pro Gly Val
                275                 280                 285

Leu Val Ala Ser Tyr Thr His Phe Cys Ser Ser Asp Leu Cys Asn
                290                 295                 300

Ser Ala Ser Ser Ser Ser Val Leu Leu Asn Ser Leu Pro Pro Gln
                305                 310                 315

Ala Ala Pro Val Pro Gly Asp Arg Gln Cys Pro Thr Cys Val Gln
                320                 325                 330

Pro Leu Gly Thr Cys Ser Ser Gly Ser Pro Arg Met Thr Cys Pro
                335                 340                 345

Arg Gly Ala Thr His Cys Tyr Asp Gly Tyr Ile His Leu Ser Gly
                350                 355                 360

Gly Gly Leu Ser Thr Lys Met Ser Ile Gln Gly Cys Val Ala Gln
                365                 370                 375

Pro Ser Ser Phe Leu Leu Asn His Thr Arg Gln Ile Gly Ile Phe
                380                 385                 390

Ser Ala Arg Glu Lys Arg Asp Val Gln Pro Pro Ala Ser Gln His
                395                 400                 405

Glu Gly Gly Gly Ala Glu Gly Leu Glu Ser Leu Thr Trp Gly Val
                410                 415                 420
```

Gly Leu Ala Leu Ala Pro Ala Leu Trp Trp Gly Val Val Cys Pro
425 430 435

Ser Cys

<210> 467
<211> 2475
<212> DNA
<213> Homo Sapien

<400> 467
gaggatttgc cacagcagcg gatagagcag gagagcacca ccggagccct 50

tgagacatcc ttgagaagag ccacagcata agagactgcc ctgcttggtg 100

ttttgcagga tgatggtggc ccttcgagga gcttctgcat tgctggttct 150

gttccttgca gcttttctgc ccccgccgca gtgtacccag gacccagcca 200

tggtgcatta catctaccag cgctttcgag tcttggagca agggctggaa 250

aaatgtaccc aagcaacgag ggcatacatt caagaattcc aagagttctc 300

aaaaaatata tctgtcatgc tgggaagatg tcagacctac acaagtgagt 350

acaagagtgc agtgggtaac ttggcactga gagttgaacg tgcccaacgg 400

gagattgact acatacaata ccttcgagag gctgacgagt gcatcgtatc 450

agaggacaag acactggcag aaatgttgct ccaagaagct gaagaagaga 500

aaaagatccg gactctgctg aatgcaagct gtgacaacat gctgatgggc 550

ataaagtctt tgaaaatagt gaagaagatg atggacacac atggctcttg 600

gatgaaagat gctgtctata actctccaaa ggtgtactta ttaattggat 650

ccagaaacaa cactgtttgg gaatttgcaa acatacgggc attcatggag 700

gataacacca agccagctcc ccggaagcaa atcctaacac tttcctggca 750

gggaacaggc caagtgatct acaaaggttt tctatttttt cataaccaag 800

caacttctaa tgagataatc aaatataacc tgcagaagag gactgtggaa 850

gatcgaatgc tgctcccagg aggggtaggc cgagcattgg tttaccagca 900

ctccccctca acttacattg acctggctgt ggatgagcat gggctctggg 950

ccatccactc tgggccaggc acccatagcc atttggttct cacaaagatt 1000

gagccgggca cactgggagt ggagcattca tgggataccc catgcagaag 1050

ccaggatgct gaagcctcat tcctcttgtg tggggttctc tatgtggtct 1100

acagtactgg gggccagggc cctcatcgca tcacctgcat ctatgatcca 1150

ctgggcacta tcagtgagga ggacttgccc aacttgttct tccccaagag 1200

accaagaagt cactccatga tccattacaa ccccagagat aagcagctct 1250

atgcctggaa tgaaggaaac cagatcattt acaaactcca gacaaagaga 1300

aagctgcctc tgaagtaatg cattacagct gtgagaaaga gcactgtggc 1350

tttggcagct gttctacagg acagtgaggc tatagcccct tcacaatata 1400

gtatccctct aatcacacac aggaagagtg tgtagaagtg gaaatacgta 1450

tgcctccttt cccaaatgtc actgccttag gtatcttcca agagcttaga 1500

tgagagcata tcatcaggaa agtttcaaca atgtccatta ctcccccaaa 1550

cctcctggct ctcaaggatg accacattct gatacagcct acttcaagcc 1600

ttttgtttta ctgctcccca gcatttactg taactctgcc atcttccctc 1650

ccacaattag agttgtatgc cagcccctaa tattcaccac tggcttttct 1700

ctcccctggc ctttgctgaa gctcttccct ctttttcaaa tgtctattga 1750

tattctccca ttttcactgc ccaactaaaa tactattaat atttctttct 1800

tttcttttct ttttttgag acaaggtctc actatgttgc ccaggctggt 1850

ctcaaactcc agagctcaag agatcctcct gcctcagcct cctaagtacc 1900

tgggattaca ggcatgtgcc accacacctg gcttaaaata ctatttctta 1950

ttgaggttta acctctattt cccctagccc tgtccttcca ctaagcttgg 2000

tagatgtaat aataaagtga aaatattaac atttgaatat cgctttccag 2050

gtgtggagtg tttgcacatc attgaattct cgtttcacct ttgtgaaaca 2100

tgcacaagtc tttacagctg tcattctaga gtttaggtga gtaacacaat 2150

tacaaagtga aagatacagc tagaaaatac tacaaatccc atagtttttc 2200

cattgcccaa ggaagcatca aatacgtatg tttgttcacc tactcttata 2250

gtcaatgcgt tcatcgtttc agcctaaaaa taatagtctg tccctttagc 2300

cagttttcat gtctgcacaa gacctttcaa taggcctttc aaatgataat 2350

tcctccagaa aaccagtcta agggtgagga ccccaactct agcctcctct 2400

tgtcttgctg tcctctgttt ctctctttct gctttaaatt caataaaagt 2450

gacactgagc aaaaaaaaaa aaaaa 2475

<210> 468
<211> 402
<212> PRT
<213> Homo Sapien

<400> 468
Met Met Val Ala Leu Arg Gly Ala Ser Ala Leu Leu Val Leu Phe
1 5 10 15

Leu Ala Ala Phe Leu Pro Pro Pro Gln Cys Thr Gln Asp Pro Ala
20 25 30

Met Val His Tyr Ile Tyr Gln Arg Phe Arg Val Leu Glu Gln Gly
35 40 45

```
Leu Glu Lys Cys Thr Gln Ala Thr Arg Ala Tyr Ile Gln Glu Phe
                50                  55                  60

Gln Glu Phe Ser Lys Asn Ile Ser Val Met Leu Gly Arg Cys Gln
                65                  70                  75

Thr Tyr Thr Ser Glu Tyr Lys Ser Ala Val Gly Asn Leu Ala Leu
                80                  85                  90

Arg Val Glu Arg Ala Gln Arg Glu Ile Asp Tyr Ile Gln Tyr Leu
                95                  100                 105

Arg Glu Ala Asp Glu Cys Ile Val Ser Glu Asp Lys Thr Leu Ala
                110                 115                 120

Glu Met Leu Leu Gln Glu Ala Glu Glu Glu Lys Lys Ile Arg Thr
                125                 130                 135

Leu Leu Asn Ala Ser Cys Asp Asn Met Leu Met Gly Ile Lys Ser
                140                 145                 150

Leu Lys Ile Val Lys Lys Met Met Asp Thr His Gly Ser Trp Met
                155                 160                 165

Lys Asp Ala Val Tyr Asn Ser Pro Lys Val Tyr Leu Leu Ile Gly
                170                 175                 180

Ser Arg Asn Asn Thr Val Trp Glu Phe Ala Asn Ile Arg Ala Phe
                185                 190                 195

Met Glu Asp Asn Thr Lys Pro Ala Pro Arg Lys Gln Ile Leu Thr
                200                 205                 210

Leu Ser Trp Gln Gly Thr Gly Gln Val Ile Tyr Lys Gly Phe Leu
                215                 220                 225

Phe Phe His Asn Gln Ala Thr Ser Asn Glu Ile Ile Lys Tyr Asn
                230                 235                 240

Leu Gln Lys Arg Thr Val Glu Asp Arg Met Leu Leu Pro Gly Gly
                245                 250                 255

Val Gly Arg Ala Leu Val Tyr Gln His Ser Pro Ser Thr Tyr Ile
                260                 265                 270

Asp Leu Ala Val Asp Glu His Gly Leu Trp Ala Ile His Ser Gly
                275                 280                 285

Pro Gly Thr His Ser His Leu Val Leu Thr Lys Ile Glu Pro Gly
                290                 295                 300

Thr Leu Gly Val Glu His Ser Trp Asp Thr Pro Cys Arg Ser Gln
                305                 310                 315

Asp Ala Glu Ala Ser Phe Leu Leu Cys Gly Val Leu Tyr Val Val
                320                 325                 330

Tyr Ser Thr Gly Gly Gln Gly Pro His Arg Ile Thr Cys Ile Tyr
                335                 340                 345

Asp Pro Leu Gly Thr Ile Ser Glu Glu Asp Leu Pro Asn Leu Phe
                350                 355                 360

Phe Pro Lys Arg Pro Arg Ser His Ser Met Ile His Tyr Asn Pro
```

365 370 375

Arg Asp Lys Gln Leu Tyr Ala Trp Asn Glu Gly Asn Gln Ile Ile
380 385 390

Tyr Lys Leu Gln Thr Lys Arg Lys Leu Pro Leu Lys
395 400

<210> 469
<211> 1415
<212> DNA
<213> Homo Sapien

<400> 469
tggcctcccc agcttgccag gcacaaggct gagcgggagg aagcgagagg 50

catctaagca ggcagtgttt tgccttcacc ccaagtgacc atgagaggtg 100

ccacgcgagt ctcaatcatg ctcctcctag taactgtgtc tgactgtgct 150

gtgatcacag gggcctgtga gcgggatgtc cagtgtgggg caggcacctg 200

ctgtgccatc agcctgtggc ttcgagggct gcggatgtgc accccgctgg 250

ggcgggaagg cgaggagtgc caccccggca gccacaaggt ccccttcttc 300

aggaaacgca agcaccacac ctgtccttgc ttgcccaacc tgctgtgctc 350

caggttcccg gacggcaggt accgctgctc catggacttg aagaacatca 400

atttttaggc gcttgcctgg tctcaggata cccaccatcc ttttcctgag 450

cacagcctgg atttttattt ctgccatgaa acccagctcc catgactctc 500

ccagtcccta cactgactac cctgatctct cttgtctagt acgcacatat 550

gcacacaggc agacatacct cccatcatga catggtcccc aggctggcct 600

gaggatgtca cagcttgagg ctgtggtgtg aaaggtggcc agcctggttc 650

tcttccctgc tcaggctgcc agagaggtgg taaatggcag aaaggacatt 700

ccccctcccc tccccaggtg acctgctctc tttcctgggc cctgcccctc 750

tccccacatg tatccctcgg tctgaattag acattcctgg gcacaggctc 800

ttgggtgcat tgctcagagt cccaggtcct ggcctgaccc tcaggccctt 850

cacgtgaggt ctgtgaggac caatttgtgg gtagttcatc ttccctcgat 900

tggttaactc cttagtttca gaccacagac tcaagattgg ctcttcccag 950

agggcagcag acagtcaccc caaggcaggt gtagggagcc cagggaggcc 1000

aatcagcccc ctgaagactc tggtcccagt cagcctgtgg cttgtggcct 1050

gtgacctgtg accttctgcc agaattgtca tgcctctgag gcccccctctt 1100

accacacttt accagttaac cactgaagcc cccaattccc acagcttttc 1150

cattaaaatg caaatggtgg tggttcaatc taatctgata ttgacatatt 1200

agaaggcaat tagggtgttt ccttaaacaa ctcctttcca aggatcagcc 1250

ctgagagcag gttggtgact ttgaggaggg cagtcctctg tccagattgg 1300

ggtgggagca agggacaggg agcagggcag gggctgaaag gggcactgat 1350

tcagaccagg gaggcaacta cacaccaaca tgctggcttt agaataaaag 1400

caccaactga aaaaa 1415

<210> 470
<211> 105
<212> PRT
<213> Homo Sapien

<400> 470
Met Arg Gly Ala Thr Arg Val Ser Ile Met Leu Leu Leu Val Thr
1 5 10 15

Val Ser Asp Cys Ala Val Ile Thr Gly Ala Cys Glu Arg Asp Val
20 25 30

Gln Cys Gly Ala Gly Thr Cys Cys Ala Ile Ser Leu Trp Leu Arg
35 40 45

Gly Leu Arg Met Cys Thr Pro Leu Gly Arg Glu Gly Glu Glu Cys
50 55 60

His Pro Gly Ser His Lys Val Pro Phe Phe Arg Lys Arg Lys His
65 70 75

His Thr Cys Pro Cys Leu Pro Asn Leu Leu Cys Ser Arg Phe Pro
80 85 90

Asp Gly Arg Tyr Arg Cys Ser Met Asp Leu Lys Asn Ile Asn Phe
95 100 105

<210> 471
<211> 1281
<212> DNA
<213> Homo Sapien

<400> 471
agcgcccggg cgtcggggcg gtaaaaggcc ggcagaaggg aggcacttga 50

gaaatgtctt tcctccagga cccaagtttc ttcaccatgg ggatgtggtc 100

cattggtgca ggagccctgg ggctgctgc cttggcattg ctgcttgcca 150

acacagacgt gtttctgtcc aagccccaga aagcggccct ggagtacctg 200

gaggatatag acctgaaaac actggagaag gaaccaagga ctttcaaagc 250

aaaggagcta tgggaaaaaa atggagctgt gattatggcc gtgcggaggc 300

caggctgttt cctctgtcga gaggaagctg cggatctgtc ctccctgaaa 350

agcatgttgg accagctggg cgtcccccctc tatgcagtgg taaaggagca 400

catcaggact gaagtgaagg atttccagcc ttatttcaaa ggagaaatct 450

tcctggatga aaagaaaaag ttctatggtc cacaaaggcg gaagatgatg 500

tttatgggat ttatccgtct gggagtgtgg tacaacttct tccgagcctg 550

gaacggaggc ttctctggaa acctggaagg agaaggcttc atccttgggg 600

gagttttcgt ggtgggatca ggaaagcagg gcattcttct tgagcaccga 650
gaaaaagaat ttggagacaa agtaaaccta ctttctgttc tggaagctgc 700
taagatgatc aaaccacaga ctttggcctc agagaaaaaa tgattgtgtg 750
aaactgccca gctcagggat aaccagggac attcacctgt gttcatggga 800
tgtattgttt ccactcgtgt ccctaaggag tgagaaaccc atttatactc 850
tactctcagt atggattatt aatgtatttt aatattctgt ttaggcccac 900
taaggcaaaa tagccccaaa acaagactga caaaaatctg aaaaactaat 950
gaggattatt aagctaaaac ctgggaaata ggaggcttaa aattgactgc 1000
caggctgggt gcagtggctc acacctgtaa tcccagcact ttgggaggcc 1050
aaggtgagca agtcacttga ggtcgggagt tcgagaccag cctgagcaac 1100
atggcgaaac cccgtctcta ctaaaaatac aaaaatcacc cgggtgtggt 1150
ggcaggcacc tgtagtccca gctacccggg aggctgaggc aggagaatca 1200
cttgaacctg ggaggtggag gttgcggtga gctgagatca caccactgta 1250
ttccagcctg ggtgactgag actctaacta a 1281

<210> 472
<211> 229
<212> PRT
<213> Homo Sapien

<400> 472
Met Ser Phe Leu Gln Asp Pro Ser Phe Phe Thr Met Gly Met Trp
1 5 10 15

Ser Ile Gly Ala Gly Ala Leu Gly Ala Ala Ala Leu Ala Leu Leu
20 25 30

Leu Ala Asn Thr Asp Val Phe Leu Ser Lys Pro Gln Lys Ala Ala
35 40 45

Leu Glu Tyr Leu Glu Asp Ile Asp Leu Lys Thr Leu Glu Lys Glu
50 55 60

Pro Arg Thr Phe Lys Ala Lys Glu Leu Trp Glu Lys Asn Gly Ala
65 70 75

Val Ile Met Ala Val Arg Arg Pro Gly Cys Phe Leu Cys Arg Glu
80 85 90

Glu Ala Ala Asp Leu Ser Ser Leu Lys Ser Met Leu Asp Gln Leu
95 100 105

Gly Val Pro Leu Tyr Ala Val Val Lys Glu His Ile Arg Thr Glu
110 115 120

Val Lys Asp Phe Gln Pro Tyr Phe Lys Gly Glu Ile Phe Leu Asp
125 130 135

Glu Lys Lys Lys Phe Tyr Gly Pro Gln Arg Arg Lys Met Met Phe
140 145 150

```
Met Gly Phe Ile Arg Leu Gly Val Trp Tyr Asn Phe Phe Arg Ala
                155                 160                 165

Trp Asn Gly Gly Phe Ser Gly Asn Leu Glu Gly Glu Gly Phe Ile
                170                 175                 180

Leu Gly Gly Val Phe Val Val Gly Ser Gly Lys Gln Gly Ile Leu
                185                 190                 195

Leu Glu His Arg Glu Lys Glu Phe Gly Asp Lys Val Asn Leu Leu
                200                 205                 210

Ser Val Leu Glu Ala Ala Lys Met Ile Lys Pro Gln Thr Leu Ala
                215                 220                 225

Ser Glu Lys Lys


<210> 473
<211> 713
<212> DNA
<213> Homo Sapien

<400> 473
aatatatcat ctatttatca ttaatcaata atgtattctt ttattccaat 50

aacatttggg ttttgggatt ttaattttca aacacagcag aatgacattt 100

tttctgtcac tattattatt gttggtatgt gaagctattt ggagatccaa 150

ttcaggaagc aacacattgg agaatggcta ctttctatca agaaataaag 200

agaaccacag tcaacccaca caatcatctt tagaagacag tgtgactcct 250

accaaagctg tcaaaaccac aggcaagggc atagttaaag gacggaatct 300

tgactcaaga gggttaattc ttggtgctga agcctggggc aggggtgtaa 350

agaaaaacac ttagattcaa tgattgtaaa tttaaggcaa atacacatat 400

tagtattacc ttagtgtaat gtatccctgt catatataca ataaggtgaa 450

attataagta ccctatgcag ttggctggac agttctaaat tggactttat 500

taatttttaa aatcagtaac tgatttatca ctggctatgt gcttagatct 550

acaggagatc atataatttg atacaaataa aagaaaagtg ttctctcccc 600

ttacagaatt gacattttaa atgcgataca gttagaatag gaaatatgac 650

attagaaagg aagaatgaca gggagaaagg aaagaaggga aaatgttgcc 700

aaggaaaaaa aaa 713

<210> 474
<211> 90
<212> PRT
<213> Homo Sapien

<400> 474
Met Thr Phe Phe Leu Ser Leu Leu Leu Leu Leu Val Cys Glu Ala
  1               5                  10                  15
```

Ile Trp Arg Ser Asn Ser Gly Ser Asn Thr Leu Glu Asn Gly Tyr
20 25 30

Phe Leu Ser Arg Asn Lys Glu Asn His Ser Gln Pro Thr Gln Ser
35 40 45

Ser Leu Glu Asp Ser Val Thr Pro Thr Lys Ala Val Lys Thr Thr
50 55 60

Gly Lys Gly Ile Val Lys Gly Arg Asn Leu Asp Ser Arg Gly Leu
65 70 75

Ile Leu Gly Ala Glu Ala Trp Gly Arg Gly Val Lys Lys Asn Thr
80 85 90

<210> 475
<211> 1844
<212> DNA
<213> Homo Sapien

<400> 475
gacagtggag ggcagtggag aggaccgcgc tgtcctgctg tcaccaagag 50

ctggagacac catctcccac cgagagtcat ggccccattg gccctgcacc 100

tcctcgtcct cgtccccatc ctcctcagcc tggtggcctc ccaggactgg 150

aaggctgaac gcagccaaga ccccttcgag aaatgcatgc aggatcctga 200

ctatgagcag ctgctcaagg tggtgacctg gggctcaat cggaccctga 250

agccccagag ggtgattgtg gttggcgctg gtgtggccgg gctggtggcc 300

gccaaggtgc tcagcgatgc tggacacaag gtcaccatcc tggaggcaga 350

taacaggatc gggggccgca tcttcaccta ccgggaccag aacacgggct 400

ggattgggga gctgggagcc atgcgcatgc ccagctctca caggatcctc 450

cacaagctct gccagggcct ggggctcaac ctgaccaagt tcacccagta 500

cgacaagaac acgtggacgg aggtgcacga agtgaagctg cgcaactatg 550

tggtggagaa ggtgcccgag aagctgggct acgccttgcg tccccaggaa 600

aagggccact cgcccgaaga catctaccag atggctctca accaggccct 650

caaagacctc aaggcactgg gctgcagaaa ggcgatgaag aagtttgaaa 700

ggcacacgct cttggaatat cttctcgggg aggggaacct gagccggccg 750

gccgtgcagc ttctgggaga cgtgatgtcc gaggatggct tcttctatct 800

cagcttcgcc gaggccctcc gggcccacag ctgcctcagc gacagactcc 850

agtacagccg catcgtgggt ggctgggacc tgctgccgcg cgcgctgctg 900

agctcgctgt ccgggcttgt gctgttgaac gcgcccgtgg tggcgatgac 950

ccagggaccg cacgatgtgc acgtgcagat cgagacctct cccccggcgc 1000

ggaatctgaa ggtgctgaag gccgacgtgg tgctgctgac ggcgagcgga 1050

ccggcggtga agcgcatcac cttctcgccg ccgctgcccc gccacatgca 1100

ggaggcgctg cggaggctgc actacgtgcc ggccaccaag gtgttcctaa 1150

gcttccgcag gcccttctgg cgcgaggagc acattgaagg cggccactca 1200

aacaccgatc gcccgtcgcg catgattttc tacccgccgc cgcgcgaggg 1250

cgcgctgctg ctggcctcgt acacgtggtc ggacgcggcg gcagcgttcg 1300

ccggcttgag ccgggaagag gcgttgcgct tggcgctcga cgacgtggcg 1350

gcattgcacg ggcctgtcgt gcgccagctc tgggacggca ccggcgtcgt 1400

caagcgttgg gcggaggacc agcacagcca gggtggcttt gtggtacagc 1450

cgccggcgct ctggcaaacc gaaaaggatg actggacggt cccttatggc 1500

cgcatctact ttgccggcga gcacaccgcc tacccgcacg gctgggtgga 1550

gacggcggtc aagtcggcgc tgcgcgccgc catcaagatc aacagccgga 1600

aggggcctgc atcggacacg gccagccccg aggggcacgc atctgacatg 1650

gaggggcagg ggcatgtgca tggggtggcc agcagcccct cgcatgacct 1700

ggcaaaggaa gaaggcagcc accctccagt ccaaggccag ttatctctcc 1750

aaaacacgac ccacacgagg acctcgcatt aaagtatttt cggaaaaaaa 1800

aaaaaaaaaa aaaaaaaaaa aaaaaaaaaa aaaaaaaaaa aaaa 1844

<210> 476
<211> 567
<212> PRT
<213> Homo Sapien

<400> 476
Met Ala Pro Leu Ala Leu His Leu Leu Val Leu Val Pro Ile Leu
1 5 10 15

Leu Ser Leu Val Ala Ser Gln Asp Trp Lys Ala Glu Arg Ser Gln
20 25 30

Asp Pro Phe Glu Lys Cys Met Gln Asp Pro Asp Tyr Glu Gln Leu
35 40 45

Leu Lys Val Val Thr Trp Gly Leu Asn Arg Thr Leu Lys Pro Gln
50 55 60

Arg Val Ile Val Val Gly Ala Gly Val Ala Gly Leu Val Ala Ala
65 70 75

Lys Val Leu Ser Asp Ala Gly His Lys Val Thr Ile Leu Glu Ala
80 85 90

Asp Asn Arg Ile Gly Gly Arg Ile Phe Thr Tyr Arg Asp Gln Asn
95 100 105

Thr Gly Trp Ile Gly Glu Leu Gly Ala Met Arg Met Pro Ser Ser
110 115 120

His Arg Ile Leu His Lys Leu Cys Gln Gly Leu Gly Leu Asn Leu
125 130 135

```
Thr Lys Phe Thr Gln Tyr Asp Lys Asn Thr Trp Thr Glu Val His
                140                 145                 150

Glu Val Lys Leu Arg Asn Tyr Val Val Glu Lys Val Pro Glu Lys
                155                 160                 165

Leu Gly Tyr Ala Leu Arg Pro Gln Glu Lys Gly His Ser Pro Glu
                170                 175                 180

Asp Ile Tyr Gln Met Ala Leu Asn Gln Ala Leu Lys Asp Leu Lys
                185                 190                 195

Ala Leu Gly Cys Arg Lys Ala Met Lys Lys Phe Glu Arg His Thr
                200                 205                 210

Leu Leu Glu Tyr Leu Leu Gly Glu Gly Asn Leu Ser Arg Pro Ala
                215                 220                 225

Val Gln Leu Leu Gly Asp Val Met Ser Glu Asp Gly Phe Phe Tyr
                230                 235                 240

Leu Ser Phe Ala Glu Ala Leu Arg Ala His Ser Cys Leu Ser Asp
                245                 250                 255

Arg Leu Gln Tyr Ser Arg Ile Val Gly Gly Trp Asp Leu Leu Pro
                260                 265                 270

Arg Ala Leu Leu Ser Ser Leu Ser Gly Leu Val Leu Leu Asn Ala
                275                 280                 285

Pro Val Val Ala Met Thr Gln Gly Pro His Asp Val His Val Gln
                290                 295                 300

Ile Glu Thr Ser Pro Pro Ala Arg Asn Leu Lys Val Leu Lys Ala
                305                 310                 315

Asp Val Val Leu Leu Thr Ala Ser Gly Pro Ala Val Lys Arg Ile
                320                 325                 330

Thr Phe Ser Pro Pro Leu Pro Arg His Met Gln Glu Ala Leu Arg
                335                 340                 345

Arg Leu His Tyr Val Pro Ala Thr Lys Val Phe Leu Ser Phe Arg
                350                 355                 360

Arg Pro Phe Trp Arg Glu Glu His Ile Glu Gly Gly His Ser Asn
                365                 370                 375

Thr Asp Arg Pro Ser Arg Met Ile Phe Tyr Pro Pro Pro Arg Glu
                380                 385                 390

Gly Ala Leu Leu Leu Ala Ser Tyr Thr Trp Ser Asp Ala Ala Ala
                395                 400                 405

Ala Phe Ala Gly Leu Ser Arg Glu Glu Ala Leu Arg Leu Ala Leu
                410                 415                 420

Asp Asp Val Ala Ala Leu His Gly Pro Val Val Arg Gln Leu Trp
                425                 430                 435

Asp Gly Thr Gly Val Val Lys Arg Trp Ala Glu Asp Gln His Ser
                440                 445                 450

Gln Gly Gly Phe Val Val Gln Pro Pro Ala Leu Trp Gln Thr Glu
```

455 460 465

Lys Asp Asp Trp Thr Val Pro Tyr Gly Arg Ile Tyr Phe Ala Gly
470 475 480

Glu His Thr Ala Tyr Pro His Gly Trp Val Glu Thr Ala Val Lys
485 490 495

Ser Ala Leu Arg Ala Ala Ile Lys Ile Asn Ser Arg Lys Gly Pro
500 505 510

Ala Ser Asp Thr Ala Ser Pro Glu Gly His Ala Ser Asp Met Glu
515 520 525

Gly Gln Gly His Val His Gly Val Ala Ser Ser Pro Ser His Asp
530 535 540

Leu Ala Lys Glu Glu Gly Ser His Pro Pro Val Gln Gly Gln Leu
545 550 555

Ser Leu Gln Asn Thr Thr His Thr Arg Thr Ser His
560 565

<210> 477
<211> 3316
<212> DNA
<213> Homo Sapien

<400> 477
ctgacatggc ctgactcggg acagctcaga gcagggcaga actggggaca 50

ctctgggccg gccttctgcc tgcatggacg ctctgaagcc accctgtctc 100

tggaggaacc acgagcgagg gaagaaggac agggactcgt gtggcaggaa 150

gaactcagag ccgggaagcc cccattcact agaagcactg agagatgcgg 200

ccccctcgca gggtctgaat ttcctgctgc tgttcacaaa gatgcttttt 250

atctttaact ttttgttttc cccacttccg accccggcgt tgatctgcat 300

cctgacattt ggagctgcca tcttcttgtg gctgatcacc agacctcaac 350

ccgtcttacc tcttcttgac ctgaacaatc agtctgtggg aattgaggga 400

ggagcacgga aggggtttc ccagaagaac aatgacctaa caagttgctg 450

cttctcagat gccaagacta tgtatgaggt tttccaaaga ggactcgctg 500

tgtctgacaa tgggccctgc ttgggatata gaaaaccaaa ccagccctac 550

agatggctat cttacaaaca ggtgtctgat agagcagagt acctgggttc 600

ctgtctcttg cataaaggtt ataaatcatc accagaccag tttgtcggca 650

tctttgctca gaataggcca gagtggatca tctccgaatt ggcttgttac 700

acgtactcta tggtagctgt acctctgtat gacaccttgg gaccagaagc 750

catcgtacat attgtcaaca aggctgatat cgccatggtg atctgtgaca 800

caccccaaaa ggcattggtg ctgataggga atgtagagaa aggcttcacc 850

ccgagcctga aggtgatcat ccttatggac ccctttgatg atgacctgaa 900

gcaaagaggg gagaagagtg gaattgagat cttatcccta tatgatgctg 950
agaacctagg caaagagcac ttcagaaaac ctgtgcctcc tagcccagaa 1000
gacctgagcg tcatctgctt caccagtggg accacaggtg accccaaagg 1050
agccatgata acccatcaaa atattgtttc aaatgctgct gcctttctca 1100
aatgtgtgga gcatgcttat gagcccactc ctgatgatgt ggccatatcc 1150
tacctccctc tggctcatat gtttgagagg attgtacagg ctgttgtgta 1200
cagctgtgga gccagagttg gattcttcca aggggatatt cggttgctgg 1250
ctgacgacat gaagactttg aagcccacat tgtttcccgc ggtgcctcga 1300
ctccttaaca ggatctacga taaggtacaa aatgaggcca agacaccctt 1350
gaagaagttc ttgttgaagc tggctgtttc cagtaaattc aaagagcttc 1400
aaaagggtat catcaggcat gatagtttct gggacaagct catctttgca 1450
aagatccagg acagcctggg cggaagggtt cgtgtaattg tcactggagc 1500
tgcccccatg tccacttcag tcatgacatt cttccgggca gcaatgggat 1550
gtcaggtgta tgaagcttat ggtcaaacag aatgcacagg tggctgtaca 1600
tttacattac ctggggactg gacatcaggt cacgttgggg tgcccctggc 1650
ttgcaattac gtgaagctgg aagatgtggc tgacatgaac tactttacag 1700
tgaataatga aggagaggtc tgcatcaagg gtacaaacgt gttcaaagga 1750
tacctgaagg accctgagaa gacacaggaa gccctggaca gtgatggctg 1800
gcttcacaca ggagacattg gtcgctggct cccgaatgga actctgaaga 1850
tcatcgaccg taaaaagaac attttcaagc tggcccaagg agaatacatt 1900
gcaccagaga agatagaaaa tatctacaac aggagtcaac cagtgttaca 1950
aatttttgta cacggggaga gcttacggtc atccttagta ggagtggtgg 2000
ttcctgacac agatgtactt ccctcatttg cagccaagct tggggtgaag 2050
ggctcctttg aggaactgtg ccaaaaccaa gttgtaaggg aagccatttt 2100
agaagacttg cagaaaattg ggaaagaaag tggccttaaa acttttgaac 2150
aggtcaaagc cattttttctt catccagagc cattttccat tgaaaatggg 2200
ctcttgacac caacattgaa agcaaagcga ggagagcttt ccaaatactt 2250
tcggacccaa attgacagcc tgtatgagca catccaggat taggataagg 2300
tacttaagta cctgccggcc cactgtgcac tgcttgtgag aaaatggatt 2350
aaaaactatt cttacatttg ttttgccttt cctcctattt ttttttaacc 2400
tgttaaactc taaagccata gcttttgttt tatattgaga catataatgt 2450
gtaaacttag ttcccaaata aatcaatcct gtctttccca tcttcgatgt 2500

tgctaatatt aaggcttcag ggctactttt atcaacatgc ctgtcttcaa 2550

gatcccagtt tatgttctgt gtccttcctc atgatttcca accttaatac 2600

tattagtaac cacaagttca agggtcaaag ggaccctctg tgccttcttc 2650

tttgttttgt gataaacata acttgccaac agtctctatg cttatttaca 2700

tcttctactg ttcaaactaa gagattttta aattctgaaa aactgcttac 2750

aattcatgtt ttctagccac tccacaaacc actaaaattt tagttttagc 2800

ctatcactca tgtcaatcat atctatgaga caaatgtctc cgatgctctt 2850

ctgcgtaaat taaattgtgt actgaaggga aaagtttgat cataccaaac 2900

atttcctaaa ctctctagtt agatatctga cttgggagta ttaaaaattg 2950

ggtctatgac atactgtcca aaaggaatgc tgttcttaaa gcattattta 3000

cagtaggaac tggggagtaa atctgttccc tacagtttgc tgctgagctg 3050

gaagctgtgg gggaaggagt tgacaggtgg gcccagtgaa cttttccagt 3100

aaatgaagca agcactgaat aaaaacctcc tgaactggga acaaagatct 3150

acaggcaagc aagatgccca cacaacaggc ttattttctg tgaaggaacc 3200

aactgatctc ccccacccttggattagagt tcctgctcta ccttacccac 3250

agataacaca tgttgtttct acttgtaaat gtaaagtctt taaaataaac 3300

tattacagat aaaaaa 3316

<210> 478
<211> 739
<212> PRT
<213> Homo Sapien

<400> 478
Met Asp Ala Leu Lys Pro Pro Cys Leu Trp Arg Asn His Glu Arg
1 5 10 15

Gly Lys Lys Asp Arg Asp Ser Cys Gly Arg Lys Asn Ser Glu Pro
20 25 30

Gly Ser Pro His Ser Leu Glu Ala Leu Arg Asp Ala Ala Pro Ser
35 40 45

Gln Gly Leu Asn Phe Leu Leu Leu Phe Thr Lys Met Leu Phe Ile
50 55 60

Phe Asn Phe Leu Phe Ser Pro Leu Pro Thr Pro Ala Leu Ile Cys
65 70 75

Ile Leu Thr Phe Gly Ala Ala Ile Phe Leu Trp Leu Ile Thr Arg
80 85 90

Pro Gln Pro Val Leu Pro Leu Leu Asp Leu Asn Asn Gln Ser Val
95 100 105

Gly Ile Glu Gly Gly Ala Arg Lys Gly Val Ser Gln Lys Asn Asn
110 115 120

```
Asp Leu Thr Ser Cys Cys Phe Ser Asp Ala Lys Thr Met Tyr Glu
                125                 130                 135

Val Phe Gln Arg Gly Leu Ala Val Ser Asp Asn Gly Pro Cys Leu
                140                 145                 150

Gly Tyr Arg Lys Pro Asn Gln Pro Tyr Arg Trp Leu Ser Tyr Lys
                155                 160                 165

Gln Val Ser Asp Arg Ala Glu Tyr Leu Gly Ser Cys Leu Leu His
                170                 175                 180

Lys Gly Tyr Lys Ser Ser Pro Asp Gln Phe Val Gly Ile Phe Ala
                185                 190                 195

Gln Asn Arg Pro Glu Trp Ile Ile Ser Glu Leu Ala Cys Tyr Thr
                200                 205                 210

Tyr Ser Met Val Ala Val Pro Leu Tyr Asp Thr Leu Gly Pro Glu
                215                 220                 225

Ala Ile Val His Ile Val Asn Lys Ala Asp Ile Ala Met Val Ile
                230                 235                 240

Cys Asp Thr Pro Gln Lys Ala Leu Val Leu Ile Gly Asn Val Glu
                245                 250                 255

Lys Gly Phe Thr Pro Ser Leu Lys Val Ile Ile Leu Met Asp Pro
                260                 265                 270

Phe Asp Asp Asp Leu Lys Gln Arg Gly Glu Lys Ser Gly Ile Glu
                275                 280                 285

Ile Leu Ser Leu Tyr Asp Ala Glu Asn Leu Gly Lys Glu His Phe
                290                 295                 300

Arg Lys Pro Val Pro Pro Ser Pro Glu Asp Leu Ser Val Ile Cys
                305                 310                 315

Phe Thr Ser Gly Thr Thr Gly Asp Pro Lys Gly Ala Met Ile Thr
                320                 325                 330

His Gln Asn Ile Val Ser Asn Ala Ala Ala Phe Leu Lys Cys Val
                335                 340                 345

Glu His Ala Tyr Glu Pro Thr Pro Asp Asp Val Ala Ile Ser Tyr
                350                 355                 360

Leu Pro Leu Ala His Met Phe Glu Arg Ile Val Gln Ala Val Val
                365                 370                 375

Tyr Ser Cys Gly Ala Arg Val Gly Phe Phe Gln Gly Asp Ile Arg
                380                 385                 390

Leu Leu Ala Asp Asp Met Lys Thr Leu Lys Pro Thr Leu Phe Pro
                395                 400                 405

Ala Val Pro Arg Leu Leu Asn Arg Ile Tyr Asp Lys Val Gln Asn
                410                 415                 420

Glu Ala Lys Thr Pro Leu Lys Lys Phe Leu Leu Lys Leu Ala Val
                425                 430                 435
```

```
Ser Ser Lys Phe Lys Glu Leu Gln Lys Gly Ile Ile Arg His Asp
                440                 445                 450

Ser Phe Trp Asp Lys Leu Ile Phe Ala Lys Ile Gln Asp Ser Leu
                455                 460                 465

Gly Gly Arg Val Arg Val Ile Val Thr Gly Ala Ala Pro Met Ser
                470                 475                 480

Thr Ser Val Met Thr Phe Phe Arg Ala Ala Met Gly Cys Gln Val
                485                 490                 495

Tyr Glu Ala Tyr Gly Gln Thr Glu Cys Thr Gly Gly Cys Thr Phe
                500                 505                 510

Thr Leu Pro Gly Asp Trp Thr Ser Gly His Val Gly Val Pro Leu
                515                 520                 525

Ala Cys Asn Tyr Val Lys Leu Glu Asp Val Ala Asp Met Asn Tyr
                530                 535                 540

Phe Thr Val Asn Asn Glu Gly Glu Val Cys Ile Lys Gly Thr Asn
                545                 550                 555

Val Phe Lys Gly Tyr Leu Lys Asp Pro Glu Lys Thr Gln Glu Ala
                560                 565                 570

Leu Asp Ser Asp Gly Trp Leu His Thr Gly Asp Ile Gly Arg Trp
                575                 580                 585

Leu Pro Asn Gly Thr Leu Lys Ile Ile Asp Arg Lys Lys Asn Ile
                590                 595                 600

Phe Lys Leu Ala Gln Gly Glu Tyr Ile Ala Pro Glu Lys Ile Glu
                605                 610                 615

Asn Ile Tyr Asn Arg Ser Gln Pro Val Leu Gln Ile Phe Val His
                620                 625                 630

Gly Glu Ser Leu Arg Ser Ser Leu Val Gly Val Val Val Pro Asp
                635                 640                 645

Thr Asp Val Leu Pro Ser Phe Ala Ala Lys Leu Gly Val Lys Gly
                650                 655                 660

Ser Phe Glu Glu Leu Cys Gln Asn Gln Val Val Arg Glu Ala Ile
                665                 670                 675

Leu Glu Asp Leu Gln Lys Ile Gly Lys Glu Ser Gly Leu Lys Thr
                680                 685                 690

Phe Glu Gln Val Lys Ala Ile Phe Leu His Pro Glu Pro Phe Ser
                695                 700                 705

Ile Glu Asn Gly Leu Leu Thr Pro Thr Leu Lys Ala Lys Arg Gly
                710                 715                 720

Glu Leu Ser Lys Tyr Phe Arg Thr Gln Ile Asp Ser Leu Tyr Glu
                725                 730                 735

His Ile Gln Asp
```

<210> 479

<211> 2725
<212> DNA
<213> Homo Sapien

<400> 479
ggaggcggag gccgcggcga gccgggccga gcagtgaggg ccctagcggg 50

gcccgagcgg ggcccggggc ccctaagcca ttcctgaagt catgggctgg 100

ccaggacatt ggtgacccgc caatccggta tggacgactg gaagcccagc 150

cccctcatca agccctttgg ggctcggaag aagcggagct ggtaccttac 200

ctggaagtat aaactgacaa accagcgggc cctgcggaga ttctgtcaga 250

caggggccgt gcttttcctg ctggtgactg tcattgtcaa tatcaagttg 300

atcctggaca ctcggcgagc catcagtgaa gccaatgaag acccagagcc 350

agagcaagac tatgatgagg ccctaggccg cctggagccc ccacggcgca 400

gaggcagtgg tccccggcgg gtcctggacg tagaggtgta ttcaagtcgc 450

agcaaagtat atgtggcagt ggatggcacc acggtgctgg aggatgaggc 500

ccgggagcag ggccggggca tccatgtcat tgtcctcaac caggccacgg 550

gccacgtgat ggcaaaacgt gtgtttgaca cgtactcacc tcatgaggat 600

gaggccatgg tgctattcct caacatggta gcgcccggcc gagtgctcat 650

ctgcactgtc aaggatgagg gctccttcca cctcaaggac acagccaagg 700

ctctgctgag gagcctgggc agccaggctg gccctgccct gggctggagg 750

gacacatggg ccttcgtggg acgaaaagga ggtcctgtct tcggggagaa 800

acattctaag tcacctgccc tctcttcctg gggggaccca gtcctgctga 850

agacagatgt gccattgagc tcagcagaag aggcagagtg ccactgggca 900

gacacagagc tgaaccgtcg ccgccggcgc ttctgcagca aagttgaggg 950

ctatggaagt gtatgcagct gcaaggaccc cacacccatc gagttcagcc 1000

ctgacccact cccagacaac aaggtcctca atgtgcctgt ggctgtcatt 1050

gcagggaacc gacccaatta cctgtacagg atgctgcgct ctctgctttc 1100

agcccagggg gtgtctcctc agatgataac agttttcatt gacggctact 1150

atgaggaacc catggatgtg gtggcactgt ttggtctgag ggcatccag 1200

catactccca tcagcatcaa gaatgcccgc gtgtctcagc actacaaggc 1250

cagcctcact gccactttca acctgtttcc ggaggccaag tttgctgtgg 1300

ttctggaaga ggacctggac attgctgtgg attttttcag tttcctgagc 1350

caatccatcc acctactgga ggaggatgac agcctgtact gcatctctgc 1400

ctggaatgac caggggtatg aacacacggc tgaggaccca gcactactgt 1450

accgtgtgga gaccatgcct gggctgggct gggtgctcag gaggtccttg 1500

tacaaggagg agcttgagcc caagtggcct acaccggaaa agctctggga 1550

ttgggacatg tggatgcgga tgcctgaaca acgccggggc cgagagtgca 1600

tcatccctga cgtttcccga tcctaccact ttggcatcgt cggcctcaac 1650

atgaatggct actttcacga ggcctacttc aagaagcaca agttcaacac 1700

ggttccaggt gtccagctca ggaatgtgga cagtctgaag aaagaagctt 1750

atgaagtgga agttcacagg ctgctcagtg aggctgaggt tctggaccac 1800

agcaagaacc cttgtgaaga ctctttcctg ccagacacag agggccacac 1850

ctacgtggcc tttattcgaa tggagaaaga tgatgacttc accacctgga 1900

cccagcttgc caagtgcctc catatctggg acctggatgt gcgtggcaac 1950

catcggggcc tgtggagatt gtttcggaag aagaaccact tcctggtggt 2000

gggggtcccg gcttccccct actcagtgaa gaagccaccc tcagtcaccc 2050

caattttcct ggagccaccc ccaaaggagg agggagcccc aggagcccca 2100

gaacagacat gagacctcct ccaggaccct gcggggctgg gtactgtgta 2150

cccccaggct ggctagccct tccctccatc ctgtaggatt ttgtagatgc 2200

tggtaggggc tggggctacc ttgtttttaa catgagactt aattactaac 2250

tccaagggga gggttcccct gctccaacac cccgttcctg agttaaaagt 2300

ctatttattt acttccttgt tggagaaggg caggagagta cctgggaatc 2350

attacgatcc ctagcagctc atcctgccct ttgaataccc tcactttcca 2400

ggcctggctc agaatctaac ctatttattg actgtcctga gggccttgaa 2450

aacaggccga acctggaggg cctggatttc tttttgggct ggaatgctgc 2500

cctgagggtg gggctggctc ttactcagga aactgctgtg cccaacccat 2550

ggacaggccc agctggggcc cacatgctga cacagactca ctcagagacc 2600

cttagacact ggaccaggcc tcctctcagc cttctctttg tccagatttc 2650

caaagctgga taagttggtc attgattaaa aaaggagaag ccctctggga 2700

aaaaaaaaaa aaaaaaaaaa aaaaa 2725

<210> 480
<211> 660
<212> PRT
<213> Homo Sapien

<400> 480

```
Met Asp Asp Trp Lys Pro Ser Pro Leu Ile Lys Pro Phe Gly Ala
  1               5                  10                  15

Arg Lys Lys Arg Ser Trp Tyr Leu Thr Trp Lys Tyr Lys Leu Thr
                 20                  25                  30

Asn Gln Arg Ala Leu Arg Arg Phe Cys Gln Thr Gly Ala Val Leu
```

```
                35                  40                  45

Phe Leu Leu Val Thr Val Ile Val Asn Ile Lys Leu Ile Leu Asp
                50                  55                  60

Thr Arg Arg Ala Ile Ser Glu Ala Asn Glu Asp Pro Glu Pro Glu
                65                  70                  75

Gln Asp Tyr Asp Glu Ala Leu Gly Arg Leu Glu Pro Pro Arg Arg
                80                  85                  90

Arg Gly Ser Gly Pro Arg Arg Val Leu Asp Val Glu Val Tyr Ser
                95                  100                 105

Ser Arg Ser Lys Val Tyr Val Ala Val Asp Gly Thr Thr Val Leu
                110                 115                 120

Glu Asp Glu Ala Arg Glu Gln Gly Arg Gly Ile His Val Ile Val
                125                 130                 135

Leu Asn Gln Ala Thr Gly His Val Met Ala Lys Arg Val Phe Asp
                140                 145                 150

Thr Tyr Ser Pro His Glu Asp Glu Ala Met Val Leu Phe Leu Asn
                155                 160                 165

Met Val Ala Pro Gly Arg Val Leu Ile Cys Thr Val Lys Asp Glu
                170                 175                 180

Gly Ser Phe His Leu Lys Asp Thr Ala Lys Ala Leu Leu Arg Ser
                185                 190                 195

Leu Gly Ser Gln Ala Gly Pro Ala Leu Gly Trp Arg Asp Thr Trp
                200                 205                 210

Ala Phe Val Gly Arg Lys Gly Gly Pro Val Phe Gly Glu Lys His
                215                 220                 225

Ser Lys Ser Pro Ala Leu Ser Ser Trp Gly Asp Pro Val Leu Leu
                230                 235                 240

Lys Thr Asp Val Pro Leu Ser Ser Ala Glu Glu Ala Glu Cys His
                245                 250                 255

Trp Ala Asp Thr Glu Leu Asn Arg Arg Arg Arg Arg Phe Cys Ser
                260                 265                 270

Lys Val Glu Gly Tyr Gly Ser Val Cys Ser Cys Lys Asp Pro Thr
                275                 280                 285

Pro Ile Glu Phe Ser Pro Asp Pro Leu Pro Asp Asn Lys Val Leu
                290                 295                 300

Asn Val Pro Val Ala Val Ile Ala Gly Asn Arg Pro Asn Tyr Leu
                305                 310                 315

Tyr Arg Met Leu Arg Ser Leu Leu Ser Ala Gln Gly Val Ser Pro
                320                 325                 330

Gln Met Ile Thr Val Phe Ile Asp Gly Tyr Tyr Glu Glu Pro Met
                335                 340                 345

Asp Val Val Ala Leu Phe Gly Leu Arg Gly Ile Gln His Thr Pro
                350                 355                 360
```

```
Ile Ser Ile Lys Asn Ala Arg Val Ser Gln His Tyr Lys Ala Ser
                365                 370                 375

Leu Thr Ala Thr Phe Asn Leu Phe Pro Glu Ala Lys Phe Ala Val
                380                 385                 390

Val Leu Glu Glu Asp Leu Asp Ile Ala Val Asp Phe Phe Ser Phe
                395                 400                 405

Leu Ser Gln Ser Ile His Leu Leu Glu Glu Asp Asp Ser Leu Tyr
                410                 415                 420

Cys Ile Ser Ala Trp Asn Asp Gln Gly Tyr Glu His Thr Ala Glu
                425                 430                 435

Asp Pro Ala Leu Leu Tyr Arg Val Glu Thr Met Pro Gly Leu Gly
                440                 445                 450

Trp Val Leu Arg Arg Ser Leu Tyr Lys Glu Glu Leu Glu Pro Lys
                455                 460                 465

Trp Pro Thr Pro Glu Lys Leu Trp Asp Trp Asp Met Trp Met Arg
                470                 475                 480

Met Pro Glu Gln Arg Arg Gly Arg Glu Cys Ile Ile Pro Asp Val
                485                 490                 495

Ser Arg Ser Tyr His Phe Gly Ile Val Gly Leu Asn Met Asn Gly
                500                 505                 510

Tyr Phe His Glu Ala Tyr Phe Lys Lys His Lys Phe Asn Thr Val
                515                 520                 525

Pro Gly Val Gln Leu Arg Asn Val Asp Ser Leu Lys Lys Glu Ala
                530                 535                 540

Tyr Glu Val Glu Val His Arg Leu Leu Ser Glu Ala Glu Val Leu
                545                 550                 555

Asp His Ser Lys Asn Pro Cys Glu Asp Ser Phe Leu Pro Asp Thr
                560                 565                 570

Glu Gly His Thr Tyr Val Ala Phe Ile Arg Met Glu Lys Asp Asp
                575                 580                 585

Asp Phe Thr Thr Trp Thr Gln Leu Ala Lys Cys Leu His Ile Trp
                590                 595                 600

Asp Leu Asp Val Arg Gly Asn His Arg Gly Leu Trp Arg Leu Phe
                605                 610                 615

Arg Lys Lys Asn His Phe Leu Val Val Gly Val Pro Ala Ser Pro
                620                 625                 630

Tyr Ser Val Lys Lys Pro Pro Ser Val Thr Pro Ile Phe Leu Glu
                635                 640                 645

Pro Pro Pro Lys Glu Glu Gly Ala Pro Gly Ala Pro Glu Gln Thr
                650                 655                 660
```

<210> 481
<211> 1346
<212> DNA

-

<213> Homo Sapien

<400> 481
gaaagaatgt tgtggctgct ctttttttctg gtgactgcca ttcatgctga 50
actctgtcaa ccaggtgcag aaaatgcttt taaagtgaga cttagtatca 100
gaacagctct gggagataaa gcatatgcct gggataccaa tgaagaatac 150
ctcttcaaag cgatggtagc tttctccatg agaaaagttc ccaacagaga 200
agcaacagaa atttcccatg tcctactttg caatgtaacc cagagggtat 250
cattctggtt tgtggttaca gacccttcaa aaaatcacac ccttcctgct 300
gttgaggtgc aatcagccat aagaatgaac aagaaccgga tcaacaatgc 350
cttctttcta aatgaccaaa ctctggaatt tttaaaaatc ccttccacac 400
ttgcaccacc catggaccca tctgtgccca tctggattat tatatttggt 450
gtgatatttt gcatcatcat agttgcaatt gcactactga ttttatcagg 500
gatctggcaa cgtagaagaa agaacaaaga accatctgaa gtggatgacg 550
ctgaagataa gtgtgaaaac atgatcacaa ttgaaaatgg catcccctct 600
gatcccctgg acatgaaggg gggcatatta atgatgcctt catgacagag 650
gatgagaggc tcacccctct ctgaagggct gttgttctgc ttcctcaaga 700
aattaaacat ttgtttctgt gtgactgctg agcatcctga aataccaaga 750
gcagatcata tattttgttt caccattctt cttttgtaat aaattttgaa 800
tgtgcttgaa agtgaaaagc aatcaattat acccaccaac accactgaaa 850
tcataagcta ttcacgactc aaaatattct aaaatatttt tctgacagta 900
tagtgtataa atgtggtcat gtggtatttg tagttattga tttaagcatt 950
tttagaaata agatcaggca tatgtatata ttttcacact tcaaagacct 1000
aaggaaaaat aaattttcca gtggagaata catataatat ggtgtagaaa 1050
tcattgaaaa tggatccttt ttgacgatca cttatatcac tctgtatatg 1100
actaagtaaa caaaagtgag aagtaattat tgtaaatgga tggataaaaa 1150
tggaattact catatacagg gtggaatttt atcctgttat cacaccaaca 1200
gttgattata tattttctga atatcagccc ctaataggac aattctattt 1250
gttgaccatt tctacaattt gtaaaagtcc aatctgtgct aacttaataa 1300
agtaataatc atctcttttt aaaaaaaaaa aaaaaaaaaa aaaaaa 1346

<210> 482
<211> 212
<212> PRT
<213> Homo Sapien

<400> 482
Met Leu Trp Leu Leu Phe Phe Leu Val Thr Ala Ile His Ala Glu

```
1               5                   10                  15

Leu Cys Gln Pro Gly Ala Glu Asn Ala Phe Lys Val Arg Leu Ser
                20                  25                  30

Ile Arg Thr Ala Leu Gly Asp Lys Ala Tyr Ala Trp Asp Thr Asn
                35                  40                  45

Glu Glu Tyr Leu Phe Lys Ala Met Val Ala Phe Ser Met Arg Lys
                50                  55                  60

Val Pro Asn Arg Glu Ala Thr Glu Ile Ser His Val Leu Leu Cys
                65                  70                  75

Asn Val Thr Gln Arg Val Ser Phe Trp Phe Val Val Thr Asp Pro
                80                  85                  90

Ser Lys Asn His Thr Leu Pro Ala Val Glu Val Gln Ser Ala Ile
                95                  100                 105

Arg Met Asn Lys Asn Arg Ile Asn Asn Ala Phe Phe Leu Asn Asp
                110                 115                 120

Gln Thr Leu Glu Phe Leu Lys Ile Pro Ser Thr Leu Ala Pro Pro
                125                 130                 135

Met Asp Pro Ser Val Pro Ile Trp Ile Ile Ile Phe Gly Val Ile
                140                 145                 150

Phe Cys Ile Ile Ile Val Ala Ile Ala Leu Leu Ile Leu Ser Gly
                155                 160                 165

Ile Trp Gln Arg Arg Arg Lys Asn Lys Glu Pro Ser Glu Val Asp
                170                 175                 180

Asp Ala Glu Asp Lys Cys Glu Asn Met Ile Thr Ile Glu Asn Gly
                185                 190                 195

Ile Pro Ser Asp Pro Leu Asp Met Lys Gly Gly Ile Leu Met Met
                200                 205                 210

Pro Ser
```

<210> 483
<211> 2498
<212> DNA
<213> Homo Sapien

<400> 483

```
cgtctctgcg ttcgccatgc gtcccggggc gccagggcca ctctggcctc 50

tgccctgggg ggccctggct tgggccgtgg gcttcgtgag ctccatgggc 100

tcggggaacc ccgcgcccgg tggtgtttgc tggctccagc agggccagga 150

ggccacctgc agcctggtgc tccagactga tgtcacccgg gccgagtgct 200

gtgcctccgg caacattgac accgcctggt ccaacctcac ccacccgggg 250

aacaagatca acctcctcgg cttcttgggc cttgtccact gccttccctg 300

caaagattcg tgcgacggcg tggagtgcgg cccgggcaag gcgtgccgca 350
```

tgctgggggg ccgcccgcgc tgcgagtgcg cgcccgactg ctcggggctc 400
ccggcgcggc tgcaggtctg cggctcagac ggcgccacct accgcgacga 450
gtgcgagctg cgcgccgcgc gctgccgcgg ccacccggac ctgagcgtca 500
tgtaccgggg ccgctgccgc aagtcctgtg agcacgtggt gtgcccgcgg 550
ccacagtcgt gcgtcgtgga ccagacgggc agcgcccact gcgtggtgtg 600
tcgagcggcg ccctgccctg tgccctccag ccccggccag gagctttgcg 650
gcaacaacaa cgtcacctac atctcctcgt gccacatgcg ccaggccacc 700
tgcttcctgg gccgctccat cggcgtgcgc cacgcgggca gctgcgcagg 750
cacccctgag gagccgccag gtggtgagtc tgcagaagag gaagagaact 800
tcgtgtgagc ctgcaggaca ggcctgggcc tggtgcccga ggccccccat 850
catcccctgt tatttattgc cacagcagag tctaatttat atgccacgga 900
cactccttag agcccggatt cggaccactt ggggatccca gaacctccct 950
gacgatatcc tggaaggact gaggaaggga ggcctggggg ccggctggtg 1000
ggtgggatag acctgcgttc cggacactga gcgcctgatt tagggccctt 1050
ctctaggatg ccccagcccc taccctaaga cctattgccg gggaggattc 1100
cacacttccg ctcctttggg gataaaccta ttaattattg ctactatcaa 1150
gagggctggg cattctctgc tggtaattcc tgaagaggca tgactgcttt 1200
tctcagcccc aagcctctag tctgggtgtg tacggagggt ctagcctggg 1250
tgtgtacgga gggtctagcc tgggtgagta cggagggtct agcctgggtg 1300
agtacggagg gtctagcctg ggtgagtacg gagggtctag cctgggtgtg 1350
tatggaggat ctagcctggg tgagtatgga gggtctagcc tgggtgagta 1400
tggagggtct agcctgggtg tgtatggagg gtctagcctg ggtgagtatg 1450
gagggtctag cctgggtgtg tatggagggt ctagcctggg tgagtatgga 1500
gggtctagcc tgggtgtgta cggagggtct agtctgagtg cgtgtgggga 1550
cctcagaaca ctgtgacctt agcccagcaa gccaggccct tcatgaaggc 1600
caagaaggct gccaccattc cctgccagcc caagaactcc agcttcccca 1650
ctgcctctgt gtgccccttt gcgtcctgtg aaggccattg agaaatgccc 1700
agtgtgcccc ctgggaaagg gcacggcctg tgctcctgac acgggctgtg 1750
cttggccaca gaaccaccca gcgtctcccc tgctgctgtc cacgtcagtt 1800
catgaggcaa cgtcgcgtgg tctcagacgt ggagcagcca gcggcagctc 1850
agagcagggc actgtgtccg gcggagccaa gtccactctg gggagctct 1900
ggcggggacc acgggccact gctcacccac tggccccgag gggggtgtag 1950

acgccaagac tcacgcatgt gtgacatccg gagtcctgga gccgggtgtc 2000

ccagtggcac cactaggtgc ctgctgcctc cacagtgggg ttcacaccca 2050

gggctccttg gtcccccaca acctgccccg gccaggcctg cagacccaga 2100

ctccagccag acctgcctca cccaccaatg cagccggggc tggcgacacc 2150

agccaggtgc tggtcttggg ccagttctcc cacgacggct caccctcccc 2200

tccatctgcg ttgatgctca gaatcgccta cctgtgcctg cgtgtaaacc 2250

acagcctcag accagctatg gggagaggac aacacggagg atatccagct 2300

tccccggtct ggggtgagga atgtggggag cttgggcatc ctcctccagc 2350

ctcctccagc ccccaggcag tgccttacct gtggtgccca gaaaagtgcc 2400

cctaggttgg tgggtctaca ggagcctcag ccaggcagcc caccccaccc 2450

tggggccctg cctcaccaag gaaataaaga ctcaagccat aaaaaaaa 2498

<210> 484
<211> 263
<212> PRT
<213> Homo Sapien

<400> 484
Met Arg Pro Gly Ala Pro Gly Pro Leu Trp Pro Leu Pro Trp Gly
1 5 10 15

Ala Leu Ala Trp Ala Val Gly Phe Val Ser Ser Met Gly Ser Gly
20 25 30

Asn Pro Ala Pro Gly Gly Val Cys Trp Leu Gln Gln Gly Gln Glu
35 40 45

Ala Thr Cys Ser Leu Val Leu Gln Thr Asp Val Thr Arg Ala Glu
50 55 60

Cys Cys Ala Ser Gly Asn Ile Asp Thr Ala Trp Ser Asn Leu Thr
65 70 75

His Pro Gly Asn Lys Ile Asn Leu Leu Gly Phe Leu Gly Leu Val
80 85 90

His Cys Leu Pro Cys Lys Asp Ser Cys Asp Gly Val Glu Cys Gly
95 100 105

Pro Gly Lys Ala Cys Arg Met Leu Gly Gly Arg Pro Arg Cys Glu
110 115 120

Cys Ala Pro Asp Cys Ser Gly Leu Pro Ala Arg Leu Gln Val Cys
125 130 135

Gly Ser Asp Gly Ala Thr Tyr Arg Asp Glu Cys Glu Leu Arg Ala
140 145 150

Ala Arg Cys Arg Gly His Pro Asp Leu Ser Val Met Tyr Arg Gly
155 160 165

Arg Cys Arg Lys Ser Cys Glu His Val Val Cys Pro Arg Pro Gln
170 175 180

```
Ser Cys Val Val Asp Gln Thr Gly Ser Ala His Cys Val Val Cys
                185                 190                 195

Arg Ala Ala Pro Cys Pro Val Pro Ser Ser Pro Gly Gln Glu Leu
                200                 205                 210

Cys Gly Asn Asn Asn Val Thr Tyr Ile Ser Ser Cys His Met Arg
                215                 220                 225

Gln Ala Thr Cys Phe Leu Gly Arg Ser Ile Gly Val Arg His Ala
                230                 235                 240

Gly Ser Cys Ala Gly Thr Pro Glu Glu Pro Pro Gly Gly Glu Ser
                245                 250                 255

Ala Glu Glu Glu Glu Asn Phe Val
                260
```

<210> 485
<211> 1429
<212> DNA
<213> Homo Sapien

<400> 485

```
gctcgaggcc ggcggcggcg ggagagcgac ccgggcggcc tcgtagcggg 50

gccccggatc cccgagtggc ggccggagcc tcgaaaagag attctcagcg 100

ctgattttga gatgatgggc ttgggaaacg ggcgtcgcag catgaagtcg 150

ccgcccctcg tgctggccgc cctggtggcc tgcatcatcg tcttgggctt 200

caactactgg attgcgagct cccggagcgt ggacctccag acacggatca 250

tggagctgga aggcagggtc cgcagggcgg ctgcagagag aggcgccgtg 300

gagctgaaga agaacgagtt ccagggagag ctggagaagc agcgggagca 350

gcttgacaaa atccagtcca gccacaactt ccagctggag agcgtcaaca 400

agctgtacca ggacgaaaag gcggttttgg tgaataacat caccacaggt 450

gagaggctca tccgagtgct gcaagaccag ttaaagaccc tgcagaggaa 500

ttacggcagg ctgcagcagg atgtcctcca gtttcagaag aaccagacca 550

acctggagag gaagttctcc tacgacctga gccagtgcat caatcagatg 600

aaggaggtga aggaacagtg tgaggagcga atagaagagg tcaccaaaaa 650

ggggaatgaa gctgtagctt ccagagacct gagtgaaaac aacgaccaga 700

gacagcagct ccaagccctc agtgagcctc agcccaggct gcaggcagca 750

ggcctgccac acacagaggt gccacaaggg aagggaaacg tgcttggtaa 800

cagcaagtcc cagacaccag cccccagttc cgaagtggtt ttggattcaa 850

agagacaagt tgagaaagag gaaaccaatg agatccaggt ggtgaatgag 900

gagcctcaga gggacaggct gccgcaggag ccaggccggg agcaggtggt 950

ggaagacaga cctgtaggtg gaagaggctt cgggggagcc ggagaactgg 1000
```

gccagacccc acaggtgcag gctgccctgt cagtgagcca ggaaaatcca 1050

gagatggagg gccctgagcg agaccagctt gtcatccccg acggacagga 1100

ggaggagcag gaagctgccg gggaagggag aaaccagcag aaactgagag 1150

gagaagatga ctacaacatg gatgaaaatg aagcagaatc tgagacagac 1200

aagcaagcag ccctggcagg gaatgacaga aacatagatg tttttaatgt 1250

tgaagatcag aaaagagaca ccataaattt acttgatcag cgtgaaaagc 1300

ggaatcatac actctgaatt gaactggaat cacatatttc acaacagggc 1350

cgaagagatg actataaaat gttcatgagg gactgaatac tgaaaactgt 1400

gaaatgtact aaataaaatg tacatctga 1429

<210> 486
<211> 401
<212> PRT
<213> Homo Sapien

<400> 486
Met Met Gly Leu Gly Asn Gly Arg Arg Ser Met Lys Ser Pro Pro
1 5 10 15

Leu Val Leu Ala Ala Leu Val Ala Cys Ile Ile Val Leu Gly Phe
20 25 30

Asn Tyr Trp Ile Ala Ser Ser Arg Ser Val Asp Leu Gln Thr Arg
35 40 45

Ile Met Glu Leu Glu Gly Arg Val Arg Arg Ala Ala Ala Glu Arg
50 55 60

Gly Ala Val Glu Leu Lys Lys Asn Glu Phe Gln Gly Glu Leu Glu
65 70 75

Lys Gln Arg Glu Gln Leu Asp Lys Ile Gln Ser Ser His Asn Phe
80 85 90

Gln Leu Glu Ser Val Asn Lys Leu Tyr Gln Asp Glu Lys Ala Val
95 100 105

Leu Val Asn Asn Ile Thr Thr Gly Glu Arg Leu Ile Arg Val Leu
110 115 120

Gln Asp Gln Leu Lys Thr Leu Gln Arg Asn Tyr Gly Arg Leu Gln
125 130 135

Gln Asp Val Leu Gln Phe Gln Lys Asn Gln Thr Asn Leu Glu Arg
140 145 150

Lys Phe Ser Tyr Asp Leu Ser Gln Cys Ile Asn Gln Met Lys Glu
155 160 165

Val Lys Glu Gln Cys Glu Glu Arg Ile Glu Glu Val Thr Lys Lys
170 175 180

Gly Asn Glu Ala Val Ala Ser Arg Asp Leu Ser Glu Asn Asn Asp
185 190 195

Gln Arg Gln Gln Leu Gln Ala Leu Ser Glu Pro Gln Pro Arg Leu

200 205 210

Gln Ala Ala Gly Leu Pro His Thr Glu Val Pro Gln Gly Lys Gly
215 220 225

Asn Val Leu Gly Asn Ser Lys Ser Gln Thr Pro Ala Pro Ser Ser
230 235 240

Glu Val Val Leu Asp Ser Lys Arg Gln Val Glu Lys Glu Glu Thr
245 250 255

Asn Glu Ile Gln Val Val Asn Glu Glu Pro Gln Arg Asp Arg Leu
260 265 270

Pro Gln Glu Pro Gly Arg Glu Gln Val Val Glu Asp Arg Pro Val
275 280 285

Gly Gly Arg Gly Phe Gly Gly Ala Gly Glu Leu Gly Gln Thr Pro
290 295 300

Gln Val Gln Ala Ala Leu Ser Val Ser Gln Glu Asn Pro Glu Met
305 310 315

Glu Gly Pro Glu Arg Asp Gln Leu Val Ile Pro Asp Gly Gln Glu
320 325 330

Glu Glu Gln Glu Ala Ala Gly Glu Gly Arg Asn Gln Gln Lys Leu
335 340 345

Arg Gly Glu Asp Asp Tyr Asn Met Asp Glu Asn Glu Ala Glu Ser
350 355 360

Glu Thr Asp Lys Gln Ala Ala Leu Ala Gly Asn Asp Arg Asn Ile
365 370 375

Asp Val Phe Asn Val Glu Asp Gln Lys Arg Asp Thr Ile Asn Leu
380 385 390

Leu Asp Gln Arg Glu Lys Arg Asn His Thr Leu
395 400

<210> 487
<211> 1371
<212> DNA
<213> Homo Sapien

<400> 487
aactcaaact cctctctctg ggaaaacgcg gtgcttgctc ctcccggagt 50

ggccttggca gggtgttgga gccctcggtc tgccccgtcc ggtctctggg 100

gccaaggctg ggtttccctc atgtatggca agagctctac tcgtgcggtg 150

cttcttctcc ttggcataca gctcacagct ctttggccta tagcagctgt 200

ggaaatttat acctcccggg tgctggaggc tgttaatggg acagatgctc 250

ggttaaaatg cactttctcc agctttgccc ctgtgggtga tgctctaaca 300

gtgacctgga attttcgtcc tctagacggg ggacctgagc agtttgtatt 350

ctactaccac atagatccct tccaacccat gagtgggcgg tttaaggacc 400

gggtgtcttg ggatgggaat cctgagcggt acgatgcctc catccttctc 450

tggaaactgc agttcgacga caatgggaca tacacctgcc aggtgaagaa 500

cccacctgat gttgatgggg tgatagggga gatccggctc agcgtcgtgc 550

acactgtacg cttctctgag atccacttcc tggctctggc cattggctct 600

gcctgtgcac tgatgatcat aatagtaatt gtagtggtcc tcttccagca 650

ttaccggaaa aagcgatggg ccgaaagagc tcataaagtg gtggagataa 700

aatcaaaaga agaggaaagg ctcaaccaag agaaaaaggt ctctgtttat 750

ttagaagaca cagactaaca attttagatg gaagctgaga tgatttccaa 800

gaacaagaac cctagtattt cttgaagtta atggaaactt ttctttggct 850

tttccagttg tgacccgttt tccaaccagt tctgcagcat attagattct 900

agacaagcaa cacccctctg gagccagcac agtgctcctc catatcacca 950

gtcatacaca gcctcattat taaggtctta tttaatttca gagtgtaaat 1000

tttttcaagt gctcattagg ttttataaac aagaagctac atttttgccc 1050

ttaagacact acttacagtg ttatgacttg tatacacata tattggtatc 1100

aaaggggata aaagccaatt tgtctgttac atttcctttc acgtatttct 1150

tttagcagca cttctgctac taaagttaat gtgtttactc tctttccttc 1200

ccacattctc aattaaaagg tgagctaagc ctcctcggtg tttctgatta 1250

acagtaaatc ctaaattcaa actgttaaat gacattttta tttttatgtc 1300

tctccttaac tatgagacac atcttgtttt actgaatttc tttcaatatt 1350

ccaggtgata gatttttgtc g 1371

<210> 488
<211> 215
<212> PRT
<213> Homo Sapien

<400> 488
Met Tyr Gly Lys Ser Ser Thr Arg Ala Val Leu Leu Leu Leu Gly
1 5 10 15

Ile Gln Leu Thr Ala Leu Trp Pro Ile Ala Ala Val Glu Ile Tyr
20 25 30

Thr Ser Arg Val Leu Glu Ala Val Asn Gly Thr Asp Ala Arg Leu
35 40 45

Lys Cys Thr Phe Ser Ser Phe Ala Pro Val Gly Asp Ala Leu Thr
50 55 60

Val Thr Trp Asn Phe Arg Pro Leu Asp Gly Gly Pro Glu Gln Phe
65 70 75

Val Phe Tyr Tyr His Ile Asp Pro Phe Gln Pro Met Ser Gly Arg
80 85 90

Phe Lys Asp Arg Val Ser Trp Asp Gly Asn Pro Glu Arg Tyr Asp

95 100 105

Ala Ser Ile Leu Leu Trp Lys Leu Gln Phe Asp Asp Asn Gly Thr
110 115 120

Tyr Thr Cys Gln Val Lys Asn Pro Pro Asp Val Asp Gly Val Ile
125 130 135

Gly Glu Ile Arg Leu Ser Val Val His Thr Val Arg Phe Ser Glu
140 145 150

Ile His Phe Leu Ala Leu Ala Ile Gly Ser Ala Cys Ala Leu Met
155 160 165

Ile Ile Ile Val Ile Val Val Val Leu Phe Gln His Tyr Arg Lys
170 175 180

Lys Arg Trp Ala Glu Arg Ala His Lys Val Val Glu Ile Lys Ser
185 190 195

Lys Glu Glu Glu Arg Leu Asn Gln Glu Lys Lys Val Ser Val Tyr
200 205 210

Leu Glu Asp Thr Asp
215

<210> 489
<211> 2476
<212> DNA
<213> Homo Sapien

<400> 489
aagcaaccaa actgcaagct ttgggagttg ttcgctgtcc ctgccctgct 50

ctgctaggga gagaacgcca gagggaggcg gctggcccgg cggcaggctc 100

tcagaaccgc taccggcgat gctactgctg tgggtgtcgg tggtcgcagc 150

cttggcgctg gcggtactgg cccccggagc aggggagcag aggcggagag 200

cagccaaagc gcccaatgtg gtgctggtcg tgagcgactc cttcgatgga 250

aggttaacat ttcatccagg aagtcaggta gtgaaacttc cttttatcaa 300

ctttatgaag acacgtggga cttcctttct gaatgcctac acaaactctc 350

caatttgttg cccatcacgc gcagcaatgt ggagtggcct cttcactcac 400

ttaacagaat cttggaataa ttttaagggt ctagatccaa attatacaac 450

atggatggat gtcatggaga ggcatggcta ccgaacacag aaatttggga 500

aactggacta tacttcagga catcactcca ttagtaatcg tgtggaagcg 550

tggacaagag atgttgcttt cttactcaga caagaaggca ggcccatggt 600

taatcttatc cgtaacagga ctaaagtcag agtgatggaa agggattggc 650

agaatacaga caaagcagta aactggttaa gaaaggaagc aattaattac 700

actgaaccat ttgttattta cttgggatta aatttaccac acccttaccc 750

ttcaccatct tctggagaaa attttggatc ttcaacattt cacacatctc 800

tttattggct tgaaaaagtg tctcatgatg ccatcaaaat cccaaagtgg 850
tcacctttgt cagaaatgca ccctgtagat tattactctt cttatacaaa 900
aaactgcact ggaagattta caaaaaaaga aattaagaat attagagcat 950
tttattatgc tatgtgtgct gagacagatg ccatgcttgg tgaaattatt 1000
ttggcccttc atcaattaga tcttcttcag aaaactattg tcatatactc 1050
ctcagaccat ggagagctgg ccatggaaca tcgacagttt tataaaatga 1100
gcatgtacga ggctagtgca catgttccgc ttttgatgat gggaccagga 1150
attaaagccg gcctacaagt atcaaatgtg gtttctcttg tggatattta 1200
ccctaccatg cttgatattg ctggaattcc tctgcctcag aacctgagtg 1250
gatactcttt gttgccgtta tcatcagaaa catttaagaa tgaacataaa 1300
gtcaaaaacc tgcatccacc ctggattctg agtgaattcc atggatgtaa 1350
tgtgaatgcc tccacctaca tgcttcgaac taaccactgg aaatatatag 1400
cctattcgga tggtgcatca atattgcctc aactctttga tctttcctcg 1450
gatccagatg aattaacaaa tgttgctgta aaatttccag aaattactta 1500
ttctttggat cagaagcttc attccattat aaactaccct aaagtttctg 1550
cttctgtcca ccagtataat aaagagcagt ttatcaagtg gaaacaaagt 1600
ataggacaga attattcaaa cgttatagca aatcttaggt ggcaccaaga 1650
ctggcagaag gaaccaagga agtatgaaaa tgcaattgat cagtggctta 1700
aaacccatat gaatccaaga gcagtttgaa caaaaagttt aaaaatagtg 1750
ttctagagat acatataaat atattacaag atcataatta tgtattttaa 1800
atgaaacagt tttaataatt accaagtttt ggccgggcac agtggctcac 1850
acctgtaatc ccaggacttt gggaggctga ggaaagcaga tcacaaggtc 1900
aagagattga gaccatcctg gccaacatgg tgaaaccctg tctctactaa 1950
aaatacaaaa attagctggg cgcggtggtg cacacctata gtctcagcta 2000
ctcagaggct gaggcaggag gatcgcttga acccgggagg cagcagttgc 2050
agtgagctga gattgcgcca ctgtactcca gcctggcaac agagtgagac 2100
tgtgtcgcaa aaaaataaaa ataaaataat aataattacc aatttttcat 2150
tattttgtaa gaatgtagtg tattttaaga taaaatgcca atgattataa 2200
aatcacatat tttcaaaaat ggttattatt taggcctttg tacaatttct 2250
aacaatttag tggaagtatc aaaaggattg aagcaaatac tgtaacagtt 2300
atgttccttt aaataataga gaatataaaa tattgtaata atatgtatca 2350
taaaatagtt gtatgtgagc atttgatggt gaaaaaaaaa aaaaaaaaaa 2400

aaaaaaaaaa aaaaaaaaaa aaaaaaaaaa aaaaaaaaaa aaaaaaaaaa 2450

aaaaaaaaaa aaaaaaaaaa aaaaaa 2476

<210> 490
<211> 536
<212> PRT
<213> Homo Sapien

<400> 490

```
Met Leu Leu Leu Trp Val Ser Val Val Ala Ala Leu Ala Leu Ala
1               5                   10                  15

Val Leu Ala Pro Gly Ala Gly Glu Gln Arg Arg Arg Ala Ala Lys
                20                  25                  30

Ala Pro Asn Val Val Leu Val Val Ser Asp Ser Phe Asp Gly Arg
                35                  40                  45

Leu Thr Phe His Pro Gly Ser Gln Val Val Lys Leu Pro Phe Ile
                50                  55                  60

Asn Phe Met Lys Thr Arg Gly Thr Ser Phe Leu Asn Ala Tyr Thr
                65                  70                  75

Asn Ser Pro Ile Cys Cys Pro Ser Arg Ala Ala Met Trp Ser Gly
                80                  85                  90

Leu Phe Thr His Leu Thr Glu Ser Trp Asn Asn Phe Lys Gly Leu
                95                  100                 105

Asp Pro Asn Tyr Thr Thr Trp Met Asp Val Met Glu Arg His Gly
                110                 115                 120

Tyr Arg Thr Gln Lys Phe Gly Lys Leu Asp Tyr Thr Ser Gly His
                125                 130                 135

His Ser Ile Ser Asn Arg Val Glu Ala Trp Thr Arg Asp Val Ala
                140                 145                 150

Phe Leu Leu Arg Gln Glu Gly Arg Pro Met Val Asn Leu Ile Arg
                155                 160                 165

Asn Arg Thr Lys Val Arg Val Met Glu Arg Asp Trp Gln Asn Thr
                170                 175                 180

Asp Lys Ala Val Asn Trp Leu Arg Lys Glu Ala Ile Asn Tyr Thr
                185                 190                 195

Glu Pro Phe Val Ile Tyr Leu Gly Leu Asn Leu Pro His Pro Tyr
                200                 205                 210

Pro Ser Pro Ser Ser Gly Glu Asn Phe Gly Ser Ser Thr Phe His
                215                 220                 225

Thr Ser Leu Tyr Trp Leu Glu Lys Val Ser His Asp Ala Ile Lys
                230                 235                 240

Ile Pro Lys Trp Ser Pro Leu Ser Glu Met His Pro Val Asp Tyr
                245                 250                 255

Tyr Ser Ser Tyr Thr Lys Asn Cys Thr Gly Arg Phe Thr Lys Lys
                260                 265                 270
```

Glu Ile Lys Asn Ile Arg Ala Phe Tyr Tyr Ala Met Cys Ala Glu
275 280 285

Thr Asp Ala Met Leu Gly Glu Ile Ile Leu Ala Leu His Gln Leu
290 295 300

Asp Leu Leu Gln Lys Thr Ile Val Ile Tyr Ser Ser Asp His Gly
305 310 315

Glu Leu Ala Met Glu His Arg Gln Phe Tyr Lys Met Ser Met Tyr
320 325 330

Glu Ala Ser Ala His Val Pro Leu Leu Met Met Gly Pro Gly Ile
335 340 345

Lys Ala Gly Leu Gln Val Ser Asn Val Val Ser Leu Val Asp Ile
350 355 360

Tyr Pro Thr Met Leu Asp Ile Ala Gly Ile Pro Leu Pro Gln Asn
365 370 375

Leu Ser Gly Tyr Ser Leu Leu Pro Leu Ser Ser Glu Thr Phe Lys
380 385 390

Asn Glu His Lys Val Lys Asn Leu His Pro Pro Trp Ile Leu Ser
395 400 405

Glu Phe His Gly Cys Asn Val Asn Ala Ser Thr Tyr Met Leu Arg
410 415 420

Thr Asn His Trp Lys Tyr Ile Ala Tyr Ser Asp Gly Ala Ser Ile
425 430 435

Leu Pro Gln Leu Phe Asp Leu Ser Ser Asp Pro Asp Glu Leu Thr
440 445 450

Asn Val Ala Val Lys Phe Pro Glu Ile Thr Tyr Ser Leu Asp Gln
455 460 465

Lys Leu His Ser Ile Ile Asn Tyr Pro Lys Val Ser Ala Ser Val
470 475 480

His Gln Tyr Asn Lys Glu Gln Phe Ile Lys Trp Lys Gln Ser Ile
485 490 495

Gly Gln Asn Tyr Ser Asn Val Ile Ala Asn Leu Arg Trp His Gln
500 505 510

Asp Trp Gln Lys Glu Pro Arg Lys Tyr Glu Asn Ala Ile Asp Gln
515 520 525

Trp Leu Lys Thr His Met Asn Pro Arg Ala Val
530 535

<210> 491
<211> 1475
<212> DNA
<213> Homo Sapien

<400> 491
gagagaagtc agcctggcag agagactctg aaatgaggga ttagaggtgt 50

tcaaggagca agagcttcag cctgaagaca agggagcagt ccctgaagac 100

gcttctactg agaggtctgc catggcctct cttggcctcc aacttgtggg 150
ctacatccta ggccttctgg ggcttttggg cacactggtt gccatgctgc 200
tccccagctg gaaaacaagt tcttatgtcg gtgccagcat tgtgacagca 250
gttggcttct ccaagggcct ctggatggaa tgtgccacac acagcacagg 300
catcacccag tgtgacatct atagcaccct tctgggcctg cccgctgaca 350
tccaggctgc ccaggccatg atggtgacat ccagtgcaat ctcctccctg 400
gcctgcatta tctctgtggt gggcatgaga tgcacagtct tctgccagga 450
atcccgagcc aaagacagag tggcggtagc aggtggagtc tttttcatcc 500
ttggaggcct cctgggattc attcctgttg cctggaatct tcatgggatc 550
ctacgggact tctactcacc actggtgcct gacagcatga aatttgagat 600
tggagaggct ctttacttgg gcattatttc ttccctgttc tccctgatag 650
ctggaatcat cctctgcttt tcctgctcat cccagagaaa tcgctccaac 700
tactacgatg cctaccaagc ccaacctctt gccacaagga gctctccaag 750
gcctggtcaa cctcccaaag tcaagagtga gttcaattcc tacagcctga 800
cagggtatgt gtgaagaacc aggggccaga gctggggggt ggctgggtct 850
gtgaaaaaca gtggacagca ccccgagggc cacaggtgag ggacactacc 900
actggatcgt gtcagaaggt gctgctgagg atagactgac tttggccatt 950
ggattgagca aaggcagaaa tgggggctag tgtaacagca tgcaggttga 1000
attgccaagg atgctcgcca tgccagcctt tctgttttcc tcaccttgct 1050
gctcccctgc cctaagtccc caaccctcaa cttgaaaccc cattccctta 1100
agccaggact cagaggatcc ctttgccctc tggtttacct gggactccat 1150
ccccaaaccc actaatcaca tcccactgac tgaccctctg tgatcaaaga 1200
ccctctctct ggctgaggtt ggctcttagc tcattgctgg ggatgggaag 1250
gagaagcagt ggcttttgtg ggcattgctc taacctactt ctcaagcttc 1300
cctccaaaga aactgattgg ccctggaacc tccatcccac tcttgttatg 1350
actccacagt gtccagacta atttgtgcat gaactgaaat aaaaccatcc 1400
tacggtatcc agggaacaga aagcaggatg caggatggga ggacaggaag 1450
gcagcctggg acatttaaaa aaata 1475

<210> 492
<211> 230
<212> PRT
<213> Homo Sapien

<400> 492
Met Ala Ser Leu Gly Leu Gln Leu Val Gly Tyr Ile Leu Gly Leu
1 5 10 15

```
Leu Gly Leu Leu Gly Thr Leu Val Ala Met Leu Leu Pro Ser Trp
                20                  25                  30

Lys Thr Ser Ser Tyr Val Gly Ala Ser Ile Val Thr Ala Val Gly
                35                  40                  45

Phe Ser Lys Gly Leu Trp Met Glu Cys Ala Thr His Ser Thr Gly
                50                  55                  60

Ile Thr Gln Cys Asp Ile Tyr Ser Thr Leu Leu Gly Leu Pro Ala
                65                  70                  75

Asp Ile Gln Ala Ala Gln Ala Met Met Val Thr Ser Ser Ala Ile
                80                  85                  90

Ser Ser Leu Ala Cys Ile Ile Ser Val Val Gly Met Arg Cys Thr
                95                  100                 105

Val Phe Cys Gln Glu Ser Arg Ala Lys Asp Arg Val Ala Val Ala
                110                 115                 120

Gly Gly Val Phe Phe Ile Leu Gly Gly Leu Leu Gly Phe Ile Pro
                125                 130                 135

Val Ala Trp Asn Leu His Gly Ile Leu Arg Asp Phe Tyr Ser Pro
                140                 145                 150

Leu Val Pro Asp Ser Met Lys Phe Glu Ile Gly Glu Ala Leu Tyr
                155                 160                 165

Leu Gly Ile Ile Ser Ser Leu Phe Ser Leu Ile Ala Gly Ile Ile
                170                 175                 180

Leu Cys Phe Ser Cys Ser Ser Gln Arg Asn Arg Ser Asn Tyr Tyr
                185                 190                 195

Asp Ala Tyr Gln Ala Gln Pro Leu Ala Thr Arg Ser Ser Pro Arg
                200                 205                 210

Pro Gly Gln Pro Pro Lys Val Lys Ser Glu Phe Asn Ser Tyr Ser
                215                 220                 225

Leu Thr Gly Tyr Val
                230
```

<210> 493
<211> 610
<212> DNA
<213> Homo Sapien

<400> 493

```
gcactgctgc tgtcccatca gctgctctga agctccatgg tgcccagaat 50

cttcgctcct gcttatgtgt cagtctgtct cctcctcttg tgtccaaggg 100

aagtcatcgc tcccgctggc tcagaaccat ggctgtgcca gccggcaccc 150

aggtgtggag acaagatcta caacccccttg gagcagtgct gttacaatga 200

cgccatcgtg tccctgagcg agacccgcca atgtggtccc ccctgcacct 250

tctggccctg ctttgagctc tgctgtcttg attcctttgg cctcacaaac 300
```

gattttgttg tgaagctgaa ggttcagggt gtgaattccc agtgccactc 350

atctcccatc tccagtaaat gtgaaagcag aagacgtttt ccctgagaag 400

acatagaaag aaaatcaact ttcactaagg catctcagaa acataggcta 450

aggtaatatg tgtaccagta gagaagcctg aggaatttac aaaatgatgc 500

agctccaagc cattgtatgg cccatgtggg agactgatgg gacatggaga 550

atgacagtag attatcagga aataaataaa gtggtttttc caatgtacac 600

acctgtaaaa 610

<210> 494
<211> 119
<212> PRT
<213> Homo Sapien

<400> 494
Met Val Pro Arg Ile Phe Ala Pro Ala Tyr Val Ser Val Cys Leu
1               5                   10                  15

Leu Leu Leu Cys Pro Arg Glu Val Ile Ala Pro Ala Gly Ser Glu
                20                  25                  30

Pro Trp Leu Cys Gln Pro Ala Pro Arg Cys Gly Asp Lys Ile Tyr
                35                  40                  45

Asn Pro Leu Glu Gln Cys Cys Tyr Asn Asp Ala Ile Val Ser Leu
                50                  55                  60

Ser Glu Thr Arg Gln Cys Gly Pro Pro Cys Thr Phe Trp Pro Cys
                65                  70                  75

Phe Glu Leu Cys Cys Leu Asp Ser Phe Gly Leu Thr Asn Asp Phe
                80                  85                  90

Val Val Lys Leu Lys Val Gln Gly Val Asn Ser Gln Cys His Ser
                95                  100                 105

Ser Pro Ile Ser Ser Lys Cys Glu Ser Arg Arg Arg Phe Pro
                110                 115

<210> 495
<211> 771
<212> DNA
<213> Homo Sapien

<400> 495
ctccactgca accacccaga gccatggctc cccgaggctg catcgtagct 50

gtctttgcca ttttctgcat ctccaggctc ctctgctcac acggagcccc 100

agtggccccc atgactcctt acctgatgct gtgccagcca cacaagagat 150

gtggggacaa gttctacgac cccctgcagc actgttgcta tgatgatgcc 200

gtcgtgccct tggccaggac ccagacgtgt ggaaactgca ccttcagagt 250

ctgctttgag cagtgctgcc cctggacctt catggtgaag ctgataaacc 300

agaactgcga ctcagcccgg acctcggatg acaggctttg tcgcagtgtc 350

agctaatgga acatcagggg aacgatgact cctggattct ccttcctggg 400

tgggcctgga gaaagaggct ggtgttacct gagatctggg atgctgagtg 450

gctgtttggg ggccagagaa acacacactc aactgcccac ttcattctgt 500

gacctgtctg aggcccaccc tgcagctgcc ctgaggaggc ccacaggtcc 550

ccttctagaa ttctggacag catgagatgc gtgtgctgat gggggcccag 600

ggactctgaa ccctcctgat gacccctatg gccaacatca acccggcacc 650

accccaaggc tggctgggga acccttcacc cttctgtgag attttccatc 700

atctcaagtt ctcttctatc caggagcaaa gcacaggatc ataataaatt 750

tatgtacttt ataaatgaaa a 771

<210> 496
<211> 110
<212> PRT
<213> Homo Sapien

<400> 496
Met Ala Pro Arg Gly Cys Ile Val Ala Val Phe Ala Ile Phe Cys
1               5                   10                  15

Ile Ser Arg Leu Leu Cys Ser His Gly Ala Pro Val Ala Pro Met
                20                  25                  30

Thr Pro Tyr Leu Met Leu Cys Gln Pro His Lys Arg Cys Gly Asp
                35                  40                  45

Lys Phe Tyr Asp Pro Leu Gln His Cys Cys Tyr Asp Asp Ala Val
                50                  55                  60

Val Pro Leu Ala Arg Thr Gln Thr Cys Gly Asn Cys Thr Phe Arg
                65                  70                  75

Val Cys Phe Glu Gln Cys Cys Pro Trp Thr Phe Met Val Lys Leu
                80                  85                  90

Ile Asn Gln Asn Cys Asp Ser Ala Arg Thr Ser Asp Asp Arg Leu
                95                  100                 105

Cys Arg Ser Val Ser
                110

<210> 497
<211> 2089
<212> DNA
<213> Homo Sapien

<400> 497
tgaaggactt ttccaggacc caaggccaca cactggaagt cttgcagctg 50

aagggaggca ctccttggcc tccgcagccg atcacatgaa ggtggtgcca 100

agtctcctgc tctccgtcct cctggcacag gtgtggctgg tacccggctt 150

ggcccccagt cctcagtcgc cagagacccc agcccctcag aaccagacca 200

gcagggtagt gcaggctccc agggaggaag aggaagatga gcaggaggcc 250

agcgaggaga aggccggtga ggaagagaaa gcctggctga tggccagcag 300
gcagcagctt gccaaggaga cttcaaactt cggattcagc ctgctgcgaa 350
agatctccat gaggcacgat ggcaacatgg tcttctctcc atttggcatg 400
tccttggcca tgacaggctt gatgctgggg gccacagggc cgactgaaac 450
ccagatcaag agagggctcc acttgcaggc cctgaagccc accaagcccg 500
ggctcctgcc ttccctcttt aagggactca gagagaccct ctcccgcaac 550
ctggaactgg gcctctcaca ggggagtttt gccttcatcc acaaggattt 600
tgatgtcaaa gagactttct tcaatttatc caagaggtat tttgatacag 650
agtgcgtgcc tatgaatttt cgcaatgcct cacaggccaa aaggctcatg 700
aatcattaca ttaacaaaga gactcggggg aaaattccca aactgtttga 750
tgagattaat cctgaaacca aattaattct tgtggattac atcttgttca 800
aagggaaatg gttgacccca tttgaccctg tcttcaccga agtcgacact 850
ttccacctgg acaagtacaa gaccattaag gtgcccatga tgtacggtgc 900
aggcaagttt gcctccacct ttgacaagaa ttttcgttgt catgtcctca 950
aactgcccta ccaaggaaat gccaccatgc tggtggtcct catggagaaa 1000
atgggtgacc acctcgccct tgaagactac ctgaccacag acttggtgga 1050
gacatggctc agaaacatga aaaccagaaa catggaagtt ttctttccga 1100
agttcaagct agatcagaag tatgagatgc atgagctgct taggcagatg 1150
ggaatcagaa gaatcttctc accctttgct gaccttagtg aactctcagc 1200
tactggaaga aatctccaag tatccagggt tttacgaaga acagtgattg 1250
aagttgatga aaggggcact gaggcagtgg caggaatctt gtcagaaatt 1300
actgcttatt ccatgcctcc tgtcatcaaa gtggaccggc catttcattt 1350
catgatctat gaagaaacct ctggaatgct tctgtttctg ggcagggtgg 1400
tgaatccgac tctcctataa ttcaggacat gcataagcac ttcgtgctgt 1450
agtagatgct gaatctgagg tatcaaacac acacaggata ccagcaatgg 1500
atggcagggg agagtgttcc ttttgttctt aactagttta gggtgttctc 1550
aaataaatac agtagtcccc acttatctga gggggataca ttcaaagacc 1600
cccagcagat gcctgaaacg gtggacagtg ctgaacctta tatatatttt 1650
ttcctacaca tacataccta tgataaagtt taatttataa attaggcaca 1700
gtaagagatt aacaataata acaacattaa gtaaaatgag ttacttgaac 1750
gcaagcactg caataccata acagtcaaac tgattataga gaaggctact 1800
aagtgactca tgggcgagga gcatagacag tgtggagaca ttgggcaagg 1850

ggagaattca catcctgggt gggacagagc aggacgatgc aagattccat 1900

cccactactc agaatggcat gctgcttaag acttttagat tgtttatttc 1950

tggaattttt catttaatgt ttttggacca tggttgacca tggttaactg 2000

agactgcaga aagcaaaacc atggataagg gaggactact acaaaagcat 2050

taaattgata catatttttt aaaaaaaaaa aaaaaaaaa 2089

<210> 498
<211> 444
<212> PRT
<213> Homo Sapien

<400> 498
Met Lys Val Val Pro Ser Leu Leu Leu Ser Val Leu Leu Ala Gln
1 5 10 15

Val Trp Leu Val Pro Gly Leu Ala Pro Ser Pro Gln Ser Pro Glu
20 25 30

Thr Pro Ala Pro Gln Asn Gln Thr Ser Arg Val Val Gln Ala Pro
35 40 45

Arg Glu Glu Glu Glu Asp Glu Gln Glu Ala Ser Glu Glu Lys Ala
50 55 60

Gly Glu Glu Glu Lys Ala Trp Leu Met Ala Ser Arg Gln Gln Leu
65 70 75

Ala Lys Glu Thr Ser Asn Phe Gly Phe Ser Leu Leu Arg Lys Ile
80 85 90

Ser Met Arg His Asp Gly Asn Met Val Phe Ser Pro Phe Gly Met
95 100 105

Ser Leu Ala Met Thr Gly Leu Met Leu Gly Ala Thr Gly Pro Thr
110 115 120

Glu Thr Gln Ile Lys Arg Gly Leu His Leu Gln Ala Leu Lys Pro
125 130 135

Thr Lys Pro Gly Leu Leu Pro Ser Leu Phe Lys Gly Leu Arg Glu
140 145 150

Thr Leu Ser Arg Asn Leu Glu Leu Gly Leu Ser Gln Gly Ser Phe
155 160 165

Ala Phe Ile His Lys Asp Phe Asp Val Lys Glu Thr Phe Phe Asn
170 175 180

Leu Ser Lys Arg Tyr Phe Asp Thr Glu Cys Val Pro Met Asn Phe
185 190 195

Arg Asn Ala Ser Gln Ala Lys Arg Leu Met Asn His Tyr Ile Asn
200 205 210

Lys Glu Thr Arg Gly Lys Ile Pro Lys Leu Phe Asp Glu Ile Asn
215 220 225

Pro Glu Thr Lys Leu Ile Leu Val Asp Tyr Ile Leu Phe Lys Gly
230 235 240

Lys Trp Leu Thr Pro Phe Asp Pro Val Phe Thr Glu Val Asp Thr
245 250 255

Phe His Leu Asp Lys Tyr Lys Thr Ile Lys Val Pro Met Met Tyr
260 265 270

Gly Ala Gly Lys Phe Ala Ser Thr Phe Asp Lys Asn Phe Arg Cys
275 280 285

His Val Leu Lys Leu Pro Tyr Gln Gly Asn Ala Thr Met Leu Val
290 295 300

Val Leu Met Glu Lys Met Gly Asp His Leu Ala Leu Glu Asp Tyr
305 310 315

Leu Thr Thr Asp Leu Val Glu Thr Trp Leu Arg Asn Met Lys Thr
320 325 330

Arg Asn Met Glu Val Phe Phe Pro Lys Phe Lys Leu Asp Gln Lys
335 340 345

Tyr Glu Met His Glu Leu Leu Arg Gln Met Gly Ile Arg Arg Ile
350 355 360

Phe Ser Pro Phe Ala Asp Leu Ser Glu Leu Ser Ala Thr Gly Arg
365 370 375

Asn Leu Gln Val Ser Arg Val Leu Arg Arg Thr Val Ile Glu Val
380 385 390

Asp Glu Arg Gly Thr Glu Ala Val Ala Gly Ile Leu Ser Glu Ile
395 400 405

Thr Ala Tyr Ser Met Pro Pro Val Ile Lys Val Asp Arg Pro Phe
410 415 420

His Phe Met Ile Tyr Glu Glu Thr Ser Gly Met Leu Leu Phe Leu
425 430 435

Gly Arg Val Val Asn Pro Thr Leu Leu
440

<210> 499
<211> 693
<212> DNA
<213> Homo Sapien

<400> 499
ctagcctgcg ccaaggggta gtgagaccgc gcggcaacag cttgcggctg 50

cggggagctc ccgtgggcgc tccgctggct gtgcaggcgg ccatggattc 100

cttgcggaaa atgctgatct cagtcgcaat gctgggcgca ggggctggcg 150

tgggctacgc gctcctcgtt atcgtgaccc cgggagagcg gcggaagcag 200

gaaatgctaa aggagatgcc actgcaggac ccaaggagca gggaggaggc 250

ggccaggacc cagcagctat tgctggccac tctgcaggag gcagcgacca 300

cgcaggagaa cgtggcctgg aggaagaact ggatggttgg cggcgaaggc 350

ggcgccagcg ggaggtcacc gtgagaccgg acttgcctcc gtgggcgccg 400

gaccttggct tgggcgcagg aatccgaggc agcctttctc cttcgtgggc 450

ccagcggaga gtccggaccg agataccatg ccaggactct ccggggtcct 500

gtgagctgcc gtcgggtgag cacgtttccc ccaaaccctg gactgactgc 550

tttaaggtcc gcaaggcggg ccagggccga gacgcgagtc ggatgtggtg 600

aactgaaaga accaataaaa tcatgttcct ccaaaaaaaa aaaaaaaaaa 650

aaaaaaaaaa aaaaaaaaaa aaaaaaaaaa aaaaaaaaaa aaa 693

<210> 500
<211> 93
<212> PRT
<213> Homo Sapien

<400> 500
Met Asp Ser Leu Arg Lys Met Leu Ile Ser Val Ala Met Leu Gly
1               5                   10                  15

Ala Gly Ala Gly Val Gly Tyr Ala Leu Leu Val Ile Val Thr Pro
            20                  25                  30

Gly Glu Arg Arg Lys Gln Glu Met Leu Lys Glu Met Pro Leu Gln
            35                  40                  45

Asp Pro Arg Ser Arg Glu Glu Ala Ala Arg Thr Gln Gln Leu Leu
            50                  55                  60

Leu Ala Thr Leu Gln Glu Ala Ala Thr Thr Gln Glu Asn Val Ala
            65                  70                  75

Trp Arg Lys Asn Trp Met Val Gly Gly Glu Gly Gly Ala Ser Gly
            80                  85                  90

Arg Ser Pro

<210> 501
<211> 1883
<212> DNA
<213> Homo Sapien

<400> 501
caggagagaa ggcaccgccc ccacccccgcc tccaaagcta accctcgggc 50

ttgaggggaa gaggctgact gtacgttcct tctactctgg caccactctc 100

caggctgcca tggggcccag cacccctctc ctcatcttgt tccttttgtc 150

atggtcggga cccctccaag gacagcagca ccaccttgtg gagtacatgg 200

aacgccgact agctgcttta gaggaacggc tggcccagtg ccaggaccag 250

agtagtcggc atgctgctga gctgcgggac ttcaagaaca agatgctgcc 300

actgctggag gtggcagaga aggagcggga ggcactcaga actgaggccg 350

acaccatctc cgggagagtg gatcgtctgg agcgggaggt agactatctg 400

gagacccaga acccagctct gccctgtgta gagtttgatg agaaggtgac 450

tggaggccct gggaccaaag gcaagggaag aaggaatgag aagtacgata 500

tggtgacaga ctgtggctac acaatctctc aagtgagatc aatgaagatt 550
ctgaagcgat ttggtggccc agctggtcta tggaccaagg atccactggg 600
gcaaacagag aagatctacg tgttagatgg gacacagaat gacacagcct 650
ttgtcttccc aaggctgcgt gacttcaccc ttgccatggc tgcccggaaa 700
gcttcccgag tccgggtgcc cttcccctgg gtaggcacag ggcagctggt 750
atatggtggc tttctttatt ttgctcggag gcctcctgga agacctggtg 800
gaggtggtga gatggagaac actttgcagc taatcaaatt ccacctggca 850
aaccgaacag tggtggacag ctcagtattc ccagcagagg ggctgatccc 900
cccctacggc ttgacagcag acacctacat cgacctggta gctgatgagg 950
aaggtctttg ggctgtctat gccacccggg aggatgacag gcacttgtgt 1000
ctggccaagt tagatccaca gacactggac acagagcagc agtgggacac 1050
accatgtccc agagagaatg ctgaggctgc ctttgtcatc tgtgggaccc 1100
tctatgtcgt ctataacacc cgtcctgcca gtcgggcccg catccagtgc 1150
tcctttgatg ccagcggcac cctgacccct gaacgggcag cactccctta 1200
ttttccccgc agatatggtg cccatgccag cctccgctat aacccccgag 1250
aacgccagct ctatgcctgg gatgatggct accagattgt ctataagctg 1300
gagatgagga agaaagagga ggaggtttga ggagctagcc ttgtttttttg 1350
catctttctc actcccatac atttatatta tatccccact aaatttcttg 1400
ttcctcattc ttcaaatgtg ggccagttgt ggctcaaatc ctctatattt 1450
ttagccaatg gcaatcaaat tctttcagct cctttgtttc atacggaact 1500
ccagatcctg agtaatcctt ttagagcccg aagagtcaaa accctcaatg 1550
ttccctcctg ctctcctgcc ccatgtcaac aaatttcagg ctaaggatgc 1600
cccagaccca gggctctaac cttgtatgcg ggcaggccca gggagcaggc 1650
agcagtgttc ttcccctcag agtgacttgg ggagggagaa ataggaggag 1700
acgtccagct ctgtcctctc ttcctcactc ctcccttcag tgtcctgagg 1750
aacaggactt tctccacatt gttttgtatt gcaacatttt gcattaaaag 1800
gaaaatccac aaaaaaaaaa aaaaaaaaaa aaaaaaaaaa aaaaaaaaaa 1850
aaaaaaaaaa aaaaaaaaaa aaaaaaaaaa aaa 1883

<210> 502
<211> 406
<212> PRT
<213> Homo Sapien

<400> 502
Met Gly Pro Ser Thr Pro Leu Leu Ile Leu Phe Leu Leu Ser Trp

```
1               5                   10                  15
Ser Gly Pro Leu Gln Gly Gln Gln His His Leu Val Glu Tyr Met
                20                  25                  30
Glu Arg Arg Leu Ala Ala Leu Glu Glu Arg Leu Ala Gln Cys Gln
                35                  40                  45
Asp Gln Ser Ser Arg His Ala Ala Glu Leu Arg Asp Phe Lys Asn
                50                  55                  60
Lys Met Leu Pro Leu Leu Glu Val Ala Glu Lys Glu Arg Glu Ala
                65                  70                  75
Leu Arg Thr Glu Ala Asp Thr Ile Ser Gly Arg Val Asp Arg Leu
                80                  85                  90
Glu Arg Glu Val Asp Tyr Leu Glu Thr Gln Asn Pro Ala Leu Pro
                95                  100                 105
Cys Val Glu Phe Asp Glu Lys Val Thr Gly Gly Pro Gly Thr Lys
                110                 115                 120
Gly Lys Gly Arg Arg Asn Glu Lys Tyr Asp Met Val Thr Asp Cys
                125                 130                 135
Gly Tyr Thr Ile Ser Gln Val Arg Ser Met Lys Ile Leu Lys Arg
                140                 145                 150
Phe Gly Gly Pro Ala Gly Leu Trp Thr Lys Asp Pro Leu Gly Gln
                155                 160                 165
Thr Glu Lys Ile Tyr Val Leu Asp Gly Thr Gln Asn Asp Thr Ala
                170                 175                 180
Phe Val Phe Pro Arg Leu Arg Asp Phe Thr Leu Ala Met Ala Ala
                185                 190                 195
Arg Lys Ala Ser Arg Val Arg Val Pro Phe Pro Trp Val Gly Thr
                200                 205                 210
Gly Gln Leu Val Tyr Gly Gly Phe Leu Tyr Phe Ala Arg Arg Pro
                215                 220                 225
Pro Gly Arg Pro Gly Gly Gly Gly Glu Met Glu Asn Thr Leu Gln
                230                 235                 240
Leu Ile Lys Phe His Leu Ala Asn Arg Thr Val Val Asp Ser Ser
                245                 250                 255
Val Phe Pro Ala Glu Gly Leu Ile Pro Pro Tyr Gly Leu Thr Ala
                260                 265                 270
Asp Thr Tyr Ile Asp Leu Val Ala Asp Glu Glu Gly Leu Trp Ala
                275                 280                 285
Val Tyr Ala Thr Arg Glu Asp Asp Arg His Leu Cys Leu Ala Lys
                290                 295                 300
Leu Asp Pro Gln Thr Leu Asp Thr Glu Gln Gln Trp Asp Thr Pro
                305                 310                 315
Cys Pro Arg Glu Asn Ala Glu Ala Ala Phe Val Ile Cys Gly Thr
                320                 325                 330
```

```
Leu Tyr Val Val Tyr Asn Thr Arg Pro Ala Ser Arg Ala Arg Ile
                335                 340                 345

Gln Cys Ser Phe Asp Ala Ser Gly Thr Leu Thr Pro Glu Arg Ala
                350                 355                 360

Ala Leu Pro Tyr Phe Pro Arg Arg Tyr Gly Ala His Ala Ser Leu
                365                 370                 375

Arg Tyr Asn Pro Arg Glu Arg Gln Leu Tyr Ala Trp Asp Asp Gly
                380                 385                 390

Tyr Gln Ile Val Tyr Lys Leu Glu Met Arg Lys Lys Glu Glu Glu
                395                 400                 405

Val
```

<210> 503
<211> 689
<212> DNA
<213> Homo Sapien

<400> 503
```
tgcggcgcag tgtagacctg ggaggatggg cggcctgctg ctggctgctt 50

ttctggcttt ggtctcggtg cccagggccc aggccgtgtg gttgggaaga 100

ctggaccctg agcagcttct tgggccctgg tacgtgcttg cggtggcctc 150

ccgggaaaag ggctttgcca tggagaagga catgaagaac gtcgtggggg 200

tggtggtgac cctcactcca gaaaacaacc tgcggacgct gtcctctcag 250

cacgggctgg gagggtgtga ccagagtgtc atggacctga taaagcgaaa 300

ctccggatgg gtgtttgaga atccctcaat aggcgtgctg gagctctggg 350

tgctggccac caacttcaga gactatgcca tcatcttcac tcagctggag 400

ttcggggacg agcccttcaa caccgtggag ctgtacagtc tgacggagac 450

agccagccag gaggccatgg ggctcttcac caagtggagc aggagcctgg 500

gcttcctgtc acagtagcag gcccagctgc agaaggacct cacctgtgct 550

cacaagatcc ttctgtgagt gctgcgtccc cagtagggat ggcgcccaca 600

gggtcctgtg acctcggcca gtgtccaccc acctcgctca gcggctcccg 650

gggcccagca ccagctcaga ataaagcgat tccacagca 689
```

<210> 504
<211> 163
<212> PRT
<213> Homo Sapien

<400> 504
```
Met Gly Gly Leu Leu Leu Ala Ala Phe Leu Ala Leu Val Ser Val
  1               5                  10                  15

Pro Arg Ala Gln Ala Val Trp Leu Gly Arg Leu Asp Pro Glu Gln
                 20                  25                  30
```

```
Leu Leu Gly Pro Trp Tyr Val Leu Ala Val Ala Ser Arg Glu Lys
                35                  40                  45

Gly Phe Ala Met Glu Lys Asp Met Lys Asn Val Val Gly Val Val
                50                  55                  60

Val Thr Leu Thr Pro Glu Asn Asn Leu Arg Thr Leu Ser Ser Gln
                65                  70                  75

His Gly Leu Gly Gly Cys Asp Gln Ser Val Met Asp Leu Ile Lys
                80                  85                  90

Arg Asn Ser Gly Trp Val Phe Glu Asn Pro Ser Ile Gly Val Leu
                95                  100                 105

Glu Leu Trp Val Leu Ala Thr Asn Phe Arg Asp Tyr Ala Ile Ile
                110                 115                 120

Phe Thr Gln Leu Glu Phe Gly Asp Glu Pro Phe Asn Thr Val Glu
                125                 130                 135

Leu Tyr Ser Leu Thr Glu Thr Ala Ser Gln Glu Ala Met Gly Leu
                140                 145                 150

Phe Thr Lys Trp Ser Arg Ser Leu Gly Phe Leu Ser Gln
                155                 160
```

<210> 505
<211> 1204
<212> DNA
<213> Homo Sapien

<400> 505

```
gttccgcaga tgcagaggtt gaggtggctg cgggactgga agtcatcggg 50

cagaggtctc acagcagcca aggaacctgg ggcccgctcc tccccccctcc 100

aggccatgag gattctgcag ttaatcctgc ttgctctggc aacagggctt 150

gtagggggag agaccaggat catcaagggg ttcgagtgca agcctcactc 200

ccagccctgg caggcagccc tgttcgagaa gacgcggcta ctctgtgggg 250

cgacgctcat cgcccccaga tggctcctga cagcagccca ctgcctcaag 300

ccccgctaca tagttcacct ggggcagcac aacctccaga aggaggaggg 350

ctgtgagcag acccggacag ccactgagtc cttcccccac cccggcttca 400

acaacagcct ccccaacaaa gaccaccgca atgacatcat gctggtgaag 450

atggcatcgc cagtctccat cacctgggct gtgcgacccc tcaccctctc 500

ctcacgctgt gtcactgctg gcaccagctg cctcatttcc ggctggggca 550

gcacgtccag cccccagtta cgcctgcctc acaccttgcg atgcgccaac 600

atcaccatca ttgagcacca gaagtgtgag aacgcctacc ccggcaacat 650

cacagacacc atggtgtgtg ccagcgtgca ggaagggggc aaggactcct 700

gccagggtga ctccgggggc cctctggtct gtaaccagtc tcttcaaggc 750
```

attatctcct ggggccagga tccgtgtgcg atcacccgaa agcctggtgt 800

ctacacgaaa gtctgcaaat atgtggactg gatccaggag acgatgaaga 850

acaattagac tggacccacc caccacagcc catcaccctc catttccact 900

tggtgtttgg ttcctgttca ctctgttaat aagaaaccct aagccaagac 950

cctctacgaa cattctttgg gcctcctgga ctacaggaga tgctgtcact 1000

taataatcaa cctggggttc gaaatcagtg agacctggat tcaaattctg 1050

ccttgaaata ttgtgactct gggaatgaca acacctggtt tgttctctgt 1100

tgtatcccca gccccaaaga cagctcctgg ccatatatca aggtttcaat 1150

aaatatttgc taaatgaaaa aaaaaaaaaa aaaaaaaaaa aaaaaaaaaa 1200

aaaa 1204

<210> 506
<211> 250
<212> PRT
<213> Homo Sapien

<400> 506
Met Arg Ile Leu Gln Leu Ile Leu Leu Ala Leu Ala Thr Gly Leu
1 5 10 15

Val Gly Gly Glu Thr Arg Ile Ile Lys Gly Phe Glu Cys Lys Pro
20 25 30

His Ser Gln Pro Trp Gln Ala Ala Leu Phe Glu Lys Thr Arg Leu
35 40 45

Leu Cys Gly Ala Thr Leu Ile Ala Pro Arg Trp Leu Leu Thr Ala
50 55 60

Ala His Cys Leu Lys Pro Arg Tyr Ile Val His Leu Gly Gln His
65 70 75

Asn Leu Gln Lys Glu Glu Gly Cys Glu Gln Thr Arg Thr Ala Thr
80 85 90

Glu Ser Phe Pro His Pro Gly Phe Asn Asn Ser Leu Pro Asn Lys
95 100 105

Asp His Arg Asn Asp Ile Met Leu Val Lys Met Ala Ser Pro Val
110 115 120

Ser Ile Thr Trp Ala Val Arg Pro Leu Thr Leu Ser Ser Arg Cys
125 130 135

Val Thr Ala Gly Thr Ser Cys Leu Ile Ser Gly Trp Gly Ser Thr
140 145 150

Ser Ser Pro Gln Leu Arg Leu Pro His Thr Leu Arg Cys Ala Asn
155 160 165

Ile Thr Ile Ile Glu His Gln Lys Cys Glu Asn Ala Tyr Pro Gly
170 175 180

Asn Ile Thr Asp Thr Met Val Cys Ala Ser Val Gln Glu Gly Gly
185 190 195

Lys Asp Ser Cys Gln Gly Asp Ser Gly Gly Pro Leu Val Cys Asn
200 205 210

Gln Ser Leu Gln Gly Ile Ile Ser Trp Gly Gln Asp Pro Cys Ala
215 220 225

Ile Thr Arg Lys Pro Gly Val Tyr Thr Lys Val Cys Lys Tyr Val
230 235 240

Asp Trp Ile Gln Glu Thr Met Lys Asn Asn
245 250

<210> 507
<211> 636
<212> DNA
<213> Homo Sapien

<400> 507
ctgggatcag ccactgcagc tccctgagca ctctctacag agacgcggac 50

cccagacatg aggaggctcc tcctggtcac cagcctggtg gttgtgctgc 100

tgtgggaggc aggtgcagtc ccagcaccca aggtccctat caagatgcaa 150

gtcaaacact ggccctcaga gcaggaccca gagaaggcct ggggcgcccg 200

tgtggtggag cctccggaga aggacgacca gctggtggtg ctgttccctg 250

tccagaagcc gaaactcttg accaccgagg agaagccacg aggtcagggc 300

aggggcccca tccttccagg caccaaggcc tggatggaga ccgaggacac 350

cctgggccgt gtcctgagtc ccgagcccga ccatgacagc ctgtaccacc 400

ctccgcctga ggaggaccag ggcgaggaga ggccccggtt gtgggtgatg 450

ccaaatcacc aggtgctcct gggaccggag gaagaccaag accacatcta 500

ccacccccag tagggctcca ggggccatca ctgcccccgc cctgtcccaa 550

ggcccaggct gttgggactg ggaccctccc taccctgccc cagctagaca 600

aataaacccc agcaggcaaa aaaaaaaaaa aaaaaa 636

<210> 508
<211> 151
<212> PRT
<213> Homo Sapien

<400> 508
Met Arg Arg Leu Leu Leu Val Thr Ser Leu Val Val Val Leu Leu
1 5 10 15

Trp Glu Ala Gly Ala Val Pro Ala Pro Lys Val Pro Ile Lys Met
20 25 30

Gln Val Lys His Trp Pro Ser Glu Gln Asp Pro Glu Lys Ala Trp
35 40 45

Gly Ala Arg Val Val Glu Pro Pro Glu Lys Asp Asp Gln Leu Val
50 55 60

Val Leu Phe Pro Val Gln Lys Pro Lys Leu Leu Thr Thr Glu Glu

65 70 75

Lys Pro Arg Gly Gln Gly Arg Gly Pro Ile Leu Pro Gly Thr Lys
80 85 90

Ala Trp Met Glu Thr Glu Asp Thr Leu Gly Arg Val Leu Ser Pro
95 100 105

Glu Pro Asp His Asp Ser Leu Tyr His Pro Pro Pro Glu Glu Asp
110 115 120

Gln Gly Glu Glu Arg Pro Arg Leu Trp Val Met Pro Asn His Gln
125 130 135

Val Leu Leu Gly Pro Glu Glu Asp Gln Asp His Ile Tyr His Pro
140 145 150

Gln

<210> 509
<211> 1281
<212> DNA
<213> Homo Sapien

<400> 509
gcggagccgg cgccggctgc gcagaggagc cgctctcgcc gccgccacct 50

cggctgggag cccacgaggc tgccgcatcc tgccctcgga acaatgggac 100

tcggcgcgcg aggtgcttgg gccgcgctgc tcctggggac gctgcaggtg 150

ctagcgctgc tgggggccgc ccatgaaagc gcagccatgg cggcatctgc 200

aaacatagag aattctgggc ttccacacaa ctccagtgct aactcaacag 250

agactctcca acatgtgcct tctgaccata caaatgaaac ttccaacagt 300

actgtgaaac caccaacttc agttgcctca gactccagta atacaacggt 350

caccaccatg aaacctacag cggcatctaa tacaacaaca ccagggatgg 400

tctcaacaaa tatgacttct accaccttaa agtctacacc caaaacaaca 450

agtgtttcac agaacacatc tcagatatca acatccacaa tgaccgtaac 500

ccacaatagt tcagtgacat ctgctgcttc atcagtaaca atcacaacaa 550

ctatgcattc tgaagcaaag aaaggatcaa aatttgatac tgggagcttt 600

gttggtggta ttgtattaac gctgggagtt ttatctattc tttacattgg 650

atgcaaaatg tattactcaa gaagaggcat tcggtatcga accatagatg 700

aacatgatgc catcatttaa ggaaatccat ggaccaagga tggaatacag 750

attgatgctg ccctatcaat taattttggt ttattaatag tttaaaacaa 800

tattctcttt ttgaaaatag tataaacagg ccatgcatat aatgtacagt 850

gtattacgta aatatgtaaa gattcttcaa ggtaacaagg gtttgggttt 900

tgaaataaac atctggatct tatagaccgt tcatacaatg gttttagcaa 950

gttcatagta agacaaacaa gtcctatctt ttttttttgg ctggggtggg 1000

ggcattggtc acatatgacc agtaattgaa agacgtcatc actgaaagac 1050

agaatgccat ctgggcatac aaataagaag tttgtcacag cactcaggat 1100

tttgggtatc ttttgtagct cacataaaga acttcagtgc ttttcagagc 1150

tggatatatc ttaattacta atgccacaca gaaattatac aatcaaacta 1200

gatctgaagc ataatttaag aaaaacatca acatttttttg tgctttaaac 1250

tgtagtagtt ggtctagaaa caaaatactc c 1281

<210> 510
<211> 208
<212> PRT
<213> Homo Sapien

<400> 510
Met Gly Leu Gly Ala Arg Gly Ala Trp Ala Ala Leu Leu Leu Gly
1 5 10 15

Thr Leu Gln Val Leu Ala Leu Leu Gly Ala Ala His Glu Ser Ala
20 25 30

Ala Met Ala Ala Ser Ala Asn Ile Glu Asn Ser Gly Leu Pro His
35 40 45

Asn Ser Ser Ala Asn Ser Thr Glu Thr Leu Gln His Val Pro Ser
50 55 60

Asp His Thr Asn Glu Thr Ser Asn Ser Thr Val Lys Pro Pro Thr
65 70 75

Ser Val Ala Ser Asp Ser Ser Asn Thr Thr Val Thr Thr Met Lys
80 85 90

Pro Thr Ala Ala Ser Asn Thr Thr Thr Pro Gly Met Val Ser Thr
95 100 105

Asn Met Thr Ser Thr Thr Leu Lys Ser Thr Pro Lys Thr Thr Ser
110 115 120

Val Ser Gln Asn Thr Ser Gln Ile Ser Thr Ser Thr Met Thr Val
125 130 135

Thr His Asn Ser Ser Val Thr Ser Ala Ala Ser Ser Val Thr Ile
140 145 150

Thr Thr Thr Met His Ser Glu Ala Lys Lys Gly Ser Lys Phe Asp
155 160 165

Thr Gly Ser Phe Val Gly Gly Ile Val Leu Thr Leu Gly Val Leu
170 175 180

Ser Ile Leu Tyr Ile Gly Cys Lys Met Tyr Tyr Ser Arg Arg Gly
185 190 195

Ile Arg Tyr Arg Thr Ile Asp Glu His Asp Ala Ile Ile
200 205

<210> 511
<211> 2668

<212> DNA
<213> Homo Sapien

<400> 511
gactttgctt gaatgtttac attttctgct cgctgtccta catatcacaa 50

tatagtgttc acgttttgtt aaaactttgg ggtgtcagga gttgagcttg 100

ctcagcaagc cagcatggct aggatgagct ttgttatagc agcttgccaa 150

ttggtgctgg gcctactaat gacttcatta accgagtctt ccatacagaa 200

tagtgagtgt ccacaacttt gcgtatgtga aattcgtccc tggtttaccc 250

cacagtcaac ttacagagaa gccaccactg ttgattgcaa tgacctccgc 300

ttaacaagga ttcccagtaa cctctctagt gacacacaag tgcttctctt 350

acagagcaat aacatcgcga agactgtgga tgagctgcag cagcttttca 400

acttgactga actagatttc tcccaaaaca actttactaa cattaaggag 450

gtcgggctgg caaacctaac ccagctcaca acgctgcatt tggaggaaaa 500

tcagattacc gagatgactg attactgtct acaagacctc agcaaccttc 550

aagaactcta catcaaccac aaccaaatta gcactatttc tgctcatgct 600

tttgcaggct taaaaaatct attaaggctc cacctgaact ccaacaaatt 650

gaaagttatt gatagtcgct ggtttgattc tacacccaac ctggaaattc 700

tcatgatcgg agaaaaccct gtgattggaa ttctggatat gaacttcaaa 750

cccctcgcaa atttgagaag cttagttttg gcaggaatgt atctcactga 800

tattcctgga aatgctttgg tgggtctgga tagccttgag agcctgtctt 850

tttatgataa caaactggtt aaagtccctc aacttgccct gcaaaaagtt 900

ccaaatttga aattcttaga cctcaacaaa aaccccattc acaaaatcca 950

agaaggggac ttcaaaaata tgcttcggtt aaaagaactg ggaatcaaca 1000

atatgggcga gctcgtttct gtcgaccgct atgccctgga taacttgcct 1050

gaactcacaa agctggaagc caccaataac cctaaactct cttacatcca 1100

ccgcttggct ttccgaagtg tccctgctct ggaaagcttg atgctgaaca 1150

acaatgcctt gaatgccatt taccaaaaga cagtcgaatc cctccccaat 1200

ctgcgtgaga tcagtatcca tagcaatccc ctcaggtgtg actgtgtgat 1250

ccactggatt aactccaaca aaaccaacat ccgcttcatg gagcccctgt 1300

ccatgttctg tgccatgccg cccgaatata aagggcacca ggtgaaggaa 1350

gttttaatcc aggattcgag tgaacagtgc ctcccaatga tatctcacga 1400

cagcttccca aatcgtttaa acgtggatat cggcacgacg gttttcctag 1450

actgtcgagc catggctgag ccagaacctg aaatttactg ggtcactccc 1500

attggaaata agataactgt ggaaaccctt tcagataaat acaagctaag 1550

tagcgaaggt accttggaaa tatctaacat acaaattgaa gactcaggaa 1600

gatacacatg tgttgcccag aatgtccaag ggcagacac tcgggtggca 1650

acaattaagg ttaacgggac ccttctggat ggtacccagg tgctaaaat 1700

atacgtcaag cagacagaat cccattccat cttagtgtcc tggaaagtta 1750

attccaatgt catgacgtca aacttaaaat ggtcgtctgc caccatgaag 1800

attgataacc ctcacataac atatactgcc agggtcccag tcgatgtcca 1850

tgaatacaac ctaacgcatc tgcagccttc cacagattat gaagtgtgtc 1900

tcacagtgtc caatattcat cagcagactc aaaagtcatg cgtaaatgtc 1950

acaaccaaaa atgccgcctt cgcagtggac atctctgatc aagaaaccag 2000

tacagccctt gctgcagtaa tggggtctat gtttgccgtc attagccttg 2050

cgtccattgc tgtgtacttt gccaaaagat ttaagagaaa aaactaccac 2100

cactcattaa aaaagtatat gcaaaaaacc tcttcaatcc cactaaatga 2150

gctgtaccca ccactcatta acctctggga aggtgacagc gagaaagaca 2200

aagatggttc tgcagacacc aagccaaccc aggtcgacac atccagaagc 2250

tattacatgt ggtaactcag aggatatttt gcttctggta gtaaggagca 2300

caaagacgtt tttgctttat tctgcaaaag tgaacaagtt gaagactttt 2350

gtatttttga ctttgctagt ttgtggcaga gtggagagga cgggtggata 2400

tttcaaattt ttttagtata gcgtatcgca agggtttgac acggctgcca 2450

gcgactctag gcttccagtc tgtgtttggt ttttattctt atcattatta 2500

tgattgttat tatattatta ttttatttta gttgttgtgc taaactcaat 2550

aatgctgttc taactacagt gctcaataaa atgattaatg acaggaaaaa 2600

aaaaaaaaaa aaaaaaaaaa aaaaaaaaaa aaaaaaaaaa aaaaaaaaaa 2650

aaaaaaaaaa aaaaaaaa 2668

<210> 512
<211> 716
<212> PRT
<213> Homo Sapien

<400> 512
Met Ala Arg Met Ser Phe Val Ile Ala Ala Cys Gln Leu Val Leu
1 5 10 15

Gly Leu Leu Met Thr Ser Leu Thr Glu Ser Ser Ile Gln Asn Ser
20 25 30

Glu Cys Pro Gln Leu Cys Val Cys Glu Ile Arg Pro Trp Phe Thr
35 40 45

Pro Gln Ser Thr Tyr Arg Glu Ala Thr Thr Val Asp Cys Asn Asp

```
                50                  55                  60
Leu Arg Leu Thr Arg Ile Pro Ser Asn Leu Ser Ser Asp Thr Gln
                65                  70                  75
Val Leu Leu Leu Gln Ser Asn Asn Ile Ala Lys Thr Val Asp Glu
                80                  85                  90
Leu Gln Gln Leu Phe Asn Leu Thr Glu Leu Asp Phe Ser Gln Asn
                95                  100                 105
Asn Phe Thr Asn Ile Lys Glu Val Gly Leu Ala Asn Leu Thr Gln
                110                 115                 120
Leu Thr Thr Leu His Leu Glu Glu Asn Gln Ile Thr Glu Met Thr
                125                 130                 135
Asp Tyr Cys Leu Gln Asp Leu Ser Asn Leu Gln Glu Leu Tyr Ile
                140                 145                 150
Asn His Asn Gln Ile Ser Thr Ile Ser Ala His Ala Phe Ala Gly
                155                 160                 165
Leu Lys Asn Leu Leu Arg Leu His Leu Asn Ser Asn Lys Leu Lys
                170                 175                 180
Val Ile Asp Ser Arg Trp Phe Asp Ser Thr Pro Asn Leu Glu Ile
                185                 190                 195
Leu Met Ile Gly Glu Asn Pro Val Ile Gly Ile Leu Asp Met Asn
                200                 205                 210
Phe Lys Pro Leu Ala Asn Leu Arg Ser Leu Val Leu Ala Gly Met
                215                 220                 225
Tyr Leu Thr Asp Ile Pro Gly Asn Ala Leu Val Gly Leu Asp Ser
                230                 235                 240
Leu Glu Ser Leu Ser Phe Tyr Asp Asn Lys Leu Val Lys Val Pro
                245                 250                 255
Gln Leu Ala Leu Gln Lys Val Pro Asn Leu Lys Phe Leu Asp Leu
                260                 265                 270
Asn Lys Asn Pro Ile His Lys Ile Gln Glu Gly Asp Phe Lys Asn
                275                 280                 285
Met Leu Arg Leu Lys Glu Leu Gly Ile Asn Asn Met Gly Glu Leu
                290                 295                 300
Val Ser Val Asp Arg Tyr Ala Leu Asp Asn Leu Pro Glu Leu Thr
                305                 310                 315
Lys Leu Glu Ala Thr Asn Asn Pro Lys Leu Ser Tyr Ile His Arg
                320                 325                 330
Leu Ala Phe Arg Ser Val Pro Ala Leu Glu Ser Leu Met Leu Asn
                335                 340                 345
Asn Asn Ala Leu Asn Ala Ile Tyr Gln Lys Thr Val Glu Ser Leu
                350                 355                 360
Pro Asn Leu Arg Glu Ile Ser Ile His Ser Asn Pro Leu Arg Cys
                365                 370                 375
```

```
Asp Cys Val Ile His Trp Ile Asn Ser Asn Lys Thr Asn Ile Arg
                380                 385                 390

Phe Met Glu Pro Leu Ser Met Phe Cys Ala Met Pro Pro Glu Tyr
                395                 400                 405

Lys Gly His Gln Val Lys Glu Val Leu Ile Gln Asp Ser Ser Glu
                410                 415                 420

Gln Cys Leu Pro Met Ile Ser His Asp Ser Phe Pro Asn Arg Leu
                425                 430                 435

Asn Val Asp Ile Gly Thr Thr Val Phe Leu Asp Cys Arg Ala Met
                440                 445                 450

Ala Glu Pro Glu Pro Glu Ile Tyr Trp Val Thr Pro Ile Gly Asn
                455                 460                 465

Lys Ile Thr Val Glu Thr Leu Ser Asp Lys Tyr Lys Leu Ser Ser
                470                 475                 480

Glu Gly Thr Leu Glu Ile Ser Asn Ile Gln Ile Glu Asp Ser Gly
                485                 490                 495

Arg Tyr Thr Cys Val Ala Gln Asn Val Gln Gly Ala Asp Thr Arg
                500                 505                 510

Val Ala Thr Ile Lys Val Asn Gly Thr Leu Leu Asp Gly Thr Gln
                515                 520                 525

Val Leu Lys Ile Tyr Val Lys Gln Thr Glu Ser His Ser Ile Leu
                530                 535                 540

Val Ser Trp Lys Val Asn Ser Asn Val Met Thr Ser Asn Leu Lys
                545                 550                 555

Trp Ser Ser Ala Thr Met Lys Ile Asp Asn Pro His Ile Thr Tyr
                560                 565                 570

Thr Ala Arg Val Pro Val Asp Val His Glu Tyr Asn Leu Thr His
                575                 580                 585

Leu Gln Pro Ser Thr Asp Tyr Glu Val Cys Leu Thr Val Ser Asn
                590                 595                 600

Ile His Gln Gln Thr Gln Lys Ser Cys Val Asn Val Thr Thr Lys
                605                 610                 615

Asn Ala Ala Phe Ala Val Asp Ile Ser Asp Gln Glu Thr Ser Thr
                620                 625                 630

Ala Leu Ala Ala Val Met Gly Ser Met Phe Ala Val Ile Ser Leu
                635                 640                 645

Ala Ser Ile Ala Val Tyr Phe Ala Lys Arg Phe Lys Arg Lys Asn
                650                 655                 660

Tyr His His Ser Leu Lys Lys Tyr Met Gln Lys Thr Ser Ser Ile
                665                 670                 675

Pro Leu Asn Glu Leu Tyr Pro Pro Leu Ile Asn Leu Trp Glu Gly
                680                 685                 690
```

Asp Ser Glu Lys Asp Lys Asp Gly Ser Ala Asp Thr Lys Pro Thr
695 700 705

Gln Val Asp Thr Ser Arg Ser Tyr Tyr Met Trp
710 715

<210> 513
<211> 957
<212> DNA
<213> Homo Sapien

<400> 513
gggagagagg ataaatagca gcgtggcttc cctggctcct ctctgcatcc 50

ttcccgacct tcccagcaat atgcatcttg cacgtctggt cggctcctgc 100

tccctccttc tgctactggg ggccctgtct ggatgggcgg ccagcgatga 150

ccccattgag aaggtcattg aagggatcaa ccgagggctg agcaatgcag 200

agagagaggt gggcaaggcc ctggatggca tcaacagtgg aatcacgcat 250

gccggaaggg aagtggagaa ggttttcaac ggacttagca acatggggag 300

ccacaccggc aaggagttgg acaaaggcgt ccaggggctc aaccacggca 350

tggacaaggt tgcccatgag atcaaccatg gtattggaca agcaggaaag 400

gaagcagaga agcttggcca tggggtcaac aacgctgctg gacaggccgg 450

gaaggaagca gacaaagcgg tccaagggtt ccacactggg gtccaccagg 500

ctgggaagga agcagagaaa cttggccaag ggtcaacca tgctgctgac 550

caggctggaa aggaagtgga gaagcttggc caaggtgccc accatgctgc 600

tggccaggcc gggaaggagc tgcagaatgc tcataatggg gtcaaccaag 650

ccagcaagga ggccaaccag ctgctgaatg gcaaccatca aagcggatct 700

tccagccatc aaggaggggc cacaaccacg ccgttagcct ctggggcctc 750

agtcaacacg cctttcatca accttcccgc cctgtggagg agcgtcgcca 800

acatcatgcc ctaaactggc atccggcctt gctgggagaa taatgtcgcc 850

gttgtcacat cagctgacat gacctggagg ggttggggt gggggacagg 900

tttctgaaat ccctgaaggg ggttgtactg ggatttgtga ataaacttga 950

tacacca 957

<210> 514
<211> 247
<212> PRT
<213> Homo Sapien

<400> 514
Met His Leu Ala Arg Leu Val Gly Ser Cys Ser Leu Leu Leu Leu
1 5 10 15

Leu Gly Ala Leu Ser Gly Trp Ala Ala Ser Asp Asp Pro Ile Glu
20 25 30

Lys Val Ile Glu Gly Ile Asn Arg Gly Leu Ser Asn Ala Glu Arg
35 40 45

Glu Val Gly Lys Ala Leu Asp Gly Ile Asn Ser Gly Ile Thr His
50 55 60

Ala Gly Arg Glu Val Glu Lys Val Phe Asn Gly Leu Ser Asn Met
65 70 75

Gly Ser His Thr Gly Lys Glu Leu Asp Lys Gly Val Gln Gly Leu
80 85 90

Asn His Gly Met Asp Lys Val Ala His Glu Ile Asn His Gly Ile
95 100 105

Gly Gln Ala Gly Lys Glu Ala Glu Lys Leu Gly His Gly Val Asn
110 115 120

Asn Ala Ala Gly Gln Ala Gly Lys Glu Ala Asp Lys Ala Val Gln
125 130 135

Gly Phe His Thr Gly Val His Gln Ala Gly Lys Glu Ala Glu Lys
140 145 150

Leu Gly Gln Gly Val Asn His Ala Ala Asp Gln Ala Gly Lys Glu
155 160 165

Val Glu Lys Leu Gly Gln Gly Ala His His Ala Ala Gly Gln Ala
170 175 180

Gly Lys Glu Leu Gln Asn Ala His Asn Gly Val Asn Gln Ala Ser
185 190 195

Lys Glu Ala Asn Gln Leu Leu Asn Gly Asn His Gln Ser Gly Ser
200 205 210

Ser Ser His Gln Gly Gly Ala Thr Thr Thr Pro Leu Ala Ser Gly
215 220 225

Ala Ser Val Asn Thr Pro Phe Ile Asn Leu Pro Ala Leu Trp Arg
230 235 240

Ser Val Ala Asn Ile Met Pro
245

<210> 515
<211> 1942
<212> DNA
<213> Homo Sapien

<400> 515
cccacgcgtc cgcccacgcg tccgggtgcc actcgcgcgc cggccgcgct 50

ccgggcttct cttttccctc cgacgcgcca cggctgccca gacattccgg 100

ctgccgggtc tggagagctc cccgaacccc tccgcggaga ggagcgaggc 150

ggcgccaggg tggcccccgg ggcgcgcttg gtctcggaga agcggggacg 200

aggccggagg atgagcgact gagggcgacg cgggcactga cgcgagttgg 250

ggccgcgact accggcagct gacagcgcga tgagcgactc cccagagacg 300

ccctagcccg gtgtgcgcgc caggcggagc gcgcaggtgg ggctgggctg 350

ttagtggtcc gccccacgcg ggtcgccggc cggcccagga tgggcgctgg 400
caacccgggc ccgcgcccgc cgctgctacc cctgcgcccg ctgcgagccc 450
ggcgtccggc ccgcgccctg cgctcatgga cggcggctcc cggctggcgg 500
cggcgcgccc ccgggctgtg aatgcgactc gcccctcggc cgcgctcccc 550
gcccgcccgc ccgccgggac gtggtagggg atgcccagct ccactgcgat 600
ggcagttggc gcgctctcca gttccctcct ggtcacctgc tgcctgatgg 650
tggctctgtg cagtccgagc atcccgctgg agaagctggc ccaggcacca 700
gagcagccgg gccaggagaa gcgtgagcac gccactcggg acggcccggg 750
gcgggtgaac gagctcgggc gcccggcgag ggacgagggc ggcagcggcc 800
gggactggaa gagcaagagc ggccgtgggc tcgccggccg tgagccgtgg 850
agcaagctga agcaggcctg ggtctcccag ggcgggggcg ccaaggccgg 900
ggatctgcag gtccggcccc gcggggacac cccgcaggcg gaagccctgg 950
ccgcagccgc ccaggacgcg attggcccgg aactcgcgcc cacgcccgag 1000
ccacccgagg agtacgtgta cccggactac cgtggcaagg gctgcgtgga 1050
cgagagcggc ttcgtgtacg cgatcgggga gaagttcgcg ccgggcccct 1100
cggcctgccc gtgcctgtgc accgaggagg ggccgctgtg cgcgcagccc 1150
gagtgcccga ggctgcaccc gcgctgcatc cacgtcgaca cgagccagtg 1200
ctgcccgcag tgcaaggaga ggaagaacta ctgcgagttc cggggcaaga 1250
cctatcagac tttggaggag ttcgtggtgt ctccatgcga gaggtgtcgc 1300
tgtgaagcca acggtgaggt gctatgcaca gtgtcagcgt gtccccagac 1350
ggagtgtgtg gaccctgtgt acgagcctga tcagtgctgt cccatctgca 1400
aaaatggtcc aaactgcttt gcagaaaccg cggtgatccc tgctggcaga 1450
gaagtgaaga ctgacgagtg caccatatgc cactgtactt atgaggaagg 1500
cacatggaga atcgagcggc aggccatgtg cacgagacat gaatgcaggc 1550
aaatgtagac gcttcccaga acacaaactc tgactttttc tagaacattt 1600
tactgatgtg aacattctag atgactctgg gaactatcag tcaaagaaga 1650
cttttgatga ggaataatgg aaaattgttg gtacttttcc ttttcttgat 1700
aacagttact acaacagaag gaaatggata tatttcaaaa catcaacaag 1750
aactttgggc ataaaatcct tctctaaata aatgtgctat tttcacagta 1800
agtacacaaa agtacactat tatatatcaa atgtatttct ataatccctc 1850
cattagagag cttatataag tgttttctat agatgcagat taaaaatgct 1900
gtgttgtcaa ccgtcaaaaa aaaaaaaaaa aaaaaaaaaa aa 1942

<210> 516
<211> 325
<212> PRT
<213> Homo Sapien

<400> 516
```
Met Pro Ser Ser Thr Ala Met Ala Val Gly Ala Leu Ser Ser Ser
  1               5                  10                  15

Leu Leu Val Thr Cys Cys Leu Met Val Ala Leu Cys Ser Pro Ser
                 20                  25                  30

Ile Pro Leu Glu Lys Leu Ala Gln Ala Pro Glu Gln Pro Gly Gln
                 35                  40                  45

Glu Lys Arg Glu His Ala Thr Arg Asp Gly Pro Gly Arg Val Asn
                 50                  55                  60

Glu Leu Gly Arg Pro Ala Arg Asp Glu Gly Gly Ser Gly Arg Asp
                 65                  70                  75

Trp Lys Ser Lys Ser Gly Arg Gly Leu Ala Gly Arg Glu Pro Trp
                 80                  85                  90

Ser Lys Leu Lys Gln Ala Trp Val Ser Gln Gly Gly Gly Ala Lys
                 95                 100                 105

Ala Gly Asp Leu Gln Val Arg Pro Arg Gly Asp Thr Pro Gln Ala
                110                 115                 120

Glu Ala Leu Ala Ala Ala Ala Gln Asp Ala Ile Gly Pro Glu Leu
                125                 130                 135

Ala Pro Thr Pro Glu Pro Pro Glu Glu Tyr Val Tyr Pro Asp Tyr
                140                 145                 150

Arg Gly Lys Gly Cys Val Asp Glu Ser Gly Phe Val Tyr Ala Ile
                155                 160                 165

Gly Glu Lys Phe Ala Pro Gly Pro Ser Ala Cys Pro Cys Leu Cys
                170                 175                 180

Thr Glu Glu Gly Pro Leu Cys Ala Gln Pro Glu Cys Pro Arg Leu
                185                 190                 195

His Pro Arg Cys Ile His Val Asp Thr Ser Gln Cys Cys Pro Gln
                200                 205                 210

Cys Lys Glu Arg Lys Asn Tyr Cys Glu Phe Arg Gly Lys Thr Tyr
                215                 220                 225

Gln Thr Leu Glu Glu Phe Val Val Ser Pro Cys Glu Arg Cys Arg
                230                 235                 240

Cys Glu Ala Asn Gly Glu Val Leu Cys Thr Val Ser Ala Cys Pro
                245                 250                 255

Gln Thr Glu Cys Val Asp Pro Val Tyr Glu Pro Asp Gln Cys Cys
                260                 265                 270

Pro Ile Cys Lys Asn Gly Pro Asn Cys Phe Ala Glu Thr Ala Val
                275                 280                 285
```

Ile Pro Ala Gly Arg Glu Val Lys Thr Asp Glu Cys Thr Ile Cys
290 295 300

His Cys Thr Tyr Glu Glu Gly Thr Trp Arg Ile Glu Arg Gln Ala
305 310 315

Met Cys Thr Arg His Glu Cys Arg Gln Met
320 325

<210> 517
<211> 1419
<212> DNA
<213> Homo Sapien

<400> 517
ggacaaccgt tgctgggtgt cccagggcct gaggcaggac ggtactccgc 50

tgacaccttc cctttcggcc ttgaggttcc cagcctggtg gccccaggac 100

gttccggtcg catggcagag tgctacggac gacgcctatg aagcccttag 150

tccttctagt tgcgcttttg ctatggcctt cgtctgtgcc ggcttatccg 200

agcataactg tgacacctga tgaagagcaa aacttgaatc attatataca 250

agttttagag aacctagtac gaagtgttcc ctctggggag ccaggtcgtg 300

agaaaaaatc taactctcca aaacatgttt attctatagc atcaaaggga 350

tcaaaattta aggagctagt tacacatgga gacgcttcaa ctgagaatga 400

tgttttaacc aatcctatca gtgaagaaac tacaactttc cctacaggag 450

gcttcacacc ggaaatagga aagaaaaaac acacggaaag taccccattc 500

tggtcgatca aaccaaacaa tgtttccatt gttttgcatg cagaggaacc 550

ttatattgaa aatgaagagc cagagccaga gccggagcca gctgcaaaac 600

aaactgaggc accaagaatg ttgccagttg ttactgaatc atctacaagt 650

ccatatgtta cctcatacaa gtcacctgtc accactttag ataagagcac 700

tggcattgag atctctacag aatcagaaga tgttcctcag ctctcaggtg 750

aaactgcgat agaaaaaccc gaagagtttg gaaagcaccc agagagttgg 800

aataatgatg acattttgaa aaaaatttta gatattaatt cacaagtgca 850

acaggcactt cttagtgaca ccagcaaccc agcatataga gaagatattg 900

aagcctctaa agatcaccta aaacgaagcc ttgctctagc agcagcagca 950

gaacataaat taaaaacaat gtataagtcc cagttattgc cagtaggacg 1000

aacaagtaat aaaattgatg acatcgaaac tgttattaac atgctgtgta 1050

attctagatc taaactctat gaatatttag atattaaatg tgttccacca 1100

gagatgagag aaaaagctgc tacagtattc aatacattaa aaaatatgtg 1150

tagatcaagg agagtcacag ccttattaaa agtttattaa acaataatat 1200

aaaaatttta aacctacttg atattccata acaaagctga tttaagcaaa 1250

ctgcattttt tcacaggaga aataatcata ttcgtaattt caaaagttgt 1300

ataaaaatat tttctattgt agttcaaatg tgccaacatc tttatgtgtc 1350

atgtgttatg aacaattttc atatgcacta aaaacctaat ttaaaataaa 1400

attttggttc aggaaaaaa 1419

<210> 518
<211> 350
<212> PRT
<213> Homo Sapien

<400> 518
Met Lys Pro Leu Val Leu Leu Val Ala Leu Leu Leu Trp Pro Ser
1 5 10 15

Ser Val Pro Ala Tyr Pro Ser Ile Thr Val Thr Pro Asp Glu Glu
20 25 30

Gln Asn Leu Asn His Tyr Ile Gln Val Leu Glu Asn Leu Val Arg
35 40 45

Ser Val Pro Ser Gly Glu Pro Gly Arg Glu Lys Lys Ser Asn Ser
50 55 60

Pro Lys His Val Tyr Ser Ile Ala Ser Lys Gly Ser Lys Phe Lys
65 70 75

Glu Leu Val Thr His Gly Asp Ala Ser Thr Glu Asn Asp Val Leu
80 85 90

Thr Asn Pro Ile Ser Glu Glu Thr Thr Thr Phe Pro Thr Gly Gly
95 100 105

Phe Thr Pro Glu Ile Gly Lys Lys Lys His Thr Glu Ser Thr Pro
110 115 120

Phe Trp Ser Ile Lys Pro Asn Asn Val Ser Ile Val Leu His Ala
125 130 135

Glu Glu Pro Tyr Ile Glu Asn Glu Glu Pro Glu Pro Glu Pro Glu
140 145 150

Pro Ala Ala Lys Gln Thr Glu Ala Pro Arg Met Leu Pro Val Val
155 160 165

Thr Glu Ser Ser Thr Ser Pro Tyr Val Thr Ser Tyr Lys Ser Pro
170 175 180

Val Thr Thr Leu Asp Lys Ser Thr Gly Ile Glu Ile Ser Thr Glu
185 190 195

Ser Glu Asp Val Pro Gln Leu Ser Gly Glu Thr Ala Ile Glu Lys
200 205 210

Pro Glu Glu Phe Gly Lys His Pro Glu Ser Trp Asn Asn Asp Asp
215 220 225

Ile Leu Lys Lys Ile Leu Asp Ile Asn Ser Gln Val Gln Gln Ala
230 235 240

Leu Leu Ser Asp Thr Ser Asn Pro Ala Tyr Arg Glu Asp Ile Glu

245 250 255

Ala Ser Lys Asp His Leu Lys Arg Ser Leu Ala Leu Ala Ala Ala
260 265 270

Ala Glu His Lys Leu Lys Thr Met Tyr Lys Ser Gln Leu Leu Pro
275 280 285

Val Gly Arg Thr Ser Asn Lys Ile Asp Asp Ile Glu Thr Val Ile
290 295 300

Asn Met Leu Cys Asn Ser Arg Ser Lys Leu Tyr Glu Tyr Leu Asp
305 310 315

Ile Lys Cys Val Pro Pro Glu Met Arg Glu Lys Ala Ala Thr Val
320 325 330

Phe Asn Thr Leu Lys Asn Met Cys Arg Ser Arg Arg Val Thr Ala
335 340 345

Leu Leu Lys Val Tyr
350

<210> 519
<211> 1630
<212> DNA
<213> Homo Sapien

<400> 519
cggctcgagt gcagctgtgg ggagatttca gtgcattgcc tcccctgggt 50

gctcttcatc ttggatttga aagttgagag cagcatgttt tgcccactga 100

aactcatcct gctgccagtg ttactggatt attccttggg cctgaatgac 150

ttgaatgttt ccccgcctga gctaacagtc catgtgggtg attcagctct 200

gatgggatgt gttttccaga gcacagaaga caaatgtata ttcaagatag 250

actggactct gtcaccagga gagcacgcca aggacgaata tgtgctatac 300

tattactcca atctcagtgt gcctattggg cgcttccaga accgcgtaca 350

cttgatgggg gacatcttat gcaatgatgg ctctctcctg ctccaagatg 400

tgcaagaggc tgaccaggga acctatatct gtgaaatccg cctcaaaggg 450

gagagccagg tgttcaagaa ggcggtggta ctgcatgtgc ttccagagga 500

gcccaaagag ctcatggtcc atgtgggtgg attgattcag atgggatgtg 550

ttttccagag cacagaagtg aaacacgtga ccaaggtaga atggatattt 600

tcaggacggc gcgcaaagga ggagattgta tttcgttact accacaaact 650

caggatgtct gtggagtact cccagagctg gggccacttc cagaatcgtg 700

tgaacctggt gggggacatt ttccgcaatg acggttccat catgcttcaa 750

ggagtgaggg agtcagatgg aggaaactac acctgcagta tccacctagg 800

gaacctggtg ttcaagaaaa ccattgtgct gcatgtcagc ccggaagagc 850

ctcgaacact ggtgaccccg gcagccctga ggcctctggt cttgggtggt 900

aatcagttgg tgatcattgt gggaattgtc tgtgccacaa tcctgctgct 950

ccctgttctg atattgatcg tgaagaagac ctgtggaaat aagagttcag 1000

tgaattctac agtcttggtg aagaacacga agaagactaa tccagagata 1050

aaagaaaaac cctgccattt tgaaagatgt gaaggggaga aacacattta 1100

ctccccaata attgtacggg aggtgatcga ggaagaagaa ccaagtgaaa 1150

aatcagaggc cacctacatg accatgcacc cagtttggcc ttctctgagg 1200

tcagatcgga acaactcact tgaaaaaaag tcaggtgggg gaatgccaaa 1250

aacacagcaa gccttttgag aagaatggag agtcccttca tctcagcagc 1300

ggtggagact ctctcctgtg tgtgtcctgg gccactctac cagtgatttc 1350

agactcccgc tctcccagct gtcctcctgt ctcattgttt ggtcaataca 1400

ctgaagatgg agaatttgga gcctggcaga gagactggac agctctggag 1450

gaacaggcct gctgagggga ggggagcatg gacttggcct ctggagtggg 1500

acactggccc tgggaaccag gctgagctga gtggcctcaa accccccgtt 1550

ggatcagacc ctcctgtggg cagggttctt agtggatgag ttactgggaa 1600

gaatcagaga taaaaaccaa cccaaatcaa 1630

<210> 520
<211> 394
<212> PRT
<213> Homo Sapien

<400> 520

```
Met Phe Cys Pro Leu Lys Leu Ile Leu Leu Pro Val Leu Leu Asp
  1               5                  10                  15

Tyr Ser Leu Gly Leu Asn Asp Leu Asn Val Ser Pro Pro Glu Leu
                 20                  25                  30

Thr Val His Val Gly Asp Ser Ala Leu Met Gly Cys Val Phe Gln
                 35                  40                  45

Ser Thr Glu Asp Lys Cys Ile Phe Lys Ile Asp Trp Thr Leu Ser
                 50                  55                  60

Pro Gly Glu His Ala Lys Asp Glu Tyr Val Leu Tyr Tyr Tyr Ser
                 65                  70                  75

Asn Leu Ser Val Pro Ile Gly Arg Phe Gln Asn Arg Val His Leu
                 80                  85                  90

Met Gly Asp Ile Leu Cys Asn Asp Gly Ser Leu Leu Leu Gln Asp
                 95                 100                 105

Val Gln Glu Ala Asp Gln Gly Thr Tyr Ile Cys Glu Ile Arg Leu
                110                 115                 120

Lys Gly Glu Ser Gln Val Phe Lys Lys Ala Val Val Leu His Val
                125                 130                 135
```

```
Leu Pro Glu Glu Pro Lys Glu Leu Met Val His Val Gly Gly Leu
                140                 145                 150

Ile Gln Met Gly Cys Val Phe Gln Ser Thr Glu Val Lys His Val
                155                 160                 165

Thr Lys Val Glu Trp Ile Phe Ser Gly Arg Arg Ala Lys Glu Glu
                170                 175                 180

Ile Val Phe Arg Tyr Tyr His Lys Leu Arg Met Ser Val Glu Tyr
                185                 190                 195

Ser Gln Ser Trp Gly His Phe Gln Asn Arg Val Asn Leu Val Gly
                200                 205                 210

Asp Ile Phe Arg Asn Asp Gly Ser Ile Met Leu Gln Gly Val Arg
                215                 220                 225

Glu Ser Asp Gly Gly Asn Tyr Thr Cys Ser Ile His Leu Gly Asn
                230                 235                 240

Leu Val Phe Lys Lys Thr Ile Val Leu His Val Ser Pro Glu Glu
                245                 250                 255

Pro Arg Thr Leu Val Thr Pro Ala Ala Leu Arg Pro Leu Val Leu
                260                 265                 270

Gly Gly Asn Gln Leu Val Ile Ile Val Gly Ile Val Cys Ala Thr
                275                 280                 285

Ile Leu Leu Leu Pro Val Leu Ile Leu Ile Val Lys Lys Thr Cys
                290                 295                 300

Gly Asn Lys Ser Ser Val Asn Ser Thr Val Leu Val Lys Asn Thr
                305                 310                 315

Lys Lys Thr Asn Pro Glu Ile Lys Glu Lys Pro Cys His Phe Glu
                320                 325                 330

Arg Cys Glu Gly Glu Lys His Ile Tyr Ser Pro Ile Ile Val Arg
                335                 340                 345

Glu Val Ile Glu Glu Glu Glu Pro Ser Glu Lys Ser Glu Ala Thr
                350                 355                 360

Tyr Met Thr Met His Pro Val Trp Pro Ser Leu Arg Ser Asp Arg
                365                 370                 375

Asn Asn Ser Leu Glu Lys Lys Ser Gly Gly Gly Met Pro Lys Thr
                380                 385                 390

Gln Gln Ala Phe
```

<210> 521
<211> 963
<212> DNA
<213> Homo Sapien

<400> 521
ctatgaagaa gcttcctgga aaacaataag caaaggaaaa caaatgtgtc 50

ccatctcaca tggttctacc ctactaaaga caggaagatc ataaactgac 100

agatactgaa attgtaagag ttggaaacta cattttgcaa agtcattgaa 150

ctctgagctc agttgcagta ctcgggaagc catgcaggat gaagatggat 200

acatcacctt aaatattaaa actcggaaac cagctctcgt ctccgttggc 250

cctgcatcct cctcctggtg gcgtgtgatg gctttgattc tgctgatcct 300

gtgcgtgggg atggttgtcg ggctggtggc tctggggatt tggtctgtca 350

tgcagcgcaa ttacctacaa gatgagaatg aaaatcgcac aggaactctg 400

caacaattag caaagcgctt ctgtcaatat gtggtaaaac aatcagaact 450

aaagggcact ttcaaaggtc ataaatgcag cccctgtgac acaaactgga 500

gatattatgg agatagctgc tatgggttct tcaggcacaa cttaacatgg 550

gaagagagta agcagtactg cactgacatg aatgctactc tcctgaagat 600

tgacaaccgg aacattgtgg agtacatcaa agccaggact catttaattc 650

gttgggtcgg attatctcgc cagaagtcga atgaggtctg gaagtgggag 700

gatggctcgg ttatctcaga aaatatgttt gagtttttgg aagatggaaa 750

aggaaatatg aattgtgctt attttcataa tgggaaaatg caccctacct 800

tctgtgagaa caaacattat ttaatgtgtg agaggaaggc tggcatgacc 850

aaggtggacc aactacctta atgcaaagag gtggacagga taacacagat 900

aagggcttta ttgtacaata aaagatatgt atgaatgcat cagtagctga 950

aaaaaaaaaa aaa 963

<210> 522
<211> 229
<212> PRT
<213> Homo Sapien

<400> 522

```
Met Gln Asp Glu Asp Gly Tyr Ile Thr Leu Asn Ile Lys Thr Arg
  1               5                  10                  15

Lys Pro Ala Leu Val Ser Val Gly Pro Ala Ser Ser Ser Trp Trp
                 20                  25                  30

Arg Val Met Ala Leu Ile Leu Leu Ile Leu Cys Val Gly Met Val
                 35                  40                  45

Val Gly Leu Val Ala Leu Gly Ile Trp Ser Val Met Gln Arg Asn
                 50                  55                  60

Tyr Leu Gln Asp Glu Asn Glu Asn Arg Thr Gly Thr Leu Gln Gln
                 65                  70                  75

Leu Ala Lys Arg Phe Cys Gln Tyr Val Val Lys Gln Ser Glu Leu
                 80                  85                  90

Lys Gly Thr Phe Lys Gly His Lys Cys Ser Pro Cys Asp Thr Asn
                 95                 100                 105

Trp Arg Tyr Tyr Gly Asp Ser Cys Tyr Gly Phe Phe Arg His Asn
```

```
                110                 115                 120

Leu Thr Trp Glu Glu Ser Lys Gln Tyr Cys Thr Asp Met Asn Ala
                125                 130                 135

Thr Leu Leu Lys Ile Asp Asn Arg Asn Ile Val Glu Tyr Ile Lys
                140                 145                 150

Ala Arg Thr His Leu Ile Arg Trp Val Gly Leu Ser Arg Gln Lys
                155                 160                 165

Ser Asn Glu Val Trp Lys Trp Glu Asp Gly Ser Val Ile Ser Glu
                170                 175                 180

Asn Met Phe Glu Phe Leu Glu Asp Gly Lys Gly Asn Met Asn Cys
                185                 190                 195

Ala Tyr Phe His Asn Gly Lys Met His Pro Thr Phe Cys Glu Asn
                200                 205                 210

Lys His Tyr Leu Met Cys Glu Arg Lys Ala Gly Met Thr Lys Val
                215                 220                 225

Asp Gln Leu Pro


<210> 523
<211> 1197
<212> DNA
<213> Homo Sapien

<400> 523
cagcagtggt ctctcagtcc tctcaaagca aggaaagagt actgtgtgct 50

gagagaccat ggcaaagaat cctccagaga attgtgaaga ctgtcacatt 100

ctaaatgcag aagcttttaa atccaagaaa atatgtaaat cacttaagat 150

ttgtggactg gtgtttggta tcctggccct aactctaatt gtcctgtttt 200

gggggagcaa gcacttctgg ccggaggtac ccaaaaaagc ctatgacatg 250

gagcacactt tctacagcaa tggagagaag aagaagattt acatggaaat 300

tgatcctgtg accagaactg aaatattcag aagcggaaat ggcactgatg 350

aaacattgga agtgcacgac tttaaaaacg gatacactgg catctacttc 400

gtgggtcttc aaaaatgttt tatcaaaact cagattaaag tgattcctga 450

attttctgaa ccagaagagg aaatagatga gaatgaagaa attaccacaa 500

ctttctttga acagtcagtg atttgggtcc cagcagaaaa gcctattgaa 550

aaccgagatt ttcttaaaaa ttccaaaatt ctggagattt gtgataacgt 600

gaccatgtat tggatcaatc ccactctaat atcagtttct gagttacaag 650

actttgagga ggagggagaa gatcttcact ttcctgccaa cgaaaaaaaa 700

gggattgaac aaaatgaaca gtgggtggtc cctcaagtga aagtagagaa 750

gacccgtcac gccagacaag caagtgagga agaacttcca ataaatgact 800
```

```
atactgaaaa tggaatagaa tttgatccca tgctggatga gagaggttat 850
tgttgtattt actgccgtcg aggcaaccgc tattgccgcc gcgtctgtga 900
acctttacta ggctactacc catatccata ctgctaccaa ggaggacgag 950
tcatctgtcg tgtcatcatg ccttgtaact ggtgggtggc ccgcatgctg 1000
gggagggtct aataggaggt ttgagctcaa atgcttaaac tgctggcaac 1050
atataataaa tgcatgctat tcaatgaatt tctgcctatg aggcatctgg 1100
cccctggtag ccagctctcc agaattactt gtaggtaatt cctctcttca 1150
tgttctaata aacttctaca ttatcaccaa aaaaaaaaaa aaaaaaa 1197
```

```
<210> 524
<211> 317
<212> PRT
<213> Homo Sapien

<400> 524
Met Ala Lys Asn Pro Pro Glu Asn Cys Glu Asp Cys His Ile Leu
1               5                   10                  15

Asn Ala Glu Ala Phe Lys Ser Lys Lys Ile Cys Lys Ser Leu Lys
                20                  25                  30

Ile Cys Gly Leu Val Phe Gly Ile Leu Ala Leu Thr Leu Ile Val
                35                  40                  45

Leu Phe Trp Gly Ser Lys His Phe Trp Pro Glu Val Pro Lys Lys
                50                  55                  60

Ala Tyr Asp Met Glu His Thr Phe Tyr Ser Asn Gly Glu Lys Lys
                65                  70                  75

Lys Ile Tyr Met Glu Ile Asp Pro Val Thr Arg Thr Glu Ile Phe
                80                  85                  90

Arg Ser Gly Asn Gly Thr Asp Glu Thr Leu Glu Val His Asp Phe
                95                  100                 105

Lys Asn Gly Tyr Thr Gly Ile Tyr Phe Val Gly Leu Gln Lys Cys
                110                 115                 120

Phe Ile Lys Thr Gln Ile Lys Val Ile Pro Glu Phe Ser Glu Pro
                125                 130                 135

Glu Glu Glu Ile Asp Glu Asn Glu Glu Ile Thr Thr Thr Phe Phe
                140                 145                 150

Glu Gln Ser Val Ile Trp Val Pro Ala Glu Lys Pro Ile Glu Asn
                155                 160                 165

Arg Asp Phe Leu Lys Asn Ser Lys Ile Leu Glu Ile Cys Asp Asn
                170                 175                 180

Val Thr Met Tyr Trp Ile Asn Pro Thr Leu Ile Ser Val Ser Glu
                185                 190                 195

Leu Gln Asp Phe Glu Glu Glu Gly Glu Asp Leu His Phe Pro Ala
                200                 205                 210
```

Asn Glu Lys Lys Gly Ile Glu Gln Asn Glu Gln Trp Val Val Pro
215 220 225

Gln Val Lys Val Glu Lys Thr Arg His Ala Arg Gln Ala Ser Glu
230 235 240

Glu Glu Leu Pro Ile Asn Asp Tyr Thr Glu Asn Gly Ile Glu Phe
245 250 255

Asp Pro Met Leu Asp Glu Arg Gly Tyr Cys Cys Ile Tyr Cys Arg
260 265 270

Arg Gly Asn Arg Tyr Cys Arg Arg Val Cys Glu Pro Leu Leu Gly
275 280 285

Tyr Tyr Pro Tyr Pro Tyr Cys Tyr Gln Gly Gly Arg Val Ile Cys
290 295 300

Arg Val Ile Met Pro Cys Asn Trp Trp Val Ala Arg Met Leu Gly
305 310 315

Arg Val

<210> 525
<211> 535
<212> DNA
<213> Homo Sapien

<400> 525
agtgacaatc tcagagcagc ttctacacca cagccatttc cagcatgaag 50

atcactgggg gtctccttct gctctgtaca gtggtctatt tctgtagcag 100

ctcagaagct gctagtctgt ctccaaaaaa agtggactgc agcatttaca 150

agaagtatcc agtggtggcc atcccctgcc ccatcacata cctaccagtt 200

tgtggttctg actacatcac ctatgggaat gaatgtcact tgtgtaccga 250

gagcttgaaa agtaatggaa gagttcagtt tcttcacgat ggaagttgct 300

aaattctcca tggacataga gagaaaggaa tgatattctc atcatcatct 350

tcatcatccc aggctctgac tgagtttctt tcagttttac tgatgttctg 400

ggtgggggac agagccagat tcagagtaat cttgactgaa tggagaaagt 450

ttctgtgcta cccctacaaa cccatgcctc actgacagac cagcattttt 500

tttttaacac gtcaataaaa aaataatctc ccaga 535

<210> 526
<211> 85
<212> PRT
<213> Homo Sapien

<400> 526
Met Lys Ile Thr Gly Gly Leu Leu Leu Leu Cys Thr Val Val Tyr
1 5 10 15

Phe Cys Ser Ser Ser Glu Ala Ala Ser Leu Ser Pro Lys Lys Val
20 25 30

Asp Cys Ser Ile Tyr Lys Lys Tyr Pro Val Val Ala Ile Pro Cys
35 40 45

Pro Ile Thr Tyr Leu Pro Val Cys Gly Ser Asp Tyr Ile Thr Tyr
50 55 60

Gly Asn Glu Cys His Leu Cys Thr Glu Ser Leu Lys Ser Asn Gly
65 70 75

Arg Val Gln Phe Leu His Asp Gly Ser Cys
80 85

<210> 527
<211> 2387
<212> DNA
<213> Homo Sapien

<400> 527
cgacgatgct acgcgcgccc ggctgcctcc tccggacctc cgtagcgcct 50

gccgcggccc tggctgcggc gctgctctcg tcgcttgcgc gctgctctct 100

tctagagccg agggacccgg tggcctcgtc gctcagcccc tatttcggca 150

ccaagactcg ctacgaggat gtcaaccccg tgctattgtc gggccccgag 200

gctccgtggc gggaccctga gctgctggag gggacctgca ccccggtgca 250

gctggtcgcc ctcattcgcc acggcacccg ctaccccacg gtcaaacaga 300

tccgcaagct gaggcagctg cacgggttgc tgcaggcccg cgggtccagg 350

gatggcgggg ctagtagtac cggcagccgc gacctgggtg cagcgctggc 400

cgactggcct ttgtggtacg cggactggat ggacgggcag ctagtagaga 450

agggacggca ggatatgcga cagctggcgc tgcgtctggc ctcgctcttc 500

ccggcccttt tcagccgtga gaactacggc cgcctgcggc tcatcaccag 550

ttccaagcac cgctgcatgg atagcagcgc cgccttcctg caggggctgt 600

ggcagcacta ccaccctggc ttgccgccgc cggacgtcgc agatatggag 650

tttggacctc caacagttaa tgataaacta atgagatttt ttgatcactg 700

tgagaagttt ttaactgaag tagaaaaaaa tgctacagct ctttatcacg 750

tggaagcctt caaaactgga ccagaaatgc agaacatttt aaaaaaagtt 800

gcagctactt tgcaagtgcc agtaaatgat ttaaatgcag atttaattca 850

agtagccttt ttcacctgtt catttgacct ggcaattaaa ggtgttaaat 900

ctccttggtg tgatgttttt gacatagatg atgcaaaggt attagaatat 950

ttaaatgatc tgaaacaata ttggaaaaga ggatatgggt atactattaa 1000

cagtcgatcc agctgcacct tgtttcagga tatctttcag cacttggaca 1050

aagcagttga acagaaacaa aggtctcagc caatttcttc tccagtcatc 1100

ctccagtttg gtcatgcaga gactcttctt ccactgcttt ctctcatggg 1150

ctacttcaaa gacaaggaac ccctaacagc gtacaattac aaaaaacaaa 1200
tgcatcggaa gttccgaagt ggtctcattg taccttatgc ctcgaacctg 1250
atatttgtgc tttaccactg tgaaaatgct aagactccta aagaacaatt 1300
ccgagtgcag atgttattaa atgaaaaggt gttacctttg gcttactcac 1350
aagaaactgt ttcattttat gaagatctga agaaccacta caaggacatc 1400
cttcagagtt gtcaaaccag tgaagaatgt gaattagcaa gggctaacag 1450
tacatctgat gaactatgag taactgaaga acatttttaa ttctttagga 1500
atctgcaatg agtgattaca tgcttgtaat aggtaggcaa ttccttgatt 1550
acaggaagct tttatattac ttgagtattt ctgtcttttc acagaaaaac 1600
attgggtttc tctctgggtt tggacatgaa atgtaagaaa agatttttca 1650
ctggagcagc tctcttaagg agaaacaaat ctatttagag aaacagctgg 1700
ccctgcaaat gtttacagaa atgaaattct tcctacttat ataagaaatc 1750
tcacactgag atagaattgt gatttcataa taacacttga aaagtgctgg 1800
agtaacaaaa tatctcagtt ggaccatcct taacttgatt gaactgtcta 1850
ggaactttac agattgttct gcagttctct cttcttttcc tcaggtagga 1900
cagctctagc attttcttaa tcaggaatat tgtggtaagc tgggagtatc 1950
actctggaag aaagtaacat ctccagatga gaatttgaaa caagaaacag 2000
agtgttgtaa aaggacacct tcactgaagc aagtcggaaa gtacaatgaa 2050
aataaatatt tttggtattt atttatgaaa tatttgaaca ttttttcaat 2100
aattcctttt tacttctagg aagtctcaaa agaccatctt aaattattat 2150
atgtttggac aattagcaac aagtcagata gttagaatcg aagtttttca 2200
aatccattgc ttagctaact ttttcattct gtcacttggc ttcgattttt 2250
atattttcct attatatgaa atgtatcttt tggttgtttg atttttcttt 2300
ctttctttgt aaatagttct gagttctgtc aaatgccgtg aaagtatttg 2350
ctataataaa gaaaattctt gtgactttaa aaaaaaa 2387

<210> 528
<211> 487
<212> PRT
<213> Homo Sapien

<400> 528
Met Leu Arg Ala Pro Gly Cys Leu Leu Arg Thr Ser Val Ala Pro
1 5 10 15

Ala Ala Ala Leu Ala Ala Ala Leu Leu Ser Ser Leu Ala Arg Cys
20 25 30

Ser Leu Leu Glu Pro Arg Asp Pro Val Ala Ser Ser Leu Ser Pro
35 40 45

Tyr Phe Gly Thr Lys Thr Arg Tyr Glu Asp Val Asn Pro Val Leu
50 55 60

Leu Ser Gly Pro Glu Ala Pro Trp Arg Asp Pro Glu Leu Leu Glu
65 70 75

Gly Thr Cys Thr Pro Val Gln Leu Val Ala Leu Ile Arg His Gly
80 85 90

Thr Arg Tyr Pro Thr Val Lys Gln Ile Arg Lys Leu Arg Gln Leu
95 100 105

His Gly Leu Leu Gln Ala Arg Gly Ser Arg Asp Gly Gly Ala Ser
110 115 120

Ser Thr Gly Ser Arg Asp Leu Gly Ala Ala Leu Ala Asp Trp Pro
125 130 135

Leu Trp Tyr Ala Asp Trp Met Asp Gly Gln Leu Val Glu Lys Gly
140 145 150

Arg Gln Asp Met Arg Gln Leu Ala Leu Arg Leu Ala Ser Leu Phe
155 160 165

Pro Ala Leu Phe Ser Arg Glu Asn Tyr Gly Arg Leu Arg Leu Ile
170 175 180

Thr Ser Ser Lys His Arg Cys Met Asp Ser Ser Ala Ala Phe Leu
185 190 195

Gln Gly Leu Trp Gln His Tyr His Pro Gly Leu Pro Pro Pro Asp
200 205 210

Val Ala Asp Met Glu Phe Gly Pro Pro Thr Val Asn Asp Lys Leu
215 220 225

Met Arg Phe Phe Asp His Cys Glu Lys Phe Leu Thr Glu Val Glu
230 235 240

Lys Asn Ala Thr Ala Leu Tyr His Val Glu Ala Phe Lys Thr Gly
245 250 255

Pro Glu Met Gln Asn Ile Leu Lys Lys Val Ala Ala Thr Leu Gln
260 265 270

Val Pro Val Asn Asp Leu Asn Ala Asp Leu Ile Gln Val Ala Phe
275 280 285

Phe Thr Cys Ser Phe Asp Leu Ala Ile Lys Gly Val Lys Ser Pro
290 295 300

Trp Cys Asp Val Phe Asp Ile Asp Asp Ala Lys Val Leu Glu Tyr
305 310 315

Leu Asn Asp Leu Lys Gln Tyr Trp Lys Arg Gly Tyr Gly Tyr Thr
320 325 330

Ile Asn Ser Arg Ser Ser Cys Thr Leu Phe Gln Asp Ile Phe Gln
335 340 345

His Leu Asp Lys Ala Val Glu Gln Lys Gln Arg Ser Gln Pro Ile
350 355 360

Ser Ser Pro Val Ile Leu Gln Phe Gly His Ala Glu Thr Leu Leu
365 370 375

Pro Leu Leu Ser Leu Met Gly Tyr Phe Lys Asp Lys Glu Pro Leu
380 385 390

Thr Ala Tyr Asn Tyr Lys Lys Gln Met His Arg Lys Phe Arg Ser
395 400 405

Gly Leu Ile Val Pro Tyr Ala Ser Asn Leu Ile Phe Val Leu Tyr
410 415 420

His Cys Glu Asn Ala Lys Thr Pro Lys Glu Gln Phe Arg Val Gln
425 430 435

Met Leu Leu Asn Glu Lys Val Leu Pro Leu Ala Tyr Ser Gln Glu
440 445 450

Thr Val Ser Phe Tyr Glu Asp Leu Lys Asn His Tyr Lys Asp Ile
455 460 465

Leu Gln Ser Cys Gln Thr Ser Glu Glu Cys Glu Leu Ala Arg Ala
470 475 480

Asn Ser Thr Ser Asp Glu Leu
485

<210> 529
<211> 1777
<212> DNA
<213> Homo Sapien

<400> 529
ggagagccgc ggctgggacc ggagtgggga gcgcggcgtg gaggtgccac 50

ccggcgcggg tggcggagag atcagaagcc tcttccccaa gccgagccaa 100

cctcagcggg gacccgggct cagggacgcg gcggcggcgg cggcgactgc 150

agtggctgga cgatggcagc gtccgccgga gccggggcgg tgattgcagc 200

cccagacagc cggcgctggc tgtggtcggt gctggcggcg gcgcttgggc 250

tcttgacagc tggagtatca gccttggaag tatatacgcc aaaagaaatc 300

ttcgtggcaa atggtacaca agggaagctg acctgcaagt tcaagtctac 350

tagtacgact ggcgggttga cctcagtctc ctggagcttc cagccagagg 400

gggccgacac tactgtgtcg tttttccact actcccaagg gcaagtgtac 450

cttgggaatt atccaccatt taaagacaga atcagctggg ctggagacct 500

tgacaagaaa gatgcatcaa tcaacataga aaatatgcag tttatacaca 550

atggcaccta tatctgtgat gtcaaaaacc ctcctgacat cgttgtccag 600

cctggacaca ttaggctcta tgtcgtagaa aaagagaatt tgcctgtgtt 650

tccagtttgg gtagtggtgg gcatagttac tgctgtggtc ctaggtctca 700

ctctgctcat cagcatgatt ctggctgtcc tctatagaag gaaaaactct 750

aaacgggatt acactggctg cagtacatca gagagtttgt caccagttaa 800

gcaggctcct cggaagtccc cctccgacac tgagggtctt gtaaagagtc 850

tgccttctgg atctcaccag ggcccagtca tatatgcaca gttagaccac 900

tccggcggac atcacagtga caagattaac aagtcagagt ctgtggtgta 950

tgcggatatc cgaaagaatt aagagaatac ctagaacata tcctcagcaa 1000

gaaacaaaac caaactggac tctcgtgcag aaaatgtagc ccattaccac 1050

atgtagcctt ggagacccag gcaaggacaa gtacacgtgt actcacagag 1100

ggagagaaag atgtgtacaa aggatatgta taaatattct atttagtcat 1150

cctgatatga ggagccagtg ttgcatgatg aaaagatggt atgattctac 1200

atatgtaccc attgtcttgc tgtttttgta ctttcttttc aggtcattta 1250

caattgggag atttcagaaa cattcctttc accatcattt agaaatggtt 1300

tgccttaatg gagacaatag cagatcctgt agtatttcca gtagacatgg 1350

ccttttaatc taagggctta agactgatta gtcttagcat ttactgtagt 1400

tggaggatgg agatgctatg atggaagcat acccagggtg gcctttagca 1450

cagtatcagt accatttatt tgtctgccgc ttttaaaaaa tacccattgg 1500

ctatgccact tgaaaacaat ttgagaagtt tttttgaagt ttttctcact 1550

aaaatatggg gcaattgtta gccttacatg ttgtgtagac ttactttaag 1600

tttgcaccct tgaaatgtgt catatcaatt tctggattca taatagcaag 1650

attagcaaag gataaatgcc gaaggtcact tcattctgga cacagttgga 1700

tcaatactga ttaagtagaa aatccaagct ttgcttgaga acttttgtaa 1750

cgtggagagt aaaaagtatc ggtttta 1777

<210> 530
<211> 269
<212> PRT
<213> Homo Sapien

<400> 530
Met Ala Ala Ser Ala Gly Ala Gly Ala Val Ile Ala Ala Pro Asp
1 5 10 15

Ser Arg Arg Trp Leu Trp Ser Val Leu Ala Ala Ala Leu Gly Leu
20 25 30

Leu Thr Ala Gly Val Ser Ala Leu Glu Val Tyr Thr Pro Lys Glu
35 40 45

Ile Phe Val Ala Asn Gly Thr Gln Gly Lys Leu Thr Cys Lys Phe
50 55 60

Lys Ser Thr Ser Thr Thr Gly Gly Leu Thr Ser Val Ser Trp Ser
65 70 75

Phe Gln Pro Glu Gly Ala Asp Thr Thr Val Ser Phe Phe His Tyr
80 85 90

Ser Gln Gly Gln Val Tyr Leu Gly Asn Tyr Pro Pro Phe Lys Asp
95 100 105

Arg Ile Ser Trp Ala Gly Asp Leu Asp Lys Lys Asp Ala Ser Ile
110 115 120

Asn Ile Glu Asn Met Gln Phe Ile His Asn Gly Thr Tyr Ile Cys
125 130 135

Asp Val Lys Asn Pro Pro Asp Ile Val Val Gln Pro Gly His Ile
140 145 150

Arg Leu Tyr Val Val Glu Lys Glu Asn Leu Pro Val Phe Pro Val
155 160 165

Trp Val Val Val Gly Ile Val Thr Ala Val Val Leu Gly Leu Thr
170 175 180

Leu Leu Ile Ser Met Ile Leu Ala Val Leu Tyr Arg Arg Lys Asn
185 190 195

Ser Lys Arg Asp Tyr Thr Gly Cys Ser Thr Ser Glu Ser Leu Ser
200 205 210

Pro Val Lys Gln Ala Pro Arg Lys Ser Pro Ser Asp Thr Glu Gly
215 220 225

Leu Val Lys Ser Leu Pro Ser Gly Ser His Gln Gly Pro Val Ile
230 235 240

Tyr Ala Gln Leu Asp His Ser Gly Gly His His Ser Asp Lys Ile
245 250 255

Asn Lys Ser Glu Ser Val Val Tyr Ala Asp Ile Arg Lys Asn
260 265

<210> 531
<211> 1150
<212> DNA
<213> Homo Sapien

<400> 531
gtgacactat agaagagcta tgacgtcgca tgcacgcgta cgtaagctcg 50

gaattcggct cgaggctggt gggaagaagc cgagatggcg gcagccagcg 100

ctggggcaac ccggctgctc ctgctcttgc tgatggcggt agcagcgccc 150

agtcgagccc ggggcagcgg ctgccgggcc gggactggtg cgcgaggggc 200

tggggcggaa ggtcgagagg gcgaggcctg tggcacggtg ggctgctgc 250

tggagcactc atttgagatc gatgacagtg ccaacttccg gaagcggggc 300

tcactgctct ggaaccagca ggatggtacc ttgtccctgt cacagcggca 350

gctcagcgag gaggagcggg gccgactccg ggatgtggca gccctgaatg 400

gcctgtaccg ggtccggatc ccaaggcgac ccggggccct ggatggcctg 450

gaagctggtg gctatgtctc ctcctttgtc cctgcgtgct ccctggtgga 500

gtcgcacctg tcggaccagc tgaccctgca cgtggatgtg gccggcaacg 550

tggtgggcgt gtcggtggtg acgcaccccg ggggctgccg gggccatgag 600

gtggaggacg tggacctgga gctgttcaac acctcggtgc agctgcagcc 650

gcccaccaca gccccaggcc ctgagacggc ggccttcatt gagcgcctgg 700

agatggaaca ggcccagaag gccaagaacc cccaggagca gaagtccttc 750

ttcgccaaat actggatgta catcattccc gtcgtcctgt tcctcatgat 800

gtcaggagcg ccagacaccg gggccaggg tgggggtggg ggtgggggtg 850

gtggtggggg tagtggcctt tgctgtgtgc caccctccct gtaagtctat 900

ttaaaaacat cgacgataca ttgaaatgtg tgaacgtttt gaaaagctac 950

agcttccagc agccaaaagc aactgttgtt ttggcaagac ggtcctgatg 1000

tacaagcttg attgaaattc actgctcact tgatacgtta ttcagaaacc 1050

caaggaatgg ctgtccccat cctcatgtgg ctgtgtggag ctcagctgtg 1100

ttgtgtggca gtttattaaa ctgtccccca gatcgacacg caaaaaaaaa 1150

<210> 532
<211> 269
<212> PRT
<213> Homo Sapien

<400> 532
Met Ala Ala Ala Ser Ala Gly Ala Thr Arg Leu Leu Leu Leu Leu
1 5 10 15

Leu Met Ala Val Ala Ala Pro Ser Arg Ala Arg Gly Ser Gly Cys
20 25 30

Arg Ala Gly Thr Gly Ala Arg Gly Ala Gly Ala Glu Gly Arg Glu
35 40 45

Gly Glu Ala Cys Gly Thr Val Gly Leu Leu Leu Glu His Ser Phe
50 55 60

Glu Ile Asp Asp Ser Ala Asn Phe Arg Lys Arg Gly Ser Leu Leu
65 70 75

Trp Asn Gln Gln Asp Gly Thr Leu Ser Leu Ser Gln Arg Gln Leu
80 85 90

Ser Glu Glu Glu Arg Gly Arg Leu Arg Asp Val Ala Ala Leu Asn
95 100 105

Gly Leu Tyr Arg Val Arg Ile Pro Arg Arg Pro Gly Ala Leu Asp
110 115 120

Gly Leu Glu Ala Gly Gly Tyr Val Ser Ser Phe Val Pro Ala Cys
125 130 135

Ser Leu Val Glu Ser His Leu Ser Asp Gln Leu Thr Leu His Val
140 145 150

Asp Val Ala Gly Asn Val Val Gly Val Ser Val Val Thr His Pro
155 160 165

Gly Gly Cys Arg Gly His Glu Val Glu Asp Val Asp Leu Glu Leu
170 175 180

Phe Asn Thr Ser Val Gln Leu Gln Pro Pro Thr Thr Ala Pro Gly
185 190 195

Pro Glu Thr Ala Ala Phe Ile Glu Arg Leu Glu Met Glu Gln Ala
200 205 210

Gln Lys Ala Lys Asn Pro Gln Glu Gln Lys Ser Phe Phe Ala Lys
215 220 225

Tyr Trp Met Tyr Ile Ile Pro Val Val Leu Phe Leu Met Met Ser
230 235 240

Gly Ala Pro Asp Thr Gly Gly Gln Gly Gly Gly Gly Gly Gly Gly
245 250 255

Gly Gly Gly Gly Ser Gly Leu Cys Cys Val Pro Pro Ser Leu
260 265

<210> 533
<211> 496
<212> DNA
<213> Homo Sapien

<220>
<221> unsure
<222> 396
<223> unknown base

<400> 533
tctgcctcca ctgctctgtg ctgggatcat ggaacttgca ctgctgtgtg 50

ggctggtggt gatggctggt gtgattccaa tccagggcgg gatcctgaac 100

ctgaacaaga tggtcaagca agtgactggg aaaatgccca tcctctccta 150

ctggccctac ggctgtcact gcggactagg tggcagaggc caacccaaag 200

atgccacgga ctggtgctgc cagacccatg actgctgcta tgaccacctg 250

aagacccagg ggtgcggcat ctacaaggac aacaacaaaa gcagcataca 300

ttgtatggat ttatctcaac gctattgttt aatggctgtg tttaatgtga 350

tctatctgga aaatgaggac tccgaataaa aagctattac tawttnaaaa 400

aaaaaaaaaa aaaaaaaaaa aaaaaaaaaa aaaaaaaaaa aaaaaaaaaa 450

aaaaaaaaaa aaaaaaaaaa aaaaaaaaaa aaaaaaaaaa aaaaaa 496

<210> 534
<211> 116
<212> PRT
<213> Homo Sapien

<400> 534
Met Glu Leu Ala Leu Leu Cys Gly Leu Val Val Met Ala Gly Val
1 5 10 15

Ile Pro Ile Gln Gly Gly Ile Leu Asn Leu Asn Lys Met Val Lys
20 25 30

```
Gln Val Thr Gly Lys Met Pro Ile Leu Ser Tyr Trp Pro Tyr Gly
                35                  40                  45

Cys His Cys Gly Leu Gly Gly Arg Gly Gln Pro Lys Asp Ala Thr
                50                  55                  60

Asp Trp Cys Cys Gln Thr His Asp Cys Cys Tyr Asp His Leu Lys
                65                  70                  75

Thr Gln Gly Cys Gly Ile Tyr Lys Asp Asn Asn Lys Ser Ser Ile
                80                  85                  90

His Cys Met Asp Leu Ser Gln Arg Tyr Cys Leu Met Ala Val Phe
                95                  100                 105

Asn Val Ile Tyr Leu Glu Asn Glu Asp Ser Glu
                110                 115
```

```
<210> 535
<211> 2379
<212> DNA
<213> Homo Sapien

<400> 535
gctgagcgtg tgcgcggtac ggggctctcc tgccttctgg gctccaacgc 50

agctctgtgg ctgaactggg tgctcatcac gggaactgct gggctatgga 100

atacagatgt ggcagctcag gtagccccaa attgcctgga agaatacatc 150

atgtttttcg ataagaagaa attgtaggat ccagtttttt ttttaaccgc 200

cccctcccca ccccccaaaa aaactgtaaa gatgcaaaaa cgtaatatcc 250

atgaagatcc tattacctag gaagattttg atgttttgct gcgaatgcgg 300

tgttgggatt tatttgttct tggagtgttc tgcgtggctg gcaaagaata 350

atgttccaaa atcggtccat ctcccaaggg gtccaatttt tcttcctggg 400

tgtcagcgag ccctgactca ctacagtgca gctgacaggg gctgtcatgc 450

aactggcccc taagccaaag caaaagacct aaggacgacc tttgaacaat 500

acaaaggatg ggtttcaatg taattaggct actgagcgga tcagctgtag 550

cactggttat agcccccact gtcttactga caatgctttc ttctgccgaa 600

cgaggatgcc ctaagggctg taggtgtgaa ggcaaaatgg tatattgtga 650

atctcagaaa ttacaggaga taccctcaag tatatctgct ggttgcttag 700

gtttgtccct tcgctataac agccttcaaa aacttaagta taatcaattt 750

aaagggctca accagctcac ctggctatac cttgaccata accatatcag 800

caatattgac gaaaatgctt ttaatggaat acgcagactc aaagagctga 850

ttcttagttc caatagaatc tcctattttc ttaacaatac cttcagacct 900

gtgacaaatt tacggaactt ggatctgtcc tataatcagc tgcattctct 950

gggatctgaa cagtttcggg gcttgcggaa gctgctgagt ttacatttac 1000
```

ggtctaactc cctgagaacc atccctgtgc gaatattcca agactgccgc 1050

aacctggaac ttttggacct gggatataac cggatccgaa gtttagccag 1100

gaatgtcttt gctggcatga tcagactcaa agaacttcac ctggagcaca 1150

atcaattttc caagctcaac ctggcccttt ttccaaggtt ggtcagcctt 1200

cagaaccttt acttgcagtg gaataaaatc agtgtcatag gacagaccat 1250

gtcctggacc tggagctcct tacaaaggct tgatttatca ggcaatgaga 1300

tcgaagcttt cagtggaccc agtgttttcc agtgtgtccc gaatctgcag 1350

cgcctcaacc tggattccaa caagctcaca tttattggtc aagagatttt 1400

ggattcttgg atatccctca atgacatcag tcttgctggg aatatatggg 1450

aatgcagcag aaatatttgc tcccttgtaa actggctgaa aagttttaaa 1500

ggtctaaggg agaatacaat tatctgtgcc agtcccaaag agctgcaagg 1550

agtaaatgtg atcgatgcag tgaagaacta cagcatctgt ggcaaaagta 1600

ctacagagag gtttgatctg gccagggctc tcccaaagcc gacgtttaag 1650

cccaagctcc ccaggccgaa gcatgagagc aaaccccctt tgcccccgac 1700

ggtgggagcc acagagcccg gcccagagac cgatgctgac gccgagcaca 1750

tctctttcca taaaatcatc gcgggcagcg tggcgctttt cctgtccgtg 1800

ctcgtcatcc tgctggttat ctacgtgtca tggaagcggt accctgcgag 1850

catgaagcag ctgcagcagc gctccctcat gcgaaggcac aggaaaaaga 1900

aaagacagtc cctaaagcaa atgactccca gcacccagga attttatgta 1950

gattataaac ccaccaacac ggagaccagc gagatgctgc tgaatgggac 2000

gggaccctgc acctataaca aatcgggctc cagggagtgt gaggtatgaa 2050

ccattgtgat aaaaagagct cttaaaagct gggaaataag tggtgcttta 2100

ttgaactctg gtgactatca agggaacgcg atgccccccc tccccttccc 2150

tctccctctc actttggtgg caagatcctt ccttgtccgt tttagtgcat 2200

tcataatact ggtcattttc ctctcataca taatcaaccc attgaaattt 2250

aaataccaca atcaatgtga agcttgaact ccggtttaat ataataccta 2300

ttgtataaga ccctttactg attccattaa tgtcgcattt gttttaagat 2350

aaaacttctt tcataggtaa aaaaaaaaa 2379

<210> 536
<211> 513
<212> PRT
<213> Homo Sapien

<400> 536

Met Gly Phe Asn Val Ile Arg Leu Leu Ser Gly Ser Ala Val Ala
1 5 10 15

```
Leu Val Ile Ala Pro Thr Val Leu Leu Thr Met Leu Ser Ser Ala
                20                  25                  30

Glu Arg Gly Cys Pro Lys Gly Cys Arg Cys Glu Gly Lys Met Val
                35                  40                  45

Tyr Cys Glu Ser Gln Lys Leu Gln Glu Ile Pro Ser Ser Ile Ser
                50                  55                  60

Ala Gly Cys Leu Gly Leu Ser Leu Arg Tyr Asn Ser Leu Gln Lys
                65                  70                  75

Leu Lys Tyr Asn Gln Phe Lys Gly Leu Asn Gln Leu Thr Trp Leu
                80                  85                  90

Tyr Leu Asp His Asn His Ile Ser Asn Ile Asp Glu Asn Ala Phe
                95                  100                 105

Asn Gly Ile Arg Arg Leu Lys Glu Leu Ile Leu Ser Ser Asn Arg
                110                 115                 120

Ile Ser Tyr Phe Leu Asn Asn Thr Phe Arg Pro Val Thr Asn Leu
                125                 130                 135

Arg Asn Leu Asp Leu Ser Tyr Asn Gln Leu His Ser Leu Gly Ser
                140                 145                 150

Glu Gln Phe Arg Gly Leu Arg Lys Leu Leu Ser Leu His Leu Arg
                155                 160                 165

Ser Asn Ser Leu Arg Thr Ile Pro Val Arg Ile Phe Gln Asp Cys
                170                 175                 180

Arg Asn Leu Glu Leu Leu Asp Leu Gly Tyr Asn Arg Ile Arg Ser
                185                 190                 195

Leu Ala Arg Asn Val Phe Ala Gly Met Ile Arg Leu Lys Glu Leu
                200                 205                 210

His Leu Glu His Asn Gln Phe Ser Lys Leu Asn Leu Ala Leu Phe
                215                 220                 225

Pro Arg Leu Val Ser Leu Gln Asn Leu Tyr Leu Gln Trp Asn Lys
                230                 235                 240

Ile Ser Val Ile Gly Gln Thr Met Ser Trp Thr Trp Ser Ser Leu
                245                 250                 255

Gln Arg Leu Asp Leu Ser Gly Asn Glu Ile Glu Ala Phe Ser Gly
                260                 265                 270

Pro Ser Val Phe Gln Cys Val Pro Asn Leu Gln Arg Leu Asn Leu
                275                 280                 285

Asp Ser Asn Lys Leu Thr Phe Ile Gly Gln Glu Ile Leu Asp Ser
                290                 295                 300

Trp Ile Ser Leu Asn Asp Ile Ser Leu Ala Gly Asn Ile Trp Glu
                305                 310                 315

Cys Ser Arg Asn Ile Cys Ser Leu Val Asn Trp Leu Lys Ser Phe
                320                 325                 330
```

Lys Gly Leu Arg Glu Asn Thr Ile Ile Cys Ala Ser Pro Lys Glu
335 340 345

Leu Gln Gly Val Asn Val Ile Asp Ala Val Lys Asn Tyr Ser Ile
350 355 360

Cys Gly Lys Ser Thr Thr Glu Arg Phe Asp Leu Ala Arg Ala Leu
365 370 375

Pro Lys Pro Thr Phe Lys Pro Lys Leu Pro Arg Pro Lys His Glu
380 385 390

Ser Lys Pro Pro Leu Pro Pro Thr Val Gly Ala Thr Glu Pro Gly
395 400 405

Pro Glu Thr Asp Ala Asp Ala Glu His Ile Ser Phe His Lys Ile
410 415 420

Ile Ala Gly Ser Val Ala Leu Phe Leu Ser Val Leu Val Ile Leu
425 430 435

Leu Val Ile Tyr Val Ser Trp Lys Arg Tyr Pro Ala Ser Met Lys
440 445 450

Gln Leu Gln Gln Arg Ser Leu Met Arg Arg His Arg Lys Lys Lys
455 460 465

Arg Gln Ser Leu Lys Gln Met Thr Pro Ser Thr Gln Glu Phe Tyr
470 475 480

Val Asp Tyr Lys Pro Thr Asn Thr Glu Thr Ser Glu Met Leu Leu
485 490 495

Asn Gly Thr Gly Pro Cys Thr Tyr Asn Lys Ser Gly Ser Arg Glu
500 505 510

Cys Glu Val

<210> 537
<211> 3554
<212> DNA
<213> Homo Sapien

<400> 537
gggactacaa gccgcgccgc gctgccgctg gcccctcagc aaccctcgac 50

atggcgctga ggcggccacc gcgactccgg ctctgcgctc ggctgcctga 100

cttcttcctg ctgctgcttt tcaggggctg cctgataggg gctgtaaatc 150

tcaaatccag caatcgaacc ccagtggtac aggaatttga aagtgtggaa 200

ctgtcttgca tcattacgga ttcgcagaca agtgacccca ggatcgagtg 250

gaagaaaatt caagatgaac aaaccacata tgtgtttttt gacaacaaaa 300

ttcagggaga cttggcgggt cgtgcagaaa tactggggaa gacatccctg 350

aagatctgga atgtgacacg gagagactca gccctttatc gctgtgaggt 400

cgttgctcga aatgaccgca aggaaattga tgagattgtg atcgagttaa 450

ctgtgcaagt gaagccagtg acccctgtct gtagagtgcc gaaggctgta 500

ccagtaggca agatggcaac actgcactgc caggagagtg agggccaccc 550
ccggcctcac tacagctggt atcgcaatga tgtaccactg cccacggatt 600
ccagagccaa tcccagattt cgcaattctt ctttccactt aaactctgaa 650
acaggcactt tggtgttcac tgctgttcac aaggacgact ctgggcagta 700
ctactgcatt gcttccaatg acgcaggctc agccaggtgt gaggagcagg 750
agatggaagt ctatgacctg aacattggcg gaattattgg gggggttctg 800
gttgtccttg ctgtactggc cctgatcacg ttgggcatct gctgtgcata 850
cagacgtggc tacttcatca acaataaaca ggatggagaa agttacaaga 900
acccagggaa accagatgga gttaactaca tccgcactga cgaggagggc 950
gacttcagac acaagtcatc gtttgtgatc tgagacccgc ggtgtggctg 1000
agagcgcaca gagcgcacgt gcacatacct ctgctagaaa ctcctgtcaa 1050
ggcagcgaga gctgatgcac tcggacagag ctagacactc attcagaagc 1100
ttttcgtttt ggccaaagtt gaccactact cttcttactc taacaagcca 1150
catgaataga agaattttcc tcaagatgga cccggtaaat ataaccacaa 1200
ggaagcgaaa ctgggtgcgt tcactgagtt gggttcctaa tctgtttctg 1250
gcctgattcc cgcatgagta ttagggtgat cttaaagagt ttgctcacgt 1300
aaacgcccgt gctgggccct gtgaagccag catgttcacc actggtcgtt 1350
cagcagccac gacagcacca tgtgagatgg cgaggtggct ggacagcacc 1400
agcagcgcat cccggcggga acccagaaaa ggcttcttac acagcagcct 1450
tacttcatcg gcccacagac accaccgcag tttcttctta aaggctctgc 1500
tgatcggtgt tgcagtgtcc attgtggaga agctttttgg atcagcattt 1550
tgtaaaaaca accaaaatca ggaaggtaaa ttggttgctg gaagagggat 1600
cttgcctgag gaaccctgct tgtccaacag ggtgtcagga tttaaggaaa 1650
accttcgtct taggctaagt ctgaaatggt actgaaatat gcttttctat 1700
gggtcttgtt tattttataa aattttacat ctaaattttt gctaaggatg 1750
tattttgatt attgaaaaga aaatttctat ttaaactgta aatatattgt 1800
catacaatgt taaataacct attttttttaa aaaagttcaa cttaaggtag 1850
aagttccaag ctactagtgt taaattggaa aatatcaata attaagagta 1900
ttttacccaa ggaatcctct catggaagtt tactgtgatg ttccttttct 1950
cacacaagtt ttagcctttt tcacaaggga actcatactg tctacacatc 2000
agaccatagt tgcttaggaa acctttaaaa attccagtta agcaatgttg 2050
aaatcagttt gcatctcttc aaaagaaacc tctcaggtta gctttgaact 2100

gcctcttcct gagatgacta ggacagtctg tacccagagg ccacccagaa 2150

gccctcagat gtacatacac agatgccagt cagctcctgg ggttgcgcca 2200

ggcgcccccg ctctagctca ctgttgcctc gctgtctgcc aggaggccct 2250

gccatccttg ggccctggca gtggctgtgt cccagtgagc tttactcacg 2300

tggcccttgc ttcatccagc acagctctca ggtgggcact gcagggacac 2350

tggtgtcttc catgtagcgt cccagctttg ggctcctgta acagacctct 2400

ttttggttat ggatggctca caaaataggg cccccaatgc tatttttttt 2450

ttttaagttt gtttaattat ttgttaagat tgtctaaggc caaaggcaat 2500

tgcgaaatca agtctgtcaa gtacaataac atttttaaaa gaaaatggat 2550

cccactgttc ctctttgcca cagagaaagc acccagacgc cacaggctct 2600

gtcgcatttc aaaacaaacc atgatggagt ggcggccagt ccagcctttt 2650

aaagaacgtc aggtggagca gccaggtgaa aggcctggcg gggaggaaag 2700

tgaaacgcct gaatcaaaag cagttttcta attttgactt taaatttttc 2750

atccgccgga gacactgctc ccatttgtgg ggggacatta gcaacatcac 2800

tcagaagcct gtgttcttca agagcaggtg ttctcagcct cacatgccct 2850

gccgtgctgg actcaggact gaagtgctgt aaagcaagga gctgctgaga 2900

aggagcactc cactgtgtgc ctggagaatg gctctcacta ctcaccttgt 2950

ctttcagctt ccagtgtctt gggtttttta tactttgaca gcttttttt 3000

aattgcatac atgagactgt gttgactttt tttagttatg tgaaacactt 3050

tgccgcaggc cgcctggcag aggcaggaaa tgctccagca gtggctcagt 3100

gctccctggt gtctgctgca tggcatcctg gatgcttagc atgcaagttc 3150

cctccatcat tgccaccttg gtagagaggg atggctcccc accctcagcg 3200

ttggggattc acgctccagc ctccttcttg gttgtcatag tgatagggta 3250

gccttattgc cccctcttct tataccctaa aaccttctac actagtgcca 3300

tgggaaccag gtctgaaaaa gtagagagaa gtgaaagtag agtctgggaa 3350

gtagctgcct ataactgaga ctagacggaa aaggaatact cgtgtatttt 3400

aagatatgaa tgtgactcaa gactcgaggc cgatacgagg ctgtgattct 3450

gcctttggat ggatgttgct gtacacagat gctacagact tgtactaaca 3500

caccgtaatt tggcatttgt ttaacctcat ttataaaagc ttcaaaaaaa 3550

ccca 3554

<210> 538
<211> 310
<212> PRT

<213> Homo Sapien

<400> 538

```
Met Ala Leu Arg Arg Pro Pro Arg Leu Arg Leu Cys Ala Arg Leu
1               5                   10                  15

Pro Asp Phe Phe Leu Leu Leu Leu Phe Arg Gly Cys Leu Ile Gly
                20                  25                  30

Ala Val Asn Leu Lys Ser Ser Asn Arg Thr Pro Val Val Gln Glu
                35                  40                  45

Phe Glu Ser Val Glu Leu Ser Cys Ile Ile Thr Asp Ser Gln Thr
                50                  55                  60

Ser Asp Pro Arg Ile Glu Trp Lys Lys Ile Gln Asp Glu Gln Thr
                65                  70                  75

Thr Tyr Val Phe Phe Asp Asn Lys Ile Gln Gly Asp Leu Ala Gly
                80                  85                  90

Arg Ala Glu Ile Leu Gly Lys Thr Ser Leu Lys Ile Trp Asn Val
                95                  100                 105

Thr Arg Arg Asp Ser Ala Leu Tyr Arg Cys Glu Val Val Ala Arg
                110                 115                 120

Asn Asp Arg Lys Glu Ile Asp Glu Ile Val Ile Glu Leu Thr Val
                125                 130                 135

Gln Val Lys Pro Val Thr Pro Val Cys Arg Val Pro Lys Ala Val
                140                 145                 150

Pro Val Gly Lys Met Ala Thr Leu His Cys Gln Glu Ser Glu Gly
                155                 160                 165

His Pro Arg Pro His Tyr Ser Trp Tyr Arg Asn Asp Val Pro Leu
                170                 175                 180

Pro Thr Asp Ser Arg Ala Asn Pro Arg Phe Arg Asn Ser Ser Phe
                185                 190                 195

His Leu Asn Ser Glu Thr Gly Thr Leu Val Phe Thr Ala Val His
                200                 205                 210

Lys Asp Asp Ser Gly Gln Tyr Tyr Cys Ile Ala Ser Asn Asp Ala
                215                 220                 225

Gly Ser Ala Arg Cys Glu Glu Gln Glu Met Glu Val Tyr Asp Leu
                230                 235                 240

Asn Ile Gly Gly Ile Ile Gly Gly Val Leu Val Val Leu Ala Val
                245                 250                 255

Leu Ala Leu Ile Thr Leu Gly Ile Cys Cys Ala Tyr Arg Arg Gly
                260                 265                 270

Tyr Phe Ile Asn Asn Lys Gln Asp Gly Glu Ser Tyr Lys Asn Pro
                275                 280                 285

Gly Lys Pro Asp Gly Val Asn Tyr Ile Arg Thr Asp Glu Glu Gly
                290                 295                 300

Asp Phe Arg His Lys Ser Ser Phe Val Ile
```

305 310

<210> 539
<211> 2570
<212> DNA
<213> Homo Sapien

<400> 539
ccaggaccag ggcgcaccgg ctcagcctct cacttgtcag aggccgggga 50
agagaagcaa agcgcaacgg tgtggtccaa gccggggctt ctgcttcgcc 100
tctaggacat acacgggacc ccctaacttc agtcccccaa acgcgcaccc 150
tcgaagtctt gaactccagc cccgcacatc cacgcgcggc acaggcgcgg 200
caggcggcag gtcccggccg aaggcgatgc gcgcaggggg tcgggcagct 250
gggctcgggc ggcgggagta gggcccggca gggaggcagg gaggctgcat 300
attcagagtc gcgggctgcg ccctgggcag aggccgccct cgctccacgc 350
aacacctgct gctgccaccg cgccgcgatg agccgcgtgg tctcgctgct 400
gctgggcgcc gcgctgctct gcggccacgg agccttctgc cgccgcgtgg 450
tcagcggcca aaaggtgtgt tttgctgact tcaagcatcc ctgctacaaa 500
atggcctact tccatgaact gtccagccga gtgagctttc aggaggcacg 550
cctggcttgt gagagtgagg gaggagtcct cctcagcctt gagaatgaag 600
cagaacagaa gttaatagag agcatgttgc aaaacctgac aaaacccggg 650
acagggattt ctgatggtga tttctggata gggctttgga ggaatggaga 700
tgggcaaaca tctggtgcct gcccagatct ctaccagtgg tctgatggaa 750
gcaattccca gtaccgaaac tggtacacag atgaaccttc ctgcggaagt 800
gaaaagtgtg ttgtgatgta tcaccaacca actgccaatc ctggccttgg 850
gggtccctac ctttaccagt ggaatgatga caggtgtaac atgaagcaca 900
attatatttg caagtatgaa ccagagatta atccaacagc ccctgtagaa 950
aagccttatc ttacaaatca accaggagac acccatcaga atgtggttgt 1000
tactgaagca ggtataattc ccaatctaat ttatgttgtt ataccaacaa 1050
tacccctgct cttactgata ctggttgctt ttggaacctg ttgtttccag 1100
atgctgcata aaagtaaagg aagaacaaaa actagtccaa accagtctac 1150
actgtggatt tcaaagagta ccagaaaaga aagtggcatg gaagtataat 1200
aactcattga cttggttcca gaattttgta attctggatc tgtataagga 1250
atggcatcag aacaatagct tggaatggct tgaaatcaca aaggatctgc 1300
aagatgaact gtaagctccc ccttgaggca aatattaaag taatttttat 1350
atgtctatta tttcatttaa agaatatgct gtgctaataa tggagtgaga 1400

catgcttatt ttgctaaagg atgcacccaa acttcaaact tcaagcaaat 1450
gaaatggaca atgcagataa agttgttatc aacacgtcgg gagtatgtgt 1500
gttagaagca attcctttta tttctttcac ctttcataag ttgttatcta 1550
gtcaatgtaa tgtatattgt attgaaattt acagtgtgca aaagtatttt 1600
acctttgcat aagtgtttga taaaaatgaa ctgttctaat atttattttt 1650
atggcatctc atttttcaat acatgctctt ttgattaaag aaacttatta 1700
ctgttgtcaa ctgaattcac acacacacaa atatagtacc atagaaaaag 1750
tttgttttct cgaaataatt catctttcag cttctctgct tttggtcaat 1800
gtctaggaaa tctcttcaga aataagaagc tatttcatta agtgtgatat 1850
aaacctcctc aaacatttta cttagaggca aggattgtct aatttcaatt 1900
gtgcaagaca tgtgccttat aattattttt agcttaaaat taaacagatt 1950
ttgtaataat gtaactttgt taataggtgc ataaacacta atgcagtcaa 2000
tttgaacaaa agaagtgaca tacacaatat aaatcatatg tcttcacacg 2050
ttgcctatat aatgagaagc agctctctga gggttctgaa atcaatgtgg 2100
tccctctctt gcccactaaa caaagatggt tgttcggggt ttgggattga 2150
cactggaggc agatagttgc aaagttagtc taaggtttcc ctagctgtat 2200
ttagcctctg actatattag tatacaaaga ggtcatgtgg ttgagaccag 2250
gtgaatagtc actatcagtg tggagacaag cacagcacac agacatttta 2300
ggaaggaaag gaactacgaa atcgtgtgaa aatgggttgg aacccatcag 2350
tgatcgcata ttcattgatg agggtttgct tgagatagaa aatggtggct 2400
cctttctgtc ttatctccta gtttcttcaa tgcttacgcc ttgttcttct 2450
caagagaaag ttgtaactct ctggtcttca tatgtccctg tgctcctttt 2500
aaccaaataa agagttcttg tttctggggg aaaaaaaaaa aaaaaaaaaa 2550
aaaaaaaaaa aaaaaaaaaa 2570

<210> 540
<211> 273
<212> PRT
<213> Homo Sapien

<400> 540
Met Ser Arg Val Val Ser Leu Leu Leu Gly Ala Ala Leu Leu Cys
1 5 10 15

Gly His Gly Ala Phe Cys Arg Arg Val Val Ser Gly Gln Lys Val
20 25 30

Cys Phe Ala Asp Phe Lys His Pro Cys Tyr Lys Met Ala Tyr Phe
35 40 45

His Glu Leu Ser Ser Arg Val Ser Phe Gln Glu Ala Arg Leu Ala

```
                50                  55                  60

Cys Glu Ser Glu Gly Gly Val Leu Leu Ser Leu Glu Asn Glu Ala
                65                  70                  75

Glu Gln Lys Leu Ile Glu Ser Met Leu Gln Asn Leu Thr Lys Pro
                80                  85                  90

Gly Thr Gly Ile Ser Asp Gly Asp Phe Trp Ile Gly Leu Trp Arg
                95                  100                 105

Asn Gly Asp Gly Gln Thr Ser Gly Ala Cys Pro Asp Leu Tyr Gln
                110                 115                 120

Trp Ser Asp Gly Ser Asn Ser Gln Tyr Arg Asn Trp Tyr Thr Asp
                125                 130                 135

Glu Pro Ser Cys Gly Ser Glu Lys Cys Val Val Met Tyr His Gln
                140                 145                 150

Pro Thr Ala Asn Pro Gly Leu Gly Gly Pro Tyr Leu Tyr Gln Trp
                155                 160                 165

Asn Asp Asp Arg Cys Asn Met Lys His Asn Tyr Ile Cys Lys Tyr
                170                 175                 180

Glu Pro Glu Ile Asn Pro Thr Ala Pro Val Glu Lys Pro Tyr Leu
                185                 190                 195

Thr Asn Gln Pro Gly Asp Thr His Gln Asn Val Val Val Thr Glu
                200                 205                 210

Ala Gly Ile Ile Pro Asn Leu Ile Tyr Val Val Ile Pro Thr Ile
                215                 220                 225

Pro Leu Leu Leu Leu Ile Leu Val Ala Phe Gly Thr Cys Cys Phe
                230                 235                 240

Gln Met Leu His Lys Ser Lys Gly Arg Thr Lys Thr Ser Pro Asn
                245                 250                 255

Gln Ser Thr Leu Trp Ile Ser Lys Ser Thr Arg Lys Glu Ser Gly
                260                 265                 270

Met Glu Val


<210> 541
<211> 3824
<212> DNA
<213> Homo Sapien

<400> 541
ggagaatgga gagagcagtg agagtggagt ccggggtcct ggtcggggtg 50

gtctgtctgc tcctggcatg ccctgccaca gccactgggc ccgaagttgc 100

tcagcctgaa gtagacacca ccctgggtcg tgtgcgaggc cggcaggtgg 150

gcgtgaaggg cacagaccgc cttgtgaatg tctttctggg cattccattt 200

gcccagccgc cactgggccc tgaccggttc tcagccccac acccagcaca 250

gccctgggag ggtgtgcggg atgccagcac tgcgccccca atgtgcctac 300
```

aagacgtgga gagcatgaac agcagcagat ttgtcctcaa cggaaaacag 350
cagatcttct ccgtttcaga ggactgcctg gtcctcaacg tctatagccc 400
agctgaggtc cccgcagggt ccggtaggcc ggtcatggta tgggtccatg 450
gaggcgctct gataactggc gctgccacct cctacgatgg atcagctctg 500
gctgcctatg gggatgtggt cgtggttaca gtccagtacc gccttggggt 550
ccttggcttc ttcagcactg gagatgagca tgcacctggc aaccagggct 600
tcctagatgt ggtagctgct ttgcgctggg tgcaagaaaa catcgccccc 650
ttcgggggtg acctcaactg tgtcactgtc tttggtggat ctgccggtgg 700
gagcatcatc tctggcctgg tcctgtcccc agtggctgca gggctgttcc 750
acagagccat cacacagagt ggggtcatca ccaccccagg gatcatcgac 800
tctcaccctt ggcccctagc tcagaaaatc gcaaacacct tggcctgcag 850
ctccagctcc ccggctgaga tggtgcagtg ccttcagcag aaagaaggag 900
aagagctggt ccttagcaag aagctgaaaa atactatcta tcctctcacc 950
gttgatggca ctgtcttccc caaaagcccc aaggaactcc tgaaggagaa 1000
gcccttccac tctgtgccct tcctcatggg tgtcaacaac catgagttca 1050
gctggctcat ccccaggggc tggggtctcc tggatacaat ggagcagatg 1100
agccgggagg acatgctggc catctcaaca cccgtcttga ccagtctgga 1150
tgtgccccct gagatgatgc ccaccgtcat agatgaatac ctaggaagca 1200
actcggacgc acaagccaaa tgccaggcgt tccaggaatt catgggtgac 1250
gtattcatca atgttcccac cgtcagtttt tcaagatacc ttcgagattc 1300
tggaagccct gtctttttct atgagttcca gcatcgaccc agttcttttg 1350
cgaagatcaa acctgcctgg gtgaaggctg atcatggggc cgagggtgct 1400
tttgtgttcg gaggtccctt cctcatggac gagagctccc gcctggcctt 1450
tccagaggcc acagaggagg agaagcagct aagcctcacc atgatggccc 1500
agtggaccca ctttgcccgg acaggggacc ccaatagcaa ggctctgcct 1550
ccttggcccc aattcaacca ggcggaacaa tatctggaga tcaacccagt 1600
gccacgggcc ggacagaagt tcagggaggc ctggatgcag ttctggtcag 1650
agacgctccc cagcaagata caacagtggc accagaagca gaagaacagg 1700
aaggcccagg aggacctctg aggccaggcc tgaaccttct tggctggggc 1750
aaaccactct tcaagtggtg gcagagtccc agcacggcag cccgcctctc 1800
cccctgctga gactttaatc tccaccagcc cttaaagtgt cggccgctct 1850
gtgactggag ttatgctctt ttgaaatgtc acaaggccgc ctcccacctc 1900

tggggcattg tacaagttct tccctctccc tgaagtgcct ttcctgcttt 1950
cttcgtggta ggttctagca cattcctcta gcttcctgga ggactcactc 2000
cccaggaagc cttccctgcc ttctctgggc tgtgcggccc cgagtctgcg 2050
tccattagag cacagtccac ccgaggctag caccgtgtct gtgtctgtct 2100
ccccctcaga ggagctctct caaaatgggg attagcctaa ccccactctg 2150
tcacccacac caggatcggg tgggacctgg agctaggggg tgtttgctga 2200
gtgagtgagt gaaacacaga atatgggaat ggcagctgct gaacttgaac 2250
ccagagcctt caggtgccaa agccatactc aggcccccac cgacattgtc 2300
caccctggcc agaagggtgc atgccaatgg cagagacctg ggatgggaga 2350
agtcctgggg cgccagggga tccagcctag agcagacctt agcccctgac 2400
taaggcctca gactagggcg ggaggggtct cctcctctct gctgcccagt 2450
cctggcccct gcacaagaca acagaatcca tcagggccat gagtgtcacc 2500
cagacctgac cctcaccaat tccagcccct gaccctcagg acgctggatg 2550
ccagctccca gccccagtgc cgggtcctcc ctcccttcct ggcttgggga 2600
gaccagtttc tggggagctt ccaagagcac ccaccaagac acagcaggac 2650
aggccagggg agggcatctg gaccagggca tccgtcgggc tattgtcaca 2700
gagaaaagaa gagacccacc cactcgggct gcaaaaggtg aaaagcacca 2750
agaggttttc agatggaagt gagaggtgac agtgtgctgg cagccctcac 2800
agccctcgct tgctctccct gccgcctctg cctgggctcc cactttggca 2850
gcacttgagg agcccttcaa cccgccgctg cactgtagga gccccttttct 2900
gggctggcca aggccggagc cagctccctc agcttgcggg gaggtgcgga 2950
gggagagggg cgggcaggaa ccggggctgc gcgcagcgct tgcgggccag 3000
agtgagttcc gggtgggcgt gggctcggcg gggccccact cagagcagct 3050
ggccggcccc aggcagtgag ggccttagca cctgggccag cagctgctgt 3100
gctcgatttc tcgctgggcc ttagctgcct ccccgcgggg cagggctcgg 3150
gacctgcagc cctccatgcc tgaccctccc cccacccccc gtgggctcct 3200
gtgcggccgg agcctcccca aggagcgccg ccccctgctc cacagcgccc 3250
agtcccatcg accacccaag ggctgaggag tgcgggtgca cagcgcggga 3300
ctggcaggca gctccacctg ctgccccagt gctggatcca ctgggtgaag 3350
ccagctgggc tcctgagtct ggtggggact tggagaacct ttatgtctag 3400
ctaagggatt gtaaatacac cgatgggcac tctgtatcta gctcaaggtt 3450
tgtaaacaca ccaatcagca ccctgtgtct agctcagtgt ttgtgaatgc 3500

accaatccac actctgtatc tggctactct ggtggggact tggagaacct 3550

ttgtgtccac actctgtatc tagctaatct agtggggatg tggagaacct 3600

ttgtgtctag ctcagggatc gtaaacgcac caatcagcac cctgtcaaaa 3650

cagaccactt gactctctgt aaaatggacc aatcagcagg atgtgggtgg 3700

ggcgagacaa gagaataaaa gcaggctgcc tgagccagca gtgacaaccc 3750

ccctcgggtc ccctcccacg ccgtggaagc tttgttcttt cgctctttgc 3800

aataaatctt gctactgccc aaaa 3824

<210> 542
<211> 571
<212> PRT
<213> Homo Sapien

<400> 542
Met Glu Arg Ala Val Arg Val Glu Ser Gly Val Leu Val Gly Val
1 5 10 15

Val Cys Leu Leu Leu Ala Cys Pro Ala Thr Ala Thr Gly Pro Glu
20 25 30

Val Ala Gln Pro Glu Val Asp Thr Thr Leu Gly Arg Val Arg Gly
35 40 45

Arg Gln Val Gly Val Lys Gly Thr Asp Arg Leu Val Asn Val Phe
50 55 60

Leu Gly Ile Pro Phe Ala Gln Pro Pro Leu Gly Pro Asp Arg Phe
65 70 75

Ser Ala Pro His Pro Ala Gln Pro Trp Glu Gly Val Arg Asp Ala
80 85 90

Ser Thr Ala Pro Pro Met Cys Leu Gln Asp Val Glu Ser Met Asn
95 100 105

Ser Ser Arg Phe Val Leu Asn Gly Lys Gln Gln Ile Phe Ser Val
110 115 120

Ser Glu Asp Cys Leu Val Leu Asn Val Tyr Ser Pro Ala Glu Val
125 130 135

Pro Ala Gly Ser Gly Arg Pro Val Met Val Trp Val His Gly Gly
140 145 150

Ala Leu Ile Thr Gly Ala Ala Thr Ser Tyr Asp Gly Ser Ala Leu
155 160 165

Ala Ala Tyr Gly Asp Val Val Val Val Thr Val Gln Tyr Arg Leu
170 175 180

Gly Val Leu Gly Phe Phe Ser Thr Gly Asp Glu His Ala Pro Gly
185 190 195

Asn Gln Gly Phe Leu Asp Val Val Ala Ala Leu Arg Trp Val Gln
200 205 210

Glu Asn Ile Ala Pro Phe Gly Gly Asp Leu Asn Cys Val Thr Val

```
                215                 220                 225
Phe Gly Gly Ser Ala Gly Gly Ser Ile Ile Ser Gly Leu Val Leu
                230                 235                 240
Ser Pro Val Ala Ala Gly Leu Phe His Arg Ala Ile Thr Gln Ser
                245                 250                 255
Gly Val Ile Thr Thr Pro Gly Ile Ile Asp Ser His Pro Trp Pro
                260                 265                 270
Leu Ala Gln Lys Ile Ala Asn Thr Leu Ala Cys Ser Ser Ser Ser
                275                 280                 285
Pro Ala Glu Met Val Gln Cys Leu Gln Gln Lys Glu Gly Glu Glu
                290                 295                 300
Leu Val Leu Ser Lys Lys Leu Lys Asn Thr Ile Tyr Pro Leu Thr
                305                 310                 315
Val Asp Gly Thr Val Phe Pro Lys Ser Pro Lys Glu Leu Leu Lys
                320                 325                 330
Glu Lys Pro Phe His Ser Val Pro Phe Leu Met Gly Val Asn Asn
                335                 340                 345
His Glu Phe Ser Trp Leu Ile Pro Arg Gly Trp Gly Leu Leu Asp
                350                 355                 360
Thr Met Glu Gln Met Ser Arg Glu Asp Met Leu Ala Ile Ser Thr
                365                 370                 375
Pro Val Leu Thr Ser Leu Asp Val Pro Pro Glu Met Met Pro Thr
                380                 385                 390
Val Ile Asp Glu Tyr Leu Gly Ser Asn Ser Asp Ala Gln Ala Lys
                395                 400                 405
Cys Gln Ala Phe Gln Glu Phe Met Gly Asp Val Phe Ile Asn Val
                410                 415                 420
Pro Thr Val Ser Phe Ser Arg Tyr Leu Arg Asp Ser Gly Ser Pro
                425                 430                 435
Val Phe Phe Tyr Glu Phe Gln His Arg Pro Ser Ser Phe Ala Lys
                440                 445                 450
Ile Lys Pro Ala Trp Val Lys Ala Asp His Gly Ala Glu Gly Ala
                455                 460                 465
Phe Val Phe Gly Gly Pro Phe Leu Met Asp Glu Ser Ser Arg Leu
                470                 475                 480
Ala Phe Pro Glu Ala Thr Glu Glu Glu Lys Gln Leu Ser Leu Thr
                485                 490                 495
Met Met Ala Gln Trp Thr His Phe Ala Arg Thr Gly Asp Pro Asn
                500                 505                 510
Ser Lys Ala Leu Pro Pro Trp Pro Gln Phe Asn Gln Ala Glu Gln
                515                 520                 525
Tyr Leu Glu Ile Asn Pro Val Pro Arg Ala Gly Gln Lys Phe Arg
                530                 535                 540
```

```
Glu Ala Trp Met Gln Phe Trp Ser Glu Thr Leu Pro Ser Lys Ile
                545                 550                 555

Gln Gln Trp His Gln Lys Gln Lys Asn Arg Lys Ala Gln Glu Asp
                560                 565                 570

Leu
```

<210> 543
<211> 3721
<212> DNA
<213> Homo Sapien

<400> 543

```
tgtcgcctgg ccctcgccat gcagaccccg cgagcgtccc ctccccgccc 50

ggccctcctg cttctgctgc tgctactggg gggcgcccac ggcctctttc 100

ctgaggagcc gccgccgctt agcgtggccc ccagggacta cctgaaccac 150

tatcccgtgt ttgtgggcag cgggcccgga cgcctgaccc ccgcagaagg 200

tgctgacgac ctcaacatcc agcgagtcct gcgggtcaac aggacgctgt 250

tcattgggga cagggacaac ctctaccgcg tagagctgga gccccccacg 300

tccacggagc tgcggtacca gaggaagctg acctggagat ctaaccccag 350

cgacataaac gtgtgtcgga tgaagggcaa acaggagggc gagtgtcgaa 400

acttcgtaaa ggtgctgctc cttcgggacg agtccacgct ctttgtgtgc 450

ggttccaacg ccttcaaccc ggtgtgcgcc aactacagca tagacaccct 500

gcagcccgtc ggagacaaca tcagcggtat ggcccgctgc ccgtacgacc 550

ccaagcacgc caatgttgcc ctcttctctg acgggatgct cttcacagct 600

actgttaccg acttcctagc cattgatgct gtcatctacc gcagcctcgg 650

ggacaggccc accctgcgca ccgtgaaaca tgactccaag tggttcaaag 700

agccttactt tgtccatgcg gtggagtggg gcagccatgt ctacttcttc 750

ttccgggaga ttgcgatgga gtttaactac ctggagaagg tggtggtgtc 800

ccgcgtggcc cgagtgtgca agaacgacgt gggaggctcc ccccgcgtgc 850

tggagaagca gtggacgtcc ttcctgaagg cgcggctcaa ctgctctgta 900

cccggagact cccatttcta cttcaacgtg ctgcaggctg tcacgggcgt 950

ggtcagcctc gggggccggc ccgtggtcct ggccgttttt tccacgccca 1000

gcaacagcat ccctggctcg gctgtctgcg cctttgacct gacacaggtg 1050

gcagctgtgt ttgaaggccg cttccgagag cagaagtccc ccgagtccat 1100

ctggacgccg gtgccggagg atcaggtgcc tcgaccccgg cccgggtgct 1150

gcgcagcccc cgggatgcag tacaatgcct ccagcgcctt gccggatgac 1200
```

atcctcaact ttgtcaagac ccaccctctg atggacgagg cggtgccctc 1250
gctgggccat gcgccctgga tcctgcggac cctgatgagg caccagctga 1300
ctcgagtggc tgtggacgtg ggagccggcc cctggggcaa ccagaccgtt 1350
gtcttcctgg gttctgaggc ggggacggtc ctcaagttcc tcgtccggcc 1400
caatgccagc acctcaggga cgtctgggct cagtgtcttc ctggaggagt 1450
ttgagaccta ccggccggac aggtgtggac ggcccggcgg tggcgagaca 1500
gggcagcggc tgctgagctt ggagctggac gcagcttcgg ggggcctgct 1550
ggctgccttc cccgctgcg tggtccgagt gcctgtggct cgctgccagc 1600
agtactcggg gtgtatgaag aactgtatcg gcagtcagga cccctactgc 1650
gggtgggccc ccgacggctc ctgcatcttc ctcagcccgg gcaccagagc 1700
cgcctttgag caggacgtgt ccggggccag cacctcaggc ttaggggact 1750
gcacaggact cctgcgggcc agcctctccg aggaccgcgc ggggctggtg 1800
tcggtgaacc tgctggtaac gtcgtcggtg gcggccttcg tggtgggagc 1850
cgtggtgtcc ggcttcagcg tgggctggtt cgtgggcctc cgtgagcggc 1900
gggagctggc ccggcgcaag gacaaggagg ccatcctggc gcacggggcg 1950
ggcgaggcgg tgctgagcgt cagccgcctg ggcgagcgca gggcgcaggg 2000
tcccgggggc cggggcggag gcggtggcgg tggcgccggg gttcccccgg 2050
aggccctgct ggcgcccctg atgcagaacg gctgggccaa ggccacgctg 2100
ctgcagggcg ggccccacga cctggactcg gggctgctgc ccacgcccga 2150
gcagacgccg ctgccgcaga agcgcctgcc cactccgcac ccgcaccccc 2200
acgccctggg cccccgcgcc tgggaccacg gccacccct gctcccggcc 2250
tccgcttcat cctccctcct gctgctggcg cccgcccggg cccccgagca 2300
gcccccgcg cctggggagc cgacccccga cggccgcctc tatgctgccc 2350
ggcccggccg cgcctcccac ggcgacttcc cgctcacccc ccacgccagc 2400
ccggaccgcc ggcgggtggt gtccgcgccc acgggcccct tggacccagc 2450
ctcagccgcc gatggcctcc cgcggccctg gagcccgccc ccgacgggca 2500
gcctgaggag gccactgggc ccccacgccc ctccggccgc caccctgcgc 2550
cgcacccaca cgttcaacag cggcgaggcc cggcctgggg accgccaccg 2600
cggctgccac gcccggccgg gcacagactt ggcccacctc ctcccctatg 2650
ggggggcgga caggactgcg ccccccgtgc cctaggccgg gggcccccg 2700
atgccttggc agtgccagcc acgggaacca ggagcgagag acggtgccag 2750
aacgccgggg cccggggcaa ctccgagtgg gtgctcaagt cccccccgcg 2800

acccacccgc ggagtggggg gccccctccg ccacaaggaa gcacaaccag 2850

ctcgccctcc ccctacccgg ggccgcagga cgctgagacg gtttgggggt 2900

gggtgggcgg gaggactttg ctatggattt gaggttgacc ttatgcgcgt 2950

aggttttggt tttttttgc agttttggtt tcttttgcgg ttttctaacc 3000

aattgcacaa ctccgttctc ggggtggcgg caggcagggg aggcttggac 3050

gccggtgggg aatgggggc cacagctgca gacctaagcc ctcccccacc 3100

cctggaaagg tccctcccca acccaggccc ctggcgtgtg tgggtgtgcg 3150

tgcgtgtgcg tgccgtgttc gtgtgcaagg ggccggggag gtgggcgtgt 3200

gtgtgcgtgc cagcgaaggc tgctgtgggc gtgtgtgtca agtgggccac 3250

gcgtgcaggg tgtgtgtcca cgagcgacga tcgtggtggc cccagcggcc 3300

tgggcgttgg ctgagccgac gctggggctt ccagaaggcc cgggggtctc 3350

cgaggtgccg gttaggagtt tgaaccccc ccactctgca gagggaagcg 3400

gggacaatgc cggggtttca ggcaggagac acgaggaggg cctgcccgga 3450

agtcacatcg gcagcagctg tctaaagggc ttgggggcct ggggggcggc 3500

gaaggtgggt ggggcccctc tgtaaatacg gccccagggt ggtgagagag 3550

tcccatgcca cccgtcccct tgtgacctcc cccctatgac ctccagctga 3600

ccatgcatgc cacgtggctg gctgggtcct ctgccctctt tggagtttgc 3650

ctcccccagc cccctcccca tcaataaaac tctgtttaca accaaaaaaa 3700

aaaaaaaaaa aaaaaaaaaa a 3721

<210> 544
<211> 888
<212> PRT
<213> Homo Sapien

<400> 544

| | | | | | | | | | | | | | | |
|---|---|---|---|---|---|---|---|---|---|---|---|---|---|---|
| Met | Gln | Thr | Pro | Arg | Ala | Ser | Pro | Pro | Arg | Pro | Ala | Leu | Leu | Leu |
| 1 | | | | 5 | | | | | 10 | | | | | 15 |
| Leu | Leu | Leu | Leu | Leu | Gly | Gly | Ala | His | Gly | Leu | Phe | Pro | Glu | Glu |
| | | | | 20 | | | | | 25 | | | | | 30 |
| Pro | Pro | Pro | Leu | Ser | Val | Ala | Pro | Arg | Asp | Tyr | Leu | Asn | His | Tyr |
| | | | | 35 | | | | | 40 | | | | | 45 |
| Pro | Val | Phe | Val | Gly | Ser | Gly | Pro | Gly | Arg | Leu | Thr | Pro | Ala | Glu |
| | | | | 50 | | | | | 55 | | | | | 60 |
| Gly | Ala | Asp | Asp | Leu | Asn | Ile | Gln | Arg | Val | Leu | Arg | Val | Asn | Arg |
| | | | | 65 | | | | | 70 | | | | | 75 |
| Thr | Leu | Phe | Ile | Gly | Asp | Arg | Asp | Asn | Leu | Tyr | Arg | Val | Glu | Leu |
| | | | | 80 | | | | | 85 | | | | | 90 |
| Glu | Pro | Pro | Thr | Ser | Thr | Glu | Leu | Arg | Tyr | Gln | Arg | Lys | Leu | Thr |
| | | | | 95 | | | | | 100 | | | | | 105 |

```
Trp Arg Ser Asn Pro Ser Asp Ile Asn Val Cys Arg Met Lys Gly
                110                 115                 120

Lys Gln Glu Gly Glu Cys Arg Asn Phe Val Lys Val Leu Leu Leu
                125                 130                 135

Arg Asp Glu Ser Thr Leu Phe Val Cys Gly Ser Asn Ala Phe Asn
                140                 145                 150

Pro Val Cys Ala Asn Tyr Ser Ile Asp Thr Leu Gln Pro Val Gly
                155                 160                 165

Asp Asn Ile Ser Gly Met Ala Arg Cys Pro Tyr Asp Pro Lys His
                170                 175                 180

Ala Asn Val Ala Leu Phe Ser Asp Gly Met Leu Phe Thr Ala Thr
                185                 190                 195

Val Thr Asp Phe Leu Ala Ile Asp Ala Val Ile Tyr Arg Ser Leu
                200                 205                 210

Gly Asp Arg Pro Thr Leu Arg Thr Val Lys His Asp Ser Lys Trp
                215                 220                 225

Phe Lys Glu Pro Tyr Phe Val His Ala Val Glu Trp Gly Ser His
                230                 235                 240

Val Tyr Phe Phe Phe Arg Glu Ile Ala Met Glu Phe Asn Tyr Leu
                245                 250                 255

Glu Lys Val Val Val Ser Arg Val Ala Arg Val Cys Lys Asn Asp
                260                 265                 270

Val Gly Gly Ser Pro Arg Val Leu Glu Lys Gln Trp Thr Ser Phe
                275                 280                 285

Leu Lys Ala Arg Leu Asn Cys Ser Val Pro Gly Asp Ser His Phe
                290                 295                 300

Tyr Phe Asn Val Leu Gln Ala Val Thr Gly Val Val Ser Leu Gly
                305                 310                 315

Gly Arg Pro Val Val Leu Ala Val Phe Ser Thr Pro Ser Asn Ser
                320                 325                 330

Ile Pro Gly Ser Ala Val Cys Ala Phe Asp Leu Thr Gln Val Ala
                335                 340                 345

Ala Val Phe Glu Gly Arg Phe Arg Glu Gln Lys Ser Pro Glu Ser
                350                 355                 360

Ile Trp Thr Pro Val Pro Glu Asp Gln Val Pro Arg Pro Arg Pro
                365                 370                 375

Gly Cys Cys Ala Ala Pro Gly Met Gln Tyr Asn Ala Ser Ser Ala
                380                 385                 390

Leu Pro Asp Asp Ile Leu Asn Phe Val Lys Thr His Pro Leu Met
                395                 400                 405

Asp Glu Ala Val Pro Ser Leu Gly His Ala Pro Trp Ile Leu Arg
                410                 415                 420
```

Thr Leu Met Arg His Gln Leu Thr Arg Val Ala Val Asp Val Gly
425 430 435

Ala Gly Pro Trp Gly Asn Gln Thr Val Val Phe Leu Gly Ser Glu
440 445 450

Ala Gly Thr Val Leu Lys Phe Leu Val Arg Pro Asn Ala Ser Thr
455 460 465

Ser Gly Thr Ser Gly Leu Ser Val Phe Leu Glu Glu Phe Glu Thr
470 475 480

Tyr Arg Pro Asp Arg Cys Gly Arg Pro Gly Gly Gly Glu Thr Gly
485 490 495

Gln Arg Leu Leu Ser Leu Glu Leu Asp Ala Ala Ser Gly Gly Leu
500 505 510

Leu Ala Ala Phe Pro Arg Cys Val Val Arg Val Pro Val Ala Arg
515 520 525

Cys Gln Gln Tyr Ser Gly Cys Met Lys Asn Cys Ile Gly Ser Gln
530 535 540

Asp Pro Tyr Cys Gly Trp Ala Pro Asp Gly Ser Cys Ile Phe Leu
545 550 555

Ser Pro Gly Thr Arg Ala Ala Phe Glu Gln Asp Val Ser Gly Ala
560 565 570

Ser Thr Ser Gly Leu Gly Asp Cys Thr Gly Leu Leu Arg Ala Ser
575 580 585

Leu Ser Glu Asp Arg Ala Gly Leu Val Ser Val Asn Leu Leu Val
590 595 600

Thr Ser Ser Val Ala Ala Phe Val Val Gly Ala Val Val Ser Gly
605 610 615

Phe Ser Val Gly Trp Phe Val Gly Leu Arg Glu Arg Arg Glu Leu
620 625 630

Ala Arg Arg Lys Asp Lys Glu Ala Ile Leu Ala His Gly Ala Gly
635 640 645

Glu Ala Val Leu Ser Val Ser Arg Leu Gly Glu Arg Arg Ala Gln
650 655 660

Gly Pro Gly Gly Arg Gly Gly Gly Gly Gly Gly Gly Ala Gly Val
665 670 675

Pro Pro Glu Ala Leu Leu Ala Pro Leu Met Gln Asn Gly Trp Ala
680 685 690

Lys Ala Thr Leu Leu Gln Gly Gly Pro His Asp Leu Asp Ser Gly
695 700 705

Leu Leu Pro Thr Pro Glu Gln Thr Pro Leu Pro Gln Lys Arg Leu
710 715 720

Pro Thr Pro His Pro His Pro His Ala Leu Gly Pro Arg Ala Trp
725 730 735

Asp His Gly His Pro Leu Leu Pro Ala Ser Ala Ser Ser Ser Leu

740 745 750

Leu Leu Leu Ala Pro Ala Arg Ala Pro Glu Gln Pro Pro Ala Pro
755 760 765

Gly Glu Pro Thr Pro Asp Gly Arg Leu Tyr Ala Ala Arg Pro Gly
770 775 780

Arg Ala Ser His Gly Asp Phe Pro Leu Thr Pro His Ala Ser Pro
785 790 795

Asp Arg Arg Arg Val Val Ser Ala Pro Thr Gly Pro Leu Asp Pro
800 805 810

Ala Ser Ala Ala Asp Gly Leu Pro Arg Pro Trp Ser Pro Pro Pro
815 820 825

Thr Gly Ser Leu Arg Arg Pro Leu Gly Pro His Ala Pro Pro Ala
830 835 840

Ala Thr Leu Arg Arg Thr His Thr Phe Asn Ser Gly Glu Ala Arg
845 850 855

Pro Gly Asp Arg His Arg Gly Cys His Ala Arg Pro Gly Thr Asp
860 865 870

Leu Ala His Leu Leu Pro Tyr Gly Gly Ala Asp Arg Thr Ala Pro
875 880 885

Pro Val Pro

<210> 545
<211> 1571
<212> DNA
<213> Homo Sapien

<400> 545
gatggcgcag ccacagcttc tgtgagattc gatttctccc cagttcccct 50

gtgggtctga ggggaccaga agggtgagct acgttggctt tctggaaggg 100

gaggctatat gcgtcaattc cccaaaacaa gttttgacat ttcccctgaa 150

atgtcattct ctatctattc actgcaagtg cctgctgttc caggccttac 200

ctgctgggca ctaacggcgg agccaggatg ggacagaat aaaggagcca 250

cgacctgtgc caccaactcg cactcagact ctgaactcag acctgaaatc 300

ttctcttcac gggaggcttg gcagtttttc ttactcctgt ggtctccaga 350

tttcaggcct aagatgaaag cctctagtct tgccttcagc cttctctctg 400

ctgcgtttta tctcctatgg actccttcca ctggactgaa gacactcaat 450

ttgggaagct gtgtgatcgc cacaaacctt caggaaatac gaaatggatt 500

ttctgagata cggggcagtg tgcaagccaa agatggaaac attgacatca 550

gaatcttaag gaggactgag tctttgcaag acacaaagcc tgcgaatcga 600

tgctgcctcc tgcgccattt gctaagactc tatctggaca gggtatttaa 650

aaactaccag acccctgacc attatactct ccggaagatc agcagcctcg 700

ccaattcctt tcttaccatc aagaaggacc tccggctctc tcatgcccac 750

atgacatgcc attgtgggga ggaagcaatg aagaaataca gccagattct 800

gagtcacttt gaaaagctgg aacctcaggc agcagttgtg aaggctttgg 850

gggaactaga cattcttctg caatggatgg aggagacaga ataggaggaa 900

agtgatgctg ctgctaagaa tattcgaggt caagagctcc agtcttcaat 950

acctgcagag gaggcatgac cccaaaccac catctcttta ctgtactagt 1000

cttgtgctgg tcacagtgta tcttatttat gcattacttg cttccttgca 1050

tgattgtctt tatgcatccc caatcttaat tgagaccata cttgtataag 1100

atttttgtaa tatctttctg ctattggata tatttattag ttaatatatt 1150

tatttatttt ttgctattta atgtatttat ttttttactt ggacatgaaa 1200

ctttaaaaaa attcacagat tatatttata acctgactag agcaggtgat 1250

gtatttttat acagtaaaaa aaaaaaacct tgtaaattct agaagagtgg 1300

ctagggggt tattcatttg tattcaacta aggacatatt tactcatgct 1350

gatgctctgt gagatatttg aaattgaacc aatgactact taggatgggt 1400

tgtggaataa gttttgatgt ggaattgcac atctacctta caattactga 1450

ccatccccag tagactcccc agtcccataa ttgtgtatct tccagccagg 1500

aatcctacac ggccagcatg tatttctaca aataaagttt tctttgcata 1550

ccaaaaaaaa aaaaaaaaaa a 1571

<210> 546
<211> 261
<212> PRT
<213> Homo Sapien

<400> 546

```
Met Arg Gln Phe Pro Lys Thr Ser Phe Asp Ile Ser Pro Glu Met
1               5                   10                  15

Ser Phe Ser Ile Tyr Ser Leu Gln Val Pro Ala Val Pro Gly Leu
                20                  25                  30

Thr Cys Trp Ala Leu Thr Ala Glu Pro Gly Trp Gly Gln Asn Lys
                35                  40                  45

Gly Ala Thr Thr Cys Ala Thr Asn Ser His Ser Asp Ser Glu Leu
                50                  55                  60

Arg Pro Glu Ile Phe Ser Ser Arg Glu Ala Trp Gln Phe Phe Leu
                65                  70                  75

Leu Leu Trp Ser Pro Asp Phe Arg Pro Lys Met Lys Ala Ser Ser
                80                  85                  90

Leu Ala Phe Ser Leu Leu Ser Ala Ala Phe Tyr Leu Leu Trp Thr
                95                  100                 105
```

```
Pro Ser Thr Gly Leu Lys Thr Leu Asn Leu Gly Ser Cys Val Ile
                110                 115                 120

Ala Thr Asn Leu Gln Glu Ile Arg Asn Gly Phe Ser Glu Ile Arg
                125                 130                 135

Gly Ser Val Gln Ala Lys Asp Gly Asn Ile Asp Ile Arg Ile Leu
                140                 145                 150

Arg Arg Thr Glu Ser Leu Gln Asp Thr Lys Pro Ala Asn Arg Cys
                155                 160                 165

Cys Leu Leu Arg His Leu Leu Arg Leu Tyr Leu Asp Arg Val Phe
                170                 175                 180

Lys Asn Tyr Gln Thr Pro Asp His Tyr Thr Leu Arg Lys Ile Ser
                185                 190                 195

Ser Leu Ala Asn Ser Phe Leu Thr Ile Lys Lys Asp Leu Arg Leu
                200                 205                 210

Ser His Ala His Met Thr Cys His Cys Gly Glu Glu Ala Met Lys
                215                 220                 225

Lys Tyr Ser Gln Ile Leu Ser His Phe Glu Lys Leu Glu Pro Gln
                230                 235                 240

Ala Ala Val Val Lys Ala Leu Gly Glu Leu Asp Ile Leu Leu Gln
                245                 250                 255

Trp Met Glu Glu Thr Glu
                260
```

<210> 547
<211> 2014
<212> DNA
<213> Homo Sapien

<400> 547

```
agcaactcaa gttcatcatt gtcctgagag agaggagcag cgcggttctc 50
ggccgggaca gcagaacgcc aggggaccct cacctgggcg cgccggggca 100
cgggctttga ttgtcctggg gtcgcggaga cccgcgcgcc tgccctgcac 150
gccgggcggc aacctttgca gtcgcgttgg ctgctgcgat cggccggcgg 200
gtccctgccg aaggctcggc tgcttctgtc cacctcttac acttcttcat 250
ttatcggtgg atcatttcga gagtccgtct tgtaaatgtt tggcactttg 300
ctactttatt gcttctttct ggcgacagtt ccagcactcg ccgagaccgg 350
cggagaaagg cagctgagcc cggagaagag cgaaatatgg ggacccgggc 400
taaaagcaga cgtcgtcctt cccgcccgct atttctatat tcaggcagtg 450
gatacatcag ggaataaatt cacatcttct ccaggcgaaa aggtcttcca 500
ggtgaaagtc tcagcaccag aggagcaatt cactagagtt ggagtccagg 550
ttttagaccg aaaagatggg tccttcatag taagatacag aatgtatgca 600
```

agctacaaaa atctgaaggt ggaaattaaa ttccaagggc aacatgtggc 650
caaatcccca tatattttaa aagggccggt ttaccatgag aactgtgact 700
gtcctctgca agatagtgca gcctggctac gggagatgaa ctgccctgaa 750
accattgctc agattcagag agatctggca catttccctg ctgtggatcc 800
agaaaagatt gcagtagaaa tcccaaaaag atttggacag aggcagagcc 850
tatgtcacta caccttaaag gataacaagg tttatatcaa gactcatggt 900
gaacatgtag gttttagaat tttcatggat gccatactac tttctttgac 950
tagaaaggtg aagatgccag atgtggagct ctttgttaat ttgggagact 1000
ggcctttgga aaaaaagaaa tccaattcaa acatccatcc gatcttttcc 1050
tggtgtggct ccacagattc caaggatatc gtgatgccta cgtacgattt 1100
gactgattct gttctggaaa ccatgggccg ggtaagtctg gatatgatgt 1150
ccgtgcaagc taacacgggt cctccctggg aaagcaaaaa ttccactgcc 1200
gtctggagag ggcgagacag ccgcaaagag agactcgagc tggttaaact 1250
cagtagaaaa cacccagaac tcatagacgc tgctttcacc aactttttct 1300
tctttaaaca cgatgaaaac ctgtatggtc ccattgtgaa acatatttca 1350
ttttttgatt tcttcaagca taagtatcaa ataaatatcg atggcactgt 1400
agcagcttat cgcctgccat atttgctagt tggtgacagt gttgtgctga 1450
agcaggattc catctactat gaacattttt acaatgagct gcagccctgg 1500
aaacactaca ttccagttaa gagcaacctg agcgatctgc tagaaaaact 1550
taaatgggcg aaagatcacg atgaagaggc caaaaagata gcaaaagcag 1600
gacaagaatt tgcaagaaat aatctcatgg gcgatgacat attctgttat 1650
tatttcaaac ttttccagga atatgccaat ttacaagtga gtgagcccca 1700
aatccgagag ggcatgaaaa gggtagaacc acagactgag gacgacctct 1750
tcccttgtac ttgccatagg aaaaagacca aagatgaact ctgatatgca 1800
aaataacttc tattagaata atggtgctct gaagactctt cttaactaaa 1850
aagaagaatt tttttaagta ttaattccat ggacaatata aaatctgtgt 1900
gattgtttgc agtatgaaga cacatttcta cttatgcagt attctcatga 1950
ctgtacttta aagtacattt ttagaatttt ataataaaac cacctttatt 2000
ttaaaggaaa aaaa 2014

<210> 548
<211> 502
<212> PRT
<213> Homo Sapien

<400> 548

Met Phe Gly Thr Leu Leu Leu Tyr Cys Phe Phe Leu Ala Thr Val
1               5                   10                  15

Pro Ala Leu Ala Glu Thr Gly Gly Glu Arg Gln Leu Ser Pro Glu
                20                  25                  30

Lys Ser Glu Ile Trp Gly Pro Gly Leu Lys Ala Asp Val Val Leu
                35                  40                  45

Pro Ala Arg Tyr Phe Tyr Ile Gln Ala Val Asp Thr Ser Gly Asn
                50                  55                  60

Lys Phe Thr Ser Ser Pro Gly Glu Lys Val Phe Gln Val Lys Val
                65                  70                  75

Ser Ala Pro Glu Glu Gln Phe Thr Arg Val Gly Val Gln Val Leu
                80                  85                  90

Asp Arg Lys Asp Gly Ser Phe Ile Val Arg Tyr Arg Met Tyr Ala
                95                  100                 105

Ser Tyr Lys Asn Leu Lys Val Glu Ile Lys Phe Gln Gly Gln His
                110                 115                 120

Val Ala Lys Ser Pro Tyr Ile Leu Lys Gly Pro Val Tyr His Glu
                125                 130                 135

Asn Cys Asp Cys Pro Leu Gln Asp Ser Ala Ala Trp Leu Arg Glu
                140                 145                 150

Met Asn Cys Pro Glu Thr Ile Ala Gln Ile Gln Arg Asp Leu Ala
                155                 160                 165

His Phe Pro Ala Val Asp Pro Glu Lys Ile Ala Val Glu Ile Pro
                170                 175                 180

Lys Arg Phe Gly Gln Arg Gln Ser Leu Cys His Tyr Thr Leu Lys
                185                 190                 195

Asp Asn Lys Val Tyr Ile Lys Thr His Gly Glu His Val Gly Phe
                200                 205                 210

Arg Ile Phe Met Asp Ala Ile Leu Leu Ser Leu Thr Arg Lys Val
                215                 220                 225

Lys Met Pro Asp Val Glu Leu Phe Val Asn Leu Gly Asp Trp Pro
                230                 235                 240

Leu Glu Lys Lys Lys Ser Asn Ser Asn Ile His Pro Ile Phe Ser
                245                 250                 255

Trp Cys Gly Ser Thr Asp Ser Lys Asp Ile Val Met Pro Thr Tyr
                260                 265                 270

Asp Leu Thr Asp Ser Val Leu Glu Thr Met Gly Arg Val Ser Leu
                275                 280                 285

Asp Met Met Ser Val Gln Ala Asn Thr Gly Pro Pro Trp Glu Ser
                290                 295                 300

Lys Asn Ser Thr Ala Val Trp Arg Gly Arg Asp Ser Arg Lys Glu
                305                 310                 315

Arg Leu Glu Leu Val Lys Leu Ser Arg Lys His Pro Glu Leu Ile

320 325 330

Asp Ala Ala Phe Thr Asn Phe Phe Phe Phe Lys His Asp Glu Asn
335 340 345

Leu Tyr Gly Pro Ile Val Lys His Ile Ser Phe Phe Asp Phe Phe
350 355 360

Lys His Lys Tyr Gln Ile Asn Ile Asp Gly Thr Val Ala Ala Tyr
365 370 375

Arg Leu Pro Tyr Leu Leu Val Gly Asp Ser Val Val Leu Lys Gln
380 385 390

Asp Ser Ile Tyr Tyr Glu His Phe Tyr Asn Glu Leu Gln Pro Trp
395 400 405

Lys His Tyr Ile Pro Val Lys Ser Asn Leu Ser Asp Leu Leu Glu
410 415 420

Lys Leu Lys Trp Ala Lys Asp His Asp Glu Glu Ala Lys Lys Ile
425 430 435

Ala Lys Ala Gly Gln Glu Phe Ala Arg Asn Asn Leu Met Gly Asp
440 445 450

Asp Ile Phe Cys Tyr Tyr Phe Lys Leu Phe Gln Glu Tyr Ala Asn
455 460 465

Leu Gln Val Ser Glu Pro Gln Ile Arg Glu Gly Met Lys Arg Val
470 475 480

Glu Pro Gln Thr Glu Asp Asp Leu Phe Pro Cys Thr Cys His Arg
485 490 495

Lys Lys Thr Lys Asp Glu Leu
500

<210> 549
<211> 1088
<212> DNA
<213> Homo Sapien

<400> 549
gggtgattga actaaacctt cgccgcaccg agtttgcagt acggccgtca 50

cccgcaccgc tgcctgcttg cggttggaga aatcaaggcc ctaccgggcc 100

tccgtagtca cctctctata gtgggcgtgg ccgaggccgg ggtgaccctg 150

ccggagcctc cgctgccagc gacatgttca aggtaattca gaggtccgtg 200

gggccagcca gcctgagctt gctcaccttc aaagtctatg cagcaccaaa 250

aaaggactca cctcccaaaa attccgtgaa ggttgatgag ctttcactct 300

actcagttcc tgagggtcaa tcgaagtatg tggaggaggc aaggagccag 350

cttgaagaaa gcatctcaca gctccgacac tattgcgagc catacacaac 400

ctggtgtcag gaaacgtact cccaaactaa gcccaagatg caaagtttgg 450

ttcaatgggg gttagacagc tatgactatc tccaaaatgc acctcctgga 500

ttttttccga gacttggtgt tattggtttt gctggcctta ttggactcct 550

tttggctaga ggttcaaaaa taaagaagct agtgtatccg cctggtttca 600

tgggattagc tgcctccctc tattatccac aacaagccat cgtgtttgcc 650

caggtcagtg gggagagatt atatgactgg ggtttacgag gatatatagt 700

catagaagat ttgtggaagg agaactttca aaagccagga aatgtgaaga 750

attcacctgg aactaagtag aaaactccat gctctgccat cttaatcagt 800

tataggtaaa cattggaaac tccatagaat aaatcagtat ttctacagaa 850

aaatggcata gaagtcagta ttgaatgtat taaattggct ttcttcttca 900

ggaaaaacta gaccagacct ctgttatctt ctgtgaaatc atcctacaag 950

caaactaacc tggaatccct tcacctagag ataatgtaca agccttagaa 1000

ctcctcattc tcatgttgct atttatgtac ctaattaaaa cccaagttta 1050

aaaaaaaaaa aaaaaaaaaa aaaaaaaaaa aaaaaaaa 1088

<210> 550
<211> 198
<212> PRT
<213> Homo Sapien

<400> 550

```
Met Phe Lys Val Ile Gln Arg Ser Val Gly Pro Ala Ser Leu Ser
  1               5                  10                  15

Leu Leu Thr Phe Lys Val Tyr Ala Ala Pro Lys Lys Asp Ser Pro
                 20                  25                  30

Pro Lys Asn Ser Val Lys Val Asp Glu Leu Ser Leu Tyr Ser Val
                 35                  40                  45

Pro Glu Gly Gln Ser Lys Tyr Val Glu Glu Ala Arg Ser Gln Leu
                 50                  55                  60

Glu Glu Ser Ile Ser Gln Leu Arg His Tyr Cys Glu Pro Tyr Thr
                 65                  70                  75

Thr Trp Cys Gln Glu Thr Tyr Ser Gln Thr Lys Pro Lys Met Gln
                 80                  85                  90

Ser Leu Val Gln Trp Gly Leu Asp Ser Tyr Asp Tyr Leu Gln Asn
                 95                 100                 105

Ala Pro Pro Gly Phe Phe Pro Arg Leu Gly Val Ile Gly Phe Ala
                110                 115                 120

Gly Leu Ile Gly Leu Leu Leu Ala Arg Gly Ser Lys Ile Lys Lys
                125                 130                 135

Leu Val Tyr Pro Pro Gly Phe Met Gly Leu Ala Ala Ser Leu Tyr
                140                 145                 150

Tyr Pro Gln Gln Ala Ile Val Phe Ala Gln Val Ser Gly Glu Arg
                155                 160                 165

Leu Tyr Asp Trp Gly Leu Arg Gly Tyr Ile Val Ile Glu Asp Leu
```

```
                  170                 175                 180
Trp Lys Glu Asn Phe Gln Lys Pro Gly Asn Val Lys Asn Ser Pro
                  185                 190                 195
Gly Thr Lys
```

## Description

### FIELD OF THE INVENTION

The present invention relates generally to the identification and isolation of novel DNA and to the recombinant production of novel polypeptides.

### BACKGROUND OF THE INVENTION

Extracellular proteins play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci. 93:7108-7113 (1996); U.S. Patent No. 5,536,637)].

Membrane-bound proteins and receptors can play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesin molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immunoadhesins, for instance, can be employed as therapeutic agents to block receptor-ligand interactions. The membrane-bound proteins can also be employed for screening of potential peptide or small molecule inhibitors of the relevant receptor/ligand interaction.

Efforts are being undertaken by both industry and academia to identify new, native receptor or membrane-bound proteins. Many efforts are focused on the screening of mammalian recombinant DNA libraries to identify the coding sequences for novel receptor or membrane-bound proteins.

### SUMMARY OF THE INVENTION

In one embodiment, the invention provides an isolated nucleic acid molecule comprising a nucleotide sequence that encodes a PRO polypeptide.

In one aspect, the isolated nucleic acid molecule comprises a nucleotide sequence having at least about 80 % nucleic acid sequence identity, alternatively at least about 81% nucleic acid sequence identity, alternatively at least about 82 % nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity. alternatively at least about 91 % nucleic acid sequence identity, alternatively at least about 92% nucleic acid sequence identity, alternatively at least about 93 % nucleic acid sequence identity, alternatively at least about 94 % nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81 % nucleic acid sequence identity, alternatively at least about 82 % nucleic acid sequence identity, alternatively at least about 83 % nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92 % nucleic acid sequence identity, alternatively at least about 93 % nucleic acid sequence identity, alternatively at least about 94 % nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80 % nucleic acid sequence identity, alternatively at least about 81 % nucleic acid sequence identity, alternatively at least about 82% nucleic acid sequence identity, alternatively at least about 83 % nucleic acid sequence identity, alternatively at least about 84% nucleic acid sequence identity, alternatively at least about 85 % nucleic acid sequence identity, alternatively at least about 86 % nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92 % nucleic acid sequence identity, alternatively at least about 93 % nucleic acid sequence identity, alternatively at least about 94% nucleic acid sequence identity, alternatively at least about 95% nucleic acid sequence identity, alternatively at least about 96 % nucleic acid sequence identity, alternatively at least about 97 % nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 10 nucleotides in length, alternatively at least about 15 nucleotides in length, alternatively at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences hereinabove identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83 % amino acid sequence identity, alternatively at least about 84 % amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96 % amino acid sequence identity, alternatively at least about 97 % amino acid sequence identity, alternatively at least about 98 % amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81 % amino acid sequence identity, alternatively at least about 82 % amino acid sequence identity, alternatively at least about 83 % amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97 % amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a specific aspect, the invention provides an isolated PRO polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as hereinbefore described, or an anti-PRO antibody, for the preparation of a medicament useful in the treatment of a condition which is responsive to the PRO polypeptide, an agonist or antagonist thereof or an anti-PRO antibody.

In other embodiments of the present invention, the invention provides vectors comprising DNA encoding any of the herein described polypeptides. Host cell comprising any such vector are also provided. By way of example, the host cells may be CHO cells, E. *coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which binds, preferably specifically, to any of the above or below described polypeptides. Optionally, the antibody is a monoclonal antibody, humanized antibody, antibody fragment or single-chain antibody.

In yet other embodiments, the invention provides oligonucleotide probes which may be useful for isolating genomic and cDNA nucleotide sequences, measuring or detecting expression of an associated gene or as antisense probes, wherein those probes may be derived from any of the above or below described nucleotide sequences. Preferred probe lengths are described above.

In yet other embodiments, the present invention is directed to methods of using the PRO polypeptides of the present invention for a variety of uses based upon the functional biological assay data presented in the Examples below.

## BRIEF DESCRIPTION OF THE DRAWINGS

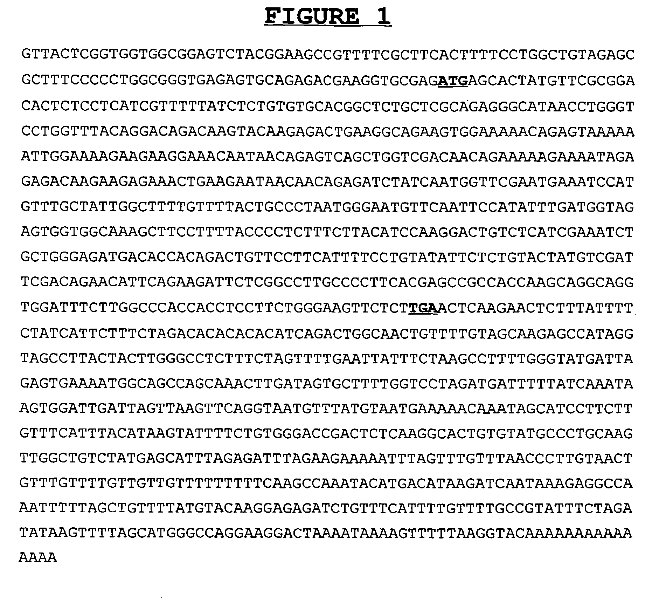

Figure 1 shows a nucleotide sequence (SEQ ID NO:1) of a native sequence PR0177 cDNA, wherein SEQ ID NO: 1 is a clone designated herein as "DNA16438-1387".

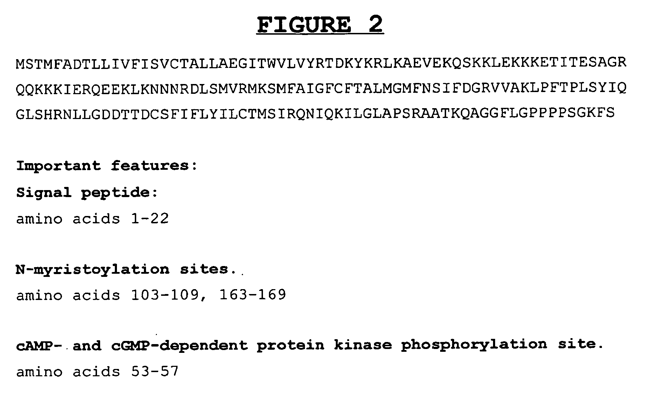

Figure 2 shows the amino acid sequence (SEQ ID NO:2) derived from the coding sequence of SEQ ID NO:1 shown in Figure 1.

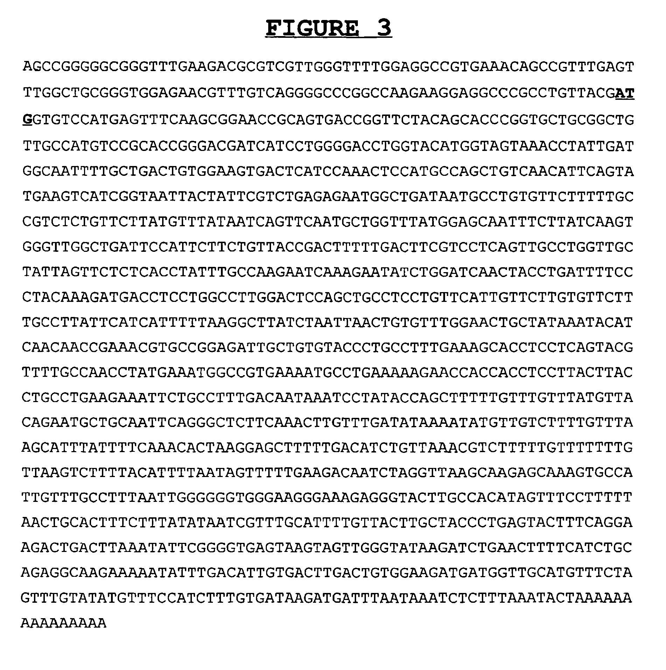

Figure 3 shows a nucleotide sequence (SEQ ID NO:3) of a native sequence PR03574 cDNA, wherein SEQ ID NO: 3 is a clone designated herein as "DNA19360-2552".

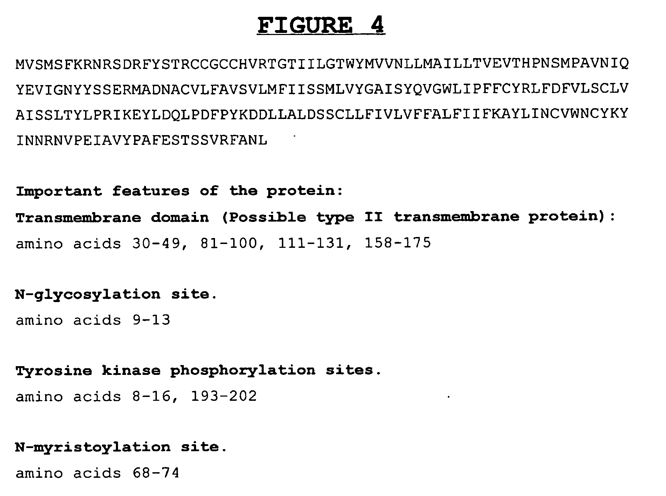

Figure 4 shows the amino acid sequence (SEQ ID NO:4) derived from the coding sequence of SEQ ID NO:3 shown in Figure 3.

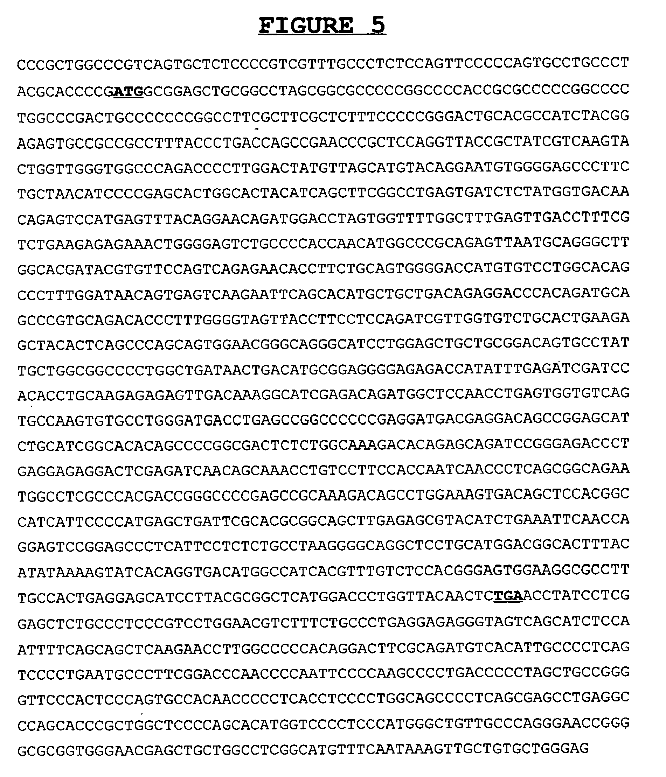

Figure 5 shows a nucleotide sequence (SEQ ID NO:5) of a native sequence PRO1280 cDNA, wherein SEQ ID NO: 5 is a clone designated herein as "DNA33455-1548".

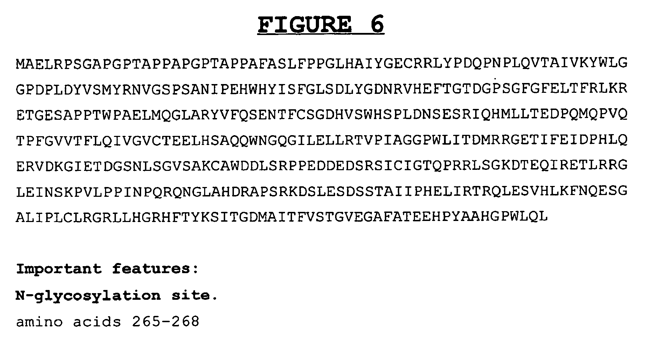

Figure 6 shows the amino acid sequence (SEQ ID NO:6) derived from the coding sequence of SEQ ID NO:5 shown in Figure 5.

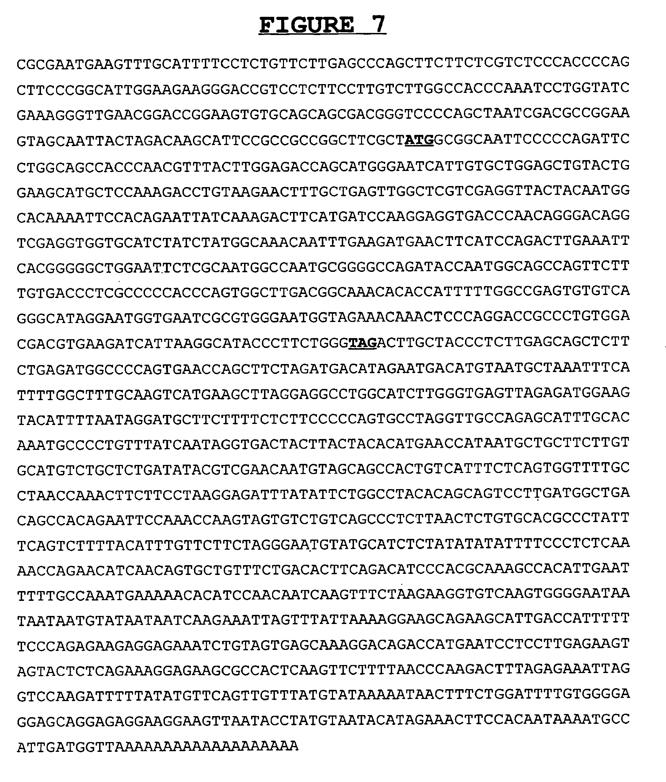

Figure 7 shows a nucleotide sequence (SEQ ID NO:7) of a native sequence PR04984 cDNA, wherein SEQ ID NO: 7 is a clone designated herein as "DNA37155-2651".

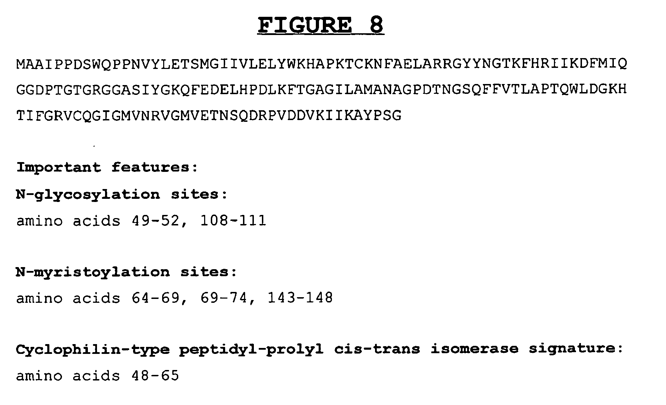

Figure 8 shows the amino acid sequence (SEQ ID NO:8) derived from the coding sequence of SEQ ID NO:7 shown in Figure 7.

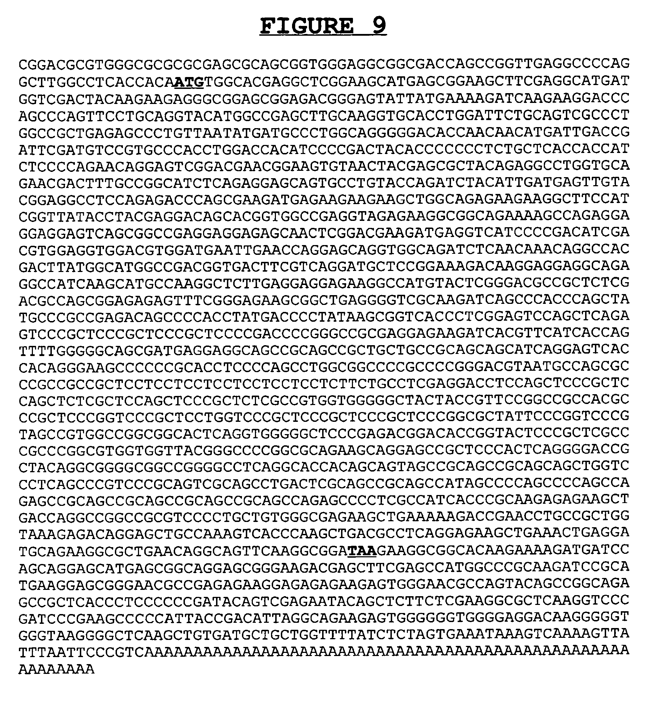

Figure 9 shows a nucleotide sequence (SEQ ID NO:9) of a native sequence PR04988 cDNA, wherein SEQ ID NO: 9 is a clone designated herein as "DNA38269-2654".

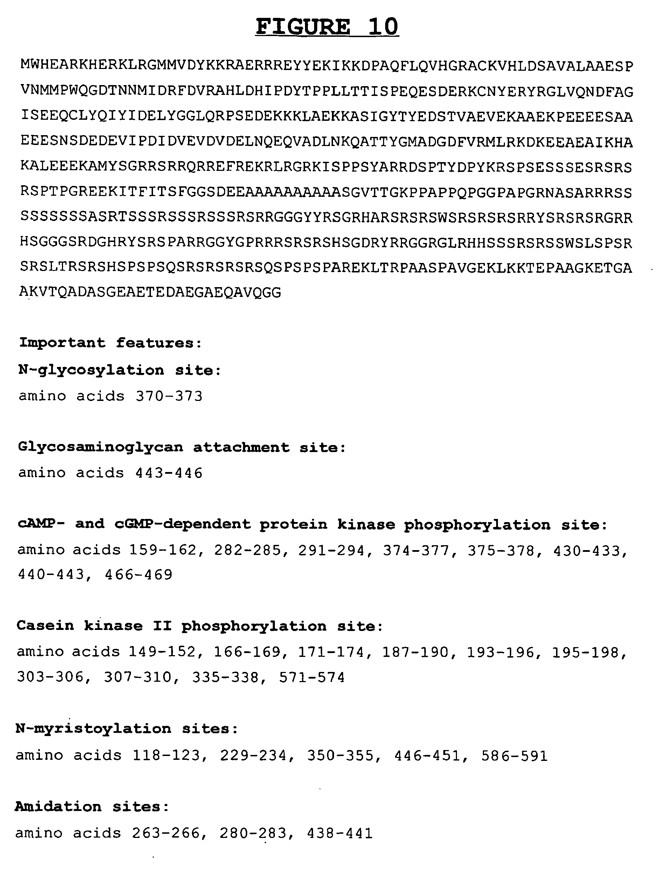

Figure 10 shows the amino acid sequence (SEQ ID NO:10) derived from the coding sequence of SEQ ID NO:9 shown in Figure 9.

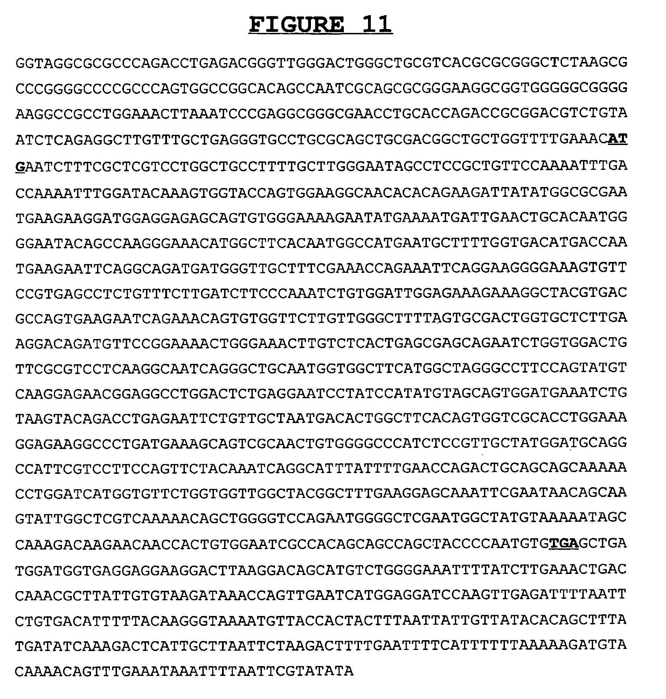

Figure 11 shows a nucleotide sequence (SEQ ID NO: 11) of a native sequence PR0305 cDNA, wherein SEQ ID NO:11 is a clone designated herein as "DNA40619-1220".

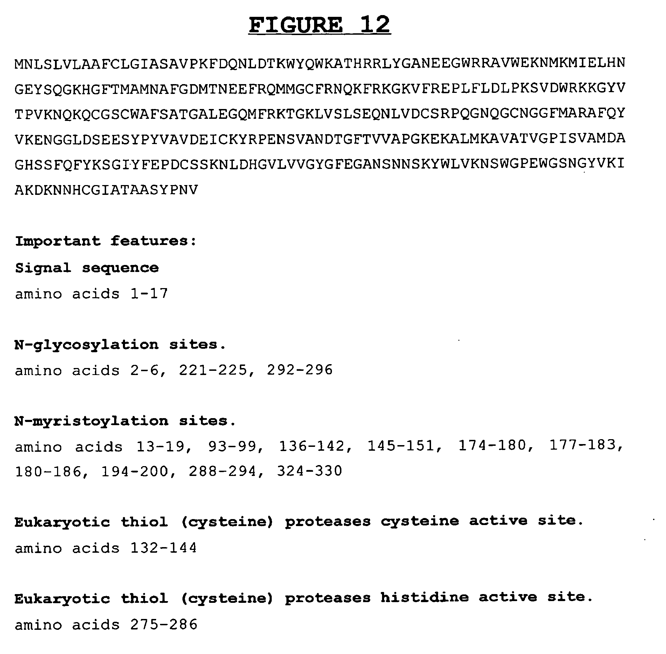

Figure 12 shows the amino acid sequence (SEQ ID NO: 12) derived from the coding sequence of SEQ ID NO:11 shown in Figure 11.

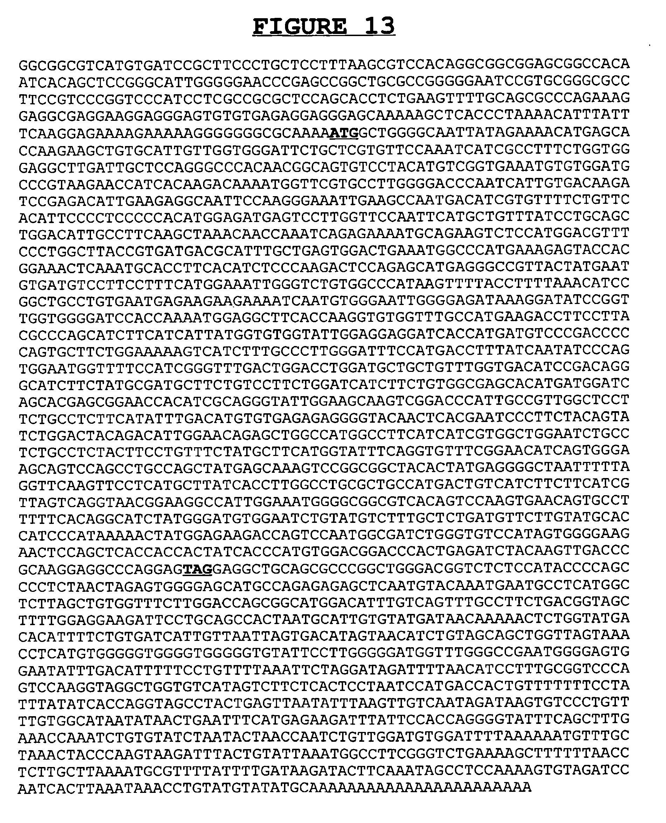

Figure 13 shows a nucleotide sequence (SEQ ID NO:13) of a native sequence PRO 1866 cDNA, wherein SEQ ID NO: 13 is a clone designated herein as "DNA44174-2513".

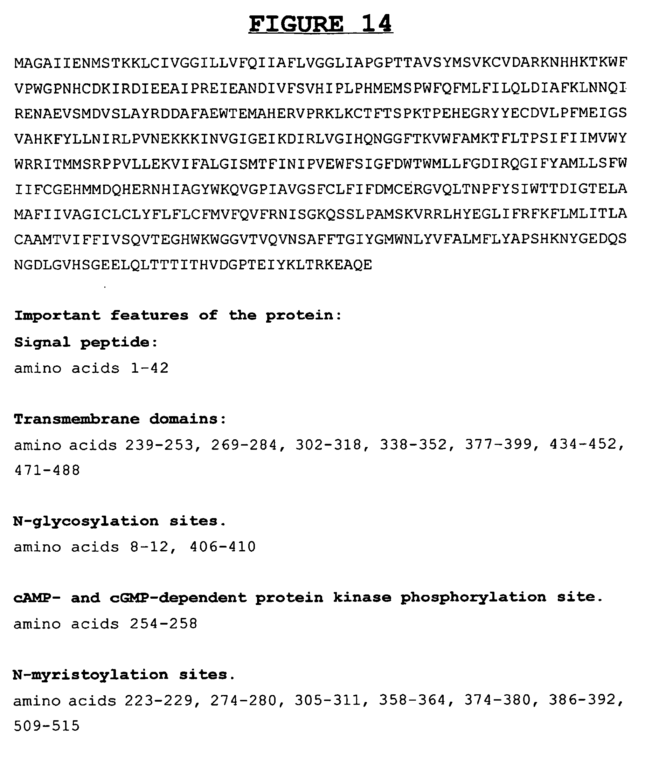

Figure 14 shows the amino acid sequence (SEQ ID NO: 14) derived from the coding sequence of SEQ ID NO: 13 shown in Figure 13.

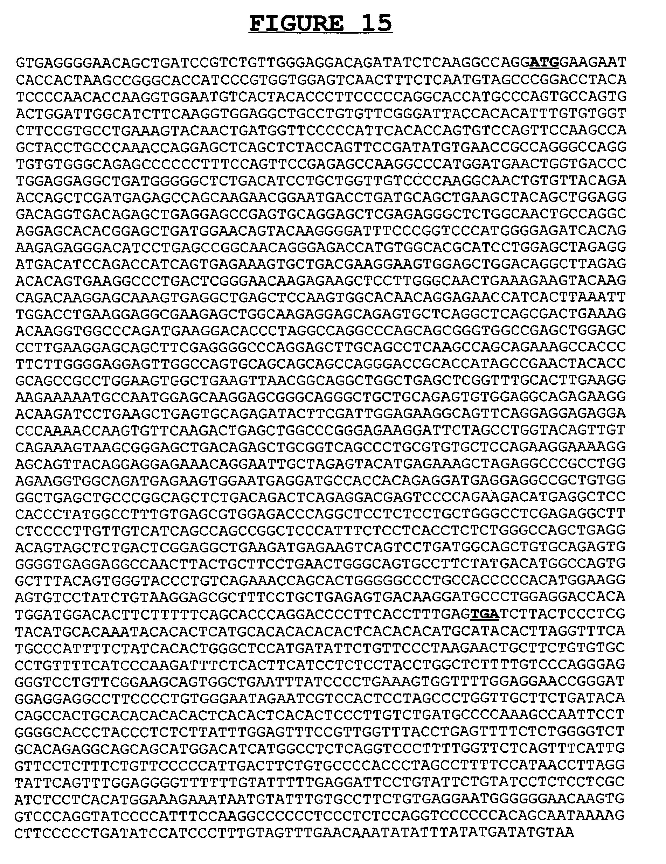

Figure 15 shows a nucleotide sequence (SEQ ID NO:15) of a native sequence PR04996 cDNA, wherein SEQ ID NO: 15 is a clone designated herein as "DNA44675-2662".

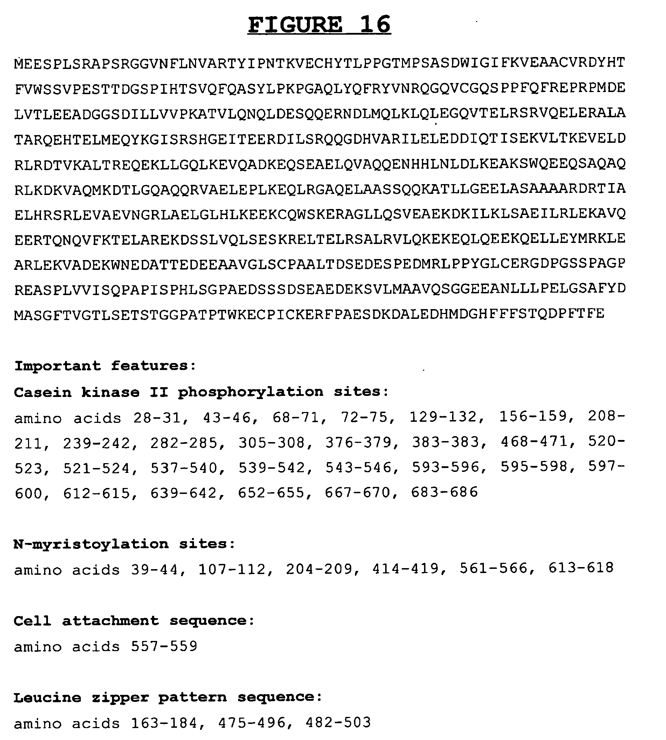

Figure 16 shows the amino acid sequence (SEQ ID NO:16) derived from the coding sequence of SEQ ID NO: 15 shown in Figure 15.

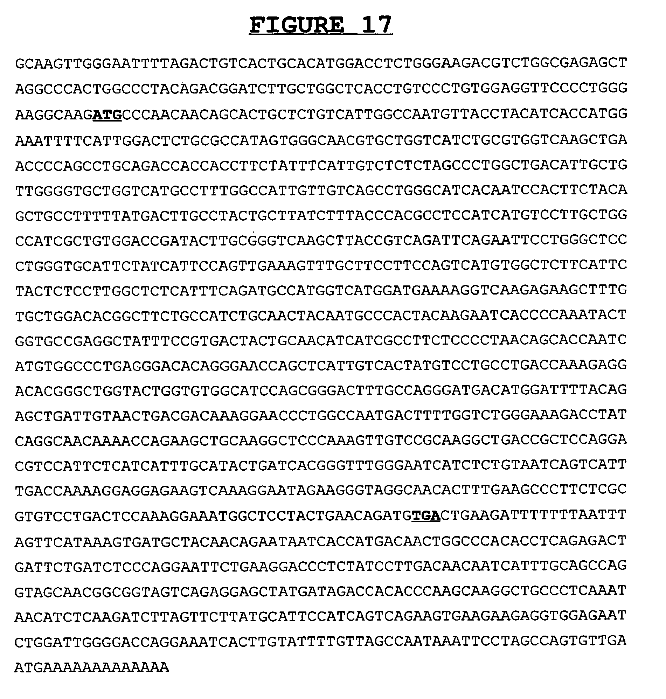

Figure 17 shows a nucleotide sequence (SEQ ID NO:17) of a native sequence PR04406 cDNA, wherein SEQ ID NO: 17 is a clone designated herein as "DNA45408-2615".

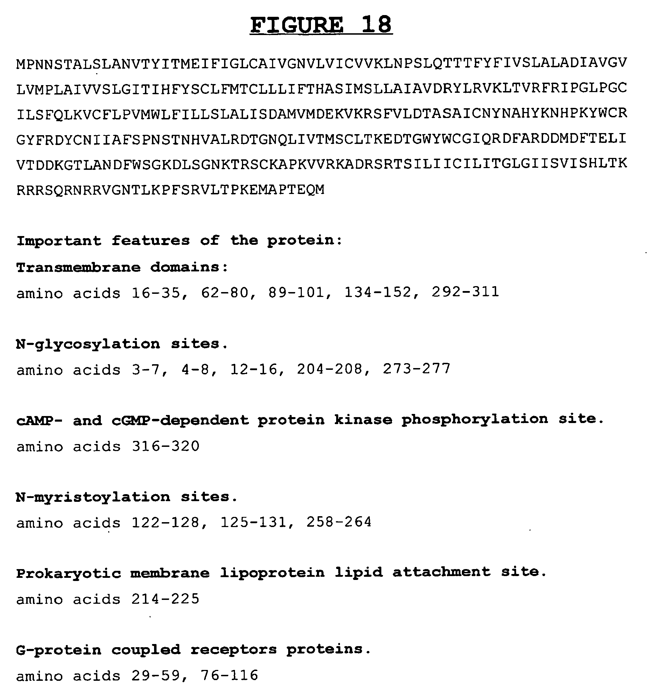

Figure 18 shows the amino acid sequence (SEQ ID NO:18) derived from the coding sequence of SEQ ID NO:17 shown in Figure 17.

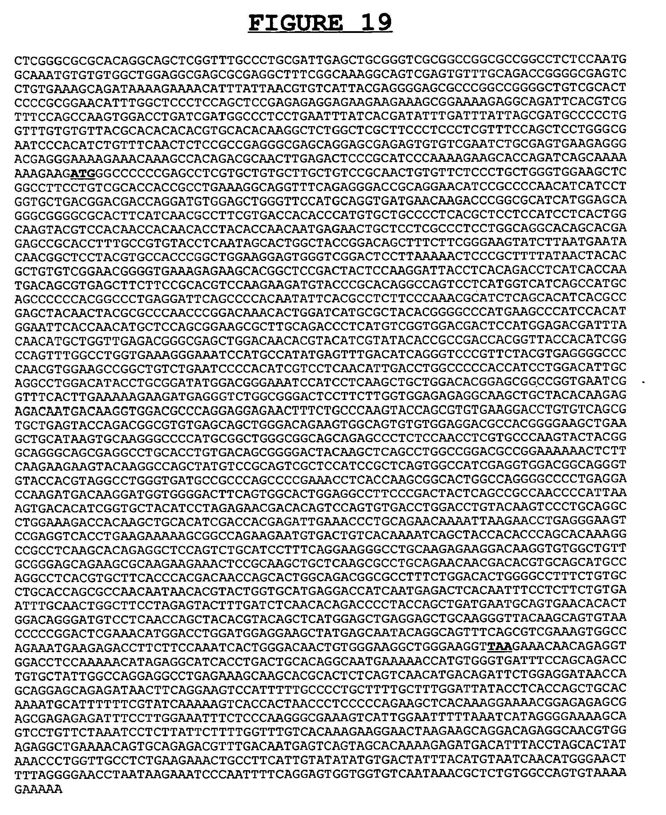

Figure 19 shows a nucleotide sequence (SEQ ID NO: 19) of a native sequence PRO1120 cDNA, wherein SEQ ID NO:19 is a clone designated herein as "DNA48606-1479".

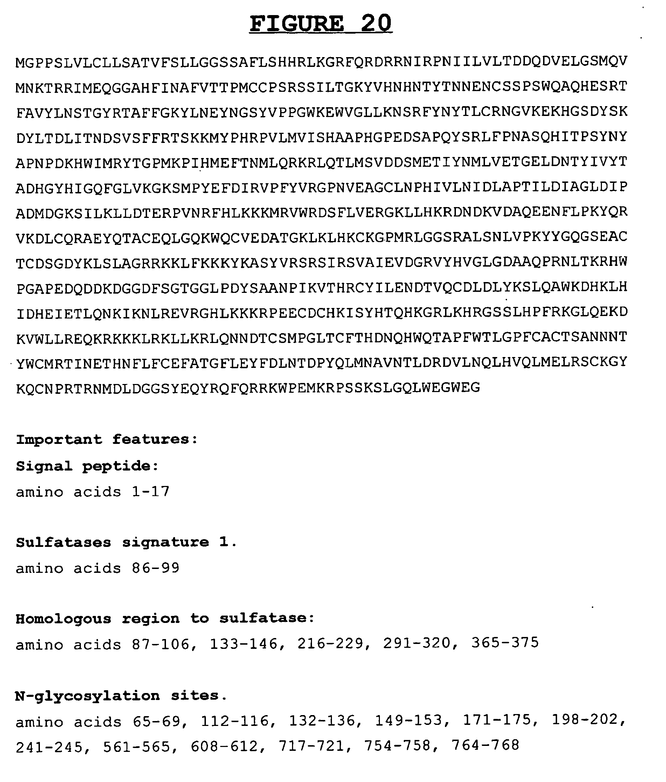

Figure 20 shows the amino acid sequence (SEQ ID NO:20) derived from the coding sequence of SEQ ID NO:19 shown in Figure 19.

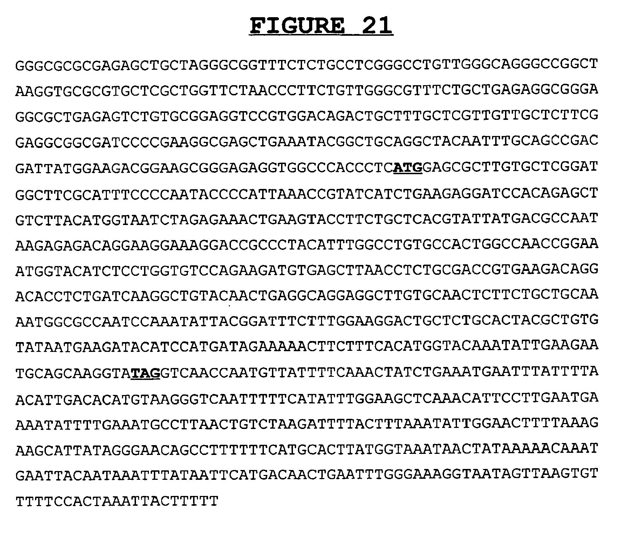

Figure 21 shows a nucleotide sequence (SEQ ID NO:21) of a native sequence PR04990 cDNA, wherein SEQ ID NO:21 is a clone designated herein as "DNA52753-2656".

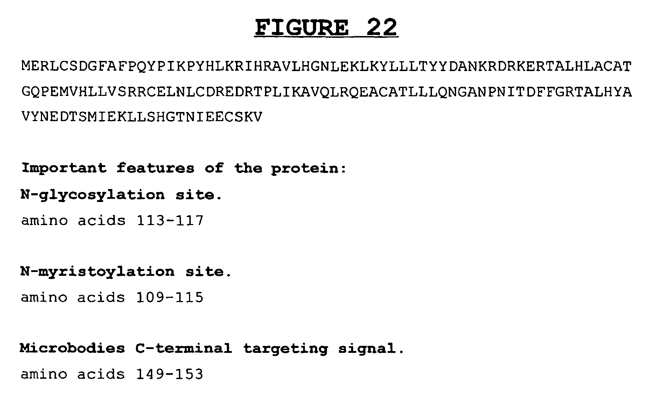

Figure 22 shows the amino acid sequence (SEQ ID NO:22) derived from the coding sequence of SEQ ID NO:21 shown in Figure 21.

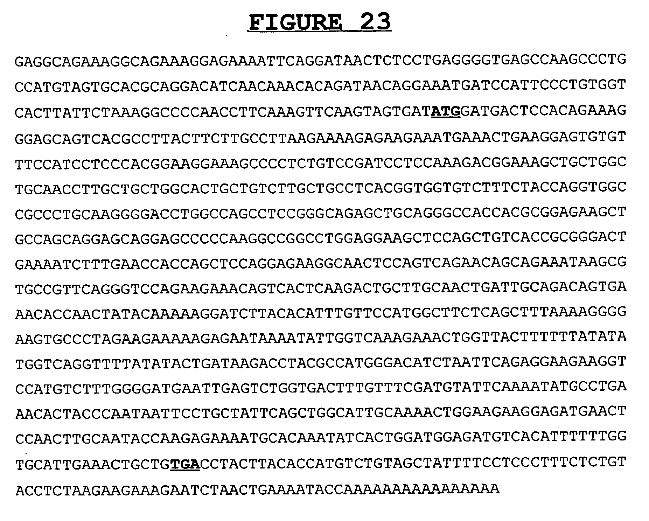

Figure 23 shows a nucleotide sequence (SEQ ID NO:23) of a native sequence PR0738 cDNA, wherein SEQ ID NO:23 is a clone designated herein as "DNA53915-1258".

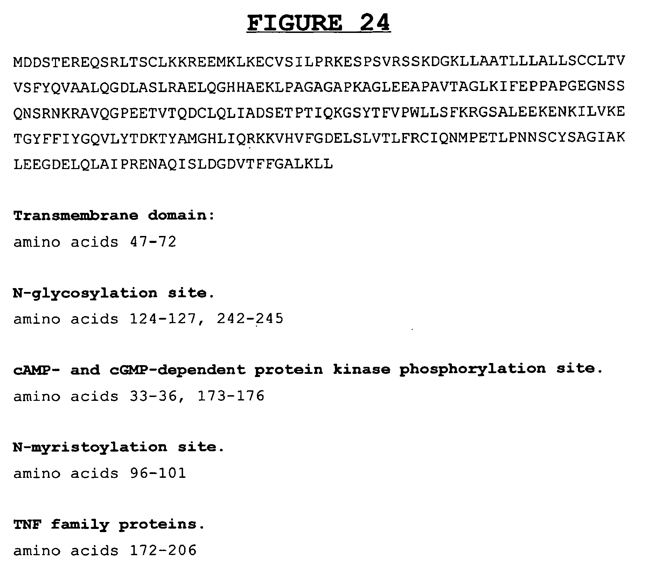

Figure 24 shows the amino acid sequence (SEQ ID NO:24) derived from the coding sequence of SEQ ID NO:23 shown in Figure 23.

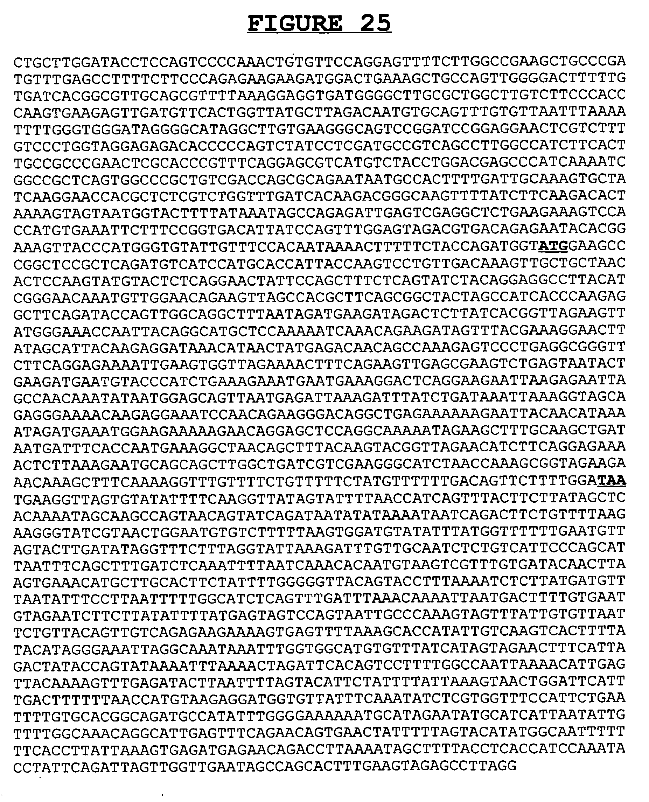

Figure 25 shows a nucleotide sequence (SEQ ID NO:25) of a native sequence PR03577 cDNA, wherein SEQ ID NO:25 is a clone designated herein as "DNA53991-2553".

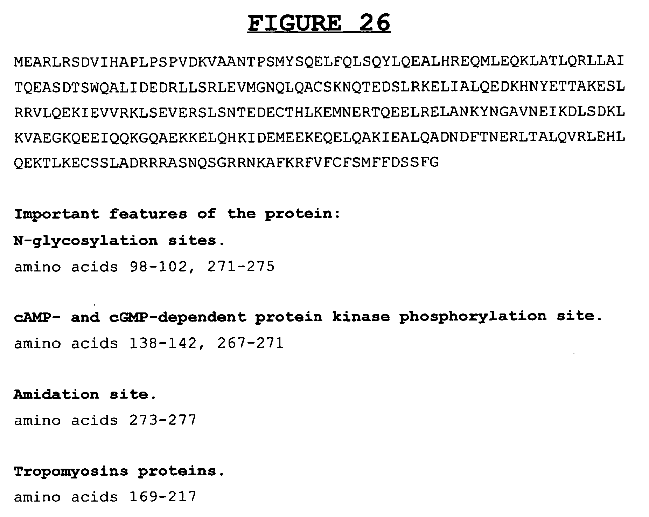

Figure 26 shows the amino acid sequence (SEQ ID NO:26) derived from the coding sequence of SEQ ID NO:25 shown in Figure 25.

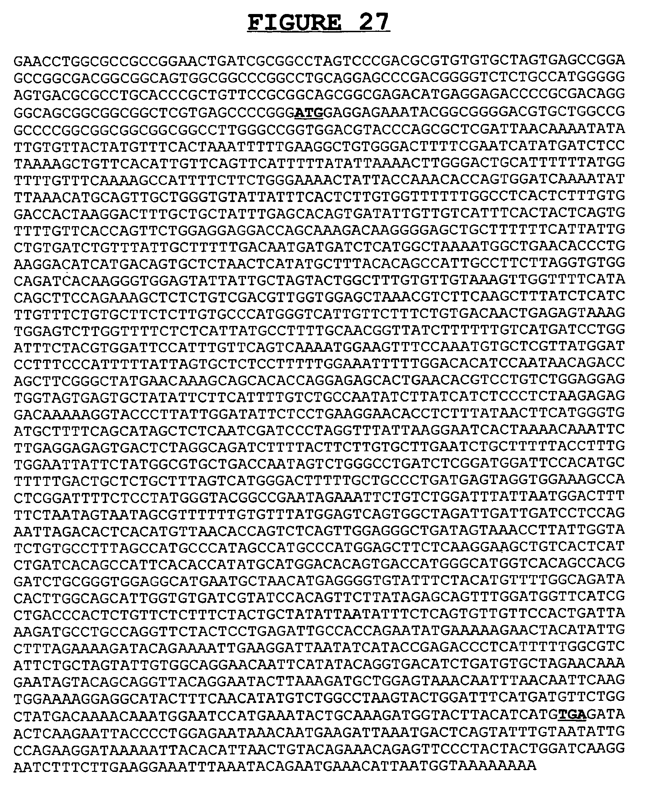

Figure 27 shows a nucleotide sequence (SEQ ID NO:27) of a native sequence PRO 1879 cDNA, wherein SEQ ID NO:27 is a clone designated herein as "DNA54009-2517".

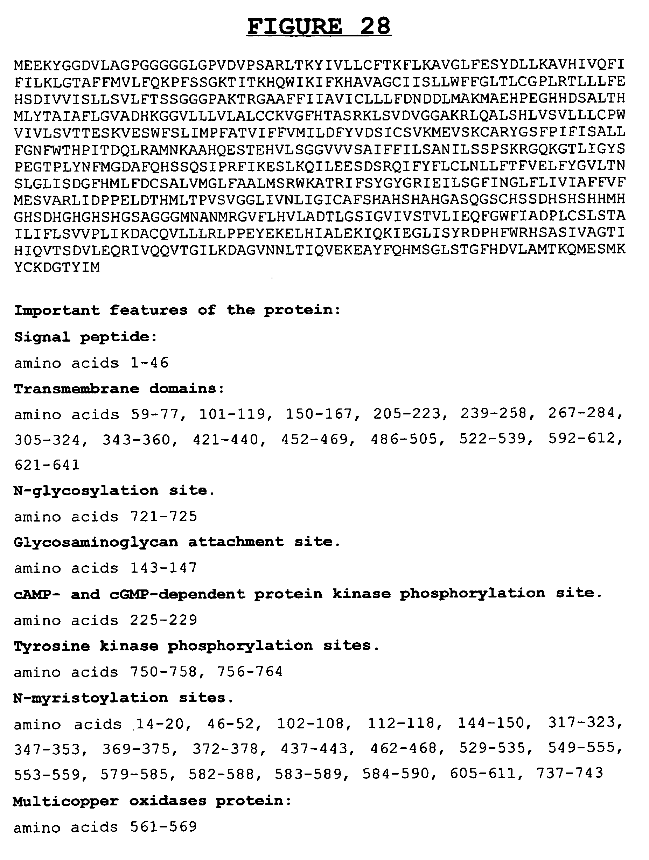

Figure 28 shows the amino acid sequence (SEQ ID NO:28) derived from the coding sequence of SEQ ID NO:27 shown in Figure 27.

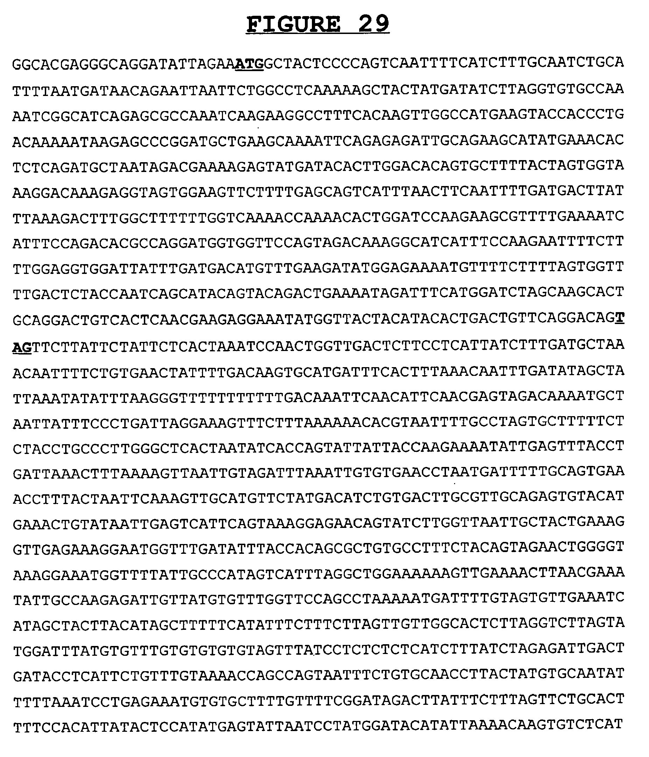

Figure 29 shows a nucleotide sequence (SEQ ID NO:29) of a native sequence PRO1471 cDNA, wherein SEQ ID NO:29 is a clone designated herein as "DNA56055-1643".

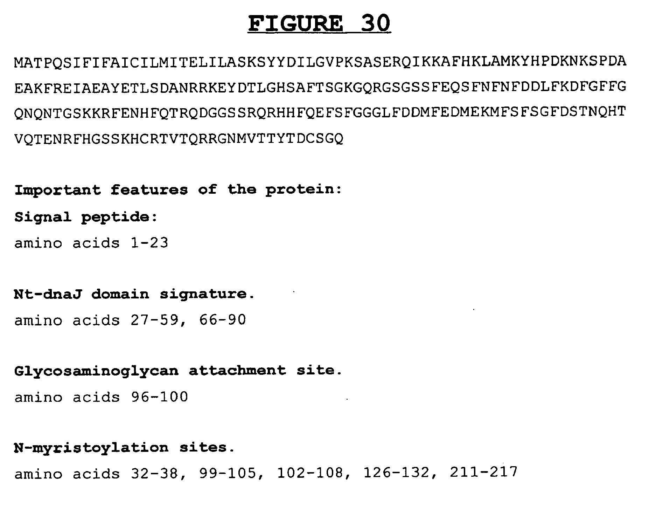

Figure 30 shows the amino acid sequence (SEQ ID NO:30) derived from the coding sequence of SEQ ID NO:29 shown in Figure 29.

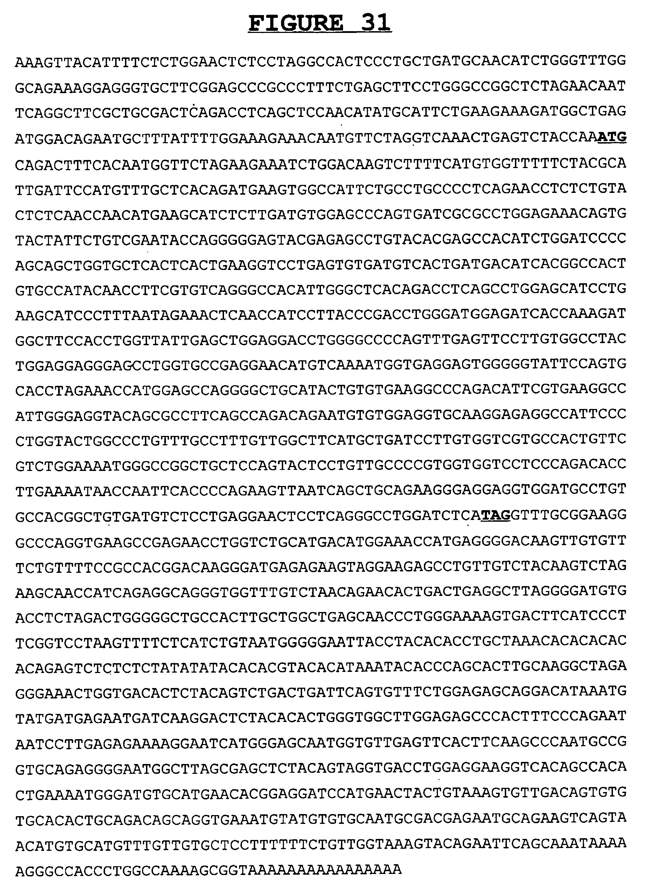

Figure 31 shows a nucleotide sequence (SEQ ID NO:31) of a native sequence PRO1114 cDNA, wherein SEQ ID NO:31 is a clone designated herein as "DNA57033-1403".

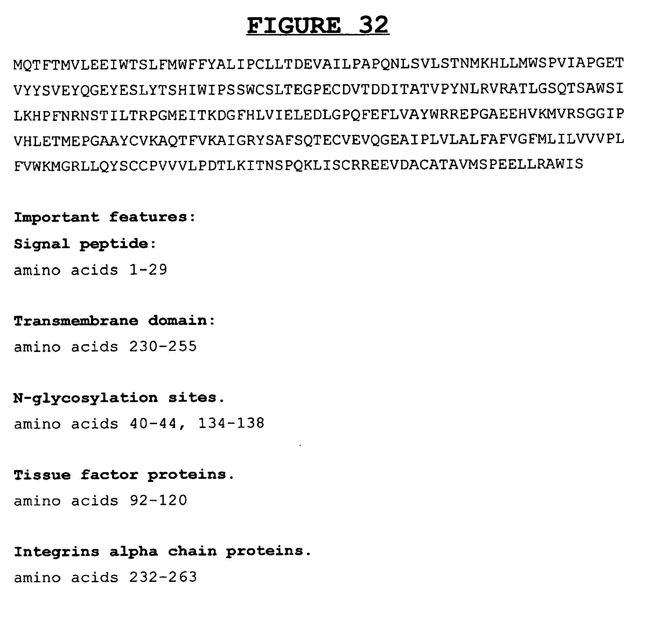

Figure 32 shows the amino acid sequence (SEQ ID NO:32) derived from the coding sequence of SEQ ID NO:31 shown in Figure 31.

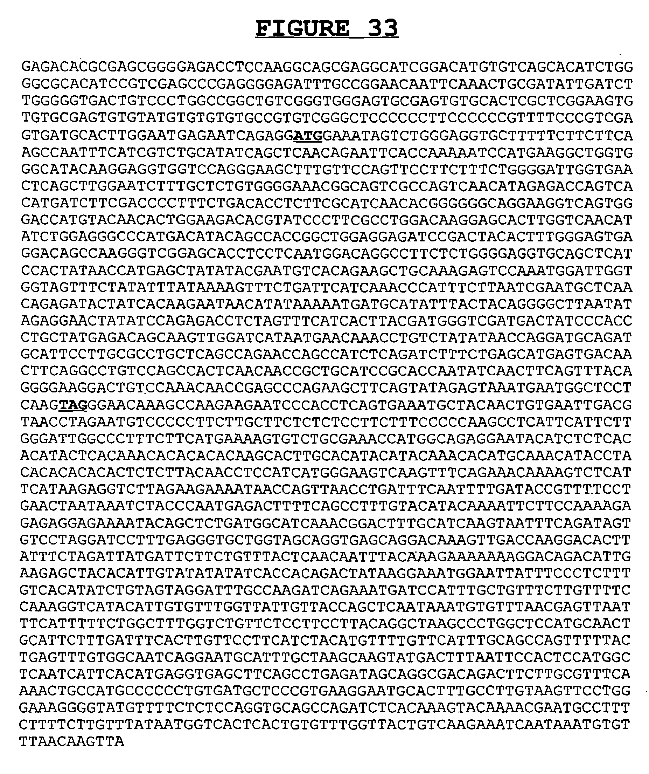

Figure 33 shows a nucleotide sequence (SEQ ID NO:33) of a native sequence PRO1076 cDNA, wherein SEQ ID NO:33 is a clone designated herein as "DNA57252-1453".

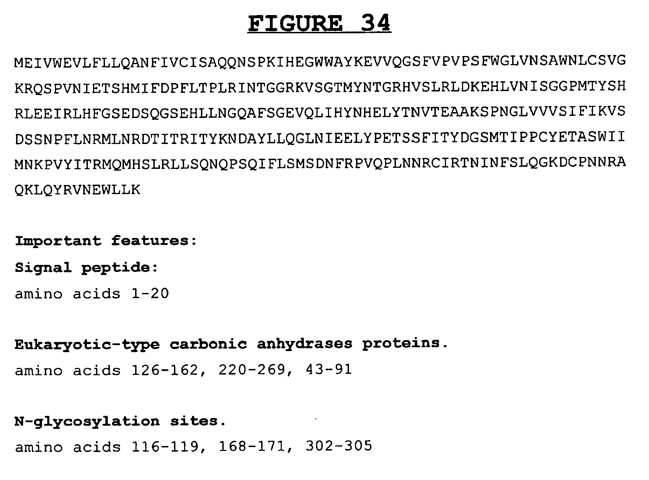

Figure 34 shows the amino acid sequence (SEQ ID NO:34) derived from the coding sequence of SEQ ID NO:33 shown in Figure 33.

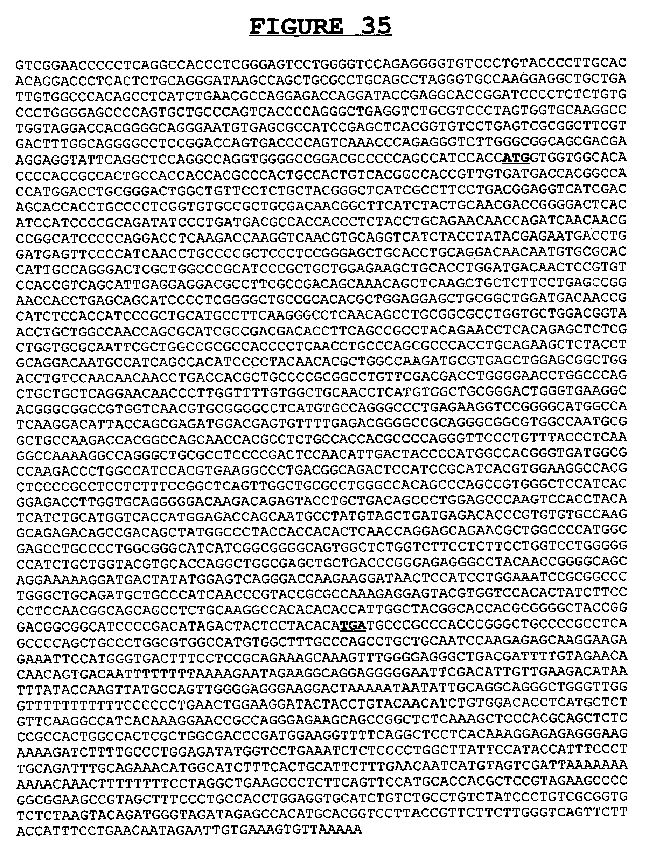

Figure 35 shows a nucleotide sequence (SEQ ID NO:35) of a native sequence PRO1483 cDNA, wherein SEQ ID NO:35 is a clone designated herein as "DNA58799-1652".

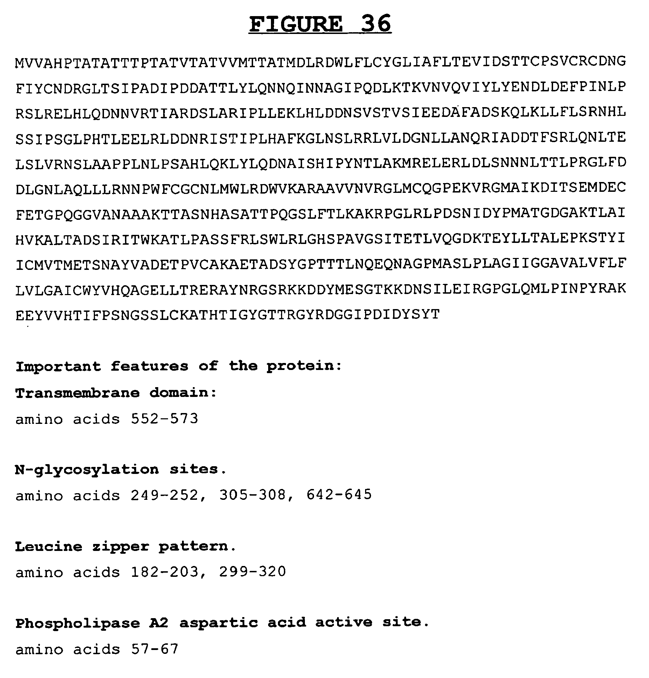

Figure 36 shows the amino acid sequence (SEQ ID NO:36) derived from the coding sequence of SEQ ID NO:35 shown in Figure 35.

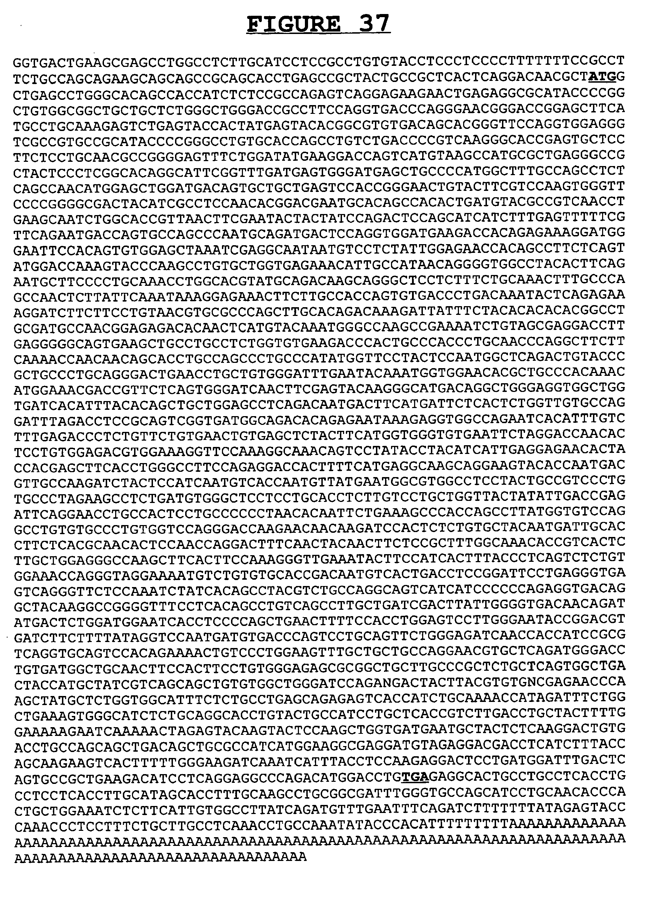

Figure 37 shows a nucleotide sequence (SEQ ID NO:37) of a native sequence PR04985 cDNA, wherein SEQ ID NO:37 is a clone designated herein as "DNA59770-2652".

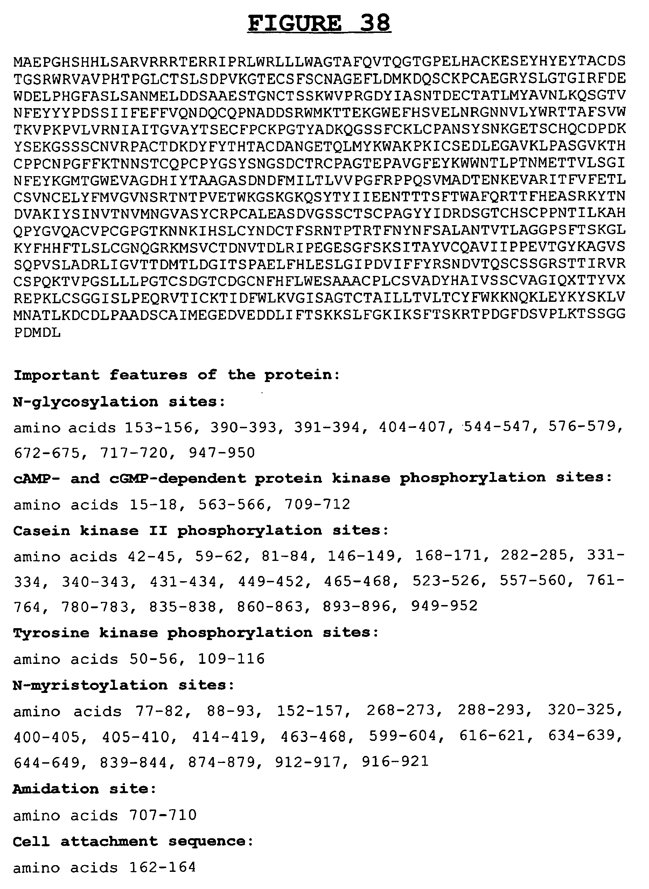

Figure 38 shows the amino acid sequence (SEQ ID NO:38) derived from the coding sequence of SEQ ID NO:37 shown in Figure 37.

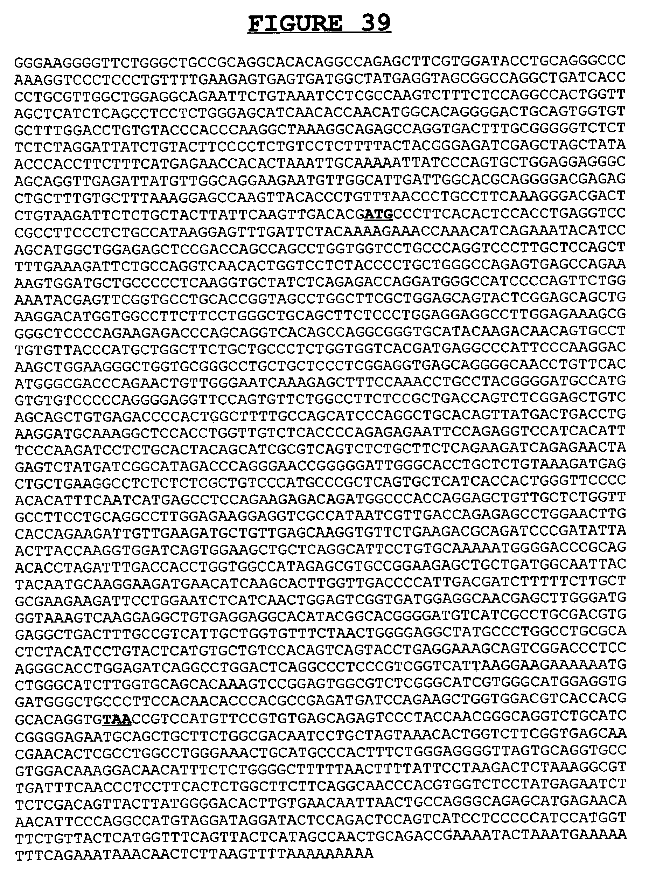

Figure 39 shows a nucleotide sequence (SEQ ID NO:39) of a native sequence PRO5000 cDNA, wherein SEQ ID NO:39 is a clone designated herein as "DNA59774-2665".

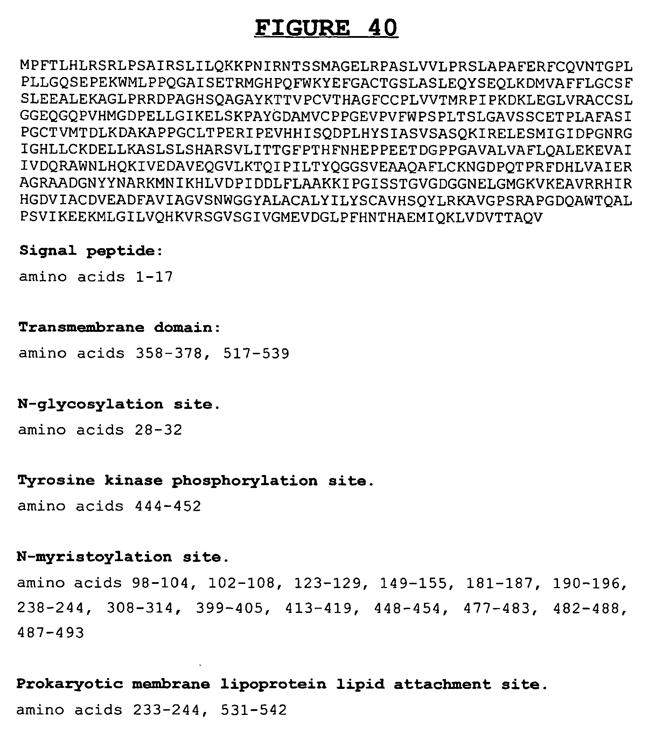

Figure 40 shows the amino acid sequence (SEQ ID NO:40) derived from the coding sequence of SEQ ID NO:39 shown in Figure 39.

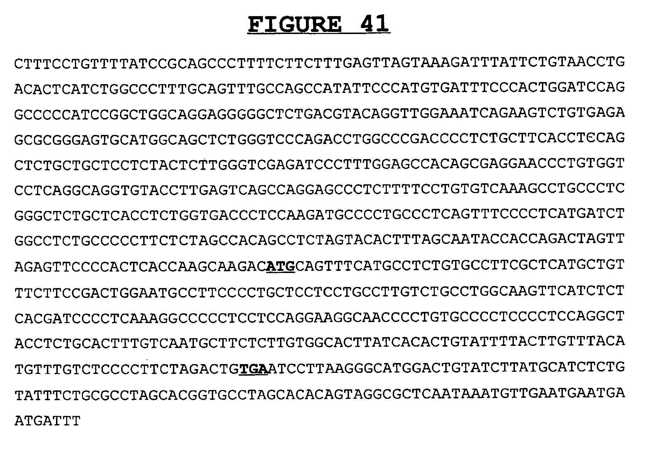

Figure 41 shows a nucleotide sequence (SEQ ID NO:41) of a native sequence PRO1881 cDNA, wherein SEQ ID NO:41 is a clone designated herein as "DNA60281-2518".

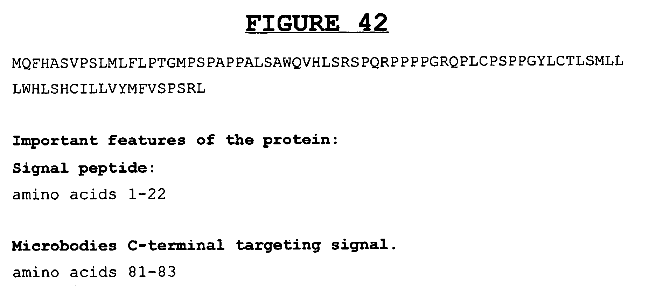

Figure 42 shows the amino acid sequence (SEQ ID NO:42) derived from the coding sequence of SEQ ID NO:41 shown in Figure 41.

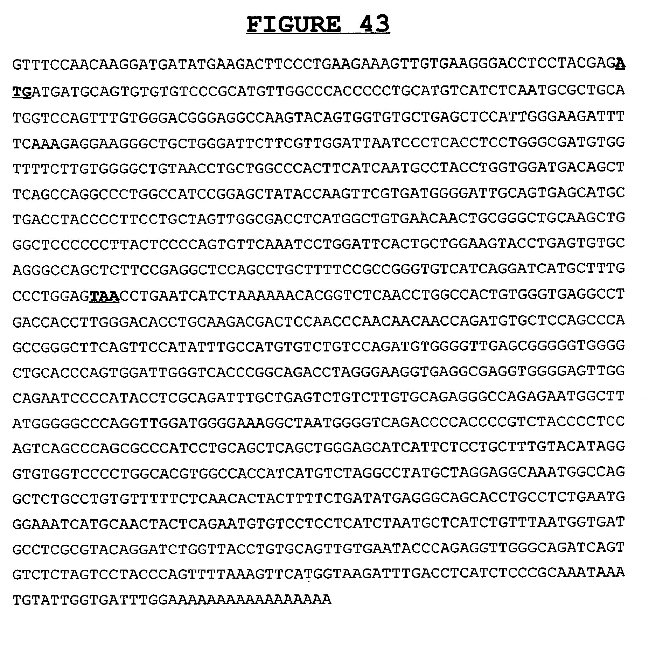

Figure 43 shows a nucleotide sequence (SEQ ID NO:43) of a native sequence PR04314 cDNA, wherein SEQ ID NO:43 is a clone designated herein as "DNA60736-2559".

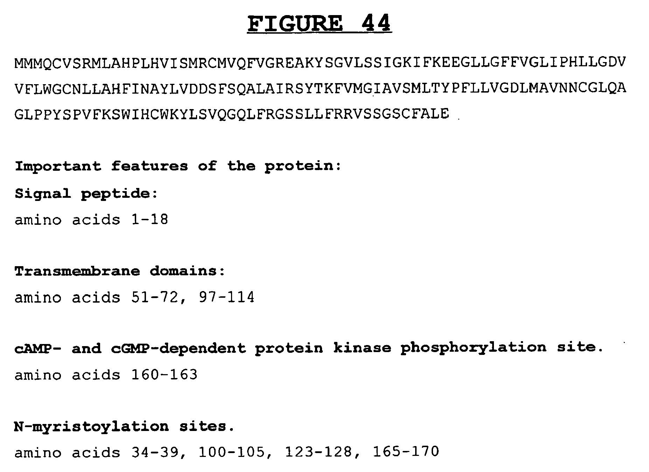

Figure 44 shows the amino acid sequence (SEQ ID NO:44) derived from the coding sequence of SEQ ID NO:43 shown in Figure 43.

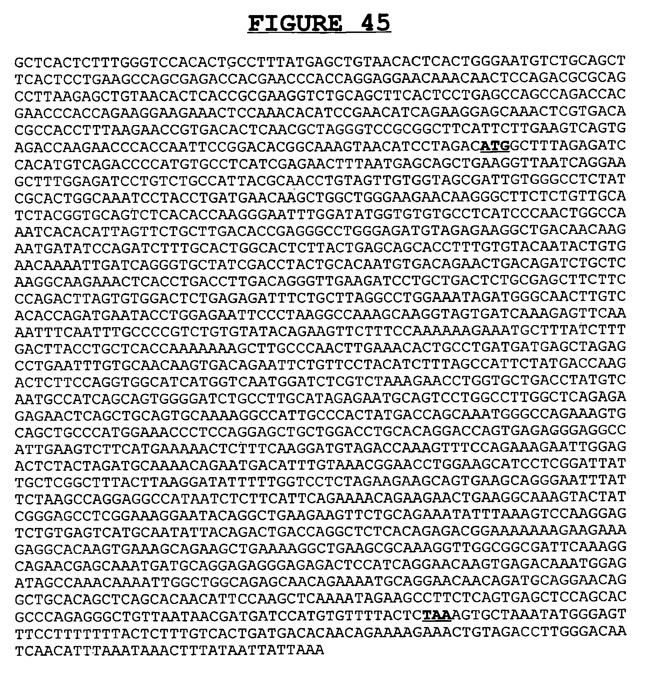

Figure 45 shows a nucleotide sequence (SEQ ID NO:45) of a native sequence PR04987 cDNA, wherein SEQ ID NO:45 is a clone designated herein as "DNA61875-2653".

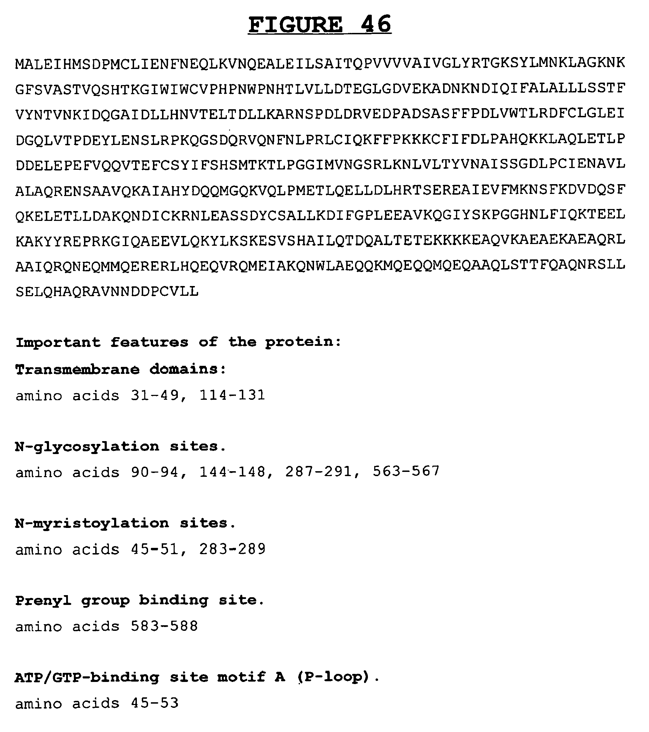

Figure 46 shows the amino acid sequence (SEQ ID NO:46) derived from the coding sequence of SEQ ID NO:45 shown in Figure 45.

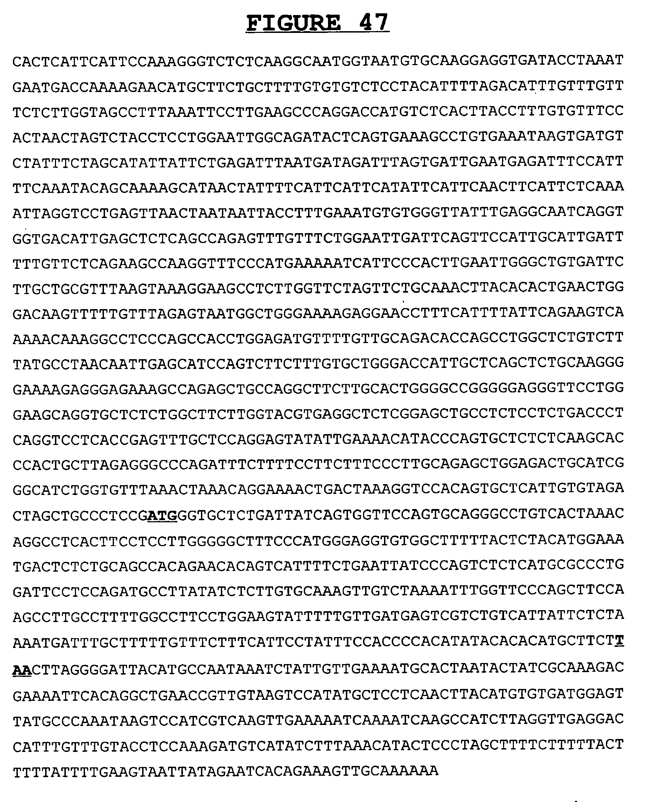

Figure 47 shows a nucleotide sequence (SEQ ID NO:47) of a native sequence PRO4313 cDNA, wherein SEQ ID NO:47 is a clone designated herein as "DNA62312-2558".

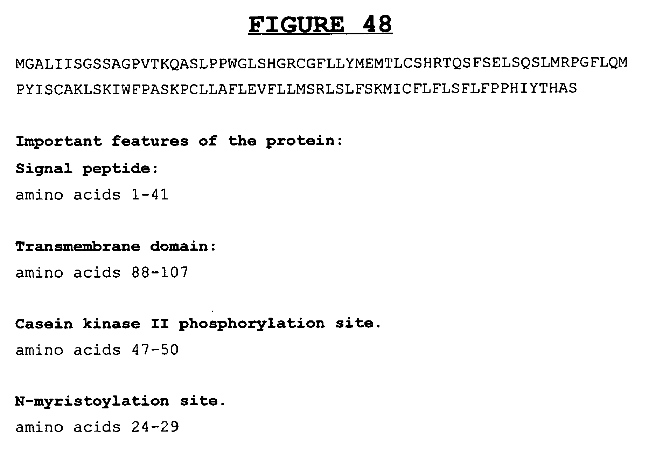

Figure 48 shows the amino acid sequence (SEQ ID NO:48) derived from the coding sequence of SEQ ID NO:47 shown in Figure 47.

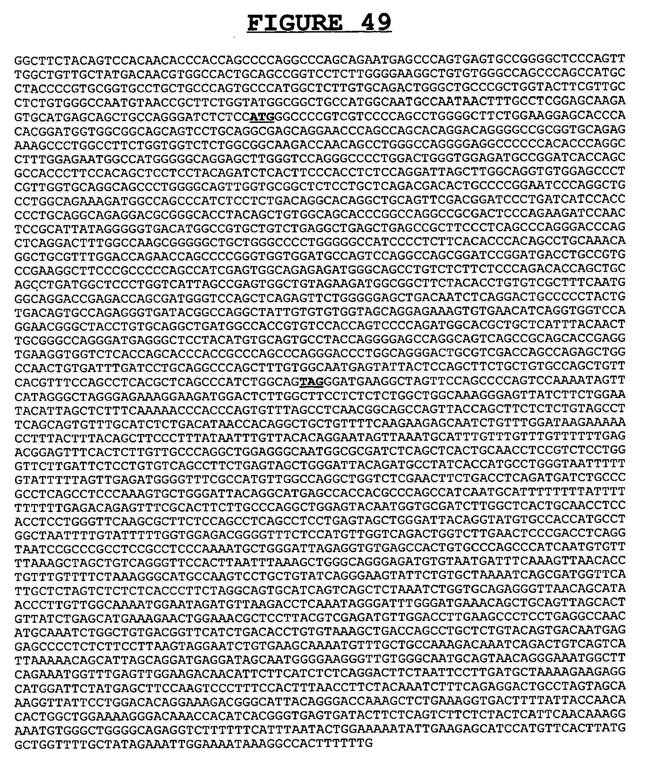

Figure 49 shows a nucleotide sequence (SEQ ID NO:49) of a native sequence PR04799 cDNA, wherein SEQ ID NO:49 is a clone designated herein as "DNA62849-1604".

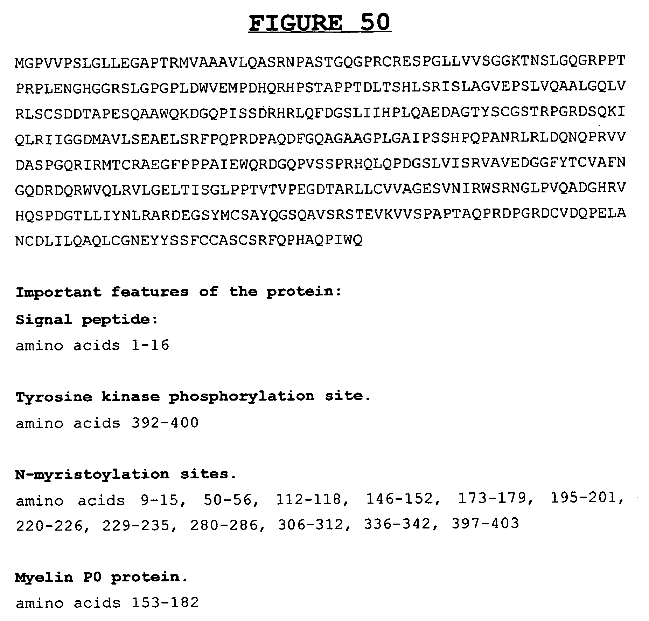

Figure 50 shows the amino acid sequence (SEQ ID NO:50) derived from the coding sequence of SEQ ID NO:49 shown in Figure 49.

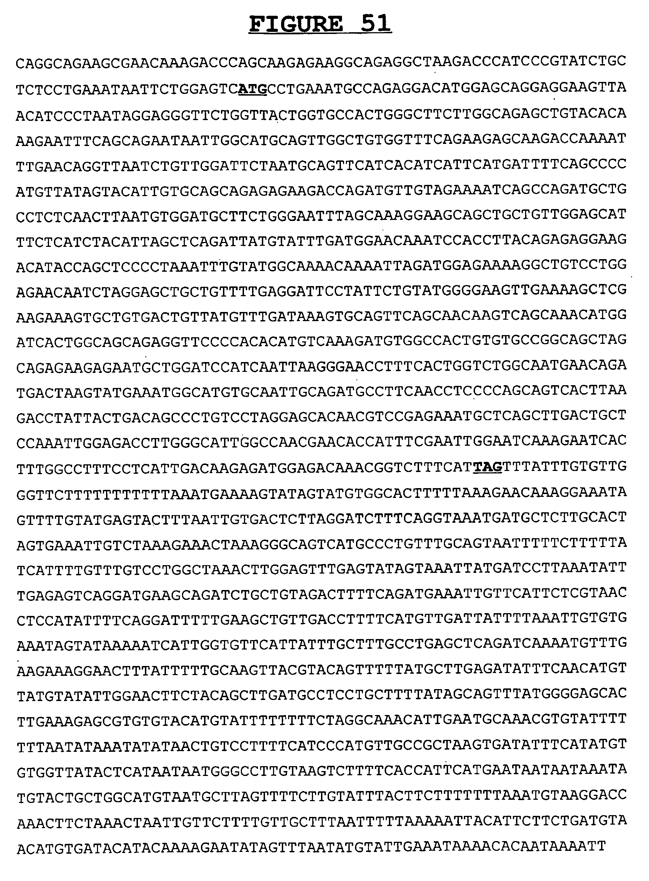

Figure 51 shows a nucleotide sequence (SEQ ID NO:51) of a native sequence PR04995 cDNA, wherein SEQ ID NO:51 is a clone designated herein as "DNA66307-2661".

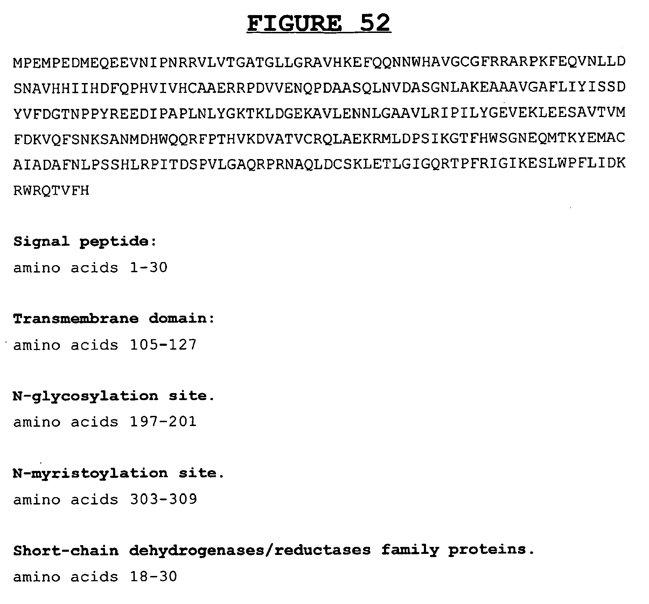

Figure 52 shows the amino acid sequence (SEQ ID NO:52) derived from the coding sequence of SEQ ID NO:51 shown in Figure 51.

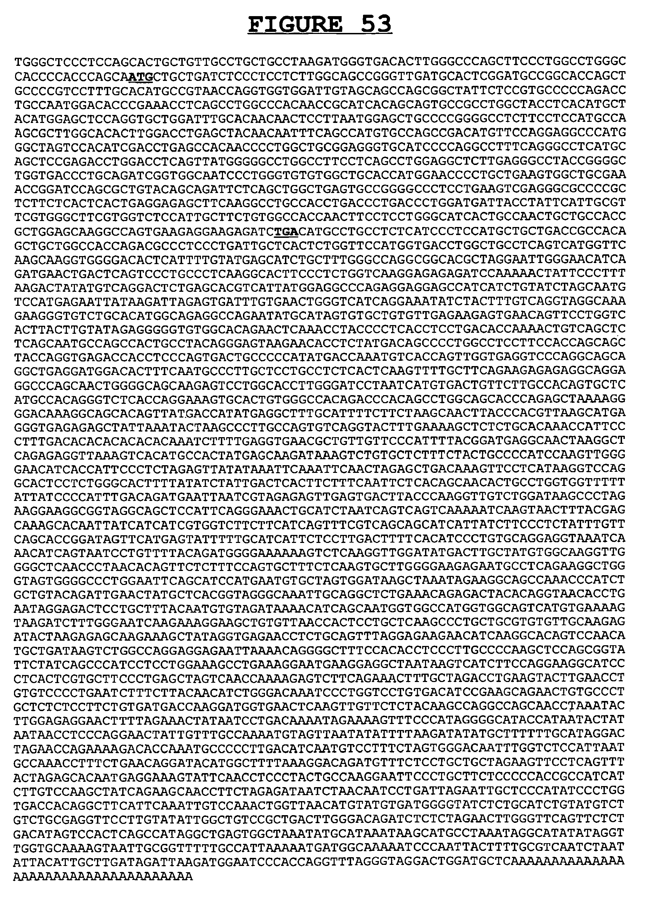

Figure 53 shows a nucleotide sequence (SEQ ID NO:53) of a native sequence PRO1341 cDNA, wherein SEQ ID NO:53 is a clone designated herein as "DNA66677-2535".

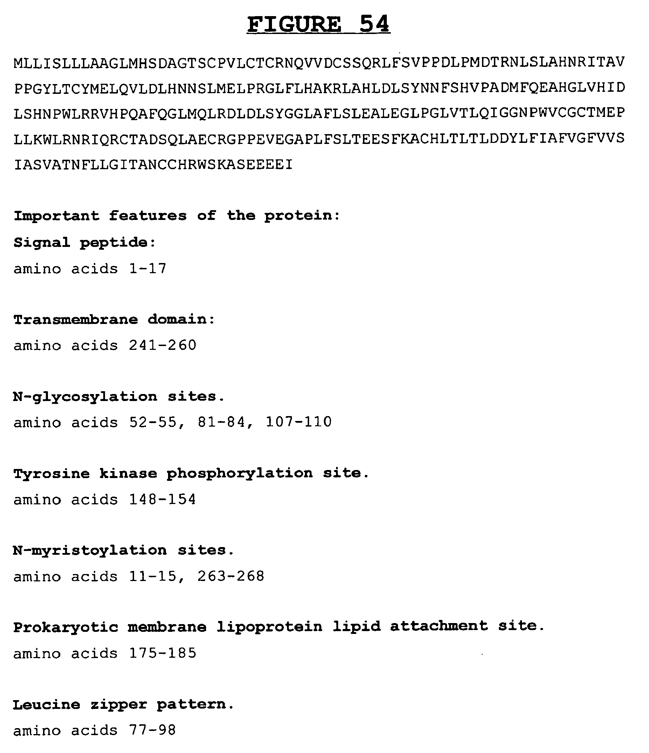

Figure 54 shows the amino acid sequence (SEQ ID NO:54) derived from the coding sequence of SEQ ID NO:53 shown in Figure 53.

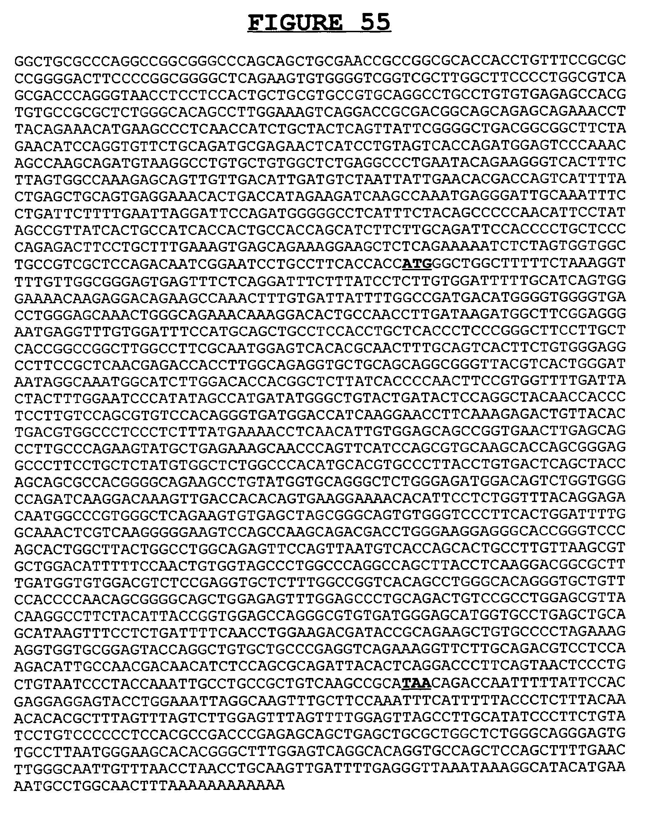

Figure 55 shows a nucleotide sequence (SEQ ID NO:55) of a native sequence PRO1777 cDNA, wherein SEQ ID NO:55 is a clone designated herein as "DNA71235-1706".

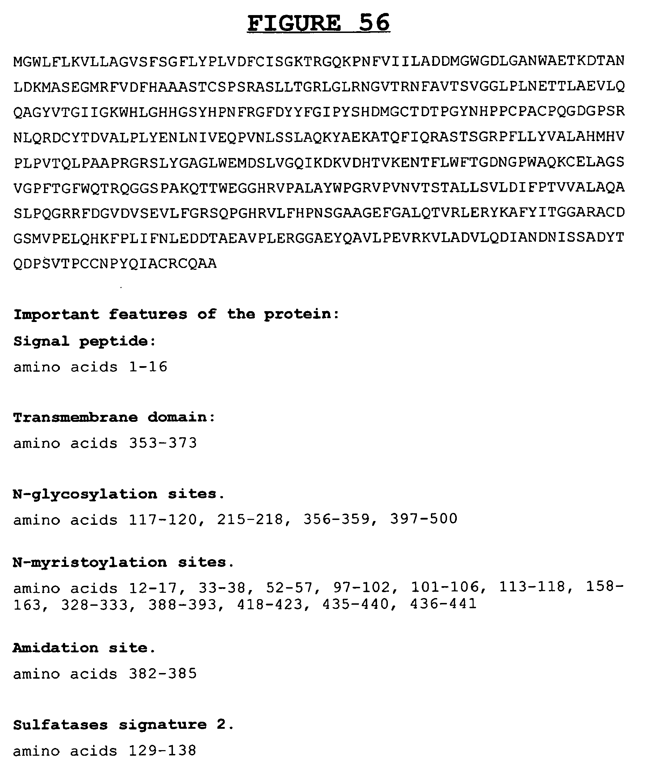

Figure 56 shows the amino acid sequence (SEQ ID NO:56) derived from the coding sequence of SEQ ID NO:55 shown in Figure 55.

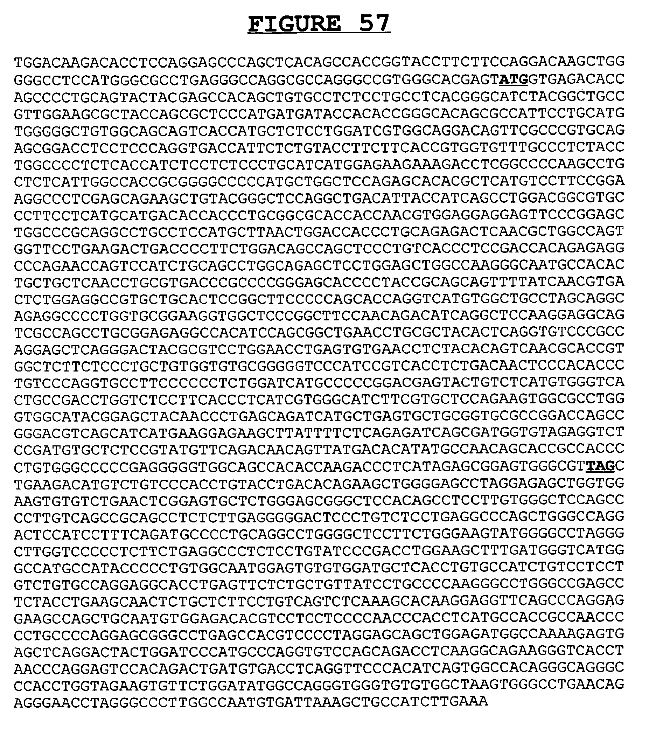

Figure 57 shows a nucleotide sequence (SEQ ID NO:57) of a native sequence PR03580 cDNA, wherein SEQ ID NO:57 is a clone designated herein as "DNA71289-2547".

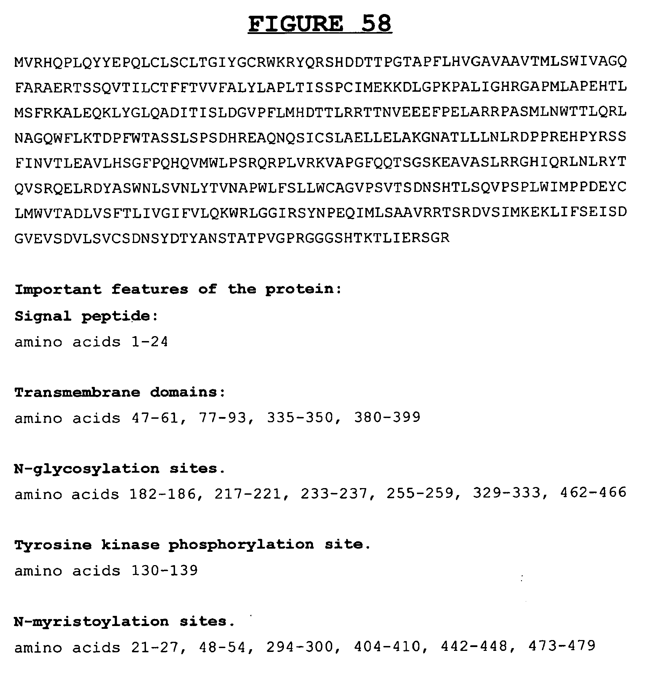

Figure 58 shows the amino acid sequence (SEQ ID NO:58) derived from the coding sequence of SEQ ID NO:57 shown in Figure 57.

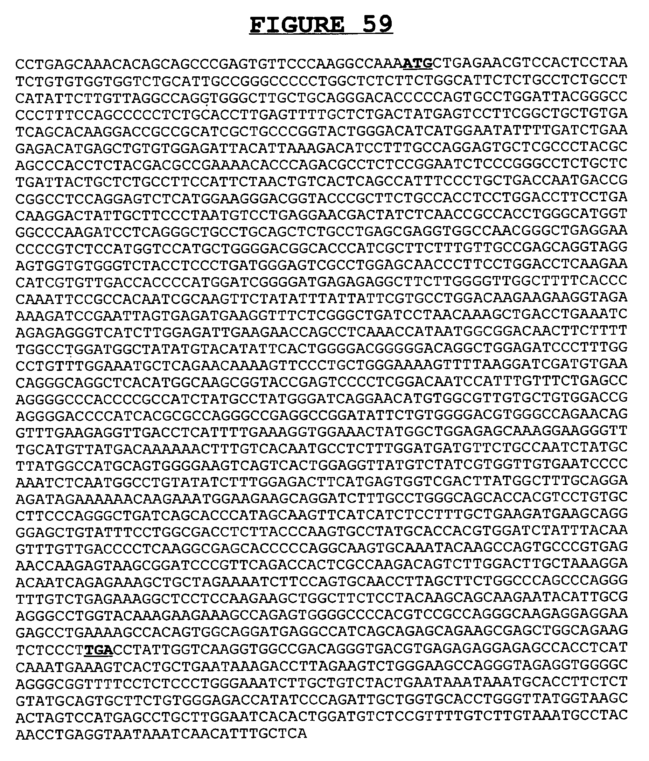

Figure 59 shows a nucleotide sequence (SEQ ID NO:59) of a native sequence PRO1779 cDNA, wherein SEQ ID NO:59 is a clone designated herein as "DNA73775-1707".

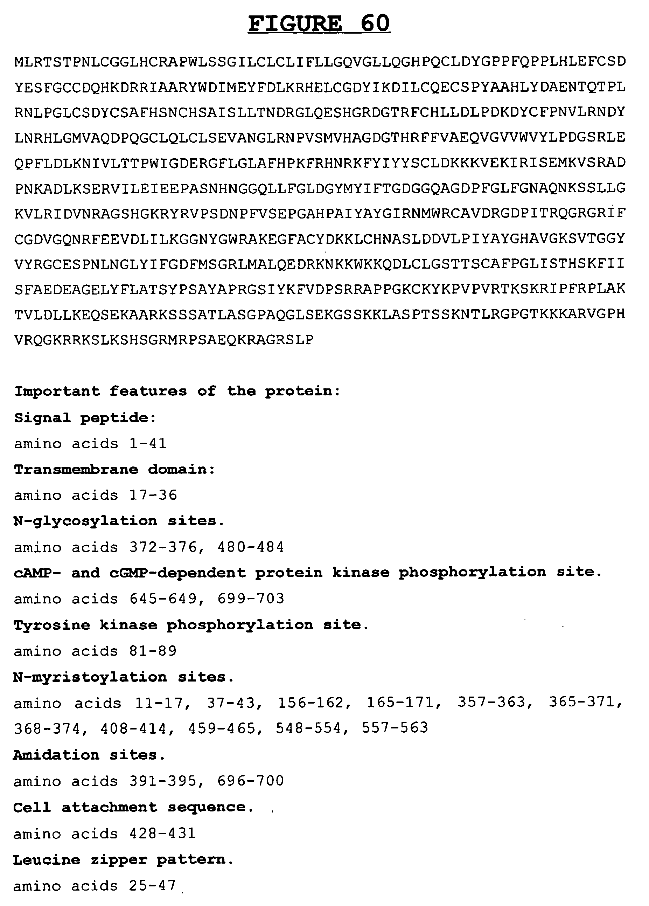

Figure 60 shows the amino acid sequence (SEQ ID NO:60) derived from the coding sequence of SEQ ID NO:59 shown in Figure 59.

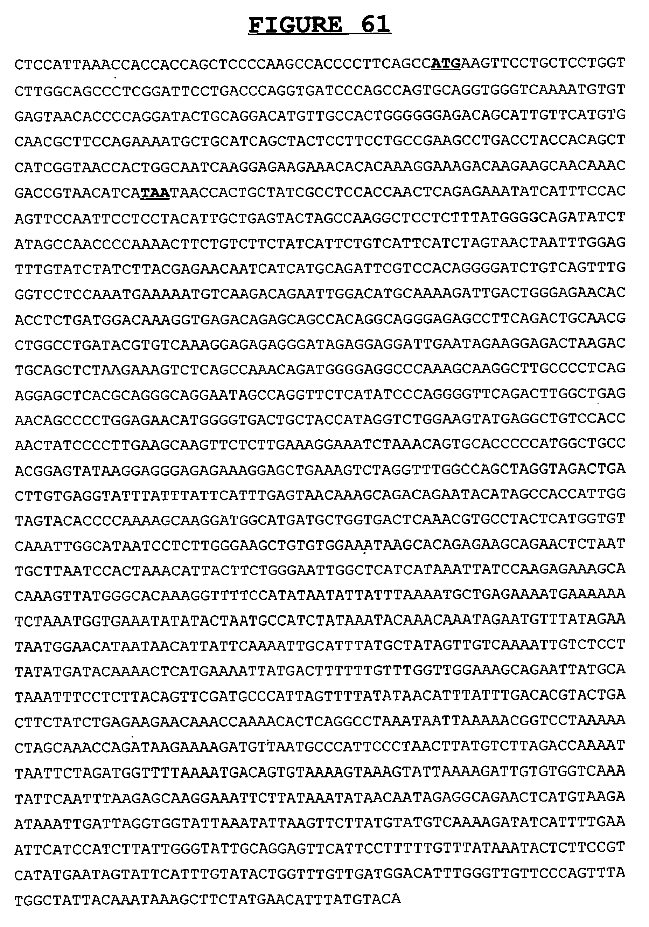

Figure 61 shows a nucleotide sequence (SEQ ID NO:61) of a native sequence PRO1754 cDNA, wherein SEQ ID NO:61 is a clone designated herein as "DNA76385-1692".

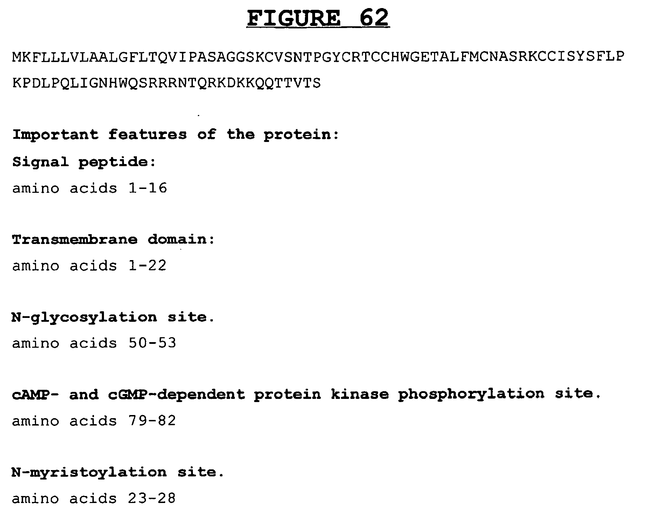

Figure 62 shows the amino acid sequence (SEQ ID NO:62) derived from the coding sequence of SEQ ID NO:61 shown in Figure 61.

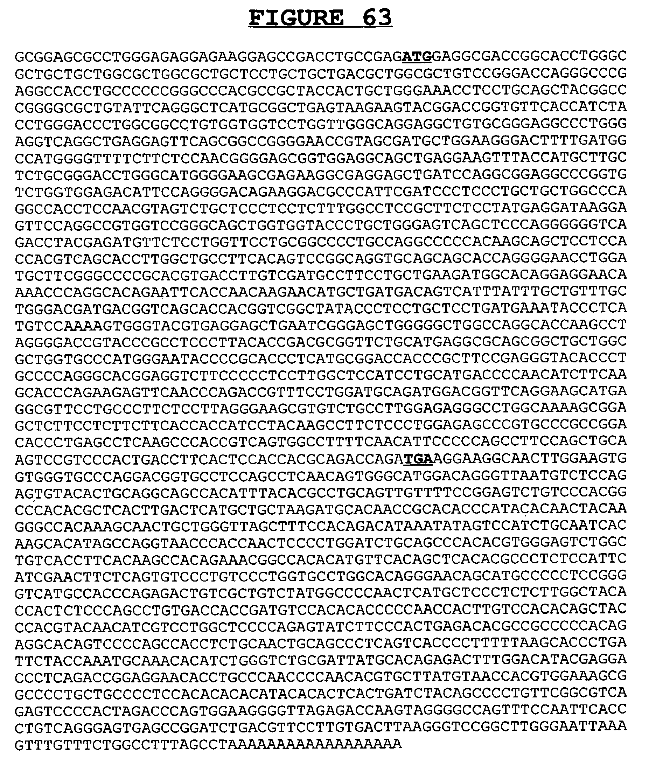

Figure 63 shows a nucleotide sequence (SEQ ID NO:63) of a native sequence PRO1906 cDNA, wherein SEQ ID NO:63 is a clone designated herein as "DNA76395-2527".

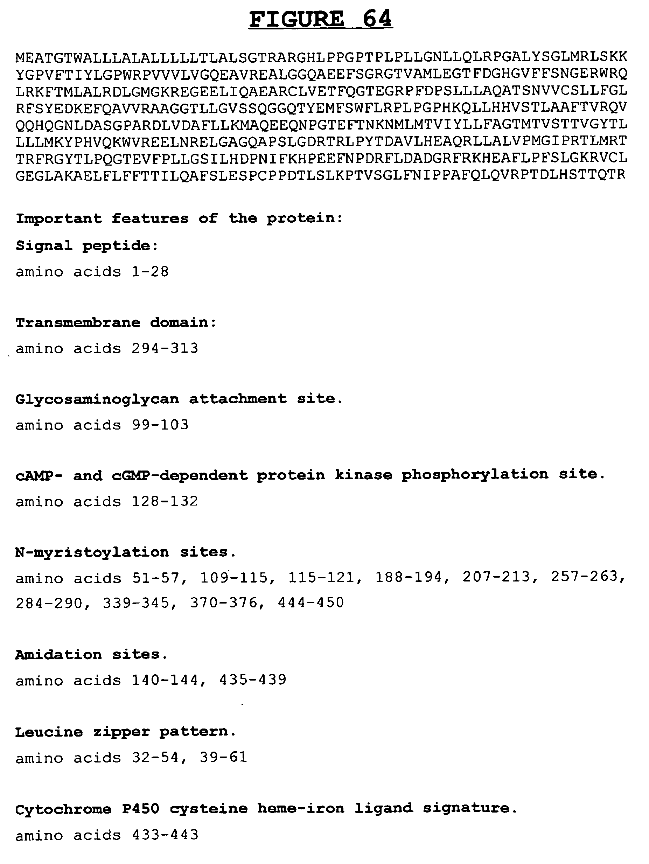

Figure 64 shows the amino acid sequence (SEQ ID NO:64) derived from the coding sequence of SEQ ID NO:63 shown in Figure 63.

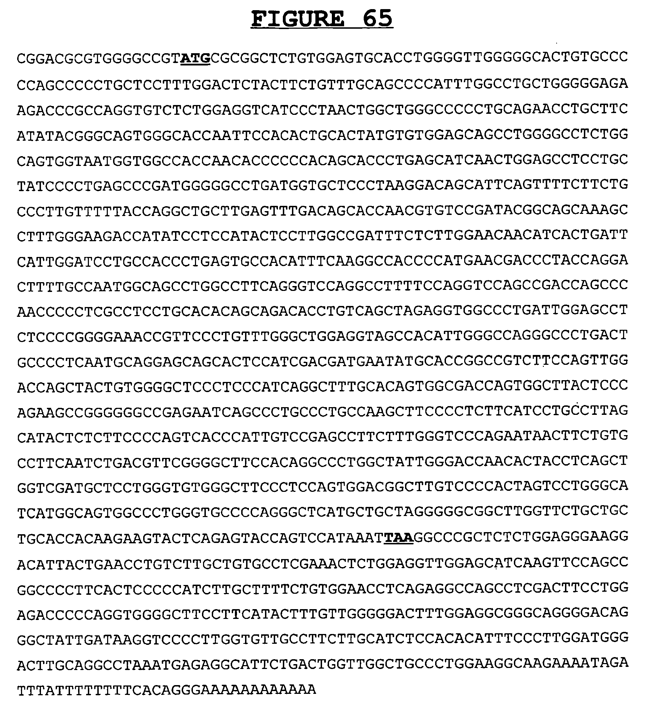

Figure 65 shows a nucleotide sequence (SEQ ID NO:65) of a native sequence PRO 1870 cDNA, wherein SEQ ID NO:65 is a clone designated herein as "DNA77622-2516".

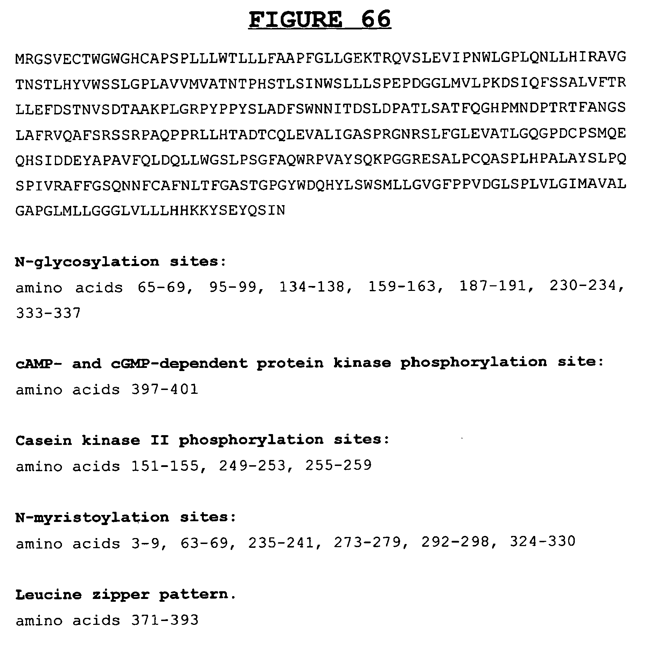

Figure 66 shows the amino acid sequence (SEQ ID NO:66) derived from the coding sequence of SEQ ID NO:65 shown in Figure 65.

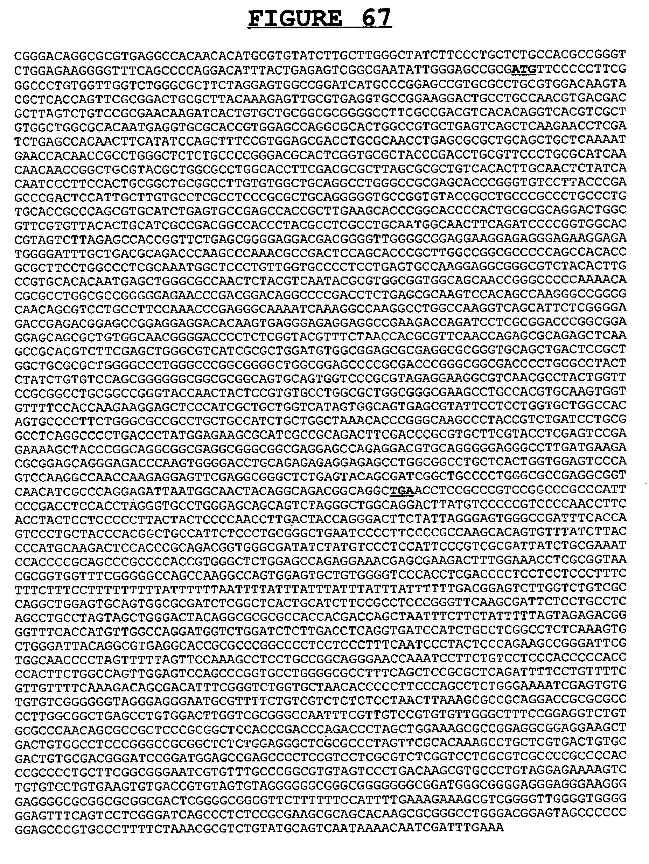

Figure 67 shows a nucleotide sequence (SEQ ID NO:67) of a native sequence PR04329 cDNA, wherein SEQ ID NO:67 is a clone designated herein as "DNA77629-2573".

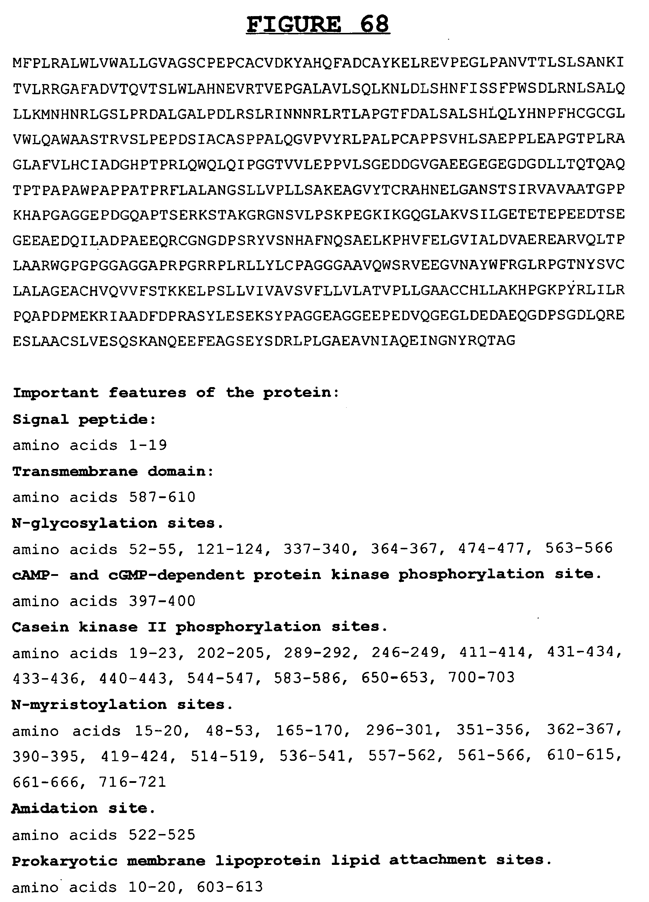

Figure 68 shows the amino acid sequence (SEQ ID NO:68) derived from the coding sequence of SEQ ID NO:67 shown in Figure 67.

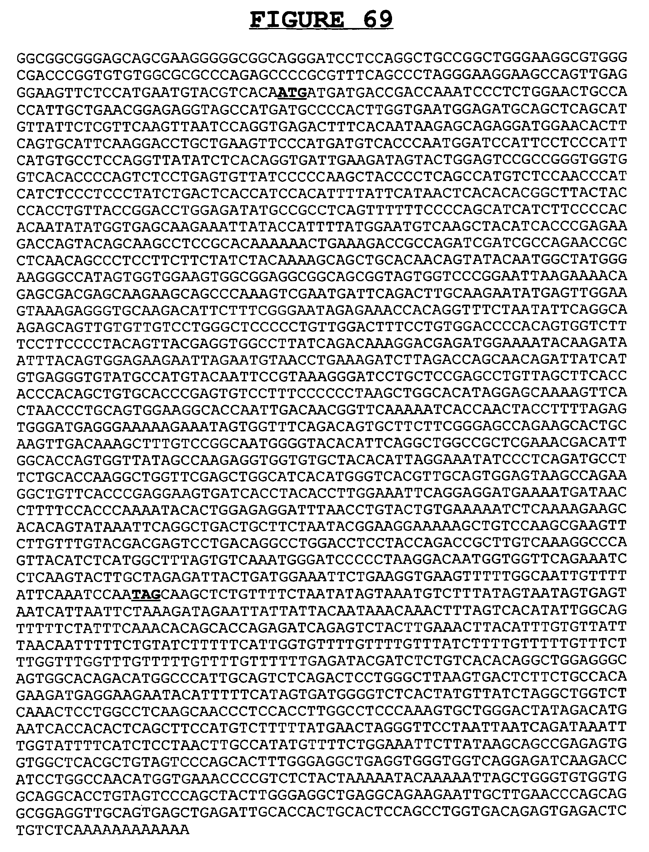

Figure 69 shows a nucleotide sequence (SEQ ID NO:69) of a native sequence PR04979 cDNA, wherein SEQ ID NO:69 is a clone designated herein as "DNA77645-2648".

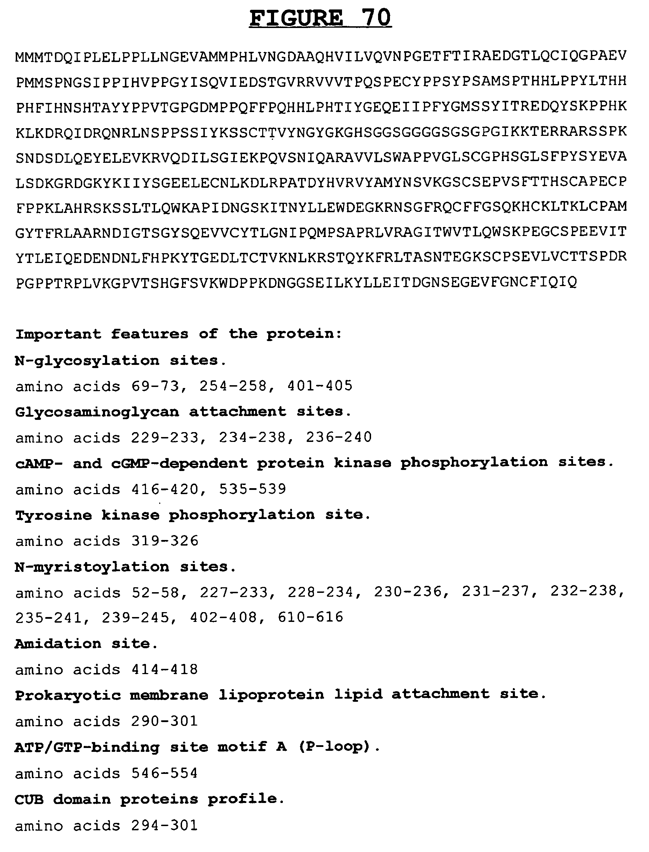

Figure 70 shows the amino acid sequence (SEQ ID NO:70) derived from the coding sequence of SEQ ID NO:69 shown in Figure 69.

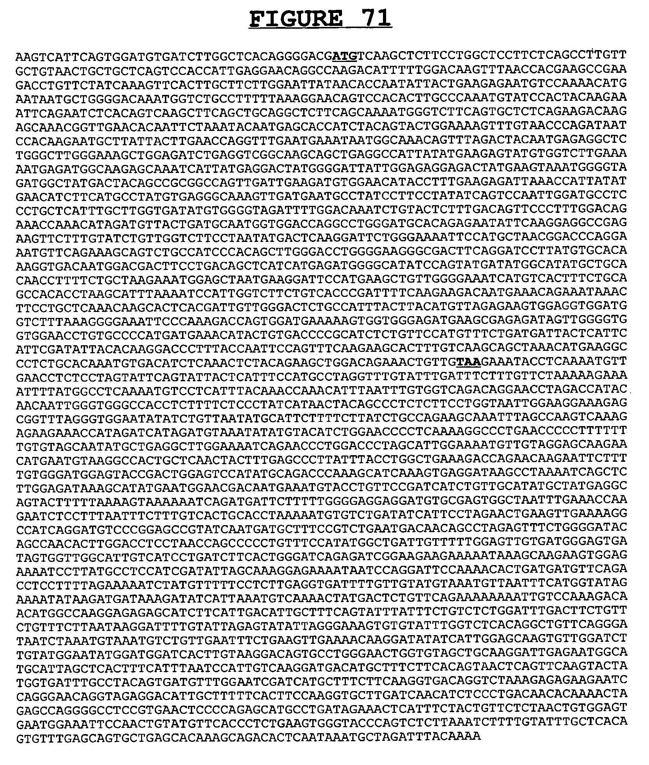

Figure 71 shows a nucleotide sequence (SEQ ID NO:71) of a native sequence PRO1885 cDNA, wherein SEQ ID NO:71 is a clone designated herein as "DNA79302-2521".

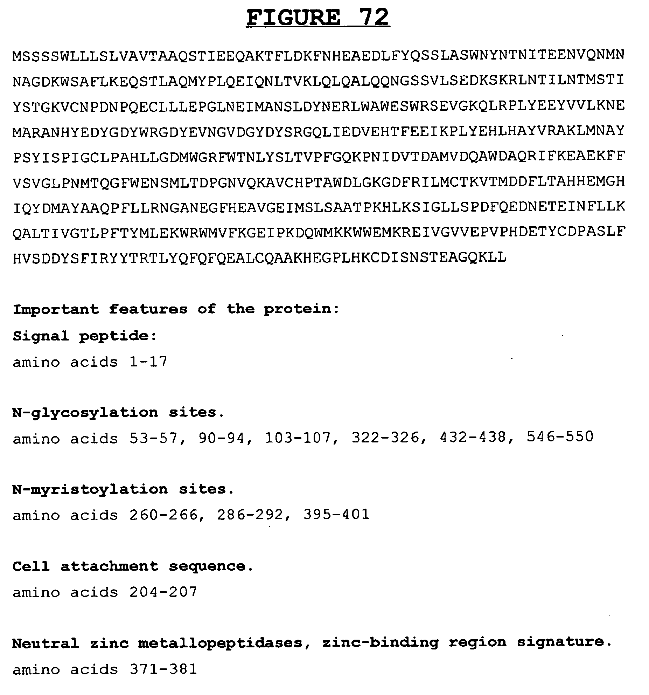

Figure 72 shows the amino acid sequence (SEQ ID NO:72) derived from the coding sequence of SEQ ID NO:71 shown in Figure 71.

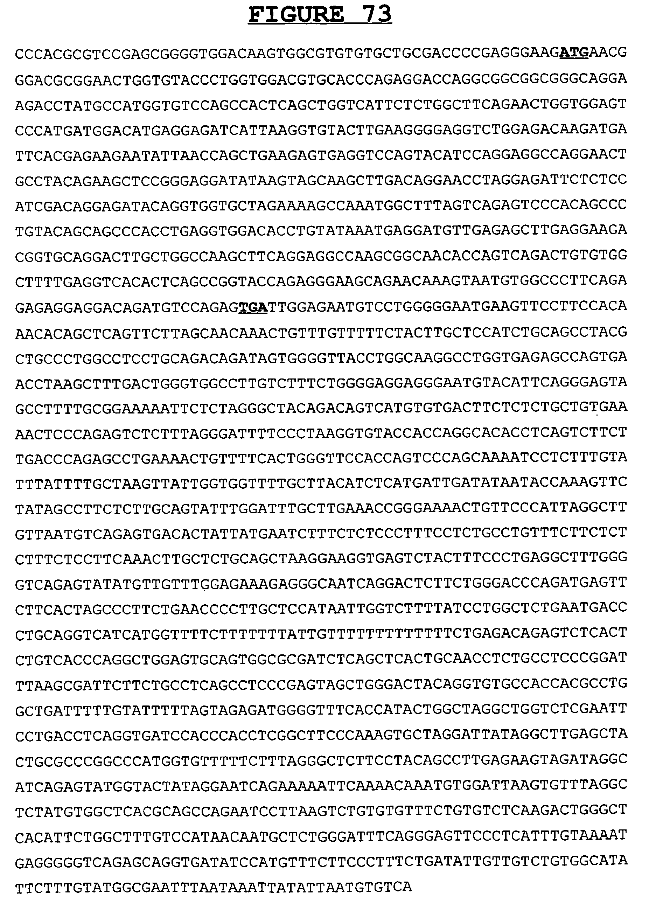

Figure 73 shows a nucleotide sequence (SEQ ID NO:73) of a native sequence PRO1882 cDNA, wherein SEQ ID NO:73 is a clone designated herein as "DNA79865-2519".

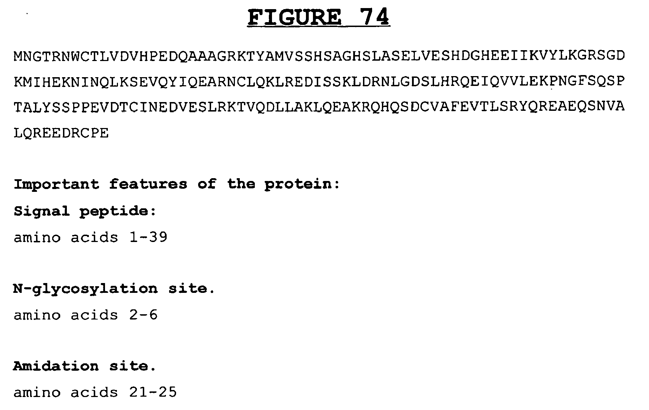

Figure 74 shows the amino acid sequence (SEQ ID NO:74) derived from the coding sequence of SEQ ID NO:73 shown in Figure 73.

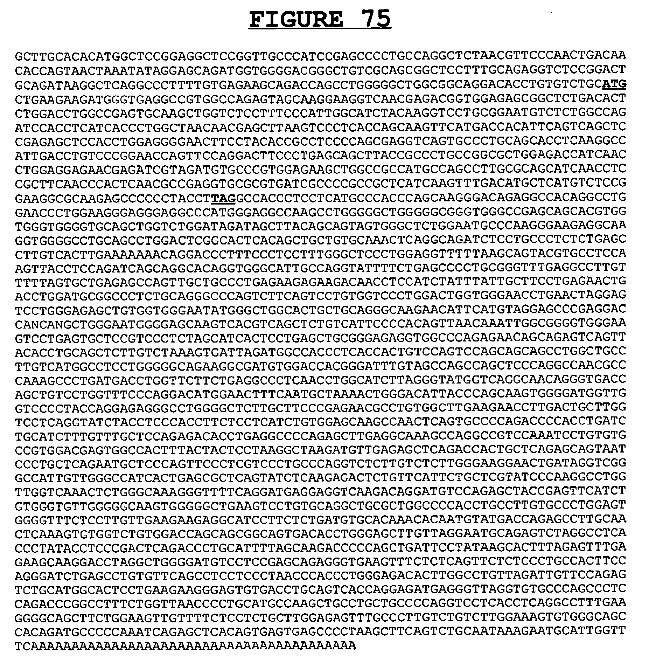

Figure 75 shows a nucleotide sequence (SEQ ID NO:75) of a native sequence PR04989 cDNA, wherein SEQ ID NO:75 is a clone designated herein as "DNA80135-2655".

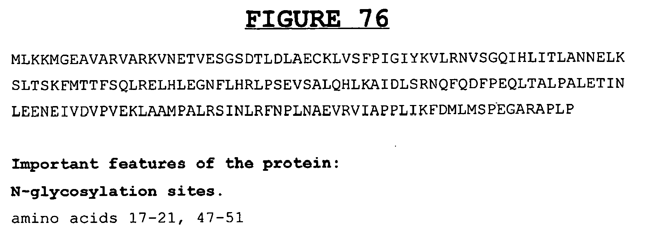

Figure 76 shows the amino acid sequence (SEQ ID NO:76) derived from the coding sequence of SEQ ID NO:75 shown in Figure 75.

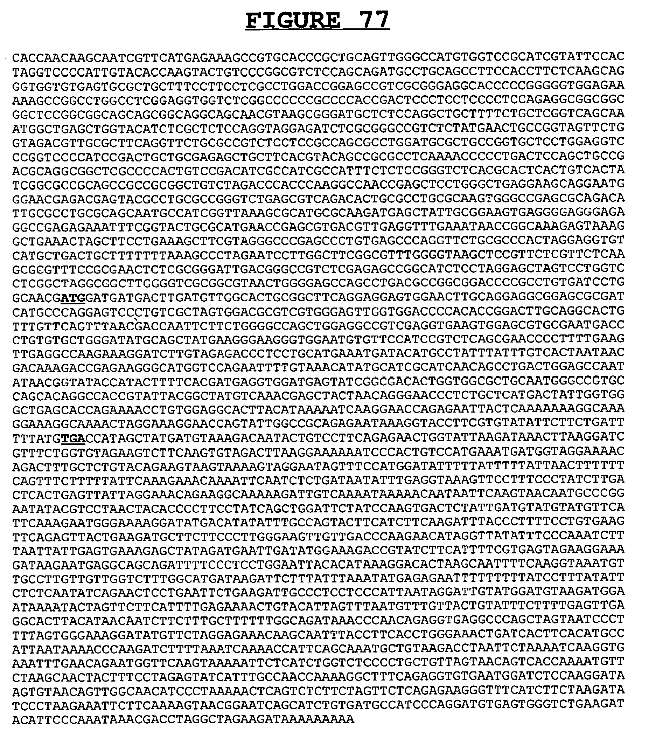

Figure 77 shows a nucleotide sequence (SEQ ID NO:77) of a native sequence PR04323 cDNA, wherein SEQ ID NO:77 is a clone designated herein as "DNA80794-2568".

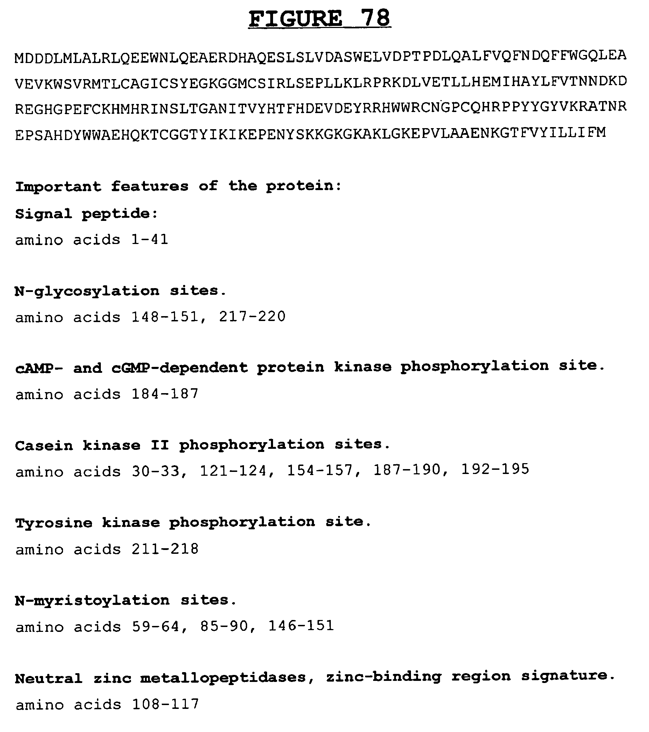

Figure 78 shows the amino acid sequence (SEQ ID NO:78) derived from the coding sequence of SEQ ID NO:77 shown in Figure 77.

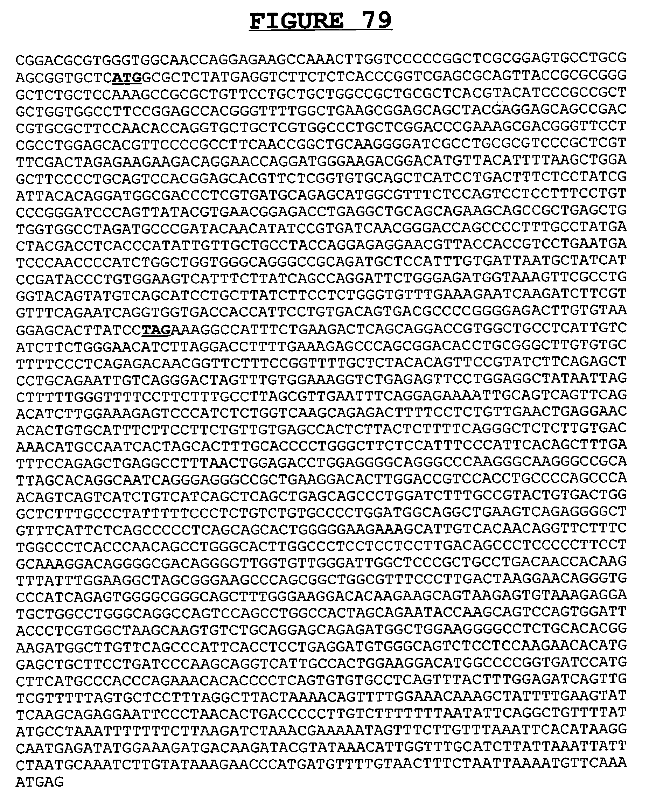

Figure 79 shows a nucleotide sequence (SEQ ID NO:79) of a native sequence PRO1886 cDNA, wherein SEQ ID NO:79 is a clone designated herein as "DNA80796-2523".

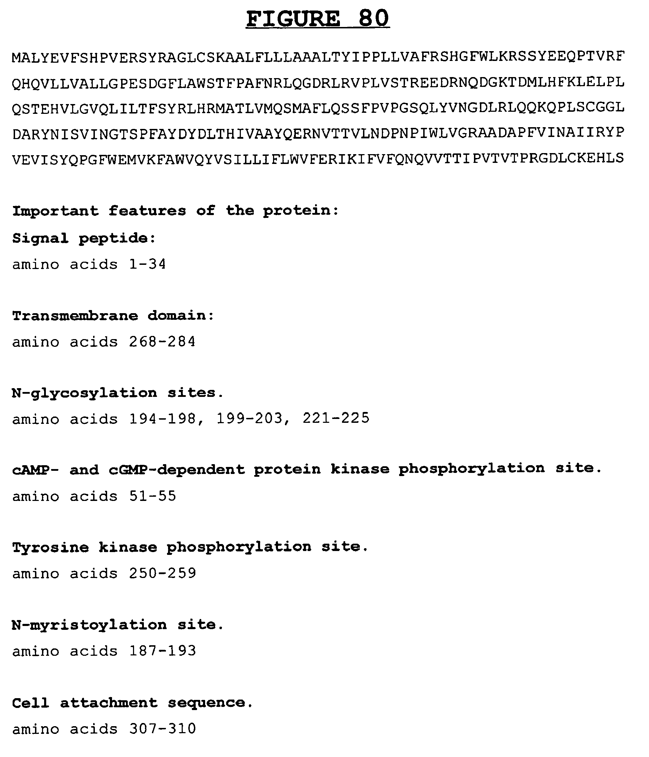

Figure 80 shows the amino acid sequence (SEQ ID NO:80) derived from the coding sequence of SEQ ID NO:79 shown in Figure 79.

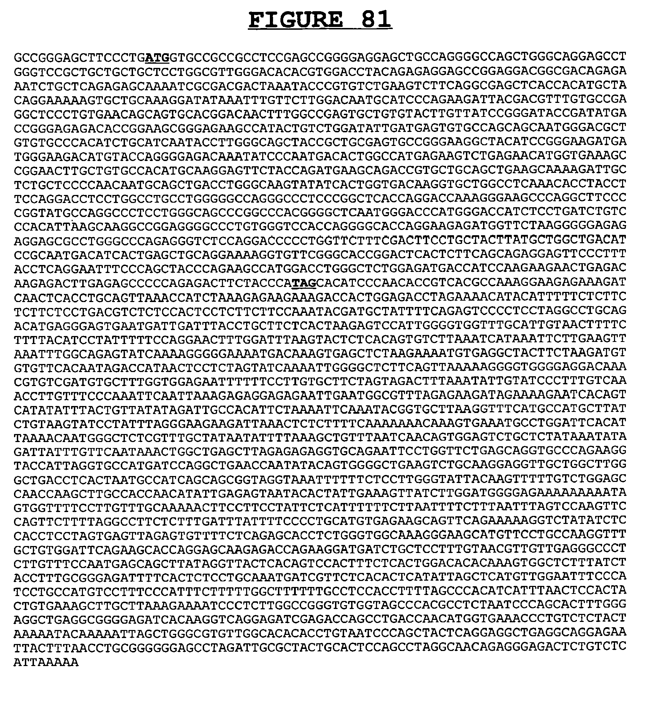

Figure 81 shows a nucleotide sequence (SEQ ID NO:81) of a native sequence PR04395 cDNA, wherein SEQ ID NO:81 is a clone designated herein as "DNA80840-2605".

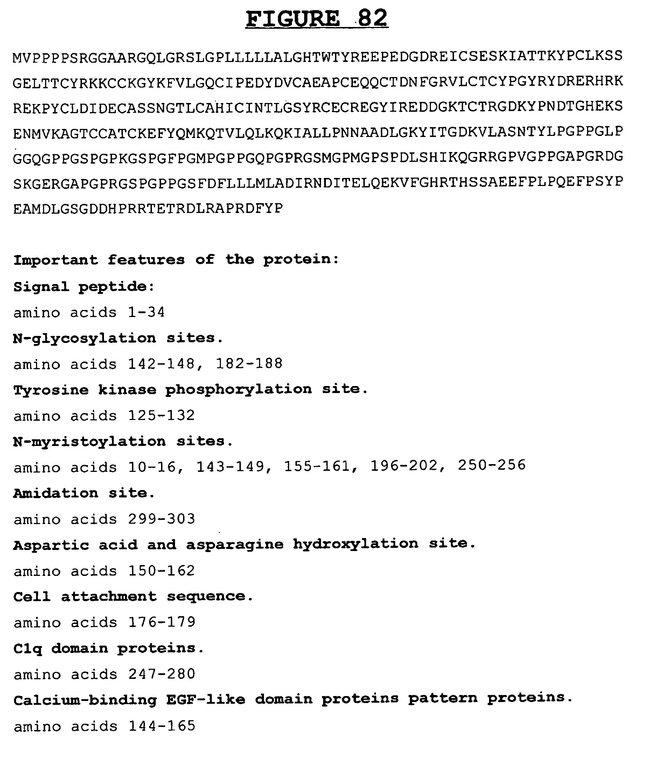

Figure 82 shows the amino acid sequence (SEQ ID NO:82) derived from the coding sequence of SEQ ID NO:81 shown in Figure 81.

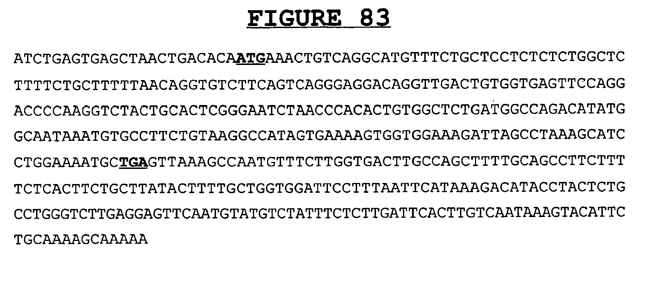

Figure 83 shows a nucleotide sequence (SEQ ID NO:83) of a native sequence PRO1782 cDNA, wherein SEQ ID NO:83 is a clone designated herein as "DNA80899-2501".

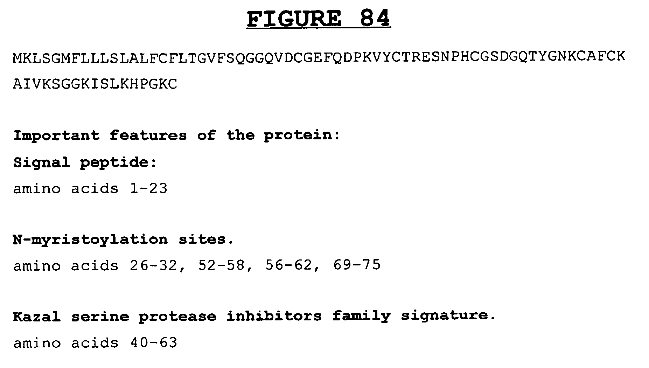

Figure 84 shows the amino acid sequence (SEQ ID NO:84) derived from the coding sequence of SEQ ID NO:83 shown in Figure 83.

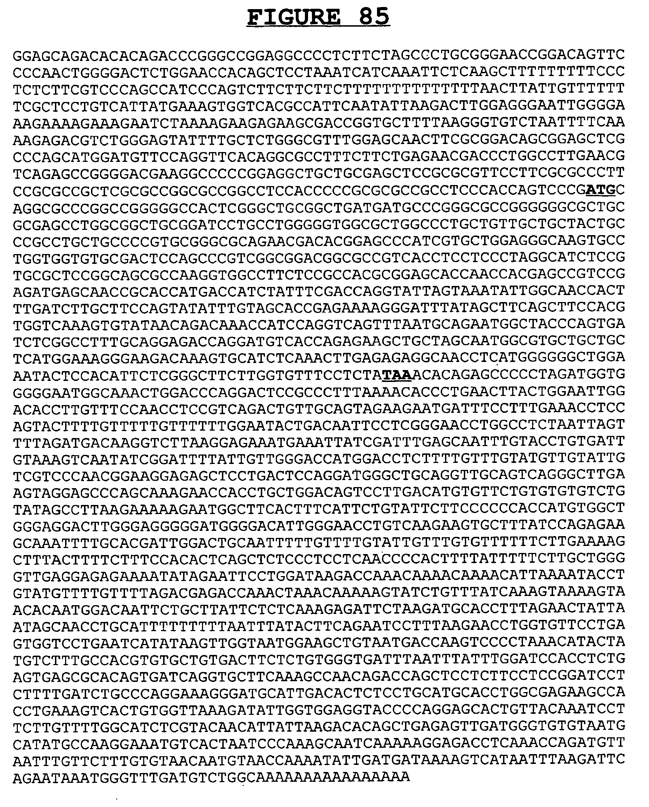

Figure 85 shows a nucleotide sequence (SEQ ID NO:85) of a native sequence PR04338 cDNA, wherein SEQ ID NO:85 is a clone designated herein as "DNA81228-2580".

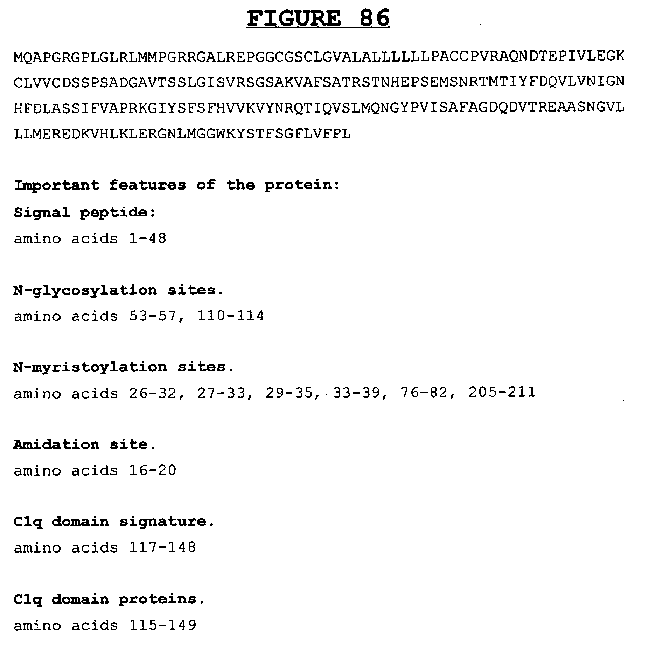

Figure 86 shows the amino acid sequence (SEQ ID NO:86) derived from the coding sequence of SEQ ID NO:85 shown in Figure 85.

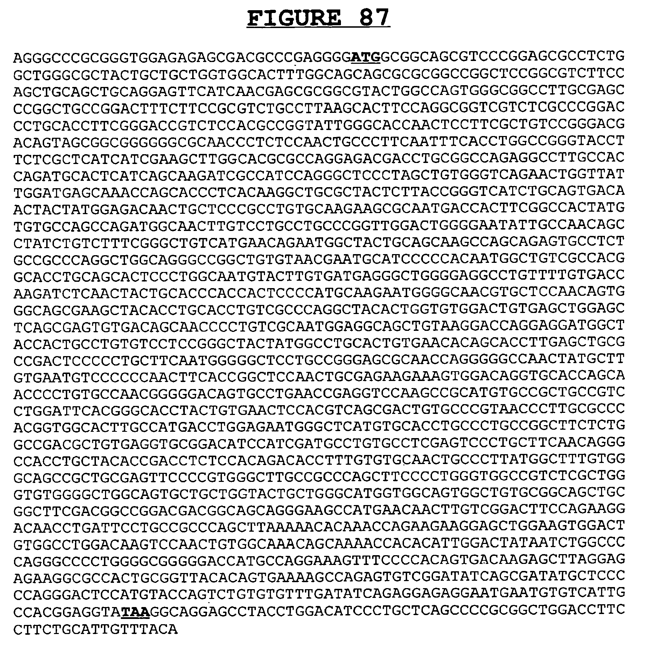

Figure 87 shows a nucleotide sequence (SEQ ID NO:87) of a native sequence PR04341 cDNA, wherein SEQ ID NO:87 is a clone designated herein as "DNA81761-2583".

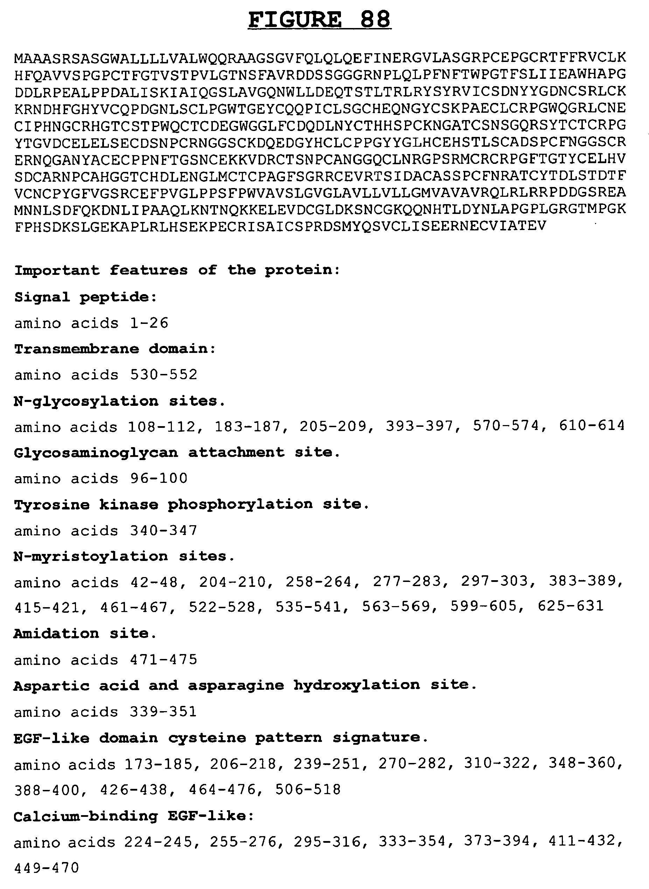

Figure 88 shows the amino acid sequence (SEQ ID NO:88) derived from the coding sequence of SEQ ID NO:87 shown in Figure 87.

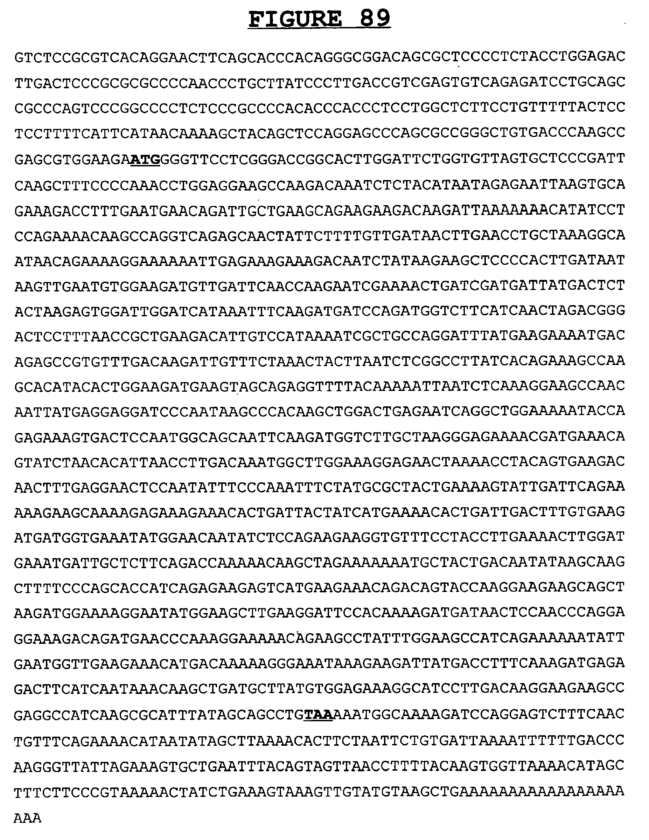

Figure 89 shows a nucleotide sequence (SEQ ID NO:89) of a native sequence PR05990 cDNA, wherein SEQ ID NO:89 is a clone designated herein as "DNA96042-2682".

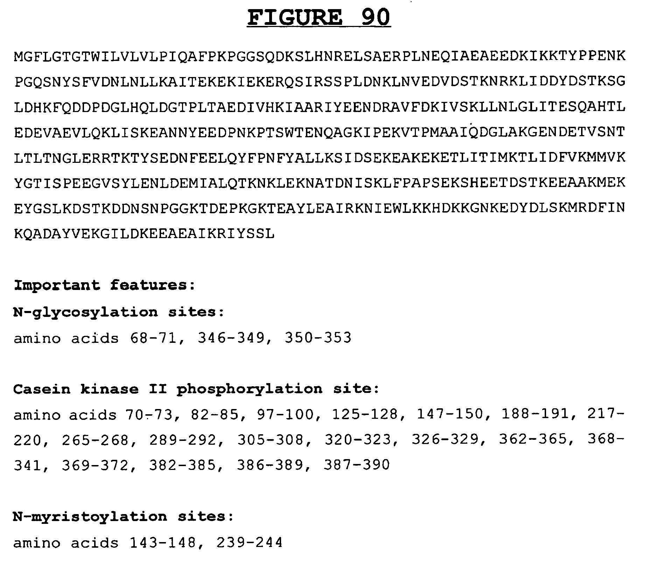

Figure 90 shows the amino acid sequence (SEQ ID NO:90) derived from the coding sequence of SEQ ID NO:89 shown in Figure 89.

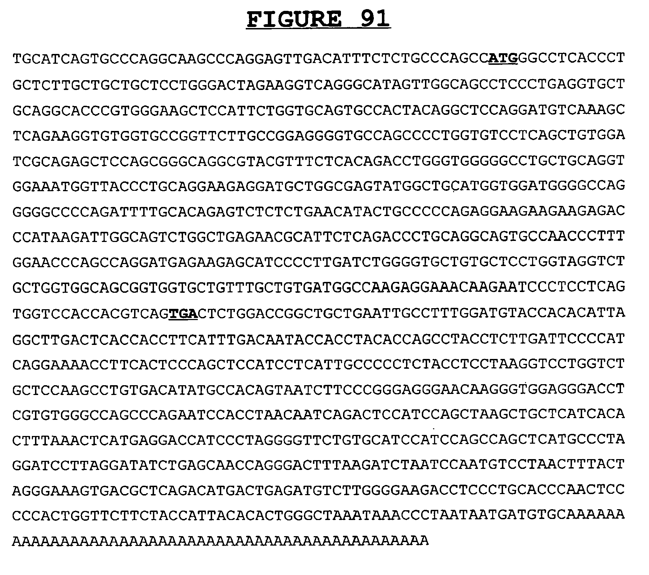

Figure 91 shows a nucleotide sequence (SEQ ID NO:91) of a native sequence PR03438 cDNA, wherein SEQ ID NO:91 is a clone designated herein as "DNA82364-2538".

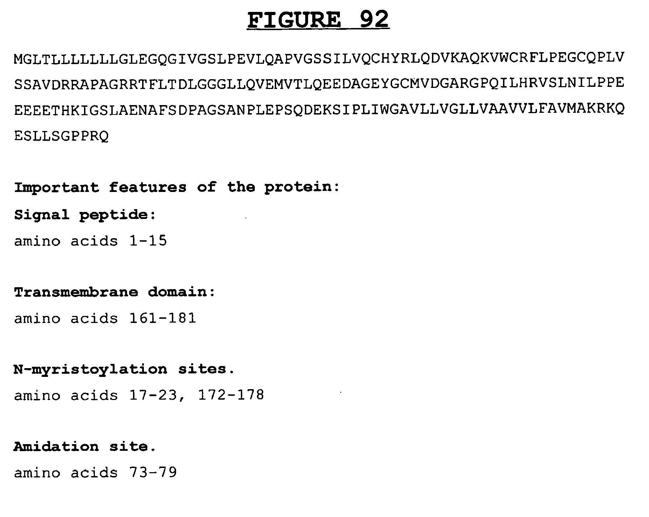

Figure 92 shows the amino acid sequence (SEQ ID NO:92) derived from the coding sequence of SEQ ID NO:91 shown in Figure 91.

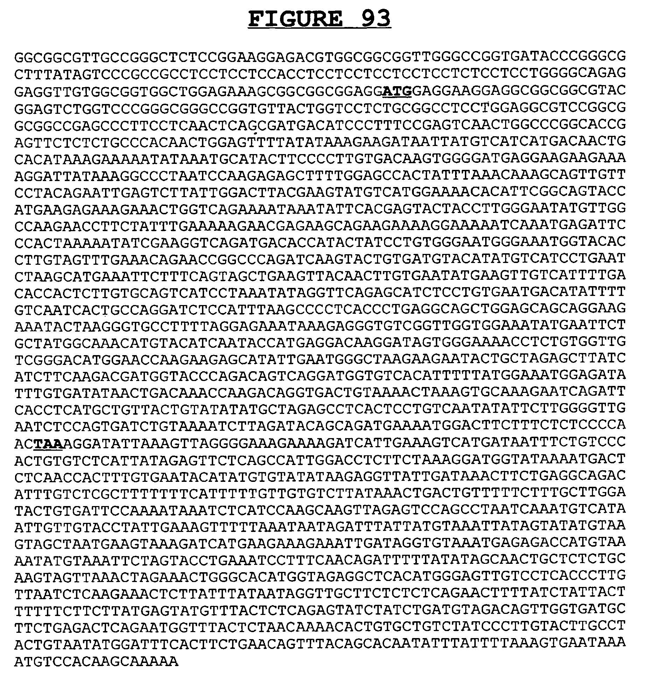

Figure 93 shows a nucleotide sequence (SEQ ID NO:93) of a native sequence PR04321 cDNA, wherein SEQ ID NO:93 is a clone designated herein as "DNA82424-2566".

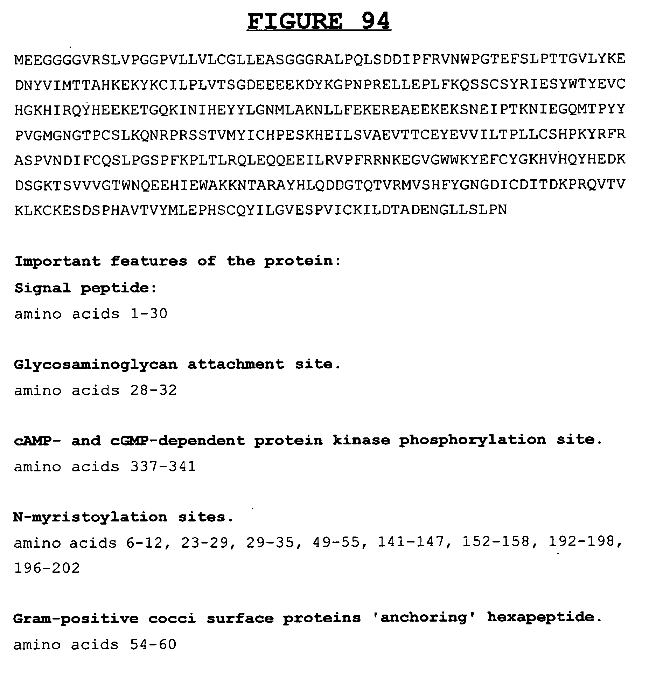

Figure 94 shows the amino acid sequence (SEQ ID NO:94) derived from the coding sequence of SEQ ID NO:93 shown in Figure 93.

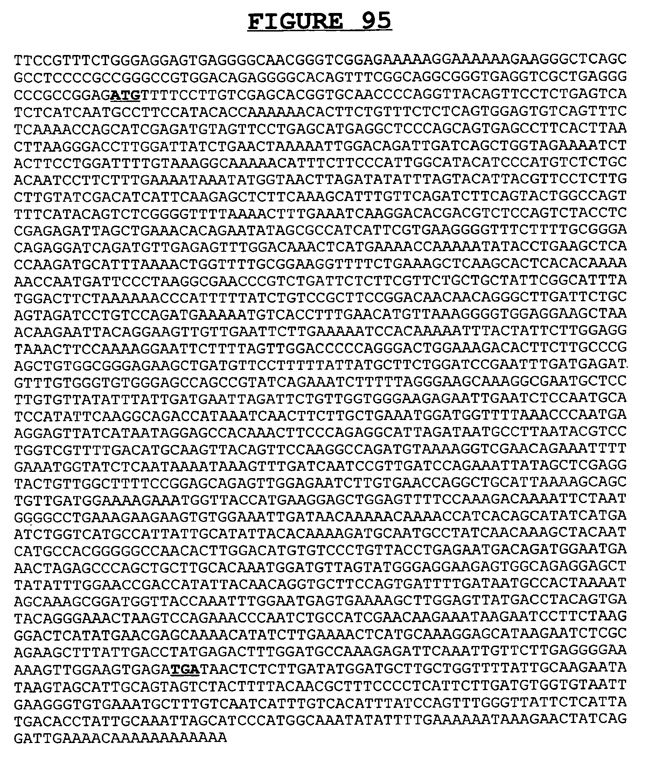

Figure 95 shows a nucleotide sequence (SEQ ID NO:95) of a native sequence PR04304 cDNA, wherein SEQ ID NO:95 is a clone designated herein as "DNA82430-2557".

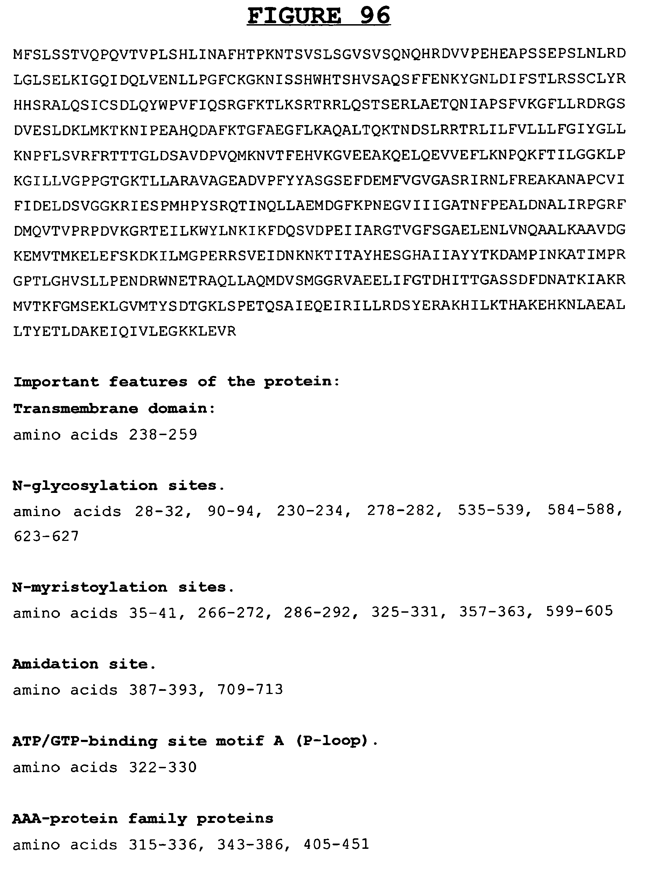

Figure 96 shows the amino acid sequence (SEQ ID NO:96) derived from the coding sequence of SEQ ID NO:95 shown in Figure 95.

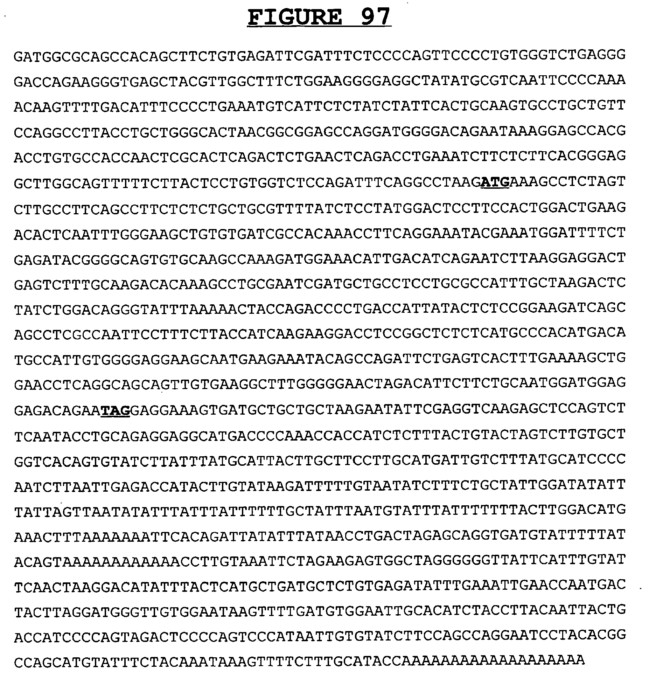

Figure 97 shows a nucleotide sequence (SEQ ID NO:97) of a native sequence PRO1801 cDNA, wherein SEQ ID NO:97 is a clone designated herein as "DNA83500-2506".

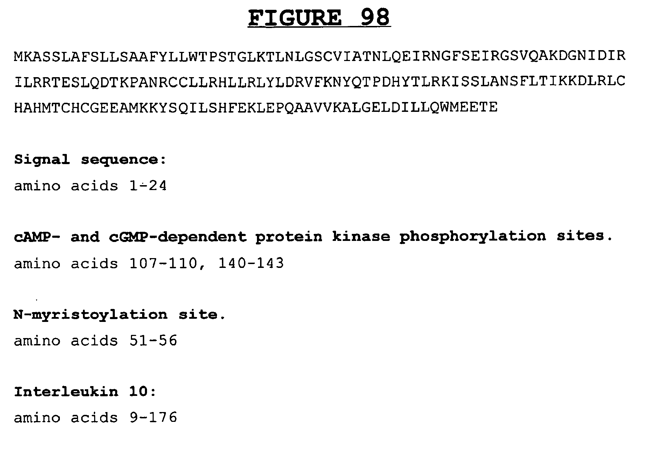

Figure 98 shows the amino acid sequence (SEQ ID NO:98) derived from the coding sequence of SEQ ID NO:97 shown in Figure 97.

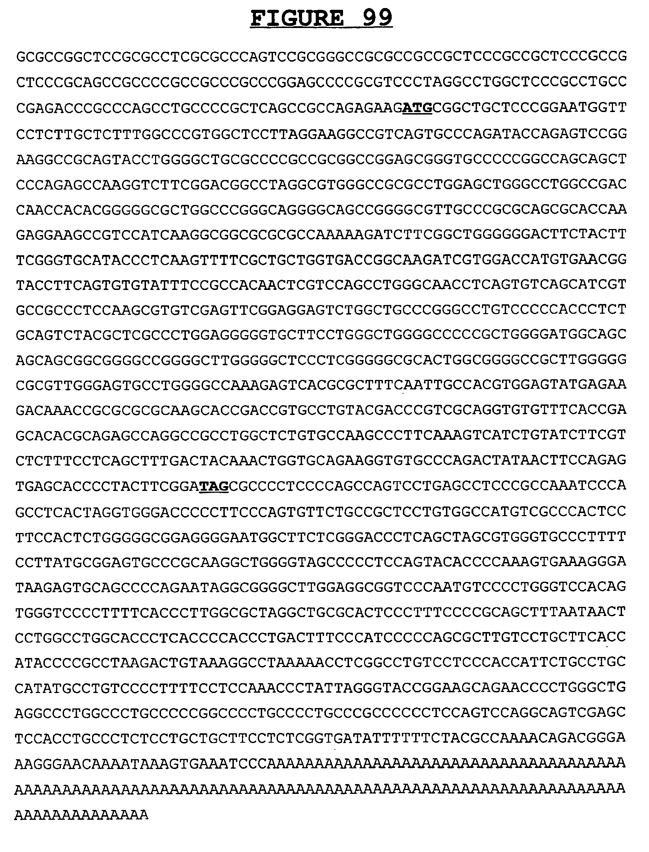

Figure 99 shows a nucleotide sequence (SEQ ID NO:99) of a native sequence PR04403 cDNA, wherein SEQ ID NO:99 is a clone designated herein as "DNA83509-2612".

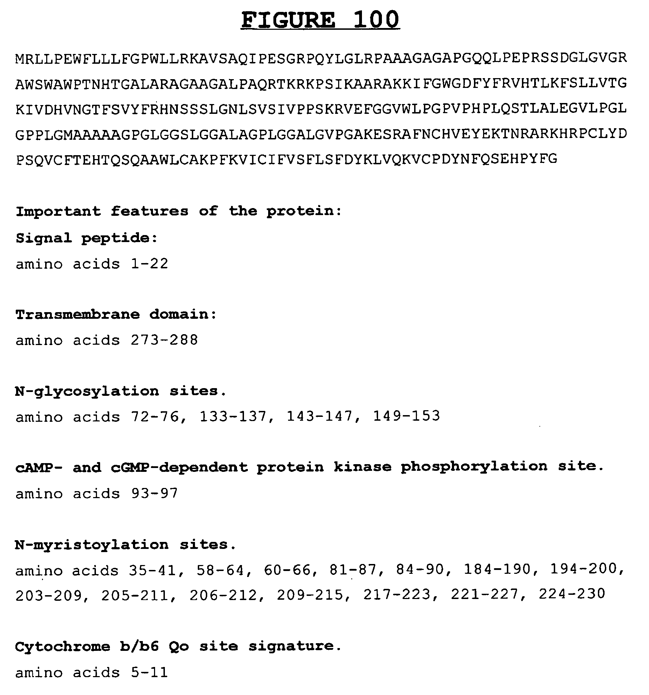

Figure 100 shows the amino acid sequence (SEQ ID NO: 100) derived from the coding sequence of SEQ ID NO: 99 shown in Figure 99.

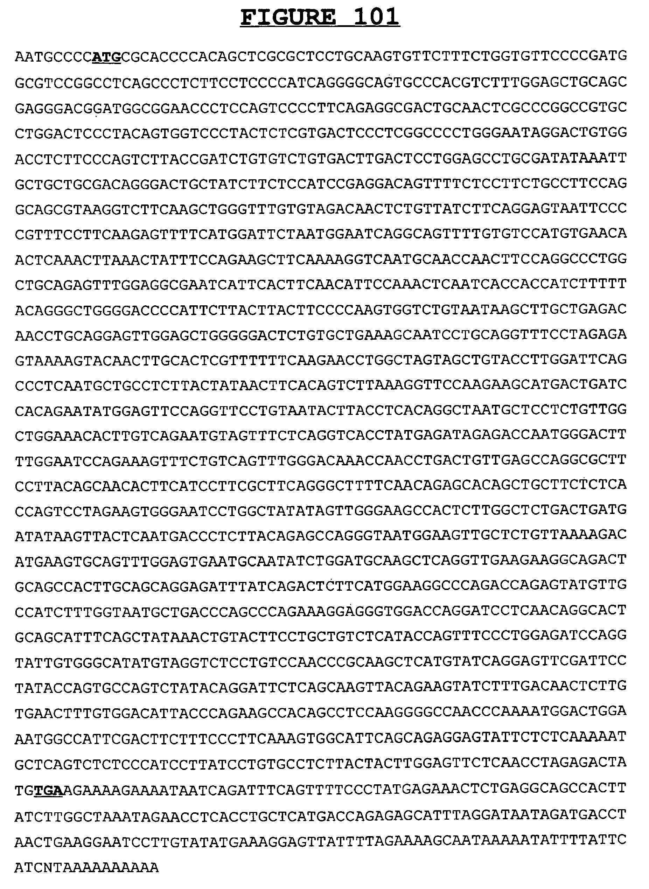

Figure 101 shows a nucleotide sequence (SEQ ID NO:101) of a native sequence PRO4324 cDNA, wherein SEQ ID NO:101 is a clone designated herein as "DNA83560-2569".

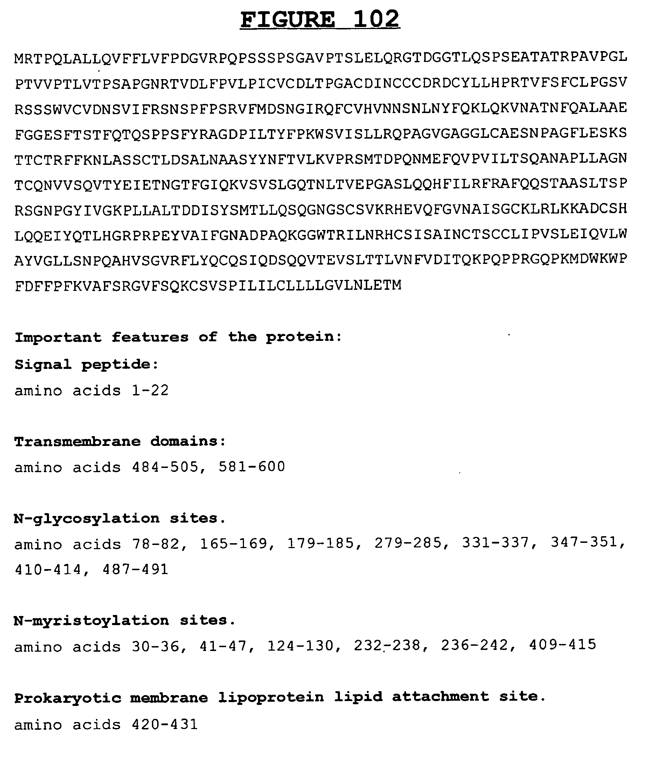

Figure 102 shows the amino acid sequence (SEQ ID NO:102) derived from the coding sequence of SEQ ID NO: 101 shown in Figure 101.

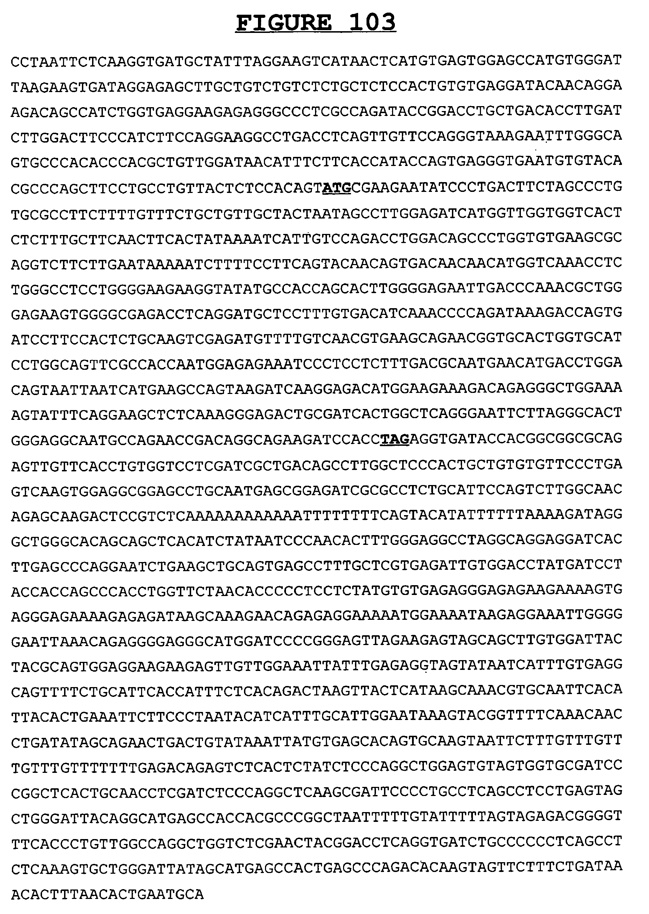

Figure 103 shows a nucleotide sequence (SEQ ID NO:103) of a native sequence PRO4303 cDNA, wherein SEQ ID NO:103 is a clone designated herein as "DNA84139-2555".

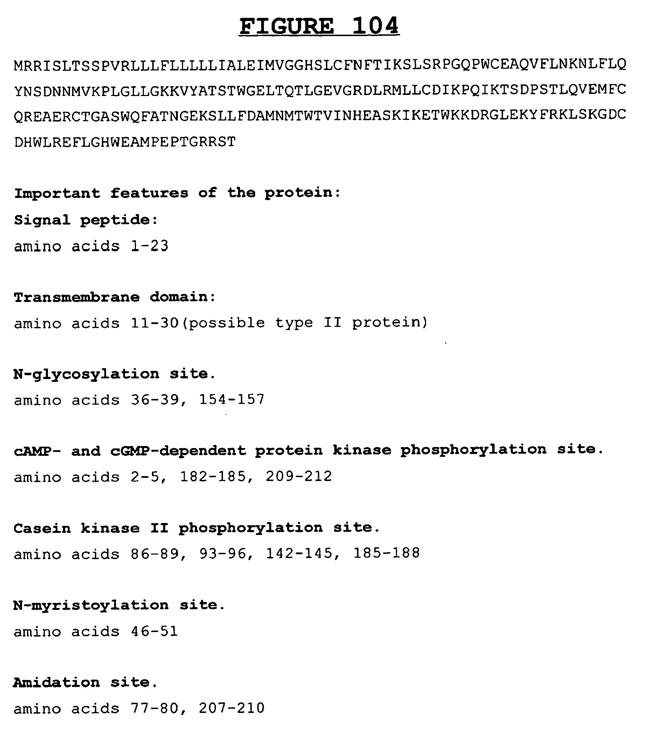

Figure 104 shows the amino acid sequence (SEQ ID NO: 104) derived from the coding sequence of SEQ ID NO: 103 shown in Figure 103.

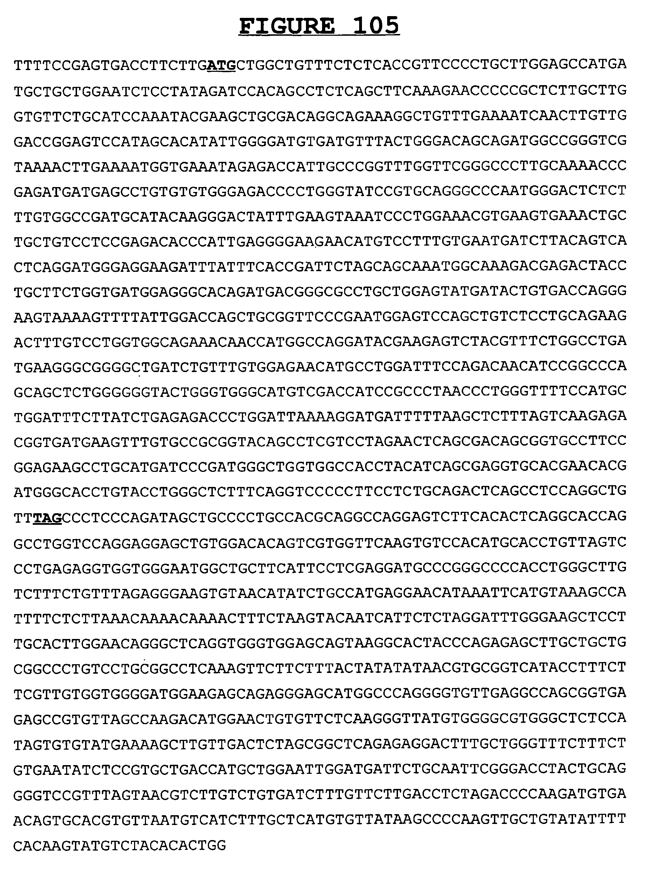

Figure 105 shows a nucleotide sequence (SEQ ID NO:105) of a native sequence PR04305 cDNA, wherein SEQ ID NO:105 is a clone designated herein as "DNA84141-2556".

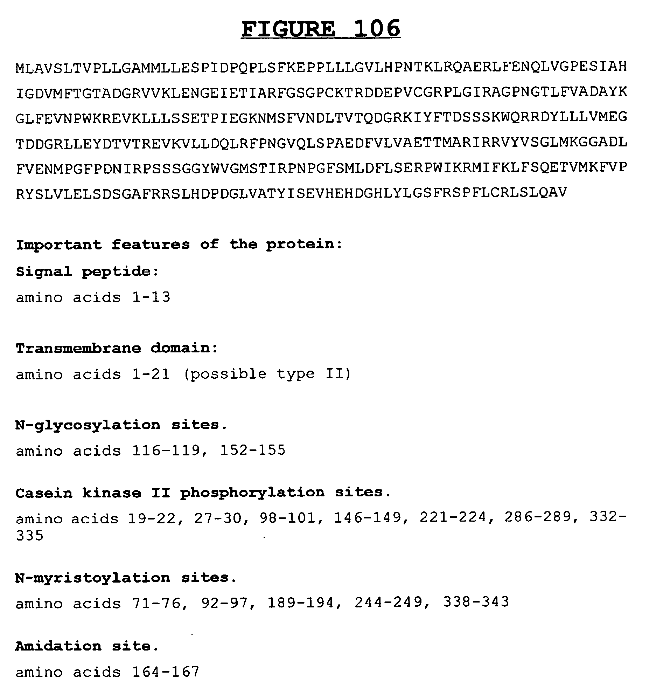

Figure 106 shows the amino acid sequence (SEQ ID NO:106) derived from the coding sequence of SEQ ID NO: 105 shown in Figure 105.

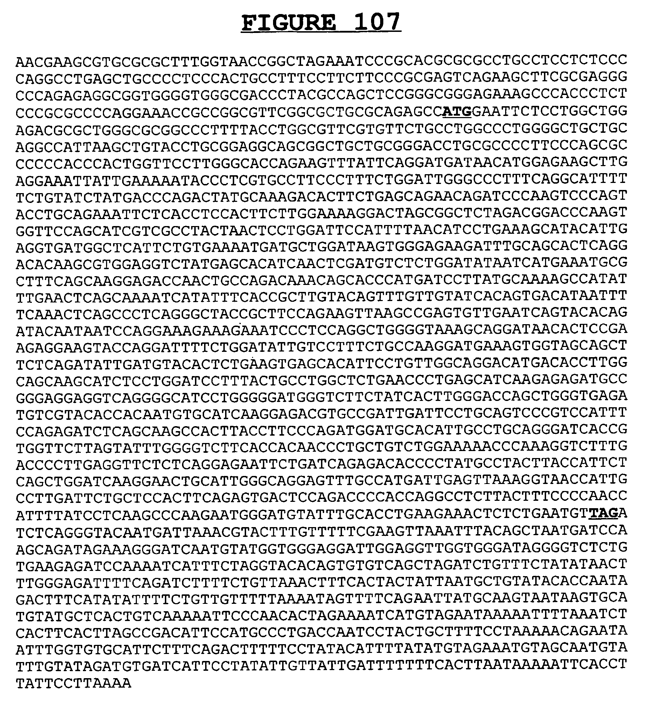

Figure 107 shows a nucleotide sequence (SEQ ID NO:107) of a native sequence PRO4404 cDNA, wherein SEQ ID NO:107 is a clone designated herein as "DNA84142-2613".

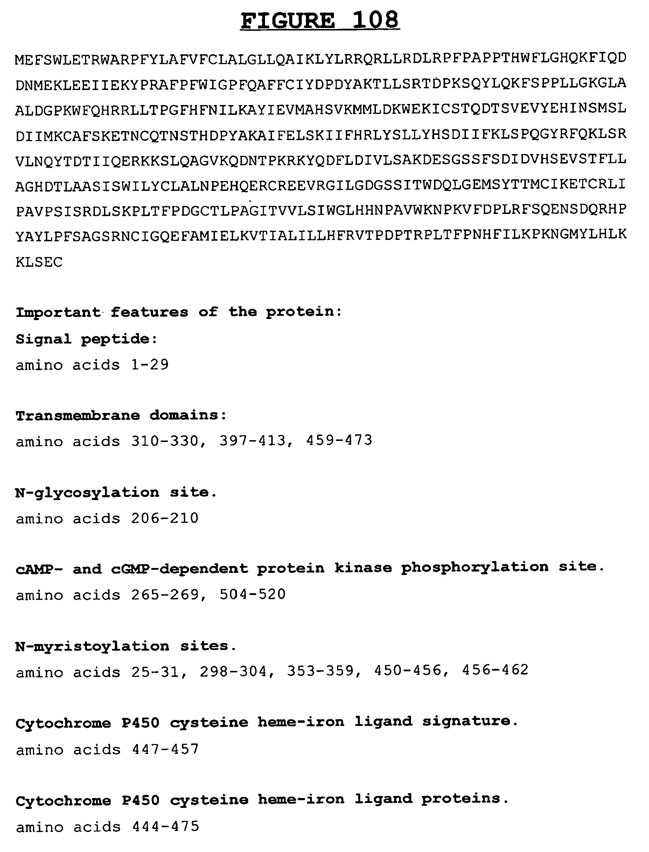

Figure 108 shows the amino acid sequence (SEQ ID NO:108) derived from the coding sequence of SEQ ID NO: 107 shown in Figure 107.

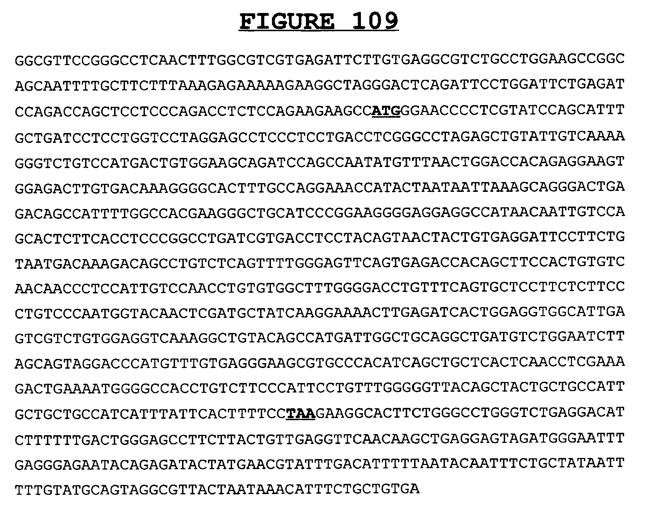

Figure 109 shows a nucleotide sequence (SEQ ID NO:109) of a native sequence PRO1884 cDNA, wherein SEQ ID NO:109 is a clone designated herein as "DNA84318-2520".

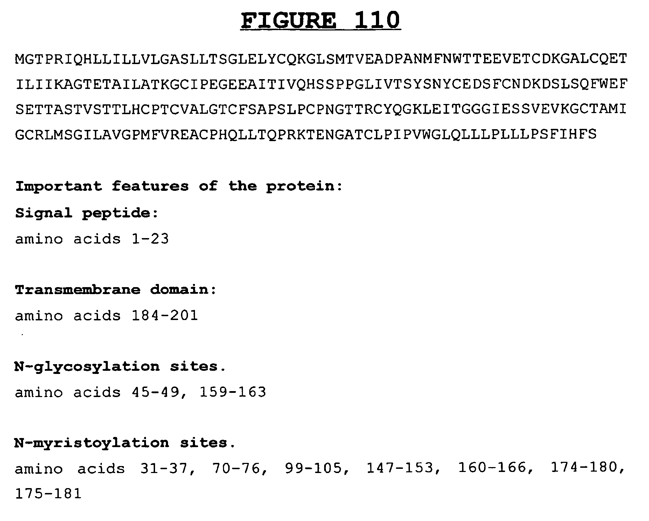

Figure 110 shows the amino acid sequence (SEQ ID NO:110) derived from the coding sequence of SEQ ID NO: 109 shown in Figure 109.

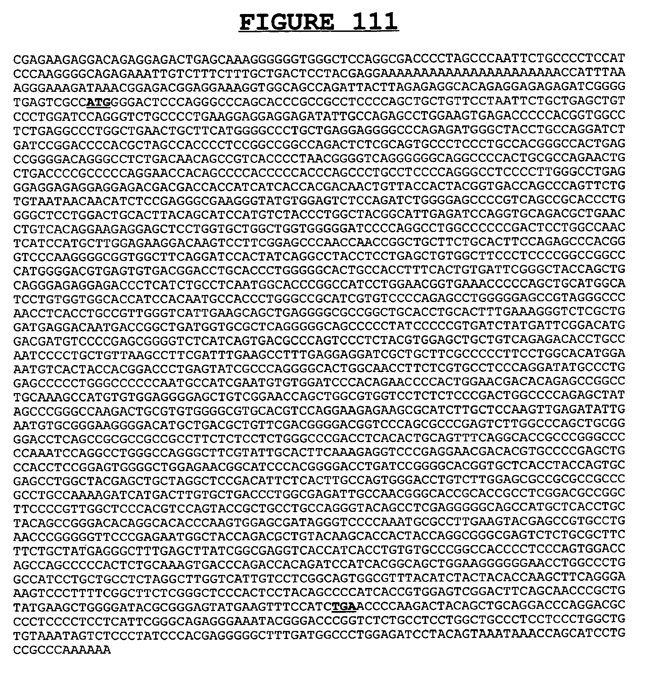

Figure 111 shows a nucleotide sequence (SEQ ID NO:111) of a native sequence PR04349 cDNA, wherein SEQ ID NO:111 is a clone designated herein as "DNA84909-2590".

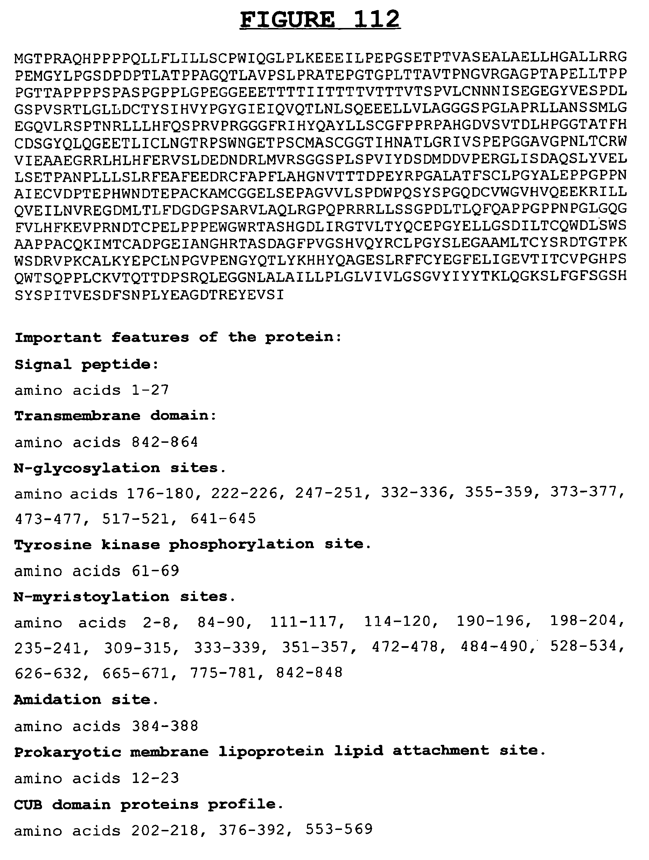

Figure 112 shows the amino acid sequence (SEQ ID NO:112) derived from the coding sequence of SEQ ID NO: 111 shown in Figure 111.

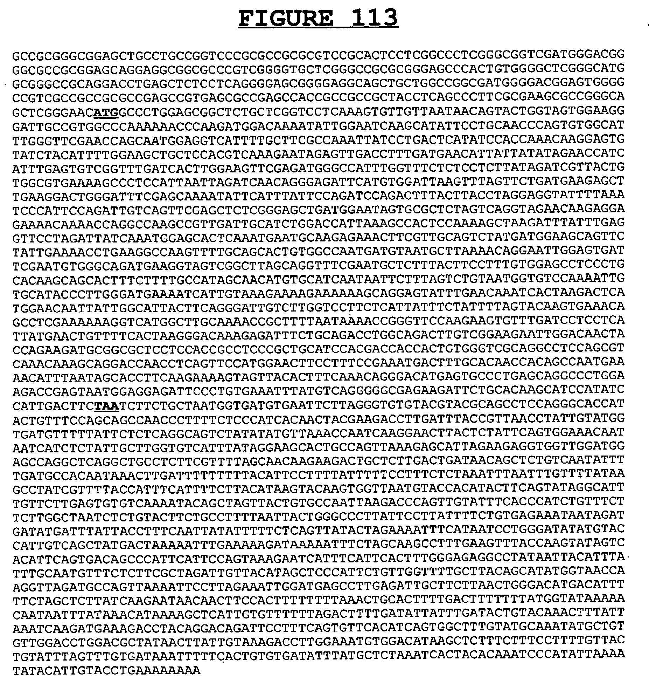

Figure 113 shows a nucleotide sequence (SEQ ID NO:113) of a native sequence PR04401 cDNA, wherein SEQ ID NO:113 is a clone designated herein as "DNA84912-2610".

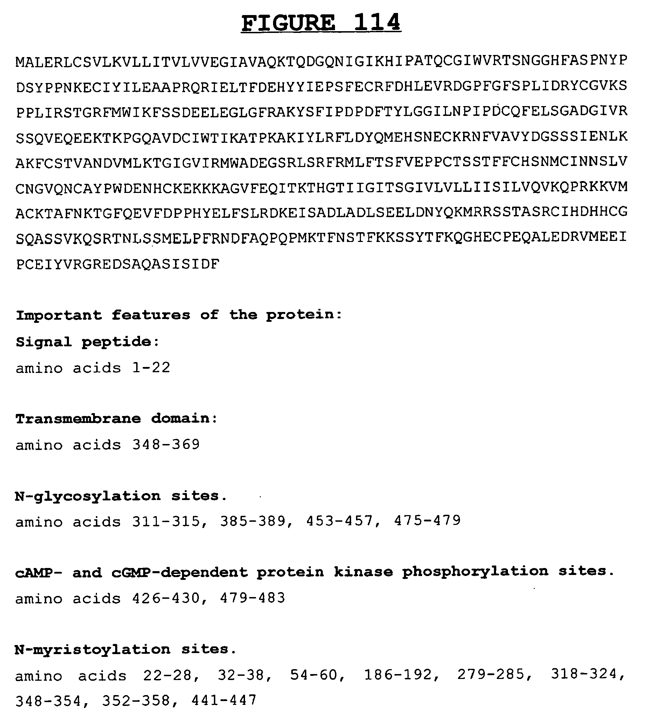

Figure 114 shows the amino acid sequence (SEQ ID NO:114) derived from the coding sequence of SEQ ID NO: 113 shown in Figure 113.

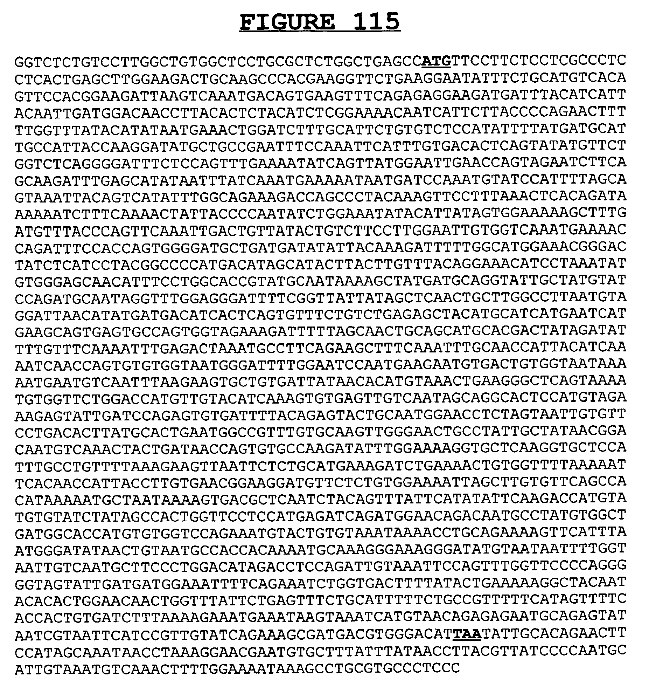

Figure 115 shows a nucleotide sequence (SEQ ID NO:115) of a native sequence PRO1867 cDNA, wherein SEQ ID NO:115 is a clone designated herein as "DNA84925-2514".

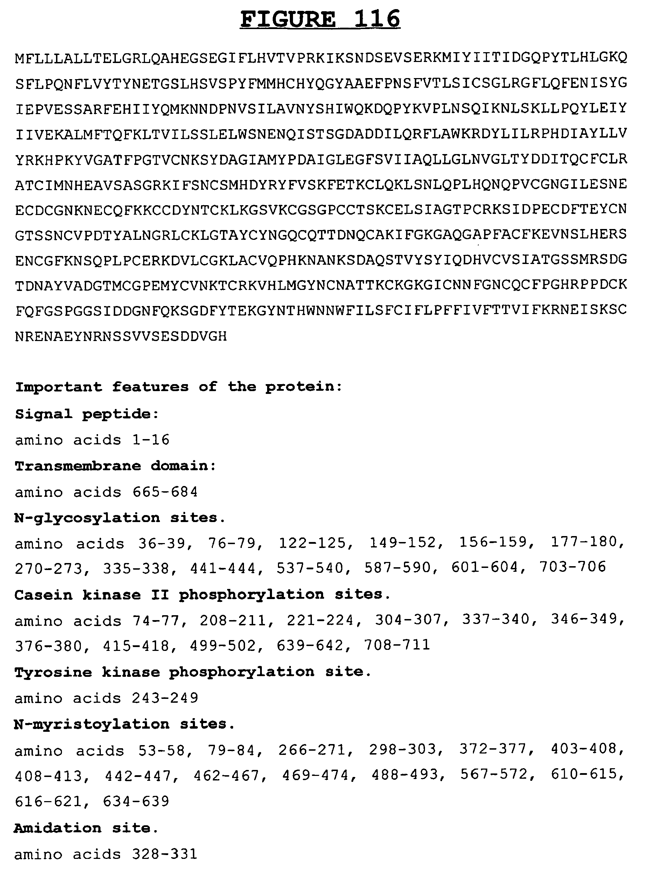

Figure 116 shows the amino acid sequence (SEQ ID NO:116) derived from the coding sequence of SEQ ID NO: 115 shown in Figure 115.

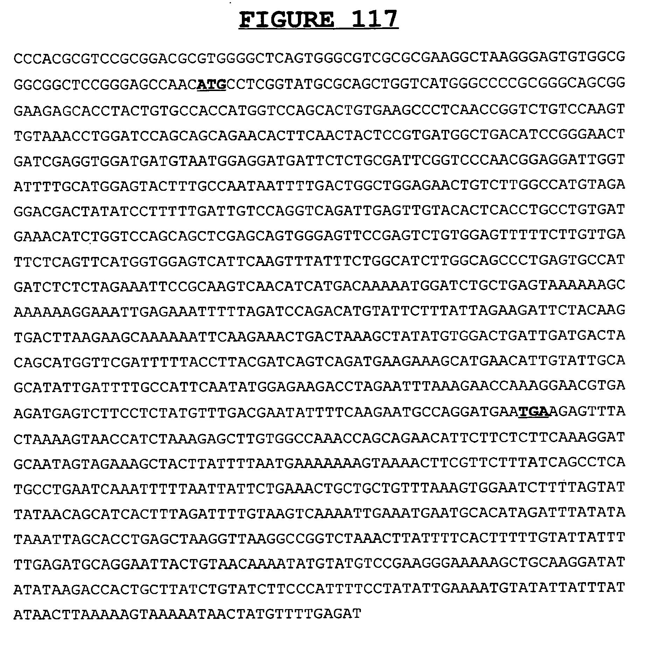

Figure 117 shows a nucleotide sequence (SEQ ID NO:117) of a native sequence PRO4319 cDNA, wherein SEQ ID NO:117 is a clone designated herein as "DNA84928-2564".

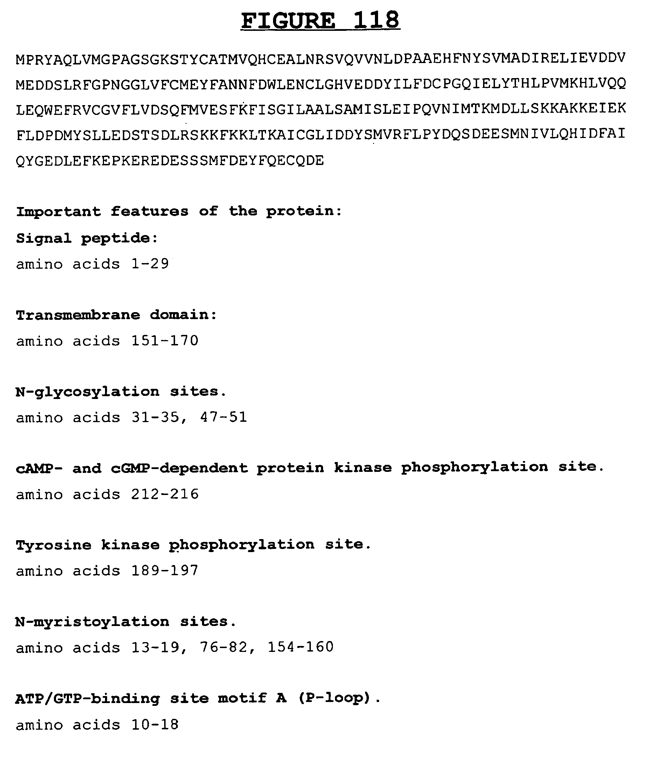

Figure 118 shows the amino acid sequence (SEQ ID NO:118) derived from the coding sequence of SEQ ID NO: 117 shown in Figure 117.

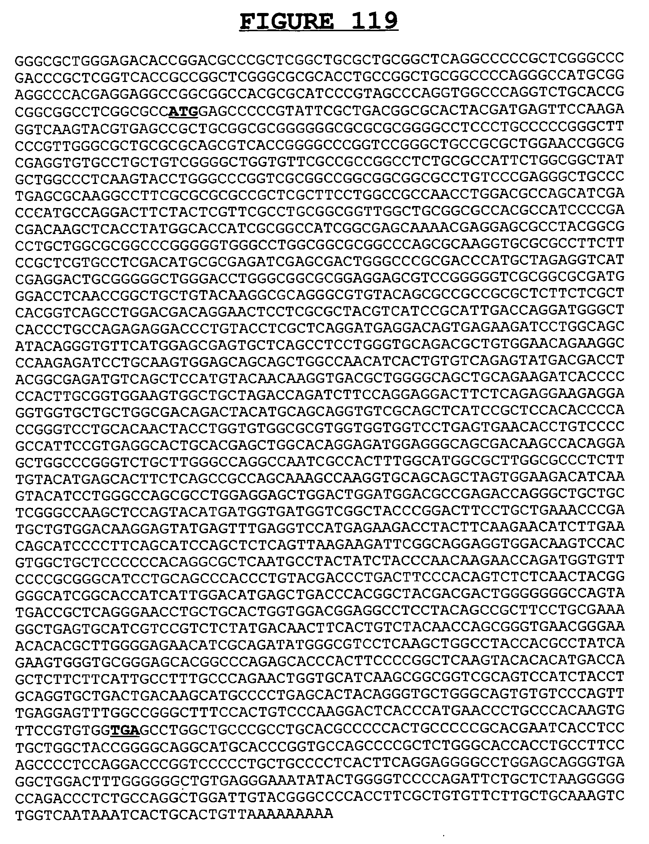

Figure 119 shows a nucleotide sequence (SEQ ID NO:119) of a native sequence PR04991 cDNA, wherein SEQ ID NO:119 is a clone designated herein as "DNA84932-2657".

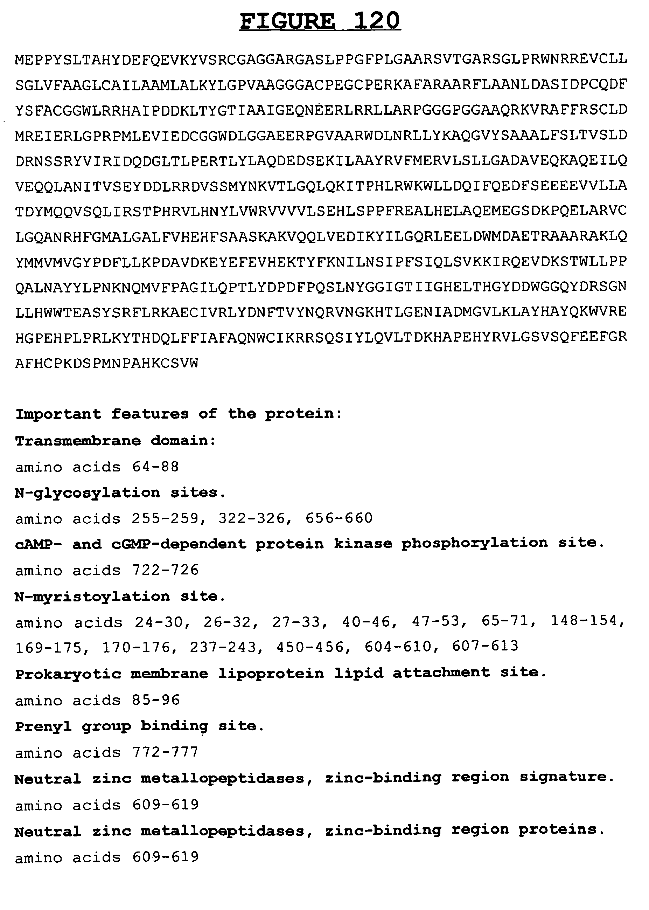

Figure 120 shows the amino acid sequence (SEQ ID NO:120) derived from the coding sequence of SEQ ID NO: 119 shown in Figure 119.

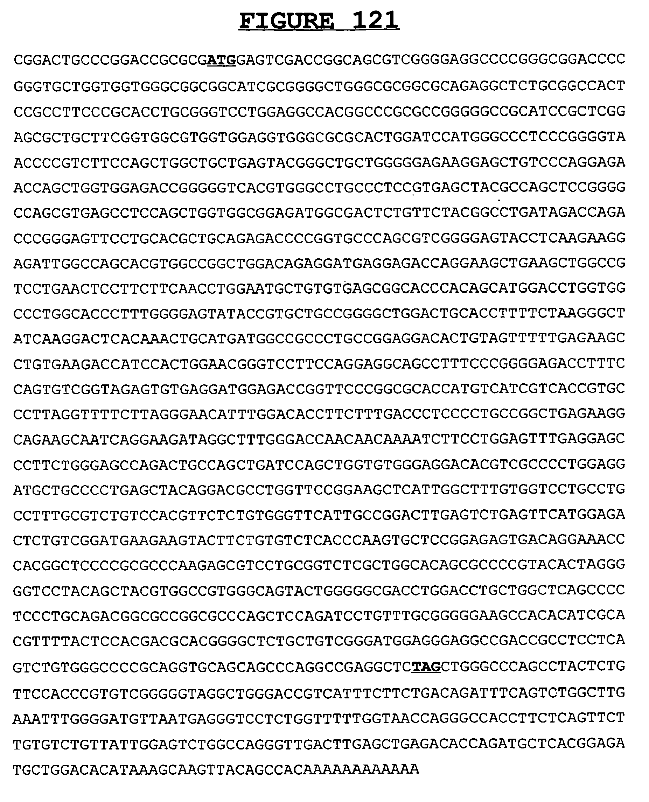

Figure 121 shows a nucleotide sequence (SEQ ID NO:121) of a native sequence PR04398 cDNA, wherein SEQ ID NO:121 is a clone designated herein as "DNA86592-2607".

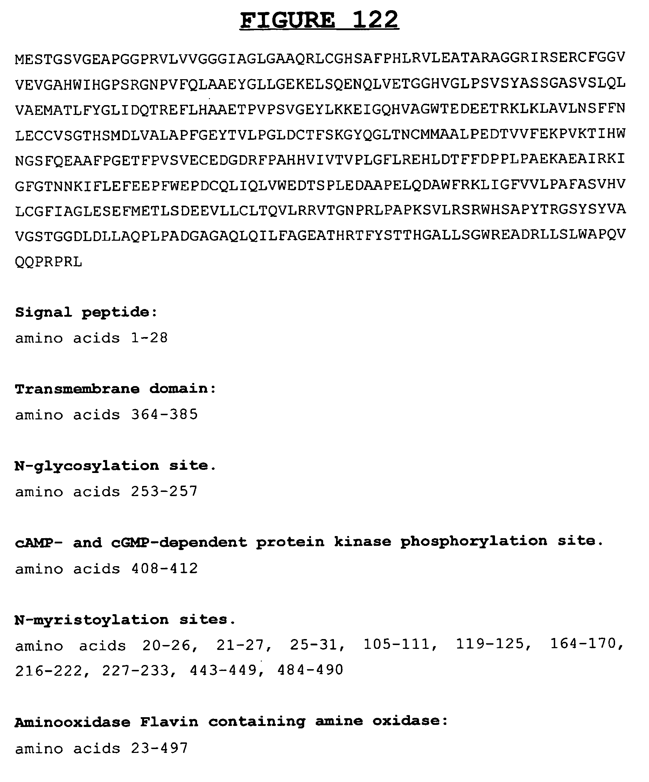

Figure 122 shows the amino acid sequence (SEQ ID NO:122) derived from the coding sequence of SEQ ID NO: 121 shown in Figure 121.

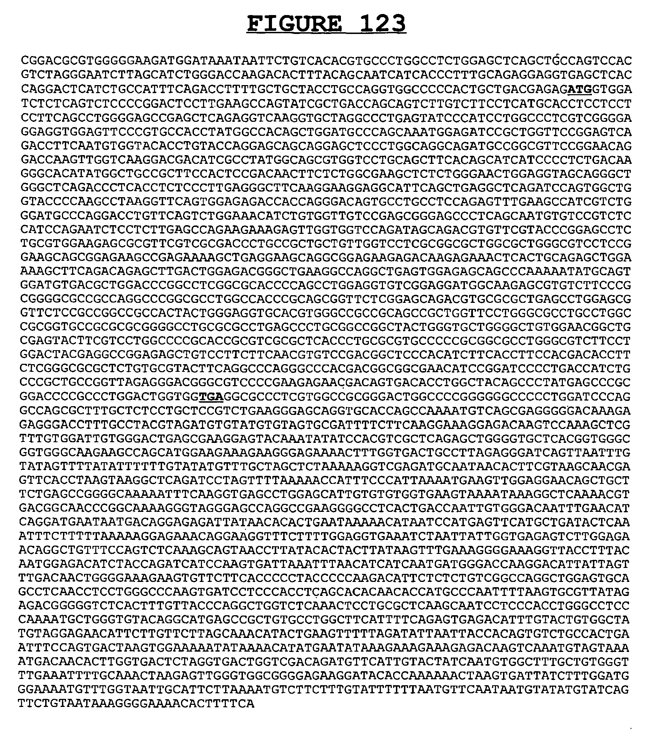

Figure 123 shows a nucleotide sequence (SEQ ID NO:123) of a native sequence PR04346 cDNA, wherein SEQ ID NO:123 is a clone designated herein as "DNA86594-2587".

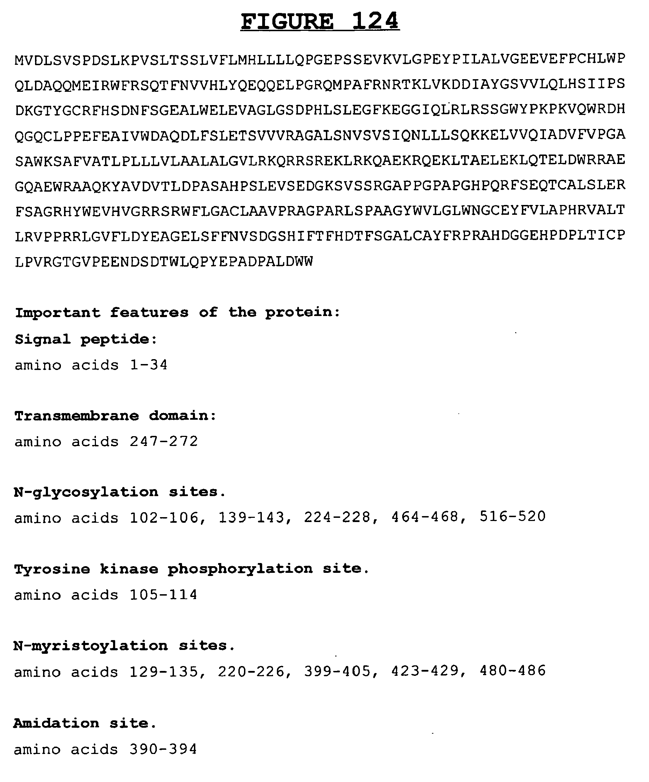

Figure 124 shows the amino acid sequence (SEQ ID NO:124) derived from the coding sequence of SEQ ID NO: 123 shown in Figure 123.

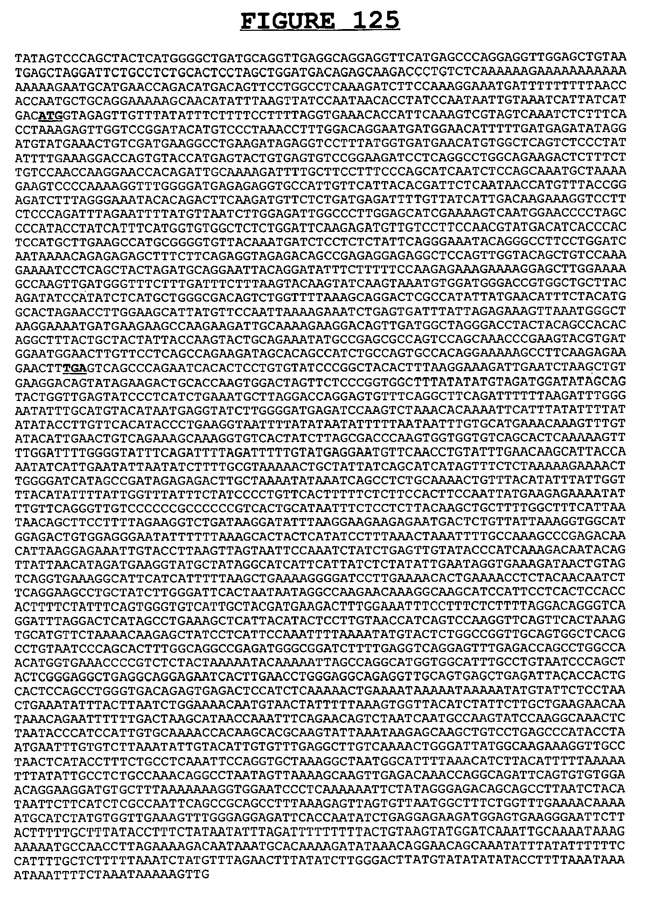

Figure 125 shows a nucleotide sequence (SEQ ID NO:125) of a native sequence PR04350 cDNA, wherein SEQ ID NO:125 is a clone designated herein as "DNA86647-2591".

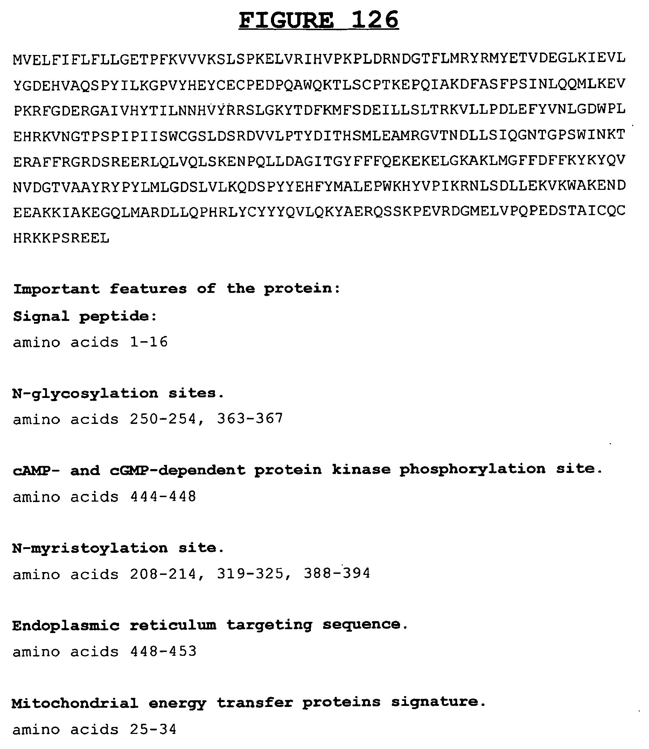

Figure 126 shows the amino acid sequence (SEQ ID NO:126) derived from the coding sequence of SEQ ID NO: 125 shown in Figure 125.

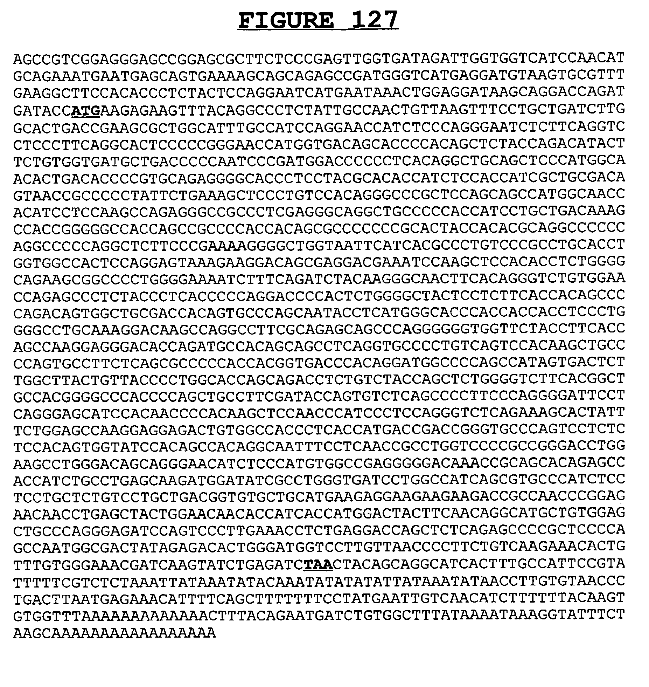

Figure 127 shows a nucleotide sequence (SEQ ID NO:127) of a native sequence PR04318 cDNA, wherein SEQ ID NO:127 is a clone designated herein as "DNA87185-2563".

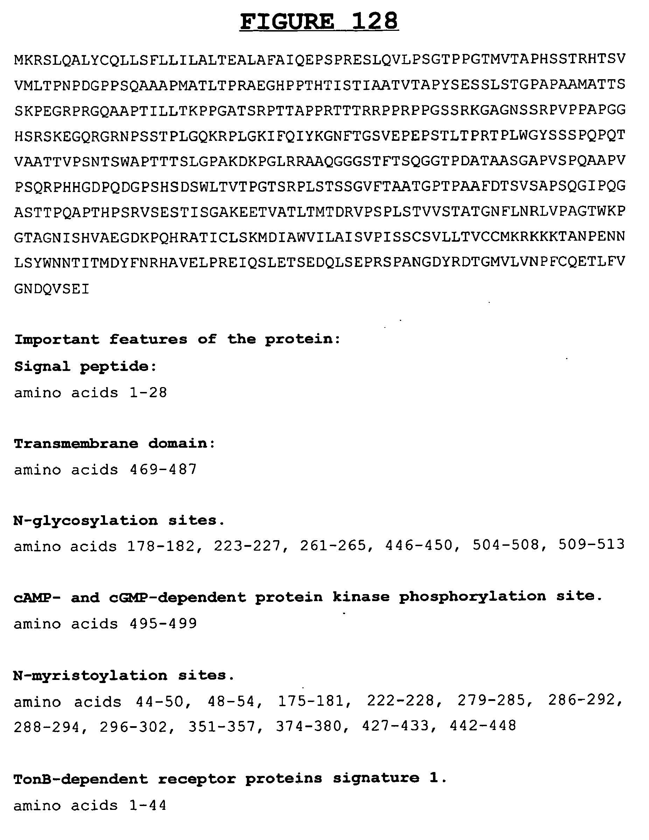

Figure 128 shows the amino acid sequence (SEQ ID NO:128) derived from the coding sequence of SEQ ID NO: 127 shown in Figure 127.

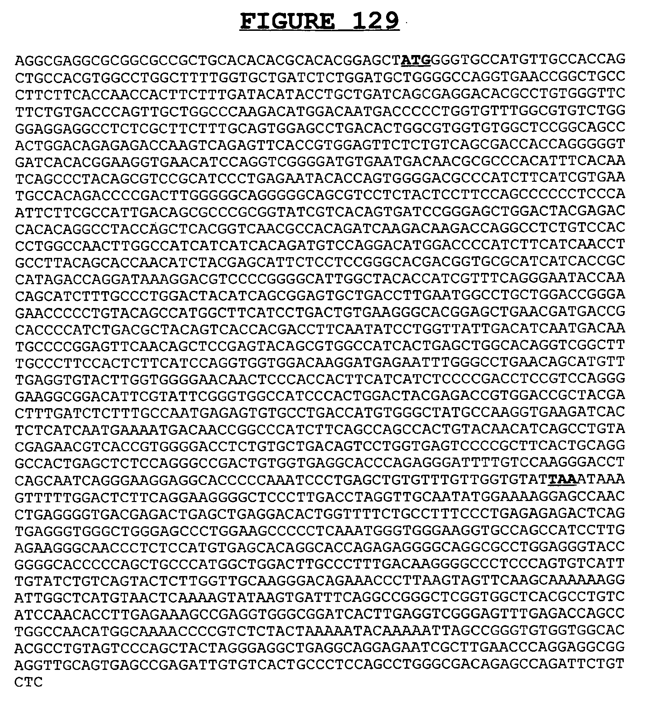

Figure 129 shows a nucleotide sequence (SEQ ID NO:129) of a native sequence PR04340 cDNA, wherein SEQ ID NO:129 is a clone designated herein as "DNA87656-2582".

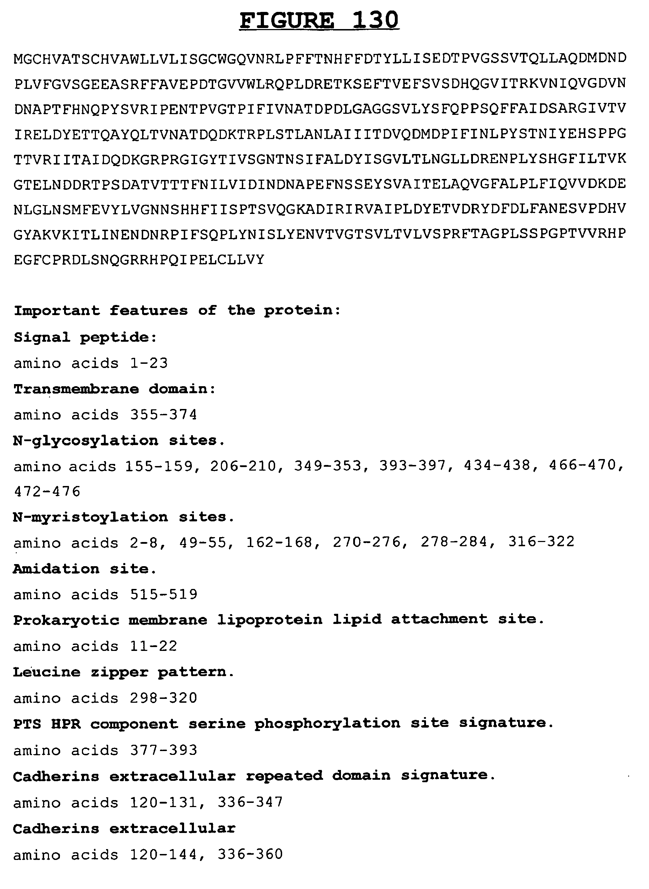

Figure 130 shows the amino acid sequence (SEQ ID NO:130) derived from the coding sequence of SEQ ID NO: 129 shown in Figure 129.

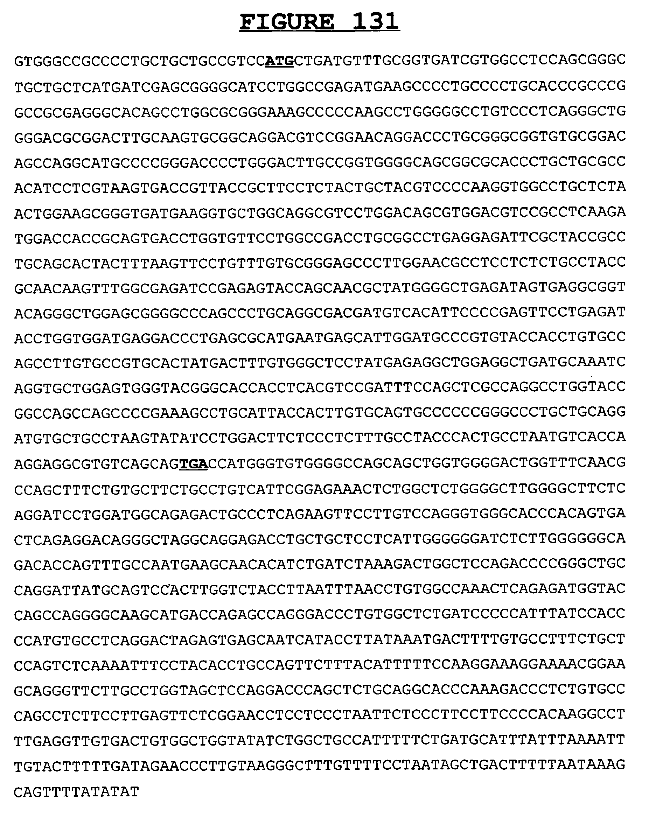

Figure 131 shows a nucleotide sequence (SEQ ID NO:131) of a native sequence PR04400 cDNA, wherein SEQ ID NO:131 is a clone designated herein as "DNA87974-2609".

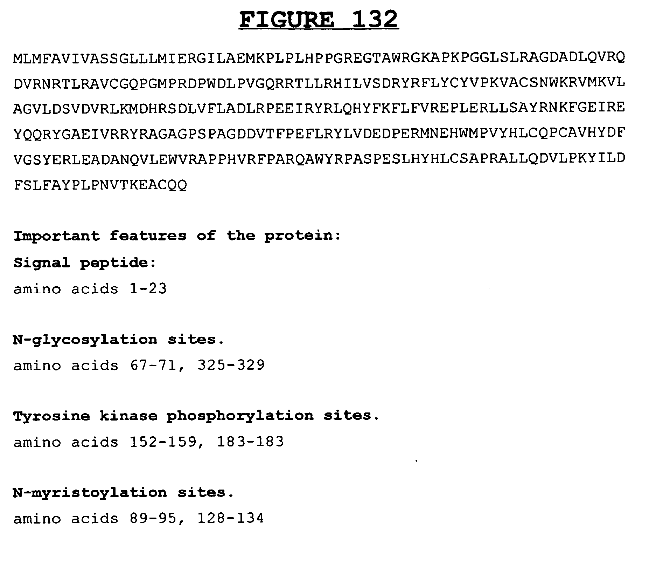

Figure 132 shows the amino acid sequence (SEQ ID NO:132) derived from the coding sequence of SEQ ID NO: 131 shown in Figure 131.

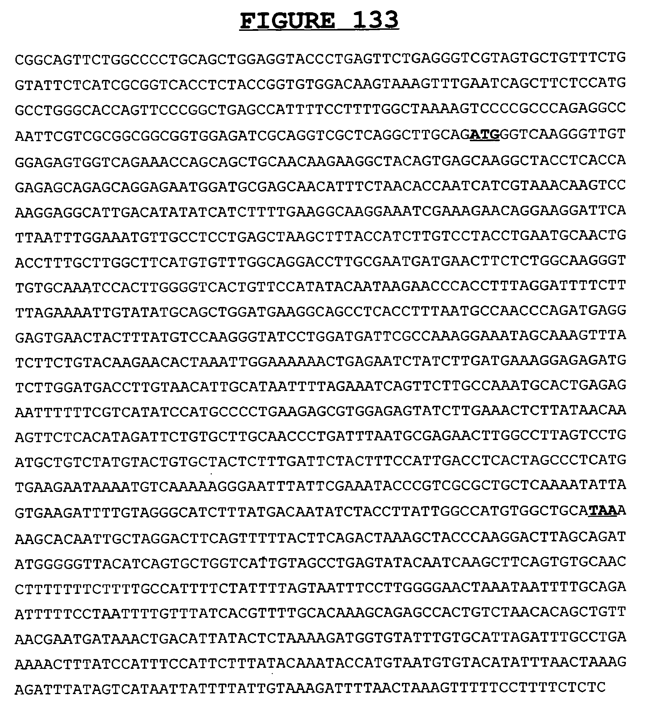

Figure 133 shows a nucleotide sequence (SEQ ID NO:133) of a native sequence PR04320 cDNA, wherein SEQ ID NO:133 is a clone designated herein as "DNA88001-2565".

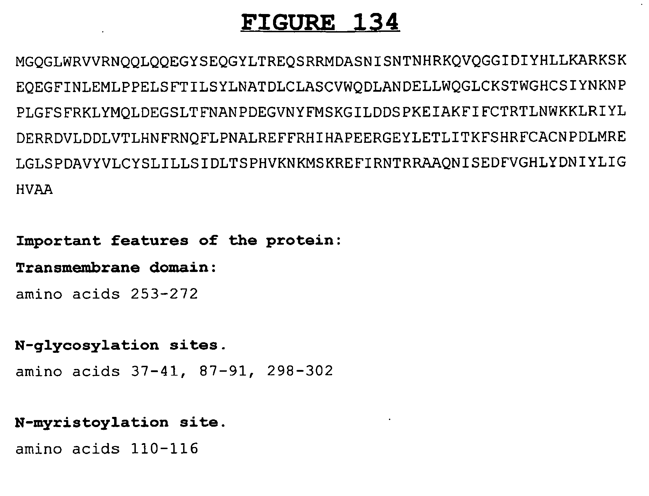

Figure 134 shows the amino acid sequence (SEQ ID NO: 134) derived from the coding sequence of SEQ ID NO: 133 shown in Figure 133.

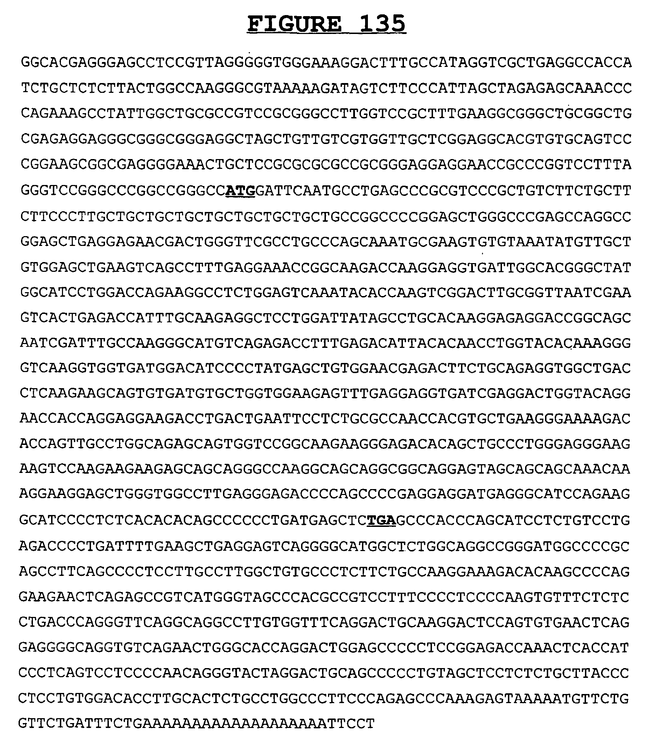

Figure 135 shows a nucleotide sequence (SEQ ID NO:135) of a native sequence PR04409 cDNA, wherein SEQ ID NO:135 is a clone designated herein as "DNA88004-2575".

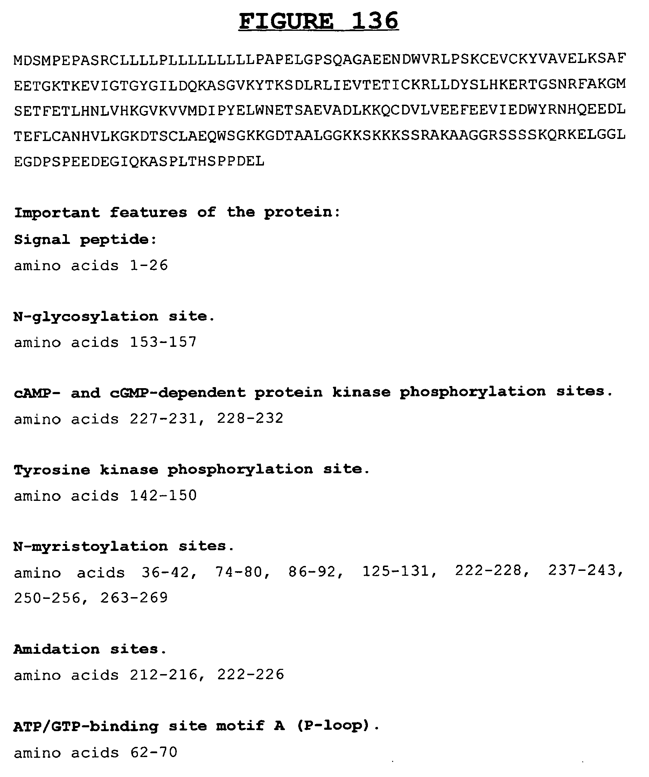

Figure 136 shows the amino acid sequence (SEQ ID NO:136) derived from the coding sequence of SEQ ID NO: 135 shown in Figure 135.

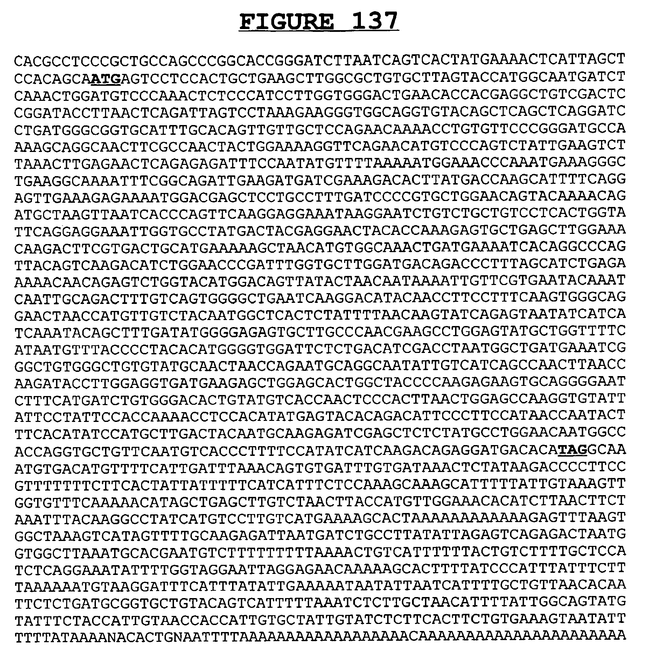

Figure 137 shows a nucleotide sequence (SEQ ID NO:137) of a native sequence PR04399 cDNA, wherein SEQ ID NO:137 is a clone designated herein as "DNA89220-2608".

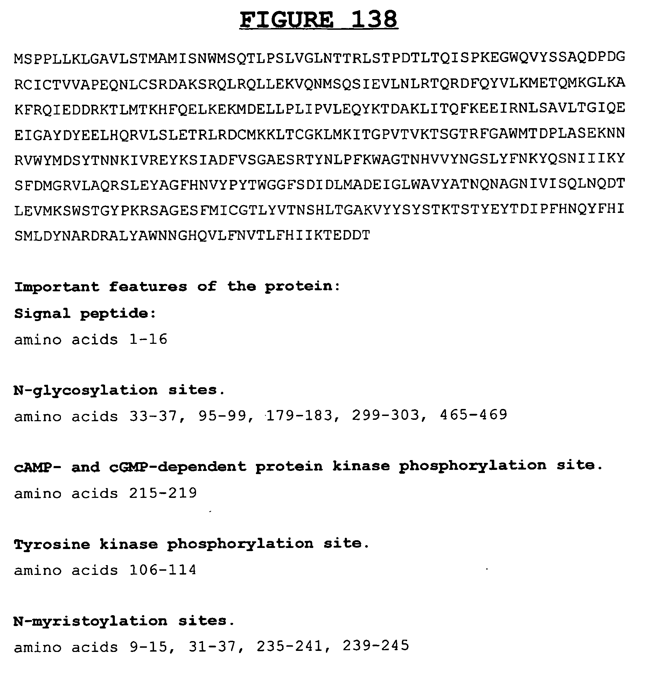

Figure 138 shows the amino acid sequence (SEQ ID NO:138) derived from the coding sequence of SEQ ID NO: 137 shown in Figure 137.

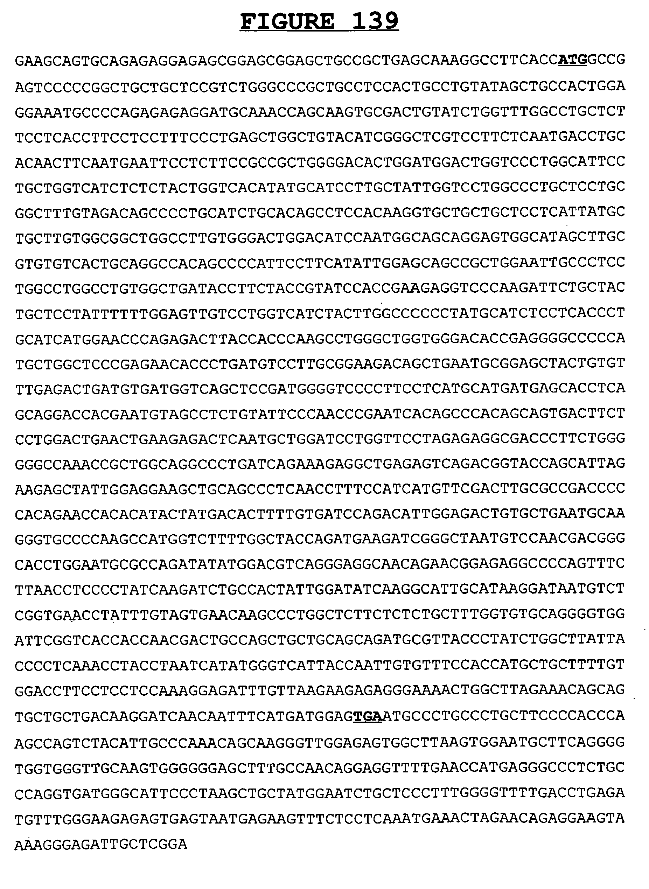

Figure 139 shows a nucleotide sequence (SEQ ID NO: 139) of a native sequence PRO4418 cDNA, wherein SEQ ID NO:139 is a clone designated herein as "DNA89947-2618".

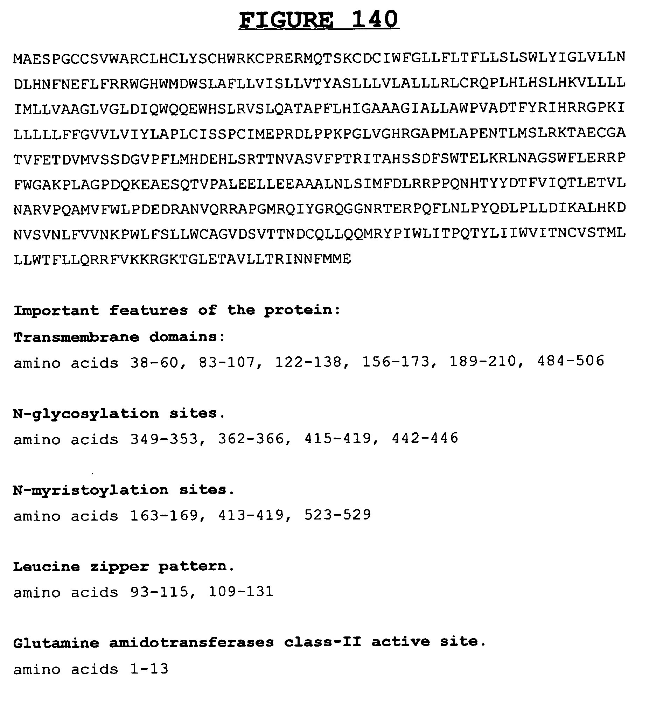

Figure 140 shows the amino acid sequence (SEQ ID NO:140) derived from the coding sequence of SEQ ID NO: 139 shown in Figure 139.

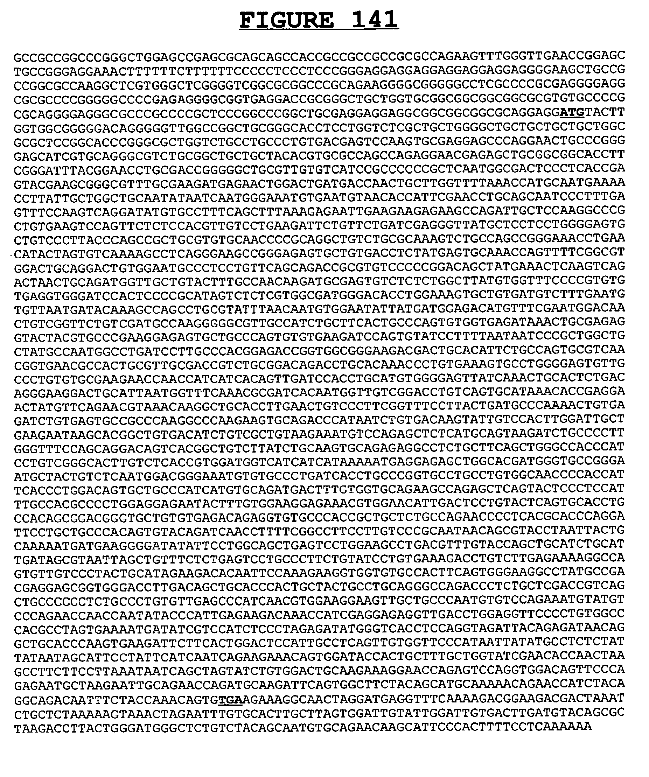

Figure 141 shows a nucleotide sequence (SEQ ID NO: 141) of a native sequence PR04330 cDNA, wherein SEQ ID NO:141 is a clone designated herein as "DNA90842-2574".

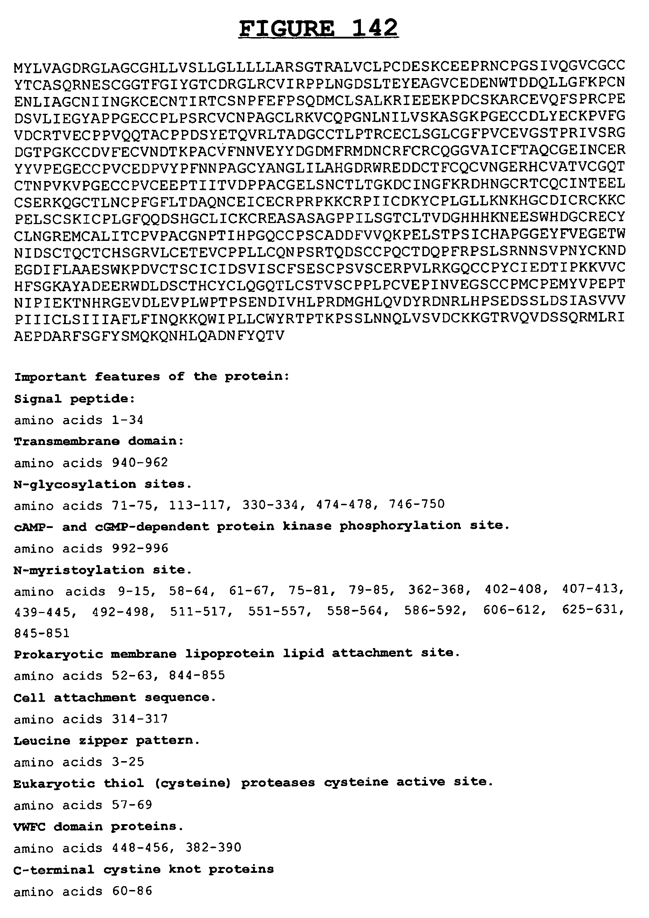

Figure 142 shows the amino acid sequence (SEQ ID NO:142) derived from the coding sequence of SEQ ID NO: 141 shown in Figure 141.

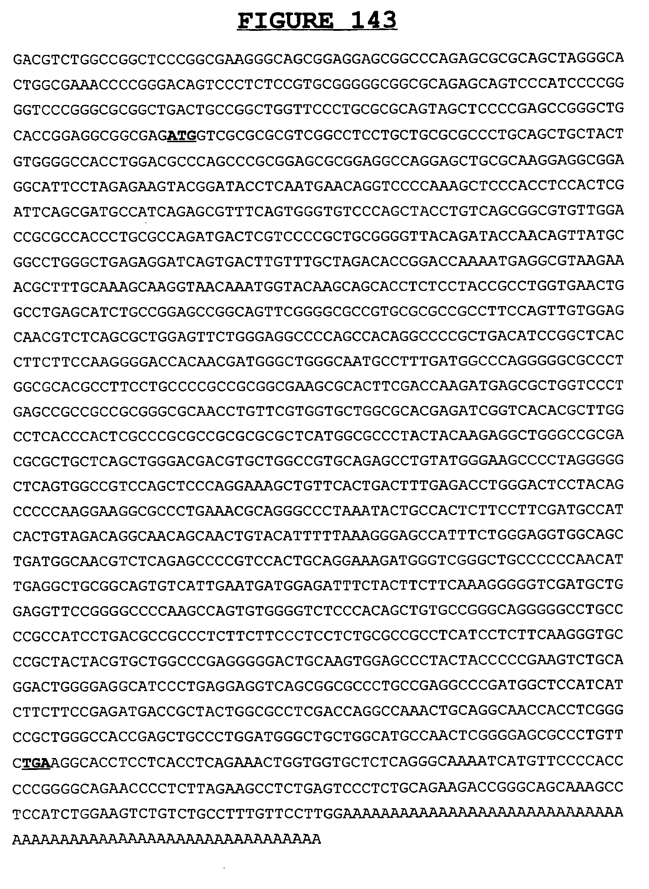

Figure 143 shows a nucleotide sequence (SEQ ID NO:143) of a native sequence PR04339 cDNA, wherein SEQ ID NO:143 is a clone designated herein as "DNA91775-2581".

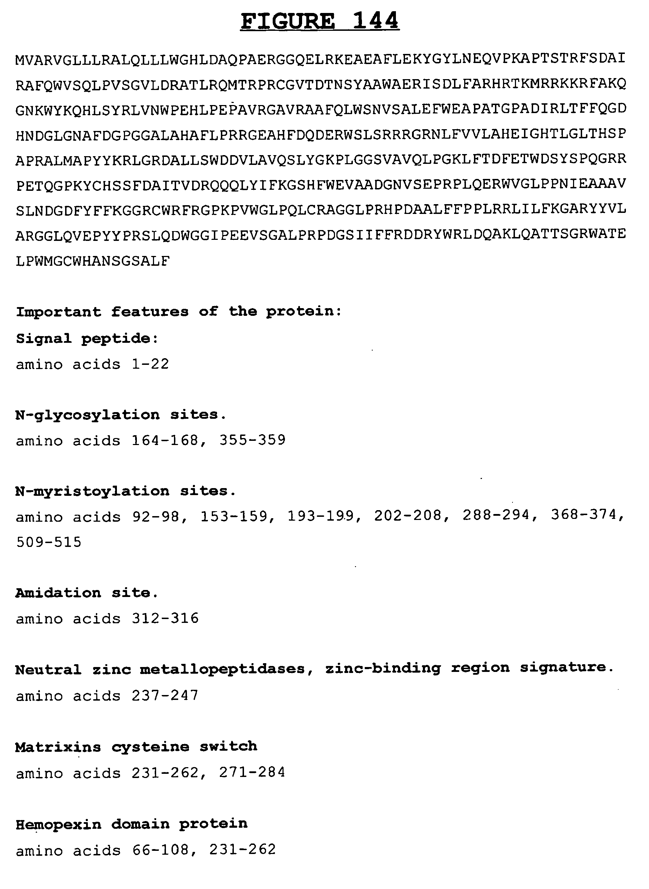

Figure 144 shows the amino acid sequence (SEQ ID NO:144) derived from the coding sequence of SEQ ID NO: 143 shown in Figure 143.

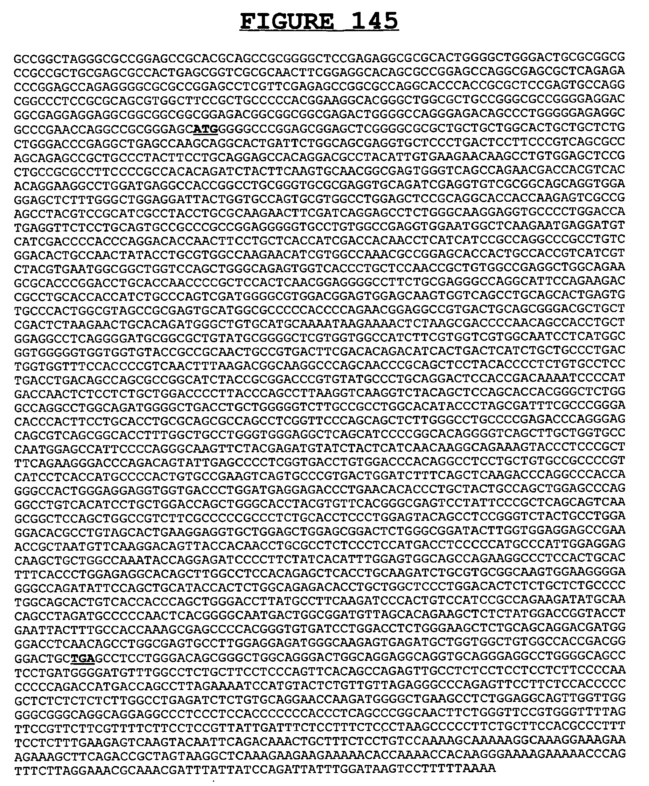

Figure 145 shows a nucleotide sequence (SEQ ID NO:145) of a native sequence PRO4326 cDNA, wherein SEQ ID NO:145 is a clone designated herein as "DNA91779-2571".

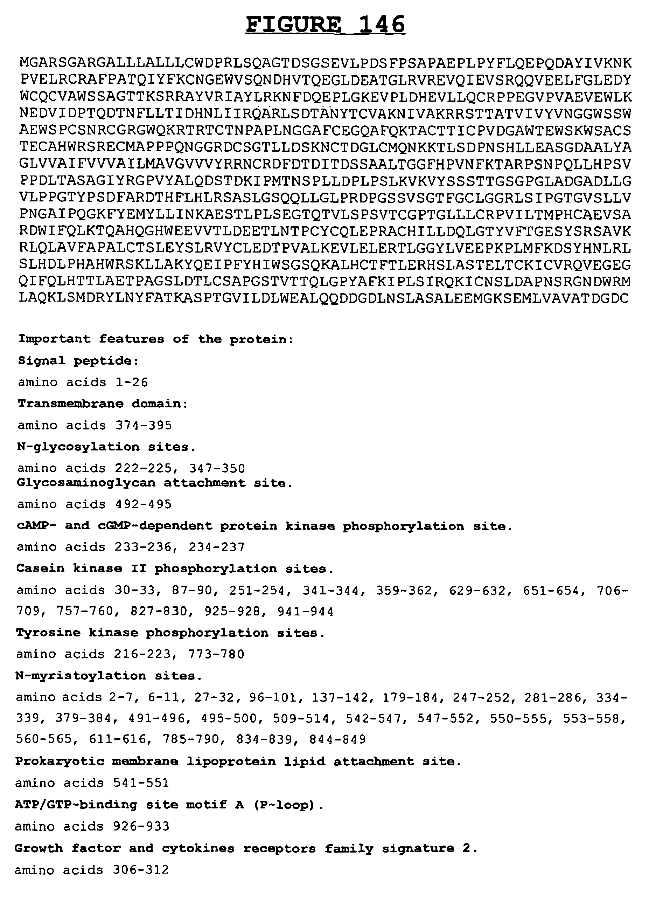

Figure 146 shows the amino acid sequence (SEQ ID NO:146) derived from the coding sequence of SEQ ID NO: 145 shown in Figure 145.

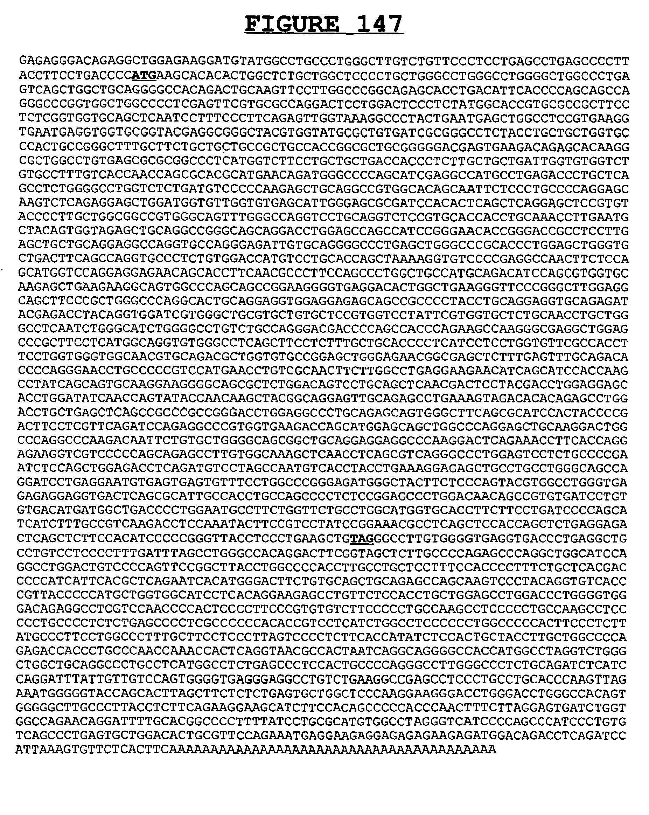

Figure 147 shows a nucleotide sequence (SEQ ID NO:147) of a native sequence PR06014 cDNA, wherein SEQ ID NO: 147 is a clone designated herein as "DNA92217-2697".

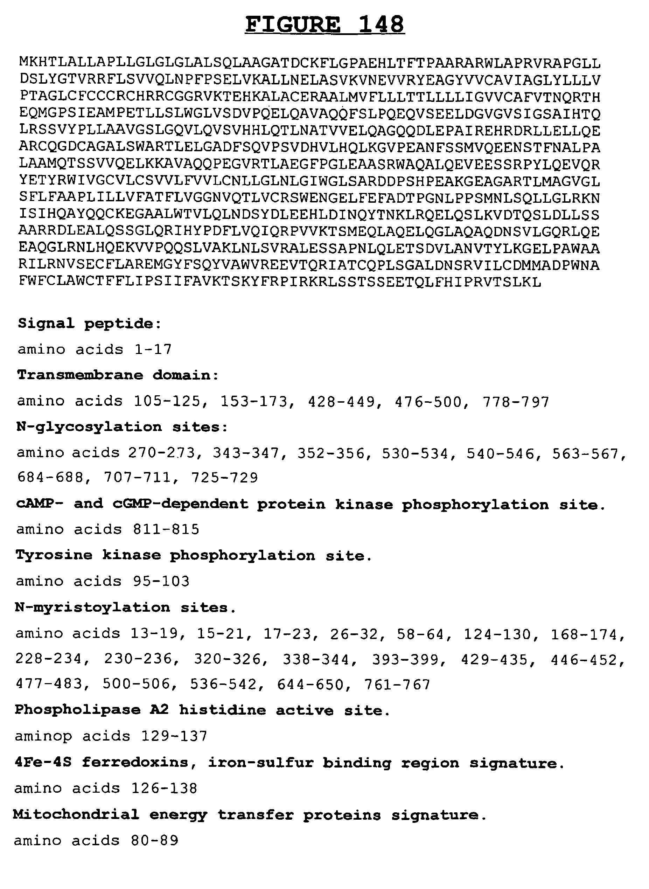

Figure 148 shows the amino acid sequence (SEQ ID NO: 148) derived from the coding sequence of SEQ ID NO: 147 shown in Figure 147.

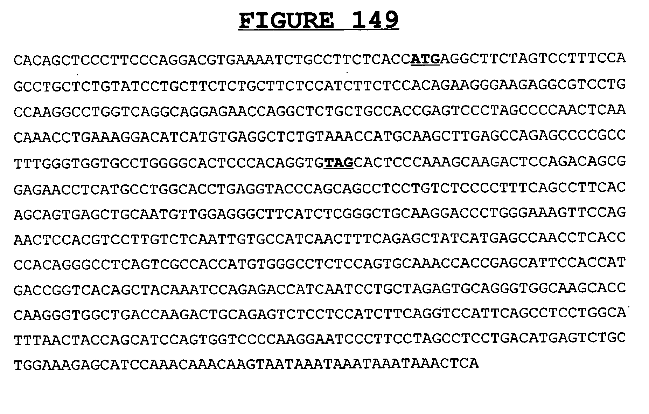

Figure 149 shows a nucleotide sequence (SEQ ID NO:149) of a native sequence PR03446 cDNA. wherein SEQ ID NO:149 is a clone designated herein as "DNA92219-2541".

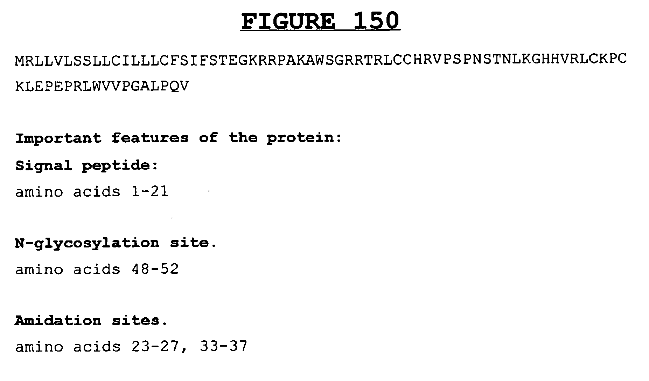

Figure 150 shows the amino acid sequence (SEQ ID NO:150) derived from the coding sequence of SEQ ID NO: 149 shown in Figure 149.

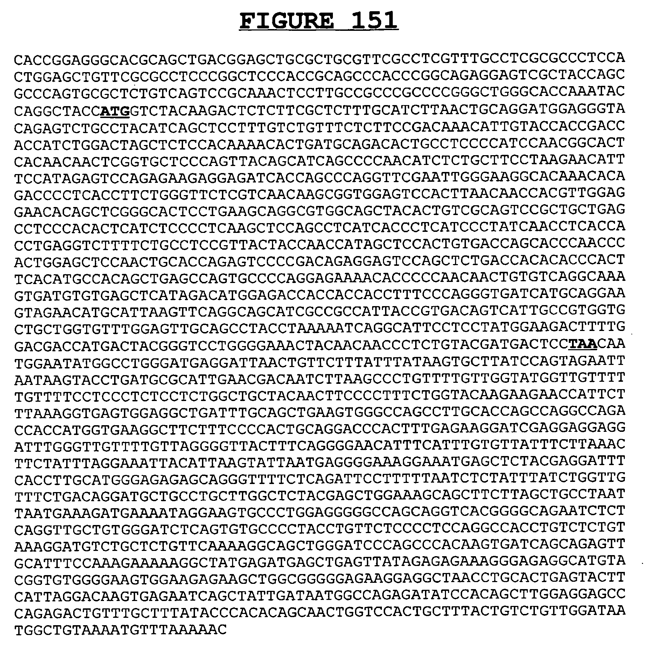

Figure 151 shows a nucleotide sequence (SEQ ID NO:151) of a native sequence PR04322 cDNA. wherein SEQ ID NO:151 is a clone designated herein as "DNA92223-2567".

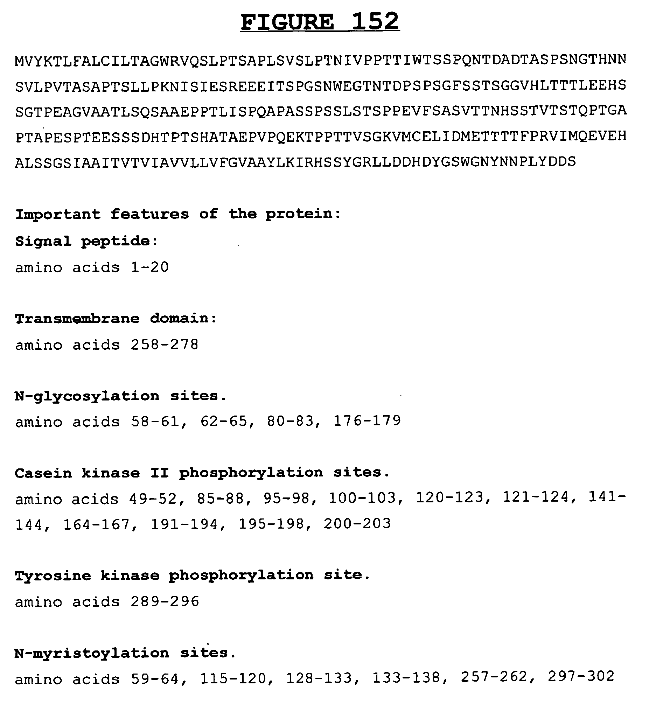

Figure 152 shows the amino acid sequence (SEQ ID NO:152) derived from the coding sequence of SEQ ID NO: 151 shown in Figure 151.

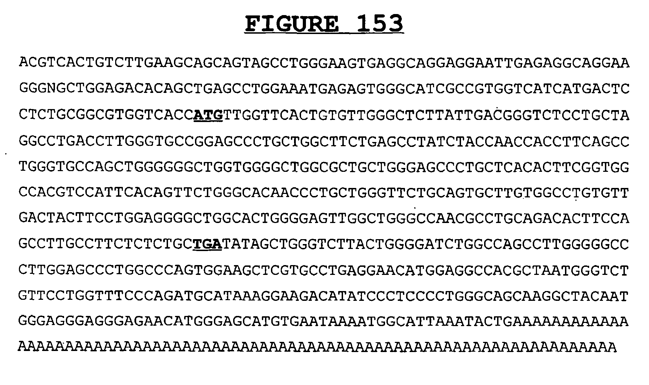

Figure 153 shows a nucleotide sequence (SEQ ID NO:153) of a native sequence PR04381 cDNA, wherein SEQ ID NO:153 is a clone designated herein as "DNA92225-2603".

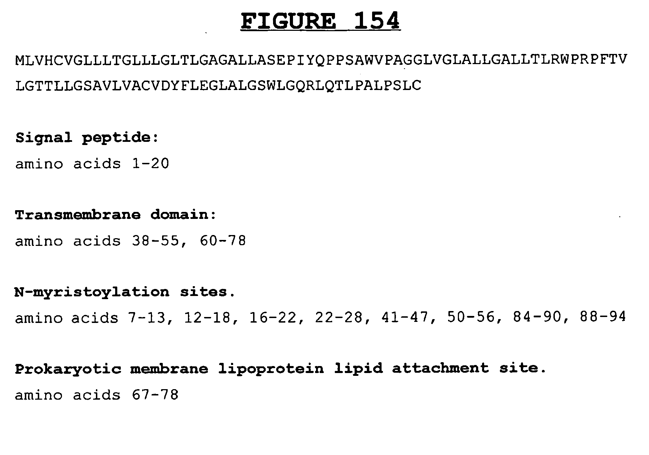

Figure 154 shows the amino acid sequence (SEQ ID NO:154) derived from the coding sequence of SEQ ID NO: 153 shown in Figure 153.

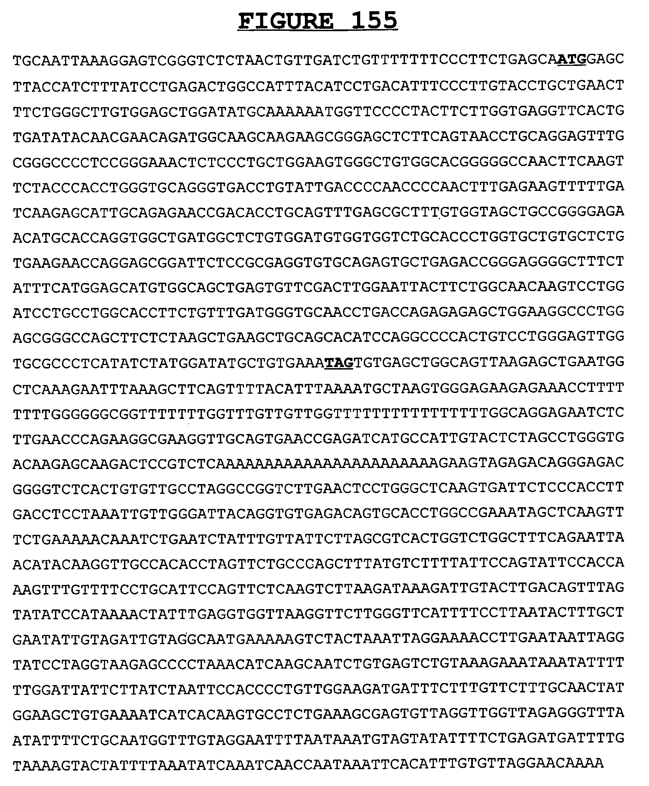

Figure 155 shows a nucleotide sequence (SEQ ID NO:155) of a native sequence PR04348 cDNA, wherein SEQ ID NO:155 is a clone designated herein as "DNA92232-2589".

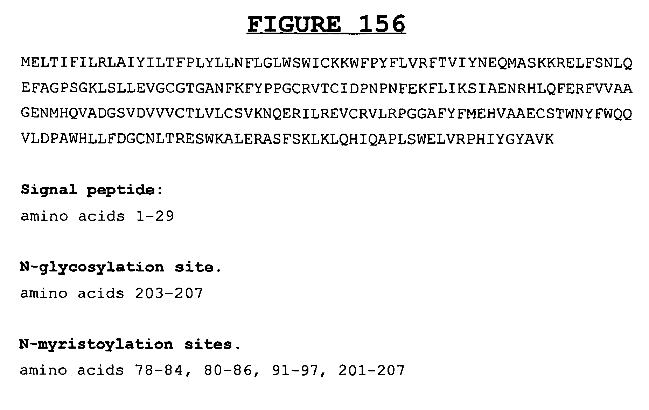

Figure 156 shows the amino acid sequence (SEQ ID NO:156) derived from the coding sequence of SEQ ID NO: 155 shown in Figure 155.

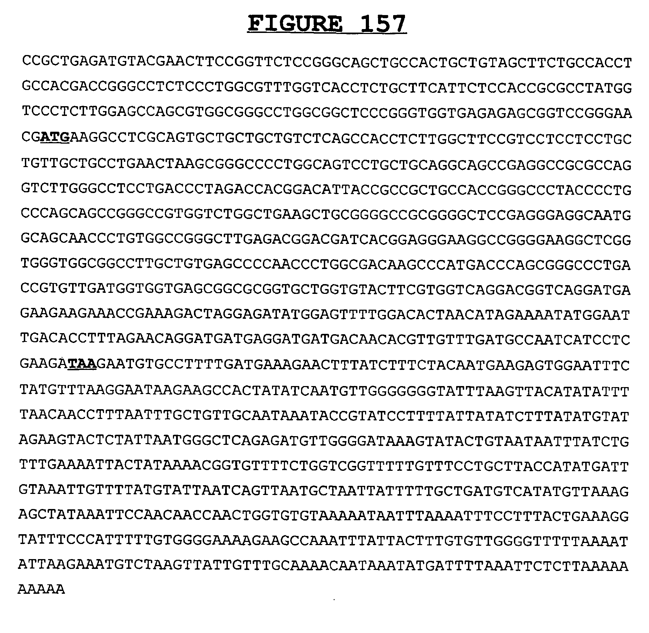

Figure 157 shows a nucleotide sequence (SEQ ID NO:157) of a native sequence PR04371 cDNA, wherein SEQ ID NO:157 is a clone designated herein as "DNA92233-2599".

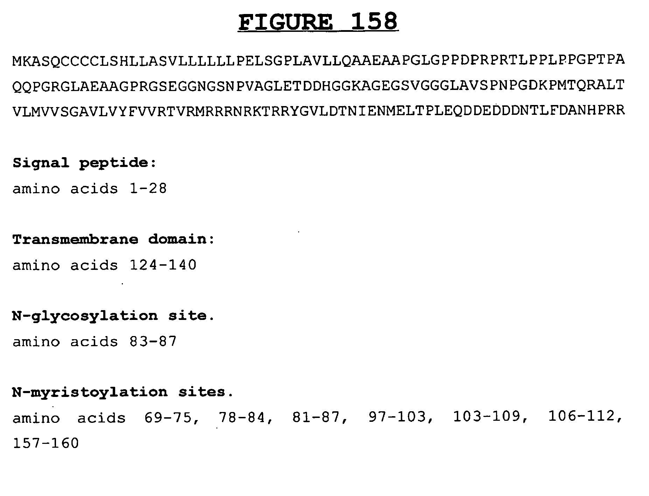

Figure 158 shows the amino acid sequence (SEQ ID NO:158) derived from the coding sequence of SEQ ID NO: 157 shown in Figure 157.

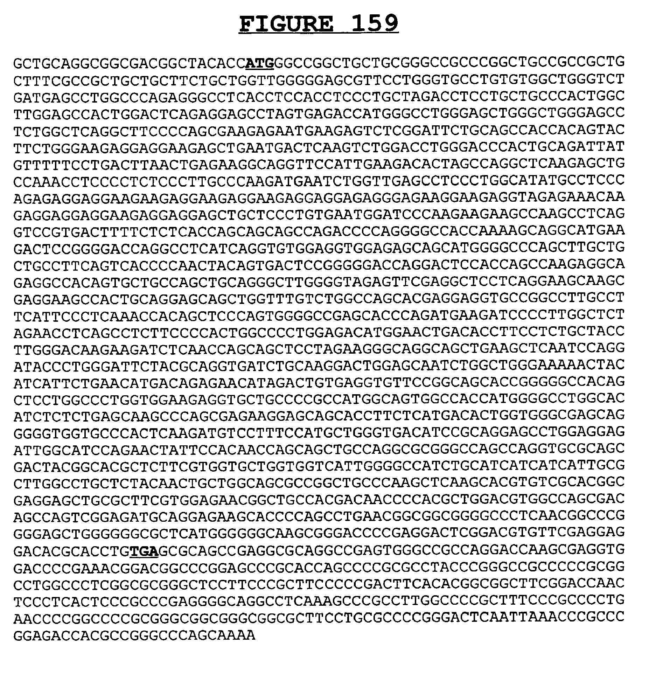

Figure 159 shows a nucleotide sequence (SEQ ID NO:159) of a native sequence PR03742 cDNA, wherein SEQ ID NO:159 is a clone designated herein as "DNA92243-2549".

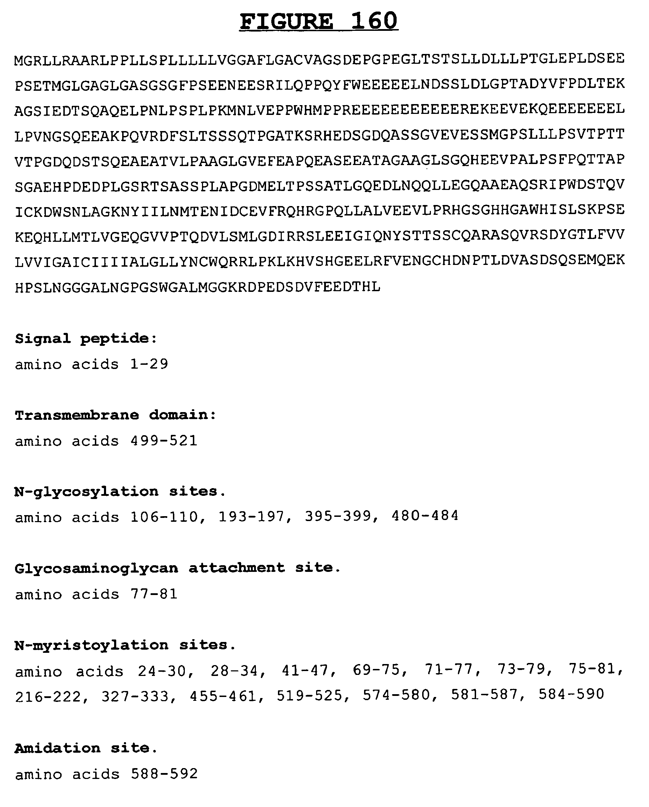

Figure 160 shows the amino acid sequence (SEQ ID NO:160) derived from the coding sequence of SEQ ID NO: 159 shown in Figure 159.

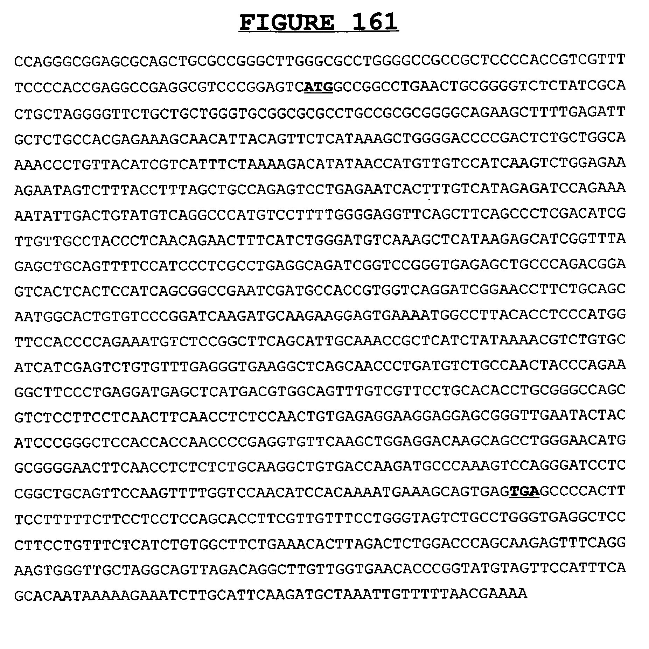

Figure 161 shows a nucleotide sequence (SEQ ID NO:161) of a native sequence PR05773 cDNA, wherein SEQ ID NO:161 is a clone designated herein as "DNA92253-2671".

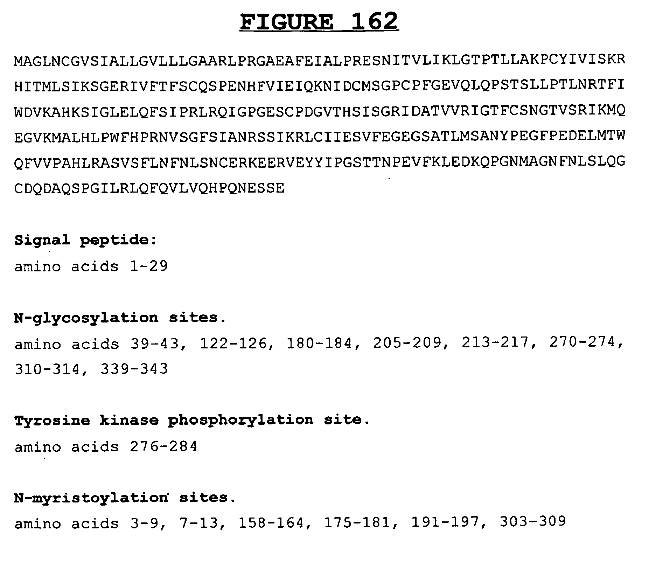

Figure 162 shows the amino acid sequence (SEQ ID NO:162) derived from the coding sequence of SEQ ID NO: 161 shown in Figure 161.

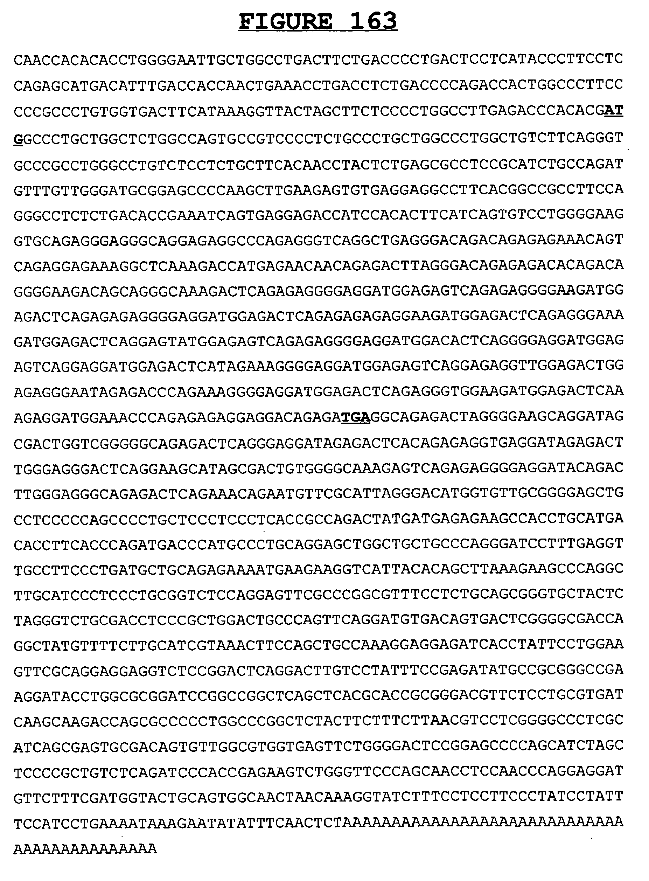

Figure 163 shows a nucleotide sequence (SEQ ID NO:163) of a native sequence PR05774 cDNA, wherein SEQ ID NO:163 is a clone designated herein as "DNA92254-2672".

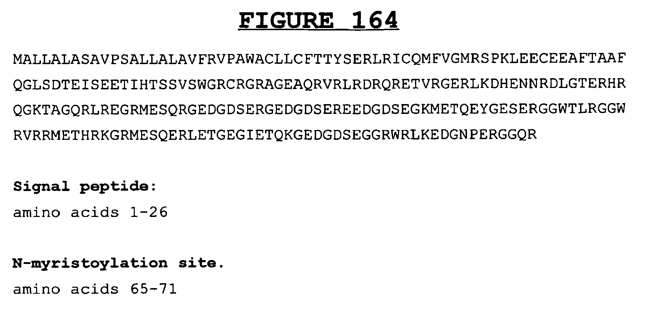

Figure 164 shows the amino acid sequence (SEQ ID NO:164) derived from the coding sequence of SEQ ID NO: 163 shown in Figure 163.

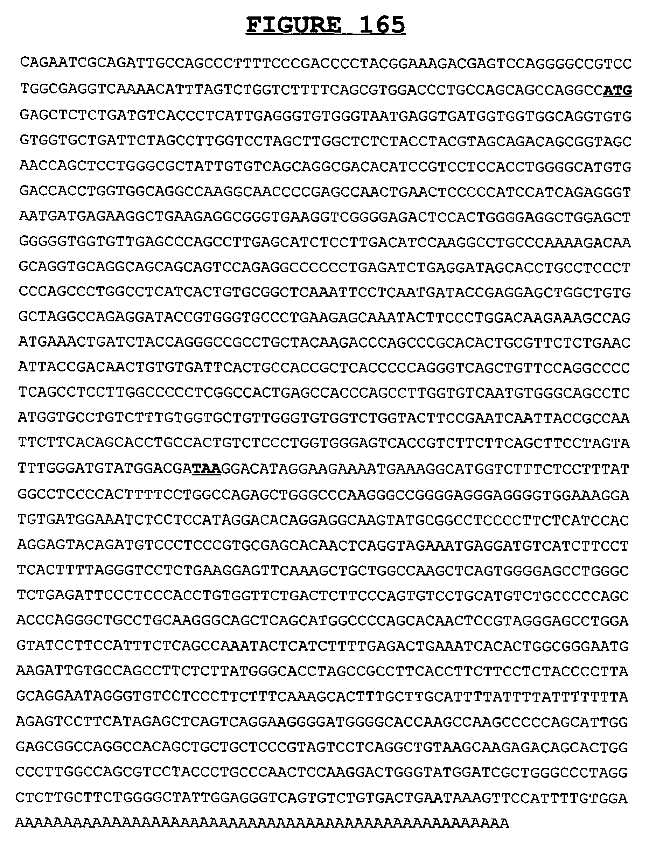

Figure 165 shows a nucleotide sequence (SEQ ID NO:165) of a native sequence PR04343 cDNA, wherein SEQ ID NO:165 is a clone designated herein as "DNA92255-2584".

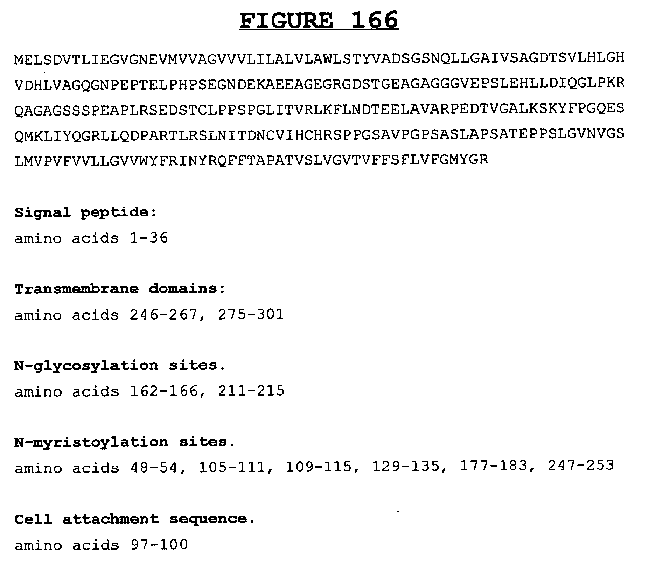

Figure 166 shows the amino acid sequence (SEQ ID NO:166) derived from the coding sequence of SEQ ID NO: 165 shown in Figure 165.

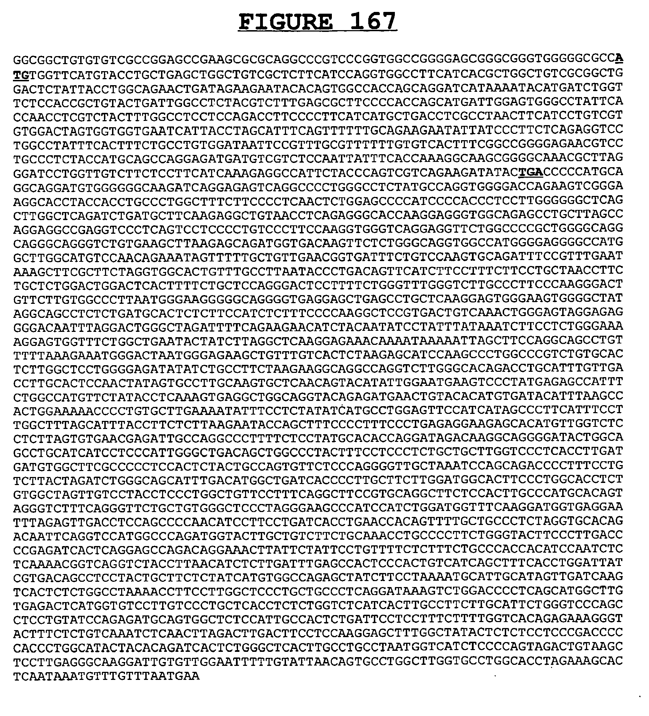

Figure 167 shows a nucleotide sequence (SEQ ID NO:167) of a native sequence PRO4325 cDNA, wherein SEQ ID NO:167 is a clone designated herein as "DNA92269-2570".

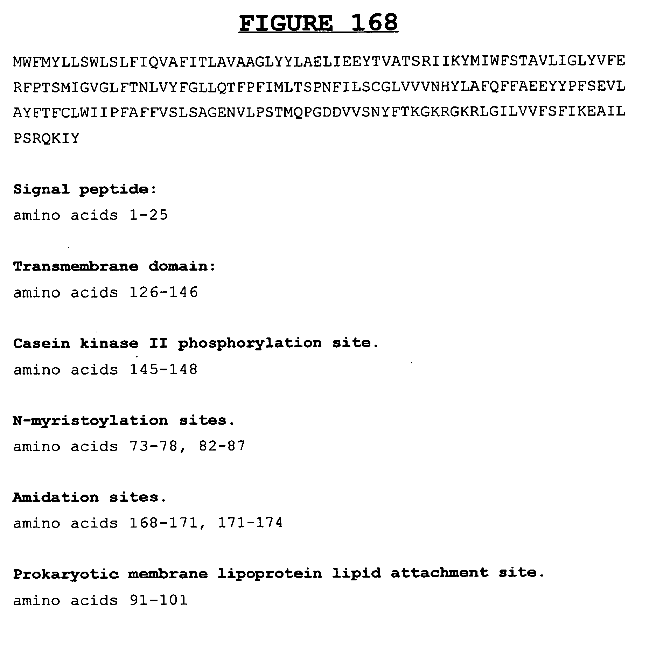

Figure 168 shows the amino acid sequence (SEQ ID NO:168) derived from the coding sequence of SEQ ID NO: 167 shown in Figure 167.

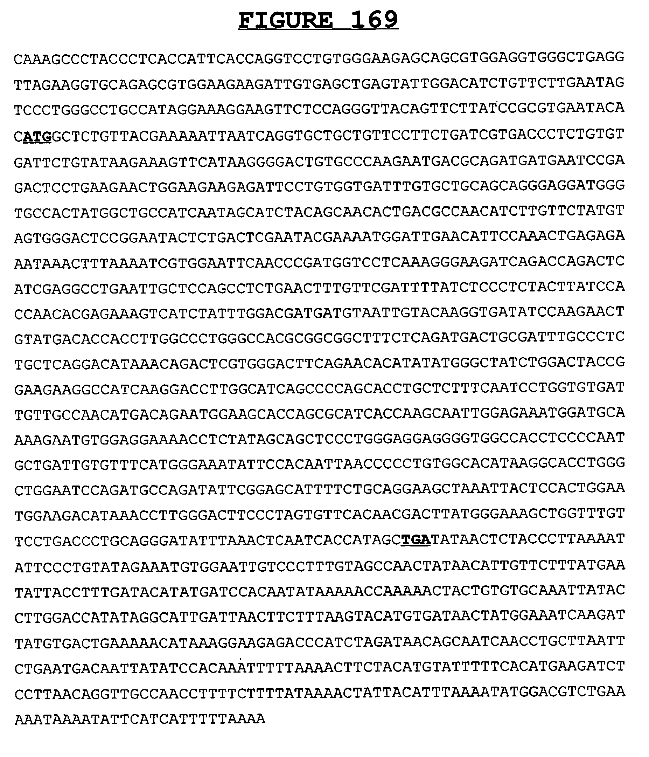

Figure 169 shows a nucleotide sequence (SEQ ID NO:169) of a native sequence PR04347 cDNA, wherein SEQ ID NO:169 is a clone designated herein as "DNA92288-2588".

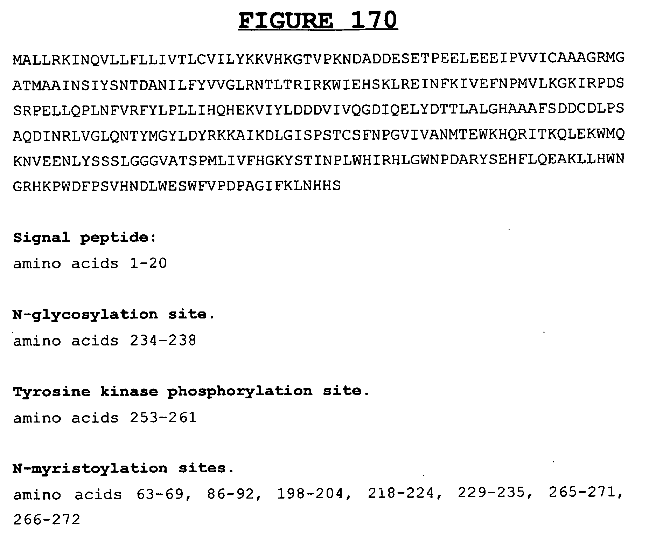

Figure 170 shows the amino acid sequence (SEQ ID NO:170) derived from the coding sequence of SEQ ID NO: 169 shown in Figure 169.

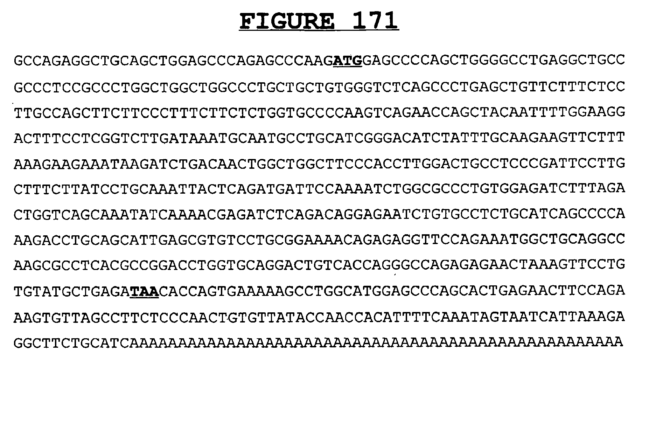

Figure 171 shows a nucleotide sequence (SEQ ID NO:171) of a native sequence PR03743 cDNA, wherein SEQ ID NO:171 is a clone designated herein as "DNA92290-2550".

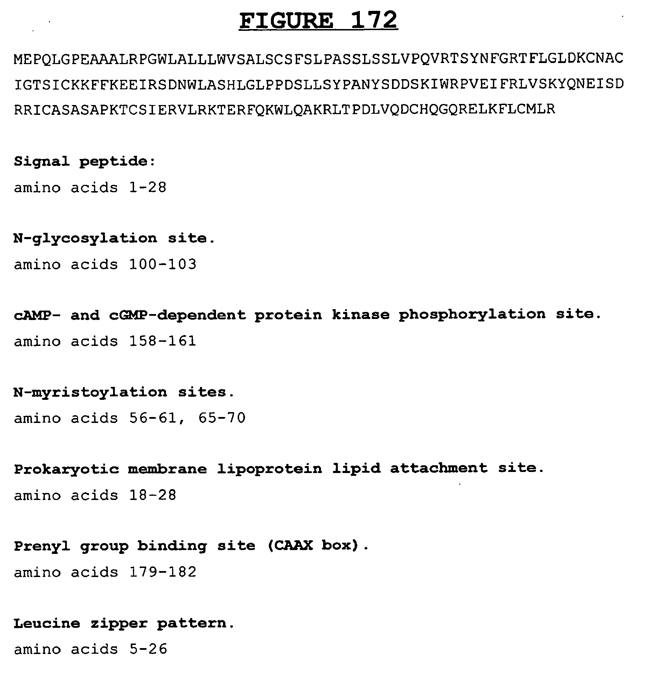

Figure 172 shows the amino acid sequence (SEQ ID NO:172) derived from the coding sequence of SEQ ID NO: 171 shown in Figure 171.

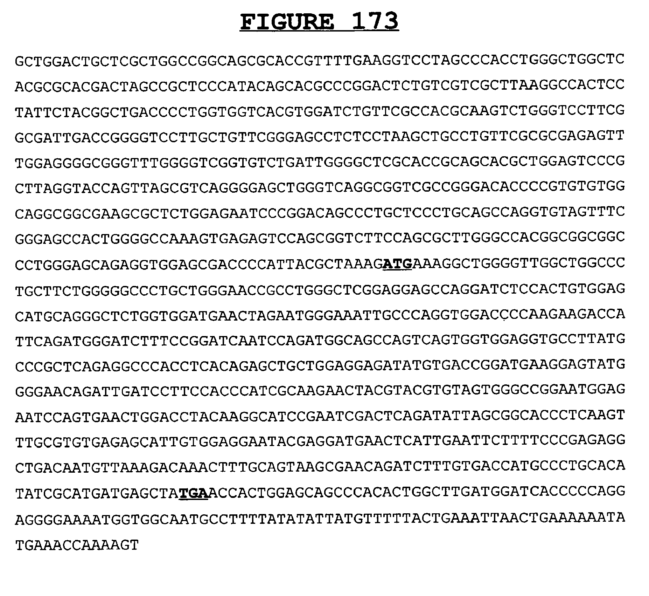

Figure 173 shows a nucleotide sequence (SEQ ID NO:173) of a native sequence PRO4426 cDNA, wherein SEQ ID NO:173 is a clone designated herein as "DNA93012-2622".

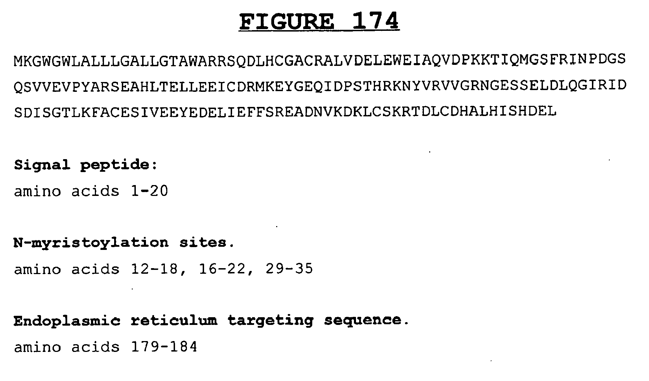

Figure 174 shows the amino acid sequence (SEQ ID NO:174) derived from the coding sequence of SEQ ID NO: 173 shown in Figure 173.

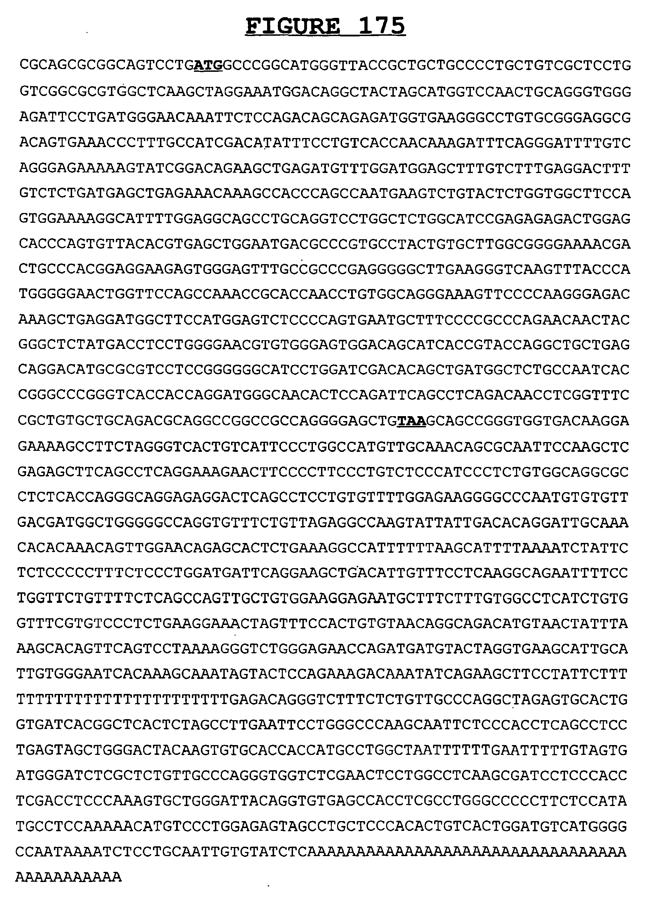

Figure 175 shows a nucleotide sequence (SEQ ID NO:175) of a native sequence PR04500 cDNA, wherein SEQ ID NO:175 is a clone designated herein as "DNA93020-2642".

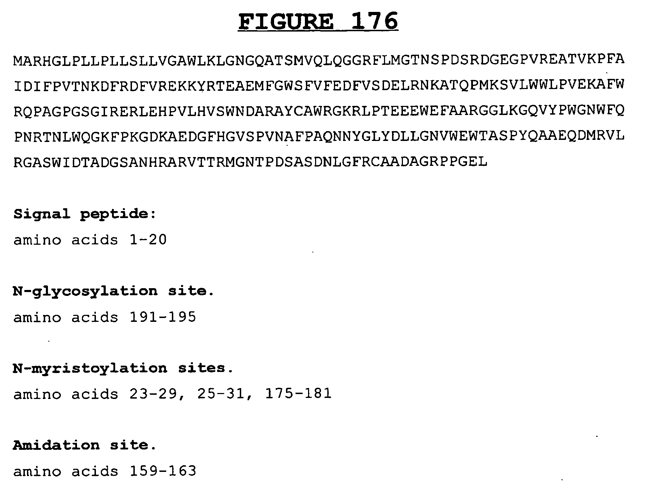

Figure 176 shows the amino acid sequence (SEQ ID NO:176) derived from the coding sequence of SEQ ID NO: 175 shown in Figure 175.

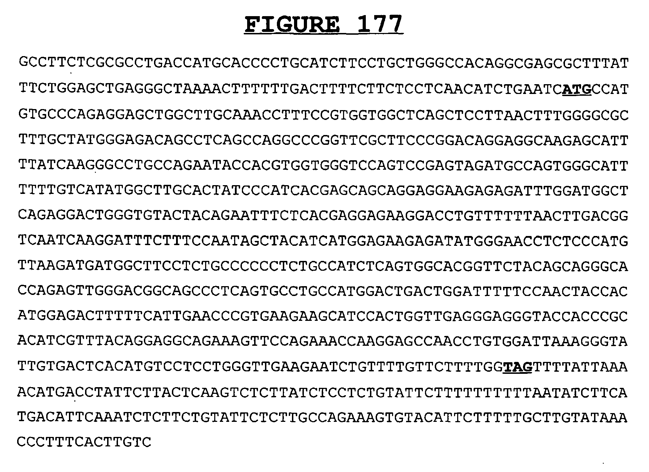

Figure 177 shows a nucleotide sequence (SEQ ID NO:177) of a native sequence PR04389 cDNA, wherein SEQ ID NO:177 is a clone designated herein as "DNA94830-2604".

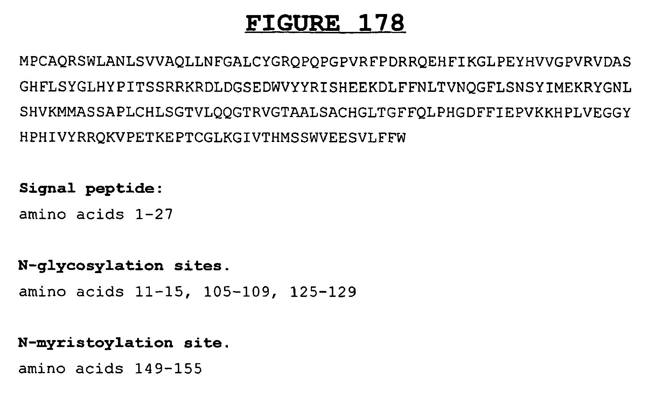

Figure 178 shows the amino acid sequence (SEQ ID NO:178) derived from the coding sequence of SEQ ID NO: 177 shown in Figure 177.

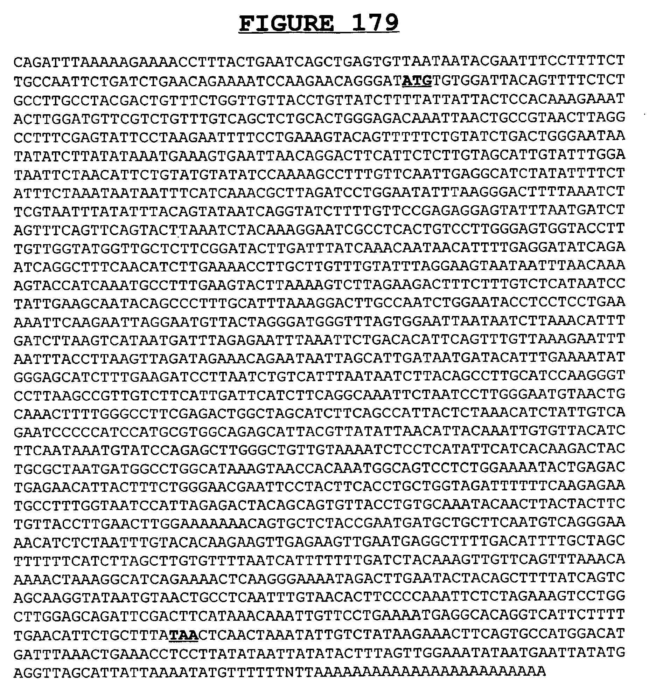

Figure 179 shows a nucleotide sequence (SEQ ID NO:179) of a native sequence PR04337 cDNA, wherein SEQ ID NO:179 is a clone designated herein as "DNA94833-2579".

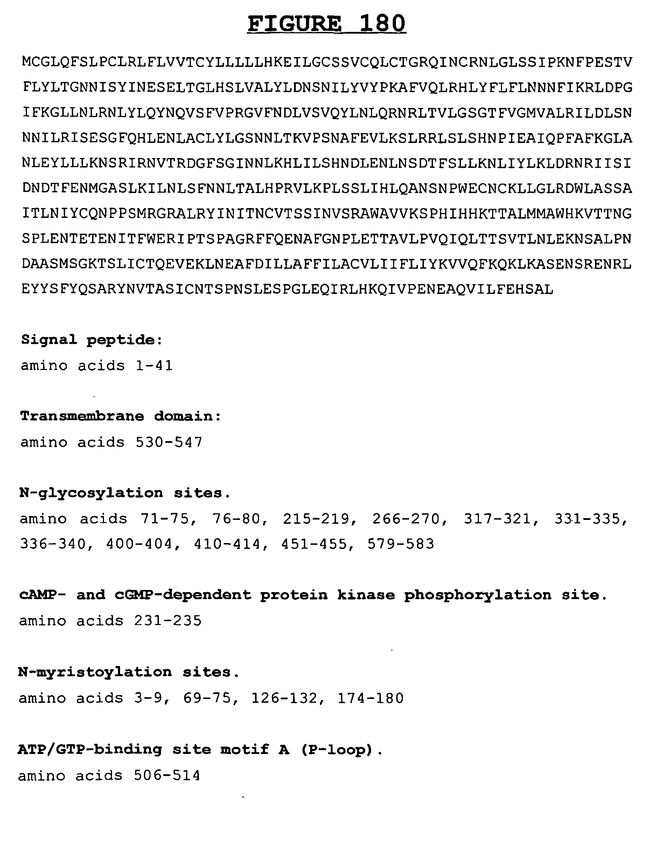

Figure 180 shows the amino acid sequence (SEQ ID NO:180) derived from the coding sequence of SEQ ID NO: 179 shown in Figure 179.

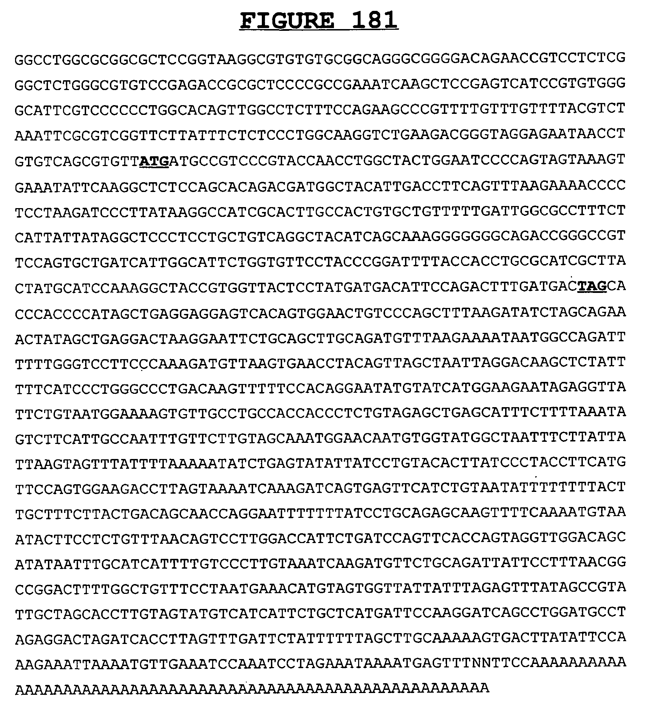

Figure 181 shows a nucleotide sequence (SEQ ID NO: 181) of a native sequence PR04992 cDNA, wherein SEQ ID NO:181 is a clone designated herein as "DNA94838-2658".

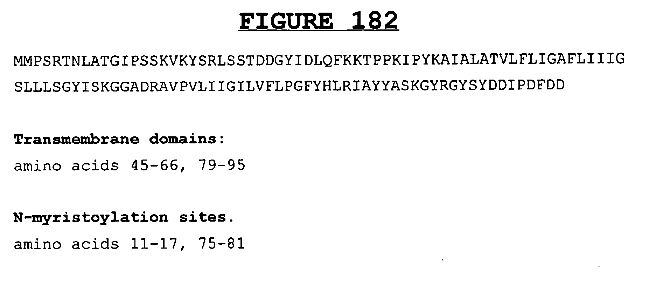

Figure 182 shows the amino acid sequence (SEQ ID NO:182) derived from the coding sequence of SEQ ID NO: 181 shown in Figure 181.

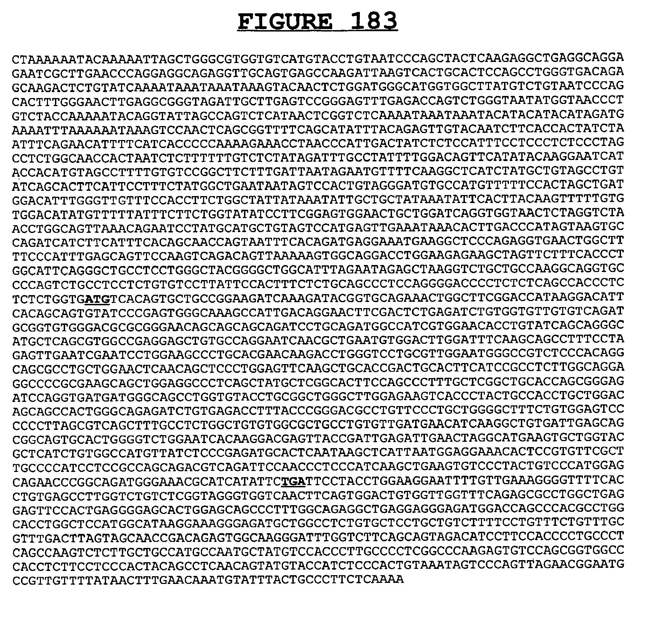

Figure 183 shows a nucleotide sequence (SEQ ID NO: 183) of a native sequence PR05996 cDNA, wherein SEQ ID NO: 183 is a clone designated herein as "DNA94844-2686".

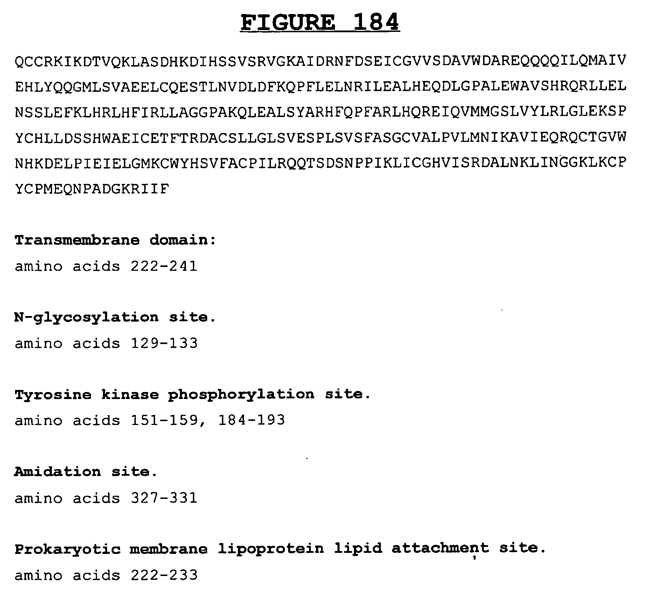

Figure 184 shows the amino acid sequence (SEQ ID NO:184) derived from the coding sequence of SEQ ID NO: 183 shown in Figure 183.

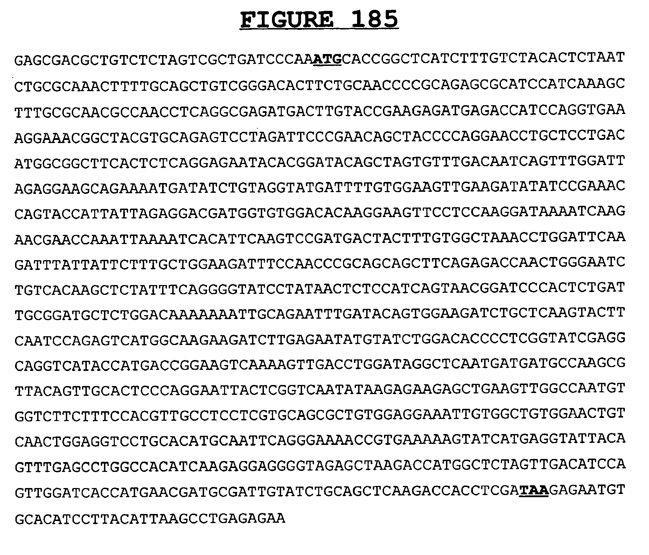

Figure 185 shows a nucleotide sequence (SEQ ID NO: 185) of a native sequence PR04345 cDNA, wherein SEQ ID NO: 185 is a clone designated herein as "DNA94854-2586".

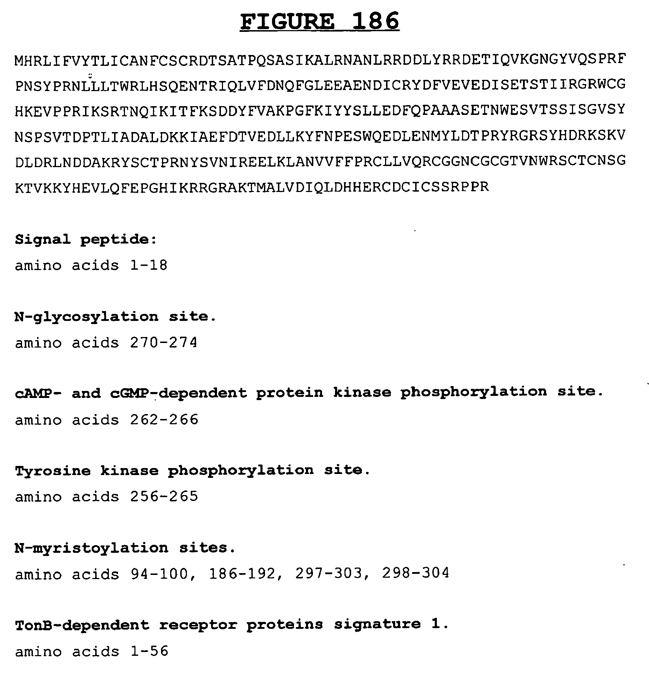

Figure 186 shows the amino acid sequence (SEQ ID NO:186) derived from the coding sequence of SEQ ID NO: 185 shown in Figure 185.

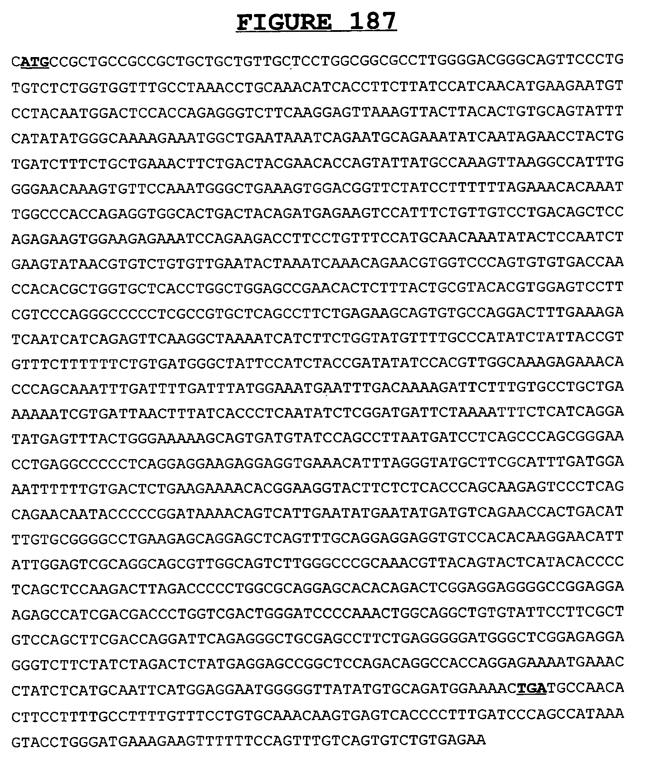

Figure 187 shows a nucleotide sequence (SEQ ID NO: 187) of a native sequence PRO4978 cDNA, wherein SEQ ID NO: 187 is a clone designated herein as "DNA95930".

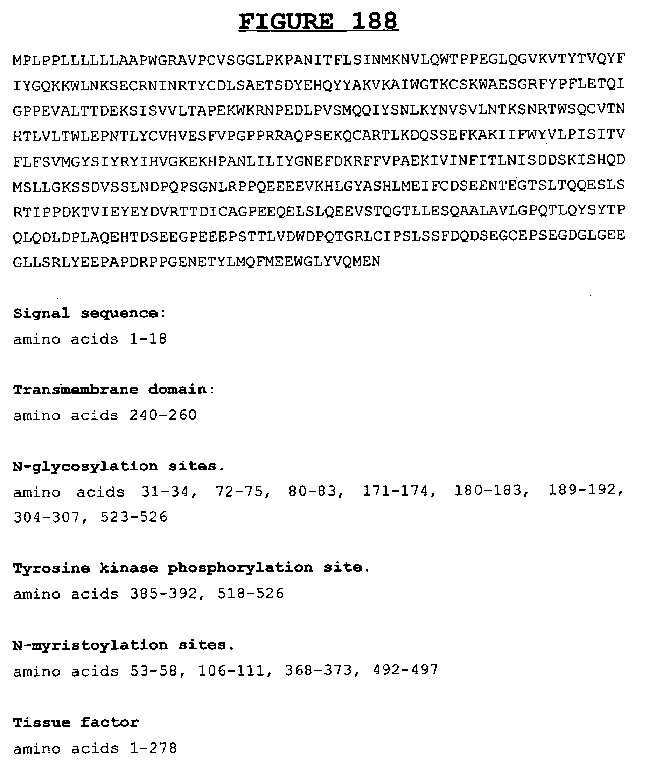

Figure 188 shows the amino acid sequence (SEQ ID NO:188) derived from the coding sequence of SEQ ID NO: 187 shown in Figure 187.

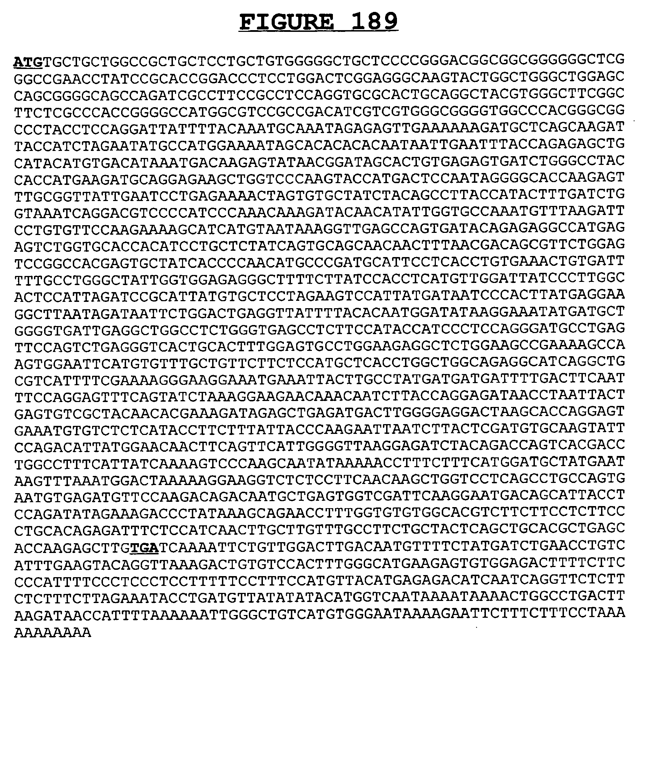

Figure 189 shows a nucleotide sequence (SEQ ID NO: 189) of a native sequence PR05780 cDNA, wherein SEQ ID NO: 189 is a clone designated herein as "DNA96868-2677".

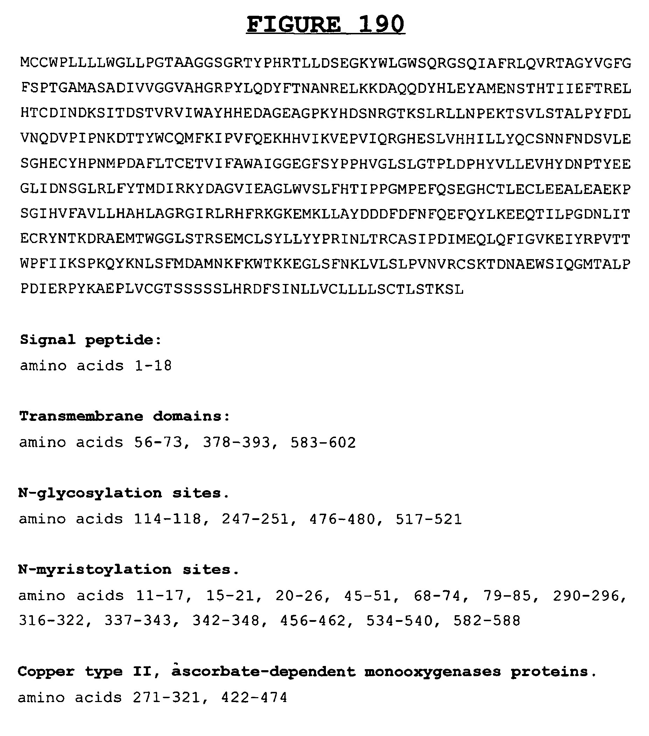

Figure 190 shows the amino acid sequence (SEQ ID NO: 190) derived from the coding sequence of SEQ ID NO: 189 shown in Figure 189.

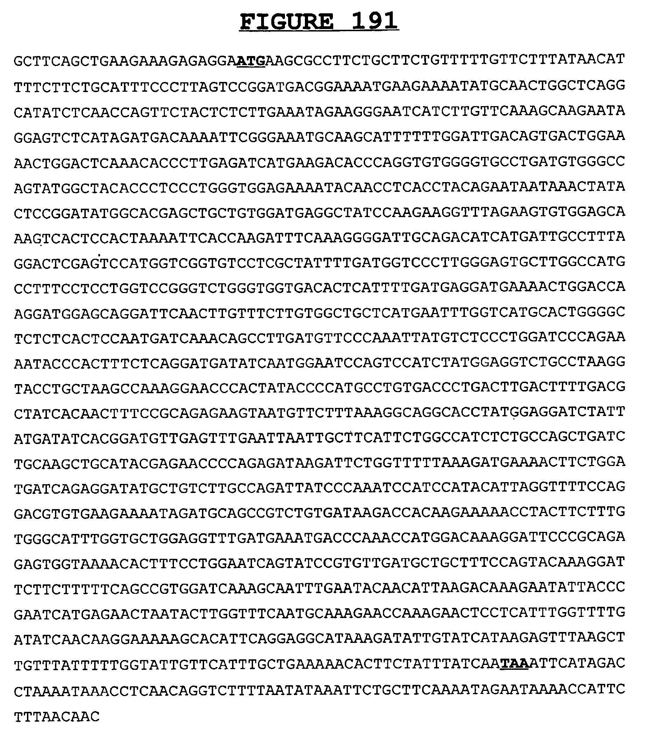

Figure 191 shows a nucleotide sequence (SEQ ID NO:191) of a native sequence PR05992 cDNA, wherein SEQ ID NO: 191 is a clone designated herein as "DNA96871-2683".

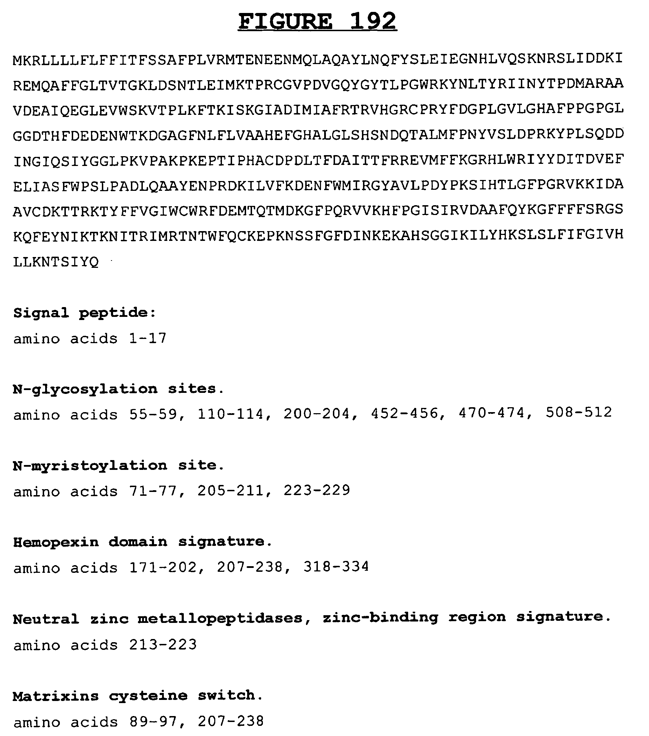

Figure 192 shows the amino acid sequence (SEQ ID NO:192) derived from the coding sequence of SEQ ID NO: 191 shown in Figure 191.

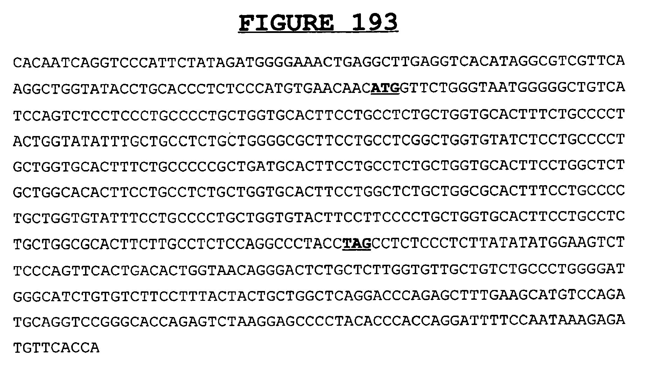

Figure 193 shows a nucleotide sequence (SEQ ID NO:193) of a native sequence PR04428 cDNA, wherein SEQ ID NO:193 is a clone designated herein as "DNA96880-2624".

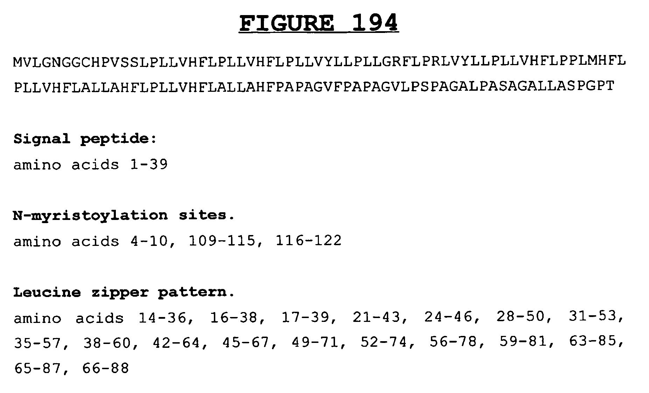

Figure 194 shows the amino acid sequence (SEQ ID NO:194) derived from the coding sequence of SEQ ID NO: 193 shown in Figure 193.

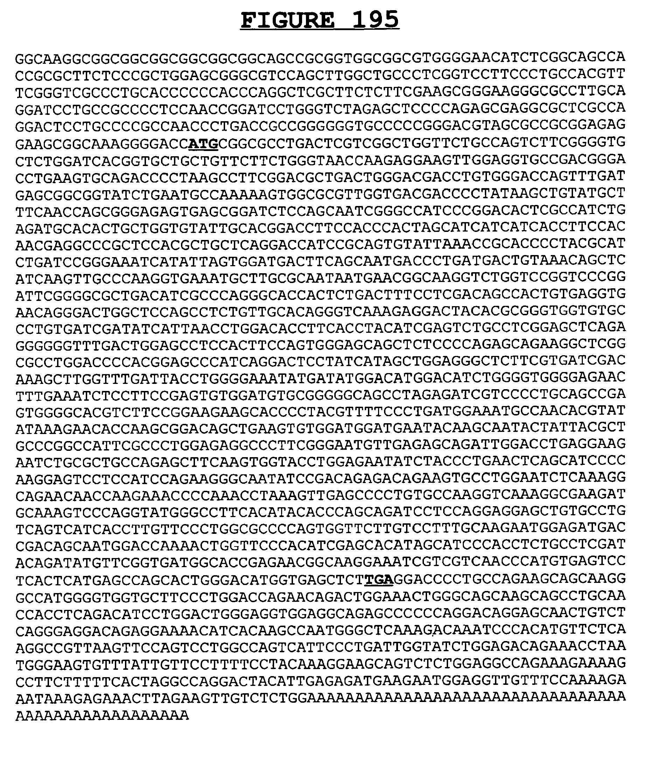

Figure 195 shows a nucleotide sequence (SEQ ID NO:195) of a native sequence PR04994 cDNA, wherein SEQ ID NO:195 is a clone designated herein as "DNA96986-2660".

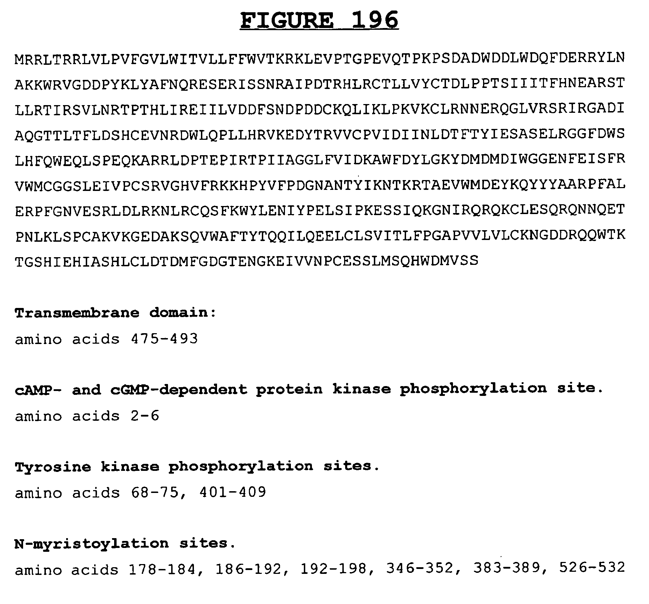

Figure 196 shows the amino acid sequence (SEQ ID NO:196) derived from the coding sequence of SEQ ID NO: 195 shown in Figure 195.

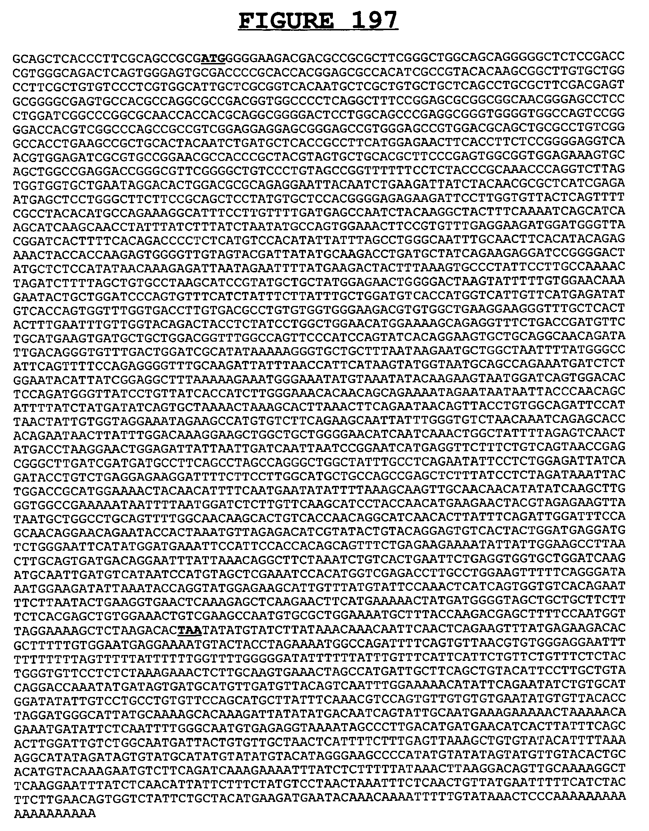

Figure 197 shows a nucleotide sequence (SEQ ID NO:197) of a native sequence PR05995 cDNA, wherein SEQ ID NO:197 is a clone designated herein as "DNA96988-2685".

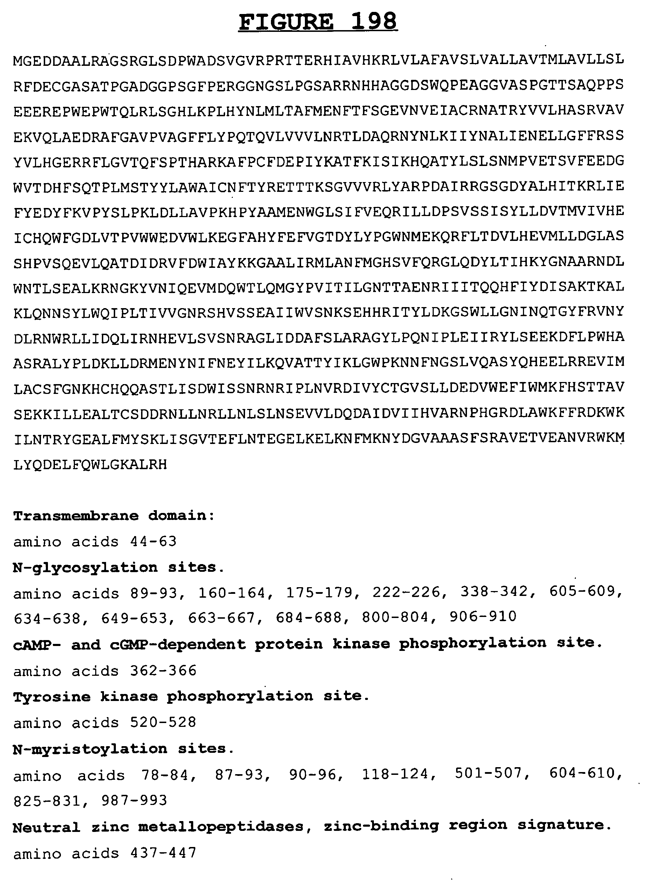

Figure 198 shows the amino acid sequence (SEQ ID NO:198) derived from the coding sequence of SEQ ID NO: 197 shown in Figure 197.

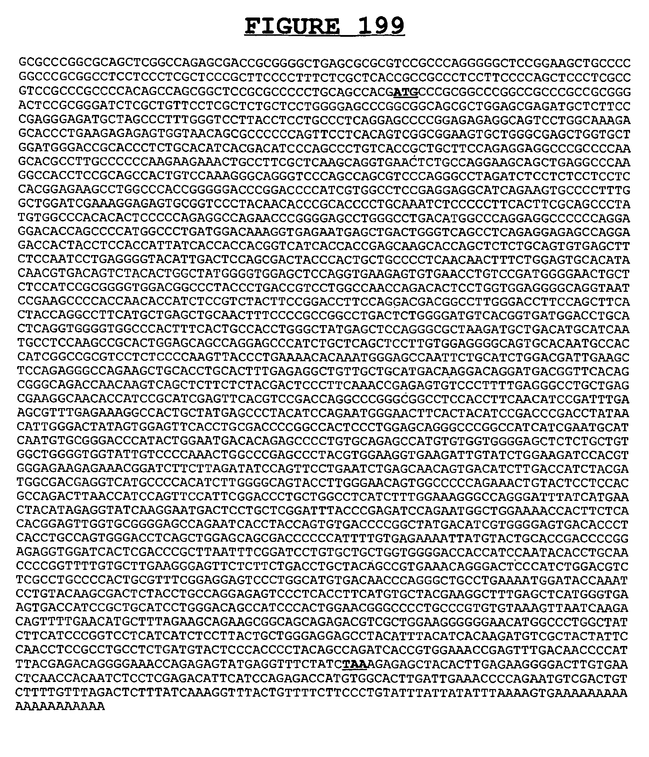

Figure 199 shows a nucleotide sequence (SEQ ID NO:199) of a native sequence PRO6094 cDNA, wherein SEQ ID NO:199 is a clone designated herein as "DNA96995-2709".

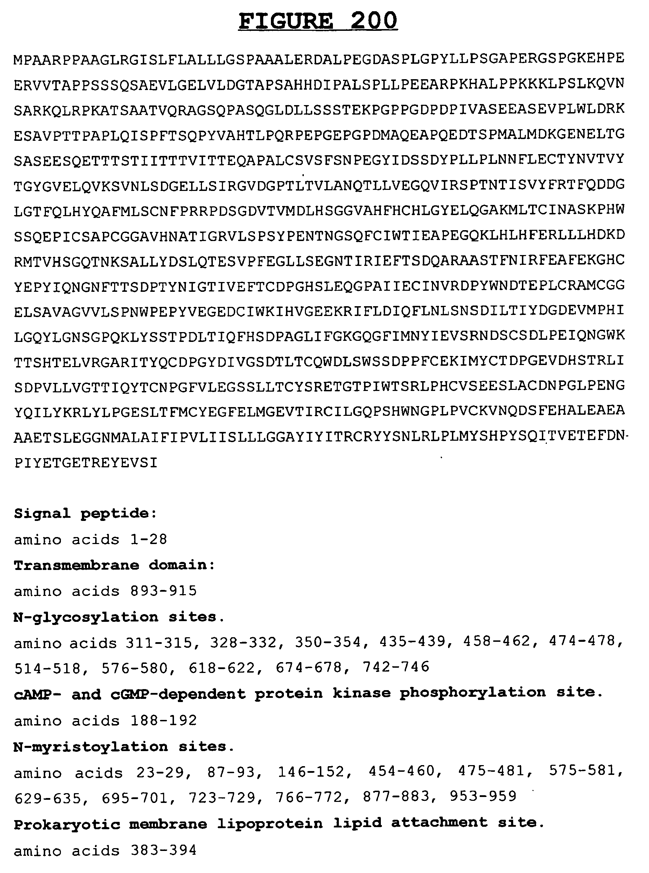

Figure 200 shows the amino acid sequence (SEQ ID NO:200) derived from the coding sequence of SEQ ID NO: 199 shown in Figure 199.

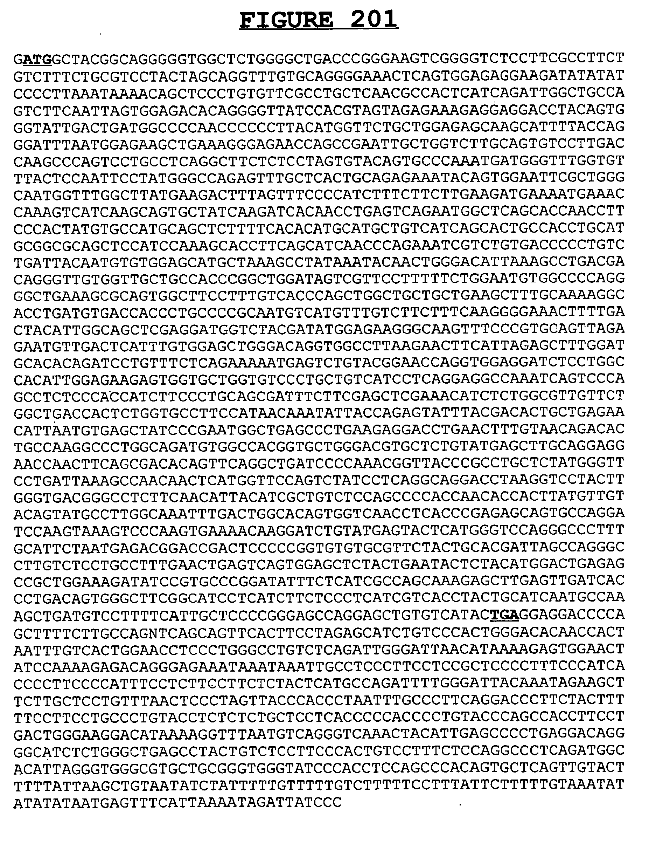

Figure 201 shows a nucleotide sequence (SEQ ID NO:201) of a native sequence PR04317 cDNA, wherein SEQ ID NO:201 is a clone designated herein as "DNA97004-2562".

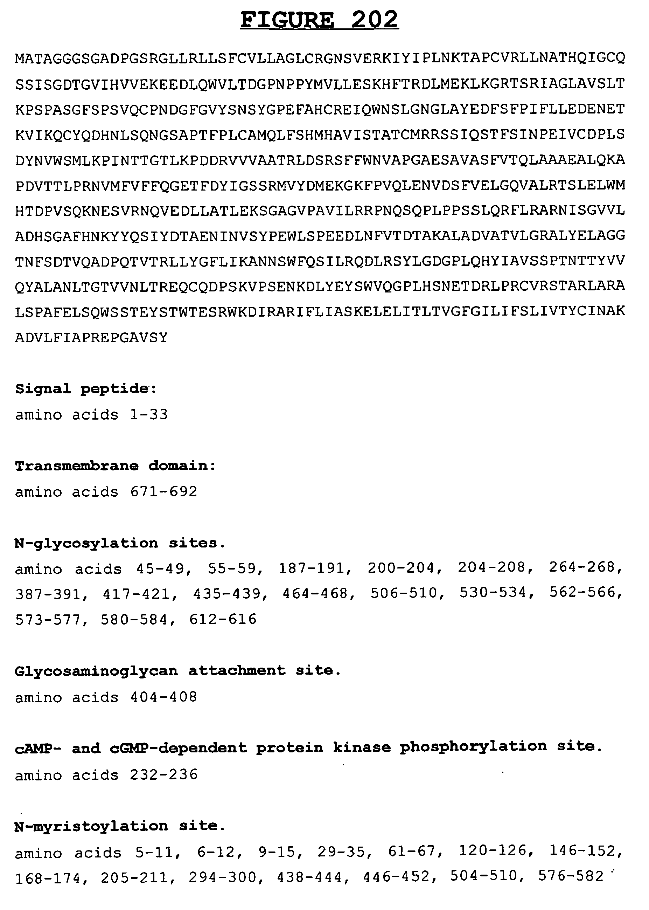

Figure 202 shows the amino acid sequence (SEQ ID NO:202) derived from the coding sequence of SEQ ID NO: 201 shown in Figure 201.

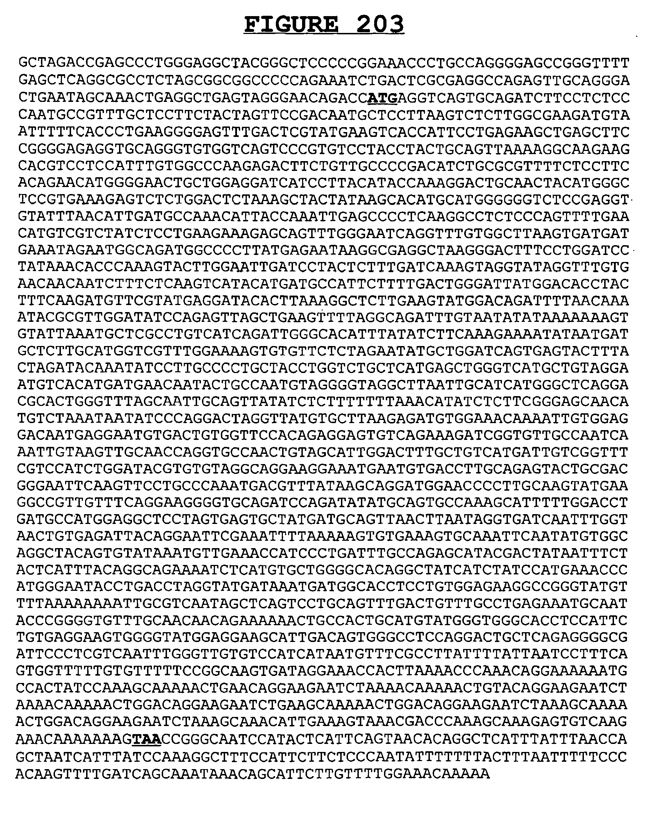

Figure 203 shows a nucleotide sequence (SEQ ID NO:203) of a native sequence PR05997 cDNA, wherein SEQ ID NO:203 is a clone designated herein as "DNA97005-2687".

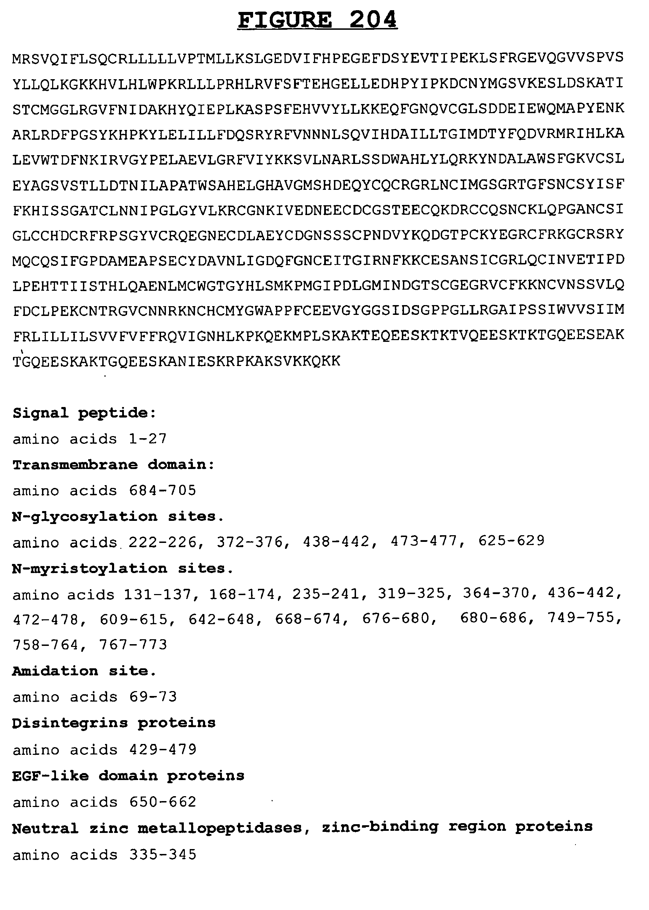

Figure 204 shows the amino acid sequence (SEQ ID NO:204) derived from the coding sequence of SEQ ID NO: 203 shown in Figure 203.

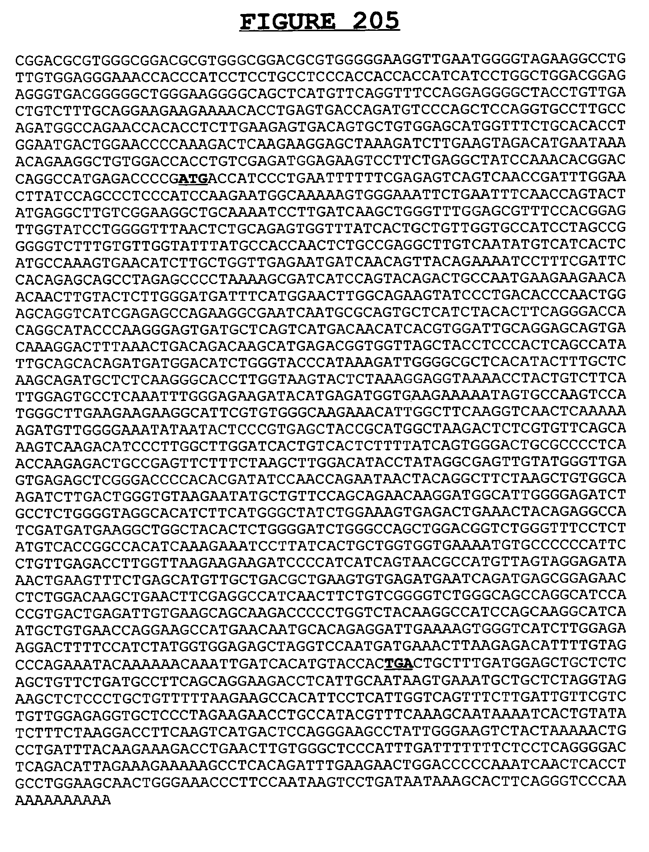

Figure 205 shows a nucleotide sequence (SEQ ID NO:205) of a native sequence PRO5005 cDNA, wherein SEQ ID NO:205 is a clone designated herein as "DNA97009-2668".

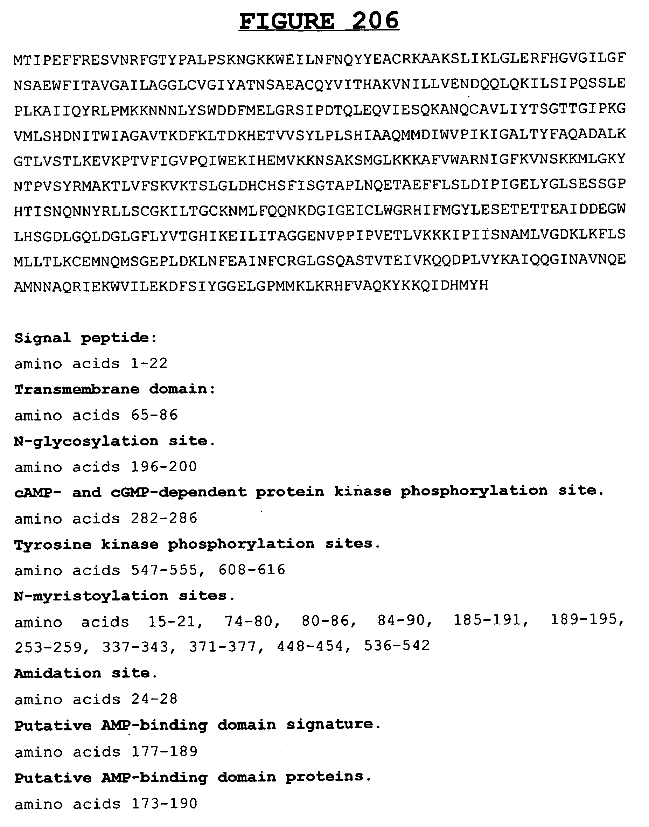

Figure 206 shows the amino acid sequence (SEQ ID NO:206) derived from the coding sequence of SEQ ID NO: 205 shown in Figure 205.

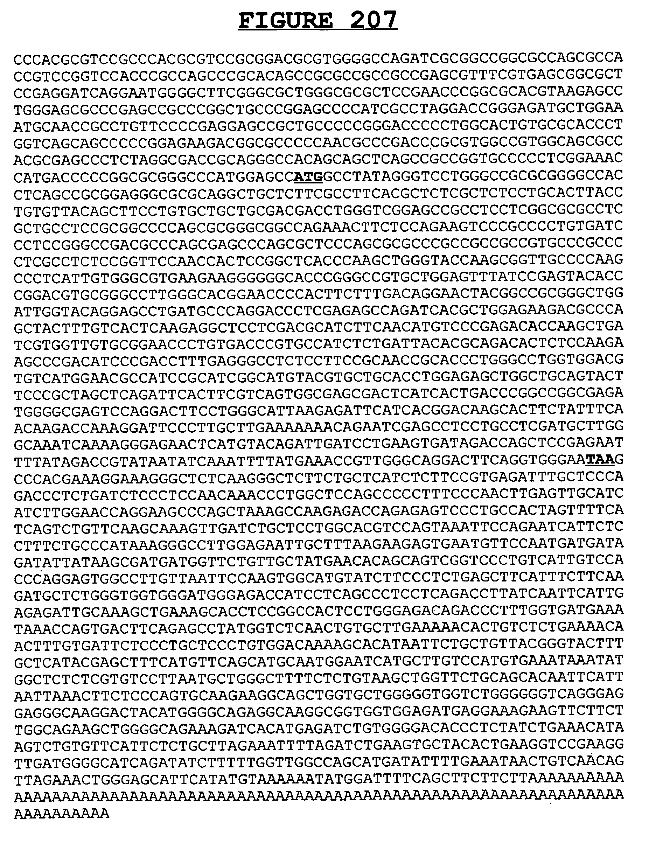

Figure 207 shows a nucleotide sequence (SEQ ID NO:207) of a native sequence PR05004 cDNA, wherein SEQ ID NO:207 is a clone designated herein as "DNA97013-2667".

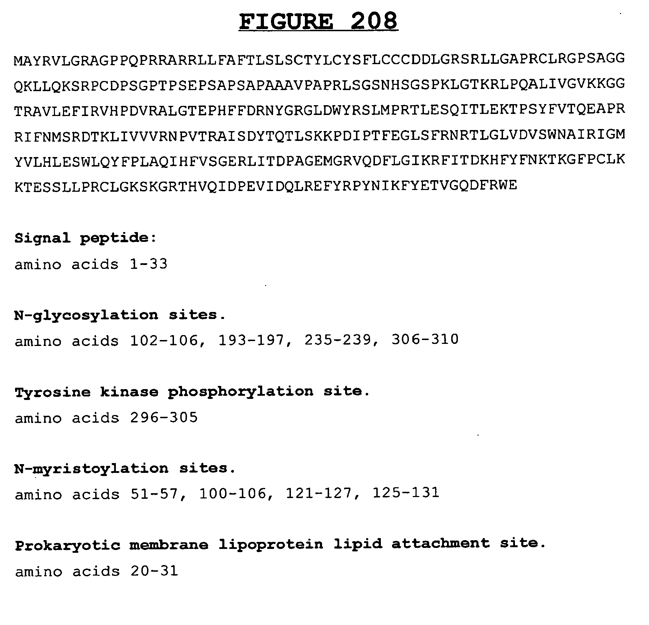

Figure 208 shows the amino acid sequence (SEQ ID NO:208) derived from the coding sequence of SEQ ID NO: 207 shown in Figure 207.

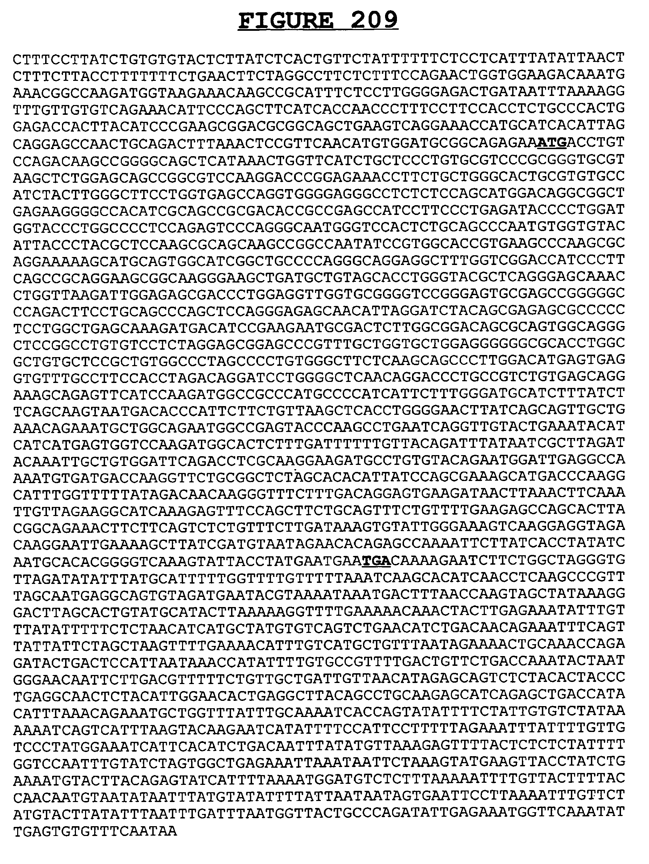

Figure 209 shows a nucleotide sequence (SEQ ID NO:209) of a native sequence PRO6001 cDNA, wherein SEQ ID NO:209 is a clone designated herein as "DNA98380-2690".

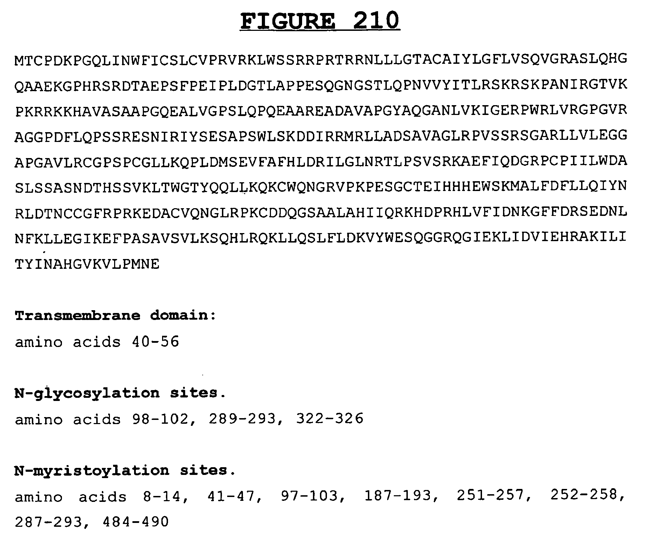

Figure 210 shows the amino acid sequence (SEQ ID NO:210) derived from the coding sequence of SEQ ID NO: 209 shown in Figure 209.

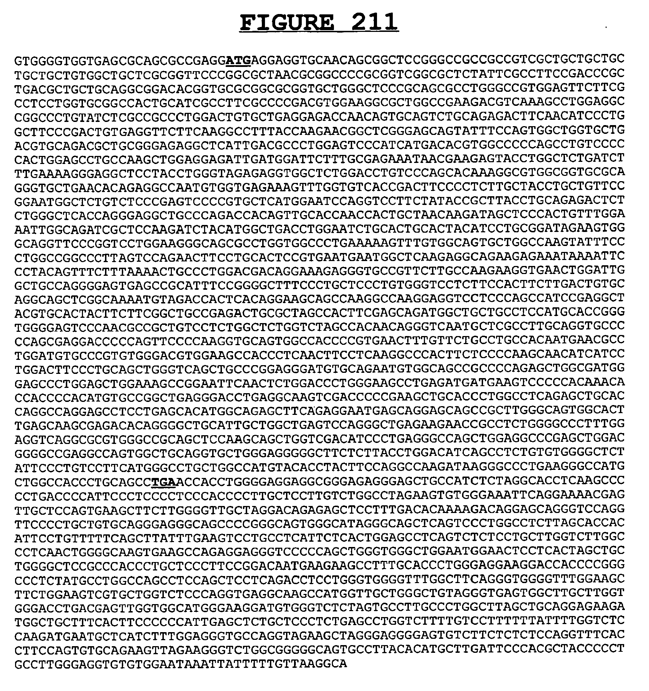

Figure 211 shows a nucleotide sequence (SEQ ID NO:211) of a native sequence PRO6013 cDNA, wherein SEQ ID NO:211 is a clone designated herein as "DNA98561-2696".

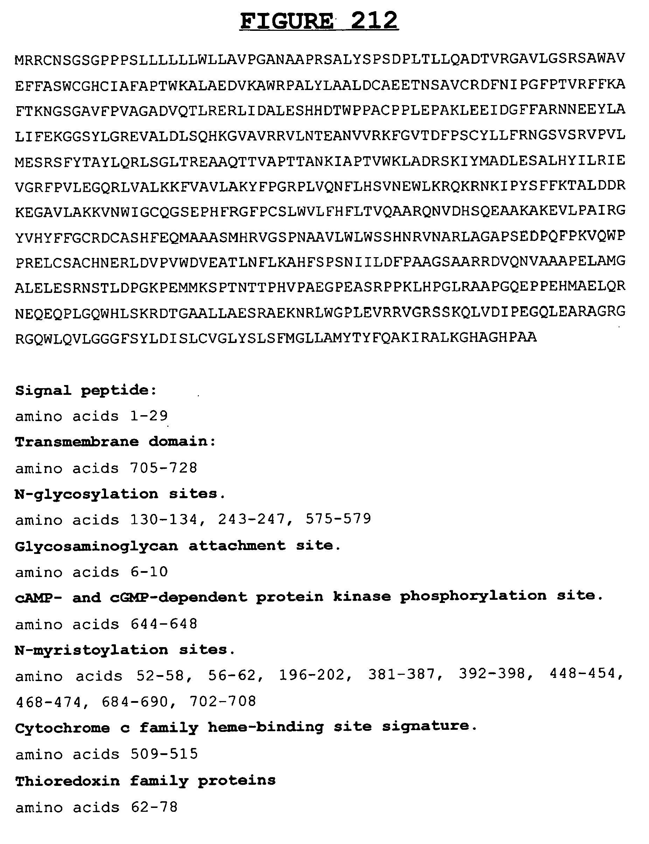

Figure 212 shows the amino acid sequence (SEQ ID NO:212) derived from the coding sequence of SEQ ID NO: 211 shown in Figure 211.

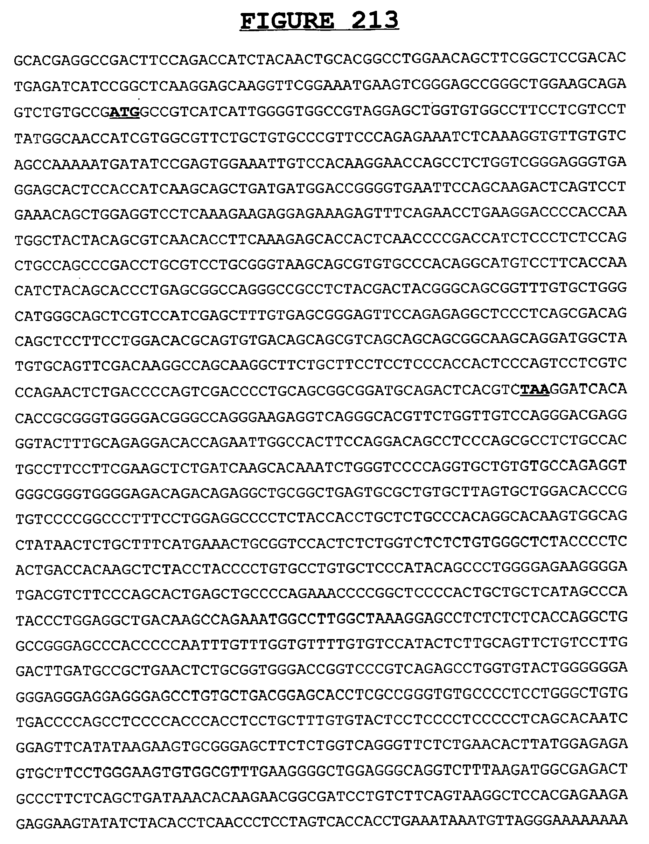

Figure 213 shows a nucleotide sequence (SEQ ID NO:213) of a native sequence PR04502 cDNA, wherein SEQ ID NO:213 is a clone designated herein as "DNA98575-2644".

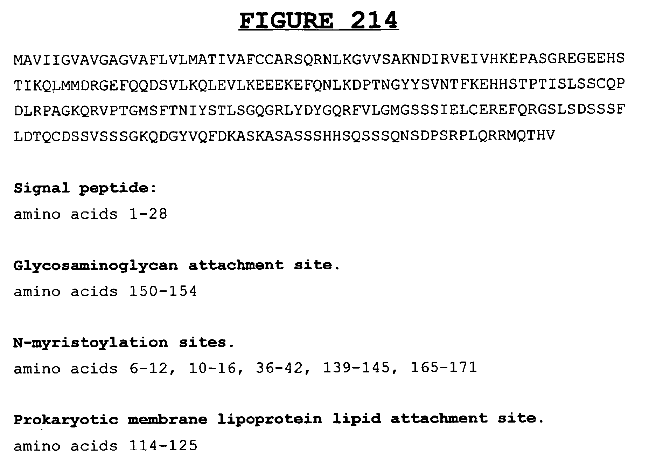

Figure 214 shows the amino acid sequence (SEQ ID NO:214) derived from the coding sequence of SEQ ID NO: 213 shown in Figure 213.

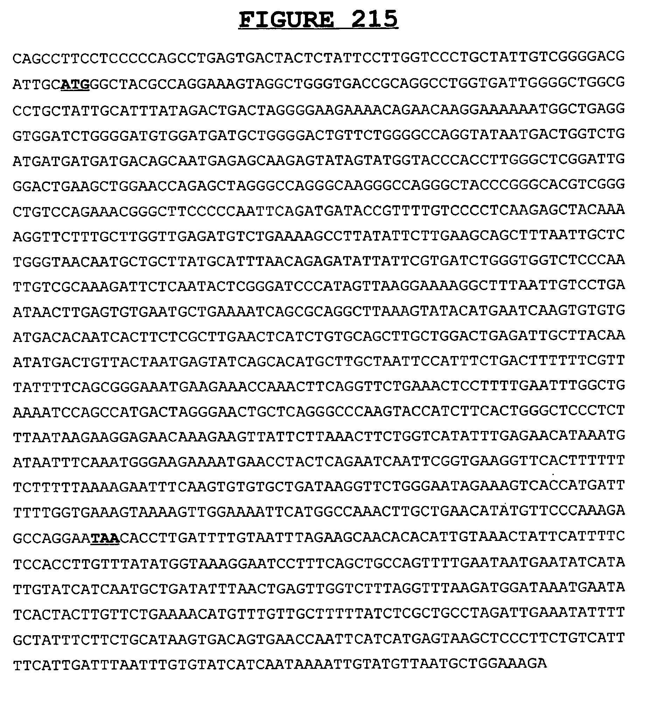

Figure 215 shows a nucleotide sequence (SEQ ID NO:215) of a native sequence PRO6007 cDNA, wherein SEQ ID NO:215 is a clone designated herein as "DNA98593-2694".

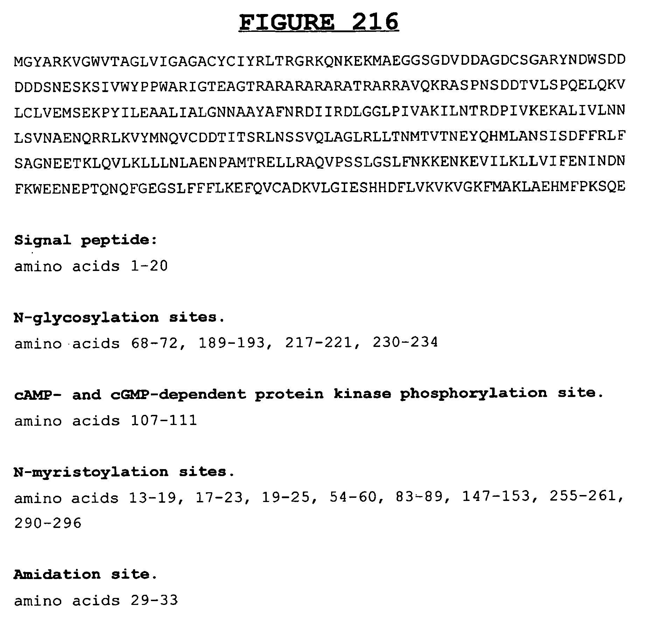

Figure 216 shows the amino acid sequence (SEQ ID NO:216) derived from the coding sequence of **SEQ** ID NO: 215 shown in Figure 215.

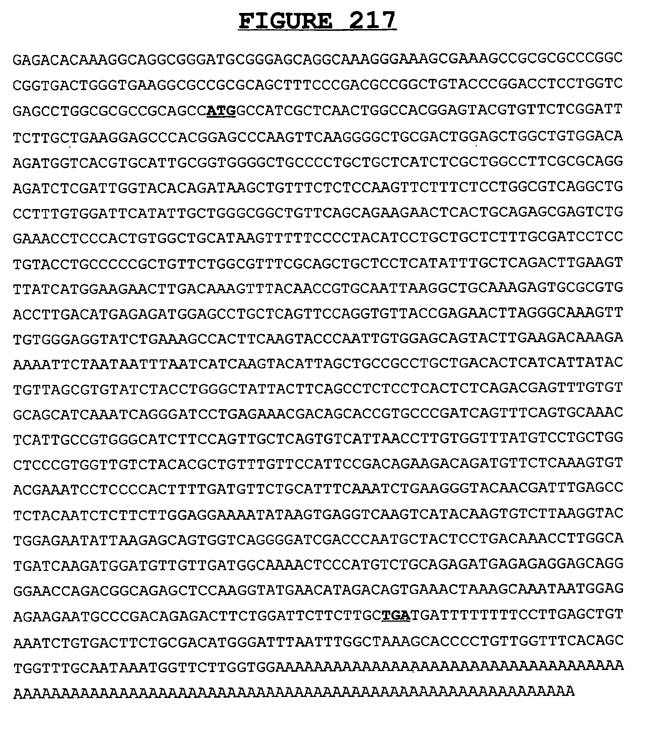

Figure 217 shows a nucleotide sequence (SEQ ID NO:217) of a native sequence PR06028 cDNA, wherein SEQ ID NO:217 is a clone designated herein as "DNA98600-2703".

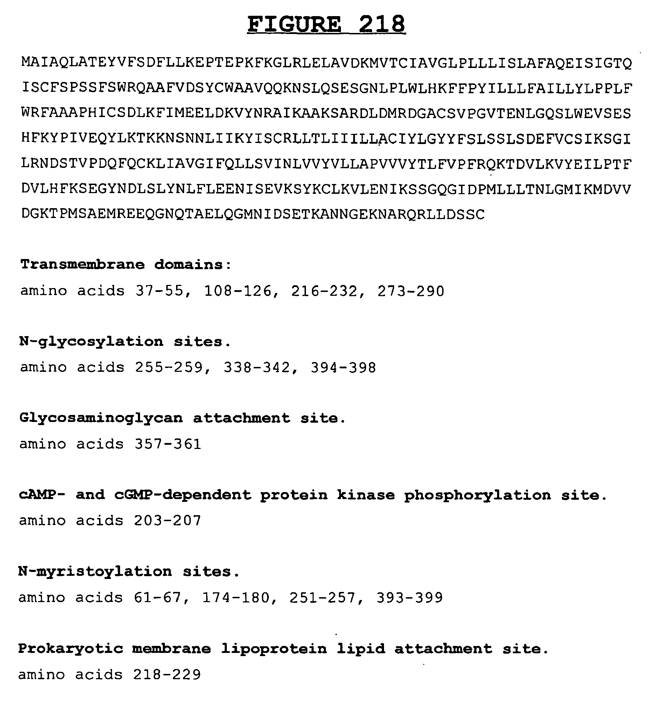

Figure 218 shows the amino acid sequence (SEQ ID NO:218) derived from the coding sequence of SEQ ID NO: 217 shown in Figure 217.

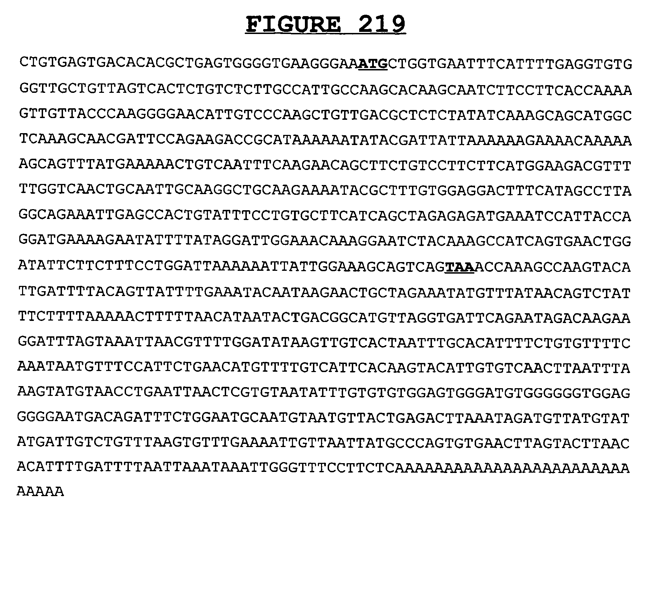

Figure 219 shows a nucleotide sequence (SEQ ID NO:219) of a native sequence PRO100 cDNA, wherein SEQ ID NO:219 is a clone designated herein as "DNA99333".

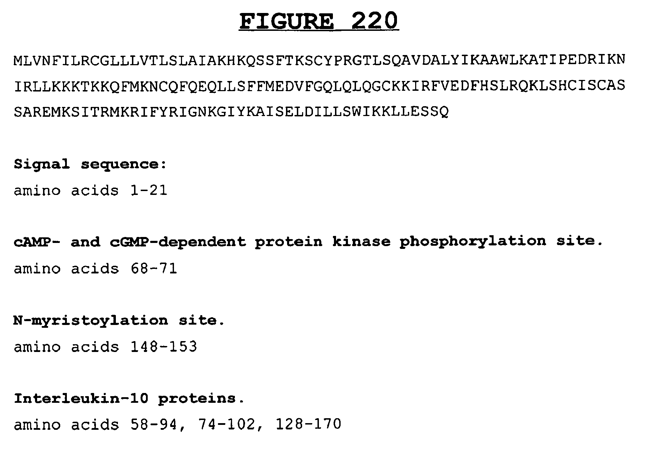

Figure 220 shows the amino acid sequence (SEQ ID NO:220) derived from the coding sequence of SEQ ID NO: 219 shown in Figure 219.

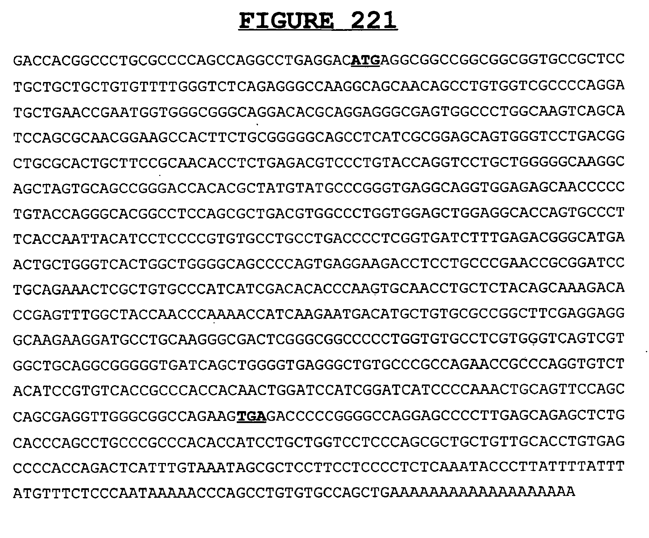

Figure 221 shows a nucleotide sequence (SEQ ID NO:221) of a native sequence PR04327 cDNA, wherein SEQ ID NO:221 is a clone designated herein as "DNA99391-2572".

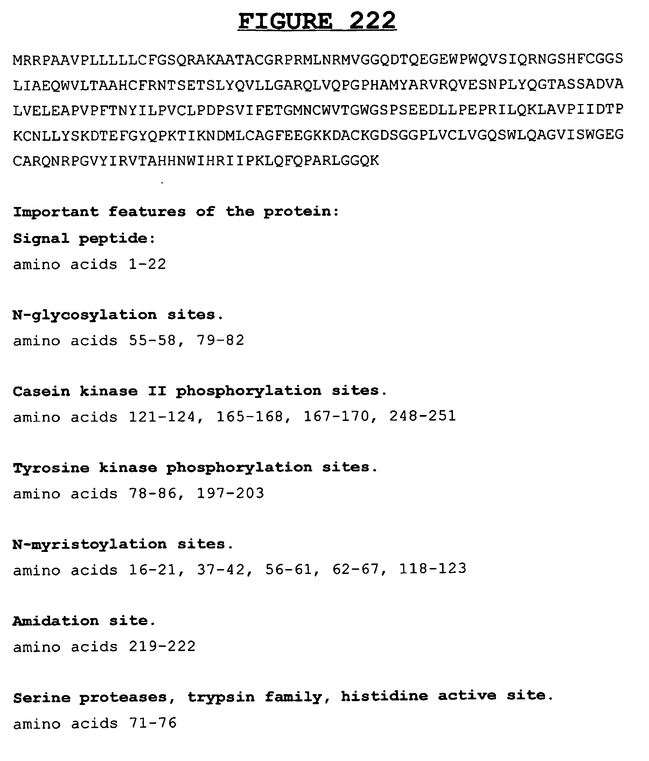

Figure 222 shows the amino acid sequence (SEQ ID NO:222) derived from the coding sequence of SEQ ID NO: 221 shown in Figure 221.

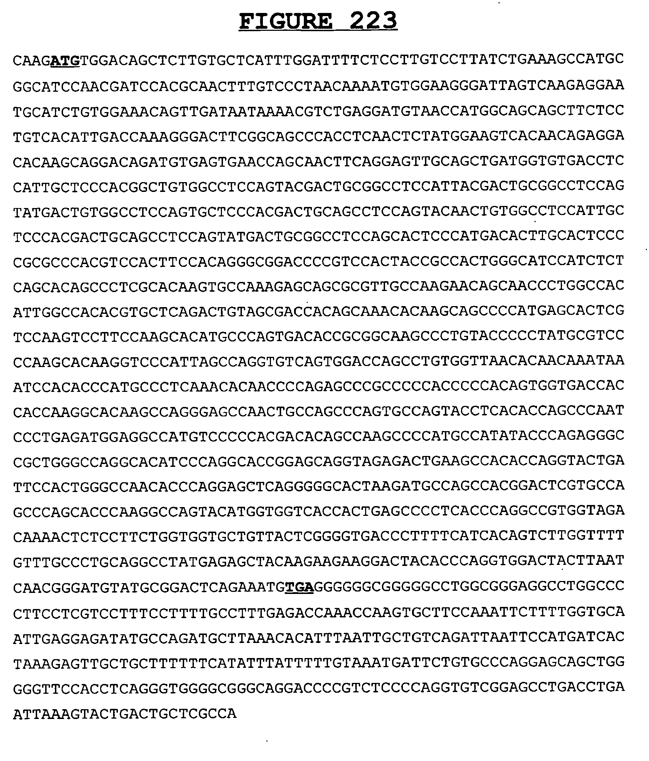

Figure 223 shows a nucleotide sequence (SEQ ID NO:223) of a native sequence PR04315 cDNA, wherein SEQ ID NO:223 is a clone designated herein as "DNA99393-2560".

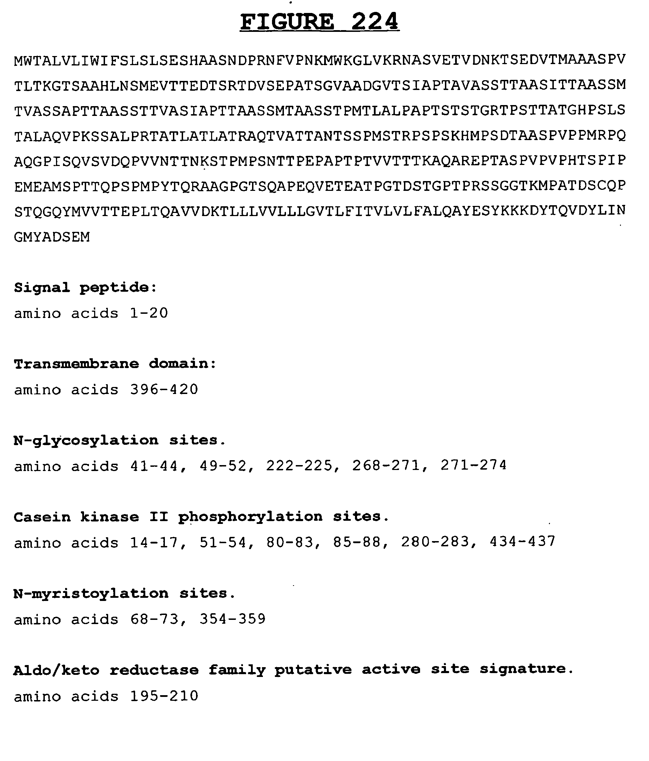

Figure 224 shows the amino acid sequence (SEQ ID NO:224) derived from the coding sequence of SEQ ID NO: 223 shown in Figure 223.

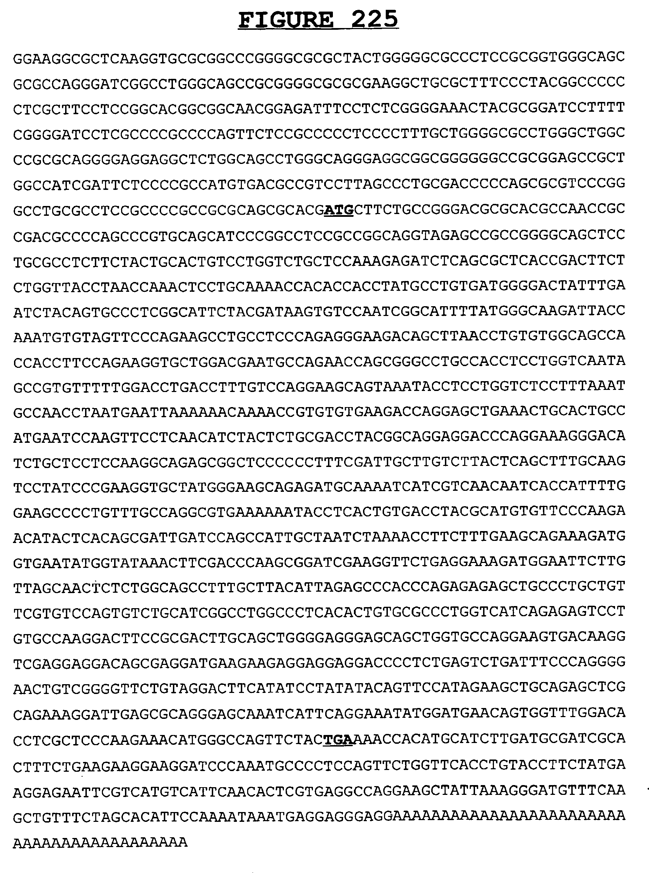

Figure 225 shows a nucleotide sequence (SEQ ID NO:225) of a native sequence PR05993 cDNA, wherein SEQ ID NO:225 is a clone designated herein as "DNA100276-2684".

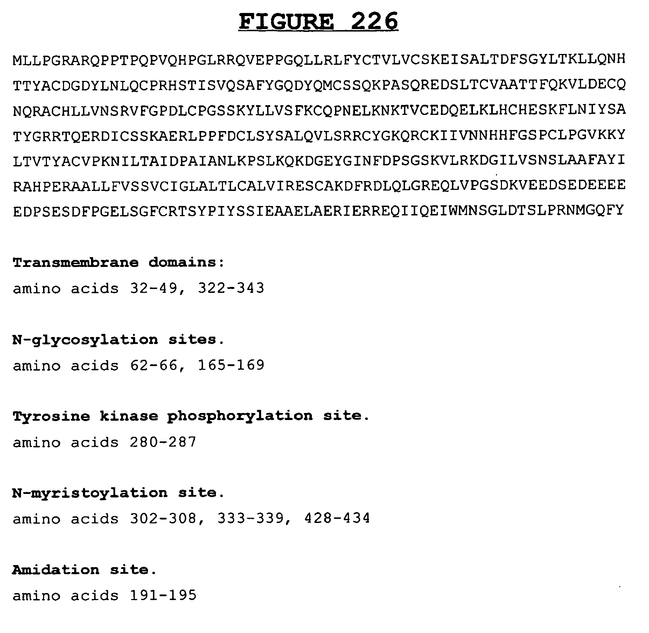

Figure 226 shows the amino acid sequence (SEQ ID NO:226) derived from the coding sequence of SEQ ID NO: 225 shown in Figure 225.

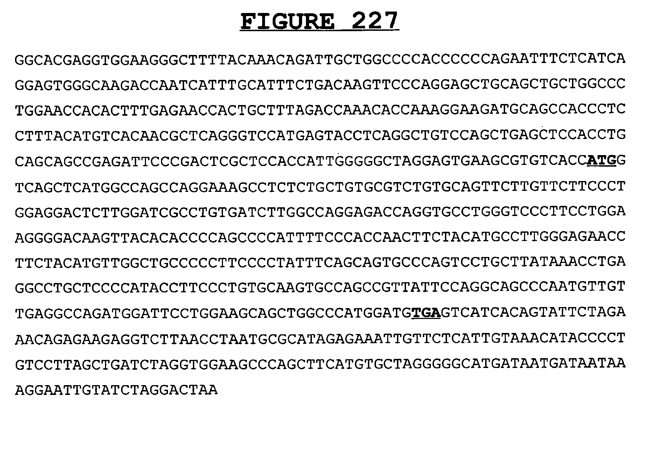

Figure 227 shows a nucleotide sequence (SEQ ID NO:227) of a native sequence PR04503 cDNA, wherein SEQ ID NO:227 is a clone designated herein as "DNA100312-2645".

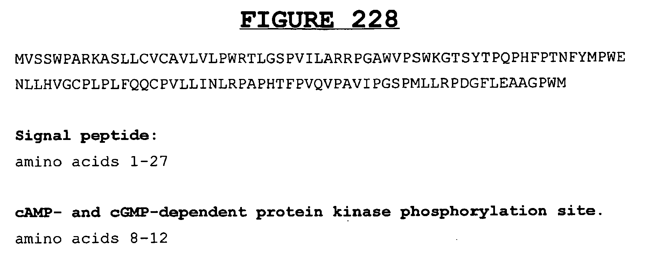

Figure 228 shows the amino acid sequence (SEQ ID NO:228) derived from the coding sequence of SEQ ID NO: 227 shown in Figure 227.

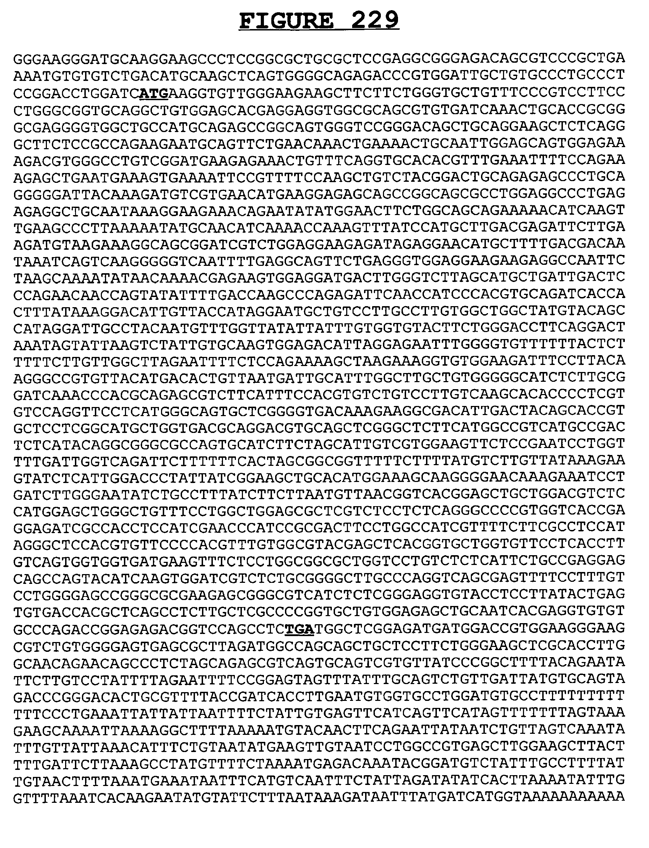

Figure 229 shows a nucleotide sequence (SEQ ID NO:229) of a native sequence PR04976 cDNA, wherein SEQ ID NO:229 is a clone designated herein as "DNA100902-2646".

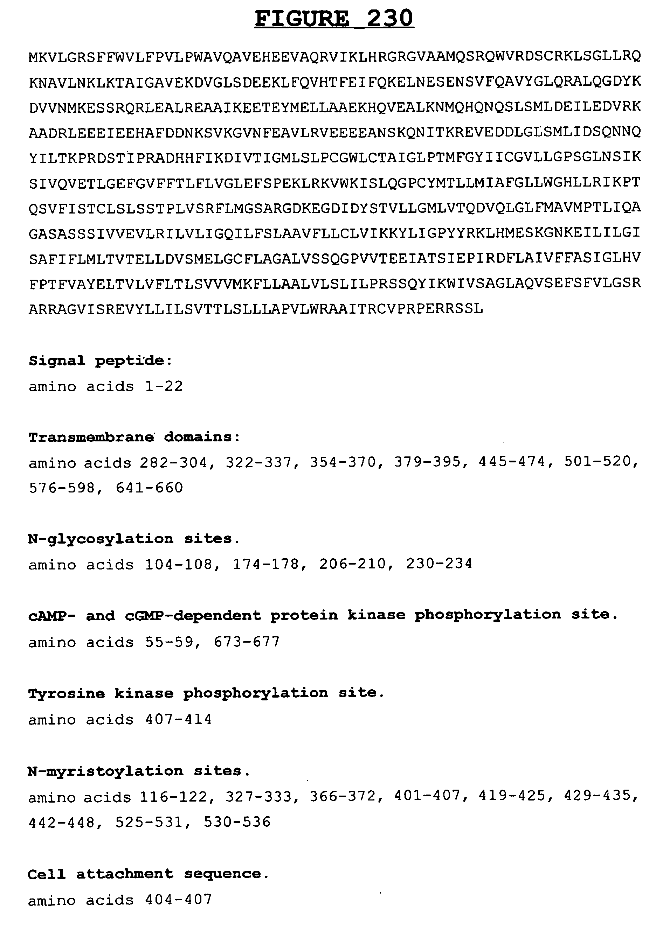

Figure 230 shows the amino acid sequence (SEQ ID NO:230) derived from the coding sequence of SEQ ID NO: 229 shown in Figure 229.

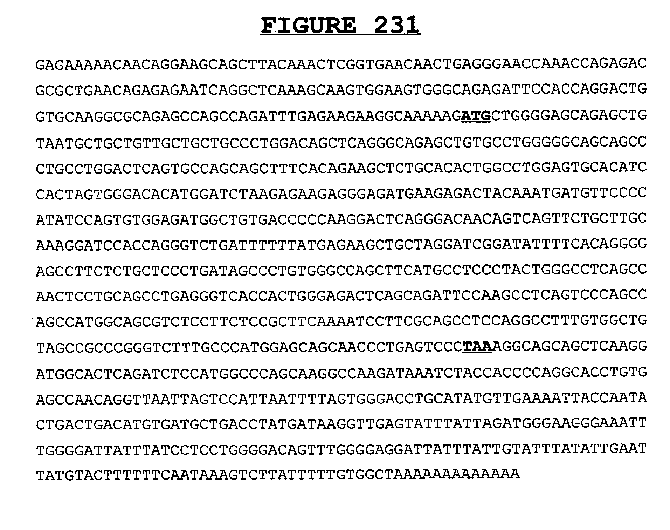

Figure 231 shows a nucleotide sequence (SEQ ID NO:231) of a native sequence PRO5798 cDNA, wherein SEQ ID NO:231 is a clone designated herein as "DNA 102899-2679".

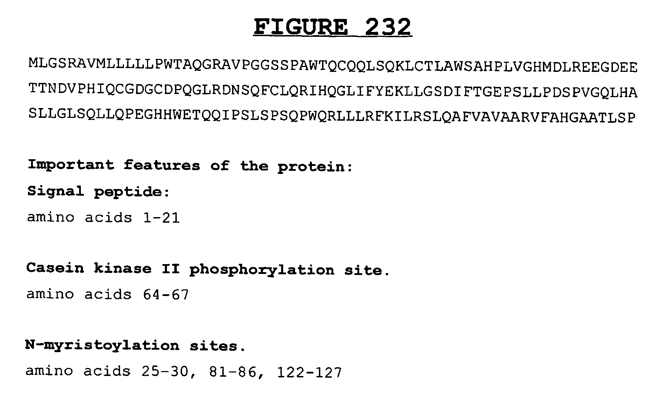

Figure 232 shows the amino acid sequence (SEQ ID NO:232) derived from the coding sequence of SEQ ID NO: 231 shown in Figure 231.

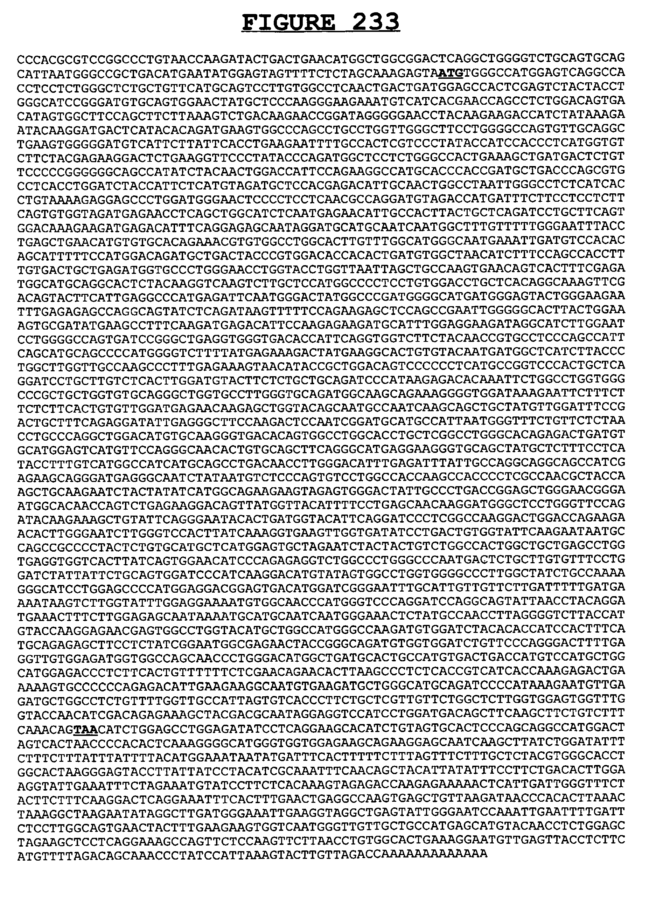

Figure 233 shows a nucleotide sequence (SEQ ID NO:233) of a native sequence PR06242 cDNA, wherein SEQ ID NO:233 is a clone designated herein as "DNA 104875-2720".

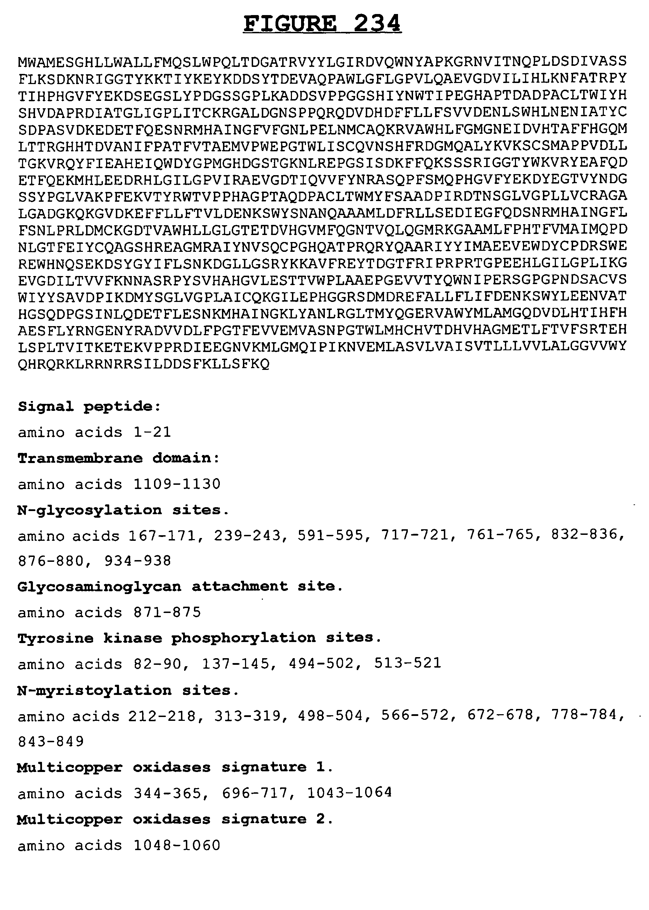

Figure 234 shows the amino acid sequence (SEQ ID NO:234) derived from the coding sequence of SEQ ID NO: 233 shown in Figure 233.

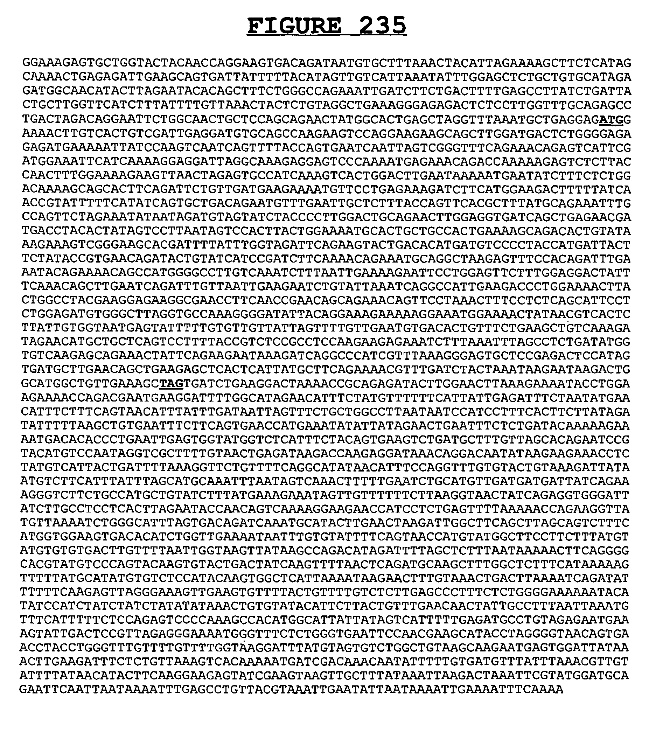

Figure 235 shows a nucleotide sequence (SEQ ID NO:235) of a native sequence PR06095 cDNA, wherein SEQ ID NO:235 is a clone designated herein as "DNA105680-2710".

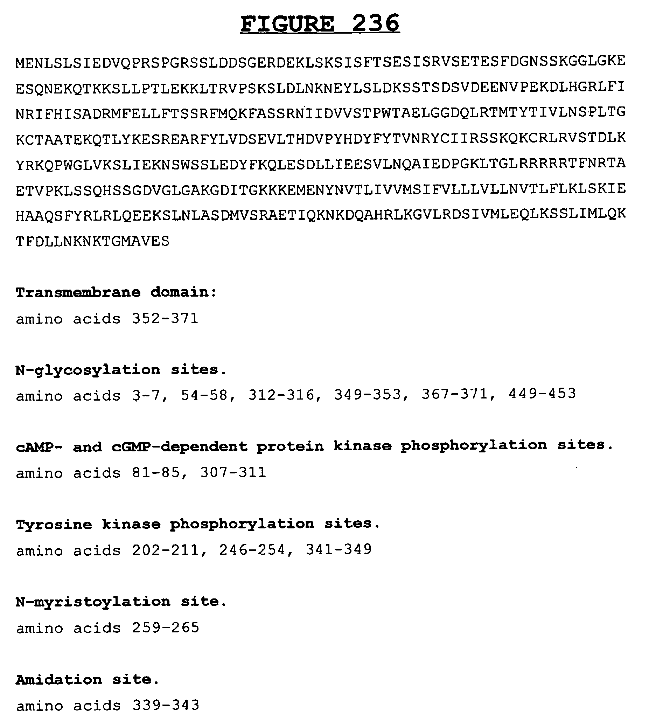

Figure 236 shows the amino acid sequence (SEQ ID NO:236) derived from the coding sequence of SEQ ID NO: 235 shown in Figure 235.

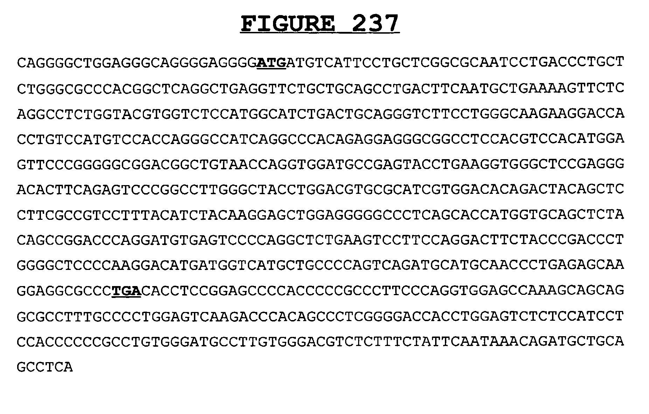

Figure 237 shows a nucleotide sequence (SEQ ID NO:237) of a native sequence PR06093 cDNA, wherein SEQ ID NO:237 is a clone designated herein as "DNA105779-2708".

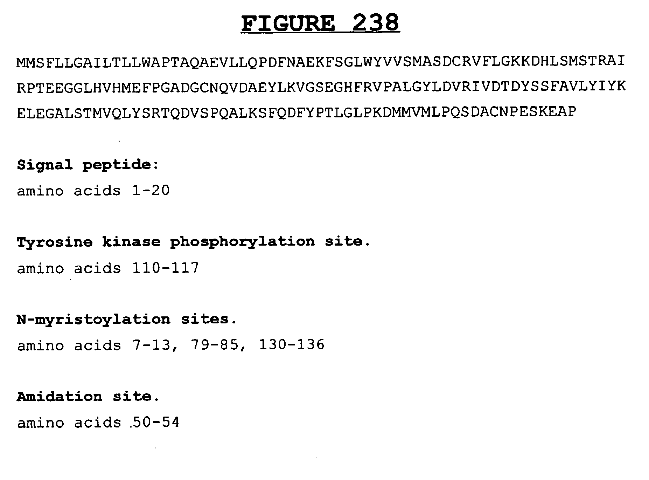

Figure 238 shows the amino acid sequence (SEQ ID NO:238) derived from the coding sequence of SEQ ID NO: 237 shown in Figure 237.

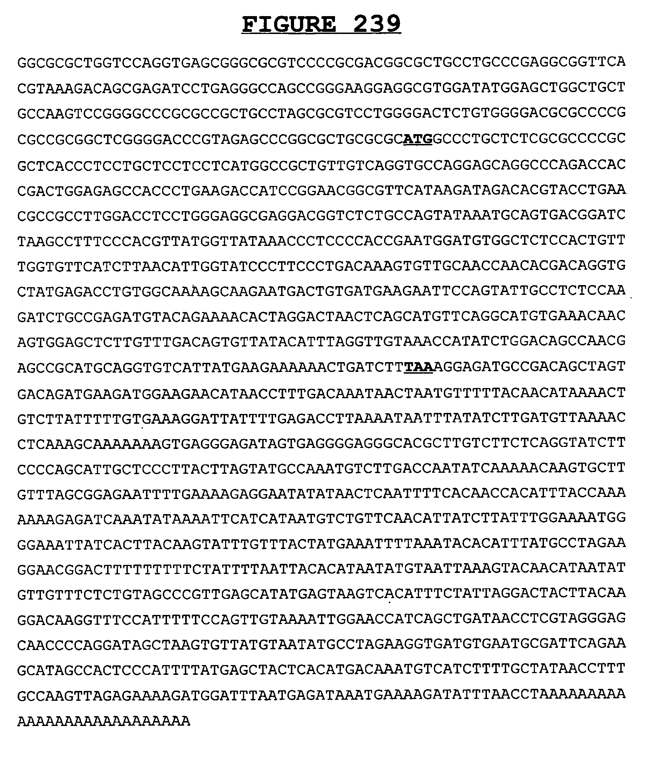

Figure 239 shows a nucleotide sequence (SEQ ID NO:239) of a native sequence PR06012 cDNA, wherein SEQ ID NO:239 is a clone designated herein as "DNA105794-2695".

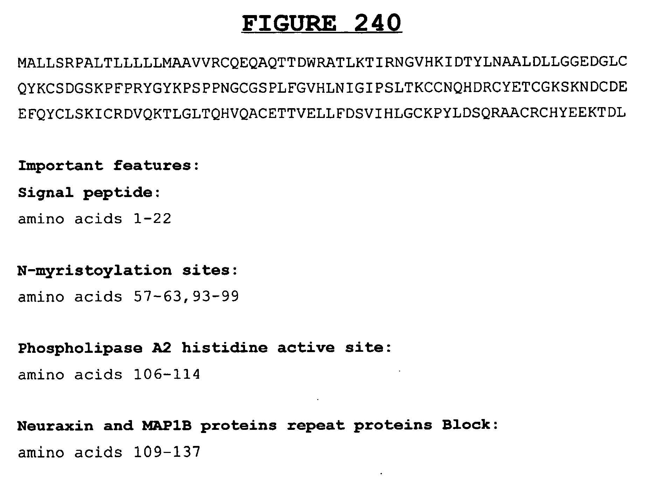

Figure 240 shows the amino acid sequence (SEQ ID NO:240) derived from the coding sequence of SEQ ID NO: 239 shown in Figure 239.

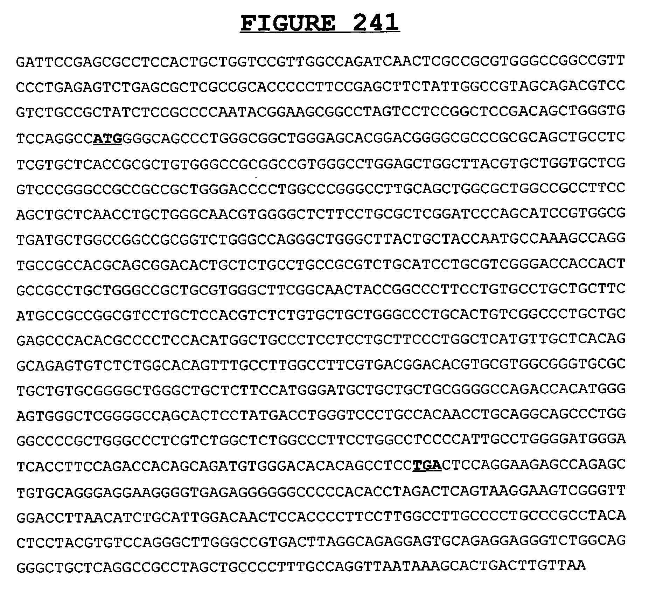

Figure 241 shows a nucleotide sequence (SEQ ID NO:241) of a native sequence PRO6027 cDNA, wherein SEQ. ID NO:241 is a clone designated herein as "DNA105838-2702".

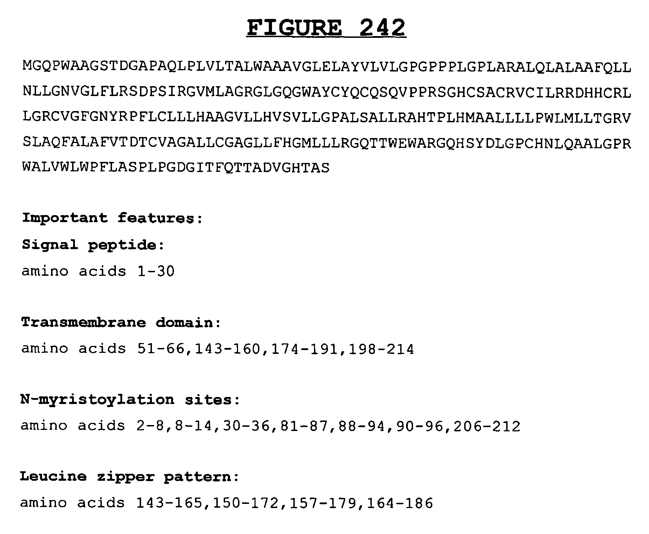

Figure 242 shows the amino acid sequence (SEQ ID NO:242) derived from the coding sequence of SEQ ID NO: 241 shown in Figure 241.

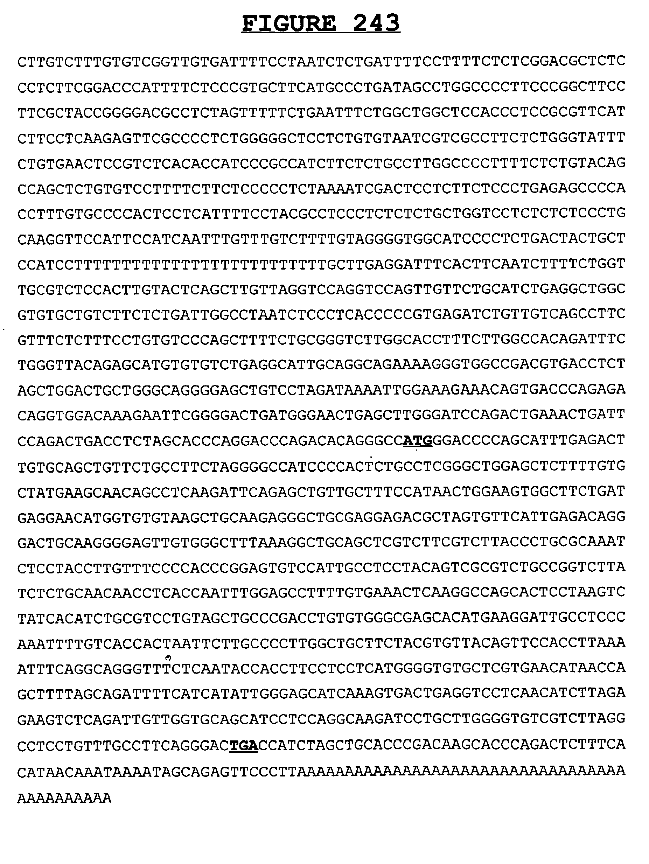

Figure 243 shows a nucleotide sequence (SEQ ID NO:243) of a native sequence PRO6181 cDNA, wherein SEQ ID NO: 243 is a clone designated herein as "DNA107698-2715".

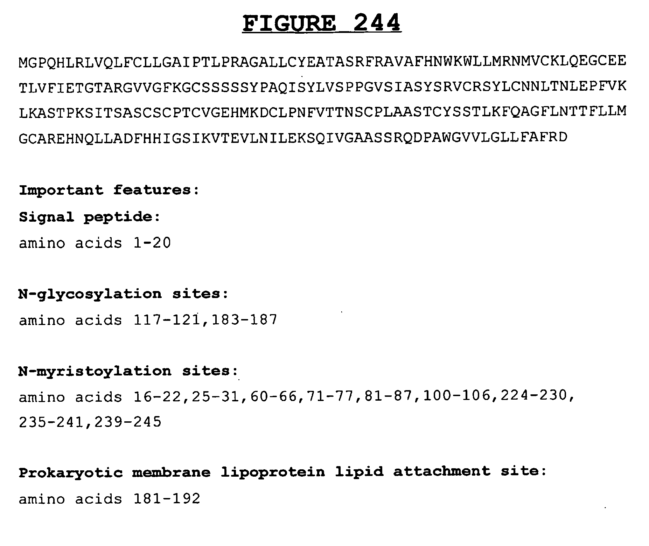

Figure 244 shows the amino acid sequence (SEQ ID NO:244) derived from the coding sequence of SEQ ID NO: 243 shown in Figure 243.

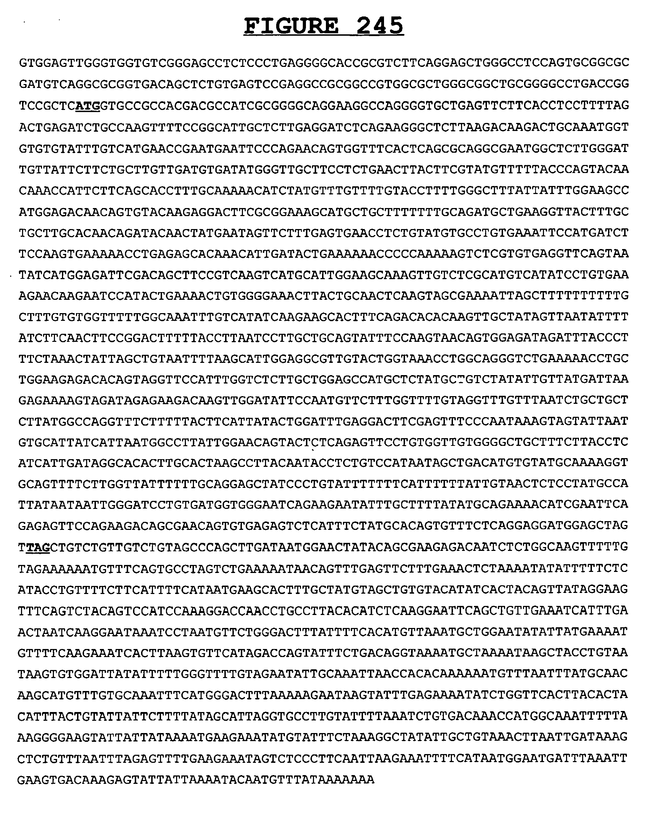

Figure 245 shows a nucleotide sequence (SEQ ID NO:245)" of a native sequence PR06097 cDNA, wherein SEQ ID NO:245 is a clone designated herein as "DNA107701-2711 ".

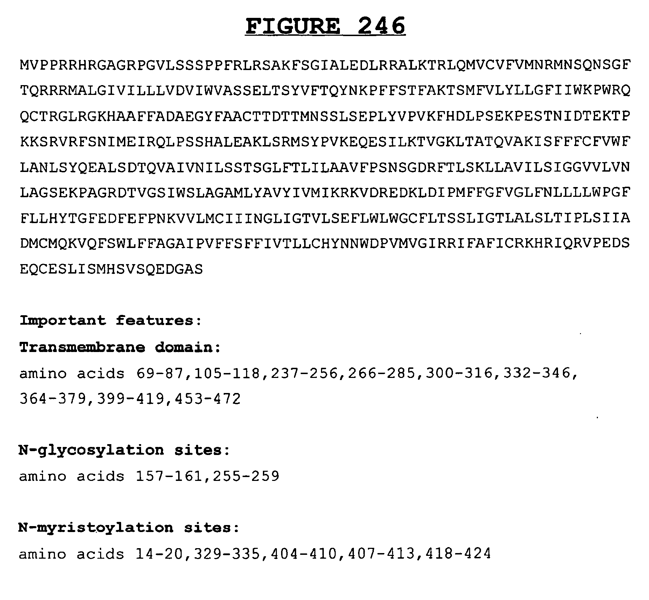

Figure 246 shows the amino acid sequence (SEQ ID NO:246) derived from the coding sequence of SEQ ID NO: 245 shown in Figure 245.

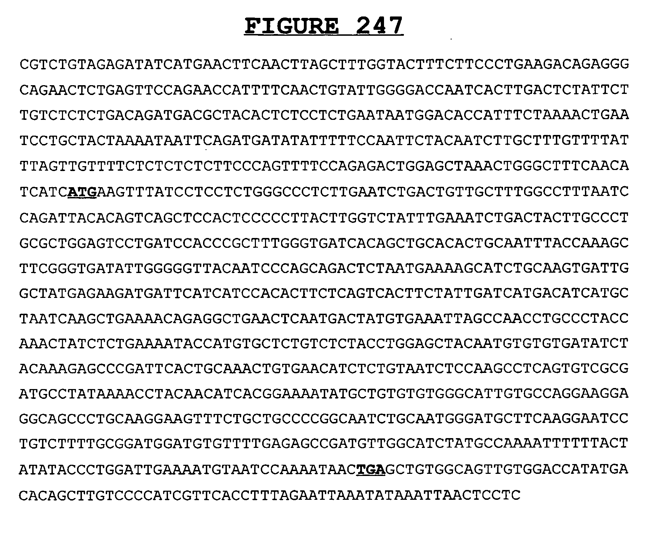

Figure 247 shows a nucleotide sequence (SEQ ID NO:247) of a native sequence PR06090 cDNA, wherein SEQ ID NO:247 is a clone designated herein as "DNA107781-2707".

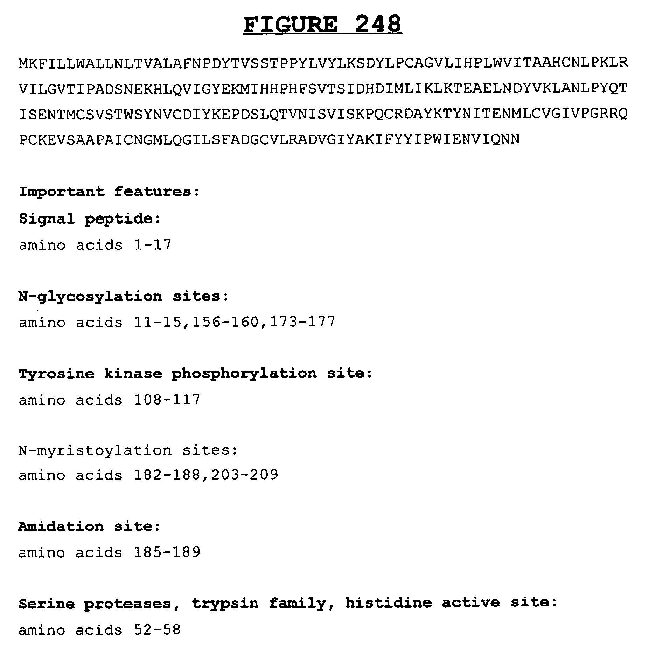

Figure 248 shows the amino acid sequence (SEQ ID NO:248) derived from the coding sequence of SEQ ID NO: 247 shown in Figure 247.

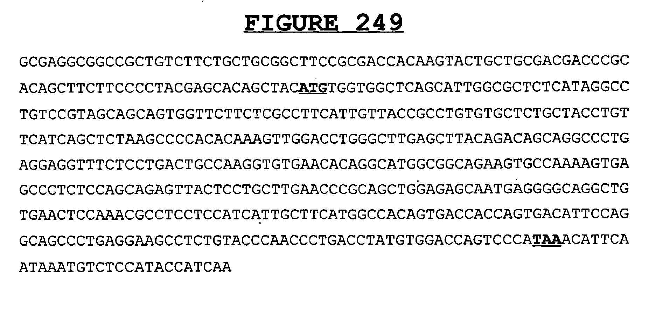

Figure 249 shows a nucleotide sequence (SEQ ID NO:249) of a native sequence PR07171 cDNA, wherein SEQ ID NO:249 is a clone designated herein as "DNA108670-2744".

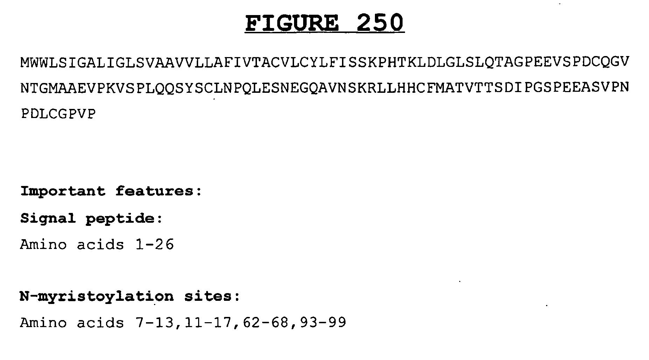

Figure 250 shows the amino acid sequence (SEQ ID NO:250) derived from the coding sequence of SEQ ID NO: 249 shown in Figure 249.

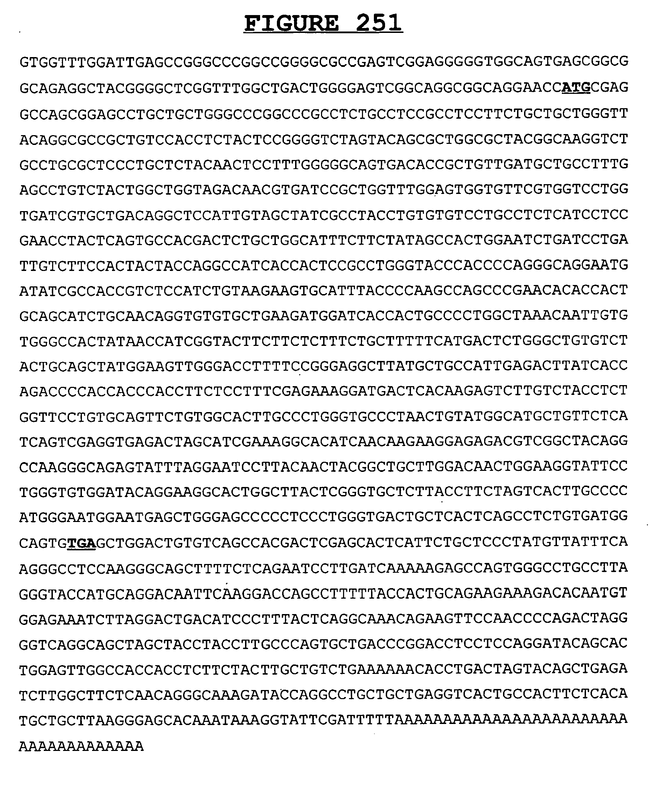

Figure 251 shows a nucleotide sequence (SEQ ID NO:251) of a native sequence PR06258 cDNA, wherein SEQ ID NO:251 is a clone designated herein as "DNAI08688-2725".

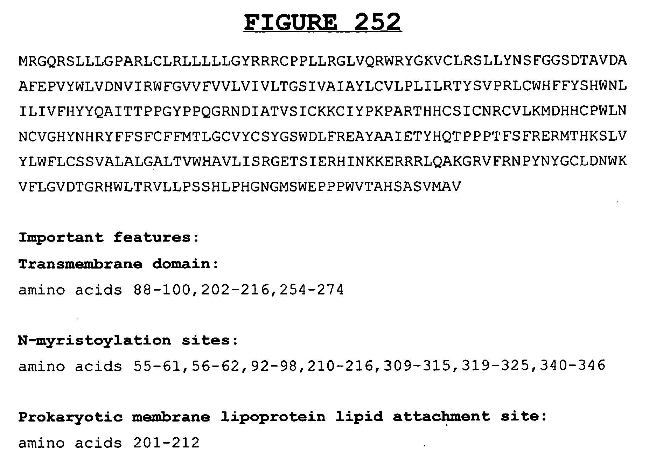

Figure 252 shows the amino acid sequence (SEQ ID NO:252) derived from the coding sequence of SEQ ID NO: 251 shown in Figure 251.

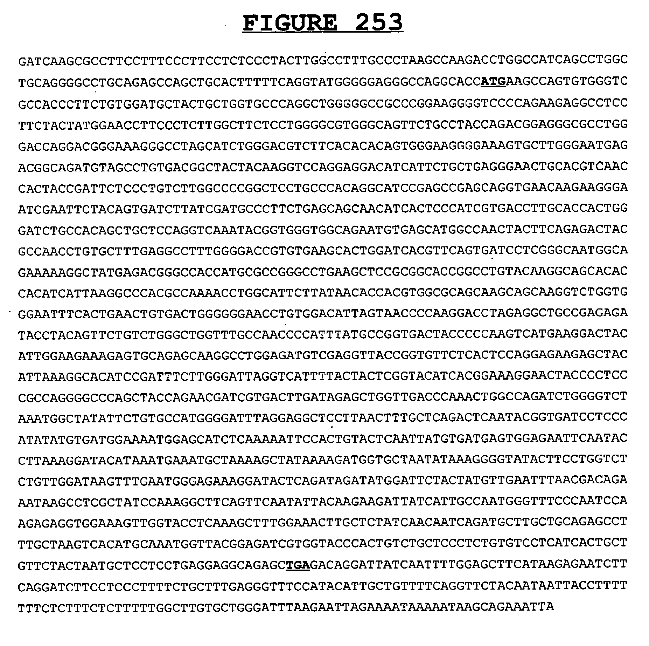

Figure 253 shows a nucleotide sequence (SEQ ID NO:253) of a native sequence PR09820 cDNA, wherein SEQ ID NO:253 is a clone designated herein as "DNA108769-2765".

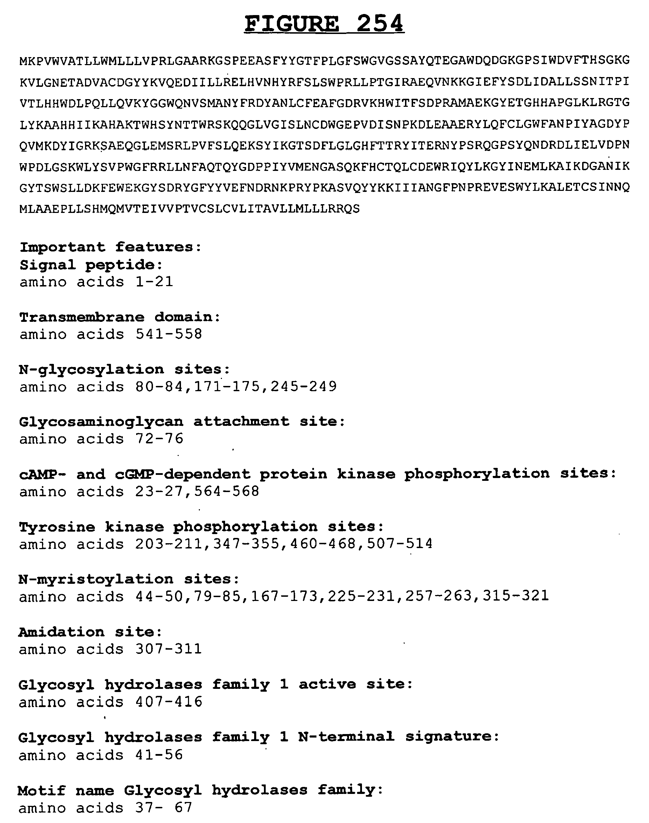

Figure 254 shows the amino acid sequence (SEQ ID NO:254) derived from the coding sequence of SEQ ID NO: 253 shown in Figure 253.

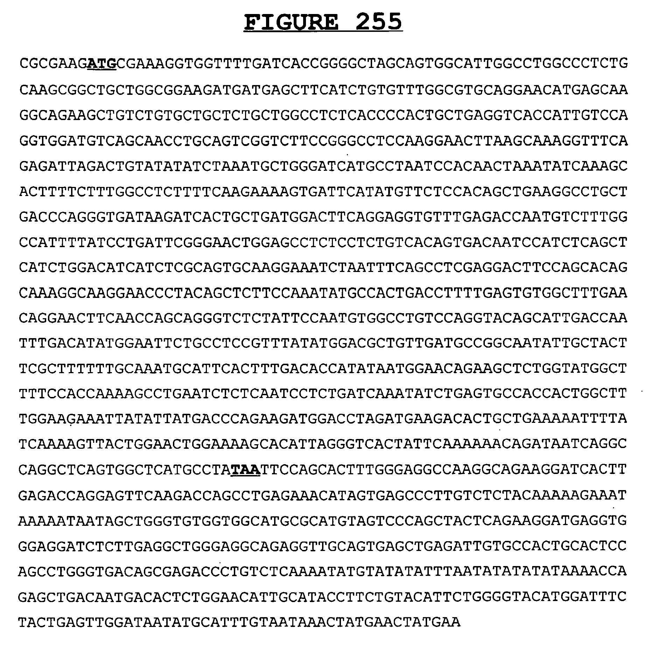

Figure 255 shows a nucleotide sequence (SEQ ID NO:255) of a native sequence PR06243 cDNA, wherein SEQ ID NO:255 is a clone designated herein as "DNA108935-2721".

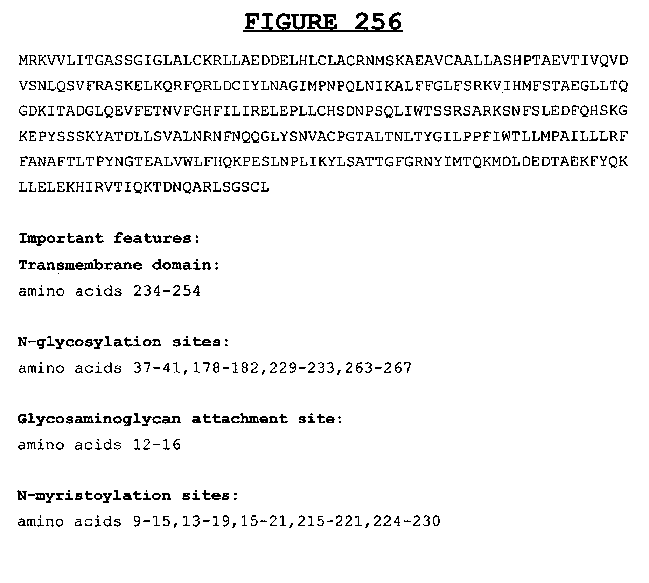

Figure 256 shows the amino acid sequence (SEQ ID NO:256) derived from the coding sequence of SEQ ID NO: 255 shown in Figure 255.

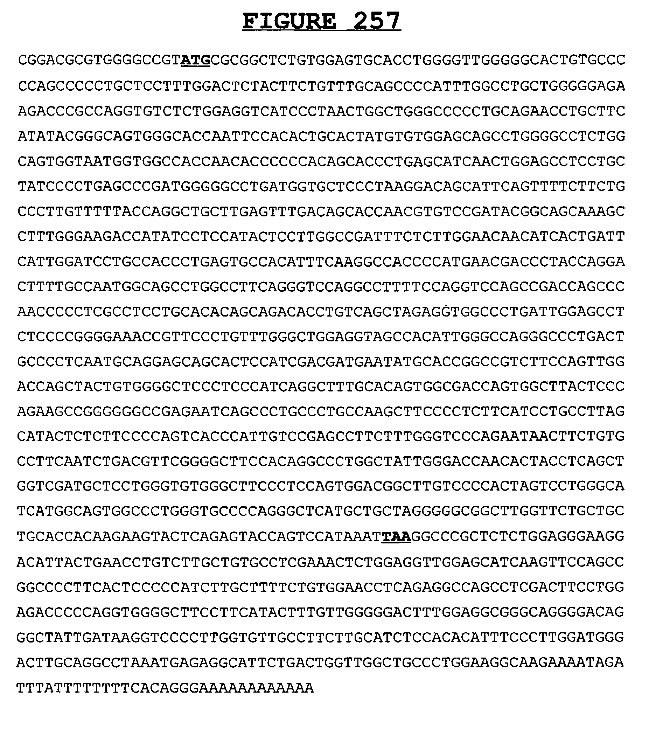

Figure 257 shows a nucleotide sequence (SEQ ID NO:257) of a native sequence PR06182 cDNA, wherein SEQ ID NO:257 is a clone designated herein as "DNA110700-2716".

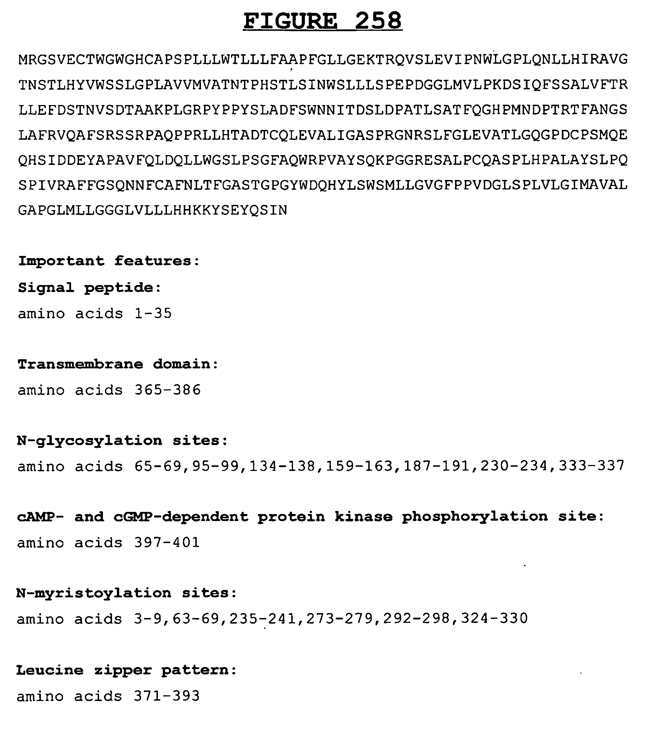

Figure 258 shows the amino acid sequence (SEQ ID NO:258) derived from the coding sequence of SEQ ID NO: 257 shown in Figure 257.

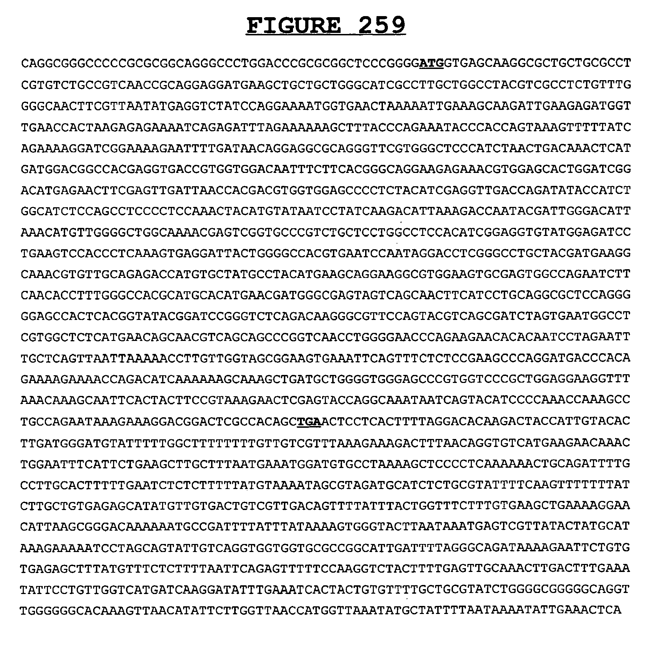

Figure 259 shows a nucleotide sequence (SEQ ID NO:259) of a native sequence PR06079 cDNA, wherein SEQ ID NO:259 is a clone designated herein as "DNA111750-2706".

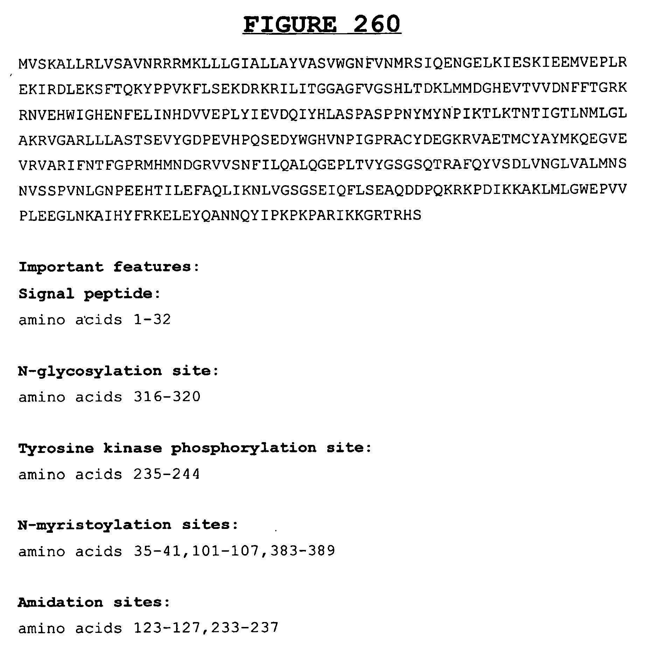

Figure 260 shows the amino acid sequence (SEQ ID NO:260) derived from the coding sequence of SEQ ID NO: 259 shown in Figure 259.

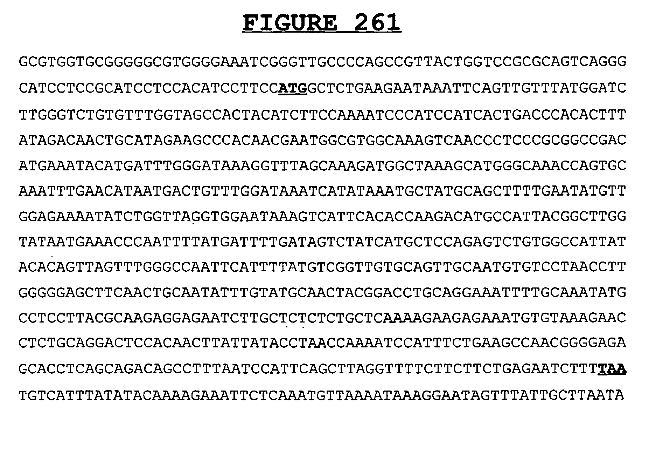

Figure 261 shows a nucleotide sequence (SEQ ID NO:261) of a native sequence PR07434 cDNA, wherein SEQ ID NO:261 is a clone designated herein as "DNA123430-2755".

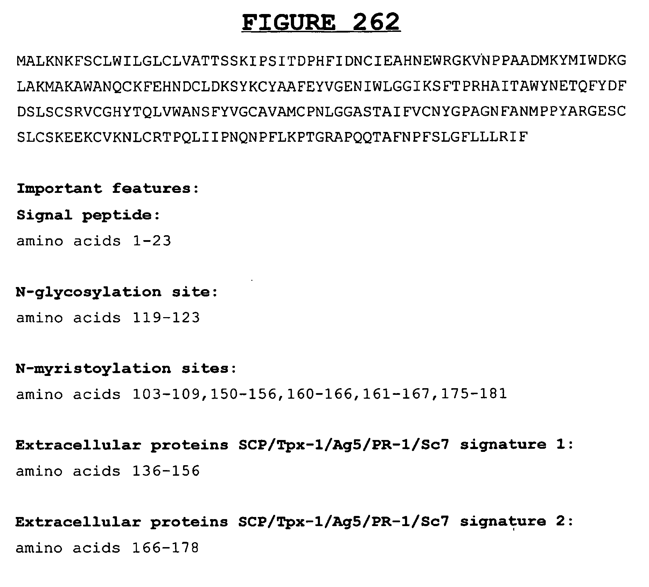

Figure 262 shows the amino acid sequence (SEQ ID NO:262) derived from the coding sequence of SEQ ID NO: 261 shown in Figure 261.

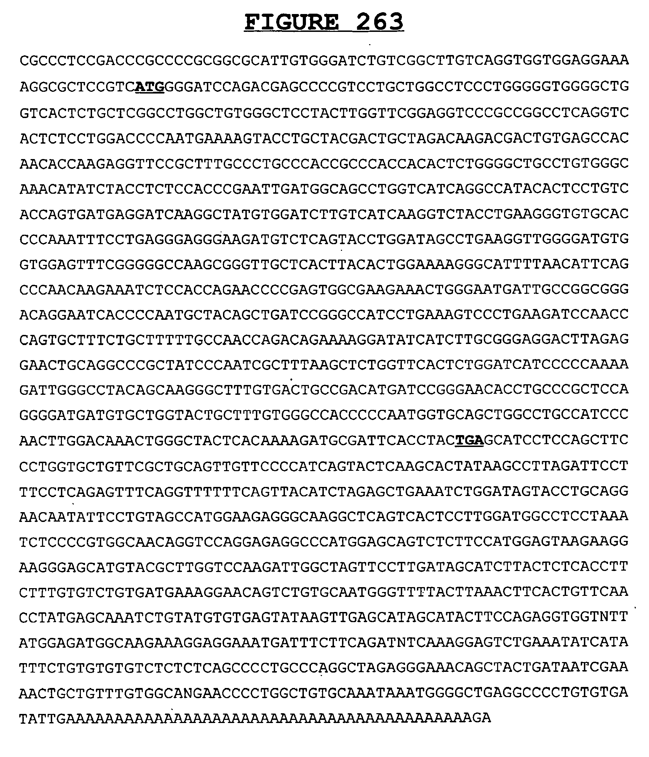

Figure 263 shows a nucleotide sequence (SEQ ID NO:263) of a native sequence PR09865 cDNA, wherein SEQ ID NO:263 is a clone designated herein as "DNA125154-2785".

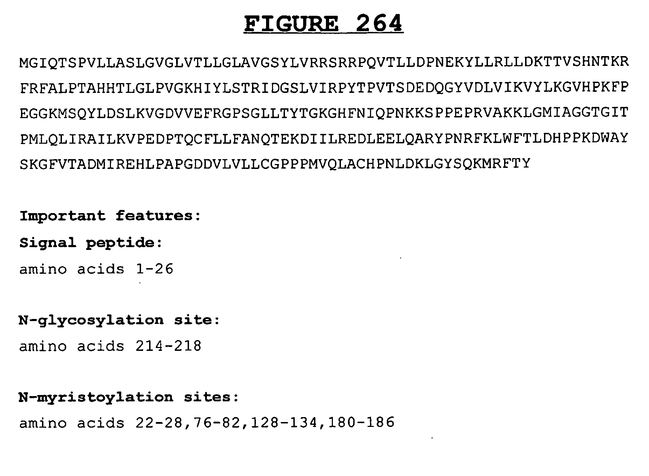

Figure 264 shows the amino acid sequence (SEQ ID NO:264) derived from the coding sequence of SEQ ID NO: 263 shown in Figure 263.

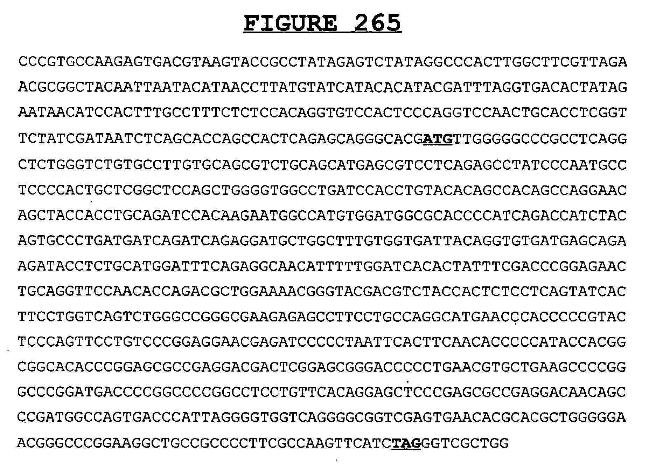

Figure 265 shows a nucleotide sequence (SEQ ID NO:265) of a native sequence PR09828 cDNA, wherein SEQ ID NO:265 is a clone designated herein as "DNA142238-2768".

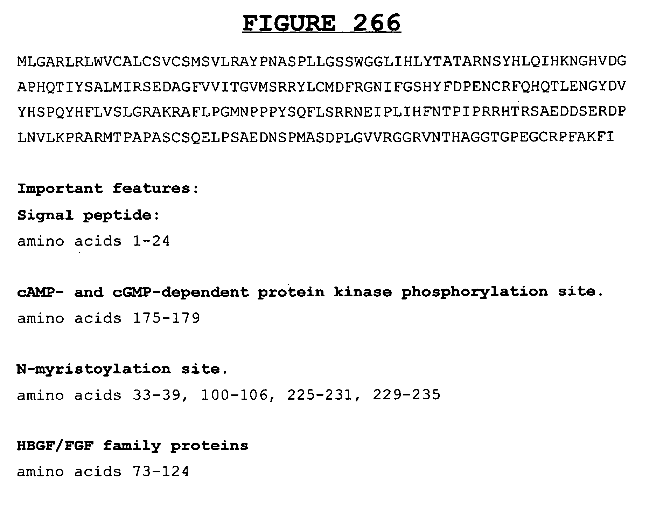

Figure 266 shows the amino acid sequence (SEQ ID NO:266) derived from the coding sequence of SEQ ID NO: 265 shown in Figure 265.

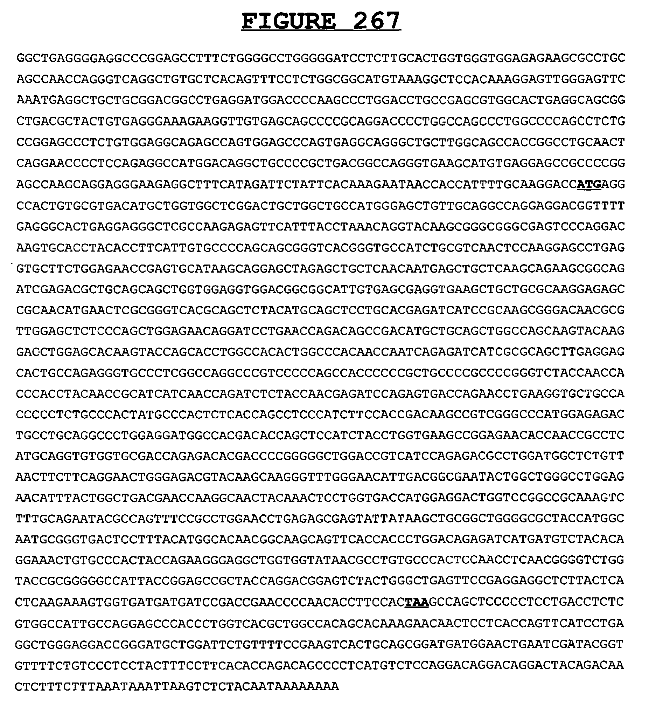

Figure 267 shows a nucleotide sequence (SEQ ID NO:267) of a native sequence PRO196 cDNA, wherein SEQ ID NO:267 is a clone designated herein as "DNA22779-1130".

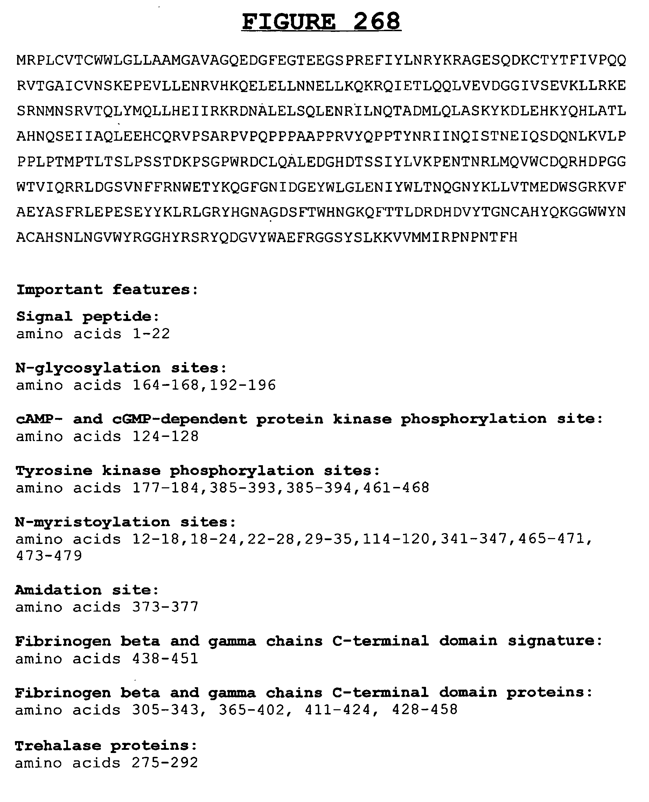

Figure 268 shows the amino acid sequence (SEQ ID NO:268) derived from the coding sequence of SEQ ID NO: 267 shown in Figure 267.

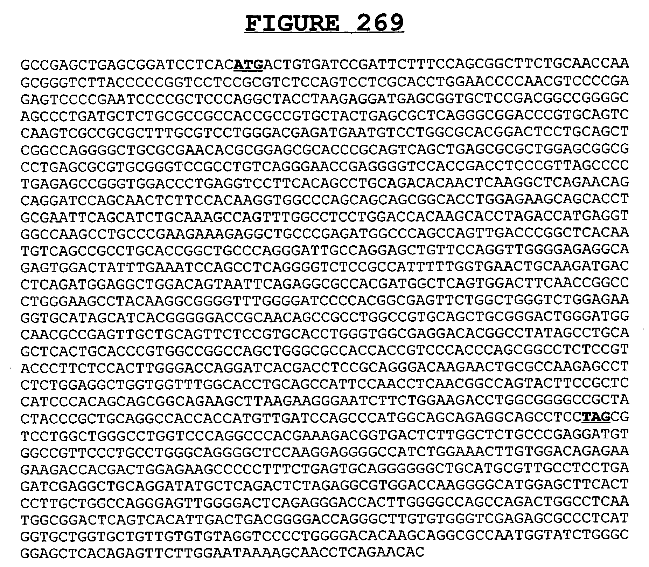

Figure 269 shows a nucleotide sequence (SEQ ID NO:269) of a native sequence PRO197 cDNA, wherein SEQ ID NO:269 is a clone designated herein as "DNA22780-1078".

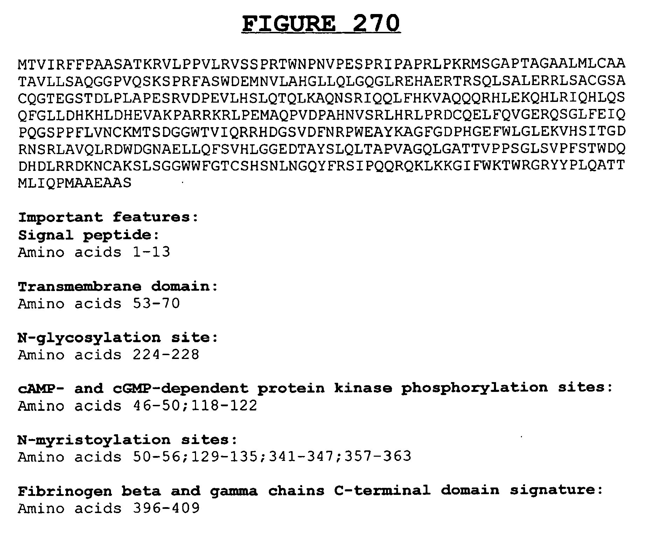

Figure 270 shows the amino acid sequence (SEQ ID NO:270) derived from the coding sequence of SEQ ID NO: 269 shown in Figure 269.

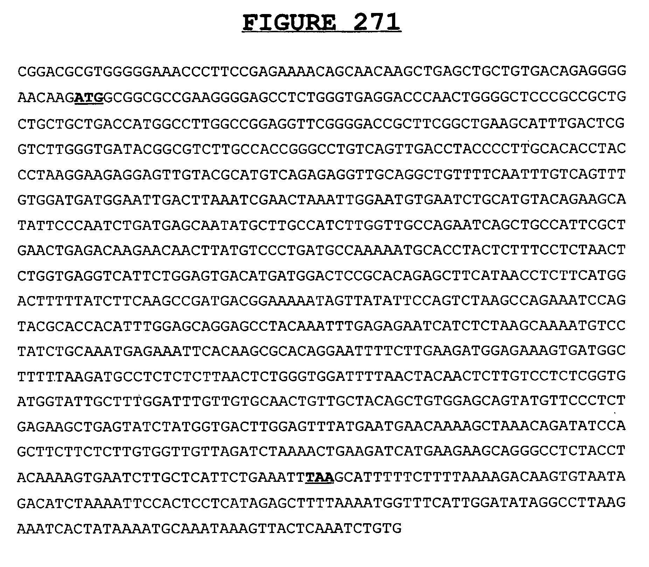

Figure 271 shows a nucleotide sequence (SEQ ID NO:271) of a native sequence PRO195 cDNA, wherein SEQ ID NO:271 is a clone designated herein as "DNA26847-1395".

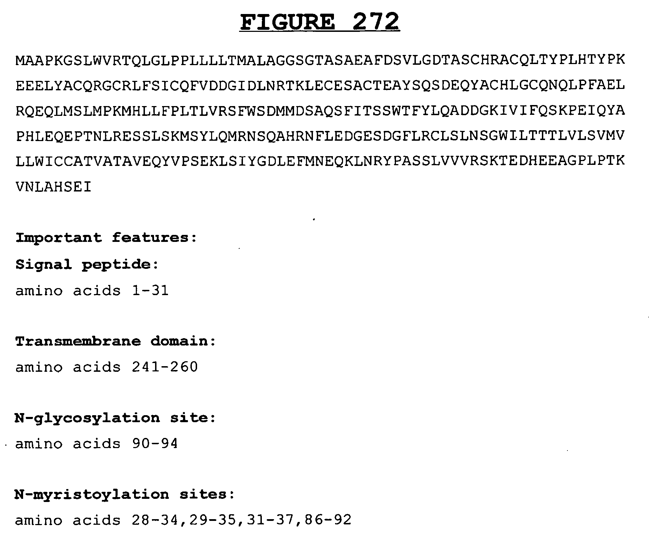

Figure 272 shows the amino acid sequence (SEQ ID NO:272) derived from the coding sequence of SEQ ID NO: 271 shown in Figure 271.

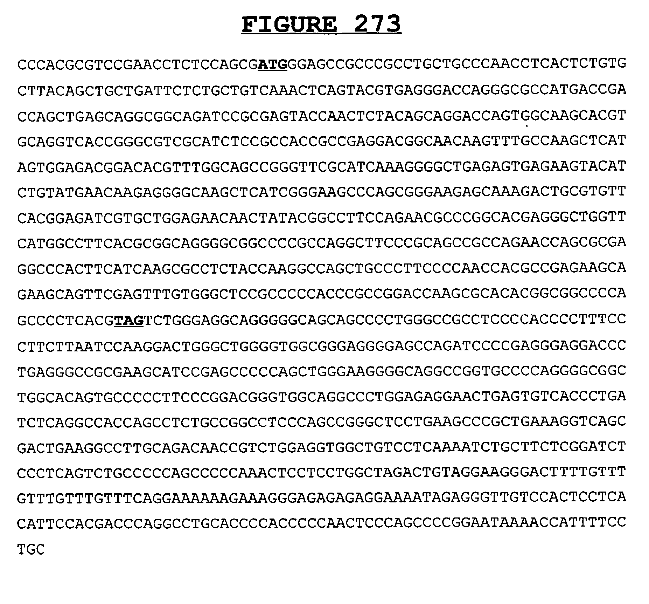

Figure 273 shows a nucleotide sequence (SEQ ID NO:273) of a native sequence PRO187 cDNA, wherein SEQ ID NO:273 is a clone designated herein as "DNA27864-1155".

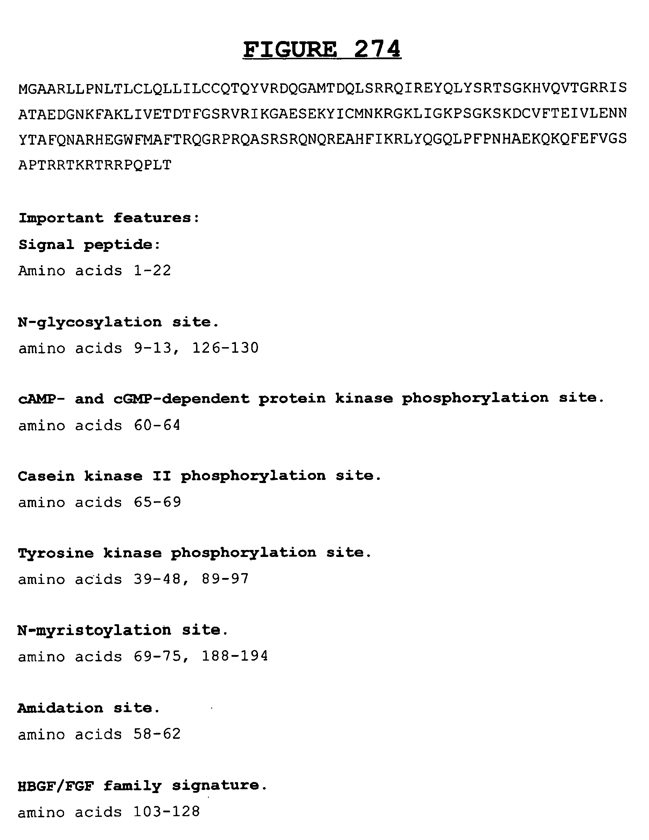

Figure 274 shows the amino acid sequence (SEQ ID NO:274) derived from the coding sequence of SEQ ID NO: 273 shown in Figure 273.

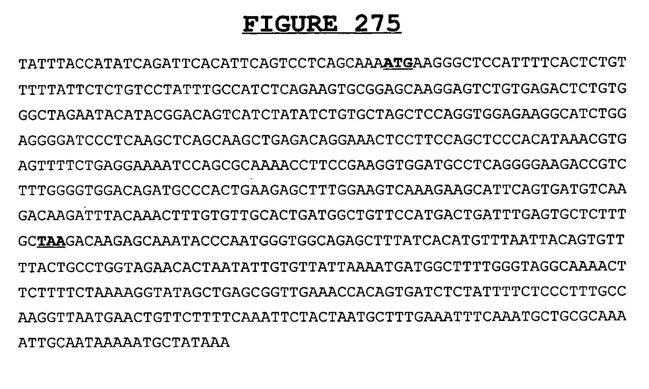

Figure 275 shows a nucleotide sequence (SEQ ID NO:275) of a native sequence PRO182 cDNA, wherein SEQ ID NO:275 is a clone designated herein as "DNA27865-1091".

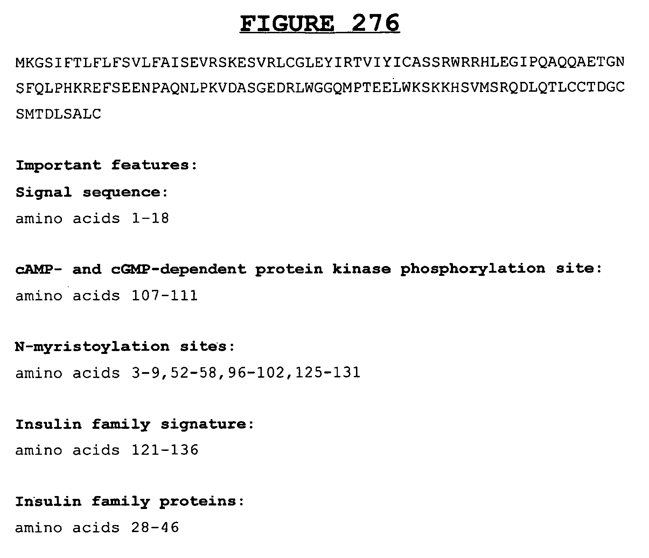

Figure 276 shows the amino acid sequence (SEQ ID NO:276) derived from the coding sequence of SEQ ID NO: 275 shown in Figure 275.

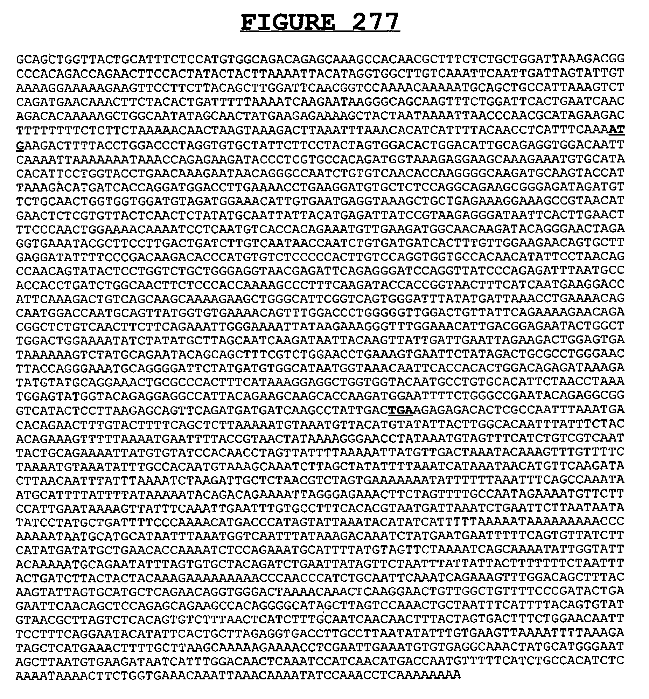

Figure 277 shows a nucleotide sequence (SEQ ID NO:277) of a native sequence PRO188 cDNA, wherein SEQ ID NO:277 is a clone designated herein as "DNA28497-1130".

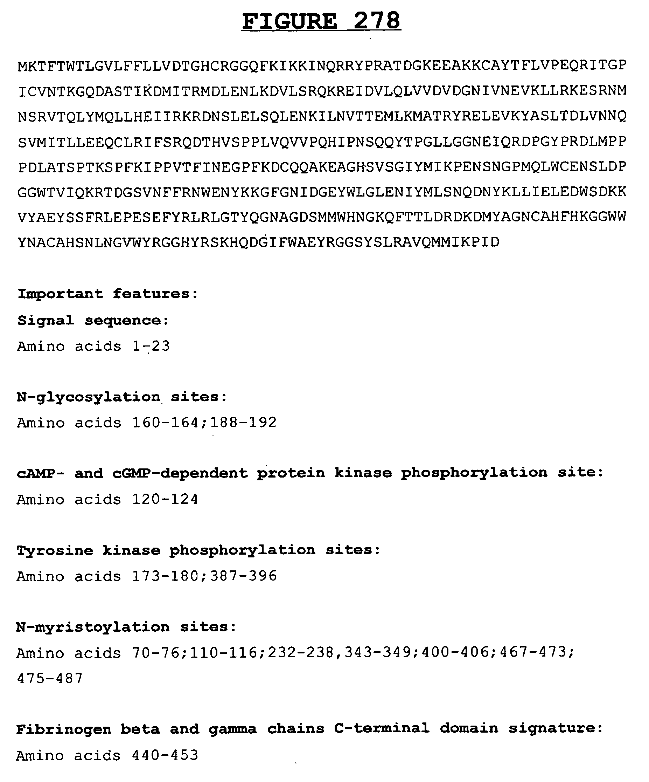

Figure 278 shows the amino acid sequence (SEQ ID NO:278) derived from the coding sequence of SEQ ID NO: 277 shown in Figure 277.

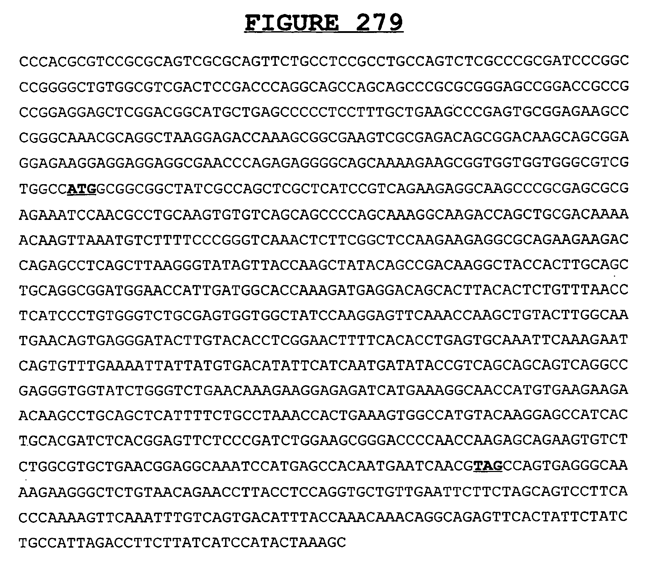

Figure 279 shows a nucleotide sequence (SEQ ID NO:279) of a native sequence PRO183 cDNA, wherein SEQ ID NO:279 is a clone designated herein as "DNA28498".

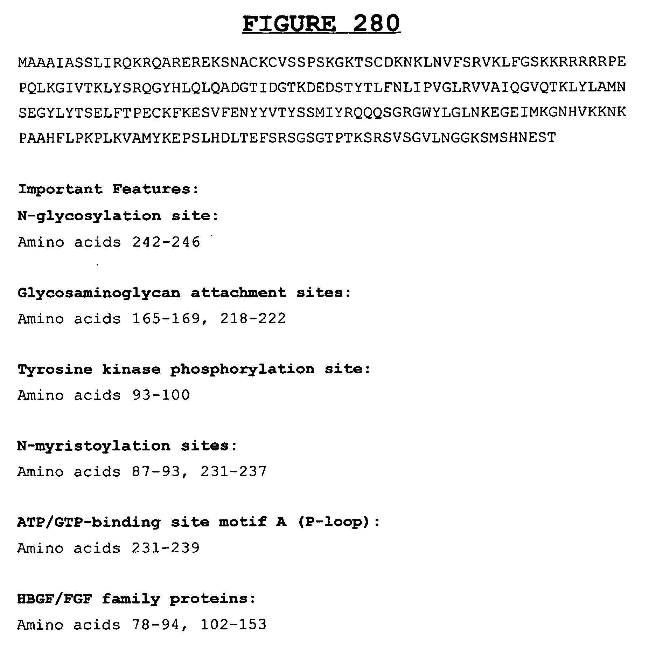

Figure 280 shows the amino acid sequence (SEQ ID NO:280) derived from the coding sequence of SEQ ID NO: 279 shown in Figure 279.

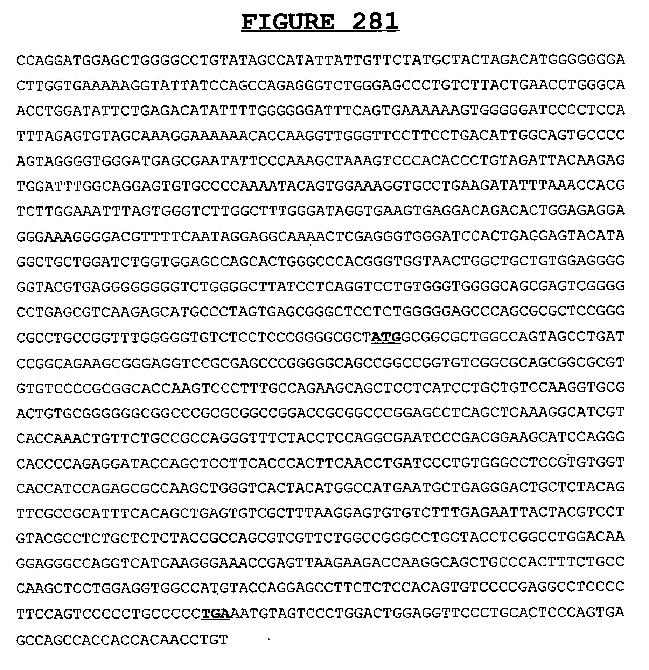

Figure 281 shows a nucleotide sequence (SEQ ID NO:281) of a native sequence PR0184 cDNA, wherein SEQ ID NO:281 is a clone designated herein as "DNA28500".

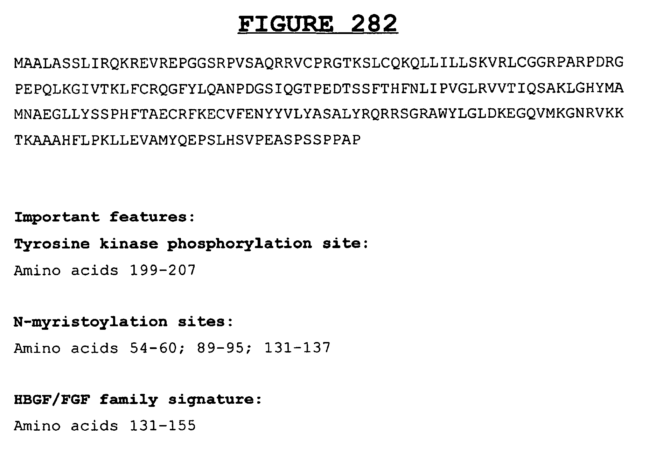

Figure 282 shows the amino acid sequence (SEQ ID NO:282) derived from the coding sequence of SEQ ID NO: 281 shown in Figure 281.

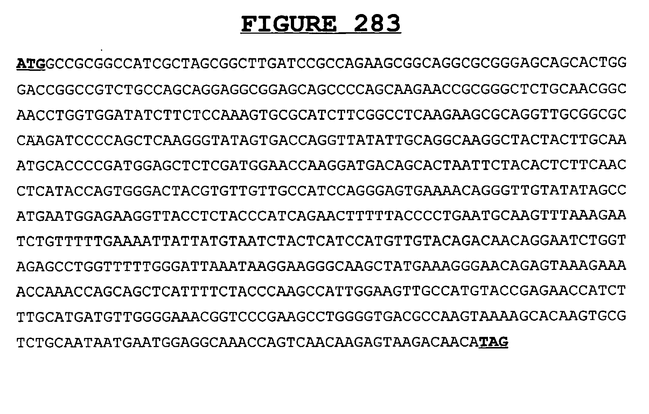

Figure 283 shows a nucleotide sequence (SEQ ID NO:283) of a native sequence PRO185 cDNA, wherein SEQ ID NO:283 is a clone designated herein as "DNA28503".

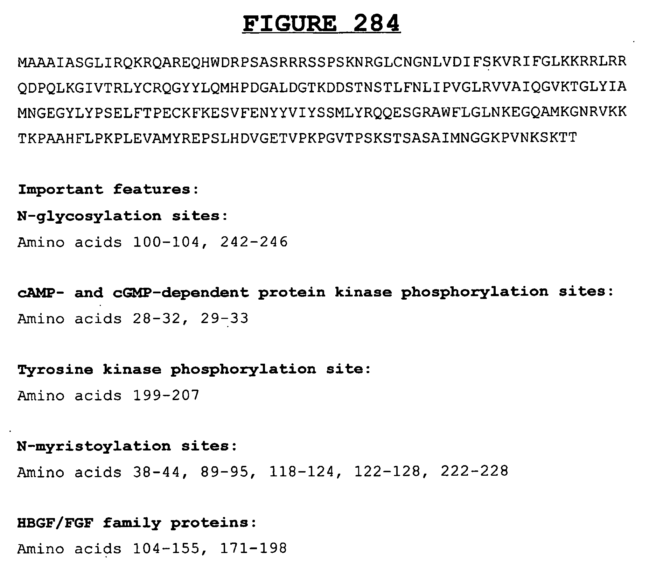

Figure 284 shows the amino acid sequence (SEQ ID NO:284) derived from the coding sequence of SEQ ID NO: 283 shown in Figure 283.

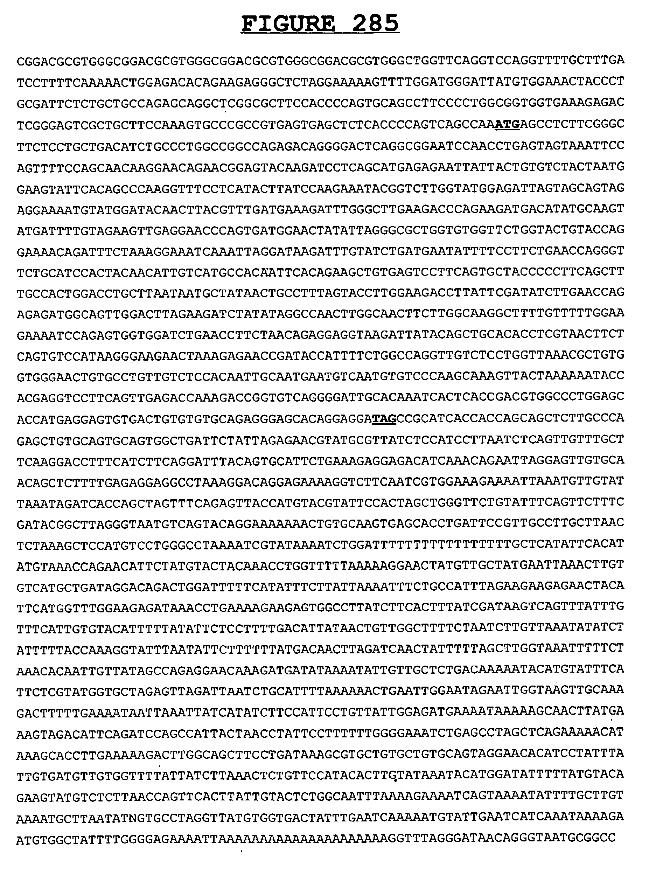

Figure 285 shows a nucleotide sequence (SEQ ID NO:285) of a native sequence PRO200 cDNA, wherein SEQ ID NO:285 is a clone designated herein as "DNA29101-1122".

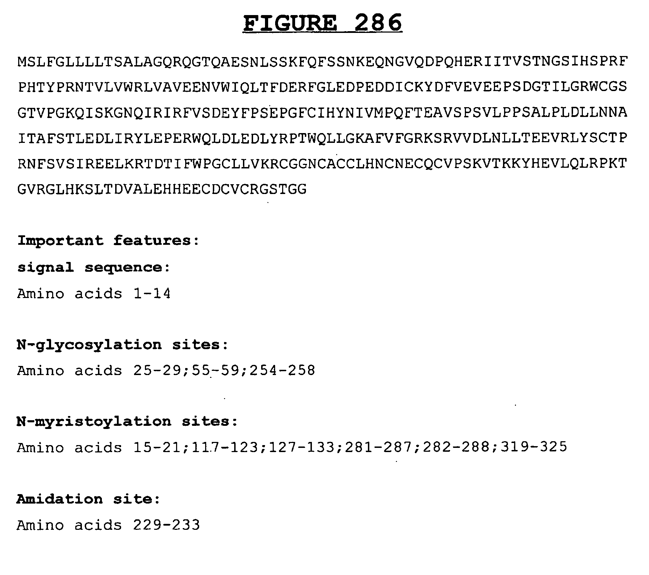

Figure 286 shows the amino acid sequence (SEQ ID NO:286) derived from the coding sequence of SEQ ID NO: 285 shown in Figure 285.

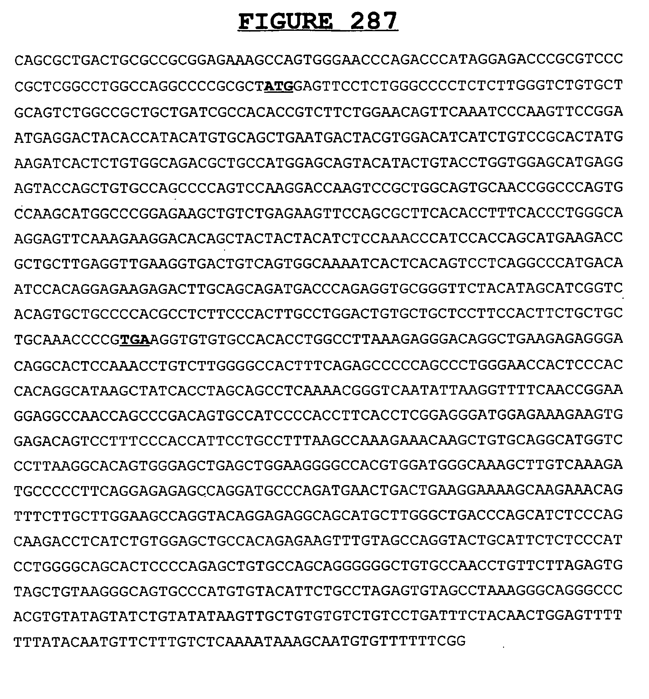

Figure 287 shows a nucleotide sequence (SEQ ID NO:287) of a native sequence PR0202 cDNA, wherein SEQ ID NO:287 is a clone designated herein as "DNA30869".

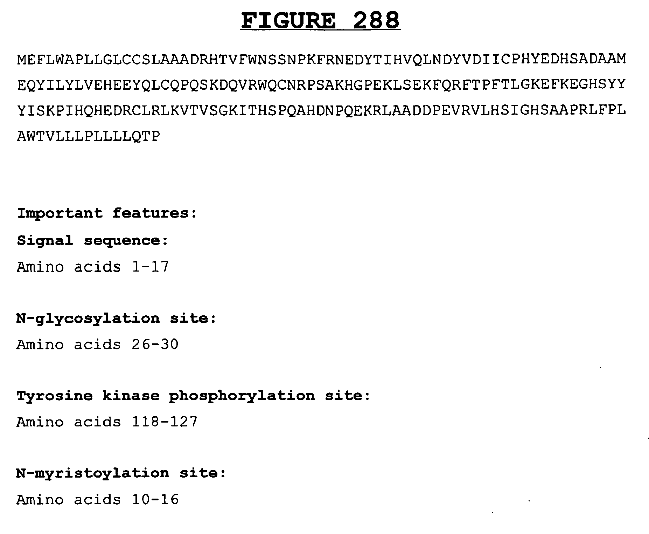

Figure 288 shows the amino acid sequence (SEQ ID NO:288) derived from the coding sequence of SEQ ID NO: 287 shown in Figure 287.

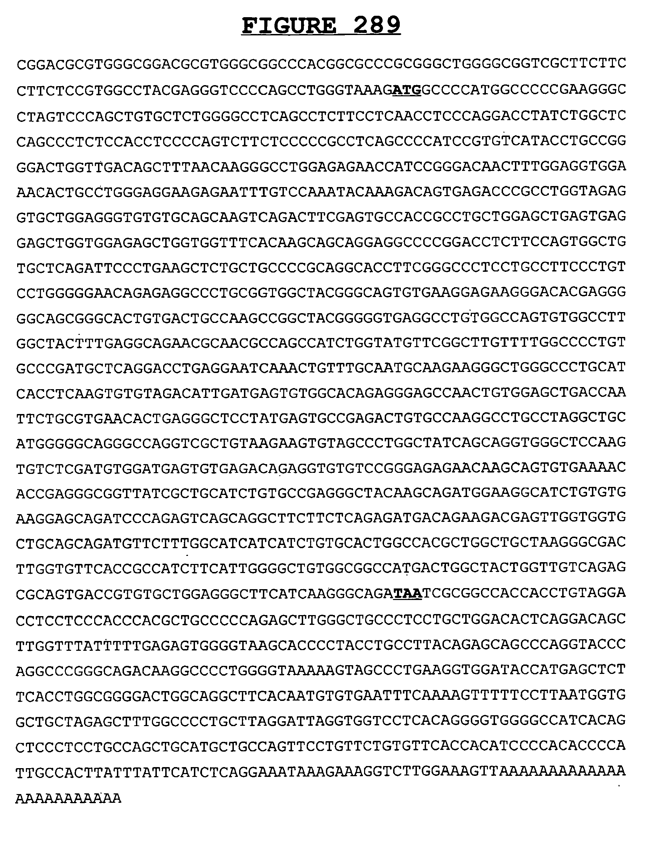

Figure 289 shows a nucleotide sequence (SEQ ID NO:289) of a native sequence PR0214 cDNA, wherein SEQ ID NO:289 is a clone designated herein as "DNA32286-1191".

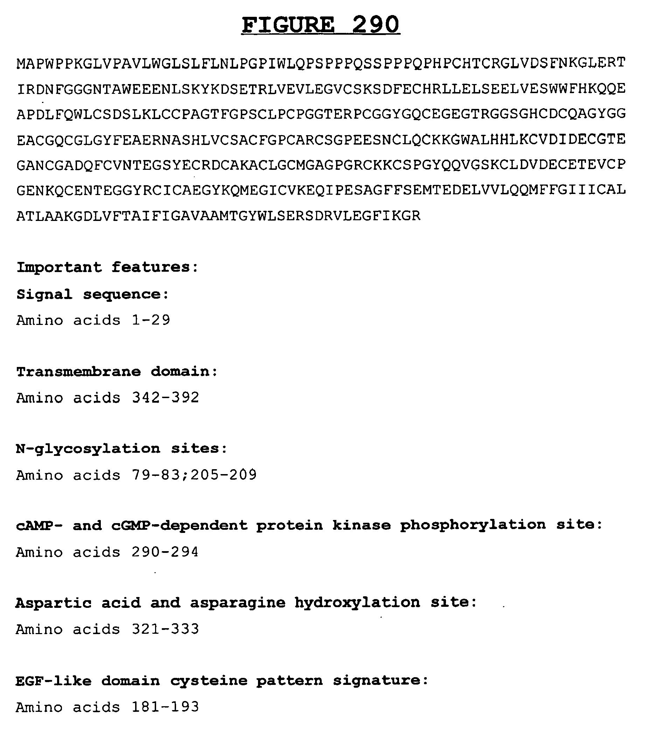

Figure 290 shows the amino acid sequence (SEQ ID NO:290) derived from the coding sequence of SEQ ID NO: 289 shown in Figure 289.

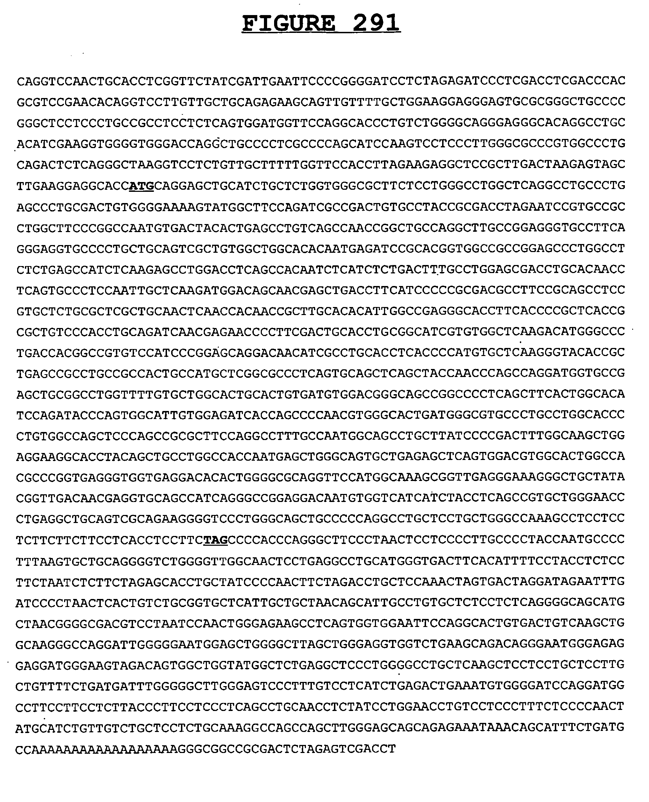

Figure 291 shows a nucleotide sequence (SEQ ID NO:291) of a native sequence PR0215 cDNA, wherein SEQ ID NO:291 is a clone designated herein as "DNA32288-1132".

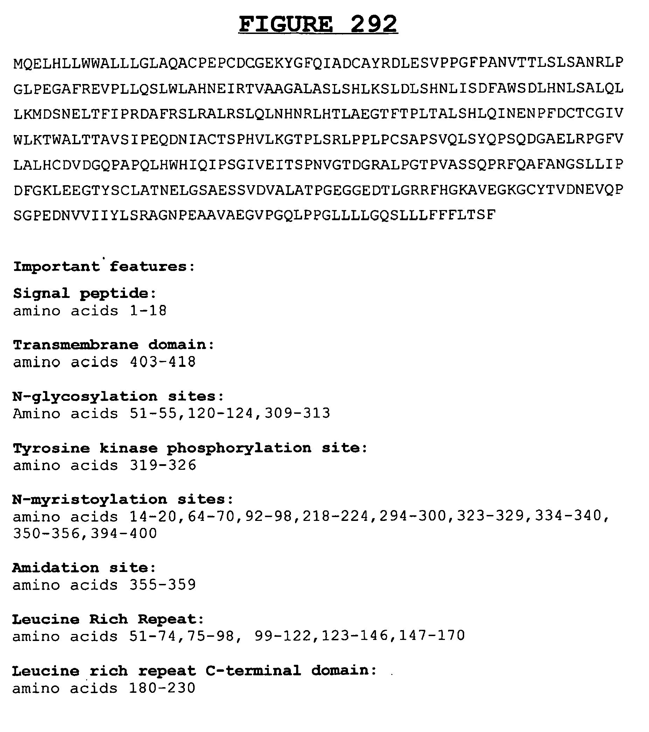

Figure 292 shows the amino acid sequence (SEQ ID NO:292) derived from the coding sequence of SEQ ID NO: 291 shown in Figure 291.

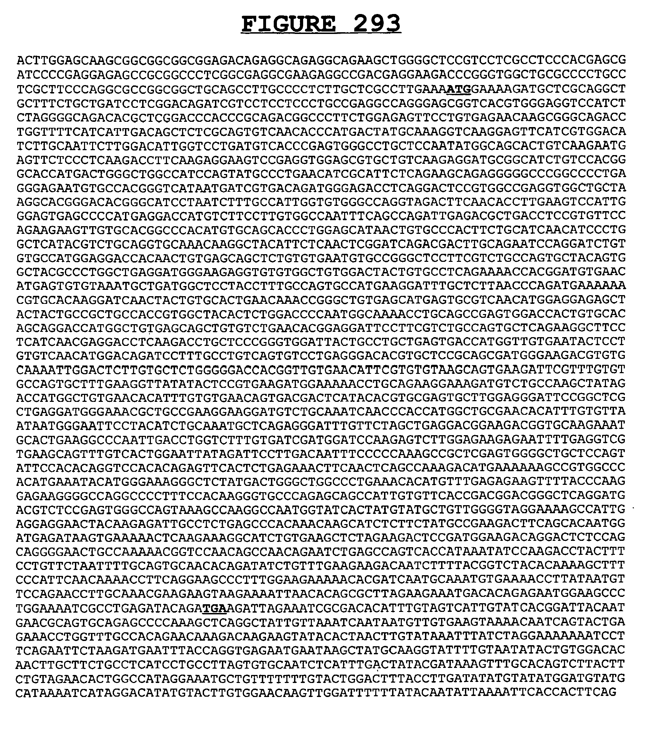

Figure 293 shows a nucleotide sequence (SEQ ID NO:293) of a native sequence PR0219 cDNA, wherein SEQ ID NO:293 is a clone designated herein as "DNA32290-1164".

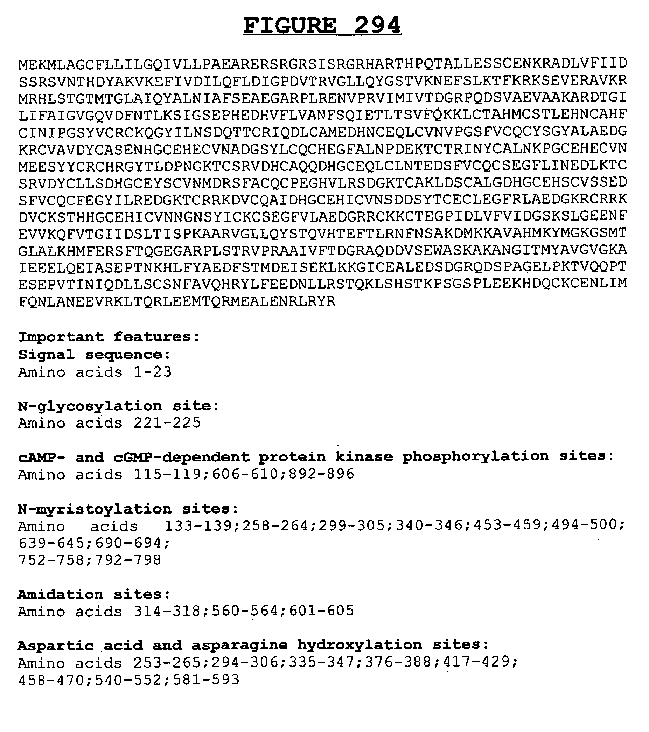

Figure 294 shows the amino acid sequence (SEQ ID NO:294) derived from the coding sequence of SEQ ID NO: 293 shown in Figure 293.

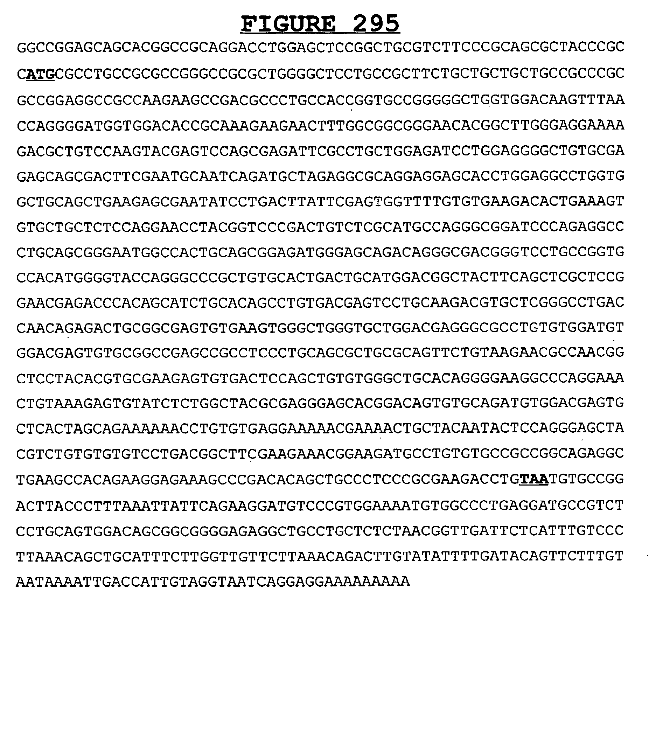

Figure 295 shows a nucleotide sequence (SEQ ID NO:295) of a native sequence PRO211 cDNA, wherein SEQ ID NO:295 is a clone designated herein as "DNA32292-1131".

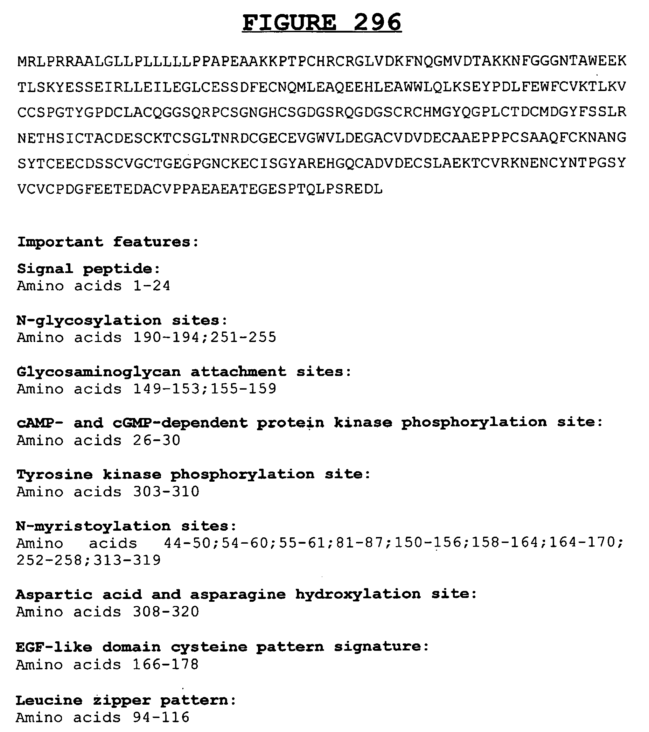

Figure 296 shows the amino acid sequence (SEQ ID NO:296) derived from the coding sequence of SEQ ID NO: 295 shown in Figure 295.

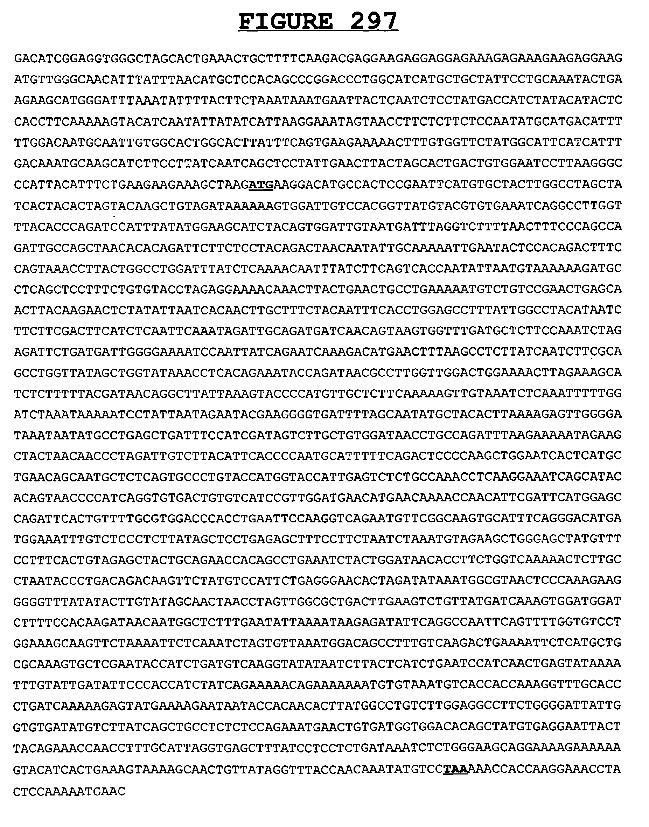

Figure 297 shows a nucleotide sequence (SEQ ID NO:297) of a native sequence PR0220 cDNA, wherein SEQ ID NO:297 is a clone designated herein as "DNA32298-1132".

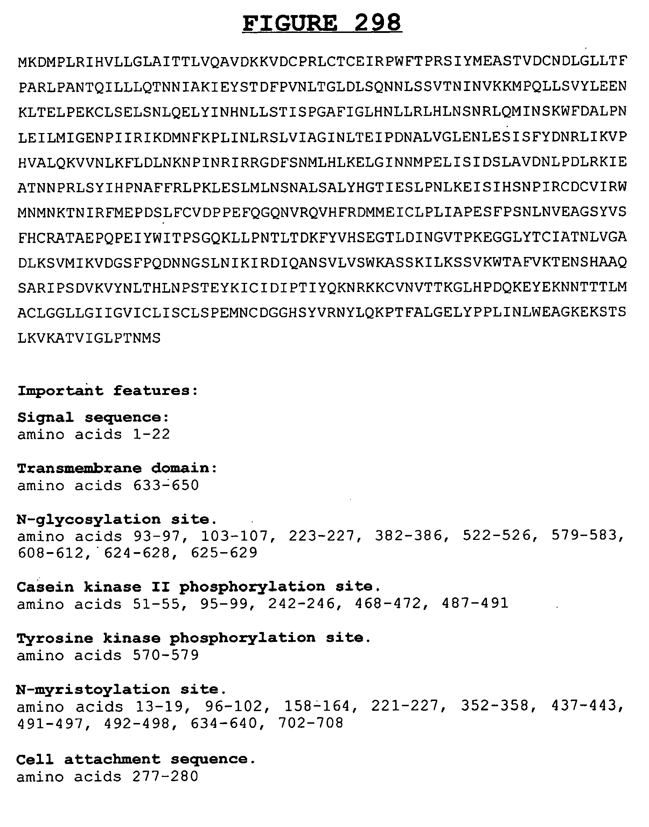

Figure 298 shows the amino acid sequence (SEQ ID NO:298) derived from the coding sequence of SEQ ID NO: 297 shown in Figure 297.

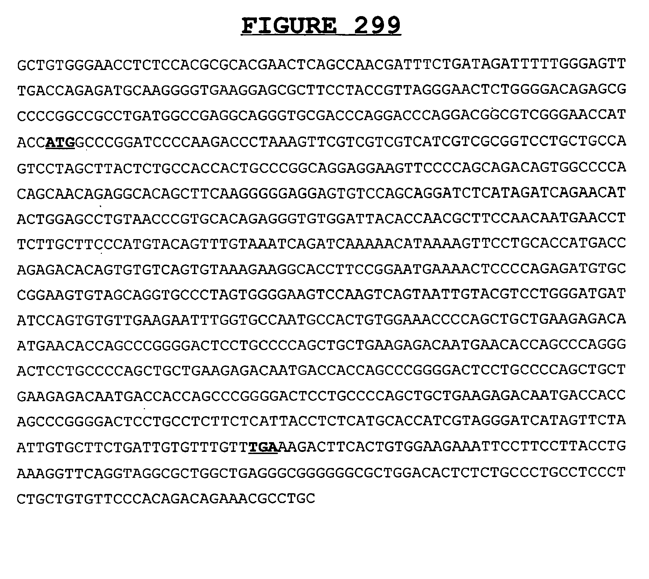

Figure 299 shows a nucleotide sequence (SEQ ID NO:299) of a native sequence PR0366 cDNA, wherein SEQ ID NO:299 is a clone designated herein as "DNA33085-1110".

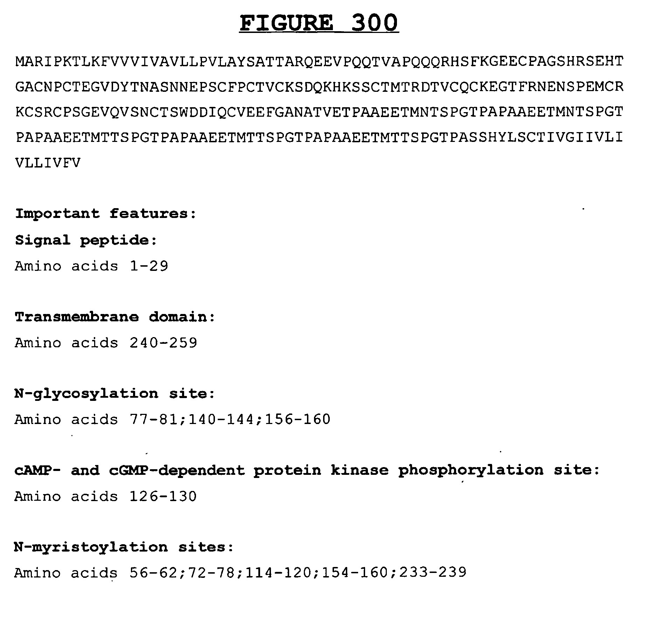

Figure 300 shows the amino acid sequence (SEQ ID NO:300) derived from the coding sequence of SEQ ID NO: 299 shown in Figure 299.

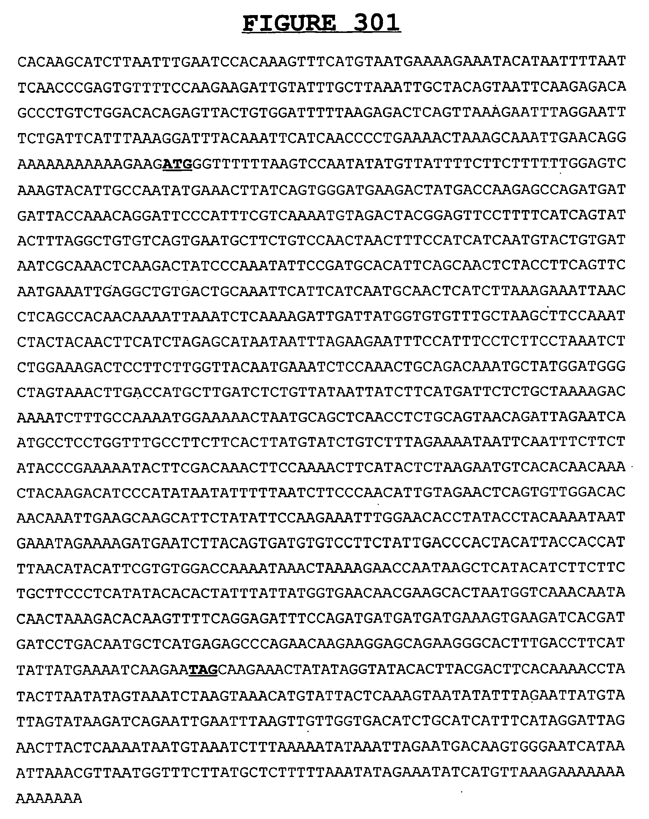

Figure 301 shows a nucleotide sequence (SEQ ID NO:301) of a native sequence PR0216 cDNA, wherein SEQ ID NO:301 is a clone designated herein as "DNA33087-1158".

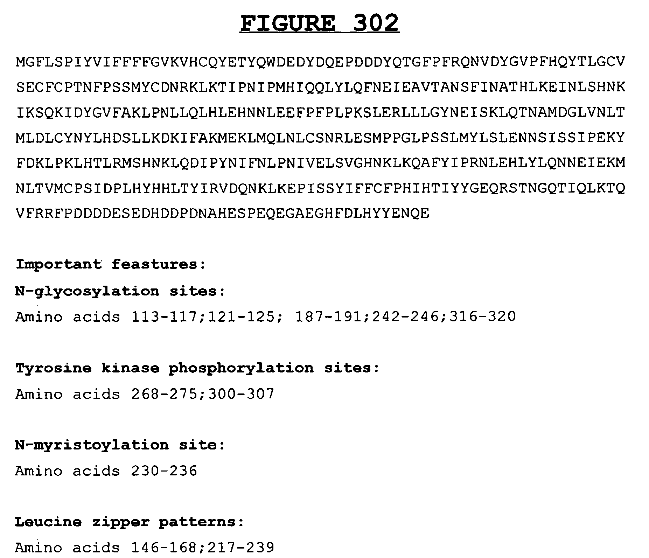

Figure 302 shows the amino acid sequence (SEQ ID NO:302) derived from the coding sequence of SEQ ID NO: 301 shown in Figure 301.

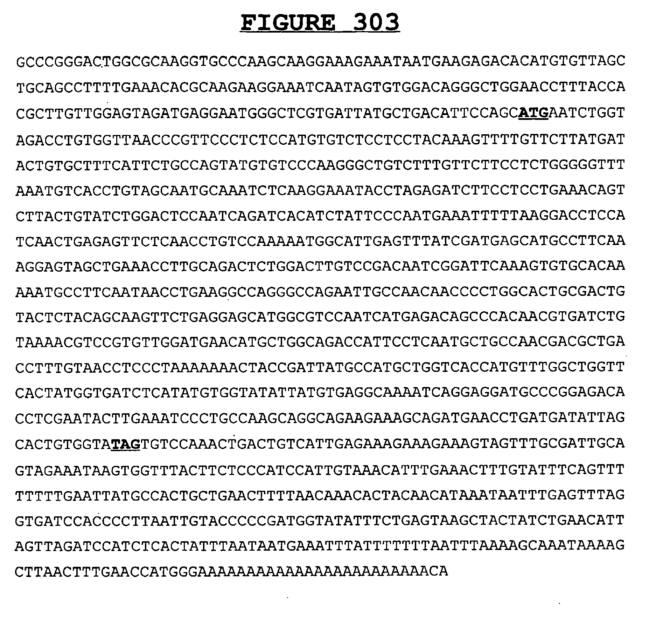

Figure 303 shows a nucleotide sequence (SEQ ID NO:303) of a native sequence PR0221 cDNA, wherein SEQ ID NO:303 is a clone designated herein as "DNA33089-1132".

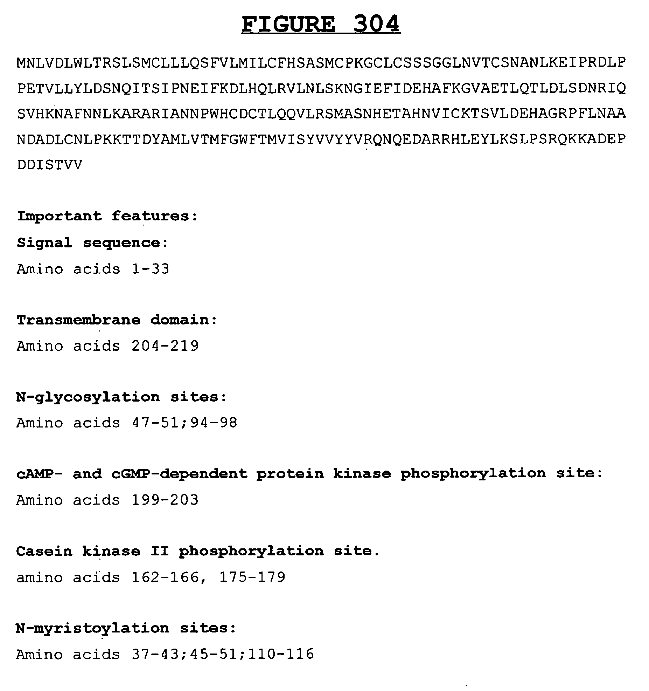

Figure 304 shows the amino acid sequence (SEQ ID NO:304) derived from the coding sequence of SEQ ID NO: 303 shown in Figure 303.

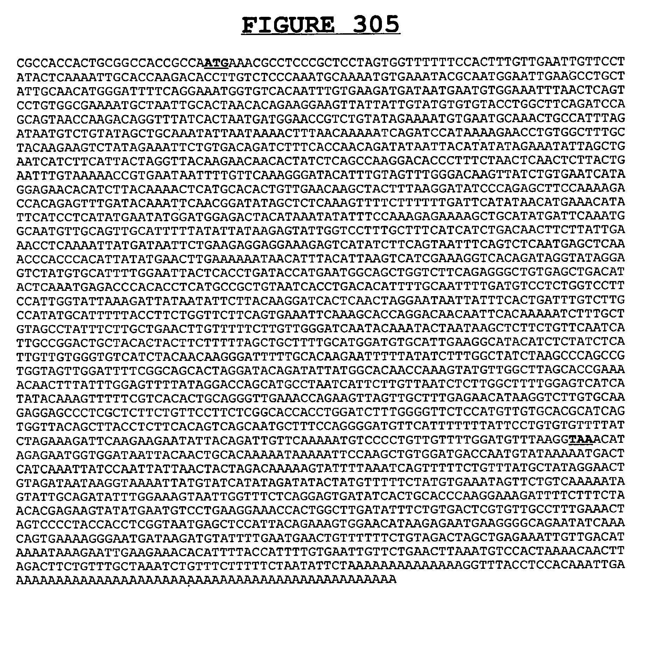

Figure 305 shows a nucleotide sequence (SEQ ID NO:305) of a native sequence PR0228 cDNA, wherein SEQ ID NO:305 is a clone designated herein as "DNA33092-1202".

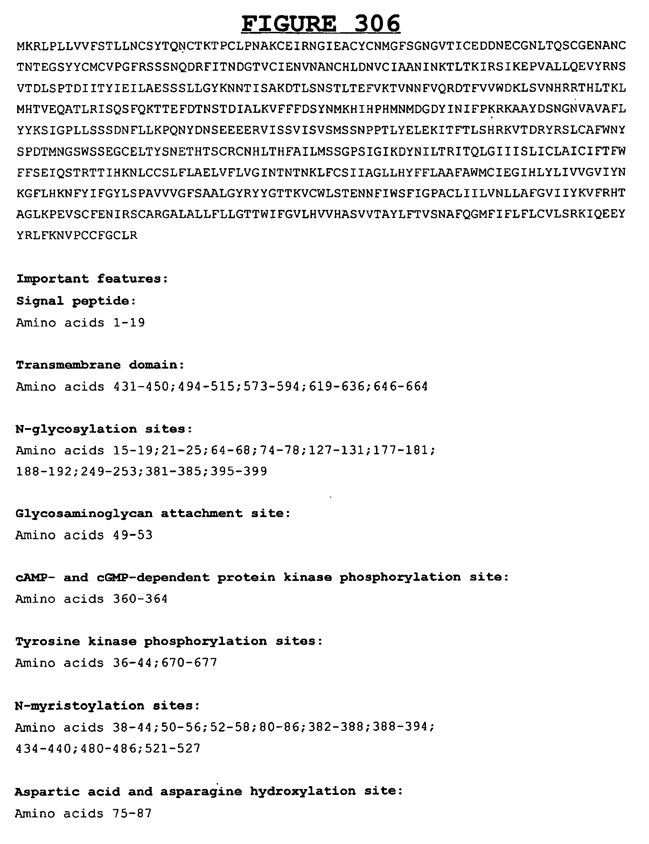

Figure 306 shows the amino acid sequence (SEQ ID NO:306) derived from the coding sequence of SEQ ID NO: 305 shown in Figure 305.

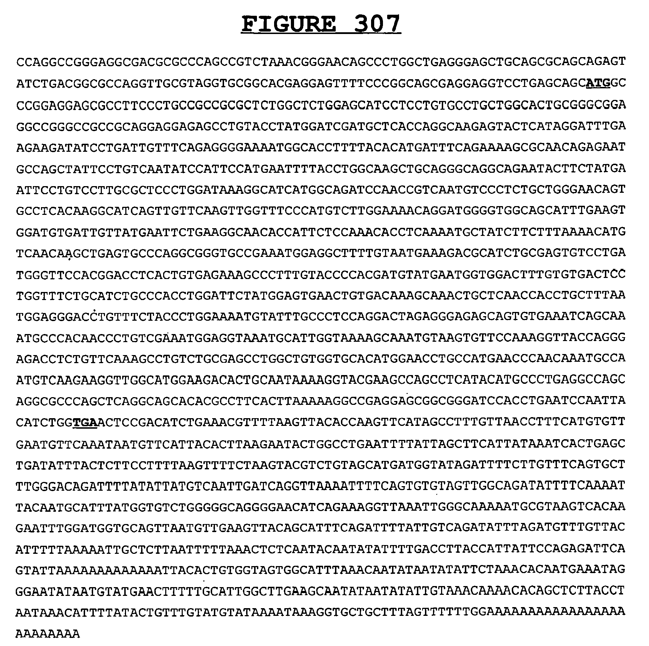

Figure 307 shows a nucleotide sequence (SEQ ID NO:307) of a native sequence PR0217 cDNA, wherein SEQ ID NO:307 is a clone designated herein as "DNA33094-1131".

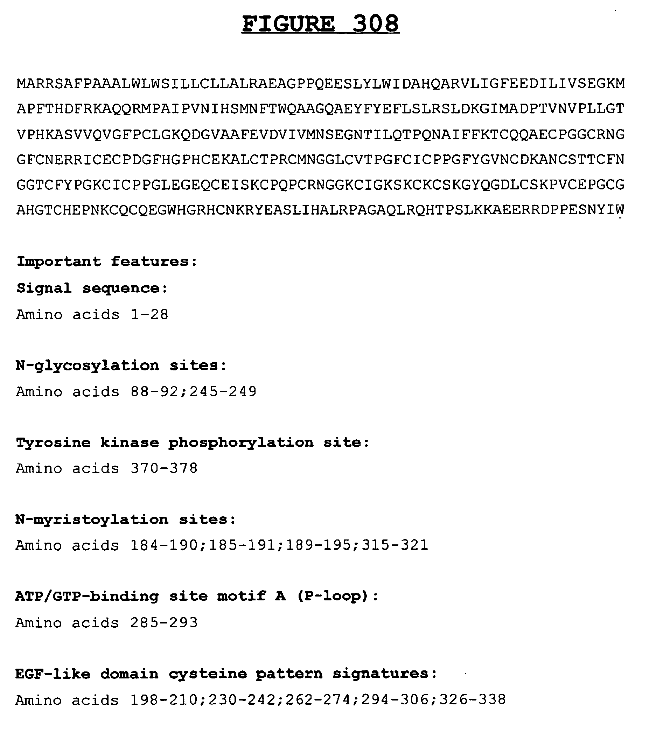

Figure 308 shows the amino acid sequence (SEQ ID NO:308) derived from the coding sequence of SEQ ID NO: 307 shown in Figure 307.

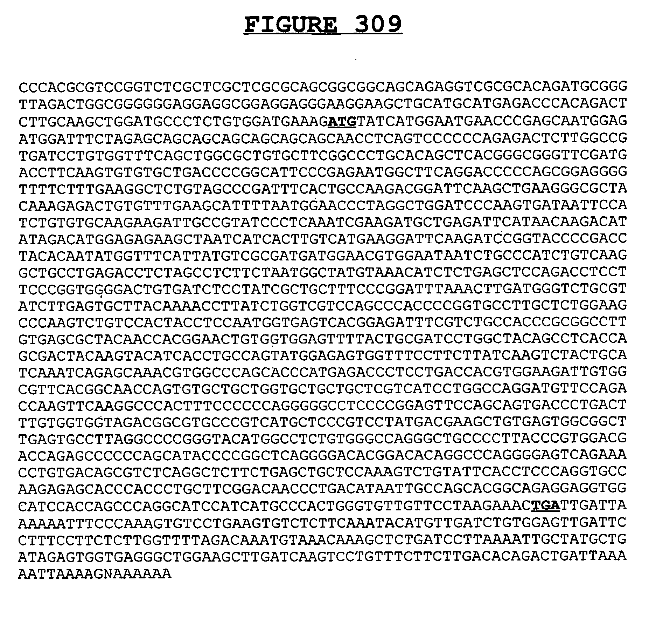

Figure 309 shows a nucleotide sequence (SEQ ID NO:309) of a native sequence PR0222 cDNA, wherein SEQ ID NO:309 is a clone designated herein as "DNA33107-1135".

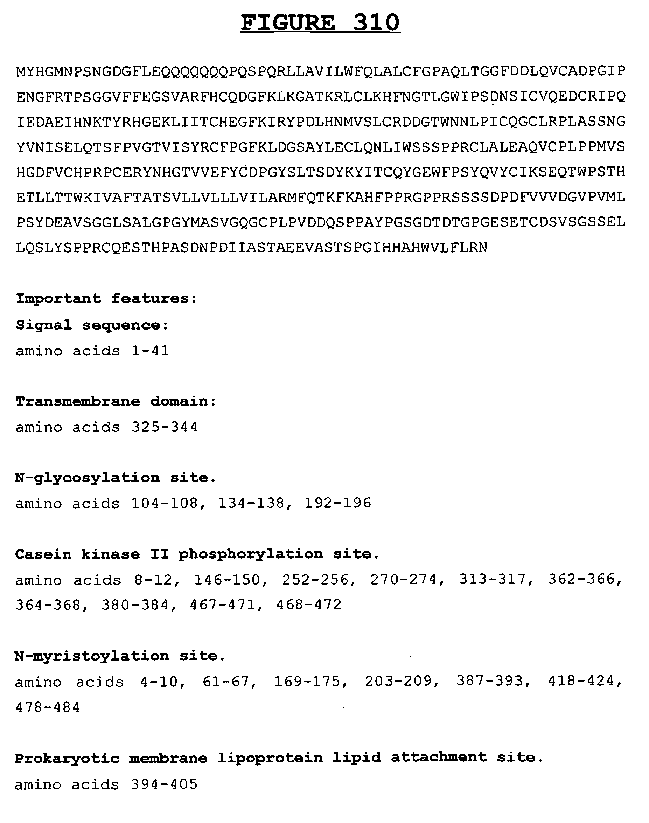

Figure 310 shows the amino acid sequence (SEQ ID NO:310) derived from the coding sequence of SEQ ID NO: 309 shown in Figure 309.

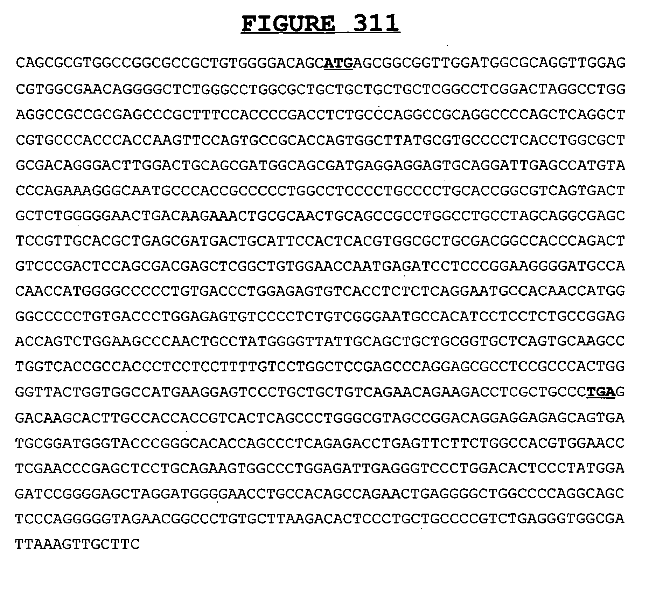

Figure 311 shows a nucleotide sequence (SEQ ID NO:311) of a native sequence PR0224 cDNA, wherein SEQ ID NO:311 is a clone designated herein as "DNA33221-1133".

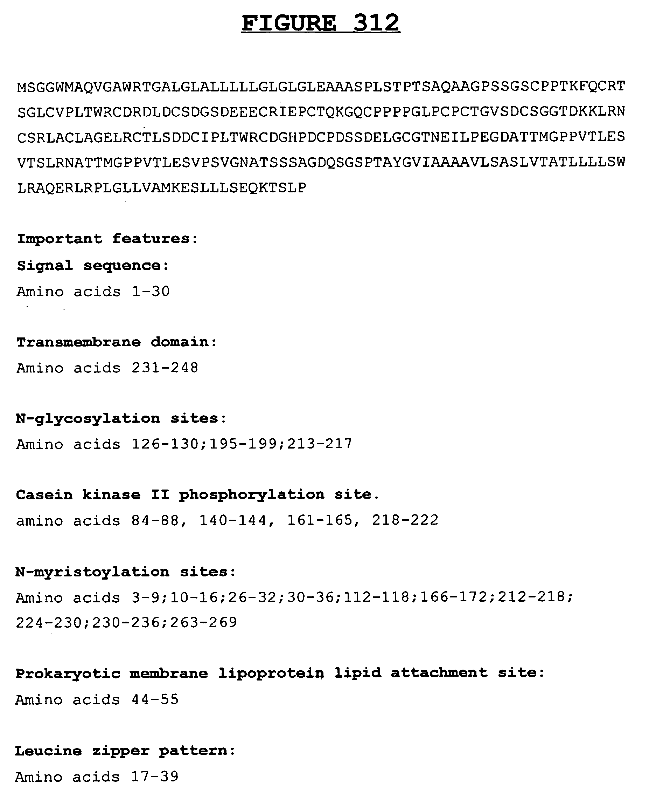

Figure 312 shows the amino acid sequence (SEQ ID NO:312) derived from the coding sequence of SEQ ID NO: 311 shown in Figure 311.

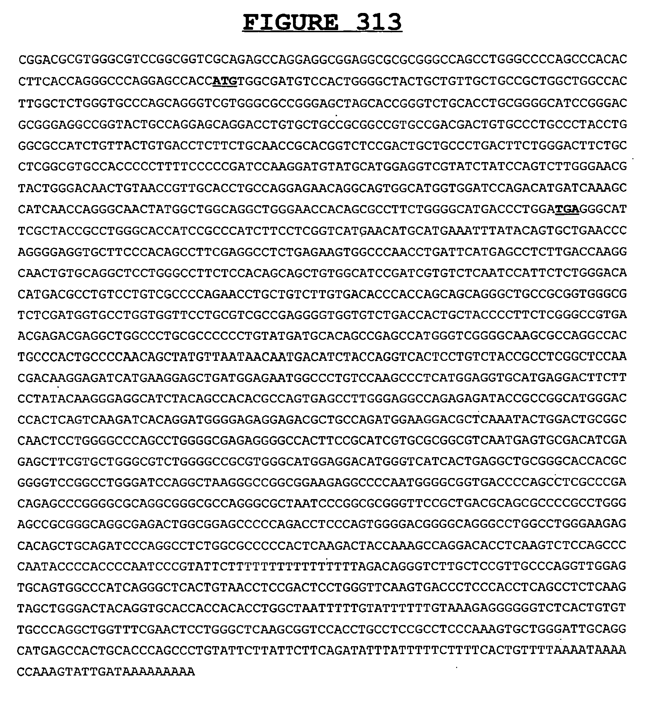

Figure 313 shows a nucleotide sequence (SEQ ID NO:313) of a native sequence PR0230 cDNA, wherein SEQ ID NO:313 is a clone designated herein as "DNA33223-1136".

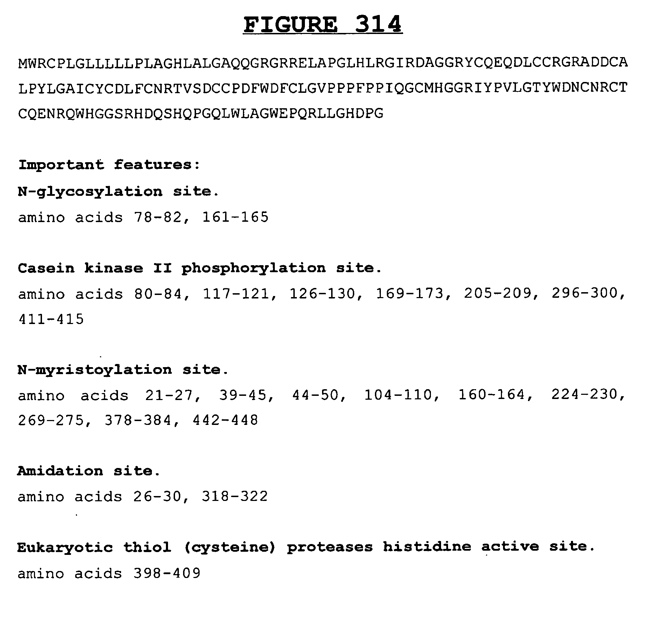

Figure 314 shows the amino acid sequence (SEQ ID NO:314) derived from the coding sequence of SEQ ID NO: 313 shown in Figure 313.

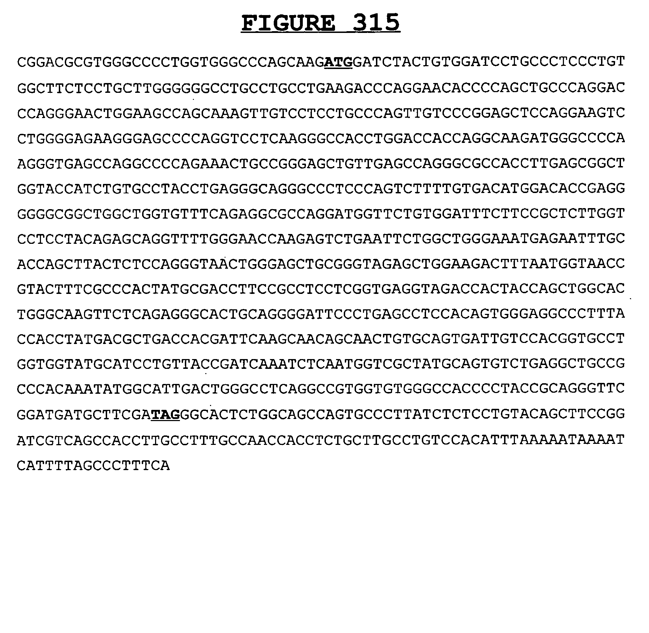

Figure 315 shows a nucleotide sequence (SEQ ID NO:315) of a native sequence PRO198 cDNA, wherein SEQ ID NO:315 is a clone designated herein as "DNA33457-1078".

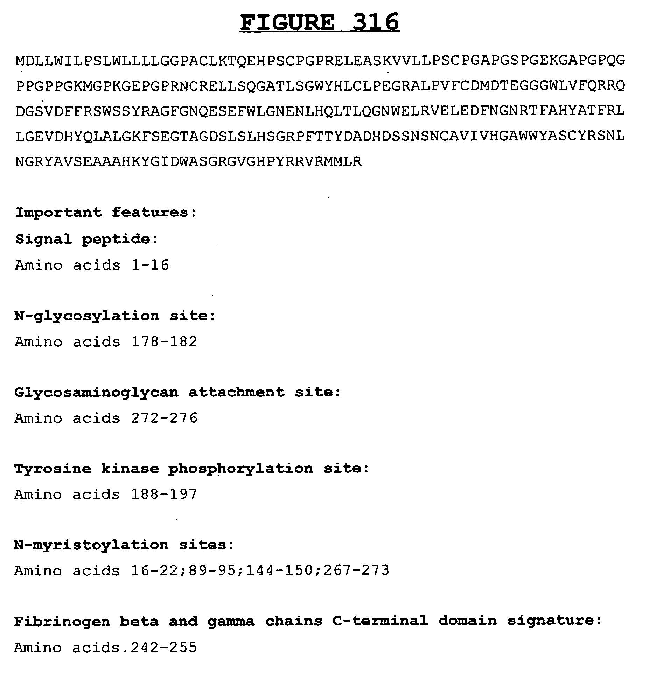

Figure 316 shows the amino acid sequence (SEQ ID NO:316) derived from the coding sequence of SEQ ID NO: 315 shown in Figure 315.

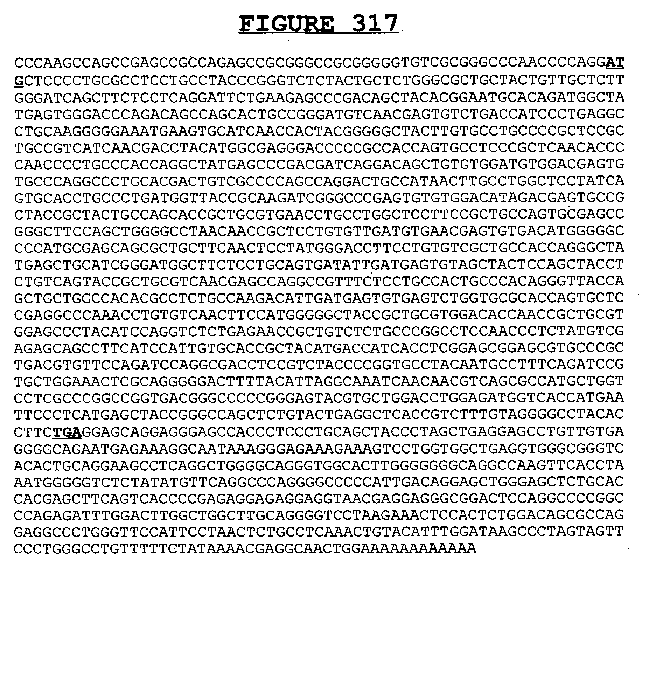

Figure 317 shows a nucleotide sequence (SEQ ID NO:317) of a native sequence PR0226 cDNA, wherein SEQ ID NO:317 is a clone designated herein as "DNA33460-1166".

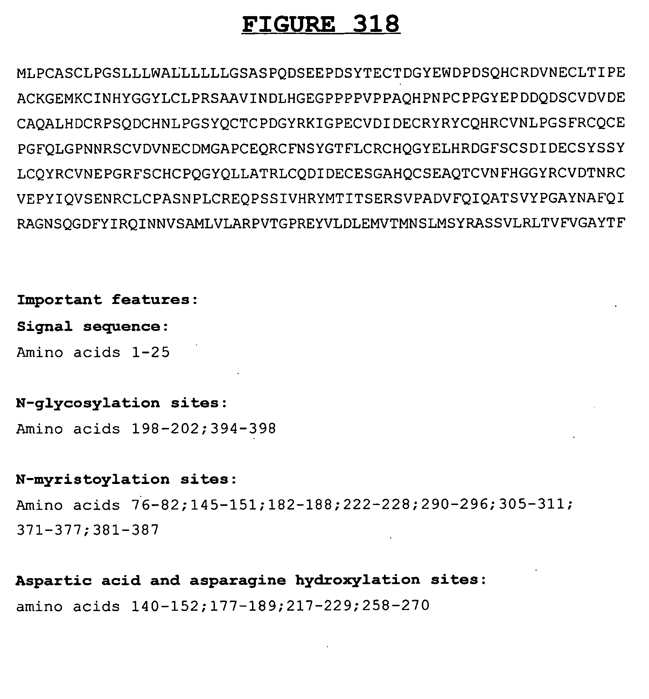

Figure 318 shows the amino acid sequence (SEQ ID NO:318) derived from the coding sequence of SEQ ID NO: 317 shown in Figure 317.

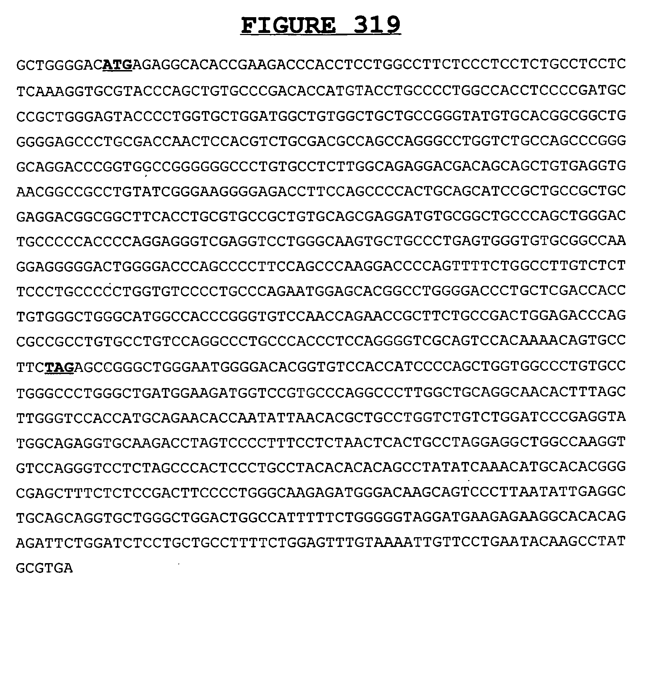

Figure 319 shows a nucleotide sequence (SEQ ID NO:319) of a native sequence PR0261 cDNA, wherein SEQ ID NO:319 is a clone designated herein as "DNA33473-1176".

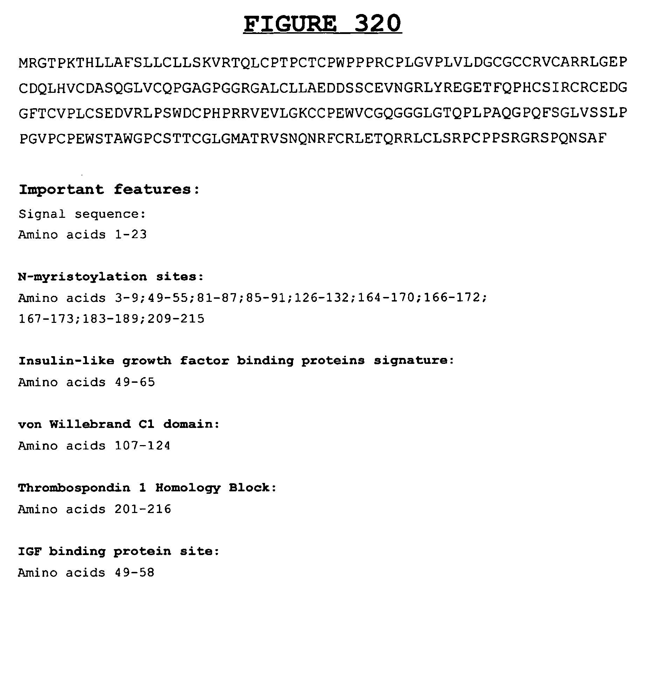

Figure 320 shows the amino acid sequence (SEQ ID NO:320) derived from the coding sequence of SEQ ID NO: 319 shown in Figure 319.

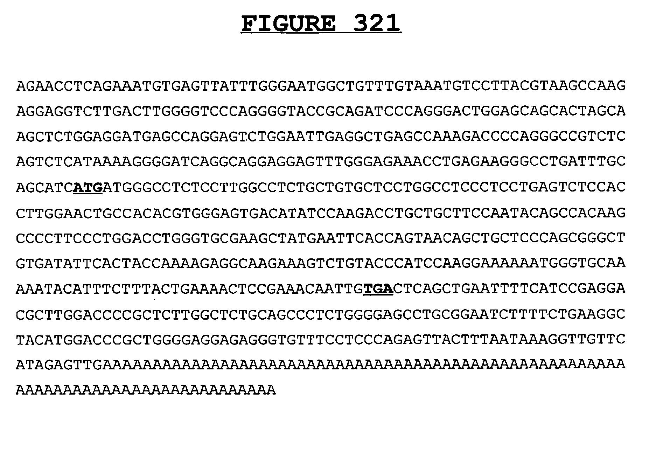

Figure 321 shows a nucleotide sequence (SEQ ID NO:321) of a native sequence PRO242 cDNA, wherein SEQ ID NO:321 is a clone designated herein as "DNA33785-1143".

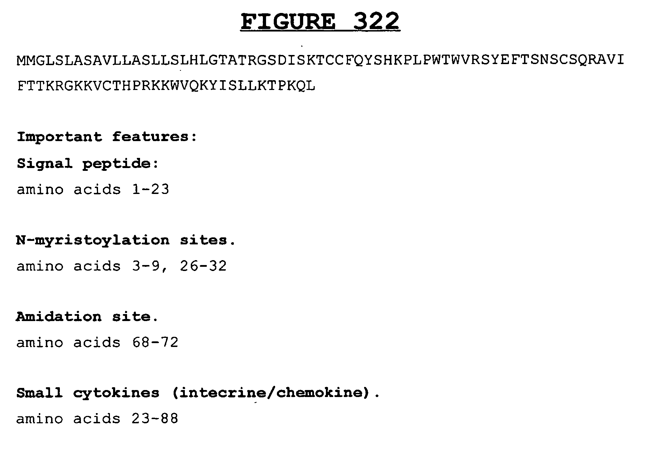

Figure 322 shows the amino acid sequence (SEQ ID NO:322) derived from the coding sequence of SEQ ID NO: 321 shown in Figure 321.

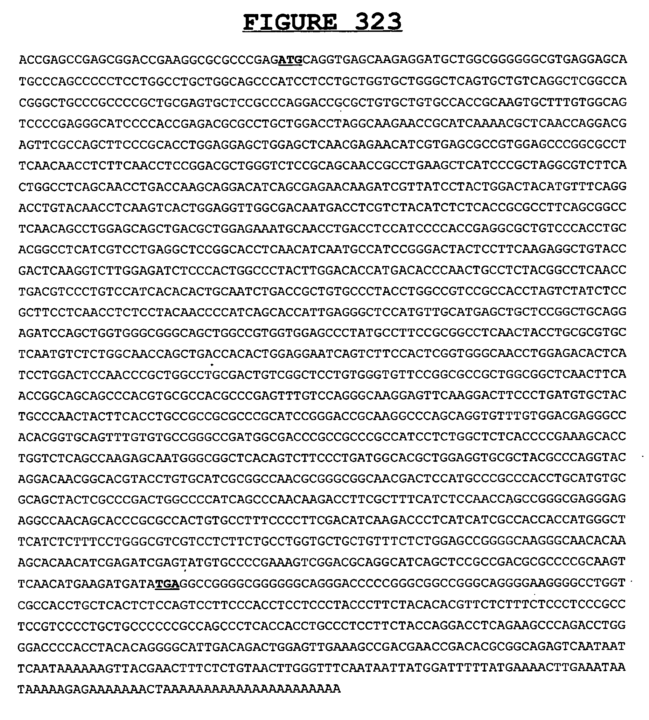

Figure 323 shows a nucleotide sequence (SEQ ID NO:323) of a native sequence PR0227 cDNA, wherein SEQ ID NO:323 is a clone designated herein as "DNA33786-1132".

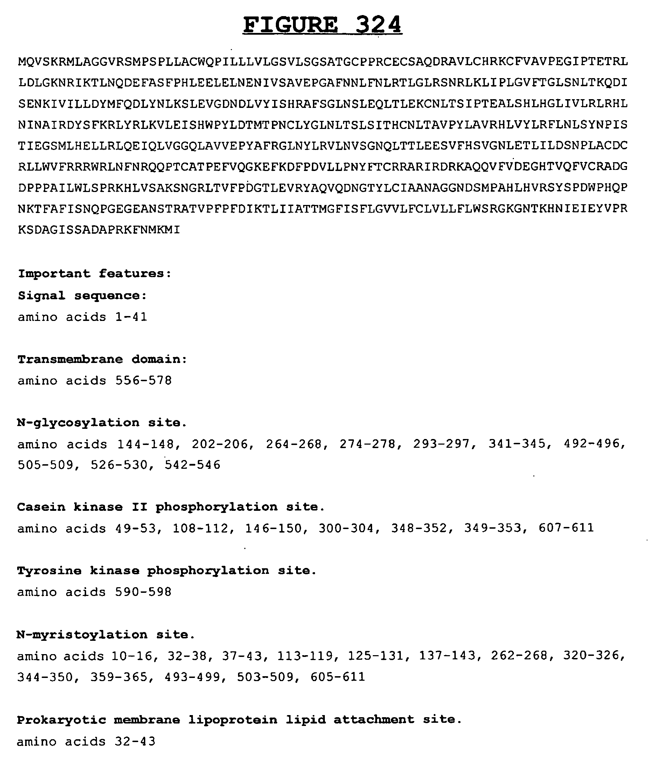

Figure 324 shows the amino acid sequence (SEQ ID NO:324) derived from the coding sequence of SEQ ID NO: 323 shown in Figure 323.

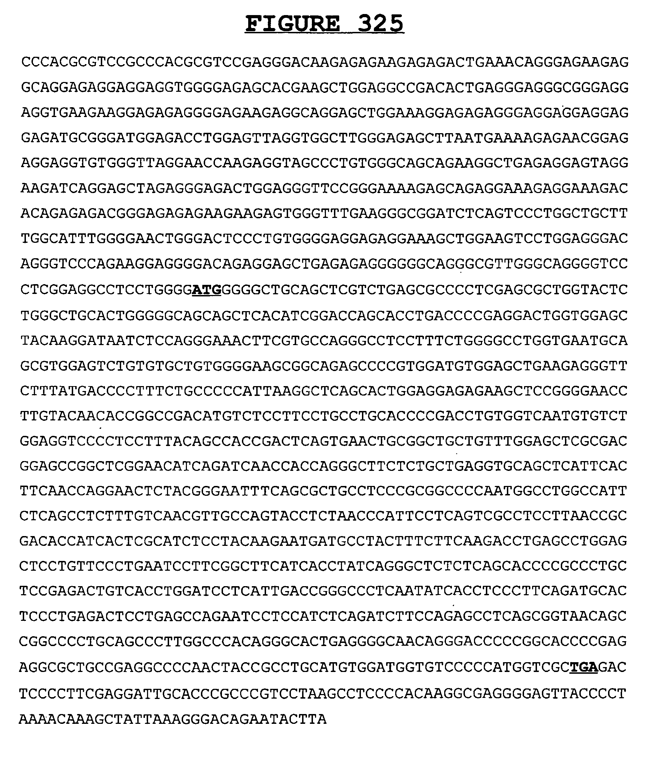

Figure 325 shows a nucleotide sequence (SEQ ID NO:325) of a native sequence PR0237 cDNA, wherein SEQ ID NO:325 is a clone designated herein as "DIVA34353-1428".

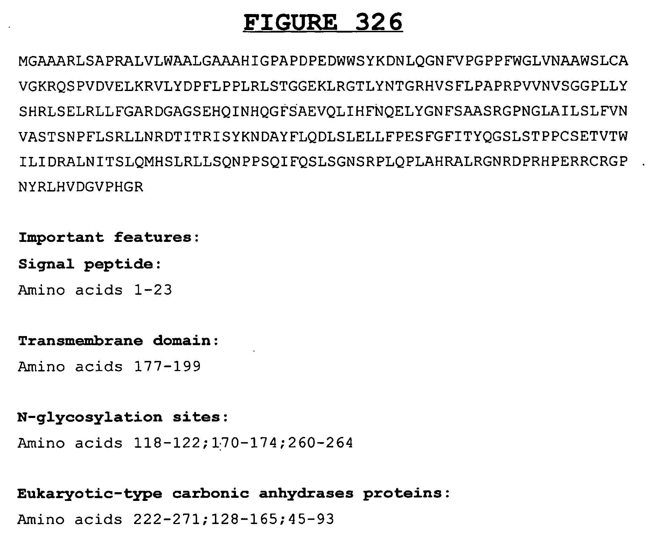

Figure 326 shows the amino acid sequence (SEQ ID NO:326) derived from the coding sequence of SEQ ID NO: 325 shown in Figure 325.

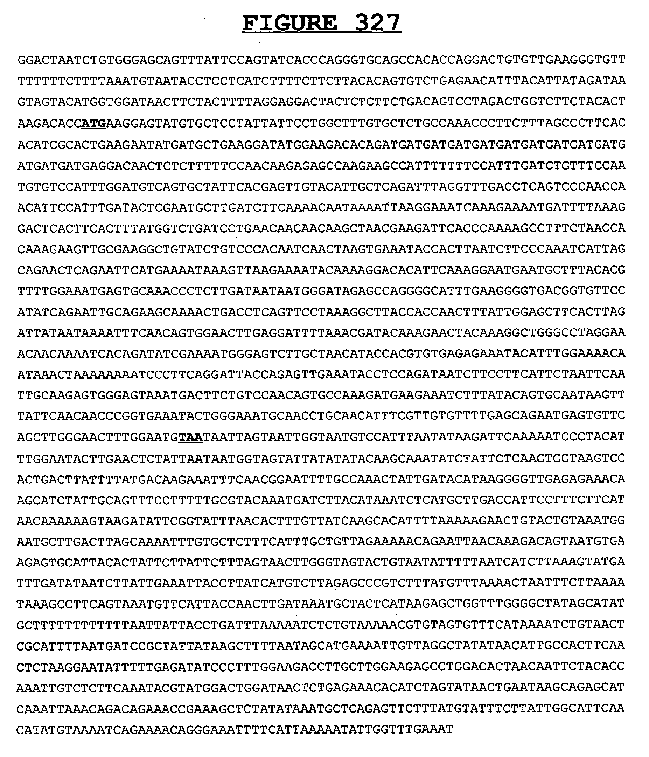

Figure 327 shows a nucleotide sequence (SEQ ID NO:327) of a native sequence PR0241 cDNA, wherein SEQ ID NO:327 is a clone designated herein as "DNA34392-1170".

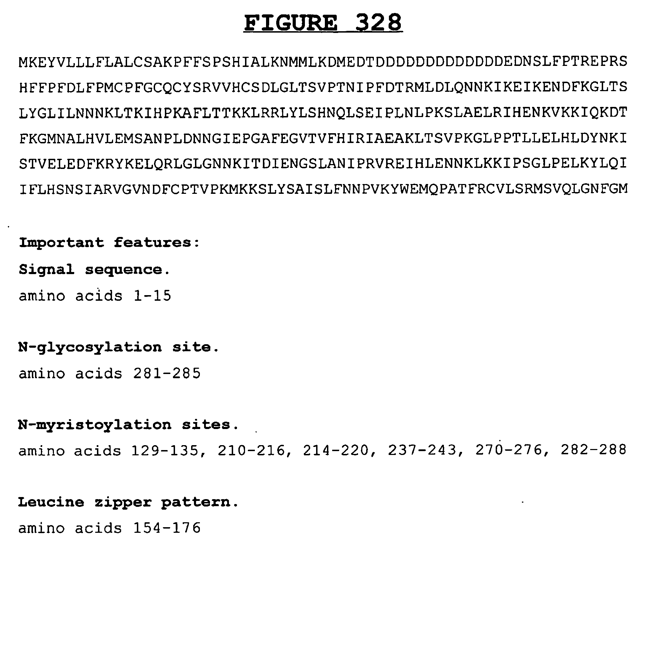

Figure 328 shows the amino acid sequence (SEQ ID NO:328) derived from the coding sequence of SEQ ID NO: 327 shown in Figure 327.

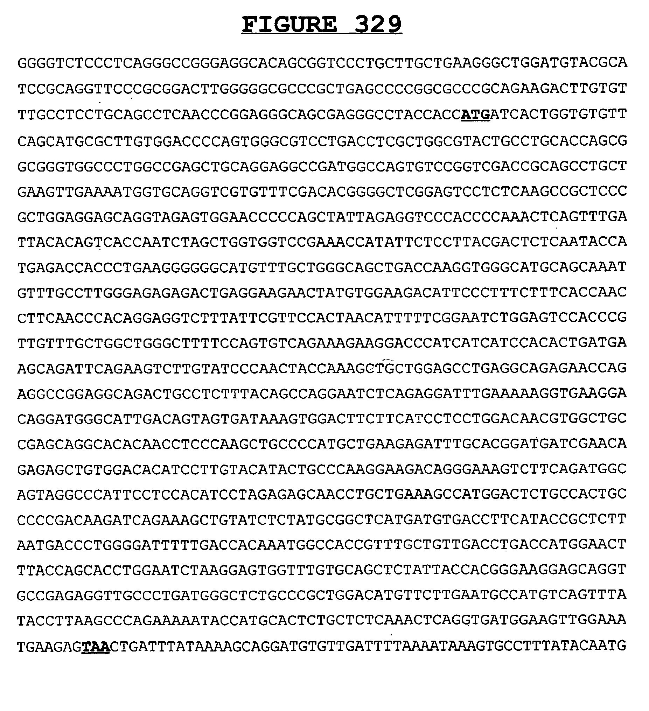

Figure 329 shows a nucleotide sequence (SEQ ID NO:329) of a native sequence PR0231 cDNA, wherein SEQ ID NO:329 is a clone designated herein as "DNA34434-1139".

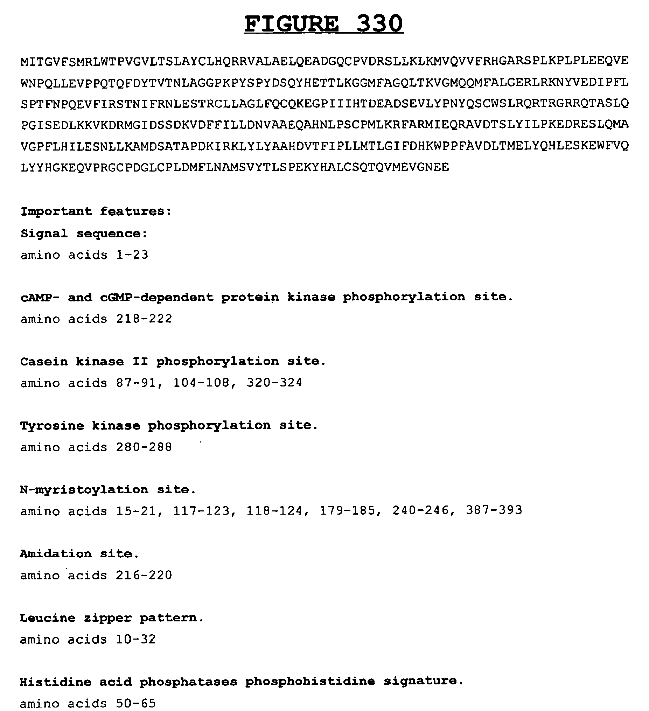

Figure 330 shows the amino acid sequence (SEQ ID NO:330) derived from the coding sequence of SEQ ID NO: 329 shown in Figure 329.

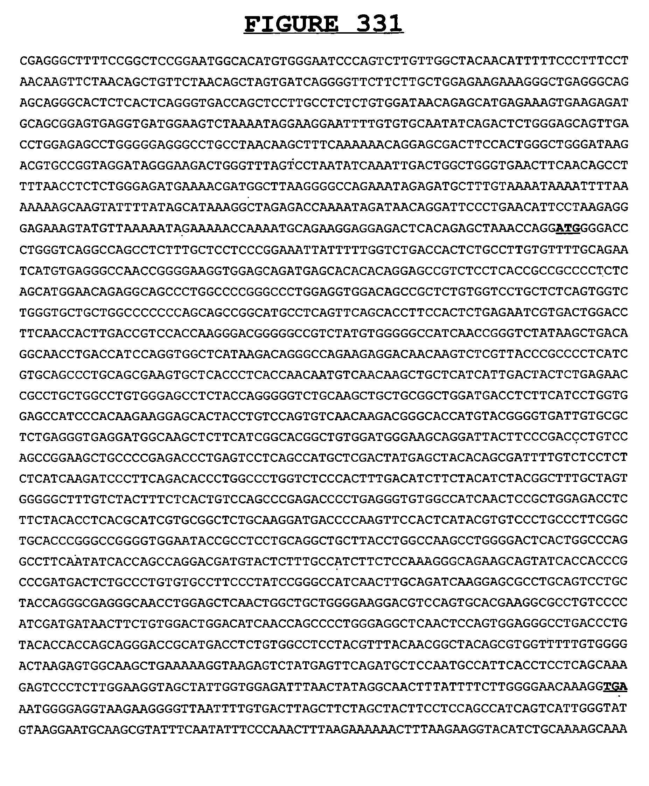

Figure 331 shows a nucleotide sequence (SEQ ID NO:331) of a native sequence PR0235 cDNA, wherein SEQ ID NO:331 is a clone designated herein as "DNA35558-1167".

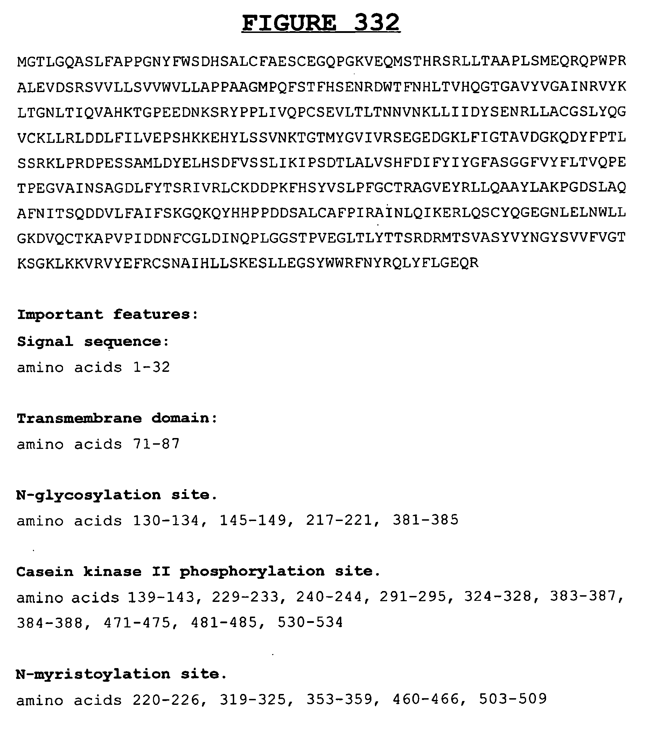

Figure 332 shows the amino acid sequence (SEQ ID NO:332) derived from the coding sequence of SEQ ID NO: 331 shown in Figure 331.

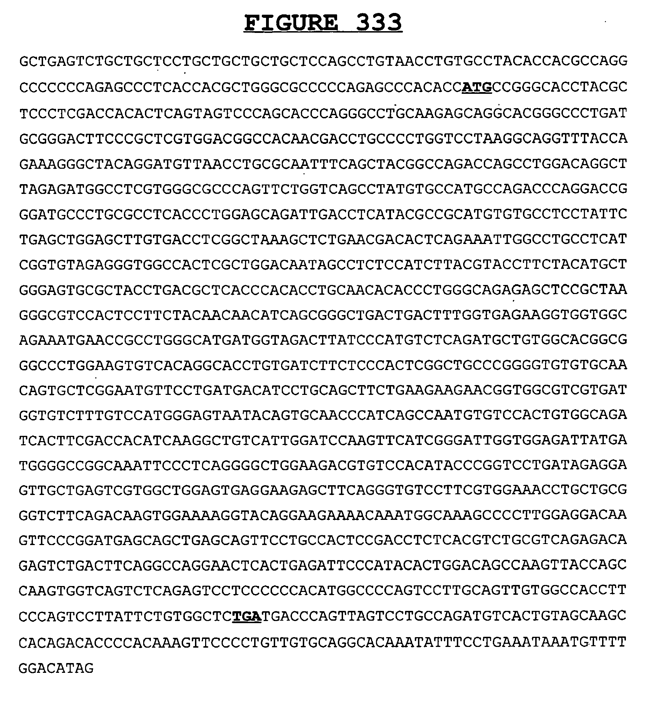

Figure 333 shows a nucleotide sequence (SEQ ID NO:333) of a native sequence PR0323 cDNA, wherein SEQ ID NO:333 is a clone designated herein as "DNA35595-1228".

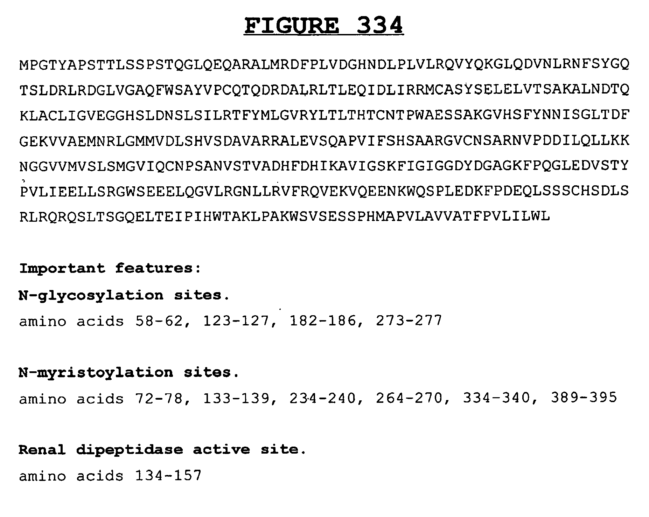

Figure 334 shows the amino acid sequence (SEQ ID NO:334) derived from the coding sequence of SEQ ID NO: 333 shown in Figure 333.

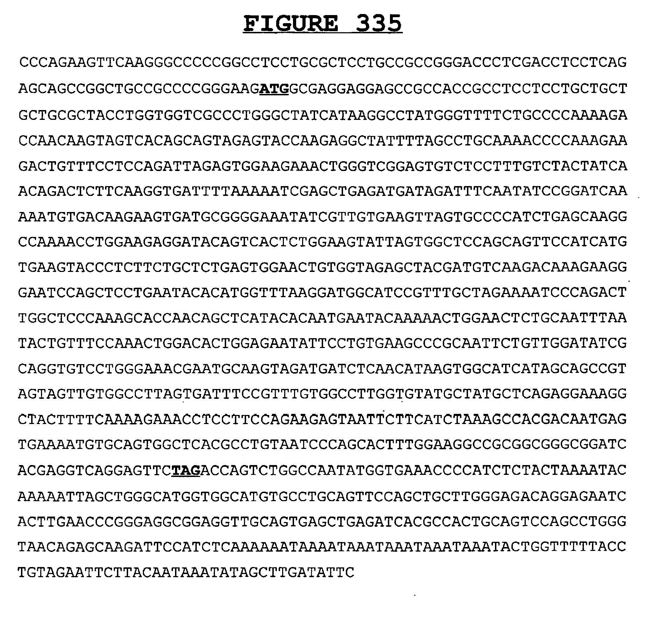

Figure 335 shows a nucleotide sequence (SEQ ID NO:335) of a native sequence PR0245 cDNA, wherein SEQ ID NO:335 is a clone designated herein as "DNA35638-1216".

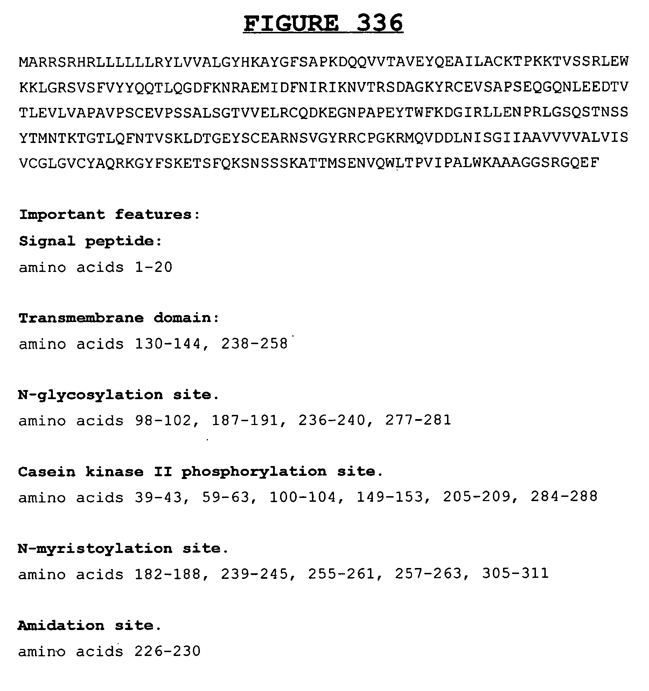

Figure 336 shows the amino acid sequence (SEQ ID NO:336) derived from the coding sequence of SEQ ID NO: 335 shown in Figure 335.

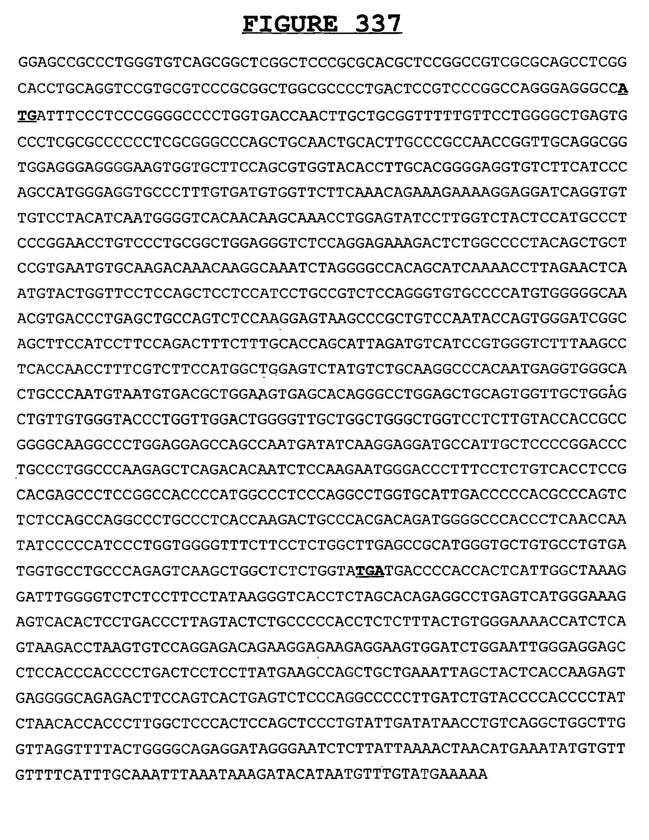

Figure 337 shows a nucleotide sequence (SEQ ID NO:337) of a native sequence PR0246 cDNA, wherein SEQ ID NO:337 is a clone designated herein as "DNA35639-1172".

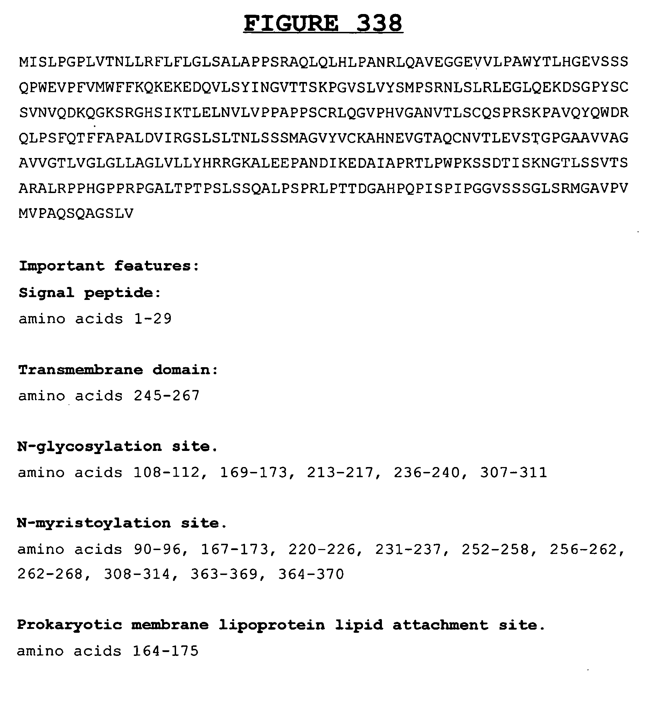

Figure 338 shows the amino acid sequence (SEQ ID NO:338) derived from the coding sequence of SEQ ID NO: 337 shown in Figure 337.

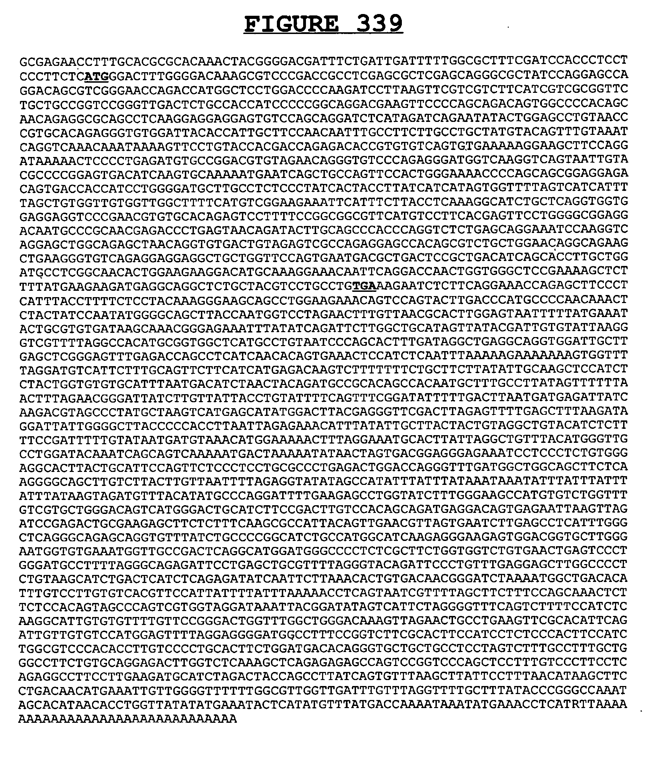

Figure 339 shows a nucleotide sequence (SEQ ID NO:339) of a native sequence PR0288 cDNA, wherein SEQ ID NO:339 is a clone designated herein as "DNA35663-1129".

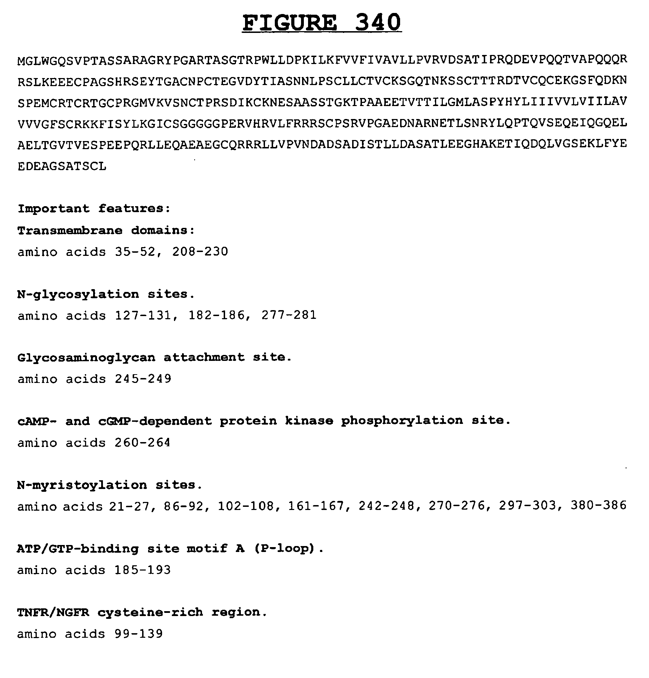

Figure 340 shows the amino acid sequence (SEQ ID NO:340) derived from the coding sequence of SEQ ID NO: 339 shown in Figure 339.

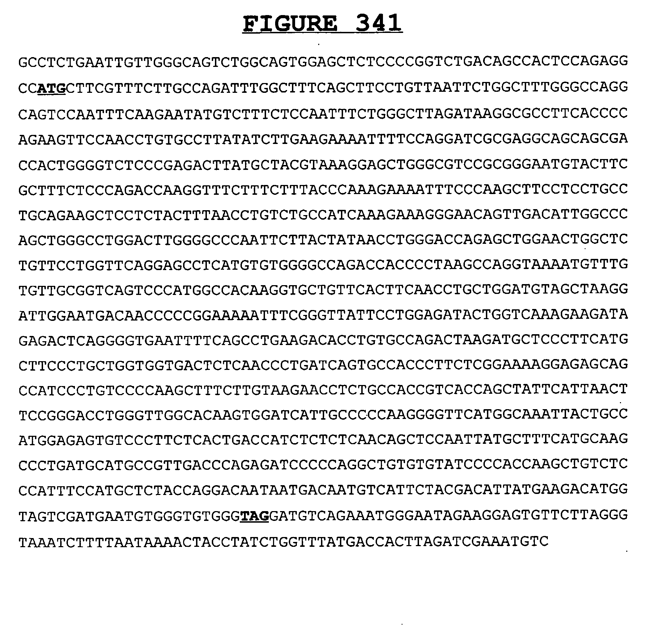

Figure 341 shows a nucleotide sequence (SEQ ID NO:341) of a native sequence PR0248 cDNA, wherein SEQ ID NO:341 is a clone designated herein as "DNA35674-1142".

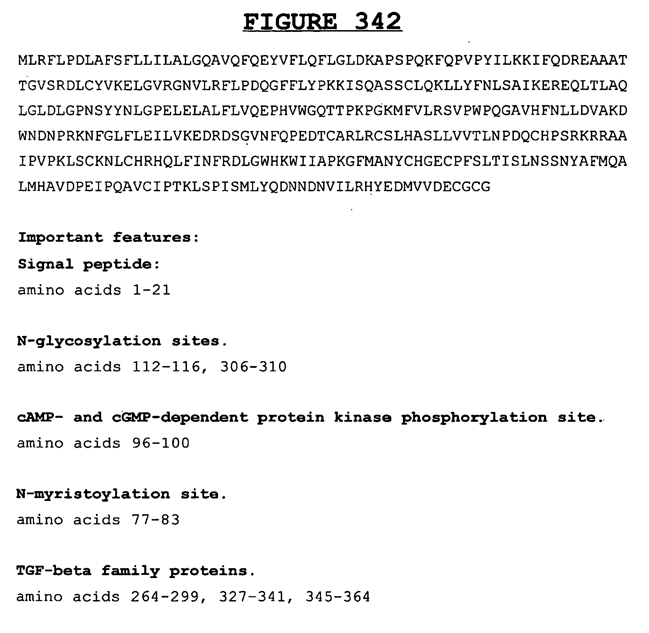

Figure 342 shows the amino acid sequence (SEQ ID NO:342) derived from the coding sequence of SEQ ID NO: 341 shown in Figure 341.

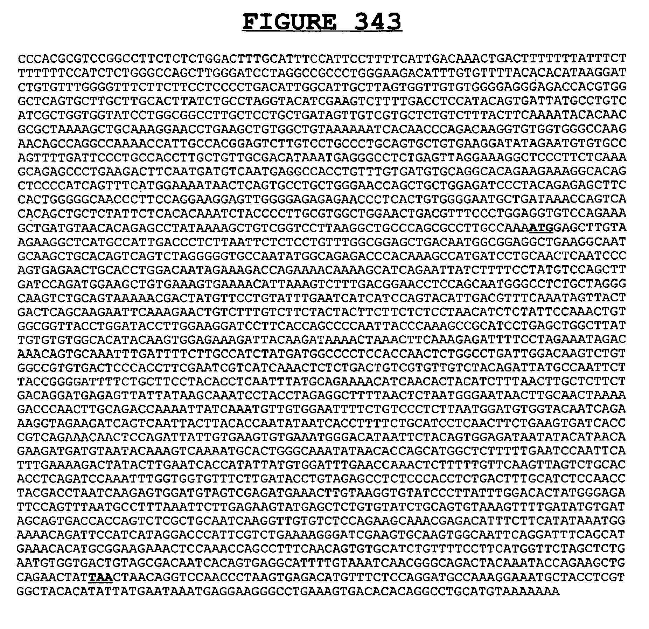

Figure 343 shows a nucleotide sequence (SEQ ID NO:343) of a native sequence PR0257 cDNA, wherein SEQ ID NO:343 is a clone designated herein as "DNA35841-1173".

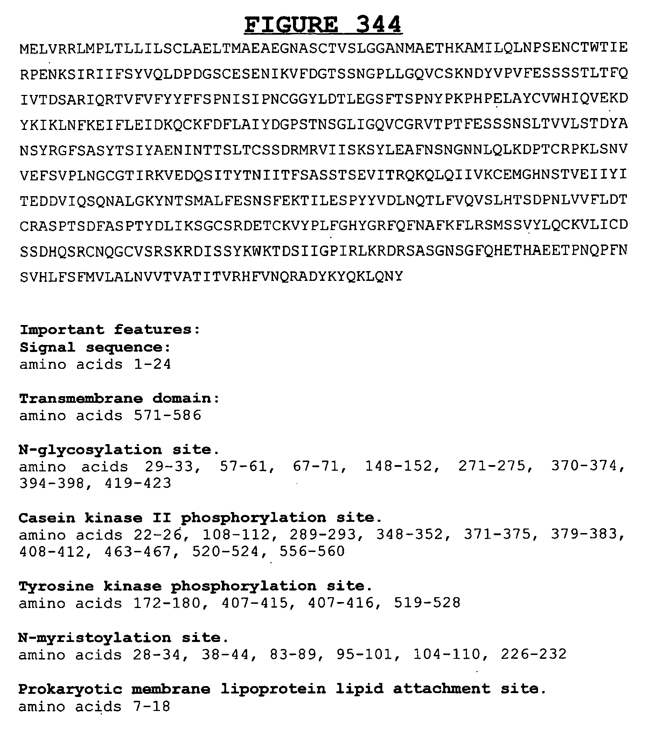

Figure 344 shows the amino acid sequence (SEQ ID NO:344) derived from the coding sequence of SEQ ID NO: 343 shown in Figure 343.

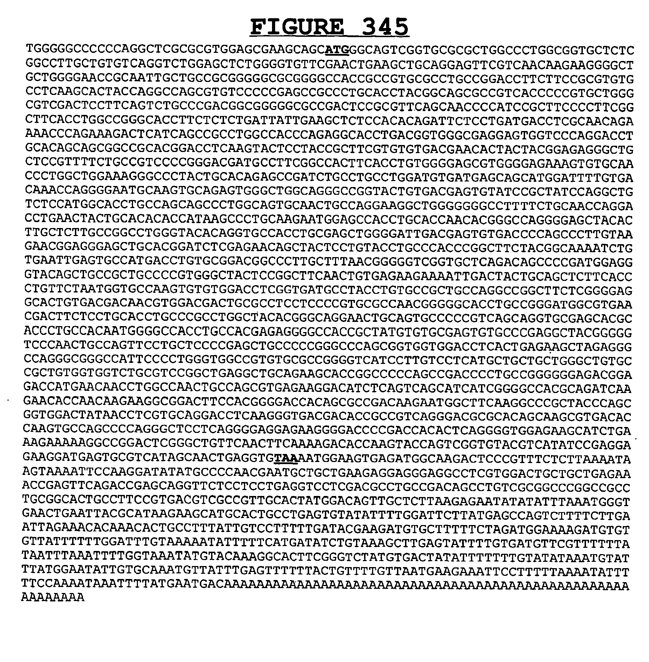

Figure 345 shows a nucleotide sequence (SEQ ID NO:345) of a native sequence PRO172 cDNA, wherein SEQ ID NO:345 is a clone designated herein as "DNA35916-1161".

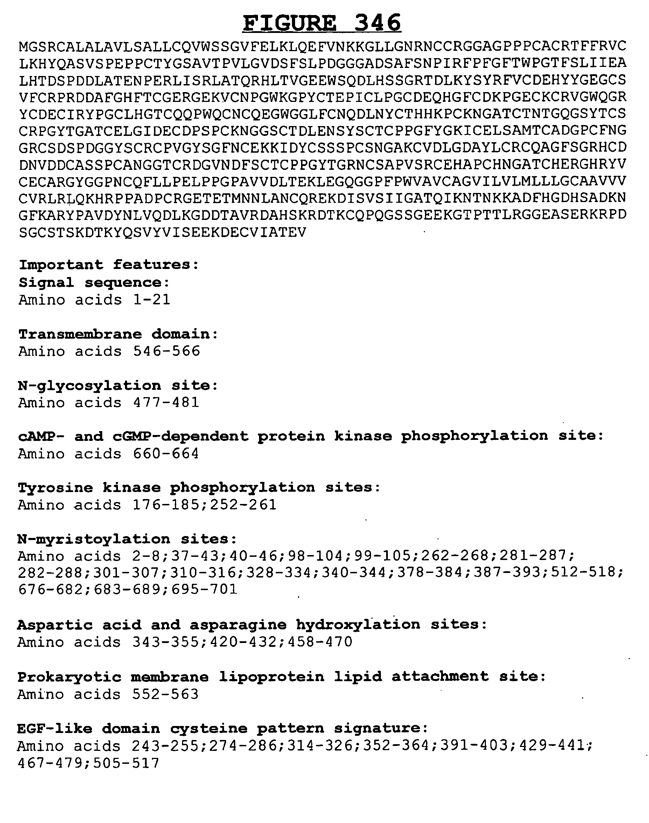

Figure 346 shows the amino acid sequence (SEQ ID NO:346) derived from the coding sequence of SEQ ID NO: 345 shown in Figure 345.

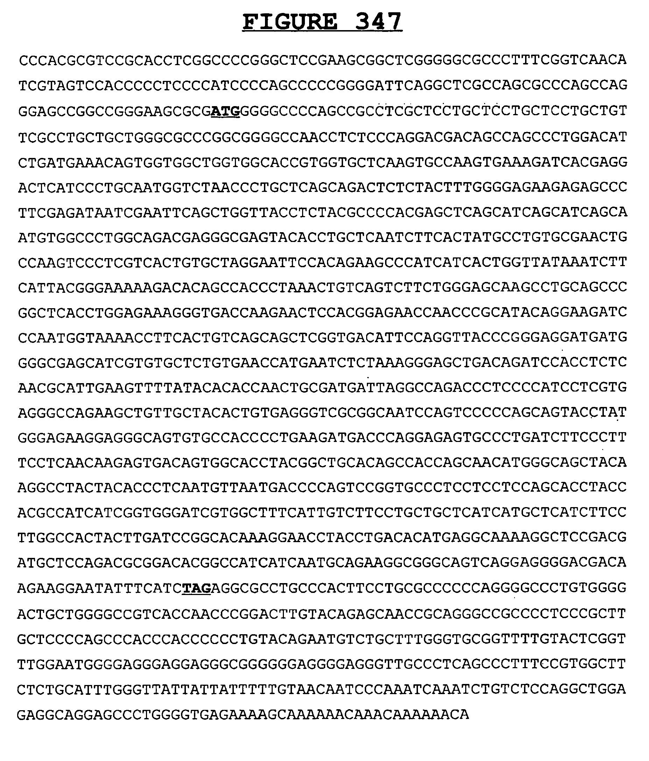

Figure 347 shows a nucleotide sequence (SEQ ID NO:347) of a native sequence PR0258 cDNA, wherein SEQ ID NO:347 is a clone designated herein as "DNA35918-1174".

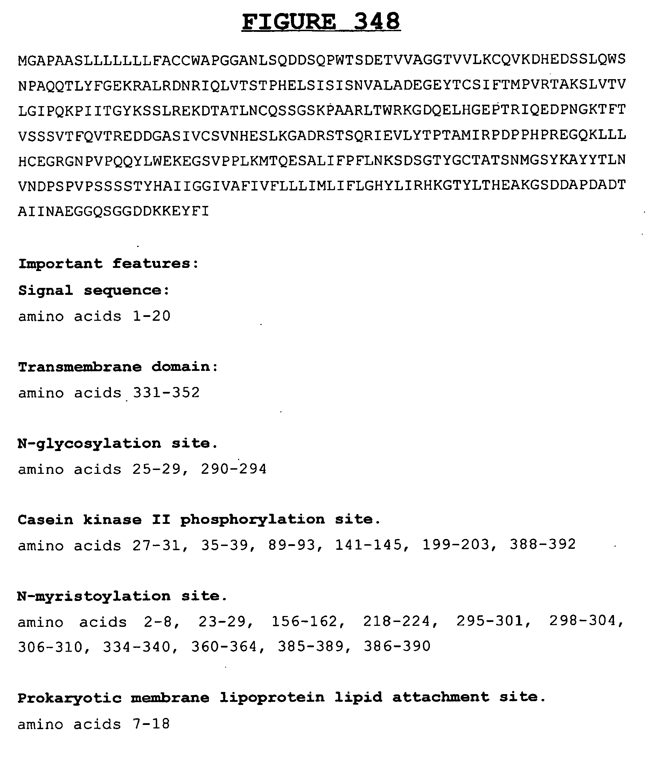

Figure 348 shows the amino acid sequence (SEQ ID NO:348) derived from the coding sequence of SEQ ID NO: 347 shown in Figure 347.

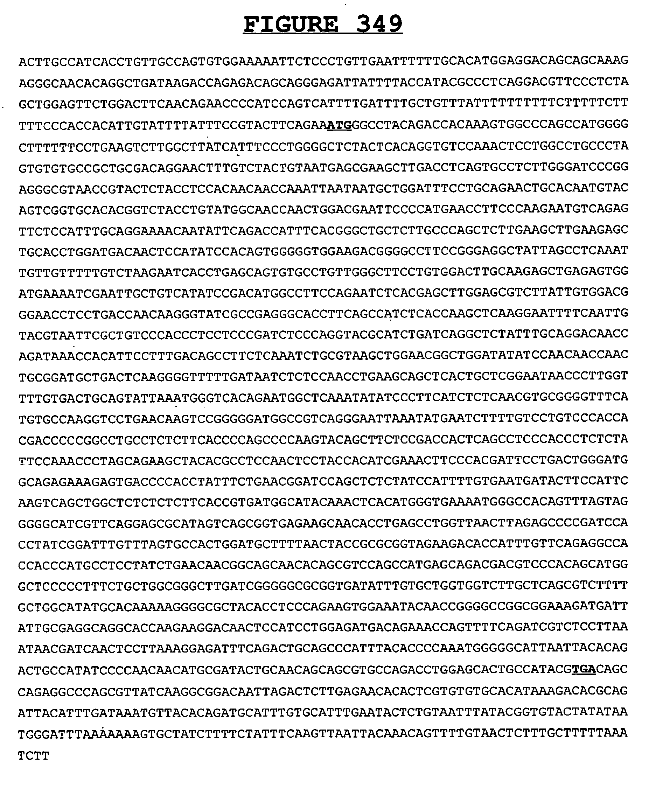

Figure 349 shows a nucleotide sequence (SEQ ID NO:349) of a native sequence PRO265 cDNA, wherein SEQ ID NO:349 is a clone designated herein as "DNA36350-1158".

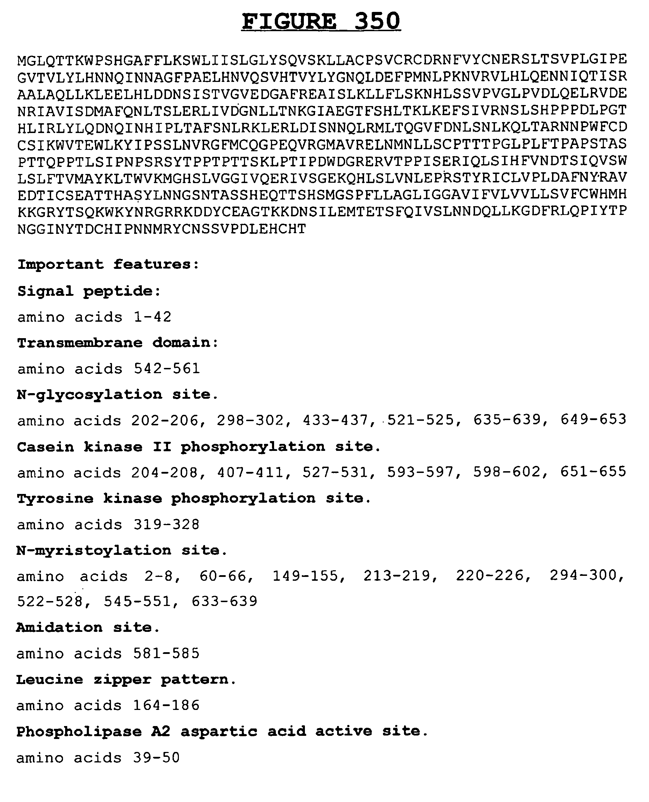

Figure 350 shows the amino acid sequence (SEQ ID NO:350) derived from the coding sequence of SEQ ID NO: 349 shown in Figure 349.

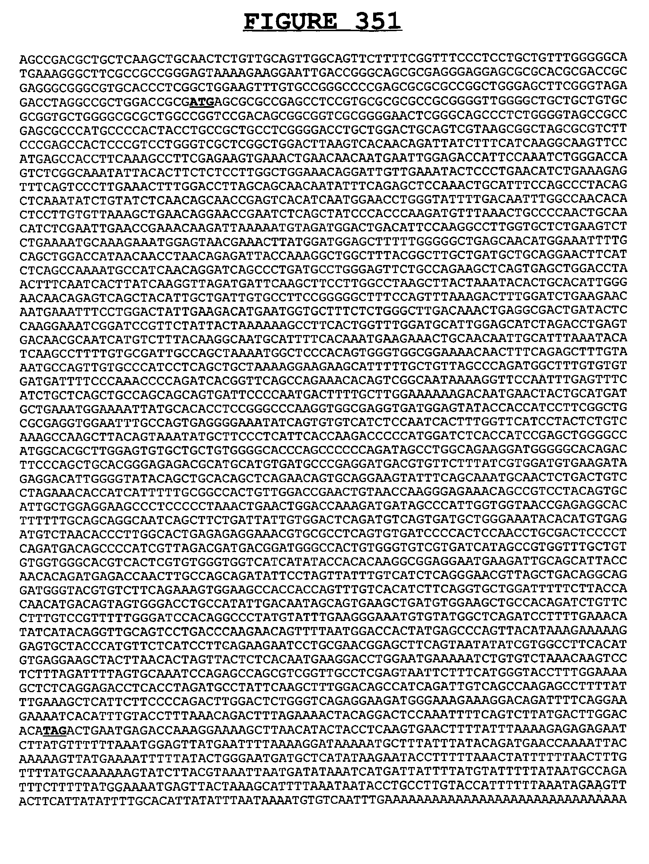

Figure 351 shows a nucleotide sequence (SEQ ID NO:351) of a native sequence PR0326 cDNA, wherein SEQ ID NO:351 is a clone designated herein as "DNA37140-1234".

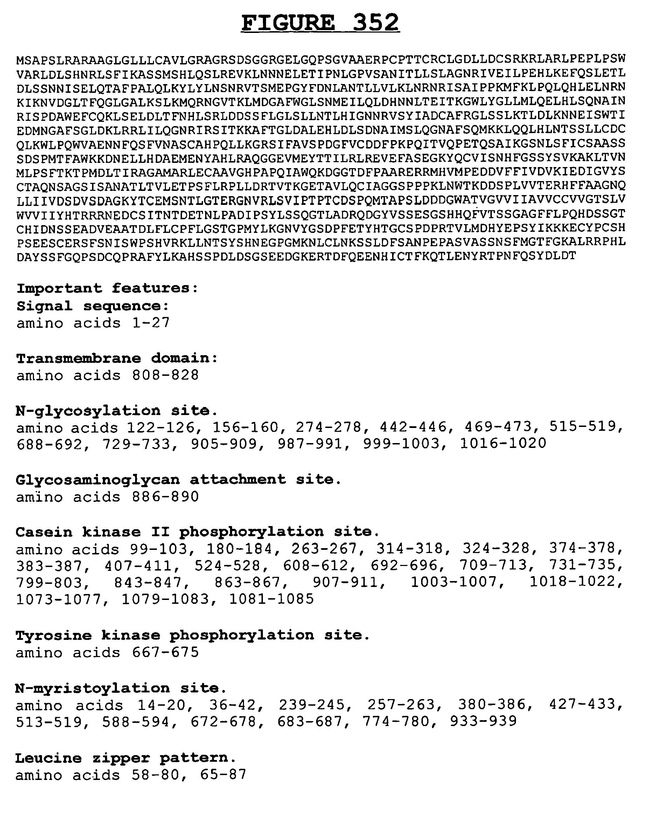

Figure 352 shows the amino acid sequence (SEQ ID NO:352) derived from the coding sequence of SEQ ID NO: 351 shown in Figure 351.

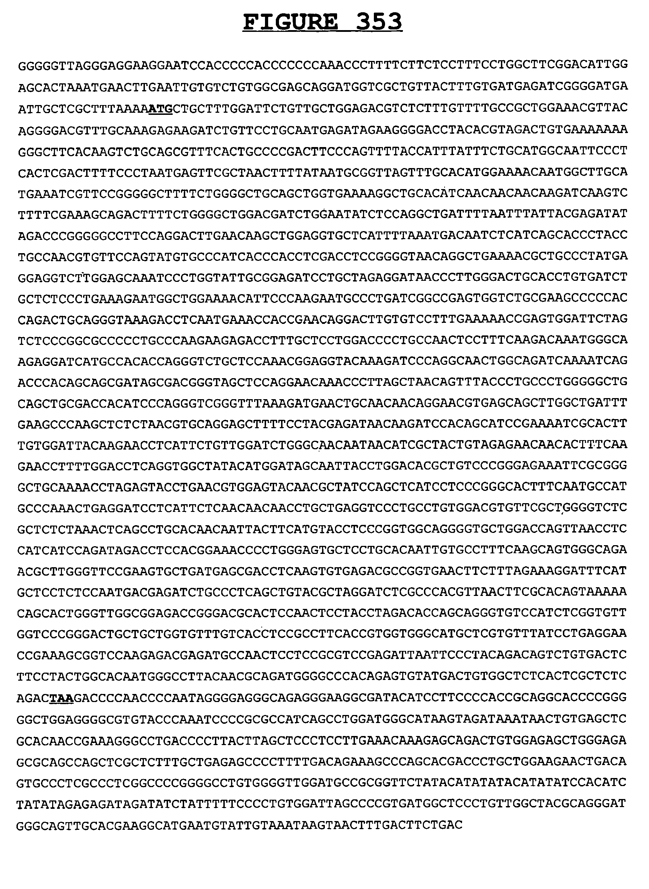

Figure 353 shows a nucleotide sequence (SEQ ID NO:353) of a native sequence PR0266 cDNA, wherein SEQ ID NO:353 is a clone designated herein as "DNA37150-1178".

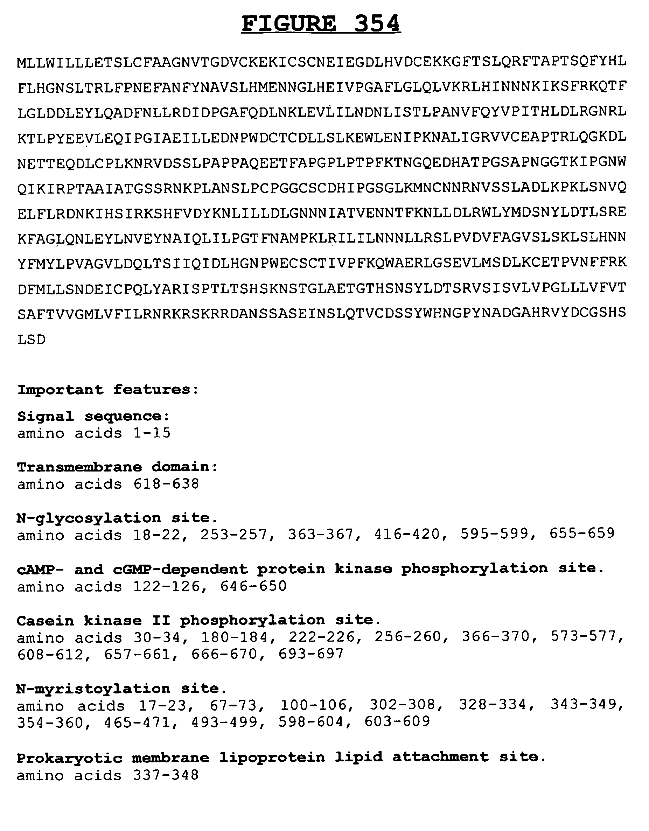

Figure 354 shows the amino acid sequence (SEQ ID NO:354) derived from the coding sequence of SEQ ID NO: 353 shown in Figure 353.

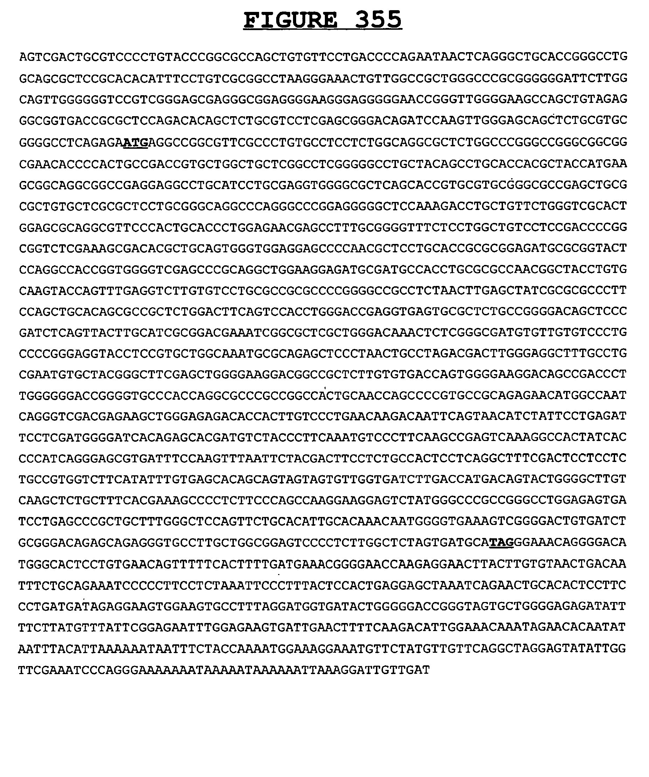

Figure 355 shows a nucleotide sequence (SEQ ID NO:355) of a native sequence PR0269 cDNA, wherein SEQ ID NO:355 is a clone designated herein as "DNA38260-1180".

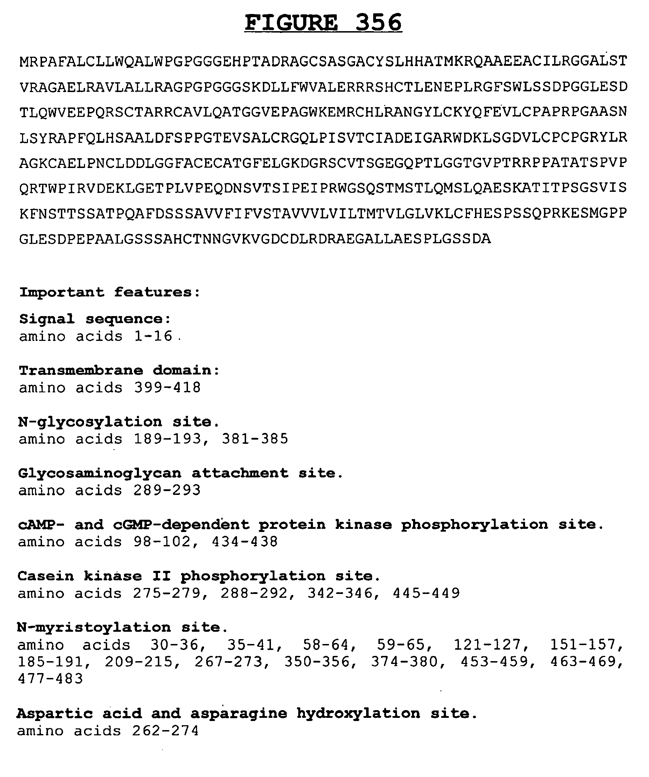

Figure 356 shows the amino acid sequence (SEQ ID NO:356) derived from the coding sequence of SEQ ID NO: 355 shown in Figure 355.

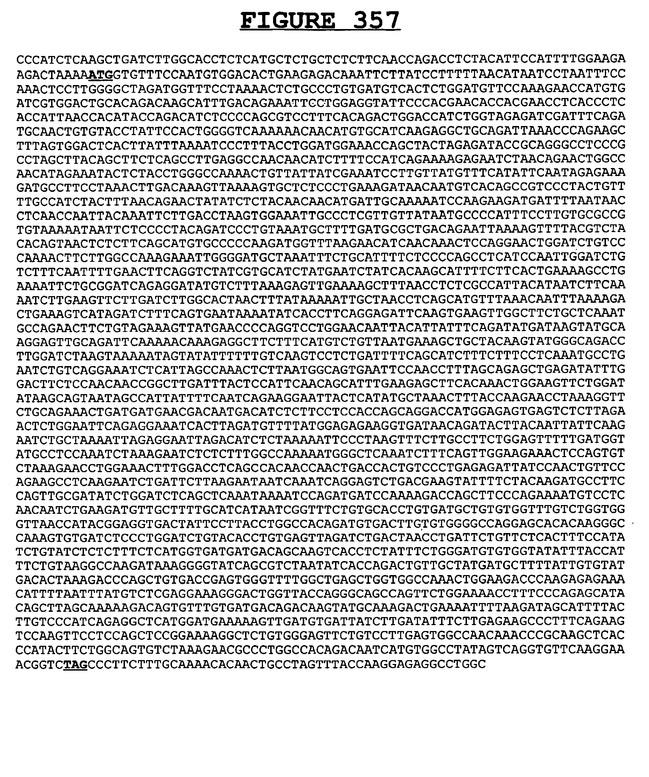

Figure 357 shows a nucleotide sequence (SEQ ID NO:357) of a native sequence PR0285 cDNA, wherein SEQ ID NO:357 is a clone designated herein as "DNA40021-1154".

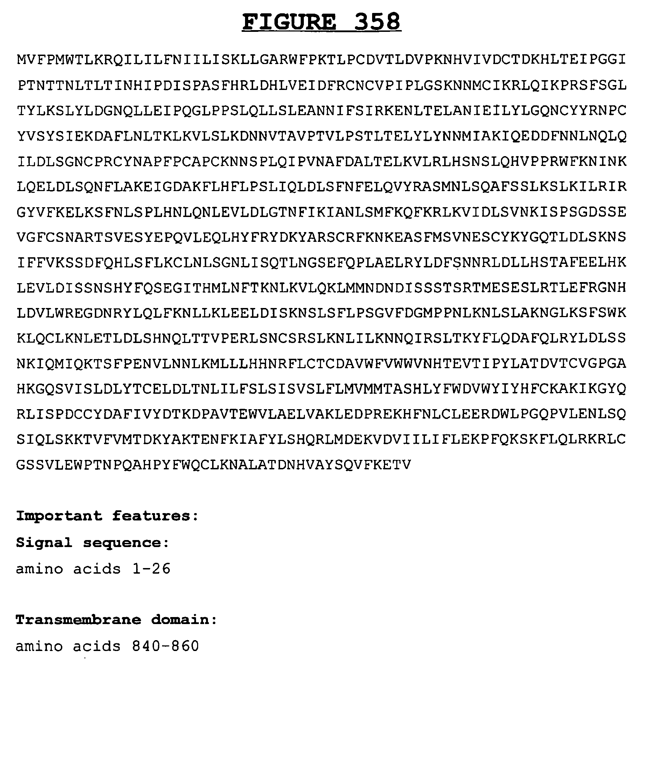

Figure 358 shows the amino acid sequence (SEQ ID NO:358) derived from the coding sequence of SEQ ID NO: 357 shown in Figure 357.

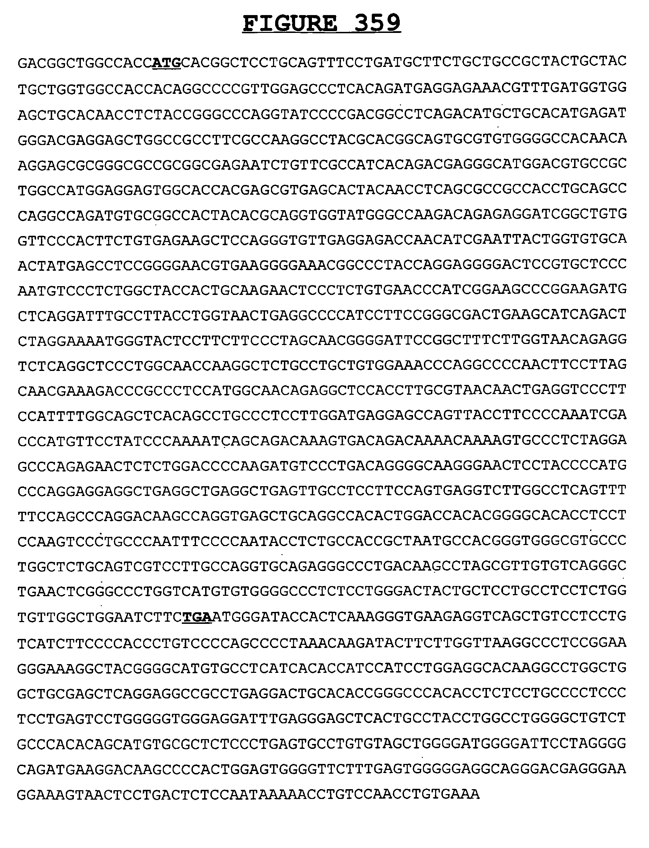

Figure 359 shows a nucleotide sequence (SEQ ID NO:359) of a native sequence PR0328 cDNA, wherein SEQ ID NO:359 is a clone designated herein as "DNA40587-1231".

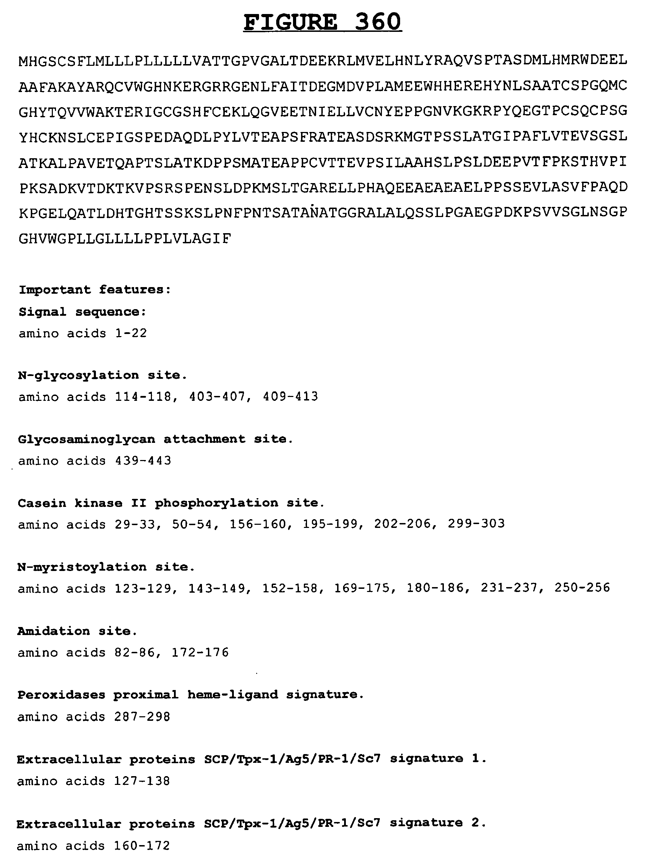

Figure 360 shows the amino acid sequence (SEQ ID NO:360) derived from the coding sequence of SEQ ID NO: 359 shown in Figure 359.

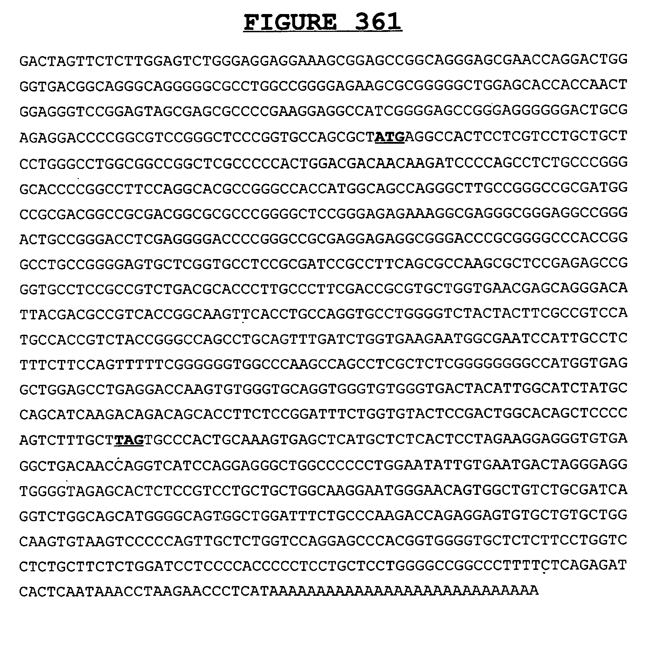

Figure 361 shows a nucleotide sequence (SEQ ID NO:361) of a native sequence PR0344 cDNA, wherein SEQ ID NO:361 is a clone designated herein as "DNA40592-1242".

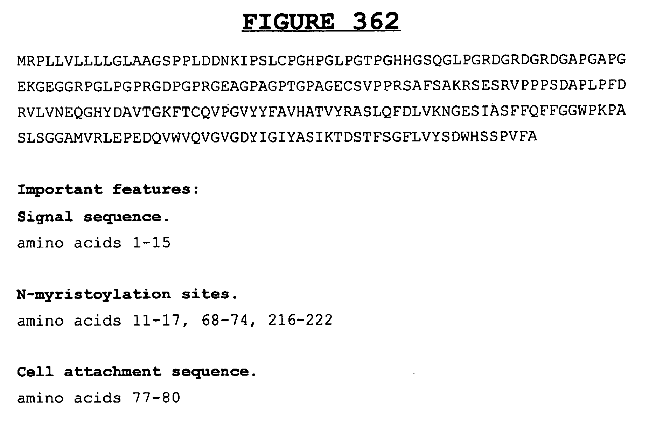

Figure 362 shows the amino acid sequence (SEQ ID NO:362) derived from the coding sequence of SEQ ID NO: 361 shown in Figure 361.

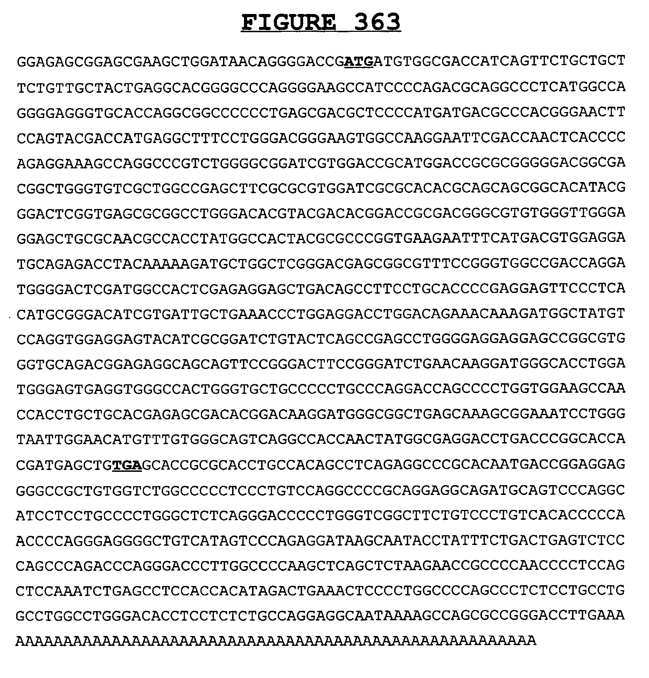

Figure 363 shows a nucleotide sequence (SEQ ID NO:363) of a native sequence PR0272 cDNA, wherein SEQ ID NO:363 is a clone designated herein as "DNA40620-1183".

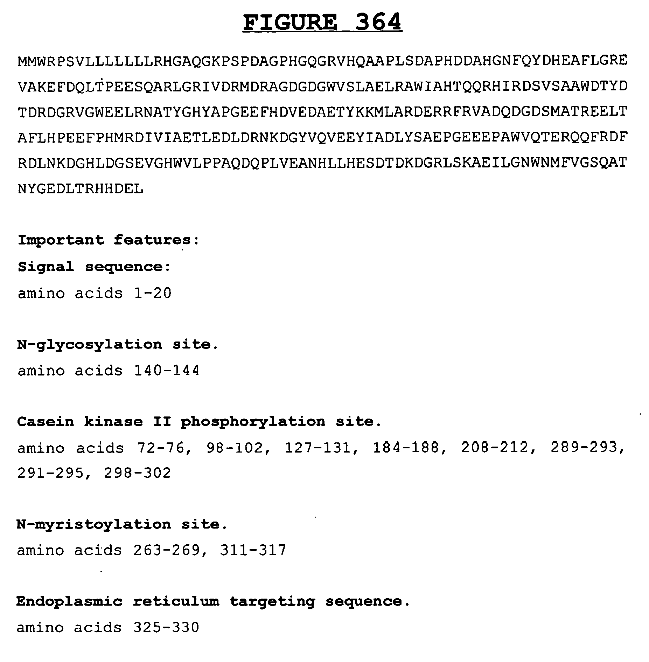

Figure 364 shows the amino acid sequence (SEQ ID NO:364) derived from the coding sequence of SEQ ID NO: 363 shown in Figure 363.

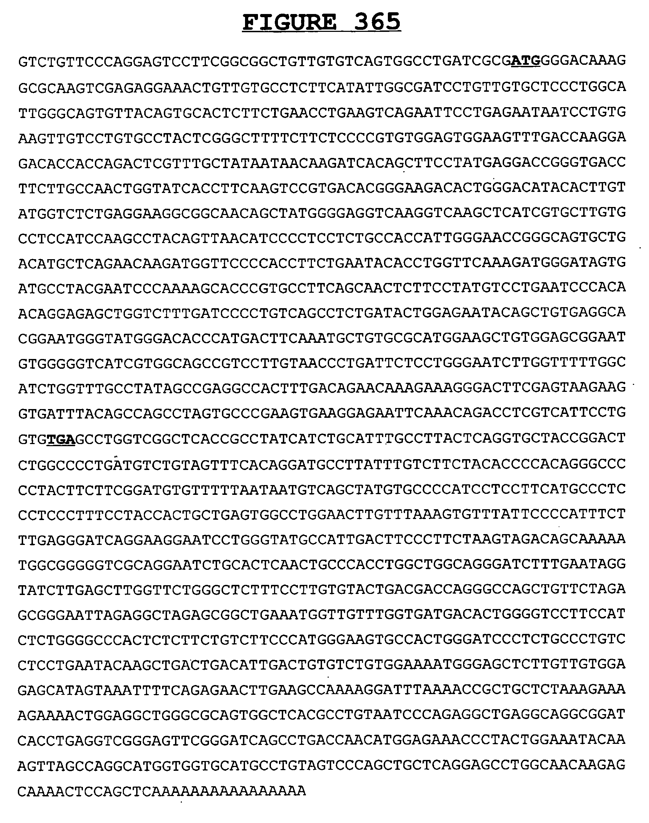

Figure 365 shows a nucleotide sequence (SEQ ID NO:365) of a native sequence PR0301 cDNA, wherein SEQ ID NO:365 is a clone designated herein as "DNA40628-1216".

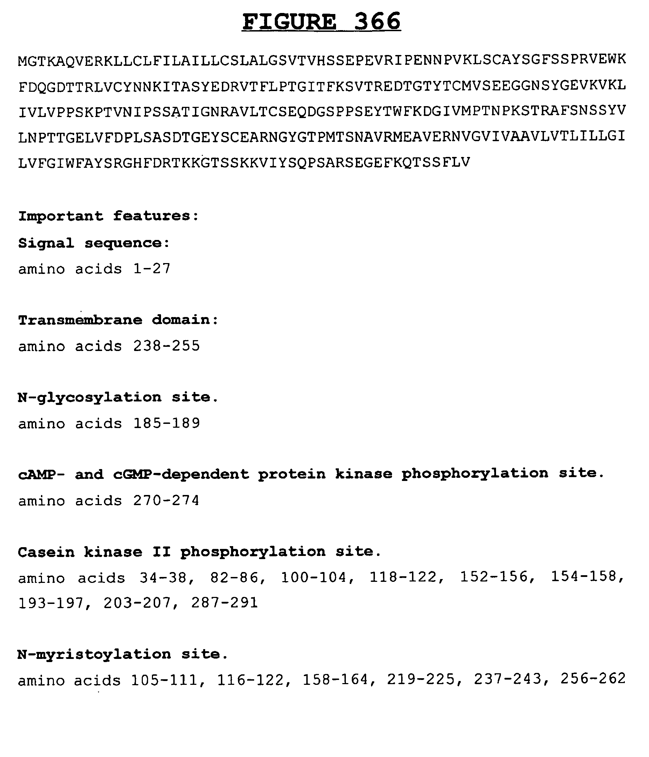

Figure 366 shows the amino acid sequence (SEQ ID NO:366) derived from the coding sequence of SEQ ID NO: 365 shown in Figure 365.

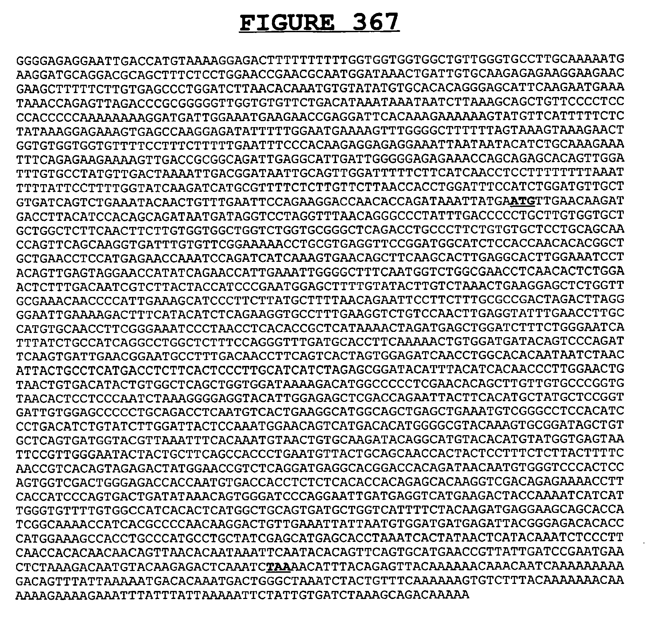

Figure 367 shows a nucleotide sequence (SEQ ID NO:367) of a native sequence PRO331 cDNA, wherein SEQ ID NO:367 is a clone designated herein as "DNA40981-1234".

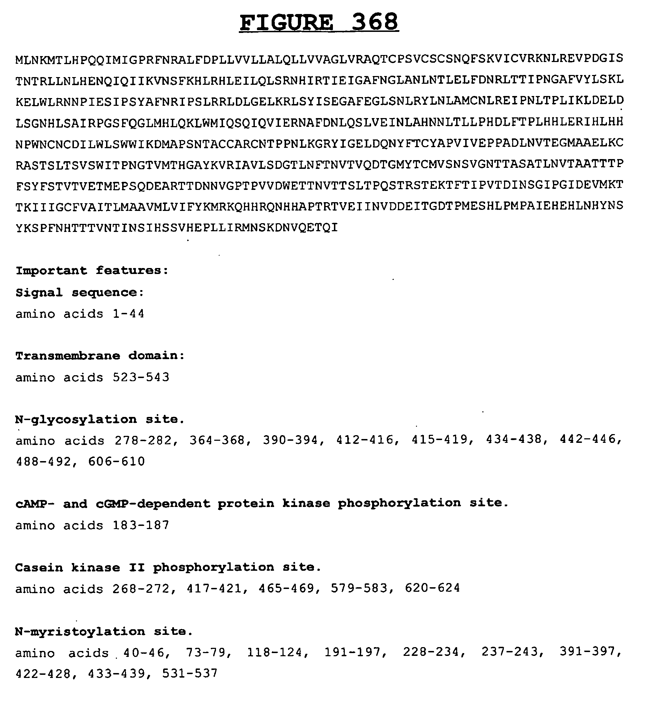

Figure 368 shows the amino acid sequence (SEQ ID NO:368) derived from the coding sequence of SEQ ID NO: 367 shown in Figure 367.

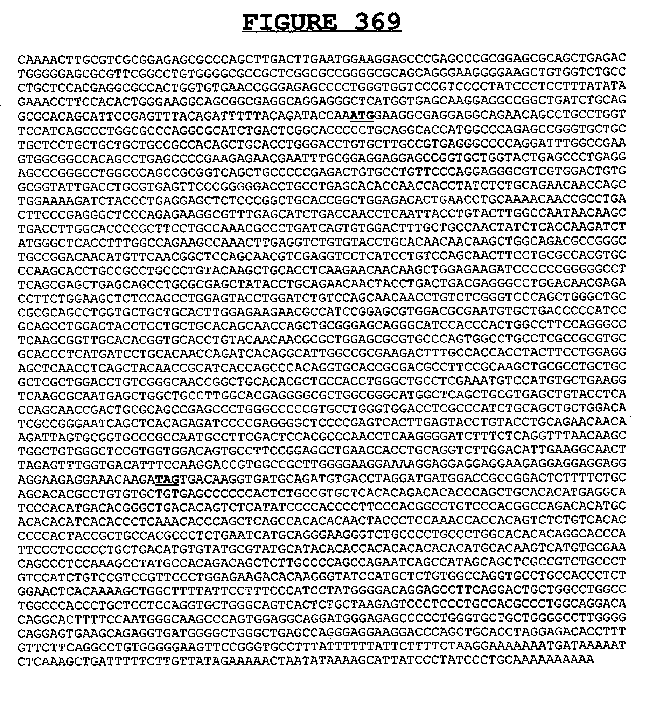

Figure 369 shows a nucleotide sequence (SEQ ID NO:369) of a native sequence PR0332 cDNA, wherein SEQ ID NO:369 is a clone designated herein as "DNA40982-1235".

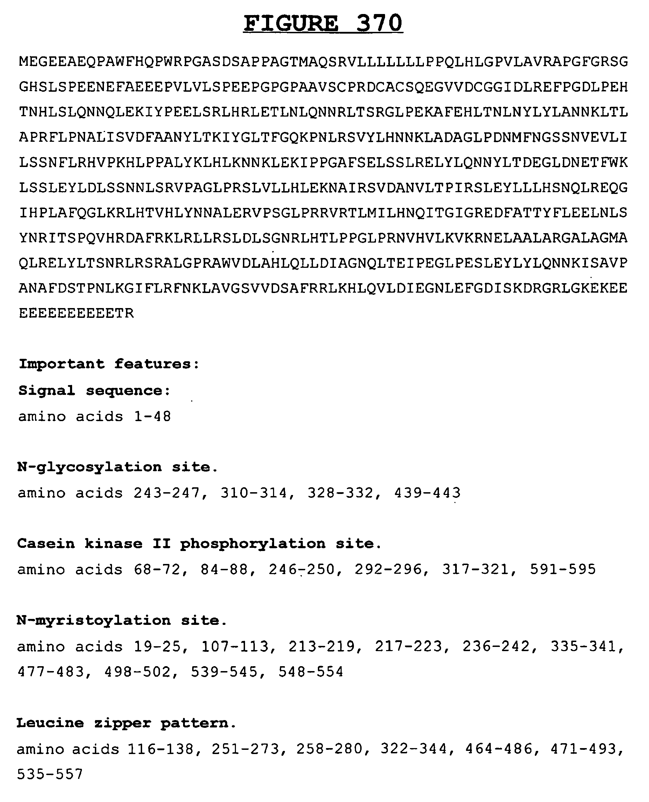

Figure 370 shows the amino acid sequence (SEQ ID NO:370) derived from the coding sequence of SEQ ID NO: 369 shown in Figure 369.

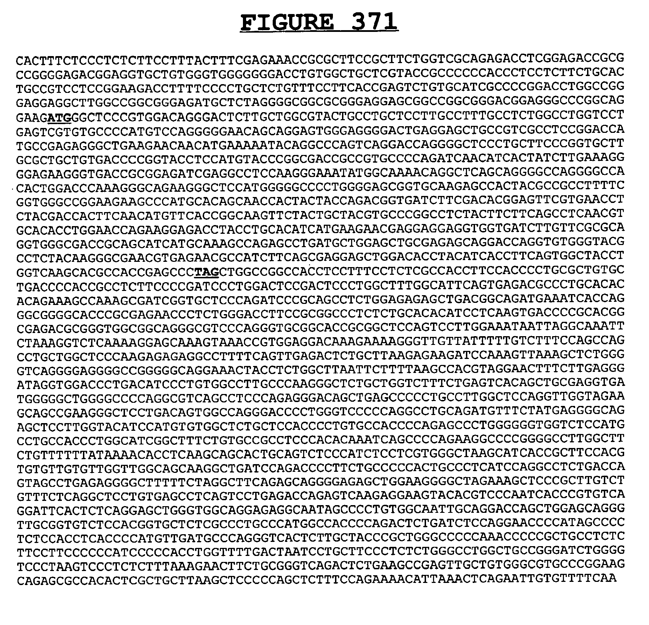

Figure 371 shows a nucleotide sequence (SEQ ID NO:371) of a native sequence PR0353 cDNA, wherein SEQ ID NO:371 is a clone designated herein as "DNA41234-1242".

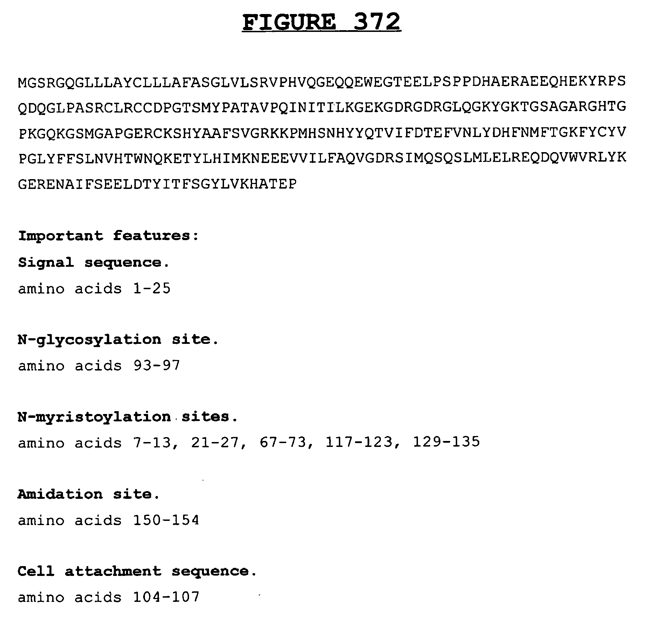

Figure 372 shows the amino acid sequence (SEQ ID NO: 372) derived from the coding sequence of SEQ ID NO: 371 shown in Figure 371.

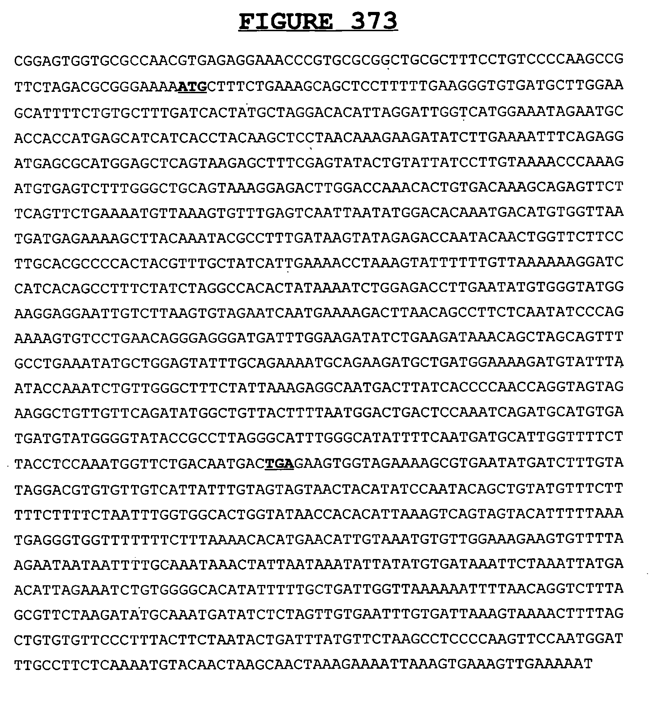

Figure 373 shows a nucleotide sequence (SEQ ID NO:373) of a native sequence PR0310 cDNA, wherein SEQ ID NO:373 is a clone designated herein as "DNA43046-1225".

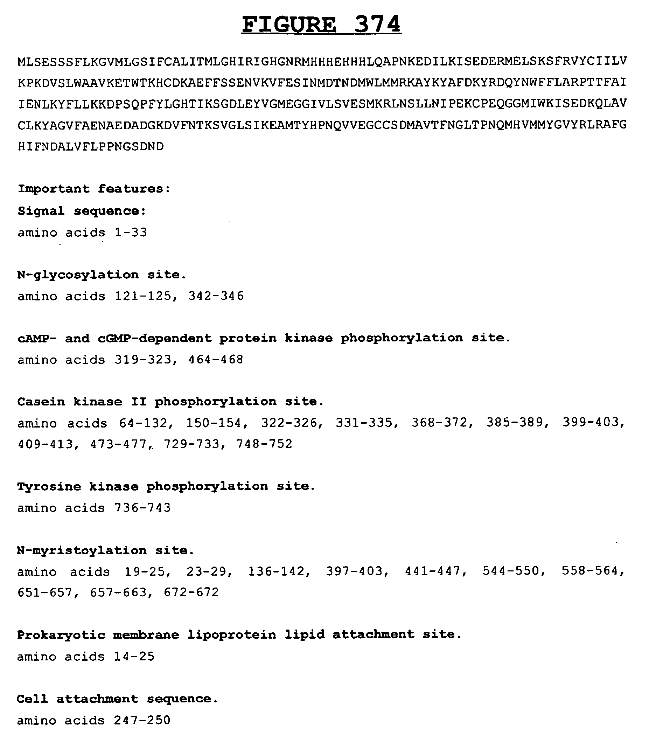

Figure 374 shows the amino acid sequence (SEQ ID NO: 374) derived from the coding sequence of SEQ ID NO: 373 shown in Figure 373.

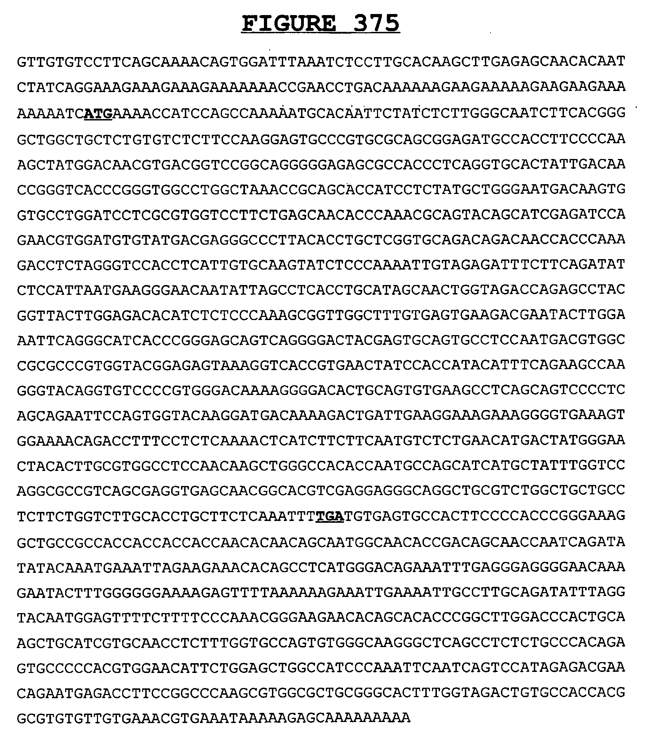

Figure 375 shows a nucleotide sequence (SEQ ID NO:375) of a native sequence PR0337 cDNA, wherein SEQ ID NO:375 is a clone designated herein as "DNA43316-1237".

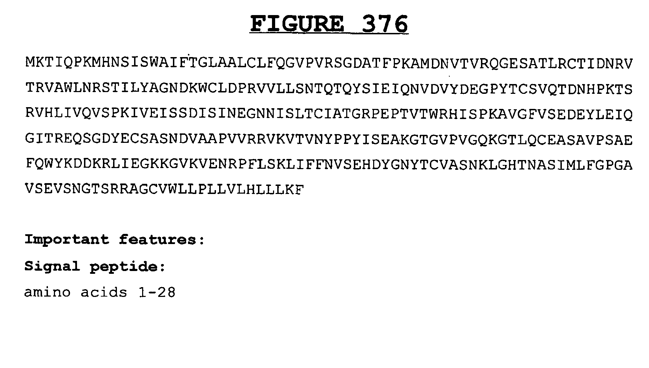

Figure 376 shows the amino acid sequence (SEQ ID NO:376) derived from the coding sequence of SEQ ID NO: 375 shown in Figure 375.

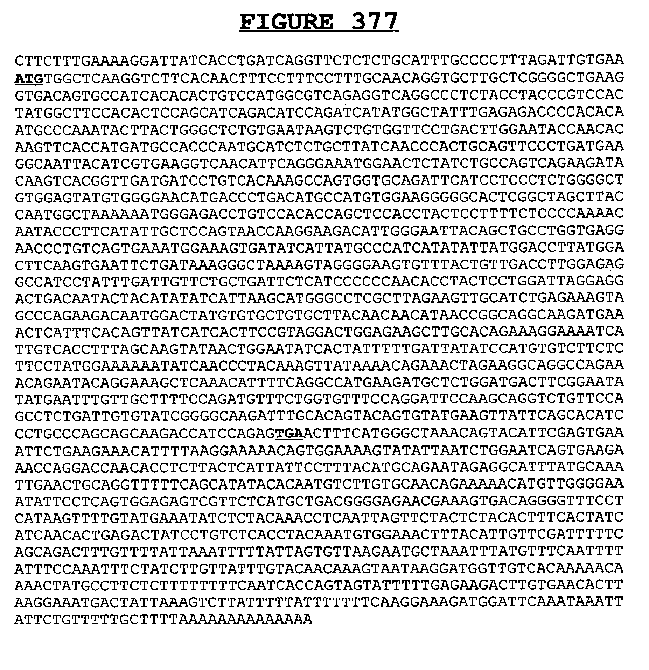

Figure 377 shows a nucleotide sequence (SEQ ID NO:377) of a native sequence PR0346 cDNA, wherein SEQ ID NO:377 is a clone designated herein as "DNA44167-1243".

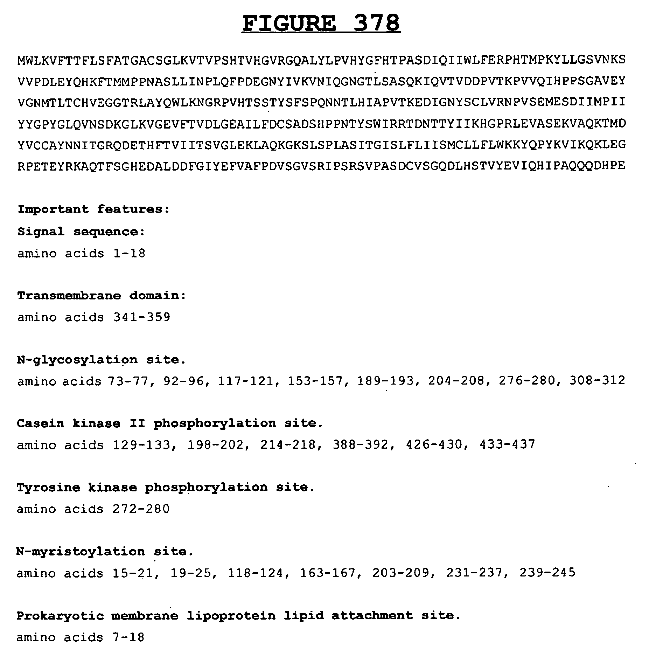

Figure 378 shows the amino acid sequence (SEQ ID NO:378) derived from the coding sequence of SEQ ID NO: 377 shown in Figure 377.

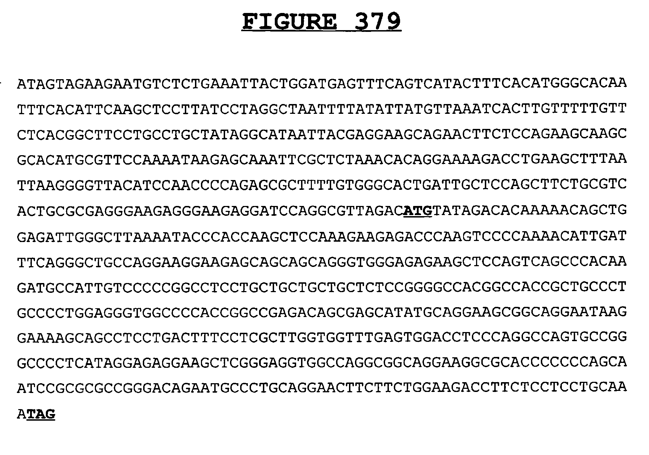

Figure 379 shows a nucleotide sequence (SEQ ID NO:379) of a native sequence PR0350 cDNA, wherein SEQ ID NO:379 is a clone designated herein as "DNA44175-1314".

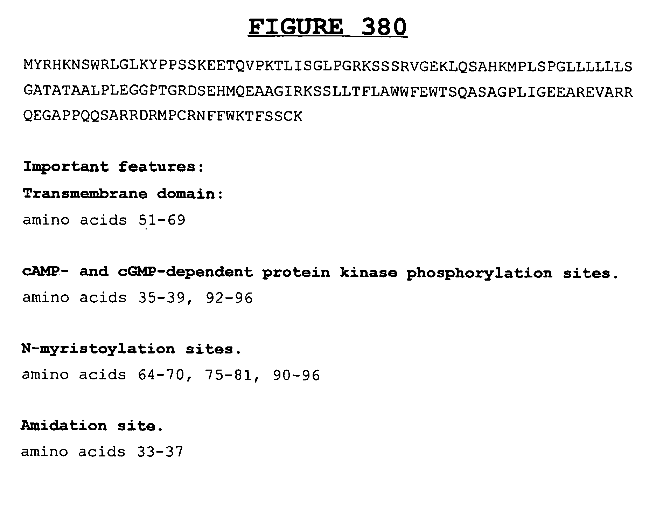

Figure 380 shows the amino acid sequence (SEQ ID NO:380) derived from the coding sequence of SEQ ID NO: 379 shown in Figure 379.

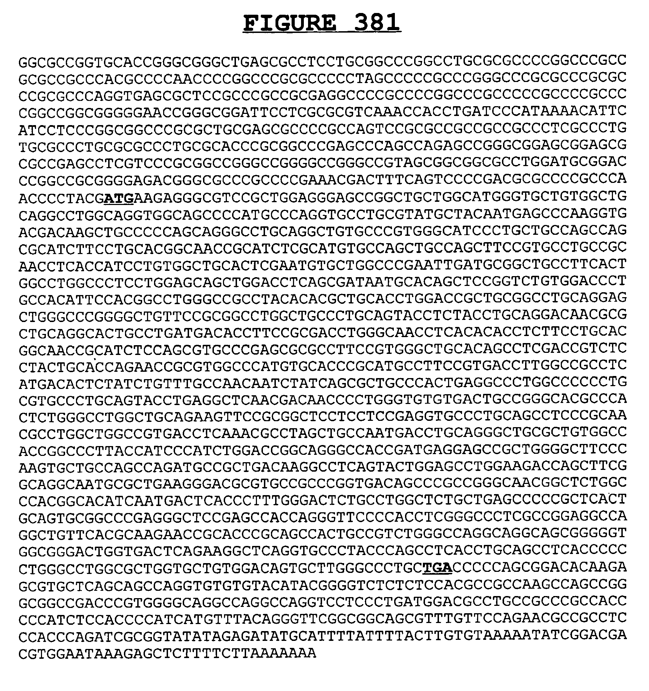

Figure 381 shows a nucleotide sequence (SEQ ID NO:381) of a native sequence PR0526 cDNA, wherein SEQ ID NO:381 is a clone designated herein as "DNA44184-1319".

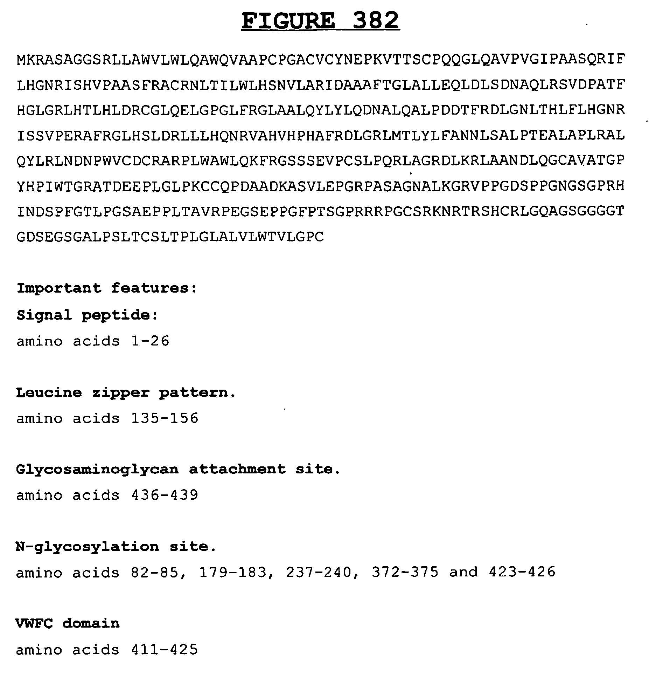

Figure 382 shows the amino acid sequence (SEQ ID NO:382) derived from the coding sequence of SEQ ID NO: 381 shown in Figure 381.

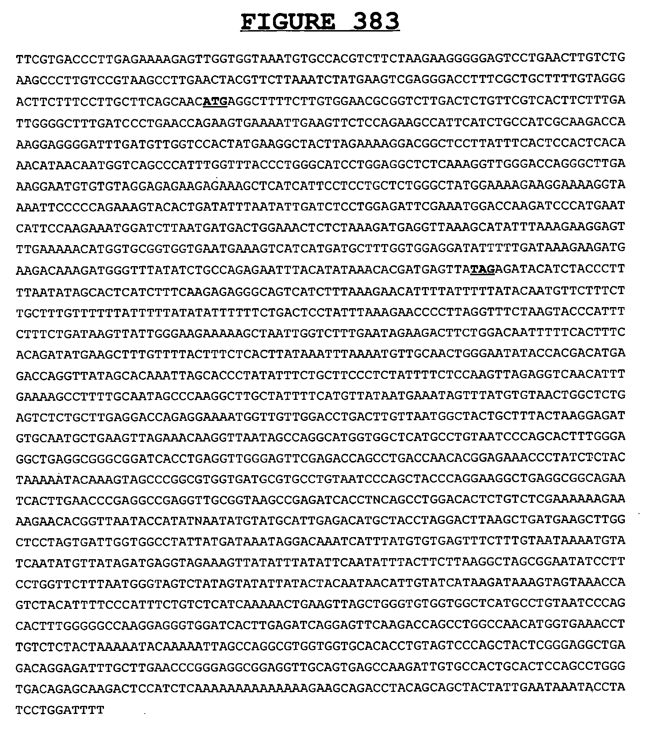

Figure 383 shows a nucleotide sequence (SEQ ID NO:383) of a native sequence PR0381 cDNA, wherein SEQ ID NO:383 is a clone designated herein as "DNA44194-1317".

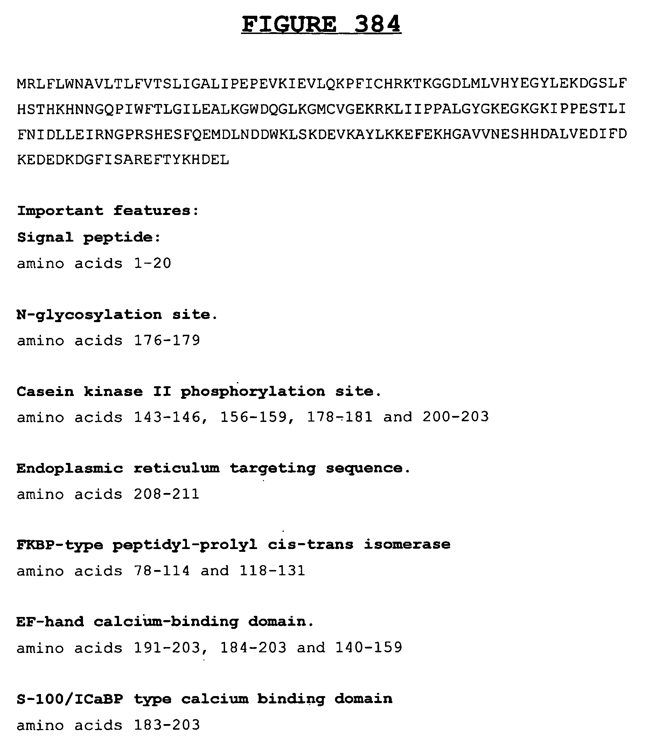

Figure 384 shows the amino acid sequence (SEQ ID NO:384) derived from the coding sequence of SEQ ID NO: 383 shown in Figure 383.

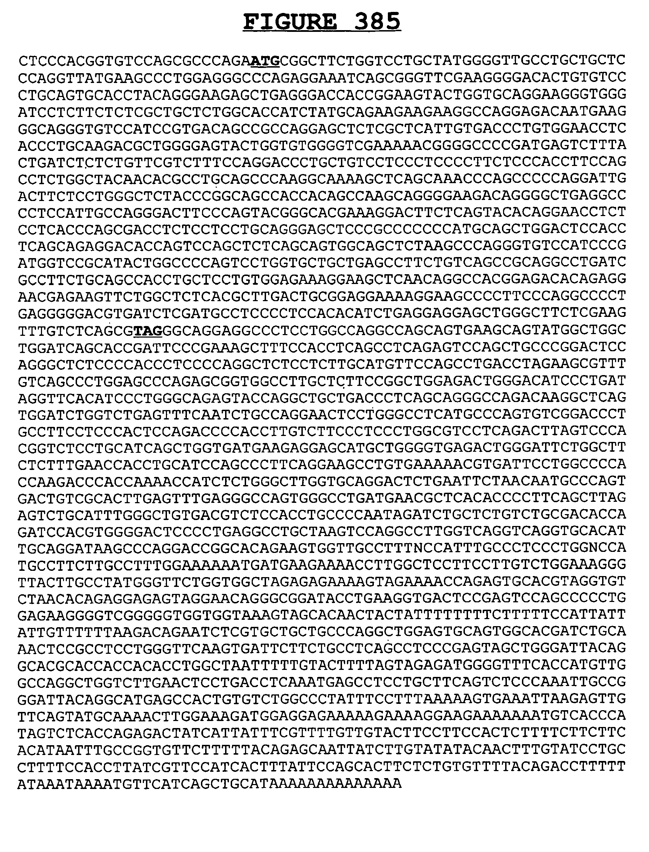

Figure 385 shows a nucleotide sequence (SEQ ID NO:385) of a native sequence PRO846 cDNA, wherein SEQ ID NO:385 is a clone designated herein as "DNA44196-1353".

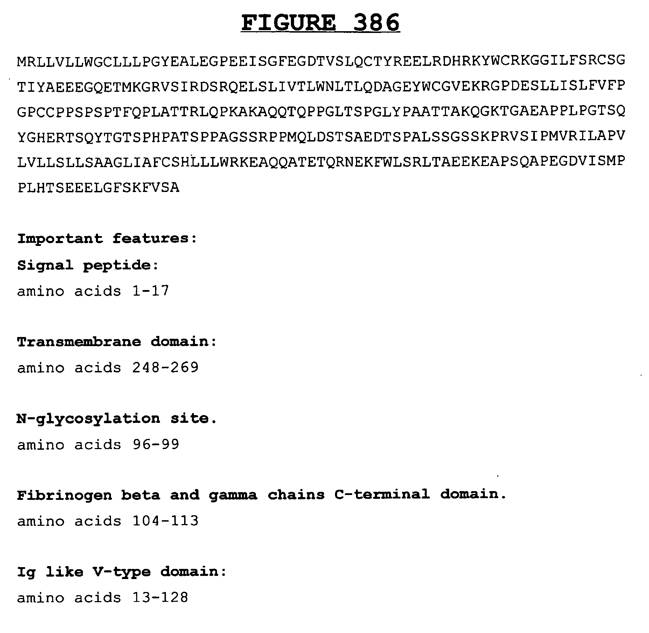

Figure 386 shows the amino acid sequence (SEQ ID NO:386) derived from the coding sequence of SEQ ID NO: 385 shown in Figure 385.

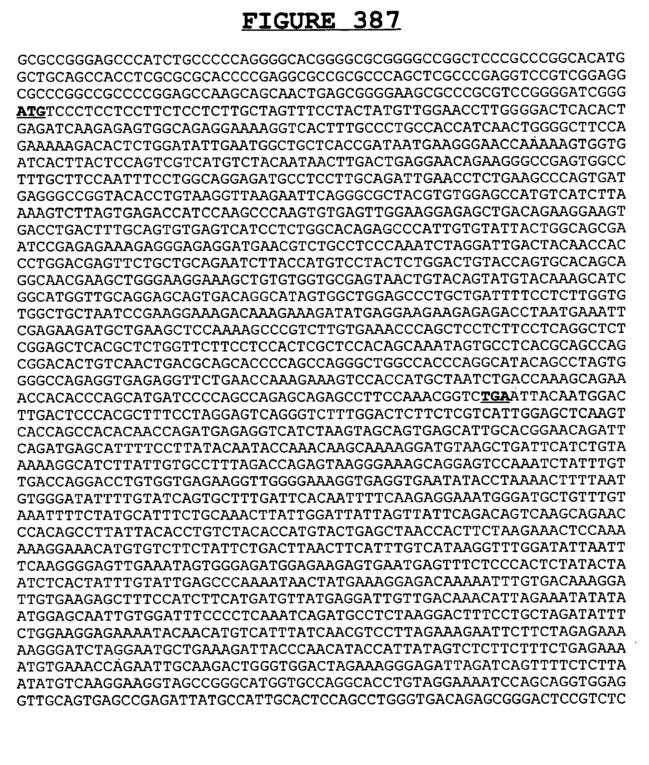

Figure 387 shows a nucleotide sequence (SEQ ID NO:387) of a native sequence PR0363 cDNA, wherein SEQ ID NO:387 is a clone designated herein as "DNA45419-1252".

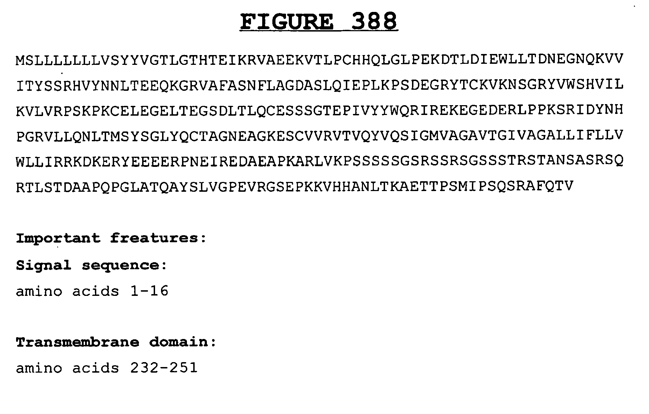

Figure 388 shows the amino acid sequence (SEQ ID NO:388) derived from the coding sequence of SEQ ID NO: 387 shown in Figure 387.

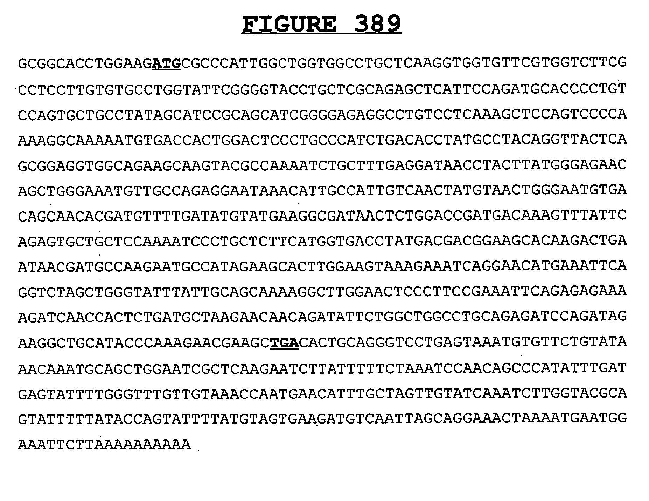

Figure 389 shows a nucleotide sequence (SEQ ID NO:389) of a native sequence PR0365 cDNA, wherein SEQ ID NO:389 is a clone designated herein as "DNA46777-1253".

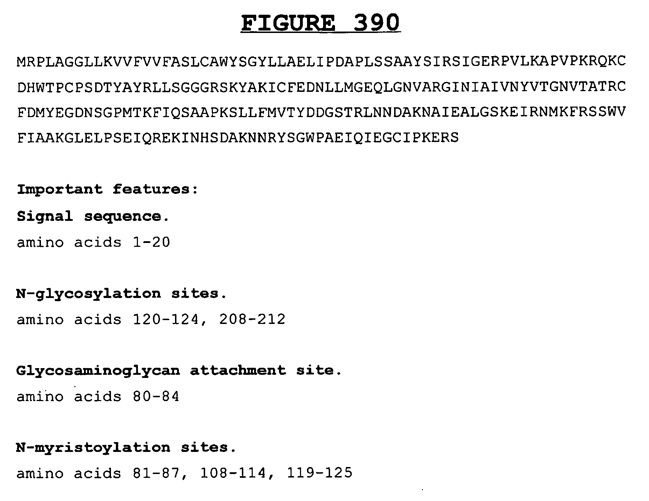

Figure 390 shows the amino acid sequence (SEQ ID NO:390) derived from the coding sequence of SEQ ID NO: 389 shown in Figure 389.

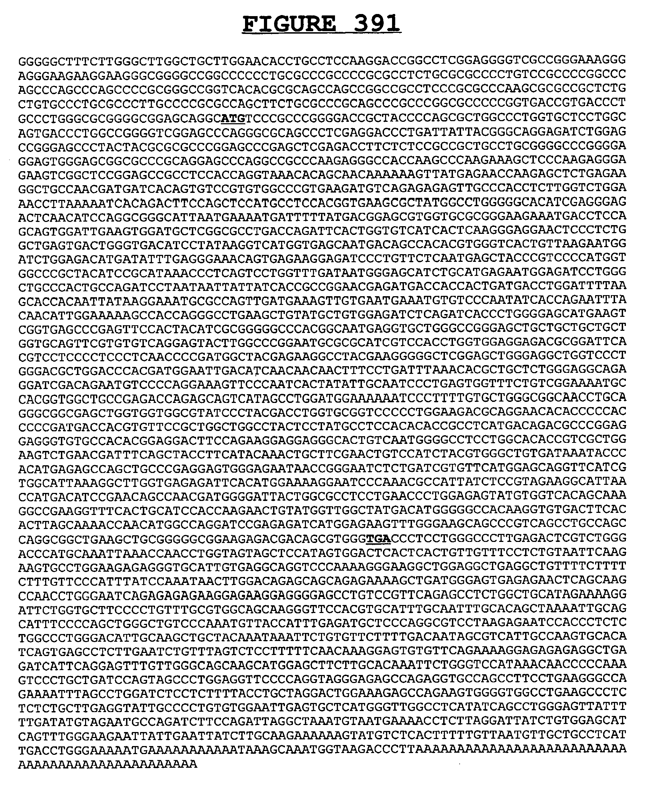

Figure 391 shows a nucleotide sequence (SEQ ID NO:391) of a native sequence PRO1310 cDNA, wherein SEQ ID NO:391 is a clone designated herein as "DNA47394-1572".

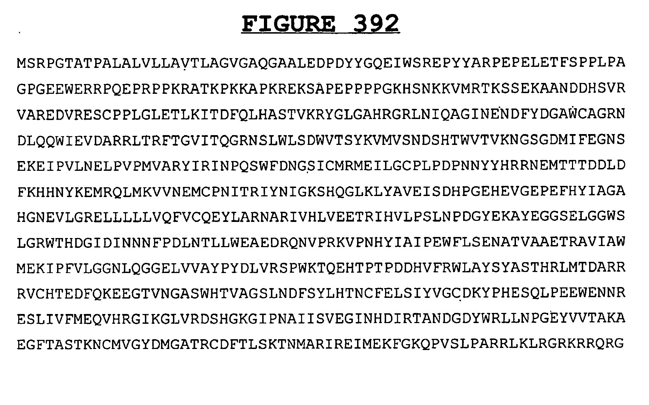

Figure 392 shows the amino acid sequence (SEQ ID NO:392) derived from the coding sequence of SEQ ID NO: 391 shown in Figure 391.

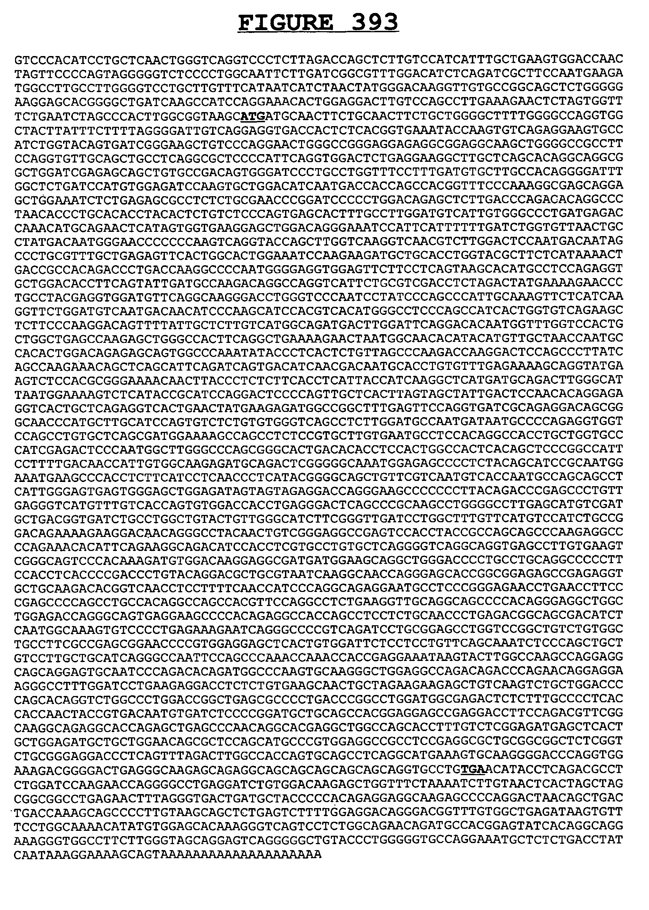

Figure 393 shows a nucleotide sequence (SEQ ID NO:393) of a native sequence PR0731 cDNA, wherein SEQ ID NO:393 is a clone designated herein as "DNA48331-1329".

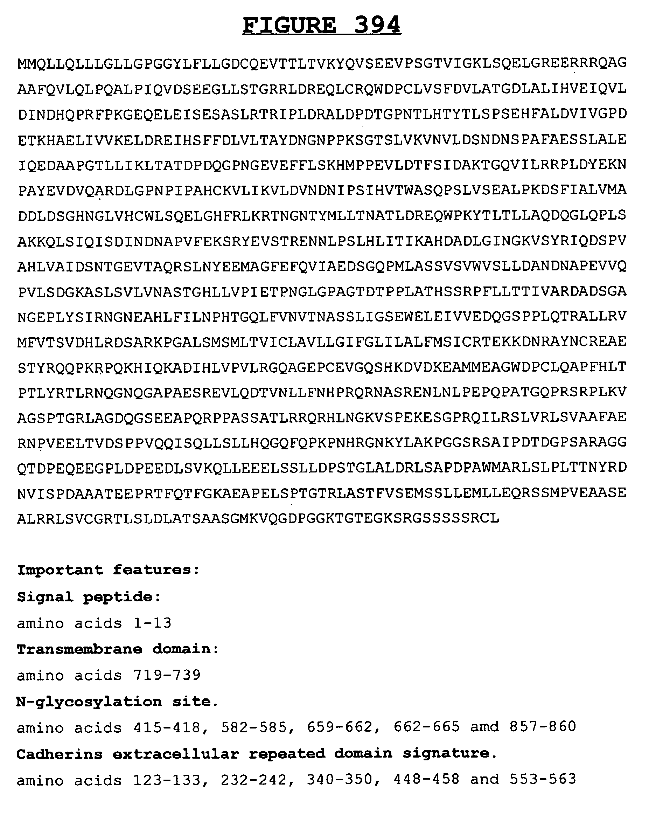

Figure 394 shows the amino acid sequence (SEQ ID NO:394) derived from the coding sequence of SEQ ID NO: 393 shown in Figure 393.

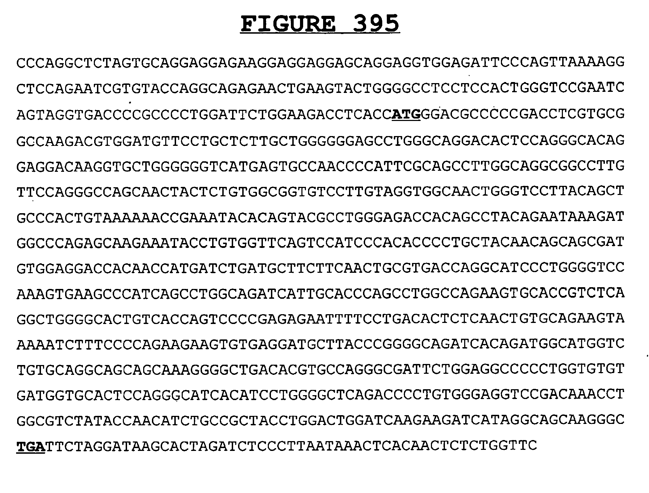

Figure 395 shows a nucleotide sequence (SEQ ID NO:395) of a native sequence PR0322 cDNA, wherein SEQ ID NO:395 is a clone designated herein as "DNA48336-1309".

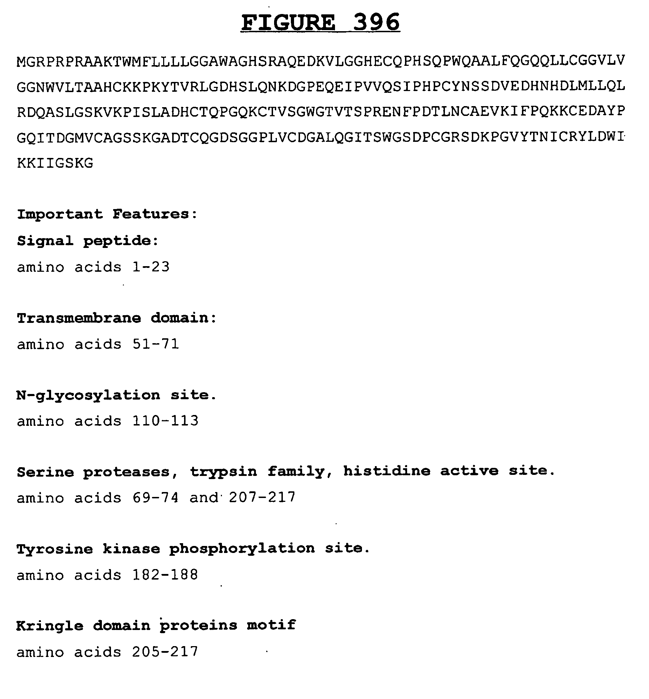

Figure 396 shows the amino acid sequence (SEQ ID NO:396) derived from the coding sequence of SEQ ID NO: 395 shown in Figure 395.

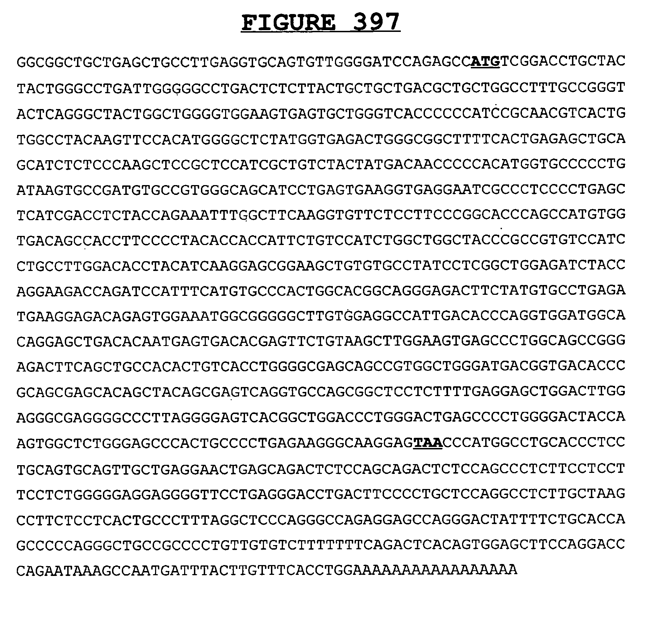

Figure 397 shows a nucleotide sequence (SEQ ID NO:397) of a native sequence PR0536 cDNA, wherein SEQ ID NO:397 is a clone designated herein as "DNA49142-1430".

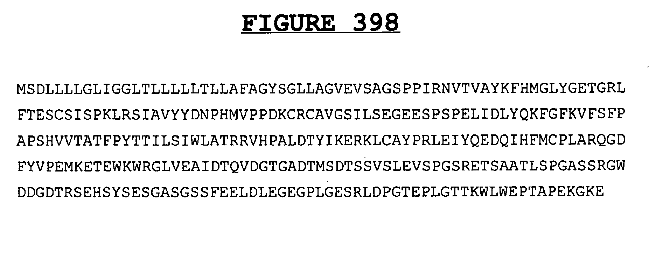

Figure 398 shows the amino acid sequence (SEQ ID NO:398) derived from the coding sequence of SEQ ID NO: 397 shown in Figure 397.

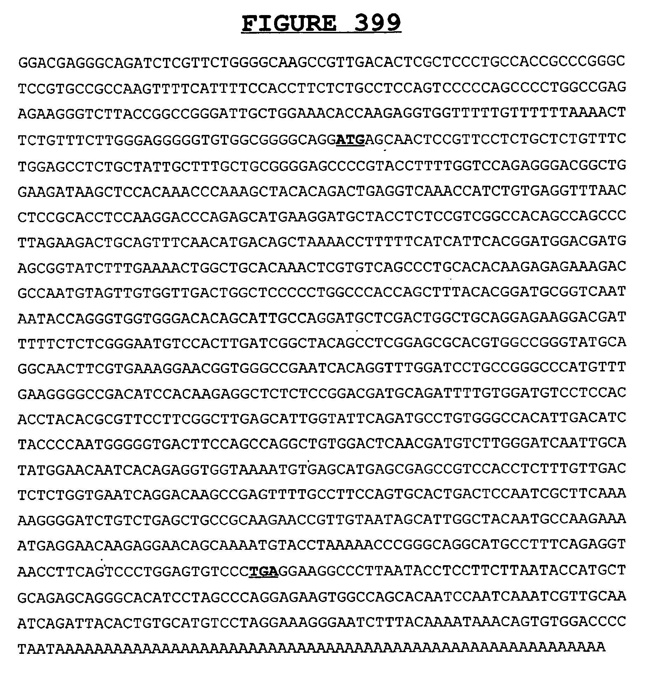

Figure 399 shows a nucleotide sequence (SEQ ID NO:399) of a native sequence PR0719 cDNA, wherein SEQ ID NO:399 is a clone designated herein as "DNA49646-1327".

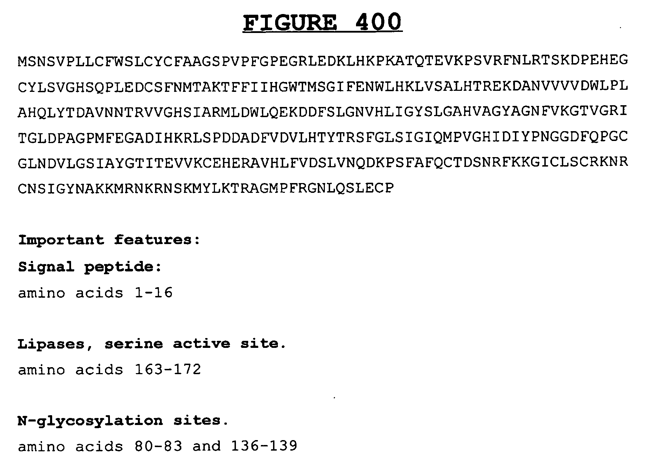

Figure 400 shows the amino acid sequence (SEQ ID NO:400) derived from the coding sequence of SEQ ID NO: 399 shown in Figure 399.

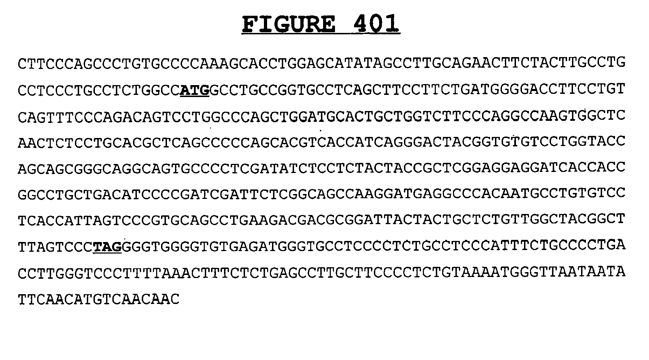

Figure 401 shows a nucleotide sequence (SEQ ID NO:401) of a native sequence PR0619 cDNA, wherein SEQ ID NO:401 is a clone designated herein as "DNA49821-1562".

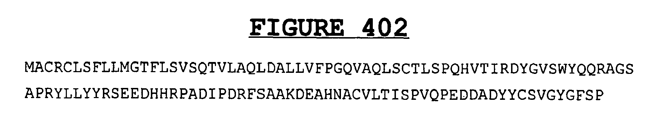

Figure 402 shows the amino acid sequence (SEQ ID NO:402) derived from the coding sequence of SEQ ID NO: 401 shown in Figure 401.

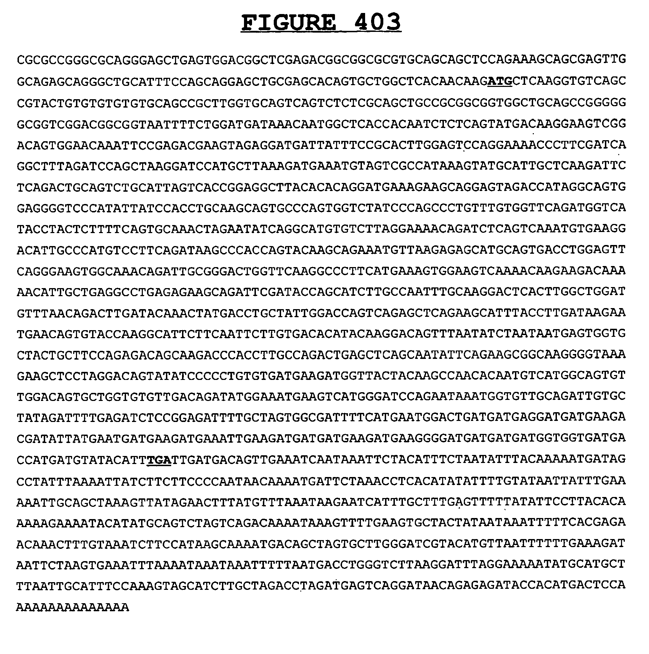

Figure 403 shows a nucleotide sequence (SEQ ID NO:403) of a native sequence PRO771 cDNA, wherein SEQ ID NO:403 is a clone designated herein as "DNA49829-1346".

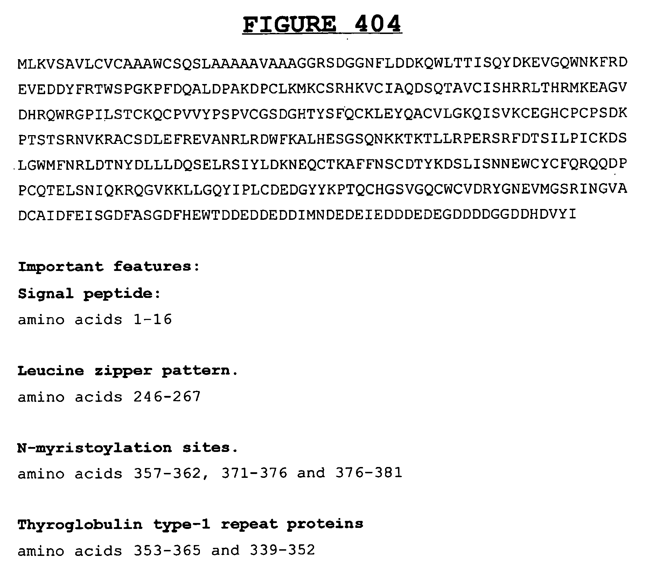

Figure 404 shows the amino acid sequence (SEQ ID NO:404) derived from the coding sequence of SEQ ID NO: 403 shown in Figure 403.

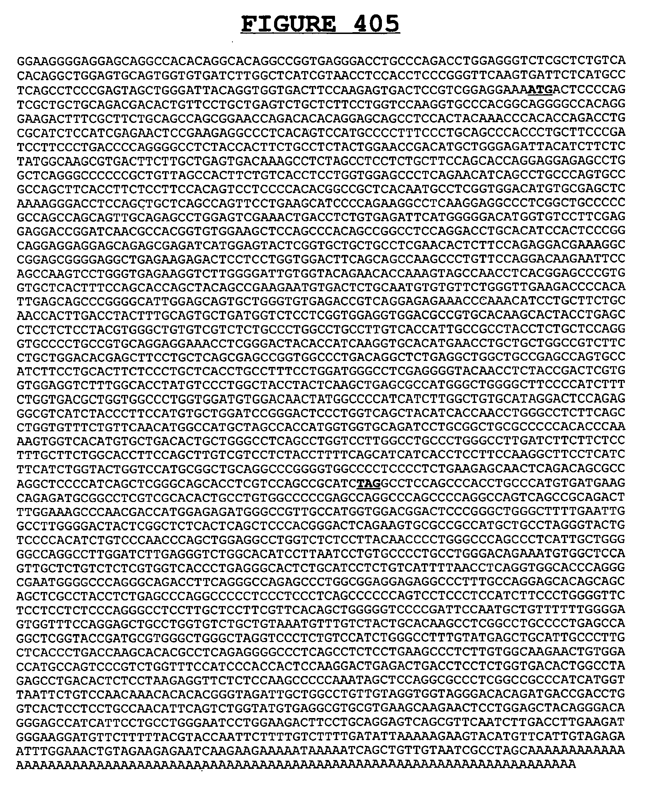

Figure 405 shows a nucleotide sequence (SEQ ID NO:405) of a native sequence PRO1083 cDNA, wherein SEQ ID NO:405 is a clone designated herein as "DNA50921-1458".

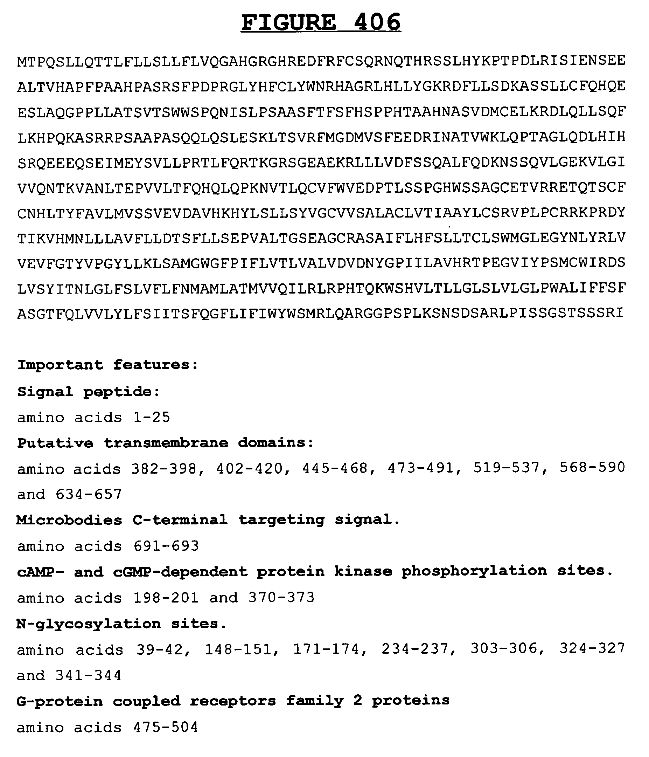

Figure 406 shows the amino acid sequence (SEQ ID NO:406) derived from the coding sequence of SEQ ID NO: 405 shown in Figure 405.

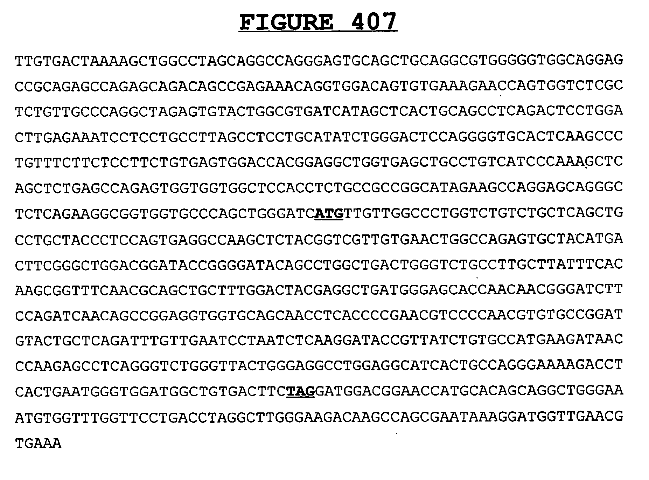

Figure 407 shows a nucleotide sequence (SEQ ID NO:407) of a native sequence PRO862 cDNA, wherein SEQ ID NO:407 is a clone designated herein as "DNA52187-1354".

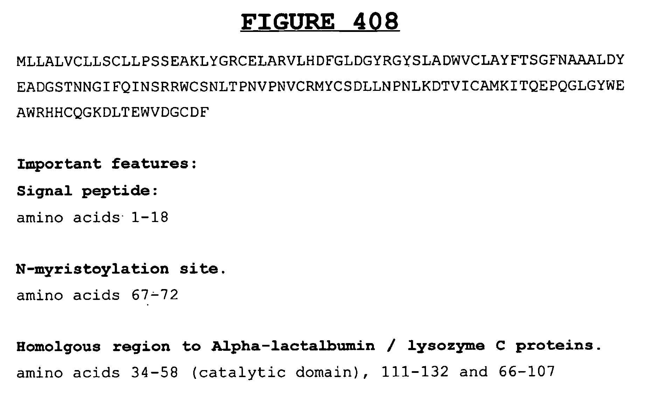

Figure 408 shows the amino acid sequence (SEQ ID NO:408) derived from the coding sequence of SEQ ID NO: 407 shown in Figure 407.

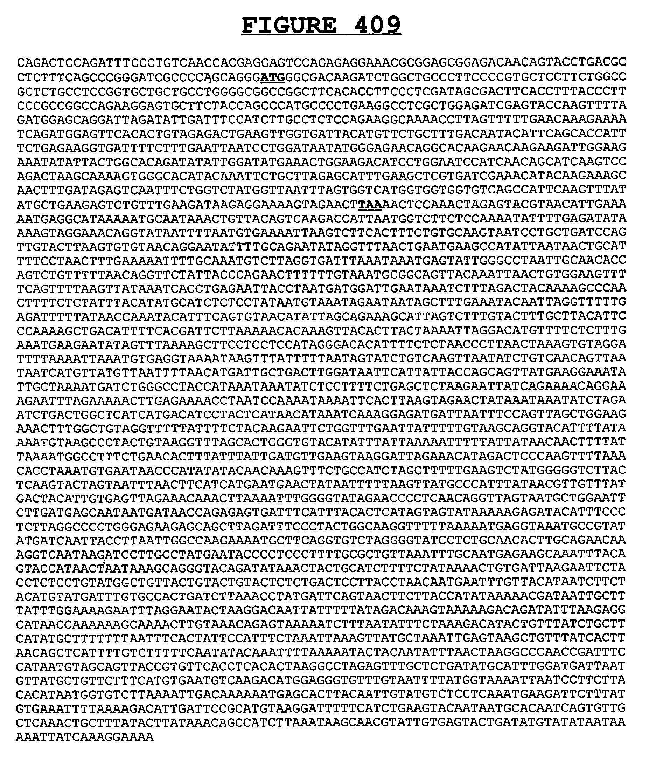

Figure 409 shows a nucleotide sequence (SEQ ID NO:409) of a native sequence PR0733 cDNA, wherein SEQ ID NO:409 is a clone designated herein as "DNA52196-1348".

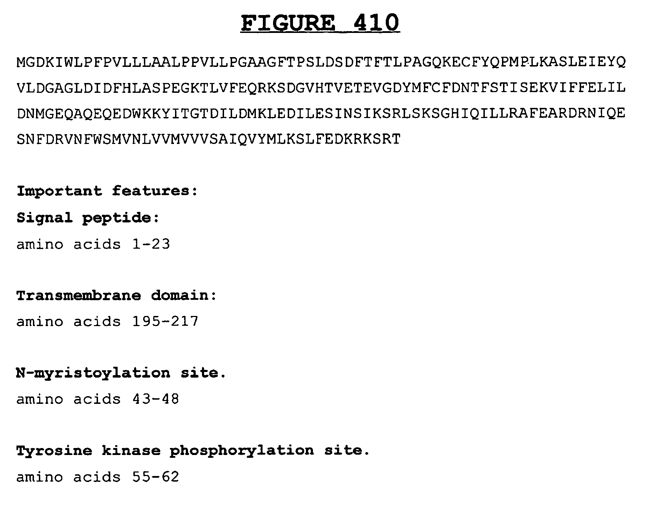

Figure 410 shows the amino acid sequence (SEQ ID NO:410) derived from the coding sequence of SEQ ID NO: 409 shown in Figure 409.

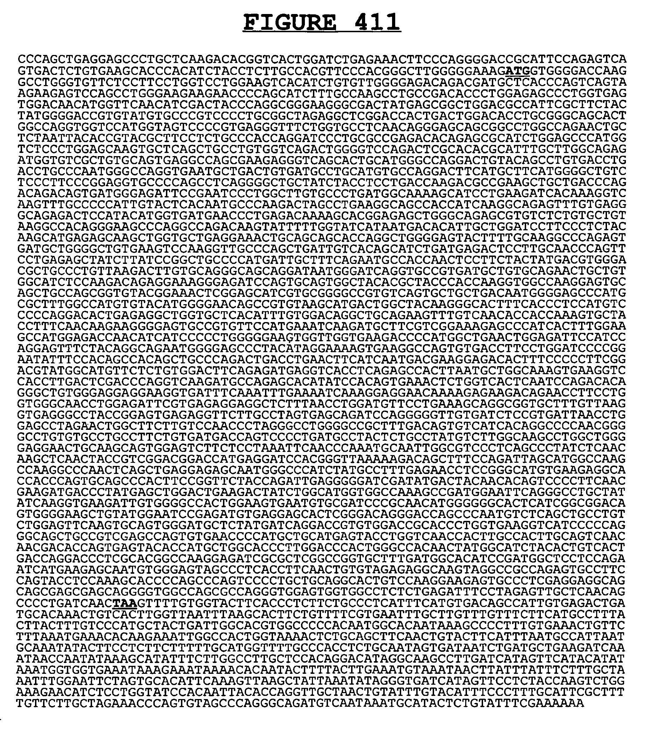

Figure 411 shows a nucleotide sequence (SEQ ID NO:411) of a native sequence PRO1188 cDNA, wherein SEQ ID NO:411 is a clone designated herein as "DNA52598-1518".

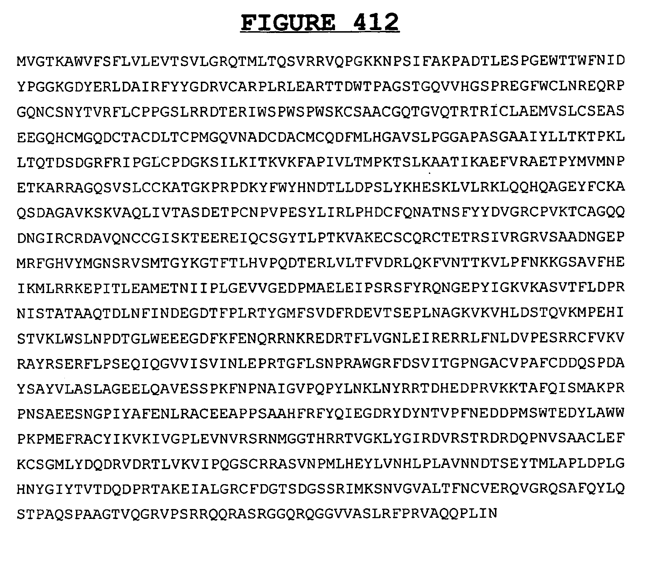

Figure 412 shows the amino acid sequence (SEQ ID NO:412) derived from the coding sequence of SEQ ID NO: 411 shown in Figure 411.

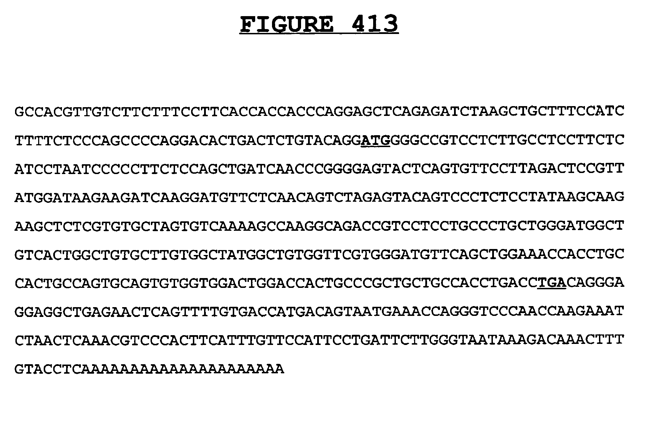

Figure 413 shows a nucleotide sequence (SEQ ID NO:413) of a native sequence PR0770 cDNA, wherein SEQ ID NO:413 is a clone designated herein as "DNA54228-1366".

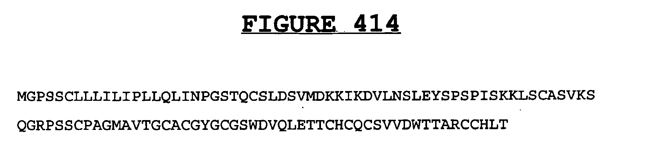

Figure 414 shows the amino acid sequence (SEQ ID NO:414) derived from the coding sequence of SEQ ID NO: 413 shown in Figure 413.

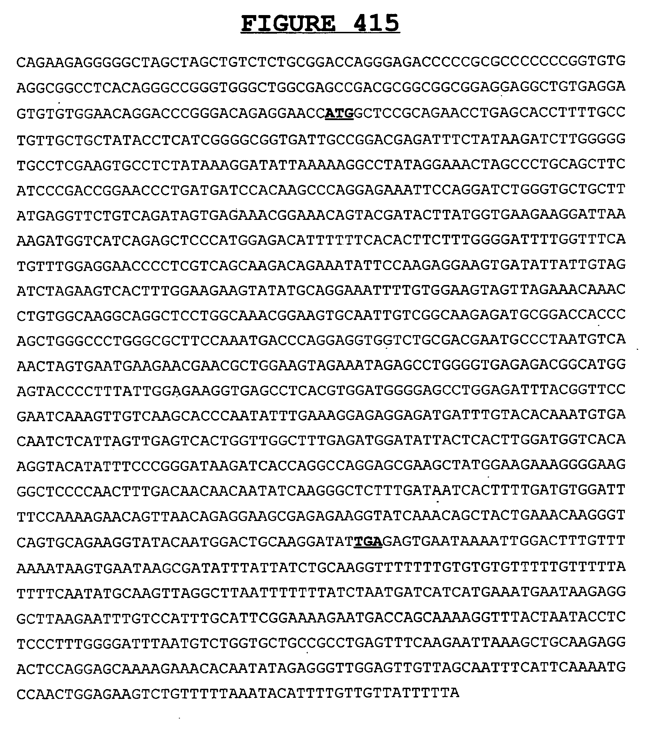

Figure 415 shows a nucleotide sequence (SEQ ID NO:415) of a native sequence PRO1080 cDNA, wherein SEQ ID NO:415 is a clone designated herein as "DNA56047-1456".

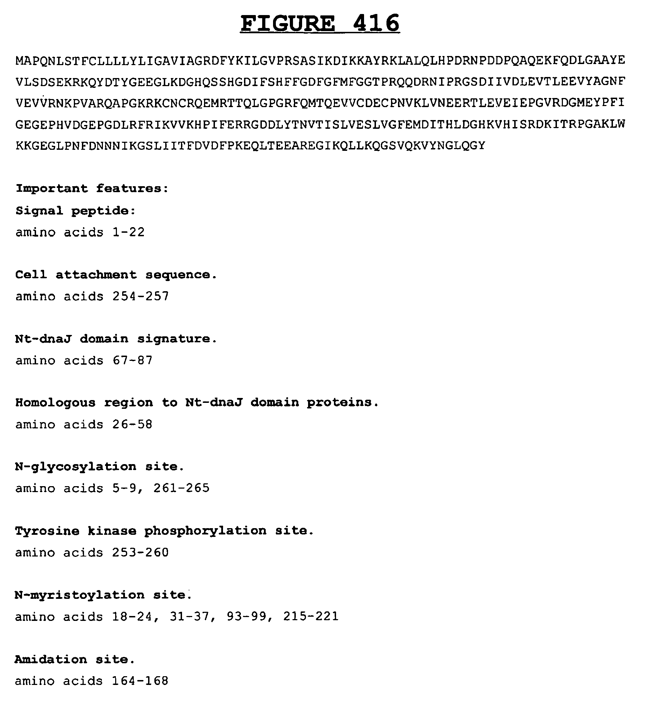

Figure 416 shows the amino acid sequence (SEQ ID NO:416) derived from the coding sequence of SEQ ID NO: 415 shown in Figure 415.

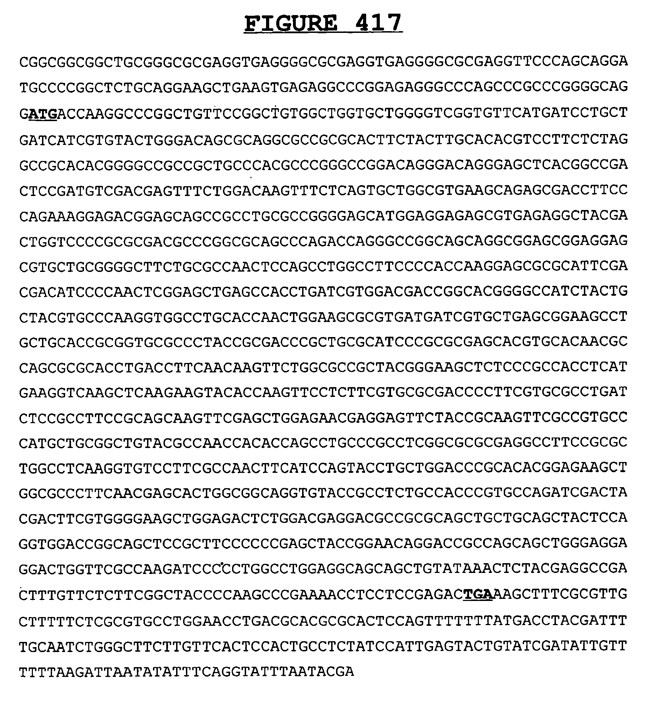

Figure 417 shows a nucleotide sequence (SEQ ID NO:417) of a native sequence PRO1017 cDNA, wherein SEQ ID NO:417 is a clone designated herein as "DNA56112-1379".

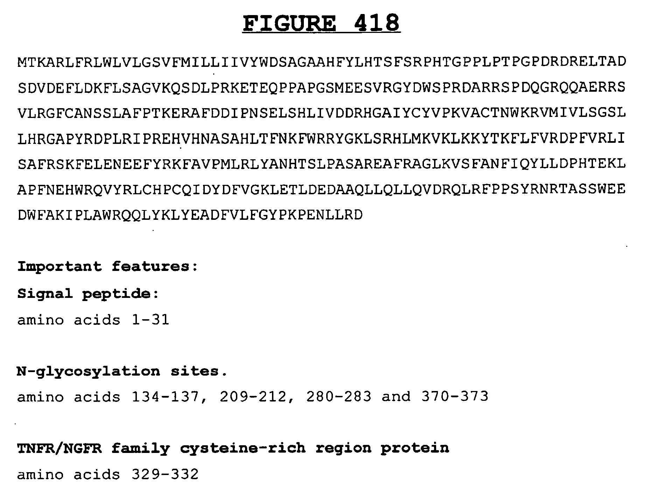

Figure 418 shows the amino acid sequence (SEQ ID NO:418) derived from the coding sequence of SEQ ID NO: 417 shown in Figure 417.

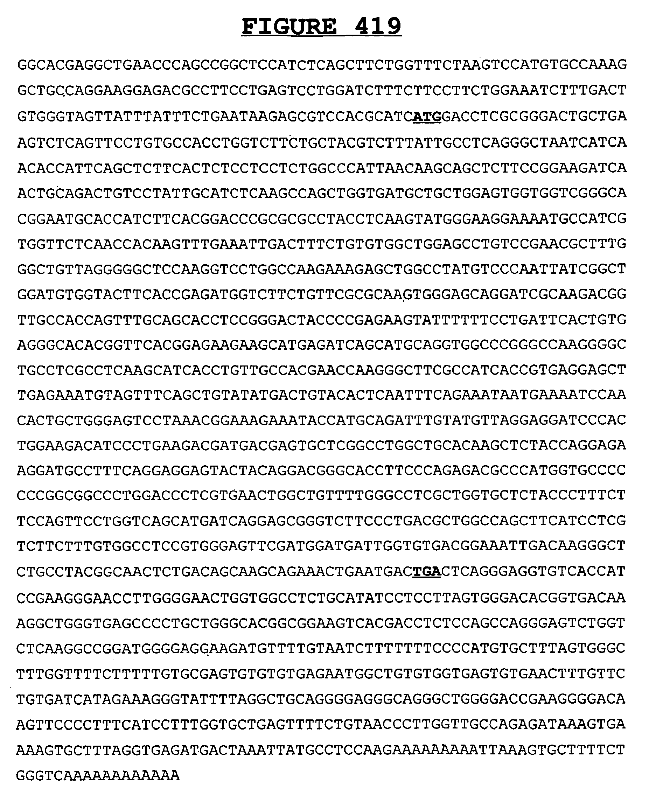

Figure 419 shows a nucleotide sequence (SEQ ID NO:419) of a native sequence PRO1016 cDNA, wherein SEQ ID NO:419 is a clone designated herein as "DNA56113-1378".

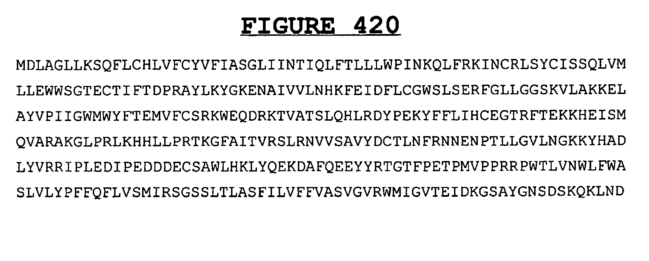

Figure 420 shows the amino acid sequence (SEQ ID NO:420) derived from the coding sequence of SEQ ID NO: 419 shown in Figure 419.

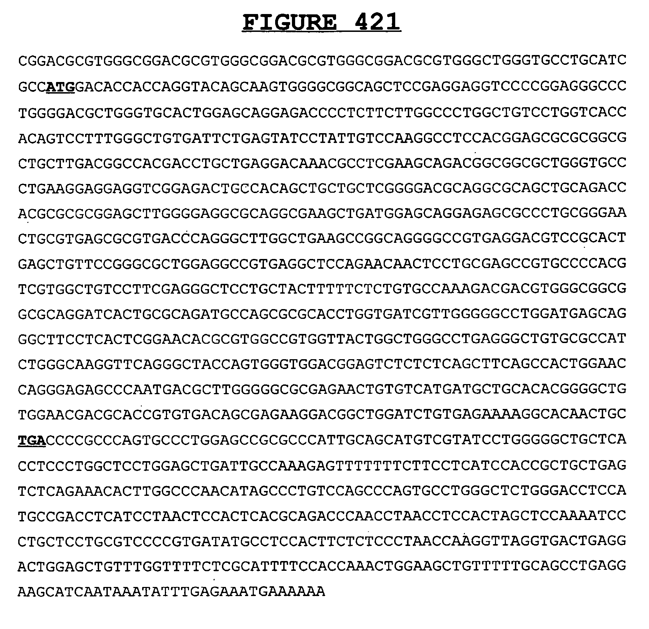

Figure 421 shows a nucleotide sequence (SEQ ID NO:421) of a native sequence PR0792 cDNA, wherein SEQ ID NO:421 is a clone designated herein as "DNA56352-1358".

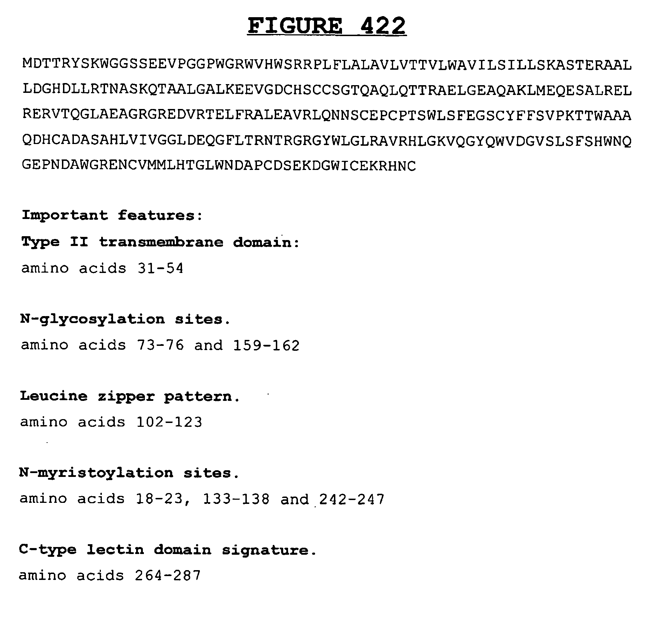

Figure 422 shows the amino acid sequence (SEQ ID NO:422) derived from the coding sequence of SEQ ID NO: 421 shown in Figure 421.

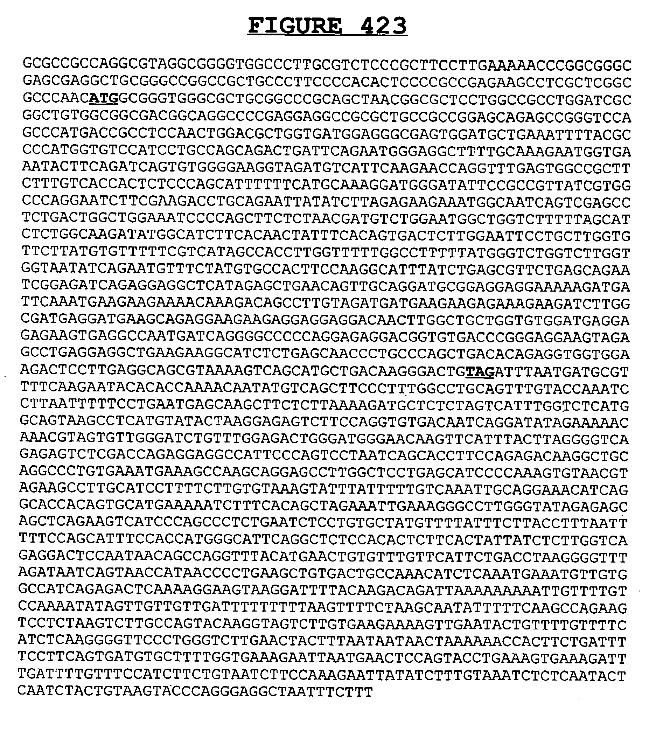

Figure 423 shows a nucleotide sequence (SEQ ID NO:423) of a native sequence PR0938 cDNA, wherein SEQ ID NO:423 is a clone designated herein as "DNA56433-1406".

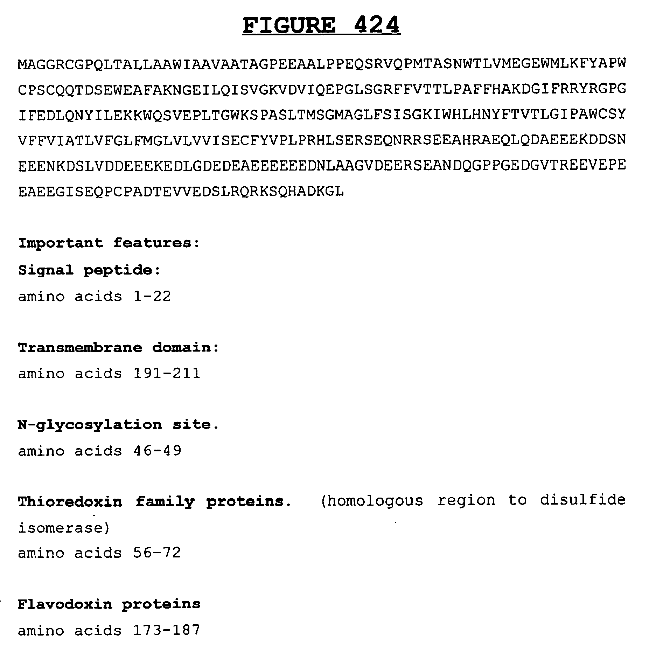

Figure 424 shows the amino acid sequence (SEQ ID NO:424) derived from the coding sequence of SEQ ID NO: 423 shown in Figure 423.

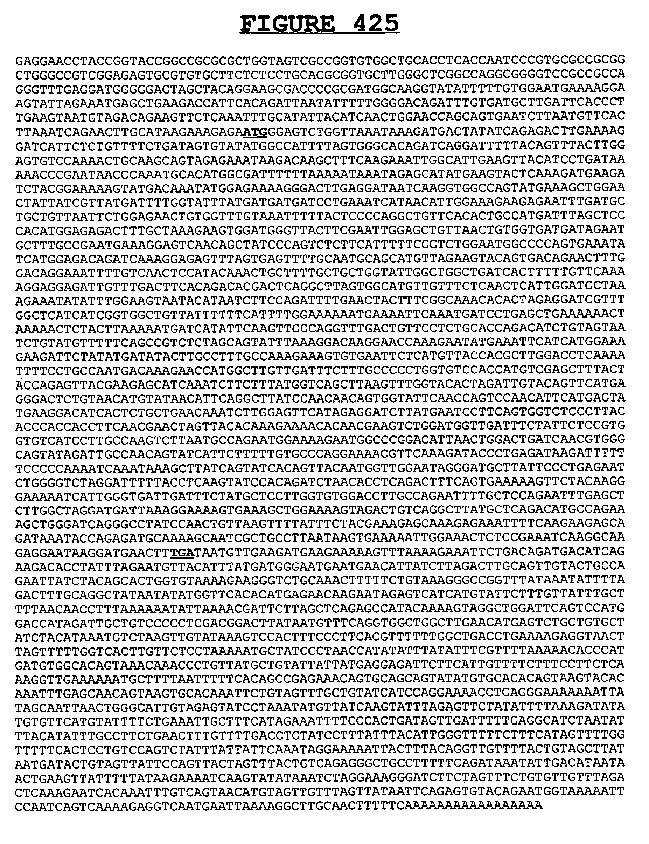

Figure 425 shows a nucleotide sequence (SEQ ID NO:425) of a native sequence PRO1012 cDNA, wherein SEQ ID NO:425 is a clone designated herein as "DNA56439-1376".

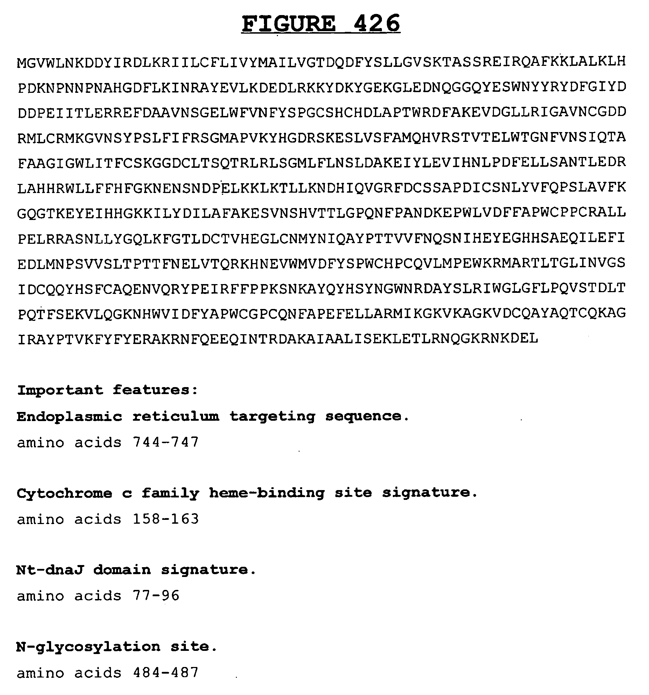

Figure 426 shows the amino acid sequence (SEQ ID NO:426) derived from the coding sequence of SEQ ID NO: 425 shown in Figure 425.

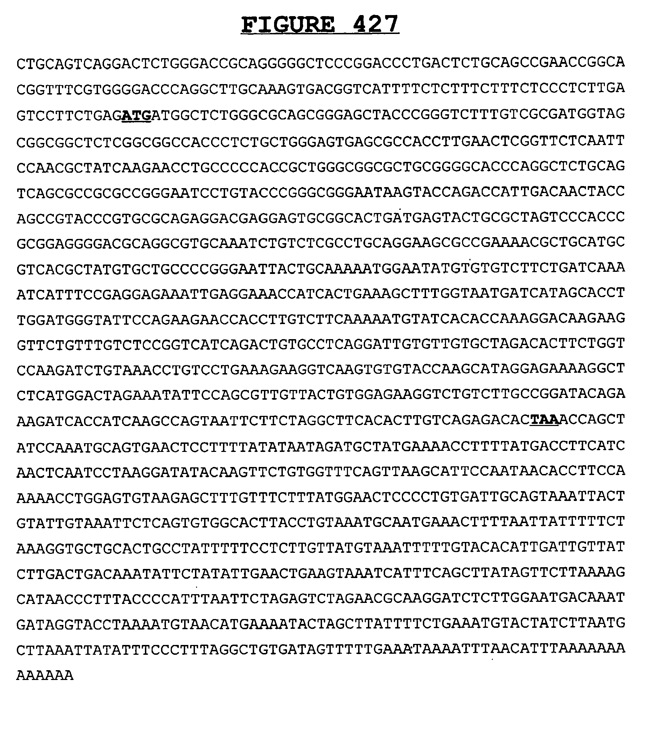

Figure 427 shows a nucleotide sequence (SEQ ID NO:427) of a native sequence PRO1008 cDNA, wherein SEQ ID NO:427 is a clone designated herein as "DNA57530-1375".

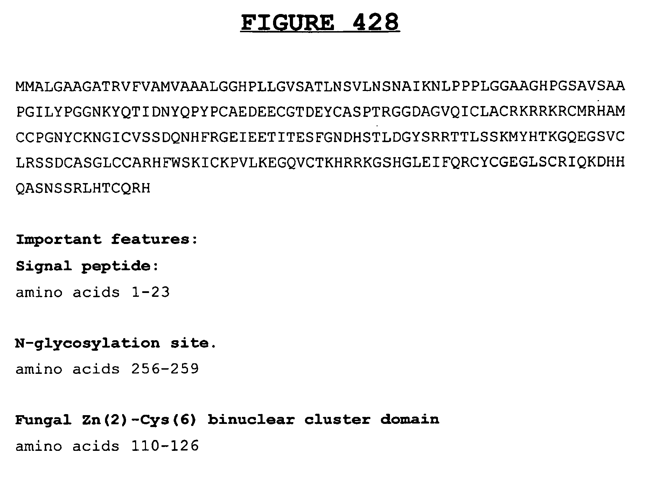

Figure 428 shows the amino acid sequence (SEQ ID NO:428) derived from the coding sequence of SEQ ID NO: 427 shown in Figure 427.

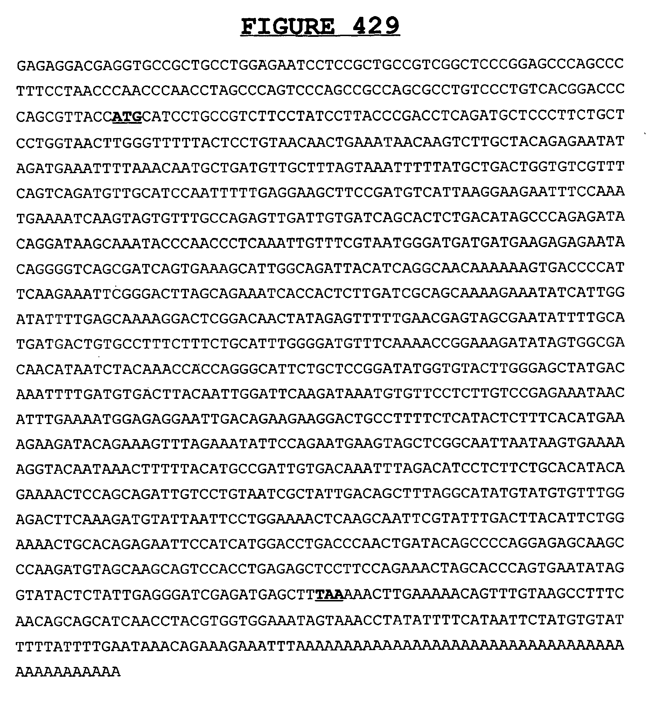

Figure 429 shows a nucleotide sequence (SEQ ID NO:429) of a native sequence PRO1075 cDNA, wherein SEQ ID NO:429 is a clone designated herein as "DNA57689-1385".

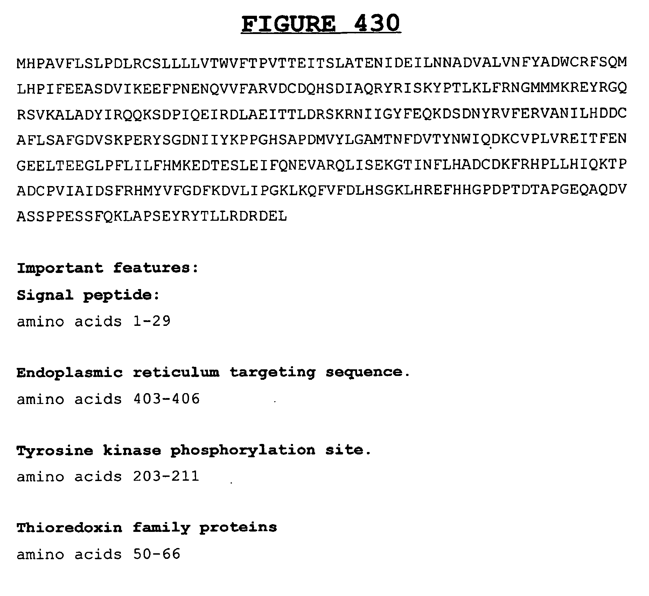

Figure 430 shows the amino acid sequence (SEQ ID NO:430) derived from the coding sequence of SEQ ID NO: 429 shown in Figure 429.

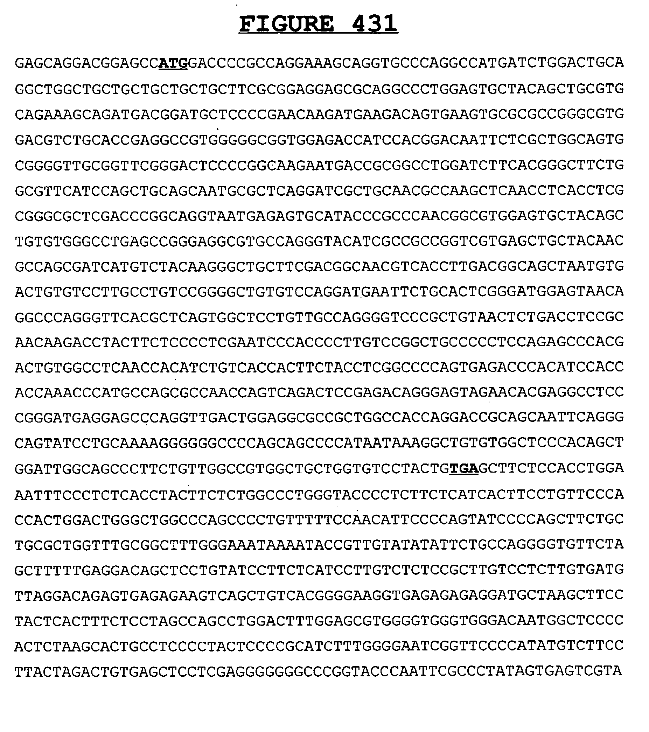

Figure 431 shows a nucleotide sequence (SEQ ID NO:431) of a native sequence PRO1007 cDNA, wherein SEQ ID NO:431 is a clone designated herein as "DNA57690-1374".

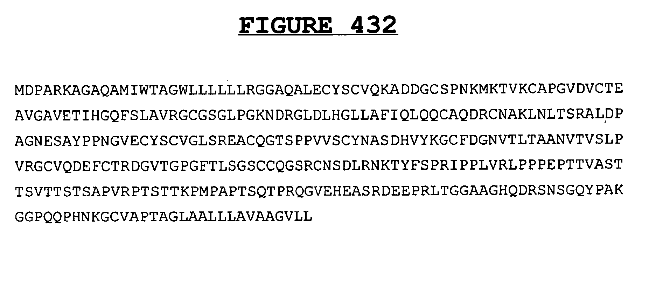

Figure 432 shows the amino acid sequence (SEQ ID NO:432) derived from the coding sequence of SEQ ID NO: 431 shown in Figure 431.

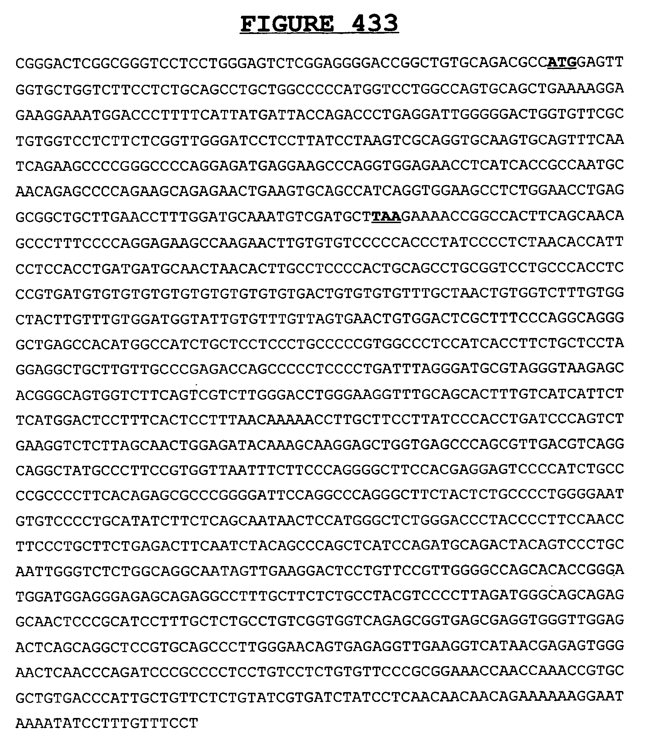

Figure 433 shows a nucleotide sequence (SEQ ID NO:433) of a native sequence PRO1056 cDNA, wherein SEQ ID NO:433 is a clone designated herein as "DNA57693-1424".

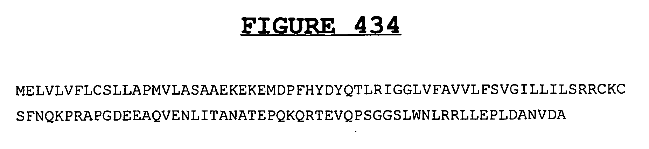

Figure 434 shows the amino acid sequence (SEQ ID NO:434) derived from the coding sequence of SEQ ID NO: 433 shown in Figure 433.

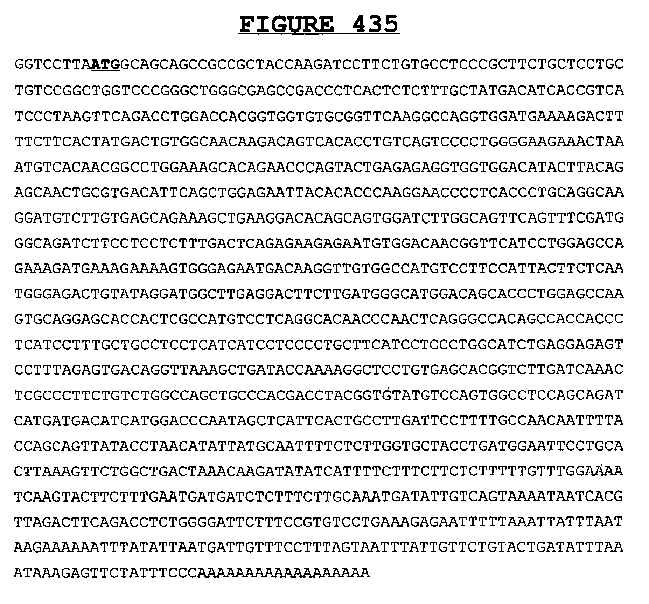

Figure 435 shows a nucleotide sequence (SEQ ID NO:435) of a native sequence PR0791 cDNA, wherein SEQ ID NO:435 is a clone designated herein as "DNA57838-1337".

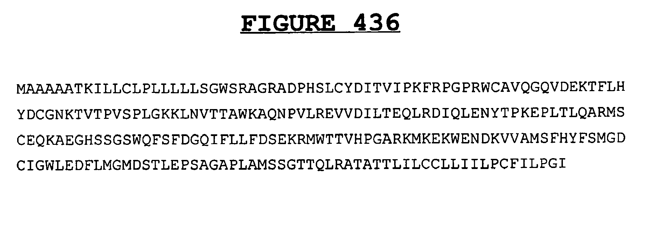

Figure 436 shows the amino acid sequence (SEQ ID NO:436) derived from the coding sequence of SEQ ID NO: 435 shown in Figure 435.

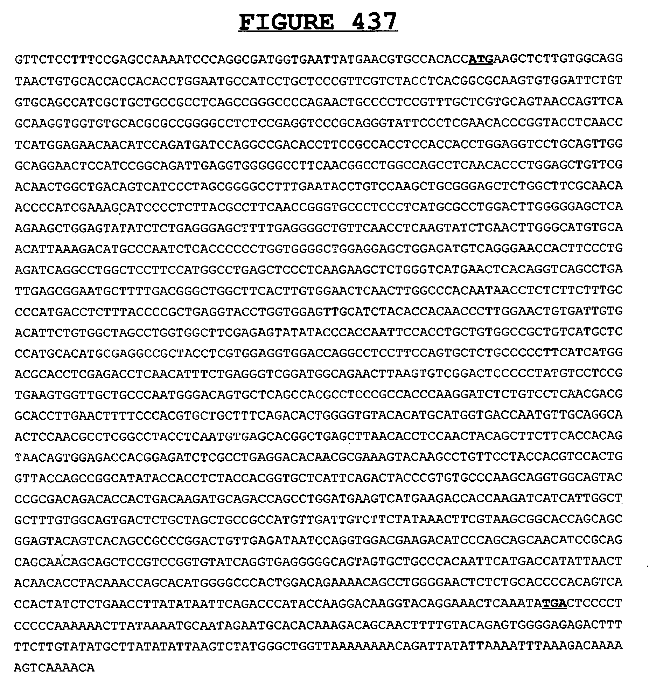

Figure 437 shows a nucleotide sequence (SEQ ID NO:437) of a native sequence PRO1111 cDNA, wherein SEQ ID NO:437 is a clone designated herein as "DNA58721-1475".

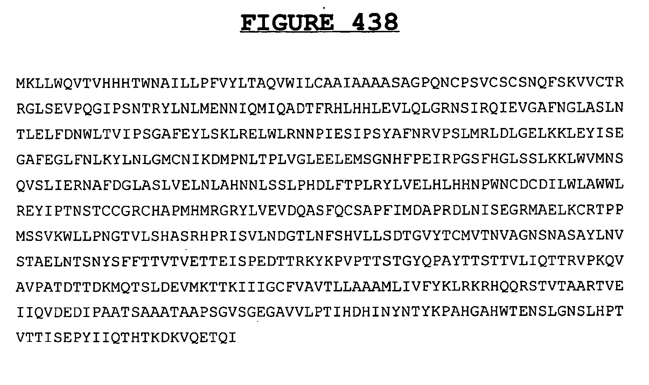

Figure 438 shows the amino acid sequence (SEQ ID NO:438) derived from the coding sequence of SEQ ID NO: 437 shown in Figure 437.

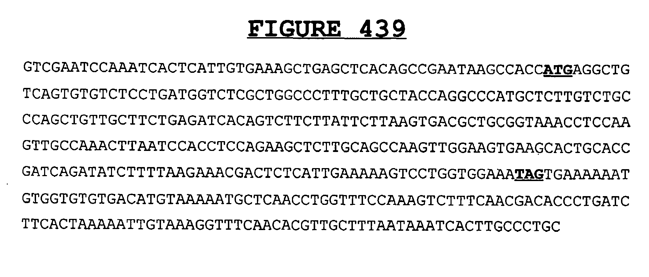

Figure 439 shows a nucleotide sequence (SEQ ID NO:439) of a native sequence PR0812 cDNA, wherein SEQ ID NO:439 is a clone designated herein as "DNA59205-1421".

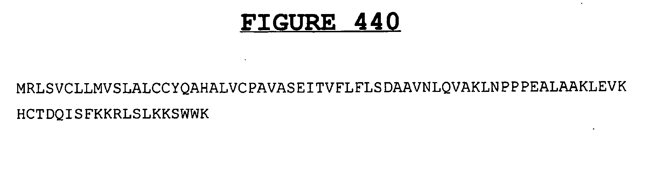

Figure 440 shows the amino acid sequence (SEQ ID NO:440) derived from the coding sequence of SEQ ID NO: 439 shown in Figure 439.

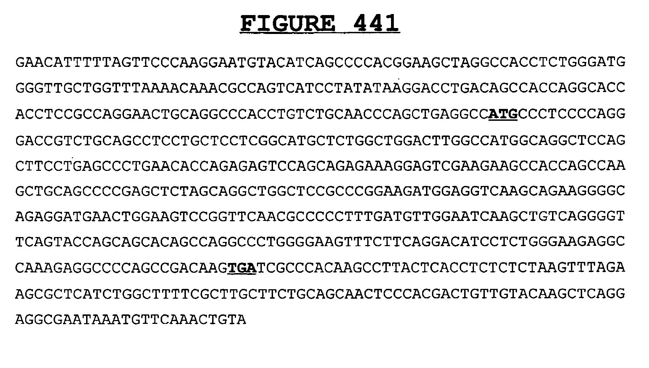

Figure 441 shows a nucleotide sequence (SEQ ID NO:441) of a native sequence PRO1066 cDNA, wherein SEQ ID NO:441 is a clone designated herein as "DNA59215-1425".

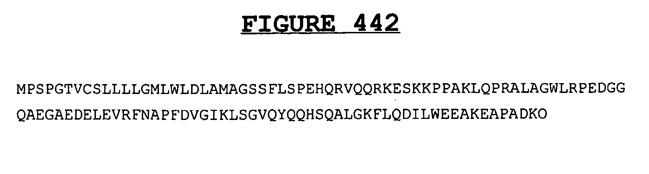

Figure 442 shows the amino acid sequence (SEQ ID NO:442) derived from the coding sequence of SEQ ID NO: 441 shown in Figure 441.

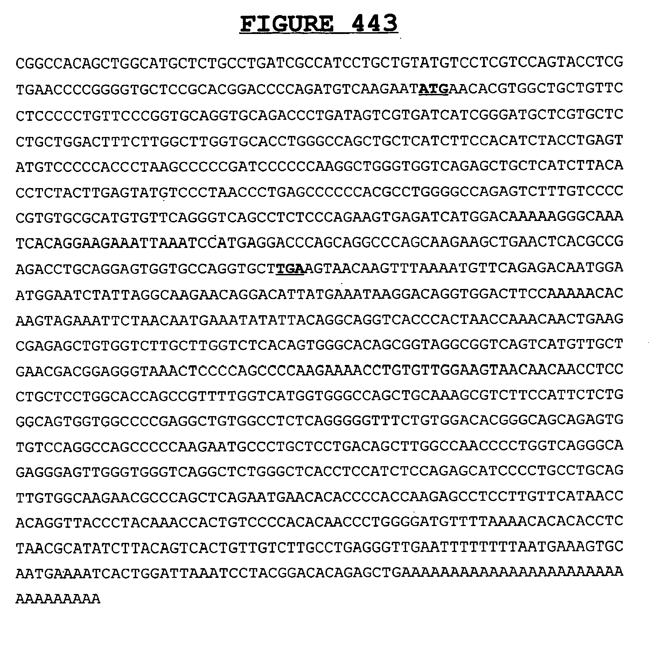

Figure 443 shows a nucleotide sequence (SEQ ID NO:443) of a native sequence PRO1185 cDNA, wherein SEQ ID NO:443 is a clone designated herein as "DNA59220-1514".

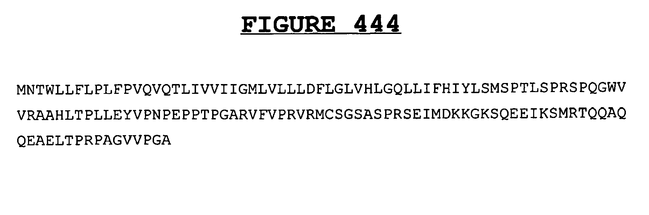

Figure 444 shows the amino acid sequence (SEQ ID NO:444) derived from the coding sequence of SEQ ID NO: 443 shown in Figure 443.

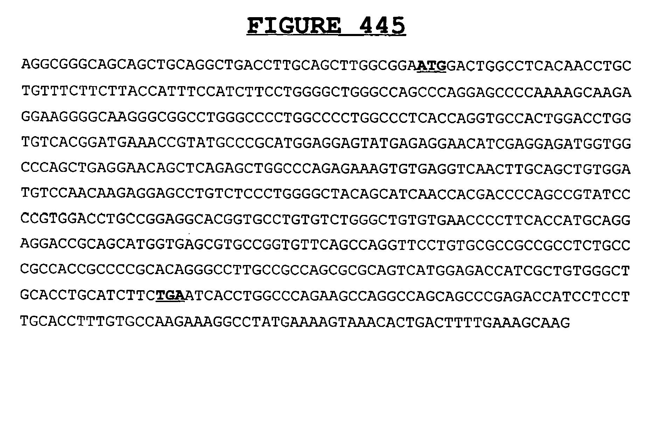

Figure 445 shows a nucleotide sequence (SEQ ID NO:445) of a native sequence PRO1031 cDNA, wherein SEQ ID NO:445 is a clone designated herein as "DNA59294-1381".

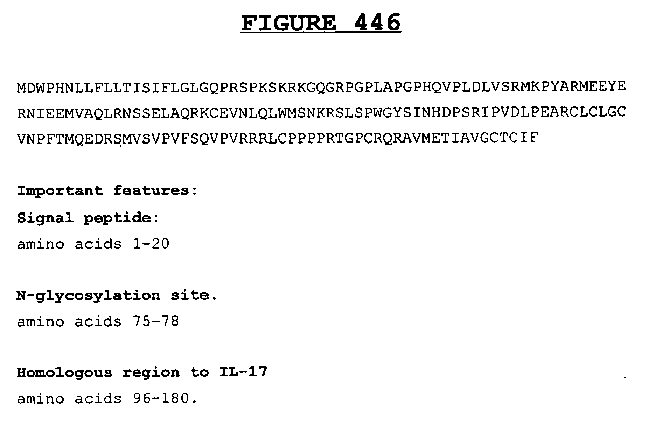

Figure 446 shows the amino acid sequence (SEQ ID NO:446) derived from the coding sequence of SEQ ID NO: 445 shown in Figure 445.

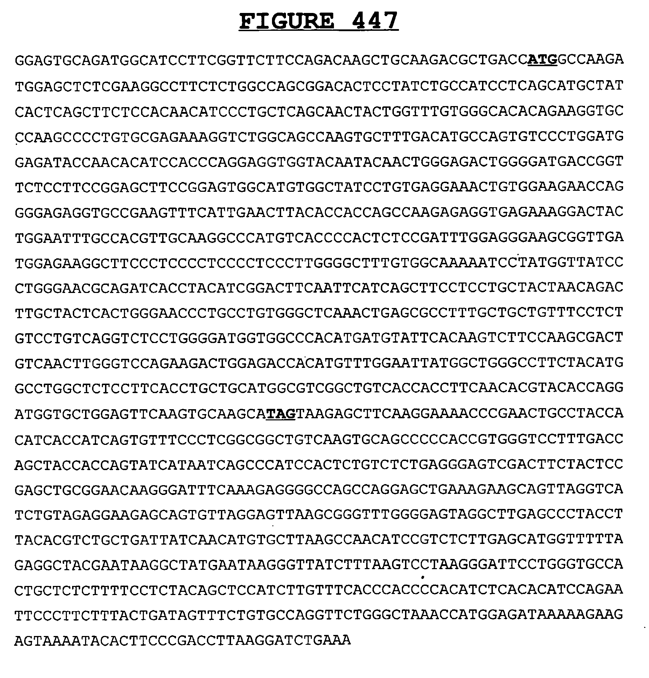

Figure 447 shows a nucleotide sequence (SEQ ID NO:447) of a native sequence PRO1360 cDNA, wherein SEQ ID NO:447 is a clone designated herein as "DNA59488-1603".

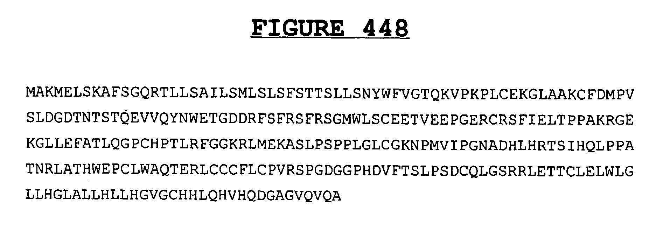

Figure 448 shows the amino acid sequence (SEQ ID NO:448) derived from the coding sequence of SEQ ID NO: 447 shown in Figure 447.

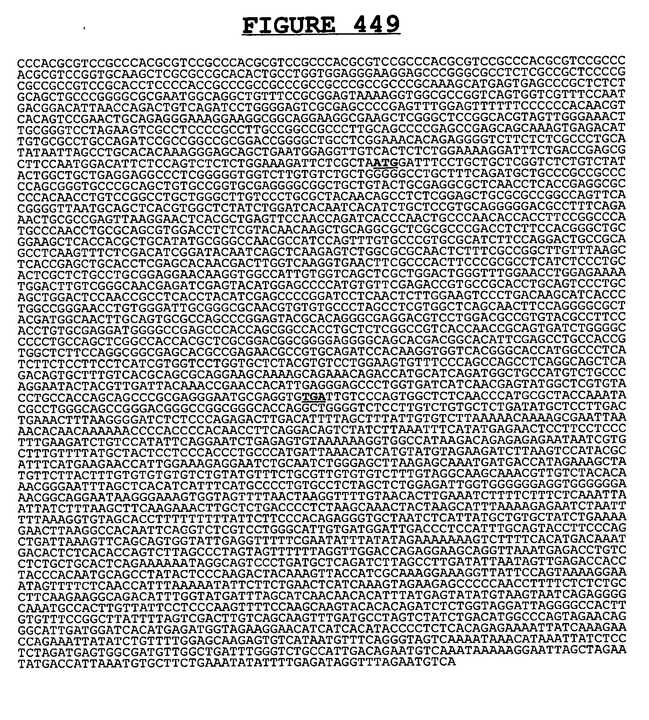

Figure 449 shows a nucleotide sequence (SEQ ID NO:449) of a native sequence PRO1309 cDNA, wherein SEQ ID NO:449 is a clone designated herein as "DNA59588-1571".

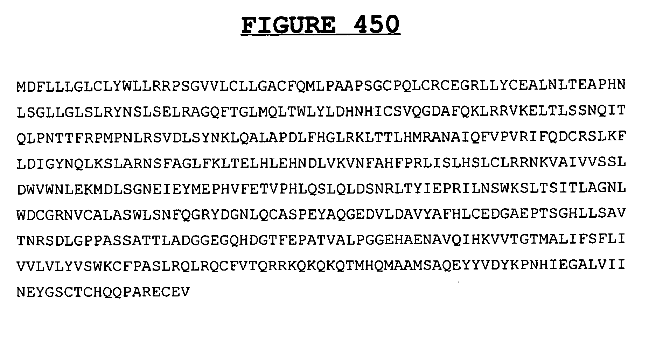

Figure 450 shows the amino acid sequence (SEQ ID NO:450) derived from the coding sequence of SEQ ID NO: 449 shown in Figure 449.

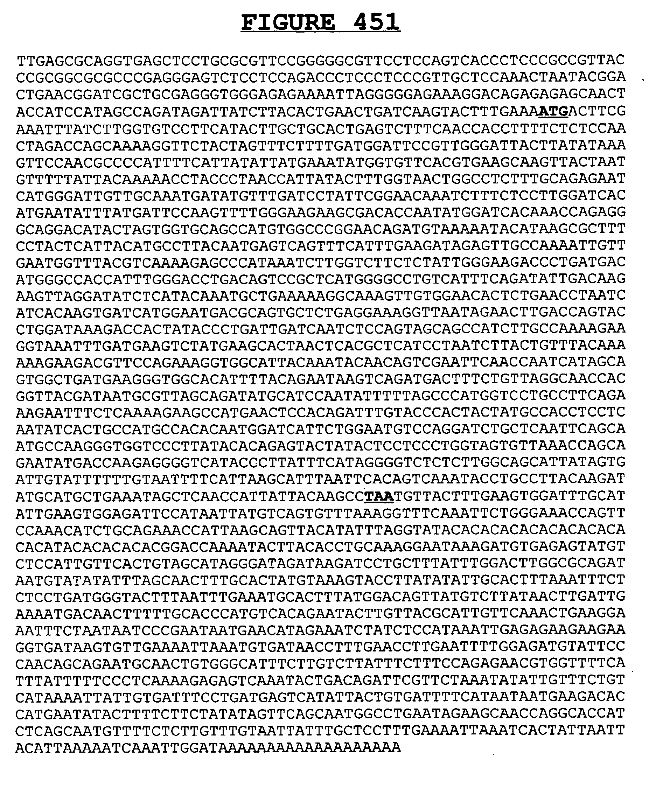

Figure 451 shows a nucleotide sequence (SEQ ID NO:451) of a native sequence PR01107 cDNA, wherein SEQ ID NO:451 is a clone designated herein as "DNA59606-1471".

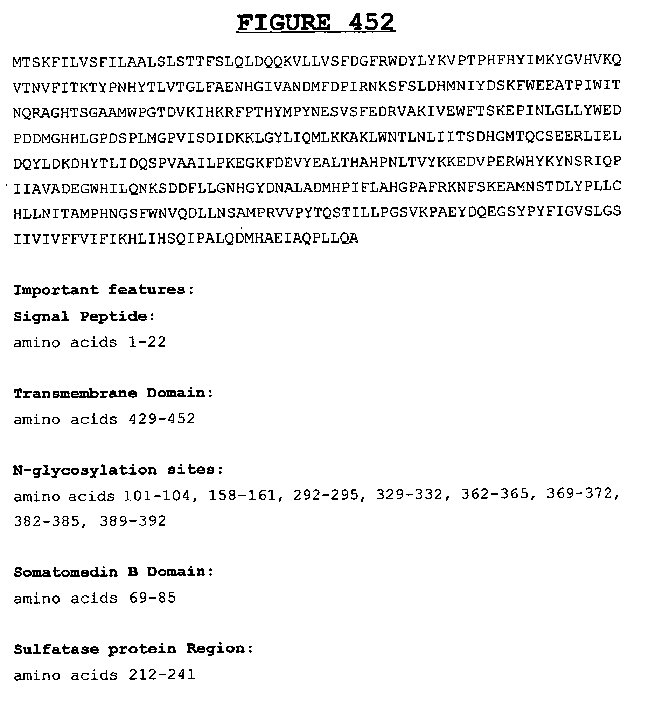

Figure 452 shows the amino acid sequence (SEQ ID NO:452) derived from the coding sequence of SEQ ID NO: 451 shown in Figure 451.

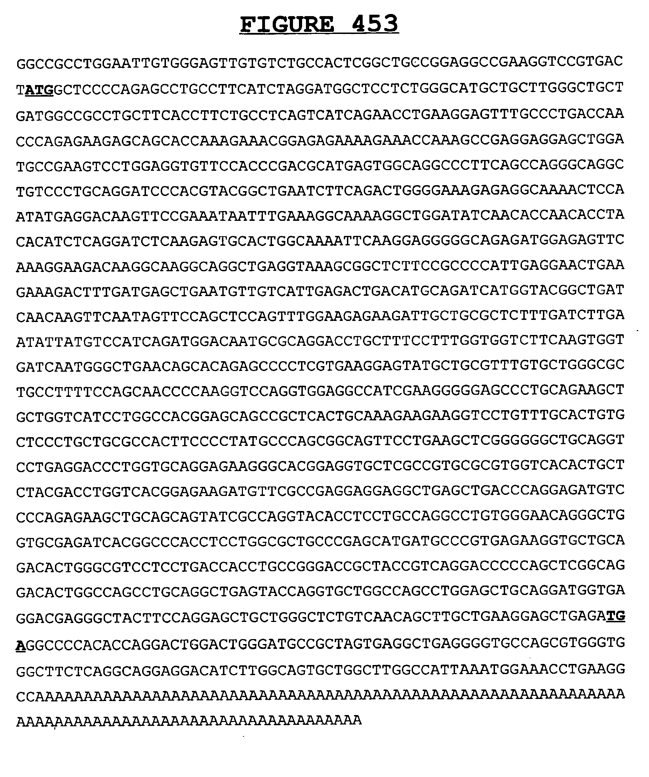

Figure 453 shows a nucleotide sequence (SEQ ID NO:453) of a native sequence PRO836 cDNA, wherein SEQ ID NO:453 is a clone designated herein as "DNA59620-1463".

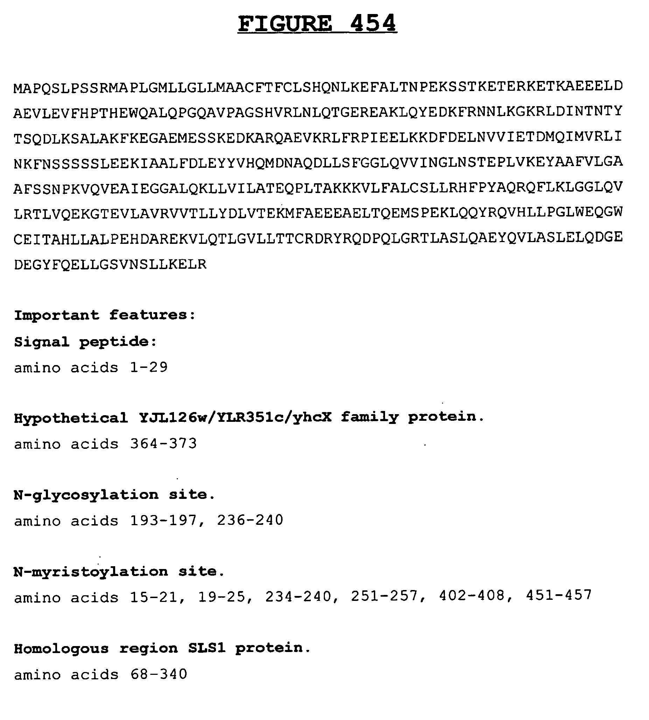

Figure 454 shows the amino acid sequence (SEQ ID NO:454) derived from the coding sequence of SEQ ID NO: 453 shown in Figure 453.

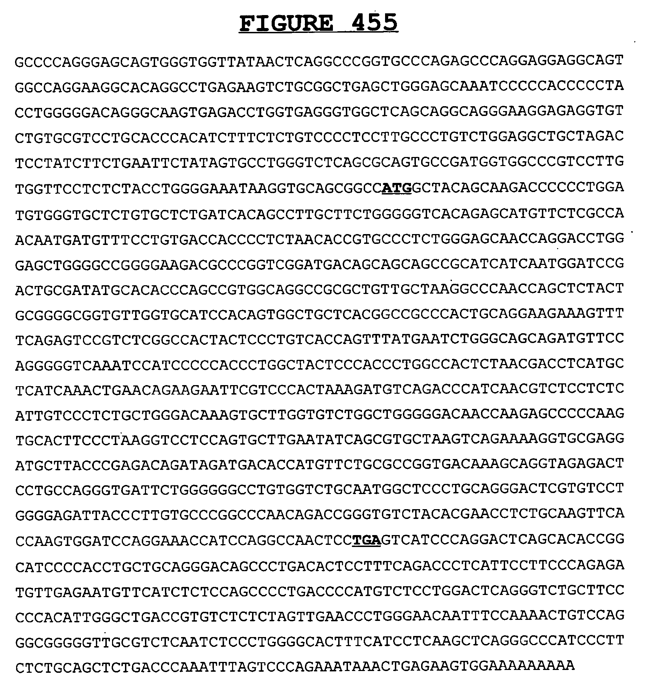

Figure 455 shows a nucleotide sequence (SEQ ID NO:455) of a native sequence PRO1132 cDNA, wherein SEQ ID NO:455 is a clone designated herein as "DNA59767-1489".

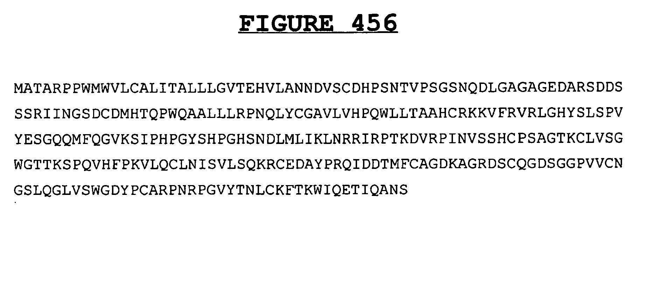

Figure 456 shows the amino acid sequence (SEQ ID NO:456) derived from the coding sequence of SEQ ID NO: 455 shown in Figure 455.

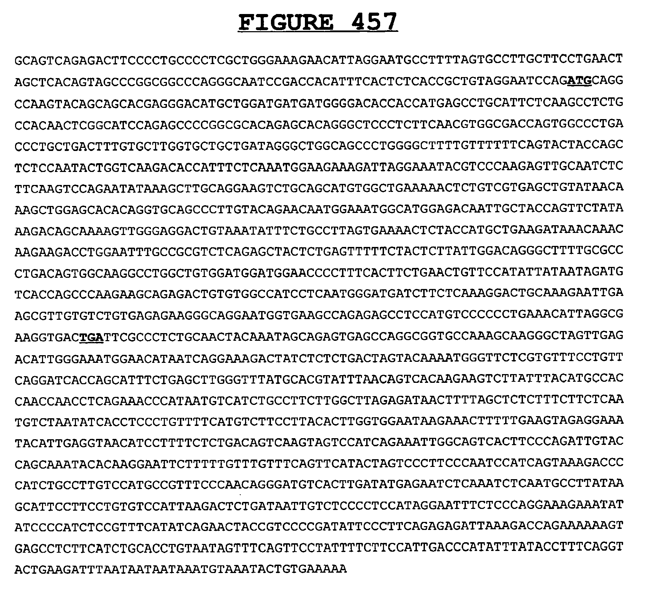

Figure 457 shows a nucleotide sequence (SEQ ID NO:457) of a native sequence PR01131 cDNA, wherein SEQ ID NO:457 is a clone designated herein as "DNA59777-1480".

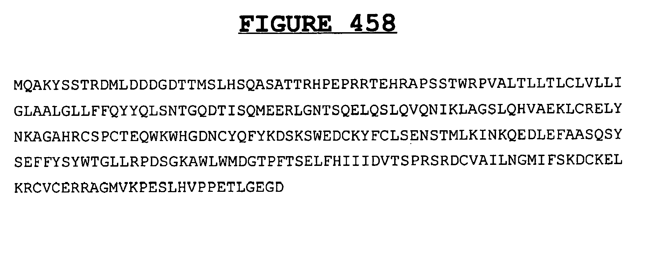

Figure 458 shows the amino acid sequence (SEQ ID NO:458) derived from the coding sequence of SEQ ID NO: 457 shown in Figure 457.

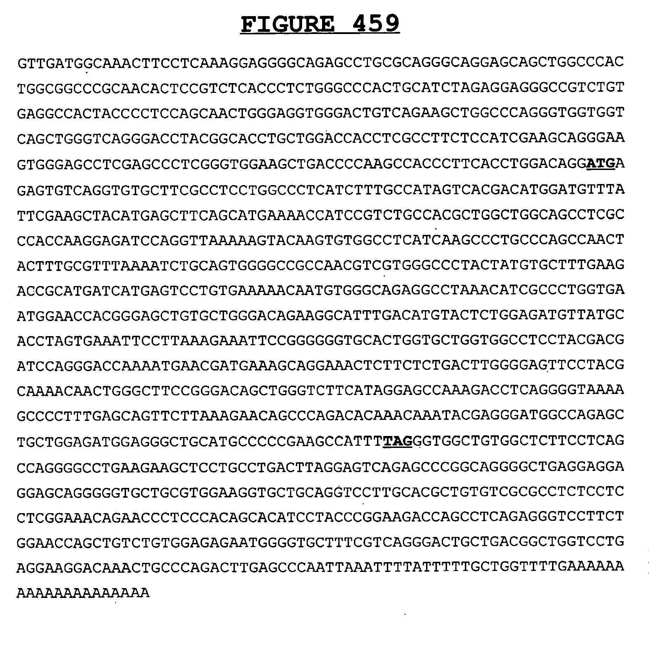

Figure 459 shows a nucleotide sequence (SEQ ID NO:459) of a native sequence PRO1130 cDNA, wherein SEQ ID NO:459 is a clone designated herein as "DNA59814-1486".

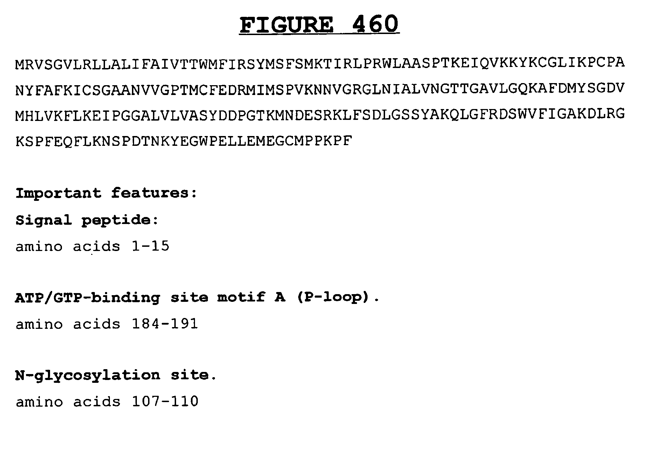

Figure 460 shows the amino acid sequence (SEQ ID NO:460) derived from the coding sequence of SEQ ID NO: 459 shown in Figure 459.

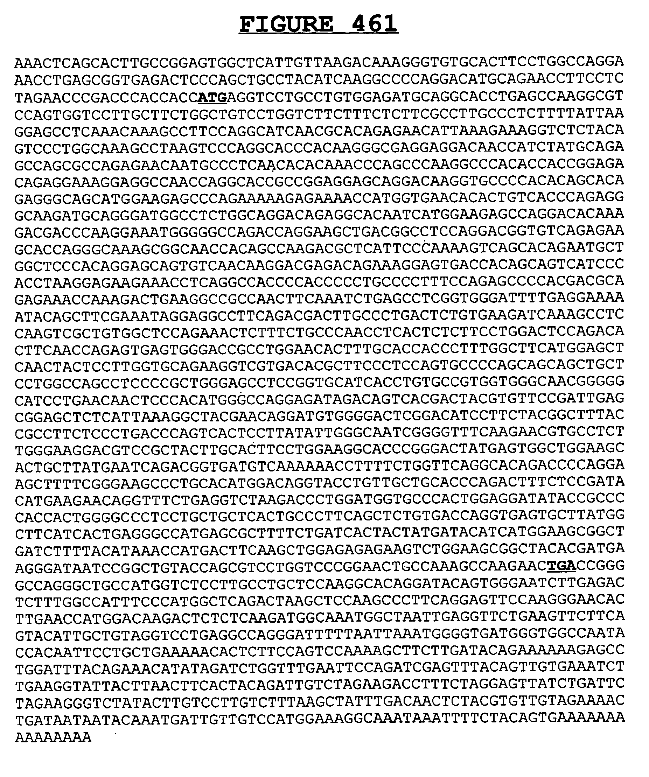

Figure 461 shows a nucleotide sequence (SEQ ID NO:461) of a native sequence PRO844 cDNA, wherein SEQ ID NO:461 is a clone designated herein as "DNA59839-1461".

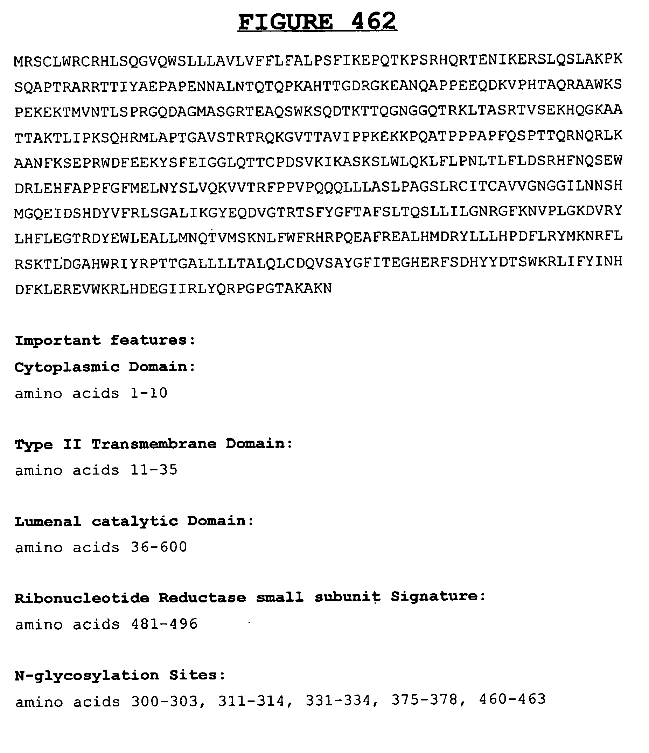

Figure 462 shows the amino acid sequence (SEQ ID NO:462) derived from the coding sequence of SEQ ID NO: 461 shown in Figure 461.

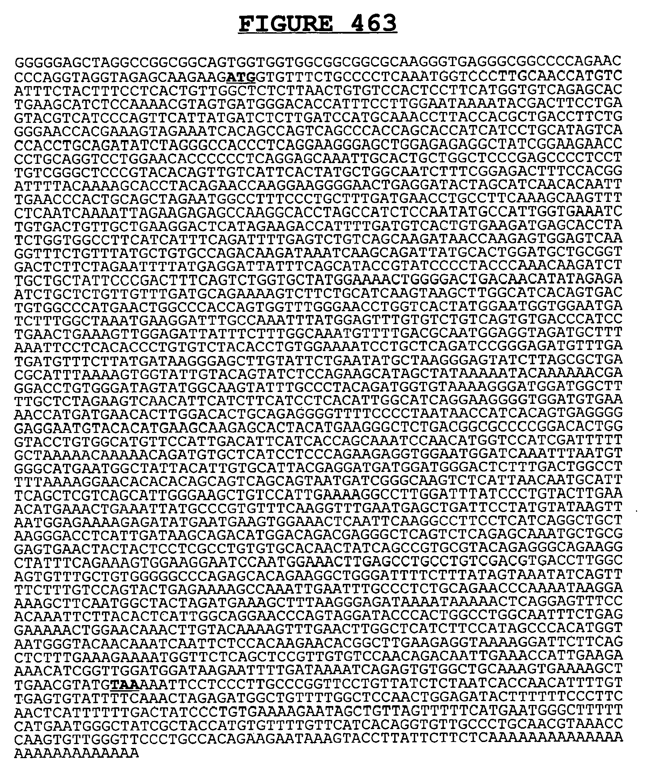

Figure 463 shows a nucleotide sequence (SEQ ID NO:463) of a native sequence PRO 1154 cDNA, wherein SEQ ID NO:463 is a clone designated herein as "DNA59846-1503".

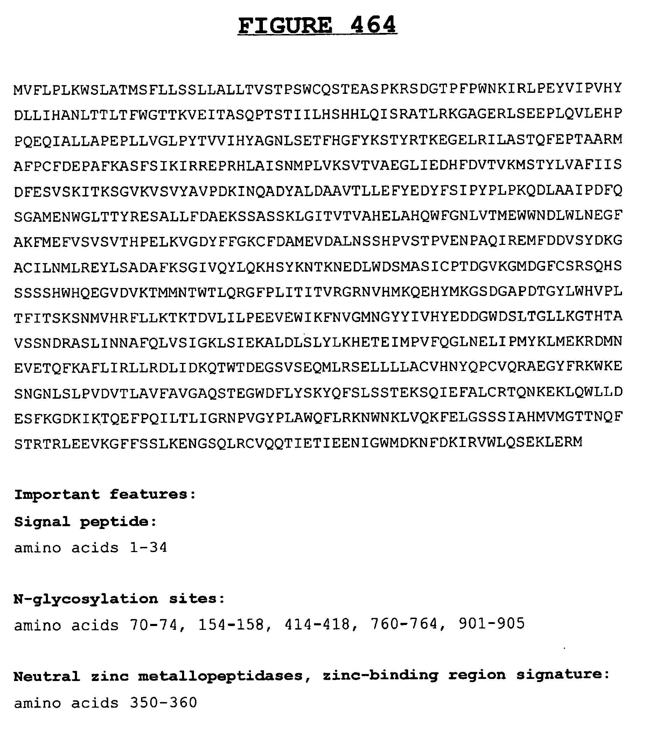

Figure 464 shows the amino acid sequence (SEQ ID NO:464) derived from the coding sequence of SEQ ID NO: 463 shown in Figure 463.

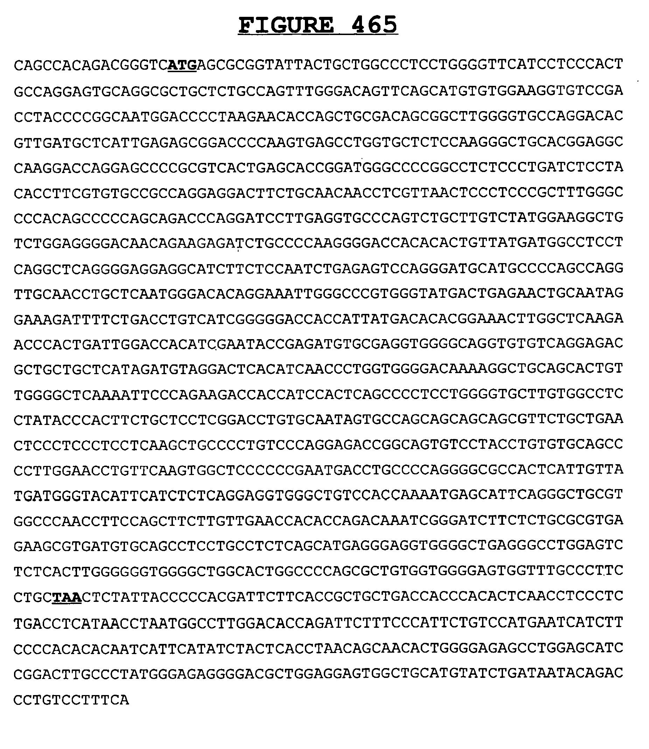

Figure 465 shows a nucleotide sequence (SEQ ID NO:465) of a native sequence PRO1181 cDNA, wherein SEQ ID NO:465 is a clone designated herein as "DNA59847-1511".

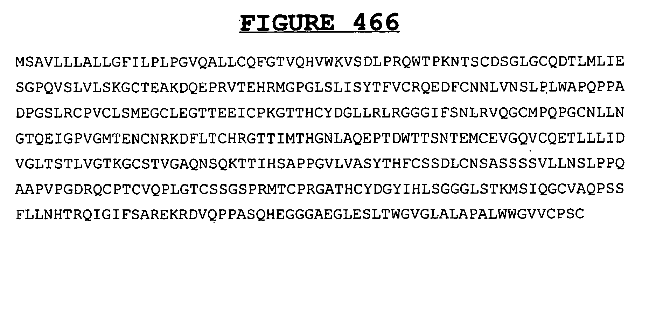

Figure 466 shows the amino acid sequence (SEQ ID NO:466) derived from the coding sequence of SEQ ID NO: 465 shown in Figure 465.

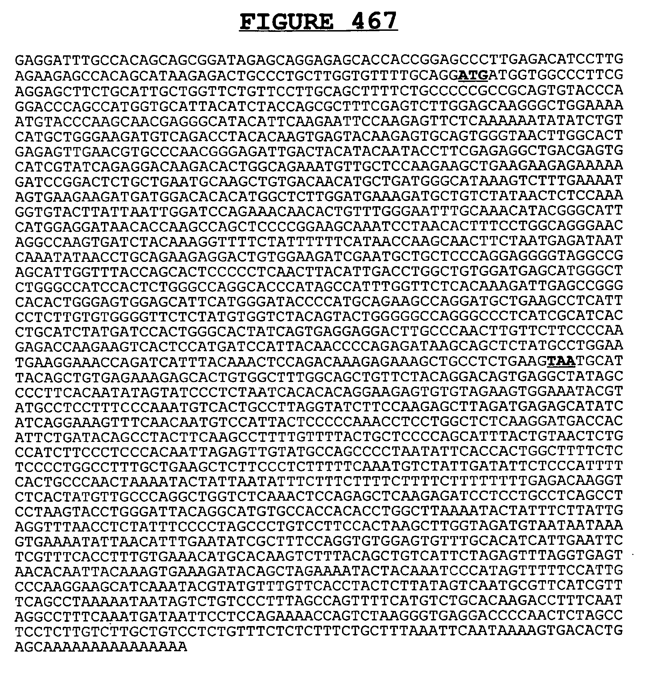

Figure 467 shows a nucleotide sequence (SEQ ID NO:467) of a native sequence PRO1126 cDNA, wherein SEQ ID NO:467 is a clone designated herein as "DNA60615-1483".

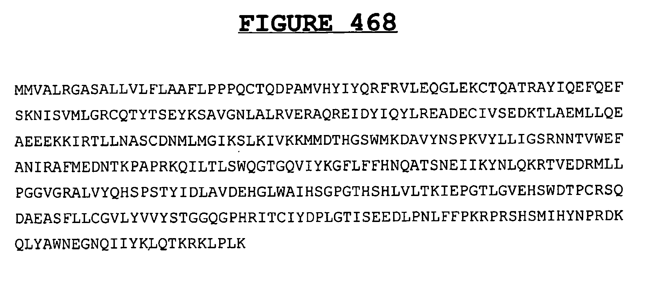

Figure 468 shows the amino acid sequence (SEQ ID NO:468) derived from the coding sequence of SEQ ID NO: 467 shown in Figure 467.

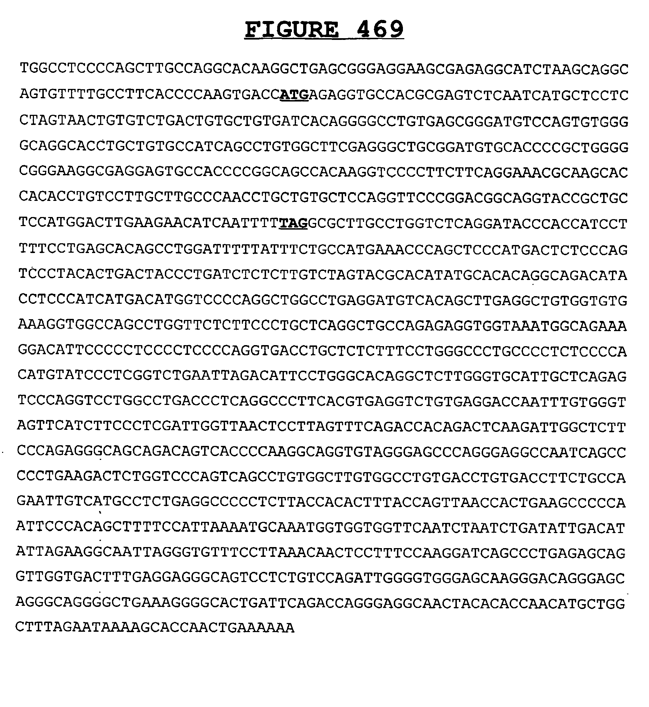

Figure 469 shows a nucleotide sequence (SEQ ID NO:469) of a native sequence PRO1186 cDNA, wherein SEQ ID NO:469 is a clone designated herein as "DNA60621-1516".

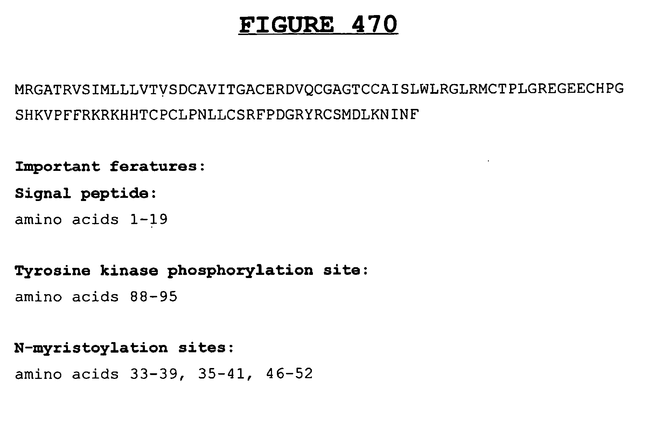

Figure 470 shows the amino acid sequence (SEQ ID NO:470) derived from the coding sequence of SEQ ID NO: 469 shown in Figure 469.

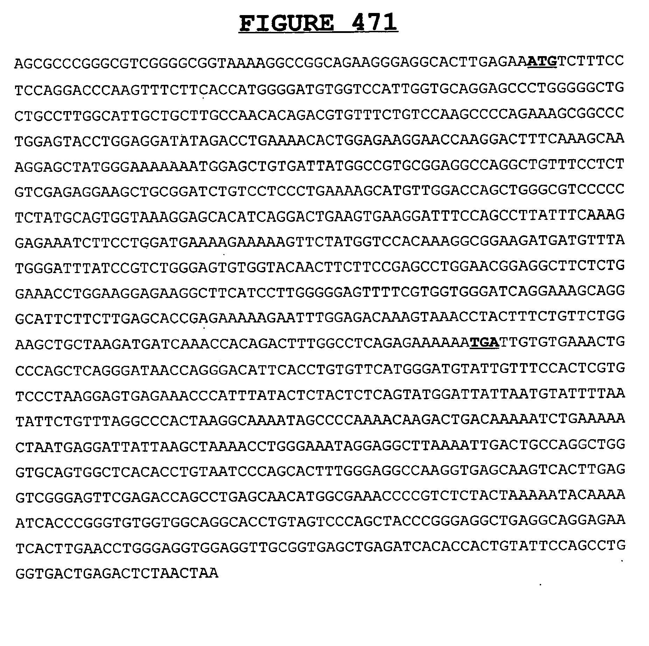

Figure 471 shows a nucleotide sequence (SEQ ID NO:471) of a native sequence PRO1198 cDNA, wherein SEQ ID NO:471 is a clone designated herein as "DNA60622-1525".

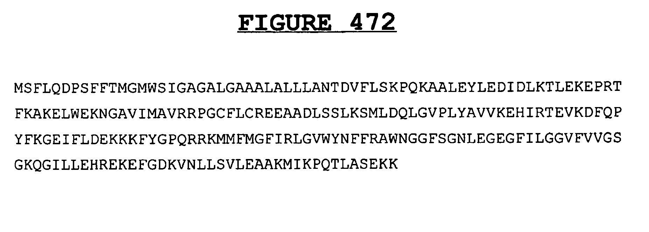

Figure 472 shows the amino acid sequence (SEQ ID NO:472) derived from the coding sequence of SEQ ID NO: 471 shown in Figure 471.

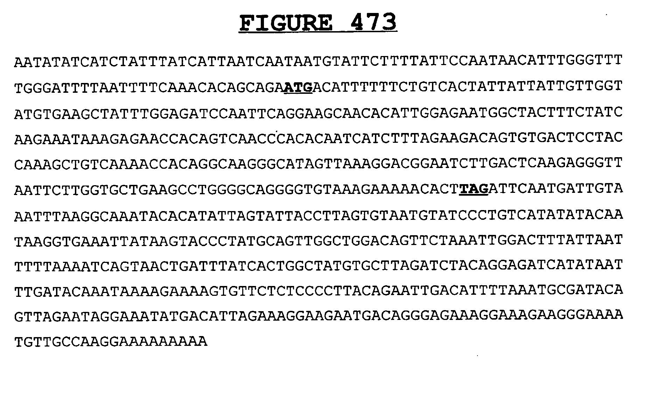

Figure 473 shows a nucleotide sequence (SEQ ID NO:473) of a native sequence PRO1159 cDNA, wherein SEQ ID NO:473 is a clone designated herein as "DNA60627-1508".

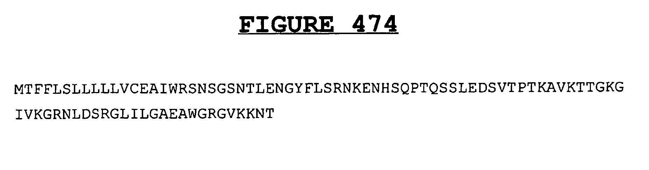

Figure 474 shows the amino acid sequence (SEQ ID NO:474) derived from the coding sequence of SEQ ID NO: 473 shown in Figure 473.

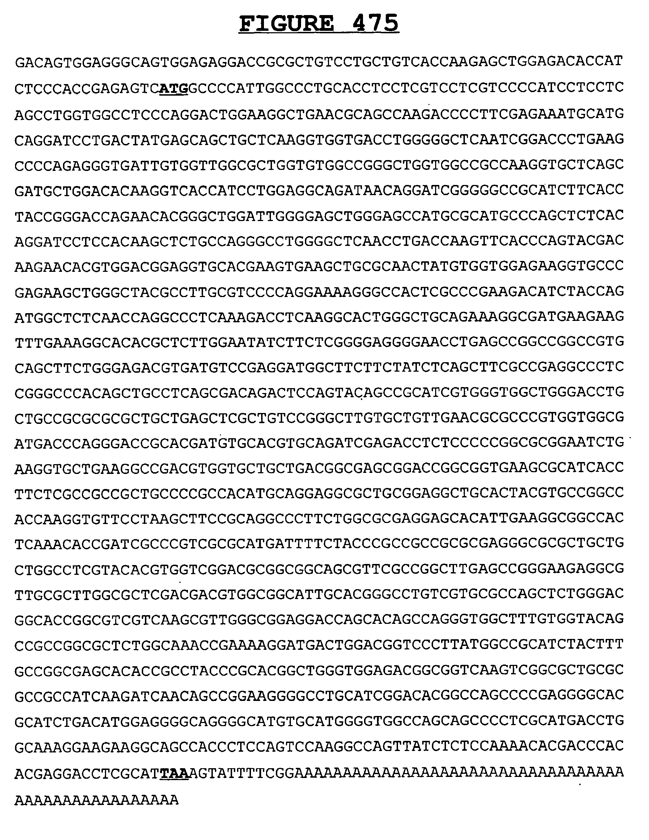

Figure 475 shows a nucleotide sequence (SEQ ID NO:475) of a native sequence PRO1265 cDNA, wherein SEQ ID NO:475 is a clone designated herein as "DNA60764-1533".

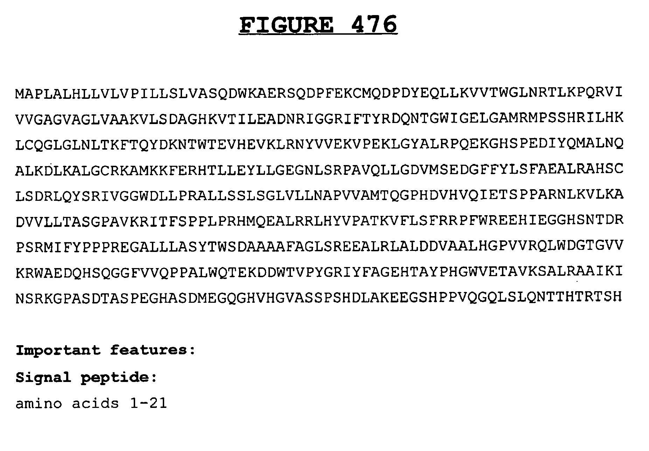

Figure 476 shows the amino acid sequence (SEQ ID NO:476) derived from the coding sequence of SEQ ID NO: 475 shown in Figure 475.

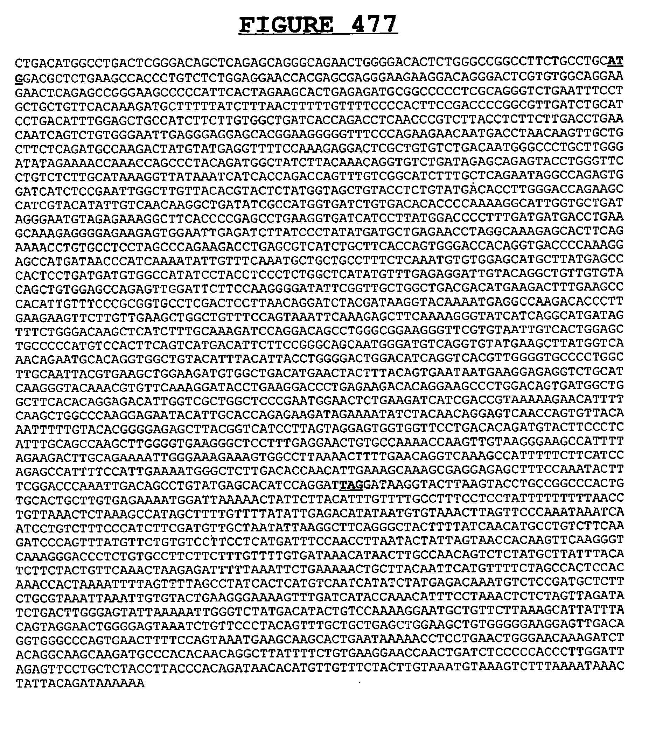

Figure 477 shows a nucleotide sequence (SEQ ID NO:477) of a native sequence PRO1250 cDNA, wherein SEQ ID NO:477 is a clone designated herein as "DNA60775-1532".

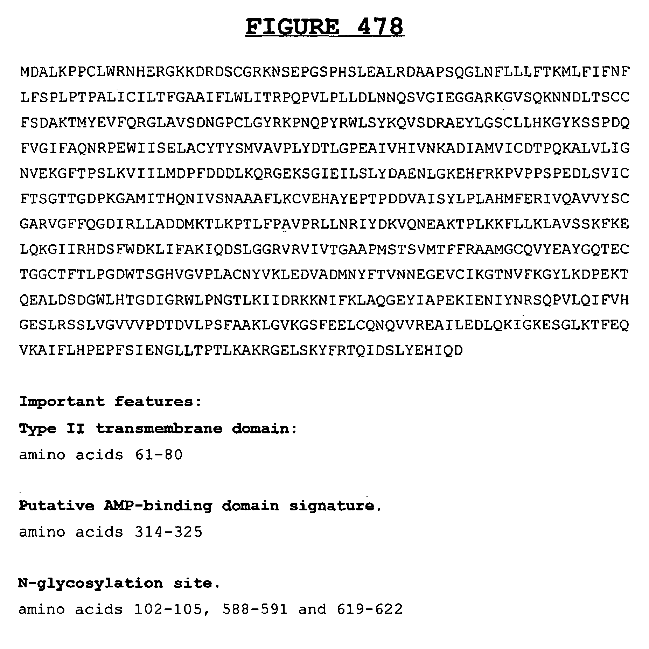

Figure 478 shows the amino acid sequence (SEQ ID NO:478) derived from the coding sequence of SEQ ID NO: 477 shown in Figure 477.

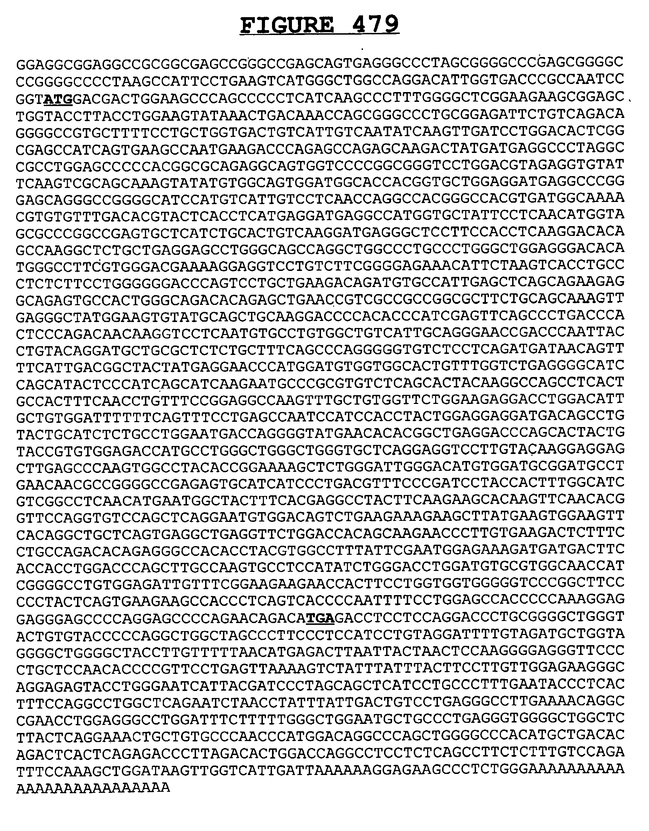

Figure 479 shows a nucleotide sequence (SEQ ID NO:479) of a native sequence PRO1475 cDNA, wherein SEQ ID NO:479 is a clone designated herein as "DNA61185-1646".

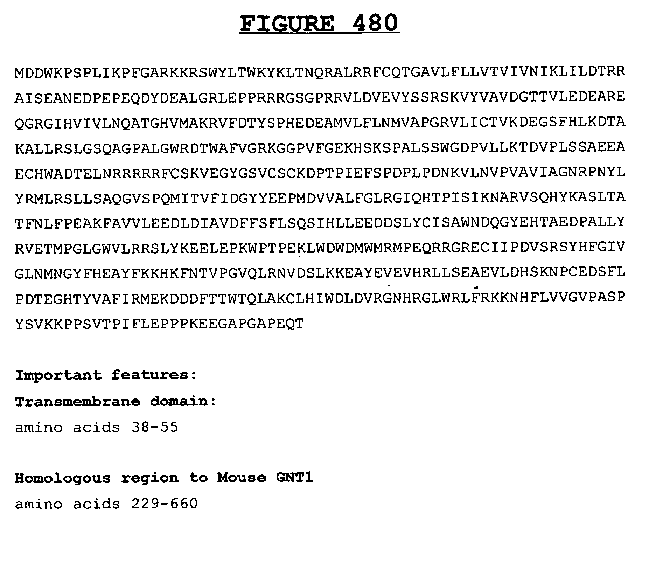

Figure 480 shows the amino acid sequence (SEQ ID NO:480) derived from the coding sequence of SEQ ID NO: 479 shown in Figure 479.

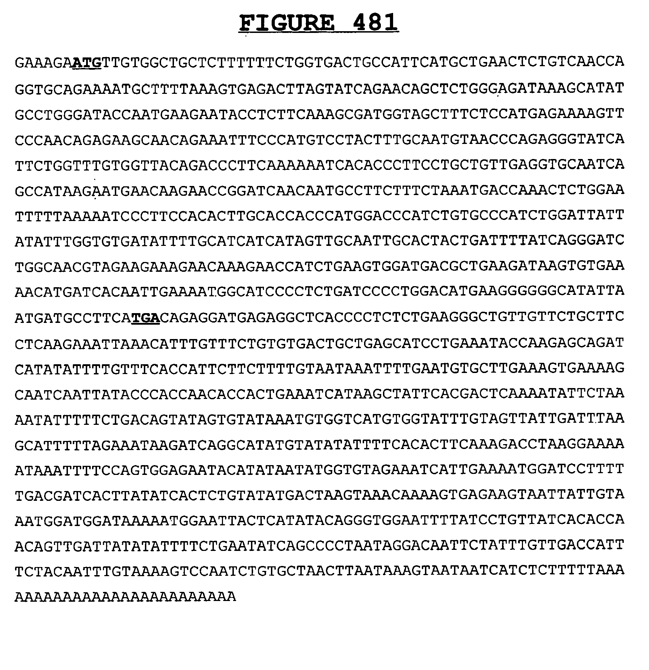

Figure 481 shows a nucleotide sequence (SEQ ID NO:481) of a native sequence PRO1312 cDNA, wherein SEQ ID NO:481 is a clone designated herein as "DNA61873-1574".

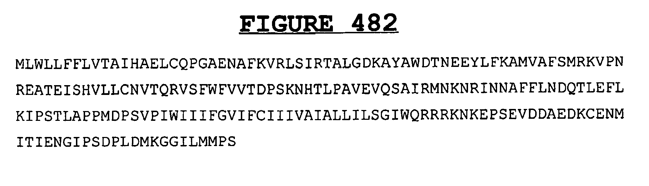

Figure 482 shows the amino acid sequence (SEQ ID NO:482) derived from the coding sequence of SEQ ID NO: 481 shown in Figure 481.

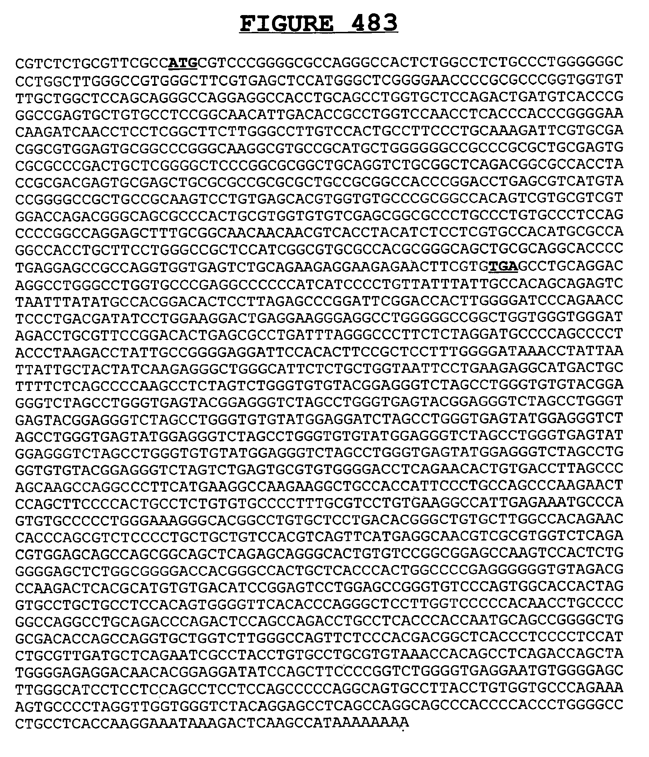

Figure 483 shows a nucleotide sequence (SEQ ID NO:483) of a native sequence PR01308 cDNA, wherein SEQ ID NO:483 is a clone designated herein as "DNA62306-1570".

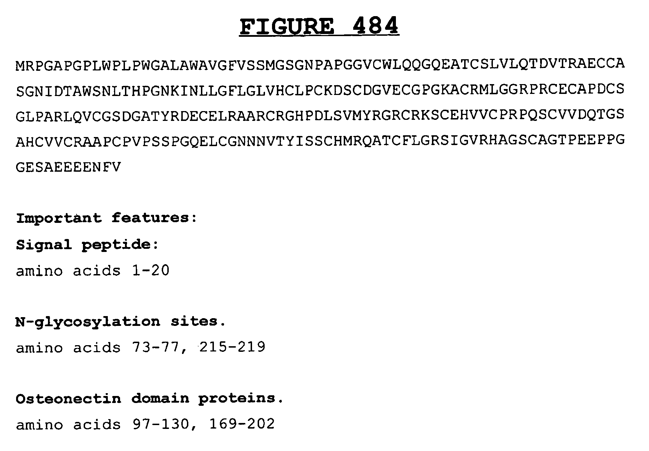

Figure 484 shows the amino acid sequence (SEQ ID NO:484) derived from the coding sequence of SEQ ID NO: 483 shown in Figure 483.

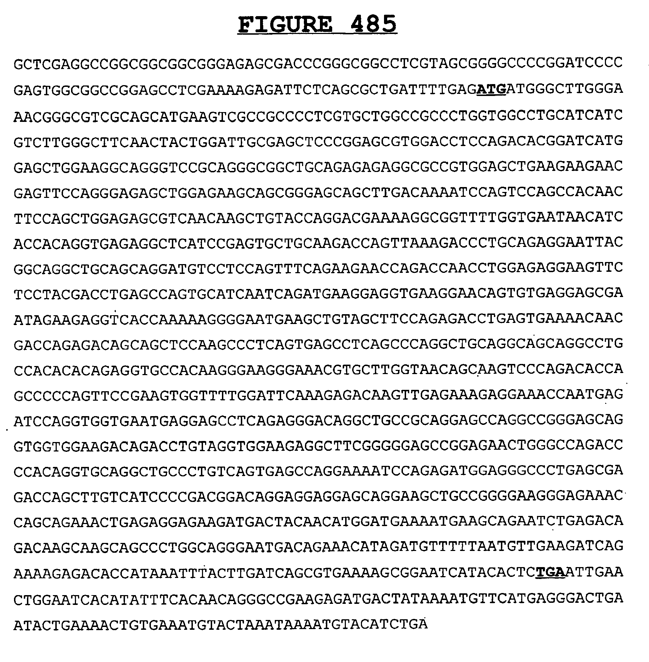

Figure 485 shows a nucleotide sequence (SEQ ID NO:485) of a native sequence PRO1326 cDNA, wherein SEQ ID NO:485 is a clone designated herein as "DNA62808-1582".

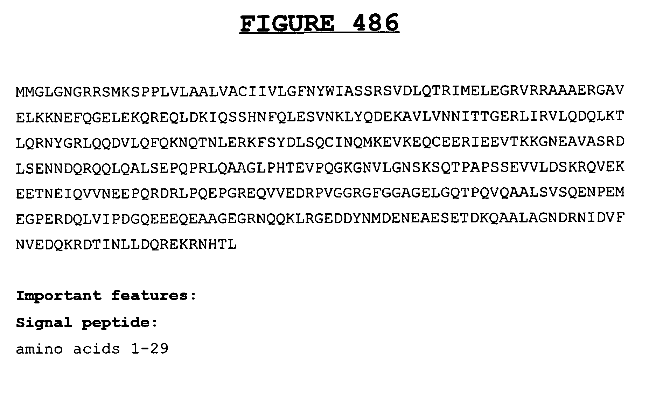

Figure 486 shows the amino acid sequence (SEQ ID NO:486) derived from the coding sequence of SEQ ID NO: 485 shown in Figure 485.

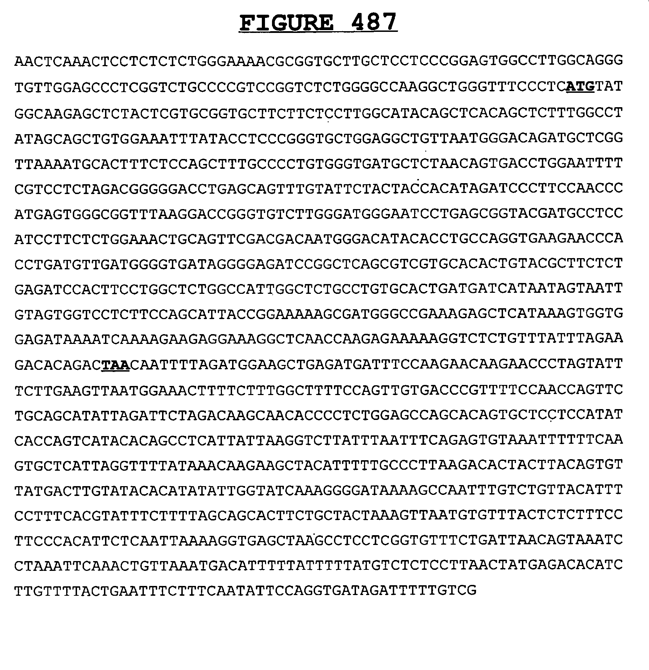

Figure 487 shows a nucleotide sequence (SEQ ID NO:487) of a native sequence PRO1192 cDNA, wherein SEQ ID NO:487 is a clone designated herein as "DNA62814-1521".

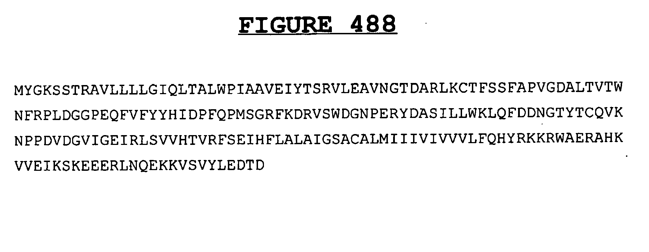

Figure 488 shows the amino acid sequence (SEQ ID NO:488) derived from the coding sequence of SEQ ID NO: 487 shown in Figure 487.

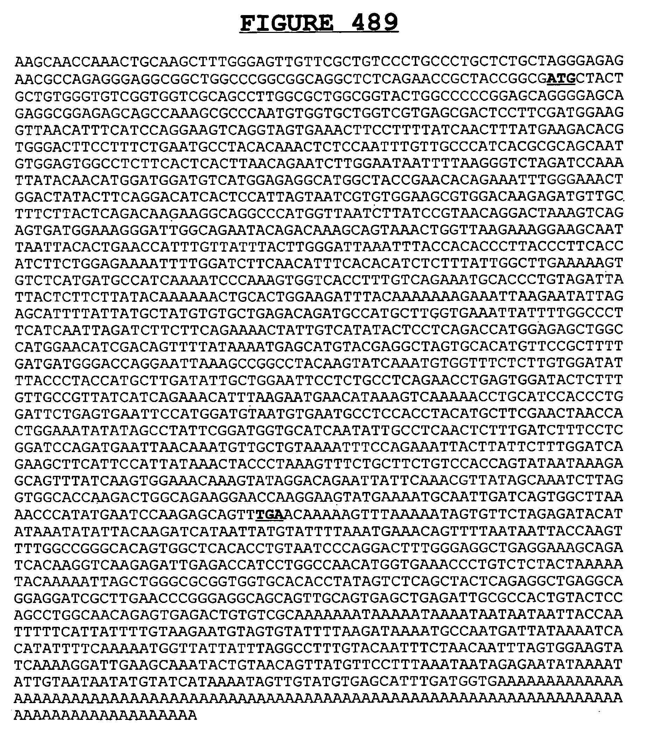

Figure 489 shows a nucleotide sequence (SEQ ID NO:489) of a native sequence PRO1246 cDNA, wherein SEQ ID NO:489 is a clone designated herein as "DNA64885-1529".

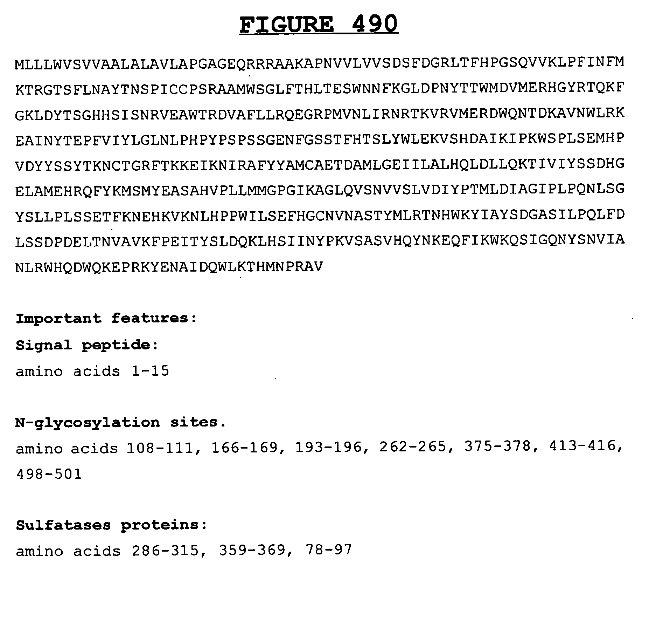

Figure 490 shows the amino acid sequence (SEQ ID NO:490) derived from the coding sequence of SEQ ID NO: 489 shown in Figure 489.

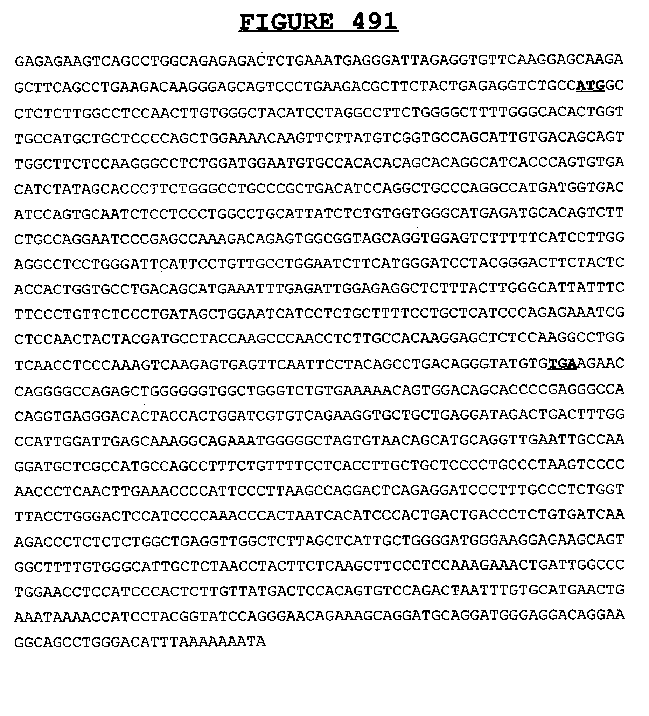

Figure 491 shows a nucleotide sequence (SEQ ID NO:491) of a native sequence PRO1356 cDNA, wherein SEQ ID NO:491 is a clone designated herein as "DNA64886-1601".

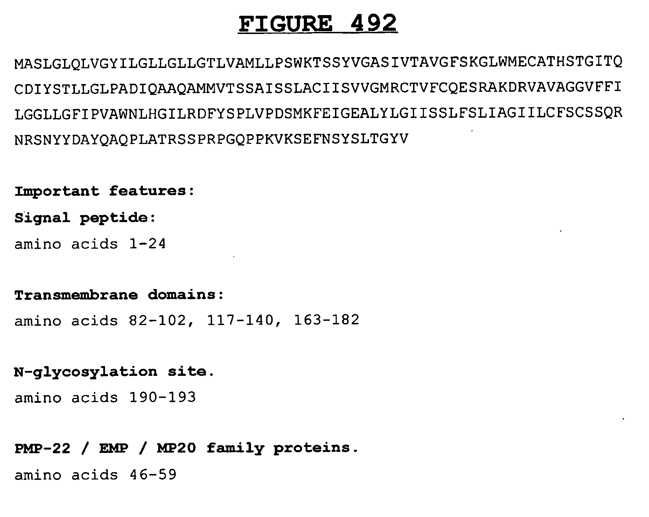

Figure 492 shows the amino acid sequence (SEQ ID NO:492) derived from the coding sequence of SEQ ID NO: 491 shown in Figure 491.

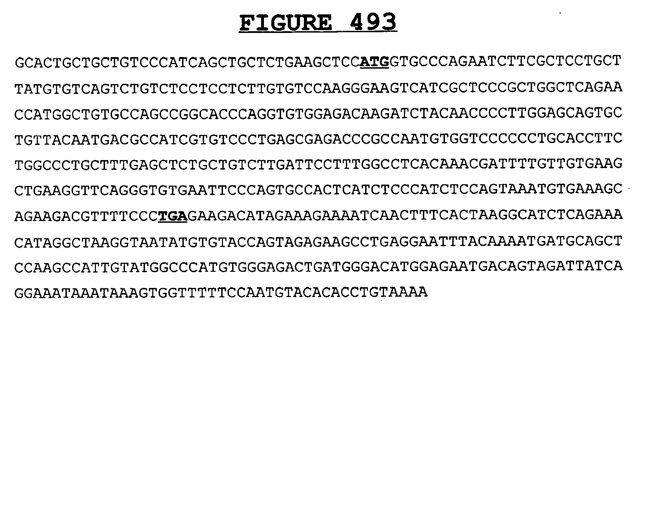

Figure 493 shows a nucleotide sequence (SEQ ID NO:493) of a native sequence PRO1275 cDNA, wherein SEQ ID NO:493 is a clone designated herein as "DNA64888-1542".

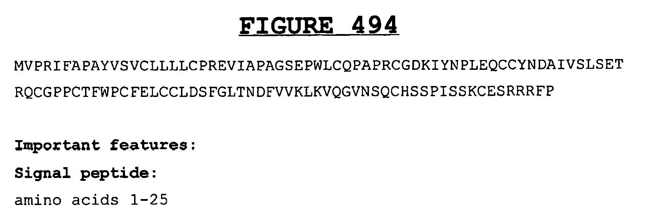

Figure 494 shows the amino acid sequence (SEQ ID NO:494) derived from the coding sequence of SEQ ID NO: 493 shown in Figure 493.

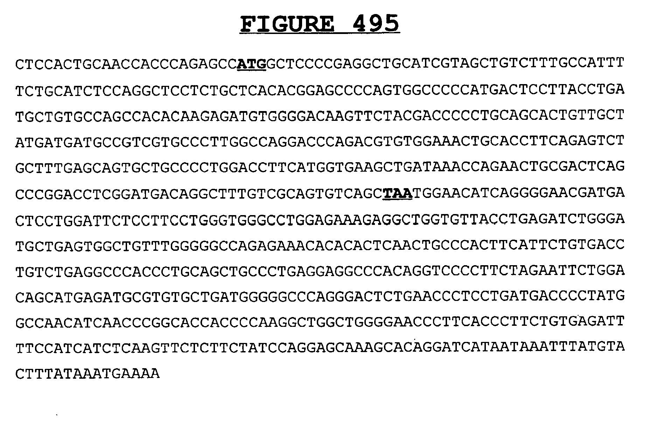

Figure 495 shows a nucleotide sequence (SEQ ID NO:495) of a native sequence PRO1274 cDNA, wherein SEQ ID NO:495 is a clone designated herein as "DNA64889-1541".

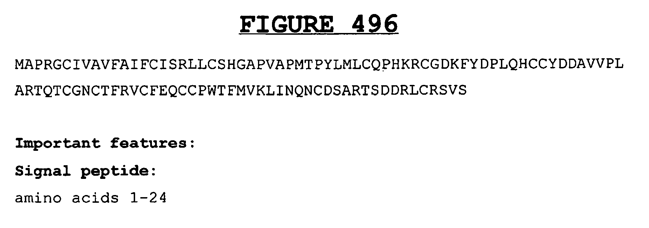

Figure 496 shows the amino acid sequence (SEQ ID NO:496) derived from the coding sequence of SEQ ID NO: 495 shown in Figure 495.

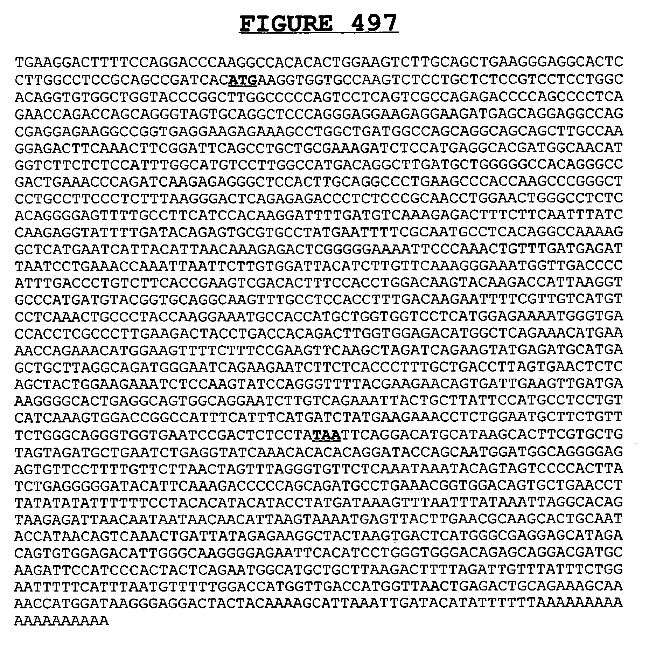

Figure 497 shows a nucleotide sequence (SEQ ID NO:497) of a native sequence PRO1358 cDNA, wherein SEQ ID NO:497 is a clone designated herein as "DNA64890-1612".

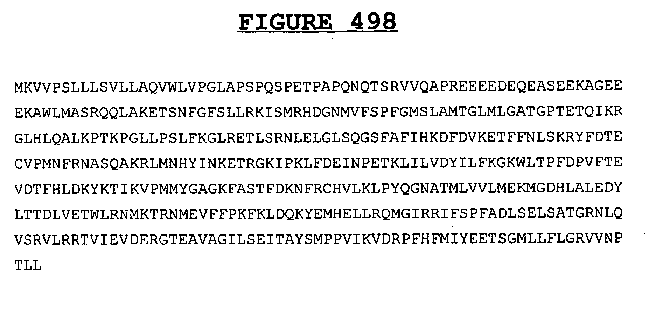

Figure 498 shows the amino acid sequence (SEQ ID NO:498) derived from the coding sequence of SEQ ID NO: 497 shown in Figure 497.

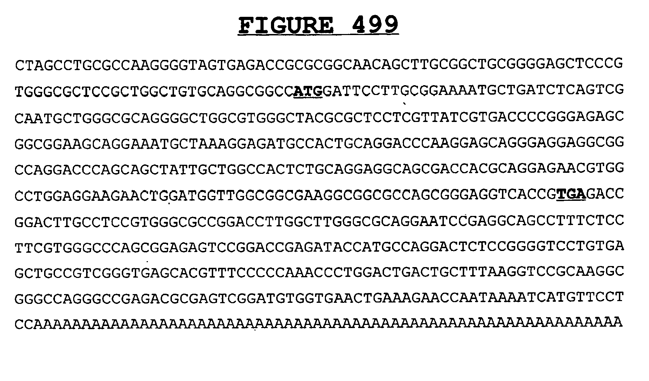

Figure 499 shows a nucleotide sequence (SEQ ID NO:499) of a native sequence PR01286 cDNA, wherein SEQ ID NO:499 is a clone designated herein as "DNA64903-1553".

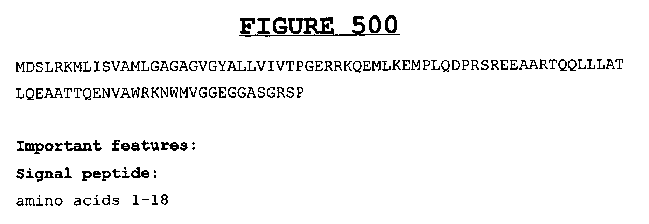

Figure 500 shows the amino acid sequence (SEQ ID NO:500) derived from the coding sequence of SEQ ID NO: 499 shown in Figure 499.

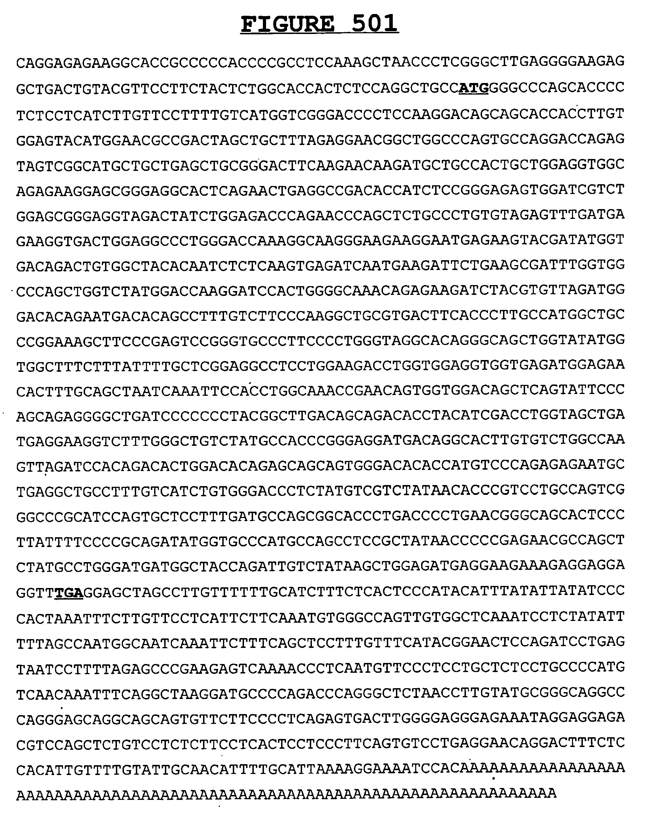

Figure 501 shows a nucleotide sequence (SEQ ID NO:501) of a native sequence PRO1294 cDNA, wherein SEQ ID NO:501 is a clone designated herein as "DNA64905-1558".

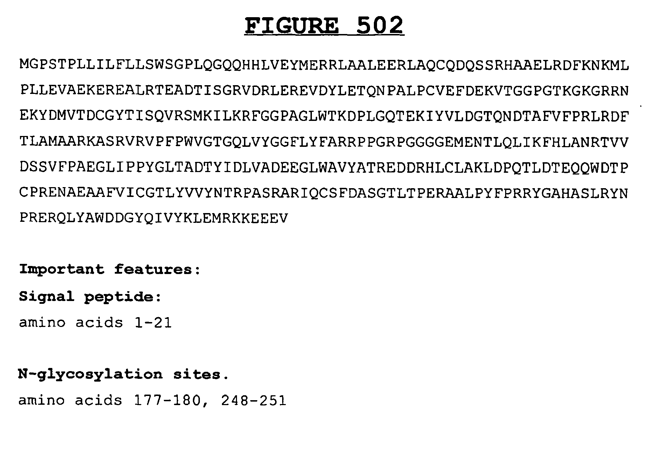

Figure 502 shows the amino acid sequence (SEQ ID NO:502) derived from the coding sequence of SEQ ID NO: 501 shown in Figure 501.

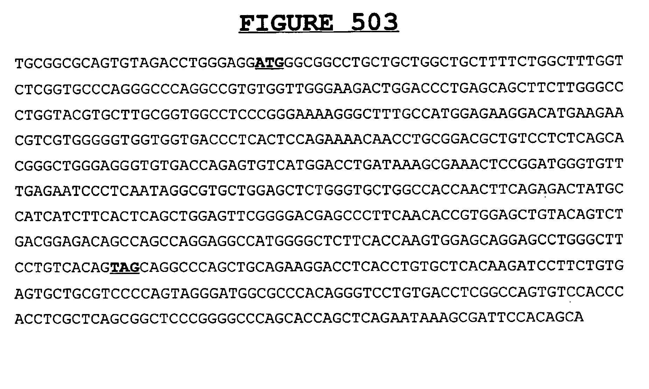

Figure 503 shows a nucleotide sequence (SEQ ID NO:503) of a native sequence PRO1273 cDNA, wherein SEQ ID NO:503 is a clone designated herein as "DNA65402-1540".

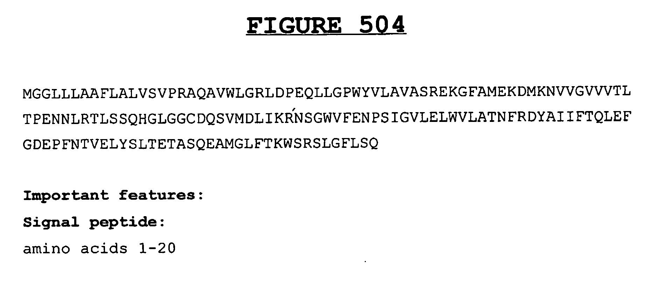

Figure 504 shows the amino acid sequence (SEQ ID NO:504) derived from the coding sequence of SEQ ID NO: 503 shown in Figure 503.

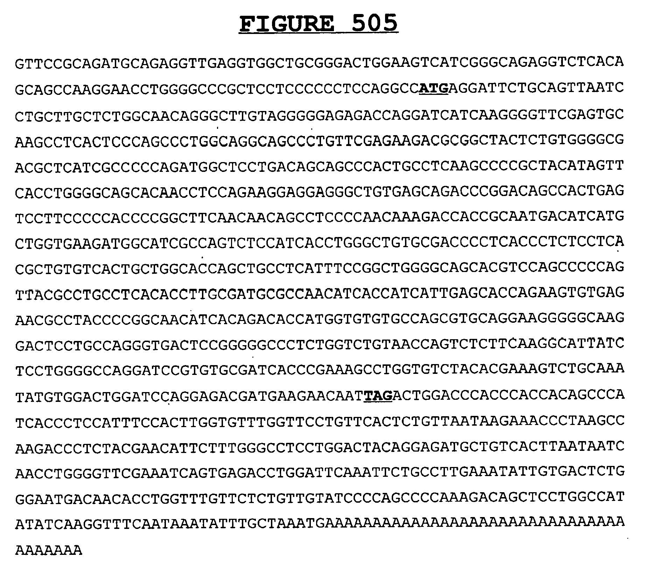

Figure 505 shows a nucleotide sequence (SEQ ID NO:505) of a native sequence PRO1279 cDNA, wherein SEQ ID NO:505 is a clone designated herein as "DNA65405-1547".

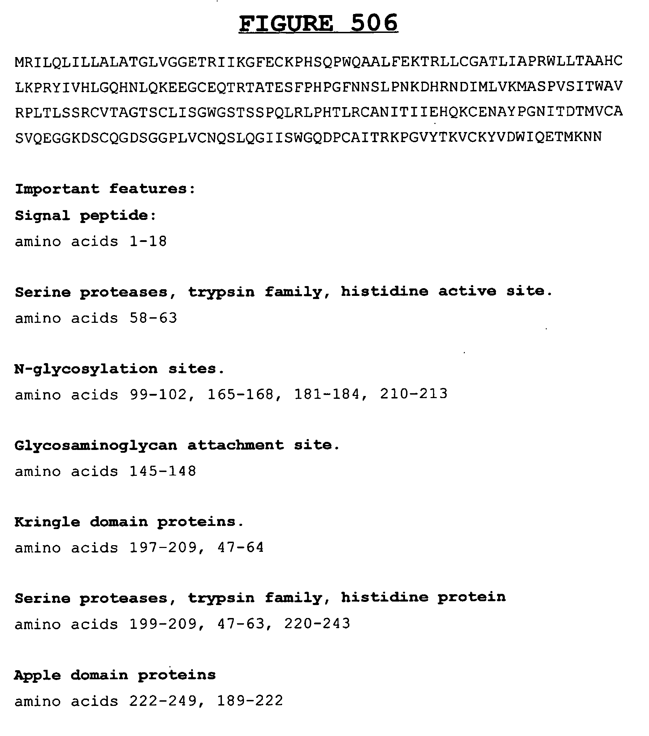

Figure 506 shows the amino acid sequence (SEQ ID NO:506) derived from the coding sequence of SEQ ID NO: 505 shown in Figure 505.

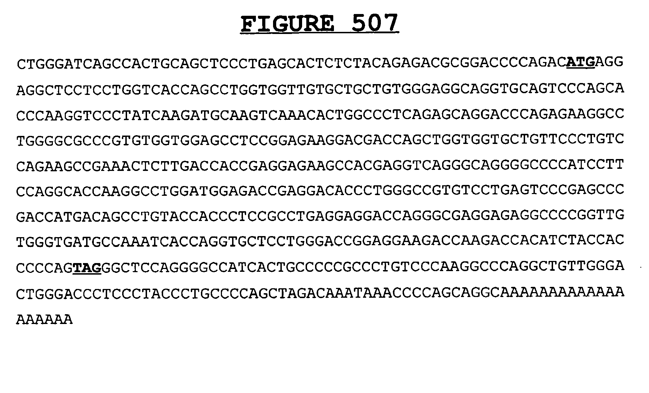

Figure 507 shows a nucleotide sequence (SEQ ID NO:507) of a native sequence PRO1195 cDNA, wherein SEQ ID NO:507 is a clone designated herein as "DNA65412-1523".

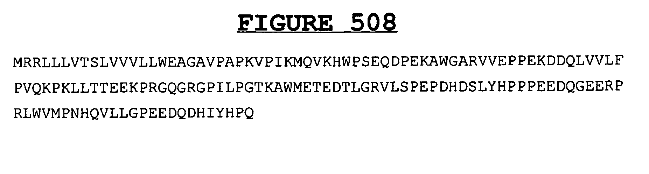

Figure 508 shows the amino acid sequence (SEQ ID NO:508) derived from the coding sequence of SEQ ID NO: 507 shown in Figure 507.

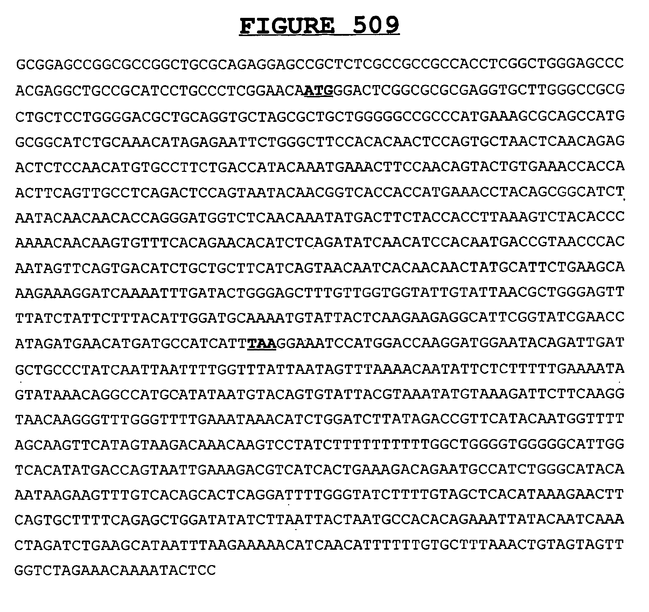

Figure 509 shows a nucleotide sequence (SEQ ID NO:509) of a native sequence PRO1271 cDNA, wherein SEQ ID NO:509 is a clone designated herein as "DNA66309-1538".

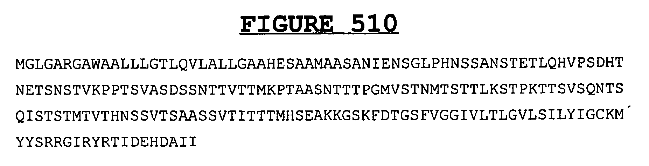

Figure 510 shows the amino acid sequence (SEQ ID NO:510) derived from the coding sequence of SEQ ID NO: 509 shown in Figure 509.

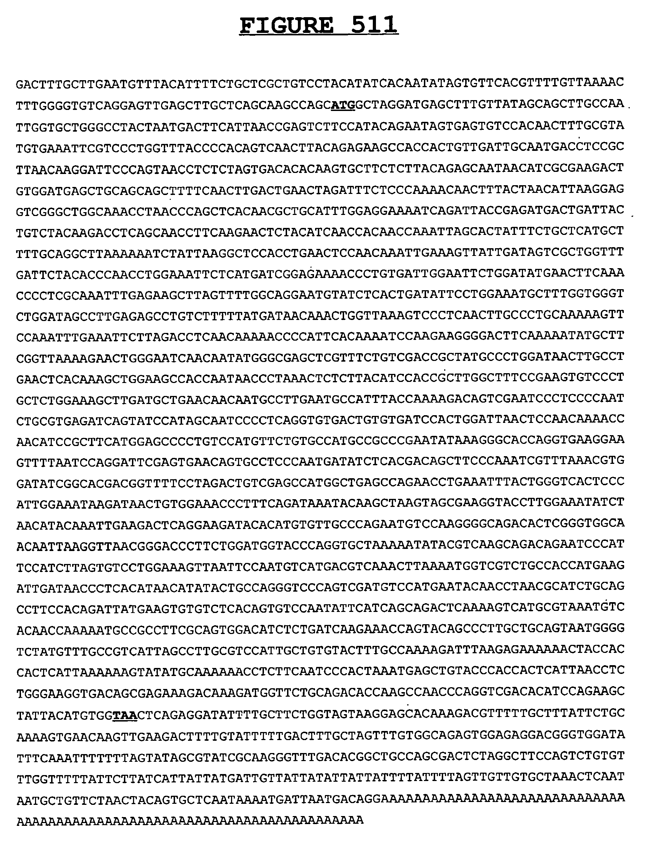

Figure 511 shows a nucleotide sequence (SEQ ID NO:511) of a native sequence PRO1338 cDNA, wherein SEQ ID NO:511 is a clone designated herein as "DNA66667-1596".

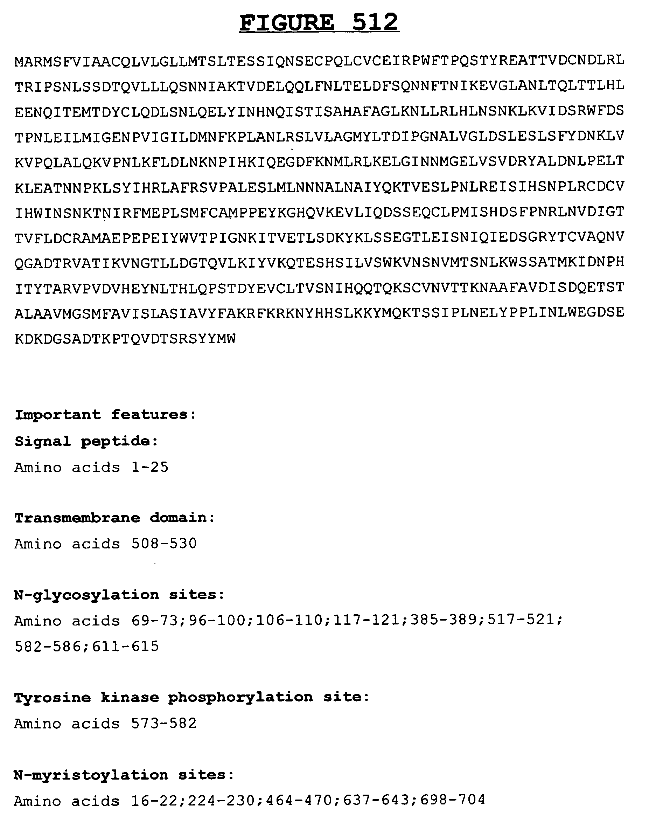

Figure 512 shows the amino acid sequence (SEQ ID NO:512) derived from the coding sequence of SEQ ID NO: 511 shown in Figure 511.

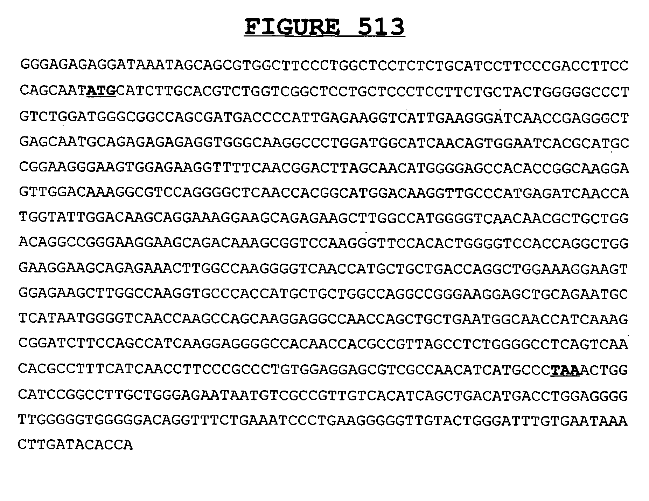

Figure 513 shows a nucleotide sequence (SEQ ID NO:513) of a native sequence PRO1343 cDNA, wherein SEQ ID NO:513 is a clone designated herein as "DNA66675-1587".

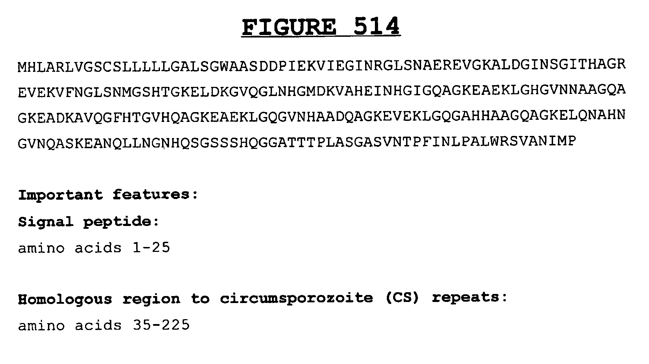

Figure 514 shows the amino acid sequence (SEQ ID NO:514) derived from the coding sequence of SEQ ID NO: 513 shown in Figure 513.

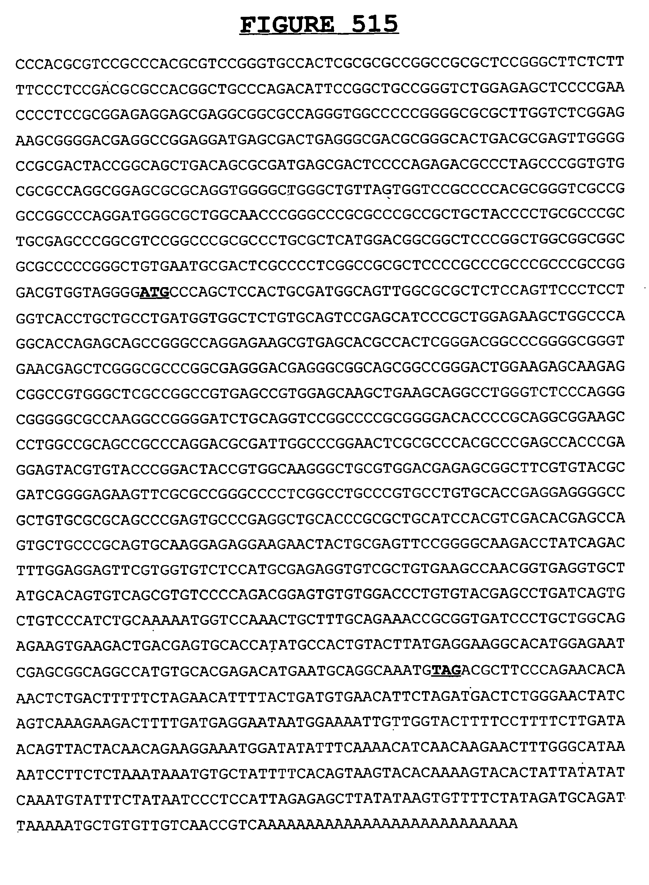

Figure 515 shows a nucleotide sequence (SEQ ID NO:515) of a native sequence PRO1434 cDNA, wherein SEQ ID NO:515 is a clone designated herein as "DNA68818-2536".

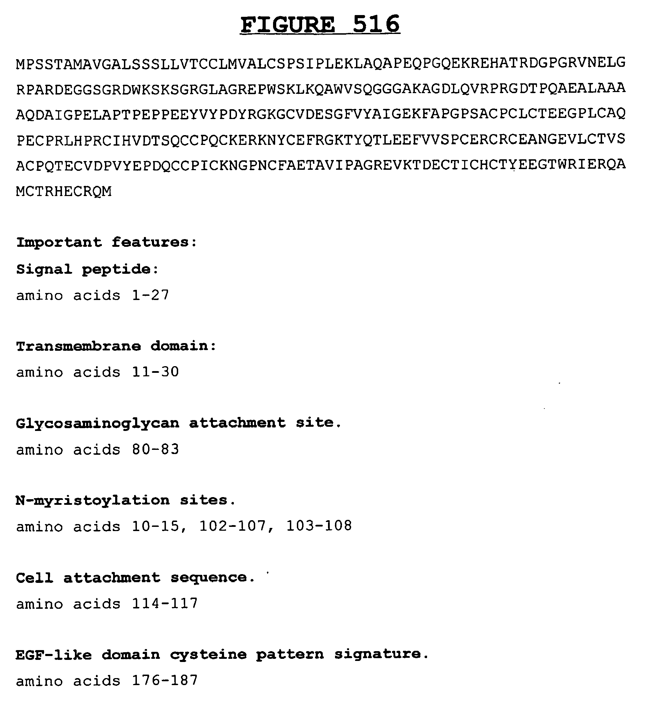

Figure 516 shows the amino acid sequence (SEQ ID NO:516) derived from the coding sequence of SEQ ID NO: 515 shown in Figure 515.

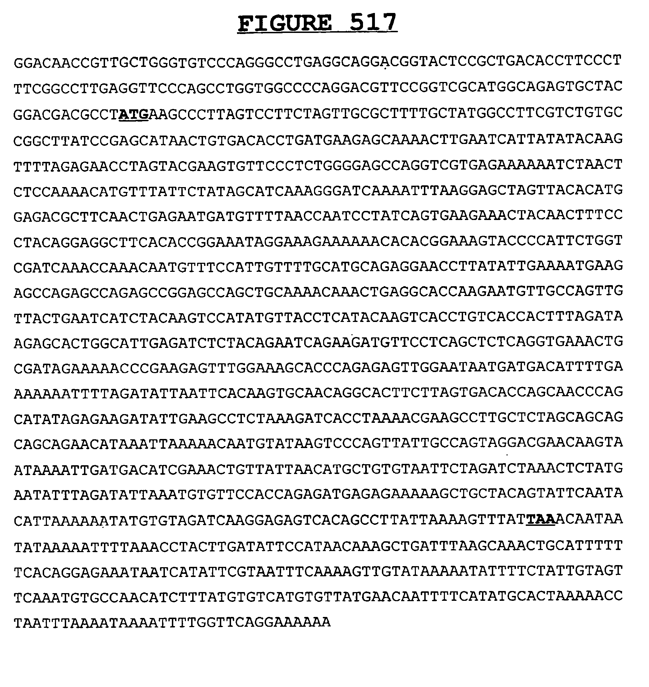

Figure 517 shows a nucleotide sequence (SEQ ID NO:517) of a native sequence PRO1418 cDNA, wherein SEQ ID NO:517 is a clone designated herein as "DNA68864-1629".

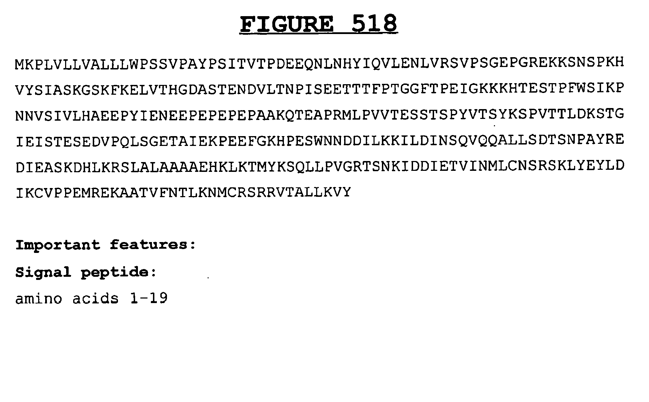

Figure 518 shows the amino acid sequence (SEQ ID NO:518) derived from the coding sequence of SEQ ID NO: 517 shown in Figure 517.

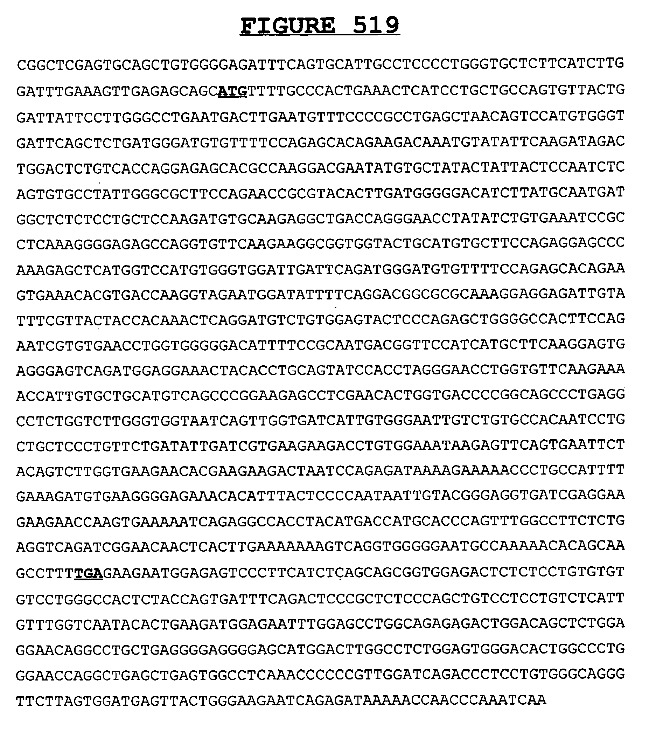

Figure 519 shows a nucleotide sequence (SEQ ID NO:519) of a native sequence PRO1387 cDNA, wherein SEQ ID NO:519 is a clone designated herein as "DNA68872-1620".

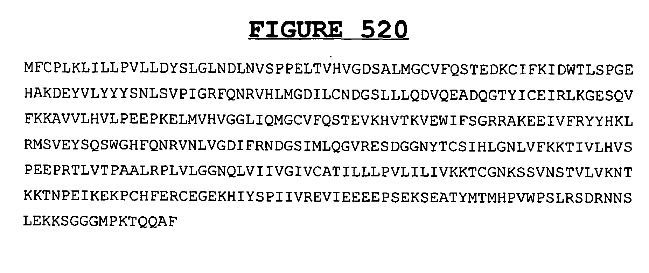

Figure 520 shows the amino acid sequence (SEQ ID NO:520) derived from the coding sequence of SEQ ID NO: 519 shown in Figure 519.

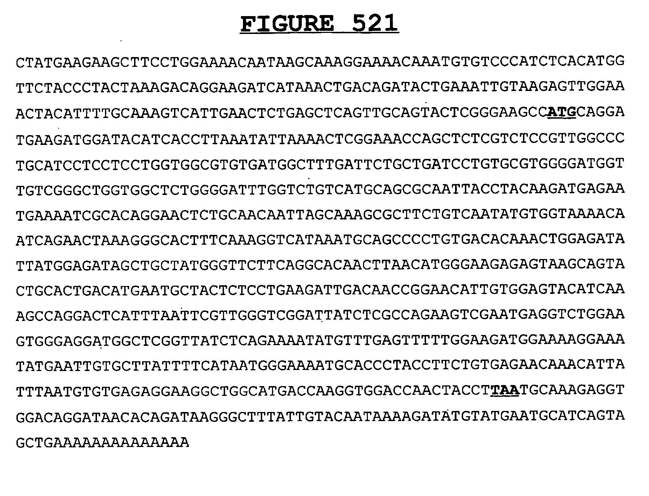

Figure 521 shows a nucleotide sequence (SEQ ID NO:521) of a native sequence PRO1384 cDNA, wherein SEQ ID NO:521 is a clone designated herein as "DNA71159-1617".

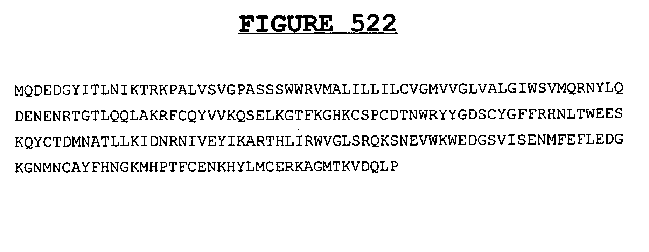

Figure 522 shows the amino acid sequence (SEQ ID NO:522) derived from the coding sequence of SEQ ID NO: 521 shown in Figure 521.

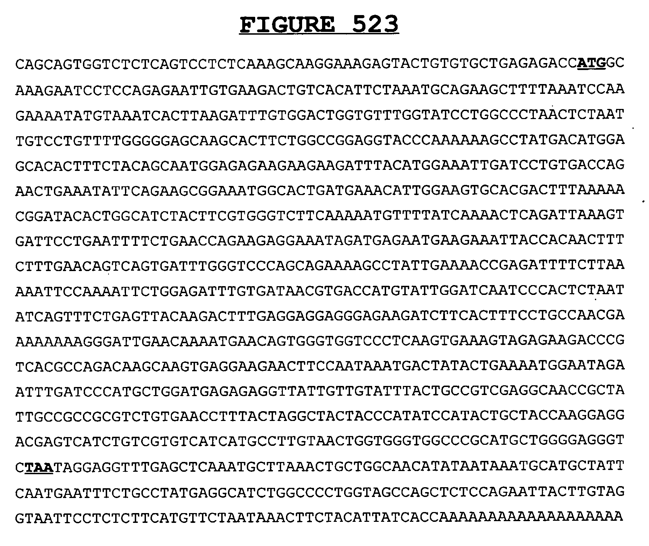

Figure 523 shows a nucleotide sequence (SEQ ID NO:523) of a native sequence PRO1565 cDNA, wherein SEQ ID NO:523 is a clone designated herein as "DNA73727-1673".

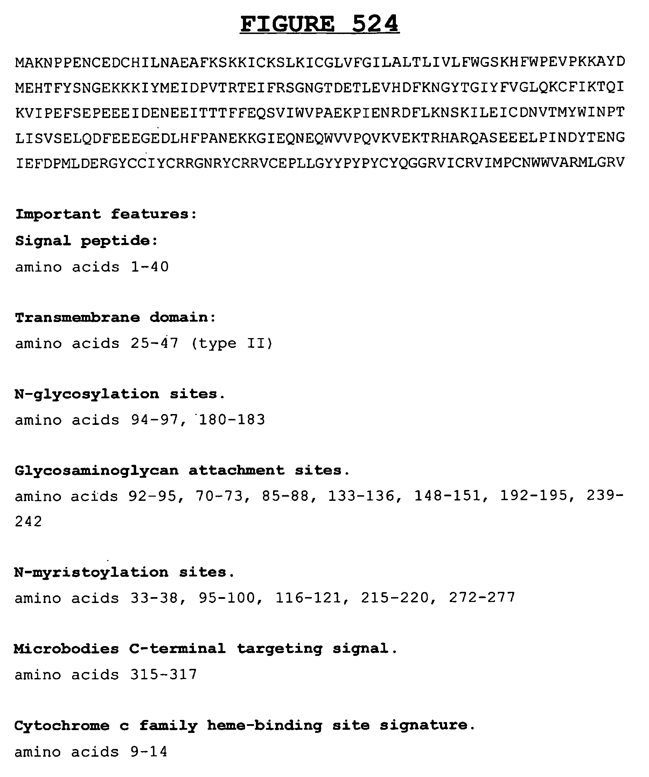

Figure 524 shows the amino acid sequence (SEQ ID NO:524) derived from the coding sequence of SEQ ID NO: 523 shown in Figure 523.

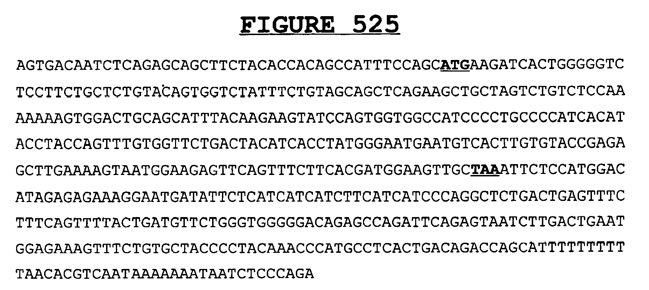

Figure 525 shows a nucleotide sequence (SEQ ID NO:525) of a native sequence PRO1474 cDNA, wherein SEQ ID NO:525 is a clone designated herein as "DNA73739-1645".

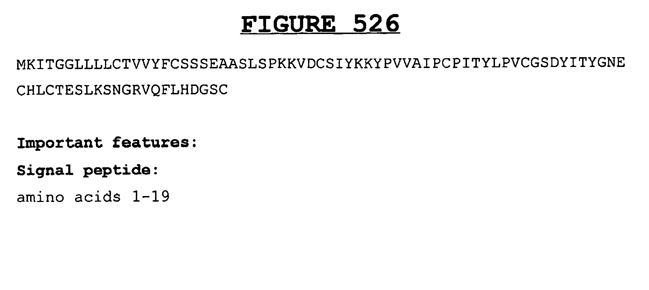

Figure 526 shows the amino acid sequence (SEQ ID NO:526) derived from the coding sequence of SEQ ID NO: 525 shown in Figure 525.

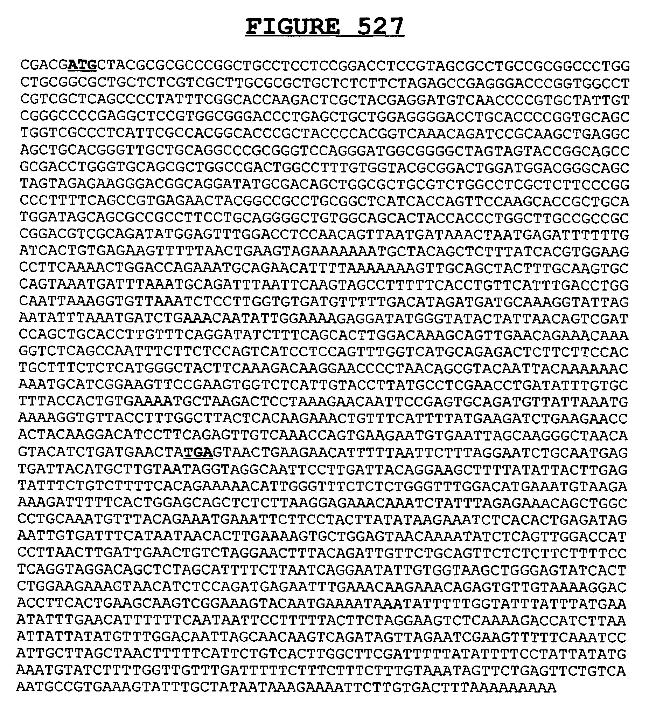

Figure 527 shows a nucleotide sequence (SEQ ID NO:527) of a native sequence PRO1917 cDNA, wherein SEQ ID NO:527 is a clone designated herein as "DNA76400-2528".

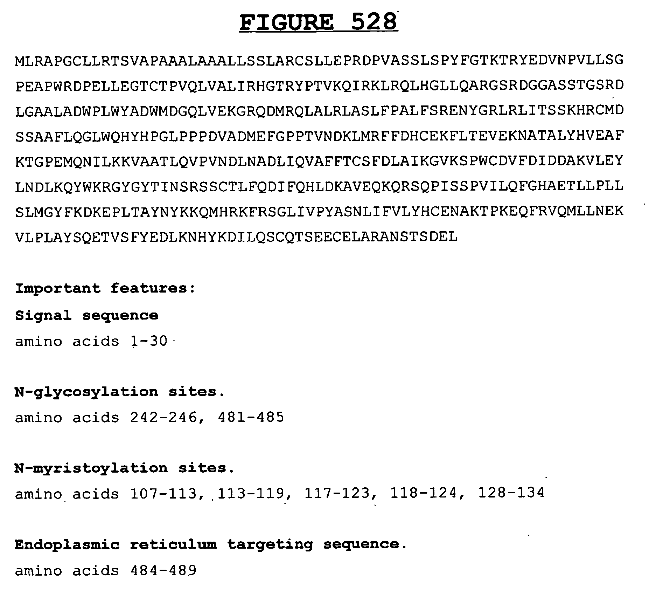

Figure 528 shows the amino acid sequence (SEQ ID NO:528) derived from the coding sequence of SEQ ID NO: 527 shown in Figure 527.

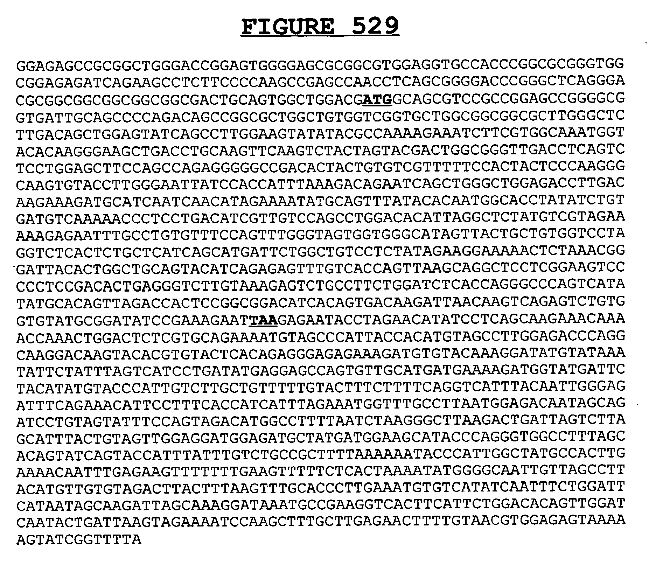

Figure 529 shows a nucleotide sequence (SEQ ID NO:529) of a native sequence PRO1787 cDNA, wherein SEQ ID NO:529 is a clone designated herein as "DNA76510-2504".

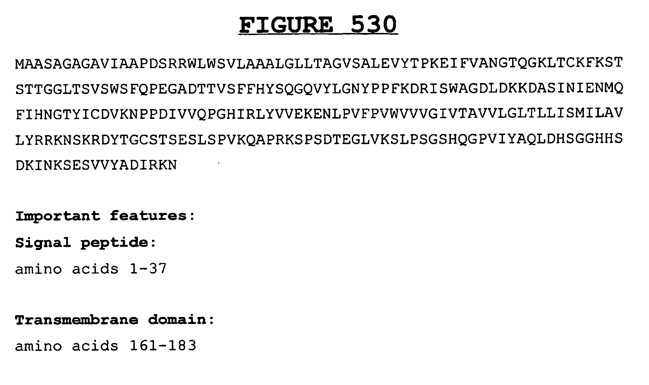

Figure 530 shows the amino acid sequence (SEQ ID NO:530) derived from the coding sequence of SEQ ID NO: 529 shown in Figure 529.

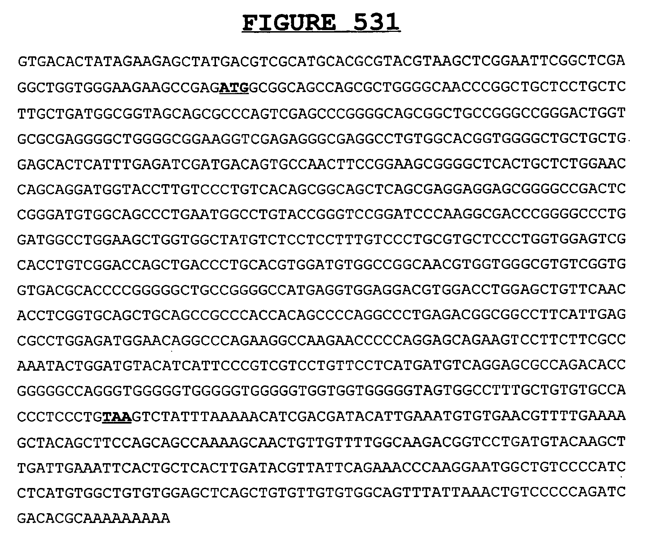

Figure 531 shows a nucleotide sequence (SEQ ID NO:531) of a native sequence PR01556 cDNA, wherein SEQ ID NO:531 is a clone designated herein as "DNA76529-1666".

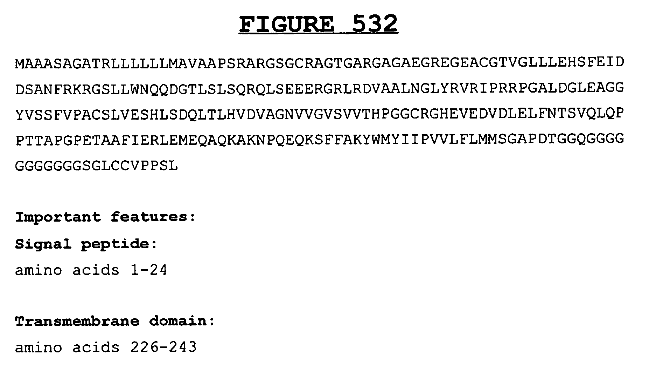

Figure 532 shows the amino acid sequence (SEQ ID NO:532) derived from the coding sequence of SEQ ID NO: 531 shown in Figure 531.

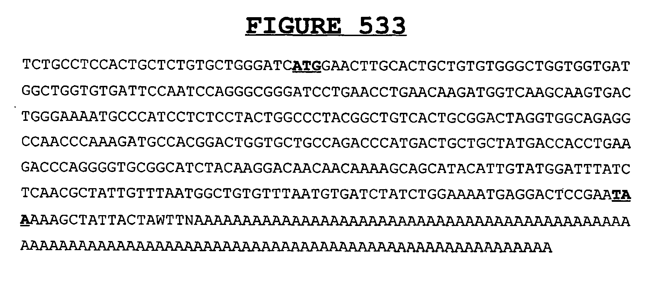

Figure 533 shows a nucleotide sequence (SEQ ID NO:533) of a native sequence PRO1561 cDNA, wherein SEQ ID NO:533 is a clone designated herein as "DNA76538-1670".

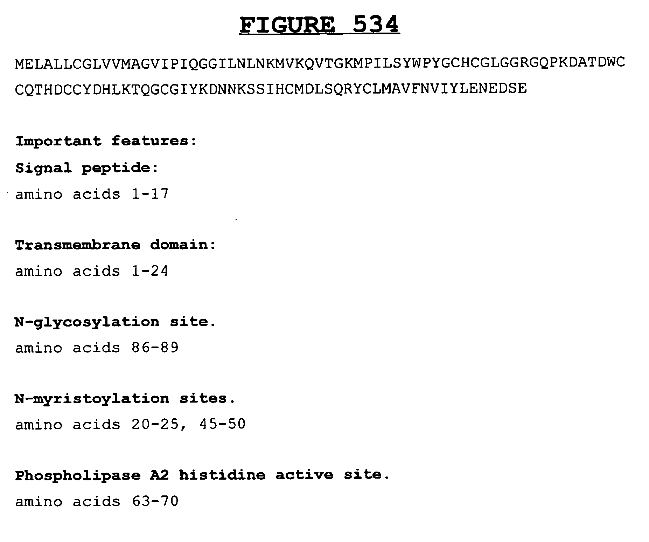

Figure 534 shows the amino acid sequence (SEQ ID NO:534) derived from the coding sequence of SEQ ID NO: 533 shown in Figure 533.

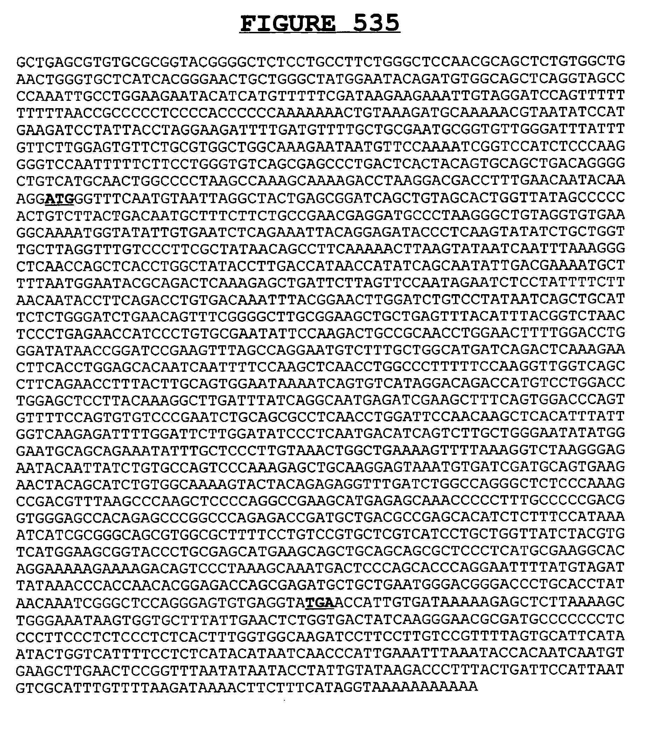

Figure 535 shows a nucleotide sequence (SEQ ID NO:535) of a native sequence PRO1693 cDNA, wherein SEQ ID NO:535 is a clone designated herein as "DNA77301-1708".

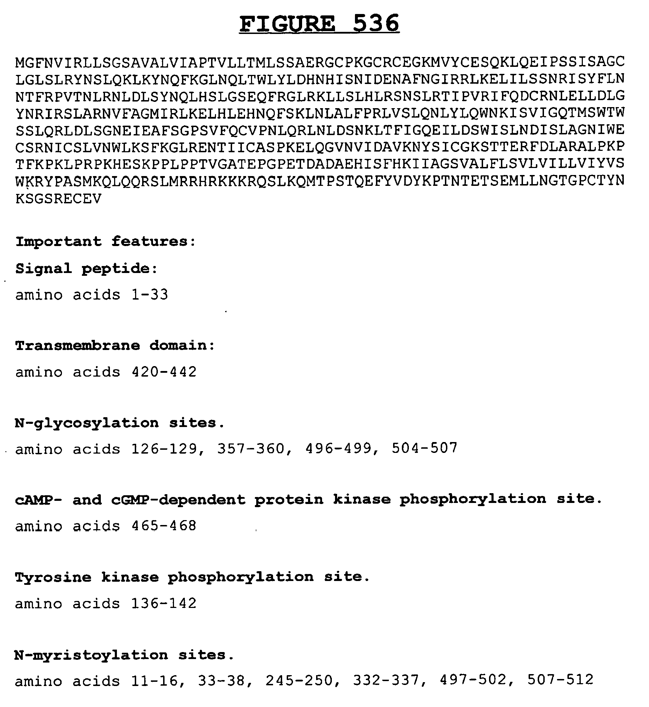

Figure 536 shows the amino acid sequence (SEQ ID NO:536) derived from the coding sequence of SEQ ID NO: 535 shown in Figure 535.

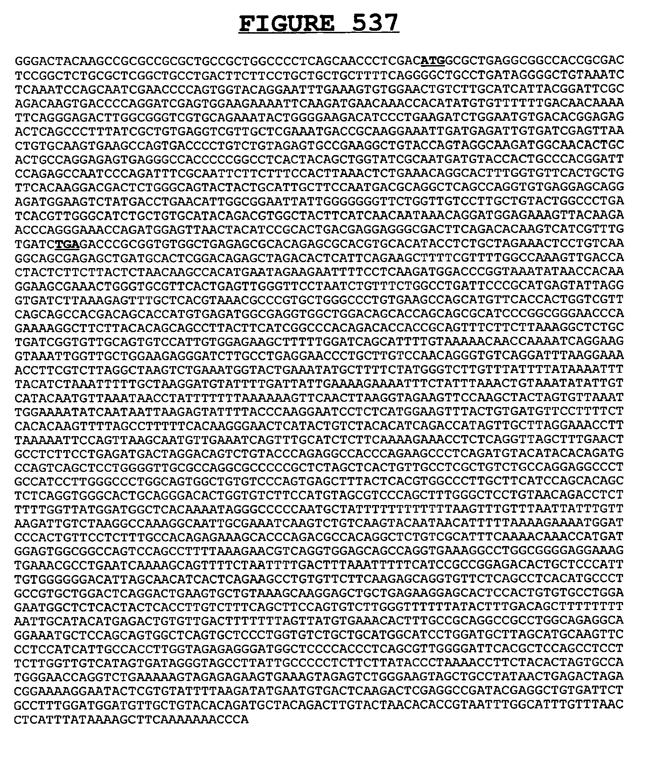

Figure 537 shows a nucleotide sequence (SEQ ID NO:537) of a native sequence PRO1868 cDNA, wherein SEQ ID NO:537 is a clone designated herein as "DNA77624-2515".

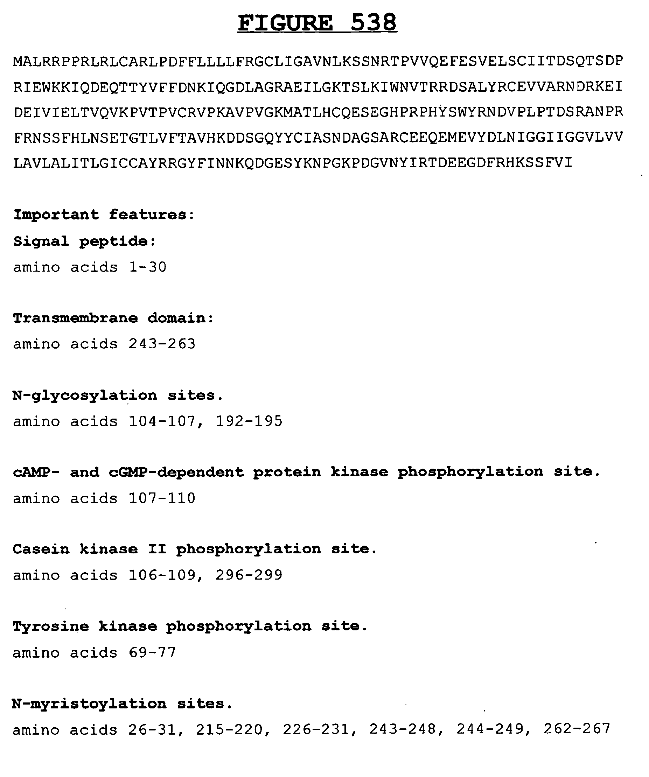

Figure 538 shows the amino acid sequence (SEQ ID NO:538) derived from the coding sequence of SEQ ID NO: 537 shown in Figure 537.

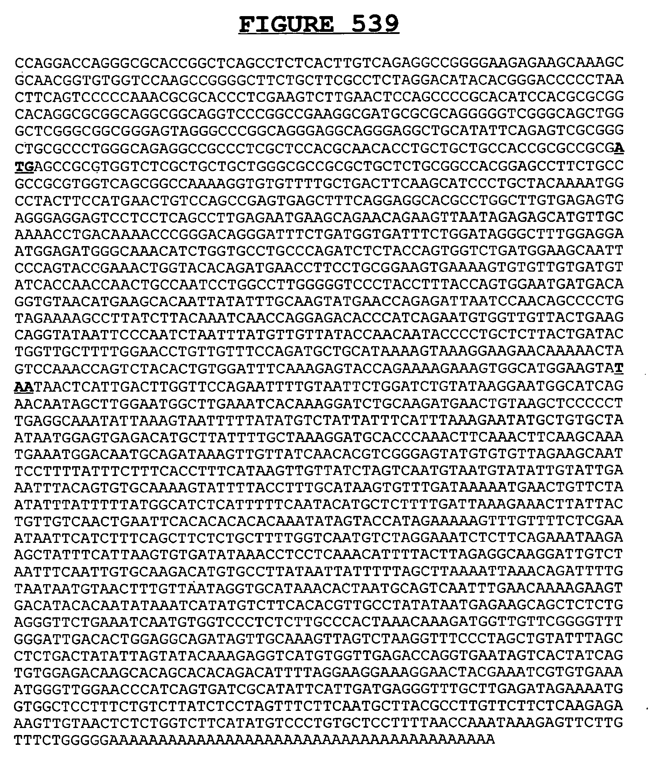

Figure 539 shows a nucleotide sequence (SEQ ID NO:539) of a native sequence PRO1890 cDNA, wherein SEQ ID NO:539 is a clone designated herein as "DNA79230-2525".

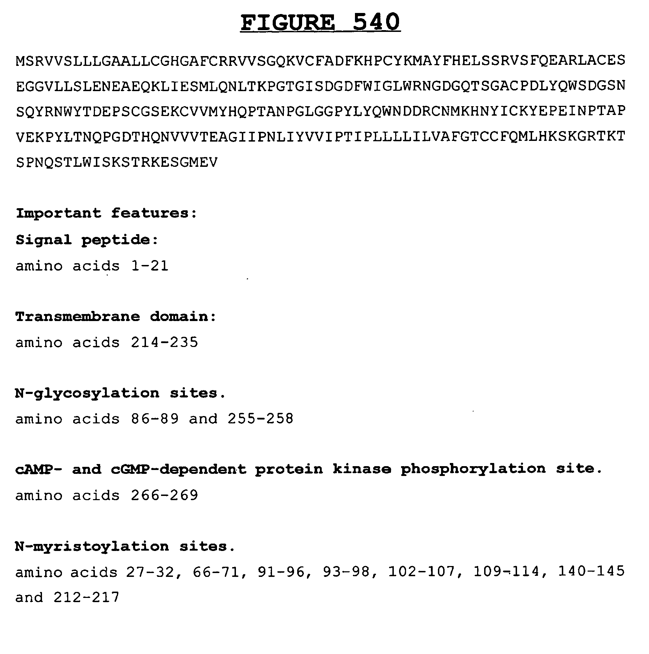

Figure 540 shows the amino acid sequence (SEQ ID NO:540) derived from the coding sequence of SEQ ID NO: 539 shown in Figure 539.

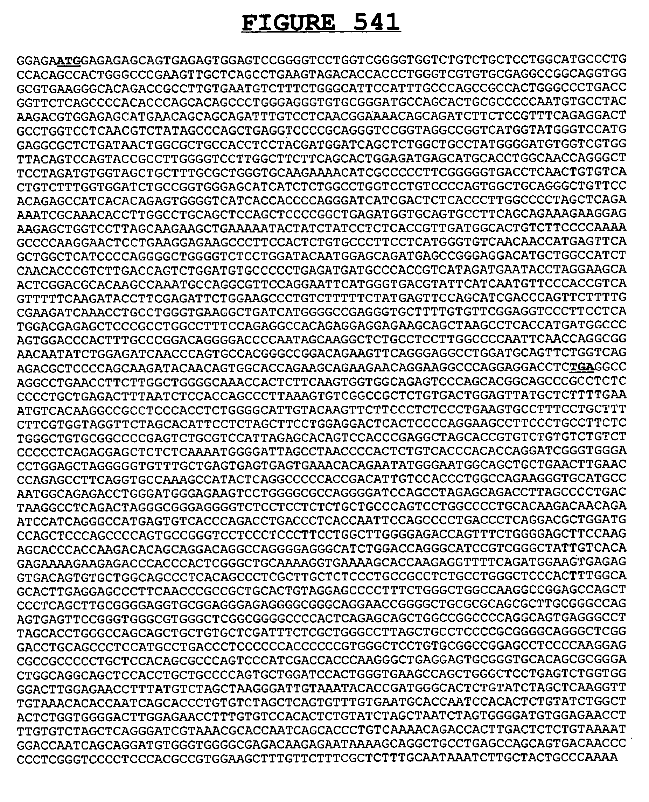

Figure 541 shows a nucleotide sequence (SEQ ID NO:541) of a native sequence PRO1887 cDNA, wherein SEQ ID NO:541 is a clone designated herein as "DNA79862-2522".

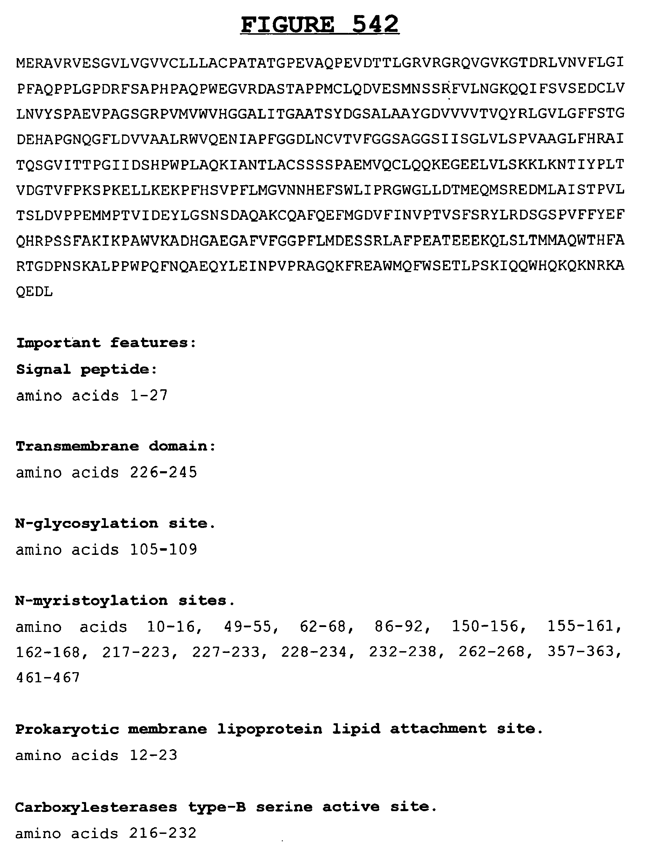

Figure 542 shows the amino acid sequence (SEQ ID NO:542) derived from the coding sequence of SEQ ID NO: 541 shown in Figure 541.

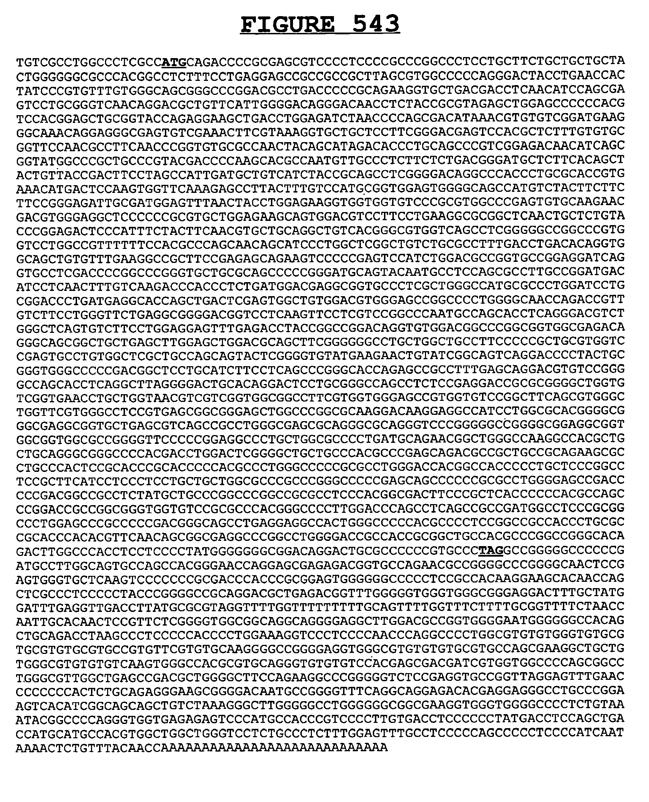

Figure 543 shows a nucleotide sequence (SEQ ID NO:543) of a native sequence PR04353 cDNA, wherein SEQ ID NO:543 is a clone designated herein as "DNA80145-2594".

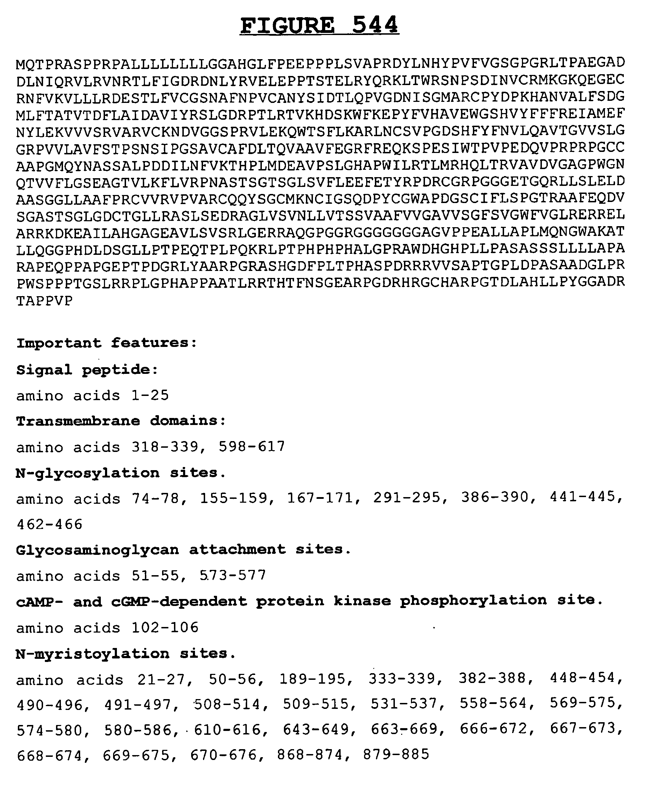

Figure 544 shows the amino acid sequence (SEQ ID NO:544) derived from the coding sequence of SEQ ID NO: 543 shown in Figure 543.

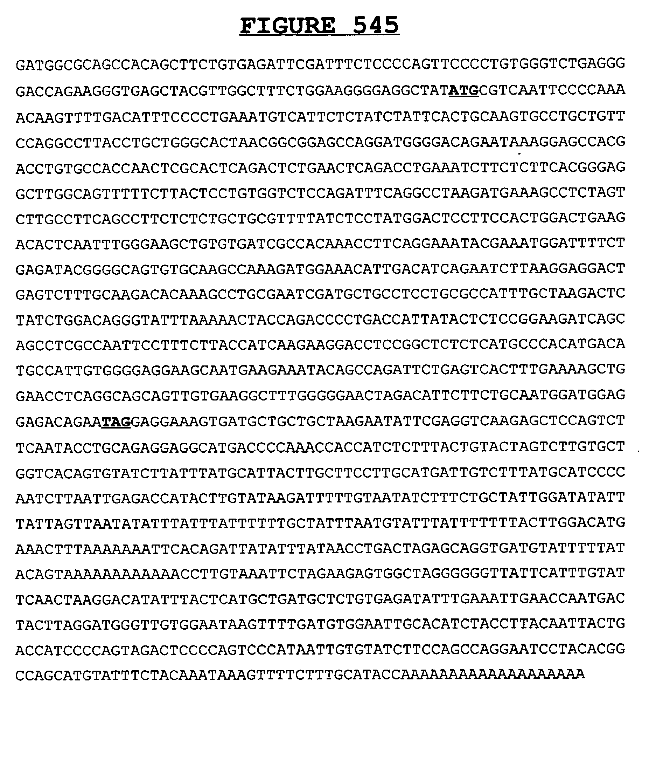

Figure 545 shows a nucleotide sequence (SEQ ID NO:545) of a native sequence PRO1801 cDNA, wherein SEQ ID NO:545 is a clone designated herein as "DNA83500-2506".

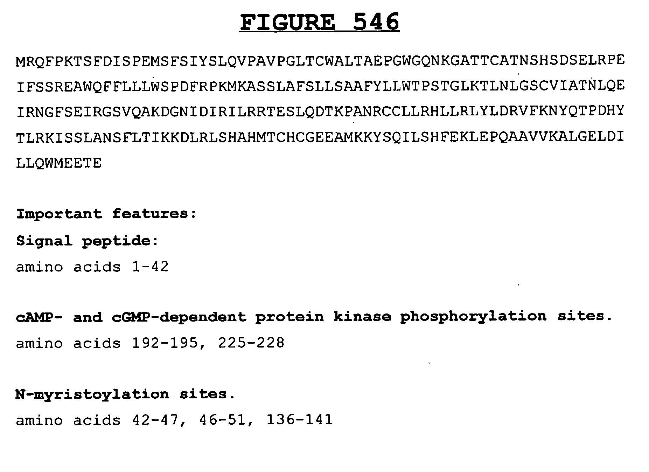

Figure 546 shows the amino acid sequence (SEQ ID NO:546) derived from the coding sequence of SEQ ID NO: 545 shown in Figure 545.

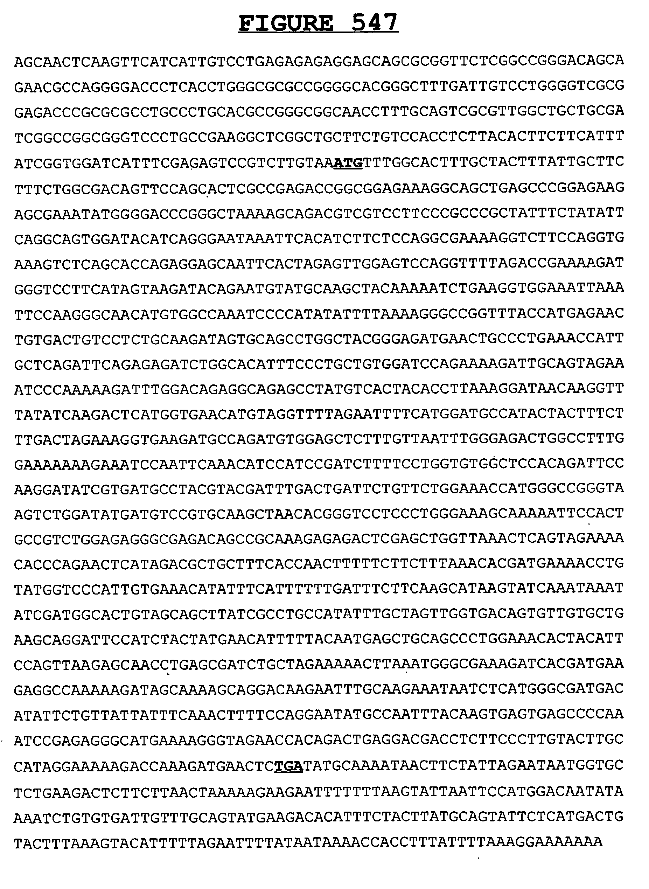

Figure 547 shows a nucleotide sequence (SEQ ID NO:547) of a native sequence PR04357 cDNA, wherein SEQ ID NO:547 is a clone designated herein as "DNA84917-2597".

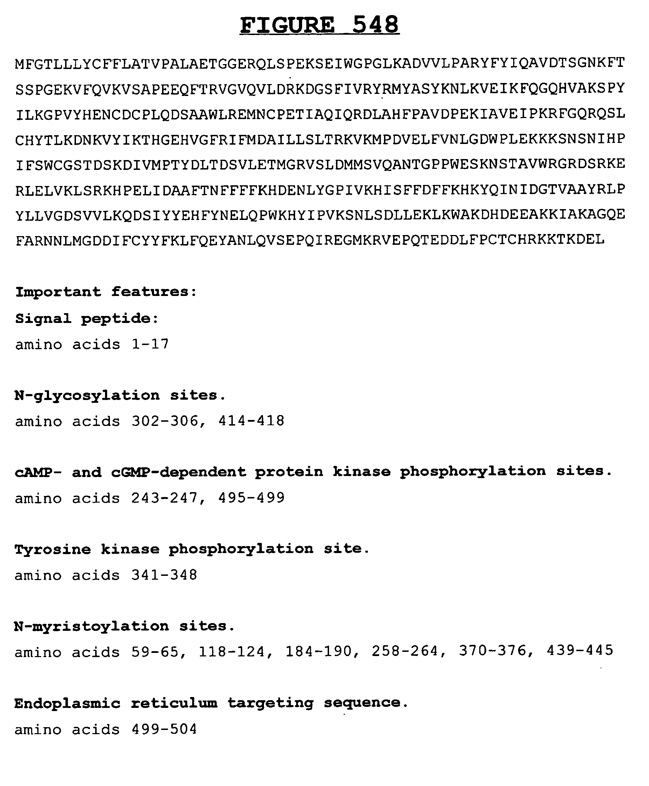

Figure 548 shows the amino acid sequence (SEQ ID NO:548) derived from the coding sequence of SEQ ID NO: 547 shown in Figure 547.

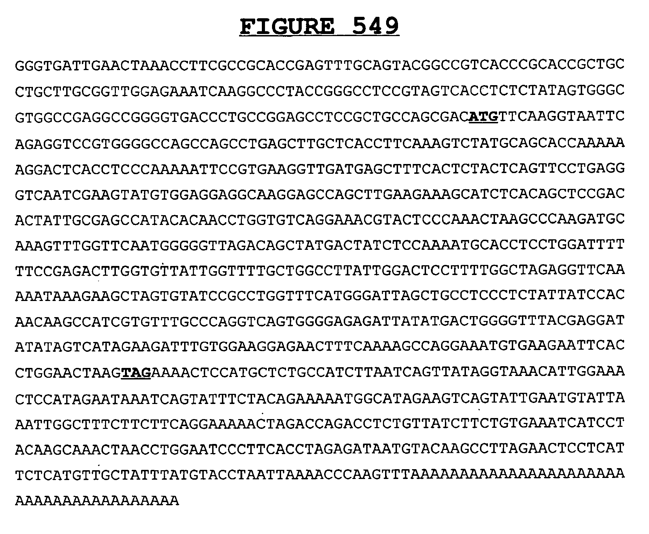

Figure 549 shows a nucleotide sequence (SEQ ID NO:549) of a native sequence PRO4302 cDNA, wherein SEQ ID NO:549 is a clone designated herein as "DNA92218-2554".

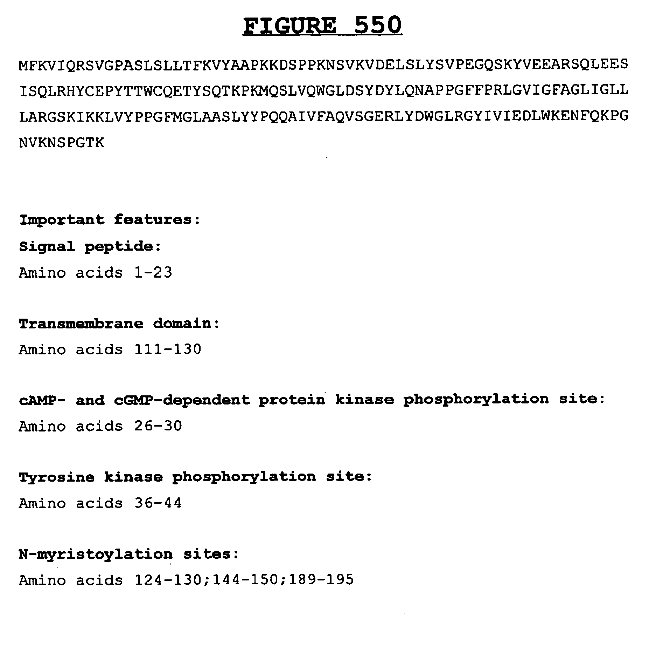

Figure 550 shows the amino acid sequence (SEQ ID NO:550) derived from the coding sequence of SEQ ID NO: 549 shown in Figure 549.

## DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1 % of such transmembrane and/or cytoplasmic domains and preferably, will have less than 0.5 % of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81 % amino acid sequence identity, alternatively at least about 82% amino acid sequence identity, alternatively at least about 83% amino acid sequence identity, alternatively at least about 84 % amino acid sequence identity, alternatively at least about 85 % amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93 % amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99 % amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO" , wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

" PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81 % nucleic acid sequence identity, alternatively at least about 82% nucleic acid sequence identity, alternatively at least about 83 % nucleic acid sequence identity, alternatively at least about 84 % nucleic acid sequence identity, alternatively at least about 85% nucleic acid sequence identity, alternatively at least about 86 % nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98 % nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 μg/ml), 0.1 % SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM. "Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. "Single-chain Fv" or "sFv" antibody fragments-comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_1$) in the same polypeptide chain ($V_H$-$V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word " label " when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "effective amount" of a polypeptide disclosed herein or an agonist or antagonist thereof is an amount sufficient to carry out a specifically stated purpose. An "effective amount" may be determined empirically and in a routine manner, in relation to the stated purpose.

## Table 1

```
/*
 *
 * C-C increased from 12 to 15
 * Z is average of EQ
 * B is average of ND
 * match with stop is _M; stop-stop = 0; J (joker) match = 0
 */
#define   _M      -8      /* value of a match with a stop */

int       _day[26][26] = {
/*        A  B  C  D  E  F  G  H  I  J  K  L  M  N  O  P  Q  R  S  T  U  V  W  X  Y  Z */
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M, 0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

## Table 1 (cont')

```
/*
 */
#include <stdio.h>
#include <ctype.h>

#define MAXJMP      16      /* max jumps in a diag */
#define MAXGAP      24      /* don't continue to penalize gaps larger than this */
#define JMPS        1024    /* max jmps in an path */
#define MX          4       /* save if there's at least MX-1 bases since last jmp */

#define DMAT        3       /* value of matching bases */
#define DMIS        0       /* penalty for mismatched bases */
#define DINS0       8       /* penalty for a gap */
#define DINS1       1       /* penalty per base */
#define PINS0       8       /* penalty for a gap */
#define PINS1       4       /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;            /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static  _dbval[26] = {
        1,14,2,13,0,0,4,11,0,0,12,0,3,15,0,0,0,5,6,8,8,7,9,0,10,0
};

static  _pbval[26] = {
        1, 2|(1<<('D'-'A'))|(1<<('N'-'A')), 4, 8, 16, 32, 64,
        128, 256, 0xFFFFFFF, 1<<10, 1<<11, 1<<12, 1<<13, 1<<14,
        1<<15, 1<<16, 1<<17, 1<<18, 1<<19, 1<<20, 1<<21, 1<<22,
        1<<23, 1<<24, 1<<25|(1<<('E'-'A'))|(1<<('Q'-'A'))
};

main(ac, av)                                                          main
        int     ac;
        char    *av[];
{
        prog = av[0];
        if (ac != 3) {
                fprintf(stderr,"usage: %s file1 file2\n", prog);
                fprintf(stderr,"where file1 and file2 are two dna or two protein sequences.\n");
                fprintf(stderr,"The sequences can be in upper- or lower-case\n");
                fprintf(stderr,"Any lines beginning with ';' or '<' are ignored\n");
                fprintf(stderr,"Output is in the file \"align.out\"\n");
                exit(1);
        }
        namex[0] = av[1];
        namex[1] = av[2];
        seqx[0] = getseq(namex[0], &len0);
        seqx[1] = getseq(namex[1], &len1);
        xbm = (dna)? _dbval : _pbval;

        endgaps = 0;                    /* 1 to penalize endgaps */
        ofile = "align.out";            /* output file */

        nw();           /* fill in the matrix, get the possible jmps */
        readjmps();     /* get the actual jmps */
        print();        /* print stats, alignment */

        cleanup(0);     /* unlink any tmp files */
}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                    nw
{
        char            *px, *py;               /* seqs and ptrs */
        int             *ndely, *dely;          /* keep track of dely */
        int             ndelx, delx;            /* keep track of delx */
        int             *tmp;                   /* for swapping row0, row1 */
        int             mis;                    /* score for each type */
        int             ins0, ins1;             /* insertion penalties */
        register        id;                     /* diagonal index */
        register        ij;                     /* jmp index */
        register        *col0, *col1;           /* score for curr, last row */
        register        xx, yy;                 /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;        /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

## Table 1 (cont')

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

**Table 1 (cont')**

...nw

```
                id = xx - yy + len1 - 1;
                if (mis >= delx && mis >= dely[yy])
                        col1[yy] = mis;
                else if (delx >= dely[yy]) {
                        col1[yy] = delx;
                        ij = dx[id].ijmp;
                        if (dx[id].jp.n[0] && (!dna || (ndelx >= MAXJMP
                        && xx > dx[id].jp.x[ij]+MX) || mis > dx[id].score+DINS0)) {
                                dx[id].ijmp++;
                                if (++ij >= MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = ndelx;
                        dx[id].jp.x[ij] = xx;
                        dx[id].score = delx;
                }
                else {
                        col1[yy] = dely[yy];
                        ij = dx[id].ijmp;
if (dx[id].jp.n[0] && (!dna || (ndely[yy] >= MAXJMP
                        && xx > dx[id].jp.x[ij]+MX) || mis > dx[id].score+DINS0)) {
                                dx[id].ijmp++;
                                if (++ij >= MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = -ndely[yy];
                        dx[id].jp.x[ij] = xx;
                        dx[id].score = dely[yy];
                }
                if (xx == len0 && yy < len1) {
                        /* last col
                         */
                        if (endgaps)
                                col1[yy] -= ins0+ins1*(len1-yy);
                        if (col1[yy] > smax) {
                                smax = col1[yy];
                                dmax = id;
                        }
                }
        }
        if (endgaps && xx < len0)
                col1[yy-1] -= ins0+ins1*(len0-xx);
        if (col1[yy-1] > smax) {
                smax = col1[yy-1];
                dmax = id;
        }
        tmp = col0; col0 = col1; col1 = tmp;
}
(void) free((char *)ndely);
(void) free((char *)dely);
(void) free((char *)col0);
(void) free((char *)col1);                    }
```

**Table 1 (cont')**

print

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256     /* maximum output line */
#define P_SPC    3       /* space between name or num and seq */

extern   _day[26][26];
int      olen;           /* set output line length */
FILE     *fx;            /* output file */

print()
{
        int     lx, ly, firstgap, lastgap;     /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {      /* leading gap in x */
                pp[0].spc = firstgap = len1 - dmax - 1;
                ly -= pp[0].spc;
        }
        else if (dmax > len1 - 1) {  /* leading gap in y */
                pp[1].spc = firstgap = dmax - (len1 - 1);
                lx -= pp[1].spc;
        }
        if (dmax0 < len0 - 1) {     /* trailing gap in x */
                lastgap = len0 - dmax0 -1;
                lx -= lastgap;
        }
        else if (dmax0 > len0 - 1) { /* trailing gap in y */
                lastgap = dmax0 - (len0 - 1);
                ly -= lastgap;
        }
        getmat(lx, ly, firstgap, lastgap);
        pr_align();
}
```

## Table 1 (cont')

getmat

```
/*
 * trace back the best path, count matches
 */
static
getmat(lx, ly, firstgap, lastgap)
        int     lx, ly;                 /* "core" (minus endgaps) */
        int     firstgap, lastgap;      /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

## Table 1 (cont')

...getmat

```
        fprintf(fx, "<gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx,"%s", outx);

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per base)\n",
                smax, DMAT, DMIS, DINS0, DINS1);
        else
                fprintf(fx,
                "\n<score: %d (Dayhoff PAM 250 matrix, gap penalty = %d + %d per residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}

static          nm;             /* matches in core -- for checking */
static          lmax;           /* lengths of stripped file names */
static          ij[2];          /* jmp index for a path */
static          nc[2];          /* number at start of current line */
static          ni[2];          /* current elem number -- for gapping */
static          siz[2];
static char     *ps[2];         /* ptr to current element */
static char     *po[2];         /* ptr to next output char slot */
static char     out[2][P_LINE]; /* output line */
static char     star[P_LINE];   /* set by stars() */

/*
 * print alignment of described in struct path pp[]
 */
static
pr_align()
{
        int             nn;     /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];                 }
```

pr_align

## Table 1 (cont')

...pr_align

```
        for (nn = nm = 0, more = 1; more; ) {
                for (i = more = 0; i < 2; i++) {
                        /*
                         * do we have more of this sequence?
                         */
                        if (!*ps[i])
                                continue;

                        more++;

                        if (pp[i].spc) {        /* leading space */
                                *po[i]++ = ' ';
                                pp[i].spc--;
                        }
                        else if (siz[i]) {      /* in a gap */
                                *po[i]++ = '-';
                                siz[i]--;
                        }
                        else {                  /* we're putting a seq element
                                                 */
                                *po[i] = *ps[i];
                                if (islower(*ps[i]))
                                        *ps[i] = toupper(*ps[i]);
                                po[i]++;
                                ps[i]++;

                                /*
                                 * are we at next gap for this seq?
                                 */
                                if (ni[i] == pp[i].x[ij[i]]) {
                                        /*
                                         * we need to merge all gaps
                                         * at this location
                                         */
                                        siz[i] = pp[i].n[ij[i]++];
                                        while (ni[i] == pp[i].x[ij[i]])
                                                siz[i] += pp[i].n[ij[i]++];
                                }
                                ni[i]++;
                        }
                }
                if (++nn == olen || !more && nn) {
                        dumpblock();
                        for (i = 0; i < 2; i++)
                                po[i] = out[i];
                        nn = 0;
                }
        }
}

/*
 * dump a block of lines, including numbers, stars: pr_align()
 */
static
dumpblock()
{
        register i;

        for (i = 0; i < 2; i++)
                *po[i]-- = '\0';
```

dumpblock

## Table 1 (cont')

...dumpblock

```
        (void) putc('\n', fx);
        for (i = 0; i < 2; i++) {
                if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                }
        }
}

/*
 * put out a number line: dumpblock()
 */
static
nums(ix)
        int     ix;     /* index in out[] holding seq line */
{
        char            nline[P_LINE];
        register        i, j;
        register char   *pn, *px, *py;

        for (pn = nline, i = 0; i < lmax+P_SPC; i++, pn++)
                *pn = ' ';
        for (i = nc[ix], py = out[ix]; *py; py++, pn++) {
                if (*py == ' ' || *py == '-')
                        *pn = ' ';
                else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                }
        }
        *pn = '\0';
        nc[ix] = i;
        for (pn = nline; *pn; pn++)
                (void) putc(*pn, fx);
        (void) putc('\n', fx);
}

/*
 * put out a line (name, [num], seq, [num]): dumpblock()
 */
static
putline(ix)
        int     ix;                     {
```

nums

putline

## Table 1 (cont')

...putline

```
        int             i;
        register char   *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
{
        int             i;
        register char   *p0, *p1, cx, *px;

        if (!*out[0] || (*out[0] == ' ' && *(po[0]) == ' ') ||
           !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

stars

## Table 1 (cont')

stripname

```
/*
 * strip path or prefix from pn, return len: pr_align()
 */
static
stripname(pn)
        char    *pn;    /* file name (may be path) */
{
        register char     *px, *py;

        py = 0;
        for (px = pn; *px; px++)
                if (*px == '/')
                        py = px + 1;
        if (py)
                (void) strcpy(pn, py);
        return(strlen(pn));
}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";            /* tmp file for jmps */
FILE    *fj;

int     cleanup();                              /* cleanup tmp file */
long    lseek();

/*
 * remove any tmp file if we blow
 */
cleanup(i)                                                              cleanup
        int     i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char    *
getseq(file, len)                                                       getseq
        char    *file;  /* file name */
        int     *len;   /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

## Table 1 (cont')

**...getseq**

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}
```

**g_calloc**

```
char    *
g_calloc(msg, nx, sz)
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}
```

**readjmps**

```
/*
 * get final jmps from dx[] or tmp file, set pp[], reset dmax: main()
 */
readjmps()
{
        int             fd = -1;
        int             siz, i0, i1;
        register  i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

## Table 1 (cont')

**...readjmps**

```
                        if (j < 0 && dx[dmax].offset && fj) {
                                (void) lseek(fd, dx[dmax].offset, 0);
                                (void) read(fd, (char *)&dx[dmax].jp, sizeof(struct jmp));
                                (void) read(fd, (char *)&dx[dmax].offset, sizeof(dx[dmax].offset));
                                dx[dmax].ijmp = MAXJMP-1;
                        }
                        else
                                break;
                }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {                  /* gap in second seq */
                                pp[1].n[i1] = -siz;
                                xx += siz;
                                /* id = xx - yy + len1 - 1
                                 */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) { /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
                        }
                }
                else
                        break;
        }

        /* reverse the order of jmps
         */
        for (j = 0, i0--; j < i0; j++, i0--) {
                i = pp[0].n[j]; pp[0].n[j] = pp[0].n[i0]; pp[0].n[i0] = i;
                i = pp[0].x[j]; pp[0].x[j] = pp[0].x[i0]; pp[0].x[i0] = i;
        }
        for (j = 0, i1--; j < i1; j++, i1--) {
                i = pp[1].n[j]; pp[1].n[j] = pp[1].n[i1]; pp[1].n[i1] = i;
                i = pp[1].x[j]; pp[1].x[j] = pp[1].x[i1]; pp[1].x[i1] = i;
        }
        if (fd >= 0)
                (void) close(fd);
        if (fj) {
                (void) unlink(jname);
                fj = 0;
                offset = 0;
        }                               }
```

## Table 1 (cont')

```
/*
 * write a filled jmp struct offset of the prev one (if any): nw()
 */
writejmps(ix)                                                              writejmps
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 15 = 33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 10 = 50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

6 divided by 14 = 42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

II. Compositions and Methods of the Invention

A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al. , Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the mainchain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Halomuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W. H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened.

Methods of labeling are well known in the art, and include the use of radiolabels like $^{32}P$-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as E. *coli.* Various E. *coli* strains are publicly available, such as E. *coli* K12 strain MM294 (ATCC 31,446); E. *coli* X1776 (ATCC 31,537); E. *coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis (e.g., B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT kan'; E. coli* W3110 strain 37D6, which has the complete genotype *tonA ptr3 phoA E15 (argF-lac)169 degP ompT rbs7 ilvG kan'; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus;*

*yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10January 1991), *andAspergillus hosts* such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) *andA. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* $\alpha$-factor leaders, the latter described in U.S. Patent No. 5,010,182), or acid phosphatase leader, the C. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2$\mu$ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

E. Uses for PRO

Nucleotide sequences (or their complement) encoding PRO have various applications in the art of molecular biology, including uses as hybridization probes, in chromosome and gene mapping and in the generation of anti-sense RNA and DNA. PRO nucleic acid will also be useful for the preparation of PRO polypeptides by the recombinant techniques described herein.

The full-length native sequence PRO gene, or portions thereof, may be used as hybridization probes for a cDNA library to isolate the full-length PRO cDNA or to isolate still other cDNAs (for instance, those encoding naturally-occurring variants of PRO or PRO from other species) which have a desired sequence identity to the native PRO sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence PRO. By way of example, a screening method will comprise isolating the coding region of the PRO gene using the known DNA sequence to synthesize a selected probe of about 40 bases. Hybridization probes may be labeled by a variety of labels, including radionucleotides such as $^{32}P$ or $^{35}S$, or enzymatic labels such as alkaline phosphatase coupled to the probe via avidin/biotin coupling systems. Labeled probes having a sequence complementary to that of the PRO gene of the present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PRO nucleic acids include antisense or sense oligonucleotides comprising a singe-stranded nucleic acid sequence (either RNA or DNA) capable of binding to target PRO mRNA (sense) or PRO DNA (antisense) sequences. Antisense or sense oligonucleotides, according to the present invention, comprise a fragment of the coding region of PRO DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PRO proteins. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, $CaPO_4$-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

The probes may also be employed in PCR techniques to generate a pool of sequences for identification of closely related PRO coding sequences.

Nucleotide sequences encoding a PRO can also be used to construct hybridization probes for mapping the gene which encodes that PRO and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PRO encode a protein which binds to another protein (example, where the PRO is a receptor), the PRO can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PRO can be used to isolate correlative ligand(s). Screening assays can be designed to find lead compounds that mimic the biological activity of a native PRO or a receptor for PRO. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PRO or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding PRO can be used to clone genomic DNA encoding PRO in accordance with established techniques and the genomic sequences used to generate transgenic animals that contain cells which express DNA encoding PRO. Methods for generating transgenic animals, particularly animals such as mice or rats, have become conventional in the art and are described, for example, in U.S. Patent Nos. 4,736,866 and 4,870,009. Typically, particular cells would be targeted for PRO transgene incorporation with tissue-specific enhancers. Transgenic animals that include a copy of a transgene encoding PRO introduced into the germ line of the animal at an embryonic stage can be used to examine the effect of increased expression of DNA encoding PRO. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of PRO can be used to construct a PRO "knock out" animal which has a defective or altered gene encoding PRO as a result of homologous recombination between the endogenous gene encoding PRO and altered genomic DNA encoding PRO introduced into an embryonic stem cell of the animal. For example, cDNA encoding PRO can be used to clone genomic DNA encoding PRO in accordance with established techniques. A portion of the genomic DNA encoding PRO can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g.,

a mouse or rat) to form aggregation chimeras [see e.g., Bradley, in *Teratocarcinomas and Embryonic Stem Cells: A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO polypeptide.

Nucleic acid encoding the PRO polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes in *vivo.* It has already been shown that short antisense oligonucleotides can be imported into cells where they act as inhibitors, despite their low intracellular concentrations caused by their restricted uptake by the cell membrane. (Zamecnik *et al.,* Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The PRO polypeptides described herein may also be employed as molecular weight markers for protein electrophoresis purposes and the isolated nucleic acid sequences may be used for recombinantly expressing those markers.

The nucleic acid molecules encoding the PRO polypeptides or fragments thereof described herein are useful for chromosome identification. In this regard, there exists an ongoing need to identify new chromosome markers, since relatively few chromosome marking reagents, based upon actual sequence data are presently available. Each PRO nucleic acid molecule of the present invention can be used as a chromosome marker.

The PRO polypeptides and nucleic acid molecules of the present invention may also be used diagnostically for tissue typing, wherein the PRO polypeptides of the present invention may be differentially expressed in one tissue as compared to another, preferably in a diseased tissue as compared to a normal tissue of the same tissue type. PRO nucleic acid molecules will find use for generating probes for PCR, Northern analysis, Southern analysis and Western analysis.

The PRO polypeptides described herein may also be employed as therapeutic agents. The PRO polypeptides of the present invention can be formulated according to known methods to prepare pharmaceutically useful compositions, whereby the PRO product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™ or PEG.

The formulations to be used for in *vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of a PRO polypeptide or agonist or antagonist thereof is employed, normal dosage amounts may vary from about 10 ng/kg to up to 100 mg/kg of mammal body weight or more per day, preferably about 1 μg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and di fferent disorders, that administration targeting one organ or tissue, for example, may necessitate delivery in a manner different from that to another organ or tissue.

Where sustained-release administration of a PRO polypeptide is desired in a formulation with release characteristics suitable for the treatment of any disease or disorder requiring administration of the PRO polypeptide, microencapsulation of the PRO polypeptide is contemplated. Microencapsulation of recombinant proteins for sustained release has been successfully performed with human growth hormone (rhGH), interferon-(rhIFN- ), interleukin-2, and MN rgp120. Johnson et al., Nat. Med., 2:795-799 (1996); Yasuda, Biomed. Ther., 27:1221-1223 (1993); Hora et al., Bio/Technology. 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-062; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that mimic the PRO polypeptide (agonists) or prevent the effect of the PRO polypeptide (antagonists). Screening assays for antagonist drug candidates are designed to identify compounds that bind or complex with the PRO polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PRO polypeptide encoded by a nucleic acid identified herein under conditions and for a time sufficient to allow these two components to interact.

In binding assays, the interaction is binding and the complex formed can be isolated or detected in the reaction mixture. In a particular embodiment, the PRO polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the PRO polypeptide and drying. Alternatively, an immobilized antibody, e.g., a monoclonal antibody, specific for the PRO polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO polypeptide encoded by a gene identified herein, its interaction with that polypeptide can be assayed by methods well known for detecting protein-protein interactions. Such assays include traditional approaches, such as, e.g., cross-linking, coimmunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER$^{TM}$) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PRO polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO polypeptide may be added to a cell along with the compound to be screened for a particular activity and the ability of the compound to inhibit the activity of interest in the presence of the PRO polypeptide indicates that the compound is an antagonist to the PRO polypeptide. Alternatively, antagonists may be detected by combining the PRO polypeptide and a potential antagonist with membrane-bound PRO polypeptide receptors or recombinant receptors under appropriate conditions for a competitive inhibition assay. The PRO polypeptide can be labeled, such as by radioactivity, such that the number of PRO polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO polypeptide and a cDNA library created from this RNA is divided into pools and used to transfect COS cells or other cells that are not responsive to the PRO polypeptide. Transfected cells that are grown on glass slides are exposed to labeled PRO polypeptide. The PRO polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PRO polypeptide can be photoaffinity- linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO polypeptide in the presence of the candidate compound. The ability of the compound to enhance or block this interaction could then be measured.

More specific examples of potential antagonists include an oligonucleotide that binds to the fusions of immunoglobulin with PRO polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO polypeptide that recognizes the receptor but imparts no effect, thereby competitively inhibiting the action of the PRO polypeptide.

Another potential PRO polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the PRO polypeptide. The antisense RNA oligonucleotide hybridizes to the mRNA *in vivo* and blocks translation of the mRNA molecule into the PRO polypeptide (antisense - Okano, Neurochem., 56:560 (1991); Oligodeoxvnucleotides as Antisense Inhibitors of Gene Expression (CRC Press: Boca Raton, FL, 1988). The oligonucleotides described above can also be delivered to cells such that the antisense RNA or DNA may be expressed in *vivo* to inhibit production of the PRO polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the PRO polypeptide, thereby blocking the normal biological activity of the PRO polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra.*

These small molecules can be identified by any one or more of the screening assays discussed hereinabove and/or by any other screening techniques well known for those skilled in the art.

Diagnostic and therapeutic uses of the herein disclosed molecules may also be based upon the positive functional assay hits disclosed and described below.

F. Anti-PRO Antibodies

The present invention further provides anti-PRO antibodies. Exemplary antibodies include polyclonal, monoclonal, humanized, bispecific, and heteroconjugate antibodies.

1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeuret al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an in *vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown in *vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supral or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321 : 522-525 (1986); Riechmann et al., Nature, 332:323-327 (1988); Verhoeyen et al., Science, 239: 1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., l. Immunol., 147(1): 86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al.,* Bio/Technology 10, 779-783 (1992); Lonberg *et al.,* Nature 368 856-859 (1994); Morrison, Nature 368, 812-13 (1994); Fishwild *et al.,* Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539(1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. $F(ab')_2$ bispecific

antibodies). Techniques for generating bispecific antibodies from antibody fragments have been described in the literature. For example, bispecific antibodies can be prepared can be prepared using chemical linkage. Brennan *et al.*, Science 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from E. *coli* and chemically coupled to form bispecific antibodies. Shalaby *et al.*, J. Exp. Med. 175:217-225 (1992) describe the production of a fully humanized bispecific antibody $F(ab')_2$ molecule. Each Fab' fragment was separately secreted from E. *coli* and subjected to directed chemical *coupling in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny *et al.*, J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al.*, Proc. Natl. Acad. Sci. USA 90:6444-6448 (1993) has provided an alternative mechanism for making bispecific antibody fragments. The fragments comprise a heavy-chain variable domain (V H) connected to a light-chain variable domain ($V_1$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber *et al.*, J. Immunol. 152:5368 (1994). Antibodies with more than two valencies are contemplated. For example, trispecific antibodies can be prepared. Tutt *et al.*, J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, *e.g.,* the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement- mediated cell killing and antibody-dependent cellular cytotoxicity (ADCC). See Caron *et al.*, *J.* Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff *et al.* Cancer Research, 53: 2560-2565 (1993). Alternatively, an antibody can be engineered that has dual Fc regions and may thereby have enhanced complement lysis and ADCC capabilities. See Stevenson *et al.*, Anti-Cancer Drug Design. 3: 219-230 (1989).

7. Immunoconjugates

The invention also pertains to immunoconjugates comprising an antibody conjugated to a cytotoxic agent such as a chemotherapeutic agent, toxin *(e. g. ,* an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bisdiazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al.,* Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See W094/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is conjugated to a cytotoxic agent *(e.g.,* a radionucleotide).

8. Immunoliposomes

The antibodies disclosed herein may also be formulated as immunoliposomes. Liposomes containing the antibody are prepared by methods known in the art, such as described in Epstein *et al.,* Proc. Natl. Acad. Sci. USA, 82: 3688 (1985); Hwang *et al.,* Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al .,* *J.* Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

9. Pharmaceutical Compositions of Antibodies

Antibodies specifically binding a PRO polypeptide identified herein, as well as other molecules identified by the screening assays disclosed hereinbefore, can be administered for the treatment of various disorders in the form of pharmaceutical compositions.

If the PRO polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable- region sequences of an antibody, peptide molecules can be designed that retain the ability to bind the target protein sequence. Such peptides can be synthesized chemically and/or produced by recombinant DNA technology. See, e.g., Marasco *et al.*, Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

G. Uses for anti-PRO Antibodies

The anti-PRO antibodies of the invention have various utilities. For example, anti-PRO antibodies may be used in diagnostic assays for PRO, e.g., detecting its expression (and in some cases, differential expression) in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Anti-PRO antibodies also are useful for the affinity purification of PRO from recombinant cell culture or natural sources. In this process, the antibodies against PRO are immobilized on a suitable support, such a Sephadex resin or filter paper, using methods well known in the art. The immobilized antibody then is contacted with a sample containing the PRO to be purified, and thereafter the support is washed with a suitable solvent that will remove substantially all the material in the sample except the PRO, which is bound to the immobilized antibody. Finally, the support is washed with another suitable solvent that will release the PRO from the antibody.

The following examples are offered for illustrative purposes only, and are not intended to limit the scope of the present invention in any way.

All patent and literature references cited in the present specification are hereby incorporated by reference in their entirety.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (e.g., Dayhoff, GenBank), and proprietary databases (e.g. LIPESEQ™, Incyte Pharmaceuticals, Palo Alto, CA). The search was performed using the computer program BLAST or BLAST-2 (Altschul et al., Methods in Enzymology 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

EXAMPLE 2: Isolation of cDNA clones by Amylase Screening

1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the Sail/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl- gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, $MAL^+$, $SUC^+$, $GAL^+$. Preferably, yeast mutants can be employed that have deficient post-translational pathways. Such mutants may have translocation deficient alleles in *sec*71, *sec*72, *sec*62, with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase- expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 $\times$ $10^6$ cells/ml (approx. $OD_{600}$=0.1) into fresh YEPD broth (500 ml) and regrown to 1 $\times$ $10^7$ cells/ml (approx. $OD_{600}$=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM $Li_2OOCCH_3$), and resuspended into LiAc/TE (2.5 ml).

Transformation took place by mixing the prepared cells (100 μl) with freshly denatured single stranded salmon testes DNA (Lofstrand Labs, Gaithersburg, MD) and transforming DNA (1 μg, vol. < 10 μl) in microfuge tubes. The mixture was mixed briefly by vortexing; then 40% PEG/TE (600 μl, 40% polyethylene glycol-4000, 10 mM Tris-HCl, 1 mM EDTA, 100 mM $Li_2OOCCH_3$, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 μl, 10 mM Tris-HCl, 1 mM EDTA pH 7.5) followed by recentrifugation. The cells were then diluted into TE (1ml) and aliquots (200 μl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15 % (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

4. Isolation of DNA by PCR Amplification

When a positive colony was isolated, a portion of it was picked by a toothpick and diluted into sterile water (30 μl) in a 96 well plate. At this time, the positive colonies were either frozen and stored for subsequent analysis or immediately amplified. An aliquot of cells (5 μl) was used as a template for the PCR reaction in a 25 *μl* volume containing: 0.5 μl Klentaq (Clontech, Palo Alto, CA); 4.0 μl 10 mM dNTP's (Perkin Elmer-Cetus); 2.5 μl Kentaq buffer (Clontech); 0.25 μl forward oligo 1; 0.25 μl reverse oligo 2; 12.5 μl distilled water. The sequence of the forward oligonucleotide 1 was:

5'-TGTAAAACGACGGCCAGTTAAATAGACCTGCAATTATTAATCT-3' (SEQ ID NO:553)

The sequence of reverse oligonucleotide 2 was:

5'-CAGGAAACAGCTATGACCACCTGCACACCTGCAAATCCATT-3' (SEQ ID NO:554)

PCR was then performed as follows:

| | | | | |
|---|---|---|---|---|
| a. | | Denature | 92°C, | 5 minutes |
| b. | 3 cycles of: | Denature | 92°C, | 30 seconds |
| | | Anneal | 59°C, | 30 seconds |
| | | Extend | 72°C, | 60 seconds |

Table continued

| | | | | |
|---|---|---|---|---|
| c. | 3 cycles of: | Denature | 92°C, | 30 seconds |
| | | Anneal | 57°C, | 30 seconds |
| | | Extend | 72°C, | 60 seconds |
| d. | 25 cycles of: | Denature | 92°C, | 30 seconds |
| | | Anneal | 55°C, | 30 seconds |
| | | Extend | 72°C, | 60 seconds |
| e. | | Hold | | 4°C |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 μl) was examined by agarose gel electrophoresis in a 1% agarose gel using a Tris-Borate-EDTA (TBE) buffering system as described by Sambrook et al., supra. Clones resulting in a single strong PCR product larger than 400 bp were further analyzed by DNA sequencing after purification with a 96 Qiaquick PCR clean-up column (Qiagen Inc., Chatsworth, CA).

EXAMPLE 3: Isolation of cDNA Clones Using Signal Algorithm Analysis

Various polypeptide-encoding nucleic acid sequences were identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

EXAMPLE 4: Isolation of cDNA clones Encoding Human PRO Polypeptides

Using the techniques described in Examples 1 to 3 above, numerous full-length cDNA clones were identified as encoding PRO polypeptides as disclosed herein. These cDNAs were then deposited under the terms of the Budapest Treaty with the American Type Culture Collection, 10801 University Blvd., Manassas, VA 20110-2209, USA (ATCC) as shown in Table 7 below.

Table 7

| Material | ATCC Dep. No. | Deposit Date |
|---|---|---|
| DNA16438-1387 | 209771 | April 14, 1998 |
| DNA 19360-2552 | 203654 | February 9, 1999 |
| DNA33455-1548 | PTA-127 | May 25, 1999 |
| DNA37155-2651 | PTA-429 | July 27, 1999 |
| DNA38269-2654 | PTA-432 | July 27, 1999 |
| DNA40619-1220 | 209525 | December 10, 1997 |
| DNA44174-2513 | 203577 | January 12, 1999 |
| DNA44675-2662 | PTA-430 | July 27, 1999 |
| DNA45408-2615 | PTA-203 | June 8, 1999 |
| DNA48606-1479 | 203040 | July 1, 1998 |
| DNA52753-2656 | PTA-611 | August 31, 1999 |
| DNA53915-1258 | 209593 | January 21, 1998 |

Table continued

| Material | ATCC Dep. No. | Deposit Date |
|---|---|---|
| DNA53991-2553 | 203649 | February 9, 1999 |
| DNA54009-2517 | 203574 | January 12, 1999 |
| DNA56055-1643 | PTA-129 | May 25, 1999 |
| DNA57033-1403 | 209905 | May 27, 1998 |
| DNA57252-1453 | 203585 | January 12, 1999 |
| DNA58799-1652 | 203665 | February 9, 1999 |
| DNA59770-2652 | PTA-427 | July 27, 1999 |
| DNA59774-2665 | PTA-615 | August 31, 1999 |
| DNA60281-2518 | 203582 | January 12, 1999 |
| DNA60736-2559 | 203838 | March 9, 1999 |
| DNA61875-2653 | PTA-428 | July 27, 1999 |
| DNA62312-2558 | 203836 | March 9, 1999 |
| DNA62849-1604 | PTA-205 | June 8, 1999 |
| DNA66307-2661 | PTA-431 | July 27, 1999 |
| DNA66677-2535 | 203659 | February 9, 1999 |
| DNA71235-1706 | 203584 | January 12, 1999 |
| DNA71289-2547 | PTA-126 | May 25, 1999 |
| DNA73775-1707 | PTA-128 | May 25, 1999 |
| DNA76385-1692 | 203664 | February 9, 1999 |
| DNA76395-2527 | 203578 | January 12, 1999 |
| DNA77622-2516 | 203554 | December 22, 1998 |
| DNA77629-2573 | 203850 | March 16, 1999 |
| DNA77645-2648 | PTA-45 | May 11, 1999 |
| DNA79302-2521 | 203545 | December 22, 1998 |
| DNA79865-2519 | 203544 | December 22, 1998 |
| DNA80135-2655 | PTA-234 | June 15, 1999 |
| DNA80794-2568 | 203848 | March 16, 1999 |
| DNA80796-2523 | 203555 | December 22, 1998 |
| DNA80840-2605 | 203949 | April 20, 1999 |
| DNA80899-2501 | 203539 | December 15, 1998 |
| DNA81228-2580 | 203871 | March 23, 1999 |
| DNA81761-2583 | 203862 | March 23, 1999 |
| DNA82358-2738 | PTA-510 | August 10, 1999 |
| DNA82364-2538 | 203603 | January 20, 1999 |
| DNA82424-2566 | 203813 | March 2, 1999 |
| DNA82430-2557 | 203812 | March 2, 1999 |
| DNA83500-2506 | 203391 | October 29, 1998 |
| DNA83509-2612 | 203965 | April 27, 1999 |
| DNA83560-2569 | 203816 | March 2, 1999 |
| DNA84139-2555 | 203814 | March 2, 1999 |
| DNA84141-2556 | 203810 | March 2, 1999 |
| DNA84142-2613 | PTA-22 | May 4, 1999 |
| DNA84318-2520 | 203580 | January 12, 1999 |
| DNA84909-2590 | 203889 | March 30, 1999 |
| DNA84912-2610 | 203964 | April 27, 1999 |
| DNA84925-2514 | 203548 | December 22, 1998 |
| DNA84928-2564 | 203817 | March 2, 1999 |
| DNA84932-2657 | PTA-235 | June 15, 1999 |
| DNA86592-2607 | 203968 | April 27, 1999 |
| DNA86594-2587 | 203894 | March 30, 1999 |

Table continued

| Material | ATCC Dep. No. | Deposit Date |
|---|---|---|
| DNA86647-2591 | 203893 | March 30, 1999 |
| DNA87185-2563 | 203811 | March 2, 1999 |
| DNA87656-2582 | 203867 | March 23, 1999 |
| DNA87974-2609 | 203963 | April 27, 1999 |
| DNA88001-2565 | 203815 | March 2, 1999 |
| DNA88004-2575 | 203890 | March 30, 1999 |
| DNA89220-2608 | PTA-130 | May 25, 1999 |
| DNA89947-2618 | 203970 | April 27, 1999 |
| DNA90842-2574 | 203845 | March 16, 1999 |
| DNA91775-2581 | 203861 | March 23, 1999 |
| DNA91779-2571 | 203844 | March 16, 1999 |
| DNA92217-2697 | PTA-513 | August 10, 1999 |
| DNA92219-2541 | 203663 | February 9, 1999 |
| DNA92223-2567 | 203851 | March 16, 1999 |
| DNA92225-2603 | 203950 | April 20, 1999 |
| DNA92232-2589 | 203895 | March 30, 1999 |
| DNA92233-2599 | PTA-134 | May 25, 1999 |
| DNA92243-2549 | 203852 | March 16, 1999 |
| DNA92253-2671 | PTA-258 | June 22, 1999 |
| DNA92254-2672 | PTA-259 | June 22, 1999 |
| DNA92255-2584 | 203866 | March 23, 1999 |
| DNA92269-2570 | 203853 | March 16, 1999 |
| DNA92288-2588 | 203892 | March 30, 1999 |
| DNA92290-2550 | 203847 | March 16, 1999 |
| DNA93012-2622 | PTA-21 | May 4, 1999 |
| DNA93020-2642 | PTA-121 | May 25, 1999 |
| DNA94830-2604 | 203951 | April 20, 1999 |
| DNA94833-2579 | 203869 | March 23, 1999 |
| DNA94838-2658 | PTA-232 | June 15, 1999 |
| DNA94844-2686 | PTA-385 | July 20, 1999 |
| DNA94854-2586 | 203864 | March 23, 1999 |
| DNA96868-2677 | PTA-262 | June 22, 1999 |
| DNA96871-2683 | PTA-381 | July 20, 1999 |
| DNA96880-2624 | PTA-15 | May 4, 1999 |
| DNA96986-2660 | PTA-239 | June 15, 1999 |
| DNA96988-2685 | PTA-384 | July 20, 1999 |
| DNA96995-2709 | PTA-475 | August 3, 1999 |
| DNA97004-2562 | 203854 | March 16, 1999 |
| DNA97005-2687 | PTA-378 | July 20, 1999 |
| DNA97009-2668 | PTA-257 | June 22, 1999 |
| DNA97013-2667 | PTA-231 | June 15, 1999 |
| DNA98380-2690 | PTA-388 | July 20, 1999 |
| DNA98561-2696 | PTA-620 | August 31, 1999 |
| DNA98575-2644 | PTA-118 | May 25, 1999 |
| DNA98593-2694 | PTA-477 | August 3, 1999 |
| DNA98600-2703 | PTA-488 | August 3, 1999 |
| DNA99391-2572 | 203849 | March 16, 1999 |
| DNA99393-2560 | 203837 | March 9, 1999 |
| DNA100276-2684 | PTA-380 | July 20, 1999 |
| DNA100312-2645 | PTA-44 | May 11, 1999 |

Table continued

| Material | ATCC Dep. No. | Deposit Date |
|---|---|---|
| DNA100902-2646 | PTA-42 | May 11, 1999 |
| DNA102899-2679 | PTA-123 | May 25, 1999 |
| DNA104875-2720 | PTA-482 | August 3, 1999 |
| DNA105680-2710 | PTA-483 | August 3, 1999 |
| DNA105779-2708 | PTA-485 | August 3, 1999 |
| DNA105794-2695 | PTA-480 | August 3, 1999 |
| DNA105838-2702 | PTA-476 | August 3, 1999 |
| DNA107698-2715 | PTA-472 | August 3, 1999 |
| DNA107701-2711 | PTA-487 | August 3, 1999 |
| DNA 107781-2707 | PTA-484 | August 3, 1999 |
| DNA108670-2744 | PTA-546 | August 17, 1999 |
| DNA 108688-2725 | PTA-515 | August 10, 1999 |
| DNA108769-2765 | PTA-861 | October 19, 1999 |
| DNA108935-2721 | PTA-518 | August 10, 1999 |
| DNA110700-2716 | PTA-512 | August 10, 1999 |
| DNA111750-2706 | PTA-489 | August 3, 1999 |
| DNA123430-2755 | PTA-614 | August 31, 1999 |
| DNA125154-2785 | PTA-957 | November 16,1999 |
| DNA 142238-2768 | PTA-819 | October 5, 1999 |
| DNA22779-1130 | 209280 | September 18, 1997 |
| DNA26847-1395 | 209772 | April 14, 1998 |
| DNA27864-1155 | 209375 | October 16, 1997 |
| DNA27865-1091 | 209296 | September 23, 1997 |
| DNA28497-1130 | 209279 | September 18, 1997 |
| DNA29101-1122 | 209653 | March 5, 1998 |
| DNA32286-1191 | 209385 | October 16, 1997 |
| DNA32288-1132 | 209261 | September 16, 1997 |
| DNA32290-1164 | 209384 | October 16, 1997 |
| DNA32292-1131 | 209258 | September 16, 1997 |
| DNA32298-1132 | 209257 | September 16, 1997 |
| DNA33085-1110 | 209087 | May 30, 1997 |
| DNA33087-1158 | 209381 | October 16, 1997 |
| DNA33089-1132 | 209262 | September 16, 1997 |
| DNA33092-1202 | 209420 | October 28, 1997 |
| DNA33094-1131 | 209256 | September 16, 1997 |
| DNA33107-1135 | 209251 | September 16, 1997 |
| DNA33221-1133 | 209263 | September 16, 1997 |
| DNA33223-1136 | 209264 | September 16, 1997 |
| DNA33460-1166 | 209376 | October 16, 1997 |
| DNA33473-1176 | 209391 | October 17, 1997 |
| DNA33785-1143 | 209417 | October 28, 1997 |
| DNA33786-1132 | 209253 | September 16, 1997 |
| DNA34353-1428 | 209855 | May 12, 1998 |
| DNA34392-1170 | 209526 | December 10, 1997 |
| DNA34434-1139 | 209252 | September 16, 1997 |
| DNA35558-1167 | 209374 | October 16, 1997 |
| DNA35595-1228 | 209528 | December 10, 1997 |
| DNA35638-1216 | 209265 | September 16, 1997 |
| DNA35639-1172 | 209396 | October 17, 1997 |
| DNA35663-1129 | 209201 | August 18, 1997 |

Table continued

| Material | ATCC Dep. No. | Deposit Date |
|---|---|---|
| DNA35674-1142 | 209416 | October 28, 1997 |
| DNA35841-1173 | 209403 | October 17, 1997 |
| DNA35916-1161 | 209419 | October 28, 1997 |
| DNA35918-1174 | 209402 | October 17, 1997 |
| DNA36350-1158 | 209378 | October 16, 1997 |
| DNA37140-1234 | 209489 | November 21, 1997 |
| DNA37150-1178 | 209401 | October 17, 1997 |
| DNA38260-1180 | 209397 | October 17, 1997 |
| DNA40021-1154 | 209389 | October 17, 1997 |
| DNA40587-1231 | 209438 | November 7, 1997 |
| DNA40592-1242 | 209492 | November 21, 1997 |
| DNA40620-1183 | 209388 | October 17, 1997 |
| DNA40628-1216 | 209432 | November 7, 1997 |
| DNA40981-1234 | 209439 | November 7, 1997 |
| DNA40982-1235 | 209433 | November 7, 1997 |
| DNA41234-1242 | 209618 | February 5, 1998 |
| DNA43046-1225 | 209484 | November 21, 1997 |
| DNA43316-1237 | 209487 | November 21, 1997 |
| DNA44167-1243 | 209434 | November 7, 1997 |
| DNA44184-1319 | 209704 | March 26, 1998 |
| DNA44194-1317 | 209808 | April 28, 1998 |
| DNA44196-1353 | 209847 | May 6, 1998 |
| DNA45419-1252 | 209616 | February 5, 1998 |
| DNA46777-1253 | 209619 | February 5, 1998 |
| DNA47394-1572 | 203109 | August 11, 1998 |
| DNA48331-1329 | 209715 | March 31, 1998 |
| DNA48336-1309 | 209669 | March 11, 1998 |
| DNA49142-1430 | 203002 | June 23, 1998 |
| DNA49646-1327 | 209705 | March 26, 1998 |
| DNA49821-1562 | 209981 | June 16, 1998 |
| DNA49829-1346 | 209749 | April 7, 1998 |
| DNA50921-1458 | 209859 | May 12, 1998 |
| DNA52187-1354 | 209845 | May 6, 1998 |
| DNA52196-1348 | 209748 | April 7, 1998 |
| DNA52598-1518 | 203107 | August 11, 1998 |
| DNA54228-1366 | 209801 | April 23, 1998 |
| DNA56047-1456 | 209948 | June 9, 1998 |
| DNA56112-1379 | 209883 | May 20, 1998 |
| DNA56113-1378 | 203049 | July 1, 1998 |
| DNA56352-1358 | 209846 | May 6, 1998 |
| DNA56433-1406 | 209857 | May 12, 1998 |
| DNA56439-1376 | 209864 | May 14, 1998 |
| DNA57530-1375 | 209880 | May 20, 1998 |
| DNA57689-1385 | 209869 | May 14, 1998 |
| DNA57690-1374 | 209950 | June 9, 1998 |
| DNA57693-1424 | 203008 | June 23, 1998 |
| DNA57838-1337 | 203014 | June 23, 1998 |
| DNA58721-1475 | 203110 | August 11, 1998 |
| DNA59205-1421 | 203009 | June 23, 1998 |
| DNA59215-1425 | 209961 | June 9, 1998 |

Table continued

| Material | ATCC Dep. No. | Deposit Date |
|---|---|---|
| DNA59220-1514 | 209962 | June 9, 1998 |
| DNA59294-1381 | 209866 | May 14, 1998 |
| DNA59488-1603 | 203157 | August 25, 1998 |
| DNA59588-1571 | 203106 | August 11, 1998 |
| DNA59606-1471 | 209945 | June 9, 1998 |
| DNA59620-1463 | 209989 | June 16, 1998 |
| DNA59767-1489 | 203108 | August 11, 1998 |
| DNA59777-1480 | 203111 | August 11, 1998 |
| DNA59814-1486 | 203359 | October 20, 1998 |
| DNA59839-1461 | 209988 | June 16, 1998 |
| DNA59846-1503 | 209978 | June 16, 1998 |
| DNA59847-1511 | 203098 | August 4, 1998 |
| DNA60615-1483 | 209980 | June 16, 1998 |
| DNA60621-1516 | 203091 | August 4, 1998 |
| DNA60622-1525 | 203090 | August 4, 1998 |
| DNA60627-1508 | 203092 | August 4, 1998 |
| DNA60764-1533 | 203452 | November 10, 1998 |
| DNA60775-1532 | 203173 | September 1, 1998 |
| DNA61185-1646 | 203464 | November 17, 1998 |
| DNA61873-1574 | 203132 | August 18, 1998 |
| DNA62306-1570 | 203254 | September 9, 1998 |
| DNA62808-1582 | 203358 | October 20, 1998 |
| DNA62814-1521 | 203093 | August 4, 1998 |
| DNA64885-1529 | 203457 | November 3, 1998 |
| DNA64886-1601 | 203241 | September 9, 1998 |
| DNA64888-1542 | 203249 | September 9, 1998 |
| DNA64889-1541 | 203250 | September 9, 1998 |
| DNA64890-1612 | 203131 | August 18, 1998 |
| DNA64903-1553 | 203223 | September 15, 1998 |
| DNA64905-1558 | 203233 | September 15, 1998 |
| DNA65402-1540 | 203252 | September 9, 1998 |
| DNA65405-1547 | 203476 | November 17, 1998 |
| DNA65412-1523 | 203094 | August 4, 1998 |
| DNA66309-1538 | 203235 | September 15, 1998 |
| DNA66667-1596 | 203267 | September 22, 1998 |
| DNA66675-1587 | 203282 | September 22, 1998 |
| DNA68818-2536 | 203657 | February 9, 1999 |
| DNA68864-1629 | 203276 | September 22, 1998 |
| DNA68872-1620 | 203160 | August 25, 1998 |
| DNA71159-1617 | 203135 | August 18, 1998 |
| DNA73727-1673 | 203459 | November 3, 1998 |
| DNA73739-1645 | 203270 | September 22, 1998 |
| DNA76400-2528 | 203573 | January 12, 1999 |
| DNA76510-2504 | 203477 | November 17, 1998 |
| DNA76529-1666 | 203315 | October 6, 1998 |
| DNA76538-1670 | 203313 | October 6, 1998 |
| DNA77301-1708 | 203407 | October 27, 1998 |
| DNA77624-2515 | 203553 | December 22, 1998 |
| DNA79230-2525 | 203549 | December 22, 1998 |
| DNA79862-2522 | 203550 | December 22, 1998 |

Table continued

| Material | ATCC Dep. No. | Deposit Date |
|---|---|---|
| DNA80145-2594 | PTA-204 | June 8, 1999 |
| DNA83500-2506 | 203391 | October 29, 1998 |
| DNA84917-2597 | 203863 | March 23, 1999 |
| DNA92218-2554 | 203834 | March 9, 1999 |
| DNA96042-2682 | PTA-382 | July 20, 1999 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

EXAMPLE 5: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1 % SDS at 42°C.

DNAs having a desired sequence identity with the DNA encoding full-length native sequence PRO can then be identified using standard techniques known in the art.

EXAMPLE 6: Expression of PRO in E. *coli*

This example illustrates preparation of an unglycosylated form of PRO by recombinant expression in *E. coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from E. *coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected E. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an E. *coli* host based on strain 52 (W3110 fuhA(tonA) Ion galE rpoHts(htpRts) clpP(laclq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate·2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purifcation and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4 % (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4 % mannitol by dialysis or by gel filtration using G25 Superfine (Pharmacia) resins equilibrated in the formulation buffer and sterile filtered.

Many of the PRO polypeptides disclosed herein were successfully expressed as described above.

## EXAMPLE 7: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-PRO DNA is mixed with about 1 μg DNA encoding the VA RNA gene [Thimmappaya et al., Cell, 31:543 (1982)] and dissolved in 500 μl of 1 mM Tris-HCl, 0.1 mM EDTA, 0.227 M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 μCi/ml $^{35}$S-cysteine and 200 μCi/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 μg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 μg/ml bovine insulin and 0.1 μg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as $CaPO_4$ or DEAE-dextran. As described above, the cell cultures can be incubated, and the medium replaced with culture medium (alone) or medium containing a radiolabel such as $^{35}S$-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO can then be concentrated and purified by any selected method, such as by $Ni^{2+}$-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect* (Quiagen), Dosper* or Fugene* (Boehringer Mannheim). The cells are grown as described in Lucas et al., supra. Approximately $3 \times 10^{-7}$ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 μm filtered PS20 with 5% 0.2 μm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with $3 \times 10^5$ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at $1.2 \times 10^6$ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 $\mu$L of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

EXAMPLE 8: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB 110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

EXAMPLE 9: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by $Ni^{2+}$-chelate affinity chromatography as follows. Extracts are prepared from recombinant virus-infected Sf9 cells as described by Rupert et al., Nature, 362: 175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$ -NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline

again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

EXAMPLE 10: Preparation of Antibodies that Bind PRO

This example illustrates preparation of monoclonal antibodies which can specifically bind PRO.

Techniques for producing the monoclonal antibodies are known in the art and are described, for instance, in Goding, supra, Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed. EXAMPLE 11: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g., high ionic strength buffers in the presence of detergent). Then, the column is eluted under conditions that disrupt antibody/PRO polypeptide binding (e.g., a low pH buffer such as approximately pH 2-3, or a high concentration of a chaotrope such as urea or thiocyanate ion), and PRO polypeptide is collected.

EXAMPLE 12: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

EXAMPLE 13: Rational Drug Design

The goal of rational drug design is to produce structural analogs of biologically active polypeptide of interest *(i.e.,* a PRO polypeptide) or of small molecules with which they interact, e.g., agonists, antagonists, or inhibitors. Any of these examples can be used to fashion drugs which are more active or stable forms of the PRO polypeptide or which enhance or interfere with the function of the PRO polypeptide *in vivo (c.f.,* Hodgson, Bio/Technology, 9: 19-21 (1991)).

In one approach, the three-dimensional structure of the PRO polypeptide, or of an PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda *et al.,* J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

EXAMPLE 14: Identification of PRO Polypeptides That Stimulate TNF-α Release In Human Blood (Assay 128)

This assay shows that certain PRO polypeptides of the present invention act to stimulate the release of TNF-α in human blood. PRO polypeptides testing positive in this assay are useful for, among other things, research purposes where stimulation of the release of TNF-α would be desired and for the therapeutic treatment of conditions wherein enhanced TNF-α release would be beneficial. Specifically, 200 μl of human blood supplemented with 50mM Hepes buffer (pH 7.2) is aliquoted per well in a 96 well test plate. To each well is then added 300μl of either the test PRO polypeptide in 50 mM Hepes buffer (at various concentrations) or 50 mM Hepes buffer alone (negative control) and the plates are incubated at 37°C for 6 hours. The samples are then centrifuged and 50μl of plasma is collected from each well and tested for the presence of TNF-α by ELISA assay. A positive in the assay is a higher amount of TNF-α in the PRO polypeptide treated samples as compared to the negative control samples.

The following PRO polypeptides tested positive in this assay: PRO195, PR0202, PRO215, PR0221, PR0217, PR0222, PRO198, PR0245, PRO172, PR0265, PR0266, PR0344, PRO337, PR0322, PRO1286, PRO1279, PRO1338 and PRO1343.

EXAMPLE 15: Detection of Polypeptides That Affect Glucose or FFA Uptake in Skeletal Muscle (Assay 106)

This assay is designed to determine whether PRO polypeptides show the ability to affect glucose or FFA uptake by skeletal muscle cells. PRO polypeptides testing positive in this assay would be expected to be useful for the therapeutic treatment of disorders where either the stimulation or inhibition of glucose uptake by skeletal muscle would be beneficial including, for example, diabetes or hyper- or hypo-insulinemia.

In a 96 well format, PRO polypeptides to be assayed are added to primary rat differentiated skeletal muscle, and allowed to incubate overnight. Then fresh media with the PRO polypeptide and +/- insulin are added to the wells. The sample media is then monitored to determine glucose and FFA uptake by the skeletal muscle cells. The insulin will stimulate glucose and FFA uptake by the skeletal muscle, and insulin in media without the PRO polypeptide is used as a positive control, and a limit for scoring. As the PRO polypeptide being tested may either stimulate or inhibit glucose and FFA uptake, results are scored as positive in the assay if greater than 1.5 times or less than 0.5 times the insulin control.

The following PRO polypeptides tested positive as being capable of affecting glucose and/or FFA uptake by skeletal muscle in this assay: PRO182, PR0366, PRO198, PRO172 and PRO719.

EXAMPLE 16: Chondrocyte Re-differentiation Assay (Assay 110)

This assay shows that certain polypeptides of the invention act to induce redifferentiation of chondrocytes, therefore, are expected to be useful for the treatment of various bone and/or cartilage disorders such as, for example, sports injuries and arthritis. The assay is performed as follows. Porcine chondrocytes are isolated by overnight collagenase digestion of articulary cartilage of metacarpophalangeal joints of 4-6 month old female pigs. The isolated cells are then seeded at 25,000 cells/$cm^2$ in Ham F-12 containing 10% FBS and 4 μg/ml gentamycin. The culture media is changed every third day and the cells are then seeded in 96 well plates at 5,000 cells/well in 100μl of the same media without serum and 100 μl of the test PRO polypeptide, 5 nM staurosporin (positive control) or medium alone (negative control) is added to give a final volume of 200 μl/well. After 5 days of incubation at 37°C, a picture of each well is taken and the differentiation state of the chondrocytes is determined. A positive result in the assay occurs when the redifferentiation of the chondrocytes is determined to be more similar to the positive control than the negative control.

The following polypeptide tested positive in this assay: PRO182, PR0366, PRO198 and PRO1868.

EXAMPLE 17: Chondrocyte Proliferation Assay (Assay 111)

This assay is designed to determine whether PRO polypeptides of the present invention show the ability to induce the proliferation and/or redifferentiation of chondrocytes in culture. PRO polypeptides testing positive in this assay would be expected to be useful for the therapeutic treatment of various bone and/or cartilage disorders such as, for example, sports injuries and arthritis.

Porcine chondrocytes are isolated by overnight collagenase digestion of articular cartilage of the metacarpophalangeal joint of 4-6 month old female pigs. The isolated cells are then seeded at 25,000 cells/$cm^2$ in Ham F-12 containing 10% FBS and 4 μg/ml gentamycin. The culture media is changed every third day and the cells are reseeded to 25,000 cells/$cm^2$ every five days. On day 12, the cells are seeded in 96 well plates at 5,000 cells/well in 100μl of the same media without serum and 100 μl of either serum-free medium (negative control), staurosporin (final concentration of 5 nM; positive control) or the test PRO polypeptide are added to give a final volume of 200 μl/well. After 5 days at 37°C, 20 μl of Alamar blue is added to each well and the plates are incubated for an additional 3 hours at 37°C. The fluorescence is then measured in each well (Ex:530 nm; Em: 590 nm). The fluorescence of a plate containing 200 μl of

the serum-free medium is measured to obtain the background. A positive result in the assay is obtained when the fluorescence of the PRO polypeptide treated sample is more like that of the positive control than the negative control.
The following PRO polypeptides tested positive in this assay: PR0202, PR0224, PRO172 and PRO1312.

EXAMPLE 18: Detection of PRO Polypeptides That Affect Glucose or FFA Uptake by Primary Rat Adipocytes (Assay 94)

This assay is designed to determine whether PRO polypeptides show the ability to affect glucose or FFA uptake by adipocyte cells. PRO polypeptides testing positive in this assay would be expected to be useful for the therapeutic treatment of disorders where either the stimulation or inhibition of glucose uptake by adipocytes would be beneficial including, for example, obesity, diabetes or hyper- or hypo-insulinemia.
In a 96 well format, PRO polypeptides to be assayed are added to primary rat adipocytes, and allowed to incubate overnight. Samples are taken at 4 and 16 hours and assayed for glycerol, glucose and FFA uptake. After the 16 hour incubation, insulin is added to the media and allowed to incubate for 4 hours. At this time, a sample is taken and glycerol, glucose and FFA uptake is measured. Media containing insulin without the PRO polypeptide is used as a positive reference control. As the PRO polypeptide being tested may either stimulate or inhibit glucose and FFA uptake, results are scored as positive in the assay if greater than 1.5 times or less than 0.5 times the insulin control.
The following PRO polypeptides tested positive as being capable of affecting glucose and/or FFA uptake in this assay: PR0202, PR0211, PR0344 and PRO1338.

EXAMPLE 19: Gene Expression in Bovine Pericytes (Assay 105)

This assay is designed to identify PRO polypeptides which activate gene expression in pericytes. Such polypeptides would be expected to be useful as growth factors and/or for situations where the activation of gene expression is desired or beneficial. Bovine pericytes are plated on 60mm culture dishes in growth media for1 week. On day 1, various PRO polypeptides are diluted (1 %) and incubated with the pericytes for 1, 4 and 24 hr. timepoints. The cells are harvested and the RNA isolated using TRI-Reagent following the included instructions. The RNA is then quantified by reading the 260/280 OD using a spectrophotometer. The gene expression analysis is done by TaqMan reactions using Perkin Elmer reagents and specially designed bovine probes and primers. Expression of the following genes is analyzed: GAPDH, beta-integrin, connective tissue growth factor (CTGF), ICAM-1, monocyte chemoattractant protein-1 (MCP-1), osteopontin, transforming growth factor-beta (TGF-beta), TGF-beta receptor, tissue inhibitor of metalloproteinase (TIMP), tissue factor (TF), VEGF-$\alpha$, thrombospondin, VEGF-$\beta$, angiopoeitin-2, and collagenase. Replicates are then averaged and the SD determined. The gene expression levels are then normalized to GAPDH. These are then normalized to the expression levels obtained with a protein (PIN32) which does not significantly induce gene expression in bovine pericytes when compared to untreated controls. Any PRO polypeptide that gives a gene expression level 2-fold or higher over the PIN32 control is considered a positive hit.
The following PRO polypeptides tested positive in this assay: PRO366.

EXAMPLE 20: Identification of PRO Polypeptides That Activate Pericytes (Assay 125)

This assay shows that certain polypeptides of the invention act to activate proliferation of pericyte cells and, therefore, are useful not only as diagnostic markers for particular types of pericyte-associated tumors but also for giving rise to antagonists which would be expected to be useful for the therapeutic treatment of pericyte-associated tumors. Such PRO polypeptides also would be expected to be useful as growth factors and/or for situations where the induction of cell proliferation is desired or beneficial. Activation of pericyte proliferation also correlates with the induction of angiogenesis and, as such, PRO polypeptides capable of inducing pericyte proliferation would be expected to be useful for the treatment of conditions where induced angiogenesis would be beneficial including, for example, wound healing, and the like. Specifically, on day 1, pericytes are received from VEC Technologies, and all but 5 ml media is removed from the flask. On day 2, the pericytes are trypsinized, washed, spun and plated on 96 well plates. On day 7, the media is removed and the pericytes are treated with 100 $\mu$l of either the specific PRO polypeptide or control treatments (positive control = DME+5% +/- PDGF @ 500ng/$\mu$l; negative control=PIN32, a polypeptide determined to have no significant effect on pericyte proliferation). C-fos and GAPDH gene expression levels are then determined and the replicates are averaged and the SD is determined. The c-fos values are normalized to GAPDH and the results are expressed as fold increase over PIN32. Anything providing at least a 2-fold or higher response as compared to the negative control is considered positive for the assay.
The following polypeptides tested positive in this assay: PRO366.

EXAMPLE 21: Ability of PRO Polypeptides to Stimulate the Release of Proteoglycans from Cartilage (Assay 97)

The ability of various PRO polypeptides to stimulate the release of proteoglycans from cartilage tissue was tested as follows.

The metacarphophalangeal joint of 4-6 month old pigs was aseptically dissected, and articular cartilage was removed by free hand slicing being careful to avoid the underlying bone. The cartilage was minced and cultured in bulk for 24 hours in a humidified atmosphere of 95% air, 5 % $CO_2$ in serum free (SF) media (DME/F 12 1:1) with 0.1 % BSA and 100U/ml penicillin and 100μg/ml streptomycin. After washing three times, approximately 100 mg of articular cartilage was aliquoted into micronics tubes and incubated for an additional 24 hours in the above SF media. PRO polypeptides were then added at 1 % either alone or in combination with 18 ng/ml interleukin-1α, a known stimulator of proteoglycan release from cartilage tissue. The supernatant was then harvested and assayed for the amount of proteoglycans using the 1,9-dimethyl-methylcne blue (DMB) colorimetric assay (Farndale and Buttle, Biochem. Biophys. Acta 883:173-177 (1985)). A positive result in this assay indicates that the test polypeptide will find use, for example, in the treatment of sports-related joint problems, articular cartilage defects, osteoarthritis or rheumatoid arthritis.

When various PRO polypeptides were tested in the above assay, the polypeptides demonstrated a marked ability to stimulate release of proteoglycans from cartilage tissue both basally and after stimulation with interleukin-1α and at 24 and 72 hours after treatment, thereby indicating that these PRO polypeptides are useful for stimulating proteoglycan release from cartilage tissue. As such, these PRO polypeptides are useful for the treatment of sports-related joint problems, articular cartilage defects, osteoarthritis or rheumatoid arthritis. The polypeptides testing positive in this assay are : PRO216.

EXAMPLE 22: Proliferation of Rat Utricular Supporting Cells (Assay 54)

This assay shows that certain polypeptides of the invention act as potent mitogens for inner ear supporting cells which are auditory hair cell progenitors and, therefore, are useful for inducing the regeneration of auditory hair cells and treating hearing loss in mammals. The assay is performed as follows. Rat UEC-4 utricular epithelial cells are aliquoted into 96 well plates with a density of 3000 cells/well in 200 μl of serum- containing medium at 33°C. The cells are cultured overnight and are then switched to serum-free medium at 37°C. Various dilutions of PRO polypeptides (or nothing for a control) are then added to the cultures and the cells are incubated for 24 hours. After the 24 hour incubation, $^3$H-thymidine (1 μCi/well) is added and the cells are then cultured for an additional 24 hours. The cultures are then washed to remove unincorporated radiolabel, the cells harvested and Cpm per well determined. Cpm of at least 30 % or greater in the PRO polypeptide treated cultures as compared to the control cultures is considered a positive in the assay.

The following polypeptides tested positive in this assay: PRO172.

EXAMPLE 23: Stimulatory Activity in Mixed Lymphocyte Reaction (MLR) Assay (Assay 24)

This example shows that certain polypeptides of the invention are active as a stimulator of the proliferation of stimulated T-lymphocytes. Compounds which stimulate proliferation of lymphocytes are useful therapeutically where enhancement of an immune response is beneficial. A therapeutic agent may take the form of antagonists of the polypeptide of the invention, for example, murine-human chimeric, humanized or human antibodies against the polypeptide.

The basic protocol for this assay is described in Current Protocols in Immunology, unit 3.12; edited by J E Coligan, A M Kruisbeek, D H Marglies, E M Shevach, W Strober, National Institutes of Health, Published by John Wiley & Sons, Inc.

More specifically, in one assay variant, peripheral blood mononuclear cells (PBMC) are isolated from mammalian individuals, for example a human volunteer, by leukopheresis (one donor will supply stimulator PBMCs, the other donor will supply responder PBMCs). If desired, the cells are frozen in fetal bovine serum and DMSO after isolation. Frozen cells may be thawed overnight in assay media (37°C, 5 % $CO_2$) and then washed and resuspended to $3\times10^6$ cells/ml of assay media (RPMI; 10% fetal bovine serum, 1% penicillin/streptomycin, 1% glutamine, 1% HEPES, 1% non-essential amino acids, 1% pyruvate). The stimulator PBMCs are prepared by irradiating the cells (about 3000 Rads).

The assay is prepared by plating in triplicate wells a mixture of:

100:1 of test sample diluted to 1 % or to 0.1%,
50 :1 of irradiated stimulator cells, and
50 :1 of responder PBMC cells.

100 microliters of cell culture media or 100 microliter of CD4-IgG is used as the control. The wells are then incubated at 37°C, 5% $CO_2$ for 4 days. On day 5, each well is pulsed with tritiated thymidine (1.0 mC/well; Amersham). After 6 hours the cells are washed 3 times and then the uptake of the label is evaluated.

In another variant of this assay, PBMCs are isolated from the spleens of Balb/c mice and C57B6 mice. The cells are teased from freshly harvested spleens in assay media (RPMI; 10% fetal bovine serum, 1% penicillin/streptomycin, 1% glutamine, 1% HEPES, 1% non-essential amino acids, 1% pyruvate) and the PBMCs are isolated by overlaying these cells over Lympholyte M (Organon Teknika), centrifuging at 2000 rpm for 20 minutes, collecting and washing the mononuclear cell layer in assay media and resuspending the cells to $1\times10^7$ cells/ml of assay media. The assay is then conducted as described above.

Positive increases over control are considered positive with increases of greater than or equal to 180% being preferred. However, any value greater than control indicates a stimulatory effect for the test protein.

The following PRO polypeptides tested positive in this assay: PRO344.

EXAMPLE 24: Pericyte c-Fos Induction (Assay 93)

This assay shows that certain polypeptides of the invention act to induce the expression of c-fos in pericyte cells and, therefore, are useful not only as diagnostic markers for particular types of pericyte-associated tumors but also for giving rise to antagonists which would be expected to be useful for the therapeutic treatment of pericyte-associated tumors. Induction of c-fos expression in pericytes is also indicative of the induction of angiogenesis and, as such, PRO polypeptides capable of inducing the expression of c-fos would be expected to be useful for the treatment of conditions where induced angiogenesis would be beneficial including, for example, wound healing, and the like. Specifically, on day 1, pericytes are received from VEC Technologies and all but 5 ml of media is removed from flask. On day 2, the pericytes are trypsinized, washed, spun and then plated onto 96 well plates. On day 7, the media is removed and the pericytes are treated with 100 μl of PRO polypeptide test samples and controls (positive control = DME+5% serum +/- PDGF at 500 ng/ml; negative control = protein 32). Replicates are averaged and SD/CV are determined. Fold increase over Protein 32 (buffer control) value indicated by chemiluminescence units (RLU) luminometer reading verses frequency is plotted on a histogram. Two-fold above Protein 32 value is considered positive for the assay. ASY Matrix: Growth media = low glucose DMEM = 20% FBS + 1X pen strep + 1X fungizone. Assay Media = low glucose DMEM +5 % FBS.

The following polypeptides tested positive in this assay: PRO301, PR0619, PR01066 and PRO1265.

EXAMPLE 25: Cytokine Release Assay (Assay 120)

This assay is designed to determine whether PRO polypeptides of the present invention are capable of inducing the release of cytokines from peripheral blood mononuclear cells (PBMCs). PRO polypeptides capable of inducing the release of cytokines from PBMCs are useful from the treatment of conditions which would benefit from enhanced cytokine release and will be readily evident to those of ordinary skill in the art. Specifically, $1\times10^6$ cells/ml of peripheral blood mononuclear cells (PBMC) are cultured with 1% of a PRO polypeptide for 3 days in complete RPMI media. The supernatant is then harvested and tested for increased concentrations of various cytokines by ELISA as compared to a human IgG treated control. A positive in the assay is a 10-fold or greater increase in cytokine concentration in the PRO polypeptide treated sample as compared to the human IgG treated control.

The following polypeptides tested positive in this assay: PRO526 and PRO1343.

EXAMPLE 26: Inhibition of A-Peptide Binding to Factor VIIA (Assay 118)

This assay is designed to identify PRO polypeptides which are capable of inhibiting the binding of A-peptide to factor VIIA, thereby affecting the blood coagulation cascade. PRO polypeptides testing positive in this assay are expected to be useful for the treatment of conditions where alteration of the blood coagulation cascade would be beneficial including, for example, stroke, heart attack and various coagulation disorders. These PRO polypeptides are also useful for the identification of agonist and antagonist molecules which would also be useful for treatment of those conditions.

Specifically, 384 well plates are coated with soluble factor VIIA and are incubated overnight at 4°C. The wells are then decanted and are blocked by the addition of 0.5 % BSA for 1 hour. The wells are then washed and 20μl of biotinylated A-peptide and either various concentration of the PRO polypeptide (test) or nothing (negative control) are added to each well. The plates are then incubated for 1 hour at room temperature. The wells are again washed and then 40μl of streptavidin-europium is added to each well. The plates are then incubated for 30 minutes at room temperature and then washed. 40μl of a fluorescence enhancement solution is then added to each well, the plates incubated for 5 minutes at room temperature and each well is then read on Wallac Victor reader under europium delayed fluorescence settings. Percent inhibition of binding of the A-peptide to the factor VIIA is then determined (as compared to the negative control), wherein a positive in the assay is a percent inhibition of 30% or greater.

The following PRO polypeptides tested positive in this assay: PRO182.

EXAMPLE 27: Inhibition of Adipocyte Differentiation Assay (Assay 66)

This assay is designed to identify PRO polypeptides which are capable of inhibiting insulin-induced differentiation of adipocytes. PRO polypeptides testing positive in this assay would be expected to be useful for the treatment of conditions associated with obesity, diabetes, etc.

Specifically, 3T3-L1 cells are seeded into the wells of 96 well plates at $6\times10^4$ cells/well and allowed to grow to confluency for 7 days. At day 7, the cells are treated with various concentrations of the PRO polypeptide (or nothing for the negative control) in the presence of 1μg/ml insulin, $0.25\times10^{-6}$ M dexamethasone and 0.5mM IBMX. The samples are then incubated at 37°C in 7% $CO_2$ for 2 days. After the incubation, the media is removed by aspiration and the cells are washed with PBS and re-exposed to the PRO polypeptide (or nothing for the negative control) and 1 μg/ml insulin. After 5 days, the media is removed and replaced with fresh PRO polypeptide (or nothing for the negative control) and insulin. After 5 days, the cells are lysed and the cell lysate is assayed using Sigma's Triglyceride [INT] kit (Sigma procedure #336). A positive in the assay is 20% greater inhibition of adipocyte differentiation in the PRO polypeptide treated samples as compared to the negative control.

The following PRO polypeptides tested positive in this assay: PRO185 and PRO198.

EXAMPLE 28: HUVEC Stimulation by PRO Polypeptides (Assay 131)

This assay is designed to identify PRO polypeptides which are capable of stimulating the proliferation of HUVEC cells. PRO polypeptides testing positive in this assay would be expected to be useful for inducing angiogenesis for the treatmnent of conditions where angiogenesis would be beneficial including, for example, wound healing, and the like. Antagonists of these PRO polypeptides would be expected to be useful for inhibiting angiogenesis for the treatment of, for example, tumors, and the like.

Specifically, COSTAR® flat bottom black plates are treated with fibronectin for 20 minutes and then washed twice with PBS. HUVEC cells are then plated at 2000 cells/well in an appropriate growth medium. The plates are then incubated overnight and then the PRO polypeptide (1 % final concentration), nothing (negative control) or IL1β (3.3 ng/ml final concentration; positive control) is added. The plates are again incubated overnight, stained with 1CAM1-Cy5 and read on FMAT. A positive in the assay is a 2-fold or greater increase in fluorescence as compared to the positive control.

The following PRO polypeptides tested positive in this assay: PRO222.

EXAMPLE 29: Promotion of Chondrocyte Redifferentiation (Assay 129)

This assay is designed to determine whether PRO polypeptides of the present invention show the ability to induce the proliferation and/or redifferentiation of chondrocytes in culture. PRO polypeptides testing positive in this assay would be expected to be useful for the therapeutic treatment of various bone and/or cartilage disorders such as, for example, sports injuries and arthritis.

Porcine chondrocytes are isolated by overnight collagenase digestion of articular cartilage of the metacarpophalangeal joint of 4-6 month old female pigs. The isolated cells are then seeded at 25,000 cells/$cm^2$ in Ham F-12 containing 10% FBS and 4 μg/ml gentamycin. The culture media is changed every third day. On day 12, the cells are seeded in 96 well plates at 5,000 cells/well in 100μl of the same media without serum and 100 μl of either serum-free medium (negative control), staurosporin (final concentration of 5 nM; positive control) or the test PRO polypeptide are added to give a final volume of 200 μl/well. After 5 days at 37°C, 22 μl of media comtaining 100μg/ml Hoechst 33342 and 50 μg/ml 5-CFDA is added to each well and incubated for an additional 10 minutes at 37°C. A picture of the green fluorescence is taken for each well and the differentiation state of the chondrocytes is calculated by morphometric analysis. A positive result in the assay is obtained when the > 50% of the PRO polypeptide treated cells are differentiated (compared to the background obtained by the negative control).

The following PRO polypeptides tested positive in this assay: PRO301.

EXAMPLE 30: Microarray Analysis to Detect Overexpression of PRO Polypeptides in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In the present example, the specific preparation of nucleic acids for hybridization and probes, slides, and hybridization conditions are all detailed in U.S. Provisional Patent Application Serial No. 60/193,767, filed on March 31, 2000 and which is herein incorporated by reference.

In the present example, cancerous tumors derived from various human tissues were studied for PRO polypeptide-encoding gene expression relative to non-cancerous human tissue in an attempt to identify those PRO polypeptides which are overexpressed in cancerous tumors. Two sets of experimental data were generated. In one set, cancerous human colon tumor tissue and matched non-cancerous human colon tumor tissue from the same patient ("matched colon control") were obtained and analyzed for PRO polypeptide expression using the above described microarray technology. In the second set of data, cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described PRO polypeptide-encoding nucleic acid sequences were used in the creation of the microarray and RNA from the tumor tissues listed above were used for the hybridization thereto. A value based upon the normalized ratio:experimental ratio was designated as a "cutoff ratio". Only values that were above this cutoff ratio were determined to be significant. Table 8 below shows the results of these experiments, demonstrating that various PRO polypeptides of the preent invention are significantly overexpressed in various human tumor tissues as compared to a non-cancerous human tissue control. As described above, these data demonstrate that the PRO polypeptides of the present invention are useful not only as diagnostic markers for the presence of one or more cancerous tumors, but also serve as therapeutic targets for the treatment of those tumors.

Table 8

| Molecule | is overexpressed in: | as compared to: |
|---|---|---|
| PRO177 | breast tumor | universal normal control |
| PRO177 | liver tumor | universal normal control |
| PRO177 | lung tumor | universal normal control |
| PR03574 | breast tumor | universal normal control |
| PR03574 | colon tumor | matched normal colon control |
| PRO1280 | breast tumor | universal normal control |
| PRO1280 | lung tumor | universal normal control |
| PR04984 | lung tumor | universal normal control |
| PR04988 | colon tumor | universal normal control |
| PR04988 | lung tumor | universal normal control |
| PR0305 | lung tumor | universal normal control |
| PR0305 | colon tumor | universal normal control |
| PRO1866 | prostate tumor | universal normal control |
| PRO1866 | lung tumor | universal normal control |
| PRO1866 | colon tumor | universal normal control |
| PR04996 | breast tumor | universal normal control |
| PR04996 | lung tumor | universal normal control |
| PR04406 | lung tumor | universal normal control |
| PR04406 | colon tumor | universal normal control |
| PRO1120 | colon tumor | universal normal control |
| PRO1120 | breast tumor | universal normal control |

Table continued

| Molecule | is overexpressed in: | as compared to: |
|---|---|---|
| PRO1120 | rectal tumor | universal normal control |
| PRO4990 | lung tumor | universal normal control |
| PR0738 | cervical tumor | universal normal control |
| PR0738 | lung tumor | universal normal control |
| PR0738 | breast tumor | universal normal control |
| PR03577 | lung tumor | universal normal control |
| PRO1879 | breast tumor | universal normal control |
| PRO1879 | lung tumor | universal normal control |
| PRO1879 | colon tumor | universal normal control |
| PRO1471 | lung tumor | universal normal control |
| PR01076 | prostate tumor | universal normal control |
| PRO1483 | lung tumor | universal normal control |
| PR04985 | rectal tumor | universal normal control |
| PR04985 | colon tumor | universal normal control |
| PR04985 | breast tumor | universal normal control |
| PR04985 | lung tumor | universal normal control |
| PR05000 | lung tumor | universal normal control |
| PRO1881 | liver tumor | universal normal control |
| PRO1881 | lung tumor | universal normal control |
| PRO1881 | breast tumor | universal normal control |
| PRO4314 | lung tumor | universal normal control |
| PR04314 | breast tumor | universal normal control |
| PR04987 | lung tumor | universal normal control |
| PRO4313 | lung tumor | universal normal control |
| PR04313 | breast tumor | universal normal control |
| PR04799 | colon tumor | universal normal control |
| PR04995 | liver tumor | universal normal control |
| PR04995 | colon tumor | universal normal control |
| PR04995 | colon tumor | matched normal colon control |
| PR01341 | prostate tumor | universal normal control |
| PR01341 | lung tumor | universal normal control |
| PRO1341 | colon tumor | universal normal control |
| PR01341 | colon tumor | matched normal colon control |
| PRO1777 | lung tumor | universal normal control |
| PRO1777 | colon tumor | matched normal colon control |
| PR03580 | lung tumor | universal normal control |
| PR03580 | prostate tumor | universal normal control |
| PRO1779 | lung tumor | universal normal control |
| PRO1779 | colon tumor | universal normal control |
| PRO 1779 | cervical tumor | universal normal control |
| PRO1754 | breast tumor | universal normal control |
| PRO 1754 | lung tumor | universal normal control |
| PRO1906 | breast tumor | universal normal control |
| PRO1906 | colon tumor | universal normal control |
| PRO1906 | prostate tumor | universal normal control |
| PRO1870 | breast tumor | universal normal control |
| PR04329 | lung tumor | universal normal control |
| PR04979 | colon tumor | universal normal control |
| PRO1885 | rectal tumor | universal normal control |
| PRO1885 | colon tumor | universal normal control |

Table continued

| Molecule | is overexpressed in: | as compared to: |
|---|---|---|
| PRO1885 | colon tumor | matched normal colon control |
| PRO1882 | prostate tumor | universal normal control |
| PRO1882 | lung tumor | universal normal control |
| PRO1882 | colon tumor | universal normal control |
| PRO1882 | breast tumor | universal normal control |
| PRO1882 | cervical tumor | universal normal control |
| PR04989 | rectal tumor | universal normal control |
| PR04989 | breast tumor | universal normal control |
| PR04989 | colon tumor | matched normal colon control |
| PR04989 | colon tumor | universal normal control |
| PR04323 | lung tumor | universal normal control |
| PR04323 | liver tumor | universal normal control |
| PRO1886 | breast tumor | universal normal control |
| PRO1886 | lung tumor | universal normal control |
| PRO1886 | rectal tumor | universal normal control |
| PR04395 | colon tumor | universal normal control |
| PR04395 | prostate tumor | universal normal control |
| PR04395 | lung tumor | universal normal control |
| PR04395 | cervical tumor | universal normal control |
| PRO1782 | colon tumor | universal normal control |
| PRO1782 | lung tumor | universal normal control |
| PR04388 | lung tumor | universal normal control |
| PRO4341 | breast tumor | universal normal control |
| PRO4341 | lung tumor | universal normal control |
| PR03438 | lung tumor | universal normal control |
| PRO4321 | breast tumor | universal normal control |
| PRO4321 | lung tumor | universal normal control |
| PRO4321 | colon tumor | universal normal control |
| PRO4304 | breast tumor | universal normal control |
| PR04304 | lung tumor | universal normal control |
| PR04403 | colon tumor | universal normal control |
| PR04403 | breast tumor | universal normal control |
| PR04403 | lung tumor | universal normal control |
| PR04324 | lung tumor | universal normal control |
| PR04324 | breast tumor | universal normal control |
| PR04303 | cervical tumor | universal normal control |
| PR04303 | lung tumor | universal normal control |
| PR04303 | breast tumor | universal normal control |
| PR04303 | colon tumor | universal normal control |
| PR04303 | prostate tumor | universal normal control |
| PR04305 | breast tumor | universal normal control |
| PR04305 | lung tumor | universal normal control |
| PR04305 | colon tumor | universal normal control |
| PR04305 | liver tumor | universal normal control |
| PRO4404 | lung tumor | universal normal control |
| PRO4404 | breast tumor | universal normal control |
| PRO4404 | rectal tumor | universal normal control |
| PRO1884 | lung tumor | universal normal control |
| PR04349 | colon tumor | universal normal control |
| PR04349 | lung tumor | universal normal control |

Table continued

| Molecule | is overexpressed in: | as compared to: |
|---|---|---|
| PR04401 | colon tumor | universal normal control |
| PRO4401 | lung tumor | universal normal control |
| PRO1867 | lung tumor | universal normal control |
| PRO1867 | liver tumor | universal normal control |
| PRO4319 | breast tumor | universal normal control |
| PR04319 | lung tumor | universal normal control |
| PRO4991 | lung tumor | universal normal control |
| PRO4991 | colon tumor | universal normal control |
| PR04398 | lung tumor | universal normal control |
| PR04346 | lung tumor | universal normal control |
| PR04350 | colon tumor | universal normal control |
| PR04350 | prostate tumor | universal normal control |
| PR04350 | lung tumor | universal normal control |
| PRO4318 | prostate tumor | universal normal control |
| PRO4318 | lung tumor | universal normal control |
| PR04340 | breast tumor | universal normal control |
| PR04340 | lung tumor | universal normal control |
| PR04400 | breast tumor | universal normal control |
| PR04400 | lung tumor | universal normal control |
| PR04320 | lung tumor | universal normal control |
| PR04409 | lung tumor | universal normal control |
| PRO4409 | cervical tumor | universal normal control |
| PR04409 | colon tumor | universal normal control |
| PR04399 | lung tumor | universal normal control |
| PR04399 | breast tumor | universal normal control |
| PRO4418 | lung tumor | universal normal control |
| PR04418 | breast tumor | universal normal control |
| PR04330 | cervical tumor | universal normal control |
| PR04330 | colon tumor | matched normal colon control |
| PR04339 | breast tumor | universal normal control |
| PR04339 | colon tumor | universal normal control |
| PRO4326 | lung tumor | universal normal control |
| PR04326 | colon tumor | universal normal control |
| PRO6014 | breast tumor | universal normal control |
| PR03446 | colon tumor | universal normal control |
| PR03446 | lung tumor | universal normal control |
| PR04322 | lung tumor | universal normal control |
| PR04322 | rectal tumor | universal normal control |
| PR04322 | colon tumor | matched normal colon control |
| PRO4381 | breast tumor | universal normal control |
| PRO4381 | lung tumor | universal normal control |
| PRO4381 | colon tumor | universal normal control |
| PR04348 | lung tumor | universal normal control |
| PR04348 | prostate tumor | universal normal control |
| PR04371 | breast tumor | universal normal control |
| PR03742 | colon tumor | universal normal control |
| PR03742 | lung tumor | universal normal control |
| PR05773 | lung tumor | universal normal control |
| PR05773 | colon tumor | universal normal control |
| PR05773 | prostate tumor | universal normal control |

Table continued

| Molecule | is overexpressed in: | as compared to: |
|---|---|---|
| PR05774 | colon tumor | universal normal control |
| PRO4343 | colon tumor | universal normal control |
| PR04325 | lung tumor | universal normal control |
| PR04347 | lung tumor | universal normal control |
| PR04347 | colon tumor | universal normal control |
| PR04347 | rectal tumor | universal normal control |
| PR03743 | colon tumor | universal normal control |
| PR03743 | lung tumor | universal normal control |
| PR03743 | prostate tumor | universal normal control |
| PR04426 | colon tumor | universal normal control |
| PR04500 | colon tumor | universal normal control |
| PR04389 | breast tumor | universal normal control |
| PR04389 | lung tumor | universal normal control |
| PR04337 | colon tumor | universal normal control |
| PR04337 | breast tumor | universal normal control |
| PR04337 | lung tumor | universal normal control |
| PR04992 | lung tumor | universal normal control |
| PRO5996 | lung tumor | universal normal control |
| PR04345 | lung tumor | universal normal control |
| PR04345 | colon tumor | universal normal control |
| PRO5780 | lung tumor | universal normal control |
| PRO5780 | breast tumor | universal normal control |
| PRO5992 | lung tumor | universal normal control |
| PR05992 | colon tumor | universal normal control |
| PR05992 | breast tumor | universal normal control |
| PR04428 | prostate tumor | universal normal control |
| PR04994 | lung tumor | universal normal control |
| PRO5995 | lung tumor | universal normal control |
| PR05995 | colon tumor | universal normal control |
| PR06094 | lung tumor | universal normal control |
| PRO6094 | colon tumor | universal normal control |
| PRO4317 | lung tumor | universal normal control |
| PR04317 | colon tumor | universal normal control |
| PR04317 | liver tumor | universal normal control |
| PR04317 | colon tumor | matched normal colon control |
| PRO5997 | colon tumor | universal normal control |
| PR05997 | lung tumor | universal normal control |
| PRO5005 | lung tumor | universal normal control |
| PR05005 | colon tumor | universal normal control |
| PR05004 | colon tumor | universal normal control |
| PR06001 | breast tumor | universal normal control |
| PR06013 | colon tumor | universal normal control |
| PR04502 | lung tumor | universal normal control |
| PR04502 | colon tumor | universal normal control |
| PR06007 | breast tumor | universal normal control |
| PR06028 | breast tumor | universal normal control |
| PR06028 | colon tumor | universal normal control |
| PR04327 | prostate tumor | universal normal control |
| PR04315 | colon tumor | universal normal control |
| PRO5993 | lung tumor | universal normal control |

Table continued

| Molecule | is overexpressed in: | as compared to: |
|---|---|---|
| PR05993 | colon tumor | universal normal control |
| PR04503 | colon tumor | universal normal control |
| PR04976 | lung tumor | universal normal control |
| PRO5798 | lung tumor | universal normal control |
| PR05798 | colon tumor | universal normal control |
| PR06242 | colon tumor | universal normal control |
| PR06242 | colon tumor | matched normal colon control |
| PR06242 | breast tumor | universal normal control |
| PR06242 | liver tumor | universal normal control |
| PRO6242 | rectal tumor | universal normal control |
| PR06095 | breast tumor | universal normal control |
| PRO6095 | lung tumor | universal normal control |
| PRO6093 | colon tumor | universal normal control |
| PRO6093 | breast tumor | universal normal control |
| PR06093 | lung tumor | universal normal control |
| PR06093 | colon tumor | matched normal colon control |
| PR06012 | colon tumor | universal normal control |
| PRO6027 | lung tumor | universal normal control |
| PR06027 | colon tumor | universal normal control |
| PR06027 | rectal tumor | universal normal control |
| PRO6181 | prostate tumor | universal normal control |
| PRO6181 | lung tumor | universal normal control |
| PRO6181 | colon tumor | universal normal control |
| PRO6097 | colon tumor | universal normal control |
| PR06097 | lung tumor | universal normal control |
| PRO6090 | lung tumor | universal normal control |
| PR07171 | lung tumor | universal normal control |
| PR07171 | colon tumor | universal normal control |
| PR07171 | breast tumor | universal normal control |
| PR06258 | prostate tumor | universal normal control |
| PR06258 | breast tumor | universal normal control |
| PRO6258 | cervical tumor | universal normal control |
| PR06258 | liver tumor | universal normal control |
| PR06258 | colon tumor | universal normal control |
| PR09820 | prostate tumor | universal normal control |
| PR06243 | lung tumor | universal normal control |
| PR06182 | lung tumor | universal normal control |
| PRO6079 | lung tumor | universal normal control |
| PRO6079 | colon tumor | universal normal control |
| PR06079 | breast tumor | universal normal control |
| PR06079 | prostate tumor | universal normal control |
| PR07434 | lung tumor | universal normal control |
| PR09865 | colon tumor | universal normal control |
| PR09828 | colon tumor | universal normal control |
| PRO196 | colon tumor | universal normal control |
| PRO196 | lung tumor | universal normal control |
| PRO196 | breast tumor | universal normal control |
| PRO197 | colon tumor | universal normal control |
| PRO197 | lung tumor | universal normal control |
| PRO197 | breast tumor | universal normal control |

Table continued

| Molecule | is overexpressed in: | as compared to: |
|---|---|---|
| PRO195 | colon tumor | universal normal control |
| PRO195 | lung tumor | universal normal control |
| PRO195 | breast tumor | universal normal control |
| PRO187 | lung tumor | universal normal control |
| PRO187 | liver tumor | universal normal control |
| PRO182 | colon tumor | universal normal control |
| PRO182 | lung tumor | universal normal control |
| PRO182 | breast tumor | universal normal control |
| PRO188 | rectal tumor | universal normal control |
| PRO183 | colon tumor | universal normal control |
| PRO183 | lung tumor | universal normal control |
| PRO183 | breast tumor | universal normal control |
| PRO183 | rectal tumor | universal normal control |
| PRO184 | lung tumor | universal normal control |
| PRO184 | breast tumor | universal normal control |
| PRO185 | lung tumor | universal normal control |
| PR0200 | colon tumor | universal normal control |
| PR0200 | lung tumor | universal normal control |
| PR0200 | breast tumor | universal normal control |
| PR0200 | rectal tumor | universal normal control |
| PR0202 | colon tumor | universal normal control |
| PR0202 | lung tumor | universal normal control |
| PR0202 | breast tumor | universal normal control |
| PR0202 | rectal tumor | universal normal control |
| PR0202 | liver tumor | universal normal control |
| PRO214 | colon tumor | universal normal control |
| PRO214 | lung tumor | universal normal control |
| PR0215 | colon tumor | universal normal control |
| PR0215 | lung tumor | universal normal control |
| PRO215 | breast tumor | universal normal control |
| PRO219 | colon tumor | universal normal control |
| PRO219 | lung tumor | universal normal control |
| PRO219 | breast tumor | universal normal control |
| PRO219 | liver tumor | universal normal control |
| PRO211 | lung tumor | universal normal control |
| PRO211 | breast tumor | universal normal control |
| PR0220 | colon tumor | universal normal control |
| PR0220 | lung tumor | universal normal control |
| PR0220 | breast tumor | universal normal control |
| PR0366 | colon tumor | universal normal control |
| PR0366 | lung tumor | universal normal control |
| PR0366 | breast tumor | universal normal control |
| PRO216 | lung tumor | universal normal control |
| PR0221 | colon tumor | universal normal control |
| PR0221 | lung tumor | universal normal control |
| PRO221 | breast tumor | universal normal control |
| PR0228 | lung tumor | universal normal control |
| PR0228 | breast tumor | universal normal control |
| PR0217 | lung tumor | universal normal control |
| PR0217 | breast tumor | universal normal control |

Table continued

| Molecule | is overexpressed in: | as compared to: |
|---|---|---|
| PRO222 | colon tumor | universal normal control |
| PR0222 | lung tumor | universal normal control |
| PR0222 | breast tumor | universal normal control |
| PR0224 | colon tumor | universal normal control |
| PR0224 | lung tumor | universal normal control |
| PR0224 | breast tumor | universal normal control |
| PR0224 | prostate tumor | universal normal control |
| PR0224 | rectal tumor | universal normal control |
| PR0230 | colon tumor | universal normal control |
| PR0230 | lung tumor | universal normal control |
| PR0230 | breast tumor | universal normal control |
| PR0230 | prostate tumor | universal normal control |
| PRO198 | colon tumor | universal normal control |
| PRO198 | lung tumor | universal normal control |
| PRO198 | breast tumor | universal normal control |
| PRO198 | liver tumor | universal normal control |
| PR0226 | lung tumor | universal normal control |
| PR0226 | breast tumor | universal normal control |
| PRO261 | lung tumor | universal normal control |
| PRO242 | colon tumor | universal normal control |
| PR0242 | lung tumor | universal normal control |
| PRO242 | breast tumor | universal normal control |
| PR0227 | colon tumor | universal normal control |
| PR0227 | lung tumor | universal normal control |
| PR0237 | colon tumor | universal normal control |
| PR0237 | lung tumor | universal normal control |
| PR0237 | breast tumor | universal normal control |
| PR0237 | prostate tumor | universal normal control |
| PRO241 | colon tumor | universal normal control |
| PRO241 | lung tumor | universal normal control |
| PRO241 | breast tumor | universal normal control |
| PRO231 | colon tumor | universal normal control |
| PRO231 | lung tumor | universal normal control |
| PRO231 | breast tumor | universal normal control |
| PRO231 | rectal tumor | universal normal control |
| PR0235 | colon tumor | universal normal control |
| PR0235 | lung tumor | universal normal control |
| PR0235 | breast tumor | universal normal control |
| PRO235 | liver tumor | universal normal control |
| PR0323 | lung tumor | universal normal control |
| PR0323 | breast tumor | universal normal control |
| PR0323 | rectal tumor | universal normal control |
| PR0245 | colon tumor | universal normal control |
| PR0245 | lung tumor | universal normal control |
| PR0245 | breast tumor | universal normal control |
| PR0245 | cervical tumor | universal normal control |
| PR0245 | liver tumor | universal normal control |
| PR0246 | colon tumor | universal normal control |
| PR0246 | lung tumor | universal normal control |
| PR0246 | breast tumor | universal normal control |

Table continued

| Molecule | is overexpressed in: | as compared to: |
|---|---|---|
| PR0288 | lung tumor | universal normal control |
| PR0288 | breast tumor | universal normal control |
| PR0248 | lung tumor | universal normal control |
| PR0248 | rectal tumor | universal normal control |
| PR0257 | colon tumor | universal normal control |
| PR0257 | lung tumor | universal normal control |
| PR0257 | prostate tumor | universal normal control |
| PRO172 | colon tumor | universal normal control |
| PRO172 | lung tumor | universal normal control |
| PRO172 | breast tumor | universal normal control |
| PRO258 | colon tumor | universal normal control |
| PR0258 | lung tumor | universal normal control |
| PR0258 | breast tumor | universal normal control |
| PR0265 | lung tumor | universal normal control |
| PR0265 | breast tumor | universal normal control |
| PR0265 | rectal tumor | universal normal control |
| PR0326 | colon tumor | universal normal control |
| PR0326 | lung tumor | universal normal control |
| PR0326 | breast tumor | universal normal control |
| PR0326 | liver tumor | universal normal control |
| PR0266 | colon tumor | universal normal control |
| PR0266 | lung tumor | universal normal control |
| PR0266 | breast tumor | universal normal control |
| PR0269 | lung tumor | universal normal control |
| PR0269 | rectal tumor | universal normal control |
| PR0285 | colon tumor | universal normal control |
| PR0285 | lung tumor | universal normal control |
| PR0285 | breast tumor | universal normal control |
| PR0328 | colon tumor | universal normal control |
| PR0328 | lung tumor | universal normal control |
| PR0328 | breast tumor | universal normal control |
| PR0344 | breast tumor | universal normal control |
| PR0272 | lung tumor | universal normal control |
| PRO301 | colon tumor | universal normal control |
| PR0301 | lung tumor | universal normal control |
| PR0301 | breast tumor | universal normal control |
| PR0331 | colon tumor | universal normal control |
| PR0331 | lung tumor | universal normal control |
| PR0331 | breast tumor | universal normal control |
| PR0332 | colon tumor | universal normal control |
| PR0332 | lung tumor | universal normal control |
| PR0332 | breast tumor | universal normal control |
| PR0353 | colon tumor | universal normal control |
| PR0353 | lung tumor | universal normal control |
| PR0353 | breast tumor | universal normal control |
| PR0310 | colon tumor | universal normal control |
| PR0310 | lung tumor | universal normal control |
| PRO310 | breast tumor | universal normal control |
| PR0310 | rectal tumor | universal normal control |
| PR0337 | colon tumor | universal normal control |

Table continued

| Molecule | is overexpressed in: | as compared to: |
|---|---|---|
| PR0337 | lung tumor | universal normal control |
| PR0337 | breast tumor | universal normal control |
| PR0346 | lung tumor | universal normal control |
| PR0350 | lung tumor | universal normal control |
| PR0350 | breast tumor | universal normal control |
| PR0526 | colon tumor | universal normal control |
| PR0526 | lung tumor | universal normal control |
| PRO526 | breast tumor | universal normal control |
| PRO381 | colon tumor | universal normal control |
| PRO381 | lung tumor | universal normal control |
| PRO381 | breast tumor | universal normal control |
| PRO381 | prostate tumor | universal normal control |
| PRO846 | colon tumor | universal normal control |
| PR0846 | lung tumor | universal normal control |
| PR0363 | colon tumor | universal normal control |
| PR0363 | lung tumor | universal normal control |
| PR0365 | lung tumor | universal normal control |
| PR0365 | breast tumor | universal normal control |
| PRO1310 | breast tumor | universal normal control |
| PR0731 | colon tumor | universal normal control |
| PR0731 | lung tumor | universal normal control |
| PR0731 | breast tumor | universal normal control |
| PR0322 | colon tumor | universal normal control |
| PRO322 | lung tumor | universal normal control |
| PR0322 | breast tumor | universal normal control |
| PR0322 | rectal tumor | universal normal control |
| PR0322 | liver tumor | universal normal control |
| PRO536 | lung tumor | universal normal control |
| PRO536 | breast tumor | universal normal control |
| PR0536 | liver tumor | universal normal control |
| PR0719 | colon tumor | universal normal control |
| PR0719 | lung tumor | universal normal control |
| PR0719 | breast tumor | universal normal control |
| PR0619 | colon tumor | universal normal control |
| PR0619 | lung tumor | universal normal control |
| PR0619 | breast tumor | universal normal control |
| PR0771 | colon tumor | universal normal control |
| PRO771 | lung tumor | universal normal control |
| PRO771 | breast tumor | universal normal control |
| PRO1083 | colon tumor | universal normal control |
| PRO1083 | lung tumor | universal normal control |
| PRO1083 | breast tumor | universal normal control |
| PRO1083 | prostate tumor | universal normal control |
| PRO862 | colon tumor | universal normal control |
| PRO862 | lung tumor | universal normal control |
| PRO862 | breast tumor | universal normal control |
| PR0733 | colon tumor | universal normal control |
| PR0733 | lung tumor | universal normal control |
| PR0733 | breast tumor | universal normal control |
| PR0733 | liver tumor | universal normal control |

Table continued

| Molecule | is overexpressed in: | as compared to: |
|---|---|---|
| PRO1188 | lung tumor | universal normal control |
| PRO1188 | breast tumor | universal normal control |
| PRO1188 | rectal tumor | universal normal control |
| PR0770 | lung tumor | universal normal control |
| PR0770 | breast tumor | universal normal control |
| PRO1080 | colon tumor | universal normal control |
| PRO1080 | lung tumor | universal normal control |
| PRO1080 | breast tumor | universal normal control |
| PRO1017 | colon tumor | universal normal control |
| PRO1017 | lung tumor | universal normal control |
| PRO1017 | breast tumor | universal normal control |
| PRO1016 | colon tumor | universal normal control |
| PRO1016 | lung tumor | universal normal control |
| PRO1016 | breast tumor | universal normal control |
| PRO1016 | rectal tumor | universal normal control |
| PR0792 | lung tumor | universal normal control |
| PR0938 | colon tumor | universal normal control |
| PR0938 | lung tumor | universal normal control |
| PR0938 | breast tumor | universal normal control |
| PRO1012 | colon tumor | universal normal control |
| PRO1012 | lung tumor | universal normal control |
| PRO1012 | rectal tumor | universal normal control |
| PRO1012 | liver tumor | universal normal control |
| PRO1008 | lung tumor | universal normal control |
| PRO1075 | colon tumor | universal normal control |
| PRO1075 | lung tumor | universal normal control |
| PRO1007 | colon tumor | universal normal control |
| PRO1007 | lung tumor | universal normal control |
| PRO1007 | breast tumor | universal normal control |
| PRO1007 | rectal tumor | universal normal control |
| PRO1056 | colon tumor | universal normal control |
| PRO1056 | lung tumor | universal normal control |
| PRO1056 | breast tumor | universal normal control |
| PRO791 | colon tumor | universal normal control |
| PR0791 | lung tumor | universal normal control |
| PR0791 | breast tumor | universal normal control |
| PR0791 | rectal tumor | universal normal control |
| PRO1111 | colon tumor | universal normal control |
| PRO1111 | lung tumor | universal normal control |
| PRO1111 | breast tumor | universal normal control |
| PR0812 | lung tumor | universal normal control |
| PR0812 | breast tumor | universal normal control |
| PRO812 | rectal tumor | universal normal control |
| PRO1066 | lung tumor | universal normal control |
| PRO1185 | colon tumor | universal normal control |
| PRO1185 | lung tumor | universal normal control |
| PRO1185 | breast tumor | universal normal control |
| PRO 1031 | lung tumor | universal normal control |
| PRO1360 | lung tumor | universal normal control |
| PRO1360 | breast tumor | universal normal control |

Table continued

| Molecule | is overexpressed in: | as compared to: |
|---|---|---|
| PRO1309 | lung tumor | universal normal control |
| PRO1309 | breast tumor | universal normal control |
| PRO1107 | lung tumor | universal normal control |
| PRO 1107 | breast tumor | universal normal control |
| PR0836 | colon tumor | universal normal control |
| PRO836 | lung tumor | universal normal control |
| PRO1132 | lung tumor | universal normal control |
| PRO1132 | breast tumor | universal normal control |
| PRO1131 | colon tumor | universal normal control |
| PRO1131 | lung tumor | universal normal control |
| PRO1131 | breast tumor | universal normal control |
| PRO1131 | liver tumor | universal normal control |
| PRO1130 | colon tumor | universal normal control |
| PRO1130 | lung tumor | universal normal control |
| PRO1130 | breast tumor | universal normal control |
| PRO844 | colon tumor | universal normal control |
| PRO844 | lung tumor | universal normal control |
| PR0844 | breast tumor | universal normal control |
| PRO844 | rectal tumor | universal normal control |
| PRO1154 | colon tumor | universal normal control |
| PRO1154 | lung tumor | universal normal control |
| PRO1154 | rectal tumor | universal normal control |
| PRO1154 | liver tumor | universal normal control |
| PRO1181 | lung tumor | universal normal control |
| PRO1181 | breast tumor | universal normal control |
| PRO1126 | colon tumor | universal normal control |
| PRO1126 | lung tumor | universal normal control |
| PRO1126 | breast tumor | universal normal control |
| PRO1126 | adrenal tumor | universal normal control |
| PRO1186 | colon tumor | universal normal control |
| PRO1186 | lung tumor | universal normal control |
| PRO1186 | breast tumor | universal normal control |
| PRO1186 | liver tumor | universal normal control |
| PRO1198 | colon tumor | universal normal control |
| PRO1198 | lung tumor | universal normal control |
| PRO1159 | lung tumor | universal normal control |
| PRO1159 | breast tumor | universal normal control |
| PRO1159 | liver tumor | universal normal control |
| PRO1265 | colon tumor | universal normal control |
| PRO1265 | breast tumor | universal normal control |
| PRO1250 | colon tumor | universal normal control |
| PRO1250 | lung tumor | universal normal control |
| PR01250 | breast tumor | universal normal control |
| PRO1475 | colon tumor | universal normal control |
| PRO1475 | breast tumor | universal normal control |
| PRO1312 | colon tumor | universal normal control |
| PR01312 | lung tumor | universal normal control |
| PRO1312 | breast tumor | universal normal control |
| PRO1308 | colon tumor | universal normal control |
| PRO1308 | lung tumor | universal normal control |

Table continued

| Molecule | is overexpressed in: | as compared to: |
|---|---|---|
| PRO1308 | liver tumor | universal normal control |
| PRO1326 | colon tumor | universal normal control |
| PRO1325 | lung tumor | universal normal control |
| PRO1326 | breast tumor | universal normal control |
| PR01192 | colon tumor | universal normal control |
| PRO1192 | lung tumor | universal normal control |
| PRO1192 | breast tumor | universal normal control |
| PRO1246 | colon tumor | universal normal control |
| PRO1246 | lung tumor | universal normal control |
| PRO1246 | breast tumor | universal normal control |
| PRO1246 | prostate tumor | universal normal control |
| PRO1356 | colon tumor | universal normal control |
| PRO1356 | lung tumor | universal normal control |
| PRO1356 | breast tumor | universal normal control |
| PRO1275 | lung tumor | universal normal control |
| PRO1275 | breast tumor | universal normal control |
| PRO1274 | lung tumor | universal normal control |
| PRO1358 | colon tumor | universal normal control |
| PRO1358 | lung tumor | universal normal control |
| PRO1358 | prostate tumor | universal normal control |
| PRO1286 | colon tumor | universal normal control |
| PRO1286 | lung tumor | universal normal control |
| PRO1286 | prostate tumor | universal normal control |
| PRO1286 | rectal tumor | universal normal control |
| PRO1294 | colon tumor | universal normal control |
| PRO1294 | lung tumor | universal normal control |
| PRO1294 | breast tumor | universal normal control |
| PRO1294 | rectal tumor | universal normal control |
| PRO1273 | lung tumor | universal normal control |
| PRO1273 | rectal tumor | universal normal control |
| PRO1279 | colon tumor | universal normal control |
| PRO1279 | lung tumor | universal normal control |
| PRO1195 | lung tumor | universal normal control |
| PRO1195 | breast tumor | universal normal control |
| PRO1271 | lung tumor | universal normal control |
| PR01271 | breast tumor | universal normal control |
| PRO1271 | liver tumor | universal normal control |
| PRO1338 | colon tumor | universal normal control |
| PRO1338 | lung tumor | universal normal control |
| PRO1338 | breast tumor | universal normal control |
| PRO1343 | colon tumor | universal normal control |
| PRO1343 | lung tumor | universal normal control |
| PRO1343 | breast tumor | universal normal control |
| PRO1343 | rectal tumor | universal normal control |
| PRO1434 | lung tumor | universal normal control |
| PRO1418 | lung tumor | universal normal control |
| PRO1418 | liver tumor | universal normal control |
| PRO1387 | colon tumor | universal normal control |
| PRO1387 | lung tumor | universal normal control |
| PRO1387 | prostate tumor | universal normal control |

Table continued

| Molecule | is overexpressed in: | as compared to: |
|---|---|---|
| PRO1387 | rectal tumor | universal normal control |
| PRO1384 | colon tumor | universal normal control |
| PRO1384 | lung tumor | universal normal control |
| PRO1565 | colon tumor | universal normal control |
| PRO1565 | lung tumor | universal normal control |
| PRO1565 | prostate tumor | universal normal control |
| PRO1474 | colon tumor | universal normal control |
| PRO1474 | lung tumor | universal normal control |
| PRO1474 | breast tumor | universal normal control |
| PR01474 | rectal tumor | universal normal control |
| PRO1917 | colon tumor | universal normal control |
| PRO1917 | lung tumor | universal normal control |
| PRO1917 | breast tumor | universal normal control |
| PRO1787 | colon tumor | universal normal control |
| PRO1787 | lung tumor | universal normal control |
| PRO1787 | breast tumor | universal normal control |
| PRO1556 | lung tumor | universal normal control |
| PRO1556 | breast tumor | universal normal control |
| PRO1561 | colon tumor | universal normal control |
| PRO1561 | lung tumor | universal normal control |
| PRO1561 | rectal tumor | universal normal control |
| PRO1693 | colon tumor | universal normal control |
| PRO1693 | lung tumor | universal normal control |
| PRO1693 | breast tumor | universal normal control |
| PRO1868 | lung tumor | universal normal control |
| PRO1868 | breast tumor | universal normal control |
| PRO1890 | colon tumor | universal normal control |
| PRO1890 | lung tumor | universal normal control |
| PRO1890 | breast tumor | universal normal control |
| PRO1890 | prostate tumor | universal normal control |
| PRO1887 | colon tumor | universal normal control |
| PRO1887 | breast tumor | universal normal control |
| PR04353 | lung tumor | universal normal control |
| PR04353 | breast tumor | universal normal control |
| PRO1801 | colon tumor | universal normal control |
| PRO1801 | lung tumor | universal normal control |
| PR04357 | lung tumor | universal normal control |
| PR04357 | breast tumor | universal normal control |
| PR04302 | colon tumor | universal normal control |
| PRO4302 | lung tumor | universal normal control |
| PR04302 | breast tumor | universal normal control |
| PR04302 | prostate tumor | universal normal control |
| PRO5990 | colon tumor | universal normal control |
| PRO5990 | lung tumor | universal normal control |
| PR05990 | breast tumor | universal normal control |

EXAMPLE 31: Identification of Receptor/Ligand Interactions

In this assay, various PRO polypeptides are tested for ability to bind to a panel of potential receptor or ligand molecules for the purpose of identifying receptor/ligand interactions. The identification of a ligand for a known receptor, a receptor for a known ligand or a novel receptor/ligand pair is useful for a variety of indications including, for example, targeting bioactive molecules (linked to the ligand or receptor) to a cell known to express the receptor or ligand, use of the receptor or ligand as a reagent to detect the presence of the ligand or receptor in a composition suspected of containing the same, wherein the composition may comprise cells suspected of expressing the ligand or receptor, modulating the growth of or another biological or immunological activity of a cell known to express or respond to the receptor or ligand, modulating the immune response of cells or toward cells that express the receptor or ligand, allowing the preparaion of agonists, antagonists and/or antibodies directed against the receptor or ligand which will modulate the growth of or a biological or immunological activity of a cell expressing the receptor or ligand, and various other indications which will be readily apparent to the ordinarily skilled artisan.

The assay is performed as follows. A PRO polypeptide of the present invention suspected of being a ligand for a receptor is expressed as a fusion protein containing the Fc domain of human IgG (an immunoadhesin). Receptor-ligand binding is detected by allowing interaction of the immunoadhesin polypeptide with cells (e.g. Cos cells) expressing candidate PRO polypeptide receptors and visualization of bound immunoadhesin with fluorescent reagents directed toward the Fc fusion domain and examination by microscope. Cells expressing candidate receptors are produced by transient transfection, in parallel, of defined subsets of a library of cDNA expression vectors encoding PRO polypeptides that may function as receptor molecules. Cells are then incubated for 1 hour in the presence of the PRO polypeptide immunoadhesin being tested for possible receptor binding. The cells are then washed and fixed with paraformaldehyde. The cells are then incubated with fluorescent conjugated antibody directed against the Fc portion of the PRO polypeptide immunoadhesin (e.g. FITC conjugated goat anti-human-Fc antibody). The cells are then washed again and examined by microscope. A positive interaction is judged by the presence of fluorescent labeling of cells transfected with cDNA encoding a particular PRO polypeptide receptor or pool of receptors and an absence of similar fluorescent labeling of similarly prepared cells that have been transfected with other cDNA or pools of cDNA. If a defined pool of cDNA expression vectors is judged to be positive for interaction with a PRO polypeptide immunoadhesin, the individual cDNA species that comprise the pool are tested individually (the pool is "broken down") to determine the specific cDNA that encodes a receptor able to interact with the PRO polypeptide immunoadhesin.

In another embodiment of this assay, an epitope-tagged potential ligand PRO polypeptide (e.g. 8 histidine "His" tag) is allowed to interact with a panel of potential receptor PRO polypeptide molecules that have been expressed as fusions with the Fc domain of human IgG (immunoadhesins). Following a 1 hour co-incubation with the epitope tagged PRO polypeptide, the candidate receptors are each immunoprecipitated with protein A beads and the beads are washed. Potential ligand interaction is determined by western blot analysis of the immunoprecipitated complexes with antibody directed towards the epitope tag. An interaction is judged to occur if a band of the anticipated molecular weight of the epitope tagged protein is observed in the western blot analysis with a candidate receptor, but is not observed to occur with the other members of the panel of potential receptors.

Using these assays, the following receptor/ligand interactions have been herein identified:

(1) PRO1801 binds to PRO1114 and PRO4978.
(2) PRO100 binds to PRO1114.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims

1. Isolated nucleic acid having at least 80% nucleic acid sequence identity to a nucleotide sequence that encodes an amino acid sequence selected from the group consisting of the amino acid sequence shown in Figure 22 (SEQ ID NO:22) and the amino acid sequences shown in evenly numbered Figures 2-550 (evenly numbered SEQ ID NOs: 2-550).

2. Isolated nucleic acid having at least 80% nucleic acid sequence identity to a nucleotide sequence selected from the group consisting of the nucleotide sequence shown in Figure 21 (SEQ ID NO:21) and the nucleotide sequences shown in oddly numbered Figures 1-549 (oddly numbered SEQ ID NOs:1-549).

3. Isolated nucleic acid having at least 80% nucleic acid sequence identity to a nucleotide sequence selected from the group consisting of the full-length coding sequence of the nucleotide sequence shown in Figure 21 (SEQ ID NO: 21) and the nucleotide sequences shown in oddly numbered Figures 1-549 (oddly numbered SEQ ID NOs:1-549).

4. Isolated nucleic acid having at least 80% nucleic acid sequence identity to the full-length coding sequence of the DNA deposited under any ATCC accession number shown in Table 7.

5. A vector comprising the nucleic acid of Claim 1.

6. The vector of Claim 5 operably linked to control sequences recognized by a host cell transformed with the vector.

7. A host cell comprising the vector of Claim 5.

8. The host cell of Claim 7, wherein said cell is a CHO cell.

9. The host cell of Claim 7, wherein said cell is an *E. coli.*

10. The host cell of Claim 7, wherein said cell is a yeast cell.

11. A process for producing a PRO polypeptides comprising culturing the host cell of Claim 7 under conditions suitable for expression of said PRO polypeptide and recovering said PRO polypeptide from the cell culture.

12. An isolated polypeptide having at least 80% amino acid sequence identity to an amino acid sequence selected from the group consisting of the amino acid sequence shown in Figure 22 (SEQ ID NO:22) and the amino acid sequences shown in evenly numbered Figures 2-550 (evenly numbered SEQ ID NOs:2-550).

13. An isolated polypeptide having at least 80% amino acid sequence identity to an amino acid sequence encoded by the full-length coding sequence of the DNA deposited under any ATCC accession number shown in Table 7.

14. A chimeric molecule comprising a polypeptide according to Claim 12 fused to a heterologous amino acid sequence.

15. The chimeric molecule of Claim 14, wherein said heterologous amino acid sequence is an epitope tag sequence.

16. The chimeric molecule of Claim 14, wherein said heterologous amino acid sequence is a Fc region of an immunoglobulin.

17. An antibody which specifically binds to a polypeptide according to Claim 12.

**18.** The antibody of Claim 17, wherein said antibody is a monoclonal antibody, a humanized antibody or a single-chain antibody.

**19.** Isolated nucleic acid having at least 80% nucleic acid sequence identity to:

(a) a nucleotide sequence encoding the polypeptide shown in Figure 22 (SEQ ID NO:22) or any one of evenly numbered Figures 2-550 (evenly numbered SEQ ID NOs:2-550), lacking its associated signal peptide;
(b) a nucleotide sequence encoding an extracellular domain of the polypeptide shown in Figure 22 (SEQ ID NO:22) or any one of evenly numbered Figures 2-550 (evenly numbered SEQ ID NOs:2-550), with its associated signal peptide; or
(c) a nucleotide sequence encoding an extracellular domain of the polypeptide shown in Figure 22 (SEQ ID NO:22) or any one of evenly numbered Figures 2-550 (evenly numbered SEQ ID NOs:2-550), lacking its associated signal peptide.

**20.** An isolated polypeptide having at least 80% amino acid sequence identity to:

(a) an amino acid sequence of the polypeptide shown in Figure 22 (SEQ ID NO:22) or any one of evenly numbered Figures 2-550 (evenly numbered SEQ ID NOs:2-550), lacking its associated signal peptide;
(b) an amino acid sequence of an extracellular domain of the polypeptide shown in Figure 22 (SEQ ID NO:22) or any one of evenly numbered Figures 2-550 (evenly numbered SEQ ID NOs:2-550), with its associated signal peptide; or
(c) an amino acid sequence of an extracellular domain of the polypeptide shown in Figure 22 (SEQ ID NO:22) or any one of evenly numbered Figures 2-550 (evenly numbered SEQ ID NOs:2-550), lacking its associated signal peptide.

**21.** A method of detecting a PRO1801 polypeptide in a sample suspected of containing a PRO1801 polypeptide, said method comprising contacting said sample with a PRO1114 or PRO4978 polypeptide and determining the formation of a PRO1801/PRO1114 or PRO1801/PRO4978 polypeptide conjugate in said sample, wherein the formation of said conjugate is indicative of the presence of a PRO1801 polypeptide in said sample.

**22.** The method according to Claim 21, wherein said sample comprises cells suspected of expressing said PRO1801 polypeptide.

**23.** The method according to Claim 21, wherein said PRO1114 or PR04978 polypeptide is labeled with a detectable label.

**24.** The method according to Claim 21, wherein said PRO1114 or PRO4978 polypeptide is attached to a solid support.

**25.** A method of detecting a PRO1114 or PR04978 polypeptide in a sample suspected of containing a PRO1114 or PR04978 polypeptide, said method comprising contacting said sample with a PRO1801 polypeptide and determining the formation of a PRO1801/PRO1114 or PRO1801/PRO4978 polypeptide conjugate in said sample, wherein the formation of said conjugate is indicative of the presence of a PRO1114 or PR04978 polypeptide in said sample.

**26.** The method according to Claim 25, wherein said sample comprises cells suspected of expressing said PRO1114 or PR04978 polypeptide.

**27.** The method according to Claim 25, wherein said PRO1801 polypeptide is labeled with a detectable label.

**28.** The method according to Claim 25, wherein said PRO1801 polypeptide is attached to a solid support.

**29.** A method of linking a bioactive molecule to a cell expressing a PRO1801 polypeptide, said method comprising contacting said cell with a PRO1114 or PR04978 polypeptide that is bound to said bioactive molecule and allowing said PRO1801 and said PRO1114 or PR04978 polypeptides to bind to one another, thereby linking said bioactive molecules to said cell.

**30.** The method according to Claim 29, wherein said bioactive molecule is a toxin, a radiolabel or an antibody.

**31.** The method according to Claim 29, wherein said bioactive molecule causes the death of said cell.

**32.** A method of linking a bioactive molecule to a cell expressing a PRO1114 or PR04978 polypeptide, said method comprising contacting said cell with a PRO1801 polypeptide that is bound to said bioactive molecule and allowing said PRO1801 and said PRO1114 or PR04978 polypeptides to bind to one another, thereby linking said bioactive molecules to said cell.

**33.** The method according to Claim 32, wherein said bioactive molecule is a toxin, a radiolabel or an antibody.

**34.** The method according to Claim 32, wherein said bioactive molecule causes the death of said cell.

**35.** A method of modulating at least one biological activity of a cell expressing a PRO1801 polypeptide, said method comprising contacting said cell with a PRO1114 or PRO4978 polypeptide or an anti-PRO1801 polypeptide antibody, whereby said PRO1114 or PR04978 polypeptide or anti-PRO1801 polypeptide antibody binds to said PRO1801 polypeptide, thereby modulating at least one biological activity of said cell.

**36.** The method according to Claim 35, wherein said cell is killed.

**37.** A method of modulating at least one biological activity of a cell expressing a PRO1114 or PR04978 polypeptide, said method comprising contacting said cell with a PRO1801 polypeptide or an anti-PRO1114 or anti-PRO4978 polypeptide antibody, whereby said PRO1801 polypeptide or anti-PRO1114 or anti-PRO4978 polypeptide antibody binds to said PRO1114 or PRO4978 polypeptide, thereby modulating at least one biological activity of said cell.

**38.** The method according to Claim 37, wherein said cell is killed.

**39.** A method of detecting a PRO1114 polypeptide in a sample suspected of containing a PRO1114 polypeptide, said method comprising contacting said sample with a PRO100 polypeptide and determining the formation of a PRO100/PRO1114 polypeptide conjugate in said sample, wherein the formation of said conjugate is indicative of the presence of a PRO1114 polypeptide in said sample.

**40.** The method according to Claim 39, wherein said sample comprises cells suspected of expressing said PRO1114 polypeptide.

**41.** The method according to Claim 39, wherein said PRO100 polypeptide is labeled with a detectable label.

**42.** The method according to Claim 39, wherein said PRO100 polypeptide is attached to a solid support.

**43.** A method of detecting a PRO100 polypeptide in a sample suspected of containing a PRO100 polypeptide, said method comprising contacting said sample with a PRO1114 polypeptide and determining the formation of a PRO100/PRO1114 polypeptide conjugate in said sample, wherein the formation of said conjugate is indicative of the presence of a PRO100 polypeptide in said sample.

**44.** The method according to Claim 43, wherein said sample comprises cells suspected of expressing said PRO100 polypeptide.

**45.** The method according to Claim 43, wherein said PRO1114 polypeptide is labeled with a detectable label.

**46.** The method according to Claim 43, wherein said PRO1114 polypeptide is attached to a solid support.

**47.** A method of linking a bioactive molecule to a cell expressing a PRO100 polypeptide, said method comprising contacting said cell with a PRO1114 polypeptide that is bound to said bioactive molecule and allowing said PRO100 and said PRO1114 polypeptides to bind to one another, thereby linking said bioactive molecules to said cell.

**48.** The method according to Claim 47, wherein said bioactive molecule is a toxin, a radiolabel or an antibody.

**49.** The method according to Claim 47, wherein said bioactive molecule causes the death of said cell.

**50.** A method of linking a bioactive molecule to a cell expressing a PRO1114 polypeptide, said method comprising contacting said cell with a PRO100 polypeptide that is bound to said bioactive molecule and allowing said PRO100 and said PRO1114 polypeptides to bind to one another, thereby linking said bioactive molecules to said cell.

**51.** The method according to Claim 50, wherein said bioactive molecule is a toxin, a radiolabel or an antibody.

**52.** The method according to Claim 50, wherein said bioactive molecule causes the death of said cell.

**53.** A method of modulating at least one biological activity of a cell expressing a PRO100 polypeptide, said method comprising contacting said cell with a PRO1114 polypeptide or an anti-PRO100 polypeptide antibody, whereby said PRO1114 polypeptide or anti-PRO100 polypeptide antibody binds to said PRO100 polypeptide, thereby modulating at least one biological activity of said cell.

**54.** The method according to Claim 53, wherein said cell is killed.

**55.** A method of modulating at least one biological activity of a cell expressing a PRO1114 polypeptide, said method comprising contacting said cell with a PRO100 polypeptide or an anti-PRO1114 polypeptide antibody, whereby said PRO100 polypeptide or anti-PRO1114 polypeptide antibody binds to said PRO1114 polypeptide, thereby modulating at least one biological activity of said cell.

**56.** The method according to Claim 55, wherein said cell is killed.

**57.** A method for stimulating the release of TNF- from human blood, said method comprising contacting said blood with a PRO195, PR0202, PR0215, PR0221, PRO217, PR0222, PRO198, PR0245, PRO172, PR0265, PR0266, PR0344, PR0337, PR0322, PRO1286, PRO1279, PRO1338 or PRO1343 polypeptide, wherein the release of TNF- from said blood is stimulated.

**58.** A method for modulating the uptake of glucose or FFA by skeletal muscle cells, said method comprising contacting said cells with a PRO182, PR0366, PRO198, PRO172 or PR0719 polypeptide, wherein the uptake of glucose or FFA by said cells is modulated.

**59.** A method for stimulating the proliferation or differentiation of chondrocyte cells, said method comprising contacting said cells with a PRO182, PRO366, PRO198, PRO1868, PR0202, PR0224, PRO172, PRO301 or PRO1312 polypeptide, wherein the proliferation or differentiation of said cells is stimulated.

**60.** A method for modulating the uptake of glucose or FFA by adipocyte cells, said method comprising contacting said cells with a PR0202, PRO211, PR0344 or PRO1338 polypeptide, wherein the uptake of glucose or FFA by said cells is modulated.

**61.** A method for stimulating the proliferation of or gene expression in pericyte cells, said method comprising contacting said cells with a PR0366 polypeptide, wherein the proliferation of or gene expression in said cells is stimulated.

**62.** A method for stimulating the release of proteoglycans from cartilage, said method comprising contacting said cartilage with a PRO216 polypeptide, wherein the release of proteoglycans from said cartilage is stimulated.

**63.** A method for stimulating the proliferation of inner ear utricular supporting cells, said method comprising contacting said cells with a PRO172 polypeptide, wherein the proliferation of said cells is stimulated.

**64.** A method for stimulating the proliferation of T-lymphocyte cells, said method comprising contacting said cells with a PR0344 polypeptide, wherein the proliferation of said cells is stimulated.

**65.** A method for stimulating the release of a cytokine from PBMC cells, said method comprising contacting said cells with a PR0526 or PRO1343 polypeptide, wherein the release of a cytokine from said cells is stimulated.

**66.** A method for inhibiting the binding of A-peptide to factor VIIA, said method comprising contacting a composition comprising said A-peptide and said factor VIIA with a PRO182 polypeptide, wherein the binding of said A-peptide to said factor VIIA is inhibited.

**67.** A method for inhibiting the differentiation of adipocyte cells, said method comprising contacting said cells with a PRO185 or PRO198 polypeptide, wherein the differentiation of said cells is inhibited.

**68.** A method for stimulating the proliferation of endothelial cells, said method comprising contacting said cells with a PR0222 polypeptide, wherein the proliferation of said cells is inhibited.

**69.** A method for detecting the presence of tumor in an mammal, said method comprising comparing the level of expression of any PRO polypeptide shown in Table 8 in (a) a test sample of cells taken from said mammal and (b) a control sample of normal cells of the same cell type, wherein a higher level of expression of said PRO polypeptide in the test sample as compared to the control sample is indicative of the presence of tumor in said mammal.

**70.** The method of Claim 69, wherein said tumor is lung tumor, colon tumor, breast tumor, prostate tumor, rectal tumor, cervical tumor or liver tumor.

**71.** An oligonucleotide probe derived from any of the nucleotide sequences shown in the accompanying figures.

# FIGURE 1

GTTACTCGGTGGTGGCGGAGTCTACGGAAGCCGTTTTCGCTTCACTTTTCCTGGCTGTAGAGC
GCTTTCCCCCTGGCGGGTGAGAGTGCAGAGACGAAGGTGCGAG**ATG**AGCACTATGTTCGCGGA
CACTCTCCTCATCGTTTTTATCTCTGTGTGCACGGCTCTGCTCGCAGAGGGCATAACCTGGGT
CCTGGTTTACAGGACAGACAAGTACAAGAGACTGAAGGCAGAAGTGGAAAAACAGAGTAAAAA
ATTGGAAAAGAAGAAGGAAACAATAACAGAGTCAGCTGGTCGACAACAGAAAAAGAAAATAGA
GAGACAAGAAGAGAAACTGAAGAATAACAACAGAGATCTATCAATGGTTCGAATGAAATCCAT
GTTTGCTATTGGCTTTTGTTTTACTGCCCTAATGGGAATGTTCAATTCCATATTTGATGGTAG
AGTGGTGGCAAAGCTTCCTTTTACCCCTCTTTCTTACATCCAAGGACTGTCTCATCGAAATCT
GCTGGGAGATGACACCACAGACTGTTCCTTCATTTTCCTGTATATTCTCTGTACTATGTCGAT
TCGACAGAACATTCAGAAGATTCTCGGCCTTGCCCCTTCACGAGCCGCCACCAAGCAGGCAGG
TGGATTTCTTGGCCCACCACCTCCTTCTGGGAAGTTCTCT**TGA**ACTCAAGAACTCTTTATTTT
CTATCATTCTTTCTAGACACACACACATCAGACTGGCAACTGTTTTGTAGCAAGAGCCATAGG
TAGCCTTACTACTTGGGCCTCTTTCTAGTTTTGAATTATTTCTAAGCCTTTTGGGTATGATTA
GAGTGAAAATGGCAGCCAGCAAACTTGATAGTGCTTTTGGTCCTAGATGATTTTTATCAAATA
AGTGGATTGATTAGTTAAGTTCAGGTAATGTTTATGTAATGAAAAACAAATAGCATCCTTCTT
GTTTCATTTACATAAGTATTTTCTGTGGGACCGACTCTCAAGGCACTGTGTATGCCCTGCAAG
TTGGCTGTCTATGAGCATTTAGAGATTTAGAAGAAAAATTTAGTTTGTTTAACCCTTGTAACT
GTTTGTTTTGTTGTTGTTTTTTTTTCAAGCCAAATACATGACATAAGATCAATAAAGAGGCCA
AATTTTTAGCTGTTTTATGTACAAGGAGAGATCTGTTTCATTTTGTTTTGCCGTATTTCTAGA
TATAAGTTTTAGCATGGGCCAGGAAGGACTAAAATAAAAGTTTTTAAGGTACAAAAAAAAAAA
AAAA

# FIGURE 2

MSTMFADTLLIVFISVCTALLAEGITWVLVYRTDKYKRLKAEVEKQSKKLEKKKETITESAGR
QQKKKIERQEEKLKNNNRDLSMVRMKSMFAIGFCFTALMGMFNSIFDGRVVAKLPFTPLSYIQ
GLSHRNLLGDDTTDCSFIFLYILCTMSIRQNIQKILGLAPSRAATKQAGGFLGPPPPSGKFS

**Important features:**

**Signal peptide:**

amino acids 1-22

**N-myristoylation sites.**

amino acids 103-109, 163-169

**cAMP- and cGMP-dependent protein kinase phosphorylation site.**

amino acids 53-57

# FIGURE 3

AGCCGGGGGCGGGTTTGAAGACGCGTCGTTGGGTTTTGGAGGCCGTGAAACAGCCGTTTGAGT
TTGGCTGCGGGTGGAGAACGTTTGTCAGGGGCCCGGCCAAGAAGGAGGCCCGCCTGTTACG**AT**
**G**GTGTCCATGAGTTTCAAGCGGAACCGCAGTGACCGGTTCTACAGCACCCGGTGCTGCGGCTG
TTGCCATGTCCGCACCGGGACGATCATCCTGGGGACCTGGTACATGGTAGTAAACCTATTGAT
GGCAATTTTGCTGACTGTGGAAGTGACTCATCCAAACTCCATGCCAGCTGTCAACATTCAGTA
TGAAGTCATCGGTAATTACTATTCGTCTGAGAGAATGGCTGATAATGCCTGTGTTCTTTTTGC
CGTCTCTGTTCTTATGTTTATAATCAGTTCAATGCTGGTTTATGGAGCAATTTCTTATCAAGT
GGGTTGGCTGATTCCATTCTTCTGTTACCGACTTTTTGACTTCGTCCTCAGTTGCCTGGTTGC
TATTAGTTCTCTCACCTATTTGCCAAGAATCAAAGAATATCTGGATCAACTACCTGATTTTCC
CTACAAAGATGACCTCCTGGCCTTGGACTCCAGCTGCCTCCTGTTCATTGTTCTTGTGTTCTT
TGCCTTATTCATCATTTTTAAGGCTTATCTAATTAACTGTGTTTGGAACTGCTATAAATACAT
CAACAACCGAAACGTGCCGGAGATTGCTGTGTACCCTGCCTTTGAAAGCACCTCCTCAGTACG
TTTTGCCAACCTATGAAATGGCCGTGAAAATGCCTGAAAAAGAACCACCACCTCCTTACTTAC
CTGCCTGAAGAAATTCTGCCTTTGACAATAAATCCTATACCAGCTTTTTGTTTGTTTATGTTA
CAGAATGCTGCAATTCAGGGCTCTTCAAACTTGTTTGATATAAAATATGTTGTCTTTTGTTTA
AGCATTTATTTTCAAACACTAAGGAGCTTTTTGACATCTGTTAAACGTCTTTTTGTTTTTTTG
TTAAGTCTTTTACATTTTAATAGTTTTTGAAGACAATCTAGGTTAAGCAAGAGCAAAGTGCCA
TTGTTTGCCTTTAATTGGGGGGTGGGAAGGGAAAGAGGGTACTTGCCACATAGTTTCCTTTTT
AACTGCACTTTCTTTATATAATCGTTTGCATTTTGTTACTTGCTACCCTGAGTACTTTCAGGA
AGACTGACTTAAATATTCGGGGTGAGTAAGTAGTTGGGTATAAGATCTGAACTTTTCATCTGC
AGAGGCAAGAAAAAATATTTGACATTGTGACTTGACTGTGGAAGATGATGGTTGCATGTTTCTA
GTTTGTATATGTTTCCATCTTTGTGATAAGATGATTTAATAAATCTCTTTAAATACTAAAAAA
AAAAAAAAA

# FIGURE 4

MVSMSFKRNRSDRFYSTRCCGCCHVRTGTIILGTWYMVVNLLMAILLTVEVTHPNSMPAVNIQ
YEVIGNYYSSERMADNACVLFAVSVLMFIISSMLVYGAISYQVGWLIPFFCYRLFDFVLSCLV
AISSLTYLPRIKEYLDQLPDFPYKDDLLALDSSCLLFIVLVFFALFIIFKAYLINCVWNCYKY
INNRNVPEIAVYPAFESTSSVRFANL

**Important features of the protein:**

**Transmembrane domain (Possible type II transmembrane protein):**

amino acids 30-49, 81-100, 111-131, 158-175

**N-glycosylation site.**

amino acids 9-13

**Tyrosine kinase phosphorylation sites.**

amino acids 8-16, 193-202

**N-myristoylation site.**

amino acids 68-74

# FIGURE 5

CCCGCTGGCCCGTCAGTGCTCTCCCCGTCGTTTGCCCTCTCCAGTTCCCCCAGTGCCTGCCCT
ACGCACCCCG**ATG**GCGGAGCTGCGGCCTAGCGGCGCCCCCGGCCCCACCGCGCCCCCGGCCCC
TGGCCCGACTGCCCCCCCGGCCTTCGCTTCGCTCTTTCCCCCGGGACTGCACGCCATCTACGG
AGAGTGCCGCCGCCTTTACCCTGACCAGCCGAACCCGCTCCAGGTTACCGCTATCGTCAAGTA
CTGGTTGGGTGGCCCAGACCCCTTGGACTATGTTAGCATGTACAGGAATGTGGGGAGCCCTTC
TGCTAACATCCCCGAGCACTGGCACTACATCAGCTTCGGCCTGAGTGATCTCTATGGTGACAA
CAGAGTCCATGAGTTTACAGGAACAGATGGACCTAGTGGTTTTGGCTTTGAGTTGACCTTTCG
TCTGAAGAGAGAAACTGGGGAGTCTGCCCCACCAACATGGCCCGCAGAGTTAATGCAGGGCTT
GGCACGATACGTGTTCCAGTCAGAGAACACCTTCTGCAGTGGGGACCATGTGTCCTGGCACAG
CCCTTTGGATAACAGTGAGTCAAGAATTCAGCACATGCTGCTGACAGAGGACCCACAGATGCA
GCCCGTGCAGACACCCTTTGGGGTAGTTACCTTCCTCCAGATCGTTGGTGTCTGCACTGAAGA
GCTACACTCAGCCCAGCAGTGGAACGGGCAGGGCATCCTGGAGCTGCTGCGGACAGTGCCTAT
TGCTGGCGGCCCCTGGCTGATAACTGACATGCGGAGGGGAGAGACCATATTTGAGATCGATCC
ACACCTGCAAGAGAGAGTTGACAAAGGCATCGAGACAGATGGCTCCAACCTGAGTGGTGTCAG
TGCCAAGTGTGCCTGGGATGACCTGAGCCGGCCCCCCGAGGATGACGAGGACAGCCGGAGCAT
CTGCATCGGCACACAGCCCCGGCGACTCTCTGGCAAAGACACAGAGCAGATCCGGGAGACCCT
GAGGAGAGGACTCGAGATCAACAGCAAACCTGTCCTTCCACCAATCAACCCTCAGCGGCAGAA
TGGCCTCGCCCACGACCGGGCCCCGAGCCGCAAAGACAGCCTGGAAAGTGACAGCTCCACGGC
CATCATTCCCCATGAGCTGATTCGCACGCGGCAGCTTGAGAGCGTACATCTGAAATTCAACCA
GGAGTCCGGAGCCCTCATTCCTCTCTGCCTAAGGGGCAGGCTCCTGCATGGACGGCACTTTAC
ATATAAAAGTATCACAGGTGACATGGCCATCACGTTTGTCTCCACGGGAGTGGAAGGCGCCTT
TGCCACTGAGGAGCATCCTTACGCGGCTCATGGACCCTGGTTACAACTC**TGA**ACCTATCCTCG
GAGCTCTGCCCTCCCGTCCTGGAACGTCTTTCTGCCCTGAGGAGAGGGTAGTCAGCATCTCCA
ATTTTCAGCAGCTCAAGAACCTTGGCCCCCACAGGACTTCGCAGATGTCACATTGCCCCTCAG
TCCCCTGAATGCCCTTCGGACCCAACCCCAATTCCCCAAGCCCCTGACCCCCTAGCTGCCGGG
GTTCCCACTCCCAGTGCCACAACCCCCTCACCTCCCCTGGCAGCCCCTCAGCGAGCCTGAGGC
CCAGCACCCGCTGGCTCCCCAGCACATGGTCCCCTCCCATGGGCTGTTGCCCAGGGAACCGGG
GCGCGGTGGGAACGAGCTGCTGGCCTCGGCATGTTTCAATAAAGTTGCTGTGCTGGGAG

# FIGURE 6

MAELRPSGAPGPTAPPAPGPTAPPAFASLFPPGLHAIYGECRRLYPDQPNPLQVTAIVKYWLG
GPDPLDYVSMYRNVGSPSANIPEHWHYISFGLSDLYGDNRVHEFTGTDGPSGFGFELTFRLKR
ETGESAPPTWPAELMQGLARYVFQSENTFCSGDHVSWHSPLDNSESRIQHMLLTEDPQMQPVQ
TPFGVVTFLQIVGVCTEELHSAQQWNGQGILELLRTVPIAGGPWLITDMRRGETIFEIDPHLQ
ERVDKGIETDGSNLSGVSAKCAWDDLSRPPEDDEDSRSICIGTQPRRLSGKDTEQIRETLRRG
LEINSKPVLPPINPQRQNGLAHDRAPSRKDSLESDSSTAIIPHELIRTRQLESVHLKFNQESG
ALIPLCLRGRLLHGRHFTYKSITGDMAITFVSTGVEGAFATEEHPYAAHGPWLQL

**Important features:**

**N-glycosylation site.**

amino acids 265-268

# FIGURE 7

CGCGAATGAAGTTTGCATTTTCCTCTGTTCTTGAGCCCAGCTTCTTCTCGTCTCCCACCCCAG
CTTCCCGGCATTGGAAGAAGGGACCGTCCTCTTCCTTGTCTTGGCCACCCAAATCCTGGTATC
GAAAGGGTTGAACGGACCGGAAGTGTGCAGCAGCGACGGGTCCCCAGCTAATCGACGCCGGAA
GTAGCAATTACTAGACAAGCATTCCGCCGCCGGCTTCGCT**ATG**GCGGCAATTCCCCCAGATTC
CTGGCAGCCACCCAACGTTTACTTGGAGACCAGCATGGGAATCATTGTGCTGGAGCTGTACTG
GAAGCATGCTCCAAAGACCTGTAAGAACTTTGCTGAGTTGGCTCGTCGAGGTTACTACAATGG
CACAAAATTCCACAGAATTATCAAAGACTTCATGATCCAAGGAGGTGACCCAACAGGGACAGG
TCGAGGTGGTGCATCTATCTATGGCAAACAATTTGAAGATGAACTTCATCCAGACTTGAAATT
CACGGGGGCTGGAATTCTCGCAATGGCCAATGCGGGGCCAGATACCAATGGCAGCCAGTTCTT
TGTGACCCTCGCCCCCACCCAGTGGCTTGACGGCAAACACACCATTTTTGGCCGAGTGTGTCA
GGGCATAGGAATGGTGAATCGCGTGGGAATGGTAGAAACAAACTCCCAGGACCGCCCTGTGGA
CGACGTGAAGATCATTAAGGCATACCCTTCTGGG**TAG**ACTTGCTACCCTCTTGAGCAGCTCTT
CTGAGATGGCCCCAGTGAACCAGCTTCTAGATGACATAGAATGACATGTAATGCTAAATTTCA
TTTTGGCTTTGCAAGTCATGAAGCTTAGGAGGCCTGGCATCTTGGGTGAGTTAGAGATGGAAG
TACATTTTAATAGGATGCTTCTTTTCTCTTCCCCCAGTGCCTAGGTTGCCAGAGCATTTGCAC
AAATGCCCCTGTTTATCAATAGGTGACTACTTACTACACATGAACCATAATGCTGCTTCTTGT
GCATGTCTGCTCTGATATACGTCGAACAATGTAGCAGCCACTGTCATTTCTCAGTGGTTTTGC
CTAACCAAACTTCTTCCTAAGGAGATTTATATTCTGGCCTACACAGCAGTCCTTGATGGCTGA
CAGCCACAGAATTCCAAACCAAGTAGTGTCTGTCAGCCCTCTTAACTCTGTGCACGCCCTATT
TCAGTCTTTTACATTTGTTCTTCTAGGGAATGTATGCATCTCTATATATATTTTCCCTCTCAA
AACCAGAACATCAACAGTGCTGTTTCTGACACTTCAGACATCCCACGCAAAGCCACATTGAAT
TTTTGCCAAATGAAAAACACATCCAACAATCAAGTTTCTAAGAAGGTGTCAAGTGGGGAATAA
TAATAATGTATAATAATCAAGAAATTAGTTTATTAAAAGGAAGCAGAAGCATTGACCATTTTT
TCCCAGAGAAGAGGAGAAATCTGTAGTGAGCAAAGGACAGACCATGAATCCTCCTTGAGAAGT
AGTACTCTCAGAAAGGAGAAGCGCCACTCAAGTTCTTTTAACCCAAGACTTTAGAGAAATTAG
GTCCAAGATTTTTATATGTTCAGTTGTTTATGTATAAAAATAACTTTCTGGATTTTGTGGGGA
GGAGCAGGAGAGGAAGGAAGTTAATACCTATGTAATACATAGAAACTTCCACAATAAAATGCC
ATTGATGGTTAAAAAAAAAAAAAAAAAAA

# FIGURE 8

MAAIPPDSWQPPNVYLETSMGIIVLELYWKHAPKTCKNFAELARRGYYNGTKFHRIIKDFMIQ
GGDPTGTGRGGASIYGKQFEDELHPDLKFTGAGILAMANAGPDTNGSQFFVTLAPTQWLDGKH
TIFGRVCQGIGMVNRVGMVETNSQDRPVDDVKIIKAYPSG

**Important features:**

**N-glycosylation sites:**

amino acids 49-52, 108-111

**N-myristoylation sites:**

amino acids 64-69, 69-74, 143-148

**Cyclophilin-type peptidyl-prolyl cis-trans isomerase signature:**

amino acids 48-65

# FIGURE 9

CGGACGCGTGGGCGCGCGCGAGCGCAGCGGTGGGAGGCGGCGACCAGCCGGTTGAGGCCCCAG
GCTTGGCCTCACCACA**ATG**TGGCACGAGGCTCGGAAGCATGAGCGGAAGCTTCGAGGCATGAT
GGTCGACTACAAGAAGAGGGCGGAGCGGAGACGGGAGTATTATGAAAAGATCAAGAAGGACCC
AGCCCAGTTCCTGCAGGTACATGGCCGAGCTTGCAAGGTGCACCTGGATTCTGCAGTCGCCCT
GGCCGCTGAGAGCCCTGTTAATATGATGCCCTGGCAGGGGGACACCAACAACATGATTGACCG
ATTCGATGTCCGTGCCCACCTGGACCACATCCCCGACTACACCCCCCCTCTGCTCACCACCAT
CTCCCCAGAACAGGAGTCGGACGAACGGAAGTGTAACTACGAGCGCTACAGAGGCCTGGTGCA
GAACGACTTTGCCGGCATCTCAGAGGAGCAGTGCCTGTACCAGATCTACATTGATGAGTTGTA
CGGAGGCCTCCAGAGACCCAGCGAAGATGAGAAGAAGAAGCTGGCAGAGAAGAAGGCTTCCAT
CGGTTATACCTACGAGGACAGCACGGTGGCCGAGGTAGAGAAGGCGGCAGAAAAGCCAGAGGA
GGAGGAGTCAGCGGCCGAGGAGGAGAGCAACTCGGACGAAGATGAGGTCATCCCCGACATCGA
CGTGGAGGTGGACGTGGATGAATTGAACCAGGAGCAGGTGGCAGATCTCAACAAACAGGCCAC
GACTTATGGCATGGCCGACGGTGACTTCGTCAGGATGCTCCGGAAAGACAAGGAGGAGGCAGA
GGCCATCAAGCATGCCAAGGCTCTTGAGGAGGAGAAGGCCATGTACTCGGGACGCCGCTCTCG
ACGCCAGCGGAGAGAGTTTCGGGAGAAGCGGCTGAGGGGTCGCAAGATCAGCCCACCCAGCTA
TGCCCGCCGAGACAGCCCCACCTATGACCCCTATAAGCGGTCACCCTCGGAGTCCAGCTCAGA
GTCCCGCTCCCGCTCCCGCTCCCCGACCCCGGGCCGCGAGGAGAAGATCACGTTCATCACCAG
TTTTGGGGGCAGCGATGAGGAGGCAGCCGCAGCCGCTGCTGCCGCAGCAGCATCAGGAGTCAC
CACAGGGAAGCCCCCCGCACCTCCCCAGCCTGGCGGCCCCGCCCCGGGACGTAATGCCAGCGC
CCGCCGCCGCTCCTCCTCCTCCTCCTCCTCCTCTTCTGCCTCGAGGACCTCCAGCTCCCGCTC
CAGCTCTCGCTCCAGCTCCCGCTCTCGCCGTGGTGGGGGCTACTACCGTTCCGGCCGCCACGC
CCGCTCCCGGTCCCGCTCCTGGTCCCGCTCCCGCTCCCGCTCCCGGCGCTATTCCCGGTCCCG
TAGCCGTGGCCGGCGGCACTCAGGTGGGGGCTCCCGAGACGGACACCGGTACTCCCGCTCGCC
CGCCCGGCGTGGTGGTTACGGGCCCCGGCGCAGAAGCAGGAGCCGCTCCCACTCAGGGGACCG
CTACAGGCGGGGCGGCCGGGGCCTCAGGCACCACAGCAGTAGCCGCAGCCGCAGCAGCTGGTC
CCTCAGCCCGTCCCGCAGTCGCAGCCTGACTCGCAGCCGCAGCCATAGCCCCAGCCCCAGCCA
GAGCCGCAGCCGCAGCCGCAGCCGCAGCCAGAGCCCCTCGCCATCACCCGCAAGAGAGAAGCT
GACCAGGCCGGCCGCGTCCCCTGCTGTGGGCGAGAAGCTGAAAAAGACCGAACCTGCCGCTGG
TAAAGAGACAGGAGCTGCCAAAGTCACCCAAGCTGACGCCTCAGGAGAAGCTGAAACTGAGGA
TGCAGAAGGCGCTGAACAGGCAGTTCAAGGCGGA**TAA**GAAGGCGGCACAAGAAAAGATGATCC
AGCAGGAGCATGAGCGGCAGGAGCGGGAAGACGAGCTTCGAGCCATGGCCCGCAAGATCCGCA
TGAAGGAGCGGGAACGCCGAGAGAAGGAGAGAGAAGAGTGGGAACGCCAGTACAGCCGGCAGA
GCCGCTCACCCTCCCCCCGATACAGTCGAGAATACAGCTCTTCTCGAAGGCGCTCAAGGTCCC
GATCCCGAAGCCCCCATTACCGACATTAGGCAGAAGAGTGGGGGGTGGGGAGGACAAGGGGGT
GGGTAAGGGGCTCAAGCTGTGATGCTGCTGGTTTTATCTCTAGTGAAATAAAGTCAAAAGTTA
TTTAATTCCGTCAAAAAAAAAAAAAAAAAAAAAAAAAAAAAAAAAAAAAAAAAAAAAAAA
AAAAAAAA

# FIGURE 10

MWHEARKHERKLRGMMVDYKKRAERRREYYEKIKKDPAQFLQVHGRACKVHLDSAVALAAESP
VNMMPWQGDTNNMIDRFDVRAHLDHIPDYTPPLLTTISPEQESDERKCNYERYRGLVQNDFAG
ISEEQCLYQIYIDELYGGLQRPSEDEKKKLAEKKASIGYTYEDSTVAEVEKAAEKPEEEESAA
EEESNSDEDEVIPDIDVEVDVDELNQEQVADLNKQATTYGMADGDFVRMLRKDKEEAEAIKHA
KALEEEKAMYSGRRSRRQRREFREKRLRGRKISPPSYARRDSPTYDPYKRSPSESSSESRSRS
RSPTPGREEKITFITSFGGSDEEAAAAAAAAAASGVTTGKPPAPPQPGGPAPGRNASARRRSS
SSSSSSSASRTSSSRSSSRSSSRSRRGGGYYRSGRHARSRSRSWSRSRSRSRRYSRSRSRGRR
HSGGGSRDGHRYSRSPARRGGYGPRRRSRSRSHSGDRYRRGGRGLRHHSSSRSRSSWSLSPSR
SRSLTRSRSHSPSPSQSRSRSRSRSQSPSPSPAREKLTRPAASPAVGEKLKKTEPAAGKETGA
AKVTQADASGEAETEDAEGAEQAVQGG

**Important features:**

**N-glycosylation site:**

amino acids 370-373

**Glycosaminoglycan attachment site:**

amino acids 443-446

**cAMP- and cGMP-dependent protein kinase phosphorylation site:**

amino acids 159-162, 282-285, 291-294, 374-377, 375-378, 430-433, 440-443, 466-469

**Casein kinase II phosphorylation site:**

amino acids 149-152, 166-169, 171-174, 187-190, 193-196, 195-198, 303-306, 307-310, 335-338, 571-574

**N-myristoylation sites:**

amino acids 118-123, 229-234, 350-355, 446-451, 586-591

**Amidation sites:**

amino acids 263-266, 280-283, 438-441

# FIGURE 11

GGTAGGCGCGCCCAGACCTGAGACGGGTTGGGACTGGGCTGCGTCACGCGCGGCTCTAAGCG
CCCGGGGCCCCGCCCAGTGGCCGGCACAGCCAATCGCAGCGCGGGAAGGCGGTGGGGGCGGGG
AAGGCCGCCTGGAAACTTAAATCCCGAGGCGGGCGAACCTGCACCAGACCGCGGACGTCTGTA
ATCTCAGAGGCTTGTTTGCTGAGGGTGCCTGCGCAGCTGCGACGGCTGCTGGTTTTGAAAC**AT**
**G**AATCTTTCGCTCGTCCTGGCTGCCTTTTGCTTGGGAATAGCCTCCGCTGTTCCAAAATTTGA
CCAAAATTTGGATACAAAGTGGTACCAGTGGAAGGCAACACACAGAAGATTATATGGCGCGAA
TGAAGAAGGATGGAGGAGAGCAGTGTGGGAAAAGAATATGAAAATGATTGAACTGCACAATGG
GGAATACAGCCAAGGGAAACATGGCTTCACAATGGCCATGAATGCTTTTGGTGACATGACCAA
TGAAGAATTCAGGCAGATGATGGGTTGCTTTCGAAACCAGAAATTCAGGAAGGGGAAAGTGTT
CCGTGAGCCTCTGTTTCTTGATCTTCCCAAATCTGTGGATTGGAGAAAGAAAGGCTACGTGAC
GCCAGTGAAGAATCAGAAACAGTGTGGTTCTTGTTGGGCTTTTAGTGCGACTGGTGCTCTTGA
AGGACAGATGTTCCGGAAAACTGGGAAACTTGTCTCACTGAGCGAGCAGAATCTGGTGGACTG
TTCGCGTCCTCAAGGCAATCAGGGCTGCAATGGTGGCTTCATGGCTAGGGCCTTCCAGTATGT
CAAGGAGAACGGAGGCCTGGACTCTGAGGAATCCTATCCATATGTAGCAGTGGATGAAATCTG
TAAGTACAGACCTGAGAATTCTGTTGCTAATGACACTGGCTTCACAGTGGTCGCACCTGGAAA
GGAGAAGGCCCTGATGAAAGCAGTCGCAACTGTGGGGCCCATCTCCGTTGCTATGGATGCAGG
CCATTCGTCCTTCCAGTTCTACAAATCAGGCATTTATTTTGAACCAGACTGCAGCAGCAAAAA
CCTGGATCATGGTGTTCTGGTGGTTGGCTACGGCTTTGAAGGAGCAAATTCGAATAACAGCAA
GTATTGGCTCGTCAAAAACAGCTGGGGTCCAGAATGGGGCTCGAATGGCTATGTAAAAATAGC
CAAAGACAAGAACAACCACTGTGGAATCGCCACAGCAGCCAGCTACCCCAATGTG**TGA**GCTGA
TGGATGGTGAGGAGGAAGGACTTAAGGACAGCATGTCTGGGGAAATTTTATCTTGAAACTGAC
CAAACGCTTATTGTGTAAGATAAACCAGTTGAATCATGGAGGATCCAAGTTGAGATTTTAATT
CTGTGACATTTTTACAAGGGTAAAATGTTACCACTACTTTAATTATTGTTATACACAGCTTTA
TGATATCAAAGACTCATTGCTTAATTCTAAGACTTTTGAATTTTCATTTTTTAAAAAGATGTA
CAAAACAGTTTGAAATAAATTTTAATTCGTATATA

# FIGURE 12

MNLSLVLAAFCLGIASAVPKFDQNLDTKWYQWKATHRRLYGANEEGWRRAVWEKNMKMIELHN
GEYSQGKHGFTMAMNAFGDMTNEEFRQMMGCFRNQKFRKGKVFREPLFLDLPKSVDWRKKGYV
TPVKNQKQCGSCWAFSATGALEGQMFRKTGKLVSLSEQNLVDCSRPQGNQGCNGGFMARAFQY
VKENGGLDSEESYPYVAVDEICKYRPENSVANDTGFTVVAPGKEKALMKAVATVGPISVAMDA
GHSSFQFYKSGIYFEPDCSSKNLDHGVLVVGYGFEGANSNNSKYWLVKNSWGPEWGSNGYVKI
AKDKNNHCGIATAASYPNV

**Important features:**

**Signal sequence**

amino acids 1-17

**N-glycosylation sites.**

amino acids 2-6, 221-225, 292-296

**N-myristoylation sites.**

amino acids 13-19, 93-99, 136-142, 145-151, 174-180, 177-183, 180-186, 194-200, 288-294, 324-330

**Eukaryotic thiol (cysteine) proteases cysteine active site.**

amino acids 132-144

**Eukaryotic thiol (cysteine) proteases histidine active site.**

amino acids 275-286

# FIGURE 13

GGCGGCGTCATGTGATCCGCTTCCCTGCTCCTTTAAGCGTCCACAGGCGGCGGAGCGGCCACA
ATCACAGCTCCGGGCATTGGGGGAACCCGAGCCGGCTGCGCCGGGGGAATCCGTGCGGGCGCC
TTCCGTCCCGGTCCCATCCTCGCCGCGCTCCAGCACCTCTGAAGTTTTGCAGCGCCCAGAAAG
GAGGCGAGGAAGGAGGGAGTGTGTGAGAGGAGGGAGCAAAAAGCTCACCCTAAAACATTTATT
TCAAGGAGAAAAGAAAAAGGGGGGGCGCAAAA**ATG**GCTGGGGCAATTATAGAAAACATGAGCA
CCAAGAAGCTGTGCATTGTTGGTGGGATTCTGCTCGTGTTCCAAATCATCGCCTTTCTGGTGG
GAGGCTTGATTGCTCCAGGGCCCACAACGGCAGTGTCCTACATGTCGGTGAAATGTGTGGATG
CCCGTAAGAACCATCACAAGACAAAATGGTTCGTGCCTTGGGGACCCAATCATTGTGACAAGA
TCCGAGACATTGAAGAGGCAATTCCAAGGGAAATTGAAGCCAATGACATCGTGTTTTCTGTTC
ACATTCCCCTCCCCCACATGGAGATGAGTCCTTGGTTCCAATTCATGCTGTTTATCCTGCAGC
TGGACATTGCCTTCAAGCTAAACAACCAAATCAGAGAAAATGCAGAAGTCTCCATGGACGTTT
CCCTGGCTTACCGTGATGACGCATTTGCTGAGTGGACTGAAATGGCCCATGAAAGAGTACCAC
GGAAACTCAAATGCACCTTCACATCTCCCAAGACTCCAGAGCATGAGGGCCGTTACTATGAAT
GTGATGTCCTTCCTTTCATGGAAATTGGGTCTGTGGCCCATAAGTTTTACCTTTTAAACATCC
GGCTGCCTGTGAATGAGAAGAAGAAAATCAATGTGGGAATTGGGGAGATAAAGGATATCCGGT
TGGTGGGGATCCACCAAAATGGAGGCTTCACCAAGGTGTGGTTTGCCATGAAGACCTTCCTTA
CGCCCAGCATCTTCATCATTATGGTGTGGTATTGGAGGAGGATCACCATGATGTCCCGACCCC
CAGTGCTTCTGGAAAAAGTCATCTTTGCCCTTGGGATTTCCATGACCTTTATCAATATCCCAG
TGGAATGGTTTTCCATCGGGTTTGACTGGACCTGGATGCTGCTGTTTGGTGACATCCGACAGG
GCATCTTCTATGCGATGCTTCTGTCCTTCTGGATCATCTTCTGTGGCGAGCACATGATGGATC
AGCACGAGCGGAACCACATCGCAGGGTATTGGAAGCAAGTCGGACCCATTGCCGTTGGCTCCT
TCTGCCTCTTCATATTTGACATGTGTGAGAGAGGGGTACAACTCACGAATCCCTTCTACAGTA
TCTGGACTACAGACATTGGAACAGAGCTGGCCATGGCCTTCATCATCGTGGCTGGAATCTGCC
TCTGCCTCTACTTCCTGTTTCTATGCTTCATGGTATTTCAGGTGTTTCGGAACATCAGTGGGA
AGCAGTCCAGCCTGCCAGCTATGAGCAAAGTCCGGCGGCTACACTATGAGGGGCTAATTTTTA
GGTTCAAGTTCCTCATGCTTATCACCTTGGCCTGCGCTGCCATGACTGTCATCTTCTTCATCG
TTAGTCAGGTAACGGAAGGCCATTGGAAATGGGGCGGCGTCACAGTCCAAGTGAACAGTGCCT
TTTTCACAGGCATCTATGGGATGTGGAATCTGTATGTCTTTGCTCTGATGTTCTTGTATGCAC
CATCCCATAAAAACTATGGAGAAGACCAGTCCAATGGCGATCTGGGTGTCCATAGTGGGGAAG
AACTCCAGCTCACCACCACTATCACCCATGTGGACGGACCCACTGAGATCTACAAGTTGACCC
GCAAGGAGGCCCAGGAG**TAG**GAGGCTGCAGCGCCCGGCTGGGACGGTCTCTCCATACCCCAGC
CCCTCTAACTAGAGTGGGGAGCATGCCAGAGAGAGCTCAATGTACAAATGAATGCCTCATGGC
TCTTAGCTGTGGTTTCTTGGACCAGCGGCATGGACATTTGTCAGTTTGCCTTCTGACGGTAGC
TTTTGGAGGAAGATTCCTGCAGCCACTAATGCATTGTGTATGATAACAAAAACTCTGGTATGA
CACATTTTCTGTGATCATTGTTAATTAGTGACATAGTAACATCTGTAGCAGCTGGTTAGTAAA
CCTCATGTGGGGGTGGGGTGGGGGTGTATTCCTTGGGGGATGGTTTGGGCCGAATGGGGAGTG
GAATATTTGACATTTTTCCTGTTTTAAATTCTAGGATAGATTTTAACATCCTTTGCGGTCCCA
GTCCAAGGTAGGCTGGTGTCATAGTCTTCTCACTCCTAATCCATGACCACTGTTTTTTTCCTA
TTTATATCACCAGGTAGCCTACTGAGTTAATATTTAAGTTGTCAATAGATAAGTGTCCCTGTT
TTGTGGCATAATATAACTGAATTTCATGAGAAGATTTATTCCACCAGGGGTATTTCAGCTTTG
AAACCAAATCTGTGTATCTAATACTAACCAATCTGTTGGATGTGGATTTTAAAAAATGTTTGC
TAAACTACCCAAGTAAGATTTACTGTATTAAATGGCCTTCGGGTCTGAAAAGCTTTTTTAACC
TCTTGCTTAAAATGCGTTTTATTTTGATAAGATACTTCAAATAGCCTCCAAAAGTGTAGATCC
AATCACTTAAATAAACCTGTATGTATATGCAAAAAAAAAAAAAAAAAAAAAAA

# FIGURE 14

MAGAIIENMSTKKLCIVGGILLVFQIIAFLVGGLIAPGPTTAVSYMSVKCVDARKNHHKTKWF
VPWGPNHCDKIRDIEEAIPREIEANDIVFSVHIPLPHMEMSPWFQFMLFILQLDIAFKLNNQI
RENAEVSMDVSLAYRDDAFAEWTEMAHERVPRKLKCTFTSPKTPEHEGRYYECDVLPFMEIGS
VAHKFYLLNIRLPVNEKKKINVGIGEIKDIRLVGIHQNGGFTKVWFAMKTFLTPSIFIIMVWY
WRRITMMSRPPVLLEKVIFALGISMTFINIPVEWFSIGFDWTWMLLFGDIRQGIFYAMLLSFW
IIFCGEHMMDQHERNHIAGYWKQVGPIAVGSFCLFIFDMCERGVQLTNPFYSIWTTDIGTELA
MAFIIVAGICLCLYFLFLCFMVFQVFRNISGKQSSLPAMSKVRRLHYEGLIFRFKFLMLITLA
CAAMTVIFFIVSQVTEGHWKWGGVTVQVNSAFFTGIYGMWNLYVFALMFLYAPSHKNYGEDQS
NGDLGVHSGEELQLTTTITHVDGPTEIYKLTRKEAQE

**Important features of the protein:**

**Signal peptide:**

amino acids 1-42

**Transmembrane domains:**

amino acids 239-253, 269-284, 302-318, 338-352, 377-399, 434-452, 471-488

**N-glycosylation sites.**

amino acids 8-12, 406-410

**cAMP- and cGMP-dependent protein kinase phosphorylation site.**

amino acids 254-258

**N-myristoylation sites.**

amino acids 223-229, 274-280, 305-311, 358-364, 374-380, 386-392, 509-515

# FIGURE 15

GTGAGGGGAACAGCTGATCCGTCTGTTGGGAGGACAGATATCTCAAGGCCAGG**ATG**GAAGAAT
CACCACTAAGCCGGGCACCATCCCGTGGTGGAGTCAACTTTCTCAATGTAGCCCGGACCTACA
TCCCCAACACCAAGGTGGAATGTCACTACACCCTTCCCCCAGGCACCATGCCCAGTGCCAGTG
ACTGGATTGGCATCTTCAAGGTGGAGGCTGCCTGTGTTCGGGATTACCACACATTTGTGTGGT
CTTCCGTGCCTGAAAGTACAACTGATGGTTCCCCCATTCACACCAGTGTCCAGTTCCAAGCCA
GCTACCTGCCCAAACCAGGAGCTCAGCTCTACCAGTTCCGATATGTGAACCGCCAGGGCCAGG
TGTGTGGGCAGAGCCCCCCCTTTCCAGTTCCGAGAGCCAAGGCCCATGGATGAACTGGTGACCC
TGGAGGAGGCTGATGGGGGCTCTGACATCCTGCTGGTTGTCCCCAAGGCAACTGTGTTACAGA
ACCAGCTCGATGAGAGCCAGCAAGAACGGAATGACCTGATGCAGCTGAAGCTACAGCTGGAGG
GACAGGTGACAGAGCTGAGGAGCCGAGTGCAGGAGCTCGAGAGGGCTCTGGCAACTGCCAGGC
AGGAGCACACGGAGCTGATGGAACAGTACAAGGGGATTTCCCGGTCCCATGGGGAGATCACAG
AAGAGAGGGACATCCTGAGCCGGCAACAGGGAGACCATGTGGCACGCATCCTGGAGCTAGAGG
ATGACATCCAGACCATCAGTGAGAAAGTGCTGACGAAGGAAGTGGAGCTGGACAGGCTTAGAG
ACACAGTGAAGGCCCTGACTCGGGAACAAGAGAAGCTCCTTGGGCAACTGAAAGAAGTACAAG
CAGACAAGGAGCAAAGTGAGGCTGAGCTCCAAGTGGCACAACAGGAGAACCATCACTTAAATT
TGGACCTGAAGGAGGCGAAGAGCTGGCAAGAGGAGCAGAGTGCTCAGGCTCAGCGACTGAAAG
ACAAGGTGGCCCAGATGAAGGACACCCTAGGCCAGGCCCAGCAGCGGGTGGCCGAGCTGGAGC
CCTTGAAGGAGCAGCTTCGAGGGGCCCAGGAGCTTGCAGCCTCAAGCCAGCAGAAAGCCACCC
TTCTTGGGGAGGAGTTGGCCAGTGCAGCAGCAGCCAGGGACCGCACCATAGCCGAACTACACC
GCAGCCGCCTGGAAGTGGCTGAAGTTAACGGCAGGCTGGCTGAGCTCGGTTTGCACTTGAAGG
AAGAAAAATGCCAATGGAGCAAGGAGCGGGCAGGGCTGCTGCAGAGTGTGGAGGCAGAGAAGG
ACAAGATCCTGAAGCTGAGTGCAGAGATACTTCGATTGGAGAAGGCAGTTCAGGAGGAGAGGA
CCCAAAACCAAGTGTTCAAGACTGAGCTGGCCCGGGAGAAGGATTCTAGCCTGGTACAGTTGT
CAGAAAGTAAGCGGGAGCTGACAGAGCTGCGGTCAGCCCTGCGTGTGCTCCAGAAGGAAAAGG
AGCAGTTACAGGAGGAGAAACAGGAATTGCTAGAGTACATGAGAAAGCTAGAGGCCCGCCTGG
AGAAGGTGGCAGATGAGAAGTGGAATGAGGATGCCACCACAGAGGATGAGGAGGCCGCTGTGG
GGCTGAGCTGCCCGGCAGCTCTGACAGACTCAGAGGACGAGTCCCCAGAAGACATGAGGCTCC
CACCCTATGGCCTTTGTGAGCGTGGAGACCCAGGCTCCTCTCCTGCTGGGCCTCGAGAGGCTT
CTCCCCTTGTTGTCATCAGCCAGCCGGCTCCCATTTCTCCTCACCTCTCTGGGCCAGCTGAGG
ACAGTAGCTCTGACTCGGAGGCTGAAGATGAGAAGTCAGTCCTGATGGCAGCTGTGCAGAGTG
GGGGTGAGGAGGCCAACTTACTGCTTCCTGAACTGGGCAGTGCCTTCTATGACATGGCCAGTG
GCTTTACAGTGGGTACCCTGTCAGAAACCAGCACTGGGGGCCCTGCCACCCCCACATGGAAGG
AGTGTCCTATCTGTAAGGAGCGCTTTCCTGCTGAGAGTGACAAGGATGCCCTGGAGGACCACA
TGGATGGACACTTCTTTTTCAGCACCCAGGACCCCTTCACCTTTGAG**TGA**TCTTACTCCCTCG
TACATGCACAAATACACACTCATGCACACACACACTCACACACATGCATACACTTAGGTTTCA
TGCCCATTTTCTATCACACTGGGCTCCATGATATTCTGTTCCCTAAGAACTGCTTCTGTGTGC
CCTGTTTTCATCCCAAGATTTCTCACTTCATCCTCTCCTACCTGGCTCTTTTGTCCCAGGGAG
GGGTCCTGTTCGGAAGCAGTGGCTGAATTTATCCCCTGAAAGTGGTTTTGGAGGAACCGGGAT
GGAGGAGGCCTTCCCCTGTGGGAATAGAATCGTCCACTCCTAGCCCTGGTTGCTTCTGATACA
CAGCCACTGCACACACACACTCACACTCACACTCCCTTGTCTGATGCCCCAAAGCCAATTCCT
GGGGCACCCTACCCTCTCTTATTTGGAGTTTCCGTTGGTTTACCTGAGTTTTCTCTGGGGTCT
GCACAGAGGCAGCAGCATGGACATCATGGCCTCTCAGGTCCCTTTTGGTTCTCAGTTTCATTG
GTTCCTCTTTCTGTTCCCCCATTGACTTCTGTGCCCCACCCTAGCCTTTTCCATAACCTTAGG
TATTCAGTTTGGAGGGGTTTTTTGTATTTTTGAGGATTCCTGTATTCTGTATCCTCTCCTCGC
ATCTCCTCACATGGAAAGAAATAATGTATTTGTGCCTTCTGTGAGGAATGGGGGGAACAAGTG
GTCCCAGGTATCCCCATTTCCAAGGCCCCCCTCCCTCTCCAGGTCCCCCCACAGCAATAAAAG
CTTCCCCCTGATATCCATCCCTTTGTAGTTTGAACAAATATATTTATATGATATGTAA

# FIGURE 16

MEESPLSRAPSRGGVNFLNVARTYIPNTKVECHYTLPPGTMPSASDWIGIFKVEAACVRDYHT
FVWSSVPESTTDGSPIHTSVQFQASYLPKPGAQLYQFRYVNRQGQVCGQSPPFQFREPRPMDE
LVTLEEADGGSDILLVVPKATVLQNQLDESQQERNDLMQLKLQLEGQVTELRSRVQELERALA
TARQEHTELMEQYKGISRSHGEITEERDILSRQQGDHVARILELEDDIQTISEKVLTKEVELD
RLRDTVKALTREQEKLLGQLKEVQADKEQSEAELQVAQQENHHLNLDLKEAKSWQEEQSAQAQ
RLKDKVAQMKDTLGQAQQRVAELEPLKEQLRGAQELAASSQQKATLLGEELASAAAARDRTIA
ELHRSRLEVAEVNGRLAELGLHLKEEKCQWSKERAGLLQSVEAEKDKILKLSAEILRLEKAVQ
EERTQNQVFKTELAREKDSSLVQLSESKRELTELRSALRVLQKEKEQLQEEKQELLEYMRKLE
ARLEKVADEKWNEDATTEDEEAAVGLSCPAALTDSEDESPEDMRLPPYGLCERGDPGSSPAGP
REASPLVVISQPAPISPHLSGPAEDSSSDSEAEDEKSVLMAAVQSGGEEANLLLPELGSAFYD
MASGFTVGTLSETSTGGPATPTWKECPICKERFPAESDKDALEDHMDGHFFFSTQDPFTFE

**Important features:**

**Casein kinase II phosphorylation sites:**

amino acids 28-31, 43-46, 68-71, 72-75, 129-132, 156-159, 208-211, 239-242, 282-285, 305-308, 376-379, 383-383, 468-471, 520-523, 521-524, 537-540, 539-542, 543-546, 593-596, 595-598, 597-600, 612-615, 639-642, 652-655, 667-670, 683-686

**N-myristoylation sites:**

amino acids 39-44, 107-112, 204-209, 414-419, 561-566, 613-618

**Cell attachment sequence:**

amino acids 557-559

**Leucine zipper pattern sequence:**

amino acids 163-184, 475-496, 482-503

# FIGURE 17

GCAAGTTGGGAATTTTAGACTGTCACTGCACATGGACCTCTGGGAAGACGTCTGGCGAGAGCT
AGGCCCACTGGCCCTACAGACGGATCTTGCTGGCTCACCTGTCCCTGTGGAGGTTCCCCTGGG
AAGGCAAG**ATG**CCCAACAACAGCACTGCTCTGTCATTGGCCAATGTTACCTACATCACCATGG
AAATTTTCATTGGACTCTGCGCCATAGTGGGCAACGTGCTGGTCATCTGCGTGGTCAAGCTGA
ACCCCAGCCTGCAGACCACCACCTTCTATTTCATTGTCTCTCTAGCCCTGGCTGACATTGCTG
TTGGGGTGCTGGTCATGCCTTTGGCCATTGTTGTCAGCCTGGGCATCACAATCCACTTCTACA
GCTGCCTTTTTATGACTTGCCTACTGCTTATCTTTACCCACGCCTCCATCATGTCCTTGCTGG
CCATCGCTGTGGACCGATACTTGCGGGTCAAGCTTACCGTCAGATTCAGAATTCCTGGGCTCC
CTGGGTGCATTCTATCATTCCAGTTGAAAGTTTGCTTCCTTCCAGTCATGTGGCTCTTCATTC
TACTCTCCTTGGCTCTCATTTCAGATGCCATGGTCATGGATGAAAAGGTCAAGAGAAGCTTTG
TGCTGGACACGGCTTCTGCCATCTGCAACTACAATGCCCACTACAAGAATCACCCCAAATACT
GGTGCCGAGGCTATTTCCGTGACTACTGCAACATCATCGCCTTCTCCCCTAACAGCACCAATC
ATGTGGCCCTGAGGGACACAGGGAACCAGCTCATTGTCACTATGTCCTGCCTGACCAAAGAGG
ACACGGGCTGGTACTGGTGTGGCATCCAGCGGGACTTTGCCAGGGATGACATGGATTTTACAG
AGCTGATTGTAACTGACGACAAAGGAACCCTGGCCAATGACTTTTGGTCTGGGAAAGACCTAT
CAGGCAACAAAACCAGAAGCTGCAAGGCTCCCAAAGTTGTCCGCAAGGCTGACCGCTCCAGGA
CGTCCATTCTCATCATTTGCATACTGATCACGGGTTTGGGAATCATCTCTGTAATCAGTCATT
TGACCAAAAGGAGGAGAAGTCAAAGGAATAGAAGGGTAGGCAACACTTTGAAGCCCTTCTCGC
GTGTCCTGACTCCAAAGGAAATGGCTCCTACTGAACAGATG**TGA**CTGAAGATTTTTTTAATTT
AGTTCATAAAGTGATGCTACAACAGAATAATCACCATGACAACTGGCCCACACCTCAGAGACT
GATTCTGATCTCCCAGGAATTCTGAAGGACCCTCTATCCTTGACAACAATCATTTGCAGCCAG
GTAGCAACGGCGGTAGTCAGAGGAGCTATGATAGACCACACCCAAGCAAGGCTGCCCTCAAAT
AACATCTCAAGATCTTAGTTCTTATGCATTCCATCAGTCAGAAGTGAAGAAGAGGTGGAGAAT
CTGGATTGGGGACCAGGAAATCACTTGTATTTTGTTAGCCAATAAATTCCTAGCCAGTGTTGA
ATGAAAAAAAAAAAAA

# FIGURE 18

MPNNSTALSLANVTYITMEIFIGLCAIVGNVLVICVVKLNPSLQTTTFYFIVSLALADIAVGV
LVMPLAIVVSLGITIHFYSCLFMTCLLLIFTHASIMSLLAIAVDRYLRVKLTVRFRIPGLPGC
ILSFQLKVCFLPVMWLFILLSLALISDAMVMDEKVKRSFVLDTASAICNYNAHYKNHPKYWCR
GYFRDYCNIIAFSPNSTNHVALRDTGNQLIVTMSCLTKEDTGWYWCGIQRDFARDDMDFTELI
VTDDKGTLANDFWSGKDLSGNKTRSCKAPKVVRKADRSRTSILIICILITGLGIISVISHLTK
RRRSQRNRRVGNTLKPFSRVLTPKEMAPTEQM

**Important features of the protein:**

**Transmembrane domains:**

amino acids 16-35, 62-80, 89-101, 134-152, 292-311

**N-glycosylation sites.**

amino acids 3-7, 4-8, 12-16, 204-208, 273-277

**cAMP- and cGMP-dependent protein kinase phosphorylation site.**

amino acids 316-320

**N-myristoylation sites.**

amino acids 122-128, 125-131, 258-264

**Prokaryotic membrane lipoprotein lipid attachment site.**

amino acids 214-225

**G-protein coupled receptors proteins.**

amino acids 29-59, 76-116

# FIGURE 19

CTCGGGCGCGCACAGGCAGCTCGGTTTGCCCTGCGATTGAGCTGCGGGTCGCGGCCGGCGCCGGCCTCTCCAATG
GCAAATGTGTGTGGCTGGAGGCGAGCGCGAGGCTTTCGGCAAAGGCAGTCGAGTGTTTGCAGACCGGGGCGAGTC
CTGTGAAAGCAGATAAAAGAAAACATTTATTAACGTGTCATTACGAGGGGAGCGCCCGGCCGGGGCTGTCGCACT
CCCCGCGGAACATTTGGCTCCCTCCAGCTCCGAGAGAGGAGAAGAAGAAAGCGGAAAAGAGGCAGATTCACGTCG
TTTCCAGCCAAGTGGACCTGATCGATGGCCCTCCTGAATTTATCACGATATTTGATTTATTAGCGATGCCCCCTG
GTTTGTGTGTTACGCACACACACGTGCACACAAGGCTCTGGCTCGCTTCCCTCCCTCGTTTCCAGCTCCTGGGCG
AATCCCACATCTGTTTCAACTCTCCGCCGAGGGCGAGCAGGAGCGAGAGTGTGTCGAATCTGCGAGTGAAGAGGG
ACGAGGGAAAAGAAACAAAGCCACAGACGCAACTTGAGACTCCCGCATCCCAAAAGAAGCACCAGATCAGCAAAA
AAAGAAG**ATG**GGCCCCCCGAGCCTCGTGCTGTGCTTGCTGTCCGCAACTGTGTTCTCCCTGCTGGGTGGAAGCTC
GGCCTTCCTGTCGCACCACCGCCTGAAAGGCAGGTTTCAGAGGGACCGCAGGAACATCCGCCCCAACATCATCCT
GGTGCTGACGGACGACCAGGATGTGGAGCTGGGTTCCATGCAGGTGATGAACAAGACCCGGCGCATCATGGAGCA
GGGCGGGGCGCACTTCATCAACGCCTTCGTGACCACACCCATGTGCTGCCCCTCACGCTCCTCCATCCTCACTGG
CAAGTACGTCCACAACCACAACACCTACACCAACAATGAGAACTGCTCCTCGCCCTCCTGGCAGGCACAGCACGA
GAGCCGCACCTTTGCCGTGTACCTCAATAGCACTGGCTACCGGACAGCTTTCTTCGGGAAGTATCTTAATGAATA
CAACGGCTCCTACGTGCCACCCGGCTGGAAGGAGTGGGTCGGACTCCTTAAAAACTCCCGCTTTTATAACTACAC
GCTGTGTCGGAACGGGGTGAAAGAGAAGCACGGCTCCGACTACTCCAAGGATTACCTCACAGACCTCATCACCAA
TGACAGCGTGAGCTTCTTCCGCACGTCCAAGAAGATGTACCCGCACAGGCCAGTCCTCATGGTCATCAGCCATGC
AGCCCCCCACGGCCCTGAGGATTCAGCCCCACAATATTCACGCCTCTTCCCAAACGCATCTCAGCACATCACGCC
GAGCTACAACTACGCGCCCAACCCGGACAAACACTGGATCATGCGCTACACGGGGCCCATGAAGCCCATCCACAT
GGAATTCACCAACATGCTCCAGCGGAAGCGCTTGCAGACCCTCATGTCGGTGGACGACTCCATGGAGACGATTTA
CAACATGCTGGTTGAGACGGGCGAGCTGGACAACACGTACATCGTATACACCGCCGACCACGGTTACCACATCGG
CCAGTTTGGCCTGGTGAAAGGGAAATCCATGCCATATGAGTTTGACATCAGGGTCCCGTTCTACGTGAGGGGCCC
CAACGTGGAAGCCGGCTGTCTGAATCCCCACATCGTCCTCAACATTGACCTGGCCCCCACCATCCTGGACATTGC
AGGCCTGGACATACCTGCGGATATGGACGGGAAATCCATCCTCAAGCTGCTGGACACGGAGCGGCCGGTGAATCG
GTTTCACTTGAAAAAGAAGATGAGGGTCTGGCGGGACTCCTTCTTGGTGGAGAGAGGCAAGCTGCTACACAAGAG
AGACAATGACAAGGTGGACGCCCAGGAGGAGAACTTTCTGCCCAAGTACCAGCGTGTGAAGGACCTGTGTCAGCG
TGCTGAGTACCAGACGGCGTGTGAGCAGCTGGGACAGAAGTGGCAGTGTGTGGAGGACGCCACGGGGAAGCTGAA
GCTGCATAAGTGCAAGGGCCCCATGCGGCTGGGCGGCAGCAGAGCCCTCTCCAACCTCGTGCCCAAGTACTACGG
GCAGGGCAGCGAGGCCTGCACCTGTGACAGCGGGGACTACAAGCTCAGCCTGGCCGGACGCCGGAAAAAACTCTT
CAAGAAGAAGTACAAGGCCAGCTATGTCCGCAGTCGCTCCATCCGCTCAGTGGCCATCGAGGTGGACGGCAGGGT
GTACCACGTAGGCCTGGGTGATGCCGCCCAGCCCCGAAACCTCACCAAGCGGCACTGGCCAGGGGCCCCTGAGGA
CCAAGATGACAAGGATGGTGGGGACTTCAGTGGCACTGGAGGCCTTCCCGACTACTCAGCCGCCAACCCCATTAA
AGTGACACATCGGTGCTACATCCTAGAGAACGACACAGTCCAGTGTGACCTGGACCTGTACAAGTCCCTGCAGGC
CTGGAAAGACCACAAGCTGCACATCGACCACGAGATTGAAACCCTGCAGAACAAAATTAAGAACCTGAGGGAAGT
CCGAGGTCACCTGAAGAAAAAGCGGCCAGAAGAATGTGACTGTCACAAAATCAGCTACCACACCCAGCACAAAGG
CCGCCTCAAGCACAGAGGCTCCAGTCTGCATCCTTTCAGGAAGGGCCTGCAAGAGAAGGACAAGGTGTGGCTGTT
GCGGGAGCAGAAGCGCAAGAAGAAACTCCGCAAGCTGCTCAAGCGCCTGCAGAACAACGACACGTGCAGCATGCC
AGGCCTCACGTGCTTCACCCACGACAACCAGCACTGGCAGACGGCGCCTTTCTGGACACTGGGGCCTTTCTGTGC
CTGCACCAGCGCCAACAATAACACGTACTGGTGCATGAGGACCATCAATGAGACTCACAATTTCCTCTTCTGTGA
ATTTGCAACTGGCTTCCTAGAGTACTTTGATCTCAACACAGACCCCTACCAGCTGATGAATGCAGTGAACACACT
GGACAGGGATGTCCTCAACCAGCTACACGTACAGCTCATGGAGCTGAGGAGCTGCAAGGGTTACAAGCAGTGTAA
CCCCCGGACTCGAAACATGGACCTGGATGGAGGAAGCTATGAGCAATACAGGCAGTTTCAGCGTCGAAAGTGGCC
AGAAATGAAGAGACCTTCTTCCAAATCACTGGGACAACTGTGGGAAGGCTGGGAAGGT**TAA**GAAACAACAGAGGT
GGACCTCCAAAAACATAGAGGCATCACCTGACTGCACAGGCAATGAAAAACCATGTGGGTGATTTCCAGCAGACC
TGTGCTATTGGCCAGGAGGCCTGAGAAAGCAAGCACGCACTCTCAGTCAACATGACAGATTCTGGAGGATAACCA
GCAGGAGCAGAGATAACTTCAGGAAGTCCATTTTTGCCCCTGCTTTTGCTTTGGATTATACCTCACCAGCTGCAC
AAAATGCATTTTTTCGTATCAAAAAGTCACCACTAACCCTCCCCCAGAAGCTCACAAAGGAAAACGGAGAGAGCG
AGCGAGAGAGATTTCCTTGGAAATTTCTCCCAAGGGCGAAAGTCATTGGAATTTTTAAATCATAGGGGAAAAGCA
GTCCTGTTCTAAATCCTCTTATTCTTTTGGTTTGTCACAAAGAAGGAACTAAGAAGCAGGACAGAGGCAACGTGG
AGAGGCTGAAAACAGTGCAGAGACGTTTGACAATGAGTCAGTAGCACAAAAGAGATGACATTTACCTAGCACTAT
AAACCCTGGTTGCCTCTGAAGAAACTGCCTTCATTGTATATATGTGACTATTTACATGTAATCAACATGGGAACT
TTTAGGGGAACCTAATAAGAAATCCCAATTTTCAGGAGTGGTGGTGTCAATAAACGCTCTGTGGCCAGTGTAAAA
GAAAAA

# FIGURE 20

MGPPSLVLCLLSATVFSLLGGSSAFLSHHRLKGRFQRDRRNIRPNIILVLTDDQDVELGSMQV
MNKTRRIMEQGGAHFINAFVTTPMCCPSRSSILTGKYVHNHNTYTNNENCSSPSWQAQHESRT
FAVYLNSTGYRTAFFGKYLNEYNGSYVPPGWKEWVGLLKNSRFYNYTLCRNGVKEKHGSDYSK
DYLTDLITNDSVSFFRTSKKMYPHRPVLMVISHAAPHGPEDSAPQYSRLFPNASQHITPSYNY
APNPDKHWIMRYTGPMKPIHMEFTNMLQRKRLQTLMSVDDSMETIYNMLVETGELDNTYIVYT
ADHGYHIGQFGLVKGKSMPYEFDIRVPFYVRGPNVEAGCLNPHIVLNIDLAPTILDIAGLDIP
ADMDGKSILKLLDTERPVNRFHLKKKMRVWRDSFLVERGKLLHKRDNDKVDAQEENFLPKYQR
VKDLCQRAEYQTACEQLGQKWQCVEDATGKLKLHKCKGPMRLGGSRALSNLVPKYYGQGSEAC
TCDSGDYKLSLAGRRKKLFKKKYKASYVRSRSIRSVAIEVDGRVYHVGLGDAAQPRNLTKRHW
PGAPEDQDDKDGGDFSGTGGLPDYSAANPIKVTHRCYILENDTVQCDLDLYKSLQAWKDHKLH
IDHEIETLQNKIKNLREVRGHLKKKRPEECDCHKISYHTQHKGRLKHRGSSLHPFRKGLQEKD
KVWLLREQKRKKKLRKLLKRLQNNDTCSMPGLTCFTHDNQHWQTAPFWTLGPFCACTSANNNT
YWCMRTINETHNFLFCEFATGFLEYFDLNTDPYQLMNAVNTLDRDVLNQLHVQLMELRSCKGY
KQCNPRTRNMDLDGGSYEQYRQFQRRKWPEMKRPSSKSLGQLWEGWEG

**Important features:**

**Signal peptide:**

amino acids 1-17

**Sulfatases signature 1.**

amino acids 86-99

**Homologous region to sulfatase:**

amino acids 87-106, 133-146, 216-229, 291-320, 365-375

**N-glycosylation sites.**

amino acids 65-69, 112-116, 132-136, 149-153, 171-175, 198-202, 241-245, 561-565, 608-612, 717-721, 754-758, 764-768

# FIGURE 21

GGGCGCGCGAGAGCTGCTAGGGCGGTTTCTCTGCCTCGGGCCTGTTGGGCAGGGCCGGCT
AAGGTGCGCGTGCTCGCTGGTTCTAACCCTTCTGTTGGGCGTTTCTGCTGAGAGGCGGGA
GGCGCTGAGAGTCTGTGCGGAGGTCCGTGGACAGACTGCTTTGCTCGTTGTTGCTCTTCG
GAGGCGGCGATCCCCGAAGGCGAGCTGAAATACGGCTGCAGGCTACAATTTGCAGCCGAC
GATTATGGAAGACGGAAGCGGGAGAGGTGGCCCACCCTC**ATG**GAGCGCTTGTGCTCGGAT
GGCTTCGCATTTCCCCAATACCCCATTAAACCGTATCATCTGAAGAGGATCCACAGAGCT
GTCTTACATGGTAATCTAGAGAAACTGAAGTACCTTCTGCTCACGTATTATGACGCCAAT
AAGAGAGACAGGAAGGAAAGGACCGCCCTACATTTGGCCTGTGCCACTGGCCAACCGGAA
ATGGTACATCTCCTGGTGTCCAGAAGATGTGAGCTTAACCTCTGCGACCGTGAAGACAGG
ACACCTCTGATCAAGGCTGTACAACTGAGGCAGGAGGCTTGTGCAACTCTTCTGCTGCAA
AATGGCGCCAATCCAAATATTACGGATTTCTTTGGAAGGACTGCTCTGCACTACGCTGTG
TATAATGAAGATACATCCATGATAGAAAAACTTCTTTCACATGGTACAAATATTGAAGAA
TGCAGCAAGGTA**TAG**GTCAACCAATGTTATTTTCAAACTATCTGAAATGAATTTATTTTA
ACATTGACACATGTAAGGGTCAATTTTTCATATTTGGAAGCTCAAACATTCCTTGAATGA
AAATATTTTGAAATGCCTTAACTGTCTAAGATTTTACTTTAAATATTGGAACTTTTAAAG
AAGCATTATAGGGAACAGCCTTTTTTCATGCACTTATGGTAAATAACTATAAAAACAAAT
GAATTACAATAAATTTATAATTCATGACAACTGAATTTGGGAAAGGTAATAGTTAAGTGT
TTTTCCACTAAATTACTTTTT

# FIGURE 22

MERLCSDGFAFPQYPIKPYHLKRIHRAVLHGNLEKLKYLLLTYYDANKRDRKERTALHLACAT
GQPEMVHLLVSRRCELNLCDREDRTPLIKAVQLRQEACATLLLQNGANPNITDFFGRTALHYA
VYNEDTSMIEKLLSHGTNIEECSKV

**Important features of the protein:**

**N-glycosylation site.**

amino acids 113-117

**N-myristoylation site.**

amino acids 109-115

**Microbodies C-terminal targeting signal.**

amino acids 149-153

# FIGURE 23

GAGGCAGAAAGGCAGAAAGGAGAAAATTCAGGATAACTCTCCTGAGGGGTGAGCCAAGCCCTG
CCATGTAGTGCACGCAGGACATCAACAAACACAGATAACAGGAAATGATCCATTCCCTGTGGT
CACTTATTCTAAAGGCCCCAACCTTCAAAGTTCAAGTAGTGAT**ATG**GATGACTCCACAGAAAG
GGAGCAGTCACGCCTTACTTCTTGCCTTAAGAAAAGAGAAGAAATGAAACTGAAGGAGTGTGT
TTCCATCCTCCCACGGAAGGAAAGCCCCTCTGTCCGATCCTCCAAAGACGGAAAGCTGCTGGC
TGCAACCTTGCTGCTGGCACTGCTGTCTTGCTGCCTCACGGTGGTGTCTTTCTACCAGGTGGC
CGCCCTGCAAGGGGACCTGGCCAGCCTCCGGGCAGAGCTGCAGGGCCACCACGCGGAGAAGCT
GCCAGCAGGAGCAGGAGCCCCCAAGGCCGGCCTGGAGGAAGCTCCAGCTGTCACCGCGGGACT
GAAAATCTTTGAACCACCAGCTCCAGGAGAAGGCAACTCCAGTCAGAACAGCAGAAATAAGCG
TGCCGTTCAGGGTCCAGAAGAAACAGTCACTCAAGACTGCTTGCAACTGATTGCAGACAGTGA
AACACCAACTATACAAAAAGGATCTTACACATTTGTTCCATGGCTTCTCAGCTTTAAAAGGGG
AAGTGCCCTAGAAGAAAAAGAGAATAAAATATTGGTCAAAGAAACTGGTTACTTTTTTATATA
TGGTCAGGTTTTATATACTGATAAGACCTACGCCATGGGACATCTAATTCAGAGGAAGAAGGT
CCATGTCTTTGGGGATGAATTGAGTCTGGTGACTTTGTTTCGATGTATTCAAAATATGCCTGA
AACACTACCCAATAATTCCTGCTATTCAGCTGGCATTGCAAAACTGGAAGAAGGAGATGAACT
CCAACTTGCAATACCAAGAGAAAATGCACAAATATCACTGGATGGAGATGTCACATTTTTTGG
TGCATTGAAACTGCTG**TGA**CCTACTTACACCATGTCTGTAGCTATTTTCCTCCCTTTCTCTGT
ACCTCTAAGAAGAAAGAATCTAACTGAAAATACCAAAAAAAAAAAAAAAA

# FIGURE 24

MDDSTEREQSRLTSCLKKREEMKLKECVSILPRKESPSVRSSKDGKLLAATLLLALLSCCLTV
VSFYQVAALQGDLASLRAELQGHHAEKLPAGAGAPKAGLEEAPAVTAGLKIFEPPAPGEGNSS
QNSRNKRAVQGPEETVTQDCLQLIADSETPTIQKGSYTFVPWLLSFKRGSALEEKENKILVKE
TGYFFIYGQVLYTDKTYAMGHLIQRKKVHVFGDELSLVTLFRCIQNMPETLPNNSCYSAGIAK
LEEGDELQLAIPRENAQISLDGDVTFFGALKLL

**Transmembrane domain:**
amino acids 47-72

**N-glycosylation site.**
amino acids 124-127, 242-245

**cAMP- and cGMP-dependent protein kinase phosphorylation site.**
amino acids 33-36, 173-176

**N-myristoylation site.**
amino acids 96-101

**TNF family proteins.**
amino acids 172-206

# FIGURE 25

CTGCTTGGATACCTCCAGTCCCCAAACTGTGTTCCAGGAGTTTTCTTGGCCGAAGCTGCCCGA
TGTTTGAGCCTTTTCTTCCCAGAGAAGAAGATGGACTGAAAGCTGCCAGTTGGGGACTTTTTG
TGATCACGGCGTTGCAGCGTTTTAAAGGAGGTGATGGGGCTTGCGCTGGCTTGTCTTCCCACC
CAAGTGAAGAGTTGATGTTCACTGGTTATGCTTAGACAATGTGCAGTTTGTGTTAATTTAAAA
TTTTGGGTGGGATAGGGGCATAGGCTTGTGAAGGGCAGTCCGGATCCGGAGGAACTCGTCTTT
GTCCCTGGTAGGAGAGACACCCCCAGTCTATCCTCGATGCCGTCAGCCTTGGCCATCTTCACT
TGCCGCCCGAACTCGCACCCGTTTCAGGAGCGTCATGTCTACCTGGACGAGCCCATCAAAATC
GGCCGCTCAGTGGCCCGCTGTCGACCAGCGCAGAATAATGCCACTTTTGATTGCAAAGTGCTA
TCAAGGAACCACGCTCTCGTCTGGTTTGATCACAAGACGGGCAAGTTTTATCTTCAAGACACT
AAAAGTAGTAATGGTACTTTTATAAATAGCCAGAGATTGAGTCGAGGCTCTGAAGAAAGTCCA
CCATGTGAAATTCTTTCCGGTGACATTATCCAGTTTGGAGTAGACGTGACAGAGAATACACGG
AAAGTTACCCATGGGTGTATTGTTTCCACAATAAAACTTTTTCTACCAGATGGT**ATG**GAAGCC
CGGCTCCGCTCAGATGTCATCCATGCACCATTACCAAGTCCTGTTGACAAAGTTGCTGCTAAC
ACTCCAAGTATGTACTCTCAGGAACTATTCCAGCTTTCTCAGTATCTACAGGAGGCCTTACAT
CGGGAACAAATGTTGGAACAGAAGTTAGCCACGCTTCAGCGGCTACTAGCCATCACCCAAGAG
GCTTCAGATACCAGTTGGCAGGCTTTAATAGATGAAGATAGACTCTTATCACGGTTAGAAGTT
ATGGGAAACCAATTACAGGCATGCTCCAAAAATCAAACAGAAGATAGTTTACGAAAGGAACTT
ATAGCATTACAAGAGGATAAACATAACTATGAGACAACAGCCAAAGAGTCCCTGAGGCGGGTT
CTTCAGGAGAAAATTGAAGTGGTTAGAAAACTTTCAGAAGTTGAGCGAAGTCTGAGTAATACT
GAAGATGAATGTACCCATCTGAAAGAAATGAATGAAAGGACTCAGGAAGAATTAAGAGAATTA
GCCAACAAATATAATGGAGCAGTTAATGAGATTAAAGATTTATCTGATAAATTAAAGGTAGCA
GAGGGAAAACAAGAGGAAATCCAACAGAAGGGACAGGCTGAGAAAAAAGAATTACAACATAAA
ATAGATGAAATGGAAGAAAAAGAACAGGAGCTCCAGGCAAAAATAGAAGCTTTGCAAGCTGAT
AATGATTTCACCAATGAAAGGCTAACAGCTTTACAAGTACGGTTAGAACATCTTCAGGAGAAA
ACTCTTAAAGAATGCAGCAGCTTGGCTGATCGTCGAAGGGCATCTAACCAAAGCGGTAGAAGA
AACAAAGCTTTCAAAAGGTTTGTTTTCTGTTTTTCTATGTTTTTTGACAGTTCTTTTGGA**TAA**
TGAAGGTTAGTGTATATTTTCAAGGTTATAGTATTTTAACCATCAGTTTACTTCTTATAGCTC
ACAAAATAGCAAGCCAGTAACAGTATCAGATAATATATAAAATAATCAGACTTCTGTTTTAAG
AAGGGTATCGTAACTGGAATGTGTCTTTTTAAGTGGATGTATATTTATGGTTTTTTGAATGTT
AGTACTTGATATAGGTTTCTTTAGGTATTAAAGATTTGTTGCAATCTCTGTCATTCCCAGCAT
TAATTTCAGCTTTGATCTCAAATTTTAATCAAACACAATGTAAGTCGTTTGTGATACAACTTA
AGTGAAACATGCTTGCACTTCTATTTTGGGGGTTACAGTACCTTTAAAATCTCTTATGATGTT
TAATATTTCCTTAATTTTTGGCATCTCAGTTTGATTTAAACAAAATTAATGACTTTTGTGAAT
GTAGAATCTTCTTATATTTTATGAGTAGTCCAGTAATTGCCCAAAGTAGTTTATTGTGTTAAT
TCTGTTACAGTTGTCAGAGAAGAAAAGTGAGTTTTAAAGCACCATATTGTCAAGTCACTTTTA
TACATAGGGAAATTAGGCAAATAAATTTGGTGGCATGTGTTTATCATAGTAGAACTTTCATTA
GACTATACCAGTATAAAATTTAAAACTAGATTCACAGTCCTTTTGGCCAATTAAAACATTGAG
TTACAAAAGTTTGAGATACTTAATTTTAGTACATTCTATTTTATTAAAGTAACTGGATTCATT
TGACTTTTTTAACCATGTAAGAGGATGGTGTTATTTCAAATATCTCGTGGTTTCCATTCTGAA
TTTTGTGCACGGCAGATGCCATATTTGGGGAAAAAATGCATAGAATATGCATCATTAATATTG
TTTTGGCAAACAGGCATTGAGTTTCAGAACAGTGAACTATTTTTAGTACATATGGCAATTTTT
TTCACCTTATTAAAGTGAGATGAGAACAGACCTTAAAATAGCTTTTACCTCACCATCCAAATA
CCTATTCAGATTAGTTGGTTGAATAGCCAGCACTTTGAAGTAGAGCCTTAGG

# FIGURE 26

MEARLRSDVIHAPLPSPVDKVAANTPSMYSQELFQLSQYLQEALHREQMLEQKLATLQRLLAI
TQEASDTSWQALIDEDRLLSRLEVMGNQLQACSKNQTEDSLRKELIALQEDKHNYETTAKESL
RRVLQEKIEVVRKLSEVERSLSNTEDECTHLKEMNERTQEELRELANKYNGAVNEIKDLSDKL
KVAEGKQEEIQQKGQAEKKELQHKIDEMEEKEQELQAKIEALQADNDFTNERLTALQVRLEHL
QEKTLKECSSLADRRRASNQSGRRNKAFKRFVFCFSMFFDSSFG

**Important features of the protein:**

**N-glycosylation sites.**

amino acids 98-102, 271-275

**cAMP- and cGMP-dependent protein kinase phosphorylation site.**

amino acids 138-142, 267-271

**Amidation site.**

amino acids 273-277

**Tropomyosins proteins.**

amino acids 169-217

# FIGURE 27

GAACCTGGCGCCGCCGGAACTGATCGCGGCCTAGTCCCGACGCGTGTGTGCTAGTGAGCCGGA
GCCGGCGACGGCGGCAGTGGCGGCCCGGCCTGCAGGAGCCCGACGGGGTCTCTGCCATGGGGG
AGTGACGCGCCTGCACCCGCTGTTCCGCGGCAGCGGCGAGACATGAGGAGACCCCGCGACAGG
GGCAGCGGCGGCGGCTCGTGAGCCCCGGG**ATG**GAGGAGAAATACGGCGGGGACGTGCTGGCCG
GCCCCGGCGGCGGCGGCGGCCTTGGGCCGGTGGACGTACCCAGCGCTCGATTAACAAAATATA
TTGTGTTACTATGTTTCACTAAATTTTTGAAGGCTGTGGGACTTTTCGAATCATATGATCTCC
TAAAAGCTGTTCACATTGTTCAGTTCATTTTTATATTAAAACTTGGGACTGCATTTTTTATGG
TTTTGTTTCAAAAGCCATTTTCTTCTGGGAAAACTATTACCAAACACCAGTGGATCAAAATAT
TTAAACATGCAGTTGCTGGGTGTATTATTTCACTCTTGTGGTTTTTTGGCCTCACTCTTTGTG
GACCACTAAGGACTTTGCTGCTATTTGAGCACAGTGATATTGTTGTCATTTCACTACTCAGTG
TTTTGTTCACCAGTTCTGGAGGAGGACCAGCAAAGACAAGGGGAGCTGCTTTTTTCATTATTG
CTGTGATCTGTTTATTGCTTTTTGACAATGATGATCTCATGGCTAAAATGGCTGAACACCCTG
AAGGACATCATGACAGTGCTCTAACTCATATGCTTTACACAGCCATTGCCTTCTTAGGTGTGG
CAGATCACAAGGGTGGAGTATTATTGCTAGTACTGGCTTTGTGTTGTAAAGTTGGTTTTCATA
CAGCTTCCAGAAAGCTCTCTGTCGACGTTGGTGGAGCTAAACGTCTTCAAGCTTTATCTCATC
TTGTTTCTGTGCTTCTCTTGTGCCCATGGGTCATTGTTCTTTCTGTGACAACTGAGAGTAAAG
TGGAGTCTTGGTTTTCTCTCATTATGCCTTTTGCAACGGTTATCTTTTTTGTCATGATCCTGG
ATTTCTACGTGGATTCCATTTGTTCAGTCAAAATGGAAGTTTCCAAATGTGCTCGTTATGGAT
CCTTTCCCATTTTTATTAGTGCTCTCCTTTTTGGAAATTTTTGGACACATCCAATAACAGACC
AGCTTCGGGCTATGAACAAAGCAGCACACCAGGAGAGCACTGAACACGTCCTGTCTGGAGGAG
TGGTAGTGAGTGCTATATTCTTCATTTTGTCTGCCAATATCTTATCATCTCCCTCTAAGAGAG
GACAAAAAGGTACCCTTATTGGATATTCTCCTGAAGGAACACCTCTTTATAACTTCATGGGTG
ATGCTTTTCAGCATAGCTCTCAATCGATCCCTAGGTTTATTAAGGAATCACTAAAACAAATTC
TTGAGGAGAGTGACTCTAGGCAGATCTTTTACTTCTTGTGCTTGAATCTGCTTTTTACCTTTG
TGGAATTATTCTATGGCGTGCTGACCAATAGTCTGGGCCTGATCTCGGATGGATTCCACATGC
TTTTTGACTGCTCTGCTTTAGTCATGGGACTTTTTGCTGCCCTGATGAGTAGGTGGAAAGCCA
CTCGGATTTTCTCCTATGGGTACGGCCGAATAGAAATTCTGTCTGGATTTATTAATGGACTTT
TTCTAATAGTAATAGCGTTTTTTGTGTTTATGGAGTCAGTGGCTAGATTGATTGATCCTCCAG
AATTAGACACTCACATGTTAACACCAGTCTCAGTTGGAGGGCTGATAGTAAACCTTATTGGTA
TCTGTGCCTTTAGCCATGCCCATAGCCATGCCCATGGAGCTTCTCAAGGAAGCTGTCACTCAT
CTGATCACAGCCATTCACACCATATGCATGGACACAGTGACCATGGGCATGGTCACAGCCACG
GATCTGCGGGTGGAGGCATGAATGCTAACATGAGGGGTGTATTTCTACATGTTTTGGCAGATA
CACTTGGCAGCATTGGTGTGATCGTATCCACAGTTCTTATAGAGCAGTTTGGATGGTTCATCG
CTGACCCACTCTGTTCTCTTTCTACTGCTATATTAATATTTCTCAGTGTTGTTCCACTGATTA
AAGATGCCTGCCAGGTTCTACTCCTGAGATTGCCACCAGAATATGAAAAAGAACTACATATTG
CTTTAGAAAAGATACAGAAAATTGAAGGATTAATATCATACCGAGACCCTCATTTTTGGCGTC
ATTCTGCTAGTATTGTGGCAGGAACAATTCATATACAGGTGACATCTGATGTGCTAGAACAAA
GAATAGTACAGCAGGTTACAGGAATACTTAAAGATGCTGGAGTAAACAATTTAACAATTCAAG
TGGAAAAGGAGGCATACTTTCAACATATGTCTGGCCTAAGTACTGGATTTCATGATGTTCTGG
CTATGACAAAACAAATGGAATCCATGAAATACTGCAAAGATGGTACTTACATCATG**TGA**GATA
ACTCAAGAATTACCCCTGGAGAATAAACAATGAAGATTAAATGACTCAGTATTTGTAATATTG
CCAGAAGGATAAAAATTACACATTAACTGTACAGAAACAGAGTTCCCTACTACTGGATCAAGG
AATCTTTCTTGAAGGAAATTTAAATACAGAATGAAACATTAATGGTAAAAAAAA

# FIGURE 28

MEEKYGGDVLAGPGGGGGLGPVDVPSARLTKYIVLLCFTKFLKAVGLFESYDLLKAVHIVQFI
FILKLGTAFFMVLFQKPFSSGKTITKHQWIKIFKHAVAGCIISLLWFFGLTLCGPLRTLLLFE
HSDIVVISLLSVLFTSSGGGPAKTRGAAFFIIAVICLLLFDNDDLMAKMAEHPEGHHDSALTH
MLYTAIAFLGVADHKGGVLLLVLALCCKVGFHTASRKLSVDVGGAKRLQALSHLVSVLLLCPW
VIVLSVTTESKVESWFSLIMPFATVIFFVMILDFYVDSICSVKMEVSKCARYGSFPIFISALL
FGNFWTHPITDQLRAMNKAAHQESTEHVLSGGVVVSAIFFILSANILSSPSKRGQKGTLIGYS
PEGTPLYNFMGDAFQHSSQSIPRFIKESLKQILEESDSRQIFYFLCLNLLFTFVELFYGVLTN
SLGLISDGFHMLFDCSALVMGLFAALMSRWKATRIFSYGYGRIEILSGFINGLFLIVIAFFVF
MESVARLIDPPELDTHMLTPVSVGGLIVNLIGICAFSHAHSHAHGASQGSCHSSDHSHSHHMH
GHSDHGHGHSHGSAGGGMNANMRGVFLHVLADTLGSIGVIVSTVLIEQFGWFIADPLCSLSTA
ILIFLSVVPLIKDACQVLLLRLPPEYEKELHIALEKIQKIEGLISYRDPHFWRHSASIVAGTI
HIQVTSDVLEQRIVQQVTGILKDAGVNNLTIQVEKEAYFQHMSGLSTGFHDVLAMTKQMESMK
YCKDGTYIM

**Important features of the protein:**

**Signal peptide:**

amino acids 1-46

**Transmembrane domains:**

amino acids 59-77, 101-119, 150-167, 205-223, 239-258, 267-284, 305-324, 343-360, 421-440, 452-469, 486-505, 522-539, 592-612, 621-641

**N-glycosylation site.**

amino acids 721-725

**Glycosaminoglycan attachment site.**

amino acids 143-147

**cAMP- and cGMP-dependent protein kinase phosphorylation site.**

amino acids 225-229

**Tyrosine kinase phosphorylation sites.**

amino acids 750-758, 756-764

**N-myristoylation sites.**

amino acids 14-20, 46-52, 102-108, 112-118, 144-150, 317-323, 347-353, 369-375, 372-378, 437-443, 462-468, 529-535, 549-555, 553-559, 579-585, 582-588, 583-589, 584-590, 605-611, 737-743

**Multicopper oxidases protein:**

amino acids 561-569

# FIGURE 29

GGCACGAGGGCAGGATATTAGAA**ATG**GCTACTCCCCAGTCAATTTTCATCTTTGCAATCTGCA
TTTTAATGATAACAGAATTAATTCTGGCCTCAAAAAGCTACTATGATATCTTAGGTGTGCCAA
AATCGGCATCAGAGCGCCAAATCAAGAAGGCCTTTCACAAGTTGGCCATGAAGTACCACCCTG
ACAAAAATAAGAGCCCGGATGCTGAAGCAAAATTCAGAGAGATTGCAGAAGCATATGAAACAC
TCTCAGATGCTAATAGACGAAAAGAGTATGATACACTTGGACACAGTGCTTTTACTAGTGGTA
AAGGACAAAGAGGTAGTGGAAGTTCTTTTGAGCAGTCATTTAACTTCAATTTTGATGACTTAT
TTAAAGACTTTGGCTTTTTTGGTCAAAACCAAAACACTGGATCCAAGAAGCGTTTTGAAAATC
ATTTCCAGACACGCCAGGATGGTGGTTCCAGTAGACAAAGGCATCATTTCCAAGAATTTTCTT
TTGGAGGTGGATTATTTGATGACATGTTTGAAGATATGGAGAAAATGTTTTCTTTTAGTGGTT
TTGACTCTACCAATCAGCATACAGTACAGACTGAAAATAGATTTCATGGATCTAGCAAGCACT
GCAGGACTGTCACTCAACGAAGAGGAAATATGGTTACTACATACACTGACTGTTCAGGACAG**T**
**AG**TTCTTATTCTATTCTCACTAAATCCAACTGGTTGACTCTTCCTCATTATCTTTGATGCTAA
ACAATTTTCTGTGAACTATTTTGACAAGTGCATGATTTCACTTTAAACAATTTGATATAGCTA
TTAAATATATTTAAGGGTTTTTTTTTTGACAAATTCAACATTCAACGAGTAGACAAAATGCT
AATTATTTCCCTGATTAGGAAAGTTTCTTTAAAAAACACGTAATTTTGCCTAGTGCTTTTTCT
CTACCTGCCCTTGGGCTCACTAATATCACCAGTATTATTACCAAGAAAATATTGAGTTTACCT
GATTAAACTTTAAAAGTTAATTGTAGATTTAAATTGTGTGAACCTAATGATTTTTGCAGTGAA
ACCTTTACTAATTCAAAGTTGCATGTTCTATGACATCTGTGACTTGCGTTGCAGAGTGTACAT
GAAACTGTATAATTGAGTCATTCAGTAAAGGAGAACAGTATCTTGGTTAATTGCTACTGAAAG
GTTGAGAAAGGAATGGTTTGATATTTACCACAGCGCTGTGCCTTTCTACAGTAGAACTGGGGT
AAAGGAAATGGTTTTATTGCCCATAGTCATTTAGGCTGGAAAAAAGTTGAAAACTTAACGAAA
TATTGCCAAGAGATTGTTATGTGTTTGGTTCCAGCCTAAAAATGATTTTGTAGTGTTGAAATC
ATAGCTACTTACATAGCTTTTTCATATTTCTTTCTTAGTTGTTGGCACTCTTAGGTCTTAGTA
TGGATTTATGTGTTTGTGTGTGTGTAGTTTATCCTCTCTCTCATCTTTATCTAGAGATTGACT
GATACCTCATTCTGTTTGTAAAACCAGCCAGTAATTTCTGTGCAACCTTACTATGTGCAATAT
TTTTAAATCCTGAGAAATGTGTGCTTTTGTTTTCGGATAGACTTATTTCTTTAGTTCTGCACT
TTTCCACATTATACTCCATATGAGTATTAATCCTATGGATACATATTAAAACAAGTGTCTCAT

# FIGURE 30

MATPQSIFIFAICILMITELILASKSYYDILGVPKSASERQIKKAFHKLAMKYHPDKNKSPDA
EAKFREIAEAYETLSDANRRKEYDTLGHSAFTSGKGQRGSGSSFEQSFNFNFDDLFKDFGFFG
QNQNTGSKKRFENHFQTRQDGGSSRQRHHFQEFSFGGGLFDDMFEDMEKMFSFSGFDSTNQHT
VQTENRFHGSSKHCRTVTQRRGNMVTTYTDCSGQ

**Important features of the protein:**

**Signal peptide:**

amino acids 1-23

**Nt-dnaJ domain signature.**

amino acids 27-59, 66-90

**Glycosaminoglycan attachment site.**

amino acids 96-100

**N-myristoylation sites.**

amino acids 32-38, 99-105, 102-108, 126-132, 211-217

# FIGURE 31

AAAGTTACATTTTCTCTGGAACTCTCCTAGGCCACTCCCTGCTGATGCAACATCTGGGTTTGG
GCAGAAAGGAGGGTGCTTCGGAGCCCGCCCTTTCTGAGCTTCCTGGGCCGGCTCTAGAACAAT
TCAGGCTTCGCTGCGACTCAGACCTCAGCTCCAACATATGCATTCTGAAGAAAGATGGCTGAG
ATGGACAGAATGCTTTATTTTGGAAAGAAACAATGTTCTAGGTCAAACTGAGTCTACCAA**ATG**
CAGACTTTCACAATGGTTCTAGAAGAAATCTGGACAAGTCTTTTCATGTGGTTTTTCTACGCA
TTGATTCCATGTTTGCTCACAGATGAAGTGGCCATTCTGCCTGCCCCTCAGAACCTCTCTGTA
CTCTCAACCAACATGAAGCATCTCTTGATGTGGAGCCCAGTGATCGCGCCTGGAGAAACAGTG
TACTATTCTGTCGAATACCAGGGGGAGTACGAGAGCCTGTACACGAGCCACATCTGGATCCCC
AGCAGCTGGTGCTCACTCACTGAAGGTCCTGAGTGTGATGTCACTGATGACATCACGGCCACT
GTGCCATACAACCTTCGTGTCAGGGCCACATTGGGCTCACAGACCTCAGCCTGGAGCATCCTG
AAGCATCCCTTTAATAGAAACTCAACCATCCTTACCCGACCTGGGATGGAGATCACCAAAGAT
GGCTTCCACCTGGTTATTGAGCTGGAGGACCTGGGGCCCCAGTTTGAGTTCCTTGTGGCCTAC
TGGAGGAGGGAGCCTGGTGCCGAGGAACATGTCAAAATGGTGAGGAGTGGGGGTATTCCAGTG
CACCTAGAAACCATGGAGCCAGGGGCTGCATACTGTGTGAAGGCCCAGACATTCGTGAAGGCC
ATTGGGAGGTACAGCGCCTTCAGCCAGACAGAATGTGTGGAGGTGCAAGGAGAGGCCATTCCC
CTGGTACTGGCCCTGTTTGCCTTTGTTGGCTTCATGCTGATCCTTGTGGTCGTGCCACTGTTC
GTCTGGAAAATGGGCCGGCTGCTCCAGTACTCCTGTTGCCCCGTGGTGGTCCTCCCAGACACC
TTGAAAATAACCAATTCACCCCAGAAGTTAATCAGCTGCAGAAGGGAGGAGGTGGATGCCTGT
GCCACGGCTGTGATGTCTCCTGAGGAACTCCTCAGGGCCTGGATCTCA**TAG**GTTTGCGGAAGG
GCCCAGGTGAAGCCGAGAACCTGGTCTGCATGACATGGAAACCATGAGGGGACAAGTTGTGTT
TCTGTTTTCCGCCACGGACAAGGGATGAGAGAAGTAGGAAGAGCCTGTTGTCTACAAGTCTAG
AAGCAACCATCAGAGGCAGGGTGGTTTGTCTAACAGAACACTGACTGAGGCTTAGGGGATGTG
ACCTCTAGACTGGGGGCTGCCACTTGCTGGCTGAGCAACCCTGGGAAAAGTGACTTCATCCCT
TCGGTCCTAAGTTTTCTCATCTGTAATGGGGGAATTACCTACACACCTGCTAAACACACACAC
ACAGAGTCTCTCTCTATATATACACACGTACACATAAATACACCCAGCACTTGCAAGGCTAGA
GGGAAACTGGTGACACTCTACAGTCTGACTGATTCAGTGTTTCTGGAGAGCAGGACATAAATG
TATGATGAGAATGATCAAGGACTCTACACACTGGGTGGCTTGGAGAGCCCACTTTCCCAGAAT
AATCCTTGAGAGAAAAGGAATCATGGGAGCAATGGTGTTGAGTTCACTTCAAGCCCAATGCCG
GTGCAGAGGGGAATGGCTTAGCGAGCTCTACAGTAGGTGACCTGGAGGAAGGTCACAGCCACA
CTGAAAATGGGATGTGCATGAACACGGAGGATCCATGAACTACTGTAAAGTGTTGACAGTGTG
TGCACACTGCAGACAGCAGGTGAAATGTATGTGTGCAATGCGACGAGAATGCAGAAGTCAGTA
ACATGTGCATGTTTGTTGTGCTCCTTTTTCTGTTGGTAAAGTACAGAATTCAGCAAATAAAA
AGGGCCACCCTGGCCAAAAGCGGTAAAAAAAAAAAAAAAA

# FIGURE 32

MQTFTMVLEEIWTSLFMWFFYALIPCLLTDEVAILPAPQNLSVLSTNMKHLLMWSPVIAPGET
VYYSVEYQGEYESLYTSHIWIPSSWCSLTEGPECDVTDDITATVPYNLRVRATLGSQTSAWSI
LKHPFNRNSTILTRPGMEITKDGFHLVIELEDLGPQFEFLVAYWRREPGAEEHVKMVRSGGIP
VHLETMEPGAAYCVKAQTFVKAIGRYSAFSQTECVEVQGEAIPLVLALFAFVGFMLILVVVPL
FVWKMGRLLQYSCCPVVVLPDTLKITNSPQKLISCRREEVDACATAVMSPEELLRAWIS

**Important features:**

**Signal peptide:**

amino acids 1-29

**Transmembrane domain:**

amino acids 230-255

**N-glycosylation sites.**

amino acids 40-44, 134-138

**Tissue factor proteins.**

amino acids 92-120

**Integrins alpha chain proteins.**

amino acids 232-263

# FIGURE 33

GAGACACGCGAGCGGGGAGACCTCCAAGGCAGCGAGGCATCGGACATGTGTCAGCACATCTGG
GGCGCACATCCGTCGAGCCCGAGGGGAGATTTGCCGGAACAATTCAAACTGCGATATTGATCT
TGGGGGTGACTGTCCCTGGCCGGCTGTCGGGTGGGAGTGCGAGTGTGCACTCGCTCGGAAGTG
TGTGCGAGTGTGTATGTGTGTGTGCCGTGTCGGGCTCCCCCCTTCCCCCCGTTTTCCCGTCGA
GTGATGCACTTGGAATGAGAATCAGAGG**ATG**GAAATAGTCTGGGAGGTGCTTTTTCTTCTTCA
AGCCAATTTCATCGTCTGCATATCAGCTCAACAGAATTCACCAAAAATCCATGAAGGCTGGTG
GGCATACAAGGAGGTGGTCCAGGGAAGCTTTGTTCCAGTTCCTTCTTTCTGGGGATTGGTGAA
CTCAGCTTGGAATCTTTGCTCTGTGGGGAAACGGCAGTCGCCAGTCAACATAGAGACCAGTCA
CATGATCTTCGACCCCTTTCTGACACCTCTTCGCATCAACACGGGGGGCAGGAAGGTCAGTGG
GACCATGTACAACACTGGAAGACACGTATCCCTTCGCCTGGACAAGGAGCACTTGGTCAACAT
ATCTGGAGGGCCCATGACATACAGCCACCGGCTGGAGGAGATCCGACTACACTTTGGGAGTGA
GGACAGCCAAGGGTCGGAGCACCTCCTCAATGGACAGGCCTTCTCTGGGGAGGTGCAGCTCAT
CCACTATAACCATGAGCTATATACGAATGTCACAGAAGCTGCAAAGAGTCCAAATGGATTGGT
GGTAGTTTCTATATTTATAAAAGTTTCTGATTCATCAAACCCATTTCTTAATCGAATGCTCAA
CAGAGATACTATCACAAGAATAACATATAAAAATGATGCATATTTACTACAGGGGCTTAATAT
AGAGGAACTATATCCAGAGACCTCTAGTTTCATCACTTACGATGGGTCGATGACTATCCCACC
CTGCTATGAGACAGCAAGTTGGATCATAATGAACAAACCTGTCTATATAACCAGGATGCAGAT
GCATTCCTTGCGCCTGCTCAGCCAGAACCAGCCATCTCAGATCTTTCTGAGCATGAGTGACAA
CTTCAGGCCTGTCCAGCCACTCAACAACCGCTGCATCCGCACCAATATCAACTTCAGTTTACA
GGGGAAGGACTGTCCAAACAACCGAGCCCAGAAGCTTCAGTATAGAGTAAATGAATGGCTCCT
CAAG**TAG**GGAACAAAGCCAAGAAGAATCCCACCTCAGTGAAATGCTACAACTGTGAATTGACG
TAACCTAGAATGTCCCCCTTCTTGCTTCTCTCTCCTTCTTTCCCCCAAGCCTCATTCATTCTT
GGGATTGGCCCTTTCTTCATGAAAAGTGTCTGCGAAACCATGGCAGAGGAATACATCTCTCAC
ACATACTCACAAACACACACACAAGCACTTGCACATACATACAAACACATGCAAACATACCTA
CACACACACACTCTCTTACAACCTCCATCATGGGAAGTCAAGTTTCAGAAACAAAAGTCTCAT
TCATAAGAGGTCTTAGAAGAAAATAACCAGTTAACCTGATTTCAATTTTGATACCGTTTTCCT
GAACTAATAAATCTACCCAATGAGACTTTTCAGCCTTTGTACATACAAAATTCTTCCAAAAGA
GAGAGGAGAAAATACAGCTCTGATGGCATCAAACGGACTTTGCATCAAGTAATTTCAGATAGT
GTCCTAGGATCCTTTGAGGGTGCTGGTAGCAGGTGAGCAGGACAAAGTTGACCAAGGACACTT
ATTTCTAGATTATGATTCTTCTGTTTACTCAACAATTTACAAAGAAAAAAAGGACAGACATTG
AAGAGCTACACATTGTATATATATCACCACAGACTATAAGGAAATGGAATTATTTCCCTCTTT
GTCACATATCTGTAGTAGGATTTGCCAAGATCAGAAATGATCCATTTGCTGTTTCTTGTTTTC
CAAAGGTCATACATTGTGTTTGGTTATTGTTACCAGCTCAATAAATGTGTTTAACGAGTTAAT
TTCATTTTTCTGGCTTTGGTCTGTTCTCCTTCCTTACAGGCTAAGCCCTGGCTCCATGCAACT
GCATTCTTTGATTTCACTTGTTCCTTCATCTACATGTTTTGTTCATTTGCAGCCAGTTTTTAC
TGAGTTTGTGGCAATCAGGAATGCATTTGCTAAGCAAGTATGACTTTAATTCCACTCCATGGC
TCAATCATTCACATGAGGTGAGCTTCAGCCTGAGATAGCAGGCGACAGACTTCTTGCGTTTCA
AAACTGCCATGCCCCCCTGTGATGCTCCCGTGAAGGAATGCACTTTGCCTTGTAAGTTCCTGG
GAAAGGGGTATGTTTTCTCTCCAGGTGCAGCCAGATCTCACAAAGTACAAAACGAATGCCTTT
CTTTTCTTGTTTATAATGGTCACTCACTGTGTTTGGTTACTGTCAAGAAATCAATAAATGTGT
TTAACAAGTTA

# FIGURE 34

MEIVWEVLFLLQANFIVCISAQQNSPKIHEGWWAYKEVVQGSFVPVPSFWGLVNSAWNLCSVG
KRQSPVNIETSHMIFDPFLTPLRINTGGRKVSGTMYNTGRHVSLRLDKEHLVNISGGPMTYSH
RLEEIRLHFGSEDSQGSEHLLNGQAFSGEVQLIHYNHELYTNVTEAAKSPNGLVVVSIFIKVS
DSSNPFLNRMLNRDTITRITYKNDAYLLQGLNIEELYPETSSFITYDGSMTIPPCYETASWII
MNKPVYITRMQMHSLRLLSQNQPSQIFLSMSDNFRPVQPLNNRCIRTNINFSLQGKDCPNNRA
QKLQYRVNEWLLK

**Important features:**

**Signal peptide:**

amino acids 1-20

**Eukaryotic-type carbonic anhydrases proteins.**

amino acids 126-162, 220-269, 43-91

**N-glycosylation sites.**

amino acids 116-119, 168-171, 302-305

# FIGURE 35

GTCGGAACCCCCTCAGGCCACCCTCGGGAGTCCTGGGGTCCAGAGGGGTGTCCCTGTACCCCTTGCAC
ACAGGACCCTCACTCTGCAGGGATAAGCCAGCTGCGCCTGCAGCCTAGGGTGCCAAGGAGGCTGCTGA
TTGTGGCCCACAGCCTCATCTGAACGCCAGGAGACCAGGATACCGAGGCACCGGATCCCCTCTCTGTG
CCCTGGGGAGCCCCAGTGCTGCCCAGTCACCCCAGGGCTGAGGTCTGCGTCCCTAGTGGTGCAAGGCC
TGGTAGGACCACGGGGCAGGGAATGTGAGCGCCATCCGAGCTCACGGTGTCCTGAGTCGCGGCTTCGT
GACTTTGGCAGGGGCCTCCGGACCAGTGACCCCAGTCAAACCCAGAGGGTCTTGGGCGGCAGCGACGA
AGGAGGTATTCAGGCTCCAGGCCAGGTGGGGCCGGACGCCCCCAGCCATCCACC**ATG**GTGGTGGCACA
CCCCACCGCCACTGCCACCACCACGCCCACTGCCACTGTCACGGCCACCGTTGTGATGACCACGGCCA
CCATGGACCTGCGGGACTGGCTGTTCCTCTGCTACGGGCTCATCGCCTTCCTGACGGAGGTCATCGAC
AGCACCACCTGCCCCTCGGTGTGCCGCTGCGACAACGGCTTCATCTACTGCAACGACCGGGGACTCAC
ATCCATCCCCGCAGATATCCCTGATGACGCCACCACCCTCTACCTGCAGAACAACCAGATCAACAACG
CCGGCATCCCCCAGGACCTCAAGACCAAGGTCAACGTGCAGGTCATCTACCTATACGAGAATGACCTG
GATGAGTTCCCCATCAACCTGCCCCGCTCCCTCCGGGAGCTGCACCTGCAGGACAACAATGTGCGCAC
CATTGCCAGGGACTCGCTGGCCCGCATCCCGCTGCTGGAGAAGCTGCACCTGGATGACAACTCCGTGT
CCACCGTCAGCATTGAGGAGGACGCCTTCGCCGACAGCAAACAGCTCAAGCTGCTCTTCCTGAGCCGG
AACCACCTGAGCAGCATCCCCTCGGGGCTGCCGCACACGCTGGAGGAGCTGCGGCTGGATGACAACCG
CATCTCCACCATCCCGCTGCATGCCTTCAAGGGCCTCAACAGCCTGCGGCGCCTGGTGCTGGACGGTA
ACCTGCTGGCCAACCAGCGCATCGCCGACGACACCTTCAGCCGCCTACAGAACCTCACAGAGCTCTCG
CTGGTGCGCAATTCGCTGGCCGCGCCACCCCTCAACCTGCCCAGCGCCCACCTGCAGAAGCTCTACCT
GCAGGACAATGCCATCAGCCACATCCCCTACAACACGCTGGCCAAGATGCGTGAGCTGGAGCGGCTGG
ACCTGTCCAACAACAACCTGACCACGCTGCCCCGCGGCCTGTTCGACGACCTGGGGAACCTGGCCCAG
CTGCTGCTCAGGAACAACCCTTGGTTTTGTGGCTGCAACCTCATGTGGCTGCGGGACTGGGTGAAGGC
ACGGGCGGCCGTGGTCAACGTGCGGGGCCTCATGTGCCAGGGCCCTGAGAAGGTCCGGGGCATGGCCA
TCAAGGACATTACCAGCGAGATGGACGAGTGTTTTGAGACGGGGCCGCAGGGCGGCGTGGCCAATGCG
GCTGCCAAGACCACGGCCAGCAACCACGCCTCTGCCACCACGCCCCAGGGTTCCCTGTTTACCCTCAA
GGCCAAAAGGCCAGGGCTGCGCCTCCCCGACTCCAACATTGACTACCCCATGGCCACGGGTGATGGCG
CCAAGACCCTGGCCATCCACGTGAAGGCCCTGACGGCAGACTCCATCCGCATCACGTGGAAGGCCACG
CTCCCCGCCTCCTCTTTCCGGCTCAGTTGGCTGCGCCTGGGCCACAGCCCAGCCGTGGGCTCCATCAC
GGAGACCTTGGTGCAGGGGGACAAGACAGAGTACCTGCTGACAGCCCTGGAGCCCAAGTCCACCTACA
TCATCTGCATGGTCACCATGGAGACCAGCAATGCCTATGTAGCTGATGAGACACCCGTGTGTGCCAAG
GCAGAGACAGCCGACAGCTATGGCCCTACCACCACACTCAACCAGGAGCAGAACGCTGGCCCCATGGC
GAGCCTGCCCCTGGCGGGCATCATCGGCGGGGCAGTGGCTCTGGTCTTCCTCTTCCTGGTCCTGGGGG
CCATCTGCTGGTACGTGCACCAGGCTGGCGAGCTGCTGACCCGGGAGAGGGCCTACAACCGGGGCAGC
AGGAAAAAGGATGACTATATGGAGTCAGGGACCAAGAAGGATAACTCCATCCTGGAAATCCGCGGCCC
TGGGCTGCAGATGCTGCCCATCAACCCGTACCGCGCCAAAGAGGAGTACGTGGTCCACACTATCTTCC
CCTCCAACGGCAGCAGCCTCTGCAAGGCCACACACACCATTGGCTACGGCACCACGCGGGGCTACCGG
GACGGCGGCATCCCCGACATAGACTACTCCTACACA**TGA**TGCCCGCCCACCCGGGCTGCCCCGCCTCA
GCCCCAGCTGCCCTGGCGTGGCCATGTGGCTTTGCCCAGCCTGCTGCAATCCAAGAGAGCAAGGAAGA
GAAATTCCATGGGTGACTTTCCTCCGCAGAAAGCAAAGTTTGGGGAGGGCTGACGATTTTGTAGAACA
CAACAGTGACAATTTTTTTAAAAGAATAGAAGGCAGGAGGGGGAATTCGACATTGTTGAAGACATAA
TTTATACCAAGTTATGCCAGTTGGGGAGGGAAGGACTAAAAATAATATTGCAGGCAGGGCTGGGTTGG
GTTTTTTTTTTTCCCCCCTGAACTGGAAGGATACTACCTGTACAACATCTGTGGACACCTCATGCTCT
GTTCAAGGCCATCACAAAGGAACCGCCAGGGAGAAGCAGCCGGCTCTCAAAGCTCCCACGCAGCTCTC
CCGCCACTGGCCACTCGCTGGCGACCCGATGGAAGGTTTTCAGGCTCCTCACAAAGGAGAGAGGGAAG
AAAAGATCTTTTGCCCTGGAGATATGGTCCTGAAATCTCTCCCCTGGCTTATTCCATACCATTTCCCT
TGCAGATTTGCAGAAACATGGCATCTTTCACTGCATTCTTTGAACAATCATGTAGTCGATTAAAAAAA
AAAACAAACTTTTTTTTCCTAGGCTGAAGCCCTCTTCAGTTCCATGCACCACGCTCCGTAGAAGCCCC
GGCGGAAGCCGTAGCTTTCCCTGCCACCTGGAGGTGCATCTGTCTGCCTGTCTATCCCTGTCGCGGTG
TCTCTAAGTACAGATGGGTAGATAGAGCCACATGCACGGTCCTTACCGTTCTTCTTGGGTCAGTTCTT
ACCATTTCCTGAACAATAGAATTGTGAAAGTGTTAAAAA

# FIGURE 36

MVVAHPTATATTTPTATVTATVVMTTATMDLRDWLFLCYGLIAFLTEVIDSTTCPSVCRCDNG
FIYCNDRGLTSIPADIPDDATTLYLQNNQINNAGIPQDLKTKVNVQVIYLYENDLDEFPINLP
RSLRELHLQDNNVRTIARDSLARIPLLEKLHLDDNSVSTVSIEEDAFADSKQLKLLFLSRNHL
SSIPSGLPHTLEELRLDDNRISTIPLHAFKGLNSLRRLVLDGNLLANQRIADDTFSRLQNLTE
LSLVRNSLAAPPLNLPSAHLQKLYLQDNAISHIPYNTLAKMRELERLDLSNNNLTTLPRGLFD
DLGNLAQLLLRNNPWFCGCNLMWLRDWVKARAAVVNVRGLMCQGPEKVRGMAIKDITSEMDEC
FETGPQGGVANAAAKTTASNHASATTPQGSLFTLKAKRPGLRLPDSNIDYPMATGDGAKTLAI
HVKALTADSIRITWKATLPASSFRLSWLRLGHSPAVGSITETLVQGDKTEYLLTALEPKSTYI
ICMVTMETSNAYVADETPVCAKAETADSYGPTTTLNQEQNAGPMASLPLAGIIGGAVALVFLF
LVLGAICWYVHQAGELLTRERAYNRGSRKKDDYMESGTKKDNSILEIRGPGLQMLPINPYRAK
EEYVVHTIFPSNGSSLCKATHTIGYGTTRGYRDGGIPDIDYSYT

**Important features of the protein:**

**Transmembrane domain:**

amino acids 552-573

**N-glycosylation sites.**

amino acids 249-252, 305-308, 642-645

**Leucine zipper pattern.**

amino acids 182-203, 299-320

**Phospholipase A2 aspartic acid active site.**

amino acids 57-67

# FIGURE 37

GGTGACTGAAGCGAGCCTGGCCTCTTGCATCCTCCGCCTGTGTACCTCCCTCCCCTTTTTTTCCGCCT
TCTGCCAGCAGAAGCAGCAGCCGCAGCACCTGAGCCGCTACTGCCGCTCACTCAGGACAACGCT**ATG**G
CTGAGCCTGGGCACAGCCACCATCTCTCCGCCAGAGTCAGGAGAAGAACTGAGAGGCGCATACCCCGG
CTGTGGCGGCTGCTGCTCTGGGCTGGGACCGCCTTCCAGGTGACCCAGGGAACGGGACCGGAGCTTCA
TGCCTGCAAAGAGTCTGAGTACCACTATGAGTACACGGCGTGTGACAGCACGGGTTCCAGGTGGAGGG
TCGCCGTGCCGCATACCCCGGGCCTGTGCACCAGCCTGTCTGACCCCGTCAAGGGCACCGAGTGCTCC
TTCTCCTGCAACGCCGGGGAGTTTCTGGATATGAAGGACCAGTCATGTAAGCCATGCGCTGAGGGCCG
CTACTCCCTCGGCACAGGCATTCGGTTTGATGAGTGGGATGAGCTGCCCCATGGCTTTGCCAGCCTCT
CAGCCAACATGGAGCTGGATGACAGTGCTGCTGAGTCCACCGGGAACTGTACTTCGTCCAAGTGGGTT
CCCCGGGGCGACTACATCGCCTCCAACACGGACGAATGCACAGCCACACTGATGTACGCCGTCAACCT
GAAGCAATCTGGCACCGTTAACTTCGAATACTACTATCCAGACTCCAGCATCATCTTTGAGTTTTTCG
TTCAGAATGACCAGTGCCAGCCCAATGCAGATGACTCCAGGTGGATGAAGACCACAGAGAAAGGATGG
GAATTCCACAGTGTGGAGCTAAATCGAGGCAATAATGTCCTCTATTGGAGAACCACAGCCTTCTCAGT
ATGGACCAAAGTACCCAAGCCTGTGCTGGTGAGAAACATTGCCATAACAGGGGTGGCCTACACTTCAG
AATGCTTCCCCTGCAAACCTGGCACGTATGCAGACAAGCAGGGCTCCTCTTTCTGCAAACTTTGCCCA
GCCAACTCTTATTCAAATAAAGGAGAAACTTCTTGCCACCAGTGTGACCCTGACAAATACTCAGAGAA
AGGATCTTCTTCCTGTAACGTGCGCCCAGCTTGCACAGACAAAGATTATTTCTACACACACACGGCCT
GCGATGCCAACGGAGAGACACAACTCATGTACAAATGGGCCAAGCCGAAAATCTGTAGCGAGGACCTT
GAGGGGGCAGTGAAGCTGCCTGCCTCTGGTGTGAAGACCCACTGCCCACCCTGCAACCCAGGCTTCTT
CAAAACCAACAACAGCACCTGCCAGCCCTGCCCATATGGTTCCTACTCCAATGGCTCAGACTGTACCC
GCTGCCCTGCAGGGACTGAACCTGCTGTGGGATTTGAATACAAATGGTGGAACACGCTGCCCACAAAC
ATGGAAACGACCGTTCTCAGTGGGATCAACTTCGAGTACAAGGGCATGACAGGCTGGGAGGTGGCTGG
TGATCACATTTACACAGCTGCTGGAGCCTCAGACAATGACTTCATGATTCTCACTCTGGTTGTGCCAG
GATTTAGACCTCCGCAGTCGGTGATGGCAGACACAGAGAATAAAGAGGTGGCCAGAATCACATTTGTC
TTTGAGACCCTCTGTTCTGTGAACTGTGAGCTCTACTTCATGGTGGGTGTGAATTCTAGGACCAACAC
TCCTGTGGAGACGTGGAAAGGTTCCAAAGGCAAACAGTCCTATACCTACATCATTGAGGAGAACACTA
CCACGAGCTTCACCTGGGCCTTCCAGAGGACCACTTTTCATGAGGCAAGCAGGAAGTACACCAATGAC
GTTGCCAAGATCTACTCCATCAATGTCACCAATGTTATGAATGGCGTGGCCTCCTACTGCCGTCCCTG
TGCCCTAGAAGCCTCTGATGTGGGCTCCTCCTGCACCTCTTGTCCTGCTGGTTACTATATTGACCGAG
ATTCAGGAACCTGCCACTCCTGCCCCCCTAACACAATTCTGAAAGCCCACCAGCCTTATGGTGTCCAG
GCCTGTGTGCCCTGTGGTCCAGGGACCAAGAACAACAAGATCCACTCTCTGTGCTACAATGATTGCAC
CTTCTCACGCAACACTCCAACCAGGACTTTCAACTACAACTTCTCCGCTTTGGCAAACACCGTCACTC
TTGCTGGAGGGCCAAGCTTCACTTCCAAAGGGTTGAAATACTTCCATCACTTTACCCTCAGTCTCTGT
GGAAACCAGGGTAGGAAAATGTCTGTGTGCACCGACAATGTCACTGACCTCCGGATTCCTGAGGGTGA
GTCAGGGTTCTCCAAATCTATCACAGCCTACGTCTGCCAGGCAGTCATCATCCCCCCAGAGGTGACAG
GCTACAAGGCCGGGGTTTCCTCACAGCCTGTCAGCCTTGCTGATCGACTTATTGGGGTGACAACAGAT
ATGACTCTGGATGGAATCACCTCCCCAGCTGAACTTTTCCACCTGGAGTCCTTGGGAATACCGGACGT
GATCTTCTTTTATAGGTCCAATGATGTGACCCAGTCCTGCAGTTCTGGGAGATCAACCACCATCCGCG
TCAGGTGCAGTCCACAGAAAACTGTCCCTGGAAGTTTGCTGCTGCCAGGAACGTGCTCAGATGGGACC
TGTGATGGCTGCAACTTCCACTTCCTGTGGGAGAGCGCGGCTGCTTGCCCGCTCTGCTCAGTGGCTGA
CTACCATGCTATCGTCAGCAGCTGTGTGGCTGGGATCCAGANGACTACTTACGTGTGNCGAGAACCCA
AGCTATGCTCTGGTGGCATTTCTCTGCCTGAGCAGAGAGTCACCATCTGCAAAACCATAGATTTCTGG
CTGAAAGTGGGCATCTCTGCAGGCACCTGTACTGCCATCCTGCTCACCGTCTTGACCTGCTACTTTTG
GAAAAAGAATCAAAAACTAGAGTACAAGTACTCCAAGCTGGTGATGAATGCTACTCTCAAGGACTGTG
ACCTGCCAGCAGCTGACAGCTGCGCCATCATGGAAGGCGAGGATGTAGAGGACGACCTCATCTTTACC
AGCAAGAAGTCACTTTTTGGGAAGATCAAATCATTTACCTCCAAGAGGACTCCTGATGGATTTGACTC
AGTGCCGCTGAAGACATCCTCAGGAGGCCCAGACATGGACCTG**TGA**GAGGCACTGCCTGCCTCACCTG
CCTCCTCACCTTGCATAGCACCTTTGCAAGCCTGCGGCGATTTGGGTGCCAGCATCCTGCAACACCCA
CTGCTGGAAATCTCTTCATTGTGGCCTTATCAGATGTTTGAATTTCAGATCTTTTTTTATAGAGTACC
CAAACCCTCCTTTCTGCTTGCCTCAAACCTGCCAAATATACCCACATTTTTTTTTAAAAAAAAAAAA
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAA

# FIGURE 38

MAEPGHSHHLSARVRRRTERRIPRLWRLLLWAGTAFQVTQGTGPELHACKESEYHYEYTACDS
TGSRWRVAVPHTPGLCTSLSDPVKGTECSFSCNAGEFLDMKDQSCKPCAEGRYSLGTGIRFDE
WDELPHGFASLSANMELDDSAAESTGNCTSSKWVPRGDYIASNTDECTATLMYAVNLKQSGTV
NFEYYYPDSSIIFEFFVQNDQCQPNADDSRWMKTTEKGWEFHSVELNRGNNVLYWRTTAFSVW
TKVPKPVLVRNIAITGVAYTSECFPCKPGTYADKQGSSFCKLCPANSYSNKGETSCHQCDPDK
YSEKGSSSCNVRPACTDKDYFYTHTACDANGETQLMYKWAKPKICSEDLEGAVKLPASGVKTH
CPPCNPGFFKTNNSTCQPCPYGSYSNGSDCTRCPAGTEPAVGFEYKWWNTLPTNMETTVLSGI
NFEYKGMTGWEVAGDHIYTAAGASDNDFMILTLVVPGFRPPQSVMADTENKEVARITFVFETL
CSVNCELYFMVGVNSRTNTPVETWKGSKGKQSYTYIIEENTTTSFTWAFQRTTFHEASRKYTN
DVAKIYSINVTNVMNGVASYCRPCALEASDVGSSCTSCPAGYYIDRDSGTCHSCPPNTILKAH
QPYGVQACVPCGPGTKNNKIHSLCYNDCTFSRNTPTRTFNYNFSALANTVTLAGGPSFTSKGL
KYFHHFTLSLCGNQGRKMSVCTDNVTDLRIPEGESGFSKSITAYVCQAVIIPPEVTGYKAGVS
SQPVSLADRLIGVTTDMTLDGITSPAELFHLESLGIPDVIFFYRSNDVTQSCSSGRSTTIRVR
CSPQKTVPGSLLLPGTCSDGTCDGCNFHFLWESAAACPLCSVADYHAIVSSCVAGIQXTTYVX
REPKLCSGGISLPEQRVTICKTIDFWLKVGISAGTCTAILLTVLTCYFWKKNQKLEYKYSKLV
MNATLKDCDLPAADSCAIMEGEDVEDDLIFTSKKSLFGKIKSFTSKRTPDGFDSVPLKTSSGG
PDMDL

**Important features of the protein:**

**N-glycosylation sites:**

amino acids 153-156, 390-393, 391-394, 404-407, 544-547, 576-579, 672-675, 717-720, 947-950

**cAMP- and cGMP-dependent protein kinase phosphorylation sites:**

amino acids 15-18, 563-566, 709-712

**Casein kinase II phosphorylation sites:**

amino acids 42-45, 59-62, 81-84, 146-149, 168-171, 282-285, 331-334, 340-343, 431-434, 449-452, 465-468, 523-526, 557-560, 761-764, 780-783, 835-838, 860-863, 893-896, 949-952

**Tyrosine kinase phosphorylation sites:**

amino acids 50-56, 109-116

**N-myristoylation sites:**

amino acids 77-82, 88-93, 152-157, 268-273, 288-293, 320-325, 400-405, 405-410, 414-419, 463-468, 599-604, 616-621, 634-639, 644-649, 839-844, 874-879, 912-917, 916-921

**Amidation site:**

amino acids 707-710

**Cell attachment sequence:**

amino acids 162-164

# FIGURE 39

GGGAAGGGGTTCTGGGCTGCCGCAGGCACACAGGCCAGAGCTTCGTGGATACCTGCAGGGCCC
AAAGGTCCCTCCCTGTTTTGAAGAGTGAGTGATGGCTATGAGGTAGCGGCCAGGCTGATCACC
CCTGCGTTGGCTGGAGGCAGAATTCTGTAAATCCTCGCCAAGTCTTTCTCCAGGCCACTGGTT
AGCTCATCTCAGCCTCCTCTGGGAGCATCAACACCAACATGGCACAGGGGACTGCAGTGGTGT
GCTTTGGACCTGTGTACCCACCCAAGGCTAAAGGCAGAGCCAGGTGACTTTGCGGGGGTCTCT
TCTCTAGGATTATCTGTACTTCCCCTCTGTCCTCTTTTACTACGGGAGATCGAGCTAGCTATA
ACCCACCTTCTTTCATGAGAACCACACTAAATTGCAAAAATTATCCCAGTGCTGGAGGAGGGC
AGCAGGTTGAGATTATGTTGGCAGGAAGAATGTTGGCATTGATTGGCACGCAGGGGACGAGAG
CTGCTTTGTGCTTTAAAGGAGCCAAGTTACACCCTGTTTAACCCTGCCTTCAAAGGGACGACT
CTGTAAGATTCTCTGCTACTTATTCAAGTTGACACG**ATG**CCCTTCACACTCCACCTGAGGTCC
CGCCTTCCCTCTGCCATAAGGAGTTTGATTCTACAAAAGAAACCAAACATCAGAAATACATCC
AGCATGGCTGGAGAGCTCCGACCAGCCAGCCTGGTGGTCCTGCCCAGGTCCCTTGCTCCAGCT
TTTGAAAGATTCTGCCAGGTCAACACTGGTCCTCTACCCCTGCTGGGCCAGAGTGAGCCAGAA
AAGTGGATGCTGCCCCCTCAAGGTGCTATCTCAGAGACCAGGATGGGCCATCCCCAGTTCTGG
AAATACGAGTTCGGTGCCTGCACCGGTAGCCTGGCTTCGCTGGAGCAGTACTCGGAGCAGCTG
AAGGACATGGTGGCCTTCTTCCTGGGCTGCAGCTTCTCCCTGGAGGAGGCCTTGGAGAAAGCG
GGGCTCCCCAGAAGAGACCCAGCAGGTCACAGCCAGGCGGGTGCATACAAGACAACAGTGCCT
TGTGTTACCCATGCTGGCTTCTGCTGCCCTCTGGTGGTCACGATGAGGCCCATTCCCAAGGAC
AAGCTGGAAGGGCTGGTGCGGGCCTGCTGCTCCCTCGGAGGTGAGCAGGGGCAACCTGTTCAC
ATGGGCGACCCAGAACTGTTGGGAATCAAAGAGCTTTCCAAACCTGCCTACGGGGATGCCATG
GTGTGTCCCCCAGGGGAGGTTCCAGTGTTCTGGCCTTCTCCGCTGACCAGTCTCGGAGCTGTC
AGCAGCTGTGAGACCCCACTGGCTTTTGCCAGCATCCCAGGCTGCACAGTTATGACTGACCTG
AAGGATGCAAAGGCTCCACCTGGTTGTCTCACCCCAGAGAGAATTCCAGAGGTCCATCACATT
TCCCAAGATCCTCTGCACTACAGCATCGCGTCAGTCTCTGCTTCTCAGAAGATCAGAGAACTA
GAGTCTATGATCGGCATAGACCCAGGGAACCGGGGGATTGGGCACCTGCTCTGTAAAGATGAG
CTGCTGAAGGCCTCTCTCTCGCTGTCCCATGCCCGCTCAGTGCTCATCACCACTGGGTTCCCC
ACACATTTCAATCATGAGCCTCCAGAAGAGACAGATGGCCCACCAGGAGCTGTTGCTCTGGTT
GCCTTCCTGCAGGCCTTGGAGAAGGAGGTCGCCATAATCGTTGACCAGAGAGCCTGGAACTTG
CACCAGAAGATTGTTGAAGATGCTGTTGAGCAAGGTGTTCTGAAGACGCAGATCCCGATATTA
ACTTACCAAGGTGGATCAGTGGAAGCTGCTCAGGCATTCCTGTGCAAAAATGGGGACCCGCAG
ACACCTAGATTTGACCACCTGGTGGCCATAGAGCGTGCCGGAAGAGCTGCTGATGGCAATTAC
TACAATGCAAGGAAGATGAACATCAAGCACTTGGTTGACCCCATTGACGATCTTTTTCTTGCT
GCGAAGAAGATTCCTGGAATCTCATCAACTGGAGTCGGTGATGGAGGCAACGAGCTTGGGATG
GGTAAAGTCAAGGAGGCTGTGAGGAGGCACATACGGCACGGGGATGTCATCGCCTGCGACGTG
GAGGCTGACTTTGCCGTCATTGCTGGTGTTTCTAACTGGGGAGGCTATGCCCTGGCCTGCGCA
CTCTACATCCTGTACTCATGTGCTGTCCACAGTCAGTACCTGAGGAAAGCAGTCGGACCCTCC
AGGGCACCTGGAGATCAGGCCTGGACTCAGGCCCTCCCGTCGGTCATTAAGGAAGAAAAAATG
CTGGGCATCTTGGTGCAGCACAAAGTCCGGAGTGGCGTCTCGGGCATCGTGGGCATGGAGGTG
GATGGGCTGCCCTTCCACAACACCCACGCCGAGATGATCCAGAAGCTGGTGGACGTCACCACG
GCACAGGTG**TAA**CCGTCCATGTTCCGTGTGAGCAGAGTCCCTACCAACGGGCAGGTCTGCATC
CGGGGAGAATGCAGCTGCTTCTGGCGACAATCCTGCTAGTAAACACTGGTCTTCGGTGAGCAA
CGAACACTCGCCTGGCCTGGGAAACTGCATGCCCACTTTCTGGGAGGGGTTAGTGCAGGTGCC
GTGGACAAAGGACAACATTTCTCTGGGGCTTTTTAACTTTTATTCCTAAGACTCTAAAGGCGT
TGATTTCAACCCTCCTTCACTCTGGCTTCTTCAGGCAACCCACGTGGTCTCCTATGAGAATCT
TCTCGACAGTTACTTATGGGGACACTTGTGAACAATTAACTGCCAGGGCAGAGCATGAGAACA
AACATTCCCAGGCCATGTAGGATAGGATACTCCAGACTCCAGTCATCCTCCCCCATCCATGGT
TTCTGTTACTCATGGTTTCAGTTACTCATAGCCAACTGCAGACCGAAAATACTAAATGAAAAA
TTTCAGAAATAAACAACTCTTAAGTTTTAAAAAAAAA

# FIGURE 40

MPFTLHLRSRLPSAIRSLILQKKPNIRNTSSMAGELRPASLVVLPRSLAPAFERFCQVNTGPL
PLLGQSEPEKWMLPPQGAISETRMGHPQFWKYEFGACTGSLASLEQYSEQLKDMVAFFLGCSF
SLEEALEKAGLPRRDPAGHSQAGAYKTTVPCVTHAGFCCPLVVTMRPIPKDKLEGLVRACCSL
GGEQGQPVHMGDPELLGIKELSKPAYGDAMVCPPGEVPVFWPSPLTSLGAVSSCETPLAFASI
PGCTVMTDLKDAKAPPGCLTPERIPEVHHISQDPLHYSIASVSASQKIRELESMIGIDPGNRG
IGHLLCKDELLKASLSLSHARSVLITTGFPTHFNHEPPEETDGPPGAVALVAFLQALEKEVAI
IVDQRAWNLHQKIVEDAVEQGVLKTQIPILTYQGGSVEAAQAFLCKNGDPQTPRFDHLVAIER
AGRAADGNYYNARKMNIKHLVDPIDDLFLAAKKIPGISSTGVGDGGNELGMGKVKEAVRRHIR
HGDVIACDVEADFAVIAGVSNWGGYALACALYILYSCAVHSQYLRKAVGPSRAPGDQAWTQAL
PSVIKEEKMLGILVQHKVRSGVSGIVGMEVDGLPFHNTHAEMIQKLVDVTTAQV

**Signal peptide:**

amino acids 1-17

**Transmembrane domain:**

amino acids 358-378, 517-539

**N-glycosylation site.**

amino acids 28-32

**Tyrosine kinase phosphorylation site.**

amino acids 444-452

**N-myristoylation site.**

amino acids 98-104, 102-108, 123-129, 149-155, 181-187, 190-196, 238-244, 308-314, 399-405, 413-419, 448-454, 477-483, 482-488, 487-493

**Prokaryotic membrane lipoprotein lipid attachment site.**

amino acids 233-244, 531-542

# FIGURE 41

CTTTCCTGTTTTATCCGCAGCCCTTTTCTTCTTTGAGTTAGTAAAGATTTATTCTGTAACCTG
ACACTCATCTGGCCCTTTGCAGTTTGCCAGCCATATTCCCATGTGATTTCCCACTGGATCCAG
GCCCCCATCCGGCTGGCAGGAGGGGGCTCTGACGTACAGGTTGGAAATCAGAAGTCTGTGAGA
GCGCGGGAGTGCATGGCAGCTCTGGGTCCCAGACCTGGCCCGACCCCTCTGCTTCACCTCCAG
CTCTGCTGCTCCTCTACTCTTGGGTCGAGATCCCTTTGGAGCCACAGCGAGGAACCCTGTGGT
CCTCAGGCAGGTGTACCTTGAGTCAGCCAGGAGCCCTCTTTTCCTGTGTCAAAGCCTGCCCTC
GGGCTCTGCTCACCTCTGGTGACCCTCCAAGATGCCCCTGCCCTCAGTTTCCCCTCATGATCT
GGCCTCTGCCCCCTTCTCTAGCCACAGCCTCTAGTACACTTTAGCAATACCACCAGACTAGTT
AGAGTTCCCCACTCACCAAGCAAGAC**ATG**CAGTTTCATGCCTCTGTGCCTTCGCTCATGCTGT
TTCTTCCGACTGGAATGCCTTCCCCTGCTCCTCCTGCCTTGTCTGCCTGGCAAGTTCATCTCT
CACGATCCCCTCAAAGGCCCCCTCCTCCAGGAAGGCAACCCCTGTGCCCCTCCCCTCCAGGCT
ACCTCTGCACTTTGTCAATGCTTCTCTTGTGGCACTTATCACACTGTATTTTACTTGTTTACA
TGTTTGTCTCCCCTTCTAGACTG**TGA**ATCCTTAAGGGCATGGACTGTATCTTATGCATCTCTG
TATTTCTGCGCCTAGCACGGTGCCTAGCACACAGTAGGCGCTCAATAAATGTTGAATGAATGA
ATGATTT

# FIGURE 42

MQFHASVPSLMLFLPTGMPSPAPPALSAWQVHLSRSPQRPPPPGRQPLCPSPPGYLCTLSMLL
LWHLSHCILLVYMFVSPSRL

**Important features of the protein:**

**Signal peptide:**

amino acids 1-22

**Microbodies C-terminal targeting signal.**

amino acids 81-83

# FIGURE 43

GTTTCCAACAAGGATGATATGAAGACTTCCCTGAAGAAAGTTGTGAAGGGACCTCCTACGAG**A**
**TG**ATGATGCAGTGTGTGTCCCGCATGTTGGCCCACCCCCTGCATGTCATCTCAATGCGCTGCA
TGGTCCAGTTTGTGGGACGGGAGGCCAAGTACAGTGGTGTGCTGAGCTCCATTGGGAAGATTT
TCAAAGAGGAAGGGCTGCTGGGATTCTTCGTTGGATTAATCCCTCACCTCCTGGGCGATGTGG
TTTTCTTGTGGGGCTGTAACCTGCTGGCCCACTTCATCAATGCCTACCTGGTGGATGACAGCT
TCAGCCAGGCCCTGGCCATCCGGAGCTATACCAAGTTCGTGATGGGGATTGCAGTGAGCATGC
TGACCTACCCCTTCCTGCTAGTTGGCGACCTCATGGCTGTGAACAACTGCGGGCTGCAAGCTG
GGCTCCCCCCTTACTCCCCAGTGTTCAAATCCTGGATTCACTGCTGGAAGTACCTGAGTGTGC
AGGGCCAGCTCTTCCGAGGCTCCAGCCTGCTTTTCCGCCGGGTGTCATCAGGATCATGCTTTG
CCCTGGAG**TAA**CCTGAATCATCTAAAAAACACGGTCTCAACCTGGCCACTGTGGGTGAGGCCT
GACCACCTTGGGACACCTGCAAGACGACTCCAACCCAACAACAACCAGATGTGCTCCAGCCCA
GCCGGGCTTCAGTTCCATATTTGCCATGTGTCTGTCCAGATGTGGGGTTGAGCGGGGGTGGGG
CTGCACCCAGTGGATTGGGTCACCCGGCAGACCTAGGGAAGGTGAGGCGAGGTGGGGAGTTGG
CAGAATCCCCATACCTCGCAGATTTGCTGAGTCTGTCTTGTGCAGAGGGCCAGAGAATGGCTT
ATGGGGGCCCAGGTTGGATGGGGAAAGGCTAATGGGGTCAGACCCCACCCCGTCTACCCCTCC
AGTCAGCCCAGCGCCCATCCTGCAGCTCAGCTGGGAGCATCATTCTCCTGCTTTGTACATAGG
GTGTGGTCCCCTGGCACGTGGCCACCATCATGTCTAGGCCTATGCTAGGAGGCAAATGGCCAG
GCTCTGCCTGTGTTTTTCTCAACACTACTTTTCTGATATGAGGGCAGCACCTGCCTCTGAATG
GGAAATCATGCAACTACTCAGAATGTGTCCTCCTCATCTAATGCTCATCTGTTTAATGGTGAT
GCCTCGCGTACAGGATCTGGTTACCTGTGCAGTTGTGAATACCCAGAGGTTGGGCAGATCAGT
GTCTCTAGTCCTACCCAGTTTTAAAGTTCATGGTAAGATTTGACCTCATCTCCCGCAAATAAA
TGTATTGGTGATTTGGAAAAAAAAAAAAAAAAA

# FIGURE 44

MMMQCVSRMLAHPLHVISMRCMVQFVGREAKYSGVLSSIGKIFKEEGLLGFFVGLIPHLLGDV
VFLWGCNLLAHFINAYLVDDSFSQALAIRSYTKFVMGIAVSMLTYPFLLVGDLMAVNNCGLQA
GLPPYSPVFKSWIHCWKYLSVQGQLFRGSSLLFRRVSSGSCFALE

**Important features of the protein:**

**Signal peptide:**

amino acids 1-18

**Transmembrane domains:**

amino acids 51-72, 97-114

**cAMP- and cGMP-dependent protein kinase phosphorylation site.**

amino acids 160-163

**N-myristoylation sites.**

amino acids 34-39, 100-105, 123-128, 165-170

# FIGURE 45

GCTCACTCTTTGGGTCCACACTGCCTTTATGAGCTGTAACACTCACTGGGAATGTCTGCAGCT
TCACTCCTGAAGCCAGCGAGACCACGAACCCACCAGGAGGAACAAACAACTCCAGACGCGCAG
CCTTAAGAGCTGTAACACTCACCGCGAAGGTCTGCAGCTTCACTCCTGAGCCAGCCAGACCAC
GAACCCACCAGAAGGAAGAAACTCCAAACACATCCGAACATCAGAAGGAGCAAACTCGTGACA
CGCCACCTTTAAGAACCGTGACACTCAACGCTAGGGTCCGCGGCTTCATTCTTGAAGTCAGTG
AGACCAAGAACCCACCAATTCCGGACACGGCAAAGTAACATCCTAGAC**ATG**GCTTTAGAGATC
CACATGTCAGACCCCATGTGCCTCATCGAGAACTTTAATGAGCAGCTGAAGGTTAATCAGGAA
GCTTTGGAGATCCTGTCTGCCATTACGCAACCTGTAGTTGTGGTAGCGATTGTGGGCCTCTAT
CGCACTGGCAAATCCTACCTGATGAACAAGCTGGCTGGGAAGAACAAGGGCTTCTCTGTTGCA
TCTACGGTGCAGTCTCACACCAAGGGAATTTGGATATGGTGTGTGCCTCATCCCAACTGGCCA
AATCACACATTAGTTCTGCTTGACACCGAGGGCCTGGGAGATGTAGAGAAGGCTGACAACAAG
AATGATATCCAGATCTTTGCACTGGCACTCTTACTGAGCAGCACCTTTGTGTACAATACTGTG
AACAAAATTGATCAGGGTGCTATCGACCTACTGCACAATGTGACAGAACTGACAGATCTGCTC
AAGGCAAGAAACTCACCTGACCTTGACAGGGTTGAAGATCCTGCTGACTCTGCGAGCTTCTTC
CCAGACTTAGTGTGGACTCTGAGAGATTTCTGCTTAGGCCTGGAAATAGATGGGCAACTTGTC
ACACCAGATGAATACCTGGAGAATTCCCTAAGGCCAAAGCAAGGTAGTGATCAAAGAGTTCAA
AATTTCAATTTGCCCCGTCTGTGTATACAGAAGTTCTTTCCAAAAAAGAAATGCTTTATCTTT
GACTTACCTGCTCACCAAAAAAAGCTTGCCCAACTTGAAACACTGCCTGATGATGAGCTAGAG
CCTGAATTTGTGCAACAAGTGACAGAATTCTGTTCCTACATCTTTAGCCATTCTATGACCAAG
ACTCTTCCAGGTGGCATCATGGTCAATGGATCTCGTCTAAAGAACCTGGTGCTGACCTATGTC
AATGCCATCAGCAGTGGGGATCTGCCTTGCATAGAGAATGCAGTCCTGGCCTTGGCTCAGAGA
GAGAACTCAGCTGCAGTGCAAAAGGCCATTGCCCACTATGACCAGCAAATGGGCCAGAAAGTG
CAGCTGCCCATGGAAACCCTCCAGGAGCTGCTGGACCTGCACAGGACCAGTGAGAGGGAGGCC
ATTGAAGTCTTCATGAAAAACTCTTTCAAGGATGTAGACCAAAGTTTCCAGAAAGAATTGGAG
ACTCTACTAGATGCAAAACAGAATGACATTTGTAAACGGAACCTGGAAGCATCCTCGGATTAT
TGCTCGGCTTTACTTAAGGATATTTTTGGTCCTCTAGAAGAAGCAGTGAAGCAGGGAATTTAT
TCTAAGCCAGGAGGCCATAATCTCTTCATTCAGAAAACAGAAGAACTGAAGGCAAAGTACTAT
CGGGAGCCTCGGAAAGGAATACAGGCTGAAGAAGTTCTGCAGAAATATTTAAAGTCCAAGGAG
TCTGTGAGTCATGCAATATTACAGACTGACCAGGCTCTCACAGAGACGGAAAAAAAGAAGAAA
GAGGCACAAGTGAAAGCAGAAGCTGAAAAGGCTGAAGCGCAAAGGTTGGCGGCGATTCAAAGG
CAGAACGAGCAAATGATGCAGGAGAGGGAGAGACTCCATCAGGAACAAGTGAGACAAATGGAG
ATAGCCAAACAAAATTGGCTGGCAGAGCAACAGAAAATGCAGGAACAACAGATGCAGGAACAG
GCTGCACAGCTCAGCACAACATTCCAAGCTCAAAATAGAAGCCTTCTCAGTGAGCTCCAGCAC
GCCCAGAGGGCTGTTAATAACGATGATCCATGTGTTTTACTC**TAA**AGTGCTAAATATGGGAGT
TTCCTTTTTTTACTCTTTGTCACTGATGACACAACAGAAAAGAAACTGTAGACCTTGGGACAA
TCAACATTTAAATAAACTTTATAATTATTAAA

# FIGURE 46

MALEIHMSDPMCLIENFNEQLKVNQEALEILSAITQPVVVVAIVGLYRTGKSYLMNKLAGKNK
GFSVASTVQSHTKGIWIWCVPHPNWPNHTLVLLDTEGLGDVEKADNKNDIQIFALALLLSSTF
VYNTVNKIDQGAIDLLHNVTELTDLLKARNSPDLDRVEDPADSASFFPDLVWTLRDFCLGLEI
DGQLVTPDEYLENSLRPKQGSDQRVQNFNLPRLCIQKFFPKKKCFIFDLPAHQKKLAQLETLP
DDELEPEFVQQVTEFCSYIFSHSMTKTLPGGIMVNGSRLKNLVLTYVNAISSGDLPCIENAVL
ALAQRENSAAVQKAIAHYDQQMGQKVQLPMETLQELLDLHRTSEREAIEVFMKNSFKDVDQSF
QKELETLLDAKQNDICKRNLEASSDYCSALLKDIFGPLEEAVKQGIYSKPGGHNLFIQKTEEL
KAKYYREPRKGIQAEEVLQKYLKSKESVSHAILQTDQALTETEKKKKEAQVKAEAEKAEAQRL
AAIQRQNEQMMQERERLHQEQVRQMEIAKQNWLAEQQKMQEQQMQEQAAQLSTTFQAQNRSLL
SELQHAQRAVNNDDPCVLL

**Important features of the protein:**

**Transmembrane domains:**

amino acids 31-49, 114-131

**N-glycosylation sites.**

amino acids 90-94, 144-148, 287-291, 563-567

**N-myristoylation sites.**

amino acids 45-51, 283-289

**Prenyl group binding site.**

amino acids 583-588

**ATP/GTP-binding site motif A (P-loop).**

amino acids 45-53

# FIGURE 47

CACTCATTCATTCCAAAGGGTCTCTCAAGGCAATGGTAATGTGCAAGGAGGTGATACCTAAAT
GAATGACCAAAAGAACATGCTTCTGCTTTTGTGTGTCTCCTACATTTTAGACATTTGTTTGTT
TCTCTTGGTAGCCTTTAAATTCCTTGAAGCCCAGGACCATGTCTCACTTACCTTTGTGTTTCC
ACTAACTAGTCTACCTCCTGGAATTGGCAGATACTCAGTGAAAGCCTGTGAAATAAGTGATGT
CTATTTCTAGCATATTATTCTGAGATTTAATGATAGATTTAGTGATTGAATGAGATTTCCATT
TTCAAATACAGCAAAAGCATAACTATTTTCATTCATTCATATTCATTCAACTTCATTCTCAAA
ATTAGGTCCTGAGTTAACTAATAATTACCTTTGAAATGTGTGGGTTATTTGAGGCAATCAGGT
GGTGACATTGAGCTCTCAGCCAGAGTTTGTTTCTGGAATTGATTCAGTTCCATTGCATTGATT
TTTGTTCTCAGAAGCCAAGGTTTCCCATGAAAAATCATTCCCACTTGAATTGGGCTGTGATTC
TTGCTGCGTTTAAGTAAAGGAAGCCTCTTGGTTCTAGTTCTGCAAACTTACACACTGAACTGG
GACAAGTTTTTGTTTAGAGTAATGGCTGGGAAAAGAGGAACCTTTCATTTTATTCAGAAGTCA
AAAACAAAGGCCTCCCAGCCACCTGGAGATGTTTTGTTGCAGACACCAGCCTGGCTCTGTCTT
TATGCCTAACAATTGAGCATCCAGTCTTCTTTGTGCTGGGACCATTGCTCAGCTCTGCAAGGG
GAAAAGAGGGAGAAAGCCAGAGCTGCCAGGCTTCTTGCACTGGGGCCGGGGGAGGGTTCCTGG
GAAGCAGGTGCTCTCTGGCTTCTTGGTACGTGAGGCTCTCGGAGCTGCCTCTCCTCTGACCCT
CAGGTCCTCACCGAGTTTGCTCCAGGAGTATATTGAAAACATACCCAGTGCTCTCTCAAGCAC
CCACTGCTTAGAGGGCCCAGATTTCTTTTCCTTCTTTCCCTTGCAGAGCTGGAGACTGCATCG
GGCATCTGGTGTTTAAACTAAACAGGAAAACTGACTAAAGGTCCACAGTGCTCATTGTGTAGA
CTAGCTGCCCTCCG**ATG**GGTGCTCTGATTATCAGTGGTTCCAGTGCAGGGCCTGTCACTAAAC
AGGCCTCACTTCCTCCTTGGGGGCTTTCCCATGGGAGGTGTGGCTTTTTACTCTACATGGAAA
TGACTCTCTGCAGCCACAGAACACAGTCATTTTCTGAATTATCCCAGTCTCTCATGCGCCCTG
GATTCCTCCAGATGCCTTATATCTCTTGTGCAAAGTTGTCTAAAATTTGGTTCCCAGCTTCCA
AGCCTTGCCTTTTGGCCTTCCTGGAAGTATTTTTGTTGATGAGTCGTCTGTCATTATTCTCTA
AAATGATTTGCTTTTTGTTTCTTTCATTCCTATTTCCACCCCACATATACACACATGCTTCT**T**
**AA**CTTAGGGGATTACATGCCAATAAATCTATTGTTGAAAATGCACTAATACTATCGCAAAGAC
GAAAATTCACAGGCTGAACCGTTGTAAGTCCATATGCTCCTCAACTTACATGTGTGATGGAGT
TATGCCCAAATAAGTCCATCGTCAAGTTGAAAAATCAAAATCAAGCCATCTTAGGTTGAGGAC
CATTTGTTTGTACCTCCAAAGATGTCATATCTTTAAACATACTCCCTAGCTTTTCTTTTTACT
TTTTATTTTGAAGTAATTATAGAATCACAGAAAGTTGCAAAAAA

# FIGURE 48

MGALIISGSSAGPVTKQASLPPWGLSHGRCGFLLYMEMTLCSHRTQSFSELSQSLMRPGFLQM
PYISCAKLSKIWFPASKPCLLAFLEVFLLMSRLSLFSKMICFLFLSFLFPPHIYTHAS

**Important features of the protein:**

**Signal peptide:**

amino acids 1-41

**Transmembrane domain:**

amino acids 88-107

**Casein kinase II phosphorylation site.**

amino acids 47-50

**N-myristoylation site.**

amino acids 24-29

# FIGURE 49

GGCTTCTACAGTCCACAACACCCACCAGCCCCAGGCCCAGCAGAATGAGCCCAGTGAGTGCCGGGGCTCCCAGTT
TGGCTGTTGCTATGACAACGTGGCCACTGCAGCCGGTCCTCTTGGGGAAGGCTGTGTGGGCCAGCCCAGCCATGC
CTACCCCGTGCGGTGCCTGCTGCCCAGTGCCCATGGCTCTTGTGCAGACTGGGCTGCCCGCTGGTACTTCGTTGC
CTCTGTGGGCCAATGTAACCGCTTCTGGTATGGCGGCTGCCATGGCAATGCCAATAACTTTGCCTCGGAGCAAGA
GTGCATGAGCAGCTGCCAGGGATCTCTCC**ATG**GGCCCCGTCGTCCCCAGCCTGGGGCTTCTGGAAGGAGCACCCA
CACGGATGGTGGCGGCAGCAGTCCTGCAGGCGAGCAGGAACCCAGCCAGCACAGGACAGGGGCCGCGGTGCAGAG
AAAGCCCTGGCCTTCTGGTGGTCTCTGGCGGCAAGACCAACAGCCTGGGCCAGGGGAGGCCCCCCACACCCAGGC
CTTTGGAGAATGGCCATGGGGGCAGGAGCTTGGGTCCAGGGCCCCTGGACTGGGTGGAGATGCCGGATCACCAGC
GCCACCCTTCCACAGCTCCTCCTACAGATCTCACTTCCCACCTCTCCAGGATTAGCTTGGCAGGTGTGGAGCCCT
CGTTGGTGCAGGCAGCCCTGGGGCAGTTGGTGCGGCTCTCCTGCTCAGACGACACTGCCCCGGAATCCCAGGCTG
CCTGGCAGAAAGATGGCCAGCCCATCTCCTCTGACAGGCACAGGCTGCAGTTCGACGGATCCCTGATCATCCACC
CCCTGCAGGCAGAGGACGCGGGCACCTACAGCTGTGGCAGCACCCGGCCAGGCCGCGACTCCCAGAAGATCCAAC
TCCGCATTATAGGGGGTGACATGGCCGTGCTGTCTGAGGCTGAGCTGAGCCGCTTCCCTCAGCCCAGGGACCCAG
CTCAGGACTTTGGCCAAGCGGGGGCTGCTGGGCCCCTGGGGGCCATCCCCTCTTCACACCCACAGCCTGCAAACA
GGCTGCGTTTGGACCAGAACCAGCCCCGGGTGGTGGATGCCAGTCCAGGCCAGCGGATCCGGATGACCTGCCGTG
CCGAAGGCTTCCCGCCCCCAGCCATCGAGTGGCAGAGAGATGGGCAGCCTGTCTCTTCTCCCAGACACCAGCTGC
AGCCTGATGGCTCCCTGGTCATTAGCCGAGTGGCTGTAGAAGATGGCGGCTTCTACACCTGTGTCGCTTTCAATG
GGCAGGACCGAGACCAGCGATGGGTCCAGCTCAGAGTTCTGGGGGAGCTGACAATCTCAGGACTGCCCCCTACTG
TGACAGTGCCAGAGGGTGATACGGCCAGGCTATTGTGTGTGGTAGCAGGAGAAAGTGTGAACATCAGGTGGTCCA
GGAACGGGCTACCTGTGCAGGCTGATGGCCACCGTGTCCACCAGTCCCCAGATGGCACGCTGCTCATTTACAACT
TGCGGGCCAGGGATGAGGGCTCCTACATGTGCAGTGCCTACCAGGGGAGCCAGGCAGTCAGCCGCAGCACCGAGG
TGAAGGTGGTCTCACCAGCACCCACCGCCCAGCCCAGGGACCCTGGCAGGGACTGCGTCGACCAGCCAGAGCTGG
CCAACTGTGATTTGATCCTGCAGGCCCAGCTTTGTGGCAATGAGTATTACTCCAGCTTCTGCTGTGCCAGCTGTT
CACGTTTCCAGCCTCACGCTCAGCCCATCTGGCAG**TAG**GGATGAAGGCTAGTTCCAGCCCCAGTCCAAAATAGTT
CATAGGGCTAGGGAGAAAGGAAGATGGACTCTTGGCTTCCTCTCTCTGGCTGGCAAAGGGAGTTATCTTCTGGAA
TACATTAGCTCTTTCAAAAACCCACCCAGTGTTTAGCCTCAACGGCAGCCAGTTACCAGCTTCTCTCTGTAGCCT
TCAGCAGTGTTTGCATCTCTGACATAACCACAGGCTGCTGTTTTCAAGAAGAGCAATCTGTTTGGATAAGAAAAA
CCTTTACTTTACAGCTTCCCTTTATAATTTGTTACACAGGAATAGTTAAATGCATTTGTTTGTTTGTTTTTTGAG
ACGGAGTTTCACTCTTGTTGCCCAGGCTGGAGGGCAATGGCGCGATCTCAGCTCACTGCAACCTCCGTCTCCTGG
GTTCTTGATTCTCCTGTGTCAGCCTTCTGAGTAGCTGGGATTACAGATGCCTATCACCATGCCTGGGTAATTTTT
GTATTTTTAGTTGAGATGGGGTTTCGCCATGTTGGCCAGGCTGGTCTCGAACTTCTGACCTCAGATGATCTGCCC
GCCTCAGCCTCCCAAAGTGCTGGGATTACAGGCATGAGCCACCACGCCCAGCCATCAATGCATTTTTTTTATTTT
TTTTTTGAGACAGAGTTTCGCACTTCTTGCCCAGGCTGGAGTACAATGGTGCGATCTTGGCTCACTGCAACCTCC
ACCTCCTGGGTTCAAGCGCTTCTCCAGCCTCAGCCTCCTGAGTAGCTGGGATTACAGGTATGTGCCACCATGCCT
GGCTAATTTTGTATTTTTGGTGGAGACGGGGTTTCTCCATGTTGGTCAGACTGGTCTTGAACTCCCGACCTCAGG
TAATCCGCCCGCCTCCGCCTCCCAAAATGCTGGGATTAGAGGTGTGAGCCACTGTGCCCAGCCCATCAATGTGTT
TTAAAGCTAGCTGTCAGGGTTCCACTTAATTTAAAGCTGGGCAGGGAGATGTGTAATGATTTCAAAGTTAACACC
TGTTTGTTTTCTAAAGGGCATGCCAAGTCCTGCTGTATCAGGGAAGTATTCTGTGCTAAAATCAGCGATGGTTCA
TTGCTCTAGTCTCTCTCACCCTTCTAGGCAGTGCATCAGTCAGCTCTAAATCTGGTGCAGAGGGTTAACAGCATA
ACCCTTGTTGGCAAAATGGAATAGATGTTAAGACCTCAAATAGGGATTTGGGATGAAACAGCTGCAGTTAGCACT
GTTATCTGAGCATGAAAGAACTGGAAACGCTCCTTACGTCGAGATGTTGGACCTTGAAGCCCTCCTGAGGCCAAC
ATGCAAATCTGGCTGTGACGGTTCATCTGACACCTGTGTAAAGCTGACCAGCCTGCTCTGTACAGTGACAATGAG
GAGCCCCTCTCTTCCTTAAGTAGGAATCTGTGAAGCAAAATGTTTGCTGCCAAAGACAAATCAGACTGTCAGTCA
TTAAAAACAGCATTAGCAGGATGAGGATAGCAATGGGGAAGGGTTGTGGGCAATGCAGTAACAGGGAAATGGCTT
CAGAAATGGTTTGAGTTGGAAGACAACATTCTTCATCTCTCAGGACTTCTAATTCCTTGATGCTAAAAGAAGAGG
CATGGATTCTATGAGCTTCCAAGTCCCTTTCCACTTTAACCTTCTACAAATCTTTCAGAGGACTGCCTAGTAGCA
AAGGTTATTCCTGGACACAGGAAAGACGGGCATTACAGGGACCAAAGCTCTGAAAGGTGACTTTTATTACCAACA
CACTGGCTGGAAAAGGGACAAACCACATCACGGGTGAGTGATACTTCTCAGTCTTCTCTACTCATTCAACAAAGG
AAATGTGGGCTGGGGCAGAGGTCTTTTTCATTTAATACTGGAAAAATATTGAAGAGCATCCATGTTCACTTATG
GCTGGTTTTGCTATAGAAATTGGAAAATAAAGGCCACTTTTTTG

# FIGURE 50

MGPVVPSLGLLEGAPTRMVAAAVLQASRNPASTGQGPRCRESPGLLVVSGGKTNSLGQGRPPT
PRPLENGHGGRSLGPGPLDWVEMPDHQRHPSTAPPTDLTSHLSRISLAGVEPSLVQAALGQLV
RLSCSDDTAPESQAAWQKDGQPISSDRHRLQFDGSLIIHPLQAEDAGTYSCGSTRPGRDSQKI
QLRIIGGDMAVLSEAELSRFPQPRDPAQDFGQAGAAGPLGAIPSSHPQPANRLRLDQNQPRVV
DASPGQRIRMTCRAEGFPPPAIEWQRDGQPVSSPRHQLQPDGSLVISRVAVEDGGFYTCVAFN
GQDRDQRWVQLRVLGELTISGLPPTVTVPEGDTARLLCVVAGESVNIRWSRNGLPVQADGHRV
HQSPDGTLLIYNLRARDEGSYMCSAYQGSQAVSRSTEVKVVSPAPTAQPRDPGRDCVDQPELA
NCDLILQAQLCGNEYYSSFCCASCSRFQPHAQPIWQ

**Important features of the protein:**

**Signal peptide:**

amino acids 1-16

**Tyrosine kinase phosphorylation site.**

amino acids 392-400

**N-myristoylation sites.**

amino acids 9-15, 50-56, 112-118, 146-152, 173-179, 195-201, 220-226, 229-235, 280-286, 306-312, 336-342, 397-403

**Myelin P0 protein.**

amino acids 153-182

# FIGURE 51

CAGGCAGAAGCGAACAAAGACCCAGCAAGAGAAGGCAGAGGCTAAGACCCATCCCGTATCTGC
TCTCCTGAAATAATTCTGGAGTC**ATG**CCTGAAATGCCAGAGGACATGGAGCAGGAGGAAGTTA
ACATCCCTAATAGGAGGGTTCTGGTTACTGGTGCCACTGGGCTTCTTGGCAGAGCTGTACACA
AAGAATTTCAGCAGAATAATTGGCATGCAGTTGGCTGTGGTTTCAGAAGAGCAAGACCAAAAT
TTGAACAGGTTAATCTGTTGGATTCTAATGCAGTTCATCACATCATTCATGATTTTCAGCCCC
ATGTTATAGTACATTGTGCAGCAGAGAGAAGACCAGATGTTGTAGAAAATCAGCCAGATGCTG
CCTCTCAACTTAATGTGGATGCTTCTGGGAATTTAGCAAAGGAAGCAGCTGCTGTTGGAGCAT
TTCTCATCTACATTAGCTCAGATTATGTATTTGATGGAACAAATCCACCTTACAGAGAGGAAG
ACATACCAGCTCCCCTAAATTTGTATGGCAAAACAAAATTAGATGGAGAAAAGGCTGTCCTGG
AGAACAATCTAGGAGCTGCTGTTTTGAGGATTCCTATTCTGTATGGGGAAGTTGAAAAGCTCG
AAGAAAGTGCTGTGACTGTTATGTTTGATAAAGTGCAGTTCAGCAACAAGTCAGCAAACATGG
ATCACTGGCAGCAGAGGTTCCCCACACATGTCAAAGATGTGGCCACTGTGTGCCGGCAGCTAG
CAGAGAAGAGAATGCTGGATCCATCAATTAAGGGAACCTTTCACTGGTCTGGCAATGAACAGA
TGACTAAGTATGAAATGGCATGTGCAATTGCAGATGCCTTCAACCTCCCCAGCAGTCACTTAA
GACCTATTACTGACAGCCCTGTCCTAGGAGCACAACGTCCGAGAAATGCTCAGCTTGACTGCT
CCAAATTGGAGACCTTGGGCATTGGCCAACGAACACCATTTCGAATTGGAATCAAAGAATCAC
TTTGGCCTTTCCTCATTGACAAGAGATGGAGACAAACGGTCTTTCAT**TAG**TTTATTTGTGTTG
GGTTCTTTTTTTTTTTAAATGAAAAGTATAGTATGTGGCACTTTTTAAAGAACAAAGGAAATA
GTTTTGTATGAGTACTTTAATTGTGACTCTTAGGATCTTTCAGGTAAATGATGCTCTTGCACT
AGTGAAATTGTCTAAAGAAACTAAAGGGCAGTCATGCCCTGTTTGCAGTAATTTTTCTTTTTA
TCATTTTGTTTGTCCTGGCTAAACTTGGAGTTTGAGTATAGTAAATTATGATCCTTAAATATT
TGAGAGTCAGGATGAAGCAGATCTGCTGTAGACTTTTCAGATGAAATTGTTCATTCTCGTAAC
CTCCATATTTTCAGGATTTTTGAAGCTGTTGACCTTTTCATGTTGATTATTTTAAATTGTGTG
AAATAGTATAAAAATCATTGGTGTTCATTATTTGCTTTGCCTGAGCTCAGATCAAAATGTTTG
AAGAAAGGAACTTTATTTTTGCAAGTTACGTACAGTTTTTATGCTTGAGATATTTCAACATGT
TATGTATATTGGAACTTCTACAGCTTGATGCCTCCTGCTTTTATAGCAGTTTATGGGGAGCAC
TTGAAAGAGCGTGTGTACATGTATTTTTTTTCTAGGCAAACATTGAATGCAAACGTGTATTTT
TTTAATATAAATATATAACTGTCCTTTTCATCCCATGTTGCCGCTAAGTGATATTTCATATGT
GTGGTTATACTCATAATAATGGGCCTTGTAAGTCTTTTCACCATTCATGAATAATAATAAATA
TGTACTGCTGGCATGTAATGCTTAGTTTCTTGTATTTACTTCTTTTTTTAAATGTAAGGACC
AAACTTCTAAACTAATTGTTCTTTTGTTGCTTTAATTTTTAAAAATTACATTCTTCTGATGTA
ACATGTGATACATACAAAAGAATATAGTTTAATATGTATTGAAATAAAACACAATAAAATT

# FIGURE 52

MPEMPEDMEQEEVNIPNRRVLVTGATGLLGRAVHKEFQQNNWHAVGCGFRRARPKFEQVNLLD
SNAVHHIIHDFQPHVIVHCAAERRPDVVENQPDAASQLNVDASGNLAKEAAAVGAFLIYISSD
YVFDGTNPPYREEDIPAPLNLYGKTKLDGEKAVLENNLGAAVLRIPILYGEVEKLEESAVTVM
FDKVQFSNKSANMDHWQQRFPTHVKDVATVCRQLAEKRMLDPSIKGTFHWSGNEQMTKYEMAC
AIADAFNLPSSHLRPITDSPVLGAQRPRNAQLDCSKLETLGIGQRTPFRIGIKESLWPFLIDK
RWRQTVFH

**Signal peptide:**
amino acids 1-30

**Transmembrane domain:**
amino acids 105-127

**N-glycosylation site.**
amino acids 197-201

**N-myristoylation site.**
amino acids 303-309

**Short-chain dehydrogenases/reductases family proteins.**
amino acids 18-30

# FIGURE 53

TGGGCTCCCTCCAGCACTGCTGTTGCCTGCTGCCTAAGATGGGTGACACTTGGGCCCAGCTTCCCTGGCCTGGGC
CACCCCACCCAGCA**ATG**CTGCTGATCTCCCTCCTCTTGGCAGCCGGGTTGATGCACTCGGATGCCGGCACCAGCT
GCCCCGTCCTTTGCACATGCCGTAACCAGGTGGTGGATTGTAGCAGCCAGCGGCTATTCTCCGTGCCCCCAGACC
TGCCAATGGACACCCGAAACCTCAGCCTGGCCCACAACCGCATCACAGCAGTGCCGCCTGGCTACCTCACATGCT
ACATGGAGCTCCAGGTGCTGGATTTGCACAACAACTCCTTAATGGAGCTGCCCCGGGGCCTCTTCCTCCATGCCA
AGCGCTTGGCACACTTGGACCTGAGCTACAACAATTTCAGCCATGTGCCAGCCGACATGTTCCAGGAGGCCCATG
GGCTAGTCCACATCGACCTGAGCCACAACCCCTGGCTGCGGAGGGTGCATCCCCAGGCCTTTCAGGGCCTCATGC
AGCTCCGAGACCTGGACCTCAGTTATGGGGGCCTGGCCTTCCTCAGCCTGGAGGCTCTTGAGGGCCTACCGGGGC
TGGTGACCCTGCAGATCGGTGGCAATCCCTGGGTGTGTGGCTGCACCATGGAACCCCTGCTGAAGTGGCTGCGAA
ACCGGATCCAGCGCTGTACAGCAGATTCTCAGCTGGCTGAGTGCCGGGGCCCTCCTGAAGTCGAGGGCGCCCCGC
TCTTCTCACTCACTGAGGAGAGCTTCAAGGCCTGCCACCTGACCCTGACCCTGGATGATTACCTATTCATTGCGT
TCGTGGGCTTCGTGGTCTCCATTGCTTCTGTGGCCACCAACTTCCTCCTGGGCATCACTGCCAACTGCTGCCACC
GCTGGAGCAAGGCCAGTGAAGAGGAAGAGATC**TGA**CATGCCTGCCTCTCATCCCTCCATGCTGCTGACCGCCACA
GCTGCTGGCCACCAGACGCCCTCCCTGATTGCTCACTCTGGTTCCATGGTGACCTGGCTGCCTCAGTCATGGTTC
AAGCAAGGTGGGGACACTCATTTTGTATGAGCATCTGCTTTGGGCCAGGCGGCACGCTAGGAATTGGGAACATCA
GATGAACTGACTCAGTCCCTGCCCTCAAGGCACTTCCCTCTGGTCAAGGAGAGAGATCCAAAAACTATTCCCTTT
AAGACTATATGTCAGGACTCTGAGCACGTCATTATGGAGGCCCAGAGGAGGAGCCATCATCTGTATCTAGCAATG
TCCATGAGAATTATAAGATTAGAGTGATTTGTGAACTGGGTCATCAGGAAATATCTACTTTGTCAGGTAGGCAAA
GAAGGGTGTCTGCACATGGCAGAGGCCAGAATATGCATAGTGTGCTGTGTTGAGAAGAGTGAACAGTTCCTGGTC
ACTTACTTGTATAGAGGGGGTGTGGCACAGAACTCAAACCTACCCCTCACCTCCTGACACCAAAACTGTCAGCTC
TCAGCAATGCCAGCCACTGCCTACAGGGAGTAAGAACACCTCTATGACAGCCCCTGGCCTCCTTCCACCAGCAGC
TACCAGGTGAGACCACCTCCCAGTGACTGCCCCCATATGACCAAATGTCACCAGTTGGTGAGGTCCCAGGCAGCA
GGCTGAGGATGGACACTTTCAATGCCCTTGCTCCTGCCTCTCACTCAAGTTTTGCTTCAGAAGAGAGAGGCAGGA
GGCCCAGCAACTGGGGCAGCAAGAGTCCTGGCACCTTGGGATCCTAATCATGTGACTGTTCTTGCCACAGTGCTC
ATGCCACAGGGTCTCACCAGGAAAGTGCACTGTGGCCACAGACCCACAGCCTGGCAGCACCCAGAGCTAAAAGG
GGACAAAGGCAGCACAGTTATGACCATATGAGGCTTTGCATTTTCTTCTAAGCAACTTACCCACGTTAAGCATGA
GGGTGAGAGAGCTATTAAATACTAAGCCCTTGCCAGTGTCAGGTACTTTGAAAAGCTCTCTGCACAAACCATTCC
CTTTGACACACACACACACAAATCTTTTGAGGTGAACGCTGTTGTTCCCATTTTACGGATGAGGCAACTAAGGCT
CAGAGAGGTTAAAGTCACATGCCACTATGAGCAAGATAAAGTCTGTGCTCTTTCTACTGCCCCATCCAAGTTGGG
GAACATCACCATTCCCTCTAGAGTTATATAAATTCAAATTCAACTAGAGCTGACAAAGTTCCTCATAAGGTCCAG
GCACTCCTCTGGGCACTTTTATATCTATTGACTCACTTCTTTCAATTCTCACAGCAACACTGCCTGGTGGTTTTT
ATTATCCCCATTTGACAGATGAATTAATCGTAGAGAGTTGAGTGACTTACCCAAGGTTGTCTGGATAAGCCCTAG
AAGGAAGGCGGTAGGCAGCTCCATTCAGGGAAACTGCATCTAATCAGTCAGTCAAAAATCAAGTAACTTTACGAG
CAAAGCACAATTATCATCATCGTGGTCTTCTTCATCAGTTTCGTCAGCAGCATCATTATCTTCCCTCTATTTGTT
CAGCACCGGATAGTTCATGAGTATTTTTGCATCATTCTCCTTGACTTTTCACATCCCTGTGCAGGAGGTAAATCA
AACATCAGTAATCCTGTTTTACAGATGGGGAAAAAAGTCTCAAGGTTGGATATGACTTGCTATGTGGCAAGGTTG
GGGCTCAACCCTAACACAGTTCTCTTTCCAGTGCTTTCTCAAGTGCTTGGGGAAGAGAATGCCTCAGAAGGCTGG
GTAGTGGGGCCCTGGAATTCAGCATCCATGAATGTGCTAGTGGATAAGCTAAATAGAAGGCAGCCAAACCCATCT
GCTGTACAGATTGAACTATGCTCACGGTAGGGCAAATTGCAGGCTCTGAAACAGAGACTACACAGGTAACACCTG
AATAGGAGACTCCTGCTTTACAATGTGTAGATAAAACATCAGCAATGGTGGCCATGGTGGCAGTCATGTGAAAAG
TAAGATCTTTGGGAATCAAGAAAGGAAGCTGTGTTAACCACTCCTGCTCAAGCCCTGCTGCGTGTGTTGCAAGAG
ATACTAAGAGAGCAAGAAAGCTATAGGTGAGAACCTCTGCAGTTTAGGAGAAGAACATCAAGGCACAGTCCAACA
TGCTGATAAGTCTGGCCAGGAGGAGAATTAAAACAGGGGCTTTCCACACCTCCCTTGCCCCAAGCTCCAGCGGTA
TTCTATCAGCCCATCCTCCTGGAAAGCCTGAAAGGAATGAAGGAGGCTAATAAGTCATCTTCCAGGAAGGCATCC
CTCACTCGTGCTTCCCTGAGCTAGTCAACCAAAAGAGTCTTCAGAAACTTTGCTAGACCTGAAGTACTTGAACCT
GTGTCCCCTGAATCTTTCTTACAACATCTGGGACAAATCCCTGGTCCTGTGACATCCGAAGCAGAACTGTGCCCT
GCTCTCTCCTTCTGTGATGACCAAGGATGGTGAACTCAAGTTGTTCTCTACAAGCCAGGCCAGCAACCTAAATAC
TTGGAGAGGAACTTTTAGAAACTATAATCCTGACAAAATAGAAAAGTTTCCCATAGGGGCATACCATAATACTAT
AATAACCTCCCAGGAACTATTGTTTGCCAAAATGTAGTTAATATATTTTAAGATATATGCTTTTTTGCATAGGAC
TAGAACCAGAAAAGACACCAAATGCCCCCTTGACATCAATGTCCTTTCTAGTGGGACAATTTGGTCTCCATTAAT
GCCAAACCTTTCTGAACAGGATACATGGCTTTTAAAGGACAGATGTTTCTCCTGCTGCTAGAAGTTCCTCAGTTT
ACTAGAGCACAATGAGGAAAGTATTCAACCTCCCTACTGCCAAGGAATTCCCTGCTTCTCCCCCACCGCCATCAT
CTTGTCCAAGCTATCAGAAGCAACCTTCTAGAGATAATCTAACAATCCTGATTAGAATTGCTCCCATATCCCTGG
TGACCACAGGCTTCATTCAAATTGTCCAAACTGGTTAACATGTATGTGATGGGGTATCTCTGCATCTGTATGTCT
GTCTGCGAGGTTCCTTGTATATTGGCTGTCCGCTGACTTGGGACAGATCTCTCTAGAACTTGGGTTCAGTTCTCT
GACATAGTCCACTCAGCCATAGGCTGAGTGGCTAAATATGCATAAATAAGCATGCCTAAATAGGCATATATAGGT
TGGTGCAAAAGTAATTGCGGTTTTTGCCATTAAAAATGATGGCAAAAATCCCAATTACTTTTGCGTCAATCTAAT
ATTACATTGCTTGATAGATTAAGATGGAATCCCACCAGGTTTAGGGTAGGACTGGATGCTCAAAAAAAAAAAAA
AAAAAAAAAAAAAAAAAAAAA

# FIGURE 54

MLLISLLLAAGLMHSDAGTSCPVLCTCRNQVVDCSSQRLFSVPPDLPMDTRNLSLAHNRITAV
PPGYLTCYMELQVLDLHNNSLMELPRGLFLHAKRLAHLDLSYNNFSHVPADMFQEAHGLVHID
LSHNPWLRRVHPQAFQGLMQLRDLDLSYGGLAFLSLEALEGLPGLVTLQIGGNPWVCGCTMEP
LLKWLRNRIQRCTADSQLAECRGPPEVEGAPLFSLTEESFKACHLTLTLDDYLFIAFVGFVVS
IASVATNFLLGITANCCHRWSKASEEEEI

**Important features of the protein:**

**Signal peptide:**

amino acids 1-17

**Transmembrane domain:**

amino acids 241-260

**N-glycosylation sites.**

amino acids 52-55, 81-84, 107-110

**Tyrosine kinase phosphorylation site.**

amino acids 148-154

**N-myristoylation sites.**

amino acids 11-15, 263-268

**Prokaryotic membrane lipoprotein lipid attachment site.**

amino acids 175-185

**Leucine zipper pattern.**

amino acids 77-98

# FIGURE 55

GGCTGCGCCCAGGCCGGCGGGCCCAGCAGCTGCGAACCGCCGGCGCACCACCTGTTTCCGCGC
CCGGGGACTTCCCCGGCGGGGCTCAGAAGTGTGGGGTCGGTCGCTTGGCTTCCCCTGGCGTCA
GCGACCCAGGGTAACCTCCTCCACTGCTGCGTGCCGTGCAGGCCTGCCTGTGTGAGAGCCACG
TGTGCCGCGCTCTGGGCACAGCCTTGGAAAGTCAGGACCGCGACGGCAGCAGAGCAGAAACCT
TACAGAAACATGAAGCCCTCAACCATCTGCTACTCAGTTATTCGGGGCTGACGGCGGCTTCTA
GAACATCCAGGTGTTCTGCAGATGCGAGAACTCATCCTGTAGTCACCAGATGGAGTCCCAAAC
AGCCAAGCAGATGTAAGGCCTGTGCTGTGGCTCTGAGGCCCTGAATACAGAAGGGTCACTTTC
TTAGTGGCCAAAGAGCAGTTGTTGACATTGATGTCTAATTATTGAACACGACCAGTCATTTTA
CTGAGCTGCAGTGAGGAAACACTGACCATAGAAGATCAAGCCAAATGAGGGATTGCAAATTTC
CTGATTCTTTTGAATTAGGATTCCAGATGGGGGCCTCATTTCTACAGCCCCCAACATTCCTAT
AGCCGTTATCACTGCCATCACCACTGCCACCAGCATCTTCTTGCAGATTCCACCCCTGCTCCC
CAGAGACTTCCTGCTTTGAAAGTGAGCAGAAAGGAAGCTCTCAGAAAAATCTCTAGTGGTGGC
TGCCGTCGCTCCAGACAATCGGAATCCTGCCTTCACCACC**ATG**GGCTGGCTTTTTCTAAAGGT
TTTGTTGGCGGGAGTGAGTTTCTCAGGATTTCTTTATCCTCTTGTGGATTTTTGCATCAGTGG
GAAAACAAGAGGACAGAAGCCAAACTTTGTGATTATTTTGGCCGATGACATGGGGTGGGGTGA
CCTGGGAGCAAACTGGGCAGAAACAAAGGACACTGCCAACCTTGATAAGATGGCTTCGGAGGG
AATGAGGTTTGTGGATTTCCATGCAGCTGCCTCCACCTGCTCACCCTCCCGGGCTTCCTTGCT
CACCGGCCGGCTTGGCCTTCGCAATGGAGTCACACGCAACTTTGCAGTCACTTCTGTGGGAGG
CCTTCCGCTCAACGAGACCACCTTGGCAGAGGTGCTGCAGCAGGCGGGTTACGTCACTGGGAT
AATAGGCAAATGGCATCTTGGACACCACGGCTCTTATCACCCCAACTTCCGTGGTTTTGATTA
CTACTTTGGAATCCCATATAGCCATGATATGGGCTGTACTGATACTCCAGGCTACAACCACCC
TCCTTGTCCAGCGTGTCCACAGGGTGATGGACCATCAAGGAACCTTCAAAGAGACTGTTACAC
TGACGTGGCCCTCCCTCTTTATGAAAACCTCAACATTGTGGAGCAGCCGGTGAACTTGAGCAG
CCTTGCCCAGAAGTATGCTGAGAAAGCAACCCAGTTCATCCAGCGTGCAAGCACCAGCGGGAG
GCCCTTCCTGCTCTATGTGGCTCTGGCCCACATGCACGTGCCCTTACCTGTGACTCAGCTACC
AGCAGCGCCACGGGGCAGAAGCCTGTATGGTGCAGGGCTCTGGGAGATGGACAGTCTGGTGGG
CCAGATCAAGGACAAAGTTGACCACACAGTGAAGGAAAACACATTCCTCTGGTTTACAGGAGA
CAATGGCCCGTGGGCTCAGAAGTGTGAGCTAGCGGGCAGTGTGGGTCCCTTCACTGGATTTTG
GCAAACTCGTCAAGGGGGAAGTCCAGCCAAGCAGACGACCTGGGAAGGAGGGCACCGGGTCCC
AGCACTGGCTTACTGGCCTGGCAGAGTTCCAGTTAATGTCACCAGCACTGCCTTGTTAAGCGT
GCTGGACATTTTTCCAACTGTGGTAGCCCTGGCCCAGGCCAGCTTACCTCAAGGACGGCGCTT
TGATGGTGTGGACGTCTCCGAGGTGCTCTTTGGCCGGTCACAGCCTGGGCACAGGGTGCTGTT
CCACCCCAACAGCGGGGCAGCTGGAGAGTTTGGAGCCCTGCAGACTGTCCGCCTGGAGCGTTA
CAAGGCCTTCTACATTACCGGTGGAGCCAGGGCGTGTGATGGGAGCATGGTGCCTGAGCTGCA
GCATAAGTTTCCTCTGATTTTCAACCTGGAAGACGATACCGCAGAAGCTGTGCCCCTAGAAAG
AGGTGGTGCGGAGTACCAGGCTGTGCTGCCCGAGGTCAGAAAGGTTCTTGCAGACGTCCTCCA
AGACATTGCCAACGACAACATCTCCAGCGCAGATTACACTCAGGACCCTTCAGTAACTCCCTG
CTGTAATCCCTACCAAATTGCCTGCCGCTGTCAAGCCGCA**TAA**CAGACCAATTTTTATTCCAC
GAGGAGGAGTACCTGGAAATTAGGCAAGTTTGCTTCCAAATTTCATTTTTACCCTCTTTACAA
ACACACGCTTTAGTTTAGTCTTGGAGTTTAGTTTTGGAGTTAGCCTTGCATATCCCTTCTGTA
TCCTGTCCCCCCTCCACGCCGACCCGAGAGCAGCTGAGCTGCGCTGGCTCTGGGCAGGGAGTG
TGCCTTAATGGGAAGCACACGGGCTTTGGAGTCAGGCACAGGTGCCAGCTCCAGCTTTTGAAC
TTGGGCAATTGTTTAACCTAACCTGCAAGTTGATTTTGAGGGTTAAATAAAGGCATACATGAA
AATGCCTGGCAACTTTAAAAAAAAAAAA

# FIGURE 56

MGWLFLKVLLAGVSFSGFLYPLVDFCISGKTRGQKPNFVIILADDMGWGDLGANWAETKDTAN
LDKMASEGMRFVDFHAAASTCSPSRASLLTGRLGLRNGVTRNFAVTSVGGLPLNETTLAEVLQ
QAGYVTGIIGKWHLGHHGSYHPNFRGFDYYFGIPYSHDMGCTDTPGYNHPPCPACPQGDGPSR
NLQRDCYTDVALPLYENLNIVEQPVNLSSLAQKYAEKATQFIQRASTSGRPFLLYVALAHMHV
PLPVTQLPAAPRGRSLYGAGLWEMDSLVGQIKDKVDHTVKENTFLWFTGDNGPWAQKCELAGS
VGPFTGFWQTRQGGSPAKQTTWEGGHRVPALAYWPGRVPVNVTSTALLSVLDIFPTVVALAQA
SLPQGRRFDGVDVSEVLFGRSQPGHRVLFHPNSGAAGEFGALQTVRLERYKAFYITGGARACD
GSMVPELQHKFPLIFNLEDDTAEAVPLERGGAEYQAVLPEVRKVLADVLQDIANDNISSADYT
QDPSVTPCCNPYQIACRCQAA

**Important features of the protein:**

**Signal peptide:**

amino acids 1-16

**Transmembrane domain:**

amino acids 353-373

**N-glycosylation sites.**

amino acids 117-120, 215-218, 356-359, 397-500

**N-myristoylation sites.**

amino acids 12-17, 33-38, 52-57, 97-102, 101-106, 113-118, 158-163, 328-333, 388-393, 418-423, 435-440, 436-441

**Amidation site.**

amino acids 382-385

**Sulfatases signature 2.**

amino acids 129-138

# FIGURE 57

TGGACAAGACACCTCCAGGAGCCCAGCTCACAGCCACCGGTACCTTCTTCCAGGACAAGCTGG
GGGCCTCCATGGGCGCCTGAGGGCCAGGCGCCAGGGCCGTGGGCACGAGT**ATG**GTGAGACACC
AGCCCCTGCAGTACTACGAGCCACAGCTGTGCCTCTCCTGCCTCACGGGCATCTACGGCTGCC
GTTGGAAGCGCTACCAGCGCTCCCATGATGATACCACACCGGGCACAGCGCCATTCCTGCATG
TGGGGGCTGTGGCAGCAGTCACCATGCTCTCCTGGATCGTGGCAGGACAGTTCGCCCGTGCAG
AGCGGACCTCCTCCCAGGTGACCATTCTCTGTACCTTCTTCACCGTGGTGTTTGCCCTCTACC
TGGCCCCTCTCACCATCTCCTCTCCCTGCATCATGGAGAAGAAAGACCTCGGCCCCAAGCCTG
CTCTCATTGGCCACCGCGGGGCCCCCATGCTGGCTCCAGAGCACACGCTCATGTCCTTCCGGA
AGGCCCTCGAGCAGAAGCTGTACGGGCTCCAGGCTGACATTACCATCAGCCTGGACGGCGTGC
CCTTCCTCATGCATGACACCACCCTGCGGCGCACCACCAACGTGGAGGAGGAGTTCCCGGAGC
TGGCCCGCAGGCCTGCCTCCATGCTTAACTGGACCACCCTGCAGAGACTCAACGCTGGCCAGT
GGTTCCTGAAGACTGACCCCTTCTGGACAGCCAGCTCCCTGTCACCCTCCGACCACAGAGAGG
CCCAGAACCAGTCCATCTGCAGCCTGGCAGAGCTCCTGGAGCTGGCCAAGGGCAATGCCACAC
TGCTGCTCAACCTGCGTGACCCGCCCCGGGAGCACCCCTACCGCAGCAGTTTTATCAACGTGA
CTCTGGAGGCCGTGCTGCACTCCGGCTTCCCCCAGCACCAGGTCATGTGGCTGCCTAGCAGGC
AGAGGCCCCTGGTGCGGAAGGTGGCTCCCGGCTTCCAACAGACATCAGGCTCCAAGGAGGCAG
TCGCCAGCCTGCGGAGAGGCCACATCCAGCGGCTGAACCTGCGCTACACTCAGGTGTCCCGCC
AGGAGCTCAGGGACTACGCGTCCTGGAACCTGAGTGTGAACCTCTACACAGTCAACGCACCGT
GGCTCTTCTCCCTGCTGTGGTGTGCGGGGGTCCCATCCGTCACCTCTGACAACTCCCACACCC
TGTCCCAGGTGCCTTCCCCCCTCTGGATCATGCCCCCGGACGAGTACTGTCTCATGTGGGTCA
CTGCCGACCTGGTCTCCTTCACCCTCATCGTGGGCATCTTCGTGCTCCAGAAGTGGCGCCTGG
GTGGCATACGGAGCTACAACCCTGAGCAGATCATGCTGAGTGCTGCGGTGCGCCGGACCAGCC
GGGACGTCAGCATCATGAAGGAGAAGCTTATTTTCTCAGAGATCAGCGATGGTGTAGAGGTCT
CCGATGTGCTCTCCGTATGTTCAGACAACAGTTATGACACATATGCCAACAGCACCGCCACCC
CTGTGGGCCCCCGAGGGGGTGGCAGCCACACCAAGACCCTCATAGAGCGGAGTGGGCGT**TAG**C
TGAAGACATGTCTGTCCCACCTGTACCTGACACAGAAGCTGGGGAGCCTAGGAGAGCTGGTGG
AAGTGTGTCTGAACTCGGAGTGCTCTGGGAGCGGGCTCCACAGCCTCCTTGTGGGCTCCAGCC
CCTTGTCAGCCGCAGCCTCTCTTGAGGGGGACTCCCTGTCTCCTGAGGCCCAGCTGGGCCAGG
ACTCCATCCTTTCAGATGCCCCTGCAGGCCTGGGGCTCCTTCTGGGAAGTATGGGGCCTAGGG
CTTGGTCCCCCTCTTCTGAGGCCCTCTCCTGTATCCCGACCTGGAAGCTTTGATGGGTCATGG
GCCATGCCATACCCCCTGTGGCAATGGAGTGTGTGGATGCTCACCTGTGCCATCTGTCCTCCT
GTCTGTGCCAGGAGGCACCTGAGTTCTCTGCTGTTATCCTGCCCCAAGGGCCTGGGCCGAGCC
TCTACCTGAAGCAACTCTGCTCTTCCTGTCAGTCTCAAAGCACAAGGAGGTTCAGCCCAGGAG
GAAGCCAGCTGCAATGTGGAGACACGTCCTCCTCCCCAACCCACCTCATGCCACCGCCAACCC
CCTGCCCCAGGAGCGGGCCTGAGCCACGTCCCCTAGGAGCAGCTGGAGATGGCCAAAAGAGTG
AGCTCAGGACTACTGGATCCCATGCCCAGGTGTCCAGCAGACCTCAAGGCAGAAGGGTCACCT
AACCCAGGAGTCCACAGACTGATGTGACCTCAGGTTCCCACATCAGTGGCCACAGGGCAGGGC
CCACCTGGTAGAAGTGTTCTGGATATGGCCAGGGTGGGTGTGTGGCTAAGTGGGCCTGAACAG
AGGGAACCTAGGGCCCTTGGCCAATGTGATTAAAGCTGCCATCTTGAAA

# FIGURE 58

MVRHQPLQYYEPQLCLSCLTGIYGCRWKRYQRSHDDTTPGTAPFLHVGAVAAVTMLSWIVAGQ
FARAERTSSQVTILCTFFTVVFALYLAPLTISSPCIMEKKDLGPKPALIGHRGAPMLAPEHTL
MSFRKALEQKLYGLQADITISLDGVPFLMHDTTLRRTTNVEEEFPELARRPASMLNWTTLQRL
NAGQWFLKTDPFWTASSLSPSDHREAQNQSICSLAELLELAKGNATLLLNLRDPPREHPYRSS
FINVTLEAVLHSGFPQHQVMWLPSRQRPLVRKVAPGFQQTSGSKEAVASLRRGHIQRLNLRYT
QVSRQELRDYASWNLSVNLYTVNAPWLFSLLWCAGVPSVTSDNSHTLSQVPSPLWIMPPDEYC
LMWVTADLVSFTLIVGIFVLQKWRLGGIRSYNPEQIMLSAAVRRTSRDVSIMKEKLIFSEISD
GVEVSDVLSVCSDNSYDTYANSTATPVGPRGGGSHTKTLIERSGR

**Important features of the protein:**

**Signal peptide:**

amino acids 1-24

**Transmembrane domains:**

amino acids 47-61, 77-93, 335-350, 380-399

**N-glycosylation sites.**

amino acids 182-186, 217-221, 233-237, 255-259, 329-333, 462-466

**Tyrosine kinase phosphorylation site.**

amino acids 130-139

**N-myristoylation sites.**

amino acids 21-27, 48-54, 294-300, 404-410, 442-448, 473-479

# FIGURE 59

CCTGAGCAAACACAGCAGCCCGAGTGTTCCCAAGGCCAAA**ATG**CTGAGAACGTCCACTCCTAA
TCTGTGTGGTGGTCTGCATTGCCGGGCCCCCTGGCTCTCTTCTGGCATTCTCTGCCTCTGCCT
CATATTCTTGTTAGGCCAGGTGGGCTTGCTGCAGGGACACCCCCAGTGCCTGGATTACGGGCC
CCCTTTCCAGCCCCCTCTGCACCTTGAGTTTGCTCTGACTATGAGTCCTTCGGCTGCTGTGA
TCAGCACAAGGACCGCCGCATCGCTGCCCGGTACTGGGACATCATGGAATATTTTGATCTGAA
GAGACATGAGCTGTGTGGAGATTACATTAAAGACATCCTTTGCCAGGAGTGCTCGCCCTACGC
AGCCCACCTCTACGACGCCGAAAACACCCAGACGCCTCTCCGGAATCTCCCGGGCCTCTGCTC
TGATTACTGCTCTGCCTTCCATTCTAACTGTCACTCAGCCATTTCCCTGCTGACCAATGACCG
CGGCCTCCAGGAGTCTCATGGAAGGGACGGTACCCGCTTCTGCCACCTCCTGGACCTTCCTGA
CAAGGACTATTGCTTCCCTAATGTCCTGAGGAACGACTATCTCAACCGCCACCTGGGCATGGT
GGCCCAAGATCCTCAGGGCTGCCTGCAGCTCTGCCTGAGCGAGGTGGCCAACGGGCTGAGGAA
CCCCGTCTCCATGGTCCATGCTGGGGACGGCACCCATCGCTTCTTTGTTGCCGAGCAGGTAGG
AGTGGTGTGGGTCTACCTCCCTGATGGGAGTCGCCTGGAGCAACCCTTCCTGGACCTCAAGAA
CATCGTGTTGACCACCCCATGGATCGGGGATGAGAGAGGCTTCTTGGGGTTGGCTTTTCACCC
CAAATTCCGCCACAATCGCAAGTTCTATATTTATTATTCGTGCCTGGACAAGAAGAAGGTAGA
AAAGATCCGAATTAGTGAGATGAAGGTTTCTCGGGCTGATCCTAACAAAGCTGACCTGAAATC
AGAGAGGGTCATCTTGGAGATTGAAGAACCAGCCTCAAACCATAATGGCGGACAACTTCTTTT
TGGCCTGGATGGCTATATGTACATATTCACTGGGGACGGGGGACAGGCTGGAGATCCCTTTGG
CCTGTTTGGAAATGCTCAGAACAAAAGTTCCCTGCTGGGAAAAGTTTTAAGGATCGATGTGAA
CAGGGCAGGCTCACATGGCAAGCGGTACCGAGTCCCCTCGGACAATCCATTTGTTTCTGAGCC
AGGGGCCCACCCCGCCATCTATGCCTATGGGATCAGGAACATGTGGCGTTGTGCTGTGGACCG
AGGGGACCCCATCACGCGCCAGGGCCGAGGCCGGATATTCTGTGGGGACGTGGGCCAGAACAG
GTTTGAAGAGGTTGACCTCATTTTGAAAGGTGGAAACTATGGCTGGAGAGCAAAGGAAGGGTT
TGCATGTTATGACAAAAAACTTTGTCACAATGCCTCTTTGGATGATGTTCTGCCAATCTATGC
TTATGGCCATGCAGTGGGGAAGTCAGTCACTGGAGGTTATGTCTATCGTGGTTGTGAATCCCC
AAATCTCAATGGCCTGTATATCTTTGGAGACTTCATGAGTGGTCGACTTATGGCTTTGCAGGA
AGATAGAAAAAACAAGAAATGGAAGAAGCAGGATCTTTGCCTGGGCAGCACCACGTCCTGTGC
CTTCCCAGGGCTGATCAGCACCCATAGCAAGTTCATCATCTCCTTTGCTGAAGATGAAGCAGG
GGAGCTGTATTTCCTGGCGACCTCTTACCCAAGTGCCTATGCACCACGTGGATCTATTTACAA
GTTTGTTGACCCCTCAAGGCGAGCACCCCCAGGCAAGTGCAAATACAAGCCAGTGCCCGTGAG
AACCAAGAGTAAGCGGATCCCGTTCAGACCACTCGCCAAGACAGTCTTGGACTTGCTAAAGGA
ACAATCAGAGAAAGCTGCTAGAAAATCTTCCAGTGCAACCTTAGCTTCTGGCCCAGCCCAGGG
TTTGTCTGAGAAAGGCTCCTCCAAGAAGCTGGCTTCTCCTACAAGCAGCAAGAATACATTGCG
AGGGCCTGGTACAAAGAAGAAAGCCAGAGTGGGGCCCCACGTCCGCCAGGGCAAGAGGAGGAA
GAGCCTGAAAAGCCACAGTGGCAGGATGAGGCCATCAGCAGAGCAGAAGCGAGCTGGCAGAAG
TCTCCCT**TGA**CCTATTGGTCAAGGTGGCCGACAGGGTGACGTGAGAGAGGAGAGCCACCTCAT
CAAATGAAAGTCACTGCTGAATAAAGACCTTAGAAGTCTGGGAAGCCAGGGTAGAGGTGGGGC
AGGGCGGTTTTCCTCTCCCTGGGAAATCTTGCTGTCTACTGAATAAATAAATGCACCTTCTCT
GTATGCAGTGCTTCTGTGGGAGACCATATCCCAGATTGCTGGTGCACCTGGGTTATGGTAAGC
ACTAGTCCATGAGCCTGCTTGGAATCACACTGGATGTCTCCGTTTTGTCTTGTAAATGCCTAC
AACCTGAGGTAATAAATCAACATTTGCTCA

# FIGURE 60

MLRTSTPNLCGGLHCRAPWLSSGILCLCLIFLLGQVGLLQGHPQCLDYGPPFQPPLHLEFCSD
YESFGCCDQHKDRRIAARYWDIMEYFDLKRHELCGDYIKDILCQECSPYAAHLYDAENTQTPL
RNLPGLCSDYCSAFHSNCHSAISLLTNDRGLQESHGRDGTRFCHLLDLPDKDYCFPNVLRNDY
LNRHLGMVAQDPQGCLQLCLSEVANGLRNPVSMVHAGDGTHRFFVAEQVGVVWVYLPDGSRLE
QPFLDLKNIVLTTPWIGDERGFLGLAFHPKFRHNRKFYIYYSCLDKKKVEKIRISEMKVSRAD
PNKADLKSERVILEIEEPASNHNGGQLLFGLDGYMYIFTGDGGQAGDPFGLFGNAQNKSSLLG
KVLRIDVNRAGSHGKRYRVPSDNPFVSEPGAHPAIYAYGIRNMWRCAVDRGDPITRQGRGRIF
CGDVGQNRFEEVDLILKGGNYGWRAKEGFACYDKKLCHNASLDDVLPIYAYGHAVGKSVTGGY
VYRGCESPNLNGLYIFGDFMSGRLMALQEDRKNKKWKKQDLCLGSTTSCAFPGLISTHSKFII
SFAEDEAGELYFLATSYPSAYAPRGSIYKFVDPSRRAPPGKCKYKPVPVRTKSKRIPFRPLAK
TVLDLLKEQSEKAARKSSSATLASGPAQGLSEKGSSKKLASPTSSKNTLRGPGTKKKARVGPH
VRQGKRRKSLKSHSGRMRPSAEQKRAGRSLP

**Important features of the protein:**

**Signal peptide:**

amino acids 1-41

**Transmembrane domain:**

amino acids 17-36

**N-glycosylation sites.**

amino acids 372-376, 480-484

**cAMP- and cGMP-dependent protein kinase phosphorylation site.**

amino acids 645-649, 699-703

**Tyrosine kinase phosphorylation site.**

amino acids 81-89

**N-myristoylation sites.**

amino acids 11-17, 37-43, 156-162, 165-171, 357-363, 365-371, 368-374, 408-414, 459-465, 548-554, 557-563

**Amidation sites.**

amino acids 391-395, 696-700

**Cell attachment sequence.**

amino acids 428-431

**Leucine zipper pattern.**

amino acids 25-47

# FIGURE 61

CTCCATTAAACCACCACCAGCTCCCCAAGCCACCCCTTCAGCC**ATG**AAGTTCCTGCTCCTGGT
CTTGGCAGCCCTCGGATTCCTGACCCAGGTGATCCCAGCCAGTGCAGGTGGGTCAAAATGTGT
GAGTAACACCCCAGGATACTGCAGGACATGTTGCCACTGGGGGGAGACAGCATTGTTCATGTG
CAACGCTTCCAGAAAATGCTGCATCAGCTACTCCTTCCTGCCGAAGCCTGACCTACCACAGCT
CATCGGTAACCACTGGCAATCAAGGAGAAGAAACACACAAAGGAAAGACAAGAAGCAACAAAC
GACCGTAACATCA**TAA**TAACCACTGCTATCGCCTCCACCAACTCAGAGAAATATCATTTCCAC
AGTTCCAATTCCTCCTACATTGCTGAGTACTAGCCAAGGCTCCTCTTTATGGGGCAGATATCT
ATAGCCAACCCCAAAACTTCTGTCTTCTATCATTCTGTCATTCATCTAGTAACTAATTTGGAG
TTTGTATCTATCTTACGAGAACAATCATCATGCAGATTCGTCCACAGGGGATCTGTCAGTTTG
GGTCCTCCAAATGAAAAATGTCAAGACAGAATTGGACATGCAAAAGATTGACTGGGAGAACAC
ACCTCTGATGGACAAAGGTGAGACAGAGCAGCCACAGGCAGGGAGAGCCTTCAGACTGCAACG
CTGGCCTGATACGTGTCAAAGGAGAGAGGGATAGAGGAGGATTGAATAGAAGGAGACTAAGAC
TGCAGCTCTAAGAAAGTCTCAGCCAAACAGATGGGGAGGCCCAAAGCAAGGCTTGCCCCTCAG
AGGAGCTCACGCAGGGCAGGAATAGCCAGGTTCTCATATCCCAGGGGTTCAGACTTGGCTGAG
AACAGCCCCTGGAGAACATGGGGTGACTGCTACCATAGGTCTGGAAGTATGAGGCTGTCCACC
AACTATCCCCTTGAAGCAAGTTCTCTTGAAAGGAAATCTAAACAGTGCACCCCCATGGCTGCC
ACGGAGTATAAGGAGGGAGAGAAAGGAGCTGAAAGTCTAGGTTTGGCCAGCTAGGTAGACTGA
CTTGTGAGGTATTTATTTATTCATTTGAGTAACAAAGCAGACAGAATACATAGCCACCATTGG
TAGTACACCCCAAAAGCAAGGATGGCATGATGCTGGTGACTCAAACGTGCCTACTCATGGTGT
CAAATTGGCATAATCCTCTTGGGAAGCTGTGTGGAAATAAGCACAGAGAAGCAGAACTCTAAT
TGCTTAATCCACTAAACATTACTTCTGGGAATTGGCTCATCATAAATTATCCAAGAGAAAGCA
CAAAGTTATGGGCACAAAGGTTTTCCATATAATATTATTTAAAATGCTGAGAAAATGAAAAAA
TCTAAATGGTGAAATATATACTAATGCCATCTATAAATACAAACAAATAGAATGTTTATAGAA
TAATGGAACATAATAACATTATTCAAAATTGCATTTATGCTATAGTTGTCAAAATTGTCTCCT
TATATGATACAAAACTCATGAAAATTATGACTTTTTTGTTTGGTTGGAAAGCAGAATTATGCA
TAAATTTCCTCTTACAGTTCGATGCCCATTAGTTTTATATAACATTTATTTGACACGTACTGA
CTTCTATCTGAGAAGAACAAACCAAAACACTCAGGCCTAAATAATTAAAAACGGTCCTAAAAA
CTAGCAAACCAGATAAGAAAAGATGTTAATGCCCATTCCCTAACTTATGTCTTAGACCAAAAT
TAATTCTAGATGGTTTTAAAATGACAGTGTAAAAGTAAAGTATTAAAAGATTGTGTGGTCAAA
TATTCAATTTAAGAGCAAGGAAATTCTTATAAATATAACAATAGAGGCAGAACTCATGTAAGA
ATAAATTGATTAGGTGGTATTAAATATTAAGTTCTTATGTATGTCAAAAGATATCATTTTGAA
ATTCATCCATCTTATTGGGTATTGCAGGAGTTCATTCCTTTTTGTTTATAAATACTCTTCCGT
CATATGAATAGTATTCATTTGTATACTGGTTTGTTGATGGACATTTGGGTTGTTCCCAGTTTA
TGGCTATTACAAATAAAGCTTCTATGAACATTTATGTACA

# FIGURE 62

MKFLLLVLAALGFLTQVIPASAGGSKCVSNTPGYCRTCCHWGETALFMCNASRKCCISYSFLP
KPDLPQLIGNHWQSRRRNTQRKDKKQQTTVTS

**Important features of the protein:**

**Signal peptide:**

amino acids 1-16

**Transmembrane domain:**

amino acids 1-22

**N-glycosylation site.**

amino acids 50-53

**cAMP- and cGMP-dependent protein kinase phosphorylation site.**

amino acids 79-82

**N-myristoylation site.**

amino acids 23-28

# FIGURE 63

GCGGAGCGCCTGGGAGAGGAGAAGGAGCCGACCTGCCGAG**ATG**GAGGCGACCGGCACCTGGGC
GCTGCTGCTGGCGCTGGCGCTGCTCCTGCTGCTGACGCTGGCGCTGTCCGGGACCAGGGCCCG
AGGCCACCTGCCCCCCGGGCCCACGCCGCTACCACTGCTGGGAAACCTCCTGCAGCTACGGCC
CGGGGCGCTGTATTCAGGGCTCATGCGGCTGAGTAAGAAGTACGGACCGGTGTTCACCATCTA
CCTGGGACCCTGGCGGCCTGTGGTGGTCCTGGTTGGGCAGGAGGCTGTGCGGGAGGCCCTGGG
AGGTCAGGCTGAGGAGTTCAGCGGCCGGGGAACCGTAGCGATGCTGGAAGGGACTTTTGATGG
CCATGGGGTTTTCTTCTCCAACGGGGAGCGGTGGAGGCAGCTGAGGAAGTTTACCATGCTTGC
TCTGCGGGACCTGGGCATGGGGAAGCGAGAAGGCGAGGAGCTGATCCAGGCGGAGGCCCGGTG
TCTGGTGGAGACATTCCAGGGGACAGAAGGACGCCCATTCGATCCCTCCCTGCTGCTGGCCCA
GGCCACCTCCAACGTAGTCTGCTCCCTCCTCTTTGGCCTCCGCTTCTCCTATGAGGATAAGGA
GTTCCAGGCCGTGGTCCGGGCAGCTGGTGGTACCCTGCTGGGAGTCAGCTCCCAGGGGGGTCA
GACCTACGAGATGTTCTCCTGGTTCCTGCGGCCCCTGCCAGGCCCCCACAAGCAGCTCCTCCA
CCACGTCAGCACCTTGGCTGCCTTCACAGTCCGGCAGGTGCAGCAGCACCAGGGGAACCTGGA
TGCTTCGGGCCCCGCACGTGACCTTGTCGATGCCTTCCTGCTGAAGATGGCACAGGAGGAACA
AAACCCAGGCACAGAATTCACCAACAAGAACATGCTGATGACAGTCATTTATTTGCTGTTTGC
TGGGACGATGACGGTCAGCACCACGGTCGGCTATACCCTCCTGCTCCTGATGAAATACCCTCA
TGTCCAAAAGTGGGTACGTGAGGAGCTGAATCGGGAGCTGGGGGCTGGCCAGGCACCAAGCCT
AGGGGACCGTACCCGCCTCCCTTACACCGACGCGGTTCTGCATGAGGCGCAGCGGCTGCTGGC
GCTGGTGCCCATGGGAATACCCCGCACCCTCATGCGGACCACCCGCTTCCGAGGGTACACCCT
GCCCCAGGGCACGGAGGTCTTCCCCCTCCTTGGCTCCATCCTGCATGACCCCAACATCTTCAA
GCACCCAGAAGAGTTCAACCCAGACCGTTTCCTGGATGCAGATGGACGGTTCAGGAAGCATGA
GGCGTTCCTGCCCTTCTCCTTAGGGAAGCGTGTCTGCCTTGGAGAGGGCCTGGCAAAAGCGGA
GCTCTTCCTCTTCTTCACCACCATCCTACAAGCCTTCTCCCTGGAGAGCCCGTGCCCGCCGGA
CACCCTGAGCCTCAAGCCCACCGTCAGTGGCCTTTTCAACATTCCCCCAGCCTTCCAGCTGCA
AGTCCGTCCCACTGACCTTCACTCCACCACGCAGACCAGA**TGA**AGGAAGGCAACTTGGAAGTG
GTGGGTGCCCAGGACGGTGCCTCCAGCCTCAACAGTGGGCATGGACAGGGTTAATGTCTCCAG
AGTGTACACTGCAGGCAGCCACATTTACACGCCTGCAGTTGTTTTCCGGAGTCTGTCCCACGG
CCCACACGCTCACTTGACTCATGCTGCTAAGATGCACAACCGCACACCCATACACAACTACAA
GGGCCACAAAGCAACTGCTGGGTTAGCTTTCCACAGACATAAATATAGTCCATCTGCAATCAC
AAGCACATAGCCAGGTAACCCACCAACTCCCCTGGATCTGCAGCCCACACGTGGGAGTCTGGC
TGTCACCTTCACAAGCCACAGAAACGGCCACACATGTTCACAGCTCACACGCCCTCTCCATTC
ATCGAACTTCTCAGTGTCCCTGTCCCTGGTGCCTGGCACAGGGAACAGCATGCCCCCTCCGGG
GTCATGCCACCCAGAGACTGTCGCTGTCTATGGCCCCAACTCATGCTCCCTCTCTTGGCTACA
CCACTCTCCCAGCCTGTGACCACCGATGTCCACACACCCCCAACCACTTGTCCACACAGCTAC
CCACGTACAACATCGTCCTGGCTCCCCAGAGTATCTTCCCACTGAGACACGCCGCCCCCACAG
AGGCACAGTCCCCAGCCACCTCTGCAACTGCAGCCCTCAGTCACCCCTTTTTAAGCACCCTGA
TTCTACCAAATGCAAACACATCTGGGTCTGCGATTATGCACAGAGACTTTGGACATACGAGGA
CCCTCAGACCGGAGGAACACCTGCCCAACCCCAACACGTGCTTATGTAACCACGTGGAAAGCG
GCCCCTGCTGCCCCTCCACACACACATACACACTCACTGATCTACAGCCCCTGTTCGGCGTCA
GAGTCCCCACTAGACCCAGTGGAAGGGGTTAGAGACCAAGTAGGGGCCAGTTTCCAATTCACC
CTGTCAGGGAGTGAGCCGGATCTGACGTTCCTTGTGACTTAAGGGTCCGGCTTGGGAATTAAA
GTTTGTTTCTGGCCTTTAGCCTAAAAAAAAAAAAAAAAAA

# FIGURE 64

MEATGTWALLLALALLLLLTLALSGTRARGHLPPGPTPLPLLGNLLQLRPGALYSGLMRLSKK
YGPVFTIYLGPWRPVVVLVGQEAVREALGGQAEEFSGRGTVAMLEGTFDGHGVFFSNGERWRQ
LRKFTMLALRDLGMGKREGEELIQAEARCLVETFQGTEGRPFDPSLLLAQATSNVVCSLLFGL
RFSYEDKEFQAVVRAAGGTLLGVSSQGGQTYEMFSWFLRPLPGPHKQLLHHVSTLAAFTVRQV
QQHQGNLDASGPARDLVDAFLLKMAQEEQNPGTEFTNKNMLMTVIYLLFAGTMTVSTTVGYTL
LLLMKYPHVQKWVREELNRELGAGQAPSLGDRTRLPYTDAVLHEAQRLLALVPMGIPRTLMRT
TRFRGYTLPQGTEVFPLLGSILHDPNIFKHPEEFNPDRFLDADGRFRKHEAFLPFSLGKRVCL
GEGLAKAELFLFFTTILQAFSLESPCPPDTLSLKPTVSGLFNIPPAFQLQVRPTDLHSTTQTR

**Important features of the protein:**

**Signal peptide:**

amino acids 1-28

**Transmembrane domain:**

amino acids 294-313

**Glycosaminoglycan attachment site.**

amino acids 99-103

**cAMP- and cGMP-dependent protein kinase phosphorylation site.**

amino acids 128-132

**N-myristoylation sites.**

amino acids 51-57, 109-115, 115-121, 188-194, 207-213, 257-263, 284-290, 339-345, 370-376, 444-450

**Amidation sites.**

amino acids 140-144, 435-439

**Leucine zipper pattern.**

amino acids 32-54, 39-61

**Cytochrome P450 cysteine heme-iron ligand signature.**

amino acids 433-443

# FIGURE 65

CGGACGCGTGGGGCCGT**ATG**CGCGGCTCTGTGGAGTGCACCTGGGGTTGGGGGCACTGTGCCC
CCAGCCCCCTGCTCCTTTGGACTCTACTTCTGTTTGCAGCCCCATTTGGCCTGCTGGGGGAGA
AGACCCGCCAGGTGTCTCTGGAGGTCATCCCTAACTGGCTGGGCCCCCTGCAGAACCTGCTTC
ATATACGGGCAGTGGGCACCAATTCCACACTGCACTATGTGTGGAGCAGCCTGGGGCCTCTGG
CAGTGGTAATGGTGGCCACCAACACCCCCCACAGCACCCTGAGCATCAACTGGAGCCTCCTGC
TATCCCCTGAGCCCGATGGGGGCCTGATGGTGCTCCCTAAGGACAGCATTCAGTTTTCTTCTG
CCCTTGTTTTTACCAGGCTGCTTGAGTTTGACAGCACCAACGTGTCCGATACGGCAGCAAAGC
CTTTGGGAAGACCATATCCTCCATACTCCTTGGCCGATTTCTCTTGGAACAACATCACTGATT
CATTGGATCCTGCCACCCTGAGTGCCACATTTCAAGGCCACCCCATGAACGACCCTACCAGGA
CTTTTGCCAATGGCAGCCTGGCCTTCAGGGTCCAGGCCTTTTCCAGGTCCAGCCGACCAGCCC
AACCCCCTCGCCTCCTGCACACAGCAGACACCTGTCAGCTAGAGGTGGCCCTGATTGGAGCCT
CTCCCCGGGGAAACCGTTCCCTGTTTGGGCTGGAGGTAGCCACATTGGGCCAGGGCCCTGACT
GCCCCTCAATGCAGGAGCAGCACTCCATCGACGATGAATATGCACCGGCCGTCTTCCAGTTGG
ACCAGCTACTGTGGGGCTCCCTCCCATCAGGCTTTGCACAGTGGCGACCAGTGGCTTACTCCC
AGAAGCCGGGGGGCCGAGAATCAGCCCTGCCCTGCCAAGCTTCCCCTCTTCATCCTGCCTTAG
CATACTCTCTTCCCCAGTCACCCATTGTCCGAGCCTTCTTTGGGTCCCAGAATAACTTCTGTG
CCTTCAATCTGACGTTCGGGGCTTCCACAGGCCCTGGCTATTGGGACCAACACTACCTCAGCT
GGTCGATGCTCCTGGGTGTGGGCTTCCCTCCAGTGGACGGCTTGTCCCCACTAGTCCTGGGCA
TCATGGCAGTGGCCCTGGGTGCCCCAGGGCTCATGCTGCTAGGGGGCGGCTTGGTTCTGCTGC
TGCACCACAAGAAGTACTCAGAGTACCAGTCCATAAAT**TAA**GGCCCGCTCTCTGGAGGGAAGG
ACATTACTGAACCTGTCTTGCTGTGCCTCGAAACTCTGGAGGTTGGAGCATCAAGTTCCAGCC
GGCCCCTTCACTCCCCCATCTTGCTTTTCTGTGGAACCTCAGAGGCCAGCCTCGACTTCCTGG
AGACCCCCAGGTGGGGCTTCCTTCATACTTTGTTGGGGGACTTTGGAGGCGGGCAGGGGACAG
GGCTATTGATAAGGTCCCCTTGGTGTTGCCTTCTTGCATCTCCACACATTTCCCTTGGATGGG
ACTTGCAGGCCTAAATGAGAGGCATTCTGACTGGTTGGCTGCCCTGGAAGGCAAGAAAATAGA
TTTATTTTTTTCACAGGGAAAAAAAAAAAA

# FIGURE 66

MRGSVECTWGWGHCAPSPLLLWTLLLFAAPFGLLGEKTRQVSLEVIPNWLGPLQNLLHIRAVG
TNSTLHYVWSSLGPLAVVMVATNTPHSTLSINWSLLLSPEPDGGLMVLPKDSIQFSSALVFTR
LLEFDSTNVSDTAAKPLGRPYPPYSLADFSWNNITDSLDPATLSATFQGHPMNDPTRTFANGS
LAFRVQAFSRSSRPAQPPRLLHTADTCQLEVALIGASPRGNRSLFGLEVATLGQGPDCPSMQE
QHSIDDEYAPAVFQLDQLLWGSLPSGFAQWRPVAYSQKPGGRESALPCQASPLHPALAYSLPQ
SPIVRAFFGSQNNFCAFNLTFGASTGPGYWDQHYLSWSMLLGVGFPPVDGLSPLVLGIMAVAL
GAPGLMLLGGGLVLLLHHKKYSEYQSIN

**N-glycosylation sites:**
amino acids 65-69, 95-99, 134-138, 159-163, 187-191, 230-234, 333-337

**cAMP- and cGMP-dependent protein kinase phosphorylation site:**
amino acids 397-401

**Casein kinase II phosphorylation sites:**
amino acids 151-155, 249-253, 255-259

**N-myristoylation sites:**
amino acids 3-9, 63-69, 235-241, 273-279, 292-298, 324-330

**Leucine zipper pattern.**
amino acids 371-393

# FIGURE 67

CGGGACAGGCGCGTGAGGCCACAACACATGCGTGTATCTTGCTTGGGCTATCTTCCCTGCTCTGCCACGCCGGGT
CTGGAGAAGGGGTTTCAGCCCCAGGACATTTACTGAGAGTCGGCGAATATTGGGAGCCGCG**ATG**TTCCCCCTTCG
GGCCCTGTGGTTGGTCTGGGCGCTTCTAGGAGTGGCCGGATCATGCCCGGAGCCGTGCGCCTGCGTGGACAAGTA
CGCTCACCAGTTCGCGGACTGCGCTTACAAAGAGTTGCGTGAGGTGCCGGAAGGACTGCCTGCCAACGTGACGAC
GCTTAGTCTGTCCGCGAACAAGATCACTGTGCTGCGGCGCGGGGCCTTCGCCGACGTCACACAGGTCACGTCGCT
GTGGCTGGCGCACAATGAGGTGCGCACCGTGGAGCCAGGCGCACTGGCCGTGCTGAGTCAGCTCAAGAACCTCGA
TCTGAGCCACAACTTCATATCCAGCTTTCCGTGGAGCGACCTGCGCAACCTGAGCGCGCTGCAGCTGCTCAAAAT
GAACCACAACCGCCTGGGCTCTCTGCCCCGGGACGCACTCGGTGCGCTACCCGACCTGCGTTCCCTGCGCATCAA
CAACAACCGGCTGCGTACGCTGGCGCCTGGCACCTTCGACGCGCTTAGCGCGCTGTCACACTTGCAACTCTATCA
CAATCCCTTCCACTGCGGCTGCGGCCTTGTGTGGCTGCAGGCCTGGGCCGCGAGCACCCGGGTGTCCTTACCCGA
GCCCGACTCCATTGCTTGTGCCTCGCCTCCCGCGCTGCAGGGGGTGCCGGTGTACCGCCTGCCCGCCCTGCCCTG
TGCACCGCCCAGCGTGCATCTGAGTGCCGAGCCACCGCTTGAAGCACCCGGCACCCCACTGCGCGCAGGACTGGC
GTTCGTGTTACACTGCATCGCCGACGGCCACCCTACGCCTCGCCTGCAATGGCAACTTCAGATCCCCGGTGGCAC
CGTAGTCTTAGAGCCACCGGTTCTGAGCGGGGAGGACGACGGGGTTGGGGCGGAGGAAGGAGAGGGAGAAGGAGA
TGGGGATTTGCTGACGCAGACCCAAGCCCAAACGCCGACTCCAGCACCCGCTTGGCCGGCGCCCCCAGCCACACC
GCGCTTCCTGGCCCTCGCAAATGGCTCCCTGTTGGTGCCCCTCCTGAGTGCCAAGGAGGCGGGCGTCTACACTTG
CCGTGCACACAATGAGCTGGGCGCCAACTCTACGTCAATACGCGTGGCGGTGGCAGCAACCGGGCCCCCAAAAACA
CGCGCCTGGCGCCGGGGGAGAACCCGACGGACAGGCCCCGACCTCTGAGCGCAAGTCCACAGCCAAGGGCCGGGG
CAACAGCGTCCTGCCTTCCAAACCCGAGGGCAAAATCAAAGGCCAAGGCCTGGCCAAGGTCAGCATTCTCGGGGA
GACCGAGACGGAGCCGGAGGAGGACACAAGTGAGGGAGAGGAGGCCGAAGACCAGATCCTCGCGGACCCGGCGGA
GGAGCAGCGCTGTGGCAACGGGGACCCCTCTCGGTACGTTTCTAACCACGCGTTCAACCAGAGCGCAGAGCTCAA
GCCGCACGTCTTCGAGCTGGGCGTCATCGCGCTGGATGTGGCGGAGCGCGAGGCGCGGGTGCAGCTGACTCCGCT
GGCTGCGCGCTGGGGCCCTGGGCCCGGCGGGGCTGGCGGAGCCCCGCGACCCGGGCGGCGACCCCTGCGCCTACT
CTATCTGTGTCCAGCGGGGGGCGGCGCGGCAGTGCAGTGGTCCCGCGTAGAGGAAGGCGTCAACGCCTACTGGTT
CCGCGGCCTGCGGCCGGGTACCAACTACTCCGTGTGCCTGGCGCTGGCGGGCGAAGCCTGCCACGTGCAAGTGGT
GTTTTCCACCAAGAAGGAGCTCCCATCGCTGCTGGTCATAGTGGCAGTGAGCGTATTCCTCCTGGTGCTGGCCAC
AGTGCCCCTTCTGGGCGCCGCCTGCTGCCATCTGCTGGCTAAACACCCGGGCAAGCCCTACCGTCTGATCCTGCG
GCCTCAGGCCCCTGACCCTATGGAGAAGCGCATCGCCGCAGACTTCGACCCGCGTGCTTCGTACCTCGAGTCCGA
GAAAAGCTACCCGGCAGGCGGCGAGGCGGGCGCGAGGAGCCAGAGGACGTGCAGGGGGAGGGCCTTGATGAAGA
CGCGGAGCAGGGAGACCCAAGTGGGGACCTGCAGAGAGAGGAGAGCCTGGCGGCCTGCTCACTGGTGGAGTCCCA
GTCCAAGGCCAACCAAGAGGAGTTCGAGGCGGGCTCTGAGTACAGCGATCGGCTGCCCCTGGGCGCCGAGGCGGT
CAACATCGCCCAGGAGATTAATGGCAACTACAGGCAGACGGCAGGC**TGA**ACCTCCGCCCGTCCGGCCCGCCCATT
CCCGACCTCCACCTAGGGTGCCTGGGAGCAGCAGTCTAGGGCTGGCAGGACTTATGTCCCCCGTCCCCAACCTTC
ACCTACTCCTCCCCCTTACTACTCCCCAACCTTGACTACCAGGGACTTCTATTAGGGAGTGGGCCGATTTCACCA
GTCCCTGCTACCCACGGCTGCCATTCTCCCTGCGGGCTGAATCCCCTTCCCCGCCAAGCACAGTGTTTATCTTAC
CCCATGCAAGACTCCACCCGCAGACGGTGGGCGATATCTATGTCCCTCCATTCCCGTCGCGATTATCTGCGAAAT
CCACCCCGCAGCCCGCCCCACCGTGGGCTCTGGAGCCAGAGGAAACGAGCGAAGACTTTGGAAACCTCGCGGTAA
CGCGGTGGTTTCGGGGGCCAGCCAAGGCCAGTGGAGTGCTGTGGGGTCCCACCTCGACCCCTCCTCCTCCCTTTC
TTTCTTTCCTTTTTTTTTATTTTTTAATTTTATTTATTTATTTATTTATTTTTTGACGGAGTCTTGGTCTGTCGC
CAGGCTGGAGTGCAGTGGCGCGATCTCGGCTCACTGCATCTTCCGCCTCCCGGGTTCAAGCGATTCTCCTGCCTC
AGCCTGCCTAGTAGCTGGGACTACAGGCGCGCGCCACCACGACCAGCTAATTTCTTCTATTTTTAGTAGAGACGG
GGTTTCACCATGTTGGCCAGGATGGTCTGGATCTCTTGACCTCAGGTGATCCATCTGCCTCGGCCTCTCAAAGTG
CTGGGATTACAGGCGTGAGGCACCGCGCCCGGCCCCTCCTCCCTTTCAATCCCTACTCCCAGAAGCCGGGATTCG
TGGCAACCCCTAGTTTTTAGTTCCAAAGCCTCCTGCCGGCAGGGAACCAAATCCTTCTGTCCTCCCACCCCCACC
CCACTTCTGGCCAGTTGGAGTCCAGCCCGGTGCCTGGGGCGCCTTTCAGCTCCGCGCTCAGATTTTCCTGTTTTC
GTTGTTTTCAAAGACAGCGACATTTCGGGTCTGGTGCTAACACCCCCTTCCCAGCCTCTGGGAAAATCGAGTGTG
TGTGTCGGGGGGTAGGGAGGGAATGCGTTTTCTGTCGTCTCTCTCCTAACTTAAAGCGCCGCAGGACCGCGCGCC
CCTTGGCGGCTGAGCCTGTGGACTTGGTCGCGGGCCAATTTCGTTGTCCGTGTGTTGGGCTTTCCGGAGGTCTGT
GCGCCCAACAGCGCCGCTCCCGCGGCTCCACCCGACCCAGACCCTAGCTGGAAAGCGCCGGAGGCGGAGGAAGCT
GACTGTGGCCTCCCGGGCCGCGGCTCTCTGGAGGGCTCGCGCCCTAGTTCGCACAAAGCCTGCTCGTGACTGTGC
GACTGTGCGACGGGATCCGGATGGAGCCGAGCCCCTCCGTCCTCGCGTCTCGGTCCTCGCGTCGCCCCGCCCCAC
CCGCCCCTGCTTCGGCGGGAATCGTGTTTGCCCGGCGTGTAGTCCCTGACAAGCGTGCCCTGTAGGAGAAAAGTC
TGTGTCCTGTGAAGTGTGACCGTGTAGTGTAGGGGGGCGGGCGGGGGGGCGGATGGGCGGGGAGGGAGGGAAGGG
GAGGGGCGCGGCGCGGCGACTCGGGGCGGGGTTCTTTTTCCATTTTGAAAGAAAGCGTCGGGGTTGGGGTGGGG
GGAGTTTCAGTCCTCGGGATCAGCCCTCTCCGCGAAGCGCAGCACAAGCGCGGGCCTGGACGGAGTAGCCCCCC
GGAGCCCGTGCCCTTTTCTAAACGCGTCTGTATGCAGTCAATAAAACAATCGATTTGAAA

# FIGURE 68

MFPLRALWLVWALLGVAGSCPEPCACVDKYAHQFADCAYKELREVPEGLPANVTTLSLSANKI
TVLRRGAFADVTQVTSLWLAHNEVRTVEPGALAVLSQLKNLDLSHNFISSFPWSDLRNLSALQ
LLKMNHNRLGSLPRDALGALPDLRSLRINNNRLRTLAPGTFDALSALSHLQLYHNPFHCGCGL
VWLQAWAASTRVSLPEPDSIACASPPALQGVPVYRLPALPCAPPSVHLSAEPPLEAPGTPLRA
GLAFVLHCIADGHPTPRLQWQLQIPGGTVVLEPPVLSGEDDGVGAEEGEGEGDGDLLTQTQAQ
TPTPAPAWPAPPATPRFLALANGSLLVPLLSAKEAGVYTCRAHNELGANSTSIRVAVAATGPP
KHAPGAGGEPDGQAPTSERKSTAKGRGNSVLPSKPEGKIKGQGLAKVSILGETETEPEEDTSE
GEEAEDQILADPAEEQRCGNGDPSRYVSNHAFNQSAELKPHVFELGVIALDVAEREARVQLTP
LAARWGPGPGGAGGAPRPGRRPLRLLYLCPAGGGAAVQWSRVEEGVNAYWFRGLRPGTNYSVC
LALAGEACHVQVVFSTKKELPSLLVIVAVSVFLLVLATVPLLGAACCHLLAKHPGKPYRLILR
PQAPDPMEKRIAADFDPRASYLESEKSYPAGGEAGGEEPEDVQGEGLDEDAEQGDPSGDLQRE
ESLAACSLVESQSKANQEEFEAGSEYSDRLPLGAEAVNIAQEINGNYRQTAG

**Important features of the protein:**

**Signal peptide:**

amino acids 1-19

**Transmembrane domain:**

amino acids 587-610

**N-glycosylation sites.**

amino acids 52-55, 121-124, 337-340, 364-367, 474-477, 563-566

**cAMP- and cGMP-dependent protein kinase phosphorylation site.**

amino acids 397-400

**Casein kinase II phosphorylation sites.**

amino acids 19-23, 202-205, 289-292, 246-249, 411-414, 431-434, 433-436, 440-443, 544-547, 583-586, 650-653, 700-703

**N-myristoylation sites.**

amino acids 15-20, 48-53, 165-170, 296-301, 351-356, 362-367, 390-395, 419-424, 514-519, 536-541, 557-562, 561-566, 610-615, 661-666, 716-721

**Amidation site.**

amino acids 522-525

**Prokaryotic membrane lipoprotein lipid attachment sites.**

amino acids 10-20, 603-613

# FIGURE 69

GGCGGCGGGAGCAGCGAAGGGGGCGGCAGGGATCCTCCAGGCTGCCGGCTGGGAAGGCGTGGG
CGACCCGGTGTGTGGCGCGCCCAGAGCCCCGCGTTTCAGCCCTAGGGAAGGAAGCCAGTTGAG
GGAAGTTCTCCATGAATGTACGTCACA**ATG**ATGATGACCGACCAAATCCCTCTGGAACTGCCA
CCATTGCTGAACGGAGAGGTAGCCATGATGCCCCACTTGGTGAATGGAGATGCAGCTCAGCAT
GTTATTCTCGTTCAAGTTAATCCAGGTGAGACTTTCACAATAAGAGCAGAGGATGGAACACTT
CAGTGCATTCAAGGACCTGCTGAAGTTCCCATGATGTCACCCAATGGATCCATTCCTCCCATT
CATGTGCCTCCAGGTTATATCTCACAGGTGATTGAAGATAGTACTGGAGTCCGCCGGGTGGTG
GTCACACCCCAGTCTCCTGAGTGTTATCCCCCAAGCTACCCCTCAGCCATGTCTCCAACCCAT
CATCTCCCTCCCTATCTGACTCACCATCCACATTTTATTCATAACTCACACACGGCTTACTAC
CCACCTGTTACCGGACCTGGAGATATGCCGCCTCAGTTTTTTCCCCAGCATCATCTTCCCCAC
ACAATATATGGTGAGCAAGAAATTATACCATTTTATGGAATGTCAAGCTACATCACCCGAGAA
GACCAGTACAGCAAGCCTCCGCACAAAAAACTGAAAGACCGCCAGATCGATCGCCAGAACCGC
CTCAACAGCCCTCCTTCTTCTATCTACAAAAGCAGCTGCACAACAGTATACAATGGCTATGGG
AAGGGCCATAGTGGTGGAAGTGGCGGAGGCGGCAGCGGTAGTGGTCCCGGAATTAAGAAAACA
GAGCGACGAGCAAGAAGCAGCCCAAAGTCGAATGATTCAGACTTGCAAGAATATGAGTTGGAA
GTAAAGAGGGTGCAAGACATTCTTTCGGGAATAGAGAAACCACAGGTTTCTAATATTCAGGCA
AGAGCAGTTGTGTTGTCCTGGGCTCCCCCTGTTGGACTTTCCTGTGGACCCCACAGTGGTCTT
TCCTTCCCCTACAGTTACGAGGTGGCCTTATCAGACAAAGGACGAGATGGAAAATACAAGATA
ATTTACAGTGGAGAAGAATTAGAATGTAACCTGAAAGATCTTAGACCAGCAACAGATTATCAT
GTGAGGGTGTATGCCATGTACAATTCCGTAAAGGGATCCTGCTCCGAGCCTGTTAGCTTCACC
ACCCACAGCTGTGCACCCGAGTGTCCTTTCCCCCCTAAGCTGGCACATAGGAGCAAAAGTTCA
CTAACCCTGCAGTGGAAGGCACCAATTGACAACGGTTCAAAAATCACCAACTACCTTTTAGAG
TGGGATGAGGGAAAAAGAAATAGTGGTTTCAGACAGTGCTTCTTCGGGAGCCAGAAGCACTGC
AAGTTGACAAAGCTTTGTCCGGCAATGGGGTACACATTCAGGCTGGCCGCTCGAAACGACATT
GGCACCAGTGGTTATAGCCAAGAGGTGGTGTGCTACACATTAGGAAATATCCCTCAGATGCCT
TCTGCACCAAGGCTGGTTCGAGCTGGCATCACATGGGTCACGTTGCAGTGGAGTAAGCCAGAA
GGCTGTTCACCCGAGGAAGTGATCACCTACACCTTGGAAATTCAGGAGGATGAAAATGATAAC
CTTTTCCACCCAAAATACACTGGAGAGGATTTAACCTGTACTGTGAAAAATCTCAAAAGAAGC
ACACAGTATAAATTCAGGCTGACTGCTTCTAATACGGAAGGAAAAAGCTGTCCAAGCGAAGTT
CTTGTTTGTACGACGAGTCCTGACAGGCCTGGACCTCCTACCAGACCGCTTGTCAAAGGCCCA
GTTACATCTCATGGCTTTAGTGTCAAATGGGATCCCCCTAAGGACAATGGTGGTTCAGAAATC
CTCAAGTACTTGCTAGAGATTACTGATGGAAATTCTGAAGGTGAAGTTTTTGGCAATTGTTTT
ATTCAAATCCAA**TAG**CAAGCTCTGTTTTCTAATATAGTAAATGTCTTTATAGTAATAGTGAGT
AATCATTAATTCTAAAGATAGAATTATTATTACAATAAACAAACTTTAGTCACATATTGGCAG
TTTTTCTATTTCAAACACAGCACCAGAGATCAGAGTCTACTTGAAACTTACATTTGTGTTATT
TAACAATTTTTCTGTATCTTTTTCATTGGTGTTTTGTTTTGTTTATCTTTTGTTTTTGTTTCT
TTGGTTTGGTTTGTTTTTGTTTTGTTTTTTGAGATACGATCTCTGTCACACAGGCTGGAGGGC
AGTGGCACAGACATGGCCCATTGCAGTCTCAGACTCCTGGGCTTAAGTGACTCTTCTGCCACA
GAAGATGAGGAAGAATACATTTTTCATAGTGATGGGGTCTCACTATGTTATCTAGGCTGGTCT
CAAACTCCTGGCCTCAAGCAACCCTCCACCTTGGCCTCCCAAAGTGCTGGGACTATAGACATG
AATCACCACACTCAGCTTCCATGTCTTTTTATGAACTAGGGTTCCTAATTAATCAGATAAATT
TGGTATTTTCATCTCCTAACTTGCCATATGTTTCTGGAAATTCTTATAAGCAGCCGAGAGTG
GTGGCTCACGCTGTAGTCCCAGCACTTTGGGAGGCTGAGGTGGGTGGTCAGGAGATCAAGACC
ATCCTGGCCAACATGGTGAAACCCCGTCTCTACTAAAAATACAAAAATTAGCTGGGTGTGGTG
GCAGGCACCTGTAGTCCCAGCTACTTGGGAGGCTGAGGCAGAAGAATTGCTTGAACCCAGCAG
GCGGAGGTTGCAGTGAGCTGAGATTGCACCACTGCACTCCAGCCTGGTGACAGAGTGAGACTC
TGTCTCAAAAAAAAAAAA

# FIGURE 70

MMMTDQIPLELPPLLNGEVAMMPHLVNGDAAQHVILVQVNPGETFTIRAEDGTLQCIQGPAEV
PMMSPNGSIPPIHVPPGYISQVIEDSTGVRRVVVTPQSPECYPPSYPSAMSPTHHLPPYLTHH
PHFIHNSHTAYYPPVTGPGDMPPQFFPQHHLPHTIYGEQEIIPFYGMSSYITREDQYSKPPHK
KLKDRQIDRQNRLNSPPSSIYKSSCTTVYNGYGKGHSGGSGGGGSGSGPGIKKTERRARSSPK
SNDSDLQEYELEVKRVQDILSGIEKPQVSNIQARAVVLSWAPPVGLSCGPHSGLSFPYSYEVA
LSDKGRDGKYKIIYSGEELECNLKDLRPATDYHVRVYAMYNSVKGSCSEPVSFTTHSCAPECP
FPPKLAHRSKSSLTLQWKAPIDNGSKITNYLLEWDEGKRNSGFRQCFFGSQKHCKLTKLCPAM
GYTFRLAARNDIGTSGYSQEVVCYTLGNIPQMPSAPRLVRAGITWVTLQWSKPEGCSPEEVIT
YTLEIQEDENDNLFHPKYTGEDLTCTVKNLKRSTQYKFRLTASNTEGKSCPSEVLVCTTSPDR
PGPPTRPLVKGPVTSHGFSVKWDPPKDNGGSEILKYLLEITDGNSEGEVFGNCFIQIQ

**Important features of the protein:**

**N-glycosylation sites.**

amino acids 69-73, 254-258, 401-405

**Glycosaminoglycan attachment sites.**

amino acids 229-233, 234-238, 236-240

**cAMP- and cGMP-dependent protein kinase phosphorylation sites.**

amino acids 416-420, 535-539

**Tyrosine kinase phosphorylation site.**

amino acids 319-326

**N-myristoylation sites.**

amino acids 52-58, 227-233, 228-234, 230-236, 231-237, 232-238, 235-241, 239-245, 402-408, 610-616

**Amidation site.**

amino acids 414-418

**Prokaryotic membrane lipoprotein lipid attachment site.**

amino acids 290-301

**ATP/GTP-binding site motif A (P-loop).**

amino acids 546-554

**CUB domain proteins profile.**

amino acids 294-301

# FIGURE 71

AAGTCATTCAGTGGATGTGATCTTGGCTCACAGGGGACG**ATG**TCAAGCTCTTCCTGGCTCCTTCTCAGCCTTGTT
GCTGTAACTGCTGCTCAGTCCACCATTGAGGAACAGGCCAAGACATTTTTGGACAAGTTTAACCACGAAGCCGAA
GACCTGTTCTATCAAAGTTCACTTGCTTCTTGGAATTATAACACCAATATTACTGAAGAGAATGTCCAAAACATG
AATAATGCTGGGGACAAATGGTCTGCCTTTTTAAAGGAACAGTCCACACTTGCCCAAATGTATCCACTACAAGAA
ATTCAGAATCTCACAGTCAAGCTTCAGCTGCAGGCTCTTCAGCAAAATGGGTCTTCAGTGCTCTCAGAAGACAAG
AGCAAACGGTTGAACACAATTCTAAATACAATGAGCACCATCTACAGTACTGGAAAAGTTTGTAACCCAGATAAT
CCACAAGAATGCTTATTACTTGAACCAGGTTTGAATGAAATAATGGCAAACAGTTTAGACTACAATGAGAGGCTC
TGGGCTTGGGAAAGCTGGAGATCTGAGGTCGGCAAGCAGCTGAGGCCATTATATGAAGAGTATGTGGTCTTGAAA
AATGAGATGGCAAGAGCAAATCATTATGAGGACTATGGGGATTATTGGAGAGGAGACTATGAAGTAAATGGGGTA
GATGGCTATGACTACAGCCGCGGCCAGTTGATTGAAGATGTGGAACATACCTTTGAAGAGATTAAACCATTATAT
GAACATCTTCATGCCTATGTGAGGGCAAAGTTGATGAATGCCTATCCTTCCTATATCAGTCCAATTGGATGCCTC
CCTGCTCATTTGCTTGGTGATATGTGGGGTAGATTTTGGACAAATCTGTACTCTTTGACAGTTCCCTTTGGACAG
AAACCAAACATAGATGTTACTGATGCAATGGTGGACCAGGCCTGGGATGCACAGAGAATATTCAAGGAGGCCGAG
AAGTTCTTTGTATCTGTTGGTCTTCCTAATATGACTCAAGGATTCTGGGAAAATTCCATGCTAACGGACCCAGGA
AATGTTCAGAAAGCAGTCTGCCATCCCACAGCTTGGGACCTGGGGAAGGGCGACTTCAGGATCCTTATGTGCACA
AAGGTGACAATGGACGACTTCCTGACAGCTCATCATGAGATGGGGCATATCCAGTATGATATGGCATATGCTGCA
CAACCTTTTCTGCTAAGAAATGGAGCTAATGAAGGATTCCATGAAGCTGTTGGGGAAATCATGTCACTTTCTGCA
GCCACACCTAAGCATTTAAAATCCATTGGTCTTCTGTCACCCGATTTTCAAGAAGACAATGAAACAGAAATAAAC
TTCCTGCTCAAACAAGCACTCACGATTGTTGGGACTCTGCCATTTACTTACATGTTAGAGAAGTGGAGGTGGATG
GTCTTTAAAGGGGAAATTCCCAAAGACCAGTGGATGAAAAAGTGGTGGGAGATGAAGCGAGAGATAGTTGGGGTG
GTGGAACCTGTGCCCCATGATGAAACATACTGTGACCCCGCATCTCTGTTCCATGTTTCTGATGATTACTCATTC
ATTCGATATTACACAAGGACCCTTTACCAATTCCAGTTTCAAGAAGCACTTTGTCAAGCAGCTAAACATGAAGGC
CCTCTGCACAAATGTGACATCTCAAACTCTACAGAAGCTGGACAGAAACTGTTG**TAA**GAAATACCTCAAAATGTT
GAACCTCTCCTAGTATTCAGTATTACTCATTTCCATGCCTAGGTTTGTATTTGATTTCTTTGTTCTAAAAAGAAA
ATTTTATGGCCTCAAAATGTCCTCATTTACAAACCAAACATTTAATTTGTGGTCAGACAGGAACCTAGACCATAC
AACAATTGGGTGGGCCACCTCTTTTCTCCCTATCATAACTACAGCCCTCTCTTCCTGGTAATTGGAAGGAAAGAG
CGGTTTAGGGTGGAATATATCTGTTAATATGCATTCTTTTCTTATCTGCCAGAAGCAAATTTAGCCAAGTCAAAG
AGAAGAAACCATAGATCATAGATGTAAATATATGTACATCTGGAACCCCTCAAAAGGCCCTGAACCCCCTTTTTT
TGTGTAGCAATATGCTGAGGCTTGGAAAATCAGAACCCTGGACCCTAGCATTGGAAAATGTTGTAGGAGCAAGAA
CATGAATGTAAGGCCACTGCTCAACTACTTTGAGCCCTTATTTACCTGGCTGAAAGACCAGAACAAGAATTCTTT
TGTGGGATGGAGTACCGACTGGAGTCCATATGCAGACCCAAAGCATCAAAGTGAGGATAAGCCTAAAATCAGCTC
TTGGAGATAAAGCATATGAATGGAACGACAATGAAATGTACCTGTTCCGATCATCTGTTGCATATGCTATGAGGC
AGTACTTTTTAAAAGTAAAAAATCAGATGATTCTTTTTGGGGAGGAGGATGTGCGAGTGGCTAATTTGAAACCAA
GAATCTCCTTTAATTTCTTTGTCACTGCACCTAAAAATGTGTCTGATATCATTCCTAGAACTGAAGTTGAAAAGG
CCATCAGGATGTCCCGGAGCCGTATCAATGATGCTTTCCGTCTGAATGACAACAGCCTAGAGTTTCTGGGGATAC
AGCCAACACTTGGACCTCCTAACCAGCCCCCTGTTTCCATATGGCTGATTGTTTTTGGAGTTGTGATGGGAGTGA
TAGTGGTTGGCATTGTCATCCTGATCTTCACTGGGATCAGAGATCGGAAGAAGAAAAATAAAGCAAGAAGTGGAG
AAAATCCTTATGCCTCCATCGATATTAGCAAAGGAGAAAATAATCCAGGATTCCAAAACACTGATGATGTTCAGA
CCTCCTTTTAGAAAAATCTATGTTTTTCCTCTTGAGGTGATTTTGTTGTATGTAAATGTTAATTTCATGGTATAG
AAAATATAAGATGATAAAGATATCATTAAATGTCAAAACTATGACTCTGTTCAGAAAAAAAATTGTCCAAAGACA
ACATGGCCAAGGAGAGAGCATCTTCATTGACATTGCTTTCAGTATTTATTTCTGTCTCTGGATTTGACTTCTGTT
CTGTTTCTTAATAAGGATTTTGTATTAGAGTATATTAGGGAAAGTGTGTATTTGGTCTCACAGGCTGTTCAGGGA
TAATCTAAATGTAAATGTCTGTTGAATTTCTGAAGTTGAAAACAAGGATATATCATTGGAGCAAGTGTTGGATCT
TGTATGGAATATGGATGGATCACTTGTAAGGACAGTGCCTGGGAACTGGTGTAGCTGCAAGGATTGAGAATGGCA
TGCATTAGCTCACTTTCATTTAATCCATTGTCAAGGATGACATGCTTTCTTCACAGTAACTCAGTTCAAGTACTA
TGGTGATTTGCCTACAGTGATGTTTGGAATCGATCATGCTTTCTTCAAGGTGACAGGTCTAAAGAGAGAAGAATC
CAGGGAACAGGTAGAGGACATTGCTTTTTCACTTCCAAGGTGCTTGATCAACATCTCCCTGACAACACAAAACTA
GAGCCAGGGGCCTCCGTGAACTCCCCAGAGCATGCCTGATAGAAACTCATTTCTACTGTTCTCTAACTGTGGAGT
GAATGGAAATTCCAACTGTATGTTCACCCTCTGAAGTGGGTACCCAGTCTCTTAAATCTTTTGTATTTGCTCACA
GTGTTTGAGCAGTGCTGAGCACAAAGCAGACACTCAATAAATGCTAGATTTACAAAA

# FIGURE 72

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSDDYSFIRYYTRTLYQFQFQEALCQAAKHEGPLHKCDISNSTEAGQKLL

**Important features of the protein:**

**Signal peptide:**

amino acids 1-17

**N-glycosylation sites.**

amino acids 53-57, 90-94, 103-107, 322-326, 432-438, 546-550

**N-myristoylation sites.**

amino acids 260-266, 286-292, 395-401

**Cell attachment sequence.**

amino acids 204-207

**Neutral zinc metallopeptidases, zinc-binding region signature.**

amino acids 371-381

# FIGURE 73

CCCACGCGTCCGAGCGGGGTGGACAAGTGGCGTGTGTGCTGCGACCCCGAGGGAAG**ATG**AACG
GGACGCGGAACTGGTGTACCCTGGTGGACGTGCACCCAGAGGACCAGGCGGCGGCGGGCAGGA
AGACCTATGCCATGGTGTCCAGCCACTCAGCTGGTCATTCTCTGGCTTCAGAACTGGTGGAGT
CCCATGATGGACATGAGGAGATCATTAAGGTGTACTTGAAGGGGAGGTCTGGAGACAAGATGA
TTCACGAGAAGAATATTAACCAGCTGAAGAGTGAGGTCCAGTACATCCAGGAGGCCAGGAACT
GCCTACAGAAGCTCCGGGAGGATATAAGTAGCAAGCTTGACAGGAACCTAGGAGATTCTCTCC
ATCGACAGGAGATACAGGTGGTGCTAGAAAAGCCAAATGGCTTTAGTCAGAGTCCCACAGCCC
TGTACAGCAGCCCACCTGAGGTGGACACCTGTATAAATGAGGATGTTGAGAGCTTGAGGAAGA
CGGTGCAGGACTTGCTGGCCAAGCTTCAGGAGGCCAAGCGGCAACACCAGTCAGACTGTGTGG
CTTTTGAGGTCACACTCAGCCGGTACCAGAGGGAAGCAGAACAAAGTAATGTGGCCCTTCAGA
GAGAGGAGGACAGATGTCCAGAG**TGA**TTGGAGAATGTCCTGGGGGAATGAAGTTCCTTCCACA
AACACAGCTCAGTTCTTAGCAACAAACTGTTTGTTTTTCTACTTGCTCCATCTGCAGCCTACG
CTGCCCTGGCCTCCTGCAGACAGATAGTGGGGTTACCTGGCAAGGCCTGGTGAGAGCCAGTGA
ACCTAAGCTTTGACTGGGTGGCCTTGTCTTTCTGGGGAGGAGGGAATGTACATTCAGGGAGTA
GCCTTTTGCGGAAAAATTCTCTAGGGCTACAGACAGTCATGTGTGACTTCTCTCTGCTGTGAA
AACTCCCAGAGTCTCTTTAGGGATTTTCCCTAAGGTGTACCACCAGGCACACCTCAGTCTTCT
TGACCCAGAGCCTGAAAACTGTTTTCACTGGGTTCCACCAGTCCCAGCAAAATCCTCTTTGTA
TTTATTTTGCTAAGTTATTGGTGGTTTTGCTTACATCTCATGATTGATATAATACCAAAGTTC
TATAGCCTTCTCTTGCAGTATTTGGATTTGCTTGAAACCGGGAAAACTGTTCCCATTAGGCTT
GTTAATGTCAGAGTGACACTATTATGAATCTTTCTCTCCCTTTCCTCTGCCTGTTTCTTCTCT
CTTTCTCCTTCAAACTTGCTCTGCAGCTAAGGAAGGTGAGTCTACTTTCCCTGAGGCTTTGGG
GTCAGAGTATATGTTGTTTGGAGAAAGAGGGCAATCAGGACTCTTCTGGGACCCAGATGAGTT
CTTCACTAGCCCTTCTGAACCCCTTGCTCCATAATTGGTCTTTTATCCTGGCTCTGAATGACC
CTGCAGGTCATCATGGTTTTCTTTTTTTATTGTTTTTTTTTTTTCTGAGACAGAGTCTCACT
CTGTCACCCAGGCTGGAGTGCAGTGGCGCGATCTCAGCTCACTGCAACCTCTGCCTCCCGGAT
TTAAGCGATTCTTCTGCCTCAGCCTCCCGAGTAGCTGGGACTACAGGTGTGCCACCACGCCTG
GCTGATTTTTGTATTTTTAGTAGAGATGGGGTTTCACCATACTGGCTAGGCTGGTCTCGAATT
CCTGACCTCAGGTGATCCACCCACCTCGGCTTCCCAAAGTGCTAGGATTATAGGCTTGAGCTA
CTGCGCCCGGCCCATGGTGTTTTTCTTTAGGGCTCTTCCTACAGCCTTGAGAAGTAGATAGGC
ATCAGAGTATGGTACTATAGGAATCAGAAAAATTCAAAACAAATGTGGATTAAGTGTTTAGGC
TCTATGTGGCTCACGCAGCCAGAATCCTTAAGTCTGTGTGTTTCTGTGTCTCAAGACTGGGCT
CACATTCTGGCTTTGTCCATAACAATGCTCTGGGATTTCAGGGAGTTCCCTCATTTGTAAAAT
GAGGGGGTCAGAGCAGGTGATATCCATGTTTCTTCCCTTTCTGATATTGTTGTCTGTGGCATA
TTCTTTGTATGGCGAATTTAATAAATTATATTAATGTGTCA

# FIGURE 74

MNGTRNWCTLVDVHPEDQAAAGRKTYAMVSSHSAGHSLASELVESHDGHEEIIKVYLKGRSGD
KMIHEKNINQLKSEVQYIQEARNCLQKLREDISSKLDRNLGDSLHRQEIQVVLEKPNGFSQSP
TALYSSPPEVDTCINEDVESLRKTVQDLLAKLQEAKRQHQSDCVAFEVTLSRYQREAEQSNVA
LQREEDRCPE

**Important features of the protein:**

**Signal peptide:**

amino acids 1-39

**N-glycosylation site.**

amino acids 2-6

**Amidation site.**

amino acids 21-25

# FIGURE 75

GCTTGCACACATGGCTCCGGAGGCTCCGGTTGCCCATCCGAGCCCCTGCCAGGCTCTAACGTTCCCAACTGACAA
CACCAGTAACTAAATATAGGAGCAGATGGTGGGGACGGGCTGTCGCAGCGGCTCCTTTGCAGAGGTCTCCGGACT
GCAGATAAGGCTCAGGCCCTTTTGTGAGAAGCAGACCAGCCTGGGGGCTGGCGGCAGGACACCTGTGTCTGC**ATG**
CTGAAGAAGATGGGTGAGGCCGTGGCCAGAGTAGCAAGGAAGGTCAACGAGACGGTGGAGAGCGGCTCTGACACT
CTGGACCTGGCCGAGTGCAAGCTGGTCTCCTTTCCCATTGGCATCTACAAGGTCCTGCGGAATGTCTCTGGCCAG
ATCCACCTCATCACCCTGGCTAACAACGAGCTTAAGTCCCTCACCAGCAAGTTCATGACCACATTCAGTCAGCTC
CGAGAGCTCCACCTGGAGGGGAACTTCCTACACCGCCTCCCCAGCGAGGTCAGTGCCCTGCAGCACCTCAAGGCC
ATTGACCTGTCCCGGAACCAGTTCCAGGACTTCCCTGAGCAGCTTACCGCCCTGCCGGCGCTGGAGACCATCAAC
CTGGAGGAGAACGAGATCGTAGATGTGCCCGTGGAGAAGCTGGCCGCCATGCCAGCCTTGCGCAGCATCAACCTC
CGCTTCAACCCACTCAACGCCGAGGTGCGCGTGATCGCCCCGCCGCTCATCAAGTTTGACATGCTCATGTCTCCG
GAAGGCGCAAGAGCCCCCCTACCT**TAG**GCCACCCTCCTCATGCCCACCCAGCAAGGGACAGAGGCCACAGGCCTG
GAACCCTGGAAGGGAGGGAGGCCCATGGGAGGCCAAGCCTGGGGGCTGGGGGCGGGTGGGCCGAGCAGCACGTGG
TGGGTGGGGTGCAGCTGGTCTGGATAGATAGCTTACAGCAGTAGTGGGCTCTGGAATGCCCAAGGGAAGAGGCAA
GGTGGGGCCTGCAGCCTGGACTCGGCACTCACAGCTGCTGTGCAAACTCAGGCAGATCTCCTGCCCTCTCTGAGC
CTTGTCACTTGAAAAAAACAGGACCCTTTCCCTCCTTTGGGCTCCCTGGAGGTTTTTAAGCAGTACGTGCCTCCA
AGTTACCTCCAGATCAGCAGGCACAGGTGGGCATTGCCAGGTATTTTCTGAGCCCCTGCGGGTTTGAGGCCTTGT
TTTTAGTGCTGAGAGCCAGTTGCTGCCCTGAGAAGAGAAGACAACCTCCATCTATTTATTGCTTCCTGAGAACTG
ACCTGGATGCGGCCCTCTGCAGGGCCCAGTCTTCAGTCCTGTGGTCCCTGGACTGGTGGGAACCTGAACTAGGAG
TCCTGGGAGAGCTGTGGTGGGAATATGGGCTGGCACTGCTGCAGGGCAAGAACATTCATGTAGGAGCCCGAGGAC
CANCANGCTGGGAATGGGGAGCAAGTCACGTCAGCTCTGTCATTCCCCACAGTTAACAAATTGGCGGGGTGGGAA
GTCCTGAGTGCTCCGTCCCTCTAGCATCACTCCTGAGCTGCGGGAGAGGTGGCCCAGAGAACAGCAGAGTCAGTT
ACACCTGCAGCTCTTGTCTAAAGTGATTAGATGGCCACCCTCACCACTGTCCAGTCCAGCAGCAGCCTGGCTGCC
TTGTCATGGCCTCCTGGGGGCAGAAGGCGATGTGGACCACGGGATTTGTAGCCAGCCAGCTCCCAGGCCAACGCC
CAAAGCCCTGATGACCTGGTTCTTCTGAGGCCCTCAACCTGGCATCTTAGGGTATGGTCAGGCAACAGGGTGACC
AGCTGTCCTGGTTTCCCAGGACATGGAACTTTCAATGCTAAAACTGGGACATTACCCAGCAAGTGGGGATGGTTG
GTCCCCTACCAGGAGAGGGCCTGGGGCTCTTGCTTCCCGAGAACGCCTGTGGCTTGAAGAACCTTGACTGCTTGG
TCCTCAGGTATCTACCTCCCACCTTCTCCTCATCTGTGGAGCAAGCCAACTCAGTGCCCCAGACCCCACCTGATC
TGCATCTTTGTTTGCTCCAGAGACACCTGAGGCCCCAGAGCTTGAGGCAAAGCCAGGCCGTCCAAATCCTGTGTG
CCGTGGACGAGTGGCCACTTTACTACTCCTAAGGCTAAGATGTTGAGAGCTCAGACCACTGCTCAGAGCAGTAAT
CCCTGCTCAGAATGCTCCCAGTTCCCTCGTCCCTGCCCAGGTCTCTTGTCTCTTGGGAAGGAACTGATAGGTCGG
GCCATTGTTGGGCCATCACTGAGCGCTCAGTATCTCAAGAGACTCTGTTCATTCTGCTCGTATCCCAAGGCCTGG
TTGGTCAAACTCTGGGCAAAGGGTTTTCAGGATGAGGAGGTCAAGACAGGATGTCCAGAGCTACCGAGTTCATCT
GTGGGTGTTGGGGGCAAGTGGGGGCTGAAGTCCTGTGCAGGCTGCGCTGGCCCCACCTGCCTTGTGCCCTGGAGT
GGGGTTTCTCCTTGTTGAAGAAGAGGCATCCTTCTCTGATGTGCACAAACACAATGTATGACCAGAGCCTTGCAA
CTCAAAGTGTGGTCTGTGGACCAGCAGCGGCAGTGACACCTGGGAGCTTGTTAGGAATGCAGAGTCTAGGCCTCA
CCCTATACCTCCCGACTCAGACCCTGCATTTTAGCAAGACCCCCAGCTGATTCCTATAAGCACTTTAGAGTTTGA
GAAGCAAGGACCTAGGCTGGGGATGTCCTCCGAGCAGAGGGTGAAGTTTCTCTCAGTTCTCTCCCTGCCACTTCC
AGGGATCTGAGCCTGTGTTCAGCCTCCTCCCTAACCCACCCTGGGAGACACTTGGCCTGTTAGATTGTTCCAGAG
TCTGCATGGCACTCCTGAAGAAGGGAGTGTGACCTGCAGTCACCAGGAGATGAGGGTTAGGTGTGCCCAGCCCTC
CAGACCCGGCCTTTCTGGTTAACCCCTGCATGCCAAGCTGCCTGCTGCCCCAGGTCCTCACCTCAGGCCTTTGAA
GGGGCAGCTTCTGGAAGTTGTTTTCTCCTCTGCTTGGAGAGTTTGCCCTTGTCTGTCTTGGAAAGTGTGGGCAGC
CACAGATGCCCCCAAATCAGAGCTCACAGTGAGTGAGCCCCTAAGCTTCAGTCTGCAATAAAGAATGCATTGGTT
TCAAAAAAAAAAAAAAAAAAAAAAAAAAAAAAAAAAAAAA

# FIGURE 76

MLKKMGEAVARVARKVNETVESGSDTLDLAECKLVSFPIGIYKVLRNVSGQIHLITLANNELK
SLTSKFMTTFSQLRELHLEGNFLHRLPSEVSALQHLKAIDLSRNQFQDFPEQLTALPALETIN
LEENEIVDVPVEKLAAMPALRSINLRFNPLNAEVRVIAPPLIKFDMLMSPEGARAPLP

**Important features of the protein:**

**N-glycosylation sites.**

amino acids 17-21, 47-51

# FIGURE 77

CACCAACAAGCAATCGTTCATGAGAAAGCCGTGCACCCGCTGCAGTTGGGCCATGTGGTCCGCATCGTATTCCAC
TAGGTCCCCATTGTACACCAAGTACTGTCCCGGCGTCTCCAGCAGATGCCTGCAGCCTTCCACCTTCTCAAGCAG
GGTGGTGTGAGTGCGCTGCTTTCCTTCCTCGCCTGGACCGGAGCCGTCGCGGGAGGCACCCCCGGGGGTGGAGAA
AAAGCCGGCCTGGCCTCGGAGGTGGTCTCGGCCCCCCGCCCCACCGACTCCCTCCTCCCCTCCAGAGGCGGCGGC
GGCTCCGGCGGCAGCAGCGGCAGGCAGCAACGTAAGCGGGATGCTCTCCAGGCTGCTTTTCTGCTCGGTCAGCAA
ATGGCTGAGCTGGTACATCTCGCTCTCCAGGTAGGAGATCTCGCGGGCCGTCTCTATGAACTGCCGGTAGTTCTG
GTAGACGTTGCGCTTCAGGTTCTGCGCCGTCTCCTCCGCCAGCGCCTGGATGCGCTGCCGGTGCTCCTGGAGGTC
CCGGTCCCCATCCGACTGCTGCGAGAGCTGCTTCACGTACAGCCGCGCCTCAAAACCCCCTGACTCCAGCTGCCG
ACGCAGGCGGCTCGCCCCACTGTCCGACATCGCCATCGCCATTTCTCTCCGGGTCTCACGCACTCACTGTCACTA
TCGGCGCCGCAGCCGCCGCGGCTGTCTAGACCCACCCAAGGCCAACCGAGCTCCTGGGCTGAGGAAGCAGGAATG
GGAACGAGACGAGTACGCCTGCGCCGGGTCTGAGCGTCAGACACTGCGCCTGCGCAAGTGGGCCGAGCGCAGACA
TTGCGCCTGCGCAGCAATGCCATCGGTTAAAGCGCATGCGCAAGATGAGCTATTGCGGAAGTGAGGGGAGGGAGA
GGCCGAGAGAAATTTCGGTACTGCGCATGAACCGAGCGTGACGTTGAGGTTTGAAATAACCGGCAAAGAGTAAAG
GCTGAAACTAGCTTCCTGAAAGCTTCGTAGGGCCCGAGCCCTGTGAGCCCAGGTTCTGCGCCCACTAGGAGGTGT
CATGCTGACTGCTTTTTTTAAAGCCCTAGAATCCTTGGCTTCGGCGTTTGGGGTAAGCTCCGTTCTCGTTCTCAA
GCGCGTTTCCGCGAACTCTCGCGGGATTGACGGGCCGTCTCGAGAGCCGGCATCTCCTAGGAGCTAGTCCTGGTC
CTCGGCTAGGCGGCTTGGGGTCGCGGCGTAACTGGGGAGCCAGCCTGACGCCGGCGGACCCCGCCTGTGATCCTG
GCAACG**ATG**GATGATGACTTGATGTTGGCACTGCGGCTTCAGGAGGAGTGGAACTTGCAGGAGGCGGAGCGCGAT
CATGCCCAGGAGTCCCTGTCGCTAGTGGACGCGTCGTGGGAGTTGGTGGACCCCACACCGGACTTGCAGGCACTG
TTTGTTCAGTTTAACGACCAATTCTTCTGGGGCCAGCTGGAGGCCGTCGAGGTGAAGTGGAGCGTGCGAATGACC
CTGTGTGCTGGGATATGCAGCTATGAAGGGAAGGGTGGAATGTGTTCCATCCGTCTCAGCGAACCCCTTTTGAAG
TTGAGGCCAAGAAAGGATCTTGTAGAGACCCTCCTGCATGAAATGATACATGCCTATTTATTTGTCACTAATAAC
GACAAAGACCGAGAAGGGCATGGTCCAGAATTTTGTAAACATATGCATCGCATCAACAGCCTGACTGGAGCCAAT
ATAACGGTATACCATACTTTTCACGATGAGGTGGATGAGTATCGGCGACACTGGTGGCGCTGCAATGGGCCGTGC
CAGCACAGGCCACCGTATTACGGCTATGTCAAACGAGCTACTAACAGGGAACCCTCTGCTCATGACTATTGGTGG
GCTGAGCACCAGAAAACCTGTGGAGGCACTTACATAAAAATCAAGGAACCAGAGAATTACTCAAAAAAAGGCAAA
GGAAAGGCAAAACTAGGAAAGGAACCAGTATTGGCCGCAGAGAATAAAGGTACCTTCGTGTATATTCTTCTGATT
TTTATG**TGA**CCATAGCTATGATGTAAAGACAATACTGTCCTTCAGAGAACTGGTATTAAGATAAACTTAAGGATC
GTTTCTGGTGTAGAAGTCTTCAAGTGTAGACTTAAGGAAAAAATCCCACTGTCCATGAAATGATGGTAGGAAAAC
AGACTTTGCTCTGTACAGAAGTAAGTAAAAGTAGGAATAGTTTCCATGGATATTTTTATTTTTATTAACTTTTTT
CAGTTTCTTTTTATTCAAAGAAACAAAATTCAATCTCTGATAATATTTGAGGTAAAGTTCCTTTCCCTATCTTGA
CTCACTGAGTTATTAGGAAACAGAAGGCAAAAAGATTGTCAAAATAAAAACAATAATTCAAGTAACAATGCCCGG
AATATACGTCCTAACTACACCCCTTCCTATCAGCTGGATTCTATCCAAGTGACTCTATTGATGTATGTATGTTCA
TTCAAAGAATGGGAAAAGGATATGACATATATTTGCCAGTACTTCATCTTCAAGATTTACCCTTTTCCTGTGAAG
TTCAGAGTTACTGAAGATGCTTCTTCCCTTGGGAAGTTGTTGACCCAAGAACATAGGTTATATTTCCCAAATCTT
TAATTATTGAGTGAAAGAGCTATAGATGAATTGATATGGAAAGACCGTATCTTCATTTTCGTGAGTAGAAGGAAA
GATAAGAATGAGGCAGCAGATTTTCCCTCCTGGAATTACACATAAAGGACACTAAGCAATTTTCAAGGTAAATGT
TGCCTTGTTGTTGGTCTTTGGCATGATAAGATTCTTTATTTAAATATGAGAGAATTTTTTTTTATCCTTTATATT
CTCTCAATATCAGAACTCCTGAATTCTGAAGATTGCCCTCCTCCCATTAATAGGATTGTATGGATGTAAGATGGA
ATAAAATACTAGTTCTTCATTTTGAGAAAACTGTACATTAGTTTAATGTTTGTTACTGTATTTCTTTTGAGTTGA
GGCACTTACATAACAATCTTCTTTGCTTTTTTGGCAGATAAACCCAACAGAGGTGAGGCCCAGCTAGTAATCCCT
TTTAGTGGGAAAGGATATGTTCTAGGAGAAACAAGCAATTTACCTTCACCTGGGAAACTGATCACTTCACATGCC
ATTAATAAAACCCAAGATCTTTTAAATCAAAACCATTCAGCAAATGCTGTAAGACCTAATTCTAAAATCAAGGTG
AAATTTGAACAGAATGGTTCAAGTAAAAATTCTCATCTGGTCTCCCCTGCTGTTAGTAACAGTCACCAAAATGTT
CTAAGCAACTACTTTCCTAGAGTATCATTTGCCAACCAAAAGGCTTTCAGAGGTGTGAATGGATCTCCAAGGATA
AGTGTAACAGTTGGCAACATCCCTAAAAACTCAGTCTCTTCTAGTTCTCAGAGAAGGGTTTCATCTTCTAAGATA
TCCCTAAGAAATTCTTCAAAAGTAACGGAATCAGCATCTGTGATGCCATCCCAGGATGTGAGTGGGTCTGAAGAT
ACATTCCCAAATAAACGACCTAGGCTAGAAGATAAAAAAAAA

# FIGURE 78

MDDDLMLALRLQEEWNLQEAERDHAQESLSLVDASWELVDPTPDLQALFVQFNDQFFWGQLEA
VEVKWSVRMTLCAGICSYEGKGGMCSIRLSEPLLKLRPRKDLVETLLHEMIHAYLFVTNNDKD
REGHGPEFCKHMHRINSLTGANITVYHTFHDEVDEYRRHWWRCNGPCQHRPPYYGYVKRATNR
EPSAHDYWWAEHQKTCGGTYIKIKEPENYSKKGKGKAKLGKEPVLAAENKGTFVYILLIFM

**Important features of the protein:**

**Signal peptide:**

amino acids 1-41

**N-glycosylation sites.**

amino acids 148-151, 217-220

**cAMP- and cGMP-dependent protein kinase phosphorylation site.**

amino acids 184-187

**Casein kinase II phosphorylation sites.**

amino acids 30-33, 121-124, 154-157, 187-190, 192-195

**Tyrosine kinase phosphorylation site.**

amino acids 211-218

**N-myristoylation sites.**

amino acids 59-64, 85-90, 146-151

**Neutral zinc metallopeptidases, zinc-binding region signature.**

amino acids 108-117

# FIGURE 79

CGGACGCGTGGGTGGCAACCAGGAGAAGCCAAACTTGGTCCCCCGGCTCGCGGAGTGCCTGCG
AGCGGTGCTC**ATG**GCGCTCTATGAGGTCTTCTCTCACCCGGTCGAGCGCAGTTACCGCGCGGG
GCTCTGCTCCAAAGCCGCGCTGTTCCTGCTGCTGGCCGCTGCGCTCACGTACATCCCGCCGCT
GCTGGTGGCCTTCCGGAGCCACGGGTTTTGGCTGAAGCGGAGCAGCTACGAGGAGCAGCCGAC
CGTGCGCTTCCAACACCAGGTGCTGCTCGTGGCCCTGCTCGGACCCGAAAGCGACGGGTTCCT
CGCCTGGAGCACGTTCCCCGCCTTCAACCGGCTGCAAGGGGATCGCCTGCGCGTCCCGCTCGT
TTCGACTAGAGAAGAAGACAGGAACCAGGATGGGAAGACGGACATGTTACATTTTAAGCTGGA
GCTTCCCCTGCAGTCCACGGAGCACGTTCTCGGTGTGCAGCTCATCCTGACTTTCTCCTATCG
ATTACACAGGATGGCGACCCTCGTGATGCAGAGCATGGCGTTTCTCCAGTCCTCCTTTCCTGT
CCCGGGATCCCAGTTATACGTGAACGGAGACCTGAGGCTGCAGCAGAAGCAGCCGCTGAGCTG
TGGTGGCCTAGATGCCCGATACAACATATCCGTGATCAACGGGACCAGCCCCTTTGCCTATGA
CTACGACCTCACCCATATTGTTGCTGCCTACCAGGAGAGGAACGTTACCACCGTCCTGAATGA
TCCCAACCCCATCTGGCTGGTGGGCAGGGCCGCAGATGCTCCATTTGTGATTAATGCTATCAT
CCGATACCCTGTGGAAGTCATTTCTTATCAGCCAGGATTCTGGGAGATGGTAAAGTTCGCCTG
GGTACAGTATGTCAGCATCCTGCTTATCTTCCTCTGGGTGTTTGAAAGAATCAAGATCTTCGT
GTTTCAGAATCAGGTGGTGACCACCATTCCTGTGACAGTGACGCCCCGGGGAGACTTGTGTAA
GGAGCACTTATCC**TAG**AAAGGCCATTTCTGAAGACTCAGCAGGACCGTGGCTGCCTCATTGTC
ATCTTCTGGGAACATCTTAGGACCTTTTGAAAGAGCCCAGCGGACACCTGCGGGCTTGTGTGC
TTTTCCCTCAGAGACAACGGTTCTTTCCGGTTTTGCTCTACACAGTTCCGTATCTTCAGAGCT
CCTGCAGAATTGTCAGGGACTAGTTTGTGGAAAGGTCTGAGAGTTCCTGGAGGCTATAATTAG
CTTTTTGGGTTTTCCTTCTTTGCCTTAGCGTTGAATTTCAGGAGAAAATTGCAGTCAGTTCAG
ACATCTTGGAAAGAGTCCCATCTCTGGTCAAGCAGAGACTTTTCCTCTGTTGAACTGAGGAAC
ACACTGTGCATTTCTTCCTTCTGTTGTGAGCCACTCTTACTCTTTTCAGGGCTCTCTTGTGAC
AAACATGCCAATCACTAGCACTTTGCACCCCTGGGCTTCTCCATTTCCCATTCACAGCTTTGA
TTTCCAGAGCTGAGGCCTTTAACTGGAGACCTGGAGGGGCAGGGCCCAAGGGCAAGGGCCGCA
TTAGCACAGGCAATCAGGGAGGGCCGCTGAAGGACACTTGGACCGTCCACCTGCCCCAGCCCA
ACAGTCAGTCATCTGTCATCAGCTCAGCTGAGCAGCCCTGGATCTTTGCCGTACTGTGACTGG
GCTCTTTGCCCTATTTTTCCCTCTGTCTGTGCCCCTGGATGGCAGGCTGAAGTCAGAGGGGCT
GTTTCATTCTCAGCCCCCTCAGCAGCACTGGGGGAAGAAAGCATTGTCACAACAGGTTCTTTC
TGGCCCTCACCCAACAGCCTGGGCACTTGGCCCTCCTCCTCCTTGACAGCCCTCCCCCTTCCT
GCAAAGGACAGGGGCGACAGGGGTTGGTGTTGGGATTGGCTCCCGCTGCCTGACAACCACAAG
TTTATTTGGAAGGCTAGCGGGAAGCCCAGCGGCTGGCGTTTCCCTTGACTAAGGAACAGGGTG
CCCATCAGAGTGGGGCGGGCAGCTTTGGGAAGGACACAAGAAGCAGTAAGAGTGTAAAGAGGA
TGCTGGCCTGGGCAGGCCAGTCCAGCCTGGCCACTAGCAGAATACCAAGCAGTCCAGTGGATT
ACCCTCGTGGCTAAGCAAGTGTCTGCAGGAGCAGAGATGGCTGGAAGGGGCCTCTGCACACGG
AAGATGGCTTGTTCAGCCCATTCACCTCCTGAGGATGTGGGCAGTCTCCTCCAAGAACACATG
GAGCTGCTTCCTGATCCCAAGCAGGTCATTGCCACTGGAAGGACATGGCCCCGGTGATCCATG
CTTCATGCCCACCCAGAAACACACCCCTCAGTGTGTGCCTCAGTTTACTTTGGAGATCAGTTG
TCGTTTTTAGTGCTCCTTTAGGCTTACTAAAACAGTTTTGGAAACAAAGCTATTTTGAAGTAT
TCAAGCAGAGGAATTCCCTAACACTGACCCCCTTGTCTTTTTTTAATATTCAGGCTGTTTTAT
ATGCCTAAATTTTTTTCTTAAGATCTAAACGAAAAATAGTTTCTTGTTTAAATTCACATAAGG
CAATGAGATATGGAAAGATGACAAGATACGTATAAACATTGGTTTGCATCTTATTAAATTATT
CTAATGCAAATCTTGTATAAAGAACCCATGATGTTTTGTAACTTTCTAATTAAAATGTTCAAA
ATGAG

# FIGURE 80

MALYEVFSHPVERSYRAGLCSKAALFLLLAAALTYIPPLLVAFRSHGFWLKRSSYEEQPTVRF
QHQVLLVALLGPESDGFLAWSTFPAFNRLQGDRLRVPLVSTREEDRNQDGKTDMLHFKLELPL
QSTEHVLGVQLILTFSYRLHRMATLVMQSMAFLQSSFPVPGSQLYVNGDLRLQQKQPLSCGGL
DARYNISVINGTSPFAYDYDLTHIVAAYQERNVTTVLNDPNPIWLVGRAADAPFVINAIIRYP
VEVISYQPGFWEMVKFAWVQYVSILLIFLWVFERIKIFVFQNQVVTTIPVTVTPRGDLCKEHLS

**Important features of the protein:**

**Signal peptide:**

amino acids 1-34

**Transmembrane domain:**

amino acids 268-284

**N-glycosylation sites.**

amino acids 194-198, 199-203, 221-225

**cAMP- and cGMP-dependent protein kinase phosphorylation site.**

amino acids 51-55

**Tyrosine kinase phosphorylation site.**

amino acids 250-259

**N-myristoylation site.**

amino acids 187-193

**Cell attachment sequence.**

amino acids 307-310

# FIGURE 81

GCCGGGAGCTTCCCTG**ATG**GTGCCGCCGCCTCCGAGCCGGGGAGGAGCTGCCAGGGGCCAGCTGGGCAGGAGCCT
GGGTCCGCTGCTGCTGCTCCTGGCGTTGGGACACACGTGGACCTACAGAGAGGAGCCGGAGGACGGCGACAGAGA
AATCTGCTCAGAGAGCAAAATCGCGACGACTAAATACCCGTGTCTGAAGTCTTCAGGCGAGCTCACCACATGCTA
CAGGAAAAAGTGCTGCAAAGGATATAAATTTGTTCTTGGACAATGCATCCCAGAAGATTACGACGTTTGTGCCGA
GGCTCCCTGTGAACAGCAGTGCACGGACAACTTTGGCCGAGTGCTGTGTACTTGTTATCCGGGATACCGATATGA
CCGGGAGAGACACCGGAAGCGGGAGAAGCCATACTGTCTGGATATTGATGAGTGTGCCAGCAGCAATGGGACGCT
GTGTGCCCACATCTGCATCAATACCTTGGGCAGCTACCGCTGCGAGTGCCGGGAAGGCTACATCCGGGAAGATGA
TGGGAAGACATGTACCAGGGGAGACAAATATCCCAATGACACTGGCCATGAGAAGTCTGAGAACATGGTGAAAGC
CGGAACTTGCTGTGCCACATGCAAGGAGTTCTACCAGATGAAGCAGACCGTGCTGCAGCTGAAGCAAAAGATTGC
TCTGCTCCCCAACAATGCAGCTGACCTGGGCAAGTATATCACTGGTGACAAGGTGCTGGCCTCAAACACCTACCT
TCCAGGACCTCCTGGCCTGCCTGGGGGCCAGGGCCCTCCCGGCTCACCAGGACCAAAGGGAAGCCCAGGCTTCCC
CGGTATGCCAGGCCCTCCTGGGCAGCCCGGCCCACGGGGCTCAATGGGACCCATGGGACCATCTCCTGATCTGTC
CCACATTAAGCAAGGCCGGAGGGGCCCTGTGGGTCCACCAGGGGCACCAGGAAGAGATGGTTCTAAGGGGGAGAG
AGGAGCGCCTGGGCCCAGAGGGTCTCCAGGACCCCCTGGTTCTTTCGACTTCCTGCTACTTATGCTGGCTGACAT
CCGCAATGACATCACTGAGCTGCAGGAAAAGGTGTTCGGGCACCGGACTCACTCTTCAGCAGAGGAGTTCCCTTT
ACCTCAGGAATTTCCCAGCTACCCAGAAGCCATGGACCTGGGCTCTGGAGATGACCATCCAAGAAGAACTGAGAC
AAGAGACTTGAGAGCCCCCAGAGACTTCTACCCA**TAG**CACATCCCAACACCGTCACGCCAAAGGAAGAGAAAGAT
CAACTCACCTGCAGTTAAACCATCTAAAGAGAAGAAAGACCACTGGAGACCTAGAAAACATACATTTTTCTCTTC
TCTTCTCCTGACGTCTCTCCACTCCTCTTCTTCCAAATACGATGCTATTTTCAGAGTCCCCTCCTAGGCCTGCAG
ACATGAGGGAGTGAATGATTGATTTACCTGCTTCTCACTAAGAGTCCATTGGGGTGGTTTGCATTGTAACTTTTC
TTTTACATCCTATTTTTCCAGGAACTTTGGATTTAAGTACTCTCACAGTGTCTTAAATCATAAATTCTTGAAGTT
AAATTTGGCAGAGTATCAAAAGGGGGAAAATGACAAAGTGAGCTCTAAGAAAATGTGAGGCTACTTCTAAGATGT
GTGTTCACAATAGACCATAACTCCTCTAGTATCAAAATTGGGGCTCTTCAGTTAAAAAGGGGTGGGGAGGACAAA
CGTGTCGATGTGCTTTGGTGGAGAATTTTTTCCTTGTGCTTCTAGTAGACTTTAAATATTGTATCCCTTTGTCAA
ACCTTGTTTCCCAAATTCAATTAAAGAGAGGAGAGAATTGAATGGCGTTTAGAGAAGATAGAAAAGAATCACAGT
CATATATTTACTGTTATATAGATTGCCACATTCTAAAATTCAAATACGGTGCTTAAGGTTTCATGCCATGCTTAT
CTGTAAGTATCCTATTTAGGGAAGAAGATTAAACTCTCTTTTCAAAAAAACAAAGTGAAATGCCTGGATTCACAT
TAAAACAATGGGCTCTCGTTTGCTATAATATTTTAAAGCTGTTTAATCAACAGTGGAGTCTGCTCTATAAATATA
GATTATTTGTTCAATAAACTGGCTGAGCTTAGAGAGAGGTGCAGAATTCCTGGTTCTGAGCAGGTGCCCAGAAGG
TACCATTAGGTGCCATGATCCAGGCTGAACCAATATACAGTGGGGCTGAAGTCTGCAAGGAGGTTGCTGGCTTGG
GCTGACCTCACTAATGCCATCAGCAGCGGTAGGTAAATTTTTTCTCCTTGGGTATTACAAGTTTTTGTCTGGAGC
CAACCAAGCTTGCCACCAACATATTGAGAGTAATACACTATTGAAAGTTATCTTGGATGGGGAGAAAAAAAAATA
GTGGTTTTCCTTGTTTGCAAAAACTTCCTTCCTATTCTCATTTTTTCTTAATTTTCTTTAATTTAGTCCAAGTTC
CAGTTCTTTTAGGCCTTCTCTTTGATTTATTTTCCCCTGCATGTGAGAAGCAGTTCAGAAAAAGGTCTATATCTC
CACCTCCTAGTGAGTTAGAGTGTTTTCTCAGAGCACCTCTGGGTGGCAAAGGGAAGCATGTTCCTGCCAAGGTTT
GCTGTGGATTCAGAAGCACCAGGAGCAAGAGACCAGAAGGATGATCTGCTCCTTTGTAACGTTGTTGAGGGCCCT
CTTGTTTCCAATGAGCAGCTTATAGGTTACTCACAGTCCACTTTCTCACTGGACACACAAAGTGGCTCTTTATCT
ACCTTTGCGGGAGATTTTCACTCTCCTGCAAATGATCGTTCTCACACTCATATTAGCTCATGTTGGAATTTCCCA
TCCTGCCATGTCCTTTCCCATTTCTTTTTGGCTTTTTTGCCTCCACCTTTTAGCCCACATCATTTAACTCCACTA
CTGTGAAAGCTTGCTTAAAGAAAATCCCTCTTGGCCGGGTGTGGTAGCCCACGCCTCTAATCCCAGCACTTTGGG
AGGCTGAGGCGGGGAGATCACAAGGTCAGGAGATCGAGACCAGCCTGACCAACATGGTGAAACCCTGTCTCTACT
AAAAATACAAAAATTAGCTGGGCGTGTTGGCACACACCTGTAATCCCAGCTACTCAGGAGGCTGAGGCAGGAGAA
TTACTTTAACCTGCGGGGGGAGCCTAGATTGCGCTACTGCACTCCAGCCTAGGCAACAGAGGGAGACTCTGTCTC
ATTAAAAA

# FIGURE 82

MVPPPPSRGGAARGQLGRSLGPLLLLLALGHTWTYREEPEDGDREICSESKIATTKYPCLKSS
GELTTCYRKKCCKGYKFVLGQCIPEDYDVCAEAPCEQQCTDNFGRVLCTCYPGYRYDRERHRK
REKPYCLDIDECASSNGTLCAHICINTLGSYRCECREGYIREDDGKTCTRGDKYPNDTGHEKS
ENMVKAGTCCATCKEFYQMKQTVLQLKQKIALLPNNAADLGKYITGDKVLASNTYLPGPPGLP
GGQGPPGSPGPKGSPGFPGMPGPPGQPGPRGSMGPMGPSPDLSHIKQGRRGPVGPPGAPGRDG
SKGERGAPGPRGSPGPPGSFDFLLLMLADIRNDITELQEKVFGHRTHSSAEEFPLPQEFPSYP
EAMDLGSGDDHPRRTETRDLRAPRDFYP

**Important features of the protein:**

**Signal peptide:**

amino acids 1-34

**N-glycosylation sites.**

amino acids 142-148, 182-188

**Tyrosine kinase phosphorylation site.**

amino acids 125-132

**N-myristoylation sites.**

amino acids 10-16, 143-149, 155-161, 196-202, 250-256

**Amidation site.**

amino acids 299-303

**Aspartic acid and asparagine hydroxylation site.**

amino acids 150-162

**Cell attachment sequence.**

amino acids 176-179

**C1q domain proteins.**

amino acids 247-280

**Calcium-binding EGF-like domain proteins pattern proteins.**

amino acids 144-165

# FIGURE 83

ATCTGAGTGAGCTAACTGACACA**ATG**AAACTGTCAGGCATGTTTCTGCTCCTCTCTCTGGCTC
TTTTCTGCTTTTTAACAGGTGTCTTCAGTCAGGGAGGACAGGTTGACTGTGGTGAGTTCCAGG
ACCCCAAGGTCTACTGCACTCGGGAATCTAACCCACACTGTGGCTCTGATGGCCAGACATATG
GCAATAAATGTGCCTTCTGTAAGGCCATAGTGAAAAGTGGTGGAAAGATTAGCCTAAAGCATC
CTGGAAAATGC**TGA**GTTAAAGCCAATGTTTCTTGGTGACTTGCCAGCTTTTGCAGCCTTCTTT
TCTCACTTCTGCTTATACTTTTGCTGGTGGATTCCTTTAATTCATAAAGACATACCTACTCTG
CCTGGGTCTTGAGGAGTTCAATGTATGTCTATTTCTCTTGATTCACTTGTCAATAAAGTACATTC
TGCAAAAGCAAAAA

# FIGURE 84

MKLSGMFLLLSLALFCFLTGVFSQGGQVDCGEFQDPKVYCTRESNPHCGSDGQTYGNKCAFCK
AIVKSGGKISLKHPGKC

**Important features of the protein:**

**Signal peptide:**

amino acids 1-23

**N-myristoylation sites.**

amino acids 26-32, 52-58, 56-62, 69-75

**Kazal serine protease inhibitors family signature.**

amino acids 40-63

# FIGURE 85

GGAGCAGACACACAGACCCGGGCCGGAGGCCCCTCTTCTAGCCCTGCGGGAACCGGACAGTTC
CCCAACTGGGGACTCTGGAACCACAGCTCCTAAATCATCAAATTCTCAAGCTTTTTTTTTCCC
TCTCTTCGTCCCAGCCATCCCAGTCTTCTTCTTCTTTTTTTTTTTTTTAACTTATTGTTTTTT
TCGCTCCTGTCATTATGAAAGTGGTCACGCCATTCAATATTAAGACTTGGAGGGAATTGGGGA
AAGAAAAGAAAGAATCTAAAAGAAGAGAAGCGACCGGTGCTTTTAAGGGTGTCTAATTTTCAA
AAGAGACGTCTGGGAGTATTTTGCTCTGGGCGTTTGGAGCAACTTCGCGGACAGCGGAGCTCG
CCCAGCATGGATGTTCCAGGTTCACAGGCGCCTTTCTTCTGAGAACGACCCTGGCCTTGAACG
TCAGAGCCGGGGACGAAGGCCCCCGGAGGCTGCTGCGAGCTCCGCGCGTTCCTTCGCGCCCTT
CCGCGCCGCTCGCGCCGGCGCCGGCCTCCACCCCCGCGCGCCGCCTCCCACCAGTCCCG**ATG**C
AGGCGCCCGGCCGGGGGCCACTCGGGCTGCGGCTGATGATGCCCGGGCGCCGGGGGGCGCTGC
GCGAGCCTGGCGGCTGCGGATCCTGCCTGGGGGTGGCGCTGGCCCTGCTGTTGCTGCTACTGC
CCGCCTGCTGCCCCGTGCGGGCGCAGAACGACACGGAGCCCATCGTGCTGGAGGGCAAGTGCC
TGGTGGTGTGCGACTCCAGCCCGTCGGCGGACGGCGCCGTCACCTCCTCCCTAGGCATCTCCG
TGCGCTCCGGCAGCGCCAAGGTGGCCTTCTCCGCCACGCGGAGCACCAACCACGAGCCGTCCG
AGATGAGCAACCGCACCATGACCATCTATTTCGACCAGGTATTAGTAAATATTGGCAACCACT
TTGATCTTGCTTCCAGTATATTTGTAGCACCGAGAAAAGGGATTTATAGCTTCAGCTTCCACG
TGGTCAAAGTGTATAACAGACAAACCATCCAGGTCAGTTTAATGCAGAATGGCTACCCAGTGA
TCTCGGCCTTTGCAGGAGACCAGGATGTCACCAGAGAAGCTGCTAGCAATGGCGTGCTGCTGC
TCATGGAAAGGGAAGACAAAGTGCATCTCAAACTTGAGAGAGGCAACCTCATGGGGGGCTGGA
AATACTCCACATTCTCGGGCTTCTTGGTGTTTCCTCTA**TAA**ACACAGAGCCCCCTAGATGGTG
GGGGAATGGCAAACTGGACCCAGGACTCCGCCCTTTAAAACACCCTGAACTTACTGGAATTGG
ACACCTTGTTTCCAACCTCCGTCAGACTGTTGCAGTAGAAGAATGATTTCCTTTGAAACCTCC
AGTACTTTTGTTTTTGTTTTTTGGAATACTGACAATTCCTCGGGAACCTGGCCTCTAATTAGT
TTTAGATGACAAGGTCTTAAGGAGAAATGAAATTATCGATTTGAGCAATTTGTACCTGTGATT
GTAAAGTCAATATCGGATTTTATTGTTGGGACCATGGACCTCTTTTGTTTGTATGTTGTATTG
TCGTCCCAACGGAAGGAGAGCTCCTGACTCCAGGATGGGCTGCAGGTTGCAGTCAGGGCTTGA
AGTAGGAGCCCAGCAAAGAACCACCTGCTGGACAGTCCTTGACATGTGTTCTGTGTGTGTCTG
TATAGCCTTAAGAAAAAGAATGGCTTCACTTTCATTCTGTATTCTTCCCCCCACCATGTGGCT
GGGAGGACTTGGGAGGGGGATGGGGACATTGGGAACCTGTCAAGAAGTGCTTTATCCAGAGAA
GCAAATTTTGCACGATTGGACTGCAATTTTTGTTTTGTATTGTTTGTGTTTTTTCTTGAAAAG
CTTTACTTTTCTTTCCACACTCAGCTCTCCCTCCTCAACCCCACTTTTATTTTTCTTGCTGGG
GTTGAGGAGAGAAAATATAGAATTCCTGGATAAGACCAAACAAAACAAAACATTAAAATACCT
GTATGTTTTGTTTTAGACGAGACCAAACTAAACAAAAAGTATCTGTTTATCAAAGTAAAAGTA
ACACAATGGACAATTCTGCTTATTCTCTCAAAGAGATTCTAAGATGCACCTTTAGAACTATTA
ATAGCAACCTGCATTTTTTTTTAATTTATACTTCAGAATCCTTTAAGAACCTGGTGTTCCTGA
GTGGTCCTGAATCATATAAGTTGGTAATGGAAGCTGTAATGACCAAGTCCCCTAAACATACTA
TGTCTTTGCCACGTGTGCTGTGACTTCTCTGTGGGTGATTTAATTTATTTGGATCCACCTCTG
AGTGAGCGCACAGTGATCAGGTGCTTCAAAGCCAACAGACCAGCTCCTCTTCCTCCGGATCCT
CTTTTGATCTGCCCAGGAAAGGGATGCATTGACACTCTCCTGCATGCACCTGGCGAGAAGCCA
CCTGAAAGTCACTGTGGTTAAAGATATTGGTGGAGGTACCCCAGGAGCACTGTTACAAATCCT
TCTTGTTTTGGCATCTCGTACAACATTATTAAGACACAGCTGAGAGTTGATGGGTGTGTAATG
CATATGCCAAGGAAATGTCACTAATCCCAAAGCAATCAAAAAGGAGACCTCAAACCAGATGTT
AATTTGTTCTTTGTGTAACAATGTAACCAAAATATTGATGATAAAAGTCATAATTTAAGATTC
AGAATAAATGGGTTTGATGTCTGGCAAAAAAAAAAAAAAAA

# FIGURE 86

MQAPGRGPLGLRLMMPGRRGALREPGGCGSCLGVALALLLLLLPACCPVRAQNDTEPIVLEGK
CLVVCDSSPSADGAVTSSLGISVRSGSAKVAFSATRSTNHEPSEMSNRTMTIYFDQVLVNIGN
HFDLASSIFVAPRKGIYSFSFHVVKVYNRQTIQVSLMQNGYPVISAFAGDQDVTREAASNGVL
LLMEREDKVHLKLERGNLMGGWKYSTFSGFLVFPL

**Important features of the protein:**

**Signal peptide:**

amino acids 1-48

**N-glycosylation sites.**

amino acids 53-57, 110-114

**N-myristoylation sites.**

amino acids 26-32, 27-33, 29-35, 33-39, 76-82, 205-211

**Amidation site.**

amino acids 16-20

**C1q domain signature.**

amino acids 117-148

**C1q domain proteins.**

amino acids 115-149

# FIGURE 87

AGGGCCCGCGGGTGGAGAGAGCGACGCCCGAGGGG**ATG**GCGGCAGCGTCCCGGAGCGCCTCTG
GCTGGGCGCTACTGCTGCTGGTGGCACTTTGGCAGCAGCGCGCGGCCGGCTCCGGCGTCTTCC
AGCTGCAGCTGCAGGAGTTCATCAACGAGCGCGGCGTACTGGCCAGTGGGCGGCCTTGCGAGC
CCGGCTGCCGGACTTTCTTCCGCGTCTGCCTTAAGCACTTCCAGGCGGTCGTCTCGCCCGGAC
CCTGCACCTTCGGGACCGTCTCCACGCCGGTATTGGGCACCAACTCCTTCGCTGTCCGGGACG
ACAGTAGCGGCGGGGGGCGCAACCCTCTCCAACTGCCCTTCAATTTCACCTGGCCGGGTACCT
TCTCGCTCATCATCGAAGCTTGGCACGCGCCAGGAGACGACCTGCGGCCAGAGGCCTTGCCAC
CAGATGCACTCATCAGCAAGATCGCCATCCAGGGCTCCCTAGCTGTGGGTCAGAACTGGTTAT
TGGATGAGCAAACCAGCACCCTCACAAGGCTGCGCTACTCTTACCGGGTCATCTGCAGTGACA
ACTACTATGGAGACAACTGCTCCCGCCTGTGCAAGAAGCGCAATGACCACTTCGGCCACTATG
TGTGCCAGCCAGATGGCAACTTGTCCTGCCTGCCCGGTTGGACTGGGGAATATTGCCAACAGC
CTATCTGTCTTTCGGGCTGTCATGAACAGAATGGCTACTGCAGCAAGCCAGCAGAGTGCCTCT
GCCGCCCAGGCTGGCAGGGCCGGCTGTGTAACGAATGCATCCCCCACAATGGCTGTCGCCACG
GCACCTGCAGCACTCCCTGGCAATGTACTTGTGATGAGGGCTGGGGAGGCCTGTTTTGTGACC
AAGATCTCAACTACTGCACCCACCACTCCCCATGCAAGAATGGGGCAACGTGCTCCAACAGTG
GGCAGCGAAGCTACACCTGCACCTGTCGCCCAGGCTACACTGGTGTGGACTGTGAGCTGGAGC
TCAGCGAGTGTGACAGCAACCCCTGTCGCAATGGAGGCAGCTGTAAGGACCAGGAGGATGGCT
ACCACTGCCTGTGTCCTCCGGGCTACTATGGCCTGCACTGTGAACACAGCACCTTGAGCTGCG
CCGACTCCCCCTGCTTCAATGGGGGCTCCTGCCGGGAGCGCAACCAGGGGGCCAACTATGCTT
GTGAATGTCCCCCCAACTTCACCGGCTCCAACTGCGAGAAGAAAGTGGACAGGTGCACCAGCA
ACCCCTGTGCCAACGGGGGACAGTGCCTGAACCGAGGTCCAAGCCGCATGTGCCGCTGCCGTC
CTGGATTCACGGGCACCTACTGTGAACTCCACGTCAGCGACTGTGCCCGTAACCCTTGCGCCC
ACGGTGGCACTTGCCATGACCTGGAGAATGGGCTCATGTGCACCTGCCCTGCCGGCTTCTCTG
GCCGACGCTGTGAGGTGCGGACATCCATCGATGCCTGTGCCTCGAGTCCCTGCTTCAACAGGG
CCACCTGCTACACCGACCTCTCCACAGACACCTTTGTGTGCAACTGCCCTTATGGCTTTGTGG
GCAGCCGCTGCGAGTTCCCCGTGGGCTTGCCGCCCAGCTTCCCCTGGGTGGCCGTCTCGCTGG
GTGTGGGGCTGGCAGTGCTGCTGGTACTGCTGGGCATGGTGGCAGTGGCTGTGCGGCAGCTGC
GGCTTCGACGGCCGGACGACGGCAGCAGGGAAGCCATGAACAACTTGTCGGACTTCCAGAAGG
ACAACCTGATTCCTGCCGCCCAGCTTAAAAACACAAACCAGAAGAAGGAGCTGGAAGTGGACT
GTGGCCTGGACAAGTCCAACTGTGGCAAACAGCAAAACCACACATTGGACTATAATCTGGCCC
CAGGGCCCCTGGGGCGGGGGACCATGCCAGGAAAGTTTCCCCACAGTGACAAGAGCTTAGGAG
AGAAGGCGCCACTGCGGTTACACAGTGAAAAGCCAGAGTGTCGGATATCAGCGATATGCTCCC
CCAGGGACTCCATGTACCAGTCTGTGTGTTTGATATCAGAGGAGAGGAATGAATGTGTCATTG
CCACGGAGGTA**TAA**GGCAGGAGCCTACCTGGACATCCCTGCTCAGCCCCGCGGCTGGACCTTC
CTTCTGCATTGTTTACA

# FIGURE 88

MAAASRSASGWALLLLVALWQQRAAGSGVFQLQLQEFINERGVLASGRPCEPGCRTFFRVCLK
HFQAVVSPGPCTFGTVSTPVLGTNSFAVRDDSSGGGRNPLQLPFNFTWPGTFSLIIEAWHAPG
DDLRPEALPPDALISKIAIQGSLAVGQNWLLDEQTSTLTRLRYSYRVICSDNYYGDNCSRLCK
KRNDHFGHYVCQPDGNLSCLPGWTGEYCQQPICLSGCHEQNGYCSKPAECLCRPGWQGRLCNE
CIPHNGCRHGTCSTPWQCTCDEGWGGLFCDQDLNYCTHHSPCKNGATCSNSGQRSYTCTCRPG
YTGVDCELELSECDSNPCRNGGSCKDQEDGYHCLCPPGYYGLHCEHSTLSCADSPCFNGGSCR
ERNQGANYACECPPNFTGSNCEKKVDRCTSNPCANGGQCLNRGPSRMCRCRPGFTGTYCELHV
SDCARNPCAHGGTCHDLENGLMCTCPAGFSGRRCEVRTSIDACASSPCFNRATCYTDLSTDTF
VCNCPYGFVGSRCEFPVGLPPSFPWVAVSLGVGLAVLLVLLGMVAVAVRQLRLRRPDDGSREA
MNNLSDFQKDNLIPAAQLKNTNQKKELEVDCGLDKSNCGKQQNHTLDYNLAPGPLGRGTMPGK
FPHSDKSLGEKAPLRLHSEKPECRISAICSPRDSMYQSVCLISEERNECVIATEV

**Important features of the protein:**

**Signal peptide:**

amino acids 1-26

**Transmembrane domain:**

amino acids 530-552

**N-glycosylation sites.**

amino acids 108-112, 183-187, 205-209, 393-397, 570-574, 610-614

**Glycosaminoglycan attachment site.**

amino acids 96-100

**Tyrosine kinase phosphorylation site.**

amino acids 340-347

**N-myristoylation sites.**

amino acids 42-48, 204-210, 258-264, 277-283, 297-303, 383-389, 415-421, 461-467, 522-528, 535-541, 563-569, 599-605, 625-631

**Amidation site.**

amino acids 471-475

**Aspartic acid and asparagine hydroxylation site.**

amino acids 339-351

**EGF-like domain cysteine pattern signature.**

amino acids 173-185, 206-218, 239-251, 270-282, 310-322, 348-360, 388-400, 426-438, 464-476, 506-518

**Calcium-binding EGF-like:**

amino acids 224-245, 255-276, 295-316, 333-354, 373-394, 411-432, 449-470

# FIGURE 89

GTCTCCGCGTCACAGGAACTTCAGCACCCACAGGGCGGACAGCGCTCCCCTCTACCTGGAGAC
TTGACTCCCGCGCGCCCCAACCCTGCTTATCCCTTGACCGTCGAGTGTCAGAGATCCTGCAGC
CGCCCAGTCCCGGCCCCTCTCCCGCCCCACACCCACCCTCCTGGCTCTTCCTGTTTTTACTCC
TCCTTTTCATTCATAACAAAAGCTACAGCTCCAGGAGCCCAGCGCCGGGCTGTGACCCAAGCC
GAGCGTGGAAGA**ATG**GGGTTCCTCGGACCGGCACTTGGATTCTGGTGTTAGTGCTCCCGATT
CAAGCTTTCCCCAAACCTGGAGGAAGCCAAGACAAATCTCTACATAATAGAGAATTAAGTGCA
GAAAGACCTTTGAATGAACAGATTGCTGAAGCAGAAGAAGACAAGATTAAAAAAACATATCCT
CCAGAAAACAAGCCAGGTCAGAGCAACTATTCTTTTGTTGATAACTTGAACCTGCTAAAGGCA
ATAACAGAAAAGGAAAAAATTGAGAAAGAAAGACAATCTATAAGAAGCTCCCCACTTGATAAT
AAGTTGAATGTGGAAGATGTTGATTCAACCAAGAATCGAAAACTGATCGATGATTATGACTCT
ACTAAGAGTGGATTGGATCATAAATTTCAAGATGATCCAGATGGTCTTCATCAACTAGACGGG
ACTCCTTTAACCGCTGAAGACATTGTCCATAAAATCGCTGCCAGGATTTATGAAGAAAATGAC
AGAGCCGTGTTTGACAAGATTGTTTCTAAACTACTTAATCTCGGCCTTATCACAGAAAGCCAA
GCACATACACTGGAAGATGAAGTAGCAGAGGTTTTACAAAAATTAATCTCAAAGGAAGCCAAC
AATTATGAGGAGGATCCCAATAAGCCCACAAGCTGGACTGAGAATCAGGCTGGAAAAATACCA
GAGAAAGTGACTCCAATGGCAGCAATTCAAGATGGTCTTGCTAAGGGAGAAAACGATGAAACA
GTATCTAACACATTAACCTTGACAAATGGCTTGGAAAGGAGAACTAAAACCTACAGTGAAGAC
AACTTTGAGGAACTCCAATATTTCCCAAATTTCTATGCGCTACTGAAAAGTATTGATTCAGAA
AAAGAAGCAAAAGAGAAAGAAACACTGATTACTATCATGAAAACACTGATTGACTTTGTGAAG
ATGATGGTGAAATATGGAACAATATCTCCAGAAGAAGGTGTTTCCTACCTTGAAAACTTGGAT
GAAATGATTGCTCTTCAGACCAAAAACAAGCTAGAAAAAAATGCTACTGACAATATAAGCAAG
CTTTTCCCAGCACCATCAGAGAAGAGTCATGAAGAAACAGACAGTACCAAGGAAGAAGCAGCT
AAGATGGAAAAGGAATATGGAAGCTTGAAGGATTCCACAAAAGATGATAACTCCAACCCAGGA
GGAAAGACAGATGAACCCAAAGGAAAAACAGAAGCCTATTTGGAAGCCATCAGAAAAAATATT
GAATGGTTGAAGAAACATGACAAAAAGGGAAATAAAGAAGATTATGACCTTTCAAAGATGAGA
GACTTCATCAATAAACAAGCTGATGCTTATGTGGAGAAAGGCATCCTTGACAAGGAAGAAGCC
GAGGCCATCAAGCGCATTTATAGCAGCCTG**TAA**AAATGGCAAAAGATCCAGGAGTCTTTCAAC
TGTTTCAGAAAACATAATATAGCTTAAAACACTTCTAATTCTGTGATTAAAATTTTTTGACCC
AAGGGTTATTAGAAAGTGCTGAATTTACAGTAGTTAACCTTTTACAAGTGGTTAAAACATAGC
TTTCTTCCCGTAAAAACTATCTGAAAGTAAAGTTGTATGTAAGCTGAAAAAAAAAAAAAAAAA
AAA

# FIGURE 90

MGFLGTGTWILVLVLPIQAFPKPGGSQDKSLHNRELSAERPLNEQIAEAEEDKIKKTYPPENK
PGQSNYSFVDNLNLLKAITEKEKIEKERQSIRSSPLDNKLNVEDVDSTKNRKLIDDYDSTKSG
LDHKFQDDPDGLHQLDGTPLTAEDIVHKIAARIYEENDRAVFDKIVSKLLNLGLITESQAHTL
EDEVAEVLQKLISKEANNYEEDPNKPTSWTENQAGKIPEKVTPMAAIQDGLAKGENDETVSNT
LTLTNGLERRTKTYSEDNFEELQYFPNFYALLKSIDSEKEAKEKETLITIMKTLIDFVKMMVK
YGTISPEEGVSYLENLDEMIALQTKNKLEKNATDNISKLFPAPSEKSHEETDSTKEEAAKMEK
EYGSLKDSTKDDNSNPGGKTDEPKGKTEAYLEAIRKNIEWLKKHDKKGNKEDYDLSKMRDFIN
KQADAYVEKGILDKEEAEAIKRIYSSL

**Important features:**

**N-glycosylation sites:**

amino acids 68-71, 346-349, 350-353

**Casein kinase II phosphorylation site:**

amino acids 70-73, 82-85, 97-100, 125-128, 147-150, 188-191, 217-220, 265-268, 289-292, 305-308, 320-323, 326-329, 362-365, 368-341, 369-372, 382-385, 386-389, 387-390

**N-myristoylation sites:**

amino acids 143-148, 239-244

# FIGURE 91

TGCATCAGTGCCCAGGCAAGCCCAGGAGTTGACATTTCTCTGCCCAGCC**ATG**GGCCTCACCCT
GCTCTTGCTGCTGCTCCTGGGACTAGAAGGTCAGGGCATAGTTGGCAGCCTCCCTGAGGTGCT
GCAGGCACCCGTGGGAAGCTCCATTCTGGTGCAGTGCCACTACAGGCTCCAGGATGTCAAAGC
TCAGAAGGTGTGGTGCCGGTTCTTGCCGGAGGGGTGCCAGCCCCTGGTGTCCTCAGCTGTGGA
TCGCAGAGCTCCAGCGGGCAGGCGTACGTTTCTCACAGACCTGGGTGGGGGCCTGCTGCAGGT
GGAAATGGTTACCCTGCAGGAAGAGGATGCTGGCGAGTATGGCTGCATGGTGGATGGGGCCAG
GGGGCCCCAGATTTTGCACAGAGTCTCTCTGAACATACTGCCCCCAGAGGAAGAAGAAGAGAC
CCATAAGATTGGCAGTCTGGCTGAGAACGCATTCTCAGACCCTGCAGGCAGTGCCAACCCTTT
GGAACCCAGCCAGGATGAGAAGAGCATCCCCTTGATCTGGGGTGCTGTGCTCCTGGTAGGTCT
GCTGGTGGCAGCGGTGGTGCTGTTTGCTGTGATGGCCAAGAGGAAACAAGAATCCCTCCTCAG
TGGTCCACCACGTCAG**TGA**CTCTGGACCGGCTGCTGAATTGCCTTTGGATGTACCACACATTA
GGCTTGACTCACCACCTTCATTTGACAATACCACCTACACCAGCCTACCTCTTGATTCCCCAT
CAGGAAAACCTTCACTCCCAGCTCCATCCTCATTGCCCCCTCTACCTCCTAAGGTCCTGGTCT
GCTCCAAGCCTGTGACATATGCCACAGTAATCTTCCCGGGAGGGAACAAGGGTGGAGGGACCT
CGTGTGGGCCAGCCCAGAATCCACCTAACAATCAGACTCCATCCAGCTAAGCTGCTCATCACA
CTTTAAACTCATGAGGACCATCCCTAGGGGTTCTGTGCATCCATCCAGCCAGCTCATGCCCTA
GGATCCTTAGGATATCTGAGCAACCAGGGACTTTAAGATCTAATCCAATGTCCTAACTTTACT
AGGGAAAGTGACGCTCAGACATGACTGAGATGTCTTGGGGAAGACCTCCCTGCACCCAACTCC
CCCACTGGTTCTTCTACCATTACACACTGGGCTAAATAAACCCTAATAATGATGTGCAAAAAA
AAAAAAAAAAAAAAAAAAAAAAAAAAAAAAAAAAAAAAAAA

# FIGURE 92

MGLTLLLLLLLGLEGQGIVGSLPEVLQAPVGSSILVQCHYRLQDVKAQKVWCRFLPEGCQPLV
SSAVDRRAPAGRRTFLTDLGGGLLQVEMVTLQEEDAGEYGCMVDGARGPQILHRVSLNILPPE
EEEETHKIGSLAENAFSDPAGSANPLEPSQDEKSIPLIWGAVLLVGLLVAAVVLFAVMAKRKQ
ESLLSGPPRQ

**Important features of the protein:**

**Signal peptide:**

amino acids 1-15

**Transmembrane domain:**

amino acids 161-181

**N-myristoylation sites.**

amino acids 17-23, 172-178

**Amidation site.**

amino acids 73-79

# FIGURE 93

GGCGGCGTTGCCGGGCTCTCCGGAAGGAGACGTGGCGGCGGTTGGGCCGGTGATACCCGGGCG
CTTTATAGTCCCGCCGCCTCCTCCTCCACCTCCTCCTCCTCCTCCTCTCCTCCTGGGGCAGAG
GAGGTTGTGGCGGTGGCTGGAGAAAGCGGCGGCGGAGG**ATG**GAGGAAGGAGGCGGCGGCGTAC
GGAGTCTGGTCCCGGGCGGGCCGGTGTTACTGGTCCTCTGCGGCCTCCTGGAGGCGTCCGGCG
GCGGCCGAGCCCTTCCTCAACTCAGCGATGACATCCCTTTCCGAGTCAACTGGCCCGGCACCG
AGTTCTCTCTGCCCACAACTGGAGTTTTATATAAAGAAGATAATTATGTCATCATGACAACTG
CACATAAAGAAAAATATAAATGCATACTTCCCCTTGTGACAAGTGGGGATGAGGAAGAAGAAA
AGGATTATAAAGGCCCTAATCCAAGAGAGCTTTTGGAGCCACTATTTAAACAAAGCAGTTGTT
CCTACAGAATTGAGTCTTATTGGACTTACGAAGTATGTCATGGAAAACACATTCGGCAGTACC
ATGAAGAGAAAGAAACTGGTCAGAAAATAAATATTCACGAGTACTACCTTGGGAATATGTTGG
CCAAGAACCTTCTATTTGAAAAAGAACGAGAAGCAGAAGAAAAGGAAAAATCAAATGAGATTC
CCACTAAAAATATCGAAGGTCAGATGACACCATACTATCCTGTGGGAATGGGAAATGGTACAC
CTTGTAGTTTGAAACAGAACCGGCCCAGATCAAGTACTGTGATGTACATATGTCATCCTGAAT
CTAAGCATGAAATTCTTTCAGTAGCTGAAGTTACAACTTGTGAATATGAAGTTGTCATTTTGA
CACCACTCTTGTGCAGTCATCCTAAATATAGGTTCAGAGCATCTCCTGTGAATGACATATTTT
GTCAATCACTGCCAGGATCTCCATTTAAGCCCCTCACCCTGAGGCAGCTGGAGCAGCAGGAAG
AAATACTAAGGGTGCCTTTTAGGAGAAATAAAGAGGGTGTCGGTTGGTGGAAATATGAATTCT
GCTATGGCAAACATGTACATCAATACCATGAGGACAAGGATAGTGGGAAAACCTCTGTGGTTG
TCGGGACATGGAACCAAGAAGAGCATATTGAATGGGCTAAGAAGAATACTGCTAGAGCTTATC
ATCTTCAAGACGATGGTACCCAGACAGTCAGGATGGTGTCACATTTTTATGGAAATGGAGATA
TTTGTGATATAACTGACAAACCAAGACAGGTGACTGTAAAACTAAAGTGCAAAGAATCAGATT
CACCTCATGCTGTTACTGTATATATGCTAGAGCCTCACTCCTGTCAATATATTCTTGGGGTTG
AATCTCCAGTGATCTGTAAAATCTTAGATACAGCAGATGAAAATGGACTTCTTTCTCTCCCCA
AC**TAA**AGGATATTAAAGTTAGGGGAAAGAAAAGATCATTGAAAGTCATGATAATTTCTGTCCC
ACTGTGTCTCATTATAGAGTTCTCAGCCATTGGACCTCTTCTAAAGGATGGTATAAAATGACT
CTCAACCACTTTGTGAATACATATGTGTATATAAGAGGTTATTGATAAACTTCTGAGGCAGAC
ATTTGTCTCGCTTTTTTTCATTTTTGTTGTGTCTTATAAACTGACTGTTTTTCTTTGCTTGGA
TACTGTGATTCCAAAATAAATCTCATCCAAGCAAGTTAGAGTCCAGCCTAATCAAATGTCATA
ATTGTTGTACCTATTGAAAGTTTTTAAATAATAGATTTATTATGTAAATTATAGTATATGTAA
GTAGCTAATGAAGTAAAGATCATGAAGAAAGAAATTGATAGGTGTAAATGAGAGACCATGTAA
AATATGTAAATTCTAGTACCTGAAATCCTTTCAACAGATTTTTATATAGCAACTGCTCTCTGC
AAGTAGTTAAACTAGAAACTGGGCACATGGTAGAGGCTCACATGGGAGTTGTCCTCACCCTTG
TTAATCTCAAGAAACTCTTATTTATAATAGGTTGCTTCTCTCTCAGAACTTTTATCTATTACT
TTTTTCTTCTTATGAGTATGTTTACTCTCAGAGTATCTATCTGATGTAGACAGTTGGTGATGC
TTCTGAGACTCAGAATGGTTTACTCTAACAAAACACTGTGCTGTCTATCCCTTGTACTTGCCT
ACTGTAATATGGATTTCACTTCTGAACAGTTTACAGCACAATATTTATTTTAAAGTGAATAAA
ATGTCCACAAGCAAAAA

# FIGURE 94

MEEGGGGVRSLVPGGPVLLVLCGLLEASGGGRALPQLSDDIPFRVNWPGTEFSLPTTGVLYKE
DNYVIMTTAHKEKYKCILPLVTSGDEEEEKDYKGPNPRELLEPLFKQSSCSYRIESYWTYEVC
HGKHIRQYHEEKETGQKINIHEYYLGNMLAKNLLFEKEREAEEKEKSNEIPTKNIEGQMTPYY
PVGMGNGTPCSLKQNRPRSSTVMYICHPESKHEILSVAEVTTCEYEVVILTPLLCSHPKYRFR
ASPVNDIFCQSLPGSPFKPLTLRQLEQQEEILRVPFRRNKEGVGWWKYEFCYGKHVHQYHEDK
DSGKTSVVVGTWNQEEHIEWAKKNTARAYHLQDDGTQTVRMVSHFYGNGDICDITDKPRQVTV
KLKCKESDSPHAVTVYMLEPHSCQYILGVESPVICKILDTADENGLLSLPN

**Important features of the protein:**

**Signal peptide:**

amino acids 1-30

**Glycosaminoglycan attachment site.**

amino acids 28-32

**cAMP- and cGMP-dependent protein kinase phosphorylation site.**

amino acids 337-341

**N-myristoylation sites.**

amino acids 6-12, 23-29, 29-35, 49-55, 141-147, 152-158, 192-198, 196-202

**Gram-positive cocci surface proteins 'anchoring' hexapeptide.**

amino acids 54-60

# FIGURE 95

TTCCGTTTCTGGGAGGAGTGAGGGGCAACGGGTCGGAGAAAAAGGAAAAAAGAAGGGCTCAGC
GCCTCCCCGCCGGGCCGTGGACAGAGGGGCACAGTTTCGGCAGGCGGGTGAGGTCGCTGAGGG
CCCGCCGGAG**ATG**TTTTCCTTGTCGAGCACGGTGCAACCCCAGGTTACAGTTCCTCTGAGTCA
TCTCATCAATGCCTTCCATACACCAAAAAACACTTCTGTTTCTCTCAGTGGAGTGTCAGTTTC
TCAAAACCAGCATCGAGATGTAGTTCCTGAGCATGAGGCTCCCAGCAGTGAGCCTTCACTTAA
CTTAAGGGACCTTGGATTATCTGAACTAAAAATTGGACAGATTGATCAGCTGGTAGAAAATCT
ACTTCCTGGATTTTGTAAAGGCAAAAACATTTCTTCCCATTGGCATACATCCCATGTCTCTGC
ACAATCCTTCTTTGAAAATAAATATGGTAACTTAGATATATTTAGTACATTACGTTCCTCTTG
CTTGTATCGACATCATTCAAGAGCTCTTCAAAGCATTTGTTCAGATCTTCAGTACTGGCCAGT
TTTCATACAGTCTCGGGGTTTTAAAACTTTGAAATCAAGGACACGACGTCTCCAGTCTACCTC
CGAGAGATTAGCTGAAACACAGAATATAGCGCCATCATTCGTGAAGGGGTTTCTTTTGCGGGA
CAGAGGATCAGATGTTGAGAGTTTGGACAAACTCATGAAAACCAAAAATATACCTGAAGCTCA
CCAAGATGCATTTAAAACTGGTTTTGCGGAAGGTTTTCTGAAAGCTCAAGCACTCACACAAAA
AACCAATGATTCCCTAAGGCGAACCCGTCTGATTCTCTTCGTTCTGCTGCTATTCGGCATTTA
TGGACTTCTAAAAAACCCATTTTTATCTGTCCGCTTCCGGACAACAACAGGGCTTGATTCTGC
AGTAGATCCTGTCCAGATGAAAAATGTCACCTTTGAACATGTTAAAGGGGTGGAGGAAGCTAA
ACAAGAATTACAGGAAGTTGTTGAATTCTTGAAAAATCCACAAAAATTTACTATTCTTGGAGG
TAAACTTCCAAAAGGAATTCTTTTAGTTGGACCCCCAGGGACTGGAAAGACACTTCTTGCCCG
AGCTGTGGCGGGAGAAGCTGATGTTCCTTTTTATTATGCTTCTGGATCCGAATTTGATGAGAT
GTTTGTGGGTGTGGGAGCCAGCCGTATCAGAAATCTTTTTAGGGAAGCAAAGGCGAATGCTCC
TTGTGTTATATTTATTGATGAATTAGATTCTGTTGGTGGGAAGAGAATTGAATCTCCAATGCA
TCCATATTCAAGGCAGACCATAAATCAACTTCTTGCTGAAATGGATGGTTTTAAACCCAATGA
AGGAGTTATCATAATAGGAGCCACAAACTTCCCAGAGGCATTAGATAATGCCTTAATACGTCC
TGGTCGTTTTGACATGCAAGTTACAGTTCCAAGGCCAGATGTAAAAGGTCGAACAGAAATTTT
GAAATGGTATCTCAATAAAATAAAGTTTGATCAATCCGTTGATCCAGAAATTATAGCTCGAGG
TACTGTTGGCTTTTCCGGAGCAGAGTTGGAGAATCTTGTGAACCAGGCTGCATTAAAAGCAGC
TGTTGATGGAAAAGAAATGGTTACCATGAAGGAGCTGGAGTTTTCCAAAGACAAAATTCTAAT
GGGGCCTGAAAGAAGAAGTGTGGAAATTGATAACAAAAACAAAACCATCACAGCATATCATGA
ATCTGGTCATGCCATTATTGCATATTACACAAAAGATGCAATGCCTATCAACAAAGCTACAAT
CATGCCACGGGGGCCAACACTTGGACATGTGTCCCTGTTACCTGAGAATGACAGATGGAATGA
AACTAGAGCCCAGCTGCTTGCACAAATGGATGTTAGTATGGGAGGAAGAGTGGCAGAGGAGCT
TATATTTGGAACCGACCATATTACAACAGGTGCTTCCAGTGATTTTGATAATGCCACTAAAAT
AGCAAAGCGGATGGTTACCAAATTTGGAATGAGTGAAAAGCTTGGAGTTATGACCTACAGTGA
TACAGGGAAACTAAGTCCAGAAACCCAATCTGCCATCGAACAAGAAATAAGAATCCTTCTAAG
GGACTCATATGAACGAGCAAAACATATCTTGAAAACTCATGCAAAGGAGCATAAGAATCTCGC
AGAAGCTTTATTGACCTATGAGACTTTGGATGCCAAAGAGATTCAAATTGTTCTTGAGGGGAA
AAAGTTGGAAGTGAGA**TGA**TAACTCTCTTGATATGGATGCTTGCTGGTTTTATTGCAAGAATA
TAAGTAGCATTGCAGTAGTCTACTTTTACAACGCTTTCCCCTCATTCTTGATGTGGTGTAATT
GAAGGGTGTGAAATGCTTTGTCAATCATTTGTCACATTTATCCAGTTTGGGTTATTCTCATTA
TGACACCTATTGCAAATTAGCATCCCATGGCAAATATATTTTGAAAAAATAAAGAACTATCAG
GATTGAAAACAAAAAAAAAAAA

# FIGURE 96

MFSLSSTVQPQVTVPLSHLINAFHTPKNTSVSLSGVSVSQNQHRDVVPEHEAPSSEPSLNLRD
LGLSELKIGQIDQLVENLLPGFCKGKNISSHWHTSHVSAQSFFENKYGNLDIFSTLRSSCLYR
HHSRALQSICSDLQYWPVFIQSRGFKTLKSRTRRLQSTSERLAETQNIAPSFVKGFLLRDRGS
DVESLDKLMKTKNIPEAHQDAFKTGFAEGFLKAQALTQKTNDSLRRTRLILFVLLLFGIYGLL
KNPFLSVRFRTTTGLDSAVDPVQMKNVTFEHVKGVEEAKQELQEVVEFLKNPQKFTILGGKLP
KGILLVGPPGTGKTLLARAVAGEADVPFYYASGSEFDEMFVGVGASRIRNLFREAKANAPCVI
FIDELDSVGGKRIESPMHPYSRQTINQLLAEMDGFKPNEGVIIIGATNFPEALDNALIRPGRF
DMQVTVPRPDVKGRTEILKWYLNKIKFDQSVDPEIIARGTVGFSGAELENLVNQAALKAAVDG
KEMVTMKELEFSKDKILMGPERRSVEIDNKNKTITAYHESGHAIIAYYTKDAMPINKATIMPR
GPTLGHVSLLPENDRWNETRAQLLAQMDVSMGGRVAEELIFGTDHITTGASSDFDNATKIAKR
MVTKFGMSEKLGVMTYSDTGKLSPETQSAIEQEIRILLRDSYERAKHILKTHAKEHKNLAEAL
LTYETLDAKEIQIVLEGKKLEVR

**Important features of the protein:**

**Transmembrane domain:**

amino acids 238-259

**N-glycosylation sites.**

amino acids 28-32, 90-94, 230-234, 278-282, 535-539, 584-588, 623-627

**N-myristoylation sites.**

amino acids 35-41, 266-272, 286-292, 325-331, 357-363, 599-605

**Amidation site.**

amino acids 387-393, 709-713

**ATP/GTP-binding site motif A (P-loop).**

amino acids 322-330

**AAA-protein family proteins**

amino acids 315-336, 343-386, 405-451

# FIGURE 97

GATGGCGCAGCCACAGCTTCTGTGAGATTCGATTTCTCCCCAGTTCCCCTGTGGGTCTGAGGG
GACCAGAAGGGTGAGCTACGTTGGCTTTCTGGAAGGGGAGGCTATATGCGTCAATTCCCCAAA
ACAAGTTTTGACATTTCCCCTGAAATGTCATTCTCTATCTATTCACTGCAAGTGCCTGCTGTT
CCAGGCCTTACCTGCTGGGCACTAACGGCGGAGCCAGGATGGGGACAGAATAAAGGAGCCACG
ACCTGTGCCACCAACTCGCACTCAGACTCTGAACTCAGACCTGAAATCTTCTCTTCACGGGAG
GCTTGGCAGTTTTTCTTACTCCTGTGGTCTCCAGATTTCAGGCCTAAG**ATG**AAAGCCTCTAGT
CTTGCCTTCAGCCTTCTCTCTGCTGCGTTTTATCTCCTATGGACTCCTTCCACTGGACTGAAG
ACACTCAATTTGGGAAGCTGTGTGATCGCCACAAACCTTCAGGAAATACGAAATGGATTTTCT
GAGATACGGGGCAGTGTGCAAGCCAAAGATGGAAACATTGACATCAGAATCTTAAGGAGGACT
GAGTCTTTGCAAGACACAAAGCCTGCGAATCGATGCTGCCTCCTGCGCCATTTGCTAAGACTC
TATCTGGACAGGGTATTTAAAAACTACCAGACCCCTGACCATTATACTCTCCGGAAGATCAGC
AGCCTCGCCAATTCCTTTCTTACCATCAAGAAGGACCTCCGGCTCTCTCATGCCCACATGACA
TGCCATTGTGGGGAGGAAGCAATGAAGAAATACAGCCAGATTCTGAGTCACTTTGAAAAGCTG
GAACCTCAGGCAGCAGTTGTGAAGGCTTTGGGGGAACTAGACATTCTTCTGCAATGGATGGAG
GAGACAGAA**TAG**GAGGAAAGTGATGCTGCTGCTAAGAATATTCGAGGTCAAGAGCTCCAGTCT
TCAATACCTGCAGAGGAGGCATGACCCCAAACCACCATCTCTTTACTGTACTAGTCTTGTGCT
GGTCACAGTGTATCTTATTTATGCATTACTTGCTTCCTTGCATGATTGTCTTTATGCATCCCC
AATCTTAATTGAGACCATACTTGTATAAGATTTTTGTAATATCTTTCTGCTATTGGATATATT
TATTAGTTAATATATTTATTTATTTTTTGCTATTTAATGTATTTATTTTTTTACTTGGACATG
AAACTTTAAAAAAATTCACAGATTATATTTATAACCTGACTAGAGCAGGTGATGTATTTTTAT
ACAGTAAAAAAAAAAAAACCTTGTAAATTCTAGAAGAGTGGCTAGGGGGGTTATTCATTTGTAT
TCAACTAAGGACATATTTACTCATGCTGATGCTCTGTGAGATATTTGAAATTGAACCAATGAC
TACTTAGGATGGGTTGTGGAATAAGTTTTGATGTGGAATTGCACATCTACCTTACAATTACTG
ACCATCCCCAGTAGACTCCCCAGTCCCATAATTGTGTATCTTCCAGCCAGGAATCCTACACGG
CCAGCATGTATTTCTACAAATAAAGTTTCTTTGCATACCAAAAAAAAAAAAAAAAAA

# FIGURE 98

MKASSLAFSLLSAAFYLLWTPSTGLKTLNLGSCVIATNLQEIRNGFSEIRGSVQAKDGNIDIR
ILRRTESLQDTKPANRCCLLRHLLRLYLDRVFKNYQTPDHYTLRKISSLANSFLTIKKDLRLC
HAHMTCHCGEEAMKKYSQILSHFEKLEPQAAVVKALGELDILLQWMEETE

**Signal sequence:**
amino acids 1-24

**cAMP- and cGMP-dependent protein kinase phosphorylation sites.**
amino acids 107-110, 140-143

**N-myristoylation site.**
amino acids 51-56

**Interleukin 10:**
amino acids 9-176

# FIGURE 99

GCGCCGGCTCCGCGCCTCGCGCCCAGTCCGCGGGCCGCGCCGCCGCTCCCGCCGCTCCCGCCG
CTCCCGCAGCCGCCCCGCCGCCCGCCCGGAGCCCCGCGTCCCTAGGCCTGGCTCCCGCCTGCC
CGAGACCCGCCCAGCCTGCCCCGCTCAGCCGCCAGAGAAG**ATG**CGGCTGCTCCCGGAATGGTT
CCTCTTGCTCTTTGGCCCGTGGCTCCTTAGGAAGGCCGTCAGTGCCCAGATACCAGAGTCCGG
AAGGCCGCAGTACCTGGGGCTGCGCCCCGCCGCGGCCGGAGCGGGTGCCCCCGGCCAGCAGCT
CCCAGAGCCAAGGTCTTCGGACGGCCTAGGCGTGGGCCGCGCCTGGAGCTGGGCCTGGCCGAC
CAACCACACGGGGGCGCTGGCCCGGGCAGGGGCAGCCGGGGCGTTGCCCGCGCAGCGCACCAA
GAGGAAGCCGTCCATCAAGGCGGCGCGCGCCAAAAAGATCTTCGGCTGGGGGGACTTCTACTT
TCGGGTGCATACCCTCAAGTTTTCGCTGCTGGTGACCGGCAAGATCGTGGACCATGTGAACGG
TACCTTCAGTGTGTATTTCCGCCACAACTCGTCCAGCCTGGGCAACCTCAGTGTCAGCATCGT
GCCGCCCTCCAAGCGTGTCGAGTTCGGAGGAGTCTGGCTGCCCGGGCCTGTCCCCCACCCTCT
GCAGTCTACGCTCGCCCTGGAGGGGGTGCTTCCTGGGCTGGGGCCCCCGCTGGGGATGGCAGC
AGCAGCGGCGGGGCCGGGGCTTGGGGGCTCCCTCGGGGGCGCACTGGCGGGGCCGCTTGGGGG
CGCGTTGGGAGTGCCTGGGGCCAAAGAGTCACGCGCTTTCAATTGCCACGTGGAGTATGAGAA
GACAAACCGCGCGCGCAAGCACCGACCGTGCCTGTACGACCCGTCGCAGGTGTGTTTCACCGA
GCACACGCAGAGCCAGGCCGCCTGGCTCTGTGCCAAGCCCTTCAAAGTCATCTGTATCTTCGT
CTCTTTCCTCAGCTTTGACTACAAACTGGTGCAGAAGGTGTGCCCAGACTATAACTTCCAGAG
TGAGCACCCCTACTTCGGA**TAG**CGCCCCTCCCCAGCCAGTCCTGAGCCTCCCGCCAAATCCCA
GCCTCACTAGGTGGGACCCCCTTCCCAGTGTTCTGCCGCTCCTGTGGCCATGTCGCCCACTCC
TTCCACTCTGGGGGCGGAGGGGAATGGCTTCTCGGGACCCTCAGCTAGCGTGGGTGCCCTTTT
CCTTATGCGGAGTGCCCGCAAGGCTGGGGTAGCCCCCTCCAGTACACCCCAAAGTGAAAGGGA
TAAGAGTGCAGCCCCAGAATAGGCGGGGCTTGGAGGCGGTCCCAATGTCCCCTGGGTCCACAG
TGGGTCCCCTTTTCACCCTTGGCGCTAGGCTGCGCACTCCCTTTCCCCGCAGCTTTAATAACT
CCTGGCCTGGCACCCTCACCCCACCCTGACTTTCCCATCCCCCAGCGCTTGTCCTGCTTCACC
ATACCCCGCCTAAGACTGTAAAGGCCTAAAAACCTCGGCCTGTCCTCCCACCATTCTGCCTGC
CATATGCCTGTCCCCTTTTCCTCCAAACCCTATTAGGGTACCGGAAGCAGAACCCCTGGGCTG
AGGCCCTGGCCCTGCCCCCGGCCCCTGCCCCTGCCCGCCCCCCTCCAGTCCAGGCAGTCGAGC
TCCACCTGCCCTCTCCTGCTGCTTCCTCTCGGTGATATTTTTCTACGCCAAAACAGACGGGA
AAGGGAACAAAATAAAGTGAAATCCCAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAA
AAAAAAAAAAAAAA

# FIGURE 100

MRLLPEWFLLLFGPWLLRKAVSAQIPESGRPQYLGLRPAAAGAGAPGQQLPEPRSSDGLGVGR
AWSWAWPTNHTGALARAGAAGALPAQRTKRKPSIKAARAKKIFGWGDFYFRVHTLKFSLLVTG
KIVDHVNGTFSVYFRHNSSSLGNLSVSIVPPSKRVEFGGVWLPGPVPHPLQSTLALEGVLPGL
GPPLGMAAAAAGPGLGGSLGGALAGPLGGALGVPGAKESRAFNCHVEYEKTNRARKHRPCLYD
PSQVCFTEHTQSQAAWLCAKPFKVICIFVSFLSFDYKLVQKVCPDYNFQSEHPYFG

**Important features of the protein:**

**Signal peptide:**

amino acids 1-22

**Transmembrane domain:**

amino acids 273-288

**N-glycosylation sites.**

amino acids 72-76, 133-137, 143-147, 149-153

**cAMP- and cGMP-dependent protein kinase phosphorylation site.**

amino acids 93-97

**N-myristoylation sites.**

amino acids 35-41, 58-64, 60-66, 81-87, 84-90, 184-190, 194-200, 203-209, 205-211, 206-212, 209-215, 217-223, 221-227, 224-230

**Cytochrome b/b6 Qo site signature.**

amino acids 5-11

# FIGURE 101

AATGCCCC**ATG**CGCACCCCACAGCTCGCGCTCCTGCAAGTGTTCTTTCTGGTGTTCCCCGATG
GCGTCCGGCCTCAGCCCTCTTCCTCCCCATCAGGGGCAGTGCCCACGTCTTTGGAGCTGCAGC
GAGGGACGGATGGCGGAACCCTCCAGTCCCCTTCAGAGGCGACTGCAACTCGCCCGGCCGTGC
CTGGACTCCCTACAGTGGTCCCTACTCTCGTGACTCCCTCGGCCCCTGGGAATAGGACTGTGG
ACCTCTTCCCAGTCTTACCGATCTGTGTCTGTGACTTGACTCCTGGAGCCTGCGATATAAATT
GCTGCTGCGACAGGGACTGCTATCTTCTCCATCCGAGGACAGTTTTCTCCTTCTGCCTTCCAG
GCAGCGTAAGGTCTTCAAGCTGGGTTTGTGTAGACAACTCTGTTATCTTCAGGAGTAATTCCC
CGTTTCCTTCAAGAGTTTTCATGGATTCTAATGGAATCAGGCAGTTTTGTGTCCATGTGAACA
ACTCAAACTTAAACTATTTCCAGAAGCTTCAAAAGGTCAATGCAACCAACTTCCAGGCCCTGG
CTGCAGAGTTTGGAGGCGAATCATTCACTTCAACATTCCAAACTCAATCACCACCATCTTTTT
ACAGGGCTGGGGACCCCATTCTTACTTACTTCCCCAAGTGGTCTGTAATAAGCTTGCTGAGAC
AACCTGCAGGAGTTGGAGCTGGGGGACTCTGTGCTGAAAGCAATCCTGCAGGTTTCCTAGAGA
GTAAAAGTACAACTTGCACTCGTTTTTCAAGAACCTGGCTAGTAGCTGTACCTTGGATTCAG
CCCTCAATGCTGCCTCTTACTATAACTTCACAGTCTTAAAGGTTCCAAGAAGCATGACTGATC
CACAGAATATGGAGTTCCAGGTTCCTGTAATACTTACCTCACAGGCTAATGCTCCTCTGTTGG
CTGGAAACACTTGTCAGAATGTAGTTTCTCAGGTCACCTATGAGATAGAGACCAATGGGACTT
TTGGAATCCAGAAAGTTTCTGTCAGTTTGGGACAAACCAACCTGACTGTTGAGCCAGGCGCTT
CCTTACAGCAACACTTCATCCTTCGCTTCAGGGCTTTTCAACAGAGCACAGCTGCTTCTCTCA
CCAGTCCTAGAAGTGGGAATCCTGGCTATATAGTTGGGAAGCCACTCTTGGCTCTGACTGATG
ATATAAGTTACTCAATGACCCTCTTACAGAGCCAGGGTAATGGAAGTTGCTCTGTTAAAAGAC
ATGAAGTGCAGTTTGGAGTGAATGCAATATCTGGATGCAAGCTCAGGTTGAAGAAGGCAGACT
GCAGCCACTTGCAGCAGGAGATTTATCAGACTCTTCATGGAAGGCCCAGACCAGAGTATGTTG
CCATCTTTGGTAATGCTGACCCAGCCCAGAAAGGAGGGTGGACCAGGATCCTCAACAGGCACT
GCAGCATTTCAGCTATAAACTGTACTTCCTGCTGTCTCATACCAGTTTCCCTGGAGATCCAGG
TATTGTGGGCATATGTAGGTCTCCTGTCCAACCCGCAAGCTCATGTATCAGGAGTTCGATTCC
TATACCAGTGCCAGTCTATACAGGATTCTCAGCAAGTTACAGAAGTATCTTTGACAACTCTTG
TGAACTTTGTGGACATTACCCAGAAGCCACAGCCTCCAAGGGGCCAACCCAAAATGGACTGGA
AATGGCCATTCGACTTCTTTCCCTTCAAAGTGGCATTCAGCAGAGGAGTATTCTCTCAAAAAT
GCTCAGTCTCTCCCATCCTTATCCTGTGCCTCTTACTACTTGGAGTTCTCAACCTAGAGACTA
TG**TGA**AGAAAAGAAAATAATCAGATTTCAGTTTTCCCTATGAGAAACTCTGAGGCAGCCACTT
ATCTTGGCTAAATAGAACCTCACCTGCTCATGACCAGAGAGCATTTAGGATAATAGATGACCT
AACTGAAGGAATCCTTGTATATGAAAGGAGTTATTTAGAAAAGCAATAAAAATATTTTATTC
ATCNTAAAAAAAAAA

# FIGURE 102

MRTPQLALLQVFFLVFPDGVRPQPSSSPSGAVPTSLELQRGTDGGTLQSPSEATATRPAVPGL
PTVVPTLVTPSAPGNRTVDLFPVLPICVCDLTPGACDINCCCDRDCYLLHPRTVFSFCLPGSV
RSSSWVCVDNSVIFRSNSPFPSRVFMDSNGIRQFCVHVNNSNLNYFQKLQKVNATNFQALAAE
FGGESFTSTFQTQSPPSFYRAGDPILTYFPKWSVISLLRQPAGVGAGGLCAESNPAGFLESKS
TTCTRFFKNLASSCTLDSALNAASYYNFTVLKVPRSMTDPQNMEFQVPVILTSQANAPLLAGN
TCQNVVSQVTYEIETNGTFGIQKVSVSLGQTNLTVEPGASLQQHFILRFRAFQQSTAASLTSP
RSGNPGYIVGKPLLALTDDISYSMTLLQSQGNGSCSVKRHEVQFGVNAISGCKLRLKKADCSH
LQQEIYQTLHGRPRPEYVAIFGNADPAQKGGWTRILNRHCSISAINCTSCCLIPVSLEIQVLW
AYVGLLSNPQAHVSGVRFLYQCQSIQDSQQVTEVSLTTLVNFVDITQKPQPPRGQPKMDWKWP
FDFFPFKVAFSRGVFSQKCSVSPILILCLLLLGVLNLETM

**Important features of the protein:**

**Signal peptide:**

amino acids 1-22

**Transmembrane domains:**

amino acids 484-505, 581-600

**N-glycosylation sites.**

amino acids 78-82, 165-169, 179-185, 279-285, 331-337, 347-351, 410-414, 487-491

**N-myristoylation sites.**

amino acids 30-36, 41-47, 124-130, 232-238, 236-242, 409-415

**Prokaryotic membrane lipoprotein lipid attachment site.**

amino acids 420-431

# FIGURE 103

CCTAATTCTCAAGGTGATGCTATTTAGGAAGTCATAACTCATGTGAGTGGAGCCATGTGGGAT
TAAGAAGTGATAGGAGAGCTTGCTGTCTGTCTCTGCTCTCCACTGTGTGAGGATACAACAGGA
AGACAGCCATCTGGTGAGGAAGAGAGGGCCCTCGCCAGATACCGGACCTGCTGACACCTTGAT
CTTGGACTTCCCATCTTCCAGGAAGGCCTGACCTCAGTTGTTCCAGGGTAAAGAATTTGGGCA
GTGCCCACACCCACGCTGTTGGATAACATTTCTTCACCATACCAGTGAGGGTGAATGTGTACA
CGCCCAGCTTCCTGCCTGTTACTCTCCACAGT**ATG**CGAAGAATATCCCTGACTTCTAGCCCTG
TGCGCCTTCTTTTGTTTCTGCTGTTGCTACTAATAGCCTTGGAGATCATGGTTGGTGGTCACT
CTCTTTGCTTCAACTTCACTATAAAATCATTGTCCAGACCTGGACAGCCCTGGTGTGAAGCGC
AGGTCTTCTTGAATAAAAATCTTTTCCTTCAGTACAACAGTGACAACAACATGGTCAAACCTC
TGGGCCTCCTGGGGAAGAAGGTATATGCCACCAGCACTTGGGGAGAATTGACCCAAACGCTGG
GAGAAGTGGGGCGAGACCTCAGGATGCTCCTTTGTGACATCAAACCCCAGATAAAGACCAGTG
ATCCTTCCACTCTGCAAGTCGAGATGTTTTGTCAACGTGAAGCAGAACGGTGCACTGGTGCAT
CCTGGCAGTTCGCCACCAATGGAGAGAAATCCCTCCTCTTTGACGCAATGAACATGACCTGGA
CAGTAATTAATCATGAAGCCAGTAAGATCAAGGAGACATGGAAGAAAGACAGAGGGCTGGAAA
AGTATTTCAGGAAGCTCTCAAAGGGAGACTGCGATCACTGGCTCAGGGAATTCTTAGGGCACT
GGGAGGCAATGCCAGAACCGACAGGCAGAAGATCCACC**TAG**AGGTGATACCACGGCGGCGCAG
AGTTGTTCACCTGTGGTCCTCGATCGCTGACAGCCTTGGCTCCCACTGCTGTGTGTTCCCTGA
GTCAAGTGGAGGCGGAGCCTGCAATGAGCGGAGATCGCGCCTCTGCATTCCAGTCTTGGCAAC
AGAGCAAGACTCCGTCTCAAAAAAAAAAAATTTTTTTCAGTACATATTTTTTAAAAGATAGG
GCTGGGCACAGCAGCTCACATCTATAATCCCAACACTTTGGGAGGCCTAGGCAGGAGGATCAC
TTGAGCCCAGGAATCTGAAGCTGCAGTGAGCCTTTGCTCGTGAGATTGTGGACCTATGATCCT
ACCACCAGCCCACCTGGTTCTAACACCCCCTCCTCTATGTGTGAGAGGGAGAGAAGAAAAGTG
AGGGAGAAAAGAGAGATAAGCAAAGAACAGAGAGGAAAAATGGAAAATAAGAGGAAATTGGGG
GAATTAAACAGAGGGGAGGGCATGGATCCCCGGGAGTTAGAAGAGTAGCAGCTTGTGGATTAC
TACGCAGTGGAGGAAGAAGAGTTGTTGGAAATTATTTGAGAGGTAGTATAATCATTTGTGAGG
CAGTTTTCTGCATTCACCATTTCTCACAGACTAAGTTACTCATAAGCAAACGTGCAATTCACA
TTACACTGAAATTCTTCCCTAATACATCATTTGCATTGGAATAAAGTACGGTTTTCAAACAAC
CTGATATAGCAGAACTGACTGTATAAATTATGTGAGCACAGTGCAAGTAATTCTTTGTTTGTT
TGTTTGTTTTTTGAGACAGAGTCTCACTCTATCTCCCAGGCTGGAGTGTAGTGGTGCGATCC
CGGCTCACTGCAACCTCGATCTCCCAGGCTCAAGCGATTCCCCTGCCTCAGCCTCCTGAGTAG
CTGGGATTACAGGCATGAGCCACCACGCCCGGCTAATTTTTGTATTTTTAGTAGAGACGGGGT
TTCACCCTGTTGGCCAGGCTGGTCTCGAACTACGGACCTCAGGTGATCTGCCCCCCTCAGCCT
CTCAAAGTGCTGGGATTATAGCATGAGCCACTGAGCCCAGACACAAGTAGTTCTTTCTGATAA
ACACTTTAACACTGAATGCA

# FIGURE 104

MRRISLTSSPVRLLLFLLLLLIALEIMVGGHSLCFNFTIKSLSRPGQPWCEAQVFLNKNLFLQ
YNSDNNMVKPLGLLGKKVYATSTWGELTQTLGEVGRDLRMLLCDIKPQIKTSDPSTLQVEMFC
QREAERCTGASWQFATNGEKSLLFDAMNMTWTVINHEASKIKETWKKDRGLEKYFRKLSKGDC
DHWLREFLGHWEAMPEPTGRRST

**Important features of the protein:**

**Signal peptide:**

amino acids 1-23

**Transmembrane domain:**

amino acids 11-30(possible type II protein)

**N-glycosylation site.**

amino acids 36-39, 154-157

**cAMP- and cGMP-dependent protein kinase phosphorylation site.**

amino acids 2-5, 182-185, 209-212

**Casein kinase II phosphorylation site.**

amino acids 86-89, 93-96, 142-145, 185-188

**N-myristoylation site.**

amino acids 46-51

**Amidation site.**

amino acids 77-80, 207-210

# FIGURE 105

TTTTCCGAGTGACCTTCTTG**ATG**CTGGCTGTTTCTCTCACCGTTCCCCTGCTTGGAGCCATGA
TGCTGCTGGAATCTCCTATAGATCCACAGCCTCTCAGCTTCAAAGAACCCCCGCTCTTGCTTG
GTGTTCTGCATCCAAATACGAAGCTGCGACAGGCAGAAAGGCTGTTTGAAAATCAACTTGTTG
GACCGGAGTCCATAGCACATATTGGGGATGTGATGTTTACTGGGACAGCAGATGGCCGGGTCG
TAAAACTTGAAAATGGTGAAATAGAGACCATTGCCCGGTTTGGTTCGGGCCCTTGCAAAACCC
GAGATGATGAGCCTGTGTGTGGGAGACCCCTGGGTATCCGTGCAGGGCCCAATGGGACTCTCT
TTGTGGCCGATGCATACAAGGGACTATTTGAAGTAAATCCCTGGAAACGTGAAGTGAAACTGC
TGCTGTCCTCCGAGACACCCATTGAGGGGAAGAACATGTCCTTTGTGAATGATCTTACAGTCA
CTCAGGATGGGAGGAAGATTTATTTCACCGATTCTAGCAGCAAATGGCAAAGACGAGACTACC
TGCTTCTGGTGATGGAGGGCACAGATGACGGGCGCCTGCTGGAGTATGATACTGTGACCAGGG
AAGTAAAAGTTTTATTGGACCAGCTGCGGTTCCCGAATGGAGTCCAGCTGTCTCCTGCAGAAG
ACTTTGTCCTGGTGGCAGAAACAACCATGGCCAGGATACGAAGAGTCTACGTTTCTGGCCTGA
TGAAGGGCGGGGCTGATCTGTTTGTGGAGAACATGCCTGGATTTCCAGACAACATCCGGCCCA
GCAGCTCTGGGGGGTACTGGGTGGGCATGTCGACCATCCGCCCTAACCCTGGGTTTTCCATGC
TGGATTTCTTATCTGAGAGACCCTGGATTAAAAGGATGATTTTTAAGCTCTTTAGTCAAGAGA
CGGTGATGAAGTTTGTGCCGCGGTACAGCCTCGTCCTAGAACTCAGCGACAGCGGTGCCTTCC
GGAGAAGCCTGCATGATCCCGATGGGCTGGTGGCCACCTACATCAGCGAGGTGCACGAACACG
ATGGGCACCTGTACCTGGGCTCTTTCAGGTCCCCCTTCCTCTGCAGACTCAGCCTCCAGGCTG
TT**TAG**CCCTCCCAGATAGCTGCCCCTGCCACGCAGGCCAGGAGTCTTCACACTCAGGCACCAG
GCCTGGTCCAGGAGGAGCTGTGGACACAGTCGTGGTTCAAGTGTCCACATGCACCTGTTAGTC
CCTGAGAGGTGGTGGGAATGGCTGCTTCATTCCTCGAGGATGCCCGGCCCCACCTGGGCTTG
TCTTTCTGTTTAGAGGGAAGTGTAACATATCTGCCATGAGGAACATAAATTCATGTAAAGCCA
TTTTCTCTTAAACAAAACAAAACTTTCTAAGTACAATCATTCTCTAGGATTTGGGAAGCTCCT
TGCACTTGGAACAGGGCTCAGGTGGGTGGAGCAGTAAGGCACTACCCAGAGAGCTTGCTGCTG
CGGCCCTGTCCTGCGGCCTCAAAGTTCTTCTTTACTATATATAACGTGCGGTCATACCTTTCT
TCGTTGTGGTGGGGATGGAAGAGCAGAGGGAGCATGGCCCAGGGGTGTTGAGGCCAGCGGTGA
GAGCCGTGTTAGCCAAGACATGGAACTGTGTTCTCAAGGGTTATGTGGGGCGTGGGCTCTCCA
TAGTGTGTATGAAAAGCTTGTTGACTCTAGCGGCTCAGAGAGGACTTTGCTGGGTTTCTTTCT
GTGAATATCTCCGTGCTGACCATGCTGGAATTGGATGATTCTGCAATTCGGGACCTACTGCAG
GGGTCCGTTTAGTAACGTCTTGTCTGTGATCTTTGTTCTTGACCTCTAGACCCCAAGATGTGA
ACAGTGCACGTGTTAATGTCATCTTTGCTCATGTGTTATAAGCCCCAAGTTGCTGTATATTTT
CACAAGTATGTCTACACACTGG

# FIGURE 106

MLAVSLTVPLLGAMMLLESPIDPQPLSFKEPPLLLGVLHPNTKLRQAERLFENQLVGPESIAH
IGDVMFTGTADGRVVKLENGEIETIARFGSGPCKTRDDEPVCGRPLGIRAGPNGTLFVADAYK
GLFEVNPWKREVKLLLSSETPIEGKNMSFVNDLTVTQDGRKIYFTDSSSKWQRRDYLLLVMEG
TDDGRLLEYDTVTREVKVLLDQLRFPNGVQLSPAEDFVLVAETTMARIRRVYVSGLMKGGADL
FVENMPGFPDNIRPSSSGGYWVGMSTIRPNPGFSMLDFLSERPWIKRMIFKLFSQETVMKFVP
RYSLVLELSDSGAFRRSLHDPDGLVATYISEVHEHDGHLYLGSFRSPFLCRLSLQAV

**Important features of the protein:**

**Signal peptide:**

amino acids 1-13

**Transmembrane domain:**

amino acids 1-21 (possible type II)

**N-glycosylation sites.**

amino acids 116-119, 152-155

**Casein kinase II phosphorylation sites.**

amino acids 19-22, 27-30, 98-101, 146-149, 221-224, 286-289, 332-335

**N-myristoylation sites.**

amino acids 71-76, 92-97, 189-194, 244-249, 338-343

**Amidation site.**

amino acids 164-167

# FIGURE 107

AACGAAGCGTGCGCGCTTTGGTAACCGGCTAGAAATCCCGCACGCGCGCCTGCCTCCTCTCCC
CAGGCCTGAGCTGCCCCTCCCACTGCCTTTCCTTCTTCCCGCGAGTCAGAAGCTTCGCGAGGG
CCCAGAGAGGCGGTGGGGTGGGCGACCCTACGCCAGCTCCGGGCGGGAGAAAGCCCACCCTCT
CCCGCGCCCCAGGAAACCGCCGGCGTTCGGCGCTGCGCAGAGCC**ATG**GAATTCTCCTGGCTGG
AGACGCGCTGGGCGCGGCCCTTTTACCTGGCGTTCGTGTTCTGCCTGGCCCTGGGGCTGCTGC
AGGCCATTAAGCTGTACCTGCGGAGGCAGCGGCTGCTGCGGGACCTGCGCCCCTTCCCAGCGC
CCCCCACCCACTGGTTCCTTGGGCACCAGAAGTTTATTCAGGATGATAACATGGAGAAGCTTG
AGGAAATTATTGAAAAATACCCTCGTGCCTTCCCTTTCTGGATTGGGCCCTTTCAGGCATTTT
TCTGTATCTATGACCCAGACTATGCAAAGACACTTCTGAGCAGAACAGATCCCAAGTCCCAGT
ACCTGCAGAAATTCTCACCTCCACTTCTTGGAAAAGGACTAGCGGCTCTAGACGGACCCAAGT
GGTTCCAGCATCGTCGCCTACTAACTCCTGGATTCCATTTTAACATCCTGAAAGCATACATTG
AGGTGATGGCTCATTCTGTGAAAATGATGCTGGATAAGTGGGAGAAGATTTGCAGCACTCAGG
ACACAAGCGTGGAGGTCTATGAGCACATCAACTCGATGTCTCTGGATATAATCATGAAATGCG
CTTTCAGCAAGGAGACCAACTGCCAGACAAACAGCACCCATGATCCTTATGCAAAAGCCATAT
TTGAACTCAGCAAAATCATATTTCACCGCTTGTACAGTTTGTTGTATCACAGTGACATAATTT
TCAAACTCAGCCCTCAGGGCTACCGCTTCCAGAAGTTAAGCCGAGTGTTGAATCAGTACACAG
ATACAATAATCCAGGAAAGAAAGAAATCCCTCCAGGCTGGGGTAAAGCAGGATAACACTCCGA
AGAGGAAGTACCAGGATTTTCTGGATATTGTCCTTTCTGCCAAGGATGAAAGTGGTAGCAGCT
TCTCAGATATTGATGTACACTCTGAAGTGAGCACATTCCTGTTGGCAGGACATGACACCTTGG
CAGCAAGCATCTCCTGGATCCTTTACTGCCTGGCTCTGAACCCTGAGCATCAAGAGAGATGCC
GGGAGGAGGTCAGGGGCATCCTGGGGGATGGGTCTTCTATCACTTGGGACCAGCTGGGTGAGA
TGTCGTACACCACAATGTGCATCAAGGAGACGTGCCGATTGATTCCTGCAGTCCCGTCCATTT
CCAGAGATCTCAGCAAGCCACTTACCTTCCCAGATGGATGCACATTGCCTGCAGGGATCACCG
TGGTTCTTAGTATTTGGGGTCTTCACCACAACCCTGCTGTCTGGAAAAACCCAAAGGTCTTTG
ACCCCTTGAGGTTCTCTCAGGAGAATTCTGATCAGAGACACCCCTATGCCTACTTACCATTCT
CAGCTGGATCAAGGAACTGCATTGGGCAGGAGTTTGCCATGATTGAGTTAAAGGTAACCATTG
CCTTGATTCTGCTCCACTTCAGAGTGACTCCAGACCCCACCAGGCCTCTTACTTTCCCCAACC
ATTTTATCCTCAAGCCCAAGAATGGGATGTATTTGCACCTGAAGAAACTCTCTGAATGT**TAG**A
TCTCAGGGTACAATGATTAAACGTACTTTGTTTTTCGAAGTTAAATTTACAGCTAATGATCCA
AGCAGATAGAAAGGGATCAATGTATGGTGGGAGGATTGGAGGTTGGTGGGATAGGGGTCTCTG
TGAAGAGATCCAAAATCATTTCTAGGTACACAGTGTGTCAGCTAGATCTGTTTCTATATAACT
TTGGGAGATTTTCAGATCTTTTCTGTTAAACTTTCACTACTATTAATGCTGTATACACCAATA
GACTTTCATATATTTTCTGTTGTTTTTAAAATAGTTTTCAGAATTATGCAAGTAATAAGTGCA
TGTATGCTCACTGTCAAAAATTCCCAACACTAGAAAATCATGTAGAATAAAAATTTTAAATCT
CACTTCACTTAGCCGACATTCCATGCCCTGACCAATCCTACTGCTTTTCCTAAAAACAGAATA
ATTTGGTGTGCATTCTTTCAGACTTTTTCCTATACATTTTATATGTAGAAATGTAGCAATGTA
TTTGTATAGATGTGATCATTCCTATATTGTTATTGATTTTTTTCACTTAATAAAAATTCACCT
TATTCCTTAAAA

# FIGURE 108

MEFSWLETRWARPFYLAFVFCLALGLLQAIKLYLRRQRLLRDLRPFPAPPTHWFLGHQKFIQD
DNMEKLEEIIEKYPRAFPFWIGPFQAFFCIYDPDYAKTLLSRTDPKSQYLQKFSPPLLGKGLA
ALDGPKWFQHRRLLTPGFHFNILKAYIEVMAHSVKMMLDKWEKICSTQDTSVEVYEHINSMSL
DIIMKCAFSKETNCQTNSTHDPYAKAIFELSKIIFHRLYSLLYHSDIIFKLSPQGYRFQKLSR
VLNQYTDTIIQERKKSLQAGVKQDNTPKRKYQDFLDIVLSAKDESGSSFSDIDVHSEVSTFLL
AGHDTLAASISWILYCLALNPEHQERCREEVRGILGDGSSITWDQLGEMSYTTMCIKETCRLI
PAVPSISRDLSKPLTFPDGCTLPAGITVVLSIWGLHHNPAVWKNPKVFDPLRFSQENSDQRHP
YAYLPFSAGSRNCIGQEFAMIELKVTIALILLHFRVTPDPTRPLTFPNHFILKPKNGMYLHLK
KLSEC

**Important features of the protein:**

**Signal peptide:**

amino acids 1-29

**Transmembrane domains:**

amino acids 310-330, 397-413, 459-473

**N-glycosylation site.**

amino acids 206-210

**cAMP- and cGMP-dependent protein kinase phosphorylation site.**

amino acids 265-269, 504-520

**N-myristoylation sites.**

amino acids 25-31, 298-304, 353-359, 450-456, 456-462

**Cytochrome P450 cysteine heme-iron ligand signature.**

amino acids 447-457

**Cytochrome P450 cysteine heme-iron ligand proteins.**

amino acids 444-475

# FIGURE 109

GGCGTTCCGGGCCTCAACTTTGGCGTCGTGAGATTCTTGTGAGGCGTCTGCCTGGAAGCCGGC
AGCAATTTTGCTTCTTTAAAGAGAAAAAGAAGGCTAGGGACTCAGATTCCTGGATTCTGAGAT
CCAGACCAGCTCCTCCCAGACCTCTCCAGAAGAAGCC**ATG**GGAACCCCTCGTATCCAGCATTT
GCTGATCCTCCTGGTCCTAGGAGCCTCCCTCCTGACCTCGGGCCTAGAGCTGTATTGTCAAAA
GGGTCTGTCCATGACTGTGGAAGCAGATCCAGCCAATATGTTTAACTGGACCACAGAGGAAGT
GGAGACTTGTGACAAAGGGGCACTTTGCCAGGAAACCATACTAATAATTAAAGCAGGGACTGA
GACAGCCATTTTGGCCACGAAGGGCTGCATCCCGGAAGGGGAGGAGGCCATAACAATTGTCCA
GCACTCTTCACCTCCCGGCCTGATCGTGACCTCCTACAGTAACTACTGTGAGGATTCCTTCTG
TAATGACAAAGACAGCCTGTCTCAGTTTTGGGAGTTCAGTGAGACCACAGCTTCCACTGTGTC
AACAACCCTCCATTGTCCAACCTGTGTGGCTTTGGGGACCTGTTTCAGTGCTCCTTCTCTTCC
CTGTCCCAATGGTACAACTCGATGCTATCAAGGAAAACTTGAGATCACTGGAGGTGGCATTGA
GTCGTCTGTGGAGGTCAAAGGCTGTACAGCCATGATTGGCTGCAGGCTGATGTCTGGAATCTT
AGCAGTAGGACCCATGTTTGTGAGGGAAGCGTGCCCACATCAGCTGCTCACTCAACCTCGAAA
GACTGAAAATGGGGCCACCTGTCTTCCCATTCCTGTTTGGGGGTTACAGCTACTGCTGCCATT
GCTGCTGCCATCATTTATTCACTTTTCC**TAA**GAAGGCACTTCTGGGCCTGGGTCTGAGGACAT
CTTTTTTGACTGGGAGCCTTCTTACTGTTGAGGTTCAACAAGCTGAGGAGTAGATGGGAATTT
GAGGGAGAATACAGAGATACTATGAACGTATTTGACATTTTTAATACAATTTCTGCTATAATT
TTTGTATGCAGTAGGCGTTACTAATAAACATTTCTGCTGTGA

# FIGURE 110

MGTPRIQHLLILLVLGASLLTSGLELYCQKGLSMTVEADPANMFNWTTEEVETCDKGALCQET
ILIIKAGTETAILATKGCIPEGEEAITIVQHSSPPGLIVTSYSNYCEDSFCNDKDSLSQFWEF
SETTASTVSTTLHCPTCVALGTCFSAPSLPCPNGTTRCYQGKLEITGGGIESSVEVKGCTAMI
GCRLMSGILAVGPMFVREACPHQLLTQPRKTENGATCLPIPVWGLQLLLPLLLPSFIHFS

**Important features of the protein:**

**Signal peptide:**

amino acids 1-23

**Transmembrane domain:**

amino acids 184-201

**N-glycosylation sites.**

amino acids 45-49, 159-163

**N-myristoylation sites.**

amino acids 31-37, 70-76, 99-105, 147-153, 160-166, 174-180, 175-181

# FIGURE 111

CGAGAAGAGGACAGAGGAGACTGAGCAAAGGGGGGTGGGCTCCAGGCGACCCCTAGCCCAATTCTGCCCCTCCAT
CCCAAGGGGCAGAGAAATTGTCTTTCTTTGCTGACTCCTACGAGGAAAAAAAAAAAAAAAAAAAAAAACCATTTAA
AGGGAAAGATAAACGGAGACGGAGGAAAGGTGGCAGCCAGATTACTTAGAGAGGCACAGAGGAGAGAGATCGGGG
TGAGTCGCC**ATG**GGGACTCCCAGGGCCCAGCACCCGCCGCCTCCCCAGCTGCTGTTCCTAATTCTGCTGAGCTGT
CCCTGGATCCAGGGTCTGCCCCTGAAGGAGGAGGAGATATTGCCAGAGCCTGGAAGTGAGACCCCCACGGTGGCC
TCTGAGGCCCTGGCTGAACTGCTTCATGGGGCCCTGCTGAGGAGGGGCCCAGAGATGGGCTACCTGCCAGGATCT
GATCCGGACCCCACGCTAGCCACCCCTCCGGCCGGCCAGACTCTCGCAGTGCCCTCCCTGCCACGGGCCACTGAG
CCGGGGACAGGGCCTCTGACAACAGCCGTCACCCCTAACGGGGTCAGGGGGGCAGGCCCCACTGCGCCAGAACTG
CTGACCCCGCCCCCAGGAACCACAGCCCCACCCCCACCCAGCCCTGCCTCCCCAGGGCCTCCCCTTGGCCTGAG
GGAGGAGAGGAGGAGACGACGACCACCATCATCACCACGACAACTGTTACCACTACGGTGACCAGCCCAGTTCTG
TGTAATAACAACATCTCCGAGGGCGAAGGGTATGTGGAGTCTCCAGATCTGGGGAGCCCCGTCAGCCGCACCCTG
GGGCTCCTGGACTGCACTTACAGCATCCATGTCTACCCTGGCTACGGCATTGAGATCCAGGTGCAGACGCTGAAC
CTGTCACAGGAAGAGGAGCTCCTGGTGCTGGCTGGTGGGGATCCCCAGGCCTGGCCCCCCGACTCCTGGCCAAC
TCATCCATGCTTGGAGAAGGACAAGTCCTTCGGAGCCCAACCAACCGGCTGCTTCTGCACTTCCAGAGCCCACGG
GTCCCAAGGGGCGGTGGCTTCAGGATCCACTATCAGGCCTACCTCCTGAGCTGTGGCTTCCCTCCCCGGCCGGCC
CATGGGGACGTGAGTGTGACGGACCTGCACCCTGGGGGCACTGCCACCTTTCACTGTGATTCGGGCTACCAGCTG
CAGGGAGAGGAGACCCTCATCTGCCTCAATGGCACCCGGCCATCCTGGAACGGTGAAACCCCCAGCTGCATGGCA
TCCTGTGGTGGCACCATCCACAATGCCACCCTGGGCCGCATCGTGTCCCCAGAGCCTGGGGGAGCCGTAGGGCCC
AACCTCACCTGCCGTTGGGTCATTGAAGCAGCTGAGGGGCGCCGGCTGCACCTGCACTTTGAAAGGGTCTCGCTG
GATGAGGACAATGACCGGCTGATGGTGCGCTCAGGGGGCAGCCCCCTATCCCCCGTGATCTATGATTCGGACATG
GACGATGTCCCCGAGCGGGGTCTCATCAGTGACGCCCAGTCCCTCTACGTGGAGCTGCTGTCAGAGACACCTGCC
AATCCCCTGCTGTTAAGCCTTCGATTTGAAGCCTTTGAGGAGGATCGCTGCTTCGCCCCCTTCCTGGCACATGGA
AATGTCACTACCACGGACCCTGAGTATCGCCCAGGGGCACTGGCAACCTTCTCGTGCCTCCCAGGATATGCCCTG
GAGCCCCCTGGGCCCCCCAATGCCATCGAATGTGTGGATCCCACAGAACCCCACTGGAACGACACAGAGCCGGCC
TGCAAAGCCATGTGTGGAGGGGAGCTGTCGGAACCAGCTGGCGTGGTCCTCTCTCCCGACTGGCCCCAGAGCTAT
AGCCCGGGCCAAGACTGCGTGTGGGGCGTGCACGTCCAGGAAGAGAAGCGCATCTTGCTCCAAGTTGAGATATTG
AATGTGCGGGAAGGGGACATGCTGACGCTGTTCGACGGGGACGGTCCCAGCGCCCGAGTCTTGGCCCAGCTGCGG
GGACCTCAGCCGCGCCGCCGCCTTCTCTCCTCTGGGCCCGACCTCACACTGCAGTTTCAGGCACCGCCCGGGCCC
CCAAATCCAGGCCTGGGCCAGGGCTTCGTATTGCACTTCAAAGAGGTCCCGAGGAACGACACGTGCCCCGAGCTG
CCACCTCCGGAGTGGGGCTGGAGAACGGCATCCCACGGGGACCTGATCCGGGGCACGGTGCTCACCTACCAGTGC
GAGCCTGGCTACGAGCTGCTAGGCTCCGACATTCTCACTTGCCAGTGGGACCTGTCTTGGAGCGCCGCGCCGCCC
GCCTGCCAAAAGATCATGACTTGTGCTGACCCTGGCGAGATTGCCAACGGGCACCGCACCGCCTCGGACGCCGGC
TTCCCCGTTGGCTCCCACGTCCAGTACCGCTGCCTGCCAGGGTACAGCCTCGAGGGGGCAGCCATGCTCACCTGC
TACAGCCGGGACACAGGCACACCCAAGTGGAGCGATAGGGTCCCCAAATGCGCCTTGAAGTACGAGCCGTGCCTG
AACCCGGGGGTTCCCGAGAATGGCTACCAGACGCTGTACAAGCACCACTACCAGGCGGGCGAGTCTCTGCGCTTC
TTCTGCTATGAGGGCTTTGAGCTTATCGGCGAGGTCACCATCACCTGTGTGCCCGGCCACCCCTCCCAGTGGACC
AGCCAGCCCCCACTCTGCAAAGTGACCCAGACCACAGATCCATCACGGCAGCTGGAAGGGGGGAACCTGGCCCTG
GCCATCCTGCTGCCTCTAGGCTTGGTCATTGTCCTCGGCAGTGGCGTTTACATCTACTACACCAAGCTTCAGGGA
AAGTCCCTTTTCGGCTTCTCGGGCTCCCACTCCTACAGCCCCATCACCGTGGAGTCGGACTTCAGCAACCCGCTG
TATGAAGCTGGGGATACGCGGGAGTATGAAGTTTCCATC**TGA**ACCCCAAGACTACAGCTGCAGGACCCAGGACGC
CCCTCCCCTCCTCATTCGGGCAGAGGGAAATACGGACCCGGTCTCTGCCTCCTGGCTGCCCTCCTCCCTGGCTG
TGTAAATAGTCTCCCTATCCCACGAGGGGGCTTTGATGGCCCTGGAGATCCTACAGTAAATAAACCAGCATCCTG
CCGCCCAAAAAA

# FIGURE 112

MGTPRAQHPPPPQLLFLILLSCPWIQGLPLKEEEILPEPGSETPTVASEALAELLHGALLRRG
PEMGYLPGSDPDPTLATPPAGQTLAVPSLPRATEPGTGPLTTAVTPNGVRGAGPTAPELLTPP
PGTTAPPPPSPASPGPPLGPEGGEEETTTTIITTTTVTTTVTSPVLCNNNISEGEGYVESPDL
GSPVSRTLGLLDCTYSIHVYPGYGIEIQVQTLNLSQEEELLVLAGGGSPGLAPRLLANSSMLG
EGQVLRSPTNRLLLHFQSPRVPRGGGFRIHYQAYLLSCGFPPRPAHGDVSVTDLHPGGTATFH
CDSGYQLQGEETLICLNGTRPSWNGETPSCMASCGGTIHNATLGRIVSPEPGGAVGPNLTCRW
VIEAAEGRRLHLHFERVSLDEDNDRLMVRSGGSPLSPVIYDSDMDDVPERGLISDAQSLYVEL
LSETPANPLLLSLRFEAFEEDRCFAPFLAHGNVTTTDPEYRPGALATFSCLPGYALEPPGPPN
AIECVDPTEPHWNDTEPACKAMCGGELSEPAGVVLSPDWPQSYSPGQDCVWGVHVQEEKRILL
QVEILNVREGDMLTLFDGDGPSARVLAQLRGPQPRRRLLSSGPDLTLQFQAPPGPPNPGLGQG
FVLHFKEVPRNDTCPELPPPEWGWRTASHGDLIRGTVLTYQCEPGYELLGSDILTCQWDLSWS
AAPPACQKIMTCADPGEIANGHRTASDAGFPVGSHVQYRCLPGYSLEGAAMLTCYSRDTGTPK
WSDRVPKCALKYEPCLNPGVPENGYQTLYKHHYQAGESLRFFCYEGFELIGEVTITCVPGHPS
QWTSQPPLCKVTQTTDPSRQLEGGNLALAILLPLGLVIVLGSGVYIYYTKLQGKSLFGFSGSH
SYSPITVESDFSNPLYEAGDTREYEVSI

**Important features of the protein:**

**Signal peptide:**

amino acids 1-27

**Transmembrane domain:**

amino acids 842-864

**N-glycosylation sites.**

amino acids 176-180, 222-226, 247-251, 332-336, 355-359, 373-377, 473-477, 517-521, 641-645

**Tyrosine kinase phosphorylation site.**

amino acids 61-69

**N-myristoylation sites.**

amino acids 2-8, 84-90, 111-117, 114-120, 190-196, 198-204, 235-241, 309-315, 333-339, 351-357, 472-478, 484-490, 528-534, 626-632, 665-671, 775-781, 842-848

**Amidation site.**

amino acids 384-388

**Prokaryotic membrane lipoprotein lipid attachment site.**

amino acids 12-23

**CUB domain proteins profile.**

amino acids 202-218, 376-392, 553-569

# FIGURE 113

GCCGCGGGCGGAGCTGCCTGCCGGTCCCGCGCCGCGCGTCCGCACTCCTCGGCCCTCGGGCGGTCGATGGGACGG
GGCGCCGCGGAGCAGGAGGCGGCGCCCGTCGGGGTGCTCGGGCCGCGCGGGAGCCCACTGTGGGGCTCGGGCATG
GCGGGCCGCAGGACCTGAGCTCTCCTCAGGGGAGCGGGGAGGCAGCTGCTGGCCGGCGATGGGGACGGAGTGGGG
CCGTCGCCGCCGCGCCGAGCCGTGAGCGCCGAGCCACCGCCGCCGCTACCTCAGCCCTTCGCGAAGCGCCGGGCA
GCTCGGGAAC**ATG**GCCCTGGAGCGGCTCTGCTCGGTCCTCAAAGTGTTGTTAATAACAGTACTGGTAGTGGAAGG
GATTGCCGTGGCCCAAAAAACCCAAGATGGACAAAATATTGGAATCAAGCATATTCCTGCAACCCAGTGTGGCAT
TTGGGTTCGAACCAGCAATGGAGGTCATTTTGCTTCGCCAAATTATCCTGACTCATATCCACCAAACAAGGAGTG
TATCTACATTTTGGAAGCTGCTCCACGTCAAAGAATAGAGTTGACCTTTGATGAACATTATTATATAGAACCATC
ATTTGAGTGTCGGTTTGATCACTTGGAAGTTCGAGATGGGCCATTTGGTTTCTCTCCTCTTATAGATCGTTACTG
TGGCGTGAAAAGCCCTCCATTAATTAGATCAACAGGGAGATTCATGTGGATTAAGTTTAGTTCTGATGAAGAGCT
TGAAGGACTGGGATTTCGAGCAAAATATTCATTTATTCCAGATCCAGACTTTACTTACCTAGGAGGTATTTTAAA
TCCCATTCCAGATTGTCAGTTCGAGCTCTCGGGAGCTGATGGAATAGTGCGCTCTAGTCAGGTAGAACAAGAGGA
GAAAACAAAACCAGGCCAAGCCGTTGATTGCATCTGGACCATTAAAGCCACTCCAAAAGCTAAGATTTATTTGAG
GTTCCTAGATTATCAAATGGAGCACTCAAATGAATGCAAGAGAAACTTCGTTGCAGTCTATGATGGAAGCAGTTC
TATTGAAAACCTGAAGGCCAAGTTTTGCAGCACTGTGGCCAATGATGTAATGCTTAAAACAGGAATTGGAGTGAT
TCGAATGTGGGCAGATGAAGGTAGTCGGCTTAGCAGGTTTCGAATGCTCTTTACTTCCTTTGTGGAGCCTCCCTG
CACAAGCAGCACTTTCTTTTGCCATAGCAACATGTGCATCAATAATTCTTTAGTCTGTAATGGTGTCCAAAATTG
TGCATACCCTTGGGATGAAAATCATTGTAAAGAAAAGAAAAAAGCAGGAGTATTTGAACAAATCACTAAGACTCA
TGGAACAATTATTGGCATTACTTCAGGGATTGTCTTGGTCCTTCTCATTATTTCTATTTTAGTACAAGTGAAACA
GCCTCGAAAAAAGGTCATGGCTTGCAAAACCGCTTTTAATAAAACCGGGTTCCAAGAAGTGTTTGATCCTCCTCA
TTATGAACTGTTTTCACTAAGGGACAAAGAGATTTCTGCAGACCTGGCAGACTTGTCGGAAGAATTGGACAACTA
CCAGAAGATGCGGCGCTCCTCCACCGCCTCCCGCTGCATCCACGACCACCACTGTGGGTCGCAGGCCTCCAGCGT
CAAACAAAGCAGGACCAACCTCAGTTCCATGGAACTTCCTTTCCGAAATGACTTTGCACAACCACAGCCAATGAA
AACATTTAATAGCACCTTCAAGAAAAGTAGTTACACTTTCAAACAGGGACATGAGTGCCCTGAGCAGGCCCTGGA
AGACCGAGTAATGGAGGAGATTCCCTGTGAAATTTATGTCAGGGGGCGAGAAGATTCTGCACAAGCATCCATATC
CATTGACTTC**TAA**TCTTCTGCTAATGGTGATGTGAATTCTTAGGGTGTGTACGTACGCAGCCTCCAGGGCACCAT
ACTGTTTCCAGCAGCCAACCCTTTTCTCCCATCACAACTACGAAGACCTTGATTTACCGTTAACCTATTGTATGG
TGATGTTTTTATTCTCTCAGGCAGTCTATATATGTTAAACCAATCAAGGAACTTACTCTATTCAGTGGAAACAAT
AATCATCTCTATTGCTTGGTGTCATTTATAGGAAGCACTGCCAGTTAAAGAGCATTAGAAGAGGTGGTTGGATGG
AGCCAGGCTCAGGCTGCCTCTTCGTTTTAGCAACAAGAAGACTGCTCTTGACTGATAACAGCTCTGTCAATATTT
TGATGCCACAATAAACTTGATTTTTTTTTACATTCCTTTTATTTTTCCTTTCTCTAAATTTAATTTGTTTTATAA
GCCTATCGTTTTACCATTTCATTTTCTTACATAAGTACAAGTGGTTAATGTACCACATACTTCAGTATAGGCATT
TGTTCTTGAGTGTGTCAAAATACAGCTAGTTACTGTGCCAATTAAGACCCAGTTGTATTTCACCCATCTGTTTCT
TCTTGGCTAATCTCTGTACTTCTGCCTTTTAATTACTGGGCCCTTATTCCTTATTTCTGTGAGAAATAATAGAT
GATATGATTTATTACCTTTCAATTATATTTTTCTCAGTTATACTAGAAAATTTCATAATCCTGGGATATATGTAC
CATTGTCAGCTATGACTAAAAATTTGAAAAAGATAAAAATTTCTAGCAAGCCTTTGAAGTTTACCAAGTATAGTC
ACATTCAGTGACAGCCCATTCATTCCAGTAAAGAATCATTTCATTCACTTTGGGAGAGGCCTATAATTACATTTA
TTTGCAATGTTTCTCTTCGCTAGATTGTTACATAGCTCCCATTCTGTTGGTTTTGCTTACAGCATATGGTAACCA
AGGTTAGATGCCAGTTAAAATTCCTTAGAAATTGGATGAGCCTTGAGATTGCTTCTTAACTGGGACATGACATTT
TTCTAGCTCTTATCAAGAATAACAACTTCCACTTTTTTTAAACTGCACTTTTGACTTTTTTTATGGTATAAAAA
CAATAATTTATAAACATAAAAGCTCATTGTGTTTTTTAGACTTTTGATATTATTTGATACTGTACAAACTTTATT
AAATCAAGATGAAAGACCTACAGGACAGATTCCTTTCAGTGTTCACATCAGTGGCTTTGTATGCAAATATGCTGT
GTTGGACCTGGACGCTATAACTTATTGTAAAGACCTTGGAAATGTGGACATAAGCTCTTTCTTTCCTTTTGTTAC
TGTATTTAGTTTGTGATAAATTTTTCACTGTGTGATATTTATGCTCTAAATCACTACACAAATCCCATATTAAAA
TATACATTGTACCTGAAAAAAAA

# FIGURE 114

MALERLCSVLKVLLITVLVVEGIAVAQKTQDGQNIGIKHIPATQCGIWVRTSNGGHFASPNYP
DSYPPNKECIYILEAAPRQRIELTFDEHYYIEPSFECRFDHLEVRDGPFGFSPLIDRYCGVKS
PPLIRSTGRFMWIKFSSDEELEGLGFRAKYSFIPDPDFTYLGGILNPIPDCQFELSGADGIVR
SSQVEQEEKTKPGQAVDCIWTIKATPKAKIYLRFLDYQMEHSNECKRNFVAVYDGSSSIENLK
AKFCSTVANDVMLKTGIGVIRMWADEGSRLSRFRMLFTSFVEPPCTSSTFFCHSNMCINNSLV
CNGVQNCAYPWDENHCKEKKKAGVFEQITKTHGTIIGITSGIVLVLLIISILVQVKQPRKKVM
ACKTAFNKTGFQEVFDPPHYELFSLRDKEISADLADLSEELDNYQKMRRSSTASRCIHDHHCG
SQASSVKQSRTNLSSMELPFRNDFAQPQPMKTFNSTFKKSSYTFKQGHECPEQALEDRVMEEI
PCEIYVRGREDSAQASISIDF

**Important features of the protein:**

**Signal peptide:**

amino acids 1-22

**Transmembrane domain:**

amino acids 348-369

**N-glycosylation sites.**

amino acids 311-315, 385-389, 453-457, 475-479

**cAMP- and cGMP-dependent protein kinase phosphorylation sites.**

amino acids 426-430, 479-483

**N-myristoylation sites.**

amino acids 22-28, 32-38, 54-60, 186-192, 279-285, 318-324, 348-354, 352-358, 441-447

# FIGURE 115

GGTCTCTGTCCTTGGCTGTGGCTCCTGCGCTCTGGCTGAGCC**ATG**TTCCTTCTCCTCGCCCTC
CTCACTGAGCTTGGAAGACTGCAAGCCCACGAAGGTTCTGAAGGAATATTTCTGCATGTCACA
GTTCCACGGAAGATTAAGTCAAATGACAGTGAAGTTTCAGAGAGGAAGATGATTTACATCATT
ACAATTGATGGACAACCTTACACTCTACATCTCGGAAAACAATCATTCTTACCCCAGAACTTT
TTGGTTTATACATATAATGAAACTGGATCTTTGCATTCTGTGTCTCCATATTTTATGATGCAT
TGCCATTACCAAGGATATGCTGCCGAATTTCCAAATTCATTTGTGACACTCAGTATATGTTCT
GGTCTCAGGGGATTTCTCCAGTTTGAAAATATCAGTTATGGAATTGAACCAGTAGAATCTTCA
GCAAGATTTGAGCATATAATTTATCAAATGAAAAATAATGATCCAAATGTATCCATTTTAGCA
GTAAATTACAGTCATATTTGGCAGAAAGACCAGCCCTACAAAGTTCCTTTAAACTCACAGATA
AAAAATCTTTCAAAACTATTACCCCAATATCTGGAAATATACATTATAGTGGAAAAAGCTTTG
ATGTTTACCCAGTTCAAATTGACTGTTATACTGTCTTCCTTGGAATTGTGGTCAAATGAAAAC
CAGATTTCCACCAGTGGGGATGCTGATGATATATTACAAAGATTTTTGGCATGGAAACGGGAC
TATCTCATCCTACGGCCCCATGACATAGCATACTTACTTGTTTACAGGAAACATCCTAAATAT
GTGGGAGCAACATTTCCTGGCACCGTATGCAATAAAAGCTATGATGCAGGTATTGCTATGTAT
CCAGATGCAATAGGTTTGGAGGGATTTTCGGTTATTATAGCTCAACTGCTTGGCCTTAATGTA
GGATTAACATATGATGACATCACTCAGTGTTTCTGTCTGAGAGCTACATGCATCATGAATCAT
GAAGCAGTGAGTGCCAGTGGTAGAAAGATTTTTAGCAACTGCAGCATGCACGACTATAGATAT
TTTGTTTCAAAATTTGAGACTAAATGCCTTCAGAAGCTTTCAAATTTGCAACCATTACATCAA
AATCAACCAGTGTGTGGTAATGGGATTTTGGAATCCAATGAAGAATGTGACTGTGGTAATAAA
AATGAATGTCAATTTAAGAAGTGCTGTGATTATAACACATGTAAACTGAAGGGCTCAGTAAAA
TGTGGTTCTGGACCATGTTGTACATCAAAGTGTGAGTTGTCAATAGCAGGCACTCCATGTAGA
AAGAGTATTGATCCAGAGTGTGATTTTACAGAGTACTGCAATGGAACCTCTAGTAATTGTGTT
CCTGACACTTATGCACTGAATGGCCGTTTGTGCAAGTTGGGAACTGCCTATTGCTATAACGGA
CAATGTCAAACTACTGATAACCAGTGTGCCAAGATATTTGGAAAAGGTGCTCAAGGTGCTCCA
TTTGCCTGTTTTAAAGAAGTTAATTCTCTGCATGAAAGATCTGAAAACTGTGGTTTTAAAAAT
TCACAACCATTACCTTGTGAACGGAAGGATGTTCTCTGTGGAAAATTAGCTTGTGTTCAGCCA
CATAAAAATGCTAATAAAAGTGACGCTCAATCTACAGTTTATTCATATATTCAAGACCATGTA
TGTGTATCTATAGCCACTGGTTCCTCCATGAGATCAGATGGAACAGACAATGCCTATGTGGCT
GATGGCACCATGTGTGGTCCAGAAATGTACTGTGTAAATAAAACCTGCAGAAAAGTTCATTTA
ATGGGATATAACTGTAATGCCACCACAAAATGCAAAGGGAAAGGGATATGTAATAATTTTGGT
AATTGTCAATGCTTCCCTGGACATAGACCTCCAGATTGTAAATTCCAGTTTGGTTCCCCAGGG
GGTAGTATTGATGATGGAAATTTTCAGAAATCTGGTGACTTTTATACTGAAAAAGGCTACAAT
ACACACTGGAACAACTGGTTTATTCTGAGTTTCTGCATTTTTCTGCCGTTTTTCATAGTTTTC
ACCACTGTGATCTTTAAAAGAAATGAAATAAGTAAATCATGTAACAGAGAGAATGCAGAGTAT
AATCGTAATTCATCCGTTGTATCAGAAAGCGATGACGTGGGACAT**TAA**TATTGCACAGAACTT
CCATAGCAAATAACCTAAAGGAACGAATGTGCTTTATTTATAACCTTACGTTATCCCCAATGC
ATTGTAAATGTCAAACTTTTGGAAAATAAAGCCTGCGTGCCCTCCC

# FIGURE 116

MFLLLALLTELGRLQAHEGSEGIFLHVTVPRKIKSNDSEVSERKMIYIITIDGQPYTLHLGKQ
SFLPQNFLVYTYNETGSLHSVSPYFMMHCHYQGYAAEFPNSFVTLSICSGLRGFLQFENISYG
IEPVESSARFEHIIYQMKNNDPNVSILAVNYSHIWQKDQPYKVPLNSQIKNLSKLLPQYLEIY
IIVEKALMFTQFKLTVILSSLELWSNENQISTSGDADDILQRFLAWKRDYLILRPHDIAYLLV
YRKHPKYVGATFPGTVCNKSYDAGIAMYPDAIGLEGFSVIIAQLLGLNVGLTYDDITQCFCLR
ATCIMNHEAVSASGRKIFSNCSMHDYRYFVSKFETKCLQKLSNLQPLHQNQPVCGNGILESNE
ECDCGNKNECQFKKCCDYNTCKLKGSVKCGSGPCCTSKCELSIAGTPCRKSIDPECDFTEYCN
GTSSNCVPDTYALNGRLCKLGTAYCYNGQCQTTDNQCAKIFGKGAQGAPFACFKEVNSLHERS
ENCGFKNSQPLPCERKDVLCGKLACVQPHKNANKSDAQSTVYSYIQDHVCVSIATGSSMRSDG
TDNAYVADGTMCGPEMYCVNKTCRKVHLMGYNCNATTKCKGKGICNNFGNCQCFPGHRPPDCK
FQFGSPGGSIDDGNFQKSGDFYTEKGYNTHWNNWFILSFCIFLPFFIVFTTVIFKRNEISKSC
NRENAEYNRNSSVVSESDDVGH

**Important features of the protein:**

**Signal peptide:**

amino acids 1-16

**Transmembrane domain:**

amino acids 665-684

**N-glycosylation sites.**

amino acids 36-39, 76-79, 122-125, 149-152, 156-159, 177-180, 270-273, 335-338, 441-444, 537-540, 587-590, 601-604, 703-706

**Casein kinase II phosphorylation sites.**

amino acids 74-77, 208-211, 221-224, 304-307, 337-340, 346-349, 376-380, 415-418, 499-502, 639-642, 708-711

**Tyrosine kinase phosphorylation site.**

amino acids 243-249

**N-myristoylation sites.**

amino acids 53-58, 79-84, 266-271, 298-303, 372-377, 403-408, 408-413, 442-447, 462-467, 469-474, 488-493, 567-572, 610-615, 616-621, 634-639

**Amidation site.**

amino acids 328-331

# FIGURE 117

CCCACGCGTCCGCGGACGCGTGGGGCTCAGTGGGCGTCGCGCGAAGGCTAAGGGAGTGTGGCG
GGCGGCTCCGGGAGCCAAC**ATG**CCTCGGTATGCGCAGCTGGTCATGGGCCCCGCGGGCAGCGG
GAAGAGCACCTACTGTGCCACCATGGTCCAGCACTGTGAAGCCCTCAACCGGTCTGTCCAAGT
TGTAAACCTGGATCCAGCAGCAGAACACTTCAACTACTCCGTGATGGCTGACATCCGGGAACT
GATCGAGGTGGATGATGTAATGGAGGATGATTCTCTGCGATTCGGTCCCAACGGAGGATTGGT
ATTTTGCATGGAGTACTTTGCCAATAATTTGACTGGCTGGAGAACTGTCTTGGCCATGTAGA
GGACGACTATATCCTTTTTGATTGTCCAGGTCAGATTGAGTTGTACACTCACCTGCCTGTGAT
GAAACATCTGGTCCAGCAGCTCGAGCAGTGGGAGTTCCGAGTCTGTGGAGTTTTTCTTGTTGA
TTCTCAGTTCATGGTGGAGTCATTCAAGTTTATTTCTGGCATCTTGGCAGCCCTGAGTGCCAT
GATCTCTCTAGAAATTCCGCAAGTCAACATCATGACAAAAATGGATCTGCTGAGTAAAAAAGC
AAAAAAGGAAATTGAGAAATTTTTAGATCCAGACATGTATTCTTTATTAGAAGATTCTACAAG
TGACTTAAGAAGCAAAAAATTCAAGAAACTGACTAAAGCTATATGTGGACTGATTGATGACTA
CAGCATGGTTCGATTTTTACCTTACGATCAGTCAGATGAAGAAAGCATGAACATTGTATTGCA
GCATATTGATTTTGCCATTCAATATGGAGAAGACCTAGAATTTAAAGAACCAAAGGAACGTGA
AGATGAGTCTTCCTCTATGTTTGACGAATATTTTCAAGAATGCCAGGATGAA**TGA**AGAGTTTA
CTAAAAGTAACCATCTAAAGAGCTTGTGGCCAAACCAGCAGAACATTCTTCTCTTCAAAGGAT
GCAATAGTAGAAAGCTACTTATTTAATGAAAAAAAGTAAAACTTCGTTCTTTATCAGCCTCA
TGCCTGAATCAAATTTTTAATTATTCTGAAACTGCTGCTGTTTAAAGTGGAATCTTTTAGTAT
TATAACAGCATCACTTTAGATTTTGTAAGTCAAAATTGAAATGAATGCACATAGATTTATATA
TAAATTAGCACCTGAGCTAAGGTTAAGGCCGGTCTAAACTTATTTTCACTTTTTGTATTATTT
TTGAGATGCAGGAATTACTGTAACAAAATATGTATGTCCGAAGGGAAAAAGCTGCAAGGATAT
ATATAAGACCACTGCTTATCTGTATCTTCCCATTTTCCTATATTGAAAATGTATATTATTTAT
ATAACTTAAAAAGTAAAAATAACTATGTTTTGAGAT

# FIGURE 118

MPRYAQLVMGPAGSGKSTYCATMVQHCEALNRSVQVVNLDPAAEHFNYSVMADIRELIEVDDV
MEDDSLRFGPNGGLVFCMEYFANNFDWLENCLGHVEDDYILFDCPGQIELYTHLPVMKHLVQQ
LEQWEFRVCGVFLVDSQFMVESFKFISGILAALSAMISLEIPQVNIMTKMDLLSKKAKKEIEK
FLDPDMYSLLEDSTSDLRSKKFKKLTKAICGLIDDYSMVRFLPYDQSDEESMNIVLQHIDFAI
QYGEDLEFKEPKEREDESSSMFDEYFQECQDE

**Important features of the protein:**

**Signal peptide:**

amino acids 1-29

**Transmembrane domain:**

amino acids 151-170

**N-glycosylation sites.**

amino acids 31-35, 47-51

**cAMP- and cGMP-dependent protein kinase phosphorylation site.**

amino acids 212-216

**Tyrosine kinase phosphorylation site.**

amino acids 189-197

**N-myristoylation sites.**

amino acids 13-19, 76-82, 154-160

**ATP/GTP-binding site motif A (P-loop).**

amino acids 10-18

# FIGURE 119

GGGCGCTGGGAGACACCGGACGCCCGCTCGGCTGCGCTGCGGCTCAGGCCCCCGCTCGGGCCC
GACCCGCTCGGTCACCGCCGGCTCGGGCGCGCACCTGCCGGCTGCGGCCCCAGGGCCATGCGG
AGGCCCACGAGGAGGCCGGCGGCCACGCGCATCCCGTAGCCCAGGTGGCCCAGGTCTGCACCG
CGGCGGCCTCGGCGCC**ATG**GAGCCCCCGTATTCGCTGACGGCGCACTACGATGAGTTCCAAGA
GGTCAAGTACGTGAGCCGCTGCGGCGCGGGGGCGCGCGCGGGGCCTCCCTGCCCCCGGGCTT
CCCGTTGGGCGCTGCGCGCAGCGTCACCGGGGCCCGGTCCGGGCTGCCGCGCTGGAACCGGCG
CGAGGTGTGCCTGCTGTCGGGGCTGGTGTTCGCCGCCGGCCTCTGCGCCATTCTGGCGGCTAT
GCTGGCCCTCAAGTACCTGGGCCCGGTCGCGGCCGGCGGCGGCGCCTGTCCCGAGGGCTGCCC
TGAGCGCAAGGCCTTCGCGCGCGCCGCTCGCTTCCTGGCCGCCAACCTGGACGCCAGCATCGA
CCCATGCCAGGACTTCTACTCGTTCGCCTGCGGCGGTTGGCTGCGGCGCCACGCCATCCCCGA
CGACAAGCTCACCTATGGCACCATCGCGGCCATCGGCGAGCAAAACGAGGAGCGCCTACGGCG
CCTGCTGGCGCGGCCCGGGGGTGGGCCTGGCGGCGCGGCCCAGCGCAAGGTGCGCGCCTTCTT
CCGCTCGTGCCTCGACATGCGCGAGATCGAGCGACTGGGCCCGCGACCCATGCTAGAGGTCAT
CGAGGACTGCGGGGGCTGGGACCTGGGCGGCGCGGAGGAGCGTCCGGGGGTCGCGGCGCGATG
GGACCTCAACCGGCTGCTGTACAAGGCGCAGGGCGTGTACAGCGCCGCCGCGCTCTTCTCGCT
CACGGTCAGCCTGGACGACAGGAACTCCTCGCGCTACGTCATCCGCATTGACCAGGATGGGCT
CACCCTGCCAGAGAGGACCCTGTACCTCGCTCAGGATGAGGACAGTGAGAAGATCCTGGCAGC
ATACAGGGTGTTCATGGAGCGAGTGCTCAGCCTCCTGGGTGCAGACGCTGTGGAACAGAAGGC
CCAAGAGATCCTGCAAGTGGAGCAGCAGCTGGCCAACATCACTGTGTCAGAGTATGACGACCT
ACGGCGAGATGTCAGCTCCATGTACAACAAGGTGACGCTGGGGCAGCTGCAGAAGATCACCCC
CCACTTGCGGTGGAAGTGGCTGCTAGACCAGATCTTCCAGGAGGACTTCTCAGAGGAAGAGGA
GGTGGTGCTGCTGGCGACAGACTACATGCAGCAGGTGTCGCAGCTCATCCGCTCCACACCCCA
CCGGGTCCTGCACAACTACCTGGTGTGGCGCGTGGTGGTGGTCCTGAGTGAACACCTGTCCCC
GCCATTCCGTGAGGCACTGCACGAGCTGGCACAGGAGATGGAGGGCAGCGACAAGCCACAGGA
GCTGGCCCGGGTCTGCTTGGGCCAGGCCAATCGCCACTTTGGCATGGCGCTTGGCGCCCTCTT
TGTACATGAGCACTTCTCAGCCGCCAGCAAAGCCAAGGTGCAGCAGCTAGTGGAAGACATCAA
GTACATCCTGGGCCAGCGCCTGGAGGAGCTGGACTGGATGGACGCCGAGACCAGGGCTGCTGC
TCGGGCCAAGCTCCAGTACATGATGGTGATGGTCGGCTACCCGGACTTCCTGCTGAAACCCGA
TGCTGTGGACAAGGAGTATGAGTTTGAGGTCCATGAGAAGACCTACTTCAAGAACATCTTGAA
CAGCATCCCCTTCAGCATCCAGCTCTCAGTTAAGAAGATTCGGCAGGAGGTGGACAAGTCCAC
GTGGCTGCTCCCCCCACAGGCGCTCAATGCCTACTATCTACCCAACAAGAACCAGATGGTGTT
CCCCGCGGGCATCCTGCAGCCCACCCTGTACGACCCTGACTTCCCACAGTCTCTCAACTACGG
GGGCATCGGCACCATCATTGGACATGAGCTGACCCACGGCTACGACGACTGGGGGGGCCAGTA
TGACCGCTCAGGGAACCTGCTGCACTGGTGGACGGAGGCCTCCTACAGCCGCTTCCTGCGAAA
GGCTGAGTGCATCGTCCGTCTCTATGACAACTTCACTGTCTACAACCAGCGGGTGAACGGGAA
ACACACGCTTGGGGAGAACATCGCAGATATGGGCGTCCTCAAGCTGGCCTACCACGCCTATCA
GAAGTGGGTGCGGGAGCACGGCCCAGAGCACCCACTTCCCCGGCTCAAGTACACACATGACCA
GCTCTTCTTCATTGCCTTTGCCCAGAACTGGTGCATCAAGCGGCGGTCGCAGTCCATCTACCT
GCAGGTGCTGACTGACAAGCATGCCCCTGAGCACTACAGGGTGCTGGGCAGTGTGTCCCAGTT
TGAGGAGTTTGGCCGGGCTTTCCACTGTCCCAAGGACTCACCCATGAACCCTGCCCACAAGTG
TTCCGTGTGG**TGA**GCCTGGCTGCCCGCCTGCACGCCCCCACTGCCCCCGCACGAATCACCTCC
TGCTGGCTACCGGGGCAGGCATGCACCCGGTGCCAGCCCCGCTCTGGGCACCACCTGCCTTCC
AGCCCCTCCAGGACCCGGTCCCCCTGCTGCCCCTCACTTCAGGAGGGGCCTGGAGCAGGGTGA
GGCTGGACTTTGGGGGGCTGTGAGGGAAATATACTGGGGTCCCCAGATTCTGCTCTAAGGGGG
CCAGACCCTCTGCCAGGCTGGATTGTACGGGCCCCACCTTCGCTGTGTTCTTGCTGCAAAGTC
TGGTCAATAAATCACTGCACTGTTAAAAAAAAA

# FIGURE 120

MEPPYSLTAHYDEFQEVKYVSRCGAGGARGASLPPGFPLGAARSVTGARSGLPRWNRREVCLL
SGLVFAAGLCAILAAMLALKYLGPVAAGGGACPEGCPERKAFARAARFLAANLDASIDPCQDF
YSFACGGWLRRHAIPDDKLTYGTIAAIGEQNEERLRRLLARPGGGPGGAAQRKVRAFFRSCLD
MREIERLGPRPMLEVIEDCGGWDLGGAEERPGVAARWDLNRLLYKAQGVYSAAALFSLTVSLD
DRNSSRYVIRIDQDGLTLPERTLYLAQDEDSEKILAAYRVFMERVLSLLGADAVEQKAQEILQ
VEQQLANITVSEYDDLRRDVSSMYNKVTLGQLQKITPHLRWKWLLDQIFQEDFSEEEEVVLLA
TDYMQQVSQLIRSTPHRVLHNYLVWRVVVVLSEHLSPPFREALHELAQEMEGSDKPQELARVC
LGQANRHFGMALGALFVHEHFSAASKAKVQQLVEDIKYILGQRLEELDWMDAETRAAARAKLQ
YMMVMVGYPDFLLKPDAVDKEYEFEVHEKTYFKNILNSIPFSIQLSVKKIRQEVDKSTWLLPP
QALNAYYLPNKNQMVFPAGILQPTLYDPDFPQSLNYGGIGTIIGHELTHGYDDWGGQYDRSGN
LLHWWTEASYSRFLRKAECIVRLYDNFTVYNQRVNGKHTLGENIADMGVLKLAYHAYQKWVRE
HGPEHPLPRLKYTHDQLFFIAFAQNWCIKRRSQSIYLQVLTDKHAPEHYRVLGSVSQFEEFGR
AFHCPKDSPMNPAHKCSVW

**Important features of the protein:**

**Transmembrane domain:**

amino acids 64-88

**N-glycosylation sites.**

amino acids 255-259, 322-326, 656-660

**cAMP- and cGMP-dependent protein kinase phosphorylation site.**

amino acids 722-726

**N-myristoylation site.**

amino acids 24-30, 26-32, 27-33, 40-46, 47-53, 65-71, 148-154, 169-175, 170-176, 237-243, 450-456, 604-610, 607-613

**Prokaryotic membrane lipoprotein lipid attachment site.**

amino acids 85-96

**Prenyl group binding site.**

amino acids 772-777

**Neutral zinc metallopeptidases, zinc-binding region signature.**

amino acids 609-619

**Neutral zinc metallopeptidases, zinc-binding region proteins.**

amino acids 609-619

# FIGURE 121

CGGACTGCCCGGACCGCGCG**ATG**GAGTCGACCGGCAGCGTCGGGGAGGCCCCGGGCGGACCCC
GGGTGCTGGTGGTGGGCGGCGGCATCGCGGGGCTGGGCGCGGCGCAGAGGCTCTGCGGCCACT
CCGCCTTCCCGCACCTGCGGGTCCTGGAGGCCACGGCCCGCGCCGGGGGCCGCATCCGCTCGG
AGCGCTGCTTCGGTGGCGTGGTGGAGGTGGGCGCGCACTGGATCCATGGGCCCTCCCGGGGTA
ACCCCGTCTTCCAGCTGGCTGCTGAGTACGGGCTGCTGGGGGAGAAGGAGCTGTCCCAGGAGA
ACCAGCTGGTGGAGACCGGGGGTCACGTGGGCCTGCCCTCCGTGAGCTACGCCAGCTCCGGGG
CCAGCGTGAGCCTCCAGCTGGTGGCGGAGATGGCGACTCTGTTCTACGGCCTGATAGACCAGA
CCCGGGAGTTCCTGCACGCTGCAGAGACCCCGGTGCCCAGCGTCGGGGAGTACCTCAAGAAGG
AGATTGGCCAGCACGTGGCCGGCTGGACAGAGGATGAGGAGACCAGGAAGCTGAAGCTGGCCG
TCCTGAACTCCTTCTTCAACCTGGAATGCTGTGTGAGCGGCACCCACAGCATGGACCTGGTGG
CCCTGGCACCCTTTGGGGAGTATACCGTGCTGCCGGGGCTGGACTGCACCTTTTCTAAGGGCT
ATCAAGGACTCACAAACTGCATGATGGCCGCCCTGCCGGAGGACACTGTAGTTTTTGAGAAGC
CTGTGAAGACCATCCACTGGAACGGGTCCTTCCAGGAGGCAGCCTTTCCCGGGGAGACCTTTC
CAGTGTCGGTAGAGTGTGAGGATGGAGACCGGTTCCCGGCGCACCATGTCATCGTCACCGTGC
CCTTAGGTTTTCTTAGGGAACATTTGGACACCTTCTTTGACCCTCCCCTGCCGGCTGAGAAGG
CAGAAGCAATCAGGAAGATAGGCTTTGGGACCAACAACAAAATCTTCCTGGAGTTTGAGGAGC
CCTTCTGGGAGCCAGACTGCCAGCTGATCCAGCTGGTGTGGGAGGACACGTCGCCCCTGGAGG
ATGCTGCCCCTGAGCTACAGGACGCCTGGTTCCGGAAGCTCATTGGCTTTGTGGTCCTGCCTG
CCTTTGCGTCTGTCCACGTTCTCTGTGGGTTCATTGCCGGACTTGAGTCTGAGTTCATGGAGA
CTCTGTCGGATGAAGAAGTACTTCTGTGTCTCACCCAAGTGCTCCGGAGAGTGACAGGAAACC
CACGGCTCCCCGCGCCCAAGAGCGTCCTGCGGTCTCGCTGGCACAGCGCCCCGTACACTAGGG
GGTCCTACAGCTACGTGGCCGTGGGCAGTACTGGGGGCGACCTGGACCTGCTGGCTCAGCCCC
TCCCTGCAGACGGCGCCGGCGCCCAGCTCCAGATCCTGTTTGCGGGGGAAGCCACACATCGCA
CGTTTTACTCCACGACGCACGGGGCTCTGCTGTCGGGATGGAGGGAGGCCGACCGCCTCCTCA
GTCTGTGGGCCCCGCAGGTGCAGCAGCCCAGGCCGAGGCTC**TAG**CTGGGCCCAGCCTACTCTG
TTCCACCCGTGTCGGGGGTAGGCTGGGACCGTCATTTCTTCTGACAGATTTCAGTCTGGCTTG
AAATTTGGGGATGTTAATGAGGGTCCTCTGGTTTTTGGTAACCAGGGCCACCTTCTCAGTTCT
TGTGTCTGTTATTGGAGTCTGGCCAGGGTTGACTTGAGCTGAGACACCAGATGCTCACGGAGA
TGCTGGACACATAAAGCAAGTTACAGCCACAAAAAAAAAAAA

# FIGURE 122

MESTGSVGEAPGGPRVLVVGGGIAGLGAAQRLCGHSAFPHLRVLEATARAGGRIRSERCFGGV
VEVGAHWIHGPSRGNPVFQLAAEYGLLGEKELSQENQLVETGGHVGLPSVSYASSGASVSLQL
VAEMATLFYGLIDQTREFLHAAETPVPSVGEYLKKEIGQHVAGWTEDEETRKLKLAVLNSFFN
LECCVSGTHSMDLVALAPFGEYTVLPGLDCTFSKGYQGLTNCMMAALPEDTVVFEKPVKTIHW
NGSFQEAAFPGETFPVSVECEDGDRFPAHHVIVTVPLGFLREHLDTFFDPPLPAEKAEAIRKI
GFGTNNKIFLEFEEPFWEPDCQLIQLVWEDTSPLEDAAPELQDAWFRKLIGFVVLPAFASVHV
LCGFIAGLESEFMETLSDEEVLLCLTQVLRRVTGNPRLPAPKSVLRSRWHSAPYTRGSYSYVA
VGSTGGDLDLLAQPLPADGAGAQLQILFAGEATHRTFYSTTHGALLSGWREADRLLSLWAPQV
QQPRPRL

**Signal peptide:**

amino acids 1-28

**Transmembrane domain:**

amino acids 364-385

**N-glycosylation site.**

amino acids 253-257

**cAMP- and cGMP-dependent protein kinase phosphorylation site.**

amino acids 408-412

**N-myristoylation sites.**

amino acids 20-26, 21-27, 25-31, 105-111, 119-125, 164-170, 216-222, 227-233, 443-449, 484-490

**Aminooxidase Flavin containing amine oxidase:**

amino acids 23-497

# FIGURE 123

CGGACGCGTGGGGGAAGATGGATAAATAATTCTGTCACACGTGCCCTGGCCTCTGGAGCTCAGCTGCCAGTCCAC
GTCTAGGGAATCTTAGCATCTGGGACCAAGACACTTTACAGCAATCATCACCCTTTGCAGAGGAGGTGAGCTCAC
CAGGACTCATCTGCCATTTCAGACCTTTTGCTGCTACCTGCCAGGTGGCCCCCACTGCTGACGAGAG**ATG**GTGGA
TCTCTCAGTCTCCCCGGACTCCTTGAAGCCAGTATCGCTGACCAGCAGTCTTGTCTTCCTCATGCACCTCCTCCT
CCTTCAGCCTGGGGAGCCGAGCTCAGAGGTCAAGGTGCTAGGCCCTGAGTATCCCATCCTGGCCCTCGTCGGGGA
GGAGGTGGAGTTCCCGTGCCACCTATGGCCACAGCTGGATGCCCAGCAAATGGAGATCCGCTGGTTCCGGAGTCA
GACCTTCAATGTGGTACACCTGTACCAGGAGCAGCAGGAGCTCCCTGGCAGGCAGATGCCGGCGTTCCGGAACAG
GACCAAGTTGGTCAAGGACGACATCGCCTATGGCAGCGTGGTCCTGCAGCTTCACAGCATCATCCCCTCTGACAA
GGGCACATATGGCTGCCGCTTCCACTCCGACAACTTCTCTGGCGAAGCTCTCTGGGAACTGGAGGTAGCAGGGCT
GGGCTCAGACCCTCACCTCTCCCTTGAGGGCTTCAAGGAAGGAGGCATTCAGCTGAGGCTCAGATCCAGTGGCTG
GTACCCCAAGCCTAAGGTTCAGTGGAGAGACCACCAGGGACAGTGCCTGCCTCCAGAGTTTGAAGCCATCGTCTG
GGATGCCCAGGACCTGTTCAGTCTGGAAACATCTGTGGTTGTCCGAGCGGGAGCCCTCAGCAATGTGTCCGTCTC
CATCCAGAATCTCCTCTTGAGCCAGAAGAAAGAGTTGGTGGTCCAGATAGCAGACGTGTTCGTACCCGGAGCCTC
TGCGTGGAAGAGCGCGTTCGTCGCGACCCTGCCGCTGCTGTTGGTCCTCGCGGCGCTGGCGCTGGGCGTCCTCCG
GAAGCAGCGGAGAAGCCGAGAAAAGCTGAGGAAGCAGGCGGAGAAGAGACAAGAGAAACTCACTGCAGAGCTGGA
AAAGCTTCAGACAGAGCTTGACTGGAGACGGGCTGAAGGCCAGGCTGAGTGGAGAGCAGCCCAAAAATATGCAGT
GGATGTGACGCTGGACCCGGCCTCGGCGCACCCCAGCCTGGAGGTGTCGGAGGATGGCAAGAGCGTGTCTTCCCG
CGGGGCGCCGCCAGGCCCGGCGCCTGGCCACCCGCAGCGGTTCTCGGAGCAGACGTGCGCGCTGAGCCTGGAGCG
GTTCTCCGCCGGCCGCCACTACTGGGAGGTGCACGTGGGCCGCCGCAGCCGCTGGTTCCTGGGCGCCTGCCTGGC
CGCGGTGCCGCGCGCGGGGCCTGCGCGCCTGAGCCCTGCGGCCGGCTACTGGGTGCTGGGGCTGTGGAACGGCTG
CGAGTACTTCGTCCTGGCCCCGCACCGCGTCGCGCTCACCCTGCGCGTGCCCCCGCGGCGCCTGGGCGTCTTCCT
GGACTACGAGGCCGGAGAGCTGTCCTTCTTCAACGTGTCCGACGGCTCCCACATCTTCACCTTCCACGACACCTT
CTCGGGCGCGCTCTGTGCGTACTTCAGGCCCAGGGCCCACGACGGCGGCGAACATCCGGATCCCCTGACCATCTG
CCCGCTGCCGGTTAGAGGGACGGGCGTCCCCGAAGAGAACGACAGTGACACCTGGCTACAGCCCTATGAGCCCGC
GGACCCCGCCCTGGACTGGTGG**TGA**GGCGCCCTCGTGGCCGCGGGACTGGCCCCGGGGGGCCCCCTGGATCCCAG
GCCAGCGCTTTGCTCTCCTGCTCCGTCTGAAGGGAGCAGGTGCACCAGCCAAAATGTCAGCGAGGGGGACAAAGA
GAGGGACCTTTGCCTACGTAGATGTGTATGTGTAGTGCGATTTTCTTCAAGGAAAGGAGACAAGTCCAAAGCTCG
TTTGTGGATTGTGGGACTGAGCGAAGGAGTACAAATATATCCACGTCGCTCAGAGCTGGGGTGCTCACGGTGGGC
GGTGGGCAAGAAGCCAGCATGGAAGAAAGAAGGGAGAAAACTTTGGTGACTGCCTTAGAGGGATCAGTTAATTTG
TATAGTTTTATATTTTTTGTATATGTTTGCTAGCTCTAAAAAGGTCGAGATGCAATAACACTTCGTAAGCAACGA
GTTCACCTAAGTAAGGCTCAGATCCTAGTTTTAAAAACCATTTCCCATTAAAATGAAGTTGGAGGAACAGCTGCT
TCTGAGCCGGGGCAAAAATTTCAAGGTGAGCCTGGAGCATTGTGTGTGGTGAAGTAAAATAAAGGCTCAAAACGT
GACGGCAACCCGGCAAAAGGGTAGGGAGCCAGGCCGAAGGGGCCTCACTGACCAATTGTGGGACAATTTGAACAT
CAGGATGAATAATGACAGGAGAGATTATAACACACTGAATAAAAACATAATCCATGAGTTCATGCTGATACTCAA
ATTTCTTTTTAAAAAGGAGAAACAGGAAGGTTTCTTTTGGAGGTGAAATCTAATTATTGGTGAGAGTCTTGGAGA
ACAGGCTGTTTCCAGTCTCAAAGCAGTAACCTTATACACTACTTATAAGTTTGAAAGGGGAAAGGTTACCTTTAC
AATGGAGACATCTACCAGATCATCCAAGTGATTAAATTTAACATCATCAATGATGGGACCAAGGACATTATTAGT
TTGACAACTGGGGAAAGAAGTGTTCTTCACCCCCTACCCCCAAGACATTCTCTCTGTCGGCCAGGCTGGAGTGCA
GCCTCAACCTCCTGGGCCCAAGTGATCCTCCCACCTCAGCACACAACACCATGCCCAATTTTAAGTGCGTTATAG
AGACGGGGGTCTCACTTTGTTACCCAGGCTGGTCTCAAACTCCTGCGCTCAAGCAATCCTCCCACCTGGGCCTCC
CAAAATGCTGGGTGTACAGGCATGAGCCGCTGTGCCTGGCTTCATTTTCAGAGTGAGACATTTGTACTGTGGCTA
TGTAGGAGAACATTCTTGTTCTTAGCAAACATACTGAAGTTTTTAGATATTAATTACCACAGTGTCTGCCACTGA
ATTTCCAGTGACTAAGTGGAAAAATATAAAACATATGAATATAAAGAAAGAAAGAGACAAGTCAAATGTAGTAAA
ATGACAACACTTGGTGACTCTAGGTGACTGGTCGACAGATGTTCATTGTACTATCAATGTGGCTTTGCTGTGGGT
TTGAAATTTTGCAAACTAAGAGTTGGGTGGCGGGGAGAAGGATACACCAAAAAACTAAGTGATTATCTTTGGATG
GGAAAATGTTTGGTAATTGCATTCTTAAAATGTCTTCTTTGTATTTTTTAATGTTCAATAATGTATATGTATCAG
TTCTGTAATAAAGGGGAAAACACTTTTCA

# FIGURE 124

MVDLSVSPDSLKPVSLTSSLVFLMHLLLLQPGEPSSEVKVLGPEYPILALVGEEVEFPCHLWP
QLDAQQMEIRWFRSQTFNVVHLYQEQQELPGRQMPAFRNRTKLVKDDIAYGSVVLQLHSIIPS
DKGTYGCRFHSDNFSGEALWELEVAGLGSDPHLSLEGFKEGGIQLRLRSSGWYPKPKVQWRDH
QGQCLPPEFEAIVWDAQDLFSLETSVVVRAGALSNVSVSIQNLLLSQKKELVVQIADVFVPGA
SAWKSAFVATLPLLLVLAALALGVLRKQRRSREKLRKQAEKRQEKLTAELEKLQTELDWRRAE
GQAEWRAAQKYAVDVTLDPASAHPSLEVSEDGKSVSSRGAPPGPAPGHPQRFSEQTCALSLER
FSAGRHYWEVHVGRRSRWFLGACLAAVPRAGPARLSPAAGYWVLGLWNGCEYFVLAPHRVALT
LRVPPRRLGVFLDYEAGELSFFNVSDGSHIFTFHDTFSGALCAYFRPRAHDGGEHPDPLTICP
LPVRGTGVPEENDSDTWLQPYEPADPALDWW

**Important features of the protein:**

**Signal peptide:**

amino acids 1-34

**Transmembrane domain:**

amino acids 247-272

**N-glycosylation sites.**

amino acids 102-106, 139-143, 224-228, 464-468, 516-520

**Tyrosine kinase phosphorylation site.**

amino acids 105-114

**N-myristoylation sites.**

amino acids 129-135, 220-226, 399-405, 423-429, 480-486

**Amidation site.**

amino acids 390-394

# FIGURE 125

TATAGTCCCAGCTACTCATGGGGCTGATGCAGGTTGAGGCAGGAGGTTCATGAGCCCAGGAGGTTGGAGCTGTAA
TGAGCTAGGATTCTGCCTCTGCACTCCTAGCTGGATGACAGAGCAAGACCCTGTCTCAAAAAGAAAAAAAAAAA
AAAAAGAATGCATGAACCAGACATGACAGTTCCTGGCCTCAAAGATCTTCCAAAGGAAATGATTTTTTTTAACC
ACCAATGCTGCAGGAAAAAGCAACATATTTAAGTTATCCAATAACACCTATCCAATAATTGTAAATCATTATCAT
GAC**ATG**GTAGAGTTGTTTATATTTCTTTTCCTTTTAGGTGAAACACCATTCAAAGTCGTAGTCAAATCTCTTTCA
CCTAAAGAGTTGGTCCGGATACATGTCCCTAAACCTTTGGACAGGAATGATGGAACATTTTTGATGAGATATAGG
ATGTATGAAACTGTCGATGAAGGCCTGAAGATAGAGGTCCTTTATGGTGATGAACATGTGGCTCAGTCTCCCTAT
ATTTTGAAAGGACCAGTGTACCATGAGTACTGTGAGTGTCCGGAAGATCCTCAGGCCTGGCAGAAGACTCTTTCT
TGTCCAACCAAGGAACCACAGATTGCAAAAGATTTTGCTTCCTTTCCCAGCATCAATCTCCAGCAAATGCTAAAA
GAAGTCCCCAAAAGGTTTGGGGATGAGAGAGGTGCCATTGTTCATTACACGATTCTCAATAACCATGTTTACCGG
AGATCTTTAGGGAAATACACAGACTTCAAGATGTTCTCTGATGAGATTTTGTTATCATTGACAAGAAAGGTCCTT
CTCCCAGATTTAGAATTTTATGTTAATCTTGGAGATTGGCCCTTGGAGCATCGAAAAGTCAATGGAACCCCTAGC
CCCATACCTATCATTTCATGGTGTGGCTCTCTGGATTCAAGAGATGTTGTCCTTCCAACGTATGACATCACCCAC
TCCATGCTTGAAGCCATGCGGGGTGTTACAAATGATCTCCTCTCTATTCAGGGAAATACAGGGCCTTCCTGGATC
AATAAAACAGAGAGAGCTTTCTTCAGAGGTAGAGACAGCCGAGAGGAGAGGCTCCAGTTGGTACAGCTGTCCAAA
GAAAATCCTCAGCTACTAGATGCAGGAATTACAGGATATTTCTTTTTCCAAGAGAAAGAAAAGGAGCTTGGAAAA
GCCAAGTTGATGGGTTTCTTTGATTTCTTTAAGTACAAGTATCAAGTAAATGTGGATGGGACCGTGGCTGCTTAC
AGATATCCATATCTCATGCTGGGCGACAGTCTGGTTTTAAAGCAGGACTCGCCATATTATGAACATTTCTACATG
GCACTAGAACCTTGGAAGCATTATGTTCCAATTAAAAGAAATCTGAGTGATTTATTAGAGAAAGTTAAATGGGCT
AAGGAAAATGATGAAGAAGCCAAGAAGATTGCAAAAGAAGGACAGTTGATGGCTAGGGACCTACTACAGCCACAC
AGGCTTTACTGCTACTATTACCAAGTACTGCAGAAATATGCCGAGCGCCAGTCCAGCAAACCCGAAGTACGTGAT
GGAATGGAACTTGTTCCTCAGCCAGAAGATAGCACAGCCATCTGCCAGTGCCACAGGAAAAAGCCTTCAAGAGAA
GAACTT**TGA**GTCAGCCCAGAATCACACTCCTGTGTATCCCGGCTACACTTTAAGGAAAGATTGAATCTAAGCTGT
GAAGGACAGTATAGAAGACTGCACCAAGTGGACTAGTTCTCCCGGTGGCTTTATATATGTAGATGGATATAGCAG
TACTGGTTGAGTATCCCTCATCTGAAATGCTTAGGACCAGGAGTGTTTCAGGCTTCAGATTTTTTAAGATTTGGG
AATATTTGCATGTACATAATGAGGTATCTTGGGGATGAGATCCAAGTCTAAACACAAAATTCATTTATATTTTAT
ATATACCTTGTTCACATACCCTGAAGGTAATTTTATATAATATTTTTAATAATTTGTGCATGAAACAAAGTTTGT
ATACATTGAACTGTCAGAAAGCAAAGGTGTCACTATCTTAGCGACCCAAGTGGTGGTGTCAGCACTCAAAAAGTT
TTGGATTTTGGGGTATTTCAGATTTTAGATTTTTGTATGAGGAATGTTCAACCTGTATTTGAACAAGCATTACCA
AATATCATTGAATATTAATATCTTTTGCGTAAAAACTGCTATTATCAGCATCATAGTTTCTCTAAAAAGAAAACT
TGGGGATCATAGCCGATAGAGAGACTTGCTAAAATATAAATCAGCCTCTGCAAAACTGTTTACATATTTATTGGT
TTACATATTTTATTGGTTTATTTCTATCCCCTGTTCACTTTTTCTCTTCCACTTCCAATTATGAAGAGAAAATAT
TTGTTCAGGGTTGTCCCCCCGCCCCCCGTCACTGCATAATTTCTCCTCTTACAAGCTGCTTTTGGCTTTCATTAA
TAACAGCTTCCTTTTAGAAGGTCTGATAAGGATATTTAAGGAAGAAGAGAATGACTCTGTTATTAAAGGTGGCAT
GGAGACTGTGGAGGGAATATTTTTTAAAGCACTACTCATATCCTTTAAACTAAATTTTGCCAAAGCCCGAGACAA
CATTAAGGAGAAATTGTACCTTAAGTTAGTAATTCCAAATCTATCTGAGTTGTATACCCATCAAAGACAATACAG
TTATTAACATAGATGAAGGTATGCTATAGGCATCATTCATTATCTCTATATTGAATAGGTGAAAGATAACTGTAG
TCAGGTGAAAGGCATTCATCATTTTTAAGCTGAAAAGGGGATCCTTGAAAACACTGAAAACCTCTACAACAATCT
TCAGGAAGCCTGCTATCTTGGGATTCACTAATAATAGGCCAAGAACAAAGGCAAGCATCCATTCCTCACTCCACC
ACTTTTCTATTTCAGTGGGTGTCATTGCTACGATGAAGACTTTGGAAATTTCCTTTCTCTTTTAGGACAGGGTCA
GGATTTAGGACTCATAGCCTGAAAGCTCATTACATACTCCTTGTAACCATCAGTCCAAGGTTCAGTTCACTAAAG
TGCATGTTCTAAAACAAGAGCTATCCTCATTCCAAATTTTAAAATATGTACTCTGGCCGGTTGCAGTGGCTCACG
CCTGTAATCCCAGCACTTTGGCAGGCCGAGATGGGCGGATCTTTTGAGGTCAGGAGTTTGAGACCAGCCTGGCCA
ACATGGTGAAACCCCGTCTCTACTAAAAATACAAAAATTAGCCAGGCATGGTGGCATTTGCCTGTAATCCCAGCT
ACTCGGGAGGCTGAGGCAGGAGAATCACTTGAACCTGGGAGGCAGAGGTTGCAGTGAGCTGAGATTACACCACTG
CACTCCAGCCTGGGTGACAGAGTGAGACTCCATCTCAAAAACTGAAAATAAAAATAAAAATATGTATTCTCCTAA
CTGAAATATTTACTTAATCTGGAAAACAATGTAACTATTTTTAAAGTGGTTACATCTATTCTTGCTGAAGAACAA
TAAACAGAATTTTTTGACTAAGCATAACCAAATTTCAGAACAGTCTAATCAATGCCAAGTATCCAAGGCAAACTC
TAATACCCATCCATTGTGCAAAACCACAAGCACGCAAGTATTAAATAAGAGCAAGCTGTCCTGAGCCCATACCTA
ATGAATTTGTGTCTTAAATATTGTACATTGTGTTTGAGGCTTGTCAAAACTGGGATTATGGCAAGAAAGGTTGCC
TAACTCATACCTTTCTGCCTCAAATTCCAGGTGCTAAAGGCTAATGGCATTTTAAACATCTTACATTTTTAAAAA
TTTATATTGCCTCTGCCAAACAGGCCTAATAGTTAAAAGCAAGTTGAGACAAACCAGGCAGATTCAGTGTGTGGA
ACAGGAAGGATGTGCTTTAAAAAAAGGTGGAATCCCTCAAAAAATTCTATAGGGAGACAGCAGCCTTAATCTACA
TAATTCTTCATCTCGCCAATTCAGCCGCAGCCTTTAAAGAGTTAGTGTTAATGGCTTTCTGGTTTGAAAACAAAA
ATGCATCTATGTGGTTGAAAGTTTGGGAGGAGATTCACCAATATCTGAGGAGAAGATGGAGTGAAGGGAATTCTT
ACTTTTTGCTTTATACCTTTCTATAATATTTAGATTTTTTTTACTGTAAGTATGGATCAAATTGCAAAATAAAG
AAAAATGCCAACCTTAGAAAAGACAATAAATGCACAAAAGATATAAACAGGAACAGCAAATATTTATATTTTTTC
CATTTTGCTCTTTTTAAATCTATGTTTAGAACTTTATATCTTGGGACTTATGTATATATATACCTTTTAAATAAA
ATAAATTTTCTAAATAAAAAGTTG

# FIGURE 126

MVELFIFLFLLGETPFKVVVKSLSPKELVRIHVPKPLDRNDGTFLMRYRMYETVDEGLKIEVL
YGDEHVAQSPYILKGPVYHEYCECPEDPQAWQKTLSCPTKEPQIAKDFASFPSINLQQMLKEV
PKRFGDERGAIVHYTILNNHVYRRSLGKYTDFKMFSDEILLSLTRKVLLPDLEFYVNLGDWPL
EHRKVNGTPSPIPIISWCGSLDSRDVVLPTYDITHSMLEAMRGVTNDLLSIQGNTGPSWINKT
ERAFFRGRDSREERLQLVQLSKENPQLLDAGITGYFFFQEKEKELGKAKLMGFFDFFKYKYQV
NVDGTVAAYRYPYLMLGDSLVLKQDSPYYEHFYMALEPWKHYVPIKRNLSDLLEKVKWAKEND
EEAKKIAKEGQLMARDLLQPHRLYCYYYQVLQKYAERQSSKPEVRDGMELVPQPEDSTAICQC
HRKKPSREEL

**Important features of the protein:**

**Signal peptide:**

amino acids 1-16

**N-glycosylation sites.**

amino acids 250-254, 363-367

**cAMP- and cGMP-dependent protein kinase phosphorylation site.**

amino acids 444-448

**N-myristoylation site.**

amino acids 208-214, 319-325, 388-394

**Endoplasmic reticulum targeting sequence.**

amino acids 448-453

**Mitochondrial energy transfer proteins signature.**

amino acids 25-34

# FIGURE 127

AGCCGTCGGAGGGAGCCGGAGCGCTTCTCCCGAGTTGGTGATAGATTGGTGGTCATCCAACAT
GCAGAAATGAATGAGCAGTGAAAAGCAGCAGAGCCGATGGGTCATGAGGATGTAAGTGCGTTT
GAAGGCTTCCACACCCTCTACTCCAGGAATCATGAATAAACTGGAGGATAAGCAGGACCAGAT
GATACC**ATG**AAGAGAAGTTTACAGGCCCTCTATTGCCAACTGTTAAGTTTCCTGCTGATCTTG
GCACTGACCGAAGCGCTGGCATTTGCCATCCAGGAACCATCTCCCAGGGAATCTCTTCAGGTC
CTCCCTTCAGGCACTCCCCCGGGAACCATGGTGACAGCACCCCACAGCTCTACCAGACATACT
TCTGTGGTGATGCTGACCCCCAATCCCGATGGACCCCCCTCACAGGCTGCAGCTCCCATGGCA
ACACTGACACCCCGTGCAGAGGGGCACCCTCCTACGCACACCATCTCCACCATCGCTGCGACA
GTAACCGCCCCCTATTCTGAAAGCTCCCTGTCCACAGGGCCCGCTCCAGCAGCCATGGCAACC
ACATCCTCCAAGCCAGAGGGCCGCCCTCGAGGGCAGGCTGCCCCCACCATCCTGCTGACAAAG
CCACCGGGGGCCACCAGCCGCCCCACCACAGCGCCCCCCCGCACTACCACACGCAGGCCCCCC
AGGCCCCCAGGCTCTTCCCGAAAAGGGGCTGGTAATTCATCACGCCCTGTCCCGCCTGCACCT
GGTGGCCACTCCAGGAGTAAAGAAGGACAGCGAGGACGAAATCCAAGCTCCACACCTCTGGGG
CAGAAGCGGCCCCTGGGGAAAATCTTTCAGATCTACAAGGGCAACTTCACAGGGTCTGTGGAA
CCAGAGCCCTCTACCCTCACCCCCAGGACCCCACTCTGGGGCTACTCCTCTTCACCACAGCCC
CAGACAGTGGCTGCGACCACAGTGCCCAGCAATACCTCATGGGCACCCACCACCACCTCCCTG
GGGCCTGCAAAGGACAAGCCAGGCCTTCGCAGAGCAGCCCAGGGGGGTGGTTCTACCTTCACC
AGCCAAGGAGGGACACCAGATGCCACAGCAGCCTCAGGTGCCCCTGTCAGTCCACAAGCTGCC
CCAGTGCCTTCTCAGCGCCCCCACCACGGTGACCCACAGGATGGCCCCAGCCATAGTGACTCT
TGGCTTACTGTTACCCCTGGCACCAGCAGACCTCTGTCTACCAGCTCTGGGGTCTTCACGGCT
GCCACGGGGCCCACCCCAGCTGCCTTCGATACCAGTGTCTCAGCCCCTTCCCAGGGGATTCCT
CAGGGAGCATCCACAACCCCACAAGCTCCAACCCATCCCTCCAGGGTCTCAGAAAGCACTATT
TCTGGAGCCAAGGAGGAGACTGTGGCCACCCTCACCATGACCGACCGGGTGCCCAGTCCTCTC
TCCACAGTGGTATCCACAGCCACAGGCAATTTCCTCAACCGCCTGGTCCCCGCCGGGACCTGG
AAGCCTGGGACAGCAGGGAACATCTCCCATGTGGCCGAGGGGGACAAACCGCAGCACAGAGCC
ACCATCTGCCTGAGCAAGATGGATATCGCCTGGGTGATCCTGGCCATCAGCGTGCCCATCTCC
TCCTGCTCTGTCCTGCTGACGGTGTGCTGCATGAAGAGGAAGAAGAAGACCGCCAACCCGGAG
AACAACCTGAGCTACTGGAACAACACCATCACCATGGACTACTTCAACAGGCATGCTGTGGAG
CTGCCCAGGGAGATCCAGTCCCTTGAAACCTCTGAGGACCAGCTCTCAGAGCCCCGCTCCCCA
GCCAATGGCGACTATAGAGACACTGGGATGGTCCTTGTTAACCCCTTCTGTCAAGAAACACTG
TTTGTGGGAAACGATCAAGTATCTGAGATC**TAA**CTACAGCAGGCATCACTTTGCCATTCCGTA
TTTTTCGTCTCTAAATTATAAATATACAAATATATATATTATAAATATAACCTTGTGTAACCC
TGACTTAATGAGAAACATTTTCAGCTTTTTTTCCTATGAATTGTCAACATCTTTTTTACAAGT
GTGGTTTAAAAAAAAAAAAAACTTTACAGAATGATCTGTGGCTTTATAAAATAAAGGTATTTCT
AAGCAAAAAAAAAAAAAAAAA

# FIGURE 128

MKRSLQALYCQLLSFLLILALTEALAFAIQEPSPRESLQVLPSGTPPGTMVTAPHSSTRHTSV
VMLTPNPDGPPSQAAAPMATLTPRAEGHPPTHTISTIAATVTAPYSESSLSTGPAPAAMATTS
SKPEGRPRGQAAPTILLTKPPGATSRPTTAPPRTTTRRPPRPPGSSRKGAGNSSRPVPPAPGG
HSRSKEGQRGRNPSSTPLGQKRPLGKIFQIYKGNFTGSVEPEPSTLTPRTPLWGYSSSPQPQT
VAATTVPSNTSWAPTTTSLGPAKDKPGLRRAAQGGGSTFTSQGGTPDATAASGAPVSPQAAPV
PSQRPHHGDPQDGPSHSDSWLTVTPGTSRPLSTSSGVFTAATGPTPAAFDTSVSAPSQGIPQG
ASTTPQAPTHPSRVSESTISGAKEETVATLTMTDRVPSPLSTVVSTATGNFLNRLVPAGTWKP
GTAGNISHVAEGDKPQHRATICLSKMDIAWVILAISVPISSCSVLLTVCCMKRKKKTANPENN
LSYWNNTITMDYFNRHAVELPREIQSLETSEDQLSEPRSPANGDYRDTGMVLVNPFCQETLFV
GNDQVSEI

**Important features of the protein:**

**Signal peptide:**

amino acids 1-28

**Transmembrane domain:**

amino acids 469-487

**N-glycosylation sites.**

amino acids 178-182, 223-227, 261-265, 446-450, 504-508, 509-513

**cAMP- and cGMP-dependent protein kinase phosphorylation site.**

amino acids 495-499

**N-myristoylation sites.**

amino acids 44-50, 48-54, 175-181, 222-228, 279-285, 286-292, 288-294, 296-302, 351-357, 374-380, 427-433, 442-448

**TonB-dependent receptor proteins signature 1.**

amino acids 1-44

# FIGURE 129

AGGCGAGGCGCGGCGCCGCTGCACACACGCACACGGAGCT**ATG**GGGTGCCATGTTGCCACCAG
CTGCCACGTGGCCTGGCTTTTGGTGCTGATCTCTGGATGCTGGGGCCAGGTGAACCGGCTGCC
CTTCTTCACCAACCACTTCTTTGATACATACCTGCTGATCAGCGAGGACACGCCTGTGGGTTC
TTCTGTGACCCAGTTGCTGGCCCAAGACATGGACAATGACCCCCTGGTGTTTGGCGTGTCTGG
GGAGGAGGCCTCTCGCTTCTTTGCAGTGGAGCCTGACACTGGCGTGGTGTGGCTCCGGCAGCC
ACTGGACAGAGAGACCAAGTCAGAGTTCACCGTGGAGTTCTCTGTCAGCGACCACCAGGGGGT
GATCACACGGAAGGTGAACATCCAGGTCGGGGATGTGAATGACAACGCGCCCACATTTCACAA
TCAGCCCTACAGCGTCCGCATCCCTGAGAATACACCAGTGGGGACGCCCATCTTCATCGTGAA
TGCCACAGACCCCGACTTGGGGGCAGGGGGCAGCGTCCTCTACTCCTTCCAGCCCCCCTCCCA
ATTCTTCGCCATTGACAGCGCCCGCGGTATCGTCACAGTGATCCGGGAGCTGGACTACGAGAC
CACACAGGCCTACCAGCTCACGGTCAACGCCACAGATCAAGACAAGACCAGGCCTCTGTCCAC
CCTGGCCAACTTGGCCATCATCATCACAGATGTCCAGGACATGGACCCCATCTTCATCAACCT
GCCTTACAGCACCAACATCTACGAGCATTCTCCTCCGGGCACGACGGTGCGCATCATCACCGC
CATAGACCAGGATAAAGGACGTCCCCGGGGCATTGGCTACACCATCGTTTCAGGGAATACCAA
CAGCATCTTTGCCCTGGACTACATCAGCGGAGTGCTGACCTTGAATGGCCTGCTGGACCGGGA
GAACCCCCTGTACAGCCATGGCTTCATCCTGACTGTGAAGGGCACGGAGCTGAACGATGACCG
CACCCCATCTGACGCTACAGTCACCACGACCTTCAATATCCTGGTTATTGACATCAATGACAA
TGCCCCGGAGTTCAACAGCTCCGAGTACAGCGTGGCCATCACTGAGCTGGCACAGGTCGGCTT
TGCCCTTCCACTCTTCATCCAGGTGGTGGACAAGGATGAGAATTTGGGCCTGAACAGCATGTT
TGAGGTGTACTTGGTGGGGAACAACTCCCACCACTTCATCATCTCCCCGACCTCCGTCCAGGG
GAAGGCGGACATTCGTATTCGGGTGGCCATCCCACTGGACTACGAGACCGTGGACCGCTACGA
CTTTGATCTCTTTGCCAATGAGAGTGTGCCTGACCATGTGGGCTATGCCAAGGTGAAGATCAC
TCTCATCAATGAAAATGACAACCGGCCCATCTTCAGCCAGCCACTGTACAACATCAGCCTGTA
CGAGAACGTCACCGTGGGGACCTCTGTGCTGACAGTCCTGGTGAGTCCCCGCTTCACTGCAGG
GCCACTGAGCTCTCCAGGGCCGACTGTGGTGAGGCACCCAGAGGGATTTTGTCCAAGGGACCT
CAGCAATCAGGGAAGGAGGCACCCCCAAATCCCTGAGCTGTGTTTGTTGGTGTAT**TAA**ATAAA
GTTTTTGGACTCTTCAGGAAGGGGCTCCCTTGACCTAGGTTGCAATATGGAAAAGGAGCCAAC
CTGAGGGGTGACGAGACTGAGCTGAGGACACTGGTTTTCTGCCTTTCCCTGAGAGAGACTCAG
TGAGGGTGGGCTGGGAGCCCTGGAAGCCCCCTCAAATGGGTGGGAAGGTGCCAGCCATCCTTG
AGAAGGGCAACCCTCTCCATGTGAGCACAGGCACCAGAGAGGGGCAGGCGCCTGGAGGGTACC
GGGGCACCCCCAGCTGCCCATGGCTGGACTTGCCCTTTGACAAGGGGCCCTCCCAGTGTCATT
TGTATCTGTCAGTACTCTTGGTTGCAAGGGACAGAAACCCTTAAGTAGTTCAAGCAAAAAAGG
ATTGGCTCATGTAACTCAAAAGTATAAGTGATTTCAGGCCGGGCTCGGTGGCTCACGCCTGTC
ATCCAACACCTTGAGAAAGCCGAGGTGGGCGGATCACTTGAGGTCGGGAGTTTGAGACCAGCC
TGGCCAACATGGCAAAACCCCGTCTCTACTAAAAATACAAAAATTAGCCGGGTGTGGTGGCAC
ACGCCTGTAGTCCCAGCTACTAGGGAGGCTGAGGCAGGAGAATCGCTTGAACCCAGGAGGCGG
AGGTTGCAGTGAGCCGAGATTGTGTCACTGCCCTCCAGCCTGGGCGACAGAGCCAGATTCTGT
CTC

# FIGURE 130

MGCHVATSCHVAWLLVLISGCWGQVNRLPFFTNHFFDTYLLISEDTPVGSSVTQLLAQDMDND
PLVFGVSGEEASRFFAVEPDTGVVWLRQPLDRETKSEFTVEFSVSDHQGVITRKVNIQVGDVN
DNAPTFHNQPYSVRIPENTPVGTPIFIVNATDPDLGAGGSVLYSFQPPSQFFAIDSARGIVTV
IRELDYETTQAYQLTVNATDQDKTRPLSTLANLAIIITDVQDMDPIFINLPYSTNIYEHSPPG
TTVRIITAIDQDKGRPRGIGYTIVSGNTNSIFALDYISGVLTLNGLLDRENPLYSHGFILTVK
GTELNDDRTPSDATVTTTFNILVIDINDNAPEFNSSEYSVAITELAQVGFALPLFIQVVDKDE
NLGLNSMFEVYLVGNNSHHFIISPTSVQGKADIRIRVAIPLDYETVDRYDFDLFANESVPDHV
GYAKVKITLINENDNRPIFSQPLYNISLYENVTVGTSVLTVLVSPRFTAGPLSSPGPTVVRHP
EGFCPRDLSNQGRRHPQIPELCLLVY

**Important features of the protein:**

**Signal peptide:**

amino acids 1-23

**Transmembrane domain:**

amino acids 355-374

**N-glycosylation sites.**

amino acids 155-159, 206-210, 349-353, 393-397, 434-438, 466-470, 472-476

**N-myristoylation sites.**

amino acids 2-8, 49-55, 162-168, 270-276, 278-284, 316-322

**Amidation site.**

amino acids 515-519

**Prokaryotic membrane lipoprotein lipid attachment site.**

amino acids 11-22

**Leucine zipper pattern.**

amino acids 298-320

**PTS HPR component serine phosphorylation site signature.**

amino acids 377-393

**Cadherins extracellular repeated domain signature.**

amino acids 120-131, 336-347

**Cadherins extracellular**

amino acids 120-144, 336-360

# FIGURE 131

GTGGGCCGCCCCTGCTGCTGCCGTCC**ATG**CTGATGTTTGCGGTGATCGTGGCCTCCAGCGGGC
TGCTGCTCATGATCGAGCGGGGCATCCTGGCCGAGATGAAGCCCCTGCCCCTGCACCCGCCCG
GCCGCGAGGGCACAGCCTGGCGCGGGAAAGCCCCCAAGCCTGGGGGCCTGTCCCTCAGGGCTG
GGGACGCGGACTTGCAAGTGCGGCAGGACGTCCGGAACAGGACCCTGCGGGCGGTGTGCGGAC
AGCCAGGCATGCCCCGGGACCCCTGGGACTTGCCGGTGGGGCAGCGGCGCACCCTGCTGCGCC
ACATCCTCGTAAGTGACCGTTACCGCTTCCTCTACTGCTACGTCCCCAAGGTGGCCTGCTCTA
ACTGGAAGCGGGTGATGAAGGTGCTGGCAGGCGTCCTGGACAGCGTGGACGTCCGCCTCAAGA
TGGACCACCGCAGTGACCTGGTGTTCCTGGCCGACCTGCGGCCTGAGGAGATTCGCTACCGCC
TGCAGCACTACTTTAAGTTCCTGTTTGTGCGGGAGCCCTTGGAACGCCTCCTCTCTGCCTACC
GCAACAAGTTTGGCGAGATCCGAGAGTACCAGCAACGCTATGGGGCTGAGATAGTGAGGCGGT
ACAGGGCTGGAGCGGGGCCCAGCCCTGCAGGCGACGATGTCACATTCCCCGAGTTCCTGAGAT
ACCTGGTGGATGAGGACCCTGAGCGCATGAATGAGCATTGGATGCCCGTGTACCACCTGTGCC
AGCCTTGTGCCGTGCACTATGACTTTGTGGGCTCCTATGAGAGGCTGGAGGCTGATGCAAATC
AGGTGCTGGAGTGGGTACGGGCACCACCTCACGTCCGATTTCCAGCTCGCCAGGCCTGGTACC
GGCCAGCCAGCCCCGAAAGCCTGCATTACCACTTGTGCAGTGCCCCCCGGGCCCTGCTGCAGG
ATGTGCTGCCTAAGTATATCCTGGACTTCTCCCTCTTTGCCTACCCACTGCCTAATGTCACCA
AGGAGGCGTGTCAGCAG**TGA**CCATGGGTGTGGGGCCAGCAGCTGGTGGGGACTGGTTTCAACG
CCAGCTTTCTGTGCTTCTGCCTGTCATTCGGAGAAACTCTGGCTCTGGGGCTTGGGGCTTCTC
AGGATCCTGGATGGCAGAGACTGCCCTCAGAAGTTCCTTGTCCAGGGTGGGCACCCACAGTGA
CTCAGAGGACAGGGCTAGGCAGGAGACCTGCTGCTCCTCATTGGGGGGATCTCTTGGGGGGCA
GACACCAGTTTGCCAATGAAGCAACACATCTGATCTAAAGACTGGCTCCAGACCCCGGGCTGC
CAGGATTATGCAGTCCACTTGGTCTACCTTAATTTAACCTGTGGCCAAACTCAGAGATGGTAC
CAGCCAGGGGCAAGCATGACCAGAGCCAGGGACCCTGTGGCTCTGATCCCCCATTTATCCACC
CCATGTGCCTCAGGACTAGAGTGAGCAATCATACCTTATAAATGACTTTTGTGCCTTTCTGCT
CCAGTCTCAAAATTTCCTACACCTGCCAGTTCTTTACATTTTTCCAAGGAAAGGAAAACGGAA
GCAGGGTTCTTGCCTGGTAGCTCCAGGACCCAGCTCTGCAGGCACCCAAAGACCCTCTGTGCC
CAGCCTCTTCCTTGAGTTCTCGGAACCTCCTCCCTAATTCTCCCTTCCTTCCCCACAAGGCCT
TTGAGGTTGTGACTGTGGCTGGTATATCTGGCTGCCATTTTTCTGATGCATTTATTTAAAATT
TGTACTTTTTGATAGAACCCTTGTAAGGGCTTTGTTTTCCTAATAGCTGACTTTTTAATAAAG
CAGTTTTATATAT

# FIGURE 132

MLMFAVIVASSGLLLMIERGILAEMKPLPLHPPGREGTAWRGKAPKPGGLSLRAGDADLQVRQ
DVRNRTLRAVCGQPGMPRDPWDLPVGQRRTLLRHILVSDRYRFLYCYVPKVACSNWKRVMKVL
AGVLDSVDVRLKMDHRSDLVFLADLRPEEIRYRLQHYFKFLFVREPLERLLSAYRNKFGEIRE
YQQRYGAEIVRRYRAGAGPSPAGDDVTFPEFLRYLVDEDPERMNEHWMPVYHLCQPCAVHYDF
VGSYERLEADANQVLEWVRAPPHVRFPARQAWYRPASPESLHYHLCSAPRALLQDVLPKYILD
FSLFAYPLPNVTKEACQQ

**Important features of the protein:**

**Signal peptide:**

amino acids 1-23

**N-glycosylation sites.**

amino acids 67-71, 325-329

**Tyrosine kinase phosphorylation sites.**

amino acids 152-159, 183-183

**N-myristoylation sites.**

amino acids 89-95, 128-134

# FIGURE 133

CGGCAGTTCTGGCCCCTGCAGCTGGAGGTACCCTGAGTTCTGAGGGTCGTAGTGCTGTTTCTG
GTATTCTCATCGCGGTCACCTCTACCGGTGTGGACAAGTAAAGTTTGAATCAGCTTCTCCATG
GCCTGGGCACCAGTTCCCGGCTGAGCCATTTTCCTTTTGGCTAAAAGTCCCCGCCCAGAGGCC
AATTCGTCGCGGCGGCGGTGGAGATCGCAGGTCGCTCAGGCTTGCAG**ATG**GGTCAAGGGTTGT
GGAGAGTGGTCAGAAACCAGCAGCTGCAACAAGAAGGCTACAGTGAGCAAGGCTACCTCACCA
GAGAGCAGAGCAGGAGAATGGATGCGAGCAACATTTCTAACACCAATCATCGTAAACAAGTCC
AAGGAGGCATTGACATATATCATCTTTTGAAGGCAAGGAAATCGAAAGAACAGGAAGGATTCA
TTAATTTGGAAATGTTGCCTCCTGAGCTAAGCTTTACCATCTTGTCCTACCTGAATGCAACTG
ACCTTTGCTTGGCTTCATGTGTTTGGCAGGACCTTGCGAATGATGAACTTCTCTGGCAAGGGT
TGTGCAAATCCACTTGGGGTCACTGTTCCATATACAATAAGAACCCACCTTTAGGATTTTCTT
TTAGAAAATTGTATATGCAGCTGGATGAAGGCAGCCTCACCTTTAATGCCAACCCAGATGAGG
GAGTGAACTACTTTATGTCCAAGGGTATCCTGGATGATTCGCCAAAGGAAATAGCAAAGTTTA
TCTTCTGTACAAGAACACTAAATTGGAAAAAACTGAGAATCTATCTTGATGAAAGGAGAGATG
TCTTGGATGACCTTGTAACATTGCATAATTTAGAAATCAGTTCTTGCCAAATGCACTGAGAG
AATTTTTTCGTCATATCCATGCCCCTGAAGAGCGTGGAGAGTATCTTGAAACTCTTATAACAA
AGTTCTCACATAGATTCTGTGCTTGCAACCCTGATTTAATGCGAGAACTTGGCCTTAGTCCTG
ATGCTGTCTATGTACTGTGCTACTCTTTGATTCTACTTTCCATTGACCTCACTAGCCCTCATG
TGAAGAATAAAATGTCAAAAAGGGAATTTATTCGAAATACCCGTCGCGCTGCTCAAAATATTA
GTGAAGATTTTGTAGGGCATCTTTATGACAATATCTACCTTATTGGCCATGTGGCTGCA**TAA**A
AAGCACAATTGCTAGGACTTCAGTTTTTACTTCAGACTAAAGCTACCCAAGGACTTAGCAGAT
ATGGGGGTTACATCAGTGCTGGTCATTGTAGCCTGAGTATACAATCAAGCTTCAGTGTGCAAC
CTTTTTTTCTTTTGCCATTTTCTATTTTAGTAATTTCCTTGGGGAACTAAATAATTTTGCAGA
ATTTTTCCTAATTTTGTTTATCACGTTTTGCACAAAGCAGAGCCACTGTCTAACACAGCTGTT
AACGAATGATAAACTGACATTATACTCTAAAAGATGGTGTATTTGTGCATTAGATTTGCCTGA
AAAACTTTATCCATTTCCATTCTTTATACAAATACCATGTAATGTGTACATATTTAACTAAAG
AGATTTATAGTCATAATTATTTTATTGTAAAGATTTAACTAAAGTTTTTCCTTTTCTCTC

# FIGURE 134

MGQGLWRVVRNQQLQQEGYSEQGYLTREQSRRMDASNISNTNHRKQVQGGIDIYHLLKARKSK
EQEGFINLEMLPPELSFTILSYLNATDLCLASCVWQDLANDELLWQGLCKSTWGHCSIYNKNP
PLGFSFRKLYMQLDEGSLTFNANPDEGVNYFMSKGILDDSPKEIAKFIFCTRTLNWKKLRIYL
DERRDVLDDLVTLHNFRNQFLPNALREFFRHIHAPEERGEYLETLITKFSHRFCACNPDLMRE
LGLSPDAVYVLCYSLILLSIDLTSPHVKNKMSKREFIRNTRRAAQNISEDFVGHLYDNIYLIG
HVAA

**Important features of the protein:**

**Transmembrane domain:**

amino acids 253-272

**N-glycosylation sites.**

amino acids 37-41, 87-91, 298-302

**N-myristoylation site.**

amino acids 110-116

# FIGURE 135

GGCACGAGGGAGCCTCCGTTAGGGGGTGGGAAAGGACTTTGCCATAGGTCGCTGAGGCCACCA
TCTGCTCTCTTACTGGCCAAGGGCGTAAAAAGATAGTCTTCCCATTAGCTAGAGAGCAAACCC
CAGAAAGCCTATTGGCTGCGCCGTCCGCGGGCCTTGGTCCGCTTTGAAGGCGGGCTGCGGCTG
CGAGAGGAGGGCGGGCGGGAGGCTAGCTGTTGTCGTGGTTGCTCGGAGGCACGTGTGCAGTCC
CGGAAGCGGCGAGGGGAAACTGCTCCGCGCGCGCCGCGGGAGGAGGAACCGCCCGGTCCTTTA
GGGTCCGGGCCCGGCCGGGCC**ATG**GATTCAATGCCTGAGCCCGCGTCCCGCTGTCTTCTGCTT
CTTCCCTTGCTGCTGCTGCTGCTGCTGCTGCTGCCGGCCCCGGAGCTGGGCCCGAGCCAGGCC
GGAGCTGAGGAGAACGACTGGGTTCGCCTGCCCAGCAAATGCGAAGTGTGTAAATATGTTGCT
GTGGAGCTGAAGTCAGCCTTTGAGGAAACCGGCAAGACCAAGGAGGTGATTGGCACGGGCTAT
GGCATCCTGGACCAGAAGGCCTCTGGAGTCAAATACACCAAGTCGGACTTGCGGTTAATCGAA
GTCACTGAGACCATTTGCAAGAGGCTCCTGGATTATAGCCTGCACAAGGAGAGGACCGGCAGC
AATCGATTTGCCAAGGGCATGTCAGAGACCTTTGAGACATTACACAACCTGGTACACAAAGGG
GTCAAGGTGGTGATGGACATCCCCTATGAGCTGTGGAACGAGACTTCTGCAGAGGTGGCTGAC
CTCAAGAAGCAGTGTGATGTGCTGGTGGAAGAGTTTGAGGAGGTGATCGAGGACTGGTACAGG
AACCACCAGGAGGAAGACCTGACTGAATTCCTCTGCGCCAACCACGTGCTGAAGGGAAAAGAC
ACCAGTTGCCTGGCAGAGCAGTGGTCCGGCAAGAAGGGAGACACAGCTGCCCTGGGAGGGAAG
AAGTCCAAGAAGAAGAGCAGCAGGGCCAAGGCAGCAGGCGGCAGGAGTAGCAGCAGCAAACAA
AGGAAGGAGCTGGGTGGCCTTGAGGGAGACCCCAGCCCCGAGGAGGATGAGGGCATCCAGAAG
GCATCCCCTCTCACACACAGCCCCCCTGATGAGCTC**TGA**GCCCACCCAGCATCCTCTGTCCTG
AGACCCCTGATTTTGAAGCTGAGGAGTCAGGGGCATGGCTCTGGCAGGCCGGGATGGCCCCGC
AGCCTTCAGCCCCTCCTTGCCTTGGCTGTGCCCTCTTCTGCCAAGGAAAGACACAAGCCCCAG
GAAGAACTCAGAGCCGTCATGGGTAGCCCACGCCGTCCTTTCCCCTCCCCAAGTGTTTCTCTC
CTGACCCAGGGTTCAGGCAGGCCTTGTGGTTTCAGGACTGCAAGGACTCCAGTGTGAACTCAG
GAGGGGCAGGTGTCAGAACTGGGCACCAGGACTGGAGCCCCCTCCGGAGACCAAACTCACCAT
CCCTCAGTCCTCCCCAACAGGGTACTAGGACTGCAGCCCCCTGTAGCTCCTCTCTGCTTACCC
CTCCTGTGGACACCTTGCACTCTGCCTGGCCCTTCCCAGAGCCCAAAGAGTAAAAATGTTCTG
GTTCTGATTTCTGAAAAAAAAAAAAAAAAAAAATTCCT

# FIGURE 136

MDSMPEPASRCLLLLPLLLLLLLLLPAPELGPSQAGAEENDWVRLPSKCEVCKYVAVELKSAF
EETGKTKEVIGTGYGILDQKASGVKYTKSDLRLIEVTETICKRLLDYSLHKERTGSNRFAKGM
SETFETLHNLVHKGVKVVMDIPYELWNETSAEVADLKKQCDVLVEEFEEVIEDWYRNHQEEDL
TEFLCANHVLKGKDTSCLAEQWSGKKGDTAALGGKKSKKKSSRAKAAGGRSSSSKQRKELGGL
EGDPSPEEDEGIQKASPLTHSPPDEL

**Important features of the protein:**

**Signal peptide:**

amino acids 1-26

**N-glycosylation site.**

amino acids 153-157

**cAMP- and cGMP-dependent protein kinase phosphorylation sites.**

amino acids 227-231, 228-232

**Tyrosine kinase phosphorylation site.**

amino acids 142-150

**N-myristoylation sites.**

amino acids 36-42, 74-80, 86-92, 125-131, 222-228, 237-243, 250-256, 263-269

**Amidation sites.**

amino acids 212-216, 222-226

**ATP/GTP-binding site motif A (P-loop).**

amino acids 62-70

# FIGURE 137

CACGCCTCCCGCTGCCAGCCCGGCACCGGGATCTTAATCAGTCACTATGAAAACTCATTAGCT
CCACAGCA**ATG**AGTCCTCCACTGCTGAAGCTTGGCGCTGTGCTTAGTACCATGGCAATGATCT
CAAACTGGATGTCCCAAACTCTCCCATCCTTGGTGGGACTGAACACCACGAGGCTGTCGACTC
CGGATACCTTAACTCAGATTAGTCCTAAAGAAGGGTGGCAGGTGTACAGCTCAGCTCAGGATC
CTGATGGGCGGTGCATTTGCACAGTTGTTGCTCCAGAACAAAACCTGTGTTCCCGGGATGCCA
AAAGCAGGCAACTTCGCCAACTACTGGAAAAGGTTCAGAACATGTCCCAGTCTATTGAAGTCT
TAAACTTGAGAACTCAGAGAGATTTCCAATATGTTTTAAAAATGGAAACCCAAATGAAAGGGC
TGAAGGCAAAATTTCGGCAGATTGAAGATGATCGAAAGACACTTATGACCAAGCATTTTCAGG
AGTTGAAAGAGAAAATGGACGAGCTCCTGCCTTTGATCCCCGTGCTGGAACAGTACAAAACAG
ATGCTAAGTTAATCACCCAGTTCAAGGAGGAAATAAGGAATCTGTCTGCTGTCCTCACTGGTA
TTCAGGAGGAAATTGGTGCCTATGACTACGAGGAACTACACCAAAGAGTGCTGAGCTTGGAAA
CAAGACTTCGTGACTGCATGAAAAAGCTAACATGTGGCAAACTGATGAAAATCACAGGCCCAG
TTACAGTCAAGACATCTGGAACCCGATTTGGTGCTTGGATGACAGACCCTTTAGCATCTGAGA
AAAACAACAGAGTCTGGTACATGGACAGTTATACTAACAATAAAATTGTTCGTGAATACAAAT
CAATTGCAGACTTTGTCAGTGGGGCTGAATCAAGGACATACAACCTTCCTTTCAAGTGGGCAG
GAACTAACCATGTTGTCTACAATGGCTCACTCTATTTTAACAAGTATCAGAGTAATATCATCA
TCAAATACAGCTTTGATATGGGGAGAGTGCTTGCCCAACGAAGCCTGGAGTATGCTGGTTTTC
ATAATGTTTACCCCTACACATGGGGTGGATTCTCTGACATCGACCTAATGGCTGATGAAATCG
GGCTGTGGGCTGTGTATGCAACTAACCAGAATGCAGGCAATATTGTCATCAGCCAACTTAACC
AAGATACCTTGGAGGTGATGAAGAGCTGGAGCACTGGCTACCCCAAGAGAAGTGCAGGGGAAT
CTTTCATGATCTGTGGGACACTGTATGTCACCAACTCCCACTTAACTGGAGCCAAGGTGTATT
ATTCCTATTCCACCAAAACCTCCACATATGAGTACACAGACATTCCCTTCCATAACCAATACT
TTCACATATCCATGCTTGACTACAATGCAAGAGATCGAGCTCTCTATGCCTGGAACAATGGCC
ACCAGGTGCTGTTCAATGTCACCCTTTTCCATATCATCAAGACAGAGGATGACACA**TAG**GCAA
ATGTGACATGTTTTCATTGATTTAAACAGTGTGATTTGTGATAAACTCTATAAGACCCCTTCC
GTTTTTTTCTTCACTATTATTTTTCATCATTTCTCCAAAGCAAAGCATTTTTATTGTAAAGTT
GGTGTTTCAAAAACATAGCTGAGCTTGTCTAACTTACCATGTTGGAAACACATCTTAACTTCT
AAATTTACAAGGCCTATCATGTCCTTGTCATGAAAAGCACTAAAAAAAAAAAAGAGTTTAAGT
GGCTAAAGTCATAGTTTTGCAAGAGATTAATGATCTGCCTTATATTAGAGTCAGAGACTAATG
GTGGCTTAAATGCACGAATGTCTTTTTTTTTAAAACTGTCATTTTTTACTGTCTTTTGCTCCA
TCTCAGGAAATATTTTGGTAGGAATTAGGAGAACAAAAAGCACTTTTATCCCATTTATTTCTT
TAAAAAATGTAAGGATTTCATTTATATTGAAAAATAATATTAATCATTTTGCTGTTAACACAA
TTCTCTGATGCGGTGCTGTACAGTCATTTTTAAATCTCTTGCTAACATTTTATTGGCAGTATG
TATTTCTACCATTGTAACCACCATTGTGCTATTGTATCTCTTCACTTCTGTGAAAGTAATATT
TTTTATAAAANACACTGNAATTTTAAAAAAAAAAAAAAAAAACAAAAAAAAAAAAAAAAAAAAA

# FIGURE 138

MSPPLLKLGAVLSTMAMISNWMSQTLPSLVGLNTTRLSTPDTLTQISPKEGWQVYSSAQDPDG
RCICTVVAPEQNLCSRDAKSRQLRQLLEKVQNMSQSIEVLNLRTQRDFQYVLKMETQMKGLKA
KFRQIEDDRKTLMTKHFQELKEKMDELLPLIPVLEQYKTDAKLITQFKEEIRNLSAVLTGIQE
EIGAYDYEELHQRVLSLETRLRDCMKKLTCGKLMKITGPVTVKTSGTRFGAWMTDPLASEKNN
RVWYMDSYTNNKIVREYKSIADFVSGAESRTYNLPFKWAGTNHVVYNGSLYFNKYQSNIIIKY
SFDMGRVLAQRSLEYAGFHNVYPYTWGGFSDIDLMADEIGLWAVYATNQNAGNIVISQLNQDT
LEVMKSWSTGYPKRSAGESFMICGTLYVTNSHLTGAKVYYSYSTKTSTYEYTDIPFHNQYFHI
SMLDYNARDRALYAWNNGHQVLFNVTLFHIIKTEDDT

**Important features of the protein:**

**Signal peptide:**

amino acids 1-16

**N-glycosylation sites.**

amino acids 33-37, 95-99, 179-183, 299-303, 465-469

**cAMP- and cGMP-dependent protein kinase phosphorylation site.**

amino acids 215-219

**Tyrosine kinase phosphorylation site.**

amino acids 106-114

**N-myristoylation sites.**

amino acids 9-15, 31-37, 235-241, 239-245

# FIGURE 139

GAAGCAGTGCAGAGAGGAGAGCGGAGCGGAGCTGCCGCTGAGCAAAGGCCTTCACC**ATG**GCCG
AGTCCCCCGGCTGCTGCTCCGTCTGGGCCCGCTGCCTCCACTGCCTGTATAGCTGCCACTGGA
GGAAATGCCCCAGAGAGAGGATGCAAACCAGCAAGTGCGACTGTATCTGGTTTGGCCTGCTCT
TCCTCACCTTCCTCCTTTCCCTGAGCTGGCTGTACATCGGGCTCGTCCTTCTCAATGACCTGC
ACAACTTCAATGAATTCCTCTTCCGCCGCTGGGGACACTGGATGGACTGGTCCCTGGCATTCC
TGCTGGTCATCTCTCTACTGGTCACATATGCATCCTTGCTATTGGTCCTGGCCCTGCTCCTGC
GGCTTTGTAGACAGCCCCTGCATCTGCACAGCCTCCACAAGGTGCTGCTGCTCCTCATTATGC
TGCTTGTGGCGGCTGGCCTTGTGGGACTGGACATCCAATGGCAGCAGGAGTGGCATAGCTTGC
GTGTGTCACTGCAGGCCACAGCCCCATTCCTTCATATTGGAGCAGCCGCTGGAATTGCCCTCC
TGGCCTGGCCTGTGGCTGATACCTTCTACCGTATCCACCGAAGAGGTCCCAAGATTCTGCTAC
TGCTCCTATTTTTTGGAGTTGTCCTGGTCATCTACTTGGCCCCCCTATGCATCTCCTCACCCT
GCATCATGGAACCCAGAGACTTACCACCCAAGCCTGGGCTGGTGGGACACCGAGGGGCCCCCA
TGCTGGCTCCCGAGAACACCCTGATGTCCTTGCGGAAGACAGCTGAATGCGGAGCTACTGTGT
TTGAGACTGATGTGATGGTCAGCTCCGATGGGGTCCCCTTCCTCATGCATGATGAGCACCTCA
GCAGGACCACGAATGTAGCCTCTGTATTCCCAACCCGAATCACAGCCCACAGCAGTGACTTCT
CCTGGACTGAACTGAAGAGACTCAATGCTGGATCCTGGTTCCTAGAGAGGCGACCCTTCTGGG
GGGCCAAACCGCTGGCAGGCCCTGATCAGAAAGAGGCTGAGAGTCAGACGGTACCAGCATTAG
AAGAGCTATTGGAGGAAGCTGCAGCCCTCAACCTTTCCATCATGTTCGACTTGCGCCGACCCC
CACAGAACCACACATACTATGACACTTTTGTGATCCAGACATTGGAGACTGTGCTGAATGCAA
GGGTGCCCCAAGCCATGGTCTTTTGGCTACCAGATGAAGATCGGGCTAATGTCCAACGACGGG
CACCTGGAATGCGCCAGATATATGGACGTCAGGGAGGCAACAGAACGGAGAGGCCCCAGTTTC
TTAACCTCCCCTATCAAGATCTGCCACTATTGGATATCAAGGCATTGCATAAGGATAATGTCT
CGGTGAACCTATTTGTAGTGAACAAGCCCTGGCTCTTCTCTCTGCTTTGGTGTGCAGGGGTGG
ATTCGGTCACCACCAACGACTGCCAGCTGCTGCAGCAGATGCGTTACCCTATCTGGCTTATTA
CCCCTCAAACCTACCTAATCATATGGGTCATTACCAATTGTGTTTCCACCATGCTGCTTTTGT
GGACCTTCCTCCTCCAAAGGAGATTTGTTAAGAAGAGAGGGAAAACTGGCTTAGAAACAGCAG
TGCTGCTGACAAGGATCAACAATTTCATGATGGAG**TGA**ATGCCCTGCCCTGCTTCCCCACCCA
AGCCAGTCTACATTGCCCAAACAGCAAGGGTTGGAGAGTGGCTTAAGTGGAATGCTTCAGGGG
TGGTGGGTTGCAAGTGGGGGGAGCTTTGCCAACAGGAGGTTTTGAACCATGAGGGCCCTCTGC
CCAGGTGATGGGCATTCCCTAAGCTGCTATGGAATCTGCTCCCTTTGGGGTTTTGACCTGAGA
TGTTTGGGAAGAGAGTGAGTAATGAGAAGTTTCTCCTCAAATGAAACTAGAACAGAGGAAGTA
AAAGGGAGATTGCTCGGA

# FIGURE 140

MAESPGCCSVWARCLHCLYSCHWRKCPRERMQTSKCDCIWFGLLFLTFLLSLSWLYIGLVLLN
DLHNFNEFLFRRWGHWMDWSLAFLLVISLLVTYASLLLVLALLLRLCRQPLHLHSLHKVLLLL
IMLLVAAGLVGLDIQWQQEWHSLRVSLQATAPFLHIGAAAGIALLAWPVADTFYRIHRRGPKI
LLLLLFFGVVLVIYLAPLCISSPCIMEPRDLPPKPGLVGHRGAPMLAPENTLMSLRKTAECGA
TVFETDVMVSSDGVPFLMHDEHLSRTTNVASVFPTRITAHSSDFSWTELKRLNAGSWFLERRP
FWGAKPLAGPDQKEAESQTVPALEELLEEAAALNLSIMFDLRRPPQNHTYYDTFVIQTLETVL
NARVPQAMVFWLPDEDRANVQRRAPGMRQIYGRQGGNRTERPQFLNLPYQDLPLLDIKALHKD
NVSVNLFVVNKPWLFSLLWCAGVDSVTTNDCQLLQQMRYPIWLITPQTYLIIWVITNCVSTML
LLWTFLLQRRFVKKRGKTGLETAVLLTRINNFMME

**Important features of the protein:**

**Transmembrane domains:**

amino acids 38-60, 83-107, 122-138, 156-173, 189-210, 484-506

**N-glycosylation sites.**

amino acids 349-353, 362-366, 415-419, 442-446

**N-myristoylation sites.**

amino acids 163-169, 413-419, 523-529

**Leucine zipper pattern.**

amino acids 93-115, 109-131

**Glutamine amidotransferases class-II active site.**

amino acids 1-13

# FIGURE 141

GCCGCCGGCCCGGGCTGGAGCCGAGCGCAGCAGCCACCGCCGCCGCCGCGCCAGAAGTTTGGGTTGAACCGGAGC
TGCCGGGAGGAAACTTTTTTCTTTTTTCCCCCTCCCTCCCGGGAGGAGGAGGAGGAGGAGGAGGGGAAGCTGCCG
CCGGCGCCAAGGCTCGTGGGCTCGGGGTCGGCGCGGCCCGCAGAAGGGGCGGGGGCCTCGCCCCGCGAGGGGAGG
CGCGCCCCGGGGGCCCCGAGAGGGGCGGTGAGGACCGCGGGCTGCTGGTGCGGCGGCGGCGGCGCGTGTGCCCCG
CGCAGGGGAGGGCGCCCGCCCCGCTCCCGGCCCGGCTGCGAGGAGGAGGCGGCGGCGGCGCAGGAGG**<u>ATG</u>**TACTT
GGTGGCGGGGGACAGGGGGTTGGCCGGCTGCGGGCACCTCCTGGTCTCGCTGCTGGGGCTGCTGCTGCTGCTGGC
GCGCTCCGGCACCCGGGCGCTGGTCTGCCTGCCCTGTGACGAGTCCAAGTGCGAGGAGCCCAGGAACTGCCCGGG
GAGCATCGTGCAGGGCGTCTGCGGCTGCTGCTACACGTGCGCCAGCCAGAGGAACGAGAGCTGCGGCGGCACCTT
CGGGATTTACGGAACCTGCGACCGGGGGCTGCGTTGTGTCATCCGCCCCCCGCTCAATGGCGACTCCCTCACCGA
GTACGAAGCGGGCGTTTGCGAAGATGAGAACTGGACTGATGACCAACTGCTTGGTTTTAAACCATGCAATGAAAA
CCTTATTGCTGGCTGCAATATAATCAATGGGAAATGTGAATGTAACACCATTCGAACCTGCAGCAATCCCTTTGA
GTTTCCAAGTCAGGATATGTGCCTTTCAGCTTTAAAGAGAATTGAAGAAGAGAAGCCAGATTGCTCCAAGGCCCG
CTGTGAAGTCCAGTTCTCTCCACGTTGTCCTGAAGATTCTGTTCTGATCGAGGGTTATGCTCCTCCTGGGGAGTG
CTGTCCCTTACCCAGCCGCTGCGTGTGCAACCCCGCAGGCTGTCTGCGCAAAGTCTGCCAGCCGGGAAACCTGAA
CATACTAGTGTCAAAAGCCTCAGGGAAGCCGGGAGAGTGCTGTGACCTCTATGAGTGCAAACCAGTTTTCGGCGT
GGACTGCAGGACTGTGGAATGCCCTCCTGTTCAGCAGACCGCGTGTCCCCCGGACAGCTATGAAACTCAAGTCAG
ACTAACTGCAGATGGTTGCTGTACTTTGCCAACAAGATGCGAGTGTCTCTCTGGCTTATGTGGTTTCCCCGTGTG
TGAGGTGGGATCCACTCCCCGCATAGTCTCTCGTGGCGATGGGACACCTGGAAAGTGCTGTGATGTCTTTGAATG
TGTTAATGATACAAAGCCAGCCTGCGTATTTAACAATGTGGAATATTATGATGGAGACATGTTTCGAATGGACAA
CTGTCGGTTCTGTCGATGCCAAGGGGGCGTTGCCATCTGCTTCACTGCCCAGTGTGGTGAGATAAACTGCGAGAG
GTACTACGTGCCCGAAGGAGAGTGCTGCCCAGTGTGTGAAGATCCAGTGTATCCTTTTAATAATCCCGCTGGCTG
CTATGCCAATGGCCTGATCCTTGCCCACGGAGACCGGTGGCGGGAAGACGACTGCACATTCTGCCAGTGCGTCAA
CGGTGAACGCCACTGCGTTGCGACCGTCTGCGGACAGACCTGCACAAACCCTGTGAAAGTGCCTGGGGAGTGTTG
CCCTGTGTGCGAAGAACCAACCATCATCACAGTTGATCCACCTGCATGTGGGGAGTTATCAAACTGCACTCTGAC
AGGGAAGGACTGCATTAATGGTTTCAAACGCGATCACAATGGTTGTCGGACCTGTCAGTGCATAAACACCGAGGA
ACTATGTTCAGAACGTAAACAAGGCTGCACCTTGAACTGTCCCTTCGGTTTCCTTACTGATGCCCAAAACTGTGA
GATCTGTGAGTGCCGCCCAAGGCCCAAGAAGTGCAGACCCATAATCTGTGACAAGTATTGTCCACTTGGATTGCT
GAAGAATAAGCACGGCTGTGACATCTGTCGCTGTAAGAAATGTCCAGAGCTCTCATGCAGTAAGATCTGCCCCTT
GGGTTTCCAGCAGGACAGTCACGGCTGTCTTATCTGCAAGTGCAGAGAGGCCTCTGCTTCAGCTGGGCCACCCAT
CCTGTCGGGCACTTGTCTCACCGTGGATGGTCATCATCATAAAAATGAGGAGAGCTGGCACGATGGGTGCCGGGA
ATGCTACTGTCTCAATGGACGGGAAATGTGTGCCCTGATCACCTGCCCGGTGCCTGCCTGTGGCAACCCCACCAT
TCACCCTGGACAGTGCTGCCCATCATGTGCAGATGACTTTGTGGTGCAGAAGCCAGAGCTCAGTACTCCCTCCAT
TTGCCACGCCCCTGGAGGAGAATACTTTGTGGAAGGAGAAACGTGGAACATTGACTCCTGTACTCAGTGCACCTG
CCACAGCGGACGGGTGCTGTGTGAGACAGAGGTGTGCCCACCGCTGCTCTGCCAGAACCCCTCACGCACCCAGGA
TTCCTGCTGCCCACAGTGTACAGATCAACCTTTTCGGCCTTCCTTGTCCCGCAATAACAGCGTACCTAATTACTG
CAAAAATGATGAAGGGGATATATTCCTGGCAGCTGAGTCCTGGAAGCCTGACGTTTGTACCAGCTGCATCTGCAT
TGATAGCGTAATTAGCTGTTTCTCTGAGTCCTGCCCTTCTGTATCCTGTGAAAGACCTGTCTTGAGAAAAGGCCA
GTGTTGTCCCTACTGCATAGAAGACACAATTCCAAAGAAGGTGGTGTGCCACTTCAGTGGGAAGGCCTATGCCGA
CGAGGAGCGGTGGGACCTTGACAGCTGCACCCACTGCTACTGCCTGCAGGGCCAGACCCTCTGCTCGACCGTCAG
CTGCCCCCCTCTGCCCTGTGTTGAGCCCATCAACGTGGAAGGAAGTTGCTGCCCAATGTGTCCAGAAATGTATGT
CCCAGAACCAACCAATATACCCATTGAGAAGACAAACCATCGAGGAGAGGTTGACCTGGAGGTTCCCCTGTGGCC
CACGCCTAGTGAAAATGATATCGTCCATCTCCCTAGAGATATGGGTCACCTCCAGGTAGATTACAGAGATAACAG
GCTGCACCCAAGTGAAGATTCTTCACTGGACTCCATTGCCTCAGTTGTGGTTCCCATAATTATATGCCTCTCTAT
TATAATAGCATTCCTATTCATCAATCAGAAGAAACAGTGGATACCACTGCTTTGCTGGTATCGAACACCAACTAA
GCCTTCTTCCTTAAATAATCAGCTAGTATCTGTGGACTGCAAGAAAGGAACCAGAGTCCAGGTGGACAGTTCCCA
GAGAATGCTAAGAATTGCAGAACCAGATGCAAGATTCAGTGGCTTCTACAGCATGCAAAAACAGAACCATCTACA
GGCAGACAATTTCTACCAAACAGTG**<u>TGA</u>**AGAAAGGCAACTAGGATGAGGTTTCAAAAGACGGAAGACGACTAAAT
CTGCTCTAAAAAGTAAACTAGAATTTGTGCACTTGCTTAGTGGATTGTATTGGATTGTGACTTGATGTACAGCGC
TAAGACCTTACTGGGATGGGCTCTGTCTACAGCAATGTGCAGAACAAGCATTCCCACTTTTCCTCAAAAAA

# FIGURE 142

MYLVAGDRGLAGCGHLLVSLLGLLLLLARSGTRALVCLPCDESKCEEPRNCPGSIVQGVCGCC
YTCASQRNESCGGTFGIYGTCDRGLRCVIRPPLNGDSLTEYEAGVCEDENWTDDQLLGFKPCN
ENLIAGCNIINGKCECNTIRTCSNPFEFPSQDMCLSALKRIEEEKPDCSKARCEVQFSPRCPE
DSVLIEGYAPPGECCPLPSRCVCNPAGCLRKVCQPGNLNILVSKASGKPGECCDLYECKPVFG
VDCRTVECPPVQQTACPPDSYETQVRLTADGCCTLPTRCECLSGLCGFPVCEVGSTPRIVSRG
DGTPGKCCDVFECVNDTKPACVFNNVEYYDGDMFRMDNCRFCRCQGGVAICFTAQCGEINCER
YYVPEGECCPVCEDPVYPFNNPAGCYANGLILAHGDRWREDDCTFCQCVNGERHCVATVCGQT
CTNPVKVPGECCPVCEEPTIITVDPPACGELSNCTLTGKDCINGFKRDHNGCRTCQCINTEEL
CSERKQGCTLNCPFGFLTDAQNCEICECRPRPKKCRPIICDKYCPLGLLKNKHGCDICRCKKC
PELSCSKICPLGFQQDSHGCLICKCREASASAGPPILSGTCLTVDGHHHKNEESWHDGCRECY
CLNGREMCALITCPVPACGNPTIHPGQCCPSCADDFVVQKPELSTPSICHAPGGEYFVEGETW
NIDSCTQCTCHSGRVLCETEVCPPLLCQNPSRTQDSCCPQCTDQPFRPSLSRNNSVPNYCKND
EGDIFLAAESWKPDVCTSCICIDSVISCFSESCPSVSCERPVLRKGQCCPYCIEDTIPKKVVC
HFSGKAYADEERWDLDSCTHCYCLQGQTLCSTVSCPPLPCVEPINVEGSCCPMCPEMYVPEPT
NIPIEKTNHRGEVDLEVPLWPTPSENDIVHLPRDMGHLQVDYRDNRLHPSEDSSLDSIASVVV
PIIICLSIIIAFLFINQKKQWIPLLCWYRTPTKPSSLNNQLVSVDCKKGTRVQVDSSQRMLRI
AEPDARFSGFYSMQKQNHLQADNFYQTV

**Important features of the protein:**

**Signal peptide:**

amino acids 1-34

**Transmembrane domain:**

amino acids 940-962

**N-glycosylation sites.**

amino acids 71-75, 113-117, 330-334, 474-478, 746-750

**cAMP- and cGMP-dependent protein kinase phosphorylation site.**

amino acids 992-996

**N-myristoylation site.**

amino acids 9-15, 58-64, 61-67, 75-81, 79-85, 362-368, 402-408, 407-413, 439-445, 492-498, 511-517, 551-557, 558-564, 586-592, 606-612, 625-631, 845-851

**Prokaryotic membrane lipoprotein lipid attachment site.**

amino acids 52-63, 844-855

**Cell attachment sequence.**

amino acids 314-317

**Leucine zipper pattern.**

amino acids 3-25

**Eukaryotic thiol (cysteine) proteases cysteine active site.**

amino acids 57-69

**VWFC domain proteins.**

amino acids 448-456, 382-390

**C-terminal cystine knot proteins**

amino acids 60-86

# FIGURE 143

GACGTCTGGCCGGCTCCCGGCGAAGGGCAGCGGAGGAGCGGCCCAGAGCGCGCAGCTAGGGCA
CTGGCGAAACCCCGGGACAGTCCCTCTCCGTGCGGGGGCGGCGCAGAGCAGTCCCATCCCCGG
GGTCCCGGGCGCGGCTGACTGCCGGCTGGTTCCCTGCGCGCAGTAGCTCCCCGAGCCGGGCTG
CACCGGAGGCGGCGAG**ATG**GTCGCGCGCGTCGGCCTCCTGCTGCGCGCCCTGCAGCTGCTACT
GTGGGGCCACCTGGACGCCCAGCCCGCGGAGCGCGGAGGCCAGGAGCTGCGCAAGGAGGCGGA
GGCATTCCTAGAGAAGTACGGATACCTCAATGAACAGGTCCCCAAAGCTCCCACCTCCACTCG
ATTCAGCGATGCCATCAGAGCGTTTCAGTGGGTGTCCCAGCTACCTGTCAGCGGCGTGTTGGA
CCGCGCCACCCTGCGCCAGATGACTCGTCCCCGCTGCGGGGTTACAGATACCAACAGTTATGC
GGCCTGGGCTGAGAGGATCAGTGACTTGTTTGCTAGACACCGGACCAAAATGAGGCGTAAGAA
ACGCTTTGCAAAGCAAGGTAACAAATGGTACAAGCAGCACCTCTCCTACCGCCTGGTGAACTG
GCCTGAGCATCTGCCGGAGCCGGCAGTTCGGGGCGCCGTGCGCGCCGCCTTCCAGTTGTGGAG
CAACGTCTCAGCGCTGGAGTTCTGGGAGGCCCCAGCCACAGGCCCCGCTGACATCCGGCTCAC
CTTCTTCCAAGGGGACCACAACGATGGGCTGGGCAATGCCTTTGATGGCCCAGGGGGCGCCCT
GGCGCACGCCTTCCTGCCCCGCCGCGGCGAAGCGCACTTCGACCAAGATGAGCGCTGGTCCCT
GAGCCGCCGCCGCGGGCGCAACCTGTTCGTGGTGCTGGCGCACGAGATCGGTCACACGCTTGG
CCTCACCCACTCGCCCGCGCCGCGCGCGCTCATGGCGCCCTACTACAAGAGGCTGGGCCGCGA
CGCGCTGCTCAGCTGGGACGACGTGCTGGCCGTGCAGAGCCTGTATGGGAAGCCCCTAGGGGG
CTCAGTGGCCGTCCAGCTCCCAGGAAAGCTGTTCACTGACTTTGAGACCTGGGACTCCTACAG
CCCCCAAGGAAGGCGCCCTGAAACGCAGGGCCCTAAATACTGCCACTCTTCCTTCGATGCCAT
CACTGTAGACAGGCAACAGCAACTGTACATTTTTAAAGGGAGCCATTTCTGGGAGGTGGCAGC
TGATGGCAACGTCTCAGAGCCCCGTCCACTGCAGGAAAGATGGGTCGGGCTGCCCCCCAACAT
TGAGGCTGCGGCAGTGTCATTGAATGATGGAGATTTCTACTTCTTCAAAGGGGGTCGATGCTG
GAGGTTCCGGGGCCCCAAGCCAGTGTGGGGTCTCCCACAGCTGTGCCGGGCAGGGGGCCTGCC
CCGCCATCCTGACGCCGCCCTCTTCTTCCCTCCTCTGCGCCGCCTCATCCTCTTCAAGGGTGC
CCGCTACTACGTGCTGGCCCGAGGGGGACTGCAAGTGGAGCCCTACTACCCCCGAAGTCTGCA
GGACTGGGGAGGCATCCCTGAGGAGGTCAGCGGCGCCCTGCCGAGGCCCGATGGCTCCATCAT
CTTCTTCCGAGATGACCGCTACTGGCGCCTCGACCAGGCCAAACTGCAGGCAACCACCTCGGG
CCGCTGGGCCACCGAGCTGCCCTGGATGGGCTGCTGGCATGCCAACTCGGGGAGCGCCCTGTT
C**TGA**AGGCACCTCCTCACCTCAGAAACTGGTGGTGCTCTCAGGGCAAAATCATGTTCCCCACC
CCCGGGGCAGAACCCCTCTTAGAAGCCTCTGAGTCCCTCTGCAGAAGACCGGGCAGCAAAGCC
TCCATCTGGAAGTCTGTCTGCCTTTGTTCCTTGGAAAAAAAAAAAAAAAAAAAAAAAAAAAAA
AAAAAAAAAAAAAAAAAAAAAAAAAAAAAA

# FIGURE 144

MVARVGLLLRALQLLLWGHLDAQPAERGGQELRKEAEAFLEKYGYLNEQVPKAPTSTRFSDAI
RAFQWVSQLPVSGVLDRATLRQMTRPRCGVTDTNSYAAWAERISDLFARHRTKMRRKKRFAKQ
GNKWYKQHLSYRLVNWPEHLPEPAVRGAVRAAFQLWSNVSALEFWEAPATGPADIRLTFFQGD
HNDGLGNAFDGPGGALAHAFLPRRGEAHFDQDERWSLSRRRGRNLFVVLAHEIGHTLGLTHSP
APRALMAPYYKRLGRDALLSWDDVLAVQSLYGKPLGGSVAVQLPGKLFTDFETWDSYSPQGRR
PETQGPKYCHSSFDAITVDRQQQLYIFKGSHFWEVAADGNVSEPRPLQERWVGLPPNIEAAAV
SLNDGDFYFFKGGRCWRFRGPKPVWGLPQLCRAGGLPRHPDAALFFPPLRRLILFKGARYYVL
ARGGLQVEPYYPRSLQDWGGIPEEVSGALPRPDGSIIFFRDDRYWRLDQAKLQATTSGRWATE
LPWMGCWHANSGSALF

**Important features of the protein:**

**Signal peptide:**

amino acids 1-22

**N-glycosylation sites.**

amino acids 164-168, 355-359

**N-myristoylation sites.**

amino acids 92-98, 153-159, 193-199, 202-208, 288-294, 368-374, 509-515

**Amidation site.**

amino acids 312-316

**Neutral zinc metallopeptidases, zinc-binding region signature.**

amino acids 237-247

**Matrixins cysteine switch**

amino acids 231-262, 271-284

**Hemopexin domain protein**

amino acids 66-108, 231-262

# FIGURE 145

GCCGGCTAGGGCGCCGGAGCCGCACGCAGCCGCGGGGCTCCGAGAGGCGCGCACTGGGGCTGGGACTGCGCGGCG
CCGCCGCTGCGAGCGCCACTGAGCGGTCGCGCAACTTCGGAGGCACAGCGCCGGAGCCAGGCGAGCGCTCAGAGA
CCCGGAGCCAGAGGGGCGCGCCGGAGCCTCGTTCGAGAGCCGGCGCCAGGCACCCACCGCGCTCCGAGTGCCAGG
CGGCCCTCCGCGCAGCGTGGCTTCCGCTGCCCCCACGGAAGGCACGGGCTGGCGCTGCCGGGCGCCGGGGAGGAC
GGCGAGGAGGAGGCGGCGGCGGCGGAGACGGCGGCGGCGAGACTGGGGCCAGGGAGACAGCCCTGGGGGAGAGGC
GCCCGAACCAGGCCGCGGGAGC**ATG**GGGGCCCGGAGCGGAGCTCGGGGCGCGCTGCTGCTGGCACTGCTGCTCTG
CTGGGACCCGAGGCTGAGCCAAGCAGGCACTGATTCTGGCAGCGAGGTGCTCCCTGACTCCTTCCCGTCAGCGCC
AGCAGAGCCGCTGCCCTACTTCCTGCAGGAGCCACAGGACGCCTACATTGTGAAGAACAAGCCTGTGGAGCTCCG
CTGCCGCGCCTTCCCCGCCACACAGATCTACTTCAAGTGCAACGGCGAGTGGGTCAGCCAGAACGACCACGTCAC
ACAGGAAGGCCTGGATGAGGCCACCGGCCTGCGGGTGCGCGAGGTGCAGATCGAGGTGTCGCGGCAGCAGGTGGA
GGAGCTCTTTGGGCTGGAGGATTACTGGTGCCAGTGCGTGGCCTGGAGCTCCGCAGGCACCACCAAGAGTCGCCG
AGCCTACGTCCGCATCGCCTACCTGCGCAAGAACTTCGATCAGGAGCCTCTGGGCAAGGAGGTGCCCCTGGACCA
TGAGGTTCTCCTGCAGTGCCGCCCGCCGGAGGGGGTGCCTGTGGCCGAGGTGGAATGGCTCAAGAATGAGGATGT
CATCGACCCCACCCAGGACACCAACTTCCTGCTCACCATCGACCACAACCTCATCATCCGCCAGGCCCGCCTGTC
GGACACTGCCAACTATACCTGCGTGGCCAAGAACATCGTGGCCAAACGCCGGAGCACCACTGCCACCGTCATCGT
CTACGTGAATGGCGGCTGGTCCAGCTGGGCAGAGTGGTCACCCTGCTCCAACCGCTGTGGCCGAGGCTGGCAGAA
GCGCACCCGGACCTGCACCAACCCCGCTCCACTCAACGGAGGGGCCTTCTGCGAGGGCCAGGCATTCCAGAAGAC
CGCCTGCACCACCATCTGCCCAGTCGATGGGGCGTGGACGGAGTGGAGCAAGTGGTCAGCCTGCAGCACTGAGTG
TGCCCACTGGCGTAGCCGCGAGTGCATGGCGCCCCCACCCCAGAACGGAGGCCGTGACTGCAGCGGGACGCTGCT
CGACTCTAAGAACTGCACAGATGGGCTGTGCATGCAAAATAAGAAAACTCTAAGCGACCCCAACAGCCACCTGCT
GGAGGCCTCAGGGGATGCGGCGCTGTATGCGGGGCTCGTGGTGGCCATCTTCGTGGTCGTGGCAATCCTCATGGC
GGTGGGGGTGGTGGTGTACCGCCGCAACTGCCGTGACTTCGACACAGACATCACTGACTCATCTGCTGCCCTGAC
TGGTGGTTTCCACCCCGTCAACTTTAAGACGGCAAGGCCCAGCAACCCGCAGCTCCTACACCCCTCTGTGCCTCC
TGACCTGACAGCCAGCGCCGGCATCTACCGCGGACCCGTGTATGCCCTGCAGGACTCCACCGACAAAATCCCCAT
GACCAACTCTCCTCTGCTGGACCCCTTACCCAGCCTTAAGGTCAAGGTCTACAGCTCCAGCACCACGGGCTCTGG
GCCAGGCCTGGCAGATGGGGCTGACCTGCTGGGGGTCTTGCCGCCTGGCACATACCCTAGCGATTTCGCCCGGGA
CACCCACTTCCTGCACCTGCGCAGCGCCAGCCTCGGTTCCCAGCAGCTCTTGGGCCTGCCCCGAGACCCAGGGAG
CAGCGTCAGCGGCACCTTTGGCTGCCTGGGTGGGAGGCTCAGCATCCCCGGCACAGGGGTCAGCTTGCTGGTGCC
CAATGGAGCCATTCCCCAGGGCAAGTTCTACGAGATGTATCTACTCATCAACAAGGCAGAAAGTACCCTCCCGCT
TTCAGAAGGGACCCAGACAGTATTGAGCCCCTCGGTGACCTGTGGACCCACAGGCCTCCTGCTGTGCCGCCCCGT
CATCCTCACCATGCCCCACTGTGCCGAAGTCAGTGCCCGTGACTGGATCTTTCAGCTCAAGACCCAGGCCCACCA
GGGCCACTGGGAGGAGGTGGTGACCCTGGATGAGGAGACCCTGAACACACCCTGCTACTGCCAGCTGGAGCCCAG
GGCCTGTCACATCCTGCTGGACCAGCTGGGCACCTACGTGTTCACGGGCGAGTCCTATTCCCGCTCAGCAGTCAA
GCGGCTCCAGCTGGCCGTCTTCGCCCCCGCCCTCTGCACCTCCCTGGAGTACAGCCTCCGGGTCTACTGCCTGGA
GGACACGCCTGTAGCACTGAAGGAGGTGCTGGAGCTGGAGCGGACTCTGGGCGGATACTTGGTGGAGGAGCCGAA
ACCGCTAATGTTCAAGGACAGTTACCACAACCTGCGCCTCTCCCTCCATGACCTCCCCCATGCCCATTGGAGGAG
CAAGCTGCTGGCCAAATACCAGGAGATCCCCTTCTATCACATTTGGAGTGGCAGCCAGAAGGCCCTCCACTGCAC
TTTCACCCTGGAGAGGCACAGCTTGGCCTCCACAGAGCTCACCTGCAAGATCTGCGTGCGGCAAGTGGAAGGGGA
GGGCCAGATATTCCAGCTGCATACCACTCTGGCAGAGACACCTGCTGGCTCCCTGGACACTCTCTGCTCTGCCCC
TGGCAGCACTGTCACCACCCAGCTGGGACCTTATGCCTTCAAGATCCCACTGTCCATCCGCCAGAAGATATGCAA
CAGCCTAGATGCCCCCAACTCACGGGGCAATGACTGGCGGATGTTAGCACAGAAGCTCTCTATGGACCGGTACCT
GAATTACTTTGCCACCAAAGCGAGCCCCACGGGTGTGATCCTGGACCTCTGGGAAGCTCTGCAGCAGGACGATGG
GGACCTCAACAGCCTGGCGAGTGCCTTGGAGGAGATGGGCAAGAGTGAGATGCTGGTGGCTGTGGCCACCGACGG
GGACTGC**TGA**GCCTCCTGGGACAGCGGGCTGGCAGGGACTGGCAGGAGGCAGGTGCAGGGAGGCCTGGGGCAGCC
TCCTGATGGGGATGTTTGGCCTCTGCTTCCTCCCAGTTCACAGCCAGAGTTGCCTCTCCTCCTCCTCTTCCCCAA
CCCCCAGACCATGACCAGCCTTAGAAAATCCATGTACTCTGTTGTTAGAGGGCCCAGAGTTCCTTCTCCACCCCC
GCTCTCTCTCTCTTGGCCTGAGATCTCTGTGCAGGAACCAAGATGGGGCTGAAGCCTCTGGAGGCAGTTGGTTGG
GGGCGGGCAGGCAGGAGGCCCTCCCTCCACCCCCCCACCCTCAGCCCGGCAACTTCTGGGTTCCGTGGGTTTTAG
TTCCGTTCTTCGTTTTCTTCCTCCGTTATTGATTTCTCCTTTCTCCCTAAGCCCCCTTCTGCTTCCACGCCCTTT
TCCTCTTTGAAGAGTCAAGTACAATTCAGACAAACTGCTTTCTCCTGTCCAAAAGCAAAAAGGCAAAGGAAAGAA
AGAAAGCTTCAGACCGCTAGTAAGGCTCAAAGAAGAAGAAAAACACCAAAACCACAAGGGAAAAGAAAAACCCAG
TTTCTTAGGAAACGCAAACGATTTATTATCCAGATTATTTGGATAAGTCCTTTTTAAAA

# FIGURE 146

MGARSGARGALLLALLLCWDPRLSQAGTDSGSEVLPDSFPSAPAEPLPYFLQEPQDAYIVKNK
PVELRCRAFPATQIYFKCNGEWVSQNDHVTQEGLDEATGLRVREVQIEVSRQQVEELFGLEDY
WCQCVAWSSAGTTKSRRAYVRIAYLRKNFDQEPLGKEVPLDHEVLLQCRPPEGVPVAEVEWLK
NEDVIDPTQDTNFLLTIDHNLIIRQARLSDTANYTCVAKNIVAKRRSTTATVIVYVNGGWSSW
AEWSPCSNRCGRGWQKRTRTCTNPAPLNGGAFCEGQAFQKTACTTICPVDGAWTEWSKWSACS
TECAHWRSRECMAPPPQNGGRDCSGTLLDSKNCTDGLCMQNKKTLSDPNSHLLEASGDAALYA
GLVVAIFVVVAILMAVGVVVYRRNCRDFDTDITDSSAALTGGFHPVNFKTARPSNPQLLHPSV
PPDLTASAGIYRGPVYALQDSTDKIPMTNSPLLDPLPSLKVKVYSSSTTGSGPGLADGADLLG
VLPPGTYPSDFARDTHFLHLRSASLGSQQLLGLPRDPGSSVSGTFGCLGGRLSIPGTGVSLLV
PNGAIPQGKFYEMYLLINKAESTLPLSEGTQTVLSPSVTCGPTGLLLCRPVILTMPHCAEVSA
RDWIFQLKTQAHQGHWEEVVTLDEETLNTPCYCQLEPRACHILLDQLGTYVFTGESYSRSAVK
RLQLAVFAPALCTSLEYSLRVYCLEDTPVALKEVLELERTLGGYLVEEPKPLMFKDSYHNLRL
SLHDLPHAHWRSKLLAKYQEIPFYHIWSGSQKALHCTFTLERHSLASTELTCKICVRQVEGEG
QIFQLHTTLAETPAGSLDTLCSAPGSTVTTQLGPYAFKIPLSIRQKICNSLDAPNSRGNDWRM
LAQKLSMDRYLNYFATKASPTGVILDLWEALQQDDGDLNSLASALEEMGKSEMLVAVATDGDC

**Important features of the protein:**

**Signal peptide:**

amino acids 1-26

**Transmembrane domain:**

amino acids 374-395

**N-glycosylation sites.**

amino acids 222-225, 347-350

**Glycosaminoglycan attachment site.**

amino acids 492-495

**cAMP- and cGMP-dependent protein kinase phosphorylation site.**

amino acids 233-236, 234-237

**Casein kinase II phosphorylation sites.**

amino acids 30-33, 87-90, 251-254, 341-344, 359-362, 629-632, 651-654, 706-709, 757-760, 827-830, 925-928, 941-944

**Tyrosine kinase phosphorylation sites.**

amino acids 216-223, 773-780

**N-myristoylation sites.**

amino acids 2-7, 6-11, 27-32, 96-101, 137-142, 179-184, 247-252, 281-286, 334-339, 379-384, 491-496, 495-500, 509-514, 542-547, 547-552, 550-555, 553-558, 560-565, 611-616, 785-790, 834-839, 844-849

**Prokaryotic membrane lipoprotein lipid attachment site.**

amino acids 541-551

**ATP/GTP-binding site motif A (P-loop).**

amino acids 926-933

**Growth factor and cytokines receptors family signature 2.**

amino acids 306-312

# FIGURE 147

GAGAGGGACAGAGGCTGGAGAAGGATGTATGGCCTGCCCTGGGCTTGTCTGTTCCCTCCTGAGCCTGAGCCCCTT
ACCTTCCTGACCCC**ATG**AAGCACACACTGGCTCTGCTGGCTCCCCTGCTGGGCCTGGGCCTGGGGCTGGCCCTGA
GTCAGCTGGCTGCAGGGGCCACAGACTGCAAGTTCCTTGGCCCGGCAGAGCACCTGACATTCACCCCAGCAGCCA
GGGCCCGGTGGCTGGCCCCTCGAGTTCGTGCGCCAGGACTCCTGGACTCCCTCTATGGCACCGTGCGCCGCTTCC
TCTCGGTGGTGCAGCTCAATCCTTTCCCTTCAGAGTTGGTAAAGGCCCTACTGAATGAGCTGGCCTCCGTGAAGG
TGAATGAGGTGGTGCGGTACGAGGCGGGCTACGTGGTATGCGCTGTGATCGCGGGCCTCTACCTGCTGCTGGTGC
CCACTGCCGGGCTTTGCTTCTGCTGCTGCCGCTGCCACCGGCGCTGCGGGGGACGAGTGAAGACAGAGCACAAGG
CGCTGGCCTGTGAGCGCGCGGCCCTCATGGTCTTCCTGCTGCTGACCACCCTCTTGCTGCTGATTGGTGTGGTCT
GTGCCTTTGTCACCAACCAGCGCACGCATGAACAGATGGGCCCCAGCATCGAGGCCATGCCTGAGACCCTGCTCA
GCCTCTGGGGCCTGGTCTCTGATGTCCCCCAAGAGCTGCAGGCCGTGGCACAGCAATTCTCCCTGCCCCAGGAGC
AAGTCTCAGAGGAGCTGGATGGTGTTGGTGTGAGCATTGGGAGCGCGATCCACACTCAGCTCAGGAGCTCCGTGT
ACCCCTTGCTGGCGGCCGTGGGCAGTTTGGGCCAGGTCCTGCAGGTCTCCGTGCACCACCTGCAAACCTTGAATG
CTACAGTGGTAGAGCTGCAGGCCGGGCAGCAGGACCTGGAGCCAGCCATCCGGGAACACCGGGACCGCCTCCTTG
AGCTGCTGCAGGAGGCCAGGTGCCAGGGAGATTGTGCAGGGGCCCTGAGCTGGGCCCGCACCCTGGAGCTGGGTG
CTGACTTCAGCCAGGTGCCCTCTGTGGACCATGTCCTGCACCAGCTAAAAGGTGTCCCCGAGGCCAACTTCTCCA
GCATGGTCCAGGAGGAGAACAGCACCTTCAACGCCCTTCCAGCCCTGGCTGCCATGCAGACATCCAGCGTGGTGC
AAGAGCTGAAGAAGGCAGTGGCCCAGCAGCCGGAAGGGGTGAGGACACTGGCTGAAGGGTTCCCGGGCTTGGAGG
CAGCTTCCCGCTGGGCCCAGGCACTGCAGGAGGTGGAGGAGAGCAGCCGCCCCTACCTGCAGGAGGTGCAGAGAT
ACGAGACCTACAGGTGGATCGTGGGCTGCGTGCTGTGCTCCGTGGTCCTATTCGTGGTGCTCTGCAACCTGCTGG
GCCTCAATCTGGGCATCTGGGGCCTGTCTGCCAGGGACGACCCCAGCCACCCAGAAGCCAAGGGCGAGGCTGGAG
CCCGCTTCCTCATGGCAGGTGTGGGCCTCAGCTTCCTCTTTGCTGCACCCCTCATCCTCCTGGTGTTCGCCACCT
TCCTGGTGGGTGGCAACGTGCAGACGCTGGTGTGCCGGAGCTGGGAGAACGGCGAGCTCTTTGAGTTTGCAGACA
CCCCAGGGAACCTGCCCCCGTCCATGAACCTGTCGCAACTTCTTGGCCTGAGGAAGAACATCAGCATCCACCAAG
CCTATCAGCAGTGCAAGGAAGGGGCAGCGCTCTGGACAGTCCTGCAGCTCAACGACTCCTACGACCTGGAGGAGC
ACCTGGATATCAACCAGTATACCAACAAGCTACGGCAGGAGTTGCAGAGCCTGAAAGTAGACACACAGAGCCTGG
ACCTGCTGAGCTCAGCCGCCCGCCGGGACCTGGAGGCCCTGCAGAGCAGTGGGCTTCAGCGCATCCACTACCCCG
ACTTCCTCGTTCAGATCCAGAGGCCCGTGGTGAAGACCAGCATGGAGCAGCTGGCCCAGGAGCTGCAAGGACTGG
CCCAGGCCCAAGACAATTCTGTGCTGGGGCAGCGGCTGCAGGAGGAGGCCCAAGGACTCAGAAACCTTCACCAGG
AGAAGGTCGTCCCCCAGCAGAGCCTTGTGGCAAAGCTCAACCTCAGCGTCAGGGCCCTGGAGTCCTCTGCCCCGA
ATCTCCAGCTGGAGACCTCAGATGTCCTAGCCAATGTCACCTACCTGAAAGGAGAGCTGCCTGCCTGGGCAGCCA
GGATCCTGAGGAATGTGAGTGAGTGTTTCCTGGCCCGGGAGATGGGCTACTTCTCCCAGTACGTGGCCTGGGTGA
GAGAGGAGGTGACTCAGCGCATTGCCACCTGCCAGCCCCTCTCCGGAGCCCTGGACAACAGCCGTGTGATCCTGT
GTGACATGATGGCTGACCCCTGGAATGCCTTCTGGTTCTGCCTGGCATGGTGCACCTTCTTCCTGATCCCCAGCA
TCATCTTTGCCGTCAAGACCTCCAAATACTTCCGTCCTATCCGGAAACGCCTCAGCTCCACCAGCTCTGAGGAGA
CTCAGCTCTTCCACATCCCCCGGGTTACCTCCCTGAAGCTG**TAG**GGCCTTGTGGGGTGAGGTGACCCTGAGGCTG
CCTGTCCTCCCCTTTGATTTAGCCTGGGCCACAGGACTTCGGTAGCTCTTGCCCCAGAGCCCAGGCTGGCATCCA
GGCCTGGACTGTCCCCAGTTCCGGCTTACCTGGCCCCACCTTGCCTGCTCCTTTCCACCCCTTTCTGCTCACGAC
CCCCATCATTCACGCTCAGAATCACATGGGACTTCTGTGCAGCTGCAGAGCCAGCAAGTCCCTACAGGTGTCACC
CGTTACCCCCATGCTGGTGGCATCCTCACAGGAAGAGCCTGTTCTCCACCTGCTGGAGCCTGGACCCTGGGGTGG
GACAGAGGCCTCGTCCAACCCCACTCCCCTTCCCGTGTGTCTTCCCCCTGCCAAGCCTCCCCCTGCCAAGCCTCC
CCCTGCCCCTCTCTGAGCCCCTCGCCCCCCACACCGTCCTCATCTGGCCTCCCCCCTGGCCCCCACTTCCCTCTT
ATGCCCTTCCTGGCCCTTTGCTTCCTCCCTTAGTCCCCTCTTCACCATATCTCCACTGCTACCTTGCTGGCCCCA
GAGACCACCCTGCCCAACCAAACCACTCAGGTAACGCCACTAATCAGGCAGGGGCCACCATGGCCTAGGTCTGGG
CTGGCTGCAGGCCCTGCCTCATGGCCTCTGAGCCCTCCACTGCCCCAGGGCCTTGGGCCCTCTGCAGATCTCATC
CAGGATTTATTGTTGTCCAGTGGGGTGAGGGAGGCCTGTCTGAAGGCCGAGCCTCCCTGCCTGCACCCAAGTTAG
AAATGGGGGTACCAGCACTTAGCTTCTCTCTGAGTGCTGGCTCCCAAGGAAGGGACCTGGGACCTGGGCCACAGT
GGGGGCTTGCCCTTACCTCTTCAGAAGGAAGCATCTTCCACAGCCCCCACCCAACTTTCTTAGGAGTGATCTGGT
GGCCAGAACAGGATTTTGCACGGCCCCTTTTATCCTGCGCATGTGGCCTAGGGTCATCCCCAGCCCATCCCTGTG
TCAGCCCTGAGTGCTGGACACTGCGTTCCAGAAATGAGGAAGAGGAGAGAGAAGAGATGGACAGACCTCAGATCC
ATTAAAGTGTTCTCACTTCAAAAAAAAAAAAAAAAAAAAAAAAAAAAAAAAAAAAAAAA

# FIGURE 148

MKHTLALLAPLLGLGLGLALSQLAAGATDCKFLGPAEHLTFTPAARARWLAPRVRAPGLL
DSLYGTVRRFLSVVQLNPFPSELVKALLNELASVKVNEVVRYEAGYVVCAVIAGLYLLLV
PTAGLCFCCCRCHRRCGGRVKTEHKALACERAALMVFLLLTTLLLLIGVVCAFVTNQRTH
EQMGPSIEAMPETLLSLWGLVSDVPQELQAVAQQFSLPQEQVSEELDGVGVSIGSAIHTQ
LRSSVYPLLAAVGSLGQVLQVSVHHLQTLNATVVELQAGQQDLEPAIREHRDRLLELLQE
ARCQGDCAGALSWARTLELGADFSQVPSVDHVLHQLKGVPEANFSSMVQEENSTFNALPA
LAAMQTSSVVQELKKAVAQQPEGVRTLAEGFPGLEAASRWAQALQEVEESSRPYLQEVQR
YETYRWIVGCVLCSVVLFVVLCNLLGLNLGIWGLSARDDPSHPEAKGEAGARTLMAGVGL
SFLFAAPLILLVFATFLVGGNVQTLVCRSWENGELFEFADTPGNLPPSMNLSQLLGLRKN
ISIHQAYQQCKEGAALWTVLQLNDSYDLEEHLDINQYTNKLRQELQSLKVDTQSLDLLSS
AARRDLEALQSSGLQRIHYPDFLVQIQRPVVKTSMEQLAQELQGLAQAQDNSVLGQRLQE
EAQGLRNLHQEKVVPQQSLVAKLNLSVRALESSAPNLQLETSDVLANVTYLKGELPAWAA
RILRNVSECFLAREMGYFSQYVAWVREEVTQRIATCQPLSGALDNSRVILCDMMADPWNA
FWFCLAWCTFFLIPSIIFAVKTSKYFRPIRKRLSSTSSEETQLFHIPRVTSLKL

**Signal peptide:**

amino acids 1-17

**Transmembrane domain:**

amino acids 105-125, 153-173, 428-449, 476-500, 778-797

**N-glycosylation sites:**

amino acids 270-273, 343-347, 352-356, 530-534, 540-546, 563-567, 684-688, 707-711, 725-729

**cAMP- and cGMP-dependent protein kinase phosphorylation site.**

amino acids 811-815

**Tyrosine kinase phosphorylation site.**

amino acids 95-103

**N-myristoylation sites.**

amino acids 13-19, 15-21, 17-23, 26-32, 58-64, 124-130, 168-174, 228-234, 230-236, 320-326, 338-344, 393-399, 429-435, 446-452, 477-483, 500-506, 536-542, 644-650, 761-767

**Phospholipase A2 histidine active site.**

aminop acids 129-137

**4Fe-4S ferredoxins, iron-sulfur binding region signature.**

amino acids 126-138

**Mitochondrial energy transfer proteins signature.**

amino acids 80-89

# FIGURE 149

CACAGCTCCCTTCCCAGGACGTGAAAATCTGCCTTCTCACC**ATG**AGGCTTCTAGTCCTTTCCA
GCCTGCTCTGTATCCTGCTTCTCTGCTTCTCCATCTTCTCCACAGAAGGGAAGAGGCGTCCTG
CCAAGGCCTGGTCAGGCAGGAGAACCAGGCTCTGCTGCCACCGAGTCCCTAGCCCCAACTCAA
CAAACCTGAAAGGACATCATGTGAGGCTCTGTAAACCATGCAAGCTTGAGCCAGAGCCCCGCC
TTTGGGTGGTGCCTGGGGCACTCCCACAGGTG**TAG**CACTCCCAAAGCAAGACTCCAGACAGCG
GAGAACCTCATGCCTGGCACCTGAGGTACCCAGCAGCCTCCTGTCTCCCCTTTCAGCCTTCAC
AGCAGTGAGCTGCAATGTTGGAGGGCTTCATCTCGGGCTGCAAGGACCCTGGGAAAGTTCCAG
AACTCCACGTCCTTGTCTCAATTGTGCCATCAACTTTCAGAGCTATCATGAGCCAACCTCACC
CCACAGGGCCTCAGTCGCCACCATGTGGGCCTCTCCAGTGCAAACCACCGAGCATTCCACCAT
GACCGGTCACAGCTACAAATCCAGAGACCATCAATCCTGCTAGAGTGCAGGGTGGCAAGCACC
CAAGGGTGGCTGACCAAGACTGCAGAGTCTCCTCCATCTTCAGGTCCATTCAGCCTCCTGGCA
TTTAACTACCAGCATCCAGTGGTCCCCAAGGAATCCCTTCCTAGCCTCCTGACATGAGTCTGC
TGGAAAGAGCATCCAAACAAACAAGTAATAAATAAATAAATAAACTCA

# FIGURE 150

MRLLVLSSLLCILLLCFSIFSTEGKRRPAKAWSGRRTRLCCHRVPSPNSTNLKGHHVRLCKPC
KLEPEPRLWVVPGALPQV

**Important features of the protein:**

**Signal peptide:**

amino acids 1-21

**N-glycosylation site.**

amino acids 48-52

**Amidation sites.**

amino acids 23-27, 33-37

# FIGURE 151

CACCGGAGGGCACGCAGCTGACGGAGCTGCGCTGCGTTCGCCTCGTTTGCCTCGCGCCCTCCA
CTGGAGCTGTTCGCGCCTCCCGGCTCCCACCGCAGCCCACCCGGCAGAGGAGTCGCTACCAGC
GCCCAGTGCGCTCTGTCAGTCCGCAAACTCCTTGCCGCCCGCCCCGGGCTGGGCACCAAATAC
CAGGCTACC**ATG**GTCTACAAGACTCTCTTCGCTCTTTGCATCTTAACTGCAGGATGGAGGGTA
CAGAGTCTGCCTACATCAGCTCCTTTGTCTGTTTCTCTTCCGACAAACATTGTACCACCGACC
ACCATCTGGACTAGCTCTCCACAAAACACTGATGCAGACACTGCCTCCCCATCCAACGGCACT
CACAACAACTCGGTGCTCCCAGTTACAGCATCAGCCCCAACATCTCTGCTTCCTAAGAACATT
TCCATAGAGTCCAGAGAAGAGGAGATCACCAGCCCAGGTTCGAATTGGGAAGGCACAAACACA
GACCCCTCACCTTCTGGGTTCTCGTCAACAAGCGGTGGAGTCCACTTAACAACCACGTTGGAG
GAACACAGCTCGGGCACTCCTGAAGCAGGCGTGGCAGCTACACTGTCGCAGTCCGCTGCTGAG
CCTCCCACACTCATCTCCCCTCAAGCTCCAGCCTCATCACCCTCATCCCTATCAACCTCACCA
CCTGAGGTCTTTTCTGCCTCCGTTACTACCAACCATAGCTCCACTGTGACCAGCACCCAACCC
ACTGGAGCTCCAACTGCACCAGAGTCCCCGACAGAGGAGTCCAGCTCTGACCACACACCCACT
TCACATGCCACAGCTGAGCCAGTGCCCCAGGAGAAAACACCCCCAACAACTGTGTCAGGCAAA
GTGATGTGTGAGCTCATAGACATGGAGACCACCACCACCTTTCCCAGGGTGATCATGCAGGAA
GTAGAACATGCATTAAGTTCAGGCAGCATCGCCGCCATTACCGTGACAGTCATTGCCGTGGTG
CTGCTGGTGTTTGGAGTTGCAGCCTACCTAAAAATCAGGCATTCCTCCTATGGAAGACTTTTG
GACGACCATGACTACGGGTCCTGGGGAAACTACAACAACCCTCTGTACGATGACTCC**TAA**CAA
TGGAATATGGCCTGGGATGAGGATTAACTGTTCTTTATTTATAAGTGCTTATCCAGTAGAATT
AATAAGTACCTGATGCGCATTGAACGACAATCTTAAGCCCTGTTTTGTTGGTATGGTTGTTTT
TGTTTTCCTCCCTCTCCTCTGGCTGCTACAACTTCCCCTTTCTGGTACAAGAAGAACCATTCT
TTAAAGGTGAGTGGAGGCTGATTTGCAGCTGAAGTGGGCCAGCCTTGCACCAGCCAGGCCAGA
CCACCATGGTGAAGGCTTCTTTCCCCACTGCAGGACCCACTTTGAGAAGGATCGAGGAGGAGG
ATTTGGGTTGTTTTGTTAGGGGTTACTTTCAGGGGAACATTTCATTTGTGTTATTTCTTAAAC
TTCTATTTAGGAAATTACATTAAGTATTAATGAGGGGAAAGGAAATGAGCTCTACGAGGATTT
CACCTTGCATGGGAGAGAGCAGGGTTTTCTCAGATTCCTTTTTAATCTCTATTTATCTGGTTG
TTTCTGACAGGATGCTGCCTGCTTGGCTCTACGAGCTGGAAAGCAGCTTCTTAGCTGCCTAAT
TAATGAAAGATGAAAATAGGAAGTGCCCTGGAGGGGGCCAGCAGGTCACGGGGCAGAATCTCT
CAGGTTGCTGTGGGATCTCAGTGTGCCCCTACCTGTTCTCCCCTCCAGGCCACCTGTCTCTGT
AAAGGATGTCTGCTCTGTTCAAAAGGCAGCTGGGATCCCAGCCCACAAGTGATCAGCAGAGTT
GCATTTCCAAAGAAAAAGGCTATGAGATGAGCTGAGTTATAGAGAGAAAGGGAGAGGCATGTA
CGGTGTGGGGAAGTGGAAGAGAAGCTGGCGGGGGAGAAGGAGGCTAACCTGCACTGAGTACTT
CATTAGGACAAGTGAGAATCAGCTATTGATAATGGCCAGAGATATCCACAGCTTGGAGGAGCC
CAGAGACTGTTTGCTTTATACCCACACAGCAACTGGTCCACTGCTTTACTGTCTGTTGGATAA
TGGCTGTAAAATGTTTAAAAAC

# FIGURE 152

MVYKTLFALCILTAGWRVQSLPTSAPLSVSLPTNIVPPTTIWTSSPQNTDADTASPSNGTHNN
SVLPVTASAPTSLLPKNISIESREEEITSPGSNWEGTNTDPSPSGFSSTSGGVHLTTTLEEHS
SGTPEAGVAATLSQSAAEPPTLISPQAPASSPSSLSTSPPEVFSASVTTNHSSTVTSTQPTGA
PTAPESPTEESSSDHTPTSHATAEPVPQEKTPPTTVSGKVMCELIDMETTTTFPRVIMQEVEH
ALSSGSIAAITVTVIAVVLLVFGVAAYLKIRHSSYGRLLDDHDYGSWGNYNNPLYDDS

**Important features of the protein:**

**Signal peptide:**

amino acids 1-20

**Transmembrane domain:**

amino acids 258-278

**N-glycosylation sites.**

amino acids 58-61, 62-65, 80-83, 176-179

**Casein kinase II phosphorylation sites.**

amino acids 49-52, 85-88, 95-98, 100-103, 120-123, 121-124, 141-144, 164-167, 191-194, 195-198, 200-203

**Tyrosine kinase phosphorylation site.**

amino acids 289-296

**N-myristoylation sites.**

amino acids 59-64, 115-120, 128-133, 133-138, 257-262, 297-302

# FIGURE 153

ACGTCACTGTCTTGAAGCAGCAGTAGCCTGGGAAGTGAGGCAGGAGGAATTGAGAGGCAGGAA
GGGNGCTGGAGACACAGCTGAGCCTGGAAATGAGAGTGGGCATCGCCGTGGTCATCATGACTC
CTCTGCGGCGTGGTCACC**ATG**TTGGTTCACTGTGTTGGGCTCTTATTGACGGGTCTCCTGCTA
GGCCTGACCTTGGGTGCCGGAGCCCTGCTGGCTTCTGAGCCTATCTACCAACCACCTTCAGCC
TGGGTGCCAGCTGGGGGGCTGGTGGGGCTGGCGCTGCTGGGAGCCCTGCTCACACTTCGGTGG
CCACGTCCATTCACAGTTCTGGGCACAACCCTGCTGGGTTCTGCAGTGCTTGTGGCCTGTGTT
GACTACTTCCTGGAGGGGCTGGCACTGGGGAGTTGGCTGGGCCAACGCCTGCAGACACTTCCA
GCCTTGCCTTCTCTCTGC**TGA**TATAGCTGGGTCTTACTGGGGATCTGGCCAGCCTTGGGGGCC
CTTGGAGCCCTGGCCCAGTGGAAGCTCGTGCCTGAGGAACATGGAGGCCACGCTAATGGGTCT
GTTCCTGGTTTCCCAGATGCATAAAGGAAGACATATCCCTCCCCTGGGCAGCAAGGCTACAAT
GGGAGGGAGGGAGAACATGGGAGCATGTGAATAAAATGGCATTAAATACTGAAAAAAAAAAAA
AAAAAAAAAAAAAAAAAAAAAAAAAAAAAAAAAAAAAAAAAAAAAAAAAAAAAAAAAAAAAA

# FIGURE 154

MLVHCVGLLLTGLLLGLTLGAGALLASEPIYQPPSAWVPAGGLVGLALLGALLTLRWPRPFTV
LGTTLLGSAVLVACVDYFLEGLALGSWLGQRLQTLPALPSLC

**Signal peptide:**

amino acids 1-20

**Transmembrane domain:**

amino acids 38-55, 60-78

**N-myristoylation sites.**

amino acids 7-13, 12-18, 16-22, 22-28, 41-47, 50-56, 84-90, 88-94

**Prokaryotic membrane lipoprotein lipid attachment site.**

amino acids 67-78

# FIGURE 155

TGCAATTAAAGGAGTCGGGTCTCTAACTGTTGATCTGTTTTTTCCCTTCTGAGCA**ATG**GAGC
TTACCATCTTTATCCTGAGACTGGCCATTTACATCCTGACATTTCCCTTGTACCTGCTGAACT
TTCTGGGCTTGTGGAGCTGGATATGCAAAAAATGGTTCCCCTACTTCTTGGTGAGGTTCACTG
TGATATACAACGAACAGATGGCAAGCAAGAAGCGGGAGCTCTTCAGTAACCTGCAGGAGTTTG
CGGGCCCCTCCGGGAAACTCTCCCTGCTGGAAGTGGGCTGTGGCACGGGGGCCAACTTCAAGT
TCTACCCACCTGGGTGCAGGGTGACCTGTATTGACCCCAACCCCAACTTTGAGAAGTTTTTGA
TCAAGAGCATTGCAGAGAACCGACACCTGCAGTTTGAGCGCTTTGTGGTAGCTGCCGGGGAGA
ACATGCACCAGGTGGCTGATGGCTCTGTGGATGTGGTGGTCTGCACCCTGGTGCTGTGCTCTG
TGAAGAACCAGGAGCGGATTCTCCGCGAGGTGTGCAGAGTGCTGAGACCGGGAGGGGCTTTCT
ATTTCATGGAGCATGTGGCAGCTGAGTGTTCGACTTGGAATTACTTCTGGCAACAAGTCCTGG
ATCCTGCCTGGCACCTTCTGTTTGATGGGTGCAACCTGACCAGAGAGAGCTGGAAGGCCCTGG
AGCGGGCCAGCTTCTCTAAGCTGAAGCTGCAGCACATCCAGGCCCCACTGTCCTGGGAGTTGG
TGCGCCCTCATATCTATGGATATGCTGTGAAA**TAG**TGTGAGCTGGCAGTTAAGAGCTGAATGG
CTCAAAGAATTTAAAGCTTCAGTTTTACATTTAAAATGCTAAGTGGGAGAAGAGAAACCTTTT
TTTTGGGGGGCGGTTTTTTTGGTTTGTTGTTGGTTTTTTTTTTTTTTTGGCAGGAGAATCTC
TTGAACCCAGAAGGCGAAGGTTGCAGTGAACCGAGATCATGCCATTGTACTCTAGCCTGGGTG
ACAAGAGCAAGACTCCGTCTCAAAAAAAAAAAAAAAAAAAAAAAAGAAGTAGAGACAGGGAGAC
GGGGTCTCACTGTGTTGCCTAGGCCGGTCTTGAACTCCTGGGCTCAAGTGATTCTCCCACCTT
GACCTCCTAAATTGTTGGGATTACAGGTGTGAGACAGTGCACCTGGCCGAAATAGCTCAAGTT
TCTGAAAAACAAATCTGAATCTATTTGTTATTCTTAGCGTCACTGGTCTGGCTTTCAGAATTA
ACATACAAGGTTGCCACACCTAGTTCTGCCCAGCTTTATGTCTTTTATTCCAGTATTCCACCA
AAGTTTGTTTTCCTGCATTCCAGTTCTCAAGTCTTAAGATAAAGATTGTACTTGACAGTTTAG
TATATCCATAAAACTATTTGAGGTGGTTAAGGTTCTTGGGTTCATTTTCCTTAATACTTTGCT
GAATATTGTAGATTGTAGGCAATGAAAAAGTCTACTAAATTAGGAAAACCTTGAATAATTAGG
TATCCTAGGTAAGAGCCCCTAAACATCAAGCAATCTGTGAGTCTGTAAAGAAATAAATATTTT
TTGGATTATTCTTATCTAATTCCACCCCTGTTGGAAGATGATTTCTTTGTTCTTTGCAACTAT
GGAAGCTGTGAAAATCATCACAAGTGCCTCTGAAAGCGAGTGTTAGGTTGGTTAGAGGGTTTA
ATATTTTCTGCAATGGTTTGTAGGAATTTTAATAAATGTAGTATATTTTCTGAGATGATTTTG
TAAAAGTACTATTTTAAATATCAAATCAACCAATAAATTCACATTTGTGTTAGGAACAAAA

# FIGURE 156

MELTIFILRLAIYILTFPLYLLNFLGLWSWICKKWFPYFLVRFTVIYNEQMASKKRELFSNLQ
EFAGPSGKLSLLEVGCGTGANFKFYPPGCRVTCIDPNPNFEKFLIKSIAENRHLQFERFVVAA
GENMHQVADGSVDVVVCTLVLCSVKNQERILREVCRVLRPGGAFYFMEHVAAECSTWNYFWQQ
VLDPAWHLLFDGCNLTRESWKALERASFSKLKLQHIQAPLSWELVRPHIYGYAVK

**Signal peptide:**

amino acids 1-29

**N-glycosylation site.**

amino acids 203-207

**N-myristoylation sites.**

amino acids 78-84, 80-86, 91-97, 201-207

# FIGURE 157

CCGCTGAGATGTACGAACTTCCGGTTCTCCGGGCAGCTGCCACTGCTGTAGCTTCTGCCACCT
GCCACGACCGGGCCTCTCCCTGGCGTTTGGTCACCTCTGCTTCATTCTCCACCGCGCCTATGG
TCCCTCTTGGAGCCAGCGTGGCGGGCCTGGCGGCTCCCGGGTGGTGAGAGAGCGGTCCGGGAA
CG**ATG**AAGGCCTCGCAGTGCTGCTGCTGTCTCAGCCACCTCTTGGCTTCCGTCCTCCTCCTGC
TGTTGCTGCCTGAACTAAGCGGGCCCCTGGCAGTCCTGCTGCAGGCAGCCGAGGCCGCGCCAG
GTCTTGGGCCTCCTGACCCTAGACCACGGACATTACCGCCGCTGCCACCGGGCCCTACCCCTG
CCCAGCAGCCGGGCCGTGGTCTGGCTGAAGCTGCGGGGCCGCGGGGCTCCGAGGGAGGCAATG
GCAGCAACCCTGTGGCCGGGCTTGAGACGGACGATCACGGAGGGAAGGCCGGGGAAGGCTCGG
TGGGTGGCGGCCTTGCTGTGAGCCCCAACCCTGGCGACAAGCCCATGACCCAGCGGGCCCTGA
CCGTGTTGATGGTGGTGAGCGGCGCGGTGCTGGTGTACTTCGTGGTCAGGACGGTCAGGATGA
GAAGAAGAAACCGAAAGACTAGGAGATATGGAGTTTTGGACACTAACATAGAAAATATGGAAT
TGACACCTTTAGAACAGGATGATGAGGATGATGACAACACGTTGTTTGATGCCAATCATCCTC
GAAGA**TAA**GAATGTGCCTTTTGATGAAAGAACTTTATCTTTCTACAATGAAGAGTGGAATTTC
TATGTTTAAGGAATAAGAAGCCACTATATCAATGTTGGGGGGTATTTAAGTTACATATATTT
TAACAACCTTTAATTTGCTGTTGCAATAAATACCGTATCCTTTTATTATATCTTTATATGTAT
AGAAGTACTCTATTAATGGGCTCAGAGATGTTGGGATAAAGTATACTGTAATAATTTATCTG
TTTGAAAATTACTATAAAACGGTGTTTTCTGGTCGGTTTTTGTTTCCTGCTTACCATATGATT
GTAAATTGTTTTATGTATTAATCAGTTAATGCTAATTATTTTTGCTGATGTCATATGTTAAAG
AGCTATAAATTCCAACAACCAACTGGTGTGTAAAAATAATTTAAAATTTCCTTTACTGAAAGG
TATTTCCCATTTTTGTGGGGAAAAGAAGCCAAATTTATTACTTTGTGTTGGGGTTTTTAAAAT
ATTAAGAAATGTCTAAGTTATTGTTTGCAAAACAATAAATATGATTTTAAATTCTCTTAAAAA
AAAAA

# FIGURE 158

MKASQCCCCLSHLLASVLLLLLLPELSGPLAVLLQAAEAAPGLGPPDPRPRTLPPLPPGPTPA
QQPGRGLAEAAGPRGSEGGNGSNPVAGLETDDHGGKAGEGSVGGGLAVSPNPGDKPMTQRALT
VLMVVSGAVLVYFVVRTVRMRRRNRKTRRYGVLDTNIENMELTPLEQDDEDDDNTLFDANHPRR

**Signal peptide:**
amino acids 1-28

**Transmembrane domain:**
amino acids 124-140

**N-glycosylation site.**
amino acids 83-87

**N-myristoylation sites.**
amino acids 69-75, 78-84, 81-87, 97-103, 103-109, 106-112, 157-160

# FIGURE 159

GCTGCAGGCGGCGACGGCTACACC**ATG**GGCCGGCTGCTGCGGGCCGCCCGGCTGCCGCCGCTG
CTTTCGCCGCTGCTGCTTCTGCTGGTTGGGGGAGCGTTCCTGGGTGCCTGTGTGGCTGGGTCT
GATGAGCCTGGCCCAGAGGGCCTCACCTCCACCTCCCTGCTAGACCTCCTGCTGCCCACTGGC
TTGGAGCCACTGGACTCAGAGGAGCCTAGTGAGACCATGGGCCTGGGAGCTGGGCTGGGAGCC
TCTGGCTCAGGCTTCCCCAGCGAAGAGAATGAAGAGTCTCGGATTCTGCAGCCACCACAGTAC
TTCTGGGAAGAGGAGGAAGAGCTGAATGACTCAAGTCTGGACCTGGGACCCACTGCAGATTAT
GTTTTTCCTGACTTAACTGAGAAGGCAGGTTCCATTGAAGACACTAGCCAGGCTCAAGAGCTG
CCAAACCTCCCCTCTCCCTTGCCCAAGATGAATCTGGTTGAGCCTCCCTGGCATATGCCTCCC
AGAGAGGAGGAAGAAGAGGAAGAGGAAGAGGAGGAGAGGGAGAAGGAAGAGGTAGAGAAACAA
GAGGAGGAGGAAGAGGAGGAGCTGCTCCCTGTGAATGGATCCCAAGAAGAAGCCAAGCCTCAG
GTCCGTGACTTTTCTCTCACCAGCAGCAGCCAGACCCCAGGGGCCACCAAAAGCAGGCATGAA
GACTCCGGGGACCAGGCCTCATCAGGTGTGGAGGTGGAGAGCAGCATGGGGCCCAGCTTGCTG
CTGCCTTCAGTCACCCCAACTACAGTGACTCCGGGGGACCAGGACTCCACCAGCCAAGAGGCA
GAGGCCACAGTGCTGCCAGCTGCAGGGCTTGGGGTAGAGTTCGAGGCTCCTCAGGAAGCAAGC
GAGGAAGCCACTGCAGGAGCAGCTGGTTTGTCTGGCCAGCACGAGGAGGTGCCGGCCTTGCCT
TCATTCCCTCAAACCACAGCTCCCAGTGGGGCCGAGCACCCAGATGAAGATCCCCTTGGCTCT
AGAACCTCAGCCTCTTCCCCACTGGCCCCTGGAGACATGGAACTGACACCTTCCTCTGCTACC
TTGGGACAAGAAGATCTCAACCAGCAGCTCCTAGAAGGGCAGGCAGCTGAAGCTCAATCCAGG
ATACCCTGGGATTCTACGCAGGTGATCTGCAAGGACTGGAGCAATCTGGCTGGGAAAAACTAC
ATCATTCTGAACATGACAGAGAACATAGACTGTGAGGTGTTCCGGCAGCACCGGGGGCCACAG
CTCCTGGCCCTGGTGGAAGAGGTGCTGCCCCGCCATGGCAGTGGCCACCATGGGGCCTGGCAC
ATCTCTCTGAGCAAGCCCAGCGAGAAGGAGCAGCACCTTCTCATGACACTGGTGGGCGAGCAG
GGGGTGGTGCCCACTCAAGATGTCCTTTCCATGCTGGGTGACATCCGCAGGAGCCTGGAGGAG
ATTGGCATCCAGAACTATTCCACAACCAGCAGCTGCCAGGCGCGGGCCAGCCAGGTGCGCAGC
GACTACGGCACGCTCTTCGTGGTGCTGGTGGTCATTGGGGCCATCTGCATCATCATCATTGCG
CTTGGCCTGCTCTACAACTGCTGGCAGCGCCGGCTGCCCAAGCTCAAGCACGTGTCGCACGGC
GAGGAGCTGCGCTTCGTGGAGAACGGCTGCCACGACAACCCCACGCTGGACGTGGCCAGCGAC
AGCCAGTCGGAGATGCAGGAGAAGCACCCCAGCCTGAACGGCGGCGGGGCCCTCAACGGCCCG
GGGAGCTGGGGGGCGCTCATGGGGGGCAAGCGGGACCCCGAGGACTCGGACGTGTTCGAGGAG
GACACGCACCTG**TGA**GCGCAGCCGAGGCGCAGGCCGAGTGGGCCGCCAGGACCAAGCGAGGTG
GACCCCGAAACGGACGGCCCGGAGCCCGCACCAGCCCCGCGCCTACCCGGGCCGCCCCCGCGG
CCTGGCCCTCGGCGCGGGCTCCTTCCCGCTTCCCCCGACTTCACACGGCGGCTTCGGACCAAC
TCCCTCACTCCCGCCCGAGGGGCAGGCCTCAAAGCCCGCCTTGGCCCCGCTTTCCCGCCCCTG
AACCCCGGCCCCGCGGGCGGCGGGCGGCGCTTCCTGCGCCCCGGGACTCAATTAAACCCGCCC
GGAGACCACGCCGGGCCCAGCAAAA

# FIGURE 160

MGRLLRAARLPPLLSPLLLLLVGGAFLGACVAGSDEPGPEGLTSTSLLDLLLPTGLEPLDSEE
PSETMGLGAGLGASGSGFPSEENEESRILQPPQYFWEEEEELNDSSLDLGPTADYVFPDLTEK
AGSIEDTSQAQELPNLPSPLPKMNLVEPPWHMPPREEEEEEEEEEEEREKEEVEKQEEEEEEL
LPVNGSQEEAKPQVRDFSLTSSSQTPGATKSRHEDSGDQASSGVEVESSMGPSLLLPSVTPTT
VTPGDQDSTSQEAEATVLPAAGLGVEFEAPQEASEEATAGAAGLSGQHEEVPALPSFPQTTAP
SGAEHPDEDPLGSRTSASSPLAPGDMELTPSSATLGQEDLNQQLLEGQAAEAQSRIPWDSTQV
ICKDWSNLAGKNYIILNMTENIDCEVFRQHRGPQLLALVEEVLPRHGSGHHGAWHISLSKPSE
KEQHLLMTLVGEQGVVPTQDVLSMLGDIRRSLEEIGIQNYSTTSSCQARASQVRSDYGTLFVV
LVVIGAICIIIIALGLLYNCWQRRLPKLKHVSHGEELRFVENGCHDNPTLDVASDSQSEMQEK
HPSLNGGGALNGPGSWGALMGGKRDPEDSDVFEEDTHL

**Signal peptide:**

amino acids 1-29

**Transmembrane domain:**

amino acids 499-521

**N-glycosylation sites.**

amino acids 106-110, 193-197, 395-399, 480-484

**Glycosaminoglycan attachment site.**

amino acids 77-81

**N-myristoylation sites.**

amino acids 24-30, 28-34, 41-47, 69-75, 71-77, 73-79, 75-81, 216-222, 327-333, 455-461, 519-525, 574-580, 581-587, 584-590

**Amidation site.**

amino acids 588-592

# FIGURE 161

CCAGGGCGGAGCGCAGCTGCGCCGGGCTTGGGCGCCTGGGGCCGCCGCTCCCCACCGTCGTTT
TCCCCACCGAGGCCGAGGCGTCCCGGAGTC**ATG**GCCGGCCTGAACTGCGGGGTCTCTATCGCA
CTGCTAGGGGTTCTGCTGCTGGGTGCGGCGCGCCTGCCGCGCGGGGCAGAAGCTTTTGAGATT
GCTCTGCCACGAGAAAGCAACATTACAGTTCTCATAAAGCTGGGGACCCCGACTCTGCTGGCA
AAACCCTGTTACATCGTCATTTCTAAAAGACATATAACCATGTTGTCCATCAAGTCTGGAGAA
AGAATAGTCTTTACCTTTAGCTGCCAGAGTCCTGAGAATCACTTTGTCATAGAGATCCAGAAA
AATATTGACTGTATGTCAGGCCCATGTCCTTTTGGGGAGGTTCAGCTTCAGCCCTCGACATCG
TTGTTGCCTACCCTCAACAGAACTTTCATCTGGGATGTCAAAGCTCATAAGAGCATCGGTTTA
GAGCTGCAGTTTTCCATCCCTCGCCTGAGGCAGATCGGTCCGGGTGAGAGCTGCCCAGACGGA
GTCACTCACTCCATCAGCGGCCGAATCGATGCCACCGTGGTCAGGATCGGAACCTTCTGCAGC
AATGGCACTGTGTCCCGGATCAAGATGCAAGAAGGAGTGAAAATGGCCTTACACCTCCCATGG
TTCCACCCCAGAAATGTCTCCGGCTTCAGCATTGCAAACCGCTCATCTATAAAACGTCTGTGC
ATCATCGAGTCTGTGTTTGAGGGTGAAGGCTCAGCAACCCTGATGTCTGCCAACTACCCAGAA
GGCTTCCCTGAGGATGAGCTCATGACGTGGCAGTTTGTCGTTCCTGCACACCTGCGGGCCAGC
GTCTCCTTCCTCAACTTCAACCTCTCCAACTGTGAGAGGAAGGAGGAGCGGGTTGAATACTAC
ATCCCGGGCTCCACCACCAACCCCGAGGTGTTCAAGCTGGAGGACAAGCAGCCTGGGAACATG
GCGGGGAACTTCAACCTCTCTCTGCAAGGCTGTGACCAAGATGCCCAAAGTCCAGGGATCCTC
CGGCTGCAGTTCCAAGTTTTGGTCCAACATCCACAAAATGAAAGCAGTGAG**TGA**GCCCCACTT
TCCTTTTTCTTCCTCCTCCAGCACCTTCGTTGTTTCCTGGGTAGTCTGCCTGGGTGAGGCTCC
CTTCCTGTTTCTCATCTGTGGCTTCTGAAACACTTAGACTCTGGACCCAGCAAGAGTTTCAGG
AAGTGGGTTGCTAGGCAGTTAGACAGGCTTGTTGGTGAACACCCGGTATGTAGTTCCATTTCA
GCACAATAAAAAGAAATCTTGCATTCAAGATGCTAAATTGTTTTTAACGAAAA

# FIGURE 162

MAGLNCGVSIALLGVLLLGAARLPRGAEAFEIALPRESNITVLIKLGTPTLLAKPCYIVISKR
HITMLSIKSGERIVFTFSCQSPENHFVIEIQKNIDCMSGPCPFGEVQLQPSTSLLPTLNRTFI
WDVKAHKSIGLELQFSIPRLRQIGPGESCPDGVTHSISGRIDATVVRIGTFCSNGTVSRIKMQ
EGVKMALHLPWFHPRNVSGFSIANRSSIKRLCIIESVFEGEGSATLMSANYPEGFPEDELMTW
QFVVPAHLRASVSFLNFNLSNCERKEERVEYYIPGSTTNPEVFKLEDKQPGNMAGNFNLSLQG
CDQDAQSPGILRLQFQVLVQHPQNESSE

**Signal peptide:**
amino acids 1-29

**N-glycosylation sites.**
amino acids 39-43, 122-126, 180-184, 205-209, 213-217, 270-274, 310-314, 339-343

**Tyrosine kinase phosphorylation site.**
amino acids 276-284

**N-myristoylation sites.**
amino acids 3-9, 7-13, 158-164, 175-181, 191-197, 303-309

# FIGURE 163

CAACCACACACCTGGGGAATTGCTGGCCTGACTTCTGACCCCTGACTCCTCATACCCTTCCTC
CAGAGCATGACATTTGACCACCAACTGAAACCTGACCTCTGACCCCAGACCACTGGCCCTTCC
CCCGCCCTGTGGTGACTTCATAAAGGTTACTAGCTTCTCCCCTGGCCTTGAGACCCACACG**AT**
**G**GCCCTGCTGGCTCTGGCCAGTGCCGTCCCCTCTGCCCTGCTGGCCCTGGCTGTCTTCAGGGT
GCCCGCCTGGGCCTGTCTCCTCTGCTTCACAACCTACTCTGAGCGCCTCCGCATCTGCCAGAT
GTTTGTTGGGATGCGGAGCCCCAAGCTTGAAGAGTGTGAGGAGGCCTTCACGGCCGCCTTCCA
GGGCCTCTCTGACACCGAAATCAGTGAGGAGACCATCCACACTTCATCAGTGTCCTGGGGAAG
GTGCAGAGGGAGGGCAGGAGAGGCCCAGAGGGTCAGGCTGAGGGACAGACAGAGAGAAACAGT
CAGAGGAGAAAGGCTCAAAGACCATGAGAACAACAGAGACTTAGGGACAGAGAGACACAGACA
GGGGAAGACAGCAGGGCAAAGACTCAGAGAGGGGAGGATGGAGAGTCAGAGAGGGGAAGATGG
AGACTCAGAGAGAGGGGAGGATGGAGACTCAGAGAGAGAGGAAGATGGAGACTCAGAGGGAAA
GATGGAGACTCAGGAGTATGGAGAGTCAGAGAGGGGAGGATGGACACTCAGGGGAGGATGGAG
AGTCAGGAGGATGGAGACTCATAGAAAGGGGAGGATGGAGAGTCAGGAGAGGTTGGAGACTGG
AGAGGGAATAGAGACCCAGAAAGGGGAGGATGGAGACTCAGAGGGTGGAAGATGGAGACTCAA
AGAGGATGGAAACCCAGAGAGAGGAGGACAGAGA**TGA**GGCAGAGACTAGGGGAAGCAGGATAG
CGACTGGTCGGGGGCAGAGACTCAGGGAGGATAGAGACTCACAGAGAGGTGAGGATAGAGACT
TGGGAGGGACTCAGGAAGCATAGCGACTGTGGGGCAAAGAGTCAGAGAGGGGAGGATACAGAC
TTGGGAGGGCAGAGACTCAGAAACAGAATGTTCGCATTAGGGACATGGTGTTGCGGGGAGCTG
CCTCCCCCAGCCCCTGCTCCCTCCCTCACCGCCAGACTATGATGAGAGAAGCCACCTGCATGA
CACCTTCACCCAGATGACCCATGCCCTGCAGGAGCTGGCTGCTGCCCAGGGATCCTTTGAGGT
TGCCTTCCCTGATGCTGCAGAGAAAATGAAGAAGGTCATTACACAGCTTAAAGAAGCCCAGGC
TTGCATCCCTCCCTGCGGTCTCCAGGAGTTCGCCCGGCGTTTCCTCTGCAGCGGGTGCTACTC
TAGGGTCTGCGACCTCCCGCTGGACTGCCCAGTTCAGGATGTGACAGTGACTCGGGGCGACCA
GGCTATGTTTTCTTGCATCGTAAACTTCCAGCTGCCAAAGGAGGAGATCACCTATTCCTGGAA
GTTCGCAGGAGGAGGTCTCCGGACTCAGGACTTGTCCTATTTCCGAGATATGCCGCGGGCCGA
AGGATACCTGGCGCGGATCCGGCCGGCTCAGCTCACGCACCGCGGGACGTTCTCCTGCGTGAT
CAAGCAAGACCAGCGCCCCCTGGCCCGGCTCTACTTCTTTCTTAACGTCCTCGGGGCCCTCGC
ATCAGCGAGTGCGACAGTGTTGGCGTGGTGAGTTCTGGGGACTCCGGAGCCCCAGCATCTAGC
TCCCCGCTGTCTCAGATCCCACCGAGAAGTCTGGGTTCCCAGCAACCTCCAACCCAGGAGGAT
GTTCTTTCGATGGTACTGCAGTGGCAACTAACAAAGGTATCTTTCCTCCTTCCCTATCCTATT
TCCATCCTGAAAATAAAGAATATATTTCAACTCTAAAAAAAAAAAAAAAAAAAAAAAAAAAAA
AAAAAAAAAAAAAAA

# FIGURE 164

MALLALASAVPSALLALAVFRVPAWACLLCFTTYSERLRICQMFVGMRSPKLEECEEAFTAAF
QGLSDTEISEETIHTSSVSWGRCRGRAGEAQRVRLRDRQRETVRGERLKDHENNRDLGTERHR
QGKTAGQRLREGRMESQRGEDGDSERGEDGDSEREEDGDSEGKMETQEYGESERGGWTLRGGW
RVRRMETHRKGRMESQERLETGEGIETQKGEDGDSEGGRWRLKEDGNPERGGQR

**Signal peptide:**
amino acids 1-26

**N-myristoylation site.**
amino acids 65-71

# FIGURE 165

CAGAATCGCAGATTGCCAGCCCTTTTCCCGACCCCTACGGAAAGACGAGTCCAGGGGCCGTCC
TGGCGAGGTCAAAACATTTAGTCTGGTCTTTTCAGCGTGGACCCTGCCAGCAGCCAGGCC**ATG**
GAGCTCTCTGATGTCACCCTCATTGAGGGTGTGGGTAATGAGGTGATGGTGGTGGCAGGTGTG
GTGGTGCTGATTCTAGCCTTGGTCCTAGCTTGGCTCTCTACCTACGTAGCAGACAGCGGTAGC
AACCAGCTCCTGGGCGCTATTGTGTCAGCAGGCGACACATCCGTCCTCCACCTGGGGCATGTG
GACCACCTGGTGGCAGGCCAAGGCAACCCCGAGCCAACTGAACTCCCCCATCCATCAGAGGGT
AATGATGAGAAGGCTGAAGAGGCGGGTGAAGGTCGGGGAGACTCCACTGGGGAGGCTGGAGCT
GGGGGTGGTGTTGAGCCCAGCCTTGAGCATCTCCTTGACATCCAAGGCCTGCCCAAAAGACAA
GCAGGTGCAGGCAGCAGCAGTCCAGAGGCCCCCCTGAGATCTGAGGATAGCACCTGCCTCCCT
CCCAGCCCTGGCCTCATCACTGTGCGGCTCAAATTCCTCAATGATACCGAGGAGCTGGCTGTG
GCTAGGCCAGAGGATACCGTGGGTGCCCTGAAGAGCAAATACTTCCCTGGACAAGAAAGCCAG
ATGAAACTGATCTACCAGGGCCGCCTGCTACAAGACCCAGCCCGCACACTGCGTTCTCTGAAC
ATTACCGACAACTGTGTGATTCACTGCCACCGCTCACCCCCAGGGTCAGCTGTTCCAGGCCCC
TCAGCCTCCTTGGCCCCCTCGGCCACTGAGCCACCCAGCCTTGGTGTCAATGTGGGCAGCCTC
ATGGTGCCTGTCTTTGTGGTGCTGTTGGGTGTGGTCTGGTACTTCCGAATCAATTACCGCCAA
TTCTTCACAGCACCTGCCACTGTCTCCCTGGTGGGAGTCACCGTCTTCTTCAGCTTCCTAGTA
TTTGGGATGTATGGACGA**TAA**GGACATAGGAAGAAAATGAAAGGCATGGTCTTTCTCCTTTAT
GGCCTCCCCACTTTTCCTGGCCAGAGCTGGGCCCAAGGGCCGGGGAGGGAGGGGTGGAAAGGA
TGTGATGGAAATCTCCTCCATAGGACACAGGAGGCAAGTATGCGGCCTCCCCTTCTCATCCAC
AGGAGTACAGATGTCCCTCCCGTGCGAGCACAACTCAGGTAGAAATGAGGATGTCATCTTCCT
TCACTTTTAGGGTCCTCTGAAGGAGTTCAAAGCTGCTGGCCAAGCTCAGTGGGGAGCCTGGGC
TCTGAGATTCCCTCCCACCTGTGGTTCTGACTCTTCCCAGTGTCCTGCATGTCTGCCCCCAGC
ACCCAGGGCTGCCTGCAAGGGCAGCTCAGCATGGCCCCAGCACAACTCCGTAGGGAGCCTGGA
GTATCCTTCCATTTCTCAGCCAAATACTCATCTTTTGAGACTGAAATCACACTGGCGGGAATG
AAGATTGTGCCAGCCTTCTCTTATGGGCACCTAGCCGCCTTCACCTTCTTCCTCTACCCCTTA
GCAGGAATAGGGTGTCCTCCCTTCTTTCAAAGCACTTTGCTTGCATTTTATTTTATTTTTTTA
AGAGTCCTTCATAGAGCTCAGTCAGGAAGGGGATGGGGCACCAAGCCAAGCCCCCAGCATTGG
GAGCGGCCAGGCCACAGCTGCTGCTCCCGTAGTCCTCAGGCTGTAAGCAAGAGACAGCACTGG
CCCTTGGCCAGCGTCCTACCCTGCCCAACTCCAAGGACTGGGTATGGATCGCTGGGCCCTAGG
CTCTTGCTTCTGGGGCTATTGGAGGGTCAGTGTCTGTGACTGAATAAAGTTCCATTTTGTGGA
AAAAAAAAAAAAAAAAAAAAAAAAAAAAAAAAAAAAAAAAAAAAAAAA

# FIGURE 166

MELSDVTLIEGVGNEVMVVAGVVVLILALVLAWLSTYVADSGSNQLLGAIVSAGDTSVLHLGH
VDHLVAGQGNPEPTELPHPSEGNDEKAEEAGEGRGDSTGEAGAGGGVEPSLEHLLDIQGLPKR
QAGAGSSSPEAPLRSEDSTCLPPSPGLITVRLKFLNDTEELAVARPEDTVGALKSKYFPGQES
QMKLIYQGRLLQDPARTLRSLNITDNCVIHCHRSPPGSAVPGPSASLAPSATEPPSLGVNVGS
LMVPVFVVLLGVVWYFRINYRQFFTAPATVSLVGVTVFFSFLVFGMYGR

**Signal peptide:**

amino acids 1-36

**Transmembrane domains:**

amino acids 246-267, 275-301

**N-glycosylation sites.**

amino acids 162-166, 211-215

**N-myristoylation sites.**

amino acids 48-54, 105-111, 109-115, 129-135, 177-183, 247-253

**Cell attachment sequence.**

amino acids 97-100

# FIGURE 167

GGCGGCTGTGTGTCGCCGGAGCCGAAGCGCGCAGGCCCGTCCCGGTGGCCGGGGAGCGGGCGGGTGGGGGCGCC**A**
**TG**TGGTTCATGTACCTGCTGAGCTGGCTGTCGCTCTTCATCCAGGTGGCCTTCATCACGCTGGCTGTCGCGGCTG
GACTCTATTACCTGGCAGAACTGATAGAAGAATACACAGTGGCCACCAGCAGGATCATAAAATACATGATCTGGT
TCTCCACCGCTGTACTGATTGGCCTCTACGTCTTTGAGCGCTTCCCCACCAGCATGATTGGAGTGGGCCTATTCA
CCAACCTCGTCTACTTTGGCCTCCTCCAGACCTTCCCCTTCATCATGCTGACCTCGCCTAACTTCATCCTGTCGT
GTGGACTAGTGGTGGTGAATCATTACCTAGCATTTCAGTTTTTGCAGAAGAATATTATCCCTTCTCAGAGGTCC
TGGCCTATTTCACTTTCTGCCTGTGGATAATTCCGTTTGCGTTTTTGTGTCACTTTCGGCCGGGGAGAACGTCC
TGCCCTCTACCATGCAGCCAGGAGATGATGTCGTCTCCAATTATTTCACCAAAGGCAAGCGGGGCAAACGCTTAG
GGATCCTGGTTGTCTTCTCCTTCATCAAAGAGGCCATTCTACCCAGTCGTCAGAAGATATAC**TGA**CCCCCATGCA
GGCAGGATGTGGGGGGCAAGATCAGGAGAGTCAGGCCCCTGGGCCTCTATGCCAGGTGGGGACCAGAAGTCGGGA
AGGCACCTACCACCTGCCCTGGCTTTCTTCCCCTCAACTCTGGAGCCCCATCCCCACCCTCCTTGGGGGGCTCAG
CTTGGCTCAGATCTGATGCTTCAAGAGGCTGTAACCTCAGAGGGCACCAAGGAGGGTGGCAGAGCCTGCTTAGCC
AGGAGGCCGAGGTCCCTCAGTCCTCCCCTGTCCCTTCCAAGGTGGGTCAGGAGGTTCTGGCCCCGCTGGGGCAGG
CAGGGCAGGGTCTGTGAAGCTTAAGAGCAGATGGTGACAAGTTCTCTGGGCAGGTGGCCATGGGGAGGGGCCATG
GCTTGGCATGTCCAACAGAAATAGTTTTTGCTGTTGAACGGTGATTTCTGTCCAAGTGCAGATTTCCGTTTGAAT
AAAGCTTCGCTTCTAGGTGGCACTGTTTGCCTTAATACCCTGACAGTTCATCTTCCTTTCTTCCTGCTAACCTTC
TGCTCTGGACTGGACTCACTTTTCTGCTCCAGGGACTCCTTTTCTGGGTTTGGGTCTTGCCCTTCCCAAGGGACT
GTTCTTGTGGCCCTTAATGGGAAGGGGGCAGGGGTGAGGAGCTGAGCCTGCTCAAGGAGTGGGAAGTGGGGCTAT
AGGCAGCCTCTCTGATGCACTCTCTTCCATCTCTTTCCCCAAGGCTCCGTGACTGTCAAACTGGGAGTAGGAGAG
GGGACAATTTAGGACTGGGCTAGATTTTCAGAAGAACATCTACAATATCCTATTTATAAATCTTCCTCTGGGAAA
AGGAGTGGTTTCTGGCTGAATACTATCTTAGGCTCAAGGAGAAACAAAATAAAAATTAGCTTCCAGGCAGCCTGT
TTTTAAAGAAATGGACTAATGGGAGAAGCTGTTTGTCACTCTAAGAGCATCCAAGCCCTGGCCCGTCTGTGCAC
TCTTGGCTCCTGGGGAGATATATCTGCCTTCTAAGAAGGCAGGCCAGGTCTTGGGCACAGACCTGCATTTGTTGA
CCTTGCACTCCAACTATAGTGCCTTGCAAGTGCTCAACAGTACATATTGGAATGAAGTCCCTATGAGAGCCATTT
CTGGCCATGTTCTATACCTCAAAGTGAGGCTGGCAGGTACAGAGATGAACTGTACACATGTGATACATTTAAGCC
ACTGGAAAAACCCCTGTGCTTGAAAATATTTCCTCTATATCATGCCTGGAGTTCCATCATAGCCCTTCATTTCCT
TGGCTTTAGCATTTACCTTCTCTTAAGAATACCAGCTTTCCCCTTTCCCTGAGAGGAAGAGCACATGTTGGTCTC
CTCTTAGTGTGAACGAGATTGCCAGGCCCTTTTCTCCTATGCACACCAGGATAGACAAGGCAGGGGATACTGGCA
GCCTGCATCATCCTCCCATTGGGCTGACAGCTGGCCCTACTTTCCTCCCTCTGCTGCTTGGTCCCTCACCTTGAT
GATGTGGCTTCGCCCCCTCCACTCTACTGCCAGTGTTCTCCCAGGGGTTGCTAAATCCAGCAGACCCCTTTCCTG
TCTTACTAGATCTGGGCAGCATTTGACATGGCTGATCACCCCTTGCTTCTTGGATGGCACTTCCCTGGCACCTCT
GTGGCTAGTTGTCCTACCTCCCTGGCTGTTCCTTTCAGGCTTCCGTGCAGGCTTCTCCACTTGCCCATGCACAGT
AGGGTCTTTCAGGGTTCTGCTGTGGGCTCCCTAGGGAAGCCCATCCATCTGGATGGTTTCAAGGATGGTGAGGAA
TTTAGAGTTGACCTCCAGCCCCAACATCCTTCCTGATCACCTGAACCACAGTTTTGCTGCCCTCTAGGTGCACAG
ACAATTCAGGTCCATGGCCCAGATGGTACTTGCTGTCTTCTGCAAACCTGCCCCTTCTGGGTACTTCCCTTGACC
CCGAGATCACTCAGGAGCCAGACAGGAAACTTATTCTATTCCTGTTTTCTCTTTCTGCCCACCACATCCAATCTC
TCAAAACGGTCAGGTCTACCTTAACATCTCTTGATTTGAGCCACTCCCACTGTCATCAGCTTTCACCTGGATTAT
CGTGACAGCCTCCTACTGCTTCTCTATCATGTGGCCAGAGCTATCTTCCTAAAATGCATTGCATAGTTGATCAAG
TCACTCTCTGGCCTAAAACCTTCCTTGGCTCCCTGCTGCCCTCAGGATAAAGTCTGGACCCCTCAGCATGGCTTG
TGAGACTCATGGTGTCCTTGTCCCTGCTCACCTCTCTGGTCTCATCACTTGCCTTCTTGCATTCTGGGTCCCAGC
CTCCTGTATCCAGAGATGCAGTGGCTCTCCATTGCCACTCTGATTCCTCCTTTCTTTTGGTCACAGAGAAAGGGT
ACTTTCTCTGTCAAATCTCAACTTAGACTTGACTTCCTCCAAGGAGCTTTGGCTATACTCTCTCCTCCCGACCCC
CACCCTGGCATACTACACAGATCACTCTGGGCTCACTTGCCTGCCTAATGGTCATCTCCCCAGTAGACTGTAAGC
TCCTTGAGGGCAAGGATTGTGTTGGAATTTTTGTATTAACAGTGCCTGGCTTGGTGCCTGGCACCTAGAAAGCAC
TCAATAAATGTTTGTTTAATGAA

# FIGURE 168

MWFMYLLSWLSLFIQVAFITLAVAAGLYYLAELIEEYTVATSRIIKYMIWFSTAVLIGLYVFE
RFPTSMIGVGLFTNLVYFGLLQTFPFIMLTSPNFILSCGLVVVNHYLAFQFFAEEYYPFSEVL
AYFTFCLWIIPFAFFVSLSAGENVLPSTMQPGDDVVSNYFTKGKRGKRLGILVVFSFIKEAIL
PSRQKIY

**Signal peptide:**
amino acids 1-25

**Transmembrane domain:**
amino acids 126-146

**Casein kinase II phosphorylation site.**
amino acids 145-148

**N-myristoylation sites.**
amino acids 73-78, 82-87

**Amidation sites.**
amino acids 168-171, 171-174

**Prokaryotic membrane lipoprotein lipid attachment site.**
amino acids 91-101

# FIGURE 169

CAAAGCCCTACCCTCACCATTCACCAGGTCCTGTGGGAAGAGCAGCGTGGAGGTGGGCTGAGG
TTAGAAGGTGCAGAGCGTGGAAGAAGATTGTGAGCTGAGTATTGGACATCTGTTCTTGAATAG
TCCCTGGGCCTGCCATAGGAAAGGAAGTTCTCCAGGGTTACAGTTCTTATCCGCGTGAATACA
C**ATG**GCTCTGTTACGAAAAATTAATCAGGTGCTGCTGTTCCTTCTGATCGTGACCCTCTGTGT
GATTCTGTATAAGAAAGTTCATAAGGGGACTGTGCCCAAGAATGACGCAGATGATGAATCCGA
GACTCCTGAAGAACTGGAAGAAGAGATTCCTGTGGTGATTTGTGCTGCAGCAGGGAGGATGGG
TGCCACTATGGCTGCCATCAATAGCATCTACAGCAACACTGACGCCAACATCTTGTTCTATGT
AGTGGGACTCCGGAATACTCTGACTCGAATACGAAAATGGATTGAACATTCCAAACTGAGAGA
AATAAACTTTAAAATCGTGGAATTCAACCCGATGGTCCTCAAAGGGAAGATCAGACCAGACTC
ATCGAGGCCTGAATTGCTCCAGCCTCTGAACTTTGTTCGATTTTATCTCCCTCTACTTATCCA
CCAACACGAGAAAGTCATCTATTTGGACGATGATGTAATTGTACAAGGTGATATCCAAGAACT
GTATGACACCACCTTGGCCCTGGGCCACGCGGCGGCTTTCTCAGATGACTGCGATTTGCCCTC
TGCTCAGGACATAAACAGACTCGTGGGACTTCAGAACACATATATGGGCTATCTGGACTACCG
GAAGAAGGCCATCAAGGACCTTGGCATCAGCCCCAGCACCTGCTCTTTCAATCCTGGTGTGAT
TGTTGCCAACATGACAGAATGGAAGCACCAGCGCATCACCAAGCAATTGGAGAAATGGATGCA
AAAGAATGTGGAGGAAAACCTCTATAGCAGCTCCCTGGGAGGAGGGGTGGCCACCTCCCCAAT
GCTGATTGTGTTTCATGGGAAATATTCCACAATTAACCCCCTGTGGCACATAAGGCACCTGGG
CTGGAATCCAGATGCCAGATATTCGGAGCATTTTCTGCAGGAAGCTAAATTACTCCACTGGAA
TGGAAGACATAAACCTTGGGACTTCCCTAGTGTTCACAACGACTTATGGGAAAGCTGGTTTGT
TCCTGACCCTGCAGGGATATTTAAACTCAATCACCATAGC**TGA**TATAACTCTACCCTTAAAAT
ATTCCCTGTATAGAAATGTGGAATTGTCCCTTTGTAGCCAACTATAACATTGTTCTTTATGAA
TATTACCTTTGATACATATGATCCACAATATAAAAACCAAAAACTACTGTGTGCAAATTATAC
CTTGGACCATATAGGCATTGATTAACTTCTTTAAGTACATGTGATAACTATGGAAATCAAGAT
TATGTGACTGAAAAACATAAAGGAAGAGACCCATCTAGATAACAGCAATCAACCTGCTTAATT
CTGAATGACAATTATATCCACAAATTTTTAAAACTTCTACATGTATTTTTCACATGAAGATCT
CCTTAACAGGTTGCCAACCTTTTCTTTTATAAAACTATTACATTTAAAATATGGACGTCTGAA
AAATAAAATATTCATCATTTTTAAAA

# FIGURE 170

MALLRKINQVLLFLLIVTLCVILYKKVHKGTVPKNDADDESETPEELEEEIPVVICAAAGRMG
ATMAAINSIYSNTDANILFYVVGLRNTLTRIRKWIEHSKLREINFKIVEFNPMVLKGKIRPDS
SRPELLQPLNFVRFYLPLLIHQHEKVIYLDDDVIVQGDIQELYDTTLALGHAAAFSDDCDLPS
AQDINRLVGLQNTYMGYLDYRKKAIKDLGISPSTCSFNPGVIVANMTEWKHQRITKQLEKWMQ
KNVEENLYSSSLGGGVATSPMLIVFHGKYSTINPLWHIRHLGWNPDARYSEHFLQEAKLLHWN
GRHKPWDFPSVHNDLWESWFVPDPAGIFKLNHHS

**Signal peptide:**
amino acids 1-20

**N-glycosylation site.**
amino acids 234-238

**Tyrosine kinase phosphorylation site.**
amino acids 253-261

**N-myristoylation sites.**
amino acids 63-69, 86-92, 198-204, 218-224, 229-235, 265-271, 266-272

# FIGURE 171

GCCAGAGGCTGCAGCTGGAGCCCAGAGCCCAAG**ATG**GAGCCCCAGCTGGGGCCTGAGGCTGCC
GCCCTCCGCCCTGGCTGGCTGGCCCTGCTGCTGTGGGTCTCAGCCCTGAGCTGTTCTTTCTCC
TTGCCAGCTTCTTCCCTTTCTTCTCTGGTGCCCCAAGTCAGAACCAGCTACAATTTTGGAAGG
ACTTTCCTCGGTCTTGATAAATGCAATGCCTGCATCGGGACATCTATTTGCAAGAAGTTCTTT
AAAGAAGAAATAAGATCTGACAACTGGCTGGCTTCCCACCTTGGACTGCCTCCCGATTCCTTG
CTTTCTTATCCTGCAAATTACTCAGATGATTCCAAAATCTGGCGCCCTGTGGAGATCTTTAGA
CTGGTCAGCAAATATCAAAACGAGATCTCAGACAGGAGAATCTGTGCCTCTGCATCAGCCCCA
AAGACCTGCAGCATTGAGCGTGTCCTGCGGAAAACAGAGAGGTTCCAGAAATGGCTGCAGGCC
AAGCGCCTCACGCCGGACCTGGTGCAGGACTGTCACCAGGGCCAGAGAGAACTAAAGTTCCTG
TGTATGCTGAGA**TAA**CACCAGTGAAAAAGCCTGGCATGGAGCCCAGCACTGAGAACTTCCAGA
AAGTGTTAGCCTTCTCCCAACTGTGTTATACCAACCACATTTTCAAATAGTAATCATTAAAGA
GGCTTCTGCATCAAAAAAAAAAAAAAAAAAAAAAAAAAAAAAAAAAAAAAAAAAAAAAAAAAA

# FIGURE 172

MEPQLGPEAAALRPGWLALLLWVSALSCSFSLPASSLSSLVPQVRTSYNFGRTFLGLDKCNAC
IGTSICKKFFKEEIRSDNWLASHLGLPPDSLLSYPANYSDDSKIWRPVEIFRLVSKYQNEISD
RRICASASAPKTCSIERVLRKTERFQKWLQAKRLTPDLVQDCHQGQRELKFLCMLR

**Signal peptide:**
amino acids 1-28

**N-glycosylation site.**
amino acids 100-103

**cAMP- and cGMP-dependent protein kinase phosphorylation site.**
amino acids 158-161

**N-myristoylation sites.**
amino acids 56-61, 65-70

**Prokaryotic membrane lipoprotein lipid attachment site.**
amino acids 18-28

**Prenyl group binding site (CAAX box).**
amino acids 179-182

**Leucine zipper pattern.**
amino acids 5-26

# FIGURE 173

GCTGGACTGCTCGCTGGCCGGCAGCGCACCGTTTTGAAGGTCCTAGCCCACCTGGGCTGGCTC
ACGCGCACGACTAGCCGCTCCCATACAGCACGCCCGGACTCTGTCGTCGCTTAAGGCCACTCC
TATTCTACGGCTGACCCCTGGTGGTCACGTGGATCTGTTCGCCACGCAAGTCTGGGTCCTTCG
GCGATTGACCGGGGTCCTTGCTGTTCGGGAGCCTCTCCTAAGCTGCCTGTTCGCGCGAGAGTT
TGGAGGGGCGGGTTTGGGGTCGGTGTCTGATTGGGGCTCGCACCGCAGCACGCTGGAGTCCCG
CTTAGGTACCAGTTAGCGTCAGGGGAGCTGGGTCAGGCGGTCGCCGGGACACCCCGTGTGTGG
CAGGCGGCGAAGCGCTCTGGAGAATCCCGGACAGCCCTGCTCCCTGCAGCCAGGTGTAGTTTC
GGGAGCCACTGGGGCCAAAGTGAGAGTCCAGCGGTCTTCCAGCGCTTGGGCCACGGCGGCGGC
CCTGGGAGCAGAGGTGGAGCGACCCCATTACGCTAAAG**ATG**AAAGGCTGGGGTTGGCTGGCCC
TGCTTCTGGGGGCCCTGCTGGGAACCGCCTGGGCTCGGAGGAGCCAGGATCTCCACTGTGGAG
CATGCAGGGCTCTGGTGGATGAACTAGAATGGGAAATTGCCCAGGTGGACCCCAAGAAGACCA
TTCAGATGGGATCTTTCCGGATCAATCCAGATGGCAGCCAGTCAGTGGTGGAGGTGCCTTATG
CCCGCTCAGAGGCCCACCTCACAGAGCTGCTGGAGGAGATATGTGACCGGATGAAGGAGTATG
GGGAACAGATTGATCCTTCCACCCATCGCAAGAACTACGTACGTGTAGTGGGCCGGAATGGAG
AATCCAGTGAACTGGACCTACAAGGCATCCGAATCGACTCAGATATTAGCGGCACCCTCAAGT
TTGCGTGTGAGAGCATTGTGGAGGAATACGAGGATGAACTCATTGAATTCTTTTCCCGAGAGG
CTGACAATGTTAAAGACAAACTTTGCAGTAAGCGAACAGATCTTTGTGACCATGCCCTGCACA
TATCGCATGATGAGCTA**TGA**ACCACTGGAGCAGCCCACACTGGCTTGATGGATCACCCCCAGG
AGGGGAAAATGGTGGCAATGCCTTTTATATATTATGTTTTTACTGAAATTAACTGAAAAAATA
TGAAACCAAAAGT

# FIGURE 174

MKGWGWLALLLGALLGTAWARRSQDLHCGACRALVDELEWEIAQVDPKKTIQMGSFRINPDGS
QSVVEVPYARSEAHLTELLEEICDRMKEYGEQIDPSTHRKNYVRVVGRNGESSELDLQGIRID
SDISGTLKFACESIVEEYEDELIEFFSREADNVKDKLCSKRTDLCDHALHISHDEL

**Signal peptide:**
amino acids 1-20

**N-myristoylation sites.**
amino acids 12-18, 16-22, 29-35

**Endoplasmic reticulum targeting sequence.**
amino acids 179-184

# FIGURE 175

CGCAGCGCGGCAGTCCTG**ATG**GCCCGGCATGGGTTACCGCTGCTGCCCCTGCTGTCGCTCCTG
GTCGGCGCGTGGCTCAAGCTAGGAAATGGACAGGCTACTAGCATGGTCCAACTGCAGGGTGGG
AGATTCCTGATGGGAACAAATTCTCCAGACAGCAGAGATGGTGAAGGGCCTGTGCGGGAGGCG
ACAGTGAAACCCTTTGCCATCGACATATTTCCTGTCACCAACAAAGATTTCAGGGATTTTGTC
AGGGAGAAAAAGTATCGGACAGAAGCTGAGATGTTTGGATGGAGCTTTGTCTTTGAGGACTTT
GTCTCTGATGAGCTGAGAAACAAAGCCACCCAGCCAATGAAGTCTGTACTCTGGTGGCTTCCA
GTGGAAAAGGCATTTTGGAGGCAGCCTGCAGGTCCTGGCTCTGGCATCCGAGAGAGACTGGAG
CACCCAGTGTTACACGTGAGCTGGAATGACGCCCGTGCCTACTGTGCTTGGCGGGGAAAACGA
CTGCCCACGGAGGAAGAGTGGGAGTTTGCCGCCCGAGGGGGCTTGAAGGGTCAAGTTTACCCA
TGGGGGAACTGGTTCCAGCCAAACCGCACCAACCTGTGGCAGGGAAAGTTCCCCAAGGGAGAC
AAAGCTGAGGATGGCTTCCATGGAGTCTCCCCAGTGAATGCTTTCCCCGCCCAGAACAACTAC
GGGCTCTATGACCTCCTGGGGAACGTGTGGGAGTGGACAGCATCACCGTACCAGGCTGCTGAG
CAGGACATGCGCGTCCTCCGGGGGGCATCCTGGATCGACACAGCTGATGGCTCTGCCAATCAC
CGGGCCCGGGTCACCACCAGGATGGGCAACACTCCAGATTCAGCCTCAGACAACCTCGGTTTC
CGCTGTGCTGCAGACGCAGGCCGGCCGCCAGGGGAGCTG**TAA**GCAGCCGGGTGGTGACAAGGA
GAAAAGCCTTCTAGGGTCACTGTCATTCCCTGGCCATGTTGCAAACAGCGCAATTCCAAGCTC
GAGAGCTTCAGCCTCAGGAAAGAACTTCCCCTTCCCTGTCTCCCATCCCTCTGTGGCAGGCGC
CTCTCACCAGGGCAGGAGAGGACTCAGCCTCCTGTGTTTTGGAGAAGGGGCCCAATGTGTGTT
GACGATGGCTGGGGGCCAGGTGTTTCTGTTAGAGGCCAAGTATTATTGACACAGGATTGCAAA
CACACAAACAGTTGGAACAGAGCACTCTGAAAGGCCATTTTTTAAGCATTTTAAAATCTATTC
TCTCCCCCTTTCTCCCTGGATGATTCAGGAAGCTGACATTGTTTCCTCAAGGCAGAATTTTCC
TGGTTCTGTTTTCTCAGCCAGTTGCTGTGGAAGGAGAATGCTTTCTTTGTGGCCTCATCTGTG
GTTTCGTGTCCCTCTGAAGGAAACTAGTTTCCACTGTGTAACAGGCAGACATGTAACTATTTA
AAGCACAGTTCAGTCCTAAAAGGGTCTGGGAGAACCAGATGATGTACTAGGTGAAGCATTGCA
TTGTGGGAATCACAAAGCAAATAGTACTCCAGAAAGACAAATATCAGAAGCTTCCTATTCTTT
TTTTTTTTTTTTTTTTTTTTTGAGACAGGGTCTTTCTCTGTTGCCCAGGCTAGAGTGCACTG
GTGATCACGGCTCACTCTAGCCTTGAATTCCTGGGCCCAAGCAATTCTCCCACCTCAGCCTCC
TGAGTAGCTGGGACTACAAGTGTGCACCACCATGCCTGGCTAATTTTTTGAATTTTTGTAGTG
ATGGGATCTCGCTCTGTTGCCCAGGGTGGTCTCGAACTCCTGGCCTCAAGCGATCCTCCCACC
TCGACCTCCCAAAGTGCTGGGATTACAGGTGTGAGCCACCTCGCCTGGGCCCCCTTCTCCATA
TGCCTCCAAAAACATGTCCCTGGAGAGTAGCCTGCTCCCACACTGTCACTGGATGTCATGGGG
CCAATAAAATCTCCTGCAATTGTGTATCTCAAAAAAAAAAAAAAAAAAAAAAAAAAAAAAAAA
AAAAAAAAAAA

# FIGURE 176

MARHGLPLLPLLSLLVGAWLKLGNGQATSMVQLQGGRFLMGTNSPDSRDGEGPVREATVKPFA
IDIFPVTNKDFRDFVREKKYRTEAEMFGWSFVFEDFVSDELRNKATQPMKSVLWWLPVEKAFW
RQPAGPGSGIRERLEHPVLHVSWNDARAYCAWRGKRLPTEEEWEFAARGGLKGQVYPWGNWFQ
PNRTNLWQGKFPKGDKAEDGFHGVSPVNAFPAQNNYGLYDLLGNVWEWTASPYQAAEQDMRVL
RGASWIDTADGSANHRARVTTRMGNTPDSASDNLGFRCAADAGRPPGEL

**Signal peptide:**
amino acids 1-20

**N-glycosylation site.**
amino acids 191-195

**N-myristoylation sites.**
amino acids 23-29, 25-31, 175-181

**Amidation site.**
amino acids 159-163

# FIGURE 177

GCCTTCTCGCGCCTGACCATGCACCCCTGCATCTTCCTGCTGGGCCACAGGCGAGCGCTTTAT
TTCTGGAGCTGAGGGCTAAAACTTTTTTGACTTTTCTTCTCCTCAACATCTGAATC**ATG**CCAT
GTGCCCAGAGGAGCTGGCTTGCAAACCTTTCCGTGGTGGCTCAGCTCCTTAACTTTGGGGCGC
TTTGCTATGGGAGACAGCCTCAGCCAGGCCCGGTTCGCTTCCCGGACAGGAGGCAAGAGCATT
TTATCAAGGGCCTGCCAGAATACCACGTGGTGGGTCCAGTCCGAGTAGATGCCAGTGGGCATT
TTTTGTCATATGGCTTGCACTATCCCATCACGAGCAGCAGGAGGAAGAGAGATTTGGATGGCT
CAGAGGACTGGGTGTACTACAGAATTTCTCACGAGGAGAAGGACCTGTTTTTTAACTTGACGG
TCAATCAAGGATTTCTTTCCAATAGCTACATCATGGAGAAGAGATATGGGAACCTCTCCCATG
TTAAGATGATGGCTTCCTCTGCCCCCCTCTGCCATCTCAGTGGCACGGTTCTACAGCAGGGCA
CCAGAGTTGGGACGGCAGCCCTCAGTGCCTGCCATGGACTGACTGGATTTTTCCAACTACCAC
ATGGAGACTTTTTCATTGAACCCGTGAAGAAGCATCCACTGGTTGAGGGAGGGTACCACCCGC
ACATCGTTTACAGGAGGCAGAAAGTTCCAGAAACCAAGGAGCCAACCTGTGGATTAAAGGGTA
TTGTGACTCACATGTCCTCCTGGGTTGAAGAATCTGTTTTGTTCTTTTGG**TAG**TTTTATTAAA
ACATGACCTATTCTTACTCAAGTCTCTTATCTCCTCTGTATTCTTTTTTTTTTAATATCTTCA
TGACATTCAAATCTCTTCTGTATTCTCTTGCCAGAAAGTGTACATTCTTTTTGCTTGTATAAA
CCCTTTCACTTGTC

# FIGURE 178

MPCAQRSWLANLSVVAQLLNFGALCYGRQPQPGPVRFPDRRQEHFIKGLPEYHVVGPVRVDAS
GHFLSYGLHYPITSSRRKRDLDGSEDWVYYRISHEEKDLFFNLTVNQGFLSNSYIMEKRYGNL
SHVKMMASSAPLCHLSGTVLQQGTRVGTAALSACHGLTGFFQLPHGDFFIEPVKKHPLVEGGY
HPHIVYRRQKVPETKEPTCGLKGIVTHMSSWVEESVLFFW

**Signal peptide:**

amino acids 1-27

**N-glycosylation sites.**

amino acids 11-15, 105-109, 125-129

**N-myristoylation site.**

amino acids 149-155

# FIGURE 179

CAGATTTAAAAAGAAAACCTTTACTGAATCAGCTGAGTGTTAATAATACGAATTTCCTTTTCT
TGCCAATTCTGATCTGAACAGAAAATCCAAGAACAGGGAT**ATG**TGTGGATTACAGTTTTCTCT
GCCTTGCCTACGACTGTTTCTGGTTGTTACCTGTTATCTTTTATTATTACTCCACAAAGAAAT
ACTTGGATGTTCGTCTGTTTGTCAGCTCTGCACTGGGAGACAAATTAACTGCCGTAACTTAGG
CCTTTCGAGTATTCCTAAGAATTTTCCTGAAAGTACAGTTTTTCTGTATCTGACTGGGAATAA
TATATCTTATATAAATGAAAGTGAATTAACAGGACTTCATTCTCTTGTAGCATTGTATTTGGA
TAATTCTAACATTCTGTATGTATATCCAAAAGCCTTTGTTCAATTGAGGCATCTATATTTTCT
ATTTCTAAATAATAATTTCATCAAACGCTTAGATCCTGGAATATTTAAGGGACTTTTAAATCT
TCGTAATTTATATTTACAGTATAATCAGGTATCTTTTGTTCCGAGAGGAGTATTTAATGATCT
AGTTTCAGTTCAGTACTTAAATCTACAAAGGAATCGCCTCACTGTCCTTGGGAGTGGTACCTT
TGTTGGTATGGTTGCTCTTCGGATACTTGATTTATCAAACAATAACATTTTGAGGATATCAGA
ATCAGGCTTTCAACATCTTGAAAACCTTGCTTGTTTGTATTTAGGAAGTAATAATTTAACAAA
AGTACCATCAAATGCCTTTGAAGTACTTAAAAGTCTTAGAAGACTTTCTTTGTCTCATAATCC
TATTGAAGCAATACAGCCCTTTGCATTTAAAGGACTTGCCAATCTGGAATACCTCCTCCTGAA
AAATTCAAGAATTAGGAATGTTACTAGGGATGGGTTTAGTGGAATTAATAATCTTAAACATTT
GATCTTAAGTCATAATGATTTAGAGAATTTAAATTCTGACACATTCAGTTTGTTAAAGAATTT
AATTTACCTTAAGTTAGATAGAAACAGAATAATTAGCATTGATAATGATACATTTGAAAATAT
GGGAGCATCTTTGAAGATCCTTAATCTGTCATTTAATAATCTTACAGCCTTGCATCCAAGGGT
CCTTAAGCCGTTGTCTTCATTGATTCATCTTCAGGCAAATTCTAATCCTTGGGAATGTAACTG
CAAACTTTTGGGCCTTCGAGACTGGCTAGCATCTTCAGCCATTACTCTAAACATCTATTGTCA
GAATCCCCCATCCATGCGTGGCAGAGCATTACGTTATATTAACATTACAAATTGTGTTACATC
TTCAATAAATGTATCCAGAGCTTGGGCTGTTGTAAAATCTCCTCATATTCATCACAAGACTAC
TGCGCTAATGATGGCCTGGCATAAAGTAACCACAAATGGCAGTCCTCTGGAAAATACTGAGAC
TGAGAACATTACTTTCTGGGAACGAATTCCTACTTCACCTGCTGGTAGATTTTTTCAAGAGAA
TGCCTTTGGTAATCCATTAGAGACTACAGCAGTGTTACCTGTGCAAATACAACTTACTACTTC
TGTTACCTTGAACTTGGAAAAAAACAGTGCTCTACCGAATGATGCTGCTTCAATGTCAGGGAA
AACATCTCTAATTTGTACACAAGAAGTTGAGAAGTTGAATGAGGCTTTTGACATTTTGCTAGC
TTTTTTCATCTTAGCTTGTGTTTTAATCATTTTTTTGATCTACAAAGTTGTTCAGTTTAAACA
AAAACTAAAGGCATCAGAAAACTCAAGGGAAAATAGACTTGAATACTACAGCTTTTATCAGTC
AGCAAGGTATAATGTAACTGCCTCAATTTGTAACACTTCCCCAAATTCTCTAGAAAGTCCTGG
CTTGGAGCAGATTCGACTTCATAAACAAATTGTTCCTGAAAATGAGGCACAGGTCATTCTTTT
TGAACATTCTGCTTTA**TAA**CTCAACTAAATATTGTCTATAAGAAACTTCAGTGCCATGGACAT
GATTTAAACTGAAACCTCCTTATATAATTATATACTTTAGTTGGAAATATAATGAATTATATG
AGGTTAGCATTATTAAAATATGTTTTTNTTAAAAAAAAAAAAAAAAAAAAAAAA

# FIGURE 180

MCGLQFSLPCLRLFLVVTCYLLLLLHKEILGCSSVCQLCTGRQINCRNLGLSSIPKNFPESTV
FLYLTGNNISYINESELTGLHSLVALYLDNSNILYVYPKAFVQLRHLYFLFLNNNFIKRLDPG
IFKGLLNLRNLYLQYNQVSFVPRGVFNDLVSVQYLNLQRNRLTVLGSGTFVGMVALRILDLSN
NNILRISESGFQHLENLACLYLGSNNLTKVPSNAFEVLKSLRRLSLSHNPIEAIQPFAFKGLA
NLEYLLLKNSRIRNVTRDGFSGINNLKHLILSHNDLENLNSDTFSLLKNLIYLKLDRNRIISI
DNDTFENMGASLKILNLSFNNLTALHPRVLKPLSSLIHLQANSNPWECNCKLLGLRDWLASSA
ITLNIYCQNPPSMRGRALRYINITNCVTSSINVSRAWAVVKSPHIHHKTTALMMAWHKVTTNG
SPLENTETENITFWERIPTSPAGRFFQENAFGNPLETTAVLPVQIQLTTSVTLNLEKNSALPN
DAASMSGKTSLICTQEVEKLNEAFDILLAFFILACVLIIFLIYKVVQFKQKLKASENSRENRL
EYYSFYQSARYNVTASICNTSPNSLESPGLEQIRLHKQIVPENEAQVILFEHSAL

**Signal peptide:**

amino acids 1-41

**Transmembrane domain:**

amino acids 530-547

**N-glycosylation sites.**

amino acids 71-75, 76-80, 215-219, 266-270, 317-321, 331-335, 336-340, 400-404, 410-414, 451-455, 579-583

**cAMP- and cGMP-dependent protein kinase phosphorylation site.**

amino acids 231-235

**N-myristoylation sites.**

amino acids 3-9, 69-75, 126-132, 174-180

**ATP/GTP-binding site motif A (P-loop).**

amino acids 506-514

# FIGURE 181

GGCCTGGCGCGGCGCTCCGGTAAGGCGTGTGTGCGGCAGGGCGGGGACAGAACCGTCCTCTCG
GGCTCTGGGCGTGTCCGAGACCGCGCTCCCCGCCGAAATCAAGCTCCGAGTCATCCGTGTGGG
GCATTCGTCCCCCCTGGCACAGTTGGCCTCTTTCCAGAAGCCCGTTTTGTTTGTTTTACGTCT
AAATTCGCGTCGGTTCTTATTTCTCTCCCTGGCAAGGTCTGAAGACGGGTAGGAGAATAACCT
GTGTCAGCGTGTT**<u>ATG</u>**ATGCCGTCCCGTACCAACCTGGCTACTGGAATCCCCAGTAGTAAAGT
GAAATATTCAAGGCTCTCCAGCACAGACGATGGCTACATTGACCTTCAGTTTAAGAAAACCCC
TCCTAAGATCCCTTATAAGGCCATCGCACTTGCCACTGTGCTGTTTTTGATTGGCGCCTTTCT
CATTATTATAGGCTCCCTCCTGCTGTCAGGCTACATCAGCAAAGGGGGGGCAGACCGGGCCGT
TCCAGTGCTGATCATTGGCATTCTGGTGTTCCTACCCGGATTTTACCACCTGCGCATCGCTTA
CTATGCATCCAAAGGCTACCGTGGTTACTCCTATGATGACATTCCAGACTTTGATGAC**<u>TAG</u>**CA
CCCACCCCATAGCTGAGGAGGAGTCACAGTGGAACTGTCCCAGCTTTAAGATATCTAGCAGAA
ACTATAGCTGAGGACTAAGGAATTCTGCAGCTTGCAGATGTTTAAGAAAATAATGGCCAGATT
TTTTGGGTCCTTCCCAAAGATGTTAAGTGAACCTACAGTTAGCTAATTAGGACAAGCTCTATT
TTTCATCCCTGGGCCCTGACAAGTTTTTCCACAGGAATATGTATCATGGAAGAATAGAGGTTA
TTCTGTAATGGAAAAGTGTTGCCTGCCACCACCCTCTGTAGAGCTGAGCATTTCTTTTAAATA
GTCTTCATTGCCAATTTGTTCTTGTAGCAAATGGAACAATGTGGTATGGCTAATTTCTTATTA
TTAAGTAGTTTATTTTAAAAATATCTGAGTATATTATCCTGTACACTTATCCCTACCTTCATG
TTCCAGTGGAAGACCTTAGTAAAATCAAAGATCAGTGAGTTCATCTGTAATATTTTTTTTACT
TGCTTTCTTACTGACAGCAACCAGGAATTTTTTTATCCTGCAGAGCAAGTTTTCAAAATGTAA
ATACTTCCTCTGTTTAACAGTCCTTGGACCATTCTGATCCAGTTCACCAGTAGGTTGGACAGC
ATATAATTTGCATCATTTTGTCCCTTGTAAATCAAGATGTTCTGCAGATTATTCCTTTAACGG
CCGGACTTTTGGCTGTTTCCTAATGAAACATGTAGTGGTTATTATTTAGAGTTTATAGCCGTA
TTGCTAGCACCTTGTAGTATGTCATCATTCTGCTCATGATTCCAAGGATCAGCCTGGATGCCT
AGAGGACTAGATCACCTTAGTTTGATTCTATTTTTTAGCTTGCAAAAAGTGACTTATATTCCA
AAGAAATTAAAATGTTGAAATCCAAATCCTAGAAATAAAATGAGTTTNNTTCCAAAAAAAAAA
AAAAAAAAAAAAAAAAAAAAAAAAAAAAAAAAAAAAAAAAAAAAAAAAAA

# FIGURE 182

MMPSRTNLATGIPSSKVKYSRLSSTDDGYIDLQFKKTPPKIPYKAIALATVLFLIGAFLIIIG
SLLLSGYISKGGADRAVPVLIIGILVFLPGFYHLRIAYYASKGYRGYSYDDIPDFDD

**Transmembrane domains:**

amino acids 45-66, 79-95

**N-myristoylation sites.**

amino acids 11-17, 75-81

# FIGURE 183

CTAAAAAATACAAAAATTAGCTGGGCGTGGTGTCATGTACCTGTAATCCCAGCTACTCAAGAGGCTGAGGCAGGA
GAATCGCTTGAACCCAGGAGGCAGAGGTTGCAGTGAGCCAAGATTAAGTCACTGCACTCCAGCCTGGGTGACAGA
GCAAGACTCTGTATCAAAATAAATAAATAAAGTACAACTCTGGATGGGCATGGTGGCTTATGTCTGTAATCCCAG
CACTTTGGGAACTTGAGGCGGGTAGATTGCTTGAGTCCGGGAGTTTGAGACCAGTCTGGGTAATATGGTAACCCT
GTCTACCAAAAATACAGGTATTAGCCAGTCTCATAACTCGGTCTCAAAATAAATAAATACATACATACATAGATG
AAAATTTAAAAAATAAAGTCCAACTCAGCGGTTTTCAGCATATTTACAGAGTTGTACAATCTTCACCACTATCTA
ATTTCAGAACATTTTCATCACCCCCAAAAGAAACCTAACCCATTGACTATCTCTCCATTTCCTCCCTCTCCCTAG
CCTCTGGCAACCACTAATCTCTTTTTTGTCTCTATAGATTTGCCTATTTTGGACAGTTCATATACAAGGAATCAT
ACCACATGTAGCCTTTTGTGTCCGGCTTCTTTGATTAATAGAATGTTTTCAAGGCTCATCTATGCTGTAGCCTGT
ATCAGCACTTCATTCCTTTCTATGGCTGAATAATAGTCCACTGTAGGGATGTGCCATGTTTTTCCACTAGCTGAT
GGACATTTGGGTTGTTTCCACCTTCTGGCTATTATAAATATTGCTGCTATAAATATTCACTTACAAGTTTTTGTG
TGGACATATGTTTTTATTTCTTCTGGTATATCCTTCGGAGTGGAACTGCTGGATCAGGTGGTAACTCTAGGTCTA
ACCTGGCAGTTAAACAGAATCCTATGCATGCTGTAGTCCATGAGTTGAAATAAACACTTGACCCATAGTAAGTGC
CAGATCATCTTCATTTCACAGCAACCAGTAATTTCACAGATGAGGAAATGAAGGCTCCCAGAGGTGAACTGGCTT
TTCCCATTTGAGCAGTTCCAAGTCAGACAGTTAAAAAGTGGCAGGACCTGGAAGAGAAGCTAGTTCTTTCACCCT
GGCATTCAGGGCTGCCTCCTGGGCTACGGGGCTGGCATTTAGAATAGAGCTAAGGTCTGCTGCCAAGGCAGGTGC
CCCAGTCTGCCTCCTCTGTGTCCTTATTCCACTTTCTCTGCAGCCCTCCAGGGGACCCCTCTCTCAGCCACCCTC
TCTCTGGTG**ATG**TCACAGTGCTGCCGGAAGATCAAAGATACGGTGCAGAAACTGGCTTCGGACCATAAGGACATT
CACAGCAGTGTATCCCGAGTGGGCAAAGCCATTGACAGGAACTTCGACTCTGAGATCTGTGGTGTTGTGTCAGAT
GCGGTGTGGGACGCGCGGGAACAGCAGCAGCAGATCCTGCAGATGGCCATCGTGGAACACCTGTATCAGCAGGGC
ATGCTCAGCGTGGCCGAGGAGCTGTGCCAGGAATCAACGCTGAATGTGGACTTGGATTTCAAGCAGCCTTTCCTA
GAGTTGAATCGAATCCTGGAAGCCCTGCACGAACAAGACCTGGGTCCTGCGTTGGAATGGGCCGTCTCCCACAGG
CAGCGCCTGCTGGAACTCAACAGCTCCCTGGAGTTCAAGCTGCACCGACTGCACTTCATCCGCCTCTTGGCAGGA
GGCCCCGCGAAGCAGCTGGAGGCCCTCAGCTATGCTCGGCACTTCCAGCCCTTTGCTCGGCTGCACCAGCGGGAG
ATCCAGGTGATGATGGGCAGCCTGGTGTACCTGCGGCTGGGCTTGGAGAAGTCACCCTACTGCCACCTGCTGGAC
AGCAGCCACTGGGCAGAGATCTGTGAGACCTTTACCCGGGACGCCTGTTCCCTGCTGGGGCTTTCTGTGGAGTCC
CCCCTTAGCGTCAGCTTTGCCTCTGGCTGTGTGGCGCTGCCTGTGTTGATGAACATCAAGGCTGTGATTGAGCAG
CGGCAGTGCACTGGGGTCTGGAATCACAAGGACGAGTTACCGATTGAGATTGAACTAGGCATGAAGTGCTGGTAC
GCTCATCTGTGGCCATGTTATCTCCCGAGATGCACTCAATAAGCTCATTAATGGAGGAAACACTCCGTGTTCGCT
TGCCCCATCCTCCGCCAGCAGACGTCAGATTCCAACCCTCCCATCAAGCTGAAGTGTCCCTACTGTCCCATGGAG
CAGAACCCGGCAGATGGGAAACGCATCATATTC**TGA**TTCCTACCTGGAAGGAATTTTGTTGAAAGGGGTTTTCAC
CTGTGAGCCTTGGTCTGTCTCGGTAGGGTGGTCAACTTCAGTGGACTGTGGTTGGTTTCAGAGCGCCTGGCTGAG
GAGTTCCACTGAGGGGAGCACTGGAGCAGCCCTTTGGCAGAGGCTGAGGAGGGAGATGGACCAGCCCACGCCTGG
CACCTGGCTCCATGGCATAAGGAAAGGGAGATGCTGGCCTCTGTGCTCCTGCTGTCTTTTCCTGTTTCTGTTTGC
GTTTGACTTAGTAGCAACCGACAGAGTGGCAAGGGATTTGGTCTTCAGCAGTAGACATCCTTCCACCCCTGCCCT
CAGCCAAGTCTCTTGCTGCCATGCCAATGCTATGTCCACCCTTGCCCCTCGGCCCAAGAGTGTCCAGCGGTGGCC
CACCTCTTCCTCCCACTACAGCCTCAACAGTATGTACCATCTCCCACTGTAAATAGTCCCAGTTAGAACGGAATG
CCGTTGTTTTATAACTTTGAACAAATGTATTTACTGCCCTTCTCAAAA

# FIGURE 184

QCCRKIKDTVQKLASDHKDIHSSVSRVGKAIDRNFDSEICGVVSDAVWDAREQQQQILQMAIV
EHLYQQGMLSVAEELCQESTLNVDLDFKQPFLELNRILEALHEQDLGPALEWAVSHRQRLLEL
NSSLEFKLHRLHFIRLLAGGPAKQLEALSYARHFQPFARLHQREIQVMMGSLVYLRLGLEKSP
YCHLLDSSHWAEICETFTRDACSLLGLSVESPLSVSFASGCVALPVLMNIKAVIEQRQCTGVW
NHKDELPIEIELGMKCWYHSVFACPILRQQTSDSNPPIKLICGHVISRDALNKLINGGKLKCP
YCPMEQNPADGKRIIF

**Transmembrane domain:**

amino acids 222-241

**N-glycosylation site.**

amino acids 129-133

**Tyrosine kinase phosphorylation site.**

amino acids 151-159, 184-193

**Amidation site.**

amino acids 327-331

**Prokaryotic membrane lipoprotein lipid attachment site.**

amino acids 222-233

# FIGURE 185

GAGCGACGCTGTCTCTAGTCGCTGATCCCAA**ATG**CACCGGCTCATCTTTGTCTACACTCTAAT
CTGCGCAAACTTTTGCAGCTGTCGGGACACTTCTGCAACCCCGCAGAGCGCATCCATCAAAGC
TTTGCGCAACGCCAACCTCAGGCGAGATGACTTGTACCGAAGAGATGAGACCATCCAGGTGAA
AGGAAACGGCTACGTGCAGAGTCCTAGATTCCCGAACAGCTACCCCAGGAACCTGCTCCTGAC
ATGGCGGCTTCACTCTCAGGAGAATACACGGATACAGCTAGTGTTTGACAATCAGTTTGGATT
AGAGGAAGCAGAAAATGATATCTGTAGGTATGATTTTGTGGAAGTTGAAGATATATCCGAAAC
CAGTACCATTATTAGAGGACGATGGTGTGGACACAAGGAAGTTCCTCCAAGGATAAAATCAAG
AACGAACCAAATTAAAATCACATTCAAGTCCGATGACTACTTTGTGGCTAAACCTGGATTCAA
GATTTATTATTCTTTGCTGGAAGATTTCCAACCCGCAGCAGCTTCAGAGACCAACTGGGAATC
TGTCACAAGCTCTATTTCAGGGGTATCCTATAACTCTCCATCAGTAACGGATCCCACTCTGAT
TGCGGATGCTCTGGACAAAAAAATTGCAGAATTTGATACAGTGGAAGATCTGCTCAAGTACTT
CAATCCAGAGTCATGGCAAGAAGATCTTGAGAATATGTATCTGGACACCCCTCGGTATCGAGG
CAGGTCATACCATGACCGGAAGTCAAAAGTTGACCTGGATAGGCTCAATGATGATGCCAAGCG
TTACAGTTGCACTCCCAGGAATTACTCGGTCAATATAAGAGAAGAGCTGAAGTTGGCCAATGT
GGTCTTCTTTCCACGTTGCCTCCTCGTGCAGCGCTGTGGAGGAAATTGTGGCTGTGGAACTGT
CAACTGGAGGTCCTGCACATGCAATTCAGGGAAAACCGTGAAAAAGTATCATGAGGTATTACA
GTTTGAGCCTGGCCACATCAAGAGGAGGGGTAGAGCTAAGACCATGGCTCTAGTTGACATCCA
GTTGGATCACCATGAACGATGCGATTGTATCTGCAGCTCAAGACCACCTCGA**TAA**GAGAATGT
GCACATCCTTACATTAAGCCTGAGAGAA

# FIGURE 186

MHRLIFVYTLICANFCSCRDTSATPQSASIKALRNANLRRDDLYRRDETIQVKGNGYVQSPRF
PNSYPRNLLLTWRLHSQENTRIQLVFDNQFGLEEAENDICRYDFVEVEDISETSTIIRGRWCG
HKEVPPRIKSRTNQIKITFKSDDYFVAKPGFKIYYSLLEDFQPAAASETNWESVTSSISGVSY
NSPSVTDPTLIADALDKKIAEFDTVEDLLKYFNPESWQEDLENMYLDTPRYRGRSYHDRKSKV
DLDRLNDDAKRYSCTPRNYSVNIREELKLANVVFFPRCLLVQRCGGNCGCGTVNWRSCTCNSG
KTVKKYHEVLQFEPGHIKRRGRAKTMALVDIQLDHHERCDCICSSRPPR

**Signal peptide:**
amino acids 1-18

**N-glycosylation site.**
amino acids 270-274

**cAMP- and cGMP-dependent protein kinase phosphorylation site.**
amino acids 262-266

**Tyrosine kinase phosphorylation site.**
amino acids 256-265

**N-myristoylation sites.**
amino acids 94-100, 186-192, 297-303, 298-304

**TonB-dependent receptor proteins signature 1.**
amino acids 1-56

# FIGURE 187

C**ATG**CCGCTGCCGCCGCTGCTGCTGTTGCTCCTGGCGGCGCCTTGGGGACGGGCAGTTCCCTG
TGTCTCTGGTGGTTTGCCTAAACCTGCAAACATCACCTTCTTATCCATCAACATGAAGAATGT
CCTACAATGGACTCCACCAGAGGGTCTTCAAGGAGTTAAAGTTACTTACACTGTGCAGTATTT
CATATATGGGCAAAAGAAATGGCTGAATAAATCAGAATGCAGAAATATCAATAGAACCTACTG
TGATCTTTCTGCTGAAACTTCTGACTACGAACACCAGTATTATGCCAAAGTTAAGGCCATTTG
GGGAACAAAGTGTTCCAAATGGGCTGAAAGTGGACGGTTCTATCCTTTTTTAGAAACACAAAT
TGGCCCACCAGAGGTGGCACTGACTACAGATGAGAAGTCCATTTCTGTTGTCCTGACAGCTCC
AGAGAAGTGGAAGAGAAATCCAGAAGACCTTCCTGTTTCCATGCAACAAATATACTCCAATCT
GAAGTATAACGTGTCTGTGTTGAATACTAAATCAAACAGAACGTGGTCCCAGTGTGTGACCAA
CCACACGCTGGTGCTCACCTGGCTGGAGCCGAACACTCTTTACTGCGTACACGTGGAGTCCTT
CGTCCCAGGGCCCCCTCGCCGTGCTCAGCCTTCTGAGAAGCAGTGTGCCAGGACTTTGAAAGA
TCAATCATCAGAGTTCAAGGCTAAAATCATCTTCTGGTATGTTTTGCCCATATCTATTACCGT
GTTTCTTTTTTCTGTGATGGGCTATTCCATCTACCGATATATCCACGTTGGCAAAGAGAAACA
CCCAGCAAATTTGATTTTGATTTATGGAAATGAATTTGACAAAAGATTCTTTGTGCCTGCTGA
AAAAATCGTGATTAACTTTATCACCCTCAATATCTCGGATGATTCTAAAATTTCTCATCAGGA
TATGAGTTTACTGGGAAAAAGCAGTGATGTATCCAGCCTTAATGATCCTCAGCCCAGCGGGAA
CCTGAGGCCCCCTCAGGAGGAAGAGGAGGTGAAACATTTAGGGTATGCTTCGCATTTGATGGA
AATTTTTTGTGACTCTGAAGAAAACACGGAAGGTACTTCTCTCACCCAGCAAGAGTCCCTCAG
CAGAACAATACCCCCGGATAAAACAGTCATTGAATATGAATATGATGTCAGAACCACTGACAT
TTGTGCGGGGCCTGAAGAGCAGGAGCTCAGTTTGCAGGAGGAGGTGTCCACACAAGGAACATT
ATTGGAGTCGCAGGCAGCGTTGGCAGTCTTGGGCCCGCAAACGTTACAGTACTCATACACCCC
TCAGCTCCAAGACTTAGACCCCCTGGCGCAGGAGCACACAGACTCGGAGGAGGGGCCGGAGGA
AGAGCCATCGACGACCCTGGTCGACTGGGATCCCCAAACTGGCAGGCTGTGTATTCCTTCGCT
GTCCAGCTTCGACCAGGATTCAGAGGGCTGCGAGCCTTCTGAGGGGGATGGGCTCGGAGAGGA
GGGTCTTCTATCTAGACTCTATGAGGAGCCGGCTCCAGACAGGCCACCAGGAGAAAATGAAAC
CTATCTCATGCAATTCATGGAGGAATGGGGGTTATATGTGCAGATGGAAAAC**TGA**TGCCAACA
CTTCCTTTTGCCTTTTGTTTCCTGTGCAAACAAGTGAGTCACCCCTTTGATCCCAGCCATAAA
GTACCTGGGATGAAAGAAGTTTTTCCAGTTTGTCAGTGTCTGTGAGAA

# FIGURE 188

MPLPPLLLLLLAAPWGRAVPCVSGGLPKPANITFLSINMKNVLQWTPPEGLQGVKVTYTVQYF
IYGQKKWLNKSECRNINRTYCDLSAETSDYEHQYYAKVKAIWGTKCSKWAESGRFYPFLETQI
GPPEVALTTDEKSISVVLTAPEKWKRNPEDLPVSMQQIYSNLKYNVSVLNTKSNRTWSQCVTN
HTLVLTWLEPNTLYCVHVESFVPGPPRRAQPSEKQCARTLKDQSSEFKAKIIFWYVLPISITV
FLFSVMGYSIYRYIHVGKEKHPANLILIYGNEFDKRFFVPAEKIVINFITLNISDDSKISHQD
MSLLGKSSDVSSLNDPQPSGNLRPPQEEEEVKHLGYASHLMEIFCDSEENTEGTSLTQQESLS
RTIPPDKTVIEYEYDVRTTDICAGPEEQELSLQEEVSTQGTLLESQAALAVLGPQTLQYSYTP
QLQDLDPLAQEHTDSEEGPEEEPSTTLVDWDPQTGRLCIPSLSSFDQDSEGCEPSEGDGLGEE
GLLSRLYEEPAPDRPPGENETYLMQFMEEWGLYVQMEN

**Signal sequence:**
amino acids 1-18

**Transmembrane domain:**
amino acids 240-260

**N-glycosylation sites.**
amino acids 31-34, 72-75, 80-83, 171-174, 180-183, 189-192, 304-307, 523-526

**Tyrosine kinase phosphorylation site.**
amino acids 385-392, 518-526

**N-myristoylation sites.**
amino acids 53-58, 106-111, 368-373, 492-497

**Tissue factor**
amino acids 1-278

# FIGURE 189

**ATG**TGCTGCTGGCCGCTGCTCCTGCTGTGGGGGCTGCTCCCCGGGACGGCGGCGGGGGGCTCG
GGCCGAACCTATCCGCACCGGACCCTCCTGGACTCGGAGGGCAAGTACTGGCTGGGCTGGAGC
CAGCGGGGCAGCCAGATCGCCTTCCGCCTCCAGGTGCGCACTGCAGGCTACGTGGGCTTCGGC
TTCTCGCCCACCGGGGCCATGGCGTCCGCCGACATCGTCGTGGGCGGGGTGGCCCACGGGCGG
CCCTACCTCCAGGATTATTTTACAAATGCAAATAGAGAGTTGAAAAAAGATGCTCAGCAAGAT
TACCATCTAGAATATGCCATGGAAAATAGCACACACACAATAATTGAATTTACCAGAGAGCTG
CATACATGTGACATAAATGACAAGAGTATAACGGATAGCACTGTGAGAGTGATCTGGGCCTAC
CACCATGAAGATGCAGGAGAAGCTGGTCCCAAGTACCATGACTCCAATAGGGGCACCAAGAGT
TTGCGGTTATTGAATCCTGAGAAAACTAGTGTGCTATCTACAGCCTTACCATACTTTGATCTG
GTAAATCAGGACGTCCCCATCCCAAACAAAGATACAACATATTGGTGCCAAATGTTTAAGATT
CCTGTGTTCCAAGAAAAGCATCATGTAATAAAGGTTGAGCCAGTGATACAGAGAGGCCATGAG
AGTCTGGTGCACCACATCCTGCTCTATCAGTGCAGCAACAACTTTAACGACAGCGTTCTGGAG
TCCGGCCACGAGTGCTATCACCCCAACATGCCCGATGCATTCCTCACCTGTGAAACTGTGATT
TTTGCCTGGGCTATTGGTGGAGAGGGCTTTTCTTATCCACCTCATGTTGGATTATCCCTTGGC
ACTCCATTAGATCCGCATTATGTGCTCCTAGAAGTCCATTATGATAATCCCACTTATGAGGAA
GGCTTAATAGATAATTCTGGACTGAGGTTATTTTACACAATGGATATAAGGAAATATGATGCT
GGGGTGATTGAGGCTGGCCTCTGGGTGAGCCTCTTCCATACCATCCCTCCAGGGATGCCTGAG
TTCCAGTCTGAGGGTCACTGCACTTTGGAGTGCCTGGAAGAGGCTCTGGAAGCCGAAAAGCCA
AGTGGAATTCATGTGTTTGCTGTTCTTCTCCATGCTCACCTGGCTGGCAGAGGCATCAGGCTG
CGTCATTTTCGAAAAGGGAAGGAAATGAAATTACTTGCCTATGATGATGATTTTGACTTCAAT
TTCCAGGAGTTTCAGTATCTAAAGGAAGAACAAACAATCTTACCAGGAGATAACCTAATTACT
GAGTGTCGCTACAACACGAAAGATAGAGCTGAGATGACTTGGGGAGGACTAAGCACCAGGAGT
GAAATGTGTCTCTCATACCTTCTTTATTACCCAAGAATTAATCTTACTCGATGTGCAAGTATT
CCAGACATTATGGAACAACTTCAGTTCATTGGGGTTAAGGAGATCTACAGACCAGTCACGACC
TGGCCTTTCATTATCAAAAGTCCCAAGCAATATAAAAACCTTTCTTTCATGGATGCTATGAAT
AAGTTTAAATGGACTAAAAAGGAAGGTCTCTCCTTCAACAAGCTGGTCCTCAGCCTGCCAGTG
AATGTGAGATGTTCCAAGACAGACAATGCTGAGTGGTCGATTCAAGGAATGACAGCATTACCT
CCAGATATAGAAAGACCCTATAAAGCAGAACCTTTGGTGTGTGGCACGTCTTCTTCCTCTTCC
CTGCACAGAGATTTCTCCATCAACTTGCTTGTTTGCCTTCTGCTACTCAGCTGCACGCTGAGC
ACCAAGAGCTTG**TGA**TCAAAATTCTGTTGGACTTGACAATGTTTTCTATGATCTGAACCTGTC
ATTTGAAGTACAGGTTAAAGACTGTGTCCACTTTGGGCATGAAGAGTGTGGAGACTTTTCTTC
CCCATTTTCCCTCCCTCCTTTTTCCTTTCCATGTTACATGAGAGACATCAATCAGGTTCTCTT
CTCTTTCTTAGAAATACCTGATGTTATATATACATGGTCAATAAAATAAAACTGGCCTGACTT
AAGATAACCATTTTAAAAAATTGGGCTGTCATGTGGGAATAAAAGAATTCTTTCTTTCCTAAA
AAAAAAAA

# FIGURE 190

MCCWPLLLLWGLLPGTAAGGSGRTYPHRTLLDSEGKYWLGWSQRGSQIAFRLQVRTAGYVGFG
FSPTGAMASADIVVGGVAHGRPYLQDYFTNANRELKKDAQQDYHLEYAMENSTHTIIEFTREL
HTCDINDKSITDSTVRVIWAYHHEDAGEAGPKYHDSNRGTKSLRLLNPEKTSVLSTALPYFDL
VNQDVPIPNKDTTYWCQMFKIPVFQEKHHVIKVEPVIQRGHESLVHHILLYQCSNNFNDSVLE
SGHECYHPNMPDAFLTCETVIFAWAIGGEGFSYPPHVGLSLGTPLDPHYVLLEVHYDNPTYEE
GLIDNSGLRLFYTMDIRKYDAGVIEAGLWVSLFHTIPPGMPEFQSEGHCTLECLEEALEAEKP
SGIHVFAVLLHAHLAGRGIRLRHFRKGKEMKLLAYDDDFDFNFQEFQYLKEEQTILPGDNLIT
ECRYNTKDRAEMTWGGLSTRSEMCLSYLLYYPRINLTRCASIPDIMEQLQFIGVKEIYRPVTT
WPFIIKSPKQYKNLSFMDAMNKFKWTKKEGLSFNKLVLSLPVNVRCSKTDNAEWSIQGMTALP
PDIERPYKAEPLVCGTSSSSSLHRDFSINLLVCLLLLSCTLSTKSL

**Signal peptide:**
amino acids 1-18

**Transmembrane domains:**
amino acids 56-73, 378-393, 583-602

**N-glycosylation sites.**
amino acids 114-118, 247-251, 476-480, 517-521

**N-myristoylation sites.**
amino acids 11-17, 15-21, 20-26, 45-51, 68-74, 79-85, 290-296, 316-322, 337-343, 342-348, 456-462, 534-540, 582-588

**Copper type II, ascorbate-dependent monooxygenases proteins.**
amino acids 271-321, 422-474

# FIGURE 191

GCTTCAGCTGAAGAAAGAGAGGA**ATG**AAGCGCCTTCTGCTTCTGTTTTTGTTCTTTATAACAT
TTTCTTCTGCATTTCCCTTAGTCCGGATGACGGAAAATGAAGAAAATATGCAACTGGCTCAGG
CATATCTCAACCAGTTCTACTCTCTTGAAATAGAAGGGAATCATCTTGTTCAAAGCAAGAATA
GGAGTCTCATAGATGACAAAATTCGGGAAATGCAAGCATTTTTTGGATTGACAGTGACTGGAA
AACTGGACTCAAACACCCTTGAGATCATGAAGACACCCAGGTGTGGGGTGCCTGATGTGGGCC
AGTATGGCTACACCCTCCCTGGGTGGAGAAAATACAACCTCACCTACAGAATAATAAACTATA
CTCCGGATATGGCACGAGCTGCTGTGGATGAGGCTATCCAAGAAGGTTTAGAAGTGTGGAGCA
AAGTCACTCCACTAAAATTCACCAAGATTTCAAAGGGGATTGCAGACATCATGATTGCCTTTA
GGACTCGAGTCCATGGTCGGTGTCCTCGCTATTTTGATGGTCCCTTGGGAGTGCTTGGCCATG
CCTTTCCTCCTGGTCCGGGTCTGGGTGGTGACACTCATTTTGATGAGGATGAAAACTGGACCA
AGGATGGAGCAGGATTCAACTTGTTTCTTGTGGCTGCTCATGAATTTGGTCATGCACTGGGGC
TCTCTCACTCCAATGATCAAACAGCCTTGATGTTCCCAAATTATGTCTCCCTGGATCCCAGAA
AATACCCACTTTCTCAGGATGATATCAATGGAATCCAGTCCATCTATGGAGGTCTGCCTAAGG
TACCTGCTAAGCCAAAGGAACCCACTATACCCCATGCCTGTGACCCTGACTTGACTTTTGACG
CTATCACAACTTTCCGCAGAGAAGTAATGTTCTTTAAAGGCAGGCACCTATGGAGGATCTATT
ATGATATCACGGATGTTGAGTTTGAATTAATTGCTTCATTCTGGCCATCTCTGCCAGCTGATC
TGCAAGCTGCATACGAGAACCCCAGAGATAAGATTCTGGTTTTTAAAGATGAAAACTTCTGGA
TGATCAGAGGATATGCTGTCTTGCCAGATTATCCCAAATCCATCCATACATTAGGTTTTCCAG
GACGTGTGAAGAAAATAGATGCAGCCGTCTGTGATAAGACCACAAGAAAAACCTACTTCTTTG
TGGGCATTTGGTGCTGGAGGTTTGATGAAATGACCCAAACCATGGACAAAGGATTCCCGCAGA
GAGTGGTAAAACACTTTCCTGGAATCAGTATCCGTGTTGATGCTGCTTTCCAGTACAAAGGAT
TCTTCTTTTTCAGCCGTGGATCAAAGCAATTTGAATACAACATTAAGACAAAGAATATTACCC
GAATCATGAGAACTAATACTTGGTTTCAATGCAAAGAACCAAAGAACTCCTCATTTGGTTTTG
ATATCAACAAGGAAAAAGCACATTCAGGAGGCATAAAGATATTGTATCATAAGAGTTTAAGCT
TGTTTATTTTTGGTATTGTTCATTTGCTGAAAAACACTTCTATTTATCAA**TAA**ATTCATAGAC
CTAAAATAAACCTCAACAGGTCTTTTAATATAAATTCTGCTTCAAAATAGAATAAAACCATTC
TTTAACAAC

# FIGURE 192

MKRLLLLFLFFITFSSAFPLVRMTENEENMQLAQAYLNQFYSLEIEGNHLVQSKNRSLIDDKI
REMQAFFGLTVTGKLDSNTLEIMKTPRCGVPDVGQYGYTLPGWRKYNLTYRIINYTPDMARAA
VDEAIQEGLEVWSKVTPLKFTKISKGIADIMIAFRTRVHGRCPRYFDGPLGVLGHAFPPGPGL
GGDTHFDEDENWTKDGAGFNLFLVAAHEFGHALGLSHSNDQTALMFPNYVSLDPRKYPLSQDD
INGIQSIYGGLPKVPAKPKEPTIPHACDPDLTFDAITTFRREVMFFKGRHLWRIYYDITDVEF
ELIASFWPSLPADLQAAYENPRDKILVFKDENFWMIRGYAVLPDYPKSIHTLGFPGRVKKIDA
AVCDKTTRKTYFFVGIWCWRFDEMTQTMDKGFPQRVVKHFPGISIRVDAAFQYKGFFFFSRGS
KQFEYNIKTKNITRIMRTNTWFQCKEPKNSSFGFDINKEKAHSGGIKILYHKSLSLFIFGIVH
LLKNTSIYQ

**Signal peptide:**
amino acids 1-17

**N-glycosylation sites.**
amino acids 55-59, 110-114, 200-204, 452-456, 470-474, 508-512

**N-myristoylation site.**
amino acids 71-77, 205-211, 223-229

**Hemopexin domain signature.**
amino acids 171-202, 207-238, 318-334

**Neutral zinc metallopeptidases, zinc-binding region signature.**
amino acids 213-223

**Matrixins cysteine switch.**
amino acids 89-97, 207-238

# FIGURE 193

CACAATCAGGTCCCATTCTATAGATGGGGAAACTGAGGCTTGAGGTCACATAGGCGTCGTTCA
AGGCTGGTATACCTGCACCCTCTCCCATGTGAACAAC**ATG**GTTCTGGGTAATGGGGGCTGTCA
TCCAGTCTCCTCCCTGCCCCTGCTGGTGCACTTCCTGCCTCTGCTGGTGCACTTTCTGCCCCT
ACTGGTATATTTGCTGCCTCTGCTGGGGCGCTTCCTGCCTCGGCTGGTGTATCTCCTGCCCCT
GCTGGTGCACTTTCTGCCCCCGCTGATGCACTTCCTGCCTCTGCTGGTGCACTTCCTGGCTCT
GCTGGCACACTTCCTGCCTCTGCTGGTGCACTTCCTGGCTCTGCTGGCGCACTTTCCTGCCCC
TGCTGGTGTATTTCCTGCCCCTGCTGGTGTACTTCCTTCCCCTGCTGGTGCACTTCCTGCCTC
TGCTGGCGCACTTCTTGCCTCTCCAGGCCCTACC**TAG**CCTCTCCCTCTTATATATGGAAGTCT
TCCCAGTTCACTGACACTGGTAACAGGGACTCTGCTCTTGGTGTTGCTGTCTGCCCTGGGGAT
GGGCATCTGTGTCTTCCTTTACTACTGCTGGCTCAGGACCCAGAGCTTTGAAGCATGTCCAGA
TGCAGGTCCGGGCACCAGAGTCTAAGGAGCCCCTACACCCACCAGGATTTTCCAATAAAGAGA
TGTTCACCA

# FIGURE 194

MVLGNGGCHPVSSLPLLVHFLPLLVHFLPLLVYLLPLLGRFLPRLVYLLPLLVHFLPPLMHFL
PLLVHFLALLAHFLPLLVHFLALLAHFPAPAGVFPAPAGVLPSPAGALPASAGALLASPGPT

**Signal peptide:**
amino acids 1-39

**N-myristoylation sites.**
amino acids 4-10, 109-115, 116-122

**Leucine zipper pattern.**
amino acids 14-36, 16-38, 17-39, 21-43, 24-46, 28-50, 31-53, 35-57, 38-60, 42-64, 45-67, 49-71, 52-74, 56-78, 59-81, 63-85, 65-87, 66-88

# FIGURE 195

GGCAAGGCGGCGGCGGCGGCGGCAGCCGCGGTGGCGGCGTGGGGAACATCTCGGCAGCCA
CCGCGCTTCTCCCGCTGGAGCGGGCGTCCAGCTTGGCTGCCCTCGGTCCTTCCCTGCCACGTT
TCGGGTCGCCCTGCACCCCCCACCCAGGCTCGCTTCTCTTCGAAGCGGGAAGGGCGCCTTGCA
GGATCCTGCCGCCCCTCCAACCGGATCCTGGGTCTAGAGCTCCCCAGAGCGAGGCGCTCGCCA
GGACTCCTGCCCCGCCAACCCTGACCGCCGGGGGGTGCCCCCGGGACGTAGCGCCGCGGAGAG
GAAGCGGCAAAGGGGACC**ATG**CGGCGCCTGACTCGTCGGCTGGTTCTGCCAGTCTTCGGGGTG
CTCTGGATCACGGTGCTGCTGTTCTTCTGGGTAACCAAGAGGAAGTTGGAGGTGCCGACGGGA
CCTGAAGTGCAGACCCCTAAGCCTTCGGACGCTGACTGGGACGACCTGTGGGACCAGTTTGAT
GAGCGGCGGTATCTGAATGCCAAAAAGTGGCGCGTTGGTGACGACCCCTATAAGCTGTATGCT
TTCAACCAGCGGGAGAGTGAGCGGATCTCCAGCAATCGGGCCATCCCGGACACTCGCCATCTG
AGATGCACACTGCTGGTGTATTGCACGGACCTTCCACCCACTAGCATCATCATCACCTTCCAC
AACGAGGCCCGCTCCACGCTGCTCAGGACCATCCGCAGTGTATTAAACCGCACCCCTACGCAT
CTGATCCGGGAAATCATATTAGTGGATGACTTCAGCAATGACCCTGATGACTGTAAACAGCTC
ATCAAGTTGCCCAAGGTGAAATGCTTGCGCAATAATGAACGGCAAGGTCTGGTCCGGTCCCGG
ATTCGGGGCGCTGACATCGCCCAGGGCACCACTCTGACTTTCCTCGACAGCCACTGTGAGGTG
AACAGGGACTGGCTCCAGCCTCTGTTGCACAGGGTCAAAGAGGACTACACGCGGGTGGTGTGC
CCTGTGATCGATATCATTAACCTGGACACCTTCACCTACATCGAGTCTGCCTCGGAGCTCAGA
GGGGGGTTTGACTGGAGCCTCCACTTCCAGTGGGAGCAGCTCTCCCCAGAGCAGAAGGCTCGG
CGCCTGGACCCCACGGAGCCCATCAGGACTCCTATCATAGCTGGAGGGCTCTTCGTGATCGAC
AAAGCTTGGTTTGATTACCTGGGGAAATATGATATGGACATGGACATCTGGGGTGGGGAGAAC
TTTGAAATCTCCTTCCGAGTGTGGATGTGCGGGGGCAGCCTAGAGATCGTCCCCTGCAGCCGA
GTGGGGCACGTCTTCCGGAAGAAGCACCCCTACGTTTTCCCTGATGGAAATGCCAACACGTAT
ATAAAGAACACCAAGCGGACAGCTGAAGTGTGGATGGATGAATACAAGCAATACTATTACGCT
GCCCGGCCATTCGCCCTGGAGAGGCCCTTCGGGAATGTTGAGAGCAGATTGGACCTGAGGAAG
AATCTGCGCTGCCAGAGCTTCAAGTGGTACCTGGAGAATATCTACCCTGAACTCAGCATCCCC
AAGGAGTCCTCCATCCAGAAGGGCAATATCCGACAGAGACAGAAGTGCCTGGAATCTCAAAGG
CAGAACAACCAAGAAACCCCAAACCTAAAGTTGAGCCCCTGTGCCAAGGTCAAAGGCGAAGAT
GCAAAGTCCCAGGTATGGGCCTTCACATACACCCAGCAGATCCTCCAGGAGGAGCTGTGCCTG
TCAGTCATCACCTTGTTCCCTGGCGCCCCAGTGGTTCTTGTCCTTTGCAAGAATGGAGATGAC
CGACAGCAATGGACCAAAACTGGTTCCCACATCGAGCACATAGCATCCCACCTCTGCCTCGAT
ACAGATATGTTCGGTGATGGCACCGAGAACGGCAAGGAAATCGTCGTCAACCCATGTGAGTCC
TCACTCATGAGCCAGCACTGGGACATGGTGAGCTCT**TGA**GGACCCCTGCCAGAAGCAGCAAGG
GCCATGGGGTGGTGCTTCCCTGGACCAGAACAGACTGGAAACTGGGCAGCAAGCAGCCTGCAA
CCACCTCAGACATCCTGGACTGGGAGGTGGAGGCAGAGCCCCCCAGGACAGGAGCAACTGTCT
CAGGGAGGACAGAGGAAAACATCACAAGCCAATGGGCTCAAAGACAAATCCCACATGTTCTCA
AGGCCGTTAAGTTCCAGTCCTGGCCAGTCATTCCCTGATTGGTATCTGGAGACAGAAACCTAA
TGGGAAGTGTTTATTGTTCCTTTTCCTACAAAGGAAGCAGTCTCTGGAGGCCAGAAAGAAAAG
CCTTCTTTTTCACTAGGCCAGGACTACATTGAGAGATGAAGAATGGAGGTTGTTTCCAAAAGA
AATAAAGAGAAACTTAGAAGTTGTCTCTGGAAAAAAAAAAAAAAAAAAAAAAAAAAAAAA
AAAAAAAAAAAAAAAAA

# FIGURE 196

MRRLTRRLVLPVFGVLWITVLLFFWVTKRKLEVPTGPEVQTPKPSDADWDDLWDQFDERRYLN
AKKWRVGDDPYKLYAFNQRESERISSNRAIPDTRHLRCTLLVYCTDLPPTSIIITFHNEARST
LLRTIRSVLNRTPTHLIREIILVDDFSNDPDDCKQLIKLPKVKCLRNNERQGLVRSRIRGADI
AQGTTLTFLDSHCEVNRDWLQPLLHRVKEDYTRVVCPVIDIINLDTFTYIESASELRGGFDWS
LHFQWEQLSPEQKARRLDPTEPIRTPIIAGGLFVIDKAWFDYLGKYDMDMDIWGGENFEISFR
VWMCGGSLEIVPCSRVGHVFRKKHPYVFPDGNANTYIKNTKRTAEVWMDEYKQYYYAARPFAL
ERPFGNVESRLDLRKNLRCQSFKWYLENIYPELSIPKESSIQKGNIRQRQKCLESQRQNNQET
PNLKLSPCAKVKGEDAKSQVWAFTYTQQILQEELCLSVITLFPGAPVVLVLCKNGDDRQQWTK
TGSHIEHIASHLCLDTDMFGDGTENGKEIVVNPCESSLMSQHWDMVSS

**Transmembrane domain:**

amino acids 475-493

**cAMP- and cGMP-dependent protein kinase phosphorylation site.**

amino acids 2-6

**Tyrosine kinase phosphorylation sites.**

amino acids 68-75, 401-409

**N-myristoylation sites.**

amino acids 178-184, 186-192, 192-198, 346-352, 383-389, 526-532

# FIGURE 197

GCAGCTCACCCTTCGCAGCCGCG**ATG**GGGGAAGACGACGCCGCGCTTCGGGCTGGCAGCAGGGGGCTCTCCGACC
CGTGGGCAGACTCAGTGGGAGTGCGACCCCGCACCACGGAGCGCCACATCGCCGTACACAAGCGGCTTGTGCTGG
CCTTCGCTGTGTCCCTCGTGGCATTGCTCGCGGTCACAATGCTCGCTGTGCTGCTCAGCCTGCGCTTCGACGAGT
GCGGGGCGAGTGCCACGCCAGGCGCCGACGGTGGCCCCTCAGGCTTTCCGGAGCGCGGCGGCAACGGGAGCCTCC
CTGGATCGGCCCGGCGCAACCACCACGCAGGCGGGGACTCCTGGCAGCCCGAGGCGGGTGGGGTGGCCAGTCCGG
GGACCACGTCGGCCCAGCCGCCGTCGGAGGAGGAGCGGGAGCCGTGGGAGCCGTGGACGCAGCTGCGCCTGTCGG
GCCACCTGAAGCCGCTGCACTACAATCTGATGCTCACCGCCTTCATGGAGAACTTCACCTTCTCCGGGGAGGTCA
ACGTGGAGATCGCGTGCCGGAACGCCACCCGCTACGTAGTGCTGCACGCTTCCCGAGTGGCGGTGGAGAAAGTGC
AGCTGGCCGAGGACCGGGCGTTCGGGGCTGTCCCTGTAGCCGGTTTTTCCTCTACCCGCAAACCCAGGTCTTAG
TGGTGGTGCTGAATAGGACACTGGACGCGCAGAGGAATTACAATCTGAAGATTATCTACAACGCGCTCATCGAGA
ATGAGCTCCTGGGCTTCTTCCGCAGCTCCTATGTGCTCCACGGGGAGAGAAGATTCCTTGGTGTTACTCAGTTTT
CGCCTACACATGCCAGAAAGGCATTTCCTTGTTTTGATGAGCCAATCTACAAGGCTACTTTCAAAATCAGCATCA
AGCATCAAGCAACCTATTTATCTTTATCTAATATGCCAGTGGAAACTTCCGTGTTTGAGGAAGATGGATGGGTTA
CGGATCACTTTTCACAGACCCCTCTCATGTCCACATATTATTTAGCCTGGGCAATTTGCAACTTCACATACAGAG
AAACTACCACCAAGAGTGGGGTTGTAGTACGATTATATGCAAGACCTGATGCTATCAGAAGAGGATCCGGGGACT
ATGCTCTCCATATAACAAAGAGATTAATAGAATTTTATGAAGACTACTTTAAAGTGCCCTATTCCTTGCCAAAAC
TAGATCTTTTAGCTGTGCCTAAGCATCCGTATGCTGCTATGGAGAACTGGGGACTAAGTATTTTTGTGGAACAAA
GAATACTGCTGGATCCCAGTGTTTCATCTATTTCTTATTTGCTGGATGTCACCATGGTCATTGTTCATGAGATAT
GTCACCAGTGGTTTGGTGACCTTGTGACGCCTGTGTGGTGGGAAGACGTGTGGCTGAAGGAAGGGTTTGCTCACT
ACTTTGAATTTGTTGGTACAGACTACCTCTATCCTGGCTGGAACATGGAAAAGCAGAGGTTTCTGACCGATGTTC
TGCATGAAGTGATGCTGCTGGACGGTTTGGCCAGTTCCCATCCAGTATCACAGGAAGTGCTGCAGGCAACAGATA
TTGACAGGGTGTTTGACTGGATCGCATATAAAAAGGGTGCTGCTTTAATAAGAATGCTGGCTAATTTTATGGGCC
ATTCAGTTTTCCAGAGGGGTTTGCAAGATTATTTAACCATTCATAAGTATGGTAATGCAGCCAGAAATGATCTCT
GGAATACATTATCGGAGGCTTTAAAAAGAAATGGGAAATATGTAAATATACAAGAAGTAATGGATCAGTGGACAC
TCCAGATGGGTTATCCTGTTATCACCATCTTGGGAAACACAACAGCAGAAAATAGAATAATAATTACCCAACAGC
ATTTTATCTATGATATCAGTGCTAAAACTAAAGCACTTAAACTTCAGAATAACAGTTACCTGTGGCAGATTCCAT
TAACTATTGTGGTAGGAAATAGAAGCCATGTGTCTTCAGAAGCAATTATTTGGGTGTCTAACAAATCAGAGCACC
ACAGAATAACTTATTTGGACAAAGGAAGCTGGCTGCTGGGGAACATCAATCAAACTGGCTATTTTAGAGTCAACT
ATGACCTAAGGAACTGGAGATTATTAATTGATCAATTAATCCGGAATCATGAGGTTCTTTCTGTCAGTAACCGAG
CGGGCTTGATCGATGATGCCTTCAGCCTAGCCAGGGCTGGCTATTTGCCTCAGAATATTCCTCTGGAGATTATCA
GATACCTGTCTGAGGAGAAGGATTTTCTTCCTTGGCATGCTGCCAGCCGAGCTCTTTATCCTCTAGATAAATTAC
TGGACCGCATGGAAAACTACAACATTTTCAATGAATATATTTTAAAGCAAGTTGCAACAACATATATCAAGCTTG
GGTGGCCGAAAAATAATTTTAATGGATCTCTTGTTCAAGCATCCTACCAACATGAAGAACTACGTAGAGAAGTTA
TAATGCTGGCCTGCAGTTTTGGCAACAAGCACTGTCACCAACAGGCATCAACACTTATTTCAGATTGGATTTCCA
GCAACAGGAACAGAATACCACTAAATGTTAGAGACATCGTATACTGTACAGGAGTGTCACTACTGGATGAGGATG
TCTGGGAATTCATATGGATGAAATTCCATTCCACCACAGCAGTTTCTGAGAAGAAAATATTATTGGAAGCCTTAA
CTTGCAGTGATGACAGGAATTTATTAAACAGGCTTCTAAATCTGTCACTGAATTCTGAGGTGGTGCTGGATCAAG
ATGCAATTGATGTCATAATCCATGTAGCTCGAAATCCACATGGTCGAGACCTTGCCTGGAAGTTTTTCAGGGATA
AATGGAAGATATTAAATACCAGGTATGGAGAAGCATTGTTTATGTATTCCAAACTCATCAGTGGTGTCACAGAAT
TTCTTAATACTGAAGGTGAACTCAAAGAGCTCAAGAACTTCATGAAAAACTATGATGGGGTAGCTGCTGCTTCTT
TCTCACGAGCTGTGGAAACTGTCGAAGCCAATGTGCGCTGGAAAATGCTTTACCAAGACGAGCTTTTCCAATGGT
TAGGAAAAGCTCTAAGACAC**TAA**TATATGTATCTTATAAACAAACAATTCAACTCAGAAGTTTATGAGAAGACAC
GCTTTTTGTGGAATGAGGAAAATGTACTACCTAGAAAATGGCCAGATTTTCAGTGTTAACGTGTGGGAGGAATTT
TTTTTTTTAGTTTTTATTTTTTGGTTTTGGGGGATATTTTTTATTTGTTTCATTCATTCTGTTCTGTTTCTCTAC
TGGGTGTTCCTCTCTAAAGAAACTCTTGCAAGTGAAACTAGCCATGATTGCTTCAGCTGTACATTCCTTGCTGTA
CAGGACCAAATATGATAGTGATGCATGTTGATGTTACAGTCAATTTGGAAAAACATATTCAGAATATCTGTGCAT
GGATATATTGTCCTGCCTGTGTTCCAGCATGCTTATTTCAAACGTCCAGTGTTGTGTGTGAATATGTGTTACACC
TAGGATGGGCATTATGCAAAAGCACAAAGATTATATATGACAATCAGTATTGCAATGAAAGAAAAACTAAAAACA
GAAATGATATTCTCAATTTTGGGCAATGTGAGAGGTAAAATAGCCCTTGACATGATGAACATCACTTATTTCAGC
ACTTGGATTGTCTGGCAATGATTACTGTGTTGCTAACTCATTTTCTTTGAGTTAAAGCTGTGTATACATTTTAAA
AGGCATATAGATAGTGTATGCATATGTATATGTACATAGGGAAGCCCCATATGTATATAGTATGTTGTACACTGC
ACATGTACAAAGAATGTCTTCAGATCAAAGAAAATTTATCTCTTTTTATAAACTTAAGGACAGTTGCAAAAGGCT
TCAAGGAATTTATCTCAACATTATTCTTTCTATGTCCTAACTAAATTTCTCAACTGTTATGAATTTTTCATCTAC
TTCTTGAACAGTGGTCTATTCTGCTACATGAAGATGAATACAAACAAAATTTTTGTATAAACTCCCAAAAAAAAA
AAAAAAAAAA

# FIGURE 198

MGEDDAALRAGSRGLSDPWADSVGVRPRTTERHIAVHKRLVLAFAVSLVALLAVTMLAVLLSL
RFDECGASATPGADGGPSGFPERGGNGSLPGSARRNHHAGGDSWQPEAGGVASPGTTSAQPPS
EEEREPWEPWTQLRLSGHLKPLHYNLMLTAFMENFTFSGEVNVEIACRNATRYVVLHASRVAV
EKVQLAEDRAFGAVPVAGFFLYPQTQVLVVVLNRTLDAQRNYNLKIIYNALIENELLGFFRSS
YVLHGERRFLGVTQFSPTHARKAFPCFDEPIYKATFKISIKHQATYLSLSNMPVETSVFEEDG
WVTDHFSQTPLMSTYYLAWAICNFTYRETTTKSGVVVRLYARPDAIRRGSGDYALHITKRLIE
FYEDYFKVPYSLPKLDLLAVPKHPYAAMENWGLSIFVEQRILLDPSVSSISYLLDVTMVIVHE
ICHQWFGDLVTPVWWEDVWLKEGFAHYFEFVGTDYLYPGWNMEKQRFLTDVLHEVMLLDGLAS
SHPVSQEVLQATDIDRVFDWIAYKKGAALIRMLANFMGHSVFQRGLQDYLTIHKYGNAARNDL
WNTLSEALKRNGKYVNIQEVMDQWTLQMGYPVITILGNTTAENRIIITQQHFIYDISAKTKAL
KLQNNSYLWQIPLTIVVGNRSHVSSEAIIWVSNKSEHHRITYLDKGSWLLGNINQTGYFRVNY
DLRNWRLLIDQLIRNHEVLSVSNRAGLIDDAFSLARAGYLPQNIPLEIIRYLSEEKDFLPWHA
ASRALYPLDKLLDRMENYNIFNEYILKQVATTYIKLGWPKNNFNGSLVQASYQHEELRREVIM
LACSFGNKHCHQQASTLISDWISSNRNRIPLNVRDIVYCTGVSLLDEDVWEFIWMKFHSTTAV
SEKKILLEALTCSDDRNLLNRLLNLSLNSEVVLDQDAIDVIIHVARNPHGRDLAWKFFRDKWK
ILNTRYGEALFMYSKLISGVTEFLNTEGELKELKNFMKNYDGVAAASFSRAVETVEANVRWKM
LYQDELFQWLGKALRH

**Transmembrane domain:**

amino acids 44-63

**N-glycosylation sites.**

amino acids 89-93, 160-164, 175-179, 222-226, 338-342, 605-609, 634-638, 649-653, 663-667, 684-688, 800-804, 906-910

**cAMP- and cGMP-dependent protein kinase phosphorylation site.**

amino acids 362-366

**Tyrosine kinase phosphorylation site.**

amino acids 520-528

**N-myristoylation sites.**

amino acids 78-84, 87-93, 90-96, 118-124, 501-507, 604-610, 825-831, 987-993

**Neutral zinc metallopeptidases, zinc-binding region signature.**

amino acids 437-447

# FIGURE 199

GCGCCCGGCGCAGCTCGGCCAGAGCGACCGCGGGGCTGAGCGCGCGTCCGCCCAGGGGGCTCCGGAAGCTGCCCC
GGCCCGCGGCCTCCTCCCTCGCTCCCGCTTCCCCTTTCTCGCTCACCGCCGCCCTCCTTCCCCAGCTCCCTCGCC
GTCCGCCCGCCCCACAGCCAGCGGCTCCGCGCCCCCTGCAGCCACG**ATG**CCCGCGGCCCGGCCGCCCGCCGCGGG
ACTCCGCGGGATCTCGCTGTTCCTCGCTCTGCTCCTGGGGAGCCCGGCGGCAGCGCTGGAGCGAGATGCTCTTCC
CGAGGGAGATGCTAGCCCTTTGGGTCCTTACCTCCTGCCCTCAGGAGCCCCGGAGAGAGGCAGTCCTGGCAAAGA
GCACCCTGAAGAGAGAGTGGTAACAGCGCCCCCCAGTTCCTCACAGTCGGCGGAAGTGCTGGGCGAGCTGGTGCT
GGATGGGACCGCACCCTCTGCACATCACGACATCCCAGCCCTGTCACCGCTGCTTCCAGAGGAGGCCCGCCCCAA
GCACGCCTTGCCCCCCAAGAAGAAACTGCCTTCGCTCAAGCAGGTGAACTCTGCCAGGAAGCAGCTGAGGCCCAA
GGCCACCTCCGCAGCCACTGTCCAAAGGGCAGGGTCCCAGCCAGCGTCCCAGGGCCTAGATCTCCTCTCCTCCTC
CACGGAGAAGCCTGGCCCACCGGGGGACCCGGACCCCATCGTGGCCTCCGAGGAGGCATCAGAAGTGCCCCTTTG
GCTGGATCGAAAGGAGAGTGCGGTCCCTACAACACCCGCACCCCTGCAAATCTCCCCCTTCACTTCGCAGCCCTA
TGTGGCCCACACACTCCCCCAGAGGCCAGAACCCGGGGAGCCTGGGCCTGACATGGCCCAGGAGGCCCCCCAGGA
GGACACCAGCCCCATGGCCCTGATGGACAAAGGTGAGAATGAGCTGACTGGGTCAGCCTCAGAGGAGAGCCAGGA
GACCACTACCTCCACCATTATCACCACCACGGTCATCACCACCGAGCAAGCACCAGCTCTCTGCAGTGTGAGCTT
CTCCAATCCTGAGGGGTACATTGACTCCAGCGACTACCCACTGCTGCCCCTCAACAACTTTCTGGAGTGCACATA
CAACGTGACAGTCTACACTGGCTATGGGGTGGAGCTCCAGGTGAAGAGTGTGAACCTGTCCGATGGGGAACTGCT
CTCCATCCGCGGGGTGGACGGCCCTACCCTGACCGTCCTGGCCAACCAGACACTCCTGGTGGAGGGGCAGGTAAT
CCGAAGCCCCACCAACACCATCTCCGTCTACTTCCGGACCTTCCAGGACGACGGCCTTGGGACCTTCCAGCTTCA
CTACCAGGCCTTCATGCTGAGCTGCAACTTTCCCCGCCGGCCTGACTCTGGGGATGTCACGGTGATGGACCTGCA
CTCAGGTGGGGTGGCCCACTTTCACTGCCACCTGGGCTATGAGCTCCAGGGCGCTAAGATGCTGACATGCATCAA
TGCCTCCAAGCCGCACTGGAGCAGCCAGGAGCCCATCTGCTCAGCTCCTTGTGGAGGGGCAGTGCACAATGCCAC
CATCGGCCGCGTCCTCTCCCCAAGTTACCCTGAAAACACAAATGGGAGCCAATTCTGCATCTGGACGATTGAAGC
TCCAGAGGGCCAGAAGCTGCACCTGCACTTTGAGAGGCTGTTGCTGCATGACAAGGACAGGATGACGGTTCACAG
CGGGCAGACCAACAAGTCAGCTCTTCTCTACGACTCCCTTCAAACCGAGAGTGTCCCTTTTGAGGGCCTGCTGAG
CGAAGGCAACACCATCCGCATCGAGTTCACGTCCGACCAGGCCCGGGCGGCCTCCACCTTCAACATCCGATTTGA
AGCGTTTGAGAAAGGCCACTGCTATGAGCCCTACATCCAGAATGGGAACTTCACTACATCCGACCCGACCTATAA
CATTGGGACTATAGTGGAGTTCACCTGCGACCCCGGCCACTCCCTGGAGCAGGGCCCGGCCATCATCGAATGCAT
CAATGTGCGGGACCCATACTGGAATGACACAGAGCCCCTGTGCAGAGCCATGTGTGGTGGGGAGCTCTCTGCTGT
GGCTGGGGTGGTATTGTCCCCAAACTGGCCCGAGCCCTACGTGGAAGGTGAAGATTGTATCTGGAAGATCCACGT
GGGAGAAGAGAAACGGATCTTCTTAGATATCCAGTTCCTGAATCTGAGCAACAGTGACATCTTGACCATCTACGA
TGGCGACGAGGTCATGCCCCACATCTTGGGGCAGTACCTTGGGAACAGTGGCCCCCAGAAACTGTACTCCTCCAC
GCCAGACTTAACCATCCAGTTCCATTCGGACCCTGCTGGCCTCATCTTTGGAAAGGGCCAGGGATTTATCATGAA
CTACATAGAGGTATCAAGGAATGACTCCTGCTCGGATTTACCCGAGATCCAGAATGGCTGGAAAACCACTTCTCA
CACGGAGTTGGTGCGGGGAGCCAGAATCACCTACCAGTGTGACCCCGGCTATGACATCGTGGGGAGTGACACCCT
CACCTGCCAGTGGGACCTCAGCTGGAGCAGCGACCCCCCATTTTGTGAGAAAATTATGTACTGCACCGACCCCGG
AGAGGTGGATCACTCGACCCGCTTAATTTCGGATCCTGTGCTGCTGGTGGGGACCACCATCCAATACACCTGCAA
CCCCGGTTTTGTGCTTGAAGGGAGTTCTCTTCTGACCTGCTACAGCCGTGAAACAGGGACTCCCATCTGGACGTC
TCGCCTGCCCCACTGCGTTTCGGAGGAGTCCCTGGCATGTGACAACCCAGGGCTGCCTGAAAATGGATACCAAAT
CCTGTACAAGCGACTCTACCTGCCAGGAGAGTCCCTCACCTTCATGTGCTACGAAGGCTTTGAGCTCATGGGTGA
AGTGACCATCCGCTGCATCCTGGGACAGCCATCCCACTGGAACGGCCCCTGCCCGTGTGTAAAGTTAATCAAGA
CAGTTTTGAACATGCTTTAGAAGCAGAAGCGGCAGCAGAGACGTCGCTGGAAGGGGGGAACATGGCCCTGGCTAT
CTTCATCCCGGTCCTCATCATCTCCTTACTGCTGGGAGGAGCCTACATTTACATCACAAGATGTCGCTACTATTC
CAACCTCCGCCTGCCTCTGATGTACTCCCACCCCTACAGCCAGATCACCGTGGAAACCGAGTTTGACAACCCCAT
TTACGAGACAGGGGAAACCAGAGAGTATGAGGTTTCTATC**TAA**AGAGAGCTACACTTGAGAAGGGGACTTGTGAA
CTCAACCACAATCTCCTCGAGACATTCATCCAGAGACCATGTGGCACTTGATTGAAACCCCAGAATGTCGACTGT
CTTTTGTTTAGACTCTTTATCAAAGGTTTACTGTTTTCTTCCCTGTATTTATTATATTTAAAAGTGAAAAAAAAA
AAAAAAAAAAA

# FIGURE 200

MPAARPPAAGLRGISLFLALLLGSPAAALERDALPEGDASPLGPYLLPSGAPERGSPGKEHPE
ERVVTAPPSSSQSAEVLGELVLDGTAPSAHHDIPALSPLLPEEARPKHALPPKKKLPSLKQVN
SARKQLRPKATSAATVQRAGSQPASQGLDLLSSSTEKPGPPGDPDPIVASEEASEVPLWLDRK
ESAVPTTPAPLQISPFTSQPYVAHTLPQRPEPGEPGPDMAQEAPQEDTSPMALMDKGENELTG
SASEESQETTTSTIITTTVITTEQAPALCSVSFSNPEGYIDSSDYPLLPLNNFLECTYNVTVY
TGYGVELQVKSVNLSDGELLSIRGVDGPTLTVLANQTLLVEGQVIRSPTNTISVYFRTFQDDG
LGTFQLHYQAFMLSCNFPRRPDSGDVTVMDLHSGGVAHFHCHLGYELQGAKMLTCINASKPHW
SSQEPICSAPCGGAVHNATIGRVLSPSYPENTNGSQFCIWTIEAPEGQKLHLHFERLLLHDKD
RMTVHSGQTNKSALLYDSLQTESVPFEGLLSEGNTIRIEFTSDQARAASTFNIRFEAFEKGHC
YEPYIQNGNFTTSDPTYNIGTIVEFTCDPGHSLEQGPAIIECINVRDPYWNDTEPLCRAMCGG
ELSAVAGVVLSPNWPEPYVEGEDCIWKIHVGEEKRIFLDIQFLNLSNSDILTIYDGDEVMPHI
LGQYLGNSGPQKLYSSTPDLTIQFHSDPAGLIFGKGQGFIMNYIEVSRNDSCSDLPEIQNGWK
TTSHTELVRGARITYQCDPGYDIVGSDTLTCQWDLSWSSDPPFCEKIMYCTDPGEVDHSTRLI
SDPVLLVGTTIQYTCNPGFVLEGSSLLTCYSRETGTPIWTSRLPHCVSEESLACDNPGLPENG
YQILYKRLYLPGESLTFMCYEGFELMGEVTIRCILGQPSHWNGPLPVCKVNQDSFEHALEAEA
AAETSLEGGNMALAIFIPVLIISLLLGGAYIYITRCRYYSNLRLPLMYSHPYSQITVETEFDN-
PIYETGETREYEVSI

**Signal peptide:**

amino acids 1-28

**Transmembrane domain:**

amino acids 893-915

**N-glycosylation sites.**

amino acids 311-315, 328-332, 350-354, 435-439, 458-462, 474-478, 514-518, 576-580, 618-622, 674-678, 742-746

**cAMP- and cGMP-dependent protein kinase phosphorylation site.**

amino acids 188-192

**N-myristoylation sites.**

amino acids 23-29, 87-93, 146-152, 454-460, 475-481, 575-581, 629-635, 695-701, 723-729, 766-772, 877-883, 953-959

**Prokaryotic membrane lipoprotein lipid attachment site.**

amino acids 383-394

# FIGURE 201

G**ATG**GCTACGGCAGGGGGTGGCTCTGGGGCTGACCCGGGAAGTCGGGGTCTCCTTCGCCTTCT
GTCTTTCTGCGTCCTACTAGCAGGTTTGTGCAGGGGAAACTCAGTGGAGAGGAAGATATATAT
CCCCTTAAATAAAACAGCTCCCTGTGTTCGCCTGCTCAACGCCACTCATCAGATTGGCTGCCA
GTCTTCAATTAGTGGAGACACAGGGGTTATCCACGTAGTAGAGAAAGAGGAGGACCTACAGTG
GGTATTGACTGATGGCCCCAACCCCCCTTACATGGTTCTGCTGGAGAGCAAGCATTTTACCAG
GGATTTAATGGAGAAGCTGAAAGGGAGAACCAGCCGAATTGCTGGTCTTGCAGTGTCCTTGAC
CAAGCCCAGTCCTGCCTCAGGCTTCTCTCCTAGTGTACAGTGCCCAAATGATGGGTTTGGTGT
TTACTCCAATTCCTATGGGCCAGAGTTTGCTCACTGCAGAGAAATACAGTGGAATTCGCTGGG
CAATGGTTTGGCTTATGAAGACTTTAGTTTCCCCATCTTTCTTCTTGAAGATGAAAATGAAAC
CAAAGTCATCAAGCAGTGCTATCAAGATCACAACCTGAGTCAGAATGGCTCAGCACCAACCTT
CCCACTATGTGCCATGCAGCTCTTTTCACACATGCATGCTGTCATCAGCACTGCCACCTGCAT
GCGGCGCAGCTCCATCCAAAGCACCTTCAGCATCAACCCAGAAATCGTCTGTGACCCCCTGTC
TGATTACAATGTGTGGAGCATGCTAAAGCCTATAAATACAACTGGGACATTAAAGCCTGACGA
CAGGGTTGTGGTTGCTGCCACCCGGCTGGATAGTCGTTCCTTTTTCTGGAATGTGGCCCCAGG
GGCTGAAAGCGCAGTGGCTTCCTTTGTCACCCAGCTGGCTGCTGCTGAAGCTTTGCAAAAGGC
ACCTGATGTGACCACCCTGCCCCGCAATGTCATGTTTGTCTTCTTTCAAGGGGAAACTTTTGA
CTACATTGGCAGCTCGAGGATGGTCTACGATATGGAGAAGGGCAAGTTTCCCGTGCAGTTAGA
GAATGTTGACTCATTTGTGGAGCTGGGACAGGTGGCCTTAAGAACTTCATTAGAGCTTTGGAT
GCACACAGATCCTGTTTCTCAGAAAAATGAGTCTGTACGGAACCAGGTGGAGGATCTCCTGGC
CACATTGGAGAAGAGTGGTGCTGGTGTCCCTGCTGTCATCCTCAGGAGGCCAAATCAGTCCCA
GCCTCTCCCACCATCTTCCCTGCAGCGATTTCTTCGAGCTCGAAACATCTCTGGCGTTGTTCT
GGCTGACCACTCTGGTGCCTTCCATAACAAATATTACCAGAGTATTTACGACACTGCTGAGAA
CATTAATGTGAGCTATCCCGAATGGCTGAGCCCTGAAGAGGACCTGAACTTTGTAACAGACAC
TGCCAAGGCCCTGGCAGATGTGGCCACGGTGCTGGGACGTGCTCTGTATGAGCTTGCAGGAGG
AACCAACTTCAGCGACACAGTTCAGGCTGATCCCCAAACGGTTACCCGCCTGCTCTATGGGTT
CCTGATTAAAGCCAACAACTCATGGTTCCAGTCTATCCTCAGGCAGGACCTAAGGTCCTACTT
GGGTGACGGGCCTCTTCAACATTACATCGCTGTCTCCAGCCCCACCAACACCACTTATGTTGT
ACAGTATGCCTTGGCAAATTTGACTGGCACAGTGGTCAACCTCACCCGAGAGCAGTGCCAGGA
TCCAAGTAAAGTCCCAAGTGAAAACAAGGATCTGTATGAGTACTCATGGGTCCAGGGCCCTTT
GCATTCTAATGAGACGGACCGACTCCCCCGGTGTGTGCGTTCTACTGCACGATTAGCCAGGGC
CTTGTCTCCTGCCTTTGAACTGAGTCAGTGGAGCTCTACTGAATACTCTACATGGACTGAGAG
CCGCTGGAAAGATATCCGTGCCCGGATATTTCTCATCGCCAGCAAAGAGCTTGAGTTGATCAC
CCTGACAGTGGGCTTCGGCATCCTCATCTTCTCCCTCATCGTCACCTACTGCATCAATGCCAA
AGCTGATGTCCTTTTCATTGCTCCCCGGGAGCCAGGAGCTGTGTCATAC**TGA**GGAGGACCCCA
GCTTTTCTTGCCAGNTCAGCAGTTCACTTCCTAGAGCATCTGTCCCACTGGGACACAACCACT
AATTTGTCACTGGAACCTCCCTGGGCCTGTCTCAGATTGGGATTAACATAAAAGAGTGGAACT
ATCCAAAAGAGACAGGGAGAAATAAATAAATTGCCTCCCTTCCTCCGCTCCCCTTTCCCATCA
CCCCTTCCCCATTTCCTCTTCCTTCTCTACTCATGCCAGATTTTGGGATTACAAATAGAAGCT
TCTTGCTCCTGTTTAACTCCCTAGTTACCCACCCTAATTTGCCCTTCAGGACCCTTCTACTTT
TTCCTTCCTGCCCTGTACCTCTCTCTGCTCCTCACCCCCACCCCTGTACCCAGCCACCTTCCT
GACTGGGAAGGACATAAAAGGTTTAATGTCAGGGTCAAACTACATTGAGCCCCTGAGGACAGG
GGCATCTCTGGGCTGAGCCTACTGTCTCCTTCCCACTGTCCTTTCTCCAGGCCCTCAGATGGC
ACATTAGGGTGGGCGTGCTGCGGGTGGGTATCCCACCTCCAGCCCACAGTGCTCAGTTGTACT
TTTTATTAAGCTGTAATATCTATTTTTGTTTTTGTCTTTTTCCTTTATTCTTTTTGTAAATAT
ATATATAATGAGTTTCATTAAAATAGATTATCCC

# FIGURE 202

MATAGGGSGADPGSRGLLRLLSFCVLLAGLCRGNSVERKIYIPLNKTAPCVRLLNATHQIGCQ
SSISGDTGVIHVVEKEEDLQWVLTDGPNPPYMVLLESKHFTRDLMEKLKGRTSRIAGLAVSLT
KPSPASGFSPSVQCPNDGFGVYSNSYGPEFAHCREIQWNSLGNGLAYEDFSFPIFLLEDENET
KVIKQCYQDHNLSQNGSAPTFPLCAMQLFSHMHAVISTATCMRRSSIQSTFSINPEIVCDPLS
DYNVWSMLKPINTTGTLKPDDRVVVAATRLDSRSFFWNVAPGAESAVASFVTQLAAAEALQKA
PDVTTLPRNVMFVFFQGETFDYIGSSRMVYDMEKGKFPVQLENVDSFVELGQVALRTSLELWM
HTDPVSQKNESVRNQVEDLLATLEKSGAGVPAVILRRPNQSQPLPPSSLQRFLRARNISGVVL
ADHSGAFHNKYYQSIYDTAENINVSYPEWLSPEEDLNFVTDTAKALADVATVLGRALYELAGG
TNFSDTVQADPQTVTRLLYGFLIKANNSWFQSILRQDLRSYLGDGPLQHYIAVSSPTNTTYVV
QYALANLTGTVVNLTREQCQDPSKVPSENKDLYEYSWVQGPLHSNETDRLPRCVRSTARLARA
LSPAFELSQWSSTEYSTWTESRWKDIRARIFLIASKELELITLTVGFGILIFSLIVTYCINAK
ADVLFIAPREPGAVSY

**Signal peptide:**
amino acids 1-33

**Transmembrane domain:**
amino acids 671-692

**N-glycosylation sites.**
amino acids 45-49, 55-59, 187-191, 200-204, 204-208, 264-268, 387-391, 417-421, 435-439, 464-468, 506-510, 530-534, 562-566, 573-577, 580-584, 612-616

**Glycosaminoglycan attachment site.**
amino acids 404-408

**cAMP- and cGMP-dependent protein kinase phosphorylation site.**
amino acids 232-236

**N-myristoylation site.**
amino acids 5-11, 6-12, 9-15, 29-35, 61-67, 120-126, 146-152, 168-174, 205-211, 294-300, 438-444, 446-452, 504-510, 576-582

# FIGURE 203

GCTAGACCGAGCCCTGGGAGGCTACGGGCTCCCCCGGAAACCCTGCCAGGGGAGCCGGGTTTT
GAGCTCAGGCGCCTCTAGCGGCGGCCCCCAGAAATCTGACTCGCGAGGCCAGAGTTGCAGGGA
CTGAATAGCAAACTGAGGCTGAGTAGGGAACAGACC**ATG**AGGTCAGTGCAGATCTTCCTCTCC
CAATGCCGTTTGCTCCTTCTACTAGTTCCGACAATGCTCCTTAAGTCTCTTGGCGAAGATGTA
ATTTTTCACCCTGAAGGGGAGTTTGACTCGTATGAAGTCACCATTCCTGAGAAGCTGAGCTTC
CGGGGAGAGGTGCAGGGTGTGGTCAGTCCCGTGTCCTACCTACTGCAGTTAAAAGGCAAGAAG
CACGTCCTCCATTTGTGGCCCAAGAGACTTCTGTTGCCCCGACATCTGCGCGTTTTCTCCTTC
ACAGAACATGGGGAACTGCTGGAGGATCATCCTTACATACCAAAGGACTGCAACTACATGGGC
TCCGTGAAAGAGTCTCTGGACTCTAAAGCTACTATAAGCACATGCATGGGGGGTCTCCGAGGT
GTATTTAACATTGATGCCAAACATTACCAAATTGAGCCCCTCAAGGCCTCTCCCAGTTTTGAA
CATGTCGTCTATCTCCTGAAGAAAGAGCAGTTTGGGAATCAGGTTTGTGGCTTAAGTGATGAT
GAAATAGAATGGCAGATGGCCCCTTATGAGAATAAGGCGAGGCTAAGGGACTTTCCTGGATCC
TATAAACACCCAAAGTACTTGGAATTGATCCTACTCTTTGATCAAAGTAGGTATAGGTTTGTG
AACAACAATCTTTCTCAAGTCATACATGATGCCATTCTTTTGACTGGGATTATGGACACCTAC
TTTCAAGATGTTCGTATGAGGATACACTTAAAGGCTCTTGAAGTATGGACAGATTTTAACAAA
ATACGCGTTGGATATCCAGAGTTAGCTGAAGTTTTAGGCAGATTTGTAATATATAAAAAAAGT
GTATTAAATGCTCGCCTGTCATCAGATTGGGCACATTTATATCTTCAAAGAAAATATAATGAT
GCTCTTGCATGGTCGTTTGGAAAAGTGTGTTCTCTAGAATATGCTGGATCAGTGAGTACTTTA
CTAGATACAAATATCCTTGCCCCTGCTACCTGGTCTGCTCATGAGCTGGGTCATGCTGTAGGA
ATGTCACATGATGAACAATACTGCCAATGTAGGGGTAGGCTTAATTGCATCATGGGCTCAGGA
CGCACTGGGTTTAGCAATTGCAGTTATATCTCTTTTTTTAAACATATCTCTTCGGGAGCAACA
TGTCTAAATAATATCCCAGGACTAGGTTATGTGCTTAAGAGATGTGGAAACAAAATTGTGGAG
GACAATGAGGAATGTGACTGTGGTTCCACAGAGGAGTGTCAGAAAGATCGGTGTTGCCAATCA
AATTGTAAGTTGCAACCAGGTGCCAACTGTAGCATTGGACTTTGCTGTCATGATTGTCGGTTT
CGTCCATCTGGATACGTGTGTAGGCAGGAAGGAAATGAATGTGACCTTGCAGAGTACTGCGAC
GGGAATTCAAGTTCCTGCCCAAATGACGTTTATAAGCAGGATGGAACCCCTTGCAAGTATGAA
GGCCGTTGTTTCAGGAAGGGGTGCAGATCCAGATATATGCAGTGCCAAAGCATTTTTGGACCT
GATGCCATGGAGGCTCCTAGTGAGTGCTATGATGCAGTTAACTTAATAGGTGATCAATTTGGT
AACTGTGAGATTACAGGAATTCGAAATTTTAAAAAGTGTGAAAGTGCAAATTCAATATGTGGC
AGGCTACAGTGTATAAATGTTGAAACCATCCCTGATTTGCCAGAGCATACGACTATAATTTCT
ACTCATTTACAGGCAGAAAATCTCATGTGCTGGGGCACAGGCTATCATCTATCCATGAAACCC
ATGGGAATACCTGACCTAGGTATGATAAATGATGGCACCTCCTGTGGAGAAGGCCGGGTATGT
TTTAAAAAAAATTGCGTCAATAGCTCAGTCCTGCAGTTTGACTGTTTGCCTGAGAAATGCAAT
ACCCGGGGTGTTTGCAACAACAGAAAAAACTGCCACTGCATGTATGGGTGGGCACCTCCATTC
TGTGAGGAAGTGGGGTATGGAGGAAGCATTGACAGTGGGCCTCCAGGACTGCTCAGAGGGGCG
ATTCCCTCGTCAATTTGGGTTGTGTCCATCATAATGTTTCGCCTTATTTTATTAATCCTTTCA
GTGGTTTTTGTGTTTTTCCGGCAAGTGATAGGAAACCACTTAAAACCCAAACAGGAAAAAATG
CCACTATCCAAAGCAAAAACTGAACAGGAAGAATCTAAAACAAAAACTGTACAGGAAGAATCT
AAAACAAAAACTGGACAGGAAGAATCTGAAGCAAAAACTGGACAGGAAGAATCTAAAGCAAAA
ACTGGACAGGAAGAATCTAAAGCAAACATTGAAAGTAAACGACCCAAAGCAAAGAGTGTCAAG
AAACAAAAAAAG**TAA**CCGGGCAATCCATACTCATTCAGTAACACAGGCTCATTTATTTAACCA
GCTAATCATTTATCCAAAGGCTTTCCATTCTTCTCCCAATATTTTTTTACTTTAATTTTTCCC
ACAAGTTTTGATCAGCAAATAAACAGCATTCTTGTTTTGGAAACAAAAA

# FIGURE 204

MRSVQIFLSQCRLLLLLVPTMLLKSLGEDVIFHPEGEFDSYEVTIPEKLSFRGEVQGVVSPVS
YLLQLKGKKHVLHLWPKRLLLPRHLRVFSFTEHGELLEDHPYIPKDCNYMGSVKESLDSKATI
STCMGGLRGVFNIDAKHYQIEPLKASPSFEHVVYLLKKEQFGNQVCGLSDDEIEWQMAPYENK
ARLRDFPGSYKHPKYLELILLFDQSRYRFVNNNLSQVIHDAILLTGIMDTYFQDVRMRIHLKA
LEVWTDFNKIRVGYPELAEVLGRFVIYKKSVLNARLSSDWAHLYLQRKYNDALAWSFGKVCSL
EYAGSVSTLLDTNILAPATWSAHELGHAVGMSHDEQYCQCRGRLNCIMGSGRTGFSNCSYISF
FKHISSGATCLNNIPGLGYVLKRCGNKIVEDNEECDCGSTEECQKDRCCQSNCKLQPGANCSI
GLCCHDCRFRPSGYVCRQEGNECDLAEYCDGNSSSCPNDVYKQDGTPCKYEGRCFRKGCRSRY
MQCQSIFGPDAMEAPSECYDAVNLIGDQFGNCEITGIRNFKKCESANSICGRLQCINVETIPD
LPEHTTIISTHLQAENLMCWGTGYHLSMKPMGIPDLGMINDGTSCGEGRVCFKKNCVNSSVLQ
FDCLPEKCNTRGVCNNRKNCHCMYGWAPPFCEEVGYGGSIDSGPPGLLRGAIPSSIWVVSIIM
FRLILLILSVVFVFFRQVIGNHLKPKQEKMPLSKAKTEQEESKTKTVQEESKTKTGQEESEAK
TGQEESKAKTGQEESKANIESKRPKAKSVKKQKK

**Signal peptide:**

amino acids 1-27

**Transmembrane domain:**

amino acids 684-705

**N-glycosylation sites.**

amino acids 222-226, 372-376, 438-442, 473-477, 625-629

**N-myristoylation sites.**

amino acids 131-137, 168-174, 235-241, 319-325, 364-370, 436-442, 472-478, 609-615, 642-648, 668-674, 676-680, 680-686, 749-755, 758-764, 767-773

**Amidation site.**

amino acids 69-73

**Disintegrins proteins**

amino acids 429-479

**EGF-like domain proteins**

amino acids 650-662

**Neutral zinc metallopeptidases, zinc-binding region proteins**

amino acids 335-345

# FIGURE 205

CGGACGCGTGGGCGGACGCGTGGGCGGACGCGTGGGGGAAGGTTGAATGGGGTAGAAGGCCTG
TTGTGGAGGGAAACCACCCATCCTCCTGCCTCCCACCACCACCATCATCCTGGCTGGACGGAG
AGGGTGACGGGGGCTGGGAAGGGGCAGCTCATGTTCAGGTTTCCAGGAGGGGCTACCTGTTGA
CTGTCTTTGCAGGAAGAAGAAAACACCTGAGTGACCAGATGTCCCAGCTCCAGGTGCCTTGCC
AGATGGCCAGAACCACACCTCTTGAAGAGTGACAGTGCTGTGGAGCATGGTTTCTGCACACCT
GGAATGACTGGAACCCCAAAGACTCAAGAAGGAGCTAAAGATCTTGAAGTAGACATGAATAAA
ACAGAAGGCTGTGGACCACCTGTCGAGATGGAGAAGTCCTTCTGAGGCTATCCAAACACGGAC
CAGGCCATGAGACCCCG**ATG**ACCATCCCTGAATTTTTTCGAGAGTCAGTCAACCGATTTGGAA
CTTATCCAGCCCTCCCATCCAAGAATGGCAAAAAGTGGGAAATTCTGAATTTCAACCAGTACT
ATGAGGCTTGTCGGAAGGCTGCAAAATCCTTGATCAAGCTGGGTTTGGAGCGTTTCCACGGAG
TTGGTATCCTGGGGTTTAACTCTGCAGAGTGGTTTATCACTGCTGTTGGTGCCATCCTAGCCG
GGGGTCTTTGTGTTGGTATTTATGCCACCAACTCTGCCGAGGCTTGTCAATATGTCATCACTC
ATGCCAAAGTGAACATCTTGCTGGTTGAGAATGATCAACAGTTACAGAAAATCCTTTCGATTC
CACAGAGCAGCCTAGAGCCCCTAAAAGCGATCATCCAGTACAGACTGCCAATGAAGAAGAACA
ACAACTTGTACTCTTGGGATGATTTCATGGAACTTGGCAGAAGTATCCCTGACACCCAACTGG
AGCAGGTCATCGAGAGCCAGAAGGCGAATCAATGCGCAGTGCTCATCTACACTTCAGGGACCA
CAGGCATACCCAAGGGAGTGATGCTCAGTCATGACAACATCACGTGGATTGCAGGAGCAGTGA
CAAAGGACTTTAAACTGACAGACAAGCATGAGACGGTGGTTAGCTACCTCCCACTCAGCCATA
TTGCAGCACAGATGATGGACATCTGGGTACCCATAAAGATTGGGGCGCTCACATACTTTGCTC
AAGCAGATGCTCTCAAGGGCACCTTGGTAAGTACTCTAAAGGAGGTAAAACCTACTGTCTTCA
TTGGAGTGCCTCAAATTTGGGAGAAGATACATGAGATGGTGAAGAAAAATAGTGCCAAGTCCA
TGGGCTTGAAGAAGAAGGCATTCGTGTGGGCAAGAAACATTGGCTTCAAGGTCAACTCAAAAA
AGATGTTGGGGAAATATAATACTCCCGTGAGCTACCGCATGGCTAAGACTCTCGTGTTCAGCA
AAGTCAAGACATCCCTTGGCTTGGATCACTGTCACTCTTTTATCAGTGGGACTGCGCCCCTCA
ACCAAGAGACTGCCGAGTTCTTTCTAAGCTTGGACATACCTATAGGCGAGTTGTATGGGTTGA
GTGAGAGCTCGGGACCCCACACGATATCCAACCAGAATAACTACAGGCTTCTAAGCTGTGGCA
AGATCTTGACTGGGTGTAAGAATATGCTGTTCCAGCAGAACAAGGATGGCATTGGGGAGATCT
GCCTCTGGGGTAGGCACATCTTCATGGGCTATCTGGAAAGTGAGACTGAAACTACAGAGGCCA
TCGATGATGAAGGCTGGCTACACTCTGGGGATCTGGGCCAGCTGGACGGTCTGGGTTTCCTCT
ATGTCACCGGCCACATCAAAGAAATCCTTATCACTGCTGGTGGTGAAAATGTGCCCCCCATTC
CTGTTGAGACCTTGGTTAAGAAGAAGATCCCCATCATCAGTAACGCCATGTTAGTAGGAGATA
AACTGAAGTTTCTGAGCATGTTGCTGACGCTGAAGTGTGAGATGAATCAGATGAGCGGAGAAC
CTCTGGACAAGCTGAACTTCGAGGCCATCAACTTCTGTCGGGGTCTGGGCAGCCAGGCATCCA
CCGTGACTGAGATTGTGAAGCAGCAAGACCCCCTGGTCTACAAGGCCATCCAGCAAGGCATCA
ATGCTGTGAACCAGGAAGCCATGAACAATGCACAGAGGATTGAAAAGTGGGTCATCTTGGAGA
AGGACTTTTCCATCTATGGTGGAGAGCTAGGTCCAATGATGAAACTTAAGAGACATTTTGTAG
CCCAGAAATACAAAAAACAAATTGATCACATGTACCAC**TGA**CTGCTTTGATGGAGCTGCTCTC
AGCTGTTCTGATGCCTTCAGCAGGAAGACCTCATTGCAATAAGTGAAATGCTGCTCTAGGTAG
AAGCTCTCCCTGCTGTTTTTAAGAAGCCACATTCCTCATTGGTCAGTTTCTTGATTGTTCGTC
TGTTGGAGAGGTGCTCCCTAGAAGAACCTGCCATACGTTTCAAAGCAATAAAATCACTGTATA
TCTTTCTAAGGACCTTCAAGTCATGACTCCAGGGAAGCCTATTGGGAAGTCTACTAAAAACTG
CCTGATTTACAAGAAAGACCTGAACTTGTGGGCTCCCATTTGATTTTTTTCTCCTCAGGGGAC
TCAGACATTAGAAAGAAAAAGCCTCACAGATTTGAAGAACTGGACCCCCAAATCAACTCACCT
GCCTGGAAGCAACTGGGAAACCCTTCCAATAAGTCCTGATAATAAAGCACTTCAGGGTCCCAA
AAAAAAAAAA

# FIGURE 206

MTIPEFFRESVNRFGTYPALPSKNGKKWEILNFNQYYEACRKAAKSLIKLGLERFHGVGILGF
NSAEWFITAVGAILAGGLCVGIYATNSAEACQYVITHAKVNILLVENDQQLQKILSIPQSSLE
PLKAIIQYRLPMKKNNNLYSWDDFMELGRSIPDTQLEQVIESQKANQCAVLIYTSGTTGIPKG
VMLSHDNITWIAGAVTKDFKLTDKHETVVSYLPLSHIAAQMMDIWVPIKIGALTYFAQADALK
GTLVSTLKEVKPTVFIGVPQIWEKIHEMVKKNSAKSMGLKKKAFVWARNIGFKVNSKKMLGKY
NTPVSYRMAKTLVFSKVKTSLGLDHCHSFISGTAPLNQETAEFFLSLDIPIGELYGLSESSGP
HTISNQNNYRLLSCGKILTGCKNMLFQQNKDGIGEICLWGRHIFMGYLESETETTEAIDDEGW
LHSGDLGQLDGLGFLYVTGHIKEILITAGGENVPPIPVETLVKKKIPIISNAMLVGDKLKFLS
MLLTLKCEMNQMSGEPLDKLNFEAINFCRGLGSQASTVTEIVKQQDPLVYKAIQQGINAVNQE
AMNNAQRIEKWVILEKDFSIYGGELGPMMKLKRHFVAQKYKKQIDHMYH

**Signal peptide:**
amino acids 1-22

**Transmembrane domain:**
amino acids 65-86

**N-glycosylation site.**
amino acids 196-200

**cAMP- and cGMP-dependent protein kinase phosphorylation site.**
amino acids 282-286

**Tyrosine kinase phosphorylation sites.**
amino acids 547-555, 608-616

**N-myristoylation sites.**
amino acids 15-21, 74-80, 80-86, 84-90, 185-191, 189-195, 253-259, 337-343, 371-377, 448-454, 536-542

**Amidation site.**
amino acids 24-28

**Putative AMP-binding domain signature.**
amino acids 177-189

**Putative AMP-binding domain proteins.**
amino acids 173-190

# FIGURE 207

CCCACGCGTCCGCCCACGCGTCCGCGGACGCGTGGGGCCAGATCGCGGCCGGCGCCAGCGCCA
CCGTCCGGTCCACCCGCCAGCCCGCACAGCCGCGCCGCCGCCGAGCGTTTCGTGAGCGGCGCT
CCGAGGATCAGGAATGGGGCTTCGGGCGCTGGGCGCGCTCCGAACCCGGCGCACGTAAGAGCC
TGGGAGCGCCCGAGCCGCCCGGCTGCCCGGAGCCCCATCGCCTAGGACCGGGAGATGCTGGAA
ATGCAACCGCCTGTTCCCCGAGGAGCCGCTGCCCCCGGGACCCCCTGGCACTGTGCGCACCCT
GGTCAGCAGCCCCCGGAGAAGACGGCGCCCCCAACGCCCGACCCGCGTGGCCGTGGCAGCGCC
ACGCGAGCCCTCTAGGCGACCGCAGGGCCACAGCAGCTCAGCCGCCGGTGCCCCCTCGGAAAC
CATGACCCCCGGCGCGGGCCCATGGAGCC**ATG**GCCTATAGGGTCCTGGGCCGCGCGGGGCCAC
CTCAGCCGCGGAGGGCGCGCAGGCTGCTCTTCGCCTTCACGCTCTCGCTCTCCTGCACTTACC
TGTGTTACAGCTTCCTGTGCTGCTGCGACGACCTGGGTCGGAGCCGCCTCCTCGGCGCGCCTC
GCTGCCTCCGCGGCCCCAGCGCGGGCGGCCAGAAACTTCTCCAGAAGTCCCGCCCCTGTGATC
CCTCCGGGCCGACGCCCAGCGAGCCCAGCGCTCCCAGCGCGCCCGCCGCCGCCGTGCCCGCCC
CTCGCCTCTCCGGTTCCAACCACTCCGGCTCACCCAAGCTGGGTACCAAGCGGTTGCCCCAAG
CCCTCATTGTGGGCGTGAAGAAGGGGGGCACCCGGGCCGTGCTGGAGTTTATCCGAGTACACC
CGGACGTGCGGGCCTTGGGCACGGAACCCCACTTCTTTGACAGGAACTACGGCCGCGGGCTGG
ATTGGTACAGGAGCCTGATGCCCAGGACCCTCGAGAGCCAGATCACGCTGGAGAAGACGCCCA
GCTACTTTGTCACTCAAGAGGCTCCTCGACGCATCTTCAACATGTCCCGAGACACCAAGCTGA
TCGTGGTTGTGCGGAACCCTGTGACCCGTGCCATCTCTGATTACACGCAGACACTCTCCAAGA
AGCCCGACATCCCGACCTTTGAGGGCCTCTCCTTCCGCAACCGCACCCTGGGCCTGGTGGACG
TGTCATGGAACGCCATCCGCATCGGCATGTACGTGCTGCACCTGGAGAGCTGGCTGCAGTACT
TCCCGCTAGCTCAGATTCACTTCGTCAGTGGCGAGCGACTCATCACTGACCCGGCCGGCGAGA
TGGGGCGAGTCCAGGACTTCCTGGGCATTAAGAGATTCATCACGGACAAGCACTTCTATTTCA
ACAAGACCAAAGGATTCCCTTGCTTGAAAAAAACAGAATCGAGCCTCCTGCCTCGATGCTTGG
GCAAATCAAAAGGGAGAACTCATGTACAGATTGATCCTGAAGTGATAGACCAGCTCCGAGAAT
TTTATAGACCGTATAATATCAAATTTTATGAAACCGTTGGGCAGGACTTCAGGTGGGAA**TAA**G
CCCACGAAAGGAAAGGGCTCTCAAGGGCTCTTCTGCTCATCTCTTCCGTGAGATTTGCTCCCA
GACCCTCTGATCTCCCTCCAACAAACCCTGGCTCCAGCCCCCTTTCCCAACTTGAGTTGCATC
ATCTTGGAACCAGGAAGCCCAGCTAAAGCCAAGAGACCAGAGAGTCCCTGCCACTAGTTTTCA
TCAGTCTGTTCAAGCAAAGTTGATCTGCTCCTGGCACGTCCAGTAAATTCCAGAATCATTCTC
CTTTCTGCCCATAAAGGGCCTTGGAGAATTGCTTTAAGAAGAGTGAATGTTCCAATGATGATA
GATATTATAAGCGATGATGGTTCTGTTGCTATGAACACAGCAGTCGGTCCCTGTCATTGTCCA
CCCAGGAGTGGCCTTGTTAATTCCAAGTGGCATGTATCTTCCCTCTGAGCTTCATTTCTTCAA
GATGCTCTGGGTGGTGGGATGGGAGACCATCCTCAGCCCTCCTCAGACCTTATCAATTCATTG
AGAGATTGCAAAGCTGAAAGCACCTCCGGCCACTCCTGGGAGACAGACCCTTTGGTGATGAAA
TAAACCAGTGACTTCAGAGCCTATGGTCTCAACTGTGCTTGAAAAACACTGTCTCTGAAAACA
ACTTTGTGATTCTCCCTGCTCCCTGTGGACAAAAGCACATAATTCTGCTGTTACGGGTACTTT
GCTCATACGAGCTTTCATGTTCAGCATGCAATGGAATCATGCTTGTCCATGTGAAATAAATAT
GGCTCTCTCGTGTCCTTAATGCTGGGCTTTTCTCTGTAAGCTGGTTCTGCAGCACAATTCATT
AATTAAACTTCTCCCAGTGCAAGAAGGCAGCTGGTGCTGGGGGTGGTCTGGGGGGTCAGGGAG
GAGGGCAAGGACTACATGGGGCAGAGGCAAGGCGGTGGTGGAGATGAGGAAAGAAGTTCTTCT
TGGCAGAAGCTGGGGCAGAAAGATCACATGAGATCTGTGGGGACACCCTCTATCTGAAACATA
AGTCTGTGTTCATTCTCTGCTTAGAAATTTTAGATCTGAAGTGCTACACTGAAGGTCCGAAGG
TTGATGGGGCATCAGATATCTTTTTGGTTGGCCAGCATGATATTTTGAAATAACTGTCAACAG
TTAGAAACTGGGAGCATTCATATGTAAAAAAATATGGATTTTCAGCTTCTTCTTAAAAAAAAAA
AAAAAAAAAAAAAAAAAAAAAAAAAAAAAAAAAAAAAAAAAAAAAAAAAAAAAAAAAAAAAAA
AAAAAAAAAA

# FIGURE 208

MAYRVLGRAGPPQPRRARRLLFAFTLSLSCTYLCYSFLCCCDDLGRSRLLGAPRCLRGPSAGG
QKLLQKSRPCDPSGPTPSEPSAPSAPAAAVPAPRLSGSNHSGSPKLGTKRLPQALIVGVKKGG
TRAVLEFIRVHPDVRALGTEPHFFDRNYGRGLDWYRSLMPRTLESQITLEKTPSYFVTQEAPR
RIFNMSRDTKLIVVVRNPVTRAISDYTQTLSKKPDIPTFEGLSFRNRTLGLVDVSWNAIRIGM
YVLHLESWLQYFPLAQIHFVSGERLITDPAGEMGRVQDFLGIKRFITDKHFYFNKTKGFPCLK
KTESSLLPRCLGKSKGRTHVQIDPEVIDQLREFYRPYNIKFYETVGQDFRWE

**Signal peptide:**
amino acids 1-33

**N-glycosylation sites.**
amino acids 102-106, 193-197, 235-239, 306-310

**Tyrosine kinase phosphorylation site.**
amino acids 296-305

**N-myristoylation sites.**
amino acids 51-57, 100-106, 121-127, 125-131

**Prokaryotic membrane lipoprotein lipid attachment site.**
amino acids 20-31

# FIGURE 209

CTTTCCTTATCTGTGTGTACTCTTATCTCACTGTTCTATTTTTTCTCCTCATTTATATTAACT
CTTTCTTACCTTTTTTTCTGAACTTCTAGGCCTTCTCTTTCCAGAACTGGTGGAAGACAAATG
AAACGGCCAAGATGGTAAGAAACAAGCCGCATTTCTCCTTGGGGAGACTGATAATTTAAAAGG
TTTGTTGTGTCAGAAACATTCCCAGCTTCATCACCAACCCTTTCCTTCCACCTCTGCCCACTG
GAGACCACTTACATCCCGAAGCGGACGCGGCAGCTGAAGTCAGGAAACCATGCATCACATTAG
CAGGAGCCAACTGCAGACTTTAAACTCCGTTCAACATGTGGATGCGGCAGAGAA**ATG**ACCTGT
CCAGACAAGCCGGGGCAGCTCATAAACTGGTTCATCTGCTCCCTGTGCGTCCCGCGGGTGCGT
AAGCTCTGGAGCAGCCGGCGTCCAAGGACCCGGAGAAACCTTCTGCTGGGCACTGCGTGTGCC
ATCTACTTGGGCTTCCTGGTGAGCCAGGTGGGGAGGGCCTCTCTCCAGCATGGACAGGCGGCT
GAGAAGGGGCCACATCGCAGCCGCGACACCGCCGAGCCATCCTTCCCTGAGATACCCCTGGAT
GGTACCCTGGCCCCTCCAGAGTCCCAGGGCAATGGGTCCACTCTGCAGCCCAATGTGGTGTAC
ATTACCCTACGCTCCAAGCGCAGCAAGCCGGCCAATATCCGTGGCACCGTGAAGCCCAAGCGC
AGGAAAAAGCATGCAGTGGCATCGGCTGCCCCAGGGCAGGAGGCTTTGGTCGGACCATCCCTT
CAGCCGCAGGAAGCGGCAAGGGAAGCTGATGCTGTAGCACCTGGGTACGCTCAGGGAGCAAAC
CTGGTTAAGATTGGAGAGCGACCCTGGAGGTTGGTGCGGGGTCCGGGAGTGCGAGCCGGGGGC
CCAGACTTCCTGCAGCCCAGCTCCAGGGAGAGCAACATTAGGATCTACAGCGAGAGCGCCCCC
TCCTGGCTGAGCAAAGATGACATCCGAAGAATGCGACTCTTGGCGGACAGCGCAGTGGCAGGG
CTCCGGCCTGTGTCCTCTAGGAGCGGAGCCCGTTTGCTGGTGCTGGAGGGGGGCGCACCTGGC
GCTGTGCTCCGCTGTGGCCCTAGCCCCTGTGGGCTTCTCAAGCAGCCCTTGGACATGAGTGAG
GTGTTTGCCTTCCACCTAGACAGGATCCTGGGGCTCAACAGGACCCTGCCGTCTGTGAGCAGG
AAAGCAGAGTTCATCCAAGATGGCCGCCCATGCCCCATCATTCTTTGGGATGCATCTTTATCT
TCAGCAAGTAATGACACCCATTCTTCTGTTAAGCTCACCTGGGGAACTTATCAGCAGTTGCTG
AAACAGAAATGCTGGCAGAATGGCCGAGTACCCAAGCCTGAATCAGGTTGTACTGAAATACAT
CATCATGAGTGGTCCAAGATGGCACTCTTTGATTTTTTGTTACAGATTTATAATCGCTTAGAT
ACAAATTGCTGTGGATTCAGACCTCGCAAGGAAGATGCCTGTGTACAGAATGGATTGAGGCCA
AAATGTGATGACCAAGGTTCTGCGGCTCTAGCACACATTATCCAGCGAAAGCATGACCCAAGG
CATTTGGTTTTTATAGACAACAAGGGTTTCTTTGACAGGAGTGAAGATAACTTAAACTTCAAA
TTGTTAGAAGGCATCAAAGAGTTTCCAGCTTCTGCAGTTTCTGTTTTGAAGAGCCAGCACTTA
CGGCAGAAACTTCTTCAGTCTCTGTTTCTTGATAAAGTGTATTGGGAAAGTCAAGGAGGTAGA
CAAGGAATTGAAAAGCTTATCGATGTAATAGAACACAGAGCCAAAATTCTTATCACCTATATC
AATGCACACGGGGTCAAAGTATTACCTATGAATGAA**TGA**CAAAAGAATCTTCTGGCTAGGGTG
TTAGATATATTTATGCATTTTTGGTTTTGTTTTTAAATCAAGCACATCAACCTCAAGCCCGTT
TAGCAATGAGGCAGTGTAGATGAATACGTAAAATAAATGACTTTAACCAAGTAGCTATAAAGG
GACTTAGCACTGTATGCATACTTAAAAAGGTTTTGAAAAACAAACTACTTGAGAAATATTTGT
TTATATTTTTCTCTAACATCATGCTATGTGTCAGTCTGAACATCTGACAACAGAAATTTCAGT
TATTATTCTAGCTAAGTTTTGAAAACATTTGTCATGCTGTTTAATAGAAAACTGCAAACCAGA
GATACTGACTCCATTAATAAACCATATTTTGTGCCGTTTTGACTGTTCTGACCAAATACTAAT
GGGAACAATTCTTGACGTTTTTCTGTTGCTGATTGTTAACATAGAGCAGTCTCTACACTACCC
TGAGGCAACTCTACATTGGAACACTGAGGCTTACAGCCTGCAAGAGCATCAGAGCTGACCATA
CATTTAAACAGAAATGCTGGTTTATTTGCAAAATCACCAGTATATTTTCTATTGTGTCTATAA
AAAATCAGTCATTTAAGTACAAGAATCATATTTTCCATTCCTTTTTAGAAATTTATTTTGTTG
TCCCTATGGAAATCATTCACATCTGACAATTTATATGTTAAAGAGTTTTACTCTCTCTATTTT
GGTCCAATTTGTATCTAGTGGCTGAGAAATTAAATAATTCTAAAGTATGAAGTTACCTATCTG
AAAATGTACTTACAGAGTATCATTTTAAAATGGATGTCTCTTTAAAAATTTTGTTACTTTTAC
CAACAATGTAATATAATTTATGTATATTTTATTAATAATAGTGAATTCCTTAAAATTTGTTCT
ATGTACTTATATTTAATTTGATTTAATGGTTACTGCCCAGATATTGAGAAATGGTTCAAATAT
TGAGTGTGTTTCAATAA

# FIGURE 210

MTCPDKPGQLINWFICSLCVPRVRKLWSSRRPRTRRNLLLGTACAIYLGFLVSQVGRASLQHG
QAAEKGPHRSRDTAEPSFPEIPLDGTLAPPESQGNGSTLQPNVVYITLRSKRSKPANIRGTVK
PKRRKKHAVASAAPGQEALVGPSLQPQEAAREADAVAPGYAQGANLVKIGERPWRLVRGPGVR
AGGPDFLQPSSRESNIRIYSESAPSWLSKDDIRRMRLLADSAVAGLRPVSSRSGARLLVLEGG
APGAVLRCGPSPCGLLKQPLDMSEVFAFHLDRILGLNRTLPSVSRKAEFIQDGRPCPIILWDA
SLSSASNDTHSSVKLTWGTYQQLLKQKCWQNGRVPKPESGCTEIHHHEWSKMALFDFLLQIYN
RLDTNCCGFRPRKEDACVQNGLRPKCDDQGSAALAHIIQRKHDPRHLVFIDNKGFFDRSEDNL
NFKLLEGIKEFPASAVSVLKSQHLRQKLLQSLFLDKVYWESQGGRQGIEKLIDVIEHRAKILI
TYINAHGVKVLPMNE

**Transmembrane domain:**

amino acids 40-56

**N-glycosylation sites.**

amino acids 98-102, 289-293, 322-326

**N-myristoylation sites.**

amino acids 8-14, 41-47, 97-103, 187-193, 251-257, 252-258, 287-293, 484-490

# FIGURE 211

GTGGGGTGGTGAGCGCAGCGCCGAGG**ATG**AGGAGGTGCAACAGCGGCTCCGGGCCGCCGCCGTCGCTGCTGCTGC
TGCTGCTGTGGCTGCTCGCGGTTCCCGGCGCTAACGCGGCCCCGCGGTCGGCGCTCTATTCGCCTTCCGACCCGC
TGACGCTGCTGCAGGCGGACACGGTGCGCGGCGCGGTGCTGGGCTCCCGCAGCGCCTGGGCCGTGGAGTTCTTCG
CCTCCTGGTGCGGCCACTGCATCGCCTTCGCCCCGACGTGGAAGGCGCTGGCCGAAGACGTCAAAGCCTGGAGGC
CGGCCCTGTATCTCGCCGCCCTGGACTGTGCTGAGGAGACCAACAGTGCAGTCTGCAGAGACTTCAACATCCCTG
GCTTCCCGACTGTGAGGTTCTTCAAGGCCTTTACCAAGAACGGCTCGGGAGCAGTATTTCCAGTGGCTGGTGCTG
ACGTGCAGACGCTGCGGGAGAGGCTCATTGACGCCCTGGAGTCCCATCATGACACGTGGCCCCCAGCCTGTCCCC
CACTGGAGCCTGCCAAGCTGGAGGAGATTGATGGATTCTTTGCGAGAAATAACGAAGAGTACCTGGCTCTGATCT
TTGAAAAGGGAGGCTCCTACCTGGGTAGAGAGGTGGCTCTGGACCTGTCCCAGCACAAAGGCGTGGCGGTGCGCA
GGGTGCTGAACACAGAGGCCAATGTGGTGAGAAAGTTTGGTGTCACCGACTTCCCCTCTTGCTACCTGCTGTTCC
GGAATGGCTCTGTCTCCCGAGTCCCCGTGCTCATGGAATCCAGGTCCTTCTATACCGCTTACCTGCAGAGACTCT
CTGGGCTCACCAGGGAGGCTGCCCAGACCACAGTTGCACCAACCACTGCTAACAAGATAGCTCCCACTGTTTGGA
AATTGGCAGATCGCTCCAAGATCTACATGGCTGACCTGGAATCTGCACTGCACTACATCCTGCGGATAGAAGTGG
GCAGGTTCCCGGTCCTGGAAGGGCAGCGCCTGGTGGCCCTGAAAAAGTTTGTGGCAGTGCTGGCCAAGTATTTCC
CTGGCCGGCCCTTAGTCCAGAACTTCCTGCACTCCGTGAATGAATGGCTCAAGAGGCAGAAGAGAAATAAAATTC
CCTACAGTTTCTTTAAAACTGCCCTGGACGACAGGAAAGAGGGTGCCGTTCTTGCCAAGAAGGTGAACTGGATTG
GCTGCCAGGGGAGTGAGCCGCATTTCCGGGGCTTTCCCTGCTCCCTGTGGGTCCTCTTCCACTTCTTGACTGTGC
AGGCAGCTCGGCAAAATGTAGACCACTCACAGGAAGCAGCCAAGGCCAAGGAGGTCCTCCCAGCCATCCGAGGCT
ACGTGCACTACTTCTTCGGCTGCCGAGACTGCGCTAGCCACTTCGAGCAGATGGCTGCTGCCTCCATGCACCGGG
TGGGGAGTCCCAACGCCGCTGTCCTCTGGCTCTGGTCTAGCCACAACAGGGTCAATGCTCGCCTTGCAGGTGCCC
CCAGCGAGGACCCCCAGTTCCCCAAGGTGCAGTGGCCACCCCGTGAACTTTGTTCTGCCTGCCACAATGAACGCC
TGGATGTGCCCGTGTGGGACGTGGAAGCCACCCTCAACTTCCTCAAGGCCCACTTCTCCCCAAGCAACATCATCC
TGGACTTCCCTGCAGCTGGGTCAGCTGCCCGGAGGGATGTGCAGAATGTGGCAGCCGCCCCAGAGCTGGCGATGG
GAGCCCTGGAGCTGGAAAGCCGGAATTCAACTCTGGACCCTGGGAAGCCTGAGATGATGAAGTCCCCCACAAACA
CCACCCCACATGTGCCGGCTGAGGGACCTGAGGCAAGTCGACCCCCGAAGCTGCACCCTGGCCTCAGAGCTGCAC
CAGGCCAGGAGCCTCCTGAGCACATGGCAGAGCTTCAGAGGAATGAGCAGGAGCAGCCGCTTGGGCAGTGGCACT
TGAGCAAGCGAGACACAGGGGCTGCATTGCTGGCTGAGTCCAGGGCTGAGAAGAACCGCCTCTGGGGCCCTTTGG
AGGTCAGGCGCGTGGGCCGCAGCTCCAAGCAGCTGGTCGACATCCCTGAGGGCCAGCTGGAGGCCCGAGCTGGAC
GGGGCCGAGGCCAGTGGCTGCAGGTGCTGGGAGGGGGCTTCTCTTACCTGGACATCAGCCTCTGTGTGGGGCTCT
ATTCCCTGTCCTTCATGGGCCTGCTGGCCATGTACACCTACTTCCAGGCCAAGATAAGGGCCCTGAAGGGCCATG
CTGGCCACCCTGCAGCC**TGA**ACCACCTGGGGAGGAGGCGGGAGAGGGAGCTGCCATCTCTAGGCACCTCAAGCCC
CCTGACCCCATTCCCTCCCCTCCCACCCCTTGCTCCTTGTCTGGCCTAGAAGTGTGGGAAATTCAGGAAAACGAG
TTGCTCCAGTGAAGCTTCTTGGGGTTGCTAGGACAGAGAGCTCCTTTGACACAAAAGACAGGAGCAGGGTCCAGG
TTCCCCTGCTGTGCAGGGAGGGCAGCCCCGGGCAGTGGGCATAGGGCAGCTCAGTCCCTGGCCTCTTAGCACCAC
ATTCCTGTTTTTCAGCTTATTTGAAGTCCTGCCTCATTCTCACTGGAGCCTCAGTCTCTCCTGCTTGGTCTTGGC
CCTCAACTGGGGCAAGTGAAGCCAGAGGAGGGTCCCCCAGCTGGGTGGGCTGGAATGGAACTCCTCACTAGCTGC
TGGGGCTCCGCCCACCCTGCTCCCTTCCGGACAATGAAGAAGCCTTTGCACCCTGGGAGGAAGGACCACCCCGGG
CCCTCTATGCCTGGCCAGCCTCCAGCTCCTCAGACCTCCTGGGTGGGGTTTGGCTTCAGGGTGGGGTTTGGAAGC
TTCTGGAAGTCGTGCTGGTCTCCCAGGTGAGGCAAGCCATGGTTGCTGGGCTGTAGGGTGAGTGGCTTGCTTGGT
GGGACCTGACGAGTTGGTGGCATGGGAAGGATGTGGGTCTCTAGTGCCTTGCCCTGGCTTAGCTGCAGGAGAAGA
TGGCTGCTTTCACTTCCCCCCATTGAGCTCTGCTCCCTCTGAGCCTGGTCTTTTGTCCTTTTTTATTTTGGTCTC
CAAGATGAATGCTCATCTTTGGAGGGTGCCAGGTAGAAGCTAGGGAGGGGAGTGTCTTCTCTCTCCAGGTTTCAC
CTTCCAGTGTGCAGAAGTTAGAAGGGTCTGGCGGGGGCAGTGCCTTACACATGCTTGATTCCCACGCTACCCCCT
GCCTTGGGAGGTGTGTGGAATAAATTATTTTTGTTAAGGCA

# FIGURE 212

MRRCNSGSGPPPSLLLLLLWLLAVPGANAAPRSALYSPSDPLTLLQADTVRGAVLGSRSAWAV
EFFASWCGHCIAFAPTWKALAEDVKAWRPALYLAALDCAEETNSAVCRDFNIPGFPTVRFFKA
FTKNGSGAVFPVAGADVQTLRERLIDALESHHDTWPPACPPLEPAKLEEIDGFFARNNEEYLA
LIFEKGGSYLGREVALDLSQHKGVAVRRVLNTEANVVRKFGVTDFPSCYLLFRNGSVSRVPVL
MESRSFYTAYLQRLSGLTREAAQTTVAPTTANKIAPTVWKLADRSKIYMADLESALHYILRIE
VGRFPVLEGQRLVALKKFVAVLAKYFPGRPLVQNFLHSVNEWLKRQKRNKIPYSFFKTALDDR
KEGAVLAKKVNWIGCQGSEPHFRGFPCSLWVLFHFLTVQAARQNVDHSQEAAKAKEVLPAIRG
YVHYFFGCRDCASHFEQMAAASMHRVGSPNAAVLWLWSSHNRVNARLAGAPSEDPQFPKVQWP
PRELCSACHNERLDVPVWDVEATLNFLKAHFSPSNIILDFPAAGSAARRDVQNVAAAPELAMG
ALELESRNSTLDPGKPEMMKSPTNTTPHVPAEGPEASRPPKLHPGLRAAPGQEPPEHMAELQR
NEQEQPLGQWHLSKRDTGAALLAESRAEKNRLWGPLEVRRVGRSSKQLVDIPEGQLEARAGRG
RGQWLQVLGGGFSYLDISLCVGLYSLSFMGLLAMYTYFQAKIRALKGHAGHPAA

**Signal peptide:**

amino acids 1-29

**Transmembrane domain:**

amino acids 705-728

**N-glycosylation sites.**

amino acids 130-134, 243-247, 575-579

**Glycosaminoglycan attachment site.**

amino acids 6-10

**cAMP- and cGMP-dependent protein kinase phosphorylation site.**

amino acids 644-648

**N-myristoylation sites.**

amino acids 52-58, 56-62, 196-202, 381-387, 392-398, 448-454, 468-474, 684-690, 702-708

**Cytochrome c family heme-binding site signature.**

amino acids 509-515

**Thioredoxin family proteins**

amino acids 62-78

# FIGURE 213

GCACGAGGCCGACTTCCAGACCATCTACAACTGCACGGCCTGGAACAGCTTCGGCTCCGACAC
TGAGATCATCCGGCTCAAGGAGCAAGGTTCGGAAATGAAGTCGGGAGCCGGGCTGGAAGCAGA
GTCTGTGCCG**ATG**GCCGTCATCATTGGGGTGGCCGTAGGAGCTGGTGTGGCCTTCCTCGTCCT
TATGGCAACCATCGTGGCGTTCTGCTGTGCCCGTTCCCAGAGAAATCTCAAAGGTGTTGTGTC
AGCCAAAAATGATATCCGAGTGGAAATTGTCCACAAGGAACCAGCCTCTGGTCGGGAGGGTGA
GGAGCACTCCACCATCAAGCAGCTGATGATGGACCGGGGTGAATTCCAGCAAGACTCAGTCCT
GAAACAGCTGGAGGTCCTCAAAGAAGAGGAGAAAGAGTTTCAGAACCTGAAGGACCCCACCAA
TGGCTACTACAGCGTCAACACCTTCAAAGAGCACCACTCAACCCCGACCATCTCCCTCTCCAG
CTGCCAGCCCGACCTGCGTCCTGCGGGTAAGCAGCGTGTGCCCACAGGCATGTCCTTCACCAA
CATCTACAGCACCCTGAGCGGCCAGGGCCGCCTCTACGACTACGGGCAGCGGTTTGTGCTGGG
CATGGGCAGCTCGTCCATCGAGCTTTGTGAGCGGGAGTTCCAGAGAGGCTCCCTCAGCGACAG
CAGCTCCTTCCTGGACACGCAGTGTGACAGCAGCGTCAGCAGCAGCGGCAAGCAGGATGGCTA
TGTGCAGTTCGACAAGGCCAGCAAGGCTTCTGCTTCCTCCTCCCACCACTCCCAGTCCTCGTC
CCAGAACTCTGACCCCAGTCGACCCCTGCAGCGGCGGATGCAGACTCACGTC**TAA**GGATCACA
CACCGCGGGTGGGGACGGGCCAGGGAAGAGGTCAGGGCACGTTCTGGTTGTCCAGGGACGAGG
GGTACTTTGCAGAGGACACCAGAATTGGCCACTTCCAGGACAGCCTCCCAGCGCCTCTGCCAC
TGCCTTCCTTCGAAGCTCTGATCAAGCACAAATCTGGGTCCCCAGGTGCTGTGTGCCAGAGGT
GGGCGGGTGGGGAGACAGACAGAGGCTGCGGCTGAGTGCGCTGTGCTTAGTGCTGGACACCCG
TGTCCCCGGCCCTTTCCTGGAGGCCCCTCTACCACCTGCTCTGCCCACAGGCACAAGTGGCAG
CTATAACTCTGCTTTCATGAAACTGCGGTCCACTCTCTGGTCTCTCTGTGGGCTCTACCCCTC
ACTGACCACAAGCTCTACCTACCCCTGTGCCTGTGCTCCCATACAGCCCTGGGGAGAAGGGGA
TGACGTCTTCCCAGCACTGAGCTGCCCCAGAAACCCCGGCTCCCCACTGCTGCTCATAGCCCA
TACCCTGGAGGCTGACAAGCCAGAAATGGCCTTGGCTAAAGGAGCCTCTCTCTCACCAGGCTG
GCCGGGAGCCCACCCCCAATTTGTTTGGTGTTTTGTGTCCATACTCTTGCAGTTCTGTCCTTG
GACTTGATGCCGCTGAACTCTGCGGTGGGACCGGTCCCGTCAGAGCCTGGTGTACTGGGGGGA
GGGAGGGAGGAGGGAGCCTGTGCTGACGGAGCACCTCGCCGGGTGTGCCCCTCCTGGGCTGTG
TGACCCCAGCCTCCCCACCCACCTCCTGCTTTGTGTACTCCTCCCCTCCCCCTCAGCACAATC
GGAGTTCATATAAGAAGTGCGGGAGCTTCTCTGGTCAGGGTTCTCTGAACACTTATGGAGAGA
GTGCTTCCTGGGAAGTGTGGCGTTTGAAGGGGCTGGAGGGCAGGTCTTTAAGATGGCGAGACT
GCCCTTCTCAGCTGATAAACACAAGAACGGCGATCCTGTCTTCAGTAAGGCTCCACGAGAAGA
GAGGAAGTATATCTACACCTCAACCCTCCTAGTCACCACCTGAAATAAATGTTAGGGAAAAAAAA

# FIGURE 214

MAVIIGVAVGAGVAFLVLMATIVAFCCARSQRNLKGVVSAKNDIRVEIVHKEPASGREGEEHS
TIKQLMMDRGEFQQDSVLKQLEVLKEEEKEFQNLKDPTNGYYSVNTFKEHHSTPTISLSSCQP
DLRPAGKQRVPTGMSFTNIYSTLSGQGRLYDYGQRFVLGMGSSSIELCEREFQRGSLSDSSSF
LDTQCDSSVSSSGKQDGYVQFDKASKASASSSHHSQSSSQNSDPSRPLQRRMQTHV

**Signal peptide:**
amino acids 1-28

**Glycosaminoglycan attachment site.**
amino acids 150-154

**N-myristoylation sites.**
amino acids 6-12, 10-16, 36-42, 139-145, 165-171

**Prokaryotic membrane lipoprotein lipid attachment site.**
amino acids 114-125

# FIGURE 215

CAGCCTTCCTCCCCCAGCCTGAGTGACTACTCTATTCCTTGGTCCCTGCTATTGTCGGGGACG
ATTGC**<u>ATG</u>**GGCTACGCCAGGAAAGTAGGCTGGGTGACCGCAGGCCTGGTGATTGGGGCTGGCG
CCTGCTATTGCATTTATAGACTGACTAGGGGAAGAAAACAGAACAAGGAAAAAATGGCTGAGG
GTGGATCTGGGGATGTGGATGATGCTGGGGACTGTTCTGGGGCCAGGTATAATGACTGGTCTG
ATGATGATGATGACAGCAATGAGAGCAAGAGTATAGTATGGTACCCACCTTGGGCTCGGATTG
GGACTGAAGCTGGAACCAGAGCTAGGGCCAGGGCAAGGGCCAGGGCTACCCGGGCACGTCGGG
CTGTCCAGAAACGGGCTTCCCCCAATTCAGATGATACCGTTTTGTCCCCTCAAGAGCTACAAA
AGGTTCTTTGCTTGGTTGAGATGTCTGAAAAGCCTTATATTCTTGAAGCAGCTTTAATTGCTC
TGGGTAACAATGCTGCTTATGCATTTAACAGAGATATTATTCGTGATCTGGGTGGTCTCCCAA
TTGTCGCAAAGATTCTCAATACTCGGGATCCCATAGTTAAGGAAAAGGCTTTAATTGTCCTGA
ATAACTTGAGTGTGAATGCTGAAAATCAGCGCAGGCTTAAAGTATACATGAATCAAGTGTGTG
ATGACACAATCACTTCTCGCTTGAACTCATCTGTGCAGCTTGCTGGACTGAGATTGCTTACAA
ATATGACTGTTACTAATGAGTATCAGCACATGCTTGCTAATTCCATTTCTGACTTTTTTCGTT
TATTTTCAGCGGGAAATGAAGAAACCAAACTTCAGGTTCTGAAACTCCTTTTGAATTTGGCTG
AAAATCCAGCCATGACTAGGGAACTGCTCAGGGCCCAAGTACCATCTTCACTGGGCTCCCTCT
TTAATAAGAAGGAGAACAAAGAAGTTATTCTTAAACTTCTGGTCATATTTGAGAACATAAATG
ATAATTTCAAATGGGAAGAAAATGAACCTACTCAGAATCAATTCGGTGAAGGTTCACTTTTTT
TCTTTTTAAAAGAATTTCAAGTGTGTGCTGATAAGGTTCTGGGAATAGAAAGTCACCATGATT
TTTTGGTGAAAGTAAAAGTTGGAAAATTCATGGCCAAACTTGCTGAACATATGTTCCCAAAGA
GCCAGGAA**<u>TAA</u>**CACCTTGATTTTGTAATTTAGAAGCAACACACATTGTAAACTATTCATTTTC
TCCACCTTGTTTATATGGTAAAGGAATCCTTTCAGCTGCCAGTTTTGAATAATGAATATCATA
TTGTATCATCAATGCTGATATTTAACTGAGTTGGTCTTTAGGTTTAAGATGGATAAATGAATA
TCACTACTTGTTCTGAAAACATGTTTGTTGCTTTTTATCTCGCTGCCTAGATTGAAATATTTT
GCTATTTCTTCTGCATAAGTGACAGTGAACCAATTCATCATGAGTAAGCTCCCTTCTGTCATT
TTCATTGATTTAATTTGTGTATCATCAATAAAATTGTATGTTAATGCTGGAAAGA

# FIGURE 216

MGYARKVGWVTAGLVIGAGACYCIYRLTRGRKQNKEKMAEGGSGDVDDAGDCSGARYNDWSDD
DDDSNESKSIVWYPPWARIGTEAGTRARARARARATRARRAVQKRASPNSDDTVLSPQELQKV
LCLVEMSEKPYILEAALIALGNNAAYAFNRDIIRDLGGLPIVAKILNTRDPIVKEKALIVLNN
LSVNAENQRRLKVYMNQVCDDTITSRLNSSVQLAGLRLLTNMTVTNEYQHMLANSISDFFRLF
SAGNEETKLQVLKLLLNLAENPAMTRELLRAQVPSSLGSLFNKKENKEVILKLLVIFENINDN
FKWEENEPTQNQFGEGSLFFFLKEFQVCADKVLGIESHHDFLVKVKVGKFMAKLAEHMFPKSQE

**Signal peptide:**
amino acids 1-20

**N-glycosylation sites.**
amino acids 68-72, 189-193, 217-221, 230-234

**cAMP- and cGMP-dependent protein kinase phosphorylation site.**
amino acids 107-111

**N-myristoylation sites.**
amino acids 13-19, 17-23, 19-25, 54-60, 83-89, 147-153, 255-261, 290-296

**Amidation site.**
amino acids 29-33

# FIGURE 217

GAGACACAAAGGCAGGCGGGATGCGGGAGCAGGCAAAGGGAAAGCGAAAGCCGCGCGCCCGGC
CGGTGACTGGGTGAAGGCGCCGCGCAGCTTTCCCGACGCCGGCTGTACCCGGACCTCCTGGTC
GAGCCTGGCGCGCCGCAGCC**ATG**GCCATCGCTCAACTGGCCACGGAGTACGTGTTCTCGGATT
TCTTGCTGAAGGAGCCCACGGAGCCCAAGTTCAAGGGGCTGCGACTGGAGCTGGCTGTGGACA
AGATGGTCACGTGCATTGCGGTGGGGCTGCCCCTGCTGCTCATCTCGCTGGCCTTCGCGCAGG
AGATCTCGATTGGTACACAGATAAGCTGTTTCTCTCCAAGTTCTTTCTCCTGGCGTCAGGCTG
CCTTTGTGGATTCATATTGCTGGGCGGCTGTTCAGCAGAAGAACTCACTGCAGAGCGAGTCTG
GAAACCTCCCACTGTGGCTGCATAAGTTTTTCCCCTACATCCTGCTGCTCTTTGCGATCCTCC
TGTACCTGCCCCCGCTGTTCTGGCGTTTCGCAGCTGCTCCTCATATTTGCTCAGACTTGAAGT
TTATCATGGAAGAACTTGACAAAGTTTACAACCGTGCAATTAAGGCTGCAAAGAGTGCGCGTG
ACCTTGACATGAGAGATGGAGCCTGCTCAGTTCCAGGTGTTACCGAGAACTTAGGGCAAAGTT
TGTGGGAGGTATCTGAAAGCCACTTCAAGTACCCAATTGTGGAGCAGTACTTGAAGACAAAGA
AAAATTCTAATAATTTAATCATCAAGTACATTAGCTGCCGCCTGCTGACACTCATCATTATAC
TGTTAGCGTGTATCTACCTGGGCTATTACTTCAGCCTCTCCTCACTCTCAGACGAGTTTGTGT
GCAGCATCAAATCAGGGATCCTGAGAAACGACAGCACCGTGCCCGATCAGTTTCAGTGCAAAC
TCATTGCCGTGGGCATCTTCCAGTTGCTCAGTGTCATTAACCTTGTGGTTTATGTCCTGCTGG
CTCCCGTGGTTGTCTACACGCTGTTTGTTCCATTCCGACAGAAGACAGATGTTCTCAAAGTGT
ACGAAATCCTCCCCACTTTTGATGTTCTGCATTTCAAATCTGAAGGGTACAACGATTTGAGCC
TCTACAATCTCTTCTTGGAGGAAAATATAAGTGAGGTCAAGTCATACAAGTGTCTTAAGGTAC
TGGAGAATATTAAGAGCAGTGGTCAGGGGATCGACCCAATGCTACTCCTGACAAACCTTGGCA
TGATCAAGATGGATGTTGTTGATGGCAAAACTCCCATGTCTGCAGAGATGAGAGAGGAGCAGG
GGAACCAGACGGCAGAGCTCCAAGGTATGAACATAGACAGTGAAACTAAAGCAAATAATGGAG
AGAAGAATGCCCGACAGAGACTTCTGGATTCTTCTTGC**TGA**TGATTTTTTTTCCTTGAGCTGT
AAATCTGTGACTTCTGCGACATGGGATTTAATTTGGCTAAAGCACCCCTGTTGGTTTCACAGC
TGGTTTGCAATAAATGGTTCTTGGTGGAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAA

# FIGURE 218

MAIAQLATEYVFSDFLLKEPTEPKFKGLRLELAVDKMVTCIAVGLPLLLISLAFAQEISIGTQ
ISCFSPSSFSWRQAAFVDSYCWAAVQQKNSLQSESGNLPLWLHKFFPYILLLFAILLYLPPLF
WRFAAAPHICSDLKFIMEELDKVYNRAIKAAKSARDLDMRDGACSVPGVTENLGQSLWEVSES
HFKYPIVEQYLKTKKNSNNLIIKYISCRLLTLIIIILLACIYLGYYFSLSSLSDEFVCSIKSGI
LRNDSTVPDQFQCKLIAVGIFQLLSVINLVVYVLLAPVVVYTLFVPFRQKTDVLKVYEILPTF
DVLHFKSEGYNDLSLYNLFLEENISEVKSYKCLKVLENIKSSGQGIDPMLLLTNLGMIKMDVV
DGKTPMSAEMREEQGNQTAELQGMNIDSETKANNGEKNARQRLLDSSC

**Transmembrane domains:**
amino acids 37-55, 108-126, 216-232, 273-290

**N-glycosylation sites.**
amino acids 255-259, 338-342, 394-398

**Glycosaminoglycan attachment site.**
amino acids 357-361

**cAMP- and cGMP-dependent protein kinase phosphorylation site.**
amino acids 203-207

**N-myristoylation sites.**
amino acids 61-67, 174-180, 251-257, 393-399

**Prokaryotic membrane lipoprotein lipid attachment site.**
amino acids 218-229

# FIGURE 219

CTGTGAGTGACACACGCTGAGTGGGGTGAAGGGAA**ATG**CTGGTGAATTTCATTTTGAGGTGTG
GGTTGCTGTTAGTCACTCTGTCTCTTGCCATTGCCAAGCACAAGCAATCTTCCTTCACCAAAA
GTTGTTACCCAAGGGGAACATTGTCCCAAGCTGTTGACGCTCTCTATATCAAAGCAGCATGGC
TCAAAGCAACGATTCCAGAAGACCGCATAAAAAATATACGATTATTAAAAAAGAAAACAAAAA
AGCAGTTTATGAAAAACTGTCAATTTCAAGAACAGCTTCTGTCCTTCTTCATGGAAGACGTTT
TTGGTCAACTGCAATTGCAAGGCTGCAAGAAAATACGCTTTGTGGAGGACTTTCATAGCCTTA
GGCAGAAATTGAGCCACTGTATTTCCTGTGCTTCATCAGCTAGAGAGATGAAATCCATTACCA
GGATGAAAAGAATATTTTATAGGATTGGAAACAAAGGAATCTACAAAGCCATCAGTGAACTGG
ATATTCTTCTTTCCTGGATTAAAAAATTATTGGAAAGCAGTCAG**TAA**ACCAAAGCCAAGTACA
TTGATTTTACAGTTATTTTGAAATACAATAAGAACTGCTAGAAATATGTTTATAACAGTCTAT
TTCTTTTAAAAACTTTTTAACATAATACTGACGGCATGTTAGGTGATTCAGAATAGACAAGAA
GGATTTAGTAAATTAACGTTTTGGATATAAGTTGTCACTAATTTGCACATTTTCTGTGTTTTC
AAATAATGTTTCCATTCTGAACATGTTTTGTCATTCACAAGTACATTGTGTCAACTTAATTTA
AAGTATGTAACCTGAATTAACTCGTGTAATATTTGTGTGTGGAGTGGGATGTGGGGGGTGGAG
GGGGAATGACAGATTTCTGGAATGCAATGTAATGTTACTGAGACTTAAATAGATGTTATGTAT
ATGATTGTCTGTTTAAGTGTTTGAAAATTGTTAATTATGCCCAGTGTGAACTTAGTACTTAAC
ACATTTTGATTTTAATTAAATAAATTGGGTTTCCTTCTCAAAAAAAAAAAAAAAAAAAAAAAA
AAAAA

# FIGURE 220

MLVNFILRCGLLLVTLSLAIAKHKQSSFTKSCYPRGTLSQAVDALYIKAAWLKATIPEDRIKN
IRLLKKKTKKQFMKNCQFQEQLLSFFMEDVFGQLQLQGCKKIRFVEDFHSLRQKLSHCISCAS
SAREMKSITRMKRIFYRIGNKGIYKAISELDILLSWIKKLLESSQ

**Signal sequence:**
amino acids 1-21

**cAMP- and cGMP-dependent protein kinase phosphorylation site.**
amino acids 68-71

**N-myristoylation site.**
amino acids 148-153

**Interleukin-10 proteins.**
amino acids 58-94, 74-102, 128-170

# FIGURE 221

GACCACGGCCCTGCGCCCCAGCCAGGCCTGAGGAC**<u>ATG</u>**AGGCGGCCGGCGGCGGTGCCGCTCC
TGCTGCTGCTGTGTTTTGGGTCTCAGAGGGCCAAGGCAGCAACAGCCTGTGGTCGCCCCAGGA
TGCTGAACCGAATGGTGGGCGGGCAGGACACGCAGGAGGGCGAGTGGCCCTGGCAAGTCAGCA
TCCAGCGCAACGGAAGCCACTTCTGCGGGGGCAGCCTCATCGCGGAGCAGTGGGTCCTGACGG
CTGCGCACTGCTTCCGCAACACCTCTGAGACGTCCCTGTACCAGGTCCTGCTGGGGGCAAGGC
AGCTAGTGCAGCCGGGACCACACGCTATGTATGCCCGGGTGAGGCAGGTGGAGAGCAACCCCC
TGTACCAGGGCACGGCCTCCAGCGCTGACGTGGCCCTGGTGGAGCTGGAGGCACCAGTGCCCT
TCACCAATTACATCCTCCCCGTGTGCCTGCCTGACCCCTCGGTGATCTTTGAGACGGGCATGA
ACTGCTGGGTCACTGGCTGGGGCAGCCCCAGTGAGGAAGACCTCCTGCCCGAACCGCGGATCC
TGCAGAAACTCGCTGTGCCCATCATCGACACACCCAAGTGCAACCTGCTCTACAGCAAAGACA
CCGAGTTTGGCTACCAACCCAAAACCATCAAGAATGACATGCTGTGCGCCGGCTTCGAGGAGG
GCAAGAAGGATGCCTGCAAGGGCGACTCGGGCGGCCCCCTGGTGTGCCTCGTGGGTCAGTCGT
GGCTGCAGGCGGGGGTGATCAGCTGGGGTGAGGGCTGTGCCCGCCAGAACCGCCCAGGTGTCT
ACATCCGTGTCACCGCCCACCACAACTGGATCCATCGGATCATCCCCAAACTGCAGTTCCAGC
CAGCGAGGTTGGGCGGCCAGAAG**<u>TGA</u>**GACCCCCGGGGCCAGGAGCCCCTTGAGCAGAGCTCTG
CACCCAGCCTGCCCGCCCACACCATCCTGCTGGTCCTCCCAGCGCTGCTGTTGCACCTGTGAG
CCCCACCAGACTCATTTGTAAATAGCGCTCCTTCCTCCCCTCTCAAATACCCTTATTTTATTT
ATGTTTCTCCCAATAAAAACCCAGCCTGTGTGCCAGCTGAAAAAAAAAAAAAAAAAA

# FIGURE 222

MRRPAAVPLLLLLLCFGSQRAKAATACGRPRMLNRMVGGQDTQEGEWPWQVSIQRNGSHFCGGS
LIAEQWVLTAAHCFRNTSETSLYQVLLGARQLVQPGPHAMYARVRQVESNPLYQGTASSADVA
LVELEAPVPFTNYILPVCLPDPSVIFETGMNCWVTGWGSPSEEDLLPEPRILQKLAVPIIDTP
KCNLLYSKDTEFGYQPKTIKNDMLCAGFEEGKKDACKGDSGGPLVCLVGQSWLQAGVISWGEG
CARQNRPGVYIRVTAHHNWIHRIIPKLQFQPARLGGQK

**Important features of the protein:**

**Signal peptide:**

amino acids 1-22

**N-glycosylation sites.**

amino acids 55-58, 79-82

**Casein kinase II phosphorylation sites.**

amino acids 121-124, 165-168, 167-170, 248-251

**Tyrosine kinase phosphorylation sites.**

amino acids 78-86, 197-203

**N-myristoylation sites.**

amino acids 16-21, 37-42, 56-61, 62-67, 118-123

**Amidation site.**

amino acids 219-222

**Serine proteases, trypsin family, histidine active site.**

amino acids 71-76

# FIGURE 223

CAAG**ATG**TGGACAGCTCTTGTGCTCATTTGGATTTTCTCCTTGTCCTTATCTGAAAGCCATGC
GGCATCCAACGATCCACGCAACTTTGTCCCTAACAAAATGTGGAAGGGATTAGTCAAGAGGAA
TGCATCTGTGGAAACAGTTGATAATAAAACGTCTGAGGATGTAACCATGGCAGCAGCTTCTCC
TGTCACATTGACCAAAGGGACTTCGGCAGCCCACCTCAACTCTATGGAAGTCACAACAGAGGA
CACAAGCAGGACAGATGTGAGTGAACCAGCAACTTCAGGAGTTGCAGCTGATGGTGTGACCTC
CATTGCTCCCACGGCTGTGGCCTCCAGTACGACTGCGGCCTCCATTACGACTGCGGCCTCCAG
TATGACTGTGGCCTCCAGTGCTCCCACGACTGCAGCCTCCAGTACAACTGTGGCCTCCATTGC
TCCCACGACTGCAGCCTCCAGTATGACTGCGGCCTCCAGCACTCCCATGACACTTGCACTCCC
CGCGCCCACGTCCACTTCCACAGGGCGGACCCCGTCCACTACCGCCACTGGGCATCCATCTCT
CAGCACAGCCCTCGCACAAGTGCCAAAGAGCAGCGCGTTGCCAAGAACAGCAACCCTGGCCAC
ATTGGCCACACGTGCTCAGACTGTAGCGACCACAGCAAACACAAGCAGCCCCATGAGCACTCG
TCCAAGTCCTTCCAAGCACATGCCCAGTGACACCGCGGCAAGCCCTGTACCCCCTATGCGTCC
CCAAGCACAAGGTCCCATTAGCCAGGTGTCAGTGGACCAGCCTGTGGTTAACACAACAAATAA
ATCCACACCCATGCCCTCAAACACAACCCCAGAGCCCGCCCCCACCCCCACAGTGGTGACCAC
CACCAAGGCACAAGCCAGGGAGCCAACTGCCAGCCCAGTGCCAGTACCTCACACCAGCCCAAT
CCCTGAGATGGAGGCCATGTCCCCCACGACACAGCCAAGCCCCATGCCATATACCCAGAGGGC
CGCTGGGCCAGGCACATCCCAGGCACCGGAGCAGGTAGAGACTGAAGCCACACCAGGTACTGA
TTCCACTGGGCCAACACCCAGGAGCTCAGGGGGCACTAAGATGCCAGCCACGGACTCGTGCCA
GCCCAGCACCCAAGGCCAGTACATGGTGGTCACCACTGAGCCCCTCACCCAGGCCGTGGTAGA
CAAAACTCTCCTTCTGGTGGTGCTGTTACTCGGGGTGACCCTTTTCATCACAGTCTTGGTTTT
GTTTGCCCTGCAGGCCTATGAGAGCTACAAGAAGAAGGACTACACCCAGGTGGACTACTTAAT
CAACGGGATGTATGCGGACTCAGAAATG**TGA**GGGGGGCGGGGGCCTGGCGGGAGGCCTGGCCC
CTTCCTCGTCCTTTCCTTTTGCCTTTGAGACCAAACCAAGTGCTTCCAAATTCTTTTGGTGCA
ATTGAGGAGATATGCCAGATGCTTAAACACATTTAATTGCTGTCAGATTAATTCCATGATCAC
TAAAGAGTTGCTGCTTTTTTCATATTTATTTTTGTAAATGATTCTGTGCCCAGGAGCAGCTGG
GGGTTCCACCTCAGGGTGGGGCGGGCAGGACCCCGTCTCCCCAGGTGTCGGAGCCTGACCTGA
ATTAAAGTACTGACTGCTCGCCA

# FIGURE 224

MWTALVLIWIFSLSLSESHAASNDPRNFVPNKMWKGLVKRNASVETVDNKTSEDVTMAAASPV
TLTKGTSAAHLNSMEVTTEDTSRTDVSEPATSGVAADGVTSIAPTAVASSTTAASITTAASSM
TVASSAPTTAASSTTVASIAPTTAASSMTAASSTPMTLALPAPTSTSTGRTPSTTATGHPSLS
TALAQVPKSSALPRTATLATLATRAQTVATTANTSSPMSTRPSPSKHMPSDTAASPVPPMRPQ
AQGPISQVSVDQPVVNTTNKSTPMPSNTTPEPAPTPTVVTTTKAQAREPTASPVPVPHTSPIP
EMEAMSPTTQPSPMPYTQRAAGPGTSQAPEQVETEATPGTDSTGPTPRSSGGTKMPATDSCQP
STQGQYMVVTTEPLTQAVVDKTLLLVVLLLGVTLFITVLVLFALQAYESYKKKDYTQVDYLIN
GMYADSEM

**Signal peptide:**
amino acids 1-20

**Transmembrane domain:**
amino acids 396-420

**N-glycosylation sites.**
amino acids 41-44, 49-52, 222-225, 268-271, 271-274

**Casein kinase II phosphorylation sites.**
amino acids 14-17, 51-54, 80-83, 85-88, 280-283, 434-437

**N-myristoylation sites.**
amino acids 68-73, 354-359

**Aldo/keto reductase family putative active site signature.**
amino acids 195-210

# FIGURE 225

GGAAGGCGCTCAAGGTGCGCGGCCCGGGGCGCGCTACTGGGGGCGCCCTCCGCGGTGGGCAGC
GCGCCAGGGATCGGCCTGGGCAGCCGCGGGGCGCGCGAAGGCTGCGCTTTCCCTACGGCCCCC
CTCGCTTCCTCCGGCACGGCGGCAACGGAGATTTCCTCTCGGGGAAACTACGCGGATCCTTTT
CGGGGATCCTCGCCCCGCCCCAGTTCTCCGCCCCCTCCCCTTTGCTGGGGCGCCTGGGCTGGC
CCGCGCAGGGGAGGAGGCTCTGGCAGCCTGGGCAGGGAGGCGGCGGGGGGCCGCGGAGCCGCT
GGCCATCGATTCTCCCCGCCATGTGACGCCGTCCTTAGCCCTGCGACCCCCAGCGCGTCCCGG
GCCTGCGCCTCCGCCCCGCCGCGCAGCGCACG**ATG**CTTCTGCCGGGACGCGCACGCCAACCGC
CGACGCCCCAGCCCGTGCAGCATCCCGGCCTCCGCCGGCAGGTAGAGCCGCCGGGGCAGCTCC
TGCGCCTCTTCTACTGCACTGTCCTGGTCTGCTCCAAAGAGATCTCAGCGCTCACCGACTTCT
CTGGTTACCTAACCAAACTCCTGCAAAACCACACCACCTATGCCTGTGATGGGGACTATTTGA
ATCTACAGTGCCCTCGGCATTCTACGATAAGTGTCCAATCGGCATTTTATGGGCAAGATTACC
AAATGTGTAGTTCCCAGAAGCCTGCCTCCCAGAGGGAAGACAGCTTAACCTGTGTGGCAGCCA
CCACCTTCCAGAAGGTGCTGGACGAATGCCAGAACCAGCGGGCCTGCCACCTCCTGGTCAATA
GCCGTGTTTTTGGACCTGACCTTTGTCCAGGAAGCAGTAAATACCTCCTGGTCTCCTTTAAAT
GCCAACCTAATGAATTAAAAAACAAAACCGTGTGTGAAGACCAGGAGCTGAAACTGCACTGCC
ATGAATCCAAGTTCCTCAACATCTACTCTGCGACCTACGGCAGGAGGACCCAGGAAAGGGACA
TCTGCTCCTCCAAGGCAGAGCGGCTCCCCCCTTTCGATTGCTTGTCTTACTCAGCTTTGCAAG
TCCTATCCCGAAGGTGCTATGGGAAGCAGAGATGCAAAATCATCGTCAACAATCACCATTTTG
GAAGCCCCTGTTTGCCAGGCGTGAAAAAATACCTCACTGTGACCTACGCATGTGTTCCCAAGA
ACATACTCACAGCGATTGATCCAGCCATTGCTAATCTAAAACCTTCTTTGAAGCAGAAAGATG
GTGAATATGGTATAAACTTCGACCCAAGCGGATCGAAGGTTCTGAGGAAAGATGGAATTCTTG
TTAGCAACTCTCTGGCAGCCTTTGCTTACATTAGAGCCCACCCAGAGAGAGCTGCCCTGCTGT
TCGTGTCCAGTGTCTGCATCGGCCTGGCCCTCACACTGTGCGCCCTGGTCATCAGAGAGTCCT
GTGCCAAGGACTTCCGCGACTTGCAGCTGGGGAGGGAGCAGCTGGTGCCAGGAAGTGACAAGG
TCGAGGAGGACAGCGAGGATGAAGAAGAGGAGGAGGACCCCTCTGAGTCTGATTTCCCAGGGG
AACTGTCGGGGTTCTGTAGGACTTCATATCCTATATACAGTTCCATAGAAGCTGCAGAGCTCG
CAGAAAGGATTGAGCGCAGGGAGCAAATCATTCAGGAAATATGGATGAACAGTGGTTTGGACA
CCTCGCTCCCAAGAAACATGGGCCAGTTCTAC**TGA**AAACCACATGCATCTTGATGCGATCGCA
CTTTCTGAAGAAGGAAGGATCCCAAATGCCCCTCCAGTTCTGGTTCACCTGTACCTTCTATGA
AGGAGAATTCGTCATGTCATTCAACACTCGTGAGGCCAGGAAGCTATTAAAGGGATGTTTCAA
GCTGTTTCTAGCACATTCCAAAATAAATGAGGAGGGAGGAAAAAAAAAAAAAAAAAAAAAAAA
AAAAAAAAAAAAAAAAA

# FIGURE 226

MLLPGRARQPPTPQPVQHPGLRRQVEPPGQLLRLFYCTVLVCSKEISALTDFSGYLTKLLQNH
TTYACDGDYLNLQCPRHSTISVQSAFYGQDYQMCSSQKPASQREDSLTCVAATTFQKVLDECQ
NQRACHLLVNSRVFGPDLCPGSSKYLLVSFKCQPNELKNKTVCEDQELKLHCHESKFLNIYSA
TYGRRTQERDICSSKAERLPPFDCLSYSALQVLSRRCYGKQRCKIIVNNHHFGSPCLPGVKKY
LTVTYACVPKNILTAIDPAIANLKPSLKQKDGEYGINFDPSGSKVLRKDGILVSNSLAAFAYI
RAHPERAALLFVSSVCIGLALTLCALVIRESCAKDFRDLQLGREQLVPGSDKVEEDSEDEEEE
EDPSESDFPGELSGFCRTSYPIYSSIEAAELAERIERREQIIQEIWMNSGLDTSLPRNMGQFY

**Transmembrane domains:**
amino acids 32-49, 322-343

**N-glycosylation sites.**
amino acids 62-66, 165-169

**Tyrosine kinase phosphorylation site.**
amino acids 280-287

**N-myristoylation site.**
amino acids 302-308, 333-339, 428-434

**Amidation site.**
amino acids 191-195

# FIGURE 227

GGCACGAGGTGGAAGGGCTTTTACAAACAGATTGCTGGCCCCACCCCCCAGAATTTCTCATCA
GGAGTGGGCAAGACCAATCATTTGCATTTCTGACAAGTTCCCAGGAGCTGCAGCTGCTGGCCC
TGGAACCACACTTTGAGAACCACTGCTTTAGACCAAACACCAAAGGAAGATGCAGCCACCCTC
CTTTACATGTCACAACGCTCAGGGTCCATGAGTACCTCAGGCTGTCCAGCTGAGCTCCACCTG
CAGCAGCCGAGATTCCCGACTCGCTCCACCATTGGGGGCTAGGAGTGAAGCGTGTCACC**ATG**G
TCAGCTCATGGCCAGCCAGGAAAGCCTCTCTGCTGTGCGTCTGTGCAGTTCTTGTTCTTCCCT
GGAGGACTCTTGGATCGCCTGTGATCTTGGCCAGGAGACCAGGTGCCTGGGTCCCTTCCTGGA
AGGGGACAAGTTACACACCCCAGCCCCATTTTCCCACCAACTTCTACATGCCTTGGGAGAACC
TTCTACATGTTGGCTGCCCCCTTCCCCTATTTCAGCAGTGCCCAGTCCTGCTTATAAACCTGA
GGCCTGCTCCCCATACCTTCCCTGTGCAAGTGCCAGCCGTTATTCCAGGCAGCCCAATGTTGT
TGAGGCCAGATGGATTCCTGGAAGCAGCTGGCCCATGGATG**TGA**GTCATCACAGTATTCTAGA
AACAGAGAAGAGGTCTTAACCTAATGCGCATAGAGAAATTGTTCTCATTGTAAACATACCCCT
GTCCTTAGCTGATCTAGGTGGAAGCCCAGCTTCATGTGCTAGGGGGCATGATAATGATAATAA
AGGAATTGTATCTAGGACTAA

# FIGURE 228

MVSSWPARKASLLCVCAVLVLPWRTLGSPVILARRPGAWVPSWKGTSYTPQPHFPTNFYMPWE
NLLHVGCPLPLFQQCPVLLINLRPAPHTFPVQVPAVIPGSPMLLRPDGFLEAAGPWM

**Signal peptide:**
amino acids 1-27

**cAMP- and cGMP-dependent protein kinase phosphorylation site.**
amino acids 8-12

# FIGURE 229

GGGAAGGGATGCAAGGAAGCCCTCCGGCGCTGCGCTCCGAGGCGGGAGACAGCGTCCCGCTGA
AAATGTGTGTCTGACATGCAAGCTCAGTGGGGCAGAGACCCGTGGATTGCTGTGCCCTGCCCT
CCGGACCTGGATC**ATG**AAGGTGTTGGGAAGAAGCTTCTTCTGGGTGCTGTTTCCCGTCCTTCC
CTGGGCGGTGCAGGCTGTGGAGCACGAGGAGGTGGCGCAGCGTGTGATCAAACTGCACCGCGG
GCGAGGGGTGGCTGCCATGCAGAGCCGGCAGTGGGTCCGGGACAGCTGCAGGAAGCTCTCAGG
GCTTCTCCGCCAGAAGAATGCAGTTCTGAACAAACTGAAAACTGCAATTGGAGCAGTGGAGAA
AGACGTGGGCCTGTCGGATGAAGAGAAACTGTTTCAGGTGCACACGTTTGAAATTTTCCAGAA
AGAGCTGAATGAAAGTGAAAATTCCGTTTTCCAAGCTGTCTACGGACTGCAGAGAGCCCTGCA
GGGGGATTACAAAGATGTCGTGAACATGAAGGAGAGCAGCCGGCAGCGCCTGGAGGCCCTGAG
AGAGGCTGCAATAAAGGAAGAAACAGAATATATGGAACTTCTGGCAGCAGAAAAACATCAAGT
TGAAGCCCTTAAAAATATGCAACATCAAAACCAAAGTTTATCCATGCTTGACGAGATTCTTGA
AGATGTAAGAAAGGCAGCGGATCGTCTGGAGGAAGAGATAGAGGAACATGCTTTTGACGACAA
TAAATCAGTCAAGGGGGTCAATTTTGAGGCAGTTCTGAGGGTGGAGGAAGAAGAGGCCAATTC
TAAGCAAAATATAACAAAACGAGAAGTGGAGGATGACTTGGGTCTTAGCATGCTGATTGACTC
CCAGAACAACCAGTATATTTTGACCAAGCCCAGAGATTCAACCATCCCACGTGCAGATCACCA
CTTTATAAAGGACATTGTTACCATAGGAATGCTGTCCTTGCCTTGTGGCTGGCTATGTACAGC
CATAGGATTGCCTACAATGTTTGGTTATATTATTTGTGGTGTACTTCTGGGACCTTCAGGACT
AAATAGTATTAAGTCTATTGTGCAAGTGGAGACATTAGGAGAATTTGGGGTGTTTTTTACTCT
TTTTCTTGTTGGCTTAGAATTTTCTCCAGAAAAGCTAAGAAAGGTGTGGAAGATTTCCTTACA
AGGGCCGTGTTACATGACACTGTTAATGATTGCATTTGGCTTGCTGTGGGGGCATCTCTTGCG
GATCAAACCCACGCAGAGCGTCTTCATTTCCACGTGTCTGTCCTTGTCAAGCACACCCCTCGT
GTCCAGGTTCCTCATGGGCAGTGCTCGGGGTGACAAAGAAGGCGACATTGACTACAGCACCGT
GCTCCTCGGCATGCTGGTGACGCAGGACGTGCAGCTCGGGCTCTTCATGGCCGTCATGCCGAC
TCTCATACAGGCGGGCGCCAGTGCATCTTCTAGCATTGTCGTGGAAGTTCTCCGAATCCTGGT
TTTGATTGGTCAGATTCTTTTTTCACTAGCGGCGGTTTTTCTTTTATGTCTTGTTATAAAGAA
GTATCTCATTGGACCCTATTATCGGAAGCTGCACATGGAAAGCAAGGGGAACAAAGAAATCCT
GATCTTGGGAATATCTGCCTTTATCTTCTTAATGTTAACGGTCACGGAGCTGCTGGACGTCTC
CATGGAGCTGGGCTGTTTCCTGGCTGGAGCGCTCGTCTCCTCTCAGGGCCCCGTGGTCACCGA
GGAGATCGCCACCTCCATCGAACCCATCCGCGACTTCCTGGCCATCGTTTTCTTCGCCTCCAT
AGGGCTCCACGTGTTCCCCACGTTTGTGGCGTACGAGCTCACGGTGCTGGTGTTCCTCACCTT
GTCAGTGGTGGTGATGAAGTTTCTCCTGGCGGCGCTGGTCCTGTCTCTCATTCTGCCGAGGAG
CAGCCAGTACATCAAGTGGATCGTCTCTGCGGGGCTTGCCCAGGTCAGCGAGTTTCCTTTGT
CCTGGGGAGCCGGGCGCGAAGAGCGGGCGTCATCTCTCGGGAGGTGTACCTCCTTATACTGAG
TGTGACCACGCTCAGCCTCTTGCTCGCCCCGGTGCTGTGGAGAGCTGCAATCACGAGGTGTGT
GCCCAGACCGGAGAGACGGTCCAGCCTC**TGA**TGGCTCGGAGATGATGGACCGTGGAAGGGAAG
CGTCTGTGGGGAGTGAGCGCTTAGATGGCCAGCAGCTGCTCCTTCTGGGAAGCTCGCACCTTG
GCAACAGAACAGCCCTCTAGCAGAGCGTCAGTGCAGTCGTGTTATCCCGGCTTTTACAGAATA
TTCTTGTCCTATTTTAGAATTTTCCGGAGTAGTTTATTTGCAGTCTGTTGATTATGTGCAGTA
GACCCGGGACACTGCGTTTTACCGATCACCTTGAATGTGGTGCCTGGATGTGCCTTTTTTTTT
TTTCCCTGAAATTATTATTAATTTCTATTGTGAGTTCATCAGTTCATAGTTTTTTAGTAAA
GAAGCAAAATTAAAAGGCTTTTAAAAATGTACAACTTCAGAATTATAATCTGTTAGTCAAATA
TTTGTTATTAAACATTTCTGTAATATGAAGTTGTAATCCTGGCCGTGAGCTTGGAAGCTTACT
TTTGATTCTTAAAGCCTATGTTTTCTAAAATGAGACAAATACGGATGTCTATTTGCCTTTTAT
TGTAACTTTTAAATGAAATAATTTCATGTCAATTTCTATTAGATATATCACTTAAAATATTTG
GTTTTAAATCACAAGAATATGTATTCTTTAATAAAGATAATTTATGATCATGGTAAAAAAAAAA

# FIGURE 230

MKVLGRSFFWVLFPVLPWAVQAVEHEEVAQRVIKLHRGRGVAAMQSRQWVRDSCRKLSGLLRQ
KNAVLNKLKTAIGAVEKDVGLSDEEKLFQVHTFEIFQKELNESENSVFQAVYGLQRALQGDYK
DVVNMKESSRQRLEALREAAIKEETEYMELLAAEKHQVEALKNMQHQNQSLSMLDEILEDVRK
AADRLEEEIEEHAFDDNKSVKGVNFEAVLRVEEEEANSKQNITKREVEDDLGLSMLIDSQNNQ
YILTKPRDSTIPRADHHFIKDIVTIGMLSLPCGWLCTAIGLPTMFGYIICGVLLGPSGLNSIK
SIVQVETLGEFGVFFTLFLVGLEFSPEKLRKVWKISLQGPCYMTLLMIAFGLLWGHLLRIKPT
QSVFISTCLSLSSTPLVSRFLMGSARGDKEGDIDYSTVLLGMLVTQDVQLGLFMAVMPTLIQA
GASASSSIVVEVLRILVLIGQILFSLAAVFLLCLVIKKYLIGPYYRKLHMESKGNKEILILGI
SAFIFLMLTVTELLDVSMELGCFLAGALVSSQGPVVTEEIATSIEPIRDFLAIVFFASIGLHV
FPTFVAYELTVLVFLTLSVVVMKFLLAALVLSLILPRSSQYIKWIVSAGLAQVSEFSFVLGSR
ARRAGVISREVYLLILSVTTLSLLLAPVLWRAAITRCVPRPERRSSL

**Signal peptide:**
amino acids 1-22

**Transmembrane domains:**
amino acids 282-304, 322-337, 354-370, 379-395, 445-474, 501-520, 576-598, 641-660

**N-glycosylation sites.**
amino acids 104-108, 174-178, 206-210, 230-234

**cAMP- and cGMP-dependent protein kinase phosphorylation site.**
amino acids 55-59, 673-677

**Tyrosine kinase phosphorylation site.**
amino acids 407-414

**N-myristoylation sites.**
amino acids 116-122, 327-333, 366-372, 401-407, 419-425, 429-435, 442-448, 525-531, 530-536

**Cell attachment sequence.**
amino acids 404-407

# FIGURE 231

GAGAAAAACAACAGGAAGCAGCTTACAAACTCGGTGAACAACTGAGGGAACCAAACCAGAGAC
GCGCTGAACAGAGAGAATCAGGCTCAAAGCAAGTGGAAGTGGGCAGAGATTCCACCAGGACTG
GTGCAAGGCGCAGAGCCAGCCAGATTTGAGAAGAAGGCAAAAAG**ATG**CTGGGGAGCAGAGCTG
TAATGCTGCTGTTGCTGCTGCCCTGGACAGCTCAGGGCAGAGCTGTGCCTGGGGGCAGCAGCC
CTGCCTGGACTCAGTGCCAGCAGCTTTCACAGAAGCTCTGCACACTGGCCTGGAGTGCACATC
CACTAGTGGGACACATGGATCTAAGAGAAGAGGGAGATGAAGAGACTACAAATGATGTTCCCC
ATATCCAGTGTGGAGATGGCTGTGACCCCCAAGGACTCAGGGACAACAGTCAGTTCTGCTTGC
AAAGGATCCACCAGGGTCTGATTTTTATGAGAAGCTGCTAGGATCGGATATTTTCACAGGGG
AGCCTTCTCTGCTCCCTGATAGCCCTGTGGGCCAGCTTCATGCCTCCCTACTGGGCCTCAGCC
AACTCCTGCAGCCTGAGGGTCACCACTGGGAGACTCAGCAGATTCCAAGCCTCAGTCCCAGCC
AGCCATGGCAGCGTCTCCTTCTCCGCTTCAAAATCCTTCGCAGCCTCCAGGCCTTTGTGGCTG
TAGCCGCCCGGGTCTTTGCCCATGGAGCAGCAACCCTGAGTCCC**TAA**AGGCAGCAGCTCAAGG
ATGGCACTCAGATCTCCATGGCCCAGCAAGGCCAAGATAAATCTACCACCCCAGGCACCTGTG
AGCCAACAGGTTAATTAGTCCATTAATTTTAGTGGGACCTGCATATGTTGAAAATTACCAATA
CTGACTGACATGTGATGCTGACCTATGATAAGGTTGAGTATTTATTAGATGGGAAGGGAAATT
TGGGGATTATTTATCCTCCTGGGGACAGTTTGGGGAGGATTATTTATTGTATTTATATTGAAT
TATGTACTTTTTTCAATAAAGTCTTATTTTTGTGGCTAAAAAAAAAAAAA

# FIGURE 232

MLGSRAVMLLLLLPWTAQGRAVPGGSSPAWTQCQQLSQKLCTLAWSAHPLVGHMDLREEGDEE
TTNDVPHIQCGDGCDPQGLRDNSQFCLQRIHQGLIFYEKLLGSDIFTGEPSLLPDSPVGQLHA
SLLGLSQLLQPEGHHWETQQIPSLSPSQPWQRLLLRFKILRSLQAFVAVAARVFAHGAATLSP

**Important features of the protein:**

**Signal peptide:**

amino acids 1-21

**Casein kinase II phosphorylation site.**

amino acids 64-67

**N-myristoylation sites.**

amino acids 25-30, 81-86, 122-127

# FIGURE 233

CCCACGCGTCCGGCCCTGTAACCAAGATACTGACTGAACATGGCTGGCGGACTCAGGCTGGGGTCTGCAGTGCAG
CATTAATGGGCCGCTGACATGAATATGGAGTAGTTTTCTCTAGCAAAGAGTA**ATG**TGGGCCATGGAGTCAGGCCA
CCTCCTCTGGGCTCTGCTGTTCATGCAGTCCTTGTGGCCTCAACTGACTGATGGAGCCACTCGAGTCTACTACCT
GGGCATCCGGGATGTGCAGTGGAACTATGCTCCCAAGGGAAGAAATGTCATCACGAACCAGCCTCTGGACAGTGA
CATAGTGGCTTCCAGCTTCTTAAAGTCTGACAAGAACCGGATAGGGGGAACCTACAAGAAGACCATCTATAAAGA
ATACAAGGATGACTCATACACAGATGAAGTGGCCCAGCCTGCCTGGTTGGGCTTCCTGGGGCCAGTGTTGCAGGC
TGAAGTGGGGGATGTCATTCTTATTCACCTGAAGAATTTTGCCACTCGTCCCTATACCATCCACCCTCATGGTGT
CTTCTACGAGAAGGACTCTGAAGGTTCCCTATACCCAGATGGCTCCTCTGGGCCACTGAAAGCTGATGACTCTGT
TCCCCCGGGGGGCAGCCATATCTACAACTGGACCATTCCAGAAGGCCATGCACCCACCGATGCTGACCCAGCGTG
CCTCACCTGGATCTACCATTCTCATGTAGATGCTCCACGAGACATTGCAACTGGCCTAATTGGGCCTCTCATCAC
CTGTAAAAGAGGAGCCCTGGATGGGAACTCCCCTCCTCAACGCCAGGATGTAGACCATGATTTCTTCCTCCTCTT
CAGTGTGGTAGATGAGAACCTCAGCTGGCATCTCAATGAGAACATTGCCACTTACTGCTCAGATCCTGCTTCAGT
GGACAAAGAAGATGAGACATTTCAGGAGAGCAATAGGATGCATGCAATCAATGGCTTTGTTTTTGGGAATTTACC
TGAGCTGAACATGTGTGCACAGAAACGTGTGGCCTGGCACTTGTTTGGCATGGGCAATGAAATTGATGTCCACAC
AGCATTTTTCCATGGACAGATGCTGACTACCCGTGGACACCACACTGATGTGGCTAACATCTTTCCAGCCACCTT
TGTGACTGCTGAGATGGTGCCCTGGGAACCTGGTACCTGGTTAATTAGCTGCCAAGTGAACAGTCACTTTCGAGA
TGGCATGCAGGCACTCTACAAGGTCAAGTCTTGCTCCATGGCCCCTCCTGTGGACCTGCTCACAGGCAAAGTTCG
ACAGTACTTCATTGAGGCCCATGAGATTCAATGGGACTATGGCCCGATGGGGCATGATGGGAGTACTGGGAAGAA
TTTGAGAGAGCCAGGCAGTATCTCAGATAAGTTTTTCCAGAAGAGCTCCAGCCGAATTGGGGGCACTTACTGGAA
AGTGCGATATGAAGCCTTTCAAGATGAGACATTCCAAGAGAAGATGCATTTGGAGGAAGATAGGCATCTTGGAAT
CCTGGGGCCAGTGATCCGGGCTGAGGTGGGTGACACCATTCAGGTGGTCTTCTACAACCGTGCCTCCCAGCCATT
CAGCATGCAGCCCCATGGGGTCTTTTATGAGAAAGACTATGAAGGCACTGTGTACAATGATGGCTCATCTTACCC
TGGCTTGGTTGCCAAGCCCTTTGAGAAAGTAACATACCGCTGGACAGTCCCCCCTCATGCCGGTCCCACTGCTCA
GGATCCTGCTTGTCTCACTTGGATGTACTTCTCTGCTGCAGATCCCATAAGAGACACAAATTCTGGCCTGGTGGG
CCCGCTGCTGGTGTGCAGGGCTGGTGCCTTGGGTGCAGATGGCAAGCAGAAAGGGGTGGATAAAGAATTCTTTCT
TCTCTTCACTGTGTTGGATGAGAACAAGAGCTGGTACAGCAATGCCAATCAAGCAGCTGCTATGTTGGATTTCCG
ACTGCTTTCAGAGGATATTGAGGGCTTCCAAGACTCCAATCGGATGCATGCCATTAATGGGTTTCTGTTCTCTAA
CCTGCCCAGGCTGGACATGTGCAAGGGTGACACAGTGGCCTGGCACCTGCTCGGCCTGGGCACAGAGACTGATGT
GCATGGAGTCATGTTCCAGGGCAACACTGTGCAGCTTCAGGGCATGAGGAAGGGTGCAGCTATGCTCTTTCCTCA
TACCTTTGTCATGGCCATCATGCAGCCTGACAACCTTGGGACATTTGAGATTTATTGCCAGGCAGGCAGCCATCG
AGAAGCAGGGATGAGGGCAATCTATAATGTCTCCCAGTGTCCTGGCCACCAAGCCACCCCTCGCCAACGCTACCA
AGCTGCAAGAATCTACTATATCATGGCAGAAGAAGTAGAGTGGGACTATTGCCCTGACCGGAGCTGGGAACGGGA
ATGGCACAACCAGTCTGAGAAGGACAGTTATGGTTACATTTTCCTGAGCAACAAGGATGGGCTCCTGGGTTCCAG
ATACAAGAAAGCTGTATTCAGGGAATACACTGATGGTACATTCAGGATCCCTCGGCCAAGGACTGGACCAGAAGA
ACACTTGGGAATCTTGGGTCCACTTATCAAAGGTGAAGTTGGTGATATCCTGACTGTGGTATTCAAGAATAATGC
CAGCCGCCCCTACTCTGTGCATGCTCATGGAGTGCTAGAATCTACTACTGTCTGGCCACTGGCTGCTGAGCCTGG
TGAGGTGGTCACTTATCAGTGGAACATCCCAGAGAGGTCTGGCCCTGGGCCCAATGACTCTGCTTGTGTTTCCTG
GATCTATTATTCTGCAGTGGATCCCATCAAGGACATGTATAGTGGCCTGGTGGGGCCCTTGGCTATCTGCCAAAA
GGGCATCCTGGAGCCCCATGGAGGACGGAGTGACATGGATCGGGAATTTGCATTGTTGTTCTTGATTTTTGATGA
AAATAAGTCTTGGTATTTGGAGGAAAATGTGGCAACCCATGGGTCCCAGGATCCAGGCAGTATTAACCTACAGGA
TGAAACTTTCTTGGAGAGCAATAAAATGCATGCAATCAATGGGAAACTCTATGCCAACCTTAGGGGTCTTACCAT
GTACCAAGGAGAACGAGTGGCCTGGTACATGCTGGCCATGGGCCAAGATGTGGATCTACACACCATCCACTTTCA
TGCAGAGAGCTTCCTCTATCGGAATGGCGAGAACTACCGGGCAGATGTGGTGGATCTGTTCCCAGGGACTTTTGA
GGTTGTGGAGATGGTGGCCAGCAACCCTGGGACATGGCTGATGCACTGCCATGTGACTGACCATGTCCATGCTGG
CATGGAGACCCTCTTCACTGTTTTTTCTCGAACAGAACACTTAAGCCCTCTCACCGTCATCACCAAAGAGACTGA
AAAAGTGCCCCCCAGAGACATTGAAGAAGGCAATGTGAAGATGCTGGGCATGCAGATCCCCATAAAGAATGTTGA
GATGCTGGCCTCTGTTTTGGTTGCCATTAGTGTCACCCTTCTGCTCGTTGTTCTGGCTCTTGGTGGAGTGGTTTG
GTACCAACATCGACAGAGAAAGCTACGACGCAATAGGAGGTCCATCCTGGATGACAGCTTCAAGCTTCTGTCTTT
CAAACAG**TAA**CATCTGGAGCCTGGAGATATCCTCAGGAAGCACATCTGTAGTGCACTCCCAGCAGGCCATGGACT
AGTCACTAACCCCACACTCAAAGGGGCATGGGTGGTGGAGAAGCAGAAGGAGCAATCAAGCTTATCTGGATATTT
CTTTCTTTATTTATTTTACATGGAAATAATATGATTTCACTTTTTCTTTAGTTTCTTTGCTCTACGTGGGCACCT
GGCACTAAGGGAGTACCTTATTATCCTACATCGCAAATTTCAACAGCTACATTATATTTCCTTCTGACACTTGGA
AGGTATTGAAATTTCTAGAAATGTATCCTTCTCACAAAGTAGAGACCAAGAGAAAAACTCATTGATTGGGTTTCT
ACTTCTTTCAAGGACTCAGGAAATTTCACTTTGAACTGAGGCCAAGTGAGCTGTTAAGATAACCCACACTTAAAC
TAAAGGCTAAGAATATAGGCTTGATGGAAATTGAAGGTAGGCTGAGTATTGGGAATCCAAATTGAATTTTGATT
CTCCTTGGCAGTGAACTACTTTGAAGAAGTGGTCAATGGGTTGTTGCTGCCATGAGCATGTACAACCTCTGGAGC
TAGAAGCTCCTCAGGAAAGCCAGTTCTCCAAGTTCTTAACCTGTGGCACTGAAAGGAATGTTGAGTTACCTCTTC
ATGTTTTAGACAGCAAACCCTATCCATTAAAGTACTTGTTAGACCAAAAAAAAAAAAA

# FIGURE 234

MWAMESGHLLWALLFMQSLWPQLTDGATRVYYLGIRDVQWNYAPKGRNVITNQPLDSDIVASS
FLKSDKNRIGGTYKKTIYKEYKDDSYTDEVAQPAWLGFLGPVLQAEVGDVILIHLKNFATRPY
TIHPHGVFYEKDSEGSLYPDGSSGPLKADDSVPPGGSHIYNWTIPEGHAPTDADPACLTWIYH
SHVDAPRDIATGLIGPLITCKRGALDGNSPPQRQDVDHDFFLLFSVVDENLSWHLNENIATYC
SDPASVDKEDETFQESNRMHAINGFVFGNLPELNMCAQKRVAWHLFGMGNEIDVHTAFFHGQM
LTTRGHHTDVANIFPATFVTAEMVPWEPGTWLISCQVNSHFRDGMQALYKVKSCSMAPPVDLL
TGKVRQYFIEAHEIQWDYGPMGHDGSTGKNLREPGSISDKFFQKSSSRIGGTYWKVRYEAFQD
ETFQEKMHLEEDRHLGILGPVIRAEVGDTIQVVFYNRASQPFSMQPHGVFYEKDYEGTVYNDG
SSYPGLVAKPFEKVTYRWTVPPHAGPTAQDPACLTWMYFSAADPIRDTNSGLVGPLLVCRAGA
LGADGKQKGVDKEFFLLFTVLDENKSWYSNANQAAAMLDFRLLSEDIEGFQDSNRMHAINGFL
FSNLPRLDMCKGDTVAWHLLGLGTETDVHGVMFQGNTVQLQGMRKGAAMLFPHTFVMAIMQPD
NLGTFEIYCQAGSHREAGMRAIYNVSQCPGHQATPRQRYQAARIYYIMAEEVEWDYCPDRSWE
REWHNQSEKDSYGYIFLSNKDGLLGSRYKKAVFREYTDGTFRIPRPRTGPEEHLGILGPLIKG
EVGDILTVVFKNNASRPYSVHAHGVLESTTVWPLAAEPGEVVTYQWNIPERSGPGPNDSACVS
WIYYSAVDPIKDMYSGLVGPLAICQKGILEPHGGRSDMDREFALLFLIFDENKSWYLEENVAT
HGSQDPGSINLQDETFLESNKMHAINGKLYANLRGLTMYQGERVAWYMLAMGQDVDLHTIHFH
AESFLYRNGENYRADVVDLFPGTFEVVEMVASNPGTWLMHCHVTDHVHAGMETLFTVFSRTEH
LSPLTVITKETEKVPPRDIEEGNVKMLGMQIPIKNVEMLASVLVAISVTLLLVVLALGGVVWY
QHRQKLRRNRRSILDDSFKLLSFKQ

**Signal peptide:**

amino acids 1-21

**Transmembrane domain:**

amino acids 1109-1130

**N-glycosylation sites.**

amino acids 167-171, 239-243, 591-595, 717-721, 761-765, 832-836, 876-880, 934-938

**Glycosaminoglycan attachment site.**

amino acids 871-875

**Tyrosine kinase phosphorylation sites.**

amino acids 82-90, 137-145, 494-502, 513-521

**N-myristoylation sites.**

amino acids 212-218, 313-319, 498-504, 566-572, 672-678, 778-784, 843-849

**Multicopper oxidases signature 1.**

amino acids 344-365, 696-717, 1043-1064

**Multicopper oxidases signature 2.**

amino acids 1048-1060

# FIGURE 235

GGAAAGAGTGCTGGTACTACAACCAGGAAGTGACAGATAATGTGCTTTAAACTACATTAGAAAAGCTTCTCATAG
CAAAACTGAGAGATTGAAGCAGTGATTATTTTTACATAGTTGTCATTAAATATTTGGAGCTCTGCTGTGCATAGA
GATGGCAACATACTTAGAATACACAGCTTTCTGGGCCAGAAATTGATCTTCTGACTTTTGAGCCTTATCTGATTA
CTGCTTGGTTCATCTTTATTTTGTTAAACTACTCTGTAGGCTGAAAGGGAGAGACTCTCCTTGGTTTGCAGAGCC
TGACTAGACAGGAATTCTGGCAACTGCTCCAGCAGAACTATGGCACTGAGCTAGGTTTAAATGCTGAGGAG**ATG**G
AAAACTTGTCACTGTCGATTGAGGATGTGCAGCCAAGAAGTCCAGGAAGAAGCAGCTTGGATGACTCTGGGGAGA
GAGATGAAAAATTATCCAAGTCAATCAGTTTTACCAGTGAATCAATTAGTCGGGTTTCAGAAACAGAGTCATTCG
ATGGAAATTCATCAAAAGGAGGATTAGGCAAAGAGGAGTCCCAAAATGAGAAACAGACCAAAAAGAGTCTCTTAC
CAACTTTGGAAAAGAAGTTAACTAGAGTGCCATCAAAGTCACTGGACTTGAATAAAAATGAATATCTTTCTCTGG
ACAAAAGCAGCACTTCAGATTCTGTTGATGAAGAAAATGTTCCTGAGAAAGATCTTCATGGAAGACTTTTTATCA
ACCGTATTTTTCATATCAGTGCTGACAGAATGTTTGAATTGCTCTTTACCAGTTCACGCTTTATGCAGAAATTTG
CCAGTTCTAGAAATATAATAGATGTAGTATCTACCCCTTGGACTGCAGAACTTGGAGGTGATCAGCTGAGAACGA
TGACCTACACTATAGTCCTTAATAGTCCACTTACTGGAAAATGCACTGCTGCCACTGAAAAGCAGACACTGTATA
AAGAAAGTCGGGAAGCACGATTTTATTTGGTAGATTCAGAAGTACTGACACATGATGTCCCCTACCATGATTACT
TCTATACCGTGAACAGATACTGTATCATCCGATCTTCAAAACAGAAATGCAGGCTAAGAGTTTCCACAGATTTGA
AATACAGAAAACAGCCATGGGGCCTTGTCAAATCTTTAATTGAAAAGAATTCCTGGAGTTCTTTGGAGGACTATT
TCAAACAGCTTGAATCAGATTTGTTAATTGAAGAATCTGTATTAAATCAGGCCATTGAAGACCCTGGAAAACTTA
CTGGCCTACGAAGGAGAAGGCGAACCTTCAACCGAACAGCAGAAACAGTTCCTAAACTTTCCTCTCAGCATTCCT
CTGGAGATGTGGGCTTAGGTGCCAAAGGGGATATTACAGGAAAGAAAAAGGAAATGGAAAACTATAACGTCACTC
TTATTGTGGTAATGAGTATTTTTGTGTTGTTATTAGTTTTGTTGAATGTGACACTGTTTCTGAAGCTGTCAAAGA
TAGAACATGCTGCTCAGTCCTTTTACCGTCTCCGCCTCCAAGAAGAGAAATCTTTAAATTTAGCCTCTGATATGG
TGTCAAGAGCAGAAACTATTCAGAAGAATAAAGATCAGGCCCATCGTTTAAAGGGAGTGCTCCGAGACTCCATAG
TGATGCTTGAACAGCTGAAGAGCTCACTCATTATGCTTCAGAAAACGTTTGATCTACTAAATAAGAATAAGACTG
GCATGGCTGTTGAAAGC**TAG**TGATCTGAAGGACTAAAACCGCAGAGATACTTGGAACTTAAAGAAAATACCTGGA
AGAAAACCAGACGAATGAAGGATTTTGGCATAGAACATTTCTATGTTTTTTCATTATTGAGATTTCTAATATGAA
CATTTCTTTCAGTAACATTTATTTGATAATTAGTTTCTGCTGGCCTTAATAATCCATCCTTTCACTTCTTATAGA
TATTTTTAAGCTGTGAATTTCTTCAGTGAACCATGAAATATATTATAGAACTGAATTTCTCTGATACAAAAAGAA
AATGACACACCCTGAATTGAGTGGTATGGTCTCATTTCTACAGTGAAGTCTGATGCTTTGTTAGCACAGAATCCG
TACATGTCCAATAGGTCGCTTTTGTAACTGAGATAAGACCAAGAGGATAAACAGGACAATATAAGAAGAAACCTC
TATGTCATTACTGATTTTAAAGGTTCTGTTTTCAGGCATATAACATTTCCAGGTTTGTGTACTGTAAAGATTATA
ATGTCTTCATTTATTTAGCATGCAAATTTAATAGTCAAACTTTTTGAATCTGCATGTTGATGATGATTATCAGAA
AGGGTCTTCTGCCATGCTGTATCTTTATGAAAGAAATAGTTGTTTTTTCTTAAGGTAACTATCAGAGGTGGGATT
ATCTTGCCTCCTCACTTAGAATACCAACAGTCAAAAGGAAGAACCATCCTCTGAGTTTTAAAAACCAGAAGGTTA
TGTTAAAATCTGGGCATTTAGTGACAGATCAAATGCATACTTGAACTAAGATTGGCTTCAGCTTAGCAGTCTTTC
ATGGTGGAAGTGACACATCTGGTTGAAAATAATTTGTGTATTTTCAGTAACCATGTATGGCTTCCTTCTTTATGT
ATGTGTGTGACTTGTTTTAATTGGTAAGTTATAAGCCAGACATAGATTTTAGCTCTTTAATAAAAACTTCAGGGG
CACGTATGTCCCAGTACAAGTGTACTGACTATCAAGTTTTAACTCAGATGCAAGCTTTGGCTCTTTCATAAAAAG
TTTTTATGCATATGTGTCTCCATACAAGTGGCTCATTAAAATAAGAACTTTGTAAACTGACTTAAAATCAGATAT
TTTTTCAAGAGTTAGGGAAAGTTGAAGTGTTTTACTGTTTTGTCTCTTGAGCCCTTTCTCTGGGGAAAAAATACA
TATCCATCTATCTATCTATATATAAACTGTGTATACATTCTTACTGTTTGAACAACTATTGCCTTTAATTAAATG
TTTCATTTTTCTCCAGAGTCCCCAAAGCCACATGGCATTATTATAGTCATTTTTGAGATGCCTGTAGAGAATGAA
AGTATTGACTCCGTTAGAGGGAAAATGGGTTTCTCTGGGTGAATTCCAACGAAGCATACCTAGGGGTAACAGTGA
ACCTACCTGGGTTTGTTTTGTTTTGGTAAGGATTTATGTAGTGTCTGGCTGTAAGCAAGAATGAGTGGATTATAA
ACTTGAAGATTTCTCTGTTAAAGTCACAAAAATGATCGACAAACAATATTTTTGTGATGTTTATTTAAACGTTGT
ATTTTATAACATACTTCAAGGAAGAGTATCGAAGTAAGTTGCTTTATAAATTAAGACTAAATTCGTATGGATGCA
GAATTCAATTAATAAAATTTGAGCCTGTTACGTAAATTGAATATTAATAAAATTGAAAATTTCAAAA

# FIGURE 236

MENLSLSIEDVQPRSPGRSSLDDSGERDEKLSKSISFTSESISRVSETESFDGNSSKGGLGKE
ESQNEKQTKKSLLPTLEKKLTRVPSKSLDLNKNEYLSLDKSSTSDSVDEENVPEKDLHGRLFI
NRIFHISADRMFELLFTSSRFMQKFASSRNIIDVVSTPWTAELGGDQLRTMTYTIVLNSPLTG
KCTAATEKQTLYKESREARFYLVDSEVLTHDVPYHDYFYTVNRYCIIRSSKQKCRLRVSTDLK
YRKQPWGLVKSLIEKNSWSSLEDYFKQLESDLLIEESVLNQAIEDPGKLTGLRRRRRTFNRTA
ETVPKLSSQHSSGDVGLGAKGDITGKKKEMENYNVTLIVVMSIFVLLLVLLNVTLFLKLSKIE
HAAQSFYRLRLQEEKSLNLASDMVSRAETIQKNKDQAHRLKGVLRDSIVMLEQLKSSLIMLQK
TFDLLNKNKTGMAVES

**Transmembrane domain:**
amino acids 352-371

**N-glycosylation sites.**
amino acids 3-7, 54-58, 312-316, 349-353, 367-371, 449-453

**cAMP- and cGMP-dependent protein kinase phosphorylation sites.**
amino acids 81-85, 307-311

**Tyrosine kinase phosphorylation sites.**
amino acids 202-211, 246-254, 341-349

**N-myristoylation site.**
amino acids 259-265

**Amidation site.**
amino acids 339-343

# FIGURE 237

CAGGGGCTGGAGGGCAGGGGAGGGG**ATG**ATGTCATTCCTGCTCGGCGCAATCCTGACCCTGCT
CTGGGCGCCCACGGCTCAGGCTGAGGTTCTGCTGCAGCCTGACTTCAATGCTGAAAAGTTCTC
AGGCCTCTGGTACGTGGTCTCCATGGCATCTGACTGCAGGGTCTTCCTGGGCAAGAAGGACCA
CCTGTCCATGTCCACCAGGGCCATCAGGCCCACAGAGGAGGGCGGCCTCCACGTCCACATGGA
GTTCCCGGGGGCGGACGGCTGTAACCAGGTGGATGCCGAGTACCTGAAGGTGGGCTCCGAGGG
ACACTTCAGAGTCCCGGCCTTGGGCTACCTGGACGTGCGCATCGTGGACACAGACTACAGCTC
CTTCGCCGTCCTTTACATCTACAAGGAGCTGGAGGGGGCCCTCAGCACCATGGTGCAGCTCTA
CAGCCGGACCCAGGATGTGAGTCCCCAGGCTCTGAAGTCCTTCCAGGACTTCTACCCGACCCT
GGGGCTCCCCAAGGACATGATGGTCATGCTGCCCCAGTCAGATGCATGCAACCCTGAGAGCAA
GGAGGCGCCC**TGA**CACCTCCGGAGCCCCACCCCCGCCCTTCCCAGGTGGAGCCAAAGCAGCAG
GCGCCTTTGCCCCTGGAGTCAAGACCCACAGCCCTCGGGGACCACCTGGAGTCTCTCCATCCT
CCACCCCCCGCCTGTGGGATGCCTTGTGGGACGTCTCTTTCTATTCAATAAACAGATGCTGCA
GCCTCA

# FIGURE 238

MMSFLLGAILTLLWAPTAQAEVLLQPDFNAEKFSGLWYVVSMASDCRVFLGKKDHLSMSTRAI
RPTEEGGLHVHMEFPGADGCNQVDAEYLKVGSEGHFRVPALGYLDVRIVDTDYSSFAVLYIYK
ELEGALSTMVQLYSRTQDVSPQALKSFQDFYPTLGLPKDMMVMLPQSDACNPESKEAP

**Signal peptide:**
amino acids 1-20

**Tyrosine kinase phosphorylation site.**
amino acids 110-117

**N-myristoylation sites.**
amino acids 7-13, 79-85, 130-136

**Amidation site.**
amino acids 50-54

# FIGURE 239

GGCGCGCTGGTCCAGGTGAGCGGGCGCGTCCCCGCGACGGCGCTGCCTGCCCGAGGCGGTTCA
CGTAAAGACAGCGAGATCCTGAGGGCCAGCCGGGAAGGAGGCGTGGATATGGAGCTGGCTGCT
GCCAAGTCCGGGGCCCGCGCCGCTGCCTAGCGCGTCCTGGGGACTCTGTGGGGACGCGCCCCG
CGCCGCGGCTCGGGGACCCGTAGAGCCCGGCGCTGCGCGC**ATG**GCCCTGCTCTCGCGCCCCGC
GCTCACCCTCCTGCTCCTCCTCATGGCCGCTGTTGTCAGGTGCCAGGAGCAGGCCCAGACCAC
CGACTGGAGAGCCACCCTGAAGACCATCCGGAACGGCGTTCATAAGATAGACACGTACCTGAA
CGCCGCCTTGGACCTCCTGGGAGGCGAGGACGGTCTCTGCCAGTATAAATGCAGTGACGGATC
TAAGCCTTTCCCACGTTATGGTTATAAACCCTCCCCACCGAATGGATGTGGCTCTCCACTGTT
TGGTGTTCATCTTAACATTGGTATCCCTTCCCTGACAAAGTGTTGCAACCAACACGACAGGTG
CTATGAGACCTGTGGCAAAAGCAAGAATGACTGTGATGAAGAATTCCAGTATTGCCTCTCCAA
GATCTGCCGAGATGTACAGAAAACACTAGGACTAACTCAGCATGTTCAGGCATGTGAAACAAC
AGTGGAGCTCTTGTTTGACAGTGTTATACATTTAGGTTGTAAACCATATCTGGACAGCCAACG
AGCCGCATGCAGGTGTCATTATGAAGAAAAAACTGATCTT**TAA**AGGAGATGCCGACAGCTAGT
GACAGATGAAGATGGAAGAACATAACCTTTGACAAATAACTAATGTTTTTACAACATAAAACT
GTCTTATTTTTGTGAAAGGATTATTTTGAGACCTTAAAATAATTTATATCTTGATGTTAAAAC
CTCAAAGCAAAAAAAGTGAGGGAGATAGTGAGGGGAGGGCACGCTTGTCTTCTCAGGTATCTT
CCCCAGCATTGCTCCCTTACTTAGTATGCCAAATGTCTTGACCAATATCAAAAACAAGTGCTT
GTTTAGCGGAGAATTTTGAAAAGAGGAATATATAACTCAATTTTCACAACCACATTTACCAAA
AAAAGAGATCAAATATAAAATTCATCATAATGTCTGTTCAACATTATCTTATTTGGAAAATGG
GGAAATTATCACTTACAAGTATTTGTTTACTATGAAATTTTAAATACACATTTATGCCTAGAA
GGAACGGACTTTTTTTTTCTATTTTAATTACACATAATATGTAATTAAAGTACAACATAATAT
GTTGTTTCTCTGTAGCCCGTTGAGCATATGAGTAAGTCACATTTCTATTAGGACTACTTACAA
GGACAAGGTTTCCATTTTTCCAGTTGTAAAATTGGAACCATCAGCTGATAACCTCGTAGGGAG
CAACCCCAGGATAGCTAAGTGTTATGTAATATGCCTAGAAGGTGATGTGAATGCGATTCAGAA
GCATAGCCACTCCCATTTTATGAGCTACTCACATGACAAATGTCATCTTTTGCTATAACCTTT
GCCAAGTTAGAGAAAAGATGGATTTAATGAGATAAATGAAAAGATATTTAACCTAAAAAAAAA
AAAAAAAAAAAAAAAAAA

# FIGURE 240

MALLSRPALTLLLLLMAAVVRCQEQAQTTDWRATLKTIRNGVHKIDTYLNAALDLLGGEDGLC
QYKCSDGSKPFPRYGYKPSPPNGCGSPLFGVHLNIGIPSLTKCCNQHDRCYETCGKSKNDCDE
EFQYCLSKICRDVQKTLGLTQHVQACETTVELLFDSVIHLGCKPYLDSQRAACRCHYEEKTDL

**Important features:**

**Signal peptide:**

amino acids 1-22

**N-myristoylation sites:**

amino acids 57-63,93-99

**Phospholipase A2 histidine active site:**

amino acids 106-114

**Neuraxin and MAP1B proteins repeat proteins Block:**

amino acids 109-137

# FIGURE 241

GATTCCGAGCGCCTCCACTGCTGGTCCGTTGGCCAGATCAACTCGCCGCGTGGGCCGGCCGTT
CCCTGAGAGTCTGAGCGCTCGCCGCACCCCCTTCCGAGCTTCTATTGGCCGTAGCAGACGTCC
GTCTGCCGCTATCTCCGCCCCAATACGGAAGCGGCCTAGTCCTCCGGCTCCGACAGCTGGGTG
TCCAGGCC**ATG**GGGCAGCCCTGGGCGGCTGGGAGCACGGACGGGGCGCCCGCGCAGCTGCCTC
TCGTGCTCACCGCGCTGTGGGCCGCGGCCGTGGGCCTGGAGCTGGCTTACGTGCTGGTGCTCG
GTCCCGGGCCGCCGCCGCTGGGACCCCTGGCCCGGGCCTTGCAGCTGGCGCTGGCCGCCTTCC
AGCTGCTCAACCTGCTGGGCAACGTGGGGCTCTTCCTGCGCTCGGATCCCAGCATCCGTGGCG
TGATGCTGGCCGGCCGCGGTCTGGGCCAGGGCTGGGCTTACTGCTACCAATGCCAAAGCCAGG
TGCCGCCACGCAGCGGACACTGCTCTGCCTGCCGCGTCTGCATCCTGCGTCGGGACCACCACT
GCCGCCTGCTGGGCCGCTGCGTGGGCTTCGGCAACTACCGGCCCTTCCTGTGCCTGCTGCTTC
ATGCCGCCGGCGTCCTGCTCCACGTCTCTGTGCTGCTGGGCCCTGCACTGTCGGCCCTGCTGC
GAGCCCACACGCCCCTCCACATGGCTGCCCTCCTCCTGCTTCCCTGGCTCATGTTGCTCACAG
GCAGAGTGTCTCTGGCACAGTTTGCCTTGGCCTTCGTGACGGACACGTGCGTGGCGGGTGCGC
TGCTGTGCGGGGCTGGGCTGCTCTTCCATGGGATGCTGCTGCTGCGGGGCCAGACCACATGGG
AGTGGGCTCGGGGCCAGCACTCCTATGACCTGGGTCCCTGCCACAACCTGCAGGCAGCCCTGG
GGCCCCGCTGGGCCCTCGTCTGGCTCTGGCCCTTCCTGGCCTCCCCATTGCCTGGGGATGGGA
TCACCTTCCAGACCACAGCAGATGTGGGACACACAGCCTCC**TGA**CTCCAGGAAGAGCCAGAGC
TGTGCAGGGAGGAAGGGGTGAGAGGGGGGCCCCCACACCTAGACTCAGTAAGGAAGTCGGGTT
GGACCTTAACATCTGCATTGGACAACTCCACCCCTTCCTTGGCCTTGCCCCTGCCCGCCTACA
CTCCTACGTGTCCAGGGCTTGGGCCGTGACTTAGGCAGAGGAGTGCAGAGGAGGGTCTGGCAG
GGGCTGCTCAGGCCGCCTAGCTGCCCCTTTGCCAGGTTAATAAAGCACTGACTTGTTAA

# FIGURE 242

MGQPWAAGSTDGAPAQLPLVLTALWAAAVGLELAYVLVLGPGPPPLGPLARALQLALAAFQLL
NLLGNVGLFLRSDPSIRGVMLAGRGLGQGWAYCYQCQSQVPPRSGHCSACRVCILRRDHHCRL
LGRCVGFGNYRPFLCLLLHAAGVLLHVSVLLGPALSALLRAHTPLHMAALLLLPWLMLLTGRV
SLAQFALAFVTDTCVAGALLCGAGLLFHGMLLLRGQTTWEWARGQHSYDLGPCHNLQAALGPR
WALVWLWPFLASPLPGDGITFQTTADVGHTAS

**Important features:**

**Signal peptide:**

amino acids 1-30

**Transmembrane domain:**

amino acids 51-66,143-160,174-191,198-214

**N-myristoylation sites:**

amino acids 2-8,8-14,30-36,81-87,88-94,90-96,206-212

**Leucine zipper pattern:**

amino acids 143-165,150-172,157-179,164-186

# FIGURE 243

CTTGTCTTTGTGTCGGTTGTGATTTTCCTAATCTCTGATTTTCCTTTTCTCTCGGACGCTCTC
CCTCTTCGGACCCATTTTCTCCCGTGCTTCATGCCCTGATAGCCTGGCCCCTTCCCGGCTTCC
TTCGCTACCGGGGACGCCTCTAGTTTTTCTGAATTTCTGGCTGGCTCCACCCTCCGCGTTCAT
CTTCCTCAAGAGTTCGCCCCTCTGGGGGCTCCTCTGTGTAATCGTCGCCTTCTCTGGGTATTT
CTGTGAACTCCGTCTCACACCATCCCGCCATCTTCTCTGCCTTGGCCCCTTTTCTCTGTACAG
CCAGCTCTGTGTCCTTTTCTTCTCCCCCTCTAAAATCGACTCCTCTTCTCCCTGAGAGCCCCA
CCTTTGTGCCCCACTCCTCATTTTCCTACGCCTCCCTCTCTCTGCTGGTCCTCTCTCTCCCTG
CAAGGTTCCATTCCATCAATTTGTTTGTCTTTTGTAGGGGTGGCATCCCCTCTGACTACTGCT
CCATCCTTTTTTTTTTTTTTTTTTTTTTTTTGCTTGAGGATTTCACTTCAATCTTTTCTGGT
TGCGTCTCCACTTGTACTCAGCTTGTTAGGTCCAGGTCCAGTTGTTCTGCATCTGAGGCTGGC
GTGTGCTGTCTTCTCTGATTGGCCTAATCTCCCTCACCCCCGTGAGATCTGTTGTCAGCCTTC
GTTTCTCTTTCCTGTGTCCCAGCTTTTCTGCGGGTCTTGGCACCTTTCTTGGCCACAGATTTC
TGGGTTACAGAGCATGTGTGTCTGAGGCATTGCAGGCAGAAAAGGGTGGCCGACGTGACCTCT
AGCTGGACTGCTGGGCAGGGGAGCTGTCCTAGATAAAATTGGAAAGAAACAGTGACCCAGAGA
CAGGTGGACAAAGAATTCGGGGACTGATGGGAACTGAGCTTGGGATCCAGACTGAAACTGATT
CCAGACTGACCTCTAGCACCCAGGACCCAGACACAGGGCC**ATG**GGACCCCAGCATTTGAGACT
TGTGCAGCTGTTCTGCCTTCTAGGGGCCATCCCCACTCTGCCTCGGGCTGGAGCTCTTTTGTG
CTATGAAGCAACAGCCTCAAGATTCAGAGCTGTTGCTTTCCATAACTGGAAGTGGCTTCTGAT
GAGGAACATGGTGTGTAAGCTGCAAGAGGGCTGCGAGGAGACGCTAGTGTTCATTGAGACAGG
GACTGCAAGGGGAGTTGTGGGCTTTAAAGGCTGCAGCTCGTCTTCGTCTTACCCTGCGCAAAT
CTCCTACCTTGTTTCCCCACCCGGAGTGTCCATTGCCTCCTACAGTCGCGTCTGCCGGTCTTA
TCTCTGCAACAACCTCACCAATTTGGAGCCTTTTGTGAAACTCAAGGCCAGCACTCCTAAGTC
TATCACATCTGCGTCCTGTAGCTGCCCGACCTGTGTGGGCGAGCACATGAAGGATTGCCTCCC
AAATTTTGTCACCACTAATTCTTGCCCCTTGGCTGCTTCTACGTGTTACAGTTCCACCTTAAA
ATTTCAGGCAGGGTTTCTCAATACCACCTTCCTCCTCATGGGGTGTGCTCGTGAACATAACCA
GCTTTTAGCAGATTTTCATCATATTGGGAGCATCAAAGTGACTGAGGTCCTCAACATCTTAGA
GAAGTCTCAGATTGTTGGTGCAGCATCCTCCAGGCAAGATCCTGCTTGGGGTGTCGTCTTAGG
CCTCCTGTTTGCCTTCAGGGAC**TGA**CCATCTAGCTGCACCCGACAAGCACCCAGACTCTTTCA
CATAACAAATAAAATAGCAGAGTTCCCTTAAAAAAAAAAAAAAAAAAAAAAAAAAAAAAAAAA
AAAAAAAAAA

# FIGURE 244

MGPQHLRLVQLFCLLGAIPTLPRAGALLCYEATASRFRAVAFHNWKWLLMRNMVCKLQEGCEE
TLVFIETGTARGVVGFKGCSSSSSYPAQISYLVSPPGVSIASYSRVCRSYLCNNLTNLEPFVK
LKASTPKSITSASCSCPTCVGEHMKDCLPNFVTTNSCPLAASTCYSSTLKFQAGFLNTTFLLM
GCAREHNQLLADFHHIGSIKVTEVLNILEKSQIVGAASSRQDPAWGVVLGLLFAFRD

**Important features:**

**Signal peptide:**

amino acids 1-20

**N-glycosylation sites:**

amino acids 117-121,183-187

**N-myristoylation sites:**

amino acids 16-22,25-31,60-66,71-77,81-87,100-106,224-230,
235-241,239-245

**Prokaryotic membrane lipoprotein lipid attachment site:**

amino acids 181-192

# FIGURE 245

GTGGAGTTGGGTGGTGTCGGGAGCCTCTCCCTGAGGGGCACCGCGTCTTCAGGAGCTGGGCCTCCAGTGCGGCGC
GATGTCAGGCGCGGTGACAGCTCTGTGAGTCCGAGGCCGCGGCCGTGGCGCTGGGCGGCTGCGGGGCCTGACCGG
TCCGCTC**ATG**GTGCCGCCACGACGCCATCGCGGGGCAGGAAGGCCAGGGGTGCTGAGTTCTTCACCTCCTTTTAG
ACTGAGATCTGCCAAGTTTTCCGGCATTGCTCTTGAGGATCTCAGAAGGGCTCTTAAGACAAGACTGCAAATGGT
GTGTGTATTTGTCATGAACCGAATGAATTCCCAGAACAGTGGTTTCACTCAGCGCAGGCGAATGGCTCTTGGGAT
TGTTATTCTTCTGCTTGTTGATGTGATATGGGTTGCTTCCTCTGAACTTACTTCGTATGTTTTTACCCAGTACAA
CAAACCATTCTTCAGCACCTTTGCAAAAACATCTATGTTTGTTTTGTACCTTTTGGGCTTTATTATTTGGAAGCC
ATGGAGACAACAGTGTACAAGAGGACTTCGCGGAAAGCATGCTGCTTTTTTTGCAGATGCTGAAGGTTACTTTGC
TGCTTGCACAACAGATACAACTATGAATAGTTCTTTGAGTGAACCTCTGTATGTGCCTGTGAAATTCCATGATCT
TCCAAGTGAAAAACCTGAGAGCACAAACATTGATACTGAAAAAACCCCCAAAAAGTCTCGTGTGAGGTTCAGTAA
TATCATGGAGATTCGACAGCTTCCGTCAAGTCATGCATTGGAAGCAAAGTTGTCTCGCATGTCATATCCTGTGAA
AGAACAAGAATCCATACTGAAAACTGTGGGGAAACTTACTGCAACTCAAGTAGCGAAAATTAGCTTTTTTTTTG
CTTTGTGTGGTTTTTGGCAAATTTGTCATATCAAGAAGCACTTTCAGACACACAAGTTGCTATAGTTAATATTTT
ATCTTCAACTTCCGGACTTTTTACCTTAATCCTTGCTGCAGTATTTCCAAGTAACAGTGGAGATAGATTTACCCT
TTCTAAACTATTAGCTGTAATTTTAAGCATTGGAGGCGTTGTACTGGTAAACCTGGCAGGGTCTGAAAAACCTGC
TGGAAGAGACACAGTAGGTTCCATTTGGTCTCTTGCTGGAGCCATGCTCTATGCTGTCTATATTGTTATGATTAA
GAGAAAAGTAGATAGAGAAGACAAGTTGGATATTCCAATGTTCTTTGGTTTTGTAGGTTTGTTTAATCTGCTGCT
CTTATGGCCAGGTTTCTTTTTACTTCATTATACTGGATTTGAGGACTTCGAGTTTCCCAATAAAGTAGTATTAAT
GTGCATTATCATTAATGGCCTTATTGGAACAGTACTCTCAGAGTTCCTGTGGTTGTGGGGCTGCTTTCTTACCTC
ATCATTGATAGGCACACTTGCACTAAGCCTTACAATACCTCTGTCCATAATAGCTGACATGTGTATGCAAAAGGT
GCAGTTTTCTTGGTTATTTTTTGCAGGAGCTATCCCTGTATTTTTTTCATTTTTTATTGTAACTCTCCTATGCCA
TTATAATAATTGGGATCCTGTGATGGTGGGAATCAGAAGAATATTTGCTTTTATATGCAGAAAACATCGAATTCA
GAGAGTTCCAGAAGACAGCGAACAGTGTGAGAGTCTCATTTCTATGCACAGTGTTTCTCAGGAGGATGGAGCTAG
T**TAG**CTGTCTGTTGTCTGTAGCCCAGCTTGATAATGGAACTATACAGCGAAGAGACAATCTCTGGCAAGTTTTTG
TAGAAAAAAATGTTTCAGTGCCTAGTCTGAAAAATAACAGTTTGAGTTCTTTGAAACTCTAAAATATATTTTTCTC
ATACCTGTTTTCTTCATTTTCATAATGAAGCACTTTGCTATGTAGCTGTGTACATATCACTACAGTTATAGGAAG
TTTCAGTCTACAGTCCATCCAAAGGACCAACCTGCCTTACACATCTCAAGGAATTCAGCTGTTGAAATCATTTGA
ACTAATCAAGGAATAAATCCTAATGTTCTGGGACTTTATTTTCACATGTTAAATGCTGGAATATATTATGAAAAT
GTTTTCAAGAAATCACTTAAGTGTTCATAGACCAGTATTTCTGACAGGTAAAATGCTAAAATAAGCTACCTGTAA
TAAGTGTGGATTATATTTTTGGGTTTTGTAGAATATTGCAAATTAACCACACAAAAAAATGTTTAATTTATGCAAC
AAGCATGTTTGTGCAAATTTCATGGGACTTTAAAAAGAATAAGTATTTGAGAAAATATCTGGTTCACTTACACTA
CATTTACTGTATTATTCTTTTATAGCATTAGGTGCCTTGTATTTTAAATCTGTGACAAACCATGGCAAATTTTTA
AAGGGGAAGTATTATTATAAAATGAAGAAATATGTATTTCTAAAGGCTATATTGCTGTAAACTTAATTGATAAAG
CTCTGTTTAATTTAGAGTTTTGAAGAAATAGTCTCCCTTCAATTAAGAAATTTTCATAATGGAATGATTTAAATT
GAAGTGACAAAGAGTATTATTAAAATACAATGTTTATAAAAAAA

# FIGURE 246

MVPPRRHRGAGRPGVLSSSPPFRLRSAKFSGIALEDLRRALKTRLQMVCVFVMNRMNSQNSGF
TQRRRMALGIVILLLVDVIWVASSELTSYVFTQYNKPFFSTFAKTSMFVLYLLGFIIWKPWRQ
QCTRGLRGKHAAFFADAEGYFAACTTDTTMNSSLSEPLYVPVKFHDLPSEKPESTNIDTEKTP
KKSRVRFSNIMEIRQLPSSHALEAKLSRMSYPVKEQESILKTVGKLTATQVAKISFFFCFVWF
LANLSYQEALSDTQVAIVNILSSTSGLFTLILAAVFPSNSGDRFTLSKLLAVILSIGGVVLVN
LAGSEKPAGRDTVGSIWSLAGAMLYAVYIVMIKRKVDREDKLDIPMFFGFVGLFNLLLLWPGF
FLLHYTGFEDFEFPNKVVLMCIIINGLIGTVLSEFLWLWGCFLTSSLIGTLALSLTIPLSIIA
DMCMQKVQFSWLFFAGAIPVFFSFFIVTLLCHYNNWDPVMVGIRRIFAFICRKHRIQRVPEDS
EQCESLISMHSVSQEDGAS

**Important features:**

**Transmembrane domain:**

amino acids 69-87,105-118,237-256,266-285,300-316,332-346,
364-379,399-419,453-472

**N-glycosylation sites:**

amino acids 157-161,255-259

**N-myristoylation sites:**

amino acids 14-20,329-335,404-410,407-413,418-424

# FIGURE 247

CGTCTGTAGAGATATCATGAACTTCAACTTAGCTTTGGTACTTTCTTCCCTGAAGACAGAGGG
CAGAACTCTGAGTTCCAGAACCATTTTCAACTGTATTGGGGACCAATCACTTGACTCTATTCT
TGTCTCTCTGACAGATGACGCTACACTCTCCTCTGAATAATGGACACCATTTCTAAAACTGAA
TCCTGCTACTAAAATAATTCAGATGATATATTTTTCCAATTCTACAATCTTGCTTTGTTTTAT
TTAGTTGTTTTCTCTCTCTCTTCCCAGTTTTCCAGAGACTGGAGCTAAACTGGGCTTTCAACA
TCATC**ATG**AAGTTTATCCTCCTCTGGGCCCTCTTGAATCTGACTGTTGCTTTGGCCTTTAATC
CAGATTACACAGTCAGCTCCACTCCCCCTTACTTGGTCTATTTGAAATCTGACTACTTGCCCT
GCGCTGGAGTCCTGATCCACCCGCTTTGGGTGATCACAGCTGCACACTGCAATTTACCAAAGC
TTCGGGTGATATTGGGGGTTACAATCCCAGCAGACTCTAATGAAAAGCATCTGCAAGTGATTG
GCTATGAGAAGATGATTCATCATCCACACTTCTCAGTCACTTCTATTGATCATGACATCATGC
TAATCAAGCTGAAAACAGAGGCTGAACTCAATGACTATGTGAAATTAGCCAACCTGCCCTACC
AAACTATCTCTGAAAATACCATGTGCTCTGTCTCTACCTGGAGCTACAATGTGTGTGATATCT
ACAAAGAGCCCGATTCACTGCAAACTGTGAACATCTCTGTAATCTCCAAGCCTCAGTGTCGCG
ATGCCTATAAAACCTACAACATCACGGAAAATATGCTGTGTGTGGGCATTGTGCCAGGAAGGA
GGCAGCCCTGCAAGGAAGTTTCTGCTGCCCCGGCAATCTGCAATGGGATGCTTCAAGGAATCC
TGTCTTTTGCGGATGGATGTGTTTTGAGAGCCGATGTTGGCATCTATGCCAAAATTTTTTACT
ATATACCCTGGATTGAAAATGTAATCCAAAATAAC**TGA**GCTGTGGCAGTTGTGGACCATATGA
CACAGCTTGTCCCCATCGTTCACCTTTAGAATTAAATATAAATTAACTCCTC

# FIGURE 248

MKFILLWALLNLTVALAFNPDYTVSSTPPYLVYLKSDYLPCAGVLIHPLWVITAAHCNLPKLR
VILGVTIPADSNEKHLQVIGYEKMIHHPHFSVTSIDHDIMLIKLKTEAELNDYVKLANLPYQT
ISENTMCSVSTWSYNVCDIYKEPDSLQTVNISVISKPQCRDAYKTYNITENMLCVGIVPGRRQ
PCKEVSAAPAICNGMLQGILSFADGCVLRADVGIYAKIFYYIPWIENVIQNN

**Important features:**

**Signal peptide:**

amino acids 1-17

**N-glycosylation sites:**

amino acids 11-15,156-160,173-177

**Tyrosine kinase phosphorylation site:**

amino acids 108-117

N-myristoylation sites:

amino acids 182-188,203-209

**Amidation site:**

amino acids 185-189

**Serine proteases, trypsin family, histidine active site:**

amino acids 52-58

# FIGURE 249

GCGAGGCGGCCGCTGTCTTCTGCTGCGGCTTCCGCGACCACAAGTACTGCTGCGACGACCCGC
ACAGCTTCTTCCCCTACGAGCACAGCTAC**ATG**TGGTGGCTCAGCATTGGCGCTCTCATAGGCC
TGTCCGTAGCAGCAGTGGTTCTTCTCGCCTTCATTGTTACCGCCTGTGTGCTCTGCTACCTGT
TCATCAGCTCTAAGCCCCACACAAAGTTGGACCTGGGCTTGAGCTTACAGACAGCAGGCCCTG
AGGAGGTTTCTCCTGACTGCCAAGGTGTGAACACAGGCATGGCGGCAGAAGTGCCAAAAGTGA
GCCCTCTCCAGCAGAGTTACTCCTGCTTGAACCCGCAGCTGGAGAGCAATGAGGGGCAGGCTG
TGAACTCCAAACGCCTCCTCCATCATTGCTTCATGGCCACAGTGACCACCAGTGACATTCCAG
GCAGCCCTGAGGAAGCCTCTGTACCCAACCCTGACCTATGTGGACCAGTCCCA**TAA**ACATTCA
ATAAATGTCTCCATACCATCAA

# FIGURE 250

MWWLSIGALIGLSVAAVVLLAFIVTACVLCYLFISSKPHTKLDLGLSLQTAGPEEVSPDCQGV
NTGMAAEVPKVSPLQQSYSCLNPQLESNEGQAVNSKRLLHHCFMATVTTSDIPGSPEEASVPN
PDLCGPVP

**Important features:**

**Signal peptide:**

Amino acids 1-26

**N-myristoylation sites:**

Amino acids 7-13,11-17,62-68,93-99

# FIGURE 251

GTGGTTTGGATTGAGCCGGGCCCGGCCGGGGCGCCGAGTCGGAGGGGGTGGCAGTGAGCGGCG
GCAGAGGCTACGGGGCTCGGTTTGGCTGACTGGGGAGTCGGCAGGCGGCAGGAACC**ATG**CGAG
GCCAGCGGAGCCTGCTGCTGGGCCCGGCCCGCCTCTGCCTCCGCCTCCTTCTGCTGCTGGGTT
ACAGGCGCCGCTGTCCACCTCTACTCCGGGGTCTAGTACAGCGCTGGCGCTACGGCAAGGTCT
GCCTGCGCTCCCTGCTCTACAACTCCTTTGGGGGCAGTGACACCGCTGTTGATGCTGCCTTTG
AGCCTGTCTACTGGCTGGTAGACAACGTGATCCGCTGGTTTGGAGTGGTGTTCGTGGTCCTGG
TGATCGTGCTGACAGGCTCCATTGTAGCTATCGCCTACCTGTGTGTCCTGCCTCTCATCCTCC
GAACCTACTCAGTGCCACGACTCTGCTGGCATTTCTTCTATAGCCACTGGAATCTGATCCTGA
TTGTCTTCCACTACTACCAGGCCATCACCACTCCGCCTGGGTACCCACCCCAGGGCAGGAATG
ATATCGCCACCGTCTCCATCTGTAAGAAGTGCATTTACCCCAAGCCAGCCCGAACACACCACT
GCAGCATCTGCAACAGGTGTGTGCTGAAGATGGATCACCACTGCCCCTGGCTAAACAATTGTG
TGGGCCACTATAACCATCGGTACTTCTTCTCTTTCTGCTTTTTCATGACTCTGGGCTGTGTCT
ACTGCAGCTATGGAAGTTGGGACCTTTTCCGGGAGGCTTATGCTGCCATTGAGACTTATCACC
AGACCCCACCACCCACCTTCTCCTTTCGAGAAAGGATGACTCACAAGAGTCTTGTCTACCTCT
GGTTCCTGTGCAGTTCTGTGGCACTTGCCCTGGGTGCCCTAACTGTATGGCATGCTGTTCTCA
TCAGTCGAGGTGAGACTAGCATCGAAAGGCACATCAACAAGAAGGAGAGACGTCGGCTACAGG
CCAAGGGCAGAGTATTTAGGAATCCTTACAACTACGGCTGCTTGGACAACTGGAAGGTATTCC
TGGGTGTGGATACAGGAAGGCACTGGCTTACTCGGGTGCTCTTACCTTCTAGTCACTTGCCCC
ATGGGAATGGAATGAGCTGGGAGCCCCCTCCCTGGGTGACTGCTCACTCAGCCTCTGTGATGG
CAGTG**TGA**GCTGGACTGTGTCAGCCACGACTCGAGCACTCATTCTGCTCCCTATGTTATTTCA
AGGGCCTCCAAGGGCAGCTTTTCTCAGAATCCTTGATCAAAAAGAGCCAGTGGGCCTGCCTTA
GGGTACCATGCAGGACAATTCAAGGACCAGCCTTTTTACCACTGCAGAAGAAAGACACAATGT
GGAGAAATCTTAGGACTGACATCCCTTTACTCAGGCAAACAGAAGTTCCAACCCCAGACTAGG
GGTCAGGCAGCTAGCTACCTACCTTGCCCAGTGCTGACCCGGACCTCCTCCAGGATACAGCAC
TGGAGTTGGCCACCACCTCTTCTACTTGCTGTCTGAAAAAACACCTGACTAGTACAGCTGAGA
TCTTGGCTTCTCAACAGGGCAAAGATACCAGGCCTGCTGCTGAGGTCACTGCCACTTCTCACA
TGCTGCTTAAGGGAGCACAAATAAAGGTATTCGATTTTTAAAAAAAAAAAAAAAAAAAAAAAA
AAAAAAAAAAAAA

# FIGURE 252

MRGQRSLLLGPARLCLRLLLLLGYRRRCPPLLRGLVQRWRYGKVCLRSLLYNSFGGSDTAVDA
AFEPVYWLVDNVIRWFGVVFVVLVIVLTGSIVAIAYLCVLPLILRTYSVPRLCWHFFYSHWNL
ILIVFHYYQAITTPPGYPPQGRNDIATVSICKKCIYPKPARTHHCSICNRCVLKMDHHCPWLN
NCVGHYNHRYFFSFCFFMTLGCVYCSYGSWDLFREAYAAIETYHQTPPPTFSFRERMTHKSLV
YLWFLCSSVALALGALTVWHAVLISRGETSIERHINKKERRRLQAKGRVFRNPYNYGCLDNWK
VFLGVDTGRHWLTRVLLPSSHLPHGNGMSWEPPPWVTAHSASVMAV

**Important features:**

**Transmembrane domain:**

amino acids 88-100,202-216,254-274

**N-myristoylation sites:**

amino acids 55-61,56-62,92-98,210-216,309-315,319-325,340-346

**Prokaryotic membrane lipoprotein lipid attachment site:**

amino acids 201-212

# FIGURE 253

GATCAAGCGCCTTCCTTTCCCTTCCTCTCCCTACTTGGCCTTTGCCCTAAGCCAAGACCTGGCCATCAGCCTGGC
TGCAGGGGCCTGCAGAGCCAGCTGCACTTTTTCAGGTATGGGGGAGGGCCAGGCACC**ATG**AAGCCAGTGTGGGTC
GCCACCCTTCTGTGGATGCTACTGCTGGTGCCCAGGCTGGGGGCCGCCCGGAAGGGGTCCCCAGAAGAGGCCTCC
TTCTACTATGGAACCTTCCCTCTTGGCTTCTCCTGGGGCGTGGGCAGTTCTGCCTACCAGACGGAGGGCGCCTGG
GACCAGGACGGGAAAGGGCCTAGCATCTGGGACGTCTTCACACACAGTGGGAAGGGGAAAGTGCTTGGGAATGAG
ACGGCAGATGTAGCCTGTGACGGCTACTACAAGGTCCAGGAGGACATCATTCTGCTGAGGGAACTGCACGTCAAC
CACTACCGATTCTCCCTGTCTTGGCCCCGGCTCCTGCCCACAGGCATCCGAGCCGAGCAGGTGAACAAGAAGGGA
ATCGAATTCTACAGTGATCTTATCGATGCCCTTCTGAGCAGCAACATCACTCCCATCGTGACCTTGCACCACTGG
GATCTGCCACAGCTGCTCCAGGTCAAATACGGTGGGTGGCAGAATGTGAGCATGGCCAACTACTTCAGAGACTAC
GCCAACCTGTGCTTTGAGGCCTTTGGGGACCGTGTGAAGCACTGGATCACGTTCAGTGATCCTCGGGCAATGGCA
GAAAAAGGCTATGAGACGGGCCACCATGCGCCGGGCCTGAAGCTCCGCGGCACCGGCCTGTACAAGGCAGCACAC
CACATCATTAAGGCCCACGCCAAAACCTGGCATTCTTATAACACCACGTGGCGCAGCAAGCAGCAAGGTCTGGTG
GGAATTTCACTGAACTGTGACTGGGGGGAACCTGTGGACATTAGTAACCCCAAGGACCTAGAGGCTGCCGAGAGA
TACCTACAGTTCTGTCTGGGCTGGTTTGCCAACCCCATTTATGCCGGTGACTACCCCCAAGTCATGAAGGACTAC
ATTGGAAGAAAGAGTGCAGAGCAAGGCCTGGAGATGTCGAGGTTACCGGTGTTCTCACTCCAGGAGAAGAGCTAC
ATTAAAGGCACATCCGATTTCTTGGGATTAGGTCATTTTACTACTCGGTACATCACGGAAAGGAACTACCCCTCC
CGCCAGGGGCCCAGCTACCAGAACGATCGTGACTTGATAGAGCTGGTTGACCCAAACTGGCCAGATCTGGGGTCT
AAATGGCTATATTCTGTGCCATGGGGATTTAGGAGGCTCCTTAACTTTGCTCAGACTCAATACGGTGATCCTCCC
ATATATGTGATGGAAAATGGAGCATCTCAAAAATTCCACTGTACTCAATTATGTGATGAGTGGAGAATTCAATAC
CTTAAAGGATACATAAATGAAATGCTAAAAGCTATAAAAGATGGTGCTAATATAAAGGGGTATACTTCCTGGTCT
CTGTTGGATAAGTTTGAATGGGAGAAAGGATACTCAGATAGATATGGATTCTACTATGTTGAATTTAACGACAGA
AATAAGCCTCGCTATCCAAAGGCTTCAGTTCAATATTACAAGAAGATTATCATTGCCAATGGGTTTCCCAATCCA
AGAGAGGTGGAAAGTTGGTACCTCAAAGCTTTGGAAACTTGCTCTATCAACAATCAGATGCTTGCTGCAGAGCCT
TTGCTAAGTCACATGCAAATGGTTACGGAGATCGTGGTACCCACTGTCTGCTCCCTCTGTGTCCTCATCACTGCT
GTTCTACTAATGCTCCTCCTGAGGAGGCAGAGC**TGA**GACAGGATTATCAATTTTGGAGCTTCATAAGAGAATCTT
CAGGATCTTCCTCCCTTTTCTGCTTTGAGGGTTTCCATACATTGCTGTTTTCAGGTTCTACAATAATTACCTTTT
TTTCTCTTTCTCTTTTTGGCTTGTGCTGGGATTTAAGAATTAGAAAATAAAAATAAGCAGAAATTA

# FIGURE 254

MKPVWVATLLWMLLLVPRLGAARKGSPEEASFYYGTFPLGFSWGVGSSAYQTEGAWDQDGKGPSIWDVFTHSGKG
KVLGNETADVACDGYYKVQEDIILLRELHVNHYRFSLSWPRLLPTGIRAEQVNKKGIEFYSDLIDALLSSNITPI
VTLHHWDLPQLLQVKYGGWQNVSMANYFRDYANLCFEAFGDRVKHWITFSDPRAMAEKGYETGHHAPGLKLRGTG
LYKAAHHIIKAHAKTWHSYNTTWRSKQQGLVGISLNCDWGEPVDISNPKDLEAAERYLQFCLGWFANPIYAGDYP
QVMKDYIGRKSAEQGLEMSRLPVFSLQEKSYIKGTSDFLGLGHFTTRYITERNYPSRQGPSYQNDRDLIELVDPN
WPDLGSKWLYSVPWGFRRLLNFAQTQYGDPPIYVMENGASQKFHCTQLCDEWRIQYLKGYINEMLKAIKDGANIK
GYTSWSLLDKFEWEKGYSDRYGFYYVEFNDRNKPRYPKASVQYYKKIIIANGFPNPREVESWYLKALETCSINNQ
MLAAEPLLSHMQMVTEIVVPTVCSLCVLITAVLLMLLLRRQS

**Important features:**
**Signal peptide:**
amino acids 1-21

**Transmembrane domain:**
amino acids 541-558

**N-glycosylation sites:**
amino acids 80-84,171-175,245-249

**Glycosaminoglycan attachment site:**
amino acids 72-76

**cAMP- and cGMP-dependent protein kinase phosphorylation sites:**
amino acids 23-27,564-568

**Tyrosine kinase phosphorylation sites:**
amino acids 203-211,347-355,460-468,507-514

**N-myristoylation sites:**
amino acids 44-50,79-85,167-173,225-231,257-263,315-321

**Amidation site:**
amino acids 307-311

**Glycosyl hydrolases family 1 active site:**
amino acids 407-416

**Glycosyl hydrolases family 1 N-terminal signature:**
amino acids 41-56

**Motif name Glycosyl hydrolases family:**
amino acids 37- 67

# FIGURE 255

CGCGAAG**ATG**CGAAAGGTGGTTTTGATCACCGGGGCTAGCAGTGGCATTGGCCTGGCCCTCTG
CAAGCGGCTGCTGGCGGAAGATGATGAGCTTCATCTGTGTTTGGCGTGCAGGAACATGAGCAA
GGCAGAAGCTGTCTGTGCTGCTCTGCTGGCCTCTCACCCCACTGCTGAGGTCACCATTGTCCA
GGTGGATGTCAGCAACCTGCAGTCGGTCTTCCGGGCCTCCAAGGAACTTAAGCAAAGGTTTCA
GAGATTAGACTGTATATATCTAAATGCTGGGATCATGCCTAATCCACAACTAAATATCAAAGC
ACTTTTCTTTGGCCTCTTTTCAAGAAAAGTGATTCATATGTTCTCCACAGCTGAAGGCCTGCT
GACCCAGGGTGATAAGATCACTGCTGATGGACTTCAGGAGGTGTTTGAGACCAATGTCTTTGG
CCATTTTATCCTGATTCGGGAACTGGAGCCTCTCCTCTGTCACAGTGACAATCCATCTCAGCT
CATCTGGACATCATCTCGCAGTGCAAGGAAATCTAATTTCAGCCTCGAGGACTTCCAGCACAG
CAAAGGCAAGGAACCCTACAGCTCTTCCAAATATGCCACTGACCTTTTGAGTGTGGCTTTGAA
CAGGAACTTCAACCAGCAGGGTCTCTATTCCAATGTGGCCTGTCCAGGTACAGCATTGACCAA
TTTGACATATGGAATTCTGCCTCCGTTTATATGGACGCTGTTGATGCCGGCAATATTGCTACT
TCGCTTTTTTGCAAATGCATTCACTTTGACACCATATAATGGAACAGAAGCTCTGGTATGGCT
TTTCCACCAAAAGCCTGAATCTCTCAATCCTCTGATCAAATATCTGAGTGCCACCACTGGCTT
TGGAAGAAATTATATTATGACCCAGAAGATGGACCTAGATGAAGACACTGCTGAAAAATTTTA
TCAAAAGTTACTGGAACTGGAAAAGCACATTAGGGTCACTATTCAAAAAACAGATAATCAGGC
CAGGCTCAGTGGCTCATGCCTA**TAA**TTCCAGCACTTTGGGAGGCCAAGGCAGAAGGATCACTT
GAGACCAGGAGTTCAAGACCAGCCTGAGAAACATAGTGAGCCCTTGTCTCTACAAAAAGAAAT
AAAAATAATAGCTGGGTGTGGTGGCATGCGCATGTAGTCCCAGCTACTCAGAAGGATGAGGTG
GGAGGATCTCTTGAGGCTGGGAGGCAGAGGTTGCAGTGAGCTGAGATTGTGCCACTGCACTCC
AGCCTGGGTGACAGCGAGACCCTGTCTCAAAATATGTATATATTTAATATATATATATAAAACCA
GAGCTGACAATGACACTCTGGAACATTGCATACCTTCTGTACATTCTGGGGTACATGGATTTC
TACTGAGTTGGATAATATGCATTTGTAATAAACTATGAACTATGAA

# FIGURE 256

MRKVVLITGASSGIGLALCKRLLAEDDELHLCLACRNMSKAEAVCAALLASHPTAEVTIVQVD
VSNLQSVFRASKELKQRFQRLDCIYLNAGIMPNPQLNIKALFFGLFSRKVIHMFSTAEGLLTQ
GDKITADGLQEVFETNVFGHFILIRELEPLLCHSDNPSQLIWTSSRSARKSNFSLEDFQHSKG
KEPYSSSKYATDLLSVALNRNFNQQGLYSNVACPGTALTNLTYGILPPFIWTLLMPAILLLRF
FANAFTLTPYNGTEALVWLFHQKPESLNPLIKYLSATTGFGRNYIMTQKMDLDEDTAEKFYQK
LLELEKHIRVTIQKTDNQARLSGSCL

**Important features:**

**Transmembrane domain:**

amino acids 234-254

**N-glycosylation sites:**

amino acids 37-41,178-182,229-233,263-267

**Glycosaminoglycan attachment site:**

amino acids 12-16

**N-myristoylation sites:**

amino acids 9-15,13-19,15-21,215-221,224-230

# FIGURE 257

CGGACGCGTGGGGCCGT**ATG**CGCGGCTCTGTGGAGTGCACCTGGGGTTGGGGGCACTGTGCCC
CCAGCCCCCTGCTCCTTTGGACTCTACTTCTGTTTGCAGCCCCATTTGGCCTGCTGGGGGAGA
AGACCCGCCAGGTGTCTCTGGAGGTCATCCCTAACTGGCTGGGCCCCCTGCAGAACCTGCTTC
ATATACGGGCAGTGGGCACCAATTCCACACTGCACTATGTGTGGAGCAGCCTGGGGCCTCTGG
CAGTGGTAATGGTGGCCACCAACACCCCCCACAGCACCCTGAGCATCAACTGGAGCCTCCTGC
TATCCCCTGAGCCCGATGGGGGCCTGATGGTGCTCCCTAAGGACAGCATTCAGTTTTCTTCTG
CCCTTGTTTTTACCAGGCTGCTTGAGTTTGACAGCACCAACGTGTCCGATACGGCAGCAAAGC
CTTTGGGAAGACCATATCCTCCATACTCCTTGGCCGATTTCTCTTGGAACAACATCACTGATT
CATTGGATCCTGCCACCCTGAGTGCCACATTTCAAGGCCACCCCATGAACGACCCTACCAGGA
CTTTTGCCAATGGCAGCCTGGCCTTCAGGGTCCAGGCCTTTTCCAGGTCCAGCCGACCAGCCC
AACCCCCTCGCCTCCTGCACACAGCAGACACCTGTCAGCTAGAGGTGGCCCTGATTGGAGCCT
CTCCCCGGGGAAACCGTTCCCTGTTTGGGCTGGAGGTAGCCACATTGGGCCAGGGCCCTGACT
GCCCCTCAATGCAGGAGCAGCACTCCATCGACGATGAATATGCACCGGCCGTCTTCCAGTTGG
ACCAGCTACTGTGGGGCTCCCTCCCATCAGGCTTTGCACAGTGGCGACCAGTGGCTTACTCCC
AGAAGCCGGGGGGCCGAGAATCAGCCCTGCCCTGCCAAGCTTCCCCTCTTCATCCTGCCTTAG
CATACTCTCTTCCCCAGTCACCCATTGTCCGAGCCTTCTTTGGGTCCCAGAATAACTTCTGTG
CCTTCAATCTGACGTTCGGGGCTTCCACAGGCCCTGGCTATTGGGACCAACACTACCTCAGCT
GGTCGATGCTCCTGGGTGTGGGCTTCCCTCCAGTGGACGGCTTGTCCCCACTAGTCCTGGGCA
TCATGGCAGTGGCCCTGGGTGCCCCAGGGCTCATGCTGCTAGGGGGCGGCTTGGTTCTGCTGC
TGCACCACAAGAAGTACTCAGAGTACCAGTCCATAAAT**TAA**GGCCCGCTCTCTGGAGGGAAGG
ACATTACTGAACCTGTCTTGCTGTGCCTCGAAACTCTGGAGGTTGGAGCATCAAGTTCCAGCC
GGCCCCTTCACTCCCCCATCTTGCTTTTCTGTGGAACCTCAGAGGCCAGCCTCGACTTCCTGG
AGACCCCCAGGTGGGGCTTCCTTCATACTTTGTTGGGGGACTTTGGAGGCGGGCAGGGGACAG
GGCTATTGATAAGGTCCCCTTGGTGTTGCCTTCTTGCATCTCCACACATTTCCCTTGGATGGG
ACTTGCAGGCCTAAATGAGAGGCATTCTGACTGGTTGGCTGCCCTGGAAGGCAAGAAAATAGA
TTTATTTTTTTCACAGGGAAAAAAAAAAAA

# FIGURE 258

MRGSVECTWGWGHCAPSPLLLWTLLLFAAPFGLLGEKTRQVSLEVIPNWLGPLQNLLHIRAVG
TNSTLHYVWSSLGPLAVVMVATNTPHSTLSINWSLLLSPEPDGGLMVLPKDSIQFSSALVFTR
LLEFDSTNVSDTAAKPLGRPYPPYSLADFSWNNITDSLDPATLSATFQGHPMNDPTRTFANGS
LAFRVQAFSRSSRPAQPPRLLHTADTCQLEVALIGASPRGNRSLFGLEVATLGQGPDCPSMQE
QHSIDDEYAPAVFQLDQLLWGSLPSGFAQWRPVAYSQKPGGRESALPCQASPLHPALAYSLPQ
SPIVRAFFGSQNNFCAFNLTFGASTGPGYWDQHYLSWSMLLGVGFPPVDGLSPLVLGIMAVAL
GAPGLMLLGGGLVLLLHHKKYSEYQSIN

**Important features:**

**Signal peptide:**

amino acids 1-35

**Transmembrane domain:**

amino acids 365-386

**N-glycosylation sites:**

amino acids 65-69,95-99,134-138,159-163,187-191,230-234,333-337

**cAMP- and cGMP-dependent protein kinase phosphorylation site:**

amino acids 397-401

**N-myristoylation sites:**

amino acids 3-9,63-69,235-241,273-279,292-298,324-330

**Leucine zipper pattern:**

amino acids 371-393

# FIGURE 259

CAGGCGGGCCCCCGCGCGGCAGGGCCCTGGACCCGCGCGGCTCCCGGGG**ATG**GTGAGCAAGGCGCTGCTGCGCCT
CGTGTCTGCCGTCAACCGCAGGAGGATGAAGCTGCTGCTGGGCATCGCCTTGCTGGCCTACGTCGCCTCTGTTTG
GGGCAACTTCGTTAATATGAGGTCTATCCAGGAAAATGGTGAACTAAAAATTGAAAGCAAGATTGAAGAGATGGT
TGAACCACTAAGAGAGAAAATCAGAGATTTAGAAAAAAGCTTTACCCAGAAATACCCACCAGTAAAGTTTTTATC
AGAAAAGGATCGGAAAAGAATTTTGATAACAGGAGGCGCAGGGTTCGTGGGCTCCCATCTAACTGACAAACTCAT
GATGGACGGCCACGAGGTGACCGTGGTGGACAATTTCTTCACGGGCAGGAAGAGAAACGTGGAGCACTGGATCGG
ACATGAGAACTTCGAGTTGATTAACCACGACGTGGTGGAGCCCCTCTACATCGAGGTTGACCAGATATACCATCT
GGCATCTCCAGCCTCCCCTCCAAACTACATGTATAATCCTATCAAGACATTAAAGACCAATACGATTGGGACATT
AAACATGTTGGGGCTGGCAAAACGAGTCGGTGCCCGTCTGCTCCTGGCCTCCACATCGGAGGTGTATGGAGATCC
TGAAGTCCACCCTCAAAGTGAGGATTACTGGGGCCACGTGAATCCAATAGGACCTCGGGCCTGCTACGATGAAGG
CAAACGTGTTGCAGAGACCATGTGCTATGCCTACATGAAGCAGGAAGGCGTGGAAGTGCGAGTGGCCAGAATCTT
CAACACCTTTGGGCCACGCATGCACATGAACGATGGGCGAGTAGTCAGCAACTTCATCCTGCAGGCGCTCCAGGG
GGAGCCACTCACGGTATACGGATCCGGGTCTCAGACAAGGGCGTTCCAGTACGTCAGCGATCTAGTGAATGGCCT
CGTGGCTCTCATGAACAGCAACGTCAGCAGCCCGGTCAACCTGGGGAACCCAGAAGAACACACAATCCTAGAATT
TGCTCAGTTAATTAAAAACCTTGTTGGTAGCGGAAGTGAAATTCAGTTTCTCTCCGAAGCCCAGGATGACCCACA
GAAAAGAAAACCAGACATCAAAAAAGCAAAGCTGATGCTGGGGTGGGAGCCCGTGGTCCCGCTGGAGGAAGGTTT
AAACAAAGCAATTCACTACTTCCGTAAAGAACTCGAGTACCAGGCAAATAATCAGTACATCCCCAAACCAAAGCC
TGCCAGAATAAAGAAAGGACGGACTCGCCACAGC**TGA**ACTCCTCACTTTTAGGACACAAGACTACCATTGTACAC
TTGATGGGATGTATTTTTGGCTTTTTTTTGTTGTCGTTTAAAGAAAGACTTTAACAGGTGTCATGAAGAACAAAC
TGGAATTTCATTCTGAAGCTTGCTTTAATGAAATGGATGTGCCTAAAAGCTCCCCTCAAAAAACTGCAGATTTTG
CCTTGCACTTTTTGAATCTCTCTTTTTATGTAAAATAGCGTAGATGCATCTCTGCGTATTTTCAAGTTTTTTTAT
CTTGCTGTGAGAGCATATGTTGTGACTGTCGTTGACAGTTTTATTTACTGGTTTCTTTGTGAAGCTGAAAAGGAA
CATTAAGCGGGACAAAAAATGCCGATTTTATTTATAAAAGTGGGTACTTAATAAATGAGTCGTTATACTATGCAT
AAAGAAAAATCCTAGCAGTATTGTCAGGTGGTGGTGCGCCGGCATTGATTTTAGGGCAGATAAAAGAATTCTGTG
TGAGAGCTTTATGTTTCTCTTTTAATTCAGAGTTTTTCCAAGGTCTACTTTTGAGTTGCAAACTTGACTTTGAAA
TATTCCTGTTGGTCATGATCAAGGATATTTGAAATCACTACTGTGTTTTGCTGCGTATCTGGGGCGGGGGCAGGT
TGGGGGGCACAAAGTTAACATATTCTTGGTTAACCATGGTTAAATATGCTATTTTAATAAAATATTGAAACTCA

# FIGURE 260

MVSKALLRLVSAVNRRRMKLLLGIALLAYVASVWGNFVNMRSIQENGELKIESKIEEMVEPLR
EKIRDLEKSFTQKYPPVKFLSEKDRKRILITGGAGFVGSHLTDKLMMDGHEVTVVDNFFTGRK
RNVEHWIGHENFELINHDVVEPLYIEVDQIYHLASPASPPNYMYNPIKTLKTNTIGTLNMLGL
AKRVGARLLLASTSEVYGDPEVHPQSEDYWGHVNPIGPRACYDEGKRVAETMCYAYMKQEGVE
VRVARIFNTFGPRMHMNDGRVVSNFILQALQGEPLTVYGSGSQTRAFQYVSDLVNGLVALMNS
NVSSPVNLGNPEEHTILEFAQLIKNLVGSGSEIQFLSEAQDDPQKRKPDIKKAKLMLGWEPVV
PLEEGLNKAIHYFRKELEYQANNQYIPKPKPARIKKGRTRHS

**Important features:**

**Signal peptide:**

amino acids 1-32

**N-glycosylation site:**

amino acids 316-320

**Tyrosine kinase phosphorylation site:**

amino acids 235-244

**N-myristoylation sites:**

amino acids 35-41,101-107,383-389

**Amidation sites:**

amino acids 123-127,233-237

# FIGURE 261

GCGTGGTGCGGGGGCGTGGGGAAATCGGGTTGCCCCAGCCGTTACTGGTCCGCGCAGTCAGGG
CATCCTCCGCATCCTCCACATCCTTCC**ATG**GCTCTGAAGAATAAATTCAGTTGTTTATGGATC
TTGGGTCTGTGTTTGGTAGCCACTACATCTTCCAAAATCCCATCCATCACTGACCCACACTTT
ATAGACAACTGCATAGAAGCCCACAACGAATGGCGTGGCAAAGTCAACCCTCCCGCGGCCGAC
ATGAAATACATGATTTGGGATAAAGGTTTAGCAAAGATGGCTAAAGCATGGGCAAACCAGTGC
AAATTTGAACATAATGACTGTTTGGATAAATCATATAAATGCTATGCAGCTTTTGAATATGTT
GGAGAAAATATCTGGTTAGGTGGAATAAAGTCATTCACACCAAGACATGCCATTACGGCTTGG
TATAATGAAACCCAATTTTATGATTTTGATAGTCTATCATGCTCCAGAGTCTGTGGCCATTAT
ACACAGTTAGTTTGGGCCAATTCATTTTATGTCGGTTGTGCAGTTGCAATGTGTCCTAACCTT
GGGGGAGCTTCAACTGCAATATTTGTATGCAACTACGGACCTGCAGGAAATTTTGCAAATATG
CCTCCTTACGCAAGAGGAGAATCTTGCTCTCTCTGCTCAAAAGAAGAGAAATGTGTAAAGAAC
CTCTGCAGGACTCCACAACTTATTATACCTAACCAAAATCCATTTCTGAAGCCAACGGGGAGA
GCACCTCAGCAGACAGCCTTTAATCCATTCAGCTTAGGTTTTCTTCTTCTGAGAATCTTT**TAA**
TGTCATTTATATACAAAAGAAATTCTCAAATGTTAAAATAAAGGAATAGTTTATTGCTTAATA

# FIGURE 262

MALKNKFSCLWILGLCLVATTSSKIPSITDPHFIDNCIEAHNEWRGKVNPPAADMKYMIWDKG
LAKMAKAWANQCKFEHNDCLDKSYKCYAAFEYVGENIWLGGIKSFTPRHAITAWYNETQFYDF
DSLSCSRVCGHYTQLVWANSFYVGCAVAMCPNLGGASTAIFVCNYGPAGNFANMPPYARGESC
SLCSKEEKCVKNLCRTPQLIIPNQNPFLKPTGRAPQQTAFNPFSLGFLLLRIF

**Important features:**

**Signal peptide:**

amino acids 1-23

**N-glycosylation site:**

amino acids 119-123

**N-myristoylation sites:**

amino acids 103-109,150-156,160-166,161-167,175-181

**Extracellular proteins SCP/Tpx-1/Ag5/PR-1/Sc7 signature 1:**

amino acids 136-156

**Extracellular proteins SCP/Tpx-1/Ag5/PR-1/Sc7 signature 2:**

amino acids 166-178

# FIGURE 263

CGCCCTCCGACCCGCCCCGCGGCGCATTGTGGGATCTGTCGGCTTGTCAGGTGGTGGAGGAAA
AGGCGCTCCGTC**ATG**GGGATCCAGACGAGCCCCGTCCTGCTGGCCTCCCTGGGGGTGGGGCTG
GTCACTCTGCTCGGCCTGGCTGTGGGCTCCTACTTGGTTCGGAGGTCCCGCCGGCCTCAGGTC
ACTCTCCTGGACCCCAATGAAAAGTACCTGCTACGACTGCTAGACAAGACGACTGTGAGCCAC
AACACCAAGAGGTTCCGCTTTGCCCTGCCCACCGCCCACCACACTCTGGGGCTGCCTGTGGGC
AAACATATCTACCTCTCCACCCGAATTGATGGCAGCCTGGTCATCAGGCCATACACTCCTGTC
ACCAGTGATGAGGATCAAGGCTATGTGGATCTTGTCATCAAGGTCTACCTGAAGGGTGTGCAC
CCCAAATTTCCTGAGGGAGGGAAGATGTCTCAGTACCTGGATAGCCTGAAGGTTGGGGATGTG
GTGGAGTTTCGGGGGCCAAGCGGGTTGCTCACTTACACTGGAAAAGGGCATTTTAACATTCAG
CCCAACAAGAAATCTCCACCAGAACCCCGAGTGGCGAAGAAACTGGGAATGATTGCCGGCGGG
ACAGGAATCACCCCAATGCTACAGCTGATCCGGGCCATCCTGAAAGTCCCTGAAGATCCAACC
CAGTGCTTTCTGCTTTTTGCCAACCAGACAGAAAAGGATATCATCTTGCGGGAGGACTTAGAG
GAACTGCAGGCCCGCTATCCCAATCGCTTTAAGCTCTGGTTCACTCTGGATCATCCCCCAAAA
GATTGGGCCTACAGCAAGGGCTTTGTGACTGCCGACATGATCCGGGAACACCTGCCCGCTCCA
GGGGATGATGTGCTGGTACTGCTTTGTGGGCCACCCCCAATGGTGCAGCTGGCCTGCCATCCC
AACTTGGACAAACTGGGCTACTCACAAAAGATGCGATTCACCTAC**TGA**GCATCCTCCAGCTTC
CCTGGTGCTGTTCGCTGCAGTTGTTCCCCATCAGTACTCAAGCACTATAAGCCTTAGATTCCT
TTCCTCAGAGTTTCAGGTTTTTTCAGTTACATCTAGAGCTGAAATCTGGATAGTACCTGCAGG
AACAATATTCCTGTAGCCATGGAAGAGGGCAAGGCTCAGTCACTCCTTGGATGGCCTCCTAAA
TCTCCCCGTGGCAACAGGTCCAGGAGAGGCCCATGGAGCAGTCTCTTCCATGGAGTAAGAAGG
AAGGGAGCATGTACGCTTGGTCCAAGATTGGCTAGTTCCTTGATAGCATCTTACTCTCACCTT
CTTTGTGTCTGTGATGAAAGGAACAGTCTGTGCAATGGGTTTTACTTAAACTTCACTGTTCAA
CCTATGAGCAAATCTGTATGTGTGAGTATAAGTTGAGCATAGCATACTTCCAGAGGTGGTNTT
ATGGAGATGGCAAGAAAGGAGGAAATGATTTCTTCAGATNTCAAAGGAGTCTGAAATATCATA
TTTCTGTGTGTGTCTCTCTCAGCCCCTGCCCAGGCTAGAGGGAAACAGCTACTGATAATCGAA
AACTGCTGTTTGTGGCANGAACCCCTGGCTGTGCAAATAAATGGGGCTGAGGCCCCTGTGTGA
TATTGAAAAAAAAAAAAAAAAAAAAAAAAAAAAAAAAAAAAAAAAAAAAAAGA

# FIGURE 264

MGIQTSPVLLASLGVGLVTLLGLAVGSYLVRRSRRPQVTLLDPNEKYLLRLLDKTTVSHNTKR
FRFALPTAHHTLGLPVGKHIYLSTRIDGSLVIRPYTPVTSDEDQGYVDLVIKVYLKGVHPKFP
EGGKMSQYLDSLKVGDVVEFRGPSGLLTYTGKGHFNIQPNKKSPPEPRVAKKLGMIAGGTGIT
PMLQLIRAILKVPEDPTQCFLLFANQTEKDIILREDLEELQARYPNRFKLWFTLDHPPKDWAY
SKGFVTADMIREHLPAPGDDVLVLLCGPPPMVQLACHPNLDKLGYSQKMRFTY

**Important features:**

**Signal peptide:**

amino acids 1-26

**N-glycosylation site:**

amino acids 214-218

**N-myristoylation sites:**

amino acids 22-28,76-82,128-134,180-186

# FIGURE 265

CCCGTGCCAAGAGTGACGTAAGTACCGCCTATAGAGTCTATAGGCCCACTTGGCTTCGTTAGA
ACGCGGCTACAATTAATACATAACCTTATGTATCATACACATACGATTTAGGTGACACTATAG
AATAACATCCACTTTGCCTTTCTCTCCACAGGTGTCCACTCCCAGGTCCAACTGCACCTCGGT
TCTATCGATAATCTCAGCACCAGCCACTCAGAGCAGGGCACG**ATG**TTGGGGGCCCGCCTCAGG
CTCTGGGTCTGTGCCTTGTGCAGCGTCTGCAGCATGAGCGTCCTCAGAGCCTATCCCAATGCC
TCCCCACTGCTCGGCTCCAGCTGGGGTGGCCTGATCCACCTGTACACAGCCACAGCCAGGAAC
AGCTACCACCTGCAGATCCACAAGAATGGCCATGTGGATGGCGCACCCCATCAGACCATCTAC
AGTGCCCTGATGATCAGATCAGAGGATGCTGGCTTTGTGGTGATTACAGGTGTGATGAGCAGA
AGATACCTCTGCATGGATTTCAGAGGCAACATTTTTGGATCACACTATTTCGACCCGGAGAAC
TGCAGGTTCCAACACCAGACGCTGGAAAACGGGTACGACGTCTACCACTCTCCTCAGTATCAC
TTCCTGGTCAGTCTGGGCCGGGCGAAGAGAGCCTTCCTGCCAGGCATGAACCCACCCCCGTAC
TCCCAGTTCCTGTCCCGGAGGAACGAGATCCCCCTAATTCACTTCAACACCCCCATACCACGG
CGGCACACCCGGAGCGCCGAGGACGACTCGGAGCGGGACCCCCTGAACGTGCTGAAGCCCCGG
GCCCGGATGACCCCGGCCCCGGCCTCCTGTTCACAGGAGCTCCCGAGCGCCGAGGACAACAGC
CCGATGGCCAGTGACCCATTAGGGGTGGTCAGGGGCGGTCGAGTGAACACGCACGCTGGGGGA
ACGGGCCCGGAAGGCTGCCGCCCCTTCGCCAAGTTCATC**TAG**GGTCGCTGG

# FIGURE 266

MLGARLRLWVCALCSVCSMSVLRAYPNASPLLGSSWGGLIHLYTATARNSYHLQIHKNGHVDG
APHQTIYSALMIRSEDAGFVVITGVMSRRYLCMDFRGNIFGSHYFDPENCRFQHQTLENGYDV
YHSPQYHFLVSLGRAKRAFLPGMNPPPYSQFLSRRNEIPLIHFNTPIPRRHTRSAEDDSERDP
LNVLKPRARMTPAPASCSQELPSAEDNSPMASDPLGVVRGGRVNTHAGGTGPEGCRPFAKFI

**Important features:**

**Signal peptide:**

amino acids 1-24

**cAMP- and cGMP-dependent protein kinase phosphorylation site.**

amino acids 175-179

**N-myristoylation site.**

amino acids 33-39, 100-106, 225-231, 229-235

**HBGF/FGF family proteins**

amino acids 73-124

# FIGURE 267

GGCTGAGGGGAGGCCCGGAGCCTTTCTGGGGCCTGGGGGATCCTCTTGCACTGGTGGGTGGAGAGAAGCGCCTGC
AGCCAACCAGGGTCAGGCTGTGCTCACAGTTTCCTCTGGCGGCATGTAAAGGCTCCACAAAGGAGTTGGGAGTTC
AAATGAGGCTGCTGCGGACGGCCTGAGGATGGACCCCAAGCCCTGGACCTGCCGAGCGTGGCACTGAGGCAGCGG
CTGACGCTACTGTGAGGGAAAGAAGGTTGTGAGCAGCCCCGCAGGACCCCTGGCCAGCCCTGGCCCCAGCCTCTG
CCGGAGCCCTCTGTGGAGGCAGAGCCAGTGGAGCCCAGTGAGGCAGGGCTGCTTGGCAGCCACCGGCCTGCAACT
CAGGAACCCCTCCAGAGGCCATGGACAGGCTGCCCCGCTGACGGCCAGGGTGAAGCATGTGAGGAGCCGCCCCGG
AGCCAAGCAGGAGGGAAGAGGCTTTCATAGATTCTATTCACAAAGAATAACCACCATTTTGCAAGGACC**ATG**AGG
CCACTGTGCGTGACATGCTGGTGGCTCGGACTGCTGGCTGCCATGGGAGCTGTTGCAGGCCAGGAGGACGGTTTT
GAGGGCACTGAGGAGGGCTCGCCAAGAGAGTTCATTTACCTAAACAGGTACAAGCGGGCGGGCGAGTCCCAGGAC
AAGTGCACCTACACCTTCATTGTGCCCCAGCAGCGGGTCACGGGTGCCATCTGCGTCAACTCCAAGGAGCCTGAG
GTGCTTCTGGAGAACCGAGTGCATAAGCAGGAGCTAGAGCTGCTCAACAATGAGCTGCTCAAGCAGAAGCGGCAG
ATCGAGACGCTGCAGCAGCTGGTGGAGGTGGACGGCGGCATTGTGAGCGAGGTGAAGCTGCTGCGCAAGGAGAGC
CGCAACATGAACTCGCGGGTCACGCAGCTCTACATGCAGCTCCTGCACGAGATCATCCGCAAGCGGGACAACGCG
TTGGAGCTCTCCCAGCTGGAGAACAGGATCCTGAACCAGACAGCCGACATGCTGCAGCTGGCCAGCAAGTACAAG
GACCTGGAGCACAAGTACCAGCACCTGGCCACACTGGCCCACAACCAATCAGAGATCATCGCGCAGCTTGAGGAG
CACTGCCAGAGGGTGCCCTCGGCCAGGCCCGTCCCCCAGCCACCCCCCGCTGCCCCGCCCCGGGTCTACCAACCA
CCCACCTACAACCGCATCATCAACCAGATCTCTACCAACGAGATCCAGAGTGACCAGAACCTGAAGGTGCTGCCA
CCCCCTCTGCCCACTATGCCCACTCTCACCAGCCTCCCATCTTCCACCGACAAGCCGTCGGGCCCATGGAGAGAC
TGCCTGCAGGCCCTGGAGGATGGCCACGACACCAGCTCCATCTACCTGGTGAAGCCGGAGAACACCAACCGCCTC
ATGCAGGTGTGGTGCGACCAGAGACACGACCCCGGGGGCTGGACCGTCATCCAGAGACGCCTGGATGGCTCTGTT
AACTTCTTCAGGAACTGGGAGACGTACAAGCAAGGGTTTGGGAACATTGACGGCGAATACTGGCTGGGCCTGGAG
AACATTTACTGGCTGACGAACCAAGGCAACTACAAACTCCTGGTGACCATGGAGGACTGGTCCGGCCGCAAAGTC
TTTGCAGAATACGCCAGTTTCCGCCTGGAACCTGAGAGCGAGTATTATAAGCTGCGGCTGGGGCGCTACCATGGC
AATGCGGGTGACTCCTTTACATGGCACAACGGCAAGCAGTTCACCACCCTGGACAGAGATCATGATGTCTACACA
GGAAACTGTGCCCACTACCAGAAGGGAGGCTGGTGGTATAACGCCTGTGCCCACTCCAACCTCAACGGGGTCTGG
TACCGCGGGGGCCATTACCGGAGCCGCTACCAGGACGGAGTCTACTGGGCTGAGTTCCGAGGAGGCTCTTACTCA
CTCAAGAAAGTGGTGATGATGATCCGACCGAACCCCAACACCTTCCAC**TAA**GCCAGCTCCCCCTCCTGACCTCTC
GTGGCCATTGCCAGGAGCCCACCCTGGTCACGCTGGCCACAGCACAAAGAACAACTCCTCACCAGTTCATCCTGA
GGCTGGGAGGACCGGGATGCTGGATTCTGTTTTCCGAAGTCACTGCAGCGGATGATGGAACTGAATCGATACGGT
GTTTTCTGTCCCTCCTACTTTCCTTCACACCAGACAGCCCCTCATGTCTCCAGGACAGGACAGGACTACAGACAA
CTCTTTCTTTAAATAAATTAAGTCTCTACAATAAAAAAAA

# FIGURE 268

MRPLCVTCWWLGLLAAMGAVAGQEDGFEGTEEGSPREFIYLNRYKRAGESQDKCTYTFIVPQQ
RVTGAICVNSKEPEVLLENRVHKQELELLNNELLKQKRQIETLQQLVEVDGGIVSEVKLLRKE
SRNMNSRVTQLYMQLLHEIIRKRDNALELSQLENRILNQTADMLQLASKYKDLEHKYQHLATL
AHNQSEIIAQLEEHCQRVPSARPVPQPPPAAPPRVYQPPTYNRIINQISTNEIQSDQNLKVLP
PPLPTMPTLTSLPSSTDKPSGPWRDCLQALEDGHDTSSIYLVKPENTNRLMQVWCDQRHDPGG
WTVIQRRLDGSVNFFRNWETYKQGFGNIDGEYWLGLENIYWLTNQGNYKLLVTMEDWSGRKVF
AEYASFRLEPESEYYKLRLGRYHGNAGDSFTWHNGKQFTTLDRDHDVYTGNCAHYQKGGWWYN
ACAHSNLNGVWYRGGHYRSRYQDGVYWAEFRGGSYSLKKVVMMIRPNPNTFH

**Important features:**

**Signal peptide:**
amino acids 1-22

**N-glycosylation sites:**
amino acids 164-168,192-196

**cAMP- and cGMP-dependent protein kinase phosphorylation site:**
amino acids 124-128

**Tyrosine kinase phosphorylation sites:**
amino acids 177-184,385-393,385-394,461-468

**N-myristoylation sites:**
amino acids 12-18,18-24,22-28,29-35,114-120,341-347,465-471,
473-479

**Amidation site:**
amino acids 373-377

**Fibrinogen beta and gamma chains C-terminal domain signature:**
amino acids 438-451

**Fibrinogen beta and gamma chains C-terminal domain proteins:**
amino acids 305-343, 365-402, 411-424, 428-458

**Trehalase proteins:**
amino acids 275-292

# FIGURE 269

GCCGAGCTGAGCGGATCCTCAC**ATG**ACTGTGATCCGATTCTTTCCAGCGGCTTCTGCAACCAA
GCGGGTCTTACCCCCGGTCCTCCGCGTCTCCAGTCCTCGCACCTGGAACCCCAACGTCCCCGA
GAGTCCCCGAATCCCCGCTCCCAGGCTACCTAAGAGGATGAGCGGTGCTCCGACGGCCGGGGC
AGCCCTGATGCTCTGCGCCGCCACCGCCGTGCTACTGAGCGCTCAGGGCGGACCCGTGCAGTC
CAAGTCGCCGCGCTTTGCGTCCTGGGACGAGATGAATGTCCTGGCGCACGGACTCCTGCAGCT
CGGCCAGGGGCTGCGCGAACACGCGGAGCGCACCCGCAGTCAGCTGAGCGCGCTGGAGCGGCG
CCTGAGCGCGTGCGGGTCCGCCTGTCAGGGAACCGAGGGGTCCACCGACCTCCCGTTAGCCCC
TGAGAGCCGGGTGGACCCTGAGGTCCTTCACAGCCTGCAGACACAACTCAAGGCTCAGAACAG
CAGGATCCAGCAACTCTTCCACAAGGTGGCCCAGCAGCAGCGGCACCTGGAGAAGCAGCACCT
GCGAATTCAGCATCTGCAAAGCCAGTTTGGCCTCCTGGACCACAAGCACCTAGACCATGAGGT
GGCCAAGCCTGCCCGAAGAAAGAGGCTGCCCGAGATGGCCCAGCCAGTTGACCCGGCTCACAA
TGTCAGCCGCCTGCACCGGCTGCCCAGGGATTGCCAGGAGCTGTTCCAGGTTGGGGAGAGGCA
GAGTGGACTATTTGAAATCCAGCCTCAGGGGTCTCCGCCATTTTTGGTGAACTGCAAGATGAC
CTCAGATGGAGGCTGGACAGTAATTCAGAGGCGCCACGATGGCTCAGTGGACTTCAACCGGCC
CTGGGAAGCCTACAAGGCGGGGTTTGGGGATCCCCACGGCGAGTTCTGGCTGGGTCTGGAGAA
GGTGCATAGCATCACGGGGGACCGCAACAGCCGCCTGGCCGTGCAGCTGCGGGACTGGGATGG
CAACGCCGAGTTGCTGCAGTTCTCCGTGCACCTGGGTGGCGAGGACACGGCCTATAGCCTGCA
GCTCACTGCACCCGTGGCCGGCCAGCTGGGCGCCACCACCGTCCCACCCAGCGGCCTCTCCGT
ACCCTTCTCCACTTGGGACCAGGATCACGACCTCCGCAGGGACAAGAACTGCGCCAAGAGCCT
CTCTGGAGGCTGGTGGTTTGGCACCTGCAGCCATTCCAACCTCAACGGCCAGTACTTCCGCTC
CATCCCACAGCAGCGGCAGAAGCTTAAGAAGGGAATCTTCTGGAAGACCTGGCGGGGCCGCTA
CTACCCGCTGCAGGCCACCACCATGTTGATCCAGCCCATGGCAGCAGAGGCAGCCTCC**TAG**CG
TCCTGGCTGGGCCTGGTCCCAGGCCCACGAAAGACGGTGACTCTTGGCTCTGCCCGAGGATGT
GGCCGTTCCCTGCCTGGGCAGGGGCTCCAAGGAGGGGCCATCTGGAAACTTGTGGACAGAGAA
GAAGACCACGACTGGAGAAGCCCCCTTTCTGAGTGCAGGGGGGCTGCATGCGTTGCCTCCTGA
GATCGAGGCTGCAGGATATGCTCAGACTCTAGAGGCGTGGACCAAGGGGCATGGAGCTTCACT
CCTTGCTGGCCAGGGAGTTGGGGACTCAGAGGGACCACTTGGGGCCAGCCAGACTGGCCTCAA
TGGCGGACTCAGTCACATTGACTGACGGGGACCAGGGCTTGTGTGGGTCGAGAGCGCCCTCAT
GGTGCTGGTGCTGTTGTGTGTAGGTCCCCTGGGGACACAAGCAGGCGCCAATGGTATCTGGGC
GGAGCTCACAGAGTTCTTGGAATAAAAGCAACCTCAGAACAC

# FIGURE 270

MTVIRFFPAASATKRVLPPVLRVSSPRTWNPNVPESPRIPAPRLPKRMSGAPTAGAALMLCAA
TAVLLSAQGGPVQSKSPRFASWDEMNVLAHGLLQLGQGLREHAERTRSQLSALERRLSACGSA
CQGTEGSTDLPLAPESRVDPEVLHSLQTQLKAQNSRIQQLFHKVAQQQRHLEKQHLRIQHLQS
QFGLLDHKHLDHEVAKPARRKRLPEMAQPVDPAHNVSRLHRLPRDCQELFQVGERQSGLFEIQ
PQGSPPFLVNCKMTSDGGWTVIQRRHDGSVDFNRPWEAYKAGFGDPHGEFWLGLEKVHSITGD
RNSRLAVQLRDWDGNAELLQFSVHLGGEDTAYSLQLTAPVAGQLGATTVPPSGLSVPFSTWDQ
DHDLRRDKNCAKSLSGGWWFGTCSHSNLNGQYFRSIPQQRQKLKKGIFWKTWRGRYYPLQATT
MLIQPMAAEAAS

**Important features:**
**Signal peptide:**
Amino acids 1-13

**Transmembrane domain:**
Amino acids 53-70

**N-glycosylation site:**
Amino acids 224-228

**cAMP- and cGMP-dependent protein kinase phosphorylation sites:**
Amino acids 46-50;118-122

**N-myristoylation sites:**
Amino acids 50-56;129-135;341-347;357-363

**Fibrinogen beta and gamma chains C-terminal domain signature:**
Amino acids 396-409

# FIGURE 271

CGGACGCGTGGGGGAAACCCTTCCGAGAAAACAGCAACAAGCTGAGCTGCTGTGACAGAGGGG
AACAAG**ATG**GCGGCGCCGAAGGGGAGCCTCTGGGTGAGGACCCAACTGGGGCTCCCGCCGCTG
CTGCTGCTGACCATGGCCTTGGCCGGAGGTTCGGGGACCGCTTCGGCTGAAGCATTTGACTCG
GTCTTGGGTGATACGGCGTCTTGCCACCGGGCCTGTCAGTTGACCTACCCCTTGCACACCTAC
CCTAAGGAAGAGGAGTTGTACGCATGTCAGAGAGGTTGCAGGCTGTTTTCAATTTGTCAGTTT
GTGGATGATGGAATTGACTTAAATCGAACTAAATTGGAATGTGAATCTGCATGTACAGAAGCA
TATTCCCAATCTGATGAGCAATATGCTTGCCATCTTGGTTGCCAGAATCAGCTGCCATTCGCT
GAACTGAGACAAGAACAACTTATGTCCCTGATGCCAAAAATGCACCTACTCTTTCCTCTAACT
CTGGTGAGGTCATTCTGGAGTGACATGATGGACTCCGCACAGAGCTTCATAACCTCTTCATGG
ACTTTTTATCTTCAAGCCGATGACGGAAAAATAGTTATATTCCAGTCTAAGCCAGAAATCCAG
TACGCACCACATTTGGAGCAGGAGCCTACAAATTTGAGAGAATCATCTCTAAGCAAAATGTCC
TATCTGCAAATGAGAAATTCACAAGCGCACAGGAATTTTCTTGAAGATGGAGAAAGTGATGGC
TTTTTAAGATGCCTCTCTCTTAACTCTGGGTGGATTTTAACTACAACTCTTGTCCTCTCGGTG
ATGGTATTGCTTTGGATTTGTTGTGCAACTGTTGCTACAGCTGTGGAGCAGTATGTTCCCTCT
GAGAAGCTGAGTATCTATGGTGACTTGGAGTTTATGAATGAACAAAAGCTAAACAGATATCCA
GCTTCTTCTCTTGTGGTTGTTAGATCTAAAACTGAAGATCATGAAGAAGCAGGGCCTCTACCT
ACAAAAGTGAATCTTGCTCATTCTGAAATT**TAA**GCATTTTTCTTTTAAAAGACAAGTGTAATA
GACATCTAAAATTCCACTCCTCATAGAGCTTTTAAAATGGTTTCATTGGATATAGGCCTTAAG
AAATCACTATAAAATGCAAATAAAGTTACTCAAATCTGTG

# FIGURE 272

MAAPKGSLWVRTQLGLPPLLLLTMALAGGSGTASAEAFDSVLGDTASCHRACQLTYPLHTYPK
EEELYACQRGCRLFSICQFVDDGIDLNRTKLECESACTEAYSQSDEQYACHLGCQNQLPFAEL
RQEQLMSLMPKMHLLFPLTLVRSFWSDMMDSAQSFITSSWTFYLQADDGKIVIFQSKPEIQYA
PHLEQEPTNLRESSLSKMSYLQMRNSQAHRNFLEDGESDGFLRCLSLNSGWILTTTLVLSVMV
LLWICCATVATAVEQYVPSEKLSIYGDLEFMNEQKLNRYPASSLVVVRSKTEDHEEAGPLPTK
VNLAHSEI

**Important features:**

**Signal peptide:**

amino acids 1-31

**Transmembrane domain:**

amino acids 241-260

**N-glycosylation site:**

amino acids 90-94

**N-myristoylation sites:**

amino acids 28-34,29-35,31-37,86-92

# FIGURE 273

CCCACGCGTCCGAACCTCTCCAGCG**ATG**GGAGCCGCCCGCCTGCTGCCCAACCTCACTCTGTG
CTTACAGCTGCTGATTCTCTGCTGTCAAACTCAGTACGTGAGGGACCAGGGCGCCATGACCGA
CCAGCTGAGCAGGCGGCAGATCCGCGAGTACCAACTCTACAGCAGGACCAGTGGCAAGCACGT
GCAGGTCACCGGGCGTCGCATCTCCGCCACCGCCGAGGACGGCAACAAGTTTGCCAAGCTCAT
AGTGGAGACGGACACGTTTGGCAGCCGGGTTCGCATCAAAGGGGCTGAGAGTGAGAAGTACAT
CTGTATGAACAAGAGGGGCAAGCTCATCGGGAAGCCCAGCGGGAAGAGCAAAGACTGCGTGTT
CACGGAGATCGTGCTGGAGAACAACTATACGGCCTTCCAGAACGCCCGGCACGAGGGCTGGTT
CATGGCCTTCACGCGGCAGGGGCGGCCCCGCCAGGCTTCCCGCAGCCGCCAGAACCAGCGCGA
GGCCCACTTCATCAAGCGCCTCTACCAAGGCCAGCTGCCCTTCCCCAACCACGCCGAGAAGCA
GAAGCAGTTCGAGTTTGTGGGCTCCGCCCCCACCCGCCGGACCAAGCGCACACGGCGGCCCCA
GCCCCTCACG**TAG**TCTGGGAGGCAGGGGGCAGCAGCCCCTGGGCCGCCTCCCCACCCCTTTCC
CTTCTTAATCCAAGGACTGGGCTGGGGTGGCGGGAGGGGAGCCAGATCCCCGAGGGAGGACCC
TGAGGGCCGCGAAGCATCCGAGCCCCCAGCTGGGAAGGGGCAGGCCGGTGCCCCAGGGGCGGC
TGGCACAGTGCCCCCTTCCCGGACGGGTGGCAGGCCCTGGAGAGGAACTGAGTGTCACCCTGA
TCTCAGGCCACCAGCCTCTGCCGGCCTCCCAGCCGGGCTCCTGAAGCCCGCTGAAAGGTCAGC
GACTGAAGGCCTTGCAGACAACCGTCTGGAGGTGGCTGTCCTCAAAATCTGCTTCTCGGATCT
CCCTCAGTCTGCCCCCAGCCCCCAAACTCCTCCTGGCTAGACTGTAGGAAGGGACTTTTGTTT
GTTTGTTTGTTTCAGGAAAAAAGAAAGGGAGAGAGAGGAAAATAGAGGGTTGTCCACTCCTCA
CATTCCACGACCCAGGCCTGCACCCCACCCCCAACTCCCAGCCCCGGAATAAAACCATTTTCC
TGC

# FIGURE 274

MGAARLLPNLTLCLQLLILCCQTQYVRDQGAMTDQLSRRQIREYQLYSRTSGKHVQVTGRRIS
ATAEDGNKFAKLIVETDTFGSRVRIKGAESEKYICMNKRGKLIGKPSGKSKDCVFTEIVLENN
YTAFQNARHEGWFMAFTRQGRPRQASRSRQNQREAHFIKRLYQGQLPFPNHAEKQKQFEFVGS
APTRRTKRTRRPQPLT

**Important features:**

**Signal peptide:**

Amino acids 1-22

**N-glycosylation site.**

amino acids 9-13, 126-130

**cAMP- and cGMP-dependent protein kinase phosphorylation site.**

amino acids 60-64

**Casein kinase II phosphorylation site.**

amino acids 65-69

**Tyrosine kinase phosphorylation site.**

amino acids 39-48, 89-97

**N-myristoylation site.**

amino acids 69-75, 188-194

**Amidation site.**

amino acids 58-62

**HBGF/FGF family signature.**

amino acids 103-128

# FIGURE 275

TATTTACCATATCAGATTCACATTCAGTCCTCAGCAAA**ATG**AAGGGCTCCATTTTCACTCTGT
TTTTATTCTCTGTCCTATTTGCCATCTCAGAAGTGCGGAGCAAGGAGTCTGTGAGACTCTGTG
GGCTAGAATACATACGGACAGTCATCTATATCTGTGCTAGCTCCAGGTGGAGAAGGCATCTGG
AGGGGATCCCTCAAGCTCAGCAAGCTGAGACAGGAAACTCCTTCCAGCTCCCACATAAACGTG
AGTTTTCTGAGGAAAATCCAGCGCAAAACCTTCCGAAGGTGGATGCCTCAGGGGAAGACCGTC
TTTGGGGTGGACAGATGCCCACTGAAGAGCTTTGGAAGTCAAAGAAGCATTCAGTGATGTCAA
GACAAGATTTACAAACTTTGTGTTGCACTGATGGCTGTTCCATGACTGATTTGAGTGCTCTTT
GC**TAA**GACAAGAGCAAATACCCAATGGGTGGCAGAGCTTTATCACATGTTTAATTACAGTGTT
TTACTGCCTGGTAGAACACTAATATTGTGTTATTAAAATGATGGCTTTTGGGTAGGCAAAACT
TCTTTTCTAAAAGGTATAGCTGAGCGGTTGAAACCACAGTGATCTCTATTTTCTCCCTTTGCC
AAGGTTAATGAACTGTTCTTTTCAAATTCTACTAATGCTTTGAAATTTCAAATGCTGCGCAAA
ATTGCAATAAAAATGCTATAAA

# FIGURE 276

MKGSIFTLFLFSVLFAISEVRSKESVRLCGLEYIRTVIYICASSRWRRHLEGIPQAQQAETGN
SFQLPHKREFSEENPAQNLPKVDASGEDRLWGGQMPTEELWKSKKHSVMSRQDLQTLCCTDGC
SMTDLSALC

**Important features:**

**Signal sequence:**

amino acids 1-18

**cAMP- and cGMP-dependent protein kinase phosphorylation site:**

amino acids 107-111

**N-myristoylation sites:**

amino acids 3-9,52-58,96-102,125-131

**Insulin family signature:**

amino acids 121-136

**Insulin family proteins:**

amino acids 28-46

# FIGURE 277

GCAGCTGGTTACTGCATTTCTCCATGTGGCAGACAGAGCAAAGCCACAACGCTTTCTCTGCTGGATTAAAGACGG
CCCACAGACCAGAACTTCCACTATACTACTTAAAATTACATAGGTGGCTTGTCAAATTCAATTGATTAGTATTGT
AAAAGGAAAAAGAAGTTCCTTCTTACAGCTTGGATTCAACGGTCCAAAACAAAAATGCAGCTGCCATTAAAGTCT
CAGATGAACAAACTTCTACACTGATTTTTAAAATCAAGAATAAGGGCAGCAAGTTTCTGGATTCACTGAATCAAC
AGACACAAAAAGCTGGCAATATAGCAACTATGAAGAGAAAAGCTACTAATAAAATTAACCCAACGCATAGAAGAC
TTTTTTTTCTCTTCTAAAAACAACTAAGTAAAGACTTAAATTTAAACACATCATTTTACAACCTCATTTCAAA**AT**
**G**AAGACTTTTACCTGGACCCTAGGTGTGCTATTCTTCCTACTAGTGGACACTGGACATTGCAGAGGTGGACAATT
CAAAATTAAAAAAATAAACCAGAGAAGATACCCTCGTGCCACAGATGGTAAAGAGGAAGCAAAGAAATGTGCATA
CACATTCCTGGTACCTGAACAAAGAATAACAGGGCCAATCTGTGTCAACACCAAGGGGCAAGATGCAAGTACCAT
TAAAGACATGATCACCAGGATGGACCTTGAAAACCTGAAGGATGTGCTCTCCAGGCAGAAGCGGGAGATAGATGT
TCTGCAACTGGTGGTGGATGTAGATGGAAACATTGTGAATGAGGTAAAGCTGCTGAGAAAGGAAAGCCGTAACAT
GAACTCTCGTGTTACTCAACTCTATATGCAATTATTACATGAGATTATCCGTAAGAGGGATAATTCACTTGAACT
TTCCCAACTGGAAAACAAAATCCTCAATGTCACCACAGAAATGTTGAAGATGGCAACAAGATACAGGGAACTAGA
GGTGAAATACGCTTCCTTGACTGATCTTGTCAATAACCAATCTGTGATGATCACTTTGTTGGAAGAACAGTGCTT
GAGGATATTTTCCCGACAAGACACCCATGTGTCTCCCCCACTTGTCCAGGTGGTGCCACAACATATTCCTAACAG
CCAACAGTATACTCCTGGTCTGCTGGGAGGTAACGAGATTCAGAGGGATCCAGGTTATCCCAGAGATTTAATGCC
ACCACCTGATCTGGCAACTTCTCCCACCAAAAGCCCTTTCAAGATACCACCGGTAACTTTCATCAATGAAGGACC
ATTCAAAGACTGTCAGCAAGCAAAAGAAGCTGGGCATTCGGTCAGTGGGATTTATATGATTAAACCTGAAAACAG
CAATGGACCAATGCAGTTATGGTGTGAAAACAGTTTGGACCCTGGGGGTTGGACTGTTATTCAGAAAAGAACAGA
CGGCTCTGTCAACTTCTTCAGAAATTGGGAAAATTATAAGAAAGGGTTTGGAAACATTGACGGAGAATACTGGCT
TGGACTGGAAAATATCTATATGCTTAGCAATCAAGATAATTACAAGTTATTGATTGAATTAGAAGACTGGAGTGA
TAAAAAAGTCTATGCAGAATACAGCAGCTTTCGTCTGGAACCTGAAAGTGAATTCTATAGACTGCGCCTGGGAAC
TTACCAGGGAAATGCAGGGGATTCTATGATGTGGCATAATGGTAAACAATTCACCACACTGGACAGAGATAAAGA
TATGTATGCAGGAAACTGCGCCCACTTTCATAAAGGAGGCTGGTGGTACAATGCCTGTGCACATTCTAACCTAAA
TGGAGTATGGTACAGAGGAGGCCATTACAGAAGCAAGCACCAAGATGGAATTTTCTGGGCCGAATACAGAGGCGG
GTCATACTCCTTAAGAGCAGTTCAGATGATGATCAAGCCTATTGAC**TGA**AGAGAGACACTCGCCAATTTAAATGA
CACAGAACTTTGTACTTTTCAGCTCTTAAAAATGTAAATGTTACATGTATATTACTTGGCACAATTTATTTCTAC
ACAGAAAGTTTTTAAAATGAATTTTACCGTAACTATAAAAGGGAACCTATAAATGTAGTTTCATCTGTCGTCAAT
TACTGCAGAAAATTATGTGTATCCACAACCTAGTTATTTTAAAAATTATGTTGACTAAATACAAAGTTTGTTTTC
TAAAATGTAAATATTTGCCACAATGTAAAGCAAATCTTAGCTATATTTTAAATCATAAATAACATGTTCAAGATA
CTTAACAATTTATTTAAAATCTAAGATTGCTCTAACGTCTAGTGAAAAAAATATTTTTTAAATTTCAGCCAAATA
ATGCATTTTATTTTATAAAAATACAGACAGAAAATTAGGGAGAAACTTCTAGTTTTGCCAATAGAAAATGTTCTT
CCATTGAATAAAAGTTATTTCAAATTGAATTTGTGCCTTTCACACGTAATGATTAAATCTGAATTCTTAATAATA
TATCCTATGCTGATTTTCCCAAAACATGACCCATAGTATTAAATACATATCATTTTTAAAAATAAAAAAAAACCC
AAAAATAATGCATGCATAATTTAAATGGTCAATTTATAAAGACAAATCTATGAATGAATTTTTCAGTGTTATCTT
CATATGATATGCTGAACACCAAAATCTCCAGAAATGCATTTTATGTAGTTCTAAAATCAGCAAAATATTGGTATT
ACAAAAATGCAGAATATTTAGTGTGCTACAGATCTGAATTATAGTTCTAATTTATTATTACTTTTTTTCTAATTT
ACTGATCTTACTACTACAAAGAAAAAAAAACCCAACCCATCTGCAATTCAAATCAGAAAGTTTGGACAGCTTTAC
AAGTATTAGTGCATGCTCAGAACAGGTGGGACTAAAACAAACTCAAGGAACTGTTGGCTGTTTTCCCGATACTGA
GAATTCAACAGCTCCAGAGCAGAAGCCACAGGGGCATAGCTTAGTCCAAACTGCTAATTTCATTTTACAGTGTAT
GTAACGCTTAGTCTCACAGTGTCTTTAACTCATCTTTGCAATCAACAACTTTACTAGTGACTTTCTGGAACAATT
TCCTTTCAGGAATACATATTCACTGCTTAGAGGTGACCTTGCCTTAATATATTTGTGAAGTTAAAATTTTAAAGA
TAGCTCATGAAACTTTTGCTTAAGCAAAAAGAAAACCTCGAATTGAAATGTGTGAGGCAAACTATGCATGGGAAT
AGCTTAATGTGAAGATAATCATTTGGACAACTCAAATCCATCAACATGACCAATGTTTTTCATCTGCCACATCTC
AAAATAAAACTTCTGGTGAAACAAATTAAACAAAATATCCAAACCTCAAAAAAAA

# FIGURE 278

MKTFTWTLGVLFFLLVDTGHCRGGQFKIKKINQRRYPRATDGKEEAKKCAYTFLVPEQRITGP
ICVNTKGQDASTIKDMITRMDLENLKDVLSRQKREIDVLQLVVDVDGNIVNEVKLLRKESRNM
NSRVTQLYMQLLHEIIRKRDNSLELSQLENKILNVTTEMLKMATRYRELEVKYASLTDLVNNQ
SVMITLLEEQCLRIFSRQDTHVSPPLVQVVPQHIPNSQQYTPGLLGGNEIQRDPGYPRDLMPP
PDLATSPTKSPFKIPPVTFINEGPFKDCQQAKEAGHSVSGIYMIKPENSNGPMQLWCENSLDP
GGWTVIQKRTDGSVNFFRNWENYKKGFGNIDGEYWLGLENIYMLSNQDNYKLLIELEDWSDKK
VYAEYSSFRLEPESEFYRLRLGTYQGNAGDSMMWHNGKQFTTLDRDKDMYAGNCAHFHKGGWW
YNACAHSNLNGVWYRGGHYRSKHQDGIFWAEYRGGSYSLRAVQMMIKPID

**Important features:**

**Signal sequence:**

Amino acids 1-23

**N-glycosylation sites:**

Amino acids 160-164;188-192

**cAMP- and cGMP-dependent protein kinase phosphorylation site:**

Amino acids 120-124

**Tyrosine kinase phosphorylation sites:**

Amino acids 173-180;387-396

**N-myristoylation sites:**

Amino acids 70-76;110-116;232-238,343-349;400-406;467-473;
475-487

**Fibrinogen beta and gamma chains C-terminal domain signature:**

Amino acids 440-453

# FIGURE 279

CCCACGCGTCCGCGCAGTCGCGCAGTTCTGCCTCCGCCTGCCAGTCTCGCCCGCGATCCCGGC
CCGGGGCTGTGGCGTCGACTCCGACCCAGGCAGCCAGCAGCCCGCGCGGGAGCCGGACCGCCG
CCGGAGGAGCTCGGACGGCATGCTGAGCCCCCTCCTTTGCTGAAGCCCGAGTGCGGAGAAGCC
CGGGCAAACGCAGGCTAAGGAGACCAAAGCGGCGAAGTCGCGAGACAGCGGACAAGCAGCGGA
GGAGAAGGAGGAGGAGGCGAACCCAGAGAGGGGCAGCAAAAGAAGCGGTGGTGGTGGGCGTCG
TGGCC**ATG**GCGGCGGCTATCGCCAGCTCGCTCATCCGTCAGAAGAGGCAAGCCCGCGAGCGCG
AGAAATCCAACGCCTGCAAGTGTGTCAGCAGCCCCAGCAAAGGCAAGACCAGCTGCGACAAAA
ACAAGTTAAATGTCTTTTCCCGGGTCAAACTCTTCGGCTCCAAGAAGAGGCGCAGAAGAAGAC
CAGAGCCTCAGCTTAAGGGTATAGTTACCAAGCTATACAGCCGACAAGGCTACCACTTGCAGC
TGCAGGCGGATGGAACCATTGATGGCACCAAAGATGAGGACAGCACTTACACTCTGTTTAACC
TCATCCCTGTGGGTCTGCGAGTGGTGGCTATCCAAGGAGTTCAAACCAAGCTGTACTTGGCAA
TGAACAGTGAGGGATACTTGTACACCTCGGAACTTTTCACACCTGAGTGCAAATTCAAAGAAT
CAGTGTTTGAAAATTATTATGTGACATATTCATCAATGATATACCGTCAGCAGCAGTCAGGCC
GAGGGTGGTATCTGGGTCTGAACAAAGAAGGAGAGATCATGAAAGGCAACCATGTGAAGAAGA
ACAAGCCTGCAGCTCATTTTCTGCCTAAACCACTGAAAGTGGCCATGTACAAGGAGCCATCAC
TGCACGATCTCACGGAGTTCTCCCGATCTGGAAGCGGGACCCCAACCAAGAGCAGAAGTGTCT
CTGGCGTGCTGAACGGAGGCAAATCCATGAGCCACAATGAATCAACG**TAG**CCAGTGAGGGCAA
AAGAAGGGCTCTGTAACAGAACCTTACCTCCAGGTGCTGTTGAATTCTTCTAGCAGTCCTTCA
CCCAAAAGTTCAAATTTGTCAGTGACATTTACCAAACAAACAGGCAGAGTTCACTATTCTATC
TGCCATTAGACCTTCTTATCATCCATACTAAAGC

# FIGURE 280

MAAAIASSLIRQKRQAREREKSNACKCVSSPSKGKTSCDKNKLNVFSRVKLFGSKKRRRRRPE
PQLKGIVTKLYSRQGYHLQLQADGTIDGTKDEDSTYTLFNLIPVGLRVVAIQGVQTKLYLAMN
SEGYLYTSELFTPECKFKESVFENYYVTYSSMIYRQQQSGRGWYLGLNKEGEIMKGNHVKKNK
PAAHFLPKPLKVAMYKEPSLHDLTEFSRSGSGTPTKSRSVSGVLNGGKSMSHNEST

**Important Features:**

**N-glycosylation site:**

Amino acids 242-246

**Glycosaminoglycan attachment sites:**

Amino acids 165-169, 218-222

**Tyrosine kinase phosphorylation site:**

Amino acids 93-100

**N-myristoylation sites:**

Amino acids 87-93, 231-237

**ATP/GTP-binding site motif A (P-loop):**

Amino acids 231-239

**HBGF/FGF family proteins:**

Amino acids 78-94, 102-153

# FIGURE 281

CCAGGATGGAGCTGGGGCCTGTATAGCCATATTATTGTTCTATGCTACTAGACATGGGGGGGA
CTTGGTGAAAAAGGTATTATCCAGCCAGAGGGTCTGGGAGCCCTGTCTTACTGAACCTGGGCA
ACCTGGATATTCTGAGACATATTTTGGGGGGATTTCAGTGAAAAAAGTGGGGATCCCCTCCA
TTTAGAGTGTAGCAAAGGAAAAAACACCAAGGTTGGGTTCCTTCCTGACATTGGCAGTGCCCC
AGTAGGGGTGGGATGAGCGAATATTCCCAAAGCTAAAGTCCCACACCCTGTAGATTACAAGAG
TGGATTTGGCAGGAGTGTGCCCCAAAATACAGTGGAAAGGTGCCTGAAGATATTTAAACCACG
TCTTGGAAATTTAGTGGGTCTTGGCTTTGGGATAGGTGAAGTGAGGACAGACACTGGAGAGGA
GGGAAAGGGGACGTTTTCAATAGGAGGCAAAACTCGAGGGTGGGATCCACTGAGGAGTACATA
GGCTGCTGGATCTGGTGGAGCCAGCACTGGGCCCACGGGTGGTAACTGGCTGCTGTGGAGGGG
GGTACGTGAGGGGGGGGTCTGGGGCTTATCCTCAGGTCCTGTGGGTGGGGCAGCGAGTCGGGG
CCTGAGCGTCAAGAGCATGCCCTAGTGAGCGGGCTCCTCTGGGGGAGCCCAGCGCGCTCCGGG
CGCCTGCCGGTTTGGGGGTGTCTCCTCCCGGGGCGCT**ATG**GCGGCGCTGGCCAGTAGCCTGAT
CCGGCAGAAGCGGGAGGTCCGCGAGCCCGGGGGCAGCCGGCCGGTGTCGGCGCAGCGGCGCGT
GTGTCCCCGCGGCACCAAGTCCCTTTGCCAGAAGCAGCTCCTCATCCTGCTGTCCAAGGTGCG
ACTGTGCGGGGGGCGGCCCGCGCGGCCGGACCGCGGCCCGGAGCCTCAGCTCAAAGGCATCGT
CACCAAACTGTTCTGCCGCCAGGGTTTCTACCTCCAGGCGAATCCCGACGGAAGCATCCAGGG
CACCCCAGAGGATACCAGCTCCTTCACCCACTTCAACCTGATCCCTGTGGGCCTCCGTGTGGT
CACCATCCAGAGCGCCAAGCTGGGTCACTACATGGCCATGAATGCTGAGGGACTGCTCTACAG
TTCGCCGCATTTCACAGCTGAGTGTCGCTTTAAGGAGTGTGTCTTTGAGAATTACTACGTCCT
GTACGCCTCTGCTCTCTACCGCCAGCGTCGTTCTGGCCGGGCCTGGTACCTCGGCCTGGACAA
GGAGGGCCAGGTCATGAAGGGAAACCGAGTTAAGAAGACCAAGGCAGCTGCCCACTTTCTGCC
CAAGCTCCTGGAGGTGGCCATGTACCAGGAGCCTTCTCTCCACAGTGTCCCCGAGGCCTCCCC
TTCCAGTCCCCCTGCCCCC**TGA**AATGTAGTCCCTGGACTGGAGGTTCCCTGCACTCCCAGTGA
GCCAGCCACCACCACAACCTGT

# FIGURE 282

MAALASSLIRQKREVREPGGSRPVSAQRRVCPRGTKSLCQKQLLILLSKVRLCGGRPARPDRG
PEPQLKGIVTKLFCRQGFYLQANPDGSIQGTPEDTSSFTHFNLIPVGLRVVTIQSAKLGHYMA
MNAEGLLYSSPHFTAECRFKECVFENYYVLYASALYRQRRSGRAWYLGLDKEGQVMKGNRVKK
TKAAAHFLPKLLEVAMYQEPSLHSVPEASPSSPPAP

**Important features:**

**Tyrosine kinase phosphorylation site:**

Amino acids 199-207

**N-myristoylation sites:**

Amino acids 54-60; 89-95; 131-137

**HBGF/FGF family signature:**

Amino acids 131-155

# FIGURE 283

**ATG**GCCGCGGCCATCGCTAGCGGCTTGATCCGCCAGAAGCGGCAGGCGCGGGAGCAGCACTGG
GACCGGCCGTCTGCCAGCAGGAGGCGGAGCAGCCCCAGCAAGAACCGCGGGCTCTGCAACGGC
AACCTGGTGGATATCTTCTCCAAAGTGCGCATCTTCGGCCTCAAGAAGCGCAGGTTGCGGCGC
CAAGATCCCCAGCTCAAGGGTATAGTGACCAGGTTATATTGCAGGCAAGGCTACTACTTGCAA
ATGCACCCCGATGGAGCTCTCGATGGAACCAAGGATGACAGCACTAATTCTACACTCTTCAAC
CTCATACCAGTGGGACTACGTGTTGTTGCCATCCAGGGAGTGAAAACAGGGTTGTATATAGCC
ATGAATGGAGAAGGTTACCTCTACCCATCAGAACTTTTTACCCCTGAATGCAAGTTTAAAGAA
TCTGTTTTTGAAAATTATTATGTAATCTACTCATCCATGTTGTACAGACAACAGGAATCTGGT
AGAGCCTGGTTTTTGGGATTAAATAAGGAAGGGCAAGCTATGAAAGGGAACAGAGTAAAGAAA
ACCAAACCAGCAGCTCATTTTCTACCCAAGCCATTGGAAGTTGCCATGTACCGAGAACCATCT
TTGCATGATGTTGGGGAAACGGTCCCGAAGCCTGGGGTGACGCCAAGTAAAAGCACAAGTGCG
TCTGCAATAATGAATGGAGGCAAACCAGTCAACAAGAGTAAGACAACA**TAG**

# FIGURE 284

MAAAIASGLIRQKRQAREQHWDRPSASRRRSSPSKNRGLCNGNLVDIFSKVRIFGLKKRRLRR
QDPQLKGIVTRLYCRQGYYLQMHPDGALDGTKDDSTNSTLFNLIPVGLRVVAIQGVKTGLYIA
MNGEGYLYPSELFTPECKFKESVFENYYVIYSSMLYRQQESGRAWFLGLNKEGQAMKGNRVKK
TKPAAHFLPKPLEVAMYREPSLHDVGETVPKPGVTPSKSTSASAIMNGGKPVNKSKTT

**Important features:**

**N-glycosylation sites:**

Amino acids 100-104, 242-246

**cAMP- and cGMP-dependent protein kinase phosphorylation sites:**

Amino acids 28-32, 29-33

**Tyrosine kinase phosphorylation site:**

Amino acids 199-207

**N-myristoylation sites:**

Amino acids 38-44, 89-95, 118-124, 122-128, 222-228

**HBGF/FGF family proteins:**

Amino acids 104-155, 171-198

# FIGURE 285

CGGACGCGTGGGCGGACGCGTGGGCGGACGCGTGGGCGGACGCGTGGGCTGGTTCAGGTCCAGGTTTTGCTTTGA
TCCTTTTCAAAAACTGGAGACACAGAAGAGGGCTCTAGGAAAAAGTTTTGGATGGGATTATGTGGAAACTACCCT
GCGATTCTCTGCTGCCAGAGCAGGCTCGGCGCTTCCACCCCAGTGCAGCCTTCCCCTGGCGGTGGTGAAAGAGAC
TCGGGAGTCGCTGCTTCCAAAGTGCCCGCCGTGAGTGAGCTCTCACCCCAGTCAGCCAA**ATG**AGCCTCTTCGGGC
TTCTCCTGCTGACATCTGCCCTGGCCGGCCAGAGACAGGGGACTCAGGCGGAATCCAACCTGAGTAGTAAATTCC
AGTTTTCCAGCAACAAGGAACAGAACGGAGTACAAGATCCTCAGCATGAGAGAATTATTACTGTGTCTACTAATG
GAAGTATTCACAGCCCAAGGTTTCCTCATACTTATCCAAGAAATACGGTCTTGGTATGGAGATTAGTAGCAGTAG
AGGAAAATGTATGGATACAACTTACGTTTGATGAAAGATTTGGGCTTGAAGACCCAGAAGATGACATATGCAAGT
ATGATTTTGTAGAAGTTGAGGAACCCAGTGATGGAACTATATTAGGGCGCTGGTGTGGTTCTGGTACTGTACCAG
GAAAACAGATTTCTAAAGGAAATCAAATTAGGATAAGATTTGTATCTGATGAATATTTTCCTTCTGAACCAGGGT
TCTGCATCCACTACAACATTGTCATGCCACAATTCACAGAAGCTGTGAGTCCTTCAGTGCTACCCCCTTCAGCTT
TGCCACTGGACCTGCTTAATAATGCTATAACTGCCTTTAGTACCTTGGAAGACCTTATTCGATATCTTGAACCAG
AGAGATGGCAGTTGGACTTAGAAGATCTATATAGGCCAACTTGGCAACTTCTTGGCAAGGCTTTTGTTTTTGGAA
GAAAATCCAGAGTGGTGGATCTGAACCTTCTAACAGAGGAGGTAAGATTATACAGCTGCACACCTCGTAACTTCT
CAGTGTCCATAAGGGAAGAACTAAAGAGAACCGATACCATTTTCTGGCCAGGTTGTCTCCTGGTTAAACGCTGTG
GTGGGAACTGTGCCTGTTGTCTCCACAATTGCAATGAATGTCAATGTGTCCCAAGCAAAGTTACTAAAAAATACC
ACGAGGTCCTTCAGTTGAGACCAAAGACCGGTGTCAGGGGATTGCACAAATCACTCACCGACGTGGCCCTGGAGC
ACCATGAGGAGTGTGACTGTGTGTGCAGAGGGAGCACAGGAGGA**TAG**CCGCATCACCACCAGCAGCTCTTGCCCA
GAGCTGTGCAGTGCAGTGGCTGATTCTATTAGAGAACGTATGCGTTATCTCCATCCTTAATCTCAGTTGTTTGCT
TCAAGGACCTTTCATCTTCAGGATTTACAGTGCATTCTGAAAGAGGAGACATCAAACAGAATTAGGAGTTGTGCA
ACAGCTCTTTTGAGAGGAGGCCTAAAGGACAGGAGAAAAGGTCTTCAATCGTGGAAAGAAAATTAAATGTTGTAT
TAAATAGATCACCAGCTAGTTTCAGAGTTACCATGTACGTATTCCACTAGCTGGGTTCTGTATTTCAGTTCTTTC
GATACGGCTTAGGGTAATGTCAGTACAGGAAAAAAACTGTGCAAGTGAGCACCTGATTCCGTTGCCTTGCTTAAC
TCTAAAGCTCCATGTCCTGGGCCTAAAATCGTATAAAATCTGGATTTTTTTTTTTTTTTTGCTCATATTCACAT
ATGTAAACCAGAACATTCTATGTACTACAAACCTGGTTTTAAAAAGGAACTATGTTGCTATGAATTAAACTTGT
GTCATGCTGATAGGACAGACTGGATTTTTCATATTTCTTATTAAAATTTCTGCCATTTAGAAGAAGAGAACTACA
TTCATGGTTTGGAAGAGATAAACCTGAAAAGAAGAGTGGCCTTATCTTCACTTTATCGATAAGTCAGTTTATTTG
TTTCATTGTGTACATTTTTATATTCTCCTTTTGACATTATAACTGTTGGCTTTTCTAATCTTGTTAAATATATCT
ATTTTTACCAAAGGTATTTAATATTCTTTTTTATGACAACTTAGATCAACTATTTTTAGCTTGGTAAATTTTTCT
AAACACAATTGTTATAGCCAGAGGAACAAAGATGATATAAAATATTGTTGCTCTGACAAAAATACATGTATTTCA
TTCTCGTATGGTGCTAGAGTTAGATTAATCTGCATTTTAAAAAACTGAATTGGAATAGAATTGGTAAGTTGCAAA
GACTTTTTGAAAATAATTAAATTATCATATCTTCCATTCCTGTTATTGGAGATGAAAATAAAAAGCAACTTATGA
AAGTAGACATTCAGATCCAGCCATTACTAACCTATTCCTTTTTTGGGGAAATCTGAGCCTAGCTCAGAAAAACAT
AAAGCACCTTGAAAAAGACTTGGCAGCTTCCTGATAAAGCGTGCTGTGCTGTGCAGTAGGAACACATCCTATTTA
TTGTGATGTTGTGGTTTTATTATCTTAAACTCTGTTCCATACACTTGTATAAATACATGGATATTTTTATGTACA
GAAGTATGTCTCTTAACCAGTTCACTTATTGTACTCTGGCAATTTAAAAGAAAATCAGTAAAATATTTTGCTTGT
AAAATGCTTAATATNGTGCCTAGGTTATGTGGTGACTATTTGAATCAAAAATGTATTGAATCATCAAATAAAAGA
ATGTGGCTATTTTGGGGAGAAAATTAAAAAAAAAAAAAAAAAAAAAGGTTTAGGATAACAGGGTAATGCGGCC

# FIGURE 286

MSLFGLLLLTSALAGQRQGTQAESNLSSKFQFSSNKEQNGVQDPQHERIITVSTNGSIHSPRF
PHTYPRNTVLVWRLVAVEENVWIQLTFDERFGLEDPEDDICKYDFVEVEEPSDGTILGRWCGS
GTVPGKQISKGNQIRIRFVSDEYFPSEPGFCIHYNIVMPQFTEAVSPSVLPPSALPLDLLNNA
ITAFSTLEDLIRYLEPERWQLDLEDLYRPTWQLLGKAFVFGRKSRVVDLNLLTEEVRLYSCTP
RNFSVSIREELKRTDTIFWPGCLLVKRCGGNCACCLHNCNECQCVPSKVTKKYHEVLQLRPKT
GVRGLHKSLTDVALEHHEECDCVCRGSTGG

**Important features:**

**signal sequence:**

Amino acids 1-14

**N-glycosylation sites:**

Amino acids 25-29;55-59;254-258

**N-myristoylation sites:**

Amino acids 15-21;117-123;127-133;281-287;282-288;319-325

**Amidation site:**

Amino acids 229-233

# FIGURE 287

CAGCGCTGACTGCGCCGCGGAGAAAGCCAGTGGGAACCCAGACCCATAGGAGACCCGCGTCCC
CGCTCGGCCTGGCCAGGCCCCGCGCT**ATG**GAGTTCCTCTGGGCCCCTCTCTTGGGTCTGTGCT
GCAGTCTGGCCGCTGCTGATCGCCACACCGTCTTCTGGAACAGTTCAAATCCCAAGTTCCGGA
ATGAGGACTACACCATACATGTGCAGCTGAATGACTACGTGGACATCATCTGTCCGCACTATG
AAGATCACTCTGTGGCAGACGCTGCCATGGAGCAGTACATACTGTACCTGGTGGAGCATGAGG
AGTACCAGCTGTGCCAGCCCCAGTCCAAGGACCAAGTCCGCTGGCAGTGCAACCGGCCCAGTG
CCAAGCATGGCCCGGAGAAGCTGTCTGAGAAGTTCCAGCGCTTCACACCTTTCACCCTGGGCA
AGGAGTTCAAAGAAGGACACAGCTACTACTACATCTCCAAACCCATCCACCAGCATGAAGACC
GCTGCTTGAGGTTGAAGGTGACTGTCAGTGGCAAAATCACTCACAGTCCTCAGGCCCATGACA
ATCCACAGGAGAAGAGACTTGCAGCAGATGACCCAGAGGTGCGGGTTCTACATAGCATCGGTC
ACAGTGCTGCCCCACGCCTCTTCCCACTTGCCTGGACTGTGCTGCTCCTTCCACTTCTGCTGC
TGCAAACCCCG**TGA**AGGTGTGTGCCACACCTGGCCTTAAAGAGGGACAGGCTGAAGAGAGGGA
CAGGCACTCCAAACCTGTCTTGGGGCCACTTTCAGAGCCCCCAGCCCTGGGAACCACTCCCAC
CACAGGCATAAGCTATCACCTAGCAGCCTCAAAACGGGTCAATATTAAGGTTTTCAACCGGAA
GGAGGCCAACCAGCCCGACAGTGCCATCCCCACCTTCACCTCGGAGGGATGGAGAAAGAAGTG
GAGACAGTCCTTTCCCACCATTCCTGCCTTTAAGCCAAAGAAACAAGCTGTGCAGGCATGGTC
CCTTAAGGCACAGTGGGAGCTGAGCTGGAAGGGGCCACGTGGATGGGCAAAGCTTGTCAAAGA
TGCCCCCTTCAGGAGAGAGCCAGGATGCCCAGATGAACTGACTGAAGGAAAAGCAAGAAACAG
TTTCTTGCTTGGAAGCCAGGTACAGGAGAGGCAGCATGCTTGGGCTGACCCAGCATCTCCCAG
CAAGACCTCATCTGTGGAGCTGCCACAGAGAAGTTTGTAGCCAGGTACTGCATTCTCTCCCAT
CCTGGGGCAGCACTCCCCAGAGCTGTGCCAGCAGGGGGCTGTGCCAACCTGTTCTTAGAGTG
TAGCTGTAAGGGCAGTGCCCATGTGTACATTCTGCCTAGAGTGTAGCCTAAAGGGCAGGGCCC
ACGTGTATAGTATCTGTATATAAGTTGCTGTGTGTCTGTCCTGATTTCTACAACTGGAGTTTT
TTTATACAATGTTCTTTGTCTCAAAATAAAGCAATGTGTTTTTTCGG

# FIGURE 288

MEFLWAPLLGLCCSLAAADRHTVFWNSSNPKFRNEDYTIHVQLNDYVDIICPHYEDHSADAAM
EQYILYLVEHEEYQLCQPQSKDQVRWQCNRPSAKHGPEKLSEKFQRFTPFTLGKEFKEGHSYY
YISKPIHQHEDRCLRLKVTVSGKITHSPQAHDNPQEKRLAADDPEVRVLHSIGHSAAPRLFPL
AWTVLLLPLLLLQTP

**Important features:**

**Signal sequence:**

Amino acids 1-17

**N-glycosylation site:**

Amino acids 26-30

**Tyrosine kinase phosphorylation site:**

Amino acids 118-127

**N-myristoylation site:**

Amino acids 10-16

# FIGURE 289

CGGACGCGTGGGCGGACGCGTGGGCGGCCCACGGCGCCCGCGGGCTGGGGCGGTCGCTTCTTC
CTTCTCCGTGGCCTACGAGGGTCCCCAGCCTGGGTAAAG**ATG**GCCCCATGGCCCCCGAAGGGC
CTAGTCCCAGCTGTGCTCTGGGGCCTCAGCCTCTTCCTCAACCTCCCAGGACCTATCTGGCTC
CAGCCCTCTCCACCTCCCCAGTCTTCTCCCCCGCCTCAGCCCCATCCGTGTCATACCTGCCGG
GGACTGGTTGACAGCTTTAACAAGGGCCTGGAGAGAACCATCCGGGACAACTTTGGAGGTGGA
AACACTGCCTGGGAGGAAGAGAATTTGTCCAAATACAAAGACAGTGAGACCCGCCTGGTAGAG
GTGCTGGAGGGTGTGTGCAGCAAGTCAGACTTCGAGTGCCACCGCCTGCTGGAGCTGAGTGAG
GAGCTGGTGGAGAGCTGGTGGTTTCACAAGCAGCAGGAGGCCCCGGACCTCTTCCAGTGGCTG
TGCTCAGATTCCCTGAAGCTCTGCTGCCCCGCAGGCACCTTCGGGCCCTCCTGCCTTCCCTGT
CCTGGGGGAACAGAGAGGCCCTGCGGTGGCTACGGGCAGTGTGAAGGAGAAGGGACACGAGGG
GGCAGCGGGCACTGTGACTGCCAAGCCGGCTACGGGGGTGAGGCCTGTGGCCAGTGTGGCCTT
GGCTACTTTGAGGCAGAACGCAACGCCAGCCATCTGGTATGTTCGGCTTGTTTTGGCCCCTGT
GCCCGATGCTCAGGACCTGAGGAATCAAACTGTTTGCAATGCAAGAAGGGCTGGGCCCTGCAT
CACCTCAAGTGTGTAGACATTGATGAGTGTGGCACAGAGGGAGCCAACTGTGGAGCTGACCAA
TTCTGCGTGAACACTGAGGGCTCCTATGAGTGCCGAGACTGTGCCAAGGCCTGCCTAGGCTGC
ATGGGGGCAGGGCCAGGTCGCTGTAAGAAGTGTAGCCCTGGCTATCAGCAGGTGGGCTCCAAG
TGTCTCGATGTGGATGAGTGTGAGACAGAGGTGTGTCCGGGAGAGAACAAGCAGTGTGAAAAC
ACCGAGGGCGGTTATCGCTGCATCTGTGCCGAGGGCTACAAGCAGATGGAAGGCATCTGTGTG
AAGGAGCAGATCCCAGAGTCAGCAGGCTTCTTCTCAGAGATGACAGAAGACGAGTTGGTGGTG
CTGCAGCAGATGTTCTTTGGCATCATCATCTGTGCACTGGCCACGCTGGCTGCTAAGGGCGAC
TTGGTGTTCACCGCCATCTTCATTGGGGCTGTGGCGGCCATGACTGGCTACTGGTTGTCAGAG
CGCAGTGACCGTGTGCTGGAGGGCTTCATCAAGGGCAGA**TAA**TCGCGGCCACCACCTGTAGGA
CCTCCTCCCACCCACGCTGCCCCCAGAGCTTGGGCTGCCCTCCTGCTGGACACTCAGGACAGC
TTGGTTTATTTTTGAGAGTGGGGTAAGCACCCCTACCTGCCTTACAGAGCAGCCCAGGTACCC
AGGCCCGGGCAGACAAGGCCCCTGGGGTAAAAAGTAGCCCTGAAGGTGGATACCATGAGCTCT
TCACCTGGCGGGGACTGGCAGGCTTCACAATGTGTGAATTTCAAAAGTTTTTCCTTAATGGTG
GCTGCTAGAGCTTTGGCCCCTGCTTAGGATTAGGTGGTCCTCACAGGGGTGGGGCCATCACAG
CTCCCTCCTGCCAGCTGCATGCTGCCAGTTCCTGTTCTGTGTTCACCACATCCCCACACCCCA
TTGCCACTTATTTATTCATCTCAGGAAATAAAGAAAGGTCTTGGAAAGTTAAAAAAAAAAAAA
AAAAAAAAAAA

# FIGURE 290

MAPWPPKGLVPAVLWGLSLFLNLPGPIWLQPSPPPQSSPPPQPHPCHTCRGLVDSFNKGLERT
IRDNFGGGNTAWEEENLSKYKDSETRLVEVLEGVCSKSDFECHRLLELSEELVESWWFHKQQE
APDLFQWLCSDSLKLCCPAGTFGPSCLPCPGGTERPCGGYGQCEGEGTRGGSGHCDCQAGYGG
EACGQCGLGYFEAERNASHLVCSACFGPCARCSGPEESNCLQCKKGWALHHLKCVDIDECGTE
GANCGADQFCVNTEGSYECRDCAKACLGCMGAGPGRCKKCSPGYQQVGSKCLDVDECETEVCP
GENKQCENTEGGYRCICAEGYKQMEGICVKEQIPESAGFFSEMTEDELVVLQQMFFGIIICAL
ATLAAKGDLVFTAIFIGAVAAMTGYWLSERSDRVLEGFIKGR

**Important features:**

**Signal sequence:**

Amino acids 1-29

**Transmembrane domain:**

Amino acids 342-392

**N-glycosylation sites:**

Amino acids 79-83;205-209

**cAMP- and cGMP-dependent protein kinase phosphorylation site:**

Amino acids 290-294

**Aspartic acid and asparagine hydroxylation site:**

Amino acids 321-333

**EGF-like domain cysteine pattern signature:**

Amino acids 181-193

# FIGURE 291

CAGGTCCAACTGCACCTCGGTTCTATCGATTGAATTCCCCGGGGATCCTCTAGAGATCCCTCGACCTCGACCCAC
GCGTCCGAACACAGGTCCTTGTTGCTGCAGAGAAGCAGTTGTTTTGCTGGAAGGAGGGAGTGCGCGGGCTGCCCC
GGGCTCCTCCCTGCCGCCTCCTCTCAGTGGATGGTTCCAGGCACCCTGTCTGGGGCAGGGAGGGCACAGGCCTGC
ACATCGAAGGTGGGGTGGGACCAGGCTGCCCCTCGCCCCAGCATCCAAGTCCTCCCTTGGGCGCCCGTGGCCCTG
CAGACTCTCAGGGCTAAGGTCCTCTGTTGCTTTTTGGTTCCACCTTAGAAGAGGCTCCGCTTGACTAAGAGTAGC
TTGAAGGAGGCACC**ATG**CAGGAGCTGCATCTGCTCTGGTGGGCGCTTCTCCTGGGCCTGGCTCAGGCCTGCCCTG
AGCCCTGCGACTGTGGGGAAAAGTATGGCTTCCAGATCGCCGACTGTGCCTACCGCGACCTAGAATCCGTGCCGC
CTGGCTTCCCGGCCAATGTGACTACACTGAGCCTGTCAGCCAACCGGCTGCCAGGCTTGCCGGAGGGTGCCTTCA
GGGAGGTGCCCCTGCTGCAGTCGCTGTGGCTGGCACACAATGAGATCCGCACGGTGGCCGCCGGAGCCCTGGCCT
CTCTGAGCCATCTCAAGAGCCTGGACCTCAGCCACAATCTCATCTCTGACTTTGCCTGGAGCGACCTGCACAACC
TCAGTGCCCTCCAATTGCTCAAGATGGACAGCAACGAGCTGACCTTCATCCCCCGCGACGCCTTCCGCAGCCTCC
GTGCTCTGCGCTCGCTGCAACTCAACCACAACCGCTTGCACACATTGGCCGAGGGCACCTTCACCCCGCTCACCG
CGCTGTCCCACCTGCAGATCAACGAGAACCCCTTCGACTGCACCTGCGGCATCGTGTGGCTCAAGACATGGGCCC
TGACCACGGCCGTGTCCATCCCGGAGCAGGACAACATCGCCTGCACCTCACCCCATGTGCTCAAGGGTACACCGC
TGAGCCGCCTGCCGCCACTGCCATGCTCGGCGCCCTCAGTGCAGCTCAGCTACCAACCCAGCCAGGATGGTGCCG
AGCTGCGGCCTGGTTTTGTGCTGGCACTGCACTGTGATGTGGACGGGCAGCCGGCCCCTCAGCTTCACTGGCACA
TCCAGATACCCAGTGGCATTGTGGAGATCACCAGCCCCAACGTGGGCACTGATGGGCGTGCCCTGCCTGGCACCC
CTGTGGCCAGCTCCCAGCCGCGCTTCCAGGCCTTTGCCAATGGCAGCCTGCTTATCCCCGACTTTGGCAAGCTGG
AGGAAGGCACCTACAGCTGCCTGGCCACCAATGAGCTGGGCAGTGCTGAGAGCTCAGTGGACGTGGCACTGGCCA
CGCCCGGTGAGGGTGGTGAGGACACACTGGGGCGCAGGTTCCATGGCAAAGCGGTTGAGGGAAAGGGCTGCTATA
CGGTTGACAACGAGGTGCAGCCATCAGGGCCGGAGGACAATGTGGTCATCATCTACCTCAGCCGTGCTGGGAACC
CTGAGGCTGCAGTCGCAGAAGGGGTCCCTGGGCAGCTGCCCCCAGGCCTGCTCCTGCTGGGCCAAAGCCTCCTCC
TCTTCTTCTTCCTCACCTCCTTC**TAG**CCCCACCCAGGGCTTCCCTAACTCCTCCCCTTGCCCCTACCAATGCCCC
TTTAAGTGCTGCAGGGGTCTGGGGTTGGCAACTCCTGAGGCCTGCATGGGTGACTTCACATTTTCCTACCTCTCC
TTCTAATCTCTTCTAGAGCACCTGCTATCCCCAACTTCTAGACCTGCTCCAAACTAGTGACTAGGATAGAATTTG
ATCCCCTAACTCACTGTCTGCGGTGCTCATTGCTGCTAACAGCATTGCCTGTGCTCTCCTCTCAGGGGCAGCATG
CTAACGGGGCGACGTCCTAATCCAACTGGGAGAAGCCTCAGTGGTGGAATTCCAGGCACTGTGACTGTCAAGCTG
GCAAGGGCCAGGATTGGGGGAATGGAGCTGGGGCTTAGCTGGGAGGTGGTCTGAAGCAGACAGGGAATGGGAGAG
GAGGATGGGAAGTAGACAGTGGCTGGTATGGCTCTGAGGCTCCCTGGGGCCTGCTCAAGCTCCTCCTGCTCCTTG
CTGTTTTCTGATGATTTGGGGGCTTGGGAGTCCCTTTGTCCTCATCTGAGACTGAAATGTGGGGATCCAGGATGG
CCTTCCTTCCTCTTACCCTTCCTCCCTCAGCCTGCAACCTCTATCCTGGAACCTGTCCTCCCTTTCTCCCCAACT
ATGCATCTGTTGTCTGCTCCTCTGCAAAGGCCAGCCAGCTTGGGAGCAGCAGAGAAATAAACAGCATTTCTGATG
CCAAAAAAAAAAAAAAAAAAGGGCGGCCGCGACTCTAGAGTCGACCT

# FIGURE 292

MQELHLLWWALLLGLAQACPEPCDCGEKYGFQIADCAYRDLESVPPGFPANVTTLSLSANRLP
GLPEGAFREVPLLQSLWLAHNEIRTVAAGALASLSHLKSLDLSHNLISDFAWSDLHNLSALQL
LKMDSNELTFIPRDAFRSLRALRSLQLNHNRLHTLAEGTFTPLTALSHLQINENPFDCTCGIV
WLKTWALTTAVSIPEQDNIACTSPHVLKGTPLSRLPPLPCSAPSVQLSYQPSQDGAELRPGFV
LALHCDVDGQPAPQLHWHIQIPSGIVEITSPNVGTDGRALPGTPVASSQPRFQAFANGSLLIP
DFGKLEEGTYSCLATNELGSAESSVDVALATPGEGGEDTLGRRFHGKAVEGKGCYTVDNEVQP
SGPEDNVVIIYLSRAGNPEAAVAEGVPGQLPPGLLLLGQSLLLFFFLTSF

**Important features:**

**Signal peptide:**
amino acids 1-18

**Transmembrane domain:**
amino acids 403-418

**N-glycosylation sites:**
Amino acids 51-55,120-124,309-313

**Tyrosine kinase phosphorylation site:**
amino acids 319-326

**N-myristoylation sites:**
amino acids 14-20,64-70,92-98,218-224,294-300,323-329,334-340, 350-356,394-400

**Amidation site:**
amino acids 355-359

**Leucine Rich Repeat:**
amino acids 51-74,75-98, 99-122,123-146,147-170

**Leucine rich repeat C-terminal domain:**
amino acids 180-230

# FIGURE 293

ACTTGGAGCAAGCGGCGGCGGCGGAGACAGAGGCAGAGGCAGAAGCTGGGGCTCCGTCCTCGCCTCCCACGAGCG
ATCCCCGAGGAGAGCCGCGGCCCTCGGCGAGGCGAAGAGGCCGACGAGGAAGACCCGGGTGGCTGCGCCCCTGCC
TCGCTTCCCAGGCGCCGGCGGCTGCAGCCTTGCCCCTCTTGCTCGCCTTGAAA**ATG**GAAAAGATGCTCGCAGGCT
GCTTTCTGCTGATCCTCGGACAGATCGTCCTCCTCCCTGCCGAGGCCAGGGAGCGGTCACGTGGGAGGTCCATCT
CTAGGGGCAGACACGCTCGGACCCACCCGCAGACGGCCCTTCTGGAGAGTTCCTGTGAGAACAAGCGGGCAGACC
TGGTTTTCATCATTGACAGCTCTCGCAGTGTCAACACCCATGACTATGCAAAGGTCAAGGAGTTCATCGTGGACA
TCTTGCAATTCTTGGACATTGGTCCTGATGTCACCCGAGTGGGCCTGCTCCAATATGGCAGCACTGTCAAGAATG
AGTTCTCCCTCAAGACCTTCAAGAGGAAGTCCGAGGTGGAGCGTGCTGTCAAGAGGATGCGGCATCTGTCCACGG
GCACCATGACTGGGCTGGCCATCCAGTATGCCCTGAACATCGCATTCTCAGAAGCAGAGGGGGCCCGGCCCCTGA
GGGAGAATGTGCCACGGGTCATAATGATCGTGACAGATGGGAGACCTCAGGACTCCGTGGCCGAGGTGGCTGCTA
AGGCACGGGACACGGGCATCCTAATCTTTGCCATTGGTGTGGGCCAGGTAGACTTCAACACCTTGAAGTCCATTG
GGAGTGAGCCCCATGAGGACCATGTCTTCCTTGTGGCCAATTTCAGCCAGATTGAGACGCTGACCTCCGTGTTCC
AGAAGAAGTTGTGCACGGCCCACATGTGCAGCACCCTGGAGCATAACTGTGCCCACTTCTGCATCAACATCCCTG
GCTCATACGTCTGCAGGTGCAAACAAGGCTACATTCTCAACTCGGATCAGACGACTTGCAGAATCCAGGATCTGT
GTGCCATGGAGGACCACAACTGTGAGCAGCTCTGTGTGAATGTGCCGGGCTCCTTCGTCTGCCAGTGCTACAGTG
GCTACGCCCTGGCTGAGGATGGGAAGAGGTGTGTGGCTGTGGACTACTGTGCCTCAGAAAACCACGGATGTGAAC
ATGAGTGTGTAAATGCTGATGGCTCCTACCTTTGCCAGTGCCATGAAGGATTTGCTCTTAACCCAGATGAAAAAA
CGTGCACAAGGATCAACTACTGTGCACTGAACAAACCGGGCTGTGAGCATGAGTGCGTCAACATGGAGGAGAGCT
ACTACTGCCGCTGCCACCGTGGCTACACTCTGGACCCCAATGGCAAAACCTGCAGCCGAGTGGACCACTGTGCAC
AGCAGGACCATGGCTGTGAGCAGCTGTGTCTGAACACGGAGGATTCCTTCGTCTGCCAGTGCTCAGAAGGCTTCC
TCATCAACGAGGACCTCAAGACCTGCTCCCGGGTGGATTACTGCCTGCTGAGTGACCATGGTTGTGAATACTCCT
GTGTCAACATGGACAGATCCTTTGCCTGTCAGTGTCCTGAGGGACACGTGCTCCGCAGCGATGGGAAGACGTGTG
CAAAATTGGACTCTTGTGCTCTGGGGGACCACGGTTGTGAACATTCGTGTGTAAGCAGTGAAGATTCGTTTGTGT
GCCAGTGCTTTGAAGGTTATATACTCCGTGAAGATGGAAAAACCTGCAGAAGGAAAGATGTCTGCCAAGCTATAG
ACCATGGCTGTGAACACATTTGTGTGAACAGTGACGACTCATACACGTGCGAGTGCTTGGAGGGATTCCGGCTCG
CTGAGGATGGGAAACGCTGCCGAAGGAAGGATGTCTGCAAATCAACCCACCATGGCTGCGAACACATTTGTGTTA
ATAATGGGAATTCCTACATCTGCAAATGCTCAGAGGGATTTGTTCTAGCTGAGGACGGAAGACGGTGCAAGAAAT
GCACTGAAGGCCCAATTGACCTGGTCTTTGTGATCGATGGATCCAAGAGTCTTGGAGAAGAGAATTTTGAGGTCG
TGAAGCAGTTTGTCACTGGAATTATAGATTCCTTGACAATTTCCCCCAAAGCCGCTCGAGTGGGGCTGCTCCAGT
ATTCCACACAGGTCCACACAGAGTTCACTCTGAGAAACTTCAACTCAGCCAAAGACATGAAAAAAGCCGTGGCCC
ACATGAAATACATGGGAAAGGGCTCTATGACTGGGCTGGCCCTGAAACACATGTTTGAGAGAAGTTTTACCCAAG
GAGAAGGGGCCAGGCCCCTTTCCACAAGGGTGCCCAGAGCAGCCATTGTGTTCACCGACGGACGGGCTCAGGATG
ACGTCTCCGAGTGGGCCAGTAAAGCCAAGGCCAATGGTATCACTATGTATGCTGTTGGGGTAGGAAAAGCCATTG
AGGAGGAACTACAAGAGATTGCCTCTGAGCCCACAAACAAGCATCTCTTCTATGCCGAAGACTTCAGCACAATGG
ATGAGATAAGTGAAAAACTCAAGAAAGGCATCTGTGAAGCTCTAGAAGACTCCGATGGAAGACAGGACTCTCCAG
CAGGGGAACTGCCAAAAACGGTCCAACAGCCAACAGAATCTGAGCCAGTCACCATAAATATCCAAGACCTACTTT
CCTGTTCTAATTTTGCAGTGCAACACAGATATCTGTTTGAAGAAGACAATCTTTTACGGTCTACACAAAAGCTTT
CCCATTCAACAAAACCTTCAGGAAGCCCTTTGGAAGAAAAACACGATCAATGCAAATGTGAAAACCTTATAATGT
TCCAGAACCTTGCAAACGAAGAAGTAAGAAAATTAACACAGCGCTTAGAAGAAATGACACAGAGAATGGAAGCCC
TGGAAAATCGCCTGAGATACAGA**TGA**AGATTAGAAATCGCGACACATTTGTAGTCATTGTATCACGGATTACAAT
GAACGCAGTGCAGAGCCCCAAAGCTCAGGCTATTGTTAAATCAATAATGTTGTGAAGTAAAACAATCAGTACTGA
GAAACCTGGTTTGCCACAGAACAAAGACAAGAAGTATACACTAACTTGTATAAATTTATCTAGGAAAAAAATCCT
TCAGAATTCTAAGATGAATTTACCAGGTGAGAATGAATAAGCTATGCAAGGTATTTTGTAATATACTGTGGACAC
AACTTGCTTCTGCCTCATCCTGCCTTAGTGTGCAATCTCATTTGACTATACGATAAAGTTTGCACAGTCTTACTT
CTGTAGAACACTGGCCATAGGAAATGCTGTTTTTTTGTACTGGACTTTACCTTGATATATGTATATGGATGTATG
CATAAAATCATAGGACATATGTACTTGTGGAACAAGTTGGATTTTTTATACAATATTAAAATTCACCACTTCAG

# FIGURE 294

MEKMLAGCFLLILGQIVLLPAEARERSRGRSISRGRHARTHPQTALLESSCENKRADLVFIID
SSRSVNTHDYAKVKEFIVDILQFLDIGPDVTRVGLLQYGSTVKNEFSLKTFKRKSEVERAVKR
MRHLSTGTMTGLAIQYALNIAFSEAEGARPLRENVPRVIMIVTDGRPQDSVAEVAAKARDTGI
LIFAIGVGQVDFNTLKSIGSEPHEDHVFLVANFSQIETLTSVFQKKLCTAHMCSTLEHNCAHF
CINIPGSYVCRCKQGYILNSDQTTCRIQDLCAMEDHNCEQLCVNVPGSFVCQCYSGYALAEDG
KRCVAVDYCASENHGCEHECVNADGSYLCQCHEGFALNPDEKTCTRINYCALNKPGCEHECVN
MEESYYCRCHRGYTLDPNGKTCSRVDHCAQQDHGCEQLCLNTEDSFVCQCSEGFLINEDLKTC
SRVDYCLLSDHGCEYSCVNMDRSFACQCPEGHVLRSDGKTCAKLDSCALGDHGCEHSCVSSED
SFVCQCFEGYILREDGKTCRRKDVCQAIDHGCEHICVNSDDSYTCECLEGFRLAEDGKRCRRK
DVCKSTHHGCEHICVNNGNSYICKCSEGFVLAEDGRRCKKCTEGPIDLVFVIDGSKSLGEENF
EVVKQFVTGIIDSLTISPKAARVGLLQYSTQVHTEFTLRNFNSAKDMKKAVAHMKYMGKGSMT
GLALKHMFERSFTQGEGARPLSTRVPRAAIVFTDGRAQDDVSEWASKAKANGITMYAVGVGKA
IEEELQEIASEPTNKHLFYAEDFSTMDEISEKLKKGICEALEDSDGRQDSPAGELPKTVQQPT
ESEPVTINIQDLLSCSNFAVQHRYLFEEDNLLRSTQKLSHSTKPSGSPLEEKHDQCKCENLIM
FQNLANEEVRKLTQRLEEMTQRMEALENRLRYR

**Important features:**
**Signal sequence:**
Amino acids 1-23

**N-glycosylation site:**
Amino acids 221-225

**cAMP- and cGMP-dependent protein kinase phosphorylation sites:**
Amino acids 115-119;606-610;892-896

**N-myristoylation sites:**
Amino acids 133-139;258-264;299-305;340-346;453-459;494-500;
639-645;690-694;
752-758;792-798

**Amidation sites:**
Amino acids 314-318;560-564;601-605

**Aspartic acid and asparagine hydroxylation sites:**
Amino acids 253-265;294-306;335-347;376-388;417-429;
458-470;540-552;581-593

# FIGURE 295

GGCCGGAGCAGCACGGCCGCAGGACCTGGAGCTCCGGCTGCGTCTTCCCGCAGCGCTACCCGC
C**ATG**CGCCTGCCGCGCCGGGCCGCGCTGGGGCTCCTGCCGCTTCTGCTGCTGCTGCCGCCCGC
GCCGGAGGCCGCCAAGAAGCCGACGCCCTGCCACCGGTGCCGGGGGCTGGTGGACAAGTTTAA
CCAGGGGATGGTGGACACCGCAAAGAAGAACTTTGGCGGCGGGAACACGGCTTGGGAGGAAAA
GACGCTGTCCAAGTACGAGTCCAGCGAGATTCGCCTGCTGGAGATCCTGGAGGGGCTGTGCGA
GAGCAGCGACTTCGAATGCAATCAGATGCTAGAGGCGCAGGAGGAGCACCTGGAGGCCTGGTG
GCTGCAGCTGAAGAGCGAATATCCTGACTTATTCGAGTGGTTTTGTGTGAAGACACTGAAAGT
GTGCTGCTCTCCAGGAACCTACGGTCCCGACTGTCTCGCATGCCAGGGCGGATCCCAGAGGCC
CTGCAGCGGGAATGGCCACTGCAGCGGAGATGGGAGCAGACAGGGCGACGGGTCCTGCCGGTG
CCACATGGGGTACCAGGGCCCGCTGTGCACTGACTGCATGGACGGCTACTTCAGCTCGCTCCG
GAACGAGACCCACAGCATCTGCACAGCCTGTGACGAGTCCTGCAAGACGTGCTCGGGCCTGAC
CAACAGAGACTGCGGCGAGTGTGAAGTGGGCTGGGTGCTGGACGAGGGCGCCTGTGTGGATGT
GGACGAGTGTGCGGCCGAGCCGCCTCCCTGCAGCGCTGCGCAGTTCTGTAAGAACGCCAACGG
CTCCTACACGTGCGAAGAGTGTGACTCCAGCTGTGTGGGCTGCACAGGGGAAGGCCCAGGAAA
CTGTAAAGAGTGTATCTCTGGCTACGCGAGGGAGCACGGACAGTGTGCAGATGTGGACGAGTG
CTCACTAGCAGAAAAAACCTGTGTGAGGAAAAACGAAAACTGCTACAATACTCCAGGGAGCTA
CGTCTGTGTGTGTCCTGACGGCTTCGAAGAAACGGAAGATGCCTGTGTGCCGCCGGCAGAGGC
TGAAGCCACAGAAGGAGAAAGCCCGACACAGCTGCCCTCCCGCGAAGACCTG**TAA**TGTGCCGG
ACTTACCCTTTAAATTATTCAGAAGGATGTCCCGTGGAAAATGTGGCCCTGAGGATGCCGTCT
CCTGCAGTGGACAGCGGCGGGGAGAGGCTGCCTGCTCTCTAACGGTTGATTCTCATTTGTCCC
TTAAACAGCTGCATTTCTTGGTTGTTCTTAAACAGACTTGTATATTTTGATACAGTTCTTTGT
AATAAAATTGACCATTGTAGGTAATCAGGAGGAAAAAAAAA

# FIGURE 296

MRLPRRAALGLLPLLLLLPPAPEAAKKPTPCHRCRGLVDKFNQGMVDTAKKNFGGGNTAWEEK
TLSKYESSEIRLLEILEGLCESSDFECNQMLEAQEEHLEAWWLQLKSEYPDLFEWFCVKTLKV
CCSPGTYGPDCLACQGGSQRPCSGNGHCSGDGSRQGDGSCRCHMGYQGPLCTDCMDGYFSSLR
NETHSICTACDESCKTCSGLTNRDCGECEVGWVLDEGACVDVDECAAEPPPCSAAQFCKNANG
SYTCEECDSSCVGCTGEGPGNCKECISGYAREHGQCADVDECSLAEKTCVRKNENCYNTPGSY
VCVCPDGFEETEDACVPPAEAEATEGESPTQLPSREDL

**Important features:**

**Signal peptide:**
Amino acids 1-24

**N-glycosylation sites:**
Amino acids 190-194;251-255

**Glycosaminoglycan attachment sites:**
Amino acids 149-153;155-159

**cAMP- and cGMP-dependent protein kinase phosphorylation site:**
Amino acids 26-30

**Tyrosine kinase phosphorylation site:**
Amino acids 303-310

**N-myristoylation sites:**
Amino acids 44-50;54-60;55-61;81-87;150-156;158-164;164-170;
252-258;313-319

**Aspartic acid and asparagine hydroxylation site:**
Amino acids 308-320

**EGF-like domain cysteine pattern signature:**
Amino acids 166-178

**Leucine zipper pattern:**
Amino acids 94-116

# FIGURE 297

GACATCGGAGGTGGGCTAGCACTGAAACTGCTTTTCAAGACGAGGAAGAGGAGGAGAAAGAGAAAGAAGAGGAAG
ATGTTGGGCAACATTTATTTAACATGCTCCACAGCCCGGACCCTGGCATCATGCTGCTATTCCTGCAAATACTGA
AGAAGCATGGGATTTAAATATTTTACTTCTAAATAAATGAATTACTCAATCTCCTATGACCATCTATACATACTC
CACCTTCAAAAAGTACATCAATATTATATCATTAAGGAAATAGTAACCTTCTCTTCTCCAATATGCATGACATTT
TTGGACAATGCAATTGTGGCACTGGCACTTATTTCAGTGAAGAAAAACTTTGTGGTTCTATGGCATTCATCATTT
GACAAATGCAAGCATCTTCCTTATCAATCAGCTCCTATTGAACTTACTAGCACTGACTGTGGAATCCTTAAGGGC
CCATTACATTTCTGAAGAAGAAAGCTAAG**ATG**AAGGACATGCCACTCCGAATTCATGTGCTACTTGGCCTAGCTA
TCACTACACTAGTACAAGCTGTAGATAAAAAAGTGGATTGTCCACGGTTATGTACGTGTGAAATCAGGCCTTGGT
TTACACCCAGATCCATTTATATGGAAGCATCTACAGTGGATTGTAATGATTTAGGTCTTTTAACTTTCCCAGCCA
GATTGCCAGCTAACACACAGATTCTTCTCCTACAGACTAACAATATTGCAAAAATTGAATACTCCACAGACTTTC
CAGTAAACCTTACTGGCCTGGATTTATCTCAAAACAATTTATCTTCAGTCACCAATATTAATGTAAAAAAGATGC
CTCAGCTCCTTTCTGTGTACCTAGAGGAAAACAAACTTACTGAACTGCCTGAAAAATGTCTGTCCGAACTGAGCA
ACTTACAAGAACTCTATATTAATCACAACTTGCTTTCTACAATTTCACCTGGAGCCTTTATTGGCCTACATAATC
TTCTTCGACTTCATCTCAATTCAAATAGATTGCAGATGATCAACAGTAAGTGGTTTGATGCTCTTCCAAATCTAG
AGATTCTGATGATTGGGGAAAATCCAATTATCAGAATCAAAGACATGAACTTTAAGCCTCTTATCAATCTTCGCA
GCCTGGTTATAGCTGGTATAAACCTCACAGAAATACCAGATAACGCCTTGGTTGGACTGGAAAACTTAGAAAGCA
TCTCTTTTTACGATAACAGGCTTATTAAAGTACCCCATGTTGCTCTTCAAAAAGTTGTAAATCTCAAATTTTTGG
ATCTAAATAAAAATCCTATTAATAGAATACGAAGGGGTGATTTTAGCAATATGCTACACTTAAAAGAGTTGGGGA
TAAATAATATGCCTGAGCTGATTTCCATCGATAGTCTTGCTGTGGATAACCTGCCAGATTTAAGAAAAATAGAAG
CTACTAACAACCCTAGATTGTCTTACATTCACCCCAATGCATTTTTCAGACTCCCCAAGCTGGAATCACTCATGC
TGAACAGCAATGCTCTCAGTGCCCTGTACCATGGTACCATTGAGTCTCTGCCAAACCTCAAGGAAATCAGCATAC
ACAGTAACCCCATCAGGTGTGACTGTGTCATCCGTTGGATGAACATGAACAAAACCAACATTCGATTCATGGAGC
CAGATTCACTGTTTTGCGTGGACCCACCTGAATTCCAAGGTCAGAATGTTCGGCAAGTGCATTTCAGGGACATGA
TGGAAATTTGTCTCCCTCTTATAGCTCCTGAGAGCTTTCCTTCTAATCTAAATGTAGAAGCTGGGAGCTATGTTT
CCTTTCACTGTAGAGCTACTGCAGAACCACAGCCTGAAATCTACTGGATAACACCTTCTGGTCAAAAACTCTTGC
CTAATACCCTGACAGACAAGTTCTATGTCCATTCTGAGGGAACACTAGATATAAATGGCGTAACTCCCAAAGAAG
GGGGTTTATATACTTGTATAGCAACTAACCTAGTTGGCGCTGACTTGAAGTCTGTTATGATCAAAGTGGATGGAT
CTTTTCCACAAGATAACAATGGCTCTTTGAATATTAAAATAAGAGATATTCAGGCCAATTCAGTTTTGGTGTCCT
GGAAAGCAAGTTCTAAAATTCTCAAATCTAGTGTTAAATGGACAGCCTTTGTCAAGACTGAAAATTCTCATGCTG
CGCAAAGTGCTCGAATACCATCTGATGTCAAGGTATATAATCTTACTCATCTGAATCCATCAACTGAGTATAAAA
TTTGTATTGATATTCCCACCATCTATCAGAAAAACAGAAAAAAATGTGTAAATGTCACCACCAAAGGTTTGCACC
CTGATCAAAAAGAGTATGAAAAGAATAATACCACAACACTTATGGCCTGTCTTGGAGGCCTTCTGGGGATTATTG
GTGTGATATGTCTTATCAGCTGCCTCTCTCCAGAAATGAACTGTGATGGTGGACACAGCTATGTGAGGAATTACT
TACAGAAACCAACCTTTGCATTAGGTGAGCTTTATCCTCCTCTGATAAATCTCTGGGAAGCAGGAAAAGAAAAAA
GTACATCACTGAAAGTAAAAGCAACTGTTATAGGTTTACCAACAAATATGTCC**TAA**AAACCACCAAGGAAACCTA
CTCCAAAAATGAAC

# FIGURE 298

MKDMPLRIHVLLGLAITTLVQAVDKKVDCPRLCTCEIRPWFTPRSIYMEASTVDCNDLGLLTF
PARLPANTQILLLQTNNIAKIEYSTDFPVNLTGLDLSQNNLSSVTNINVKKMPQLLSVYLEEN
KLTELPEKCLSELSNLQELYINHNLLSTISPGAFIGLHNLLRLHLNSNRLQMINSKWFDALPN
LEILMIGENPIIRIKDMNFKPLINLRSLVIAGINLTEIPDNALVGLENLESISFYDNRLIKVP
HVALQKVVNLKFLDLNKNPINRIRRGDFSNMLHLKELGINNMPELISIDSLAVDNLPDLRKIE
ATNNPRLSYIHPNAFFRLPKLESLMLNSNALSALYHGTIESLPNLKEISIHSNPIRCDCVIRW
MNMNKTNIRFMEPDSLFCVDPPEFQGQNVRQVHFRDMMEICLPLIAPESFPSNLNVEAGSYVS
FHCRATAEPQPEIYWITPSGQKLLPNTLTDKFYVHSEGTLDINGVTPKEGGLYTCIATNLVGA
DLKSVMIKVDGSFPQDNNGSLNIKIRDIQANSVLVSWKASSKILKSSVKWTAFVKTENSHAAQ
SARIPSDVKVYNLTHLNPSTEYKICIDIPTIYQKNRKKCVNVTTKGLHPDQKEYEKNNTTTLM
ACLGGLLGIIGVICLISCLSPEMNCDGGHSYVRNYLQKPTFALGELYPPLINLWEAGKEKSTS
LKVKATVIGLPTNMS

**Important features:**

**Signal sequence:**
amino acids 1-22

**Transmembrane domain:**
amino acids 633-650

**N-glycosylation site.**
amino acids 93-97, 103-107, 223-227, 382-386, 522-526, 579-583, 608-612, 624-628, 625-629

**Casein kinase II phosphorylation site.**
amino acids 51-55, 95-99, 242-246, 468-472, 487-491

**Tyrosine kinase phosphorylation site.**
amino acids 570-579

**N-myristoylation site.**
amino acids 13-19, 96-102, 158-164, 221-227, 352-358, 437-443, 491-497, 492-498, 634-640, 702-708

**Cell attachment sequence.**
amino acids 277-280

# FIGURE 299

GCTGTGGGAACCTCTCCACGCGCACGAACTCAGCCAACGATTTCTGATAGATTTTTGGGAGTT
TGACCAGAGATGCAAGGGGTGAAGGAGCGCTTCCTACCGTTAGGGAACTCTGGGGACAGAGCG
CCCCGGCCGCCTGATGGCCGAGGCAGGGTGCGACCCAGGACCCAGGACGGCGTCGGGAACCAT
ACC**ATG**GCCCGGATCCCCAAGACCCTAAAGTTCGTCGTCGTCATCGTCGCGGTCCTGCTGCCA
GTCCTAGCTTACTCTGCCACCACTGCCCGGCAGGAGGAAGTTCCCCAGCAGACAGTGGCCCCA
CAGCAACAGAGGCACAGCTTCAAGGGGGAGGAGTGTCCAGCAGGATCTCATAGATCAGAACAT
ACTGGAGCCTGTAACCCGTGCACAGAGGGTGTGGATTACACCAACGCTTCCAACAATGAACCT
TCTTGCTTCCCATGTACAGTTTGTAAATCAGATCAAAAACATAAAAGTTCCTGCACCATGACC
AGAGACACAGTGTGTCAGTGTAAAGAAGGCACCTTCCGGAATGAAAACTCCCCAGAGATGTGC
CGGAAGTGTAGCAGGTGCCCTAGTGGGGAAGTCCAAGTCAGTAATTGTACGTCCTGGGATGAT
ATCCAGTGTGTTGAAGAATTTGGTGCCAATGCCACTGTGGAAACCCCAGCTGCTGAAGAGACA
ATGAACACCAGCCCGGGGACTCCTGCCCCAGCTGCTGAAGAGACAATGAACACCAGCCCAGGG
ACTCCTGCCCCAGCTGCTGAAGAGACAATGACCACCAGCCCGGGGACTCCTGCCCCAGCTGCT
GAAGAGACAATGACCACCAGCCCGGGGACTCCTGCCCCAGCTGCTGAAGAGACAATGACCACC
AGCCCGGGGACTCCTGCCTCTTCTCATTACCTCTCATGCACCATCGTAGGGATCATAGTTCTA
ATTGTGCTTCTGATTGTGTTTGTT**TGA**AAGACTTCACTGTGGAAGAAATTCCTTCCTTACCTG
AAAGGTTCAGGTAGGCGCTGGCTGAGGGCGGGGGGCGCTGGACACTCTCTGCCCTGCCTCCCT
CTGCTGTGTTCCCACAGACAGAAACGCCTGC

# FIGURE 300

MARIPKTLKFVVVIVAVLLPVLAYSATTARQEEVPQQTVAPQQQRHSFKGEECPAGSHRSEHT
GACNPCTEGVDYTNASNNEPSCFPCTVCKSDQKHKSSCTMTRDTVCQCKEGTFRNENSPEMCR
KCSRCPSGEVQVSNCTSWDDIQCVEEFGANATVETPAAEETMNTSPGTPAPAAEETMNTSPGT
PAPAAEETMTTSPGTPAPAAEETMTTSPGTPAPAAEETMTTSPGTPASSHYLSCTIVGIIVLI
VLLIVFV

**Important features:**

**Signal peptide:**

Amino acids 1-29

**Transmembrane domain:**

Amino acids 240-259

**N-glycosylation site:**

Amino acids 77-81;140-144;156-160

**cAMP- and cGMP-dependent protein kinase phosphorylation site:**

Amino acids 126-130

**N-myristoylation sites:**

Amino acids 56-62;72-78;114-120;154-160;233-239

# FIGURE 301

CACAAGCATCTTAATTTGAATCCACAAAGTTTCATGTAATGAAAAGAAATACATAATTTTAAT
TCAACCCGAGTGTTTTCCAAGAAGATTGTATTTGCTTAAATTGCTACAGTAATTCAAGAGACA
GCCCTGTCTGGACACAGAGTTACTGTGGATTTTTAAGAGACTCAGTTAAAGAATTTAGGAATT
TCTGATTCATTTAAAGGATTTACAAATTCATCAACCCCTGAAAACTAAAGCAAATTGAACAGG
AAAAAAAAAAAGAAG**ATG**GGTTTTTAAGTCCAATATATGTTATTTTCTTCTTTTTTGGAGTC
AAAGTACATTGCCAATATGAAACTTATCAGTGGGATGAAGACTATGACCAAGAGCCAGATGAT
GATTACCAAACAGGATTCCCATTTCGTCAAAATGTAGACTACGGAGTTCCTTTTCATCAGTAT
ACTTTAGGCTGTGTCAGTGAATGCTTCTGTCCAACTAACTTTCCATCATCAATGTACTGTGAT
AATCGCAAACTCAAGACTATCCCAAATATTCCGATGCACATTCAGCAACTCTACCTTCAGTTC
AATGAAATTGAGGCTGTGACTGCAAATTCATTCATCAATGCAACTCATCTTAAAGAAATTAAC
CTCAGCCACAACAAAATTAAATCTCAAAAGATTGATTATGGTGTGTTTGCTAAGCTTCCAAAT
CTACTACAACTTCATCTAGAGCATAATAATTTAGAAGAATTTCCATTTCCTCTTCCTAAATCT
CTGGAAAGACTCCTTCTTGGTTACAATGAAATCTCCAAACTGCAGACAAATGCTATGGATGGG
CTAGTAAACTTGACCATGCTTGATCTCTGTTATAATTATCTTCATGATTCTCTGCTAAAAGAC
AAAATCTTTGCCAAAATGGAAAAACTAATGCAGCTCAACCTCTGCAGTAACAGATTAGAATCA
ATGCCTCCTGGTTTGCCTTCTTCACTTATGTATCTGTCTTTAGAAAATAATTCAATTTCTTCT
ATACCCGAAAAATACTTCGACAAACTTCCAAAACTTCATACTCTAAGAATGTCACACAACAAA
CTACAAGACATCCCATATAATATTTTTAATCTTCCCAACATTGTAGAACTCAGTGTTGGACAC
AACAAATTGAAGCAAGCATTCTATATTCCAAGAAATTTGGAACACCTATACCTACAAAATAAT
GAAATAGAAAAGATGAATCTTACAGTGATGTGTCCTTCTATTGACCCACTACATTACCACCAT
TTAACATACATTCGTGTGGACCAAAATAAACTAAAAGAACCAATAAGCTCATACATCTTCTTC
TGCTTCCCTCATATACACACTATTTATTATGGTGAACAACGAAGCACTAATGGTCAAACAATA
CAACTAAAGACACAAGTTTTCAGGAGATTTCCAGATGATGATGATGAAAGTGAAGATCACGAT
GATCCTGACAATGCTCATGAGAGCCCAGAACAAGAAGGAGCAGAAGGGCACTTTGACCTTCAT
TATTATGAAAATCAAGAA**TAG**CAAGAAACTATATAGGTATACACTTACGACTTCACAAAACCTA
TACTTAATATAGTAAATCTAAGTAAACATGTATTACTCAAAGTAATATATTTAGAATTATGTA
TTAGTATAAGATCAGAATTGAATTTAAGTTGTTGGTGACATCTGCATCATTTCATAGGATTAG
AACTTACTCAAAATAATGTAAATCTTTAAAAATATAAATTAGAATGACAAGTGGGAATCATAA
ATTAAACGTTAATGGTTTCTTATGCTCTTTTTAAATATAGAAATATCATGTTAAAGAAAAAAA
AAAAAAA

# FIGURE 302

MGFLSPIYVIFFFFGVKVHCQYETYQWDEDYDQEPDDDYQTGFPFRQNVDYGVPFHQYTLGCV
SECFCPTNFPSSMYCDNRKLKTIPNIPMHIQQLYLQFNEIEAVTANSFINATHLKEINLSHNK
IKSQKIDYGVFAKLPNLLQLHLEHNNLEEFPFPLPKSLERLLLGYNEISKLQTNAMDGLVNLT
MLDLCYNYLHDSLLKDKIFAKMEKLMQLNLCSNRLESMPPGLPSSLMYLSLENNSISSIPEKY
FDKLPKLHTLRMSHNKLQDIPYNIFNLPNIVELSVGHNKLKQAFYIPRNLEHLYLQNNEIEKM
NLTVMCPSIDPLHYHHLTYIRVDQNKLKEPISSYIFFCFPHIHTIYYGEQRSTNGQTIQLKTQ
VFRRFPDDDDESEDHDDPDNAHESPEQEGAEGHFDLHYYENQE

**Important feastures:**

**N-glycosylation sites:**

Amino acids 113-117;121-125; 187-191;242-246;316-320

**Tyrosine kinase phosphorylation sites:**

Amino acids 268-275;300-307

**N-myristoylation site:**

Amino acids 230-236

**Leucine zipper patterns:**

Amino acids 146-168;217-239

# FIGURE 303

GCCCGGGACTGGCGCAAGGTGCCCAAGCAAGGAAAGAAATAATGAAGAGACACATGTGTTAGC
TGCAGCCTTTTGAAACACGCAAGAAGGAAATCAATAGTGTGGACAGGGCTGGAACCTTTACCA
CGCTTGTTGGAGTAGATGAGGAATGGGCTCGTGATTATGCTGACATTCCAGC**ATG**AATCTGGT
AGACCTGTGGTTAACCCGTTCCCTCTCCATGTGTCTCCTCCTACAAAGTTTTGTTCTTATGAT
ACTGTGCTTTCATTCTGCCAGTATGTGTCCCAAGGGCTGTCTTTGTTCTTCCTCTGGGGGTTT
AAATGTCACCTGTAGCAATGCAAATCTCAAGGAAATACCTAGAGATCTTCCTCCTGAAACAGT
CTTACTGTATCTGGACTCCAATCAGATCACATCTATTCCCAATGAAATTTTTAAGGACCTCCA
TCAACTGAGAGTTCTCAACCTGTCCAAAAATGGCATTGAGTTTATCGATGAGCATGCCTTCAA
AGGAGTAGCTGAAACCTTGCAGACTCTGGACTTGTCCGACAATCGGATTCAAAGTGTGCACAA
AAATGCCTTCAATAACCTGAAGGCCAGGGCCAGAATTGCCAACAACCCCTGGCACTGCGACTG
TACTCTACAGCAAGTTCTGAGGAGCATGGCGTCCAATCATGAGACAGCCCACAACGTGATCTG
TAAAACGTCCGTGTTGGATGAACATGCTGGCAGACCATTCCTCAATGCTGCCAACGACGCTGA
CCTTTGTAACCTCCCTAAAAAAACTACCGATTATGCCATGCTGGTCACCATGTTTGGCTGGTT
CACTATGGTGATCTCATATGTGGTATATTATGTGAGGCAAAATCAGGAGGATGCCCGGAGACA
CCTCGAATACTTGAAATCCCTGCCAAGCAGGCAGAAGAAAGCAGATGAACCTGATGATATTAG
CACTGTGGTA**TAG**TGTCCAAACTGACTGTCATTGAGAAAGAAAGAAAGTAGTTTGCGATTGCA
GTAGAAATAAGTGGTTTACTTCTCCCATCCATTGTAAACATTTGAAACTTTGTATTTCAGTTT
TTTTTGAATTATGCCACTGCTGAACTTTTAACAAACACTACAACATAAATAATTTGAGTTTAG
GTGATCCACCCCTTAATTGTACCCCCGATGGTATATTTCTGAGTAAGCTACTATCTGAACATT
AGTTAGATCCATCTCACTATTTAATAATGAAATTTATTTTTTAATTTAAAAGCAAATAAAAG
CTTAACTTTGAACCATGGGAAAAAAAAAAAAAAAAAAAAAAAAACA

# FIGURE 304

MNLVDLWLTRSLSMCLLLQSFVLMILCFHSASMCPKGCLCSSSGGLNVTCSNANLKEIPRDLP
PETVLLYLDSNQITSIPNEIFKDLHQLRVLNLSKNGIEFIDEHAFKGVAETLQTLDLSDNRIQ
SVHKNAFNNLKARARIANNPWHCDCTLQQVLRSMASNHETAHNVICKTSVLDEHAGRPFLNAA
NDADLCNLPKKTTDYAMLVTMFGWFTMVISYVVYYVRQNQEDARRHLEYLKSLPSRQKKADEP
DDISTVV

**Important features:**

**Signal sequence:**

Amino acids 1-33

**Transmembrane domain:**

Amino acids 204-219

**N-glycosylation sites:**

Amino acids 47-51;94-98

**cAMP- and cGMP-dependent protein kinase phosphorylation site:**

Amino acids 199-203

**Casein kinase II phosphorylation site.**

amino acids 162-166, 175-179

**N-myristoylation sites:**

Amino acids 37-43;45-51;110-116

# FIGURE 305

CGCCACCACTGCGGCCACCGCCA**ATG**AAACGCCTCCCGCTCCTAGTGGTTTTTTCCACTTTGTTGAATTGTTCCT
ATACTCAAAATTGCACCAAGACACCTTGTCTCCCAAATGCAAAATGTGAAATACGCAATGGAATTGAAGCCTGCT
ATTGCAACATGGGATTTTCAGGAAATGGTGTCACAATTTGTGAAGATGATAATGAATGTGGAAATTTAACTCAGT
CCTGTGGCGAAAATGCTAATTGCACTAACACAGAAGGAAGTTATTATTGTATGTGTGTACCTGGCTTCAGATCCA
GCAGTAACCAAGACAGGTTTATCACTAATGATGGAACCGTCTGTATAGAAAATGTGAATGCAAACTGCCATTTAG
ATAATGTCTGTATAGCTGCAAATATTAATAAAACTTTAACAAAAATCAGATCCATAAAAGAACCTGTGGCTTTGC
TACAAGAAGTCTATAGAAATTCTGTGACAGATCTTTCACCAACAGATATAATTACATATATATAGAAATATTAGCTG
AATCATCTTCATTACTAGGTTACAAGAACAACACTATCTCAGCCAAGGACACCCTTTCTAACTCAACTCTTACTG
AATTTGTAAAAACCGTGAATAATTTTGTTCAAAGGGATACATTTGTAGTTTGGGACAAGTTATCTGTGAATCATA
GGAGAACACATCTTACAAAACTCATGCACACTGTTGAACAAGCTACTTTAAGGATATCCCAGAGCTTCCAAAAGA
CCACAGAGTTTGATACAAATTCAACGGATATAGCTCTCAAAGTTTTCTTTTTTGATTCATATAACATGAAACATA
TTCATCCTCATATGAATATGGATGGAGACTACATAAATATATTTCCAAAGAGAAAAGCTGCATATGATTCAAATG
GCAATGTTGCAGTTGCATTTTTATATTATAAGAGTATTGGTCCTTTGCTTTCATCATCTGACAACTTCTTATTGA
AACCTCAAAATTATGATAATTCTGAAGAGGAGGAAAGAGTCATATCTTCAGTAATTTCAGTCTCAATGAGCTCAA
ACCCACCCACATTATATGAACTTGAAAAAATAACATTTACATTAAGTCATCGAAAGGTCACAGATAGGTATAGGA
GTCTATGTGCATTTTGGAATTACTCACCTGATACCATGAATGGCAGCTGGTCTTCAGAGGGCTGTGAGCTGACAT
ACTCAAATGAGACCCACACCTCATGCCGCTGTAATCACCTGACACATTTTGCAATTTTGATGTCCTCTGGTCCTT
CCATTGGTATTAAAGATTATAATATTCTTACAAGGATCACTCAACTAGGAATAATTATTTCACTGATTTGTCTTG
CCATATGCATTTTTACCTTCTGGTTCTTCAGTGAAATTCAAAGCACCAGGACAACAATTCACAAAAATCTTTGCT
GTAGCCTATTTCTTGCTGAACTTGTTTTTCTTGTTGGGATCAATACAAATACTAATAAGCTCTTCTGTTCAATCA
TTGCCGGACTGCTACACTACTTCTTTTTAGCTGCTTTTGCATGGATGTGCATTGAAGGCATACATCTCTATCTCA
TTGTTGTGGGTGTCATCTACAACAAGGGATTTTTGCACAAGAATTTTTATATCTTTGGCTATCTAAGCCCAGCCG
TGGTAGTTGGATTTTCGGCAGCACTAGGATACAGATATTATGGCACAACCAAAGTATGTTGGCTTAGCACCGAAA
ACAACTTTATTTGGAGTTTTATAGGACCAGCATGCCTAATCATTCTTGTTAATCTCTTGGCTTTTGGAGTCATCA
TATACAAAGTTTTTCGTCACACTGCAGGGTTGAAACCAGAAGTTAGTTGCTTTGAGAACATAAGGTCTTGTGCAA
GAGGAGCCCTCGCTCTTCTGTTCCTTCTCGGCACCACCTGGATCTTTGGGGTTCTCCATGTTGTGCACGCATCAG
TGGTTACAGCTTACCTCTTCACAGTCAGCAATGCTTTCCAGGGGATGTTCATTTTTTTATTCCTGTGTGTTTTAT
CTAGAAAGATTCAAGAAGAATATTACAGATTGTTCAAAAATGTCCCCTGTTGTTTTGGATGTTTAAGG**TAA**ACAT
AGAGAATGGTGGATAATTACAACTGCACAAAAATAAAAATTCCAAGCTGTGGATGACCAATGTATAAAAATGACT
CATCAAATTATCCAATTATTAACTACTAGACAAAAAGTATTTTAAATCAGTTTTTCTGTTTATGCTATAGGAACT
GTAGATAATAAGGTAAAATTATGTATCATATAGATATACTATGTTTTTCTATGTGAAATAGTTCTGTCAAAAATA
GTATTGCAGATATTTGGAAAGTAATTGGTTTCTCAGGAGTGATATCACTGCACCCAAGGAAAGATTTTCTTTCTA
ACACGAGAAGTATATGAATGTCCTGAAGGAAACCACTGGCTTGATATTTCTGTGACTCGTGTTGCCTTTGAAACT
AGTCCCCTACCACCTCGGTAATGAGCTCCATTACAGAAAGTGGAACATAAGAGAATGAAGGGGCAGAATATCAAA
CAGTGAAAAGGGAATGATAAGATGTATTTTGAATGAACTGTTTTTTCTGTAGACTAGCTGAGAAATTGTTGACAT
AAAATAAAGAATTGAAGAAACACATTTACCATTTTGTGAATTGTTCTGAACTTAAATGTCCACTAAAACAACTT
AGACTTCTGTTTGCTAAATCTGTTTCTTTTTCTAATATTCTAAAAAAAAAAAAAAAGGTTTACCTCCACAAATTGA
AAAAAAAAAAAAAAAAAAAAAAAAAAAAAAAAAAAAAAAAAAAAAAAAAA

# FIGURE 306

MKRLPLLVVFSTLLNCSYTQNCTKTPCLPNAKCEIRNGIEACYCNMGFSGNGVTICEDDNECGNLTQSCGENANC
TNTEGSYYCMCVPGFRSSSNQDRFITNDGTVCIENVNANCHLDNVCIAANINKTLTKIRSIKEPVALLQEVYRNS
VTDLSPTDIITYIEILAESSSLLGYKNNTISAKDTLSNSTLTEFVKTVNNFVQRDTFVVWDKLSVNHRRTHLTKL
MHTVEQATLRISQSFQKTTEFDTNSTDIALKVFFFDSYNMKHIHPHMNMDGDYINIFPKRKAAYDSNGNVAVAFL
YYKSIGPLLSSSDNFLLKPQNYDNSEEEERVISSVISVSMSSNPPTLYELEKITFTLSHRKVTDRYRSLCAFWNY
SPDTMNGSWSSEGCELTYSNETHTSCRCNHLTHFAILMSSGPSIGIKDYNILTRITQLGIIISLICLAICIFTFW
FFSEIQSTRTTIHKNLCCSLFLAELVFLVGINTNTNKLFCSIIAGLLHYFFLAAFAWMCIEGIHLYLIVVGVIYN
KGFLHKNFYIFGYLSPAVVVGFSAALGYRYYGTTKVCWLSTENNFIWSFIGPACLIILVNLLAFGVIIYKVFRHT
AGLKPEVSCFENIRSCARGALALLFLLGTTWIFGVLHVVHASVVTAYLFTVSNAFQGMFIFLFLCVLSRKIQEEY
YRLFKNVPCCFGCLR

**Important features:**

**Signal peptide:**

Amino acids 1-19

**Transmembrane domain:**

Amino acids 431-450;494-515;573-594;619-636;646-664

**N-glycosylation sites:**

Amino acids 15-19;21-25;64-68;74-78;127-131;177-181;
188-192;249-253;381-385;395-399

**Glycosaminoglycan attachment site:**

Amino acids 49-53

**cAMP- and cGMP-dependent protein kinase phosphorylation site:**

Amino acids 360-364

**Tyrosine kinase phosphorylation sites:**

Amino acids 36-44;670-677

**N-myristoylation sites:**

Amino acids 38-44;50-56;52-58;80-86;382-388;388-394;
434-440;480-486;521-527

**Aspartic acid and asparagine hydroxylation site:**

Amino acids 75-87

# FIGURE 307

CCAGGCCGGGAGGCGACGCGCCCAGCCGTCTAAACGGAACAGCCCTGGCTGAGGGAGCTGCAGCGCAGCAGAGT
ATCTGACGGCGCCAGGTTGCGTAGGTGCGGCACGAGGAGTTTTCCCGGCAGCGAGGAGGTCCTGAGCAGC**ATG**GC
CCGGAGGAGCGCCTTCCCTGCCGCCGCGCTCTGGCTCTGGAGCATCCTCCTGTGCCTGCTGGCACTGCGGGCGGA
GGCCGGGCCGCCGCAGGAGGAGAGCCTGTACCTATGGATCGATGCTCACCAGGCAAGAGTACTCATAGGATTTGA
AGAAGATATCCTGATTGTTTCAGAGGGGAAAATGGCACCTTTTACACATGATTTCAGAAAAGCGCAACAGAGAAT
GCCAGCTATTCCTGTCAATATCCATTCCATGAATTTTACCTGGCAAGCTGCAGGGCAGGCAGAATACTTCTATGA
ATTCCTGTCCTTGCGCTCCCTGGATAAAGGCATCATGGCAGATCCAACCGTCAATGTCCCTCTGCTGGGAACAGT
GCCTCACAAGGCATCAGTTGTTCAAGTTGGTTTCCCATGTCTTGGAAAACAGGATGGGGTGGCAGCATTTGAAGT
GGATGTGATTGTTATGAATTCTGAAGGCAACACCATTCTCCAAACACCTCAAAATGCTATCTTCTTTAAAACATG
TCAACAAGCTGAGTGCCCAGGCGGGTGCCGAAATGGAGGCTTTTGTAATGAAAGACGCATCTGCGAGTGTCCTGA
TGGGTTCCACGGACCTCACTGTGAGAAAGCCCTTTGTACCCCACGATGTATGAATGGTGGACTTTGTGTGACTCC
TGGTTTCTGCATCTGCCCACCTGGATTCTATGGAGTGAACTGTGACAAAGCAAACTGCTCAACCACCTGCTTTAA
TGGAGGGACCTGTTTCTACCCTGGAAAATGTATTTGCCCTCCAGGACTAGAGGGAGAGCAGTGTGAAATCAGCAA
ATGCCCACAACCCTGTCGAAATGGAGGTAAATGCATTGGTAAAAGCAAATGTAAGTGTTCCAAAGGTTACCAGGG
AGACCTCTGTTCAAAGCCTGTCTGCGAGCCTGGCTGTGGTGCACATGGAACCTGCCATGAACCCAACAAATGCCA
ATGTCAAGAAGGTTGGCATGGAAGACACTGCAATAAAAGGTACGAAGCCAGCCTCATACATGCCCTGAGGCCAGC
AGGCGCCCAGCTCAGGCAGCACACGCCTTCACTTAAAAAGGCCGAGGAGCGGCGGGATCCACCTGAATCCAATTA
CATCTGG**TGA**ACTCCGACATCTGAAACGTTTTAAGTTACACCAAGTTCATAGCCTTTGTTAACCTTTCATGTGTT
GAATGTTCAAATAATGTTCATTACACTTAAGAATACTGGCCTGAATTTTATTAGCTTCATTATAAATCACTGAGC
TGATATTTACTCTTCCTTTTAAGTTTTCTAAGTACGTCTGTAGCATGATGGTATAGATTTTCTTGTTTCAGTGCT
TTGGGACAGATTTTATATTATGTCAATTGATCAGGTTAAAATTTTCAGTGTGTAGTTGGCAGATATTTTCAAAAT
TACAATGCATTTATGGTGTCTGGGGGCAGGGGAACATCAGAAAGGTTAAATTGGGCAAAAATGCGTAAGTCACAA
GAATTTGGATGGTGCAGTTAATGTTGAAGTTACAGCATTTCAGATTTTATTGTCAGATATTTAGATGTTTGTTAC
ATTTTTAAAAATTGCTCTTAATTTTTAAACTCTCAATACAATATATTTTGACCTTACCATTATTCCAGAGATTCA
GTATTAAAAAAAAAAAATTACACTGTGGTAGTGGCATTTAAACAATATAATATATTCTAAACACAATGAAATAG
GGAATATAATGTATGAACTTTTTGCATTGGCTTGAAGCAATATAATATATTGTAAACAAAACACAGCTCTTACCT
AATAAACATTTTATACTGTTTGTATGTATAAAATAAAGGTGCTGCTTTAGTTTTTGGAAAAAAAAAAAAAAAAAA
AAAAAAAA

# FIGURE 308

MARRSAFPAAALWLWSILLCLLALRAEAGPPQEESLYLWIDAHQARVLIGFEEDILIVSEGKM
APFTHDFRKAQQRMPAIPVNIHSMNFTWQAAGQAEYFYEFLSLRSLDKGIMADPTVNVPLLGT
VPHKASVVQVGFPCLGKQDGVAAFEVDVIVMNSEGNTILQTPQNAIFFKTCQQAECPGGCRNG
GFCNERRICECPDGFHGPHCEKALCTPRCMNGGLCVTPGFCICPPGFYGVNCDKANCSTTCFN
GGTCFYPGKCICPPGLEGEQCEISKCPQPCRNGGKCIGKSKCKCSKGYQGDLCSKPVCEPGCG
AHGTCHEPNKCQCQEGWHGRHCNKRYEASLIHALRPAGAQLRQHTPSLKKAEERRDPPESNYIW

**Important features:**

**Signal sequence:**

Amino acids 1-28

**N-glycosylation sites:**

Amino acids 88-92;245-249

**Tyrosine kinase phosphorylation site:**

Amino acids 370-378

**N-myristoylation sites:**

Amino acids 184-190;185-191;189-195;315-321

**ATP/GTP-binding site motif A (P-loop):**

Amino acids 285-293

**EGF-like domain cysteine pattern signatures:**

Amino acids 198-210;230-242;262-274;294-306;326-338

# FIGURE 309

CCCACGCGTCCGGTCTCGCTCGCTCGCGCAGCGGCGGCAGCAGAGGTCGCGCACAGATGCGGG
TTAGACTGGCGGGGGGAGGAGGCGGAGGAGGGAAGGAAGCTGCATGCATGAGACCCACAGACT
CTTGCAAGCTGGATGCCCTCTGTGGATGAAAG**ATG**TATCATGGAATGAACCCGAGCAATGGAG
ATGGATTTCTAGAGCAGCAGCAGCAGCAGCAGCAACCTCAGTCCCCCCAGAGACTCTTGGCCG
TGATCCTGTGGTTTCAGCTGGCGCTGTGCTTCGGCCCTGCACAGCTCACGGGCGGGTTCGATG
ACCTTCAAGTGTGTGCTGACCCCGGCATTCCCGAGAATGGCTTCAGGACCCCCAGCGGAGGGG
TTTTCTTTGAAGGCTCTGTAGCCCGATTTCACTGCCAAGACGGATTCAAGCTGAAGGGCGCTA
CAAAGAGACTGTGTTTGAAGCATTTTAATGGAACCCTAGGCTGGATCCCAAGTGATAATTCCA
TCTGTGTGCAAGAAGATTGCCGTATCCCTCAAATCGAAGATGCTGAGATTCATAACAAGACAT
ATAGACATGGAGAGAAGCTAATCATCACTTGTCATGAAGGATTCAAGATCCGGTACCCCGACC
TACACAATATGGTTTCATTATGTCGCGATGATGGAACGTGGAATAATCTGCCCATCTGTCAAG
GCTGCCTGAGACCTCTAGCCTCTTCTAATGGCTATGTAAACATCTCTGAGCTCCAGACCTCCT
TCCCGGTGGGGACTGTGATCTCCTATCGCTGCTTTCCCGGATTTAAACTTGATGGGTCTGCGT
ATCTTGAGTGCTTACAAAACCTTATCTGGTCGTCCAGCCCACCCCGGTGCCTTGCTCTGGAAG
CCCAAGTCTGTCCACTACCTCCAATGGTGAGTCACGGAGATTTCGTCTGCCACCCGCGGCCTT
GTGAGCGCTACAACCACGGAACTGTGGTGGAGTTTTACTGCGATCCTGGCTACAGCCTCACCA
GCGACTACAAGTACATCACCTGCCAGTATGGAGAGTGGTTTCCTTCTTATCAAGTCTACTGCA
TCAAATCAGAGCAAACGTGGCCCAGCACCCATGAGACCCTCCTGACCACGTGGAAGATTGTGG
CGTTCACGGCAACCAGTGTGCTGCTGGTGCTGCTGCTCGTCATCCTGGCCAGGATGTTCCAGA
CCAAGTTCAAGGCCCACTTTCCCCCCAGGGGGCCTCCCCGGAGTTCCAGCAGTGACCCTGACT
TTGTGGTGGTAGACGGCGTGCCCGTCATGCTCCCGTCCTATGACGAAGCTGTGAGTGGCGGCT
TGAGTGCCTTAGGCCCCGGGTACATGGCCTCTGTGGGCCAGGGCTGCCCCTTACCCGTGGACG
ACCAGAGCCCCCCAGCATACCCCGGCTCAGGGGACACGGACACAGGCCCAGGGGAGTCAGAAA
CCTGTGACAGCGTCTCAGGCTCTTCTGAGCTGCTCCAAAGTCTGTATTCACCTCCCAGGTGCC
AAGAGAGCACCCACCCTGCTTCGGACAACCCTGACATAATTGCCAGCACGGCAGAGGAGGTGG
CATCCACCAGCCCAGGCATCCATCATGCCCACTGGGTGTTGTTCCTAAGAAAC**TGA**TTGATTA
AAAAATTTCCCAAAGTGTCCTGAAGTGTCTCTTCAAATACATGTTGATCTGTGGAGTTGATTC
CTTTCCTTCTCTTGGTTTTAGACAAATGTAAACAAAGCTCTGATCCTTAAAATTGCTATGCTG
ATAGAGTGGTGAGGGCTGGAAGCTTGATCAAGTCCTGTTTCTTCTTGACACAGACTGATTAAA
AATTAAAAGNAAAAAA

# FIGURE 310

MYHGMNPSNGDGFLEQQQQQQQPQSPQRLLAVILWFQLALCFGPAQLTGGFDDLQVCADPGIP
ENGFRTPSGGVFFEGSVARFHCQDGFKLKGATKRLCLKHFNGTLGWIPSDNSICVQEDCRIPQ
IEDAEIHNKTYRHGEKLIITCHEGFKIRYPDLHNMVSLCRDDGTWNNLPICQGCLRPLASSNG
YVNISELQTSFPVGTVISYRCFPGFKLDGSAYLECLQNLIWSSSPPRCLALEAQVCPLPPMVS
HGDFVCHPRPCERYNHGTVVEFYCDPGYSLTSDYKYITCQYGEWFPSYQVYCIKSEQTWPSTH
ETLLTTWKIVAFTATSVLLVLLLVILARMFQTKFKAHFPPRGPPRSSSSDPDFVVVDGVPVML
PSYDEAVSGGLSALGPGYMASVGQGCPLPVDDQSPPAYPGSGDTDTGPGESETCDSVSGSSEL
LQSLYSPPRCQESTHPASDNPDIIASTAEEVASTSPGIHHAHWVLFLRN

**Important features:**

**Signal sequence:**

amino acids 1-41

**Transmembrane domain:**

amino acids 325-344

**N-glycosylation site.**

amino acids 104-108, 134-138, 192-196

**Casein kinase II phosphorylation site.**

amino acids 8-12, 146-150, 252-256, 270-274, 313-317, 362-366, 364-368, 380-384, 467-471, 468-472

**N-myristoylation site.**

amino acids 4-10, 61-67, 169-175, 203-209, 387-393, 418-424, 478-484

**Prokaryotic membrane lipoprotein lipid attachment site.**

amino acids 394-405

# FIGURE 311

CAGCGCGTGGCCGGCGCCGCTGTGGGGACAGC**ATG**AGCGGCGGTTGGATGGCGCAGGTTGGAG
CGTGGCGAACAGGGGCTCTGGGCCTGGCGCTGCTGCTGCTCGGCCTCGGACTAGGCCTGG
AGGCCGCCGCGAGCCCGCTTTCCACCCCGACCTCTGCCCAGGCCGCAGGCCCCAGCTCAGGCT
CGTGCCCACCCACCAAGTTCCAGTGCCGCACCAGTGGCTTATGCGTGCCCCTCACCTGGCGCT
GCGACAGGGACTTGGACTGCAGCGATGGCAGCGATGAGGAGGAGTGCAGGATTGAGCCATGTA
CCCAGAAAGGGCAATGCCCACCGCCCCCTGGCCTCCCCTGCCCCTGCACCGGCGTCAGTGACT
GCTCTGGGGGAACTGACAAGAAACTGCGCAACTGCAGCCGCCTGGCCTGCCTAGCAGGCGAGC
TCCGTTGCACGCTGAGCGATGACTGCATTCCACTCACGTGGCGCTGCGACGGCCACCCAGACT
GTCCCGACTCCAGCGACGAGCTCGGCTGTGGAACCAATGAGATCCTCCCGGAAGGGGATGCCA
CAACCATGGGGCCCCCTGTGACCCTGGAGAGTGTCACCTCTCTCAGGAATGCCACAACCATGG
GGCCCCCTGTGACCCTGGAGAGTGTCCCCTCTGTCGGGAATGCCACATCCTCCTCTGCCGGAG
ACCAGTCTGGAAGCCCAACTGCCTATGGGGTTATTGCAGCTGCTGCGGTGCTCAGTGCAAGCC
TGGTCACCGCCACCCTCCTCCTTTTGTCCTGGCTCCGAGCCCAGGAGCGCCTCCGCCCACTGG
GGTTACTGGTGGCCATGAAGGAGTCCCTGCTGCTGTCAGAACAGAAGACCTCGCTGCCC**TGA**G
GACAAGCACTTGCCACCACCGTCACTCAGCCCTGGGCGTAGCCGGACAGGAGGAGAGCAGTGA
TGCGGATGGGTACCCGGGCACACCAGCCCTCAGAGACCTGAGTTCTTCTGGCCACGTGGAACC
TCGAACCCGAGCTCCTGCAGAAGTGGCCCTGGAGATTGAGGGTCCCTGGACACTCCCTATGGA
GATCCGGGGAGCTAGGATGGGGAACCTGCCACAGCCAGAACTGAGGGGCTGGCCCCAGGCAGC
TCCCAGGGGGTAGAACGGCCCTGTGCTTAAGACACTCCCTGCTGCCCCGTCTGAGGGTGGCGA
TTAAAGTTGCTTC

# FIGURE 312

MSGGWMAQVGAWRTGALGLALLLLLGLGLGLEAAASPLSTPTSAQAAGPSSGSCPPTKFQCRT
SGLCVPLTWRCDRDLDCSDGSDEEECRIEPCTQKGQCPPPPGLPCPCTGVSDCSGGTDKKLRN
CSRLACLAGELRCTLSDDCIPLTWRCDGHPDCPDSSDELGCGTNEILPEGDATTMGPPVTLES
VTSLRNATTMGPPVTLESVPSVGNATSSSAGDQSGSPTAYGVIAAAAVLSASLVTATLLLLSW
LRAQERLRPLGLLVAMKESLLLSEQKTSLP

**Important features:**

**Signal sequence:**

Amino acids 1-30

**Transmembrane domain:**

Amino acids 231-248

**N-glycosylation sites:**

Amino acids 126-130;195-199;213-217

**Casein kinase II phosphorylation site.**

amino acids 84-88, 140-144, 161-165, 218-222

**N-myristoylation sites:**

Amino acids 3-9;10-16;26-32;30-36;112-118;166-172;212-218;
224-230;230-236;263-269

**Prokaryotic membrane lipoprotein lipid attachment site:**

Amino acids 44-55

**Leucine zipper pattern:**

Amino acids 17-39

# FIGURE 313

CGGACGCGTGGGCGTCCGGCGGTCGCAGAGCCAGGAGGCGGAGGCGCGCGGGCCAGCCTGGGCCCCAGCCCACAC
CTTCACCAGGGCCCAGGAGCCACC**ATG**TGGCGATGTCCACTGGGGCTACTGCTGTTGCTGCCGCTGGCTGGCCAC
TTGGCTCTGGGTGCCCAGCAGGGTCGTGGGCGCCGGGAGCTAGCACCGGGTCTGCACCTGCGGGGCATCCGGGAC
GCGGGAGGCCGGTACTGCCAGGAGCAGGACCTGTGCTGCCGCGGCCGTGCCGACGACTGTGCCCTGCCCTACCTG
GGCGCCATCTGTTACTGTGACCTCTTCTGCAACCGCACGGTCTCCGACTGCTGCCCTGACTTCTGGGACTTCTGC
CTCGGCGTGCCACCCCCTTTTCCCCCGATCCAAGGATGTATGCATGGAGGTCGTATCTATCCAGTCTTGGGAACG
TACTGGGACAACTGTAACCGTTGCACCTGCCAGGAGAACAGGCAGTGGCATGGTGGATCCAGACATGATCAAAGC
CATCAACCAGGGCAACTATGGCTGGCAGGCTGGGAACCACAGCGCCTTCTGGGGCATGACCCTGGA**TGA**GGGCAT
TCGCTACCGCCTGGGCACCATCCGCCCATCTTCCTCGGTCATGAACATGCATGAAATTTATACAGTGCTGAACCC
AGGGGAGGTGCTTCCCACAGCCTTCGAGGCCTCTGAGAAGTGGCCCAACCTGATTCATGAGCCTCTTGACCAAGG
CAACTGTGCAGGCTCCTGGGCCTTCTCCACAGCAGCTGTGGCATCCGATCGTGTCTCAATCCATTCTCTGGGACA
CATGACGCCTGTCCTGTCGCCCCAGAACCTGCTGTCTTGTGACACCCACCAGCAGCAGGGCTGCCGCGGTGGGCG
TCTCGATGGTGCCTGGTGGTTCCTGCGTCGCCGAGGGGTGGTGTCTGACCACTGCTACCCCTTCTCGGGCCGTGA
ACGAGACGAGGCTGGCCCTGCGCCCCCCTGTATGATGCACAGCCGAGCCATGGGTCGGGGCAAGCGCCAGGCCAC
TGCCCACTGCCCCAACAGCTATGTTAATAACAATGACATCTACCAGGTCACTCCTGTCTACCGCCTCGGCTCCAA
CGACAAGGAGATCATGAAGGAGCTGATGGAGAATGGCCCTGTCCAAGCCCTCATGGAGGTGCATGAGGACTTCTT
CCTATACAAGGGAGGCATCTACAGCCACACGCCAGTGAGCCTTGGGAGGCCAGAGAGATACCGCCGGCATGGGAC
CCACTCAGTCAAGATCACAGGATGGGGAGAGGAGACGCTGCCAGATGGAAGGACGCTCAAATACTGGACTGCGGC
CAACTCCTGGGGCCCAGCCTGGGGCGAGAGGGGCCACTTCCGCATCGTGCGCGGCGTCAATGAGTGCGACATCGA
GAGCTTCGTGCTGGGCGTCTGGGGCCGCGTGGGCATGGAGGACATGGGTCATCACTGAGGCTGCGGGCACCACGC
GGGGTCCGGCCTGGGATCCAGGCTAAGGGCCGGCGGAAGAGGCCCCAATGGGGCGGTGACCCCAGCCTCGCCCGA
CAGAGCCCGGGGCGCAGGCGGGCGCCAGGGCGCTAATCCCGGCGCGGGTTCCGCTGACGCAGCGCCCCGCCTGGG
AGCCGCGGGCAGGCGAGACTGGCGGAGCCCCCAGACCTCCCAGTGGGGACGGGGCAGGGCCTGGCCTGGGAAGAG
CACAGCTGCAGATCCCAGGCCTCTGGCGCCCCCACTCAAGACTACCAAAGCCAGGACACCTCAAGTCTCCAGCCC
CAATACCCCACCCCAATCCCGTATTCTTTTTTTTTTTTTTTAGACAGGGTCTTGCTCCGTTGCCCAGGTTGGAG
TGCAGTGGCCCATCAGGGCTCACTGTAACCTCCGACTCCTGGGTTCAAGTGACCCTCCCACCTCAGCCTCTCAAG
TAGCTGGGACTACAGGTGCACCACCACACCTGGCTAATTTTTGTATTTTTTGTAAAGAGGGGGGTCTCACTGTGT
TGCCCAGGCTGGTTTCGAACTCCTGGGCTCAAGCGGTCCACCTGCCTCCGCCTCCCAAAGTGCTGGGATTGCAGG
CATGAGCCACTGCACCCAGCCCTGTATTCTTATTCTTCAGATATTTATTTTTCTTTTCACTGTTTTAAAATAAAA
CCAAAGTATTGATAAAAAAAAA

# FIGURE 314

MWRCPLGLLLLLPLAGHLALGAQQGRGRRELAPGLHLRGIRDAGGRYCQEQDLCCRGRADDCA
LPYLGAICYCDLFCNRTVSDCCPDFWDFCLGVPPPFPPIQGCMHGGRIYPVLGTYWDNCNRCT
CQENRQWHGGSRHDQSHQPGQLWLAGWEPQRLLGHDPG

**Important features:**

**N-glycosylation site.**

amino acids 78-82, 161-165

**Casein kinase II phosphorylation site.**

amino acids 80-84, 117-121, 126-130, 169-173, 205-209, 296-300, 411-415

**N-myristoylation site.**

amino acids 21-27, 39-45, 44-50, 104-110, 160-164, 224-230, 269-275, 378-384, 442-448

**Amidation site.**

amino acids 26-30, 318-322

**Eukaryotic thiol (cysteine) proteases histidine active site.**

amino acids 398-409

# FIGURE 315

CGGACGCGTGGGCCCCTGGTGGGCCCAGCAAG**ATG**GATCTACTGTGGATCCTGCCCTCCCTGT
GGCTTCTCCTGCTTGGGGGGCCTGCCTGCCTGAAGACCCAGGAACACCCCAGCTGCCCAGGAC
CCAGGGAACTGGAAGCCAGCAAAGTTGTCCTCCTGCCCAGTTGTCCCGGAGCTCCAGGAAGTC
CTGGGGAGAAGGGAGCCCCAGGTCCTCAAGGGCCACCTGGACCACCAGGCAAGATGGGCCCCA
AGGGTGAGCCAGGCCCCAGAAACTGCCGGGAGCTGTTGAGCCAGGGCGCCACCTTGAGCGGCT
GGTACCATCTGTGCCTACCTGAGGGCAGGGCCCTCCCAGTCTTTTGTGACATGGACACCGAGG
GGGGCGGCTGGCTGGTGTTTCAGAGGCGCCAGGATGGTTCTGTGGATTTCTTCCGCTCTTGGT
CCTCCTACAGAGCAGGTTTTGGGAACCAAGAGTCTGAATTCTGGCTGGGAAATGAGAATTTGC
ACCAGCTTACTCTCCAGGGTAACTGGGAGCTGCGGGTAGAGCTGGAAGACTTTAATGGTAACC
GTACTTTCGCCCACTATGCGACCTTCCGCCTCCTCGGTGAGGTAGACCACTACCAGCTGGCAC
TGGGCAAGTTCTCAGAGGGCACTGCAGGGGATTCCCTGAGCCTCCACAGTGGGAGGCCCTTTA
CCACCTATGACGCTGACCACGATTCAAGCAACAGCAACTGTGCAGTGATTGTCCACGGTGCCT
GGTGGTATGCATCCTGTTACCGATCAAATCTCAATGGTCGCTATGCAGTGTCTGAGGCTGCCG
CCCACAAATATGGCATTGACTGGGCCTCAGGCCGTGGTGTGGGCCACCCCTACCGCAGGGTTC
GGATGATGCTTCGA**TAG**GGCACTCTGGCAGCCAGTGCCCTTATCTCTCCTGTACAGCTTCCGG
ATCGTCAGCCACCTTGCCTTTGCCAACCACCTCTGCTTGCCTGTCCACATTTAAAAATAAAAT
CATTTTAGCCCTTTCA

# FIGURE 316

MDLLWILPSLWLLLLGGPACLKTQEHPSCPGPRELEASKVVLLPSCPGAPGSPGEKGAPGPQG
PPGPPGKMGPKGEPGPRNCRELLSQGATLSGWYHLCLPEGRALPVFCDMDTEGGGWLVFQRRQ
DGSVDFFRSWSSYRAGFGNQESEFWLGNENLHQLTLQGNWELRVELEDFNGNRTFAHYATFRL
LGEVDHYQLALGKFSEGTAGDSLSLHSGRPFTTYDADHDSSNSNCAVIVHGAWWYASCYRSNL
NGRYAVSEAAAHKYGIDWASGRGVGHPYRRVRMMLR

**Important features:**

**Signal peptide:**

Amino acids 1-16

**N-glycosylation site:**

Amino acids 178-182

**Glycosaminoglycan attachment site:**

Amino acids 272-276

**Tyrosine kinase phosphorylation site:**

Amino acids 188-197

**N-myristoylation sites:**

Amino acids 16-22;89-95;144-150;267-273

**Fibrinogen beta and gamma chains C-terminal domain signature:**

Amino acids.242-255

# FIGURE 317

CCCAAGCCAGCCGAGCCGCCAGAGCCGCGGGCCGCGGGGGTGTCGCGGGCCCAACCCCAGG**AT**
**G**CTCCCCTGCGCCTCCTGCCTACCCGGGTCTCTACTGCTCTGGGCGCTGCTACTGTTGCTCTT
GGGATCAGCTTCTCCTCAGGATTCTGAAGAGCCCGACAGCTACACGGAATGCACAGATGGCTA
TGAGTGGGACCCAGACAGCCAGCACTGCCGGGATGTCAACGAGTGTCTGACCATCCCTGAGGC
CTGCAAGGGGGAAATGAAGTGCATCAACCACTACGGGGGCTACTTGTGCCTGCCCCGCTCCGC
TGCCGTCATCAACGACCTACATGGCGAGGGACCCCCGCCACCAGTGCCTCCCGCTCAACACCC
CAACCCCTGCCCACCAGGCTATGAGCCCGACGATCAGGACAGCTGTGTGGATGTGGACGAGTG
TGCCCAGGCCCTGCACGACTGTCGCCCCAGCCAGGACTGCCATAACTTGCCTGGCTCCTATCA
GTGCACCTGCCCTGATGGTTACCGCAAGATCGGGCCCGAGTGTGTGGACATAGACGAGTGCCG
CTACCGCTACTGCCAGCACCGCTGCGTGAACCTGCCTGGCTCCTTCCGCTGCCAGTGCGAGCC
GGGCTTCCAGCTGGGGCCTAACAACCGCTCCTGTGTTGATGTGAACGAGTGTGACATGGGGGC
CCCATGCGAGCAGCGCTGCTTCAACTCCTATGGGACCTTCCTGTGTCGCTGCCACCAGGGCTA
TGAGCTGCATCGGGATGGCTTCTCCTGCAGTGATATTGATGAGTGTAGCTACTCCAGCTACCT
CTGTCAGTACCGCTGCGTCAACGAGCCAGGCCGTTTCTCCTGCCACTGCCCACAGGGTTACCA
GCTGCTGGCCACACGCCTCTGCCAAGACATTGATGAGTGTGAGTCTGGTGCGCACCAGTGCTC
CGAGGCCCAAACCTGTGTCAACTTCCATGGGGGCTACCGCTGCGTGGACACCAACCGCTGCGT
GGAGCCCTACATCCAGGTCTCTGAGAACCGCTGTCTCTGCCCGGCCTCCAACCCTCTATGTCG
AGAGCAGCCTTCATCCATTGTGCACCGCTACATGACCATCACCTCGGAGCGGAGCGTGCCCGC
TGACGTGTTCCAGATCCAGGCGACCTCCGTCTACCCCGGTGCCTACAATGCCTTTCAGATCCG
TGCTGGAAACTCGCAGGGGGACTTTTACATTAGGCAAATCAACAACGTCAGCGCCATGCTGGT
CCTCGCCCGGCCGGTGACGGGCCCCCGGGAGTACGTGCTGGACCTGGAGATGGTCACCATGAA
TTCCCTCATGAGCTACCGGGCCAGCTCTGTACTGAGGCTCACCGTCTTTGTAGGGGCCTACAC
CTTC**TGA**GGAGCAGGAGGGAGCCACCCTCCCTGCAGCTACCCTAGCTGAGGAGCCTGTTGTGA
GGGGCAGAATGAGAAAGGCAATAAAGGGAGAAAGAAAGTCCTGGTGGCTGAGGTGGGCGGGTC
ACACTGCAGGAAGCCTCAGGCTGGGGCAGGGTGGCACTTGGGGGGGCAGGCCAAGTTCACCTA
AATGGGGGTCTCTATATGTTCAGGCCCAGGGGCCCCCATTGACAGGAGCTGGGAGCTCTGCAC
CACGAGCTTCAGTCACCCCGAGAGGAGAGGAGGTAACGAGGAGGGCGGACTCCAGGCCCCGGC
CCAGAGATTTGGACTTGGCTGGCTTGCAGGGGTCCTAAGAAACTCCACTCTGGACAGCGCCAG
GAGGCCCTGGGTTCCATTCCTAACTCTGCCTCAAACTGTACATTTGGATAAGCCCTAGTAGTT
CCCTGGGCCTGTTTTTCTATAAAACGAGGCAACTGGAAAAAAAAAAAA

# FIGURE 318

MLPCASCLPGSLLLWALLLLLLLGSASPQDSEEPDSYTECTDGYEWDPDSQHCRDVNECLTIPE
ACKGEMKCINHYGGYLCLPRSAAVINDLHGEGPPPPVPPAQHPNPCPPGYEPDDQDSCVDVDE
CAQALHDCRPSQDCHNLPGSYQCTCPDGYRKIGPECVDIDECRYRYCQHRCVNLPGSFRCQCE
PGFQLGPNNRSCVDVNECDMGAPCEQRCFNSYGTFLCRCHQGYELHRDGFSCSDIDECSYSSY
LCQYRCVNEPGRFSCHCPQGYQLLATRLCQDIDECESGAHQCSEAQTCVNFHGGYRCVDTNRC
VEPYIQVSENRCLCPASNPLCREQPSSIVHRYMTITSERSVPADVFQIQATSVYPGAYNAFQI
RAGNSQGDFYIRQINNVSAMLVLARPVTGPREYVLDLEMVTMNSLMSYRASSVLRLTVFVGAYTF

**Important features:**

**Signal sequence:**

Amino acids 1-25

**N-glycosylation sites:**

Amino acids 198-202;394-398

**N-myristoylation sites:**

Amino acids 76-82;145-151;182-188;222-228;290-296;305-311;
371-377;381-387

**Aspartic acid and asparagine hydroxylation sites:**

amino acids 140-152;177-189;217-229;258-270

# FIGURE 319

GCTGGGGAC**ATG**AGAGGCACACCGAAGACCCACCTCCTGGCCTTCTCCCTCCTCTGCCTCCTC
TCAAAGGTGCGTACCCAGCTGTGCCCGACACCATGTACCTGCCCCTGGCCACCTCCCCGATGC
CCGCTGGGAGTACCCCTGGTGCTGGATGGCTGTGGCTGCTGCCGGGTATGTGCACGGCGGCTG
GGGGAGCCCTGCGACCAACTCCACGTCTGCGACGCCAGCCAGGGCCTGGTCTGCCAGCCCGGG
GCAGGACCCGGTGGCCGGGGGGCCCTGTGCCTCTTGGCAGAGGACGACAGCAGCTGTGAGGTG
AACGGCCGCCTGTATCGGGAAGGGGAGACCTTCCAGCCCCACTGCAGCATCCGCTGCCGCTGC
GAGGACGGCGGCTTCACCTGCGTGCCGCTGTGCAGCGAGGATGTGCGGCTGCCCAGCTGGGAC
TGCCCCCACCCCAGGAGGGTCGAGGTCCTGGGCAAGTGCTGCCCTGAGTGGGTGTGCGGCCAA
GGAGGGGGACTGGGGACCCAGCCCCTTCCAGCCCAAGGACCCCAGTTTTCTGGCCTTGTCTCT
TCCCTGCCCCCTGGTGTCCCCTGCCCAGAATGGAGCACGGCCTGGGGACCCTGCTCGACCACC
TGTGGGCTGGGCATGGCCACCCGGGTGTCCAACCAGAACCGCTTCTGCCGACTGGAGACCCAG
CGCCGCCTGTGCCTGTCCAGGCCCTGCCCACCCTCCAGGGGTCGCAGTCCACAAAACAGTGCC
TTC**TAG**AGCCGGGCTGGGAATGGGGACACGGTGTCCACCATCCCCAGCTGGTGGCCCTGTGCC
TGGGCCCTGGGCTGATGGAAGATGGTCCGTGCCCAGGCCCTTGGCTGCAGGCAACACTTTAGC
TTGGGTCCACCATGCAGAACACCAATATTAACACGCTGCCTGGTCTGTCTGGATCCCGAGGTA
TGGCAGAGGTGCAAGACCTAGTCCCCTTTCCTCTAACTCACTGCCTAGGAGGCTGGCCAAGGT
GTCCAGGGTCCTCTAGCCCACTCCCTGCCTACACACACAGCCTATATCAAACATGCACACGGG
CGAGCTTTCTCTCCGACTTCCCCTGGGCAAGAGATGGGACAAGCAGTCCCTTAATATTGAGGC
TGCAGCAGGTGCTGGGCTGGACTGGCCATTTTTCTGGGGGTAGGATGAAGAGAAGGCACACAG
AGATTCTGGATCTCCTGCTGCCTTTTCTGGAGTTTGTAAAATTGTTCCTGAATACAAGCCTAT
GCGTGA

# FIGURE 320

MRGTPKTHLLAFSLLCLLSKVRTQLCPTPCTCPWPPPRCPLGVPLVLDGCGCCRVCARRLGEP
CDQLHVCDASQGLVCQPGAGPGGRGALCLLAEDDSSCEVNGRLYREGETFQPHCSIRCRCEDG
GFTCVPLCSEDVRLPSWDCPHPRRVEVLGKCCPEWVCGQGGGLGTQPLPAQGPQFSGLVSSLP
PGVPCPEWSTAWGPCSTTCGLGMATRVSNQNRFCRLETQRRLCLSRPCPPSRGRSPQNSAF

**Important features:**

Signal sequence:
Amino acids 1-23

**N-myristoylation sites:**
Amino acids 3-9;49-55;81-87;85-91;126-132;164-170;166-172;
167-173;183-189;209-215

**Insulin-like growth factor binding proteins signature:**
Amino acids 49-65

**von Willebrand C1 domain:**
Amino acids 107-124

**Thrombospondin 1 Homology Block:**
Amino acids 201-216

**IGF binding protein site:**
Amino acids 49-58

# FIGURE 321

AGAACCTCAGAAATGTGAGTTATTTGGGAATGGCTGTTTGTAAATGTCCTTACGTAAGCCAAG
AGGAGGTCTTGACTTGGGGTCCCAGGGGTACCGCAGATCCCAGGGACTGGAGCAGCACTAGCA
AGCTCTGGAGGATGAGCCAGGAGTCTGGAATTGAGGCTGAGCCAAAGACCCCAGGGCCGTCTC
AGTCTCATAAAAGGGGATCAGGCAGGAGGAGTTTGGGAGAAACCTGAGAAGGGCCTGATTTGC
AGCATC**ATG**ATGGGCCTCTCCTTGGCCTCTGCTGTGCTCCTGGCCTCCCTCCTGAGTCTCCAC
CTTGGAACTGCCACACGTGGGAGTGACATATCCAAGACCTGCTGCTTCCAATACAGCCACAAG
CCCCTTCCCTGGACCTGGGTGCGAAGCTATGAATTCACCAGTAACAGCTGCTCCCAGCGGGCT
GTGATATTCACTACCAAAAGAGGCAAGAAAGTCTGTACCCATCCAAGGAAAAAATGGGTGCAA
AAATACATTTCTTTACTGAAAACTCCGAAACAATTG**TGA**CTCAGCTGAATTTTCATCCGAGGA
CGCTTGGACCCCGCTCTTGGCTCTGCAGCCCTCTGGGGAGCCTGCGGAATCTTTTCTGAAGGC
TACATGGACCCGCTGGGGAGGAGAGGGTGTTTCCTCCCAGAGTTACTTTAATAAAGGTTGTTC
ATAGAGTTGAAAAAAAAAAAAAAAAAAAAAAAAAAAAAAAAAAAAAAAAAAAAAAAAAAAAAA
AAAAAAAAAAAAAAAAAAAAAAAAAAA

# FIGURE 322

MMGLSLASAVLLASLLSLHLGTATRGSDISKTCCFQYSHKPLPWTWVRSYEFTSNSCSQRAVI
FTTKRGKKVCTHPRKKWVQKYISLLKTPKQL

**Important features:**

**Signal peptide:**

amino acids 1-23

**N-myristoylation sites.**

amino acids 3-9, 26-32

**Amidation site.**

amino acids 68-72

**Small cytokines (intecrine/chemokine).**

amino acids 23-88

# FIGURE 323

ACCGAGCCGAGCGGACCGAAGGCGCGCCCGAG**ATG**CAGGTGAGCAAGAGGATGCTGGCGGGGGGCGTGAGGAGCA
TGCCCAGCCCCCTCCTGGCCTGCTGGCAGCCCATCCTCCTGCTGGTGCTGGGCTCAGTGCTGTCAGGCTCGGCCA
CGGGCTGCCCGCCCCGCTGCGAGTGCTCCGCCCAGGACCGCGCTGTGCTGTGCCACCGCAAGTGCTTTGTGGCAG
TCCCCGAGGGCATCCCCACCGAGACGCGCCTGCTGGACCTAGGCAAGAACCGCATCAAAACGCTCAACCAGGACG
AGTTCGCCAGCTTCCCGCACCTGGAGGAGCTGGAGCTCAACGAGAACATCGTGAGCGCCGTGGAGCCCGGCGCCT
TCAACAACCTCTTCAACCTCCGGACGCTGGGTCTCCGCAGCAACCGCCTGAAGCTCATCCCGCTAGGCGTCTTCA
CTGGCCTCAGCAACCTGACCAAGCAGGACATCAGCGAGAACAAGATCGTTATCCTACTGGACTACATGTTTCAGG
ACCTGTACAACCTCAAGTCACTGGAGGTTGGCGACAATGACCTCGTCTACATCTCTCACCGCGCCTTCAGCGGCC
TCAACAGCCTGGAGCAGCTGACGCTGGAGAAATGCAACCTGACCTCCATCCCCACCGAGGCGCTGTCCCACCTGC
ACGGCCTCATCGTCCTGAGGCTCCGGCACCTCAACATCAATGCCATCCGGGACTACTCCTTCAAGAGGCTGTACC
GACTCAAGGTCTTGGAGATCTCCCACTGGCCCTACTTGGACACCATGACACCCAACTGCCTCTACGGCCTCAACC
TGACGTCCCTGTCCATCACACACTGCAATCTGACCGCTGTGCCCTACCTGGCCGTCCGCCACCTAGTCTATCTCC
GCTTCCTCAACCTCTCCTACAACCCCATCAGCACCATTGAGGGCTCCATGTTGCATGAGCTGCTCCGGCTGCAGG
AGATCCAGCTGGTGGGCGGGCAGCTGGCCGTGGTGGAGCCCTATGCCTTCCGCGGCCTCAACTACCTGCGCGTGC
TCAATGTCTCTGGCAACCAGCTGACCACACTGGAGGAATCAGTCTTCCACTCGGTGGGCAACCTGGAGACACTCA
TCCTGGACTCCAACCCGCTGGCCTGCGACTGTCGGCTCCTGTGGGTGTTCCGGCGCCGCTGGCGGCTCAACTTCA
ACCGGCAGCAGCCCACGTGCGCCACGCCCGAGTTTGTCCAGGGCAAGGAGTTCAAGGACTTCCCTGATGTGCTAC
TGCCCAACTACTTCACCTGCCGCCGCGCCCGCATCCGGGACCGCAAGGCCCAGCAGGTGTTTGTGGACGAGGGCC
ACACGGTGCAGTTTGTGTGCCGGGCCGATGGCGACCCGCCGCCCGCCATCCTCTGGCTCTCACCCCGAAAGCACC
TGGTCTCAGCCAAGAGCAATGGGCGGCTCACAGTCTTCCCTGATGGCACGCTGGAGGTGCGCTACGCCCAGGTAC
AGGACAACGGCACGTACCTGTGCATCGCGGCCAACGCGGGCGGCAACGACTCCATGCCCGCCCACCTGCATGTGC
GCAGCTACTCGCCCGACTGGCCCCATCAGCCCAACAAGACCTTCGCTTTCATCTCCAACCAGCCGGGCGAGGGAG
AGGCCAACAGCACCCGCGCCACTGTGCCTTTCCCCTTCGACATCAAGACCCTCATCATCGCCACCACCATGGGCT
TCATCTCTTTCCTGGGCGTCGTCCTCTTCTGCCTGGTGCTGCTGTTTCTCTGGAGCCGGGGCAAGGGCAACACAA
AGCACAACATCGAGATCGAGTATGTGCCCCGAAAGTCGGACGCAGGCATCAGCTCCGCCGACGCGCCCCGCAAGT
TCAACATGAAGATGATA**TGA**GGCCGGGGCGGGGGGCAGGGACCCCCGGGCGGCCGGGCAGGGGAAGGGGCCTGGT
CGCCACCTGCTCACTCTCCAGTCCTTCCCACCTCCTCCCTACCCTTCTACACACGTTCTCTTTCTCCCTCCCGCC
TCCGTCCCCTGCTGCCCCCCGCCAGCCCTCACCACCTGCCCTCCTTCTACCAGGACCTCAGAAGCCCAGACCTGG
GGACCCCACCTACACAGGGGCATTGACAGACTGGAGTTGAAAGCCGACGAACCGACACGCGGCAGAGTCAATAAT
TCAATAAAAAAGTTACGAACTTTCTCTGTAACTTGGGTTTCAATAATTATGGATTTTTATGAAAACTTGAAATAA
TAAAAAGAGAAAAAAACTAAAAAAAAAAAAAAAAAAAAA

# FIGURE 324

MQVSKRMLAGGVRSMPSPLLACWQPILLLVLGSVLSGSATGCPPRCECSAQDRAVLCHRKCFVAVPEGIPTETRL
LDLGKNRIKTLNQDEFASFPHLEELELNENIVSAVEPGAFNNLFNLRTLGLRSNRLKLIPLGVFTGLSNLTKQDI
SENKIVILLDYMFQDLYNLKSLEVGDNDLVYISHRAFSGLNSLEQLTLEKCNLTSIPTEALSHLHGLIVLRLRHL
NINAIRDYSFKRLYRLKVLEISHWPYLDTMTPNCLYGLNLTSLSITHCNLTAVPYLAVRHLVYLRFLNLSYNPIS
TIEGSMLHELLRLQEIQLVGGQLAVVEPYAFRGLNYLRVLNVSGNQLTTLEESVFHSVGNLETLILDSNPLACDC
RLLWVFRRRWRLNFNRQQPTCATPEFVQGKEFKDFPDVLLPNYFTCRRARIRDRKAQQVFVDEGHTVQFVCRADG
DPPPAILWLSPRKHLVSAKSNGRLTVFPDGTLEVRYAQVQDNGTYLCIAANAGGNDSMPAHLHVRSYSPDWPHQP
NKTFAFISNQPGEGEANSTRATVPFPFDIKTLIIATTMGFISFLGVVLFCLVLLFLWSRGKGNTKHNIEIEYVPR
KSDAGISSADAPRKFNMKMI

**Important features:**

**Signal sequence:**

amino acids 1-41

**Transmembrane domain:**

amino acids 556-578

**N-glycosylation site.**

amino acids 144-148, 202-206, 264-268, 274-278, 293-297, 341-345, 492-496, 505-509, 526-530, 542-546

**Casein kinase II phosphorylation site.**

amino acids 49-53, 108-112, 146-150, 300-304, 348-352, 349-353, 607-611

**Tyrosine kinase phosphorylation site.**

amino acids 590-598

**N-myristoylation site.**

amino acids 10-16, 32-38, 37-43, 113-119, 125-131, 137-143, 262-268, 320-326, 344-350, 359-365, 493-499, 503-509, 605-611

**Prokaryotic membrane lipoprotein lipid attachment site.**

amino acids 32-43

# FIGURE 325

CCCACGCGTCCGCCCACGCGTCCGAGGGACAAGAGAGAAGAGAGACTGAAACAGGGAGAAGAG
GCAGGAGAGGAGGAGGTGGGGAGAGCACGAAGCTGGAGGCCGACACTGAGGGAGGGCGGGAGG
AGGTGAAGAAGGAGAGAGGGGAGAAGAGGCAGGAGCTGGAAAGGAGAGAGGGAGGAGGAGGAG
GAGATGCGGGATGGAGACCTGGAGTTAGGTGGCTTGGGAGAGCTTAATGAAAAGAGAACGGAG
AGGAGGTGTGGGTTAGGAACCAAGAGGTAGCCCTGTGGGCAGCAGAAGGCTGAGAGGAGTAGG
AAGATCAGGAGCTAGAGGGAGACTGGAGGGTTCCGGGAAAAGAGCAGAGGAAAGAGGAAAGAC
ACAGAGAGACGGGAGAGAGAAGAAGAGTGGGTTTGAAGGGCGGATCTCAGTCCCTGGCTGCTT
TGGCATTTGGGGAACTGGGACTCCCTGTGGGGAGGAGAGGAAAGCTGGAAGTCCTGGAGGGAC
AGGGTCCCAGAAGGAGGGGACAGAGGAGCTGAGAGAGGGGGGCAGGGCGTTGGGCAGGGGTCC
CTCGGAGGCCTCCTGGGG**ATG**GGGGCTGCAGCTCGTCTGAGCGCCCCTCGAGCGCTGGTACTC
TGGGCTGCACTGGGGGCAGCAGCTCACATCGGACCAGCACCTGACCCCGAGGACTGGTGGAGC
TACAAGGATAATCTCCAGGGAAACTTCGTGCCAGGGCCTCCTTTCTGGGGCCTGGTGAATGCA
GCGTGGAGTCTGTGTGCTGTGGGGAAGCGGCAGAGCCCCGTGGATGTGGAGCTGAAGAGGGTT
CTTTATGACCCCTTTCTGCCCCCATTAAGGCTCAGCACTGGAGGAGAGAAGCTCCGGGGAACC
TTGTACAACACCGGCCGACATGTCTCCTTCCTGCCTGCACCCCGACCTGTGGTCAATGTGTCT
GGAGGTCCCCTCCTTTACAGCCACCGACTCAGTGAACTGCGGCTGCTGTTTGGAGCTCGCGAC
GGAGCCGGCTCGGAACATCAGATCAACCACCAGGGCTTCTCTGCTGAGGTGCAGCTCATTCAC
TTCAACCAGGAACTCTACGGGAATTTCAGCGCTGCCTCCCGCGGCCCCAATGGCCTGGCCATT
CTCAGCCTCTTTGTCAACGTTGCCAGTACCTCTAACCCATTCCTCAGTCGCCTCCTTAACCGC
GACACCATCACTCGCATCTCCTACAAGAATGATGCCTACTTTCTTCAAGACCTGAGCCTGGAG
CTCCTGTTCCCTGAATCCTTCGGCTTCATCACCTATCAGGGCTCTCTCAGCACCCCGCCCTGC
TCCGAGACTGTCACCTGGATCCTCATTGACCGGGCCCTCAATATCACCTCCCTTCAGATGCAC
TCCCTGAGACTCCTGAGCCAGAATCCTCCATCTCAGATCTTCCAGAGCCTCAGCGGTAACAGC
CGGCCCCTGCAGCCCTTGGCCCACAGGGCACTGAGGGGCAACAGGGACCCCCGGCACCCCGAG
AGGCGCTGCCGAGGCCCCAACTACCGCCTGCATGTGGATGGTGTCCCCCATGGTCGC**TGA**GAC
TCCCCTTCGAGGATTGCACCCGCCCGTCCTAAGCCTCCCCACAAGGCGAGGGGAGTTACCCCT
AAAACAAAGCTATTAAAGGGACAGAATACTTA

# FIGURE 326

MGAAARLSAPRALVLWAALGAAAHIGPAPDPEDWWSYKDNLQGNFVPGPPFWGLVNAAWSLCA
VGKRQSPVDVELKRVLYDPFLPPLRLSTGGEKLRGTLYNTGRHVSFLPAPRPVVNVSGGPLLY
SHRLSELRLLFGARDGAGSEHQINHQGFSAEVQLIHFNQELYGNFSAASRGPNGLAILSLFVN
VASTSNPFLSRLLNRDTITRISYKNDAYFLQDLSLELLFPESFGFITYQGSLSTPPCSETVTW
ILIDRALNITSLQMHSLRLLSQNPPSQIFQSLSGNSRPLQPLAHRALRGNRDPRHPERRCRGP
NYRLHVDGVPHGR

**Important features:**

**Signal peptide:**

Amino acids 1-23

**Transmembrane domain:**

Amino acids 177-199

**N-glycosylation sites:**

Amino acids 118-122;170-174;260-264

**Eukaryotic-type carbonic anhydrases proteins:**

Amino acids 222-271;128-165;45-93

# FIGURE 327

GGACTAATCTGTGGGAGCAGTTTATTCCAGTATCACCCAGGGTGCAGCCACACCAGGACTGTGTTGAAGGGTGTT
TTTTTTCTTTTAAATGTAATACCTCCTCATCTTTTCTTCTTACACAGTGTCTGAGAACATTTACATTATAGATAA
GTAGTACATGGTGGATAACTTCTACTTTTAGGAGGACTACTCTCTTCTGACAGTCCTAGACTGGTCTTCTACACT
AAGACACC**ATG**AAGGAGTATGTGCTCCTATTATTCCTGGCTTTGTGCTCTGCCAAACCCTTCTTTAGCCCTTCAC
ACATCGCACTGAAGAATATGATGCTGAAGGATATGGAAGACACAGATGATGATGATGATGATGATGATGATGATG
ATGATGATGAGGACAACTCTCTTTTTCCAACAAGAGAGCCAAGAAGCCATTTTTTTCCATTTGATCTGTTTCCAA
TGTGTCCATTTGGATGTCAGTGCTATTCACGAGTTGTACATTGCTCAGATTTAGGTTTGACCTCAGTCCCAACCA
ACATTCCATTTGATACTCGAATGCTTGATCTTCAAAACAATAAAATTAAGGAAATCAAAGAAAATGATTTTAAAG
GACTCACTTCACTTTATGGTCTGATCCTGAACAACAACAAGCTAACGAAGATTCACCCAAAAGCCTTTCTAACCA
CAAAGAAGTTGCGAAGGCTGTATCTGTCCCACAATCAACTAAGTGAAATACCACTTAATCTTCCCAAATCATTAG
CAGAACTCAGAATTCATGAAAATAAAGTTAAGAAAATACAAAAGGACACATTCAAAGGAATGAATGCTTTACACG
TTTTGGAAATGAGTGCAAACCCTCTTGATAATAATGGGATAGAGCCAGGGGCATTTGAAGGGGTGACGGTGTTCC
ATATCAGAATTGCAGAAGCAAAACTGACCTCAGTTCCTAAAGGCTTACCACCAACTTTATTGGAGCTTCACTTAG
ATTATAATAAAATTTCAACAGTGGAACTTGAGGATTTTAAACGATACAAAGAACTACAAAGGCTGGGCCTAGGAA
ACAACAAAATCACAGATATCGAAAATGGGAGTCTTGCTAACATACCACGTGTGAGAGAAATACATTTGGAAAACA
ATAAACTAAAAAAAATCCCTTCAGGATTACCAGAGTTGAAATACCTCCAGATAATCTTCCTTCATTCTAATTCAA
TTGCAAGAGTGGGAGTAAATGACTTCTGTCCAACAGTGCCAAAGATGAAGAAATCTTTATACAGTGCAATAAGTT
TATTCAACAACCCGGTGAAATACTGGGAAATGCAACCTGCAACATTTCGTTGTGTTTTGAGCAGAATGAGTGTTC
AGCTTGGGAACTTTGGAATG**TAA**TAATTAGTAATTGGTAATGTCCATTTAATATAAGATTCAAAAATCCCTACAT
TTGGAATACTTGAACTCTATTAATAATGGTAGTATTATATATACAAGCAAATATCTATTCTCAAGTGGTAAGTCC
ACTGACTTATTTTATGACAAGAAATTTCAACGGAATTTTGCCAAACTATTGATACATAAGGGGTTGAGAGAAACA
AGCATCTATTGCAGTTTCCTTTTTGCGTACAAATGATCTTACATAAATCTCATGCTTGACCATTCCTTTCTTCAT
AACAAAAAAGTAAGATATTCGGTATTTAACACTTTGTTATCAAGCACATTTTAAAAAGAACTGTACTGTAAATGG
AATGCTTGACTTAGCAAAATTTGTGCTCTTTCATTTGCTGTTAGAAAAACAGAATTAACAAAGACAGTAATGTGA
AGAGTGCATTACACTATTCTTATTCTTTAGTAACTTGGGTAGTACTGTAATATTTTTAATCATCTTAAAGTATGA
TTTGATATAATCTTATTGAAATTACCTTATCATGTCTTAGAGCCCGTCTTTATGTTTAAAACTAATTTCTTAAAA
TAAAGCCTTCAGTAAATGTTCATTACCAACTTGATAAATGCTACTCATAAGAGCTGGTTTGGGGCTATAGCATAT
GCTTTTTTTTTTTTAATTATTACCTGATTTAAAAATCTCTGTAAAAACGTGTAGTGTTTCATAAAATCTGTAACT
CGCATTTTAATGATCCGCTATTATAAGCTTTTAATAGCATGAAAATTGTTAGGCTATATAACATTGCCACTTCAA
CTCTAAGGAATATTTTTGAGATATCCCTTTGGAAGACCTTGCTTGGAAGAGCCTGGACACTAACAATTCTACACC
AAATTGTCTCTTCAAATACGTATGGACTGGATAACTCTGAGAAACACATCTAGTATAACTGAATAAGCAGAGCAT
CAAATTAAACAGACAGAAACCGAAAGCTCTATATAAATGCTCAGAGTTCTTTATGTATTTCTTATTGGCATTCAA
CATATGTAAAATCAGAAAACAGGGAAATTTTCATTAAAAATATTGGTTTGAAAT

# FIGURE 328

MKEYVLLLFLALCSAKPFFSPSHIALKNMMLKDMEDTDDDDDDDDDDDDDDEDNSLFPTREPRS
HFFPFDLFPMCPFGCQCYSRVVHCSDLGLTSVPTNIPFDTRMLDLQNNKIKEIKENDFKGLTS
LYGLILNNNKLTKIHPKAFLTTKKLRRLYLSHNQLSEIPLNLPKSLAELRIHENKVKKIQKDT
FKGMNALHVLEMSANPLDNNGIEPGAFEGVTVFHIRIAEAKLTSVPKGLPPTLLELHLDYNKI
STVELEDFKRYKELQRLGLGNNKITDIENGSLANIPRVREIHLENNKLKKIPSGLPELKYLQI
IFLHSNSIARVGVNDFCPTVPKMKKSLYSAISLFNNPVKYWEMQPATFRCVLSRMSVQLGNFGM

**Important features:**

**Signal sequence.**

amino acids 1-15

**N-glycosylation site.**

amino acids 281-285

**N-myristoylation sites.**

amino acids 129-135, 210-216, 214-220, 237-243, 270-276, 282-288

**Leucine zipper pattern.**

amino acids 154-176

# FIGURE 329

GGGGTCTCCCTCAGGGCCGGGAGGCACAGCGGTCCCTGCTTGCTGAAGGGCTGGATGTACGCA
TCCGCAGGTTCCCGCGGACTTGGGGGCGCCCGCTGAGCCCCGGCGCCCGCAGAAGACTTGTGT
TTGCCTCCTGCAGCCTCAACCCGGAGGGCAGCGAGGGCCTACCACC**ATG**ATCACTGGTGTGTT
CAGCATGCGCTTGTGGACCCCAGTGGGCGTCCTGACCTCGCTGGCGTACTGCCTGCACCAGCG
GCGGGTGGCCCTGGCCGAGCTGCAGGAGGCCGATGGCCAGTGTCCGGTCGACCGCAGCCTGCT
GAAGTTGAAAATGGTGCAGGTCGTGTTTCGACACGGGGCTCGGAGTCCTCTCAAGCCGCTCCC
GCTGGAGGAGCAGGTAGAGTGGAACCCCCAGCTATTAGAGGTCCCACCCCAAACTCAGTTTGA
TTACACAGTCACCAATCTAGCTGGTGGTCCGAAACCATATTCTCCTTACGACTCTCAATACCA
TGAGACCACCCTGAAGGGGGGCATGTTTGCTGGGCAGCTGACCAAGGTGGGCATGCAGCAAAT
GTTTGCCTTGGGAGAGAGACTGAGGAAGAACTATGTGGAAGACATTCCCTTTCTTTCACCAAC
CTTCAACCCACAGGAGGTCTTTATTCGTTCCACTAACATTTTTCGGAATCTGGAGTCCACCCG
TTGTTTGCTGGCTGGGCTTTTCCAGTGTCAGAAAGAAGGACCCATCATCATCCACACTGATGA
AGCAGATTCAGAAGTCTTGTATCCCAACTACCAAAGCTGCTGGAGCCTGAGGCAGAGAACCAG
AGGCCGGAGGCAGACTGCCTCTTTACAGCCAGGAATCTCAGAGGATTTGAAAAAGGTGAAGGA
CAGGATGGGCATTGACAGTAGTGATAAAGTGGACTTCTTCATCCTCCTGGACAACGTGGCTGC
CGAGCAGGCACACAACCTCCCAAGCTGCCCCATGCTGAAGAGATTTGCACGGATGATCGAACA
GAGAGCTGTGGACACATCCTTGTACATACTGCCCAAGGAAGACAGGGAAAGTCTTCAGATGGC
AGTAGGCCCATTCCTCCACATCCTAGAGAGCAACCTGCTGAAAGCCATGGACTCTGCCACTGC
CCCCGACAAGATCAGAAAGCTGTATCTCTATGCGGCTCATGATGTGACCTTCATACCGCTCTT
AATGACCCTGGGGATTTTTGACCACAAATGGCCACCGTTTGCTGTTGACCTGACCATGGAACT
TTACCAGCACCTGGAATCTAAGGAGTGGTTTGTGCAGCTCTATTACCACGGGAAGGAGCAGGT
GCCGAGAGGTTGCCCTGATGGGCTCTGCCCGCTGGACATGTTCTTGAATGCCATGTCAGTTTA
TACCTTAAGCCCAGAAAAATACCATGCACTCTGCTCTCAAACTCAGGTGATGGAAGTTGGAAA
TGAAGAG**TAA**CTGATTTATAAAAGCAGGATGTGTTGATTTAAAATAAAGTGCCTTTATACAATG

# FIGURE 330

MITGVFSMRLWTPVGVLTSLAYCLHQRRVALAELQEADGQCPVDRSLLKLKMVQVVFRHGARSPLKPLPLEEQVE
WNPQLLEVPPQTQFDYTVTNLAGGPKPYSPYDSQYHETTLKGGMFAGQLTKVGMQQMFALGERLRKNYVEDIPFL
SPTFNPQEVFIRSTNIFRNLESTRCLLAGLFQCQKEGPIIIHTDEADSEVLYPNYQSCWSLRQRTRGRRQTASLQ
PGISEDLKKVKDRMGIDSSDKVDFFILLDNVAAEQAHNLPSCPMLKRFARMIEQRAVDTSLYILPKEDRESLQMA
VGPFLHILESNLLKAMDSATAPDKIRKLYLYAAHDVTFIPLLMTLGIFDHKWPPFAVDLTMELYQHLESKEWFVQ
LYYHGKEQVPRGCPDGLCPLDMFLNAMSVYTLSPEKYHALCSQTQVMEVGNEE

**Important features:**

**Signal sequence:**

amino acids 1-23

**cAMP- and cGMP-dependent protein kinase phosphorylation site.**

amino acids 218-222

**Casein kinase II phosphorylation site.**

amino acids 87-91, 104-108, 320-324

**Tyrosine kinase phosphorylation site.**

amino acids 280-288

**N-myristoylation site.**

amino acids 15-21, 117-123, 118-124, 179-185, 240-246, 387-393

**Amidation site.**

amino acids 216-220

**Leucine zipper pattern.**

amino acids 10-32

**Histidine acid phosphatases phosphohistidine signature.**

amino acids 50-65

# FIGURE 331

CGAGGGCTTTTCCGGCTCCGGAATGGCACATGTGGGAATCCCAGTCTTGTTGGCTACAACATTTTTCCCTTTCCT
AACAAGTTCTAACAGCTGTTCTAACAGCTAGTGATCAGGGGTTCTTCTTGCTGGAGAAGAAAGGGCTGAGGGCAG
AGCAGGGCACTCTCACTCAGGGTGACCAGCTCCTTGCCTCTCTGTGGATAACAGAGCATGAGAAAGTGAAGAGAT
GCAGCGGAGTGAGGTGATGGAAGTCTAAAATAGGAAGGAATTTTGTGTGCAATATCAGACTCTGGGAGCAGTTGA
CCTGGAGAGCCTGGGGGAGGGCCTGCCTAACAAGCTTTCAAAAAACAGGAGCGACTTCCACTGGGCTGGGATAAG
ACGTGCCGGTAGGATAGGGAAGACTGGGTTTAGTCCTAATATCAAATTGACTGGCTGGGTGAACTTCAACAGCCT
TTTAACCTCTCTGGGAGATGAAAACGATGGCTTAAGGGGCCAGAAATAGAGATGCTTTGTAAAATAAAATTTTAA
AAAAAGCAAGTATTTTATAGCATAAAGGCTAGAGACCAAAATAGATAACAGGATTCCCTGAACATTCCTAAGAGG
GAGAAAGTATGTTAAAAATAGAAAAACCAAAATGCAGAAGGAGGAGACTCACAGAGCTAAACCAGG**ATG**GGGACC
CTGGGTCAGGCCAGCCTCTTTGCTCCTCCCGGAAATTATTTTTGGTCTGACCACTCTGCCTTGTGTTTTGCAGAA
TCATGTGAGGGCCAACCGGGGAAGGTGGAGCAGATGAGCACACACAGGAGCCGTCTCCTCACCGCCGCCCCTCTC
AGCATGGAACAGAGGCAGCCCTGGCCCCGGGCCCTGGAGGTGGACAGCCGCTCTGTGGTCCTGCTCTCAGTGGTC
TGGGTGCTGCTGGCCCCCCCAGCAGCCGGCATGCCTCAGTTCAGCACCTTCCACTCTGAGAATCGTGACTGGACC
TTCAACCACTTGACCGTCCACCAAGGGACGGGGGCCGTCTATGTGGGGGCCATCAACCGGGTCTATAAGCTGACA
GGCAACCTGACCATCCAGGTGGCTCATAAGACAGGGCCAGAAGAGGACAACAAGTCTCGTTACCCGCCCCTCATC
GTGCAGCCCTGCAGCGAAGTGCTCACCCTCACCAACAATGTCAACAAGCTGCTCATCATTGACTACTCTGAGAAC
CGCCTGCTGGCCTGTGGGAGCCTCTACCAGGGGGTCTGCAAGCTGCTGCGGCTGGATGACCTCTTCATCCTGGTG
GAGCCATCCCACAAGAAGGAGCACTACCTGTCCAGTGTCAACAAGACGGGCACCATGTACGGGGTGATTGTGCGC
TCTGAGGGTGAGGATGGCAAGCTCTTCATCGGCACGGCTGTGGATGGGAAGCAGGATTACTTCCCGACCCTGTCC
AGCCGGAAGCTGCCCCGAGACCCTGAGTCCTCAGCCATGCTCGACTATGAGCTACACAGCGATTTTGTCTCCTCT
CTCATCAAGATCCCTTCAGACACCCTGGCCCTGGTCTCCCACTTTGACATCTTCTACATCTACGGCTTTGCTAGT
GGGGGCTTTGTCTACTTTCTCACTGTCCAGCCCGAGACCCCTGAGGGTGTGGCCATCAACTCCGCTGGAGACCTC
TTCTACACCTCACGCATCGTGCGGCTCTGCAAGGATGACCCCAAGTTCCACTCATACGTGTCCCTGCCCTTCGGC
TGCACCCGGGCCGGGGTGGAATACCGCCTCCTGCAGGCTGCTTACCTGGCCAAGCCTGGGGACTCACTGGCCCAG
GCCTTCAATATCACCAGCCAGGACGATGTACTCTTTGCCATCTTCTCCAAAGGGCAGAAGCAGTATCACCACCCG
CCCGATGACTCTGCCCTGTGTGCCTTCCCTATCCGGGCCATCAACTTGCAGATCAAGGAGCGCCTGCAGTCCTGC
TACCAGGGCGAGGGCAACCTGGAGCTCAACTGGCTGCTGGGGAAGGACGTCCAGTGCACGAAGGCGCCTGTCCCC
ATCGATGATAACTTCTGTGGACTGGACATCAACCAGCCCCTGGGAGGCTCAACTCCAGTGGAGGGCCTGACCCTG
TACACCACCAGCAGGGACCGCATGACCTCTGTGGCCTCCTACGTTTACAACGGCTACAGCGTGGTTTTTGTGGGG
ACTAAGAGTGGCAAGCTGAAAAAGGTAAGAGTCTATGAGTTCAGATGCTCCAATGCCATTCACCTCCTCAGCAAA
GAGTCCCTCTTGGAAGGTAGCTATTGGTGGAGATTTAACTATAGGCAACTTTATTTTCTTGGGGAACAAAGG**TGA**
AATGGGGAGGTAAGAAGGGGTTAATTTTGTGACTTAGCTTCTAGCTACTTCCTCCAGCCATCAGTCATTGGGTAT
GTAAGGAATGCAAGCGTATTTCAATATTTCCCAAACTTTAAGAAAAAACTTTAAGAAGGTACATCTGCAAAAGCAAA

# FIGURE 332

MGTLGQASLFAPPGNYFWSDHSALCFAESCEGQPGKVEQMSTHRSRLLTAAPLSMEQRQPWPR
ALEVDSRSVVLLSVVWVLLAPPAAGMPQFSTFHSENRDWTFNHLTVHQGTGAVYVGAINRVYK
LTGNLTIQVAHKTGPEEDNKSRYPPLIVQPCSEVLTLTNNVNKLLIIDYSENRLLACGSLYQG
VCKLLRLDDLFILVEPSHKKEHYLSSVNKTGTMYGVIVRSEGEDGKLFIGTAVDGKQDYFPTL
SSRKLPRDPESSAMLDYELHSDFVSSLIKIPSDTLALVSHFDIFYIYGFASGGFVYFLTVQPE
TPEGVAINSAGDLFYTSRIVRLCKDDPKFHSYVSLPFGCTRAGVEYRLLQAAYLAKPGDSLAQ
AFNITSQDDVLFAIFSKGQKQYHHPPDDSALCAFPIRAINLQIKERLQSCYQGEGNLELNWLL
GKDVQCTKAPVPIDDNFCGLDINQPLGGSTPVEGLTLYTTSRDRMTSVASYVYNGYSVVFVGT
KSGKLKKVRVYEFRCSNAIHLLSKESLLEGSYWWRFNYRQLYFLGEQR

**Important features:**

**Signal sequence:**

amino acids 1-32

**Transmembrane domain:**

amino acids 71-87

**N-glycosylation site.**

amino acids 130-134, 145-149, 217-221, 381-385

**Casein kinase II phosphorylation site.**

amino acids 139-143, 229-233, 240-244, 291-295, 324-328, 383-387, 384-388, 471-475, 481-485, 530-534

**N-myristoylation site.**

amino acids 220-226, 319-325, 353-359, 460-466, 503-509

# FIGURE 333

GCTGAGTCTGCTGCTCCTGCTGCTGCTGCTCCAGCCTGTAACCTGTGCCTACACCACGCCAGG
CCCCCCCAGAGCCCTCACCACGCTGGGCGCCCCCAGAGCCCACACC**ATG**CCGGGCACCTACGC
TCCCTCGACCACACTCAGTAGTCCCAGCACCCAGGGCCTGCAAGAGCAGGCACGGGCCCTGAT
GCGGGACTTCCCGCTCGTGGACGGCCACAACGACCTGCCCCTGGTCCTAAGGCAGGTTTACCA
GAAAGGGCTACAGGATGTTAACCTGCGCAATTTCAGCTACGGCCAGACCAGCCTGGACAGGCT
TAGAGATGGCCTCGTGGGCGCCCAGTTCTGGTCAGCCTATGTGCCATGCCAGACCCAGGACCG
GGATGCCCTGCGCCTCACCCTGGAGCAGATTGACCTCATACGCCGCATGTGTGCCTCCTATTC
TGAGCTGGAGCTTGTGACCTCGGCTAAAGCTCTGAACGACACTCAGAAATTGGCCTGCCTCAT
CGGTGTAGAGGGTGGCCACTCGCTGGACAATAGCCTCTCCATCTTACGTACCTTCTACATGCT
GGGAGTGCGCTACCTGACGCTCACCCACACCTGCAACACACCCTGGGCAGAGAGCTCCGCTAA
GGGCGTCCACTCCTTCTACAACAACATCAGCGGGCTGACTGACTTTGGTGAGAAGGTGGTGGC
AGAAATGAACCGCCTGGGCATGATGGTAGACTTATCCCATGTCTCAGATGCTGTGGCACGGCG
GGCCCTGGAAGTGTCACAGGCACCTGTGATCTTCTCCCACTCGGCTGCCCGGGGTGTGTGCAA
CAGTGCTCGGAATGTTCCTGATGACATCCTGCAGCTTCTGAAGAAGAACGGTGGCGTCGTGAT
GGTGTCTTTGTCCATGGGAGTAATACAGTGCAACCCATCAGCCAATGTGTCCACTGTGGCAGA
TCACTTCGACCACATCAAGGCTGTCATTGGATCCAAGTTCATCGGGATTGGTGGAGATTATGA
TGGGGCCGGCAAATTCCCTCAGGGGCTGGAAGACGTGTCCACATACCCGGTCCTGATAGAGGA
GTTGCTGAGTCGTGGCTGGAGTGAGGAAGAGCTTCAGGGTGTCCTTCGTGGAAACCTGCTGCG
GGTCTTCAGACAAGTGGAAAAGGTACAGGAAGAAAACAAATGGCAAAGCCCCTTGGAGGACAA
GTTCCCGGATGAGCAGCTGAGCAGTTCCTGCCACTCCGACCTCTCACGTCTGCGTCAGAGACA
GAGTCTGACTTCAGGCCAGGAACTCACTGAGATTCCCATACACTGGACAGCCAAGTTACCAGC
CAAGTGGTCAGTCTCAGAGTCCTCCCCCCACATGGCCCCAGTCCTTGCAGTTGTGGCCACCTT
CCCAGTCCTTATTCTGTGGCTC**TGA**TGACCCAGTTAGTCCTGCCAGATGTCACTGTAGCAAGC
CACAGACACCCCACAAAGTTCCCCTGTTGTGCAGGCACAAATATTTCCTGAAATAAATGTTTT
GGACATAG

# FIGURE 334

MPGTYAPSTTLSSPSTQGLQEQARALMRDFPLVDGHNDLPLVLRQVYQKGLQDVNLRNFSYGQ
TSLDRLRDGLVGAQFWSAYVPCQTQDRDALRLTLEQIDLIRRMCASYSELELVTSAKALNDTQ
KLACLIGVEGGHSLDNSLSILRTFYMLGVRYLTLTHTCNTPWAESSAKGVHSFYNNISGLTDF
GEKVVAEMNRLGMMVDLSHVSDAVARRALEVSQAPVIFSHSAARGVCNSARNVPDDILQLLKK
NGGVVMVSLSMGVIQCNPSANVSTVADHFDHIKAVIGSKFIGIGGDYDGAGKFPQGLEDVSTY
PVLIEELLSRGWSEEELQGVLRGNLLRVFRQVEKVQEENKWQSPLEDKFPDEQLSSSCHSDLS
RLRQRQSLTSGQELTEIPIHWTAKLPAKWSVSESSPHMAPVLAVVATFPVLILWL

**Important features:**

**N-glycosylation sites.**

amino acids 58-62, 123-127, 182-186, 273-277

**N-myristoylation sites.**

amino acids 72-78, 133-139, 234-240, 264-270, 334-340, 389-395

**Renal dipeptidase active site.**

amino acids 134-157

# FIGURE 335

CCCAGAAGTTCAAGGGCCCCCGGCCTCCTGCGCTCCTGCCGCCGGGACCCTCGACCTCCTCAG
AGCAGCCGGCTGCCGCCCCGGGAAG**ATG**GCGAGGAGGAGCCGCCACCGCCTCCTCCTGCTGCT
GCTGCGCTACCTGGTGGTCGCCCTGGGCTATCATAAGGCCTATGGGTTTTCTGCCCCAAAAGA
CCAACAAGTAGTCACAGCAGTAGAGTACCAAGAGGCTATTTTAGCCTGCAAAACCCCAAAGAA
GACTGTTTCCTCCAGATTAGAGTGGAAGAAACTGGGTCGGAGTGTCTCCTTTGTCTACTATCA
ACAGACTCTTCAAGGTGATTTTAAAAATCGAGCTGAGATGATAGATTTCAATATCCGGATCAA
AAATGTGACAAGAAGTGATGCGGGGAAATATCGTTGTGAAGTTAGTGCCCCATCTGAGCAAGG
CCAAAACCTGGAAGAGGATACAGTCACTCTGGAAGTATTAGTGGCTCCAGCAGTTCCATCATG
TGAAGTACCCTCTTCTGCTCTGAGTGGAACTGTGGTAGAGCTACGATGTCAAGACAAAGAAGG
GAATCCAGCTCCTGAATACACATGGTTTAAGGATGGCATCCGTTTGCTAGAAAATCCCAGACT
TGGCTCCCAAAGCACCAACAGCTCATACACAATGAATACAAAAACTGGAACTCTGCAATTTAA
TACTGTTTCCAAACTGGACACTGGAGAATATTCCTGTGAAGCCCGCAATTCTGTTGGATATCG
CAGGTGTCCTGGGAAACGAATGCAAGTAGATGATCTCAACATAAGTGGCATCATAGCAGCCGT
AGTAGTTGTGGCCTTAGTGATTTCCGTTTGTGGCCTTGGTGTATGCTATGCTCAGAGGAAAGG
CTACTTTTCAAAAGAAACCTCCTTCCAGAAGAGTAATTCTTCATCTAAAGCCACGACAATGAG
TGAAAATGTGCAGTGGCTCACGCCTGTAATCCCAGCACTTTGGAAGGCCGCGGCGGGCGGATC
ACGAGGTCAGGAGTTC**TAG**ACCAGTCTGGCCAATATGGTGAAACCCCATCTCTACTAAAATAC
AAAAATTAGCTGGGCATGGTGGCATGTGCCTGCAGTTCCAGCTGCTTGGGAGACAGGAGAATC
ACTTGAACCCGGGAGGCGGAGGTTGCAGTGAGCTGAGATCACGCCACTGCAGTCCAGCCTGGG
TAACAGAGCAAGATTCCATCTCAAAAAATAAAATAAATAAATAAATAAATACTGGTTTTTACC
TGTAGAATTCTTACAATAAATATAGCTTGATATTC

# FIGURE 336

MARRSRHRLLLLLLLRYLVVALGYHKAYGFSAPKDQQVVTAVEYQEAILACKTPKKTVSSRLEW
KKLGRSVSFVYYQQTLQGDFKNRAEMIDFNIRIKNVTRSDAGKYRCEVSAPSEQGQNLEEDTV
TLEVLVAPAVPSCEVPSSALSGTVVELRCQDKEGNPAPEYTWFKDGIRLLENPRLGSQSTNSS
YTMNTKTGTLQFNTVSKLDTGEYSCEARNSVGYRRCPGKRMQVDDLNISGIIAAVVVVALVIS
VCGLGVCYAQRKGYFSKETSFQKSNSSSKATTMSENVQWLTPVIPALWKAAAGGSRGQEF

**Important features:**

**Signal peptide:**

amino acids 1-20

**Transmembrane domain:**

amino acids 130-144, 238-258

**N-glycosylation site.**

amino acids 98-102, 187-191, 236-240, 277-281

**Casein kinase II phosphorylation site.**

amino acids 39-43, 59-63, 100-104, 149-153, 205-209, 284-288

**N-myristoylation site.**

amino acids 182-188, 239-245, 255-261, 257-263, 305-311

**Amidation site.**

amino acids 226-230

# FIGURE 337

GGAGCCGCCCTGGGTGTCAGCGGCTCGGCTCCCGCGCACGCTCCGGCCGTCGCGCAGCCTCGG
CACCTGCAGGTCCGTGCGTCCCGCGGCTGGCGCCCCTGACTCCGTCCCGGCCAGGGAGGGCC**A**
**TG**ATTTCCCTCCCGGGGCCCCTGGTGACCAACTTGCTGCGGTTTTTGTTCCTGGGGCTGAGTG
CCCTCGCGCCCCCCTCGCGGGCCCAGCTGCAACTGCACTTGCCCGCCAACCGGTTGCAGGCGG
TGGAGGGAGGGGAAGTGGTGCTTCCAGCGTGGTACACCTTGCACGGGGAGGTGTCTTCATCCC
AGCCATGGGAGGTGCCCTTTGTGATGTGGTTCTTCAAACAGAAAGAAAAGGAGGATCAGGTGT
TGTCCTACATCAATGGGGTCACAACAAGCAAACCTGGAGTATCCTTGGTCTACTCCATGCCCT
CCCGGAACCTGTCCCTGCGGCTGGAGGGTCTCCAGGAGAAAGACTCTGGCCCCTACAGCTGCT
CCGTGAATGTGCAAGACAAACAAGGCAAATCTAGGGGCCACAGCATCAAAACCTTAGAACTCA
ATGTACTGGTTCCTCCAGCTCCTCCATCCTGCCGTCTCCAGGGTGTGCCCCATGTGGGGGCAA
ACGTGACCCTGAGCTGCCAGTCTCCAAGGAGTAAGCCCGCTGTCCAATACCAGTGGGATCGGC
AGCTTCCATCCTTCCAGACTTTCTTTGCACCAGCATTAGATGTCATCCGTGGGTCTTTAAGCC
TCACCAACCTTTCGTCTTCCATGGCTGGAGTCTATGTCTGCAAGGCCCACAATGAGGTGGGCA
CTGCCCAATGTAATGTGACGCTGGAAGTGAGCACAGGGCCTGGAGCTGCAGTGGTTGCTGGAG
CTGTTGTGGGTACCCTGGTTGGACTGGGGTTGCTGGCTGGGCTGGTCCTCTTGTACCACCGCC
GGGGCAAGGCCCTGGAGGAGCCAGCCAATGATATCAAGGAGGATGCCATTGCTCCCCGGACCC
TGCCCTGGCCCAAGAGCTCAGACACAATCTCCAAGAATGGGACCCTTTCCTCTGTCACCTCCG
CACGAGCCCTCCGGCCACCCCATGGCCCTCCCAGGCCTGGTGCATTGACCCCCACGCCCAGTC
TCTCCAGCCAGGCCCTGCCCTCACCAAGACTGCCCACGACAGATGGGGCCCACCCTCAACCAA
TATCCCCCATCCCTGGTGGGGTTTCTTCCTCTGGCTTGAGCCGCATGGGTGCTGTGCCTGTGA
TGGTGCCTGCCCAGAGTCAAGCTGGCTCTCTGGTA**TGA**TGACCCCACCACTCATTGGCTAAAG
GATTTGGGGTCTCTCCTTCCTATAAGGGTCACCTCTAGCACAGAGGCCTGAGTCATGGGAAAG
AGTCACACTCCTGACCCTTAGTACTCTGCCCCCACCTCTCTTTACTGTGGGAAAACCATCTCA
GTAAGACCTAAGTGTCCAGGAGACAGAAGGAGAAGAGGAAGTGGATCTGGAATTGGGAGGAGC
CTCCACCCACCCCTGACTCCTCCTTATGAAGCCAGCTGCTGAAATTAGCTACTCACCAAGAGT
GAGGGGCAGAGACTTCCAGTCACTGAGTCTCCCAGGCCCCCTTGATCTGTACCCCACCCCTAT
CTAACACCACCCTTGGCTCCCACTCCAGCTCCCTGTATTGATATAACCTGTCAGGCTGGCTTG
GTTAGGTTTTACTGGGGCAGAGGATAGGGAATCTCTTATTAAAACTAACATGAAATATGTGTT
GTTTTCATTTGCAAATTTAAATAAAGATACATAATGTTTGTATGAAAAA

# FIGURE 338

MISLPGPLVTNLLRFLFLGLSALAPPSRAQLQLHLPANRLQAVEGGEVVLPAWYTLHGEVSSS
QPWEVPFVMWFFKQKEKEDQVLSYINGVTTSKPGVSLVYSMPSRNLSLRLEGLQEKDSGPYSC
SVNVQDKQGKSRGHSIKTLELNVLVPPAPPSCRLQGVPHVGANVTLSCQSPRSKPAVQYQWDR
QLPSFQTFFAPALDVIRGSLSLTNLSSSMAGVYVCKAHNEVGTAQCNVTLEVSTGPGAAVVAG
AVVGTLVGLGLLAGLVLLYHRRGKALEEPANDIKEDAIAPRTLPWPKSSDTISKNGTLSSVTS
ARALRPPHGPPRPGALTPTPSLSSQALPSPRLPTTDGAHPQPISPIPGGVSSSGLSRMGAVPV
MVPAQSQAGSLV

**Important features:**

**Signal peptide:**

amino acids 1-29

**Transmembrane domain:**

amino acids 245-267

**N-glycosylation site.**

amino acids 108-112, 169-173, 213-217, 236-240, 307-311

**N-myristoylation site.**

amino acids 90-96, 167-173, 220-226, 231-237, 252-258, 256-262, 262-268, 308-314, 363-369, 364-370

**Prokaryotic membrane lipoprotein lipid attachment site.**

amino acids 164-175

# FIGURE 339

GCGAGAACCTTTGCACGCGCACAAACTACGGGGACGATTTCTGATTGATTTTTGGCGCTTTCGATCCACCCTCCT
CCCTTCTC**ATG**GGACTTTGGGGACAAAGCGTCCCGACCGCCTCGAGCGCTCGAGCAGGGCGCTATCCAGGAGCCA
GGACAGCGTCGGGAACCAGACCATGGCTCCTGGACCCCAAGATCCTTAAGTTCGTCGTCTTCATCGTCGCGGTTC
TGCTGCCGGTCCGGGTTGACTCTGCCACCATCCCCCGGCAGGACGAAGTTCCCCAGCAGACAGTGGCCCCACAGC
AACAGAGGCGCAGCCTCAAGGAGGAGGAGTGTCCAGCAGGATCTCATAGATCAGAATATACTGGAGCCTGTAACC
CGTGCACAGAGGGTGTGGATTACACCATTGCTTCCAACAATTTGCCTTCTTGCCTGCTATGTACAGTTTGTAAAT
CAGGTCAAACAAATAAAAGTTCCTGTACCACGACCAGAGACACCGTGTGTCAGTGTGAAAAAGGAAGCTTCCAGG
ATAAAAACTCCCCTGAGATGTGCCGGACGTGTAGAACAGGGTGTCCCAGAGGGATGGTCAAGGTCAGTAATTGTA
CGCCCCGGAGTGACATCAAGTGCAAAAATGAATCAGCTGCCAGTTCCACTGGGAAAACCCCAGCAGCGGAGGAGA
CAGTGACCACCATCCTGGGGATGCTTGCCTCTCCCTATCACTACCTTATCATCATAGTGGTTTTAGTCATCATTT
TAGCTGTGGTTGTGGTTGGCTTTTCATGTCGGAAGAAATTCATTTCTTACCTCAAAGGCATCTGCTCAGGTGGTG
GAGGAGGTCCCGAACGTGTGCACAGAGTCCTTTTCCGGCGGCGTTCATGTCCTTCACGAGTTCCTGGGGCGGAGG
ACAATGCCCGCAACGAGACCCTGAGTAACAGATACTTGCAGCCCACCCAGGTCTCTGAGCAGGAAATCCAAGGTC
AGGAGCTGGCAGAGCTAACAGGTGTGACTGTAGAGTCGCCAGAGGAGCCACAGCGTCTGCTGGAACAGGCAGAAG
CTGAAGGGTGTCAGAGGAGGAGGCTGCTGGTTCCAGTGAATGACGCTGACTCCGCTGACATCAGCACCTTGCTGG
ATGCCTCGGCAACACTGGAAGAAGGACATGCAAAGGAAACAATTCAGGACCAACTGGTGGGCTCCGAAAAGCTCT
TTTATGAAGAAGATGAGGCAGGCTCTGCTACGTCCTGCCTG**TGA**AAGAATCTCTTCAGGAAACCAGAGCTTCCCT
CATTTACCTTTTCTCCTACAAAGGGAAGCAGCCTGGAAGAAACAGTCCAGTACTTGACCCATGCCCCAACAAACT
CTACTATCCAATATGGGGCAGCTTACCAATGGTCCTAGAACTTTGTTAACGCACTTGGAGTAATTTTTATGAAAT
ACTGCGTGTGATAAGCAAACGGGAGAAATTTATATCAGATTCTTGGCTGCATAGTTATACGATTGTGTATTAAGG
GTCGTTTTAGGCCACATGCGGTGGCTCATGCCTGTAATCCCAGCACTTTGATAGGCTGAGGCAGGTGGATTGCTT
GAGCTCGGGAGTTTGAGACCAGCCTCATCAACACAGTGAAACTCCATCTCAATTTAAAAAGAAAAAAAGTGGTTT
TAGGATGTCATTCTTTGCAGTTCTTCATCATGAGACAAGTCTTTTTTTCTGCTTCTTATATTGCAAGCTCCATCT
CTACTGGTGTGTGCATTTAATGACATCTAACTACAGATGCCGCACAGCCACAATGCTTTGCCTTATAGTTTTTA
ACTTTAGAACGGGATTATCTTGTTATTACCTGTATTTTCAGTTTCGGATATTTTTGACTTAATGATGAGATTATC
AAGACGTAGCCCTATGCTAAGTCATGAGCATATGGACTTACGAGGGTTCGACTTAGAGTTTTGAGCTTTAAGATA
GGATTATTGGGGCTTACCCCCACCTTAATTAGAGAAACATTTATATTGCTTACTACTGTAGGCTGTACATCTCTT
TTCCGATTTTTGTATAATGATGTAAACATGGAAAAACTTTAGGAAATGCACTTATTAGGCTGTTTACATGGGTTG
CCTGGATACAAATCAGCAGTCAAAAATGACTAAAAATATAACTAGTGACGGAGGGAGAAATCCTCCCTCTGTGGG
AGGCACTTACTGCATTCCAGTTCTCCCTCCTGCGCCCTGAGACTGGACCAGGGTTTGATGGCTGGCAGCTTCTCA
AGGGGCAGCTTGTCTTACTTGTTAATTTTAGAGGTATATAGCCATATTTATTTATAAATAAATATTTATTTATTT
ATTTATAAGTAGATGTTTACATATGCCCAGGATTTTGAAGAGCCTGGTATCTTTGGGAAGCCATGTGTCTGGTTT
GTCGTGCTGGGACAGTCATGGGACTGCATCTTCCGACTTGTCCACAGCAGATGAGGACAGTGAGAATTAAGTTAG
ATCCGAGACTGCGAAGAGCTTCTCTTTCAAGCGCCATTACAGTTGAACGTTAGTGAATCTTGAGCCTCATTTGGG
CTCAGGGCAGAGCAGGTGTTTATCTGCCCCGGCATCTGCCATGGCATCAAGAGGGAAGAGTGGACGGTGCTTGGG
AATGGTGTGAAATGGTTGCCGACTCAGGCATGGATGGGCCCCTCTCGCTTCTGGTGGTCTGTGAACTGAGTCCCT
GGGATGCCTTTTAGGGCAGAGATTCCTGAGCTGCGTTTTAGGGTACAGATTCCCTGTTTGAGGAGCTTGGCCCCT
CTGTAAGCATCTGACTCATCTCAGAGATATCAATTCTTAAACACTGTGACAACGGGATCTAAAATGGCTGACACA
TTTGTCCTTGTGTCACGTTCCATTATTTTATTTAAAAACCTCAGTAATCGTTTTAGCTTCTTTCCAGCAAACTCT
TCTCCACAGTAGCCCAGTCGTGGTAGGATAAATTACGGATATAGTCATTCTAGGGGTTTCAGTCTTTTCCATCTC
AAGGCATTGTGTGTTTTGTTCCGGGACTGGTTTGGCTGGGACAAAGTTAGAACTGCCTGAAGTTCGCACATTCAG
ATTGTTGTGTCCATGGAGTTTTAGGAGGGGATGGCCTTTCCGGTCTTCGCACTTCCATCCTCTCCCACTTCCATC
TGGCGTCCCACACCTTGTCCCCTGCACTTCTGGATGACACAGGGTGCTGCTGCCTCCTAGTCTTTGCCTTTGCTG
GGCCTTCTGTGCAGGAGACTTGGTCTCAAAGCTCAGAGAGAGCCAGTCCGGTCCCAGCTCCTTTGTCCCTTCCTC
AGAGGCCTTCCTTGAAGATGCATCTAGACTACCAGCCTTATCAGTGTTTAAGCTTATTCCTTTAACATAAGCTTC
CTGACAACATGAAATTGTTGGGGTTTTTTGGCGTTGGTTGATTTGTTTAGGTTTTGCTTTATACCCGGGCCAAAT
AGCACATAACACCTGGTTATATATGAAATACTCATATGTTTATGACCAAAATAAATATGAAACCTCATRTTAAAA
AAAAAAAAAAAAAAAAAAAAAAAAAA

# FIGURE 340

MGLWGQSVPTASSARAGRYPGARTASGTRPWLLDPKILKFVVFIVAVLLPVRVDSATIPRQDEVPQQTVAPQQQR
RSLKEEECPAGSHRSEYTGACNPCTEGVDYTIASNNLPSCLLCTVCKSGQTNKSSCTTTRDTVCQCEKGSFQDKN
SPEMCRTCRTGCPRGMVKVSNCTPRSDIKCKNESAASSTGKTPAAEETVTTILGMLASPYHYLIIIVVLVIILAV
VVVGFSCRKKFISYLKGICSGGGGGPERVHRVLFRRRSCPSRVPGAEDNARNETLSNRYLQPTQVSEQEIQGQEL
AELTGVTVESPEEPQRLLEQAEAEGCQRRRLLVPVNDADSADISTLLDASATLEEGHAKETIQDQLVGSEKLFYE
EDEAGSATSCL

**Important features:**

**Transmembrane domains:**

amino acids 35-52, 208-230

**N-glycosylation sites.**

amino acids 127-131, 182-186, 277-281

**Glycosaminoglycan attachment site.**

amino acids 245-249

**cAMP- and cGMP-dependent protein kinase phosphorylation site.**

amino acids 260-264

**N-myristoylation sites.**

amino acids 21-27, 86-92, 102-108, 161-167, 242-248, 270-276, 297-303, 380-386

**ATP/GTP-binding site motif A (P-loop).**

amino acids 185-193

**TNFR/NGFR cysteine-rich region.**

amino acids 99-139

# FIGURE 341

GCCTCTGAATTGTTGGGCAGTCTGGCAGTGGAGCTCTCCCCGGTCTGACAGCCACTCCAGAGG
CC**ATG**CTTCGTTTCTTGCCAGATTTGGCTTTCAGCTTCCTGTTAATTCTGGCTTTGGGCCAGG
CAGTCCAATTTCAAGAATATGTCTTTCTCCAATTTCTGGGCTTAGATAAGGCGCCTTCACCCC
AGAAGTTCCAACCTGTGCCTTATATCTTGAAGAAAATTTTCCAGGATCGCGAGGCAGCAGCGA
CCACTGGGGTCTCCCGAGACTTATGCTACGTAAAGGAGCTGGGCGTCCGCGGGAATGTACTTC
GCTTTCTCCCAGACCAAGGTTTCTTTCTTTACCCAAAGAAAATTTCCCAAGCTTCCTCCTGCC
TGCAGAAGCTCCTCTACTTTAACCTGTCTGCCATCAAAGAAAGGGAACAGTTGACATTGGCCC
AGCTGGGCCTGGACTTGGGGCCCAATTCTTACTATAACCTGGGACCAGAGCTGGAACTGGCTC
TGTTCCTGGTTCAGGAGCCTCATGTGTGGGGCCAGACCACCCCTAAGCCAGGTAAAATGTTTG
TGTTGCGGTCAGTCCCATGGCCACAAGGTGCTGTTCACTTCAACCTGCTGGATGTAGCTAAGG
ATTGGAATGACAACCCCCGGAAAAATTTCGGGTTATTCCTGGAGATACTGGTCAAAGAAGATA
GAGACTCAGGGGTGAATTTTCAGCCTGAAGACACCTGTGCCAGACTAAGATGCTCCCTTCATG
CTTCCCTGCTGGTGGTGACTCTCAACCCTGATCAGTGCCACCCTTCTCGGAAAAGGAGAGCAG
CCATCCCTGTCCCCAAGCTTTCTTGTAAGAACCTCTGCCACCGTCACCAGCTATTCATTAACT
TCCGGGACCTGGGTTGGCACAAGTGGATCATTGCCCCCAAGGGGTTCATGGCAAATTACTGCC
ATGGAGAGTGTCCCTTCTCACTGACCATCTCTCTCAACAGCTCCAATTATGCTTTCATGCAAG
CCCTGATGCATGCCGTTGACCCAGAGATCCCCCAGGCTGTGTGTATCCCCACCAAGCTGTCTC
CCATTTCCATGCTCTACCAGGACAATAATGACAATGTCATTCTACGACATTATGAAGACATGG
TAGTCGATGAATGTGGGTGTGGG**TAG**GATGTCAGAAATGGGAATAGAAGGAGTGTTCTTAGGG
TAAATCTTTTAATAAAACTACCTATCTGGTTTATGACCACTTAGATCGAAATGTC

# FIGURE 342

MLRFLPDLAFSFLLILALGQAVQFQEYVFLQFLGLDKAPSPQKFQPVPYILKKIFQDREAAAT
TGVSRDLCYVKELGVRGNVLRFLPDQGFFLYPKKISQASSCLQKLLYFNLSAIKEREQLTLAQ
LGLDLGPNSYYNLGPELELALFLVQEPHVWGQTTPKPGKMFVLRSVPWPQGAVHFNLLDVAKD
WNDNPRKNFGLFLEILVKEDRDSGVNFQPEDTCARLRCSLHASLLVVTLNPDQCHPSRKRRAA
IPVPKLSCKNLCHRHQLFINFRDLGWHKWIIAPKGFMANYCHGECPFSLTISLNSSNYAFMQA
LMHAVDPEIPQAVCIPTKLSPISMLYQDNNDNVILRHYEDMVVDECGCG

**Important features:**

**Signal peptide:**

amino acids 1-21

**N-glycosylation sites.**

amino acids 112-116, 306-310

**cAMP- and cGMP-dependent protein kinase phosphorylation site.**

amino acids 96-100

**N-myristoylation site.**

amino acids 77-83

**TGF-beta family proteins.**

amino acids 264-299, 327-341, 345-364

# FIGURE 343

CCCACGCGTCCGGCCTTCTCTCTGGACTTTGCATTTCCATTCCTTTTCATTGACAAACTGACTTTTTTTATTTCT
TTTTTTCCATCTCTGGGCCAGCTTGGGATCCTAGGCCGCCCTGGGAAGACATTTGTGTTTTACACACATAAGGAT
CTGTGTTTGGGGTTTCTTCTTCCTCCCCTGACATTGGCATTGCTTAGTGGTTGTGTGGGGAGGGAGACCACGTGG
GCTCAGTGCTTGCTTGCACTTATCTGCCTAGGTACATCGAAGTCTTTTGACCTCCATACAGTGATTATGCCTGTC
ATCGCTGGTGGTATCCTGGCGGCCTTGCTCCTGCTGATAGTTGTCGTGCTCTGTCTTTACTTCAAAATACACAAC
GCGCTAAAAGCTGCAAAGGAACCTGAAGCTGTGGCTGTAAAAAATCACAACCCAGACAAGGTGTGGTGGGCCAAG
AACAGCCAGGCCAAAACCATTGCCACGGAGTCTTGTCCTGCCCTGCAGTGCTGTGAAGGATATAGAATGTGTGCC
AGTTTTGATTCCCTGCCACCTTGCTGTTGCGACATAAATGAGGGCCTCTGAGTTAGGAAAGGCTCCCTTCTCAAA
GCAGAGCCCTGAAGACTTCAATGATGTCAATGAGGCCACCTGTTTGTGATGTGCAGGCACAGAAGAAAGGCACAG
CTCCCCATCAGTTTCATGGAAAATAACTCAGTGCCTGCTGGGAACCAGCTGCTGGAGATCCCTACAGAGAGCTTC
CACTGGGGGCAACCCTTCCAGGAAGGAGTTGGGGAGAGAGAACCCTCACTGTGGGGAATGCTGATAAACCAGTCA
CACAGCTGCTCTATTCTCACACAAATCTACCCCTTGCGTGGCTGGAACTGACGTTTCCCTGGAGGTGTCCAGAAA
GCTGATGTAACACAGAGCCTATAAAAGCTGTCGGTCCTTAAGGCTGCCCAGCGCCTTGCCAAA**ATG**GAGCTTGTA
AGAAGGCTCATGCCATTGACCCTCTTAATTCTCTCCTGTTTGGCGGAGCTGACAATGGCGGAGGCTGAAGGCAAT
GCAAGCTGCACAGTCAGTCTAGGGGGTGCCAATATGGCAGAGACCCACAAAGCCATGATCCTGCAACTCAATCCC
AGTGAGAACTGCACCTGGACAATAGAAAGACCAGAAAACAAAAGCATCAGAATTATCTTTTCCTATGTCCAGCTT
GATCCAGATGGAAGCTGTGAAAGTGAAAACATTAAAGTCTTTGACGGAACCTCCAGCAATGGGCCTCTGCTAGGG
CAAGTCTGCAGTAAAAACGACTATGTTCCTGTATTTGAATCATCATCCAGTACATTGACGTTTCAAATAGTTACT
GACTCAGCAAGAATTCAAAGAACTGTCTTTGTCTTCTACTACTTCTTCTCTCCTAACATCTCTATTCCAAACTGT
GGCGGTTACCTGGATACCTTGGAAGGATCCTTCACCAGCCCCAATTACCCAAAGCCGCATCCTGAGCTGGCTTAT
TGTGTGTGGCACATACAAGTGGAGAAAGATTACAAGATAAAACTAAACTTCAAAGAGATTTTCCTAGAAATAGAC
AAACAGTGCAAATTTGATTTTCTTGCCATCTATGATGGCCCCTCCACCAACTCTGGCCTGATTGGACAAGTCTGT
GGCCGTGTGACTCCCACCTTCGAATCGTCATCAAACTCTCTGACTGTCGTGTTGTCTACAGATTATGCCAATTCT
TACCGGGGATTTTCTGCTTCCTACACCTCAATTTATGCAGAAAACATCAACACTACATCTTTAACTTGCTCTTCT
GACAGGATGAGAGTTATTATAAGCAAATCCTACCTAGAGGCTTTTAACTCTAATGGGAATAACTTGCAACTAAAA
GACCCAACTTGCAGACCAAAATTATCAAATGTTGTGGAATTTTCTGTCCCTCTTAATGGATGTGGTACAATCAGA
AAGGTAGAAGATCAGTCAATTACTTACACCAATATAATCACCTTTTCTGCATCCTCAACTTCTGAAGTGATCACC
CGTCAGAAACAACTCCAGATTATTGTGAAGTGTGAAATGGGACATAATTCTACAGTGGAGATAATATACATAACA
GAAGATGATGTAATACAAAGTCAAAATGCACTGGGCAAATATAACACCAGCATGGCTCTTTTTGAATCCAATTCA
TTTGAAAAGACTATACTTGAATCACCATATTATGTGGATTTGAACCAAACTCTTTTTGTTCAAGTTAGTCTGCAC
ACCTCAGATCCAAATTTGGTGGTGTTTCTTGATACCTGTAGAGCCTCTCCCACCTCTGACTTTGCATCTCCAACC
TACGACCTAATCAAGAGTGGATGTAGTCGAGATGAAACTTGTAAGGTGTATCCCTTATTTGGACACTATGGGAGA
TTCCAGTTTAATGCCTTTAAATTCTTGAGAAGTATGAGCTCTGTGTATCTGCAGTGTAAAGTTTTGATATGTGAT
AGCAGTGACCACCAGTCTCGCTGCAATCAAGGTTGTGTCTCCAGAAGCAAACGAGACATTTCTTCATATAAATGG
AAAACAGATTCCATCATAGGACCCATTCGTCTGAAAAGGGATCGAAGTGCAAGTGGCAATTCAGGATTTCAGCAT
GAAACACATGCGGAAGAAACTCCAAACCAGCCTTTCAACAGTGTGCATCTGTTTTCCTTCATGGTTCTAGCTCTG
AATGTGGTGACTGTAGCGACAATCACAGTGAGGCATTTTGTAAATCAACGGGCAGACTACAAATACCAGAAGCTG
CAGAACTAT**TAA**CTAACAGGTCCAACCCTAAGTGAGACATGTTTCTCCAGGATGCCAAAGGAAATGCTACCTCGT
GGCTACACATATTATGAATAAATGAGGAAGGGCCTGAAAGTGACACACAGGCCTGCATGTAAAAAAA

# FIGURE 344

MELVRRLMPLTLLILSCLAELTMAEAEGNASCTVSLGGANMAETHKAMILQLNPSENCTWTIE
RPENKSIRIIFSYVQLDPDGSCESENIKVFDGTSSNGPLLGQVCSKNDYVPVFESSSSTLTFQ
IVTDSARIQRTVFVFYYFFSPNISIPNCGGYLDTLEGSFTSPNYPKPHPELAYCVWHIQVEKD
YKIKLNFKEIFLEIDKQCKFDFLAIYDGPSTNSGLIGQVCGRVTPTFESSSNSLTVVLSTDYA
NSYRGFSASYTSIYAENINTTSLTCSSDRMRVIISKSYLEAFNSNGNNLQLKDPTCRPKLSNV
VEFSVPLNGCGTIRKVEDQSITYTNIITFSASSTSEVITRQKQLQIIVKCEMGHNSTVEIIYI
TEDDVIQSQNALGKYNTSMALFESNSFEKTILESPYYVDLNQTLFVQVSLHTSDPNLVVFLDT
CRASPTSDFASPTYDLIKSGCSRDETCKVYPLFGHYGRFQFNAFKFLRSMSSVYLQCKVLICD
SSDHQSRCNQGCVSRSKRDISSYKWKTDSIIGPIRLKRDRSASGNSGFQHETHAEETPNQPFN
SVHLFSFMVLALNVVTVATITVRHFVNQRADYKYQKLQNY

**Important features:**
**Signal sequence:**
amino acids 1-24

**Transmembrane domain:**
amino acids 571-586

**N-glycosylation site.**
amino acids 29-33, 57-61, 67-71, 148-152, 271-275, 370-374, 394-398, 419-423

**Casein kinase II phosphorylation site.**
amino acids 22-26, 108-112, 289-293, 348-352, 371-375, 379-383, 408-412, 463-467, 520-524, 556-560

**Tyrosine kinase phosphorylation site.**
amino acids 172-180, 407-415, 407-416, 519-528

**N-myristoylation site.**
amino acids 28-34, 38-44, 83-89, 95-101, 104-110, 226-232

**Prokaryotic membrane lipoprotein lipid attachment site.**
amino acids 7-18

# FIGURE 345

TGGGGGCCCCCCAGGCTCGCGCGTGGAGCGAAGCAGC**ATG**GGCAGTCGGTGCGCGCTGGCCCTGGCGGTGCTCTC
GGCCTTGCTGTGTCAGGTCTGGAGCTCTGGGGTGTTCGAACTGAAGCTGCAGGAGTTCGTCAACAAGAAGGGGCT
GCTGGGGAACCGCAATTGCTGCCGCGGGGGCGCGGGGCCACCGCCGTGCGCCTGCCGGACCTTCTTCCGCGTGTG
CCTCAAGCACTACCAGGCCAGCGTGTCCCCCGAGCCGCCCTGCACCTACGGCAGCGCCGTCACCCCCGTGCTGGG
CGTCGACTCCTTCAGTCTGCCCGACGGCGGGGGCGCCGACTCCGCGTTCAGCAACCCCATCCGCTTCCCCTTCGG
CTTCACCTGGCCGGGCACCTTCTCTCTGATTATTGAAGCTCTCCACACAGATTCTCCTGATGACCTCGCAACAGA
AAACCCAGAAAGACTCATCAGCCGCCTGGCCACCCAGAGGCACCTGACGGTGGGCGAGGAGTGGTCCCAGGACCT
GCACAGCAGCGGCCGCACGGACCTCAAGTACTCCTACCGCTTCGTGTGTGACGAACACTACTACGGAGAGGGCTG
CTCCGTTTTCTGCCGTCCCCGGGACGATGCCTTCGGCCACTTCACCTGTGGGGAGCGTGGGGAGAAAGTGTGCAA
CCCTGGCTGGAAAGGGCCCTACTGCACAGAGCCGATCTGCCTGCCTGGATGTGATGAGCAGCATGGATTTTGTGA
CAAACCAGGGGAATGCAAGTGCAGAGTGGGCTGGCAGGGCCGGTACTGTGACGAGTGTATCCGCTATCCAGGCTG
TCTCCATGGCACCTGCCAGCAGCCCTGGCAGTGCAACTGCCAGGAAGGCTGGGGGGGCCTTTTCTGCAACCAGGA
CCTGAACTACTGCACACACCATAAGCCCTGCAAGAATGGAGCCACCTGCACCAACACGGGCCAGGGGAGCTACAC
TTGCTCTTGCCGGCCTGGGTACACAGGTGCCACCTGCGAGCTGGGGATTGACGAGTGTGACCCCAGCCCTTGTAA
GAACGGAGGGAGCTGCACGGATCTCGAGAACAGCTACTCCTGTACCTGCCCACCCGGCTTCTACGGCAAAATCTG
TGAATTGAGTGCCATGACCTGTGCGGACGGCCCTTGCTTTAACGGGGGTCGGTGCTCAGACAGCCCCGATGGAGG
GTACAGCTGCCGCTGCCCCGTGGGCTACTCCGGCTTCAACTGTGAGAAGAAAATTGACTACTGCAGCTCTTCACC
CTGTTCTAATGGTGCCAAGTGTGTGGACCTCGGTGATGCCTACCTGTGCCGCTGCCAGGCCGGCTTCTCGGGGAG
GCACTGTGACGACAACGTGGACGACTGCGCCTCCTCCCCGTGCGCCAACGGGGGCACCTGCCGGGATGGCGTGAA
CGACTTCTCCTGCACCTGCCCGCCTGGCTACACGGGCAGGAACTGCAGTGCCCCCGTCAGCAGGTGCGAGCACGC
ACCCTGCCACAATGGGGCCACCTGCCACGAGAGGGGCCACCGCTATGTGTGCGAGTGTGCCCGAGGCTACGGGGG
TCCCAACTGCCAGTTCCTGCTCCCCGAGCTGCCCCCGGGCCCAGCGGTGGTGGACCTCACTGAGAAGCTAGAGGG
CCAGGGCGGGCCATTCCCCTGGGTGGCCGTGTGCGCCGGGGTCATCCTTGTCCTCATGCTGCTGCTGGGCTGTGC
CGCTGTGGTGGTCTGCGTCCGGCTGAGGCTGCAGAAGCACCGGCCCCCAGCCGACCCCTGCCGGGGGGAGACGGA
GACCATGAACAACCTGGCCAACTGCCAGCGTGAGAAGGACATCTCAGTCAGCATCATCGGGGCCACGCAGATCAA
GAACACCAACAAGAAGGCGGACTTCCACGGGGACCACAGCGCCGACAAGAATGGCTTCAAGGCCCGCTACCCAGC
GGTGGACTATAACCTCGTGCAGGACCTCAAGGGTGACGACACCGCCGTCAGGGACGCGCACAGCAAGCGTGACAC
CAAGTGCCAGCCCCAGGGCTCCTCAGGGGAGGAGAAGGGGACCCCGACCACACTCAGGGGTGGAGAAGCATCTGA
AAGAAAAAGGCCGGACTCGGGCTGTTCAACTTCAAAAGACACCAAGTACCAGTCGGTGTACGTCATATCCGAGGA
GAAGGATGAGTGCGTCATAGCAACTGAGGTG**TAA**AATGGAAGTGAGATGGCAAGACTCCCGTTTCTCTTAAAATA
AGTAAAATTCCAAGGATATATGCCCCAACGAATGCTGCTGAAGAGGAGGGAGGCCTCGTGGACTGCTGCTGAGAA
ACCGAGTTCAGACCGAGCAGGTTCTCCTCCTGAGGTCCTCGACGCCTGCCGACAGCCTGTCGCGGCCCGGCCGCC
TGCGGCACTGCCTTCCGTGACGTCGCCGTTGCACTATGGACAGTTGCTCTTAAGAGAATATATATTTAAATGGGT
GAACTGAATTACGCATAAGAAGCATGCACTGCCTGAGTGTATATTTTGGATTCTTATGAGCCAGTCTTTTCTTGA
ATTAGAAACACAAACACTGCCTTTATTGTCCTTTTTGATACGAAGATGTGCTTTTTCTAGATGGAAAAGATGTGT
GTTATTTTTTGGATTTGTAAAAATATTTTTCATGATATCTGTAAAGCTTGAGTATTTTGTGATGTTCGTTTTTTA
TAATTTAAATTTTGGTAAATATGTACAAAGGCACTTCGGGTCTATGTGACTATATTTTTTTGTATATAAATGTAT
TTATGGAATATTGTGCAAATGTTATTTGAGTTTTTACTGTTTTGTTAATGAAGAAATTCCTTTTTAAAATATTT
TTCCAAAATAAATTTTATGAATGACAAAAAAAAAAAAAAAAAAAAAAAAAAAAAAAAAAAAAAAAAAAAA
AAAAAAAA

# FIGURE 346

MGSRCALALAVLSALLCQVWSSGVFELKLQEFVNKKGLLGNRNCCRGGAGPPPCACRTFFRVC
LKHYQASVSPEPPCTYGSAVTPVLGVDSFSLPDGGGADSAFSNPIRFPFGFTWPGTFSLIIEA
LHTDSPDDLATENPERLISRLATQRHLTVGEEWSQDLHSSGRTDLKYSYRFVCDEHYYGEGCS
VFCRPRDDAFGHFTCGERGEKVCNPGWKGPYCTEPICLPGCDEQHGFCDKPGECKCRVGWQGR
YCDECIRYPGCLHGTCQQPWQCNCQEGWGGLFCNQDLNYCTHHKPCKNGATCTNTGQGSYTCS
CRPGYTGATCELGIDECDPSPCKNGGSCTDLENSYSCTCPPGFYGKICELSAMTCADGPCFNG
GRCSDSPDGGYSCRCPVGYSGFNCEKKIDYCSSSPCSNGAKCVDLGDAYLCRCQAGFSGRHCD
DNVDDCASSPCANGGTCRDGVNDFSCTCPPGYTGRNCSAPVSRCEHAPCHNGATCHERGHRYV
CECARGYGGPNCQFLLPELPPGPAVVDLTEKLEGQGGPFPWVAVCAGVILVLMLLLGCAAVVV
CVRLRLQKHRPPADPCRGETETMNNLANCQREKDISVSIIGATQIKNTNKKADFHGDHSADKN
GFKARYPAVDYNLVQDLKGDDTAVRDAHSKRDTKCQPQGSSGEEKGTPTTLRGGEASERKRPD
SGCSTSKDTKYQSVYVISEEKDECVIATEV

**Important features:**
**Signal sequence:**
Amino acids 1-21

**Transmembrane domain:**
Amino acids 546-566

**N-glycosylation site:**
Amino acids 477-481

**cAMP- and cGMP-dependent protein kinase phosphorylation site:**
Amino acids 660-664

**Tyrosine kinase phosphorylation sites:**
Amino acids 176-185;252-261

**N-myristoylation sites:**
Amino acids 2-8;37-43;40-46;98-104;99-105;262-268;281-287;
282-288;301-307;310-316;328-334;340-344;378-384;387-393;512-518;
676-682;683-689;695-701

**Aspartic acid and asparagine hydroxylation sites:**
Amino acids 343-355;420-432;458-470

**Prokaryotic membrane lipoprotein lipid attachment site:**
Amino acids 552-563

**EGF-like domain cysteine pattern signature:**
Amino acids 243-255;274-286;314-326;352-364;391-403;429-441;
467-479;505-517

# FIGURE 347

CCCACGCGTCCGCACCTCGGCCCCGGGCTCCGAAGCGGCTCGGGGGCGCCCTTTCGGTCAACA
TCGTAGTCCACCCCCTCCCCATCCCCAGCCCCCGGGGATTCAGGCTCGCCAGCGCCCAGCCAG
GGAGCCGGCCGGGAAGCGCG**ATG**GGGGCCCCAGCCGCCTCGCTCCTGCTCCTGCTCCTGCTGT
TCGCCTGCTGCTGGGCGCCCGGCGGGGCCAACCTCTCCCAGGACGACAGCCAGCCCTGGACAT
CTGATGAAACAGTGGTGGCTGGTGGCACCGTGGTGCTCAAGTGCCAAGTGAAAGATCACGAGG
ACTCATCCCTGCAATGGTCTAACCCTGCTCAGCAGACTCTCTACTTTGGGGAGAAGAGAGCCC
TTCGAGATAATCGAATTCAGCTGGTTACCTCTACGCCCCACGAGCTCAGCATCAGCATCAGCA
ATGTGGCCCTGGCAGACGAGGGCGAGTACACCTGCTCAATCTTCACTATGCCTGTGCGAACTG
CCAAGTCCCTCGTCACTGTGCTAGGAATTCCACAGAAGCCCATCATCACTGGTTATAAATCTT
CATTACGGGAAAAAGACACAGCCACCCTAAACTGTCAGTCTTCTGGGAGCAAGCCTGCAGCCC
GGCTCACCTGGAGAAAGGGTGACCAAGAACTCCACGGAGAACCAACCCGCATACAGGAAGATC
CCAATGGTAAAACCTTCACTGTCAGCAGCTCGGTGACATTCCAGGTTACCCGGGAGGATGATG
GGGCGAGCATCGTGTGCTCTGTGAACCATGAATCTCTAAAGGGAGCTGACAGATCCACCTCTC
AACGCATTGAAGTTTTATACACACCAACTGCGATGATTAGGCCAGACCCTCCCCATCCTCGTG
AGGGCCAGAAGCTGTTGCTACACTGTGAGGGTCGCGGCAATCCAGTCCCCCAGCAGTACCTAT
GGGAGAAGGAGGGCAGTGTGCCACCCCTGAAGATGACCCAGGAGAGTGCCCTGATCTTCCCTT
TCCTCAACAAGAGTGACAGTGGCACCTACGGCTGCACAGCCACCAGCAACATGGGCAGCTACA
AGGCCTACTACACCCTCAATGTTAATGACCCCAGTCCGGTGCCCTCCTCCTCCAGCACCTACC
ACGCCATCATCGGTGGGATCGTGGCTTTCATTGTCTTCCTGCTGCTCATCATGCTCATCTTCC
TTGGCCACTACTTGATCCGGCACAAAGGAACCTACCTGACACATGAGGCAAAAGGCTCCGACG
ATGCTCCAGACGCGGACACGGCCATCATCAATGCAGAAGGCGGGCAGTCAGGAGGGGACGACA
AGAAGGAATATTTCATC**TAG**AGGCGCCTGCCCACTTCCTGCGCCCCCCAGGGGCCCTGTGGGG
ACTGCTGGGGCCGTCACCAACCCGGACTTGTACAGAGCAACCGCAGGGCCGCCCCTCCCGCTT
GCTCCCCAGCCCACCCACCCCCCTGTACAGAATGTCTGCTTTGGGTGCGGTTTTGTACTCGGT
TTGGAATGGGGAGGGAGGAGGGCGGGGGGAGGGGAGGGTTGCCCTCAGCCCTTTCCGTGGCTT
CTCTGCATTTGGGTTATTATTATTTTTGTAACAATCCCAAATCAAATCTGTCTCCAGGCTGGA
GAGGCAGGAGCCCTGGGGTGAGAAAAGCAAAAAACAAACAAAAAACA

# FIGURE 348

MGAPAASLLLLLLLFACCWAPGGANLSQDDSQPWTSDETVVAGGTVVLKCQVKDHEDSSLQWS
NPAQQTLYFGEKRALRDNRIQLVTSTPHELSISISNVALADEGEYTCSIFTMPVRTAKSLVTV
LGIPQKPIITGYKSSLREKDTATLNCQSSGSKPAARLTWRKGDQELHGEPTRIQEDPNGKTFT
VSSSVTFQVTREDDGASIVCSVNHESLKGADRSTSQRIEVLYTPTAMIRPDPPHPREGQKLLL
HCEGRGNPVPQQYLWEKEGSVPPLKMTQESALIFPFLNKSDSGTYGCTATSNMGSYKAYYTLN
VNDPSPVPSSSSTYHAIIGGIVAFIVFLLLIMLIFLGHYLIRHKGTYLTHEAKGSDDAPDADT
AIINAEGGQSGGDDKKEYFI

**Important features:**

**Signal sequence:**

amino acids 1-20

**Transmembrane domain:**

amino acids 331-352

**N-glycosylation site.**

amino acids 25-29, 290-294

**Casein kinase II phosphorylation site.**

amino acids 27-31, 35-39, 89-93, 141-145, 199-203, 388-392

**N-myristoylation site.**

amino acids 2-8, 23-29, 156-162, 218-224, 295-301, 298-304, 306-310, 334-340, 360-364, 385-389, 386-390

**Prokaryotic membrane lipoprotein lipid attachment site.**

amino acids 7-18

# FIGURE 349

ACTTGCCATCACCTGTTGCCAGTGTGGAAAAATTCTCCCTGTTGAATTTTTTGCACATGGAGGACAGCAGCAAAG
AGGGCAACACAGGCTGATAAGACCAGAGACAGCAGGGAGATTATTTACCATACGCCCTCAGGACGTTCCCTCTA
GCTGGAGTTCTGGACTTCAACAGAACCCCATCCAGTCATTTTGATTTTGCTGTTTATTTTTTTTTCTTTTTCTT
TTTCCCACCACATTGTATTTTATTTCCGTACTTCAGAA**ATG**GGCCTACAGACCACAAAGTGGCCCAGCCATGGGG
CTTTTTTCCTGAAGTCTTGGCTTATCATTTCCCTGGGGCTCTACTCACAGGTGTCCAAACTCCTGGCCTGCCCTA
GTGTGTGCCGCTGCGACAGGAACTTTGTCTACTGTAATGAGCGAAGCTTGACCTCAGTGCCTCTTGGGATCCCGG
AGGGCGTAACCGTACTCTACCTCCACAACAACCAAATTAATAATGCTGGATTTCCTGCAGAACTGCACAATGTAC
AGTCGGTGCACACGGTCTACCTGTATGGCAACCAACTGGACGAATTCCCCATGAACCTTCCCAAGAATGTCAGAG
TTCTCCATTTGCAGGAAAACAATATTCAGACCATTTCACGGGCTGCTCTTGCCCAGCTCTTGAAGCTTGAAGAGC
TGCACCTGGATGACAACTCCATATCCACAGTGGGGGTGGAAGACGGGGCCTTCCGGGAGGCTATTAGCCTCAAAT
TGTTGTTTTTGTCTAAGAATCACCTGAGCAGTGTGCCTGTTGGGCTTCCTGTGGACTTGCAAGAGCTGAGAGTGG
ATGAAAATCGAATTGCTGTCATATCCGACATGGCCTTCCAGAATCTCACGAGCTTGGAGCGTCTTATTGTGGACG
GGAACCTCCTGACCAACAAGGGTATCGCCGAGGGCACCTTCAGCCATCTCACCAAGCTCAAGGAATTTTCAATTG
TACGTAATTCGCTGTCCCACCCTCCTCCCGATCTCCCAGGTACGCATCTGATCAGGCTCTATTTGCAGGACAACC
AGATAAACCACATTCCTTTGACAGCCTTCTCAAATCTGCGTAAGCTGGAACGGCTGGATATATCCAACAACCAAC
TGCGGATGCTGACTCAAGGGGTTTTTGATAATCTCTCCAACCTGAAGCAGCTCACTGCTCGGAATAACCCTTGGT
TTTGTGACTGCAGTATTAAATGGGTCACAGAATGGCTCAAATATATCCCTTCATCTCTCAACGTGCGGGGTTTCA
TGTGCCAAGGTCCTGAACAAGTCCGGGGGATGGCCGTCAGGGAATTAAATATGAATCTTTTGTCCTGTCCCACCA
CGACCCCCGGCCTGCCTCTCTTCACCCCAGCCCCAAGTACAGCTTCTCCGACCACTCAGCCTCCCACCCTCTCTA
TTCCAAACCCTAGCAGAAGCTACACGCCTCCAACTCCTACCACATCGAAACTTCCCACGATTCCTGACTGGGATG
GCAGAGAAAGAGTGACCCCACCTATTTCTGAACGGATCCAGCTCTCTATCCATTTTGTGAATGATACTTCCATTC
AAGTCAGCTGGCTCTCTCTCTTCACCGTGATGGCATACAAACTCACATGGGTGAAAATGGGCCACAGTTTAGTAG
GGGGCATCGTTCAGGAGCGCATAGTCAGCGGTGAGAAGCAACACCTGAGCCTGGTTAACTTAGAGCCCCGATCCA
CCTATCGGATTTGTTTAGTGCCACTGGATGCTTTTAACTACCGCGCGGTAGAAGACACCATTTGTTCAGAGGCCA
CCACCCATGCCTCCTATCTGAACAACGGCAGCAACACAGCGTCCAGCCATGAGCAGACGACGTCCCACAGCATGG
GCTCCCCCTTTCTGCTGGCGGGCTTGATCGGGGGCGCGGTGATATTTGTGCTGGTGGTCTTGCTCAGCGTCTTTT
GCTGGCATATGCACAAAAAGGGGCGCTACACCTCCCAGAAGTGGAAATACAACCGGGGCCGGCGGAAAGATGATT
ATTGCGAGGCAGGCACCAAGAAGGACAACTCCATCCTGGAGATGACAGAAACCAGTTTTCAGATCGTCTCCTTAA
ATAACGATCAACTCCTTAAAGGAGATTTCAGACTGCAGCCCATTTACACCCCAAATGGGGGCATTAATTACACAG
ACTGCCATATCCCCAACAACATGCGATACTGCAACAGCAGCGTGCCAGACCTGGAGCACTGCCATACG**TGA**CAGC
CAGAGGCCCAGCGTTATCAAGGCGGACAATTAGACTCTTGAGAACACACTCGTGTGTGCACATAAAGACACGCAG
ATTACATTTGATAAATGTTACACAGATGCATTTGTGCATTTGAATACTCTGTAATTTATACGGTGTACTATATAA
TGGGATTTAAAAAAAGTGCTATCTTTTCTATTTCAAGTTAATTACAAACAGTTTTGTAACTCTTTGCTTTTTAAA
TCTT

# FIGURE 350

MGLQTTKWPSHGAFFLKSWLIISLGLYSQVSKLLACPSVCRCDRNFVYCNERSLTSVPLGIPE
GVTVLYLHNNQINNAGFPAELHNVQSVHTVYLYGNQLDEFPMNLPKNVRVLHLQENNIQTISR
AALAQLLKLEELHLDDNSISTVGVEDGAFREAISLKLLFLSKNHLSSVPVGLPVDLQELRVDE
NRIAVISDMAFQNLTSLERLIVDGNLLTNKGIAEGTFSHLTKLKEFSIVRNSLSHPPPDLPGT
HLIRLYLQDNQINHIPLTAFSNLRKLERLDISNNQLRMLTQGVFDNLSNLKQLTARNNPWFCD
CSIKWVTEWLKYIPSSLNVRGFMCQGPEQVRGMAVRELNMNLLSCPTTTPGLPLFTPAPSTAS
PTTQPPTLSIPNPSRSYTPPTPTTSKLPTIPDWDGRERVTPPISERIQLSIHFVNDTSIQVSW
LSLFTVMAYKLTWVKMGHSLVGGIVQERIVSGEKQHLSLVNLEPRSTYRICLVPLDAFNYRAV
EDTICSEATTHASYLNNGSNTASSHEQTTSHSMGSPFLLAGLIGGAVIFVLVVLLSVFCWHMH
KKGRYTSQKWKYNRGRRKDDYCEAGTKKDNSILEMTETSFQIVSLNNDQLLKGDFRLQPIYTP
NGGINYTDCHIPNNMRYCNSSVPDLEHCHT

**Important features:**

**Signal peptide:**

amino acids 1-42

**Transmembrane domain:**

amino acids 542-561

**N-glycosylation site.**

amino acids 202-206, 298-302, 433-437, 521-525, 635-639, 649-653

**Casein kinase II phosphorylation site.**

amino acids 204-208, 407-411, 527-531, 593-597, 598-602, 651-655

**Tyrosine kinase phosphorylation site.**

amino acids 319-328

**N-myristoylation site.**

amino acids 2-8, 60-66, 149-155, 213-219, 220-226, 294-300, 522-528, 545-551, 633-639

**Amidation site.**

amino acids 581-585

**Leucine zipper pattern.**

amino acids 164-186

**Phospholipase A2 aspartic acid active site.**

amino acids 39-50

# FIGURE 351

AGCCGACGCTGCTCAAGCTGCAACTCTGTTGCAGTTGGCAGTTCTTTTCGGTTTCCCTCCTGCTGTTTGGGGGCA
TGAAAGGGCTTCGCCGCCGGGAGTAAAAGAAGGAATTGACCGGGCAGCGCGAGGGAGGAGCGCGCACGCGACCGC
GAGGGCGGGCGTGCACCCTCGGCTGGAAGTTTGTGCCGGGCCCCGAGCGCGCCGGCTGGGAGCTTCGGGTAGA
GACCTAGGCCGCTGGACCGCG**ATG**AGCGCGCCGAGCCTCCGTGCGCGCGCCGCGGGGTTGGGGCTGCTGCTGTGC
GCGGTGCTGGGGCGCGCTGGCCGGTCCGACAGCGGCGGTCGCGGGGAACTCGGGCAGCCCTCTGGGGTAGCCGCC
GAGCGCCCATGCCCCACTACCTGCCGCTGCCTCGGGGACCTGCTGGACTGCAGTCGTAAGCGGCTAGCGCGTCTT
CCCGAGCCACTCCCGTCCTGGGTCGCTCGGCTGGACTTAAGTCACAACAGATTATCTTTCATCAAGGCAAGTTCC
ATGAGCCACCTTCAAAGCCTTCGAGAAGTGAAACTGAACAACAATGAATTGGAGACCATTCCAAATCTGGGACCA
GTCTCGGCAAATATTACACTTCTCTCCTTGGCTGGAAACAGGATTGTTGAAATACTCCCTGAACATCTGAAAGAG
TTTCAGTCCCTTGAAACTTTGGACCTTAGCAGCAACAATATTTCAGAGCTCCAAACTGCATTTCCAGCCCTACAG
CTCAAATATCTGTATCTCAACAGCAACCGAGTCACATCAATGGAACCTGGGTATTTTGACAATTTGGCCAACACA
CTCCTTGTGTTAAAGCTGAACAGGAACCGAATCTCAGCTATCCCACCCAAGATGTTTAAACTGCCCCAACTGCAA
CATCTCGAATTGAACCGAAACAAGATTAAAAATGTAGATGGACTGACATTCCAAGGCCTTGGTGCTCTGAAGTCT
CTGAAAATGCAAAGAAATGGAGTAACGAAACTTATGGATGGAGCTTTTTGGGGGCTGAGCAACATGGAAATTTTG
CAGCTGGACCATAACAACCTAACAGAGATTACCAAAGGCTGGCTTTACGGCTTGCTGATGCTGCAGGAACTTCAT
CTCAGCCAAAATGCCATCAACAGGATCAGCCCTGATGCCTGGGAGTTCTGCCAGAAGCTCAGTGAGCTGGACCTA
ACTTTCAATCACTTATCAAGGTTAGATGATTCAAGCTTCCTTGGCCTAAGCTTACTAAATACACTGCACATTGGG
AACAACAGAGTCAGCTACATTGCTGATTGTGCCTTCCGGGGGCTTTCCAGTTTAAAGACTTTGGATCTGAAGAAC
AATGAAATTTCCTGGACTATTGAAGACATGAATGGTGCTTTCTCTGGGCTTGACAAACTGAGGCGACTGATACTC
CAAGGAAATCGGATCCGTTCTATTACTAAAAAAGCCTTCACTGGTTTGGATGCATTGGAGCATCTAGACCTGAGT
GACAACGCAATCATGTCTTTACAAGGCAATGCATTTTCACAAATGAAGAAACTGCAACAATTGCATTTAAATACA
TCAAGCCTTTTGTGCGATTGCCAGCTAAAATGGCTCCCACAGTGGGTGGCGGAAAACAACTTTCAGAGCTTTGTA
AATGCCAGTTGTGCCCATCCTCAGCTGCTAAAAGGAAGAAGCATTTTTGCTGTTAGCCCAGATGGCTTTGTGTGT
GATGATTTTCCCAAACCCCAGATCACGGTTCAGCCAGAAACACAGTCGGCAATAAAAGGTTCCAATTTGAGTTTC
ATCTGCTCAGCTGCCAGCAGCAGTGATTCCCCAATGACTTTTGCTTGGAAAAAAGACAATGAACTACTGCATGAT
GCTGAAATGGAAAATTATGCACACCTCCGGGCCCAAGGTGGCGAGGTGATGGAGTATACCACCATCCTTCGGCTG
CGCGAGGTGGAATTTGCCAGTGAGGGGAAATATCAGTGTGTCATCTCCAATCACTTTGGTTCATCCTACTCTGTC
AAAGCCAAGCTTACAGTAAATATGCTTCCCTCATTCACCAAGACCCCCATGGATCTCACCATCCGAGCTGGGGCC
ATGGCACGCTTGGAGTGTGCTGCTGTGGGGCACCCAGCCCCCCAGATAGCCTGGCAGAAGGATGGGGGCACAGAC
TTCCCAGCTGCACGGGAGAGACGCATGCATGTGATGCCCGAGGATGACGTGTTCTTTATCGTGGATGTGAAGATA
GAGGACATTGGGGTATACAGCTGCACAGCTCAGAACAGTGCAGGAAGTATTTCAGCAAATGCAACTCTGACTGTC
CTAGAAACACCATCATTTTTGCGGCCACTGTTGGACCGAACTGTAACCAAGGGAGAAACAGCCGTCCTACAGTGC
ATTGCTGGAGGAAGCCCTCCCCCTAAACTGAACTGGACCAAAGATGATAGCCCATTGGTGGTAACCGAGAGGCAC
TTTTTTGCAGCAGGCAATCAGCTTCTGATTATTGTGGACTCAGATGTCAGTGATGCTGGGAAATACACATGTGAG
ATGTCTAACACCCTTGGCACTGAGAGAGGAAACGTGCGCCTCAGTGTGATCCCCACTCCAACCTGCGACTCCCCT
CAGATGACAGCCCCATCGTTAGACGATGACGGATGGGCCACTGTGGGTGTCGTGATCATAGCCGTGGTTTGCTGT
GTGGTGGGCACGTCACTCGTGTGGGTGGTCATCATATACCACACAAGGCGGAGGAATGAAGATTGCAGCATTACC
AACACAGATGAGACCAACTTGCCAGCAGATATTCCTAGTTATTTGTCATCTCAGGGAACGTTAGCTGACAGGCAG
GATGGGTACGTGTCTTCAGAAAGTGGAAGCCACCACCAGTTTGTCACATCTTCAGGTGCTGGATTTTTCTTACCA
CAACATGACAGTAGTGGGACCTGCCATATTGACAATAGCAGTGAAGCTGATGTGGAAGCTGCCACAGATCTGTTC
CTTTGTCCGTTTTTGGGATCCACAGGCCCTATGTATTTGAAGGGAAATGTGTATGGCTCAGATCCTTTTGAAACA
TATCATACAGGTTGCAGTCCTGACCCAAGAACAGTTTTAATGGACCACTATGAGCCCAGTTACATAAAGAAAAAG
GAGTGCTACCCATGTTCTCATCCTTCAGAAGAATCCTGCGAACGGAGCTTCAGTAATATATCGTGGCCTTCACAT
GTGAGGAAGCTACTTAACACTAGTTACTCTCACAATGAAGGACCTGGAATGAAAAATCTGTGTCTAAACAAGTCC
TCTTTAGATTTTAGTGCAAATCCAGAGCCAGCGTCGGTTGCCTCGAGTAATTCTTTCATGGGTACCTTTGGAAAA
GCTCTCAGGAGACCTCACCTAGATGCCTATTCAAGCTTTGGACAGCCATCAGATTGTCAGCCAAGAGCCTTTTAT
TTGAAAGCTCATTCTTCCCCAGACTTGGACTCTGGGTCAGAGGAAGATGGGAAAGAAAGGACAGATTTTCAGGAA
GAAAATCACATTTGTACCTTTAAACAGACTTTAGAAAACTACAGGACTCCAAATTTTCAGTCTTATGACTTGGAC
ACA**TAG**ACTGAATGAGACCAAAGGAAAAGCTTAACATACTACCTCAAGTGAACTTTTATTTAAAAGAGAGAGAAT
CTTATGTTTTTTAAATGGAGTTATGAATTTTAAAAGGATAAAAATGCTTTATTTATACAGATGAACCAAAATTAC
AAAAAGTTATGAAAATTTTTATACTGGGAATGATGCTCATATAAGAATACCTTTTTAAACTATTTTTTAACTTTG
TTTTATGCAAAAAAGTATCTTACGTAAATTAATGATATAAATCATGATTATTTTATGTATTTTTATAATGCCAGA
TTTCTTTTTATGGAAAATGAGTTACTAAAGCATTTTAAATAATACCTGCCTTGTACCATTTTTTAAATAGAAGTT
ACTTCATTATATTTGCACATTATATTTAATAAAATGTGTCAATTTGAAAAAAAAAAAAAAAAAAAAAAAA

# FIGURE 352

MSAPSLRARAAGLGLLLCAVLGRAGRSDSGGRGELGQPSGVAAERPCPTTCRCLGDLLDCSRKRLARLPEPLPSW
VARLDLSHNRLSFIKASSMSHLQSLREVKLNNNELETIPNLGPVSANITLLSLAGNRIVEILPEHLKEFQSLETL
DLSSNNISELQTAFPALQLKYLYLNSNRVTSMEPGYFDNLANTLLVLKLNRNRISAIPPKMFKLPQLQHLELNRN
KIKNVDGLTFQGLGALKSLKMQRNGVTKLMDGAFWGLSNMEILQLDHNNLTEITKGWLYGLLMLQELHLSQNAIN
RISPDAWEFCQKLSELDLTFNHLSRLDDSSFLGLSLLNTLHIGNNRVSYIADCAFRGLSSLKTLDLKNNEISWTI
EDMNGAFSGLDKLRRLILQGNRIRSITKKAFTGLDALEHLDLSDNAIMSLQGNAFSQMKKLQQLHLNTSSLLCDC
QLKWLPQWVAENNFQSFVNASCAHPQLLKGRSIFAVSPDGFVCDDFPKPQITVQPETQSAIKGSNLSFICSAASS
SDSPMTFAWKKDNELLHDAEMENYAHLRAQGGEVMEYTTILRLREVEFASEGKYQCVISNHFGSSYSVKAKLTVN
MLPSFTKTPMDLTIRAGAMARLECAAVGHPAPQIAWQKDGGTDFPAARERRMHVMPEDDVFFIVDVKIEDIGVYS
CTAQNSAGSISANATLTVLETPSFLRPLLDRTVTKGETAVLQCIAGGSPPPKLNWTKDDSPLVVTERHFFAAGNQ
LLIIVDSDVSDAGKYTCEMSNTLGTERGNVRLSVIPTPTCDSPQMTAPSLDDDGWATVGVVIIAVVCCVVGTSLV
WVVIIYHTRRRNEDCSITNTDETNLPADIPSYLSSQGTLADRQDGYVSSESGSHHQFVTSSGAGFFLPQHDSSGT
CHIDNSSEADVEAATDLFLCPFLGSTGPMYLKGNVYGSDPFETYHTGCSPDPRTVLMDHYEPSYIKKKECYPCSH
PSEESCERSFSNISWPSHVRKLLNTSYSHNEGPGMKNLCLNKSSLDFSANPEPASVASSNSFMGTFGKALRRPHL
DAYSSFGQPSDCQPRAFYLKAHSSPDLDSGSEEDGKERTDFQEENHICTFKQTLENYRTPNFQSYDLDT

**Important features:**
**Signal sequence:**
amino acids 1-27

**Transmembrane domain:**
amino acids 808-828

**N-glycosylation site.**
amino acids 122-126, 156-160, 274-278, 442-446, 469-473, 515-519, 688-692, 729-733, 905-909, 987-991, 999-1003, 1016-1020

**Glycosaminoglycan attachment site.**
amino acids 886-890

**Casein kinase II phosphorylation site.**
amino acids 99-103, 180-184, 263-267, 314-318, 324-328, 374-378, 383-387, 407-411, 524-528, 608-612, 692-696, 709-713, 731-735, 799-803, 843-847, 863-867, 907-911, 1003-1007, 1018-1022, 1073-1077, 1079-1083, 1081-1085

**Tyrosine kinase phosphorylation site.**
amino acids 667-675

**N-myristoylation site.**
amino acids 14-20, 36-42, 239-245, 257-263, 380-386, 427-433, 513-519, 588-594, 672-678, 683-687, 774-780, 933-939

**Leucine zipper pattern.**
amino acids 58-80, 65-87

# FIGURE 353

GGGGGTTAGGGAGGAAGGAATCCACCCCCACCCCCCCAAACCCTTTTCTTCTCCTTTCCTGGCTTCGGACATTGG
AGCACTAAATGAACTTGAATTGTGTCTGTGGCGAGCAGGATGGTCGCTGTTACTTTGTGATGAGATCGGGGATGA
ATTGCTCGCTTTAAAA**ATG**CTGCTTTGGATTCTGTTGCTGGAGACGTCTCTTTGTTTTGCCGCTGGAAACGTTAC
AGGGGACGTTTGCAAAGAGAAGATCTGTTCCTGCAATGAGATAGAAGGGGACCTACACGTAGACTGTGAAAAAAA
GGGCTTCACAAGTCTGCAGCGTTTCACTGCCCCGACTTCCCAGTTTTACCATTTATTTCTGCATGGCAATTCCCT
CACTCGACTTTTCCCTAATGAGTTCGCTAACTTTTATAATGCGGTTAGTTTGCACATGGAAAACAATGGCTTGCA
TGAAATCGTTCCGGGGGCTTTTCTGGGGCTGCAGCTGGTGAAAAGGCTGCACATCAACAACAACAAGATCAAGTC
TTTTCGAAAGCAGACTTTTCTGGGGCTGGACGATCTGGAATATCTCCAGGCTGATTTTAATTTATTACGAGATAT
AGACCCGGGGGCCTTCCAGGACTTGAACAAGCTGGAGGTGCTCATTTTAAATGACAATCTCATCAGCACCCTACC
TGCCAACGTGTTCCAGTATGTGCCCATCACCCACCTCGACCTCCGGGGTAACAGGCTGAAAACGCTGCCCTATGA
GGAGGTCTTGGAGCAAATCCCTGGTATTGCGGAGATCCTGCTAGAGGATAACCCTTGGGACTGCACCTGTGATCT
GCTCTCCCTGAAAGAATGGCTGGAAAACATTCCCAAGAATGCCCTGATCGGCCGAGTGGTCTGCGAAGCCCCCAC
CAGACTGCAGGGTAAAGACCTCAATGAAACCACCGAACAGGACTTGTGTCCTTTGAAAAACCGAGTGGATTCTAG
TCTCCCGGCGCCCCCTGCCCAAGAAGAGACCTTTGCTCCTGGACCCCTGCCAACTCCTTTCAAGACAAATGGGCA
AGAGGATCATGCCACACCAGGGTCTGCTCCAAACGGAGGTACAAAGATCCCAGGCAACTGGCAGATCAAAATCAG
ACCCACAGCAGCGATAGCGACGGGTAGCTCCAGGAACAAACCCTTAGCTAACAGTTTACCCTGCCCTGGGGGCTG
CAGCTGCGACCACATCCCAGGGTCGGGTTTAAAGATGAACTGCAACAACAGGAACGTGAGCAGCTTGGCTGATTT
GAAGCCCAAGCTCTCTAACGTGCAGGAGCTTTTCCTACGAGATAACAAGATCCACAGCATCCGAAAATCGCACTT
TGTGGATTACAAGAACCTCATTCTGTTGGATCTGGGCAACAATAACATCGCTACTGTAGAGAACAACACTTTCAA
GAACCTTTTGGACCTCAGGTGGCTATACATGGATAGCAATTACCTGGACACGCTGTCCCGGGAGAAATTCGCGGG
GCTGCAAAACCTAGAGTACCTGAACGTGGAGTACAACGCTATCCAGCTCATCCTCCCGGGCACTTTCAATGCCAT
GCCCAAACTGAGGATCCTCATTCTCAACAACAACCTGCTGAGGTCCCTGCCTGTGGACGTGTTCGCTGGGGTCTC
GCTCTCTAAACTCAGCCTGCACAACAATTACTTCATGTACCTCCCGGTGGCAGGGGTGCTGGACCAGTTAACCTC
CATCATCCAGATAGACCTCCACGGAAACCCCTGGGAGTGCTCCTGCACAATTGTGCCTTTCAAGCAGTGGGCAGA
ACGCTTGGGTTCCGAAGTGCTGATGAGCGACCTCAAGTGTGAGACGCCGGTGAACTTCTTTAGAAAGGATTTCAT
GCTCCTCTCCAATGACGAGATCTGCCCTCAGCTGTACGCTAGGATCTCGCCCACGTTAACTTCGCACAGTAAAAA
CAGCACTGGGTTGGCGGAGACCGGGACGCACTCCAACTCCTACCTAGACACCAGCAGGGTGTCCATCTCGGTGTT
GGTCCCGGGACTGCTGCTGGTGTTTGTCACCTCCGCCTTCACCGTGGTGGGCATGCTCGTGTTTATCCTGAGGAA
CCGAAAGCGGTCCAAGAGACGAGATGCCAACTCCTCCGCGTCCGAGATTAATTCCCTACAGACAGTCTGTGACTC
TTCCTACTGGCACAATGGGCCTTACAACGCAGATGGGGCCCACAGAGTGTATGACTGTGGCTCTCACTCGCTCTC
AGAC**TAA**GACCCCAACCCCAATAGGGGAGGGCAGAGGGAAGGCGATACATCCTTCCCCACCGCAGGCACCCCGGG
GGCTGGAGGGGCGTGTACCCAAATCCCCGCGCCATCAGCCTGGATGGGCATAAGTAGATAAATAACTGTGAGCTC
GCACAACCGAAAGGGCCTGACCCCTTACTTAGCTCCCTCCTTGAAACAAAGAGCAGACTGTGGAGAGCTGGGAGA
GCGCAGCCAGCTCGCTCTTTGCTGAGAGCCCCTTTTGACAGAAAGCCCAGCACGACCCTGCTGGAAGAACTGACA
GTGCCCTCGCCCTCGGCCCCGGGGCCTGTGGGGTTGGATGCCGCGGTTCTATACATATATACATATATCCACATC
TATATAGAGAGATAGATATCTATTTTTCCCCTGTGGATTAGCCCCGTGATGGCTCCCTGTTGGCTACGCAGGGAT
GGGCAGTTGCACGAAGGCATGAATGTATTGTAAATAAGTAACTTTGACTTCTGAC

# FIGURE 354

MLLWILLLETSLCFAAGNVTGDVCKEKICSCNEIEGDLHVDCEKKGFTSLQRFTAPTSQFYHL
FLHGNSLTRLFPNEFANFYNAVSLHMENNGLHEIVPGAFLGLQLVKRLHINNNKIKSFRKQTF
LGLDDLEYLQADFNLLRDIDPGAFQDLNKLEVLILNDNLISTLPANVFQYVPITHLDLRGNRL
KTLPYEEVLEQIPGIAEILLEDNPWDCTCDLLSLKEWLENIPKNALIGRVVCEAPTRLQGKDL
NETTEQDLCPLKNRVDSSLPAPPAQEETFAPGPLPTPFKTNGQEDHATPGSAPNGGTKIPGNW
QIKIRPTAAIATGSSRNKPLANSLPCPGGCSCDHIPGSGLKMNCNNRNVSSLADLKPKLSNVQ
ELFLRDNKIHSIRKSHFVDYKNLILLDLGNNNIATVENNTFKNLLDLRWLYMDSNYLDTLSRE
KFAGLQNLEYLNVEYNAIQLILPGTFNAMPKLRILILNNNLLRSLPVDVFAGVSLSKLSLHNN
YFMYLPVAGVLDQLTSIIQIDLHGNPWECSCTIVPFKQWAERLGSEVLMSDLKCETPVNFFRK
DFMLLSNDEICPQLYARISPTLTSHSKNSTGLAETGTHSNSYLDTSRVSISVLVPGLLLVFVT
SAFTVVGMLVFILRNRKRSKRRDANSSASEINSLQTVCDSSYWHNGPYNADGAHRVYDCGSHS
LSD

**Important features:**

**Signal sequence:**
amino acids 1-15

**Transmembrane domain:**
amino acids 618-638

**N-glycosylation site.**
amino acids 18-22, 253-257, 363-367, 416-420, 595-599, 655-659

**cAMP- and cGMP-dependent protein kinase phosphorylation site.**
amino acids 122-126, 646-650

**Casein kinase II phosphorylation site.**
amino acids 30-34, 180-184, 222-226, 256-260, 366-370, 573-577, 608-612, 657-661, 666-670, 693-697

**N-myristoylation site.**
amino acids 17-23, 67-73, 100-106, 302-308, 328-334, 343-349, 354-360, 465-471, 493-499, 598-604, 603-609

**Prokaryotic membrane lipoprotein lipid attachment site.**
amino acids 337-348

# FIGURE 355

AGTCGACTGCGTCCCCTGTACCCGGCGCCAGCTGTGTTCCTGACCCCAGAATAACTCAGGGCTGCACCGGGCCTG
GCAGCGCTCCGCACACATTTCCTGTCGCGGCCTAAGGGAAACTGTTGGCCGCTGGGCCCGCGGGGGATTCTTGG
CAGTTGGGGGGTCCGTCGGGAGCGAGGGCGGAGGGGAAGGGAGGGGGAACCGGGTTGGGGAAGCCAGCTGTAGAG
GGCGGTGACCGCGCTCCAGACACAGCTCTGCGTCCTCGAGCGGGACAGATCCAAGTTGGGAGCAGCTCTGCGTGC
GGGGCCTCAGAGA**ATG**AGGCCGGCGTTCGCCCTGTGCCTCCTCTGGCAGGCGCTCTGGCCCGGGCCGGGCGGCGG
CGAACACCCCACTGCCGACCGTGCTGGCTGCTCGGCCTCGGGGGCCTGCTACAGCCTGCACCACGCTACCATGAA
GCGGCAGGCGGCCGAGGAGGCCTGCATCCTGCGAGGTGGGGCGCTCAGCACCGTGCGTGCGGGCGCCGAGCTGCG
CGCTGTGCTCGCGCTCCTGCGGGCAGGCCCAGGGCCCGGAGGGGGCTCCAAAGACCTGCTGTTCTGGGTCGCACT
GGAGCGCAGGCGTTCCCACTGCACCCTGGAGAACGAGCCTTTGCGGGGTTTCTCCTGGCTGTCCTCCGACCCCGG
CGGTCTCGAAAGCGACACGCTGCAGTGGGTGGAGGAGCCCCAACGCTCCTGCACCGCGCGGAGATGCGCGGTACT
CCAGGCCACCGGTGGGGTCGAGCCCGCAGGCTGGAAGGAGATGCGATGCCACCTGCGCGCCAACGGCTACCTGTG
CAAGTACCAGTTTGAGGTCTTGTGTCCTGCGCCGCGCCCCGGGGCCGCCTCTAACTTGAGCTATCGCGCGCCCTT
CCAGCTGCACAGCGCCGCTCTGGACTTCAGTCCACCTGGGACCGAGGTGAGTGCGCTCTGCCGGGGACAGCTCCC
GATCTCAGTTACTTGCATCGCGGACGAAATCGGCGCTCGCTGGGACAAACTCTCGGGCGATGTGTTGTGTCCCTG
CCCCGGGAGGTACCTCCGTGCTGGCAAATGCGCAGAGCTCCCTAACTGCCTAGACGACTTGGGAGGCTTTGCCTG
CGAATGTGCTACGGGCTTCGAGCTGGGGAAGGACGGCCGCTCTTGTGTGACCAGTGGGGAAGGACAGCCGACCCT
TGGGGGGACCGGGGTGCCCACCAGGCGCCCGCCGGCCACTGCAACCAGCCCCGTGCCGCAGAGAACATGGCCAAT
CAGGGTCGACGAGAAGCTGGGAGAGACACCACTTGTCCCTGAACAAGACAATTCAGTAACATCTATTCCTGAGAT
TCCTCGATGGGGATCACAGAGCACGATGTCTACCCTTCAAATGTCCCTTCAAGCCGAGTCAAAGGCCACTATCAC
CCCATCAGGGAGCGTGATTTCCAAGTTTAATTCTACGACTTCCTCTGCCACTCCTCAGGCTTTCGACTCCTCCTC
TGCCGTGGTCTTCATATTTGTGAGCACAGCAGTAGTAGTGTTGGTGATCTTGACCATGACAGTACTGGGGCTTGT
CAAGCTCTGCTTTCACGAAAGCCCCTCTTCCCAGCCAAGGAAGGAGTCTATGGGCCCGCCGGGCCTGGAGAGTGA
TCCTGAGCCCGCTGCTTTGGGCTCCAGTTCTGCACATTGCACAAACAATGGGGTGAAAGTCGGGGACTGTGATCT
GCGGGACAGAGCAGAGGGTGCCTTGCTGGCGGAGTCCCCTCTTGGCTCTAGTGATGCA**TAG**GGAAACAGGGGACA
TGGGCACTCCTGTGAACAGTTTTTCACTTTTGATGAAACGGGGAACCAAGAGGAACTTACTTGTGTAACTGACAA
TTTCTGCAGAAATCCCCCTTCCTCTAAATTCCCTTTACTCCACTGAGGAGCTAAATCAGAACTGCACACTCCTTC
CCTGATGATAGAGGAAGTGGAAGTGCCTTTAGGATGGTGATACTGGGGGACCGGGTAGTGCTGGGGAGAGATATT
TTCTTATGTTTATTCGGAGAATTTGGAGAAGTGATTGAACTTTTCAAGACATTGGAAACAAATAGAACACAATAT
AATTTACATTAAAAAATAATTTCTACCAAAATGGAAAGGAAATGTTCTATGTTGTTCAGGCTAGGAGTATATTGG
TTCGAAATCCCAGGGAAAAAAATAAAAATAAAAAATTAAAGGATTGTTGAT

# FIGURE 356

MRPAFALCLLWQALWPGPGGGEHPTADRAGCSASGACYSLHHATMKRQAAEEACILRGGALST
VRAGAELRAVLALLRAGPGPGGGSKDLLFWVALERRRSHCTLENEPLRGFSWLSSDPGGLESD
TLQWVEEPQRSCTARRCAVLQATGGVEPAGWKEMRCHLRANGYLCKYQFEVLCPAPRPGAASN
LSYRAPFQLHSAALDFSPPGTEVSALCRGQLPISVTCIADEIGARWDKLSGDVLCPCPGRYLR
AGKCAELPNCLDDLGGFACECATGFELGKDGRSCVTSGEGQPTLGGTGVPTRRPPATATSPVP
QRTWPIRVDEKLGETPLVPEQDNSVTSIPEIPRWGSQSTMSTLQMSLQAESKATITPSGSVIS
KFNSTTSSATPQAFDSSSAVVFIFVSTAVVVLVILTMTVLGLVKLCFHESPSSQPRKESMGPP
GLESDPEPAALGSSSAHCTNNGVKVGDCDLRDRAEGALLAESPLGSSDA

**Important features:**

**Signal sequence:**
amino acids 1-16

**Transmembrane domain:**
amino acids 399-418

**N-glycosylation site.**
amino acids 189-193, 381-385

**Glycosaminoglycan attachment site.**
amino acids 289-293

**cAMP- and cGMP-dependent protein kinase phosphorylation site.**
amino acids 98-102, 434-438

**Casein kinase II phosphorylation site.**
amino acids 275-279, 288-292, 342-346, 445-449

**N-myristoylation site.**
amino acids 30-36, 35-41, 58-64, 59-65, 121-127, 151-157, 185-191, 209-215, 267-273, 350-356, 374-380, 453-459, 463-469, 477-483

**Aspartic acid and asparagine hydroxylation site.**
amino acids 262-274

# FIGURE 357

CCCATCTCAAGCTGATCTTGGCACCTCTCATGCTCTGCTCTCTTCAACCAGACCTCTACATTCCATTTTGGAAGA
AGACTAAAA**ATG**GTGTTTCCAATGTGGACACTGAAGAGACAAATTCTTATCCTTTTTAACATAATCCTAATTTCC
AAACTCCTTGGGGCTAGATGGTTTCCTAAAACTCTGCCCTGTGATGTCACTCTGGATGTTCCAAAGAACCATGTG
ATCGTGGACTGCACAGACAAGCATTTGACAGAAATTCCTGGAGGTATTCCCACGAACACCACGAACCTCACCCTC
ACCATTAACCACATACCAGACATCTCCCCAGCGTCCTTTCACAGACTGGACCATCTGGTAGAGATCGATTTCAGA
TGCAACTGTGTACCTATTCCACTGGGGTCAAAAAACAACATGTGCATCAAGAGGCTGCAGATTAAACCCAGAAGC
TTTAGTGGACTCACTTATTTAAAATCCCTTTACCTGGATGGAAACCAGCTACTAGAGATACCGCAGGGCCTCCCG
CCTAGCTTACAGCTTCTCAGCCTTGAGGCCAACAACATCTTTTCCATCAGAAAAGAGAATCTAACAGAACTGGCC
AACATAGAAATACTCTACCTGGGCCAAAACTGTTATTATCGAAATCCTTGTTATGTTTCATATTCAATAGAGAAA
GATGCCTTCCTAAACTTGACAAAGTTAAAAGTGCTCTCCCTGAAAGATAACAATGTCACAGCCGTCCCTACTGTT
TTGCCATCTACTTTAACAGAACTATATCTCTACAACAACATGATTGCAAAAATCCAAGAAGATGATTTTAATAAC
CTCAACCAATTACAAATTCTTGACCTAAGTGGAAATTGCCCTCGTTGTTATAATGCCCCATTTCCTTGTGCGCCG
TGTAAAAATAATTCTCCCCTACAGATCCCTGTAAATGCTTTTGATGCGCTGACAGAATTAAAAGTTTTACGTCTA
CACAGTAACTCTCTTCAGCATGTGCCCCCAAGATGGTTTAAGAACATCAACAAACTCCAGGAACTGGATCTGTCC
CAAAACTTCTTGGCCAAAGAAATTGGGGATGCTAAATTTCTGCATTTTCTCCCCAGCCTCATCCAATTGGATCTG
TCTTTCAATTTTGAACTTCAGGTCTATCGTGCATCTATGAATCTATCACAAGCATTTTCTTCACTGAAAAGCCTG
AAAATTCTGCGGATCAGAGGATATGTCTTTAAAGAGTTGAAAAGCTTTAACCTCTCGCCATTACATAATCTTCAA
AATCTTGAAGTTCTTGATCTTGGCACTAACTTTATAAAAATTGCTAACCTCAGCATGTTTAAACAATTTAAAAGA
CTGAAAGTCATAGATCTTTCAGTGAATAAAATATCACCTTCAGGAGATTCAAGTGAAGTTGGCTTCTGCTCAAAT
GCCAGAACTTCTGTAGAAAGTTATGAACCCCAGGTCCTGGAACAATTACATTATTTCAGATATGATAAGTATGCA
AGGAGTTGCAGATTCAAAAACAAAGAGGCTTCTTTCATGTCTGTTAATGAAAGCTGCTACAAGTATGGGCAGACC
TTGGATCTAAGTAAAAATAGTATATTTTTTGTCAAGTCCTCTGATTTTCAGCATCTTTCTTTCCTCAAATGCCTG
AATCTGTCAGGAAATCTCATTAGCCAAACTCTTAATGGCAGTGAATTCCAACCTTTAGCAGAGCTGAGATATTTG
GACTTCTCCAACAACCGGCTTGATTTACTCCATTCAACAGCATTTGAAGAGCTTCACAAACTGGAAGTTCTGGAT
ATAAGCAGTAATAGCCATTATTTTCAATCAGAAGGAATTACTCATATGCTAAACTTTACCAAGAACCTAAAGGTT
CTGCAGAAACTGATGATGAACGACAATGACATCTCTTCCTCCACCAGCAGGACCATGGAGAGTGAGTCTCTTAGA
ACTCTGGAATTCAGAGGAAATCACTTAGATGTTTTATGGAGAGAAGGTGATAACAGATACTTACAATTATTCAAG
AATCTGCTAAAATTAGAGGAATTAGACATCTCTAAAAATTCCCTAAGTTTCTTGCCTTCTGGAGTTTTTGATGGT
ATGCCTCCAAATCTAAAGAATCTCTCTTTGGCCAAAAATGGGCTCAAATCTTTCAGTTGGAAGAAACTCCAGTGT
CTAAAGAACCTGGAAACTTTGGACCTCAGCCACAACCAACTGACCACTGTCCCTGAGAGATTATCCAACTGTTCC
AGAAGCCTCAAGAATCTGATTCTTAAGAATAATCAAATCAGGAGTCTGACGAAGTATTTTCTACAAGATGCCTTC
CAGTTGCGATATCTGGATCTCAGCTCAAATAAAATCCAGATGATCCAAAAGACCAGCTTCCCAGAAAATGTCCTC
AACAATCTGAAGATGTTGCTTTTGCATCATAATCGGTTTCTGTGCACCTGTGATGCTGTGTGGTTTGTCTGGTGG
GTTAACCATACGGAGGTGACTATTCCTTACCTGGCCACAGATGTGACTTGTGTGGGGCCAGGAGCACACAAGGGC
CAAAGTGTGATCTCCCTGGATCTGTACACCTGTGAGTTAGATCTGACTAACCTGATTCTGTTCTCACTTTCCATA
TCTGTATCTCTCTTTCTCATGGTGATGATGACAGCAAGTCACCTCTATTTCTGGGATGTGTGGTATATTTACCAT
TTCTGTAAGGCCAAGATAAAGGGGTATCAGCGTCTAATATCACCAGACTGTTGCTATGATGCTTTTATTGTGTAT
GACACTAAAGACCCAGCTGTGACCGAGTGGGTTTTGGCTGAGCTGGTGGCCAAACTGGAAGACCCAAGAGAGAAA
CATTTTAATTTATGTCTCGAGGAAAGGGACTGGTTACCAGGGCAGCCAGTTCTGGAAAACCTTTCCCAGAGCATA
CAGCTTAGCAAAAAGACAGTGTTTGTGATGACAGACAAGTATGCAAAGACTGAAAATTTTAAGATAGCATTTTAC
TTGTCCCATCAGAGGCTCATGGATGAAAAAGTTGATGTGATTATCTTGATATTTCTTGAGAAGCCCTTTCAGAAG
TCCAAGTTCCTCCAGCTCCGGAAAAGGCTCTGTGGGAGTTCTGTCCTTGAGTGGCCAACAAACCCGCAAGCTCAC
CCATACTTCTGGCAGTGTCTAAAGAACGCCCTGGCCACAGACAATCATGTGGCCTATAGTCAGGTGTTCAAGGAA
ACGGTC**TAG**CCCTTCTTTGCAAAACACAACTGCCTAGTTTACCAAGGAGAGGCCTGGC

# FIGURE 358

MVFPMWTLKRQILILFNIILISKLLGARWFPKTLPCDVTLDVPKNHVIVDCTDKHLTEIPGGI
PTNTTNLTLTINHIPDISPASFHRLDHLVEIDFRCNCVPIPLGSKNNMCIKRLQIKPRSFSGL
TYLKSLYLDGNQLLEIPQGLPPSLQLLSLEANNIFSIRKENLTELANIEILYLGQNCYYRNPC
YVSYSIEKDAFLNLTKLKVLSLKDNNVTAVPTVLPSTLTELYLYNNMIAKIQEDDFNNLNQLQ
ILDLSGNCPRCYNAPFPCAPCKNNSPLQIPVNAFDALTELKVLRLHSNSLQHVPPRWFKNINK
LQELDLSQNFLAKEIGDAKFLHFLPSLIQLDLSFNFELQVYRASMNLSQAFSSLKSLKILRIR
GYVFKELKSFNLSPLHNLQNLEVLDLGTNFIKIANLSMFKQFKRLKVIDLSVNKISPSGDSSE
VGFCSNARTSVESYEPQVLEQLHYFRYDKYARSCRFKNKEASFMSVNESCYKYGQTLDLSKNS
IFFVKSSDFQHLSFLKCLNLSGNLISQTLNGSEFQPLAELRYLDFSNNRLDLLHSTAFEELHK
LEVLDISSNSHYFQSEGITHMLNFTKNLKVLQKLMMNDNDISSSTSRTMESESLRTLEFRGNH
LDVLWREGDNRYLQLFKNLLKLEELDISKNSLSFLPSGVFDGMPPNLKNLSLAKNGLKSFSWK
KLQCLKNLETLDLSHNQLTTVPERLSNCSRSLKNLILKNNQIRSLTKYFLQDAFQLRYLDLSS
NKIQMIQKTSFPENVLNNLKMLLLHHNRFLCTCDAVWFVWWVNHTEVTIPYLATDVTCVGPGA
HKGQSVISLDLYTCELDLTNLILFSLSISVSLFLMVMMTASHLYFWDVWYIYHFCKAKIKGYQ
RLISPDCCYDAFIVYDTKDPAVTEWVLAELVAKLEDPREKHFNLCLEERDWLPGQPVLENLSQ
SIQLSKKTVFVMTDKYAKTENFKIAFYLSHQRLMDEKVDVIILIFLEKPFQKSKFLQLRKRLC
GSSVLEWPTNPQAHPYFWQCLKNALATDNHVAYSQVFKETV

**Important features:**

**Signal sequence:**

amino acids 1-26

**Transmembrane domain:**

amino acids 840-860

# FIGURE 359

GACGGCTGGCCACC**ATG**CACGGCTCCTGCAGTTTCCTGATGCTTCTGCTGCCGCTACTGCTAC
TGCTGGTGGCCACCACAGGCCCCGTTGGAGCCCTCACAGATGAGGAGAAACGTTTGATGGTGG
AGCTGCACAACCTCTACCGGGCCCAGGTATCCCCGACGGCCTCAGACATGCTGCACATGAGAT
GGGACGAGGAGCTGGCCGCCTTCGCCAAGGCCTACGCACGGCAGTGCGTGTGGGGCCACAACA
AGGAGCGCGGGCGCCGCGGCGAGAATCTGTTCGCCATCACAGACGAGGGCATGGACGTGCCGC
TGGCCATGGAGGAGTGGCACCACGAGCGTGAGCACTACAACCTCAGCGCCGCCACCTGCAGCC
CAGGCCAGATGTGCGGCCACTACACGCAGGTGGTATGGGCCAAGACAGAGAGGATCGGCTGTG
GTTCCCACTTCTGTGAGAAGCTCCAGGGTGTTGAGGAGACCAACATCGAATTACTGGTGTGCA
ACTATGAGCCTCCGGGGAACGTGAAGGGGAAACGGCCCTACCAGGAGGGGACTCCGTGCTCCC
AATGTCCCTCTGGCTACCACTGCAAGAACTCCCTCTGTGAACCCATCGGAAGCCCGGAAGATG
CTCAGGATTTGCCTTACCTGGTAACTGAGGCCCCATCCTTCCGGGCGACTGAAGCATCAGACT
CTAGGAAAATGGGTACTCCTTCTTCCCTAGCAACGGGGATTCCGGCTTTCTTGGTAACAGAGG
TCTCAGGCTCCCTGGCAACCAAGGCTCTGCCTGCTGTGGAAACCCAGGCCCCAACTTCCTTAG
CAACGAAAGACCCGCCCTCCATGGCAACAGAGGCTCCACCTTGCGTAACAACTGAGGTCCCTT
CCATTTTGGCAGCTCACAGCCTGCCCTCCTTGGATGAGGAGCCAGTTACCTTCCCCAAATCGA
CCCATGTTCCTATCCCAAAATCAGCAGACAAAGTGACAGACAAAACAAAAGTGCCCTCTAGGA
GCCCAGAGAACTCTCTGGACCCCAAGATGTCCCTGACAGGGGCAAGGGAACTCCTACCCCATG
CCCAGGAGGAGGCTGAGGCTGAGGCTGAGTTGCCTCCTTCCAGTGAGGTCTTGGCCTCAGTTT
TTCCAGCCCAGGACAAGCCAGGTGAGCTGCAGGCCACACTGGACCACACGGGGCACACCTCCT
CCAAGTCCCTGCCCAATTTCCCCAATACCTCTGCCACCGCTAATGCCACGGGTGGGCGTGCCC
TGGCTCTGCAGTCGTCCTTGCCAGGTGCAGAGGGCCCTGACAAGCCTAGCGTTGTGTCAGGGC
TGAACTCGGGCCCTGGTCATGTGTGGGGCCCTCTCCTGGGACTACTGCTCCTGCCTCCTCTGG
TGTTGGCTGGAATCTTC**TGA**ATGGGATACCACTCAAAGGGTGAAGAGGTCAGCTGTCCTCCTG
TCATCTTCCCCACCCTGTCCCCAGCCCCTAAACAAGATACTTCTTGGTTAAGGCCCTCCGGAA
GGGAAAGGCTACGGGGCATGTGCCTCATCACACCATCCATCCTGGAGGCACAAGGCCTGGCTG
GCTGCGAGCTCAGGAGGCCGCCTGAGGACTGCACACCGGGCCCACACCTCTCCTGCCCCTCCC
TCCTGAGTCCTGGGGGTGGGAGGATTTGAGGGAGCTCACTGCCTACCTGGCCTGGGGCTGTCT
GCCCACACAGCATGTGCGCTCTCCCTGAGTGCCTGTGTAGCTGGGGATGGGGATTCCTAGGGG
CAGATGAAGGACAAGCCCCACTGGAGTGGGGTTCTTTGAGTGGGGGAGGCAGGGACGAGGGAA
GGAAAGTAACTCCTGACTCTCCAATAAAAACCTGTCCAACCTGTGAAA

# FIGURE 360

MHGSCSFLMLLLPLLLLLVATTGPVGALTDEEKRLMVELHNLYRAQVSPTASDMLHMRWDEEL
AAFAKAYARQCVWGHNKERGRRGENLFAITDEGMDVPLAMEEWHHEREHYNLSAATCSPGQMC
GHYTQVVWAKTERIGCGSHFCEKLQGVEETNIELLVCNYEPPGNVKGKRPYQEGTPCSQCPSG
YHCKNSLCEPIGSPEDAQDLPYLVTEAPSFRATEASDSRKMGTPSSLATGIPAFLVTEVSGSL
ATKALPAVETQAPTSLATKDPPSMATEAPPCVTTEVPSILAAHSLPSLDEEPVTFPKSTHVPI
PKSADKVTDKTKVPSRSPENSLDPKMSLTGARELLPHAQEEAEAEAELPPSSEVLASVFPAQD
KPGELQATLDHTGHTSSKSLPNFPNTSATANATGGRALALQSSLPGAEGPDKPSVVSGLNSGP
GHVWGPLLGLLLLPPLVLAGIF

**Important features:**

**Signal sequence:**

amino acids 1-22

**N-glycosylation site.**

amino acids 114-118, 403-407, 409-413

**Glycosaminoglycan attachment site.**

amino acids 439-443

**Casein kinase II phosphorylation site.**

amino acids 29-33, 50-54, 156-160, 195-199, 202-206, 299-303

**N-myristoylation site.**

amino acids 123-129, 143-149, 152-158, 169-175, 180-186, 231-237, 250-256

**Amidation site.**

amino acids 82-86, 172-176

**Peroxidases proximal heme-ligand signature.**

amino acids 287-298

**Extracellular proteins SCP/Tpx-1/Ag5/PR-1/Sc7 signature 1.**

amino acids 127-138

**Extracellular proteins SCP/Tpx-1/Ag5/PR-1/Sc7 signature 2.**

amino acids 160-172

# FIGURE 361

GACTAGTTCTCTTGGAGTCTGGGAGGAGGAAAGCGGAGCCGGCAGGGAGCGAACCAGGACTGG
GGTGACGGCAGGGCAGGGGGCGCCTGGCCGGGGAGAAGCGCGGGGGCTGGAGCACCACCAACT
GGAGGGTCCGGAGTAGCGAGCGCCCCGAAGGAGGCCATCGGGGAGCCGGGAGGGGGGACTGCG
AGAGGACCCCGGCGTCCGGGCTCCCGGTGCCAGCGCT**ATG**AGGCCACTCCTCGTCCTGCTGCT
CCTGGGCCTGGCGGCCGGCTCGCCCCCACTGGACGACAACAAGATCCCCAGCCTCTGCCCGGG
GCACCCCGGCCTTCCAGGCACGCCGGGCCACCATGGCAGCCAGGGCTTGCCGGGCCGCGATGG
CCGCGACGGCCGCGACGGCGCGCCCGGGGCTCCGGGAGAGAAAGGCGAGGGCGGGAGGCCGGG
ACTGCCGGGACCTCGAGGGGACCCCGGGCCGCGAGGAGAGGCGGGACCCGCGGGGCCCACCGG
GCCTGCCGGGGAGTGCTCGGTGCCTCCGCGATCCGCCTTCAGCGCCAAGCGCTCCGAGAGCCG
GGTGCCTCCGCCGTCTGACGCACCCTTGCCCTTCGACCGCGTGCTGGTGAACGAGCAGGGACA
TTACGACGCCGTCACCGGCAAGTTCACCTGCCAGGTGCCTGGGGTCTACTACTTCGCCGTCCA
TGCCACCGTCTACCGGGCCAGCCTGCAGTTTGATCTGGTGAAGAATGGCGAATCCATTGCCTC
TTTCTTCCAGTTTTTCGGGGGGTGGCCCAAGCCAGCCTCGCTCTCGGGGGGGGCCATGGTGAG
GCTGGAGCCTGAGGACCAAGTGTGGGTGCAGGTGGGTGTGGGTGACTACATTGGCATCTATGC
CAGCATCAAGACAGACAGCACCTTCTCCGGATTTCTGGTGTACTCCGACTGGCACAGCTCCCC
AGTCTTTGCT**TAG**TGCCCACTGCAAAGTGAGCTCATGCTCTCACTCCTAGAAGGAGGGTGTGA
GGCTGACAACCAGGTCATCCAGGAGGGCTGGCCCCCCTGGAATATTGTGAATGACTAGGGAGG
TGGGGTAGAGCACTCTCCGTCCTGCTGCTGGCAAGGAATGGGAACAGTGGCTGTCTGCGATCA
GGTCTGGCAGCATGGGGCAGTGGCTGGATTTCTGCCCAAGACCAGAGGAGTGTGCTGTGCTGG
CAAGTGTAAGTCCCCCAGTTGCTCTGGTCCAGGAGCCCACGGTGGGGTGCTCTCTTCCTGGTC
CTCTGCTTCTCTGGATCCTCCCCACCCCCTCCTGCTCCTGGGGCCGGCCCTTTTCTCAGAGAT
CACTCAATAAACCTAAGAACCCTCATAAAAAAAAAAAAAAAAAAAAAAAAAAA

# FIGURE 362

MRPLLVLLLLGLAAGSPPLDDNKIPSLCPGHPGLPGTPGHHGSQGLPGRDGRDGRDGAPGAPG
EKGEGGRPGLPGPRGDPGPRGEAGPAGPTGPAGECSVPPRSAFSAKRSESRVPPPSDAPLPFD
RVLVNEQGHYDAVTGKFTCQVPGVYYFAVHATVYRASLQFDLVKNGESIASFFQFFGGWPKPA
SLSGGAMVRLEPEDQVWVQVGVGDYIGIYASIKTDSTFSGFLVYSDWHSSPVFA

**Important features:**

**Signal sequence.**

amino acids 1-15

**N-myristoylation sites.**

amino acids 11-17, 68-74, 216-222

**Cell attachment sequence.**

amino acids 77-80

# FIGURE 363

GGAGAGCGGAGCGAAGCTGGATAACAGGGGACCG**ATG**ATGTGGCGACCATCAGTTCTGCTGCT
TCTGTTGCTACTGAGGCACGGGGCCCAGGGGAAGCCATCCCCAGACGCAGGCCCTCATGGCCA
GGGGAGGGTGCACCAGGCGGCCCCCCTGAGCGACGCTCCCCATGATGACGCCCACGGGAACTT
CCAGTACGACCATGAGGCTTTCCTGGGACGGGAAGTGGCCAAGGAATTCGACCAACTCACCCC
AGAGGAAAGCCAGGCCCGTCTGGGGCGGATCGTGGACCGCATGGACCGCGCGGGGGACGGCGA
CGGCTGGGTGTCGCTGGCCGAGCTTCGCGCGTGGATCGCGCACACGCAGCAGCGGCACATACG
GGACTCGGTGAGCGCGGCCTGGGACACGTACGACACGGACCGCGACGGGCGTGTGGGTTGGGA
GGAGCTGCGCAACGCCACCTATGGCCACTACGCGCCCGGTGAAGAATTTCATGACGTGGAGGA
TGCAGAGACCTACAAAAAGATGCTGGCTCGGGACGAGCGGCGTTTCCGGGTGGCCGACCAGGA
TGGGGACTCGATGGCCACTCGAGAGGAGCTGACAGCCTTCCTGCACCCCGAGGAGTTCCCTCA
CATGCGGGACATCGTGATTGCTGAAACCCTGGAGGACCTGGACAGAAACAAAGATGGCTATGT
CCAGGTGGAGGAGTACATCGCGGATCTGTACTCAGCCGAGCCTGGGGAGGAGGAGCCGGCGTG
GGTGCAGACGGAGAGGCAGCAGTTCCGGGACTTCCGGGATCTGAACAAGGATGGGCACCTGGA
TGGGAGTGAGGTGGGCCACTGGGTGCTGCCCCCTGCCCAGGACCAGCCCCTGGTGGAAGCCAA
CCACCTGCTGCACGAGAGCGACACGGACAAGGATGGGCGGCTGAGCAAAGCGGAAATCCTGGG
TAATTGGAACATGTTTGTGGGCAGTCAGGCCACCAACTATGGCGAGGACCTGACCCGGCACCA
CGATGAGCTG**TGA**GCACCGCGCACCTGCCACAGCCTCAGAGGCCCGCACAATGACCGGAGGAG
GGGCCGCTGTGGTCTGGCCCCCTCCCTGTCCAGGCCCCGCAGGAGGCAGATGCAGTCCCAGGC
ATCCTCCTGCCCCTGGGCTCTCAGGGACCCCCTGGGTCGGCTTCTGTCCCTGTCACACCCCCA
ACCCCAGGGAGGGGCTGTCATAGTCCCAGAGGATAAGCAATACCTATTTCTGACTGAGTCTCC
CAGCCCAGACCCAGGGACCCTTGGCCCCAAGCTCAGCTCTAAGAACCGCCCCAACCCCTCCAG
CTCCAAATCTGAGCCTCCACCACATAGACTGAAACTCCCCTGGCCCCAGCCCTCTCCTGCCTG
GCCTGGCCTGGGACACCTCCTCTCTGCCAGGAGGCAATAAAAGCCAGCGCCGGGACCTTGAAA
AAAAAAAAAAAAAAAAAAAAAAAAAAAAAAAAAAAAAAAAAAAAAAAAAAAAAA

# FIGURE 364

MMWRPSVLLLLLLLLRHGAQGKPSPDAGPHGQGRVHQAAPLSDAPHDDAHGNFQYDHEAFLGRE
VAKEFDQLTPEESQARLGRIVDRMDRAGDGDGWVSLAELRAWIAHTQQRHIRDSVSAAWDTYD
TDRDGRVGWEELRNATYGHYAPGEEFHDVEDAETYKKMLARDERRFRVADQDGDSMATREELT
AFLHPEEFPHMRDIVIAETLEDLDRNKDGYVQVEEYIADLYSAEPGEEEPAWVQTERQQFRDF
RDLNKDGHLDGSEVGHWVLPPAQDQPLVEANHLLHESDTDKDGRLSKAEILGNWNMFVGSQAT
NYGEDLTRHHDEL

**Important features:**

**Signal sequence:**

amino acids 1-20

**N-glycosylation site.**

amino acids 140-144

**Casein kinase II phosphorylation site.**

amino acids 72-76, 98-102, 127-131, 184-188, 208-212, 289-293, 291-295, 298-302

**N-myristoylation site.**

amino acids 263-269, 311-317

**Endoplasmic reticulum targeting sequence.**

amino acids 325-330

# FIGURE 365

GTCTGTTCCCAGGAGTCCTTCGGCGGCTGTTGTGTCAGTGGCCTGATCGCG**ATG**GGGACAAAG
GCGCAAGTCGAGAGGAAACTGTTGTGCCTCTTCATATTGGCGATCCTGTTGTGCTCCCTGGCA
TTGGGCAGTGTTACAGTGCACTCTTCTGAACCTGAAGTCAGAATTCCTGAGAATAATCCTGTG
AAGTTGTCCTGTGCCTACTCGGGCTTTTCTTCTCCCCGTGTGGAGTGGAAGTTTGACCAAGGA
GACACCACCAGACTCGTTTGCTATAATAACAAGATCACAGCTTCCTATGAGGACCGGGTGACC
TTCTTGCCAACTGGTATCACCTTCAAGTCCGTGACACGGGAAGACACTGGGACATACACTTGT
ATGGTCTCTGAGGAAGGCGGCAACAGCTATGGGGAGGTCAAGGTCAAGCTCATCGTGCTTGTG
CCTCCATCCAAGCCTACAGTTAACATCCCCTCCTCTGCCACCATTGGGAACCGGGCAGTGCTG
ACATGCTCAGAACAAGATGGTTCCCCACCTTCTGAATACACCTGGTTCAAAGATGGGATAGTG
ATGCCTACGAATCCCAAAAGCACCCGTGCCTTCAGCAACTCTTCCTATGTCCTGAATCCCACA
ACAGGAGAGCTGGTCTTTGATCCCCTGTCAGCCTCTGATACTGGAGAATACAGCTGTGAGGCA
CGGAATGGGTATGGGACACCCATGACTTCAAATGCTGTGCGCATGGAAGCTGTGGAGCGGAAT
GTGGGGGTCATCGTGGCAGCCGTCCTTGTAACCCTGATTCTCCTGGGAATCTTGGTTTTTGGC
ATCTGGTTTGCCTATAGCCGAGGCCACTTTGACAGAACAAAGAAAGGGACTTCGAGTAAGAAG
GTGATTTACAGCCAGCCTAGTGCCCGAAGTGAAGGAGAATTCAAACAGACCTCGTCATTCCTG
GTG**TGA**GCCTGGTCGGCTCACCGCCTATCATCTGCATTTGCCTTACTCAGGTGCTACCGGACT
CTGGCCCCTGATGTCTGTAGTTTCACAGGATGCCTTATTTGTCTTCTACACCCCACAGGGCCC
CCTACTTCTTCGGATGTGTTTTTAATAATGTCAGCTATGTGCCCCATCCTCCTTCATGCCCTC
CCTCCCTTTCCTACCACTGCTGAGTGGCCTGGAACTTGTTTAAAGTGTTTATTCCCCATTTCT
TTGAGGGATCAGGAAGGAATCCTGGGTATGCCATTGACTTCCCTTCTAAGTAGACAGCAAAAA
TGGCGGGGGTCGCAGGAATCTGCACTCAACTGCCCACCTGGCTGGCAGGGATCTTTGAATAGG
TATCTTGAGCTTGGTTCTGGGCTCTTTCCTTGTGTACTGACGACCAGGGCCAGCTGTTCTAGA
GCGGGAATTAGAGGCTAGAGCGGCTGAAATGGTTGTTTGGTGATGACACTGGGGTCCTTCCAT
CTCTGGGGCCCACTCTCTTCTGTCTTCCCATGGGAAGTGCCACTGGGATCCCTCTGCCCTGTC
CTCCTGAATACAAGCTGACTGACATTGACTGTGTCTGTGGAAAATGGGAGCTCTTGTTGTGGA
GAGCATAGTAAATTTTCAGAGAACTTGAAGCCAAAAGGATTTAAAACCGCTGCTCTAAAGAAA
AGAAAACTGGAGGCTGGGCGCAGTGGCTCACGCCTGTAATCCCAGAGGCTGAGGCAGGCGGAT
CACCTGAGGTCGGGAGTTCGGGATCAGCCTGACCAACATGGAGAAACCCTACTGGAAATACAA
AGTTAGCCAGGCATGGTGGTGCATGCCTGTAGTCCCAGCTGCTCAGGAGCCTGGCAACAAGAG
CAAAACTCCAGCTCAAAAAAAAAAAAAAAA

# FIGURE 366

MGTKAQVERKLLCLFILAILLCSLALGSVTVHSSEPEVRIPENNPVKLSCAYSGFSSPRVEWK
FDQGDTTRLVCYNNKITASYEDRVTFLPTGITFKSVTREDTGTYTCMVSEEGGNSYGEVKVKL
IVLVPPSKPTVNIPSSATIGNRAVLTCSEQDGSPPSEYTWFKDGIVMPTNPKSTRAFSNSSYV
LNPTTGELVFDPLSASDTGEYSCEARNGYGTPMTSNAVRMEAVERNVGVIVAAVLVTLILLGI
LVFGIWFAYSRGHFDRTKKGTSSKKVIYSQPSARSEGEFKQTSSFLV

**Important features:**

**Signal sequence:**

amino acids 1-27

**Transmembrane domain:**

amino acids 238-255

**N-glycosylation site.**

amino acids 185-189

**cAMP- and cGMP-dependent protein kinase phosphorylation site.**

amino acids 270-274

**Casein kinase II phosphorylation site.**

amino acids 34-38, 82-86, 100-104, 118-122, 152-156, 154-158, 193-197, 203-207, 287-291

**N-myristoylation site.**

amino acids 105-111, 116-122, 158-164, 219-225, 237-243, 256-262

# FIGURE 367

GGGGAGAGGAATTGACCATGTAAAAGGAGACTTTTTTTTTGGTGGTGGTGGCTGTTGGGTGCCTTGCAAAAATG
AAGGATGCAGGACGCAGCTTTCTCCTGGAACCGAACGCAATGGATAAACTGATTGTGCAAGAGAGAAGGAAGAAC
GAAGCTTTTTCTTGTGAGCCCTGGATCTTAACACAAATGTGTATATGTGCACACAGGGAGCATTCAAGAATGAAA
TAAACCAGAGTTAGACCCGCGGGGGTTGGTGTGTTCTGACATAAATAAATAATCTTAAAGCAGCTGTTCCCCTCC
CCACCCCCAAAAAAAAGGATGATTGGAAATGAAGAACCGAGGATTCACAAAGAAAAAAGTATGTTCATTTTTCTC
TATAAAGGAGAAAGTGAGCCAAGGAGATATTTTTGGAATGAAAAGTTTGGGGCTTTTTTAGTAAAGTAAAGAACT
GGTGTGGTGGTGTTTTCCTTTCTTTTTGAATTTCCCACAAGAGGAGAGGAAATTAATAATACATCTGCAAAGAAA
TTTCAGAGAAGAAAAGTTGACCGCGGCAGATTGAGGCATTGATTGGGGGAGAGAAACCAGCAGAGCACAGTTGGA
TTTGTGCCTATGTTGACTAAAATTGACGGATAATTGCAGTTGGATTTTTCTTCATCAACCTCCTTTTTTTTAAAT
TTTTATTCCTTTTGGTATCAAGATCATGCGTTTTCTCTTGTTCTTAACCACCTGGATTTCCATCTGGATGTTGCT
GTGATCAGTCTGAAATACAACTGTTTGAATTCCAGAAGGACCAACACCAGATAAATTATGA**ATG**TTGAACAAGAT
GACCTTACATCCACAGCAGATAATGATAGGTCCTAGGTTTAACAGGGCCCTATTTGACCCCCTGCTTGTGGTGCT
GCTGGCTCTTCAACTTCTTGTGGTGGCTGGTCTGGTGCGGGCTCAGACCTGCCCTTCTGTGTGCTCCTGCAGCAA
CCAGTTCAGCAAGGTGATTTGTGTTCGGAAAAACCTGCGTGAGGTTCCGGATGGCATCTCCACCAACACACGGCT
GCTGAACCTCCATGAGAACCAAATCCAGATCATCAAAGTGAACAGCTTCAAGCACTTGAGGCACTTGGAAATCCT
ACAGTTGAGTAGGAACCATATCAGAACCATTGAAATTGGGGCTTTCAATGGTCTGGCGAACCTCAACACTCTGGA
ACTCTTTGACAATCGTCTTACTACCATCCCGAATGGAGCTTTTGTATACTTGTCTAAACTGAAGGAGCTCTGGTT
GCGAAACAACCCCATTGAAAGCATCCCTTCTTATGCTTTTAACAGAATTCCTTCTTTGCGCCGACTAGACTTAGG
GGAATTGAAAAGACTTTCATACATCTCAGAAGGTGCCTTTGAAGGTCTGTCCAACTTGAGGTATTTGAACCTTGC
CATGTGCAACCTTCGGGAAATCCCTAACCTCACACCGCTCATAAAACTAGATGAGCTGGATCTTTCTGGGAATCA
TTTATCTGCCATCAGGCCTGGCTCTTTCCAGGGTTTGATGCACCTTCAAAAACTGTGGATGATACAGTCCCAGAT
TCAAGTGATTGAACGGAATGCCTTTGACAACCTTCAGTCACTAGTGGAGATCAACCTGGCACACAATAATCTAAC
ATTACTGCCTCATGACCTCTTCACTCCCTTGCATCATCTAGAGCGGATACATTTACATCACAACCCTTGGAACTG
TAACTGTGACATACTGTGGCTCAGCTGGTGGATAAAAGACATGGCCCCCTCGAACACAGCTTGTTGTGCCCGGTG
TAACACTCCTCCCAATCTAAAGGGGAGGTACATTGGAGAGCTCGACCAGAATTACTTCACATGCTATGCTCCGGT
GATTGTGGAGCCCCCTGCAGACCTCAATGTCACTGAAGGCATGGCAGCTGAGCTGAAATGTCGGGCCTCCACATC
CCTGACATCTGTATCTTGGATTACTCCAAATGGAACAGTCATGACACATGGGGCGTACAAAGTGCGGATAGCTGT
GCTCAGTGATGGTACGTTAAATTTCACAAATGTAACTGTGCAAGATACAGGCATGTACACATGTATGGTGAGTAA
TTCCGTTGGGAATACTACTGCTTCAGCCACCCTGAATGTTACTGCAGCAACCACTACTCCTTTCTCTTACTTTTC
AACCGTCACAGTAGAGACTATGGAACCGTCTCAGGATGAGGCACGGACCACAGATAACAATGTGGGTCCCACTCC
AGTGGTCGACTGGGAGACCACCAATGTGACCACCTCTCTCACACCACAGAGCACAAGGTCGACAGAGAAAACCTT
CACCATCCCAGTGACTGATATAAACAGTGGGATCCCAGGAATTGATGAGGTCATGAAGACTACCAAAATCATCAT
TGGGTGTTTTGTGGCCATCACACTCATGGCTGCAGTGATGCTGGTCATTTTCTACAAGATGAGGAAGCAGCACCA
TCGGCAAAACCATCACGCCCCAACAAGGACTGTTGAAATTATTAATGTGGATGATGAGATTACGGGAGACACACC
CATGGAAAGCCACCTGCCCATGCCTGCTATCGAGCATGAGCACCTAAATCACTATAACTCATACAAATCTCCCTT
CAACCACACAACAACAGTTAACACAATAAATTCAATACACAGTTCAGTGCATGAACCGTTATTGATCCGAATGAA
CTCTAAAGACAATGTACAAGAGACTCAAATC**TAA**AACATTTACAGAGTTACAAAAAACAAACAATCAAAAAAAAA
GACAGTTTATTAAAAATGACACAAATGACTGGGCTAAATCTACTGTTTCAAAAAAGTGTCTTTACAAAAAAACAA
AAAAGAAAAGAAATTTATTTATTAAAAATTCTATTGTGATCTAAAGCAGACAAAAA

# FIGURE 368

MLNKMTLHPQQIMIGPRFNRALFDPLLVVLLALQLLVVAGLVRAQTCPSVCSCSNQFSKVICVRKNLREVPDGIS
TNTRLLNLHENQIQIIKVNSFKHLRHLEILQLSRNHIRTIEIGAFNGLANLNTLELFDNRLTTIPNGAFVYLSKL
KELWLRNNPIESIPSYAFNRIPSLRRLDLGELKRLSYISEGAFEGLSNLRYLNLAMCNLREIPNLTPLIKLDELD
LSGNHLSAIRPGSFQGLMHLQKLWMIQSQIQVIERNAFDNLQSLVEINLAHNNLTLLPHDLFTPLHHLERIHLHH
NPWNCNCDILWLSWWIKDMAPSNTACCARCNTPPNLKGRYIGELDQNYFTCYAPVIVEPPADLNVTEGMAAELKC
RASTSLTSVSWITPNGTVMTHGAYKVRIAVLSDGTLNFTNVTVQDTGMYTCMVSNSVGNTTASATLNVTAATTTP
FSYFSTVTVETMEPSQDEARTTDNNVGPTPVVDWETTNVTTSLTPQSTRSTEKTFTIPVTDINSGIPGIDEVMKT
TKIIIGCFVAITLMAAVMLVIFYKMRKQHHRQNHHAPTRTVEIINVDDEITGDTPMESHLPMPAIEHEHLNHYNS
YKSPFNHTTTVNTINSIHSSVHEPLLIRMNSKDNVQETQI

**Important features:**

**Signal sequence:**

amino acids 1-44

**Transmembrane domain:**

amino acids 523-543

**N-glycosylation site.**

amino acids 278-282, 364-368, 390-394, 412-416, 415-419, 434-438, 442-446, 488-492, 606-610

**cAMP- and cGMP-dependent protein kinase phosphorylation site.**

amino acids 183-187

**Casein kinase II phosphorylation site.**

amino acids 268-272, 417-421, 465-469, 579-583, 620-624

**N-myristoylation site.**

amino acids 40-46, 73-79, 118-124, 191-197, 228-234, 237-243, 391-397, 422-428, 433-439, 531-537

# FIGURE 369

CAAAACTTGCGTCGCGGAGAGCGCCCAGCTTGACTTGAATGGAAGGAGCCCGAGCCCGCGGAGCGCAGCTGAGAC
TGGGGGAGCGCGTTCGGCCTGTGGGGCGCCGCTCGGCGCCGGGGCGCAGCAGGGAAGGGGAAGCTGTGGTCTGCC
CTGCTCCACGAGGCGCCACTGGTGTGAACCGGGAGAGCCCCTGGGTGGTCCCGTCCCCTATCCCTCCTTTATATA
GAAACCTTCCACACTGGGAAGGCAGCGGCGAGGCAGGAGGGCTCATGGTGAGCAAGGAGGCCGGCTGATCTGCAG
GCGCACAGCATTCCGAGTTTACAGATTTTTACAGATACCAA**ATG**GAAGGCGAGGAGGCAGAACAGCCTGCCTGGT
TCCATCAGCCCTGGCGCCCAGGCGCATCTGACTCGGCACCCCCTGCAGGCACCATGGCCCAGAGCCGGGTGCTGC
TGCTCCTGCTGCTGCTGCCGCCACAGCTGCACCTGGGACCTGTGCTTGCCGTGAGGGCCCCAGGATTTGGCCGAA
GTGGCGGCCACAGCCTGAGCCCCGAAGAGAACGAATTTGCGGAGGAGGAGCCGGTGCTGGTACTGAGCCCTGAGG
AGCCCGGGCCTGGCCCAGCCGCGGTCAGCTGCCCCCGAGACTGTGCCTGTTCCCAGGAGGGCGTCGTGGACTGTG
GCGGTATTGACCTGCGTGAGTTCCCGGGGGACCTGCCTGAGCACACCAACCACCTATCTCTGCAGAACAACCAGC
TGGAAAAGATCTACCCTGAGGAGCTCTCCCGGCTGCACCGGCTGGAGACACTGAACCTGCAAAACAACCGCCTGA
CTTCCCGAGGGCTCCCAGAGAAGGCGTTTGAGCATCTGACCAACCTCAATTACCTGTACTTGGCCAATAACAAGC
TGACCTTGGCACCCCGCTTCCTGCCAAACGCCCTGATCAGTGTGGACTTTGCTGCCAACTATCTCACCAAGATCT
ATGGGCTCACCTTTGGCCAGAAGCCAAACTTGAGGTCTGTGTACCTGCACAACAACAAGCTGGCAGACGCCGGGC
TGCCGGACAACATGTTCAACGGCTCCAGCAACGTCGAGGTCCTCATCCTGTCCAGCAACTTCCTGCGCCACGTGC
CCAAGCACCTGCCGCCTGCCCTGTACAAGCTGCACCTCAAGAACAACAAGCTGGAGAAGATCCCCCCGGGGGCCT
TCAGCGAGCTGAGCAGCCTGCGCGAGCTATACCTGCAGAACAACTACCTGACTGACGAGGGCCTGGACAACGAGA
CCTTCTGGAAGCTCTCCAGCCTGGAGTACCTGGATCTGTCCAGCAACAACCTGTCTCGGGTCCCAGCTGGGCTGC
CGCGCAGCCTGGTGCTGCTGCACTTGGAGAAGAACGCCATCCGGAGCGTGGACGCGAATGTGCTGACCCCCATCC
GCAGCCTGGAGTACCTGCTGCTGCACAGCAACCAGCTGCGGGAGCAGGGCATCCACCCACTGGCCTTCCAGGGCC
TCAAGCGGTTGCACACGGTGCACCTGTACAACAACGCGCTGGAGCGCGTGCCCAGTGGCCTGCCTCGCCGCGTGC
GCACCCTCATGATCCTGCACAACCAGATCACAGGCATTGGCCGCGAAGACTTTGCCACCACCTACTTCCTGGAGG
AGCTCAACCTCAGCTACAACCGCATCACCAGCCCACAGGTGCACCGCGACGCCTTCCGCAAGCTGCGCCTGCTGC
GCTCGCTGGACCTGTCGGGCAACCGGCTGCACACGCTGCCACCTGGGCTGCCTCGAAATGTCCATGTGCTGAAGG
TCAAGCGCAATGAGCTGGCTGCCTTGGCACGAGGGGCGCTGGCGGGCATGGCTCAGCTGCGTGAGCTGTACCTCA
CCAGCAACCGACTGCGCAGCCGAGCCCTGGGCCCCCGTGCCTGGGTGGACCTCGCCCATCTGCAGCTGCTGGACA
TCGCCGGGAATCAGCTCACAGAGATCCCCGAGGGGCTCCCCGAGTCACTTGAGTACCTGTACCTGCAGAACAACA
AGATTAGTGCGGTGCCCGCCAATGCCTTCGACTCCACGCCCAACCTCAAGGGGATCTTTCTCAGGTTTAACAAGC
TGGCTGTGGGCTCCGTGGTGGACAGTGCCTTCCGGAGGCTGAAGCACCTGCAGGTCTTGGACATTGAAGGCAACT
TAGAGTTTGGTGACATTTCCAAGGACCGTGGCCGCTTGGGGAAGGAAAAGGAGGAGGAGGAAGAGGAGGAGGAGG
AGGAAGAGGAAACAAGA**TAG**TGACAAGGTGATGCAGATGTGACCTAGGATGATGGACCGCCGGACTCTTTTCTGC
AGCACACGCCTGTGTGCTGTGAGCCCCCCACTCTGCCGTGCTCACACAGACACACCCAGCTGCACACATGAGGCA
TCCCACATGACACGGGCTGACACAGTCTCATATCCCCACCCCTTCCCACGGCGTGTCCCACGGCCAGACACATGC
ACACACATCACACCCTCAAACACCCAGCTCAGCCACACACAACTACCCTCCAAACCACCACAGTCTCTGTCACAC
CCCCACTACCGCTGCCACGCCCTCTGAATCATGCAGGGAAGGGTCTGCCCCTGCCCTGGCACACACAGGCACCCA
TTCCCTCCCCCTGCTGACATGTGTATGCGTATGCATACACACCACACACACACACATGCACAAGTCATGTGCGAA
CAGCCCTCCAAAGCCTATGCCACAGACAGCTCTTGCCCCAGCCAGAATCAGCCATAGCAGCTCGCCGTCTGCCCT
GTCCATCTGTCCGTCCGTTCCCTGGAGAAGACACAAGGGTATCCATGCTCTGTGGCCAGGTGCCTGCCACCCTCT
GGAACTCACAAAAGCTGGCTTTTATTCCTTTCCCATCCTATGGGGACAGGAGCCTTCAGGACTGCTGGCCTGGCC
TGGCCCACCCTGCTCCTCCAGGTGCTGGGCAGTCACTCTGCTAAGAGTCCCTCCCTGCCACGCCCTGGCAGGACA
CAGGCACTTTTCCAATGGGCAAGCCCAGTGGAGGCAGGATGGGAGAGCCCCCTGGGTGCTGCTGGGGCCTTGGGG
CAGGAGTGAAGCAGAGGTGATGGGGCTGGGCTGAGCCAGGGAGGAAGGACCCAGCTGCACCTAGGAGACACCTTT
GTTCTTCAGGCCTGTGGGGGAAGTTCCGGGTGCCTTTATTTTTTATTCTTTTCTAAGGAAAAAAATGATAAAAT
CTCAAAGCTGATTTTTCTTGTTATAGAAAAACTAATATAAAAGCATTATCCCTATCCCTGCAAAAAAAAAA

# FIGURE 370

MEGEEAEQPAWFHQPWRPGASDSAPPAGTMAQSRVLLLLLLLPPQLHLGPVLAVRAPGFGRSG
GHSLSPEENEFAEEEPVLVLSPEEPGPGPAAVSCPRDCACSQEGVVDCGGIDLREFPGDLPEH
TNHLSLQNNQLEKIYPEELSRLHRLETLNLQNNRLTSRGLPEKAFEHLTNLNYLYLANNKLTL
APRFLPNALISVDFAANYLTKIYGLTFGQKPNLRSVYLHNNKLADAGLPDNMFNGSSNVEVLI
LSSNFLRHVPKHLPPALYKLHLKNNKLEKIPPGAFSELSSLRELYLQNNYLTDEGLDNETFWK
LSSLEYLDLSSNNLSRVPAGLPRSLVLLHLEKNAIRSVDANVLTPIRSLEYLLLHSNQLREQG
IHPLAFQGLKRLHTVHLYNNALERVPSGLPRRVRTLMILHNQITGIGREDFATTYFLEELNLS
YNRITSPQVHRDAFRKLRLLRSLDLSGNRLHTLPPGLPRNVHVLKVKRNELAALARGALAGMA
QLRELYLTSNRLRSRALGPRAWVDLAHLQLLDIAGNQLTEIPEGLPESLEYLYLQNNKISAVP
ANAFDSTPNLKGIFLRFNKLAVGSVVDSAFRRLKHLQVLDIEGNLEFGDISKDRGRLGKEKEE
EEEEEEEEEETR

**Important features:**

**Signal sequence:**

amino acids 1-48

**N-glycosylation site.**

amino acids 243-247, 310-314, 328-332, 439-443

**Casein kinase II phosphorylation site.**

amino acids 68-72, 84-88, 246-250, 292-296, 317-321, 591-595

**N-myristoylation site.**

amino acids 19-25, 107-113, 213-219, 217-223, 236-242, 335-341, 477-483, 498-502, 539-545, 548-554

**Leucine zipper pattern.**

amino acids 116-138, 251-273, 258-280, 322-344, 464-486, 471-493, 535-557

# FIGURE 371

CACTTTCTCCCTCTCTTCCTTTACTTTCGAGAAACCGCGCTTCCGCTTCTGGTCGCAGAGACCTCGGAGACCGCG
CCGGGGAGACGGAGGTGCTGTGGGTGGGGGGACCTGTGGCTGCTCGTACCGCCCCCCACCCTCCTCTTCTGCAC
TGCCGTCCTCCGGAAGACCTTTTCCCCTGCTCTGTTTCCTTCACCGAGTCTGTGCATCGCCCCGGACCTGGCCGG
GAGGAGGCTTGGCCGGCGGGAGATGCTCTAGGGGCGGCGCGGGAGGAGCGGCCGGCGGGACGGAGGGCCCGGCAG
GAAG**ATG**GGCTCCCGTGGACAGGGACTCTTGCTGGCGTACTGCCTGCTCCTTGCCTTTGCCTCTGGCCTGGTCCT
GAGTCGTGTGCCCCATGTCCAGGGGGAACAGCAGGAGTGGGAGGGGACTGAGGAGCTGCCGTCGCCTCCGGACCA
TGCCGAGAGGGCTGAAGAACAACATGAAAAATACAGGCCCAGTCAGGACCAGGGGCTCCCTGCTTCCCGGTGCTT
GCGCTGCTGTGACCCCGGTACCTCCATGTACCCGGCGACCGCCGTGCCCCAGATCAACATCACTATCTTGAAAGG
GGAGAAGGGTGACCGCGGAGATCGAGGCCTCCAAGGGAAATATGGCAAAACAGGCTCAGCAGGGGCCAGGGGCCA
CACTGGACCCAAAGGGCAGAAGGGCTCCATGGGGGCCCCTGGGGAGCGGTGCAAGAGCCACTACGCCGCCTTTTC
GGTGGGCCGGAAGAAGCCCATGCACAGCAACCACTACTACCAGACGGTGATCTTCGACACGGAGTTCGTGAACCT
CTACGACCACTTCAACATGTTCACCGGCAAGTTCTACTGCTACGTGCCCGGCCTCTACTTCTTCAGCCTCAACGT
GCACACCTGGAACCAGAAGGAGACCTACCTGCACATCATGAAGAACGAGGAGGAGGTGGTGATCTTGTTCGCGCA
GGTGGGCGACCGCAGCATCATGCAAAGCCAGAGCCTGATGCTGGAGCTGCGAGAGCAGGACCAGGTGTGGGTACG
CCTCTACAAGGGCGAACGTGAGAACGCCATCTTCAGCGAGGAGCTGGACACCTACATCACCTTCAGTGGCTACCT
GGTCAAGCACGCCACCGAGCCC**TAG**CTGGCCGGCCACCTCCTTTCCTCTCGCCACCTTCCACCCCTGCGCTGTGC
TGACCCCACCGCCTCTTCCCCGATCCCTGGACTCCGACTCCCTGGCTTTGGCATTCAGTGAGACGCCCTGCACAC
ACAGAAAGCCAAAGCGATCGGTGCTCCCAGATCCCGCAGCCTCTGGAGAGAGCTGACGGCAGATGAAATCACCAG
GGCGGGGCACCCGCGAGAACCCTCTGGGACCTTCCGCGGCCCTCTCTGCACACATCCTCAAGTGACCCCGCACGG
CGAGACGCGGGTGGCGGCAGGGCGTCCCAGGGTGCGGCACCGCGGCTCCAGTCCTTGGAAATAATTAGGCAAATT
CTAAAGGTCTCAAAAGGAGCAAAGTAAACCGTGGAGGACAAAGAAAAGGGTTGTTATTTTTGTCTTTCCAGCCAG
CCTGCTGGCTCCCAAGAGAGAGGCCTTTTCAGTTGAGACTCTGCTTAAGAGAAGATCCAAAGTTAAAGCTCTGGG
GTCAGGGGAGGGGCCGGGGGCAGGAAACTACCTCTGGCTTAATTCTTTTAAGCCACGTAGGAACTTTCTTGAGGG
ATAGGTGGACCCTGACATCCCTGTGGCCTTGCCCAAGGGCTCTGCTGGTCTTTCTGAGTCACAGCTGCGAGGTGA
TGGGGGCTGGGGCCCCAGGCGTCAGCCTCCCAGAGGGACAGCTGAGCCCCCTGCCTTGGCTCCAGGTTGGTAGAA
GCAGCCGAAGGGCTCCTGACAGTGGCCAGGGACCCCTGGGTCCCCCAGGCCTGCAGATGTTTCTATGAGGGGCAG
AGCTCCTTGGTACATCCATGTGTGGCTCTGCTCCACCCCTGTGCCACCCCAGAGCCCTGGGGGGTGGTCTCCATG
CCTGCCACCCTGGCATCGGCTTTCTGTGCCGCCTCCCACACAAATCAGCCCCAGAAGGCCCCGGGGCCTTGGCTT
CTGTTTTTTATAAAACACCTCAAGCAGCACTGCAGTCTCCCATCTCCTCGTGGGCTAAGCATCACCGCTTCCACG
TGTGTTGTGTTGGTTGGCAGCAAGGCTGATCCAGACCCCTTCTGCCCCCACTGCCCTCATCCAGGCCTCTGACCA
GTAGCCTGAGAGGGGCTTTTTCTAGGCTTCAGAGCAGGGGAGAGCTGGAAGGGGCTAGAAAGCTCCCGCTTGTCT
GTTTCTCAGGCTCCTGTGAGCCTCAGTCCTGAGACCAGAGTCAAGAGGAAGTACACGTCCCAATCACCCGTGTCA
GGATTCACTCTCAGGAGCTGGGTGGCAGGAGAGGCAATAGCCCCTGTGGCAATTGCAGGACCAGCTGGAGCAGGG
TTGCGGTGTCTCCACGGTGCTCTCGCCCTGCCCATGGCCACCCCAGACTCTGATCTCCAGGAACCCCATAGCCCC
TCTCCACCTCACCCCATGTTGATGCCCAGGGTCACTCTTGCTACCCGCTGGGCCCCCAAACCCCCGCTGCCTCTC
TTCCTTCCCCCCATCCCCCACCTGGTTTTGACTAATCCTGCTTCCCTCTCTGGGCCTGGCTGCCGGGATCTGGGG
TCCCTAAGTCCCTCTCTTTAAAGAACTTCTGCGGGTCAGACTCTGAAGCCGAGTTGCTGTGGGCGTGCCCGGAAG
CAGAGCGCCACACTCGCTGCTTAAGCTCCCCCAGCTCTTTCCAGAAAACATTAAACTCAGAATTGTGTTTTCAA

# FIGURE 372

MGSRGQGLLLAYCLLLAFASGLVLSRVPHVQGEQQEWEGTEELPSPPDHAERAEEQHEKYRPS
QDQGLPASRCLRCCDPGTSMYPATAVPQINITILKGEKGDRGDRGLQGKYGKTGSAGARGHTG
PKGQKGSMGAPGERCKSHYAAFSVGRKKPMHSNHYYQTVIFDTEFVNLYDHFNMFTGKFYCYV
PGLYFFSLNVHTWNQKETYLHIMKNEEEVVILFAQVGDRSIMQSQSLMLELREQDQVWVRLYK
GERENAIFSEELDTYITFSGYLVKHATEP

**Important features:**

**Signal sequence.**

amino acids 1-25

**N-glycosylation site.**

amino acids 93-97

**N-myristoylation sites.**

amino acids 7-13, 21-27, 67-73, 117-123, 129-135

**Amidation site.**

amino acids 150-154

**Cell attachment sequence.**

amino acids 104-107

# FIGURE 373

CGGAGTGGTGCGCCAACGTGAGAGGAAACCCGTGCGCGGCTGCGCTTTCCTGTCCCCAAGCCG
TTCTAGACGCGGGAAAA**ATG**CTTTCTGAAAGCAGCTCCTTTTTGAAGGGTGTGATGCTTGGAA
GCATTTTCTGTGCTTTGATCACTATGCTAGGACACATTAGGATTGGTCATGGAAATAGAATGC
ACCACCATGAGCATCATCACCTACAAGCTCCTAACAAAGAAGATATCTTGAAAATTTCAGAGG
ATGAGCGCATGGAGCTCAGTAAGAGCTTTCGAGTATACTGTATTATCCTTGTAAAACCCAAAG
ATGTGAGTCTTTGGGCTGCAGTAAAGGAGACTTGGACCAAACACTGTGACAAAGCAGAGTTCT
TCAGTTCTGAAAATGTTAAAGTGTTTGAGTCAATTAATATGGACACAAATGACATGTGGTTAA
TGATGAGAAAAGCTTACAAATACGCCTTTGATAAGTATAGAGACCAATACAACTGGTTCTTCC
TTGCACGCCCCACTACGTTTGCTATCATTGAAAACCTAAAGTATTTTTTGTTAAAAAAGGATC
CATCACAGCCTTTCTATCTAGGCCACACTATAAAATCTGGAGACCTTGAATATGTGGGTATGG
AAGGAGGAATTGTCTTAAGTGTAGAATCAATGAAAAGACTTAACAGCCTTCTCAATATCCCAG
AAAAGTGTCCTGAACAGGGAGGGATGATTTGGAAGATATCTGAAGATAAACAGCTAGCAGTTT
GCCTGAAATATGCTGGAGTATTTGCAGAAAATGCAGAAGATGCTGATGGAAAAGATGTATTTA
ATACCAAATCTGTTGGGCTTTCTATTAAAGAGGCAATGACTTATCACCCCAACCAGGTAGTAG
AAGGCTGTTGTTCAGATATGGCTGTTACTTTTAATGGACTGACTCCAAATCAGATGCATGTGA
TGATGTATGGGGTATACCGCCTTAGGGCATTTGGGCATATTTTCAATGATGCATTGGTTTTCT
TACCTCCAAATGGTTCTGACAATGAC**TGA**GAAGTGGTAGAAAAGCGTGAATATGATCTTTGTA
TAGGACGTGTGTTGTCATTATTTGTAGTAGTAACTACATATCCAATACAGCTGTATGTTTCTT
TTTCTTTTCTAATTTGGTGGCACTGGTATAACCACACATTAAAGTCAGTAGTACATTTTTAAA
TGAGGGTGGTTTTTTTCTTTAAAACACATGAACATTGTAAATGTGTTGGAAAGAAGTGTTTTA
AGAATAATAATTTTGCAAATAAACTATTAATAAATATTATATGTGATAAATTCTAAATTATGA
ACATTAGAAATCTGTGGGGCACATATTTTTGCTGATTGGTTAAAAAATTTTAACAGGTCTTTA
GCGTTCTAAGATATGCAAATGATATCTCTAGTTGTGAATTTGTGATTAAAGTAAAACTTTTAG
CTGTGTGTTCCCTTTACTTCTAATACTGATTTATGTTCTAAGCCTCCCCAAGTTCCAATGGAT
TTGCCTTCTCAAAATGTACAACTAAGCAACTAAAGAAAATTAAAGTGAAAGTTGAAAAAT

# FIGURE 374

MLSESSSFLKGVMLGSIFCALITMLGHIRIGHGNRMHHHEHHHLQAPNKEDILKISEDERMELSKSFRVYCIILV
KPKDVSLWAAVKETWTKHCDKAEFFSSENVKVFESINMDTNDMWLMMRKAYKYAFDKYRDQYNWFFLARPTTFAI
IENLKYFLLKKDPSQPFYLGHTIKSGDLEYVGMEGGIVLSVESMKRLNSLLNIPEKCPEQGGMIWKISEDKQLAV
CLKYAGVFAENAEDADGKDVFNTKSVGLSIKEAMTYHPNQVVEGCCSDMAVTFNGLTPNQMHVMMYGVYRLRAFG
HIFNDALVFLPPNGSDND

**Important features:**

**Signal sequence:**

amino acids 1-33

**N-glycosylation site.**

amino acids 121-125, 342-346

**cAMP- and cGMP-dependent protein kinase phosphorylation site.**

amino acids 319-323, 464-468

**Casein kinase II phosphorylation site.**

amino acids 64-132, 150-154, 322-326, 331-335, 368-372, 385-389, 399-403, 409-413, 473-477, 729-733, 748-752

**Tyrosine kinase phosphorylation site.**

amino acids 736-743

**N-myristoylation site.**

amino acids 19-25, 23-29, 136-142, 397-403, 441-447, 544-550, 558-564, 651-657, 657-663, 672-672

**Prokaryotic membrane lipoprotein lipid attachment site.**

amino acids 14-25

**Cell attachment sequence.**

amino acids 247-250

# FIGURE 375

GTTGTGTCCTTCAGCAAAACAGTGGATTTAAATCTCCTTGCACAAGCTTGAGAGCAACACAAT
CTATCAGGAAAGAAAGAAAGAAAAAAACCGAACCTGACAAAAAAGAAGAAAAAGAAGAAGAAA
AAAAATC**ATG**AAAACCATCCAGCCAAAAATGCACAATTCTATCTCTTGGGCAATCTTCACGGG
GCTGGCTGCTCTGTGTCTCTTCCAAGGAGTGCCCGTGCGCAGCGGAGATGCCACCTTCCCCAA
AGCTATGGACAACGTGACGGTCCGGCAGGGGGAGAGCGCCACCCTCAGGTGCACTATTGACAA
CCGGGTCACCCGGGTGGCCTGGCTAAACCGCAGCACCATCCTCTATGCTGGGAATGACAAGTG
GTGCCTGGATCCTCGCGTGGTCCTTCTGAGCAACACCCAAACGCAGTACAGCATCGAGATCCA
GAACGTGGATGTGTATGACGAGGGCCCTTACACCTGCTCGGTGCAGACAGACAACCACCCAAA
GACCTCTAGGGTCCACCTCATTGTGCAAGTATCTCCCAAAATTGTAGAGATTTCTTCAGATAT
CTCCATTAATGAAGGGAACAATATTAGCCTCACCTGCATAGCAACTGGTAGACCAGAGCCTAC
GGTTACTTGGAGACACATCTCTCCCAAAGCGGTTGGCTTTGTGAGTGAAGACGAATACTTGGA
AATTCAGGGCATCACCCGGGAGCAGTCAGGGGACTACGAGTGCAGTGCCTCCAATGACGTGGC
CGCGCCCGTGGTACGGAGAGTAAAGGTCACCGTGAACTATCCACCATACATTTCAGAAGCCAA
GGGTACAGGTGTCCCCGTGGGACAAAAGGGGACACTGCAGTGTGAAGCCTCAGCAGTCCCCTC
AGCAGAATTCCAGTGGTACAAGGATGACAAAAGACTGATTGAAGGAAAGAAAGGGGTGAAAGT
GGAAAACAGACCTTTCCTCTCAAAACTCATCTTCTTCAATGTCTCTGAACATGACTATGGGAA
CTACACTTGCGTGGCCTCCAACAAGCTGGGCCACACCAATGCCAGCATCATGCTATTTGGTCC
AGGCGCCGTCAGCGAGGTGAGCAACGGCACGTCGAGGAGGGCAGGCTGCGTCTGGCTGCTGCC
TCTTCTGGTCTTGCACCTGCTTCTCAAATTT**TGA**TGTGAGTGCCACTTCCCCACCCGGGAAAG
GCTGCCGCCACCACCACCACCAACACAACAGCAATGGCAACACCGACAGCAACCAATCAGATA
TATACAAATGAAATTAGAAGAAACACAGCCTCATGGGACAGAAATTTGAGGGAGGGGAACAAA
GAATACTTTGGGGGGAAAAGAGTTTTAAAAAAGAAATTGAAAATTGCCTTGCAGATATTTAGG
TACAATGGAGTTTTCTTTTCCCAAACGGGAAGAACACAGCACACCCGGCTTGGACCCACTGCA
AGCTGCATCGTGCAACCTCTTTGGTGCCAGTGTGGGCAAGGGCTCAGCCTCTCTGCCCACAGA
GTGCCCCCACGTGGAACATTCTGGAGCTGGCCATCCCAAATTCAATCAGTCCATAGAGACGAA
CAGAATGAGACCTTCCGGCCCAAGCGTGGCGCTGCGGGCACTTTGGTAGACTGTGCCACCACG
GCGTGTGTTGTGAAACGTGAAATAAAAAGAGCAAAAAAAAA

# FIGURE 376

MKTIQPKMHNSISWAIFTGLAALCLFQGVPVRSGDATFPKAMDNVTVRQGESATLRCTIDNRV
TRVAWLNRSTILYAGNDKWCLDPRVVLLSNTQTQYSIEIQNVDVYDEGPYTCSVQTDNHPKTS
RVHLIVQVSPKIVEISSDISINEGNNISLTCIATGRPEPTVTWRHISPKAVGFVSEDEYLEIQ
GITREQSGDYECSASNDVAAPVVRRVKVTVNYPPYISEAKGTGVPVGQKGTLQCEASAVPSAE
FQWYKDDKRLIEGKKGVKVENRPFLSKLIFFNVSEHDYGNYTCVASNKLGHTNASIMLFGPGA
VSEVSNGTSRRAGCVWLLPLLVLHLLLKF

**Important features:**

**Signal peptide:**

amino acids 1-28

# FIGURE 377

CTTCTTTGAAAAGGATTATCACCTGATCAGGTTCTCTCTGCATTTGCCCCTTTAGATTGTGAA
**ATG**TGGCTCAAGGTCTTCACAACTTTCCTTTCCTTTGCAACAGGTGCTTGCTCGGGGCTGAAG
GTGACAGTGCCATCACACACTGTCCATGGCGTCAGAGGTCAGGCCCTCTACCTACCCGTCCAC
TATGGCTTCCACACTCCAGCATCAGACATCCAGATCATATGGCTATTTGAGAGACCCCACACA
ATGCCCAAATACTTACTGGGCTCTGTGAATAAGTCTGTGGTTCCTGACTTGGAATACCAACAC
AAGTTCACCATGATGCCACCCAATGCATCTCTGCTTATCAACCCACTGCAGTTCCCTGATGAA
GGCAATTACATCGTGAAGGTCAACATTCAGGGAAATGGAACTCTATCTGCCAGTCAGAAGATA
CAAGTCACGGTTGATGATCCTGTCACAAAGCCAGTGGTGCAGATTCATCCTCCCTCTGGGGCT
GTGGAGTATGTGGGGAACATGACCCTGACATGCCATGTGGAAGGGGGCACTCGGCTAGCTTAC
CAATGGCTAAAAAATGGGAGACCTGTCCACACCAGCTCCACCTACTCCTTTTCTCCCCAAAAC
AATACCCTTCATATTGCTCCAGTAACCAAGGAAGACATTGGGAATTACAGCTGCCTGGTGAGG
AACCCTGTCAGTGAAATGGAAAGTGATATCATTATGCCCATCATATATTATGGACCTTATGGA
CTTCAAGTGAATTCTGATAAAGGGCTAAAAGTAGGGGAAGTGTTTACTGTTGACCTTGGAGAG
GCCATCCTATTTGATTGTTCTGCTGATTCTCATCCCCCCAACACCTACTCCTGGATTAGGAGG
ACTGACAATACTACATATATCATTAAGCATGGGCCTCGCTTAGAAGTTGCATCTGAGAAAGTA
GCCCAGAAGACAATGGACTATGTGTGCTGTGCTTACAACAACATAACCGGCAGGCAAGATGAA
ACTCATTTCACAGTTATCATCACTTCCGTAGGACTGGAGAAGCTTGCACAGAAAGGAAAATCA
TTGTCACCTTTAGCAAGTATAACTGGAATATCACTATTTTTGATTATATCCATGTGTCTTCTC
TTCCTATGGAAAAAATATCAACCCTACAAAGTTATAAAACAGAAACTAGAAGGCAGGCCAGAA
ACAGAATACAGGAAAGCTCAAACATTTTCAGGCCATGAAGATGCTCTGGATGACTTCGGAATA
TATGAATTTGTTGCTTTTCCAGATGTTTCTGGTGTTTCCAGGATTCCAAGCAGGTCTGTTCCA
GCCTCTGATTGTGTATCGGGGCAAGATTTGCACAGTACAGTGTATGAAGTTATTCAGCACATC
CCTGCCCAGCAGCAAGACCATCCAGAG**TGA**ACTTTCATGGGCTAAACAGTACATTCGAGTGAA
ATTCTGAAGAAACATTTTAAGGAAAAACAGTGGAAAAGTATATTAATCTGGAATCAGTGAAGA
AACCAGGACCAACACCTCTTACTCATTATTCCTTTACATGCAGAATAGAGGCATTTATGCAAA
TTGAACTGCAGGTTTTTCAGCATATACACAATGTCTTGTGCAACAGAAAAACATGTTGGGGAA
ATATTCCTCAGTGGAGAGTCGTTCTCATGCTGACGGGGAGAACGAAAGTGACAGGGGTTTCCT
CATAAGTTTTGTATGAAATATCTCTACAAACCTCAATTAGTTCTACTCTACACTTTCACTATC
ATCAACACTGAGACTATCCTGTCTCACCTACAAATGTGGAAACTTTACATTGTTCGATTTTTC
AGCAGACTTTGTTTTATTAAATTTTTATTAGTGTTAAGAATGCTAAATTTATGTTTCAATTTT
ATTTCCAAATTTCTATCTTGTTATTTGTACAACAAAGTAATAAGGATGGTTGTCACAAAAACA
AAACTATGCCTTCTCTTTTTTTTCAATCACCAGTAGTATTTTTGAGAAGACTTGTGAACACTT
AAGGAAATGACTATTAAAGTCTTATTTTTATTTTTTTCAAGGAAAGATGGATTCAAATAAATT
ATTCTGTTTTTGCTTTTAAAAAAAAAAAAAA

# FIGURE 378

MWLKVFTTFLSFATGACSGLKVTVPSHTVHGVRGQALYLPVHYGFHTPASDIQIIWLFERPHTMPKYLLGSVNKS
VVPDLEYQHKFTMMPPNASLLINPLQFPDEGNYIVKVNIQGNGTLSASQKIQVTVDDPVTKPVVQIHPPSGAVEY
VGNMTLTCHVEGGTRLAYQWLKNGRPVHTSSTYSFSPQNNTLHIAPVTKEDIGNYSCLVRNPVSEMESDIIMPII
YYGPYGLQVNSDKGLKVGEVFTVDLGEAILFDCSADSHPPNTYSWIRRTDNTTYIIKHGPRLEVASEKVAQKTMD
YVCCAYNNITGRQDETHFTVIITSVGLEKLAQKGKSLSPLASITGISLFLIISMCLLFLWKKYQPYKVIKQKLEG
RPETEYRKAQTFSGHEDALDDFGIYEFVAFPDVSGVSRIPSRSVPASDCVSGQDLHSTVYEVIQHIPAQQQDHPE

**Important features:**

**Signal sequence:**

amino acids 1-18

**Transmembrane domain:**

amino acids 341-359

**N-glycosylation site.**

amino acids 73-77, 92-96, 117-121, 153-157, 189-193, 204-208, 276-280, 308-312

**Casein kinase II phosphorylation site.**

amino acids 129-133, 198-202, 214-218, 388-392, 426-430, 433-437

**Tyrosine kinase phosphorylation site.**

amino acids 272-280

**N-myristoylation site.**

amino acids 15-21, 19-25, 118-124, 163-167, 203-209, 231-237, 239-245

**Prokaryotic membrane lipoprotein lipid attachment site.**

amino acids 7-18

# FIGURE 379

ATAGTAGAAGAATGTCTCTGAAATTACTGGATGAGTTTCAGTCATACTTTCACATGGGCACAA
TTTCACATTCAAGCTCCTTATCCTAGGCTAATTTTATATTATGTTAAATCACTTGTTTTTGTT
CTCACGGCTTCCTGCCTGCTATAGGCATAATTACGAGGAAGCAGAACTTCTCCAGAAGCAAGC
GCACATGCGTTCCAAAATAAGAGCAAATTCGCTCTAAACACAGGAAAAGACCTGAAGCTTTAA
TTAAGGGGTTACATCCAACCCCAGAGCGCTTTTGTGGGCACTGATTGCTCCAGCTTCTGCGTC
ACTGCGCGAGGGAAGAGGGAAGAGGATCCAGGCGTTAGAC**ATG**TATAGACACAAAAACAGCTG
GAGATTGGGCTTAAAATACCCACCAAGCTCCAAAGAAGAGACCCAAGTCCCCAAAACATTGAT
TTCAGGGCTGCCAGGAAGGAAGAGCAGCAGCAGGGTGGGAGAGAAGCTCCAGTCAGCCCACAA
GATGCCATTGTCCCCCGGCCTCCTGCTGCTGCTGCTCTCCGGGGCCACGGCCACCGCTGCCCT
GCCCCTGGAGGGTGGCCCCACCGGCCGAGACAGCGAGCATATGCAGGAAGCGGCAGGAATAAG
GAAAAGCAGCCTCCTGACTTTCCTCGCTTGGTGGTTTGAGTGGACCTCCCAGGCCAGTGCCGG
GCCCCTCATAGGAGAGGAAGCTCGGGAGGTGGCCAGGCGGCAGGAAGGCGCACCCCCCCAGCA
ATCCGCGCGCCGGGACAGAATGCCCTGCAGGAACTTCTTCTGGAAGACCTTCTCCTCCTGCAA
A**TAG**

# FIGURE 380

MYRHKNSWRLGLKYPPSSKEETQVPKTLISGLPGRKSSSRVGEKLQSAHKMPLSPGLLLLLLS
GATATAALPLEGGPTGRDSEHMQEAAGIRKSSLLTFLAWWFEWTSQASAGPLIGEEAREVARR
QEGAPPQQSARRDRMPCRNFFWKTFSSCK

**Important features:**

**Transmembrane domain:**

amino acids 51-69

**cAMP- and cGMP-dependent protein kinase phosphorylation sites.**

amino acids 35-39, 92-96

**N-myristoylation sites.**

amino acids 64-70, 75-81, 90-96

**Amidation site.**

amino acids 33-37

# FIGURE 381

GGCGCCGGTGCACCGGGCGGGCTGAGCGCCTCCTGCGGCCCGGCCTGCGCGCCCCGGCCCGCC
GCGCCGCCCACGCCCCAACCCCGGCCCGCGCCCCCTAGCCCCCGCCCGGGCCCGCGCCCGCGC
CCGCGCCCAGGTGAGCGCTCCGCCCGCCGCGAGGCCCCGCCCCGGCCCGCCCCCGCCCCGCCC
CGGCCGGCGGGGGAACCGGGCGGATTCCTCGCGCGTCAAACCACCTGATCCCATAAAACATTC
ATCCTCCCGGCGGCCCGCGCTGCGAGCGCCCCGCCAGTCCGCGCCGCCGCCGCCCTCGCCCTG
TGCGCCCTGCGCGCCCTGCGCACCCGCGGCCCGAGCCCAGCCAGAGCCGGGCGGAGCGGAGCG
CGCCGAGCCTCGTCCCGCGGCCGGGCCGGGGCCGGGCCGTAGCGGCGGCGCCTGGATGCGGAC
CCGGCCGCGGGGAGACGGGCGCCCGCCCCGAAACGACTTTCAGTCCCCGACGCGCCCCGCCCA
ACCCCTACG**ATG**AAGAGGGCGTCCGCTGGAGGGAGCCGGCTGCTGGCATGGGTGCTGTGGCTG
CAGGCCTGGCAGGTGGCAGCCCCATGCCCAGGTGCCTGCGTATGCTACAATGAGCCCAAGGTG
ACGACAAGCTGCCCCCAGCAGGGCCTGCAGGCTGTGCCCGTGGGCATCCCTGCTGCCAGCCAG
CGCATCTTCCTGCACGGCAACCGCATCTCGCATGTGCCAGCTGCCAGCTTCCGTGCCTGCCGC
AACCTCACCATCCTGTGGCTGCACTCGAATGTGCTGGCCCGAATTGATGCGGCTGCCTTCACT
GGCCTGGCCCTCCTGGAGCAGCTGGACCTCAGCGATAATGCACAGCTCCGGTCTGTGGACCCT
GCCACATTCCACGGCCTGGGCCGCCTACACACGCTGCACCTGGACCGCTGCGGCCTGCAGGAG
CTGGGCCCGGGGCTGTTCCGCGGCCTGGCTGCCCTGCAGTACCTCTACCTGCAGGACAACGCG
CTGCAGGCACTGCCTGATGACACCTTCCGCGACCTGGGCAACCTCACACACCTCTTCCTGCAC
GGCAACCGCATCTCCAGCGTGCCCGAGCGCGCCTTCCGTGGGCTGCACAGCCTCGACCGTCTC
CTACTGCACCAGAACCGCGTGGCCCATGTGCACCCGCATGCCTTCCGTGACCTTGGCCGCCTC
ATGACACTCTATCTGTTTGCCAACAATCTATCAGCGCTGCCCACTGAGGCCCTGGCCCCCCTG
CGTGCCCTGCAGTACCTGAGGCTCAACGACAACCCCTGGGTGTGTGACTGCCGGGCACGCCCA
CTCTGGGCCTGGCTGCAGAAGTTCCGCGGCTCCTCCTCCGAGGTGCCCTGCAGCCTCCCGCAA
CGCCTGGCTGGCCGTGACCTCAAACGCCTAGCTGCCAATGACCTGCAGGGCTGCGCTGTGGCC
ACCGGCCCTTACCATCCCATCTGGACCGGCAGGGCCACCGATGAGGAGCCGCTGGGGCTTCCC
AAGTGCTGCCAGCCAGATGCCGCTGACAAGGCCTCAGTACTGGAGCCTGGAAGACCAGCTTCG
GCAGGCAATGCGCTGAAGGGACGCGTGCCGCCCGGTGACAGCCCGCCGGGCAACGGCTCTGGC
CCACGGCACATCAATGACTCACCCTTTGGGACTCTGCCTGGCTCTGCTGAGCCCCCGCTCACT
GCAGTGCGGCCCGAGGGCTCCGAGCCACCAGGGTTCCCCACCTCGGGCCCTCGCCGGAGGCCA
GGCTGTTCACGCAAGAACCGCACCCGCAGCCACTGCCGTCTGGGCCAGGCAGGCAGCGGGGGT
GGCGGGACTGGTGACTCAGAAGGCTCAGGTGCCCTACCCAGCCTCACCTGCAGCCTCACCCCC
CTGGGCCTGGCGCTGGTGCTGTGGACAGTGCTTGGGCCCTGC**TGA**CCCCCAGCGGACACAAGA
GCGTGCTCAGCAGCCAGGTGTGTGTACATACGGGGTCTCTCTCCACGCCGCCAAGCCAGCCGG
GCGGCCGACCCGTGGGGCAGGCCAGGCCAGGTCCTCCCTGATGGACGCCTGCCGCCCGCCACC
CCCATCTCCACCCCATCATGTTTACAGGGTTCGGCGGCAGCGTTTGTTCCAGAACGCCGCCTC
CCACCCAGATCGCGGTATATAGAGATATGCATTTTATTTACTTGTGTAAAAATATCGGACGA
CGTGGAATAAAGAGCTCTTTTCTTAAAAAAA

# FIGURE 382

MKRASAGGSRLLAWVLWLQAWQVAAPCPGACVCYNEPKVTTSCPQQGLQAVPVGIPAASQRIF
LHGNRISHVPAASFRACRNLTILWLHSNVLARIDAAAFTGLALLEQLDLSDNAQLRSVDPATF
HGLGRLHTLHLDRCGLQELGPGLFRGLAALQYLYLQDNALQALPDDTFRDLGNLTHLFLHGNR
ISSVPERAFRGLHSLDRLLLHQNRVAHVHPHAFRDLGRLMTLYLFANNLSALPTEALAPLRAL
QYLRLNDNPWVCDCRARPLWAWLQKFRGSSSEVPCSLPQRLAGRDLKRLAANDLQGCAVATGP
YHPIWTGRATDEEPLGLPKCCQPDAADKASVLEPGRPASAGNALKGRVPPGDSPPGNGSGPRH
INDSPFGTLPGSAEPPLTAVRPEGSEPPGFPTSGPRRRPGCSRKNRTRSHCRLGQAGSGGGT
GDSEGSGALPSLTCSLTPLGLALVLWTVLGPC

**Important features:**

**Signal peptide:**

amino acids 1-26

**Leucine zipper pattern.**

amino acids 135-156

**Glycosaminoglycan attachment site.**

amino acids 436-439

**N-glycosylation site.**

amino acids 82-85, 179-183, 237-240, 372-375 and 423-426

**VWFC domain**

amino acids 411-425

# FIGURE 383

TTCGTGACCCTTGAGAAAAGAGTTGGTGGTAAATGTGCCACGTCTTCTAAGAAGGGGGAGTCCTGAACTTGTCTG
AAGCCCTTGTCCGTAAGCCTTGAACTACGTTCTTAAATCTATGAAGTCGAGGGACCTTTCGCTGCTTTTGTAGGG
ACTTCTTTCCTTGCTTCAGCAAC**ATG**AGGCTTTTCTTGTGGAACGCGGTCTTGACTCTGTTCGTCACTTCTTTGA
TTGGGGCTTTGATCCCTGAACCAGAAGTGAAAATTGAAGTTCTCCAGAAGCCATTCATCTGCCATCGCAAGACCA
AAGGAGGGGATTTGATGTTGGTCCACTATGAAGGCTACTTAGAAAAGGACGGCTCCTTATTTCACTCCACTCACA
AACATAACAATGGTCAGCCCATTTGGTTTACCCTGGGCATCCTGGAGGCTCTCAAAGGTTGGGACCAGGGCTTGA
AAGGAATGTGTGTAGGAGAGAAGAGAAAGCTCATCATTCCTCCTGCTCTGGGCTATGGAAAAGAAGGAAAAGGTA
AAATTCCCCCAGAAAGTACACTGATATTTAATATTGATCTCCTGGAGATTCGAAATGGACCAAGATCCCATGAAT
CATTCCAAGAAATGGATCTTAATGATGACTGGAAACTCTCTAAAGATGAGGTTAAAGCATATTTAAAGAAGGAGT
TTGAAAAACATGGTGCGGTGGTGAATGAAAGTCATCATGATGCTTTGGTGGAGGATATTTTTGATAAAGAAGATG
AAGACAAAGATGGGTTTATATCTGCCAGAGAATTTACATATAAACACGATGAGTTA**TAG**AGATACATCTACCCTT
TTAATATAGCACTCATCTTTCAAGAGAGGGCAGTCATCTTTAAAGAACATTTTATTTTTATACAATGTTCTTTCT
TGCTTTGTTTTTTATTTTTATATATTTTTTCTGACTCCTATTTAAAGAACCCCTTAGGTTTCTAAGTACCCATTT
CTTTCTGATAAGTTATTGGGAAGAAAAAGCTAATTGGTCTTTGAATAGAAGACTTCTGGACAATTTTTCACTTTC
ACAGATATGAAGCTTTGTTTTACTTTCTCACTTATAAATTTAAAATGTTGCAACTGGGAATATACCACGACATGA
GACCAGGTTATAGCACAAATTAGCACCCTATATTTCTGCTTCCCTCTATTTTCTCCAAGTTAGAGGTCAACATTT
GAAAAGCCTTTTGCAATAGCCCAAGGCTTGCTATTTTCATGTTATAATGAAATAGTTTATGTGTAACTGGCTCTG
AGTCTCTGCTTGAGGACCAGAGGAAAATGGTTGTTGGACCTGACTTGTTAATGGCTACTGCTTTACTAAGGAGAT
GTGCAATGCTGAAGTTAGAAACAAGGTTAATAGCCAGGCATGGTGGCTCATGCCTGTAATCCCAGCACTTTGGGA
GGCTGAGGCGGGCGGATCACCTGAGGTTGGGAGTTCGAGACCAGCCTGACCAACACGGAGAAACCCTATCTCTAC
TAAAAATACAAAGTAGCCCGGCGTGGTGATGCGTGCCTGTAATCCCAGCTACCCAGGAAGGCTGAGGCGGCAGAA
TCACTTGAACCCGAGGCCGAGGTTGCGGTAAGCCGAGATCACCTNCAGCCTGGACACTCTGTCTCGAAAAAAAGAA
AAGAACACGGTTAATACCATATNAATATGTATGCATTGAGACATGCTACCTAGGACTTAAGCTGATGAAGCTTGG
CTCCTAGTGATTGGTGGCCTATTATGATAAATAGGACAAATCATTTATGTGTGAGTTTCTTTGTAATAAAATGTA
TCAATATGTTATAGATGAGGTAGAAAGTTATATTTATATTCAATATTTACTTCTTAAGGCTAGCGGAATATCCTT
CCTGGTTCTTTAATGGGTAGTCTATAGTATATTATACTACAATAACATTGTATCATAAGATAAAGTAGTAAACCA
GTCTACATTTTCCCATTTCTGTCTCATCAAAAACTGAAGTTAGCTGGGTGTGGTGGCTCATGCCTGTAATCCCAG
CACTTTGGGGGCCAAGGAGGGTGGATCACTTGAGATCAGGAGTTCAAGACCAGCCTGGCCAACATGGTGAAACCT
TGTCTCTACTAAAAATACAAAAATTAGCCAGGCGTGGTGGTGCACACCTGTAGTCCCAGCTACTCGGGAGGCTGA
GACAGGAGATTTGCTTGAACCCGGGAGGCGGAGGTTGCAGTGAGCCAAGATTGTGCCACTGCACTCCAGCCTGGG
TGACAGAGCAAGACTCCATCTCAAAAAAAAAAAAAAAGAAGCAGACCTACAGCAGCTACTATTGAATAAATACCTA
TCCTGGATTTT

# FIGURE 384

MRLFLWNAVLTLFVTSLIGALIPEPEVKIEVLQKPFICHRKTKGGDLMLVHYEGYLEKDGSLF
HSTHKHNNGQPIWFTLGILEALKGWDQGLKGMCVGEKRKLIIPPALGYGKEGKGKIPPESTLI
FNIDLLEIRNGPRSHESFQEMDLNDDWKLSKDEVKAYLKKEFEKHGAVVNESHHDALVEDIFD
KEDEDKDGFISAREFTYKHDEL

**Important features:**

**Signal peptide:**

amino acids 1-20

**N-glycosylation site.**

amino acids 176-179

**Casein kinase II phosphorylation site.**

amino acids 143-146, 156-159, 178-181 and 200-203

**Endoplasmic reticulum targeting sequence.**

amino acids 208-211

**FKBP-type peptidyl-prolyl cis-trans isomerase**

amino acids 78-114 and 118-131

**EF-hand calcium-binding domain.**

amino acids 191-203, 184-203 and 140-159

**S-100/ICaBP type calcium binding domain**

amino acids 183-203

# FIGURE 385

CTCCCACGGTGTCCAGCGCCCAGA**ATG**CGGCTTCTGGTCCTGCTATGGGGTTGCCTGCTGCTC
CCAGGTTATGAAGCCCTGGAGGGCCCAGAGGAAATCAGCGGGTTCGAAGGGGACACTGTGTCC
CTGCAGTGCACCTACAGGGAAGAGCTGAGGGACCACCGGAAGTACTGGTGCAGGAAGGGTGGG
ATCCTCTTCTCTCGCTGCTCTGGCACCATCTATGCAGAAGAAGAAGGCCAGGAGACAATGAAG
GGCAGGGTGTCCATCCGTGACAGCCGCCAGGAGCTCTCGCTCATTGTGACCCTGTGGAACCTC
ACCCTGCAAGACGCTGGGGAGTACTGGTGTGGGGTCGAAAAACGGGGCCCCGATGAGTCTTTA
CTGATCTCTCTGTTCGTCTTTCCAGGACCCTGCTGTCCTCCCTCCCCTTCTCCCACCTTCCAG
CCTCTGGCTACAACACGCCTGCAGCCCAAGGCAAAAGCTCAGCAAACCCAGCCCCCAGGATTG
ACTTCTCCTGGGCTCTACCCGGCAGCCACCACAGCCAAGCAGGGGAAGACAGGGGCTGAGGCC
CCTCCATTGCCAGGGACTTCCCAGTACGGGCACGAAAGGACTTCTCAGTACACAGGAACCTCT
CCTCACCCAGCGACCTCTCCTCCTGCAGGGAGCTCCCGCCCCCCCATGCAGCTGGACTCCACC
TCAGCAGAGGACACCAGTCCAGCTCTCAGCAGTGGCAGCTCTAAGCCCAGGGTGTCCATCCCG
ATGGTCCGCATACTGGCCCCAGTCCTGGTGCTGCTGAGCCTTCTGTCAGCCGCAGGCCTGATC
GCCTTCTGCAGCCACCTGCTCCTGTGGAGAAAGGAAGCTCAACAGGCCACGGAGACACAGAGG
AACGAGAAGTTCTGGCTCTCACGCTTGACTGCGGAGGAAAAGGAAGCCCCTTCCCAGGCCCCT
GAGGGGGACGTGATCTCGATGCCTCCCCTCCACACATCTGAGGAGGAGCTGGGCTTCTCGAAG
TTTGTCTCAGCG**TAG**GGCAGGAGGCCCTCCTGGCCAGGCCAGCAGTGAAGCAGTATGGCTGGC
TGGATCAGCACCGATTCCCGAAAGCTTTCCACCTCAGCCTCAGAGTCCAGCTGCCCGGACTCC
AGGGCTCTCCCCACCCTCCCCAGGCTCTCCTCTTGCATGTTCCAGCCTGACCTAGAAGCGTTT
GTCAGCCCTGGAGCCCAGAGCGGTGGCCTTGCTCTTCCGGCTGGAGACTGGGACATCCCTGAT
AGGTTCACATCCCTGGGCAGAGTACCAGGCTGCTGACCCTCAGCAGGGCCAGACAAGGCTCAG
TGGATCTGGTCTGAGTTTCAATCTGCCAGGAACTCCTGGGCCTCATGCCCAGTGTCGGACCCT
GCCTTCCTCCCACTCCAGACCCCACCTTGTCTTCCCTCCCTGGCGTCCTCAGACTTAGTCCCA
CGGTCTCCTGCATCAGCTGGTGATGAAGAGGAGCATGCTGGGGTGAGACTGGGATTCTGGCTT
CTCTTTGAACCACCTGCATCCAGCCCTTCAGGAAGCCTGTGAAAAACGTGATTCCTGGCCCCA
CCAAGACCCACCAAAACCATCTCTGGGCTTGGTGCAGGACTCTGAATTCTAACAATGCCCAGT
GACTGTCGCACTTGAGTTTGAGGGCCAGTGGGCCTGATGAACGCTCACACCCCTTCAGCTTAG
AGTCTGCATTTGGGCTGTGACGTCTCCACCTGCCCCAATAGATCTGCTCTGTCTGCGACACCA
GATCCACGTGGGGACTCCCCTGAGGCCTGCTAAGTCCAGGCCTTGGTCAGGTCAGGTGCACAT
TGCAGGATAAGCCCAGGACCGGCACAGAAGTGGTTGCCTTTNCCATTTGCCCTCCCTGGNCCA
TGCCTTCTTGCCTTTGGAAAAAATGATGAAGAAAACCTTGGCTCCTTCCTTGTCTGGAAAGGG
TTACTTGCCTATGGGTTCTGGTGGCTAGAGAGAAAAGTAGAAAACCAGAGTGCACGTAGGTGT
CTAACACAGAGGAGAGTAGGAACAGGGCGGATACCTGAAGGTGACTCCGAGTCCAGCCCCCTG
GAGAAGGGGTCGGGGGTGGTGGTAAAGTAGCACAACTACTATTTTTTTCTTTTTCCATTATT
ATTGTTTTTAAGACAGAATCTCGTGCTGCTGCCCAGGCTGGAGTGCAGTGGCACGATCTGCA
AACTCCGCCTCCTGGGTTCAAGTGATTCTTCTGCCTCAGCCTCCCGAGTAGCTGGGATTACAG
GCACGCACCACCACACCTGGCTAATTTTTGTACTTTTAGTAGAGATGGGGTTTCACCATGTTG
GCCAGGCTGGTCTTGAACTCCTGACCTCAAATGAGCCTCCTGCTTCAGTCTCCCAAATTGCCG
GGATTACAGGCATGAGCCACTGTGTCTGGCCCTATTTCCTTTAAAAAGTGAAATTAAGAGTTG
TTCAGTATGCAAAACTTGGAAAGATGGAGGAGAAAAAGAAAAGGAAGAAAAAAAATGTCACCCA
TAGTCTCACCAGAGACTATCATTATTTCGTTTTGTTGTACTTCCTTCCACTCTTTTCTTCTTC
ACATAATTTGCCGGTGTTCTTTTTACAGAGCAATTATCTTGTATATACAACTTTGTATCCTGC
CTTTTCCACCTTATCGTTCCATCACTTTATTCCAGCACTTCTCTGTGTTTTACAGACCTTTTT
ATAAATAAAATGTTCATCAGCTGCATAAAAAAAAAAAAAA

# FIGURE 386

MRLLVLLWGCLLLPGYEALEGPEEISGFEGDTVSLQCTYREELRDHRKYWCRKGGILFSRCSG
TIYAEEEGQETMKGRVSIRDSRQELSLIVTLWNLTLQDAGEYWCGVEKRGPDESLLISLFVFP
GPCCPPSPSPTFQPLATTRLQPKAKAQQTQPPGLTSPGLYPAATTAKQGKTGAEAPPLPGTSQ
YGHERTSQYTGTSPHPATSPPAGSSRPPMQLDSTSAEDTSPALSSGSSKPRVSIPMVRILAPV
LVLLSLLSAAGLIAFCSHLLLWRKEAQQATETQRNEKFWLSRLTAEEKEAPSQAPEGDVISMP
PLHTSEEELGFSKFVSA

**Important features:**

**Signal peptide:**

amino acids 1-17

**Transmembrane domain:**

amino acids 248-269

**N-glycosylation site.**

amino acids 96-99

**Fibrinogen beta and gamma chains C-terminal domain.**

amino acids 104-113

**Ig like V-type domain:**

amino acids 13-128

# FIGURE 387

GCGCCGGGAGCCCATCTGCCCCCAGGGGCACGGGGCGCGGGGCCGGCTCCCGCCCGGCACATG
GCTGCAGCCACCTCGCGCGCACCCCGAGGCGCCGCGCCCAGCTCGCCCGAGGTCCGTCGGAGG
CGCCCGGCCGCCCCGGAGCCAAGCAGCAACTGAGCGGGGAAGCGCCCGCGTCCGGGGATCGGG
**ATG**TCCCTCCTCCTTCTCCTCTTGCTAGTTTCCTACTATGTTGGAACCTTGGGGACTCACACT
GAGATCAAGAGAGTGGCAGAGGAAAAGGTCACTTTGCCCTGCCACCATCAACTGGGGCTTCCA
GAAAAAGACACTCTGGATATTGAATGGCTGCTCACCGATAATGAAGGGAACCAAAAAGTGGTG
ATCACTTACTCCAGTCGTCATGTCTACAATAACTTGACTGAGGAACAGAAGGGCCGAGTGGCC
TTTGCTTCCAATTTCCTGGCAGGAGATGCCTCCTTGCAGATTGAACCTCTGAAGCCCAGTGAT
GAGGGCCGGTACACCTGTAAGGTTAAGAATTCAGGGCGCTACGTGTGGAGCCATGTCATCTTA
AAAGTCTTAGTGAGACCATCCAAGCCCAAGTGTGAGTTGGAAGGAGAGCTGACAGAAGGAAGT
GACCTGACTTTGCAGTGTGAGTCATCCTCTGGCACAGAGCCCATTGTGTATTACTGGCAGCGA
ATCCGAGAGAAAGAGGGAGAGGATGAACGTCTGCCTCCCAAATCTAGGATTGACTACAACCAC
CCTGGACGAGTTCTGCTGCAGAATCTTACCATGTCCTACTCTGGACTGTACCAGTGCACAGCA
GGCAACGAAGCTGGGAAGGAAAGCTGTGTGGTGCGAGTAACTGTACAGTATGTACAAAGCATC
GGCATGGTTGCAGGAGCAGTGACAGGCATAGTGGCTGGAGCCCTGCTGATTTTCCTCTTGGTG
TGGCTGCTAATCCGAAGGAAAGACAAAGAAAGATATGAGGAAGAAGAGAGACCTAATGAAATT
CGAGAAGATGCTGAAGCTCCAAAAGCCCGTCTTGTGAAACCCAGCTCCTCTTCCTCAGGCTCT
CGGAGCTCACGCTCTGGTTCTTCCTCCACTCGCTCCACAGCAAATAGTGCCTCACGCAGCCAG
CGGACACTGTCAACTGACGCAGCACCCCAGCCAGGGCTGGCCACCCAGGCATACAGCCTAGTG
GGGCCAGAGGTGAGAGGTTCTGAACCAAAGAAAGTCCACCATGCTAATCTGACCAAAGCAGAA
ACCACACCCAGCATGATCCCCAGCCAGAGCAGAGCCTTCCAAACGGTC**TGA**ATTACAATGGAC
TTGACTCCCACGCTTTCCTAGGAGTCAGGGTCTTTGGACTCTTCTCGTCATTGGAGCTCAAGT
CACCAGCCACACAACCAGATGAGAGGTCATCTAAGTAGCAGTGAGCATTGCACGGAACAGATT
CAGATGAGCATTTTCCTTATACAATACCAAACAAGCAAAAGGATGTAAGCTGATTCATCTGTA
AAAAGGCATCTTATTGTGCCTTTAGACCAGAGTAAGGGAAAGCAGGAGTCCAAATCTATTTGT
TGACCAGGACCTGTGGTGAGAAGGTTGGGGAAAGGTGAGGTGAATATACCTAAAACTTTTAAT
GTGGGATATTTTGTATCAGTGCTTTGATTCACAATTTTCAAGAGGAAATGGGATGCTGTTTGT
AAATTTTCTATGCATTTCTGCAAACTTATTGGATTATTAGTTATTCAGACAGTCAAGCAGAAC
CCACAGCCTTATTACACCTGTCTACACCATGTACTGAGCTAACCACTTCTAAGAAACTCCAAA
AAAGGAAACATGTGTCTTCTATTCTGACTTAACTTCATTTGTCATAAGGTTTGGATATTAATT
TCAAGGGGAGTTGAAATAGTGGGAGATGGAGAAGAGTGAATGAGTTTCTCCCACTCTATACTA
ATCTCACTATTTGTATTGAGCCCAAAATAACTATGAAAGGAGACAAAAATTTGTGACAAAGGA
TTGTGAAGAGCTTTCCATCTTCATGATGTTATGAGGATTGTTGACAAACATTAGAAATATATA
ATGGAGCAATTGTGGATTTCCCCTCAAATCAGATGCCTCTAAGGACTTTCCTGCTAGATATTT
CTGGAAGGAGAAAATACAACATGTCATTTATCAACGTCCTTAGAAAGAATTCTTCTAGAGAAA
AAGGGATCTAGGAATGCTGAAAGATTACCCAACATACCATTATAGTCTCTTCTTTCTGAGAAA
ATGTGAAACCAGAATTGCAAGACTGGGTGGACTAGAAAGGGAGATTAGATCAGTTTTCTCTTA
ATATGTCAAGGAAGGTAGCCGGGCATGGTGCCAGGCACCTGTAGGAAAATCCAGCAGGTGGAG
GTTGCAGTGAGCCGAGATTATGCCATTGCACTCCAGCCTGGGTGACAGAGCGGGACTCCGTCTC

# FIGURE 388

MSLLLLLLLVSYYVGTLGTHTEIKRVAEEKVTLPCHHQLGLPEKDTLDIEWLLTDNEGNQKVV
ITYSSRHVYNNLTEEQKGRVAFASNFLAGDASLQIEPLKPSDEGRYTCKVKNSGRYVWSHVIL
KVLVRPSKPKCELEGELTEGSDLTLQCESSSGTEPIVYYWQRIREKEGEDERLPPKSRIDYNH
PGRVLLQNLTMSYSGLYQCTAGNEAGKESCVVRVTVQYVQSIGMVAGAVTGIVAGALLIFLLV
WLLIRRKDKERYEEEERPNEIREDAEAPKARLVKPSSSSSGSRSSRSGSSSTRSTANSASRSQ
RTLSTDAAPQPGLATQAYSLVGPEVRGSEPKKVHHANLTKAETTPSMIPSQSRAFQTV

**Important freatures:**

**Signal sequence:**

amino acids 1-16

**Transmembrane domain:**

amino acids 232-251

# FIGURE 389

GCGGCACCTGGAAG**ATG**CGCCCATTGGCTGGTGGCCTGCTCAAGGTGGTGTTCGTGGTCTTCG
CCTCCTTGTGTGCCTGGTATTCGGGGTACCTGCTCGCAGAGCTCATTCCAGATGCACCCCTGT
CCAGTGCTGCCTATAGCATCCGCAGCATCGGGGAGAGGCCTGTCCTCAAAGCTCCAGTCCCCA
AAAGGCAAAAATGTGACCACTGGACTCCCTGCCCATCTGACACCTATGCCTACAGGTTACTCA
GCGGAGGTGGCAGAAGCAAGTACGCCAAAATCTGCTTTGAGGATAACCTACTTATGGGAGAAC
AGCTGGGAAATGTTGCCAGAGGAATAAACATTGCCATTGTCAACTATGTAACTGGGAATGTGA
CAGCAACACGATGTTTTGATATGTATGAAGGCGATAACTCTGGACCGATGACAAAGTTTATTC
AGAGTGCTGCTCCAAAATCCCTGCTCTTCATGGTGACCTATGACGACGGAAGCACAAGACTGA
ATAACGATGCCAAGAATGCCATAGAAGCACTTGGAAGTAAAGAAATCAGGAACATGAAATTCA
GGTCTAGCTGGGTATTTATTGCAGCAAAAGGCTTGGAACTCCCTTCCGAAATTCAGAGAGAAA
AGATCAACCACTCTGATGCTAAGAACAACAGATATTCTGGCTGGCCTGCAGAGATCCAGATAG
AAGGCTGCATACCCAAAGAACGAAGC**TGA**CACTGCAGGGTCCTGAGTAAATGTGTTCTGTATA
AACAAATGCAGCTGGAATCGCTCAAGAATCTTATTTTTCTAAATCCAACAGCCCATATTTGAT
GAGTATTTTGGGTTTGTTGTAAACCAATGAACATTTGCTAGTTGTATCAAATCTTGGTACGCA
GTATTTTTATACCAGTATTTTATGTAGTGAAGATGTCAATTAGCAGGAAACTAAAATGAATGG
AAATTCTTAAAAAAAAAA

# FIGURE 390

MRPLAGGLLKVVFVVFASLCAWYSGYLLAELIPDAPLSSAAYSIRSIGERPVLKAPVPKRQKC
DHWTPCPSDTYAYRLLSGGGRSKYAKICFEDNLLMGEQLGNVARGINIAIVNYVTGNVTATRC
FDMYEGDNSGPMTKFIQSAAPKSLLFMVTYDDGSTRLNNDAKNAIEALGSKEIRNMKFRSSWV
FIAAKGLELPSEIQREKINHSDAKNNRYSGWPAEIQIEGCIPKERS

**Important features:**

**Signal sequence.**

amino acids 1-20

**N-glycosylation sites.**

amino acids 120-124, 208-212

**Glycosaminoglycan attachment site.**

amino acids 80-84

**N-myristoylation sites.**

amino acids 81-87, 108-114, 119-125

# FIGURE 391

GGGGGCTTTCTTGGGCTTGGCTGCTTGGAACACCTGCCTCCAAGGACCGGCCTCGGAGGGGTCGCCGGGAAAGGG
AGGGAAGAAGGAAGGGCGGGGCCGGCCCCCCTGCGCCCGCCCCGCGCCTCTGCGCGCCCCTGTCCGCCCCGGCCC
AGCCCAGCCCAGCCCCGCGGGCCGGTCACACGCGCAGCCAGCCGGCCGCCTCCCGCGCCCAAGCGCGCCGCTCTG
CTGTGCCCTGCGCCCTTGCCCCGCGCCAGCTTCTGCGCCCGCAGCCCGCCCGGCGCCCCCGGTGACCGTGACCCT
GCCCTGGGCGCGGGGCGGAGCAGGC**ATG**TCCCGCCCGGGGACCGCTACCCCAGCGCTGGCCCTGGTGCTCCTGGC
AGTGACCCTGGCCGGGGTCGGAGCCCAGGGCGCAGCCCTCGAGGACCCTGATTATTACGGGCAGGAGATCTGGAG
CCGGGAGCCCTACTACGCGCGCCCGGAGCCCGAGCTCGAGACCTTCTCTCCGCCGCTGCCTGCGGGGCCCGGGGA
GGAGTGGGAGCGGCGCCCGCAGGAGCCCAGGCCGCCCAAGAGGGCCACCAAGCCCAAGAAAGCTCCCAAGAGGGA
GAAGTCGGCTCCGGAGCCGCCTCCACCAGGTAAACACAGCAACAAAAAAGTTATGAGAACCAAGAGCTCTGAGAA
GGCTGCCAACGATGATCACAGTGTCCGTGTGGCCCGTGAAGATGTCAGAGAGAGTTGCCCACCTCTTGGTCTGGA
AACCTTAAAAATCACAGACTTCCAGCTCCATGCCTCCACGGTGAAGCGCTATGGCCTGGGGGCACATCGAGGGAG
ACTCAACATCCAGGCGGGCATTAATGAAAATGATTTTTATGACGGAGCGTGGTGCGCGGGAAGAAATGACCTCCA
GCAGTGGATTGAAGTGGATGCTCGGCGCCTGACCAGATTCACTGGTGTCATCACTCAAGGGAGGAACTCCCTCTG
GCTGAGTGACTGGGTGACATCCTATAAGGTCATGGTGAGCAATGACAGCCACACGTGGGTCACTGTTAAGAATGG
ATCTGGAGACATGATATTTGAGGGAAACAGTGAGAAGGAGATCCCTGTTCTCAATGAGCTACCCGTCCCCATGGT
GGCCCGCTACATCCGCATAAACCCTCAGTCCTGGTTTGATAATGGGAGCATCTGCATGAGAATGGAGATCCTGGG
CTGCCCACTGCCAGATCCTAATAATTATTATCACCGCCGGAACGAGATGACCACCACTGATGACCTGGATTTTAA
GCACCACAATTATAAGGAAATGCGCCAGTTGATGAAAGTTGTGAATGAAATGTGTCCCAATATCACCAGAATTTA
CAACATTGGAAAAAGCCACCAGGGCCTGAAGCTGTATGCTGTGGAGATCTCAGATCACCCTGGGGAGCATGAAGT
CGGTGAGCCCGAGTTCCACTACATCGCGGGGGCCCACGGCAATGAGGTGCTGGGCCGGGAGCTGCTGCTGCTGCT
GGTGCAGTTCGTGTGTCAGGAGTACTTGGCCCGGAATGCGCGCATCGTCCACCTGGTGGAGGAGACGCGGATTCA
CGTCCTCCCCTCCCTCAACCCCGATGGCTACGAGAAGGCCTACGAAGGGGGCTCGGAGCTGGGAGGCTGGTCCCT
GGGACGCTGGACCCACGATGGAATTGACATCAACAACAACTTTCCTGATTTAAACACGCTGCTCTGGGAGGCAGA
GGATCGACAGAATGTCCCCAGGAAAGTTCCCAATCACTATATTGCAATCCCTGAGTGGTTTCTGTCGGAAAATGC
CACGGTGGCTGCCGAGACCAGAGCAGTCATAGCCTGGATGGAAAAAATCCCTTTTGTGCTGGGCGGCAACCTGCA
GGGCGGCGAGCTGGTGGTGGCGTATCCCTACGACCTGGTGCGGTCCCCCTGGAAGACGCAGGAACACACCCCCAC
CCCCGATGACCACGTGTTCCGCTGGCTGGCCTACTCCTATGCCTCCACACACCGCCTCATGACAGACGCCCGGAG
GAGGGTGTGCCACACGGAGGACTTCCAGAAGGAGGAGGGCACTGTCAATGGGGCCTCCTGGCACACCGTCGCTGG
AAGTCTGAACGATTTCAGCTACCTTCATACAAACTGCTTCGAACTGTCCATCTACGTGGGCTGTGATAAATACCC
ACATGAGAGCCAGCTGCCCGAGGAGTGGGAGAATAACCGGGAATCTCTGATCGTGTTCATGGAGCAGGTTCATCG
TGGCATTAAAGGCTTGGTGAGAGATTCACATGGAAAAGGAATCCCAAACGCCATTATCTCCGTAGAAGGCATTAA
CCATGACATCCGAACAGCCAACGATGGGGATTACTGGCGCCTCCTGAACCCTGGAGAGTATGTGGTCACAGCAAA
GGCCGAAGGTTTCACTGCATCCACCAAGAACTGTATGGTTGGCTATGACATGGGGGCCACAAGGTGTGACTTCAC
ACTTAGCAAAACCAACATGGCCAGGATCCGAGAGATCATGGAGAAGTTTGGGAAGCAGCCCGTCAGCCTGCCAGC
CAGGCGGCTGAAGCTGCGGGGGCGGAAGAGACGACAGCGTGGG**TGA**CCCTCCTGGGCCCTTGAGACTCGTCTGGG
ACCCATGCAAATTAAACCAACCTGGTAGTAGCTCCATAGTGGACTCACTCACTGTTGTTTCCTCTGTAATTCAAG
AAGTGCCTGGAAGAGAGGGTGCATTGTGAGGCAGGTCCCAAAAGGGAAGGCTGGAGGCTGAGGCTGTTTTCTTTT
CTTTGTTCCCATTTATCCAAATAACTTGGACAGAGCAGCAGCAGAGAAAAGCTGATGGGAGTGAGAGAACTCAGCAAG
CCAACCTGGGAATCAGAGAGAGAAGGAGAAGGAGGGGAGCCTGTCCGTTCAGAGCCTCTGGCTGCATAGAAAAGG
ATTCTGGTGCTTCCCCTGTTTGCGTGGCAGCAAGGGTTCCACGTGCATTTGCAATTTGCACAGCTAAAATTGCAG
CATTTCCCCAGCTGGGCTGTCCCAAATGTTACCATTTGAGATGCTCCCAGGCGTCCTAAGAGAATCCACCCTCTC
TGGCCCTGGGACATTGCAAGCTGCTACAAATAAATTCTGTGTTCTTTTGACAATAGCGTCATTGCCAAGTGCACA
TCAGTGAGCCTCTTGAATCTGTTTAGTCTCCTTTTTCAACAAAGGAGTGTGTTCAGAAAAGGAGAGAGAGGCTGA
GATCATTCAGGAGTTTGTTGGGCAGCAAGCATGGAGCTTCTTGCACAAATTCTGGGTCCATAAACAACCCCCAAA
GTCCCTGCTGATCCAGTAGCCCTGGAGGTTCCCCAGGTAGGGAGAGCCAGAGGTGCCAGCCTTCCTGAAGGGCCA
GAAAATTTAGCCTGGATCTCCTCTTTTACCTGCTAGGACTGGAAAGAGCCAGAAGTGGGGTGGCCTGAAGCCCTC
TCTCTGCTTGAGGTATTGCCCCTGTGTGGAATTGAGTGCTCATGGGTTGGCCTCATATCAGCCTGGGAGTTATTT
TTGATATGTAGAATGCCAGATCTTCCAGATTAGGCTAAATGTAATGAAAACCTCTTAGGATTATCTGTGGAGCAT
CAGTTTGGGAAGAATTATTGAATTATCTTGCAAGAAAAAAGTATGTCTCACTTTTTGTTAATGTTGCTGCCTCAT
TGACCTGGGAAAAATGAAAAAAAAAAAATAAAGCAAATGGTAAGACCCTTAAAAAAAAAAAAAAAAAAAAAAAAA
AAAAAAAAAAAAAAAAAAAAAA

# FIGURE 392

MSRPGTATPALALVLLAVTLAGVGAQGAALEDPDYYGQEIWSREPYYARPEPELETFSPPLPA
GPGEEWERRPQEPRPPKRATKPKKAPKREKSAPEPPPPGKHSNKKVMRTKSSEKAANDDHSVR
VAREDVRESCPPLGLETLKITDFQLHASTVKRYGLGAHRGRLNIQAGINENDFYDGAWCAGRN
DLQQWIEVDARRLTRFTGVITQGRNSLWLSDWVTSYKVMVSNDSHTWVTVKNGSGDMIFEGNS
EKEIPVLNELPVPMVARYIRINPQSWFDNGSICMRMEILGCPLPDPNNYYHRRNEMTTTDDLD
FKHHNYKEMRQLMKVVNEMCPNITRIYNIGKSHQGLKLYAVEISDHPGEHEVGEPEFHYIAGA
HGNEVLGRELLLLLVQFVCQEYLARNARIVHLVEETRIHVLPSLNPDGYEKAYEGGSELGGWS
LGRWTHDGIDINNNFPDLNTLLWEAEDRQNVPRKVPNHYIAIPEWFLSENATVAAETRAVIAW
MEKIPFVLGGNLQGGELVVAYPYDLVRSPWKTQEHTPTPDDHVFRWLAYSYASTHRLMTDARR
RVCHTEDFQKEEGTVNGASWHTVAGSLNDFSYLHTNCFELSIYVGCDKYPHESQLPEEWENNR
ESLIVFMEQVHRGIKGLVRDSHGKGIPNAIISVEGINHDIRTANDGDYWRLLNPGEYVVTAKA
EGFTASTKNCMVGYDMGATRCDFTLSKTNMARIREIMEKFGKQPVSLPARRLKLRGRKRRQRG

# FIGURE 393

GTCCCACATCCTGCTCAACTGGGTCAGGTCCCTCTTAGACCAGCTCTTGTCCATCATTTGCTGAAGTGGACCAAC
TAGTTCCCCAGTAGGGGGTCTCCCCTGGCAATTCTTGATCGGCGTTTGGACATCTCAGATCGCTTCCAATGAAGA
TGGCCTTGCCTTGGGGTCCTGCTTGTTTCATAATCATCTAACTATGGGACAAGGTTGTGCCGGCAGCTCTGGGGG
AAGGAGCACGGGGCTGATCAAGCCATCCAGGAAACACTGGAGGACTTGTCCAGCCTTGAAAGAACTCTAGTGGTT
TCTGAATCTAGCCCACTTGGCGGTAAGC**ATG**ATGCAACTTCTGCAACTTCTGCTGGGGCTTTTGGGGCCAGGTGG
CTACTTATTTCTTTTAGGGGATTGTCAGGAGGTGACCACTCTCACGGTGAAATACCAAGTGTCAGAGGAAGTGCC
ATCTGGTACAGTGATCGGGAAGCTGTCCCAGGAACTGGGCCGGGAGGAGAGGCGGAGGCAAGCTGGGGCCGCCTT
CCAGGTGTTGCAGCTGCCTCAGGCGCTCCCCATTCAGGTGGACTCTGAGGAAGGCTTGCTCAGCACAGGCAGGCG
GCTGGATCGAGAGCAGCTGTGCCGACAGTGGGATCCCTGCCTGGTTTCCTTTGATGTGCTTGCCACAGGGGATTT
GGCTCTGATCCATGTGGAGATCCAAGTGCTGGACATCAATGACCACCAGCCACGGTTTCCCAAAGGCGAGCAGGA
GCTGGAAATCTCTGAGAGCGCCTCTCTGCGAACCCGGATCCCCCTGGACAGAGCTCTTGACCCAGACACAGGCCC
TAACACCCTGCACACCTACACTCTGTCTCCCAGTGAGCACTTTGCCTTGGATGTCATTGTGGGCCCTGATGAGAC
CAAACATGCAGAACTCATAGTGGTGAAGGAGCTGGACAGGGAAATCCATTCATTTTTGATCTGGTGTTAACTGC
CTATGACAATGGGAACCCCCCCAAGTCAGGTACCAGCTTGGTCAAGGTCAACGTCTTGGACTCCAATGACAATAG
CCCTGCGTTTGCTGAGAGTTCACTGGCACTGGAAATCCAAGAAGATGCTGCACCTGGTACGCTTCTCATAAAACT
GACCGCCACAGACCCTGACCAAGGCCCCAATGGGGAGGTGGAGTTCTTCCTCAGTAAGCACATGCCTCCAGAGGT
GCTGGACACCTTCAGTATTGATGCCAAGACAGGCCAGGTCATTCTGCGTCGACCTCTAGACTATGAAAAGAACCC
TGCCTACGAGGTGGATGTTCAGGCAAGGGACCTGGGTCCCAATCCTATCCCAGCCCATTGCAAAGTTCTCATCAA
GGTTCTGGATGTCAATGACAACATCCCAAGCATCCACGTCACATGGGCCTCCCAGCCATCACTGGTGTCAGAAGC
TCTTCCCAAGGACAGTTTTATTGCTCTTGTCATGGCAGATGACTTGGATTCAGGACACAATGGTTTGGTCCACTG
CTGGCTGAGCCAAGAGCTGGGCCACTTCAGGCTGAAAAGAACTAATGGCAACACATACATGTTGCTAACCAATGC
CACACTGGACAGAGAGCAGTGGCCCAAATATACCCTCACTCTGTTAGCCCAAGACCAAGGACTCCAGCCCTTATC
AGCCAAGAAACAGCTCAGCATTCAGATCAGTGACATCAACGACAATGCACCTGTGTTTGAGAAAAGCAGGTATGA
AGTCTCCACGCGGGAAAACAACTTACCCTCTCTTCACCTCATTACCATCAAGGCTCATGATGCAGACTTGGGCAT
TAATGGAAAAGTCTCATACCGCATCCAGGACTCCCCAGTTGCTCACTTAGTAGCTATTGACTCCAACACAGGAGA
GGTCACTGCTCAGAGGTCACTGAACTATGAAGAGATGGCCGGCTTTGAGTTCCAGGTGATCGCAGAGGACAGCGG
GCAACCCATGCTTGCATCCAGTGTCTCTGTGTGGGTCAGCCTCTTGGATGCCAATGATAATGCCCCAGAGGTGGT
CCAGCCTGTGCTCAGCGATGGAAAAGCCAGCCTCTCCGTGCTTGTGAATGCCTCCACAGGCCACCTGCTGGTGCC
CATCGAGACTCCCAATGGCTTGGGCCCAGCGGGCACTGACACACCTCCACTGGCCACTCACAGCTCCCGGCCATT
CCTTTTGACAACCATTGTGGCAAGAGATGCAGACTCGGGGGCAAATGGAGAGCCCCTCTACAGCATCCGCAATGG
AAATGAAGCCCACCTCTTCATCCTCAACCCTCATACGGGGCAGCTGTTCGTCAATGTCACCAATGCCAGCAGCCT
CATTGGGAGTGAGTGGGAGCTGGAGATAGTAGTAGAGGACCAGGGAAGCCCCCCCTTACAGACCCGAGCCCTGTT
GAGGGTCATGTTTGTCACCAGTGTGGACCACCTGAGGGACTCAGCCCGCAAGCCTGGGGCCTTGAGCATGTCGAT
GCTGACGGTGATCTGCCTGGCTGTACTGTTGGGCATCTTCGGGTTGATCCTGGCTTTGTTCATGTCCATCTGCCG
GACAGAAAAGAAGGACAACAGGGCCTACAACTGTCGGGAGGCCGAGTCCACCTACCGCCAGCAGCCCAAGAGGCC
CCAGAAACACATTCAGAAGGCAGACATCCACCTCGTGCCTGTGCTCAGGGGTCAGGCAGGTGAGCCTTGTGAAGT
CGGGCAGTCCCACAAAGATGTGGACAAGGAGGCGATGATGGAAGCAGGCTGGGACCCCTGCCTGCAGGCCCCCTT
CCACCTCACCCCGACCCTGTACAGGACGCTGCGTAATCAAGGCAACCAGGGAGCACCGGCGGAGAGCCGAGAGGT
GCTGCAAGACACGGTCAACCTCCTTTTCAACCATCCCAGGCAGAGGAATGCCTCCCGGGAGAACCTGAACCTTCC
CGAGCCCCAGCCTGCCACAGGCCAGCCACGTTCCAGGCCTCTGAAGGTTGCAGGCAGCCCCACAGGGAGGCTGGC
TGGAGACCAGGGCAGTGAGGAAGCCCCACAGAGGCCACCAGCCTCCTCTGCAACCCTGAGACGGCAGCGACATCT
CAATGGCAAAGTGTCCCCTGAGAAAGAATCAGGGCCCCGTCAGATCCTGCGGAGCCTGGTCCGGCTGTCTGTGGC
TGCCTTCGCCGAGCGGAACCCCGTGGAGGAGCTCACTGTGGATTCTCCTCCTGTTCAGCAAATCTCCCAGCTGCT
GTCCTTGCTGCATCAGGGCCAATTCCAGCCCAAACCAAACCACCGAGGAAATAAGTACTTGGCCAAGCCAGGAGG
CAGCAGGAGTGCAATCCCAGACACAGATGGCCCAAGTGCAAGGGCTGGAGGCCAGACAGACCCAGAACAGGAGGA
AGGGCCTTTGGATCCTGAAGAGGACCTCTCTGTGAAGCAACTGCTAGAAGAAGAGCTGTCAAGTCTGCTGGACCC
CAGCACAGGTCTGGCCCTGGACCGGCTGAGCGCCCCTGACCCGGCCTGGATGGCGAGACTCTCTTTGCCCCTCAC
CACCAACTACCGTGACAATGTGATCTCCCCGGATGCTGCAGCCACGGAGGAGCCGAGGACCTTCCAGACGTTCGG
CAAGGCAGAGGCACCAGAGCTGAGCCCAACAGGCACGAGGCTGGCCAGCACCTTTGTCTCGGAGATGAGCTCACT
GCTGGAGATGCTGCTGGAACAGCGCTCCAGCATGCCCGTGGAGGCCGCCTCCGAGGCGCTGCGGCGGCTCTCGGT
CTGCGGGAGGACCCTCAGTTTAGACTTGGCCACCAGTGCAGCCTCAGGCATGAAAGTGCAAGGGGACCCAGGTGG
AAAGACGGGGACTGAGGGCAAGAGCAGAGGCAGCAGCAGCAGCAGCAGGTGCCTG**TGA**ACATACCTCAGACGCCT
CTGGATCCAAGAACCAGGGGCCTGAGGATCTGTGGACAAGAGCTGGTTTCTAAAATCTTGTAACTCACTAGCTAG
CGGCGGCCTGAGAACTTTAGGGTGACTGATGCTACCCCCACAGAGGAGGCAAGAGCCCCAGGACTAACAGCTGAC
TGACCAAAGCAGCCCCTTGTAAGCAGCTCTGAGTCTTTTGGAGGACAGGGACGGTTTGTGGCTGAGATAAGTGTT
TCCTGGCAAAACATATGTGGAGCACAAAGGGTCAGTCCTCTGGCAGAACAGATGCCACGGAGTATCACAGGCAGG
AAAGGGTGGCCTTCTTGGGTAGCAGGAGTCAGGGGGCTGTACCCTGGGGGTGCCAGGAAATGCTCTCTGACCTAT
CAATAAAGGAAAAGCAGTAAAAAAAAAAAAAAAAAAAA

# FIGURE 394

MMQLLQLLLGLLGPGGYLFLLGDCQEVTTLTVKYQVSEEVPSGTVIGKLSQELGREERRRQAG
AAFQVLQLPQALPIQVDSEEGLLSTGRRLDREQLCRQWDPCLVSFDVLATGDLALIHVEIQVL
DINDHQPRFPKGEQELEISESASLRTRIPLDRALDPDTGPNTLHTYTLSPSEHFALDVIVGPD
ETKHAELIVVKELDREIHSFFDLVLTAYDNGNPPKSGTSLVKVNVLDSNDNSPAFAESSLALE
IQEDAAPGTLLIKLTATDPDQGPNGEVEFFLSKHMPPEVLDTFSIDAKTGQVILRRPLDYEKN
PAYEVDVQARDLGPNPIPAHCKVLIKVLDVNDNIPSIHVTWASQPSLVSEALPKDSFIALVMA
DDLDSGHNGLVHCWLSQELGHFRLKRTNGNTYMLLTNATLDREQWPKYTLTLLAQDQGLQPLS
AKKQLSIQISDINDNAPVFEKSRYEVSTRENNLPSLHLITIKAHDADLGINGKVSYRIQDSPV
AHLVAIDSNTGEVTAQRSLNYEEMAGFEFQVIAEDSGQPMLASSVSVWVSLLDANDNAPEVVQ
PVLSDGKASLSVLVNASTGHLLVPIETPNGLGPAGTDTPPLATHSSRPFLLTTIVARDADSGA
NGEPLYSIRNGNEAHLFILNPHTGQLFVNVTNASSLIGSEWELEIVVEDQGSPPLQTRALLRV
MFVTSVDHLRDSARKPGALSMSMLTVICLAVLLGIFGLILALFMSICRTEKKDNRAYNCREAE
STYRQQPKRPQKHIQKADIHLVPVLRGQAGEPCEVGQSHKDVDKEAMMEAGWDPCLQAPFHLT
PTLYRTLRNQGNQGAPAESREVLQDTVNLLFNHPRQRNASRENLNLPEPQPATGQPRSRPLKV
AGSPTGRLAGDQGSEEAPQRPPASSATLRRQRHLNGKVSPEKESGPRQILRSLVRLSVAAFAE
RNPVEELTVDSPPVQQISQLLSLLHQGQFQPKPNHRGNKYLAKPGGSRSAIPDTDGPSARAGG
QTDPEQEEGPLDPEEDLSVKQLLEEELSSLLDPSTGLALDRLSAPDPAWMARLSLPLTTNYRD
NVISPDAAATEEPRTFQTFGKAEAPELSPTGTRLASTFVSEMSSLLEMLLEQRSSMPVEAASE
ALRRLSVCGRTLSLDLATSAASGMKVQGDPGGKTGTEGKSRGSSSSSRCL

**Important features:**

**Signal peptide:**

amino acids 1-13

**Transmembrane domain:**

amino acids 719-739

**N-glycosylation site.**

amino acids 415-418, 582-585, 659-662, 662-665 amd 857-860

**Cadherins extracellular repeated domain signature.**

amino acids 123-133, 232-242, 340-350, 448-458 and 553-563

# FIGURE 395

CCCAGGCTCTAGTGCAGGAGGAGAAGGAGGAGGAGCAGGAGGTGGAGATTCCCAGTTAAAAGG
CTCCAGAATCGTGTACCAGGCAGAGAACTGAAGTACTGGGGCCTCCTCCACTGGGTCCGAATC
AGTAGGTGACCCCGCCCCTGGATTCTGGAAGACCTCACC**ATG**GGACGCCCCCGACCTCGTGCG
GCCAAGACGTGGATGTTCCTGCTCTTGCTGGGGGGAGCCTGGGCAGGACACTCCAGGGCACAG
GAGGACAAGGTGCTGGGGGGTCATGAGTGCCAACCCCATTCGCAGCCTTGGCAGGCGGCCTTG
TTCCAGGGCCAGCAACTACTCTGTGGCGGTGTCCTTGTAGGTGGCAACTGGGTCCTTACAGCT
GCCCACTGTAAAAAACCGAAATACACAGTACGCCTGGGAGACCACAGCCTACAGAATAAAGAT
GGCCCAGAGCAAGAAATACCTGTGGTTCAGTCCATCCCACACCCCTGCTACAACAGCAGCGAT
GTGGAGGACCACAACCATGATCTGATGCTTCTTCAACTGCGTGACCAGGCATCCCTGGGGTCC
AAAGTGAAGCCCATCAGCCTGGCAGATCATTGCACCCAGCCTGGCCAGAAGTGCACCGTCTCA
GGCTGGGGCACTGTCACCAGTCCCCGAGAGAATTTTCCTGACACTCTCAACTGTGCAGAAGTA
AAAATCTTTCCCCAGAAGAAGTGTGAGGATGCTTACCCGGGGCAGATCACAGATGGCATGGTC
TGTGCAGGCAGCAGCAAAGGGGCTGACACGTGCCAGGGCGATTCTGGAGGCCCCCTGGTGTGT
GATGGTGCACTCCAGGGCATCACATCCTGGGGCTCAGACCCCTGTGGGAGGTCCGACAAACCT
GGCGTCTATACCAACATCTGCCGCTACCTGGACTGGATCAAGAAGATCATAGGCAGCAAGGGC
**TGA**TTCTAGGATAAGCACTAGATCTCCCTTAATAAACTCACAACTCTCTGGTTC

# FIGURE 396

MGRPRPRAAKTWMFLLLLGGAWAGHSRAQEDKVLGGHECQPHSQPWQAALFQGQQLLCGGVLV
GGNWVLTAAHCKKPKYTVRLGDHSLQNKDGPEQEIPVVQSIPHPCYNSSDVEDHNHDLMLLQL
RDQASLGSKVKPISLADHCTQPGQKCTVSGWGTVTSPRENFPDTLNCAEVKIFPQKKCEDAYP
GQITDGMVCAGSSKGADTCQGDSGGPLVCDGALQGITSWGSDPCGRSDKPGVYTNICRYLDWI
KKIIGSKG

**Important Features:**

**Signal peptide:**

amino acids 1-23

**Transmembrane domain:**

amino acids 51-71

**N-glycosylation site.**

amino acids 110-113

**Serine proteases, trypsin family, histidine active site.**

amino acids 69-74 and 207-217

**Tyrosine kinase phosphorylation site.**

amino acids 182-188

**Kringle domain proteins motif**

amino acids 205-217

# FIGURE 397

GGCGGCTGCTGAGCTGCCTTGAGGTGCAGTGTTGGGGATCCAGAGCC**ATG**TCGGACCTGCTAC
TACTGGGCCTGATTGGGGGCCTGACTCTCTTACTGCTGCTGACGCTGCTGGCCTTTGCCGGGT
ACTCAGGGCTACTGGCTGGGGTGGAAGTGAGTGCTGGGTCACCCCCCATCCGCAACGTCACTG
TGGCCTACAAGTTCCACATGGGGCTCTATGGTGAGACTGGGCGGCTTTTCACTGAGAGCTGCA
GCATCTCTCCCAAGCTCCGCTCCATCGCTGTCTACTATGACAACCCCCACATGGTGCCCCCTG
ATAAGTGCCGATGTGCCGTGGGCAGCATCCTGAGTGAAGGTGAGGAATCGCCCTCCCCTGAGC
TCATCGACCTCTACCAGAAATTTGGCTTCAAGGTGTTCTCCTTCCCGGCACCCAGCCATGTGG
TGACAGCCACCTTCCCCTACACCACCATTCTGTCCATCTGGCTGGCTACCCGCCGTGTCCATC
CTGCCTTGGACACCTACATCAAGGAGCGGAAGCTGTGTGCCTATCCTCGGCTGGAGATCTACC
AGGAAGACCAGATCCATTTCATGTGCCCACTGGCACGGCAGGGAGACTTCTATGTGCCTGAGA
TGAAGGAGACAGAGTGGAAATGGCGGGGGCTTGTGGAGGCCATTGACACCCAGGTGGATGGCA
CAGGAGCTGACACAATGAGTGACACGAGTTCTGTAAGCTTGGAAGTGAGCCCTGGCAGCCGGG
AGACTTCAGCTGCCACACTGTCACCTGGGGCGAGCAGCCGTGGCTGGGATGACGGTGACACCC
GCAGCGAGCACAGCTACAGCGAGTCAGGTGCCAGCGGCTCCTCTTTTGAGGAGCTGGACTTGG
AGGGCGAGGGGCCCTTAGGGGAGTCACGGCTGGACCCTGGGACTGAGCCCCTGGGGACTACCA
AGTGGCTCTGGGAGCCCACTGCCCCTGAGAAGGGCAAGGAG**TAA**CCCATGGCCTGCACCCTCC
TGCAGTGCAGTTGCTGAGGAACTGAGCAGACTCTCCAGCAGACTCTCCAGCCCTCTTCCTCCT
TCCTCTGGGGGAGGAGGGGTTCCTGAGGGACCTGACTTCCCCTGCTCCAGGCCTCTTGCTAAG
CCTTCTCCTCACTGCCCTTTAGGCTCCCAGGGCCAGAGGAGCCAGGGACTATTTCTGCACCA
GCCCCCAGGGCTGCCGCCCCTGTTGTGTCTTTTTTCAGACTCACAGTGGAGCTTCCAGGACC
CAGAATAAAGCCAATGATTTACTTGTTTCACCTGGAAAAAAAAAAAAAAAAA

# FIGURE 398

MSDLLLLGLIGGLTLLLLLTLLAFAGYSGLLAGVEVSAGSPPIRNVTVAYKFHMGLYGETGRL
FTESCSISPKLRSIAVYYDNPHMVPPDKCRCAVGSILSEGEESPSPELIDLYQKFGFKVFSFP
APSHVVTATFPYTTILSIWLATRRVHPALDTYIKERKLCAYPRLEIYQEDQIHFMCPLARQGD
FYVPEMKETEWKWRGLVEAIDTQVDGTGADTMSDTSSVSLEVSPGSRETSAATLSPGASSRGW
DDGDTRSEHSYSESGASGSSFEELDLEGEGPLGESRLDPGTEPLGTTKWLWEPTAPEKGKE

# FIGURE 399

GGACGAGGGCAGATCTCGTTCTGGGGCAAGCCGTTGACACTCGCTCCCTGCCACCGCCCGGGC
TCCGTGCCGCCAAGTTTTCATTTTCCACCTTCTCTGCCTCCAGTCCCCCAGCCCCTGGCCGAG
AGAAGGGTCTTACCGGCCGGGATTGCTGGAAACACCAAGAGGTGGTTTTTGTTTTTTAAAACT
TCTGTTTCTTGGGAGGGGGTGTGGCGGGGCAGG**ATG**AGCAACTCCGTTCCTCTGCTCTGTTTC
TGGAGCCTCTGCTATTGCTTTGCTGCGGGGAGCCCCGTACCTTTTGGTCCAGAGGGACGGCTG
GAAGATAAGCTCCACAAACCCAAAGCTACACAGACTGAGGTCAAACCATCTGTGAGGTTTAAC
CTCCGCACCTCCAAGGACCCAGAGCATGAAGGATGCTACCTCTCCGTCGGCCACAGCCAGCCC
TTAGAAGACTGCAGTTTCAACATGACAGCTAAAACCTTTTTCATCATTCACGGATGGACGATG
AGCGGTATCTTTGAAAACTGGCTGCACAAACTCGTGTCAGCCCTGCACACAAGAGAGAAAGAC
GCCAATGTAGTTGTGGTTGACTGGCTCCCCCTGGCCCACCAGCTTTACACGGATGCGGTCAAT
AATACCAGGGTGGTGGGACACAGCATTGCCAGGATGCTCGACTGGCTGCAGGAGAAGGACGAT
TTTTCTCTCGGGAATGTCCACTTGATCGGCTACAGCCTCGGAGCGCACGTGGCCGGGTATGCA
GGCAACTTCGTGAAAGGAACGGTGGGCCGAATCACAGGTTTGGATCCTGCCGGGCCCATGTTT
GAAGGGGCCGACATCCACAAGAGGCTCTCTCCGGACGATGCAGATTTTGTGGATGTCCTCCAC
ACCTACACGCGTTCCTTCGGCTTGAGCATTGGTATTCAGATGCCTGTGGGCCACATTGACATC
TACCCCAATGGGGGTGACTTCCAGCCAGGCTGTGGACTCAACGATGTCTTGGGATCAATTGCA
TATGGAACAATCACAGAGGTGGTAAAATGTGAGCATGAGCGAGCCGTCCACCTCTTTGTTGAC
TCTCTGGTGAATCAGGACAAGCCGAGTTTTGCCTTCCAGTGCACTGACTCCAATCGCTTCAAA
AAGGGGATCTGTCTGAGCTGCCGCAAGAACCGTTGTAATAGCATTGGCTACAATGCCAAGAAA
ATGAGGAACAAGAGGAACAGCAAAATGTACCTAAAAACCCGGGCAGGCATGCCTTTCAGAGGT
AACCTTCAGTCCCTGGAGTGTCCC**TGA**GGAAGGCCCTTAATACCTCCTTCTTAATACCATGCT
GCAGAGCAGGGCACATCCTAGCCCAGGAGAAGTGGCCAGCACAATCCAATCAAATCGTTGCAA
ATCAGATTACACTGTGCATGTCCTAGGAAAGGGAATCTTTACAAAATAAACAGTGTGGACCCC
TAATAAAAAAAAAAAAAAAAAAAAAAAAAAAAAAAAAAAAAAAAAAAAAAAAAAAAAAAAA

# FIGURE 400

MSNSVPLLCFWSLCYCFAAGSPVPFGPEGRLEDKLHKPKATQTEVKPSVRFNLRTSKDPEHEG
CYLSVGHSQPLEDCSFNMTAKTFFIIHGWTMSGIFENWLHKLVSALHTREKDANVVVVDWLPL
AHQLYTDAVNNTRVVGHSIARMLDWLQEKDDFSLGNVHLIGYSLGAHVAGYAGNFVKGTVGRI
TGLDPAGPMFEGADIHKRLSPDDADFVDVLHTYTRSFGLSIGIQMPVGHIDIYPNGGDFQPGC
GLNDVLGSIAYGTITEVVKCEHERAVHLFVDSLVNQDKPSFAFQCTDSNRFKKGICLSCRKNR
CNSIGYNAKKMRNKRNSKMYLKTRAGMPFRGNLQSLECP

**Important features:**

**Signal peptide:**

amino acids 1-16

**Lipases, serine active site.**

amino acids 163-172

**N-glycosylation sites.**

amino acids 80-83 and 136-139

# FIGURE 401

CTTCCCAGCCCTGTGCCCCAAAGCACCTGGAGCATATAGCCTTGCAGAACTTCTACTTGCCTG
CCTCCCTGCCTCTGGCC**ATG**GCCTGCCGGTGCCTCAGCTTCCTTCTGATGGGGACCTTCCTGT
CAGTTTCCCAGACAGTCCTGGCCCAGCTGGATGCACTGCTGGTCTTCCCAGGCCAAGTGGCTC
AACTCTCCTGCACGCTCAGCCCCCAGCACGTCACCATCAGGGACTACGGTGTGTCCTGGTACC
AGCAGCGGGCAGGCAGTGCCCCTCGATATCTCCTCTACTACCGCTCGGAGGAGGATCACCACC
GGCCTGCTGACATCCCCGATCGATTCTCGGCAGCCAAGGATGAGGCCCACAATGCCTGTGTCC
TCACCATTAGTCCCGTGCAGCCTGAAGACGACGCGGATTACTACTGCTCTGTTGGCTACGGCT
TTAGTCCC**TAG**GGGTGGGGTGTGAGATGGGTGCCTCCCCTCTGCCTCCCATTTCTGCCCCTGA
CCTTGGGTCCCTTTTAAACTTTCTCTGAGCCTTGCTTCCCCTCTGTAAAATGGGTTAATAATA
TTCAACATGTCAACAAC

# FIGURE 402

MACRCLSFLLMGTFLSVSQTVLAQLDALLVFPGQVAQLSCTLSPQHVTIRDYGVSWYQQRAGS
APRYLLYYRSEEDHHRPADIPDRFSAAKDEAHNACVLTISPVQPEDDADYYCSVGYGFSP

# FIGURE 403

CGCGCCGGGCGCAGGGAGCTGAGTGGACGGCTCGAGACGGCGGCGCGTGCAGCAGCTCCAGAAAGCAGCGAGTTG
GCAGAGCAGGGCTGCATTTCCAGCAGGAGCTGCGAGCACAGTGCTGGCTCACAACAAG**ATG**CTCAAGGTGTCAGC
CGTACTGTGTGTGTGTGCAGCCGCTTGGTGCAGTCAGTCTCTCGCAGCTGCCGCGGCGGTGGCTGCAGCCGGGGG
GCGGTCGGACGGCGGTAATTTTCTGGATGATAAACAATGGCTCACCACAATCTCTCAGTATGACAAGGAAGTCGG
ACAGTGGAACAAATTCCGAGACGAAGTAGAGGATGATTATTTCCGCACTTGGAGTCCAGGAAAACCCTTCGATCA
GGCTTTAGATCCAGCTAAGGATCCATGCTTAAAGATGAAATGTAGTCGCCATAAAGTATGCATTGCTCAAGATTC
TCAGACTGCAGTCTGCATTAGTCACCGGAGGCTTACACACAGGATGAAAGAAGCAGGAGTAGACCATAGGCAGTG
GAGGGGTCCCATATTATCCACCTGCAAGCAGTGCCCAGTGGTCTATCCCAGCCCTGTTTGTGGTTCAGATGGTCA
TACCTACTCTTTTCAGTGCAAACTAGAATATCAGGCATGTGTCTTAGGAAAACAGATCTCAGTCAAATGTGAAGG
ACATTGCCCATGTCCTTCAGATAAGCCCACCAGTACAAGCAGAAATGTTAAGAGAGCATGCAGTGACCTGGAGTT
CAGGGAAGTGGCAAACAGATTGCGGGACTGGTTCAAGGCCCTTCATGAAAGTGGAAGTCAAAACAAGAAGACAAA
AACATTGCTGAGGCCTGAGAGAAGCAGATTCGATACCAGCATCTTGCCAATTTGCAAGGACTCACTTGGCTGGAT
GTTTAACAGACTTGATACAAACTATGACCTGCTATTGGACCAGTCAGAGCTCAGAAGCATTTACCTTGATAAGAA
TGAACAGTGTACCAAGGCATTCTTCAATTCTTGTGACACATACAAGGACAGTTTAATATCTAATAATGAGTGGTG
CTACTGCTTCCAGAGACAGCAAGACCCACCTTGCCAGACTGAGCTCAGCAATATTCAGAAGCGGCAAGGGGTAAA
GAAGCTCCTAGGACAGTATATCCCCCTGTGTGATGAAGATGGTTACTACAAGCCAACACAATGTCATGGCAGTGT
TGGACAGTGCTGGTGTGTTGACAGATATGGAAATGAAGTCATGGGATCCAGAATAAATGGTGTTGCAGATTGTGC
TATAGATTTTGAGATCTCCGGAGATTTTGCTAGTGGCGATTTTCATGAATGGACTGATGATGAGGATGATGAAGA
CGATATTATGAATGATGAAGATGAAATTGAAGATGATGATGAAGATGAAGGGGATGATGATGATGGTGGTGATGA
CCATGATGTATACATT**TGA**TTGATGACAGTTGAAATCAATAAATTCTACATTTCTAATATTTACAAAAATGATAG
CCTATTTAAAATTATCTTCTTCCCCAATAACAAAATGATTCTAAACCTCACATATATTTTGTATAATTATTTGAA
AAATTGCAGCTAAAGTTATAGAACTTTATGTTTAAATAAGAATCATTTGCTTTGAGTTTTTATATTCCTTACACA
AAAAGAAAATACATATGCAGTCTAGTCAGACAAAATAAAGTTTTGAAGTGCTACTATAATAAATTTTTCACGAGA
ACAAACTTTGTAAATCTTCCATAAGCAAAATGACAGCTAGTGCTTGGGATCGTACATGTTAATTTTTTGAAAGAT
AATTCTAAGTGAAATTTAAAATAAATAAATTTTTAATGACCTGGGTCTTAAGGATTTAGGAAAAATATGCATGCT
TTAATTGCATTTCCAAAGTAGCATCTTGCTAGACCTAGATGAGTCAGGATAACAGAGAGATACCACATGACTCCA
AAAAAAAAAAAAAA

# FIGURE 404

MLKVSAVLCVCAAAWCSQSLAAAAAVAAAGGRSDGGNFLDDKQWLTTISQYDKEVGQWNKFRD
EVEDDYFRTWSPGKPFDQALDPAKDPCLKMKCSRHKVCIAQDSQTAVCISHRRLTHRMKEAGV
DHRQWRGPILSTCKQCPVVYPSPVCGSDGHTYSFQCKLEYQACVLGKQISVKCEGHCPCPSDK
PTSTSRNVKRACSDLEFREVANRLRDWFKALHESGSQNKKTKTLLRPERSRFDTSILPICKDS
LGWMFNRLDTNYDLLLDQSELRSIYLDKNEQCTKAFFNSCDTYKDSLISNNEWCYCFQRQQDP
PCQTELSNIQKRQGVKKLLGQYIPLCDEDGYYKPTQCHGSVGQCWCVDRYGNEVMGSRINGVA
DCAIDFEISGDFASGDFHEWTDDEDDEDDIMNDEDEIEDDDEDEGDDDDGGDDHDVYI

**Important features:**

**Signal peptide:**

amino acids 1-16

**Leucine zipper pattern.**

amino acids 246-267

**N-myristoylation sites.**

amino acids 357-362, 371-376 and 376-381

**Thyroglobulin type-1 repeat proteins**

amino acids 353-365 and 339-352

# FIGURE 405

GGAAGGGGAGGAGCAGGCCACACAGGCACAGGCCGGTGAGGGACCTGCCCAGACCTGGAGGGTCTCGCTCTGTCA
CACAGGCTGGAGTGCAGTGGTGTGATCTTGGCTCATCGTAACCTCCACCTCCCGGGTTCAAGTGATTCTCATGCC
TCAGCCTCCCGAGTAGCTGGGATTACAGGTGGTGACTTCCAAGAGTGACTCCGTCGGAGGAAA**ATG**ACTCCCCAG
TCGCTGCTGCAGACGACACTGTTCCTGCTGAGTCTGCTCTTCCTGGTCCAAGGTGCCCACGGCAGGGGCCACAGG
GAAGACTTTCGCTTCTGCAGCCAGCGGAACCAGACACACAGGAGCAGCCTCCACTACAAACCCACACCAGACCTG
CGCATCTCCATCGAGAACTCCGAAGAGGCCCTCACAGTCCATGCCCCTTTCCCTGCAGCCCACCCTGCTTCCCGA
TCCTTCCCTGACCCCAGGGGCCTCTACCACTTCTGCCTCTACTGGAACCGACATGCTGGGAGATTACATCTTCTC
TATGGCAAGCGTGACTTCTTGCTGAGTGACAAAGCCTCTAGCCTCCTCTGCTTCCAGCACCAGGAGGAGAGCCTG
GCTCAGGGCCCCCCGCTGTTAGCCACTTCTGTCACCTCCTGGTGGAGCCCTCAGAACATCAGCCTGCCCAGTGCC
GCCAGCTTCACCTTCTCCTTCCACAGTCCTCCCCACACGGCCGCTCACAATGCCTCGGTGGACATGTGCGAGCTC
AAAAGGGACCTCCAGCTGCTCAGCCAGTTCCTGAAGCATCCCCAGAAGGCCTCAAGGAGGCCCTCGGCTGCCCCC
GCCAGCCAGCAGTTGCAGAGCCTGGAGTCGAAACTGACCTCTGTGAGATTCATGGGGGACATGGTGTCCTTCGAG
GAGGACCGGATCAACGCCACGGTGTGGAAGCTCCAGCCCACAGCCGGCCTCCAGGACCTGCACATCCACTCCCGG
CAGGAGGAGGAGCAGAGCGAGATCATGGAGTACTCGGTGCTGCTGCCTCGAACACTCTTCCAGAGGACGAAAGGC
CGGAGCGGGGAGGCTGAGAAGAGACTCCTCCTGGTGGACTTCAGCAGCCAAGCCCTGTTCCAGGACAAGAATTCC
AGCCAAGTCCTGGGTGAGAAGGTCTTGGGGATTGTGGTACAGAACACCAAAGTAGCCAACCTCACGGAGCCCGTG
GTGCTCACTTTCCAGCACCAGCTACAGCCGAAGAATGTGACTCTGCAATGTGTGTTCTGGGTTGAAGACCCCACA
TTGAGCAGCCCGGGGCATTGGAGCAGTGCTGGGTGTGAGACCGTCAGGAGAGAAACCCAAACATCCTGCTTCTGC
AACCACTTGACCTACTTTGCAGTGCTGATGGTCTCCTCGGTGGAGGTGGACGCCGTGCACAAGCACTACCTGAGC
CTCCTCTCCTACGTGGGCTGTGTCGTCTCTGCCCTGGCCTGCCTTGTCACCATTGCCGCCTACCTCTGCTCCAGG
GTGCCCCTGCCGTGCAGGAGGAAACCTCGGGACTACACCATCAAGGTGCACATGAACCTGCTGCTGGCCGTCTTC
CTGCTGGACACGAGCTTCCTGCTCAGCGAGCCGGTGGCCCTGACAGGCTCTGAGGCTGGCTGCCGAGCCAGTGCC
ATCTTCCTGCACTTCTCCCTGCTCACCTGCCTTTCCTGGATGGGCCTCGAGGGGTACAACCTCTACCGACTCGTG
GTGGAGGTCTTTGGCACCTATGTCCCTGGCTACCTACTCAAGCTGAGCGCCATGGGCTGGGGCTTCCCCATCTTT
CTGGTGACGCTGGTGGCCCTGGTGGATGTGGACAACTATGGCCCCATCATCTTGGCTGTGCATAGGACTCCAGAG
GGCGTCATCTACCCTTCCATGTGCTGGATCCGGGACTCCCTGGTCAGCTACATCACCAACCTGGGCCTCTTCAGC
CTGGTGTTTCTGTTCAACATGGCCATGCTAGCCACCATGGTGGTGCAGATCCTGCGGCTGCGCCCCCACACCCAA
AAGTGGTCACATGTGCTGACACTGCTGGGCCTCAGCCTGGTCCTTGGCCTGCCCTGGGCCTTGATCTTCTTCTCC
TTTGCTTCTGGCACCTTCCAGCTTGTCGTCCTCTACCTTTTCAGCATCATCACCTCCTTCCAAGGCTTCCTCATC
TTCATCTGGTACTGGTCCATGCGGCTGCAGGCCCGGGGTGGCCCCTCCCCTCTGAAGAGCAACTCAGACAGCGCC
AGGCTCCCCATCAGCTCGGGCAGCACCTCGTCCAGCCGCATC**TAG**GCCTCCAGCCCACCTGCCCATGTGATGAAG
CAGAGATGCGGCCTCGTCGCACACTGCCTGTGGCCCCCGAGCCAGGCCCAGCCCCAGGCCAGTCAGCCGCAGACT
TTGGAAAGCCCAACGACCATGGAGAGATGGGCCGTTGCCATGGTGGACGGACTCCCGGGCTGGGCTTTTGAATTG
GCCTTGGGGACTACTCGGCTCTCACTCAGCTCCCACGGGACTCAGAAGTGCGCCGCCATGCTGCCTAGGGTACTG
TCCCCACATCTGTCCCAACCCAGCTGGAGGCCTGGTCTCTCCTTACAACCCCTGGGCCCAGCCCTCATTGCTGGG
GGCCAGGCCTTGGATCTTGAGGGTCTGGCACATCCTTAATCCTGTGCCCCTGCCTGGGACAGAAATGTGGCTCCA
GTTGCTCTGTCTCTCGTGGTCACCCTGAGGGCACTCTGCATCCTCTGTCATTTTAACCTCAGGTGGCACCCAGGG
CGAATGGGGCCCAGGGCAGACCTTCAGGGCCAGAGCCCTGGCGGAGGAGAGGCCCTTTGCCAGGAGCACAGCAGC
AGCTCGCCTACCTCTGAGCCCAGGCCCCCTCCCTCCCTCAGCCCCCCAGTCCTCCCTCCATCTTCCCTGGGGTTC
TCCTCCTCTCCCAGGGCCTCCTTGCTCCTTCGTTCACAGCTGGGGGTCCCCGATTCCAATGCTGTTTTTTGGGGA
GTGGTTTCCAGGAGCTGCCTGGTGTCTGCTGTAAATGTTTGTCTACTGCACAAGCCTCGGCCTGCCCCTGAGCCA
GGCTCGGTACCGATGCGTGGGCTGGGCTAGGTCCCTCTGTCCATCTGGGCCTTTGTATGAGCTGCATTGCCCTTG
CTCACCCTGACCAAGCACACGCCTCAGAGGGGCCCTCAGCCTCTCCTGAAGCCCTCTTGTGGCAAGAACTGTGGA
CCATGCCAGTCCCGTCTGGTTTCCATCCCACCACTCCAAGGACTGAGACTGACCTCCTCTGGTGACACTGGCCTA
GAGCCTGACACTCTCCTAAGAGGTTCTCTCCAAGCCCCCAAATAGCTCCAGGCGCCCTCGGCCGCCCATCATGGT
TAATTCTGTCCAACAAACACACACGGGTAGATTGCTGGCCTGTTGTAGGTGGTAGGGACACAGATGACCGACCTG
GTCACTCCTCCTGCCAACATTCAGTCTGGTATGTGAGGCGTGCGTGAAGCAAGAACTCCTGGAGCTACAGGGACA
GGGAGCCATCATTCCTGCCTGGGAATCCTGGAAGACTTCCTGCAGGAGTCAGCGTTCAATCTTGACCTTGAAGAT
GGGAAGGATGTTCTTTTTACGTACCAATTCTTTTGTCTTTTGATATTAAAAAGAAGTACATGTTCATTGTAGAGA
ATTTGGAAACTGTAGAAGAGAATCAAGAAGAAAAATAAAAATCAGCTGTTGTAATCGCCTAGCAAAAAAAAAAAAA
AAAAAAAAAAAAAAAAAAAAAAAAAAAAAAAAAAAAAAAAAAAAAAAAAAAAAAAAAAAAAAAAAAAAA

# FIGURE 406

MTPQSLLQTTLFLLSLLFLVQGAHGRGHREDFRFCSQRNQTHRSSLHYKPTPDLRISIENSEE
ALTVHAPFPAAHPASRSFPDPRGLYHFCLYWNRHAGRLHLLYGKRDFLLSDKASSLLCFQHQE
ESLAQGPPLLATSVTSWWSPQNISLPSAASFTFSFHSPPHTAAHNASVDMCELKRDLQLLSQF
LKHPQKASRRPSAAPASQQLQSLESKLTSVRFMGDMVSFEEDRINATVWKLQPTAGLQDLHIH
SRQEEEQSEIMEYSVLLPRTLFQRTKGRSGEAEKRLLLVDFSSQALFQDKNSSQVLGEKVLGI
VVQNTKVANLTEPVVLTFQHQLQPKNVTLQCVFWVEDPTLSSPGHWSSAGCETVRRETQTSCF
CNHLTYFAVLMVSSVEVDAVHKHYLSLLSYVGCVVSALACLVTIAAYLCSRVPLPCRRKPRDY
TIKVHMNLLLAVFLLDTSFLLSEPVALTGSEAGCRASAIFLHFSLLTCLSWMGLEGYNLYRLV
VEVFGTYVPGYLLKLSAMGWGFPIFLVTLVALVDVDNYGPIILAVHRTPEGVIYPSMCWIRDS
LVSYITNLGLFSLVFLFNMAMLATMVVQILRLRPHTQKWSHVLTLLGLSLVLGLPWALIFFSF
ASGTFQLVVLYLFSIITSFQGFLIFIWYWSMRLQARGGPSPLKSNSDSARLPISSGSTSSSRI

**Important features:**

**Signal peptide:**

amino acids 1-25

**Putative transmembrane domains:**

amino acids 382-398, 402-420, 445-468, 473-491, 519-537, 568-590 and 634-657

**Microbodies C-terminal targeting signal.**

amino acids 691-693

**cAMP- and cGMP-dependent protein kinase phosphorylation sites.**

amino acids 198-201 and 370-373

**N-glycosylation sites.**

amino acids 39-42, 148-151, 171-174, 234-237, 303-306, 324-327 and 341-344

**G-protein coupled receptors family 2 proteins**

amino acids 475-504

# FIGURE 407

TTGTGACTAAAAGCTGGCCTAGCAGGCCAGGGAGTGCAGCTGCAGGCGTGGGGGTGGCAGGAG
CCGCAGAGCCAGAGCAGACAGCCGAGAAACAGGTGGACAGTGTGAAAGAACCAGTGGTCTCGC
TCTGTTGCCCAGGCTAGAGTGTACTGGCGTGATCATAGCTCACTGCAGCCTCAGACTCCTGGA
CTTGAGAAATCCTCCTGCCTTAGCCTCCTGCATATCTGGGACTCCAGGGGTGCACTCAAGCCC
TGTTTCTTCTCCTTCTGTGAGTGGACCACGGAGGCTGGTGAGCTGCCTGTCATCCCAAAGCTC
AGCTCTGAGCCAGAGTGGTGGTGGCTCCACCTCTGCCGCCGGCATAGAAGCCAGGAGCAGGGC
TCTCAGAAGGCGGTGGTGCCCAGCTGGGATC**ATG**TTGTTGGCCCTGGTCTGTCTGCTCAGCTG
CCTGCTACCCTCCAGTGAGGCCAAGCTCTACGGTCGTTGTGAACTGGCCAGAGTGCTACATGA
CTTCGGGCTGGACGGATACCGGGGATACAGCCTGGCTGACTGGGTCTGCCTTGCTTATTTCAC
AAGCGGTTTCAACGCAGCTGCTTTGGACTACGAGGCTGATGGGAGCACCAACAACGGGATCTT
CCAGATCAACAGCCGGAGGTGGTGCAGCAACCTCACCCCGAACGTCCCCAACGTGTGCCGGAT
GTACTGCTCAGATTTGTTGAATCCTAATCTCAAGGATACCGTTATCTGTGCCATGAAGATAAC
CCAAGAGCCTCAGGGTCTGGGTTACTGGGAGGCCTGGAGGCATCACTGCCAGGGAAAAGACCT
CACTGAATGGGTGGATGGCTGTGACTTC**TAG**GATGGACGGAACCATGCACAGCAGGCTGGGAA
ATGTGGTTTGGTTCCTGACCTAGGCTTGGGAAGACAAGCCAGCGAATAAAGGATGGTTGAACG
TGAAA

# FIGURE 408

MLLALVCLLSCLLPSSEAKLYGRCELARVLHDFGLDGYRGYSLADWVCLAYFTSGFNAAALDY
EADGSTNNGIFQINSRRWCSNLTPNVPNVCRMYCSDLLNPNLKDTVICAMKITQEPQGLGYWE
AWRHHCQGKDLTEWVDGCDF

**Important features:**

**Signal peptide:**

amino acids 1-18

**N-myristoylation site.**

amino acids 67-72

**Homolgous region to Alpha-lactalbumin / lysozyme C proteins.**

amino acids 34-58 (catalytic domain), 111-132 and 66-107

# FIGURE 409

CAGACTCCAGATTTCCCTGTCAACCACGAGGAGTCCAGAGAGGAAACGCGGAGCGGAGACAACAGTACCTGACGC
CTCTTTCAGCCCGGGATCGCCCCAGCAGGG**ATG**GGCGACAAGATCTGGCTGCCCTTCCCCGTGCTCCTTCTGGCC
GCTCTGCCTCCGGTGCTGCTGCCTGGGGCGGCCGGCTTCACACCTTCCCTCGATAGCGACTTCACCTTTACCCTT
CCCGCCGGCCAGAAGGAGTGCTTCTACCAGCCCATGCCCCTGAAGGCCTCGCTGGAGATCGAGTACCAAGTTTTA
GATGGAGCAGGATTAGATATTGATTTCCATCTTGCCTCTCCAGAAGGCAAAACCTTAGTTTTTGAACAAAGAAAA
TCAGATGGAGTTCACACTGTAGAGACTGAAGTTGGTGATTACATGTTCTGCTTTGACAATACATTCAGCACCATT
TCTGAGAAGGTGATTTTCTTTGAATTAATCCTGGATAATATGGGAGAACAGGCACAAGAACAAGAAGATTGGAAG
AAATATATTACTGGCACAGATATATTGGATATGAAACTGGAAGACATCCTGGAATCCATCAACAGCATCAAGTCC
AGACTAAGCAAAAGTGGGCACATACAAATTCTGCTTAGAGCATTTGAAGCTCGTGATCGAAACATACAAGAAAGC
AACTTTGATAGAGTCAATTTCTGGTCTATGGTTAATTTAGTGGTCATGGTGGTGGTGTCAGCCATTCAAGTTTAT
ATGCTGAAGAGTCTGTTTGAAGATAAGAGGAAAAGTAGAACT**TAA**AACTCCAAACTAGAGTACGTAACATTGAAA
AATGAGGCATAAAAATGCAATAAACTGTTACAGTCAAGACCATTAATGGTCTTCTCCAAAATATTTTGAGATATA
AAAGTAGGAAACAGGTATAATTTTAATGTGAAAATTAAGTCTTCACTTTCTGTGCAAGTAATCCTGCTGATCCAG
TTGTACTTAAGTGTGTAACAGGAATATTTTGCAGAATATAGGTTTAACTGAATGAAGCCATATTAATAACTGCAT
TTTCCTAACTTTGAAAAATTTTGCAAATGTCTTAGGTGATTTAAATAAATGAGTATTGGGCCTAATTGCAACACC
AGTCTGTTTTTAACAGGTTCTATTACCCAGAACTTTTTTGTAAATGCGGCAGTTACAAATTAACTGTGGAAGTTT
TCAGTTTTAAGTTATAAATCACCTGAGAATTACCTAATGATGGATTGAATAAATCTTTAGACTACAAAAGCCCAA
CTTTTCTCTATTTACATATGCATCTCTCCTATAATGTAAATAGAATAATAGCTTTGAAATACAATTAGGTTTTTG
AGATTTTTATAACCAAATACATTTCAGTGTAACATATTAGCAGAAAGCATTAGTCTTTGTACTTTGCTTACATTC
CCAAAAGCTGACATTTTCACGATTCTTAAAAACACAAAGTTACACTTACTAAAATTAGGACATGTTTTCTCTTTG
AAATGAAGAATATAGTTTAAAAGCTTCCTCCTCCATAGGGACACATTTTCTCTAACCCTTAACTAAAGTGTAGGA
TTTTAAAATTAAATGTGAGGTAAAATAAGTTTATTTTTAATAGTATCTGTCAAGTTAATATCTGTCAACAGTTAA
TAATCATGTTATGTTAATTTTAACATGATTGCTGACTTGGATAATTCATTATTACCAGCAGTTATGAAGGAAATA
TTGCTAAAATGATCTGGGCCTACCATAAATAAATATCTCCTTTTCTGAGCTCTAAGAATTATCAGAAAACAGGAA
AGAATTTAGAAAAACTTGAGAAAACCTAATCCAAAATAAAATTCACTTAAGTAGAACTATAAATAAATATCTAGA
ATCTGACTGGCTCATCATGACATCCTACTCATAACATAAATCAAAGGAGATGATTAATTTCCAGTTAGCTGGAAG
AAACTTTGGCTGTAGGTTTTTATTTTCTACAAGAATTCTGGTTTGAATTATTTTTGTAAGCAGGTACATTTTATA
AAATGTAAGCCCTACTGTAAGGTTTAGCACTGGGTGTACATATTTATTAAAAATTTTTATTATAACAACTTTTAT
TAAAATGGCCTTTCTGAACACTTTATTTATTGATGTTGAAGTAAGGATTAGAAACATAGACTCCCAAGTTTTAAA
CACCTAAATGTGAATAACCCATATATACAACAAAGTTTCTGCCATCTAGCTTTTTGAAGTCTATGGGGGTCTTAC
TCAAGTACTAGTAATTTAACTTCATCATGAATGAACTATAATTTTTAAGTTATGCCCATTTATAACGTTGTTTAT
GACTACATTGTGAGTTAGAAACAAACTTAAAATTTGGGGTATAGAACCCCTCAACAGGTTAGTAATGCTGGAATT
CTTGATGAGCAATAATGATAACCAGAGAGTGATTTCATTTACACTCATAGTAGTATAAAAAGAGATACATTTCCC
TCTTAGGCCCCTGGGAGAAGAGCAGCTTAGATTTCCCTACTGGCAAGGTTTTTAAAAATGAGGTAAATGCCGTAT
ATGATCAATTACCTTAATTGGCCAAGAAAATGCTTCAGGTGTCTAGGGGTATCCTCTGCAACACTTGCAGAACAA
AGGTCAATAAGATCCTTGCCTATGAATACCCCTCCCTTTTGCGCTGTTAAATTTGCAATGAGAAGCAAATTTACA
GTACCATAACTAATAAAGCAGGGTACAGATATAAACTACTGCATCTTTTCTATAAAACTGTGATTAAGAATTCTA
CCTCTCCTGTATGGCTGTTACTGTACTGTACTCTCTGACTCCTTACCTAACAATGAATTTGTTACATAATCTTCT
ACATGTATGATTTGTGCCACTGATCTTAAACCTATGATTCAGTAACTTCTTACCATATAAAAACGATAATTGCTT
TATTTGGAAAAGAATTTAGGAATACTAAGGACAATTATTTTTATAGACAAAGTAAAAAGACAGATATTTAAGAGG
CATAACCAAAAAAGCAAAACTTGTAAACAGAGTAAAAATCTTTAATATTTCTAAAGACATACTGTTTATCTGCTT
CATATGCTTTTTTTAATTTCACTATTCCATTTCTAAATTAAAGTTATGCTAAATTGAGTAAGCTGTTTATCACTT
AACAGCTCATTTTGTCTTTTTCAATATACAAATTTTAAAAATACTACAATATTTAACTAAGGCCCAACCGATTTC
CATAATGTAGCAGTTACCGTGTTCACCTCACACTAAGGCCTAGAGTTTGCTCTGATATGCATTTGGATGATTAAT
GTTATGCTGTTCTTTCATGTGAATGTCAAGACATGGAGGGTGTTTGTAATTTTATGGTAAAATTAATCCTTCTTA
CACATAATGGTGTCTTAAAATTGACAAAAAATGAGCACTTACAATTGTATGTCTCCTCAAATGAAGATTCTTTAT
GTGAAATTTTAAAAGACATTGATTCCGCATGTAAGGATTTTTCATCTGAAGTACAATAATGCACAATCAGTGTTG
CTCAAACTGCTTTATACTTATAAACAGCCATCTTAAATAAGCAACGTATTGTGAGTACTGATATGTATATAATAA
AAATTATCAAAGGAAAA

# FIGURE 410

MGDKIWLPFPVLLLAALPPVLLPGAAGFTPSLDSDFTFTLPAGQKECFYQPMPLKASLEIEYQ
VLDGAGLDIDFHLASPEGKTLVFEQRKSDGVHTVETEVGDYMFCFDNTFSTISEKVIFFELIL
DNMGEQAQEQEDWKKYITGTDILDMKLEDILESINSIKSRLSKSGHIQILLRAFEARDRNIQE
SNFDRVNFWSMVNLVVMVVVSAIQVYMLKSLFEDKRKSRT

**Important features:**

**Signal peptide:**

amino acids 1-23

**Transmembrane domain:**

amino acids 195-217

**N-myristoylation site.**

amino acids 43-48

**Tyrosine kinase phosphorylation site.**

amino acids 55-62

# FIGURE 411

CCCAGCTGAGGAGCCCTGCTCAAGACACGGTCACTGGATCTGAGAAACTTCCCAGGGGACCGCATTCCAGAGTCA
GTGACTCTGTGAAGCACCCACATCTACCTCTTGCCACGTTCCCACGGGCTTGGGGGAAAG**ATG**GTGGGGACCAAG
GCCTGGGTGTTCTCCTTCCTGGTCCTGGAAGTCACATCTGTGTTGGGGAGACAGACGATGCTCACCCAGTCAGTA
AGAAGAGTCCAGCCTGGGAAGAAGAACCCCAGCATCTTTGCCAAGCCTGCCGACACCCTGGAGAGCCCTGGTGAG
TGGACAACATGGTTCAACATCGACTACCCAGGCGGGAAGGGCGACTATGAGCGGCTGGACGCCATTCGCTTCTAC
TATGGGGACCGTGTATGTGCCCGTCCCCTGCGGCTAGAGGCTCGGACCACTGACTGGACACCTGCGGGCAGCACT
GGCCAGGTGGTCCATGGTAGTCCCCGTGAGGGTTTCTGGTGCCTCAACAGGGAGCAGCGGCCTGGCCAGAACTGC
TCTAATTACACCGTACGCTTCCTCTGCCCACCAGGATCCCTGCGCCGAGACACAGAGCGCATCTGGAGCCCATGG
TCTCCCTGGAGCAAGTGCTCAGCTGCCTGTGGTCAGACTGGGGTCCAGACTCGCACACGCATTTGCTTGGCAGAG
ATGGTGTCGCTGTGCAGTGAGGCCAGCGAAGAGGGTCAGCACTGCATGGGCCAGGACTGTACAGCCTGTGACCTG
ACCTGCCCAATGGGCCAGGTGAATGCTGACTGTGATGCCTGCATGTGCCAGGACTTCATGCTTCATGGGGCTGTC
TCCCTTCCCGGAGGTGCCCCAGCCTCAGGGGCTGCTATCTACCTCCTGACCAAGACGCCGAAGCTGCTGACCCAG
ACAGACAGTGATGGGAGATTCCGAATCCCTGGCTTGTGCCCTGATGGCAAAAGCATCCTGAAGATCACAAAGGTC
AAGTTTGCCCCCATTGTACTCACAATGCCCAAGACTAGCCTGAAGGCAGCCACCATCAAGGCAGAGTTTGTGAGG
GCAGAGACTCCATACATGGTGATGAACCCTGAGACAAAAGCACGGAGAGCTGGGCAGAGCGTGTCTCTGTGCTGT
AAGGCCACAGGGAAGCCCAGGCCAGACAAGTATTTTTGGTATCATAATGACACATTGCTGGATCCTTCCCTCTAC
AAGCATGAGAGCAAGCTGGTGCTGAGGAAACTGCAGCAGCACCAGGCTGGGGAGTACTTTTGCAAGGCCCAGAGT
GATGCTGGGGCTGTGAAGTCCAAGGTTGCCCAGCTGATTGTCACAGCATCTGATGAGACTCCTTGCAACCCAGTT
CCTGAGAGCTATCTTATCCGGCTGCCCCATGATTGCTTTCAGAATGCCACCAACTCCTTCTACTATGACGTGGGA
CGCTGCCCTGTTAAGACTTGTGCAGGGCAGCAGGATAATGGGATCAGGTGCCGTGATGCTGTGCAGAACTGCTGT
GGCATCTCCAAGACAGAGGAAAGGGAGATCCAGTGCAGTGGCTACACGCTACCCACCAAGGTGGCCAAGGAGTGC
AGCTGCCAGCGGTGTACGGAAACTCGGAGCATCGTGCGGGGCCGTGTCAGTGCTGCTGACAATGGGGAGCCCATG
CGCTTTGGCCATGTGTACATGGGGAACAGCCGTGTAAGCATGACTGGCTACAAGGGCACTTTCACCCTCCATGTC
CCCCAGGACACTGAGAGGCTGGTGCTCACATTTGTGGACAGGCTGCAGAAGTTTGTCAACACCACCAAAGTGCTA
CCTTTCAACAAGAAGGGGAGTGCCGTGTTCCATGAAATCAAGATGCTTCGTCGGAAAGAGCCCATCACTTTGGAA
GCCATGGAGACCAACATCATCCCCCTGGGGGAAGTGGTTGGTGAAGACCCCATGGCTGAACTGGAGATTCCATCC
AGGAGTTTCTACAGGCAGAATGGGGAGCCCTACATAGGAAAAGTGAAGGCCAGTGTGACCTTCCTGGATCCCCGG
AATATTTCCACAGCCACAGCTGCCCAGACTGACCTGAACTTCATCAATGACGAAGGAGACACTTTCCCCCTTCGG
ACGTATGGCATGTTCTCTGTGGACTTCAGAGATGAGGTCACCTCAGAGCCACTTAATGCTGGCAAAGTGAAGGTC
CACCTTGACTCGACCCAGGTCAAGATGCCAGAGCACATATCCACAGTGAAACTCTGGTCACTCAATCCAGACACA
GGGCTGTGGGAGGAGGAAGGTGATTTCAAATTTGAAAATCAAAGGAGGAACAAAAGAGAAGACAGAACCTTCCTG
GTGGGCAACCTGGAGATTCGTGAGAGGAGGCTCTTTAACCTGGATGTTCCTGAAAGCAGGCGGTGCTTTGTTAAG
GTGAGGGCCTACCGGAGTGAGAGGTTCTTGCCTAGTGAGCAGATCCAGGGGGTTGTGATCTCCGTGATTAACCTG
GAGCCTAGAACTGGCTTCTTGTCCAACCCTAGGGCCTGGGGCCGCTTTGACAGTGTCATCACAGGCCCCAACGGG
GCCTGTGTGCCTGCCTTCTGTGATGACCAGTCCCCTGATGCCTACTCTGCCTATGTCTTGGCAAGCCTGGCTGGG
GAGGAACTGCAAGCAGTGGAGTCTTCTCCTAAATTCAACCCAAATGCAATTGGCGTCCCTCAGCCCTATCTCAAC
AAGCTCAACTACCGTCGGACGGACCATGAGGATCCACGGGTTAAAAAGACAGCTTTCCAGATTAGCATGGCCAAG
CCAAGGCCCAACTCAGCTGAGGAGAGCAATGGGCCCATCTATGCCTTTGAGAACCTCCGGGCATGTGAAGAGGCA
CCACCCAGTGCAGCCCACTTCCGGTTCTACCAGATTGAGGGGGATCGATATGACTACAACACAGTCCCCTTCAAC
GAAGATGACCCTATGAGCTGGACTGAAGACTATCTGGCATGGTGGCCAAAGCCGATGGAATTCAGGGCCTGCTAT
ATCAAGGTGAAGATTGTGGGGCCACTGGAAGTGAATGTGCGATCCCGCAACATGGGGGGCACTCATCGGCGGACA
GTGGGGAAGCTGTATGGAATCCGAGATGTGAGGAGCACTCGGGACAGGGACCAGCCCAATGTCTCAGCTGCCTGT
CTGGAGTTCAAGTGCAGTGGGATGCTCTATGATCAGGACCGTGTGGACCGCACCCTGGTGAAGGTCATCCCCCAG
GGCAGCTGCCGTCGAGCCAGTGTGAACCCCATGCTGCATGAGTACCTGGTCAACCACTTGCCACTTGCAGTCAAC
AACGACACCAGTGAGTACACCATGCTGGCACCCTTGGACCCACTGGGCCACAACTATGGCATCTACACTGTCACT
GACCAGGACCCTCGCACGGCCAAGGAGATCGCGCTCGGCCGGTGCTTTGATGGCACATCCGATGGCTCCTCCAGA
ATCATGAAGAGCAATGTGGGAGTAGCCCTCACCTTCAACTGTGTAGAGAGGCAAGTAGGCCGCCAGAGTGCCTTC
CAGTACCTCCAAAGCACCCCAGCCCAGTCCCCTGCTGCAGGCACTGTCCAAGGAAGAGTGCCCTCGAGGAGGCAG
CAGCGAGCGAGCAGGGGTGGCCAGCGCCAGGGTGGAGTGGTGGCCTCTCTGAGATTTCCTAGAGTTGCTCAACAG
CCCCTGATCAAC**TAA**GTTTTGTGGTACTTCACCCTCTTCTGCCCTCATTTCATGTGACAGCCATTGTGAGACTGA
TGCACAAACTGTCACTTGGTTAATTTAAGCACTTCTGTTTCGTGAATTTGCTTGTTTGTTTCTTCATGCCTTTA
CTTACTTTGTCCCATGCTACTGATTGGCACGTGGCCCCACAATGGCACAATAAAGCCCCTTTGTGAAACTGTTC
TTTAAATGAAACACAAGAAATTGGCCACTGGTAAAACTCTGCAGCTTCAACTGTACTTCATTTAATGCCATTAAT
GCAAATATACTTCCTCTTCTTTTTGCATGGTTTTGCCCACCTCTGCAATAGTGATAATCTGATGCTGAAGATCAA
ATAACCAATATAAAGCATATTTCTTGGCCTTGCTCCACAGGACATAGGCAAGCCTTGATCATAGTTCATACATAT
AAATGGTGGTGAAATAAAGAAATAAAACACAATACTTTTACTTGAAATGTAAATAACTTATTTATTTCTTTGCTA
AATTTGGAATTCTAGTGCACATTCAAAGTTAAGCTATTAAATATAGGGTGATCATAGTTCCTCTACCAAGTCTGG
AAAGAACATCTCCTGGTATCCACAATTACACCAGGTTGCTAACTGTATTTGTACATTTCCCTTTGCATTCGCTTT
TGTTCTTGCTAGAAACCCAGTGTAGCCCAGGGCAGATGTCAATAAATGCATACTCTGTATTTCGAAAAAA

# FIGURE 412

MVGTKAWVFSFLVLEVTSVLGRQTMLTQSVRRVQPGKKNPSIFAKPADTLESPGEWTTWFNID
YPGGKGDYERLDAIRFYYGDRVCARPLRLEARTTDWTPAGSTGQVVHGSPREGFWCLNREQRP
GQNCSNYTVRFLCPPGSLRRDTERIWSPWSPWSKCSAACGQTGVQTRTRICLAEMVSLCSEAS
EEGQHCMGQDCTACDLTCPMGQVNADCDACMCQDFMLHGAVSLPGGAPASGAAIYLLTKTPKL
LTQTDSDGRFRIPGLCPDGKSILKITKVKFAPIVLTMPKTSLKAATIKAEFVRAETPYMVMNP
ETKARRAGQSVSLCCKATGKPRPDKYFWYHNDTLLDPSLYKHESKLVLRKLQQHQAGEYFCKA
QSDAGAVKSKVAQLIVTASDETPCNPVPESYLIRLPHDCFQNATNSFYYDVGRCPVKTCAGQQ
DNGIRCRDAVQNCCGISKTEEREIQCSGYTLPTKVAKECSCQRCTETRSIVRGRVSAADNGEP
MRFGHVYMGNSRVSMTGYKGTFTLHVPQDTERLVLTFVDRLQKFVNTTKVLPFNKKGSAVFHE
IKMLRRKEPITLEAMETNIIPLGEVVGEDPMAELEIPSRSFYRQNGEPYIGKVKASVTFLDPR
NISTATAAQTDLNFINDEGDTFPLRTYGMFSVDFRDEVTSEPLNAGKVKVHLDSTQVKMPEHI
STVKLWSLNPDTGLWEEEGDFKFENQRRNKREDRTFLVGNLEIRERRLFNLDVPESRRCFVKV
RAYRSERFLPSEQIQGVVISVINLEPRTGFLSNPRAWGRFDSVITGPNGACVPAFCDDQSPDA
YSAYVLASLAGEELQAVESSPKFNPNAIGVPQPYLNKLNYRRTDHEDPRVKKTAFQISMAKPR
PNSAEESNGPIYAFENLRACEEAPPSAAHFRFYQIEGDRYDYNTVPFNEDDPMSWTEDYLAWW
PKPMEFRACYIKVKIVGPLEVNVRSRNMGGTHRRTVGKLYGIRDVRSTRDRDQPNVSAACLEF
KCSGMLYDQDRVDRTLVKVIPQGSCRRASVNPMLHEYLVNHLPLAVNNDTSEYTMLAPLDPLG
HNYGIYTVTDQDPRTAKEIALGRCFDGTSDGSSRIMKSNVGVALTFNCVERQVGRQSAFQYLQ
STPAQSPAAGTVQGRVPSRRQQRASRGGQRQGGVVASLRFPRVAQQPLIN

# FIGURE 413

GCCACGTTGTCTTCTTTCCTTCACCACCACCCAGGAGCTCAGAGATCTAAGCTGCTTTCCATC
TTTTCTCCCAGCCCCAGGACACTGACTCTGTACAGG**ATG**GGGCCGTCCTCTTGCCTCCTTCTC
ATCCTAATCCCCCTTCTCCAGCTGATCAACCCGGGGAGTACTCAGTGTTCCTTAGACTCCGTT
ATGGATAAGAAGATCAAGGATGTTCTCAACAGTCTAGAGTACAGTCCCTCTCCTATAAGCAAG
AAGCTCTCGTGTGCTAGTGTCAAAAGCCAAGGCAGACCGTCCTCCTGCCCTGCTGGGATGGCT
GTCACTGGCTGTGCTTGTGGCTATGGCTGTGGTTCGTGGGATGTTCAGCTGGAAACCACCTGC
CACTGCCAGTGCAGTGTGGTGGACTGGACCACTGCCCGCTGCTGCCACCTGACC**TGA**CAGGGA
GGAGGCTGAGAACTCAGTTTTGTGACCATGACAGTAATGAAACCAGGGTCCCAACCAAGAAAT
CTAACTCAAACGTCCCACTTCATTTGTTCCATTCCTGATTCTTGGGTAATAAAGACAAACTTT
GTACCTCAAAAAAAAAAAAAAAAAAAAAA

# FIGURE 414

MGPSSCLLLILIPLLQLINPGSTQCSLDSVMDKKIKDVLNSLEYSPSPISKKLSCASVKS
QGRPSSCPAGMAVTGCACGYGCGSWDVQLETTCHCQCSVVDWTTARCCHLT

# FIGURE 415

CAGAAGAGGGGGCTAGCTAGCTGTCTCTGCGGACCAGGGAGACCCCCGCGCCCCCCCGGTGTG
AGGCGGCCTCACAGGGCCGGGTGGGCTGGCGAGCCGACGCGGCGGCGGAGGAGGCTGTGAGGA
GTGTGTGGAACAGGACCCGGGACAGAGGAACC**ATG**GCTCCGCAGAACCTGAGCACCTTTTGCC
TGTTGCTGCTATACCTCATCGGGGCGGTGATTGCCGGACGAGATTTCTATAAGATCTTGGGGG
TGCCTCGAAGTGCCTCTATAAAGGATATTAAAAAGGCCTATAGGAAACTAGCCCTGCAGCTTC
ATCCCGACCGGAACCCTGATGATCCACAAGCCCAGGAGAAATTCCAGGATCTGGGTGCTGCTT
ATGAGGTTCTGTCAGATAGTGAGAAACGGAAACAGTACGATACTTATGGTGAAGAAGGATTAA
AAGATGGTCATCAGAGCTCCCATGGAGACATTTTTTCACACTTCTTTGGGGATTTTGGTTTCA
TGTTTGGAGGAACCCCTCGTCAGCAAGACAGAAATATTCCAAGAGGAAGTGATATTATTGTAG
ATCTAGAAGTCACTTTGGAAGAAGTATATGCAGGAAATTTTGTGGAAGTAGTTAGAAACAAAC
CTGTGGCAAGGCAGGCTCCTGGCAAACGGAAGTGCAATTGTCGGCAAGAGATGCGGACCACCC
AGCTGGGCCCTGGGCGCTTCCAAATGACCCAGGAGGTGGTCTGCGACGAATGCCCTAATGTCA
AACTAGTGAATGAAGAACGAACGCTGGAAGTAGAAATAGAGCCTGGGGTGAGAGACGGCATGG
AGTACCCCTTTATTGGAGAAGGTGAGCCTCACGTGGATGGGGAGCCTGGAGATTTACGGTTCC
GAATCAAAGTTGTCAAGCACCCAATATTTGAAAGGAGAGGAGATGATTTGTACACAAATGTGA
CAATCTCATTAGTTGAGTCACTGGTTGGCTTTGAGATGGATATTACTCACTTGGATGGTCACA
AGGTACATATTTCCCGGGATAAGATCACCAGGCCAGGAGCGAAGCTATGGAAGAAAGGGGAAG
GGCTCCCCAACTTTGACAACAACAATATCAAGGGCTCTTTGATAATCACTTTTGATGTGGATT
TTCCAAAAGAACAGTTAACAGAGGAAGCGAGAGAAGGTATCAAACAGCTACTGAAACAAGGGT
CAGTGCAGAAGGTATACAATGGACTGCAAGGATAT**TGA**GAGTGAATAAAATTGGACTTTGTTT
AAAATAAGTGAATAAGCGATATTTATTATCTGCAAGGTTTTTTGTGTGTGTTTTTGTTTTTA
TTTTCAATATGCAAGTTAGGCTTAATTTTTTTATCTAATGATCATCATGAAATGAATAAGAGG
GCTTAAGAATTTGTCCATTTGCATTCGGAAAAGAATGACCAGCAAAAGGTTTACTAATACCTC
TCCCTTTGGGGATTTAATGTCTGGTGCTGCCGCCTGAGTTTCAAGAATTAAAGCTGCAAGAGG
ACTCCAGGAGCAAAAGAAACACAATATAGAGGGTTGGAGTTGTTAGCAATTTCATTCAAAATG
CCAACTGGAGAAGTCTGTTTTTAAATACATTTTGTTGTTATTTTTA

# FIGURE 416

MAPQNLSTFCLLLLYLIGAVIAGRDFYKILGVPRSASIKDIKKAYRKLALQLHPDRNPDDPQAQEKFQDLGAAYE
VLSDSEKRKQYDTYGEEGLKDGHQSSHGDIFSHFFGDFGFMFGGTPRQQDRNIPRGSDIIVDLEVTLEEVYAGNF
VEVVRNKPVARQAPGKRKCNCRQEMRTTQLGPGRFQMTQEVVCDECPNVKLVNEERTLEVEIEPGVRDGMEYPFI
GEGEPHVDGEPGDLRFRIKVVKHPIFERRGDDLYTNVTISLVESLVGFEMDITHLDGHKVHISRDKITRPGAKLW
KKGEGLPNFDNNNIKGSLIITFDVDFPKEQLTEEAREGIKQLLKQGSVQKVYNGLQGY

**Important features:**

**Signal peptide:**

amino acids 1-22

**Cell attachment sequence.**

amino acids 254-257

**Nt-dnaJ domain signature.**

amino acids 67-87

**Homologous region to Nt-dnaJ domain proteins.**

amino acids 26-58

**N-glycosylation site.**

amino acids 5-9, 261-265

**Tyrosine kinase phosphorylation site.**

amino acids 253-260

**N-myristoylation site.**

amino acids 18-24, 31-37, 93-99, 215-221

**Amidation site.**

amino acids 164-168

# FIGURE 417

CGGCGGCGGCTGCGGGCGCGAGGTGAGGGGCGCGAGGTGAGGGGCGCGAGGTTCCCAGCAGGA
TGCCCCGGCTCTGCAGGAAGCTGAAGTGAGAGGCCCGGAGAGGGCCCAGCCCGCCCGGGGCAG
G**ATG**ACCAAGGCCCGGCTGTTCCGGCTGTGGCTGGTGCTGGGGTCGGTGTTCATGATCCTGCT
GATCATCGTGTACTGGGACAGCGCAGGCGCCGCGCACTTCTACTTGCACACGTCCTTCTCTAG
GCCGCACACGGGGCCGCCGCTGCCCACGCCCGGGCCGGACAGGGACAGGGAGCTCACGGCCGA
CTCCGATGTCGACGAGTTTCTGGACAAGTTTCTCAGTGCTGGCGTGAAGCAGAGCGACCTTCC
CAGAAAGGAGACGGAGCAGCCGCCTGCGCCGGGGAGCATGGAGGAGAGCGTGAGAGGCTACGA
CTGGTCCCCGCGCGACGCCCGGCGCAGCCCAGACCAGGGCCGGCAGCAGGCGGAGCGGAGGAG
CGTGCTGCGGGGCTTCTGCGCCAACTCCAGCCTGGCCTTCCCCACCAAGGAGCGCGCATTCGA
CGACATCCCCAACTCGGAGCTGAGCCACCTGATCGTGGACGACCGGCACGGGGCCATCTACTG
CTACGTGCCCAAGGTGGCCTGCACCAACTGGAAGCGCGTGATGATCGTGCTGAGCGGAAGCCT
GCTGCACCGCGGTGCGCCCTACCGCGACCCGCTGCGCATCCCGCGCGAGCACGTGCACAACGC
CAGCGCGCACCTGACCTTCAACAAGTTCTGGCGCCGCTACGGGAAGCTCTCCCGCCACCTCAT
GAAGGTCAAGCTCAAGAAGTACACCAAGTTCCTCTTCGTGCGCGACCCCTTCGTGCGCCTGAT
CTCCGCCTTCCGCAGCAAGTTCGAGCTGGAGAACGAGGAGTTCTACCGCAAGTTCGCCGTGCC
CATGCTGCGGCTGTACGCCAACCACACCAGCCTGCCCGCCTCGGCGCGCGAGGCCTTCCGCGC
TGGCCTCAAGGTGTCCTTCGCCAACTTCATCCAGTACCTGCTGGACCCGCACACGGAGAAGCT
GGCGCCCTTCAACGAGCACTGGCGGCAGGTGTACCGCCTCTGCCACCCGTGCCAGATCGACTA
CGACTTCGTGGGGAAGCTGGAGACTCTGGACGAGGACGCCGCGCAGCTGCTGCAGCTACTCCA
GGTGGACCGGCAGCTCCGCTTCCCCCCGAGCTACCGGAACAGGACCGCCAGCAGCTGGGAGGA
GGACTGGTTCGCCAAGATCCCCCTGGCCTGGAGGCAGCAGCTGTATAAACTCTACGAGGCCGA
CTTTGTTCTCTTCGGCTACCCCAAGCCCGAAAACCTCCTCCGAGAC**TGA**AAGCTTTCGCGTTG
CTTTTTCTCGCGTGCCTGGAACCTGACGCACGCGCACTCCAGTTTTTTATGACCTACGATTT
TGCAATCTGGGCTTCTTGTTCACTCCACTGCCTCTATCCATTGAGTACTGTATCGATATTGTT
TTTTAAGATTAATATATTTCAGGTATTTAATACGA

# FIGURE 418

MTKARLFRLWLVLGSVFMILLIIVYWDSAGAAHFYLHTSFSRPHTGPPLPTPGPDRDRELTAD
SDVDEFLDKFLSAGVKQSDLPRKETEQPPAPGSMEESVRGYDWSPRDARRSPDQGRQQAERRS
VLRGFCANSSLAFPTKERAFDDIPNSELSHLIVDDRHGAIYCYVPKVACTNWKRVMIVLSGSL
LHRGAPYRDPLRIPREHVHNASAHLTFNKFWRRYGKLSRHLMKVKLKKYTKFLFVRDPFVRLI
SAFRSKFELENEEFYRKFAVPMLRLYANHTSLPASAREAFRAGLKVSFANFIQYLLDPHTEKL
APFNEHWRQVYRLCHPCQIDYDFVGKLETLDEDAAQLLQLLQVDRQLRFPPSYRNRTASSWEE
DWFAKIPLAWRQQLYKLYEADFVLFGYPKPENLLRD

**Important features:**

**Signal peptide:**

amino acids 1-31

**N-glycosylation sites.**

amino acids 134-137, 209-212, 280-283 and 370-373

**TNFR/NGFR family cysteine-rich region protein**

amino acids 329-332

# FIGURE 419

GGCACGAGGCTGAACCCAGCCGGCTCCATCTCAGCTTCTGGTTTCTAAGTCCATGTGCCAAAG
GCTGCCAGGAAGGAGACGCCTTCCTGAGTCCTGGATCTTTCTTCCTTCTGGAAATCTTTGACT
GTGGGTAGTTATTTATTTCTGAATAAGAGCGTCCACGCATC**ATG**GACCTCGCGGGACTGCTGA
AGTCTCAGTTCCTGTGCCACCTGGTCTTCTGCTACGTCTTTATTGCCTCAGGGCTAATCATCA
ACACCATTCAGCTCTTCACTCTCCTCCTCTGGCCCATTAACAAGCAGCTCTTCCGGAAGATCA
ACTGCAGACTGTCCTATTGCATCTCAAGCCAGCTGGTGATGCTGCTGGAGTGGTGGTCGGGCA
CGGAATGCACCATCTTCACGGACCCGCGCGCCTACCTCAAGTATGGGAAGGAAAATGCCATCG
TGGTTCTCAACCACAAGTTTGAAATTGACTTTCTGTGTGGCTGGAGCCTGTCCGAACGCTTTG
GGCTGTTAGGGGGCTCCAAGGTCCTGGCCAAGAAAGAGCTGGCCTATGTCCCAATTATCGGCT
GGATGTGGTACTTCACCGAGATGGTCTTCTGTTCGCGCAAGTGGGAGCAGGATCGCAAGACGG
TTGCCACCAGTTTGCAGCACCTCCGGGACTACCCCGAGAAGTATTTTTTCCTGATTCACTGTG
AGGGCACACGGTTCACGGAGAAGAAGCATGAGATCAGCATGCAGGTGGCCCGGGCCAAGGGGC
TGCCTCGCCTCAAGCATCACCTGTTGCCACGAACCAAGGGCTTCGCCATCACCGTGAGGAGCT
TGAGAAATGTAGTTTCAGCTGTATATGACTGTACACTCAATTTCAGAAATAATGAAAATCCAA
CACTGCTGGGAGTCCTAAACGGAAAGAAATACCATGCAGATTTGTATGTTAGGAGGATCCCAC
TGGAAGACATCCCTGAAGACGATGACGAGTGCTCGGCCTGGCTGCACAAGCTCTACCAGGAGA
AGGATGCCTTTCAGGAGGAGTACTACAGGACGGGCACCTTCCCAGAGACGCCCATGGTGCCCC
CCCGGCGGCCCTGGACCCTCGTGAACTGGCTGTTTTGGGCCTCGCTGGTGCTCTACCCTTTCT
TCCAGTTCCTGGTCAGCATGATCAGGAGCGGGTCTTCCCTGACGCTGGCCAGCTTCATCCTCG
TCTTCTTTGTGGCCTCCGTGGGAGTTCGATGGATGATTGGTGTGACGGAAATTGACAAGGGCT
CTGCCTACGGCAACTCTGACAGCAAGCAGAAACTGAATGAC**TGA**CTCAGGGAGGTGTCACCAT
CCGAAGGGAACCTTGGGGAACTGGTGGCCTCTGCATATCCTCCTTAGTGGGACACGGTGACAA
AGGCTGGGTGAGCCCCTGCTGGGCACGGCGGAAGTCACGACCTCTCCAGCCAGGGAGTCTGGT
CTCAAGGCCGGATGGGGAGGAAGATGTTTTGTAATCTTTTTTTCCCCATGTGCTTTAGTGGGC
TTTGGTTTTCTTTTTGTGCGAGTGTGTGTGAGAATGGCTGTGTGGTGAGTGTGAACTTTGTTC
TGTGATCATAGAAAGGGTATTTAGGCTGCAGGGGAGGGCAGGGCTGGGGACCGAAGGGGACA
AGTTCCCCTTTCATCCTTTGGTGCTGAGTTTTCTGTAACCCTTGGTTGCCAGAGATAAAGTGA
AAAGTGCTTTAGGTGAGATGACTAAATTATGCCTCCAAGAAAAAAAAATTAAAGTGCTTTTCT
GGGTCAAAAAAAAAAAA

# FIGURE 420

MDLAGLLKSQFLCHLVFCYVFIASGLIINTIQLFTLLLWPINKQLFRKINCRLSYCISSQLVM
LLEWWSGTECTIFTDPRAYLKYGKENAIVVLNHKFEIDFLCGWSLSERFGLLGGSKVLAKKEL
AYVPIIGWMWYFTEMVFCSRKWEQDRKTVATSLQHLRDYPEKYFFLIHCEGTRFTEKKHEISM
QVARAKGLPRLKHHLLPRTKGFAITVRSLRNVVSAVYDCTLNFRNNENPTLLGVLNGKKYHAD
LYVRRIPLEDIPEDDDECSAWLHKLYQEKDAFQEEYYRTGTFPETPMVPPRRPWTLVNWLFWA
SLVLYPFFQFLVSMIRSGSSLTLASFILVFFVASVGVRWMIGVTEIDKGSAYGNSDSKQKLND

# FIGURE 421

CGGACGCGTGGGCGGACGCGTGGGCGGACGCGTGGGCGGACGCGTGGGCTGGGTGCCTGCATC
GCC**ATG**GACACCACCAGGTACAGCAAGTGGGGCGGCAGCTCCGAGGAGGTCCCCGGAGGGCCC
TGGGGACGCTGGGTGCACTGGAGCAGGAGACCCCTCTTCTTGGCCCTGGCTGTCCTGGTCACC
ACAGTCCTTTGGGCTGTGATTCTGAGTATCCTATTGTCCAAGGCCTCCACGGAGCGCGCGGCG
CTGCTTGACGGCCACGACCTGCTGAGGACAAACGCCTCGAAGCAGACGGCGGCGCTGGGTGCC
CTGAAGGAGGAGGTCGGAGACTGCCACAGCTGCTGCTCGGGGACGCAGGCGCAGCTGCAGACC
ACGCGCGCGGAGCTTGGGGAGGCGCAGGCGAAGCTGATGGAGCAGGAGAGCGCCCTGCGGGAA
CTGCGTGAGCGCGTGACCCAGGGCTTGGCTGAAGCCGGCAGGGGCCGTGAGGACGTCCGCACT
GAGCTGTTCCGGGCGCTGGAGGCCGTGAGGCTCCAGAACAACTCCTGCGAGCCGTGCCCCACG
TCGTGGCTGTCCTTCGAGGGCTCCTGCTACTTTTTCTCTGTGCCAAAGACGACGTGGGCGGCG
GCGCAGGATCACTGCGCAGATGCCAGCGCGCACCTGGTGATCGTTGGGGGCCTGGATGAGCAG
GGCTTCCTCACTCGGAACACGCGTGGCCGTGGTTACTGGCTGGGCCTGAGGGCTGTGCGCCAT
CTGGGCAAGGTTCAGGGCTACCAGTGGGTGGACGGAGTCTCTCTCAGCTTCAGCCACTGGAAC
CAGGGAGAGCCCAATGACGCTTGGGGGCGCGAGAACTGTGTCATGATGCTGCACACGGGGCTG
TGGAACGACGCACCGTGTGACAGCGAGAAGGACGGCTGGATCTGTGAGAAAAGGCACAACTGC
**TGA**CCCCGCCCAGTGCCCTGGAGCCGCGCCCATTGCAGCATGTCGTATCCTGGGGGCTGCTCA
CCTCCCTGGCTCCTGGAGCTGATTGCCAAAGAGTTTTTTTCTTCCTCATCCACCGCTGCTGAG
TCTCAGAAACACTTGGCCCAACATAGCCCTGTCCAGCCCAGTGCCTGGGCTCTGGGACCTCCA
TGCCGACCTCATCCTAACTCCACTCACGCAGACCCAACCTAACCTCCACTAGCTCCAAAATCC
CTGCTCCTGCGTCCCCGTGATATGCCTCCACTTCTCTCCCTAACCAAGGTTAGGTGACTGAGG
ACTGGAGCTGTTTGGTTTTCTCGCATTTTCCACCAAACTGGAAGCTGTTTTTGCAGCCTGAGG
AAGCATCAATAAATATTTGAGAAATGAAAAAA

# FIGURE 422

MDTTRYSKWGGSSEEVPGGPWGRWVHWSRRPLFLALAVLVTTVLWAVILSILLSKASTERAAL
LDGHDLLRTNASKQTAALGALKEEVGDCHSCCSGTQAQLQTTRAELGEAQAKLMEQESALREL
RERVTQGLAEAGRGREDVRTELFRALEAVRLQNNSCEPCPTSWLSFEGSCYFFSVPKTTWAAA
QDHCADASAHLVIVGGLDEQGFLTRNTRGRGYWLGLRAVRHLGKVQGYQWVDGVSLSFSHWNQ
GEPNDAWGRENCVMMLHTGLWNDAPCDSEKDGWICEKRHNC

**Important features:**

**Type II transmembrane domain:**

amino acids 31-54

**N-glycosylation sites.**

amino acids 73-76 and 159-162

**Leucine zipper pattern.**

amino acids 102-123

**N-myristoylation sites.**

amino acids 18-23, 133-138 and 242-247

**C-type lectin domain signature.**

amino acids 264-287

# FIGURE 423

GCGCCGCCAGGCGTAGGCGGGGTGGCCCTTGCGTCTCCCGCTTCCTTGAAAAACCCGGCGGGC
GAGCGAGGCTGCGGGCCGGCCGCTGCCCTTCCCCACACTCCCCGCCGAGAAGCCTCGCTCGGC
GCCCAACATGGCGGGTGGGCGCTGCGGCCCGCAGCTAACGGCGCTCCTGGCCGCCTGGATCGC
GGCTGTGGCGGCGACGGCAGGCCCCGAGGAGGCCGCGCTGCCGCCGGAGCAGAGCCGGGTCCA
GCCCATGACCGCCTCCAACTGGACGCTGGTGATGGAGGGCGAGTGGATGCTGAAATTTTACGC
CCCATGGTGTCCATCCTGCCAGCAGACTGATTCAGAATGGGAGGCTTTTGCAAAGAATGGTGA
AATACTTCAGATCAGTGTGGGGAAGGTAGATGTCATTCAAGAACCAGGTTTGAGTGGCCGCTT
CTTTGTCACCACTCTCCCAGCATTTTTTCATGCAAAGGATGGGATATTCCGCCGTTATCGTGG
CCCAGGAATCTTCGAAGACCTGCAGAATTATATCTTAGAGAAGAAATGGCAATCAGTCGAGCC
TCTGACTGGCTGGAAATCCCCAGCTTCTCTAACGATGTCTGGAATGGCTGGTCTTTTTAGCAT
CTCTGGCAAGATATGGCATCTTCACAACTATTTCACAGTGACTCTTGGAATTCCTGCTTGGTG
TTCTTATGTGTTTTTCGTCATAGCCACCTTGGTTTTTGGCCTTTTTATGGGTCTGGTCTTGGT
GGTAATATCAGAATGTTTCTATGTGCCACTTCCAAGGCATTTATCTGAGCGTTCTGAGCAGAA
TCGGAGATCAGAGGAGGCTCATAGAGCTGAACAGTTGCAGGATGCGGAGGAGGAAAAAGATGA
TTCAAATGAAGAAGAAAACAAAGACAGCCTTGTAGATGATGAAGAAGAGAAAGAAGATCTTGG
CGATGAGGATGAAGCAGAGGAAGAAGAGGAGGAGGACAACTTGGCTGCTGGTGTGGATGAGGA
GAGAAGTGAGGCCAATGATCAGGGGCCCCCAGGAGAGGACGGTGTGACCCGGGAGGAAGTAGA
GCCTGAGGAGGCTGAAGAAGGCATCTCTGAGCAACCCTGCCCAGCTGACACAGAGGTGGTGGA
AGACTCCTTGAGGCAGCGTAAAAGTCAGCATGCTGACAAGGGACTGTAGATTTAATGATGCGT
TTTCAAGAATACACACCAAAACAATATGTCAGCTTCCCTTTGGCCTGCAGTTTGTACCAAATC
CTTAATTTTTCCTGAATGAGCAAGCTTCTCTTAAAAGATGCTCTCTAGTCATTTGGTCTCATG
GCAGTAAGCCTCATGTATACTAAGGAGAGTCTTCCAGGTGTGACAATCAGGATATAGAAAAAC
AAACGTAGTGTTGGGATCTGTTTGGAGACTGGGATGGGAACAAGTTCATTTACTTAGGGGTCA
GAGAGTCTCGACCAGAGGAGGCCATTCCCAGTCCTAATCAGCACCTTCCAGAGACAAGGCTGC
AGGCCCTGTGAAATGAAAGCCAAGCAGGAGCCTTGGCTCCTGAGCATCCCCAAAGTGTAACGT
AGAAGCCTTGCATCCTTTTCTTGTGTAAAGTATTTATTTTTGTCAAATTGCAGGAAACATCAG
GCACCACAGTGCATGAAAAATCTTTCACAGCTAGAAATTGAAAGGGCCTTGGGTATAGAGAGC
AGCTCAGAAGTCATCCCAGCCCTCTGAATCTCCTGTGCTATGTTTTATTTCTTACCTTTAATT
TTTCCAGCATTTCCACCATGGGCATTCAGGCTCTCCACACTCTTCACTATTATCTCTTGGTCA
GAGGACTCCAATAACAGCCAGGTTTACATGAACTGTGTTTGTTCATTCTGACCTAAGGGGTTT
AGATAATCAGTAACCATAACCCCTGAAGCTGTGACTGCCAAACATCTCAAATGAAATGTTGTG
GCCATCAGAGACTCAAAAGGAAGTAAGGATTTTACAAGACAGATTAAAAAAAAATTGTTTTGT
CCAAAATATAGTTGTTGTTGATTTTTTTTTAAGTTTTCTAAGCAATATTTTTCAAGCCAGAAG
TCCTCTAAGTCTTGCCAGTACAAGGTAGTCTTGTGAAGAAAAGTTGAATACTGTTTTGTTTTC
ATCTCAAGGGGTTCCCTGGGTCTTGAACTACTTTAATAATAACTAAAAAACCACTTCTGATTT
TCCTTCAGTGATGTGCTTTTGGTGAAAGAATTAATGAACTCCAGTACCTGAAAGTGAAAGATT
TGATTTTGTTTCCATCTTCTGTAATCTTCCAAAGAATTATATCTTTGTAAATCTCTCAATACT
CAATCTACTGTAAGTACCCAGGGAGGCTAATTTCTTT

# FIGURE 424

MAGGRCGPQLTALLAAWIAAVAATAGPEEAALPPEQSRVQPMTASNWTLVMEGEWMLKFYAPW
CPSCQQTDSEWEAFAKNGEILQISVGKVDVIQEPGLSGRFFVTTLPAFFHAKDGIFRRYRGPG
IFEDLQNYILEKKWQSVEPLTGWKSPASLTMSGMAGLFSISGKIWHLHNYFTVTLGIPAWCSY
VFFVIATLVFGLFMGLVLVVISECFYVPLPRHLSERSEQNRRSEEAHRAEQLQDAEEEKDDSN
EEENKDSLVDDEEEKEDLGDEDEAEEEEEEDNLAAGVDEERSEANDQGPPGEDGVTREEVEPE
EAEEGISEQPCPADTEVVEDSLRQRKSQHADKGL

**Important features:**

**Signal peptide:**

amino acids 1-22

**Transmembrane domain:**

amino acids 191-211

**N-glycosylation site.**

amino acids 46-49

**Thioredoxin family proteins.** (homologous region to disulfide isomerase)

amino acids 56-72

**Flavodoxin proteins**

amino acids 173-187

# FIGURE 425

GAGGAACCTACCGGTACCGGCCGCGCGCTGGTAGTCGCCGGTGTGGCTGCACCTCACCAATCCCGTGCGCCGCGG
CTGGGCCGTCGGAGAGTGCGTGTGCTTCTCTCCTGCACGCGGTGCTTGGGCTCGGCCAGGCGGGGTCCGCCGCCA
GGGTTTGAGGATGGGGGAGTAGCTACAGGAAGCGACCCCGCGATGGCAAGGTATATTTTTGTGGAATGAAAAGGA
AGTATTAGAAATGAGCTGAAGACCATTCACAGATTAATATTTTTGGGGACAGATTTGTGATGCTTGATTCACCCT
TGAAGTAATGTAGACAGAAGTTCTCAAATTTGCATATTACATCAACTGGAACCAGCAGTGAATCTTAATGTTCAC
TTAAATCAGAACTTGCATAAGAAAGAGA**<u>ATG</u>**GGAGTCTGGTTAAATAAAGATGACTATATCAGAGACTTGAAAAG
GATCATTCTCTGTTTTCTGATAGTGTATATGGCCATTTTAGTGGGCACAGATCAGGATTTTTACAGTTTACTTGG
AGTGTCCAAAACTGCAAGCAGTAGAGAAATAAGACAAGCTTTCAAGAAATTGGCATTGAAGTTACATCCTGATAA
AAACCCGAATAACCCAAATGCACATGGCGATTTTTTAAAAATAAATAGAGCATATGAAGTACTCAAAGATGAAGA
TCTACGGAAAAAGTATGACAAATATGGAGAAAAGGGACTTGAGGATAATCAAGGTGGCCAGTATGAAAGCTGGAA
CTATTATCGTTATGATTTTGGTATTTATGATGATGATCCTGAAATCATAACATTGGAAAGAAGAGAATTTGATGC
TGCTGTTAATTCTGGAGAACTGTGGTTTGTAAATTTTTACTCCCCAGGCTGTTCACACTGCCATGATTTAGCTCC
CACATGGAGAGACTTTGCTAAAGAAGTGGATGGGTTACTTCGAATTGGAGCTGTTAACTGTGGTGATGATAGAAT
GCTTTGCCGAATGAAAGGAGTCAACAGCTATCCCAGTCTCTTCATTTTTCGGTCTGGAATGGCCCCAGTGAAATA
TCATGGAGACAGATCAAAGGAGAGTTTAGTGAGTTTTGCAATGCAGCATGTTAGAAGTACAGTGACAGAACTTTG
GACAGGAAATTTTGTCAACTCCATACAAACTGCTTTTGCTGCTGGTATTGGCTGGCTGATCACTTTTTGTTCAAA
AGGAGGAGATTGTTTGACTTCACAGACACGACTCAGGCTTAGTGGCATGTTGTTTCTCAACTCATTGGATGCTAA
AGAAATATATTTGGAAGTAATACATAATCTTCCAGATTTTGAACTACTTTCGGCAAACACACTAGAGGATCGTTT
GGCTCATCATCGGTGGCTGTTATTTTTTCATTTTGGAAAAAATGAAAATTCAAATGATCCTGAGCTGAAAAAACT
AAAAACTCTACTTAAAAATGATCATATTCAAGTTGGCAGGTTTGACTGTTCCTCTGCACCAGACATCTGTAGTAA
TCTGTATGTTTTTCAGCCGTCTCTAGCAGTATTTAAAGGACAAGGAACCAAAGAATATGAAATTCATCATGGAAA
GAAGATTCTATATGATATACTTGCCTTTGCCAAAGAAAGTGTGAATTCTCATGTTACCACGCTTGGACCTCAAAA
TTTTCCTGCCAATGACAAAGAACCATGGCTTGTTGATTTCTTTGCCCCCTGGTGTCCACCATGTCGAGCTTTACT
ACCAGAGTTACGAAGAGCATCAAATCTTCTTTATGGTCAGCTTAAGTTTGGTACACTAGATTGTACAGTTCATGA
GGGACTCTGTAACATGTATAACATTCAGGCTTATCCAACAACAGTGGTATTCAACCAGTCCAACATTCATGAGTA
TGAAGGACATCACTCTGCTGAACAAATCTTGGAGTTCATAGAGGATCTTATGAATCCTTCAGTGGTCTCCCTTAC
ACCCACCACCTTCAACGAACTAGTTACACAAAGAAAACACAACGAAGTCTGGATGGTTGATTTCTATTCTCCGTG
GTGTCATCCTTGCCAAGTCTTAATGCCAGAATGGAAAAGAATGGCCCGGACATTAACTGGACTGATCAACGTGGG
CAGTATAGATTGCCAACAGTATCATTCTTTTTGTGCCCAGGAAAACGTTCAAAGATACCCTGAGATAAGATTTTT
TCCCCCAAAATCAAATAAAGCTTATCAGTATCACAGTTACAATGGTTGGAATAGGGATGCTTATTCCCTGAGAAT
CTGGGGTCTAGGATTTTTACCTCAAGTATCCACAGATCTAACACCTCAGACTTTCAGTGAAAAAGTTCTACAAGG
GAAAAATCATTGGGTGATTGATTTCTATGCTCCTTGGTGTGGACCTTGCCAGAATTTTGCTCCAGAATTTGAGCT
CTTGGCTAGGATGATTAAAGGAAAAGTGAAAGCTGGAAAAGTAGACTGTCAGGCTTATGCTCAGACATGCCAGAA
AGCTGGGATCAGGGCCTATCCAACTGTTAAGTTTTATTTCTACGAAAGAGCAAAGAGAAATTTTCAAGAAGAGCA
GATAAATACCAGAGATGCAAAAGCAATCGCTGCCTTAATAAGTGAAAAATTGGAAACTCTCCGAAATCAAGGCAA
GAGGAATAAGGATGAACTT**<u>TGA</u>**TAATGTTGAAGATGAAGAAAAAGTTTAAAAGAAATTCTGACAGATGACATCAG
AAGACACCTATTTAGAATGTTACATTTATGATGGGAATGAATGAACATTATCTTAGACTTGCAGTTGTACTGCCA
GAATTATCTACAGCACTGGTGTAAAAGAAGGGTCTGCAAACTTTTTCTGTAAAGGGCCGGTTTATAAATATTTTA
GACTTTGCAGGCTATAATATATGGTTCACACATGAGAACAAGAATAGAGTCATCATGTATTCTTTGTTATTTGCT
TTTAACAACCTTTAAAAAATATTAAAACGATTCTTAGCTCAGAGCCATACAAAAGTAGGCTGGATTCAGTCCATG
GACCATAGATTGCTGTCCCCCTCGACGGACTTATAATGTTTCAGGTGGCTGGCTTGAACATGAGTCTGCTGTGCT
ATCTACATAAATGTCTAAGTTGTATAAAGTCCACTTTCCCTTCACGTTTTTTGGCTGACCTGAAAAGAGGTAACT
TAGTTTTTGGTCACTTGTTCTCCTAAAAATGCTATCCCTAACCATATATTTATATTTCGTTTTAAAAACACCCAT
GATGTGGCACAGTAAACAAACCCTGTTATGCTGTATTATTATGAGGAGATTCTTCATTGTTTTCTTTCCTTCTCA
AAGGTTGAAAAAATGCTTTTAATTTTTCACAGCCGAGAAACAGTGCAGCAGTATATGTGCACACAGTAAGTACAC
AAATTTGAGCAACAGTAAGTGCACAAATTCTGTAGTTTGCTGTATCATCCAGGAAAACCTGAGGGAAAAAAATTA
TAGCAATTAACTGGGCATTGTAGAGTATCCTAAATATGTTATCAAGTATTTAGAGTTCTATATTTAAAGATATA
TGTGTTCATGTATTTTCTGAAATTGCTTTCATAGAAATTTTCCCACTGATAGTTGATTTTTGAGGCATCTAATAT
TTACATATTTGCCTTCTGAACTTTGTTTTGACCTGTATCCTTTATTTACATTGGGTTTTTCTTTCATAGTTTTGG
TTTTTCACTCCTGTCCAGTCTATTTATTATTCAAATAGGAAAAATTACTTTACAGGTTGTTTTACTGTAGCTTAT
AATGATACTGTAGTTATTCCAGTTACTAGTTTACTGTCAGAGGGCTGCCTTTTTCAGATAAATATTGACATAATA
ACTGAAGTTATTTTTATAAGAAAATCAAGTATATAAATCTAGGAAAGGATCTTCTAGTTTCTGTGTTGTTTAGA
CTCAAAGAATCACAAATTTGTCAGTAACATGTAGTTGTTTAGTTATAATTCAGAGTGTACAGAATGGTAAAAATT
CCAATCAGTCAAAAGAGGTCAATGAATTAAAAGGCTTGCAACTTTTTCAAAAAAAAAAAAAAAAA

# FIGURE 426

MGVWLNKDDYIRDLKRIILCFLIVYMAILVGTDQDFYSLLGVSKTASSREIRQAFKKLALKLH
PDKNPNNPNAHGDFLKINRAYEVLKDEDLRKKYDKYGEKGLEDNQGGQYESWNYYRYDFGIYD
DDPEIITLERREFDAAVNSGELWFVNFYSPGCSHCHDLAPTWRDFAKEVDGLLRIGAVNCGDD
RMLCRMKGVNSYPSLFIFRSGMAPVKYHGDRSKESLVSFAMQHVRSTVTELWTGNFVNSIQTA
FAAGIGWLITFCSKGGDCLTSQTRLRLSGMLFLNSLDAKEIYLEVIHNLPDFELLSANTLEDR
LAHHRWLLFFHFGKNENSNDPELKKLKTLLKNDHIQVGRFDCSSAPDICSNLYVFQPSLAVFK
GQGTKEYEIHHGKKILYDILAFAKESVNSHVTTLGPQNFPANDKEPWLVDFFAPWCPPCRALL
PELRRASNLLYGQLKFGTLDCTVHEGLCNMYNIQAYPTTVVFNQSNIHEYEGHHSAEQILEFI
EDLMNPSVVSLTPTTFNELVTQRKHNEVWMVDFYSPWCHPCQVLMPEWKRMARTLTGLINVGS
IDCQQYHSFCAQENVQRYPEIRFFPPKSNKAYQYHSYNGWNRDAYSLRIWGLGFLPQVSTDLT
PQTFSEKVLQGKNHWVIDFYAPWCGPCQNFAPEFELLARMIKGKVKAGKVDCQAYAQTCQKAG
IRAYPTVKFYFYERAKRNFQEEQINTRDAKAIAALISEKLETLRNQGKRNKDEL

**Important features:**

**Endoplasmic reticulum targeting sequence.**

amino acids 744-747

**Cytochrome c family heme-binding site signature.**

amino acids 158-163

**Nt-dnaJ domain signature.**

amino acids 77-96

**N-glycosylation site.**

amino acids 484-487

# FIGURE 427

CTGCAGTCAGGACTCTGGGACCGCAGGGGGCTCCCGGACCCTGACTCTGCAGCCGAACCGGCA
CGGTTTCGTGGGGACCCAGGCTTGCAAAGTGACGGTCATTTTCTCTTTCTTTCTCCCTCTTGA
GTCCTTCTGAG**ATG**ATGGCTCTGGGCGCAGCGGGAGCTACCCGGGTCTTTGTCGCGATGGTAG
CGGCGGCTCTCGGCGGCCACCCTCTGCTGGGAGTGAGCGCCACCTTGAACTCGGTTCTCAATT
CCAACGCTATCAAGAACCTGCCCCCACCGCTGGGCGGCGCTGCGGGGCACCCAGGCTCTGCAG
TCAGCGCCGCGCCGGGAATCCTGTACCCGGGCGGGAATAAGTACCAGACCATTGACAACTACC
AGCCGTACCCGTGCGCAGAGGACGAGGAGTGCGGCACTGATGAGTACTGCGCTAGTCCCACCC
GCGGAGGGGACGCAGGCGTGCAAATCTGTCTCGCCTGCAGGAAGCGCCGAAAACGCTGCATGC
GTCACGCTATGTGCTGCCCCGGGAATTACTGCAAAAATGGAATATGTGTGTCTTCTGATCAAA
ATCATTTCCGAGGAGAAATTGAGGAAACCATCACTGAAAGCTTTGGTAATGATCATAGCACCT
TGGATGGGTATTCCAGAAGAACCACCTTGTCTTCAAAAATGTATCACACCAAAGGACAAGAAG
GTTCTGTTTGTCTCCGGTCATCAGACTGTGCCTCAGGATTGTGTTGTGCTAGACACTTCTGGT
CCAAGATCTGTAAACCTGTCCTGAAAGAAGGTCAAGTGTGTACCAAGCATAGGAGAAAAGGCT
CTCATGGACTAGAAATATTCCAGCGTTGTTACTGTGGAGAAGGTCTGTCTTGCCGGATACAGA
AAGATCACCATCAAGCCAGTAATTCTTCTAGGCTTCACACTTGTCAGAGACAC**TAA**ACCAGCT
ATCCAAATGCAGTGAACTCCTTTTATATAATAGATGCTATGAAAACCTTTTATGACCTTCATC
AACTCAATCCTAAGGATATACAAGTTCTGTGGTTTCAGTTAAGCATTCCAATAACACCTTCCA
AAAACCTGGAGTGTAAGAGCTTTGTTTCTTTATGGAACTCCCCTGTGATTGCAGTAAATTACT
GTATTGTAAATTCTCAGTGTGGCACTTACCTGTAAATGCAATGAAACTTTTAATTATTTTTCT
AAAGGTGCTGCACTGCCTATTTTTCCTCTTGTTATGTAAATTTTTGTACACATTGATTGTTAT
CTTGACTGACAAATATTCTATATTGAACTGAAGTAAATCATTTCAGCTTATAGTTCTTAAAAG
CATAACCCTTTACCCCATTTAATTCTAGAGTCTAGAACGCAAGGATCTCTTGGAATGACAAAT
GATAGGTACCTAAAATGTAACATGAAAATACTAGCTTATTTTCTGAAATGTACTATCTTAATG
CTTAAATTATATTTCCCTTTAGGCTGTGATAGTTTTGAAATAAAATTTAACATTTAAAAAAA
AAAAAA

# FIGURE 428

MMALGAAGATRVFVAMVAAALGGHPLLGVSATLNSVLNSNAIKNLPPPLGGAAGHPGSAVSAA
PGILYPGGNKYQTIDNYQPYPCAEDEECGTDEYCASPTRGGDAGVQICLACRKRRKRCMRHAM
CCPGNYCKNGICVSSDQNHFRGEIEETITESFGNDHSTLDGYSRRTTLSSKMYHTKGQEGSVC
LRSSDCASGLCCARHFWSKICKPVLKEGQVCTKHRRKGSHGLEIFQRCYCGEGLSCRIQKDHH
QASNSSRLHTCQRH

**Important features:**

**Signal peptide:**

amino acids 1-23

**N-glycosylation site.**

amino acids 256-259

**Fungal Zn(2)-Cys(6) binuclear cluster domain**

amino acids 110-126

# FIGURE 429

GAGAGGACGAGGTGCCGCTGCCTGGAGAATCCTCCGCTGCCGTCGGCTCCCGGAGCCCAGCCC
TTTCCTAACCCAACCCAACCTAGCCCAGTCCCAGCCGCCAGCGCCTGTCCCTGTCACGGACCC
CAGCGTTACC**ATG**CATCCTGCCGTCTTCCTATCCTTACCCGACCTCAGATGCTCCCTTCTGCT
CCTGGTAACTTGGGTTTTTACTCCTGTAACAACTGAAATAACAAGTCTTGCTACAGAGAATAT
AGATGAAATTTTAAACAATGCTGATGTTGCTTTAGTAAATTTTTATGCTGACTGGTGTCGTTT
CAGTCAGATGTTGCATCCAATTTTTGAGGAAGCTTCCGATGTCATTAAGGAAGAATTTCCAAA
TGAAAATCAAGTAGTGTTTGCCAGAGTTGATTGTGATCAGCACTCTGACATAGCCCAGAGATA
CAGGATAAGCAAATACCCAACCCTCAAATTGTTTCGTAATGGGATGATGATGAAGAGAGAATA
CAGGGGTCAGCGATCAGTGAAAGCATTGGCAGATTACATCAGGCAACAAAAAAGTGACCCCAT
TCAAGAAATTCGGGACTTAGCAGAAATCACCACTCTTGATCGCAGCAAAAGAAATATCATTGG
ATATTTTGAGCAAAAGGACTCGGACAACTATAGAGTTTTTGAACGAGTAGCGAATATTTTGCA
TGATGACTGTGCCTTTCTTTCTGCATTTGGGGATGTTTCAAAACCGGAAAGATATAGTGGCGA
CAACATAATCTACAAACCACCAGGGCATTCTGCTCCGGATATGGTGTACTTGGGAGCTATGAC
AAATTTTGATGTGACTTACAATTGGATTCAAGATAAATGTGTTCCTCTTGTCCGAGAAATAAC
ATTTGAAAATGGAGAGGAATTGACAGAAGAAGGACTGCCTTTTCTCATACTCTTTCACATGAA
AGAAGATACAGAAAGTTTAGAAATATTCCAGAATGAAGTAGCTCGGCAATTAATAAGTGAAAA
AGGTACAATAAACTTTTTACATGCCGATTGTGACAAATTTAGACATCCTCTTCTGCACATACA
GAAAACTCCAGCAGATTGTCCTGTAATCGCTATTGACAGCTTTAGGCATATGTATGTGTTTGG
AGACTTCAAAGATGTATTAATTCCTGGAAAACTCAAGCAATTCGTATTTGACTTACATTCTGG
AAAACTGCACAGAGAATTCCATCATGGACCTGACCCAACTGATACAGCCCCAGGAGAGCAAGC
CCAAGATGTAGCAAGCAGTCCACCTGAGAGCTCCTTCCAGAAACTAGCACCCAGTGAATATAG
GTATACTCTATTGAGGGATCGAGATGAGCTT**TAA**AAACTTGAAAAACAGTTTGTAAGCCTTTC
AACAGCAGCATCAACCTACGTGGTGGAAATAGTAAACCTATATTTTCATAATTCTATGTGTAT
TTTTATTTTGAATAAACAGAAAGAATTTAAAAAAAAAAAAAAAAAAAAAAAAAAAAAAAAAAA
AAAAAAAAAAA

# FIGURE 430

MHPAVFLSLPDLRCSLLLLVTWVFTPVTTEITSLATENIDEILNNADVALVNFYADWCRFSQM
LHPIFEEASDVIKEEFPNENQVVFARVDCDQHSDIAQRYRISKYPTLKLFRNGMMMKREYRGQ
RSVKALADYIRQQKSDPIQEIRDLAEITTLDRSKRNIIGYFEQKDSDNYRVFERVANILHDDC
AFLSAFGDVSKPERYSGDNIIYKPPGHSAPDMVYLGAMTNFDVTYNWIQDKCVPLVREITFEN
GEELTEEGLPFLILFHMKEDTESLEIFQNEVARQLISEKGTINFLHADCDKFRHPLLHIQKTP
ADCPVIAIDSFRHMYVFGDFKDVLIPGKLKQFVFDLHSGKLHREFHHGPDPTDTAPGEQAQDV
ASSPPESSFQKLAPSEYRYTLLRDRDEL

**Important features:**

**Signal peptide:**

amino acids 1-29

**Endoplasmic reticulum targeting sequence.**

amino acids 403-406

**Tyrosine kinase phosphorylation site.**

amino acids 203-211

**Thioredoxin family proteins**

amino acids 50-66

# FIGURE 431

GAGCAGGACGGAGCC**ATG**GACCCCGCCAGGAAAGCAGGTGCCCAGGCCATGATCTGGACTGCA
GGCTGGCTGCTGCTGCTGCTGCTTCGCGGAGGAGCGCAGGCCCTGGAGTGCTACAGCTGCGTG
CAGAAAGCAGATGACGGATGCTCCCCGAACAAGATGAAGACAGTGAAGTGCGCGCCGGGCGTG
GACGTCTGCACCGAGGCCGTGGGGGCGGTGGAGACCATCCACGGACAATTCTCGCTGGCAGTG
CGGGGTTGCGGTTCGGGACTCCCCGGCAAGAATGACCGCGGCCTGGATCTTCACGGGCTTCTG
GCGTTCATCCAGCTGCAGCAATGCGCTCAGGATCGCTGCAACGCCAAGCTCAACCTCACCTCG
CGGGCGCTCGACCCGGCAGGTAATGAGAGTGCATACCCGCCCAACGGCGTGGAGTGCTACAGC
TGTGTGGGCCTGAGCCGGGAGGCGTGCCAGGGTACATCGCCGCCGGTCGTGAGCTGCTACAAC
GCCAGCGATCATGTCTACAAGGGCTGCTTCGACGGCAACGTCACCTTGACGGCAGCTAATGTG
ACTGTGTCCTTGCCTGTCCGGGGCTGTGTCCAGGATGAATTCTGCACTCGGGATGGAGTAACA
GGCCCAGGGTTCACGCTCAGTGGCTCCTGTTGCCAGGGGTCCCGCTGTAACTCTGACCTCCGC
AACAAGACCTACTTCTCCCCTCGAATCCCACCCCTTGTCCGGCTGCCCCCTCCAGAGCCCACG
ACTGTGGCCTCAACCACATCTGTCACCACTTCTACCTCGGCCCCAGTGAGACCCACATCCACC
ACCAAACCCATGCCAGCGCCAACCAGTCAGACTCCGAGACAGGGAGTAGAACACGAGGCCTCC
CGGGATGAGGAGCCCAGGTTGACTGGAGGCGCCGCTGGCCACCAGGACCGCAGCAATTCAGGG
CAGTATCCTGCAAAAGGGGGGCCCCAGCAGCCCCATAATAAAGGCTGTGTGGCTCCCACAGCT
GGATTGGCAGCCCTTCTGTTGGCCGTGGCTGCTGGTGTCCTACTG**TGA**GCTTCTCCACCTGGA
AATTTCCCTCTCACCTACTTCTCTGGCCCTGGGTACCCCTCTTCTCATCACTTCCTGTTCCCA
CCACTGGACTGGGCTGGCCCAGCCCCTGTTTTTCCAACATTCCCCAGTATCCCCAGCTTCTGC
TGCGCTGGTTTGCGGCTTTGGGAAATAAAATACCGTTGTATATATTCTGCCAGGGGTGTTCTA
GCTTTTTGAGGACAGCTCCTGTATCCTTCTCATCCTTGTCTCTCCGCTTGTCCTCTTGTGATG
TTAGGACAGAGTGAGAGAAGTCAGCTGTCACGGGGAAGGTGAGAGAGAGGATGCTAAGCTTCC
TACTCACTTTCTCCTAGCCAGCCTGGACTTTGGAGCGTGGGGTGGGTGGGACAATGGCTCCCC
ACTCTAAGCACTGCCTCCCCTACTCCCCGCATCTTTGGGGAATCGGTTCCCCATATGTCTTCC
TTACTAGACTGTGAGCTCCTCGAGGGGGGGCCCGGTACCCAATTCGCCCTATAGTGAGTCGTA

# FIGURE 432

MDPARKAGAQAMIWTAGWLLLLLLLRGGAQALECYSCVQKADDGCSPNKMKTVKCAPGVDVCTE
AVGAVETIHGQFSLAVRGCGSGLPGKNDRGLDLHGLLAFIQLQQCAQDRCNAKLNLTSRALDP
AGNESAYPPNGVECYSCVGLSREACQGTSPPVVSCYNASDHVYKGCFDGNVTLTAANVTVSLP
VRGCVQDEFCTRDGVTGPGFTLSGSCCQGSRCNSDLRNKTYFSPRIPPLVRLPPPEPTTVAST
TSVTTSTSAPVRPTSTTKPMPAPTSQTPRQGVEHEASRDEEPRLTGGAAGHQDRSNSGQYPAK
GGPQQPHNKGCVAPTAGLAALLLAVAAGVLL

# FIGURE 433

CGGGACTCGGCGGGTCCTCCTGGGAGTCTCGGAGGGGACCGGCTGTGCAGACGCC**ATG**GAGTT
GGTGCTGGTCTTCCTCTGCAGCCTGCTGGCCCCCATGGTCCTGGCCAGTGCAGCTGAAAAGGA
GAAGGAAATGGACCCTTTTCATTATGATTACCAGACCCTGAGGATTGGGGGACTGGTGTTCGC
TGTGGTCCTCTTCTCGGTTGGGATCCTCCTTATCCTAAGTCGCAGGTGCAAGTGCAGTTTCAA
TCAGAAGCCCCGGGCCCCAGGAGATGAGGAAGCCCAGGTGGAGAACCTCATCACCGCCAATGC
AACAGAGCCCCAGAAGCAGAGAACTGAAGTGCAGCCATCAGGTGGAAGCCTCTGGAACCTGAG
GCGGCTGCTTGAACCTTTGGATGCAAATGTCGATGCT**TAA**GAAAACCGGCCACTTCAGCAACA
GCCCTTTCCCCAGGAGAAGCCAAGAACTTGTGTGTCCCCCACCCTATCCCCTCTAACACCATT
CCTCCACCTGATGATGCAACTAACACTTGCCTCCCCACTGCAGCCTGCGGTCCTGCCCACCTC
CCGTGATGTGTGTGTGTGTGTGTGTGTGTGACTGTGTGTGTTTGCTAACTGTGGTCTTTGTGG
CTACTTGTTTGTGGATGGTATTGTGTTTGTTAGTGAACTGTGGACTCGCTTTCCCAGGCAGGG
GCTGAGCCACATGGCCATCTGCTCCTCCCTGCCCCCGTGGCCCTCCATCACCTTCTGCTCCTA
GGAGGCTGCTTGTTGCCCGAGACCAGCCCCCTCCCCTGATTTAGGGATGCGTAGGGTAAGAGC
ACGGGCAGTGGTCTTCAGTCGTCTTGGGACCTGGGAAGGTTTGCAGCACTTTGTCATCATTCT
TCATGGACTCCTTTCACTCCTTTAACAAAAACCTTGCTTCCTTATCCCACCTGATCCCAGTCT
GAAGGTCTCTTAGCAACTGGAGATACAAAGCAAGGAGCTGGTGAGCCCAGCGTTGACGTCAGG
CAGGCTATGCCCTTCCGTGGTTAATTTCTTCCCAGGGGCTTCCACGAGGAGTCCCCATCTGCC
CCGCCCCTTCACAGAGCGCCCGGGGATTCCAGGCCCAGGGCTTCTACTCTGCCCCTGGGGAAT
GTGTCCCCTGCATATCTTCTCAGCAATAACTCCATGGGCTCTGGGACCCTACCCCTTCCAACC
TTCCCTGCTTCTGAGACTTCAATCTACAGCCCAGCTCATCCAGATGCAGACTACAGTCCCTGC
AATTGGGTCTCTGGCAGGCAATAGTTGAAGGACTCCTGTTCCGTTGGGGCCAGCACACCGGGA
TGGATGGAGGGAGAGCAGAGGCCTTTGCTTCTCTGCCTACGTCCCCTTAGATGGGCAGCAGAG
GCAACTCCCGCATCCTTTGCTCTGCCTGTCGGTGGTCAGAGCGGTGAGCGAGGTGGGTTGGAG
ACTCAGCAGGCTCCGTGCAGCCCTTGGGAACAGTGAGAGGTTGAAGGTCATAACGAGAGTGGG
AACTCAACCCAGATCCCGCCCCTCCTGTCCTCTGTGTTCCCGCGGAAACCAACCAAACCGTGC
GCTGTGACCCATTGCTGTTCTCTGTATCGTGATCTATCCTCAACAACAACAGAAAAAAGGAAT
AAAATATCCTTTGTTTCCT

# FIGURE 434

MELVLVFLCSLLAPMVLASAAEKEKEMDPFHYDYQTLRIGGLVFAVVLFSVGILLILSRRCKC
SFNQKPRAPGDEEAQVENLITANATEPQKQRTEVQPSGGSLWNLRRLLEPLDANVDA

# FIGURE 435

GGTCCTTA**ATG**GCAGCAGCCGCCGCTACCAAGATCCTTCTGTGCCTCCCGCTTCTGCTCCTGC
TGTCCGGCTGGTCCCGGGCTGGGCGAGCCGACCCTCACTCTCTTTGCTATGACATCACCGTCA
TCCCTAAGTTCAGACCTGGACCACGGTGGTGTGCGGTTCAAGGCCAGGTGGATGAAAAGACTT
TTCTTCACTATGACTGTGGCAACAAGACAGTCACACCTGTCAGTCCCCTGGGGAAGAAACTAA
ATGTCACAACGGCCTGGAAAGCACAGAACCCAGTACTGAGAGAGGTGGTGGACATACTTACAG
AGCAACTGCGTGACATTCAGCTGGAGAATTACACACCCAAGGAACCCCTCACCCTGCAGGCAA
GGATGTCTTGTGAGCAGAAAGCTGAAGGACACAGCAGTGGATCTTGGCAGTTCAGTTTCGATG
GGCAGATCTTCCTCCTCTTTGACTCAGAGAAGAGAATGTGGACAACGGTTCATCCTGGAGCCA
GAAAGATGAAAGAAAAGTGGGAGAATGACAAGGTTGTGGCCATGTCCTTCCATTACTTCTCAA
TGGGAGACTGTATAGGATGGCTTGAGGACTTCTTGATGGGCATGGACAGCACCCTGGAGCCAA
GTGCAGGAGCACCACTCGCCATGTCCTCAGGCACAACCCAACTCAGGGCCACAGCCACCACCC
TCATCCTTTGCTGCCTCCTCATCATCCTCCCCTGCTTCATCCTCCCTGGCATCTGAGGAGAGT
CCTTTAGAGTGACAGGTTAAAGCTGATACCAAAAGGCTCCTGTGAGCACGGTCTTGATCAAAC
TCGCCCTTCTGTCTGGCCAGCTGCCCACGACCTACGGTGTATGTCCAGTGGCCTCCAGCAGAT
CATGATGACATCATGGACCCAATAGCTCATTCACTGCCTTGATTCCTTTTGCCAACAATTTTA
CCAGCAGTTATACCTAACATATTATGCAATTTTCTCTTGGTGCTACCTGATGGAATTCCTGCA
CTTAAAGTTCTGGCTGACTAAACAAGATATATCATTTTCTTTCTTCTCTTTTTGTTTGGAAAA
TCAAGTACTTCTTTGAATGATGATCTCTTTCTTGCAAATGATATTGTCAGTAAAATAATCACG
TTAGACTTCAGACCTCTGGGGATTCTTTCCGTGTCCTGAAAGAGAATTTTTAAATTATTTAAT
AAGAAAAAATTTATATTAATGATTGTTTCCTTTAGTAATTTATTGTTCTGTACTGATATTTAA
ATAAAGAGTTCTATTTCCCAAAAAAAAAAAAAAAAAA

# FIGURE 436

MAAAAATKILLCLPLLLLLSGWSRAGRADPHSLCYDITVIPKFRPGPRWCAVQGQVDEKTFLH
YDCGNKTVTPVSPLGKKLNVTTAWKAQNPVLREVVDILTEQLRDIQLENYTPKEPLTLQARMS
CEQKAEGHSSGSWQFSFDGQIFLLFDSEKRMWTTVHPGARKMKEKWENDKVVAMSFHYFSMGD
CIGWLEDFLMGMDSTLEPSAGAPLAMSSGTTQLRATATTLILCCLLIILPCFILPGI

# FIGURE 437

GTTCTCCTTTCCGAGCCAAAATCCCAGGCGATGGTGAATTATGAACGTGCCACACC**ATG**AAGCTCTTGTGGCAGG
TAACTGTGCACCACCACACCTGGAATGCCATCCTGCTCCCGTTCGTCTACCTCACGGCGCAAGTGTGGATTCTGT
GTGCAGCCATCGCTGCTGCCGCCTCAGCCGGGCCCCAGAACTGCCCCTCCGTTTGCTCGTGCAGTAACCAGTTCA
GCAAGGTGGTGTGCACGCGCCGGGGCCTCTCCGAGGTCCCGCAGGGTATTCCCTCGAACACCCGGTACCTCAACC
TCATGGAGAACAACATCCAGATGATCCAGGCCGACACCTTCCGCCACCTCCACCACCTGGAGGTCCTGCAGTTGG
GCAGGAACTCCATCCGGCAGATTGAGGTGGGGGCCTTCAACGGCCTGGCCAGCCTCAACACCCTGGAGCTGTTCG
ACAACTGGCTGACAGTCATCCCTAGCGGGGCCTTTGAATACCTGTCCAAGCTGCGGGAGCTCTGGCTTCGCAACA
ACCCCATCGAAAGCATCCCCTCTTACGCCTTCAACCGGGTGCCCTCCCTCATGCGCCTGGACTTGGGGGAGCTCA
AGAAGCTGGAGTATATCTCTGAGGGAGCTTTTGAGGGGCTGTTCAACCTCAAGTATCTGAACTTGGGCATGTGCA
ACATTAAAGACATGCCCAATCTCACCCCCCTGGTGGGGCTGGAGGAGCTGGAGATGTCAGGGAACCACTTCCCTG
AGATCAGGCCTGGCTCCTTCCATGGCCTGAGCTCCCTCAAGAAGCTCTGGGTCATGAACTCACAGGTCAGCCTGA
TTGAGCGGAATGCTTTTGACGGGCTGGCTTCACTTGTGGAACTCAACTTGGCCCACAATAACCTCTCTTCTTTGC
CCCATGACCTCTTTACCCCGCTGAGGTACCTGGTGGAGTTGCATCTACACCACAACCCTTGGAACTGTGATTGTG
ACATTCTGTGGCTAGCCTGGTGGCTTCGAGAGTATATACCCACCAATTCCACCTGCTGTGGCCGCTGTCATGCTC
CCATGCACATGCGAGGCCGCTACCTCGTGGAGGTGGACCAGGCCTCCTTCCAGTGCTCTGCCCCCTTCATCATGG
ACGCACCTCGAGACCTCAACATTTCTGAGGGTCGGATGGCAGAACTTAAGTGTCGGACTCCCCCTATGTCCTCCG
TGAAGTGGTTGCTGCCCAATGGGACAGTGCTCAGCCACGCCTCCCGCCACCCAAGGATCTCTGTCCTCAACGACG
GCACCTTGAACTTTTCCCACGTGCTGCTTTCAGACACTGGGGTGTACACATGCATGGTGACCAATGTTGCAGGCA
ACTCCAACGCCTCGGCCTACCTCAATGTGAGCACGGCTGAGCTTAACACCTCCAACTACAGCTTCTTCACCACAG
TAACAGTGGAGACCACGGAGATCTCGCCTGAGGACACAACGCGAAAGTACAAGCCTGTTCCTACCACGTCCACTG
GTTACCAGCCGGCATATACCACCTCTACCACGGTGCTCATTCAGACTACCCGTGTGCCCAAGCAGGTGGCAGTAC
CCGCGACAGACACCACTGACAAGATGCAGACCAGCCTGGATGAAGTCATGAAGACCACCAAGATCATCATTGGCT
GCTTTGTGGCAGTGACTCTGCTAGCTGCCGCCATGTTGATTGTCTTCTATAAACTTCGTAAGCGGCACCAGCAGC
GGAGTACAGTCACAGCCGCCCGGACTGTTGAGATAATCCAGGTGGACGAAGACATCCCAGCAGCAACATCCGCAG
CAGCAACAGCAGCTCCGTCCGGTGTATCAGGTGAGGGGGCAGTAGTGCTGCCCACAATTCATGACCATATTAACT
ACAACACCTACAAACCAGCACATGGGGCCCACTGGACAGAAAACAGCCTGGGGAACTCTCTGCACCCCACAGTCA
CCACTATCTCTGAACCTTATATAATTCAGACCCATACCAAGGACAAGGTACAGGAAACTCAAATA**TGA**CTCCCCT
CCCCCAAAAAACTTATAAAATGCAATAGAATGCACACAAAGACAGCAACTTTTGTACAGAGTGGGGAGAGACTTT
TTCTTGTATATGCTTATATATTAAGTCTATGGGCTGGTTAAAAAAAACAGATTATATTAAAATTTAAAGACAAAA
AGTCAAAACA

# FIGURE 438

MKLLWQVTVHHHTWNAILLPFVYLTAQVWILCAAIAAAASAGPQNCPSVCSCSNQFSKVVCTR
RGLSEVPQGIPSNTRYLNLMENNIQMIQADTFRHLHHLEVLQLGRNSIRQIEVGAFNGLASLN
TLELFDNWLTVIPSGAFEYLSKLRELWLRNNPIESIPSYAFNRVPSLMRLDLGELKKLEYISE
GAFEGLFNLKYLNLGMCNIKDMPNLTPLVGLEELEMSGNHFPEIRPGSFHGLSSLKKLWVMNS
QVSLIERNAFDGLASLVELNLAHNNLSSLPHDLFTPLRYLVELHLHHNPWNCDCDILWLAWWL
REYIPTNSTCCGRCHAPMHMRGRYLVEVDQASFQCSAPFIMDAPRDLNISEGRMAELKCRTPP
MSSVKWLLPNGTVLSHASRHPRISVLNDGTLNFSHVLLSDTGVYTCMVTNVAGNSNASAYLNV
STAELNTSNYSFFTTVTVETTEISPEDTTRKYKPVPTTSTGYQPAYTTSTTVLIQTTRVPKQV
AVPATDTTDKMQTSLDEVMKTTKIIIGCFVAVTLLAAAMLIVFYKLRKRHQQRSTVTAARTVE
IIQVDEDIPAATSAAATAAPSGVSGEGAVVLPTIHDHINYNTYKPAHGAHWTENSLGNSLHPT
VTTISEPYIIQTHTKDKVQETQI

# FIGURE 439

GTCGAATCCAAATCACTCATTGTGAAAGCTGAGCTCACAGCCGAATAAGCCACC**ATG**AGGCTG
TCAGTGTGTCTCCTGATGGTCTCGCTGGCCCTTTGCTGCTACCAGGCCCATGCTCTTGTCTGC
CCAGCTGTTGCTTCTGAGATCACAGTCTTCTTATTCTTAAGTGACGCTGCGGTAAACCTCCAA
GTTGCCAAACTTAATCCACCTCCAGAAGCTCTTGCAGCCAAGTTGGAAGTGAAGCACTGCACC
GATCAGATATCTTTTAAGAAACGACTCTCATTGAAAAAGTCCTGGTGGAAA**TAG**TGAAAAAAT
GTGGTGTGTGACATGTAAAAATGCTCAACCTGGTTTCCAAAGTCTTTCAACGACACCCTGATC
TTCACTAAAAATTGTAAAGGTTTCAACACGTTGCTTTAATAAATCACTTGCCCTGC

# FIGURE 440

MRLSVCLLMVSLALCCYQAHALVCPAVASEITVFLFLSDAAVNLQVAKLNPPPEALAAKLEVK
HCTDQISFKKRLSLKKSWWK

# FIGURE 441

GAACATTTTTAGTTCCCAAGGAATGTACATCAGCCCCACGGAAGCTAGGCCACCTCTGGGATG
GGGTTGCTGGTTTAAAACAAACGCCAGTCATCCTATATAAGGACCTGACAGCCACCAGGCACC
ACCTCCGCCAGGAACTGCAGGCCCACCTGTCTGCAACCCAGCTGAGGCC**ATG**CCCTCCCCAGG
GACCGTCTGCAGCCTCCTGCTCCTCGGCATGCTCTGGCTGGACTTGGCCATGGCAGGCTCCAG
CTTCCTGAGCCCTGAACACCAGAGAGTCCAGCAGAGAAAGGAGTCGAAGAAGCCACCAGCCAA
GCTGCAGCCCCGAGCTCTAGCAGGCTGGCTCCGCCCGGAAGATGGAGGTCAAGCAGAAGGGGC
AGAGGATGAACTGGAAGTCCGGTTCAACGCCCCCTTTGATGTTGGAATCAAGCTGTCAGGGGT
TCAGTACCAGCAGCACAGCCAGGCCCTGGGGAAGTTTCTTCAGGACATCCTCTGGGAAGAGGC
CAAAGAGGCCCCAGCCGACAAG**TGA**TCGCCCACAAGCCTTACTCACCTCTCTCTAAGTTTAGA
AGCGCTCATCTGGCTTTTCGCTTGCTTCTGCAGCAACTCCCACGACTGTTGTACAAGCTCAGG
AGGCGAATAAATGTTCAAACTGTA

# FIGURE 442

MPSPGTVCSLLLLGMLWLDLAMAGSSFLSPEHQRVQQRKESKKPPAKLQPRALAGWLRPEDGG
QAEGAEDELEVRFNAPFDVGIKLSGVQYQQHSQALGKFLQDILWEEAKEAPADKO

# FIGURE 443

CGGCCACAGCTGGCATGCTCTGCCTGATCGCCATCCTGCTGTATGTCCTCGTCCAGTACCTCG
TGAACCCCGGGGTGCTCCGCACGGACCCCAGATGTCAAGAAT**ATG**AACACGTGGCTGCTGTTC
CTCCCCCTGTTCCCGGTGCAGGTGCAGACCCTGATAGTCGTGATCATCGGGATGCTCGTGCTC
CTGCTGGACTTTCTTGGCTTGGTGCACCTGGGCCAGCTGCTCATCTTCCACATCTACCTGAGT
ATGTCCCCCACCCTAAGCCCCCGATCCCCCCAAGGCTGGGTGGTCAGAGCTGCTCATCTTACA
CCTCTACTTGAGTATGTCCCTAACCCTGAGCCCCCCACGCCTGGGGCCAGAGTCTTTGTCCCC
CGTGTGCGCATGTGTTCAGGGTCAGCCTCTCCCAGAAGTGAGATCATGGACAAAAAGGGCAAA
TCACAGGAAGAAATTAAATCCATGAGGACCCAGCAGGCCCAGCAAGAAGCTGAACTCACGCCG
AGACCTGCAGGAGTGGTGCCAGGTGCT**TGA**AGTAACAAGTTTAAAATGTTCAGAGACAATGGA
ATGGAATCTATTAGGCAAGAACAGGACATTATGAAATAAGGACAGGTGGACTTCCAAAAACAC
AAGTAGAAATTCTAACAATGAAATATATTACAGGCAGGTCACCCACTAACCAAACAACTGAAG
CGAGAGCTGTGGTCTTGCTTGGTCTCACAGTGGGCACAGCGGTAGGCGGTCAGTCATGTTGCT
GAACGACGGAGGGTAAACTCCCCAGCCCCAAGAAAACCTGTGTTGGAAGTAACAACAACCTCC
CTGCTCCTGGCACCAGCCGTTTTGGTCATGGTGGGCCAGCTGCAAAGCGTCTTCCATTCTCTG
GGCAGTGGTGGCCCCGAGGCTGTGGCCTCTCAGGGGGTTTCTGTGGACACGGGCAGCAGAGTG
TGTCCAGGCCAGCCCCCAAGAATGCCCTGCTCCTGACAGCTTGGCCAACCCCTGGTCAGGGCA
GAGGGAGTTGGGTGGGTCAGGCTCTGGGCTCACCTCCATCTCCAGAGCATCCCCTGCCTGCAG
TTGTGGCAAGAACGCCCAGCTCAGAATGAACACACCCCACCAAGAGCCTCCTTGTTCATAACC
ACAGGTTACCCTACAAACCACTGTCCCCACACAACCCTGGGGATGTTTTAAAACACACACCTC
TAACGCATATCTTACAGTCACTGTTGTCTTGCCTGAGGGTTGAATTTTTTTTAATGAAAGTGC
AATGAAAATCACTGGATTAAATCCTACGGACACAGAGCTGAAAAAAAAAAAAAAAAAAAAAAA
AAAAAAAAA

# FIGURE 444

MNTWLLFLPLFPVQVQTLIVVIIGMLVLLLDFLGLVHLGQLLIFHIYLSMSPTLSPRSPQGWV
VRAAHLTPLLEYVPNPEPPTPGARVFVPRVRMCSGSASPRSEIMDKKGKSQEEIKSMRTQQAQ
QEAELTPRPAGVVPGA

# FIGURE 445

AGGCGGGCAGCAGCTGCAGGCTGACCTTGCAGCTTGGCGGA**<u>ATG</u>**GACTGGCCTCACAACCTGC
TGTTTCTTCTTACCATTTCCATCTTCCTGGGGCTGGGCCAGCCCAGGAGCCCCAAAAGCAAGA
GGAAGGGGCAAGGGCGGCCTGGGCCCCTGGCCCCTGGCCCTCACCAGGTGCCACTGGACCTGG
TGTCACGGATGAAACCGTATGCCCGCATGGAGGAGTATGAGAGGAACATCGAGGAGATGGTGG
CCCAGCTGAGGAACAGCTCAGAGCTGGCCCAGAGAAAGTGTGAGGTCAACTTGCAGCTGTGGA
TGTCCAACAAGAGGAGCCTGTCTCCCTGGGGCTACAGCATCAACCACGACCCCAGCCGTATCC
CCGTGGACCTGCCGGAGGCACGGTGCCTGTGTCTGGGCTGTGTGAACCCCTTCACCATGCAGG
AGGACCGCAGCATGGTGAGCGTGCCGGTGTTCAGCCAGGTTCCTGTGCGCCGCCGCCTCTGCC
CGCCACCGCCCCGCACAGGGCCTTGCCGCCAGCGCGCAGTCATGGAGACCATCGCTGTGGGCT
GCACCTGCATCTTC**<u>TGA</u>**ATCACCTGGCCCAGAAGCCAGGCCAGCAGCCCGAGACCATCCTCCT
TGCACCTTTGTGCCAAGAAAGGCCTATGAAAAGTAAACACTGACTTTTGAAAGCAAG

# FIGURE 446

MDWPHNLLFLLTISIFLGLGQPRSPKSKRKGQGRPGPLAPGPHQVPLDLVSRMKPYARMEEYE
RNIEEMVAQLRNSSELAQRKCEVNLQLWMSNKRSLSPWGYSINHDPSRIPVDLPEARCLCLGC
VNPFTMQEDRSMVSVPVFSQVPVRRRLCPPPPRTGPCRQRAVMETIAVGCTCIF

**Important features:**

**Signal peptide:**

amino acids 1-20

**N-glycosylation site.**

amino acids 75-78

**Homologous region to IL-17**

amino acids 96-180.

# FIGURE 447

GGAGTGCAGATGGCATCCTTCGGTTCTTCCAGACAAGCTGCAAGACGCTGACC**ATG**GCCAAGA
TGGAGCTCTCGAAGGCCTTCTCTGGCCAGCGGACACTCCTATCTGCCATCCTCAGCATGCTAT
CACTCAGCTTCTCCACAACATCCCTGCTCAGCAACTACTGGTTTGTGGCACACAGAAGGTGC
CCAAGCCCCTGTGCGAGAAAGGTCTGGCAGCCAAGTGCTTTGACATGCCAGTGTCCCTGGATG
GAGATACCAACACATCCACCCAGGAGGTGGTACAATACAACTGGGAGACTGGGGATGACCGGT
TCTCCTTCCGGAGCTTCCGGAGTGGCATGTGGCTATCCTGTGAGGAAACTGTGGAAGAACCAG
GGGAGAGGTGCCGAAGTTTCATTGAACTTACACCACCAGCCAAGAGAGGTGAGAAAGGACTAC
TGGAATTTGCCACGTTGCAAGGCCCATGTCACCCCACTCTCCGATTTGGAGGGAAGCGGTTGA
TGGAGAAGGCTTCCCTCCCCTCCCCTCCCTTGGGGCTTTGTGGCAAAAATCCTATGGTTATCC
CTGGGAACGCAGATCACCTACATCGGACTTCAATTCATCAGCTTCCTCCTGCTACTAACAGAC
TTGCTACTCACTGGGAACCCTGCCTGTGGGCTCAAACTGAGCGCCTTTGCTGCTGTTTCCTCT
GTCCTGTCAGGTCTCCTGGGGATGGTGGCCCACATGATGTATTCACAAGTCTTCCAAGCGACT
GTCAACTTGGGTCCAGAAGACTGGAGACCACATGTTTGGAATTATGGCTGGGCCTTCTACATG
GCCTGGCTCTCCTTCACCTGCTGCATGGCGTCGGCTGTCACCACCTTCAACACGTACACCAGG
ATGGTGCTGGAGTTCAAGTGCAAGCA**TAG**TAAGAGCTTCAAGGAAAACCCGAACTGCCTACCA
CATCACCATCAGTGTTTCCCTCGGCGGCTGTCAAGTGCAGCCCCCACCGTGGGTCCTTTGACC
AGCTACCACCAGTATCATAATCAGCCCATCCACTCTGTCTCTGAGGGAGTCGACTTCTACTCC
GAGCTGCGGAACAAGGGATTTCAAAGAGGGGCCAGCCAGGAGCTGAAAGAAGCAGTTAGGTCA
TCTGTAGAGGAAGAGCAGTGTTAGGAGTTAAGCGGGTTTGGGGAGTAGGCTTGAGCCCTACCT
TACACGTCTGCTGATTATCAACATGTGCTTAAGCCAACATCCGTCTCTTGAGCATGGTTTTTA
GAGGCTACGAATAAGGCTATGAATAAGGGTTATCTTTAAGTCCTAAGGGATTCCTGGGTGCCA
CTGCTCTCTTTTCCTCTACAGCTCCATCTTGTTTCACCCACCCCACATCTCACACATCCAGAA
TTCCCTTCTTTACTGATAGTTTCTGTGCCAGGTTCTGGGCTAAACCATGGAGATAAAAAGAAG
AGTAAAATACACTTCCCGACCTTAAGGATCTGAAA

# FIGURE 448

MAKMELSKAFSGQRTLLSAILSMLSLSFSTTSLLSNYWFVGTQKVPKPLCEKGLAAKCFDMPV
SLDGDTNTSTQEVVQYNWETGDDRFSFRSFRSGMWLSCEETVEEPGERCRSFIELTPPAKRGE
KGLLEFATLQGPCHPTLRFGGKRLMEKASLPSPPLGLCGKNPMVIPGNADHLHRTSIHQLPPA
TNRLATHWEPCLWAQTERLCCCFLCPVRSPGDGGPHDVFTSLPSDCQLGSRRLETTCLELWLG
LLHGLALLHLLHGVGCHHLQHVHQDGAGVQVQA

# FIGURE 449

CCCACGCGTCCGCCCACGCGTCCGCCCACGCGTCCGCCCACGCGTCCGCCCACGCGTCCGCCCACGCGTCCGCCC
ACGCGTCCGGTGCAAGCTCGCGCCGCACACTGCCTGGTGGAGGGAAGGAGCCCGGGCGCCTCTCGCCGCTCCCCG
CGCCGCCGTCCGCACCTCCCCACCGCCCGCCGCCCGCCGCCCGCCGCCCGCAAAGCATGAGTGAGCCCGCTCTCT
GCAGCTGCCCGGGGCGCGAATGGCAGGCTGTTTCCGCGGAGTAAAAGGTGGCGCCGGTCAGTGGTCGTTTCCAAT
GACGGACATTAACCAGACTGTCAGATCCTGGGGAGTCGCGAGCCCCGAGTTTGGAGTTTTTCCCCCCACAACGT
CACAGTCCGAACTGCAGAGGGAAAGGAAGGCGGCAGGAAGGCGAAGCTCGGGCTCCGGCACGTAGTTGGGAAACT
TGCGGGTCCTAGAAGTCGCCTCCCCGCCTTGCCGGCCGCCCTTGCAGCCCCGAGCCGAGCAGCAAAGTGAGACAT
TGTGCGCCTGCCAGATCCGCCGGCCGCGGACCGGGGCTGCCTCGGAAACACAGAGGGGTCTTCTCTCGCCCTGCA
TATAATTAGCCTGCACACAAAGGGAGCAGCTGAATGGAGGTTGTCACTCTCTGGAAAAGGATTTCTGACCGAGCG
CTTCCAATGGACATTCTCCAGTCTCTCTGGAAAGATTCTCGCTA**<u>ATG</u>**GATTTCCTGCTGCTCGGTCTCTGTCTAT
ACTGGCTGCTGAGGAGGCCCTCGGGGGTGGTCTTGTGTCTGCTGGGGGCCTGCTTTCAGATGCTGCCCGCCGCCC
CCAGCGGGTGCCCGCAGCTGTGCCGGTGCGAGGGGCGGCTGCTGTACTGCGAGGCGCTCAACCTCACCGAGGCGC
CCCACAACCTGTCCGGCCTGCTGGGCTTGTCCCTGCGCTACAACAGCCTCTCGGAGCTGCGCGCCGGCCAGTTCA
CGGGGTTAATGCAGCTCACGTGGCTCTATCTGGATCACAATCACATCTGCTCCGTGCAGGGGGACGCCTTTCAGA
AACTGCGCCGAGTTAAGGAACTCACGCTGAGTTCCAACCAGATCACCCAACTGCCCAACACCACCTTCCGGCCCA
TGCCCAACCTGCGCAGCGTGGACCTCTCGTACAACAAGCTGCAGGCGCTCGCGCCCGACCTCTTCCACGGGCTGC
GGAAGCTCACCACGCTGCATATGCGGGCCAACGCCATCCAGTTTGTGCCCGTGCGCATCTTCCAGGACTGCCGCA
GCCTCAAGTTTCTCGACATCGGATACAATCAGCTCAAGAGTCTGGCGCGCAACTCTTTCGCCGGCTTGTTTAAGC
TCACCGAGCTGCACCTCGAGCACAACGACTTGGTCAAGGTGAACTTCGCCCACTTCCCGCGCCTCATCTCCCTGC
ACTCGCTCTGCCTGCGGAGGAACAAGGTGGCCATTGTGGTCAGCTCGCTGGACTGGGTTTGGAACCTGGAGAAAA
TGGACTTGTCGGGCAACGAGATCGAGTACATGGAGCCCCATGTGTTCGAGACCGTGCCGCACCTGCAGTCCCTGC
AGCTGGACTCCAACCGCCTCACCTACATCGAGCCCCGGATCCTCAACTCTTGGAAGTCCCTGACAAGCATCACCC
TGGCCGGGAACCTGTGGGATTGCGGGCGCAACGTGTGTGCCCTAGCCTCGTGGCTCAGCAACTTCCAGGGGCGCT
ACGATGGCAACTTGCAGTGCGCCAGCCCGGAGTACGCACAGGGCGAGGACGTCCTGGACGCCGTGTACGCCTTCC
ACCTGTGCGAGGATGGGGCCGAGCCCACCAGCGGCCACCTGCTCTCGGCCGTCACCAACCGCAGTGATCTGGGGC
CCCCTGCCAGCTCGGCCACCACGCTCGCGGACGGCGGGGAGGGGCAGCACGACGGCACATTCGAGCCTGCCACCG
TGGCTCTTCCAGGCGGCGAGCACGCCGAGAACGCCGTGCAGATCCACAAGGTGGTCACGGGCACCATGGCCCTCA
TCTTCTCCTTCCTCATCGTGGTCCTGGTGCTCTACGTGTCCTGGAAGTGTTTCCCAGCCAGCCTCAGGCAGCTCA
GACAGTGCTTTGTCACGCAGCGCAGGAAGCAAAAGCAGAAACAGACCATGCATCAGATGGCTGCCATGTCTGCCC
AGGAATACTACGTTGATTACAAACCGAACCACATTGAGGGAGCCCTGGTGATCATCAACGAGTATGGCTCGTGTA
CCTGCCACCAGCAGCCCGCGAGGGAATGCGAGGTG**<u>TGA</u>**TTGTCCCAGTGGCTCTCAACCCATGCGCTACCAAATA
CGCCTGGGCAGCCGGGACGGGCCGGCGGGCACCAGGCTGGGGTCTCCTTGTCTGTGCTCTGATATGCTCCTTGAC
TGAAACTTTAAGGGGATCTCTCCCAGAGACTTGACATTTTAGCTTTATTGTGTCTTAAAAACAAAAGCGAATTAA
AACACAACAAAAAACCCCACCCCACAACCTTCAGGACAGTCTATCTTAAATTTCATATGAGAACTCCTTCCTCCC
TTTGAAGATCTGTCCATATTCAGGAATCTGAGAGTGTAAAAAAGGTGGCCATAAGACAGAGAGAGAATAATCGTG
CTTTGTTTTATGCTACTCCTCCCACCCTGCCCATGATTAAACATCATGTATGTAGAAGATCTTAAGTCCATACGC
ATTTCATGAAGAACCATTGGAAAGAGGAATCTGCAATCTGGGAGCTTAAGAGCAAATGATGACCATAGAAAGCTA
TGTTCTTACTTTGTGTGTGTGTCTGTATGTTTCTGCGTTGTGTGTCTTTGTAGGCAAGCAAACGTTGTCTACACA
AACGGGAATTTAGCTCACATCATTTCATGCCCCTGTGCCTCTAGCTCTGGAGATTGGTGGGGGAGGTGGGGGA
AACGGCAGGAATAAGGGAAAGTGGTAGTTTTAACTAAGGTTTTGTAACACTTGAAATCTTTTCTTTCTCAAATTA
ATTATCTTTAAGCTTCAAGAAACTTGCTCTGACCCCTCTAAGCAAACTACTAAGCATTTAAAAGAGAATCTAATT
TTTAAAGGTGTAGCACCTTTTTTTTTATTCTTCCCACAGAGGGTGCTAATCTCATTATGCTGTGCTATCTGAAAA
GAACTTAAGGCCACAATTCAGGTCTCGTCCTGGGCATTGTGATGGATTGACCCTCCATTTGCAGTACCTTCCCAG
CTGATTAAAGTTCAGCAGTGGTATTGAGGTTTTTCGAATATTTATATAGAAAAAAAGTCTTTTCACATGACAAAT
GACACTCTCACACCAGTCTTAGCCCTAGTAGTTTTTTAGGTTGGACCAGAGGAAGCAGGTTAAATGAGACCTGTC
CTCTGCTGCACTCAGAAAAAAATAGGCAGTCCCTGATGCTCAGATCTTAGCCTTGATATTAATAGTTGAGACCACC
TACCCACAATGCAGCCTATACTCCCAAGACTACAAAGTTACCATCGCAAAGGAAAGGTTATTCCAGTAAAAGGAA
ATAGTTTTCTCAACCATTTAAAAATATTCTTCTGAACTCATCAAAGTAGAAGAGCCCCCAACCTTTTCTCTCTGC
CTTCAAGAAGGCAGACATTTGGTATGATTTAGCATCAACAACACATTTATGAGTATATGTAAGTAATCAGAGGGG
CAAATGCCACTTGTTATTCCTCCCAAGTTTTCCAAGCAAGTACACACAGATCTCTGGTAGGATTAGGGGCCACTT
GTGTTTCCGGCTTATTTTAGTCGACTTGTCAGCAAGTTTGATGCCTAGTCTATCTGACATGGCCCAGTAGAACAG
GGCATTGATGGATCACATGAGATGGTAGAAGGAACATCATCACATACCCCTCTCACAGAGAAAATTATCAAAGAA
CCAGAAATTATATCTGTTTTGGAGCAAGAGTGTCATAATGTTTCAGGGTAGTCAAAATAAACATAAATTATCTCC
TCTAGATGAGTGGCGATGTTGGCTGATTTGGGTCTGCCATTGACAGAATGTCAAATAAAAAGGAATTAGCTAGAA
TATGACCATTAAATGTGCTTCTGAAATATATTTGAGATAGGTTTAGAATGTCA

# FIGURE 450

MDFLLLGLCLYWLLRRPSGVVLCLLGACFQMLPAAPSGCPQLCRCEGRLLYCEALNLTEAPHN
LSGLLGLSLRYNSLSELRAGQFTGLMQLTWLYLDHNHICSVQGDAFQKLRRVKELTLSSNQIT
QLPNTTFRPMPNLRSVDLSYNKLQALAPDLFHGLRKLTTLHMRANAIQFVPVRIFQDCRSLKF
LDIGYNQLKSLARNSFAGLFKLTELHLEHNDLVKVNFAHFPRLISLHSLCLRRNKVAIVVSSL
DWVWNLEKMDLSGNEIEYMEPHVFETVPHLQSLQLDSNRLTYIEPRILNSWKSLTSITLAGNL
WDCGRNVCALASWLSNFQGRYDGNLQCASPEYAQGEDVLDAVYAFHLCEDGAEPTSGHLLSAV
TNRSDLGPPASSATTLADGGEGQHDGTFEPATVALPGGEHAENAVQIHKVVTGTMALIFSFLI
VVLVLYVSWKCFPASLRQLRQCFVTQRRKQKQKQTMHQMAAMSAQEYYVDYKPNHIEGALVII
NEYGSCTCHQQPARECEV

# FIGURE 451

TTGAGCGCAGGTGAGCTCCTGCGCGTTCCGGGGGCGTTCCTCCAGTCACCCTCCCGCCGTTAC
CCGCGGCGCGCCCGAGGGAGTCTCCTCCAGACCCTCCCTCCCGTTGCTCCAAACTAATACGGA
CTGAACGGATCGCTGCGAGGGTGGGAGAGAAAATTAGGGGGAGAAAGGACAGAGAGAGCAACT
ACCATCCATAGCCAGATAGATTATCTTACACTGAACTGATCAAGTACTTTGAAA**ATG**ACTTCG
AAATTTATCTTGGTGTCCTTCATACTTGCTGCACTGAGTCTTTCAACCACCTTTTCTCTCCAA
CTAGACCAGCAAAAGGTTCTACTAGTTTCTTTTGATGGATTCCGTTGGGATTACTTATATAAA
GTTCCAACGCCCCATTTTCATTATATTATGAAATATGGTGTTCACGTGAAGCAAGTTACTAAT
GTTTTTATTACAAAAACCTACCCTAACCATTATACTTTGGTAACTGGCCTCTTTGCAGAGAAT
CATGGGATTGTTGCAAATGATATGTTTGATCCTATTCGGAACAAATCTTTCTCCTTGGATCAC
ATGAATATTTATGATTCCAAGTTTTGGGAAGAAGCGACACCAATATGGATCACAAACCAGAGG
GCAGGACATACTAGTGGTGCAGCCATGTGGCCCGGAACAGATGTAAAAATACATAAGCGCTTT
CCTACTCATTACATGCCTTACAATGAGTCAGTTTCATTTGAAGATAGAGTTGCCAAAATTGTT
GAATGGTTTACGTCAAAAGAGCCCATAAATCTTGGTCTTCTCTATTGGGAAGACCCTGATGAC
ATGGGCCACCATTTGGGACCTGACAGTCCGCTCATGGGGCCTGTCATTTCAGATATTGACAAG
AAGTTAGGATATCTCATACAAATGCTGAAAAAGGCAAAGTTGTGGAACACTCTGAACCTAATC
ATCACAAGTGATCATGGAATGACGCAGTGCTCTGAGGAAAGGTTAATAGAACTTGACCAGTAC
CTGGATAAAGACCACTATACCCTGATTGATCAATCTCCAGTAGCAGCCATCTTGCCAAAAGAA
GGTAAATTTGATGAAGTCTATGAAGCACTAACTCACGCTCATCCTAATCTTACTGTTTACAAA
AAAGAAGACGTTCCAGAAAGGTGGCATTACAAATACAACAGTCGAATTCAACCAATCATAGCA
GTGGCTGATGAAGGGTGGCACATTTTACAGAATAAGTCAGATGACTTTCTGTTAGGCAACCAC
GGTTACGATAATGCGTTAGCAGATATGCATCCAATATTTTTAGCCCATGGTCCTGCCTTCAGA
AAGAATTTCTCAAAAGAAGCCATGAACTCCACAGATTTGTACCCACTACTATGCCACCTCCTC
AATATCACTGCCATGCCACACAATGGATCATTCTGGAATGTCCAGGATCTGCTCAATTCAGCA
ATGCCAAGGGTGGTCCCTTATACACAGAGTACTATACTCCTCCCTGGTAGTGTTAAACCAGCA
GAATATGACCAAGAGGGGTCATACCCTTATTTCATAGGGGTCTCTCTTGGCAGCATTATAGTG
ATTGTATTTTTTGTAATTTTCATTAAGCATTTAATTCACAGTCAAATACCTGCCTTACAAGAT
ATGCATGCTGAAATAGCTCAACCATTATTACAAGCC**TAA**TGTTACTTTGAAGTGGATTTGCAT
ATTGAAGTGGAGATTCCATAATTATGTCAGTGTTTAAAGGTTTCAAATTCTGGGAAACCAGTT
CCAAACATCTGCAGAAACCATTAAGCAGTTACATATTTAGGTATACACACACACACACACACA
CACATACACACACACGGACCAAAATACTTACACCTGCAAAGGAATAAAGATGTGAGAGTATGT
CTCCATTGTTCACTGTAGCATAGGGATAGATAAGATCCTGCTTTATTTGGACTTGGCGCAGAT
AATGTATATATTTAGCAACTTTGCACTATGTAAAGTACCTTATATATTGCACTTTAAATTTCT
CTCCTGATGGGTACTTTAATTTGAAATGCACTTTATGGACAGTTATGTCTTATAACTTGATTG
AAAATGACAACTTTTTGCACCCATGTCACAGAATACTTGTTACGCATTGTTCAAACTGAAGGA
AATTTCTAATAATCCCGAATAATGAACATAGAAATCTATCTCCATAAATTGAGAGAAGAAGAA
GGTGATAAGTGTTGAAAATTAAATGTGATAACCTTTGAACCTTGAATTTTGGAGATGTATTCC
CAACAGCAGAATGCAACTGTGGGCATTTCTTGTCTTATTTCTTTCCAGAGAACGTGGTTTTCA
TTTATTTTTCCCTCAAAAGAGAGTCAAATACTGACAGATTCGTTCTAAATATATTGTTTCTGT
CATAAAATTATTGTGATTTCCTGATGAGTCATATTACTGTGATTTTCATAATAATGAAGACAC
CATGAATATACTTTTCTTCTATATAGTTCAGCAATGGCCTGAATAGAAGCAACCAGGCACCAT
CTCAGCAATGTTTTCTCTTGTTTGTAATTATTTGCTCCTTTGAAAATTAAATCACTATTAATT
ACATTAAAAATCAAATTGGATAAAAAAAAAAAAAAAAAAA

# FIGURE 452

MTSKFILVSFILAALSLSTTFSLQLDQQKVLLVSFDGFRWDYLYKVPTPHFHYIMKYGVHVKQ
VTNVFITKTYPNHYTLVTGLFAENHGIVANDMFDPIRNKSFSLDHMNIYDSKFWEEATPIWIT
NQRAGHTSGAAMWPGTDVKIHKRFPTHYMPYNESVSFEDRVAKIVEWFTSKEPINLGLLYWED
PDDMGHHLGPDSPLMGPVISDIDKKLGYLIQMLKKAKLWNTLNLIITSDHGMTQCSEERLIEL
DQYLDKDHYTLIDQSPVAAILPKEGKFDEVYEALTHAHPNLTVYKKEDVPERWHYKYNSRIQP
IIAVADEGWHILQNKSDDFLLGNHGYDNALADMHPIFLAHGPAFRKNFSKEAMNSTDLYPLLC
HLLNITAMPHNGSFWNVQDLLNSAMPRVVPYTQSTILLPGSVKPAEYDQEGSYPYFIGVSLGS
IIVIVFFVIFIKHLIHSQIPALQDMHAEIAQPLLQA

**Important features:**

**Signal Peptide:**

amino acids 1-22

**Transmembrane Domain:**

amino acids 429-452

**N-glycosylation sites:**

amino acids 101-104, 158-161, 292-295, 329-332, 362-365, 369-372, 382-385, 389-392

**Somatomedin B Domain:**

amino acids 69-85

**Sulfatase protein Region:**

amino acids 212-241

# FIGURE 453

GGCCGCCTGGAATTGTGGGAGTTGTGTCTGCCACTCGGCTGCCGGAGGCCGAAGGTCCGTGAC
T**ATG**GCTCCCCAGAGCCTGCCTTCATCTAGGATGGCTCCTCTGGGCATGCTGCTTGGGCTGCT
GATGGCCGCCTGCTTCACCTTCTGCCTCAGTCATCAGAACCTGAAGGAGTTTGCCCTGACCAA
CCCAGAGAAGAGCAGCACCAAAGAAACGGAGAGAAAAGAAACCAAAGCCGAGGAGGAGCTGGA
TGCCGAAGTCCTGGAGGTGTTCCACCCGACGCATGAGTGGCAGGCCCTTCAGCCAGGGCAGGC
TGTCCCTGCAGGATCCCACGTACGGCTGAATCTTCAGACTGGGGAAAGAGAGGCAAAACTCCA
ATATGAGGACAAGTTCCGAAATAATTTGAAAGGCAAAAGGCTGGATATCAACACCAACACCTA
CACATCTCAGGATCTCAAGAGTGCACTGGCAAAATTCAAGGAGGGGGCAGAGATGGAGAGTTC
AAAGGAAGACAAGGCAAGGCAGGCTGAGGTAAAGCGGCTCTTCCGCCCCATTGAGGAACTGAA
GAAAGACTTTGATGAGCTGAATGTTGTCATTGAGACTGACATGCAGATCATGGTACGGCTGAT
CAACAAGTTCAATAGTTCCAGCTCCAGTTTGGAAGAGAAGATTGCTGCGCTCTTTGATCTTGA
ATATTATGTCCATCAGATGGACAATGCGCAGGACCTGCTTTCCTTTGGTGGTCTTCAAGTGGT
GATCAATGGGCTGAACAGCACAGAGCCCCTCGTGAAGGAGTATGCTGCGTTTGTGCTGGGCGC
TGCCTTTTCCAGCAACCCCAAGGTCCAGGTGGAGGCCATCGAAGGGGGAGCCCTGCAGAAGCT
GCTGGTCATCCTGGCCACGGAGCAGCCGCTCACTGCAAAGAAGAAGGTCCTGTTTGCACTGTG
CTCCCTGCTGCGCCACTTCCCCTATGCCCAGCGGCAGTTCCTGAAGCTCGGGGGGCTGCAGGT
CCTGAGGACCCTGGTGCAGGAGAAGGGCACGGAGGTGCTCGCCGTGCGCGTGGTCACACTGCT
CTACGACCTGGTCACGGAGAAGATGTTCGCCGAGGAGGAGGCTGAGCTGACCCAGGAGATGTC
CCCAGAGAAGCTGCAGCAGTATCGCCAGGTACACCTCCTGCCAGGCCTGTGGGAACAGGGCTG
GTGCGAGATCACGGCCCACCTCCTGGCGCTGCCCGAGCATGATGCCCGTGAGAAGGTGCTGCA
GACACTGGGCGTCCTCCTGACCACCTGCCGGGACCGCTACCGTCAGGACCCCCAGCTCGGCAG
GACACTGGCCAGCCTGCAGGCTGAGTACCAGGTGCTGGCCAGCCTGGAGCTGCAGGATGGTGA
GGACGAGGGCTACTTCCAGGAGCTGCTGGGCTCTGTCAACAGCTTGCTGAAGGAGCTGAGA**TG**
**A**GGCCCCACACCAGGACTGGACTGGGATGCCGCTAGTGAGGCTGAGGGGTGCCAGCGTGGGTG
GGCTTCTCAGGCAGGAGGACATCTTGGCAGTGCTGGCTTGGCCATTAAATGGAAACCTGAAGG
CCAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAA

# FIGURE 454

MAPQSLPSSRMAPLGMLLGLLMAACFTFCLSHQNLKEFALTNPEKSSTKETERKETKAEEELD
AEVLEVFHPTHEWQALQPGQAVPAGSHVRLNLQTGEREAKLQYEDKFRNNLKGKRLDINTNTY
TSQDLKSALAKFKEGAEMESSKEDKARQAEVKRLFRPIEELKKDFDELNVVIETDMQIMVRLI
NKFNSSSSSLEEKIAALFDLEYYVHQMDNAQDLLSFGGLQVVINGLNSTEPLVKEYAAFVLGA
AFSSNPKVQVEAIEGGALQKLLVILATEQPLTAKKKVLFALCSLLRHFPYAQRQFLKLGGLQV
LRTLVQEKGTEVLAVRVVTLLYDLVTEKMFAEEEAELTQEMSPEKLQQYRQVHLLPGLWEQGW
CEITAHLLALPEHDAREKVLQTLGVLLTTCRDRYRQDPQLGRTLASLQAEYQVLASLELQDGE
DEGYFQELLGSVNSLLKELR

**Important features:**

**Signal peptide:**

amino acids 1-29

**Hypothetical YJL126w/YLR351c/yhcX family protein.**

amino acids 364-373

**N-glycosylation site.**

amino acids 193-197, 236-240

**N-myristoylation site.**

amino acids 15-21, 19-25, 234-240, 251-257, 402-408, 451-457

**Homologous region SLS1 protein.**

amino acids 68-340

# FIGURE 455

GCCCCAGGGAGCAGTGGGTGGTTATAACTCAGGCCCGGTGCCCAGAGCCCAGGAGGAGGCAGT
GGCCAGGAAGGCACAGGCCTGAGAAGTCTGCGGCTGAGCTGGGAGCAAATCCCCCACCCCCTA
CCTGGGGGACAGGGCAAGTGAGACCTGGTGAGGGTGGCTCAGCAGGCAGGGAAGGAGAGGTGT
CTGTGCGTCCTGCACCCACATCTTTCTCTGTCCCCTCCTTGCCCTGTCTGGAGGCTGCTAGAC
TCCTATCTTCTGAATTCTATAGTGCCTGGGTCTCAGCGCAGTGCCGATGGTGGCCCGTCCTTG
TGGTTCCTCTCTACCTGGGGAAATAAGGTGCAGCGGCC**ATG**GCTACAGCAAGACCCCCCTGGA
TGTGGGTGCTCTGTGCTCTGATCACAGCCTTGCTTCTGGGGGTCACAGAGCATGTTCTCGCCA
ACAATGATGTTTCCTGTGACCACCCCTCTAACACCGTGCCCTCTGGGAGCAACCAGGACCTGG
GAGCTGGGGCCGGGGAAGACGCCCGGTCGGATGACAGCAGCAGCCGCATCATCAATGGATCCG
ACTGCGATATGCACACCCAGCCGTGGCAGGCCGCGCTGTTGCTAAGGCCCAACCAGCTCTACT
GCGGGGCGGTGTTGGTGCATCCACAGTGGCTGCTCACGGCCGCCCACTGCAGGAAGAAAGTTT
TCAGAGTCCGTCTCGGCCACTACTCCCTGTCACCAGTTTATGAATCTGGGCAGCAGATGTTCC
AGGGGGTCAAATCCATCCCCCACCCTGGCTACTCCCACCCTGGCCACTCTAACGACCTCATGC
TCATCAAACTGAACAGAAGAATTCGTCCCACTAAAGATGTCAGACCCATCAACGTCTCCTCTC
ATTGTCCCTCTGCTGGGACAAAGTGCTTGGTGTCTGGCTGGGGGACAACCAAGAGCCCCCAAG
TGCACTTCCCTAAGGTCCTCCAGTGCTTGAATATCAGCGTGCTAAGTCAGAAAAGGTGCGAGG
ATGCTTACCCGAGACAGATAGATGACACCATGTTCTGCGCCGGTGACAAAGCAGGTAGAGACT
CCTGCCAGGGTGATTCTGGGGGGCCTGTGGTCTGCAATGGCTCCCTGCAGGGACTCGTGTCCT
GGGGAGATTACCCTTGTGCCCGGCCCAACAGACCGGGTGTCTACACGAACCTCTGCAAGTTCA
CCAAGTGGATCCAGGAAACCATCCAGGCCAACTCC**TGA**GTCATCCCAGGACTCAGCACACCGG
CATCCCCACCTGCTGCAGGGACAGCCCTGACACTCCTTTCAGACCCTCATTCCTTCCCAGAGA
TGTTGAGAATGTTCATCTCTCCAGCCCCTGACCCCATGTCTCCTGGACTCAGGGTCTGCTTCC
CCCACATTGGGCTGACCGTGTCTCTCTAGTTGAACCCTGGGAACAATTTCCAAAACTGTCCAG
GGCGGGGGTTGCGTCTCAATCTCCCTGGGGCACTTTCATCCTCAAGCTCAGGGCCCATCCCTT
CTCTGCAGCTCTGACCCAAATTTAGTCCCAGAAATAAACTGAGAAGTGGAAAAAAAAA

# FIGURE 456

MATARPPWMWVLCALITALLLGVTEHVLANNDVSCDHPSNTVPSGSNQDLGAGAGEDARSDDS
SSRIINGSDCDMHTQPWQAALLLRPNQLYCGAVLVHPQWLLTAAHCRKKVFRVRLGHYSLSPV
YESGQQMFQGVKSIPHPGYSHPGHSNDLMLIKLNRRIRPTKDVRPINVSSHCPSAGTKCLVSG
WGTTKSPQVHFPKVLQCLNISVLSQKRCEDAYPRQIDDTMFCAGDKAGRDSCQGDSGGPVVCN
GSLQGLVSWGDYPCARPNRPGVYTNLCKFTKWIQETIQANS

# FIGURE 457

GCAGTCAGAGACTTCCCCTGCCCCTCGCTGGGAAAGAACATTAGGAATGCCTTTTAGTGCCTTGCTTCCTGAACT
AGCTCACAGTAGCCCGGCGGCCCAGGGCAATCCGACCACATTTCACTCTCACCGCTGTAGGAATCCAG**ATG**CAGG
CCAAGTACAGCAGCACGAGGGACATGCTGGATGATGATGGGGACACCACCATGAGCCTGCATTCTCAAGCCTCTG
CCACAACTCGGCATCCAGAGCCCCGGCGCACAGAGCACAGGGCTCCCTCTTCAACGTGGCGACCAGTGGCCCTGA
CCCTGCTGACTTTGTGCTTGGTGCTGCTGATAGGGCTGGCAGCCCTGGGGCTTTTGTTTTTTCAGTACTACCAGC
TCTCCAATACTGGTCAAGACACCATTTCTCAAATGGAAGAAAGATTAGGAAATACGTCCCAAGAGTTGCAATCTC
TTCAAGTCCAGAATATAAAGCTTGCAGGAAGTCTGCAGCATGTGGCTGAAAAACTCTGTCGTGAGCTGTATAACA
AAGCTGGAGCACACAGGTGCAGCCCTTGTACAGAACAATGGAAATGGCATGGAGACAATTGCTACCAGTTCTATA
AAGACAGCAAAAGTTGGGAGGACTGTAAATATTTCTGCCTTAGTGAAAACTCTACCATGCTGAAGATAAACAAAC
AAGAAGACCTGGAATTTGCCGCGTCTCAGAGCTACTCTGAGTTTTCTACTCTTATTGGACAGGGCTTTTGCGCC
CTGACAGTGGCAAGGCCTGGCTGTGGATGGATGGAACCCCTTTCACTTCTGAACTGTTCCATATTATAATAGATG
TCACCAGCCCAAGAAGCAGAGACTGTGTGGCCATCCTCAATGGGATGATCTTCTCAAAGGACTGCAAAGAATTGA
AGCGTTGTGTCTGTGAGAGAAGGGCAGGAATGGTGAAGCCAGAGAGCCTCCATGTCCCCCCTGAAACATTAGGCG
AAGGTGAC**TGA**TTCGCCCTCTGCAACTACAAATAGCAGAGTGAGCCAGGCGGTGCCAAAGCAAGGGCTAGTTGAG
ACATTGGGAAATGGAACATAATCAGGAAAGACTATCTCTCTGACTAGTACAAAATGGGTTCTCGTGTTTCCTGTT
CAGGATCACCAGCATTTCTGAGCTTGGGTTTATGCACGTATTTAACAGTCACAAGAAGTCTTATTTACATGCCAC
CAACCAACCTCAGAAACCCATAATGTCATCTGCCTTCTTGGCTTAGAGATAACTTTTAGCTCTCTTTCTTCTCAA
TGTCTAATATCACCTCCCTGTTTTCATGTCTTCCTTACACTTGGTGGAATAAGAAACTTTTTGAAGTAGAGGAAA
TACATTGAGGTAACATCCTTTTCTCTGACAGTCAAGTAGTCCATCAGAAATTGGCAGTCACTTCCCAGATTGTAC
CAGCAAATACACAAGGAATTCTTTTTGTTTGTTTCAGTTCATACTAGTCCCTTCCCAATCCATCAGTAAAGACCC
CATCTGCCTTGTCCATGCCGTTTCCCAACAGGGATGTCACTTGATATGAGAATCTCAAATCTCAATGCCTTATAA
GCATTCCTTCCTGTGTCCATTAAGACTCTGATAATTGTCTCCCCTCCATAGGAATTTCTCCCAGGAAAGAAATAT
ATCCCCATCTCCGTTTCATATCAGAACTACCGTCCCCGATATTCCCTTCAGAGAGATTAAAGACCAGAAAAAAGT
GAGCCTCTTCATCTGCACCTGTAATAGTTTCAGTTCCTATTTTCTTCCATTGACCCATATTTATACCTTTCAGGT
ACTGAAGATTTAATAATAATAAATGTAAATACTGTGAAAAA

# FIGURE 458

MQAKYSSTRDMLDDDGDTTMSLHSQASATTRHPEPRRTEHRAPSSTWRPVALTLLTLCLVLLI
GLAALGLLFFQYYQLSNTGQDTISQMEERLGNTSQELQSLQVQNIKLAGSLQHVAEKLCRELY
NKAGAHRCSPCTEQWKWHGDNCYQFYKDSKSWEDCKYFCLSENSTMLKINKQEDLEFAASQSY
SEFFYSYWTGLLRPDSGKAWLWMDGTPFTSELFHIIIDVTSPRSRDCVAILNGMIFSKDCKEL
KRCVCERRAGMVKPESLHVPPETLGEGD

# FIGURE 459

GTTGATGGCAAACTTCCTCAAAGGAGGGGCAGAGCCTGCGCAGGGCAGGAGCAGCTGGCCCAC
TGGCGGCCCGCAACACTCCGTCTCACCCTCTGGGCCCACTGCATCTAGAGGAGGGCCGTCTGT
GAGGCCACTACCCCTCCAGCAACTGGGAGGTGGGACTGTCAGAAGCTGGCCCAGGGTGGTGGT
CAGCTGGGTCAGGGACCTACGGCACCTGCTGGACCACCTCGCCTTCTCCATCGAAGCAGGGAA
GTGGGAGCCTCGAGCCCTCGGGTGGAAGCTGACCCCAAGCCACCCTTCACCTGGACAGG**ATG**A
GAGTGTCAGGTGTGCTTCGCCTCCTGGCCCTCATCTTTGCCATAGTCACGACATGGATGTTTA
TTCGAAGCTACATGAGCTTCAGCATGAAAACCATCCGTCTGCCACGCTGGCTGGCAGCCTCGC
CCACCAAGGAGATCCAGGTTAAAAAGTACAAGTGTGGCCTCATCAAGCCCTGCCCAGCCAACT
ACTTTGCGTTTAAAATCTGCAGTGGGGCCGCCAACGTCGTGGGCCCTACTATGTGCTTTGAAG
ACCGCATGATCATGAGTCCTGTGAAAAACAATGTGGGCAGAGGCCTAAACATCGCCCTGGTGA
ATGGAACCACGGGAGCTGTGCTGGGACAGAAGGCATTTGACATGTACTCTGGAGATGTTATGC
ACCTAGTGAAATTCCTTAAAGAAATTCCGGGGGGTGCACTGGTGCTGGTGGCCTCCTACGACG
ATCCAGGGACCAAAATGAACGATGAAAGCAGGAAACTCTTCTCTGACTTGGGGAGTTCCTACG
CAAAACAACTGGGCTTCCGGGACAGCTGGGTCTTCATAGGAGCCAAAGACCTCAGGGGTAAAA
GCCCCTTTGAGCAGTTCTTAAAGAACAGCCCAGACACAAACAAATACGAGGGATGGCCAGAGC
TGCTGGAGATGGAGGGCTGCATGCCCCCGAAGCCATTT**TAG**GGTGGCTGTGGCTCTTCCTCAG
CCAGGGGCCTGAAGAAGCTCCTGCCTGACTTAGGAGTCAGAGCCCGGCAGGGGCTGAGGAGGA
GGAGCAGGGGGTGCTGCGTGGAAGGTGCTGCAGGTCCTTGCACGCTGTGTCGCGCCTCTCCTC
CTCGGAAACAGAACCCTCCCACAGCACATCCTACCCGGAAGACCAGCCTCAGAGGGTCCTTCT
GGAACCAGCTGTCTGTGGAGAGAATGGGGTGCTTTCGTCAGGGACTGCTGACGGCTGGTCCTG
AGGAAGGACAAACTGCCCAGACTTGAGCCCAATTAAATTTTATTTTTGCTGGTTTTGAAAAAA
AAAAAAAAAAAAAA

# FIGURE 460

MRVSGVLRLLALIFAIVTTWMFIRSYMSFSMKTIRLPRWLAASPTKEIQVKKYKCGLIKPCPA
NYFAFKICSGAANVVGPTMCFEDRMIMSPVKNNVGRGLNIALVNGTTGAVLGQKAFDMYSGDV
MHLVKFLKEIPGGALVLVASYDDPGTKMNDESRKLFSDLGSSYAKQLGFRDSWVFIGAKDLRG
KSPFEQFLKNSPDTNKYEGWPELLEMEGCMPPKPF

**Important features:**

**Signal peptide:**

amino acids 1-15

**ATP/GTP-binding site motif A (P-loop).**

amino acids 184-191

**N-glycosylation site.**

amino acids 107-110

# FIGURE 461

AAACTCAGCACTTGCCGGAGTGGCTCATTGTTAAGACAAAGGGTGTGCACTTCCTGGCCAGGA
AACCTGAGCGGTGAGACTCCCAGCTGCCTACATCAAGGCCCCAGGACATGCAGAACCTTCCTC
TAGAACCCGACCCACCACC**ATG**AGGTCCTGCCTGTGGAGATGCAGGCACCTGAGCCAAGGCGT
CCAGTGGTCCTTGCTTCTGGCTGTCCTGGTCTTCTTTCTCTTCGCCTTGCCCTCTTTTATTAA
GGAGCCTCAAACAAAGCCTTCCAGGCATCAACGCACAGAGAACATTAAAGAAAGGTCTCTACA
GTCCCTGGCAAAGCCTAAGTCCCAGGCACCCACAAGGGCGAGGAGGACAACCATCTATGCAGA
GCCAGCGCCAGAGAACAATGCCCTCAACACACAAACCCAGCCCAAGGCCCACACCACCGGAGA
CAGAGGAAAGGAGGCCAACCAGGCACCGCCGGAGGAGCAGGACAAGGTGCCCCACACAGCACA
GAGGGCAGCATGGAAGAGCCCAGAAAAAGAGAAAACCATGGTGAACACACTGTCACCCAGAGG
GCAAGATGCAGGGATGGCCTCTGGCAGGACAGAGGCACAATCATGGAAGAGCCAGGACACAAA
GACGACCCAAGGAAATGGGGGCCAGACCAGGAAGCTGACGGCCTCCAGGACGGTGTCAGAGAA
GCACCAGGGCAAAGCGGCAACCACAGCCAAGACGCTCATTCCCAAAAGTCAGCACAGAATGCT
GGCTCCCACAGGAGCAGTGTCAACAAGGACGAGACAGAAAGGAGTGACCACAGCAGTCATCCC
ACCTAAGGAGAAGAAACCTCAGGCCACCCCACCCCCTGCCCCTTTCCAGAGCCCCACGACGCA
GAGAAACCAAAGACTGAAGGCCGCCAACTTCAAATCTGAGCCTCGGTGGGATTTTGAGGAAAA
ATACAGCTTCGAAATAGGAGGCCTTCAGACGACTTGCCCTGACTCTGTGAAGATCAAAGCCTC
CAAGTCGCTGTGGCTCCAGAAACTCTTTCTGCCCAACCTCACTCTCTTCCTGGACTCCAGACA
CTTCAACCAGAGTGAGTGGGACCGCCTGGAACACTTTGCACCACCCTTTGGCTTCATGGAGCT
CAACTACTCCTTGGTGCAGAAGGTCGTGACACGCTTCCCTCCAGTGCCCCAGCAGCAGCTGCT
CCTGGCCAGCCTCCCCGCTGGGAGCCTCCGGTGCATCACCTGTGCCGTGGTGGGCAACGGGGG
CATCCTGAACAACTCCCACATGGGCCAGGAGATAGACAGTCACGACTACGTGTTCCGATTGAG
CGGAGCTCTCATTAAAGGCTACGAACAGGATGTGGGGACTCGGACATCCTTCTACGGCTTTAC
CGCCTTCTCCCTGACCCAGTCACTCCTTATATTGGGCAATCGGGGTTTCAAGAACGTGCCTCT
TGGGAAGGACGTCCGCTACTTGCACTTCCTGGAAGGCACCCGGGACTATGAGTGGCTGGAAGC
ACTGCTTATGAATCAGACGGTGATGTCAAAAAACCTTTTCTGGTTCAGGCACAGACCCCAGGA
AGCTTTTCGGGAAGCCCTGCACATGGACAGGTACCTGTTGCTGCACCCAGACTTTCTCCGATA
CATGAAGAACAGGTTTCTGAGGTCTAAGACCCTGGATGGTGCCCACTGGAGGATATACCGCCC
CACCACTGGGGCCCTCCTGCTGCTCACTGCCCTTCAGCTCTGTGACCAGGTGAGTGCTTATGG
CTTCATCACTGAGGGCCATGAGCGCTTTTCTGATCACTACTATGATACATCATGGAAGCGGCT
GATCTTTTACATAAACCATGACTTCAAGCTGGAGAGAGAAGTCTGGAAGCGGCTACACGATGA
AGGGATAATCCGGCTGTACCAGCGTCCTGGTCCCGGAACTGCCAAAGCCAAGAAC**TGA**CCGGG
GCCAGGGCTGCCATGGTCTCCTTGCCTGCTCCAAGGCACAGGATACAGTGGGAATCTTGAGAC
TCTTTGGCCATTTCCCATGGCTCAGACTAAGCTCCAAGCCCTTCAGGAGTTCCAAGGGAACAC
TTGAACCATGGACAAGACTCTCTCAAGATGGCAAATGGCTAATTGAGGTTCTGAAGTTCTTCA
GTACATTGCTGTAGGTCCTGAGGCCAGGGATTTTTAATTAAATGGGGTGATGGGTGGCCAATA
CCACAATTCCTGCTGAAAAACACTCTTCCAGTCCAAAAGCTTCTTGATACAGAAAAAAGAGCC
TGGATTTACAGAAACATATAGATCTGGTTTGAATTCCAGATCGAGTTTACAGTTGTGAAATCT
TGAAGGTATTACTTAACTTCACTACAGATTGTCTAGAAGACCTTTCTAGGAGTTATCTGATTC
TAGAAGGGTCTATACTTGTCCTTGTCTTTAAGCTATTTGACAACTCTACGTGTTGTAGAAAAC
TGATAATAATACAAATGATTGTTGTCCATGGAAAGGCAAATAAATTTTCTACAGTGAAAAAAA
AAAAAAAA

# FIGURE 462

MRSCLWRCRHLSQGVQWSLLLAVLVFFLFALPSFIKEPQTKPSRHQRTENIKERSLQSLAKPK
SQAPTRARRTTIYAEPAPENNALNTQTQPKAHTTGDRGKEANQAPPEEQDKVPHTAQRAAWKS
PEKEKTMVNTLSPRGQDAGMASGRTEAQSWKSQDTKTTQGNGGQTRKLTASRTVSEKHQGKAA
TTAKTLIPKSQHRMLAPTGAVSTRTRQKGVTTAVIPPKEKKPQATPPPAPFQSPTTQRNQRLK
AANFKSEPRWDFEEKYSFEIGGLQTTCPDSVKIKASKSLWLQKLFLPNLTLFLDSRHFNQSEW
DRLEHFAPPFGFMELNYSLVQKVVTRFPPVPQQQLLLASLPAGSLRCITCAVVGNGGILNNSH
MGQEIDSHDYVFRLSGALIKGYEQDVGTRTSFYGFTAFSLTQSLLILGNRGFKNVPLGKDVRY
LHFLEGTRDYEWLEALLMNQTVMSKNLFWFRHRPQEAFREALHMDRYLLLHPDFLRYMKNRFL
RSKTLDGAHWRIYRPTTGALLLLTALQLCDQVSAYGFITEGHERFSDHYYDTSWKRLIFYINH
DFKLEREVWKRLHDEGIIRLYQRPGPGTAKAKN

**Important features:**

**Cytoplasmic Domain:**

amino acids 1-10

**Type II Transmembrane Domain:**

amino acids 11-35

**Lumenal catalytic Domain:**

amino acids 36-600

**Ribonucleotide Reductase small subunit Signature:**

amino acids 481-496

**N-glycosylation Sites:**

amino acids 300-303, 311-314, 331-334, 375-378, 460-463

# FIGURE 463

GGGGGAGCTAGGCCGGCGGCAGTGGTGGTGGCGGCGGCGCAAGGGTGAGGGCGGCCCCAGAAC
CCCAGGTAGGTAGAGCAAGAAG**ATG**GTGTTTCTGCCCCTCAAATGGTCCCTTGCAACCATGTC
ATTTCTACTTTCCTCACTGTTGGCTCTCTTAACTGTGTCCACTCCTTCATGGTGTCAGAGCAC
TGAAGCATCTCCAAAACGTAGTGATGGGACACCATTTCCTTGGAATAAAATACGACTTCCTGA
GTACGTCATCCCAGTTCATTATGATCTCTTGATCCATGCAAACCTTACCACGCTGACCTTCTG
GGGAACCACGAAAGTAGAAATCACAGCCAGTCAGCCCACCAGCACCATCATCCTGCATAGTCA
CCACCTGCAGATATCTAGGGCCACCCTCAGGAAGGGAGCTGGAGAGAGGCTATCGGAAGAACC
CCTGCAGGTCCTGGAACACCCCCCTCAGGAGCAAATTGCACTGCTGGCTCCCGAGCCCCTCCT
TGTCGGGCTCCCGTACACAGTTGTCATTCACTATGCTGGCAATCTTTCGGAGACTTTCCACGG
ATTTTACAAAAGCACCTACAGAACCAAGGAAGGGGAACTGAGGATACTAGCATCAACACAATT
TGAACCCACTGCAGCTAGAATGGCCTTTCCCTGCTTTGATGAACCTGCCTTCAAAGCAAGTTT
CTCAATCAAAATTAGAAGAGAGCCAAGGCACCTAGCCATCTCCAATATGCCATTGGTGAAATC
TGTGACTGTTGCTGAAGGACTCATAGAAGACCATTTTGATGTCACTGTGAAGATGAGCACCTA
TCTGGTGGCCTTCATCATTTCAGATTTTGAGTCTGTCAGCAAGATAACCAAGAGTGGAGTCAA
GGTTTCTGTTTATGCTGTGCCAGACAAGATAAATCAAGCAGATTATGCACTGGATGCTGCGGT
GACTCTTCTAGAATTTTATGAGGATTATTTCAGCATACCGTATCCCCTACCCAAACAAGATCT
TGCTGCTATTCCCGACTTTCAGTCTGGTGCTATGGAAAACTGGGGACTGACAACATATAGAGA
ATCTGCTCTGTTGTTTGATGCAGAAAAGTCTTCTGCATCAAGTAAGCTTGGCATCACAGTGAC
TGTGGCCCATGAACTGGCCCACCAGTGGTTTGGGAACCTGGTCACTATGGAATGGTGGAATGA
TCTTTGGCTAAATGAAGGATTTGCCAAATTTATGGAGTTTGTGTCTGTCAGTGTGACCCATCC
TGAACTGAAAGTTGGAGATTATTTCTTTGGCAAATGTTTTGACGCAATGGAGGTAGATGCTTT
AAATTCCTCACACCCTGTGTCTACACCTGTGGAAAATCCTGCTCAGATCCGGGAGATGTTTGA
TGATGTTTCTTATGATAAGGGAGCTTGTATTCTGAATATGCTAAGGGAGTATCTTAGCGCTGA
CGCATTTAAAAGTGGTATTGTACAGTATCTCCAGAAGCATAGCTATAAAAATACAAAAAACGA
GGACCTGTGGGATAGTATGGCAAGTATTTGCCCTACAGATGGTGTAAAAGGGATGGATGGCTT
TTGCTCTAGAAGTCAACATTCATCTTCATCCTCACATTGGCATCAGGAAGGGGTGGATGTGAA
AACCATGATGAACACTTGGACACTGCAGAGGGGTTTTCCCCTAATAACCATCACAGTGAGGGG
GAGGAATGTACACATGAAGCAAGAGCACTACATGAAGGGCTCTGACGGCGCCCCGGACACTGG
GTACCTGTGGCATGTTCCATTGACATTCATCACCAGCAAATCCAACATGGTCCATCGATTTTT
GCTAAAAACAAAAACAGATGTGCTCATCCTCCCAGAAGAGGTGGAATGGATCAAATTTAATGT
GGGCATGAATGGCTATTACATTGTGCATTACGAGGATGATGGATGGGACTCTTTGACTGGCCT
TTTAAAAGGAACACACACAGCAGTCAGCAGTAATGATCGGGCAAGTCTCATTAACAATGCATT
TCAGCTCGTCAGCATTGGGAAGCTGTCCATTGAAAAGGCCTTGGATTTATCCCTGTACTTGAA
ACATGAAACTGAAATTATGCCCGTGTTTCAAGGTTTGAATGAGCTGATTCCTATGTATAAGTT
AATGGAGAAAAGAGATATGAATGAAGTGGAAACTCAATTCAAGGCCTTCCTCATCAGGCTGCT
AAGGGACCTCATTGATAAGCAGACATGGACAGACGAGGGCTCAGTCTCAGAGCAAATGCTGCG
GAGTGAACTACTACTCCTCGCCTGTGTGCACAACTATCAGCCGTGCGTACAGAGGGCAGAAGG
CTATTTCAGAAAGTGGAAGGAATCCAATGGAAACTTGAGCCTGCCTGTCGACGTGACCTTGGC
AGTGTTTGCTGTGGGGGCCCAGAGCACAGAAGGCTGGGATTTTCTTTATAGTAAATATCAGTT
TTCTTTGTCCAGTACTGAGAAAAGCCAAATTGAATTTGCCCTCTGCAGAACCCAAAATAAGGA
AAAGCTTCAATGGCTACTAGATGAAAGCTTTAAGGGAGATAAAATAAAAACTCAGGAGTTTCC
ACAAATTCTTACACTCATTGGCAGGAACCCAGTAGGATACCCACTGGCCTGGCAATTTCTGAG
GAAAAACTGGAACAAACTTGTACAAAAGTTTGAACTTGGCTCATCTTCCATAGCCCACATGGT
AATGGGTACAACAAATCAATTCTCCACAAGAACACGGCTTGAAGAGGTAAAAGGATTCTTCAG
CTCTTTGAAAGAAAATGGTTCTCAGCTCCGTTGTGTCCAACAGACAATTGAAACCATTGAAGA
AAACATCGGTTGGATGGATAAGAATTTTGATAAAATCAGAGTGTGGCTGCAAAGTGAAAAGCT
TGAACGTATG**TAA**AAATTCCTCCCTTGCCCGGTTCCTGTTATCTCTAATCACCAACATTTTGT
TGAGTGTATTTTCAAACTAGAGATGGCTGTTTTGGCTCCAACTGGAGATACTTTTTTCCCTTC
AACTCATTTTTTGACTATCCCTGTGAAAAGAATAGCTGTTAGTTTTTCATGAATGGGCTTTTT
CATGAATGGGCTATCGCTACCATGTGTTTTGTTCATCACAGGTGTTGCCCTGCAACGTAAACC
CAAGTGTTGGGTTCCCTGCCACAGAAGAATAAAGTACCTTATTCTTCTCAAAAAAAAAAAAAA
AAAAAAAAAAAA

# FIGURE 464

MVFLPLKWSLATMSFLLSSLLALLTVSTPSWCQSTEASPKRSDGTPFPWNKIRLPEYVIPVHY
DLLIHANLTTLTFWGTTKVEITASQPTSTIILHSHHLQISRATLRKGAGERLSEEPLQVLEHP
PQEQIALLAPEPLLVGLPYTVVIHYAGNLSETFHGFYKSTYRTKEGELRILASTQFEPTAARM
AFPCFDEPAFKASFSIKIRREPRHLAISNMPLVKSVTVAEGLIEDHFDVTVKMSTYLVAFIIS
DFESVSKITKSGVKVSVYAVPDKINQADYALDAAVTLLEFYEDYFSIPYPLPKQDLAAIPDFQ
SGAMENWGLTTYRESALLFDAEKSSASSKLGITVTVAHELAHQWFGNLVTMEWWNDLWLNEGF
AKFMEFVSVSVTHPELKVGDYFFGKCFDAMEVDALNSSHPVSTPVENPAQIREMFDDVSYDKG
ACILNMLREYLSADAFKSGIVQYLQKHSYKNTKNEDLWDSMASICPTDGVKGMDGFCSRSQHS
SSSSHWHQEGVDVKTMMNTWTLQRGFPLITITVRGRNVHMKQEHYMKGSDGAPDTGYLWHVPL
TFITSKSNMVHRFLLKTKTDVLILPEEVEWIKFNVGMNGYYIVHYEDDGWDSLTGLLKGTHTA
VSSNDRASLINNAFQLVSIGKLSIEKALDLSLYLKHETEIMPVFQGLNELIPMYKLMEKRDMN
EVETQFKAFLIRLLRDLIDKQTWTDEGSVSEQMLRSELLLLACVHNYQPCVQRAEGYFRKWKE
SNGNLSLPVDVTLAVFAVGAQSTEGWDFLYSKYQFSLSSTEKSQIEFALCRTQNKEKLQWLLD
ESFKGDKIKTQEFPQILTLIGRNPVGYPLAWQFLRKNWNKLVQKFELGSSSIAHMVMGTTNQF
STRTRLEEVKGFFSSLKENGSQLRCVQQTIETIEENIGWMDKNFDKIRVWLQSEKLERM

**Important features:**

**Signal peptide:**

amino acids 1-34

**N-glycosylation sites:**

amino acids 70-74, 154-158, 414-418, 760-764, 901-905

**Neutral zinc metallopeptidases, zinc-binding region signature:**

amino acids 350-360

# FIGURE 465

CAGCCACAGACGGGTC**ATG**AGCGCGGTATTACTGCTGGCCCTCCTGGGGTTCATCCTCCCACT
GCCAGGAGTGCAGGCGCTGCTCTGCCAGTTTGGGACAGTTCAGCATGTGTGGAAGGTGTCCGA
CCTACCCCGGCAATGGACCCCTAAGAACACCAGCTGCGACAGCGGCTTGGGGTGCCAGGACAC
GTTGATGCTCATTGAGAGCGGACCCCAAGTGAGCCTGGTGCTCTCCAAGGGCTGCACGGAGGC
CAAGGACCAGGAGCCCCGCGTCACTGAGCACCGGATGGGCCCCGGCCTCTCCCTGATCTCCTA
CACCTTCGTGTGCCGCCAGGAGGACTTCTGCAACAACCTCGTTAACTCCCTCCCGCTTTGGGC
CCCACAGCCCCCAGCAGACCCAGGATCCTTGAGGTGCCCAGTCTGCTTGTCTATGGAAGGCTG
TCTGGAGGGGACAACAGAAGAGATCTGCCCCAAGGGGACCACACACTGTTATGATGGCCTCCT
CAGGCTCAGGGGAGGAGGCATCTTCTCCAATCTGAGAGTCCAGGGATGCATGCCCCAGCCAGG
TTGCAACCTGCTCAATGGGACACAGGAAATTGGGCCCGTGGGTATGACTGAGAACTGCAATAG
GAAAGATTTTCTGACCTGTCATCGGGGGACCACCATTATGACACACGGAAACTTGGCTCAAGA
ACCCACTGATTGGACCACATCGAATACCGAGATGTGCGAGGTGGGGCAGGTGTGTCAGGAGAC
GCTGCTGCTCATAGATGTAGGACTCACATCAACCCTGGTGGGGACAAAAGGCTGCAGCACTGT
TGGGGCTCAAAATTCCCAGAAGACCACCATCCACTCAGCCCCTCCTGGGGTGCTTGTGGCCTC
CTATACCCACTTCTGCTCCTCGGACCTGTGCAATAGTGCCAGCAGCAGCAGCGTTCTGCTGAA
CTCCCTCCCTCCTCAAGCTGCCCCTGTCCCAGGAGACCGGCAGTGTCCTACCTGTGTGCAGCC
CCTTGGAACCTGTTCAAGTGGCTCCCCCCGAATGACCTGCCCCAGGGGCGCCACTCATTGTTA
TGATGGGTACATTCATCTCTCAGGAGGTGGGCTGTCCACCAAAATGAGCATTCAGGGCTGCGT
GGCCCAACCTTCCAGCTTCTTGTTGAACCACACCAGACAAATCGGGATCTTCTCTGCGCGTGA
GAAGCGTGATGTGCAGCCTCCTGCCTCTCAGCATGAGGGAGGTGGGGCTGAGGGCCTGGAGTC
TCTCACTTGGGGGGTGGGGCTGGCACTGGCCCCAGCGCTGTGGTGGGGAGTGGTTTGCCCTTC
CTGC**TAA**CTCTATTACCCCCACGATTCTTCACCGCTGCTGACCACCCACACTCAACCTCCCTC
TGACCTCATAACCTAATGGCCTTGGACACCAGATTCTTTCCCATTCTGTCCATGAATCATCTT
CCCCACACACAATCATTCATATCTACTCACCTAACAGCAACACTGGGGAGAGCCTGGAGCATC
CGGACTTGCCCTATGGGAGAGGGGACGCTGGAGGAGTGGCTGCATGTATCTGATAATACAGAC
CCTGTCCTTTCA

# FIGURE 466

MSAVLLLALLGFILPLPGVQALLCQFGTVQHVWKVSDLPRQWTPKNTSCDSGLGCQDTLMLIE
SGPQVSLVLSKGCTEAKDQEPRVTEHRMGPGLSLISYTFVCRQEDFCNNLVNSLPLWAPQPPA
DPGSLRCPVCLSMEGCLEGTTEEICPKGTTHCYDGLLRLRGGGIFSNLRVQGCMPQPGCNLLN
GTQEIGPVGMTENCNRKDFLTCHRGTTIMTHGNLAQEPTDWTTSNTEMCEVGQVCQETLLLID
VGLTSTLVGTKGCSTVGAQNSQKTTIHSAPPGVLVASYTHFCSSDLCNSASSSSVLLNSLPPQ
AAPVPGDRQCPTCVQPLGTCSSGSPRMTCPRGATHCYDGYIHLSGGGLSTKMSIQGCVAQPSS
FLLNHTRQIGIFSAREKRDVQPPASQHEGGGAEGLESLTWGVGLALAPALWWGVVCPSC

# FIGURE 467

GAGGATTTGCCACAGCAGCGGATAGAGCAGGAGAGCACCACCGGAGCCCTTGAGACATCCTTG
AGAAGAGCCACAGCATAAGAGACTGCCCTGCTTGGTGTTTTGCAGG**ATG**ATGGTGGCCCTTCG
AGGAGCTTCTGCATTGCTGGTTCTGTTCCTTGCAGCTTTTCTGCCCCCGCCGCAGTGTACCCA
GGACCCAGCCATGGTGCATTACATCTACCAGCGCTTTCGAGTCTTGGAGCAAGGGCTGGAAAA
ATGTACCCAAGCAACGAGGGCATACATTCAAGAATTCCAAGAGTTCTCAAAAAATATATCTGT
CATGCTGGGAAGATGTCAGACCTACACAAGTGAGTACAAGAGTGCAGTGGGTAACTTGGCACT
GAGAGTTGAACGTGCCCAACGGGAGATTGACTACATACAATACCTTCGAGAGGCTGACGAGTG
CATCGTATCAGAGGACAAGACACTGGCAGAAATGTTGCTCCAAGAAGCTGAAGAAGAGAAAAA
GATCCGGACTCTGCTGAATGCAAGCTGTGACAACATGCTGATGGGCATAAAGTCTTTGAAAAT
AGTGAAGAAGATGATGGACACACATGGCTCTTGGATGAAAGATGCTGTCTATAACTCTCCAAA
GGTGTACTTATTAATTGGATCCAGAAACAACACTGTTTGGGAATTTGCAAACATACGGGCATT
CATGGAGGATAACACCAAGCCAGCTCCCCGGAAGCAAATCCTAACACTTTCCTGGCAGGGAAC
AGGCCAAGTGATCTACAAAGGTTTTCTATTTTTTCATAACCAAGCAACTTCTAATGAGATAAT
CAAATATAACCTGCAGAAGAGGACTGTGGAAGATCGAATGCTGCTCCCAGGAGGGGTAGGCCG
AGCATTGGTTTACCAGCACTCCCCCTCAACTTACATTGACCTGGCTGTGGATGAGCATGGGCT
CTGGGCCATCCACTCTGGGCCAGGCACCCATAGCCATTTGGTTCTCACAAAGATTGAGCCGGG
CACACTGGGAGTGGAGCATTCATGGGATACCCCATGCAGAAGCCAGGATGCTGAAGCCTCATT
CCTCTTGTGTGGGGTTCTCTATGTGGTCTACAGTACTGGGGGCCAGGGCCCTCATCGCATCAC
CTGCATCTATGATCCACTGGGCACTATCAGTGAGGAGGACTTGCCCAACTTGTTCTTCCCCAA
GAGACCAAGAAGTCACTCCATGATCCATTACAACCCCAGAGATAAGCAGCTCTATGCCTGGAA
TGAAGGAAACCAGATCATTTACAAACTCCAGACAAAGAGAAAGCTGCCTCTGAAG**TAA**TGCAT
TACAGCTGTGAGAAAGAGCACTGTGGCTTTGGCAGCTGTTCTACAGGACAGTGAGGCTATAGC
CCCTTCACAATATAGTATCCCTCTAATCACACACAGGAAGAGTGTGTAGAAGTGGAAATACGT
ATGCCTCCTTTCCCAAATGTCACTGCCTTAGGTATCTTCCAAGAGCTTAGATGAGAGCATATC
ATCAGGAAAGTTTCAACAATGTCCATTACTCCCCCAAACCTCCTGGCTCTCAAGGATGACCAC
ATTCTGATACAGCCTACTTCAAGCCTTTTGTTTTACTGCTCCCCAGCATTTACTGTAACTCTG
CCATCTTCCCTCCCACAATTAGAGTTGTATGCCAGCCCCTAATATTCACCACTGGCTTTTCTC
TCCCCTGGCCTTTGCTGAAGCTCTTCCCTCTTTTTCAAATGTCTATTGATATTCTCCCATTTT
CACTGCCCAACTAAAATACTATTAATATTTCTTTCTTTTCTTTTCTTTTTTTTGAGACAAGGT
CTCACTATGTTGCCCAGGCTGGTCTCAAACTCCAGAGCTCAAGAGATCCTCCTGCCTCAGCCT
CCTAAGTACCTGGGATTACAGGCATGTGCCACCACACCTGGCTTAAAATACTATTTCTTATTG
AGGTTTAACCTCTATTTCCCCTAGCCCTGTCCTTCCACTAAGCTTGGTAGATGTAATAATAAA
GTGAAAATATTAACATTTGAATATCGCTTTCCAGGTGTGGAGTGTTTGCACATCATTGAATTC
TCGTTTCACCTTTGTGAAACATGCACAAGTCTTTACAGCTGTCATTCTAGAGTTTAGGTGAGT
AACACAATTACAAAGTGAAAGATACAGCTAGAAAATACTACAAATCCCATAGTTTTTCCATTG
CCCAAGGAAGCATCAAATACGTATGTTTGTTCACCTACTCTTATAGTCAATGCGTTCATCGTT
TCAGCCTAAAAATAATAGTCTGTCCCTTTAGCCAGTTTTCATGTCTGCACAAGACCTTTCAAT
AGGCCTTTCAAATGATAATTCCTCCAGAAAACCAGTCTAAGGGTGAGGACCCCAACTCTAGCC
TCCTCTTGTCTTGCTGTCCTCTGTTTCTCTCTTTCTGCTTTAAATTCAATAAAAGTGACACTG
AGCAAAAAAAAAAAAAA

# FIGURE 468

MMVALRGASALLVLFLAAFLPPPQCTQDPAMVHYIYQRFRVLEQGLEKCTQATRAYIQEFQEF
SKNISVMLGRCQTYTSEYKSAVGNLALRVERAQREIDYIQYLREADECIVSEDKTLAEMLLQE
AEEEKKIRTLLNASCDNMLMGIKSLKIVKKMMDTHGSWMKDAVYNSPKVYLLIGSRNNTVWEF
ANIRAFMEDNTKPAPRKQILTLSWQGTGQVIYKGFLFFHNQATSNEIIKYNLQKRTVEDRMLL
PGGVGRALVYQHSPSTYIDLAVDEHGLWAIHSGPGTHSHLVLTKIEPGTLGVEHSWDTPCRSQ
DAEASFLLCGVLYVVYSTGGQGPHRITCIYDPLGTISEEDLPNLFFPKRPRSHSMIHYNPRDK
QLYAWNEGNQIIYKLQTKRKLPLK

# FIGURE 469

TGGCCTCCCCAGCTTGCCAGGCACAAGGCTGAGCGGGAGGAAGCGAGAGGCATCTAAGCAGGC
AGTGTTTTGCCTTCACCCCAAGTGACC**ATG**AGAGGTGCCACGCGAGTCTCAATCATGCTCCTC
CTAGTAACTGTGTCTGACTGTGCTGTGATCACAGGGGCCTGTGAGCGGGATGTCCAGTGTGGG
GCAGGCACCTGCTGTGCCATCAGCCTGTGGCTTCGAGGGCTGCGGATGTGCACCCCGCTGGGG
CGGGAAGGCGAGGAGTGCCACCCCGGCAGCCACAAGGTCCCCTTCTTCAGGAAACGCAAGCAC
CACACCTGTCCTTGCTTGCCCAACCTGCTGTGCTCCAGGTTCCCGGACGGCAGGTACCGCTGC
TCCATGGACTTGAAGAACATCAATTTT**TAG**GCGCTTGCCTGGTCTCAGGATACCCACCATCCT
TTTCCTGAGCACAGCCTGGATTTTTATTTCTGCCATGAAACCCAGCTCCCATGACTCTCCCAG
TCCCTACACTGACTACCCTGATCTCTCTTGTCTAGTACGCACATATGCACACAGGCAGACATA
CCTCCCATCATGACATGGTCCCCAGGCTGGCCTGAGGATGTCACAGCTTGAGGCTGTGGTGTG
AAAGGTGGCCAGCCTGGTTCTCTTCCCTGCTCAGGCTGCCAGAGAGGTGGTAAATGGCAGAAA
GGACATTCCCCCTCCCCTCCCCAGGTGACCTGCTCTCTTTCCTGGGCCCTGCCCCTCTCCCCA
CATGTATCCCTCGGTCTGAATTAGACATTCCTGGGCACAGGCTCTTGGGTGCATTGCTCAGAG
TCCCAGGTCCTGGCCTGACCCTCAGGCCCTTCACGTGAGGTCTGTGAGGACCAATTTGTGGGT
AGTTCATCTTCCCTCGATTGGTTAACTCCTTAGTTTCAGACCACAGACTCAAGATTGGCTCTT
CCCAGAGGGCAGCAGACAGTCACCCCAAGGCAGGTGTAGGGAGCCCAGGGAGGCCAATCAGCC
CCCTGAAGACTCTGGTCCCAGTCAGCCTGTGGCTTGTGGCCTGTGACCTGTGACCTTCTGCCA
GAATTGTCATGCCTCTGAGGCCCCCTCTTACCACACTTTACCAGTTAACCACTGAAGCCCCCA
ATTCCCACAGCTTTTCCATTAAAATGCAAATGGTGGTGGTTCAATCTAATCTGATATTGACAT
ATTAGAAGGCAATTAGGGTGTTTCCTTAAACAACTCCTTTCCAAGGATCAGCCCTGAGAGCAG
GTTGGTGACTTTGAGGAGGGCAGTCCTCTGTCCAGATTGGGGTGGGAGCAAGGGACAGGGAGC
AGGGCAGGGGCTGAAAGGGGCACTGATTCAGACCAGGGAGGCAACTACACACCAACATGCTGG
CTTTAGAATAAAAGCACCAACTGAAAAAA

# FIGURE 470

MRGATRVSIMLLLVTVSDCAVITGACERDVQCGAGTCCAISLWLRGLRMCTPLGREGEECHPG
SHKVPFFRKRKHHTCPCLPNLLCSRFPDGRYRCSMDLKNINF

**Important feratures:**

**Signal peptide:**

amino acids 1-19

**Tyrosine kinase phosphorylation site:**

amino acids 88-95

**N-myristoylation sites:**

amino acids 33-39, 35-41, 46-52

# FIGURE 471

AGCGCCCGGGCGTCGGGGCGGTAAAAGGCCGGCAGAAGGGAGGCACTTGAGAA**ATG**TCTTTCC
TCCAGGACCCAAGTTTCTTCACCATGGGGATGTGGTCCATTGGTGCAGGAGCCCTGGGGGCTG
CTGCCTTGGCATTGCTGCTTGCCAACACAGACGTGTTTCTGTCCAAGCCCCAGAAAGCGGCCC
TGGAGTACCTGGAGGATATAGACCTGAAAACACTGGAGAAGGAACCAAGGACTTTCAAAGCAA
AGGAGCTATGGGAAAAAAATGGAGCTGTGATTATGGCCGTGCGGAGGCCAGGCTGTTTCCTCT
GTCGAGAGGAAGCTGCGGATCTGTCCTCCCTGAAAAGCATGTTGGACCAGCTGGGCGTCCCCC
TCTATGCAGTGGTAAAGGAGCACATCAGGACTGAAGTGAAGGATTTCCAGCCTTATTTCAAAG
GAGAAATCTTCCTGGATGAAAAGAAAAAGTTCTATGGTCCACAAAGGCGGAAGATGATGTTTA
TGGGATTTATCCGTCTGGGAGTGTGGTACAACTTCTTCCGAGCCTGGAACGGAGGCTTCTCTG
GAAACCTGGAAGGAGAAGGCTTCATCCTTGGGGGAGTTTTCGTGGTGGGATCAGGAAAGCAGG
GCATTCTTCTTGAGCACCGAGAAAAAGAATTTGGAGACAAAGTAAACCTACTTTCTGTTCTGG
AAGCTGCTAAGATGATCAAACCACAGACTTTGGCCTCAGAGAAAAAA**TGA**TTGTGTGAAACTG
CCCAGCTCAGGGATAACCAGGGACATTCACCTGTGTTCATGGGATGTATTGTTTCCACTCGTG
TCCCTAAGGAGTGAGAAACCCATTTATACTCTACTCTCAGTATGGATTATTAATGTATTTTAA
TATTCTGTTTAGGCCCACTAAGGCAAAATAGCCCCAAAACAAGACTGACAAAAATCTGAAAAA
CTAATGAGGATTATTAAGCTAAAACCTGGGAAATAGGAGGCTTAAAATTGACTGCCAGGCTGG
GTGCAGTGGCTCACACCTGTAATCCCAGCACTTTGGGAGGCCAAGGTGAGCAAGTCACTTGAG
GTCGGGAGTTCGAGACCAGCCTGAGCAACATGGCGAAACCCCGTCTCTACTAAAAATACAAAA
ATCACCCGGGTGTGGTGGCAGGCACCTGTAGTCCCAGCTACCCGGGAGGCTGAGGCAGGAGAA
TCACTTGAACCTGGGAGGTGGAGGTTGCGGTGAGCTGAGATCACACCACTGTATTCCAGCCTG
GGTGACTGAGACTCTAACTAA

# FIGURE 472

MSFLQDPSFFTMGMWSIGAGALGAAALALLLANTDVFLSKPQKAALEYLEDIDLKTLEKEPRT
FKAKELWEKNGAVIMAVRRPGCFLCREEAADLSSLKSMLDQLGVPLYAVVKEHIRTEVKDFQP
YFKGEIFLDEKKKFYGPQRRKMMFMGFIRLGVWYNFFRAWNGGFSGNLEGEGFILGGVFVVGS
GKQGILLEHREKEFGDKVNLLSVLEAAKMIKPQTLASEKK

# FIGURE 473

AATATATCATCTATTTATCATTAATCAATAATGTATTCTTTTATTCCAATAACATTTGGGTTT
TGGGATTTTAATTTTCAAACACAGCAGA**ATG**ACATTTTTTCTGTCACTATTATTATTGTTGGT
ATGTGAAGCTATTTGGAGATCCAATTCAGGAAGCAACACATTGGAGAATGGCTACTTTCTATC
AAGAAATAAAGAGAACCACAGTCAACCCACACAATCATCTTTAGAAGACAGTGTGACTCCTAC
CAAAGCTGTCAAAACCACAGGCAAGGGCATAGTTAAAGGACGGAATCTTGACTCAAGAGGGTT
AATTCTTGGTGCTGAAGCCTGGGGCAGGGGTGTAAAGAAAAACACT**TAG**ATTCAATGATTGTA
AATTTAAGGCAAATACACATATTAGTATTACCTTAGTGTAATGTATCCCTGTCATATATACAA
TAAGGTGAAATTATAAGTACCCTATGCAGTTGGCTGGACAGTTCTAAATTGGACTTTATTAAT
TTTTAAAATCAGTAACTGATTTATCACTGGCTATGTGCTTAGATCTACAGGAGATCATATAAT
TTGATACAAATAAAAGAAAAGTGTTCTCTCCCCTTACAGAATTGACATTTTAAATGCGATACA
GTTAGAATAGGAAATATGACATTAGAAAGGAAGAATGACAGGGAGAAAGGAAAGAAGGGAAAA
TGTTGCCAAGGAAAAAAAAA

# FIGURE 474

MTFFLSLLLLLVCEAIWRSNSGSNTLENGYFLSRNKENHSQPTQSSLEDSVTPTKAVKTTGKG
IVKGRNLDSRGLILGAEAWGRGVKKNT

# FIGURE 475

GACAGTGGAGGGCAGTGGAGAGGACCGCGCTGTCCTGCTGTCACCAAGAGCTGGAGACACCAT
CTCCCACCGAGAGTC**ATG**GCCCCATTGGCCCTGCACCTCCTCGTCCTCGTCCCCATCCTCCTC
AGCCTGGTGGCCTCCCAGGACTGGAAGGCTGAACGCAGCCAAGACCCCTTCGAGAAATGCATG
CAGGATCCTGACTATGAGCAGCTGCTCAAGGTGGTGACCTGGGGGCTCAATCGGACCCTGAAG
CCCCAGAGGGTGATTGTGGTTGGCGCTGGTGTGGCCGGGCTGGTGGCCGCCAAGGTGCTCAGC
GATGCTGGACACAAGGTCACCATCCTGGAGGCAGATAACAGGATCGGGGGCCGCATCTTCACC
TACCGGGACCAGAACACGGGCTGGATTGGGGAGCTGGGAGCCATGCGCATGCCCAGCTCTCAC
AGGATCCTCCACAAGCTCTGCCAGGGCCTGGGGCTCAACCTGACCAAGTTCACCCAGTACGAC
AAGAACACGTGGACGGAGGTGCACGAAGTGAAGCTGCGCAACTATGTGGTGGAGAAGGTGCCC
GAGAAGCTGGGCTACGCCTTGCGTCCCCAGGAAAAGGGCCACTCGCCCGAAGACATCTACCAG
ATGGCTCTCAACCAGGCCCTCAAAGACCTCAAGGCACTGGGCTGCAGAAAGGCGATGAAGAAG
TTTGAAAGGCACACGCTCTTGGAATATCTTCTCGGGGAGGGGAACCTGAGCCGGCCGGCCGTG
CAGCTTCTGGGAGACGTGATGTCCGAGGATGGCTTCTTCTATCTCAGCTTCGCCGAGGCCCTC
CGGGCCCACAGCTGCCTCAGCGACAGACTCCAGTACAGCCGCATCGTGGGTGGCTGGGACCTG
CTGCCGCGCGCGCTGCTGAGCTCGCTGTCCGGGCTTGTGCTGTTGAACGCGCCCGTGGTGGCG
ATGACCCAGGGACCGCACGATGTGCACGTGCAGATCGAGACCTCTCCCCCGGCGCGGAATCTG
AAGGTGCTGAAGGCCGACGTGGTGCTGCTGACGGCGAGCGGACCGGCGGTGAAGCGCATCACC
TTCTCGCCGCCGCTGCCCCGCCACATGCAGGAGGCGCTGCGGAGGCTGCACTACGTGCCGGCC
ACCAAGGTGTTCCTAAGCTTCCGCAGGCCCTTCTGGCGCGAGGAGCACATTGAAGGCGGCCAC
TCAAACACCGATCGCCCGTCGCGCATGATTTCTACCCGCCGCCGCGCGAGGGCGCGCTGCTG
CTGGCCTCGTACACGTGGTCGGACGCGGCGGCAGCGTTCGCCGGCTTGAGCCGGGAAGAGGCG
TTGCGCTTGGCGCTCGACGACGTGGCGGCATTGCACGGGCCTGTCGTGCGCCAGCTCTGGGAC
GGCACCGGCGTCGTCAAGCGTTGGGCGGAGGACCAGCACAGCCAGGGTGGCTTTGTGGTACAG
CCGCCGGCGCTCTGGCAAACCGAAAAGGATGACTGGACGGTCCCTTATGGCCGCATCTACTTT
GCCGGCGAGCACACCGCCTACCCGCACGGCTGGGTGGAGACGGCGGTCAAGTCGGCGCTGCGC
GCCGCCATCAAGATCAACAGCCGGAAGGGGCCTGCATCGGACACGGCCAGCCCCGAGGGGCAC
GCATCTGACATGGAGGGGCAGGGGCATGTGCATGGGGTGGCCAGCAGCCCCTCGCATGACCTG
GCAAAGGAAGAAGGCAGCCACCCTCCAGTCCAAGGCCAGTTATCTCTCCAAAACACGACCCAC
ACGAGGACCTCGCAT**TAA**AGTATTTTCGGAAAAAAAAAAAAAAAAAAAAAAAAAAAAAAAAA
AAAAAAAAAAAAAAAAA

# FIGURE 476

MAPLALHLLVLVPILLSLVASQDWKAERSQDPFEKCMQDPDYEQLLKVVTWGLNRTLKPQRVI
VVGAGVAGLVAAKVLSDAGHKVTILEADNRIGGRIFTYRDQNTGWIGELGAMRMPSSHRILHK
LCQGLGLNLTKFTQYDKNTWTEVHEVKLRNYVVEKVPEKLGYALRPQEKGHSPEDIYQMALNQ
ALKDLKALGCRKAMKKFERHTLLEYLLGEGNLSRPAVQLLGDVMSEDGFFYLSFAEALRAHSC
LSDRLQYSRIVGGWDLLPRALLSSLSGLVLLNAPVVAMTQGPHDVHVQIETSPPARNLKVLKA
DVVLLTASGPAVKRITFSPPLPRHMQEALRRLHYVPATKVFLSFRRPFWREEHIEGGHSNTDR
PSRMIFYPPPREGALLLASYTWSDAAAAFAGLSREEALRLALDDVAALHGPVVRQLWDGTGVV
KRWAEDQHSQGGFVVQPPALWQTEKDDWTVPYGRIYFAGEHTAYPHGWVETAVKSALRAAIKI
NSRKGPASDTASPEGHASDMEGQGHVHGVASSPSHDLAKEEGSHPPVQGQLSLQNTTHTRTSH

**Important features:**

**Signal peptide:**

amino acids 1-21

# FIGURE 477

CTGACATGGCCTGACTCGGGACAGCTCAGAGCAGGGCAGAACTGGGGACACTCTGGGCCGGCCTTCTGCCTGC**AT**
**G**GACGCTCTGAAGCCACCCTGTCTCTGGAGGAACCACGAGCGAGGGAAGAAGGACAGGGACTCGTGTGGCAGGAA
GAACTCAGAGCCGGGAAGCCCCCATTCACTAGAAGCACTGAGAGATGCGGCCCCCTCGCAGGGTCTGAATTTCCT
GCTGCTGTTCACAAAGATGCTTTTTATCTTTAACTTTTTGTTTTCCCCACTTCCGACCCCGGCGTTGATCTGCAT
CCTGACATTTGGAGCTGCCATCTTCTTGTGGCTGATCACCAGACCTCAACCCGTCTTACCTCTTCTTGACCTGAA
CAATCAGTCTGTGGGAATTGAGGGAGGAGCACGGAAGGGGGTTTCCCAGAAGAACAATGACCTAACAAGTTGCTG
CTTCTCAGATGCCAAGACTATGTATGAGGTTTTCCAAAGAGGACTCGCTGTGTCTGACAATGGGCCCTGCTTGGG
ATATAGAAAACCAAACCAGCCCTACAGATGGCTATCTTACAAACAGGTGTCTGATAGAGCAGAGTACCTGGGTTC
CTGTCTCTTGCATAAAGGTTATAAATCATCACCAGACCAGTTTGTCGGCATCTTTGCTCAGAATAGGCCAGAGTG
GATCATCTCCGAATTGGCTTGTTACACGTACTCTATGGTAGCTGTACCTCTGTATGACACCTTGGGACCAGAAGC
CATCGTACATATTGTCAACAAGGCTGATATCGCCATGGTGATCTGTGACACACCCCAAAAGGCATTGGTGCTGAT
AGGGAATGTAGAGAAAGGCTTCACCCCGAGCCTGAAGGTGATCATCCTTATGGACCCCTTTGATGATGACCTGAA
GCAAAGAGGGGAGAAGAGTGGAATTGAGATCTTATCCCTATATGATGCTGAGAACCTAGGCAAAGAGCACTTCAG
AAAACCTGTGCCTCCTAGCCCAGAAGACCTGAGCGTCATCTGCTTCACCAGTGGGACCACAGGTGACCCCAAAGG
AGCCATGATAACCCATCAAAATATTGTTTCAAATGCTGCTGCCTTTCTCAAATGTGTGGAGCATGCTTATGAGCC
CACTCCTGATGATGTGGCCATATCCTACCTCCCTCTGGCTCATATGTTTGAGAGGATTGTACAGGCTGTTGTGTA
CAGCTGTGGAGCCAGAGTTGGATTCTTCCAAGGGGATATTCGGTTGCTGGCTGACGACATGAAGACTTTGAAGCC
CACATTGTTTCCCGCGGTGCCTCGACTCCTTAACAGGATCTACGATAAGGTACAAAATGAGGCCAAGACACCCTT
GAAGAAGTTCTTGTTGAAGCTGGCTGTTTCCAGTAAATTCAAAGAGCTTCAAAAGGGTATCATCAGGCATGATAG
TTTCTGGGACAAGCTCATCTTTGCAAAGATCCAGGACAGCCTGGGCGGAAGGGTTCGTGTAATTGTCACTGGAGC
TGCCCCCATGTCCACTTCAGTCATGACATTCTTCCGGGCAGCAATGGGATGTCAGGTGTATGAAGCTTATGGTCA
AACAGAATGCACAGGTGGCTGTACATTTACATTACCTGGGGACTGGACATCAGGTCACGTTGGGGTGCCCCTGGC
TTGCAATTACGTGAAGCTGGAAGATGTGGCTGACATGAACTACTTTACAGTGAATAATGAAGGAGAGGTCTGCAT
CAAGGGTACAAACGTGTTCAAAGGATACCTGAAGGACCCTGAGAAGACACAGGAAGCCCTGGACAGTGATGGCTG
GCTTCACACAGGAGACATTGGTCGCTGGCTCCCGAATGGAACTCTGAAGATCATCGACCGTAAAAAGAACATTTT
CAAGCTGGCCCAAGGAGAATACATTGCACCAGAGAAGATAGAAAATATCTACAACAGGAGTCAACCAGTGTTACA
AATTTTTGTACACGGGGAGAGCTTACGGTCATCCTTAGTAGGAGTGGTGGTTCCTGACACAGATGTACTTCCCTC
ATTTGCAGCCAAGCTTGGGGTGAAGGGCTCCTTTGAGGAACTGTGCCAAAACCAAGTTGTAAGGGAAGCCATTTT
AGAAGACTTGCAGAAAATTGGGAAAGAAAGTGGCCTTAAAACTTTTGAACAGGTCAAAGCCATTTTTCTTCATCC
AGAGCCATTTTCCATTGAAAATGGGCTCTTGACACCAACATTGAAAGCAAAGCGAGGAGAGCTTTCCAAATACTT
TCGGACCCAAATTGACAGCCTGTATGAGCACATCCAGGAT**TAG**GATAAGGTACTTAAGTACCTGCCGGCCCACTG
TGCACTGCTTGTGAGAAAATGGATTAAAAACTATTCTTACATTTGTTTTGCCTTTCCTCCTATTTTTTTTAACC
TGTTAAACTCTAAAGCCATAGCTTTTGTTTTATATTGAGACATATAATGTGTAAACTTAGTTCCCAAATAAATCA
ATCCTGTCTTTCCCATCTTCGATGTTGCTAATATTAAGGCTTCAGGGCTACTTTTATCAACATGCCTGTCTTCAA
GATCCCAGTTTATGTTCTGTGTCCTTCCTCATGATTTCCAACCTTAATACTATTAGTAACCACAAGTTCAAGGGT
CAAAGGGACCCTCTGTGCCTTCTTCTTTGTTTTGTGATAAACATAACTTGCCAACAGTCTCTATGCTTATTTACA
TCTTCTACTGTTCAAACTAAGAGATTTTTAAATTCTGAAAAACTGCTTACAATTCATGTTTTCTAGCCACTCCAC
AAACCACTAAAATTTTAGTTTTAGCCTATCACTCATGTCAATCATATCTATGAGACAAATGTCTCCGATGCTCTT
CTGCGTAAATTAAATTGTGTACTGAAGGGAAAAGTTTGATCATACCAAACATTTCCTAAACTCTCTAGTTAGATA
TCTGACTTGGGAGTATTAAAAATTGGGTCTATGACATACTGTCCAAAAGGAATGCTGTTCTTAAAGCATTATTTA
CAGTAGGAACTGGGGAGTAAATCTGTTCCCTACAGTTTGCTGCTGAGCTGGAAGCTGTGGGGGAAGGAGTTGACA
GGTGGGCCCAGTGAACTTTTCCAGTAAATGAAGCAAGCACTGAATAAAAACCTCCTGAACTGGGAACAAAGATCT
ACAGGCAAGCAAGATGCCCACACAACAGGCTTATTTTCTGTGAAGGAACCAACTGATCTCCCCCACCCTTGGATT
AGAGTTCCTGCTCTACCTTACCCACAGATAACACATGTTGTTTCTACTTGTAAATGTAAAGTCTTTAAAATAAAC
TATTACAGATAAAAAA

# FIGURE 478

MDALKPPCLWRNHERGKKDRDSCGRKNSEPGSPHSLEALRDAAPSQGLNFLLLFTKMLFIFNF
LFSPLPTPALICILTFGAAIFLWLITRPQPVLPLLDLNNQSVGIEGGARKGVSQKNNDLTSCC
FSDAKTMYEVFQRGLAVSDNGPCLGYRKPNQPYRWLSYKQVSDRAEYLGSCLLHKGYKSSPDQ
FVGIFAQNRPEWIISELACYTYSMVAVPLYDTLGPEAIVHIVNKADIAMVICDTPQKALVLIG
NVEKGFTPSLKVIILMDPFDDDLKQRGEKSGIEILSLYDAENLGKEHFRKPVPPSPEDLSVIC
FTSGTTGDPKGAMITHQNIVSNAAAFLKCVEHAYEPTPDDVAISYLPLAHMFERIVQAVVYSC
GARVGFFQGDIRLLADDMKTLKPTLFPAVPRLLNRIYDKVQNEAKTPLKKFLLKLAVSSKFKE
LQKGIIRHDSFWDKLIFAKIQDSLGGRVRVIVTGAAPMSTSVMTFFRAAMGCQVYEAYGQTEC
TGGCTFTLPGDWTSGHVGVPLACNYVKLEDVADMNYFTVNNEGEVCIKGTNVFKGYLKDPEKT
QEALDSDGWLHTGDIGRWLPNGTLKIIDRKKNIFKLAQGEYIAPEKIENIYNRSQPVLQIFVH
GESLRSSLVGVVVPDTDVLPSFAAKLGVKGSFEELCQNQVVREAILEDLQKIGKESGLKTFEQ
VKAIFLHPEPFSIENGLLTPTLKAKRGELSKYFRTQIDSLYEHIQD

**Important features:**

**Type II transmembrane domain:**

amino acids 61-80

**Putative AMP-binding domain signature.**

amino acids 314-325

**N-glycosylation site.**

amino acids 102-105, 588-591 and 619-622

# FIGURE 479

GGAGGCGGAGGCCGCGGCGAGCCGGGCCGAGCAGTGAGGGCCCTAGCGGGGCCCGAGCGGGGC
CCGGGGCCCCTAAGCCATTCCTGAAGTCATGGGCTGGCCAGGACATTGGTGACCCGCCAATCC
GGT**ATG**GACGACTGGAAGCCCAGCCCCCTCATCAAGCCCTTTGGGGCTCGGAAGAAGCGGAGC
TGGTACCTTACCTGGAAGTATAAACTGACAAACCAGCGGGCCCTGCGGAGATTCTGTCAGACA
GGGGCCGTGCTTTTCCTGCTGGTGACTGTCATTGTCAATATCAAGTTGATCCTGGACACTCGG
CGAGCCATCAGTGAAGCCAATGAAGACCCAGAGCCAGAGCAAGACTATGATGAGGCCCTAGGC
CGCCTGGAGCCCCCACGGCGCAGAGGCAGTGGTCCCCGGCGGGTCCTGGACGTAGAGGTGTAT
TCAAGTCGCAGCAAAGTATATGTGGCAGTGGATGGCACCACGGTGCTGGAGGATGAGGCCCGG
GAGCAGGGCCGGGGCATCCATGTCATTGTCCTCAACCAGGCCACGGGCCACGTGATGGCAAAA
CGTGTGTTTGACACGTACTCACCTCATGAGGATGAGGCCATGGTGCTATTCCTCAACATGGTA
GCGCCCGGCCGAGTGCTCATCTGCACTGTCAAGGATGAGGGCTCCTTCCACCTCAAGGACACA
GCCAAGGCTCTGCTGAGGAGCCTGGGCAGCCAGGCTGGCCCTGCCCTGGGCTGGAGGGACACA
TGGGCCTTCGTGGGACGAAAAGGAGGTCCTGTCTTCGGGGAGAAACATTCTAAGTCACCTGCC
CTCTCTTCCTGGGGGGACCCAGTCCTGCTGAAGACAGATGTGCCATTGAGCTCAGCAGAAGAG
GCAGAGTGCCACTGGGCAGACACAGAGCTGAACCGTCGCCGCCGGCGCTTCTGCAGCAAAGTT
GAGGGCTATGGAAGTGTATGCAGCTGCAAGGACCCCACACCCATCGAGTTCAGCCCTGACCCA
CTCCCAGACAACAAGGTCCTCAATGTGCCTGTGGCTGTCATTGCAGGGAACCGACCCAATTAC
CTGTACAGGATGCTGCGCTCTCTGCTTTCAGCCCAGGGGGTGTCTCCTCAGATGATAACAGTT
TTCATTGACGGCTACTATGAGGAACCCATGGATGTGGTGGCACTGTTTGGTCTGAGGGGCATC
CAGCATACTCCCATCAGCATCAAGAATGCCCGCGTGTCTCAGCACTACAAGGCCAGCCTCACT
GCCACTTTCAACCTGTTTCCGGAGGCCAAGTTTGCTGTGGTTCTGGAAGAGGACCTGGACATT
GCTGTGGATTTTTTCAGTTTCCTGAGCCAATCCATCCACCTACTGGAGGAGGATGACAGCCTG
TACTGCATCTCTGCCTGGAATGACCAGGGGTATGAACACACGGCTGAGGACCCAGCACTACTG
TACCGTGTGGAGACCATGCCTGGGCTGGGCTGGGTGCTCAGGAGGTCCTTGTACAAGGAGGAG
CTTGAGCCCAAGTGGCCTACACCGGAAAAGCTCTGGGATTGGGACATGTGGATGCGGATGCCT
GAACAACGCCGGGGCCGAGAGTGCATCATCCCTGACGTTTCCCGATCCTACCACTTTGGCATC
GTCGGCCTCAACATGAATGGCTACTTTCACGAGGCCTACTTCAAGAAGCACAAGTTCAACACG
GTTCCAGGTGTCCAGCTCAGGAATGTGGACAGTCTGAAGAAAGAAGCTTATGAAGTGGAAGTT
CACAGGCTGCTCAGTGAGGCTGAGGTTCTGGACCACAGCAAGAACCCTTGTGAAGACTCTTTC
CTGCCAGACACACAGAGGGCCACACCTACGTGGCCTTTATTCGAATGGAGAAAGATGATGACTTC
ACCACCTGGACCCAGCTTGCCAAGTGCCTCCATATCTGGGACCTGGATGTGCGTGGCAACCAT
CGGGGCCTGTGGAGATTGTTTCGGAAGAAGAACCACTTCCTGGTGGTGGGGGTCCCGGCTTCC
CCCTACTCAGTGAAGAAGCCACCCTCAGTCACCCCAATTTTCCTGGAGCCACCCCCAAAGGAG
GAGGGAGCCCCAGGAGCCCCAGAACAGACA**TGA**GACCTCCTCCAGGACCCTGCGGGGCTGGGT
ACTGTGTACCCCCAGGCTGGCTAGCCCTTCCCTCCATCCTGTAGGATTTTGTAGATGCTGGTA
GGGGCTGGGGCTACCTTGTTTTAACATGAGACTTAATTACTAACTCCAAGGGGAGGGTTCCC
CTGCTCCAACACCCCGTTCCTGAGTTAAAAGTCTATTTATTTACTTCCTTGTTGGAGAAGGGC
AGGAGAGTACCTGGGAATCATTACGATCCCTAGCAGCTCATCCTGCCCTTTGAATACCCTCAC
TTTCCAGGCCTGGCTCAGAATCTAACCTATTTATTGACTGTCCTGAGGGCCTTGAAAACAGGC
CGAACCTGGAGGGCCTGGATTTCTTTTTGGGCTGGAATGCTGCCCTGAGGGTGGGGCTGGCTC
TTACTCAGGAAACTGCTGTGCCCAACCCATGGACAGGCCCAGCTGGGGCCCACATGCTGACAC
AGACTCACTCAGAGACCCTTAGACACTGGACCAGGCCTCCTCTCAGCCTTCTCTTTGTCCAGA
TTTCCAAAGCTGGATAAGTTGGTCATTGATTAAAAAAGGAGAAGCCCTCTGGGAAAAAAAAAA
AAAAAAAAAAAAAAA

# FIGURE 480

MDDWKPSPLIKPFGARKKRSWYLTWKYKLTNQRALRRFCQTGAVLFLLVTVIVNIKLILDTRR
AISEANEDPEPEQDYDEALGRLEPPRRRGSGPRRVLDVEVYSSRSKVYYVAVDGTTVLEDEARE
QGRGIHVIVLNQATGHVMAKRVFDTYSPHEDEAMVLFLNMVAPGRVLICTVKDEGSFHLKDTA
KALLRSLGSQAGPALGWRDTWAFVGRKGGPVFGEKHSKSPALSSWGDPVLLKTDVPLSSAEEA
ECHWADTELNRRRRRFCSKVEGYGSVCSCKDPTPIEFSPDPLPDNKVLNVPVAVIAGNRPNYL
YRMLRSLLSAQGVSPQMITVFIDGYYEEPMDVVALFGLRGIQHTPISIKNARVSQHYKASLTA
TFNLFPEAKFAVVLEEDLDIAVDFFSFLSQSIHLLEEDDSLYCISAWNDQGYEHTAEDPALLY
RVETMPGLGWVLRRSLYKEELEPKWPTPEKLWDWDMWMRMPEQRRGRECIIPDVSRSYHFGIV
GLNMNGYFHEAYFKKHKFNTVPGVQLRNVDSLKKEAYEVEVHRLLSEAEVLDHSKNPCEDSFL
PDTEGHTYVAFIRMEKDDDFTTWTQLAKCLHIWDLDVRGNHRGLWRLFRKKNHFLVVGVPASP
YSVKKPPSVTPIFLEPPPKEEGAPGAPEQT

**Important features:**

**Transmembrane domain:**

amino acids 38-55

**Homologous region to Mouse GNT1**

amino acids 229-660

# FIGURE 481

GAAAGA**ATG**TTGTGGCTGCTCTTTTTTCTGGTGACTGCCATTCATGCTGAACTCTGTCAACCA
GGTGCAGAAAATGCTTTTAAAGTGAGACTTAGTATCAGAACAGCTCTGGGAGATAAAGCATAT
GCCTGGGATACCAATGAAGAATACCTCTTCAAAGCGATGGTAGCTTTCTCCATGAGAAAAGTT
CCCAACAGAGAAGCAACAGAAATTTCCCATGTCCTACTTTGCAATGTAACCCAGAGGGTATCA
TTCTGGTTTGTGGTTACAGACCCTTCAAAAAATCACACCCTTCCTGCTGTTGAGGTGCAATCA
GCCATAAGAATGAACAAGAACCGGATCAACAATGCCTTCTTTCTAAATGACCAAACTCTGGAA
TTTTTAAAAATCCCTTCCACACTTGCACCACCCATGGACCCATCTGTGCCCATCTGGATTATT
ATATTTGGTGTGATATTTTGCATCATCATAGTTGCAATTGCACTACTGATTTTATCAGGGATC
TGGCAACGTAGAAGAAAGAACAAAGAACCATCTGAAGTGGATGACGCTGAAGATAAGTGTGAA
AACATGATCACAATTGAAAATGGCATCCCCTCTGATCCCCTGGACATGAAGGGGGGCATATTA
ATGATGCCTTCA**TGA**CAGAGGATGAGAGGCTCACCCCTCTCTGAAGGGCTGTTGTTCTGCTTC
CTCAAGAAATTAAACATTTGTTTCTGTGTGACTGCTGAGCATCCTGAAATACCAAGAGCAGAT
CATATATTTTGTTTCACCATTCTTCTTTTGTAATAAATTTTGAATGTGCTTGAAAGTGAAAAG
CAATCAATTATACCCACCAACACCACTGAAATCATAAGCTATTCACGACTCAAAATATTCTAA
AATATTTTTCTGACAGTATAGTGTATAAATGTGGTCATGTGGTATTTGTAGTTATTGATTTAA
GCATTTTTAGAAATAAGATCAGGCATATGTATATATTTTCACACTTCAAAGACCTAAGGAAAA
ATAAATTTTCCAGTGGAGAATACATATAATATGGTGTAGAAATCATTGAAAATGGATCCTTTT
TGACGATCACTTATATCACTCTGTATATGACTAAGTAAACAAAAGTGAGAAGTAATTATTGTA
AATGGATGGATAAAAATGGAATTACTCATATACAGGGTGGAATTTTATCCTGTTATCACACCA
ACAGTTGATTATATATTTTCTGAATATCAGCCCCTAATAGGACAATTCTATTTGTTGACCATT
TCTACAATTTGTAAAAGTCCAATCTGTGCTAACTTAATAAAGTAATAATCATCTCTTTTTAAA
AAAAAAAAAAAAAAAAAAAAAA

# FIGURE 482

MLWLLFFLVTAIHAELCQPGAENAFKVRLSIRTALGDKAYAWDTNEEYLFKAMVAFSMRKVPN
REATEISHVLLCNVTQRVSFWFVVTDPSKNHTLPAVEVQSAIRMNKNRINNAFFLNDQTLEFL
KIPSTLAPPMDPSVPIWIIIFGVIFCIIIVAIALLILSGIWQRRRKNKEPSEVDDAEDKCENM
ITIENGIPSDPLDMKGGILMMPS

# FIGURE 483

CGTCTCTGCGTTCGCC**ATG**CGTCCCGGGGCGCCAGGGCCACTCTGGCCTCTGCCCTGGGGGC
CCTGGCTTGGGCCGTGGGCTTCGTGAGCTCCATGGGCTCGGGGAACCCCGCGCCCGGTGGTGT
TTGCTGGCTCCAGCAGGGCCAGGAGGCCACCTGCAGCCTGGTGCTCCAGACTGATGTCACCCG
GGCCGAGTGCTGTGCCTCCGGCAACATTGACACCGCCTGGTCCAACCTCACCCACCCGGGGAA
CAAGATCAACCTCCTCGGCTTCTTGGGCCTTGTCCACTGCCTTCCCTGCAAAGATTCGTGCGA
CGGCGTGGAGTGCGGCCCGGGCAAGGCGTGCCGCATGCTGGGGGGCCGCCCGCGCTGCGAGTG
CGCGCCCGACTGCTCGGGGCTCCCGGCGCGGCTGCAGGTCTGCGGCTCAGACGGCGCCACCTA
CCGCGACGAGTGCGAGCTGCGCGCCGCGCGCTGCCGCGGCCACCCGGACCTGAGCGTCATGTA
CCGGGGCCGCTGCCGCAAGTCCTGTGAGCACGTGGTGTGCCCGCGGCCACAGTCGTGCGTCGT
GGACCAGACGGGCAGCGCCCACTGCGTGGTGTGTCGAGCGGCGCCCTGCCCTGTGCCCTCCAG
CCCCGGCCAGGAGCTTTGCGGCAACAACAACGTCACCTACATCTCCTCGTGCCACATGCGCCA
GGCCACCTGCTTCCTGGGCCGCTCCATCGGCGTGCGCCACGCGGGCAGCTGCGCAGGCACCCC
TGAGGAGCCGCCAGGTGGTGAGTCTGCAGAAGAGGAAGAGAACTTCGTG**TGA**GCCTGCAGGAC
AGGCCTGGGCCTGGTGCCCGAGGCCCCCCATCATCCCCTGTTATTTATTGCCACAGCAGAGTC
TAATTTATATGCCACGGACACTCCTTAGAGCCCGGATTCGGACCACTTGGGGATCCCAGAACC
TCCCTGACGATATCCTGGAAGGACTGAGGAAGGGAGGCCTGGGGGCCGGCTGGTGGGTGGGAT
AGACCTGCGTTCCGGACACTGAGCGCCTGATTTAGGGCCCTTCTCTAGGATGCCCCAGCCCCT
ACCCTAAGACCTATTGCCGGGGAGGATTCCACACTTCCGCTCCTTTGGGGATAAACCTATTAA
TTATTGCTACTATCAAGAGGGCTGGGCATTCTCTGCTGGTAATTCCTGAAGAGGCATGACTGC
TTTTCTCAGCCCCAAGCCTCTAGTCTGGGTGTGTACGGAGGGTCTAGCCTGGGTGTGTACGGA
GGGTCTAGCCTGGGTGAGTACGGAGGGTCTAGCCTGGGTGAGTACGGAGGGTCTAGCCTGGGT
GAGTACGGAGGGTCTAGCCTGGGTGTGTATGGAGGATCTAGCCTGGGTGAGTATGGAGGGTCT
AGCCTGGGTGAGTATGGAGGGTCTAGCCTGGGTGTGTATGGAGGGTCTAGCCTGGGTGAGTAT
GGAGGGTCTAGCCTGGGTGTGTATGGAGGGTCTAGCCTGGGTGAGTATGGAGGGTCTAGCCTG
GGTGTGTACGGAGGGTCTAGTCTGAGTGCGTGTGGGGACCTCAGAACACTGTGACCTTAGCCC
AGCAAGCCAGGCCCTTCATGAAGGCCAAGAAGGCTGCCACCATTCCCTGCCAGCCCAAGAACT
CCAGCTTCCCCACTGCCTCTGTGTGCCCCTTTGCGTCCTGTGAAGGCCATTGAGAAATGCCCA
GTGTGCCCCCTGGGAAAGGGCACGGCCTGTGCTCCTGACACGGGCTGTGCTTGGCCACAGAAC
CACCCAGCGTCTCCCCTGCTGCTGTCCACGTCAGTTCATGAGGCAACGTCGCGTGGTCTCAGA
CGTGGAGCAGCCAGCGGCAGCTCAGAGCAGGGCACTGTGTCCGGCGGAGCCAAGTCCACTCTG
GGGGAGCTCTGGCGGGGACCACGGGCCACTGCTCACCCACTGGCCCCGAGGGGGGTGTAGACG
CCAAGACTCACGCATGTGTGACATCCGGAGTCCTGGAGCCGGGTGTCCCAGTGGCACCACTAG
GTGCCTGCTGCCTCCACAGTGGGGTTCACACCCAGGGCTCCTTGGTCCCCCACAACCTGCCCC
GGCCAGGCCTGCAGACCCAGACTCCAGCCAGACCTGCCTCACCCACCAATGCAGCCGGGGCTG
GCGACACCAGCCAGGTGCTGGTCTTGGGCCAGTTCTCCCACGACGGCTCACCCTCCCCTCCAT
CTGCGTTGATGCTCAGAATCGCCTACCTGTGCCTGCGTGTAAACCACAGCCTCAGACCAGCTA
TGGGGAGAGGACAACACGGAGGATATCCAGCTTCCCCGGTCTGGGGTGAGGAATGTGGGGAGC
TTGGGCATCCTCCTCCAGCCTCCTCCAGCCCCCAGGCAGTGCCTTACCTGTGGTGCCCAGAAA
AGTGCCCCTAGGTTGGTGGGTCTACAGGAGCCTCAGCCAGGCAGCCCACCCCACCCTGGGGCC
CTGCCTCACCAAGGAAATAAAGACTCAAGCCATAAAAAAAA

# FIGURE 484

MRPGAPGPLWPLPWGALAWAVGFVSSMGSGNPAPGGVCWLQQGQEATCSLVLQTDVTRAECCA
SGNIDTAWSNLTHPGNKINLLGFLGLVHCLPCKDSCDGVECGPGKACRMLGGRPRCECAPDCS
GLPARLQVCGSDGATYRDECELRAARCRGHPDLSVMYRGRCRKSCEHVVCPRPQSCVVDQTGS
AHCVVCRAAPCPVPSSPGQELCGNNNVTYISSCHMRQATCFLGRSIGVRHAGSCAGTPEEPPG
GESAEEEENFV

**Important features:**

**Signal peptide:**

amino acids 1-20

**N-glycosylation sites.**

amino acids 73-77, 215-219

**Osteonectin domain proteins.**

amino acids 97-130, 169-202

# FIGURE 485

GCTCGAGGCCGGCGGCGGCGGGAGAGCGACCCGGGCGGCCTCGTAGCGGGGCCCCGGATCCCC
GAGTGGCGGCCGGAGCCTCGAAAAGAGATTCTCAGCGCTGATTTTGAG**ATG**ATGGGCTTGGGA
AACGGGCGTCGCAGCATGAAGTCGCCGCCCCTCGTGCTGGCCGCCCTGGTGGCCTGCATCATC
GTCTTGGGCTTCAACTACTGGATTGCGAGCTCCCGGAGCGTGGACCTCCAGACACGGATCATG
GAGCTGGAAGGCAGGGTCCGCAGGGCGGCTGCAGAGAGAGGCGCCGTGGAGCTGAAGAAGAAC
GAGTTCCAGGGAGAGCTGGAGAAGCAGCGGGAGCAGCTTGACAAAATCCAGTCCAGCCACAAC
TTCCAGCTGGAGAGCGTCAACAAGCTGTACCAGGACGAAAAGGCGGTTTTGGTGAATAACATC
ACCACAGGTGAGAGGCTCATCCGAGTGCTGCAAGACCAGTTAAAGACCCTGCAGAGGAATTAC
GGCAGGCTGCAGCAGGATGTCCTCCAGTTTCAGAAGAACCAGACCAACCTGGAGAGGAAGTTC
TCCTACGACCTGAGCCAGTGCATCAATCAGATGAAGGAGGTGAAGGAACAGTGTGAGGAGCGA
ATAGAAGAGGTCACCAAAAAGGGGAATGAAGCTGTAGCTTCCAGAGACCTGAGTGAAAACAAC
GACCAGAGACAGCAGCTCCAAGCCCTCAGTGAGCCTCAGCCCAGGCTGCAGGCAGCAGGCCTG
CCACACACAGAGGTGCCACAAGGGAAGGGAAACGTGCTTGGTAACAGCAAGTCCCAGACACCA
GCCCCCAGTTCCGAAGTGGTTTTGGATTCAAAGAGACAAGTTGAGAAAGAGGAAACCAATGAG
ATCCAGGTGGTGAATGAGGAGCCTCAGAGGGACAGGCTGCCGCAGGAGCCAGGCCGGGAGCAG
GTGGTGGAAGACAGACCTGTAGGTGGAAGAGGCTTCGGGGGAGCCGGAGAACTGGGCCAGACC
CCACAGGTGCAGGCTGCCCTGTCAGTGAGCCAGGAAAATCCAGAGATGGAGGGCCCTGAGCGA
GACCAGCTTGTCATCCCCGACGGACAGGAGGAGGAGCAGGAAGCTGCCGGGGAAGGGAGAAAC
CAGCAGAAACTGAGAGGAGAAGATGACTACAACATGGATGAAAATGAAGCAGAATCTGAGACA
GACAAGCAAGCAGCCCTGGCAGGGAATGACAGAAACATAGATGTTTTTAATGTTGAAGATCAG
AAAAGAGACACCATAAATTTACTTGATCAGCGTGAAAAGCGGAATCATACACTC**TGA**ATTGAA
CTGGAATCACATATTTCACAACAGGGCCGAAGAGATGACTATAAAATGTTCATGAGGGACTGA
ATACTGAAAACTGTGAAATGTACTAAATAAAATGTACATCTGA

# FIGURE 486

MMGLGNGRRSMKSPPLVLAALVACIIVLGFNYWIASSRSVDLQTRIMELEGRVRRAAAERGAV
ELKKNEFQGELEKQREQLDKIQSSHNFQLESVNKLYQDEKAVLVNNITTGERLIRVLQDQLKT
LQRNYGRLQQDVLQFQKNQTNLERKFSYDLSQCINQMKEVKEQCEERIEEVTKKGNEAVASRD
LSENNDQRQQLQALSEPQPRLQAAGLPHTEVPQGKGNVLGNSKSQTPAPSSEVVLDSKRQVEK
EETNEIQVVNEEPQRDRLPQEPGREQVVEDRPVGGRGFGGAGELGQTPQVQAALSVSQENPEM
EGPERDQLVIPDGQEEEQEAAGEGRNQQKLRGEDDYNMDENEAESETDKQAALAGNDRNIDVF
NVEDQKRDTINLLDQREKRNHTL

**Important features:**

**Signal peptide:**

amino acids 1-29

# FIGURE 487

AACTCAAACTCCTCTCTCTGGGAAAACGCGGTGCTTGCTCCTCCCGGAGTGGCCTTGGCAGGG
TGTTGGAGCCCTCGGTCTGCCCCGTCCGGTCTCTGGGGCCAAGGCTGGGTTTCCCTC**ATG**TAT
GGCAAGAGCTCTACTCGTGCGGTGCTTCTTCTCCTTGGCATACAGCTCACAGCTCTTTGGCCT
ATAGCAGCTGTGGAAATTTATACCTCCCGGGTGCTGGAGGCTGTTAATGGGACAGATGCTCGG
TTAAAATGCACTTTCTCCAGCTTTGCCCCTGTGGGTGATGCTCTAACAGTGACCTGGAATTTT
CGTCCTCTAGACGGGGGACCTGAGCAGTTTGTATTCTACTACCACATAGATCCCTTCCAACCC
ATGAGTGGGCGGTTTAAGGACCGGGTGTCTTGGGATGGGAATCCTGAGCGGTACGATGCCTCC
ATCCTTCTCTGGAAACTGCAGTTCGACGACAATGGGACATACACCTGCCAGGTGAAGAACCCA
CCTGATGTTGATGGGGTGATAGGGGAGATCCGGCTCAGCGTCGTGCACACTGTACGCTTCTCT
GAGATCCACTTCCTGGCTCTGGCCATTGGCTCTGCCTGTGCACTGATGATCATAATAGTAATT
GTAGTGGTCCTCTTCCAGCATTACCGGAAAAAGCGATGGGCCGAAAGAGCTCATAAAGTGGTG
GAGATAAAATCAAAAGAAGAGGAAAGGCTCAACCAAGAGAAAAAGGTCTCTGTTTATTTAGAA
GACACAGAC**TAA**CAATTTTAGATGGAAGCTGAGATGATTTCCAAGAACAAGAACCCTAGTATT
TCTTGAAGTTAATGGAAACTTTTCTTTGGCTTTTCCAGTTGTGACCCGTTTTCCAACCAGTTC
TGCAGCATATTAGATTCTAGACAAGCAACACCCCTCTGGAGCCAGCACAGTGCTCCTCCATAT
CACCAGTCATACACAGCCTCATTATTAAGGTCTTATTTAATTTCAGAGTGTAAATTTTTTCAA
GTGCTCATTAGGTTTTATAAACAAGAAGCTACATTTTTGCCCTTAAGACACTACTTACAGTGT
TATGACTTGTATACACATATATTGGTATCAAAGGGGATAAAAGCCAATTTGTCTGTTACATTT
CCTTTCACGTATTTCTTTTAGCAGCACTTCTGCTACTAAAGTTAATGTGTTTACTCTCTTTCC
TTCCCACATTCTCAATTAAAAGGTGAGCTAAGCCTCCTCGGTGTTTCTGATTAACAGTAAATC
CTAAATTCAAACTGTTAAATGACATTTTTATTTTTATGTCTCTCCTTAACTATGAGACACATC
TTGTTTTACTGAATTTCTTTCAATATTCCAGGTGATAGATTTTTGTCG

# FIGURE 488

MYGKSSTRAVLLLLGIQLTALWPIAAVEIYTSRVLEAVNGTDARLKCTFSSFAPVGDALTVTW
NFRPLDGGPEQFVFYYHIDPFQPMSGRFKDRVSWDGNPERYDASILLWKLQFDDNGTYTCQVK
NPPDVDGVIGEIRLSVVHTVRFSEIHFLALAIGSACALMIIIVIVVVLFQHYRKKRWAERAHK
VVEIKSKEEERLNQEKKVSVYLEDTD

# FIGURE 489

AAGCAACCAAACTGCAAGCTTTGGGAGTTGTTCGCTGTCCCTGCCCTGCTCTGCTAGGGAGAG
AACGCCAGAGGGAGGCGGCTGGCCCGGCGGCAGGCTCTCAGAACCGCTACCGGCG**ATG**CTACT
GCTGTGGGTGTCGGTGGTCGCAGCCTTGGCGCTGGCGGTACTGGCCCCCGGAGCAGGGGAGCA
GAGGCGGAGAGCAGCCAAAGCGCCCAATGTGGTGCTGGTCGTGAGCGACTCCTTCGATGGAAG
GTTAACATTTCATCCAGGAAGTCAGGTAGTGAAACTTCCTTTTATCAACTTTATGAAGACACG
TGGGACTTCCTTTCTGAATGCCTACACAAACTCTCCAATTTGTTGCCCATCACGCGCAGCAAT
GTGGAGTGGCCTCTTCACTCACTTAACAGAATCTTGGAATAATTTTAAGGGTCTAGATCCAAA
TTATACAACATGGATGGATGTCATGGAGAGGCATGGCTACCGAACACAGAAATTTGGGAAACT
GGACTATACTTCAGGACATCACTCCATTAGTAATCGTGTGGAAGCGTGGACAAGAGATGTTGC
TTTCTTACTCAGACAAGAAGGCAGGCCCATGGTTAATCTTATCCGTAACAGGACTAAAGTCAG
AGTGATGGAAAGGGATTGGCAGAATACAGACAAAGCAGTAAACTGGTTAAGAAAGGAAGCAAT
TAATTACACTGAACCATTTGTTATTTACTTGGGATTAAATTTACCACACCCTTACCCTTCACC
ATCTTCTGGAGAAAATTTTGGATCTTCAACATTTCACACATCTCTTTATTGGCTTGAAAAAGT
GTCTCATGATGCCATCAAAATCCCAAAGTGGTCACCTTTGTCAGAAATGCACCCTGTAGATTA
TTACTCTTCTTATACAAAAAAACTGCACTGGAAGATTTACAAAAAAAGAAATTAAGAATATTAG
AGCATTTTATTATGCTATGTGTGCTGAGACAGATGCCATGCTTGGTGAAATTATTTTGGCCCT
TCATCAATTAGATCTTCTTCAGAAAACTATTGTCATATACTCCTCAGACCATGGAGAGCTGGC
CATGGAACATCGACAGTTTTATAAAATGAGCATGTACGAGGCTAGTGCACATGTTCCGCTTTT
GATGATGGGACCAGGAATTAAAGCCGGCCTACAAGTATCAAATGTGGTTTCTCTTGTGGATAT
TTACCCTACCATGCTTGATATTGCTGGAATTCCTCTGCCTCAGAACCTGAGTGGATACTCTTT
GTTGCCGTTATCATCAGAAACATTTAAGAATGAACATAAAGTCAAAAACCTGCATCCACCCTG
GATTCTGAGTGAATTCCATGGATGTAATGTGAATGCCTCCACCTACATGCTTCGAACTAACCA
CTGGAAATATATAGCCTATTCGGATGGTGCATCAATATTGCCTCAACTCTTTGATCTTTCCTC
GGATCCAGATGAATTAACAAATGTTGCTGTAAAATTTCCAGAAATTACTTATTCTTTGGATCA
GAAGCTTCATTCCATTATAAACTACCCTAAAGTTTCTGCTTCTGTCCACCAGTATAATAAAGA
GCAGTTTATCAAGTGGAAACAAAGTATAGGACAGAATTATTCAAACGTTATAGCAAATCTTAG
GTGGCACCAAGACTGGCAGAAGGAACCAAGGAAGTATGAAAATGCAATTGATCAGTGGCTTAA
AACCCATATGAATCCAAGAGCAGTT**TGA**ACAAAAAGTTTAAAAATAGTGTTCTAGAGATACAT
ATAAATATATTACAAGATCATAATTATGTATTTTAAATGAAACAGTTTTAATAATTACCAAGT
TTTGGCCGGGCACAGTGGCTCACACCTGTAATCCCAGGACTTTGGGAGGCTGAGGAAAGCAGA
TCACAAGGTCAAGAGATTGAGACCATCCTGGCCAACATGGTGAAACCCTGTCTCTACTAAAAA
TACAAAAATTAGCTGGGCGCGGTGGTGCACACCTATAGTCTCAGCTACTCAGAGGCTGAGGCA
GGAGGATCGCTTGAACCCGGGAGGCAGCAGTTGCAGTGAGCTGAGATTGCGCCACTGTACTCC
AGCCTGGCAACAGAGTGAGACTGTGTCGCAAAAAAATAAAAATAAAATAATAATAATTACCAA
TTTTTCATTATTTTGTAAGAATGTAGTGTATTTTAAGATAAAATGCCAATGATTATAAAATCA
CATATTTTCAAAAATGGTTATTATTTAGGCCTTTGTACAATTTCTAACAATTTAGTGGAAGTA
TCAAAAGGATTGAAGCAAATACTGTAACAGTTATGTTCCTTTAAATAATAGAGAATATAAAAT
ATTGTAATAATATGTATCATAAAATAGTTGTATGTGAGCATTTGATGGTGAAAAAAAAAAAAA
AAAAAAAAAAAAAAAAAAAAAAAAAAAAAAAAAAAAAAAAAAAAAAAAAAAAAAAAAAAAAAA
AAAAAAAAAAAAAAAAAAA

# FIGURE 490

MLLLWVSVVAALALAVLAPGAGEQRRRAAKAPNVVLVVSDSFDGRLTFHPGSQVVKLPFINFM
KTRGTSFLNAYTNSPICCPSRAAMWSGLFTHLTESWNNFKGLDPNYTTWMDVMERHGYRTQKF
GKLDYTSGHHSISNRVEAWTRDVAFLLRQEGRPMVNLIRNRTKVRVMERDWQNTDKAVNWLRK
EAINYTEPFVIYLGLNLPHPYPSPSSGENFGSSTFHTSLYWLEKVSHDAIKIPKWSPLSEMHP
VDYYSSYTKNCTGRFTKKEIKNIRAFYYAMCAETDAMLGEIILALHQLDLLQKTIVIYSSDHG
ELAMEHRQFYKMSMYEASAHVPLLMMGPGIKAGLQVSNVVSLVDIYPTMLDIAGIPLPQNLSG
YSLLPLSSETFKNEHKVKNLHPPWILSEFHGCNVNASTYMLRTNHWKYIAYSDGASILPQLFD
LSSDPDELTNVAVKFPEITYSLDQKLHSIINYPKVSASVHQYNKEQFIKWKQSIGQNYSNVIA
NLRWHQDWQKEPRKYENAIDQWLKTHMNPRAV

**Important features:**

**Signal peptide:**

amino acids 1-15

**N-glycosylation sites.**

amino acids 108-111, 166-169, 193-196, 262-265, 375-378, 413-416, 498-501

**Sulfatases proteins:**

amino acids 286-315, 359-369, 78-97

# FIGURE 491

GAGAGAAGTCAGCCTGGCAGAGAGACTCTGAAATGAGGGATTAGAGGTGTTCAAGGAGCAAGA
GCTTCAGCCTGAAGACAAGGGAGCAGTCCCTGAAGACGCTTCTACTGAGAGGTCTGCC**ATG**GC
CTCTCTTGGCCTCCAACTTGTGGGCTACATCCTAGGCCTTCTGGGGCTTTTGGGCACACTGGT
TGCCATGCTGCTCCCCAGCTGGAAAACAAGTTCTTATGTCGGTGCCAGCATTGTGACAGCAGT
TGGCTTCTCCAAGGGCCTCTGGATGGAATGTGCCACACACAGCACAGGCATCACCCAGTGTGA
CATCTATAGCACCCTTCTGGGCCTGCCCGCTGACATCCAGGCTGCCCAGGCCATGATGGTGAC
ATCCAGTGCAATCTCCTCCCTGGCCTGCATTATCTCTGTGGTGGGCATGAGATGCACAGTCTT
CTGCCAGGAATCCCGAGCCAAAGACAGAGTGGCGGTAGCAGGTGGAGTCTTTTTCATCCTTGG
AGGCCTCCTGGGATTCATTCCTGTTGCCTGGAATCTTCATGGGATCCTACGGGACTTCTACTC
ACCACTGGTGCCTGACAGCATGAAATTTGAGATTGGAGAGGCTCTTTACTTGGGCATTATTTC
TTCCCTGTTCTCCCTGATAGCTGGAATCATCCTCTGCTTTTCCTGCTCATCCCAGAGAAATCG
CTCCAACTACTACGATGCCTACCAAGCCCAACCTCTTGCCACAAGGAGCTCTCCAAGGCCTGG
TCAACCTCCCAAAGTCAAGAGTGAGTTCAATTCCTACAGCCTGACAGGGTATGTG**TGA**AGAAC
CAGGGGCCAGAGCTGGGGGGTGGCTGGGTCTGTGAAAAACAGTGGACAGCACCCCGAGGGCCA
CAGGTGAGGGACACTACCACTGGATCGTGTCAGAAGGTGCTGCTGAGGATAGACTGACTTTGG
CCATTGGATTGAGCAAAGGCAGAAATGGGGGCTAGTGTAACAGCATGCAGGTTGAATTGCCAA
GGATGCTCGCCATGCCAGCCTTTCTGTTTTCCTCACCTTGCTGCTCCCCTGCCCTAAGTCCCC
AACCCTCAACTTGAAACCCCATTCCCTTAAGCCAGGACTCAGAGGATCCCTTTGCCCTCTGGT
TTACCTGGGACTCCATCCCCAAACCCACTAATCACATCCCACTGACTGACCCTCTGTGATCAA
AGACCCTCTCTCTGGCTGAGGTTGGCTCTTAGCTCATTGCTGGGGATGGGAAGGAGAAGCAGT
GGCTTTTGTGGGCATTGCTCTAACCTACTTCTCAAGCTTCCCTCCAAAGAAACTGATTGGCCC
TGGAACCTCCATCCCACTCTTGTTATGACTCCACAGTGTCCAGACTAATTTGTGCATGAACTG
AAATAAAACCATCCTACGGTATCCAGGGAACAGAAAGCAGGATGCAGGATGGGAGGACAGGAA
GGCAGCCTGGGACATTTAAAAAAATA

# FIGURE 492

MASLGLQLVGYILGLLGLLGTLVAMLLPSWKTSSYVGASIVTAVGFSKGLWMECATHSTGITQ
CDIYSTLLGLPADIQAAQAMMVTSSAISSLACIISVVGMRCTVFCQESRAKDRVAVAGGVFFI
LGGLLGFIPVAWNLHGILRDFYSPLVPDSMKFEIGEALYLGIISSLFSLIAGIILCFSCSSQR
NRSNYYDAYQAQPLATRSSPRPGQPPKVKSEFNSYSLTGYV

**Important features:**

**Signal peptide:**

amino acids 1-24

**Transmembrane domains:**

amino acids 82-102, 117-140, 163-182

**N-glycosylation site.**

amino acids 190-193

**PMP-22 / EMP / MP20 family proteins.**

amino acids 46-59

# FIGURE 493

GCACTGCTGCTGTCCCATCAGCTGCTCTGAAGCTCC**ATG**GTGCCCAGAATCTTCGCTCCTGCT
TATGTGTCAGTCTGTCTCCTCCTCTTGTGTCCAAGGGAAGTCATCGCTCCCGCTGGCTCAGAA
CCATGGCTGTGCCAGCCGGCACCCAGGTGTGGAGACAAGATCTACAACCCCTTGGAGCAGTGC
TGTTACAATGACGCCATCGTGTCCCTGAGCGAGACCCGCCAATGTGGTCCCCCCTGCACCTTC
TGGCCCTGCTTTGAGCTCTGCTGTCTTGATTCCTTTGGCCTCACAAACGATTTTGTTGTGAAG
CTGAAGGTTCAGGGTGTGAATTCCCAGTGCCACTCATCTCCCATCTCCAGTAAATGTGAAAGC
AGAAGACGTTTTCCC**TGA**GAAGACATAGAAAGAAAATCAACTTTCACTAAGGCATCTCAGAAA
CATAGGCTAAGGTAATATGTGTACCAGTAGAGAAGCCTGAGGAATTTACAAAATGATGCAGCT
CCAAGCCATTGTATGGCCCATGTGGGAGACTGATGGGACATGGAGAATGACAGTAGATTATCA
GGAAATAAATAAAGTGGTTTTTCCAATGTACACACCTGTAAAA

# FIGURE 494

MVPRIFAPAYVSVCLLLLCPREVIAPAGSEPWLCQPAPRCGDKIYNPLEQCCYNDAIVSLSET
RQCGPPCTFWPCFELCCLDSFGLTNDFVVKLKVQGVNSQCHSSPISSKCESRRRFP

**Important features:**

**Signal peptide:**

amino acids 1-25

# FIGURE 495

CTCCACTGCAACCACCCAGAGCC**ATG**GCTCCCCGAGGCTGCATCGTAGCTGTCTTTGCCATTT
TCTGCATCTCCAGGCTCCTCTGCTCACACGGAGCCCCAGTGGCCCCCATGACTCCTTACCTGA
TGCTGTGCCAGCCACACAAGAGATGTGGGGACAAGTTCTACGACCCCCTGCAGCACTGTTGCT
ATGATGATGCCGTCGTGCCCTTGGCCAGGACCCAGACGTGTGGAAACTGCACCTTCAGAGTCT
GCTTTGAGCAGTGCTGCCCCTGGACCTTCATGGTGAAGCTGATAAACCAGAACTGCGACTCAG
CCCGGACCTCGGATGACAGGCTTTGTCGCAGTGTCAGC**TAA**TGGAACATCAGGGGAACGATGA
CTCCTGGATTCTCCTTCCTGGGTGGGCCTGGAGAAAGAGGCTGGTGTTACCTGAGATCTGGGA
TGCTGAGTGGCTGTTTGGGGGCCAGAGAAACACACACTCAACTGCCCACTTCATTCTGTGACC
TGTCTGAGGCCCACCCTGCAGCTGCCCTGAGGAGGCCCACAGGTCCCCTTCTAGAATTCTGGA
CAGCATGAGATGCGTGTGCTGATGGGGGCCCAGGGACTCTGAACCCTCCTGATGACCCCTATG
GCCAACATCAACCCGGCACCACCCCAAGGCTGGCTGGGGAACCCTTCACCCTTCTGTGAGATT
TTCCATCATCTCAAGTTCTCTTCTATCCAGGAGCAAAGCACAGGATCATAATAAATTTATGTA
CTTTATAAATGAAAA

# FIGURE 496

MAPRGCIVAVFAIFCISRLLCSHGAPVAPMTPYLMLCQPHKRCGDKFYDPLQHCCYDDAVVPL
ARTQTCGNCTFRVCFEQCCPWTFMVKLINQNCDSARTSDDRLCRSVS

**Important features:**

**Signal peptide:**

amino acids 1-24

# FIGURE 497

TGAAGGACTTTTCCAGGACCCAAGGCCACACACTGGAAGTCTTGCAGCTGAAGGGAGGCACTC
CTTGGCCTCCGCAGCCGATCAC**ATG**AAGGTGGTGCCAAGTCTCCTGCTCTCCGTCCTCCTGGC
ACAGGTGTGGCTGGTACCCGGCTTGGCCCCCAGTCCTCAGTCGCCAGAGACCCCAGCCCCTCA
GAACCAGACCAGCAGGGTAGTGCAGGCTCCCAGGGAGGAAGAGGAAGATGAGCAGGAGGCCAG
CGAGGAGAAGGCCGGTGAGGAAGAGAAAGCCTGGCTGATGGCCAGCAGGCAGCAGCTTGCCAA
GGAGACTTCAAACTTCGGATTCAGCCTGCTGCGAAAGATCTCCATGAGGCACGATGGCAACAT
GGTCTTCTCTCCATTTGGCATGTCCTTGGCCATGACAGGCTTGATGCTGGGGGCCACAGGGCC
GACTGAAACCCAGATCAAGAGAGGGCTCCACTTGCAGGCCCTGAAGCCCACCAAGCCCGGGCT
CCTGCCTTCCCTCTTTAAGGGACTCAGAGAGACCCTCTCCCGCAACCTGGAACTGGGCCTCTC
ACAGGGGAGTTTTGCCTTCATCCACAAGGATTTTGATGTCAAAGAGACTTTCTTCAATTTATC
CAAGAGGTATTTTGATACAGAGTGCGTGCCTATGAATTTTCGCAATGCCTCACAGGCCAAAAG
GCTCATGAATCATTACATTAACAAAGAGACTCGGGGGAAAATTCCCAAACTGTTTGATGAGAT
TAATCCTGAAACCAAATTAATTCTTGTGGATTACATCTTGTTCAAAGGGAAATGGTTGACCCC
ATTTGACCCTGTCTTCACCGAAGTCGACACTTTCCACCTGGACAAGTACAAGACCATTAAGGT
GCCCATGATGTACGGTGCAGGCAAGTTTGCCTCCACCTTTGACAAGAATTTTCGTTGTCATGT
CCTCAAACTGCCCTACCAAGGAAATGCCACCATGCTGGTGGTCCTCATGGAGAAAATGGGTGA
CCACCTCGCCCTTGAAGACTACCTGACCACAGACTTGGTGGAGACATGGCTCAGAAACATGAA
AACCAGAAACATGGAAGTTTTCTTTCCGAAGTTCAAGCTAGATCAGAAGTATGAGATGCATGA
GCTGCTTAGGCAGATGGGAATCAGAAGAATCTTCTCACCCTTTGCTGACCTTAGTGAACTCTC
AGCTACTGGAAGAAATCTCCAAGTATCCAGGGTTTTACGAAGAACAGTGATTGAAGTTGATGA
AAGGGGCACTGAGGCAGTGGCAGGAATCTTGTCAGAAATTACTGCTTATTCCATGCCTCCTGT
CATCAAAGTGGACCGGCCATTTCATTTCATGATCTATGAAGAAACCTCTGGAATGCTTCTGTT
TCTGGGCAGGGTGGTGAATCCGACTCTCCTA**TAA**TTCAGGACATGCATAAGCACTTCGTGCTG
TAGTAGATGCTGAATCTGAGGTATCAAACACACACAGGATACCAGCAATGGATGGCAGGGGAG
AGTGTTCCTTTTGTTCTTAACTAGTTTAGGGTGTTCTCAAATAAATACAGTAGTCCCCACTTA
TCTGAGGGGGATACATTCAAAGACCCCCAGCAGATGCCTGAAACGGTGGACAGTGCTGAACCT
TATATATATTTTTTCCTACACATACATACCTATGATAAAGTTTAATTTATAAATTAGGCACAG
TAAGAGATTAACAATAATAACAACATTAAGTAAAATGAGTTACTTGAACGCAAGCACTGCAAT
ACCATAACAGTCAAACTGATTATAGAGAAGGCTACTAAGTGACTCATGGGCGAGGAGCATAGA
CAGTGTGGAGACATTGGGCAAGGGGAGAATTCACATCCTGGGTGGGACAGAGCAGGACGATGC
AAGATTCCATCCCACTACTCAGAATGGCATGCTGCTTAAGACTTTTAGATTGTTTATTTCTGG
AATTTTTCATTTAATGTTTTTGGACCATGGTTGACCATGGTTAACTGAGACTGCAGAAAGCAA
AACCATGGATAAGGGAGGACTACTACAAAAGCATTAAATTGATACATATTTTTTAAAAAAAAA
AAAAAAAAAA

# FIGURE 498

MKVVPSLLLSVLLAQVWLVPGLAPSPQSPETPAPQNQTSRVVQAPREEEEDEQEASEEKAGEE
EKAWLMASRQQLAKETSNFGFSLLRKISMRHDGNMVFSPFGMSLAMTGLMLGATGPTETQIKR
GLHLQALKPTKPGLLPSLFKGLRETLSRNLELGLSQGSFAFIHKDFDVKETFFNLSKRYFDTE
CVPMNFRNASQAKRLMNHYINKETRGKIPKLFDEINPETKLILVDYILFKGKWLTPFDPVFTE
VDTFHLDKYKTIKVPMMYGAGKFASTFDKNFRCHVLKLPYQGNATMLVVLMEKMGDHLALEDY
LTTDLVETWLRNMKTRNMEVFFPKFKLDQKYEMHELLRQMGIRRIFSPFADLSELSATGRNLQ
VSRVLRRTVIEVDERGTEAVAGILSEITAYSMPPVIKVDRPFHFMIYEETSGMLLFLGRVVNP
TLL

# FIGURE 499

CTAGCCTGCGCCAAGGGGTAGTGAGACCGCGCGGCAACAGCTTGCGGCTGCGGGGAGCTCCCG
TGGGCGCTCCGCTGGCTGTGCAGGCGGCC**ATG**GATTCCTTGCGGAAAATGCTGATCTCAGTCG
CAATGCTGGGCGCAGGGGCTGGCGTGGGCTACGCGCTCCTCGTTATCGTGACCCCGGGAGAGC
GGCGGAAGCAGGAAATGCTAAAGGAGATGCCACTGCAGGACCCAAGGAGCAGGGAGGAGGCGG
CCAGGACCCAGCAGCTATTGCTGGCCACTCTGCAGGAGGCAGCGACCACGCAGGAGAACGTGG
CCTGGAGGAAGAACTGGATGGTTGGCGGCGAAGGCGGCGCCAGCGGGAGGTCACCG**TGA**GACC
GGACTTGCCTCCGTGGGCGCCGGACCTTGGCTTGGGCGCAGGAATCCGAGGCAGCCTTTCTCC
TTCGTGGGCCCAGCGGAGAGTCCGGACCGAGATACCATGCCAGGACTCTCCGGGGTCCTGTGA
GCTGCCGTCGGGTGAGCACGTTTCCCCCAAACCCTGGACTGACTGCTTTAAGGTCCGCAAGGC
GGGCCAGGGCCGAGACGCGAGTCGGATGTGGTGAACTGAAAGAACCAATAAAATCATGTTCCT
CCAAAAAAAAAAAAAAAAAAAAAAAAAAAAAAAAAAAAAAAAAAAAAAAAAAAAAAAAAAAAA

# FIGURE 500

MDSLRKMLISVAMLGAGAGVGYALLVIVTPGERRKQEMLKEMPLQDPRSREEAARTQQLLLAT
LQEAATTQENVAWRKNWMVGGEGGASGRSP

**Important features:**

**Signal peptide:**

amino acids 1-18

# FIGURE 501

CAGGAGAGAAGGCACCGCCCCCACCCCGCCTCCAAAGCTAACCCTCGGGCTTGAGGGGAAGAG
GCTGACTGTACGTTCCTTCTACTCTGGCACCACTCTCCAGGCTGCC**ATG**GGGCCCAGCACCCC
TCTCCTCATCTTGTTCCTTTTGTCATGGTCGGGACCCCTCCAAGGACAGCAGCACCACCTTGT
GGAGTACATGGAACGCCGACTAGCTGCTTTAGAGGAACGGCTGGCCCAGTGCCAGGACCAGAG
TAGTCGGCATGCTGCTGAGCTGCGGGACTTCAAGAACAAGATGCTGCCACTGCTGGAGGTGGC
AGAGAAGGAGCGGGAGGCACTCAGAACTGAGGCCGACACCATCTCCGGGAGAGTGGATCGTCT
GGAGCGGGAGGTAGACTATCTGGAGACCCAGAACCCAGCTCTGCCCTGTGTAGAGTTTGATGA
GAAGGTGACTGGAGGCCCTGGGACCAAAGGCAAGGGAAGAAGGAATGAGAAGTACGATATGGT
GACAGACTGTGGCTACACAATCTCTCAAGTGAGATCAATGAAGATTCTGAAGCGATTTGGTGG
CCCAGCTGGTCTATGGACCAAGGATCCACTGGGGCAAACAGAGAAGATCTACGTGTTAGATGG
GACACAGAATGACACAGCCTTTGTCTTCCCAAGGCTGCGTGACTTCACCCTTGCCATGGCTGC
CCGGAAAGCTTCCCGAGTCCGGGTGCCCTTCCCCTGGGTAGGCACAGGGCAGCTGGTATATGG
TGGCTTTCTTTATTTTGCTCGGAGGCCTCCTGGAAGACCTGGTGGAGGTGGTGAGATGGAGAA
CACTTTGCAGCTAATCAAATTCCACCTGGCAAACCGAACAGTGGTGGACAGCTCAGTATTCCC
AGCAGAGGGGCTGATCCCCCCCTACGGCTTGACAGCAGACACCTACATCGACCTGGTAGCTGA
TGAGGAAGGTCTTTGGGCTGTCTATGCCACCCGGGAGGATGACAGGCACTTGTGTCTGGCCAA
GTTAGATCCACAGACACTGGACACAGAGCAGCAGTGGGACACACCATGTCCCAGAGAGAATGC
TGAGGCTGCCTTTGTCATCTGTGGGACCCTCTATGTCGTCTATAACACCCGTCCTGCCAGTCG
GGCCCGCATCCAGTGCTCCTTTGATGCCAGCGGCACCCTGACCCCTGAACGGGCAGCACTCCC
TTATTTTCCCCGCAGATATGGTGCCCATGCCAGCCTCCGCTATAACCCCCGAGAACGCCAGCT
CTATGCCTGGGATGATGGCTACCAGATTGTCTATAAGCTGGAGATGAGGAAGAAAGAGGAGGA
GGTT**TGA**GGAGCTAGCCTTGTTTTTTGCATCTTTCTCACTCCCATACATTTATATTATATCCC
CACTAAATTTCTTGTTCCTCATTCTTCAAATGTGGGCCAGTTGTGGCTCAAATCCTCTATATT
TTTAGCCAATGGCAATCAAATTCTTTCAGCTCCTTTGTTTCATACGGAACTCCAGATCCTGAG
TAATCCTTTTAGAGCCCGAAGAGTCAAAACCCTCAATGTTCCCTCCTGCTCTCCTGCCCCATG
TCAACAAATTTCAGGCTAAGGATGCCCCAGACCCAGGGCTCTAACCTTGTATGCGGGCAGGCC
CAGGGAGCAGGCAGCAGTGTTCTTCCCCTCAGAGTGACTTGGGGAGGGAGAAATAGGAGGAGA
CGTCCAGCTCTGTCCTCTCTTCCTCACTCCTCCCTTCAGTGTCCTGAGGAACAGGACTTTCTC
CACATTGTTTTGTATTGCAACATTTTGCATTAAAAGGAAAATCCACAAAAAAAAAAAAAAAAA
AAAAAAAAAAAAAAAAAAAAAAAAAAAAAAAAAAAAAAAAAAAAAAAAAAAAAA

# FIGURE 502

MGPSTPLLILFLLSWSGPLQGQQHHLVEYMERRLAALEERLAQCQDQSSRHAAELRDFKNKML
PLLEVAEKEREALRTEADTISGRVDRLEREVDYLETQNPALPCVEFDEKVTGGPGTKGKGRRN
EKYDMVTDCGYTISQVRSMKILKRFGGPAGLWTKDPLGQTEKIYVLDGTQNDTAFVFPRLRDF
TLAMAARKASRVRVPFPWVGTGQLVYGGFLYFARRPPGRPGGGGEMENTLQLIKFHLANRTVV
DSSVFPAEGLIPPYGLTADTYIDLVADEEGLWAVYATREDDRHLCLAKLDPQTLDTEQQWDTP
CPRENAEAAFVICGTLYVVYNTRPASRARIQCSFDASGTLTPERAALPYFPRRYGAHASLRYN
PRERQLYAWDDGYQIVYKLEMRKKEEEV

**Important features:**

**Signal peptide:**

amino acids 1-21

**N-glycosylation sites.**

amino acids 177-180, 248-251

# FIGURE 503

TGCGGCGCAGTGTAGACCTGGGAGG**ATG**GGCGGCCTGCTGCTGGCTGCTTTTCTGGCTTTGGT
CTCGGTGCCCAGGGCCCAGGCCGTGTGGTTGGGAAGACTGGACCCTGAGCAGCTTCTTGGGCC
CTGGTACGTGCTTGCGGTGGCCTCCCGGGAAAAGGGCTTTGCCATGGAGAAGGACATGAAGAA
CGTCGTGGGGGTGGTGGTGACCCTCACTCCAGAAAACAACCTGCGGACGCTGTCCTCTCAGCA
CGGGCTGGGAGGGTGTGACCAGAGTGTCATGGACCTGATAAAGCGAAACTCCGGATGGGTGTT
TGAGAATCCCTCAATAGGCGTGCTGGAGCTCTGGGTGCTGGCCACCAACTTCAGAGACTATGC
CATCATCTTCACTCAGCTGGAGTTCGGGGACGAGCCCTTCAACACCGTGGAGCTGTACAGTCT
GACGGAGACAGCCAGCCAGGAGGCCATGGGGCTCTTCACCAAGTGGAGCAGGAGCCTGGGCTT
CCTGTCACAG**TAG**CAGGCCCAGCTGCAGAAGGACCTCACCTGTGCTCACAAGATCCTTCTGTG
AGTGCTGCGTCCCCAGTAGGGATGGCGCCCACAGGGTCCTGTGACCTCGGCCAGTGTCCACCC
ACCTCGCTCAGCGGCTCCCGGGGCCCAGCACCAGCTCAGAATAAAGCGATTCCACAGCA

# FIGURE 504

MGGLLLAAFLALVSVPRAQAVWLGRLDPEQLLGPWYVLAVASREKGFAMEKDMKNVVGVVVTL
TPENNLRTLSSQHGLGGCDQSVMDLIKRNSGWVFENPSIGVLELWVLATNFRDYAIIFTQLEF
GDEPFNTVELYSLTETASQEAMGLFTKWSRSLGFLSQ

**Important features:**

**Signal peptide:**

amino acids 1-20

# FIGURE 505

GTTCCGCAGATGCAGAGGTTGAGGTGGCTGCGGGACTGGAAGTCATCGGGCAGAGGTCTCACA
GCAGCCAAGGAACCTGGGGCCCGCTCCTCCCCCCTCCAGGCC**ATG**AGGATTCTGCAGTTAATC
CTGCTTGCTCTGGCAACAGGGCTTGTAGGGGGAGAGACCAGGATCATCAAGGGGTTCGAGTGC
AAGCCTCACTCCCAGCCCTGGCAGGCAGCCCTGTTCGAGAAGACGCGGCTACTCTGTGGGGCG
ACGCTCATCGCCCCCAGATGGCTCCTGACAGCAGCCCACTGCCTCAAGCCCCGCTACATAGTT
CACCTGGGGCAGCACAACCTCCAGAAGGAGGAGGGCTGTGAGCAGACCCGGACAGCCACTGAG
TCCTTCCCCCACCCCGGCTTCAACAACAGCCTCCCCAACAAAGACCACCGCAATGACATCATG
CTGGTGAAGATGGCATCGCCAGTCTCCATCACCTGGGCTGTGCGACCCCTCACCCTCTCCTCA
CGCTGTGTCACTGCTGGCACCAGCTGCCTCATTTCCGGCTGGGGCAGCACGTCCAGCCCCCAG
TTACGCCTGCCTCACACCTTGCGATGCGCCAACATCACCATCATTGAGCACCAGAAGTGTGAG
AACGCCTACCCCGGCAACATCACAGACACCATGGTGTGTGCCAGCGTGCAGGAAGGGGGCAAG
GACTCCTGCCAGGGTGACTCCGGGGGCCCTCTGGTCTGTAACCAGTCTCTTCAAGGCATTATC
TCCTGGGGCCAGGATCCGTGTGCGATCACCCGAAAGCCTGGTGTCTACACGAAAGTCTGCAAA
TATGTGGACTGGATCCAGGAGACGATGAAGAACAAT**TAG**ACTGGACCCACCCACCACAGCCCA
TCACCCTCCATTTCCACTTGGTGTTTGGTTCCTGTTCACTCTGTTAATAAGAAACCCTAAGCC
AAGACCCTCTACGAACATTCTTTGGGCCTCCTGGACTACAGGAGATGCTGTCACTTAATAATC
AACCTGGGGTTCGAAATCAGTGAGACCTGGATTCAAATTCTGCCTTGAAATATTGTGACTCTG
GGAATGACAACACCTGGTTTGTTCTCTGTTGTATCCCCAGCCCCAAAGACAGCTCCTGGCCAT
ATATCAAGGTTTCAATAAATATTTGCTAAATGAAAAAAAAAAAAAAAAAAAAAAAAAAAAAAA
AAAAAAA

# FIGURE 506

MRILQLILLALATGLVGGETRIIKGFECKPHSQPWQAALFEKTRLLCGATLIAPRWLLTAAHC
LKPRYIVHLGQHNLQKEEGCEQTRTATESFPHPGFNNSLPNKDHRNDIMLVKMASPVSITWAV
RPLTLSSRCVTAGTSCLISGWGSTSSPQLRLPHTLRCANITIIEHQKCENAYPGNITDTMVCA
SVQEGGKDSCQGDSGGPLVCNQSLQGIISWGQDPCAITRKPGVYTKVCKYVDWIQETMKNN

**Important features:**

**Signal peptide:**

amino acids 1-18

**Serine proteases, trypsin family, histidine active site.**

amino acids 58-63

**N-glycosylation sites.**

amino acids 99-102, 165-168, 181-184, 210-213

**Glycosaminoglycan attachment site.**

amino acids 145-148

**Kringle domain proteins.**

amino acids 197-209, 47-64

**Serine proteases, trypsin family, histidine protein**

amino acids 199-209, 47-63, 220-243

**Apple domain proteins**

amino acids 222-249, 189-222

# FIGURE 507

CTGGGATCAGCCACTGCAGCTCCCTGAGCACTCTCTACAGAGACGCGGACCCCAGAC**ATG**AGG
AGGCTCCTCCTGGTCACCAGCCTGGTGGTTGTGCTGCTGTGGGAGGCAGGTGCAGTCCCAGCA
CCCAAGGTCCCTATCAAGATGCAAGTCAAACACTGGCCCTCAGAGCAGGACCCAGAGAAGGCC
TGGGGCGCCCGTGTGGTGGAGCCTCCGGAGAAGGACGACCAGCTGGTGGTGCTGTTCCCTGTC
CAGAAGCCGAAACTCTTGACCACCGAGGAGAAGCCACGAGGTCAGGGCAGGGGCCCCATCCTT
CCAGGCACCAAGGCCTGGATGGAGACCGAGGACACCCTGGGCCGTGTCCTGAGTCCCGAGCCC
GACCATGACAGCCTGTACCACCCTCCGCCTGAGGAGGACCAGGGCGAGGAGAGGCCCCGGTTG
TGGGTGATGCCAAATCACCAGGTGCTCCTGGGACCGGAGGAAGACCAAGACCACATCTACCAC
CCCCAG**TAG**GGCTCCAGGGGCCATCACTGCCCCCGCCCTGTCCCAAGGCCCAGGCTGTTGGGA
CTGGGACCCTCCCTACCCTGCCCCAGCTAGACAAATAAACCCCAGCAGGCAAAAAAAAAAAAA
AAAAAA

# FIGURE 508

MRRLLLVTSLVVVLLWEAGAVPAPKVPIKMQVKHWPSEQDPEKAWGARVVEPPEKDDQLVVLF
PVQKPKLLTTEEKPRGQGRGPILPGTKAWMETEDTLGRVLSPEPDHDSLYHPPPEEDQGEERP
RLWVMPNHQVLLGPEEDQDHIYHPQ

# FIGURE 509

GCGGAGCCGGCGCCGGCTGCGCAGAGGAGCCGCTCTCGCCGCCGCCACCTCGGCTGGGAGCCC
ACGAGGCTGCCGCATCCTGCCCTCGGAACA**ATG**GGACTCGGCGCGCGAGGTGCTTGGGCCGCG
CTGCTCCTGGGGACGCTGCAGGTGCTAGCGCTGCTGGGGGCCGCCCATGAAAGCGCAGCCATG
GCGGCATCTGCAAACATAGAGAATTCTGGGCTTCCACACAACTCCAGTGCTAACTCAACAGAG
ACTCTCCAACATGTGCCTTCTGACCATACAAATGAAACTTCCAACAGTACTGTGAAACCACCA
ACTTCAGTTGCCTCAGACTCCAGTAATACAACGGTCACCACCATGAAACCTACAGCGGCATCT
AATACAACAACACCAGGGATGGTCTCAACAAATATGACTTCTACCACCTTAAAGTCTACACCC
AAAACAACAAGTGTTTCACAGAACACATCTCAGATATCAACATCCACAATGACCGTAACCCAC
AATAGTTCAGTGACATCTGCTGCTTCATCAGTAACAATCACAACAACTATGCATTCTGAAGCA
AAGAAAGGATCAAAATTTGATACTGGGAGCTTTGTTGGTGGTATTGTATTAACGCTGGGAGTT
TTATCTATTCTTTACATTGGATGCAAAATGTATTACTCAAGAAGAGGCATTCGGTATCGAACC
ATAGATGAACATGATGCCATCATT**TAA**GGAAATCCATGGACCAAGGATGGAATACAGATTGAT
GCTGCCCTATCAATTAATTTTGGTTTATTAATAGTTTAAAACAATATTCTCTTTTTGAAAATA
GTATAAACAGGCCATGCATATAATGTACAGTGTATTACGTAAATATGTAAAGATTCTTCAAGG
TAACAAGGGTTTGGGTTTTGAAATAAACATCTGGATCTTATAGACCGTTCATACAATGGTTTT
AGCAAGTTCATAGTAAGACAAACAAGTCCTATCTTTTTTTTTTGGCTGGGGTGGGGGCATTGG
TCACATATGACCAGTAATTGAAAGACGTCATCACTGAAAGACAGAATGCCATCTGGGCATACA
AATAAGAAGTTTGTCACAGCACTCAGGATTTTGGGTATCTTTTGTAGCTCACATAAAGAACTT
CAGTGCTTTTCAGAGCTGGATATATCTTAATTACTAATGCCACACAGAAATTATACAATCAAA
CTAGATCTGAAGCATAATTTAAGAAAAACATCAACATTTTTTGTGCTTTAAACTGTAGTAGTT
GGTCTAGAAACAAAATACTCC

# FIGURE 510

MGLGARGAWAALLLGTLQVLALLGAAHESAAMAASANIENSGLPHNSSANSTETLQHVPSDHT
NETSNSTVKPPTSVASDSSNTTVTTMKPTAASNTTTPGMVSTNMTSTTLKSTPKTTSVSQNTS
QISTSTMTVTHNSSVTSAASSVTITTTMHSEAKKGSKFDTGSFVGGIVLTLGVLSILYIGCKM
YYSRRGIRYRTIDEHDAII

# FIGURE 511

GACTTTGCTTGAATGTTTACATTTTCTGCTCGCTGTCCTACATATCACAATATAGTGTTCACGTTTTGTTAAAAC
TTTGGGGTGTCAGGAGTTGAGCTTGCTCAGCAAGCCAGC**ATG**GCTAGGATGAGCTTTGTTATAGCAGCTTGCCAA
TTGGTGCTGGGCCTACTAATGACTTCATTAACCGAGTCTTCCATACAGAATAGTGAGTGTCCACAACTTTGCGTA
TGTGAAATTCGTCCCTGGTTTACCCCACAGTCAACTTACAGAGAAGCCACCACTGTTGATTGCAATGACCTCCGC
TTAACAAGGATTCCCAGTAACCTCTCTAGTGACACACAAGTGCTTCTCTTACAGAGCAATAACATCGCGAAGACT
GTGGATGAGCTGCAGCAGCTTTTCAACTTGACTGAACTAGATTTCTCCCAAAACAACTTTACTAACATTAAGGAG
GTCGGGCTGGCAAACCTAACCCAGCTCACAACGCTGCATTTGGAGGAAAATCAGATTACCGAGATGACTGATTAC
TGTCTACAAGACCTCAGCAACCTTCAAGAACTCTACATCAACCACAACCAAATTAGCACTATTTCTGCTCATGCT
TTTGCAGGCTTAAAAAATCTATTAAGGCTCCACCTGAACTCCAACAAATTGAAAGTTATTGATAGTCGCTGGTTT
GATTCTACACCCAACCTGGAAATTCTCATGATCGGAGAAAACCCTGTGATTGGAATTCTGGATATGAACTTCAAA
CCCCTCGCAAATTTGAGAAGCTTAGTTTTGGCAGGAATGTATCTCACTGATATTCCTGGAAATGCTTTGGTGGGT
CTGGATAGCCTTGAGAGCCTGTCTTTTTATGATAACAAACTGGTTAAAGTCCCTCAACTTGCCCTGCAAAAAGTT
CCAAATTTGAAATTCTTAGACCTCAACAAAAACCCCATTCACAAAATCCAAGAAGGGGACTTCAAAAATATGCTT
CGGTTAAAAGAACTGGGAATCAACAATATGGGCGAGCTCGTTTCTGTCGACCGCTATGCCCTGGATAACTTGCCT
GAACTCACAAAGCTGGAAGCCACCAATAACCCTAAACTCTCTTACATCCACCGCTTGGCTTTCCGAAGTGTCCCT
GCTCTGGAAAGCTTGATGCTGAACAACAATGCCTTGAATGCCATTTACCAAAAGACAGTCGAATCCCTCCCCAAT
CTGCGTGAGATCAGTATCCATAGCAATCCCCTCAGGTGTGACTGTGTGATCCACTGGATTAACTCCAACAAAACC
AACATCCGCTTCATGGAGCCCCTGTCCATGTTCTGTGCCATGCCGCCCGAATATAAAGGGCACCAGGTGAAGGAA
GTTTTAATCCAGGATTCGAGTGAACAGTGCCTCCCAATGATATCTCACGACAGCTTCCCAAATCGTTTAAACGTG
GATATCGGCACGACGGTTTTCCTAGACTGTCGAGCCATGGCTGAGCCAGAACCTGAAATTTACTGGGTCACTCCC
ATTGGAAATAAGATAACTGTGGAAACCCTTTCAGATAAATACAAGCTAAGTAGCGAAGGTACCTTGGAAATATCT
AACATACAAATTGAAGACTCAGGAAGATACACATGTGTTGCCCAGAATGTCCAAGGGGCAGACACTCGGGTGGCA
ACAATTAAGGTTAACGGGACCCTTCTGGATGGTACCCAGGTGCTAAAAATATACGTCAAGCAGACAGAATCCCAT
TCCATCTTAGTGTCCTGGAAAGTTAATTCCAATGTCATGACGTCAAACTTAAAATGGTCGTCTGCCACCATGAAG
ATTGATAACCCTCACATAACATATACTGCCAGGGTCCCAGTCGATGTCCATGAATACAACCTAACGCATCTGCAG
CCTTCCACAGATTATGAAGTGTGTCTCACAGTGTCCAATATTCATCAGCAGACTCAAAAGTCATGCGTAAATGTC
ACAACCAAAAATGCCGCCTTCGCAGTGGACATCTCTGATCAAGAAACCAGTACAGCCCTTGCTGCAGTAATGGGG
TCTATGTTTGCCGTCATTAGCCTTGCGTCCATTGCTGTGTACTTTGCCAAAAGATTTAAGAGAAAAAACTACCAC
CACTCATTAAAAAAGTATATGCAAAAAACCTCTTCAATCCCACTAAATGAGCTGTACCCACCACTCATTAACCTC
TGGGAAGGTGACAGCGAGAAAGACAAAGATGGTTCTGCAGACACCAAGCCAACCCAGGTCGACACATCCAGAAGC
TATTACATGTGG**TAA**CTCAGAGGATATTTTGCTTCTGGTAGTAAGGAGCACAAAGACGTTTTTGCTTTATTCTGC
AAAAGTGAACAAGTTGAAGACTTTTGTATTTTTGACTTTGCTAGTTTGTGGCAGAGTGGAGAGGACGGGTGGATA
TTTCAAATTTTTTTAGTATAGCGTATCGCAAGGGTTTGACACGGCTGCCAGCGACTCTAGGCTTCCAGTCTGTGT
TTGGTTTTTATTCTTATCATTATTATGATTGTTATTATATTATTATTTTATTTTAGTTGTTGTGCTAAACTCAAT
AATGCTGTTCTAACTACAGTGCTCAATAAAATGATTAATGACAGGAAAAAAAAAAAAAAAAAAAAAAAAAAAAAA
AAAAAAAAAAAAAAAAAAAAAAAAAAAAAAAAAAAAAAAAAA

# FIGURE 512

MARMSFVIAACQLVLGLLMTSLTESSIQNSECPQLCVCEIRPWFTPQSTYREATTVDCNDLRL
TRIPSNLSSDTQVLLLQSNNIAKTVDELQQLFNLTELDFSQNNFTNIKEVGLANLTQLTTLHL
EENQITEMTDYCLQDLSNLQELYINHNQISTISAHAFAGLKNLLRLHLNSNKLKVIDSRWFDS
TPNLEILMIGENPVIGILDMNFKPLANLRSLVLAGMYLTDIPGNALVGLDSLESLSFYDNKLV
KVPQLALQKVPNLKFLDLNKNPIHKIQEGDFKNMLRLKELGINNMGELVSVDRYALDNLPELT
KLEATNNPKLSYIHRLAFRSVPALESLMLNNNALNAIYQKTVESLPNLREISIHSNPLRCDCV
IHWINSNKTNIRFMEPLSMFCAMPPEYKGHQVKEVLIQDSSEQCLPMISHDSFPNRLNVDIGT
TVFLDCRAMAEPEPEIYWVTPIGNKITVETLSDKYKLSSEGTLEISNIQIEDSGRYTCVAQNV
QGADTRVATIKVNGTLLDGTQVLKIYVKQTESHSILVSWKVNSNVMTSNLKWSSATMKIDNPH
ITYTARVPVDVHEYNLTHLQPSTDYEVCLTVSNIHQQTQKSCVNVTTKNAAFAVDISDQETST
ALAAVMGSMFAVISLASIAVYFAKRFKRKNYHHSLKKYMQKTSSIPLNELYPPLINLWEGDSE
KDKDGSADTKPTQVDTSRSYYMW

**Important features:**

**Signal peptide:**

Amino acids 1-25

**Transmembrane domain:**

Amino acids 508-530

**N-glycosylation sites:**

Amino acids 69-73;96-100;106-110;117-121;385-389;517-521;
582-586;611-615

**Tyrosine kinase phosphorylation site:**

Amino acids 573-582

**N-myristoylation sites:**

Amino acids 16-22;224-230;464-470;637-643;698-704

# FIGURE 513

GGGAGAGAGGATAAATAGCAGCGTGGCTTCCCTGGCTCCTCTCTGCATCCTTCCCGACCTTCC
CAGCAAT**ATG**CATCTTGCACGTCTGGTCGGCTCCTGCTCCCTCCTTCTGCTACTGGGGGCCCT
GTCTGGATGGGCGGCCAGCGATGACCCCATTGAGAAGGTCATTGAAGGGATCAACCGAGGGCT
GAGCAATGCAGAGAGAGAGGTGGGCAAGGCCCTGGATGGCATCAACAGTGGAATCACGCATGC
CGGAAGGGAAGTGGAGAAGGTTTTCAACGGACTTAGCAACATGGGGAGCCACACCGGCAAGGA
GTTGGACAAAGGCGTCCAGGGGCTCAACCACGGCATGGACAAGGTTGCCCATGAGATCAACCA
TGGTATTGGACAAGCAGGAAAGGAAGCAGAGAAGCTTGGCCATGGGGTCAACAACGCTGCTGG
ACAGGCCGGGAAGGAAGCAGACAAAGCGGTCCAAGGGTTCCACACTGGGGTCCACCAGGCTGG
GAAGGAAGCAGAGAAACTTGGCCAAGGGGTCAACCATGCTGCTGACCAGGCTGGAAAGGAAGT
GGAGAAGCTTGGCCAAGGTGCCCACCATGCTGCTGGCCAGGCCGGGAAGGAGCTGCAGAATGC
TCATAATGGGGTCAACCAAGCCAGCAAGGAGGCCAACCAGCTGCTGAATGGCAACCATCAAAG
CGGATCTTCCAGCCATCAAGGAGGGGCCACAACCACGCCGTTAGCCTCTGGGGCCTCAGTCAA
CACGCCTTTCATCAACCTTCCCGCCCTGTGGAGGAGCGTCGCCAACATCATGCCC**TAA**ACTGG
CATCCGGCCTTGCTGGGAGAATAATGTCGCCGTTGTCACATCAGCTGACATGACCTGGAGGGG
TTGGGGGTGGGGGACAGGTTTCTGAAATCCCTGAAGGGGGTTGTACTGGGATTTGTGAATAAA
CTTGATACACCA

# FIGURE 514

MHLARLVGSCSLLLLLGALSGWAASDDPIEKVIEGINRGLSNAEREVGKALDGINSGITHAGR
EVEKVFNGLSNMGSHTGKELDKGVQGLNHGMDKVAHEINHGIGQAGKEAEKLGHGVNNAAGQA
GKEADKAVQGFHTGVHQAGKEAEKLGQGVNHAADQAGKEVEKLGQGAHHAAGQAGKELQNAHN
GVNQASKEANQLLNGNHQSGSSSHQGGATTTPLASGASVNTPFINLPALWRSVANIMP

**Important features:**

**Signal peptide:**

amino acids 1-25

**Homologous region to circumsporozoite (CS) repeats:**

amino acids 35-225

# FIGURE 515

CCCACGCGTCCGCCCACGCGTCCGGGTGCCACTCGCGCGCCGGCCGCGCTCCGGGCTTCTCTT
TTCCCTCCGACGCGCCACGGCTGCCCAGACATTCCGGCTGCCGGGTCTGGAGAGCTCCCCGAA
CCCCTCCGCGGAGAGGAGCGAGGCGGCGCCAGGGTGGCCCCCGGGGCGCGCTTGGTCTCGGAG
AAGCGGGGACGAGGCCGGAGGATGAGCGACTGAGGGCGACGCGGGCACTGACGCGAGTTGGGG
CCGCGACTACCGGCAGCTGACAGCGCGATGAGCGACTCCCCAGAGACGCCCTAGCCCGGTGTG
CGCGCCAGGCGGAGCGCGCAGGTGGGGCTGGGCTGTTAGTGGTCCGCCCCACGCGGGTCGCCG
GCCGGCCCAGGATGGGCGCTGGCAACCCGGGCCCGCGCCCGCCGCTGCTACCCCTGCGCCCGC
TGCGAGCCCGGCGTCCGGCCCGCGCCCTGCGCTCATGGACGGCGGCTCCCGGCTGGCGGCGGC
GCGCCCCCGGGCTGTGAATGCGACTCGCCCCTCGGCCGCGCTCCCCGCCCGCCCGCCCGCCGG
GACGTGGTAGGGG**ATG**CCCAGCTCCACTGCGATGGCAGTTGGCGCGCTCTCCAGTTCCCTCCT
GGTCACCTGCTGCCTGATGGTGGCTCTGTGCAGTCCGAGCATCCCGCTGGAGAAGCTGGCCCA
GGCACCAGAGCAGCCGGGCCAGGAGAAGCGTGAGCACGCCACTCGGGACGGCCCGGGGCGGGT
GAACGAGCTCGGGCGCCCGGCGAGGGACGAGGGCGGCAGCGGCCGGGACTGGAAGAGCAAGAG
CGGCCGTGGGCTCGCCGGCCGTGAGCCGTGGAGCAAGCTGAAGCAGGCCTGGGTCTCCCAGGG
CGGGGGCGCCAAGGCCGGGGATCTGCAGGTCCGGCCCCGCGGGGACACCCCGCAGGCGGAAGC
CCTGGCCGCAGCCGCCCAGGACGCGATTGGCCCGGAACTCGCGCCCACGCCCGAGCCACCCGA
GGAGTACGTGTACCCGGACTACCGTGGCAAGGGCTGCGTGGACGAGAGCGGCTTCGTGTACGC
GATCGGGGAGAAGTTCGCGCCGGGCCCCTCGGCCTGCCCGTGCCTGTGCACCGAGGAGGGGCC
GCTGTGCGCGCAGCCCGAGTGCCCGAGGCTGCACCCGCGCTGCATCCACGTCGACACGAGCCA
GTGCTGCCCGCAGTGCAAGGAGAGGAAGAACTACTGCGAGTTCCGGGGCAAGACCTATCAGAC
TTTGGAGGAGTTCGTGGTGTCTCCATGCGAGAGGTGTCGCTGTGAAGCCAACGGTGAGGTGCT
ATGCACAGTGTCAGCGTGTCCCCAGACGGAGTGTGTGGACCCTGTGTACGAGCCTGATCAGTG
CTGTCCCATCTGCAAAAATGGTCCAAACTGCTTTGCAGAAACCGCGGTGATCCCTGCTGGCAG
AGAAGTGAAGACTGACGAGTGCACCATATGCCACTGTACTTATGAGGAAGGCACATGGAGAAT
CGAGCGGCAGGCCATGTGCACGAGACATGAATGCAGGCAAATG**TAG**ACGCTTCCCAGAACACA
AACTCTGACTTTTTCTAGAACATTTTACTGATGTGAACATTCTAGATGACTCTGGGAACTATC
AGTCAAAGAAGACTTTTGATGAGGAATAATGGAAAATTGTTGGTACTTTTCCTTTTCTTGATA
ACAGTTACTACAACAGAAGGAAATGGATATATTTCAAAACATCAACAAGAACTTTGGGCATAA
AATCCTTCTCTAAATAAATGTGCTATTTTCACAGTAAGTACACAAAAGTACACTATTATATAT
CAAATGTATTTCTATAATCCCTCCATTAGAGAGCTTATATAAGTGTTTTCTATAGATGCAGAT
TAAAAATGCTGTGTTGTCAACCGTCAAAAAAAAAAAAAAAAAAAAAAAAAA

# FIGURE 516

MPSSTAMAVGALSSSLLVTCCLMVALCSPSIPLEKLAQAPEQPGQEKREHATRDGPGRVNELG
RPARDEGGSGRDWKSKSGRGLAGREPWSKLKQAWVSQGGGAKAGDLQVRPRGDTPQAEALAAA
AQDAIGPELAPTPEPPEEYVYPDYRGKGCVDESGFVYAIGEKFAPGPSACPCLCTEEGPLCAQ
PECPRLHPRCIHVDTSQCCPQCKERKNYCEFRGKTYQTLEEFVVSPCERCRCEANGEVLCTVS
ACPQTECVDPVYEPDQCCPICKNGPNCFAETAVIPAGREVKTDECTICHCTYEEGTWRIERQA
MCTRHECRQM

**Important features:**

**Signal peptide:**

amino acids 1-27

**Transmembrane domain:**

amino acids 11-30

**Glycosaminoglycan attachment site.**

amino acids 80-83

**N-myristoylation sites.**

amino acids 10-15, 102-107, 103-108

**Cell attachment sequence.**

amino acids 114-117

**EGF-like domain cysteine pattern signature.**

amino acids 176-187

# FIGURE 517

GGACAACCGTTGCTGGGTGTCCCAGGGCCTGAGGCAGGACGGTACTCCGCTGACACCTTCCCT
TTCGGCCTTGAGGTTCCCAGCCTGGTGGCCCCAGGACGTTCCGGTCGCATGGCAGAGTGCTAC
GGACGACGCCT**ATG**AAGCCCTTAGTCCTTCTAGTTGCGCTTTTGCTATGGCCTTCGTCTGTGC
CGGCTTATCCGAGCATAACTGTGACACCTGATGAAGAGCAAAACTTGAATCATTATATACAAG
TTTTAGAGAACCTAGTACGAAGTGTTCCCTCTGGGGAGCCAGGTCGTGAGAAAAAATCTAACT
CTCCAAAACATGTTTATTCTATAGCATCAAAGGGATCAAAATTTAAGGAGCTAGTTACACATG
GAGACGCTTCAACTGAGAATGATGTTTTAACCAATCCTATCAGTGAAGAAACTACAACTTTCC
CTACAGGAGGCTTCACACCGGAAATAGGAAAGAAAAAACACACGGAAAGTACCCCATTCTGGT
CGATCAAACCAAACAATGTTTCCATTGTTTGCATGCAGAGGAACCTTATATTGAAAATGAAG
AGCCAGAGCCAGAGCCGGAGCCAGCTGCAAAACAAACTGAGGCACCAAGAATGTTGCCAGTTG
TTACTGAATCATCTACAAGTCCATATGTTACCTCATACAAGTCACCTGTCACCACTTTAGATA
AGAGCACTGGCATTGAGATCTCTACAGAATCAGAAGATGTTCCTCAGCTCTCAGGTGAAACTG
CGATAGAAAAACCCGAAGAGTTTGGAAAGCACCCAGAGAGTTGGAATAATGATGACATTTTGA
AAAAAATTTTAGATATTAATTCACAAGTGCAACAGGCACTTCTTAGTGACACCAGCAACCCAG
CATATAGAGAAGATATTGAAGCCTCTAAAGATCACCTAAAACGAAGCCTTGCTCTAGCAGCAG
CAGCAGAACATAAATTAAAAACAATGTATAAGTCCCAGTTATTGCCAGTAGGACGAACAAGTA
ATAAAATTGATGACATCGAAACTGTTATTAACATGCTGTGTAATTCTAGATCTAAACTCTATG
AATATTTAGATATTAAATGTGTTCCACCAGAGATGAGAGAAAAAGCTGCTACAGTATTCAATA
CATTAAAAAATATGTGTAGATCAAGGAGAGTCACAGCCTTATTAAAAGTTTAT**TAA**ACAATAA
TATAAAAATTTTAAACCTACTTGATATTCCATAACAAAGCTGATTTAAGCAAACTGCATTTTT
TCACAGGAGAAATAATCATATTCGTAATTTCAAAAGTTGTATAAAAATATTTTCTATTGTAGT
TCAAATGTGCCAACATCTTTATGTGTCATGTGTTATGAACAATTTTCATATGCACTAAAAACC
TAATTTAAAATAAAATTTTGGTTCAGGAAAAAA

# FIGURE 518

MKPLVLLVALLLWPSSVPAYPSITVTPDEEQNLNHYIQVLENLVRSVPSGEPGREKKSNSPKH
VYSIASKGSKFKELVTHGDASTENDVLTNPISEETTTFPTGGFTPEIGKKKHTESTPFWSIKP
NNVSIVLHAEEPYIENEEPEPEPEPAAKQTEAPRMLPVVTESSTSPYVTSYKSPVTTLDKSTG
IEISTESEDVPQLSGETAIEKPEEFGKHPESWNNDDILKKILDINSQVQQALLSDTSNPAYRE
DIEASKDHLKRSLALAAAAEHKLKTMYKSQLLPVGRTSNKIDDIETVINMLCNSRSKLYEYLD
IKCVPPEMREKAATVFNTLKNMCRSRRVTALLKVY

**Important features:**

**Signal peptide:**

amino acids 1-19

# FIGURE 519

CGGCTCGAGTGCAGCTGTGGGGAGATTTCAGTGCATTGCCTCCCCTGGGTGCTCTTCATCTTG
GATTTGAAAGTTGAGAGCAGC**ATG**TTTTGCCCACTGAAACTCATCCTGCTGCCAGTGTTACTG
GATTATTCCTTGGGCCTGAATGACTTGAATGTTTCCCCGCCTGAGCTAACAGTCCATGTGGGT
GATTCAGCTCTGATGGGATGTGTTTTCCAGAGCACAGAAGACAAATGTATATTCAAGATAGAC
TGGACTCTGTCACCAGGAGAGCACGCCAAGGACGAATATGTGCTATACTATTACTCCAATCTC
AGTGTGCCTATTGGGCGCTTCCAGAACCGCGTACACTTGATGGGGGACATCTTATGCAATGAT
GGCTCTCTCCTGCTCCAAGATGTGCAAGAGGCTGACCAGGGAACCTATATCTGTGAAATCCGC
CTCAAAGGGGAGAGCCAGGTGTTCAAGAAGGCGGTGGTACTGCATGTGCTTCCAGAGGAGCCC
AAAGAGCTCATGGTCCATGTGGGTGGATTGATTCAGATGGGATGTGTTTTCCAGAGCACAGAA
GTGAAACACGTGACCAAGGTAGAATGGATATTTTCAGGACGGCGCGCAAAGGAGGAGATTGTA
TTTCGTTACTACCACAAACTCAGGATGTCTGTGGAGTACTCCCAGAGCTGGGGCCACTTCCAG
AATCGTGTGAACCTGGTGGGGGACATTTTCCGCAATGACGGTTCCATCATGCTTCAAGGAGTG
AGGGAGTCAGATGGAGGAAACTACACCTGCAGTATCCACCTAGGGAACCTGGTGTTCAAGAAA
ACCATTGTGCTGCATGTCAGCCCGGAAGAGCCTCGAACACTGGTGACCCCGGCAGCCCTGAGG
CCTCTGGTCTTGGGTGGTAATCAGTTGGTGATCATTGTGGGAATTGTCTGTGCCACAATCCTG
CTGCTCCCTGTTCTGATATTGATCGTGAAGAAGACCTGTGGAAATAAGAGTTCAGTGAATTCT
ACAGTCTTGGTGAAGAACACGAAGAAGACTAATCCAGAGATAAAAGAAAAACCCTGCCATTTT
GAAAGATGTGAAGGGGAGAAACACATTTACTCCCCAATAATTGTACGGGAGGTGATCGAGGAA
GAAGAACCAAGTGAAAAATCAGAGGCCACCTACATGACCATGCACCCAGTTTGGCCTTCTCTG
AGGTCAGATCGGAACAACTCACTTGAAAAAAAGTCAGGTGGGGGAATGCCAAAAACACAGCAA
GCCTTT**TGA**GAAGAATGGAGAGTCCCTTCATCTCAGCAGCGGTGGAGACTCTCTCCTGTGTGT
GTCCTGGGCCACTCTACCAGTGATTTCAGACTCCCGCTCTCCCAGCTGTCCTCCTGTCTCATT
GTTTGGTCAATACACTGAAGATGGAGAATTTGGAGCCTGGCAGAGAGACTGGACAGCTCTGGA
GGAACAGGCCTGCTGAGGGGAGGGGAGCATGGACTTGGCCTCTGGAGTGGGACACTGGCCCTG
GGAACCAGGCTGAGCTGAGTGGCCTCAAACCCCCCGTTGGATCAGACCCTCCTGTGGGCAGGG
TTCTTAGTGGATGAGTTACTGGGAAGAATCAGAGATAAAAACCAACCCAAATCAA

# FIGURE 520

MFCPLKLILLPVLLDYSLGLNDLNVSPPELTVHVGDSALMGCVFQSTEDKCIFKIDWTLSPGE
HAKDEYVLYYYSNLSVPIGRFQNRVHLMGDILCNDGSLLLQDVQEADQGTYICEIRLKGESQV
FKKAVVLHVLPEEPKELMVHVGGLIQMGCVFQSTEVKHVTKVEWIFSGRRAKEEIVFRYYHKL
RMSVEYSQSWGHFQNRVNLVGDIFRNDGSIMLQGVRESDGGNYTCSIHLGNLVFKKTIVLHVS
PEEPRTLVTPAALRPLVLGGNQLVIIVGIVCATILLLPVLILIVKKTCGNKSSVNSTVLVKNT
KKTNPEIKEKPCHFERCEGEKHIYSPIIVREVIEEEEPSEKSEATYMTMHPVWPSLRSDRNNS
LEKKSGGGMPKTQQAF

# FIGURE 521

CTATGAAGAAGCTTCCTGGAAAACAATAAGCAAAGGAAAACAAATGTGTCCCATCTCACATGG
TTCTACCCTACTAAAGACAGGAAGATCATAAACTGACAGATACTGAAATTGTAAGAGTTGGAA
ACTACATTTTGCAAAGTCATTGAACTCTGAGCTCAGTTGCAGTACTCGGGAAGCC**ATG**CAGGA
TGAAGATGGATACATCACCTTAAATATTAAAACTCGGAAACCAGCTCTCGTCTCCGTTGGCCC
TGCATCCTCCTCCTGGTGGCGTGTGATGGCTTTGATTCTGCTGATCCTGTGCGTGGGGATGGT
TGTCGGGCTGGTGGCTCTGGGGATTTGGTCTGTCATGCAGCGCAATTACCTACAAGATGAGAA
TGAAAATCGCACAGGAACTCTGCAACAATTAGCAAAGCGCTTCTGTCAATATGTGGTAAAACA
ATCAGAACTAAAGGGCACTTTCAAAGGTCATAAATGCAGCCCCTGTGACACAAACTGGAGATA
TTATGGAGATAGCTGCTATGGGTTCTTCAGGCACAACTTAACATGGGAAGAGAGTAAGCAGTA
CTGCACTGACATGAATGCTACTCTCCTGAAGATTGACAACCGGAACATTGTGGAGTACATCAA
AGCCAGGACTCATTTAATTCGTTGGGTCGGATTATCTCGCCAGAAGTCGAATGAGGTCTGGAA
GTGGGAGGATGGCTCGGTTATCTCAGAAAATATGTTTGAGTTTTTGGAAGATGGAAAAGGAAA
TATGAATTGTGCTTATTTTCATAATGGGAAAATGCACCCTACCTTCTGTGAGAACAAACATTA
TTTAATGTGTGAGAGGAAGGCTGGCATGACCAAGGTGGACCAACTACCT**TAA**TGCAAAGAGGT
GGACAGGATAACACAGATAAGGGCTTTATTGTACAATAAAAGATATGTATGAATGCATCAGTA
GCTGAAAAAAAAAAAAAAA

# FIGURE 522

MQDEDGYITLNIKTRKPALVSVGPASSSWWRVMALILLILCVGMVVGLVALGIWSVMQRNYLQ
DENENRTGTLQQLAKRFCQYVVKQSELKGTFKGHKCSPCDTNWRYYGDSCYGFFRHNLTWEES
KQYCTDMNATLLKIDNRNIVEYIKARTHLIRWVGLSRQKSNEVWKWEDGSVISENMFEFLEDG
KGNMNCAYFHNGKMHPTFCENKHYLMCERKAGMTKVDQLP

# FIGURE 523

CAGCAGTGGTCTCTCAGTCCTCTCAAAGCAAGGAAAGAGTACTGTGTGCTGAGAGACC**ATG**GC
AAAGAATCCTCCAGAGAATTGTGAAGACTGTCACATTCTAAATGCAGAAGCTTTTAAATCCAA
GAAAATATGTAAATCACTTAAGATTTGTGGACTGGTGTTTGGTATCCTGGCCCTAACTCTAAT
TGTCCTGTTTTGGGGGAGCAAGCACTTCTGGCCGGAGGTACCCAAAAAAGCCTATGACATGGA
GCACACTTTCTACAGCAATGGAGAGAAGAAGAAGATTTACATGGAAATTGATCCTGTGACCAG
AACTGAAATATTCAGAAGCGGAAATGGCACTGATGAAACATTGGAAGTGCACGACTTTAAAAA
CGGATACACTGGCATCTACTTCGTGGGTCTTCAAAAATGTTTTATCAAAACTCAGATTAAAGT
GATTCCTGAATTTTCTGAACCAGAAGAGGAAATAGATGAGAATGAAGAAATTACCACAACTTT
CTTTGAACAGTCAGTGATTTGGGTCCCAGCAGAAAAGCCTATTGAAAACCGAGATTTTCTTAA
AAATTCCAAAATTCTGGAGATTTGTGATAACGTGACCATGTATTGGATCAATCCCACTCTAAT
ATCAGTTTCTGAGTTACAAGACTTTGAGGAGGAGGGAGAAGATCTTCACTTTCCTGCCAACGA
AAAAAAAGGGATTGAACAAAATGAACAGTGGGTGGTCCCTCAAGTGAAAGTAGAGAAGACCCG
TCACGCCAGACAAGCAAGTGAGGAAGAACTTCCAATAAATGACTATACTGAAAATGGAATAGA
ATTTGATCCCATGCTGGATGAGAGAGGTTATTGTTGTATTTACTGCCGTCGAGGCAACCGCTA
TTGCCGCCGCGTCTGTGAACCTTTACTAGGCTACTACCCATATCCATACTGCTACCAAGGAGG
ACGAGTCATCTGTCGTGTCATCATGCCTTGTAACTGGTGGGTGGCCCGCATGCTGGGGAGGGT
C**TAA**TAGGAGGTTTGAGCTCAAATGCTTAAACTGCTGGCAACATATAATAAATGCATGCTATT
CAATGAATTTCTGCCTATGAGGCATCTGGCCCCTGGTAGCCAGCTCTCCAGAATTACTTGTAG
GTAATTCCTCTCTTCATGTTCTAATAAACTTCTACATTATCACCAAAAAAAAAAAAAAAAAAA

# FIGURE 524

MAKNPPENCEDCHILNAEAFKSKKICKSLKICGLVFGILALTLIVLFWGSKHFWPEVPKKAYD
MEHTFYSNGEKKKIYMEIDPVTRTEIFRSGNGTDETLEVHDFKNGYTGIYFVGLQKCFIKTQI
KVIPEFSEPEEEIDENEEITTTFFEQSVIWVPAEKPIENRDFLKNSKILEICDNVTMYWINPT
LISVSELQDFEEEGEDLHFPANEKKGIEQNEQWVVPQVKVEKTRHARQASEEELPINDYTENG
IEFDPMLDERGYCCIYCRRGNRYCRRVCEPLLGYYPYPYCYQGGRVICRVIMPCNWWVARMLGRV

**Important features:**

**Signal peptide:**

amino acids 1-40

**Transmembrane domain:**

amino acids 25-47 (type II)

**N-glycosylation sites.**

amino acids 94-97, 180-183

**Glycosaminoglycan attachment sites.**

amino acids 92-95, 70-73, 85-88, 133-136, 148-151, 192-195, 239-242

**N-myristoylation sites.**

amino acids 33-38, 95-100, 116-121, 215-220, 272-277

**Microbodies C-terminal targeting signal.**

amino acids 315-317

**Cytochrome c family heme-binding site signature.**

amino acids 9-14

# FIGURE 525

AGTGACAATCTCAGAGCAGCTTCTACACCACAGCCATTTCCAGC**ATG**AAGATCACTGGGGGTC
TCCTTCTGCTCTGTACAGTGGTCTATTTCTGTAGCAGCTCAGAAGCTGCTAGTCTGTCTCCAA
AAAAAGTGGACTGCAGCATTTACAAGAAGTATCCAGTGGTGGCCATCCCCTGCCCCATCACAT
ACCTACCAGTTTGTGGTTCTGACTACATCACCTATGGGAATGAATGTCACTTGTGTACCGAGA
GCTTGAAAAGTAATGGAAGAGTTCAGTTTCTTCACGATGGAAGTTGC**TAA**ATTCTCCATGGAC
ATAGAGAGAAAGGAATGATATTCTCATCATCATCTTCATCATCCCAGGCTCTGACTGAGTTTC
TTTCAGTTTTACTGATGTTCTGGGTGGGGGACAGAGCCAGATTCAGAGTAATCTTGACTGAAT
GGAGAAAGTTTCTGTGCTACCCCTACAAACCCATGCCTCACTGACAGACCAGCATTTTTTTT
TAACACGTCAATAAAAAAATAATCTCCCAGA

# FIGURE 526

MKITGGLLLLCTVVYFCSSSEAASLSPKKVDCSIYKKYPVVAIPCPITYLPVCGSDYITYGNE
CHLCTESLKSNGRVQFLHDGSC

**Important features:**

**Signal peptide:**

amino acids 1-19

# FIGURE 527

CGACG**ATG**CTACGCGCGCCCGGCTGCCTCCTCCGGACCTCCGTAGCGCCTGCCGCGGCCCTGG
CTGCGGCGCTGCTCTCGTCGCTTGCGCGCTGCTCTCTTCTAGAGCCGAGGGACCCGGTGGCCT
CGTCGCTCAGCCCCTATTTCGGCACCAAGACTCGCTACGAGGATGTCAACCCCGTGCTATTGT
CGGGCCCCGAGGCTCCGTGGCGGGACCCTGAGCTGCTGGAGGGGACCTGCACCCCGGTGCAGC
TGGTCGCCCTCATTCGCCACGGCACCCGCTACCCCACGGTCAAACAGATCCGCAAGCTGAGGC
AGCTGCACGGGTTGCTGCAGGCCCGCGGGTCCAGGGATGGCGGGGCTAGTAGTACCGGCAGCC
GCGACCTGGGTGCAGCGCTGGCCGACTGGCCTTTGTGGTACGCGGACTGGATGGACGGGCAGC
TAGTAGAGAAGGGACGGCAGGATATGCGACAGCTGGCGCTGCGTCTGGCCTCGCTCTTCCCGG
CCCTTTTCAGCCGTGAGAACTACGGCCGCCTGCGGCTCATCACCAGTTCCAAGCACCGCTGCA
TGGATAGCAGCGCCGCCTTCCTGCAGGGGCTGTGGCAGCACTACCACCCTGGCTTGCCGCCGC
CGGACGTCGCAGATATGGAGTTTGGACCTCCAACAGTTAATGATAAACTAATGAGATTTTTTG
ATCACTGTGAGAAGTTTTTAACTGAAGTAGAAAAAAATGCTACAGCTCTTTATCACGTGGAAG
CCTTCAAAACTGGACCAGAAATGCAGAACATTTTAAAAAAAGTTGCAGCTACTTTGCAAGTGC
CAGTAAATGATTTAAATGCAGATTTAATTCAAGTAGCCTTTTTCACCTGTTCATTTGACCTGG
CAATTAAAGGTGTTAAATCTCCTTGGTGTGATGTTTTTGACATAGATGATGCAAAGGTATTAG
AATATTTAAATGATCTGAAACAATATTGGAAAAGAGGATATGGGTATACTATTAACAGTCGAT
CCAGCTGCACCTTGTTTCAGGATATCTTTCAGCACTTGGACAAAGCAGTTGAACAGAAACAAA
GGTCTCAGCCAATTTCTTCTCCAGTCATCCTCCAGTTTGGTCATGCAGAGACTCTTCTTCCAC
TGCTTTCTCTCATGGGCTACTTCAAAGACAAGGAACCCCTAACAGCGTACAATTACAAAAAAC
AAATGCATCGGAAGTTCCGAAGTGGTCTCATTGTACCTTATGCCTCGAACCTGATATTTGTGC
TTTACCACTGTGAAAATGCTAAGACTCCTAAAGAACAATTCCGAGTGCAGATGTTATTAAATG
AAAAGGTGTTACCTTTGGCTTACTCACAAGAAACTGTTTCATTTTATGAAGATCTGAAGAACC
ACTACAAGGACATCCTTCAGAGTTGTCAAACCAGTGAAGAATGTGAATTAGCAAGGGCTAACA
GTACATCTGATGAACTA**TGA**GTAACTGAAGAACATTTTTAATTCTTTAGGAATCTGCAATGAG
TGATTACATGCTTGTAATAGGTAGGCAATTCCTTGATTACAGGAAGCTTTTATATTACTTGAG
TATTTCTGTCTTTTCACAGAAAAACATTGGGTTTCTCTCTGGGTTTGGACATGAAATGTAAGA
AAAGATTTTTCACTGGAGCAGCTCTCTTAAGGAGAAACAAATCTATTTAGAGAAACAGCTGGC
CCTGCAAATGTTTACAGAAATGAAATTCTTCCTACTTATATAAGAAATCTCACACTGAGATAG
AATTGTGATTTCATAATAACACTTGAAAAGTGCTGGAGTAACAAAATATCTCAGTTGGACCAT
CCTTAACTTGATTGAACTGTCTAGGAACTTTACAGATTGTTCTGCAGTTCTCTCTTCTTTTCC
TCAGGTAGGACAGCTCTAGCATTTTCTTAATCAGGAATATTGTGGTAAGCTGGGAGTATCACT
CTGGAAGAAAGTAACATCTCCAGATGAGAATTTGAAACAAGAAACAGAGTGTTGTAAAAGGAC
ACCTTCACTGAAGCAAGTCGGAAAGTACAATGAAAATAAATATTTTTGGTATTTATTTATGAA
ATATTTGAACATTTTTTCAATAATTCCTTTTTACTTCTAGGAAGTCTCAAAAGACCATCTTAA
ATTATTATATGTTTGGACAATTAGCAACAAGTCAGATAGTTAGAATCGAAGTTTTTCAAATCC
ATTGCTTAGCTAACTTTTTCATTCTGTCACTTGGCTTCGATTTTTATATTTTCCTATTATATG
AAATGTATCTTTTGGTTGTTTGATTTTTCTTTCTTTCTTTGTAAATAGTTCTGAGTTCTGTCA
AATGCCGTGAAAGTATTTGCTATAATAAAGAAAATTCTTGTGACTTTAAAAAAAAA

# FIGURE 528

MLRAPGCLLRTSVAPAAALAAALLSSLARCSLLEPRDPVASSLSPYFGTKTRYEDVNPVLLSG
PEAPWRDPELLEGTCTPVQLVALIRHGTRYPTVKQIRKLRQLHGLLQARGSRDGGASSTGSRD
LGAALADWPLWYADWMDGQLVEKGRQDMRQLALRLASLFPALFSRENYGRLRLITSSKHRCMD
SSAAFLQGLWQHYHPGLPPPDVADMEFGPPTVNDKLMRFFDHCEKFLTEVEKNATALYHVEAF
KTGPEMQNILKKVAATLQVPVNDLNADLIQVAFFTCSFDLAIKGVKSPWCDVFDIDDAKVLEY
LNDLKQYWKRGYGYTINSRSSCTLFQDIFQHLDKAVEQKQRSQPISSPVILQFGHAETLLPLL
SLMGYFKDKEPLTAYNYKKQMHRKFRSGLIVPYASNLIFVLYHCENAKTPKEQFRVQMLLNEK
VLPLAYSQETVSFYEDLKNHYKDILQSCQTSEECELARANSTSDEL

**Important features:**

**Signal sequence**

amino acids 1-30

**N-glycosylation sites.**

amino acids 242-246, 481-485

**N-myristoylation sites.**

amino acids 107-113, 113-119, 117-123, 118-124, 128-134

**Endoplasmic reticulum targeting sequence.**

amino acids 484-489

# FIGURE 529

GGAGAGCCGCGGCTGGGACCGGAGTGGGGAGCGCGGCGTGGAGGTGCCACCCGGCGCGGGTGG
CGGAGAGATCAGAAGCCTCTTCCCCAAGCCGAGCCAACCTCAGCGGGGACCCGGGCTCAGGGA
CGCGGCGGCGGCGGCGGCGACTGCAGTGGCTGGACG**ATG**GCAGCGTCCGCCGGAGCCGGGGCG
GTGATTGCAGCCCCAGACAGCCGGCGCTGGCTGTGGTCGGTGCTGGCGGCGGCGCTTGGGCTC
TTGACAGCTGGAGTATCAGCCTTGGAAGTATATACGCCAAAAGAAATCTTCGTGGCAAATGGT
ACACAAGGGAAGCTGACCTGCAAGTTCAAGTCTACTAGTACGACTGGCGGGTTGACCTCAGTC
TCCTGGAGCTTCCAGCCAGAGGGGGCCGACACTACTGTGTCGTTTTTCCACTACTCCCAAGGG
CAAGTGTACCTTGGGAATTATCCACCATTTAAAGACAGAATCAGCTGGGCTGGAGACCTTGAC
AAGAAAGATGCATCAATCAACATAGAAAATATGCAGTTTATACACAATGGCACCTATATCTGT
GATGTCAAAAACCCTCCTGACATCGTTGTCCAGCCTGGACACATTAGGCTCTATGTCGTAGAA
AAAGAGAATTTGCCTGTGTTTCCAGTTTGGGTAGTGGTGGGCATAGTTACTGCTGTGGTCCTA
GGTCTCACTCTGCTCATCAGCATGATTCTGGCTGTCCTCTATAGAAGGAAAAACTCTAAACGG
GATTACACTGGCTGCAGTACATCAGAGAGTTTGTCACCAGTTAAGCAGGCTCCTCGGAAGTCC
CCCTCCGACACTGAGGGTCTTGTAAAGAGTCTGCCTTCTGGATCTCACCAGGGCCCAGTCATA
TATGCACAGTTAGACCACTCCGGCGGACATCACAGTGACAAGATTAACAAGTCAGAGTCTGTG
GTGTATGCGGATATCCGAAAGAAT**TAA**GAGAATACCTAGAACATATCCTCAGCAAGAAACAAA
ACCAAACTGGACTCTCGTGCAGAAAATGTAGCCCATTACCACATGTAGCCTTGGAGACCCAGG
CAAGGACAAGTACACGTGTACTCACAGAGGGAGAGAAAGATGTGTACAAAGGATATGTATAAA
TATTCTATTTAGTCATCCTGATATGAGGAGCCAGTGTTGCATGATGAAAAGATGGTATGATTC
TACATATGTACCCATTGTCTTGCTGTTTTTGTACTTTCTTTTCAGGTCATTTACAATTGGGAG
ATTTCAGAAACATTCCTTTCACCATCATTTAGAAATGGTTTGCCTTAATGGAGACAATAGCAG
ATCCTGTAGTATTTCCAGTAGACATGGCCTTTTAATCTAAGGGCTTAAGACTGATTAGTCTTA
GCATTTACTGTAGTTGGAGGATGGAGATGCTATGATGGAAGCATACCCAGGGTGGCCTTTAGC
ACAGTATCAGTACCATTTATTTGTCTGCCGCTTTTAAAAAATACCCATTGGCTATGCCACTTG
AAAACAATTTGAGAAGTTTTTTTGAAGTTTTTCTCACTAAAATATGGGGCAATTGTTAGCCTT
ACATGTTGTGTAGACTTACTTTAAGTTTGCACCCTTGAAATGTGTCATATCAATTTCTGGATT
CATAATAGCAAGATTAGCAAAGGATAAATGCCGAAGGTCACTTCATTCTGGACACAGTTGGAT
CAATACTGATTAAGTAGAAAATCCAAGCTTTGCTTGAGAACTTTTGTAACGTGGAGAGTAAAA
AGTATCGGTTTTA

# FIGURE 530

MAASAGAGAVIAAPDSRRWLWSVLAAALGLLTAGVSALEVYTPKEIFVANGTQGKLTCKFKST
STTGGLTSVSWSFQPEGADTTVSFFHYSQGQVYLGNYPPFKDRISWAGDLDKKDASINIENMQ
FIHNGTYICDVKNPPDIVVQPGHIRLYVVEKENLPVFPVWVVVGIVTAVVLGLTLLISMILAV
LYRRKNSKRDYTGCSTSESLSPVKQAPRKSPSDTEGLVKSLPSGSHQGPVIYAQLDHSGGHHS
DKINKSESVVYADIRKN

**Important features:**

**Signal peptide:**

amino acids 1-37

**Transmembrane domain:**

amino acids 161-183

# FIGURE 531

GTGACACTATAGAAGAGCTATGACGTCGCATGCACGCGTACGTAAGCTCGGAATTCGGCTCGA
GGCTGGTGGGAAGAAGCCGAG**ATG**GCGGCAGCCAGCGCTGGGGCAACCCGGCTGCTCCTGCTC
TTGCTGATGGCGGTAGCAGCGCCCAGTCGAGCCCGGGGCAGCGGCTGCCGGGCCGGGACTGGT
GCGCGAGGGGCTGGGGCGGAAGGTCGAGAGGGCGAGGCCTGTGGCACGGTGGGGCTGCTGCTG
GAGCACTCATTTGAGATCGATGACAGTGCCAACTTCCGGAAGCGGGGCTCACTGCTCTGGAAC
CAGCAGGATGGTACCTTGTCCCTGTCACAGCGGCAGCTCAGCGAGGAGGAGCGGGGCCGACTC
CGGGATGTGGCAGCCCTGAATGGCCTGTACCGGGTCCGGATCCCAAGGCGACCCGGGGCCCTG
GATGGCCTGGAAGCTGGTGGCTATGTCTCCTCCTTTGTCCCTGCGTGCTCCCTGGTGGAGTCG
CACCTGTCGGACCAGCTGACCCTGCACGTGGATGTGGCCGGCAACGTGGTGGGCGTGTCGGTG
GTGACGCACCCCGGGGGCTGCCGGGGCCATGAGGTGGAGGACGTGGACCTGGAGCTGTTCAAC
ACCTCGGTGCAGCTGCAGCCGCCCACCACAGCCCCAGGCCCTGAGACGGCGGCCTTCATTGAG
CGCCTGGAGATGGAACAGGCCCAGAAGGCCAAGAACCCCCAGGAGCAGAAGTCCTTCTTCGCC
AAATACTGGATGTACATCATTCCCGTCGTCCTGTTCCTCATGATGTCAGGAGCGCCAGACACC
GGGGGCCAGGGTGGGGGTGGGGGTGGGGGTGGTGGTGGGGGTAGTGGCCTTTGCTGTGTGCCA
CCCTCCCTG**TAA**GTCTATTTAAAAACATCGACGATACATTGAAATGTGTGAACGTTTTGAAAA
GCTACAGCTTCCAGCAGCCAAAAGCAACTGTTGTTTTGGCAAGACGGTCCTGATGTACAAGCT
TGATTGAAATTCACTGCTCACTTGATACGTTATTCAGAAACCCAAGGAATGGCTGTCCCCATC
CTCATGTGGCTGTGTGGAGCTCAGCTGTGTTGTGTGGCAGTTTATTAAACTGTCCCCCAGATC
GACACGCAAAAAAAAA

# FIGURE 532

MAAASAGATRLLLLLLMAVAAPSRARGSGCRAGTGARGAGAEGREGEACGTVGLLLEHSFEID
DSANFRKRGSLLWNQQDGTLSLSQRQLSEEERGRLRDVAALNGLYRVRIPRRPGALDGLEAGG
YVSSFVPACSLVESHLSDQLTLHVDVAGNVVGVSVVTHPGGCRGHEVEDVDLELFNTSVQLQP
PTTAPGPETAAFIERLEMEQAQKAKNPQEQKSFFAKYWMYIIPVVLFLMMSGAPDTGGQGGGG
GGGGGGGSGLCCVPPSL

**Important features:**

**Signal peptide:**

amino acids 1-24

**Transmembrane domain:**

amino acids 226-243

# FIGURE 533

TCTGCCTCCACTGCTCTGTGCTGGGATC**ATG**GAACTTGCACTGCTGTGTGGGCTGGTGGTGAT
GGCTGGTGTGATTCCAATCCAGGGCGGGATCCTGAACCTGAACAAGATGGTCAAGCAAGTGAC
TGGGAAAATGCCCATCCTCTCCTACTGGCCCTACGGCTGTCACTGCGGACTAGGTGGCAGAGG
CCAACCCAAAGATGCCACGGACTGGTGCTGCCAGACCCATGACTGCTGCTATGACCACCTGAA
GACCCAGGGGTGCGGCATCTACAAGGACAACAACAAAAGCAGCATACATTGTATGGATTTATC
TCAACGCTATTGTTTAATGGCTGTGTTTAATGTGATCTATCTGGAAAATGAGGACTCCGAA**TA**
**A**AAAGCTATTACTAWTTNAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA

# FIGURE 534

MELALLCGLVVMAGVIPIQGGILNLNKMVKQVTGKMPILSYWPYGCHCGLGGRGQPKDATDWC
CQTHDCCYDHLKTQGCGIYKDNNKSSIHCMDLSQRYCLMAVFNVIYLENEDSE

**Important features:**

**Signal peptide:**

amino acids 1-17

**Transmembrane domain:**

amino acids 1-24

**N-glycosylation site.**

amino acids 86-89

**N-myristoylation sites.**

amino acids 20-25, 45-50

**Phospholipase A2 histidine active site.**

amino acids 63-70

# FIGURE 535

GCTGAGCGTGTGCGCGGTACGGGGCTCTCCTGCCTTCTGGGCTCCAACGCAGCTCTGTGGCTG
AACTGGGTGCTCATCACGGGAACTGCTGGGCTATGGAATACAGATGTGGCAGCTCAGGTAGCC
CCAAATTGCCTGGAAGAATACATCATGTTTTTCGATAAGAAGAAATTGTAGGATCCAGTTTTT
TTTTTAACCGCCCCCTCCCCACCCCCCAAAAAAACTGTAAAGATGCAAAAACGTAATATCCAT
GAAGATCCTATTACCTAGGAAGATTTTGATGTTTTGCTGCGAATGCGGTGTTGGGATTTATTT
GTTCTTGGAGTGTTCTGCGTGGCTGGCAAAGAATAATGTTCCAAAATCGGTCCATCTCCCAAG
GGGTCCAATTTTTCTTCCTGGGTGTCAGCGAGCCCTGACTCACTACAGTGCAGCTGACAGGGG
CTGTCATGCAACTGGCCCCTAAGCCAAAGCAAAAGACCTAAGGACGACCTTTGAACAATACAA
AGG**ATG**GGTTTCAATGTAATTAGGCTACTGAGCGGATCAGCTGTAGCACTGGTTATAGCCCCC
ACTGTCTTACTGACAATGCTTTCTTCTGCCGAACGAGGATGCCCTAAGGGCTGTAGGTGTGAA
GGCAAAATGGTATATTGTGAATCTCAGAAATTACAGGAGATACCCTCAAGTATATCTGCTGGT
TGCTTAGGTTTGTCCCTTCGCTATAACAGCCTTCAAAAACTTAAGTATAATCAATTTAAAGGG
CTCAACCAGCTCACCTGGCTATACCTTGACCATAACCATATCAGCAATATTGACGAAAATGCT
TTTAATGGAATACGCAGACTCAAAGAGCTGATTCTTAGTTCCAATAGAATCTCCTATTTTCTT
AACAATACCTTCAGACCTGTGACAAATTTACGGAACTTGGATCTGTCCTATAATCAGCTGCAT
TCTCTGGGATCTGAACAGTTTCGGGGCTTGCGGAAGCTGCTGAGTTTACATTTACGGTCTAAC
TCCCTGAGAACCATCCCTGTGCGAATATTCCAAGACTGCCGCAACCTGGAACTTTTGGACCTG
GGATATAACCGGATCCGAAGTTTAGCCAGGAATGTCTTTGCTGGCATGATCAGACTCAAAGAA
CTTCACCTGGAGCACAATCAATTTTCCAAGCTCAACCTGGCCCTTTTTCCAAGGTTGGTCAGC
CTTCAGAACCTTTACTTGCAGTGGAATAAAATCAGTGTCATAGGACAGACCATGTCCTGGACC
TGGAGCTCCTTACAAAGGCTTGATTTATCAGGCAATGAGATCGAAGCTTTCAGTGGACCCAGT
GTTTTCCAGTGTGTCCCGAATCTGCAGCGCCTCAACCTGGATTCCAACAAGCTCACATTTATT
GGTCAAGAGATTTTGGATTCTTGGATATCCCTCAATGACATCAGTCTTGCTGGGAATATATGG
GAATGCAGCAGAAATATTTGCTCCCTTGTAAACTGGCTGAAAAGTTTTAAAGGTCTAAGGGAG
AATACAATTATCTGTGCCAGTCCCAAAGAGCTGCAAGGAGTAAATGTGATCGATGCAGTGAAG
AACTACAGCATCTGTGGCAAAAGTACTACAGAGAGGTTTGATCTGGCCAGGGCTCTCCCAAAG
CCGACGTTTAAGCCCAAGCTCCCCAGGCCGAAGCATGAGAGCAAACCCCCTTTGCCCCCGACG
GTGGGAGCCACAGAGCCCGGCCCAGAGACCGATGCTGACGCCGAGCACATCTCTTTCCATAAA
ATCATCGCGGGCAGCGTGGCGCTTTTCCTGTCCGTGCTCGTCATCCTGCTGGTTATCTACGTG
TCATGGAAGCGGTACCCTGCGAGCATGAAGCAGCTGCAGCAGCGCTCCCTCATGCGAAGGCAC
AGGAAAAAGAAAAGACAGTCCCTAAAGCAAATGACTCCCAGCACCCAGGAATTTTATGTAGAT
TATAAACCCACCAACACGGAGACCAGCGAGATGCTGCTGAATGGGACGGGACCCTGCACCTAT
AACAAATCGGGCTCCAGGGAGTGTGAGGTA**TGA**ACCATTGTGATAAAAAGAGCTCTTAAAAGC
TGGGAAATAAGTGGTGCTTTATTGAACTCTGGTGACTATCAAGGGAACGCGATGCCCCCCCTC
CCCTTCCCTCTCCCTCTCACTTTGGTGGCAAGATCCTTCCTTGTCCGTTTTAGTGCATTCATA
ATACTGGTCATTTTCCTCTCATACATAATCAACCCATTGAAATTTAAATACCACAATCAATGT
GAAGCTTGAACTCCGGTTTAATATAATACCTATTGTATAAGACCCTTTACTGATTCCATTAAT
GTCGCATTTGTTTTAAGATAAAACTTCTTTCATAGGTAAAAAAAAAAA

# FIGURE 536

MGFNVIRLLSGSAVALVIAPTVLLTMLSSAERGCPKGCRCEGKMVYCESQKLQEIPSSISAGC
LGLSLRYNSLQKLKYNQFKGLNQLTWLYLDHNHISNIDENAFNGIRRLKELILSSNRISYFLN
NTFRPVTNLRNLDLSYNQLHSLGSEQFRGLRKLLSLHLRSNSLRTIPVRIFQDCRNLELLDLG
YNRIRSLARNVFAGMIRLKELHLEHNQFSKLNLALFPRLVSLQNLYLQWNKISVIGQTMSWTW
SSLQRLDLSGNEIEAFSGPSVFQCVPNLQRLNLDSNKLTFIGQEILDSWISLNDISLAGNIWE
CSRNICSLVNWLKSFKGLRENTIICASPKELQGVNVIDAVKNYSICGKSTTERFDLARALPKP
TFKPKLPRPKHESKPPLPPTVGATEPGPETDADAEHISFHKIIAGSVALFLSVLVILLVIYVS
WKRYPASMKQLQQRSLMRRHRKKKRQSLKQMTPSTQEFYVDYKPTNTETSEMLLNGTGPCTYN
KSGSRECEV

**Important features:**

**Signal peptide:**

amino acids 1-33

**Transmembrane domain:**

amino acids 420-442

**N-glycosylation sites.**

amino acids 126-129, 357-360, 496-499, 504-507

**cAMP- and cGMP-dependent protein kinase phosphorylation site.**

amino acids 465-468

**Tyrosine kinase phosphorylation site.**

amino acids 136-142

**N-myristoylation sites.**

amino acids 11-16, 33-38, 245-250, 332-337, 497-502, 507-512

# FIGURE 537

GGGACTACAAGCCGCGCCGCGCTGCCGCTGGCCCCTCAGCAACCCTCGAC**ATG**GCGCTGAGGCGGCCACCGCGAC
TCCGGCTCTGCGCTCGGCTGCCTGACTTCTTCCTGCTGCTGCTTTTCAGGGGCTGCCTGATAGGGGCTGTAAATC
TCAAATCCAGCAATCGAACCCCAGTGGTACAGGAATTTGAAAGTGTGGAACTGTCTTGCATCATTACGGATTCGC
AGACAAGTGACCCCAGGATCGAGTGGAAGAAAATTCAAGATGAACAAACCACATATGTGTTTTTTGACAACAAAA
TTCAGGGAGACTTGGCGGGTCGTGCAGAAATACTGGGAAGACATCCCTGAAGATCTGGAATGTGACACGGAGAG
ACTCAGCCCTTTATCGCTGTGAGGTCGTTGCTCGAAATGACCGCAAGGAAATTGATGAGATTGTGATCGAGTTAA
CTGTGCAAGTGAAGCCAGTGACCCCTGTCTGTAGAGTGCCGAAGGCTGTACCAGTAGGCAAGATGGCAACACTGC
ACTGCCAGGAGAGTGAGGGCCACCCCCGGCCTCACTACAGCTGGTATCGCAATGATGTACCACTGCCCACGGATT
CCAGAGCCAATCCCAGATTTCGCAATTCTTCTTTCCACTTAAACTCTGAAACAGGCACTTTGGTGTTCACTGCTG
TTCACAAGGACGACTCTGGGCAGTACTACTGCATTGCTTCCAATGACGCAGGCTCAGCCAGGTGTGAGGAGCAGG
AGATGGAAGTCTATGACCTGAACATTGGCGGAATTATTGGGGGGGTTCTGGTTGTCCTTGCTGTACTGGCCCTGA
TCACGTTGGGCATCTGCTGTGCATACAGACGTGGCTACTTCATCAACAATAAACAGGATGGAGAAAGTTACAAGA
ACCCAGGGAAACCAGATGGAGTTAACTACATCCGCACTGACGAGGAGGGCGACTTCAGACACAAGTCATCGTTTG
TGATC**TGA**GACCCGCGGTGTGGCTGAGAGCGCACAGAGCGCACGTGCACATACCTCTGCTAGAAACTCCTGTCAA
GGCAGCGAGAGCTGATGCACTCGGACAGAGCTAGACACTCATTCAGAAGCTTTTCGTTTTGGCCAAAGTTGACCA
CTACTCTTCTTACTCTAACAAGCCACATGAATAGAAGAATTTTCCTCAAGATGGACCCGGTAAATATAACCACAA
GGAAGCGAAACTGGGTGCGTTCACTGAGTTGGGTTCCTAATCTGTTTCTGGCCTGATTCCCGCATGAGTATTAGG
GTGATCTTAAAGAGTTTGCTCACGTAAACGCCCGTGCTGGGCCCTGTGAAGCCAGCATGTTCACCACTGGTCGTT
CAGCAGCCACGACAGCACCATGTGAGATGGCGAGGTGGCTGGACAGCACCAGCAGCGCATCCCGGCGGGAACCCA
GAAAAGGCTTCTTACACAGCAGCCTTACTTCATCGGCCCACAGACACCACCGCAGTTTCTTCTTAAAGGCTCTGC
TGATCGGTGTTGCAGTGTCCATTGTGGAGAAGCTTTTTGGATCAGCATTTTGTAAAAACAACCAAAATCAGGAAG
GTAAATTGGTTGCTGGAAGAGGGATCTTGCCTGAGGAACCCTGCTTGTCCAACAGGGTGTCAGGATTTAAGGAAA
ACCTTCGTCTTAGGCTAAGTCTGAAATGGTACTGAAATATGCTTTTCTATGGGTCTTGTTTATTTTATAAAATTT
TACATCTAAATTTTTGCTAAGGATGTATTTTGATTATTGAAAAGAAAATTTCTATTTAAACTGTAAATATATTGT
CATACAATGTTAAATAACCTATTTTTTTAAAAAAGTTCAACTTAAGGTAGAAGTTCCAAGCTACTAGTGTTAAAT
TGGAAAATATCAATAATTAAGAGTATTTTACCCAAGGAATCCTCTCATGGAAGTTTACTGTGATGTTCCTTTTCT
CACACAAGTTTTAGCCTTTTTCACAAGGGAACTCATACTGTCTACACATCAGACCATAGTTGCTTAGGAAACCTT
TAAAAATTCCAGTTAAGCAATGTTGAAATCAGTTTGCATCTCTTCAAAAGAAACCTCTCAGGTTAGCTTTGAACT
GCCTCTTCCTGAGATGACTAGGACAGTCTGTACCCAGAGGCCACCCAGAAGCCCTCAGATGTACATACACAGATG
CCAGTCAGCTCCTGGGGTTGCGCCAGGCGCCCCCGCTCTAGCTCACTGTTGCCTCGCTGTCTGCCAGGAGGCCCT
GCCATCCTTGGGCCCTGGCAGTGGCTGTGTCCCAGTGAGCTTTACTCACGTGGCCCTTGCTTCATCCAGCACAGC
TCTCAGGTGGGCACTGCAGGGACACTGGTGTCTTCCATGTAGCGTCCCAGCTTTGGGCTCCTGTAACAGACCTCT
TTTTGGTTATGGATGGCTCACAAAATAGGGCCCCCAATGCTATTTTTTTTTTTTAAGTTTGTTTAATTATTTGTT
AAGATTGTCTAAGGCCAAAGGCAATTGCGAAATCAAGTCTGTCAAGTACAATAACATTTTTAAAAGAAAATGGAT
CCCACTGTTCCTCTTTGCCACAGAGAAAGCACCCAGACGCCACAGGCTCTGTCGCATTTCAAAACAAACCATGAT
GGAGTGGCGGCCAGTCCAGCCTTTTAAAGAACGTCAGGTGGAGCAGCCAGGTGAAAGGCCTGGCGGGGAGGAAAG
TGAAACGCCTGAATCAAAAGCAGTTTTCTAATTTTGACTTTAAATTTTTCATCCGCCGGAGACACTGCTCCCATT
TGTGGGGGGACATTAGCAACATCACTCAGAAGCCTGTGTTCTTCAAGAGCAGGTGTTCTCAGCCTCACATGCCCT
GCCGTGCTGGACTCAGGACTGAAGTGCTGTAAAGCAAGGAGCTGCTGAGAAGGAGCACTCCACTGTGTGCCTGGA
GAATGGCTCTCACTACTCACCTTGTCTTTCAGCTTCCAGTGTCTTGGGTTTTTTATACTTTGACAGCTTTTTTTT
AATTGCATACATGAGACTGTGTTGACTTTTTTTAGTTATGTGAAACACTTTGCCGCAGGCCGCCTGGCAGAGGCA
GGAAATGCTCCAGCAGTGGCTCAGTGCTCCCTGGTGTCTGCTGCATGGCATCCTGGATGCTTAGCATGCAAGTTC
CCTCCATCATTGCCACCTTGGTAGAGAGGGATGGCTCCCCACCCTCAGCGTTGGGGATTCACGCTCCAGCCTCCT
TCTTGGTTGTCATAGTGATAGGGTAGCCTTATTGCCCCCTCTTCTTATACCCTAAAACCTTCTACACTAGTGCCA
TGGGAACCAGGTCTGAAAAAGTAGAGAGAAGTGAAAGTAGAGTCTGGGAAGTAGCTGCCTATAACTGAGACTAGA
CGGAAAAGGAATACTCGTGTATTTTAAGATATGAATGTGACTCAAGACTCGAGGCCGATACGAGGCTGTGATTCT
GCCTTTGGATGGATGTTGCTGTACACAGATGCTACAGACTTGTACTAACACACCGTAATTTGGCATTTGTTTAAC
CTCATTTATAAAAGCTTCAAAAAAACCCA

# FIGURE 538

MALRRPPRLRLCARLPDFFLLLLFRGCLIGAVNLKSSNRTPVVQEFESVELSCIITDSQTSDP
RIEWKKIQDEQTTYVFFDNKIQGDLAGRAEILGKTSLKIWNVTRRDSALYRCEVVARNDRKEI
DEIVIELTVQVKPVTPVCRVPKAVPVGKMATLHCQESEGHPRPHYSWYRNDVPLPTDSRANPR
FRNSSFHLNSETGTLVFTAVHKDDSGQYYCIASNDAGSARCEEQEMEVYDLNIGGIIGGVLVV
LAVLALITLGICCAYRRGYFINNKQDGESYKNPGKPDGVNYIRTDEEGDFRHKSSFVI

**Important features:**

**Signal peptide:**

amino acids 1-30

**Transmembrane domain:**

amino acids 243-263

**N-glycosylation sites.**

amino acids 104-107, 192-195

**cAMP- and cGMP-dependent protein kinase phosphorylation site.**

amino acids 107-110

**Casein kinase II phosphorylation site.**

amino acids 106-109, 296-299

**Tyrosine kinase phosphorylation site.**

amino acids 69-77

**N-myristoylation sites.**

amino acids 26-31, 215-220, 226-231, 243-248, 244-249, 262-267

# FIGURE 539

CCAGGACCAGGGCGCACCGGCTCAGCCTCTCACTTGTCAGAGGCCGGGGAAGAGAAGCAAAGC
GCAACGGTGTGGTCCAAGCCGGGGCTTCTGCTTCGCCTCTAGGACATACACGGGACCCCCTAA
CTTCAGTCCCCCAAACGCGCACCCTCGAAGTCTTGAACTCCAGCCCCGCACATCCACGCGCGG
CACAGGCGCGGCAGGCGGCAGGTCCCGGCCGAAGGCGATGCGCGCAGGGGGTCGGGCAGCTGG
GCTCGGGCGGCGGGAGTAGGGCCCGGCAGGGAGGCAGGGAGGCTGCATATTCAGAGTCGCGGG
CTGCGCCCTGGGCAGAGGCCGCCCTCGCTCCACGCAACACCTGCTGCTGCCACCGCGCCGCG**A**
**TG**AGCCGCGTGGTCTCGCTGCTGCTGGGCGCCGCGCTGCTCTGCGGCCACGGAGCCTTCTGCC
GCCGCGTGGTCAGCGGCCAAAAGGTGTGTTTTGCTGACTTCAAGCATCCCTGCTACAAAATGG
CCTACTTCCATGAACTGTCCAGCCGAGTGAGCTTTCAGGAGGCACGCCTGGCTTGTGAGAGTG
AGGGAGGAGTCCTCCTCAGCCTTGAGAATGAAGCAGAACAGAAGTTAATAGAGAGCATGTTGC
AAAACCTGACAAAACCCGGGACAGGGATTTCTGATGGTGATTTCTGGATAGGGCTTTGGAGGA
ATGGAGATGGGCAAACATCTGGTGCCTGCCCAGATCTCTACCAGTGGTCTGATGGAAGCAATT
CCCAGTACCGAAACTGGTACACAGATGAACCTTCCTGCGGAAGTGAAAAGTGTGTTGTGATGT
ATCACCAACCAACTGCCAATCCTGGCCTTGGGGGTCCCTACCTTTACCAGTGGAATGATGACA
GGTGTAACATGAAGCACAATTATATTTGCAAGTATGAACCAGAGATTAATCCAACAGCCCCTG
TAGAAAAGCCTTATCTTACAAATCAACCAGGAGACACCCATCAGAATGTGGTTGTTACTGAAG
CAGGTATAATTCCCAATCTAATTTATGTTGTTATACCAACAATACCCCTGCTCTTACTGATAC
TGGTTGCTTTTGGAACCTGTTGTTTCCAGATGCTGCATAAAAGTAAAGGAAGAACAAAAACTA
GTCCAAACCAGTCTACACTGTGGATTTCAAAGAGTACCAGAAAAGAAAGTGGCATGGAAGTA**T**
**AA**TAACTCATTGACTTGGTTCCAGAATTTTGTAATTCTGGATCTGTATAAGGAATGGCATCAG
AACAATAGCTTGGAATGGCTTGAAATCACAAAGGATCTGCAAGATGAACTGTAAGCTCCCCCT
TGAGGCAAATATTAAAGTAATTTTTATATGTCTATTATTTCATTTAAAGAATATGCTGTGCTA
ATAATGGAGTGAGACATGCTTATTTTGCTAAAGGATGCACCCAAACTTCAAACTTCAAGCAAA
TGAAATGGACAATGCAGATAAAGTTGTTATCAACACGTCGGGAGTATGTGTGTTAGAAGCAAT
TCCTTTTATTTCTTTCACCTTTCATAAGTTGTTATCTAGTCAATGTAATGTATATTGTATTGA
AATTTACAGTGTGCAAAAGTATTTTACCTTTGCATAAGTGTTTGATAAAAATGAACTGTTCTA
ATATTTATTTTTATGGCATCTCATTTTTCAATACATGCTCTTTTGATTAAAGAAACTTATTAC
TGTTGTCAACTGAATTCACACACACACAAATATAGTACCATAGAAAAAGTTTGTTTTCTCGAA
ATAATTCATCTTTCAGCTTCTCTGCTTTTGGTCAATGTCTAGGAAATCTCTTCAGAAATAAGA
AGCTATTTCATTAAGTGTGATATAAACCTCCTCAAACATTTTACTTAGAGGCAAGGATTGTCT
AATTTCAATTGTGCAAGACATGTGCCTTATAATTATTTTTAGCTTAAAATTAAACAGATTTTG
TAATAATGTAACTTTGTTAATAGGTGCATAAACACTAATGCAGTCAATTTGAACAAAAGAAGT
GACATACACAATATAAATCATATGTCTTCACACGTTGCCTATATAATGAGAAGCAGCTCTCTG
AGGGTTCTGAAATCAATGTGGTCCCTCTCTTGCCCACTAAACAAAGATGGTTGTTCGGGGTTT
GGGATTGACACTGGAGGCAGATAGTTGCAAAGTTAGTCTAAGGTTTCCCTAGCTGTATTTAGC
CTCTGACTATATTAGTATACAAAGAGGTCATGTGGTTGAGACCAGGTGAATAGTCACTATCAG
TGTGGAGACAAGCACAGCACACAGACATTTTAGGAAGGAAAGGAACTACGAAATCGTGTGAAA
ATGGGTTGGAACCCATCAGTGATCGCATATTCATTGATGAGGGTTTGCTTGAGATAGAAAATG
GTGGCTCCTTTCTGTCTTATCTCCTAGTTTCTTCAATGCTTACGCCTTGTTCTTCTCAAGAGA
AAGTTGTAACTCTCTGGTCTTCATATGTCCCTGTGCTCCTTTTAACCAAATAAAGAGTTCTTG
TTTCTGGGGGAAAAAAAAAAAAAAAAAAAAAAAAAAAAAAAAAAAAAAAAA

# FIGURE 540

MSRVVSLLLGAALLCGHGAFCRRVVSGQKVCFADFKHPCYKMAYFHELSSRVSFQEARLACES
EGGVLLSLENEAEQKLIESMLQNLTKPGTGISDGDFWIGLWRNGDGQTSGACPDLYQWSDGSN
SQYRNWYTDEPSCGSEKCVVMYHQPTANPGLGGPYLYQWNDDRCNMKHNYICKYEPEINPTAP
VEKPYLTNQPGDTHQNVVVTEAGIIPNLIYVVIPTIPLLLLILVAFGTCCFQMLHKSKGRTKT
SPNQSTLWISKSTRKESGMEV

**Important features:**

**Signal peptide:**

amino acids 1-21

**Transmembrane domain:**

amino acids 214-235

**N-glycosylation sites.**

amino acids 86-89 and 255-258

**cAMP- and cGMP-dependent protein kinase phosphorylation site.**

amino acids 266-269

**N-myristoylation sites.**

amino acids 27-32, 66-71, 91-96, 93-98, 102-107, 109-114, 140-145 and 212-217

# FIGURE 541

GGAGA**ATG**GAGAGAGCAGTGAGAGTGGAGTCCGGGGTCCTGGTCGGGGTGGTCTGTCTGCTCCTGGCATGCCCTG
CCACAGCCACTGGGCCCGAAGTTGCTCAGCCTGAAGTAGACACCACCCTGGGTCGTGTGCGAGGCCGGCAGGTGG
GCGTGAAGGGCACAGACCGCCTTGTGAATGTCTTTCTGGGCATTCCATTTGCCCAGCCGCCACTGGGCCCTGACC
GGTTCTCAGCCCCACACCCAGCACAGCCCTGGGAGGGTGTGCGGGATGCCAGCACTGCGCCCCCAATGTGCCTAC
AAGACGTGGAGAGCATGAACAGCAGCAGATTTGTCCTCAACGGAAAACAGCAGATCTTCTCCGTTTCAGAGGACT
GCCTGGTCCTCAACGTCTATAGCCCAGCTGAGGTCCCCGCAGGGTCCGGTAGGCCGGTCATGGTATGGGTCCATG
GAGGCGCTCTGATAACTGGCGCTGCCACCTCCTACGATGGATCAGCTCTGGCTGCCTATGGGGATGTGGTCGTGG
TTACAGTCCAGTACCGCCTTGGGGTCCTTGGCTTCTTCAGCACTGGAGATGAGCATGCACCTGGCAACCAGGGCT
TCCTAGATGTGGTAGCTGCTTTGCGCTGGGTGCAAGAAAACATCGCCCCCTTCGGGGGTGACCTCAACTGTGTCA
CTGTCTTTGGTGGATCTGCCGGTGGGAGCATCATCTCTGGCCTGGTCCTGTCCCCAGTGGCTGCAGGGCTGTTCC
ACAGAGCCATCACACAGAGTGGGGTCATCACCACCCCAGGGATCATCGACTCTCACCCTTGGCCCCTAGCTCAGA
AAATCGCAAACACCTTGGCCTGCAGCTCCAGCTCCCCGGCTGAGATGGTGCAGTGCCTTCAGCAGAAAGAAGGAG
AAGAGCTGGTCCTTAGCAAGAAGCTGAAAAATACTATCTATCCTCTCACCGTTGATGGCACTGTCTTCCCCAAAA
GCCCCAAGGAACTCCTGAAGGAGAAGCCCTTCCACTCTGTGCCCTTCCTCATGGGTGTCAACAACCATGAGTTCA
GCTGGCTCATCCCCAGGGGCTGGGGTCTCCTGGATACAATGGAGCAGATGAGCCGGGAGGACATGCTGGCCATCT
CAACACCCGTCTTGACCAGTCTGGATGTGCCCCCTGAGATGATGCCCACCGTCATAGATGAATACCTAGGAAGCA
ACTCGGACGCACAAGCCAAATGCCAGGCGTTCCAGGAATTCATGGGTGACGTATTCATCAATGTTCCCACCGTCA
GTTTTTCAAGATACCTTCGAGATTCTGGAAGCCCTGTCTTTTTCTATGAGTTCCAGCATCGACCCAGTTCTTTTG
CGAAGATCAAACCTGCCTGGGTGAAGGCTGATCATGGGGCCGAGGGTGCTTTTGTGTTCGGAGGTCCCTTCCTCA
TGGACGAGAGCTCCCGCCTGGCCTTTCCAGAGGCCACAGAGGAGGAGAAGCAGCTAAGCCTCACCATGATGGCCC
AGTGGACCCACTTTGCCCGGACAGGGGACCCCAATAGCAAGGCTCTGCCTCCTTGGCCCCAATTCAACCAGGCGG
AACAATATCTGGAGATCAACCCAGTGCCACGGGCCGGACAGAAGTTCAGGGAGGCCTGGATGCAGTTCTGGTCAG
AGACGCTCCCCAGCAAGATACAACAGTGGCACCAGAAGCAGAAGAACAGGAAGGCCCAGGAGGACCTC**TGA**GGCC
AGGCCTGAACCTTCTTGGCTGGGGCAAACCACTCTTCAAGTGGTGGCAGAGTCCCAGCACGGCAGCCCGCCTCTC
CCCCTGCTGAGACTTTAATCTCCACCAGCCCTTAAAGTGTCGGCCGCTCTGTGACTGGAGTTATGCTCTTTTGAA
ATGTCACAAGGCCGCCTCCCACCTCTGGGGCATTGTACAAGTTCTTCCCTCTCCCTGAAGTGCCTTTCCTGCTTT
CTTCGTGGTAGGTTCTAGCACATTCCTCTAGCTTCCTGGAGGACTCACTCCCCAGGAAGCCTTCCCTGCCTTCTC
TGGGCTGTGCGGCCCCGAGTCTGCGTCCATTAGAGCACAGTCCACCCGAGGCTAGCACCGTGTCTGTGTCTGTCT
CCCCCTCAGAGGAGCTCTCTCAAAATGGGGATTAGCCTAACCCCACTCTGTCACCCACACCAGGATCGGGTGGGA
CCTGGAGCTAGGGGGTGTTTGCTGAGTGAGTGAGTGAAACACAGAATATGGGAATGGCAGCTGCTGAACTTGAAC
CCAGAGCCTTCAGGTGCCAAAGCCATACTCAGGCCCCCACCGACATTGTCCACCCTGGCCAGAAGGGTGCATGCC
AATGGCAGAGACCTGGGATGGGAGAAGTCCTGGGGCGCCAGGGGATCCAGCCTAGAGCAGACCTTAGCCCCTGAC
TAAGGCCTCAGACTAGGGCGGGAGGGGTCTCCTCCTCTCTGCTGCCCAGTCCTGGCCCCTGCACAAGACAACAGA
ATCCATCAGGGCCATGAGTGTCACCCAGACCTGACCCTCACCAATTCCAGCCCCTGACCCTCAGGACGCTGGATG
CCAGCTCCCAGCCCCAGTGCCGGGTCCTCCCTCCCTTCCTGGCTTGGGGAGACCAGTTTCTGGGGAGCTTCCAAG
AGCACCCACCAAGACACAGCAGGACAGGCCAGGGGAGGGCATCTGGACCAGGGCATCCGTCGGGCTATTGTCACA
GAGAAAAGAAGAGACCCACCCACTCGGGCTGCAAAAGGTGAAAAGCACCAAGAGGTTTTCAGATGGAAGTGAGAG
GTGACAGTGTGCTGGCAGCCCTCACAGCCCTCGCTTGCTCTCCCTGCCGCCTCTGCCTGGGCTCCCACTTTGGCA
GCACTTGAGGAGCCCTTCAACCCGCCGCTGCACTGTAGGAGCCCCTTTCTGGGCTGGCCAAGGCCGGAGCCAGCT
CCCTCAGCTTGCGGGGAGGTGCGGAGGGAGAGGGGCGGGCAGGAACCGGGGCTGCGCGCAGCGCTTGCGGGCCAG
AGTGAGTTCCGGGTGGGCGTGGGCTCGGCGGGGCCCCACTCAGAGCAGCTGGCCGGCCCCAGGCAGTGAGGGCCT
TAGCACCTGGGCCAGCAGCTGCTGTGCTCGATTTCTCGCTGGGCCTTAGCTGCCTCCCCGCGGGGCAGGGCTCGG
GACCTGCAGCCCTCCATGCCTGACCCTCCCCCCACCCCCCGTGGGCTCCTGTGCGGCCGGAGCCTCCCCAAGGAG
CGCCGCCCCCTGCTCCACAGCGCCCAGTCCCATCGACCACCCAAGGGCTGAGGAGTGCGGGTGCACAGCGCGGGA
CTGGCAGGCAGCTCCACCTGCTGCCCCAGTGCTGGATCCACTGGGTGAAGCCAGCTGGGCTCCTGAGTCTGGTGG
GGACTTGGAGAACCTTTATGTCTAGCTAAGGGATTGTAAATACACCGATGGGCACTCTGTATCTAGCTCAAGGTT
TGTAAACACACCAATCAGCACCCTGTGTCTAGCTCAGTGTTTGTGAATGCACCAATCCACACTCTGTATCTGGCT
ACTCTGGTGGGGACTTGGAGAACCTTTGTGTCCACACTCTGTATCTAGCTAATCTAGTGGGGATGTGGAGAACCT
TTGTGTCTAGCTCAGGGATCGTAAACGCACCAATCAGCACCCTGTCAAAACAGACCACTTGACTCTCTGTAAAAT
GGACCAATCAGCAGGATGTGGGTGGGGCGAGACAAGAGAATAAAAGCAGGCTGCCTGAGCCAGCAGTGACAACCC
CCCTCGGGTCCCCTCCCACGCCGTGGAAGCTTTGTTCTTTCGCTCTTTGCAATAAATCTTGCTACTGCCCAAAA

# FIGURE 542

MERAVRVESGVLVGVVCLLLACPATATGPEVAQPEVDTTLGRVRGRQVGVKGTDRLVNVFLGI
PFAQPPLGPDRFSAPHPAQPWEGVRDASTAPPMCLQDVESMNSSRFVLNGKQQIFSVSEDCLV
LNVYSPAEVPAGSGRPVMVWVHGGALITGAATSYDGSALAAYGDVVVVTVQYRLGVLGFFSTG
DEHAPGNQGFLDVVAALRWVQENIAPFGGDLNCVTVFGGSAGGSIISGLVLSPVAAGLFHRAI
TQSGVITTPGIIDSHPWPLAQKIANTLACSSSSPAEMVQCLQQKEGEELVLSKKLKNTIYPLT
VDGTVFPKSPKELLKEKPFHSVPFLMGVNNHEFSWLIPRGWGLLDTMEQMSREDMLAISTPVL
TSLDVPPEMMPTVIDEYLGSNSDAQAKCQAFQEFMGDVFINVPTVSFSRYLRDSGSPVFFYEF
QHRPSSFAKIKPAWVKADHGAEGAFVFGGPFLMDESSRLAFPEATEEEKQLSLTMMAQWTHFA
RTGDPNSKALPPWPQFNQAEQYLEINPVPRAGQKFREAWMQFWSETLPSKIQQWHQKQKNRKA
QEDL

**Important features:**

**Signal peptide:**

amino acids 1-27

**Transmembrane domain:**

amino acids 226-245

**N-glycosylation site.**

amino acids 105-109

**N-myristoylation sites.**

amino acids 10-16, 49-55, 62-68, 86-92, 150-156, 155-161, 162-168, 217-223, 227-233, 228-234, 232-238, 262-268, 357-363, 461-467

**Prokaryotic membrane lipoprotein lipid attachment site.**

amino acids 12-23

**Carboxylesterases type-B serine active site.**

amino acids 216-232

# FIGURE 543

TGTCGCCTGGCCCTCGCC**ATG**CAGACCCCGCGAGCGTCCCCTCCCCGCCCGGCCCTCCTGCTTCTGCTGCTGCTA
CTGGGGGGCGCCCACGGCCTCTTTCCTGAGGAGCCGCCGCCGCTTAGCGTGGCCCCCAGGGACTACCTGAACCAC
TATCCCGTGTTTGTGGGCAGCGGGCCCGGACGCCTGACCCCCGCAGAAGGTGCTGACGACCTCAACATCCAGCGA
GTCCTGCGGGTCAACAGGACGCTGTTCATTGGGGACAGGGACAACCTCTACCGCGTAGAGCTGGAGCCCCCCACG
TCCACGGAGCTGCGGTACCAGAGGAAGCTGACCTGGAGATCTAACCCCAGCGACATAAACGTGTGTCGGATGAAG
GGCAAACAGGAGGGCGAGTGTCGAAACTTCGTAAAGGTGCTGCTCCTTCGGGACGAGTCCACGCTCTTTGTGTGC
GGTTCCAACGCCTTCAACCCGGTGTGCGCCAACTACAGCATAGACACCCTGCAGCCCGTCGGAGACAACATCAGC
GGTATGGCCCGCTGCCCGTACGACCCCAAGCACGCCAATGTTGCCCTCTTCTCTGACGGGATGCTCTTCACAGCT
ACTGTTACCGACTTCCTAGCCATTGATGCTGTCATCTACCGCAGCCTCGGGGACAGGCCCACCCTGCGCACCGTG
AAACATGACTCCAAGTGGTTCAAAGAGCCTTACTTTGTCCATGCGGTGGAGTGGGGCAGCCATGTCTACTTCTTC
TTCCGGGAGATTGCGATGGAGTTTAACTACCTGGAGAAGGTGGTGGTGTCCCGCGTGGCCCGAGTGTGCAAGAAC
GACGTGGGAGGCTCCCCCCGCGTGCTGGAGAAGCAGTGGACGTCCTTCCTGAAGGCGCGGCTCAACTGCTCTGTA
CCCGGAGACTCCCATTTCTACTTCAACGTGCTGCAGGCTGTCACGGGCGTGGTCAGCCTCGGGGGCCGGCCCGTG
GTCCTGGCCGTTTTTTCCACGCCCAGCAACAGCATCCCTGGCTCGGCTGTCTGCGCCTTTGACCTGACACAGGTG
GCAGCTGTGTTTGAAGGCCGCTTCCGAGAGCAGAAGTCCCCCGAGTCCATCTGGACGCCGGTGCCGGAGGATCAG
GTGCCTCGACCCCGGCCCGGGTGCTGCGCAGCCCCCGGGATGCAGTACAATGCCTCCAGCGCCTTGCCGGATGAC
ATCCTCAACTTTGTCAAGACCCACCCTCTGATGGACGAGGCGGTGCCCTCGCTGGGCCATGCGCCCTGGATCCTG
CGGACCCTGATGAGGCACCAGCTGACTCGAGTGGCTGTGGACGTGGGAGCCGGCCCCTGGGGCAACCAGACCGTT
GTCTTCCTGGGTTCTGAGGCGGGGACGGTCCTCAAGTTCCTCGTCCGGCCCAATGCCAGCACCTCAGGGACGTCT
GGGCTCAGTGTCTTCCTGGAGGAGTTTGAGACCTACCGGCCGGACAGGTGTGGACGGCCCGGCGGTGGCGAGACA
GGGCAGCGGCTGCTGAGCTTGGAGCTGGACGCAGCTTCGGGGGGCCTGCTGGCTGCCTTCCCCCGCTGCGTGGTC
CGAGTGCCTGTGGCTCGCTGCCAGCAGTACTCGGGGTGTATGAAGAACTGTATCGGCAGTCAGGACCCCTACTGC
GGGTGGGCCCCCGACGGCTCCTGCATCTTCCTCAGCCCGGGCACCAGAGCCGCCTTTGAGCAGGACGTGTCCGGG
GCCAGCACCTCAGGCTTAGGGGACTGCACAGGACTCCTGCGGGCCAGCCTCTCCGAGGACCGCGCGGGGCTGGTG
TCGGTGAACCTGCTGGTAACGTCGTCGGTGGCGGCCTTCGTGGTGGGAGCCGTGGTGTCCGGCTTCAGCGTGGGC
TGGTTCGTGGGCCTCCGTGAGCGGCGGGAGCTGGCCCGGCGCAAGGACAAGGAGGCCATCCTGGCGCACGGGGCG
GGCGAGGCGGTGCTGAGCGTCAGCCGCCTGGGCGAGCGCAGGGCGCAGGGTCCCGGGGGCCGGGGCGGAGGCGGT
GGCGGTGGCGCCGGGGTTCCCCCGGAGGCCCTGCTGGCGCCCCTGATGCAGAACGGCTGGGCCAAGGCCACGCTG
CTGCAGGGCGGGCCCCACGACCTGGACTCGGGGCTGCTGCCCACGCCCGAGCAGACGCCGCTGCCGCAGAAGCGC
CTGCCCACTCCGCACCCGCACCCCCACGCCCTGGCCCCCGCGCCTGGGACCACGGCCACCCCCTGCTCCCGGCC
TCCGCTTCATCCTCCCTCCTGCTGCTGGCGCCCGCCCGGGCCCCCGAGCAGCCCCCCGCGCCTGGGGAGCCGACC
CCCGACGGCCGCCTCTATGCTGCCCGGCCCGGCCGCGCCTCCCACGGCGACTTCCCGCTCACCCCCCACGCCAGC
CCGGACCGCCGGCGGGTGGTGTCCGCGCCCACGGGCCCCTTGGACCCAGCCTCAGCCGCCGATGGCCTCCCGCGG
CCCTGGAGCCCGCCCCCGACGGGCAGCCTGAGGAGGCCACTGGGCCCCCACGCCCCTCCGGCCGCCACCCTGCGC
CGCACCCACACGTTCAACAGCGGCGAGGCCCGGCCTGGGGACCGCCACCGCGGCTGCCACGCCCGGCCGGGCACA
GACTTGGCCCACCTCCTCCCCTATGGGGGGGCGGACAGGACTGCGCCCCCCGTGCCC**TAG**GCCGGGGGCCCCCCG
ATGCCTTGGCAGTGCCAGCCACGGGAACCAGGAGCGAGAGACGGTGCCAGAACGCCGGGGCCCGGGGCAACTCCG
AGTGGGTGCTCAAGTCCCCCCCGCGACCCACCCGCGGAGTGGGGGGCCCCCTCCGCCACAAGGAAGCACAACCAG
CTCGCCCTCCCCCTACCCGGGGCCGCAGGACGCTGAGACGGTTTGGGGGTGGGTGGGCGGGAGGACTTTGCTATG
GATTTGAGGTTGACCTTATGCGCGTAGGTTTTGGTTTTTTTTTGCAGTTTTGGTTTCTTTTGCGGTTTTCTAACC
AATTGCACAACTCCGTTCTCGGGGTGGCGGCAGGCAGGGGAGGCTTGGACGCCGGTGGGAATGGGGGGCCACAG
CTGCAGACCTAAGCCCTCCCCCACCCCTGGAAAGGTCCCTCCCCAACCCAGGCCCCTGGCGTGTGTGGGTGTGCG
TGCGTGTGCGTGCCGTGTTCGTGTGCAAGGGGCCGGGGAGGTGGGCGTGTGTGTGCGTGCCAGCGAAGGCTGCTG
TGGGCGTGTGTGTCAAGTGGGCCACGCGTGCAGGGTGTGTGTCCACGAGCGACGATCGTGGTGGCCCCAGCGGCC
TGGGCGTTGGCTGAGCCGACGCTGGGGCTTCCAGAAGGCCCGGGGGTCTCCGAGGTGCCGGTTAGGAGTTTGAAC
CCCCCCCACTCTGCAGAGGGAAGCGGGGACAATGCCGGGGTTTCAGGCAGGAGACACGAGGAGGGCCTGCCCGGA
AGTCACATCGGCAGCAGCTGTCTAAAGGGCTTGGGGGCCTGGGGGGCGGCGAAGGTGGGTGGGGCCCCTCTGTAA
ATACGGCCCCAGGGTGGTGAGAGAGTCCCATGCCACCCGTCCCCTTGTGACCTCCCCCCTATGACCTCCAGCTGA
CCATGCATGCCACGTGGCTGGCTGGGTCCTCTGCCCTCTTTGGAGTTTGCCTCCCCCAGCCCCCTCCCCATCAAT
AAAACTCTGTTTACAACCAAAAAAAAAAAAAAAAAAAAAAAAAAAA

# FIGURE 544

MQTPRASPPRPALLLLLLLLLGGAHGLFPEEPPPLSVAPRDYLNHYPVFVGSGPGRLTPAEGAD
DLNIQRVLRVNRTLFIGDRDNLYRVELEPPTSTELRYQRKLTWRSNPSDINVCRMKGKQEGEC
RNFVKVLLLRDESTLFVCGSNAFNPVCANYSIDTLQPVGDNISGMARCPYDPKHANVALFSDG
MLFTATVTDFLAIDAVIYRSLGDRPTLRTVKHDSKWFKEPYFVHAVEWGSHVYFFFREIAMEF
NYLEKVVVSRVARVCKNDVGGSPRVLEKQWTSFLKARLNCSVPGDSHFYFNVLQAVTGVVSLG
GRPVVLAVFSTPSNSIPGSAVCAFDLTQVAAVFEGRFREQKSPESIWTPVPEDQVPRPRPGCC
AAPGMQYNASSALPDDILNFVKTHPLMDEAVPSLGHAPWILRTLMRHQLTRVAVDVGAGPWGN
QTVVFLGSEAGTVLKFLVRPNASTSGTSGLSVFLEEFETYRPDRCGRPGGGETGQRLLSLELD
AASGGLLAAFPRCVVRVPVARCQQYSGCMKNCIGSQDPYCGWAPDGSCIFLSPGTRAAFEQDV
SGASTSGLGDCTGLLRASLSEDRAGLVSVNLLVTSSVAAFVVGAVVSGFSVGWFVGLRERREL
ARRKDKEAILAHGAGEAVLSVSRLGERRAQGPGGRGGGGGGGAGVPPEALLAPLMQNGWAKAT
LLQGGPHDLDSGLLPTPEQTPLPQKRLPTPHPHPHALGPRAWDHGHPLLPASASSSLLLLAPA
RAPEQPPAPGEPTPDGRLYAARPGRASHGDFPLTPHASPDRRRVVSAPTGPLDPASAADGLPR
PWSPPPTGSLRRPLGPHAPPAATLRRTHTFNSGEARPGDRHRGCHARPGTDLAHLLPYGGADR
TAPPVP

**Important features:**

**Signal peptide:**

amino acids 1-25

**Transmembrane domains:**

amino acids 318-339, 598-617

**N-glycosylation sites.**

amino acids 74-78, 155-159, 167-171, 291-295, 386-390, 441-445, 462-466

**Glycosaminoglycan attachment sites.**

amino acids 51-55, 573-577

**cAMP- and cGMP-dependent protein kinase phosphorylation site.**

amino acids 102-106

**N-myristoylation sites.**

amino acids 21-27, 50-56, 189-195, 333-339, 382-388, 448-454, 490-496, 491-497, 508-514, 509-515, 531-537, 558-564, 569-575, 574-580, 580-586, 610-616, 643-649, 663-669, 666-672, 667-673, 668-674, 669-675, 670-676, 868-874, 879-885

# FIGURE 545

GATGGCGCAGCCACAGCTTCTGTGAGATTCGATTTCTCCCCAGTTCCCCTGTGGGTCTGAGGG
GACCAGAAGGGTGAGCTACGTTGGCTTTCTGGAAGGGGAGGCTAT**ATG**CGTCAATTCCCCAAA
ACAAGTTTTGACATTTCCCCTGAAATGTCATTCTCTATCTATTCACTGCAAGTGCCTGCTGTT
CCAGGCCTTACCTGCTGGGCACTAACGGCGGAGCCAGGATGGGGACAGAATAAAGGAGCCACG
ACCTGTGCCACCAACTCGCACTCAGACTCTGAACTCAGACCTGAAATCTTCTCTTCACGGGAG
GCTTGGCAGTTTTTCTTACTCCTGTGGTCTCCAGATTTCAGGCCTAAGATGAAAGCCTCTAGT
CTTGCCTTCAGCCTTCTCTCTGCTGCGTTTTATCTCCTATGGACTCCTTCCACTGGACTGAAG
ACACTCAATTTGGGAAGCTGTGTGATCGCCACAAACCTTCAGGAAATACGAAATGGATTTTCT
GAGATACGGGGCAGTGTGCAAGCCAAAGATGGAAACATTGACATCAGAATCTTAAGGAGGACT
GAGTCTTTGCAAGACACAAAGCCTGCGAATCGATGCTGCCTCCTGCGCCATTTGCTAAGACTC
TATCTGGACAGGGTATTTAAAAACTACCAGACCCCTGACCATTATACTCTCCGGAAGATCAGC
AGCCTCGCCAATTCCTTTCTTACCATCAAGAAGGACCTCCGGCTCTCTCATGCCCACATGACA
TGCCATTGTGGGGAGGAAGCAATGAAGAAATACAGCCAGATTCTGAGTCACTTTGAAAAGCTG
GAACCTCAGGCAGCAGTTGTGAAGGCTTTGGGGGAACTAGACATTCTTCTGCAATGGATGGAG
GAGACAGAA**TAG**GAGGAAAGTGATGCTGCTGCTAAGAATATTCGAGGTCAAGAGCTCCAGTCT
TCAATACCTGCAGAGGAGGCATGACCCCAAACCACCATCTCTTTACTGTACTAGTCTTGTGCT
GGTCACAGTGTATCTTATTTATGCATTACTTGCTTCCTTGCATGATTGTCTTTATGCATCCCC
AATCTTAATTGAGACCATACTTGTATAAGATTTTTGTAATATCTTTCTGCTATTGGATATATT
TATTAGTTAATATATTTATTTATTTTTTGCTATTTAATGTATTTATTTTTTTACTTGGACATG
AAACTTTAAAAAAATTCACAGATTATATTTATAACCTGACTAGAGCAGGTGATGTATTTTTAT
ACAGTAAAAAAAAAAAAACCTTGTAAATTCTAGAAGAGTGGCTAGGGGGGTTATTCATTTGTAT
TCAACTAAGGACATATTTACTCATGCTGATGCTCTGTGAGATATTTGAAATTGAACCAATGAC
TACTTAGGATGGGTTGTGGAATAAGTTTTGATGTGGAATTGCACATCTACCTTACAATTACTG
ACCATCCCCAGTAGACTCCCCAGTCCCATAATTGTGTATCTTCCAGCCAGGAATCCTACACGG
CCAGCATGTATTTCTACAAATAAAGTTTTCTTTGCATACCAAAAAAAAAAAAAAAAAAA

# FIGURE 546

MRQFPKTSFDISPEMSFSIYSLQVPAVPGLTCWALTAEPGWGQNKGATTCATNSHSDSELRPE
IFSSREAWQFFLLLWSPDFRPKMKASSLAFSLLSAAFYLLWTPSTGLKTLNLGSCVIATNLQE
IRNGFSEIRGSVQAKDGNIDIRILRRTESLQDTKPANRCCLLRHLLRLYLDRVFKNYQTPDHY
TLRKISSLANSFLTIKKDLRLSHAHMTCHCGEEAMKKYSQILSHFEKLEPQAAVVKALGELDI
LLQWMEETE

**Important features:**

**Signal peptide:**

amino acids 1-42

**cAMP- and cGMP-dependent protein kinase phosphorylation sites.**

amino acids 192-195, 225-228

**N-myristoylation sites.**

amino acids 42-47, 46-51, 136-141

# FIGURE 547

AGCAACTCAAGTTCATCATTGTCCTGAGAGAGAGGAGCAGCGCGGTTCTCGGCCGGGACAGCA
GAACGCCAGGGGACCCTCACCTGGGCGCGCCGGGGCACGGGCTTTGATTGTCCTGGGGTCGCG
GAGACCCGCGCGCCTGCCCTGCACGCCGGGCGGCAACCTTTGCAGTCGCGTTGGCTGCTGCGA
TCGGCCGGCGGGTCCCTGCCGAAGGCTCGGCTGCTTCTGTCCACCTCTTACACTTCTTCATTT
ATCGGTGGATCATTTCGAGAGTCCGTCTTGTAA**ATG**TTTGGCACTTTGCTACTTTATTGCTTC
TTTCTGGCGACAGTTCCAGCACTCGCCGAGACCGGCGGAGAAAGGCAGCTGAGCCCGGAGAAG
AGCGAAATATGGGGACCCGGGCTAAAAGCAGACGTCGTCCTTCCCGCCCGCTATTTCTATATT
CAGGCAGTGGATACATCAGGGAATAAATTCACATCTTCTCCAGGCGAAAAGGTCTTCCAGGTG
AAAGTCTCAGCACCAGAGGAGCAATTCACTAGAGTTGGAGTCCAGGTTTTAGACCGAAAAGAT
GGGTCCTTCATAGTAAGATACAGAATGTATGCAAGCTACAAAAATCTGAAGGTGGAAATTAAA
TTCCAAGGGCAACATGTGGCCAAATCCCCATATATTTTAAAAGGGCCGGTTTACCATGAGAAC
TGTGACTGTCCTCTGCAAGATAGTGCAGCCTGGCTACGGGAGATGAACTGCCCTGAAACCATT
GCTCAGATTCAGAGAGATCTGGCACATTTCCCTGCTGTGGATCCAGAAAAGATTGCAGTAGAA
ATCCCAAAAAGATTTGGACAGAGGCAGAGCCTATGTCACTACACCTTAAAGGATAACAAGGTT
TATATCAAGACTCATGGTGAACATGTAGGTTTTAGAATTTTCATGGATGCCATACTACTTTCT
TTGACTAGAAAGGTGAAGATGCCAGATGTGGAGCTCTTTGTTAATTTGGGAGACTGGCCTTTG
GAAAAAAAGAAATCCAATTCAAACATCCATCCGATCTTTTCCTGGTGTGGCTCCACAGATTCC
AAGGATATCGTGATGCCTACGTACGATTTGACTGATTCTGTTCTGGAAACCATGGGCCGGGTA
AGTCTGGATATGATGTCCGTGCAAGCTAACACGGGTCCTCCCTGGGAAAGCAAAAATTCCACT
GCCGTCTGGAGAGGGCGAGACAGCCGCAAAGAGAGACTCGAGCTGGTTAAACTCAGTAGAAAA
CACCCAGAACTCATAGACGCTGCTTTCACCAACTTTTTCTTCTTTAAACACGATGAAAACCTG
TATGGTCCCATTGTGAAACATATTTCATTTTTTGATTTCTTCAAGCATAAGTATCAAATAAAT
ATCGATGGCACTGTAGCAGCTTATCGCCTGCCATATTTGCTAGTTGGTGACAGTGTTGTGCTG
AAGCAGGATTCCATCTACTATGAACATTTTTACAATGAGCTGCAGCCCTGGAAACACTACATT
CCAGTTAAGAGCAACCTGAGCGATCTGCTAGAAAAACTTAAATGGGCGAAAGATCACGATGAA
GAGGCCAAAAAGATAGCAAAAGCAGGACAAGAATTTGCAAGAAATAATCTCATGGGCGATGAC
ATATTCTGTTATTATTTCAAACTTTTCCAGGAATATGCCAATTTACAAGTGAGTGAGCCCCAA
ATCCGAGAGGGCATGAAAAGGGTAGAACCACAGACTGAGGACGACCTCTTCCCTTGTACTTGC
CATAGGAAAAAGACCAAAGATGAACTC**TGA**TATGCAAAATAACTTCTATTAGAATAATGGTGC
TCTGAAGACTCTTCTTAACTAAAAAGAAGAATTTTTTAAGTATTAATTCCATGGACAATATA
AAATCTGTGTGATTGTTTGCAGTATGAAGACACATTTCTACTTATGCAGTATTCTCATGACTG
TACTTTAAAGTACATTTTTAGAATTTTATAATAAAACCACCTTTATTTTAAAGGAAAAAAA

# FIGURE 548

MFGTLLLYCFFLATVPALAETGGERQLSPEKSEIWGPGLKADVVLPARYFYIQAVDTSGNKFT
SSPGEKVFQVKVSAPEEQFTRVGVQVLDRKDGSFIVRYRMYASYKNLKVEIKFQGQHVAKSPY
ILKGPVYHENCDCPLQDSAAWLREMNCPETIAQIQRDLAHFPAVDPEKIAVEIPKRFGQRQSL
CHYTLKDNKVYIKTHGEHVGFRIFMDAILLSLTRKVKMPDVELFVNLGDWPLEKKKSNSNIHP
IFSWCGSTDSKDIVMPTYDLTDSVLETMGRVSLDMMSVQANTGPPWESKNSTAVWRGRDSRKE
RLELVKLSRKHPELIDAAFTNFFFFKHDENLYGPIVKHISFFDFFKHKYQINIDGTVAAYRLP
YLLVGDSVVLKQDSIYYEHFYNELQPWKHYIPVKSNLSDLLEKLKWAKDHDEEAKKIAKAGQE
FARNNLMGDDIFCYYFKLFQEYANLQVSEPQIREGMKRVEPQTEDDLFPCTCHRKKTKDEL

**Important features:**

**Signal peptide:**

amino acids 1-17

**N-glycosylation sites.**

amino acids 302-306, 414-418

**cAMP- and cGMP-dependent protein kinase phosphorylation sites.**

amino acids 243-247, 495-499

**Tyrosine kinase phosphorylation site.**

amino acids 341-348

**N-myristoylation sites.**

amino acids 59-65, 118-124, 184-190, 258-264, 370-376, 439-445

**Endoplasmic reticulum targeting sequence.**

amino acids 499-504

# FIGURE 549

GGGTGATTGAACTAAACCTTCGCCGCACCGAGTTTGCAGTACGGCCGTCACCCGCACCGCTGC
CTGCTTGCGGTTGGAGAAATCAAGGCCCTACCGGGCCTCCGTAGTCACCTCTCTATAGTGGGC
GTGGCCGAGGCCGGGGTGACCCTGCCGGAGCCTCCGCTGCCAGCGAC**ATG**TTCAAGGTAATTC
AGAGGTCCGTGGGGCCAGCCAGCCTGAGCTTGCTCACCTTCAAAGTCTATGCAGCACCAAAAA
AGGACTCACCTCCCAAAAATTCCGTGAAGGTTGATGAGCTTTCACTCTACTCAGTTCCTGAGG
GTCAATCGAAGTATGTGGAGGAGGCAAGGAGCCAGCTTGAAGAAAGCATCTCACAGCTCCGAC
ACTATTGCGAGCCATACACAACCTGGTGTCAGGAAACGTACTCCCAAACTAAGCCCAAGATGC
AAAGTTTGGTTCAATGGGGGTTAGACAGCTATGACTATCTCCAAAATGCACCTCCTGGATTTT
TTCCGAGACTTGGTGTTATTGGTTTTGCTGGCCTTATTGGACTCCTTTTGGCTAGAGGTTCAA
AAATAAAGAAGCTAGTGTATCCGCCTGGTTTCATGGGATTAGCTGCCTCCCTCTATTATCCAC
AACAAGCCATCGTGTTTGCCCAGGTCAGTGGGGAGAGATTATATGACTGGGGTTTACGAGGAT
ATATAGTCATAGAAGATTTGTGGAAGGAGAACTTTCAAAAGCCAGGAAATGTGAAGAATTCAC
CTGGAACTAAG**TAG**AAAACTCCATGCTCTGCCATCTTAATCAGTTATAGGTAAACATTGGAAA
CTCCATAGAATAAATCAGTATTTCTACAGAAAAATGGCATAGAAGTCAGTATTGAATGTATTA
AATTGGCTTTCTTCTTCAGGAAAAACTAGACCAGACCTCTGTTATCTTCTGTGAAATCATCCT
ACAAGCAAACTAACCTGGAATCCCTTCACCTAGAGATAATGTACAAGCCTTAGAACTCCTCAT
TCTCATGTTGCTATTTATGTACCTAATTAAAACCCAAGTTTAAAAAAAAAAAAAAAAAAAAAA
AAAAAAAAAAAAAAAAA

# FIGURE 550

MFKVIQRSVGPASLSLLTFKVYAAPKKDSPPKNSVKVDELSLYSVPEGQSKYVEEARSQLEES
ISQLRHYCEPYTTWCQETYSQTKPKMQSLVQWGLDSYDYLQNAPPGFFPRLGVIGFAGLIGLL
LARGSKIKKLVYPPGFMGLAASLYYPQQAIVFAQVSGERLYDWGLRGYIVIEDLWKENFQKPG
NVKNSPGTK

**Important features:**

**Signal peptide:**

Amino acids 1-23

**Transmembrane domain:**

Amino acids 111-130

**cAMP- and cGMP-dependent protein kinase phosphorylation site:**

Amino acids 26-30

**Tyrosine kinase phosphorylation site:**

Amino acids 36-44

**N-myristoylation sites:**

Amino acids 124-130;144-150;189-195